(19) **Europäisches Patentamt / European Patent Office / Office européen des brevets**

(11) **EP 3 070 474 A2**

(12) **EUROPEAN PATENT APPLICATION**

(43) Date of publication:
**21.09.2016 Bulletin 2016/38**

(51) Int Cl.:
***G01N 33/68*** (2006.01)

(21) Application number: **15190063.6**

(22) Date of filing: **26.02.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2010 US 308861 P**

(62) Document number(s) of the earlier application(s) in accordance with Art. 76 EPC:
**11748210.9 / 2 539 712**

(71) Applicant: **Astute Medical, Inc.**
**San Diego, CA 92121 (US)**

(72) Inventors:
- **ANDERBERG, Joseph**
  **Encinitas, CA 92024 (US)**
- **GRAY, Jeff**
  **Solana Beach, CA 92075 (US)**
- **McPherson, Paul**
  **Encinitas, CA 92024 (US)**
- **NAKAMURA, Kevin**
  **Cardiff by the Sea, CA 92007 (US)**
- **KAMPF, James Patrick**
  **San Diego, CA 92130 (US)**

(74) Representative: **Schiweck, Weinzierl & Koch**
**European Patent Attorneys**
**Landsberger Straße 98**
**80339 München (DE)**

Remarks:
•Claims filed after the date of filing / after the date of receipt of the application (Rule 68(4) EPC).
•This application was filed on 16-10-2015 as a divisional application to the application mentioned under INID code 62.

(54) **METHODS AND COMPOSITIONS FOR DIAGNOSIS AND PROGNOSIS OF RENAL INJURY AND RENAL FAILURE**

(57) The present invention relates to methods and compositions for monitoring, diagnosis, prognosis, and determination of treatment regimens in subjects suffering from or suspected of having a renal injury. In particular, the invention relates to using one or more assay configured to detect a kidney injury marker as diagnostic and prognostic biomarker in renal injuries.

**EP 3 070 474 A2**

**Description**

The present application claims priority to U.S. Provisional Patent Application No. 61/308,861 filed February 26, 2010, which is hereby incorporated in its entirety including all tables, figures, and claims.

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. Renal disease and/or injury may be acute or chronic. Acute and chronic kidney disease are described as follows (from Current Medical Diagnosis & Treatment 2008, 47th Ed, McGraw Hill, New York, pages 785-815, which are hereby incorporated by reference in their entirety): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week) reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222, and which is hereby incorporated by reference in their entirety.

Table 1:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or bums), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |

(continued)

| Intrinsic Renal | |
|---|---|
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulointerstitial nephritis | Drug reaction (eg, β-lactams, NSAIDs, sulfonamides, ciprofloxacin, thiazide diuretics, furosemide, phenytoin, |
| | allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| Postrenal | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005, which, with the references listed therein, are hereby incorporated by reference in their entirety. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004, hereby incorporated by reference in its entirety) investigated both increases and decreases in serum creatinine. Patients with a mild fall in serum creatinine of- 0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo *et al., Crit Care.* 8(4):R204-12, 2004, which is hereby incorporated by reference in its entirety, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, each hereby incorporated by reference in its entirety, the RIFLE criteria provide a uniform definition of AKI which has been validated in numerous studies.

More recently, Mehta et al., Crit. Care 11:R31 (doi:10.1186.cc5713), 2007, hereby incorporated by reference in its entirety, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4):177-197*,* hereby incorporated by reference in its entirety) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of a plurality of assays, wherein one or more of the assays is configured to detect one or more of the biomarkers listed in Table 2 herein (collectively referred to herein as "kidney injury markers, and each individually as a "kidney injury marker") The plurality of assays are combined to provide a "biomarker panel approach" which can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury). Preferred biomarker panels comprise two, three, four, five, or more assays configured to detect two, three, four, five, or more of the biomarkers listed in Table 2 herein.

These kidney injury markers may be used in panels comprising a plurality of kidney injury markers, for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.,* hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to methods for evaluating renal status in a subject. These methods comprise performing an assay method that is configured to detect one or more kidney injury markers of the present invention in a body fluid sample obtained from the subject. A plurality of assay results, for example comprising a measured concentration of one or more markers selected from the group consisting of the markers listed in Table 2 herein are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, *etc.,* is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay results, for example comprising a measured concentration of one or more markers selected from the group consisting of the biomarkers listed in Table 2 herein are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay results, for example a measured concentration of one or more markers selected from the group consisting of the biomarkers listed in Table 2 herein are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay results, for example a measured concentration of one or more markers selected from the group consisting of the biomarkers listed in Table 2 herein are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

8

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, *etc.),* by selecting a concentration representing the 75th, 85th 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, *etc.* This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test or combination of tests to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma.

As previously noted, preferred biomarker panels comprise two, three, four, five, or more assays configured to detect two, three, four, five, or more of the biomarkers listed in Table 2 herein. That said, the foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score, risk scores of Thakar et al. (J. Am. Soc. Nephrol. 16: 162-68, 2005), Mehran et al. (J. Am. Coll. Cardiol. 44: 1393-99, 2004), Wijeysundera et al. (JAMA 297: 1801-9, 2007), Goldstein and Chawla (Clin. J. Am. Soc. Nephrol. 5: 943-49, 2010), or Chawla et al. (Kidney Intl. 68: 2274-80, 2005)), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (*e.g.,* a labeled antibody that binds to the second antibody, biotin, digoxigenin, maltose, oligohistidine, 2,4-dintrobenzene, phenylarsenate, ssDNA, dsDNA, *etc.).*

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, *etc.* In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

BRIEF DESCRIPTION OF THE FIGURES

Figs. 1-43 provide a detailed summary of the ability of panels of biomarkers to evaluate acute kidney injury in an ICU patient population. Patients in the population are divided based on the the RIFLE classification of renal status. The following analyses are provided:

Fig. 1. No or R RIFLE stage versus RIFLE I or F. RIFLE stage adjudicated by sCr and Urine Output. Diseased group sample is obtained 24 hours prior to RIFLE I diagnosis.

Fig. 2. No or R RIFLE stage versus RIFLE I or F. RIFLE stage adjudicated by sCr. Diseased group sample is obtained 24 hours prior to RIFLE I diagnosis.

Fig. 3. No or R RIFLE stage versus RIFLE I or F. RIFLE stage adjudicated by Urine Output. Diseased group sample is obtained 24 hours prior to RIFLE I diagnosis.

Fig. 4. No RIFLE stage versus RIFLE R, I, or F. RIFLE stage adjudicated by sCr and Urine Output. Diseased group sample is obtained 24 hours prior to RIFLE R diagnosis.

Fig. 5. No RIFLE stage versus RIFLE R, I, or F. RIFLE stage adjudicated by sCr. Diseased group sample is obtained 24 hours prior to RIFLE R diagnosis.

Fig. 6. No RIFLE stage versus RIFLE R, I, or F. RIFLE stage adjudicated by Urine Output. Diseased group sample is obtained 24 hours prior to RIFLE R diagnosis.

Fig. 7. No, R, or I RIFLE stage versus RIFLE F. RIFLE stage adjudicated by sCr and Urine Output. Diseased group sample is obtained 24 hours prior to RIFLE I diagnosis.

Fig. 8. No, R, or I RIFLE stage versus RIFLE F. RIFLE stage adjudicated by Urine Output. Diseased group sample is obtained 24 hours prior to RIFLE I diagnosis.

Fig. 9. No or R RIFLE stage versus RIFLE I or F. RIFLE stage adjudicated by sCr and Urine Output. Diseased group sample is obtained at RIFLE I diagnosis.

Fig. 10. No or R RIFLE stage versus RIFLE I or F. RIFLE stage adjudicated by sCr. Diseased group sample is obtained at RIFLE I diagnosis.

Fig. 11. No or R RIFLE stage versus RIFLE I or F. RIFLE stage adjudicated by Urine Output. Diseased group sample is obtained at RIFLE I diagnosis.

Fig. 12. No RIFLE stage versus RIFLE R, I, or F. RIFLE stage adjudicated by sCr and Urine Output. Diseased group sample is obtained at RIFLE R diagnosis.

Fig. 13. No RIFLE stage versus RIFLE R, I, or F. RIFLE stage adjudicated by sCr. Diseased group sample is obtained at RIFLE R diagnosis.

Fig. 14. No RIFLE stage versus RIFLE R, I, or F. RIFLE stage adjudicated by Urine Output. Diseased group sample is obtained at RIFLE R diagnosis.

Fig. 15. No, R, or I RIFLE stage versus RIFLE F. RIFLE stage adjudicated by sCr and Urine Output. Diseased group sample is obtained at RIFLE I diagnosis.

Fig. 16. No, R, or I RIFLE stage versus RIFLE F. RIFLE stage adjudicated by sCr. Diseased group sample is obtained at RIFLE I diagnosis.

Fig. 17. No, R, or I RIFLE stage versus RIFLE F. RIFLE stage adjudicated by Urine Output. Diseased group sample is obtained at RIFLE I diagnosis.

Fig. 18. No or R RIFLE stage versus RIFLE I or F. RIFLE stage adjudicated by sCr and Urine Output. Diseased group sample is obtained 48 hours prior to RIFLE I diagnosis.

Fig. 19. No or R RIFLE stage versus RIFLE I or F. RIFLE stage adjudicated by sCr. Diseased group sample is obtained 48 hours prior to RIFLE I diagnosis.

Fig. 20. No or R RIFLE stage versus RIFLE I or F. RIFLE stage adjudicated by Urine Output. Diseased group sample is obtained 48 hours prior to RIFLE I diagnosis.

Fig. 21. No RIFLE stage versus RIFLE R, I, or F. RIFLE stage adjudicated by sCr and Urine Output. Diseased group sample is obtained 48 hours prior to RIFLE R diagnosis.

Fig. 22. No RIFLE stage versus RIFLE R, I, or F. RIFLE stage adjudicated by sCr. Diseased group sample is obtained 48 hours prior to RIFLE R diagnosis.

Fig. 23. No RIFLE stage versus RIFLE R, I, or F. RIFLE stage adjudicated by Urine Output. Diseased group sample is obtained 48 hours prior to RIFLE R diagnosis.

Fig. 24. No, R, or I RIFLE stage versus RIFLE F. RIFLE stage adjudicated by sCr and Urine Output. Diseased group sample is obtained 48 hours prior to RIFLE I diagnosis.

Fig. 25. Progression of RIFLE R to RIFLE I or F. RIFLE stage adjudicated by sCr and Urine Output. Sample is obtained at R diagnosis.

Fig. 26. Progression of RIFLE R to RIFLE I or F. RIFLE stage adjudicated by sCr. Sample is obtained at R diagnosis.

Fig. 27. Progression of RIFLE R to RIFLE I or F. RIFLE stage adjudicated by Urine Output. Sample is obtained at R diagnosis.

Fig. 28. No or R RIFLE stage versus RIFLE I or F. RIFLE stage adjudicated by sCr and Urine Output, within 48hrs of enrollment. Sample is obtained at enrollment.

Fig. 29. No or R RIFLE stage versus RIFLE I or F. RIFLE stage adjudicated by sCr, within 48hrs of enrollment. Sample is obtained at enrollment.

Fig. 30. No or R RIFLE stage versus RIFLE I or F. RIFLE stage adjudicated by Urine Output, within 48hrs of enrollment. Sample is obtained at enrollment.

Fig. 31. No RIFLE stage versus RIFLE R, I, or F. RIFLE stage adjudicated by sCr and Urine Output, within 48hrs of enrollment. Sample is obtained at enrollment.

Fig. 32. No RIFLE stage versus RIFLE R, I, or F. RIFLE stage adjudicated by sCr, within 48hrs of enrollment. Sample is obtained at enrollment.

Fig. 33. No RIFLE stage versus RIFLE R, I, or F. RIFLE stage adjudicated by Urine Output, within 48hrs of enrollment. Sample is obtained at enrollment.

Fig. 34. No, R, or I RIFLE stage versus RIFLE F. RIFLE stage adjudicated by sCr and Urine Output, within 48hrs of enrollment. Sample is obtained at enrollment.

Fig. 35. No, R, or I RIFLE stage versus RIFLE F. RIFLE stage adjudicated by Urine Output, within 48hrs of enrollment. Sample is obtained at enrollment.

Fig. 36. No or R RIFLE stage versus RIFLE I or F. RIFLE stage adjudicated by sCr and Urine Output, within 24hrs of enrollment. Sample is obtained at enrollment.

Fig. 37. No or R RIFLE stage versus RIFLE I or F. RIFLE stage adjudicated by sCr, within 24hrs of enrollment. Sample is obtained at enrollment.

Fig. 38. No or R RIFLE stage versus RIFLE I or F. RIFLE stage adjudicated by Urine Output, within 24hrs of enrollment. Sample is obtained at enrollment.

Fig. 39. No RIFLE stage versus RIFLE R, I, or F. RIFLE stage adjudicated by sCr and Urine Output, within 24hrs of enrollment. Sample is obtained at enrollment.

Fig. 40. No RIFLE stage versus RIFLE R, I, or F. RIFLE stage adjudicated by sCr, within 24hrs of enrollment. Sample is obtained at enrollment.

Fig. 41. No RIFLE stage versus RIFLE R, I, or F. RIFLE stage adjudicated by Urine Output, within 24hrs of enrollment. Sample is obtained at enrollment.

Fig. 42. No, R, or I RIFLE stage versus RIFLE F. RIFLE stage adjudicated by sCr and Urine Output, within 24hrs of enrollment. Sample is obtained at enrollment.

Fig. 43. No, R, or I RIFLE stage versus RIFLE F. RIFLE stage adjudicated by Urine Output, within 24hrs of enrollment. Sample is obtained at enrollment.

DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a biomarker recited in Table 2 herein, or one or more markers related thereto, is combined with one or more additional biomarkers listed in Table 2 herein and/or with one or more other biomarkers or clinical indicia, and the combination correlated to the renal status of the subject.
For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0.5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1.5-fold from baseline), or a reduction in urine output (documented oliguria of less than 0.5 ml/kg per hour for at least 6 hours). This term is synonymous with "acute kidney injury" or "AKI."

In the case of polypeptide biomarkers used herein, a "marker" or "biomarker" refers to polypeptides present in a biological sample that are derived from a particular biosynthetic precursor. In the case of biomarkers that are not polypeptides (e.g., Hyaluronic acid), a "marker" or "biomarker" refers to the particular molecular entity recited.

The following Table 2 provides a list of preferred biomarkers finding use in the present invention. In the table, the "recommended name" for the biomarker precursor from the Swiss-Prot "UniProtKB" database, and for most polypeptide biomarkers the Swiss-Prot entry number for the human precursor. In the event that the assay detects a complex, the Swiss Prot entry is listed for each member of the complex.

Table 2:

| Preferred Name | Swiss-Prot: | Preferred Name | Swiss-Prot: |
|---|---|---|---|
| 60 kDa heat shock protein, mitochondrial | P10809 | 72 kDa type IV collagenase | P08253 |
| 72 kDa type IV collagenase:Metalloproteinase inhibitor 1 complex | P08253 P01033 | 72 kDa type IV collagenase:Metalloproteinase inhibitor 2 complex | P08253 P16035 |
| 72 kDa type IV collagenase:Metalloproteinase inhibitor 4 complex | P08253 Q99727 | Adiponectin | Q15848 |
| Advanced glycosylation end product-specific receptor | Q15109 | Agouti-related protein | 000253 |
| Alkaline phosphatase, tissue-nonspecific isozyme | P05186 | Alpha-1-antitrypsin | P01009 |
| Alplia-1-antitrypsin:Neutrophil elastase complex | P01009 P08246 | Alplia-1-antitrypsin:Plasminogen complex | P01009 P00747 |
| Alpha-2 macroglobulin | P01023 | Alpha-2-HS-glycoprotein | P02765 |
| Alpha-fetoprotein | P02771 | Amphiregulin | P15514 |
| Amyloid Beta 40 | P05067 (aa672-711) | Amyloid Beta 42 | P05067 (aa672-713) |
| Angiogenin | P03950 | Angiopoietin-1 | Q15389 |
| Angiopoietin-1 receptor | Q02763 | Angiopoietin-2 | 015123 |
| Angiopoietin-related protein 3 | Q9Y5C1 | Angiopoietin-related protein 4 | Q9BY76 |
| Angiopoietin-related protein 6 | Q8NI99 | Anti-Cathepsin-G (ANCA) | NA |
| Antileukoproteinase | P03973 | Apolipoprotein A-I | P02647 |
| Apolipoprotein A-II | P02652 | Apolipoprotein B-100 | P04114 |
| Apolipoprotein C-III | P02656 | Apolipoprotein E | P02649 |
| Apolipoprotein(a) | P08519 | Appetite-regulating hormone | Q9UBU3 |
| Aspartate aminotransferase, cytoplasmic | P17174 | Bactericidal permeability-increasing protein | P17213 |
| Bcl2 antagonist of cell death | Q92934 | Beta-2-glycoprotein 1 | P02749 |
| Beta-2-microglobulin | P61769 | Beta-nerve growth factor | P01138 |
| Betacellulin | P35070 | Bone morpliogenetic protein 7 | P18075 |
| Brain-derived neurotrophic factor | P23560 | C-C motif chemokine 1 | P22362 |
| C-C motif chemokine 13 | Q99616 | C-C motif chemokine 15 | Q16663 |
| C-C motifchemokine 17 | Q92583 | C-C motif chemokine 18 | P55774 |
| C-C motif chemokine 19 | Q99731 | C-C motif chemokine 2 | P13500 |

(continued)

| Preferred Name | Swiss-Prot: | Preferred Name | Swiss-Prot: |
|---|---|---|---|
| C-C motif chemokine 20 | P78556 | C-C motif chemokine 21 | 000585 |
| C-C motif chemokine 22 | 000626 | C-C motif chemokine 23 | P55773 |
| C-C motif chemokine 24 | 000175 | C-C motif chemokine 26 | Q9Y258 |
| C-C motif chemokine 27 | Q9Y4X3 | C-C motif chemokine 3 | P10147 |
| C-C motif chemokine 4 | P13236 | C-C motif chemokine 5 | P13501 |
| C-C motif chemokine 7 | P80098 | C-C motif chemokine 8 | P80075 |
| C-Peptide | P01308(aa57-87) | C-reactive protein | P02741 |
| C-X-C motif chemokine 10 | P02778 | C-X-C motif chemokine 11 | 014625 |
| C-X-C motif chemokine 13 | O43927 | C-X-C motif chemokine 16 | Q9H2A7 |
| C-X-C motif chemokine 2 | P19875 | C-X-C motif chemokine 5 | P42830 |
| C-X-C motif chemokine 6 | P80162 | C-X-C motif chemokine 9 | Q07325 |
| Cadherin-1 | P12830 | Cadherin-16 | 075309 |
| Cadherin-3 | P22223 | Cadherin-5 | P33151 |
| Calbindin | P05937 | Calcitonin | P01258 |
| Calcitonin (Procalcitonin) | P01258-Pro | Cancer Antigen 15-3 | NA |
| Cancer Antigen 19-9 | NA | Carbonic anhydrase 9 | Q16790 |
| Carcinoembryonic antigen-related cell adhesion molecule 1 | P13688 | Carcinoembryonic antigen-related cell adhesion molecule 5 | P06731 |
| Caspase-1 | P29466 | Caspase-3, active | P42574 |
| Caspase-8 | Q14790 | Caspase-9 | P55211 |
| Cathepsin B | P07858 | Cathepsin D | P07339 |
| Catliepsin S | P25774 | CD40 ligand | P29965 |
| CD44 antigen | P16070 | Cellular tumor antigen p53 | P04637 |
| Choriogonadotropin subunit beta | P01233 | Ciliary neurotrophic factor | P26441 |
| Clusterin | P10909 | Coagulation factor VII | P08709 |
| Collagenase 3 | P45452 | Complement C3 | P01024 |
| Complement C4-B | P0C0L5 | Complement C5 | P01031 |
| Complement factor H | P08603 | Corticotropin | P01189(aal38-176) |
| Cortisol | NA | Creatine Kinase-MB | P12277 P06732 |
| Creatinine | NA | Cyclin-dependent kinase inhibitor 1 | P38936 |
| Cystatin-C | P01034 | Cytochrome c | P99999 |
| DDRGK domain-containing protein 1 | Q96HY6 | Dipeptidyl peptidase 4 | P27487 |
| E-selectin | P16581 | Endoglin | P17813 |
| Endostatin | P39060(aa1572-1754) | Endothelial protein C receptor | Q9UNN8 |

(continued)

| Preferred Name | Swiss-Prot: | Preferred Name | Swiss-Prot: |
|---|---|---|---|
| Endotlielin-1 | P05305 | Eotaxin | P51671 |
| Epidermal growth factor receptor | P00533 | Epiregulin | 014944 |
| Epithelial cell adhesion molecule | P16422 | Erythropoietin | P01588 |
| Erythropoietin receptor | P19235 | Fatty acid-binding protein, heart | P05413 |
| Fatty acid-binding protein, intestinal | P12104 | Fatty acid-binding protein, liver | P07148 |
| Ferritin | P02792 P02794 | Fibrinogen | P02671 P02675 P02679 |
| Fibroblast growth factor 19 | 095750 | Fibroblast growth factor 21 | Q9NSA1 |
| Fibroblast growth factor 23 | Q9GZV9 | Fibronectin | P02751 |
| Follistatin | P19883 | Follitropin | P01215 P01225 |
| Follitropin subunit beta | P01225 | Fractalkine | P78423 |
| Galectin-3 | P17931 | Gastric inhibitory polypeptide | P09681 |
| Glial cell line-derived neurotrophic factor | P39905 | Glial fibrillary acidic protein | P14136 |
| Glucagon | P01275 | Glucagon-like peptide 1 | P01275(aa9 8-127 aa98-128) |
| Glutathione S-transferase A1 | P08263 | Glutathione S-transferase P | P09211 |
| Granulocyte colony-stimulating factor | P09919 | Granulocyte-macrophage colony-stimulating factor | P04141 |
| Granzyme B | P10144 | Granzyme M | P51124 |
| Growth-regulated alpha protein | P09341 | Haptoglobin | P00738 |
| Heat shock 70 kDa protein 1 | P08107 | Heat shock protein beta-1 | P04792 |
| Heat shock protein beta-1 (phospho SER78 / phospho SER82) | P04792 (pS78/pS82) | Heat shock protein HSP 90-alpha | P07900 |
| Heme oxygenase 1 | P09601 | Heparan Sulfate | NA |
| Heparin-binding EGF-like growth factor | Q99075 | Heparin-binding growth factor 1 | P05230 |
| Heparin-binding growth factor 2 | P09038 | Hepatitis A virus cellular receptor 1 | 043656 |
| Hepatocyte growth factor | P14210 | Hepatocyte growth factor receptor | P08581 |
| Hyaluronic acid | NA | Hypoxia-inducible factor 1 alpha | Q16665 |
| Immunoglobulin A | NA | Immunoglobulin E | NA |
| Immunoglobulin M | NA | Immunoglogulin G1 | NA |
| Immunoglobulin G2 | NA | Immunoglogulin G3 | NA |
| Immunoglobulin G4 | NA | Insulin | P01308 |
| Insulin receptor substrate 1 | P35568 | Insulin-like growth factor 1 receptor | P08069 |

(continued)

| Preferred Name | Swiss-Prot: | Preferred Name | Swiss-Prot: |
|---|---|---|---|
| Insulin-like growth factor IA | P01343 | Insulin-like growth factor-binding protein 1 | P08833 |
| Insulin-like growth factor-binding protein 2 | P18065 | Insulin-like growth factor-binding protein 3 | P17936 |
| Insulin-like growth factor-binding protein 4 | P22692 | Insulin-like growth factor-binding protein 5 | P24593 |
| Insulin-like growth factor-binding protein 6 | P24592 | Insulin-like growth factor-binding protein 7 | Q16270 |
| Intercellular adhesion molecule 1 | P05362 | Intercellular adhesion molecule 2 | P13598 |
| Intercellular adhesion molecule 3 | P32942 | Interferon alpha-2 | P01563 |
| Interferon gamma | P01579 | Interleukin-1 alpha | P01583 |
| Interleukin-1 beta | P01584 | Interleukin-1 receptor antagonist protein | P18510 |
| Interleukin-1 receptor type I | P14778 | Interleukin-1 receptor type II | P27930 |
| Interleukin-10 | P22301 | Interleukin-11 | P20809 |
| Interleukin-12 | P29459 P29460 | Interleukin-12 subunit beta | P29460 |
| Interleukin-13 | P35225 | Interleukin-15 | P40933 |
| Interleukin-17A | Q16552 | Interleukin-18 | Q14116 |
| Interleukin-2 | P60568 | Interleukin-2 receptor alpha chain | P01589 |
| Interleukin-20 | Q9NYY1 | Interleukin-21 | Q9HBE4 |
| Interleukin-23 | Q9NPF7 P29460 | Interleukin-28A | Q8IZJ0 |
| Interleukin-29 | Q8IU54 | Interleukin-3 | P08700 |
| Interleukin-33 | O95760 | Interleukin-4 | P05112 |
| Interleukin-4 receptor alpha chain | P24394 | Interleukin-5 | P05113 |
| Interleukin-6 | P05231 | Interleukin-6 receptor subunit alpha | P08887 |
| Interleukin-6 receptor subunit beta | P40189 | Interleukin-7 | P13232 |
| Interleukin-8 | P10145 | Interleukin-9 | P15248 |
| Interstitial collagenase | P03956 | Interstitial collagenase:Metalloproteinase inhibitor 2 complex | P03956 P16035 |
| Involucrin | P07476 | Islet amyloid polypeptide | P10997 |
| Keratin, type I cytoskeletal 19 (aa311-367) | P08727 | Keratin, type II cytoskeletal 1; type1 cytoskeletal 10 (Keratin-1,-10 mix) | P04264 P13645 |
| Keratin, type II cytoskeletal 6 (6A, -6B, -6C mix) | P02538 P04259 P48668 | Kit ligand | P21583 |
| Lactotransferrin | P02788 | Leptin | P41159 |
| Leukemia inhibitory factor | P15018 | Lipopolysaccharide (serotypes -K,-O) | NA |

(continued)

| Preferred Name | Swiss-Prot: | Preferred Name | Swiss-Prot: |
|---|---|---|---|
| Lutropin | P01215 P01229 | Lutropin subunit beta | P01229 |
| Lymphatic vessel endothelial hyaluronic acid receptor 1 | Q9Y5Y7 | Lymphotactin | P47992 |
| Lymphotoxin-alpha | P01374 | Lysozyme C | P61626 |
| Macrophage colony-stimulating factor 1 | P09603 | Macrophage metalloelastase | P39900 |
| Macrophage migration inhibitory factor | P14174 | Malondialdehyde-modified low-density lipoprotein | NA |
| Matrilysin | P09237 | Matrix metalloproteinase-9 | P14780 |
| Matrix metalloproteinase-9:Metalloproteinase inhibitor 2 complex | P14780 P16035 | Matrix metalloproteinase-9:Metalloproteinase inhibitor 3 complex | P14780 P35625 |
| Metalloproteinase inhibitor 1 | P01033 | Metalloproteinase inhibitor 2 | P16035 |
| Metalloproteinase inhibitor 3 | P35625 | Metalloproteinase inhibitor 4 | Q99727 |
| Midkine | P21741 | Mix of Growth-regulated alpha, beta, and gamma proteins | P09341 P19875 P19876 |
| Monocyte differentiation antigen CD 14 | P08571 | Mucin-16 | Q8WXI7 |
| Myeloid differentiation primary response protein MyD88 | Q99836 | Myeloperoxidase | P05164 |
| Myoglobin | P02144 | Neprilysin | P08473 |
| Netrin-1 | O95631 | Neural cell adhesion molecule 1 | P13591 |
| Neuronal cell adhesion molecule | Q92823 | Neutrophil collagenase | P22894 |
| Neutrophil elastase | P08246 | Neutrophil gelatinase-associated lipocalin | P80188 |
| NF-kappa-B inhibitor alpha | P25963 | Nidogen-1 | P14543 |
| Nitric oxide synthase, inducible | P35228 | NT-pro-BNP | P16860 |
| Osteocalcin | P02818 | Osteopontin | P10451 |
| Oxidized low-density lipoprotein receptor 1 | P78380 | P-selectin | P16109 |
| P-selectin glycoprotein ligand 1 | Q14242 | Pancreatic prohormone | P01298 |
| Pappalysin-1 | Q13219 | Parathyroid hormone | P01270 |
| Peptide YY | P10082 | Pigment epithelium-derived factor | P36955 |
| Placenta growth factor | P49763 | Plasminogen activator inhibitor 1 | P05121 |
| Platelet basic protein | P02775 | Platelet endothelial cell adhesion molecule | P16284 |
| Platelet factor 4 | P02776 | Platelet-derived growth factor A | P04085 P01127 |
| Platelet-derived growth factor subunit A (dimer) | P04085 | Platelet-derived growth factor subunit B (dimer) | P01127 |
| Poly [ADP-ribose] polymerase 1 (cleaved) | P09874 | Pro-epidermal growth factor | P01133 |

(continued)

| Preferred Name | Swiss-Prot: | Preferred Name | Swiss-Prot: |
|---|---|---|---|
| Pro-Interleukin-1 beta | P01584-Pro | Pro-interleukin-16 | Q14005 |
| Prolactin | P01236 | Prostate-specific antigen | P07288 |
| Prostatic acid phosphatase | P15309 | Protein NOV homolog | P48745 |
| Protein S100-A12 | P80511 | Protein S100-B | P04271 |
| Protransforming growth factor alpha | P01135 | Renin | P00797 |
| Resistin | Q9HD89 | Serum albumin | P02768 |
| Serum amyloid A protein | P02735 | Serum amyloid P-component | P02743 |
| Sex hormone-binding globulin | P04278 | SL cytokine | P49771 |
| Somatotropin | P01241 | Stromal cell-derived factor 1 | P48061 |
| Stromelysin-1 | P08254 | Stromelysin-1:Metalloproteinase inhibitor 2 complex | P08254 P16035 |
| Stromelysin-2 | P09238 | Tenascin | P24821 |
| Thrombomodulin | P07204 | Thrombopoietin | P40225 |
| Thrombospondin-1 | P07996 | Tbrombospondin-2 | P35442 |
| Thymic stromal lymphopoietin | Q969D9 | Thyrotropin | P01215 P01222 |
| Thyroxine-binding globulin | P05543 | Tissue factor | P13726 |
| Tissue-type plasminogen activator | P00750 | Transforming growth factor beta-1 | P01137 |
| Transforming growth factor beta-2 | P61812 | Transforming growth factor beta-3 | P10600 |
| Transmembrane glycoprotein NMB | Q14956 | Transthyretin | P02766 |
| Trefoil factor 3 | Q07654 | Tubulointerstitial nephritis antigen | Q9UJW2 |
| Tumor necrosis factor | P01375 | Tumor necrosis factor ligand superfamily member 10 | P50591 |
| Tumor necrosis factor ligand superfamily member 11 | O14788 | Tumor necrosis factor ligand superfamily member 6 | P48023 |
| Tumor necrosis factor receptor superfamily member 10B | 014763 | Tumor necrosis factor receptor superfamily member 11B | O00300 |
| Tumor necrosis factor receptor superfamily member 1A | P19438 | Tumor necrosis factor receptor superfamily member 1B | P20333 |
| Tumor necrosis factor receptor superfamily member 5 | P25942 | Tumor necrosis factor receptor superfamily member 6 | P25445 |
| Tumor necrosis factor receptor superfamily member 8 | P28908 | Urokinase plasminogen activator surface receptor | Q03405 |
| Urokinase-type plasminogen activator | P00749 | Vascular cell adhesion protein 1 | P19320 |
| Vascular endothelial growth factor A | P15692 | Vascular endothelial growth factor D | 043915 |

(continued)

| Preferred Name | Swiss-Prot: | Preferred Name | Swiss-Prot: |
|---|---|---|---|
| Vascular endothelial growth factor receptor 1 | P17948 | Vascular endothelial growth factor receptor 2 | P35968 |
| Vascular endothelial growth factor receptor 3 | P35916 | Versican core protein | P13611 |
| Vitamin D-binding protein | P02774 | Vitamin K-dependent protein C | P04070 |
| von Willebrand Factor | P04275 | WAP four-disulfide core domain protein 2 | Q14508 |

Included in this list are a number of proteins which exist in one form as type-I, type-II, or GPI-anchored membrane proteins. Typically, such membrane proteins comprise a substantial extracellular domain, some or all of which can be detected as soluble forms present in aqueous samples such as blood, serum, plasma, urine, etc., either as cleavage products or as splice variants which delete an effective membrane spanning domain. These membrane proteins include Swiss-Prot entry numbers 014788, 014944, 075309, P00797, P05186, P08473, P13688, P15514, P22223, P27487, P35070, Q03405, Q14956, Q16790, Q99075, Q9Y5Y7Q15109, Q02763, P 17213, P12830, P33151, P06731, P29965, P16070, Q9H2A7, P 17813, Q9UNN8, P00533, P16422, P19235, P16581, P78423, 043656, P08581, P08069, P05362, P13598, P32942, P14778, P27930, P01589, P24394, P08887, P40189, P21583, P09603, P08571, Q8WXI7, P13591, Q92823, P78380, P16284, P01133, P15309, P01135, P16109, Q14242, P49771, P07204, P13726, P01375, P50591, P48023, O14763, P19438, P20333, P25942, P25445, P28908, P19320, P17948, and P35968. Preferred assays detect soluble forms of these biomarkers.

In this regard, the skilled artisan will understand that the signals obtained from an immunoassay are a direct result of complexes formed between one or more antibodies and the corresponding target biomolecule (i.e., the analyte) containing the necessary epitope(s) to which the antibodies bind. While such assays may detect the full length biomarker and the assay result be expressed as a concentration of a biomarker of interest, the signal from the assay is actually a result of all such "immunoreactive" molecules present in the sample. Expression of biomarkers may also be determined by means other than immunoassays, including protein measurements (such as dot blots, western blots, chromatographic methods, mass spectrometry, etc.) and nucleic acid measurements (mRNA quatitation). This list is not meant to be limiting.

As used herein, the term "relating a signal to the presence or amount" of an analyte reflects this understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, etc.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

As used herein, a "plurality" as used herein refers to at least two. Preferably, a plurality refers to at least 3, more preferably at least 4, even more preferably at least 5, even more preferably at least 10, and most preferably at least 20.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994, each of which is hereby incorporated by reference in its entirety, including all tables, figures and claims.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, *etc.)* as well as molecules that may be indirectly detected by production of a detectable reaction product (e.g., enzymes such as horseradish peroxidase, alkaline phosphatase, *etc.)* or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and hetero functional cross-linkers (containing non-identical reactive groups). Homobifunctional cross-linkers that couple through amines, sulfhydryls or react non- specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. See, e.g. Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')$_2$ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ M$^{-1}$, and preferably between about $10^8$ M-1 to about $10^9$ M$^{-1}$, about $10^9$ M$^{-1}$ to about $10^{10}$ M$^{-1}$, or about $10^{10}$ M$^{-1}$ to about $10^{12}$ M$^{-1}$.

Affinity is calculated as Kd = koff/kon (koff is the dissociation rate constant, Kon is the association rate constant and Kd is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. See, e.g., van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. See, e.g, Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. See, e.g., U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.Assay Correlations

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, *etc.)* include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Cathepsin B (P07858); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (PO1241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-lalpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltrans-

ferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); δ subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itml, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-la (P10147); MIP-3a (P78556); MIP-lbeta (P13236); MIP-ld (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times \text{24-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, *etc.* The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 $m^2$ = 2 points, 20-40 mL/min/1.73 $m^2$ = 4 points, < 20 mL/min/1.73 $m^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and able and willing to provide written informed consent for study participation and to comply with all study procedures.

Exclusion Criteria

known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 900 adults expected to be in the ICU for at least 48 hours will be enrolled. To be enrolled in the study, each patient must meet all of the following inclusion criteria and none of the following exclusion criteria:

Inclusion Criteria

males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;

increased Intra-Abdominal Pressure with acute decompensated heart failure; and severe trauma as the primary reason for ICU admission and likely to be hospitalized in the ICU for 48 hours after enrollment;

Study population 3: approximately 300 patients

expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment.

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets only the SBP < 90 mmHg inclusion criterion set forth above, and does not have shock in the attending physician's or principal investigator's opinion.

After providing informed consent, an EDTA anti-coagulated blood sample (10 mL) and a urine sample (25-30 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are is measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Biomarker Panels

The following exemplary method was used to segregate a patient population into two groups, which will be referred to for convenience as "non-diseased" (NonDis) and "diseased" (Dis). The combined NonDis/Dis population formed a data set, and the following analysis was performed. Multiple analytes were measured from either blood or urine or both, for the subjects in the data set. Blood and urine measurements were treated as separate biomarkers in calculating panel results. Panels were formed by algorithmically combing two or more analytes in one of two ways: (i) a very simple product and division of analyte values; and (ii) logistic regression.

Logistic regression is a method widely used for models which have a binary outcome, such as the "diseased" "non-diseased" dichotomy presented here. The method will be briefly described below, full treatments can be found in the literature. The model used is:

$$\Pi_i(y_i = 1 | \boldsymbol{x}_i) = \frac{e^{\alpha + \beta x_i}}{1 + e^{\alpha + \beta x_i}}$$

In this model, **x** and P are vectors, **x** representing the different observables or analyte values, $y_i = 1$ indicates a diseased state, and $\Pi_i$ is the model probability of this state for the $i^{th}$ case given **x**$_i$. The panel value for each sample is $\Pi_i$. The log odds or logit is:

$$logit = ln\left[\frac{\Pi_i(y_i = 1 | \boldsymbol{x}_i)}{1 - \Pi_i(y_i = 1 | \boldsymbol{x}_i)}\right] = \alpha + \boldsymbol{\beta}\boldsymbol{x}_i$$

Define $p_i$ as probability of observing the true outcome:

$$p_i = \begin{cases} \Pi_i(y_i = 1 | \boldsymbol{x}_i) & if\ deseased \\ 1 - \Pi_i(y_i = 1 | \boldsymbol{x}_i) & if\ non-diseased \end{cases}$$

The likelihood function is the product of the probabilities of observing the true outcomes, so the log likelihood (LL) is:

$$LL = ln\big(L(\alpha,\boldsymbol{\beta})\big) = \sum ln(p_i)$$

To find the parameters, $\alpha$ and $\beta$, that best fit the model, the negative log likelihood (-LL) is minimized. This minimization was performed using the Levenberg-Marquardt method (Numerical Recipes The Art of Scientific Computing, Third Edition, Cambridge University Press, 2007). The initial point for each parameter is 0.

A commonly used statistic to compare the fit of two nested models is the likelihood ratio test. This statistic, or the deviance, is the difference in twice the negative log likelihood (-2LL) for the two model, and is asymptotically a $\chi^2$ with DF equal to the change in the number of degrees of freedom (number of analytes) between the models. The p-value is calculated from this statistic. The null hypothesis is that the logistic model is not different than a constant model ($\beta$ set to 0), is tested for each model using the likelihood ratio test. The 'model p-value' is defined as the probability that the null hypothesis is true. For the constant model, a closed form solution for $\alpha$ and -2LL can be found. It is a function of the number of diseased (#D) and the number of non-diseased (#ND) samples in the data set.

$$\alpha = ln\left(\frac{\#D}{\#ND}\right)$$

$$-2LL = -2 * [\#ND \ln(1 - \Pi) + \#D \ln(\Pi)]$$

*Where*:

$$\Pi = \frac{\#D}{(\#ND + \#D)}$$

Each analyte can be tested for significance by holding its β at zero and comparing the model found without the analyte to the full model. The likelihood ratio test is used with 1 degree of freedom. The 'analyte p-value' is defined as the probability that the models are not different when that analyte is removed.

The analyte concentrations used in the panel can either be log transformed or untransformed. For each analyte in the panel, all the permutations of the analytes being log transformed/not transformed are calculated. The permutation with the lowest model p-value is used for that panel. For two marker panels there are 4 permutations, for six marker panels there are 8.

Table 3 contains a list of assays that were measured on urine ("U") and EDTA plasmas ("E") samples. Also listed, are the two letter codes that will be used throughout this document to refer to these assays according to the following convention: if the first character of the code is capitalized, this means the assay was measured in urine. If the second character of the code is capitalized, this means the assay was measured in EDTA plasma. If both characters of the code are capitalized, then the assay was measured in both urine and EDTA plasma. In the table some assays have a single code in capitals, indicating that the same assay format (e.g., microtiter dish, automated analyzer, etc.) was used for both urine and plasma. Other assays have separate U and E codes. This indicates that different assay formats were used for urine and EDTA plasma. A code of "**" indicates that an insufficient number of samples were measured on that assay and it will not appear in the panel results presented hereinafter.

Table 3:

| Preferred Name | Swiss-Prot: | Code U,E | Preferred Name | Swiss-Prot: | Code U,E |
|---|---|---|---|---|---|
| 60 kDa heat shock protein, mitochondrial | P10809 | YH | 72 kDa type IV collagenase | P08253 | CP |
| 72 kDa type IV collagenase:Metalloproteinase inhibitor 1 complex | P08253 P01033 | FE | 72 kDa type IV collagenase:Metalloproteinase inhibitor 2 complex | P08253 P16035 | FD |
| 72 kDa type IV collagenase:Metalloproteinase inhibitor 4 complex | P08253 Q99727 | FF | Adiponectin | Q15848 | AD |
| Advanced glycosylation end product-specific receptor | Q15109 | KK | Agouti-related protein | 000253 | SI |
| Alkaline phosphatase, tissue-nonspecific isozyme | P05186 | UL | Alplia-1-antitrypsin | P01009 | $J_j$,aC |
| Alpha-1-antitrypsin Neutrophil elastase complex | P01009 P08246 | WN | Alpha-1-antitrypsin Plasminogen complex | P01009 P00747 | TK |
| Alpha-2 macroglobulin | P01023 | Ir,aE | Alpha-2-HS-glycoprotein | P02765 | IO |
| Alpha-fetoprotein | P02771 | AF | Amphiregulin | P15514 | TN |
| Amyloid Beta 40 | P05067 (aa672-711) | YE | Amyloid Beta 42 | P05067 (aa672-713) | TM |
| Angiogenin | P03950 | Tz,wJ | Angiopoietin-1 | Q15389 | DR |
| Angiopoietin-1 receptor | Q02763 | Us,gV | Angiopoietin-2 | 015123 | HW |
| Angiopoietin-related protein 3 | Q9Y5C1 | WC | Angiopoietin-related protein 4 | Q9BY76 | WD |
| Angiopoietin-related protein 6 | Q8NI99 | WE | Anti-Cathepsin-G (ANCA) | NA | DS |
| Antileukoproteinase | P03973 | GL | Apolipoprotein A-I | P02647 | II,aG |
| Apolipoprotein A-II | P02652 | IJ | Apolipoprotein B-100 | P04114 | IH |
| Apolipoprotein C-III | P02656 | Ik,aH | Apolipoprotein E | P02649 | In,qI |
| Apolipoprotein(a) | P08519 | It,cJ | Appetite-regulating hormone | Q9UBU3 | RA |
| Aspartate aminotransferase, cytoplasmic | P17174 | DB | Bactericidal permeability-increasing protein | P17213 | DU |
| Bcl2 antagonist of cell death | Q92934 | DQ | Beta-2-glycoprotein 1 | P02749 | Im, aI |
| Beta-2-microglobulin | P61769 | AJ | Beta-nerve growth factor | P01138 | Vz,pY |
| Betacellulin | P35070 | TO | Bone morphogenetic protein 7 | P18075 | UO |

32

(continued)

| Preferred Name | Swiss-Prot: | Code U,E | Preferred Name | Swiss-Prot: | Code U,E |
|---|---|---|---|---|---|
| Brain-derived neurotrophic factor | P23560 | Jn,aK | C-C motif chemokine 1 | P22362 | MU |
| C-C motif chemokine 13 | Q99616 | Mq,oT | C-C motif chemokine 15 | Q16663 | MW |
| C-C motif chemokine 17 | Q92583 | Mx,oV | C-C motif chemokine 18 | P55774 | JL |
| C-C motif chemokine 19 | Q99731 | Nq,iA | C-C motif chemokine 2 | P13500 | Ou,cL |
| C-C motif chemokine 20 | P78556 | NR | C-C motif chemokine 21 | O00585 | MY |
| C-C motif chemokine 22 | O00626 | Mj,cM | C-C motif chemokine 23 | P55773 | VI |
| C-C motif chemokine 24 | O00175 | MZ | C-Cmotif chemokine 26 | Q9Y258 | NA |
| C-C motif chemokine 27 | Q9Y4X3 | NB | C-C motif chemokine 3 | P10147 | Mk,cN |
| C-C motif chemokine 4 | P13236 | CO | C-C motif chemokine 5 | P13501 | Js,cY |
| C-C motif chemokine 7 | P80098 | MI | C-C motif chemokine 8 | P80075 | MP |
| C-Peptide | P01308(aa57-87) | QV | C-reactive protein | P02741 | IP |
| C-X-C motif chemokine 10 | P02778 | OW | C-X-C motif chemokine 11 | O14625 | OH |
| C-X-C motif chemokine 13 | O43927 | MT | C-X-C motif chemokine 16 | Q9H2A7 | EF |
| C-X-C motif chemokine 2 | P19875 | PO | C-X-C motif chemokine 5 | P42830 | Mr,aV |
| C-X-C motif chemokine 6 | P80162 | NO | C-X-C motif chemokine 9 | Q07325 | NN |
| Cadherin-1 | P12830 | Gd,oO | Cadherin-16 | O75309 | TH |
| Cadherin-3 | P22223 | UG | Cadherin-5 | P33151 | GT |
| Calbindin | P05937 | YD | Calcitonin | P01258 | AO |
| Calcitonin (Procalcitonin) | P01258-Pro | HO | Cancer Antigen 15-3 | NA | IC |
| Cancer Antigen 19-9 | NA | AN | Carbonic anhydrase 9 | Q16790 | VH |
| Carcinoembryonic antigen-related cell adhesion molecule 1 | P13688 | VT | Carcinoembryonic antigen-related cell adhesion molecule 5 | P06731 | AR |
| Caspase-1 | P29466 | DV | Caspase-3, active | P42574 | Kx,dX |
| Caspase-8 | Q14790 | DW | Caspase-9 | P55211 | DO |

| Preferred Name | Swiss-Prot: | Code U,E | Preferred Name | Swiss-Prot: | Code U,E |
|---|---|---|---|---|---|
| Cathepsin B | P07858 | UT | Cathepsin D | P07339 | JP |
| Cathepsin S | P25774 | UW | CD40 ligand | P29965 | MI,aQ |
| CD44 antigen | P16070 | GC | Cellular tumor antigen p53 | P04637 | RX |
| Choriogonadotropin subunit beta | P01233 | Zx,zH | Ciliary neurotrophic factor | P26441 | SJ |
| Clusterin | P10909 | Ii,sO | Coagulation factor VII | P08709 | BB |
| Collagenase 3 | P45452 | Lt,lO | Complement C3 | P01024 | AL |
| Complement C4-B | P0C0L5 | JM | Complement C5 | P01031 | VU |
| Complement factor H | P08603 | QG | Corticotropin | P01189(aa1 38-176) | JY |
| Cortisol | NA | Rh,rP | Creatine Kinase-MB | P12277 P06732 | AS |
| Creatinine | NA | AA | Cyclin-dependent kinase inhibitor 1 | P38936 | Fw,rW |
| Cystatin-C | P01034 | IZ | Cytochrome c | P99999 | KY |
| DDRGK domain-containing protein 1 | Q96HY6 | WM | Dipeptidyl peptidase 4 | P27487 | VQ |
| E-selectin | P16581 | PH | Endoglin | P17813 | UP |
| Endostatin | P39060(aal 572-1754) | Ua,wK | Endothelial protein C receptor | Q9UNN8 | Vj,eC |
| Endothelin-1 | P05305 | AW | Eotaxin | P51671 | Or,aY |
| Epidermal growth factor receptor | P00533 | Kd,tQ | Epiregulin | 014944 | TR |
| Epithelial cell adhesion molecule | P16422 | UV | Erythropoietin | P01588 | Uk,aZ |
| Erythropoietin receptor | P19235 | ED | Fatty acid-binding protein, heart | P05413 | BA |
| Fatty acid-binding protein, intestinal | P12104 | EQ | Fatty acid-binding protein, liver | P07148 | EZ |
| Ferritin | P02792 P02794 | BC | Fibrinogen | P02671 P02675 P02679 | Iq,bE |
| Fibroblast growth factor 19 | 095750 | WG | Fibroblast growth factor 21 | Q9NSA1 | WH |

(continued)

| Preferred Name | Swiss-Prot: | Code U,E |
|---|---|---|
| Fibroblast growth factor 23 | Q9GZV9 | WF |
| Fibronectin | P02751 | Rm,jB |
| Follistatin | P19883 | HX |
| Follitropin | P01215 / P01225 | JU |
| Follitropin subunit beta | P01225 | SF |
| Fractalkine | P78423 | UM |
| Galectin-3 | P17931 | VB |
| Gastric inhibitory polypeptide | P09681 | QY |
| Glial cell line-derived neurotrophic factor | P39905 | TL |
| Glial fibrillary acidic protein | P14136 | EH |
| Glucagon | P01275 | QZ |
| Glucagon-like peptide 1 | P01275(aa98-127 aa98-128) | QX |
| Glutathione S-transferase A1 | P08263 | Dp,bI |
| Glutathione S-transferase P | P09211 | FY |
| Granulocyte colony-stimulating factor | P09919 | Hq,bF |
| Granulocyte-macrophage colony-stimulating factor | P04141 | Pd,bH |
| Granzyme B | P10144 | EO |
| Granzyme M | P51124 | YF |
| Growth-regulated alpha protein | P09341 | Tj,uI |
| Haptoglobin | P00738 | Iu,bJ |
| Heat shock 70 kDa protein 1 | P08107 | Yi,eP |
| Heat shock protein beta-1 | P04792 | Yg,rS |
| Heat shock protein beta-1 (phospho SER78 / phospho SER82) | P04792 (pS78/pS82) | YK |
| Heat shock protein HSP 90-alpha | P07900 | YJ |
| Heme oxygenase 1 | P09601 | EM |
| Heparan Sulfate | NA | TI |
| Heparin-binding EGF-like growth factor | Q99075 | TT |
| Heparin-binding growth factor 1 | P05230 | Ub,wL |
| Heparin-binding growth factor 2 | P09038 | Lv,tS |
| Hepatitis A virus cellular receptor 1 | 043656 | Uh,tF |
| Hepatocyte growth factor | P14210 | OK |
| Hepatocyte growth factor receptor | P08581 | RT |
| Hyaluronic acid | NA | ET |
| Hypoxia-inducible factor 1 alpha | Q16665 | OE |
| Immunoglobulin A | NA | Qe,bM |
| Immunoglobulin E | NA | BN |
| Immunoglobulin M | NA | Pz,bP |
| Immunoglogulin G1 | NA | QA |
| Immunoglogulin G2 | NA | QD |
| Immunoglogulin G3 | NA | QB |
| Immunoglobulin G4 | NA | QC |
| Insulin | P01308 | CH |
| Insulin receptor substrate 1 | P35568 | RU |
| Insulin-like growth factor 1 receptor | P08069 | RY |

(continued)

| Preferred Name | Swiss-Prot: | Code U,E | Preferred Name | Swiss-Prot: | Code U,E |
|---|---|---|---|---|---|
| Insulin-like growth factor IA | P01343 | BO | Insulin-like growth factor-binding protein 1 | P08833 | OF |
| Insulin-like growth factor-binding protein 2 | P18065 | JD | Insulin-like growth factor-binding protein 3 | P17936 | JG |
| Insulin-like growth factor-binding protein 4 | P22692 | JE | Insulin-like growth factor-binding protein 5 | P24593 | JF |
| Insulin-like growth factor-binding protein 6 | P24592 | JH | Insulin-like growth factor-binding protein 7 | Q16270 | JI |
| Intercellular adhesion molecule 1 | P05362 | JO | Intercellular adhesion molecule 2 | P13598 | GN |
| Intercellular adhesion molecule 3 | P32942 | PK | Interferon alpha-2 | P01563 | LY |
| Interferon gamma | P01579 | Pc,bL | Interleukin-1 alpha | P01583 | Lz,bX |
| Interleukin-1 beta | P01584 | PF | Interleukin-1 receptor antagonist protein | P18510 | Ma,bZ |
| Interleukin-1 receptor type I | P14778 | KF | Interleukin-1 receptor type II | P27930 | KG |
| Interleukin-10 | P22301 | Pb,bQ | Interleukin-11 | P20809 | NT |
| Interleukin-12 | P29459 P29460 | Oz,bS | Interleukin-12 subunit beta | P29460 | Oi,bR |
| Interleukin-13 | P35225 | MF | Interleukin-15 | P40933 | Mg,bU |
| Interleukin-17A | Q16552 | MH | Interleukin-18 | Q14116 | Uf,bW |
| Interleukin-2 | P60568 | Pa,cA | Interleukin-2 receptor alpha chain | P01589 | MM |
| Interleukin-20 | Q9NYY1 | NI | Interleukin-21 | Q9HBE4 | NJ |
| Interleukin-23 | Q9NPF7 P29460 | NC | Interleukin-28A | Q8IZJ0 | NK |
| Interleukin-29 | Q8IU54 | NU | Interleukin-3 | P08700 | Mb,cB |
| Interleukin-33 | O95760 | NL | Interleukin-4 | P05112 | Mc,cC |
| Interleukin-4 receptor alpha chain | P24394 | KI | Interleukin-5 | P05113 | Og,cD |
| Interleukin-6 | P05231 | Pg,cE | Interleukin-6 receptor subunit alpha | P08887 | Kj,oD |
| Interleukin-6 receptor subunit beta | P40189 | KE | Interleukin-7 | P13232 | Md,cF |
| Interleukin-8 | P10145 | Pe,cG | Interleukin-9 | P15248 | ME |

| Preferred Name | Swiss-Prot: | Code U,E | Preferred Name | Swiss-Prot: | Code U,E |
|---|---|---|---|---|---|
| Interstitial collagenase | P03956 | Lp,IK | Interstitial collagenase:Metalloproteinase inhibitor 2 complex | P03956 P16035 | FC |
| Involucrin | P07476 | Rf,rN | Islet amyloid polypeptide | P10997 | QW |
| Keratin, type I cytoskeletal 19 (aa311-367) | P08727 | Zw,zG | Keratin, type II cytoskeletal 1; type 1 cytoskeletal 10 (Keratin-1,-10 mix) | P04264 P13645 | Rj,rR |
| Keratin, type II cytoskeletal 6 (6A, -6B, -6C mix) | P02538 P04259 P48668 | Ri,rQ | Kit ligand | P21583 | Ng,dA |
| Lactotransferrin | P02788 | FA | Leptin | P41159 | Hu,cI |
| Leukemia inhibitory factor | P15018 | ND | Lipopolysaccharide (serotypes -K,-O) | NA | Rg,rO |
| Lutropin | P01215 P01229 | JV | Lutropin subunit beta | P01229 | SH |
| Lymphatic vessel endothelial hyaluronic acid receptor 1 | Q9Y5Y7 | VW | Lymphotactin | P47992 | Ns,cK |
| Lymphotoxin-alpha | P01374 | DI | Lysozyme C | P61626 | FB |
| Macrophage colony-stimulating factor 1 | P09603 | NM | Macrophage metalloelastase | P39900 | Ld,IN |
| Macrophage migration inhibitory factor | P14174 | KS | Malondialdeliyde-modified low-density lipoprotein | NA | HF |
| Matrilysin | P09237 | Lh,IL | Matrix metalloproteinase-9 | P14780 | Lj,cR |
| Matrix metalloproteinase-9:Metalloproteinase inhibitor 2 complex | P14780 P16035 | Oa,** | Matrix metalloproteinase-9:Metalloproteinase inhibitor 3 complex | P14780 P35625 | OB |
| Metalloproteinase inhibitor 1 | P01033 | Nv,dF | Metalloproteinase inhibitor 2 | P16035 | NW |
| Metalloproteinase inhibitor 3 | P35625 | Nx,sC | Metalloproteinase inhibitor 4 | Q99727 | NY |
| Midkine | P21741 | EX | Mix of Growth-regulated alpha, beta, and gamma proteins | P09341 P19875 P19876 | LX |
| Monocyte differentiation antigen CD 14 | P08571 | VO | Mucin-16 | Q8WXI7 | Id,aM |

EP 3 070 474 A2

| Preferred Name | Swiss-Prot: | Code U,E | Preferred Name | Swiss-Prot: | Code U,E |
|---|---|---|---|---|---|
| Myeloid differentiation primary response protein MyD88 | Q99836 | VC | Myeloperoxidase | P05164 | CS |
| Myoglobin | P02144 | CT | Neprilysin | P08473 | UY |
| Netrin-1 | 095631 | FN | Neural cell adhesion molecule 1 | P13591 | JT |
| Neuronal cell adhesion molecule | Q92823 | UZ | Neutrophil collagenase | P22894 | Li,IM |
| Neutrophil elastase | P08246 | FP | Neutrophil gelatinase-associated lipocalin | P80188 | FR |
| NF-kappa-B inhibitor alpha | P25963 | RV | Nidogen-1 | P14543 | VS |
| Nitric oxide synthase, inducible | P35228 | EW | NT-pro-BNP | P16860 | HC |
| Osteocalcin | P02818 | RC | Osteopontin | P10451 | OP |
| Oxidized low-density lipoprotein receptor 1 | P78380 | HB | P-selectin | P16109 | PI |
| P-selectin glycoprotein ligand 1 | Q14242 | GP | Pancreatic proliomione | P01298 | QU |
| Pappalysin-1 | Q13219 | CW | Parathyroid hormone | P01270 | RB |
| Peptide YY | P10082 | QT | Pigment epithelium-derived factor | P36955 | JK |
| Placenta growth factor | P49763 | Ue,tU | Plasminogen activator inhibitor 1 | P05121 | CU |
| Platelet basic protein | P02775 | VP | Platelet endothelial cell adhesion molecule | P16284 | HR |
| Platelet factor 4 | P02776 | VV | Platelet-derived growth factor A | P04085 P01127 | JR |
| Platelet-derived growth factor subunit A (dimer) | P04085 | JQ | Platelet-derived growth factor subunit B (dimer) | P01127 | HV |
| Poly [ADP-ribose] polymerase 1 (cleaved) | P09874 | Kz,** | Pro-epidermal growth factor | P01133 | Lu,aU |
| Pro-Interleukin-1 beta | P01584-Pro | GH | Pro-interleukin-16 | Q14005 | Mv,bV |
| Prolactin | P01236 | IB | Prostate-specific antigen | P07288 | CX |
| Prostatic acid phosphatase | P15309 | CV | Protein NOV homolog | P48745 | UU |
| Protein S100-A12 | P80511 | AX | Protein S100-B | P04271 | XA |
| Protransforming growth factor alpha | P01135 | Mn,tX | Renin | P00797 | Sr,sK |
| Resistin | Q9HD89 | WB | Serum albumin | P02768 | St,zA |

EP 3 070 474 A2

38

| Preferred Name | Swiss-Prot: | Code U,E | Preferred Name | Swiss-Prot: | Code U,E |
|---|---|---|---|---|---|
| Serum amyloid A protein | P02735 | HP | Serum auryloid P-component | P02743 | ls,cZ |
| Sex hormone-binding globulin | P04278 | DC | SL cytokine | P49771 | LW |
| Somatotropin | P01241 | BG | Stromal cell-derived factor 1 | P48061 | MS |
| Stromelysin-1 | P08254 | CQ | Stromelysin-1:Metalloproteinase inhibitor 2 complex | P08254 P16035 | FI |
| Stromelysin-2 | P09238 | UR | Tenascin | P24821 | TV |
| Tbrombomodulin | P07204 | Pj,liG | Thrombopoietin | P40225 | Ne,dJ |
| Tbrombospondin-1 | P07996 | GW | Thrombospondin-2 | P35442 | Uc,wP |
| Thymic stromal lymphopoietin | Q969D9 | NH | Thyrotropin | P01215 P01222 | DK |
| Thyroxine-binding globulin | P05543 | DD | Tissue factor | P13726 | DE |
| Tissue-type plasminogen activator | P00750 | HL | Transforming growth factor beta-1 I | P01137 | QI,qO |
| Transforming growth factor beta-2 | P61812 | Qm,qP | Transforming growth factor beta-3 | P10600 | Qn,qQ |
| Transmembrane glycoprotein NMB | Q14956 | UN | Transthyretin | P02766 | QH |
| Trefoil factor 3 | Q07654 | Ss,sM ' | Tubulointerstitial nephritis antigen | Q9UJW2 | GZ |
| Tumor necrosis factor | P01375 | Oy,dH ' | Tumor necrosis factor ligand superfamily member 10 | P50591 | NF |
| Tumor necrosis factor ligand superfamily member 11 | O14788 | YL | Tumor necrosis factor ligand superfamily member 6 | P48023 | KR |
| Tumor necrosis factor receptor superfamily member 10B | O14763 | RZ | Tumor necrosis factor receptor superfamily member 11B | O00300 | ON |
| Tumor necrosis factor receptor superfamily member 1A | P19438 | KI,oQ | Tumor necrosis factor receptor superfamily member 1B | P20333 | DG |
| Tumor necrosis factor receptor superfamily member 5 | P25942 | AP | Tumor necrosis factor receptor superfamily member 6 | P25445 | KQ |
| Tumor necrosis factor receptor superfamily member 8 | P28908 | KC | Urokinase plasminogen activator surface receptor | Q03405 | UX |
| Urokinase-type plasminogen activator | P00749 | VA | Vascular cell adhesion protein 1 | P19320 | DL |

EP 3 070 474 A2

(continued)

| Preferred Name | Swiss-Prot: | Code U,E | Preferred Name | Swiss-Prot: | Code U,E |
|---|---|---|---|---|---|
| Vascular endothelial growth factor A | P15692 | Om,dM | Vascular endothelial growth factor D | 043915 | Ud,wQ |
| Vascular endothelial growth factor receptor 1 | P17948 | KN | Vascular endothelial growth factor receptor 2 | P35968 | KO |
| Vascular endothelial growth factor receptor 3 | P35916 | KP | Versican core protein | P13611 | HA |
| Vitamin D-binding protein | P02774 | PS | Vitamin K-dependent protein C | P04070 | GB |
| von Willebrand Factor | P04275 | Iv,dN | WAP four-disulfide core domain protein 2 | Q14508 | Zq,zl |

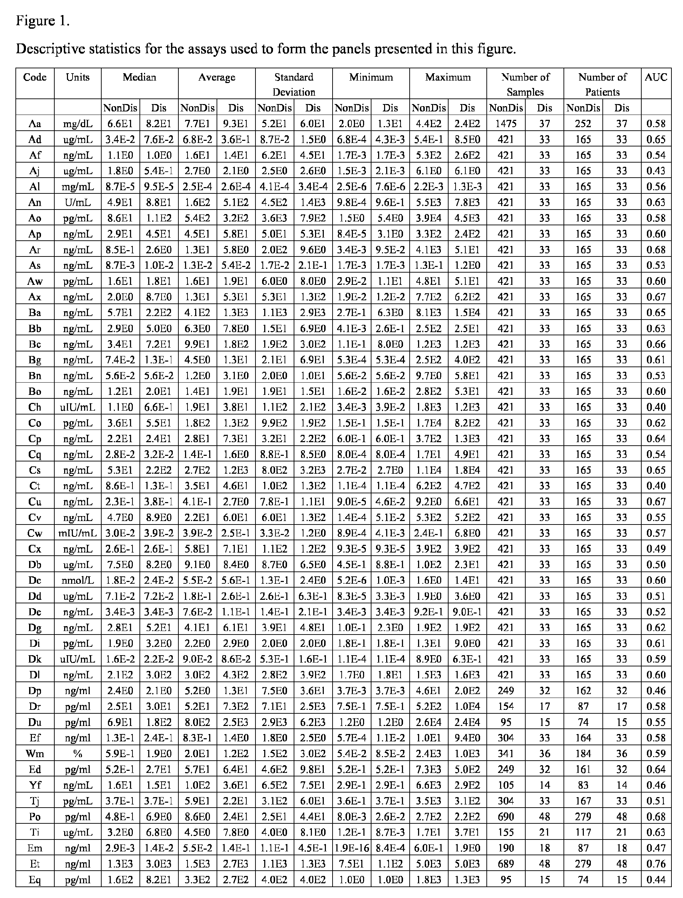

The results of each analysis presented in the following examples are presented in Figs 1-43.

Each figure contains an initial table presenting the univariate statistics for the analytes that appear in the biomarker panels that follows in the same figure. The data set was segregated into two groups, non-diseased (NonDis) and diseased (Dis) patients as indicated in the figure heading. The analyte code and units of measurement are shown, followed by the median, average, standard deviation, maximum and minimum of the analyte values calculated for each group. Also shown are the number of samples that make up each group, and the number of patients that the samples were drawn from. Values below the detectable limit of a particular assay are indicated with the value $1\times10^{-9}$ (written as "1.0E-9"). The univariate AUC for each analyte is also shown. Standard errors were calculated as described in Hanley, J. A., and McNeil, B.J., "The meaning and use of the area under a receiver operating characteristic (ROC) curve," Radiology (1982) 143: 29-36; p values were calculated with a two-tailed Z-test. In the column specifying the units of measurement, certain analytes are listed as '2.6ng/mf, '2.5ng/ml', and '2.3mU/ml'. For these markers, the values in the tables should be scaled by 2.6, 2.5, and 2.3 respectively. By way of example, a value of 1 in the 'median' column converts to a value of 2.6 ng/ml, 2.5 ng/ml, or 2.3 mU/ml, respectively. Note that applying such a scaling factor to the data does not impact the AUC of a marker or the AUC of a panel of which it is a part.

Following the univariate statistics, each figure provides panel tables which list the biomarker panels having model p-value in the ranges specified in the particular figures. When a panel is formed by combining multiple analytes from this set, the number of samples used was the intersection of the samples measured by all analytes on the particular panel. Panels that have an intersection of less than 7 samples in either the non-diseased or diseased groups were not considered in the analysis. For compactness panels are encoded as follows: aBCdEE represents the two panels aBCdEe and aBCdeE, in this case representing biomarker "ab" measured in plasma, biomarker "cd" measured in urine, and biomarker ee measured in urine for the first panel and ee measured in plasma for the second panel. Codes in () or{} and separated by a space represent a list of analytes that join with the analyte(s) outside the brackets to form panels with a common analyte. For example, Ab{cD(eF gH) Km(EfQR)} represents 5 panels which all have biomarker Ab in common. They are AbcDeF, AbcDgH, AbKmEf, AbKmQr, and AbKmqR.

Those results labeled 'Unconstrained Panels' refer to panels which were not required to have each analyte univariate p-value $\leq 0.05$. Those results labeled 'Constrained Panels' require that each analyte use in the panel to have a univariate p-value $\leq 0.05$.

Example 7. Use of Biomarker Panels

Patients from the intensive care unit (ICU) were classified by kidney status as non-injury (0), risk of injury (R), injury (I), and failure (F) according to the maximum RIFLE stage reached as determined by the RIFLE criteria. Two cohorts were defined as (Cohort 1 - "Non-diseased") patients that did not progress beyond a particular RIFLE stage, and (Cohort 2 - "Diseased") patients that reached a later RIFLE stage within 10 days. Marker concentrations were measured in samples collected from a subject at 0, 24 hours, and 48 hours prior to reaching the "Diseased" stage.

Each biomarker was measured by standard immunoassay methods using commercially available assay reagents. In the case of those biomarkers which are membrane-associated, assays which recognize soluble forms were used. As noted above, the membrane-associated biomarkers include Swiss-Prot entries 014788, 014944, 075309, P00797, P05186, P08473, P13688, P15514, P22223, P27487, P35070, Q03405, Q14956, Q16790, Q99075, Q9Y5Y7Q15109, Q02763, P17213, P12830, P33151, P06731, P29965, P16070, Q9H2A7, P17813, Q9UNN8, P00533, P16422, P19235, P16581, P78423, 043656, P08581, P08069, P05362, P13598, P32942, P14778, P27930, P01589, P24394, P08887, P40189, P21583, P09603, P08571, Q8WXI7, P13591, Q92823, P78380, P16284, P01133, P15309, P01135, P16109, Q14242, P49771, P07204, P13726, P01375, P50591, P48023, 014763, P19438, P20333, P25942, P25445, P28908, P19320, P17948, and P35968.

The ability of a biomarker panel to distinguish "Diseased" from "Non-Diseased" was determined as described above in Example 6. Patients in Cohort 2 were also separated according to the reason for adjudication to the "Diseased" stage as being based on serum creatinine measurements (sCr), being based on urine output (UO), or being based on either serum creatinine measurements or urine output. As an example, for those patients adjudicated to stage F on the basis of serum creatinine measurements alone, the stage 0 cohort may have included patients adjudicated to stage F on the basis of urine output; for those patients adjudicated to stage F on the basis of urine output alone, the stage 0 cohort may have included patients adjudicated to stage F on the basis of serum creatinine measurements; and for those patients adjudicated to stage F on the basis of serum creatinine measurements or urine output, the stage 0 cohort contains only patients in stage 0 for both serum creatinine measurements and urine output. Also, for those patients adjudicated to stage F on the basis of serum creatinine measurements or urine output, the adjudication method which yielded the most severe RIFLE stage was used.

The following data are presented in the figures:

Figure 1. No or R RIFLE stage versus RIFLE I or F. RIFLE stage adjudicated by sCr and Urine Output. Diseased group sample is 24 hours prior to RIFLE I diagnosis.

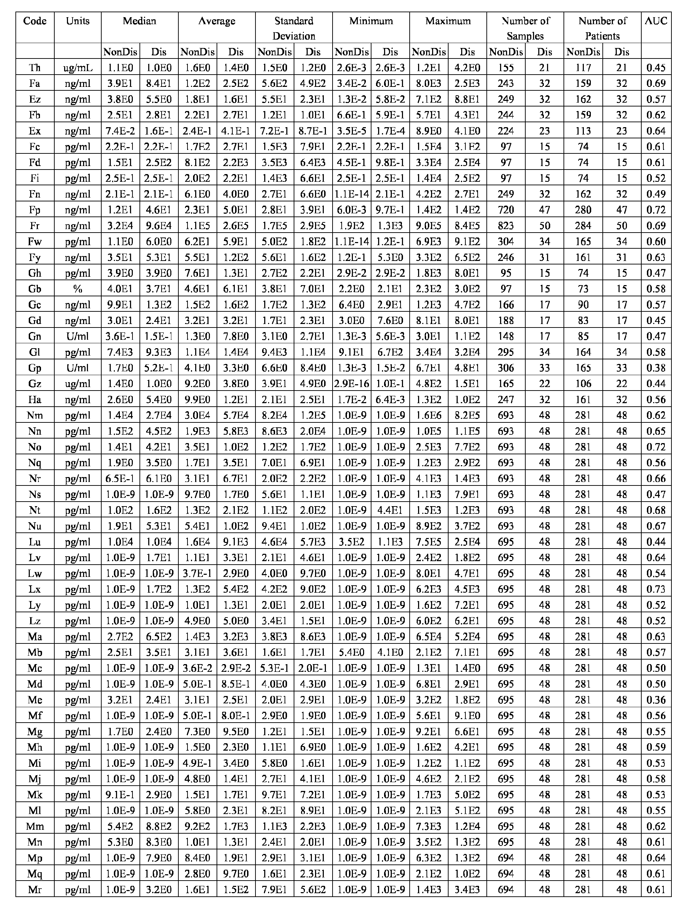

Figure 2. No or R RIFLE stage versus RIFLE I or F. RIFLE stage adjudicated by sCr. Diseased group sample is 24 hours prior to RIFLE I diagnosis.

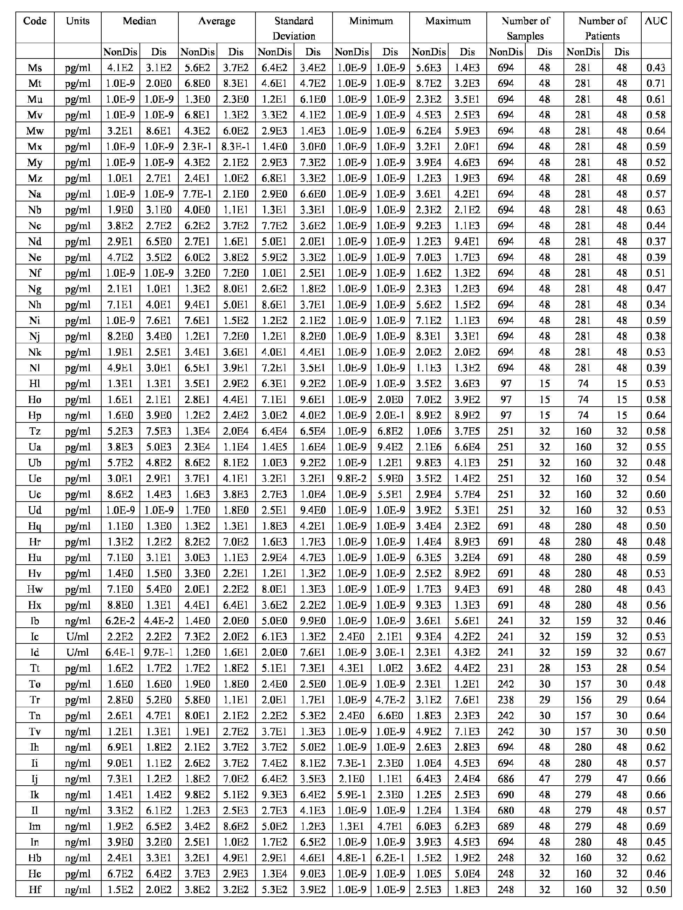

Figure 3. No or R RIFLE stage versus RIFLE I or F. RIFLE stage adjudicated by Urine Output. Diseased group sample is 24 hours prior to RIFLE I diagnosis.

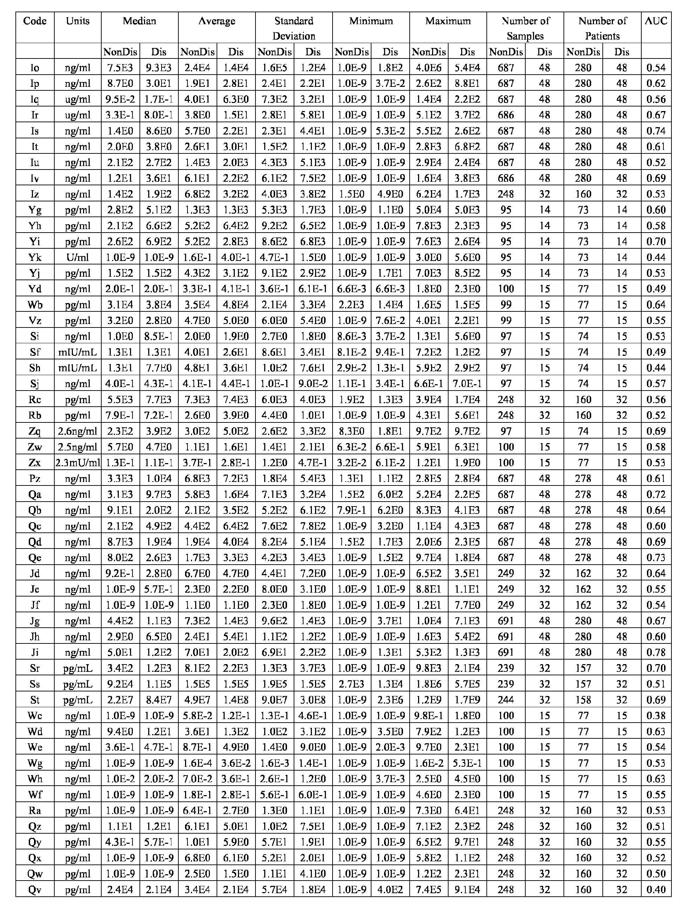

Figure 4. No RIFLE stage versus RIFLE R, I, or F. RIFLE stage adjudicated by sCr and Urine Output. Diseased group sample is 24 hours prior to RIFLE R diagnosis.

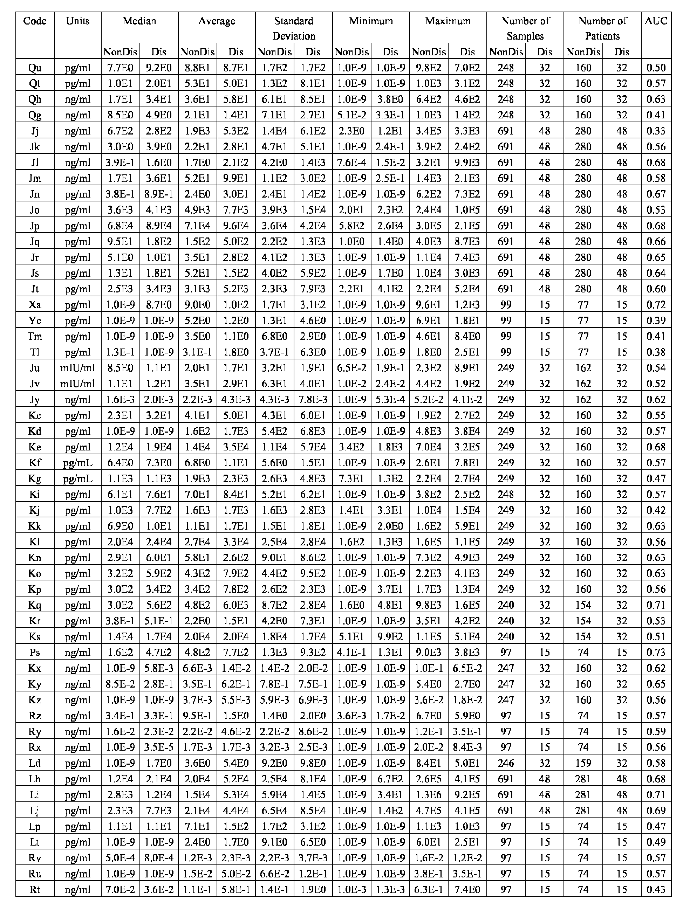

Figure 5. No RIFLE stage versus RIFLE R, I, or F. RIFLE stage adjudicated by sCr. Diseased group sample is 24 hours prior to RIFLE R diagnosis.

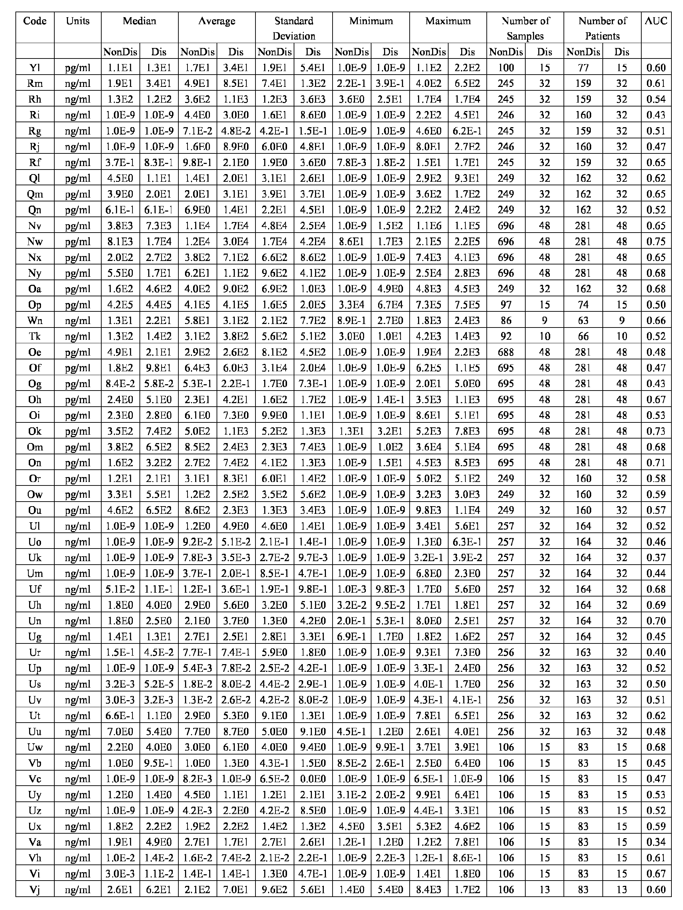

Figure 6. No RIFLE stage versus RIFLE R, I, or F. RIFLE stage adjudicated by Urine Output. Diseased group sample is 24 hours prior to RIFLE R diagnosis.

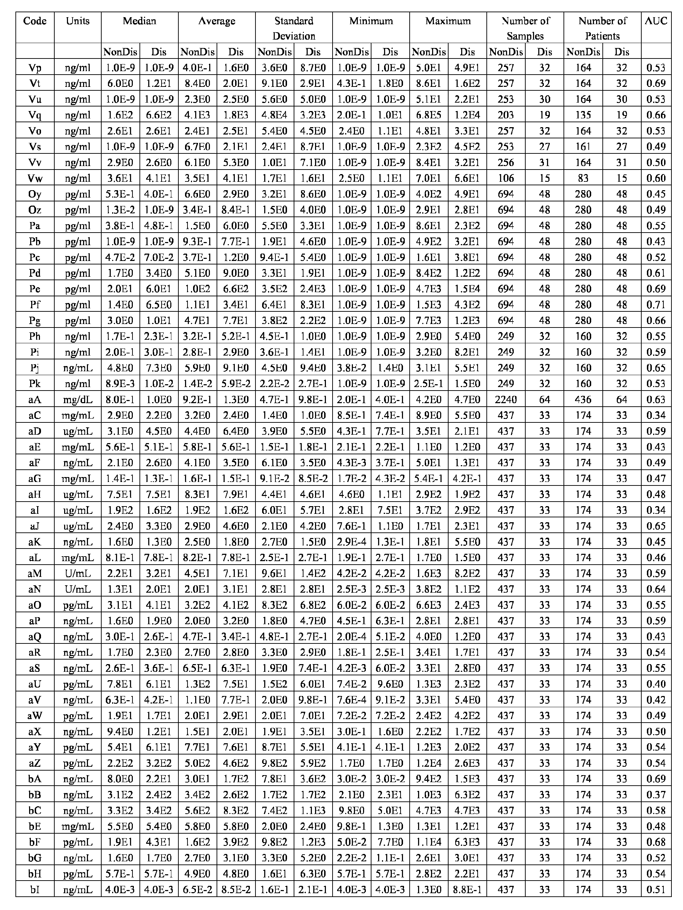

Figure 7. No, R, or I RIFLE stage versus RIFLE F. RIFLE stage adjudicated by sCr and Urine Output. Diseased group sample is 24 hours prior to RIFLE I diagnosis.

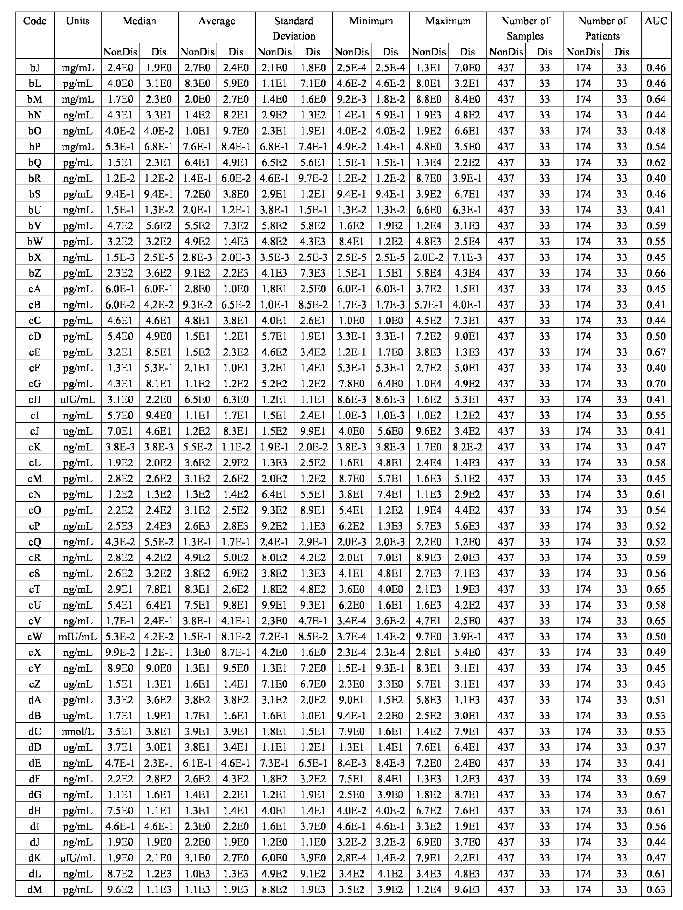

Figure 8. No, R, or I RIFLE stage versus RIFLE F. RIFLE stage adjudicated by Urine Output. Diseased group sample is 24 hours prior to RIFLE I diagnosis.

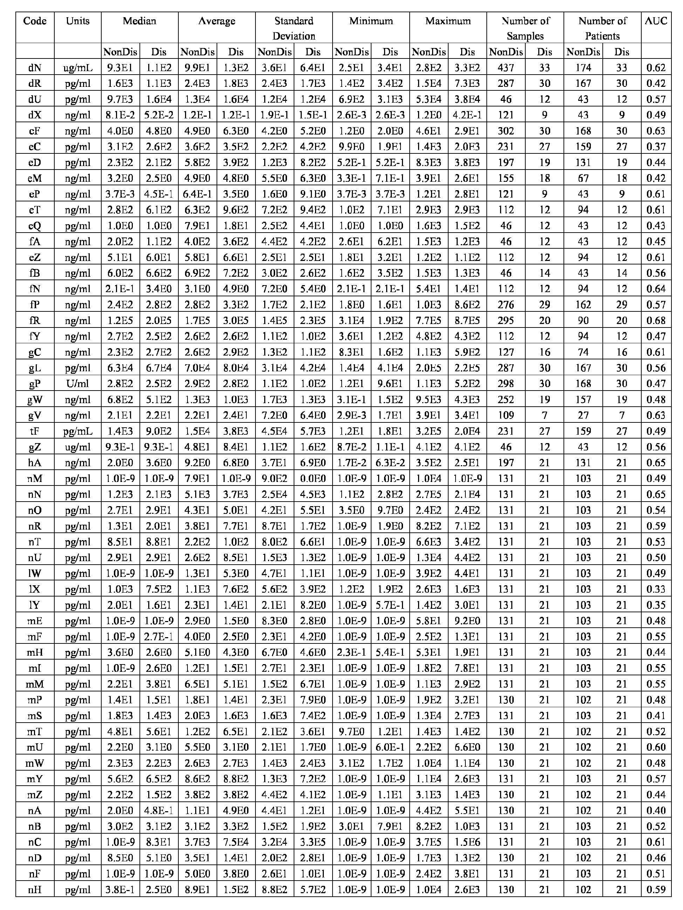

Figure 9. No or R RIFLE stage versus RIFLE I or F. RIFLE stage adjudicated by sCr and Urine Output. Diseased group sample is at RIFLE I diagnosis.

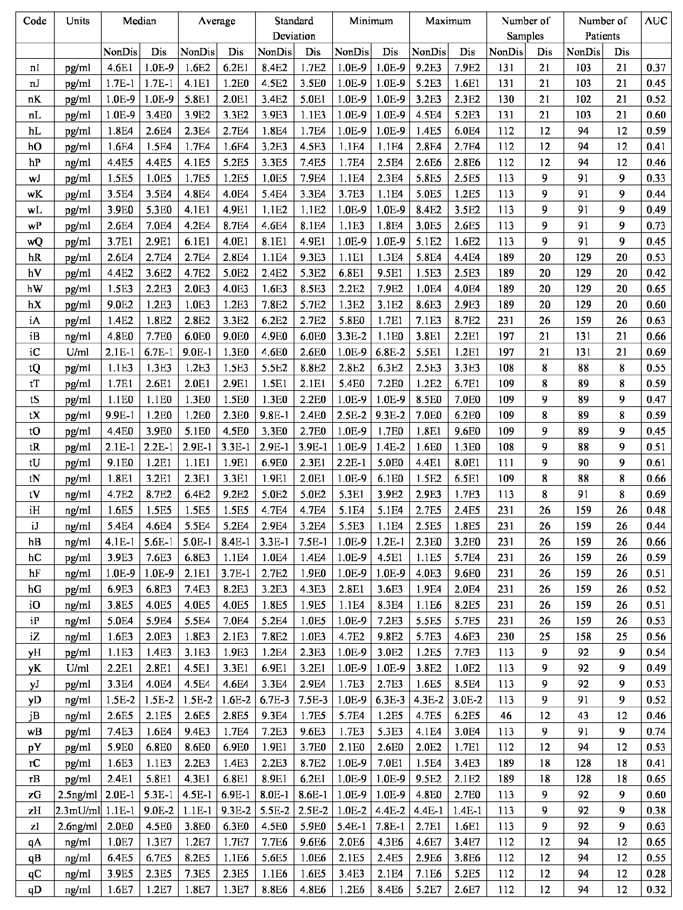

Figure 10. No or R RIFLE stage versus RIFLE I or F. RIFLE stage adjudicated by sCr. Diseased group sample is at RIFLE I diagnosis.

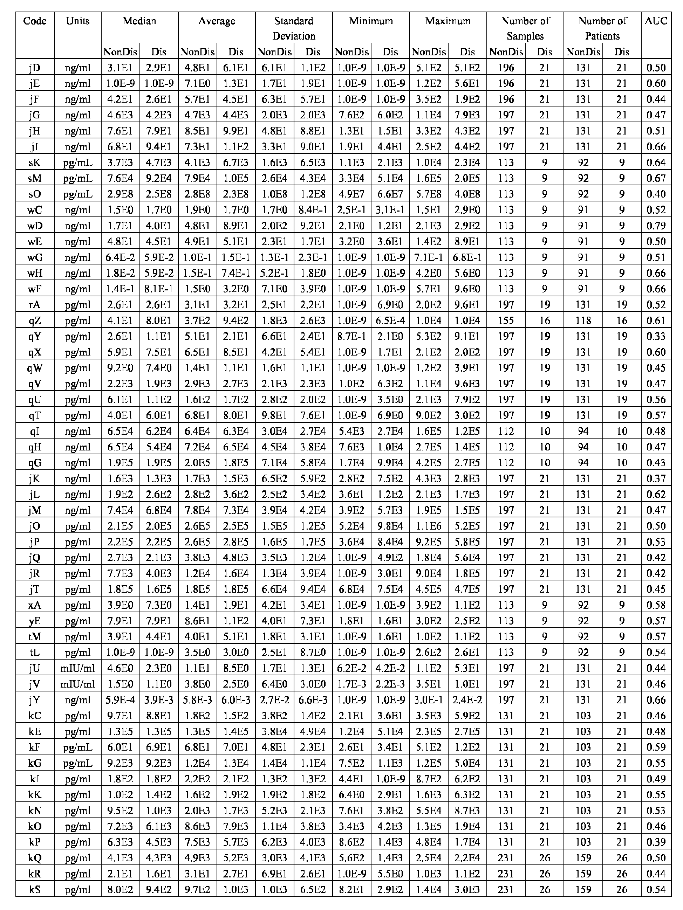

Figure 11. No or R RIFLE stage versus RIFLE I or F. RIFLE stage adjudicated by Urine Output. Diseased group sample is at RIFLE I diagnosis.

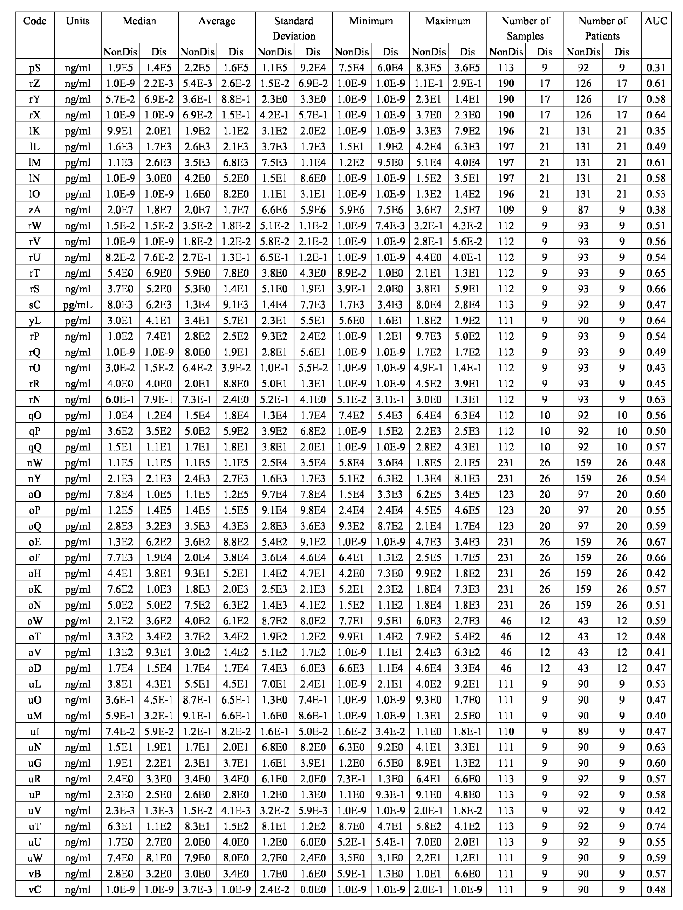

Figure 12. No RIFLE stage versus RIFLE R, I, or F. RIFLE stage adjudicated by sCr and Urine Output. Diseased group sample is at RIFLE R diagnosis.

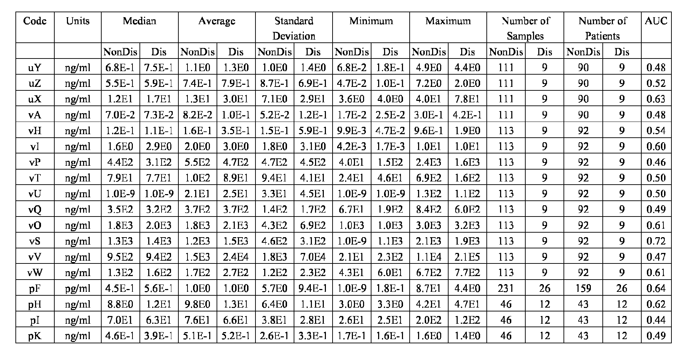

Figure 13. No RIFLE stage versus RIFLE R, I, or F. RIFLE stage adjudicated by sCr. Diseased group sample is at RIFLE R diagnosis.

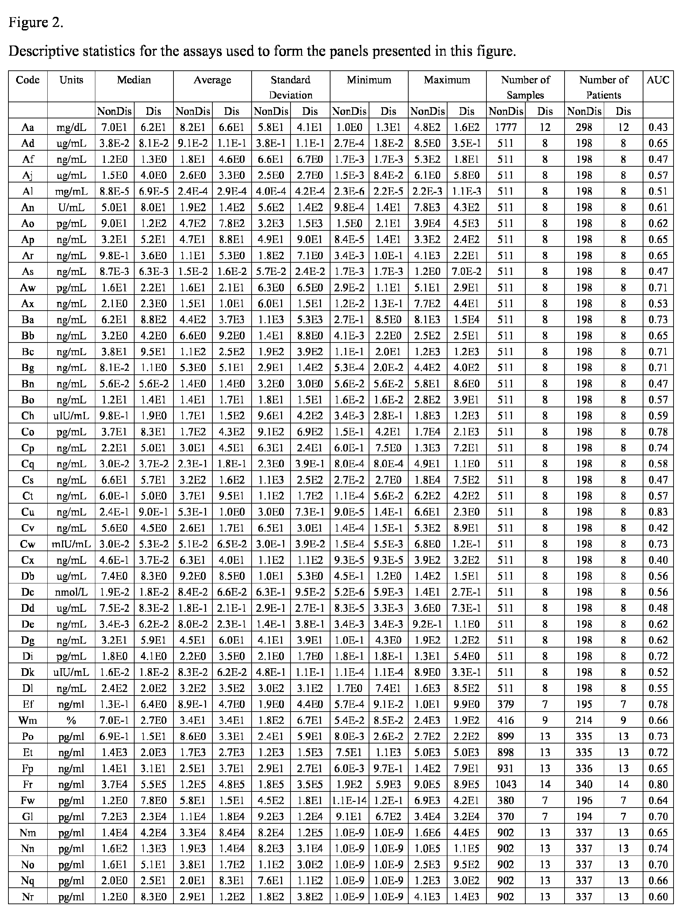

Figure 14. No RIFLE stage versus RIFLE R, I, or F. RIFLE stage adjudicated by Urine Output. Diseased group sample is at RIFLE R diagnosis.

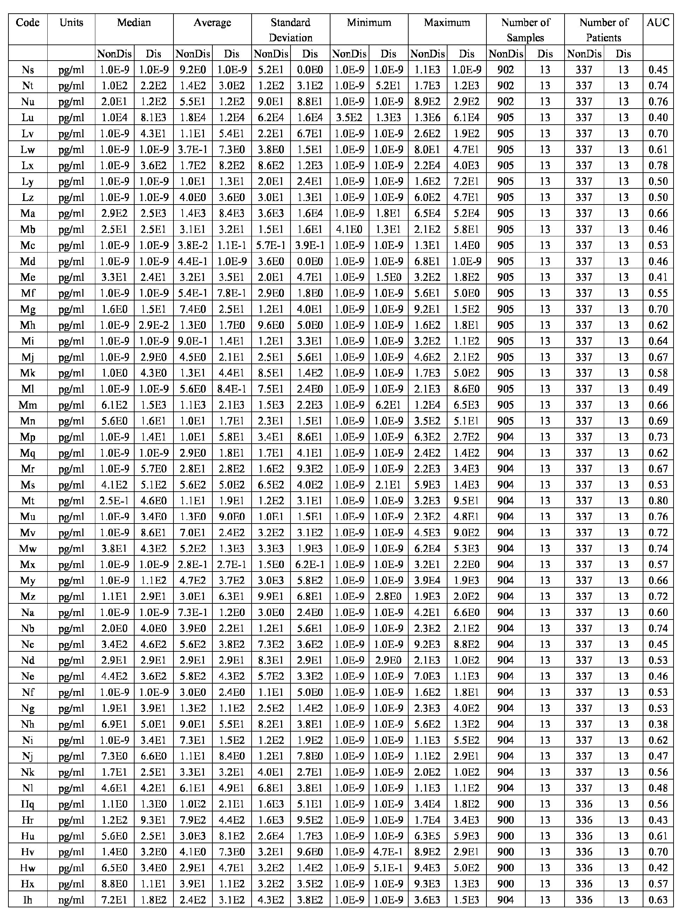

Figure 15. No, R, or I RIFLE stage versus RIFLE F. RIFLE stage adjudicated by sCr and Urine Output. Diseased group sample is at RIFLE I diagnosis.

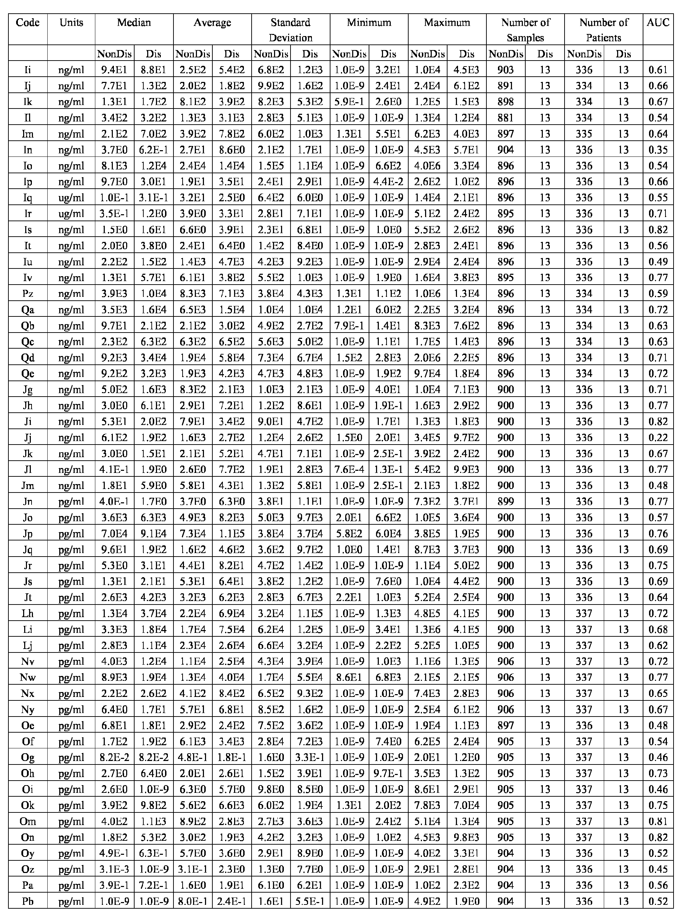

Figure 16. No, R, or I RIFLE stage versus RIFLE F. RIFLE stage adjudicated by sCr. Diseased group sample is at RIFLE I diagnosis.

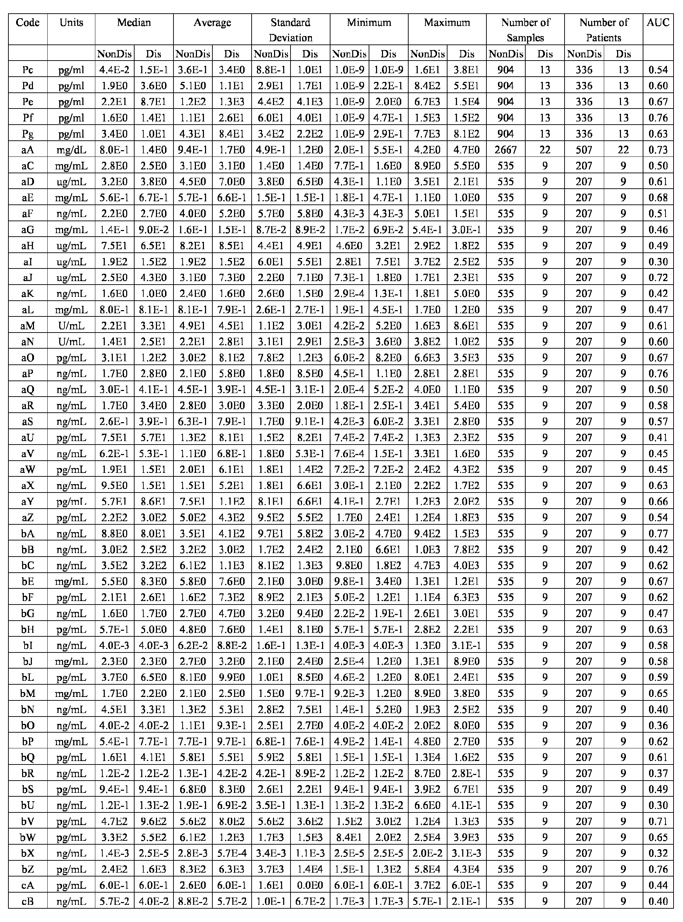

Figure 17. No, R, or I RIFLE stage versus RIFLE F. RIFLE stage adjudicated by Urine Output. Diseased group sample is at RIFLE I diagnosis.

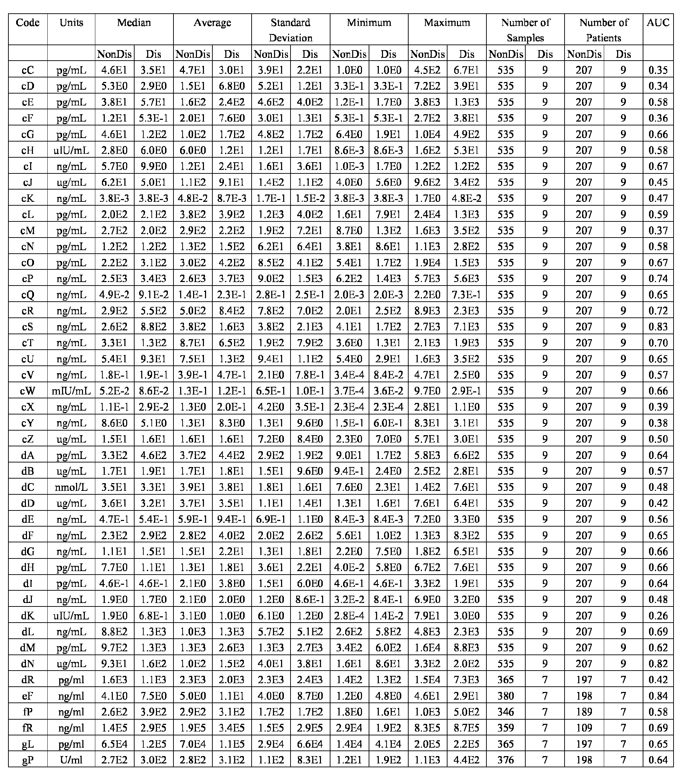

Figure 18. No or R RIFLE stage versus RIFLE I or F. RIFLE stage adjudicated by sCr and Urine Output. Diseased group sample is 48 hours prior to RIFLE I diagnosis.

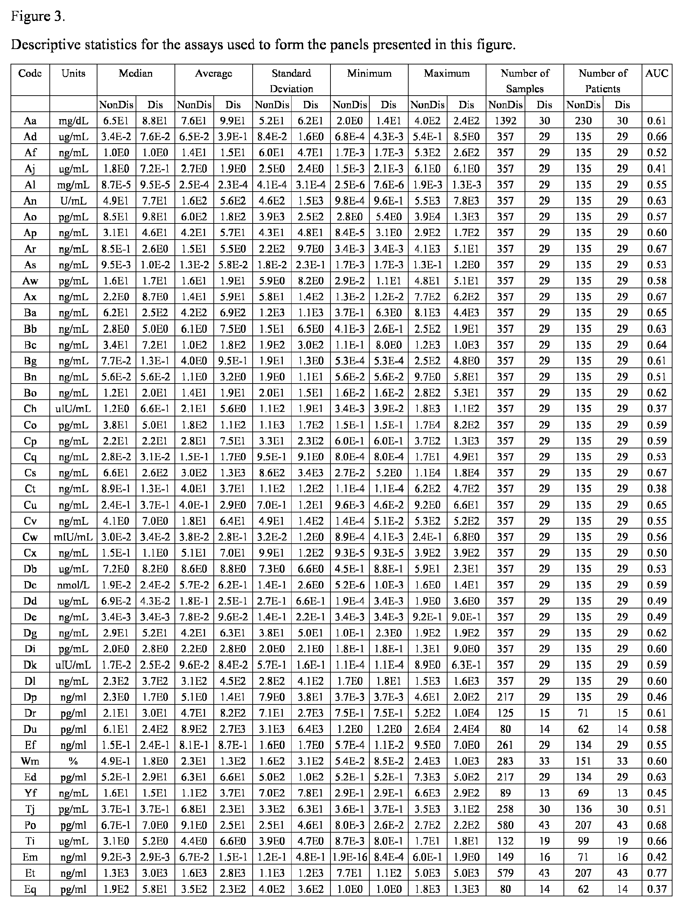

Figure 19. No or R RIFLE stage versus RIFLE I or F. RIFLE stage adjudicated by sCr. Diseased group sample is 48 hours prior to RIFLE I diagnosis.

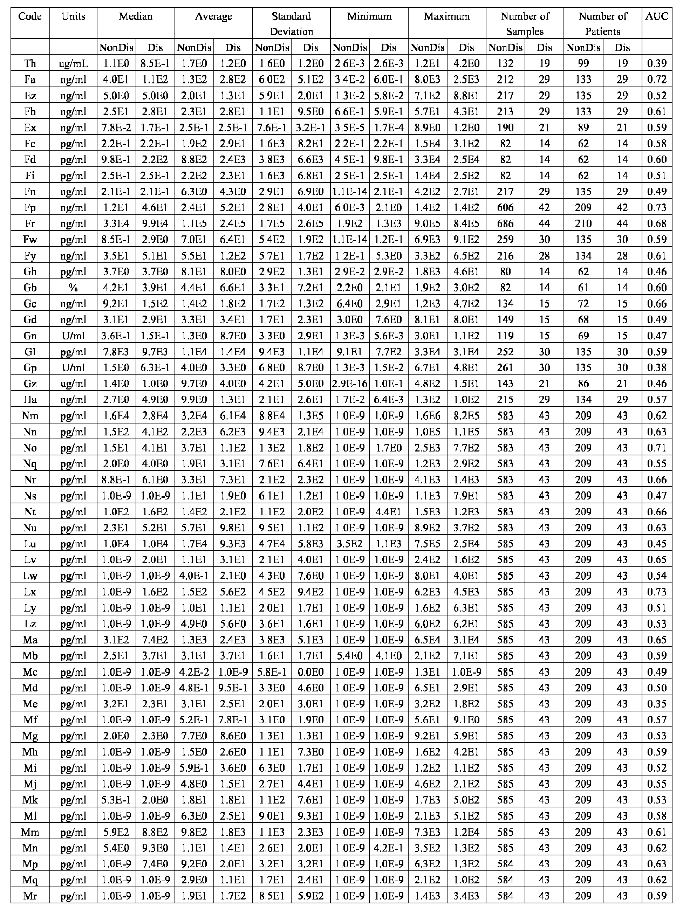

Figure 20. No or R RIFLE stage versus RIFLE I or F. RIFLE stage adjudicated by Urine Output. Diseased group sample is 48 hours prior to RIFLE I diagnosis.

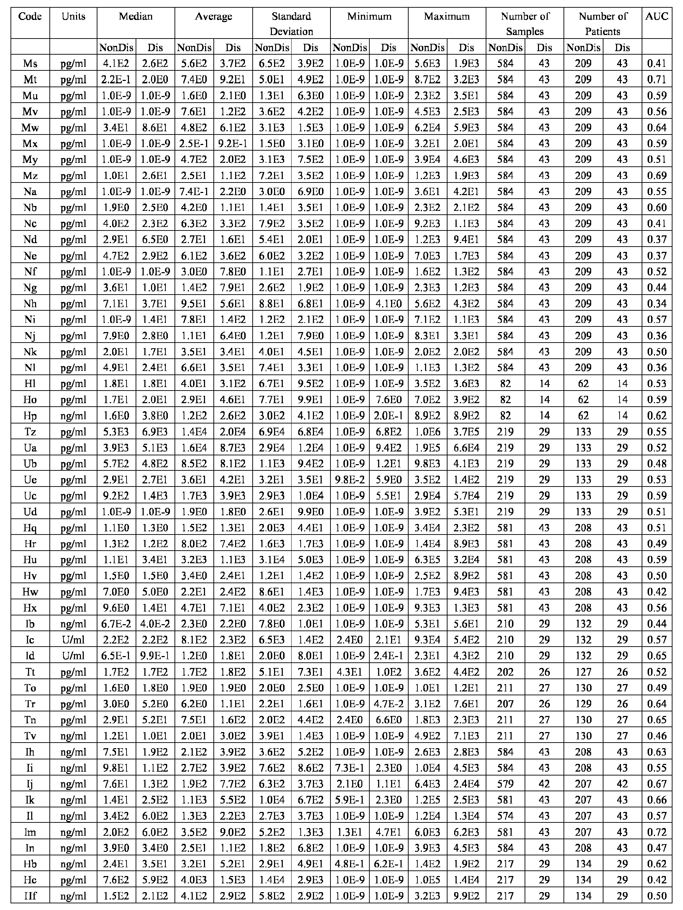

Figure 21. No RIFLE stage versus RIFLE R, I, or F. RIFLE stage adjudicated by sCr and Urine Output. Diseased group sample is 48 hours prior to RIFLE R diagnosis.

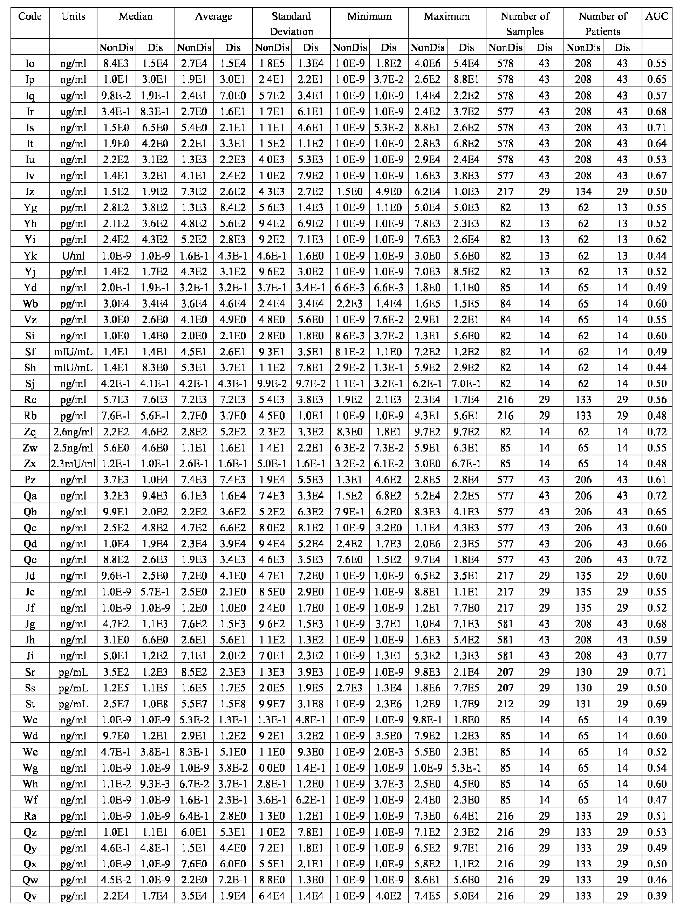

Figure 22. No RIFLE stage versus RIFLE R, I, or F. RIFLE stage adjudicated by sCr. Diseased group sample is 48 hours prior to RIFLE R diagnosis.

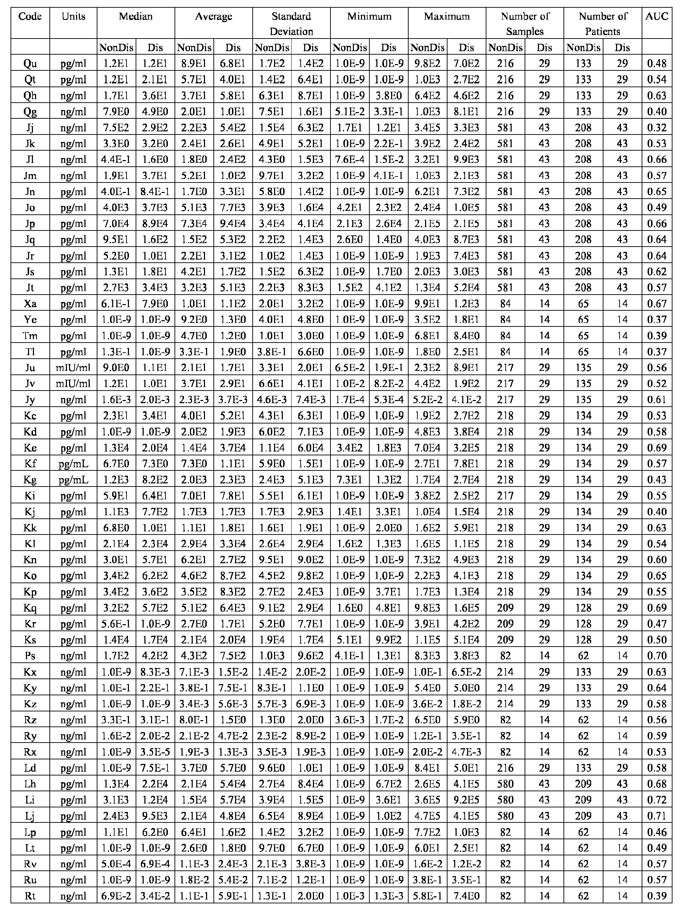

Figure 23. No RIFLE stage versus RIFLE R, I, or F. RIFLE stage adjudicated by Urine Output. Diseased group sample is 48 hours prior to RIFLE R diagnosis.

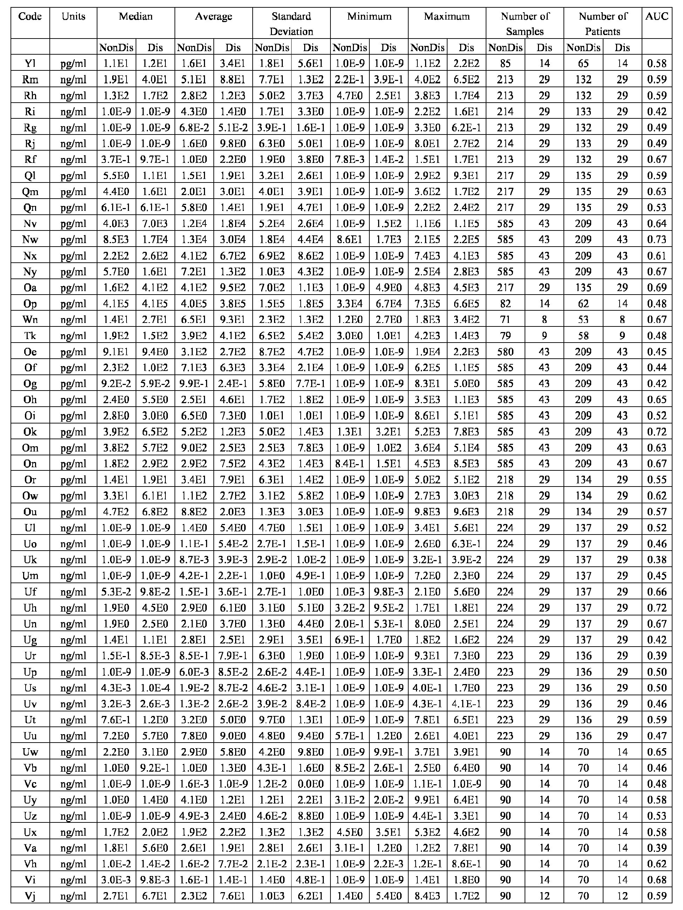

Figure 24. No, R, or I RIFLE stage versus RIFLE F. RIFLE stage adjudicated by sCr and Urine Output. Diseased group sample is 48 hours prior to RIFLE I diagnosis.

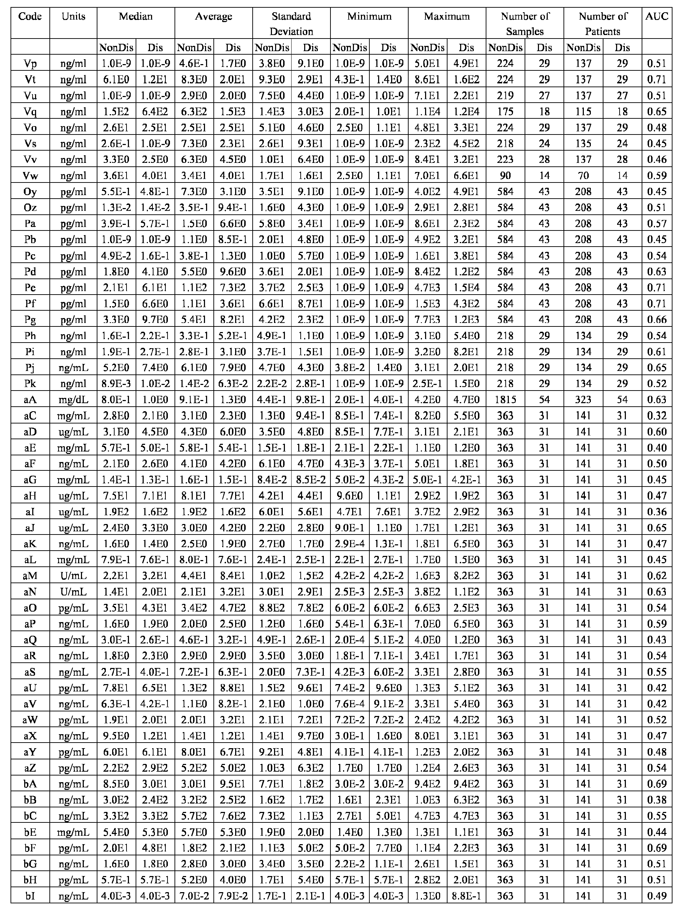

Figure 25. Progression of RIFLE R to RIFLE I or F. RIFLE stage adjudicated by sCr and Urine Output. Sample is at R diagnosis.

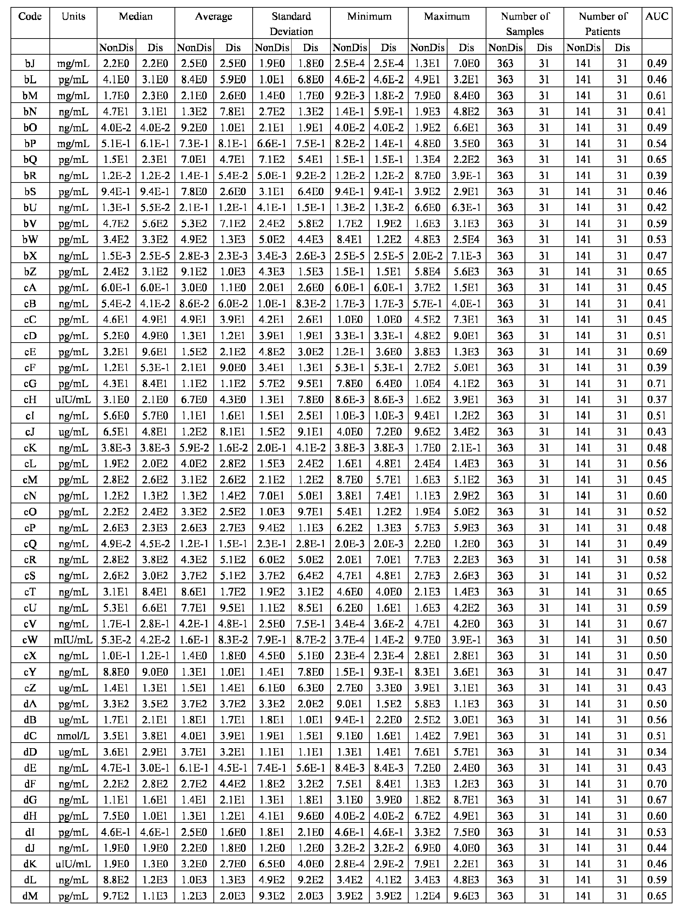

Figure 26. Progression of RIFLE R to RIFLE I or F. RIFLE stage adjudicated by sCr. Sample is at R diagnosis.

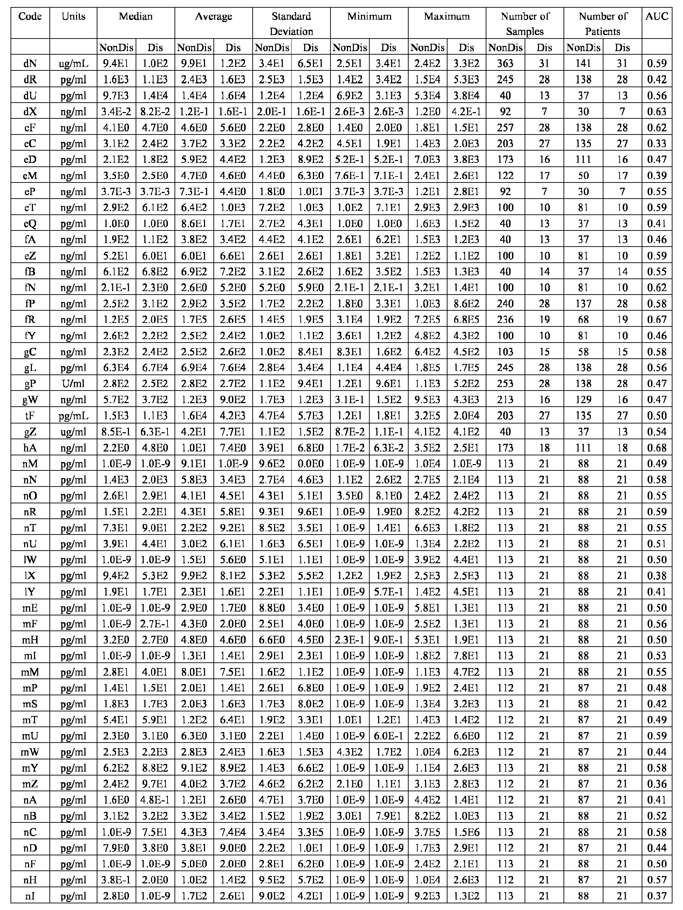

Figure 27. Progression of RIFLE R to RIFLE I or F. RIFLE stage adjudicated by Urine Output. Sample is at R diagnosis.

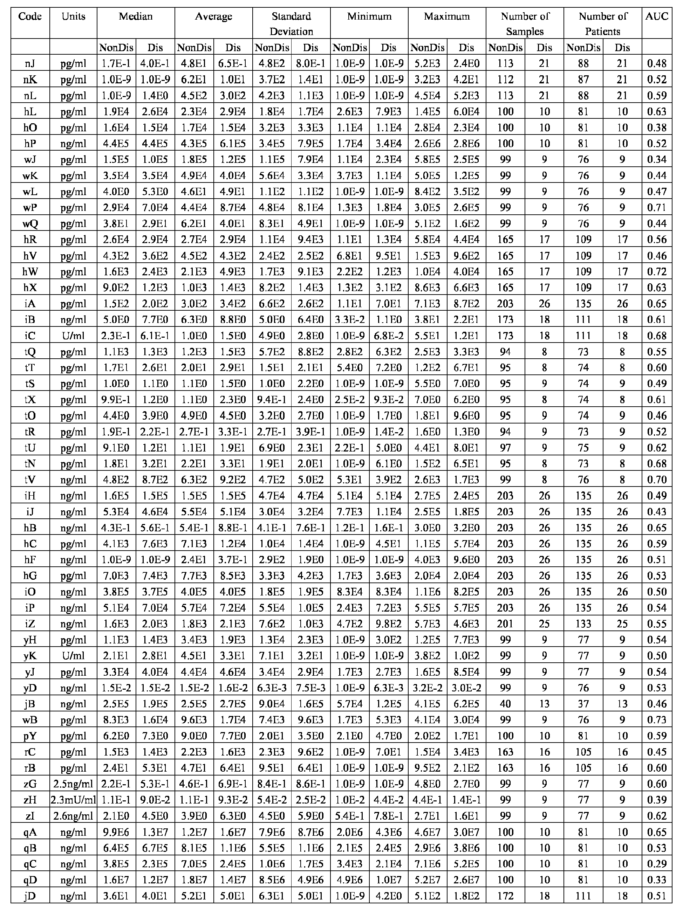

Figure 28. No or R RIFLE stage versus RIFLE I or F. RIFLE stage adjudicated by sCr and Urine Output, within 48hrs of enrollment. Sample is at enrollment.

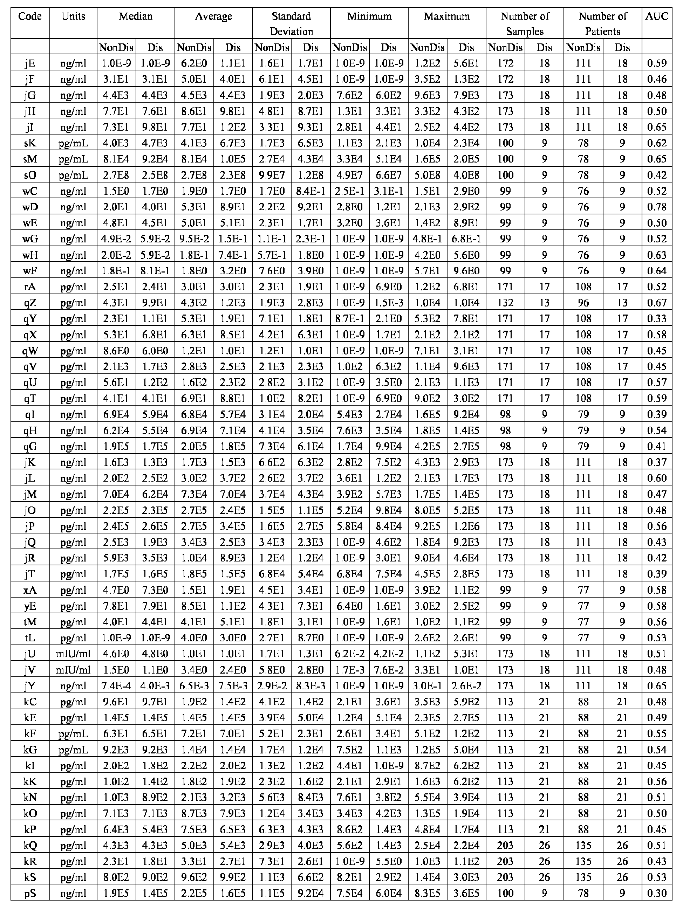

Figure 29. No or R RIFLE stage versus RIFLE I or F. RIFLE stage adjudicated by sCr, within 48hrs of enrollment. Sample is at enrollment.

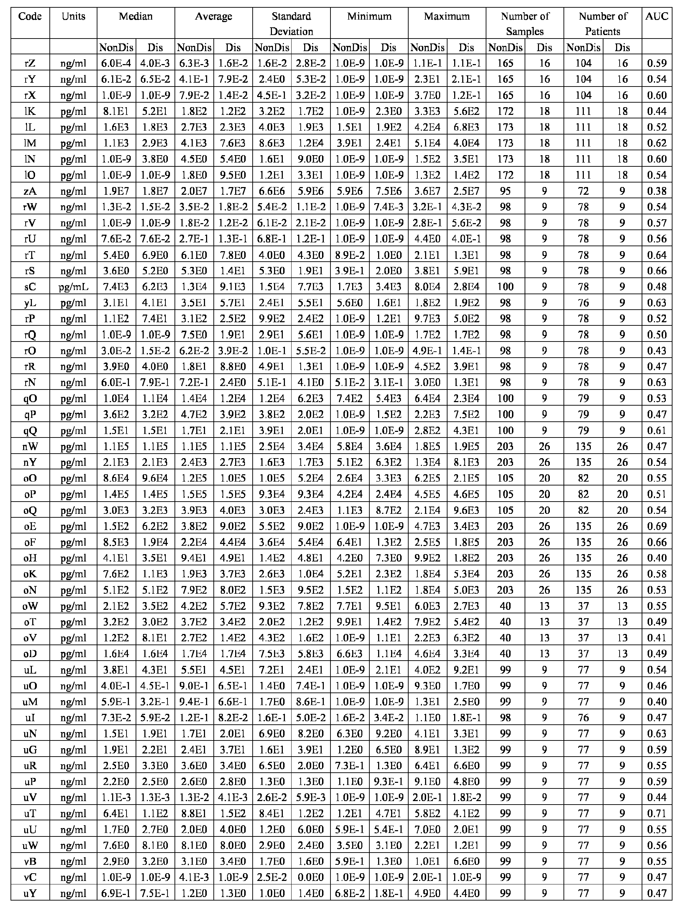

Figure 30. No or R RIFLE stage versus RIFLE I or F. RIFLE stage adjudicated by Urine Output, within 48hrs of enrollment. Sample is at enrollment.

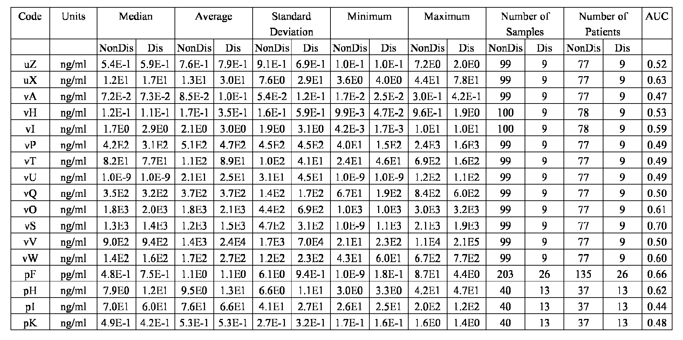

Figure 31. No RIFLE stage versus RIFLE R, I, or F. RIFLE stage adjudicated by sCr and Urine Output, within 48hrs of enrollment. Sample is at enrollment.

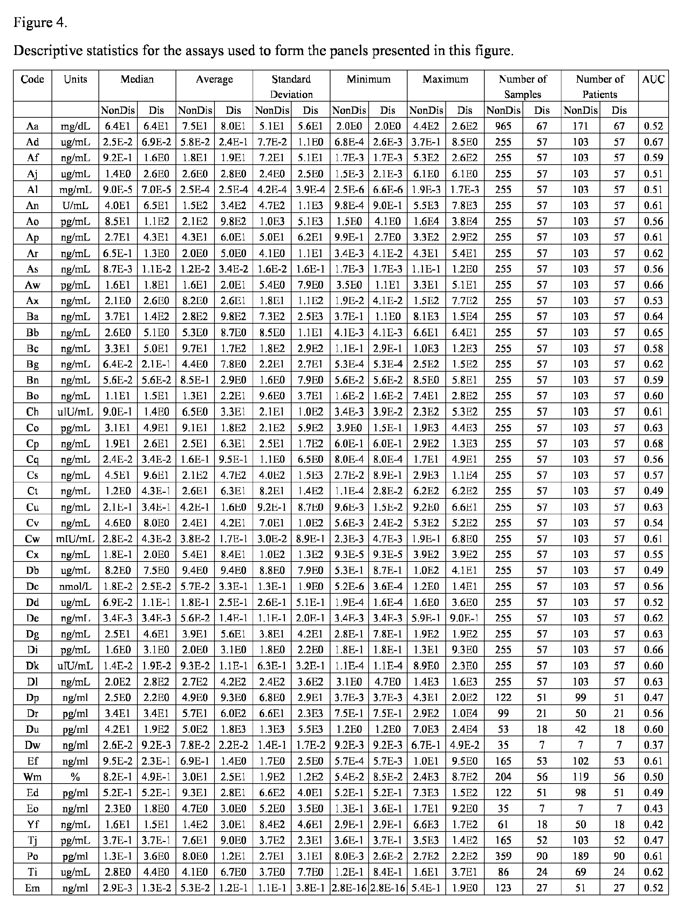

Figure 32. No RIFLE stage versus RIFLE R, I, or F. RIFLE stage adjudicated by sCr, within 48hrs of enrollment. Sample is at enrollment.

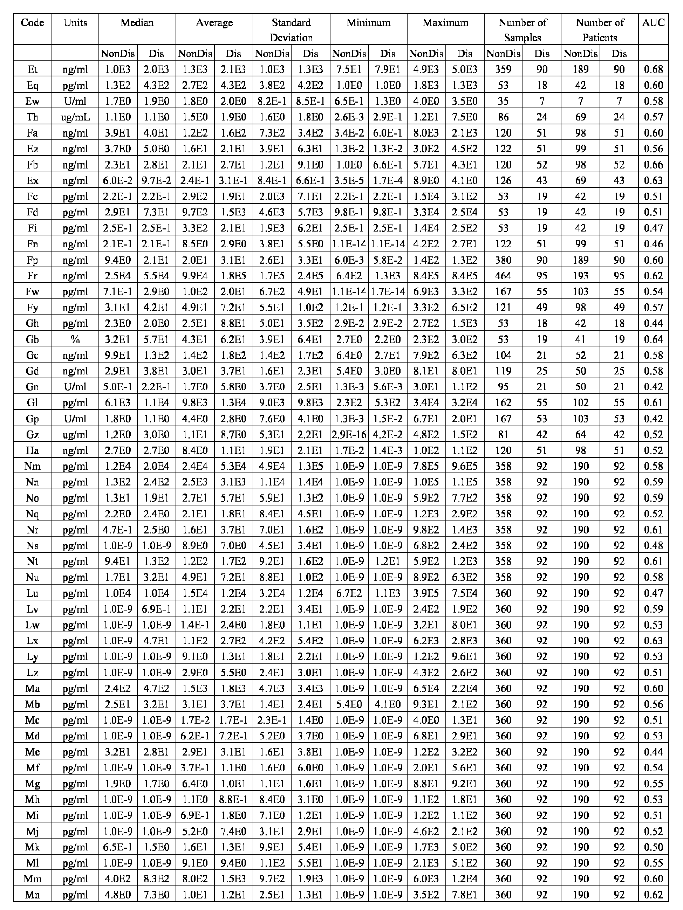

Figure 33. No RIFLE stage versus RIFLE R, I, or F. RIFLE stage adjudicated by Urine Output, within 48hrs of enrollment. Sample is at enrollment.

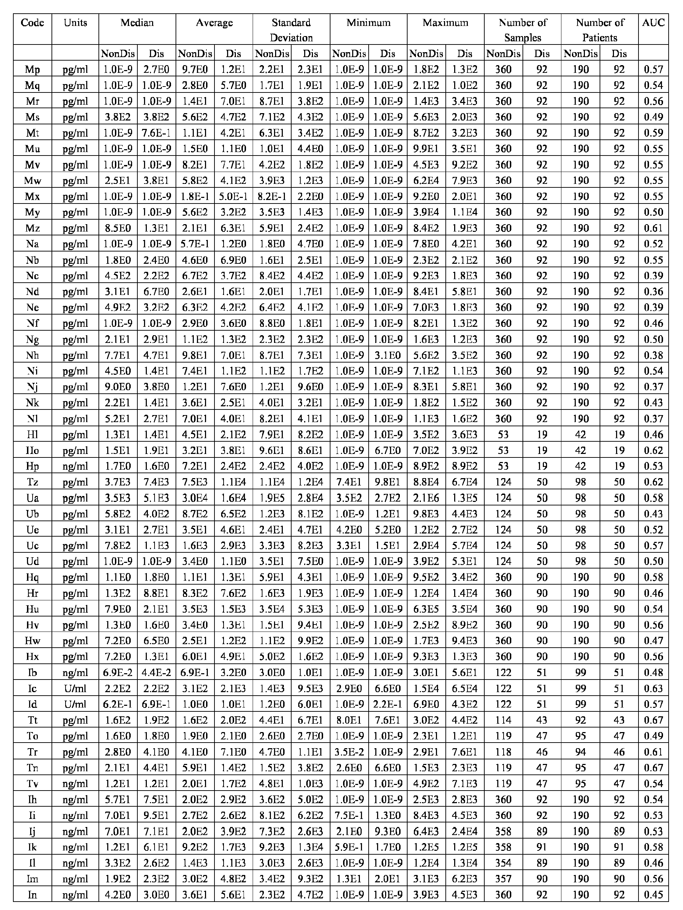

Figure 34. No, R, or I RIFLE stage versus RIFLE F. RIFLE stage adjudicated by sCr and Urine Output, within 48hrs of enrollment. Sample is at enrollment.

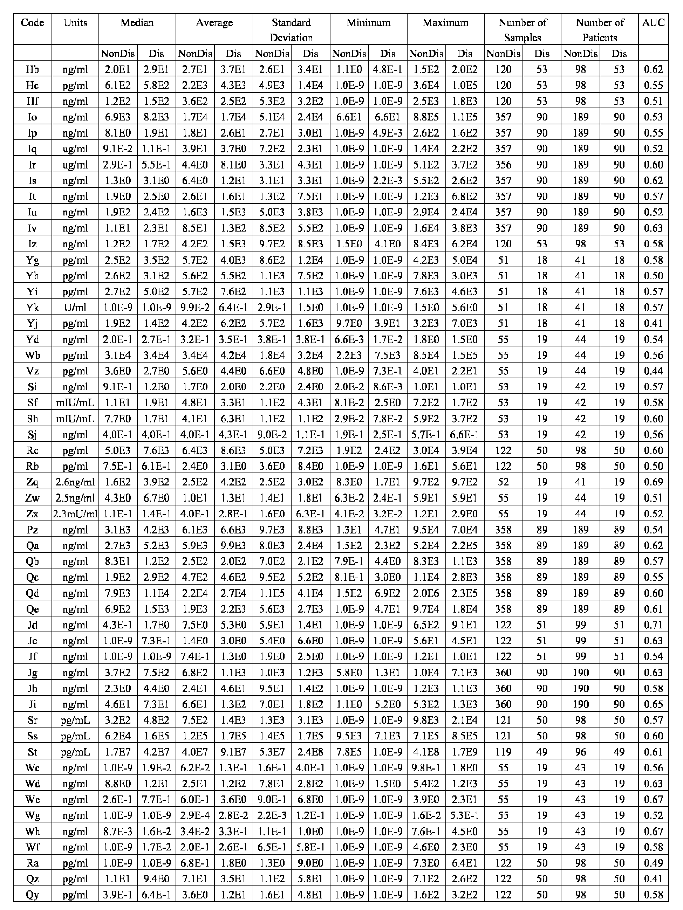

Figure 35. No, R, or I RIFLE stage versus RIFLE F. RIFLE stage adjudicated by Urine Output, within 48hrs of enrollment. Sample is at enrollment.

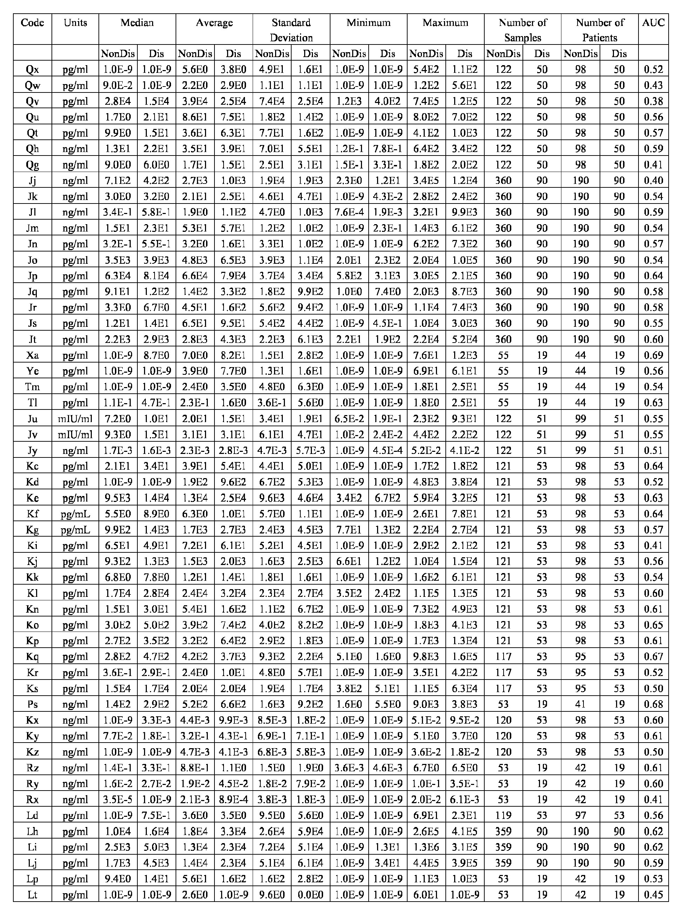

Figure 36. No or R RIFLE stage versus RIFLE I or F. RIFLE stage adjudicated by sCr and Urine Output, within 24hrs of enrollment. Sample is at enrollment.

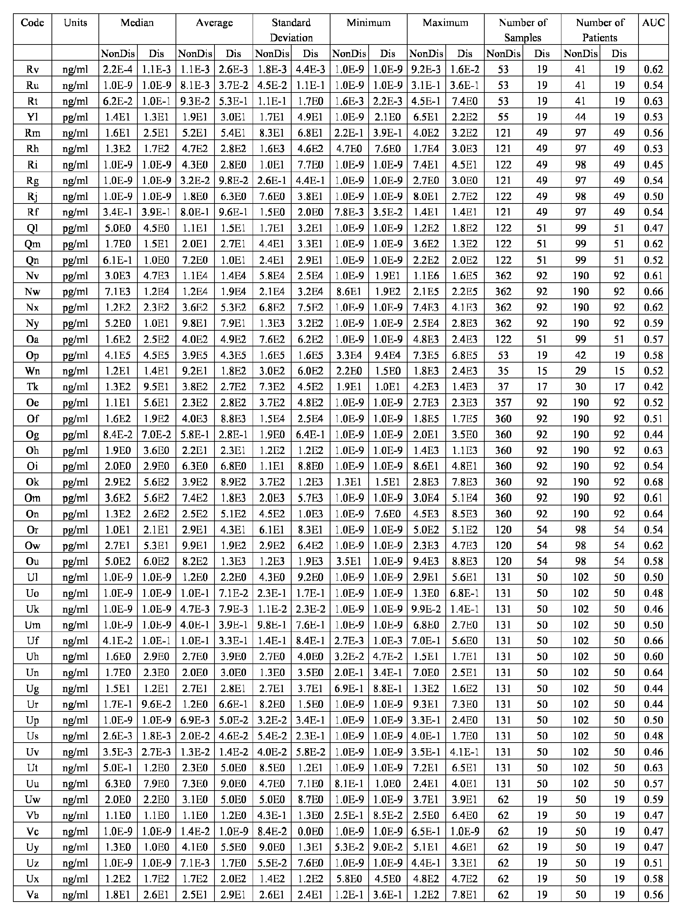

Figure 37. No or R RIFLE stage versus RIFLE I or F. RIFLE stage adjudicated by sCr, within 24hrs of enrollment. Sample is at enrollment.

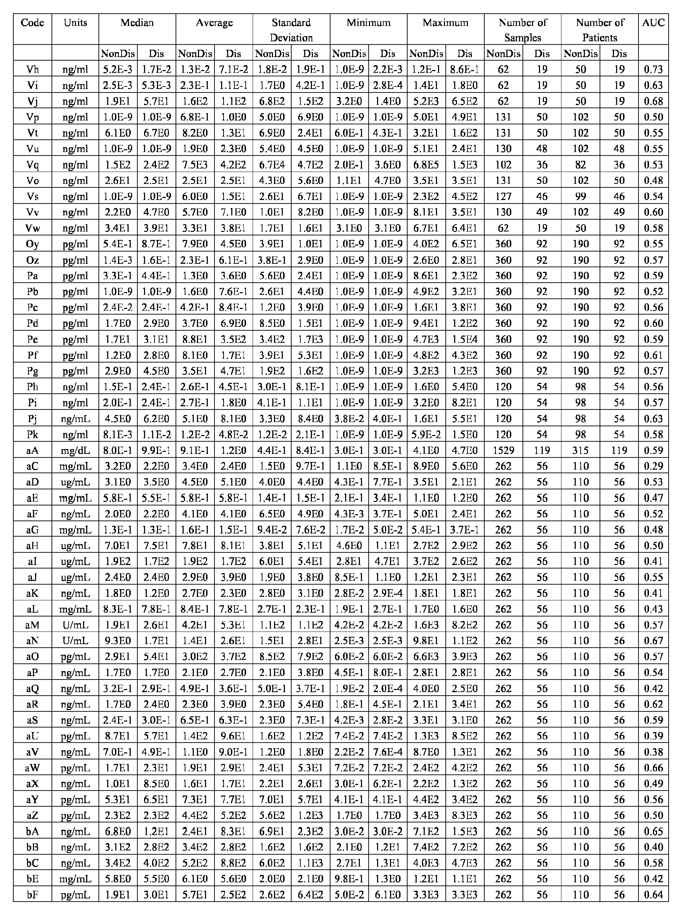

Figure 38. No or R RIFLE stage versus RIFLE I or F. RIFLE stage adjudicated by Urine Output, within 24hrs of enrollment. Sample is at enrollment.

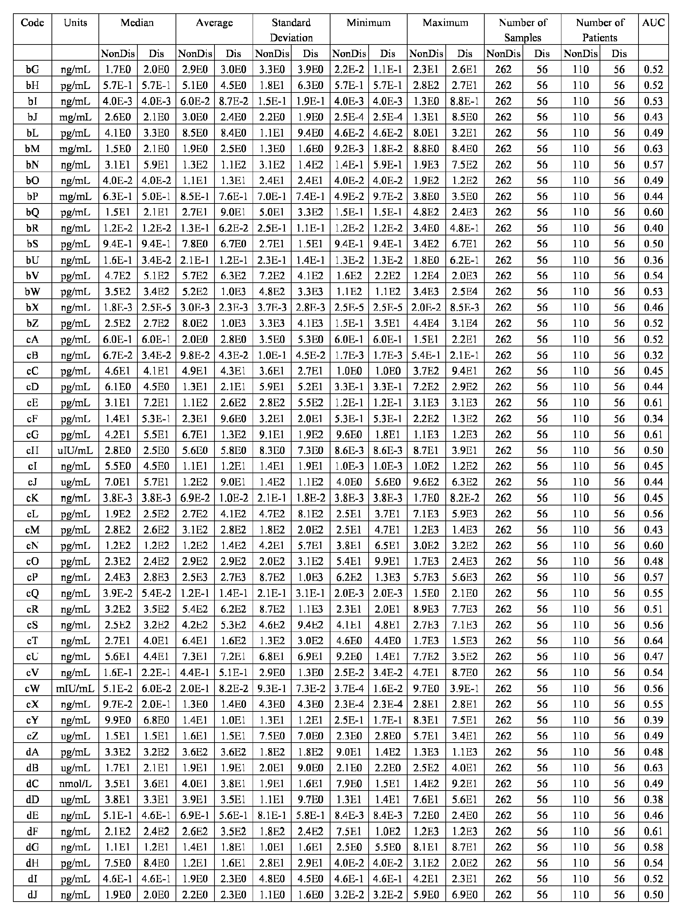

Figure 39. No RIFLE stage versus RIFLE R, I, or F. RIFLE stage adjudicated by sCr and Urine Output, within 24hrs of enrollment. Sample is at enrollment.

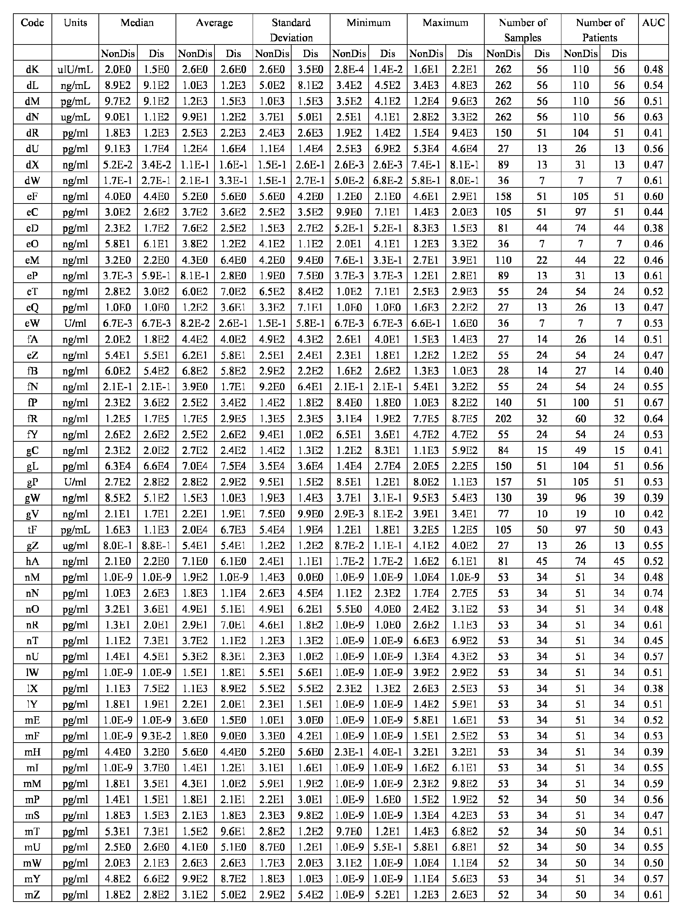

Figure 40. No RIFLE stage versus RIFLE R, I, or F. RIFLE stage adjudicated by sCr, within 24hrs of enrollment. Sample is at enrollment.

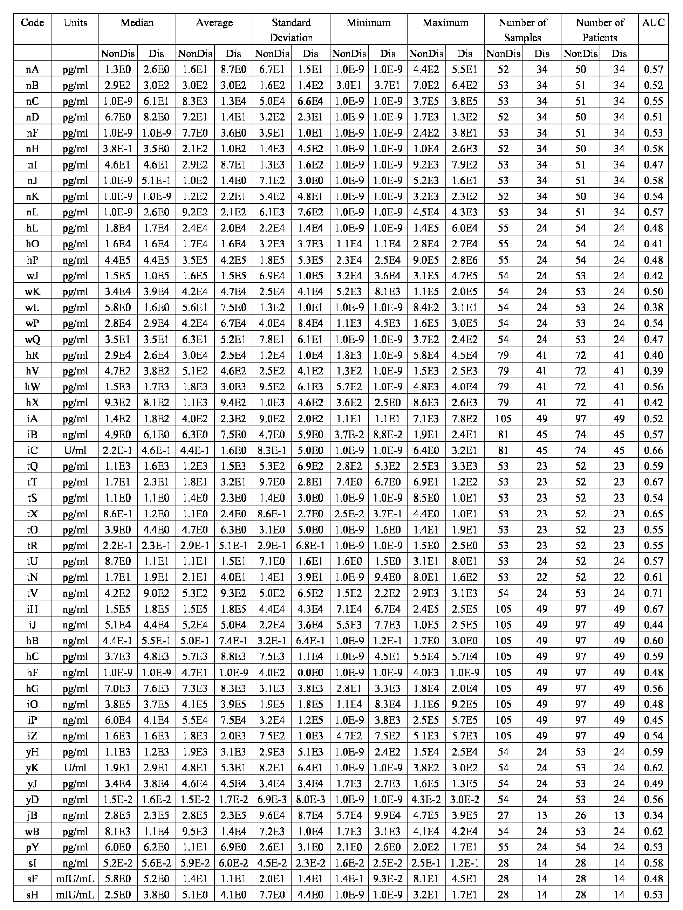

Figure 41. No RIFLE stage versus RIFLE R, I, or F. RIFLE stage adjudicated by Urine Output, within 24hrs of enrollment. Sample is at enrollment.

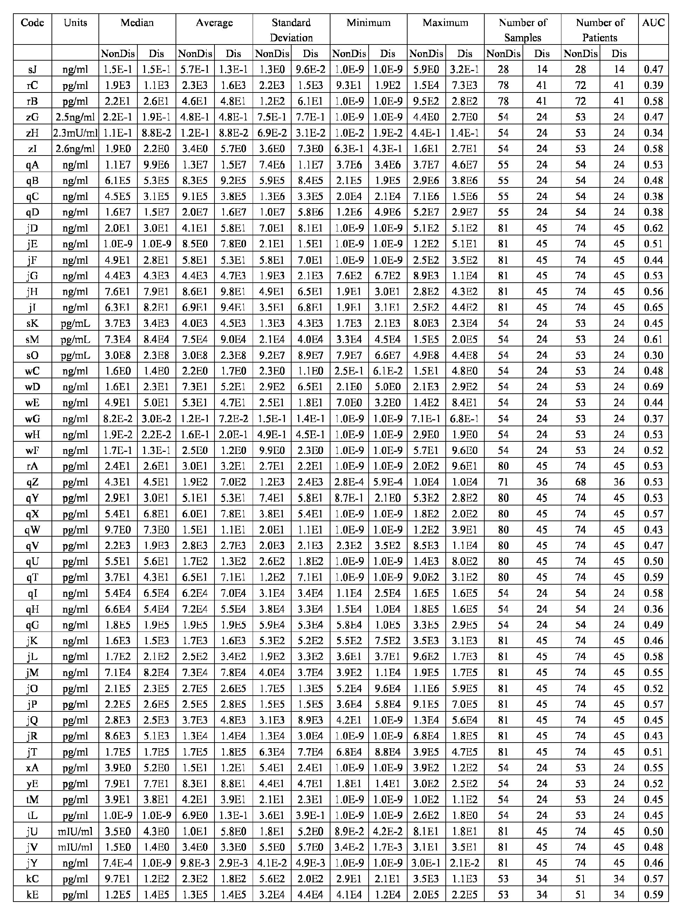

Figure 42. No, R, or I RIFLE stage versus RIFLE F. RIFLE stage adjudicated by sCr and Urine Output, within 24hrs of enrollment. Sample is at enrollment.

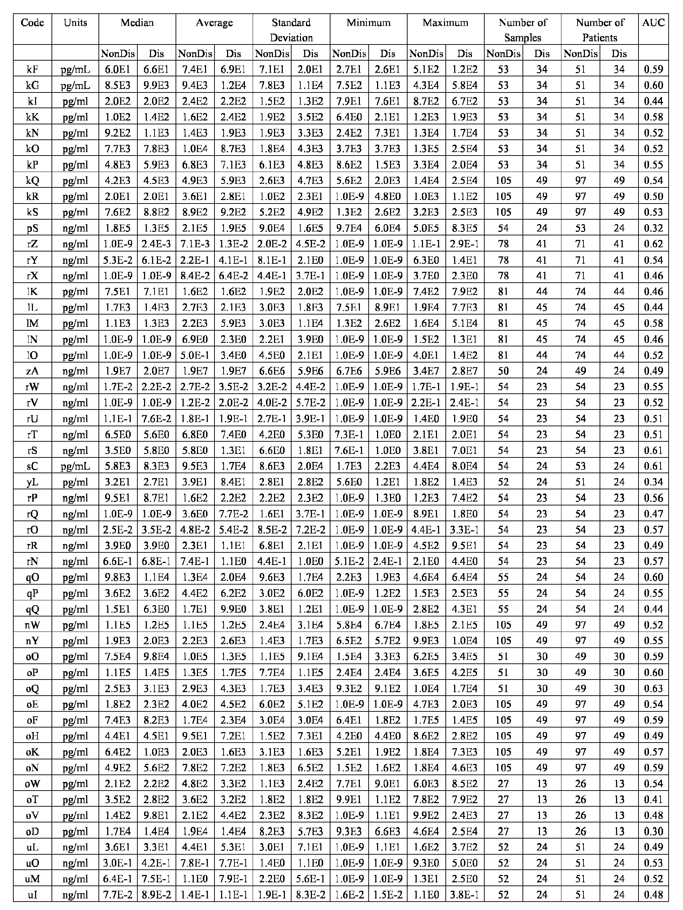

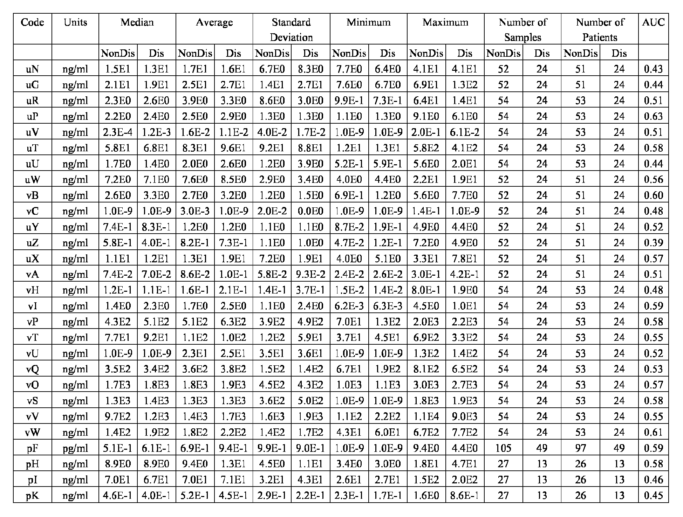

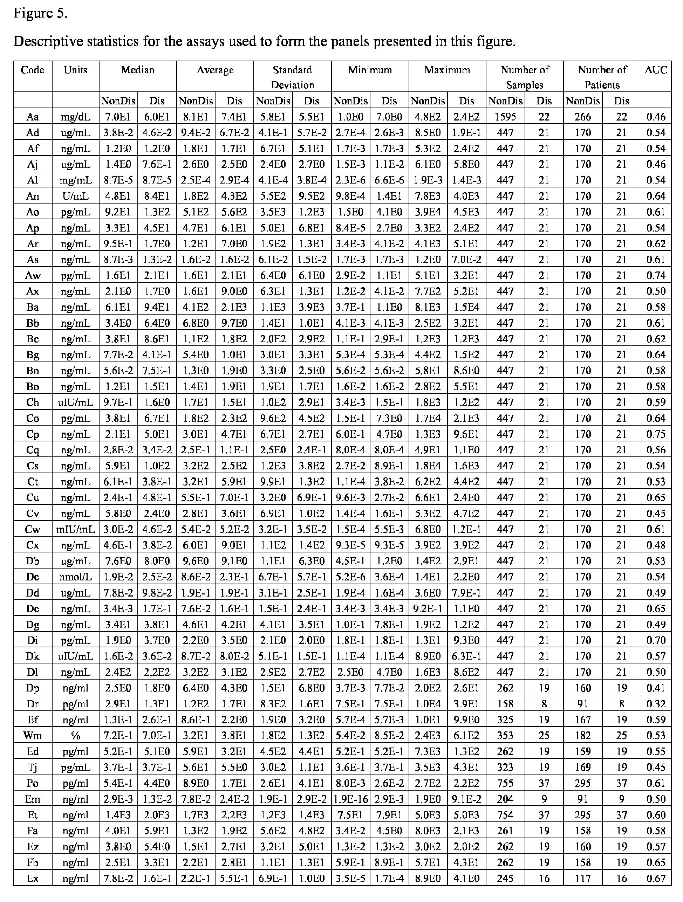

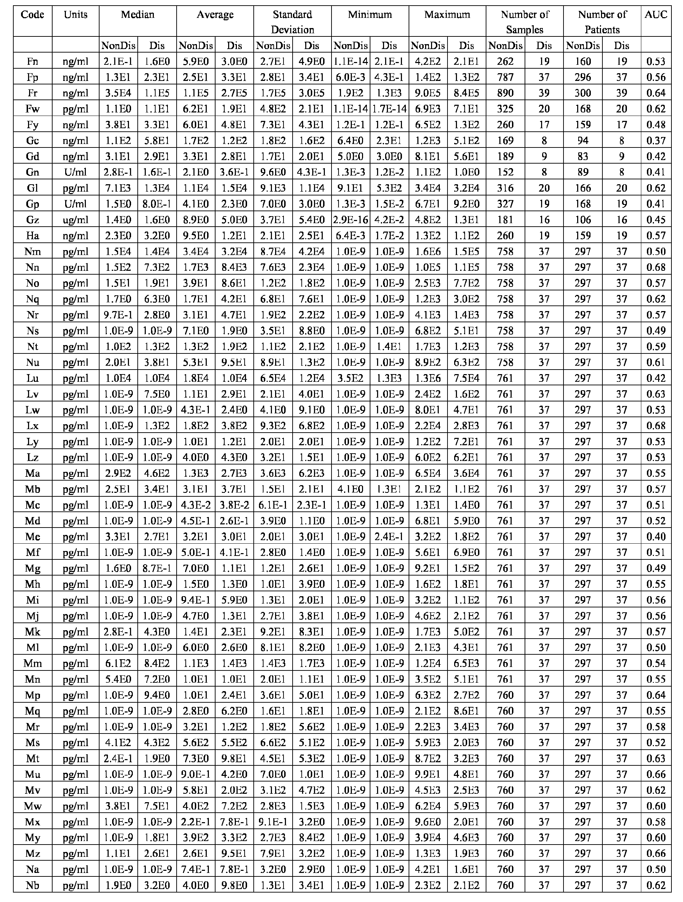

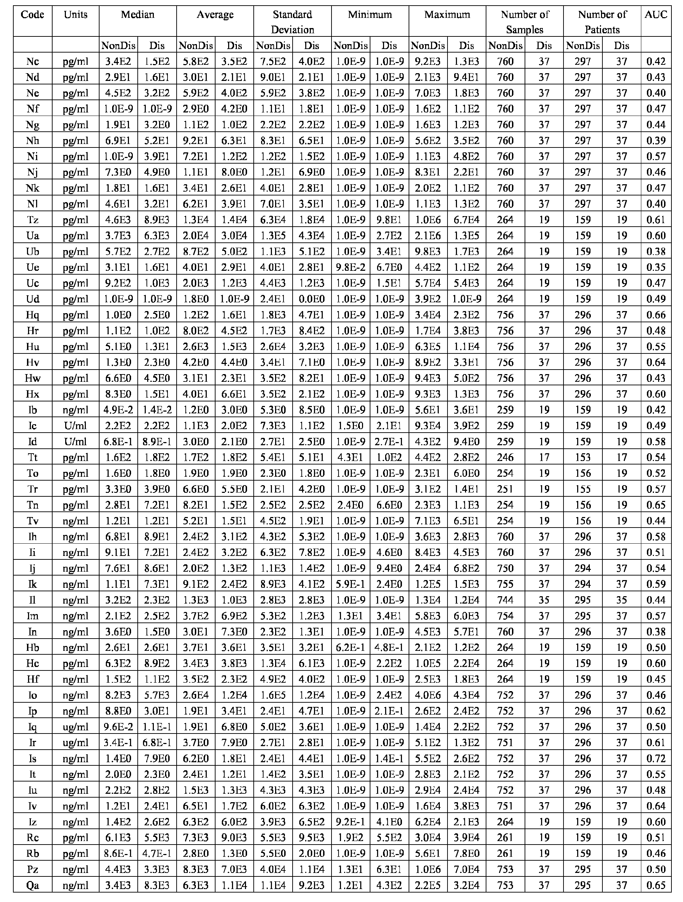

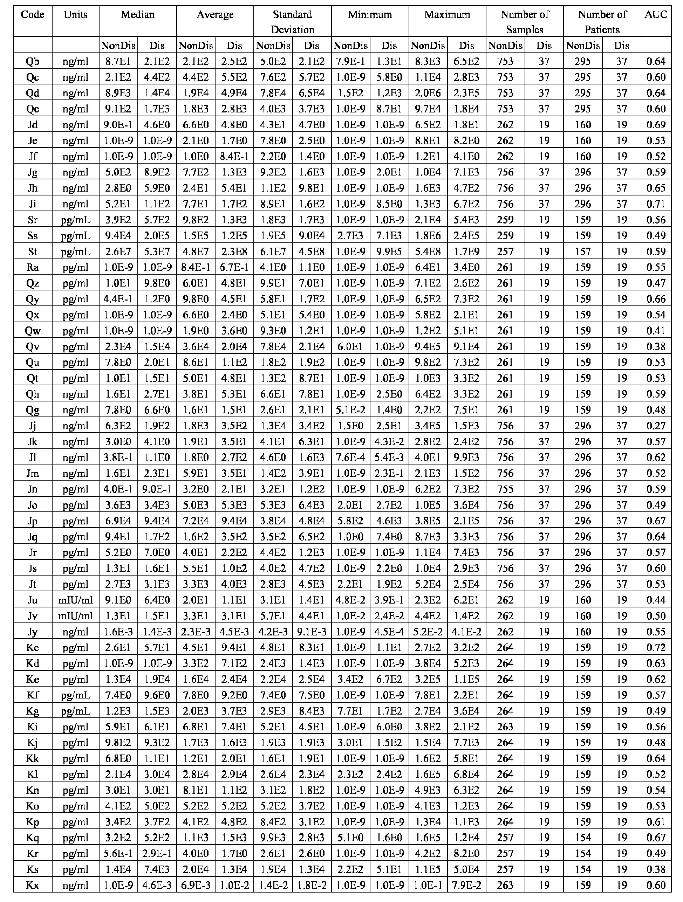

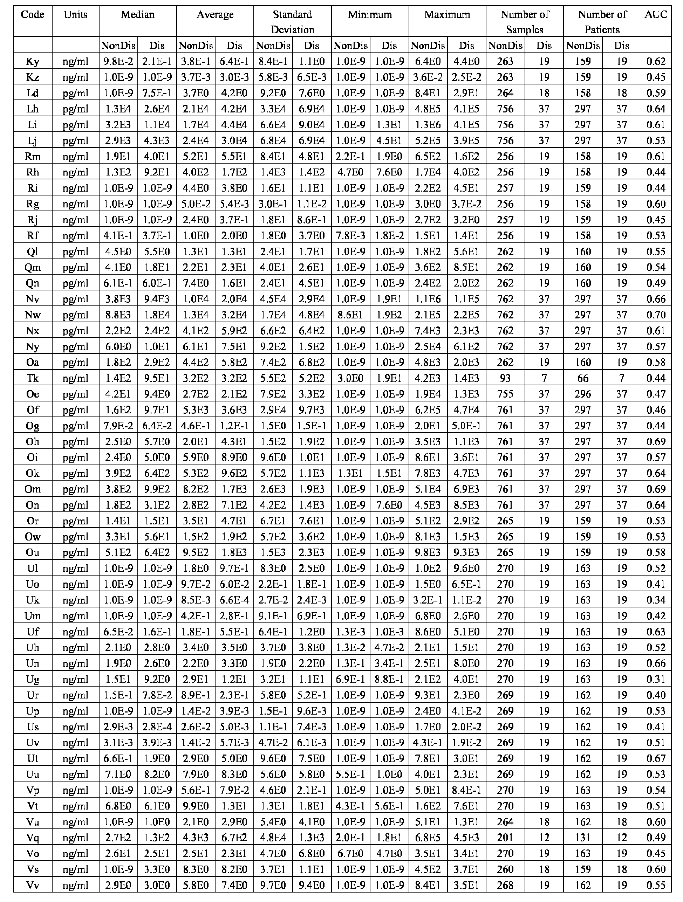

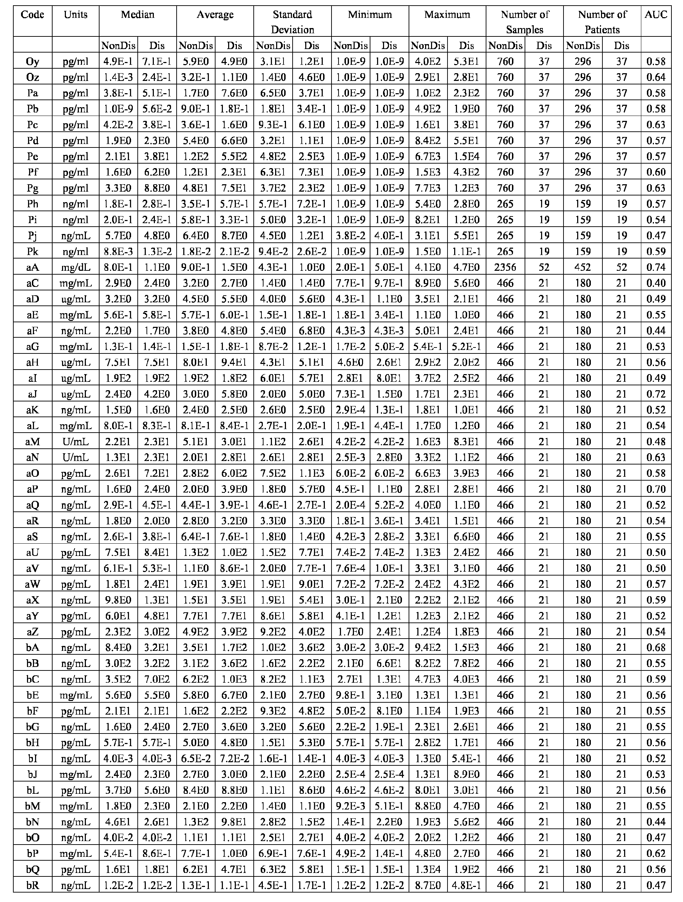

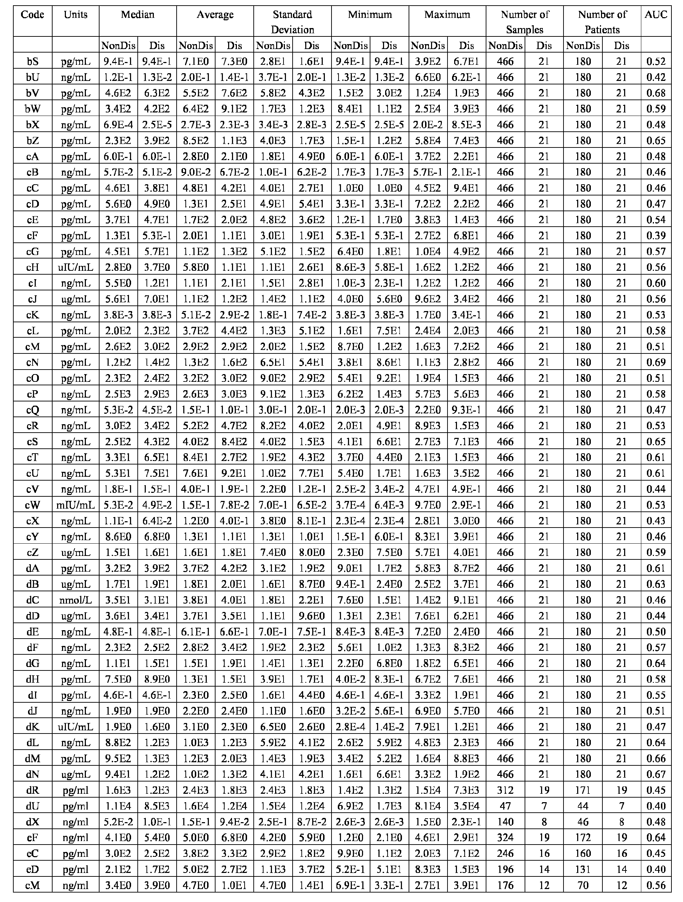

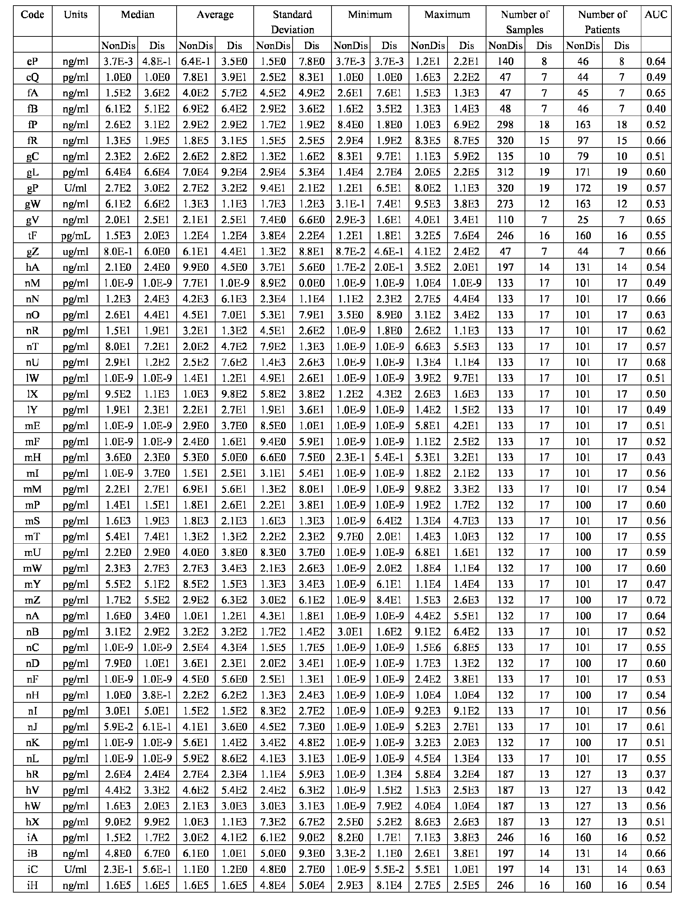

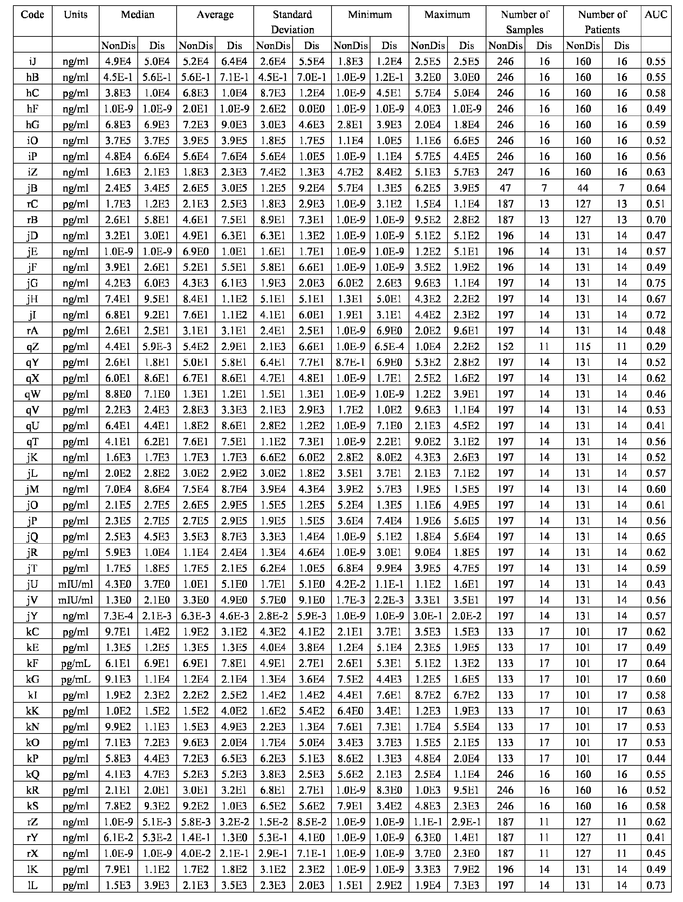

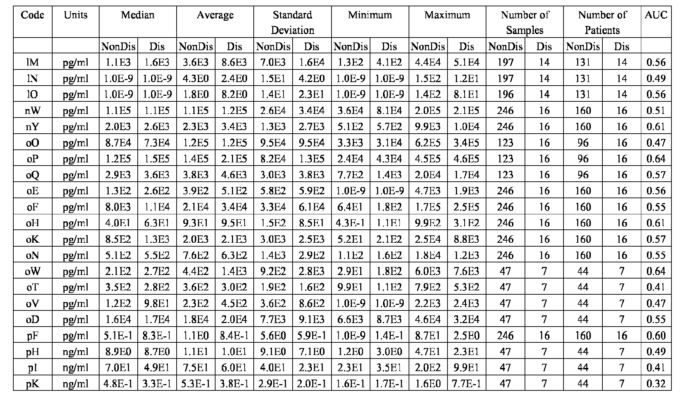

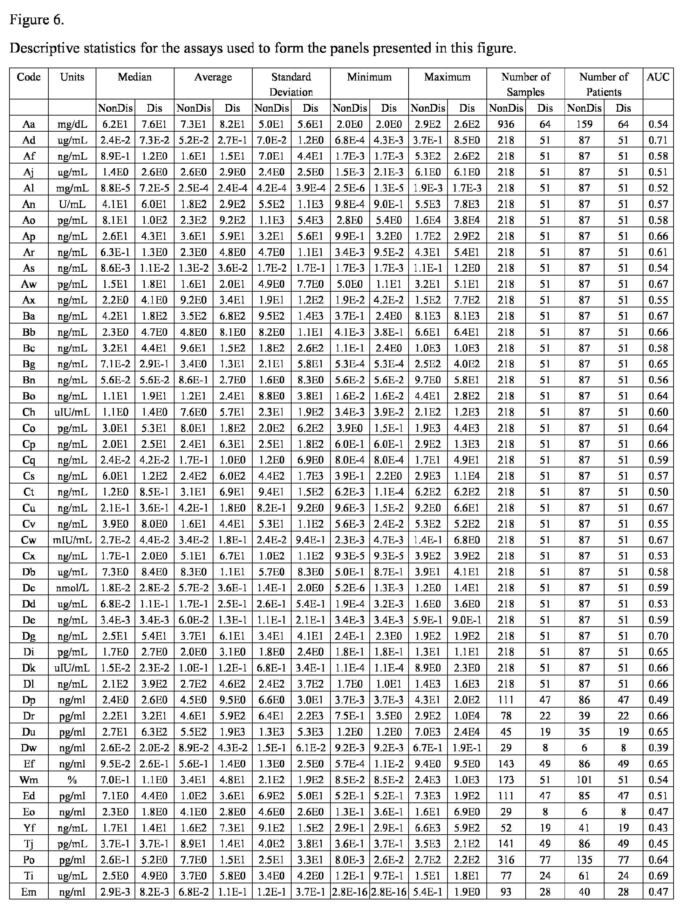

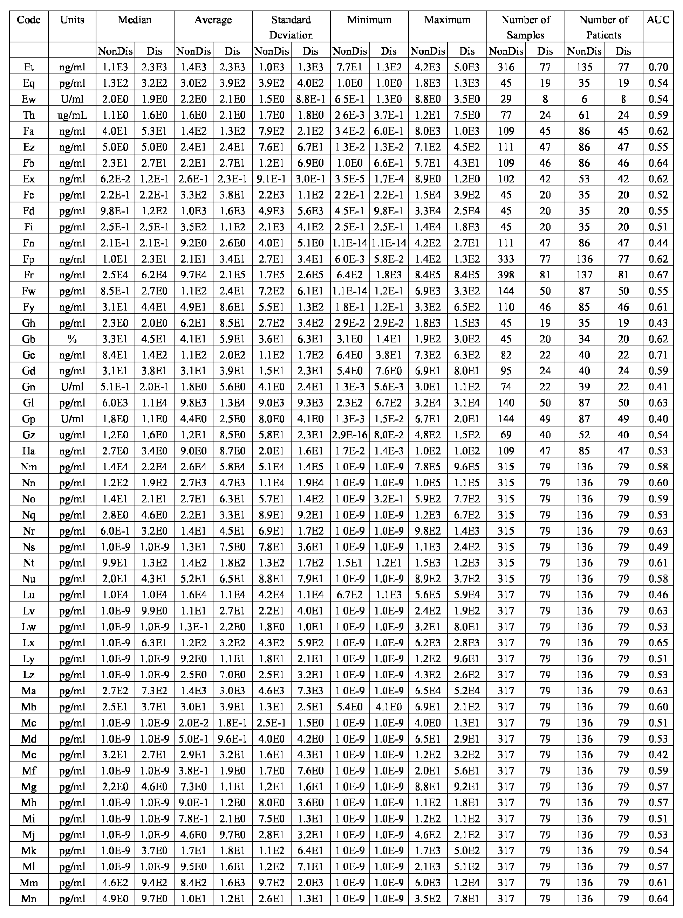

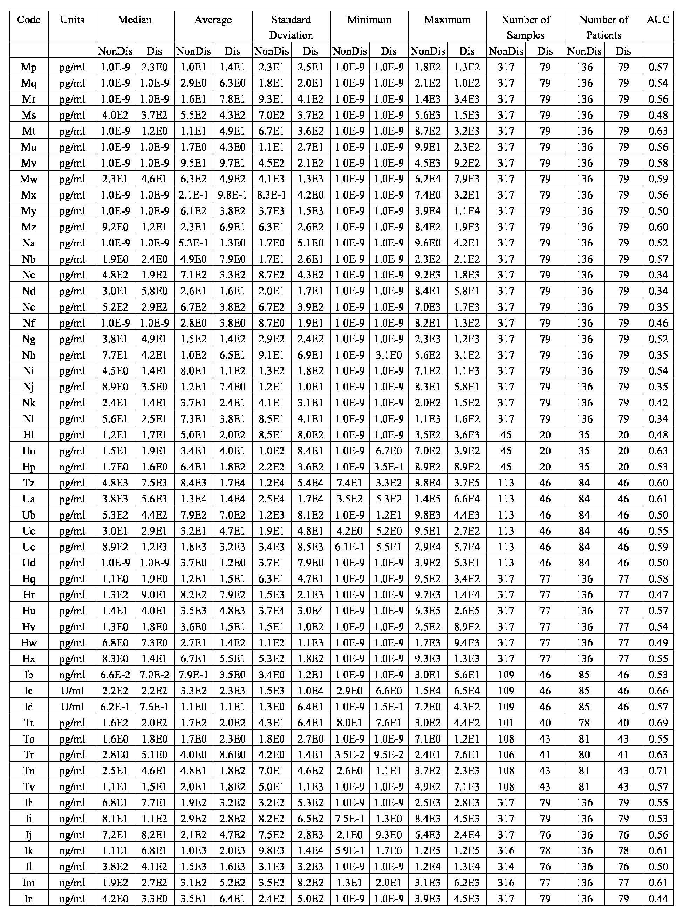

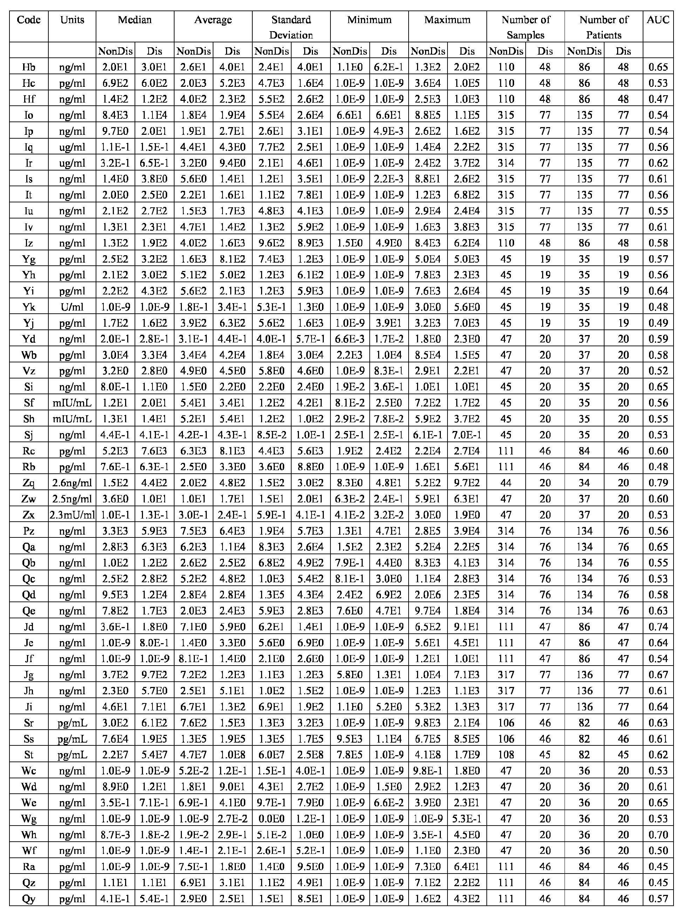

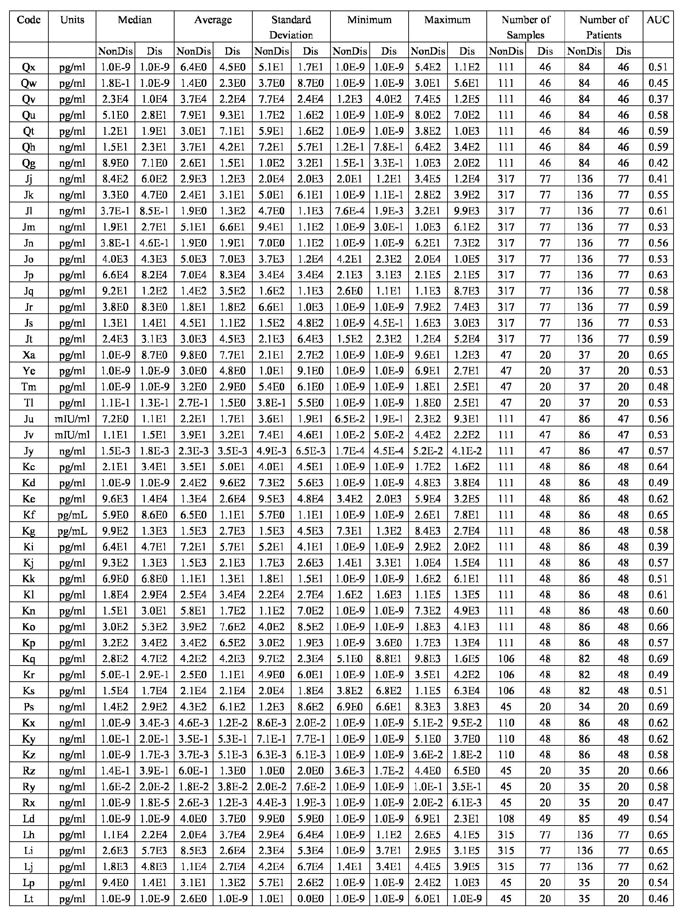

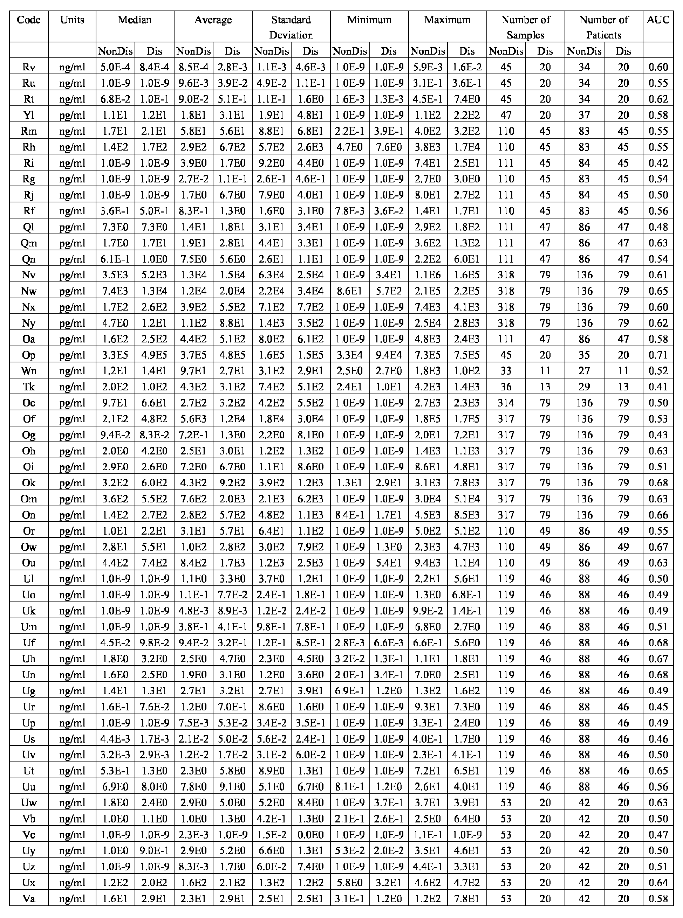

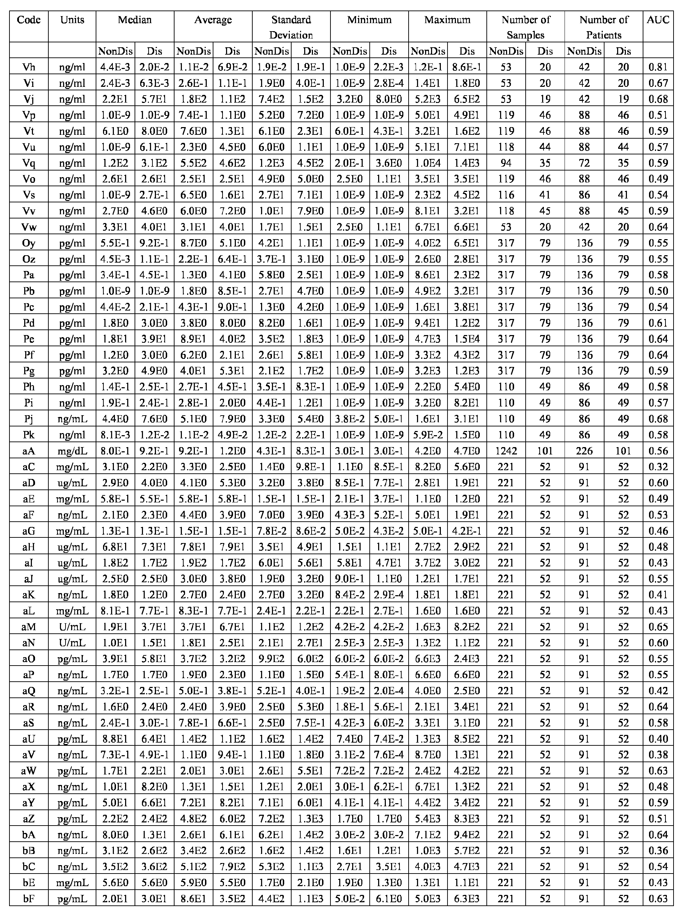

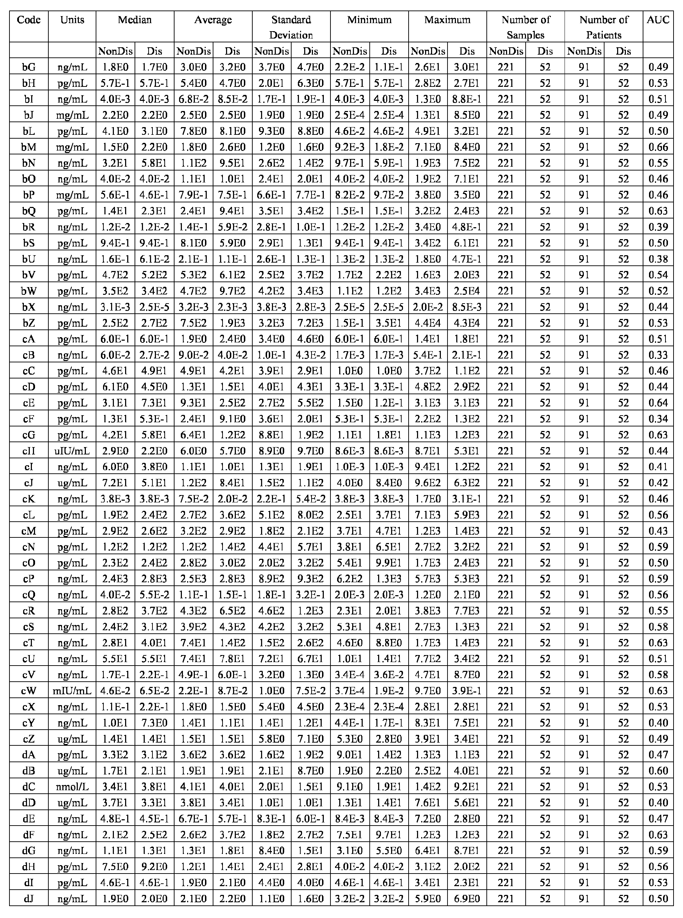

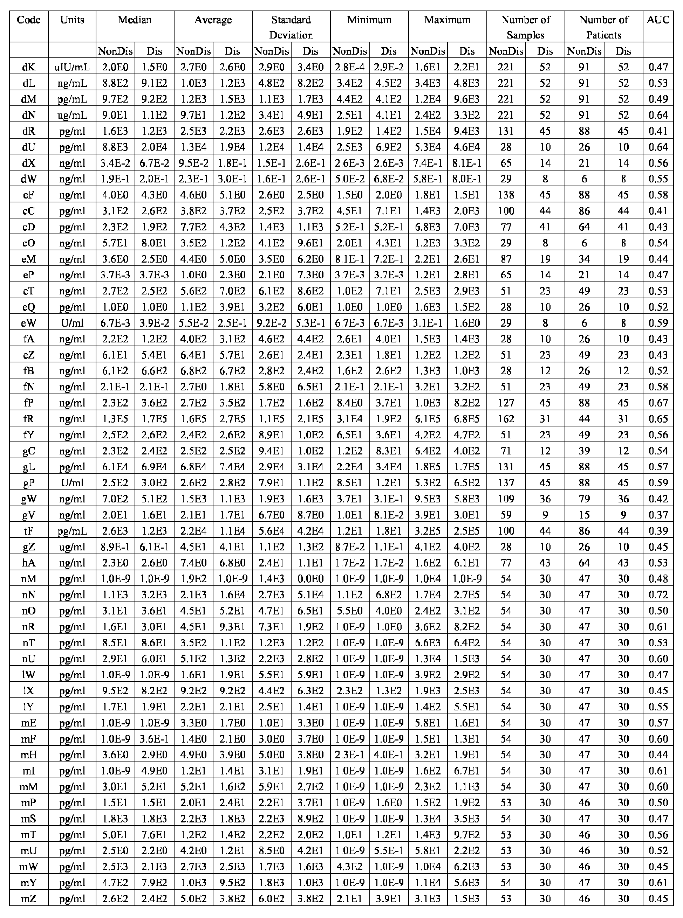

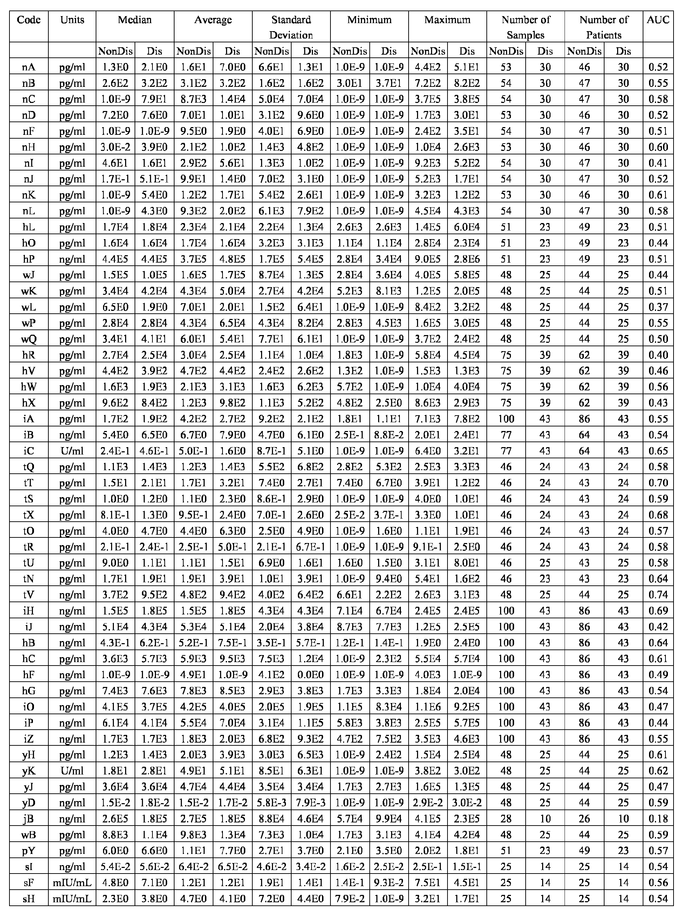

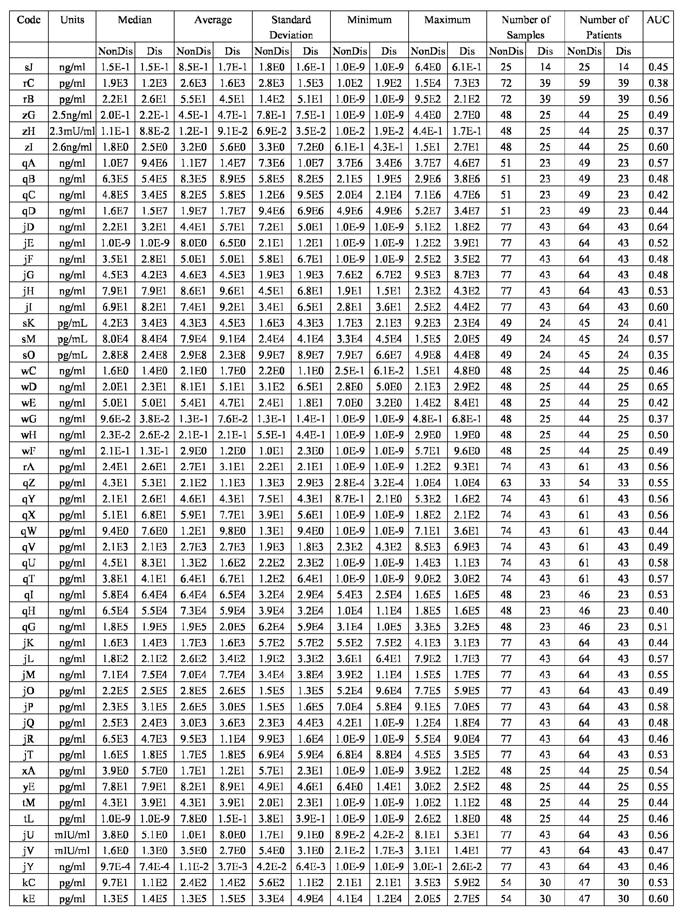

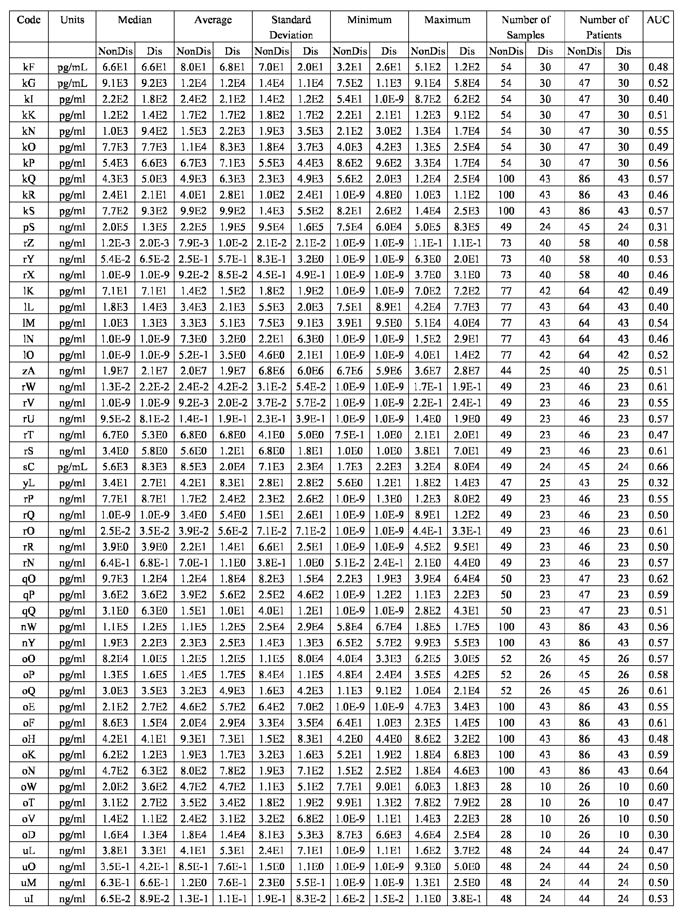

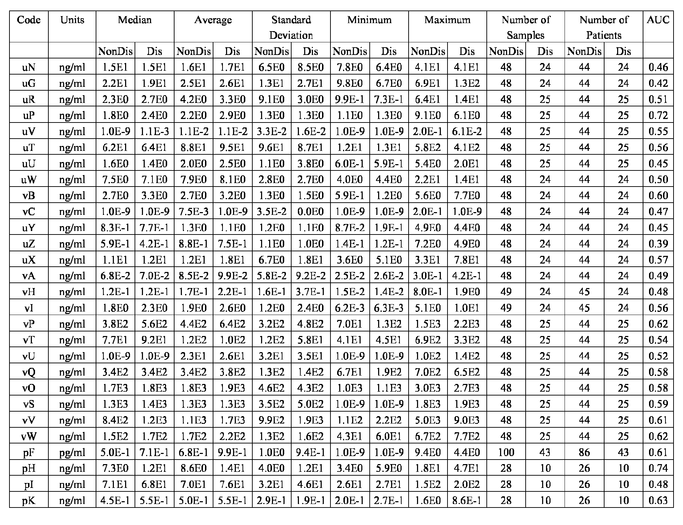

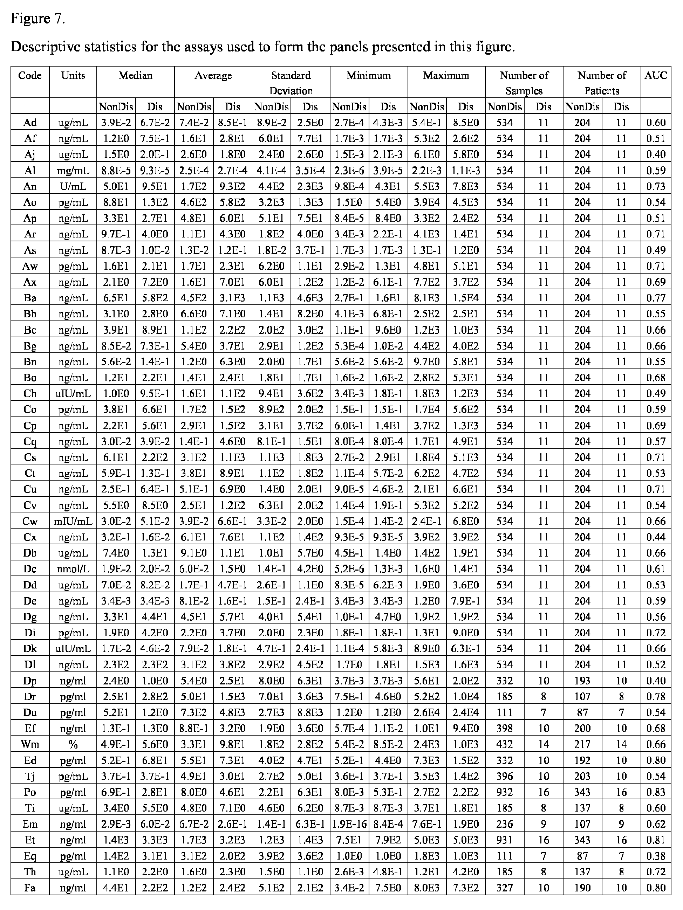

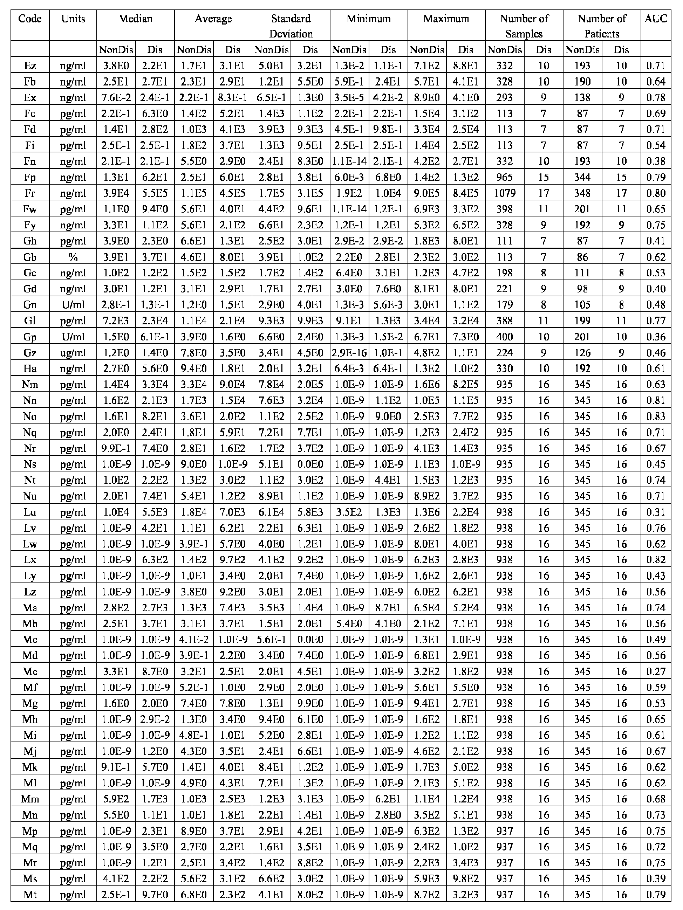

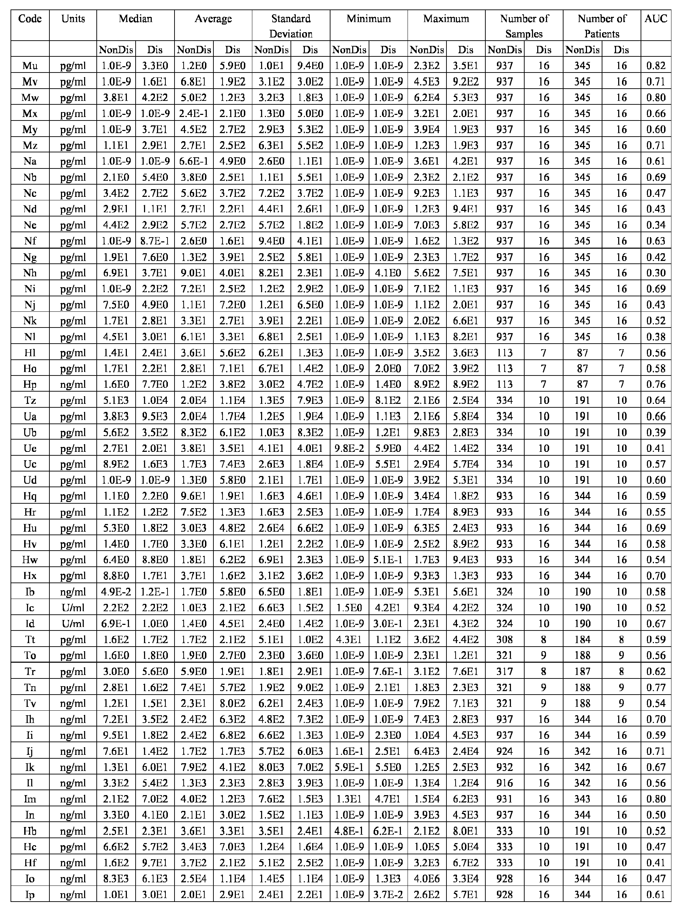

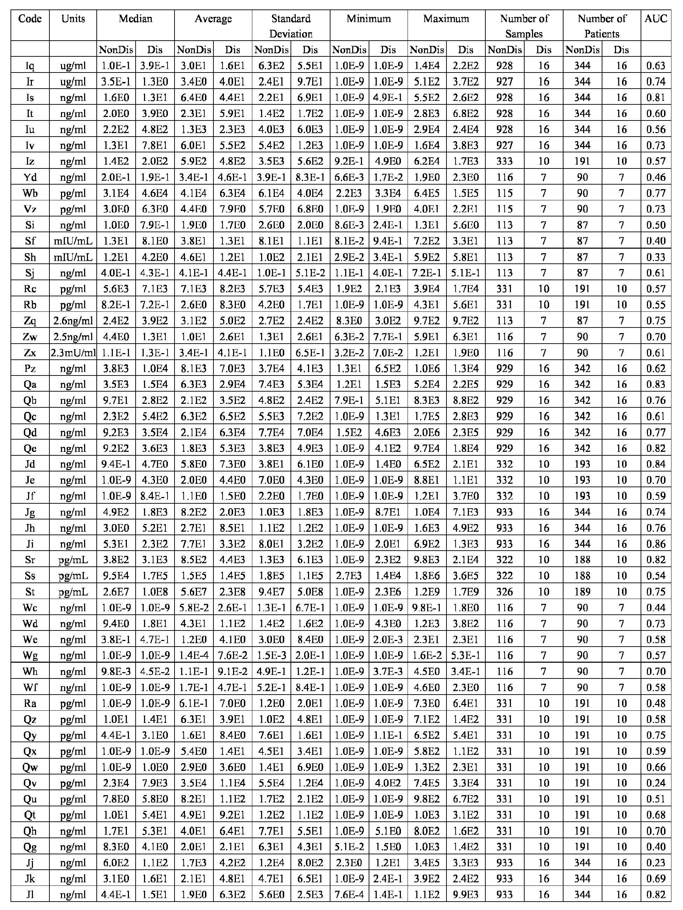

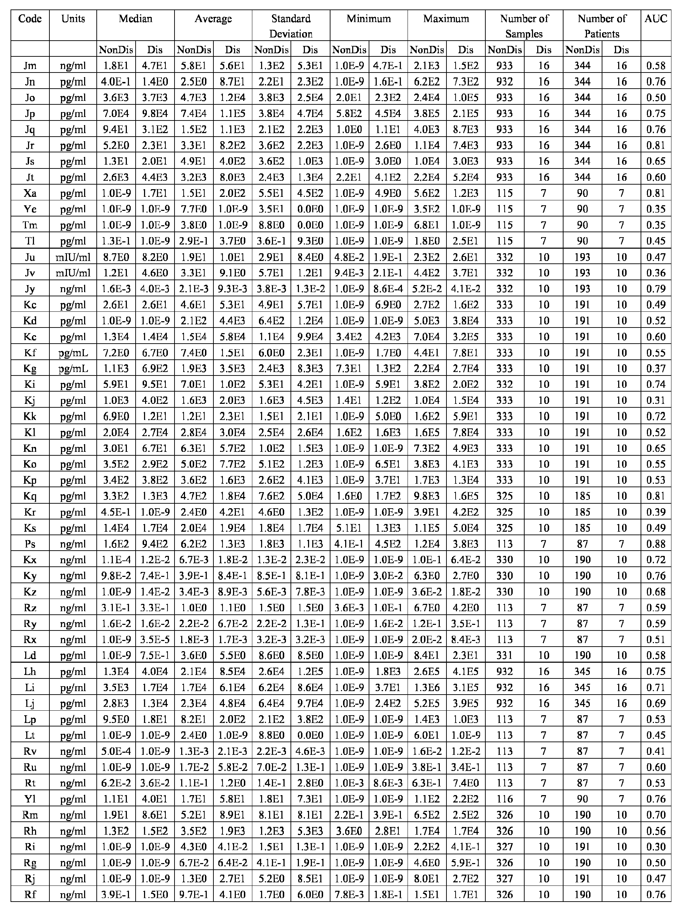

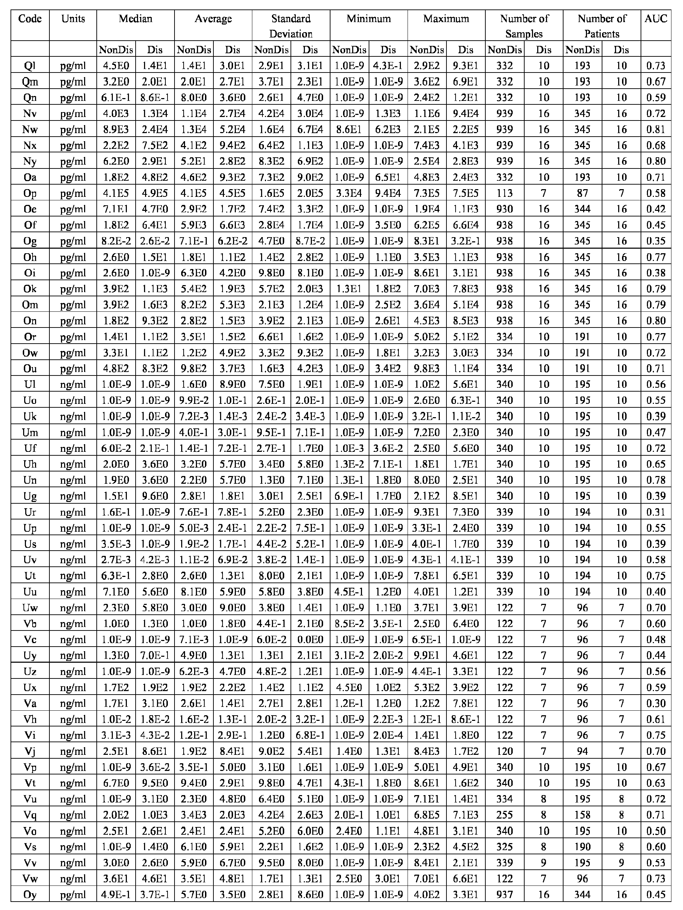

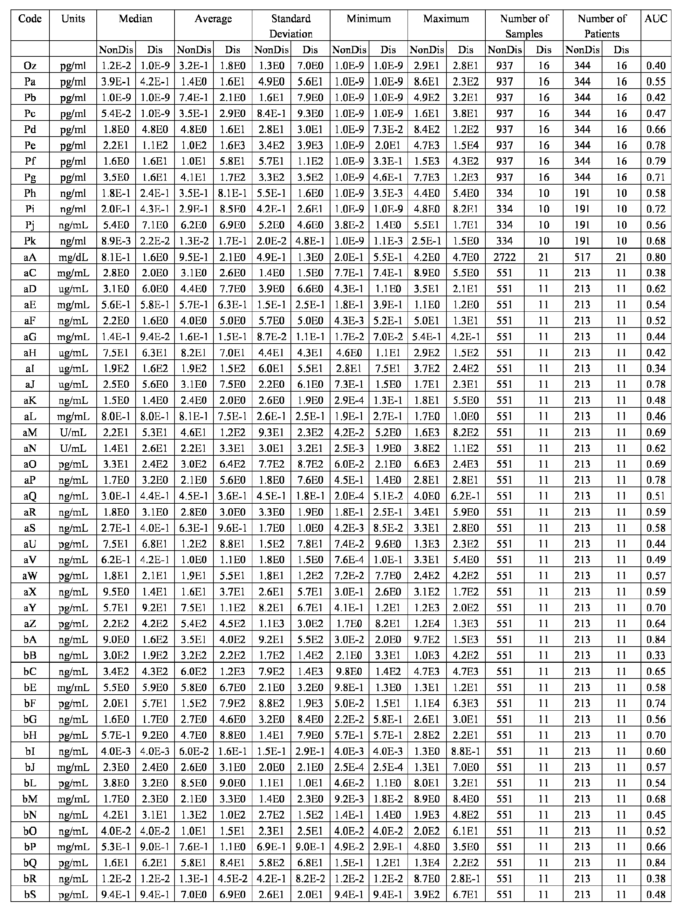

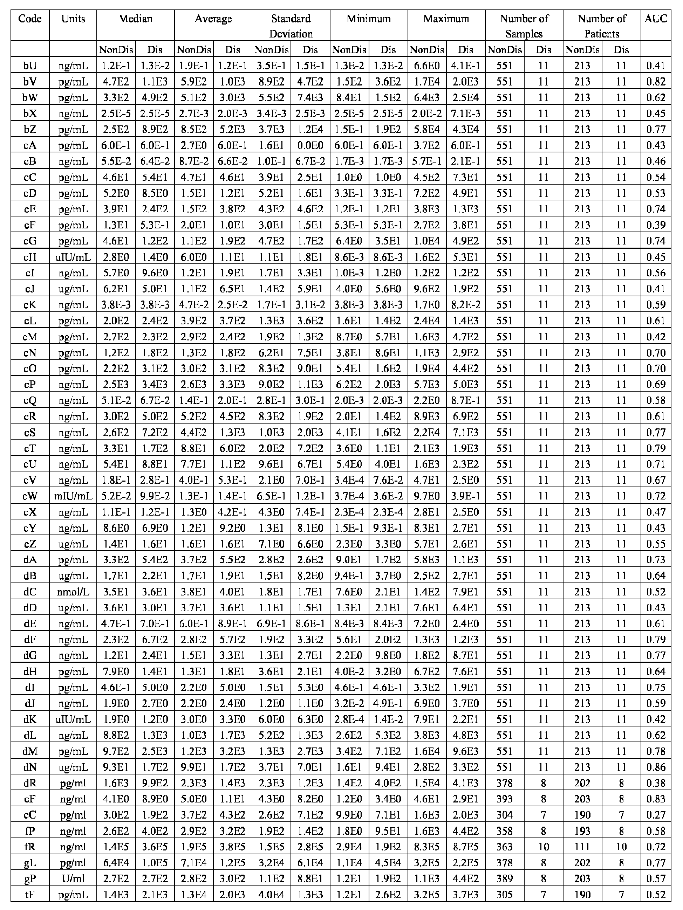

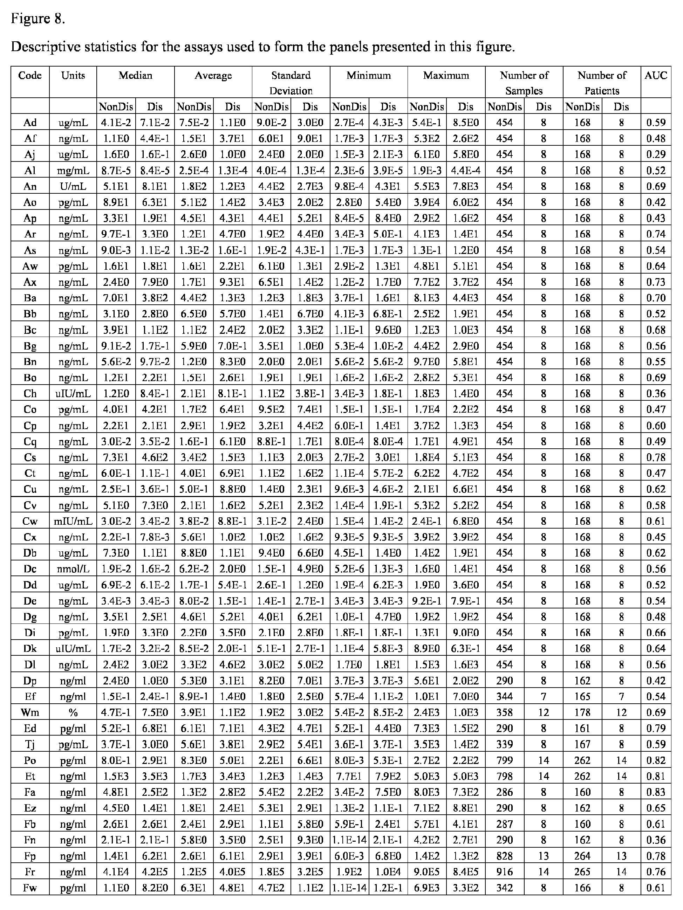

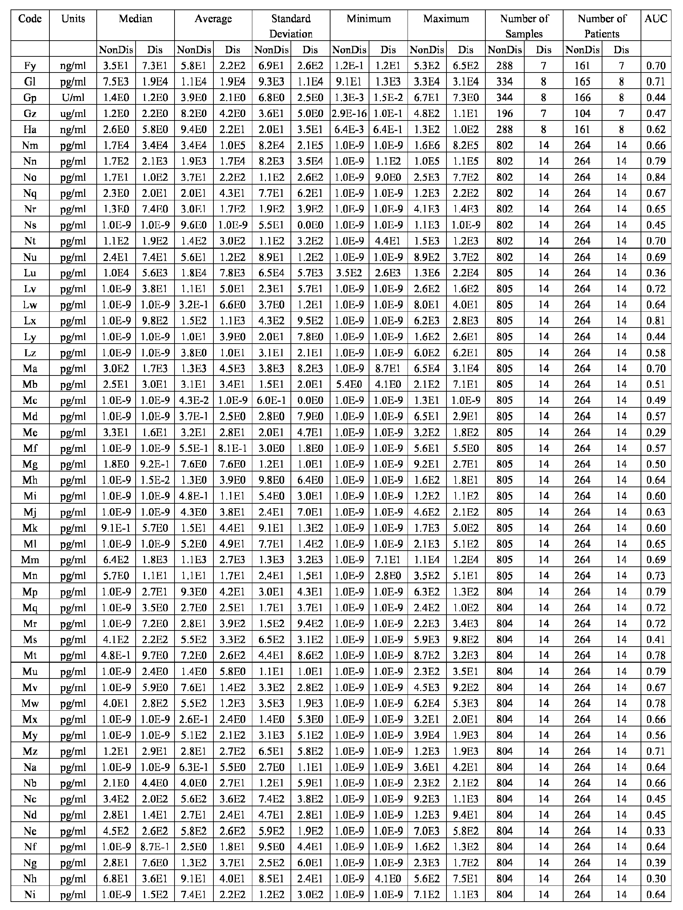

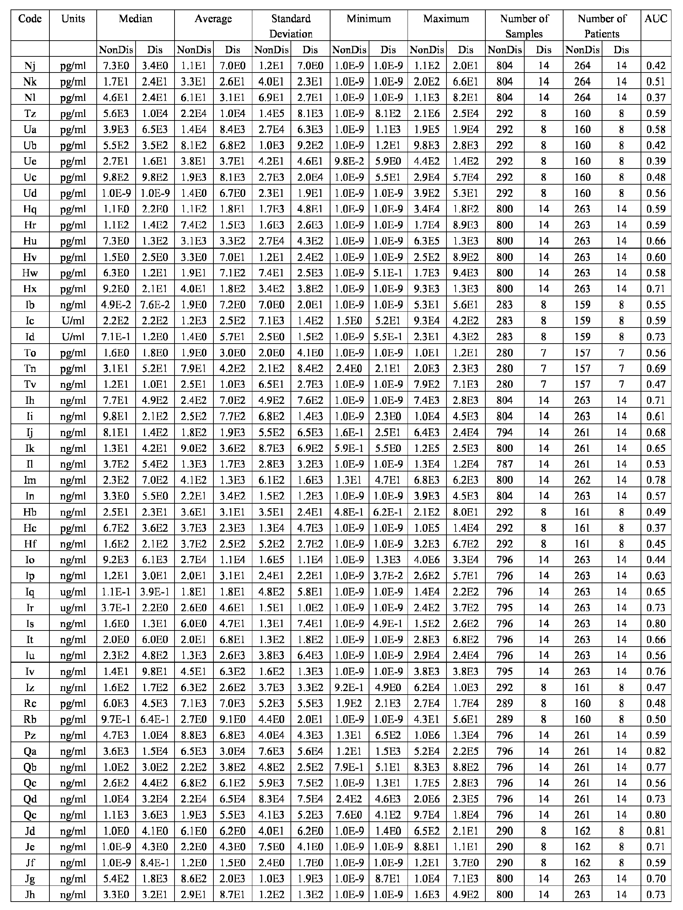

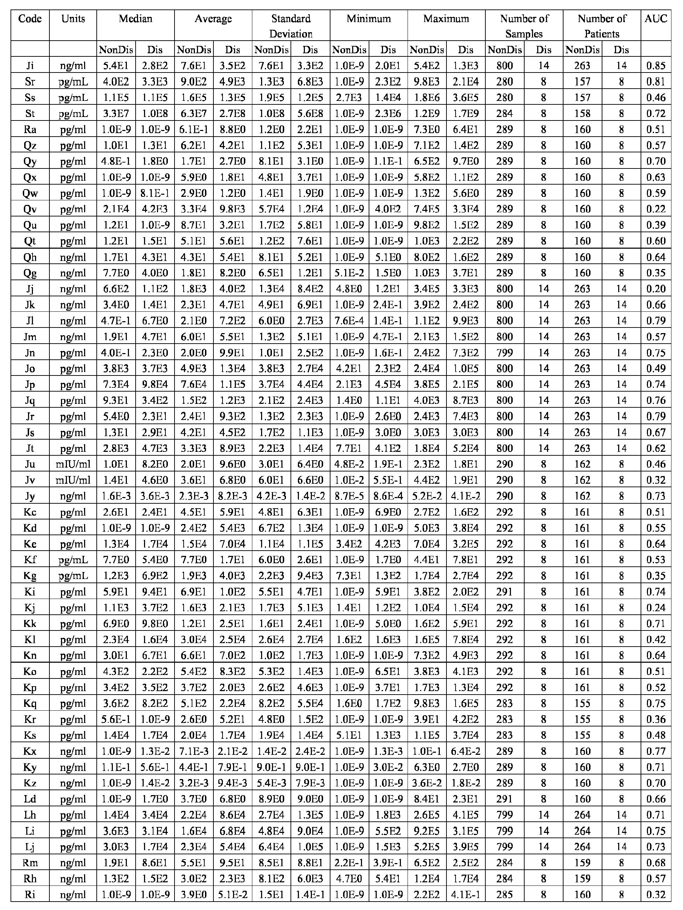

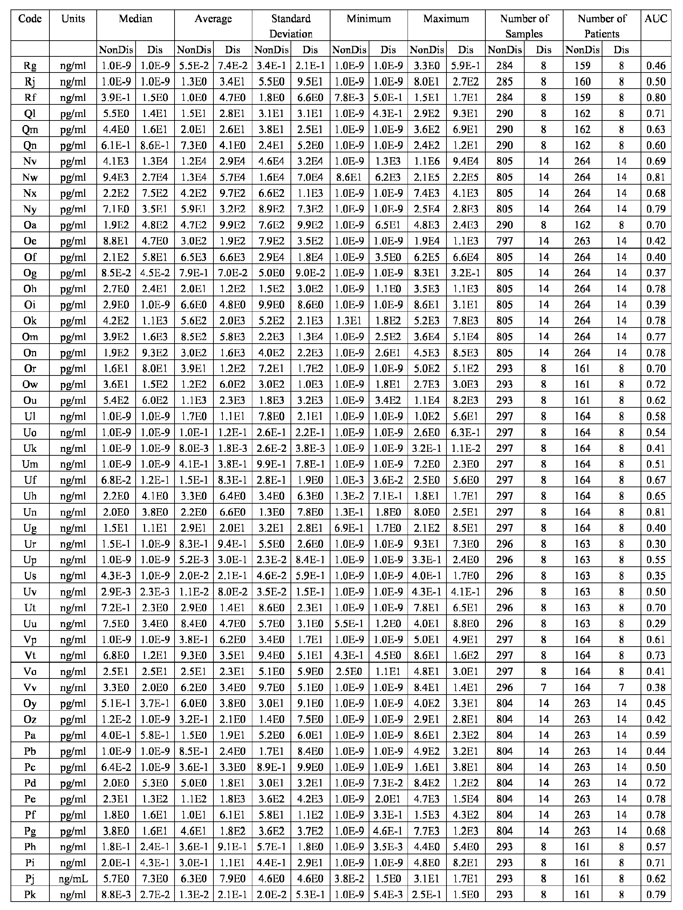

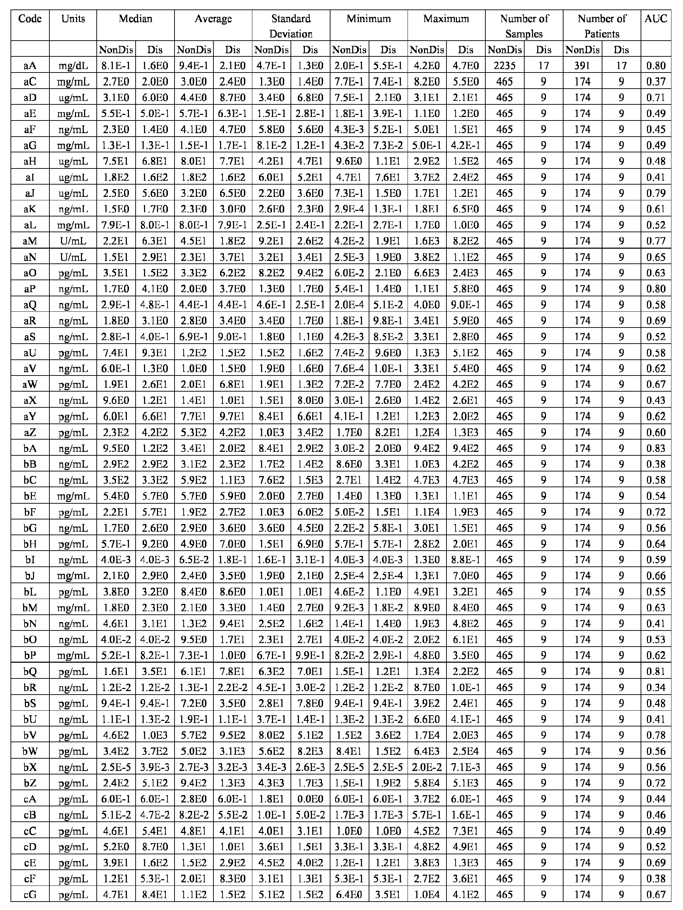

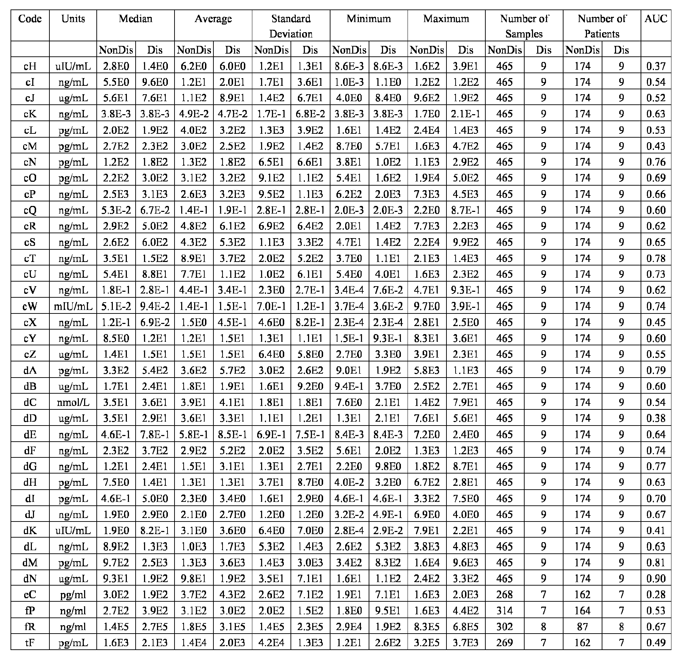

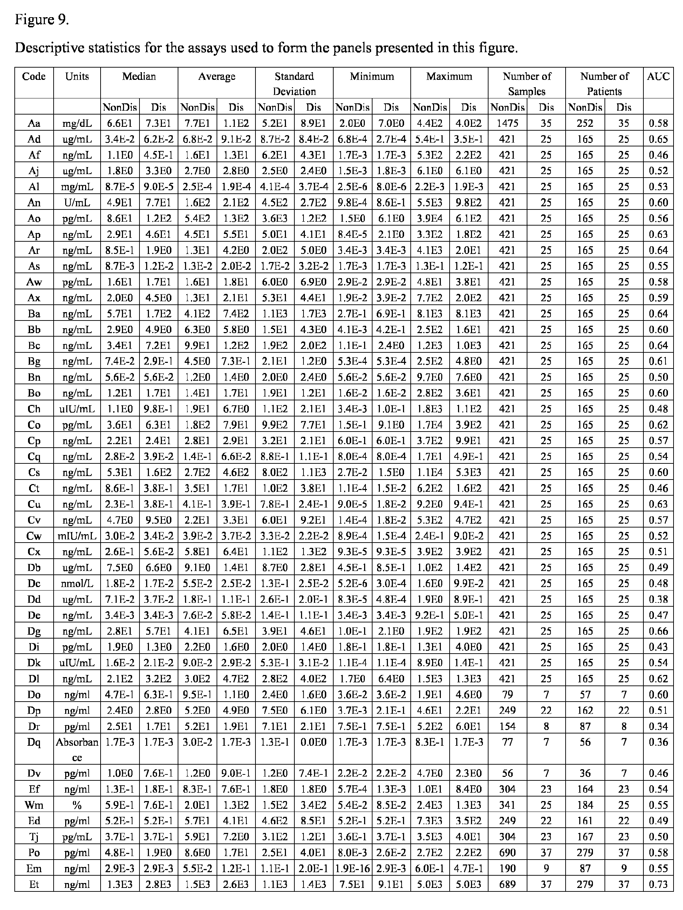

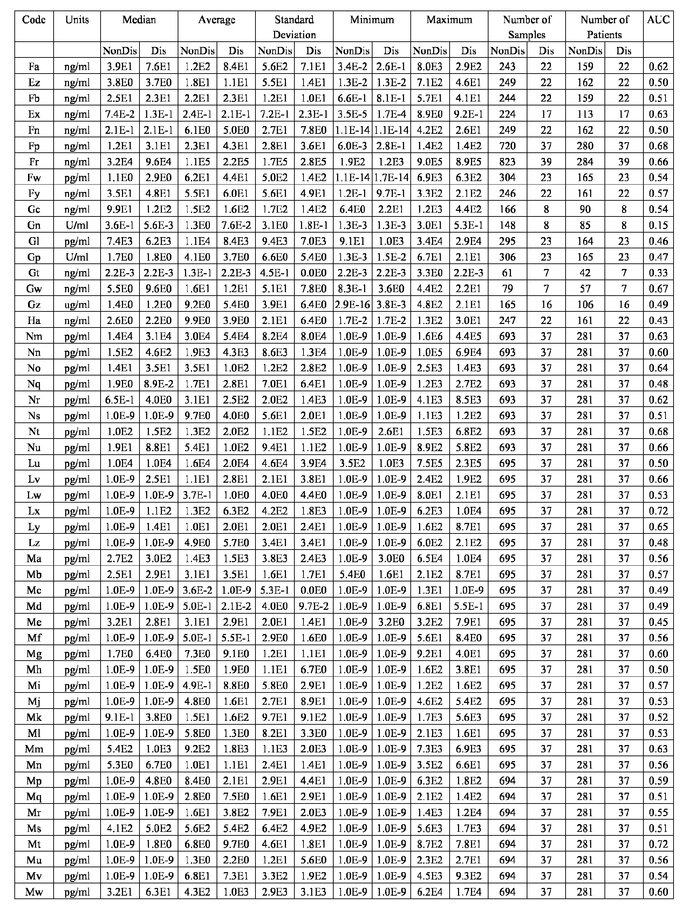

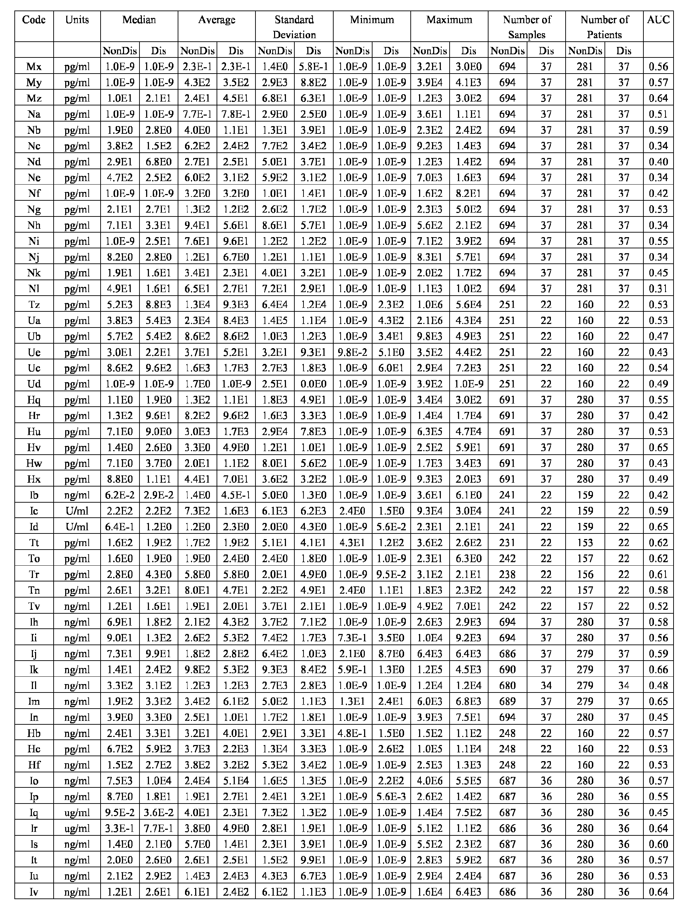

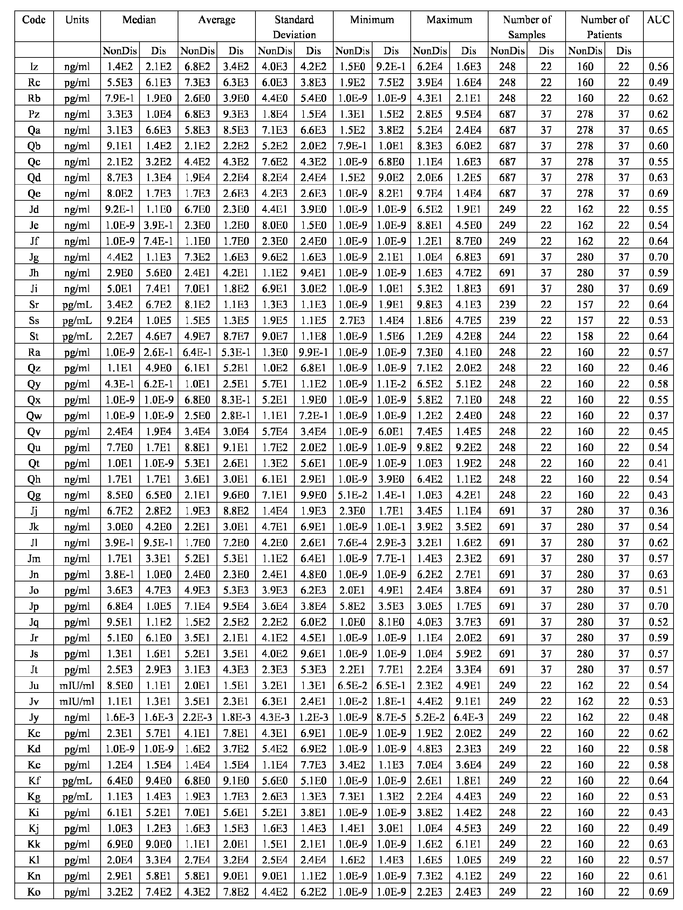

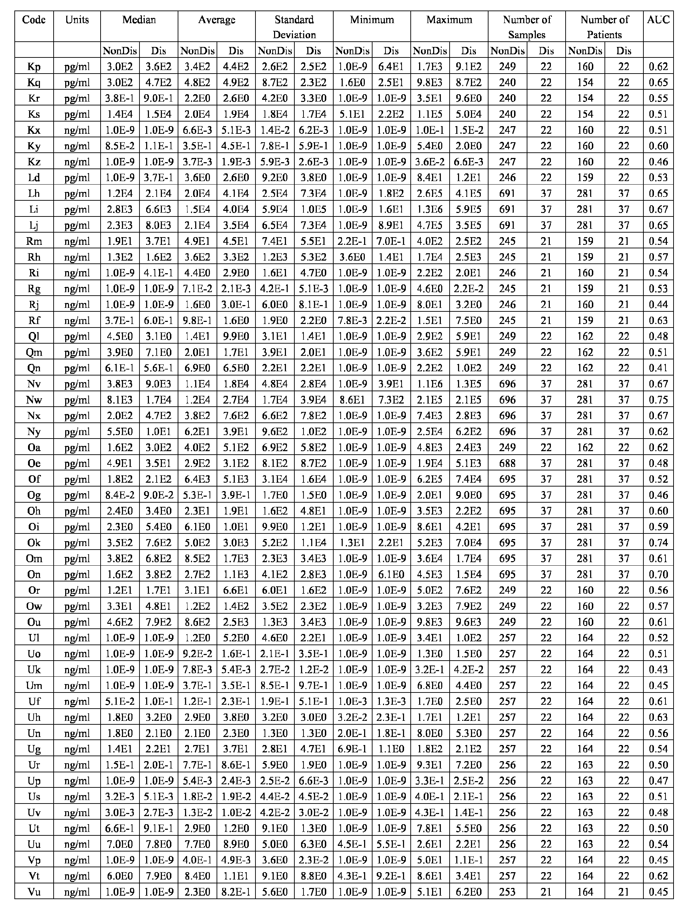

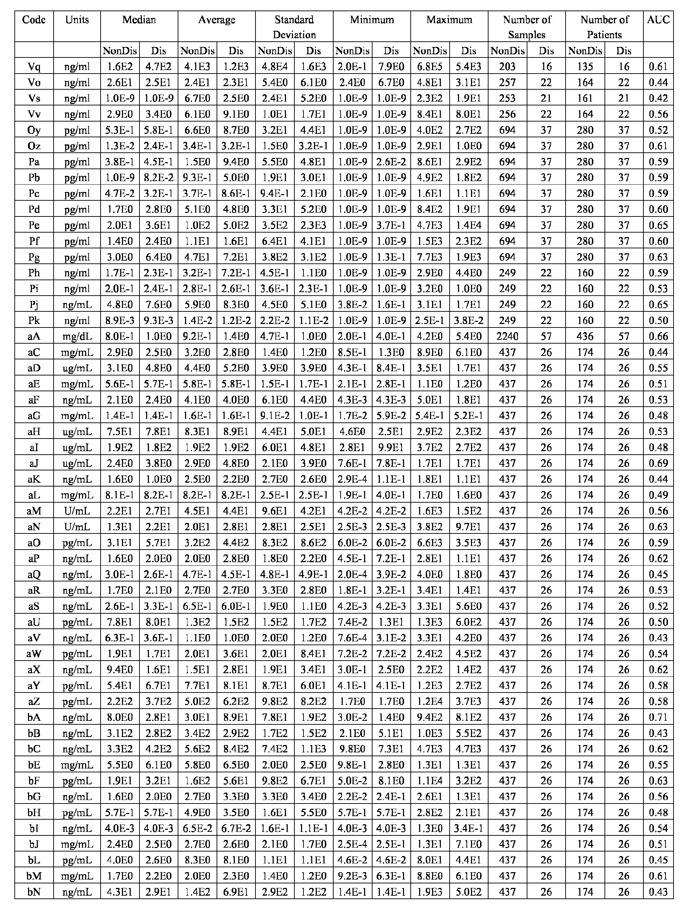

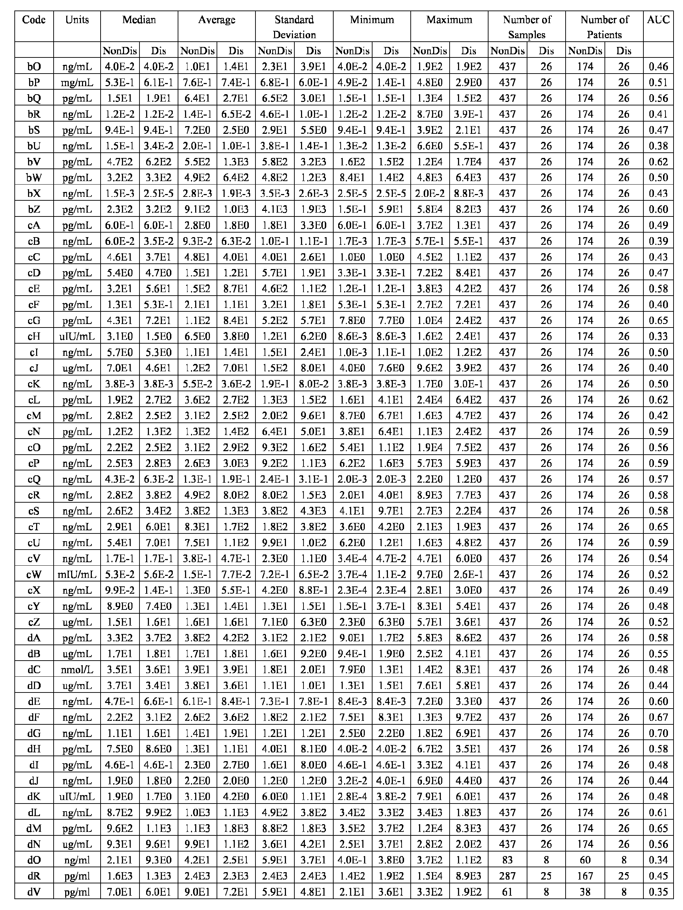

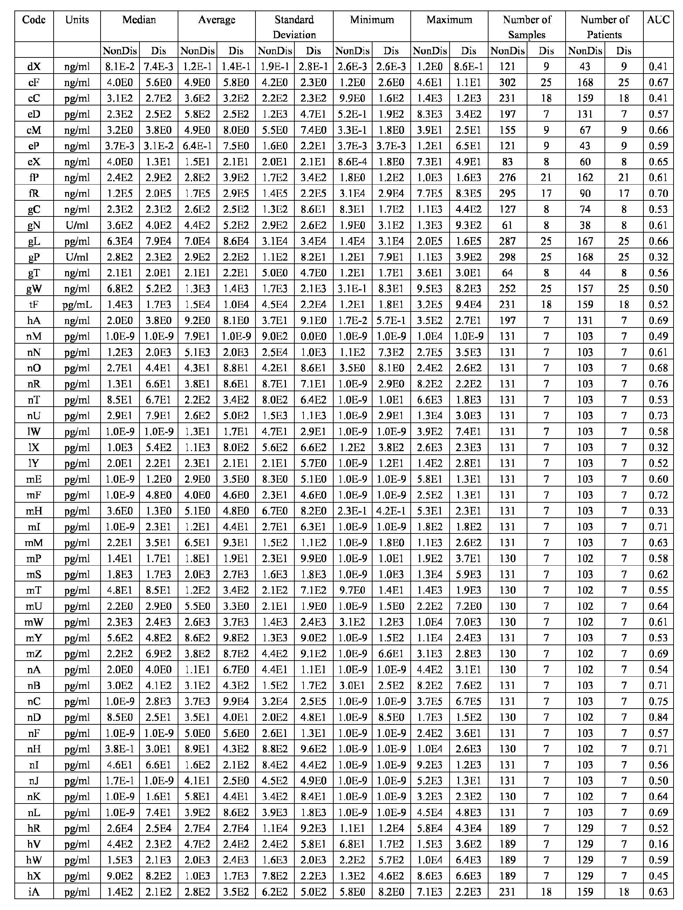

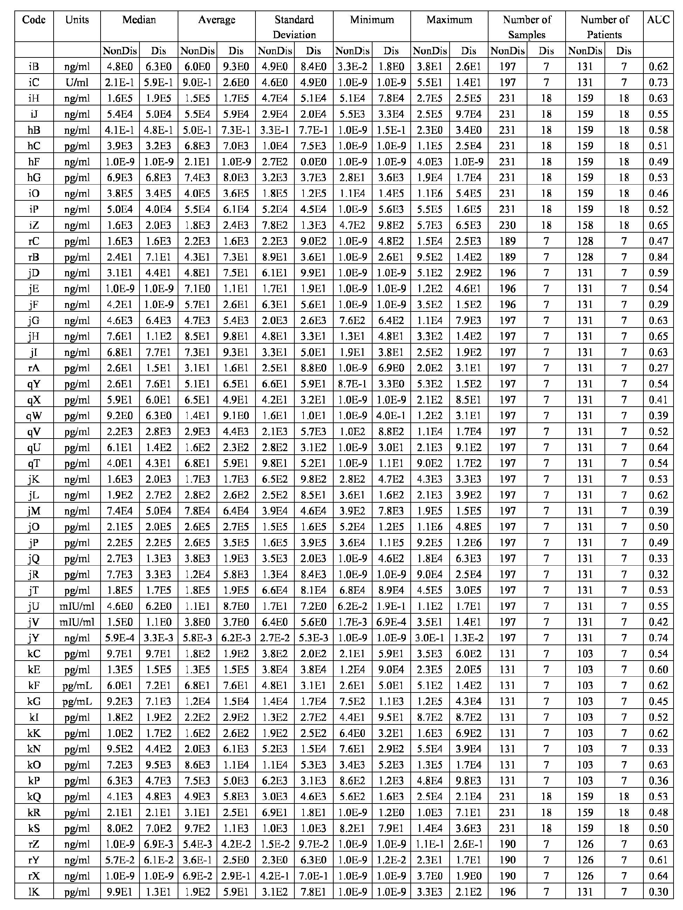

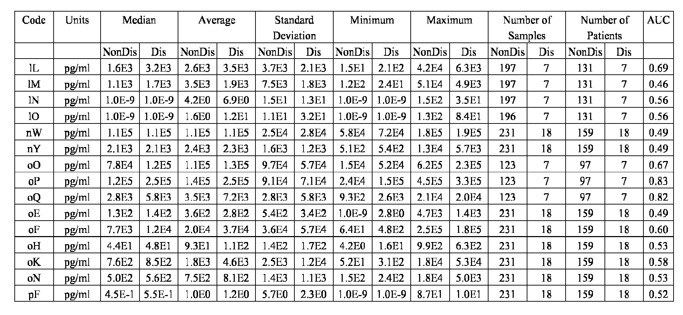

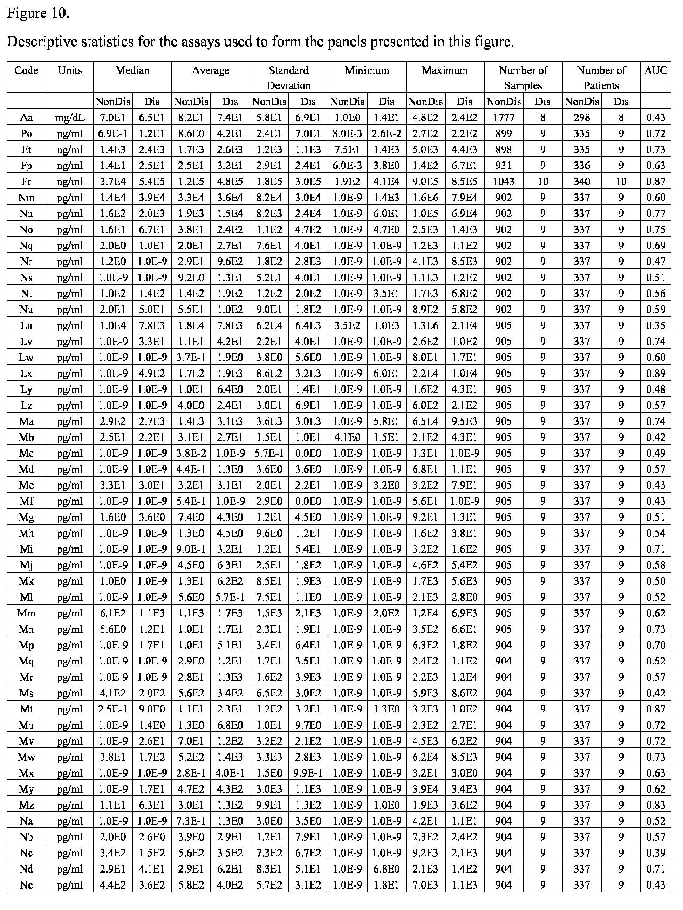

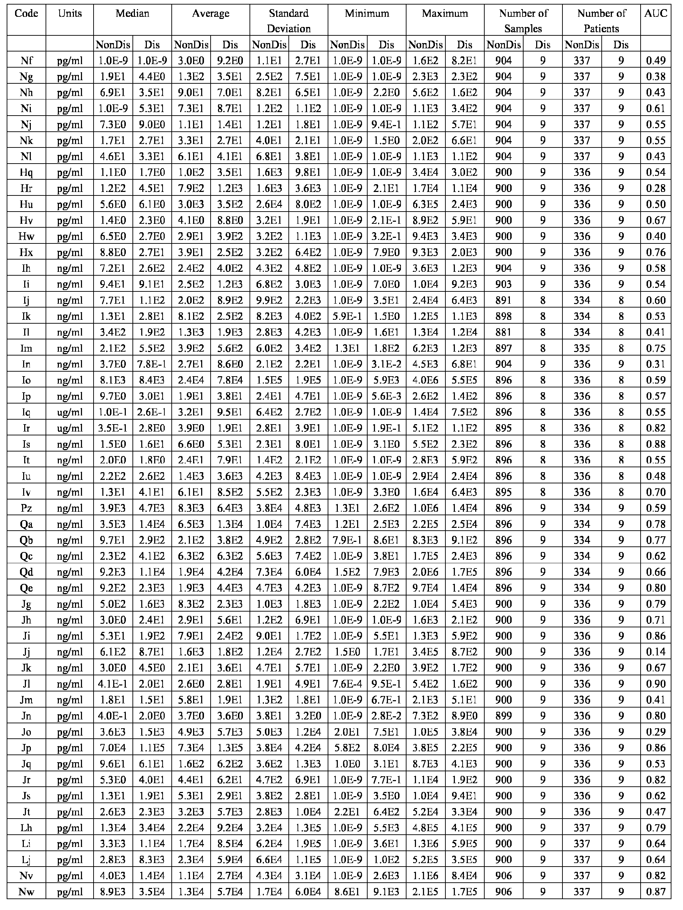

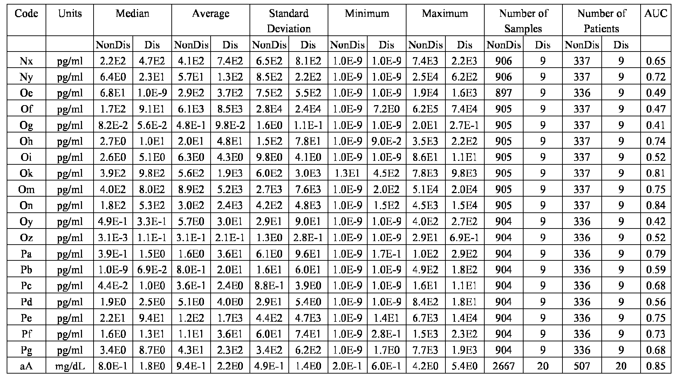

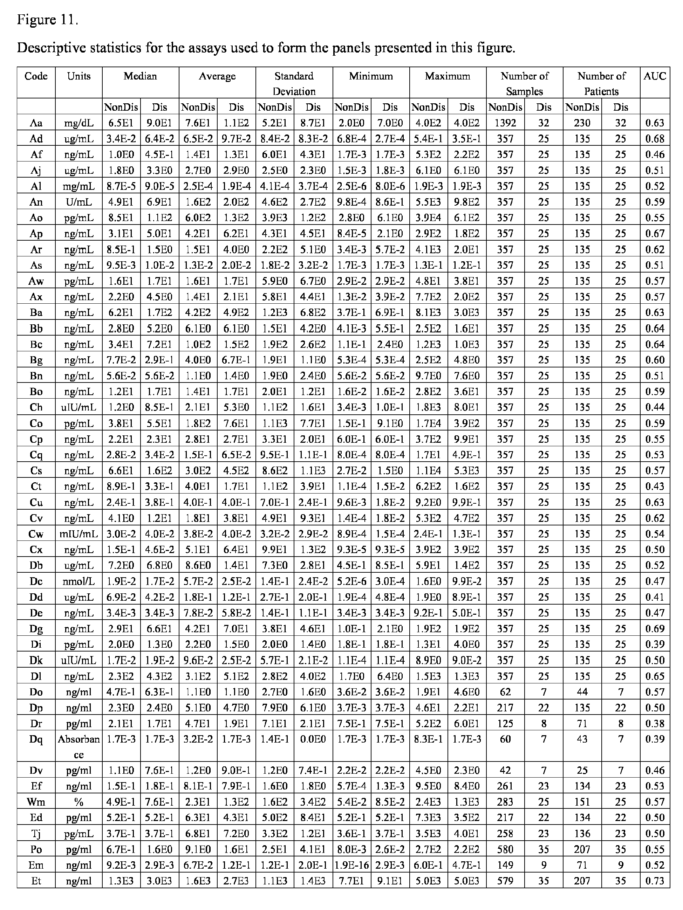

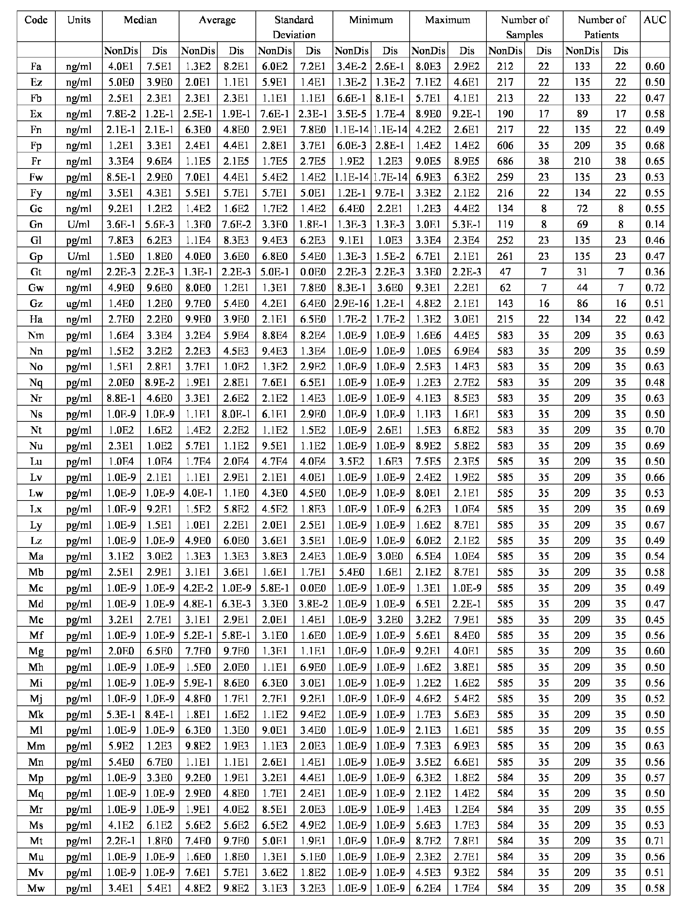

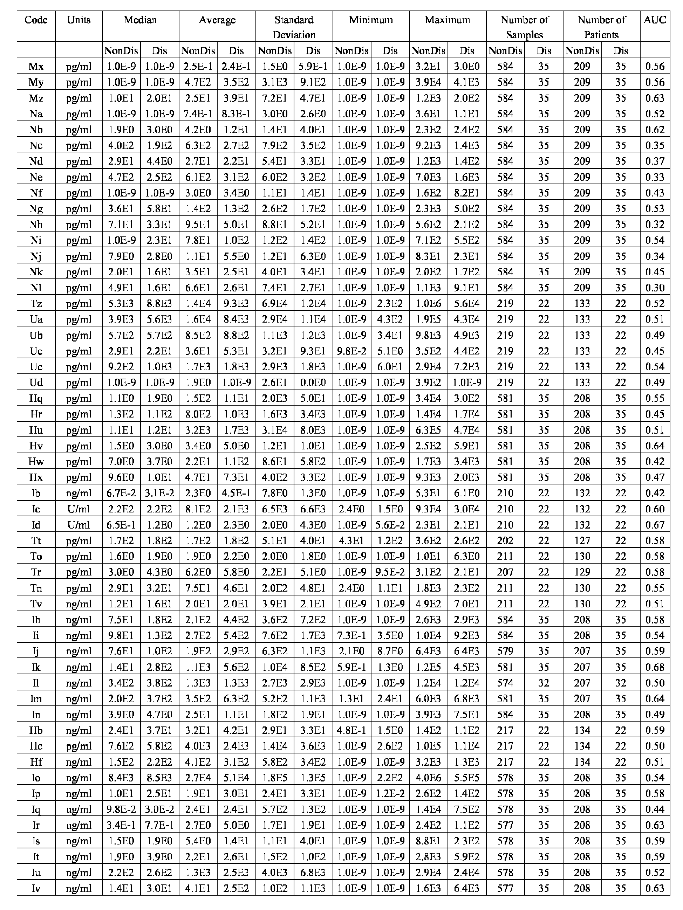

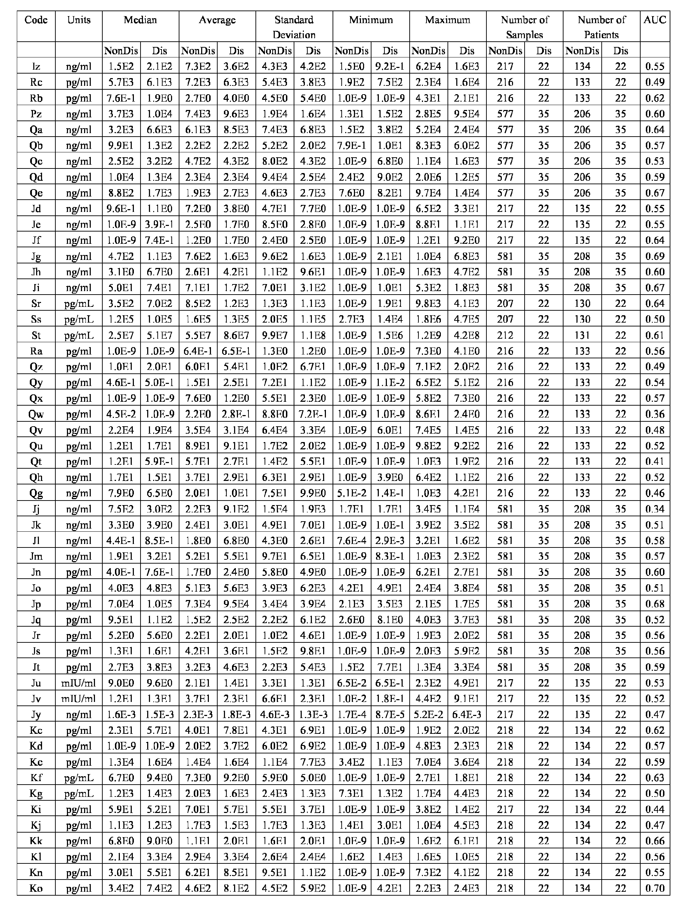

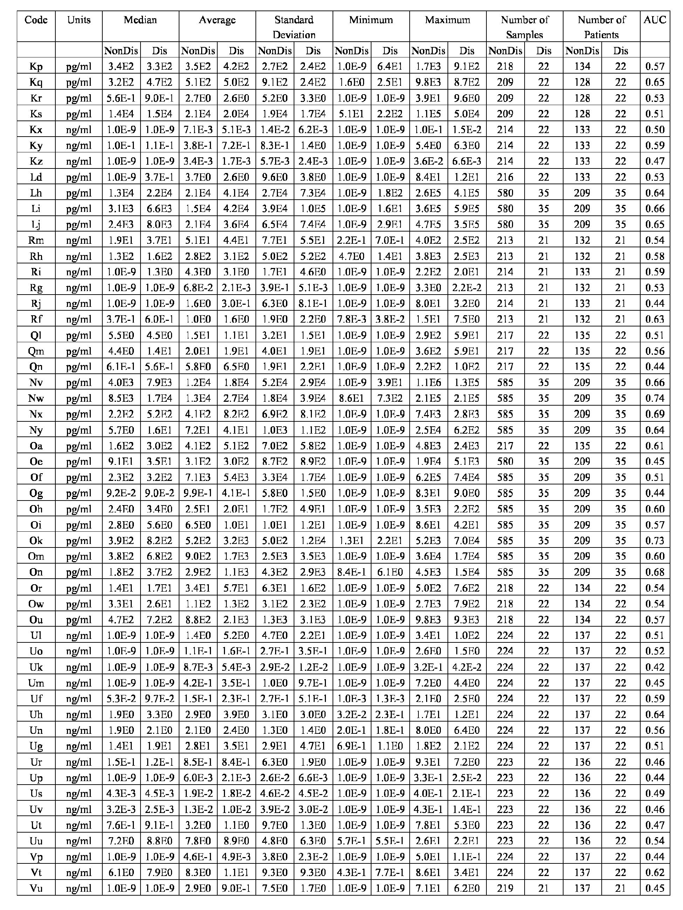

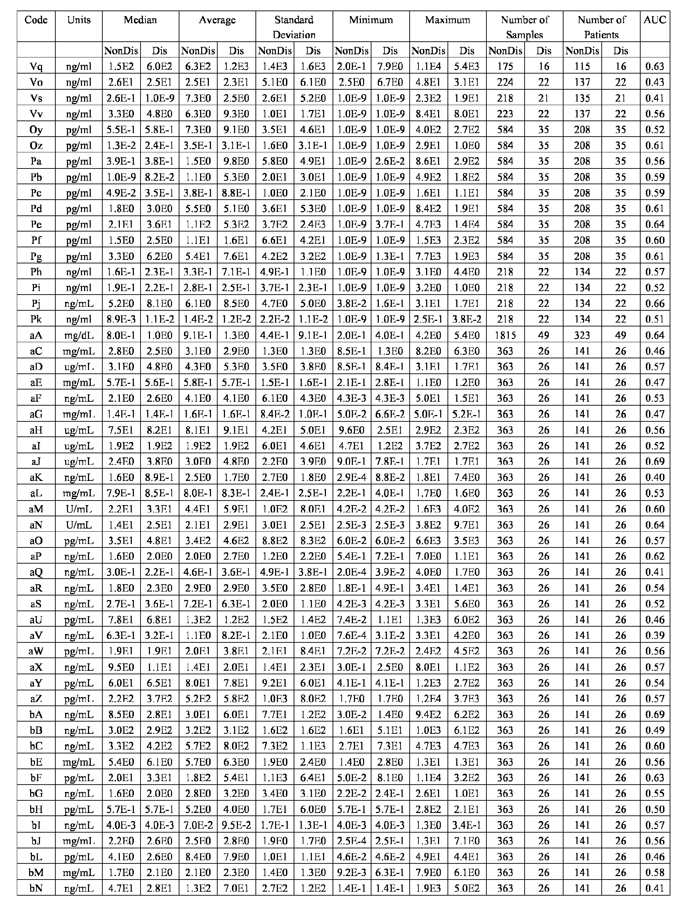

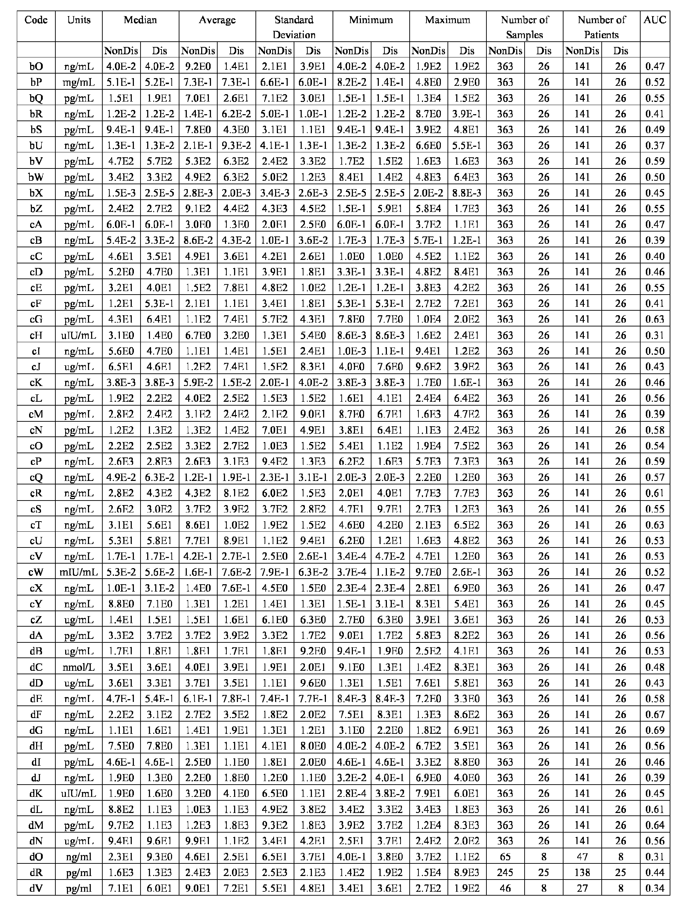

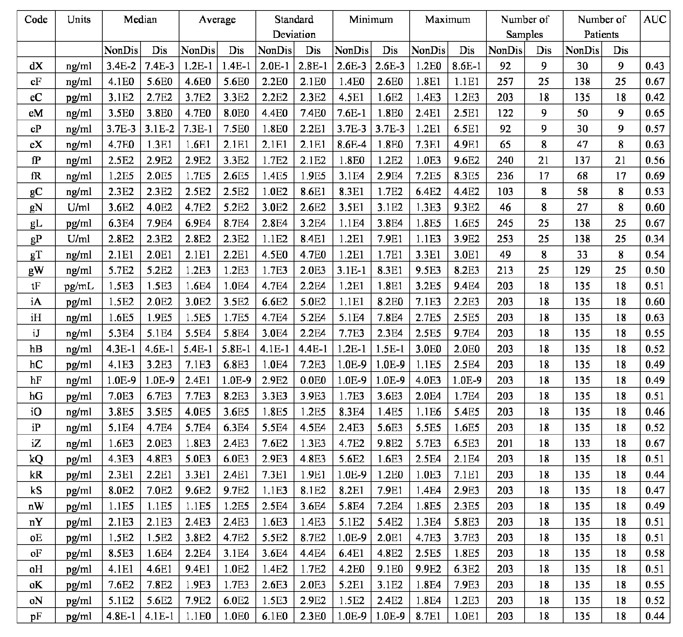

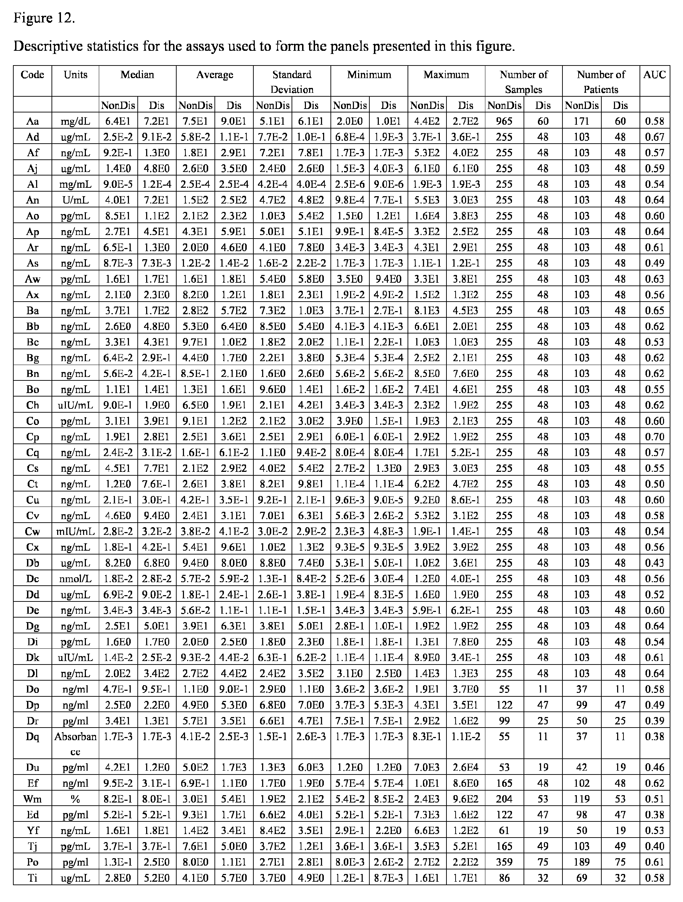

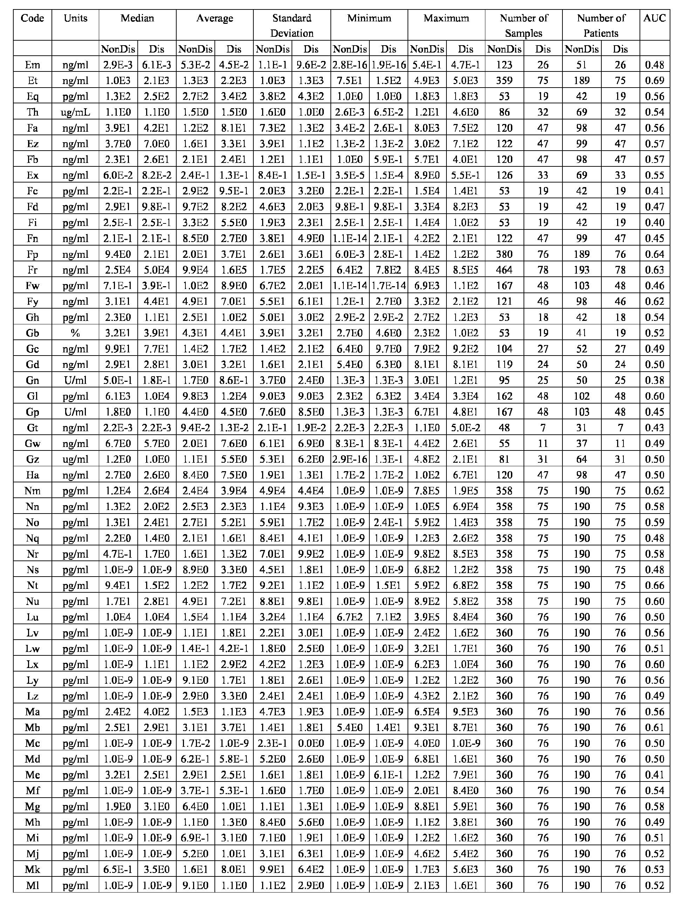

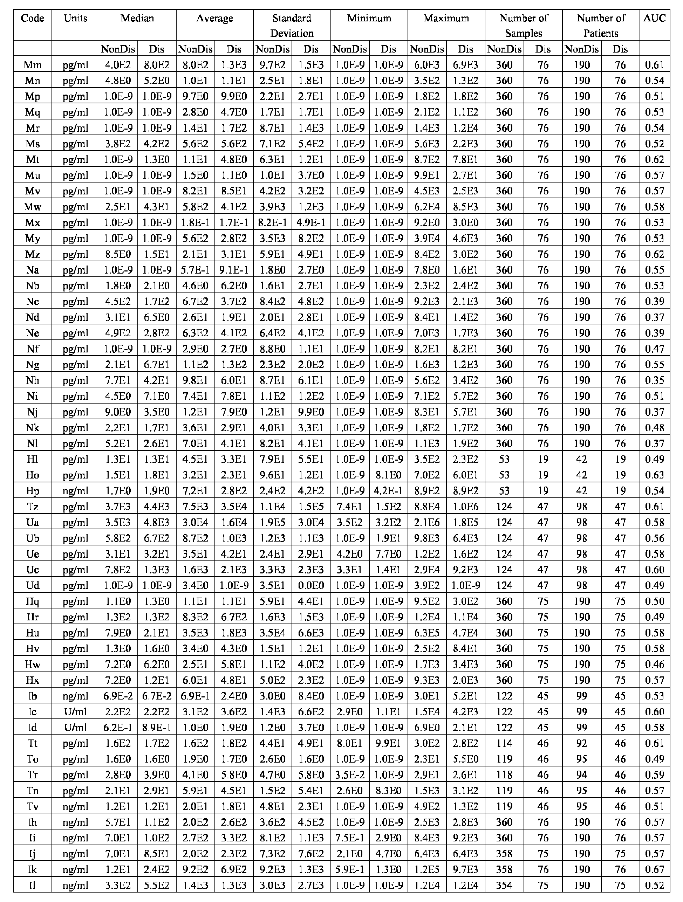

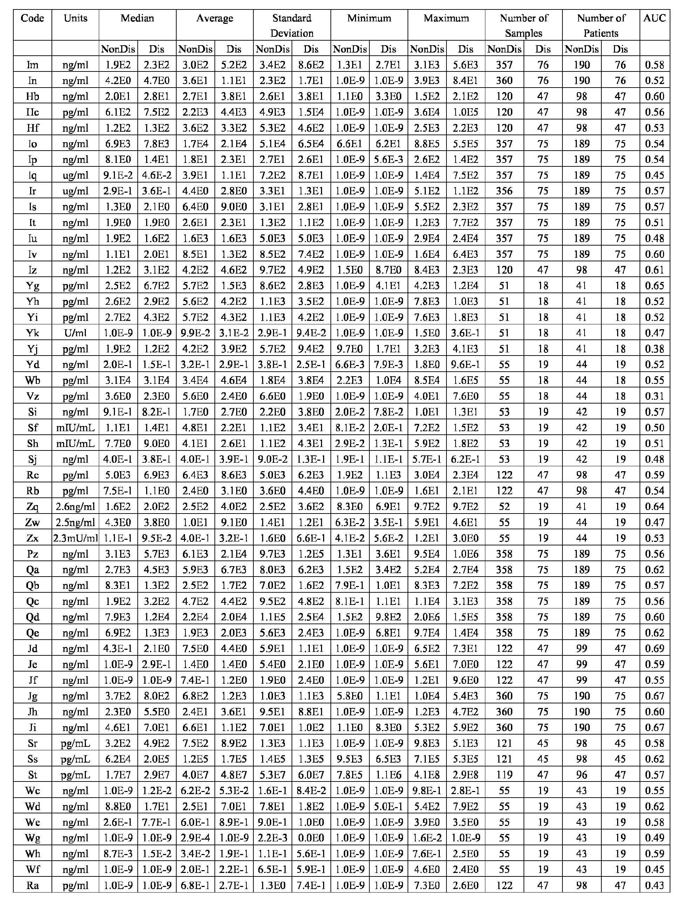

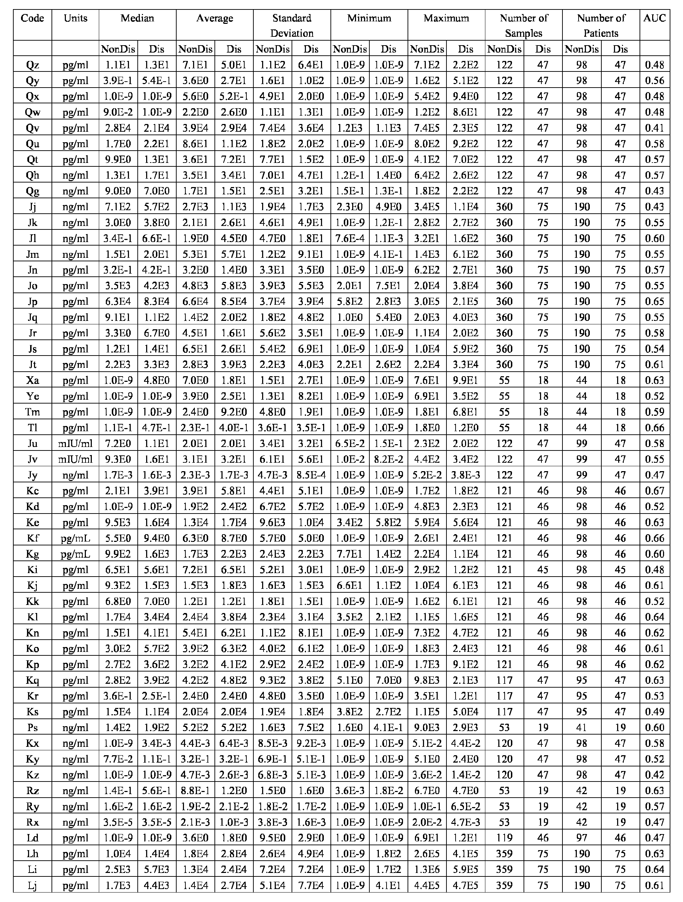

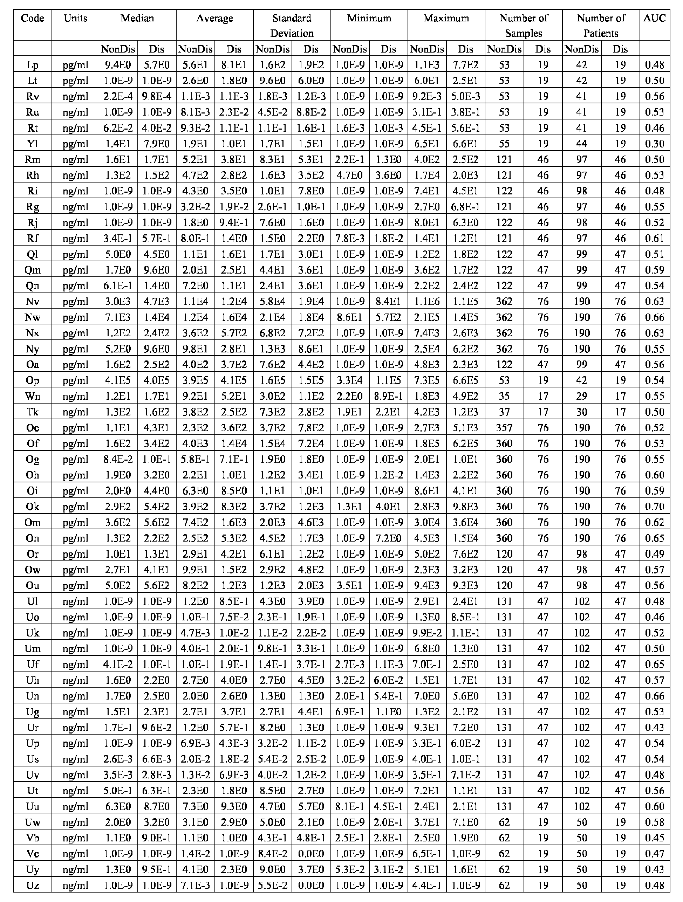

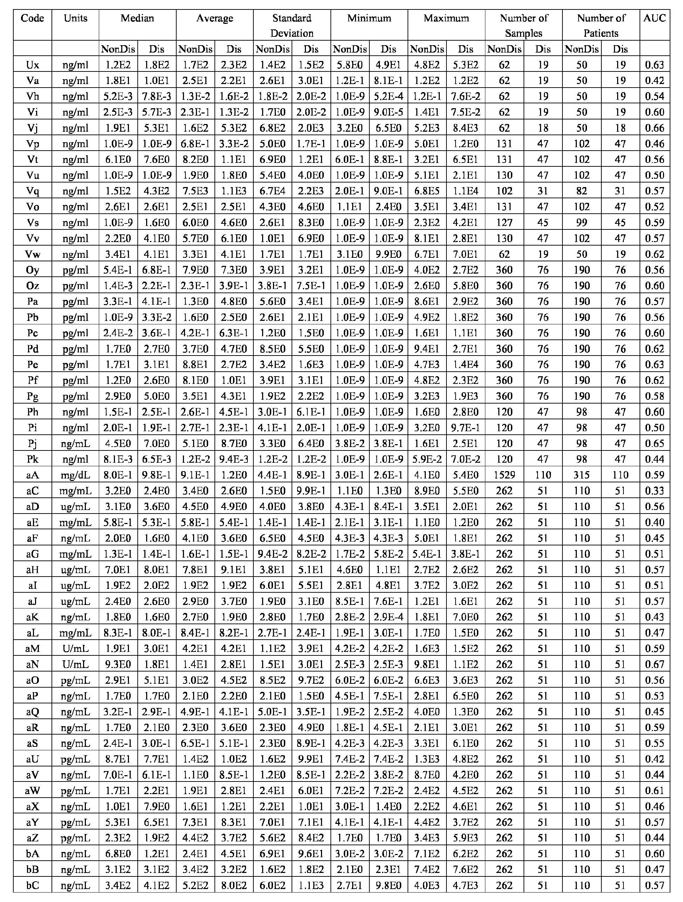

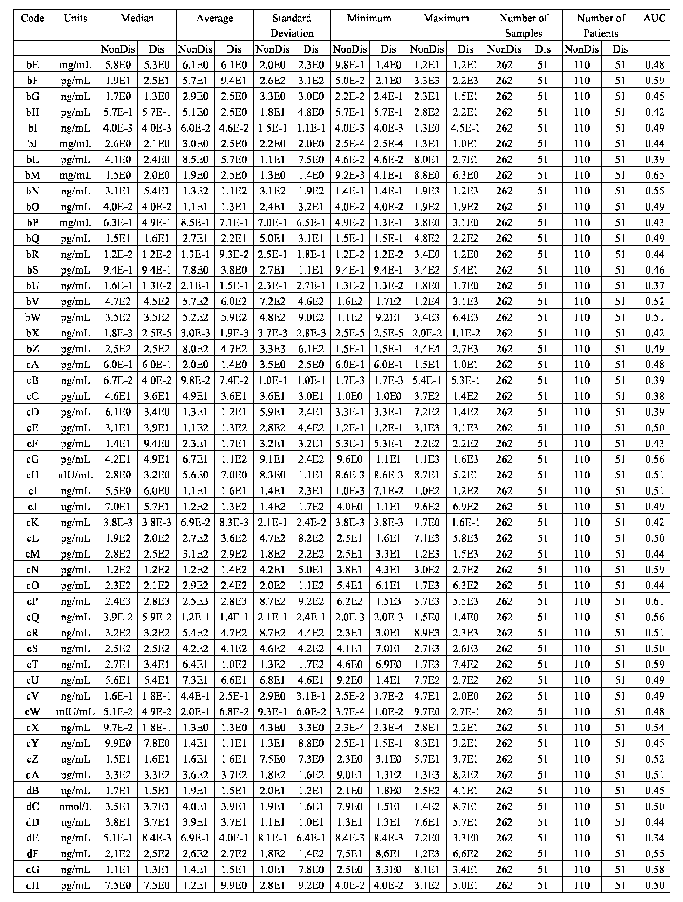

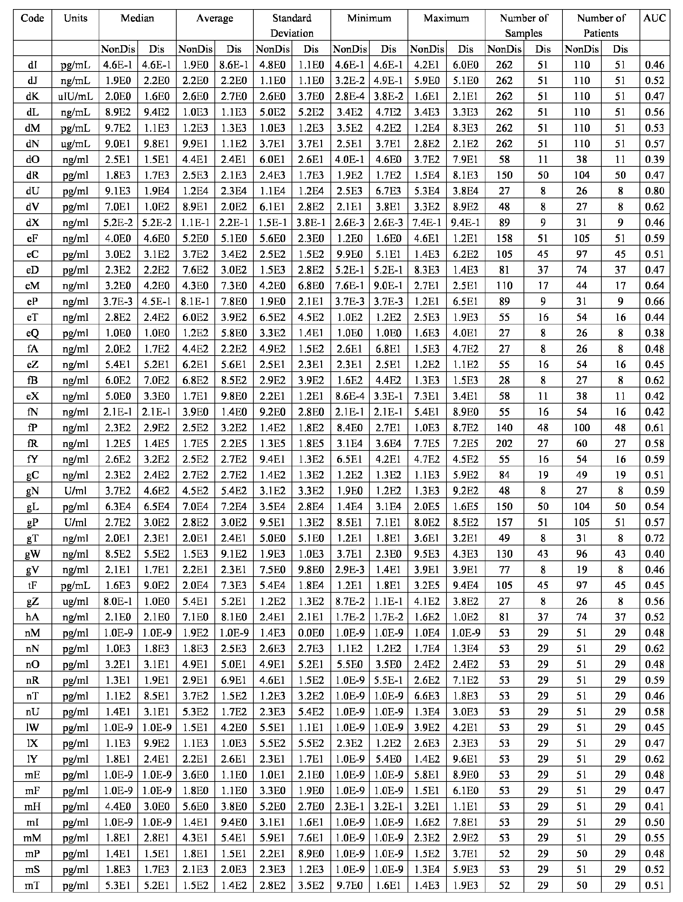

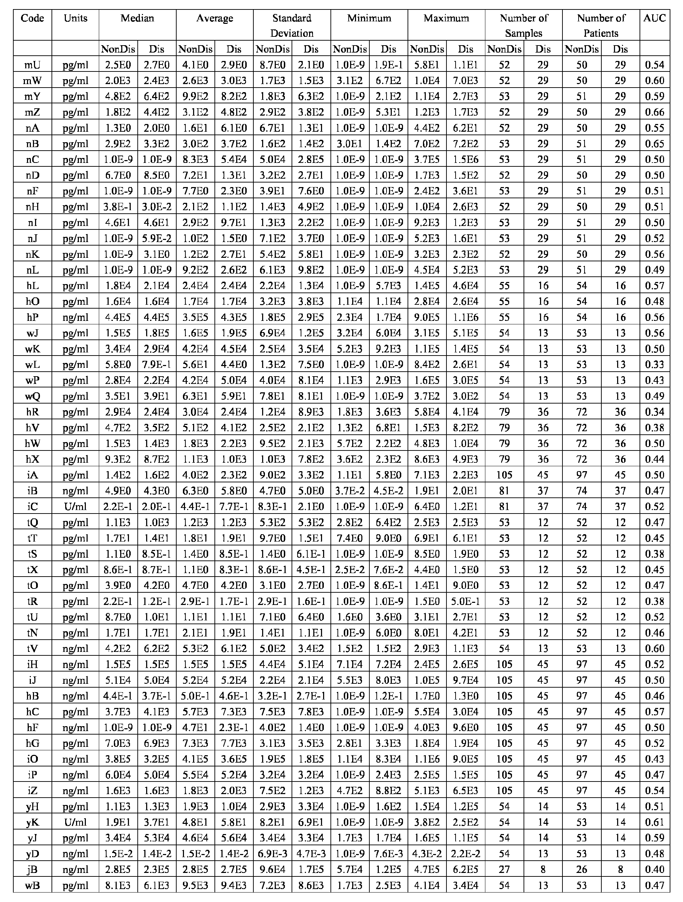

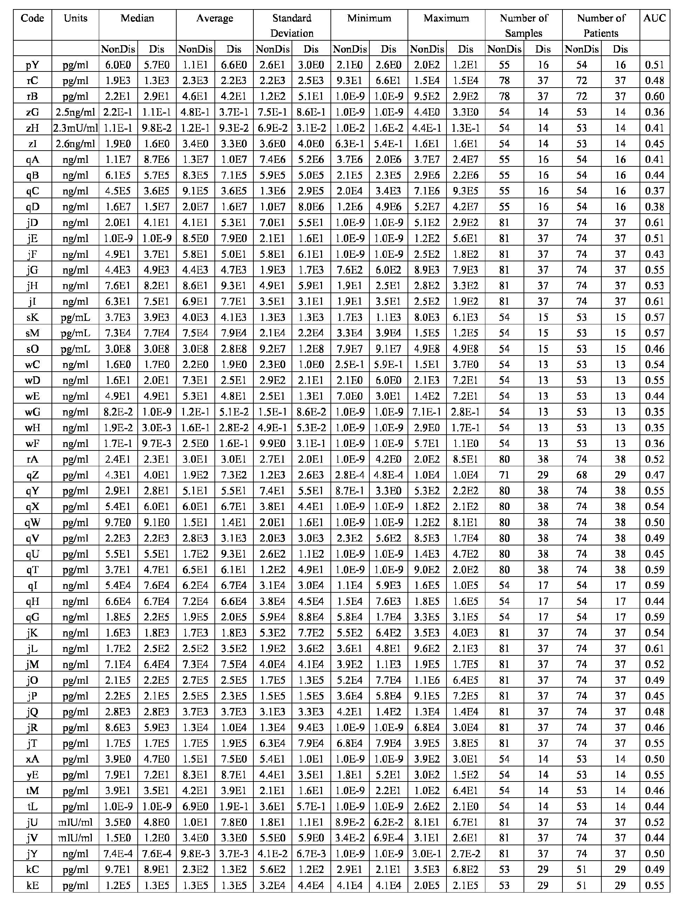

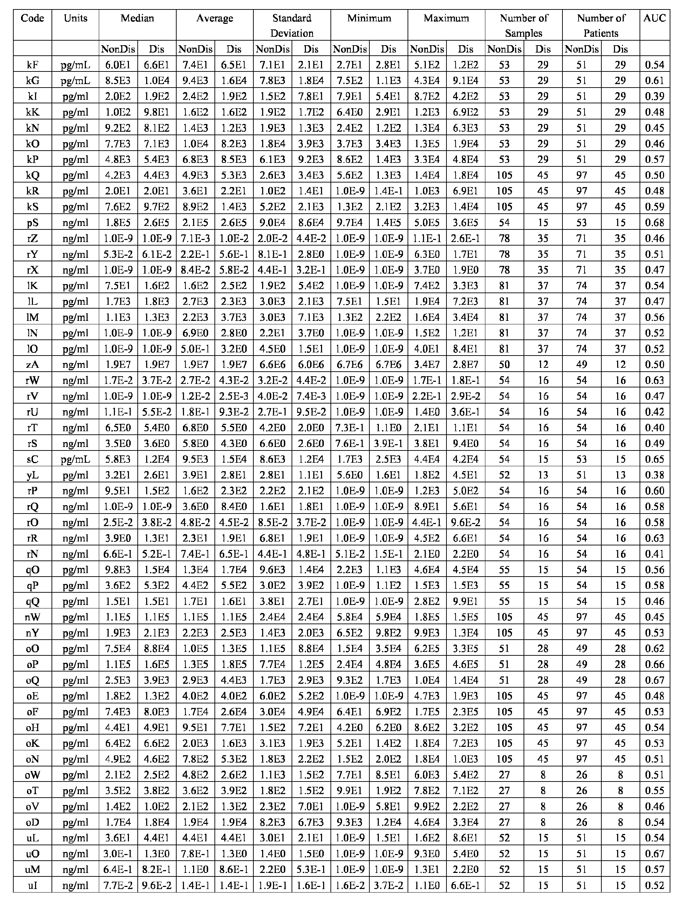

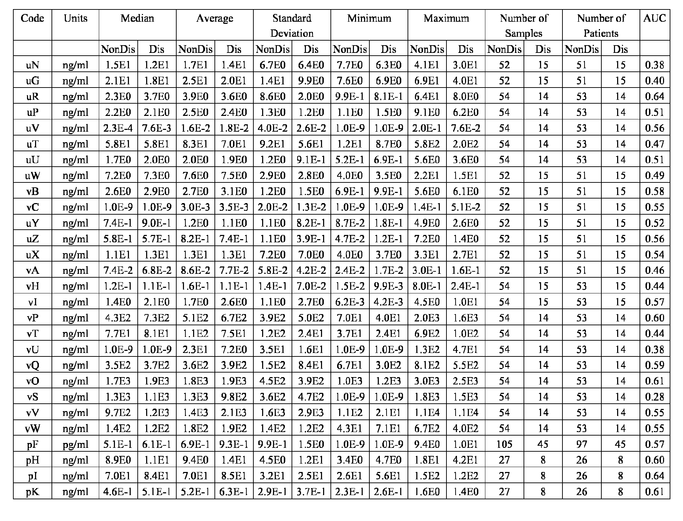

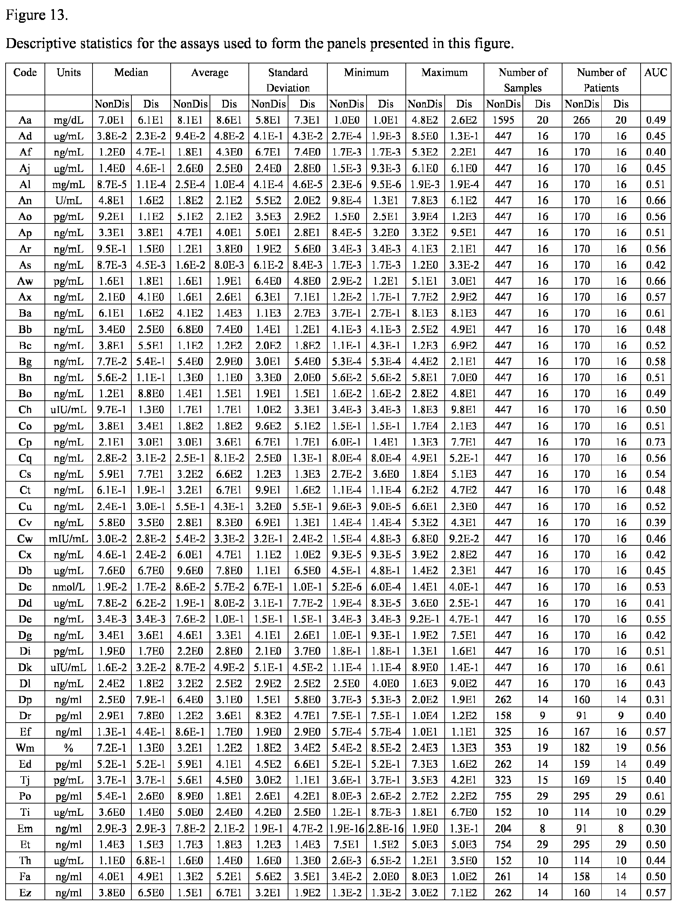

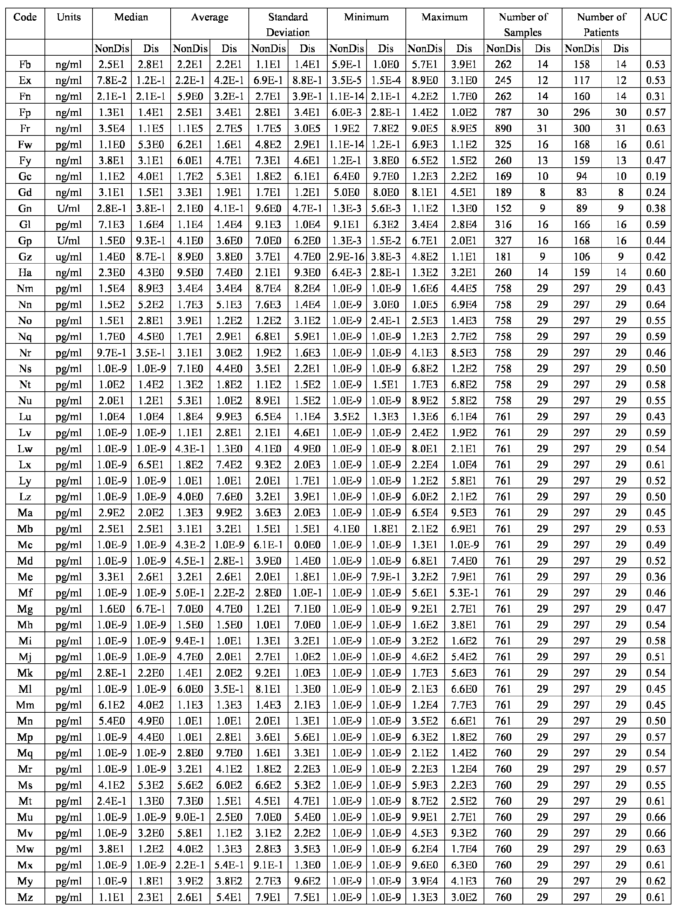

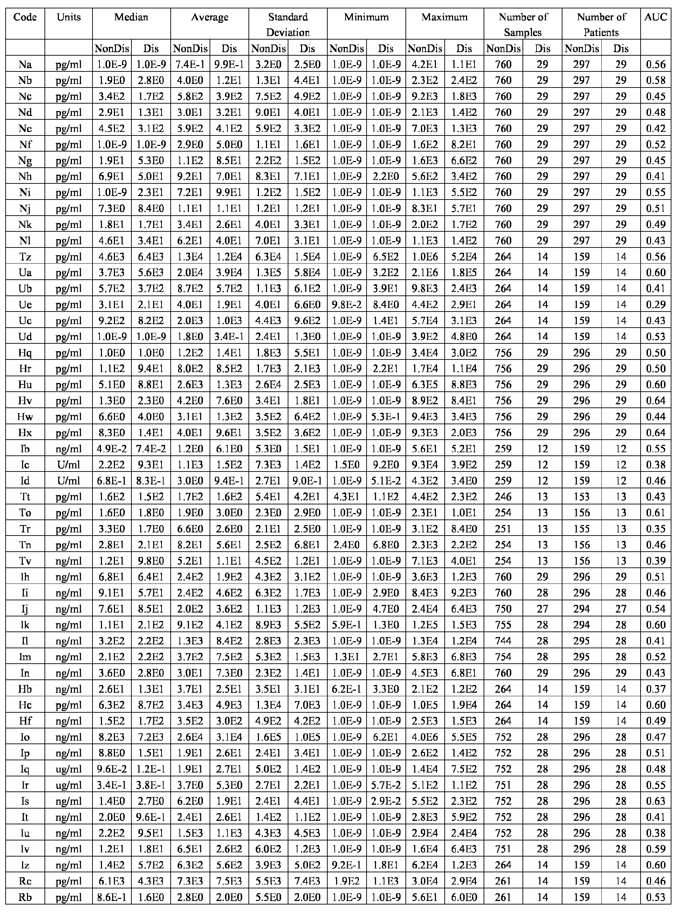

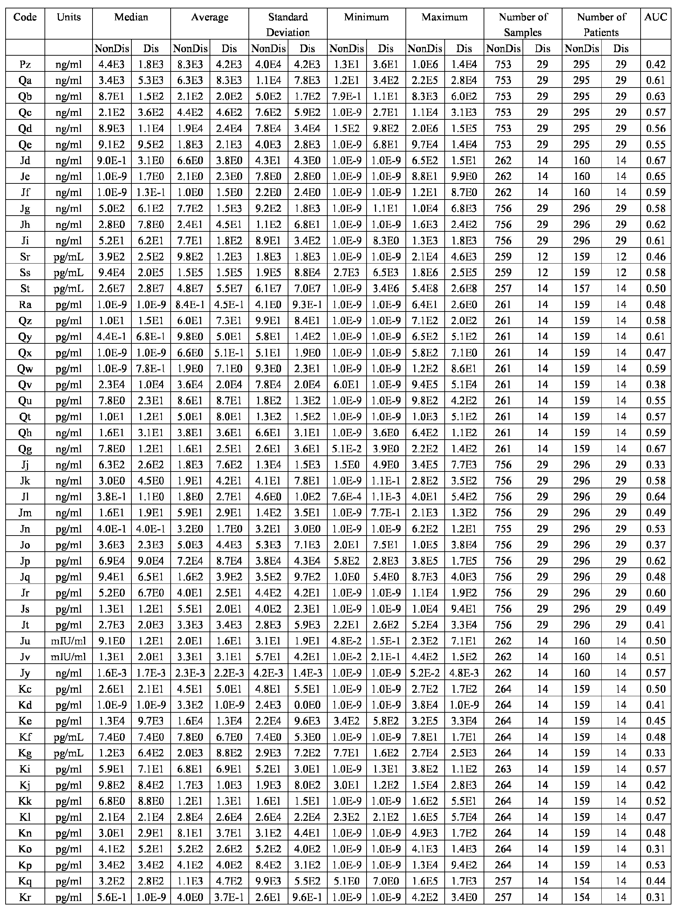

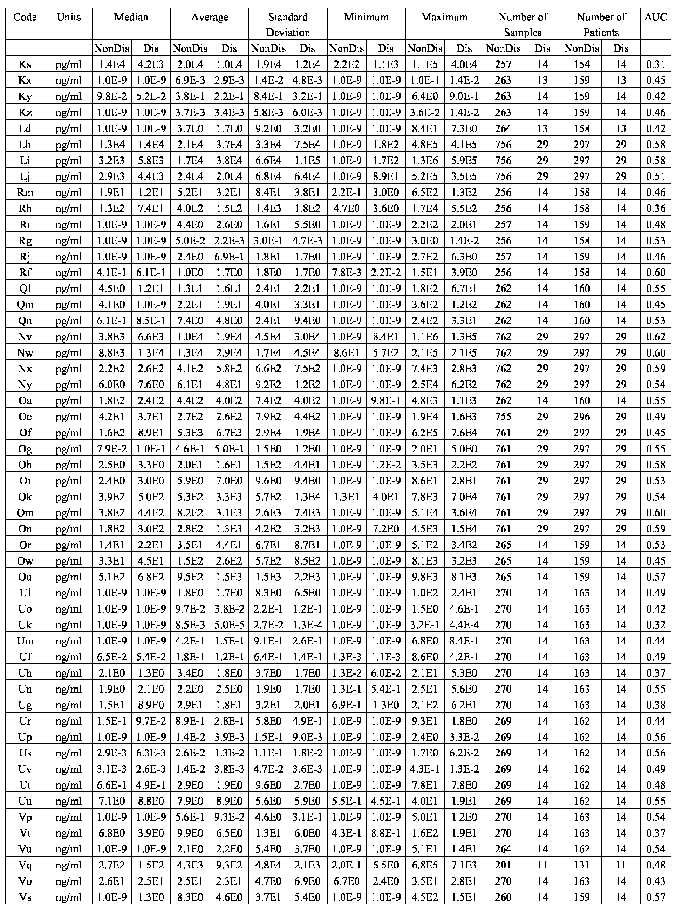

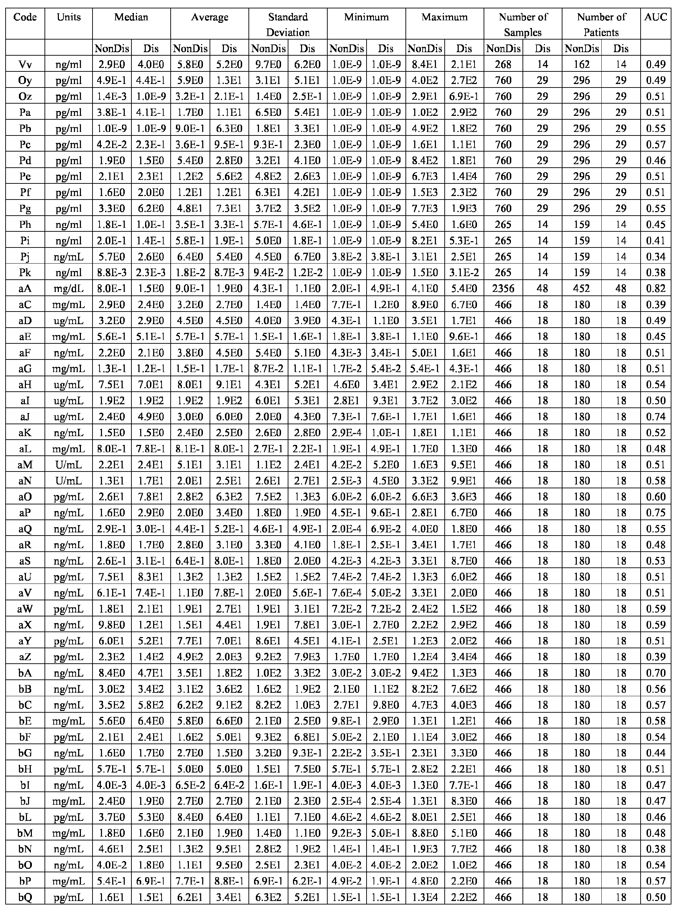

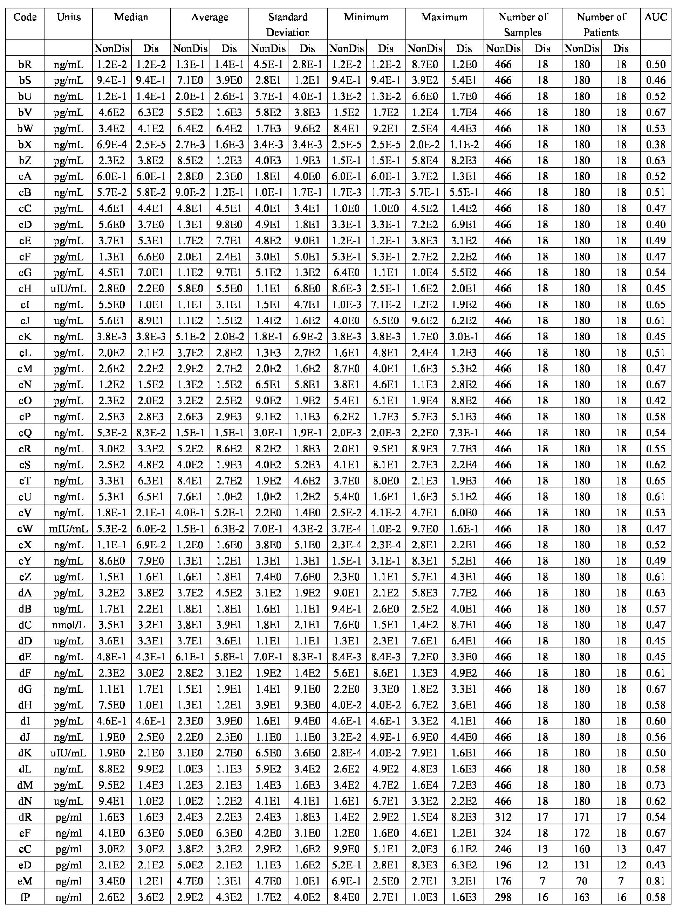

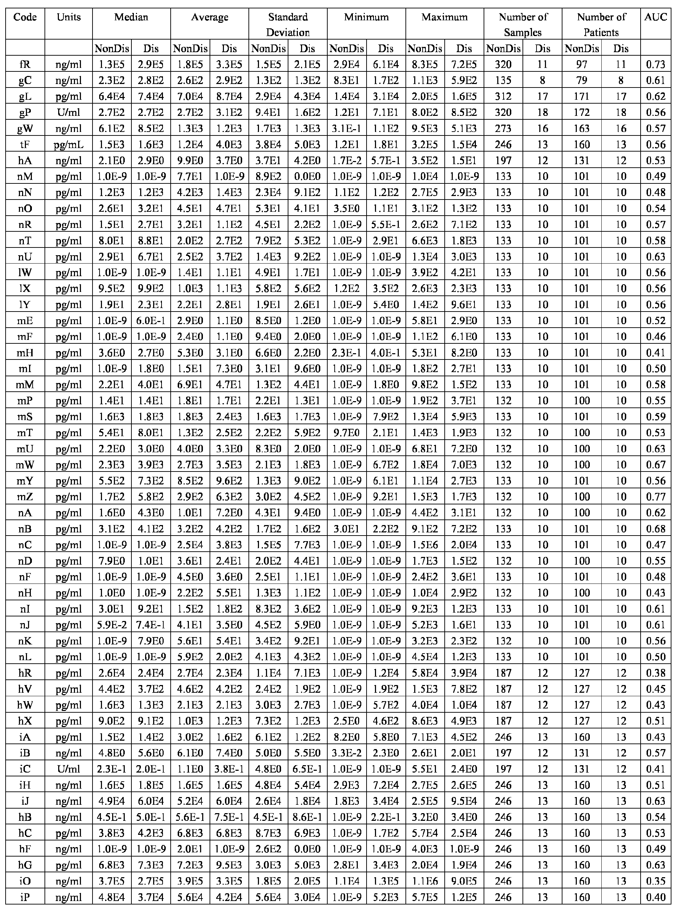

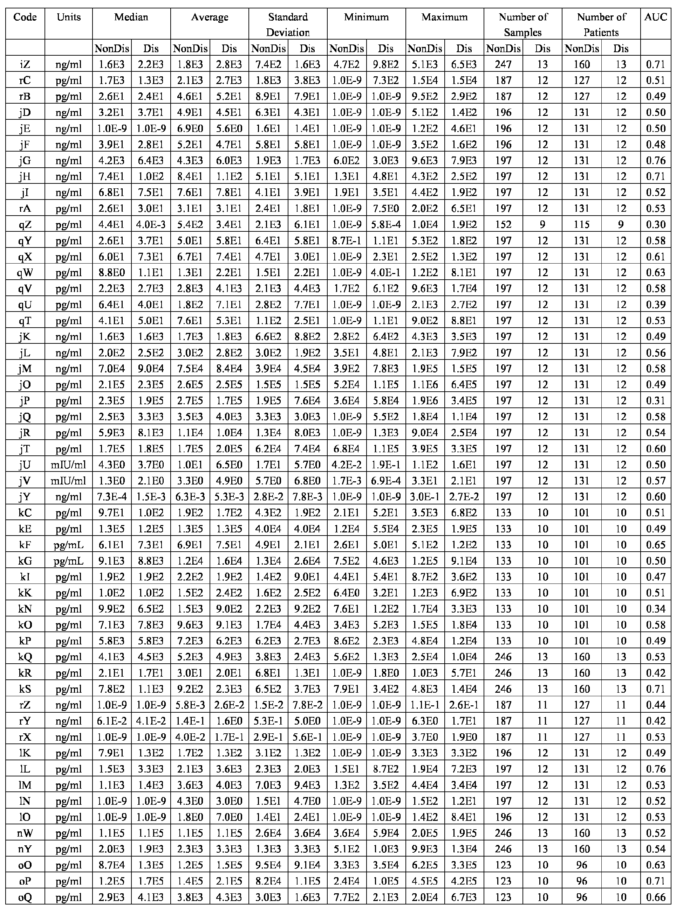

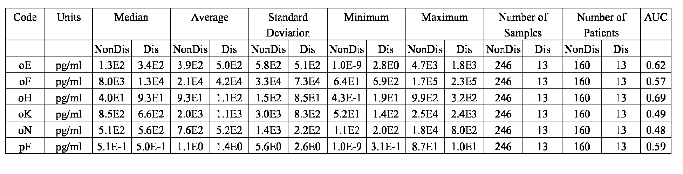

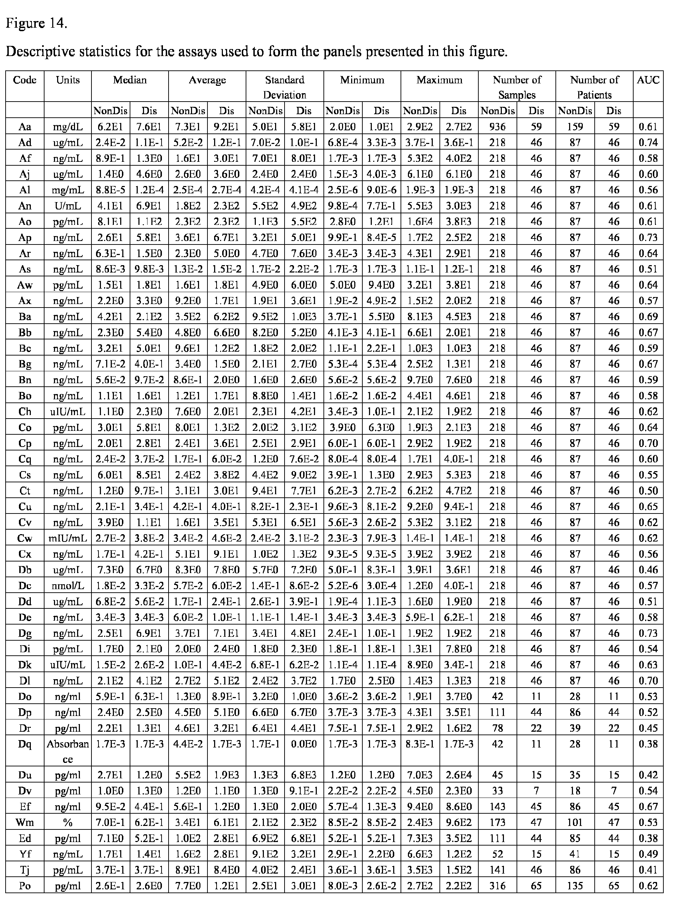

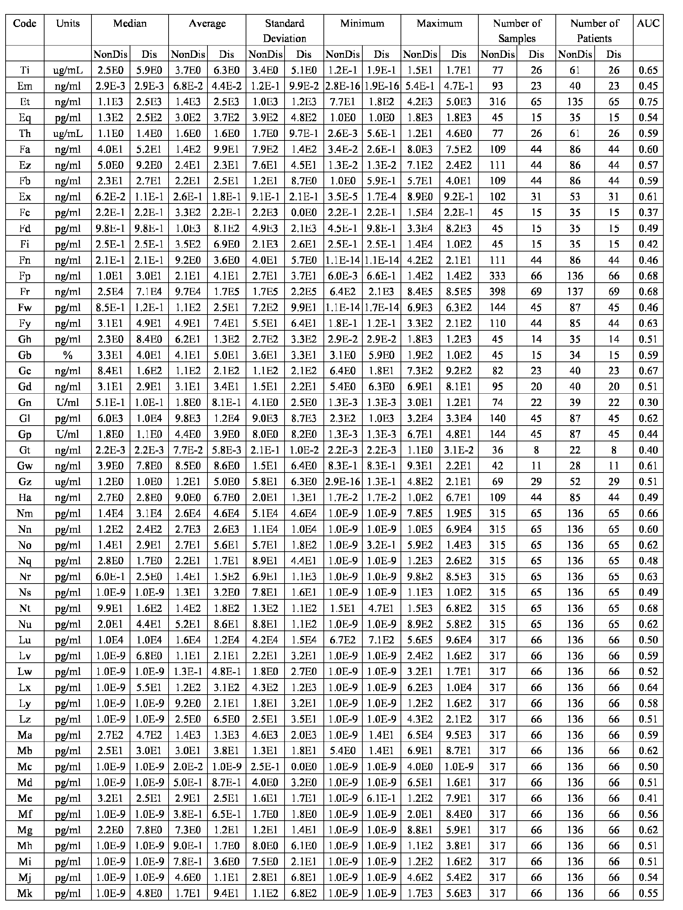

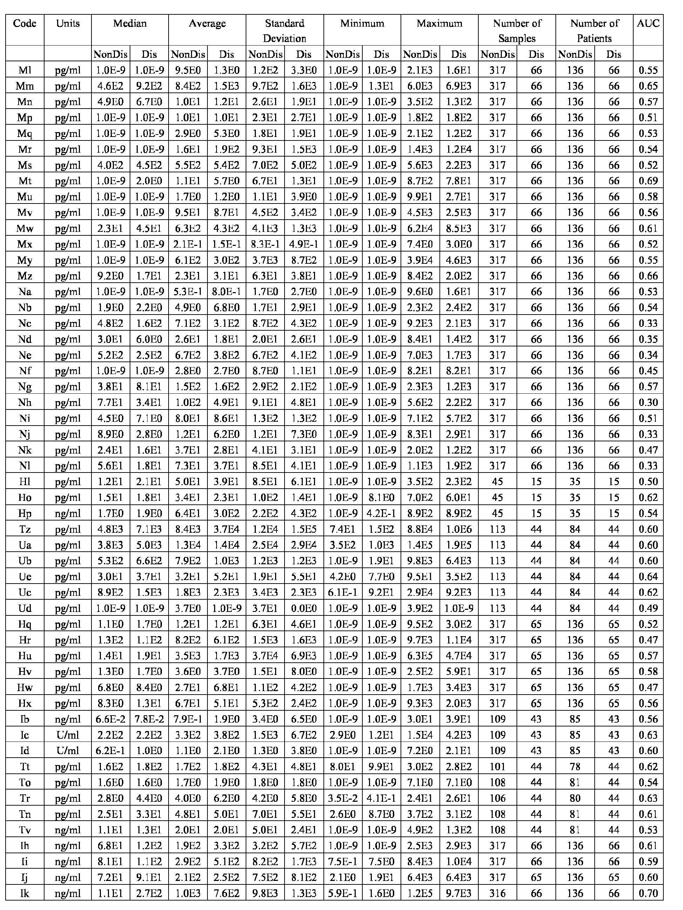

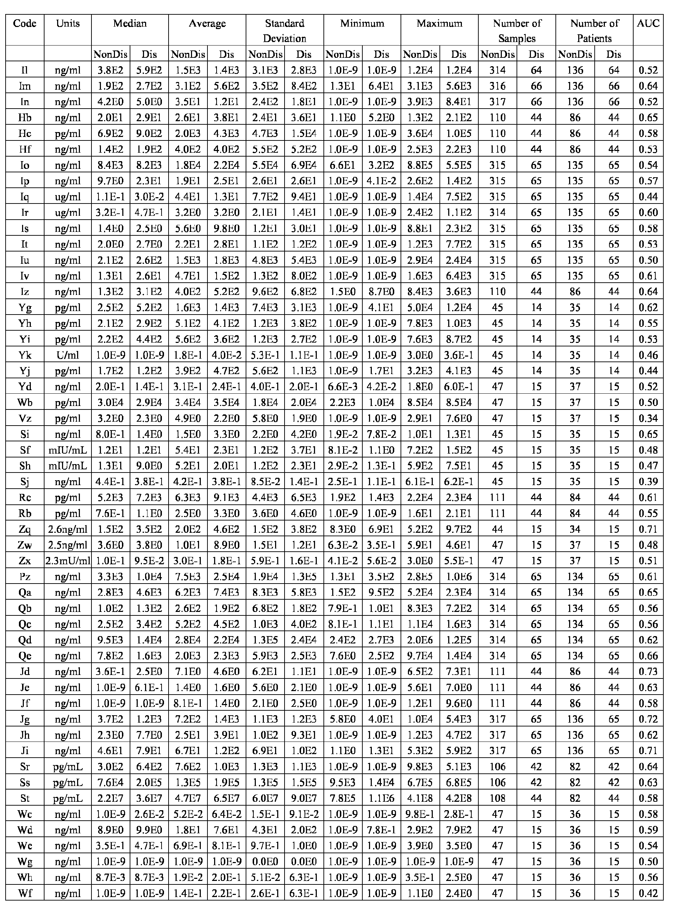

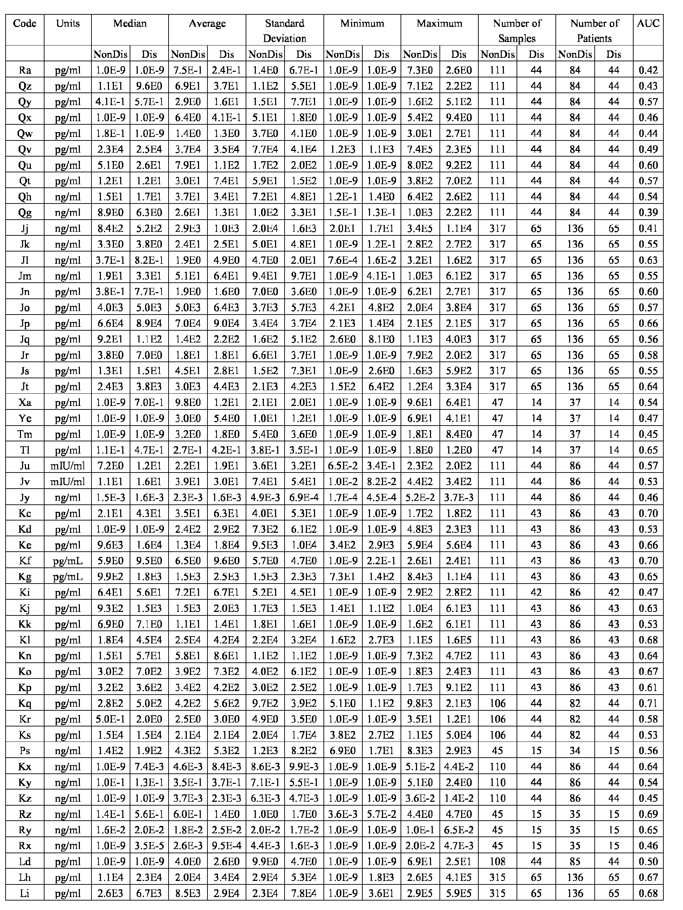

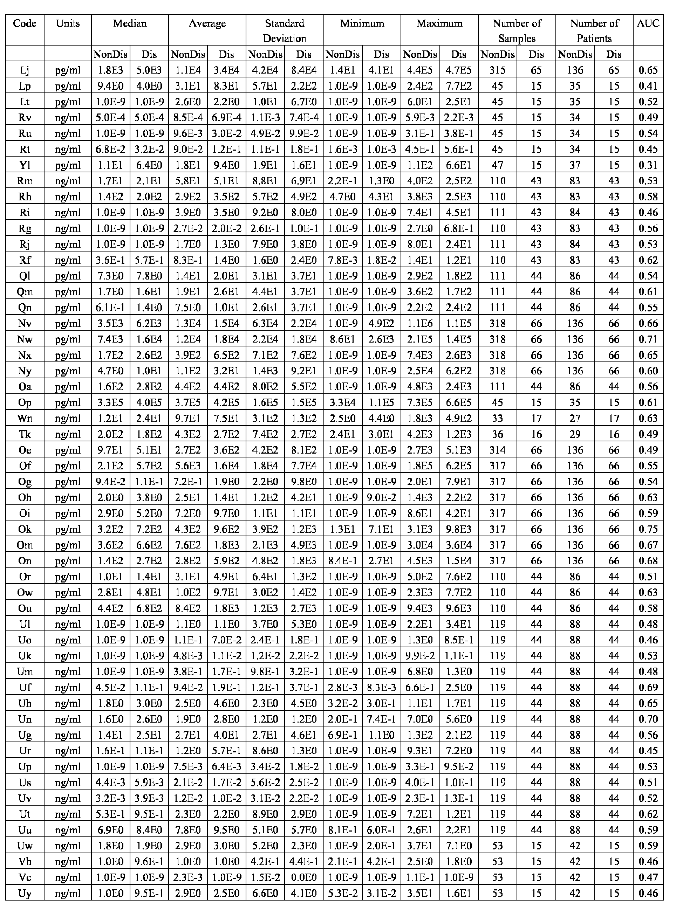

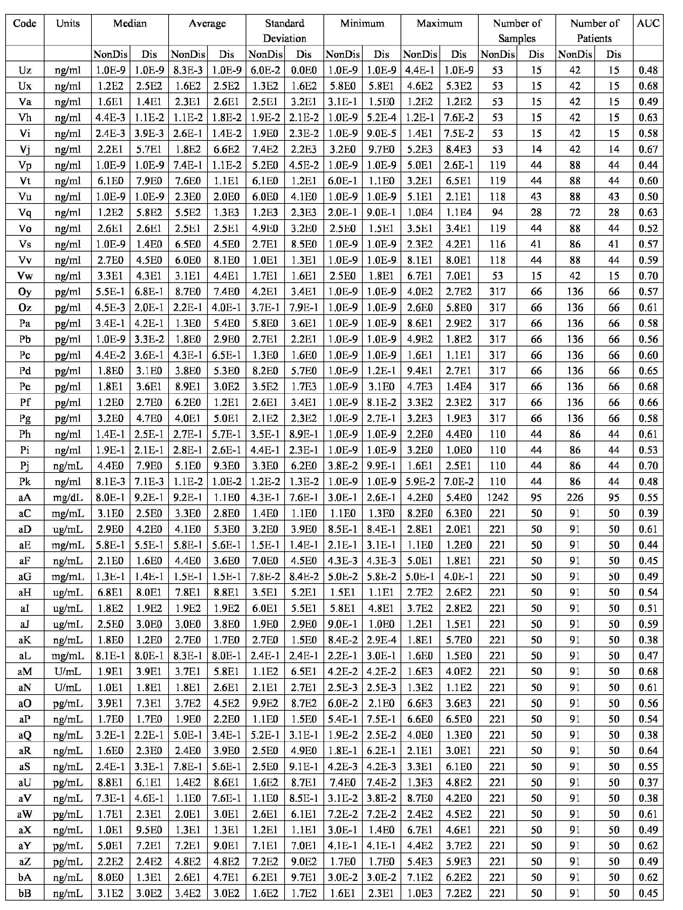

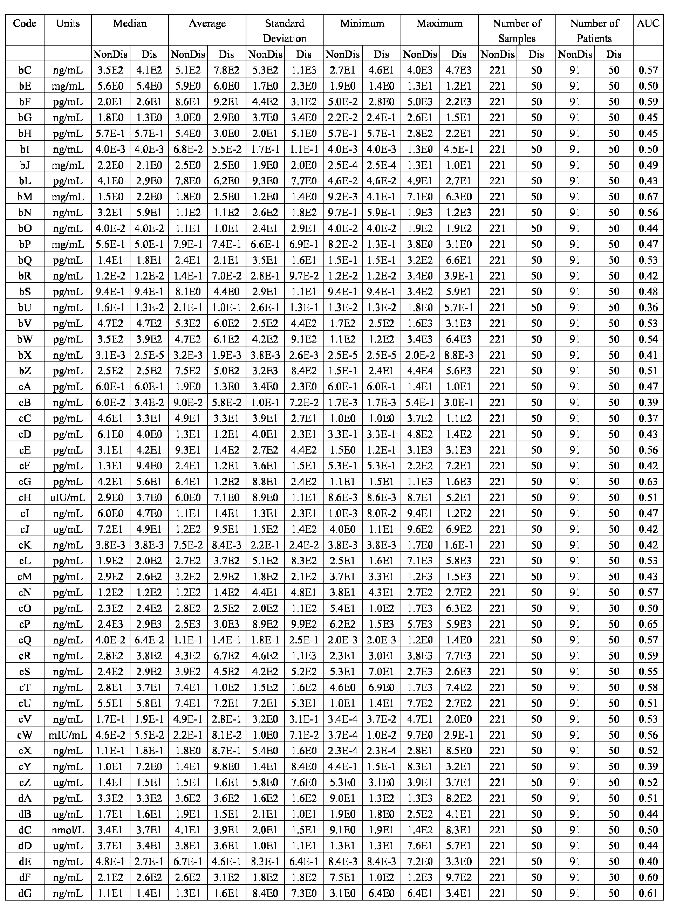

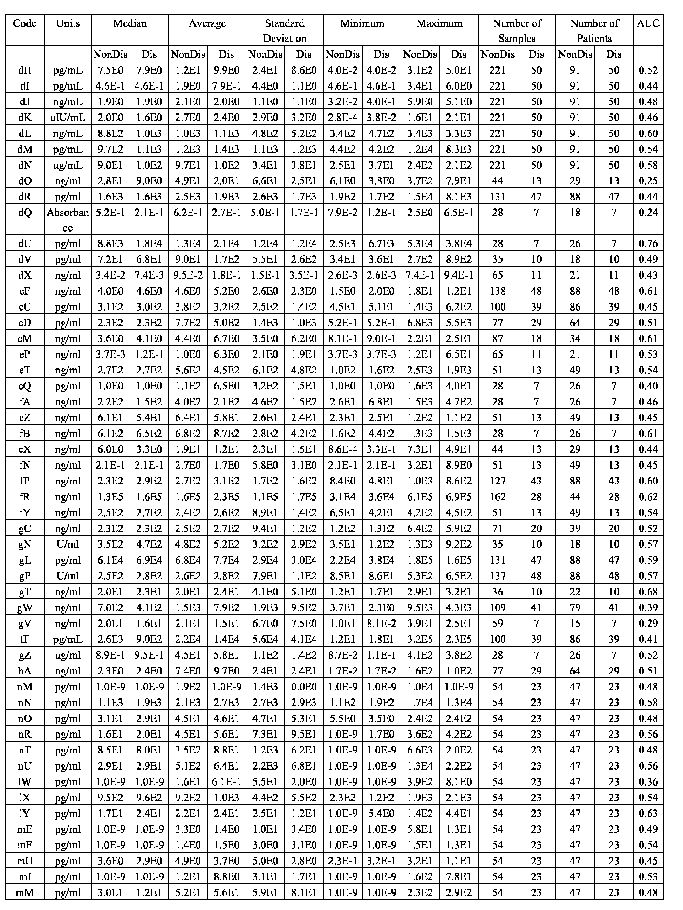

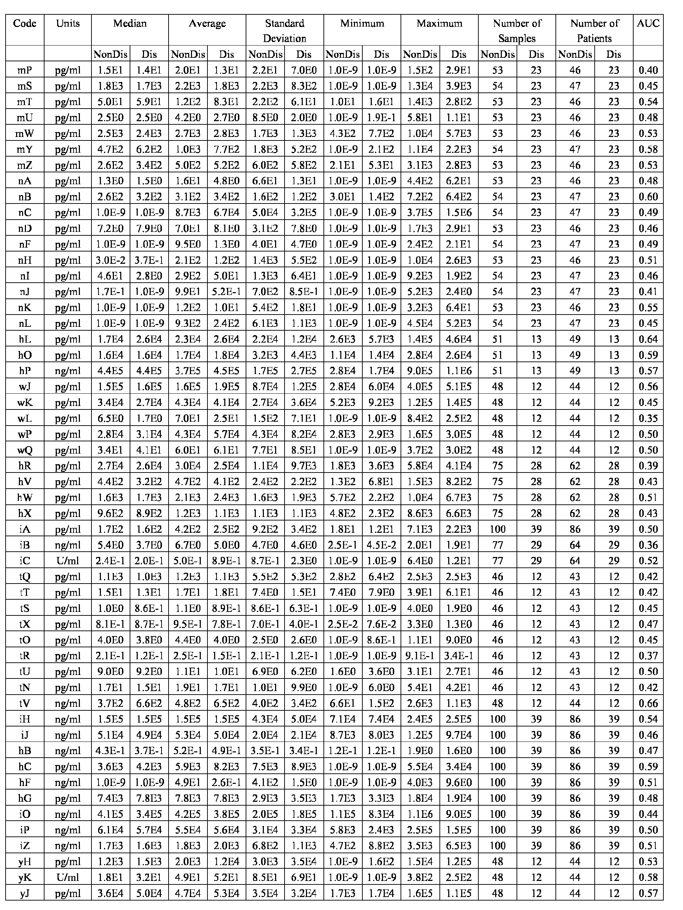

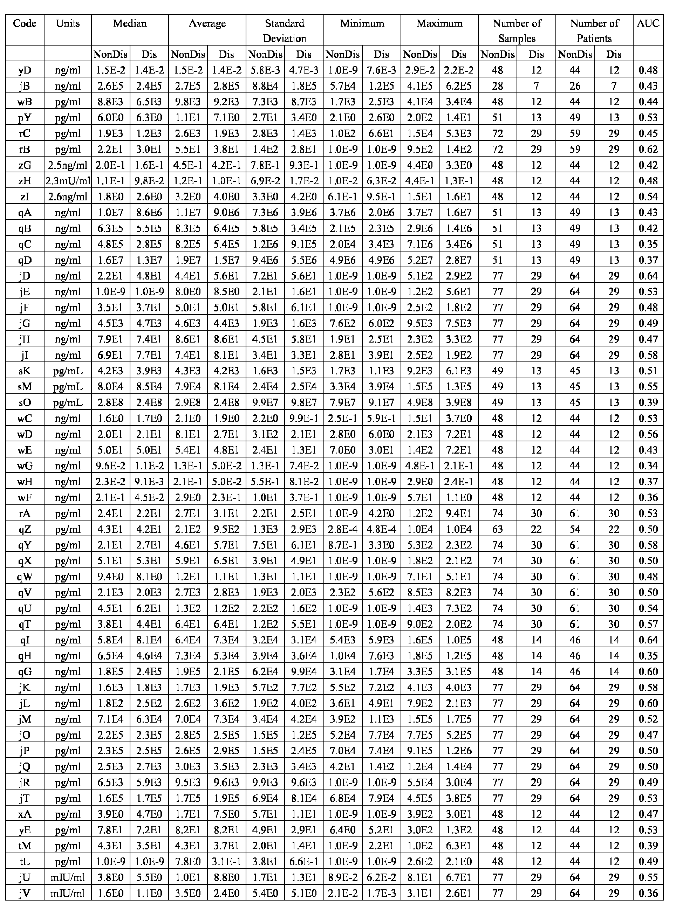

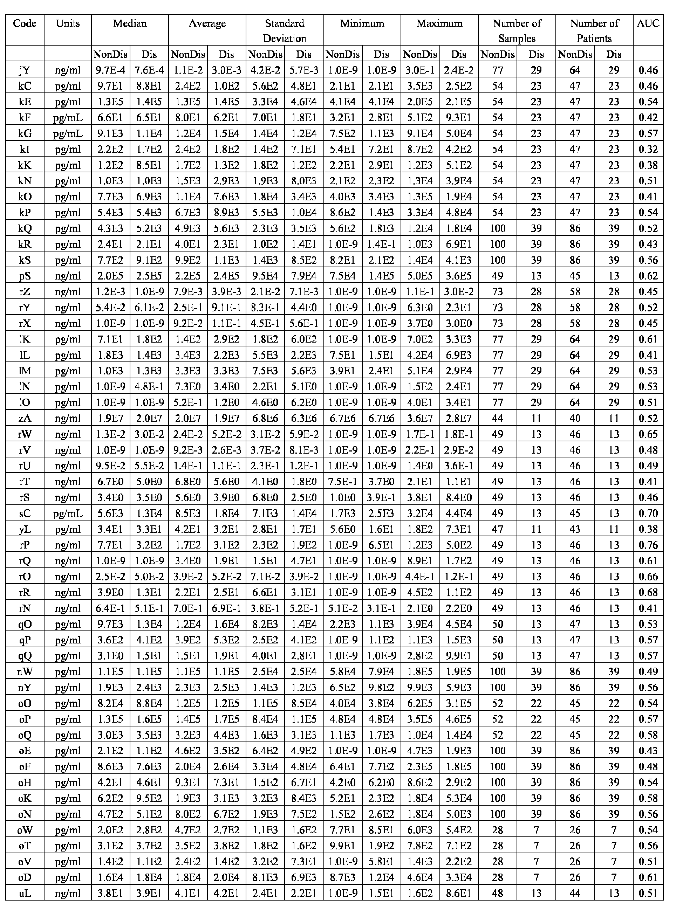

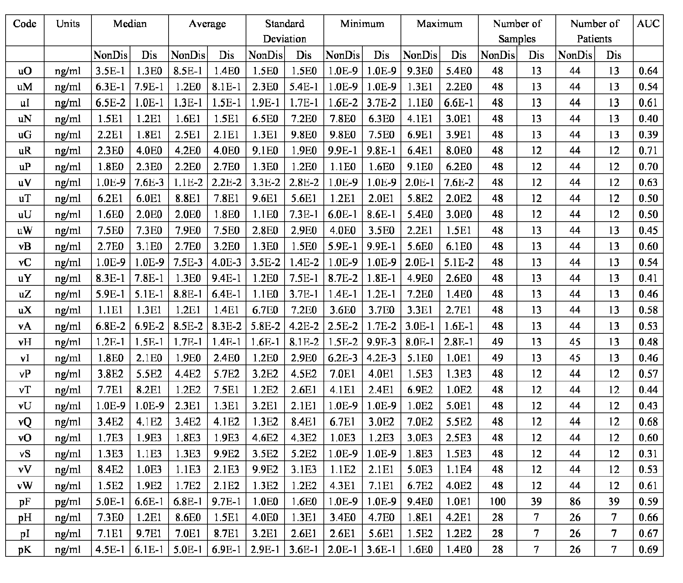

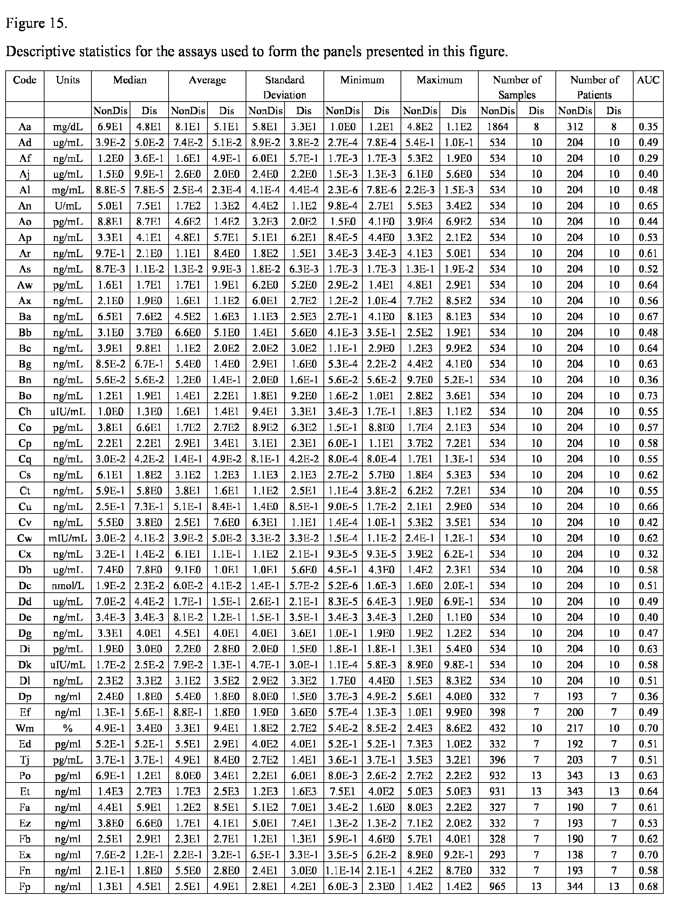

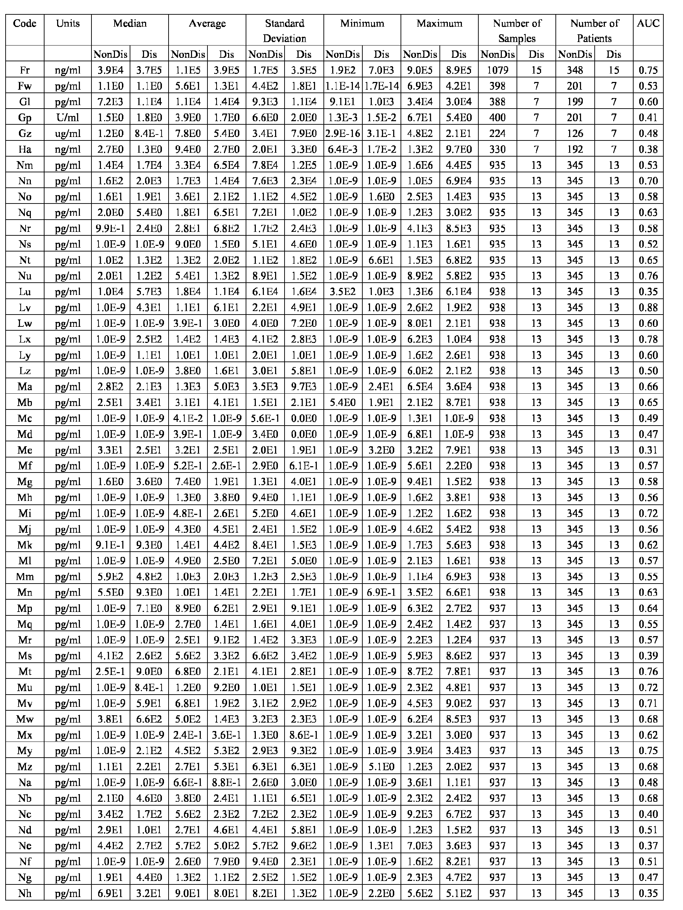

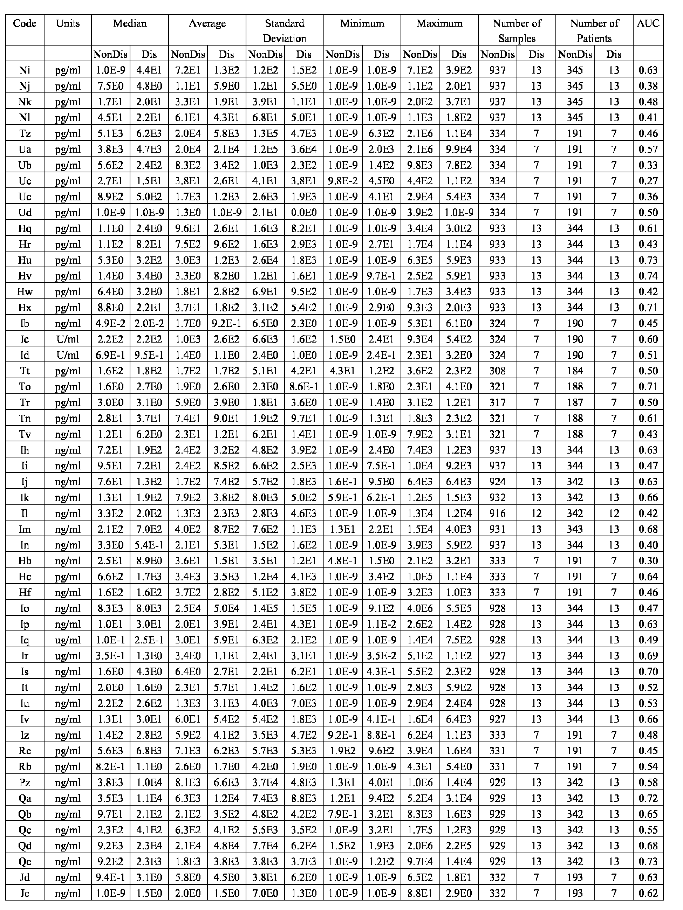

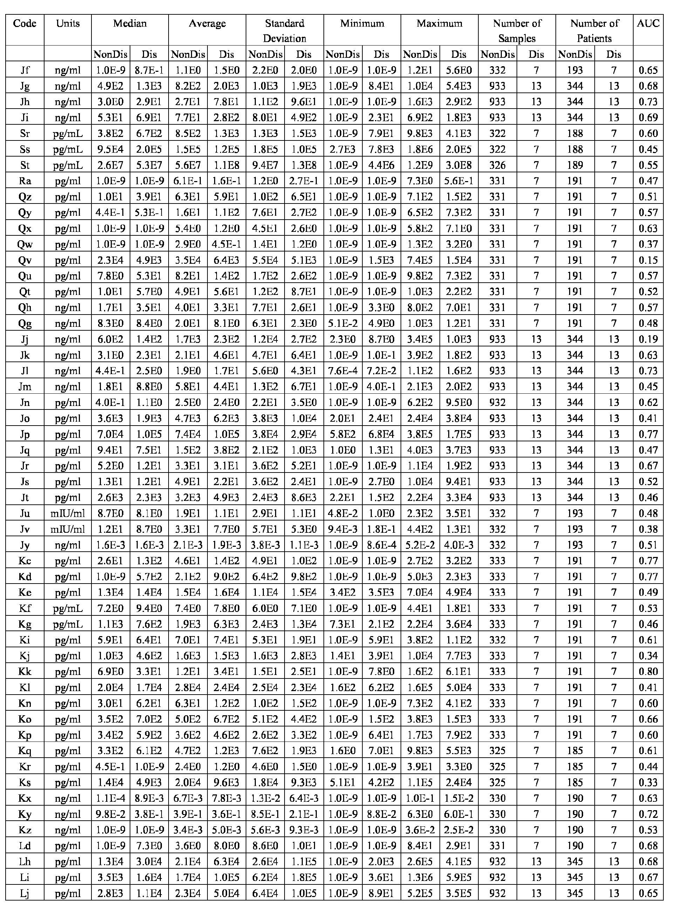

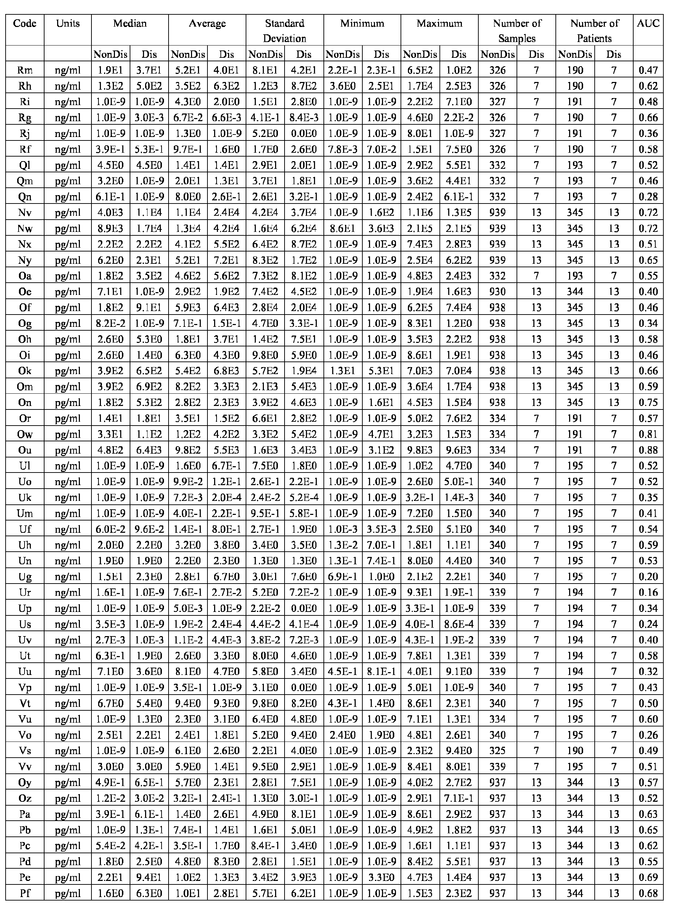

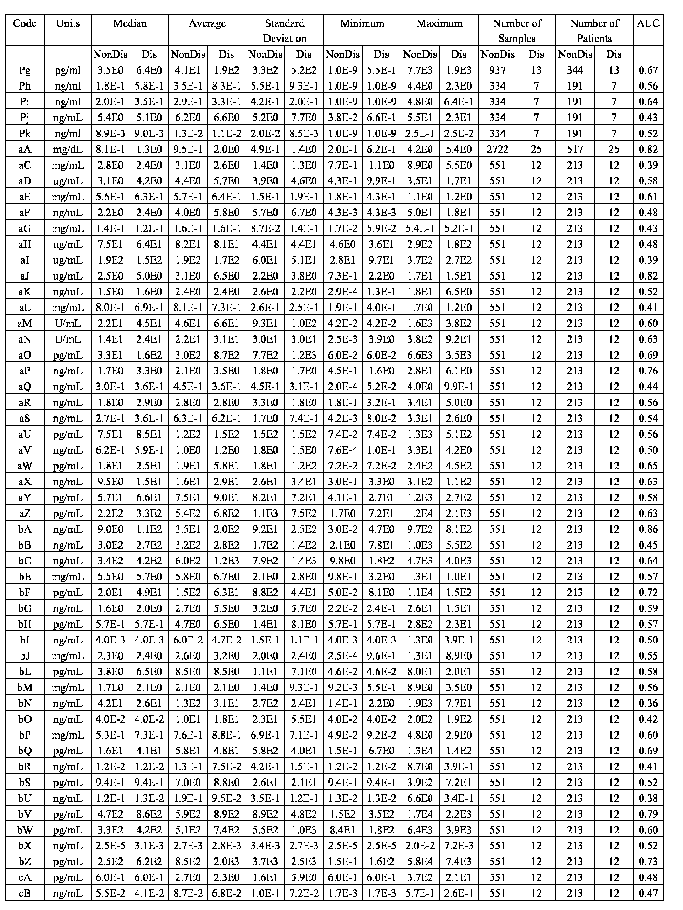

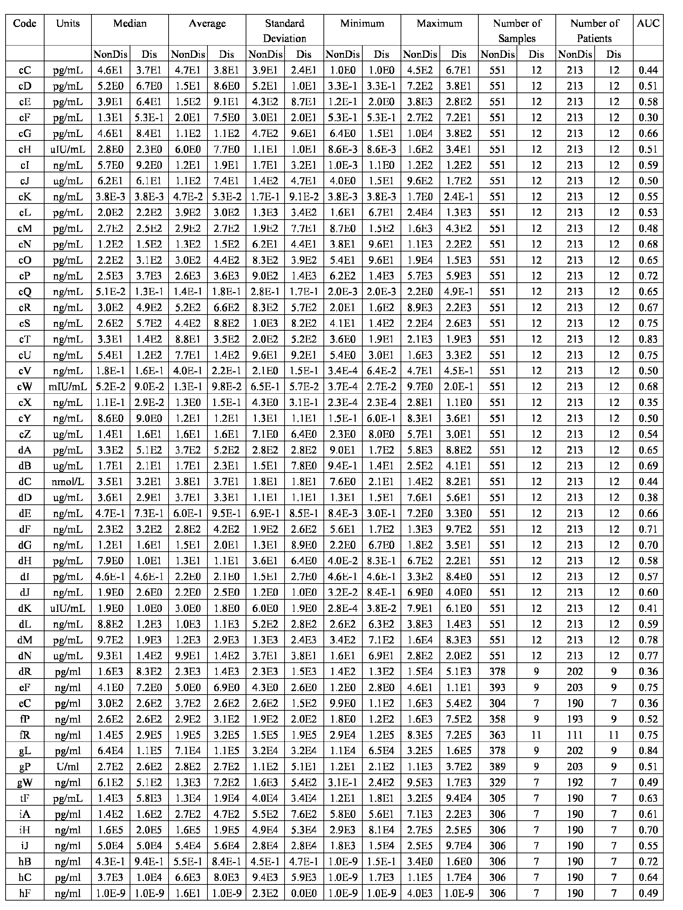

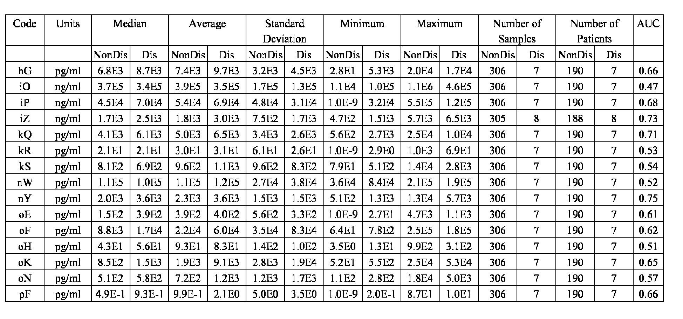

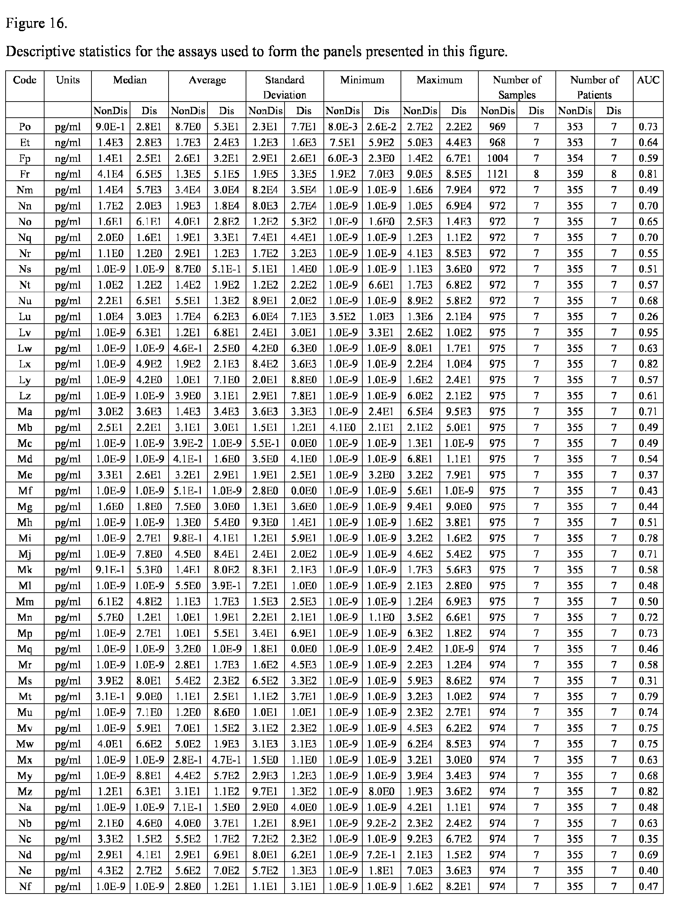

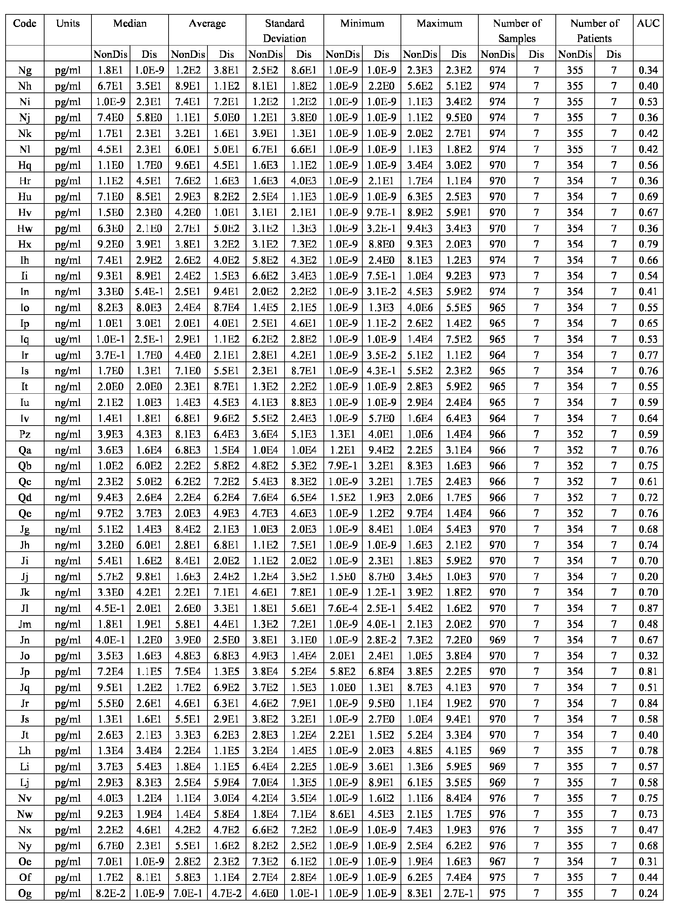

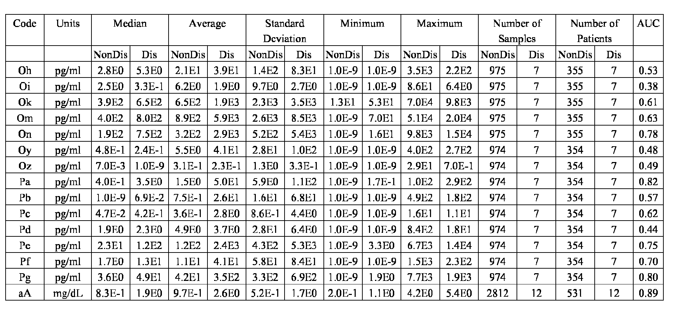

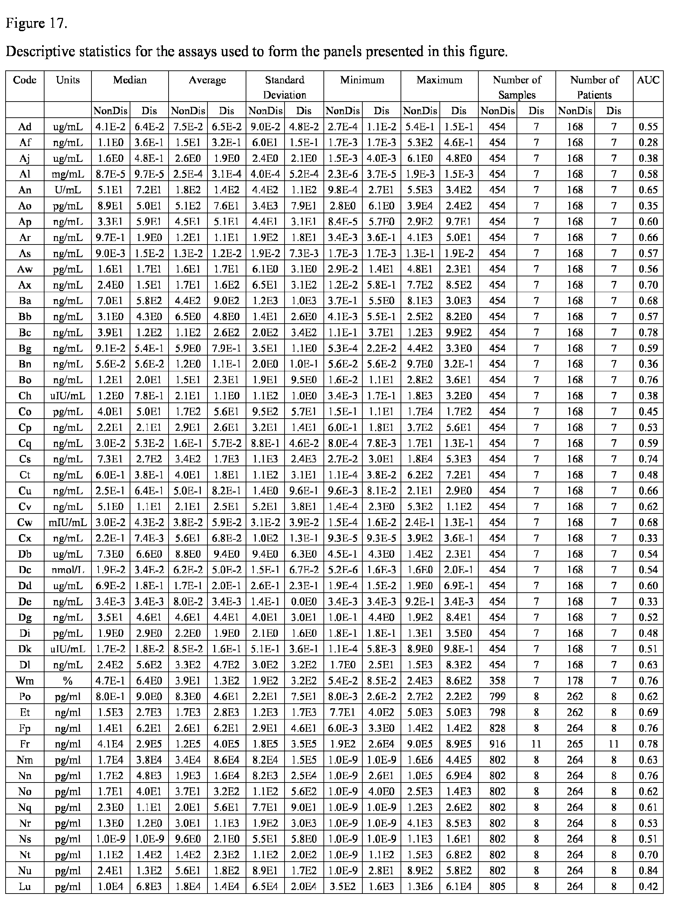

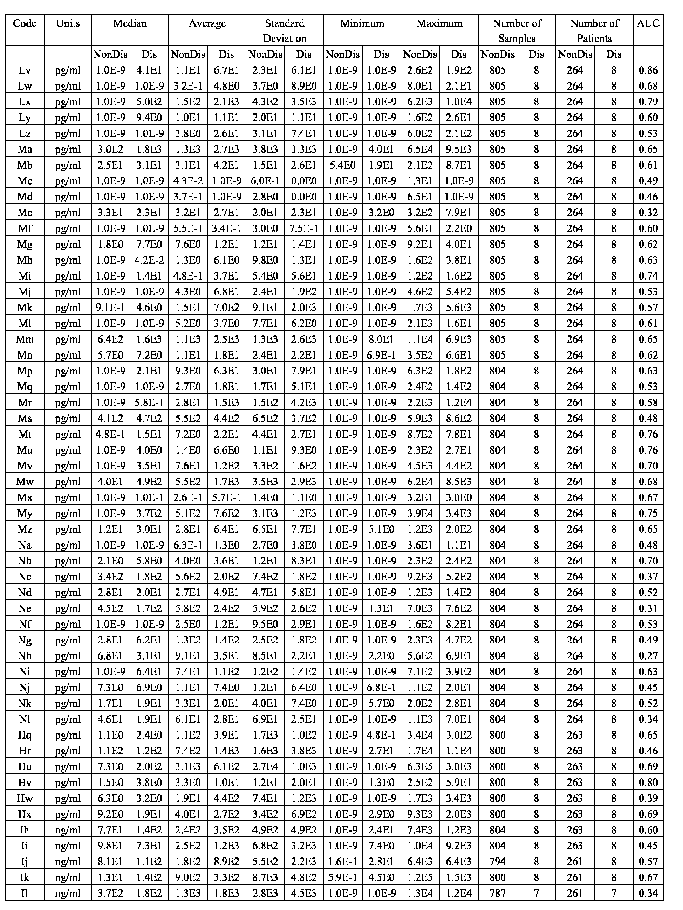

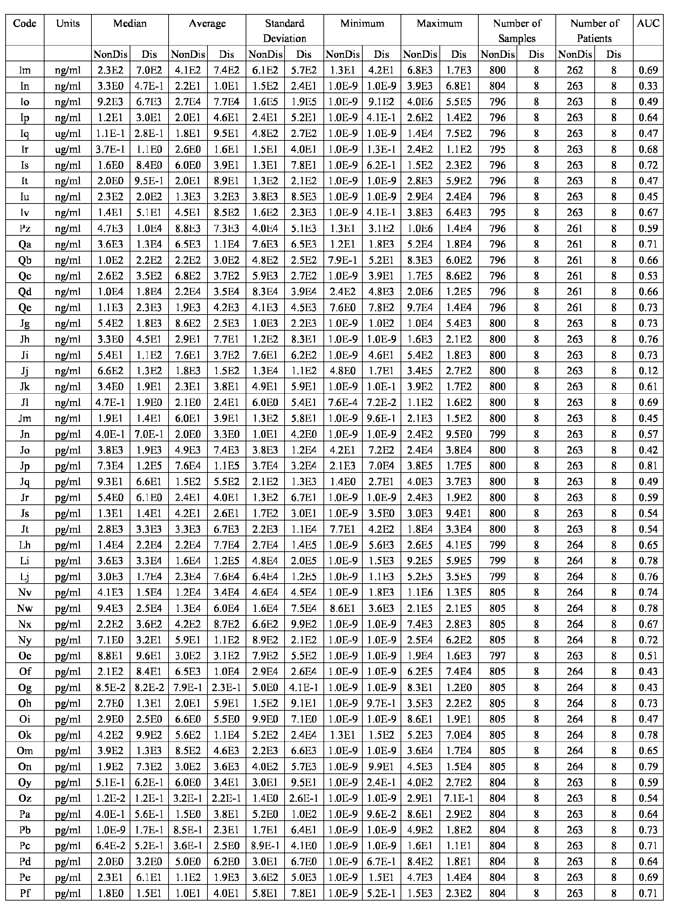

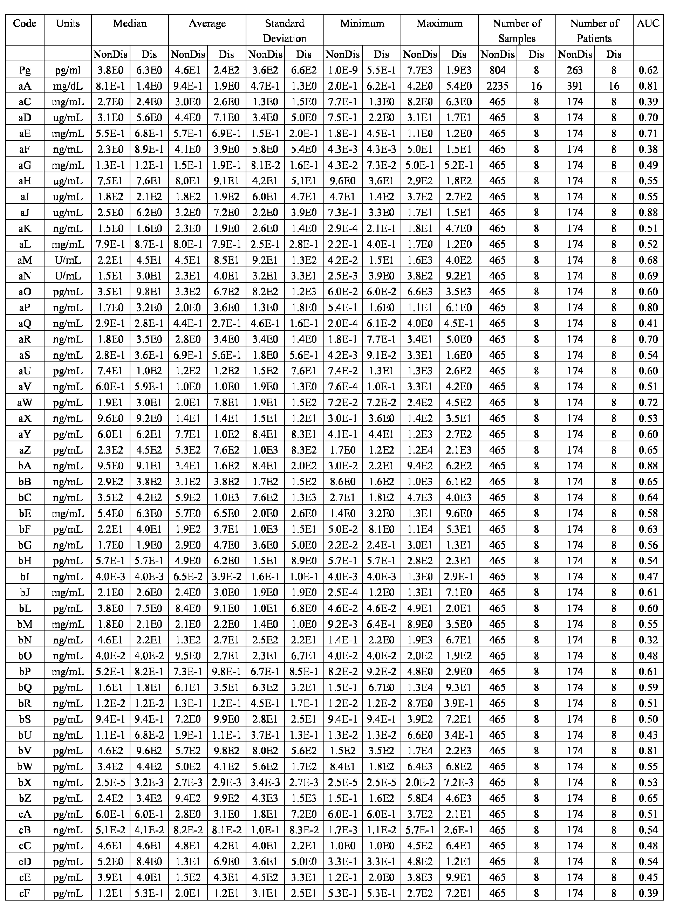

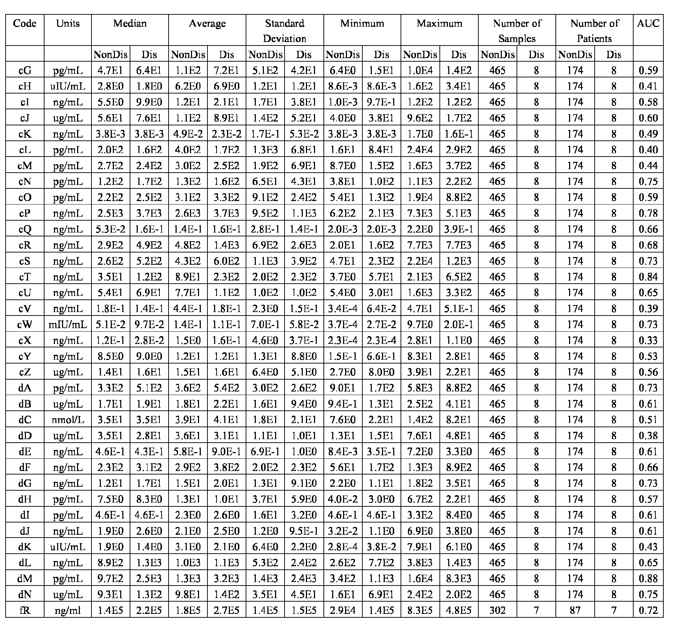

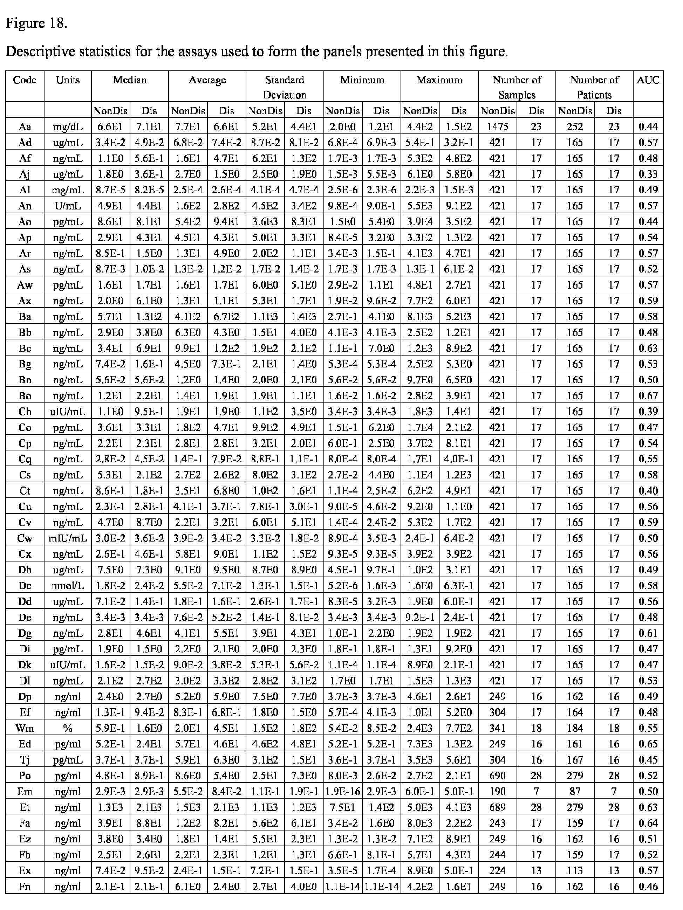

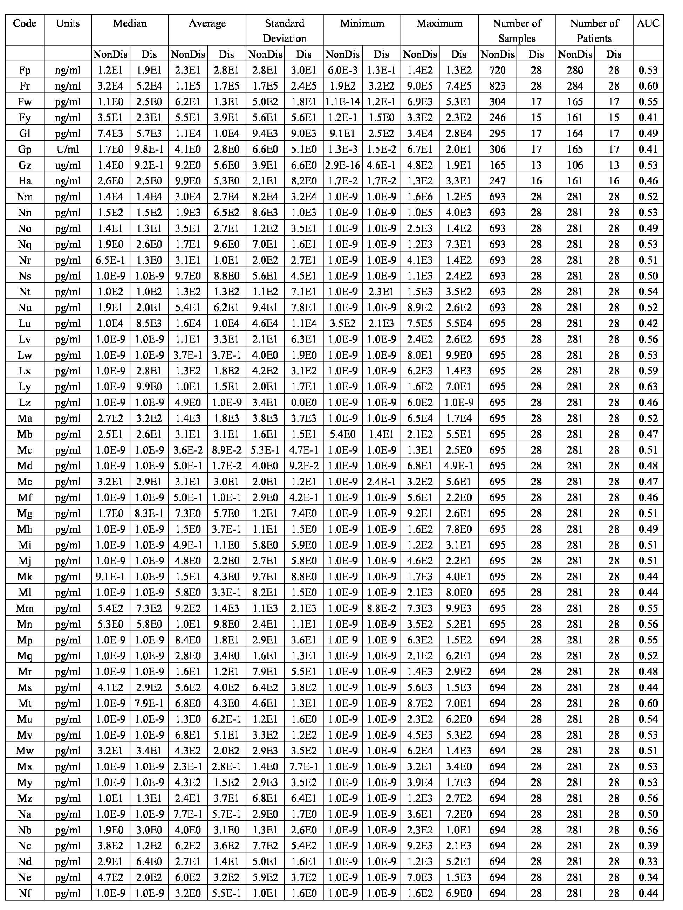

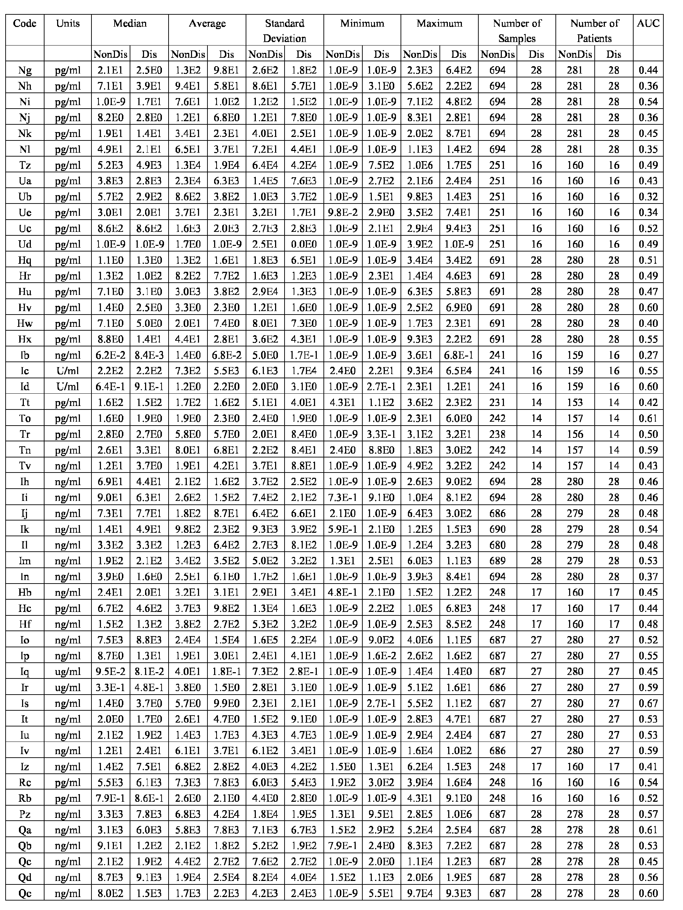

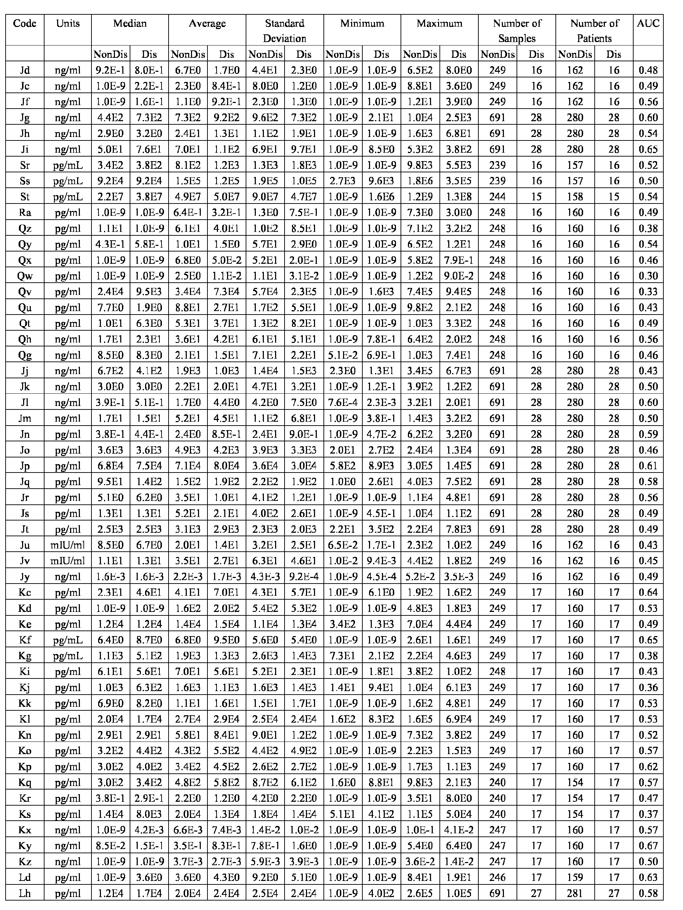

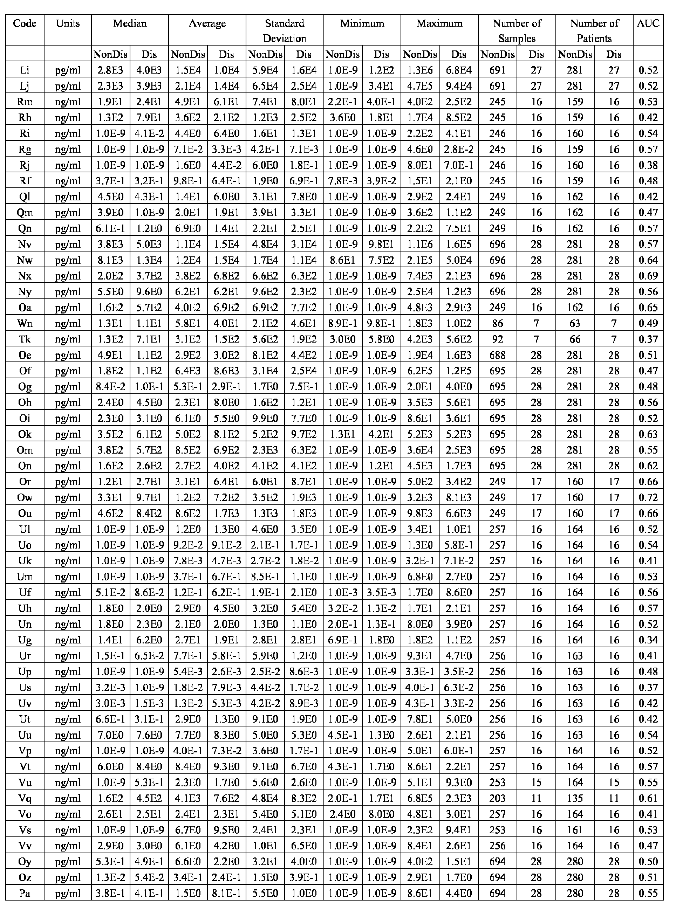

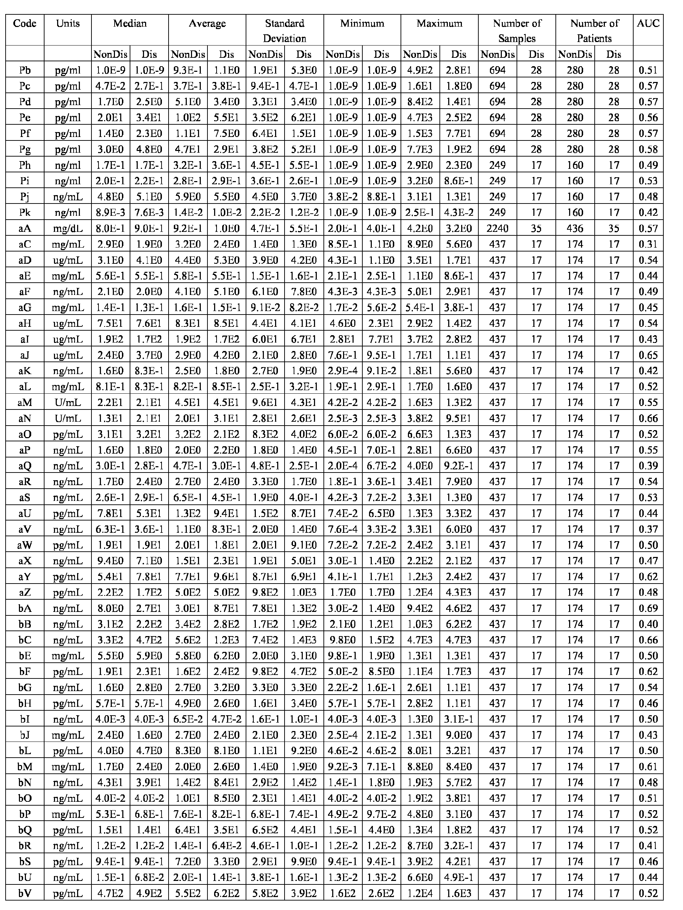

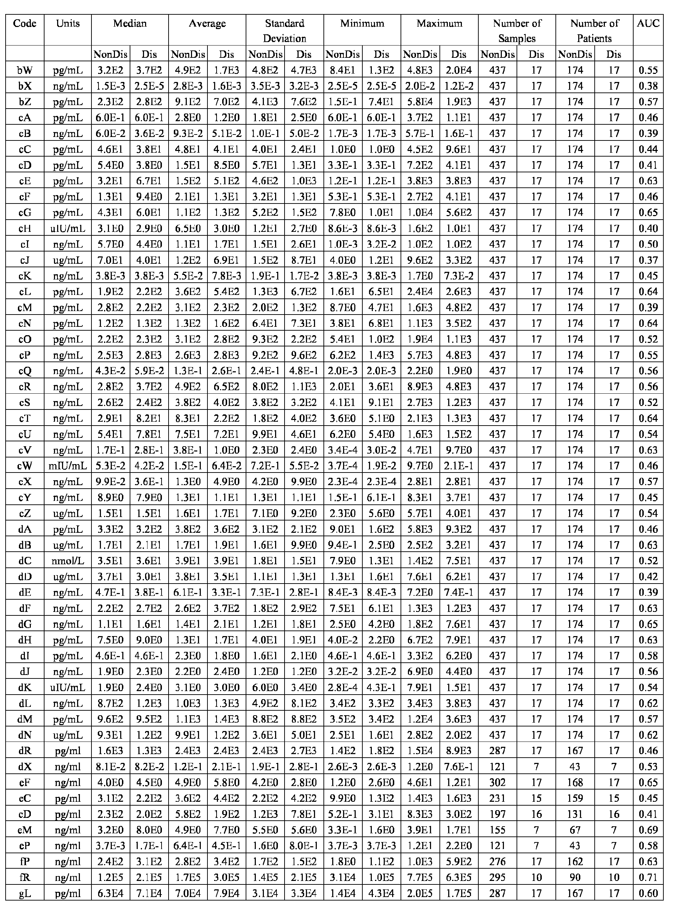

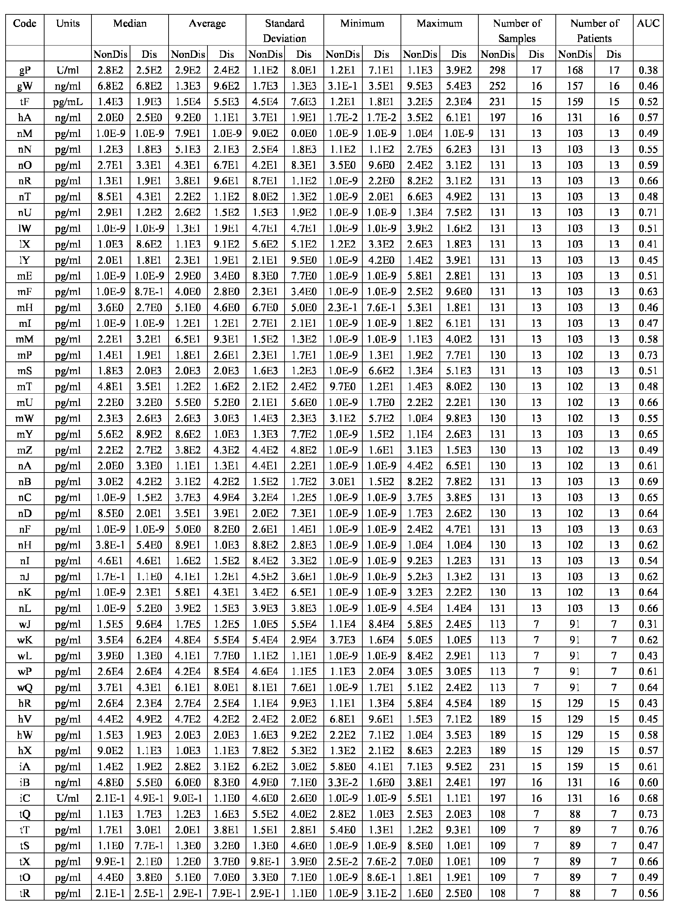

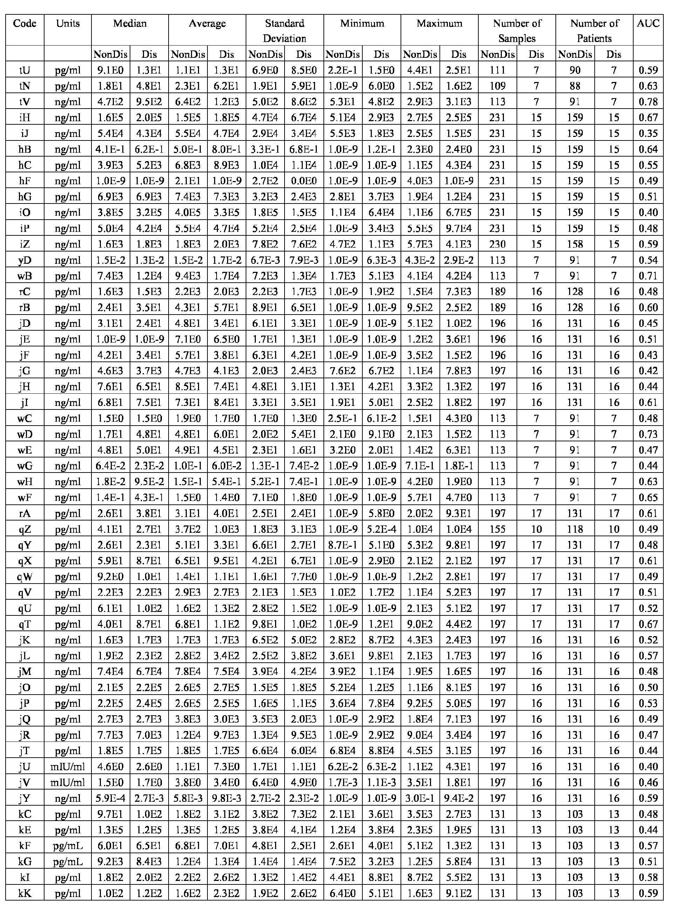

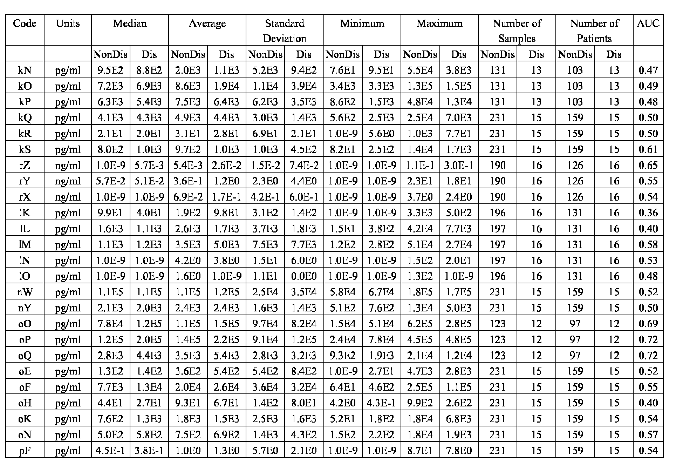

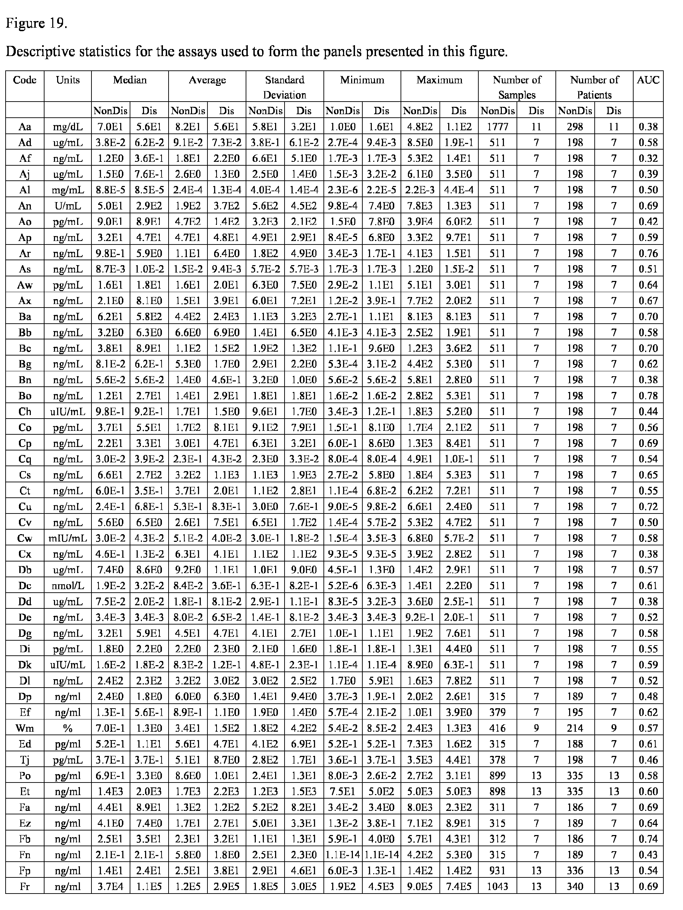

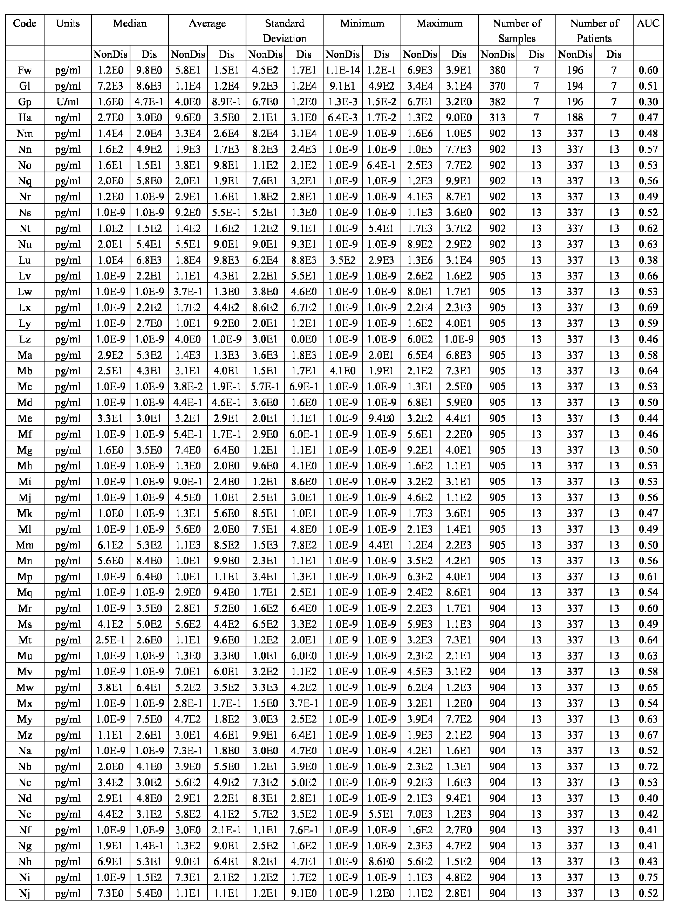

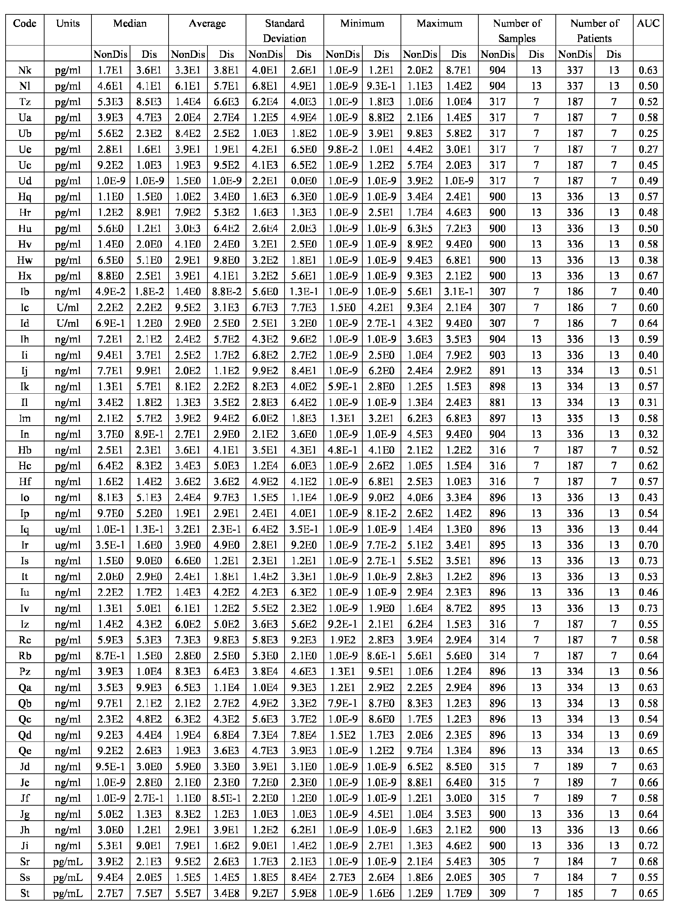

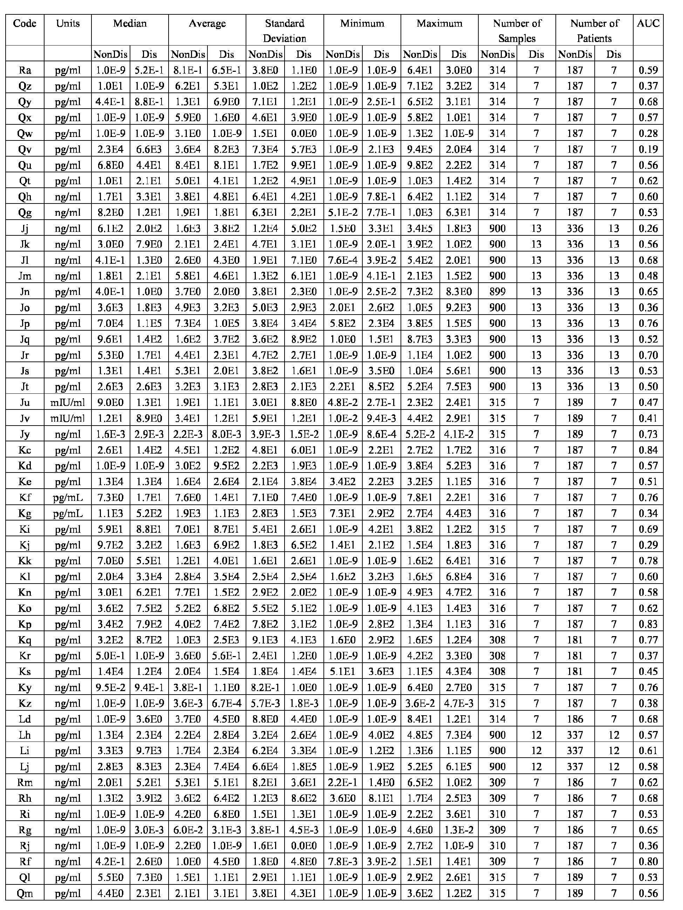

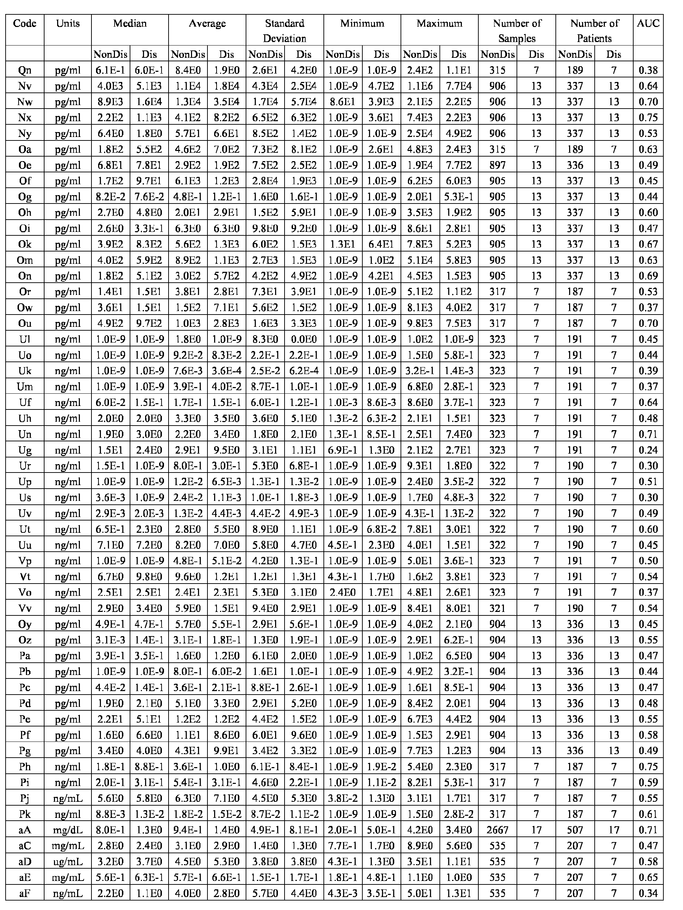

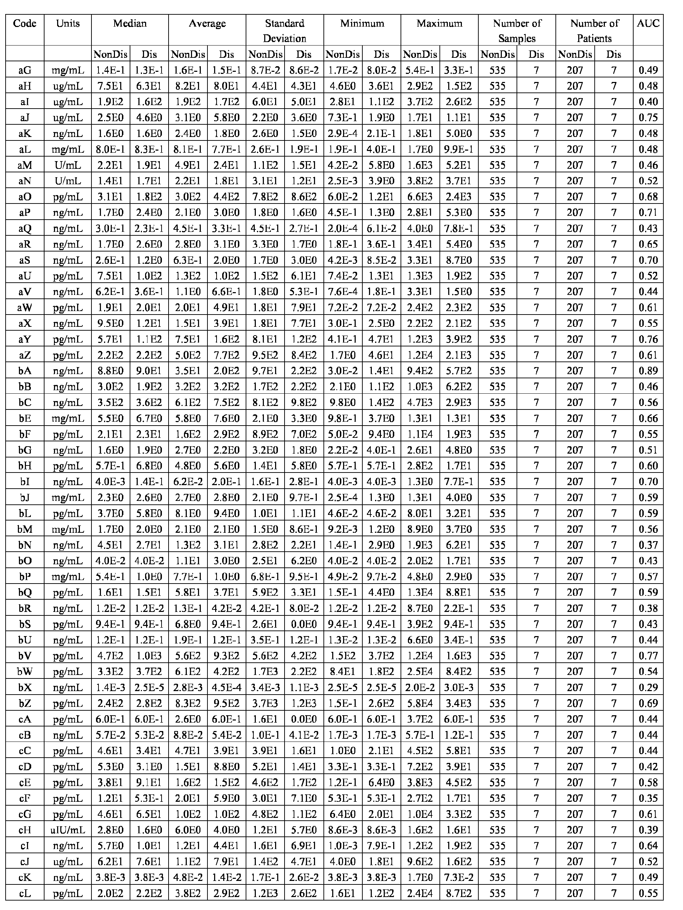

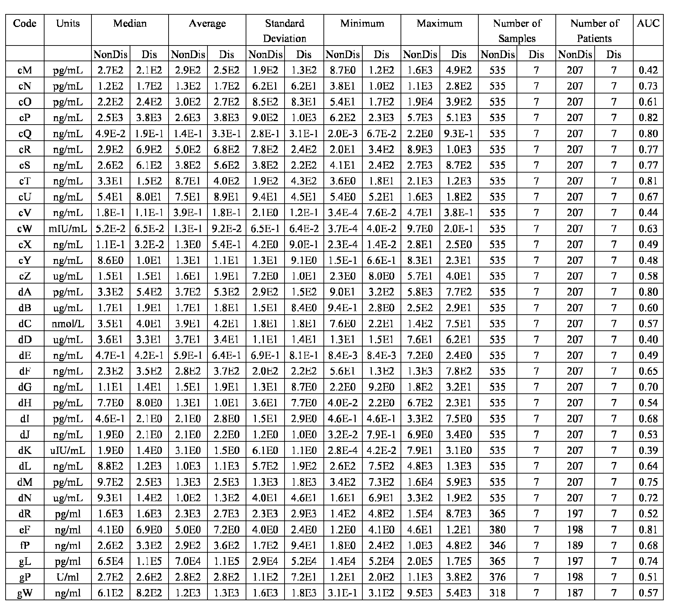

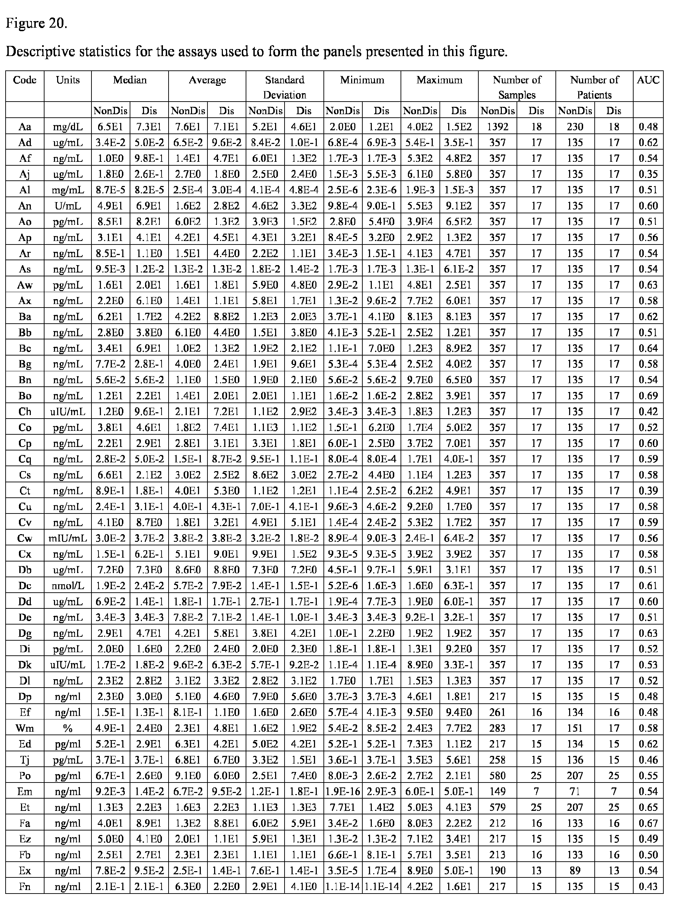

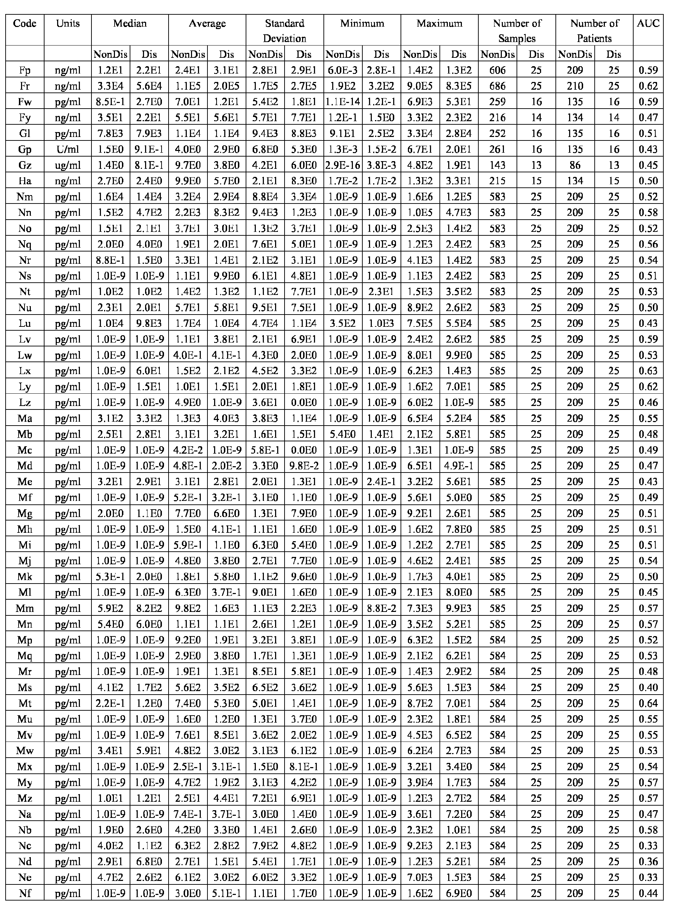

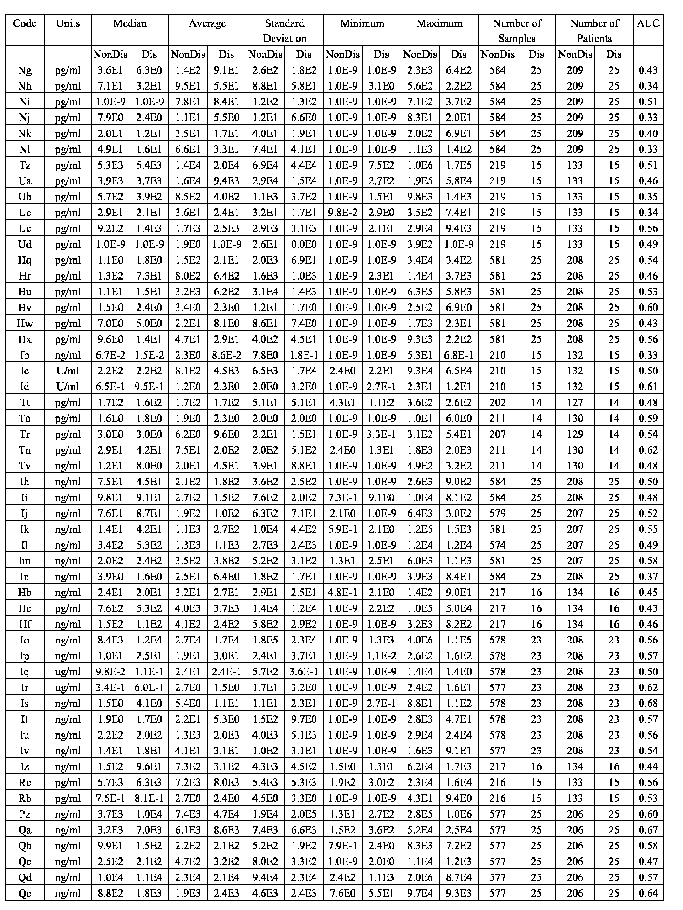

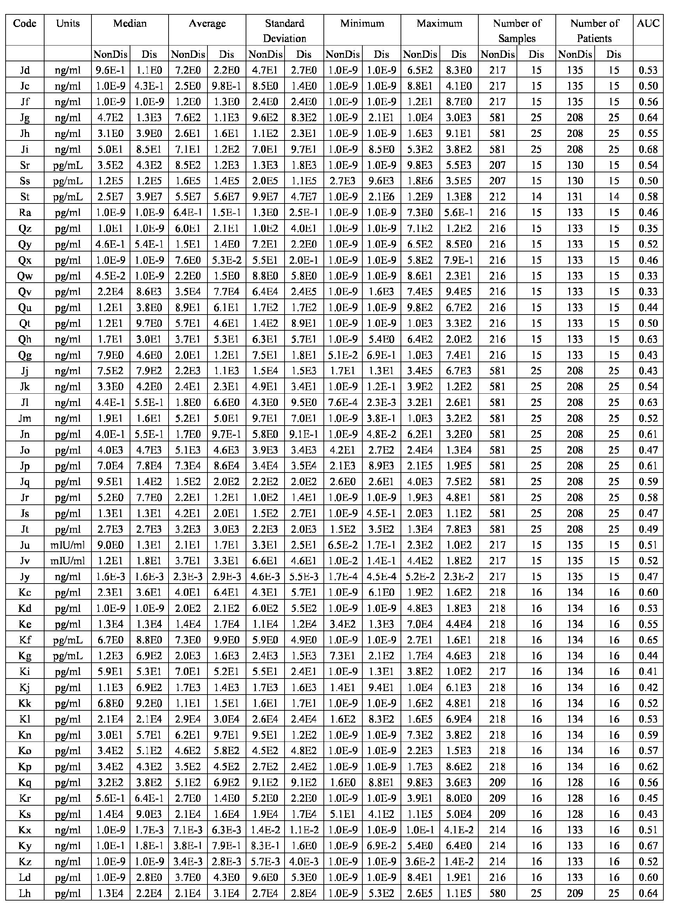

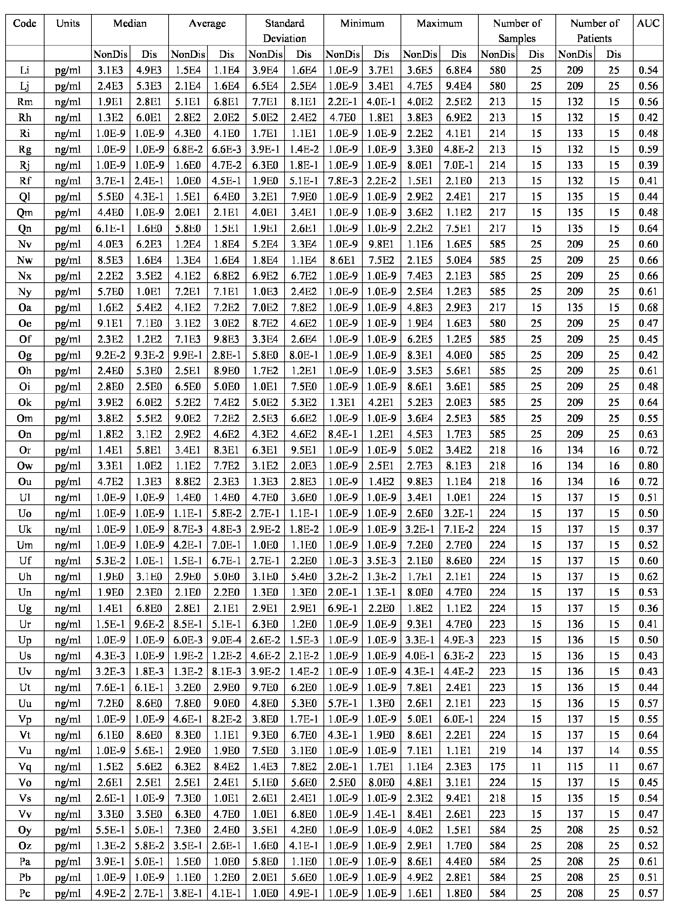

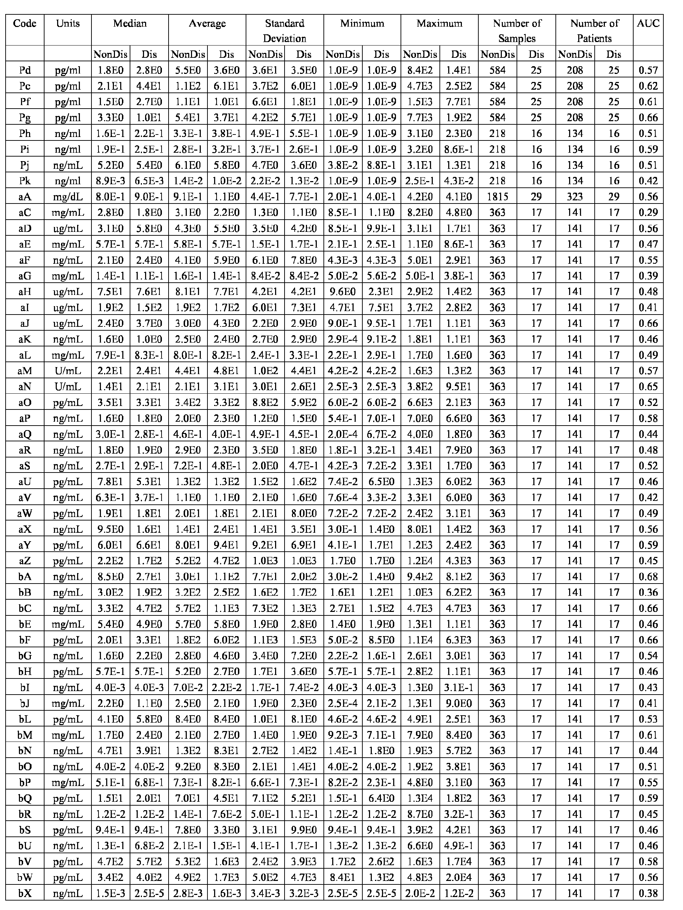

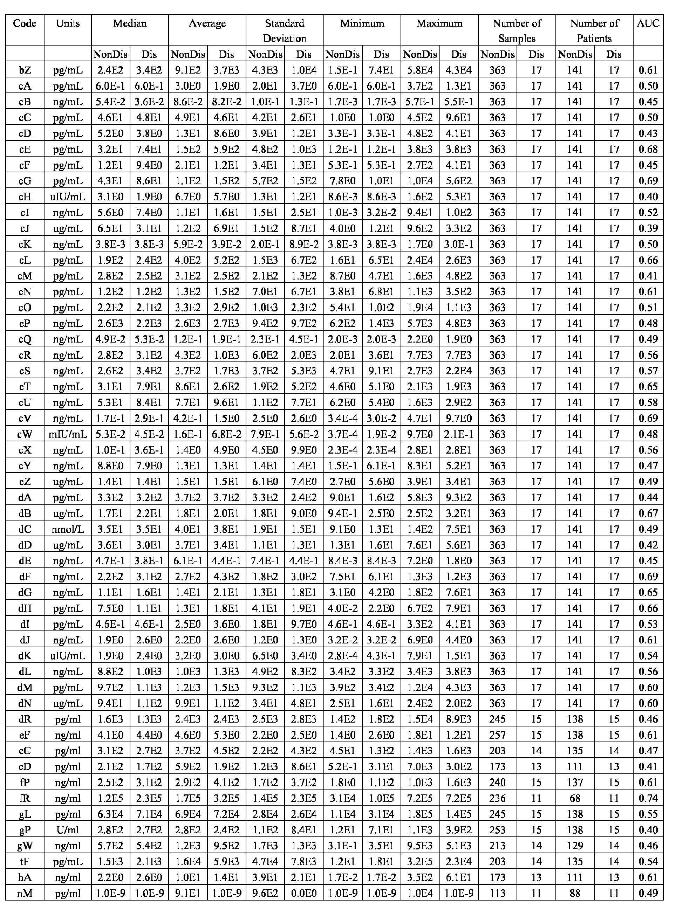

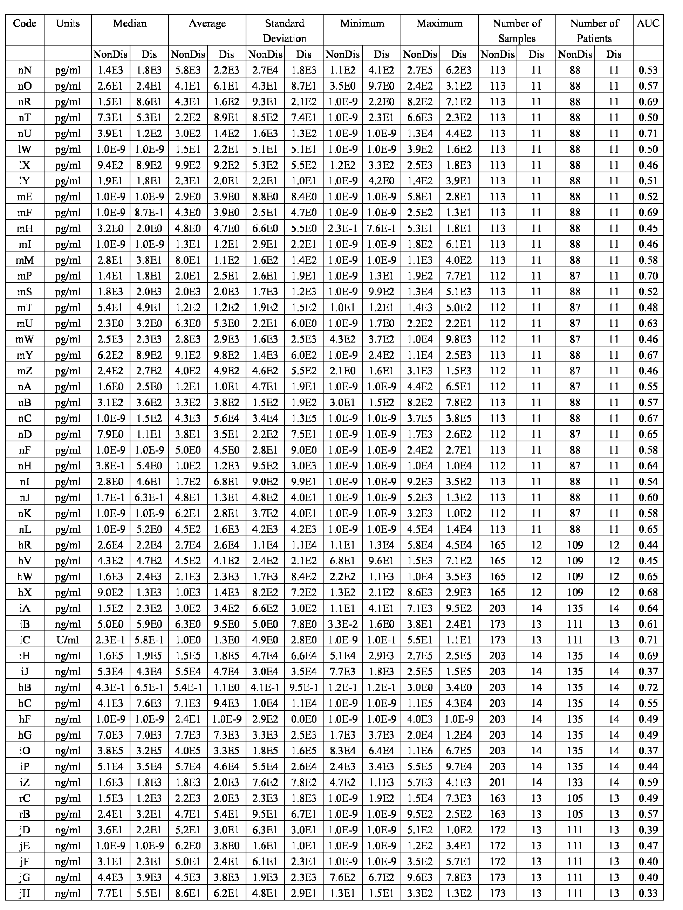

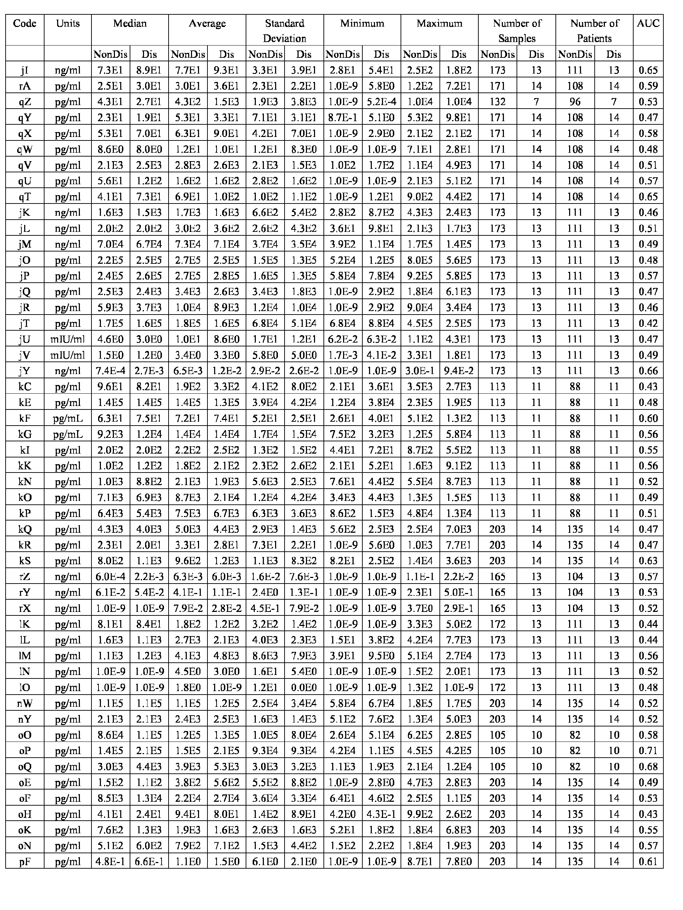

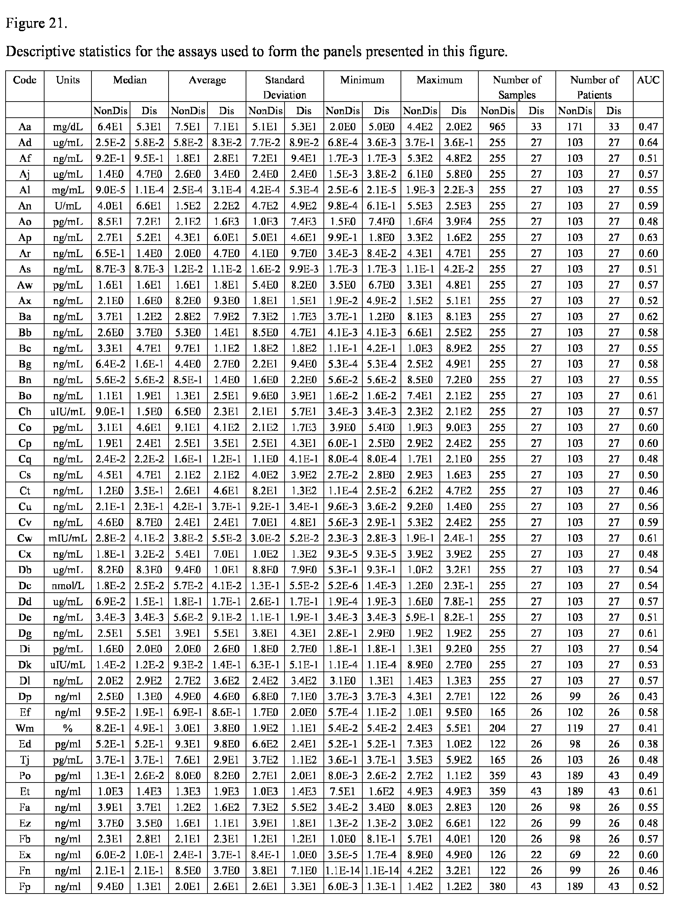

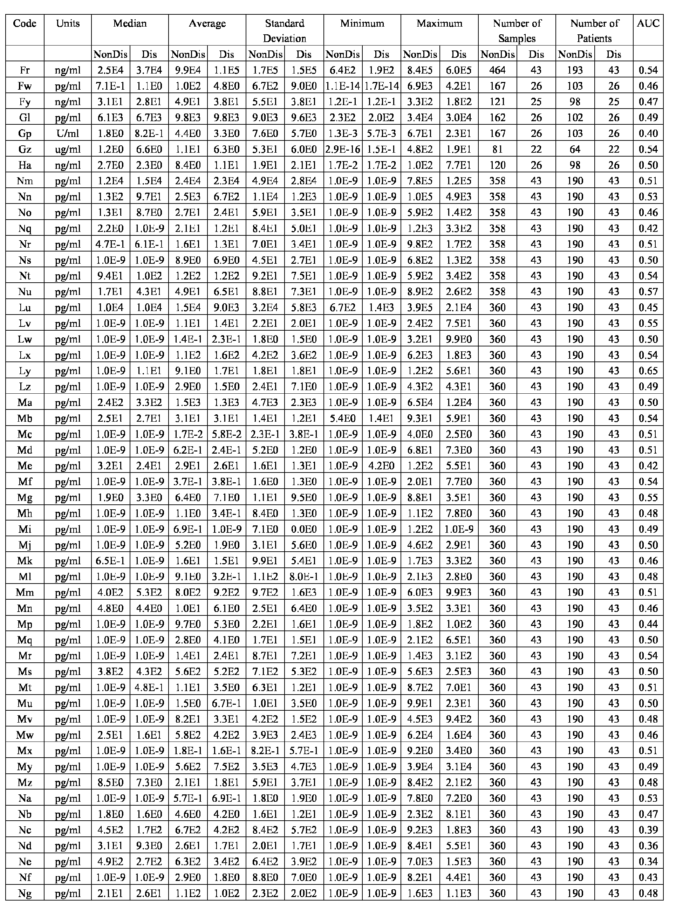

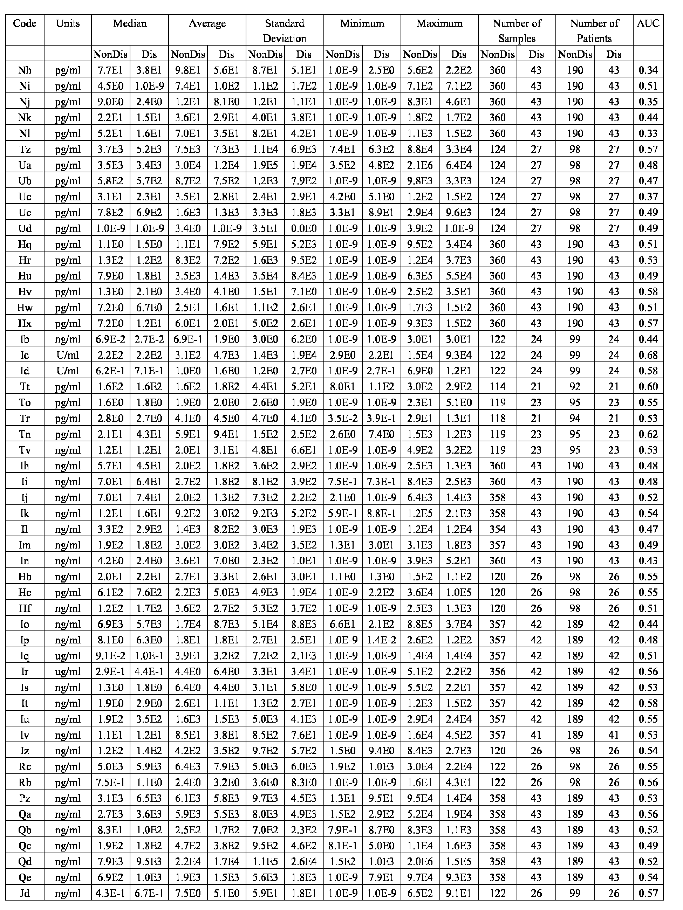

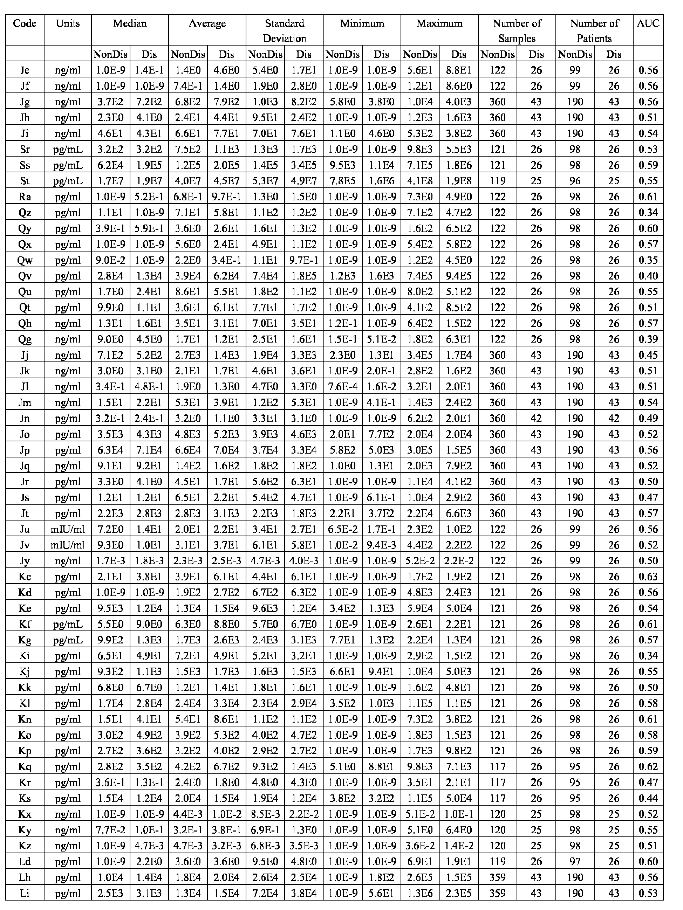

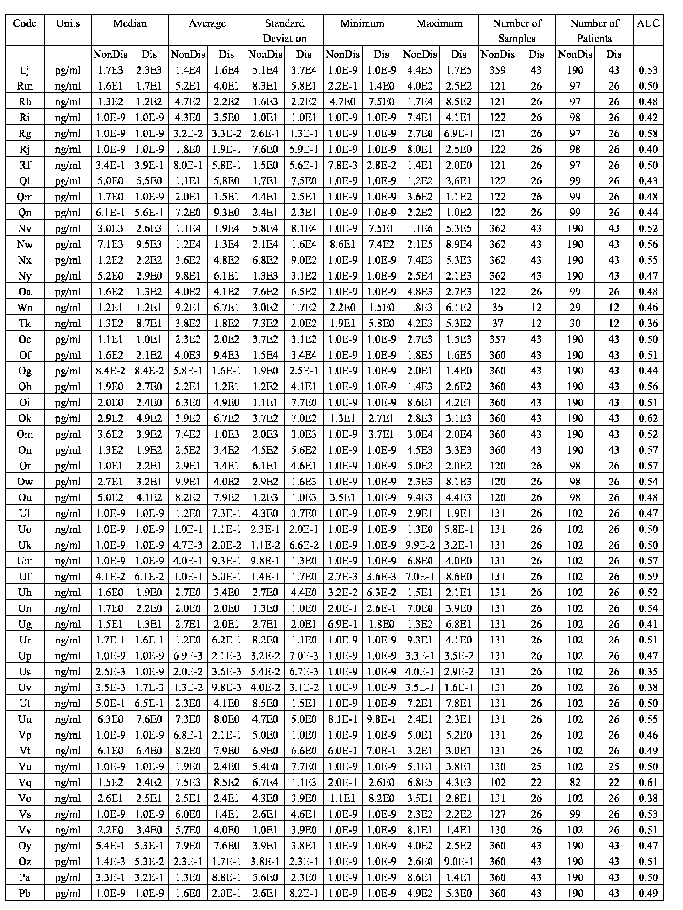

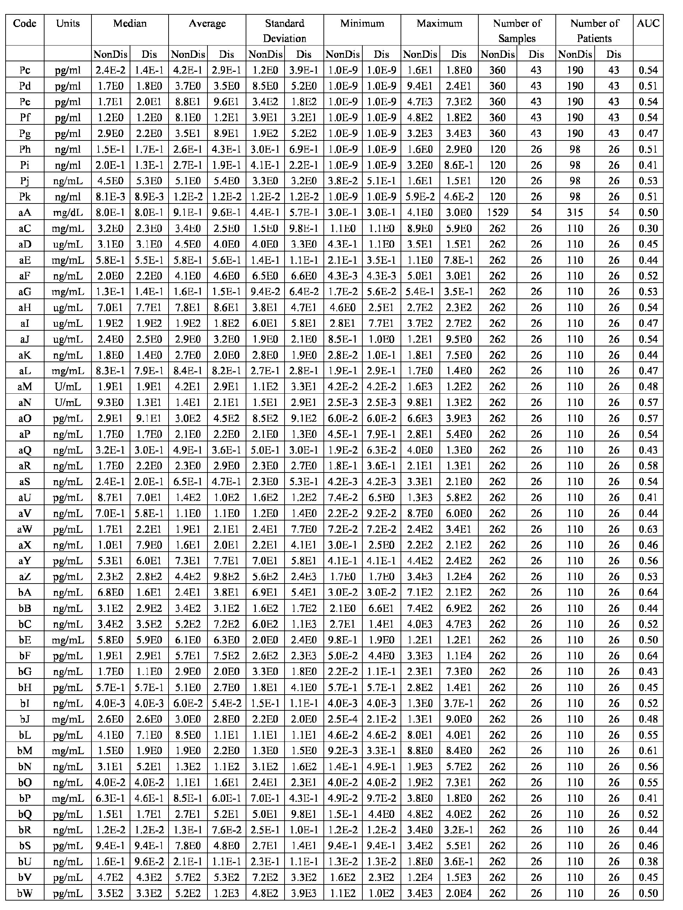

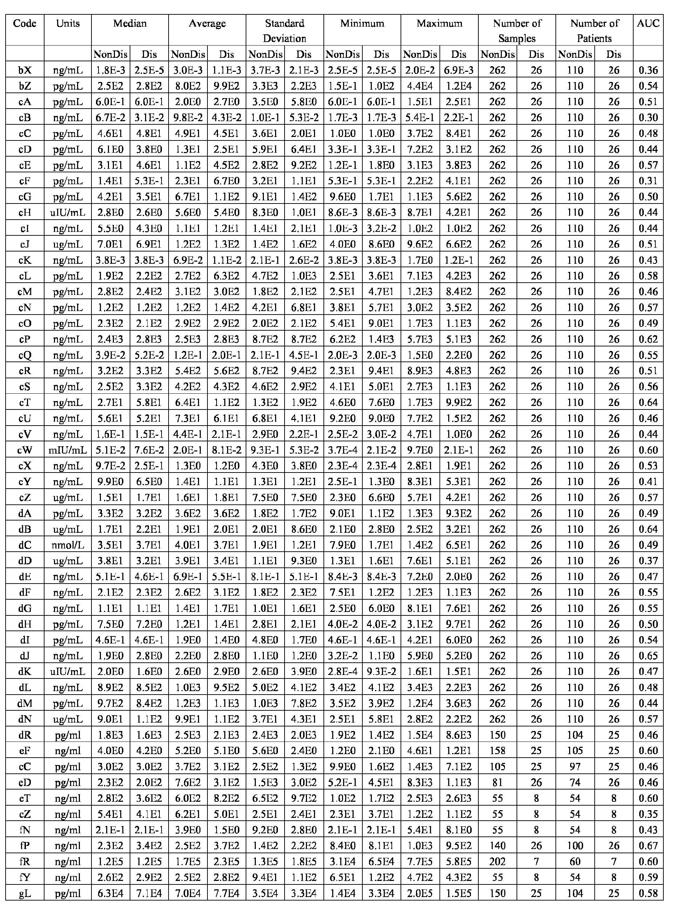

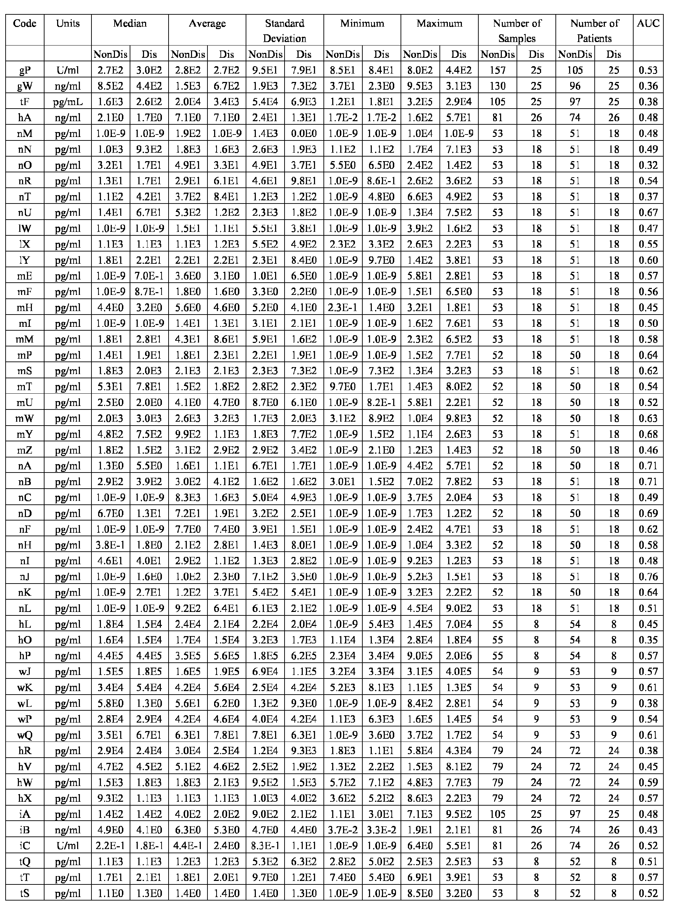

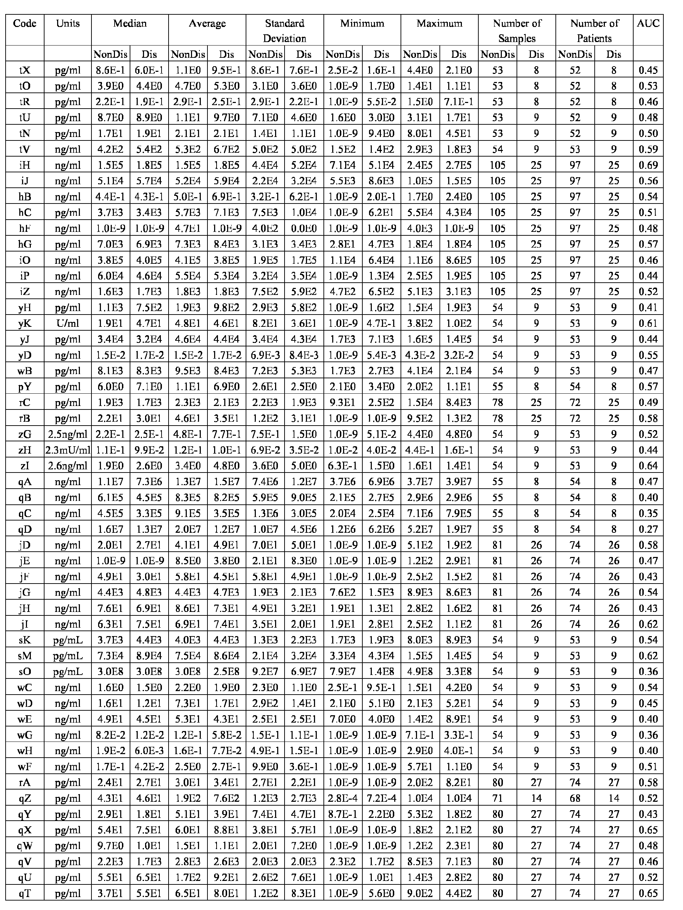

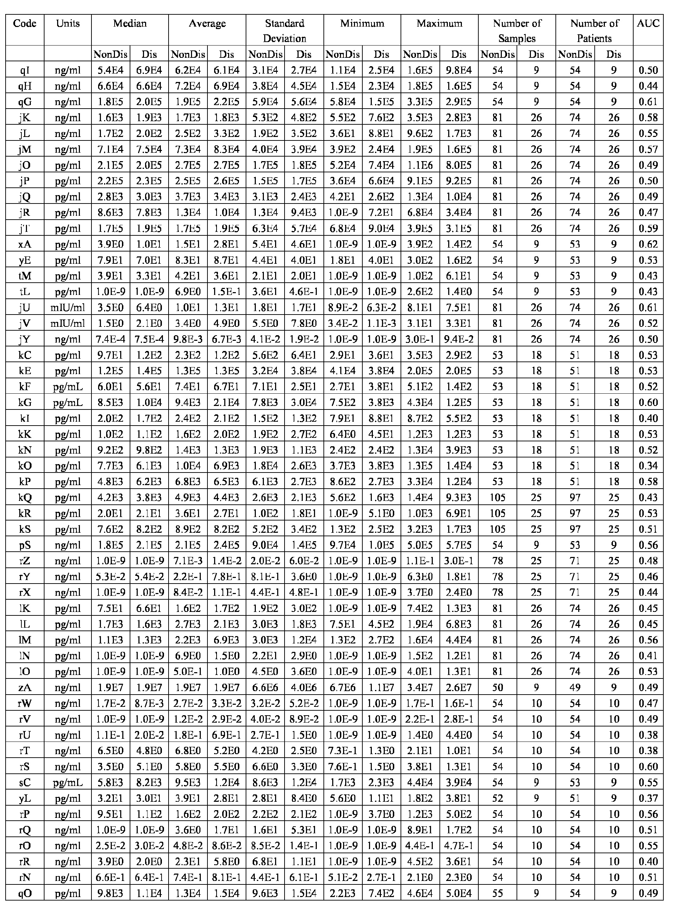

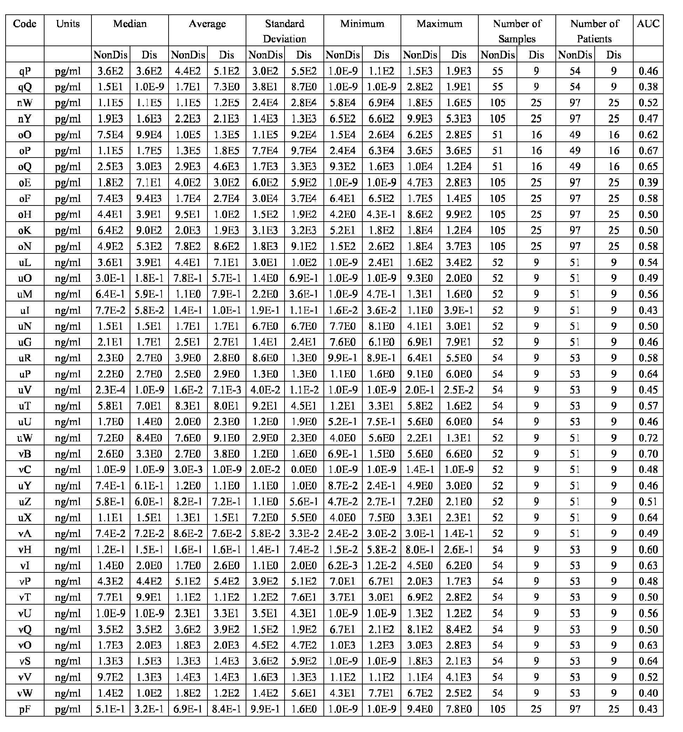

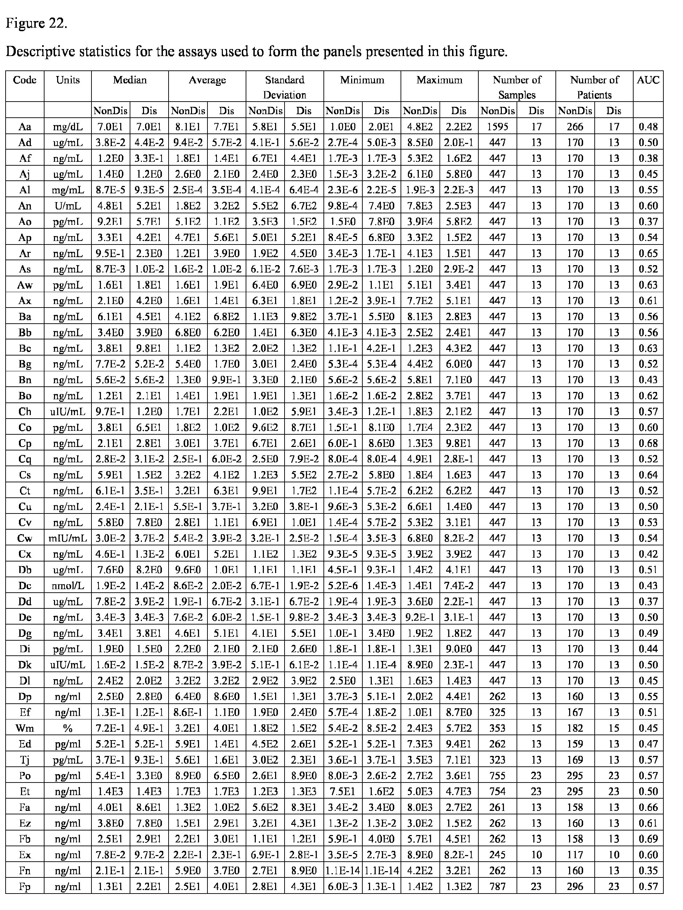

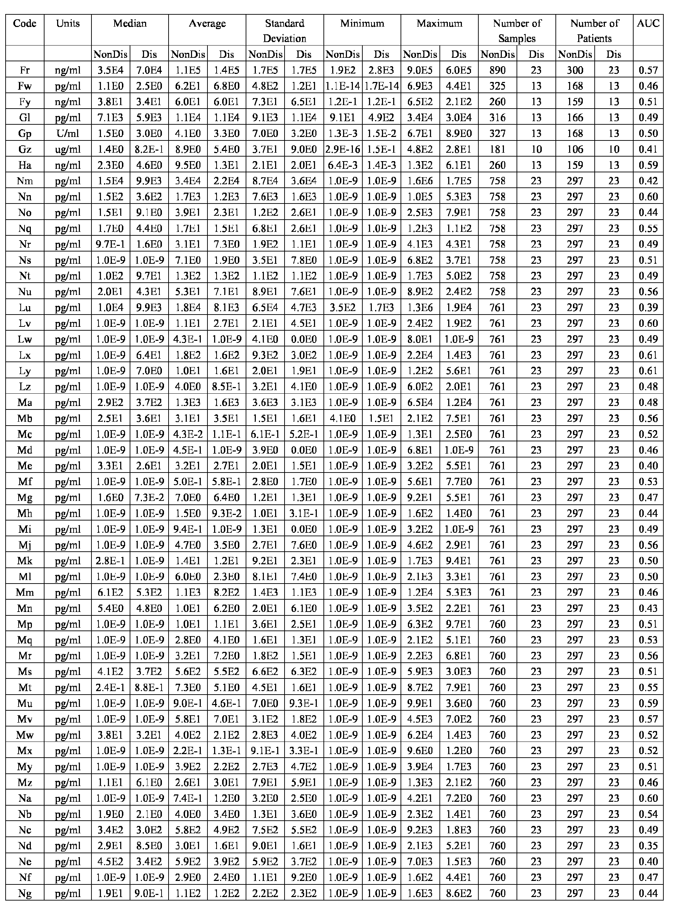

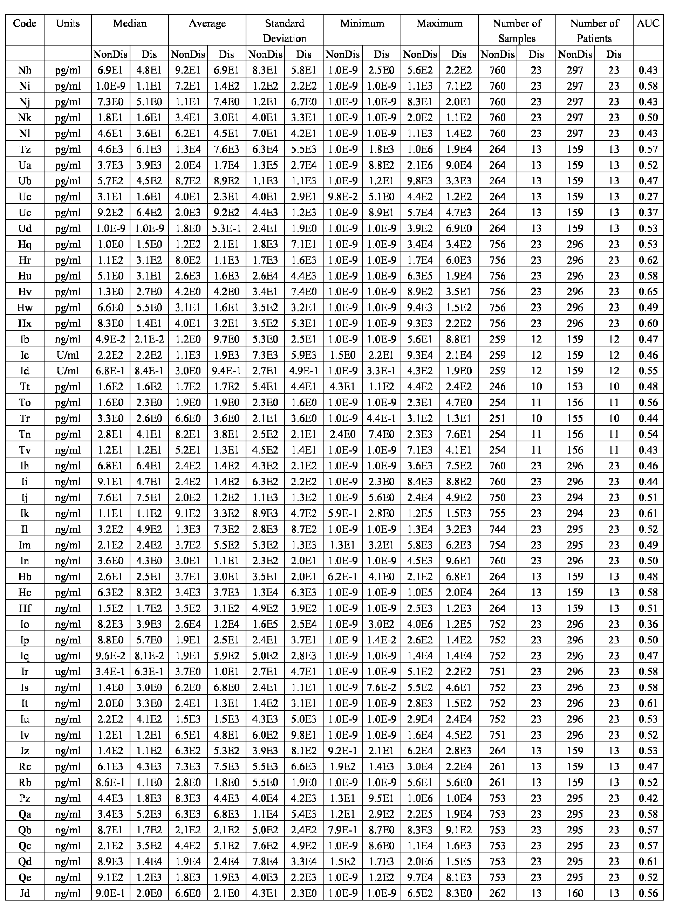

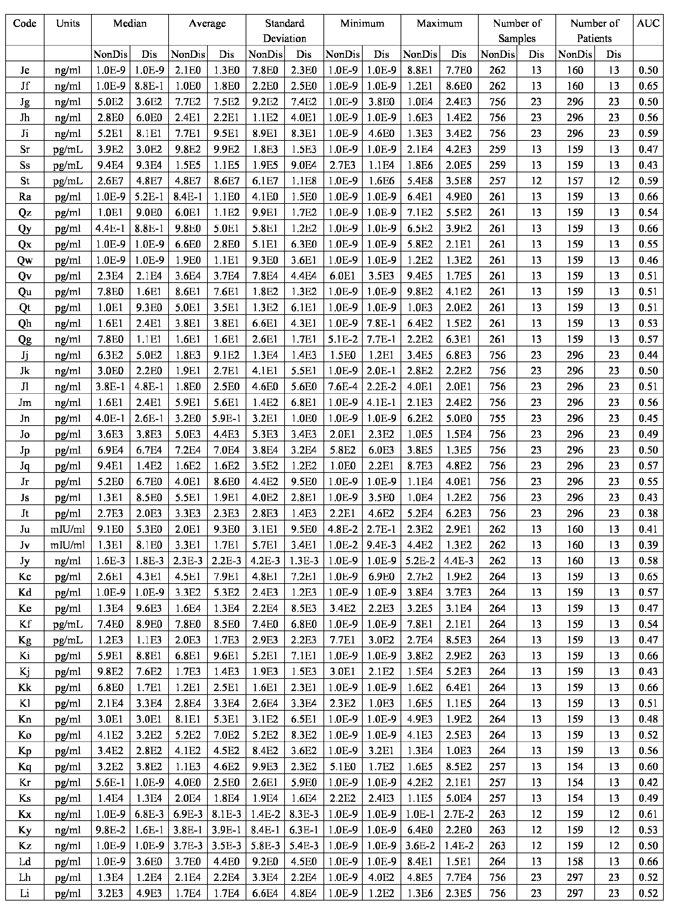

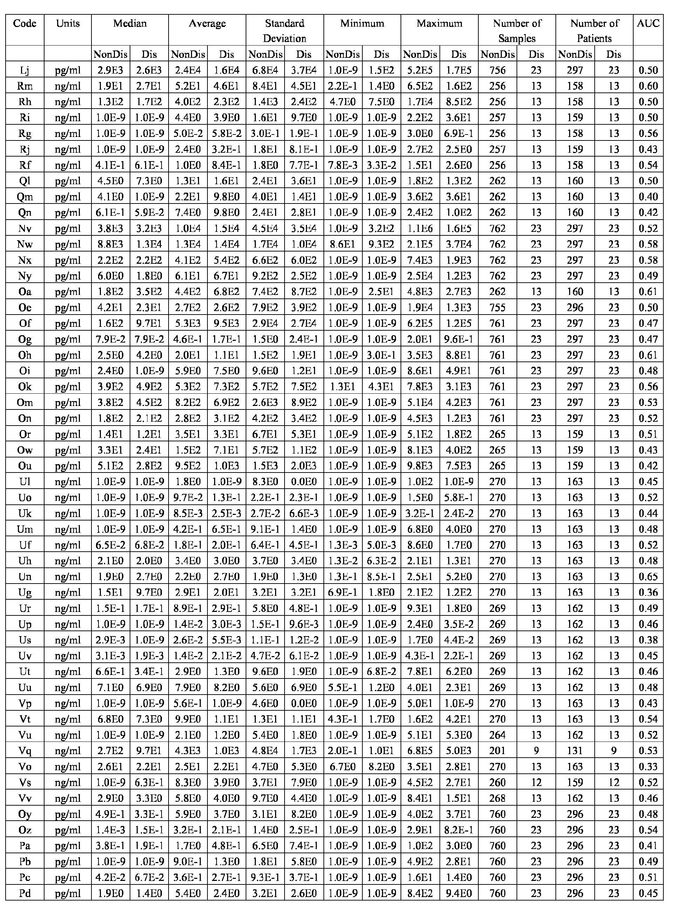

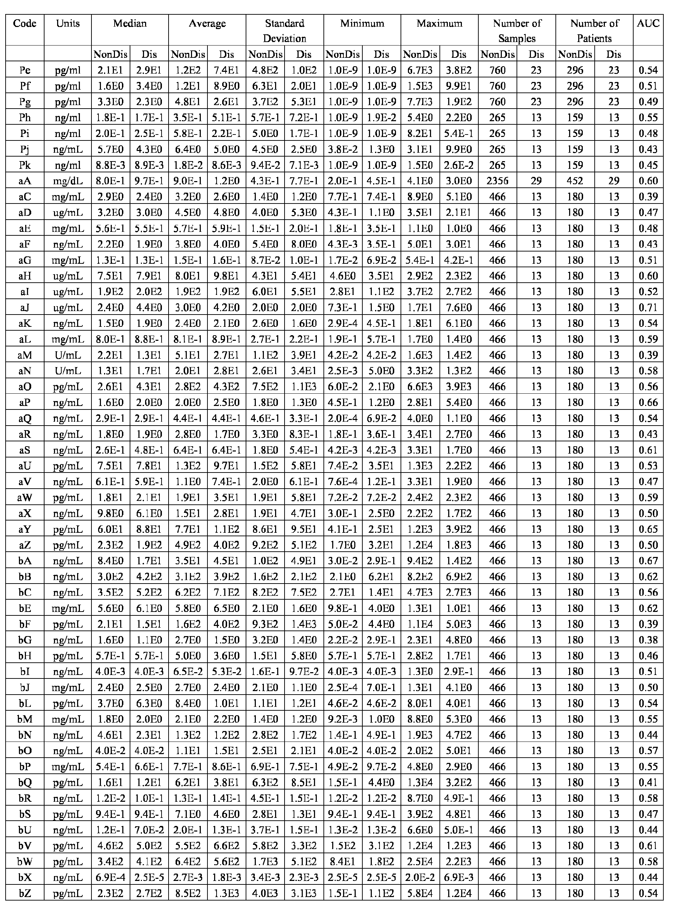

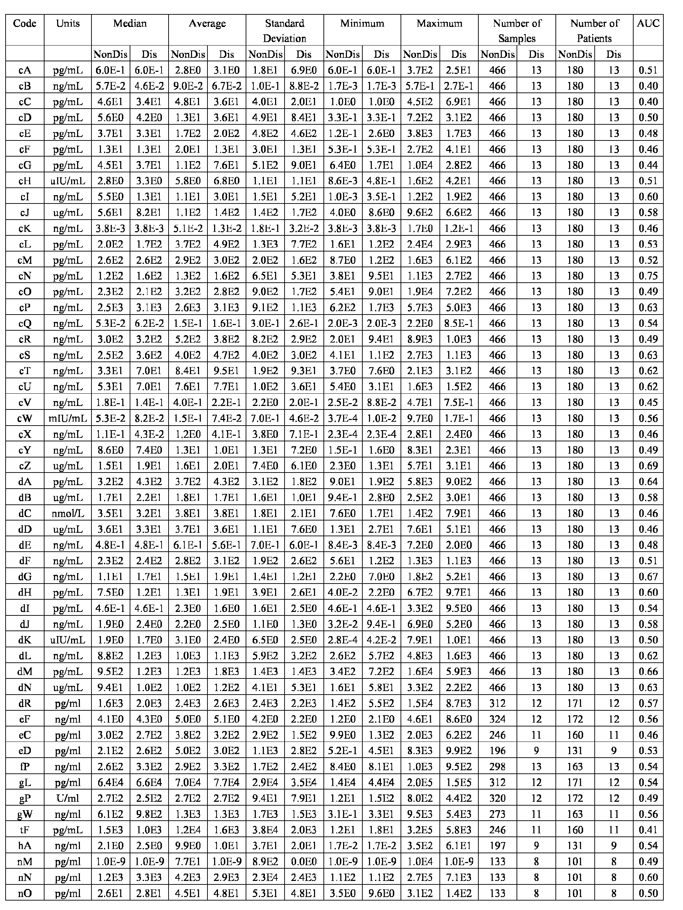

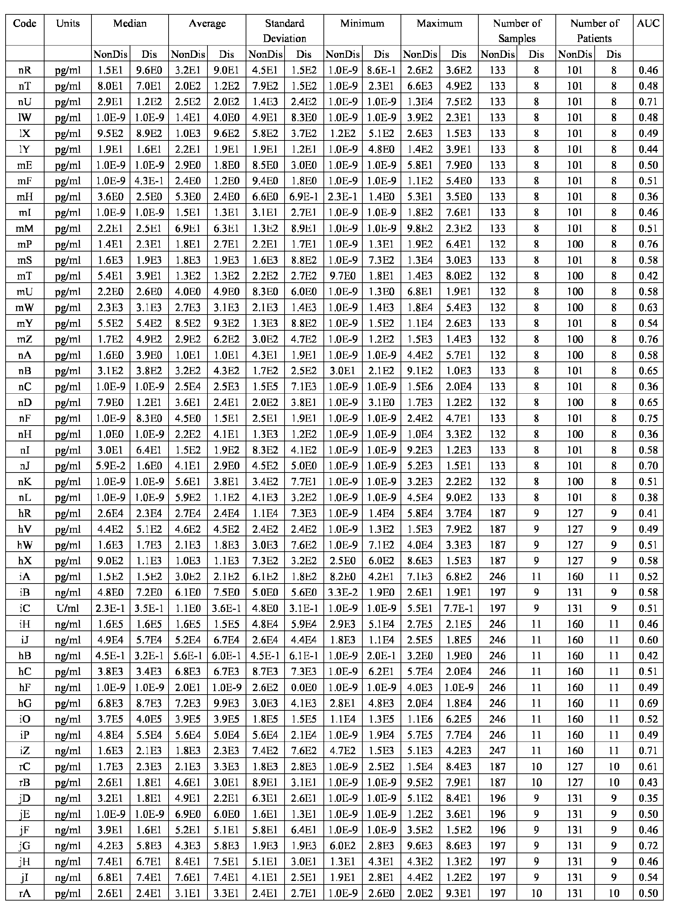

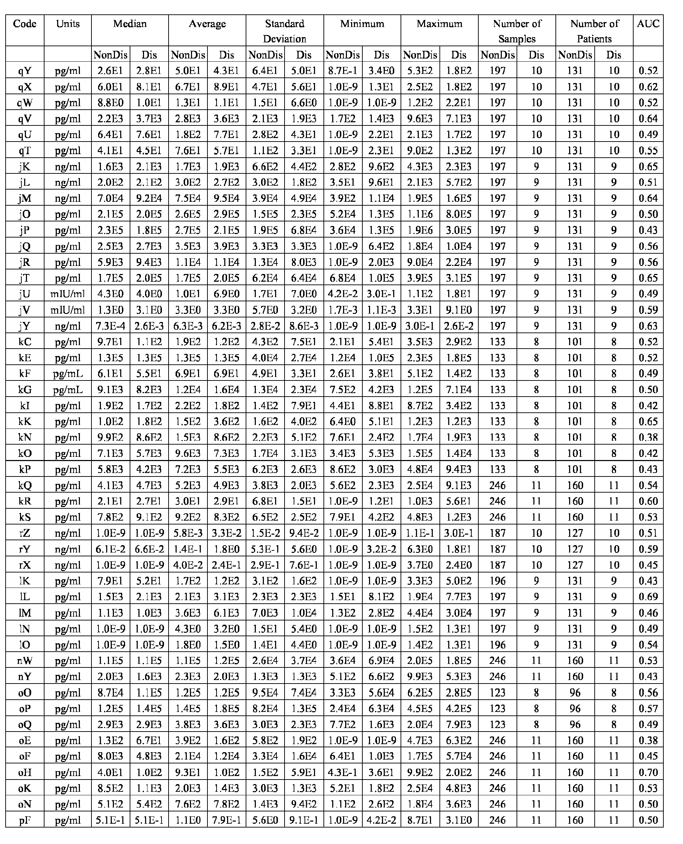

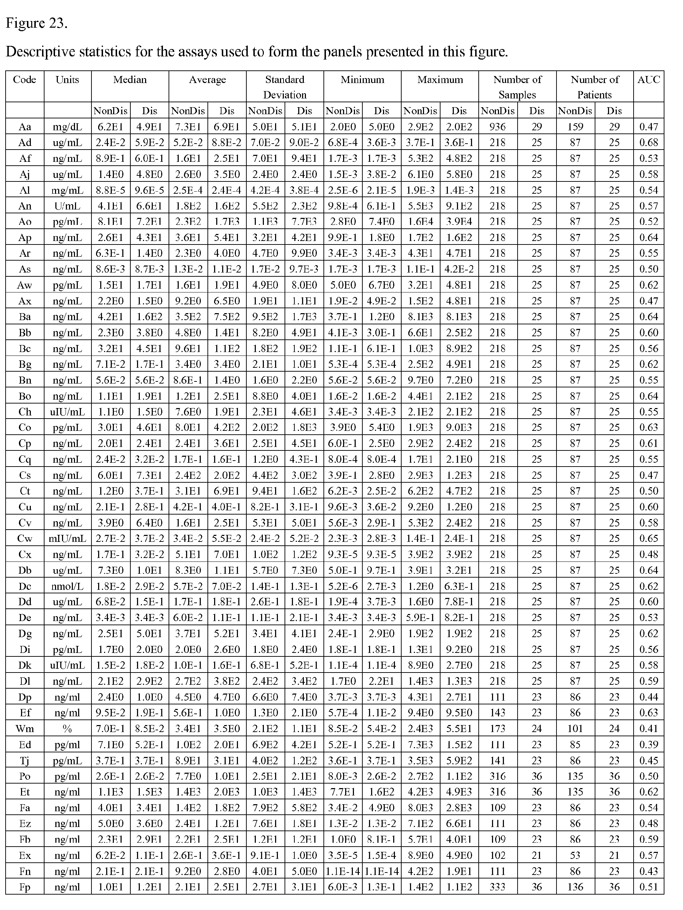

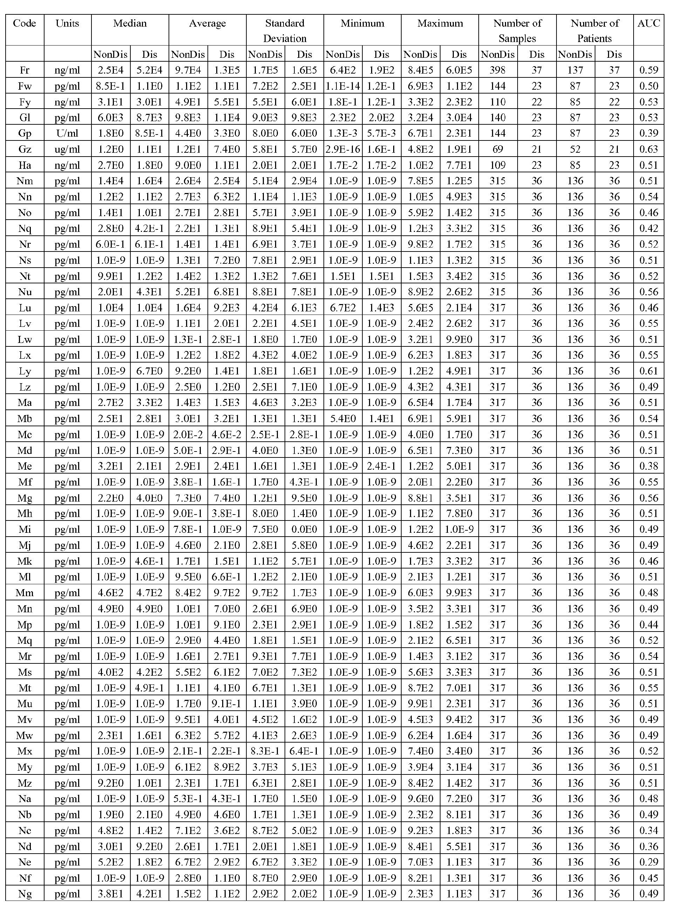

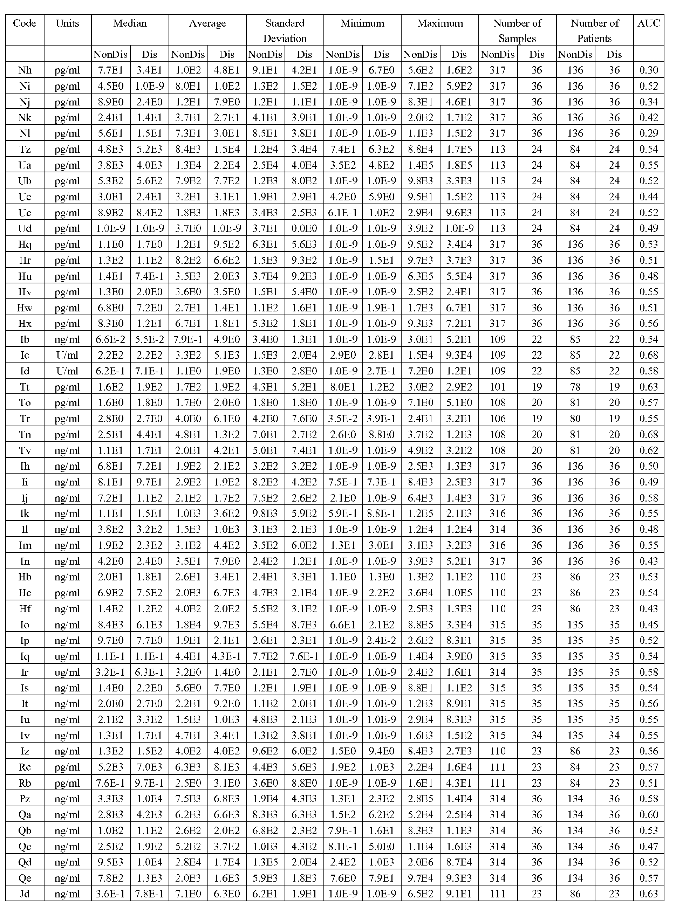

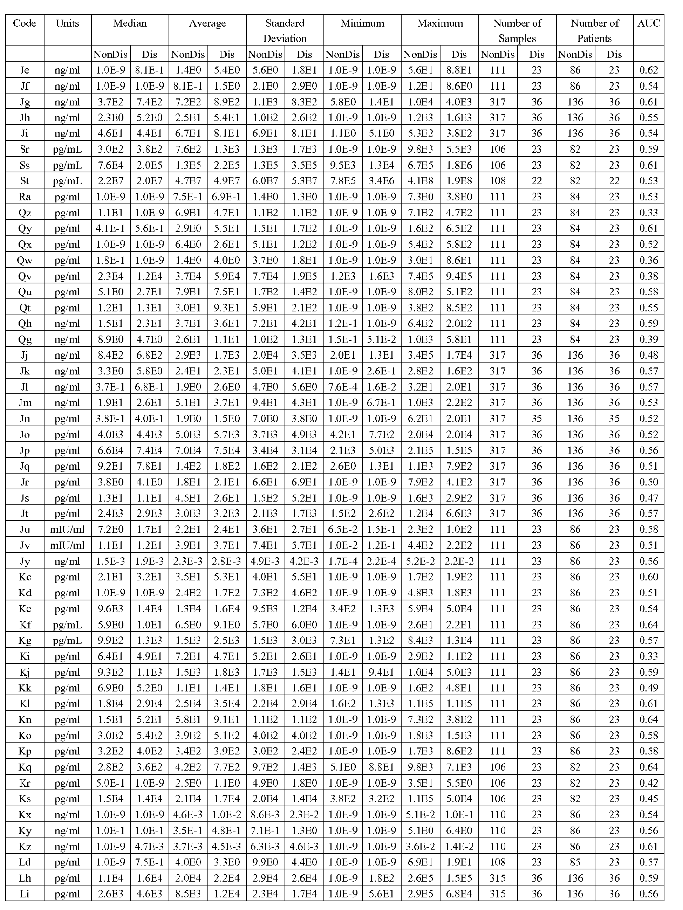

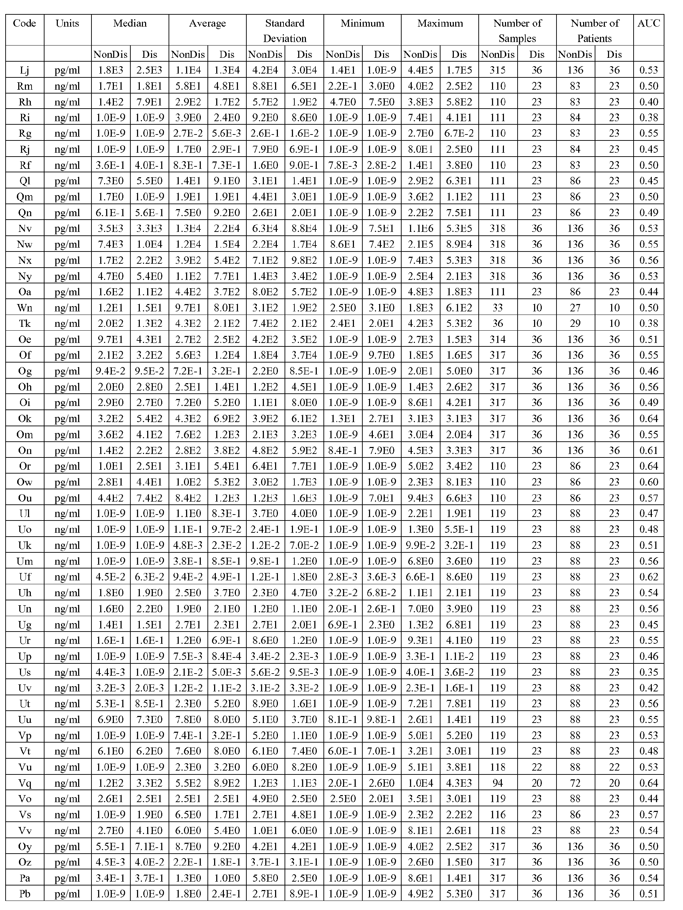

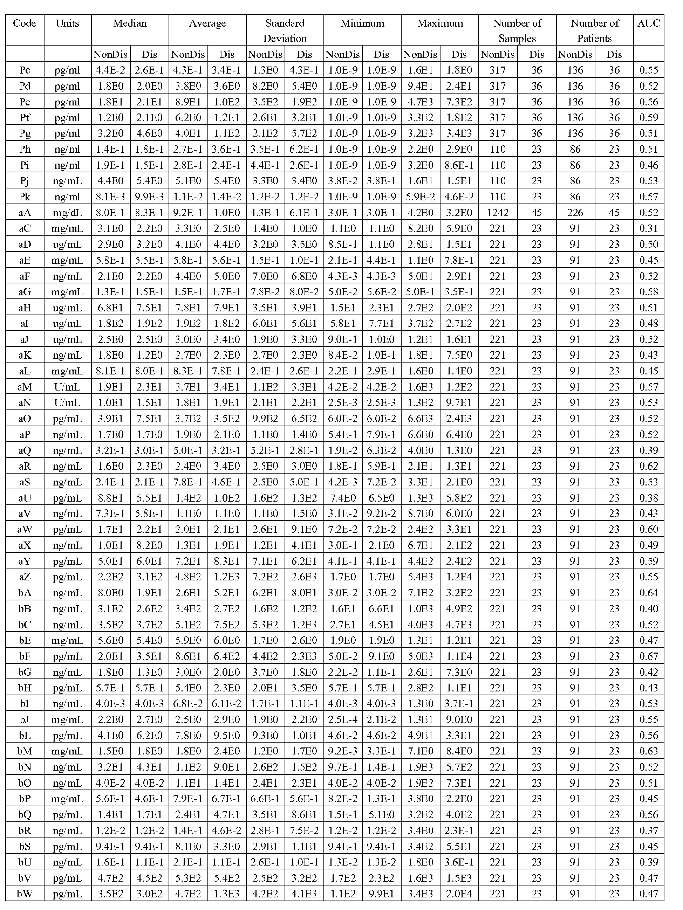

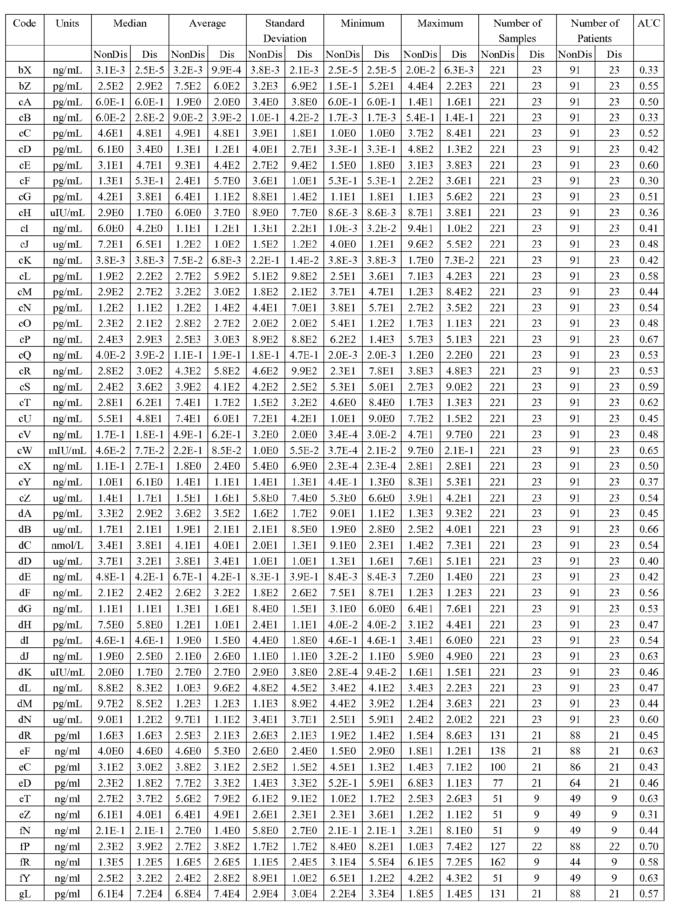

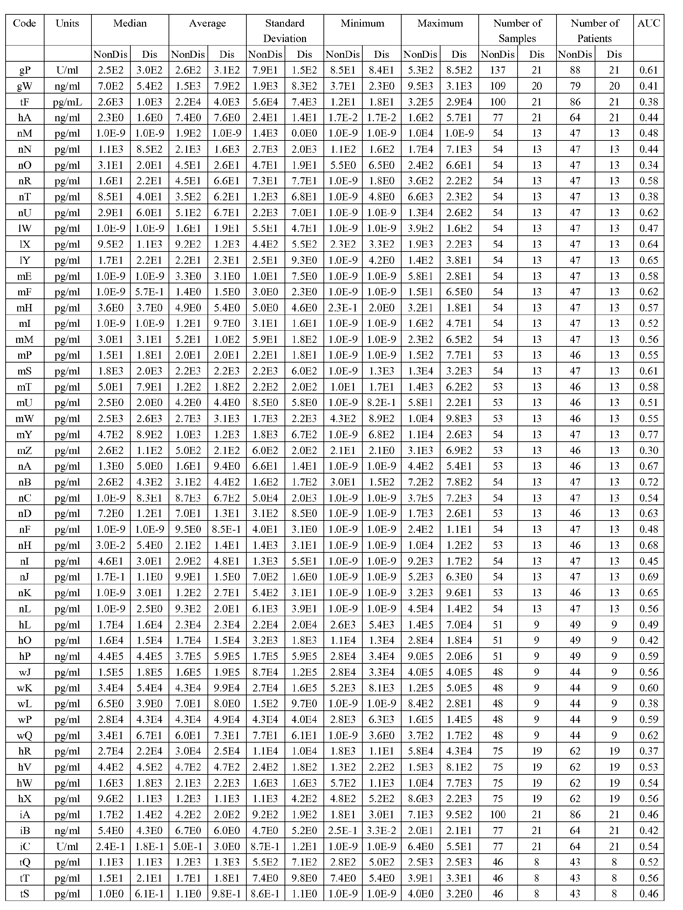

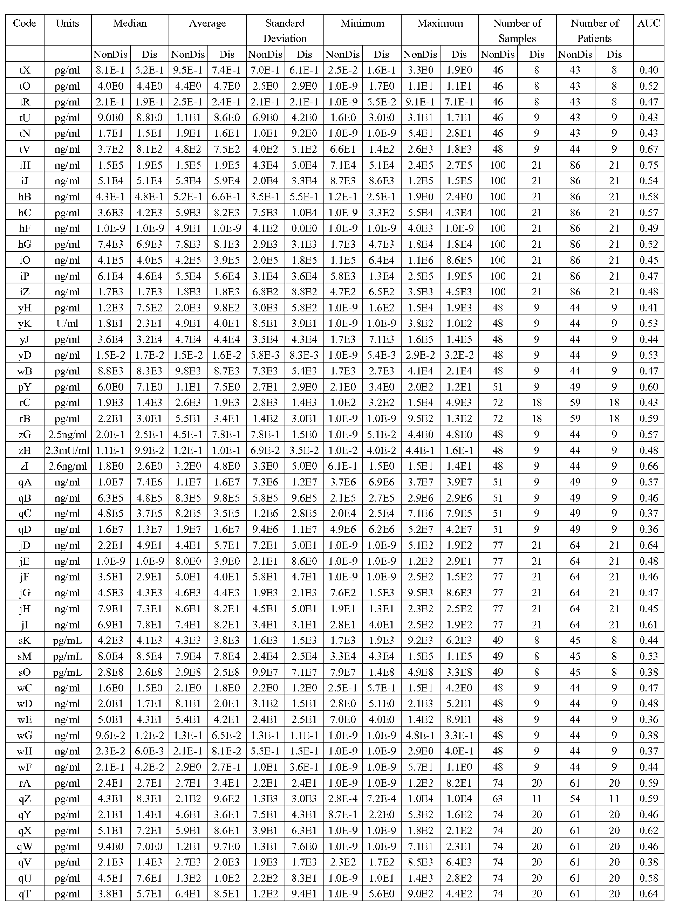

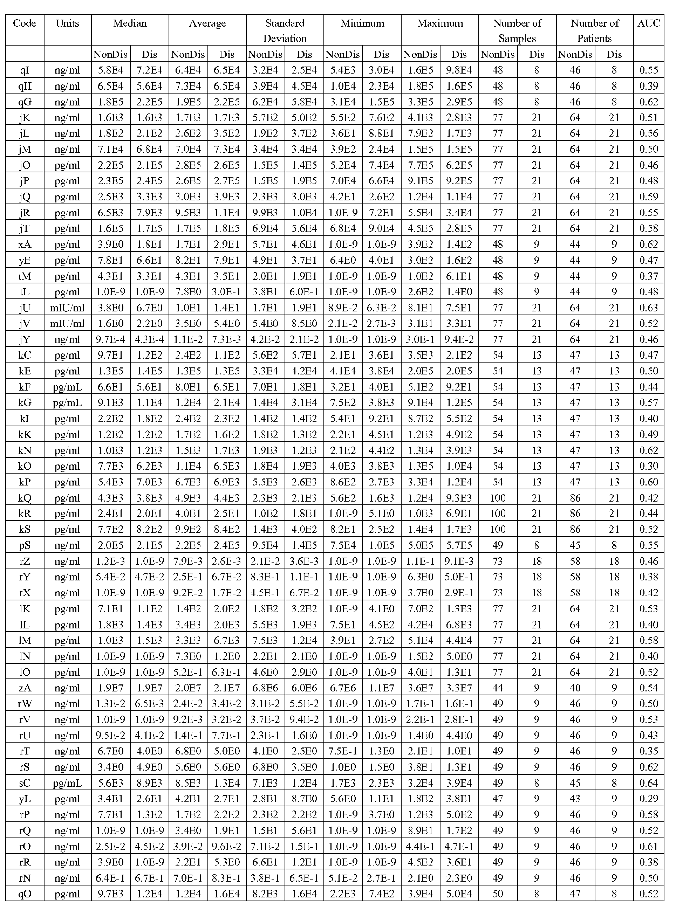

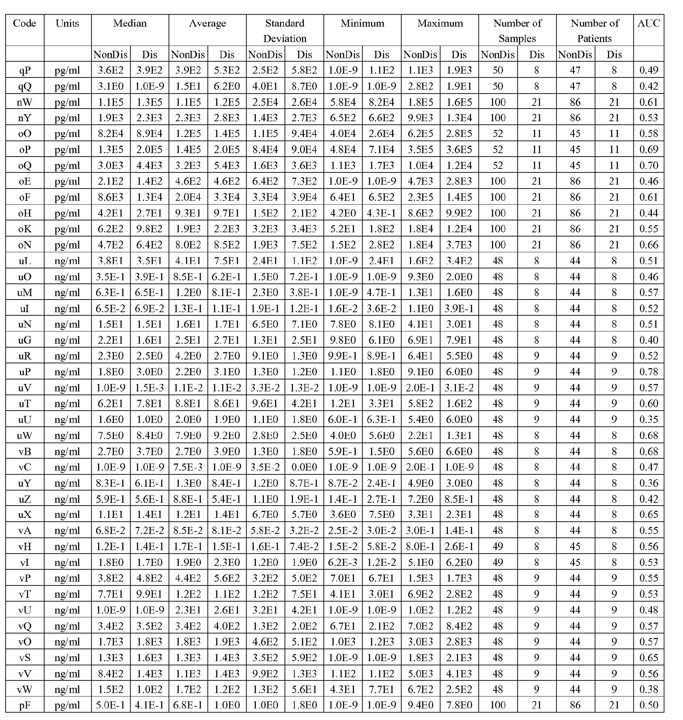

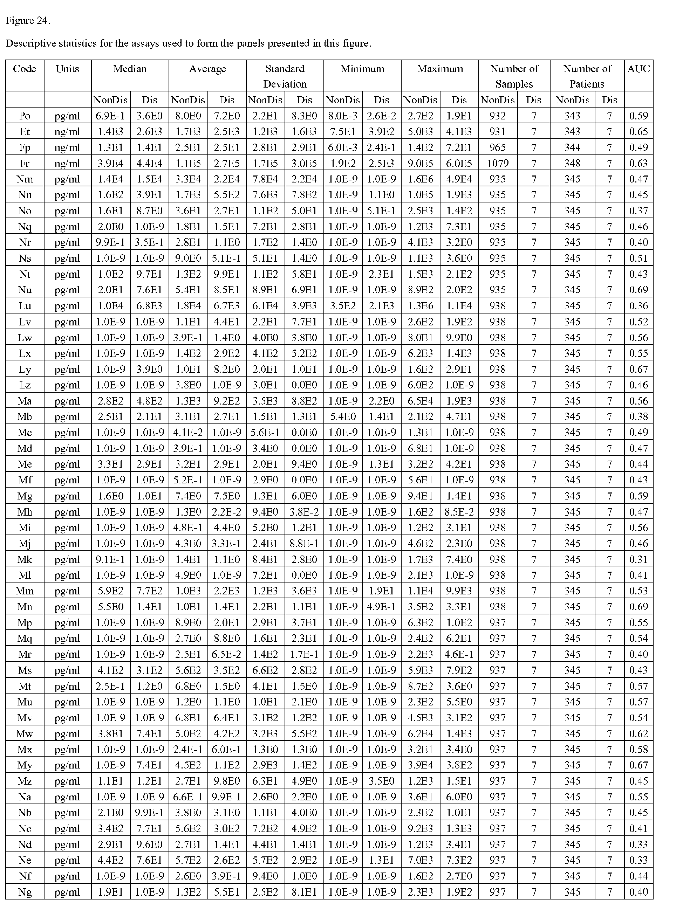

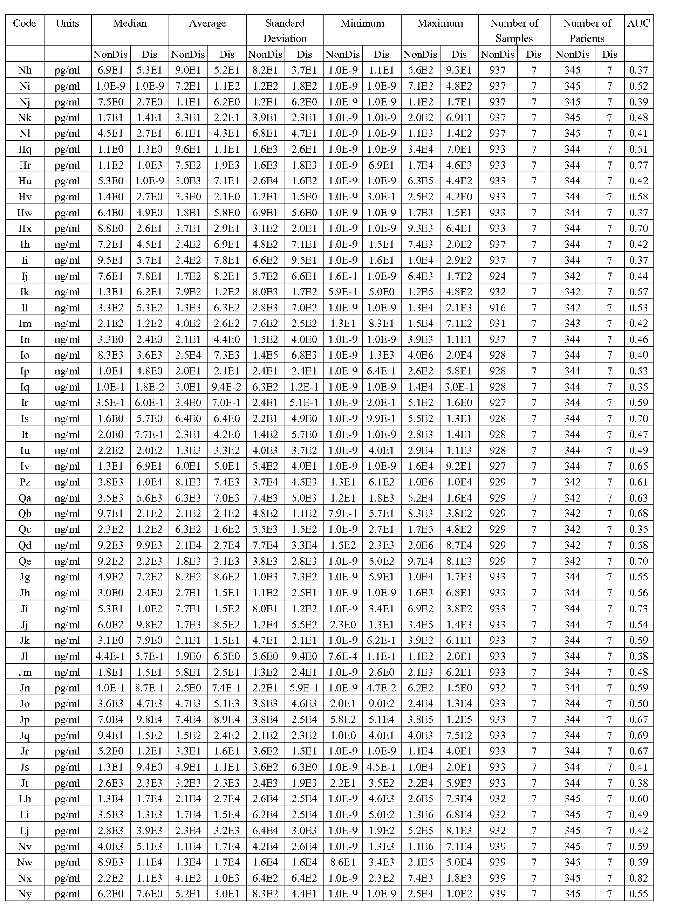

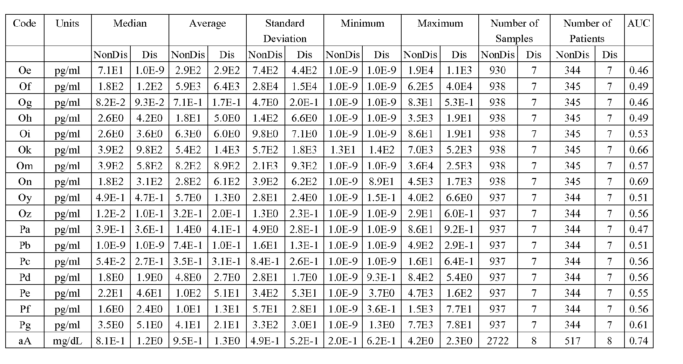

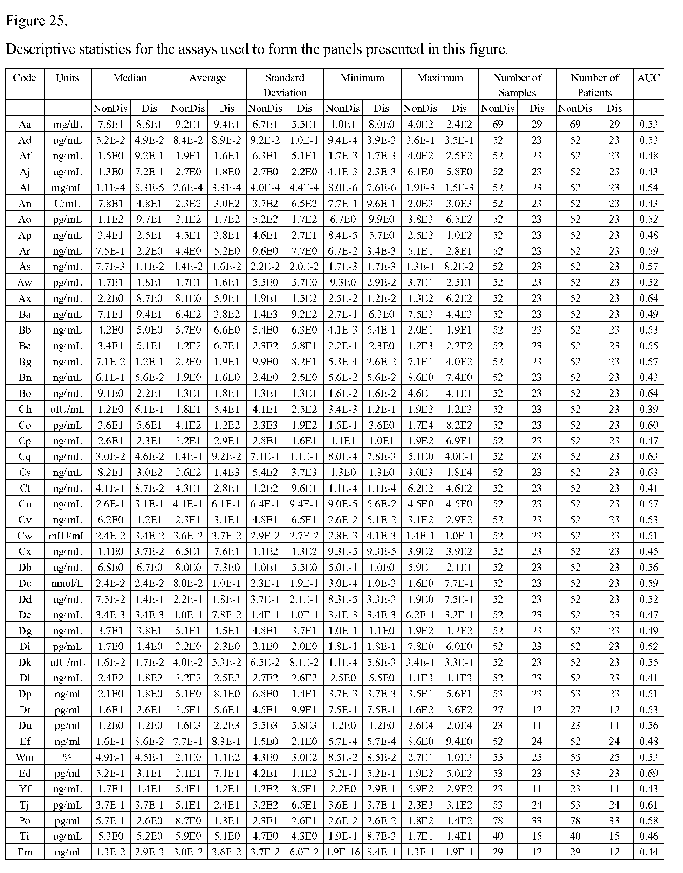

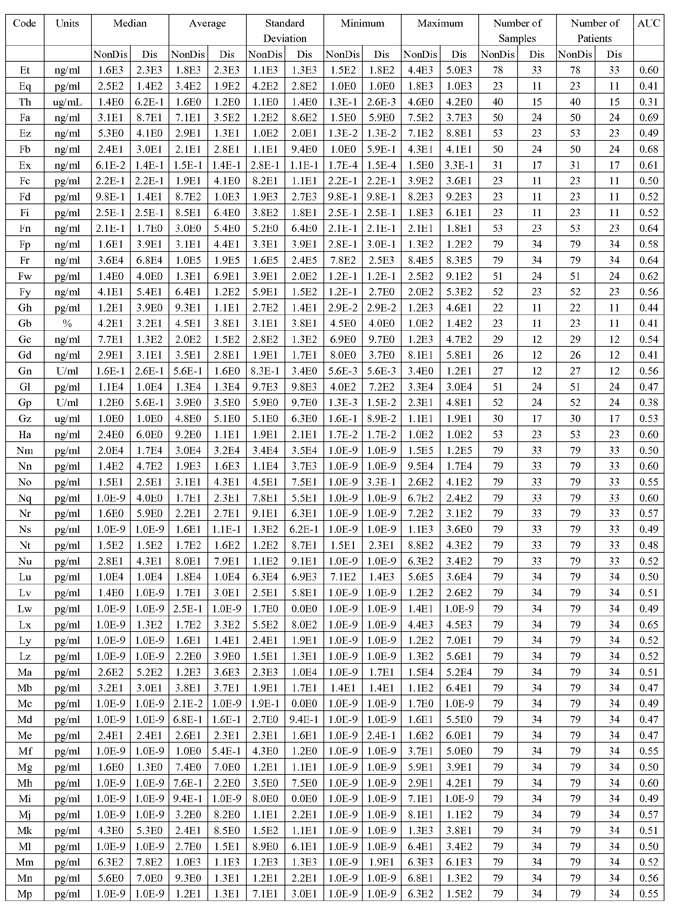

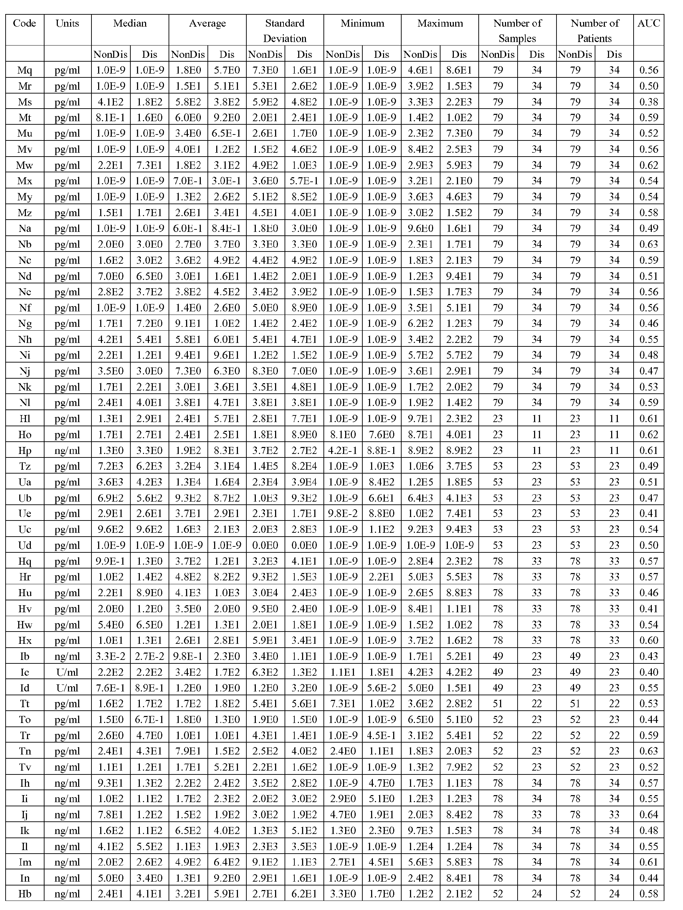

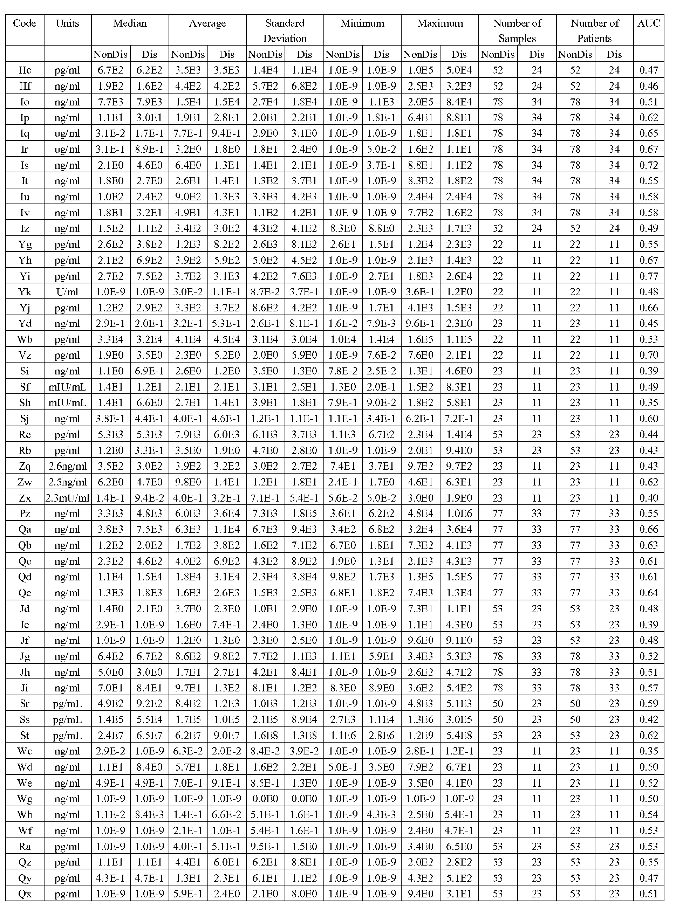

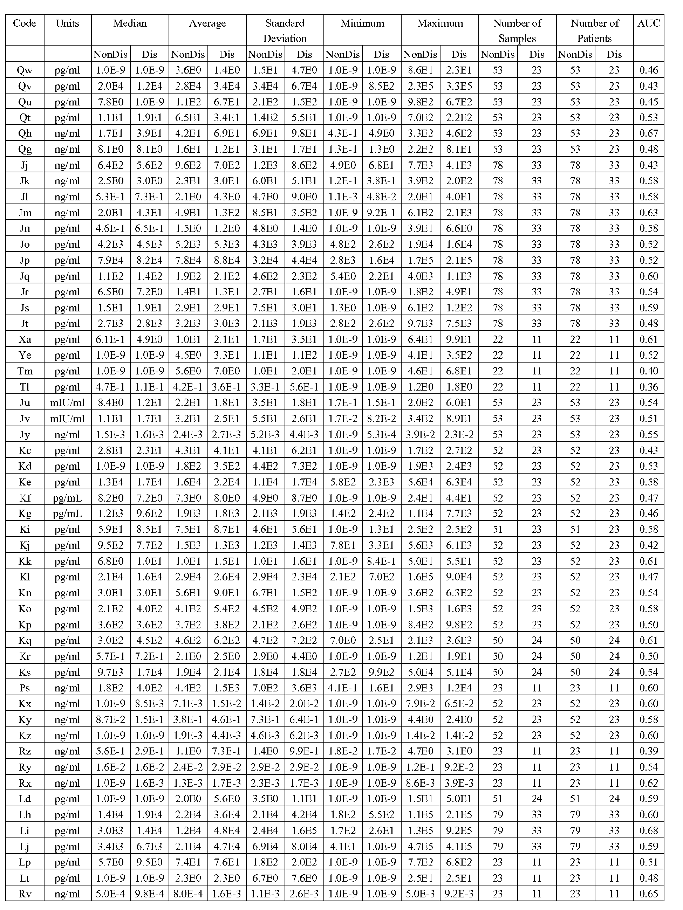

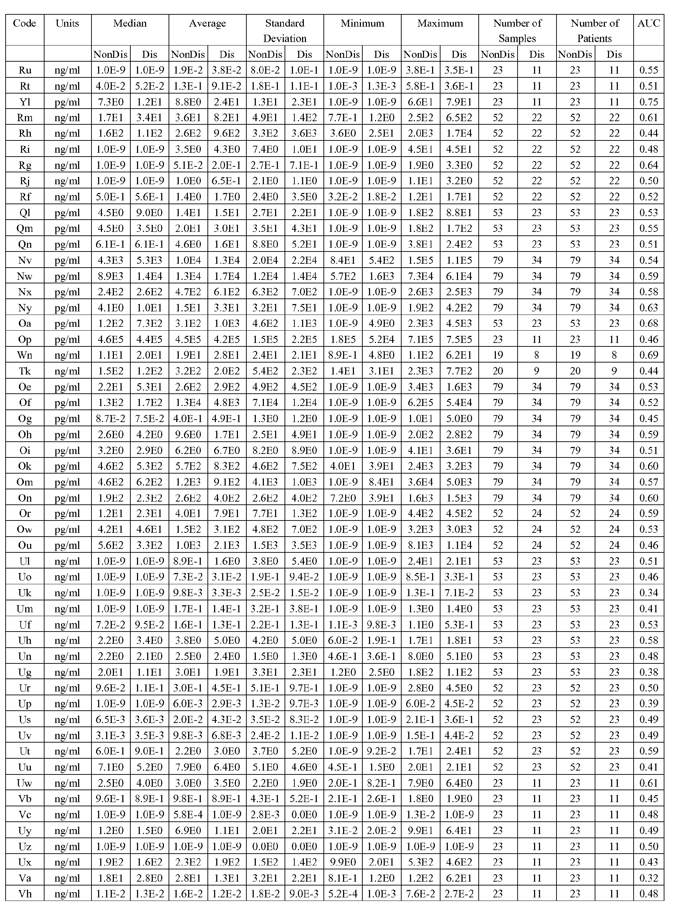

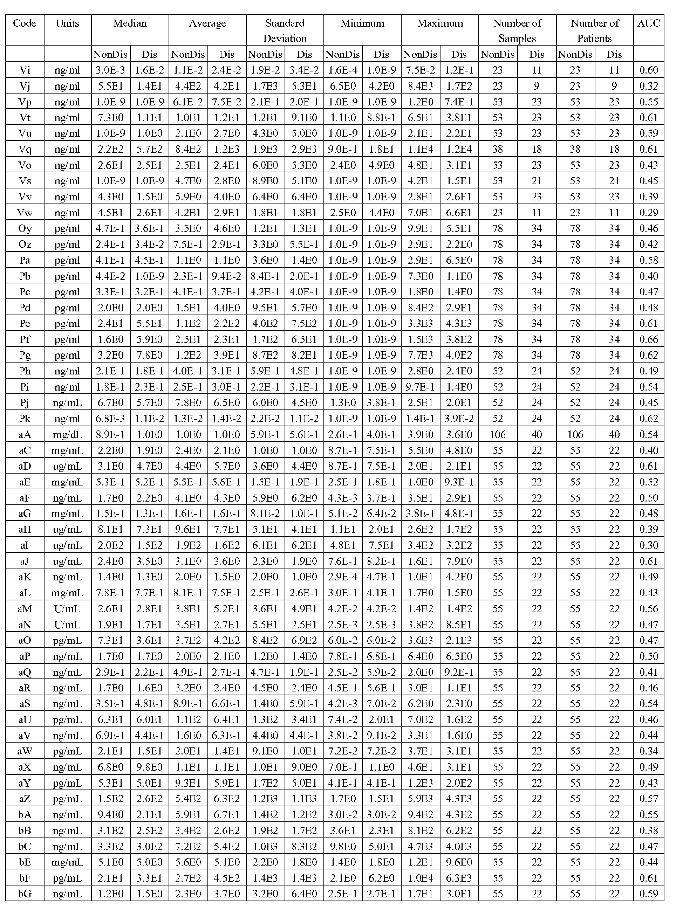

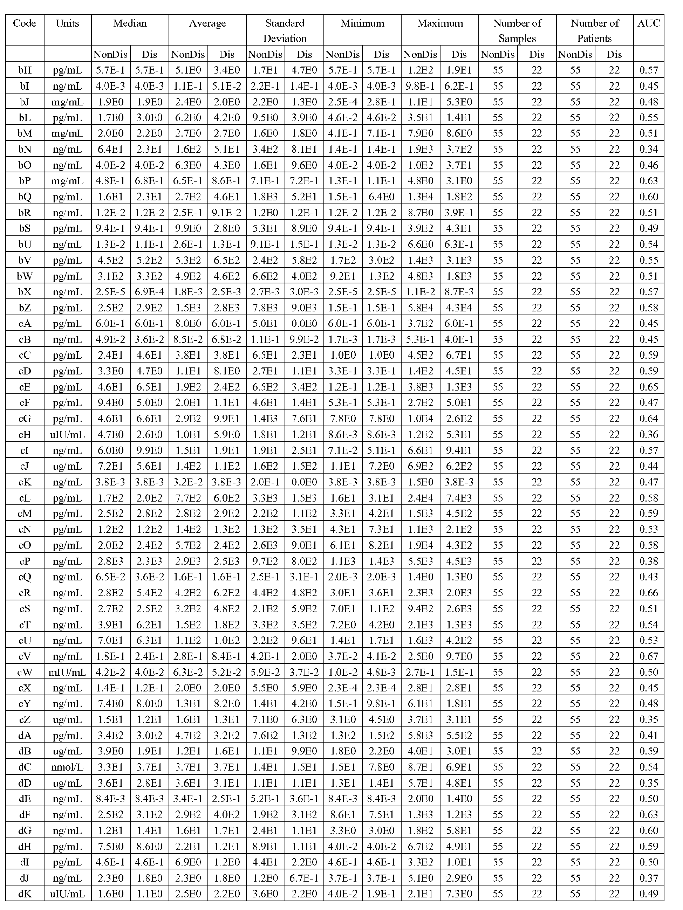

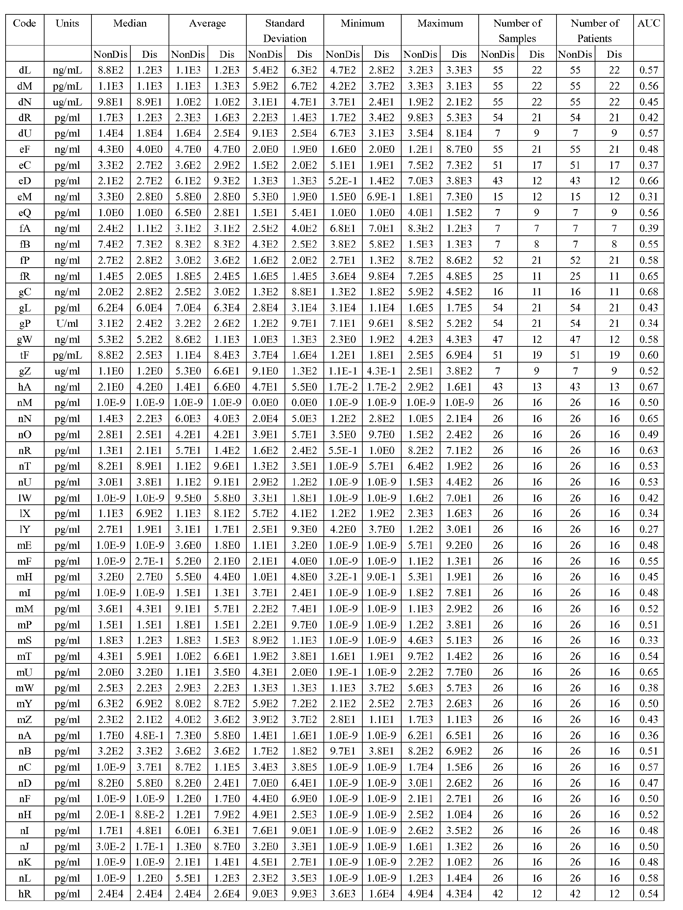

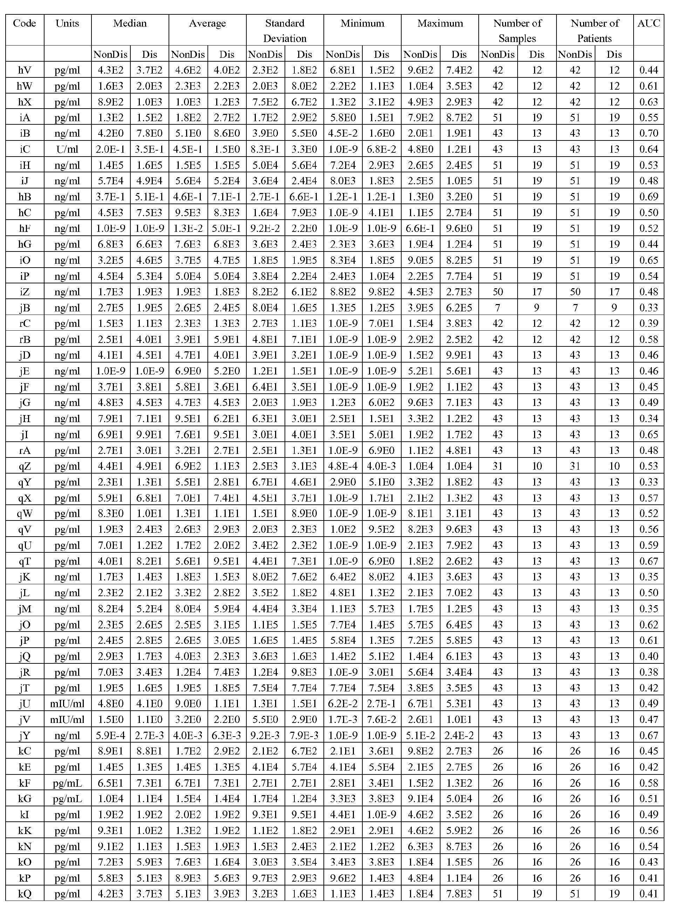

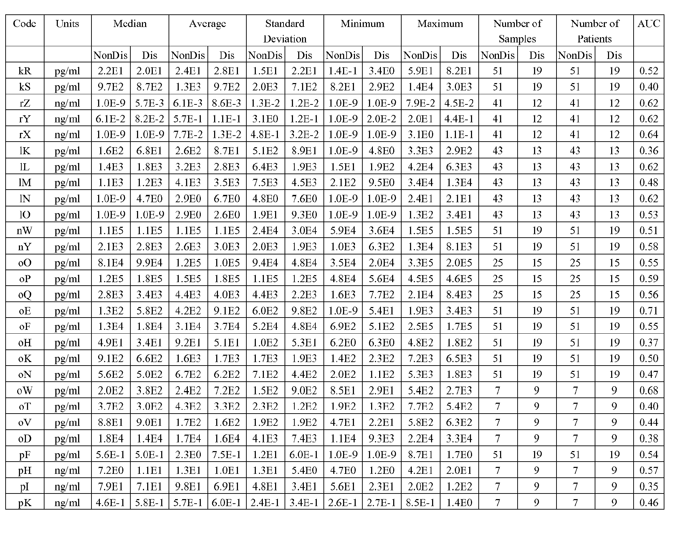

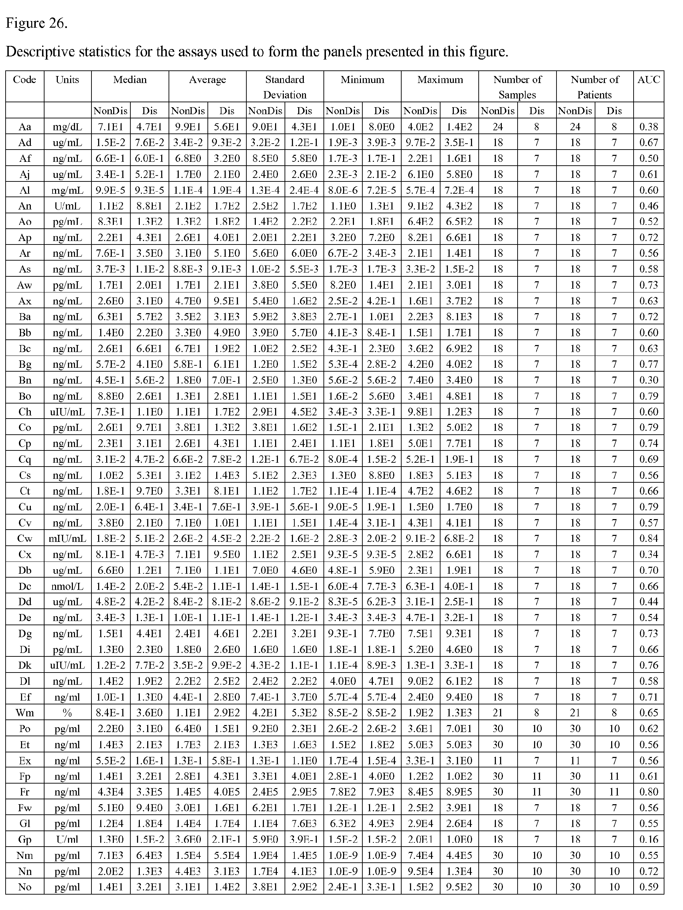

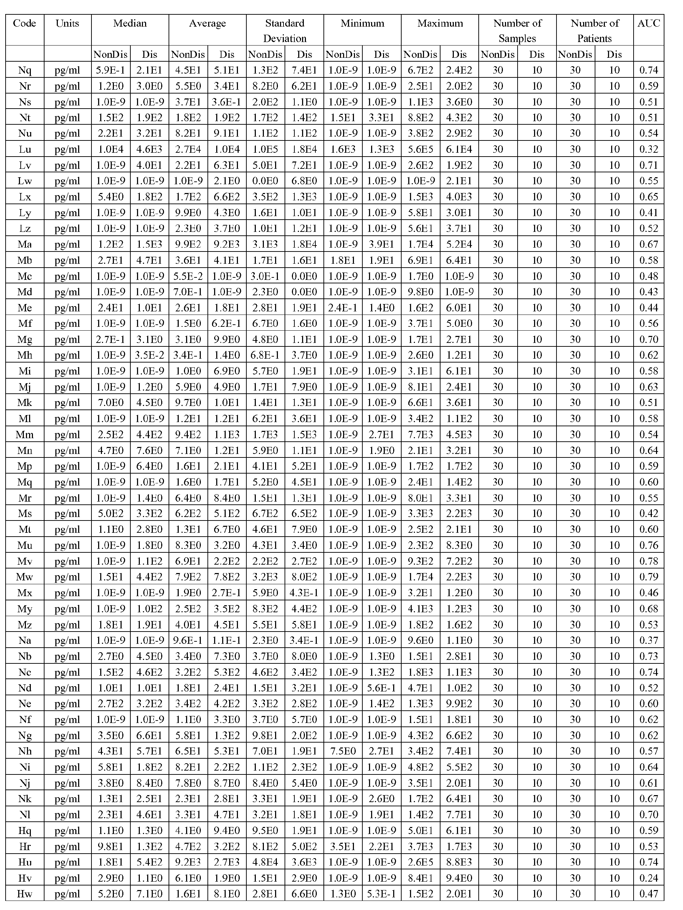

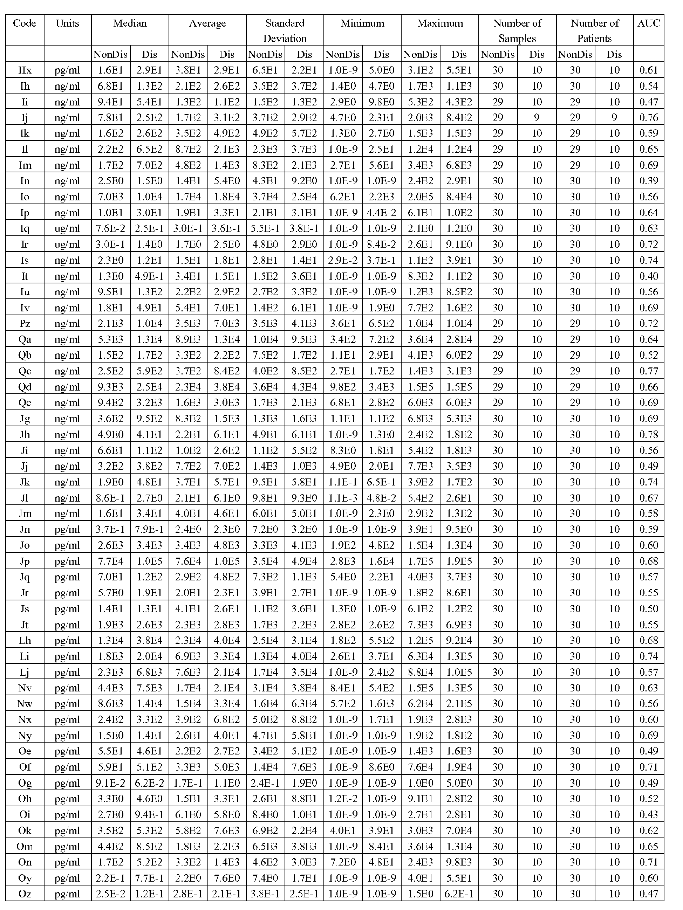

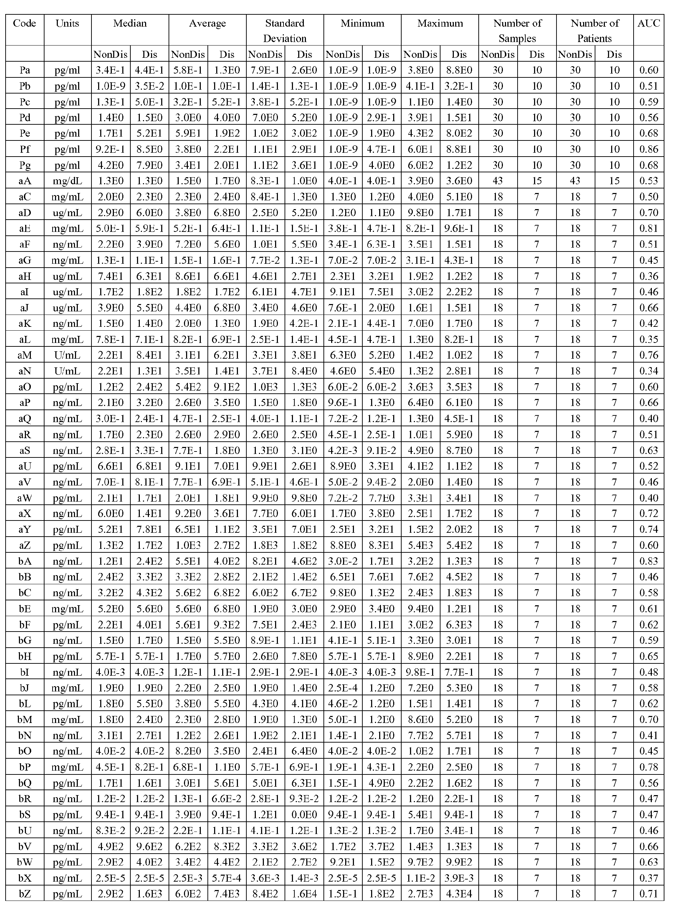

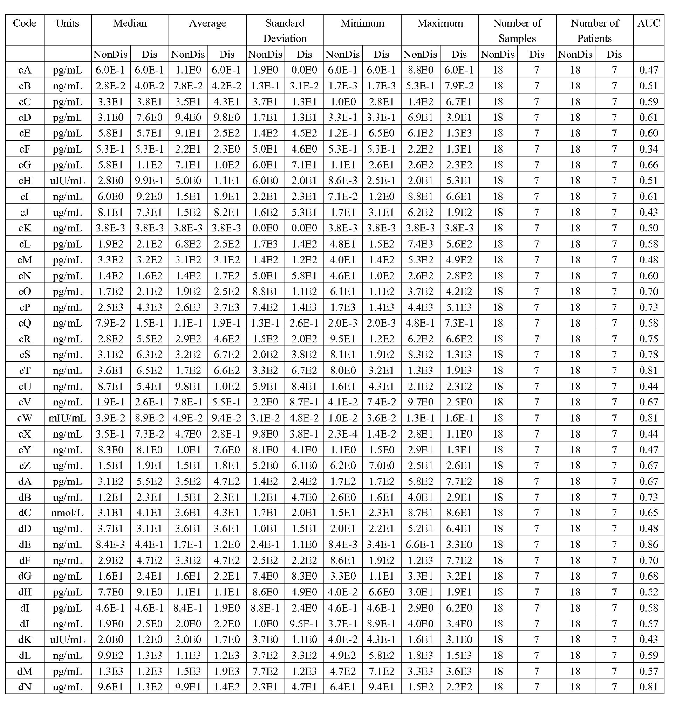

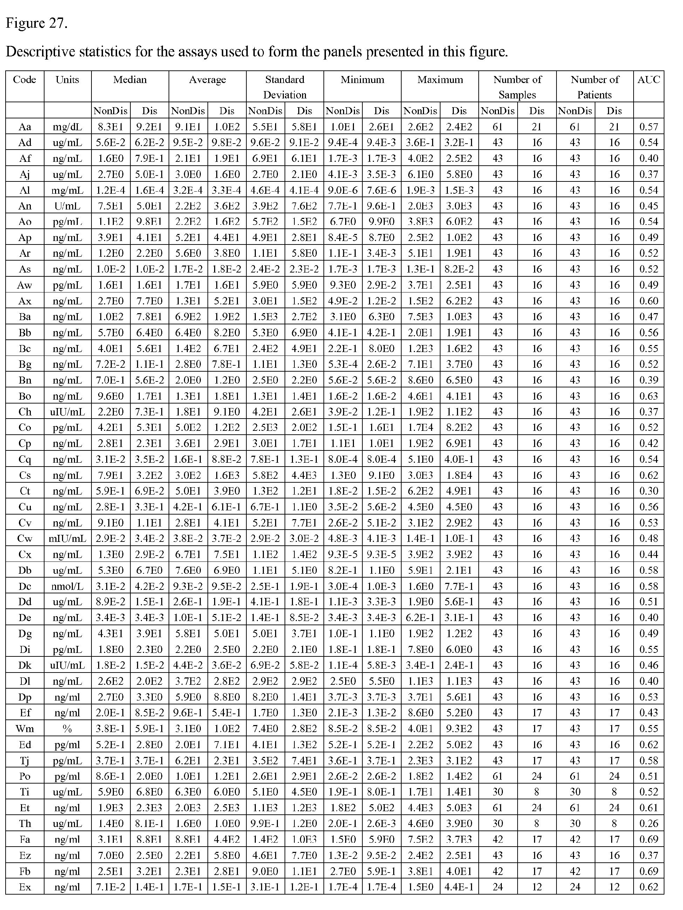

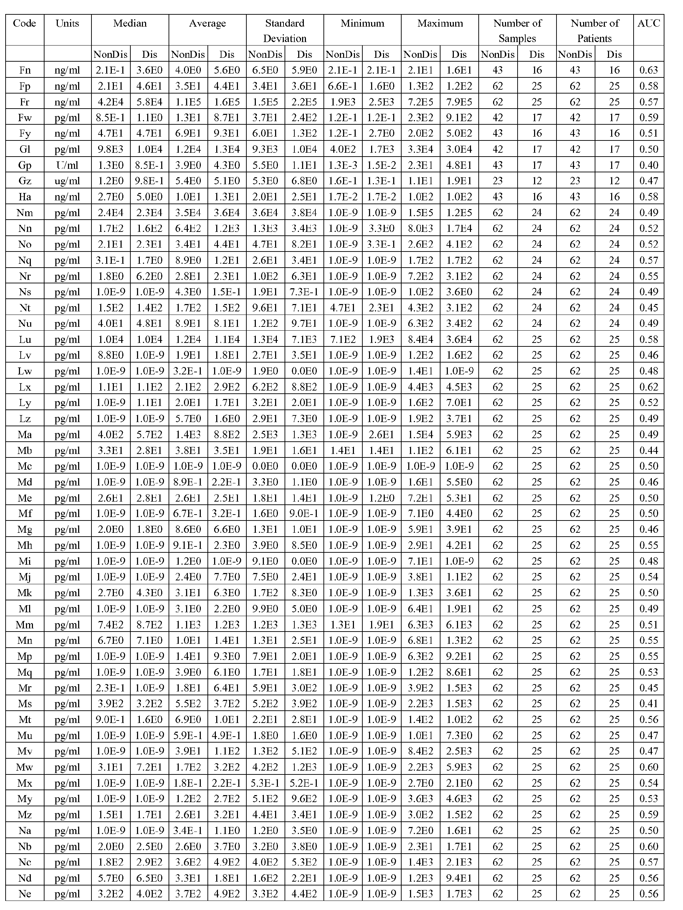

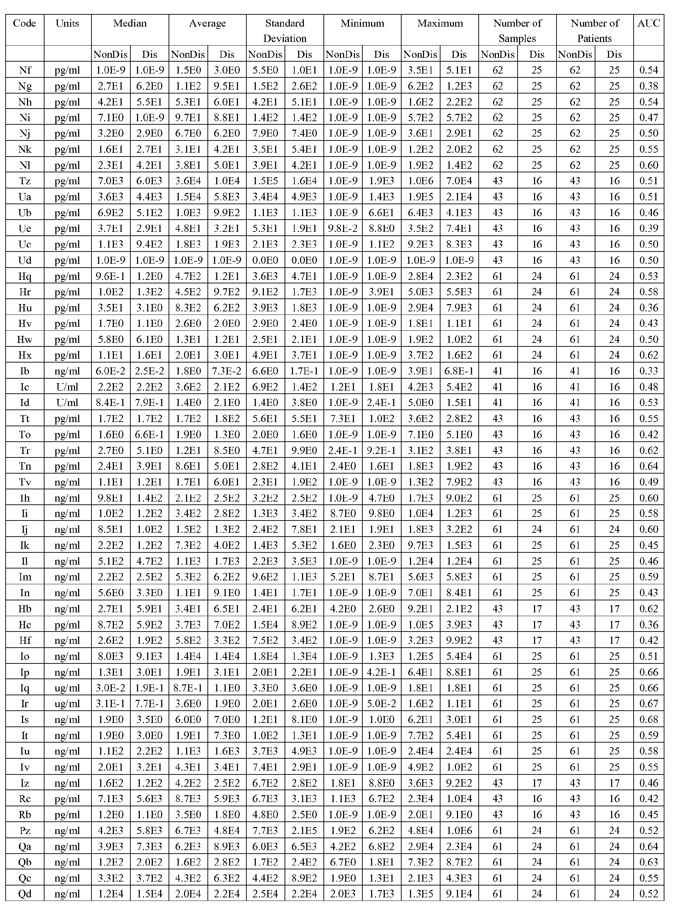

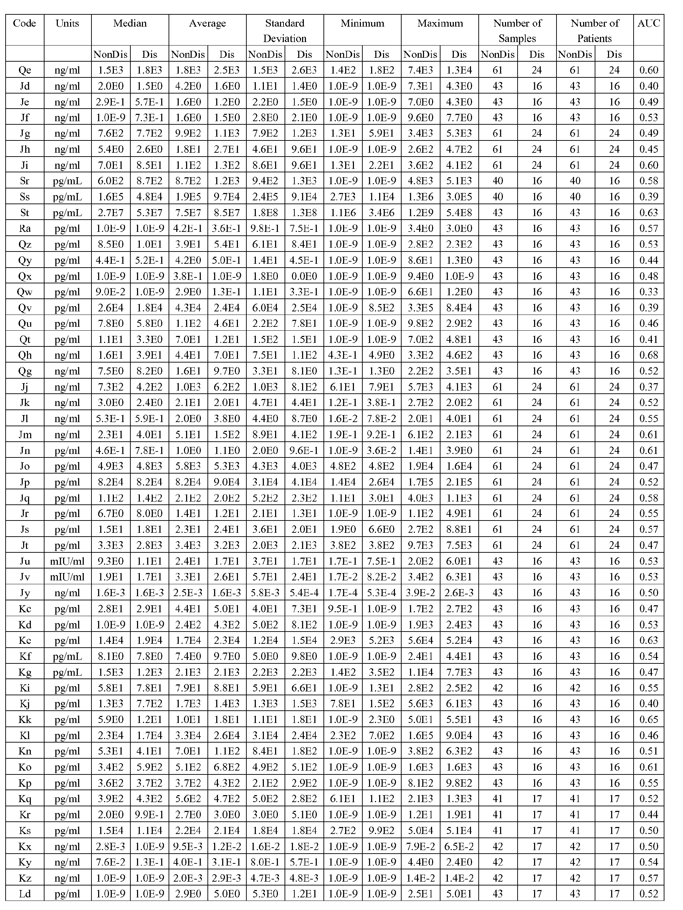

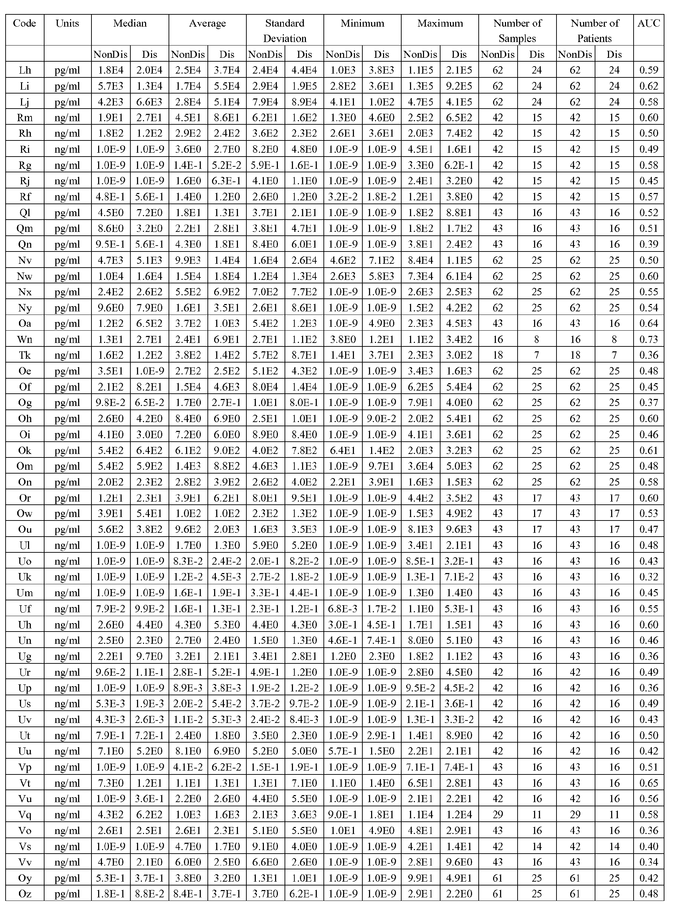

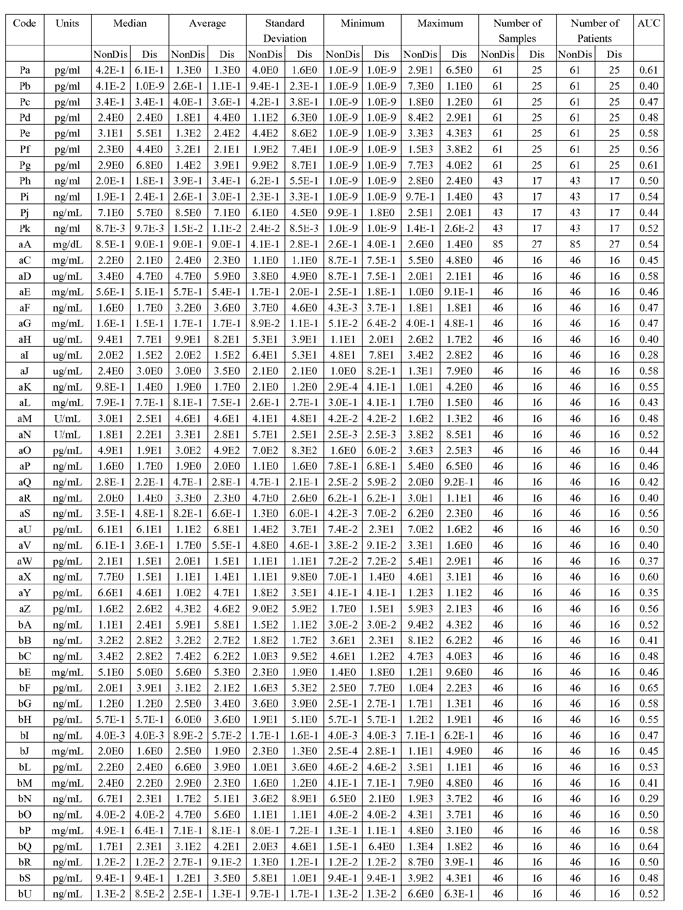

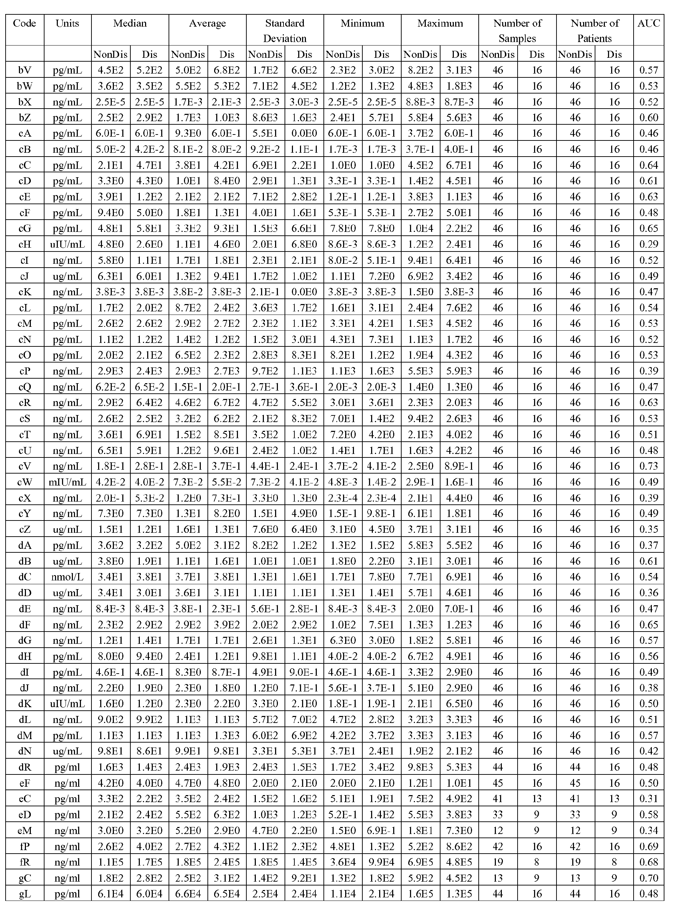

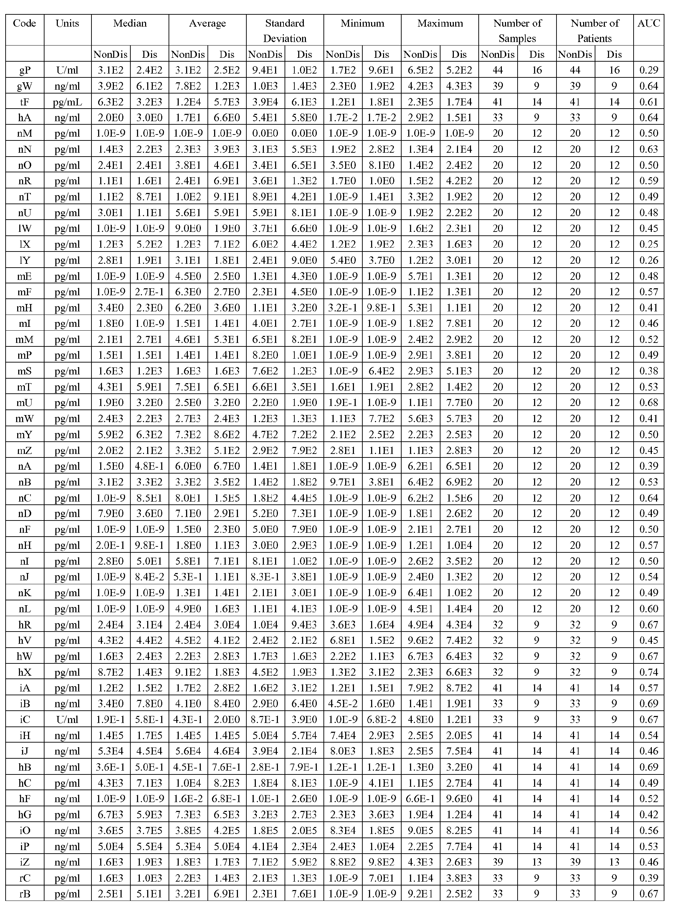

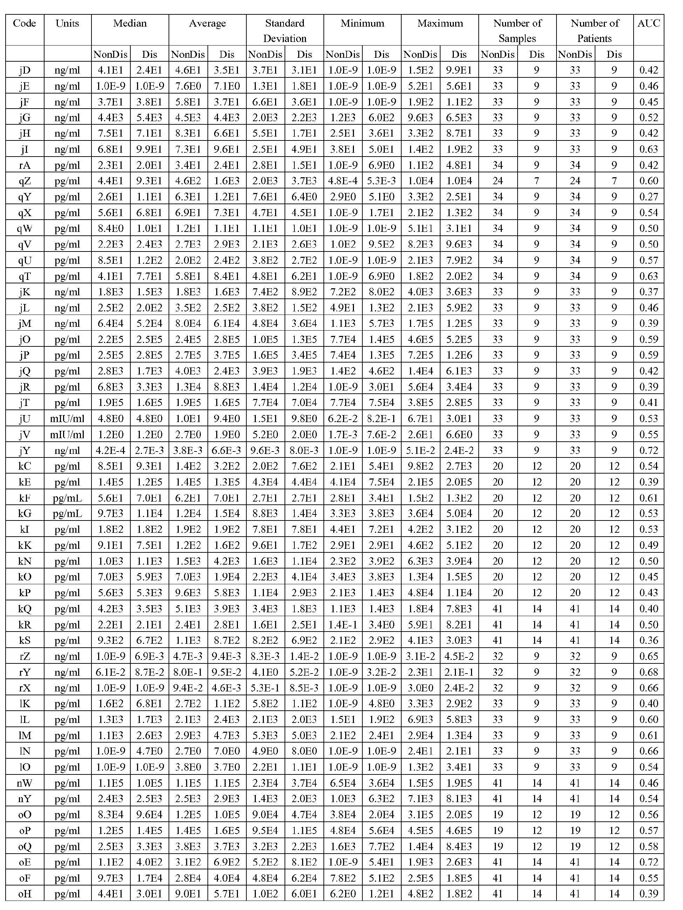

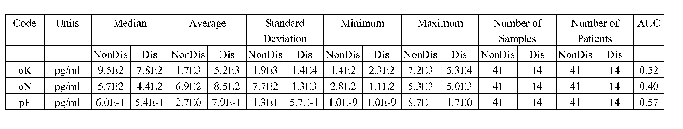

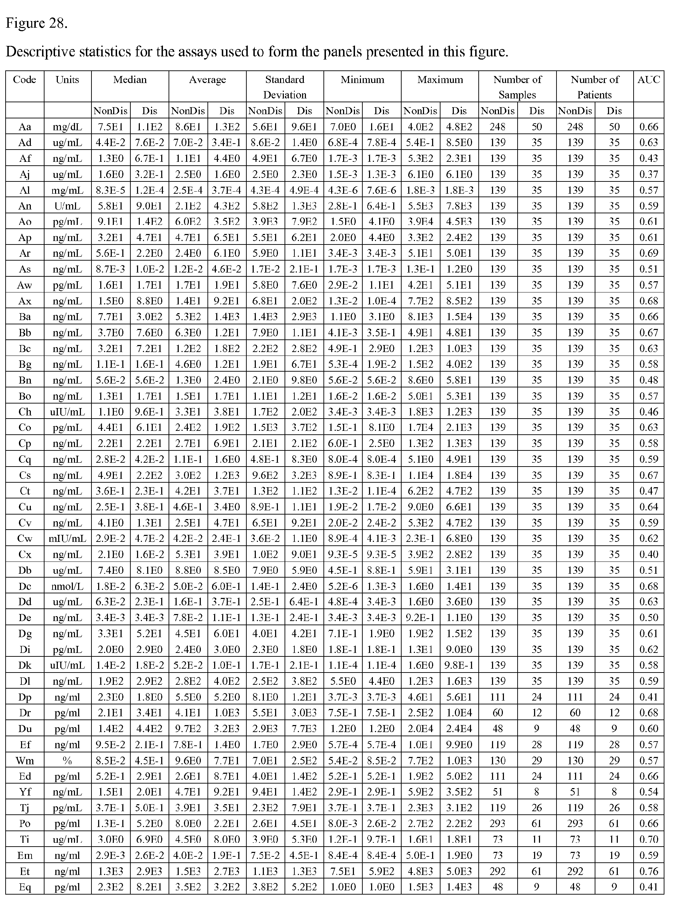

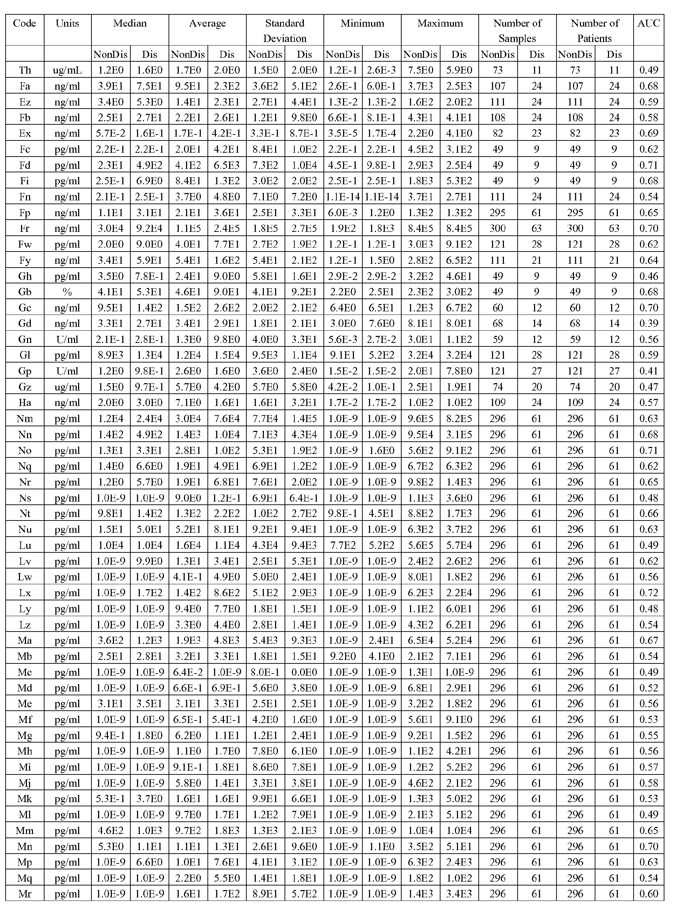

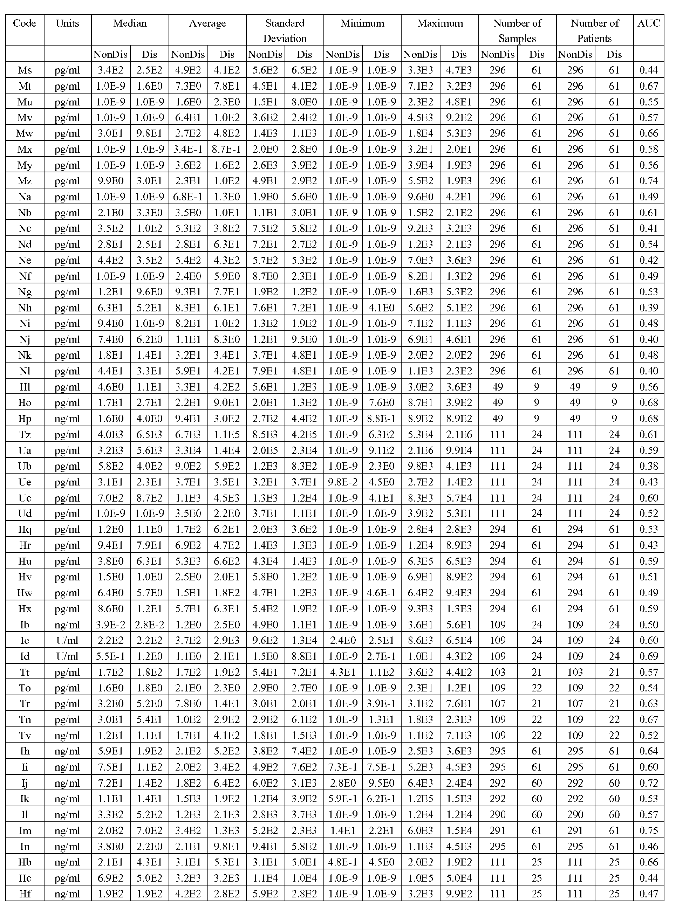

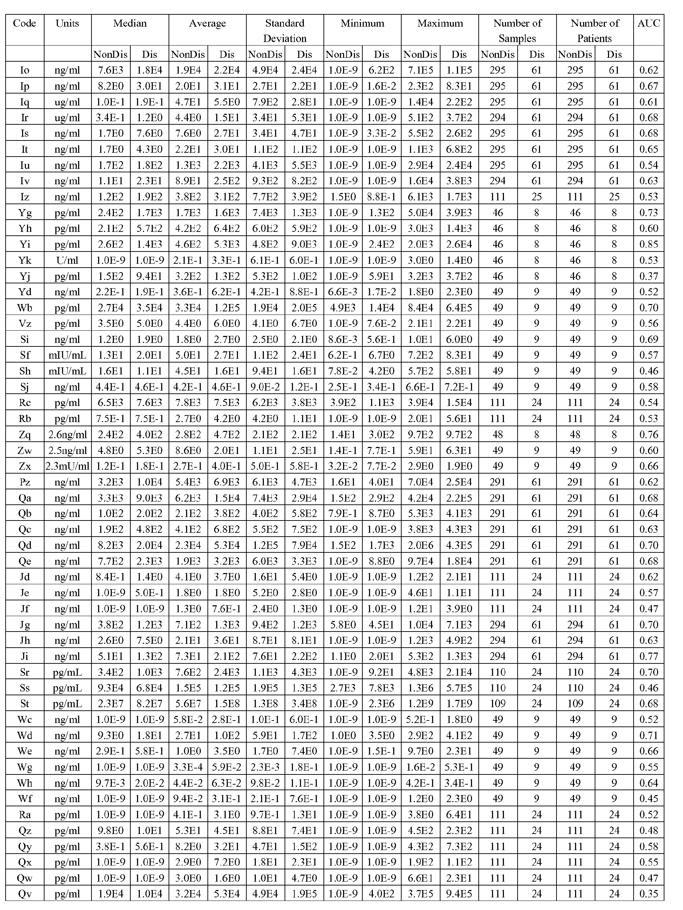

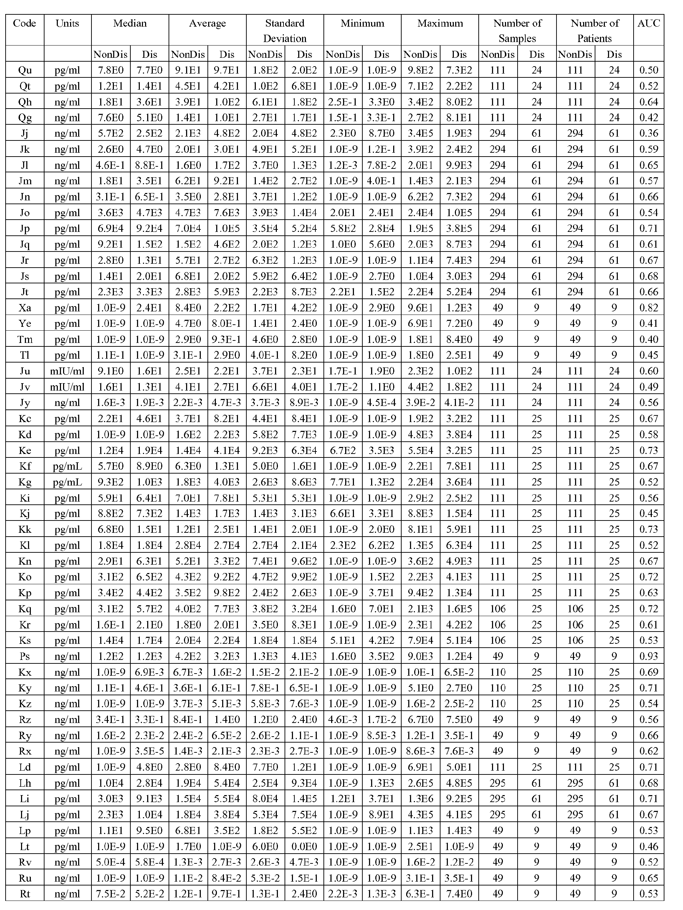

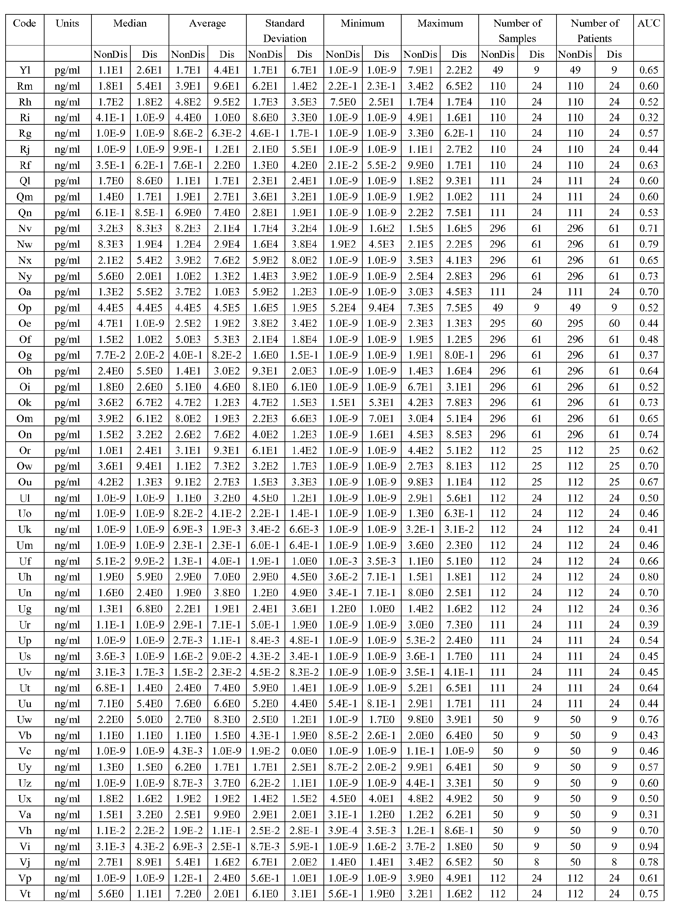

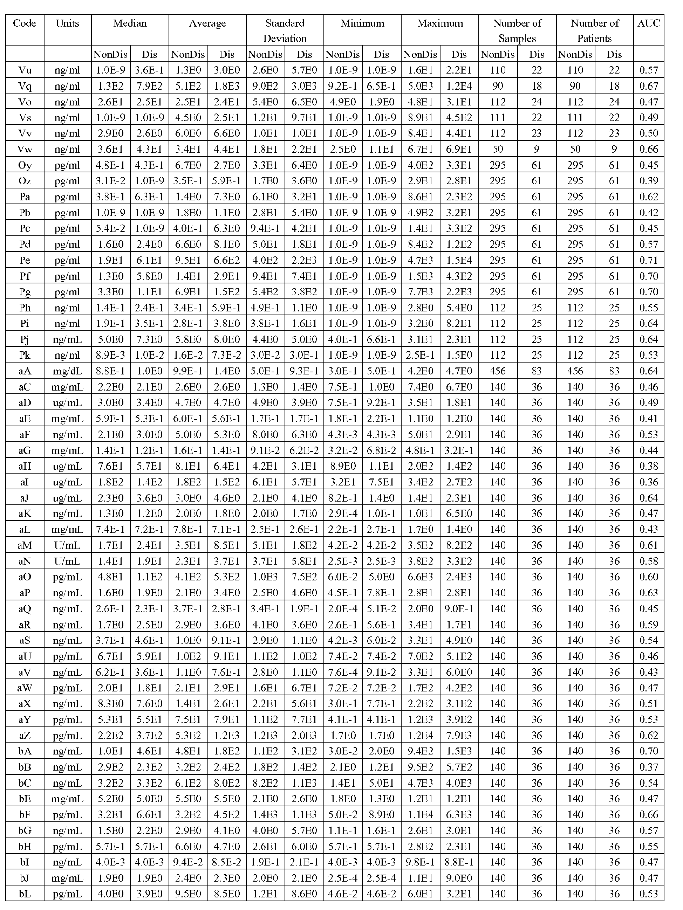

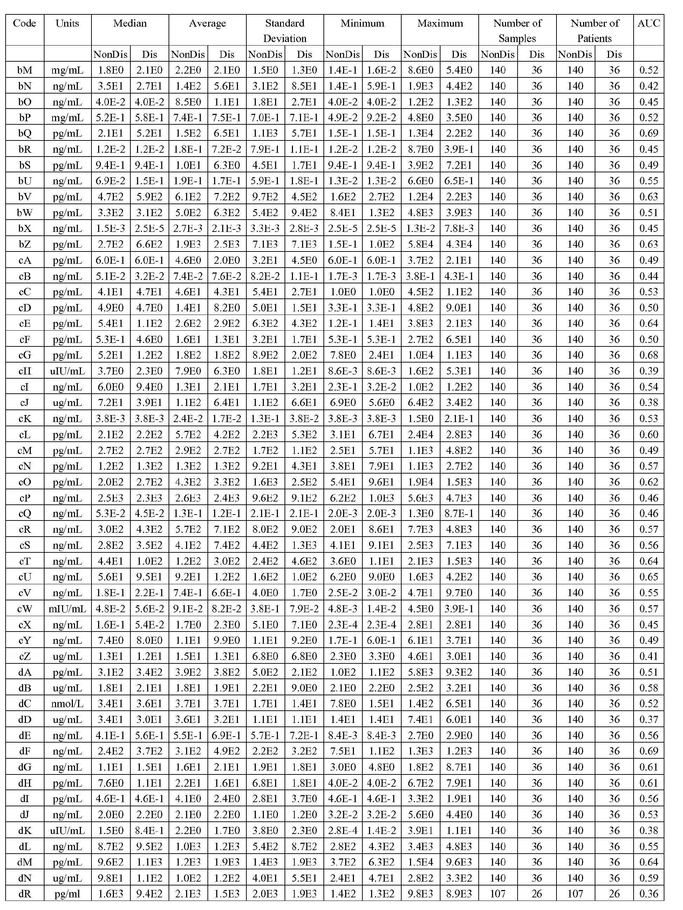

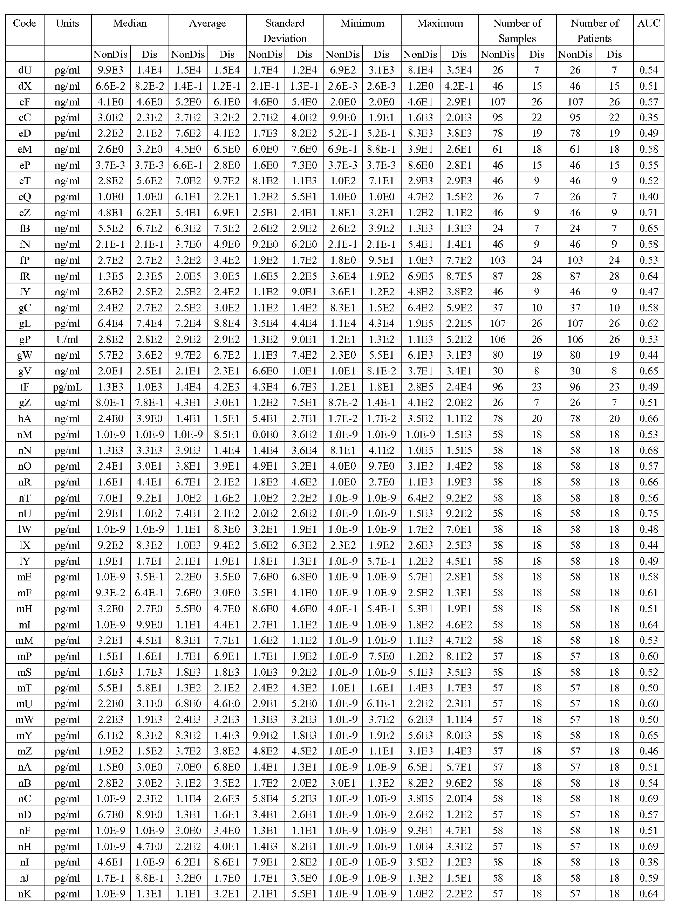

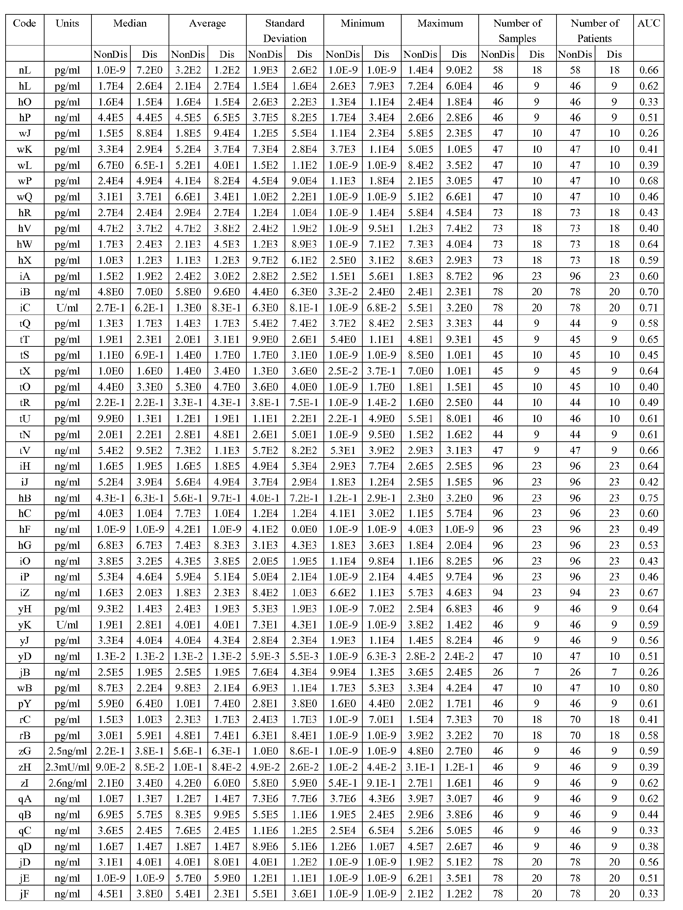

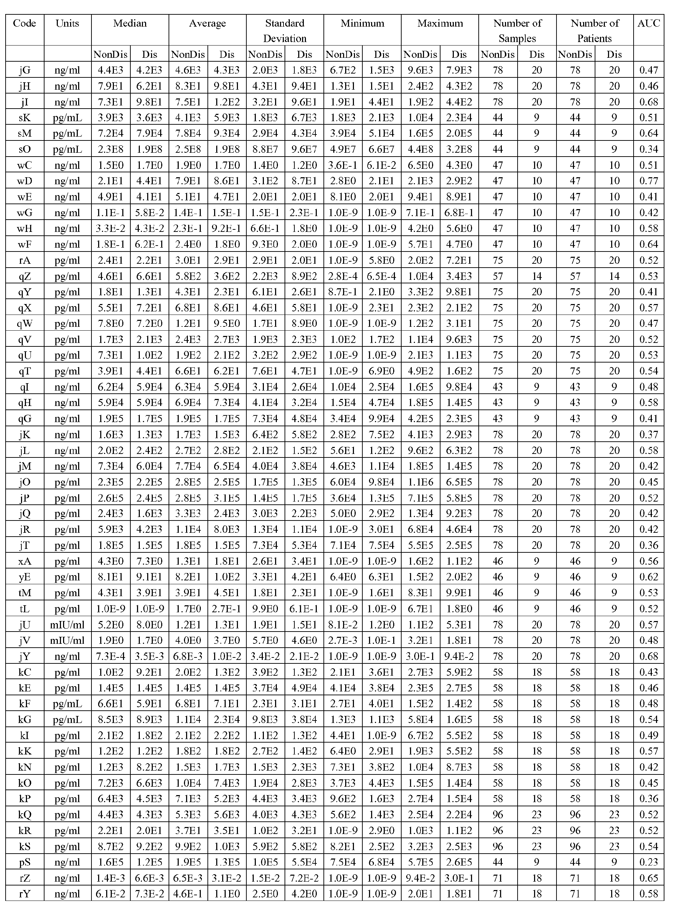

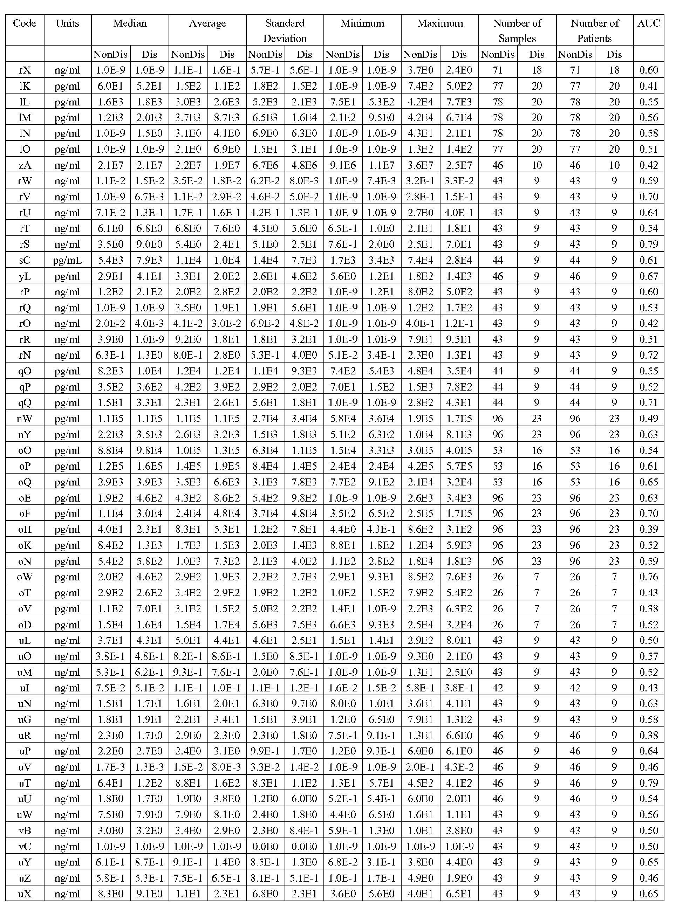

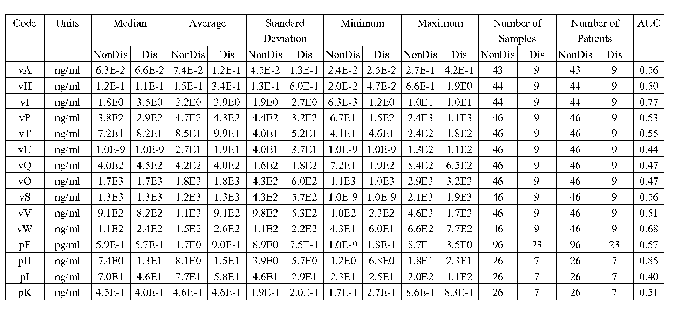

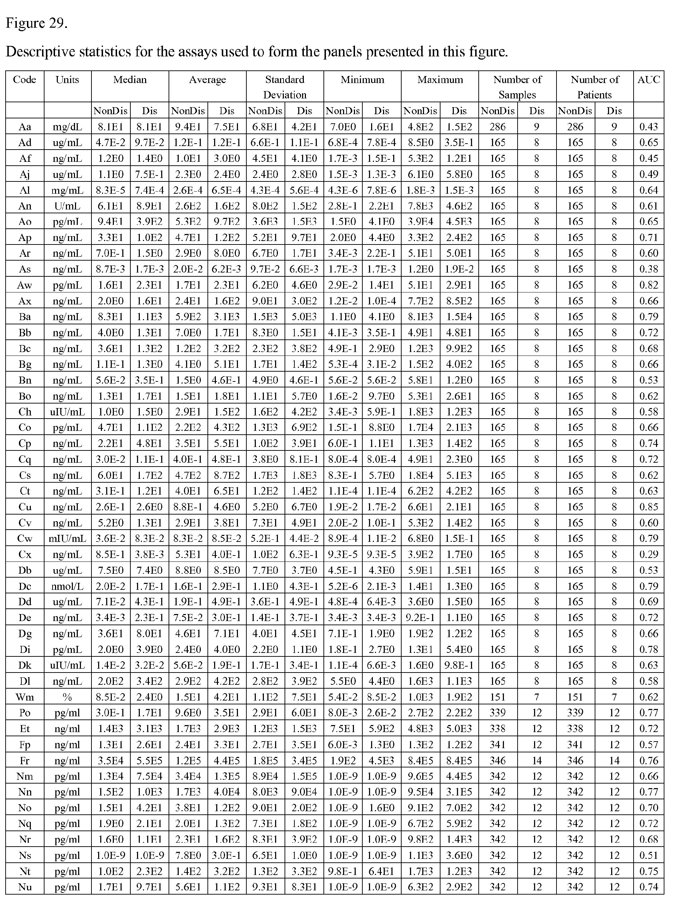

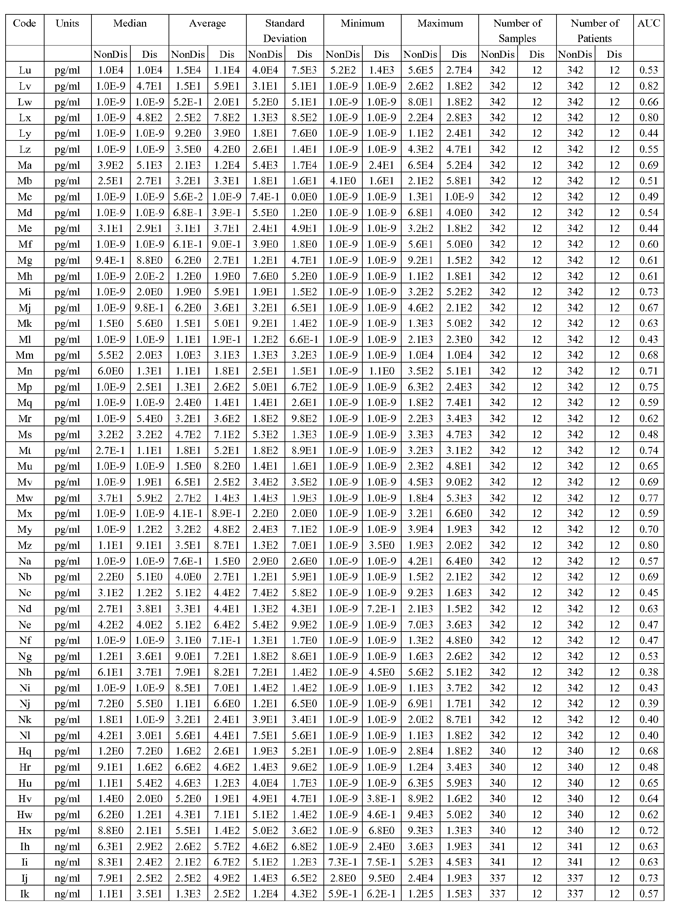

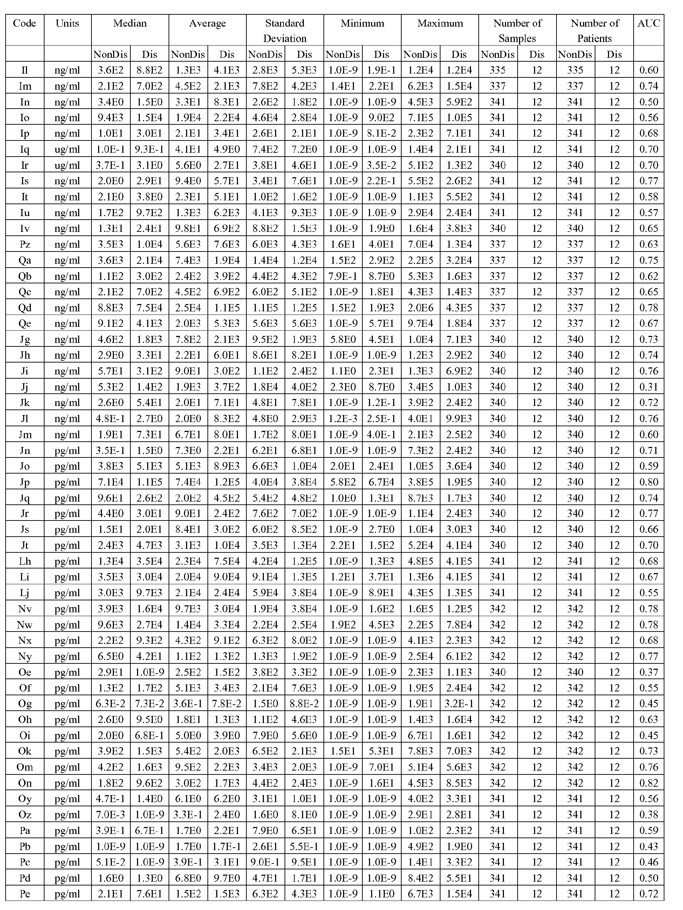

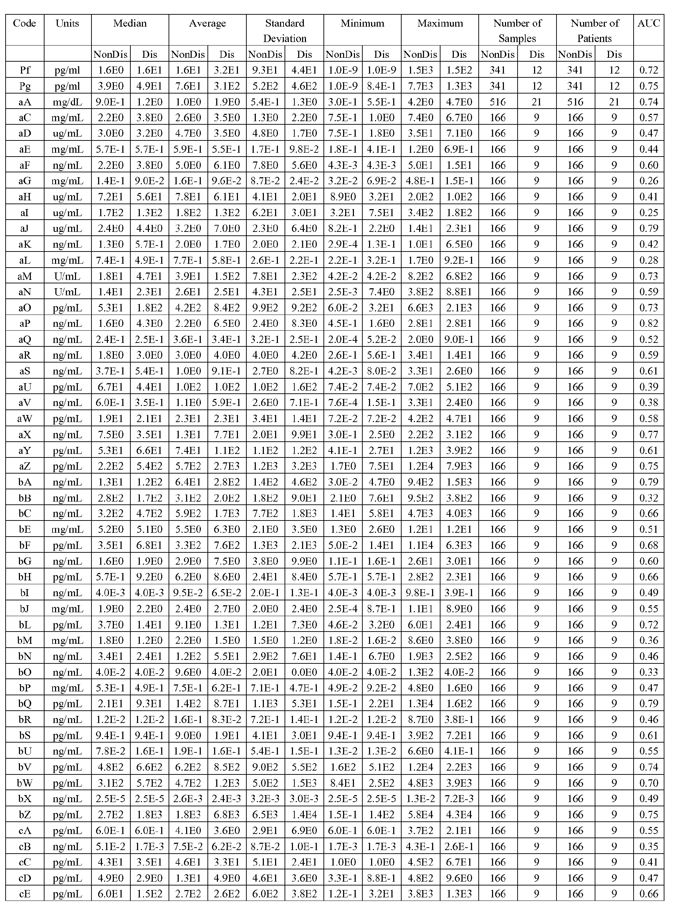

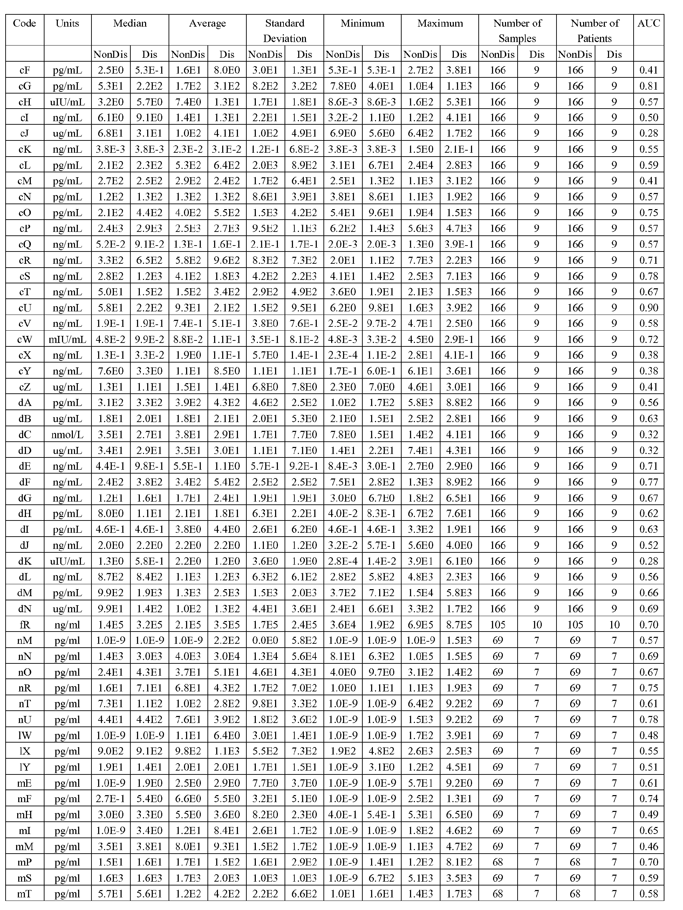

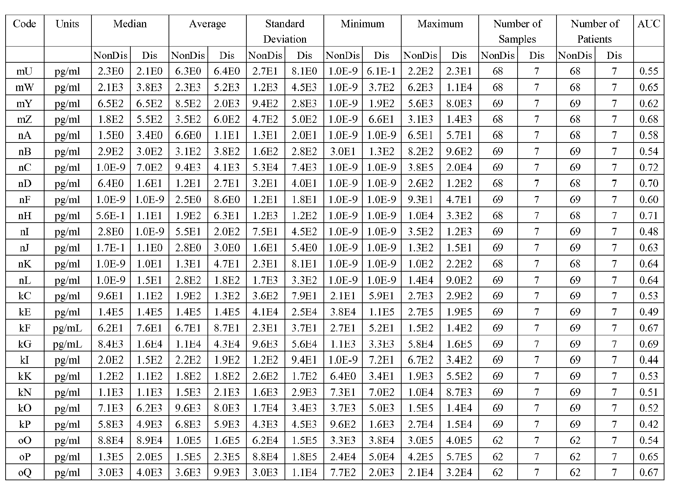

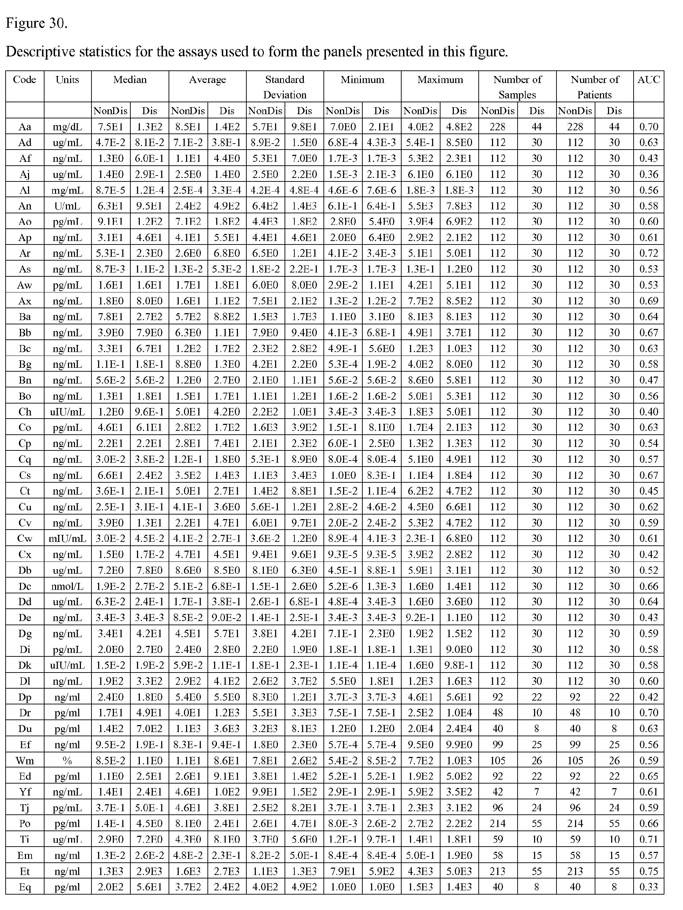

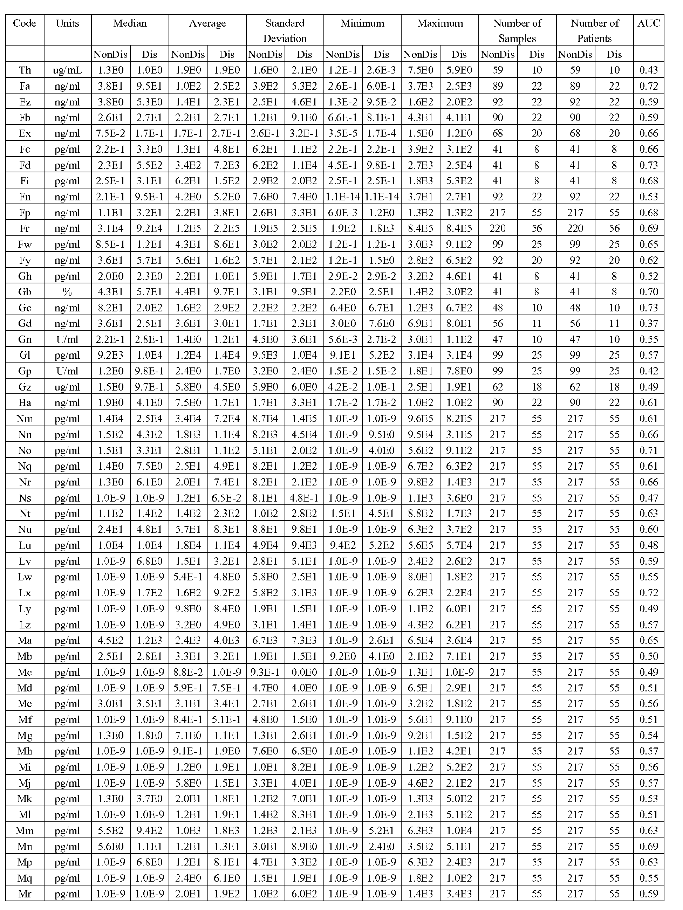

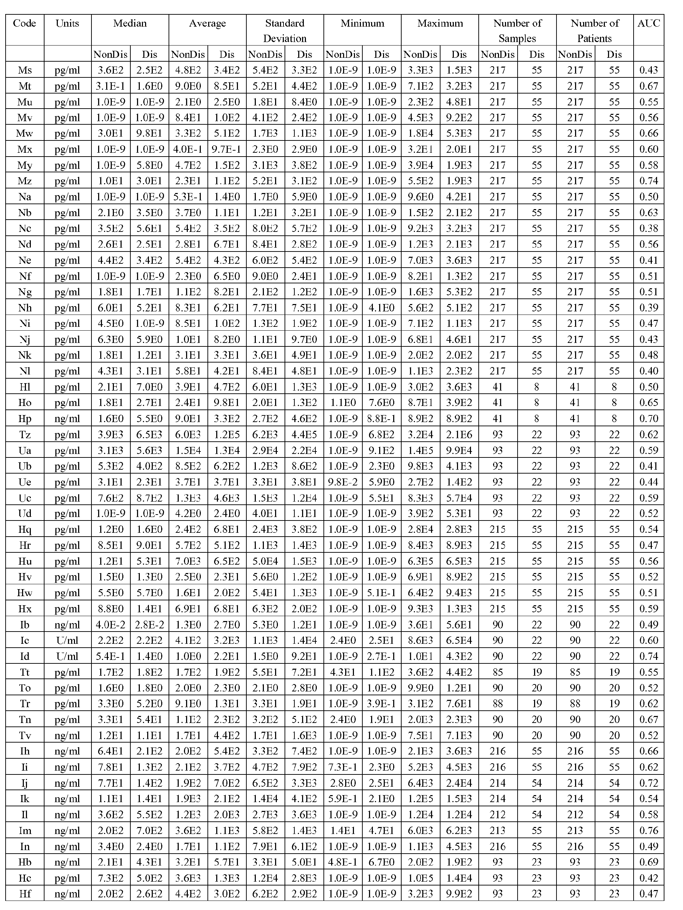

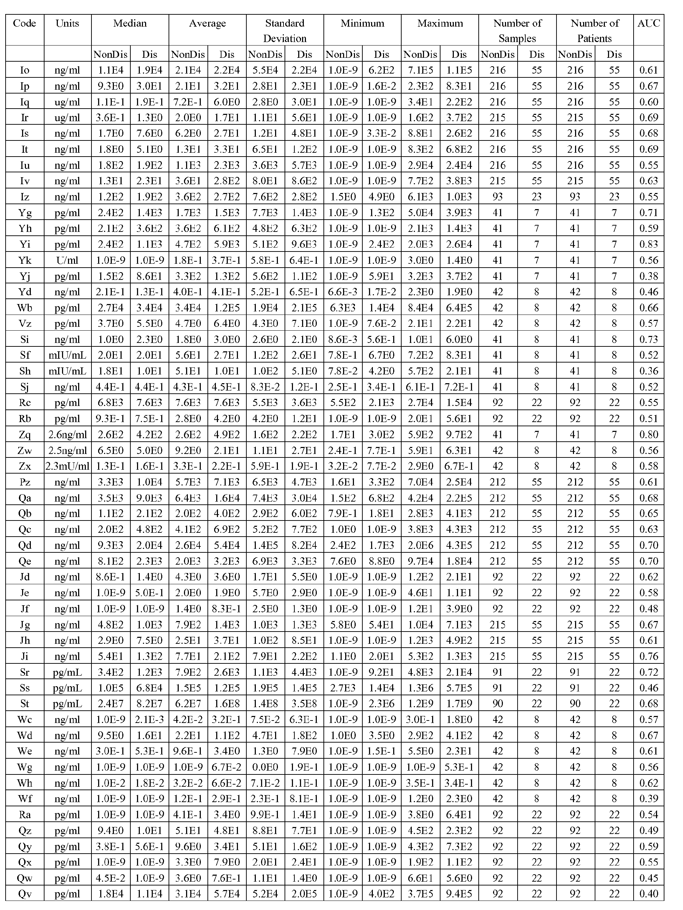

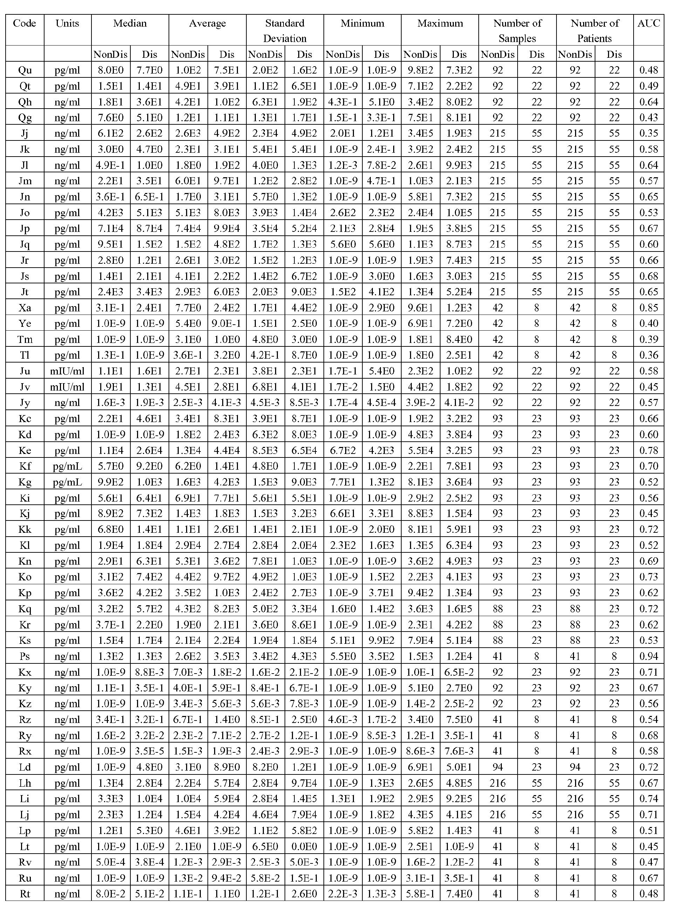

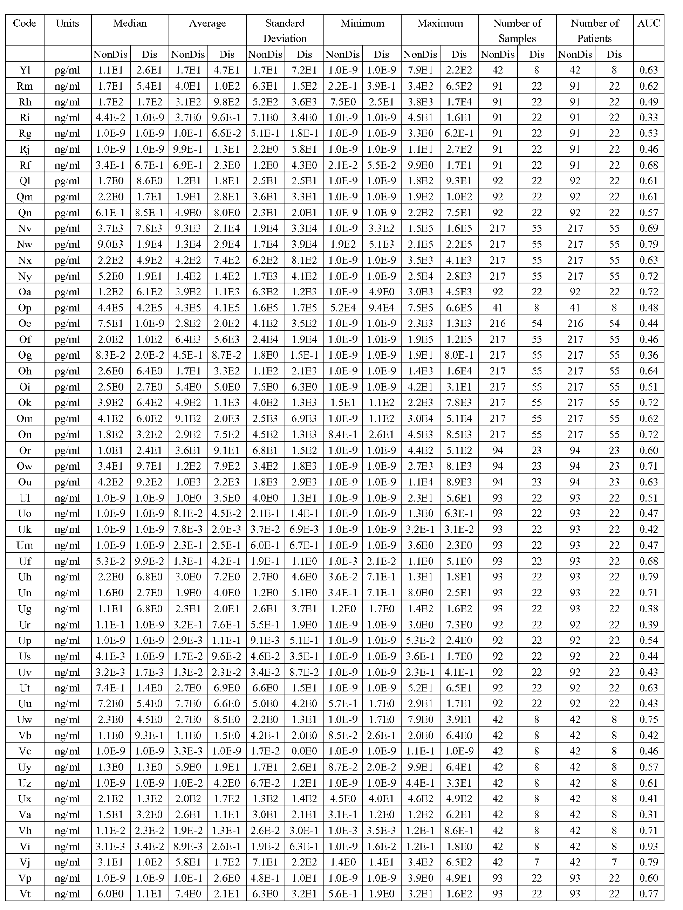

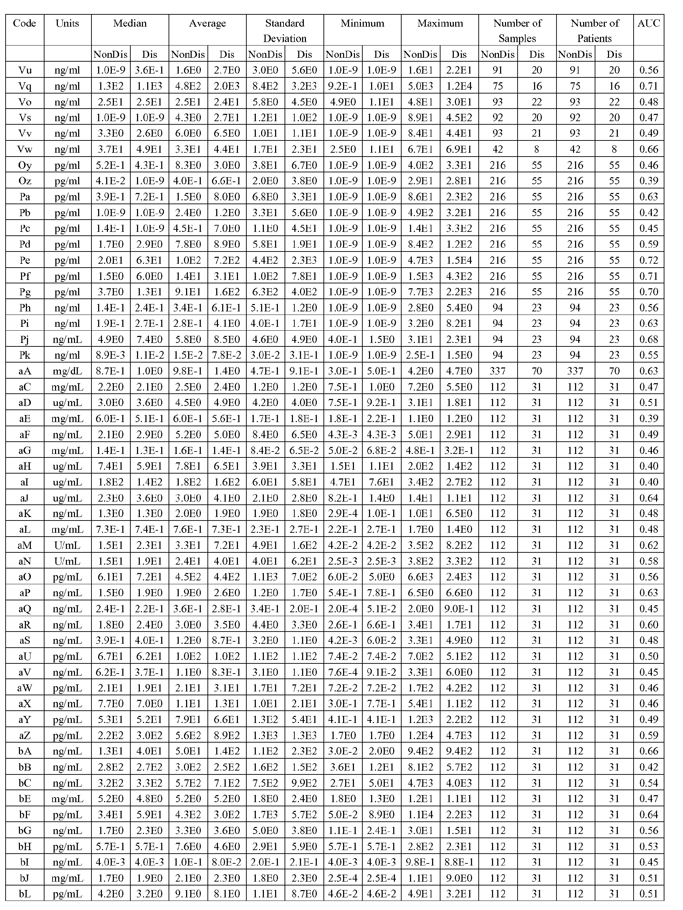

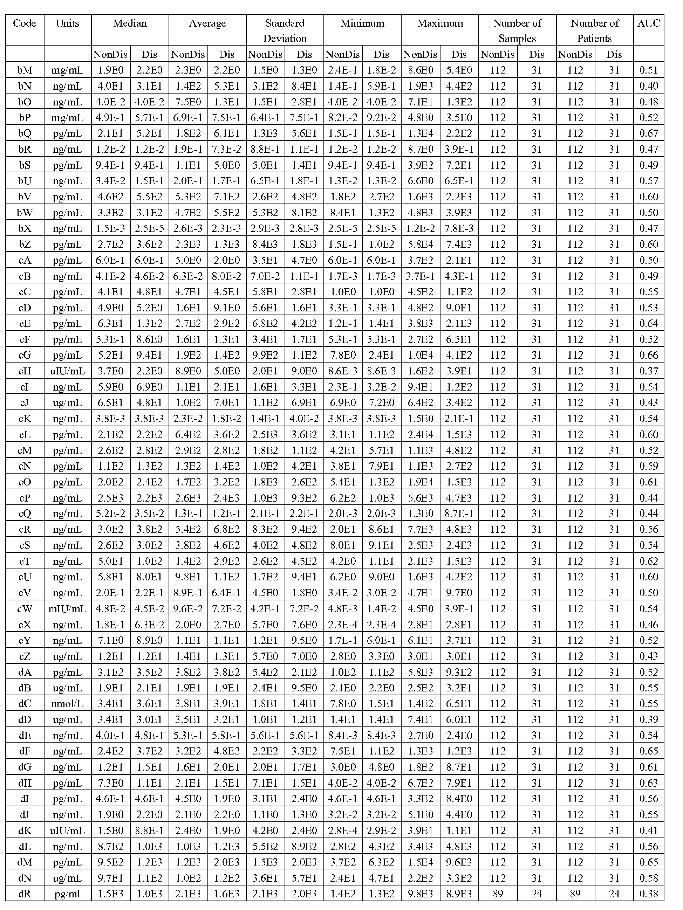

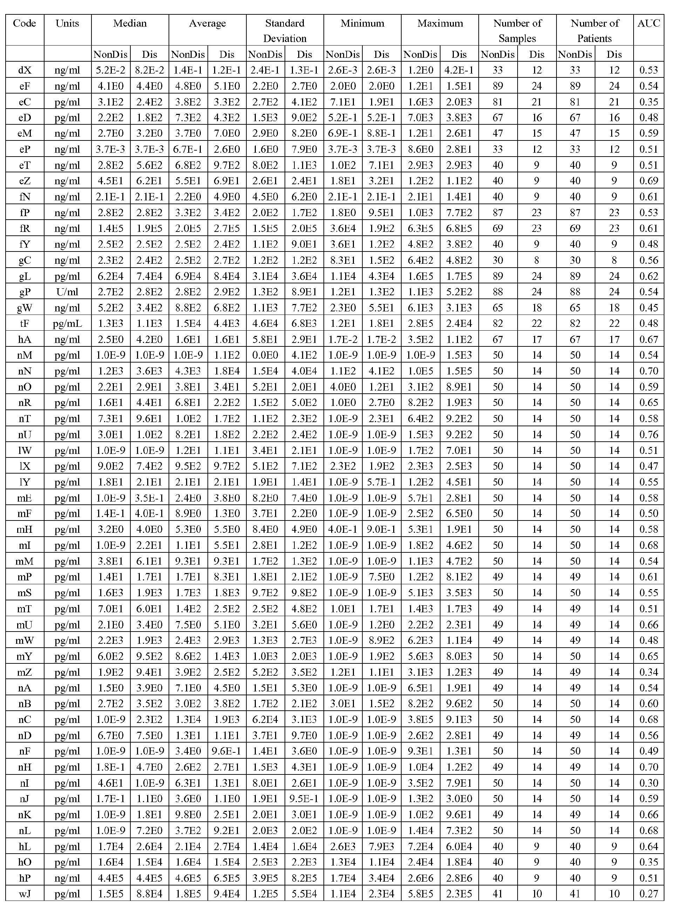

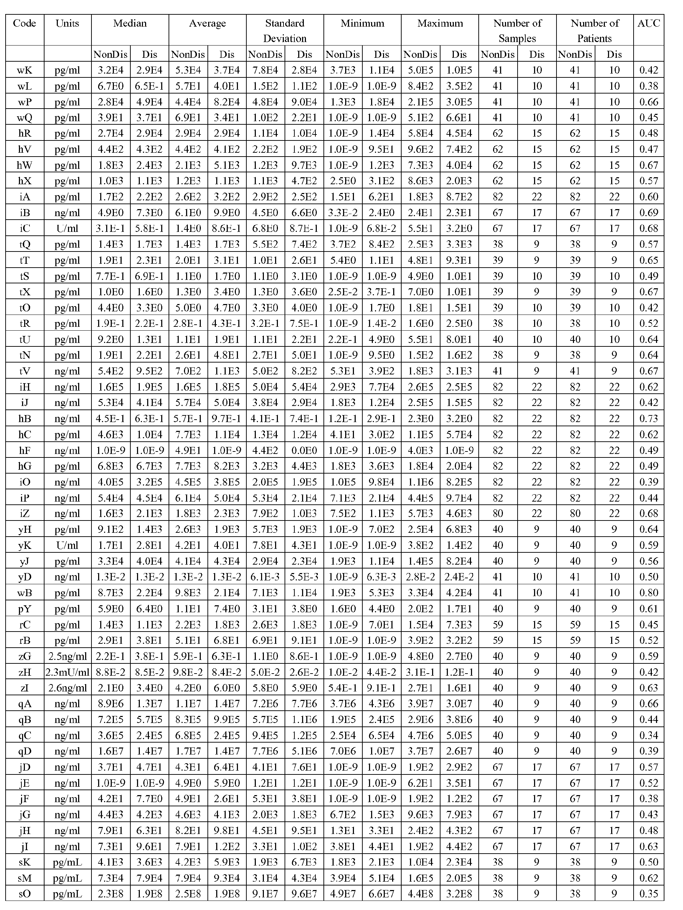

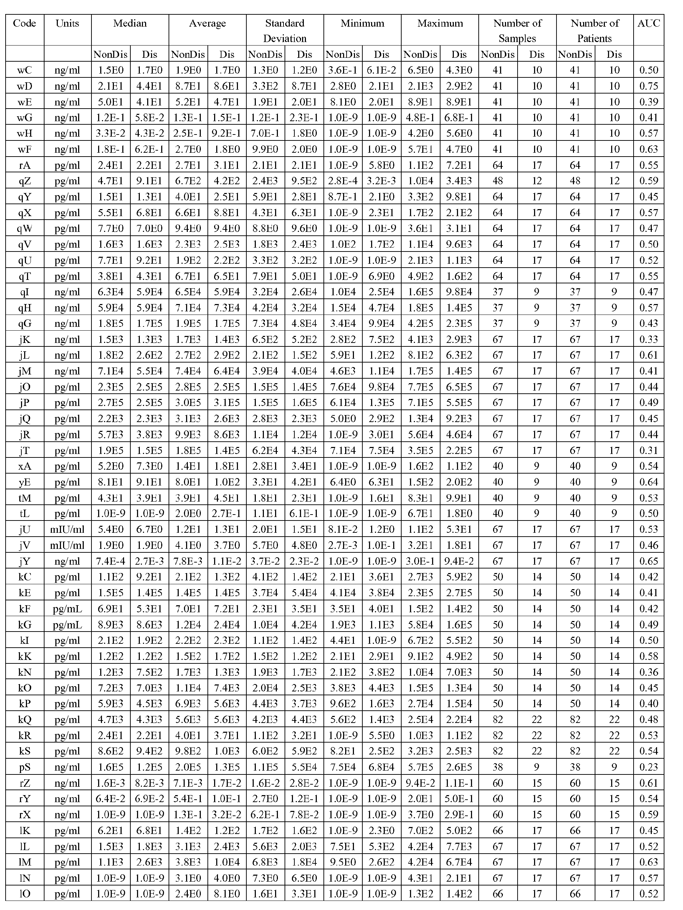

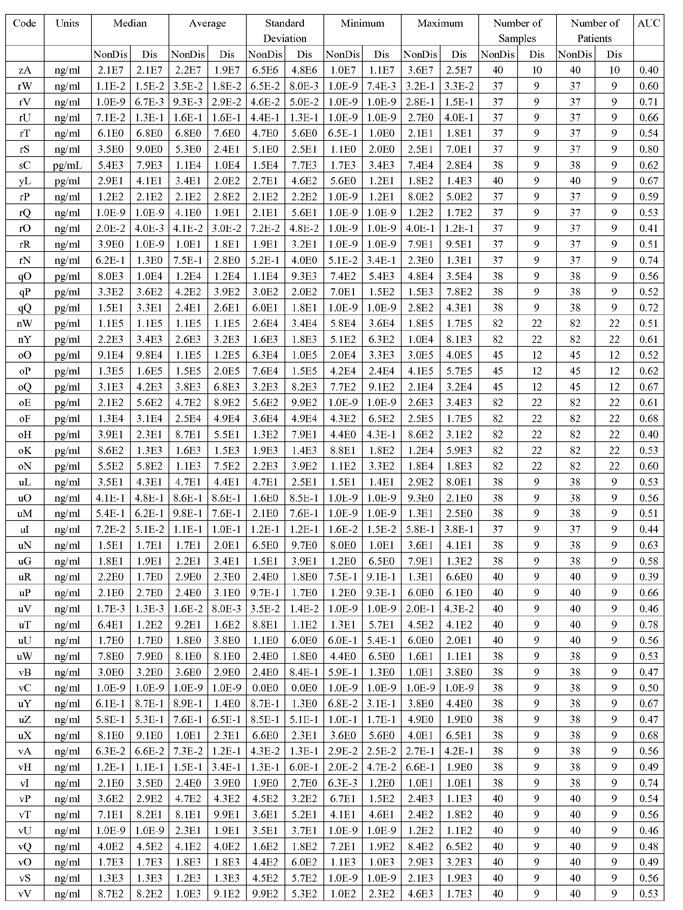

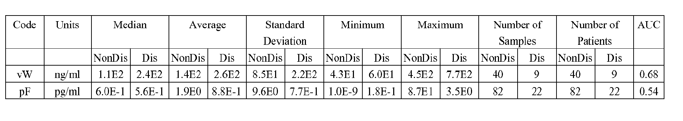

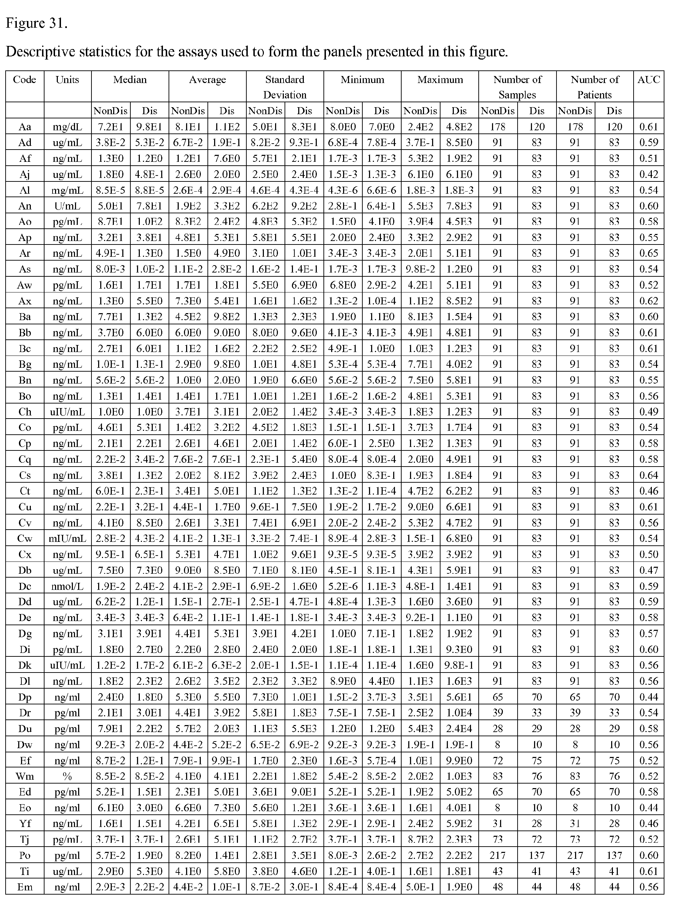

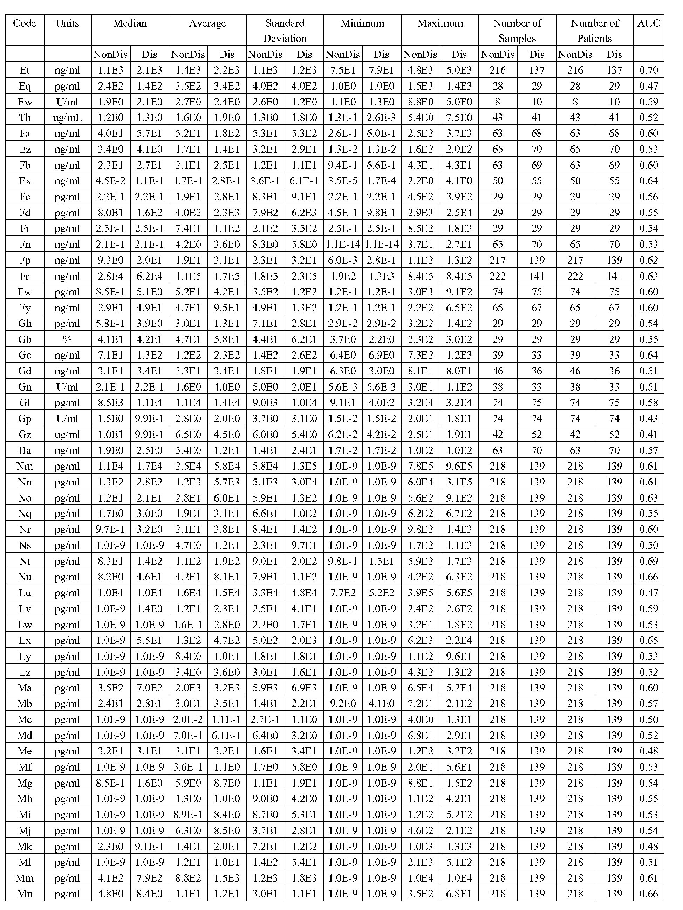

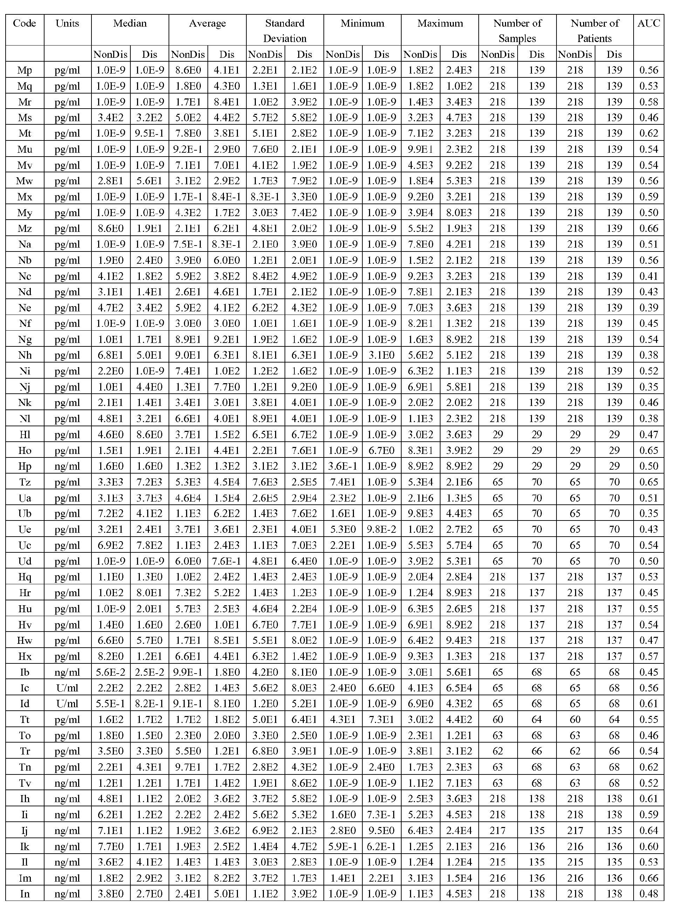

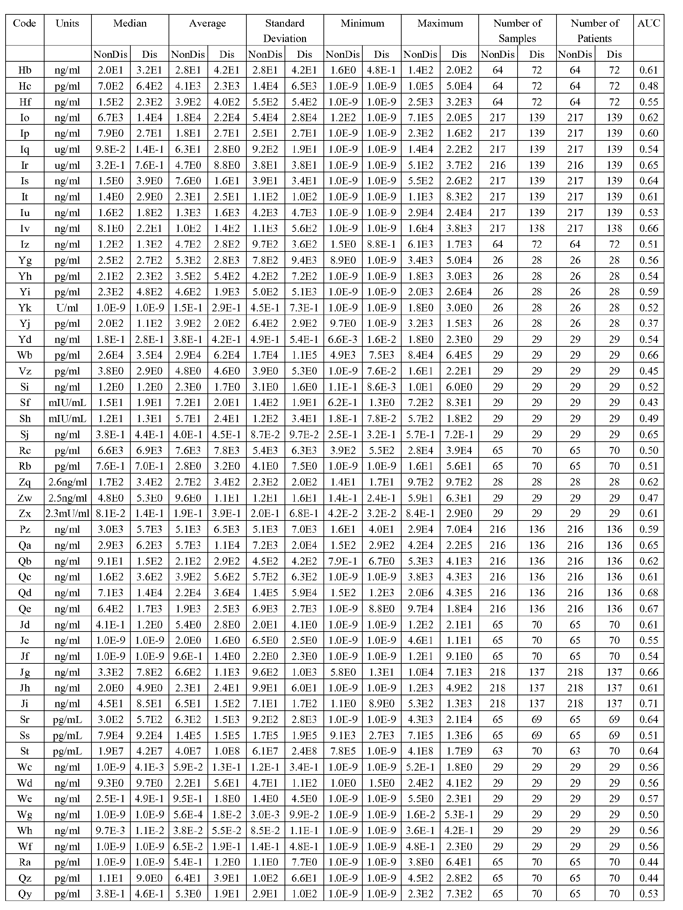

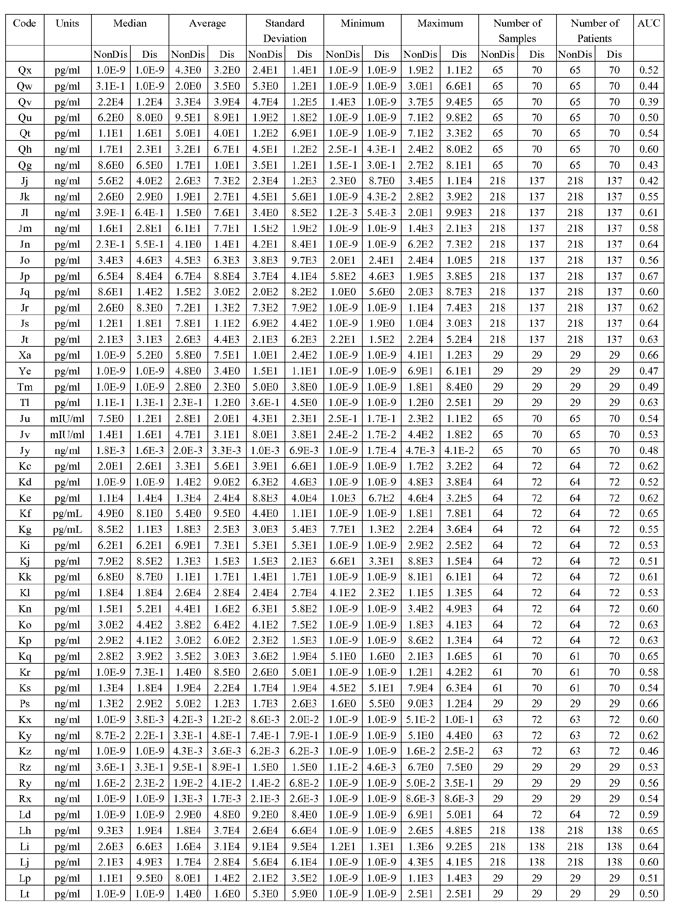

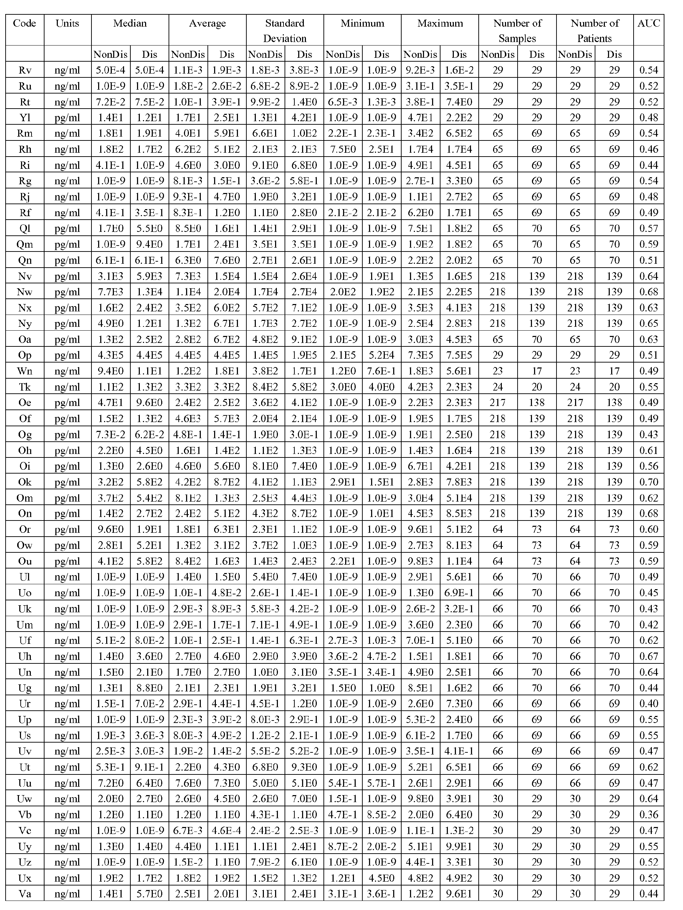

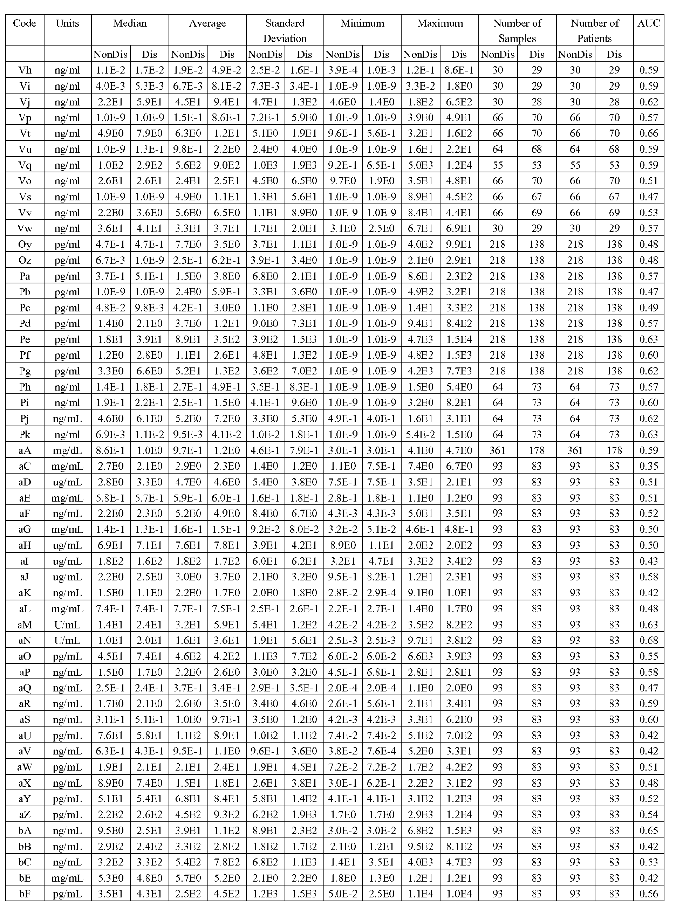

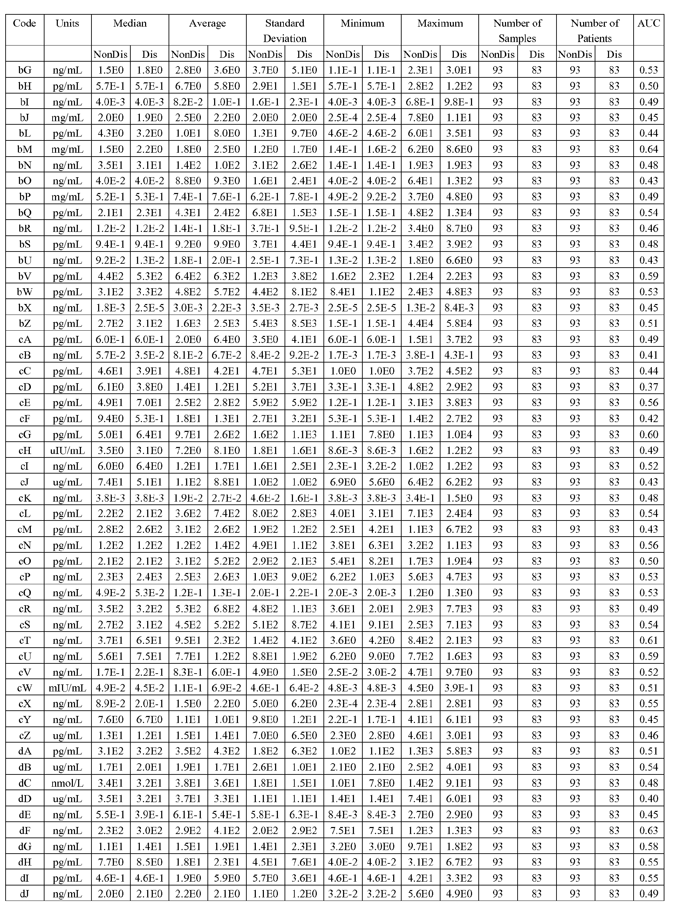

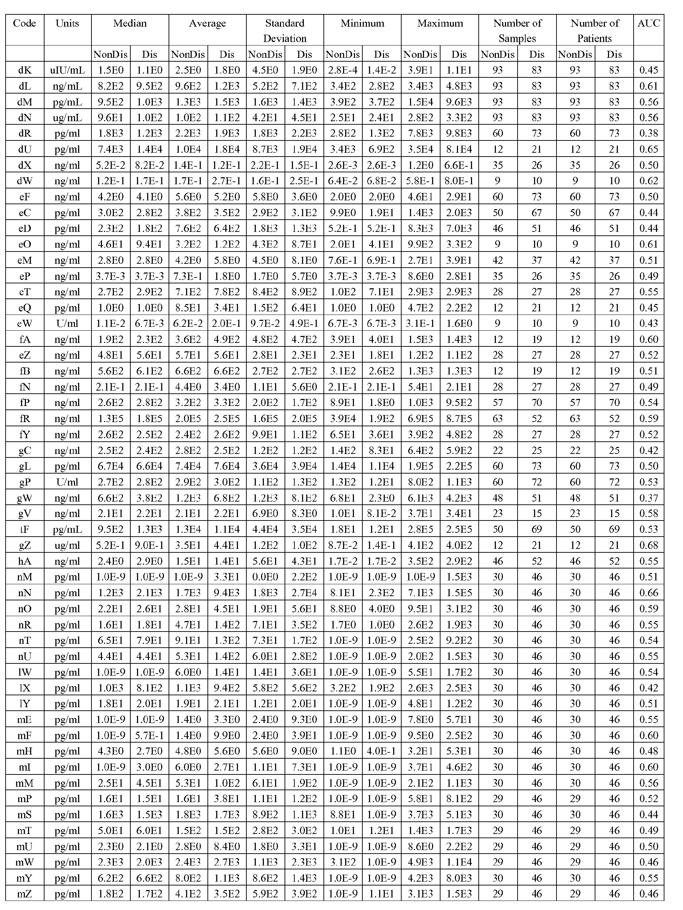

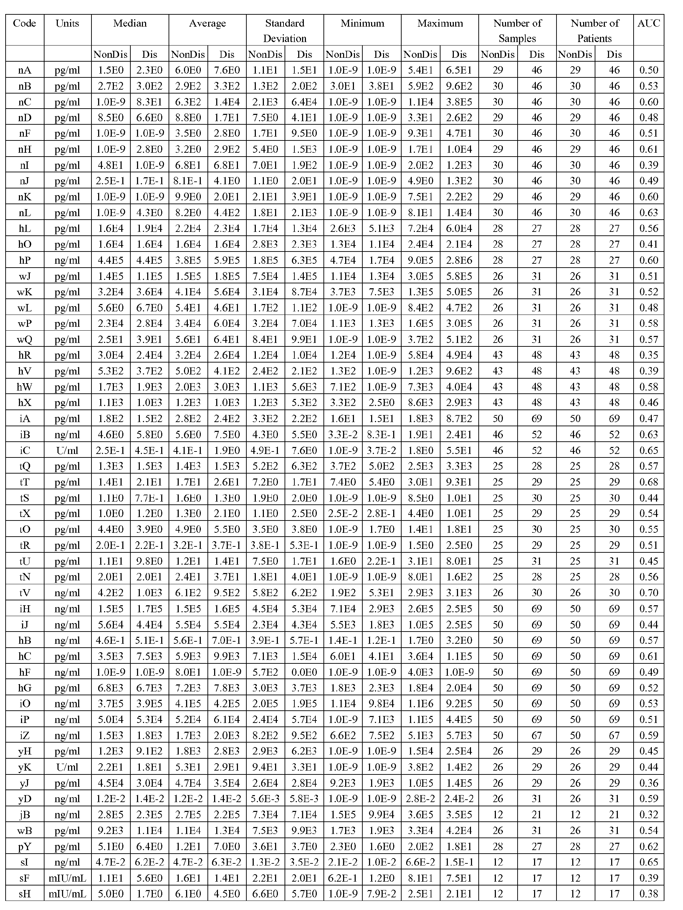

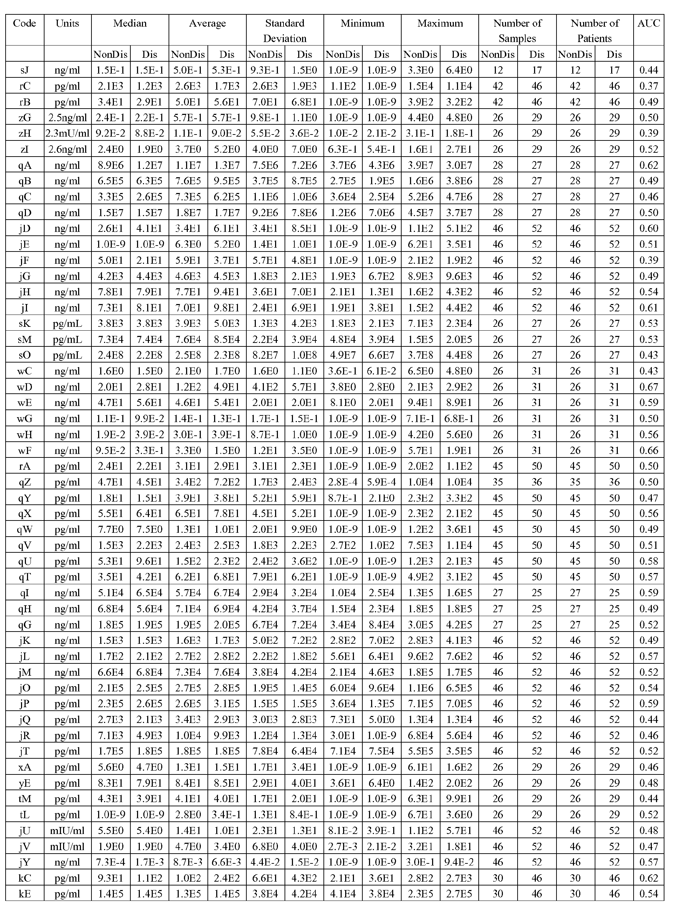

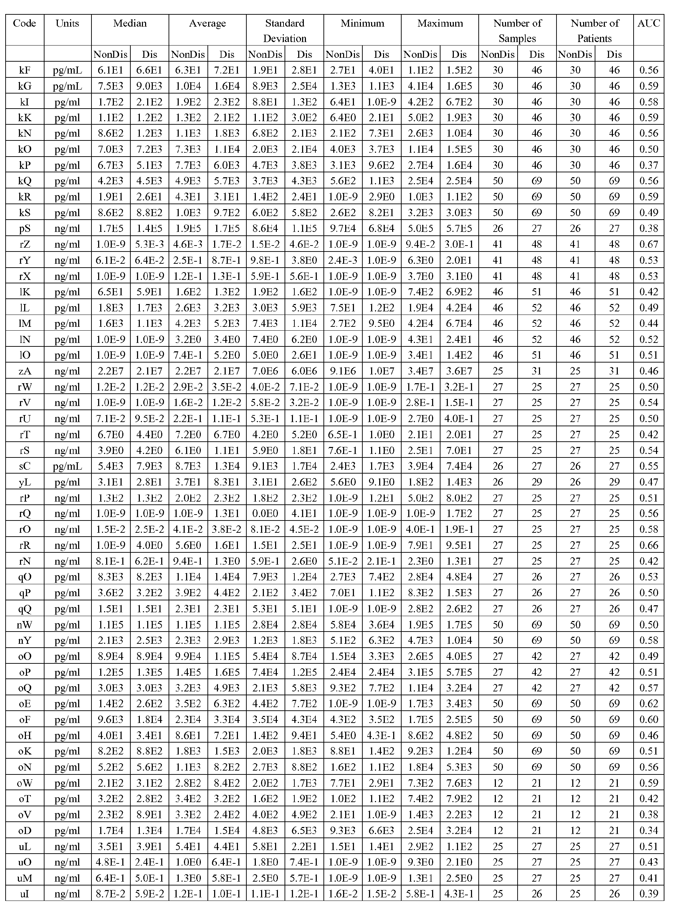

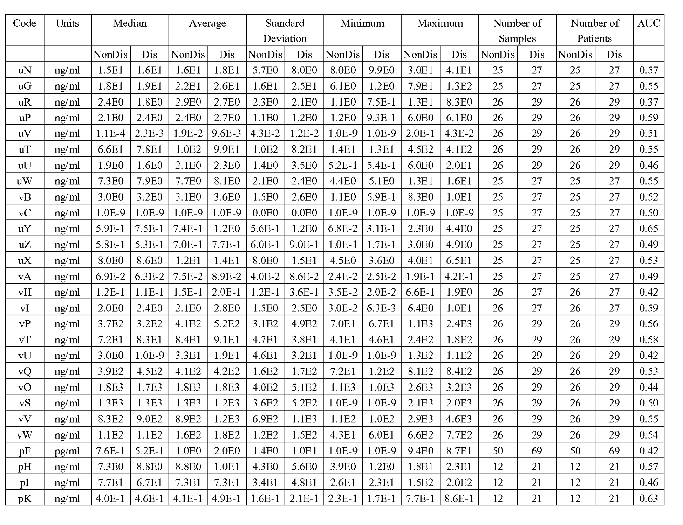

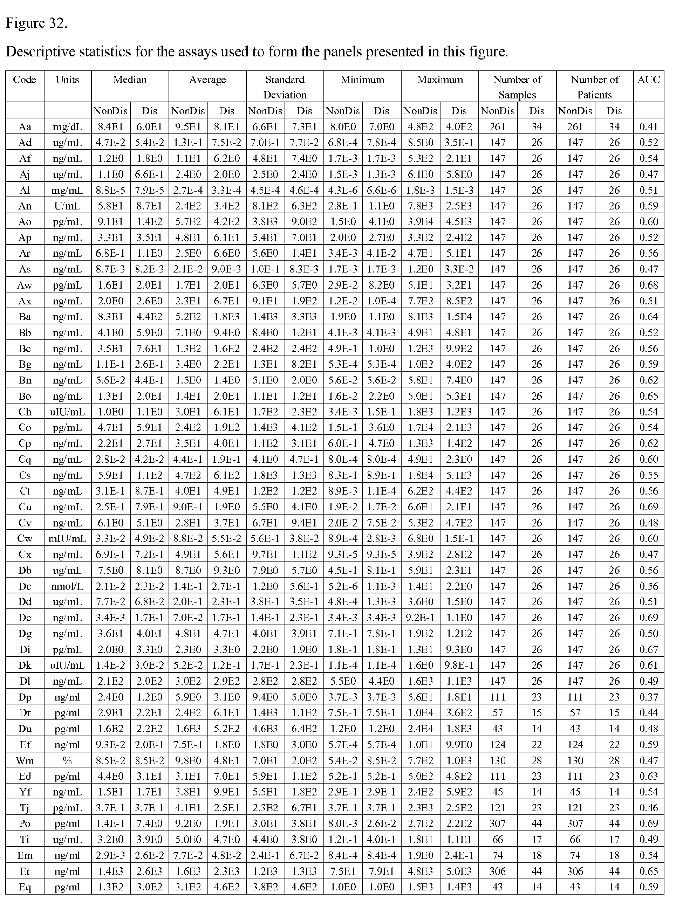

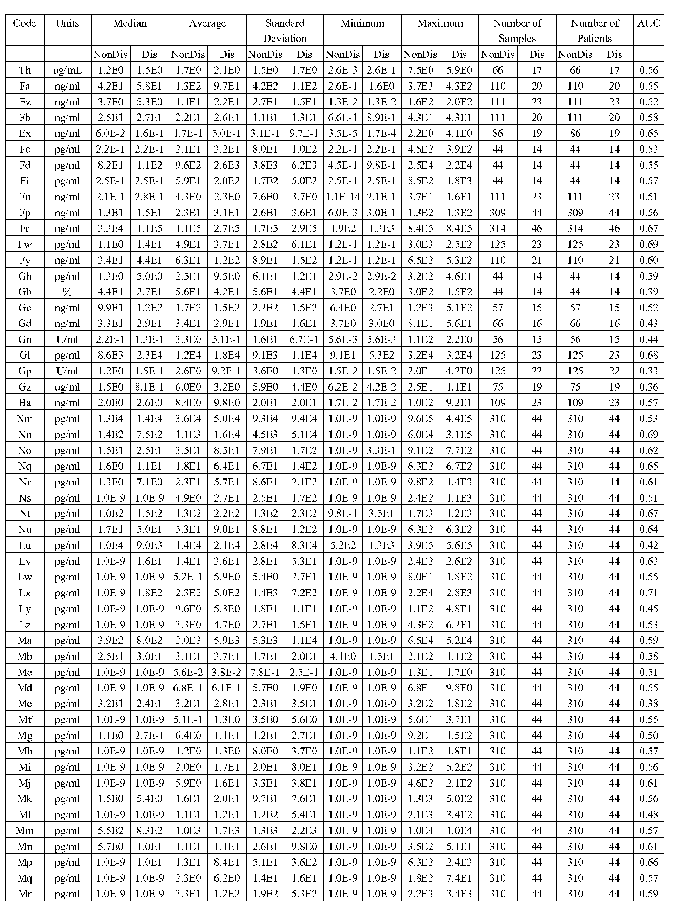

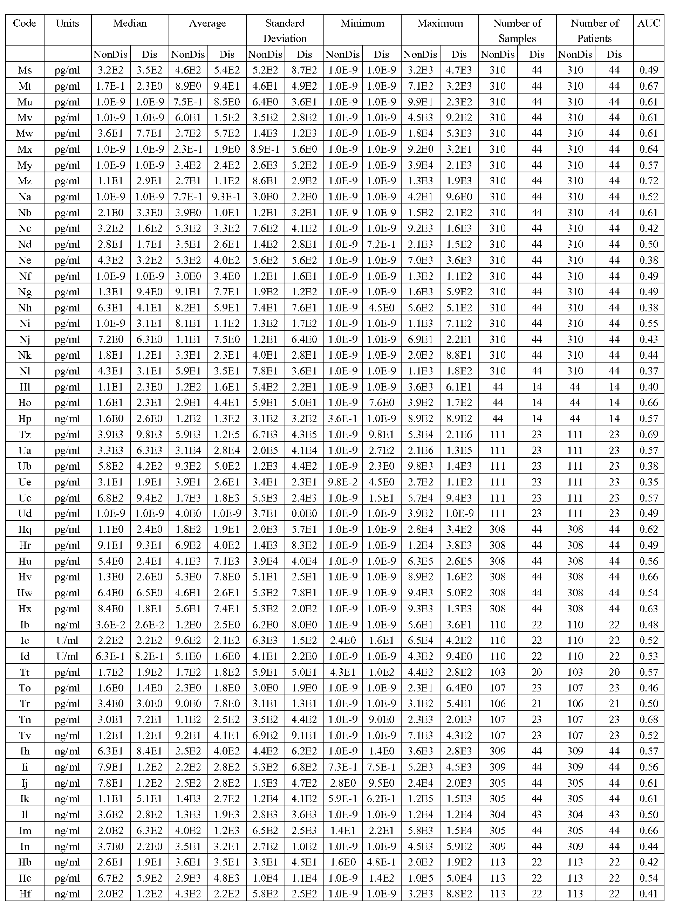

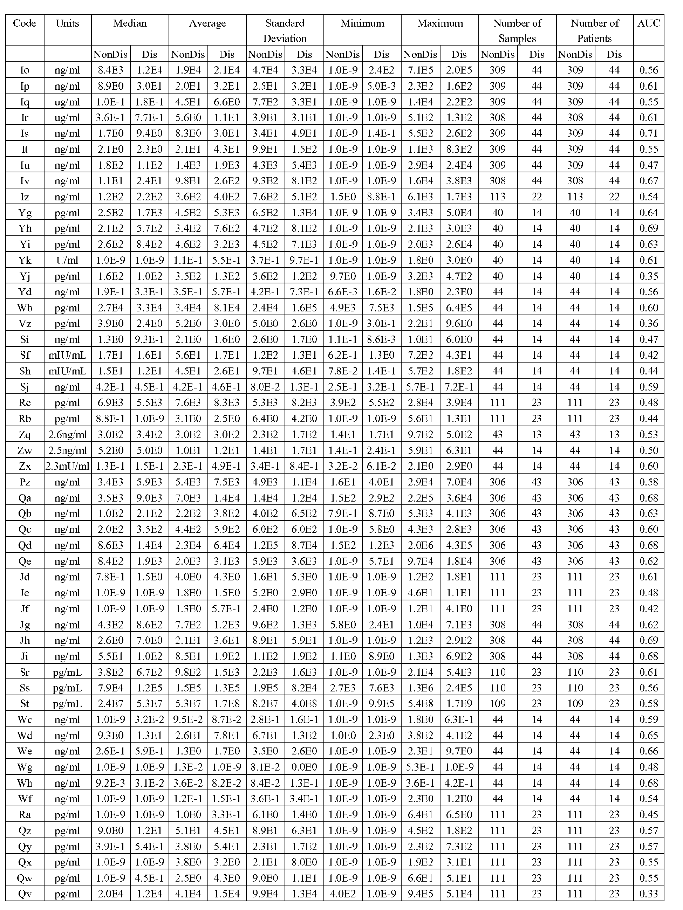

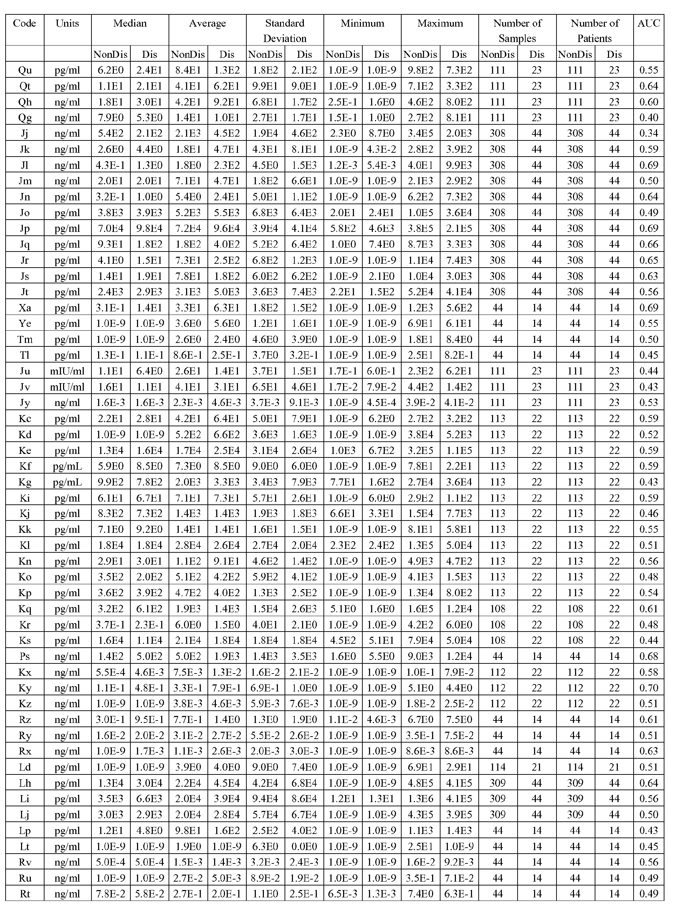

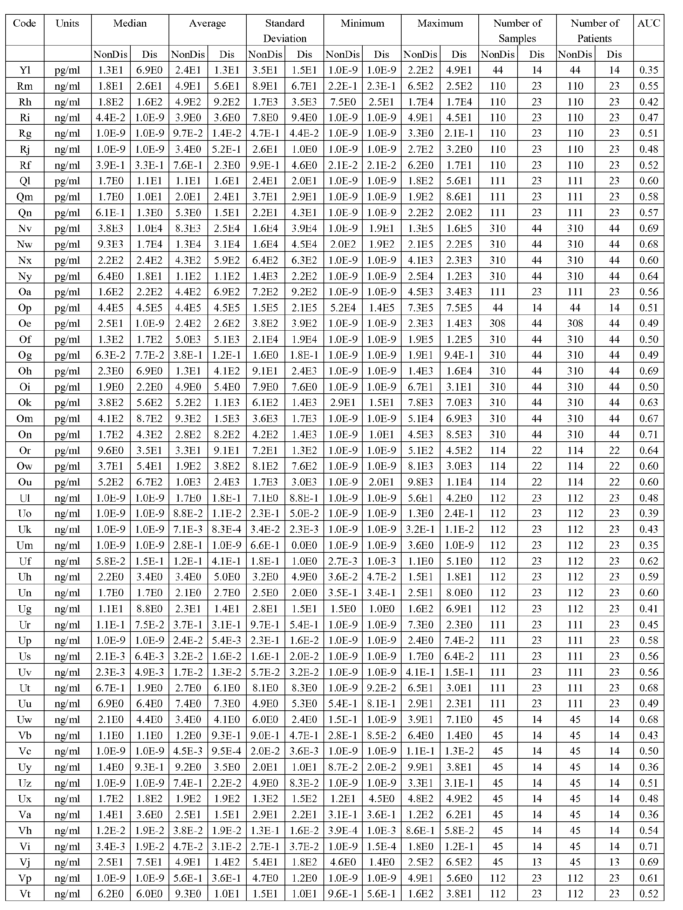

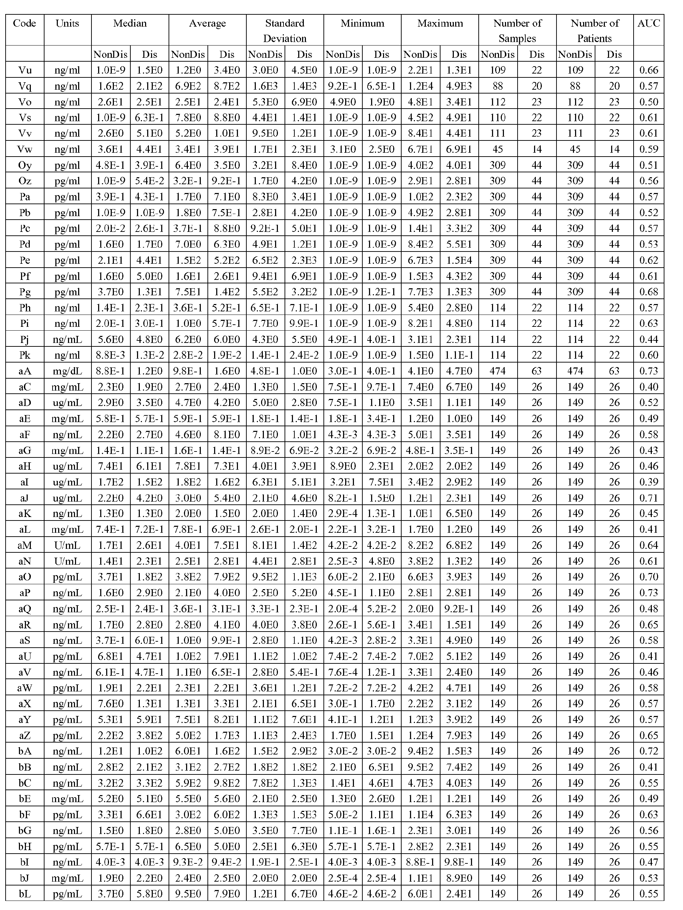

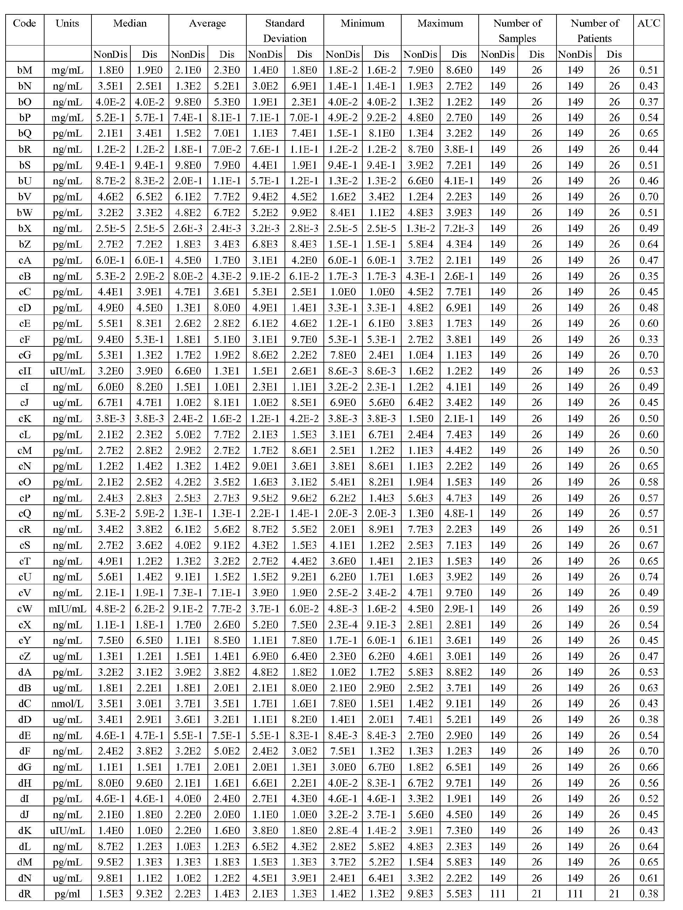

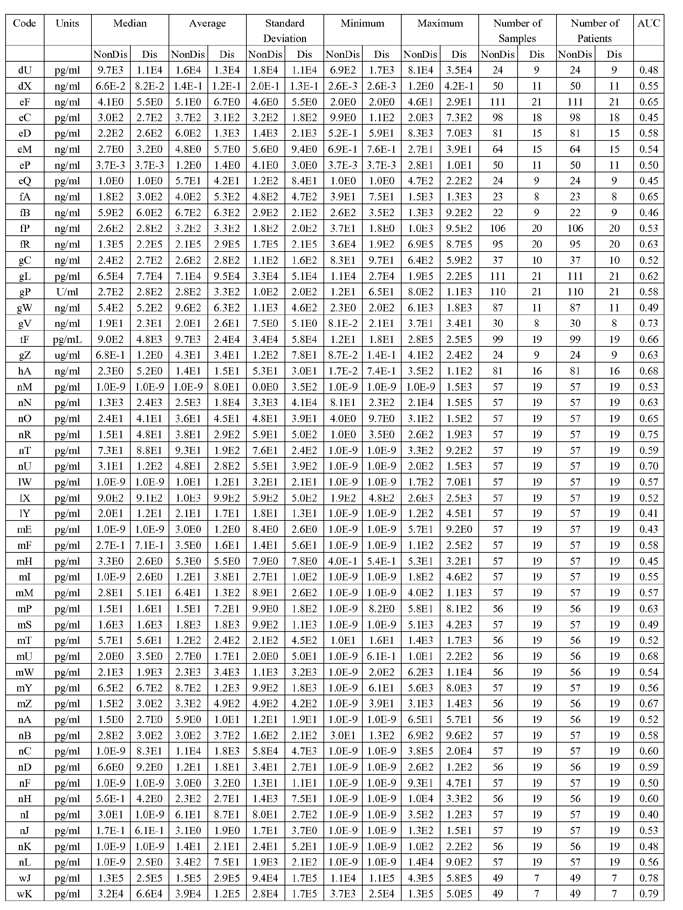

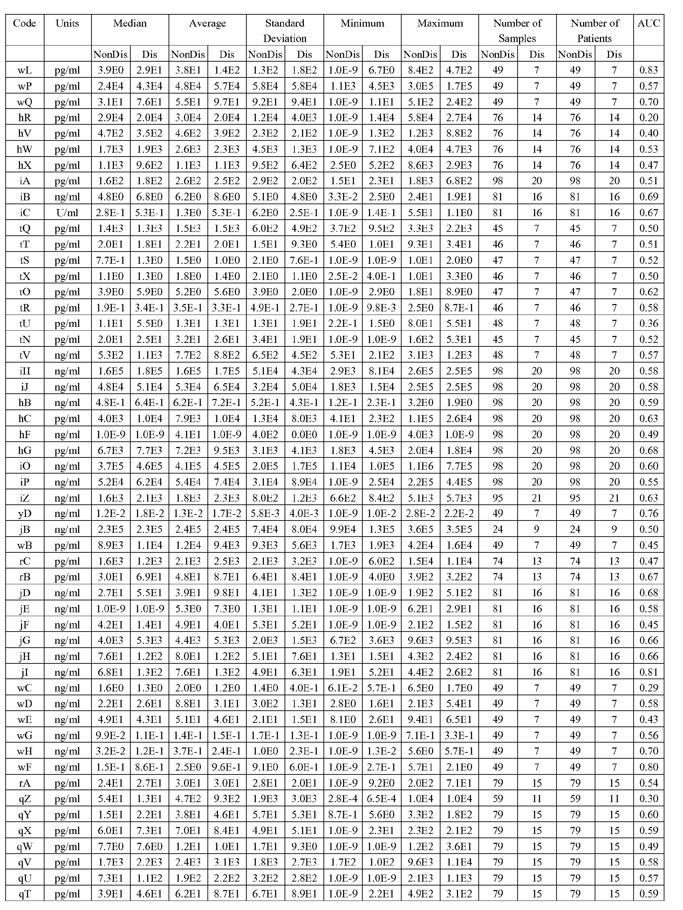

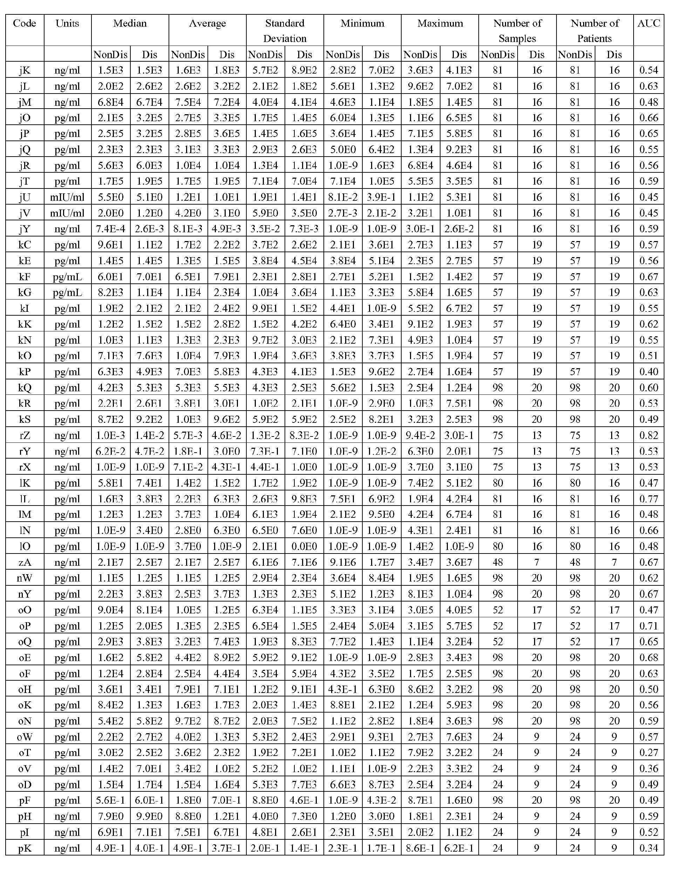

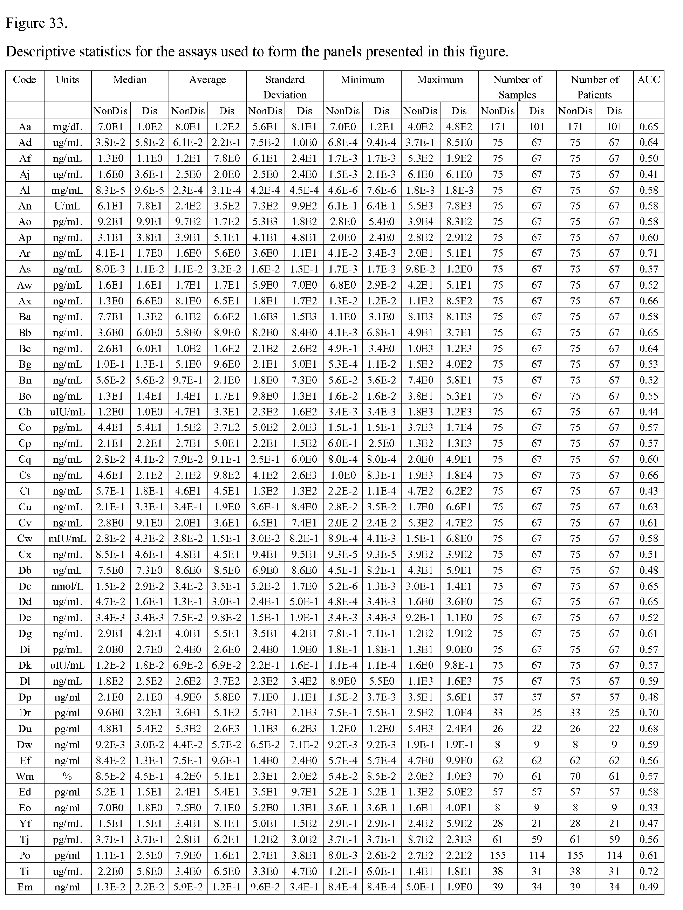

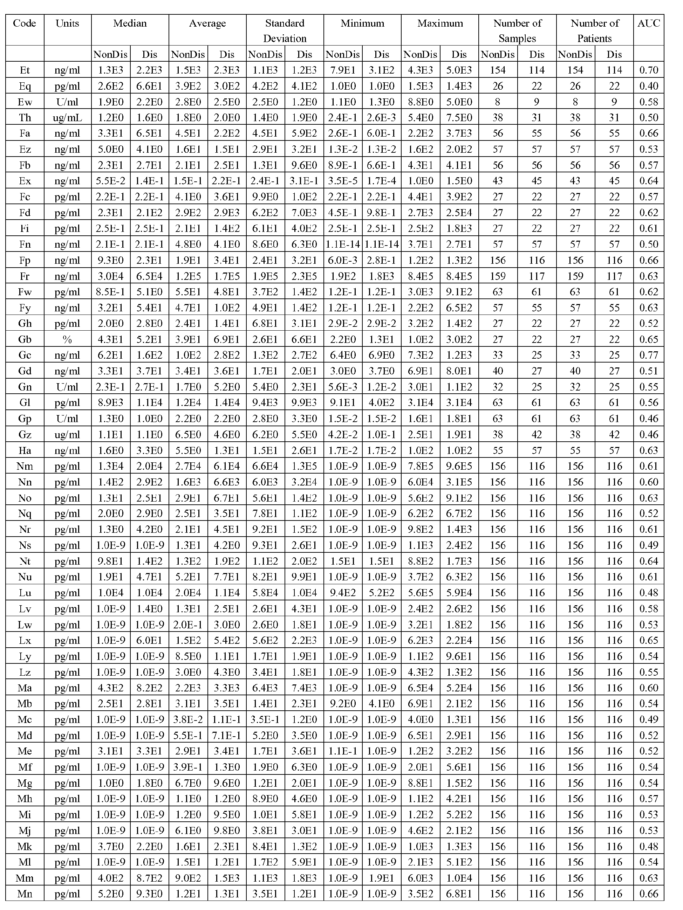

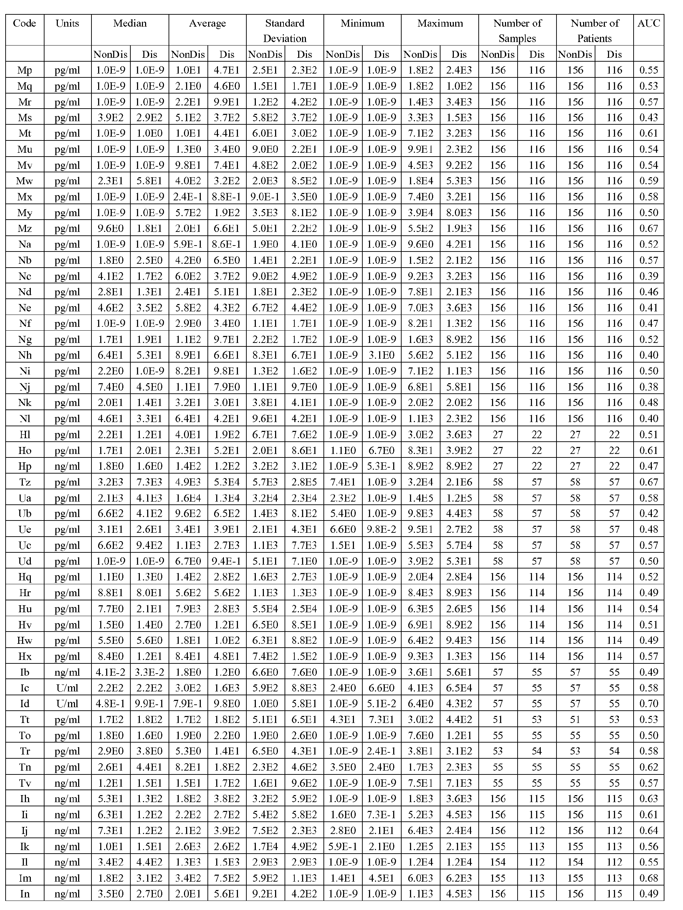

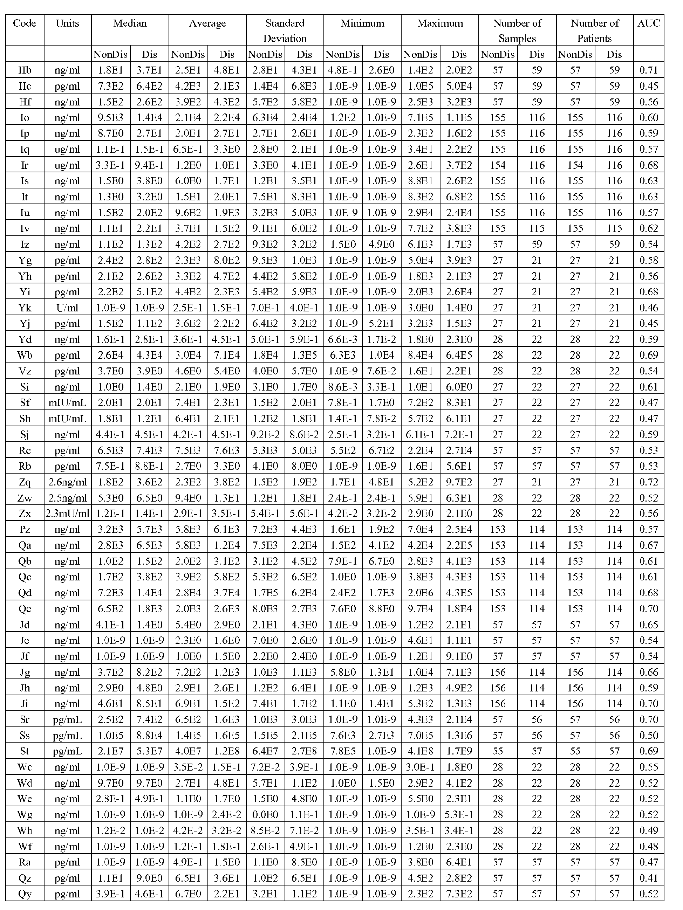

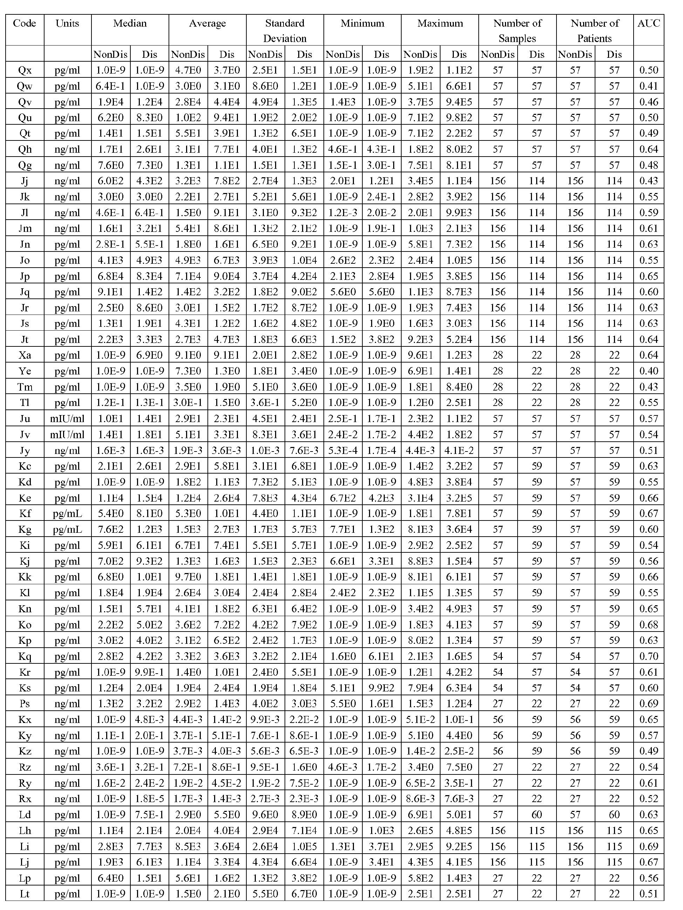

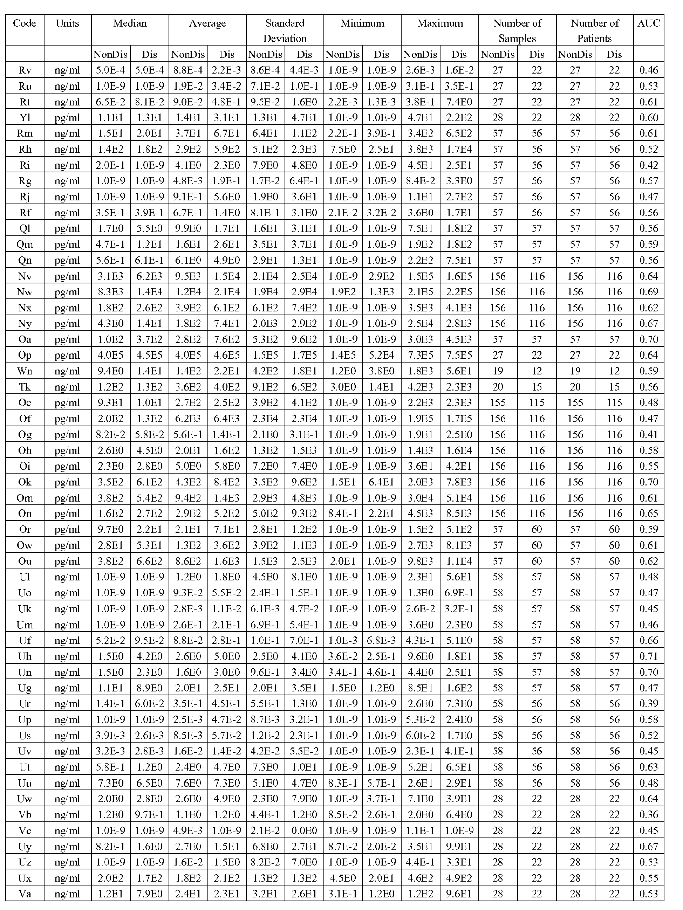

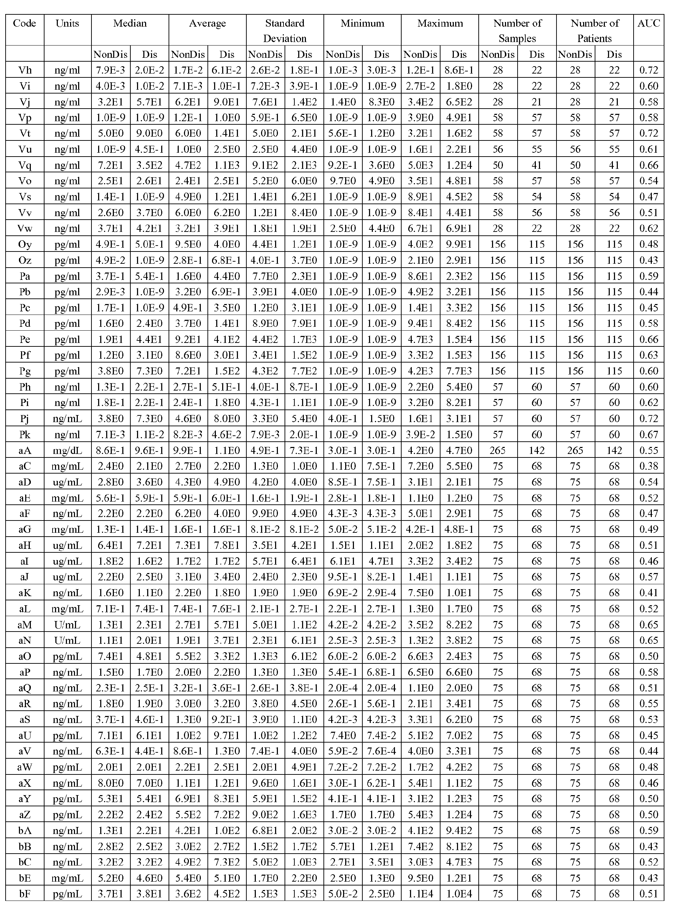

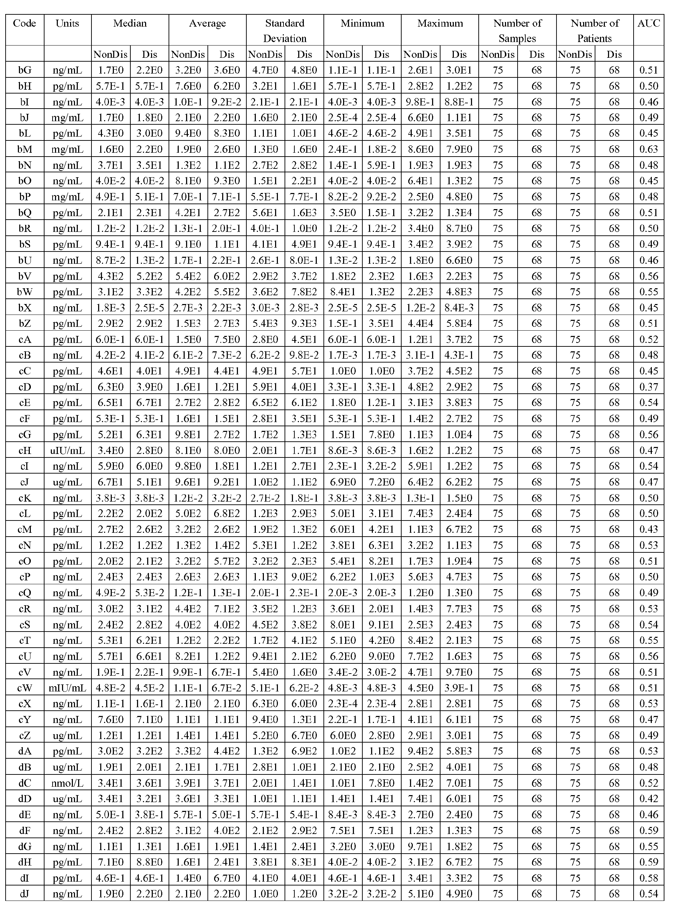

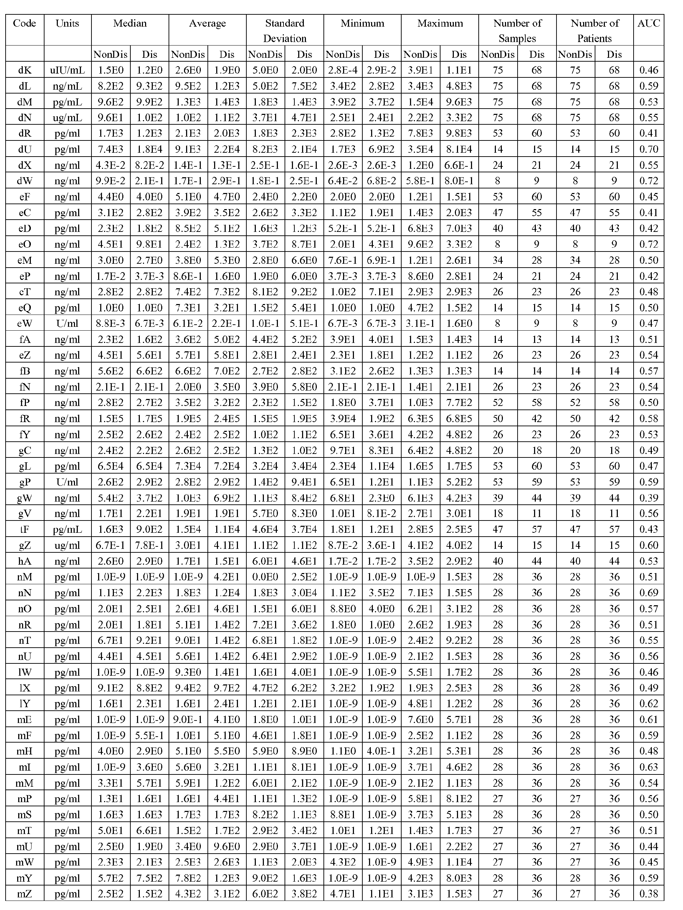

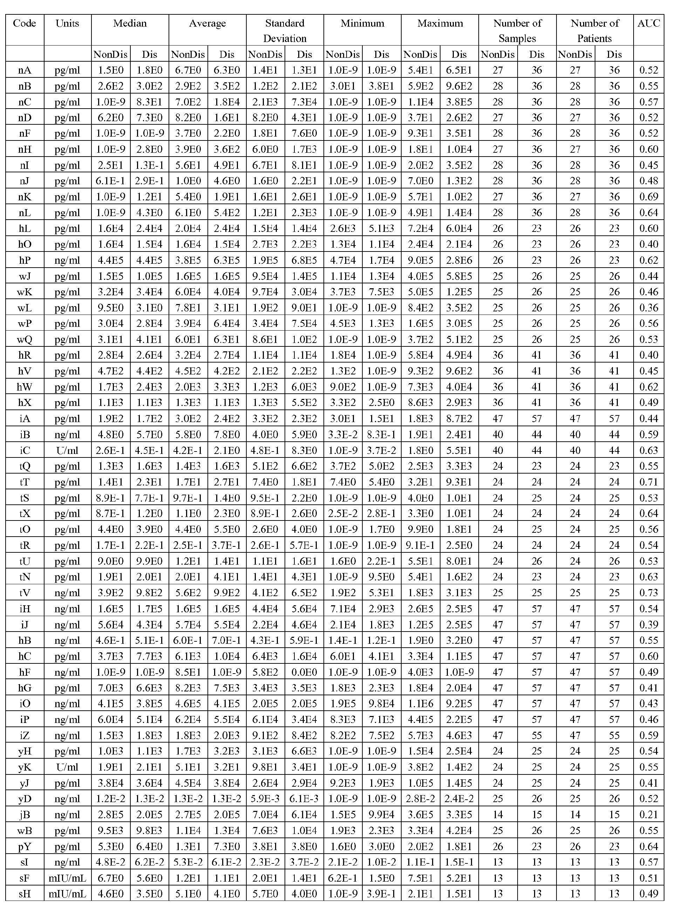

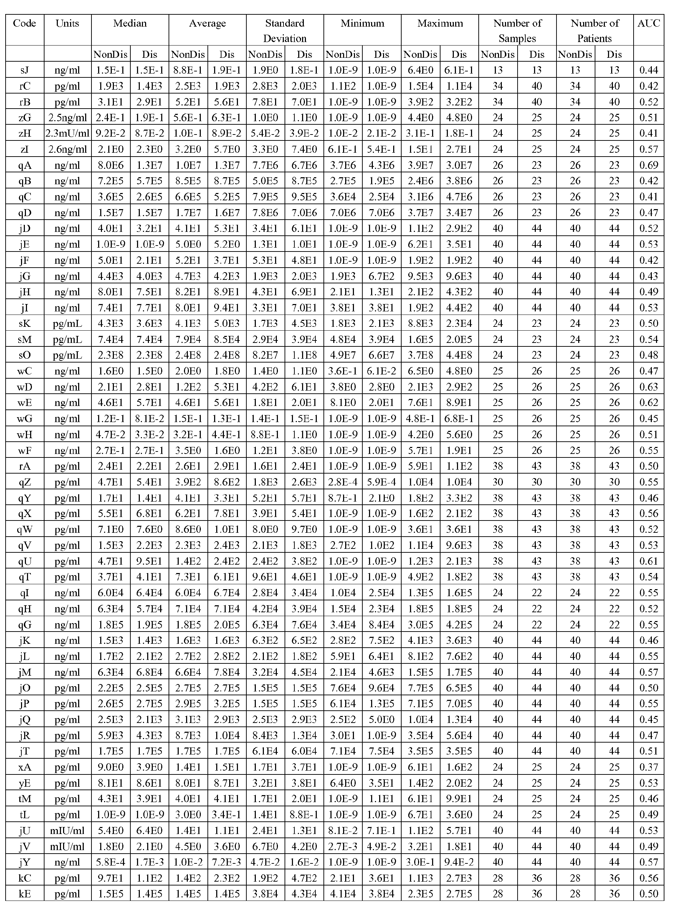

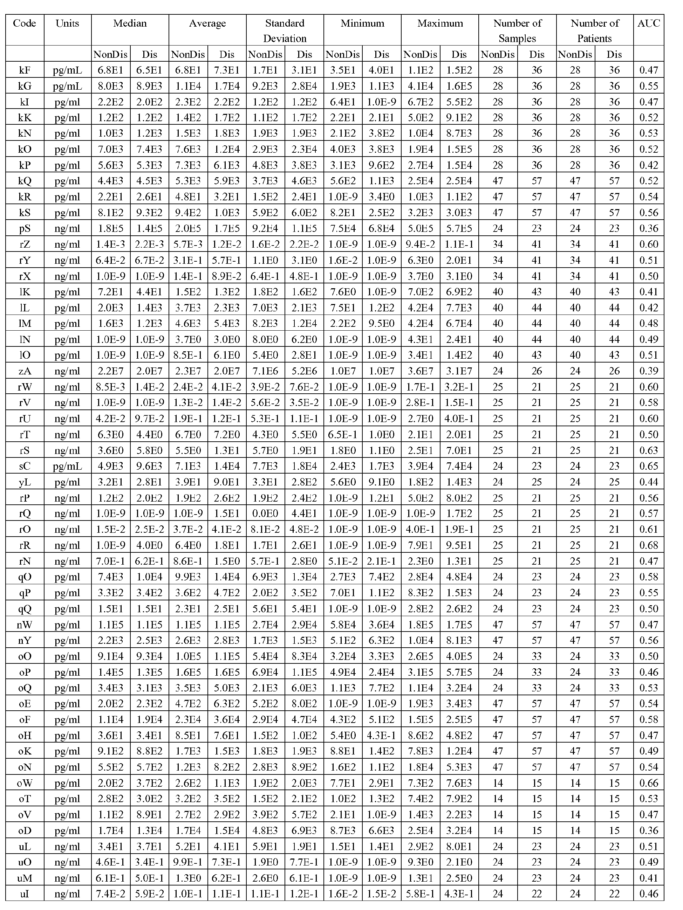

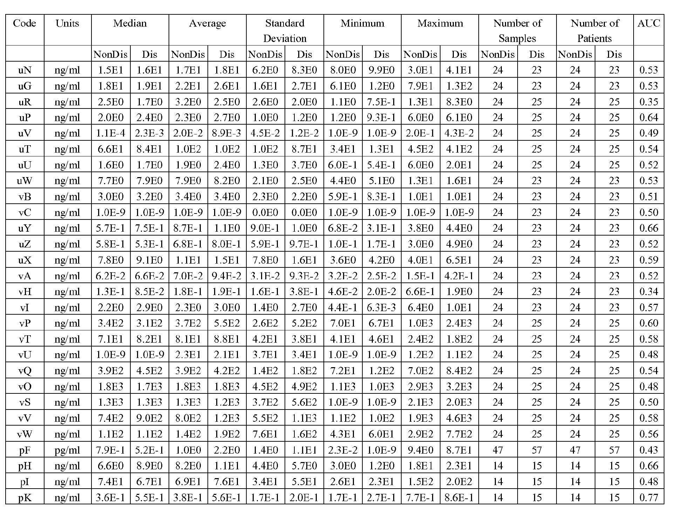

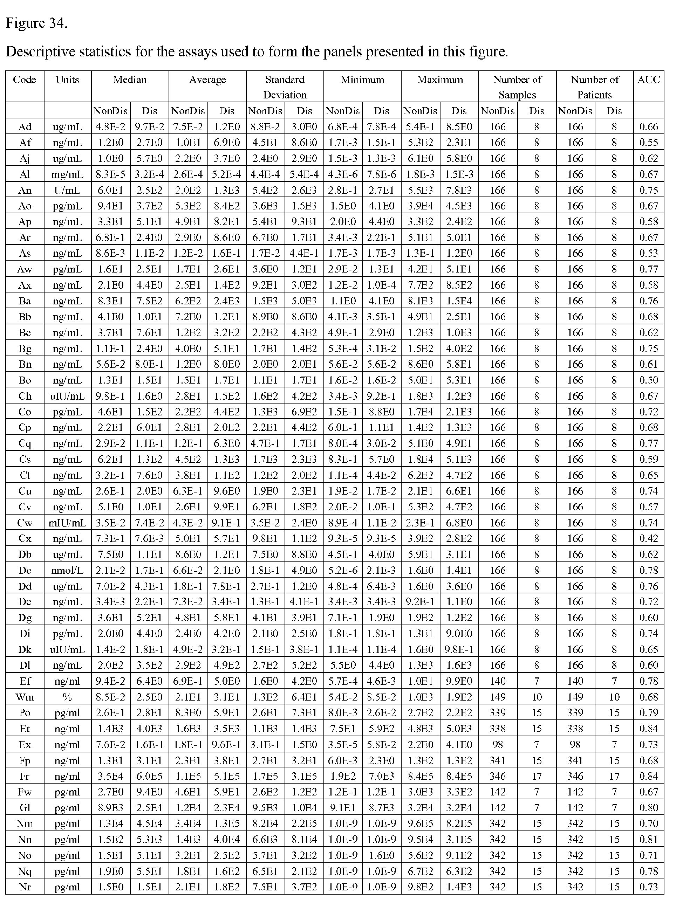

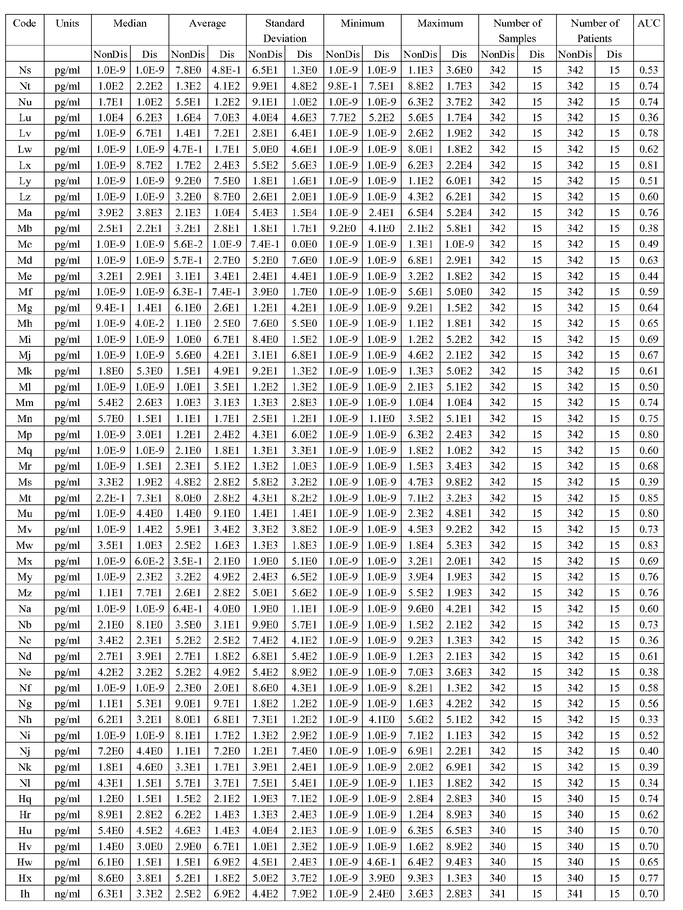

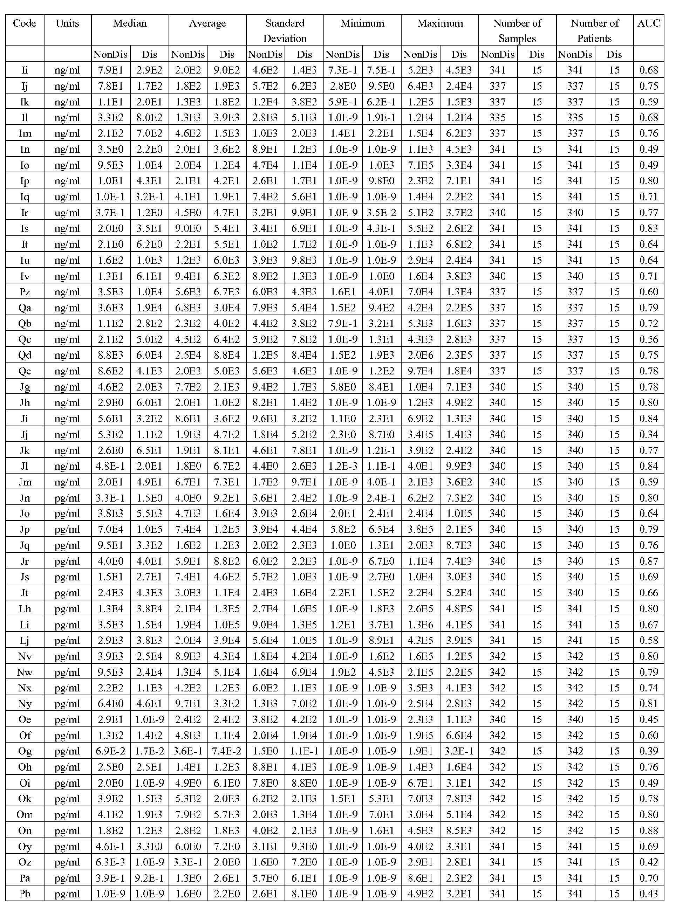

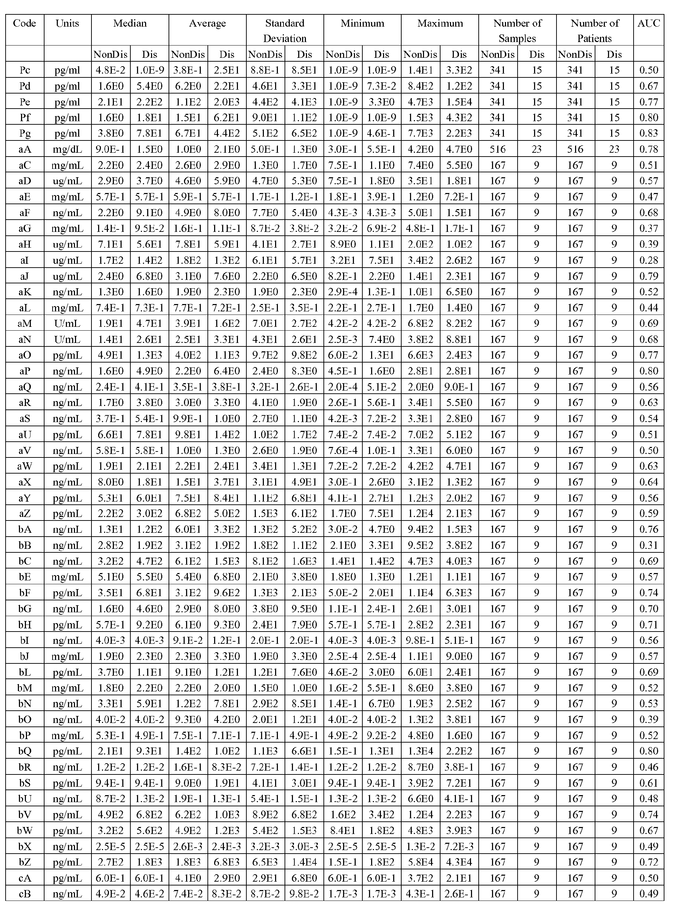

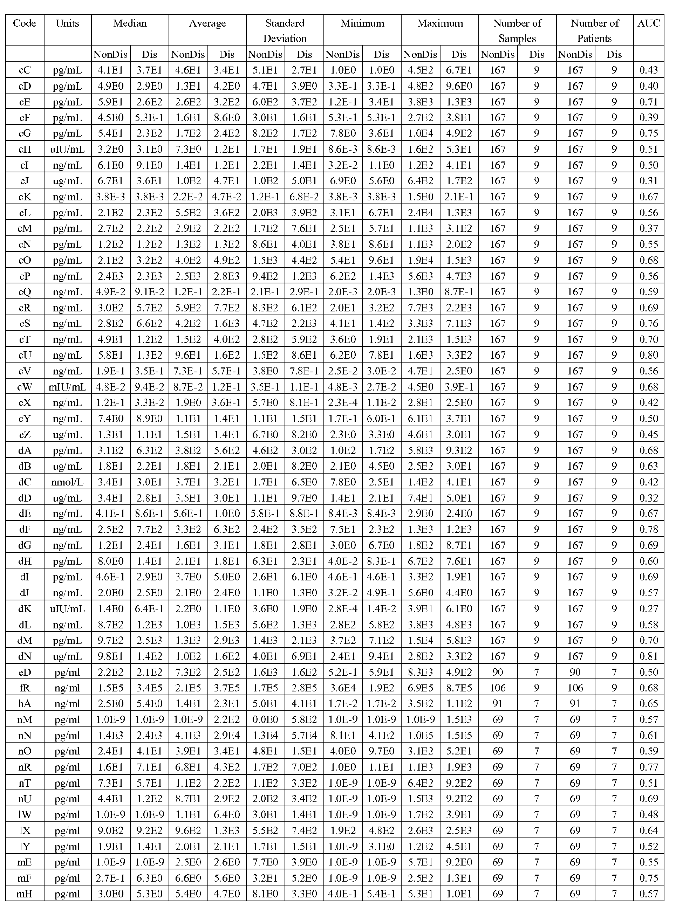

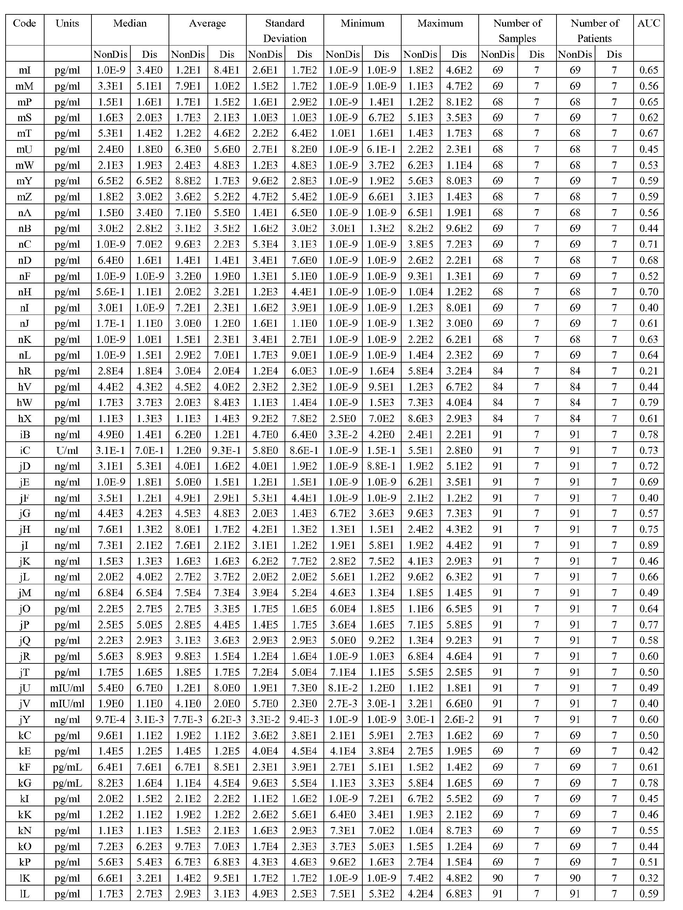

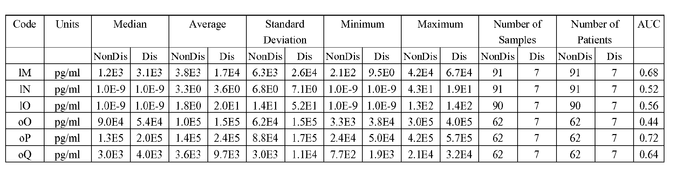

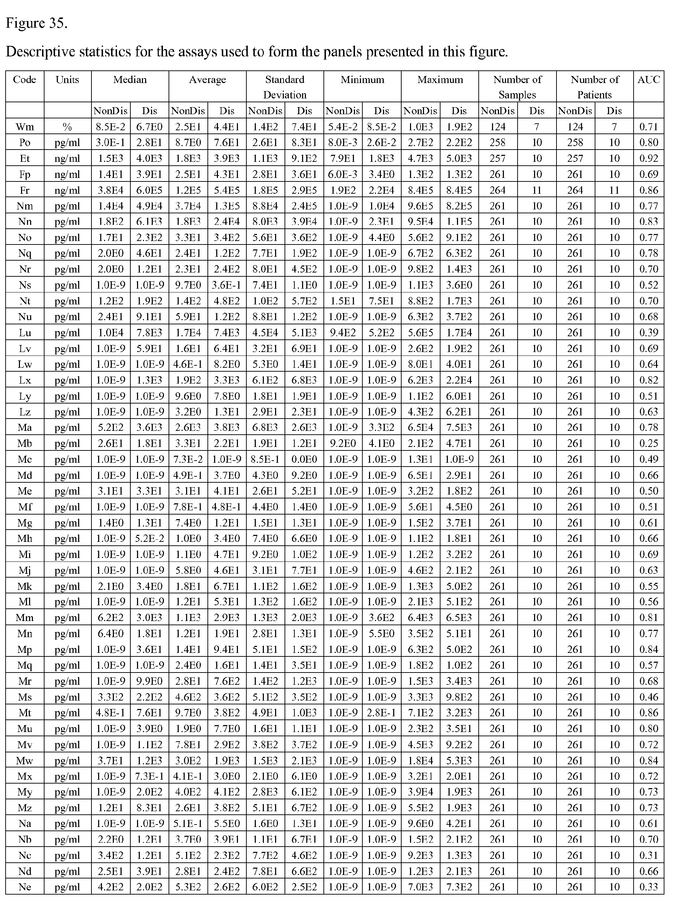

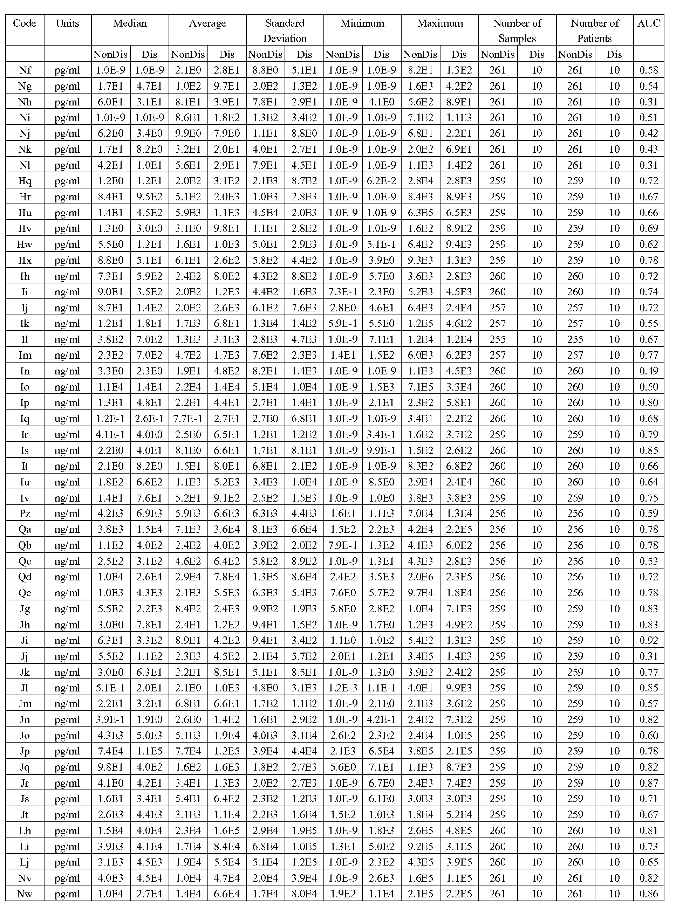

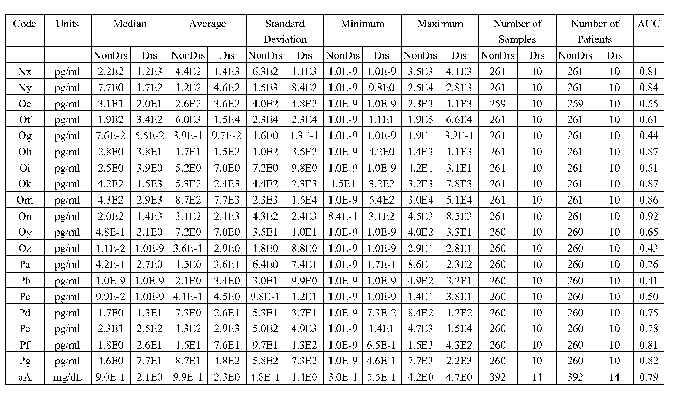

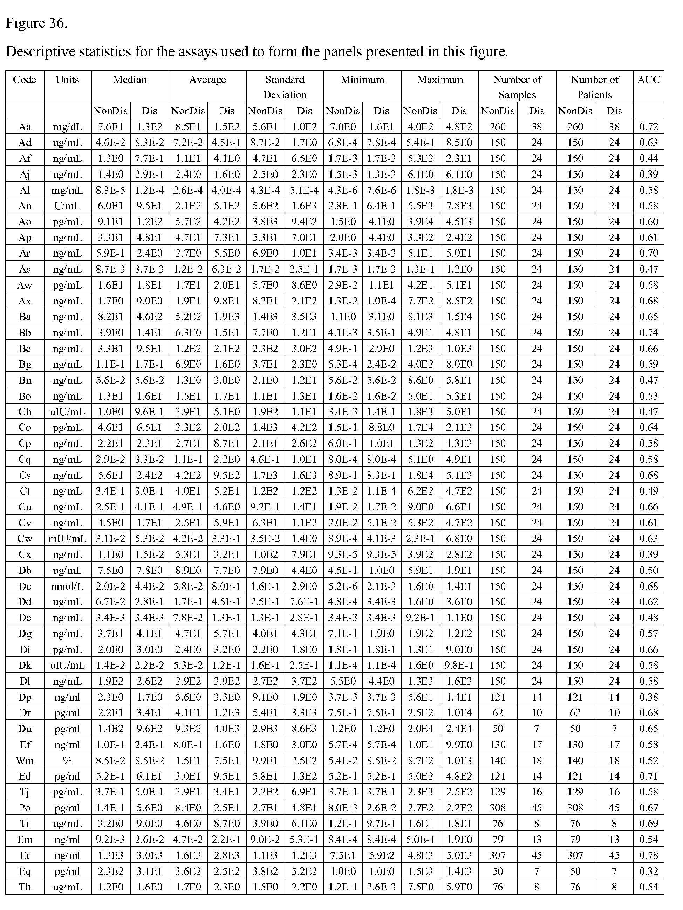

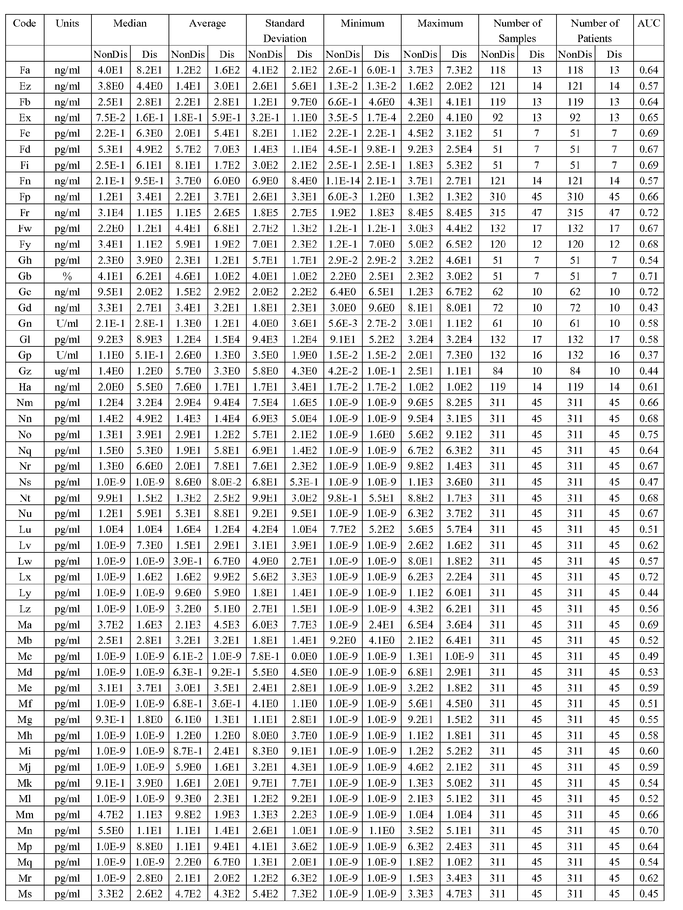

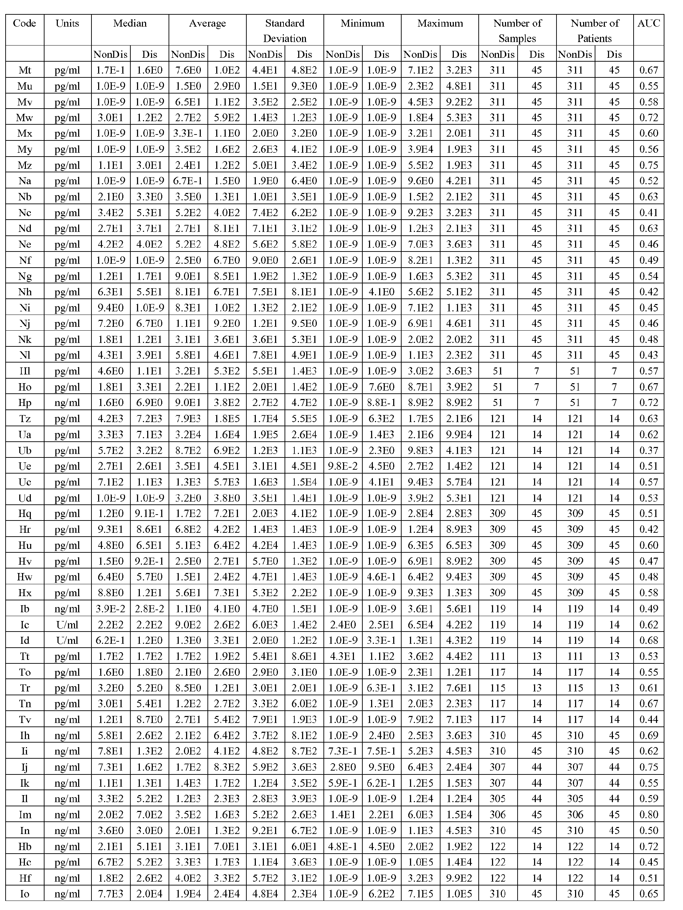

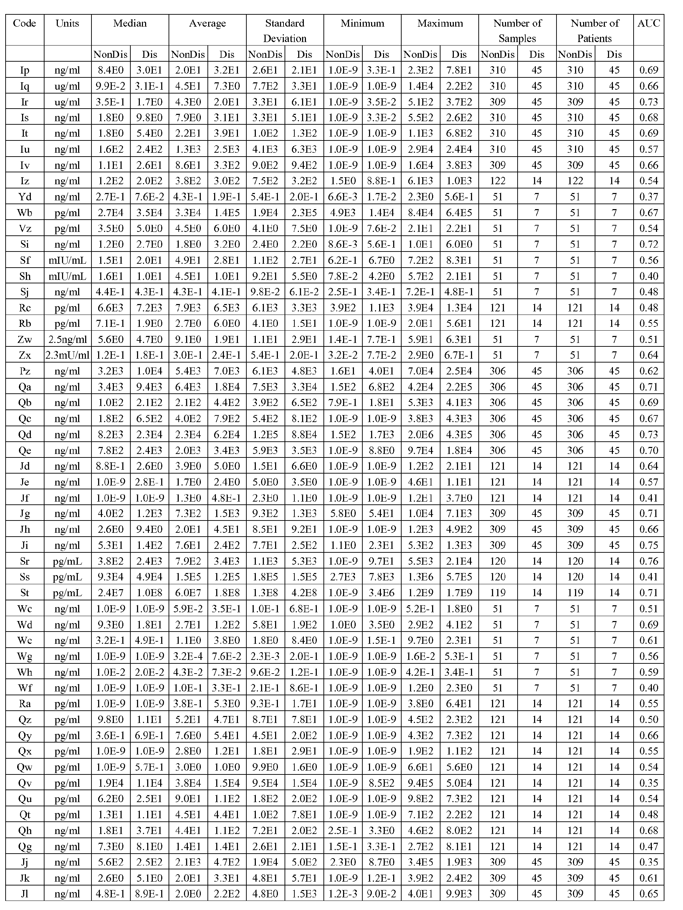

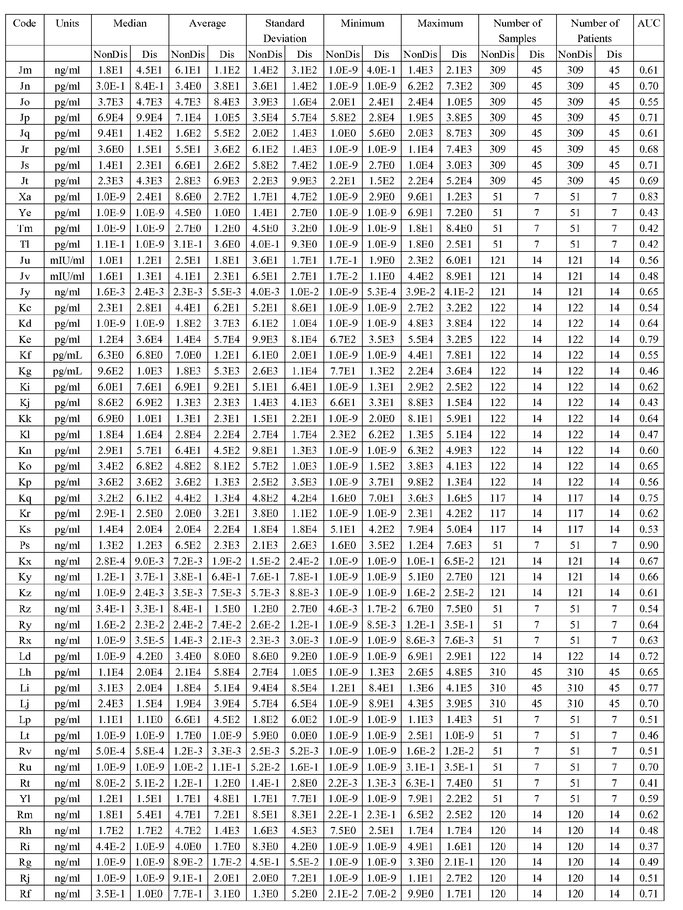

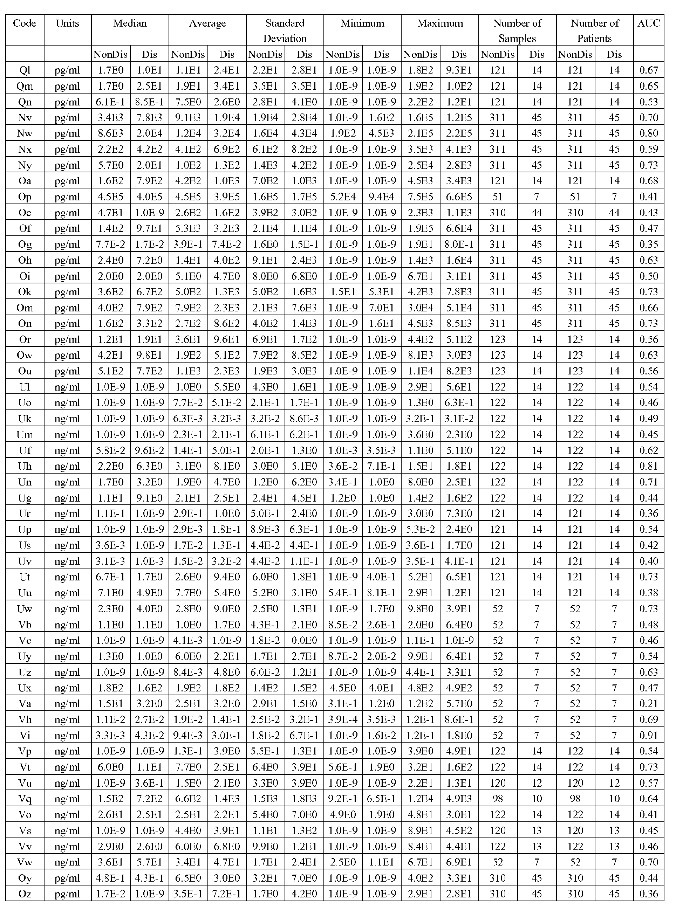

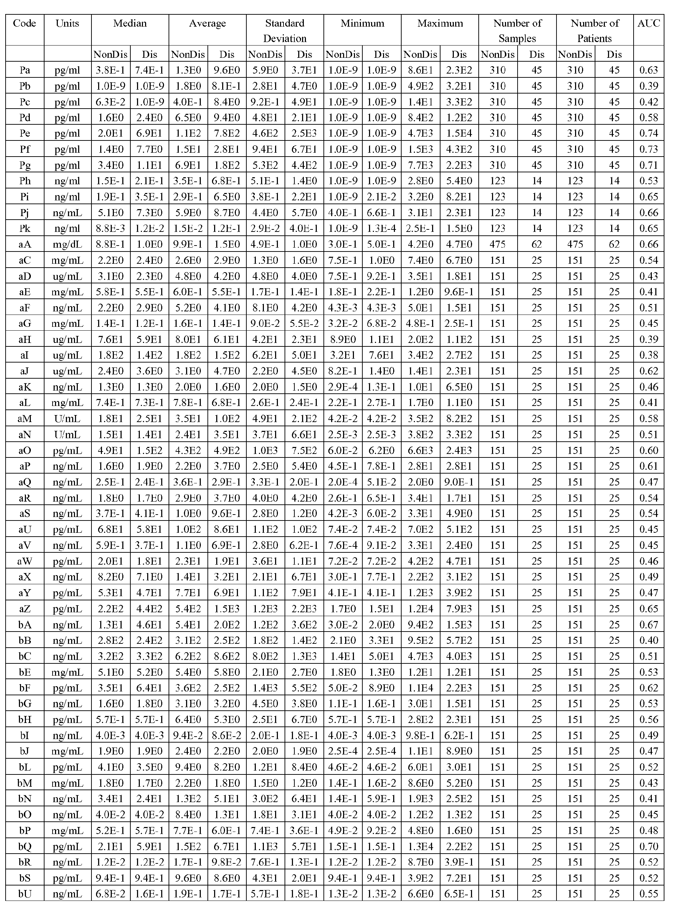

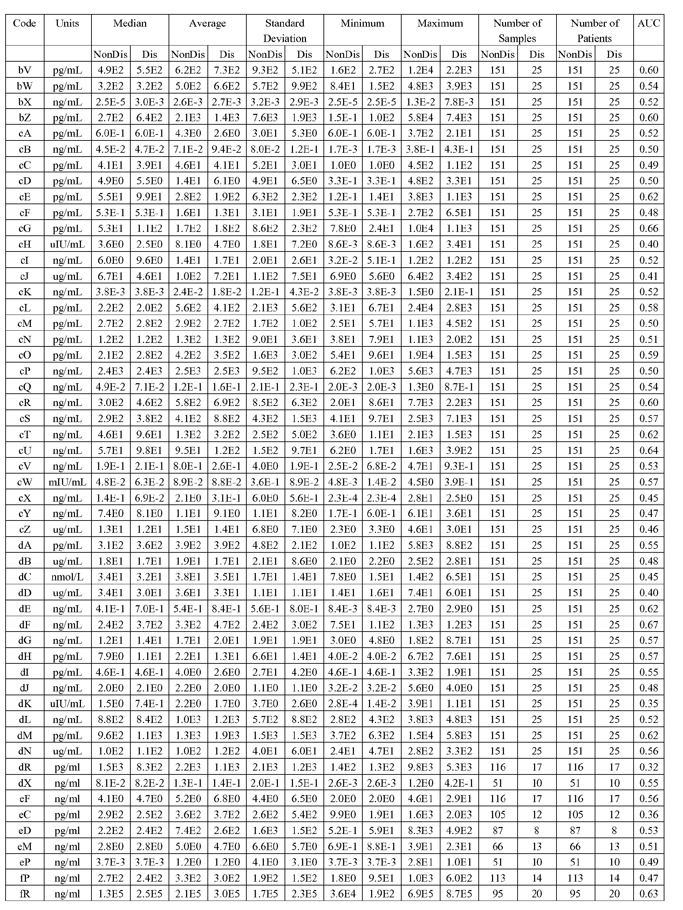

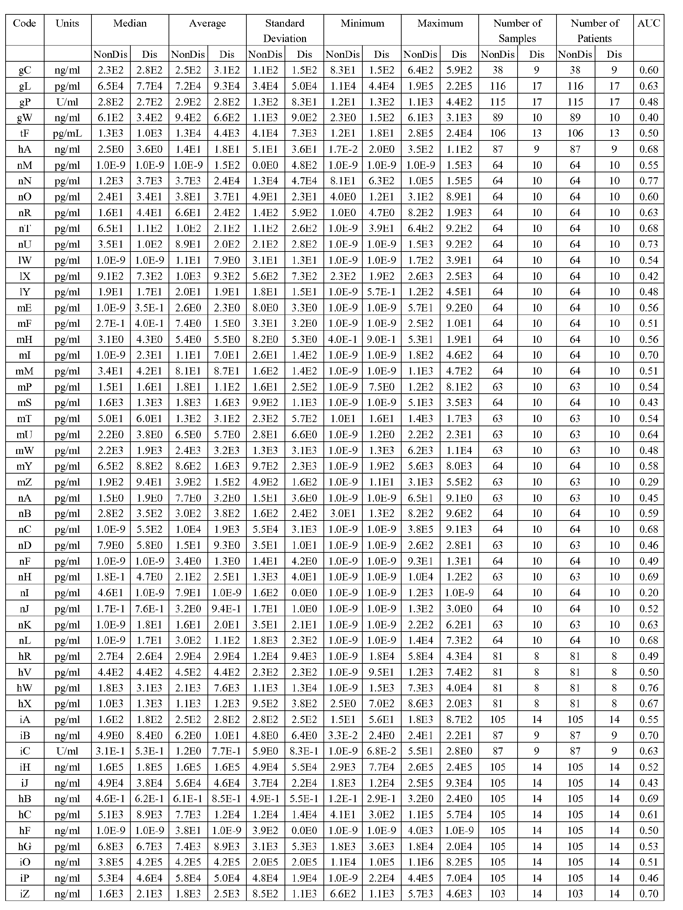

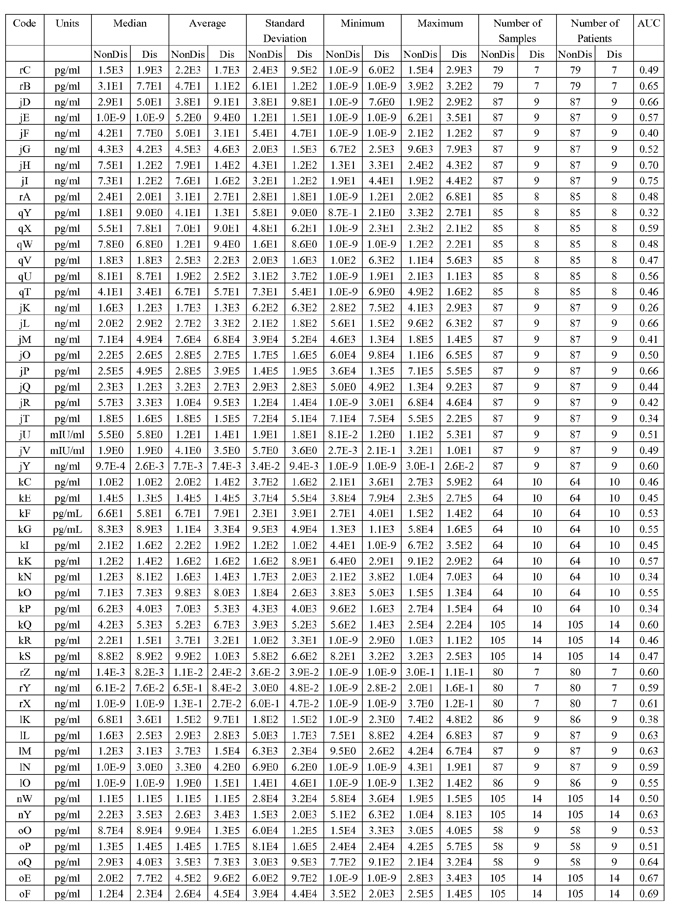

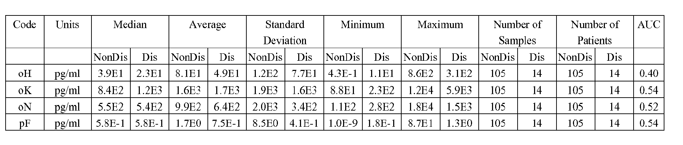

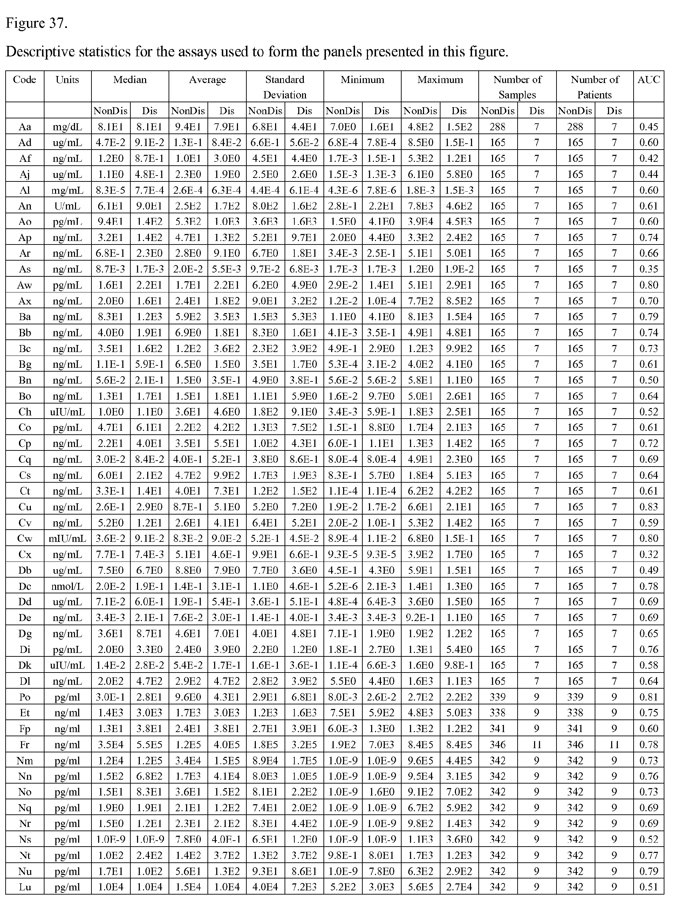

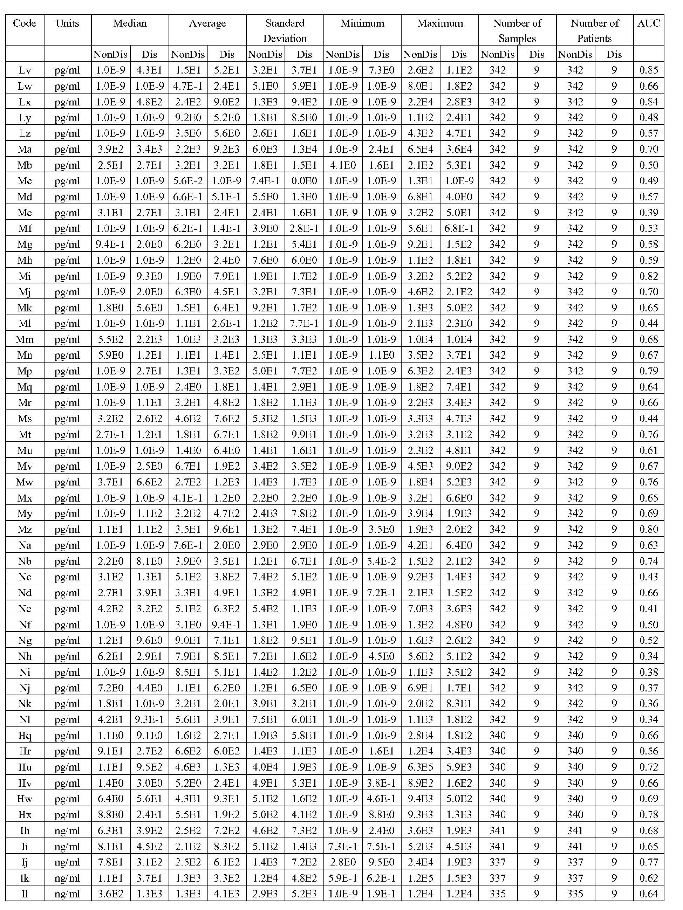

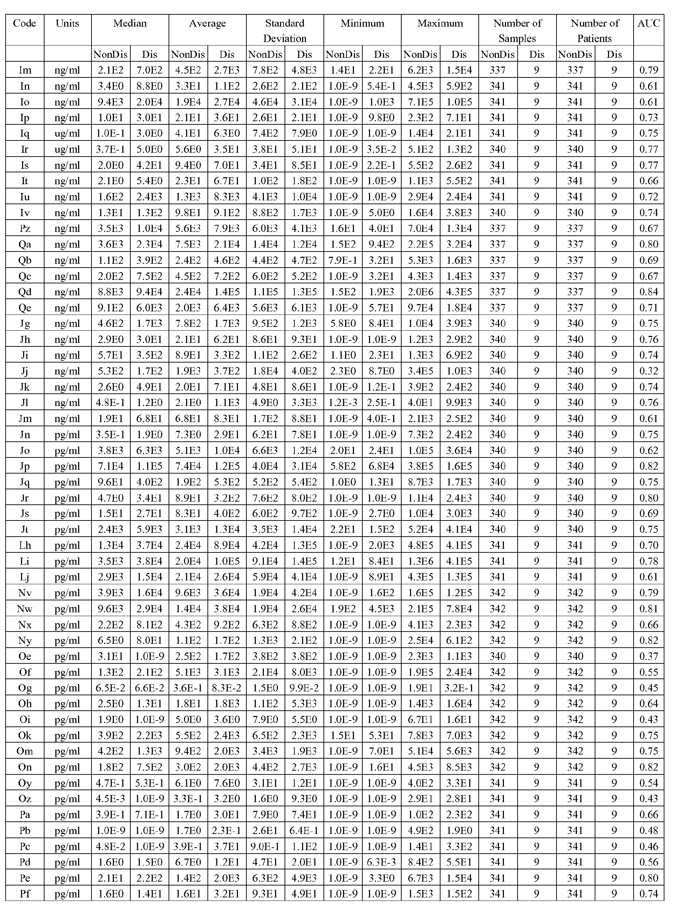

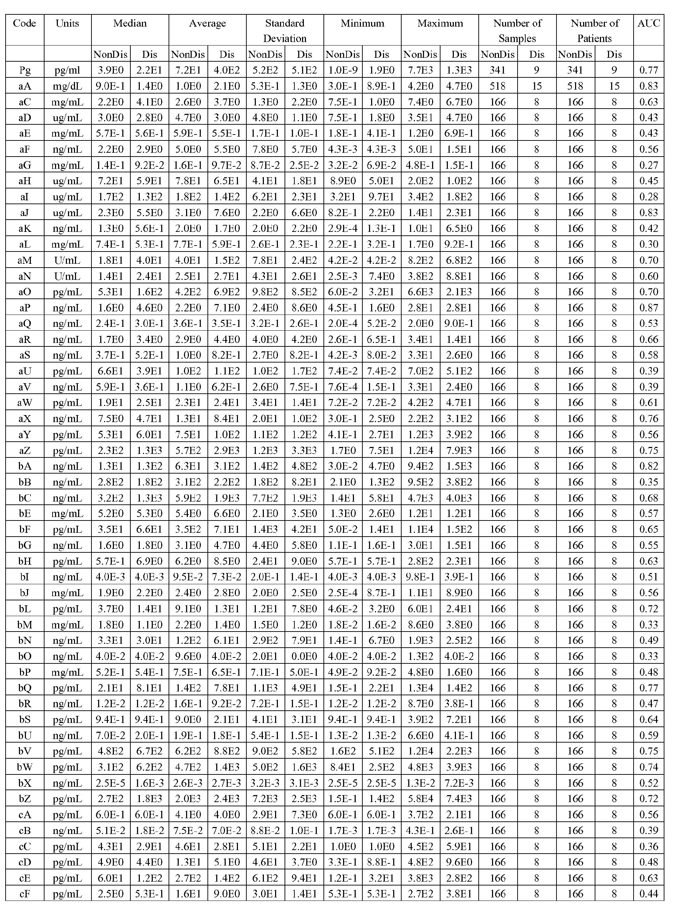

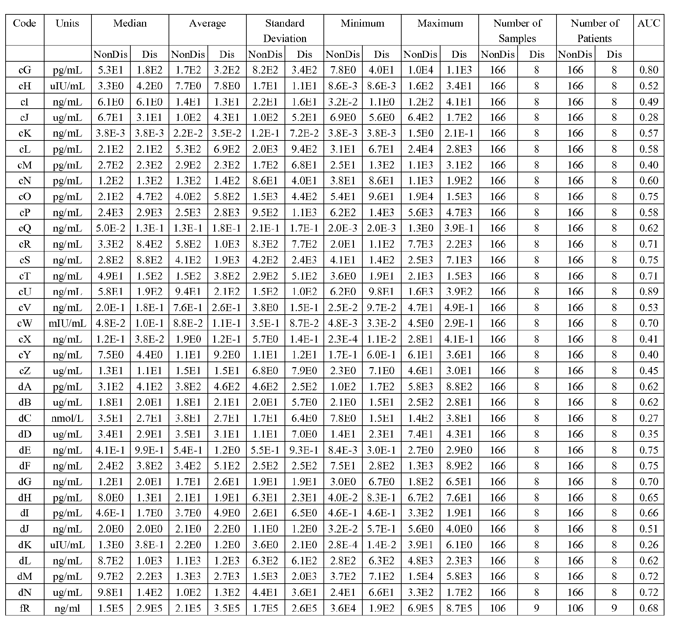

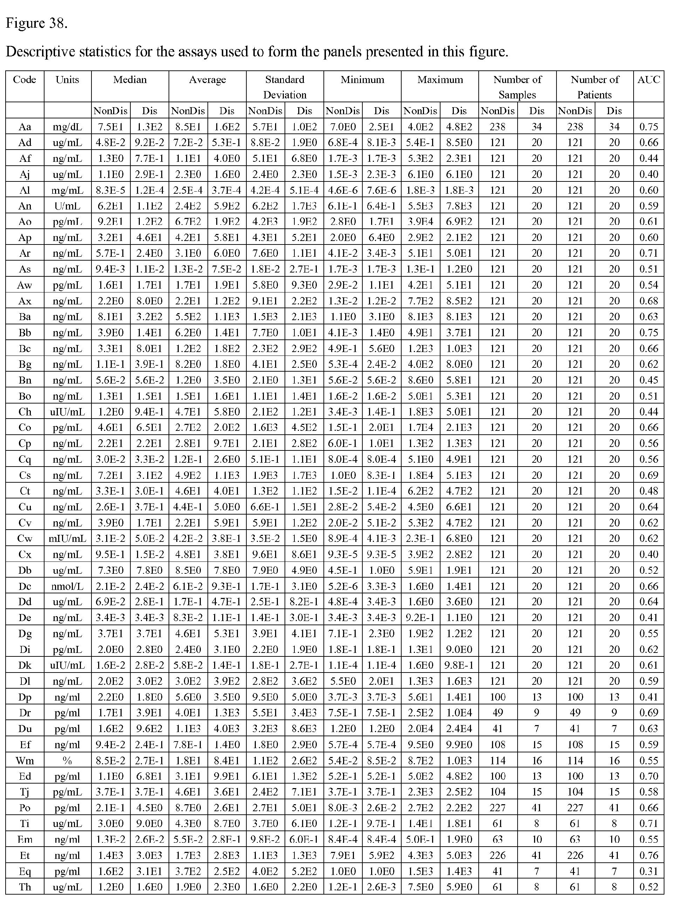

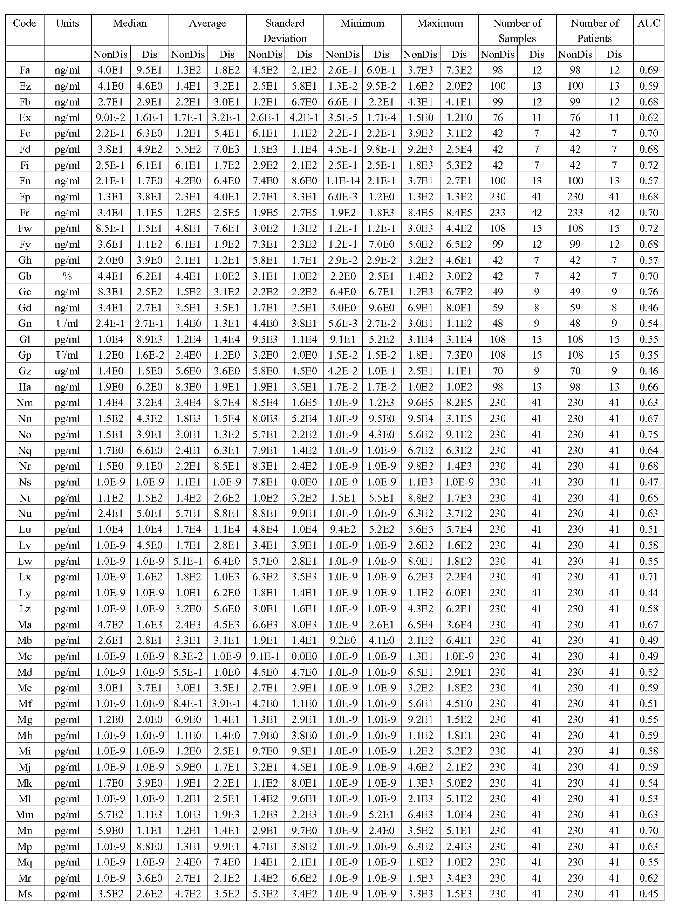

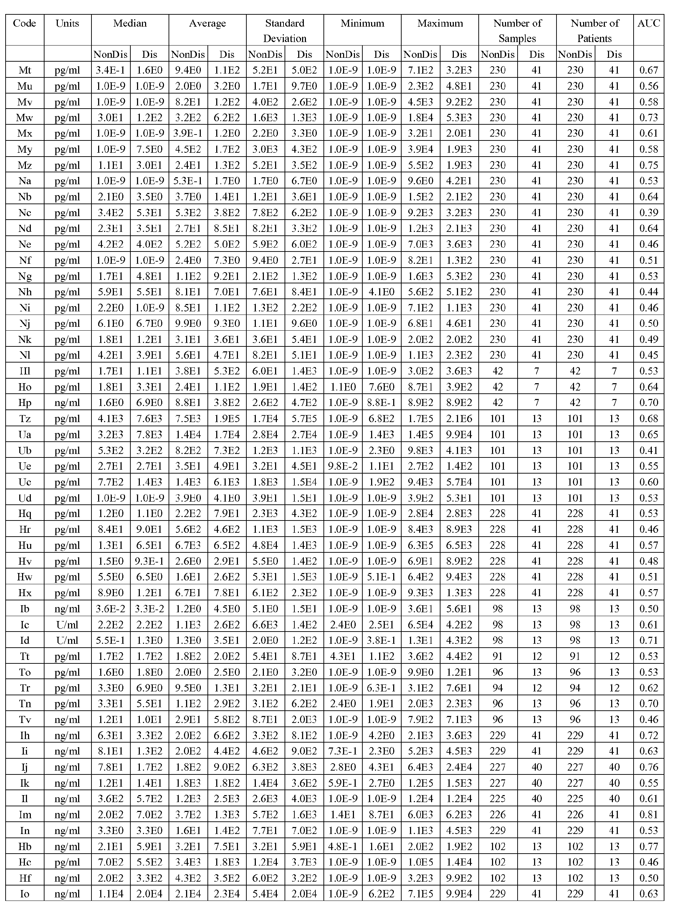

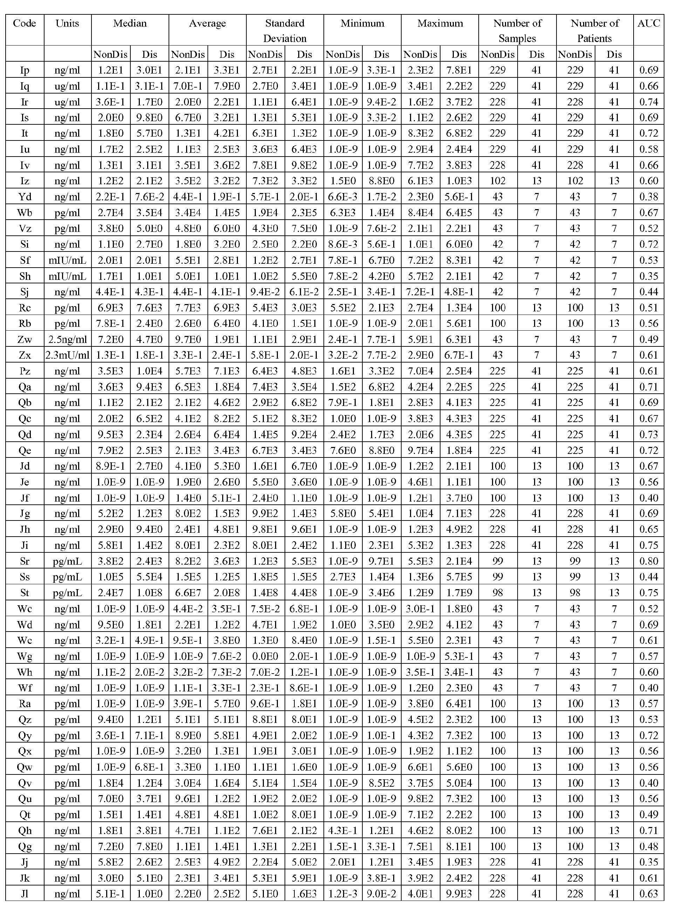

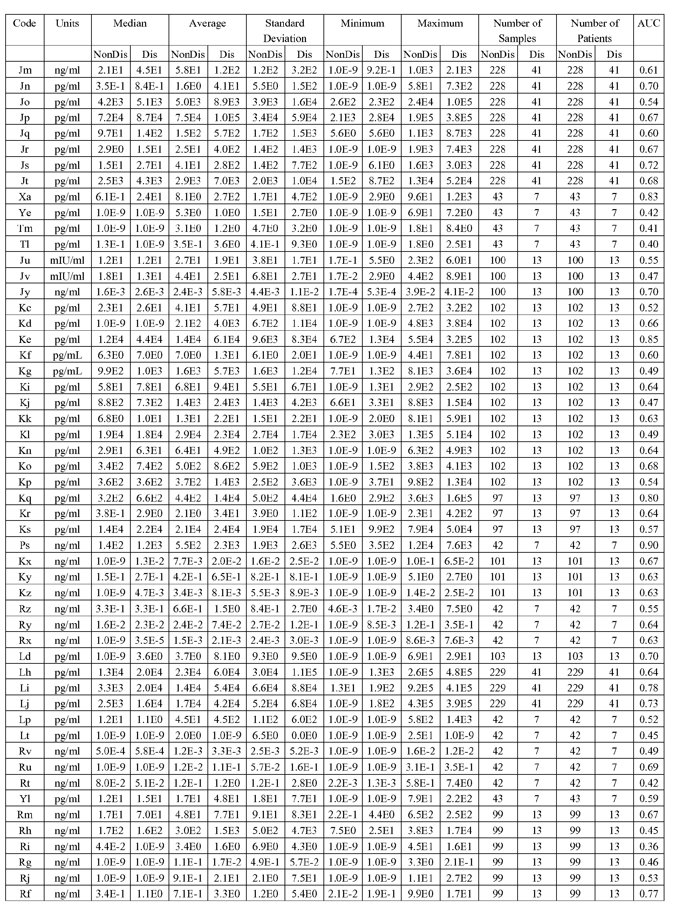

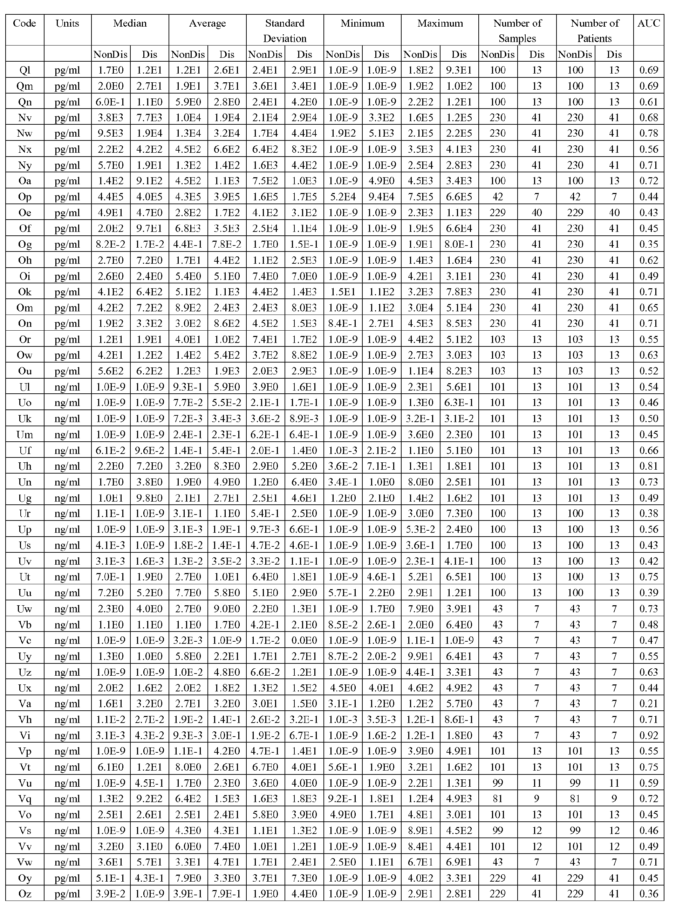

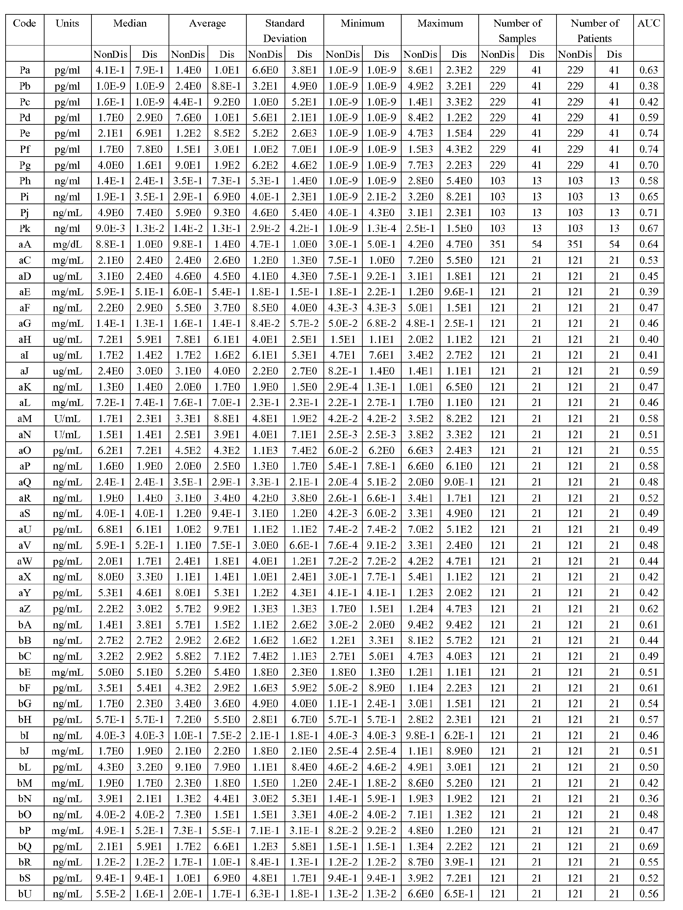

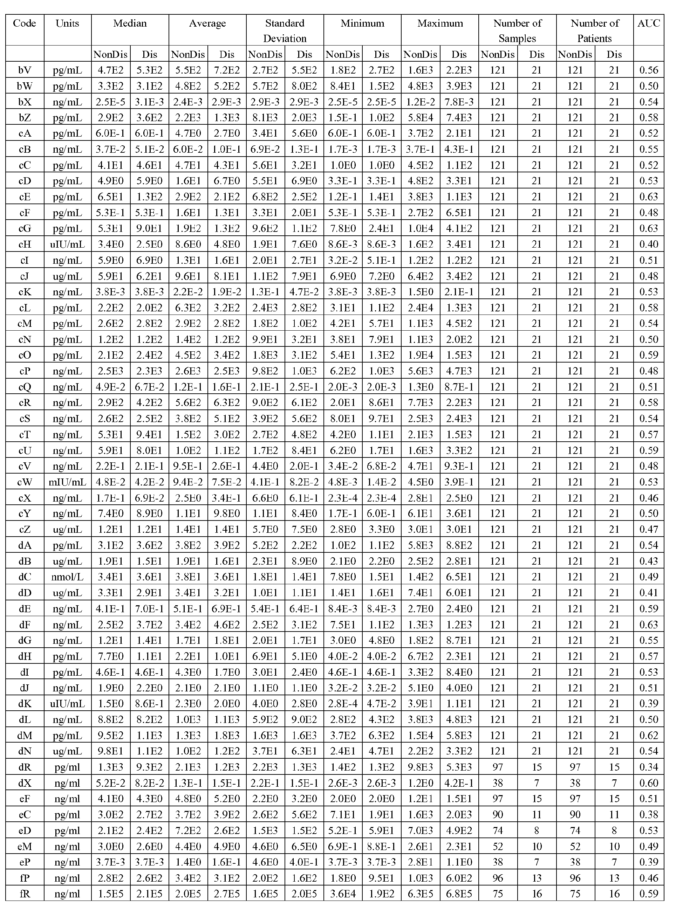

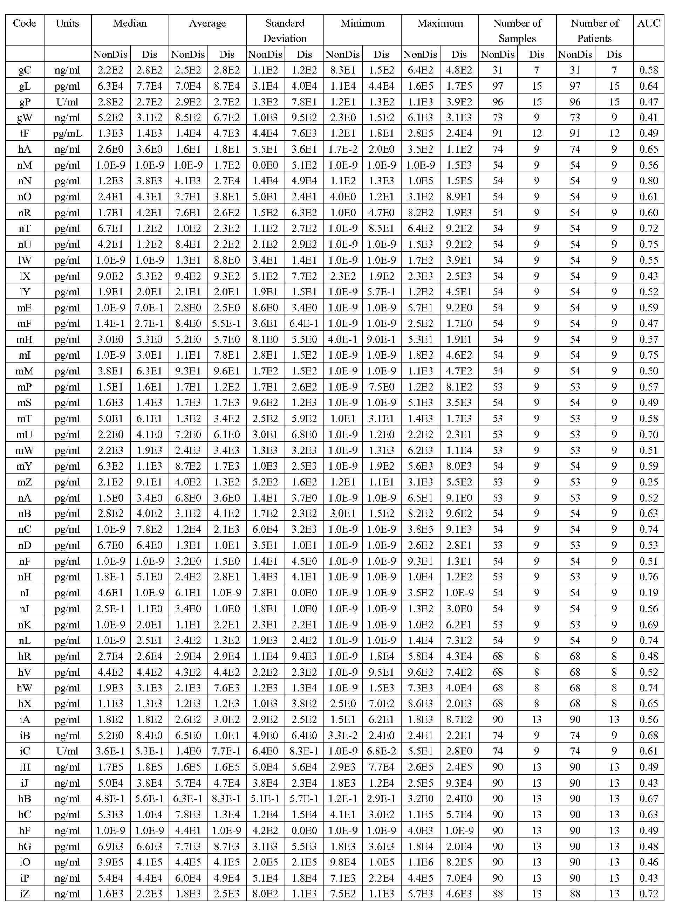

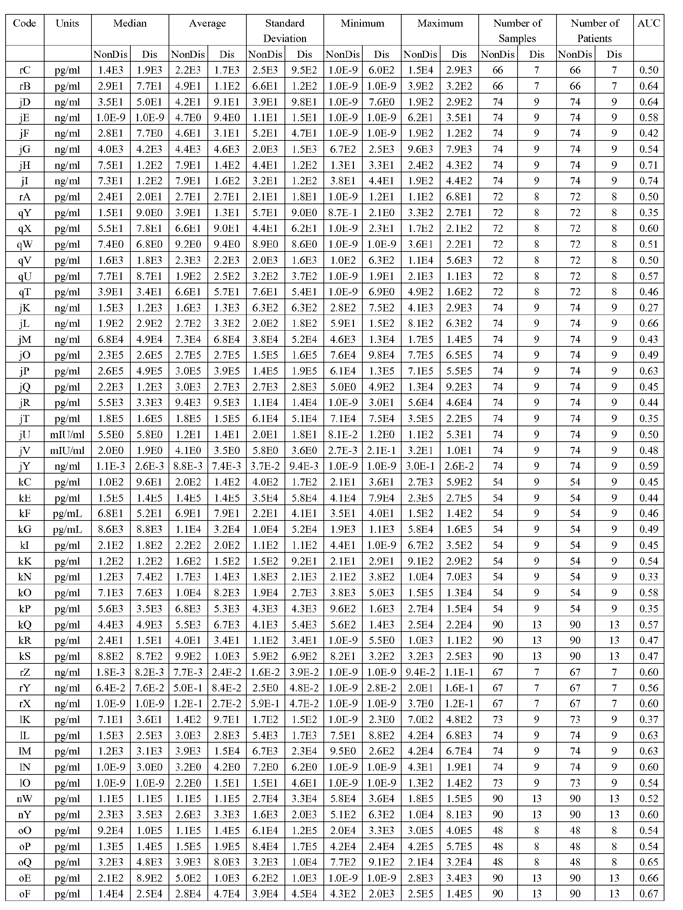

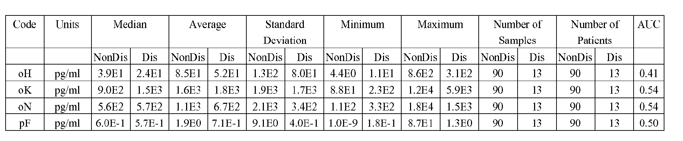

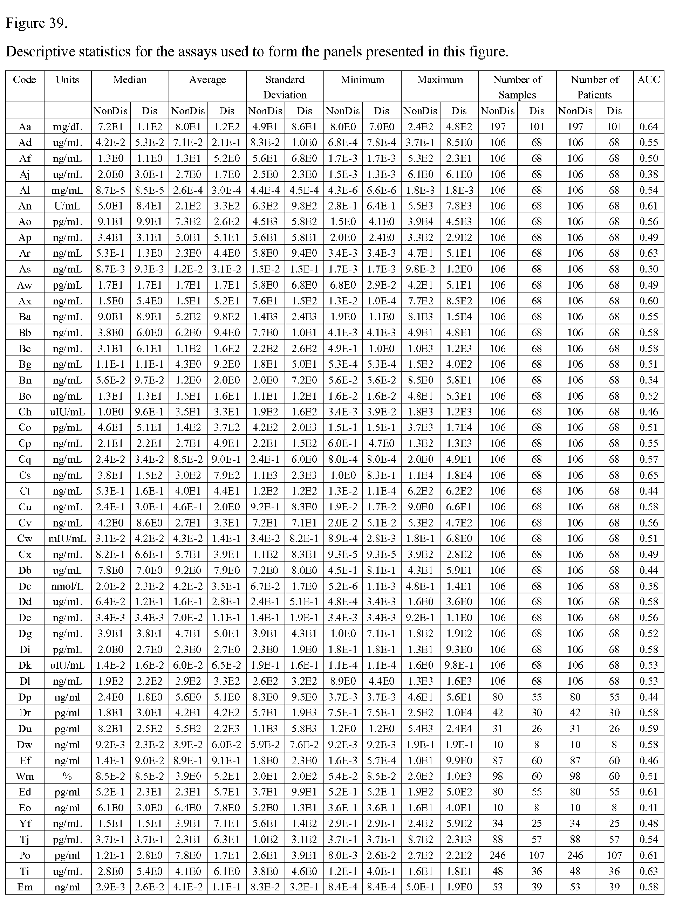

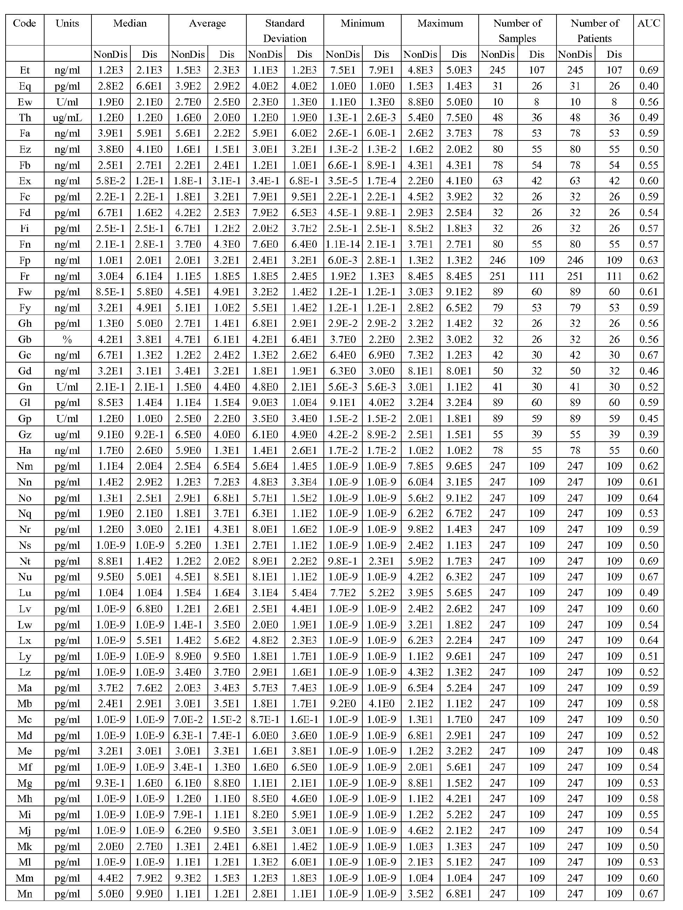

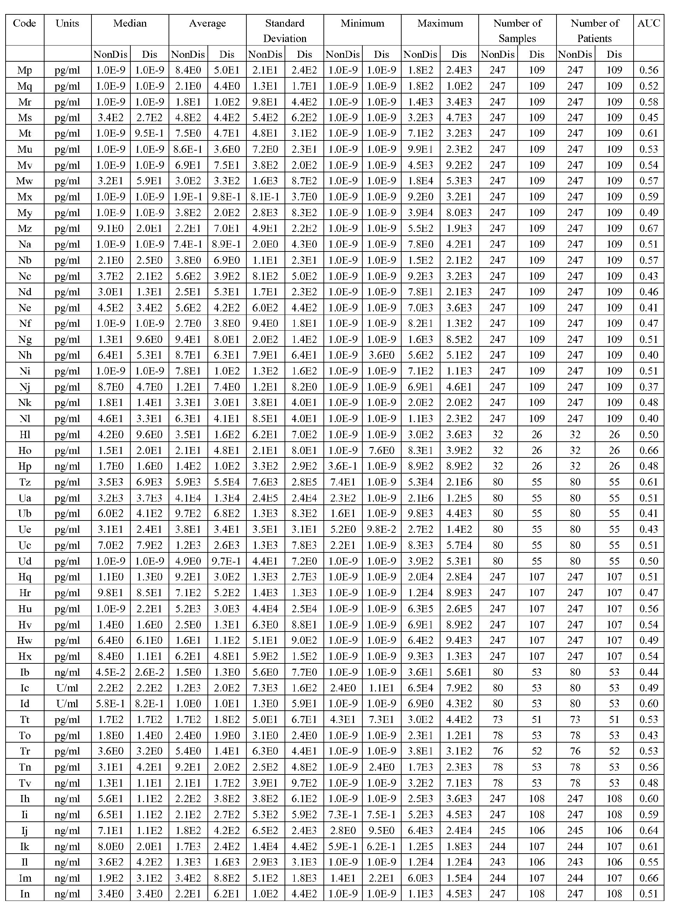

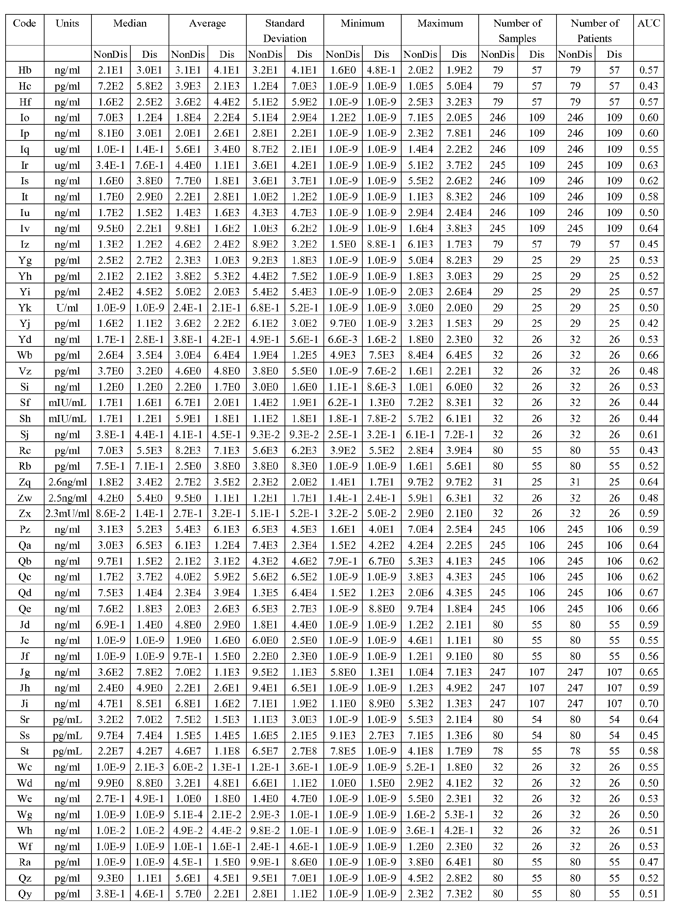

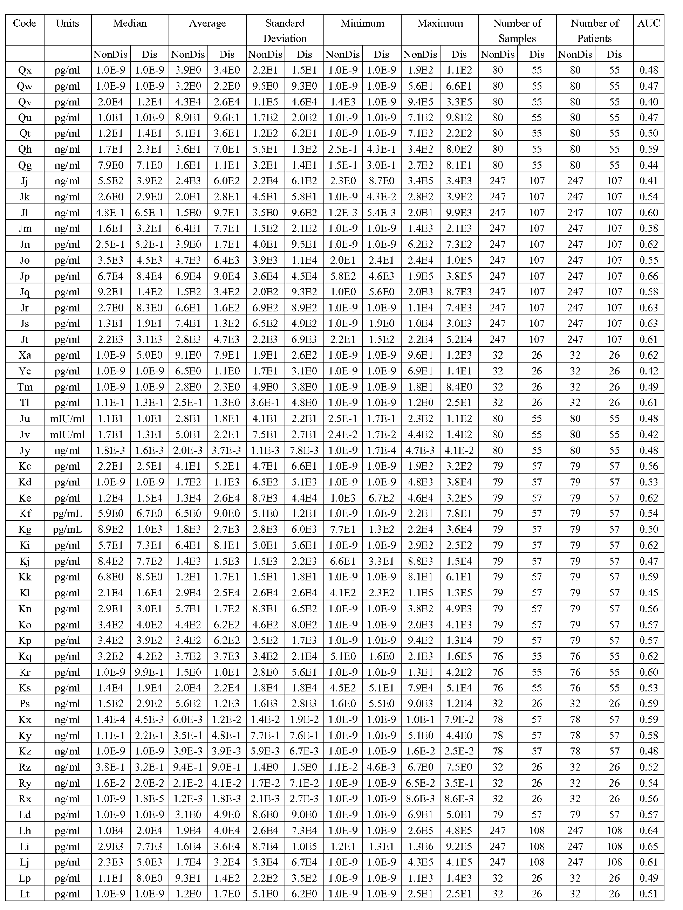

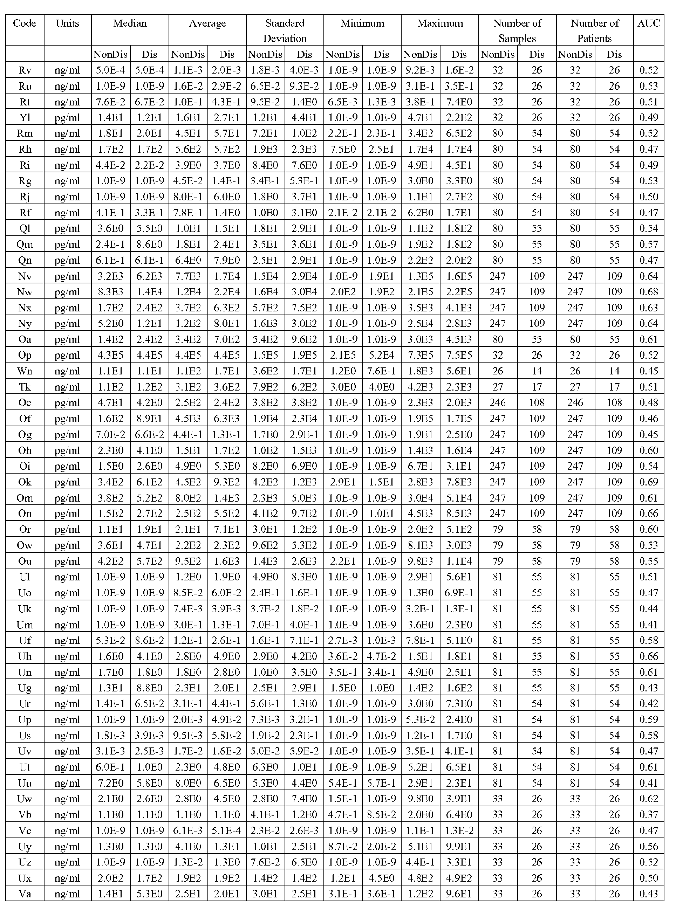

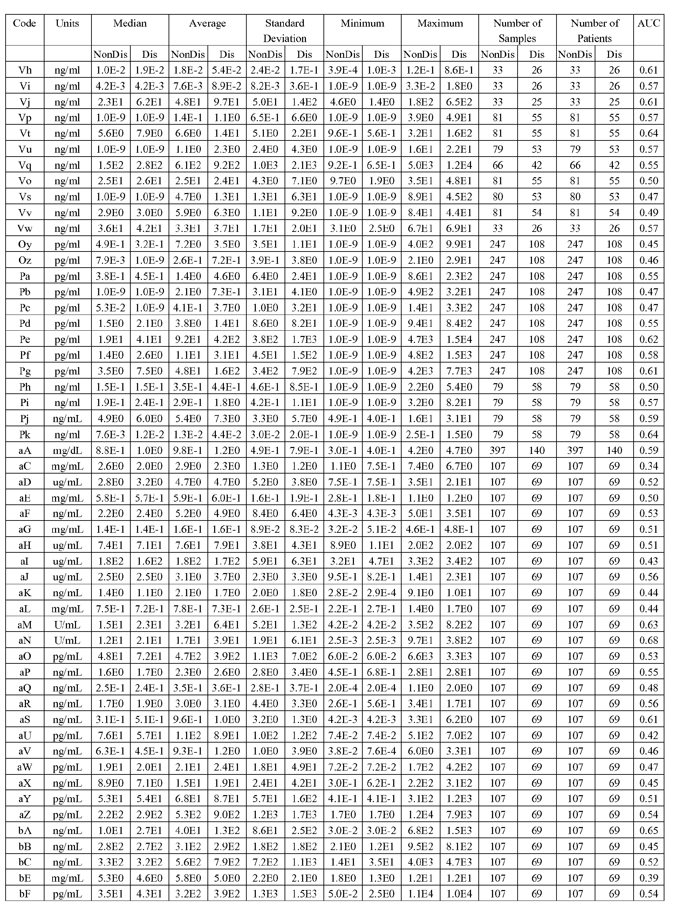

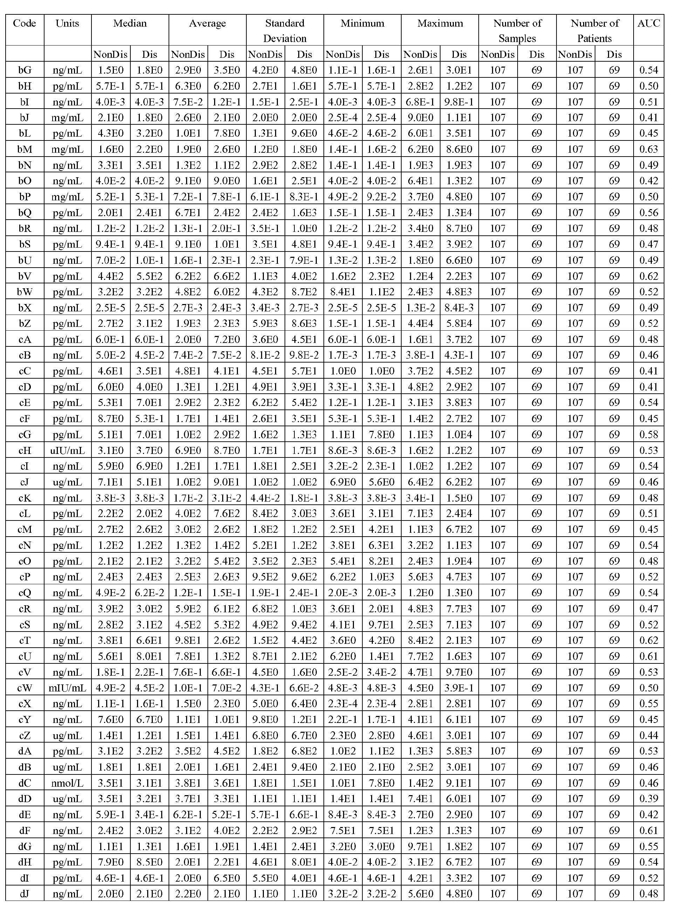

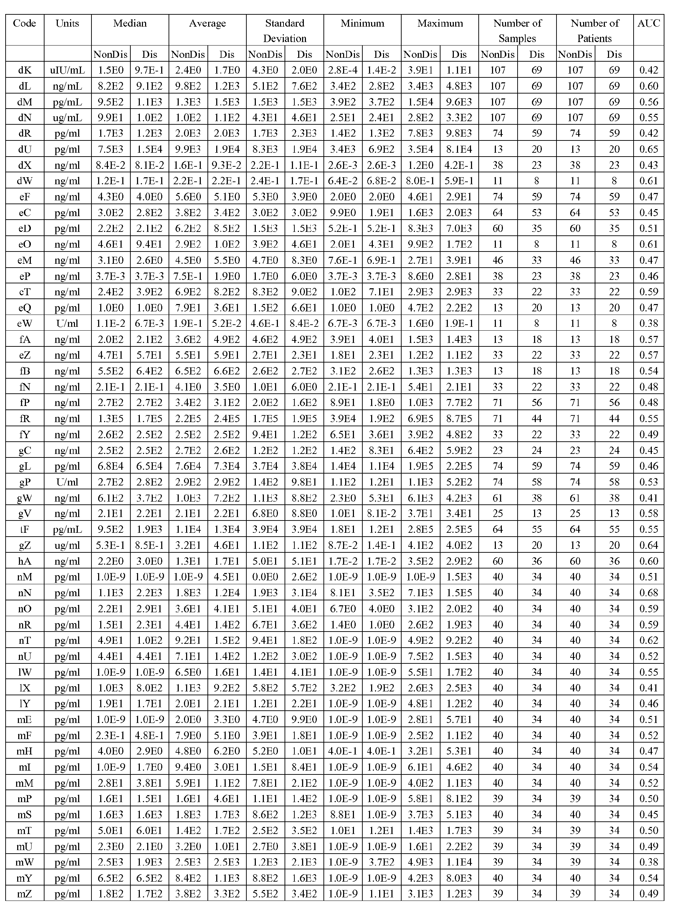

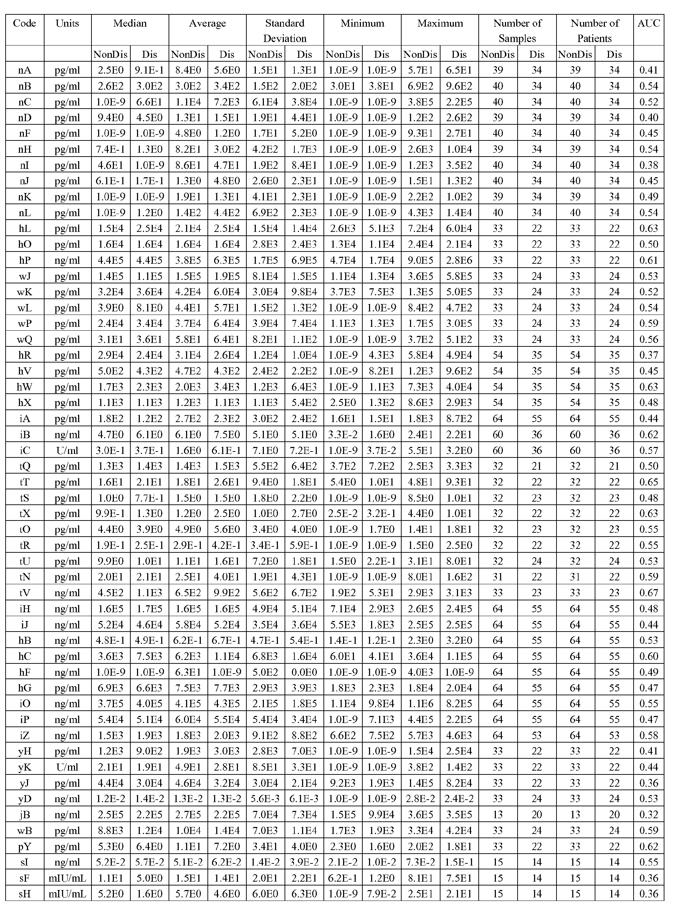

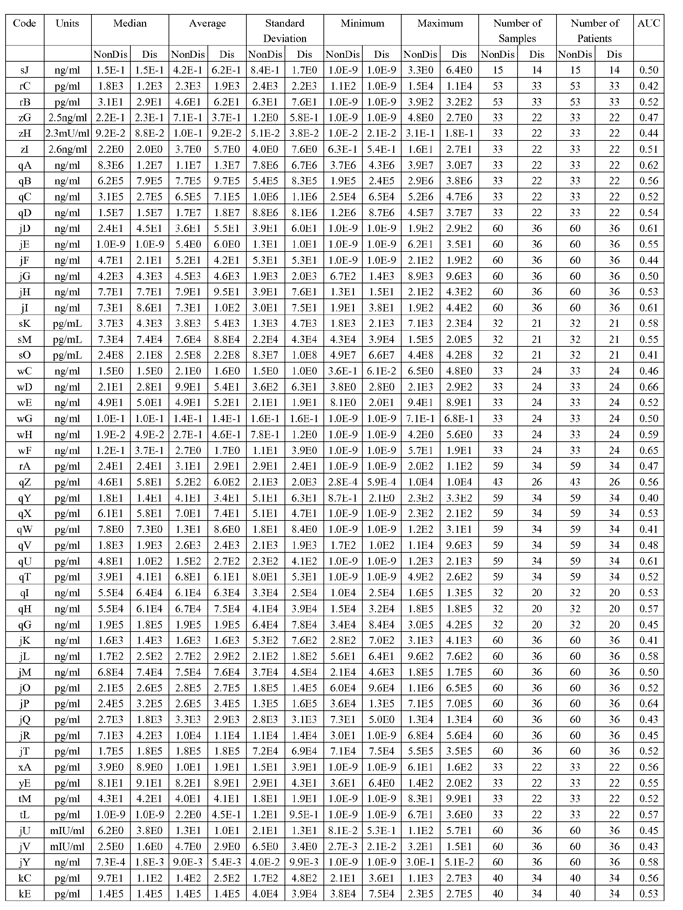

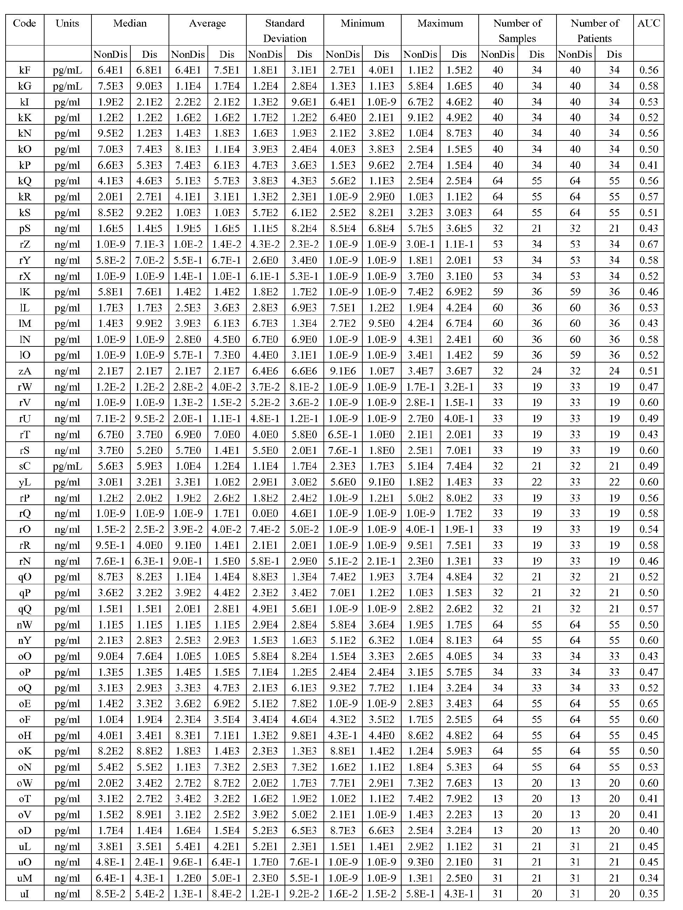

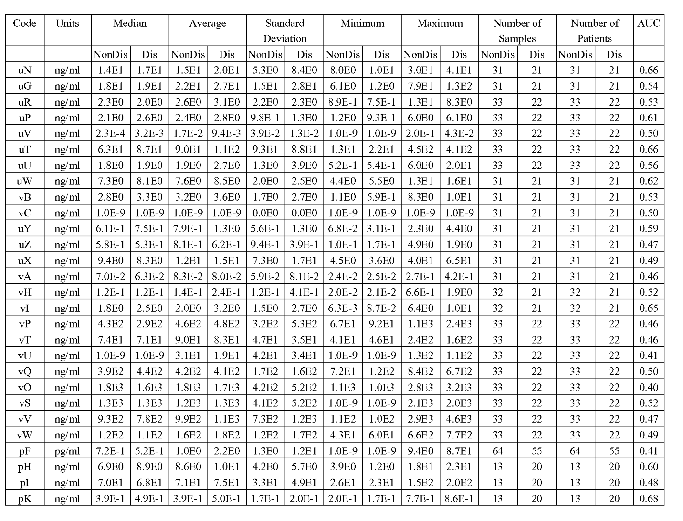

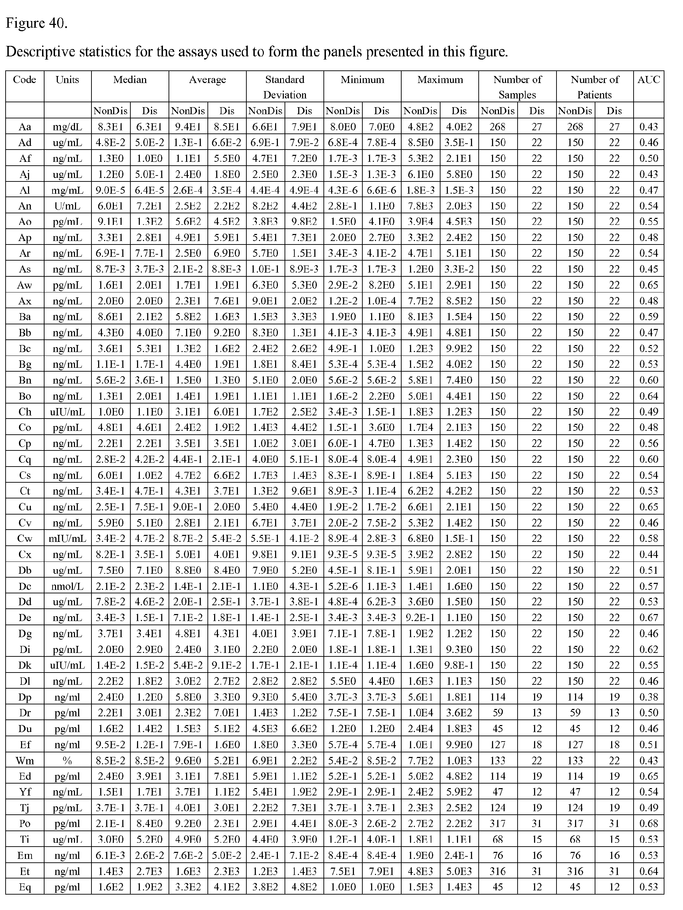

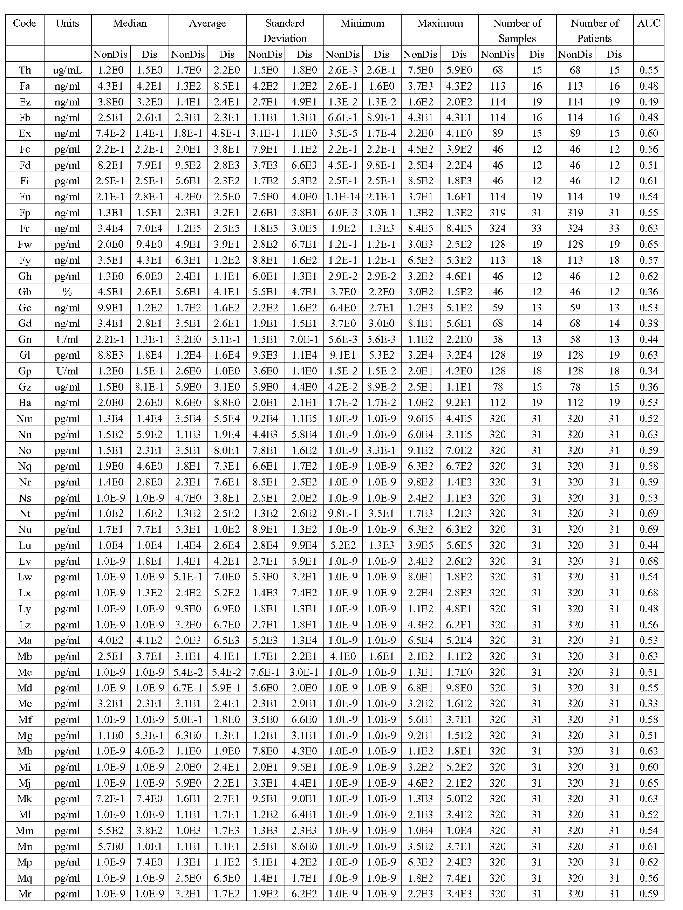

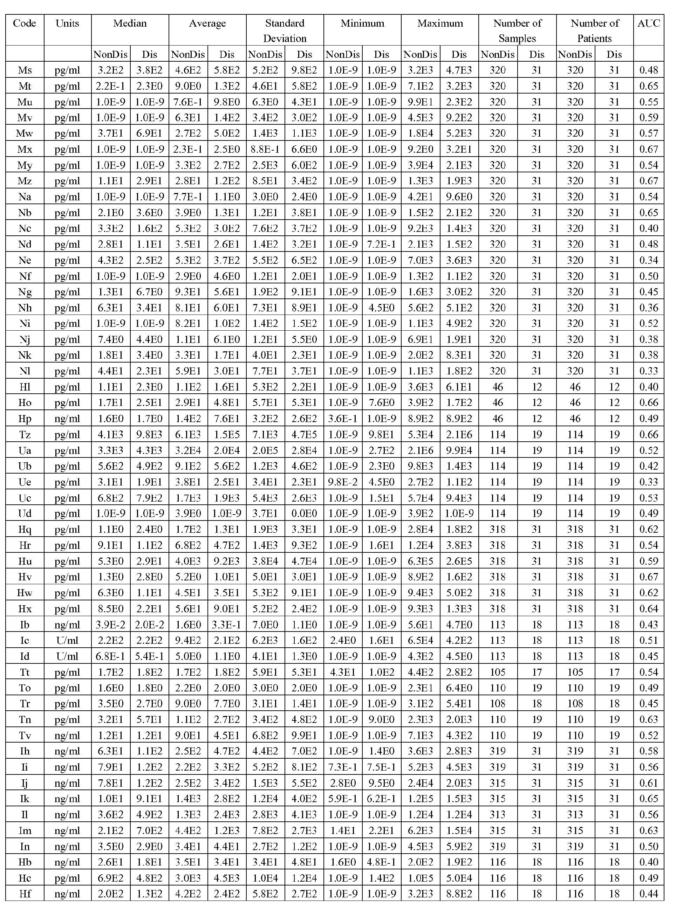

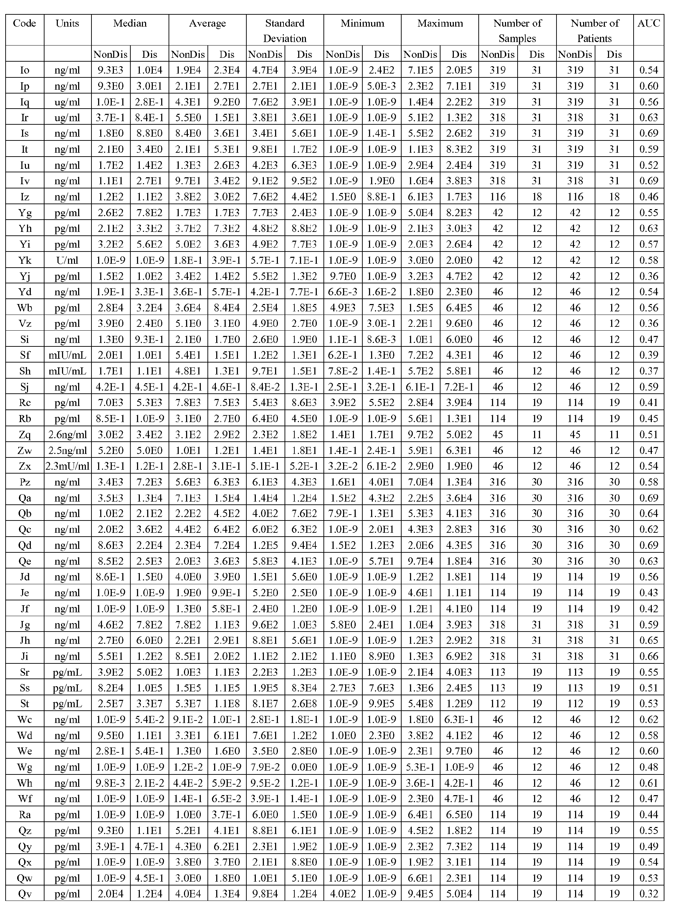

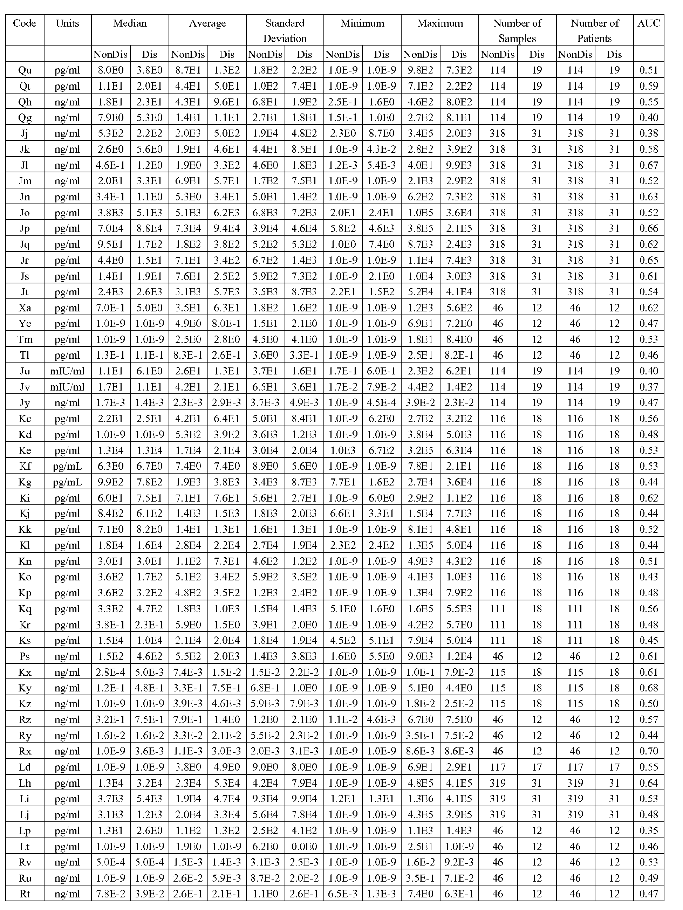

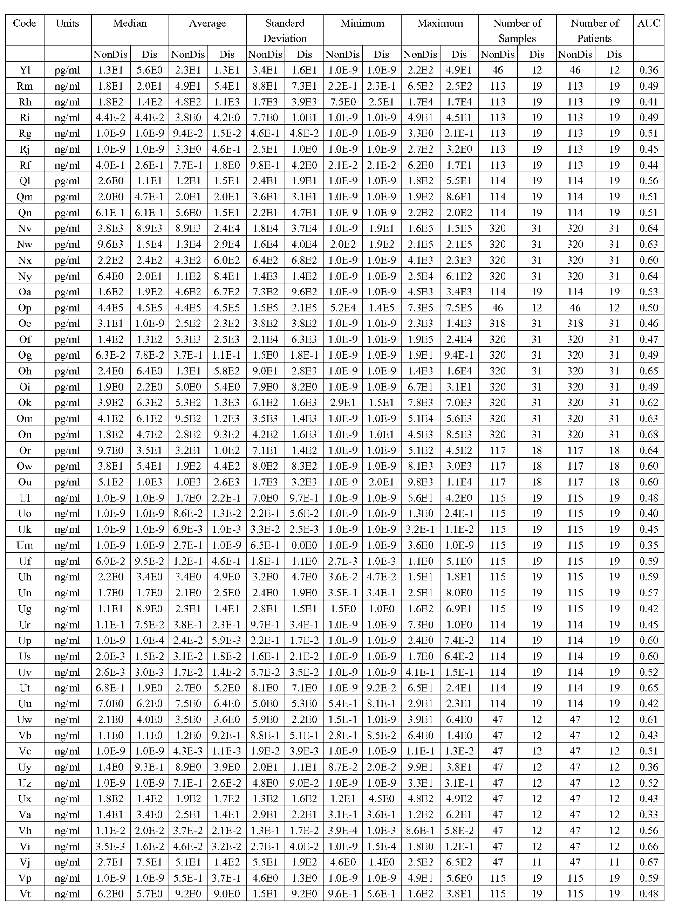

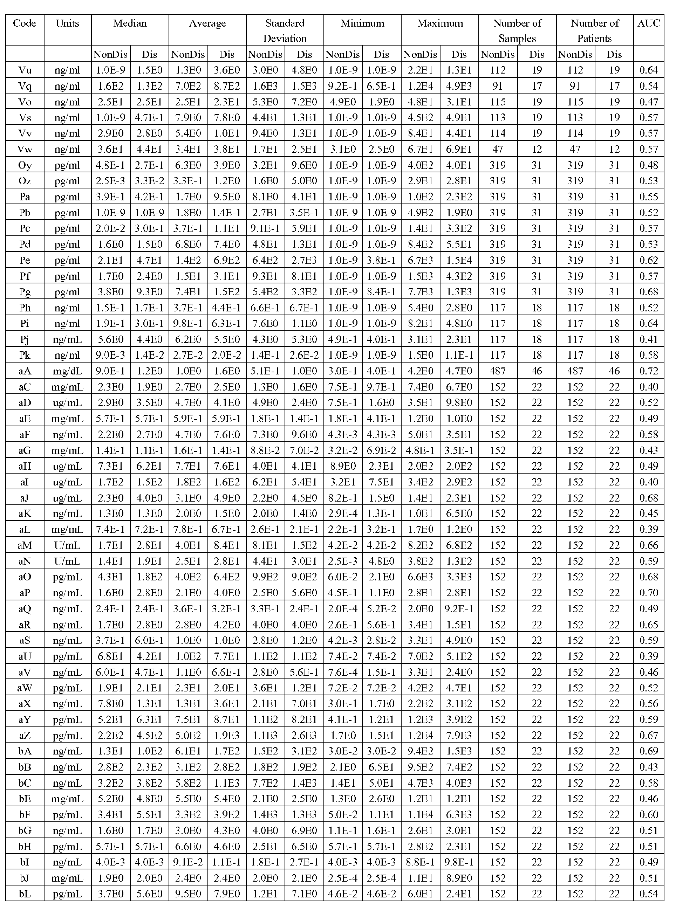

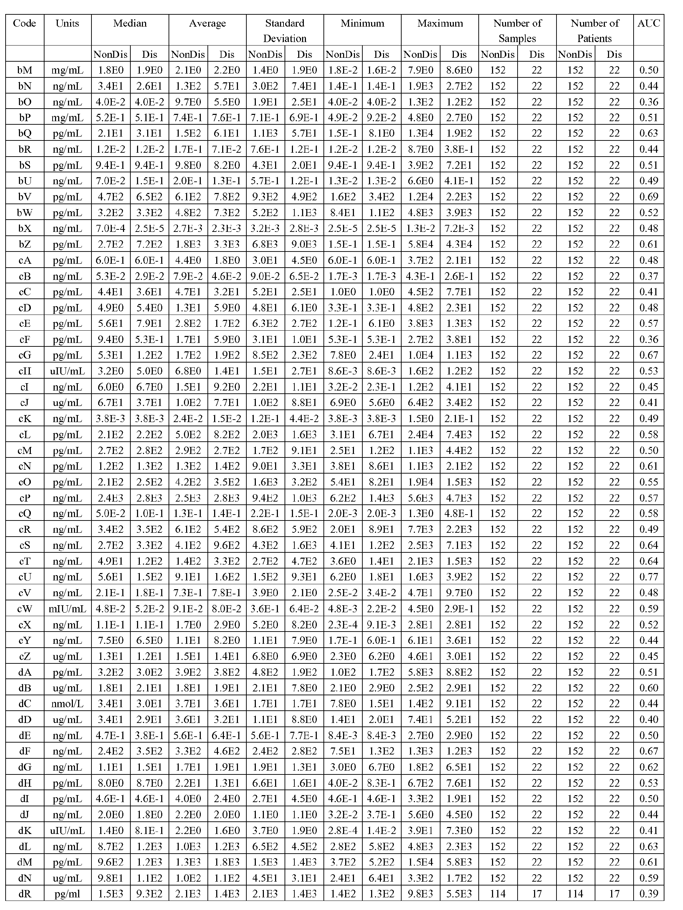

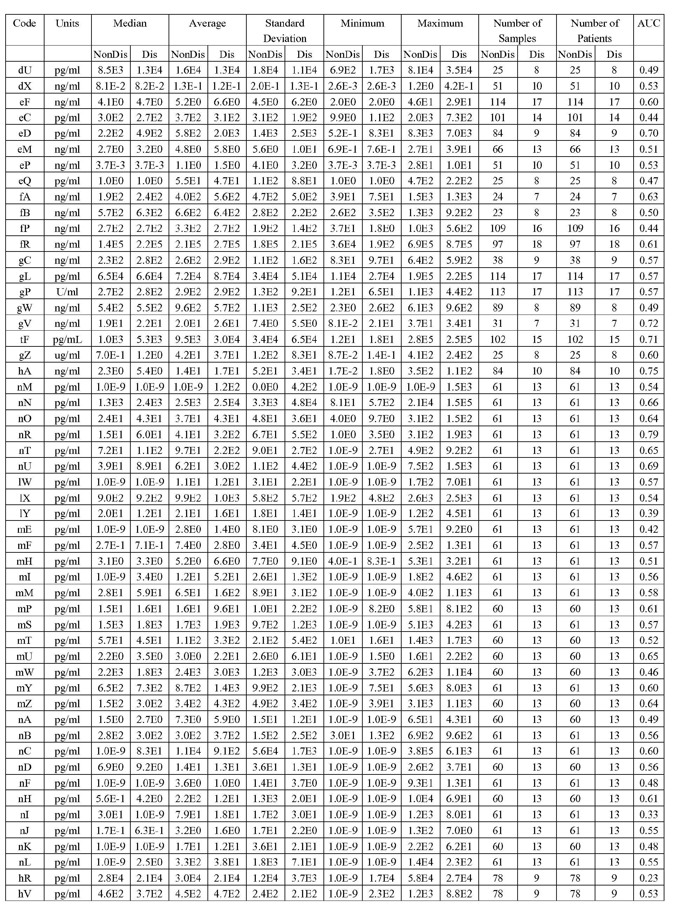

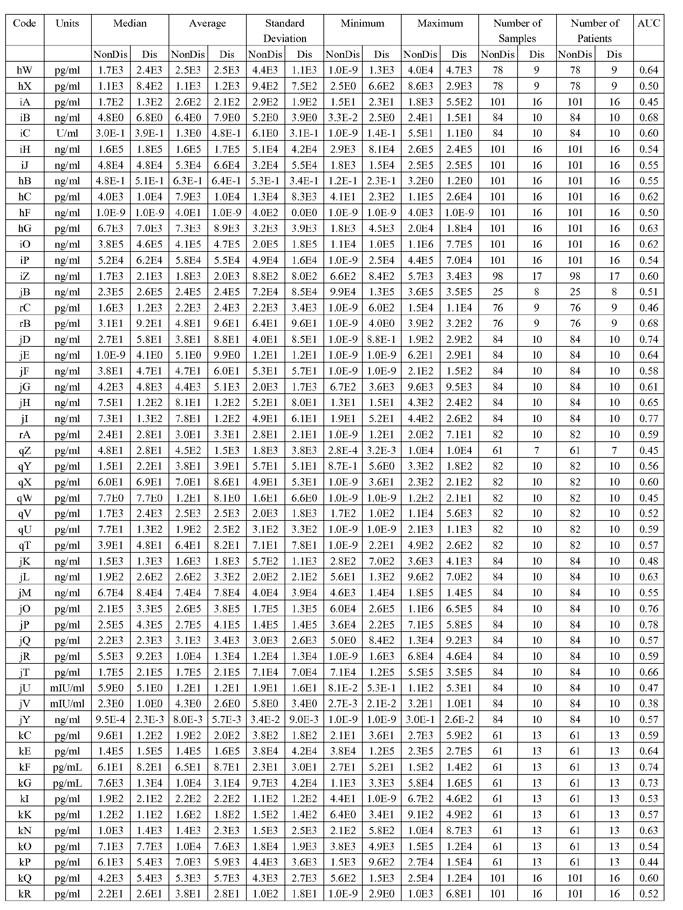

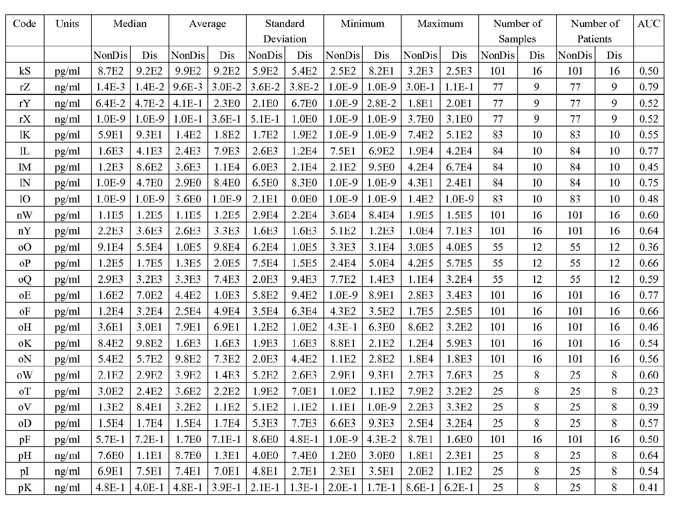

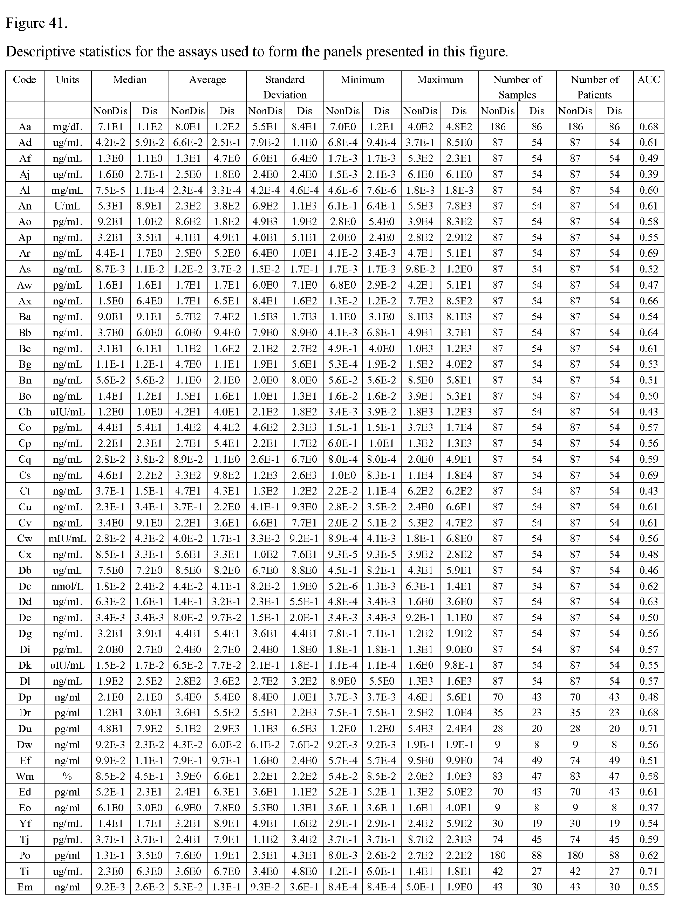

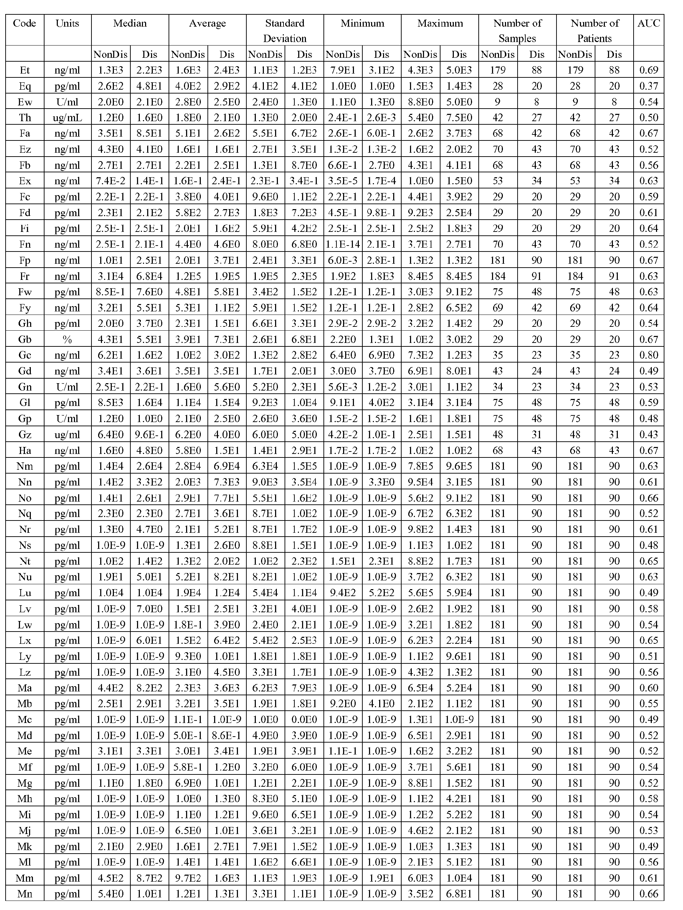

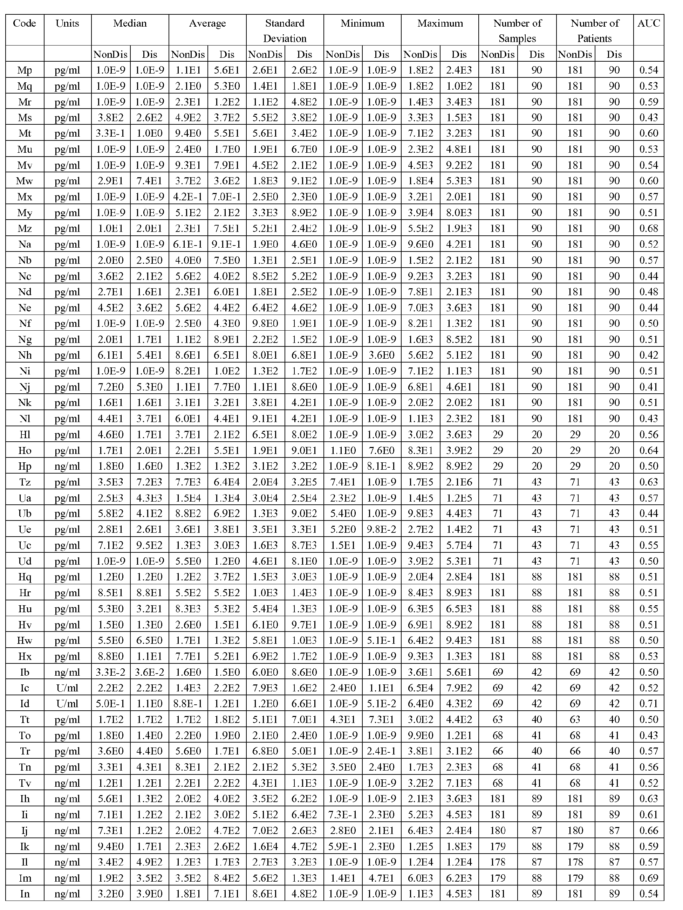

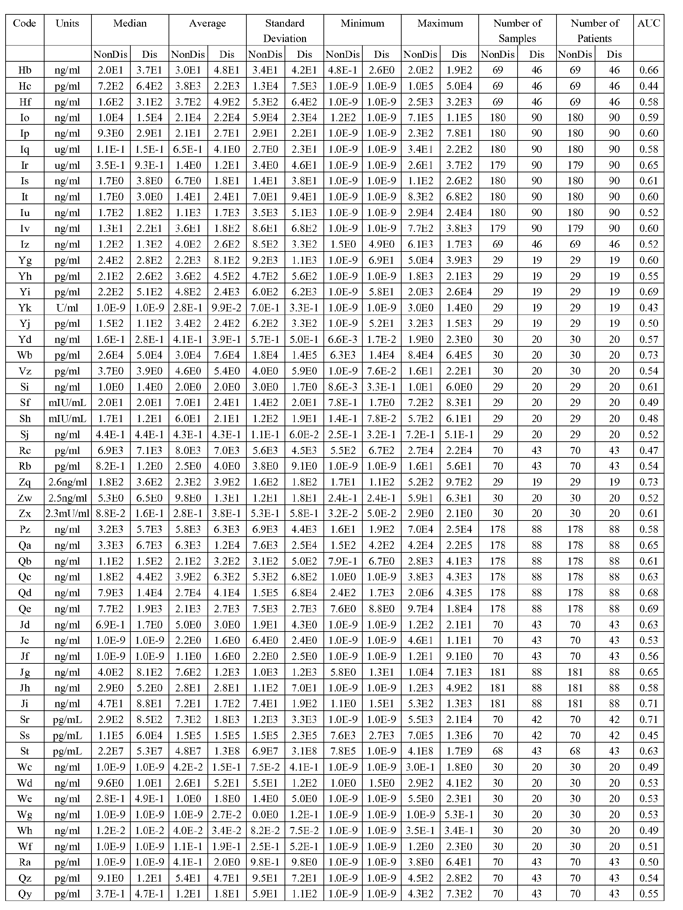

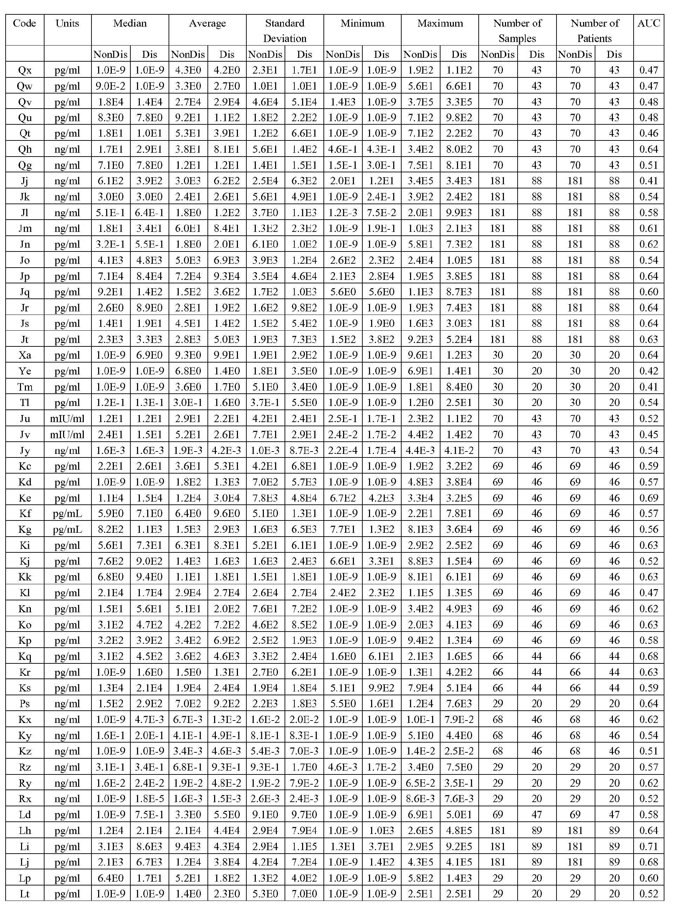

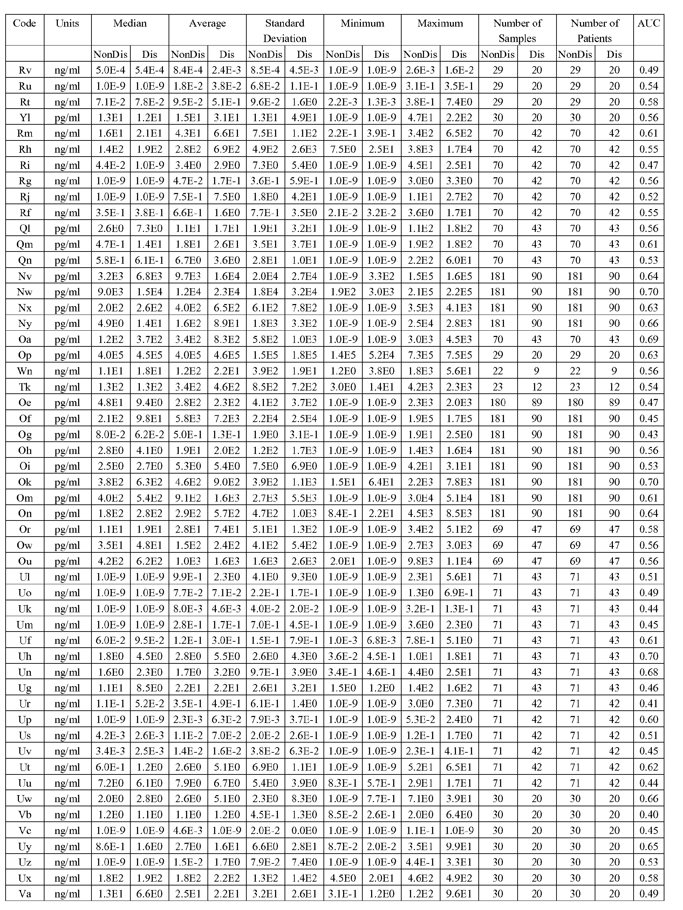

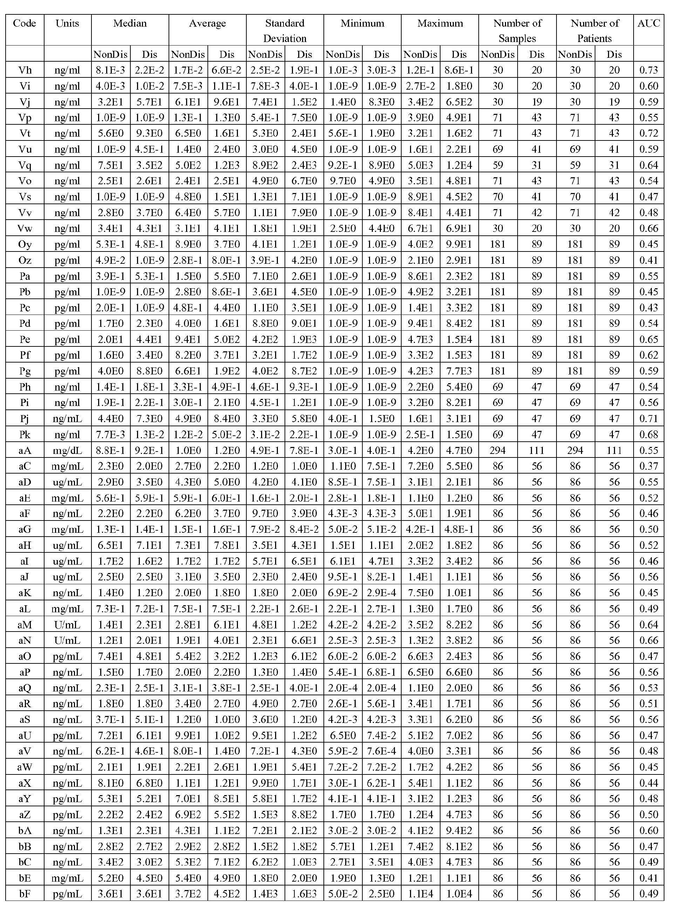

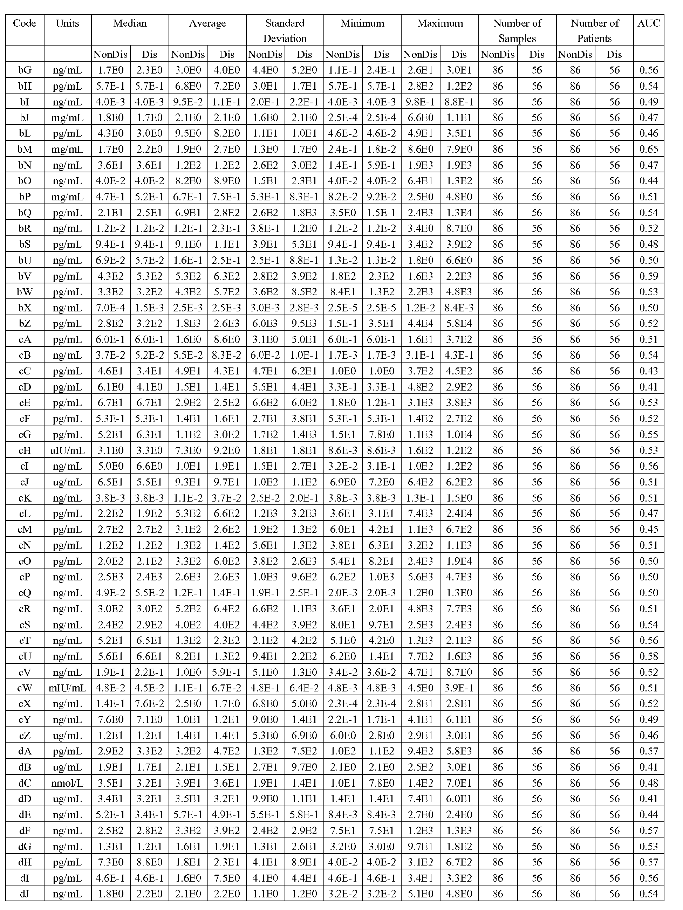

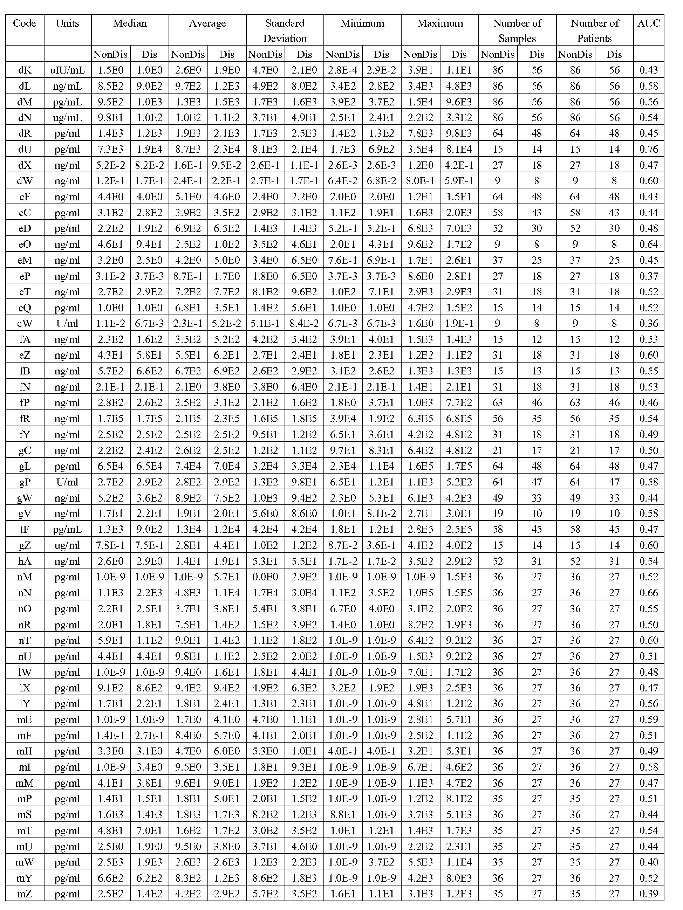

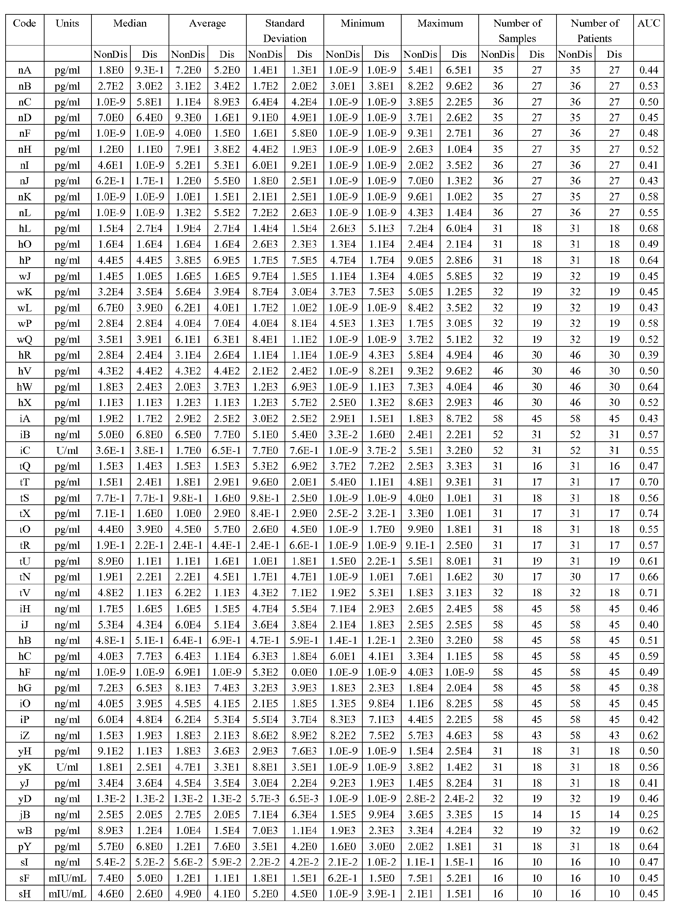

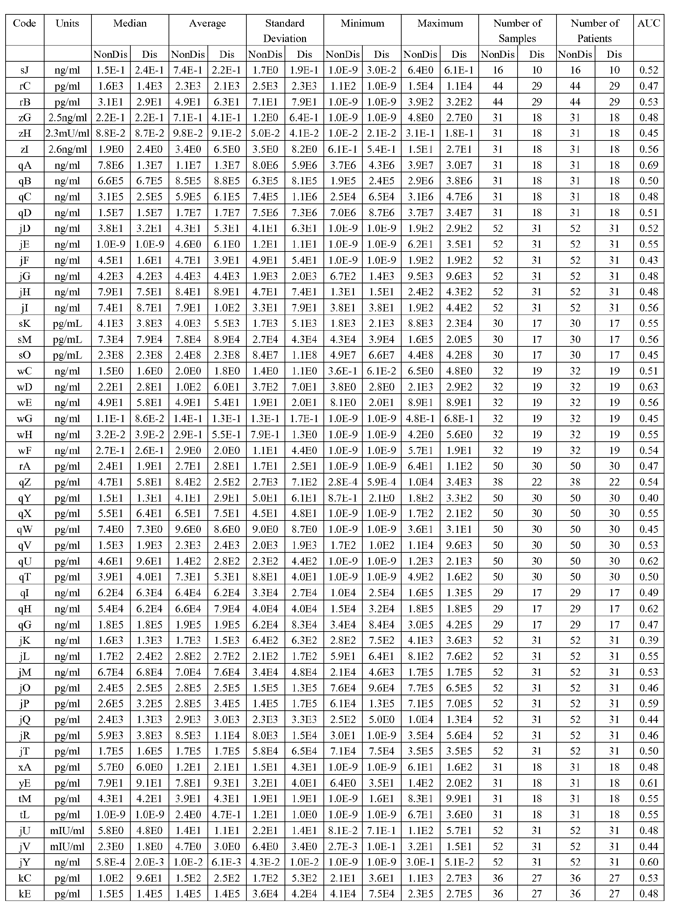

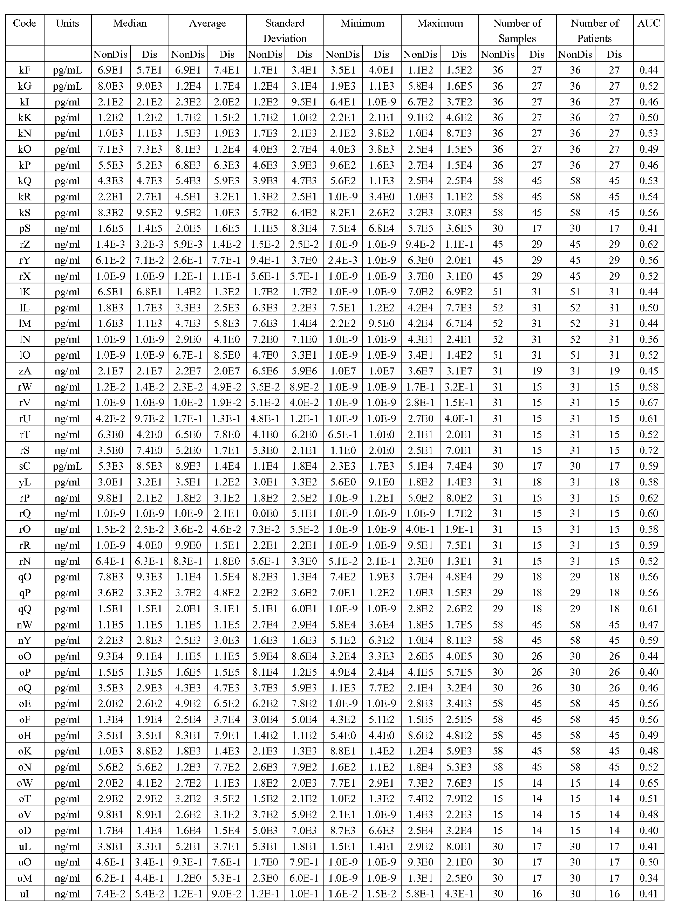

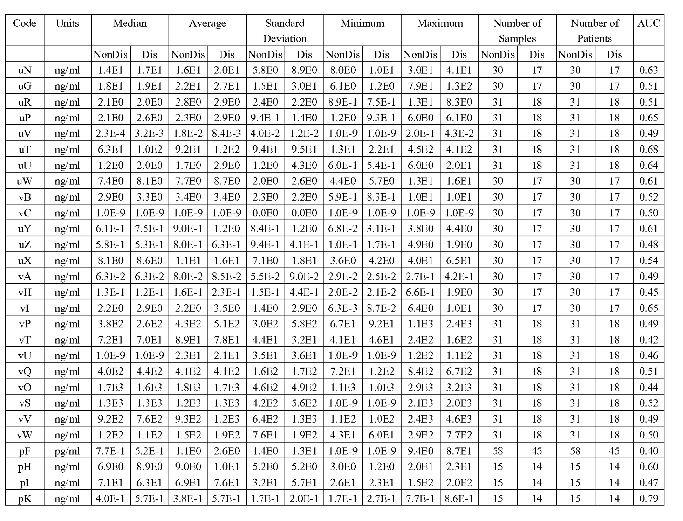

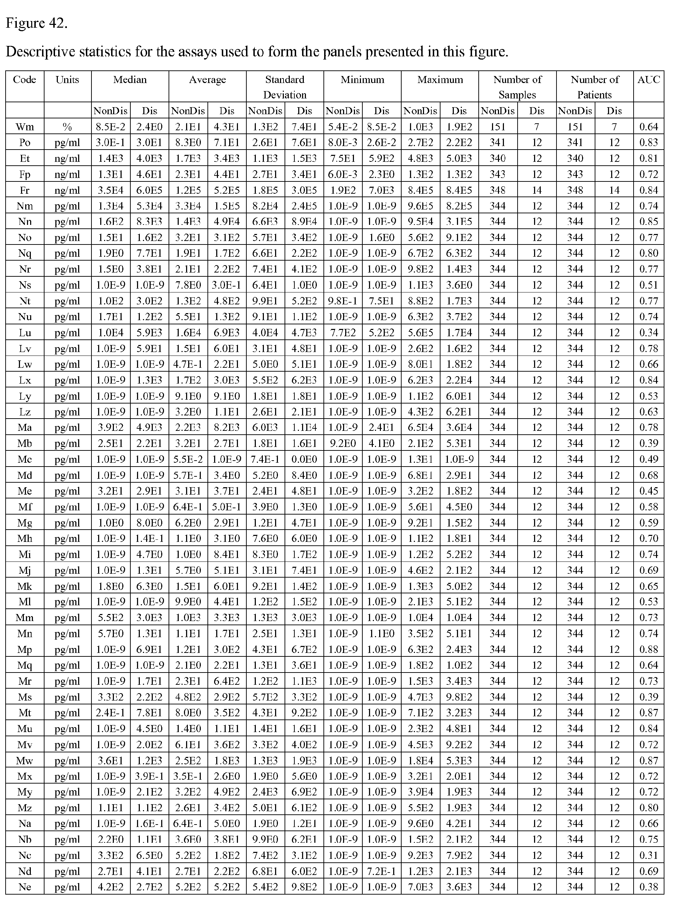

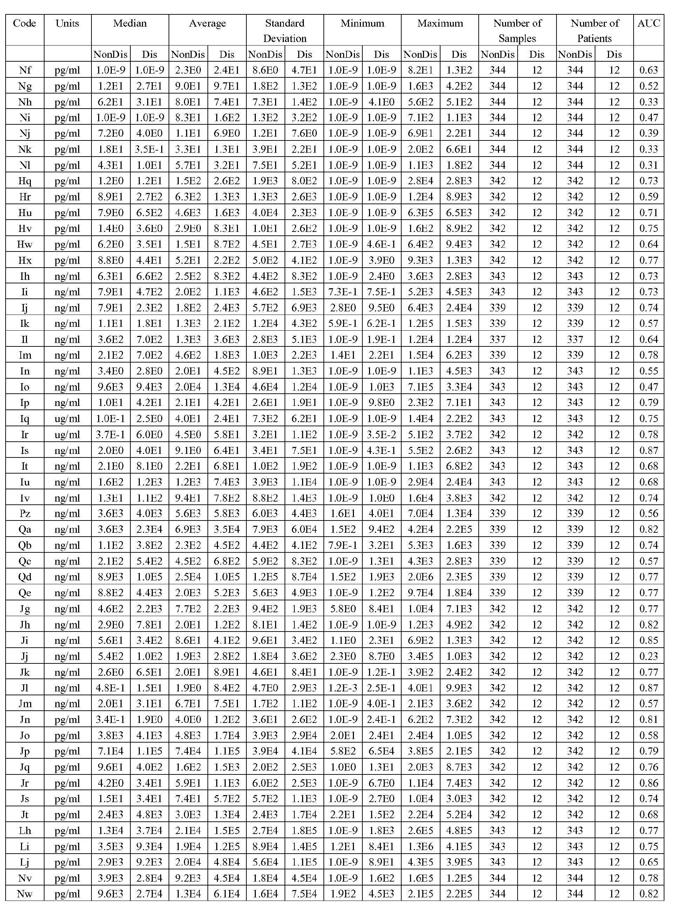

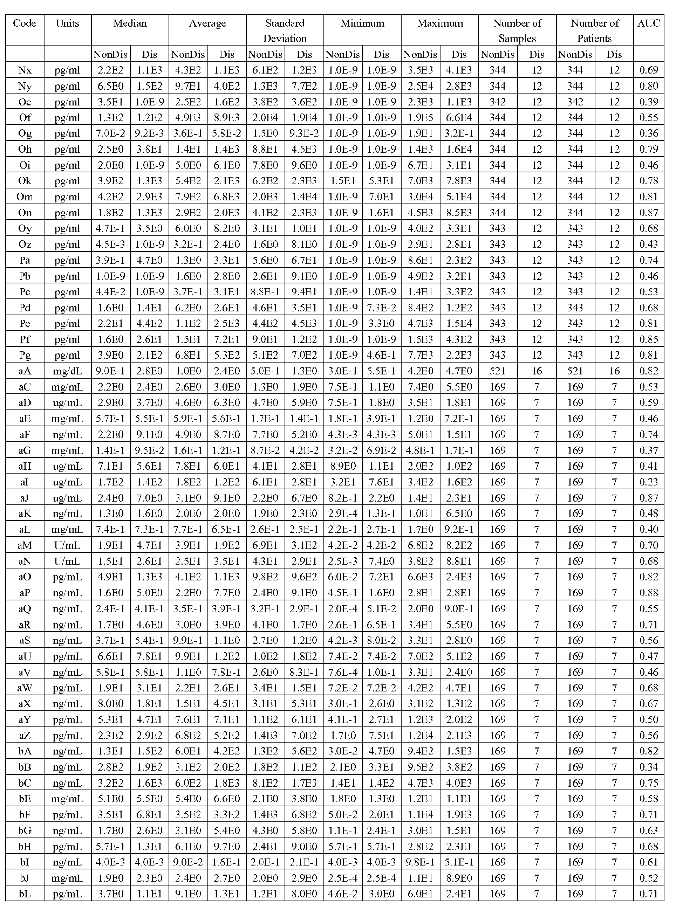

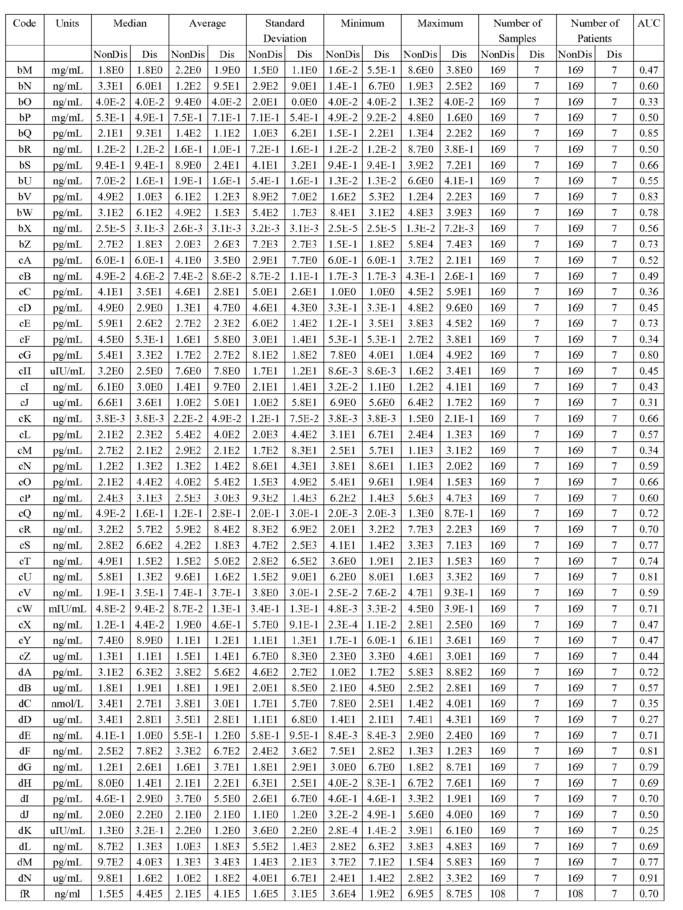

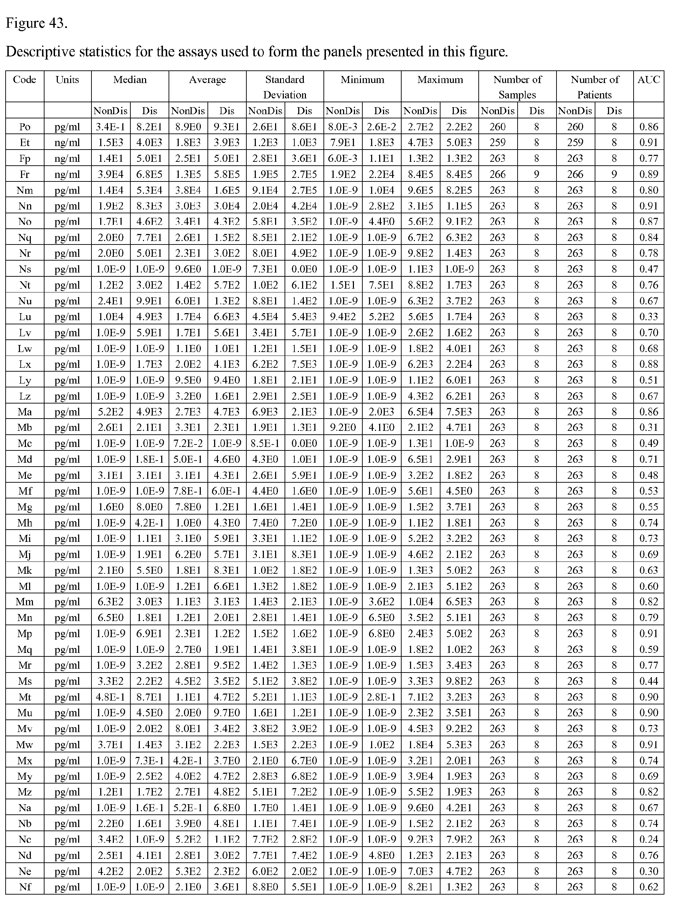

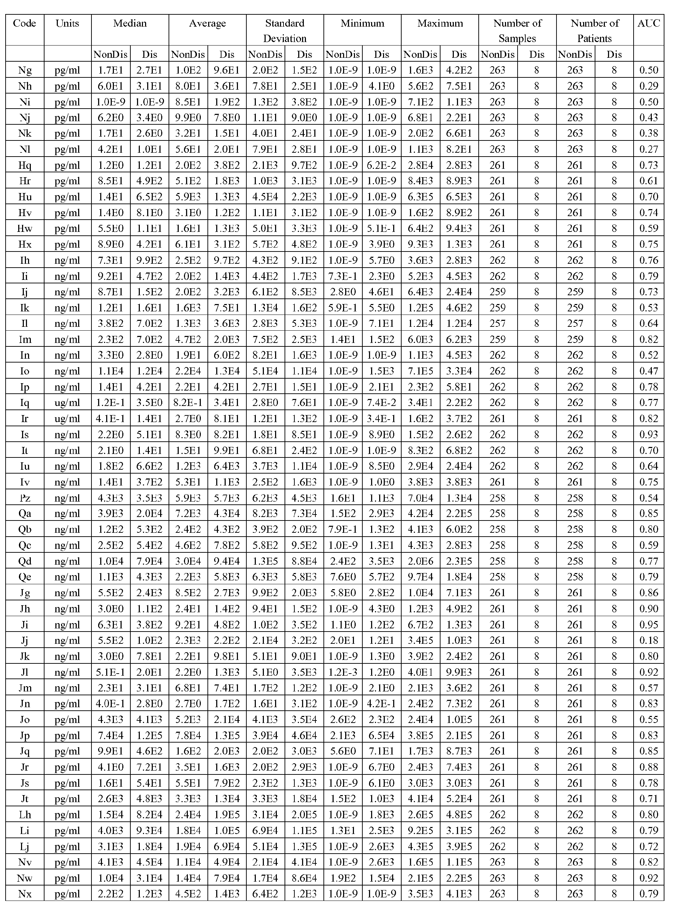

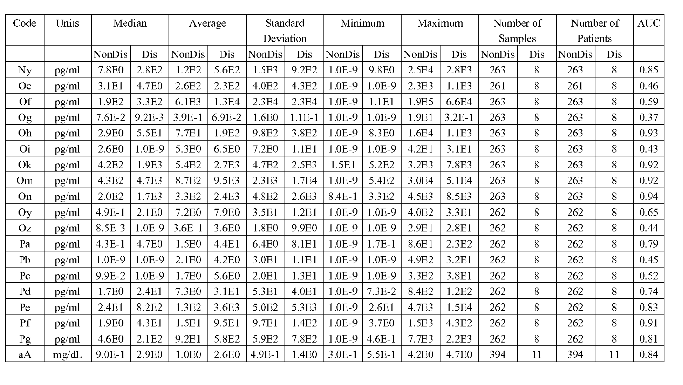

Figure 43. No, R, or I RIFLE stage versus RIFLE F. RIFLE stage adjudicated by Urine Output, within 24hrs of enrollment. Sample is at enrollment.

Example 8. Use of Biomarker Panels

The foregoing examples rely on logistic regression for identification and use of biomarker panels. As noted, this is but one type of analysis which may be used to generate such panels of markers. Any classification method can be used, including, but not limited to, Bayesian classifiers, discriminant analysis, decision trees, neural networks, support-vector machines, nonparametric kernel density estimation methods, nearest neighbor rules, sums, differences, products and ratios of marker concentrations.

As an example, the following exemplary biomarker panels of 2, 3, 4 and 5 markers were generated using products (indicated by "*" in the table) and ratios (indicated by "/" in the table) of marker concentrations. The product of the maker concentrations was used unless the panel consisted of markers that increased and markers that decreased with kidney injury as determined by the univariate performance for the measured patient population. In this case, a ratio was formed in which markers that increased were divided by markers that decreased.

In this example, Cohort 1 consisted of patients that did not progress beyond RIFLE stage R and Cohort 2 consisted of patients that reached stage I or F within 10 days. Panel values were determined for samples from patients in Cohort 1 and samples drawn 24 (+/- 12) hours prior to stage I (or F if no sample at stage I) in Cohort 2. Patients were adjudicated to RIFLE stage R, I, or F based on either serum creatinine or urine output, whichever method yielded the most severe RIFLE stage. A receiver operating characteristic (ROC) curve was generated for each panel and the area under each ROC curve (AUC) was determined. Standard errors were calculated as described in Hanley, J. A., and McNeil, B.J., The meaning and use of the area under a receiver operating characteristic (ROC) curve. Radiology (1982) 143: 29-36; p values were calculated with a two-tailed Z-test.

Examples of 2-, 3-, 4- and 5-marker panels are shown in the following table. The results for the 4 and 5 marker panels demonstrate that larger panels can be formed with the kidney injury markers of Table 2, and that these panels can have improved p-values relative to 2 and 3-marker panels.

Table 4:

| 2-Marker Panels (AUC p-value < 1e-3) | | | | | | |
|---|---|---|---|---|---|---|
| No/(dR) | Nw*iO | Fp/(Jj) | No/(Ch) | Lx*Nw | Et*oZ | Lx/(cM) |
| No*aA | No*iO | Bc/(Jj) | No/(Ck) | No*Nw | Lx*oZ | Nw*cN |
| Et*aA | No/(iQ) | Jl/(Jj) | Gx*No | Ok/(Jj) | Nw*oZ | No*cN |
| No*eF | Nw/(iQ) | Kq/(Jj) | nN/(mX) | Ar*oF | No*oZ | Lx/(cW) |
| Lx*eF | Lj/(iT) | No/(kR) | Im*Lx | Et/(ol) | Nw*pA | No/(cW) |
| Nw*eF | Is/(Bk) | Nw/(kR) | Kq*Lx | No/(ol) | Lj*pA | Nw*cV |
| Ar*eF | Nw*iV | Lh/(Jj) | Ky*Lx | Lx/(ol) | Mz*pA | No*cV |
| Ar/(eC) | Nw/(iL) | Li/(Ji) | Et*Lx | Nw/(ol) | No*pA | Lx*cV |
| Nw/(eC) | Lx/(iL) | Et*Lj | Lx/(Kj) | Nw/(oH) | Ar*pA | No*dC |
| No/(eC) | No/(iL) | Mz/(Jj) | Lx/(Jj) | No/(oH) | Ar*oY | No/(dD) |
| Mz/(eC) | Lj/(iL) | Lx*Mz | Lx/(Ct) | Lx/(oH) | Mz*oY | Lx/(dD) |
| Et/(Bk) | Ar/(iL) | Lx/(Ng) | Lx/(Ch) | Lj/(oH) | Et*oY | Nw*gL |
| Mz*iA | No/(iH) | Nr/(Jj) | Lx/(Gp) | Ar/(oH) | Nw*oY | No*gL |
| Lj*iA | Nw/(iH) | No/(Jj) | Bc*Lx | Oh/(Jj) | No*oY | Mz/(gP) |
| No*iA | No*iK | Jy*No | Lx/(Aj) | Nw*oL | Et*Fp | Et/(gP) |

| 2-Marker Panels (AUC p-value < 1e-3) | | | | | | |
|---|---|---|---|---|---|---|
| No*hB | Et*iK | Im*No | Fp*Lx | No/(pC) | bA*fR | Ji/(gP) |
| Nw*hB | Nw*iK | Et*No | Lx/(Az) | Lx*pF | cT*fR | Nw/(gP) |
| Mz*hB | Et*Im | No/(Kj) | Lx/(Bk) | Oa*pF | cV*fR | Lx/(gP) |
| Lj*hB | No/(il) | Mz*No | Et*Mi | Et*pF | No*aJ | No/(gP) |
| Ar*hB | Nw/(il) | Lj*No | No/(hH) | Lj*pF | Lx*aZ | Ar/(gP) |
| Et*hF | Ar/(il) | Mi*No | No*Oa | Mz*pF | Lx*bA | |
| Nw*hF | Et*iZ | Lw*No | No/(nW) | No*pF | Lj*bA | |
| No*hF | Nw*iX | Lx*No | Lx/(nW) | Nw*pF | By*bA | |
| Nw*hG | Et*jA | No/(Bk) | Nw/(nW) | Ar*pF | Bm*bA | |
| Nw/(iR) | Nw*jA | Aw*No | Et/(nW) | Ar*pF | Ar*bA | |
| No/(iR) | Is/(Jj) | Ay*No | Ar/(nW) | Ar*oZ | Bb*bA | |
| Ar/(iR) | Ij(Jj) | Fb*No | Fp*Nw | By*oZ | Ba/(Aj) | |
| Ar*iP | Im/(Jj) | Fp*No | Et*Nw | Nr*oZ | Lj*bZ | |
| No*iP | Ji/(Jj) | No/(Aj) | Nw/(Jj) | Mz*oZ | No*bZ | |
| Nw*iP | Et/(Jj) | By*No | Im*Nw | Lj*oZ | No/(cK) | |

| 3-Marker Panels (AUC p-value < 1e-7) | | | | |
|---|---|---|---|---|
| Et*Im/(Jj) | No/(il)/(Bk) | Bc/(Jj)/(iR) | No*Nw/(eC) | Et*Lj/(iL) |
| Et*No/(Bk) | Et*No/(eC) | No/(Bk)/(iJ) | Ar*Nw/(iL) | Et*oZ/(Ck) |
| Lu*Im/(Jj) | Ar*Nw*oZ | Mz*Nw*pF | Jy*Nw*iK | No*oZ/(Jj) |
| Et*Nw/(Bk) | No/(gP)/(Kj) | Is/(Bk)/(oH) | No/(dR)/(Ch) | oZ*Nw*pA |
| No*eF/(Bk) | No/(eC)/(cW) | No/(nW)/(Ck) | Lj/(Jj)/(iR) | Fp*Nw*oZ |
| Im*Nw/(Jj) | Nw/(gP)/(Jj) | No/(oH)/(Ch) | Lj*Nw*pF | Ar*Nw/(gP) |
| No*cV/(Bk) | Nw/(gP)/(Ck) | oZ*No*pF | No/(iR)/(eC) | iK*No/(iL) |
| Et*Nw/(gP) | No/(oH)/(Ck) | Nw*iK/(gP) | Nw*iK/(Ck) | Et*No/(iR) |
| Et*No/(Jj ) | Mz*pF/(Jj) | Mz*pF/(Bk) | Nw*iK/(Jj) | Ar*pA/(Ch) |
| Et*Is/(Bk) | Nw/(nW)/(Ck) | Bc/(Jj)/(nW) | Ar*Nw/(oH) | Bc/(Jj)/(iQ) |
| Lj*bZ/(Jj) | No/(gP)/(Ck) | No/(nW)/(Bk) | Lu*Mz*pF | Nw/(iQ)/(Ck) |
| No/(gP)/(Bk) | Lj*oZ/(Jj) | Lj/(oH)/(Jj) | No*gL/(Bk) | No*oZ/(Bk) |
| Et*Im/(Bk) | Et*Ar/(eC) | Et/(gP)/(Jj) | No*pF/(Bk) | Ar*Et*oZ |
| Im*No/(Jj) | Et*No/(nW) | No/(eC)/(Ch) | Lj/(iL)/(Jj) | No/(nW)/(gP) |
| Et*No/(gP) | oZ*Bc/(Jj) | Jy*Nw/(gP) | Et*iK/(Jj) | Ar*oZ/(Jj) |
| Et*Fp/(Jj) | eF*Lj/(Jj) | dC*Nw/(gP) | No/(nW)/(Jj) | No/(Bk)/(iS) |
| No/(cW)/(Bk) | No*pF/(Ch) | No/(pC)/(eC) | Lj/(iT)/(Jj) | Ar*Et/(nW) |
| No/(gP)/(Jj) | No/(gP)/(cK) | No/(iL)/(Bk) | No/(gP)/(Ch) | No/(nW)/(Ch) |
| Pj*Im/(Jj) | No*eF/(Jj) | Et*Nw*hF | Nw/(iR)/(eC) | Et/(nW)/(Ck) |
| Im*Ok/(Jj) | aA*Nw/(eC) | No/(iQ)/(Bk) | Nw*pF/(Ch) | No/(il)/(Ch) |
| No/(iR)/(Bk) | Nw/(gP)/(aW) | No/(eC)/(Jj) | Et*Lj*oZ | No/(gP)/(eC) |
| No*Nw/(Bk) | No*iK/(Jj) | Lj*Nw*oZ | Lu*No/(nW) | Nw*pA/(Ch) |

(continued)

| 3-Marker Panels (AUC p-value < 1e-7) | | | | |
|---|---|---|---|---|
| Nw*oZ/(Ck) | Lj/(Jj)/(gP) | iK*Nw/(iL) | Et*Lj*pF | Ar*Nw/(iR) |
| Et*Nw*iK | Et*Nw*iO | Im/(Jj)/(gP) | Lj/(Jj)/(nW) | Ar*Nw/(nW) |
| Ij*Et/(Bk) | aJ*Nw/(gP) | No*oZ/(nW) | Mz*pF/(Ch) | No*pA/(Bk) |
| No/(oH)/(Bk) | No/(nW)/(eC) | Ar*Nw/(eC) | Nw/(oH)/(Ck) | No/(iR)/(Ck) |
| No*eF/(Ch) | No*hB/(Bk) | Mz*No/(gP) | No*Nw/(nW) | Ar/(oH)/(Jj) |
| No*Nw/(gP) | Lx/(gP)/(Ch) | Mz*No*pF | No*oZ/(Ck) | Nw/(gP)/(eC) |
| Bc/(Jj)/(oH) | Nw/(gP)/(dD) | Lw*Nw/(gP) | No/(iR)/(Jj) | iZ*Nw/(eC) |
| Fy*Et/(Bk) | fS*Lx/(Ch) | Et*No*iK | Et*Nw*pF | |

| 4-Marker Panels (AUC p-value < 1e-8) | | | | |
|---|---|---|---|---|
| Nw*Im/Jj/Kg | Oa*Et*Im/Jj | Ar*Et*Im/Jj | Mb*Nw*Im/Jj | Et*Nw*Ji*Fp |
| Nw*Im/Jj/Kj | Fb*Nw*Im/Jj | Pj*Et*Im/Jj | Nt*Et*Im/Jj | Fp*Nw*Im/Jj |
| Ap*Nw*Im/Jj | Hb*Et*Im/Jj | Ke*Et*Im/Jj | Is*Et*Im/Jj | Ji*Et*Im/Jj |
| Bc*Nw*Im/Jj | Bb*Et*Im/Jj | Jy*Et*Im/Jj | Nw*Ji*Fp/Hw | Mb*Ji*Im/Jj |
| Kp*Et*Im/Jj | Cu*Et*Im/Jj | Fb*Et*Im/Jj | Mb*Et*Im/Jj | Ji*Im/Jj/Mc |
| Jy*Nw*Im/Jj | Bc*Et*Im/Jj | Aw*Et*Im/Jj | Et*Im/Jj/Mc | Fp*Et*Im/Jj |
| Cp*Nw*Im/Jj | Et*Im/Jj/Kg | Nt*Nw*Im/Jj | Ok*Et*Im/Jj | Et*Ji*Fp/Jj |
| Fy*Et*Im/Jj | Ar*Ji*Im/Jj | Nw*Im/Jj/Me | Et*Nw*Im/Jj | Fp*Ji*Im/Jj |
| Pj*Nw*Im/Jj | Aw*Nw*Im/Jj | Is*Nw*Im/Jj | Nw*Ji*Fp/Mc | Et*Ji*Fp/Lu |
| Kq*Nw*Im/Jj | Et*Im/Jj/Kj | Qe*Nw*Im/Jj | Ji*Im/Jj/Lu | Et*Ji*Fp/Mc |
| Ke*Nw*Im/Jj | Aw*Ji*Im/Jj | Nw*Im/Jj/Mc | Ji*Nw*Im/Jj | Nw*Ji*Fp/Jj |
| Ar*Nw*Im/Jj | Kq*Et*Im/Jj | Ok*Nw*Im/Jj | Ok*Ji*Im/Jj | Nw*Ji*Fp/Lu |
| Kk*Et*Im/Jj | Cp*Et*Im/Jj | Et*Aw*Qe/Jj | Pj*Is/Jj/Kg | Et*Ke*Is/Jj |
| Et*Is*Ji/Jj | Et*Ji*Nw/Lu | Et*Aw*Is/Jj | Nt*Is*Mt/Ct | Ny*Nm*Mt/Ct |
| Et*Is*Fp/Jj | Et*Jp*Fp/Jj | Et*Is/Kj/Jj | Nm*Pf*Mt/Ct | Nm*Mt/Ct/Jj |
| Et*Ji*Qe/Jj | Et*Qe*Fp/Jj | Im*Nm*Mt/Ct | Qe*Cp*Et/Jj | Nw*Et*Is/Kg |
| Mb*Is*Ji/Jj | Et*Fp/Mc/Jj | Mm*Is/Jj/Kg | Jy*Et*Qa/Jj | Ji*Is/Jj/Kg |
| Is*Ji*Fp/Jj | Im*Ok*Fp/Jj | Ar*Et*Is/Jj | Is*Nm*Mt/Ct | Nm*Mt/Ct/Ch |
| Et*Nw*Fp/Jj | Ji*Jp*Fp/Jj | Qa*Et*Aw/Jj | Nt*Pf*Mt/Ct | Is*Ok/Jj/Kg |
| Et*Ok*Fp/Jj | Ji*Qe*Fp/Jj | Jy*Et*Qe/Jj | Nb*Pf*Mt/Ct | Mz*Nm*Mt/Ct |
| Et*On*Fp/Jj | Ji*On*Fp/Jj | Is*Im*Mt/Ct | Is*Pf*Mt/Ct | Ke*Is/Jj/Kg |
| Ji*Ok*Fp/Jj | Et*Nw*Fp/Mc | Nb*Is*Mt/Ct | Ji*Et*Aw/Jj | Ke*Et*Fp/Jj |
| Et*Nw*Fp/Lu | Ok*Fp/Jj/Mc | Et*Is*Mt/Ct | Im*Pf*Mt/Ct | Im*Is/Jj/Kg |
| Nw*Ok*Fp/Jj | Et*Is/Jj/Kg | Et*Im*Ji/Aj | Nt*Im*Mt/Ct | Is*Nw/Jj/Kg |

| 5-Marker Panels (AUC p-value < 1e-9) | | | |
|---|---|---|---|
| Mz*Nw*Im/Jj/Kg | Lh*Et*Im/Jj/Kg | Nt*Fp*Nw*Im/Jj | Jp*Fp*Nw*Im/Jj |
| Kq*Nw*Im/Jj/Kg | Ip*Nw*Im/Jj/Kg | Mb*Ok*Nw*Ji*Fp | Mz*Fp*Nw*Im/Jj |
| Qa*Nw*Im/Jj/Kg | Dg*Ji*Im/Jj/Kj | Et*Is*Nw*Ji*Fp | Nt*Nw*Ji*Fp/Jj |
| Ma*Nw*Ji*Fp/Aj | Et*Cp*Ji*Im/Jj | On*Nw*Ji*Fp/Hw | Jt*Nw*Ji*Fp/Jj |

(continued)

| 5-Marker Panels (AUC p-value < 1e-9) | | | |
|---|---|---|---|
| Ok*Nw*Im/Jj/Kg | Nm*Et*Im/Jj/Aj | Et*Mi*Nw*Ji*Fp | Mi*Nw*Ji*Fp/Jj |
| Ji*Nw*Im/Jj/Kg | Et*Cp*Nw*Im/Jj | Qe*Fp*Nw*Im/Jj | Pz*Nw*Ji*Fp/Jj |
| Ke*Aw*Nw*Im/Jj | Et*Jy*Ji*Im/Jj | Fp*Nw*Im/Jj/Hw | Jp*Nw*Ji*Fp/Lu |
| Ip*Nw*Im/Jj/Kj | Ad*Nw*Im/Jj/Kj | Is*Fp*Nw*Im/Jj | Mb*Nw*Ji*Fp/Lu |
| Bc*Nw*Im/Jj/Kj | Et*Ar*Nw*Im/Jj | Im*Nw*Ji*Fp/Mc | Mz*Et*Ji*Fp/Hw |
| Ef*Nw*Im/Jj/Aj | Kl*Ji*Im/Jj/Aj | Lw*Nw*Ji*Fp/Jj | Is*Et*Ji*Fp/Hw |
| Ok*Nw*Im/Jj/Kj | Jo*Et*Im/Jj/Kg | Jo*Nw*Ji*Fp/Jj | Ok*Et*Ji*Fp/Hw |
| Ap*Et*Ji*Fp/Jj | Ad*Nw*Im/Jj/Aj | Js*Nw*Ji*Fp/Jj | Et*Is*Ji*Im/Jj |
| Qe*Nw*Im/Jj/Kj | Kq*Et*Im/Jj/Kj | Jp*Nw*Ji*Fp/Mc | Is*Ji*Im/Jj/Hw |
| Nt*Aw*Nw*Im/Jj | Dl*Et*Im/Jj/Aj | Qe*Nw*Ji*Fp/Lu | Is*Et*Ji*Fp/Jj |
| Fb*Aw*Nw*Im/Jj | Bc*Et*Im/Jj/Kj | Lw*Nw*Ji*Fp/Lu | Et*Fp*Nw*Im/Jj |
| Pj*Nw*Im/Jj/Kj | Ok*Et*Im/Jj/Kg | Mb*Nw*Ji*Fp/Mc | Et*Ji*Nw*Im/Jj |
| Ip*Nw*Im/Jj/Aj | Qe*Et*Im/Jj/Kj | Et*Nt*Nw*Im/Jj | Et*Nw*Im/Jj/Lu |
| Qe*Nw*Im/Jj/Kg | Ke*Et*Im/Jj/Ob | Et*Ok*Nw*Ji*Fp | Et*Nw*Im/Jj/Mc |
| Jg*Nw*Im/Jj/Aj | Kq*Aw*Et*Im/Jj | Jp*Nw*Ji*Fp/Hw | Et*Mb*Nw*Im/Jj |
| Kq*Aw*Nw*Im/Jj | Ke*Aw*Et*Im/Jj | Et*Jp*Nw*Ji*Fp | Ji*Et*Im/Jj/Lu |
| Et*Nw*Im/Jj/Aj | Mm*Et*Im/Jj/Kj | Nw*Ji*Fp/Nh/Jj | Ji*Fp*Et*Im/Jj |
| Ad*Et*Ji*Fp/Aj | Mm*Et*Im/Jj/Kg | Ma*Nw*Ji*Fp/Jj | Ji*Et*Im/Jj/Mc |
| Ad*Et*Ji*Fp/Jj | Ke*Et*Im/Jj/Kg | Lh*Nw*Ji*Fp/Jj | Ji*Mb*Et*Im/Jj |
| Jg*Et*Ji*Fp/Aj | Kq*Et*Im/Jj/Aj | Mz*Nt*Nw*Ji*Fp | Ok*Fp*Et*Im/Jj |
| Et*Kk*Ji*Im/Jj | Ad*Ji*Im/Jj/Aj | Ik*Ji*Nw*Im/Jj | Fp*Et*Im/Jj/Mc |
| Pj*Ji*Im/Jj/Kg | Jp*Aw*Et*Im/Jj | Nt*Et*Ji*Fp/Lu | Et*Nt*Ji*Im/Jj |
| Is*Ji*Im/Jj/Kg | Ik*Ji*Im/Jj/Aj | Nt*Nw*Et*Ji*Fp | Fp*Ji*Im/Jj/Hw |
| Ok*Aw*Nw*Im/Jj | Kq*Et*Im/Jj/Ch | Mi*Nw*Ji*Fp/Hw | Et*Ok*Ji*Im/Jj |
| Pf*Nw*Im/Jj/Ct | Dg*Ji*Im/Jj/Aj | Nw*Ji*Fp/Me/Hw | Et*Jp*Ji*Im/Jj |
| Mb*Aw*Nw*Im/Jj | Ef*Et*Im/Jj/Aj | Nw*Ji*Fp/Mc/Hw | Et*Qe*Ji*Im/Jj |
| Dg*Et*Ji*Fp/Aj | Ar*Et*Im/Jj/Mc | Qe*Nw*Ji*Fp/Hw | Et*Mi*Ji*Im/Jj |
| Aw*Nw*Im/Jj/Ob | Nt*Aw*Et*Im/Jj | Qa*Nw*Ji*Fp/Hw | Im*Et*Ji*Fp/Lu |
| Jp*Aw*Nw*Im/Jj | Et*Nw*Im/Jj/Kj | Mb*Fp*Nw*Im/Jj | Jg*Et*Ji*Fp/Jj |
| Et*Cu*Nw*Im/Jj | Qa*Et*Im/Jj/Kg | Nw*Ji*Fp/Me/Jj | Ji*Fp*Nw*Im/Jj |
| Et*Oa*Nw*Im/Jj | Pj*Et*Im/Jj/Kg | Om*Nw*Ji*Fp/Jj | Ji*Nw*Im/Jj/Lu |
| Bb*Et*Ji*Fp/Jj | Pj*Et*Im/Jj/Mc | Mi*Nw*Ji*Fp/Lu | Ji*Mb*Nw*Im/Jj |
| Pj*Nw*Im/Jj/Kg | Ke*Et*Im/Jj/Mc | Ok*Nw*Ji*Fp/Mc | Ji*Nw*Im/Jj/Mc |
| Mt*Nw*Im/Jj/Ct | Lw*Fb*Et*Im/Jj | Qe*Et*Ji*Fp/Hw | Fp*Ji*Im/Jj/Mc |
| Nw*Im/Jj/Ob/Mc | Pj*Et*Im/Jj/Kj | Qa*Et*Ji*Fp/Hw | Ok*Fp*Ji*Im/Jj |
| Et*Kk*Nw*Im/Jj | Ik*Nw*Im/Jj/Aj | Et*Is*Nw*Im/Jj | Fp*Ji*Im/Jj/Lu |
| Nm*Ji*Im/Jj/Aj | Ji*Et*Im/Jj/Kj | Ik*Et*Ji*Fp/Jj | Ok*Ji*Im/Jj/Lu |
| Ji*Ar*Nw*Im/Jj | Pj*Aw*Et*Im/Jj | Ik*Nw*Ji*Fp/Jj | Mb*Ji*Im/Jj/Mc |

(continued)

| 5-Marker Panels (AUC p-value < 1e-9) | | | |
|---|---|---|---|
| Fb*Aw*Ji*Im/Jj | Mi*Aw*Et*Im/Jj | Ji*Qe*Nw*Im/Jj | Jp*Fp*Ji*Im/Jj |
| Pf*Et*Ji*Fp/Ct | Et*Nw*Im/Jj/Kg | Jg*Nw*Ji*Fp/Jj | Mb*Ok*Ji*Im/Jj |
| Nt*Aw*Ji*Im/Jj | Jy*Et*Im/Jj/Mc | Mb*Ok*Nw*Im/Jj | Ok*Ji*Im/Jj/Mc |
| Ip*Ji*Im/Jj/Aj | Mi*Et*Im/Jj/Ob | Qd*Nw*Ji*Fp/Jj | Mb*Ji*Im/Jj/Lu |
| Et*Ji*Im/Jj/Ch | Qe*Et*Im/Jj/Kg | Mz*Nw*Ji*Fp/Jj | Mz*Fp*Ji*Im/Jj |
| Jg*Ji*Im/Jj/Aj | Fb*Pj*Et*Im/Jj | Mz*Nw*Ji*Fp/Lu | Ji*Im/Jj/Mc/Lu |
| Mt*Ji*Im/Jj/Ct | Ji*Ar*Et*Im/Jj | Nw*Et*Ji*Fp/Hw | On*Fp*Ji*Im/Jj |
| Et*Cu*Ji*Im/Jj | Mb*Et*Im/Jj/Ob | Et*Ji*Fp/Lu/Hw | Mb*Fp*Ji*Im/Jj |
| Et*Bb*Nw*Im/Jj | Et*Fb*Nw*Im/Jj | Nt*Et*Ji*Fp/Hw | Et*Ji*Fp/Mc/Jj |
| Et*Bc*Nw*Im/Jj | Jg*Et*Im/Jj/Aj | Et*Ji*Fp/Jj/Hw | Et*Ji*Fp/Lu/Jj |
| Qe*Aw*Ji*Im/Jj | Ji*Et*Im/Jj/Kg | Mi*Et*Ji*Fp/Hw | Ok*Et*Ji*Fp/Jj |
| Mm*Ji*Im/Jj/Kg | Qe*Aw*Et*Im/Jj | Im*Et*Ji*Fp/Hw | Nw*Et*Ji*Fp/Jj |
| Bc*Ji*Im/Jj/Aj | Ip*Ji*Im/Jj/Kg | Is*Nw*Im/Jj/Hw | Nw*Et*Ji*Fp/Lu |
| Mm*Nw*Im/Jj/Kj | Ji*Fb*Et*Im/Jj | Is*Nw*Ji*Fp/Hw | Et*Ji*Fp/Mc/Lu |
| Ma*Et*Ji*Fp/Aj | Bc*Et*Im/Jj/Aj | Ik*Mz*Nw*Im/Jj | Jp*Et*Ji*Fp/Jj |
| Qe*Aw*Nw*Im/Jj | Ji*Et*Im/Jj/Ob | Ik*Mz*Ji*Im/Jj | Mb*Et*Ji*Fp/Jj |
| Dg*Nw*Im/Jj/Kj | Is*Et*Im/Jj/Kj | Et*Ji*Im/Jj/Me | Qe*Et*Ji*Fp/Jj |
| Aw*Nw*Im/Jj/Mc | Lw*Aw*Et*Im/Jj | Im*Nw*Ji*Fp/Hw | Nt*Et*Ji*Fp/Jj |
| Ar*Ji*Im/Jj/Mc | Dg*Et*Im/Jj/Aj | Ji*Nt*Nw*Im/Jj | Qa*Et*Ji*Fp/Jj |
| Et*Ke*Nw*Im/Jj | Nm*Et*Im/Jj/Kj | Ji*Ok*Nw*Im/Jj | Ok*Et*Ji*Fp/Lu |
| Is*Nw*Im/Jj/Ct | Fb*Aw*Et*Im/Jj | Ji*Nw*Im/Jj/Me | Jt*Et*Ji*Fp/Jj |
| Et*Pj*Nw*Im/Jj | Is*Et*Im/Jj/Kg | Nt*Fp*Ji*Im/Jj | On*Et*Ji*Fp/Jj |
| Ji*Aw*Nw*Im/Jj | Et*Nw*Im/Jj/Ob | Mz*Nt*Ji*Im/Jj | Mi*Et*Ji*Fp/Jj |
| Dg*Nw*Im/Jj/Aj | Mb*Aw*Et*Im/Jj | Nt*Ji*Im/Jj/Mc | Jp*Et*Ji*Fp/Lu |
| Mm*Nw*Im/Jj/Kg | Et*Aw*Nw*Im/Jj | Nt*Ji*Im/Jj/Lu | Qd*Et*Ji*Fp/Jj |
| Mb*Aw*Ji*Im/Jj | Fb*Et*Im/Jj/Ob | Nt*Ok*Ji*Im/Jj | Mz*Et*Ji*Fp/Jj |
| Et*Ke*Ji*Im/Jj | Lw*Et*Im/Jj/Ob | Qe*Fp*Ji*Im/Jj | Nw*Et*Ji*Fp/Mc |
| Kl*Nw*Im/Jj/Aj | Kl*Et*Im/Jj/Aj | Mi*Fp*Ji*Im/Jj | Mz*Et*Ji*Fp/Lu |
| Is*Ji*Im/Jj/Ct | Ji*Aw*Et*Im/Jj | Jp*Mb*Ji*Im/Jj | Mb*Et*Ji*Fp/Lu |
| Et*Pj*Ji*Im/Jj | Fb*Et*Im/Jj/Mc | Qe*Ji*Im/Jj/Mc | Lw*Et*Ji*Fp/Jj |
| Et*Kq*Ji*Im/Jj | Ip*Et*Im/Jj/Kg | Ok*Ji*Im/Jj/Me | Js*Et*Ji*Fp/Jj |
| Is*Nw*Im/Jj/Kj | Ad*Et*Im/Jj/Kj | Et*J*Fp/Nh/Jj | Is*Nw*Ji*Fp/Jj |
| Aw*Et*Ji*Fp/Jj | Aw*Et*Im/Jj/Ob | Mz*Nt*Et*Ji*Fp | Nw*Ji*Fp/Lu/Hw |
| Nm*Ji*Im/Jj/Kj | Aw*Et*Im/Jj/Mc | Pz*Et*Ji*Fp/Jj | Ok*Nw*Ji*Fp/Hw |
| Et*Bb*Ji*Im/Jj | Ad*Et*Im/Jj/Aj | Mi*Et*Ji*Fp/Lu | Mz*Nw*Ji*Fp/Hw |
| Et*Kq*Nw*Im/Jj | Ik*Et*Im/Jj/Aj | Om*Et*Ji*Fp/Jj | Ok*Fp*Nw*Im/Jj |
| Ad*Ji*Im/Jj/Kj | Et*Im/Jj/Ob/Mc | Mb*Et*Ji*Fp/Mc | Fp*Nw*Im/Jj/Mc |
| Is*Nw*Im/ij/Kg | No*Nw*Ji*Fp/Hw | Lh*Et*Ji*Fp/Jj | Ok*Nw*Ji*Fp/Jj |

(continued)

| 5-Marker Panels (AUC p-value < 1e-9) | | | |
|---|---|---|---|
| Aw*Ji*Im/Jj/Mc | Et*Nw*Ji*Fp/Nh | Mb*Nw*Et*Ji*Fp | Nw*Ji*Fp/Lu/Jj |
| Nm*Nw*Im/Jj/Kj | Nw*Ji*Fp/Nh/Hw | Lw*Et*Ji*Fp/Lu | Nw*Ji*Fp/Mc/Jj |
| Et*Ji*Im/Jj/Aj | Nt*Nw*Ji*Fp/Mc | Jp*Et*Ji*Fp/Mc | Qe*Nw*Ji*Fp/Jj |
| Ke*Nt*Et*Im/Jj | Nt*Ok*Nw*Ji*Fp | Jo*Et*Ji*Fp/Jj | Jp*Nw*Ji*Fp/Jj |
| Kk*Pj*Et*Im/Jj | Et*Qe*Nw*Ji*Fp | Nt*Nw*Ji*Fp/Hw | Mb*Nw*Ji*Fp/Jj |
| Pf*Et*Im/Jj/Kg | Jm*Nw*Ji*Fp/Jj | Im*Nw*Ji*Fp/Lu | On*Nw*Ji*Fp/Jj |
| Qd*Et*Im/Jj/Kg | Nt*Nw*Ji*Fp/Lu | Nw*Ji*Fp/Jj/Hw | Qa*Nw*Ji*Fp/Jj |
| Dg*Et*Im/Jj/Kj | Om*Nw*Ji*Fp/Hw | Fp*Nw*Im/Jj/Lu | Nw*Ji*Fp/Mc/Lu |
| Et*Jy*Nw*Im/Jj | Is*Nw*Ji*Fp/Lu | On*Fp*Nw*Im/Jj | Ok*Nw*Ji*Fp/Lu |
| Kl*Et*Im*Ji/Aj | Et*Ke*Is/Jj/Kg | Pf*Nm*Mt/Ct/Ch | Mz*Nm*Pf*Mt/Ct |
| Et*Pj*Is/Jj/Kg | Et*Ji*Is/Jj/Kg | Im*Nm*Pf*Mt/Ct | Mz*Nt*Is*Mt/Ct |
| Dg*Et*Im*Ji/Aj | Mz*Nm*Mt/Ct/Jj | Ji*Im*Nm*Mt/Ct | Et*Mm*Is/Jj/Kg |
| Ad*Et*Im*Ji/Aj | Pj*Et*Is/Kj/Jj | Et*Is*Ok/Jj/Kg | Nm*Pf*Mt/Ct/Aj |
| Jg*Et*Im*Ji/Aj | Dg*Et*Is/Kj/Jj | Nb*Im*Nm*Mt/Ct | Is*Nm*mt/Ct/Ch |
| Im*Nm*Mt/Ct/Jj | Nb*Nm*Mt/Ct/Ch | Et*Nt*Im*Mt/Ct | Is*Mz*Nm*Mt/Ct |
| Im*Mz*Nm*Mt/Ct | Ny*Nm*Mt/Ct/Hw | Hu*Nm*Mt/Ct/Ch | Di*Im*Nm*Mt/Ct |
| Et*Im*Nm*Mt/Ct | Kq*Et*Is/Jj/Kg | Is*Nm*Mt/Ct/Aj | Et*Is*Nm*Mt/Ct |
| Im*Nm*mt/Ct/Ch | Kq*Et*Im*Ji/Aj | Ad*Et*Is/Kj/Jj | Pf*Et*Is/Jj/Kg |
| Nm*Pf*Mt/Ct/Oy | Et*Nt*Is*Mt/Ct | Im*Nm*Mt/Ct/Aj | Ip*Et*Is/Jj/Kg |
| Et*Is/Jj/Kg/Mc | Is*Nm*Mt/Ct/Jj | Mz*Nt*Im*Mt/Ct | Jp*Et*Is/Jj/Kg |
| Is*Nt*Im*Mt/Ct | Nb*Nt*Is*Mt/Ct | Nw*Im*Nm*Mt/Ct | Et*Aw*Is/Jj/Kj |
| Is*Im*Nm*Mt/Ct | Et*Is*Nw/Jj/Kg | Ba*Nm*Mt/Ct/Ch | Jy*Et*Is/Jj/Kg |

While the invention has been described and exemplified in sufficient detail for those skilled in this art to make and use it, various alternatives, modifications, and improvements should be apparent without departing from the spirit and scope of the invention. The examples provided herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims

**Claims**

1. A method for evaluating renal status in a subject, comprising:

 performing a plurality of assays configured to detect a plurality of kidney injury markers selected from the group consisting of WAP four-disulfide core domain protein 2, Angiopoietin-related protein 4, C-C motif chemokine 27, C-X-C motif chemokine 13 , Fibroblast growth factor 21, Follistatin, Insulin-like growth factor-binding protein 4, Protransforming growth factor alpha, Tenascin, and Tissue-type plasminogen activator on a body fluid sample obtained from the subject to provide one or more assay results; and
 correlating the assay results to the renal status of the subject, wherein said correlating step comprises assigning a likelihood of one or more future changes in renal status to the subject based on the assay results, wherein said one or more future changes in renal status comprise future acute renal failure (ARF).

2. A method according to claim 1, wherein said correlation step comprises correlating the assay results to one or more of risk stratification, diagnosis, staging, prognosis, classifying and monitoring of the renal status of the subject.

3. A method according to one of claims 1-2, wherein said assay results comprise a measured concentration of at least 3, 4, or 5 kidney injury markers selected from the group consisting of WAP four-disulfide core domain protein 2, Angiopoietin-related protein 4, C-C motif chemokine 27, C-X-C motif chemokine 13, Fibroblast growth factor 21, Follistatin, Insulin-like growth factor-binding protein 4, Protransforming growth factor alpha, Tenascin, and Tissue-type plasminogen activator.

4. A method according to one of claims 1-3, wherein said assay results are combined using a function that converts said assay results into a single composite result.

5. A method according to claim 3, wherein the likelihood of future ARF is more or less likely to occur within 30 days of the time at which the body fluid sample is obtained from the subject, or wherein the likelihood of future ARF is more or less likely to occur within a period selected from the group consisting of 30 days 21 days, 14 days, 7 days, 5 days, 96 hours, 72 hours, 48 hours, 36 hours, 24 hours, and 12 hours.

6. A method according to one of claims 1-5, wherein the subject is selected for evaluation of renal status based on the pre-existence in the subject of one or more known risk factors for prerenal, intrinsic renal, or postrenal ARF.

7. A method according to one of claims 1-5, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

8. A method according to one of claims 1-5, wherein said one or more future changes in renal status comprise one or more of a future injury to renal function, future reduced renal function, future improvement in renal function, and future acute renal failure (ARF) within 24 hours of the time at which the body fluid sample is obtained.

9. A method according to one of claims 1-5, wherein the subject is in RIFLE stage 0 or R.

10. A method according to claim 9, wherein the subject is in RIFLE stage 0, and said correlating step comprises assigning a likelihood that the subject will reach RIFLE stage R, I or F within 72, 48 or 24 hours.

11. A method according to claim 10, wherein the subject is in RIFLE stage 0, and said correlating step comprises assigning a likelihood that the subject will reach RIFLE stage I or F within 72, 48 or 24 hours.

12. A method according to claim 10, wherein the subject is in RIFLE stage 0, and said correlating step comprises assigning a likelihood that the subject will reach RIFLE stage F within 72, 48 or 24 hours.

13. A method according to claim 9, wherein the subject is in RIFLE stage 0 or R, and said correlating step comprises assigning a likelihood that the subject will reach RIFLE stage I or F within 72, 48 or 24 hours.

**14.** A kit, comprising:

reagents for performing a plurality of assays configured to detect a plurality of kidney injury markers selected from the group consisting of WAP four-disulfide core domain protein 2, Angiopoietin-related protein 4, C-C motif chemokine 27, C-X-C motif chemokine 13, Fibroblast growth factor 21, Follistatin, Insulin-like growth factor-binding protein 4, Protransforming growth factor alpha, Tenascin, and Tissue-type plasminogen activator.

**15.** Use of two or more kidney injury markers selected from the group consisting of WAP four-disulfide core domain protein 2, Angiopoietin-related protein 4, C-C motif chemokine 27, C-X-C motif chemokine 13, Fibroblast growth factor 21, Follistatin, Insulin-like growth factor-binding protein 4, Protransforming growth factor alpha, Tenascin, and Tissue-type plasminogen activator for the prognosis of future acute kidney failure.

Figure 1.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 6.6E1 | 8.2E1 | 7.7E1 | 9.3E1 | 5.2E1 | 6.0E1 | 2.0E0 | 1.3E1 | 4.4E2 | 2.4E2 | 1475 | 37 | 252 | 37 | 0.58 |
| Ad | ug/mL | 3.4E-2 | 7.6E-2 | 6.8E-2 | 3.6E-1 | 8.7E-2 | 1.5E0 | 6.8E-4 | 4.3E-3 | 5.4E-1 | 8.5E0 | 421 | 33 | 165 | 33 | 0.65 |
| Af | ng/mL | 1.1E0 | 1.0E0 | 1.6E1 | 1.4E1 | 6.2E1 | 4.5E1 | 1.7E-3 | 1.7E-3 | 5.3E2 | 2.6E2 | 421 | 33 | 165 | 33 | 0.54 |
| Aj | ug/mL | 1.8E0 | 5.4E-1 | 2.7E0 | 2.1E0 | 2.5E0 | 2.6E0 | 1.5E-3 | 2.1E-3 | 6.1E0 | 6.1E0 | 421 | 33 | 165 | 33 | 0.43 |
| Al | mg/mL | 8.7E-5 | 9.5E-5 | 2.5E-4 | 2.6E-4 | 4.1E-4 | 3.4E-4 | 2.5E-6 | 7.6E-6 | 2.2E-3 | 1.3E-3 | 421 | 33 | 165 | 33 | 0.56 |
| An | U/mL | 4.9E1 | 8.8E1 | 1.6E2 | 5.1E2 | 4.5E2 | 1.4E3 | 9.8E-4 | 9.6E-1 | 5.5E3 | 7.8E3 | 421 | 33 | 165 | 33 | 0.63 |
| Ao | pg/mL | 8.6E1 | 1.1E2 | 5.4E2 | 3.2E2 | 3.6E3 | 7.9E2 | 1.5E0 | 5.4E0 | 3.9E4 | 4.5E3 | 421 | 33 | 165 | 33 | 0.58 |
| Ap | ng/mL | 2.9E1 | 4.5E1 | 4.5E1 | 5.8E1 | 5.0E1 | 5.3E1 | 8.4E-5 | 3.1E0 | 3.3E2 | 2.4E2 | 421 | 33 | 165 | 33 | 0.60 |
| Ar | ng/mL | 8.5E-1 | 2.6E0 | 1.3E1 | 5.8E0 | 2.0E2 | 9.6E0 | 3.4E-3 | 9.5E-2 | 4.1E3 | 5.1E1 | 421 | 33 | 165 | 33 | 0.68 |
| As | ng/mL | 8.7E-3 | 1.0E-2 | 1.3E-2 | 5.4E-2 | 1.7E-2 | 2.1E-1 | 1.7E-3 | 1.7E-3 | 1.3E-1 | 1.2E0 | 421 | 33 | 165 | 33 | 0.53 |
| Aw | pg/mL | 1.6E1 | 1.8E1 | 1.6E1 | 1.9E1 | 6.0E0 | 8.0E0 | 2.9E-2 | 1.1E1 | 4.8E1 | 5.1E1 | 421 | 33 | 165 | 33 | 0.60 |
| Ax | ng/mL | 2.0E0 | 8.7E0 | 1.3E1 | 5.3E1 | 5.3E1 | 1.3E2 | 1.9E-2 | 1.2E-2 | 7.7E2 | 6.2E2 | 421 | 33 | 165 | 33 | 0.67 |
| Ba | ng/mL | 5.7E1 | 2.2E2 | 4.1E2 | 1.3E3 | 1.1E3 | 2.9E3 | 2.7E-1 | 6.3E0 | 8.1E3 | 1.5E4 | 421 | 33 | 165 | 33 | 0.65 |
| Bb | ng/mL | 2.9E0 | 5.0E0 | 6.3E0 | 7.8E0 | 1.5E1 | 6.9E0 | 4.1E-3 | 2.6E-1 | 2.5E2 | 2.5E1 | 421 | 33 | 165 | 33 | 0.63 |
| Bc | ng/mL | 3.4E1 | 7.2E1 | 9.9E1 | 1.8E2 | 1.9E2 | 3.0E2 | 1.1E-1 | 8.0E0 | 1.2E3 | 1.2E3 | 421 | 33 | 165 | 33 | 0.66 |
| Bg | ng/mL | 7.4E-2 | 1.3E-1 | 4.5E0 | 1.3E1 | 2.1E1 | 6.9E1 | 5.3E-4 | 5.3E-4 | 2.5E2 | 4.0E2 | 421 | 33 | 165 | 33 | 0.61 |
| Bn | ng/mL | 5.6E-2 | 5.6E-2 | 1.2E0 | 3.1E0 | 2.0E0 | 1.0E1 | 5.6E-2 | 5.6E-2 | 9.7E0 | 5.8E1 | 421 | 33 | 165 | 33 | 0.53 |
| Bo | ng/mL | 1.2E1 | 2.0E1 | 1.4E1 | 1.9E1 | 1.9E1 | 1.5E1 | 1.6E-2 | 1.6E-2 | 2.8E2 | 5.3E1 | 421 | 33 | 165 | 33 | 0.60 |
| Ch | uIU/mL | 1.1E0 | 6.6E-1 | 1.9E1 | 3.8E1 | 1.1E2 | 2.1E2 | 3.4E-3 | 3.9E-2 | 1.8E3 | 1.2E3 | 421 | 33 | 165 | 33 | 0.40 |
| Co | pg/mL | 3.6E1 | 5.5E1 | 1.8E2 | 1.3E2 | 9.9E2 | 1.9E2 | 1.5E-1 | 1.5E-1 | 1.7E4 | 8.2E2 | 421 | 33 | 165 | 33 | 0.62 |
| Cp | ng/mL | 2.2E1 | 2.4E1 | 2.8E1 | 7.3E1 | 3.2E1 | 2.2E2 | 6.0E-1 | 6.0E-1 | 3.7E2 | 1.3E3 | 421 | 33 | 165 | 33 | 0.64 |
| Cq | ng/mL | 2.8E-2 | 3.2E-2 | 1.4E-1 | 1.6E0 | 8.8E-1 | 8.5E0 | 8.0E-4 | 8.0E-4 | 1.7E1 | 4.9E1 | 421 | 33 | 165 | 33 | 0.54 |
| Cs | ng/mL | 5.3E1 | 2.2E2 | 2.7E2 | 1.2E3 | 8.0E2 | 3.2E3 | 2.7E-2 | 2.7E0 | 1.1E4 | 1.8E4 | 421 | 33 | 165 | 33 | 0.65 |
| Ct | ng/mL | 8.6E-1 | 1.3E-1 | 3.5E1 | 4.6E1 | 1.0E2 | 1.3E2 | 1.1E-4 | 1.1E-4 | 6.2E2 | 4.7E2 | 421 | 33 | 165 | 33 | 0.40 |
| Cu | ng/mL | 2.3E-1 | 3.8E-1 | 4.1E-1 | 2.7E0 | 7.8E-1 | 1.1E1 | 9.0E-5 | 4.6E-2 | 9.2E0 | 6.6E1 | 421 | 33 | 165 | 33 | 0.67 |
| Cv | ng/mL | 4.7E0 | 8.9E0 | 2.2E1 | 6.0E1 | 6.0E1 | 1.3E2 | 1.4E-4 | 5.1E-2 | 5.3E2 | 5.2E2 | 421 | 33 | 165 | 33 | 0.55 |
| Cw | mIU/mL | 3.0E-2 | 3.9E-2 | 3.9E-2 | 2.5E-1 | 3.3E-2 | 1.2E0 | 8.9E-4 | 4.1E-3 | 2.4E-1 | 6.8E0 | 421 | 33 | 165 | 33 | 0.57 |
| Cx | ng/mL | 2.6E-1 | 2.6E-1 | 5.8E1 | 7.1E1 | 1.1E2 | 1.2E2 | 9.3E-5 | 9.3E-5 | 3.9E2 | 3.9E2 | 421 | 33 | 165 | 33 | 0.49 |
| Db | ug/mL | 7.5E0 | 8.2E0 | 9.1E0 | 8.4E0 | 8.7E0 | 6.5E0 | 4.5E-1 | 8.8E-1 | 1.0E2 | 2.3E1 | 421 | 33 | 165 | 33 | 0.50 |
| Dc | nmol/L | 1.8E-2 | 2.4E-2 | 5.5E-2 | 5.6E-1 | 1.3E-1 | 2.4E0 | 5.2E-6 | 1.0E-3 | 1.6E0 | 1.4E1 | 421 | 33 | 165 | 33 | 0.60 |
| Dd | ug/mL | 7.1E-2 | 7.2E-2 | 1.8E-1 | 2.6E-1 | 2.6E-1 | 6.3E-1 | 8.3E-5 | 3.3E-3 | 1.9E0 | 3.6E0 | 421 | 33 | 165 | 33 | 0.51 |
| De | ng/mL | 3.4E-3 | 3.4E-3 | 7.6E-2 | 1.1E-1 | 1.4E-1 | 2.1E-1 | 3.4E-3 | 3.4E-3 | 9.2E-1 | 9.0E-1 | 421 | 33 | 165 | 33 | 0.52 |
| Dg | ng/mL | 2.8E1 | 5.2E1 | 4.1E1 | 6.1E1 | 3.9E1 | 4.8E1 | 1.0E-1 | 2.3E0 | 1.9E2 | 1.9E2 | 421 | 33 | 165 | 33 | 0.62 |
| Di | pg/mL | 1.9E0 | 3.2E0 | 2.2E0 | 2.9E0 | 2.0E0 | 2.0E0 | 1.8E-1 | 1.8E-1 | 1.3E1 | 9.0E0 | 421 | 33 | 165 | 33 | 0.61 |
| Dk | uIU/mL | 1.6E-2 | 2.2E-2 | 9.0E-2 | 8.6E-2 | 5.3E-1 | 1.6E-1 | 1.1E-4 | 1.1E-4 | 8.9E0 | 6.3E-1 | 421 | 33 | 165 | 33 | 0.59 |
| Dl | ng/mL | 2.1E2 | 3.0E2 | 3.0E2 | 4.3E2 | 2.8E2 | 3.9E2 | 1.7E0 | 1.8E1 | 1.5E3 | 1.6E3 | 421 | 33 | 165 | 33 | 0.60 |
| Dp | ng/ml | 2.4E0 | 2.1E0 | 5.2E0 | 1.3E1 | 7.5E0 | 3.6E1 | 3.7E-3 | 3.7E-3 | 4.6E1 | 2.0E2 | 249 | 32 | 162 | 32 | 0.46 |
| Dr | pg/ml | 2.5E1 | 3.0E1 | 5.2E1 | 7.3E2 | 7.1E1 | 2.5E3 | 7.5E-1 | 7.5E-1 | 5.2E2 | 1.0E4 | 154 | 17 | 87 | 17 | 0.58 |
| Du | pg/ml | 6.9E1 | 1.8E2 | 8.0E2 | 2.5E3 | 2.9E3 | 6.2E3 | 1.2E0 | 1.2E0 | 2.6E4 | 2.4E4 | 95 | 15 | 74 | 15 | 0.55 |
| Ef | ng/ml | 1.3E-1 | 2.4E-1 | 8.3E-1 | 1.4E0 | 1.8E0 | 2.5E0 | 5.7E-4 | 1.1E-2 | 1.0E1 | 9.4E0 | 304 | 33 | 164 | 33 | 0.58 |
| Wm | % | 5.9E-1 | 1.9E0 | 2.0E1 | 1.2E2 | 1.5E2 | 3.0E2 | 5.4E-2 | 8.5E-2 | 2.4E3 | 1.0E3 | 341 | 36 | 184 | 36 | 0.59 |
| Ed | pg/ml | 5.2E-1 | 2.7E1 | 5.7E1 | 6.4E1 | 4.6E2 | 9.8E1 | 5.2E-1 | 5.2E-1 | 7.3E3 | 5.0E2 | 249 | 32 | 161 | 32 | 0.64 |
| Yf | ng/mL | 1.6E1 | 1.5E1 | 1.0E2 | 3.6E1 | 6.5E2 | 7.5E1 | 2.9E-1 | 2.9E-1 | 6.6E3 | 2.9E2 | 105 | 14 | 83 | 14 | 0.46 |
| Tj | pg/mL | 3.7E-1 | 3.7E-1 | 5.9E1 | 2.2E1 | 3.1E2 | 6.0E1 | 3.6E-1 | 3.7E-1 | 3.5E3 | 3.1E2 | 304 | 33 | 167 | 33 | 0.51 |
| Po | pg/ml | 4.8E-1 | 6.9E0 | 8.6E0 | 2.4E1 | 2.5E1 | 4.4E1 | 8.0E-3 | 2.6E-2 | 2.7E2 | 2.2E2 | 690 | 48 | 279 | 48 | 0.68 |
| Ti | ug/mL | 3.2E0 | 6.8E0 | 4.5E0 | 7.8E0 | 4.0E0 | 8.1E0 | 1.2E-1 | 8.7E-3 | 1.7E1 | 3.7E1 | 155 | 21 | 117 | 21 | 0.63 |
| Em | ng/ml | 2.9E-3 | 1.4E-2 | 5.5E-2 | 1.4E-1 | 1.1E-1 | 4.5E-1 | 1.9E-16 | 8.4E-4 | 6.0E-1 | 1.9E0 | 190 | 18 | 87 | 18 | 0.47 |
| Et | ng/ml | 1.3E3 | 3.0E3 | 1.5E3 | 2.7E3 | 1.1E3 | 1.3E3 | 7.5E1 | 1.1E2 | 5.0E3 | 5.0E3 | 689 | 48 | 279 | 48 | 0.76 |
| Eq | pg/ml | 1.6E2 | 8.2E1 | 3.3E2 | 2.7E2 | 4.0E2 | 4.0E2 | 1.0E0 | 1.0E0 | 1.8E3 | 1.3E3 | 95 | 15 | 74 | 15 | 0.44 |

| Code | Units | Median | | Average | | Standard Deviation | | Minimum | | Maximum | | Number of Samples | | Number of Patients | | AUC |
|------|-------|--------|-----|---------|-----|------|------|---------|-----|---------|-----|--------|-----|---------|-----|-----|
| | | NonDis | Dis | NonDis | Dis | NonDis | Dis | NonDis | Dis | NonDis | Dis | NonDis | Dis | NonDis | Dis | |
| Th | ug/mL | 1.1E0 | 1.0E0 | 1.6E0 | 1.4E0 | 1.5E0 | 1.2E0 | 2.6E-3 | 2.6E-3 | 1.2E1 | 4.2E0 | 155 | 21 | 117 | 21 | 0.45 |
| Fa | ng/ml | 3.9E1 | 8.4E1 | 1.2E2 | 2.5E2 | 5.6E2 | 4.9E2 | 3.4E-2 | 6.0E-1 | 8.0E3 | 2.5E3 | 243 | 32 | 159 | 32 | 0.69 |
| Ez | ng/ml | 3.8E0 | 5.5E0 | 1.8E1 | 1.6E1 | 5.5E1 | 2.3E1 | 1.3E-2 | 5.8E-2 | 7.1E2 | 8.8E1 | 249 | 32 | 162 | 32 | 0.57 |
| Fb | ng/ml | 2.5E1 | 2.8E1 | 2.2E1 | 2.7E1 | 1.2E1 | 1.0E1 | 6.6E-1 | 5.9E-1 | 5.7E1 | 4.3E1 | 244 | 32 | 159 | 32 | 0.62 |
| Ex | ng/ml | 7.4E-2 | 1.6E-1 | 2.4E-1 | 4.1E-1 | 7.2E-1 | 8.7E-1 | 3.5E-5 | 1.7E-4 | 8.9E0 | 4.1E0 | 224 | 23 | 113 | 23 | 0.64 |
| Fc | pg/ml | 2.2E-1 | 2.2E-1 | 1.7E2 | 2.7E1 | 1.5E3 | 7.9E1 | 2.2E-1 | 2.2E-1 | 1.5E4 | 3.1E2 | 97 | 15 | 74 | 15 | 0.61 |
| Fd | pg/ml | 1.5E1 | 2.5E2 | 8.1E2 | 2.2E3 | 3.5E3 | 6.4E3 | 4.5E-1 | 9.8E-1 | 3.3E4 | 2.5E4 | 97 | 15 | 74 | 15 | 0.61 |
| Fi | pg/ml | 2.5E-1 | 2.5E-1 | 2.0E2 | 2.2E1 | 1.4E3 | 6.6E1 | 2.5E-1 | 2.5E-1 | 1.4E4 | 2.5E2 | 97 | 15 | 74 | 15 | 0.52 |
| Fn | ng/ml | 2.1E-1 | 2.1E-1 | 6.1E0 | 4.0E0 | 2.7E1 | 6.6E0 | 1.1E-14 | 2.1E-1 | 4.2E2 | 2.7E1 | 249 | 32 | 162 | 32 | 0.49 |
| Fp | ng/ml | 1.2E1 | 4.6E1 | 2.3E1 | 5.0E1 | 2.8E1 | 3.9E1 | 6.0E-3 | 9.7E-1 | 1.4E2 | 1.4E2 | 720 | 47 | 280 | 47 | 0.72 |
| Fr | ng/ml | 3.2E4 | 9.6E4 | 1.1E5 | 2.6E5 | 1.7E5 | 2.9E5 | 1.9E2 | 1.3E3 | 9.0E5 | 8.4E5 | 823 | 50 | 284 | 50 | 0.69 |
| Fw | pg/ml | 1.1E0 | 6.0E0 | 6.2E1 | 5.9E1 | 5.0E2 | 1.8E2 | 1.1E-14 | 1.2E-1 | 6.9E3 | 9.1E2 | 304 | 34 | 165 | 34 | 0.60 |
| Fy | ng/ml | 3.5E1 | 5.3E1 | 5.5E1 | 1.2E2 | 5.6E1 | 1.6E2 | 1.2E-1 | 5.3E0 | 3.3E2 | 6.5E2 | 246 | 31 | 161 | 31 | 0.63 |
| Gh | pg/ml | 3.9E0 | 3.9E0 | 7.6E1 | 1.3E1 | 2.7E2 | 2.2E1 | 2.9E-2 | 2.9E-2 | 1.8E3 | 8.0E1 | 95 | 15 | 74 | 15 | 0.47 |
| Gb | % | 4.0E1 | 3.7E1 | 4.6E1 | 6.1E1 | 3.8E1 | 7.0E1 | 2.2E0 | 2.1E1 | 2.3E2 | 3.0E2 | 97 | 15 | 73 | 15 | 0.58 |
| Gc | ng/ml | 9.9E1 | 1.3E2 | 1.5E2 | 1.6E2 | 1.7E2 | 1.3E2 | 6.4E0 | 2.9E1 | 1.2E3 | 4.7E2 | 166 | 17 | 90 | 17 | 0.57 |
| Gd | ng/ml | 3.0E1 | 2.4E1 | 3.2E1 | 3.2E1 | 1.7E1 | 2.3E1 | 3.0E0 | 7.6E0 | 8.1E1 | 8.0E1 | 188 | 17 | 83 | 17 | 0.45 |
| Gn | U/ml | 3.6E-1 | 1.5E-1 | 1.3E0 | 7.8E0 | 3.1E0 | 2.7E1 | 1.3E-3 | 5.6E-3 | 3.0E1 | 1.1E2 | 148 | 17 | 85 | 17 | 0.47 |
| Gl | pg/ml | 7.4E3 | 9.3E3 | 1.1E4 | 1.4E4 | 9.4E3 | 1.1E4 | 9.1E1 | 6.7E2 | 3.4E4 | 3.2E4 | 295 | 34 | 164 | 34 | 0.58 |
| Gp | U/ml | 1.7E0 | 5.2E-1 | 4.1E0 | 3.3E0 | 6.6E0 | 8.4E0 | 1.3E-3 | 1.5E-2 | 6.7E1 | 4.8E1 | 306 | 33 | 165 | 33 | 0.38 |
| Gz | ug/ml | 1.4E0 | 1.0E0 | 9.2E0 | 3.8E0 | 3.9E1 | 4.9E0 | 2.9E-16 | 1.0E-1 | 4.8E2 | 1.5E1 | 165 | 22 | 106 | 22 | 0.44 |
| Ha | ng/ml | 2.6E0 | 5.4E0 | 9.9E0 | 1.2E1 | 2.1E1 | 2.5E1 | 1.7E-2 | 6.4E-3 | 1.3E2 | 1.0E2 | 247 | 32 | 161 | 32 | 0.56 |
| Nm | pg/ml | 1.4E4 | 2.7E4 | 3.0E4 | 5.7E4 | 8.2E4 | 1.2E5 | 1.0E-9 | 1.0E-9 | 1.6E6 | 8.2E5 | 693 | 48 | 281 | 48 | 0.62 |
| Nn | pg/ml | 1.5E2 | 4.5E2 | 1.9E3 | 5.8E3 | 8.6E3 | 2.0E4 | 1.0E-9 | 1.0E-9 | 1.0E5 | 1.1E5 | 693 | 48 | 281 | 48 | 0.65 |
| No | pg/ml | 1.4E1 | 4.2E1 | 3.5E1 | 1.0E2 | 1.2E2 | 1.7E2 | 1.0E-9 | 1.0E-9 | 2.5E3 | 7.7E2 | 693 | 48 | 281 | 48 | 0.72 |
| Nq | pg/ml | 1.9E0 | 3.5E0 | 1.7E1 | 3.5E1 | 7.0E1 | 6.9E1 | 1.0E-9 | 1.0E-9 | 1.2E3 | 2.9E2 | 693 | 48 | 281 | 48 | 0.56 |
| Nr | pg/ml | 6.5E-1 | 6.1E0 | 3.1E1 | 6.7E1 | 2.0E2 | 2.2E2 | 1.0E-9 | 1.0E-9 | 4.1E3 | 1.4E3 | 693 | 48 | 281 | 48 | 0.66 |
| Ns | pg/ml | 1.0E-9 | 1.0E-9 | 9.7E0 | 1.7E0 | 5.6E1 | 1.1E1 | 1.0E-9 | 1.0E-9 | 1.1E3 | 7.9E1 | 693 | 48 | 281 | 48 | 0.47 |
| Nt | pg/ml | 1.0E2 | 1.6E2 | 1.3E2 | 2.1E2 | 1.1E2 | 2.0E2 | 1.0E-9 | 4.4E1 | 1.5E3 | 1.2E3 | 693 | 48 | 281 | 48 | 0.68 |
| Nu | pg/ml | 1.9E1 | 5.3E1 | 5.4E1 | 1.0E2 | 9.4E1 | 1.0E2 | 1.0E-9 | 1.0E-9 | 8.9E2 | 3.7E2 | 693 | 48 | 281 | 48 | 0.67 |
| Lu | pg/ml | 1.0E4 | 1.0E4 | 1.6E4 | 9.1E3 | 4.6E4 | 5.7E3 | 3.5E2 | 1.1E3 | 7.5E5 | 2.5E4 | 695 | 48 | 281 | 48 | 0.44 |
| Lv | pg/ml | 1.0E-9 | 1.7E1 | 1.1E1 | 3.3E1 | 2.1E1 | 4.6E1 | 1.0E-9 | 1.0E-9 | 2.4E2 | 1.8E2 | 695 | 48 | 281 | 48 | 0.64 |
| Lw | pg/ml | 1.0E-9 | 1.0E-9 | 3.7E-1 | 2.9E0 | 4.0E0 | 9.7E0 | 1.0E-9 | 1.0E-9 | 8.0E1 | 4.7E1 | 695 | 48 | 281 | 48 | 0.54 |
| Lx | pg/ml | 1.0E-9 | 1.7E2 | 1.3E2 | 5.4E2 | 4.2E2 | 9.0E2 | 1.0E-9 | 1.0E-9 | 6.2E3 | 4.5E3 | 695 | 48 | 281 | 48 | 0.73 |
| Ly | pg/ml | 1.0E-9 | 1.0E-9 | 1.0E1 | 1.3E1 | 2.0E1 | 2.0E1 | 1.0E-9 | 1.0E-9 | 1.6E2 | 7.2E1 | 695 | 48 | 281 | 48 | 0.52 |
| Lz | pg/ml | 1.0E-9 | 1.0E-9 | 4.9E0 | 5.0E0 | 3.4E1 | 1.5E1 | 1.0E-9 | 1.0E-9 | 6.0E2 | 6.2E1 | 695 | 48 | 281 | 48 | 0.52 |
| Ma | pg/ml | 2.7E2 | 6.5E2 | 1.4E3 | 3.2E3 | 3.8E3 | 8.6E3 | 1.0E-9 | 1.0E-9 | 6.5E4 | 5.2E4 | 695 | 48 | 281 | 48 | 0.63 |
| Mb | pg/ml | 2.5E1 | 3.5E1 | 3.1E1 | 3.6E1 | 1.6E1 | 1.7E1 | 5.4E0 | 4.1E0 | 2.1E2 | 7.1E1 | 695 | 48 | 281 | 48 | 0.57 |
| Mc | pg/ml | 1.0E-9 | 1.0E-9 | 3.6E-2 | 2.9E-2 | 5.3E-1 | 2.0E-1 | 1.0E-9 | 1.0E-9 | 1.3E1 | 1.4E0 | 695 | 48 | 281 | 48 | 0.50 |
| Md | pg/ml | 1.0E-9 | 1.0E-9 | 5.0E-1 | 8.5E-1 | 4.0E0 | 4.3E0 | 1.0E-9 | 1.0E-9 | 6.8E1 | 2.9E1 | 695 | 48 | 281 | 48 | 0.50 |
| Me | pg/ml | 3.2E1 | 2.4E1 | 3.1E1 | 2.5E1 | 2.0E1 | 2.9E1 | 1.0E-9 | 1.0E-9 | 3.2E2 | 1.8E2 | 695 | 48 | 281 | 48 | 0.36 |
| Mf | pg/ml | 1.0E-9 | 1.0E-9 | 5.0E-1 | 8.0E-1 | 2.9E0 | 1.9E0 | 1.0E-9 | 1.0E-9 | 5.6E1 | 9.1E0 | 695 | 48 | 281 | 48 | 0.56 |
| Mg | pg/ml | 1.7E0 | 2.4E0 | 7.3E0 | 9.5E0 | 1.2E1 | 1.5E1 | 1.0E-9 | 1.0E-9 | 9.2E1 | 6.6E1 | 695 | 48 | 281 | 48 | 0.55 |
| Mh | pg/ml | 1.0E-9 | 1.0E-9 | 1.5E0 | 2.3E0 | 1.1E1 | 6.9E0 | 1.0E-9 | 1.0E-9 | 1.6E2 | 4.2E1 | 695 | 48 | 281 | 48 | 0.59 |
| Mi | pg/ml | 1.0E-9 | 1.0E-9 | 4.9E-1 | 3.4E0 | 5.8E0 | 1.6E1 | 1.0E-9 | 1.0E-9 | 1.2E2 | 1.1E2 | 695 | 48 | 281 | 48 | 0.53 |
| Mj | pg/ml | 1.0E-9 | 1.0E-9 | 4.8E0 | 1.4E1 | 2.7E1 | 4.1E1 | 1.0E-9 | 1.0E-9 | 4.6E2 | 2.1E2 | 695 | 48 | 281 | 48 | 0.58 |
| Mk | pg/ml | 9.1E-1 | 2.9E0 | 1.5E1 | 1.7E1 | 9.7E1 | 7.2E1 | 1.0E-9 | 1.0E-9 | 1.7E3 | 5.0E2 | 695 | 48 | 281 | 48 | 0.53 |
| Ml | pg/ml | 1.0E-9 | 1.0E-9 | 5.8E0 | 2.3E1 | 8.2E1 | 8.9E1 | 1.0E-9 | 1.0E-9 | 2.1E3 | 5.1E2 | 695 | 48 | 281 | 48 | 0.55 |
| Mm | pg/ml | 5.4E2 | 8.8E2 | 9.2E2 | 1.7E3 | 1.1E3 | 2.2E3 | 1.0E-9 | 1.0E-9 | 7.3E3 | 1.2E4 | 695 | 48 | 281 | 48 | 0.62 |
| Mn | pg/ml | 5.3E0 | 8.3E0 | 1.0E1 | 1.3E1 | 2.4E1 | 2.0E1 | 1.0E-9 | 1.0E-9 | 3.5E2 | 1.3E2 | 695 | 48 | 281 | 48 | 0.61 |
| Mp | pg/ml | 1.0E-9 | 7.9E0 | 8.4E0 | 1.9E1 | 2.9E1 | 3.1E1 | 1.0E-9 | 1.0E-9 | 6.3E2 | 1.3E2 | 694 | 48 | 281 | 48 | 0.64 |
| Mq | pg/ml | 1.0E-9 | 1.0E-9 | 2.8E0 | 9.7E0 | 1.6E1 | 2.3E1 | 1.0E-9 | 1.0E-9 | 2.1E2 | 1.0E2 | 694 | 48 | 281 | 48 | 0.61 |
| Mr | pg/ml | 1.0E-9 | 3.2E0 | 1.6E1 | 1.5E2 | 7.9E1 | 5.6E2 | 1.0E-9 | 1.0E-9 | 1.4E3 | 3.4E3 | 694 | 48 | 281 | 48 | 0.61 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ms | pg/ml | 4.1E2 | 3.1E2 | 5.6E2 | 3.7E2 | 6.4E2 | 3.4E2 | 1.0E-9 | 1.0E-9 | 5.6E3 | 1.4E3 | 694 | 48 | 281 | 48 | 0.43 |
| Mt | pg/ml | 1.0E-9 | 2.0E0 | 6.8E0 | 8.3E1 | 4.6E1 | 4.7E2 | 1.0E-9 | 1.0E-9 | 8.7E2 | 3.2E3 | 694 | 48 | 281 | 48 | 0.71 |
| Mu | pg/ml | 1.0E-9 | 1.0E-9 | 1.3E0 | 2.3E0 | 1.2E1 | 6.1E0 | 1.0E-9 | 1.0E-9 | 2.3E2 | 3.5E1 | 694 | 48 | 281 | 48 | 0.61 |
| Mv | pg/ml | 1.0E-9 | 1.0E-9 | 6.8E1 | 1.3E2 | 3.3E2 | 4.1E2 | 1.0E-9 | 1.0E-9 | 4.5E3 | 2.5E3 | 694 | 48 | 281 | 48 | 0.58 |
| Mw | pg/ml | 3.2E1 | 8.6E1 | 4.3E2 | 6.0E2 | 2.9E3 | 1.4E3 | 1.0E-9 | 1.0E-9 | 6.2E4 | 5.9E3 | 694 | 48 | 281 | 48 | 0.64 |
| Mx | pg/ml | 1.0E-9 | 1.0E-9 | 2.3E-1 | 8.3E-1 | 1.4E0 | 3.0E0 | 1.0E-9 | 1.0E-9 | 3.2E1 | 2.0E1 | 694 | 48 | 281 | 48 | 0.59 |
| My | pg/ml | 1.0E-9 | 1.0E-9 | 4.3E2 | 2.1E2 | 2.9E3 | 7.3E2 | 1.0E-9 | 1.0E-9 | 3.9E4 | 4.6E3 | 694 | 48 | 281 | 48 | 0.52 |
| Mz | pg/ml | 1.0E1 | 2.7E1 | 2.4E1 | 1.0E2 | 6.8E1 | 3.3E2 | 1.0E-9 | 1.0E-9 | 1.2E3 | 1.9E3 | 694 | 48 | 281 | 48 | 0.69 |
| Na | pg/ml | 1.0E-9 | 1.0E-9 | 7.7E-1 | 2.1E0 | 2.9E0 | 6.6E0 | 1.0E-9 | 1.0E-9 | 3.6E1 | 4.2E1 | 694 | 48 | 281 | 48 | 0.57 |
| Nb | pg/ml | 1.9E0 | 3.1E0 | 4.0E0 | 1.1E1 | 1.3E1 | 3.3E1 | 1.0E-9 | 1.0E-9 | 2.3E2 | 2.1E2 | 694 | 48 | 281 | 48 | 0.63 |
| Nc | pg/ml | 3.8E2 | 2.7E2 | 6.2E2 | 3.7E2 | 7.7E2 | 3.6E2 | 1.0E-9 | 1.0E-9 | 9.2E3 | 1.1E3 | 694 | 48 | 281 | 48 | 0.44 |
| Nd | pg/ml | 2.9E1 | 6.5E0 | 2.7E1 | 1.6E1 | 5.0E1 | 2.0E1 | 1.0E-9 | 1.0E-9 | 1.2E3 | 9.4E1 | 694 | 48 | 281 | 48 | 0.37 |
| Ne | pg/ml | 4.7E2 | 3.5E2 | 6.0E2 | 3.8E2 | 5.9E2 | 3.3E2 | 1.0E-9 | 1.0E-9 | 7.0E3 | 1.7E3 | 694 | 48 | 281 | 48 | 0.39 |
| Nf | pg/ml | 1.0E-9 | 1.0E-9 | 3.2E0 | 7.2E0 | 1.0E1 | 2.5E1 | 1.0E-9 | 1.0E-9 | 1.6E2 | 1.3E2 | 694 | 48 | 281 | 48 | 0.51 |
| Ng | pg/ml | 2.1E1 | 1.0E1 | 1.3E2 | 8.0E1 | 2.6E2 | 1.8E2 | 1.0E-9 | 1.0E-9 | 2.3E3 | 1.2E3 | 694 | 48 | 281 | 48 | 0.47 |
| Nh | pg/ml | 7.1E1 | 4.0E1 | 9.4E1 | 5.0E1 | 8.6E1 | 3.7E1 | 1.0E-9 | 1.0E-9 | 5.6E2 | 1.5E2 | 694 | 48 | 281 | 48 | 0.34 |
| Ni | pg/ml | 1.0E-9 | 7.6E1 | 7.6E1 | 1.5E2 | 1.2E2 | 2.1E2 | 1.0E-9 | 1.0E-9 | 7.1E2 | 1.1E3 | 694 | 48 | 281 | 48 | 0.59 |
| Nj | pg/ml | 8.2E0 | 3.4E0 | 1.2E1 | 7.2E0 | 1.2E1 | 8.2E0 | 1.0E-9 | 1.0E-9 | 8.3E1 | 3.3E1 | 694 | 48 | 281 | 48 | 0.38 |
| Nk | pg/ml | 1.9E1 | 2.5E1 | 3.4E1 | 3.6E1 | 4.0E1 | 4.4E1 | 1.0E-9 | 1.0E-9 | 2.0E2 | 2.0E2 | 694 | 48 | 281 | 48 | 0.53 |
| Nl | pg/ml | 4.9E1 | 3.0E1 | 6.5E1 | 3.9E1 | 7.2E1 | 3.5E1 | 1.0E-9 | 1.0E-9 | 1.1E3 | 1.3E2 | 694 | 48 | 281 | 48 | 0.39 |
| Hl | pg/ml | 1.3E1 | 1.3E1 | 3.5E1 | 2.9E2 | 6.3E1 | 9.2E2 | 1.0E-9 | 1.0E-9 | 3.5E2 | 3.6E3 | 97 | 15 | 74 | 15 | 0.53 |
| Ho | pg/ml | 1.6E1 | 2.1E1 | 2.8E1 | 4.4E1 | 7.1E1 | 9.6E1 | 1.0E-9 | 2.0E0 | 7.0E2 | 3.9E2 | 97 | 15 | 74 | 15 | 0.58 |
| Hp | ng/ml | 1.6E0 | 3.9E0 | 1.2E2 | 2.4E2 | 3.0E2 | 4.0E2 | 1.0E-9 | 2.0E-1 | 8.9E2 | 8.9E2 | 97 | 15 | 74 | 15 | 0.64 |
| Tz | pg/ml | 5.2E3 | 7.5E3 | 1.3E4 | 2.0E4 | 6.4E4 | 6.5E4 | 1.0E-9 | 6.8E2 | 1.0E6 | 3.7E5 | 251 | 32 | 160 | 32 | 0.58 |
| Ua | pg/ml | 3.8E3 | 5.0E3 | 2.3E4 | 1.1E4 | 1.4E5 | 1.6E4 | 1.0E-9 | 9.4E2 | 2.1E6 | 6.6E4 | 251 | 32 | 160 | 32 | 0.55 |
| Ub | pg/ml | 5.7E2 | 4.8E2 | 8.6E2 | 8.1E2 | 1.0E3 | 9.2E2 | 1.0E-9 | 1.2E1 | 9.8E3 | 4.1E3 | 251 | 32 | 160 | 32 | 0.48 |
| Ue | pg/ml | 3.0E1 | 2.9E1 | 3.7E1 | 4.1E1 | 3.2E1 | 3.2E1 | 9.8E-2 | 5.9E0 | 3.5E2 | 1.4E2 | 251 | 32 | 160 | 32 | 0.54 |
| Uc | pg/ml | 8.6E2 | 1.4E3 | 1.6E3 | 3.8E3 | 2.7E3 | 1.0E4 | 1.0E-9 | 5.5E1 | 2.9E4 | 5.7E4 | 251 | 32 | 160 | 32 | 0.60 |
| Ud | pg/ml | 1.0E-9 | 1.0E-9 | 1.7E0 | 1.8E0 | 2.5E1 | 9.4E0 | 1.0E-9 | 1.0E-9 | 3.9E2 | 5.3E1 | 251 | 32 | 160 | 32 | 0.53 |
| Hq | pg/ml | 1.1E0 | 1.3E0 | 1.3E2 | 1.3E1 | 1.8E3 | 4.2E1 | 1.0E-9 | 1.0E-9 | 3.4E4 | 2.3E2 | 691 | 48 | 280 | 48 | 0.50 |
| Hr | pg/ml | 1.3E2 | 1.2E2 | 8.2E2 | 7.0E2 | 1.6E3 | 1.7E3 | 1.0E-9 | 1.0E-9 | 1.4E4 | 8.9E3 | 691 | 48 | 280 | 48 | 0.48 |
| Hu | pg/ml | 7.1E0 | 3.1E1 | 3.0E3 | 1.1E3 | 2.9E4 | 4.7E3 | 1.0E-9 | 1.0E-9 | 6.3E5 | 3.2E4 | 691 | 48 | 280 | 48 | 0.59 |
| Hv | pg/ml | 1.4E0 | 1.5E0 | 3.3E0 | 2.2E1 | 1.2E1 | 1.3E2 | 1.0E-9 | 1.0E-9 | 2.5E2 | 8.9E2 | 691 | 48 | 280 | 48 | 0.53 |
| Hw | pg/ml | 7.1E0 | 5.4E0 | 2.0E1 | 2.2E2 | 8.0E1 | 1.3E3 | 1.0E-9 | 1.0E-9 | 1.7E3 | 9.4E3 | 691 | 48 | 280 | 48 | 0.43 |
| Hx | pg/ml | 8.8E0 | 1.3E1 | 4.4E1 | 6.4E1 | 3.6E2 | 2.2E2 | 1.0E-9 | 1.0E-9 | 9.3E3 | 1.3E3 | 691 | 48 | 280 | 48 | 0.56 |
| Ib | ng/ml | 6.2E-2 | 4.4E-2 | 1.4E0 | 2.0E0 | 5.0E0 | 9.9E0 | 1.0E-9 | 1.0E-9 | 3.6E1 | 5.6E1 | 241 | 32 | 159 | 32 | 0.46 |
| Ic | U/ml | 2.2E2 | 2.2E2 | 7.3E2 | 2.0E2 | 6.1E3 | 1.3E2 | 2.4E0 | 2.1E1 | 9.3E4 | 4.2E2 | 241 | 32 | 159 | 32 | 0.53 |
| Id | U/ml | 6.4E-1 | 9.7E-1 | 1.2E0 | 1.6E1 | 2.0E0 | 7.6E1 | 1.0E-9 | 3.0E-1 | 2.3E1 | 4.3E2 | 241 | 32 | 159 | 32 | 0.67 |
| Tt | pg/ml | 1.6E2 | 1.7E2 | 1.7E2 | 1.8E2 | 5.1E1 | 7.3E1 | 4.3E1 | 1.0E2 | 3.6E2 | 4.4E2 | 231 | 28 | 153 | 28 | 0.54 |
| To | pg/ml | 1.6E0 | 1.6E0 | 1.9E0 | 1.8E0 | 2.4E0 | 2.5E0 | 1.0E-9 | 1.0E-9 | 2.3E1 | 1.2E1 | 242 | 30 | 157 | 30 | 0.48 |
| Tr | pg/ml | 2.8E0 | 5.2E0 | 5.8E0 | 1.1E1 | 2.0E1 | 1.7E1 | 1.0E-9 | 4.7E-2 | 3.1E2 | 7.6E1 | 238 | 29 | 156 | 29 | 0.64 |
| Tn | pg/ml | 2.6E1 | 4.7E1 | 8.0E1 | 2.1E2 | 2.2E2 | 5.3E2 | 2.4E0 | 6.6E0 | 1.8E3 | 2.3E3 | 242 | 30 | 157 | 30 | 0.64 |
| Tv | ng/ml | 1.2E1 | 1.3E1 | 1.9E1 | 2.7E2 | 3.7E1 | 1.3E3 | 1.0E-9 | 1.0E-9 | 4.9E2 | 7.1E3 | 242 | 30 | 157 | 30 | 0.50 |
| Ih | ng/ml | 6.9E1 | 1.8E2 | 2.1E2 | 3.7E2 | 3.7E2 | 5.0E2 | 1.0E-9 | 1.0E-9 | 2.6E3 | 2.8E3 | 694 | 48 | 280 | 48 | 0.62 |
| Ii | ng/ml | 9.0E1 | 1.1E2 | 2.6E2 | 3.7E2 | 7.4E2 | 8.1E2 | 7.3E-1 | 2.3E0 | 1.0E4 | 4.5E3 | 694 | 48 | 280 | 48 | 0.57 |
| Ij | ng/ml | 7.3E1 | 1.2E2 | 1.8E2 | 7.0E2 | 6.4E2 | 3.5E3 | 2.1E0 | 1.1E1 | 6.4E3 | 2.4E4 | 686 | 47 | 279 | 47 | 0.66 |
| Ik | ng/ml | 1.4E1 | 1.4E2 | 9.8E2 | 5.1E2 | 9.3E3 | 6.4E2 | 5.9E-1 | 2.3E0 | 1.2E5 | 2.5E3 | 690 | 48 | 279 | 48 | 0.66 |
| Il | ng/ml | 3.3E2 | 6.1E2 | 1.2E3 | 2.5E3 | 2.7E3 | 4.1E3 | 1.0E-9 | 1.0E-9 | 1.2E4 | 1.3E4 | 680 | 48 | 279 | 48 | 0.57 |
| Im | ng/ml | 1.9E2 | 6.5E2 | 3.4E2 | 8.6E2 | 5.0E2 | 1.2E3 | 1.3E1 | 4.7E1 | 6.0E3 | 6.2E3 | 689 | 48 | 279 | 48 | 0.69 |
| In | ng/ml | 3.9E0 | 3.2E0 | 2.5E1 | 1.0E2 | 1.7E2 | 6.5E2 | 1.0E-9 | 1.0E-9 | 3.9E3 | 4.5E3 | 694 | 48 | 280 | 48 | 0.45 |
| Hb | ng/ml | 2.4E1 | 3.3E1 | 3.2E1 | 4.9E1 | 2.9E1 | 4.6E1 | 4.8E-1 | 6.2E-1 | 1.5E2 | 1.9E2 | 248 | 32 | 160 | 32 | 0.62 |
| Hc | pg/ml | 6.7E2 | 6.4E2 | 3.7E3 | 2.9E3 | 1.3E4 | 9.0E3 | 1.0E-9 | 1.0E-9 | 1.0E5 | 5.0E4 | 248 | 32 | 160 | 32 | 0.46 |
| Hf | ng/ml | 1.5E2 | 2.0E2 | 3.8E2 | 3.2E2 | 5.3E2 | 3.9E2 | 1.0E-9 | 1.0E-9 | 2.5E3 | 1.8E3 | 248 | 32 | 160 | 32 | 0.50 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Io | ng/ml | 7.5E3 | 9.3E3 | 2.4E4 | 1.4E4 | 1.6E5 | 1.2E4 | 1.0E-9 | 1.8E2 | 4.0E6 | 5.4E4 | 687 | 48 | 280 | 48 | 0.54 |
| Ip | ng/ml | 8.7E0 | 3.0E1 | 1.9E1 | 2.8E1 | 2.4E1 | 2.2E1 | 1.0E-9 | 3.7E-2 | 2.6E2 | 8.8E1 | 687 | 48 | 280 | 48 | 0.62 |
| Iq | ug/ml | 9.5E-2 | 1.7E-1 | 4.0E1 | 6.3E0 | 7.3E2 | 3.2E1 | 1.0E-9 | 1.0E-9 | 1.4E4 | 2.2E2 | 687 | 48 | 280 | 48 | 0.56 |
| Ir | ug/ml | 3.3E-1 | 8.0E-1 | 3.8E0 | 1.5E1 | 2.8E1 | 5.8E1 | 1.0E-9 | 1.0E-9 | 5.1E2 | 3.7E2 | 686 | 48 | 280 | 48 | 0.67 |
| Is | ng/ml | 1.4E0 | 8.6E0 | 5.7E0 | 2.2E1 | 2.3E1 | 4.4E1 | 1.0E-9 | 5.3E-2 | 5.5E2 | 2.6E2 | 687 | 48 | 280 | 48 | 0.74 |
| It | ng/ml | 2.0E0 | 3.8E0 | 2.6E1 | 3.0E1 | 1.5E2 | 1.1E2 | 1.0E-9 | 1.0E-9 | 2.8E3 | 6.8E2 | 687 | 48 | 280 | 48 | 0.61 |
| Iu | ng/ml | 2.1E2 | 2.7E2 | 1.4E3 | 2.0E3 | 4.3E3 | 5.1E3 | 1.0E-9 | 1.0E-9 | 2.9E4 | 2.4E4 | 687 | 48 | 280 | 48 | 0.52 |
| Iv | ng/ml | 1.2E1 | 3.6E1 | 6.1E1 | 2.2E2 | 6.1E2 | 7.5E2 | 1.0E-9 | 1.0E-9 | 1.6E4 | 3.8E3 | 686 | 48 | 280 | 48 | 0.69 |
| Iz | ng/ml | 1.4E2 | 1.9E2 | 6.8E2 | 3.2E2 | 4.0E3 | 3.8E2 | 1.5E0 | 4.9E0 | 6.2E4 | 1.7E3 | 248 | 32 | 160 | 32 | 0.53 |
| Yg | pg/ml | 2.8E2 | 5.1E2 | 1.3E3 | 1.3E3 | 5.3E3 | 1.7E3 | 1.0E-9 | 1.1E0 | 5.0E4 | 5.0E3 | 95 | 14 | 73 | 14 | 0.60 |
| Yh | pg/ml | 2.1E2 | 6.6E2 | 5.2E2 | 6.4E2 | 9.2E2 | 6.5E2 | 1.0E-9 | 1.0E-9 | 7.8E3 | 2.3E3 | 95 | 14 | 73 | 14 | 0.58 |
| Yi | pg/ml | 2.6E2 | 6.9E2 | 5.2E2 | 2.8E3 | 8.6E2 | 6.8E3 | 1.0E-9 | 1.0E-9 | 7.6E3 | 2.6E4 | 95 | 14 | 73 | 14 | 0.70 |
| Yk | U/ml | 1.0E-9 | 1.0E-9 | 1.6E-1 | 4.0E-1 | 4.7E-1 | 1.5E0 | 1.0E-9 | 1.0E-9 | 3.0E0 | 5.6E0 | 95 | 14 | 73 | 14 | 0.44 |
| Yj | pg/ml | 1.5E2 | 1.5E2 | 4.3E2 | 3.1E2 | 9.1E2 | 2.9E2 | 1.0E-9 | 1.7E1 | 7.0E3 | 8.5E2 | 95 | 14 | 73 | 14 | 0.53 |
| Yd | ng/ml | 2.0E-1 | 2.0E-1 | 3.3E-1 | 4.1E-1 | 3.6E-1 | 6.1E-1 | 6.6E-3 | 6.6E-3 | 1.8E0 | 2.3E0 | 100 | 15 | 77 | 15 | 0.49 |
| Wb | pg/ml | 3.1E4 | 3.8E4 | 3.5E4 | 4.8E4 | 2.1E4 | 3.3E4 | 2.2E3 | 1.4E4 | 1.6E5 | 1.5E5 | 99 | 15 | 77 | 15 | 0.64 |
| Vz | pg/ml | 3.2E0 | 2.8E0 | 4.7E0 | 5.0E0 | 6.0E0 | 5.4E0 | 1.0E-9 | 7.6E-2 | 4.0E1 | 2.2E1 | 99 | 15 | 77 | 15 | 0.55 |
| Si | ng/ml | 1.0E0 | 8.5E-1 | 2.0E0 | 1.9E0 | 2.7E0 | 1.8E0 | 8.6E-3 | 3.7E-2 | 1.3E1 | 5.6E0 | 97 | 15 | 74 | 15 | 0.53 |
| Sf | mIU/mL | 1.3E1 | 1.3E1 | 4.0E1 | 2.6E1 | 8.6E1 | 3.4E1 | 8.1E-2 | 9.4E-1 | 7.2E2 | 1.2E2 | 97 | 15 | 74 | 15 | 0.49 |
| Sh | mIU/mL | 1.3E1 | 7.7E0 | 4.8E1 | 3.6E1 | 1.0E2 | 7.6E1 | 2.9E-2 | 1.3E-1 | 5.9E2 | 2.9E2 | 97 | 15 | 74 | 15 | 0.44 |
| Sj | ng/ml | 4.0E-1 | 4.3E-1 | 4.1E-1 | 4.4E-1 | 1.0E-1 | 9.0E-2 | 1.1E-1 | 3.4E-1 | 6.6E-1 | 7.0E-1 | 97 | 15 | 74 | 15 | 0.57 |
| Rc | pg/ml | 5.5E3 | 7.7E3 | 7.3E3 | 7.4E3 | 6.0E3 | 4.0E3 | 1.9E2 | 1.3E3 | 3.9E4 | 1.7E4 | 248 | 32 | 160 | 32 | 0.56 |
| Rb | pg/ml | 7.9E-1 | 7.2E-1 | 2.6E0 | 3.9E0 | 4.4E0 | 1.0E1 | 1.0E-9 | 1.0E-9 | 4.3E1 | 5.6E1 | 248 | 32 | 160 | 32 | 0.52 |
| Zq | 2.6ng/ml | 2.3E2 | 3.9E2 | 3.0E2 | 5.0E2 | 2.6E2 | 3.3E2 | 8.3E0 | 1.8E1 | 9.7E2 | 9.7E2 | 97 | 15 | 74 | 15 | 0.69 |
| Zw | 2.5ng/ml | 5.7E0 | 4.7E0 | 1.1E1 | 1.6E1 | 1.4E1 | 2.1E1 | 6.3E-2 | 6.6E-1 | 5.9E1 | 6.3E1 | 100 | 15 | 77 | 15 | 0.58 |
| Zx | 2.3mU/ml | 1.3E-1 | 1.1E-1 | 3.7E-1 | 2.8E-1 | 1.2E0 | 4.7E-1 | 3.2E-2 | 6.1E-2 | 1.2E1 | 1.9E0 | 100 | 15 | 77 | 15 | 0.53 |
| Pz | ng/ml | 3.3E3 | 1.0E4 | 6.8E3 | 7.2E3 | 1.8E4 | 5.4E3 | 1.3E1 | 1.1E2 | 2.8E5 | 2.8E4 | 687 | 48 | 278 | 48 | 0.61 |
| Qa | ng/ml | 3.1E3 | 9.7E3 | 5.8E3 | 1.6E4 | 7.1E3 | 3.2E4 | 1.5E2 | 6.0E2 | 5.2E4 | 2.2E5 | 687 | 48 | 278 | 48 | 0.72 |
| Qb | ng/ml | 9.1E1 | 2.0E2 | 2.1E2 | 3.5E2 | 5.2E2 | 6.1E2 | 7.9E-1 | 6.2E0 | 8.3E3 | 4.1E3 | 687 | 48 | 278 | 48 | 0.64 |
| Qc | ng/ml | 2.1E2 | 4.9E2 | 4.4E2 | 6.4E2 | 7.6E2 | 7.8E2 | 1.0E-9 | 3.2E0 | 1.1E4 | 4.3E3 | 687 | 48 | 278 | 48 | 0.60 |
| Qd | ng/ml | 8.7E3 | 1.9E4 | 1.9E4 | 4.0E4 | 8.2E4 | 5.1E4 | 1.5E2 | 1.7E3 | 2.0E6 | 2.3E5 | 687 | 48 | 278 | 48 | 0.69 |
| Qe | ng/ml | 8.0E2 | 2.6E3 | 1.7E3 | 3.3E3 | 4.2E3 | 3.4E3 | 1.0E-9 | 1.5E2 | 9.7E4 | 1.8E4 | 687 | 48 | 278 | 48 | 0.73 |
| Jd | ng/ml | 9.2E-1 | 2.8E0 | 6.7E0 | 4.7E0 | 4.4E1 | 7.2E0 | 1.0E-9 | 1.0E-9 | 6.5E2 | 3.5E1 | 249 | 32 | 162 | 32 | 0.64 |
| Je | ng/ml | 1.0E-9 | 5.7E-1 | 2.3E0 | 2.2E0 | 8.0E0 | 3.1E0 | 1.0E-9 | 1.0E-9 | 8.8E1 | 1.1E1 | 249 | 32 | 162 | 32 | 0.55 |
| Jf | ng/ml | 1.0E-9 | 1.0E-9 | 1.1E0 | 1.1E0 | 2.3E0 | 1.8E0 | 1.0E-9 | 1.0E-9 | 1.2E1 | 7.7E0 | 249 | 32 | 162 | 32 | 0.54 |
| Jg | ng/ml | 4.4E2 | 1.1E3 | 7.3E2 | 1.4E3 | 9.6E2 | 1.4E3 | 1.0E-9 | 3.7E1 | 1.0E4 | 7.1E3 | 691 | 48 | 280 | 48 | 0.67 |
| Jh | ng/ml | 2.9E0 | 6.5E0 | 2.4E1 | 5.4E1 | 1.1E2 | 1.2E2 | 1.0E-9 | 1.0E-9 | 1.6E3 | 5.4E2 | 691 | 48 | 280 | 48 | 0.60 |
| Ji | ng/ml | 5.0E1 | 1.2E2 | 7.0E1 | 2.0E2 | 6.9E1 | 2.2E2 | 1.0E-9 | 1.3E1 | 5.3E2 | 1.3E3 | 691 | 48 | 280 | 48 | 0.78 |
| Sr | pg/mL | 3.4E2 | 1.2E3 | 8.1E2 | 2.2E3 | 1.3E3 | 3.7E3 | 1.0E-9 | 1.0E-9 | 9.8E3 | 2.1E4 | 239 | 32 | 157 | 32 | 0.70 |
| Ss | pg/mL | 9.2E4 | 1.1E5 | 1.5E5 | 1.5E5 | 1.9E5 | 1.5E5 | 2.7E3 | 1.3E4 | 1.8E6 | 5.7E5 | 239 | 32 | 157 | 32 | 0.51 |
| St | pg/mL | 2.2E7 | 8.4E7 | 4.9E7 | 1.4E8 | 9.0E7 | 3.0E8 | 1.0E-9 | 2.3E6 | 1.2E9 | 1.7E9 | 244 | 32 | 158 | 32 | 0.69 |
| Wc | ng/ml | 1.0E-9 | 1.0E-9 | 5.8E-2 | 1.2E-1 | 1.3E-1 | 4.6E-1 | 1.0E-9 | 1.0E-9 | 9.8E-1 | 1.8E0 | 100 | 15 | 77 | 15 | 0.38 |
| Wd | ng/ml | 9.4E0 | 1.2E1 | 3.6E1 | 1.3E2 | 1.0E2 | 3.1E2 | 1.0E-9 | 3.5E0 | 7.9E2 | 1.2E3 | 100 | 15 | 77 | 15 | 0.63 |
| We | ng/ml | 3.6E-1 | 4.7E-1 | 8.7E-1 | 4.9E0 | 1.4E0 | 9.0E0 | 1.0E-9 | 2.0E-3 | 9.7E0 | 2.3E1 | 100 | 15 | 77 | 15 | 0.54 |
| Wg | ng/ml | 1.0E-9 | 1.0E-9 | 1.6E-4 | 3.6E-2 | 1.6E-3 | 1.4E-1 | 1.0E-9 | 1.0E-9 | 1.6E-2 | 5.3E-1 | 100 | 15 | 77 | 15 | 0.53 |
| Wh | ng/ml | 1.0E-2 | 2.0E-2 | 7.0E-2 | 3.6E-1 | 2.6E-1 | 1.2E0 | 1.0E-9 | 3.7E-3 | 2.5E0 | 4.5E0 | 100 | 15 | 77 | 15 | 0.63 |
| Wf | ng/ml | 1.0E-9 | 1.0E-9 | 1.8E-1 | 2.8E-1 | 5.6E-1 | 6.0E-1 | 1.0E-9 | 1.0E-9 | 4.6E0 | 2.3E0 | 100 | 15 | 77 | 15 | 0.55 |
| Ra | pg/ml | 1.0E-9 | 1.0E-9 | 6.4E-1 | 2.7E0 | 1.3E0 | 1.1E1 | 1.0E-9 | 1.0E-9 | 7.3E0 | 6.4E1 | 248 | 32 | 160 | 32 | 0.53 |
| Qz | pg/ml | 1.1E1 | 1.2E1 | 6.1E1 | 5.0E1 | 1.0E2 | 7.5E1 | 1.0E-9 | 1.0E-9 | 7.1E2 | 2.3E2 | 248 | 32 | 160 | 32 | 0.51 |
| Qy | pg/ml | 4.3E-1 | 5.7E-1 | 1.0E1 | 5.9E0 | 5.7E1 | 1.9E1 | 1.0E-9 | 1.0E-9 | 6.5E2 | 9.7E1 | 248 | 32 | 160 | 32 | 0.55 |
| Qx | pg/ml | 1.0E-9 | 1.0E-9 | 6.8E0 | 6.1E0 | 5.2E1 | 2.0E1 | 1.0E-9 | 1.0E-9 | 5.8E2 | 1.1E2 | 248 | 32 | 160 | 32 | 0.52 |
| Qw | pg/ml | 1.0E-9 | 1.0E-9 | 2.5E0 | 1.5E0 | 1.1E1 | 4.1E0 | 1.0E-9 | 1.0E-9 | 1.2E2 | 2.3E1 | 248 | 32 | 160 | 32 | 0.50 |
| Qv | pg/ml | 2.4E4 | 2.1E4 | 3.4E4 | 2.1E4 | 5.7E4 | 1.8E4 | 1.0E-9 | 4.0E2 | 7.4E5 | 9.1E4 | 248 | 32 | 160 | 32 | 0.40 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qu | pg/ml | 7.7E0 | 9.2E0 | 8.8E1 | 8.7E1 | 1.7E2 | 1.7E2 | 1.0E-9 | 1.0E-9 | 9.8E2 | 7.0E2 | 248 | 32 | 160 | 32 | 0.50 |
| Qt | pg/ml | 1.0E1 | 2.0E1 | 5.3E1 | 5.0E1 | 1.3E2 | 8.1E1 | 1.0E-9 | 1.0E-9 | 1.0E3 | 3.1E2 | 248 | 32 | 160 | 32 | 0.57 |
| Qh | ng/ml | 1.7E1 | 3.4E1 | 3.6E1 | 5.8E1 | 6.1E1 | 8.5E1 | 1.0E-9 | 3.8E0 | 6.4E2 | 4.6E2 | 248 | 32 | 160 | 32 | 0.63 |
| Qg | ng/ml | 8.5E0 | 4.9E0 | 2.1E1 | 1.4E1 | 7.1E1 | 2.7E1 | 5.1E-2 | 3.3E-1 | 1.0E3 | 1.4E2 | 248 | 32 | 160 | 32 | 0.41 |
| Jj | ng/ml | 6.7E2 | 2.8E2 | 1.9E3 | 5.3E2 | 1.4E4 | 6.1E2 | 2.3E0 | 1.2E1 | 3.4E5 | 3.3E3 | 691 | 48 | 280 | 48 | 0.33 |
| Jk | ng/ml | 3.0E0 | 3.9E0 | 2.2E1 | 2.8E1 | 4.7E1 | 5.1E1 | 1.0E-9 | 2.4E-1 | 3.9E2 | 2.4E2 | 691 | 48 | 280 | 48 | 0.56 |
| Jl | ng/ml | 3.9E-1 | 1.6E0 | 1.7E0 | 2.1E2 | 4.2E0 | 1.4E3 | 7.6E-4 | 1.5E-2 | 3.2E1 | 9.9E3 | 691 | 48 | 280 | 48 | 0.68 |
| Jm | ng/ml | 1.7E1 | 3.6E1 | 5.2E1 | 9.9E1 | 1.1E2 | 3.0E2 | 1.0E-9 | 2.5E-1 | 1.4E3 | 2.1E3 | 691 | 48 | 280 | 48 | 0.58 |
| Jn | pg/ml | 3.8E-1 | 8.9E-1 | 2.4E0 | 3.0E1 | 2.4E1 | 1.4E2 | 1.0E-9 | 1.0E-9 | 6.2E2 | 7.3E2 | 691 | 48 | 280 | 48 | 0.67 |
| Jo | pg/ml | 3.6E3 | 4.1E3 | 4.9E3 | 7.7E3 | 3.9E3 | 1.5E4 | 2.0E1 | 2.3E2 | 2.4E4 | 1.0E5 | 691 | 48 | 280 | 48 | 0.53 |
| Jp | pg/ml | 6.8E4 | 8.9E4 | 7.1E4 | 9.6E4 | 3.6E4 | 4.2E4 | 5.8E2 | 2.6E4 | 3.0E5 | 2.1E5 | 691 | 48 | 280 | 48 | 0.68 |
| Jq | pg/ml | 9.5E1 | 1.8E2 | 1.5E2 | 5.0E2 | 2.2E2 | 1.3E3 | 1.0E0 | 1.4E0 | 4.0E3 | 8.7E3 | 691 | 48 | 280 | 48 | 0.66 |
| Jr | pg/ml | 5.1E0 | 1.0E1 | 3.5E1 | 2.8E2 | 4.1E2 | 1.3E3 | 1.0E-9 | 1.0E-9 | 1.1E4 | 7.4E3 | 691 | 48 | 280 | 48 | 0.65 |
| Js | pg/ml | 1.3E1 | 1.8E1 | 5.2E1 | 1.5E2 | 4.0E2 | 5.9E2 | 1.0E-9 | 1.7E0 | 1.0E4 | 3.0E3 | 691 | 48 | 280 | 48 | 0.64 |
| Jt | pg/ml | 2.5E3 | 3.4E3 | 3.1E3 | 5.2E3 | 2.3E3 | 7.9E3 | 2.2E1 | 4.1E2 | 2.2E4 | 5.2E4 | 691 | 48 | 280 | 48 | 0.60 |
| Xa | pg/ml | 1.0E-9 | 8.7E0 | 9.0E0 | 1.0E2 | 1.7E1 | 3.1E2 | 1.0E-9 | 1.0E-9 | 9.6E1 | 1.2E3 | 99 | 15 | 77 | 15 | 0.72 |
| Ye | pg/ml | 1.0E-9 | 1.0E-9 | 5.2E0 | 1.2E0 | 1.3E1 | 4.6E0 | 1.0E-9 | 1.0E-9 | 6.9E1 | 1.8E1 | 99 | 15 | 77 | 15 | 0.39 |
| Tm | pg/ml | 1.0E-9 | 1.0E-9 | 3.5E0 | 1.1E0 | 6.8E0 | 2.9E0 | 1.0E-9 | 1.0E-9 | 4.6E1 | 8.4E0 | 99 | 15 | 77 | 15 | 0.41 |
| Tl | pg/ml | 1.3E-1 | 1.0E-9 | 3.1E-1 | 1.8E0 | 3.7E-1 | 6.3E0 | 1.0E-9 | 1.0E-9 | 1.8E0 | 2.5E1 | 99 | 15 | 77 | 15 | 0.38 |
| Ju | mIU/ml | 8.5E0 | 1.1E1 | 2.0E1 | 1.7E1 | 3.2E1 | 1.9E1 | 6.5E-2 | 1.9E-1 | 2.3E2 | 8.9E1 | 249 | 32 | 162 | 32 | 0.54 |
| Jv | mIU/ml | 1.1E1 | 1.2E1 | 3.5E1 | 2.9E1 | 6.3E1 | 4.0E1 | 1.0E-2 | 2.4E-2 | 4.4E2 | 1.9E2 | 249 | 32 | 162 | 32 | 0.52 |
| Jy | ng/ml | 1.6E-3 | 2.0E-3 | 2.2E-3 | 4.3E-3 | 4.3E-3 | 7.8E-3 | 1.0E-9 | 5.3E-4 | 5.2E-2 | 4.1E-2 | 249 | 32 | 162 | 32 | 0.62 |
| Kc | pg/ml | 2.3E1 | 3.2E1 | 4.1E1 | 5.0E1 | 4.3E1 | 6.0E1 | 1.0E-9 | 1.0E-9 | 1.9E2 | 2.7E2 | 249 | 32 | 160 | 32 | 0.55 |
| Kd | pg/ml | 1.0E-9 | 1.0E-9 | 1.6E2 | 1.7E3 | 5.4E2 | 6.8E3 | 1.0E-9 | 1.0E-9 | 4.8E3 | 3.8E4 | 249 | 32 | 160 | 32 | 0.57 |
| Ke | pg/ml | 1.2E4 | 1.9E4 | 1.4E4 | 3.5E4 | 1.1E4 | 5.7E4 | 3.4E2 | 1.8E3 | 7.0E4 | 3.2E5 | 249 | 32 | 160 | 32 | 0.68 |
| Kf | pg/mL | 6.4E0 | 7.3E0 | 6.8E0 | 1.1E1 | 5.6E0 | 1.5E1 | 1.0E-9 | 1.0E-9 | 2.6E1 | 7.8E1 | 249 | 32 | 160 | 32 | 0.57 |
| Kg | pg/mL | 1.1E3 | 1.1E3 | 1.9E3 | 2.3E3 | 2.6E3 | 4.8E3 | 7.3E1 | 1.3E2 | 2.2E4 | 2.7E4 | 249 | 32 | 160 | 32 | 0.47 |
| Ki | pg/ml | 6.1E1 | 7.6E1 | 7.0E1 | 8.4E1 | 5.2E1 | 6.2E1 | 1.0E-9 | 1.0E-9 | 3.8E2 | 2.5E2 | 248 | 32 | 160 | 32 | 0.57 |
| Kj | pg/ml | 1.0E3 | 7.7E2 | 1.6E3 | 1.7E3 | 1.6E3 | 2.8E3 | 1.4E1 | 3.3E1 | 1.0E4 | 1.5E4 | 249 | 32 | 160 | 32 | 0.42 |
| Kk | pg/ml | 6.9E0 | 1.0E1 | 1.1E1 | 1.7E1 | 1.5E1 | 1.8E1 | 1.0E-9 | 2.0E0 | 1.6E2 | 5.9E1 | 249 | 32 | 160 | 32 | 0.63 |
| Kl | pg/ml | 2.0E4 | 2.4E4 | 2.7E4 | 3.3E4 | 2.5E4 | 2.8E4 | 1.6E2 | 1.3E3 | 1.6E5 | 1.1E5 | 249 | 32 | 160 | 32 | 0.56 |
| Kn | pg/ml | 2.9E1 | 6.0E1 | 5.8E1 | 2.6E2 | 9.0E1 | 8.6E2 | 1.0E-9 | 1.0E-9 | 7.3E2 | 4.9E3 | 249 | 32 | 160 | 32 | 0.63 |
| Ko | pg/ml | 3.2E2 | 5.9E2 | 4.3E2 | 7.9E2 | 4.4E2 | 9.5E2 | 1.0E-9 | 1.0E-9 | 2.2E3 | 4.1E3 | 249 | 32 | 160 | 32 | 0.63 |
| Kp | pg/ml | 3.0E2 | 3.4E2 | 3.4E2 | 7.8E2 | 2.6E2 | 2.3E3 | 1.0E-9 | 3.7E1 | 1.7E3 | 1.3E4 | 249 | 32 | 160 | 32 | 0.56 |
| Kq | pg/ml | 3.0E2 | 5.6E2 | 4.8E2 | 6.0E3 | 8.7E2 | 2.8E4 | 1.6E0 | 4.8E1 | 9.8E3 | 1.6E5 | 240 | 32 | 154 | 32 | 0.71 |
| Kr | pg/ml | 3.8E-1 | 5.1E-1 | 2.2E0 | 1.5E1 | 4.2E0 | 7.3E1 | 1.0E-9 | 1.0E-9 | 3.5E1 | 4.2E2 | 240 | 32 | 154 | 32 | 0.53 |
| Ks | pg/ml | 1.4E4 | 1.7E4 | 2.0E4 | 2.0E4 | 1.8E4 | 1.7E4 | 5.1E1 | 9.9E2 | 1.1E5 | 5.1E4 | 240 | 32 | 154 | 32 | 0.51 |
| Ps | ng/ml | 1.6E2 | 4.7E2 | 4.8E2 | 7.7E2 | 1.3E3 | 9.3E2 | 4.1E-1 | 1.3E1 | 9.0E3 | 3.8E3 | 97 | 15 | 74 | 15 | 0.73 |
| Kx | ng/ml | 1.0E-9 | 5.8E-3 | 6.6E-3 | 1.4E-2 | 1.4E-2 | 2.0E-2 | 1.0E-9 | 1.0E-9 | 1.0E-1 | 6.5E-2 | 247 | 32 | 160 | 32 | 0.62 |
| Ky | ng/ml | 8.5E-2 | 2.8E-1 | 3.5E-1 | 6.2E-1 | 7.8E-1 | 7.5E-1 | 1.0E-9 | 1.0E-9 | 5.4E0 | 2.7E0 | 247 | 32 | 160 | 32 | 0.65 |
| Kz | ng/ml | 1.0E-9 | 1.0E-9 | 3.7E-3 | 5.5E-3 | 5.9E-3 | 6.9E-3 | 1.0E-9 | 1.0E-9 | 3.6E-2 | 1.8E-2 | 247 | 32 | 160 | 32 | 0.56 |
| Rz | ng/ml | 3.4E-1 | 3.3E-1 | 9.5E-1 | 1.5E0 | 1.4E0 | 2.0E0 | 3.6E-3 | 1.7E-2 | 6.7E0 | 5.9E0 | 97 | 15 | 74 | 15 | 0.57 |
| Ry | ng/ml | 1.6E-2 | 2.3E-2 | 2.2E-2 | 4.6E-2 | 2.2E-2 | 8.6E-2 | 1.0E-9 | 1.0E-9 | 1.2E-1 | 3.5E-1 | 97 | 15 | 74 | 15 | 0.59 |
| Rx | ng/ml | 1.0E-9 | 3.5E-5 | 1.7E-3 | 1.7E-3 | 3.2E-3 | 2.5E-3 | 1.0E-9 | 1.0E-9 | 2.0E-2 | 8.4E-3 | 97 | 15 | 74 | 15 | 0.56 |
| Ld | pg/ml | 1.0E-9 | 1.7E0 | 3.6E0 | 5.4E0 | 9.2E0 | 9.8E0 | 1.0E-9 | 1.0E-9 | 8.4E1 | 5.0E1 | 246 | 32 | 159 | 32 | 0.58 |
| Lh | pg/ml | 1.2E4 | 2.1E4 | 2.0E4 | 5.2E4 | 2.5E4 | 8.1E4 | 1.0E-9 | 6.7E2 | 2.6E5 | 4.1E5 | 691 | 48 | 281 | 48 | 0.68 |
| Li | pg/ml | 2.8E3 | 1.2E4 | 1.5E4 | 5.3E4 | 5.9E4 | 1.4E5 | 1.0E-9 | 3.4E1 | 1.3E6 | 9.2E5 | 691 | 48 | 281 | 48 | 0.71 |
| Lj | pg/ml | 2.3E3 | 7.7E3 | 2.1E4 | 4.4E4 | 6.5E4 | 8.5E4 | 1.0E-9 | 1.4E2 | 4.7E5 | 4.1E5 | 691 | 48 | 281 | 48 | 0.69 |
| Lp | pg/ml | 1.1E1 | 1.1E1 | 7.1E1 | 1.5E2 | 1.7E2 | 3.1E2 | 1.0E-9 | 1.0E-9 | 1.1E3 | 1.0E3 | 97 | 15 | 74 | 15 | 0.47 |
| Lt | pg/ml | 1.0E-9 | 1.0E-9 | 2.4E0 | 1.7E0 | 9.1E0 | 6.5E0 | 1.0E-9 | 1.0E-9 | 6.0E1 | 2.5E1 | 97 | 15 | 74 | 15 | 0.49 |
| Rv | ng/ml | 5.0E-4 | 8.0E-4 | 1.2E-3 | 2.3E-3 | 2.2E-3 | 3.7E-3 | 1.0E-9 | 1.0E-9 | 1.6E-2 | 1.2E-2 | 97 | 15 | 74 | 15 | 0.57 |
| Ru | ng/ml | 1.0E-9 | 1.0E-9 | 1.5E-2 | 5.0E-2 | 6.6E-2 | 1.2E-1 | 1.0E-9 | 1.0E-9 | 3.8E-1 | 3.5E-1 | 97 | 15 | 74 | 15 | 0.57 |
| Rt | ng/ml | 7.0E-2 | 3.6E-2 | 1.1E-1 | 5.8E-1 | 1.4E-1 | 1.9E0 | 1.0E-3 | 1.3E-3 | 6.3E-1 | 7.4E0 | 97 | 15 | 74 | 15 | 0.43 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Yl | pg/ml | 1.1E1 | 1.3E1 | 1.7E1 | 3.4E1 | 1.9E1 | 5.4E1 | 1.0E-9 | 1.0E-9 | 1.1E2 | 2.2E2 | 100 | 15 | 77 | 15 | 0.60 |
| Rm | ng/ml | 1.9E1 | 3.4E1 | 4.9E1 | 8.5E1 | 7.4E1 | 1.3E2 | 2.2E-1 | 3.9E-1 | 4.0E2 | 6.5E2 | 245 | 32 | 159 | 32 | 0.61 |
| Rh | ng/ml | 1.3E2 | 1.2E2 | 3.6E2 | 1.1E3 | 1.2E3 | 3.6E3 | 3.6E0 | 2.5E1 | 1.7E4 | 1.7E4 | 245 | 32 | 159 | 32 | 0.54 |
| Ri | ng/ml | 1.0E-9 | 1.0E-9 | 4.4E0 | 3.0E0 | 1.6E1 | 8.6E0 | 1.0E-9 | 1.0E-9 | 2.2E2 | 4.5E1 | 246 | 32 | 160 | 32 | 0.43 |
| Rg | ng/ml | 1.0E-9 | 1.0E-9 | 7.1E-2 | 4.8E-2 | 4.2E-1 | 1.5E-1 | 1.0E-9 | 1.0E-9 | 4.6E0 | 6.2E-1 | 245 | 32 | 159 | 32 | 0.51 |
| Rj | ng/ml | 1.0E-9 | 1.0E-9 | 1.6E0 | 8.9E0 | 6.0E0 | 4.8E1 | 1.0E-9 | 1.0E-9 | 8.0E1 | 2.7E2 | 246 | 32 | 160 | 32 | 0.47 |
| Rf | ng/ml | 3.7E-1 | 8.3E-1 | 9.8E-1 | 2.1E0 | 1.9E0 | 3.6E0 | 7.8E-3 | 1.8E-2 | 1.5E1 | 1.7E1 | 245 | 32 | 159 | 32 | 0.65 |
| Ql | pg/ml | 4.5E0 | 1.1E1 | 1.4E1 | 2.0E1 | 3.1E1 | 2.6E1 | 1.0E-9 | 1.0E-9 | 2.9E2 | 9.3E1 | 249 | 32 | 162 | 32 | 0.62 |
| Qm | pg/ml | 3.9E0 | 2.0E1 | 2.0E1 | 3.1E1 | 3.9E1 | 3.7E1 | 1.0E-9 | 1.0E-9 | 3.6E2 | 1.7E2 | 249 | 32 | 162 | 32 | 0.65 |
| Qn | pg/ml | 6.1E-1 | 6.1E-1 | 6.9E0 | 1.4E1 | 2.2E1 | 4.5E1 | 1.0E-9 | 1.0E-9 | 2.2E2 | 2.4E2 | 249 | 32 | 162 | 32 | 0.52 |
| Nv | pg/ml | 3.8E3 | 7.3E3 | 1.1E4 | 1.7E4 | 4.8E4 | 2.5E4 | 1.0E-9 | 1.5E2 | 1.1E6 | 1.1E5 | 696 | 48 | 281 | 48 | 0.65 |
| Nw | pg/ml | 8.1E3 | 1.7E4 | 1.2E4 | 3.0E4 | 1.7E4 | 4.2E4 | 8.6E1 | 1.7E3 | 2.1E5 | 2.2E5 | 696 | 48 | 281 | 48 | 0.75 |
| Nx | pg/ml | 2.0E2 | 2.7E2 | 3.8E2 | 7.1E2 | 6.6E2 | 8.6E2 | 1.0E-9 | 1.0E-9 | 7.4E3 | 4.1E3 | 696 | 48 | 281 | 48 | 0.65 |
| Ny | pg/ml | 5.5E0 | 1.7E1 | 6.2E1 | 1.1E2 | 9.6E2 | 4.1E2 | 1.0E-9 | 1.0E-9 | 2.5E4 | 2.8E3 | 696 | 48 | 281 | 48 | 0.68 |
| Oa | pg/ml | 1.6E2 | 4.6E2 | 4.0E2 | 9.0E2 | 6.9E2 | 1.0E3 | 1.0E-9 | 4.9E0 | 4.8E3 | 4.5E3 | 249 | 32 | 162 | 32 | 0.68 |
| Op | pg/ml | 4.2E5 | 4.4E5 | 4.1E5 | 4.1E5 | 1.6E5 | 2.0E5 | 3.3E4 | 6.7E4 | 7.3E5 | 7.5E5 | 97 | 15 | 74 | 15 | 0.50 |
| Wn | ng/ml | 1.3E1 | 2.2E1 | 5.8E1 | 3.1E2 | 2.1E2 | 7.7E2 | 8.9E-1 | 2.7E0 | 1.8E3 | 2.4E3 | 86 | 9 | 63 | 9 | 0.66 |
| Tk | ng/ml | 1.3E2 | 1.4E2 | 3.1E2 | 3.8E2 | 5.6E2 | 5.1E2 | 3.0E0 | 1.0E1 | 4.2E3 | 1.4E3 | 92 | 10 | 66 | 10 | 0.52 |
| Oe | pg/ml | 4.9E1 | 2.1E1 | 2.9E2 | 2.6E2 | 8.1E2 | 4.5E2 | 1.0E-9 | 1.0E-9 | 1.9E4 | 2.2E3 | 688 | 48 | 281 | 48 | 0.48 |
| Of | pg/ml | 1.8E2 | 9.8E1 | 6.4E3 | 6.0E3 | 3.1E4 | 2.0E4 | 1.0E-9 | 1.0E-9 | 6.2E5 | 1.1E5 | 695 | 48 | 281 | 48 | 0.47 |
| Og | pg/ml | 8.4E-2 | 5.8E-2 | 5.3E-1 | 2.2E-1 | 1.7E0 | 7.3E-1 | 1.0E-9 | 1.0E-9 | 2.0E1 | 5.0E0 | 695 | 48 | 281 | 48 | 0.43 |
| Oh | pg/ml | 2.4E0 | 5.1E0 | 2.3E1 | 4.2E1 | 1.6E2 | 1.7E2 | 1.0E-9 | 1.4E-1 | 3.5E3 | 1.1E3 | 695 | 48 | 281 | 48 | 0.67 |
| Oi | pg/ml | 2.3E0 | 2.8E0 | 6.1E0 | 7.3E0 | 9.9E0 | 1.1E1 | 1.0E-9 | 1.0E-9 | 8.6E1 | 5.1E1 | 695 | 48 | 281 | 48 | 0.53 |
| Ok | pg/ml | 3.5E2 | 7.4E2 | 5.0E2 | 1.1E3 | 5.2E2 | 1.3E3 | 1.3E1 | 3.2E1 | 5.2E3 | 7.8E3 | 695 | 48 | 281 | 48 | 0.73 |
| Om | pg/ml | 3.8E2 | 6.5E2 | 8.5E2 | 2.4E3 | 2.3E3 | 7.4E3 | 1.0E-9 | 1.0E2 | 3.6E4 | 5.1E4 | 695 | 48 | 281 | 48 | 0.68 |
| On | pg/ml | 1.6E2 | 3.2E2 | 2.7E2 | 7.4E2 | 4.1E2 | 1.3E3 | 1.0E-9 | 1.5E1 | 4.5E3 | 8.5E3 | 695 | 48 | 281 | 48 | 0.71 |
| Or | pg/ml | 1.2E1 | 2.1E1 | 3.1E1 | 8.3E1 | 6.0E1 | 1.4E2 | 1.0E-9 | 1.0E-9 | 5.0E2 | 5.1E2 | 249 | 32 | 160 | 32 | 0.58 |
| Ow | pg/ml | 3.3E1 | 5.5E1 | 1.2E2 | 2.5E2 | 3.5E2 | 5.6E2 | 1.0E-9 | 1.0E-9 | 3.2E3 | 3.0E3 | 249 | 32 | 160 | 32 | 0.59 |
| Ou | pg/ml | 4.6E2 | 6.5E2 | 8.6E2 | 2.3E3 | 1.3E3 | 3.4E3 | 1.0E-9 | 1.0E-9 | 9.8E3 | 1.1E4 | 249 | 32 | 160 | 32 | 0.57 |
| Ul | ng/ml | 1.0E-9 | 1.0E-9 | 1.2E0 | 4.9E0 | 4.6E0 | 1.4E1 | 1.0E-9 | 1.0E-9 | 3.4E1 | 5.6E1 | 257 | 32 | 164 | 32 | 0.52 |
| Uo | ng/ml | 1.0E-9 | 1.0E-9 | 9.2E-2 | 5.1E-2 | 2.1E-1 | 1.4E-1 | 1.0E-9 | 1.0E-9 | 1.3E0 | 6.3E-1 | 257 | 32 | 164 | 32 | 0.46 |
| Uk | ng/ml | 1.0E-9 | 1.0E-9 | 7.8E-3 | 3.5E-3 | 2.7E-2 | 9.7E-3 | 1.0E-9 | 1.0E-9 | 3.2E-1 | 3.9E-2 | 257 | 32 | 164 | 32 | 0.37 |
| Um | ng/ml | 1.0E-9 | 1.0E-9 | 3.7E-1 | 2.0E-1 | 8.5E-1 | 4.7E-1 | 1.0E-9 | 1.0E-9 | 6.8E0 | 2.3E0 | 257 | 32 | 164 | 32 | 0.44 |
| Uf | ng/ml | 5.1E-2 | 1.1E-1 | 1.2E-1 | 3.6E-1 | 1.9E-1 | 9.8E-1 | 1.0E-3 | 9.8E-3 | 1.7E0 | 5.6E0 | 257 | 32 | 164 | 32 | 0.68 |
| Uh | ng/ml | 1.8E0 | 4.0E0 | 2.9E0 | 5.6E0 | 3.2E0 | 5.1E0 | 3.2E-2 | 9.5E-2 | 1.7E1 | 1.8E1 | 257 | 32 | 164 | 32 | 0.69 |
| Un | ng/ml | 1.8E0 | 2.5E0 | 2.1E0 | 3.7E0 | 1.3E0 | 4.2E0 | 2.0E-1 | 5.3E-1 | 8.0E0 | 2.5E1 | 257 | 32 | 164 | 32 | 0.70 |
| Ug | ng/ml | 1.4E1 | 1.3E1 | 2.7E1 | 2.5E1 | 2.8E1 | 3.3E1 | 6.9E-1 | 1.7E0 | 1.8E2 | 1.6E2 | 257 | 32 | 164 | 32 | 0.45 |
| Ur | ng/ml | 1.5E-1 | 4.5E-2 | 7.7E-1 | 7.4E-1 | 5.9E0 | 1.8E0 | 1.0E-9 | 1.0E-9 | 9.3E1 | 7.3E0 | 256 | 32 | 163 | 32 | 0.40 |
| Up | ng/ml | 1.0E-9 | 1.0E-9 | 5.4E-3 | 7.8E-2 | 2.5E-2 | 4.2E-1 | 1.0E-9 | 1.0E-9 | 3.3E-1 | 2.4E0 | 256 | 32 | 163 | 32 | 0.52 |
| Us | ng/ml | 3.2E-3 | 5.2E-5 | 1.8E-2 | 8.0E-2 | 4.4E-2 | 2.9E-1 | 1.0E-9 | 1.0E-9 | 4.0E-1 | 1.7E0 | 256 | 32 | 163 | 32 | 0.50 |
| Uv | ng/ml | 3.0E-3 | 3.2E-3 | 1.3E-2 | 2.6E-2 | 4.2E-2 | 8.0E-2 | 1.0E-9 | 1.0E-9 | 4.3E-1 | 4.1E-1 | 256 | 32 | 163 | 32 | 0.51 |
| Ut | ng/ml | 6.6E-1 | 1.1E0 | 2.9E0 | 5.3E0 | 9.1E0 | 1.3E1 | 1.0E-9 | 1.0E-9 | 7.8E1 | 6.5E1 | 256 | 32 | 163 | 32 | 0.62 |
| Uu | ng/ml | 7.0E0 | 5.4E0 | 7.7E0 | 8.7E0 | 5.0E0 | 9.1E0 | 4.5E-1 | 1.2E0 | 2.6E1 | 4.0E1 | 256 | 32 | 163 | 32 | 0.48 |
| Uw | ng/ml | 2.2E0 | 4.0E0 | 3.0E0 | 6.1E0 | 4.0E0 | 9.4E0 | 1.0E-9 | 9.9E-1 | 3.7E1 | 3.9E1 | 106 | 15 | 83 | 15 | 0.68 |
| Vb | ng/ml | 1.0E0 | 9.5E-1 | 1.0E0 | 1.3E0 | 4.3E-1 | 1.5E0 | 8.5E-2 | 2.6E-1 | 2.5E0 | 6.4E0 | 106 | 15 | 83 | 15 | 0.45 |
| Vc | ng/ml | 1.0E-9 | 1.0E-9 | 8.2E-3 | 1.0E-9 | 6.5E-2 | 0.0E0 | 1.0E-9 | 1.0E-9 | 6.5E-1 | 1.0E-9 | 106 | 15 | 83 | 15 | 0.47 |
| Uy | ng/ml | 1.2E0 | 1.4E0 | 4.5E0 | 1.1E1 | 1.2E1 | 2.1E1 | 3.1E-2 | 2.0E-2 | 9.9E1 | 6.4E1 | 106 | 15 | 83 | 15 | 0.53 |
| Uz | ng/ml | 1.0E-9 | 1.0E-9 | 4.2E-3 | 2.2E0 | 4.2E-2 | 8.5E0 | 1.0E-9 | 1.0E-9 | 4.4E-1 | 3.3E1 | 106 | 15 | 83 | 15 | 0.52 |
| Ux | ng/ml | 1.8E2 | 2.2E2 | 1.9E2 | 2.2E2 | 1.4E2 | 1.3E2 | 4.5E0 | 3.5E1 | 5.3E2 | 4.6E2 | 106 | 15 | 83 | 15 | 0.59 |
| Va | ng/ml | 1.9E1 | 4.9E0 | 2.7E1 | 1.7E1 | 2.7E1 | 2.6E1 | 1.2E-1 | 1.2E0 | 1.2E2 | 7.8E1 | 106 | 15 | 83 | 15 | 0.34 |
| Vh | ng/ml | 1.0E-2 | 1.4E-2 | 1.6E-2 | 7.4E-2 | 2.1E-2 | 2.2E-1 | 1.0E-9 | 2.2E-3 | 1.2E-1 | 8.6E-1 | 106 | 15 | 83 | 15 | 0.61 |
| Vi | ng/ml | 3.0E-3 | 1.1E-2 | 1.4E-1 | 1.4E-1 | 1.3E0 | 4.7E-1 | 1.0E-9 | 1.0E-9 | 1.4E1 | 1.8E0 | 106 | 15 | 83 | 15 | 0.67 |
| Vj | ng/ml | 2.6E1 | 6.2E1 | 2.1E2 | 7.0E1 | 9.6E2 | 5.6E1 | 1.4E0 | 5.4E0 | 8.4E3 | 1.7E2 | 106 | 13 | 83 | 13 | 0.60 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vp | ng/ml | 1.0E-9 | 1.0E-9 | 4.0E-1 | 1.6E0 | 3.6E0 | 8.7E0 | 1.0E-9 | 1.0E-9 | 5.0E1 | 4.9E1 | 257 | 32 | 164 | 32 | 0.53 |
| Vt | ng/ml | 6.0E0 | 1.2E1 | 8.4E0 | 2.0E1 | 9.1E0 | 2.9E1 | 4.3E-1 | 1.8E0 | 8.6E1 | 1.6E2 | 257 | 32 | 164 | 32 | 0.69 |
| Vu | ng/ml | 1.0E-9 | 1.0E-9 | 2.3E0 | 2.5E0 | 5.6E0 | 5.0E0 | 1.0E-9 | 1.0E-9 | 5.1E1 | 2.2E1 | 253 | 30 | 164 | 30 | 0.53 |
| Vq | ng/ml | 1.6E2 | 6.6E2 | 4.1E3 | 1.8E3 | 4.8E4 | 3.2E3 | 2.0E-1 | 1.0E1 | 6.8E5 | 1.2E4 | 203 | 19 | 135 | 19 | 0.66 |
| Vo | ng/ml | 2.6E1 | 2.6E1 | 2.4E1 | 2.5E1 | 5.4E0 | 4.5E0 | 2.4E0 | 1.1E1 | 4.8E1 | 3.3E1 | 257 | 32 | 164 | 32 | 0.53 |
| Vs | ng/ml | 1.0E-9 | 1.0E-9 | 6.7E0 | 2.1E1 | 2.4E1 | 8.7E1 | 1.0E-9 | 1.0E-9 | 2.3E2 | 4.5E2 | 253 | 27 | 161 | 27 | 0.49 |
| Vv | ng/ml | 2.9E0 | 2.6E0 | 6.1E0 | 5.3E0 | 1.0E1 | 7.1E0 | 1.0E-9 | 1.0E-9 | 8.4E1 | 3.2E1 | 256 | 31 | 164 | 31 | 0.50 |
| Vw | ng/ml | 3.6E1 | 4.1E1 | 3.5E1 | 4.1E1 | 1.7E1 | 1.6E1 | 2.5E0 | 1.1E1 | 7.0E1 | 6.6E1 | 106 | 15 | 83 | 15 | 0.60 |
| Oy | pg/ml | 5.3E-1 | 4.0E-1 | 6.6E0 | 2.9E0 | 3.2E1 | 8.6E0 | 1.0E-9 | 1.0E-9 | 4.0E2 | 4.9E1 | 694 | 48 | 280 | 48 | 0.45 |
| Oz | pg/ml | 1.3E-2 | 1.0E-9 | 3.4E-1 | 8.4E-1 | 1.5E0 | 4.0E0 | 1.0E-9 | 1.0E-9 | 2.9E1 | 2.8E1 | 694 | 48 | 280 | 48 | 0.49 |
| Pa | pg/ml | 3.8E-1 | 4.8E-1 | 1.5E0 | 6.0E0 | 5.5E0 | 3.3E1 | 1.0E-9 | 1.0E-9 | 8.6E1 | 2.3E2 | 694 | 48 | 280 | 48 | 0.55 |
| Pb | pg/ml | 1.0E-9 | 1.0E-9 | 9.3E-1 | 7.7E-1 | 1.9E1 | 4.6E0 | 1.0E-9 | 1.0E-9 | 4.9E2 | 3.2E1 | 694 | 48 | 280 | 48 | 0.43 |
| Pc | pg/ml | 4.7E-2 | 7.0E-2 | 3.7E-1 | 1.2E0 | 9.4E-1 | 5.4E0 | 1.0E-9 | 1.0E-9 | 1.6E1 | 3.8E1 | 694 | 48 | 280 | 48 | 0.52 |
| Pd | pg/ml | 1.7E0 | 3.4E0 | 5.1E0 | 9.0E0 | 3.3E1 | 1.9E1 | 1.0E-9 | 1.0E-9 | 8.4E2 | 1.2E2 | 694 | 48 | 280 | 48 | 0.61 |
| Pe | pg/ml | 2.0E1 | 6.0E1 | 1.0E2 | 6.6E2 | 3.5E2 | 2.4E3 | 1.0E-9 | 1.0E-9 | 4.7E3 | 1.5E4 | 694 | 48 | 280 | 48 | 0.69 |
| Pf | pg/ml | 1.4E0 | 6.5E0 | 1.1E1 | 3.4E1 | 6.4E1 | 8.3E1 | 1.0E-9 | 1.0E-9 | 1.5E3 | 4.3E2 | 694 | 48 | 280 | 48 | 0.71 |
| Pg | pg/ml | 3.0E0 | 1.0E1 | 4.7E1 | 7.7E1 | 3.8E2 | 2.2E2 | 1.0E-9 | 1.0E-9 | 7.7E3 | 1.2E3 | 694 | 48 | 280 | 48 | 0.66 |
| Ph | ng/ml | 1.7E-1 | 2.3E-1 | 3.2E-1 | 5.2E-1 | 4.5E-1 | 1.0E0 | 1.0E-9 | 1.0E-9 | 2.9E0 | 5.4E0 | 249 | 32 | 160 | 32 | 0.55 |
| Pi | ng/ml | 2.0E-1 | 3.0E-1 | 2.8E-1 | 2.9E0 | 3.6E-1 | 1.4E1 | 1.0E-9 | 1.0E-9 | 3.2E0 | 8.2E1 | 249 | 32 | 160 | 32 | 0.59 |
| Pj | ng/mL | 4.8E0 | 7.3E0 | 5.9E0 | 9.1E0 | 4.5E0 | 9.4E0 | 3.8E-2 | 1.4E0 | 3.1E1 | 5.5E1 | 249 | 32 | 160 | 32 | 0.65 |
| Pk | ng/ml | 8.9E-3 | 1.0E-2 | 1.4E-2 | 5.9E-2 | 2.2E-2 | 2.7E-1 | 1.0E-9 | 1.0E-9 | 2.5E-1 | 1.5E0 | 249 | 32 | 160 | 32 | 0.53 |
| aA | mg/dL | 8.0E-1 | 1.0E0 | 9.2E-1 | 1.3E0 | 4.7E-1 | 9.8E-1 | 2.0E-1 | 4.0E-1 | 4.2E0 | 4.7E0 | 2240 | 64 | 436 | 64 | 0.63 |
| aC | mg/mL | 2.9E0 | 2.2E0 | 3.2E0 | 2.4E0 | 1.4E0 | 1.0E0 | 8.5E-1 | 7.4E-1 | 8.9E0 | 5.5E0 | 437 | 33 | 174 | 33 | 0.34 |
| aD | ug/mL | 3.1E0 | 4.5E0 | 4.4E0 | 6.4E0 | 3.9E0 | 5.5E0 | 4.3E-1 | 7.7E-1 | 3.5E1 | 2.1E1 | 437 | 33 | 174 | 33 | 0.59 |
| aE | mg/mL | 5.6E-1 | 5.1E-1 | 5.8E-1 | 5.6E-1 | 1.5E-1 | 1.8E-1 | 2.1E-1 | 2.2E-1 | 1.1E0 | 1.2E0 | 437 | 33 | 174 | 33 | 0.43 |
| aF | ng/mL | 2.1E0 | 2.6E0 | 4.1E0 | 3.5E0 | 6.1E0 | 3.5E0 | 4.3E-3 | 3.7E-1 | 5.0E1 | 1.3E1 | 437 | 33 | 174 | 33 | 0.49 |
| aG | mg/mL | 1.4E-1 | 1.3E-1 | 1.6E-1 | 1.5E-1 | 9.1E-2 | 8.5E-2 | 1.7E-2 | 4.3E-2 | 5.4E-1 | 4.2E-1 | 437 | 33 | 174 | 33 | 0.47 |
| aH | ug/mL | 7.5E1 | 7.5E1 | 8.3E1 | 7.9E1 | 4.4E1 | 4.6E1 | 4.6E0 | 1.1E1 | 2.9E2 | 1.9E2 | 437 | 33 | 174 | 33 | 0.48 |
| aI | ug/mL | 1.9E2 | 1.6E2 | 1.9E2 | 1.6E2 | 6.0E1 | 5.7E1 | 2.8E1 | 7.5E1 | 3.7E2 | 2.9E2 | 437 | 33 | 174 | 33 | 0.34 |
| aJ | ug/mL | 2.4E0 | 3.3E0 | 2.9E0 | 4.6E0 | 2.1E0 | 4.2E0 | 7.6E-1 | 1.1E0 | 1.7E1 | 2.3E1 | 437 | 33 | 174 | 33 | 0.65 |
| aK | ng/mL | 1.6E0 | 1.3E0 | 2.5E0 | 1.8E0 | 2.7E0 | 1.5E0 | 2.9E-4 | 1.3E-1 | 1.8E1 | 5.5E0 | 437 | 33 | 174 | 33 | 0.45 |
| aL | mg/mL | 8.1E-1 | 7.8E-1 | 8.2E-1 | 7.8E-1 | 2.5E-1 | 2.7E-1 | 1.9E-1 | 2.7E-1 | 1.7E0 | 1.5E0 | 437 | 33 | 174 | 33 | 0.46 |
| aM | U/mL | 2.2E1 | 3.2E1 | 4.5E1 | 7.1E1 | 9.6E1 | 1.4E2 | 4.2E-2 | 4.2E-2 | 1.6E3 | 8.2E2 | 437 | 33 | 174 | 33 | 0.59 |
| aN | U/mL | 1.3E1 | 2.0E1 | 2.0E1 | 3.1E1 | 2.8E1 | 2.8E1 | 2.5E-3 | 2.5E-3 | 3.8E2 | 1.1E2 | 437 | 33 | 174 | 33 | 0.64 |
| aO | pg/mL | 3.1E1 | 4.1E1 | 3.2E2 | 4.1E2 | 8.3E2 | 6.8E2 | 6.0E-2 | 6.0E-2 | 6.6E3 | 2.4E3 | 437 | 33 | 174 | 33 | 0.55 |
| aP | ng/mL | 1.6E0 | 1.9E0 | 2.0E0 | 3.2E0 | 1.8E0 | 4.7E0 | 4.5E-1 | 6.3E-1 | 2.8E1 | 2.8E1 | 437 | 33 | 174 | 33 | 0.59 |
| aQ | ng/mL | 3.0E-1 | 2.6E-1 | 4.7E-1 | 3.4E-1 | 4.8E-1 | 2.7E-1 | 2.0E-4 | 5.1E-2 | 4.0E0 | 1.2E0 | 437 | 33 | 174 | 33 | 0.43 |
| aR | ng/mL | 1.7E0 | 2.3E0 | 2.7E0 | 2.8E0 | 3.3E0 | 2.9E0 | 1.8E-1 | 2.5E-1 | 3.4E1 | 1.7E1 | 437 | 33 | 174 | 33 | 0.54 |
| aS | ng/mL | 2.6E-1 | 3.6E-1 | 6.5E-1 | 6.3E-1 | 1.9E0 | 7.4E-1 | 4.2E-3 | 6.0E-2 | 3.3E1 | 2.8E0 | 437 | 33 | 174 | 33 | 0.55 |
| aU | pg/mL | 7.8E1 | 6.1E1 | 1.3E2 | 7.5E1 | 1.5E2 | 6.0E1 | 7.4E-2 | 9.6E0 | 1.3E3 | 2.3E2 | 437 | 33 | 174 | 33 | 0.40 |
| aV | ng/mL | 6.3E-1 | 4.2E-1 | 1.1E0 | 7.7E-1 | 2.0E0 | 9.8E-1 | 7.6E-4 | 9.1E-2 | 3.3E1 | 5.4E0 | 437 | 33 | 174 | 33 | 0.42 |
| aW | pg/mL | 1.9E1 | 1.7E1 | 2.0E1 | 2.9E1 | 2.0E1 | 7.0E1 | 7.2E-2 | 7.2E-2 | 2.4E2 | 4.2E2 | 437 | 33 | 174 | 33 | 0.49 |
| aX | ng/mL | 9.4E0 | 1.2E1 | 1.5E1 | 2.0E1 | 1.9E1 | 3.5E1 | 3.0E-1 | 1.6E0 | 2.2E2 | 1.7E2 | 437 | 33 | 174 | 33 | 0.50 |
| aY | pg/mL | 5.4E1 | 6.1E1 | 7.7E1 | 7.6E1 | 8.7E1 | 5.5E1 | 4.1E-1 | 4.1E-1 | 1.2E3 | 2.0E2 | 437 | 33 | 174 | 33 | 0.54 |
| aZ | pg/mL | 2.2E2 | 3.2E2 | 5.0E2 | 4.6E2 | 9.8E2 | 5.9E2 | 1.7E0 | 1.7E0 | 1.2E4 | 2.6E3 | 437 | 33 | 174 | 33 | 0.54 |
| bA | ng/mL | 8.0E0 | 2.2E1 | 3.0E1 | 1.7E2 | 7.8E1 | 3.6E2 | 3.0E-2 | 3.0E-2 | 9.4E2 | 1.5E3 | 437 | 33 | 174 | 33 | 0.69 |
| bB | ng/mL | 3.1E2 | 2.4E2 | 3.4E2 | 2.6E2 | 1.7E2 | 1.7E2 | 2.1E0 | 2.3E1 | 1.0E3 | 6.3E2 | 437 | 33 | 174 | 33 | 0.37 |
| bC | ng/mL | 3.3E2 | 3.4E2 | 5.6E2 | 8.3E2 | 7.4E2 | 1.1E3 | 9.8E0 | 5.0E1 | 4.7E3 | 4.7E3 | 437 | 33 | 174 | 33 | 0.58 |
| bE | mg/mL | 5.5E0 | 5.4E0 | 5.8E0 | 5.8E0 | 2.0E0 | 2.4E0 | 9.8E-1 | 1.3E0 | 1.3E1 | 1.2E1 | 437 | 33 | 174 | 33 | 0.48 |
| bF | pg/mL | 1.9E1 | 4.3E1 | 1.6E2 | 3.9E2 | 9.8E2 | 1.2E3 | 5.0E-2 | 7.7E0 | 1.1E4 | 6.3E3 | 437 | 33 | 174 | 33 | 0.68 |
| bG | ng/mL | 1.6E0 | 1.7E0 | 2.7E0 | 3.1E0 | 3.3E0 | 5.2E0 | 2.2E-2 | 1.1E-1 | 2.6E1 | 3.0E1 | 437 | 33 | 174 | 33 | 0.52 |
| bH | pg/mL | 5.7E-1 | 5.7E-1 | 4.9E0 | 4.8E0 | 1.6E1 | 6.3E0 | 5.7E-1 | 5.7E-1 | 2.8E2 | 2.2E1 | 437 | 33 | 174 | 33 | 0.54 |
| bI | ng/mL | 4.0E-3 | 4.0E-3 | 6.5E-2 | 8.5E-2 | 1.6E-1 | 2.1E-1 | 4.0E-3 | 4.0E-3 | 1.3E0 | 8.8E-1 | 437 | 33 | 174 | 33 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bJ | mg/mL | 2.4E0 | 1.9E0 | 2.7E0 | 2.4E0 | 2.1E0 | 1.8E0 | 2.5E-4 | 2.5E-4 | 1.3E1 | 7.0E0 | 437 | 33 | 174 | 33 | 0.46 |
| bL | pg/mL | 4.0E0 | 3.1E0 | 8.3E0 | 5.9E0 | 1.1E1 | 7.1E0 | 4.6E-2 | 4.6E-2 | 8.0E1 | 3.2E1 | 437 | 33 | 174 | 33 | 0.46 |
| bM | mg/mL | 1.7E0 | 2.3E0 | 2.0E0 | 2.7E0 | 1.4E0 | 1.6E0 | 9.2E-3 | 1.8E-2 | 8.8E0 | 8.4E0 | 437 | 33 | 174 | 33 | 0.64 |
| bN | ng/mL | 4.3E1 | 3.3E1 | 1.4E2 | 8.2E1 | 2.9E2 | 1.3E2 | 1.4E-1 | 5.9E-1 | 1.9E3 | 4.8E2 | 437 | 33 | 174 | 33 | 0.44 |
| bO | ng/mL | 4.0E-2 | 4.0E-2 | 1.0E1 | 9.7E0 | 2.3E1 | 1.9E1 | 4.0E-2 | 4.0E-2 | 1.9E2 | 6.6E1 | 437 | 33 | 174 | 33 | 0.48 |
| bP | mg/mL | 5.3E-1 | 6.8E-1 | 7.6E-1 | 8.4E-1 | 6.8E-1 | 7.4E-1 | 4.9E-2 | 1.4E-1 | 4.8E0 | 3.5F0 | 437 | 33 | 174 | 33 | 0.54 |
| bQ | pg/mL | 1.5E1 | 2.3E1 | 6.4E1 | 4.9E1 | 6.5E2 | 5.6E1 | 1.5E-1 | 1.5E-1 | 1.3E4 | 2.2E2 | 437 | 33 | 174 | 33 | 0.62 |
| bR | ng/mL | 1.2E-2 | 1.2E-2 | 1.4E-1 | 6.0E-2 | 4.6E-1 | 9.7E-2 | 1.2E-2 | 1.2E-2 | 8.7E0 | 3.9E-1 | 437 | 33 | 174 | 33 | 0.40 |
| bS | pg/mL | 9.4E-1 | 9.4E-1 | 7.2E0 | 3.8E0 | 2.9E1 | 1.2E1 | 9.4E-1 | 9.4E-1 | 3.9E2 | 6.7E1 | 437 | 33 | 174 | 33 | 0.46 |
| bU | ng/mL | 1.5E-1 | 1.3E-2 | 2.0E-1 | 1.2E-1 | 3.8E-1 | 1.5E-1 | 1.3E-2 | 1.3E-2 | 6.6E0 | 6.3E-1 | 437 | 33 | 174 | 33 | 0.41 |
| bV | pg/mL | 4.7E2 | 5.6E2 | 5.5E2 | 7.3E2 | 5.8E2 | 5.8E2 | 1.6E2 | 1.9E2 | 1.2E4 | 3.1E3 | 437 | 33 | 174 | 33 | 0.59 |
| bW | pg/mL | 3.2E2 | 3.2E2 | 4.9E2 | 1.4E3 | 4.8E2 | 4.3E3 | 8.4E1 | 1.2E2 | 4.8E3 | 2.5E4 | 437 | 33 | 174 | 33 | 0.55 |
| bX | ng/mL | 1.5E-3 | 2.5E-5 | 2.8E-3 | 2.0E-3 | 3.5E-3 | 2.5E-3 | 2.5E-5 | 2.5E-5 | 2.0E-2 | 7.1E-3 | 437 | 33 | 174 | 33 | 0.45 |
| bZ | pg/mL | 2.3E2 | 3.6E2 | 9.1E2 | 2.2E3 | 4.1E3 | 7.3E3 | 1.5E-1 | 1.5E1 | 5.8E4 | 4.3E4 | 437 | 33 | 174 | 33 | 0.66 |
| cA | pg/mL | 6.0E-1 | 6.0E-1 | 2.8E0 | 1.0E0 | 1.8E1 | 2.5E0 | 6.0E-1 | 6.0E-1 | 3.7E2 | 1.5E1 | 437 | 33 | 174 | 33 | 0.45 |
| cB | ng/mL | 6.0E-2 | 4.2E-2 | 9.3E-2 | 6.5E-2 | 1.0E-1 | 8.5E-2 | 1.7E-3 | 1.7E-3 | 5.7E-1 | 4.0E-1 | 437 | 33 | 174 | 33 | 0.41 |
| cC | pg/mL | 4.6E1 | 4.6E1 | 4.8E1 | 3.8E1 | 4.0E1 | 2.6E1 | 1.0E0 | 1.0E0 | 4.5E2 | 7.3E1 | 437 | 33 | 174 | 33 | 0.44 |
| cD | pg/mL | 5.4E0 | 4.9E0 | 1.5E1 | 1.2E1 | 5.7E1 | 1.9E1 | 3.3E-1 | 3.3E-1 | 7.2E2 | 9.0E1 | 437 | 33 | 174 | 33 | 0.50 |
| cE | pg/mL | 3.2E1 | 8.5E1 | 1.5E2 | 2.3E2 | 4.6E2 | 3.4E2 | 1.2E-1 | 1.7E0 | 3.8E3 | 1.3E3 | 437 | 33 | 174 | 33 | 0.67 |
| cF | pg/mL | 1.3E1 | 5.3E-1 | 2.1E1 | 1.0E1 | 3.2E1 | 1.4E1 | 5.3E-1 | 5.3E-1 | 2.7E2 | 5.0E1 | 437 | 33 | 174 | 33 | 0.40 |
| cG | pg/mL | 4.3E1 | 8.1E1 | 1.1E2 | 1.2E2 | 5.2E2 | 1.2E2 | 7.8E0 | 6.4E0 | 1.0E4 | 4.9E2 | 437 | 33 | 174 | 33 | 0.70 |
| cH | uIU/mL | 3.1E0 | 2.2E0 | 6.5E0 | 6.3E0 | 1.2E1 | 1.1E1 | 8.6E-3 | 8.6E-3 | 1.6E2 | 5.3E1 | 437 | 33 | 174 | 33 | 0.41 |
| cI | ng/mL | 5.7E0 | 9.4E0 | 1.1E1 | 1.7E1 | 1.5E1 | 2.4E1 | 1.0E-3 | 1.0E-3 | 1.0E2 | 1.2E2 | 437 | 33 | 174 | 33 | 0.55 |
| cJ | ug/mL | 7.0E1 | 4.6E1 | 1.2E2 | 8.3E1 | 1.5E2 | 9.9E1 | 4.0E0 | 5.6E0 | 9.6E2 | 3.4E2 | 437 | 33 | 174 | 33 | 0.41 |
| cK | ng/mL | 3.8E-3 | 3.8E-3 | 5.5E-2 | 1.1E-2 | 1.9E-1 | 2.0E-2 | 3.8E-3 | 3.8E-3 | 1.7E0 | 8.2E-2 | 437 | 33 | 174 | 33 | 0.47 |
| cL | pg/mL | 1.9E2 | 2.0E2 | 3.6E2 | 2.9E2 | 1.3E3 | 2.5E2 | 1.6E1 | 4.8E1 | 2.4E4 | 1.4E3 | 437 | 33 | 174 | 33 | 0.58 |
| cM | pg/mL | 2.8E2 | 2.6E2 | 3.1E2 | 2.6E2 | 2.0E2 | 1.2E2 | 8.7E0 | 5.7E1 | 1.6E3 | 5.1E2 | 437 | 33 | 174 | 33 | 0.45 |
| cN | pg/mL | 1.2E2 | 1.3E2 | 1.3E2 | 1.4E2 | 6.4E1 | 5.5E1 | 3.8E1 | 7.4E1 | 1.1E3 | 2.9E2 | 437 | 33 | 174 | 33 | 0.61 |
| cO | pg/mL | 2.2E2 | 2.4E2 | 3.1E2 | 2.5E2 | 9.3E2 | 8.9E1 | 5.4E1 | 1.2E2 | 1.9E4 | 4.4E2 | 437 | 33 | 174 | 33 | 0.54 |
| cP | ng/mL | 2.5E3 | 2.4E3 | 2.6E3 | 2.8E3 | 9.2E2 | 1.1E3 | 6.2E2 | 1.3E3 | 5.7E3 | 5.6E3 | 437 | 33 | 174 | 33 | 0.52 |
| cQ | ng/mL | 4.3E-2 | 5.5E-2 | 1.3E-1 | 1.7E-1 | 2.4E-1 | 2.9E-1 | 2.0E-3 | 2.0E-3 | 2.2E0 | 1.2E0 | 437 | 33 | 174 | 33 | 0.52 |
| cR | ng/mL | 2.8E2 | 4.2E2 | 4.9E2 | 5.0E2 | 8.0E2 | 4.2E2 | 2.0E1 | 7.0E1 | 8.9E3 | 2.0E3 | 437 | 33 | 174 | 33 | 0.59 |
| cS | ng/mL | 2.6E2 | 3.2E2 | 3.8E2 | 6.9E2 | 3.8E2 | 1.3E3 | 4.1E1 | 4.8E1 | 2.7E3 | 7.1E3 | 437 | 33 | 174 | 33 | 0.56 |
| cT | ng/mL | 2.9E1 | 7.8E1 | 8.3E1 | 2.6E2 | 1.8E2 | 4.8E2 | 3.6E0 | 4.0E0 | 2.1E3 | 1.9E3 | 437 | 33 | 174 | 33 | 0.65 |
| cU | ng/mL | 5.4E1 | 6.4E1 | 7.5E1 | 9.8E1 | 9.9E1 | 9.3E1 | 6.2E0 | 1.6E1 | 1.6E3 | 4.2E2 | 437 | 33 | 174 | 33 | 0.58 |
| cV | ng/mL | 1.7E-1 | 2.4E-1 | 3.8E-1 | 4.1E-1 | 2.3E0 | 4.7E-1 | 3.4E-4 | 3.6E-2 | 4.7E1 | 2.5E0 | 437 | 33 | 174 | 33 | 0.65 |
| cW | mIU/mL | 5.3E-2 | 4.2E-2 | 1.5E-1 | 8.1E-2 | 7.2E-1 | 8.5E-2 | 3.7E-4 | 1.4E-2 | 9.7E0 | 3.9E-1 | 437 | 33 | 174 | 33 | 0.50 |
| cX | ug/mL | 9.9E-2 | 1.2E-1 | 1.3E0 | 8.7E-1 | 4.2E0 | 1.6E0 | 2.3E-4 | 2.3E-4 | 2.8E1 | 5.4E0 | 437 | 33 | 174 | 33 | 0.49 |
| cY | ng/mL | 8.9E0 | 9.0E0 | 1.3E1 | 9.5E0 | 1.3E1 | 7.2E0 | 1.5E-1 | 9.3E-1 | 8.3E1 | 3.1E1 | 437 | 33 | 174 | 33 | 0.45 |
| cZ | ug/mL | 1.5E1 | 1.3E1 | 1.6E1 | 1.4E1 | 7.1E0 | 6.7E0 | 2.3E0 | 3.3E0 | 5.7E1 | 3.1E1 | 437 | 33 | 174 | 33 | 0.43 |
| dA | pg/mL | 3.3E2 | 3.6E2 | 3.8E2 | 3.8E2 | 3.1E2 | 2.0E2 | 9.0E1 | 1.5E2 | 5.8E3 | 1.1E3 | 437 | 33 | 174 | 33 | 0.51 |
| dB | ug/mL | 1.7E1 | 1.9E1 | 1.7E1 | 1.6E1 | 1.6E1 | 1.0E1 | 9.4E-1 | 2.2E0 | 2.5E2 | 3.0E1 | 437 | 33 | 174 | 33 | 0.53 |
| dC | nmol/L | 3.5E1 | 3.8E1 | 3.9E1 | 3.9E1 | 1.8E1 | 1.5E1 | 7.9E0 | 1.6E1 | 1.4E2 | 7.9E1 | 437 | 33 | 174 | 33 | 0.53 |
| dD | ug/mL | 3.7E1 | 3.0E1 | 3.8E1 | 3.4E1 | 1.1E1 | 1.2E1 | 1.3E1 | 1.4E1 | 7.6E1 | 6.4E1 | 437 | 33 | 174 | 33 | 0.37 |
| dE | ng/mL | 4.7E-1 | 2.3E-1 | 6.1E-1 | 4.6E-1 | 7.3E-1 | 6.5E-1 | 8.4E-3 | 8.4E-3 | 7.2E0 | 2.4E0 | 437 | 33 | 174 | 33 | 0.41 |
| dF | ng/mL | 2.2E2 | 2.8E2 | 2.6E2 | 4.3E2 | 1.8E2 | 3.2E2 | 7.5E1 | 8.4E1 | 1.3E3 | 1.2E3 | 437 | 33 | 174 | 33 | 0.69 |
| dG | ng/mL | 1.1E1 | 1.6E1 | 1.4E1 | 2.2E1 | 1.2E1 | 1.9E1 | 2.5E0 | 3.9E0 | 1.8E2 | 8.7E1 | 437 | 33 | 174 | 33 | 0.67 |
| dH | pg/mL | 7.5E0 | 1.1E1 | 1.3E1 | 1.4E1 | 4.0E1 | 1.4E1 | 4.0E-2 | 4.0E-2 | 6.7E2 | 7.6E1 | 437 | 33 | 174 | 33 | 0.61 |
| dI | pg/mL | 4.6E-1 | 4.6E-1 | 2.3E0 | 2.2E0 | 1.6E1 | 3.7E0 | 4.6E-1 | 4.6E-1 | 3.3E2 | 1.9E1 | 437 | 33 | 174 | 33 | 0.56 |
| dJ | ng/mL | 1.9E0 | 1.9E0 | 2.2E0 | 1.9E0 | 1.2E0 | 1.1E0 | 3.2E-2 | 3.2E-2 | 6.9E0 | 3.7E0 | 437 | 33 | 174 | 33 | 0.44 |
| dK | uIU/mL | 1.9E0 | 2.1E0 | 3.1E0 | 2.7E0 | 6.0E0 | 3.9E0 | 2.8E-4 | 1.4E-2 | 7.9E1 | 2.2E1 | 437 | 33 | 174 | 33 | 0.47 |
| dL | ng/mL | 8.7E2 | 1.2E3 | 1.0E3 | 1.3E3 | 4.9E2 | 9.1E2 | 3.4E2 | 4.1E2 | 3.4E3 | 4.8E3 | 437 | 33 | 174 | 33 | 0.61 |
| dM | pg/mL | 9.6E2 | 1.1E3 | 1.1E3 | 1.9E3 | 8.8E2 | 1.9E3 | 3.5E2 | 3.9E2 | 1.2E4 | 9.6E3 | 437 | 33 | 174 | 33 | 0.63 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dN | ug/mL | 9.3E1 | 1.1E2 | 9.9E1 | 1.3E2 | 3.6E1 | 6.4E1 | 2.5E1 | 3.4E1 | 2.8E2 | 3.3E2 | 437 | 33 | 174 | 33 | 0.62 |
| dR | pg/ml | 1.6E3 | 1.1E3 | 2.4E3 | 1.8E3 | 2.4E3 | 1.7E3 | 1.4E2 | 3.4E2 | 1.5E4 | 7.3E3 | 287 | 30 | 167 | 30 | 0.42 |
| dU | pg/ml | 9.7E3 | 1.6E4 | 1.3E4 | 1.6E4 | 1.2E4 | 1.2E4 | 6.9E2 | 3.1E3 | 5.3E4 | 3.8E4 | 46 | 12 | 43 | 12 | 0.57 |
| dX | ng/ml | 8.1E-2 | 5.2E-2 | 1.2E-1 | 1.2E-1 | 1.9E-1 | 1.5E-1 | 2.6E-3 | 2.6E-3 | 1.2E0 | 4.2E-1 | 121 | 9 | 43 | 9 | 0.49 |
| cF | ng/ml | 4.0E0 | 4.8E0 | 4.9E0 | 6.3E0 | 4.2E0 | 5.2E0 | 1.2E0 | 2.0E0 | 4.6E1 | 2.9E1 | 302 | 30 | 168 | 30 | 0.63 |
| eC | pg/ml | 3.1E2 | 2.6E2 | 3.6E2 | 3.5E2 | 2.2E2 | 4.2E2 | 9.9E0 | 1.9E1 | 1.4E3 | 2.0E3 | 231 | 27 | 159 | 27 | 0.37 |
| eD | pg/ml | 2.3E2 | 2.1E2 | 5.8E2 | 3.9E2 | 1.2E3 | 8.2E2 | 5.2E-1 | 5.2E-1 | 8.3E3 | 3.8E3 | 197 | 19 | 131 | 19 | 0.44 |
| eM | ng/ml | 3.2E0 | 2.5E0 | 4.9E0 | 4.8E0 | 5.5E0 | 6.3E0 | 3.3E-1 | 7.1E-1 | 3.9E1 | 2.6E1 | 155 | 18 | 67 | 18 | 0.42 |
| eP | ng/ml | 3.7E-3 | 4.5E-1 | 6.4E-1 | 3.5E0 | 1.6E0 | 9.1E0 | 3.7E-3 | 3.7E-3 | 1.2E1 | 2.8E1 | 121 | 9 | 43 | 9 | 0.61 |
| eT | ng/ml | 2.8E2 | 6.1E2 | 6.3E2 | 9.6E2 | 7.2E2 | 9.4E2 | 1.0E2 | 7.1E1 | 2.9E3 | 2.9E3 | 112 | 12 | 94 | 12 | 0.61 |
| eQ | pg/ml | 1.0E0 | 1.0E0 | 7.9E1 | 1.8E1 | 2.5E2 | 4.4E1 | 1.0E0 | 1.0E0 | 1.6E3 | 1.5E2 | 46 | 12 | 43 | 12 | 0.43 |
| fA | ng/ml | 2.0E2 | 1.1E2 | 4.0E2 | 3.6E2 | 4.4E2 | 4.2E2 | 2.6E1 | 6.2E1 | 1.5E3 | 1.2E3 | 46 | 12 | 43 | 12 | 0.45 |
| eZ | ng/ml | 5.1E1 | 6.0E1 | 5.8E1 | 6.6E1 | 2.5E1 | 2.5E1 | 1.8E1 | 3.2E1 | 1.2E2 | 1.1E2 | 112 | 12 | 94 | 12 | 0.61 |
| fB | ng/ml | 6.0E2 | 6.6E2 | 6.9E2 | 7.2E2 | 3.0E2 | 2.6E2 | 1.6E2 | 3.5E2 | 1.5E3 | 1.3E3 | 46 | 14 | 43 | 14 | 0.56 |
| fN | ng/ml | 2.1E-1 | 3.4E0 | 3.1E0 | 4.9E0 | 7.2E0 | 5.4E0 | 2.1E-1 | 2.1E-1 | 5.4E1 | 1.4E1 | 112 | 12 | 94 | 12 | 0.64 |
| fP | ng/ml | 2.4E2 | 2.8E2 | 2.8E2 | 3.3E2 | 1.7E2 | 2.1E2 | 1.8E0 | 1.6E1 | 1.0E3 | 8.6E2 | 276 | 29 | 162 | 29 | 0.57 |
| fR | ng/ml | 1.2E5 | 2.0E5 | 1.7E5 | 3.0E5 | 1.4E5 | 2.3E5 | 3.1E4 | 1.9E2 | 7.7E5 | 8.7E5 | 295 | 20 | 90 | 20 | 0.68 |
| fY | ng/ml | 2.7E2 | 2.5E2 | 2.6E2 | 2.6E2 | 1.1E2 | 1.0E2 | 3.6E1 | 1.2E2 | 4.8E2 | 4.3E2 | 112 | 12 | 94 | 12 | 0.47 |
| gC | ng/ml | 2.3E2 | 2.7E2 | 2.6E2 | 2.9E2 | 1.3E2 | 1.1E2 | 8.3E1 | 1.6E2 | 1.1E3 | 5.9E2 | 127 | 16 | 74 | 16 | 0.61 |
| gL | pg/ml | 6.3E4 | 6.7E4 | 7.0E4 | 8.0E4 | 3.1E4 | 4.2E4 | 1.4E4 | 4.1E4 | 2.0E5 | 2.2E5 | 287 | 30 | 167 | 30 | 0.56 |
| gP | U/ml | 2.8E2 | 2.5E2 | 2.9E2 | 2.8E2 | 1.1E2 | 1.0E2 | 1.2E1 | 9.6E1 | 1.1E3 | 5.2E2 | 298 | 30 | 168 | 30 | 0.47 |
| gW | ng/ml | 6.8E2 | 5.1E2 | 1.3E3 | 1.0E3 | 1.7E3 | 1.3E3 | 3.1E-1 | 1.5E2 | 9.5E3 | 4.3E3 | 252 | 19 | 157 | 19 | 0.48 |
| gV | ng/ml | 2.1E1 | 2.2E1 | 2.2E1 | 2.4E1 | 7.2E0 | 6.4E0 | 2.9E-3 | 1.7E1 | 3.9E1 | 3.4E1 | 109 | 7 | 27 | 7 | 0.63 |
| tF | pg/mL | 1.4E3 | 9.0E2 | 1.5E4 | 3.8E3 | 4.5E4 | 5.7E3 | 1.2E1 | 1.8E1 | 3.2E5 | 2.0E4 | 231 | 27 | 159 | 27 | 0.49 |
| gZ | ug/ml | 9.3E-1 | 9.3E-1 | 4.8E1 | 8.4E1 | 1.1E2 | 1.6E2 | 8.7E-2 | 1.1E-1 | 4.1E2 | 4.1E2 | 46 | 12 | 43 | 12 | 0.56 |
| hA | ng/ml | 2.0E0 | 3.6E0 | 9.2E0 | 6.8E0 | 3.7E1 | 6.9E0 | 1.7E-2 | 6.3E-2 | 3.5E2 | 2.5E1 | 197 | 21 | 131 | 21 | 0.65 |
| nM | pg/ml | 1.0E-9 | 1.0E-9 | 7.9E1 | 1.0E-9 | 9.0E2 | 0.0E0 | 1.0E-9 | 1.0E-9 | 1.0E4 | 1.0E-9 | 131 | 21 | 103 | 21 | 0.49 |
| nN | pg/ml | 1.2E3 | 2.1E3 | 5.1E3 | 3.7E3 | 2.5E4 | 4.5E3 | 1.1E2 | 2.8E2 | 2.7E5 | 2.1E4 | 131 | 21 | 103 | 21 | 0.65 |
| nO | pg/ml | 2.7E1 | 2.9E1 | 4.3E1 | 5.0E1 | 4.2E1 | 5.5E1 | 3.5E0 | 9.7E0 | 2.4E2 | 2.4E2 | 131 | 21 | 103 | 21 | 0.54 |
| nR | pg/ml | 1.3E1 | 2.0E1 | 3.8E1 | 7.7E1 | 8.7E1 | 1.7E2 | 1.0E-9 | 1.9E0 | 8.2E2 | 7.1E2 | 131 | 21 | 103 | 21 | 0.59 |
| nT | pg/ml | 8.5E1 | 8.8E1 | 2.2E2 | 1.0E2 | 8.0E2 | 6.6E1 | 1.0E-9 | 1.0E-9 | 6.6E3 | 3.4E2 | 131 | 21 | 103 | 21 | 0.53 |
| nU | pg/ml | 2.9E1 | 2.9E1 | 2.6E2 | 8.5E1 | 1.5E3 | 1.3E2 | 1.0E-9 | 1.0E-9 | 1.3E4 | 4.4E2 | 131 | 21 | 103 | 21 | 0.50 |
| lW | pg/ml | 1.0E-9 | 1.0E-9 | 1.3E1 | 5.3E0 | 4.7E1 | 1.1E1 | 1.0E-9 | 1.0E-9 | 3.9E2 | 4.4E1 | 131 | 21 | 103 | 21 | 0.49 |
| lX | pg/ml | 1.0E3 | 7.5E2 | 1.1E3 | 7.6E2 | 5.6E2 | 3.9E2 | 1.2E2 | 1.9E2 | 2.6E3 | 1.6E3 | 131 | 21 | 103 | 21 | 0.33 |
| lY | pg/ml | 2.0E1 | 1.6E1 | 2.3E1 | 1.4E1 | 2.1E1 | 8.2E0 | 1.0E-9 | 5.7E-1 | 1.4E2 | 3.0E1 | 131 | 21 | 103 | 21 | 0.35 |
| mE | pg/ml | 1.0E-9 | 1.0E-9 | 2.9E0 | 1.5E0 | 8.3E0 | 2.8E0 | 1.0E-9 | 1.0E-9 | 5.8E1 | 9.2E0 | 131 | 21 | 103 | 21 | 0.48 |
| mF | pg/ml | 1.0E-9 | 2.7E-1 | 4.0E0 | 2.5E0 | 2.3E1 | 4.2E0 | 1.0E-9 | 1.0E-9 | 2.5E2 | 1.3E1 | 131 | 21 | 103 | 21 | 0.55 |
| mH | pg/ml | 3.6E0 | 2.6E0 | 5.1E0 | 4.3E0 | 6.7E0 | 4.6E0 | 2.3E-1 | 5.4E-1 | 5.3E1 | 1.9E1 | 131 | 21 | 103 | 21 | 0.44 |
| mI | pg/ml | 1.0E-9 | 2.6E0 | 1.2E1 | 1.5E1 | 2.7E1 | 2.3E1 | 1.0E-9 | 1.0E-9 | 1.8E2 | 7.8E1 | 131 | 21 | 103 | 21 | 0.55 |
| mM | pg/ml | 2.2E1 | 3.8E1 | 6.5E1 | 5.1E1 | 1.5E2 | 6.7E1 | 1.0E-9 | 1.0E-9 | 1.1E3 | 2.9E2 | 131 | 21 | 103 | 21 | 0.55 |
| mP | pg/ml | 1.4E1 | 1.5E1 | 1.8E1 | 1.4E1 | 2.3E1 | 7.9E0 | 1.0E-9 | 1.0E-9 | 1.9E2 | 3.2E1 | 130 | 21 | 102 | 21 | 0.48 |
| mS | pg/ml | 1.8E3 | 1.4E3 | 2.0E3 | 1.6E3 | 1.6E3 | 7.4E2 | 1.0E-9 | 1.0E-9 | 1.3E4 | 2.7E3 | 131 | 21 | 103 | 21 | 0.41 |
| mT | pg/ml | 4.8E1 | 5.6E1 | 1.2E2 | 6.5E1 | 2.1E2 | 3.6E1 | 9.7E0 | 1.2E1 | 1.4E3 | 1.4E2 | 130 | 21 | 102 | 21 | 0.52 |
| mU | pg/ml | 2.2E0 | 3.1E0 | 5.5E0 | 3.1E0 | 2.1E1 | 1.7E0 | 1.0E-9 | 6.0E-1 | 2.2E2 | 6.6E0 | 130 | 21 | 102 | 21 | 0.60 |
| mW | pg/ml | 2.3E3 | 2.2E3 | 2.6E3 | 2.7E3 | 1.4E3 | 2.4E3 | 3.1E2 | 1.7E2 | 1.0E4 | 1.1E4 | 130 | 21 | 102 | 21 | 0.48 |
| mY | pg/ml | 5.6E2 | 6.5E2 | 8.6E2 | 8.8E2 | 1.3E3 | 7.2E2 | 1.0E-9 | 1.0E-9 | 1.1E4 | 2.6E3 | 131 | 21 | 103 | 21 | 0.57 |
| mZ | pg/ml | 2.2E2 | 1.5E2 | 3.8E2 | 3.8E2 | 4.4E2 | 4.1E2 | 1.0E-9 | 1.1E1 | 3.1E3 | 1.4E3 | 130 | 21 | 102 | 21 | 0.44 |
| nA | pg/ml | 2.0E0 | 4.8E-1 | 1.1E1 | 4.9E0 | 4.4E1 | 1.2E1 | 1.0E-9 | 1.0E-9 | 4.4E2 | 5.5E1 | 130 | 21 | 102 | 21 | 0.40 |
| nB | pg/ml | 3.0E2 | 3.1E2 | 3.1E2 | 3.3E2 | 1.5E2 | 1.9E2 | 3.0E1 | 7.9E1 | 8.2E2 | 1.0E3 | 131 | 21 | 103 | 21 | 0.52 |
| nC | pg/ml | 1.0E-9 | 8.3E1 | 3.7E3 | 7.5E4 | 3.2E4 | 3.3E5 | 1.0E-9 | 1.0E-9 | 3.7E5 | 1.5E6 | 131 | 21 | 103 | 21 | 0.61 |
| nD | pg/ml | 8.5E0 | 5.1E0 | 3.5E1 | 1.4E1 | 2.0E2 | 2.8E1 | 1.0E-9 | 1.0E-9 | 1.7E3 | 1.3E2 | 130 | 21 | 102 | 21 | 0.46 |
| nF | pg/ml | 1.0E-9 | 1.0E-9 | 5.0E0 | 3.8E0 | 2.6E1 | 1.0E1 | 1.0E-9 | 1.0E-9 | 2.4E2 | 3.8E1 | 131 | 21 | 103 | 21 | 0.51 |
| nH | pg/ml | 3.8E-1 | 2.5E0 | 8.9E1 | 1.5E2 | 8.8E2 | 5.7E2 | 1.0E-9 | 1.0E-9 | 1.0E4 | 2.6E3 | 130 | 21 | 102 | 21 | 0.59 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| nI | pg/ml | 4.6E1 | 1.0E-9 | 1.6E2 | 6.2E1 | 8.4E2 | 1.7E2 | 1.0E-9 | 1.0E-9 | 9.2E3 | 7.9E2 | 131 | 21 | 103 | 21 | 0.37 |
| nJ | pg/ml | 1.7E-1 | 1.7E-1 | 4.1E1 | 1.2E0 | 4.5E2 | 3.5E0 | 1.0E-9 | 1.0E-9 | 5.2E3 | 1.6E1 | 131 | 21 | 103 | 21 | 0.45 |
| nK | pg/ml | 1.0E-9 | 1.0E-9 | 5.8E1 | 2.0E1 | 3.4E2 | 5.0E1 | 1.0E-9 | 1.0E-9 | 3.2E3 | 2.3E2 | 130 | 21 | 102 | 21 | 0.52 |
| nL | pg/ml | 1.0E-9 | 3.4E0 | 3.9E2 | 3.3E2 | 3.9E3 | 1.1E3 | 1.0E-9 | 1.0E-9 | 4.5E4 | 5.2E3 | 131 | 21 | 103 | 21 | 0.60 |
| hL | pg/ml | 1.8E4 | 2.6E4 | 2.3E4 | 2.7E4 | 1.8E4 | 1.7E4 | 1.0E-9 | 1.0E-9 | 1.4E5 | 6.0E4 | 112 | 12 | 94 | 12 | 0.59 |
| hO | pg/ml | 1.6E4 | 1.5E4 | 1.7E4 | 1.6E4 | 3.2E3 | 4.5E3 | 1.1E4 | 1.1E4 | 2.8E4 | 2.7E4 | 112 | 12 | 94 | 12 | 0.41 |
| hP | ng/ml | 4.4E5 | 4.4E5 | 4.1E5 | 5.2E5 | 3.3E5 | 7.4E5 | 1.7E4 | 2.5E4 | 2.6E6 | 2.8E6 | 112 | 12 | 94 | 12 | 0.46 |
| wJ | pg/ml | 1.5E5 | 1.0E5 | 1.7E5 | 1.2E5 | 1.0E5 | 7.9E4 | 1.1E4 | 2.3E4 | 5.8E5 | 2.5E5 | 113 | 9 | 91 | 9 | 0.33 |
| wK | pg/ml | 3.5E4 | 3.5E4 | 4.8E4 | 4.0E4 | 5.4E4 | 3.3E4 | 3.7E3 | 1.1E4 | 5.0E5 | 1.2E5 | 113 | 9 | 91 | 9 | 0.44 |
| wL | pg/ml | 3.9E0 | 5.3E0 | 4.1E1 | 4.9E1 | 1.1E2 | 1.1E2 | 1.0E-9 | 1.0E-9 | 8.4E2 | 3.5E2 | 113 | 9 | 91 | 9 | 0.49 |
| wP | pg/ml | 2.6E4 | 7.0E4 | 4.2E4 | 8.7E4 | 4.6E4 | 8.1E4 | 1.1E3 | 1.8E4 | 3.0E5 | 2.6E5 | 113 | 9 | 91 | 9 | 0.73 |
| wQ | pg/ml | 3.7E1 | 2.9E1 | 6.1E1 | 4.0E1 | 8.1E1 | 4.9E1 | 1.0E-9 | 1.0E-9 | 5.1E2 | 1.6E2 | 113 | 9 | 91 | 9 | 0.45 |
| hR | pg/ml | 2.6E4 | 2.7E4 | 2.7E4 | 2.8E4 | 1.1E4 | 9.3E3 | 1.1E1 | 1.3E4 | 5.8E4 | 4.4E4 | 189 | 20 | 129 | 20 | 0.53 |
| hV | pg/ml | 4.4E2 | 3.6E2 | 4.7E2 | 5.0E2 | 2.4E2 | 5.3E2 | 6.8E1 | 9.5E1 | 1.5E3 | 2.5E3 | 189 | 20 | 129 | 20 | 0.42 |
| hW | pg/ml | 1.5E3 | 2.2E3 | 2.0E3 | 4.0E3 | 1.6E3 | 8.5E3 | 2.2E2 | 7.9E2 | 1.0E4 | 4.0E4 | 189 | 20 | 129 | 20 | 0.65 |
| hX | pg/ml | 9.0E2 | 1.2E3 | 1.0E3 | 1.2E3 | 7.8E2 | 5.7E2 | 1.3E2 | 3.1E2 | 8.6E3 | 2.9E3 | 189 | 20 | 129 | 20 | 0.60 |
| iA | pg/ml | 1.4E2 | 1.8E2 | 2.8E2 | 3.3E2 | 6.2E2 | 2.7E2 | 5.8E0 | 1.7E1 | 7.1E3 | 8.7E2 | 231 | 26 | 159 | 26 | 0.63 |
| iB | ng/ml | 4.8E0 | 7.7E0 | 6.0E0 | 9.0E0 | 4.9E0 | 6.0E0 | 3.3E-2 | 1.1E0 | 3.8E1 | 2.2E1 | 197 | 21 | 131 | 21 | 0.66 |
| iC | U/ml | 2.1E-1 | 6.7E-1 | 9.0E-1 | 1.3E0 | 4.6E0 | 2.6E0 | 1.0E-9 | 6.8E-2 | 5.5E1 | 1.2E1 | 197 | 21 | 131 | 21 | 0.69 |
| tQ | pg/ml | 1.1E3 | 1.3E3 | 1.2E3 | 1.5E3 | 5.5E2 | 8.8E2 | 2.8E2 | 6.3E2 | 2.5E3 | 3.3E3 | 108 | 8 | 88 | 8 | 0.55 |
| tT | pg/ml | 1.7E1 | 2.6E1 | 2.0E1 | 2.9E1 | 1.5E1 | 2.1E1 | 5.4E0 | 7.2E0 | 1.2E2 | 6.7E1 | 109 | 8 | 89 | 8 | 0.59 |
| tS | pg/ml | 1.1E0 | 1.1E0 | 1.3E0 | 1.5E0 | 1.3E0 | 2.2E0 | 1.0E-9 | 1.0E-9 | 8.5E0 | 7.0E0 | 109 | 9 | 89 | 9 | 0.47 |
| tX | pg/ml | 9.9E-1 | 1.2E0 | 1.2E0 | 2.3E0 | 9.8E-1 | 2.4E0 | 2.5E-2 | 9.3E-2 | 7.0E0 | 6.2E0 | 109 | 8 | 89 | 8 | 0.59 |
| tO | pg/ml | 4.4E0 | 3.9E0 | 5.1E0 | 4.5E0 | 3.3E0 | 2.7E0 | 1.0E-9 | 1.7E0 | 1.8E1 | 9.6E0 | 109 | 9 | 89 | 9 | 0.45 |
| tR | pg/ml | 2.1E-1 | 2.2E-1 | 2.9E-1 | 3.3E-1 | 2.9E-1 | 3.9E-1 | 1.0E-9 | 1.4E-2 | 1.6E0 | 1.3E0 | 108 | 9 | 88 | 9 | 0.51 |
| tU | pg/ml | 9.1E0 | 1.2E1 | 1.1E1 | 1.9E1 | 6.9E0 | 2.3E1 | 2.2E-1 | 5.0E0 | 4.4E1 | 8.0E1 | 111 | 9 | 90 | 9 | 0.61 |
| tN | pg/ml | 1.8E1 | 3.2E1 | 2.3E1 | 3.3E1 | 1.9E1 | 2.0E1 | 1.0E-9 | 6.1E0 | 1.5E2 | 6.5E1 | 109 | 8 | 88 | 8 | 0.66 |
| tV | ng/ml | 4.7E2 | 8.7E2 | 6.4E2 | 9.2E2 | 5.0E2 | 5.0E2 | 5.3E1 | 3.9E2 | 2.9E3 | 1.7E3 | 113 | 8 | 91 | 8 | 0.69 |
| iH | ng/ml | 1.6E5 | 1.5E5 | 1.5E5 | 1.5E5 | 4.7E4 | 4.7E4 | 5.1E4 | 5.1E4 | 2.7E5 | 2.4E5 | 231 | 26 | 159 | 26 | 0.48 |
| iJ | ng/ml | 5.4E4 | 4.6E4 | 5.5E4 | 5.2E4 | 2.9E4 | 3.2E4 | 5.5E3 | 1.1E4 | 2.5E5 | 1.8E5 | 231 | 26 | 159 | 26 | 0.44 |
| hB | ng/ml | 4.1E-1 | 5.6E-1 | 5.0E-1 | 8.4E-1 | 3.3E-1 | 7.5E-1 | 1.0E-9 | 1.2E-1 | 2.3E0 | 3.2E0 | 231 | 26 | 159 | 26 | 0.66 |
| hC | pg/ml | 3.9E3 | 7.6E3 | 6.8E3 | 1.1E4 | 1.0E4 | 1.4E4 | 1.0E-9 | 4.5E1 | 1.1E5 | 5.7E4 | 231 | 26 | 159 | 26 | 0.59 |
| hF | ng/ml | 1.0E-9 | 1.0E-9 | 2.1E1 | 3.7E-1 | 2.7E2 | 1.9E0 | 1.0E-9 | 1.0E-9 | 4.0E3 | 9.6E0 | 231 | 26 | 159 | 26 | 0.51 |
| hG | pg/ml | 6.9E3 | 6.8E3 | 7.4E3 | 8.2E3 | 3.2E3 | 4.3E3 | 2.8E1 | 3.6E3 | 1.9E4 | 2.0E4 | 231 | 26 | 159 | 26 | 0.52 |
| iO | ng/ml | 3.8E5 | 4.0E5 | 4.0E5 | 4.0E5 | 1.8E5 | 1.9E5 | 1.1E4 | 8.3E4 | 1.1E6 | 8.2E5 | 231 | 26 | 159 | 26 | 0.51 |
| iP | ng/ml | 5.0E4 | 5.9E4 | 5.5E4 | 7.0E4 | 5.2E4 | 1.0E5 | 1.0E-9 | 7.2E3 | 5.5E5 | 5.7E5 | 231 | 26 | 159 | 26 | 0.53 |
| iZ | ng/ml | 1.6E3 | 2.0E3 | 1.8E3 | 2.1E3 | 7.8E2 | 1.0E3 | 4.7E2 | 9.8E2 | 5.7E3 | 4.6E3 | 230 | 25 | 158 | 25 | 0.56 |
| yH | pg/ml | 1.1E3 | 1.4E3 | 3.1E3 | 1.9E3 | 1.2E4 | 2.3E3 | 1.0E-9 | 3.0E2 | 1.2E5 | 7.7E3 | 113 | 9 | 92 | 9 | 0.54 |
| yK | U/ml | 2.2E1 | 2.8E1 | 4.5E1 | 3.3E1 | 6.9E1 | 3.2E1 | 1.0E-9 | 1.0E-9 | 3.8E2 | 1.0E2 | 113 | 9 | 92 | 9 | 0.49 |
| yJ | pg/ml | 3.3E4 | 4.0E4 | 4.5E4 | 4.6E4 | 3.3E4 | 2.9E4 | 1.7E3 | 2.7E3 | 1.6E5 | 8.5E4 | 113 | 9 | 92 | 9 | 0.53 |
| yD | ng/ml | 1.5E-2 | 1.5E-2 | 1.5E-2 | 1.6E-2 | 6.7E-3 | 7.5E-3 | 1.0E-9 | 6.3E-3 | 4.3E-2 | 3.0E-2 | 113 | 9 | 91 | 9 | 0.52 |
| jB | ng/ml | 2.6E5 | 2.1E5 | 2.6E5 | 2.8E5 | 9.3E4 | 1.7E5 | 5.7E4 | 1.2E5 | 4.7E5 | 6.2E5 | 46 | 12 | 43 | 12 | 0.46 |
| wB | pg/ml | 7.4E3 | 1.6E4 | 9.4E3 | 1.7E4 | 7.2E3 | 9.6E3 | 1.7E3 | 5.3E3 | 4.1E4 | 3.0E4 | 113 | 9 | 91 | 9 | 0.74 |
| pY | pg/ml | 5.9E0 | 6.8E0 | 8.6E0 | 6.9E0 | 1.9E1 | 3.7E0 | 2.1E0 | 2.6E0 | 2.0E2 | 1.7E1 | 112 | 12 | 94 | 12 | 0.53 |
| rC | pg/ml | 1.6E3 | 1.1E3 | 2.2E3 | 1.4E3 | 2.2E3 | 8.7E2 | 1.0E-9 | 7.0E1 | 1.5E4 | 3.4E3 | 189 | 18 | 128 | 18 | 0.41 |
| rB | pg/ml | 2.4E1 | 5.8E1 | 4.3E1 | 6.8E1 | 8.9E1 | 6.2E1 | 1.0E-9 | 1.0E-9 | 9.5E2 | 2.1E2 | 189 | 18 | 128 | 18 | 0.65 |
| zG | 2.5ng/ml | 2.0E-1 | 5.3E-1 | 4.5E-1 | 6.9E-1 | 8.0E-1 | 8.6E-1 | 1.0E-9 | 1.0E-9 | 4.8E0 | 2.7E0 | 113 | 9 | 92 | 9 | 0.60 |
| zH | 2.3mU/ml | 1.1E-1 | 9.0E-2 | 1.1E-1 | 9.3E-2 | 5.5E-2 | 2.5E-2 | 1.0E-2 | 4.4E-2 | 4.4E-1 | 1.4E-1 | 113 | 9 | 92 | 9 | 0.38 |
| zI | 2.6ng/ml | 2.0E0 | 4.5E0 | 3.8E0 | 6.3E0 | 4.5E0 | 5.9E0 | 5.4E-1 | 7.8E-1 | 2.7E1 | 1.6E1 | 113 | 9 | 92 | 9 | 0.63 |
| qA | ng/ml | 1.0E7 | 1.3E7 | 1.2E7 | 1.7E7 | 7.7E6 | 9.6E6 | 2.0E6 | 4.3E6 | 4.6E7 | 3.4E7 | 112 | 12 | 94 | 12 | 0.65 |
| qB | ng/ml | 6.4E5 | 6.7E5 | 8.2E5 | 1.1E6 | 5.6E5 | 1.0E6 | 2.1E5 | 2.4E5 | 2.9E6 | 3.8E6 | 112 | 12 | 94 | 12 | 0.55 |
| qC | ng/ml | 3.9E5 | 2.3E5 | 7.3E5 | 2.3E5 | 1.1E6 | 1.6E5 | 3.4E3 | 2.1E4 | 7.1E6 | 5.2E5 | 112 | 12 | 94 | 12 | 0.28 |
| qD | ng/ml | 1.6E7 | 1.2E7 | 1.8E7 | 1.3E7 | 8.8E6 | 4.8E6 | 1.2E6 | 8.4E6 | 5.2E7 | 2.6E7 | 112 | 12 | 94 | 12 | 0.32 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| jD | ng/ml | 3.1E1 | 2.9E1 | 4.8E1 | 6.1E1 | 6.1E1 | 1.1E2 | 1.0E-9 | 1.0E-9 | 5.1E2 | 5.1E2 | 196 | 21 | 131 | 21 | 0.50 |
| jE | ng/ml | 1.0E-9 | 1.0E-9 | 7.1E0 | 1.3E1 | 1.7E1 | 1.9E1 | 1.0E-9 | 1.0E-9 | 1.2E2 | 5.6E1 | 196 | 21 | 131 | 21 | 0.60 |
| jF | ng/ml | 4.2E1 | 2.6E1 | 5.7E1 | 4.5E1 | 6.3E1 | 5.7E1 | 1.0E-9 | 1.0E-9 | 3.5E2 | 1.9E2 | 196 | 21 | 131 | 21 | 0.44 |
| jG | ng/ml | 4.6E3 | 4.2E3 | 4.7E3 | 4.4E3 | 2.0E3 | 2.0E3 | 7.6E2 | 6.0E2 | 1.1E4 | 7.9E3 | 197 | 21 | 131 | 21 | 0.47 |
| jH | ng/ml | 7.6E1 | 7.9E1 | 8.5E1 | 9.9E1 | 4.8E1 | 8.8E1 | 1.3E1 | 1.5E1 | 3.3E2 | 4.3E2 | 197 | 21 | 131 | 21 | 0.51 |
| jI | ng/ml | 6.8E1 | 9.4E1 | 7.3E1 | 1.1E2 | 3.3E1 | 9.0E1 | 1.9E1 | 4.4E1 | 2.5E2 | 4.4E2 | 197 | 21 | 131 | 21 | 0.66 |
| sK | pg/mL | 3.7E3 | 4.7E3 | 4.1E3 | 6.7E3 | 1.6E3 | 6.5E3 | 1.1E3 | 2.1E3 | 1.0E4 | 2.3E4 | 113 | 9 | 92 | 9 | 0.64 |
| sM | pg/mL | 7.6E4 | 9.2E4 | 7.9E4 | 1.0E5 | 2.6E4 | 4.3E4 | 3.3E4 | 5.1E4 | 1.6E5 | 2.0E5 | 113 | 9 | 92 | 9 | 0.67 |
| sO | pg/mL | 2.9E8 | 2.5E8 | 2.8E8 | 2.3E8 | 1.0E8 | 1.2E8 | 4.9E7 | 6.6E7 | 5.7E8 | 4.0E8 | 113 | 9 | 92 | 9 | 0.40 |
| wC | ng/ml | 1.5E0 | 1.7E0 | 1.9E0 | 1.7E0 | 1.7E0 | 8.4E-1 | 2.5E-1 | 3.1E-1 | 1.5E1 | 2.9E0 | 113 | 9 | 91 | 9 | 0.52 |
| wD | ng/ml | 1.7E1 | 4.0E1 | 4.8E1 | 8.9E1 | 2.0E2 | 9.2E1 | 2.1E0 | 1.2E1 | 2.1E3 | 2.9E2 | 113 | 9 | 91 | 9 | 0.79 |
| wE | ng/ml | 4.8E1 | 4.5E1 | 4.9E1 | 5.1E1 | 2.3E1 | 1.7E1 | 3.2E0 | 3.6E1 | 1.4E2 | 8.9E1 | 113 | 9 | 91 | 9 | 0.50 |
| wG | ng/ml | 6.4E-2 | 5.9E-2 | 1.0E-1 | 1.5E-1 | 1.3E-1 | 2.3E-1 | 1.0E-9 | 1.0E-9 | 7.1E-1 | 6.8E-1 | 113 | 9 | 91 | 9 | 0.51 |
| wH | ng/ml | 1.8E-2 | 5.9E-2 | 1.5E-1 | 7.4E-1 | 5.2E-1 | 1.8E0 | 1.0E-9 | 1.0E-9 | 4.2E0 | 5.6E0 | 113 | 9 | 91 | 9 | 0.66 |
| wF | ng/ml | 1.4E-1 | 8.1E-1 | 1.5E0 | 3.2E0 | 7.1E0 | 3.9E0 | 1.0E-9 | 1.0E-9 | 5.7E1 | 9.6E0 | 113 | 9 | 91 | 9 | 0.66 |
| rA | pg/ml | 2.6E1 | 2.6E1 | 3.1E1 | 3.2E1 | 2.5E1 | 2.2E1 | 1.0E-9 | 6.9E0 | 2.0E2 | 9.6E1 | 197 | 19 | 131 | 19 | 0.52 |
| qZ | pg/ml | 4.1E1 | 8.0E1 | 3.7E2 | 9.4E2 | 1.8E3 | 2.6E3 | 1.0E-9 | 6.5E-4 | 1.0E4 | 1.0E4 | 155 | 16 | 118 | 16 | 0.61 |
| qY | pg/ml | 2.6E1 | 1.1E1 | 5.1E1 | 2.1E1 | 6.6E1 | 2.4E1 | 8.7E-1 | 2.1E0 | 5.3E2 | 9.1E1 | 197 | 19 | 131 | 19 | 0.33 |
| qX | pg/ml | 5.9E1 | 7.5E1 | 6.5E1 | 8.5E1 | 4.2E1 | 5.4E1 | 1.0E-9 | 1.7E1 | 2.1E2 | 2.0E2 | 197 | 19 | 131 | 19 | 0.60 |
| qW | pg/ml | 9.2E0 | 7.4E0 | 1.4E1 | 1.1E1 | 1.6E1 | 1.1E1 | 1.0E-9 | 1.0E-9 | 1.2E2 | 3.9E1 | 197 | 19 | 131 | 19 | 0.45 |
| qV | pg/ml | 2.2E3 | 1.9E3 | 2.9E3 | 2.7E3 | 2.1E3 | 2.3E3 | 1.0E2 | 6.3E2 | 1.1E4 | 9.6E3 | 197 | 19 | 131 | 19 | 0.47 |
| qU | pg/ml | 6.1E1 | 1.1E2 | 1.6E2 | 1.7E2 | 2.8E2 | 2.0E2 | 1.0E-9 | 3.5E0 | 2.1E3 | 7.9E2 | 197 | 19 | 131 | 19 | 0.56 |
| qT | pg/ml | 4.0E1 | 6.0E1 | 6.8E1 | 8.0E1 | 9.8E1 | 7.6E1 | 1.0E-9 | 6.9E0 | 9.0E2 | 3.0E2 | 197 | 19 | 131 | 19 | 0.57 |
| qI | ng/ml | 6.5E4 | 6.2E4 | 6.4E4 | 6.3E4 | 3.0E4 | 2.7E4 | 5.4E3 | 2.7E4 | 1.6E5 | 1.2E5 | 112 | 10 | 94 | 10 | 0.48 |
| qH | ng/ml | 6.5E4 | 5.4E4 | 7.2E4 | 6.5E4 | 4.5E4 | 3.8E4 | 7.6E3 | 1.0E4 | 2.7E5 | 1.4E5 | 112 | 10 | 94 | 10 | 0.47 |
| qG | ng/ml | 1.9E5 | 1.9E5 | 2.0E5 | 1.8E5 | 7.1E4 | 5.8E4 | 1.7E4 | 9.9E4 | 4.2E5 | 2.7E5 | 112 | 10 | 94 | 10 | 0.43 |
| jK | ng/ml | 1.6E3 | 1.3E3 | 1.7E3 | 1.5E3 | 6.5E2 | 5.9E2 | 2.8E2 | 7.5E2 | 4.3E3 | 2.8E3 | 197 | 21 | 131 | 21 | 0.37 |
| jL | ng/ml | 1.9E2 | 2.6E2 | 2.8E2 | 3.6E2 | 2.5E2 | 3.4E2 | 3.6E1 | 1.2E2 | 2.1E3 | 1.7E3 | 197 | 21 | 131 | 21 | 0.62 |
| jM | ng/ml | 7.4E4 | 6.8E4 | 7.8E4 | 7.3E4 | 3.9E4 | 4.2E4 | 3.9E2 | 5.7E3 | 1.9E5 | 1.5E5 | 197 | 21 | 131 | 21 | 0.47 |
| jO | pg/ml | 2.1E5 | 2.0E5 | 2.6E5 | 2.5E5 | 1.5E5 | 1.2E5 | 5.2E4 | 9.8E4 | 1.1E6 | 5.2E5 | 197 | 21 | 131 | 21 | 0.50 |
| jP | pg/ml | 2.2E5 | 2.2E5 | 2.6E5 | 2.8E5 | 1.6E5 | 1.7E5 | 3.6E4 | 8.4E4 | 9.2E5 | 5.8E5 | 197 | 21 | 131 | 21 | 0.53 |
| jQ | pg/ml | 2.7E3 | 2.1E3 | 3.8E3 | 4.8E3 | 3.5E3 | 1.2E4 | 1.0E-9 | 4.9E2 | 1.8E4 | 5.6E4 | 197 | 21 | 131 | 21 | 0.42 |
| jR | pg/ml | 7.7E3 | 4.0E3 | 1.2E4 | 1.6E4 | 1.3E4 | 3.9E4 | 1.0E-9 | 3.0E1 | 9.0E4 | 1.8E5 | 197 | 21 | 131 | 21 | 0.42 |
| jT | pg/ml | 1.8E5 | 1.6E5 | 1.8E5 | 1.8E5 | 6.6E4 | 9.4E4 | 6.8E4 | 7.5E4 | 4.5E5 | 4.7E5 | 197 | 21 | 131 | 21 | 0.45 |
| xA | pg/ml | 3.9E0 | 7.3E0 | 1.4E1 | 1.9E1 | 4.2E1 | 3.4E1 | 1.0E-9 | 1.0E-9 | 3.9E2 | 1.1E2 | 113 | 9 | 92 | 9 | 0.58 |
| yE | pg/ml | 7.9E1 | 7.9E1 | 8.6E1 | 1.1E2 | 4.0E1 | 7.3E1 | 1.8E1 | 1.6E1 | 3.0E2 | 2.5E2 | 113 | 9 | 92 | 9 | 0.57 |
| tM | pg/ml | 3.9E1 | 4.4E1 | 4.0E1 | 5.1E1 | 1.8E1 | 3.1E1 | 1.0E-9 | 1.6E1 | 1.0E2 | 1.1E2 | 113 | 9 | 92 | 9 | 0.57 |
| tL | pg/ml | 1.0E-9 | 1.0E-9 | 3.5E0 | 3.0E0 | 2.5E1 | 8.7E0 | 1.0E-9 | 1.0E-9 | 2.6E2 | 2.6E1 | 113 | 9 | 92 | 9 | 0.54 |
| jU | mIU/ml | 4.6E0 | 2.3E0 | 1.1E1 | 8.5E0 | 1.7E1 | 1.3E1 | 6.2E-2 | 4.2E-2 | 1.1E2 | 5.3E1 | 197 | 21 | 131 | 21 | 0.44 |
| jV | mIU/ml | 1.5E0 | 1.1E0 | 3.8E0 | 2.5E0 | 6.4E0 | 3.0E0 | 1.7E-3 | 2.2E-3 | 3.5E1 | 1.0E1 | 197 | 21 | 131 | 21 | 0.46 |
| jY | ng/ml | 5.9E-4 | 3.9E-3 | 5.8E-3 | 6.0E-3 | 2.7E-2 | 6.6E-3 | 1.0E-9 | 1.0E-9 | 3.0E-1 | 2.4E-2 | 197 | 21 | 131 | 21 | 0.66 |
| kC | pg/ml | 9.7E1 | 8.8E1 | 1.8E2 | 1.5E2 | 3.8E2 | 1.4E2 | 2.1E1 | 3.6E1 | 3.5E3 | 5.9E2 | 131 | 21 | 103 | 21 | 0.46 |
| kE | pg/ml | 1.3E5 | 1.3E5 | 1.3E5 | 1.4E5 | 3.8E4 | 4.9E4 | 1.2E4 | 5.1E4 | 2.3E5 | 2.7E5 | 131 | 21 | 103 | 21 | 0.48 |
| kF | pg/mL | 6.0E1 | 6.9E1 | 6.8E1 | 7.0E1 | 4.8E1 | 2.3E1 | 2.6E1 | 3.4E1 | 5.1E2 | 1.2E2 | 131 | 21 | 103 | 21 | 0.59 |
| kG | pg/mL | 9.2E3 | 9.2E3 | 1.2E4 | 1.3E4 | 1.4E4 | 1.1E4 | 7.5E2 | 1.1E3 | 1.2E5 | 5.0E4 | 131 | 21 | 103 | 21 | 0.55 |
| kI | pg/ml | 1.8E2 | 1.8E2 | 2.2E2 | 2.1E2 | 1.3E2 | 1.3E2 | 4.4E1 | 1.0E-9 | 8.7E2 | 6.2E2 | 131 | 21 | 103 | 21 | 0.49 |
| kK | pg/ml | 1.0E2 | 1.4E2 | 1.6E2 | 1.9E2 | 1.9E2 | 1.8E2 | 6.4E0 | 2.9E1 | 1.6E3 | 6.3E2 | 131 | 21 | 103 | 21 | 0.55 |
| kN | pg/ml | 9.5E2 | 1.0E3 | 2.0E3 | 1.7E3 | 5.2E3 | 2.1E3 | 7.6E1 | 3.8E2 | 5.5E4 | 8.7E3 | 131 | 21 | 103 | 21 | 0.53 |
| kO | pg/ml | 7.2E3 | 6.1E3 | 8.6E3 | 7.9E3 | 1.1E4 | 3.8E3 | 3.4E3 | 4.2E3 | 1.3E5 | 1.9E4 | 131 | 21 | 103 | 21 | 0.46 |
| kP | pg/ml | 6.3E3 | 4.5E3 | 7.5E3 | 5.7E3 | 6.2E3 | 4.0E3 | 8.6E2 | 1.4E3 | 4.8E4 | 1.7E4 | 131 | 21 | 103 | 21 | 0.39 |
| kQ | pg/ml | 4.1E3 | 4.3E3 | 4.9E3 | 5.2E3 | 3.0E3 | 4.1E3 | 5.6E2 | 1.4E3 | 2.5E4 | 2.2E4 | 231 | 26 | 159 | 26 | 0.50 |
| kR | pg/ml | 2.1E1 | 1.6E1 | 3.1E1 | 2.7E1 | 6.9E1 | 2.6E1 | 1.0E-9 | 5.5E0 | 1.0E3 | 1.1E2 | 231 | 26 | 159 | 26 | 0.44 |
| kS | pg/ml | 8.0E2 | 9.4E2 | 9.7E2 | 1.0E3 | 1.0E3 | 6.5E2 | 8.2E1 | 2.9E2 | 1.4E4 | 3.0E3 | 231 | 26 | 159 | 26 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| pS | ng/ml | 1.9E5 | 1.4E5 | 2.2E5 | 1.6E5 | 1.1E5 | 9.2E4 | 7.5E4 | 6.0E4 | 8.3E5 | 3.6E5 | 113 | 9 | 92 | 9 | 0.31 |
| rZ | ng/ml | 1.0E-9 | 2.2E-3 | 5.4E-3 | 2.6E-2 | 1.5E-2 | 6.9E-2 | 1.0E-9 | 1.0E-9 | 1.1E-1 | 2.9E-1 | 190 | 17 | 126 | 17 | 0.61 |
| rY | ng/ml | 5.7E-2 | 6.9E-2 | 3.6E-1 | 8.8E-1 | 2.3E0 | 3.3E0 | 1.0E-9 | 1.0E-9 | 2.3E1 | 1.4E1 | 190 | 17 | 126 | 17 | 0.58 |
| rX | ng/ml | 1.0E-9 | 1.0E-9 | 6.9E-2 | 1.5E-1 | 4.2E-1 | 5.7E-1 | 1.0E-9 | 1.0E-9 | 3.7E0 | 2.3E0 | 190 | 17 | 126 | 17 | 0.64 |
| lK | pg/ml | 9.9E1 | 2.0E1 | 1.9E2 | 1.1E2 | 3.1E2 | 2.0E2 | 1.0E-9 | 1.0E-9 | 3.3E3 | 7.9E2 | 196 | 21 | 131 | 21 | 0.35 |
| lL | pg/ml | 1.6E3 | 1.7E3 | 2.6E3 | 2.1E3 | 3.7E3 | 1.7E3 | 1.5E1 | 1.9E2 | 4.2E4 | 6.3E3 | 197 | 21 | 131 | 21 | 0.49 |
| lM | pg/ml | 1.1E3 | 2.6E3 | 3.5E3 | 6.8E3 | 7.5E3 | 1.1E4 | 1.2E2 | 9.5E0 | 5.1E4 | 4.0E4 | 197 | 21 | 131 | 21 | 0.61 |
| lN | pg/ml | 1.0E-9 | 3.0E0 | 4.2E0 | 5.2E0 | 1.5E1 | 8.6E0 | 1.0E-9 | 1.0E-9 | 1.5E2 | 3.5E1 | 197 | 21 | 131 | 21 | 0.58 |
| lO | pg/ml | 1.0E-9 | 1.0E-9 | 1.6E0 | 8.2E0 | 1.1E1 | 3.1E1 | 1.0E-9 | 1.0E-9 | 1.3E2 | 1.4E2 | 196 | 21 | 131 | 21 | 0.53 |
| zA | ng/ml | 2.0E7 | 1.8E7 | 2.0E7 | 1.7E7 | 6.6E6 | 5.9E6 | 5.9E6 | 7.5E6 | 3.6E7 | 2.5E7 | 109 | 9 | 87 | 9 | 0.38 |
| rW | ng/ml | 1.5E-2 | 1.5E-2 | 3.5E-2 | 1.8E-2 | 5.1E-2 | 1.1E-2 | 1.0E-9 | 7.4E-3 | 3.2E-1 | 4.3E-2 | 112 | 9 | 93 | 9 | 0.51 |
| rV | ng/ml | 1.0E-9 | 1.0E-9 | 1.8E-2 | 1.2E-2 | 5.8E-2 | 2.1E-2 | 1.0E-9 | 1.0E-9 | 2.8E-1 | 5.6E-2 | 112 | 9 | 93 | 9 | 0.56 |
| rU | ng/ml | 8.2E-2 | 7.6E-2 | 2.7E-1 | 1.3E-1 | 6.5E-1 | 1.2E-1 | 1.0E-9 | 1.0E-9 | 4.4E0 | 4.0E-1 | 112 | 9 | 93 | 9 | 0.54 |
| rT | ng/ml | 5.4E0 | 6.9E0 | 5.9E0 | 7.8E0 | 3.8E0 | 4.3E0 | 8.9E-2 | 1.0E0 | 2.1E1 | 1.3E1 | 112 | 9 | 93 | 9 | 0.65 |
| rS | ng/ml | 3.7E0 | 5.2E0 | 5.3E0 | 1.4E1 | 5.1E0 | 1.9E1 | 3.9E-1 | 2.0E0 | 3.8E1 | 5.9E1 | 112 | 9 | 93 | 9 | 0.66 |
| sC | pg/mL | 8.0E3 | 6.2E3 | 1.3E4 | 9.1E3 | 1.4E4 | 7.7E3 | 1.7E3 | 3.4E3 | 8.0E4 | 2.8E4 | 113 | 9 | 92 | 9 | 0.47 |
| yL | pg/ml | 3.0E1 | 4.1E1 | 3.4E1 | 5.7E1 | 2.3E1 | 5.5E1 | 5.6E0 | 1.6E1 | 1.8E2 | 1.9E2 | 111 | 9 | 90 | 9 | 0.64 |
| rP | ng/ml | 1.0E2 | 7.4E1 | 2.8E2 | 2.5E2 | 9.3E2 | 2.4E2 | 1.0E-9 | 1.2E1 | 9.7E3 | 5.0E2 | 112 | 9 | 93 | 9 | 0.54 |
| rQ | ng/ml | 1.0E-9 | 1.0E-9 | 8.0E0 | 1.9E1 | 2.8E1 | 5.6E1 | 1.0E-9 | 1.0E-9 | 1.7E2 | 1.7E2 | 112 | 9 | 93 | 9 | 0.49 |
| rO | ng/ml | 3.0E-2 | 1.5E-2 | 6.4E-2 | 3.9E-2 | 1.0E-1 | 5.5E-2 | 1.0E-9 | 1.0E-9 | 4.9E-1 | 1.4E-1 | 112 | 9 | 93 | 9 | 0.43 |
| rR | ng/ml | 4.0E0 | 4.0E0 | 2.0E1 | 8.8E0 | 5.0E1 | 1.3E1 | 1.0E-9 | 1.0E-9 | 4.5E2 | 3.9E1 | 112 | 9 | 93 | 9 | 0.45 |
| rN | ng/ml | 6.0E-1 | 7.9E-1 | 7.3E-1 | 2.4E0 | 5.2E-1 | 4.1E0 | 5.1E-2 | 3.1E-1 | 3.0E0 | 1.3E1 | 112 | 9 | 93 | 9 | 0.63 |
| qO | pg/ml | 1.0E4 | 1.2E4 | 1.5E4 | 1.8E4 | 1.3E4 | 1.7E4 | 7.4E2 | 5.4E3 | 6.4E4 | 6.3E4 | 112 | 10 | 92 | 10 | 0.56 |
| qP | pg/ml | 3.6E2 | 3.5E2 | 5.0E2 | 5.9E2 | 3.9E2 | 6.8E2 | 1.0E-9 | 1.5E2 | 2.2E3 | 2.5E3 | 112 | 10 | 92 | 10 | 0.50 |
| qQ | pg/ml | 1.5E1 | 1.1E1 | 1.7E1 | 1.8E1 | 3.8E1 | 2.0E1 | 1.0E-9 | 1.0E-9 | 2.8E2 | 4.3E1 | 112 | 10 | 92 | 10 | 0.57 |
| nW | pg/ml | 1.1E5 | 1.1E5 | 1.1E5 | 1.1E5 | 2.5E4 | 3.5E4 | 5.8E4 | 3.6E4 | 1.8E5 | 2.1E5 | 231 | 26 | 159 | 26 | 0.48 |
| nY | pg/ml | 2.1E3 | 2.1E3 | 2.4E3 | 2.7E3 | 1.6E3 | 1.7E3 | 5.1E2 | 6.3E2 | 1.3E4 | 8.1E3 | 231 | 26 | 159 | 26 | 0.54 |
| oO | pg/ml | 7.8E4 | 1.0E5 | 1.1E5 | 1.2E5 | 9.7E4 | 7.8E4 | 1.5E4 | 3.3E3 | 6.2E5 | 3.4E5 | 123 | 20 | 97 | 20 | 0.60 |
| oP | pg/ml | 1.2E5 | 1.4E5 | 1.4E5 | 1.5E5 | 9.1E4 | 9.8E4 | 2.4E4 | 2.4E4 | 4.5E5 | 4.6E5 | 123 | 20 | 97 | 20 | 0.55 |
| oQ | pg/ml | 2.8E3 | 3.2E3 | 3.5E3 | 4.3E3 | 2.8E3 | 3.6E3 | 9.3E2 | 8.7E2 | 2.1E4 | 1.7E4 | 123 | 20 | 97 | 20 | 0.59 |
| oE | pg/ml | 1.3E2 | 6.2E2 | 3.6E2 | 8.8E2 | 5.4E2 | 9.1E2 | 1.0E-9 | 1.0E-9 | 4.7E3 | 3.4E3 | 231 | 26 | 159 | 26 | 0.67 |
| oF | pg/ml | 7.7E3 | 1.9E4 | 2.0E4 | 3.8E4 | 3.6E4 | 4.6E4 | 6.4E1 | 1.3E2 | 2.5E5 | 1.7E5 | 231 | 26 | 159 | 26 | 0.66 |
| oH | pg/ml | 4.4E1 | 3.8E1 | 9.3E1 | 5.2E1 | 1.4E2 | 4.7E1 | 4.2E0 | 7.3E0 | 9.9E2 | 1.8E2 | 231 | 26 | 159 | 26 | 0.42 |
| oK | pg/ml | 7.6E2 | 1.0E3 | 1.8E3 | 2.0E3 | 2.5E3 | 2.1E3 | 5.2E1 | 2.3E2 | 1.8E4 | 7.3E3 | 231 | 26 | 159 | 26 | 0.57 |
| oN | pg/ml | 5.0E2 | 5.0E2 | 7.5E2 | 6.3E2 | 1.4E3 | 4.1E2 | 1.5E2 | 1.1E2 | 1.8E4 | 1.8E3 | 231 | 26 | 159 | 26 | 0.51 |
| oW | pg/ml | 2.1E2 | 3.6E2 | 4.0E2 | 6.1E2 | 8.7E2 | 8.0E2 | 7.7E1 | 9.5E1 | 6.0E3 | 2.7E3 | 46 | 12 | 43 | 12 | 0.59 |
| oT | pg/ml | 3.3E2 | 3.4E2 | 3.7E2 | 3.4E2 | 1.9E2 | 1.2E2 | 9.9E1 | 1.4E2 | 7.9E2 | 5.4E2 | 46 | 12 | 43 | 12 | 0.48 |
| oV | pg/ml | 1.3E2 | 9.3E1 | 3.0E2 | 1.4E2 | 5.1E2 | 1.7E2 | 1.0E-9 | 1.1E1 | 2.4E3 | 6.3E2 | 46 | 12 | 43 | 12 | 0.41 |
| oD | pg/ml | 1.7E4 | 1.5E4 | 1.7E4 | 1.7E4 | 7.4E3 | 6.0E3 | 6.6E3 | 1.1E4 | 4.6E4 | 3.3E4 | 46 | 12 | 43 | 12 | 0.47 |
| uL | ng/ml | 3.8E1 | 4.3E1 | 5.5E1 | 4.5E1 | 7.0E1 | 2.4E1 | 1.0E-9 | 2.1E1 | 4.0E2 | 9.2E1 | 111 | 9 | 90 | 9 | 0.53 |
| uO | ng/ml | 3.6E-1 | 4.5E-1 | 8.7E-1 | 6.5E-1 | 1.3E0 | 7.4E-1 | 1.0E-9 | 1.0E-9 | 9.3E0 | 1.7E0 | 111 | 9 | 90 | 9 | 0.47 |
| uM | ng/ml | 5.9E-1 | 3.2E-1 | 9.1E-1 | 6.6E-1 | 1.6E0 | 8.6E-1 | 1.0E-9 | 1.0E-9 | 1.3E1 | 2.5E0 | 111 | 9 | 90 | 9 | 0.40 |
| uI | ng/ml | 7.4E-2 | 5.9E-2 | 1.2E-1 | 8.2E-2 | 1.6E-1 | 5.0E-2 | 1.6E-2 | 3.4E-2 | 1.1E0 | 1.8E-1 | 110 | 9 | 89 | 9 | 0.47 |
| uN | ng/ml | 1.5E1 | 1.9E1 | 1.7E1 | 2.0E1 | 6.8E0 | 8.2E0 | 6.3E0 | 9.2E0 | 4.1E1 | 3.3E1 | 111 | 9 | 90 | 9 | 0.63 |
| uG | ng/ml | 1.9E1 | 2.2E1 | 2.3E1 | 3.7E1 | 1.6E1 | 3.9E1 | 1.2E0 | 6.5E0 | 8.9E1 | 1.3E2 | 111 | 9 | 90 | 9 | 0.60 |
| uR | ng/ml | 2.4E0 | 3.3E0 | 3.4E0 | 3.4E0 | 6.1E0 | 2.0E0 | 7.3E-1 | 1.3E0 | 6.4E1 | 6.6E0 | 113 | 9 | 92 | 9 | 0.57 |
| uP | ng/ml | 2.3E0 | 2.5E0 | 2.6E0 | 2.8E0 | 1.2E0 | 1.3E0 | 1.1E0 | 9.3E-1 | 9.1E0 | 4.8E0 | 113 | 9 | 92 | 9 | 0.58 |
| uV | ng/ml | 2.3E-3 | 1.3E-3 | 1.5E-2 | 4.1E-3 | 3.2E-2 | 5.9E-3 | 1.0E-9 | 1.0E-9 | 2.0E-1 | 1.8E-2 | 113 | 9 | 92 | 9 | 0.42 |
| uT | ng/ml | 6.3E1 | 1.1E2 | 8.3E1 | 1.5E2 | 8.1E1 | 1.2E2 | 8.7E0 | 4.7E1 | 5.8E2 | 4.1E2 | 113 | 9 | 92 | 9 | 0.74 |
| uU | ng/ml | 1.7E0 | 2.7E0 | 2.0E0 | 4.0E0 | 1.2E0 | 6.0E0 | 5.2E-1 | 5.4E-1 | 7.0E0 | 2.0E1 | 113 | 9 | 92 | 9 | 0.55 |
| uW | ng/ml | 7.4E0 | 8.1E0 | 7.9E0 | 8.0E0 | 2.7E0 | 2.4E0 | 3.5E0 | 3.1E0 | 2.2E1 | 1.2E1 | 111 | 9 | 90 | 9 | 0.59 |
| vB | ng/ml | 2.8E0 | 3.2E0 | 3.0E0 | 3.4E0 | 1.7E0 | 1.6E0 | 5.9E-1 | 1.3E0 | 1.0E1 | 6.6E0 | 111 | 9 | 90 | 9 | 0.57 |
| vC | ng/ml | 1.0E-9 | 1.0E-9 | 3.7E-3 | 1.0E-9 | 2.4E-2 | 0.0E0 | 1.0E-9 | 1.0E-9 | 2.0E-1 | 1.0E-9 | 111 | 9 | 90 | 9 | 0.48 |

| Code | Units | Median | | Average | | Standard Deviation | | Minimum | | Maximum | | Number of Samples | | Number of Patients | | AUC |
|------|-------|--------|-----|---------|-----|--------|-----|--------|-----|--------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| uY | ng/ml | 6.8E-1 | 7.5E-1 | 1.1E0 | 1.3E0 | 1.0E0 | 1.4E0 | 6.8E-2 | 1.8E-1 | 4.9E0 | 4.4E0 | 111 | 9 | 90 | 9 | 0.48 |
| uZ | ng/ml | 5.5E-1 | 5.9E-1 | 7.4E-1 | 7.9E-1 | 8.7E-1 | 6.9E-1 | 4.7E-2 | 1.0E-1 | 7.2E0 | 2.0E0 | 111 | 9 | 90 | 9 | 0.52 |
| uX | ng/ml | 1.2E1 | 1.7E1 | 1.3E1 | 3.0E1 | 7.1E0 | 2.9E1 | 3.6E0 | 4.0E0 | 4.0E1 | 7.8E1 | 111 | 9 | 90 | 9 | 0.63 |
| vA | ng/ml | 7.0E-2 | 7.3E-2 | 8.2E-2 | 1.0E-1 | 5.2E-2 | 1.2E-1 | 1.7E-2 | 2.5E-2 | 3.0E-1 | 4.2E-1 | 111 | 9 | 90 | 9 | 0.48 |
| vH | ng/ml | 1.2E-1 | 1.1E-1 | 1.6E-1 | 3.5E-1 | 1.5E-1 | 5.9E-1 | 9.9E-3 | 4.7E-2 | 9.6E-1 | 1.9E0 | 113 | 9 | 92 | 9 | 0.54 |
| vI | ng/ml | 1.6E0 | 2.9E0 | 2.0E0 | 3.0E0 | 1.8E0 | 3.1E0 | 4.2E-3 | 1.7E-3 | 1.0E1 | 1.0E1 | 113 | 9 | 92 | 9 | 0.60 |
| vP | ng/ml | 4.4E2 | 3.1E2 | 5.5E2 | 4.7E2 | 4.7E2 | 4.5E2 | 4.0E1 | 1.5E2 | 2.4E3 | 1.6E3 | 113 | 9 | 92 | 9 | 0.46 |
| vT | ng/ml | 7.9E1 | 7.7E1 | 1.0E2 | 8.9E1 | 9.4E1 | 4.1E1 | 2.4E1 | 4.6E1 | 6.9E2 | 1.6E2 | 113 | 9 | 92 | 9 | 0.50 |
| vU | ng/ml | 1.0E-9 | 1.0E-9 | 2.1E1 | 2.5E1 | 3.3E1 | 4.5E1 | 1.0E-9 | 1.0E-9 | 1.3E2 | 1.1E2 | 113 | 9 | 92 | 9 | 0.50 |
| vQ | ng/ml | 3.5E2 | 3.2E2 | 3.7E2 | 3.7E2 | 1.4E2 | 1.7E2 | 6.7E1 | 1.9E2 | 8.4E2 | 6.0E2 | 113 | 9 | 92 | 9 | 0.49 |
| vO | ng/ml | 1.8E3 | 2.0E3 | 1.8E3 | 2.1E3 | 4.3E2 | 6.9E2 | 1.0E3 | 1.0E3 | 3.0E3 | 3.2E3 | 113 | 9 | 92 | 9 | 0.61 |
| vS | ng/ml | 1.3E3 | 1.4E3 | 1.2E3 | 1.5E3 | 4.6E2 | 3.1E2 | 1.0E-9 | 1.1E3 | 2.1E3 | 1.9E3 | 113 | 9 | 92 | 9 | 0.72 |
| vV | ng/ml | 9.5E2 | 9.4E2 | 1.5E3 | 2.4E4 | 1.8E3 | 7.0E4 | 2.1E1 | 2.3E2 | 1.1E4 | 2.1E5 | 113 | 9 | 92 | 9 | 0.47 |
| vW | ng/ml | 1.3E2 | 1.6E2 | 1.7E2 | 2.7E2 | 1.2E2 | 2.3E2 | 4.3E1 | 6.0E1 | 6.7E2 | 7.7E2 | 113 | 9 | 92 | 9 | 0.61 |
| pF | pg/ml | 4.5E-1 | 5.6E-1 | 1.0E0 | 1.0E0 | 5.7E0 | 9.4E-1 | 1.0E-9 | 1.8E-1 | 8.7E1 | 4.4E0 | 231 | 26 | 159 | 26 | 0.64 |
| pH | ng/ml | 8.8E0 | 1.2E1 | 9.8E0 | 1.3E1 | 6.4E0 | 1.1E1 | 3.0E0 | 3.3E0 | 4.2E1 | 4.7E1 | 46 | 12 | 43 | 12 | 0.62 |
| pI | ng/ml | 7.0E1 | 6.3E1 | 7.6E1 | 6.6E1 | 3.8E1 | 2.8E1 | 2.6E1 | 2.5E1 | 2.0E2 | 1.2E2 | 46 | 12 | 43 | 12 | 0.44 |
| pK | ng/ml | 4.6E-1 | 3.9E-1 | 5.1E-1 | 5.2E-1 | 2.6E-1 | 3.3E-1 | 1.7E-1 | 1.6E-1 | 1.6E0 | 1.4E0 | 46 | 12 | 43 | 12 | 0.49 |

Figure 2.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.0E1 | 6.2E1 | 8.2E1 | 6.6E1 | 5.8E1 | 4.1E1 | 1.0E0 | 1.3E1 | 4.8E2 | 1.6E2 | 1777 | 12 | 298 | 12 | 0.43 |
| Ad | ug/mL | 3.8E-2 | 8.1E-2 | 9.1E-2 | 1.1E-1 | 3.8E-1 | 1.1E-1 | 2.7E-4 | 1.8E-2 | 8.5E0 | 3.5E-1 | 511 | 8 | 198 | 8 | 0.65 |
| Af | ng/mL | 1.2E0 | 1.3E0 | 1.8E1 | 4.6E0 | 6.6E1 | 6.7E0 | 1.7E-3 | 1.7E-3 | 5.3E2 | 1.8E1 | 511 | 8 | 198 | 8 | 0.47 |
| Aj | ug/mL | 1.5E0 | 4.0E0 | 2.6E0 | 3.3E0 | 2.5E0 | 2.7E0 | 1.5E-3 | 8.4E-2 | 6.1E0 | 5.8E0 | 511 | 8 | 198 | 8 | 0.57 |
| Al | mg/mL | 8.8E-5 | 6.9E-5 | 2.4E-4 | 2.9E-4 | 4.0E-4 | 4.2E-4 | 2.3E-6 | 2.2E-5 | 2.2E-3 | 1.1E-3 | 511 | 8 | 198 | 8 | 0.51 |
| An | U/mL | 5.0E1 | 8.0E1 | 1.9E2 | 1.4E2 | 5.6E2 | 1.4E2 | 9.8E-4 | 1.4E1 | 7.8E3 | 4.3E2 | 511 | 8 | 198 | 8 | 0.61 |
| Ao | pg/mL | 9.0E1 | 1.2E2 | 4.7E2 | 7.8E2 | 3.2E3 | 1.5E3 | 1.5E0 | 2.1E1 | 3.9E4 | 4.5E3 | 511 | 8 | 198 | 8 | 0.62 |
| Ap | ng/mL | 3.2E1 | 5.2E1 | 4.7E1 | 8.8E1 | 4.9E1 | 9.0E1 | 8.4E-5 | 1.4E1 | 3.3E2 | 2.4E2 | 511 | 8 | 198 | 8 | 0.65 |
| Ar | ng/mL | 9.8E-1 | 3.6E0 | 1.1E1 | 5.3E0 | 1.8E2 | 7.1E0 | 3.4E-3 | 1.0E-1 | 4.1E3 | 2.2E1 | 511 | 8 | 198 | 8 | 0.65 |
| As | ng/mL | 8.7E-3 | 6.3E-3 | 1.5E-2 | 1.6E-2 | 5.7E-2 | 2.4E-2 | 1.7E-3 | 1.7E-3 | 1.2E0 | 7.0E-2 | 511 | 8 | 198 | 8 | 0.47 |
| Aw | pg/mL | 1.6E1 | 2.2E1 | 1.6E1 | 2.1E1 | 6.3E0 | 6.5E0 | 2.9E-2 | 1.1E1 | 5.1E1 | 2.9E1 | 511 | 8 | 198 | 8 | 0.71 |
| Ax | ng/mL | 2.1E0 | 2.3E0 | 1.5E1 | 1.0E1 | 6.0E1 | 1.5E1 | 1.2E-2 | 1.3E-1 | 7.7E2 | 4.4E1 | 511 | 8 | 198 | 8 | 0.53 |
| Ba | ng/mL | 6.2E1 | 8.8E2 | 4.4E2 | 3.7E3 | 1.1E3 | 5.3E3 | 2.7E-1 | 8.5E0 | 8.1E3 | 1.5E4 | 511 | 8 | 198 | 8 | 0.73 |
| Bb | ng/mL | 3.2E0 | 4.2E0 | 6.6E0 | 9.2E0 | 1.4E1 | 8.8E0 | 4.1E-3 | 2.2E0 | 2.5E2 | 2.5E1 | 511 | 8 | 198 | 8 | 0.65 |
| Bc | ng/mL | 3.8E1 | 9.5E1 | 1.1E2 | 2.5E2 | 1.9E2 | 3.9E2 | 1.1E-1 | 2.0E1 | 1.2E3 | 1.2E3 | 511 | 8 | 198 | 8 | 0.71 |
| Bg | ng/mL | 8.1E-2 | 1.1E0 | 5.3E0 | 5.1E1 | 2.9E1 | 1.4E2 | 5.3E-4 | 2.0E-2 | 4.4E2 | 4.0E2 | 511 | 8 | 198 | 8 | 0.71 |
| Bn | ng/mL | 5.6E-2 | 5.6E-2 | 1.4E0 | 1.4E0 | 3.2E0 | 3.0E0 | 5.6E-2 | 5.6E-2 | 5.8E1 | 8.6E0 | 511 | 8 | 198 | 8 | 0.47 |
| Bo | ng/mL | 1.2E1 | 1.4E1 | 1.4E1 | 1.7E1 | 1.8E1 | 1.5E1 | 1.6E-2 | 1.6E-2 | 2.8E2 | 3.9E1 | 511 | 8 | 198 | 8 | 0.57 |
| Ch | uIU/mL | 9.8E-1 | 1.9E0 | 1.7E1 | 1.5E2 | 9.6E1 | 4.2E2 | 3.4E-3 | 2.8E-1 | 1.8E3 | 1.2E3 | 511 | 8 | 198 | 8 | 0.59 |
| Co | pg/mL | 3.7E1 | 8.3E1 | 1.7E2 | 4.3E2 | 9.1E2 | 6.9E2 | 1.5E-1 | 4.2E1 | 1.7E4 | 2.1E3 | 511 | 8 | 198 | 8 | 0.78 |
| Cp | ng/mL | 2.2E1 | 5.0E1 | 3.0E1 | 4.5E1 | 6.3E1 | 2.4E1 | 6.0E-1 | 7.5E0 | 1.3E3 | 7.2E1 | 511 | 8 | 198 | 8 | 0.74 |
| Cq | ng/mL | 3.0E-2 | 3.7E-2 | 2.3E-1 | 1.8E-1 | 2.3E0 | 3.9E-1 | 8.0E-4 | 8.0E-4 | 4.9E1 | 1.1E0 | 511 | 8 | 198 | 8 | 0.58 |
| Cs | ng/mL | 6.6E1 | 5.7E1 | 3.2E2 | 1.6E2 | 1.1E3 | 2.5E2 | 2.7E-2 | 2.7E0 | 1.8E4 | 7.5E2 | 511 | 8 | 198 | 8 | 0.47 |
| Ct | ng/mL | 6.0E-1 | 5.0E0 | 3.7E1 | 9.5E1 | 1.1E2 | 1.7E2 | 1.1E-4 | 5.6E-2 | 6.2E2 | 4.2E2 | 511 | 8 | 198 | 8 | 0.57 |
| Cu | ng/mL | 2.4E-1 | 9.0E-1 | 5.3E-1 | 1.0E0 | 3.0E0 | 7.3E-1 | 9.0E-5 | 1.4E-1 | 6.6E1 | 2.3E0 | 511 | 8 | 198 | 8 | 0.83 |
| Cv | ng/mL | 5.6E0 | 4.5E0 | 2.6E1 | 1.7E1 | 6.5E1 | 3.0E1 | 1.4E-4 | 1.5E-1 | 5.3E2 | 8.9E1 | 511 | 8 | 198 | 8 | 0.42 |
| Cw | mIU/mL | 3.0E-2 | 5.3E-2 | 5.1E-2 | 6.5E-2 | 3.0E-1 | 3.9E-2 | 1.5E-4 | 5.5E-3 | 6.8E0 | 1.2E-1 | 511 | 8 | 198 | 8 | 0.73 |
| Cx | ng/mL | 4.6E-1 | 3.7E-2 | 6.3E1 | 4.0E1 | 1.1E2 | 1.1E2 | 9.3E-5 | 9.3E-5 | 3.9E2 | 3.2E2 | 511 | 8 | 198 | 8 | 0.40 |
| Db | ug/mL | 7.4E0 | 8.3E0 | 9.2E0 | 8.5E0 | 1.0E1 | 5.3E0 | 4.5E-1 | 1.2E0 | 1.4E2 | 1.5E1 | 511 | 8 | 198 | 8 | 0.56 |
| Dc | nmol/L | 1.9E-2 | 1.8E-2 | 8.4E-2 | 6.6E-2 | 6.3E-1 | 9.5E-2 | 5.2E-6 | 5.9E-3 | 1.4E1 | 2.7E-1 | 511 | 8 | 198 | 8 | 0.56 |
| Dd | ug/mL | 7.5E-2 | 8.3E-2 | 1.8E-1 | 2.1E-1 | 2.9E-1 | 2.7E-1 | 8.3E-5 | 3.3E-3 | 3.6E0 | 7.3E-1 | 511 | 8 | 198 | 8 | 0.48 |
| De | ng/mL | 3.4E-3 | 6.2E-2 | 8.0E-2 | 2.3E-1 | 1.4E-1 | 3.8E-1 | 3.4E-3 | 3.4E-3 | 9.2E-1 | 1.1E0 | 511 | 8 | 198 | 8 | 0.62 |
| Dg | ng/mL | 3.2E1 | 5.9E1 | 4.5E1 | 6.0E1 | 4.1E1 | 3.9E1 | 1.0E-1 | 4.3E0 | 1.9E2 | 1.2E2 | 511 | 8 | 198 | 8 | 0.62 |
| Di | pg/mL | 1.8E0 | 4.1E0 | 2.2E0 | 3.5E0 | 2.1E0 | 1.7E0 | 1.8E-1 | 1.8E-1 | 1.3E1 | 5.4E0 | 511 | 8 | 198 | 8 | 0.72 |
| Dk | uIU/mL | 1.6E-2 | 1.8E-2 | 8.3E-2 | 6.2E-2 | 4.8E-1 | 1.1E-1 | 1.1E-4 | 1.1E-4 | 8.9E0 | 3.3E-1 | 511 | 8 | 198 | 8 | 0.52 |
| Dl | ng/mL | 2.4E2 | 2.0E2 | 3.2E2 | 3.5E2 | 3.0E2 | 3.1E2 | 1.7E0 | 7.4E1 | 1.6E3 | 8.5E2 | 511 | 8 | 198 | 8 | 0.55 |
| Ef | ng/ml | 1.3E-1 | 6.4E0 | 8.9E-1 | 4.7E0 | 1.9E0 | 4.4E0 | 5.7E-4 | 9.1E-2 | 1.0E1 | 9.9E0 | 379 | 7 | 195 | 7 | 0.78 |
| Wm | % | 7.0E-1 | 2.7E0 | 3.4E1 | 3.4E1 | 1.8E2 | 6.7E1 | 5.4E-2 | 8.5E-2 | 2.4E3 | 1.9E2 | 416 | 9 | 214 | 9 | 0.66 |
| Po | pg/ml | 6.9E-1 | 1.5E1 | 8.6E0 | 3.3E1 | 2.4E1 | 5.9E1 | 8.0E-3 | 2.6E-2 | 2.7E2 | 2.2E2 | 899 | 13 | 335 | 13 | 0.73 |
| Et | ng/ml | 1.4E3 | 2.0E3 | 1.7E3 | 2.7E3 | 1.2E3 | 1.5E3 | 7.5E1 | 1.1E3 | 5.0E3 | 5.0E3 | 898 | 13 | 335 | 13 | 0.72 |
| Fp | ng/ml | 1.4E1 | 3.1E1 | 2.5E1 | 3.7E1 | 2.9E1 | 2.7E1 | 6.0E-3 | 9.7E-1 | 1.4E2 | 7.9E1 | 931 | 13 | 336 | 13 | 0.65 |
| Fr | ng/ml | 3.7E5 | 5.5E5 | 1.2E5 | 4.8E5 | 1.8E5 | 3.5E5 | 1.9E2 | 5.9E3 | 9.0E5 | 8.9E5 | 1043 | 14 | 340 | 14 | 0.80 |
| Fw | pg/ml | 1.2E0 | 7.8E0 | 5.8E1 | 1.5E1 | 4.5E2 | 1.8E1 | 1.1E-14 | 1.2E-1 | 6.9E3 | 4.2E1 | 380 | 7 | 196 | 7 | 0.64 |
| Gl | pg/ml | 7.2E3 | 2.3E4 | 1.1E4 | 1.8E4 | 9.2E3 | 1.2E4 | 9.1E1 | 6.7E2 | 3.4E4 | 3.2E4 | 370 | 7 | 194 | 7 | 0.70 |
| Nm | pg/ml | 1.4E4 | 4.2E4 | 3.3E4 | 8.4E4 | 8.2E4 | 1.2E5 | 1.0E-9 | 1.0E-9 | 1.6E6 | 4.4E5 | 902 | 13 | 337 | 13 | 0.65 |
| Nn | pg/ml | 1.6E2 | 1.3E3 | 1.9E3 | 1.4E4 | 8.2E3 | 3.1E4 | 1.0E-9 | 1.0E-9 | 1.0E5 | 1.1E5 | 902 | 13 | 337 | 13 | 0.74 |
| No | pg/ml | 1.6E1 | 5.1E1 | 3.8E1 | 1.7E2 | 1.1E2 | 3.0E2 | 1.0E-9 | 1.0E-9 | 2.5E3 | 9.5E2 | 902 | 13 | 337 | 13 | 0.70 |
| Nq | pg/ml | 2.0E0 | 2.5E1 | 2.0E1 | 8.3E1 | 7.6E1 | 1.1E2 | 1.0E-9 | 1.0E-9 | 1.2E3 | 3.0E2 | 902 | 13 | 337 | 13 | 0.66 |
| Nr | pg/ml | 1.2E0 | 8.3E0 | 2.9E1 | 1.2E2 | 1.8E2 | 3.8E2 | 1.0E-9 | 1.0E-9 | 4.1E3 | 1.4E3 | 902 | 13 | 337 | 13 | 0.60 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ns | pg/ml | 1.0E-9 | 1.0E-9 | 9.2E0 | 1.0E-9 | 5.2E1 | 0.0E0 | 1.0E-9 | 1.0E-9 | 1.1E3 | 1.0E-9 | 902 | 13 | 337 | 13 | 0.45 |
| Nt | pg/ml | 1.0E2 | 2.2E2 | 1.4E2 | 3.0E2 | 1.2E2 | 3.1E2 | 1.0E-9 | 5.2E1 | 1.7E3 | 1.2E3 | 902 | 13 | 337 | 13 | 0.74 |
| Nu | pg/ml | 2.0E1 | 1.2E2 | 5.5E1 | 1.2E2 | 9.0E1 | 8.8E1 | 1.0E-9 | 1.0E-9 | 8.9E2 | 2.9E2 | 902 | 13 | 337 | 13 | 0.76 |
| Lu | pg/ml | 1.0E4 | 8.1E3 | 1.8E4 | 1.2E4 | 6.2E4 | 1.6E4 | 3.5E2 | 1.3E3 | 1.3E6 | 6.1E4 | 905 | 13 | 337 | 13 | 0.40 |
| Lv | pg/ml | 1.0E-9 | 4.3E1 | 1.1E1 | 5.4E1 | 2.2E1 | 6.7E1 | 1.0E-9 | 1.0E-9 | 2.6E2 | 1.9E2 | 905 | 13 | 337 | 13 | 0.70 |
| Lw | pg/ml | 1.0E-9 | 1.0E-9 | 3.7E-1 | 7.3E0 | 3.8E0 | 1.5E1 | 1.0E-9 | 1.0E-9 | 8.0E1 | 4.7E1 | 905 | 13 | 337 | 13 | 0.61 |
| Lx | pg/ml | 1.0E-9 | 3.6E2 | 1.7E2 | 8.2E2 | 8.6E2 | 1.2E3 | 1.0E-9 | 1.0E-9 | 2.2E4 | 4.0E3 | 905 | 13 | 337 | 13 | 0.78 |
| Ly | pg/ml | 1.0E-9 | 1.0E-9 | 1.0E1 | 1.3E1 | 2.0E1 | 2.4E1 | 1.0E-9 | 1.0E-9 | 1.6E2 | 7.2E1 | 905 | 13 | 337 | 13 | 0.50 |
| Lz | pg/ml | 1.0E-9 | 1.0E-9 | 4.0E0 | 3.6E0 | 3.0E1 | 1.3E1 | 1.0E-9 | 1.0E-9 | 6.0E2 | 4.7E1 | 905 | 13 | 337 | 13 | 0.50 |
| Ma | pg/ml | 2.9E2 | 2.5E3 | 1.4E3 | 8.4E3 | 3.6E3 | 1.6E4 | 1.0E-9 | 1.8E1 | 6.5E4 | 5.2E4 | 905 | 13 | 337 | 13 | 0.66 |
| Mb | pg/ml | 2.5E1 | 2.5E1 | 3.1E1 | 3.2E1 | 1.5E1 | 1.6E1 | 4.1E0 | 1.3E1 | 2.1E2 | 5.8E1 | 905 | 13 | 337 | 13 | 0.46 |
| Mc | pg/ml | 1.0E-9 | 1.0E-9 | 3.8E-2 | 1.1E-1 | 5.7E-1 | 3.9E-1 | 1.0E-9 | 1.0E-9 | 1.3E1 | 1.4E0 | 905 | 13 | 337 | 13 | 0.53 |
| Md | pg/ml | 1.0E-9 | 1.0E-9 | 4.4E-1 | 1.0E-9 | 3.6E0 | 0.0E0 | 1.0E-9 | 1.0E-9 | 6.8E1 | 1.0E-9 | 905 | 13 | 337 | 13 | 0.46 |
| Me | pg/ml | 3.3E1 | 2.4E1 | 3.2E1 | 3.5E1 | 2.0E1 | 4.7E1 | 1.0E-9 | 1.5E0 | 3.2E2 | 1.8E2 | 905 | 13 | 337 | 13 | 0.41 |
| Mf | pg/ml | 1.0E-9 | 1.0E-9 | 5.4E-1 | 7.8E-1 | 2.9E0 | 1.8E0 | 1.0E-9 | 1.0E-9 | 5.6E1 | 5.0E0 | 905 | 13 | 337 | 13 | 0.55 |
| Mg | pg/ml | 1.6E0 | 1.5E1 | 7.4E0 | 2.5E1 | 1.2E1 | 4.0E1 | 1.0E-9 | 1.0E-9 | 9.2E1 | 1.5E2 | 905 | 13 | 337 | 13 | 0.70 |
| Mh | pg/ml | 1.0E-9 | 2.9E-2 | 1.3E0 | 1.7E0 | 9.6E0 | 5.0E0 | 1.0E-9 | 1.0E-9 | 1.6E2 | 1.8E1 | 905 | 13 | 337 | 13 | 0.62 |
| Mi | pg/ml | 1.0E-9 | 1.0E-9 | 9.0E-1 | 1.4E1 | 1.2E1 | 3.3E1 | 1.0E-9 | 1.0E-9 | 3.2E2 | 1.1E2 | 905 | 13 | 337 | 13 | 0.64 |
| Mj | pg/ml | 1.0E-9 | 2.9E0 | 4.5E0 | 2.1E1 | 2.5E1 | 5.6E1 | 1.0E-9 | 1.0E-9 | 4.6E2 | 2.1E2 | 905 | 13 | 337 | 13 | 0.67 |
| Mk | pg/ml | 1.0E0 | 4.3E0 | 1.3E1 | 4.4E1 | 8.5E1 | 1.4E2 | 1.0E-9 | 1.0E-9 | 1.7E3 | 5.0E2 | 905 | 13 | 337 | 13 | 0.58 |
| Ml | pg/ml | 1.0E-9 | 1.0E-9 | 5.6E0 | 8.4E-1 | 7.5E1 | 2.4E0 | 1.0E-9 | 1.0E-9 | 2.1E3 | 8.6E0 | 905 | 13 | 337 | 13 | 0.49 |
| Mm | pg/ml | 6.1E2 | 1.5E3 | 1.1E3 | 2.1E3 | 1.5E3 | 2.2E3 | 1.0E-9 | 6.2E1 | 1.2E4 | 6.5E3 | 905 | 13 | 337 | 13 | 0.66 |
| Mn | pg/ml | 5.6E0 | 1.6E1 | 1.0E1 | 1.7E1 | 2.3E1 | 1.5E1 | 1.0E-9 | 1.0E-9 | 3.5E2 | 5.1E1 | 905 | 13 | 337 | 13 | 0.69 |
| Mp | pg/ml | 1.0E-9 | 1.4E1 | 1.0E1 | 5.8E1 | 3.4E1 | 8.6E1 | 1.0E-9 | 1.0E-9 | 6.3E2 | 2.7E2 | 904 | 13 | 337 | 13 | 0.73 |
| Mq | pg/ml | 1.0E-9 | 1.0E-9 | 2.9E0 | 1.8E1 | 1.7E1 | 4.1E1 | 1.0E-9 | 1.0E-9 | 2.4E2 | 1.4E2 | 904 | 13 | 337 | 13 | 0.62 |
| Mr | pg/ml | 1.0E-9 | 5.7E0 | 2.8E1 | 2.8E2 | 1.6E2 | 9.3E2 | 1.0E-9 | 1.0E-9 | 2.2E3 | 3.4E3 | 904 | 13 | 337 | 13 | 0.67 |
| Ms | pg/ml | 4.1E2 | 5.1E2 | 5.6E2 | 5.0E2 | 6.5E2 | 4.0E2 | 1.0E-9 | 2.1E1 | 5.9E3 | 1.4E3 | 904 | 13 | 337 | 13 | 0.53 |
| Mt | pg/ml | 2.5E-1 | 4.6E0 | 1.1E1 | 1.9E1 | 1.2E2 | 3.1E1 | 1.0E-9 | 1.0E-9 | 3.2E3 | 9.5E1 | 904 | 13 | 337 | 13 | 0.80 |
| Mu | pg/ml | 1.0E-9 | 3.4E0 | 1.3E0 | 9.0E0 | 1.0E1 | 1.5E1 | 1.0E-9 | 1.0E-9 | 2.3E2 | 4.8E1 | 904 | 13 | 337 | 13 | 0.76 |
| Mv | pg/ml | 1.0E-9 | 8.6E1 | 7.0E1 | 2.4E2 | 3.2E2 | 3.1E2 | 1.0E-9 | 1.0E-9 | 4.5E3 | 9.0E2 | 904 | 13 | 337 | 13 | 0.72 |
| Mw | pg/ml | 3.8E1 | 4.3E2 | 5.2E2 | 1.3E3 | 3.3E3 | 1.9E3 | 1.0E-9 | 1.0E-9 | 6.2E4 | 5.3E3 | 904 | 13 | 337 | 13 | 0.74 |
| Mx | pg/ml | 1.0E-9 | 1.0E-9 | 2.8E-1 | 2.7E-1 | 1.5E0 | 6.2E-1 | 1.0E-9 | 1.0E-9 | 3.2E1 | 2.2E0 | 904 | 13 | 337 | 13 | 0.57 |
| My | pg/ml | 1.0E-9 | 1.1E2 | 4.7E2 | 3.7E2 | 3.0E3 | 5.8E2 | 1.0E-9 | 1.0E-9 | 3.9E4 | 1.9E3 | 904 | 13 | 337 | 13 | 0.66 |
| Mz | pg/ml | 1.1E1 | 2.9E1 | 3.0E1 | 6.3E1 | 9.9E1 | 6.8E1 | 1.0E-9 | 2.8E0 | 1.9E3 | 2.0E2 | 904 | 13 | 337 | 13 | 0.72 |
| Na | pg/ml | 1.0E-9 | 1.0E-9 | 7.3E-1 | 1.2E0 | 3.0E0 | 2.4E0 | 1.0E-9 | 1.0E-9 | 4.2E1 | 6.6E0 | 904 | 13 | 337 | 13 | 0.60 |
| Nb | pg/ml | 2.0E0 | 4.0E0 | 3.9E0 | 2.2E1 | 1.2E1 | 5.6E1 | 1.0E-9 | 1.0E-9 | 2.3E2 | 2.1E2 | 904 | 13 | 337 | 13 | 0.74 |
| Nc | pg/ml | 3.4E2 | 4.6E2 | 5.6E2 | 3.8E2 | 7.3E2 | 3.6E2 | 1.0E-9 | 1.0E-9 | 9.2E3 | 8.8E2 | 904 | 13 | 337 | 13 | 0.45 |
| Nd | pg/ml | 2.9E1 | 2.9E1 | 2.9E1 | 2.9E1 | 8.3E1 | 2.9E1 | 1.0E-9 | 2.9E0 | 2.1E3 | 1.0E2 | 904 | 13 | 337 | 13 | 0.53 |
| Ne | pg/ml | 4.4E2 | 3.6E2 | 5.8E2 | 4.3E2 | 5.7E2 | 3.3E2 | 1.0E-9 | 1.0E-9 | 7.0E3 | 1.1E3 | 904 | 13 | 337 | 13 | 0.46 |
| Nf | pg/ml | 1.0E-9 | 1.0E-9 | 3.0E0 | 2.4E0 | 1.1E1 | 5.0E0 | 1.0E-9 | 1.0E-9 | 1.6E2 | 1.8E1 | 904 | 13 | 337 | 13 | 0.53 |
| Ng | pg/ml | 1.9E1 | 3.9E1 | 1.3E2 | 1.1E2 | 2.5E2 | 1.4E2 | 1.0E-9 | 1.0E-9 | 2.3E3 | 4.0E2 | 904 | 13 | 337 | 13 | 0.53 |
| Nh | pg/ml | 6.9E1 | 5.0E1 | 9.0E1 | 5.5E1 | 8.2E1 | 3.8E1 | 1.0E-9 | 1.0E-9 | 5.6E2 | 1.3E2 | 904 | 13 | 337 | 13 | 0.38 |
| Ni | pg/ml | 1.0E-9 | 3.4E1 | 7.3E1 | 1.5E2 | 1.2E2 | 1.9E2 | 1.0E-9 | 1.0E-9 | 1.1E3 | 5.5E2 | 904 | 13 | 337 | 13 | 0.62 |
| Nj | pg/ml | 7.3E0 | 6.6E0 | 1.1E1 | 8.4E0 | 1.2E1 | 7.8E0 | 1.0E-9 | 1.0E-9 | 1.1E2 | 2.9E1 | 904 | 13 | 337 | 13 | 0.47 |
| Nk | pg/ml | 1.7E1 | 2.5E1 | 3.3E1 | 3.2E1 | 4.0E1 | 2.7E1 | 1.0E-9 | 1.0E-9 | 2.0E2 | 1.0E2 | 904 | 13 | 337 | 13 | 0.56 |
| Nl | pg/ml | 4.6E1 | 4.2E1 | 6.1E1 | 4.9E1 | 6.8E1 | 3.8E1 | 1.0E-9 | 1.0E-9 | 1.1E3 | 1.1E2 | 904 | 13 | 337 | 13 | 0.48 |
| Hq | pg/ml | 1.1E0 | 1.3E0 | 1.0E2 | 2.1E1 | 1.6E3 | 5.1E1 | 1.0E-9 | 1.0E-9 | 3.4E4 | 1.8E2 | 900 | 13 | 336 | 13 | 0.56 |
| Hr | pg/ml | 1.2E2 | 9.3E1 | 7.9E2 | 4.4E2 | 1.6E3 | 9.5E2 | 1.0E-9 | 1.0E-9 | 1.7E4 | 3.4E3 | 900 | 13 | 336 | 13 | 0.43 |
| Hu | pg/ml | 5.6E0 | 2.5E1 | 3.0E3 | 8.1E2 | 2.6E4 | 1.7E3 | 1.0E-9 | 1.0E-9 | 6.3E5 | 5.9E3 | 900 | 13 | 336 | 13 | 0.61 |
| Hv | pg/ml | 1.4E0 | 3.2E0 | 4.1E0 | 7.3E0 | 3.2E1 | 9.6E0 | 1.0E-9 | 4.7E-1 | 8.9E2 | 2.9E1 | 900 | 13 | 336 | 13 | 0.70 |
| Hw | pg/ml | 6.5E0 | 3.4E0 | 2.9E1 | 4.7E1 | 3.2E2 | 1.4E2 | 1.0E-9 | 5.1E-1 | 9.4E3 | 5.0E2 | 900 | 13 | 336 | 13 | 0.42 |
| Hx | pg/ml | 8.8E0 | 1.1E1 | 3.9E1 | 1.1E2 | 3.2E2 | 3.5E2 | 1.0E-9 | 1.0E-9 | 9.3E3 | 1.3E3 | 900 | 13 | 336 | 13 | 0.57 |
| Ih | ng/ml | 7.2E1 | 1.8E2 | 2.4E2 | 3.1E2 | 4.3E2 | 3.8E2 | 1.0E-9 | 1.0E-9 | 3.6E3 | 1.5E3 | 904 | 13 | 336 | 13 | 0.63 |

| Code | Units | Median | | Average | | Standard Deviation | | Minimum | | Maximum | | Number of Samples | | Number of Patients | | AUC |
|------|-------|--------|------|---------|------|--------------------|------|---------|------|---------|------|-------------------|------|--------------------|------|-----|
| | | NonDis | Dis | NonDis | Dis | NonDis | Dis | NonDis | Dis | NonDis | Dis | NonDis | Dis | NonDis | Dis | |
| Ii | ng/ml | 9.4E1 | 8.8E1 | 2.5E2 | 5.4E2 | 6.8E2 | 1.2E3 | 1.0E-9 | 3.2E1 | 1.0E4 | 4.5E3 | 903 | 13 | 336 | 13 | 0.61 |
| Ij | ng/ml | 7.7E1 | 1.3E2 | 2.0E2 | 1.8E2 | 9.9E2 | 1.6E2 | 1.0E-9 | 2.4E1 | 2.4E4 | 6.1E2 | 891 | 13 | 334 | 13 | 0.66 |
| Ik | ng/ml | 1.3E1 | 1.7E2 | 8.1E2 | 3.9E2 | 8.2E3 | 5.3E2 | 5.9E-1 | 2.6E0 | 1.2E5 | 1.5E3 | 898 | 13 | 334 | 13 | 0.67 |
| Il | ng/ml | 3.4E2 | 3.2E2 | 1.3E3 | 3.1E3 | 2.8E3 | 5.1E3 | 1.0E-9 | 1.0E-9 | 1.3E4 | 1.2E4 | 881 | 13 | 334 | 13 | 0.54 |
| Im | ng/ml | 2.1E2 | 7.0E2 | 3.9E2 | 7.8E2 | 6.0E2 | 1.0E3 | 1.3E1 | 5.5E1 | 6.2E3 | 4.0E3 | 897 | 13 | 335 | 13 | 0.64 |
| In | ng/ml | 3.7E0 | 6.2E-1 | 2.7E1 | 8.6E0 | 2.1E2 | 1.7E1 | 1.0E-9 | 1.0E-9 | 4.5E3 | 5.7E1 | 904 | 13 | 336 | 13 | 0.35 |
| Io | ng/ml | 8.1E3 | 1.2E4 | 2.4E4 | 1.4E4 | 1.5E5 | 1.1E4 | 1.0E-9 | 6.6E2 | 4.0E6 | 3.3E4 | 896 | 13 | 336 | 13 | 0.54 |
| Ip | ng/ml | 9.7E0 | 3.0E1 | 1.9E1 | 3.5E1 | 2.4E1 | 2.9E1 | 1.0E-9 | 4.4E-2 | 2.6E2 | 1.0E2 | 896 | 13 | 336 | 13 | 0.66 |
| Iq | ug/ml | 1.0E-1 | 3.1E-1 | 3.2E1 | 2.5E0 | 6.4E2 | 6.0E0 | 1.0E-9 | 1.0E-9 | 1.4E4 | 2.1E1 | 896 | 13 | 336 | 13 | 0.55 |
| Ir | ug/ml | 3.5E-1 | 1.2E0 | 3.9E0 | 3.3E1 | 2.8E1 | 7.1E1 | 1.0E-9 | 1.0E-9 | 5.1E2 | 2.4E2 | 895 | 13 | 336 | 13 | 0.71 |
| Is | ng/ml | 1.5E0 | 1.6E1 | 6.6E0 | 3.9E1 | 2.3E1 | 6.8E1 | 1.0E-9 | 1.0E0 | 5.5E2 | 2.6E2 | 896 | 13 | 336 | 13 | 0.82 |
| It | ng/ml | 2.0E0 | 3.8E0 | 2.4E1 | 6.4E0 | 1.4E2 | 8.4E0 | 1.0E-9 | 1.0E-9 | 2.8E3 | 2.4E1 | 896 | 13 | 336 | 13 | 0.56 |
| Iu | ng/ml | 2.2E2 | 1.5E2 | 1.4E3 | 4.7E3 | 4.2E3 | 9.2E3 | 1.0E-9 | 1.0E-9 | 2.9E4 | 2.4E4 | 896 | 13 | 336 | 13 | 0.49 |
| Iv | ng/ml | 1.3E1 | 5.7E1 | 6.1E1 | 3.8E2 | 5.5E2 | 1.0E3 | 1.0E-9 | 1.9E0 | 1.6E4 | 3.8E3 | 895 | 13 | 336 | 13 | 0.77 |
| Pz | ng/ml | 3.9E3 | 1.0E4 | 8.3E3 | 7.1E3 | 3.8E4 | 4.3E3 | 1.3E1 | 1.1E2 | 1.0E6 | 1.3E4 | 896 | 13 | 334 | 13 | 0.59 |
| Qa | ng/ml | 3.5E3 | 1.6E4 | 6.5E3 | 1.5E4 | 1.0E4 | 1.0E4 | 1.2E1 | 6.0E2 | 2.2E5 | 3.2E4 | 896 | 13 | 334 | 13 | 0.72 |
| Qb | ng/ml | 9.7E1 | 2.1E2 | 2.1E2 | 3.0E2 | 4.9E2 | 2.7E2 | 7.9E-1 | 1.4E1 | 8.3E3 | 7.6E2 | 896 | 13 | 334 | 13 | 0.63 |
| Qc | ng/ml | 2.3E2 | 6.3E2 | 6.3E2 | 6.5E2 | 5.6E3 | 5.0E2 | 1.0E-9 | 1.1E1 | 1.7E5 | 1.4E3 | 896 | 13 | 334 | 13 | 0.63 |
| Qd | ng/ml | 9.2E3 | 3.4E4 | 1.9E4 | 5.8E4 | 7.3E4 | 6.7E4 | 1.5E2 | 2.8E3 | 2.0E6 | 2.2E5 | 896 | 13 | 334 | 13 | 0.71 |
| Qe | ng/ml | 9.2E2 | 3.2E3 | 1.9E3 | 4.2E3 | 4.7E3 | 4.8E3 | 1.0E-9 | 1.9E2 | 9.7E4 | 1.8E4 | 896 | 13 | 334 | 13 | 0.72 |
| Jg | ng/ml | 5.0E2 | 1.6E3 | 8.3E2 | 2.1E3 | 1.0E3 | 2.1E3 | 1.0E-9 | 4.0E1 | 1.0E4 | 7.1E3 | 900 | 13 | 336 | 13 | 0.71 |
| Jh | ng/ml | 3.0E0 | 6.1E1 | 2.9E1 | 7.2E1 | 1.2E2 | 8.6E1 | 1.0E-9 | 1.9E-1 | 1.6E3 | 2.9E2 | 900 | 13 | 336 | 13 | 0.77 |
| Ji | ng/ml | 5.3E1 | 2.0E2 | 7.9E1 | 3.4E2 | 9.0E1 | 4.7E2 | 1.0E-9 | 1.7E1 | 1.3E3 | 1.8E3 | 900 | 13 | 336 | 13 | 0.82 |
| Jj | ng/ml | 6.1E2 | 1.9E2 | 1.6E3 | 2.7E2 | 1.2E4 | 2.6E2 | 1.5E0 | 2.0E1 | 3.4E5 | 9.7E2 | 900 | 13 | 336 | 13 | 0.22 |
| Jk | ng/ml | 3.0E0 | 1.5E1 | 2.1E1 | 5.2E1 | 4.7E1 | 7.1E1 | 1.0E-9 | 2.5E-1 | 3.9E2 | 2.4E2 | 900 | 13 | 336 | 13 | 0.67 |
| Jl | ng/ml | 4.1E-1 | 1.9E0 | 2.6E0 | 7.7E2 | 1.9E1 | 2.8E3 | 7.6E-4 | 1.3E-1 | 5.4E2 | 9.9E3 | 900 | 13 | 336 | 13 | 0.77 |
| Jm | ng/ml | 1.8E1 | 5.9E0 | 5.8E1 | 4.3E1 | 1.3E2 | 5.8E1 | 1.0E-9 | 2.5E-1 | 2.1E3 | 1.8E2 | 900 | 13 | 336 | 13 | 0.48 |
| Jn | pg/ml | 4.0E-1 | 1.7E0 | 3.7E0 | 6.3E0 | 3.8E1 | 1.1E1 | 1.0E-9 | 1.0E-9 | 7.3E2 | 3.7E1 | 899 | 13 | 336 | 13 | 0.77 |
| Jo | pg/ml | 3.6E3 | 6.3E3 | 4.9E3 | 8.2E3 | 5.0E3 | 9.7E3 | 2.0E1 | 6.6E2 | 1.0E5 | 3.6E4 | 900 | 13 | 336 | 13 | 0.57 |
| Jp | pg/ml | 7.0E4 | 9.1E4 | 7.3E4 | 1.1E5 | 3.8E4 | 3.7E4 | 5.8E2 | 6.0E4 | 3.8E5 | 1.9E5 | 900 | 13 | 336 | 13 | 0.76 |
| Jq | pg/ml | 9.6E1 | 1.9E2 | 1.6E2 | 4.6E2 | 3.6E2 | 9.7E2 | 1.0E0 | 1.4E1 | 8.7E3 | 3.7E3 | 900 | 13 | 336 | 13 | 0.69 |
| Jr | pg/ml | 5.3E0 | 3.1E1 | 4.4E1 | 8.2E1 | 4.7E2 | 1.4E2 | 1.0E-9 | 1.0E-9 | 1.1E4 | 5.0E2 | 900 | 13 | 336 | 13 | 0.75 |
| Js | pg/ml | 1.3E1 | 2.1E1 | 5.3E1 | 6.4E1 | 3.8E2 | 1.2E2 | 1.0E-9 | 7.6E0 | 1.0E4 | 4.4E2 | 900 | 13 | 336 | 13 | 0.69 |
| Jt | pg/ml | 2.6E3 | 4.2E3 | 3.2E3 | 6.2E3 | 2.8E3 | 6.7E3 | 2.2E1 | 1.0E3 | 5.2E4 | 2.5E4 | 900 | 13 | 336 | 13 | 0.64 |
| Lh | pg/ml | 1.3E4 | 3.7E4 | 2.2E4 | 6.9E4 | 3.2E4 | 1.1E5 | 1.0E-9 | 1.3E3 | 4.8E5 | 4.1E5 | 900 | 13 | 337 | 13 | 0.72 |
| Li | pg/ml | 3.3E3 | 1.8E4 | 1.7E4 | 7.5E4 | 6.2E4 | 1.2E5 | 1.0E-9 | 3.4E1 | 1.3E6 | 4.1E5 | 900 | 13 | 337 | 13 | 0.68 |
| Lj | pg/ml | 2.8E3 | 1.1E4 | 2.3E4 | 2.6E4 | 6.6E4 | 3.2E4 | 1.0E-9 | 2.2E2 | 5.2E5 | 1.0E5 | 900 | 13 | 337 | 13 | 0.62 |
| Nv | pg/ml | 4.0E3 | 1.2E4 | 1.1E4 | 2.5E4 | 4.3E4 | 3.9E4 | 1.0E-9 | 1.0E3 | 1.1E6 | 1.3E5 | 906 | 13 | 337 | 13 | 0.72 |
| Nw | pg/ml | 8.9E3 | 1.9E4 | 1.3E4 | 4.0E4 | 1.7E4 | 5.5E4 | 8.6E1 | 6.8E3 | 2.1E5 | 2.1E5 | 906 | 13 | 337 | 13 | 0.77 |
| Nx | pg/ml | 2.2E2 | 2.6E2 | 4.1E2 | 8.4E2 | 6.5E2 | 9.3E2 | 1.0E-9 | 1.0E-9 | 7.4E3 | 2.8E3 | 906 | 13 | 337 | 13 | 0.65 |
| Ny | pg/ml | 6.4E0 | 1.7E1 | 5.7E1 | 6.8E1 | 8.5E2 | 1.6E2 | 1.0E-9 | 1.0E-9 | 2.5E4 | 6.1E2 | 906 | 13 | 337 | 13 | 0.67 |
| Oe | pg/ml | 6.8E1 | 1.8E1 | 2.9E2 | 2.4E2 | 7.5E2 | 3.6E2 | 1.0E-9 | 1.0E-9 | 1.9E4 | 1.1E3 | 897 | 13 | 336 | 13 | 0.48 |
| Of | pg/ml | 1.7E2 | 1.9E2 | 6.1E3 | 3.4E3 | 2.8E4 | 7.2E3 | 1.0E-9 | 7.4E0 | 6.2E5 | 2.4E4 | 905 | 13 | 337 | 13 | 0.54 |
| Og | pg/ml | 8.2E-2 | 8.2E-2 | 4.8E-1 | 1.8E-1 | 1.6E0 | 3.3E-1 | 1.0E-9 | 1.0E-9 | 2.0E1 | 1.2E0 | 905 | 13 | 337 | 13 | 0.46 |
| Oh | pg/ml | 2.7E0 | 6.4E0 | 2.0E1 | 2.6E1 | 1.5E2 | 3.9E1 | 1.0E-9 | 9.7E-1 | 3.5E3 | 1.3E2 | 905 | 13 | 337 | 13 | 0.73 |
| Oi | pg/ml | 2.6E0 | 1.0E-9 | 6.3E0 | 5.7E0 | 9.8E0 | 8.5E0 | 1.0E-9 | 1.0E-9 | 8.6E1 | 2.9E1 | 905 | 13 | 337 | 13 | 0.46 |
| Ok | pg/ml | 3.9E2 | 9.8E2 | 5.6E2 | 6.6E3 | 6.0E2 | 1.9E4 | 1.3E1 | 2.0E2 | 7.8E3 | 7.0E4 | 905 | 13 | 337 | 13 | 0.75 |
| Om | pg/ml | 4.0E2 | 1.1E3 | 8.9E2 | 2.8E3 | 2.7E3 | 3.6E3 | 1.0E-9 | 2.4E2 | 5.1E4 | 1.3E4 | 905 | 13 | 337 | 13 | 0.81 |
| On | pg/ml | 1.8E2 | 5.3E2 | 3.0E2 | 1.9E3 | 4.2E2 | 3.2E3 | 1.0E-9 | 1.0E2 | 4.5E3 | 9.8E3 | 905 | 13 | 337 | 13 | 0.82 |
| Oy | pg/ml | 4.9E-1 | 6.3E-1 | 5.7E0 | 3.6E0 | 2.9E1 | 8.9E0 | 1.0E-9 | 1.0E-9 | 4.0E2 | 3.3E1 | 904 | 13 | 336 | 13 | 0.52 |
| Oz | pg/ml | 3.1E-3 | 1.0E-9 | 3.1E-1 | 2.3E0 | 1.3E0 | 7.7E0 | 1.0E-9 | 1.0E-9 | 2.9E1 | 2.8E1 | 904 | 13 | 336 | 13 | 0.45 |
| Pa | pg/ml | 3.9E-1 | 7.2E-1 | 1.6E0 | 1.9E1 | 6.1E0 | 6.2E1 | 1.0E-9 | 1.0E-9 | 1.0E2 | 2.3E2 | 904 | 13 | 336 | 13 | 0.56 |
| Pb | pg/ml | 1.0E-9 | 1.0E-9 | 8.0E-1 | 2.4E-1 | 1.6E1 | 5.5E-1 | 1.0E-9 | 1.0E-9 | 4.9E2 | 1.9E0 | 904 | 13 | 336 | 13 | 0.52 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|-----|--------|-----|------|
| | | NonDis | Dis | NonDis | Dis | NonDis | Dis | NonDis | Dis | NonDis | Dis | NonDis | Dis | NonDis | Dis | |
| Pc | pg/ml | 4.4E-2 | 1.5E-1 | 3.6E-1 | 3.4E0 | 8.8E-1 | 1.0E1 | 1.0E-9 | 1.0E-9 | 1.6E1 | 3.8E1 | 904 | 13 | 336 | 13 | 0.54 |
| Pd | pg/ml | 1.9E0 | 3.6E0 | 5.1E0 | 1.1E1 | 2.9E1 | 1.7E1 | 1.0E-9 | 2.2E-1 | 8.4E2 | 5.5E1 | 904 | 13 | 336 | 13 | 0.60 |
| Pe | pg/ml | 2.2E1 | 8.7E1 | 1.2E2 | 1.3E3 | 4.4E2 | 4.1E3 | 1.0E-9 | 2.0E0 | 6.7E3 | 1.5E4 | 904 | 13 | 336 | 13 | 0.67 |
| Pf | pg/ml | 1.6E0 | 1.4E1 | 1.1E1 | 2.6E1 | 6.0E1 | 4.0E1 | 1.0E-9 | 4.7E-1 | 1.5E3 | 1.5E2 | 904 | 13 | 336 | 13 | 0.76 |
| Pg | pg/ml | 3.4E0 | 1.0E1 | 4.3E1 | 8.4E1 | 3.4E2 | 2.2E2 | 1.0E-9 | 2.9E-1 | 7.7E3 | 8.1E2 | 904 | 13 | 336 | 13 | 0.63 |
| aA | mg/dL | 8.0E-1 | 1.4E0 | 9.4E-1 | 1.7E0 | 4.9E-1 | 1.2E0 | 2.0E-1 | 5.5E-1 | 4.2E0 | 4.7E0 | 2667 | 22 | 507 | 22 | 0.73 |
| aC | mg/mL | 2.8E0 | 2.5E0 | 3.1E0 | 3.1E0 | 1.4E0 | 1.4E0 | 7.7E-1 | 1.6E0 | 8.9E0 | 5.5E0 | 535 | 9 | 207 | 9 | 0.50 |
| aD | ug/mL | 3.2E0 | 3.8E0 | 4.5E0 | 7.0E0 | 3.8E0 | 6.5E0 | 4.3E-1 | 1.1E0 | 3.5E1 | 2.1E1 | 535 | 9 | 207 | 9 | 0.61 |
| aE | mg/mL | 5.6E-1 | 6.7E-1 | 5.7E-1 | 6.6E-1 | 1.5E-1 | 1.5E-1 | 1.8E-1 | 4.7E-1 | 1.1E0 | 1.0E0 | 535 | 9 | 207 | 9 | 0.68 |
| aF | ng/mL | 2.2E0 | 2.7E0 | 4.0E0 | 5.2E0 | 5.7E0 | 5.8E0 | 4.3E-3 | 4.3E-3 | 5.0E1 | 1.5E1 | 535 | 9 | 207 | 9 | 0.51 |
| aG | mg/mL | 1.4E-1 | 9.0E-2 | 1.6E-1 | 1.5E-1 | 8.7E-2 | 8.9E-2 | 1.7E-2 | 6.9E-2 | 5.4E-1 | 3.0E-1 | 535 | 9 | 207 | 9 | 0.46 |
| aH | ug/mL | 7.5E1 | 6.5E1 | 8.2E1 | 8.5E1 | 4.4E1 | 4.9E1 | 4.6E0 | 3.2E1 | 2.9E2 | 1.8E2 | 535 | 9 | 207 | 9 | 0.49 |
| aI | ug/mL | 1.9E2 | 1.5E2 | 1.9E2 | 1.5E2 | 6.0E1 | 5.5E1 | 2.8E1 | 7.5E1 | 3.7E2 | 2.5E2 | 535 | 9 | 207 | 9 | 0.30 |
| aJ | ug/mL | 2.5E0 | 4.3E0 | 3.1E0 | 7.3E0 | 2.2E0 | 7.1E0 | 7.3E-1 | 1.8E0 | 1.7E1 | 2.3E1 | 535 | 9 | 207 | 9 | 0.72 |
| aK | ng/mL | 1.6E0 | 1.0E0 | 2.4E0 | 1.6E0 | 2.6E0 | 1.5E0 | 2.9E-4 | 1.3E-1 | 1.8E1 | 5.0E0 | 535 | 9 | 207 | 9 | 0.42 |
| aL | mg/mL | 8.0E-1 | 8.1E-1 | 8.1E-1 | 7.9E-1 | 2.6E-1 | 2.7E-1 | 1.9E-1 | 4.5E-1 | 1.7E0 | 1.2E0 | 535 | 9 | 207 | 9 | 0.47 |
| aM | U/mL | 2.2E1 | 3.3E1 | 4.9E1 | 4.5E1 | 1.1E2 | 3.0E1 | 4.2E-2 | 5.2E0 | 1.6E3 | 8.6E1 | 535 | 9 | 207 | 9 | 0.61 |
| aN | U/mL | 1.4E1 | 2.5E1 | 2.2E1 | 2.8E1 | 3.1E1 | 2.9E1 | 2.5E-3 | 3.6E0 | 3.8E2 | 1.0E2 | 535 | 9 | 207 | 9 | 0.60 |
| aO | pg/mL | 3.1E1 | 1.2E2 | 3.0E2 | 8.1E2 | 7.8E2 | 1.2E3 | 6.0E-2 | 8.2E0 | 6.6E3 | 3.5E3 | 535 | 9 | 207 | 9 | 0.67 |
| aP | ng/mL | 1.7E0 | 2.8E0 | 2.1E0 | 5.8E0 | 1.8E0 | 8.5E0 | 4.5E-1 | 1.1E0 | 2.8E1 | 2.8E1 | 535 | 9 | 207 | 9 | 0.76 |
| aQ | ng/mL | 3.0E-1 | 4.1E-1 | 4.5E-1 | 3.9E-1 | 4.5E-1 | 3.1E-1 | 2.0E-4 | 5.2E-2 | 4.0E0 | 1.1E0 | 535 | 9 | 207 | 9 | 0.50 |
| aR | ng/mL | 1.7E0 | 3.4E0 | 2.8E0 | 3.0E0 | 3.3E0 | 2.0E0 | 1.8E-1 | 2.5E-1 | 3.4E1 | 5.4E0 | 535 | 9 | 207 | 9 | 0.58 |
| aS | ng/mL | 2.6E-1 | 3.9E-1 | 6.3E-1 | 7.9E-1 | 1.7E0 | 9.1E-1 | 4.2E-3 | 6.0E-2 | 3.3E1 | 2.8E0 | 535 | 9 | 207 | 9 | 0.57 |
| aU | pg/mL | 7.5E1 | 5.7E1 | 1.3E2 | 8.1E1 | 1.5E2 | 8.2E1 | 7.4E-2 | 7.4E-2 | 1.3E3 | 2.3E2 | 535 | 9 | 207 | 9 | 0.41 |
| aV | ng/mL | 6.2E-1 | 5.3E-1 | 1.1E0 | 6.8E-1 | 1.8E0 | 5.3E-1 | 7.6E-4 | 1.5E-1 | 3.3E1 | 1.6E0 | 535 | 9 | 207 | 9 | 0.45 |
| aW | pg/mL | 1.9E1 | 1.5E1 | 2.0E1 | 6.1E1 | 1.8E1 | 1.4E2 | 7.2E-2 | 7.2E-2 | 2.4E2 | 4.3E2 | 535 | 9 | 207 | 9 | 0.45 |
| aX | ng/mL | 9.5E0 | 1.5E1 | 1.5E1 | 5.2E1 | 1.8E1 | 6.6E1 | 3.0E-1 | 2.1E0 | 2.2E2 | 1.7E2 | 535 | 9 | 207 | 9 | 0.63 |
| aY | pg/mL | 5.7E1 | 8.6E1 | 7.5E1 | 1.1E2 | 8.1E1 | 6.6E1 | 4.1E-1 | 2.7E1 | 1.2E3 | 2.0E2 | 535 | 9 | 207 | 9 | 0.66 |
| aZ | pg/mL | 2.2E2 | 3.0E2 | 5.0E2 | 4.3E2 | 9.5E2 | 5.5E2 | 1.7E0 | 2.4E1 | 1.2E4 | 1.8E3 | 535 | 9 | 207 | 9 | 0.54 |
| bA | ng/mL | 8.8E0 | 8.0E1 | 3.5E1 | 4.1E2 | 9.7E1 | 5.8E2 | 3.0E-2 | 4.7E0 | 9.4E2 | 1.5E3 | 535 | 9 | 207 | 9 | 0.77 |
| bB | ng/mL | 3.0E2 | 2.5E2 | 3.2E2 | 3.0E2 | 1.7E2 | 2.4E2 | 2.1E0 | 6.6E1 | 1.0E3 | 7.8E2 | 535 | 9 | 207 | 9 | 0.42 |
| bC | ng/mL | 3.5E2 | 3.2E2 | 6.1E2 | 1.1E3 | 8.1E2 | 1.3E3 | 9.8E0 | 1.8E2 | 4.7E3 | 4.0E3 | 535 | 9 | 207 | 9 | 0.62 |
| bE | mg/mL | 5.5E0 | 8.3E0 | 5.8E0 | 7.6E0 | 2.1E0 | 3.0E0 | 9.8E-1 | 3.4E0 | 1.3E1 | 1.2E1 | 535 | 9 | 207 | 9 | 0.67 |
| bF | pg/mL | 2.1E1 | 2.6E1 | 1.6E2 | 7.3E2 | 8.9E2 | 2.1E3 | 5.0E-2 | 1.2E1 | 1.1E4 | 6.3E3 | 535 | 9 | 207 | 9 | 0.62 |
| bG | ng/mL | 1.6E0 | 1.7E0 | 2.7E0 | 4.7E0 | 3.2E0 | 9.4E0 | 2.2E-2 | 1.9E-1 | 2.6E1 | 3.0E1 | 535 | 9 | 207 | 9 | 0.47 |
| bH | pg/mL | 5.7E-1 | 5.0E0 | 4.8E0 | 7.6E0 | 1.4E1 | 8.1E0 | 5.7E-1 | 5.7E-1 | 2.8E2 | 2.2E1 | 535 | 9 | 207 | 9 | 0.63 |
| bI | ng/mL | 4.0E-3 | 4.0E-3 | 6.2E-2 | 8.8E-2 | 1.6E-1 | 1.3E-1 | 4.0E-3 | 4.0E-3 | 1.3E0 | 3.1E-1 | 535 | 9 | 207 | 9 | 0.58 |
| bJ | mg/mL | 2.3E0 | 2.3E0 | 2.7E0 | 3.2E0 | 2.1E0 | 2.4E0 | 2.5E-4 | 1.2E0 | 1.3E1 | 8.9E0 | 535 | 9 | 207 | 9 | 0.58 |
| bL | pg/mL | 3.7E0 | 6.5E0 | 8.1E0 | 9.9E0 | 1.0E1 | 8.5E0 | 4.6E-2 | 1.2E0 | 8.0E1 | 2.4E1 | 535 | 9 | 207 | 9 | 0.59 |
| bM | mg/mL | 1.7E0 | 2.2E0 | 2.1E0 | 2.5E0 | 1.5E0 | 9.7E-1 | 9.2E-3 | 1.2E0 | 8.9E0 | 3.8E0 | 535 | 9 | 207 | 9 | 0.65 |
| bN | ng/mL | 4.5E1 | 3.3E1 | 1.3E2 | 5.3E1 | 2.8E2 | 7.5E1 | 1.4E-1 | 5.2E0 | 1.9E3 | 2.5E2 | 535 | 9 | 207 | 9 | 0.40 |
| bO | ng/mL | 4.0E-2 | 4.0E-2 | 1.1E1 | 9.3E-1 | 2.5E1 | 2.7E0 | 4.0E-2 | 4.0E-2 | 2.0E2 | 8.0E0 | 535 | 9 | 207 | 9 | 0.36 |
| bP | mg/mL | 5.4E-1 | 7.7E-1 | 7.7E-1 | 9.7E-1 | 6.8E-1 | 7.6E-1 | 4.9E-2 | 1.4E-1 | 4.8E0 | 2.7E0 | 535 | 9 | 207 | 9 | 0.62 |
| bQ | pg/mL | 1.6E1 | 4.1E1 | 5.8E1 | 5.5E1 | 5.9E2 | 5.8E1 | 1.5E-1 | 1.5E-1 | 1.3E4 | 1.6E2 | 535 | 9 | 207 | 9 | 0.61 |
| bR | ng/mL | 1.2E-2 | 1.2E-2 | 1.3E-1 | 4.2E-2 | 4.2E-1 | 8.9E-2 | 1.2E-2 | 1.2E-2 | 8.7E0 | 2.8E-1 | 535 | 9 | 207 | 9 | 0.37 |
| bS | pg/mL | 9.4E-1 | 9.4E-1 | 6.8E0 | 8.3E0 | 2.6E1 | 2.2E1 | 9.4E-1 | 9.4E-1 | 3.9E2 | 6.7E1 | 535 | 9 | 207 | 9 | 0.49 |
| bU | ng/mL | 1.2E-1 | 1.3E-2 | 1.9E-1 | 6.9E-2 | 3.5E-1 | 1.3E-1 | 1.3E-2 | 1.3E-2 | 6.6E0 | 4.1E-1 | 535 | 9 | 207 | 9 | 0.30 |
| bV | pg/mL | 4.7E2 | 9.6E2 | 5.6E2 | 8.0E2 | 5.6E2 | 3.6E2 | 1.5E2 | 3.0E2 | 1.2E4 | 1.3E3 | 535 | 9 | 207 | 9 | 0.71 |
| bW | pg/mL | 3.3E2 | 5.5E2 | 6.1E2 | 1.2E3 | 1.7E3 | 1.5E3 | 8.4E1 | 2.0E2 | 2.5E4 | 3.9E3 | 535 | 9 | 207 | 9 | 0.65 |
| bX | ng/mL | 1.4E-3 | 2.5E-5 | 2.8E-3 | 5.7E-4 | 3.4E-3 | 1.1E-3 | 2.5E-5 | 2.5E-5 | 2.0E-2 | 3.1E-3 | 535 | 9 | 207 | 9 | 0.32 |
| bZ | pg/mL | 2.4E2 | 1.6E3 | 8.3E2 | 6.3E3 | 3.7E3 | 1.4E4 | 1.5E-1 | 1.3E2 | 5.8E4 | 4.3E4 | 535 | 9 | 207 | 9 | 0.76 |
| cA | pg/mL | 6.0E-1 | 6.0E-1 | 2.6E0 | 6.0E-1 | 1.6E1 | 0.0E0 | 6.0E-1 | 6.0E-1 | 3.7E2 | 6.0E-1 | 535 | 9 | 207 | 9 | 0.44 |
| cB | ng/mL | 5.7E-2 | 4.0E-2 | 8.8E-2 | 5.7E-2 | 1.0E-1 | 6.7E-2 | 1.7E-3 | 1.7E-3 | 5.7E-1 | 2.1E-1 | 535 | 9 | 207 | 9 | 0.40 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| cC | pg/mL | 4.6E1 | 3.5E1 | 4.7E1 | 3.0E1 | 3.9E1 | 2.2E1 | 1.0E0 | 1.0E0 | 4.5E2 | 6.7E1 | 535 | 9 | 207 | 9 | 0.35 |
| cD | pg/mL | 5.3E0 | 2.9E0 | 1.5E1 | 6.8E0 | 5.2E1 | 1.2E1 | 3.3E-1 | 3.3E-1 | 7.2E2 | 3.9E1 | 535 | 9 | 207 | 9 | 0.34 |
| cE | pg/mL | 3.8E1 | 5.7E1 | 1.6E2 | 2.4E2 | 4.6E2 | 4.0E2 | 1.2E-1 | 1.7E0 | 3.8E3 | 1.3E3 | 535 | 9 | 207 | 9 | 0.58 |
| cF | pg/mL | 1.2E1 | 5.3E-1 | 2.0E1 | 7.6E0 | 3.0E1 | 1.3E1 | 5.3E-1 | 5.3E-1 | 2.7E2 | 3.8E1 | 535 | 9 | 207 | 9 | 0.36 |
| cG | pg/mL | 4.6E1 | 1.2E2 | 1.0E2 | 1.7E2 | 4.8E2 | 1.7E2 | 6.4E0 | 1.9E1 | 1.0E4 | 4.9E2 | 535 | 9 | 207 | 9 | 0.66 |
| cH | uIU/mL | 2.8E0 | 6.0E0 | 6.0E0 | 1.2E1 | 1.2E1 | 1.7E1 | 8.6E-3 | 8.6E-3 | 1.6E2 | 5.3E1 | 535 | 9 | 207 | 9 | 0.58 |
| cI | ng/mL | 5.7E0 | 9.9E0 | 1.2E1 | 2.4E1 | 1.6E1 | 3.6E1 | 1.0E-3 | 1.7E0 | 1.2E2 | 1.2E2 | 535 | 9 | 207 | 9 | 0.67 |
| cJ | ug/mL | 6.2E1 | 5.0E1 | 1.1E2 | 9.1E1 | 1.4E2 | 1.1E2 | 4.0E0 | 5.6E0 | 9.6E2 | 3.4E2 | 535 | 9 | 207 | 9 | 0.45 |
| cK | ng/mL | 3.8E-3 | 3.8E-3 | 4.8E-2 | 8.7E-3 | 1.7E-1 | 1.5E-2 | 3.8E-3 | 3.8E-3 | 1.7E0 | 4.8E-2 | 535 | 9 | 207 | 9 | 0.47 |
| cL | pg/mL | 2.0E2 | 2.1E2 | 3.8E2 | 3.9E2 | 1.2E3 | 4.0E2 | 1.6E1 | 7.9E1 | 2.4E4 | 1.3E3 | 535 | 9 | 207 | 9 | 0.59 |
| cM | pg/mL | 2.7E2 | 2.0E2 | 2.9E2 | 2.2E2 | 1.9E2 | 7.2E1 | 8.7E0 | 1.3E2 | 1.6E3 | 3.5E2 | 535 | 9 | 207 | 9 | 0.37 |
| cN | pg/mL | 1.2E2 | 1.2E2 | 1.3E2 | 1.5E2 | 6.2E1 | 6.4E1 | 3.8E1 | 8.6E1 | 1.1E3 | 2.8E2 | 535 | 9 | 207 | 9 | 0.58 |
| cO | pg/mL | 2.2E2 | 3.1E2 | 3.0E2 | 4.2E2 | 8.5E2 | 4.1E2 | 5.4E1 | 1.7E2 | 1.9E4 | 1.5E3 | 535 | 9 | 207 | 9 | 0.67 |
| cP | ng/mL | 2.5E3 | 3.4E3 | 2.6E3 | 3.7E3 | 9.0E2 | 1.5E3 | 6.2E2 | 1.4E3 | 5.7E3 | 5.6E3 | 535 | 9 | 207 | 9 | 0.74 |
| cQ | ng/mL | 4.9E-2 | 9.1E-2 | 1.4E-1 | 2.3E-1 | 2.8E-1 | 2.5E-1 | 2.0E-3 | 2.0E-3 | 2.2E0 | 7.3E-1 | 535 | 9 | 207 | 9 | 0.65 |
| cR | ng/mL | 2.9E2 | 5.5E2 | 5.0E2 | 8.4E2 | 7.8E2 | 7.0E2 | 2.0E1 | 2.5E2 | 8.9E3 | 2.3E3 | 535 | 9 | 207 | 9 | 0.72 |
| cS | ng/mL | 2.6E2 | 8.8E2 | 3.8E2 | 1.6E3 | 3.8E2 | 2.1E3 | 4.1E1 | 1.7E2 | 2.7E3 | 7.1E3 | 535 | 9 | 207 | 9 | 0.83 |
| cT | ng/mL | 3.3E1 | 1.3E2 | 8.7E1 | 6.5E2 | 1.9E2 | 7.9E2 | 3.6E0 | 1.3E1 | 2.1E3 | 1.9E3 | 535 | 9 | 207 | 9 | 0.70 |
| cU | ng/mL | 5.4E1 | 9.3E1 | 7.5E1 | 1.3E2 | 9.4E1 | 1.1E2 | 5.4E0 | 2.9E1 | 1.6E3 | 3.5E2 | 535 | 9 | 207 | 9 | 0.65 |
| cV | ng/mL | 1.8E-1 | 1.9E-1 | 3.9E-1 | 4.7E-1 | 2.1E0 | 7.8E-1 | 3.4E-4 | 8.4E-2 | 4.7E1 | 2.5E0 | 535 | 9 | 207 | 9 | 0.57 |
| cW | mIU/mL | 5.2E-2 | 8.6E-2 | 1.3E-1 | 1.2E-1 | 6.5E-1 | 1.0E-1 | 3.7E-4 | 3.6E-2 | 9.7E0 | 2.9E-1 | 535 | 9 | 207 | 9 | 0.66 |
| cX | ng/mL | 1.1E-1 | 2.9E-2 | 1.3E0 | 2.0E-1 | 4.2E0 | 3.5E-1 | 2.3E-4 | 2.3E-4 | 2.8E1 | 1.1E0 | 535 | 9 | 207 | 9 | 0.39 |
| cY | ng/mL | 8.6E0 | 5.1E0 | 1.3E1 | 8.3E0 | 1.3E1 | 9.6E0 | 1.5E-1 | 6.0E-1 | 8.3E1 | 3.1E1 | 535 | 9 | 207 | 9 | 0.38 |
| cZ | ug/mL | 1.5E1 | 1.6E1 | 1.6E1 | 1.6E1 | 7.2E0 | 8.4E0 | 2.3E0 | 7.0E0 | 5.7E1 | 3.0E1 | 535 | 9 | 207 | 9 | 0.50 |
| dA | pg/mL | 3.3E2 | 4.6E2 | 3.7E2 | 4.4E2 | 2.9E2 | 1.9E2 | 9.0E1 | 1.7E2 | 5.8E3 | 6.6E2 | 535 | 9 | 207 | 9 | 0.64 |
| dB | ug/mL | 1.7E1 | 1.9E1 | 1.7E1 | 1.8E1 | 1.5E1 | 9.6E0 | 9.4E-1 | 2.4E0 | 2.5E2 | 2.8E1 | 535 | 9 | 207 | 9 | 0.57 |
| dC | nmol/L | 3.5E1 | 3.3E1 | 3.9E1 | 3.8E1 | 1.8E1 | 1.6E1 | 7.6E0 | 2.3E1 | 1.4E2 | 7.6E1 | 535 | 9 | 207 | 9 | 0.48 |
| dD | ug/mL | 3.6E1 | 3.2E1 | 3.7E1 | 3.5E1 | 1.1E1 | 1.4E1 | 1.3E1 | 1.6E1 | 7.6E1 | 6.4E1 | 535 | 9 | 207 | 9 | 0.42 |
| dE | ng/mL | 4.7E-1 | 5.4E-1 | 5.9E-1 | 9.4E-1 | 6.9E-1 | 1.1E0 | 8.4E-3 | 8.4E-3 | 7.2E0 | 3.3E0 | 535 | 9 | 207 | 9 | 0.56 |
| dF | ng/mL | 2.3E2 | 2.9E2 | 2.8E2 | 4.0E2 | 2.0E2 | 2.6E2 | 5.6E1 | 1.0E2 | 1.3E3 | 8.3E2 | 535 | 9 | 207 | 9 | 0.65 |
| dG | ng/mL | 1.1E1 | 1.5E1 | 1.5E1 | 2.2E1 | 1.3E1 | 1.8E1 | 2.2E0 | 7.5E0 | 1.8E2 | 6.5E1 | 535 | 9 | 207 | 9 | 0.66 |
| dH | pg/mL | 7.7E0 | 1.1E1 | 1.3E1 | 1.8E1 | 3.6E1 | 2.2E1 | 4.0E-2 | 5.8E0 | 6.7E2 | 7.6E1 | 535 | 9 | 207 | 9 | 0.66 |
| dI | pg/mL | 4.6E-1 | 4.6E-1 | 2.1E0 | 3.8E0 | 1.5E1 | 6.0E0 | 4.6E-1 | 4.6E-1 | 3.3E2 | 1.9E1 | 535 | 9 | 207 | 9 | 0.64 |
| dJ | ng/mL | 1.9E0 | 1.7E0 | 2.1E0 | 2.0E0 | 1.2E0 | 8.6E-1 | 3.2E-2 | 8.4E-1 | 6.9E0 | 3.2E0 | 535 | 9 | 207 | 9 | 0.48 |
| dK | uIU/mL | 1.9E0 | 6.8E-1 | 3.1E0 | 1.0E0 | 6.1E0 | 1.2E0 | 2.8E-4 | 1.4E-2 | 7.9E1 | 3.0E0 | 535 | 9 | 207 | 9 | 0.26 |
| dL | ng/mL | 8.8E2 | 1.3E3 | 1.0E3 | 1.3E3 | 5.7E2 | 5.1E2 | 2.6E2 | 5.8E2 | 4.8E3 | 2.3E3 | 535 | 9 | 207 | 9 | 0.69 |
| dM | pg/mL | 9.7E2 | 1.3E3 | 1.3E3 | 2.6E3 | 1.3E3 | 2.7E3 | 3.4E2 | 6.0E2 | 1.6E4 | 8.8E3 | 535 | 9 | 207 | 9 | 0.62 |
| dN | ug/mL | 9.3E1 | 1.6E2 | 1.0E2 | 1.5E2 | 4.0E1 | 3.8E1 | 1.6E1 | 8.6E1 | 3.3E2 | 2.0E2 | 535 | 9 | 207 | 9 | 0.82 |
| dR | pg/ml | 1.6E3 | 1.1E3 | 2.3E3 | 2.0E3 | 2.3E3 | 2.4E3 | 1.4E2 | 1.3E2 | 1.5E4 | 7.3E3 | 365 | 7 | 197 | 7 | 0.42 |
| eF | ng/ml | 4.1E0 | 7.5E0 | 5.0E0 | 1.1E1 | 4.0E0 | 8.7E0 | 1.2E0 | 4.8E0 | 4.6E1 | 2.9E1 | 380 | 7 | 198 | 7 | 0.84 |
| fP | ng/ml | 2.6E2 | 3.9E2 | 2.9E2 | 3.1E2 | 1.7E2 | 1.7E2 | 1.8E0 | 1.6E1 | 1.0E3 | 5.0E2 | 346 | 7 | 189 | 7 | 0.58 |
| fR | ng/ml | 1.4E5 | 2.9E5 | 1.9E5 | 3.4E5 | 1.5E5 | 2.9E5 | 2.9E4 | 1.9E2 | 8.3E5 | 8.7E5 | 359 | 7 | 109 | 7 | 0.69 |
| gL | pg/ml | 6.5E4 | 1.2E5 | 7.0E4 | 1.1E5 | 2.9E4 | 6.6E4 | 1.4E4 | 4.1E4 | 2.0E5 | 2.2E5 | 365 | 7 | 197 | 7 | 0.65 |
| gP | U/ml | 2.7E2 | 3.0E2 | 2.8E2 | 3.1E2 | 1.1E2 | 8.3E1 | 1.2E1 | 1.9E2 | 1.1E3 | 4.4E2 | 376 | 7 | 198 | 7 | 0.64 |

Figure 3.

Descriptive statistics for the assays used to form the panels presented in this figure.

| Code | Units | Median | | Average | | Standard Deviation | | Minimum | | Maximum | | Number of Samples | | Number of Patients | | AUC |
|------|-------|--------|-----|---------|-----|---------|-----|--------|--------|---------|---------|--------|-----|--------|-----|-----|
| | | NonDis | Dis | NonDis | Dis | NonDis | Dis | NonDis | Dis | NonDis | Dis | NonDis | Dis | NonDis | Dis | |
| Aa | mg/dL | 6.5E1 | 8.8E1 | 7.6E1 | 9.9E1 | 5.2E1 | 6.2E1 | 2.0E0 | 1.4E1 | 4.0E2 | 2.4E2 | 1392 | 30 | 230 | 30 | 0.61 |
| Ad | ug/mL | 3.4E-2 | 7.6E-2 | 6.5E-2 | 3.9E-1 | 8.4E-2 | 1.6E0 | 6.8E-4 | 4.3E-3 | 5.4E-1 | 8.5E0 | 357 | 29 | 135 | 29 | 0.66 |
| Af | ng/mL | 1.0E0 | 1.0E0 | 1.4E1 | 1.5E1 | 6.0E1 | 4.7E1 | 1.7E-3 | 1.7E-3 | 5.3E2 | 2.6E2 | 357 | 29 | 135 | 29 | 0.52 |
| Aj | ug/mL | 1.8E0 | 7.2E-1 | 2.7E0 | 1.9E0 | 2.5E0 | 2.4E0 | 1.5E-3 | 2.1E-3 | 6.1E0 | 6.1E0 | 357 | 29 | 135 | 29 | 0.41 |
| Al | mg/mL | 8.7E-5 | 9.5E-5 | 2.5E-4 | 2.3E-4 | 4.1E-4 | 3.1E-4 | 2.5E-6 | 7.6E-6 | 1.9E-3 | 1.3E-3 | 357 | 29 | 135 | 29 | 0.55 |
| An | U/mL | 4.9E1 | 7.7E1 | 1.6E2 | 5.6E2 | 4.6E2 | 1.5E3 | 9.8E-4 | 9.6E-1 | 5.5E3 | 7.8E3 | 357 | 29 | 135 | 29 | 0.63 |
| Ao | pg/mL | 8.5E1 | 9.8E1 | 6.0E2 | 1.8E2 | 3.9E3 | 2.5E2 | 2.8E0 | 5.4E0 | 3.9E4 | 1.3E3 | 357 | 29 | 135 | 29 | 0.57 |
| Ap | ng/mL | 3.1E1 | 4.6E1 | 4.2E1 | 5.7E1 | 4.3E1 | 4.8E1 | 8.4E-5 | 3.1E0 | 2.9E2 | 1.7E2 | 357 | 29 | 135 | 29 | 0.60 |
| Ar | ng/mL | 8.5E-1 | 2.6E0 | 1.5E1 | 5.5E0 | 2.2E2 | 9.7E0 | 3.4E-3 | 3.4E-3 | 4.1E3 | 5.1E1 | 357 | 29 | 135 | 29 | 0.67 |
| As | ng/mL | 9.5E-3 | 1.0E-2 | 1.3E-2 | 5.8E-2 | 1.8E-2 | 2.3E-1 | 1.7E-3 | 1.7E-3 | 1.3E-1 | 1.2E0 | 357 | 29 | 135 | 29 | 0.53 |
| Aw | pg/mL | 1.6E1 | 1.7E1 | 1.6E1 | 1.9E1 | 5.9E0 | 8.2E0 | 2.9E-2 | 1.1E1 | 4.8E1 | 5.1E1 | 357 | 29 | 135 | 29 | 0.58 |
| Ax | ng/mL | 2.2E0 | 8.7E0 | 1.4E1 | 5.9E1 | 5.8E1 | 1.4E2 | 1.3E-2 | 1.2E-2 | 7.7E2 | 6.2E2 | 357 | 29 | 135 | 29 | 0.67 |
| Ba | ng/mL | 6.2E1 | 2.5E2 | 4.2E2 | 6.9E2 | 1.2E3 | 1.1E3 | 3.7E-1 | 6.3E0 | 8.1E3 | 4.4E3 | 357 | 29 | 135 | 29 | 0.65 |
| Bb | ng/mL | 2.8E0 | 5.0E0 | 6.1E0 | 7.5E0 | 1.5E1 | 6.5E0 | 4.1E-3 | 2.6E-1 | 2.5E2 | 1.9E1 | 357 | 29 | 135 | 29 | 0.63 |
| Bc | ng/mL | 3.4E1 | 7.2E1 | 1.0E2 | 1.8E2 | 1.9E2 | 3.0E2 | 1.1E-1 | 8.0E0 | 1.2E3 | 1.0E3 | 357 | 29 | 135 | 29 | 0.64 |
| Bg | ng/mL | 7.7E-2 | 1.3E-1 | 4.0E0 | 9.5E-1 | 1.9E1 | 1.3E0 | 5.3E-4 | 5.3E-4 | 2.5E2 | 4.8E0 | 357 | 29 | 135 | 29 | 0.61 |
| Bn | ng/mL | 5.6E-2 | 5.6E-2 | 1.1E0 | 3.2E0 | 1.9E0 | 1.1E1 | 5.6E-2 | 5.6E-2 | 9.7E0 | 5.8E1 | 357 | 29 | 135 | 29 | 0.51 |
| Bo | ng/mL | 1.2E1 | 2.0E1 | 1.4E1 | 1.9E1 | 2.0E1 | 1.5E1 | 1.6E-2 | 1.6E-2 | 2.8E2 | 5.3E1 | 357 | 29 | 135 | 29 | 0.62 |
| Ch | ulU/mL | 1.2E0 | 6.6E-1 | 2.1E1 | 5.6E0 | 1.1E2 | 1.9E1 | 3.4E-3 | 3.9E-2 | 1.8E3 | 1.1E2 | 357 | 29 | 135 | 29 | 0.37 |
| Co | pg/mL | 3.8E1 | 5.0E1 | 1.8E2 | 1.1E2 | 1.1E3 | 1.7E2 | 1.5E-1 | 1.5E-1 | 1.7E4 | 8.2E2 | 357 | 29 | 135 | 29 | 0.59 |
| Cp | ng/mL | 2.2E1 | 2.2E1 | 2.8E1 | 7.5E1 | 3.3E1 | 2.3E2 | 6.0E-1 | 6.0E-1 | 3.7E2 | 1.3E3 | 357 | 29 | 135 | 29 | 0.59 |
| Cq | ng/mL | 2.8E-2 | 3.1E-2 | 1.5E-1 | 1.7E0 | 9.5E-1 | 9.1E0 | 8.0E-4 | 8.0E-4 | 1.7E1 | 4.9E1 | 357 | 29 | 135 | 29 | 0.53 |
| Cs | ng/mL | 6.6E1 | 2.6E2 | 3.0E2 | 1.3E3 | 8.6E2 | 3.4E3 | 2.7E-2 | 5.2E0 | 1.1E4 | 1.8E4 | 357 | 29 | 135 | 29 | 0.67 |
| Ct | ng/mL | 8.9E-1 | 1.3E-1 | 4.0E1 | 3.7E1 | 1.1E2 | 1.2E2 | 1.1E-4 | 1.1E-4 | 6.2E2 | 4.7E2 | 357 | 29 | 135 | 29 | 0.38 |
| Cu | ng/mL | 2.4E-1 | 3.7E-1 | 4.0E-1 | 2.9E0 | 7.0E-1 | 1.2E1 | 9.6E-3 | 4.6E-2 | 9.2E0 | 6.6E1 | 357 | 29 | 135 | 29 | 0.65 |
| Cv | ng/mL | 4.1E0 | 7.0E0 | 1.8E1 | 6.4E1 | 4.9E1 | 1.4E2 | 1.4E-4 | 5.1E-2 | 5.3E2 | 5.2E2 | 357 | 29 | 135 | 29 | 0.55 |
| Cw | mIU/mL | 3.0E-2 | 3.4E-2 | 3.8E-2 | 2.8E-1 | 3.2E-2 | 1.2E0 | 8.9E-4 | 4.1E-3 | 2.4E-1 | 6.8E0 | 357 | 29 | 135 | 29 | 0.56 |
| Cx | ng/mL | 1.5E-1 | 1.1E0 | 5.1E1 | 7.0E1 | 9.9E1 | 1.2E2 | 9.3E-5 | 9.3E-5 | 3.9E2 | 3.9E2 | 357 | 29 | 135 | 29 | 0.50 |
| Db | ug/mL | 7.2E0 | 8.2E0 | 8.6E0 | 8.8E0 | 7.3E0 | 6.6E0 | 4.5E-1 | 8.8E-1 | 5.9E1 | 2.3E1 | 357 | 29 | 135 | 29 | 0.53 |
| Dc | nmol/L | 1.9E-2 | 2.4E-2 | 5.7E-2 | 6.2E-1 | 1.4E-1 | 2.6E0 | 5.2E-6 | 1.0E-3 | 1.6E0 | 1.4E1 | 357 | 29 | 135 | 29 | 0.59 |
| Dd | ug/mL | 6.9E-2 | 4.3E-2 | 1.8E-1 | 2.5E-1 | 2.7E-1 | 6.6E-1 | 1.9E-4 | 3.4E-3 | 1.9E0 | 3.6E0 | 357 | 29 | 135 | 29 | 0.49 |
| De | ng/mL | 3.4E-3 | 3.4E-3 | 7.8E-2 | 9.6E-2 | 1.4E-1 | 2.2E-1 | 3.4E-3 | 3.4E-3 | 9.2E-1 | 9.0E-1 | 357 | 29 | 135 | 29 | 0.49 |
| Dg | ng/mL | 2.9E1 | 5.2E1 | 4.2E1 | 6.3E1 | 3.8E1 | 5.0E1 | 1.0E-1 | 2.3E0 | 1.9E2 | 1.9E2 | 357 | 29 | 135 | 29 | 0.62 |
| Di | pg/mL | 2.0E0 | 2.8E0 | 2.2E0 | 2.8E0 | 2.0E0 | 2.1E0 | 1.8E-1 | 1.8E-1 | 1.3E1 | 9.0E0 | 357 | 29 | 135 | 29 | 0.60 |
| Dk | uIU/mL | 1.7E-2 | 2.5E-2 | 9.6E-2 | 8.4E-2 | 5.7E-1 | 1.6E-1 | 1.1E-4 | 1.1E-4 | 8.9E0 | 6.3E-1 | 357 | 29 | 135 | 29 | 0.59 |
| Dl | ng/mL | 2.3E2 | 3.7E2 | 3.1E2 | 4.5E2 | 2.8E2 | 4.1E2 | 1.7E0 | 1.8E1 | 1.5E3 | 1.6E3 | 357 | 29 | 135 | 29 | 0.60 |
| Dp | ng/ml | 2.3E0 | 1.7E0 | 5.1E0 | 1.4E1 | 7.9E0 | 3.8E1 | 3.7E-3 | 3.7E-3 | 4.6E1 | 2.0E2 | 217 | 29 | 135 | 29 | 0.46 |
| Dr | pg/ml | 2.1E1 | 3.0E1 | 4.7E1 | 8.2E2 | 7.1E1 | 2.7E3 | 7.5E-1 | 7.5E-1 | 5.2E2 | 1.0E4 | 125 | 15 | 71 | 15 | 0.61 |
| Du | pg/ml | 6.1E1 | 2.4E2 | 8.9E2 | 2.7E3 | 3.1E3 | 6.4E3 | 1.2E0 | 1.2E0 | 2.6E4 | 2.4E4 | 80 | 14 | 62 | 14 | 0.58 |
| Ef | ng/ml | 1.5E-1 | 2.4E-1 | 8.1E-1 | 8.7E-1 | 1.6E0 | 1.7E0 | 5.7E-4 | 1.1E-2 | 9.5E0 | 7.0E0 | 261 | 29 | 134 | 29 | 0.55 |
| Wm | % | 4.9E-1 | 1.8E0 | 2.3E1 | 1.3E2 | 1.6E2 | 3.1E2 | 5.4E-2 | 8.5E-2 | 2.4E3 | 1.0E3 | 283 | 33 | 151 | 33 | 0.60 |
| Ed | pg/ml | 5.2E-1 | 2.9E1 | 6.3E1 | 6.6E1 | 5.0E2 | 1.0E2 | 5.2E-1 | 5.2E-1 | 7.3E3 | 5.0E2 | 217 | 29 | 134 | 29 | 0.63 |
| Yf | ng/mL | 1.6E1 | 1.5E1 | 1.1E2 | 3.7E1 | 7.0E2 | 7.8E1 | 2.9E-1 | 2.9E-1 | 6.6E3 | 2.9E2 | 89 | 13 | 69 | 13 | 0.45 |
| Tj | pg/mL | 3.7E-1 | 3.7E-1 | 6.8E1 | 2.3E1 | 3.3E2 | 6.3E1 | 3.6E-1 | 3.7E-1 | 3.5E3 | 3.1E2 | 258 | 30 | 136 | 30 | 0.51 |
| Po | pg/ml | 6.7E-1 | 7.0E0 | 9.1E0 | 2.5E1 | 2.5E1 | 4.6E1 | 8.0E-3 | 2.6E-2 | 2.7E2 | 2.2E2 | 580 | 43 | 207 | 43 | 0.68 |
| Ti | ug/mL | 3.1E0 | 5.2E0 | 4.4E0 | 6.6E0 | 3.9E0 | 4.7E0 | 8.7E-3 | 8.0E-1 | 1.7E1 | 1.8E1 | 132 | 19 | 99 | 19 | 0.66 |
| Em | ng/ml | 9.2E-3 | 2.9E-3 | 6.7E-2 | 1.5E-1 | 1.2E-1 | 4.8E-1 | 1.9E-16 | 8.4E-4 | 6.0E-1 | 1.9E0 | 149 | 16 | 71 | 16 | 0.42 |
| Et | ng/ml | 1.3E3 | 3.0E3 | 1.6E3 | 2.8E3 | 1.1E3 | 1.2E3 | 7.7E1 | 1.1E2 | 5.0E3 | 5.0E3 | 579 | 43 | 207 | 43 | 0.77 |
| Eq | pg/ml | 1.9E2 | 5.8E1 | 3.5E2 | 2.3E2 | 4.0E2 | 3.6E2 | 1.0E0 | 1.0E0 | 1.8E3 | 1.3E3 | 80 | 14 | 62 | 14 | 0.37 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Th | ug/mL | 1.1E0 | 8.5E-1 | 1.7E0 | 1.2E0 | 1.6E0 | 1.2E0 | 2.6E-3 | 2.6E-3 | 1.2E1 | 4.2E0 | 132 | 19 | 99 | 19 | 0.39 |
| Fa | ng/ml | 4.0E1 | 1.1E2 | 1.3E2 | 2.8E2 | 6.0E2 | 5.1E2 | 3.4E-2 | 6.0E-1 | 8.0E3 | 2.5E3 | 212 | 29 | 133 | 29 | 0.72 |
| Ez | ng/ml | 5.0E0 | 5.0E0 | 2.0E1 | 1.3E1 | 5.9E1 | 2.0E1 | 1.3E-2 | 5.8E-2 | 7.1E2 | 8.8E1 | 217 | 29 | 135 | 29 | 0.52 |
| Fb | ng/ml | 2.5E1 | 2.8E1 | 2.3E1 | 2.8E1 | 1.1E1 | 9.5E0 | 6.6E-1 | 5.9E-1 | 5.7E1 | 4.3E1 | 213 | 29 | 133 | 29 | 0.61 |
| Ex | ng/ml | 7.8E-2 | 1.7E-1 | 2.5E-1 | 2.5E-1 | 7.6E-1 | 3.2E-1 | 3.5E-5 | 1.7E-4 | 8.9E0 | 1.2E0 | 190 | 21 | 89 | 21 | 0.59 |
| Fc | pg/ml | 2.2E-1 | 2.2E-1 | 1.9E2 | 2.9E1 | 1.6E3 | 8.2E1 | 2.2E-1 | 2.2E-1 | 1.5E4 | 3.1E2 | 82 | 14 | 62 | 14 | 0.58 |
| Fd | pg/ml | 9.8E-1 | 2.2E2 | 8.8E2 | 2.4E3 | 3.8E3 | 6.6E3 | 4.5E-1 | 9.8E-1 | 3.3E4 | 2.5E4 | 82 | 14 | 62 | 14 | 0.60 |
| Fi | pg/ml | 2.5E-1 | 2.5E-1 | 2.2E2 | 2.3E1 | 1.6E3 | 6.8E1 | 2.5E-1 | 2.5E-1 | 1.4E4 | 2.5E2 | 82 | 14 | 62 | 14 | 0.51 |
| Fn | ng/ml | 2.1E-1 | 2.1E-1 | 6.3E0 | 4.3E0 | 2.9E1 | 6.9E0 | 1.1E-14 | 2.1E-1 | 4.2E2 | 2.7E1 | 217 | 29 | 135 | 29 | 0.49 |
| Fp | ng/ml | 1.2E1 | 4.6E0 | 2.4E1 | 5.2E1 | 2.8E1 | 4.0E1 | 6.0E-3 | 2.1E0 | 1.4E2 | 1.4E2 | 606 | 42 | 209 | 42 | 0.73 |
| Fr | ng/ml | 3.3E4 | 9.9E4 | 1.1E5 | 2.4E5 | 1.7E5 | 2.6E5 | 1.9E2 | 1.3E3 | 9.0E5 | 8.4E5 | 686 | 44 | 210 | 44 | 0.68 |
| Fw | pg/ml | 8.5E-1 | 2.9E0 | 7.0E1 | 6.4E1 | 5.4E2 | 1.9E2 | 1.1E-14 | 1.2E-1 | 6.9E3 | 9.1E2 | 259 | 30 | 135 | 30 | 0.59 |
| Fy | ng/ml | 3.5E1 | 5.1E1 | 5.5E1 | 1.2E2 | 5.7E1 | 1.7E2 | 1.2E-1 | 5.3E0 | 3.3E2 | 6.5E2 | 216 | 28 | 134 | 28 | 0.61 |
| Gh | pg/ml | 3.7E0 | 3.7E0 | 8.1E1 | 8.0E0 | 2.9E2 | 1.3E1 | 2.9E-2 | 2.9E-2 | 1.8E3 | 4.6E1 | 80 | 14 | 62 | 14 | 0.46 |
| Gb | % | 4.2E1 | 3.9E1 | 4.4E1 | 6.6E1 | 3.3E1 | 7.2E1 | 2.2E0 | 2.1E1 | 1.9E2 | 3.0E2 | 82 | 14 | 61 | 14 | 0.60 |
| Gc | ng/ml | 9.2E1 | 1.5E2 | 1.4E2 | 1.8E2 | 1.7E2 | 1.3E2 | 6.4E0 | 2.9E1 | 1.2E3 | 4.7E2 | 134 | 15 | 72 | 15 | 0.66 |
| Gd | ng/ml | 3.1E1 | 2.9E1 | 3.3E1 | 3.4E1 | 1.7E1 | 2.3E1 | 3.0E0 | 7.6E0 | 8.1E1 | 8.0E1 | 149 | 15 | 68 | 15 | 0.49 |
| Gn | U/ml | 3.6E-1 | 1.5E-1 | 1.3E0 | 8.7E0 | 3.3E0 | 2.9E1 | 1.3E-3 | 5.6E-3 | 3.0E1 | 1.1E2 | 119 | 15 | 69 | 15 | 0.47 |
| Gl | pg/ml | 7.8E3 | 9.7E3 | 1.1E4 | 1.4E4 | 9.4E3 | 1.1E4 | 9.1E1 | 7.7E2 | 3.3E4 | 3.1E4 | 252 | 30 | 135 | 30 | 0.59 |
| Gp | U/ml | 1.5E0 | 6.3E-1 | 4.0E0 | 3.3E0 | 6.8E0 | 8.7E0 | 1.3E-3 | 1.5E-2 | 6.7E1 | 4.8E1 | 261 | 30 | 135 | 30 | 0.38 |
| Gz | ug/ml | 1.4E0 | 1.0E0 | 9.7E0 | 4.0E0 | 4.2E1 | 5.0E0 | 2.9E-16 | 1.0E-1 | 4.8E2 | 1.5E1 | 143 | 21 | 86 | 21 | 0.46 |
| Ha | ng/ml | 2.7E0 | 4.9E0 | 9.9E0 | 1.3E1 | 2.1E1 | 2.6E1 | 1.7E-2 | 6.4E-3 | 1.3E2 | 1.0E2 | 215 | 29 | 134 | 29 | 0.57 |
| Nm | pg/ml | 1.6E4 | 2.8E4 | 3.2E4 | 6.1E4 | 8.8E4 | 1.3E5 | 1.0E-9 | 1.0E-9 | 1.6E6 | 8.2E5 | 583 | 43 | 209 | 43 | 0.62 |
| Nn | pg/ml | 1.5E2 | 4.1E2 | 2.2E3 | 6.2E3 | 9.4E3 | 2.1E4 | 1.0E-9 | 1.0E-9 | 1.0E5 | 1.1E5 | 583 | 43 | 209 | 43 | 0.63 |
| No | pg/ml | 1.5E1 | 4.1E1 | 3.7E1 | 1.1E2 | 1.3E2 | 1.8E2 | 1.0E-9 | 1.7E0 | 2.5E3 | 7.7E2 | 583 | 43 | 209 | 43 | 0.71 |
| Nq | pg/ml | 2.0E0 | 4.0E0 | 1.9E1 | 3.1E1 | 7.6E1 | 6.4E1 | 1.0E-9 | 1.0E-9 | 1.2E3 | 2.9E2 | 583 | 43 | 209 | 43 | 0.55 |
| Nr | pg/ml | 8.8E-1 | 6.1E0 | 3.3E1 | 7.3E1 | 2.1E2 | 2.3E2 | 1.0E-9 | 1.0E-9 | 4.1E3 | 1.4E3 | 583 | 43 | 209 | 43 | 0.66 |
| Ns | pg/ml | 1.0E-9 | 1.0E-9 | 1.1E1 | 1.9E0 | 6.1E1 | 1.2E1 | 1.0E-9 | 1.0E-9 | 1.1E3 | 7.9E1 | 583 | 43 | 209 | 43 | 0.47 |
| Nt | pg/ml | 1.0E2 | 1.6E2 | 1.4E2 | 2.1E2 | 1.1E2 | 2.0E2 | 1.0E-9 | 4.4E1 | 1.5E3 | 1.2E3 | 583 | 43 | 209 | 43 | 0.66 |
| Nu | pg/ml | 2.3E1 | 5.2E1 | 5.7E1 | 9.8E1 | 9.5E1 | 1.1E2 | 1.0E-9 | 1.0E-9 | 8.9E2 | 3.7E2 | 583 | 43 | 209 | 43 | 0.63 |
| Lu | pg/ml | 1.0E4 | 1.0E4 | 1.7E4 | 9.3E3 | 4.7E4 | 5.8E3 | 3.5E2 | 1.1E3 | 7.5E5 | 2.5E4 | 585 | 43 | 209 | 43 | 0.45 |
| Lv | pg/ml | 1.0E-9 | 2.0E1 | 1.1E1 | 3.1E1 | 2.1E1 | 4.0E1 | 1.0E-9 | 1.0E-9 | 2.4E2 | 1.6E2 | 585 | 43 | 209 | 43 | 0.65 |
| Lw | pg/ml | 1.0E-9 | 1.0E-9 | 4.0E-1 | 2.1E0 | 4.3E0 | 7.6E0 | 1.0E-9 | 1.0E-9 | 8.0E1 | 4.0E1 | 585 | 43 | 209 | 43 | 0.54 |
| Lx | pg/ml | 1.0E-9 | 1.6E2 | 1.5E2 | 5.6E2 | 4.5E2 | 9.4E2 | 1.0E-9 | 1.0E-9 | 6.2E3 | 4.5E3 | 585 | 43 | 209 | 43 | 0.73 |
| Ly | pg/ml | 1.0E-9 | 1.0E-9 | 1.0E1 | 1.1E1 | 2.0E1 | 1.7E1 | 1.0E-9 | 1.0E-9 | 1.6E2 | 6.3E1 | 585 | 43 | 209 | 43 | 0.51 |
| Lz | pg/ml | 1.0E-9 | 1.0E-9 | 4.9E0 | 5.6E0 | 3.6E1 | 1.6E1 | 1.0E-9 | 1.0E-9 | 6.0E2 | 6.2E1 | 585 | 43 | 209 | 43 | 0.53 |
| Ma | pg/ml | 3.1E2 | 7.4E2 | 1.3E3 | 2.4E3 | 3.8E3 | 5.1E3 | 1.0E-9 | 1.0E-9 | 6.5E4 | 3.1E4 | 585 | 43 | 209 | 43 | 0.65 |
| Mb | pg/ml | 2.5E1 | 3.7E1 | 3.1E1 | 3.7E1 | 1.6E1 | 1.7E1 | 5.4E0 | 4.1E0 | 2.1E2 | 7.1E1 | 585 | 43 | 209 | 43 | 0.59 |
| Mc | pg/ml | 1.0E-9 | 1.0E-9 | 4.2E-2 | 1.0E-9 | 5.8E-1 | 0.0E0 | 1.0E-9 | 1.0E-9 | 1.3E1 | 1.0E-9 | 585 | 43 | 209 | 43 | 0.49 |
| Md | pg/ml | 1.0E-9 | 1.0E-9 | 4.8E-1 | 9.5E-1 | 3.3E0 | 4.6E0 | 1.0E-9 | 1.0E-9 | 6.5E1 | 2.9E1 | 585 | 43 | 209 | 43 | 0.50 |
| Me | pg/ml | 3.2E1 | 2.3E1 | 3.1E1 | 2.5E1 | 2.0E1 | 3.0E1 | 1.0E-9 | 1.0E-9 | 3.2E2 | 1.8E2 | 585 | 43 | 209 | 43 | 0.35 |
| Mf | pg/ml | 1.0E-9 | 1.0E-9 | 5.2E-1 | 7.8E-1 | 3.1E0 | 1.9E0 | 1.0E-9 | 1.0E-9 | 5.6E1 | 9.1E0 | 585 | 43 | 209 | 43 | 0.57 |
| Mg | pg/ml | 2.0E0 | 2.3E0 | 7.7E0 | 8.6E0 | 1.3E1 | 1.3E1 | 1.0E-9 | 1.0E-9 | 9.2E1 | 5.9E1 | 585 | 43 | 209 | 43 | 0.53 |
| Mh | pg/ml | 1.0E-9 | 1.0E-9 | 1.5E0 | 2.6E0 | 1.1E1 | 7.3E0 | 1.0E-9 | 1.0E-9 | 1.6E2 | 4.2E1 | 585 | 43 | 209 | 43 | 0.59 |
| Mi | pg/ml | 1.0E-9 | 1.0E-9 | 5.9E-1 | 3.6E0 | 6.3E0 | 1.7E1 | 1.0E-9 | 1.0E-9 | 1.2E2 | 1.1E2 | 585 | 43 | 209 | 43 | 0.52 |
| Mj | pg/ml | 1.0E-9 | 1.0E-9 | 4.8E0 | 1.5E1 | 2.7E1 | 4.4E1 | 1.0E-9 | 1.0E-9 | 4.6E2 | 2.1E2 | 585 | 43 | 209 | 43 | 0.55 |
| Mk | pg/ml | 5.3E-1 | 2.0E0 | 1.8E1 | 1.8E1 | 1.1E2 | 7.6E1 | 1.0E-9 | 1.0E-9 | 1.7E3 | 5.0E2 | 585 | 43 | 209 | 43 | 0.53 |
| Ml | pg/ml | 1.0E-9 | 1.0E-9 | 6.3E0 | 2.5E1 | 9.0E1 | 9.3E1 | 1.0E-9 | 1.0E-9 | 2.1E3 | 5.1E2 | 585 | 43 | 209 | 43 | 0.58 |
| Mm | pg/ml | 5.9E2 | 8.8E2 | 9.8E2 | 1.8E3 | 1.1E3 | 2.3E3 | 1.0E-9 | 1.0E-9 | 7.3E3 | 1.2E4 | 585 | 43 | 209 | 43 | 0.61 |
| Mn | pg/ml | 5.4E0 | 9.3E0 | 1.1E1 | 1.4E1 | 2.6E1 | 2.0E1 | 1.0E-9 | 4.2E-1 | 3.5E2 | 1.3E2 | 585 | 43 | 209 | 43 | 0.62 |
| Mp | pg/ml | 1.0E-9 | 7.4E0 | 9.2E0 | 2.0E1 | 3.2E1 | 3.2E1 | 1.0E-9 | 1.0E-9 | 6.3E2 | 1.3E2 | 584 | 43 | 209 | 43 | 0.63 |
| Mq | pg/ml | 1.0E-9 | 1.0E-9 | 2.9E0 | 1.1E1 | 1.7E1 | 2.4E1 | 1.0E-9 | 1.0E-9 | 2.1E2 | 1.0E2 | 584 | 43 | 209 | 43 | 0.62 |
| Mr | pg/ml | 1.0E-9 | 1.0E-9 | 1.9E1 | 1.7E2 | 8.5E1 | 5.9E2 | 1.0E-9 | 1.0E-9 | 1.4E3 | 3.4E3 | 584 | 43 | 209 | 43 | 0.59 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ms | pg/ml | 4.1E2 | 2.6E2 | 5.6E2 | 3.7E2 | 6.5E2 | 3.9E2 | 1.0E-9 | 1.0E-9 | 5.6E3 | 1.9E3 | 584 | 43 | 209 | 43 | 0.41 |
| Mt | pg/ml | 2.2E-1 | 2.0E0 | 7.4E0 | 9.2E1 | 5.0E1 | 4.9E2 | 1.0E-9 | 1.0E-9 | 8.7E2 | 3.2E3 | 584 | 43 | 209 | 43 | 0.71 |
| Mu | pg/ml | 1.0E-9 | 1.0E-9 | 1.6E0 | 2.1E0 | 1.3E1 | 6.3E0 | 1.0E-9 | 1.0E-9 | 2.3E2 | 3.5E1 | 584 | 43 | 209 | 43 | 0.59 |
| Mv | pg/ml | 1.0E-9 | 1.0E-9 | 7.6E1 | 1.2E2 | 3.6E2 | 4.2E2 | 1.0E-9 | 1.0E-9 | 4.5E3 | 2.5E3 | 584 | 43 | 209 | 43 | 0.56 |
| Mw | pg/ml | 3.4E1 | 8.6E1 | 4.8E2 | 6.1E2 | 3.1E3 | 1.5E3 | 1.0E-9 | 1.0E-9 | 6.2E4 | 5.9E3 | 584 | 43 | 209 | 43 | 0.64 |
| Mx | pg/ml | 1.0E-9 | 1.0E-9 | 2.5E-1 | 9.2E-1 | 1.5E0 | 3.1E0 | 1.0E-9 | 1.0E-9 | 3.2E1 | 2.0E1 | 584 | 43 | 209 | 43 | 0.59 |
| My | pg/ml | 1.0E-9 | 1.0E-9 | 4.7E2 | 2.0E2 | 3.1E3 | 7.5E2 | 1.0E-9 | 1.0E-9 | 3.9E4 | 4.6E3 | 584 | 43 | 209 | 43 | 0.51 |
| Mz | pg/ml | 1.0E1 | 2.6E1 | 2.5E1 | 1.1E2 | 7.2E1 | 3.5E2 | 1.0E-9 | 1.0E-9 | 1.2E3 | 1.9E3 | 584 | 43 | 209 | 43 | 0.69 |
| Na | pg/ml | 1.0E-9 | 1.0E-9 | 7.4E-1 | 2.2E0 | 3.0E0 | 6.9E0 | 1.0E-9 | 1.0E-9 | 3.6E1 | 4.2E1 | 584 | 43 | 209 | 43 | 0.55 |
| Nb | pg/ml | 1.9E0 | 2.5E0 | 4.2E0 | 1.1E1 | 1.4E1 | 3.5E1 | 1.0E-9 | 1.0E-9 | 2.3E2 | 2.1E2 | 584 | 43 | 209 | 43 | 0.60 |
| Nc | pg/ml | 4.0E2 | 2.3E2 | 6.3E2 | 3.3E2 | 7.9E2 | 3.5E2 | 1.0E-9 | 1.0E-9 | 9.2E3 | 1.1E3 | 584 | 43 | 209 | 43 | 0.41 |
| Nd | pg/ml | 2.9E1 | 6.5E0 | 2.7E1 | 1.6E1 | 5.4E1 | 2.0E1 | 1.0E-9 | 1.0E-9 | 1.2E3 | 9.4E1 | 584 | 43 | 209 | 43 | 0.37 |
| Ne | pg/ml | 4.7E2 | 2.9E2 | 6.1E2 | 3.6E2 | 6.0E2 | 3.2E2 | 1.0E-9 | 1.0E-9 | 7.0E3 | 1.7E3 | 584 | 43 | 209 | 43 | 0.37 |
| Nf | pg/ml | 1.0E-9 | 1.0E-9 | 3.0E0 | 7.8E0 | 1.1E1 | 2.7E1 | 1.0E-9 | 1.0E-9 | 1.6E2 | 1.3E2 | 584 | 43 | 209 | 43 | 0.52 |
| Ng | pg/ml | 3.6E1 | 1.0E1 | 1.4E2 | 7.9E1 | 2.6E2 | 1.9E2 | 1.0E-9 | 1.0E-9 | 2.3E3 | 1.2E3 | 584 | 43 | 209 | 43 | 0.44 |
| Nh | pg/ml | 7.1E1 | 3.7E1 | 9.5E1 | 5.6E1 | 8.8E1 | 6.8E1 | 1.0E-9 | 4.1E0 | 5.6E2 | 4.3E2 | 584 | 43 | 209 | 43 | 0.34 |
| Ni | pg/ml | 1.0E-9 | 1.4E1 | 7.8E1 | 1.4E2 | 1.2E2 | 2.1E2 | 1.0E-9 | 1.0E-9 | 7.1E2 | 1.1E3 | 584 | 43 | 209 | 43 | 0.57 |
| Nj | pg/ml | 7.9E0 | 2.8E0 | 1.1E1 | 6.4E0 | 1.2E1 | 7.9E0 | 1.0E-9 | 1.0E-9 | 8.3E1 | 3.3E1 | 584 | 43 | 209 | 43 | 0.36 |
| Nk | pg/ml | 2.0E1 | 1.7E1 | 3.5E1 | 3.4E1 | 4.0E1 | 4.5E1 | 1.0E-9 | 1.0E-9 | 2.0E2 | 2.0E2 | 584 | 43 | 209 | 43 | 0.50 |
| Nl | pg/ml | 4.9E1 | 2.4E1 | 6.6E1 | 3.5E1 | 7.4E1 | 3.3E1 | 1.0E-9 | 1.0E-9 | 1.1E3 | 1.3E2 | 584 | 43 | 209 | 43 | 0.36 |
| Hl | pg/ml | 1.8E1 | 1.8E1 | 4.0E1 | 3.1E2 | 6.7E1 | 9.5E2 | 1.0E-9 | 1.0E-9 | 3.5E2 | 3.6E3 | 82 | 14 | 62 | 14 | 0.53 |
| Ho | pg/ml | 1.7E1 | 2.0E1 | 2.9E1 | 4.6E1 | 7.7E1 | 9.9E1 | 1.0E-9 | 7.6E0 | 7.0E2 | 3.9E2 | 82 | 14 | 62 | 14 | 0.59 |
| Hp | ng/ml | 1.6E0 | 3.8E0 | 1.2E2 | 2.6E2 | 3.0E2 | 4.1E2 | 1.0E-9 | 2.0E-1 | 8.9E2 | 8.9E2 | 82 | 14 | 62 | 14 | 0.62 |
| Tz | pg/ml | 5.3E3 | 6.9E3 | 1.4E4 | 2.0E4 | 6.9E4 | 6.8E4 | 1.0E-9 | 6.8E2 | 1.0E6 | 3.7E5 | 219 | 29 | 133 | 29 | 0.55 |
| Ua | pg/ml | 3.9E3 | 5.1E3 | 1.6E4 | 8.7E3 | 2.9E4 | 1.2E4 | 1.0E-9 | 9.4E2 | 1.9E5 | 6.6E4 | 219 | 29 | 133 | 29 | 0.52 |
| Ub | pg/ml | 5.7E2 | 4.8E2 | 8.5E2 | 8.1E2 | 1.1E3 | 9.4E2 | 1.0E-9 | 1.2E1 | 9.8E3 | 4.1E3 | 219 | 29 | 133 | 29 | 0.48 |
| Ue | pg/ml | 2.9E1 | 2.7E1 | 3.6E1 | 4.2E1 | 3.2E1 | 3.5E1 | 9.8E-2 | 5.9E0 | 3.5E2 | 1.4E2 | 219 | 29 | 133 | 29 | 0.53 |
| Uc | pg/ml | 9.2E2 | 1.4E3 | 1.7E3 | 3.9E3 | 2.9E3 | 1.0E4 | 1.0E-9 | 5.5E1 | 2.9E4 | 5.7E4 | 219 | 29 | 133 | 29 | 0.59 |
| Ud | pg/ml | 1.0E-9 | 1.0E-9 | 1.9E0 | 1.8E0 | 2.6E1 | 9.9E0 | 1.0E-9 | 1.0E-9 | 3.9E2 | 5.3E1 | 219 | 29 | 133 | 29 | 0.51 |
| Hq | pg/ml | 1.1E0 | 1.3E0 | 1.5E2 | 1.3E1 | 2.0E3 | 4.4E1 | 1.0E-9 | 1.0E-9 | 3.4E4 | 2.3E2 | 581 | 43 | 208 | 43 | 0.51 |
| Hr | pg/ml | 1.3E2 | 1.2E2 | 8.0E2 | 7.4E2 | 1.6E3 | 1.7E3 | 1.0E-9 | 1.0E-9 | 1.4E4 | 8.9E3 | 581 | 43 | 208 | 43 | 0.49 |
| Hu | pg/ml | 1.1E1 | 3.4E1 | 3.2E3 | 1.1E3 | 3.1E4 | 5.0E3 | 1.0E-9 | 1.0E-9 | 6.3E5 | 3.2E4 | 581 | 43 | 208 | 43 | 0.59 |
| Hv | pg/ml | 1.5E0 | 1.5E0 | 3.4E0 | 2.4E1 | 1.2E1 | 1.4E2 | 1.0E-9 | 1.0E-9 | 2.5E2 | 8.9E2 | 581 | 43 | 208 | 43 | 0.50 |
| Hw | pg/ml | 7.0E0 | 5.0E0 | 2.2E1 | 2.4E2 | 8.6E1 | 1.4E3 | 1.0E-9 | 1.0E-9 | 1.7E3 | 9.4E3 | 581 | 43 | 208 | 43 | 0.42 |
| Hx | pg/ml | 9.6E0 | 1.4E1 | 4.7E1 | 7.1E1 | 4.0E2 | 2.3E2 | 1.0E-9 | 1.0E-9 | 9.3E3 | 1.3E3 | 581 | 43 | 208 | 43 | 0.56 |
| Ib | ng/ml | 6.7E-2 | 4.0E-2 | 2.3E0 | 2.2E0 | 7.8E0 | 1.0E1 | 1.0E-9 | 1.0E-9 | 5.3E1 | 5.6E1 | 210 | 29 | 132 | 29 | 0.44 |
| Ic | U/ml | 2.2E2 | 2.2E2 | 8.1E2 | 2.3E2 | 6.5E3 | 1.4E2 | 2.4E0 | 2.1E1 | 9.3E4 | 5.4E2 | 210 | 29 | 132 | 29 | 0.57 |
| Id | U/ml | 6.5E-1 | 9.9E-1 | 1.2E0 | 1.8E1 | 2.0E0 | 8.0E1 | 1.0E-9 | 2.4E-1 | 2.3E1 | 4.3E2 | 210 | 29 | 132 | 29 | 0.65 |
| Tt | pg/ml | 1.7E2 | 1.7E2 | 1.7E2 | 1.8E2 | 5.1E1 | 7.3E1 | 4.3E1 | 1.0E2 | 3.6E2 | 4.4E2 | 202 | 26 | 127 | 26 | 0.52 |
| To | pg/ml | 1.6E0 | 1.8E0 | 1.9E0 | 1.9E0 | 2.0E0 | 2.5E0 | 1.0E-9 | 1.0E-9 | 1.0E1 | 1.2E1 | 211 | 27 | 130 | 27 | 0.49 |
| Tr | pg/ml | 3.0E0 | 5.2E0 | 6.2E0 | 1.1E1 | 2.2E1 | 1.6E1 | 1.0E-9 | 4.7E-2 | 3.1E2 | 7.6E1 | 207 | 26 | 129 | 26 | 0.64 |
| Tn | pg/ml | 2.9E1 | 5.2E1 | 7.5E1 | 1.6E2 | 2.0E2 | 4.4E2 | 2.4E0 | 6.6E0 | 1.8E3 | 2.3E3 | 211 | 27 | 130 | 27 | 0.65 |
| Tv | ng/ml | 1.2E1 | 1.0E1 | 2.0E1 | 3.0E2 | 3.9E1 | 1.4E3 | 1.0E-9 | 1.0E-9 | 4.9E2 | 7.1E3 | 211 | 27 | 130 | 27 | 0.46 |
| Ih | ng/ml | 7.5E1 | 1.9E2 | 2.1E2 | 3.9E2 | 3.6E2 | 5.2E2 | 1.0E-9 | 1.0E-9 | 2.6E3 | 2.8E3 | 584 | 43 | 208 | 43 | 0.63 |
| Ii | ng/ml | 9.8E1 | 1.1E2 | 2.7E2 | 3.9E2 | 7.6E2 | 8.6E2 | 7.3E-1 | 2.3E0 | 1.0E4 | 4.5E3 | 584 | 43 | 208 | 43 | 0.55 |
| Ij | ng/ml | 7.6E1 | 1.3E2 | 1.9E2 | 7.7E2 | 6.3E2 | 3.7E3 | 2.1E0 | 1.1E1 | 6.4E3 | 2.4E4 | 579 | 42 | 207 | 42 | 0.67 |
| Ik | ng/ml | 1.4E1 | 2.5E2 | 1.1E3 | 5.5E2 | 1.0E4 | 6.7E2 | 5.9E-1 | 2.3E0 | 1.2E5 | 2.5E3 | 581 | 43 | 207 | 43 | 0.66 |
| Il | ng/ml | 3.4E2 | 6.0E2 | 1.3E3 | 2.2E3 | 2.7E3 | 3.7E3 | 1.0E-9 | 1.0E-9 | 1.2E4 | 1.3E4 | 574 | 43 | 207 | 43 | 0.57 |
| Im | ng/ml | 2.0E2 | 6.0E2 | 3.5E2 | 9.0E2 | 5.2E2 | 1.3E3 | 1.3E1 | 4.7E1 | 6.0E3 | 6.2E3 | 581 | 43 | 207 | 43 | 0.72 |
| In | ng/ml | 3.9E0 | 3.4E0 | 2.5E1 | 1.1E2 | 1.8E2 | 6.8E2 | 1.0E-9 | 1.0E-9 | 3.9E3 | 4.5E3 | 584 | 43 | 208 | 43 | 0.47 |
| Hb | ng/ml | 2.4E1 | 3.5E1 | 3.2E1 | 5.2E1 | 2.9E1 | 4.9E1 | 4.8E-1 | 6.2E-1 | 1.4E2 | 1.9E2 | 217 | 29 | 134 | 29 | 0.62 |
| Hc | pg/ml | 7.6E2 | 5.9E2 | 4.0E3 | 1.5E3 | 1.4E4 | 2.9E3 | 1.0E-9 | 1.0E-9 | 1.0E5 | 1.4E4 | 217 | 29 | 134 | 29 | 0.42 |
| Hf | ng/ml | 1.5E2 | 2.1E2 | 4.1E2 | 2.9E2 | 5.8E2 | 2.9E2 | 1.0E-9 | 1.0E-9 | 3.2E3 | 9.9E2 | 217 | 29 | 134 | 29 | 0.50 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Io | ng/ml | 8.4E3 | 1.5E4 | 2.7E4 | 1.5E4 | 1.8E5 | 1.3E4 | 1.0E-9 | 1.8E2 | 4.0E6 | 5.4E4 | 578 | 43 | 208 | 43 | 0.55 |
| Ip | ng/ml | 1.0E1 | 3.0E1 | 1.9E1 | 3.0E1 | 2.4E1 | 2.2E1 | 1.0E-9 | 3.7E-2 | 2.6E2 | 8.8E1 | 578 | 43 | 208 | 43 | 0.65 |
| Iq | ug/ml | 9.8E-2 | 1.9E-1 | 2.4E1 | 7.0E0 | 5.7E2 | 3.4E1 | 1.0E-9 | 1.0E-9 | 1.4E4 | 2.2E2 | 578 | 43 | 208 | 43 | 0.57 |
| Ir | ug/ml | 3.4E-1 | 8.3E-1 | 2.7E0 | 1.6E1 | 1.7E1 | 6.1E1 | 1.0E-9 | 1.0E-9 | 2.4E2 | 3.7E2 | 577 | 43 | 208 | 43 | 0.68 |
| Is | ng/ml | 1.5E0 | 6.5E0 | 5.4E0 | 2.1E1 | 1.1E1 | 4.6E1 | 1.0E-9 | 5.3E-2 | 8.8E1 | 2.6E2 | 578 | 43 | 208 | 43 | 0.71 |
| It | ng/ml | 1.9E0 | 4.2E0 | 2.2E1 | 3.3E1 | 1.5E2 | 1.1E2 | 1.0E-9 | 1.0E-9 | 2.8E3 | 6.8E2 | 578 | 43 | 208 | 43 | 0.64 |
| Iu | ng/ml | 2.2E2 | 3.1E2 | 1.3E3 | 2.2E3 | 4.0E3 | 5.3E3 | 1.0E-9 | 1.0E-9 | 2.9E4 | 2.4E4 | 578 | 43 | 208 | 43 | 0.53 |
| Iv | ng/ml | 1.4E1 | 3.2E1 | 4.1E1 | 2.4E2 | 1.0E2 | 7.9E2 | 1.0E-9 | 1.0E-9 | 1.6E3 | 3.8E3 | 577 | 43 | 208 | 43 | 0.67 |
| Iz | ng/ml | 1.5E2 | 1.9E2 | 7.3E2 | 2.6E2 | 4.3E3 | 2.7E2 | 1.5E0 | 4.9E0 | 6.2E4 | 1.0E3 | 217 | 29 | 134 | 29 | 0.50 |
| Yg | pg/ml | 2.8E2 | 3.8E2 | 1.3E3 | 8.4E2 | 5.6E3 | 1.4E3 | 1.0E-9 | 1.1E0 | 5.0E4 | 5.0E3 | 82 | 13 | 62 | 13 | 0.55 |
| Yh | pg/ml | 2.1E2 | 3.6E2 | 4.8E2 | 5.6E2 | 9.4E2 | 6.9E2 | 1.0E-9 | 1.0E-9 | 7.8E3 | 2.3E3 | 82 | 13 | 62 | 13 | 0.52 |
| Yi | pg/ml | 2.4E2 | 4.3E2 | 5.2E2 | 2.8E3 | 9.2E2 | 7.1E3 | 1.0E-9 | 1.0E-9 | 7.6E3 | 2.6E4 | 82 | 13 | 62 | 13 | 0.62 |
| Yk | U/ml | 1.0E-9 | 1.0E-9 | 1.6E-1 | 4.3E-1 | 4.6E-1 | 1.6E0 | 1.0E-9 | 1.0E-9 | 3.0E0 | 5.6E0 | 82 | 13 | 62 | 13 | 0.44 |
| Yj | pg/ml | 1.4E2 | 1.7E2 | 4.3E2 | 3.1E2 | 9.6E2 | 3.0E2 | 1.0E-9 | 1.0E-9 | 7.0E3 | 8.5E2 | 82 | 13 | 62 | 13 | 0.52 |
| Yd | ng/ml | 2.0E-1 | 1.9E-1 | 3.2E-1 | 3.2E-1 | 3.7E-1 | 3.4E-1 | 6.6E-3 | 6.6E-3 | 1.8E0 | 1.1E0 | 85 | 14 | 65 | 14 | 0.49 |
| Wb | pg/ml | 3.0E4 | 3.4E4 | 3.6E4 | 4.6E4 | 2.4E4 | 3.4E4 | 2.2E3 | 1.4E4 | 1.6E5 | 1.5E5 | 84 | 14 | 65 | 14 | 0.60 |
| Vz | pg/ml | 3.0E0 | 2.6E0 | 4.1E0 | 4.9E0 | 4.8E0 | 5.6E0 | 1.0E-9 | 7.6E-2 | 2.9E1 | 2.2E1 | 84 | 14 | 65 | 14 | 0.55 |
| Si | ng/ml | 1.0E0 | 1.4E0 | 2.0E0 | 2.1E0 | 2.8E0 | 1.8E0 | 8.6E-3 | 3.7E-2 | 1.3E1 | 5.6E0 | 82 | 14 | 62 | 14 | 0.60 |
| Sf | mIU/mL | 1.4E1 | 1.4E1 | 4.5E1 | 2.6E1 | 9.3E1 | 3.5E1 | 8.1E-2 | 1.1E0 | 7.2E2 | 1.2E2 | 82 | 14 | 62 | 14 | 0.49 |
| Sh | mIU/mL | 1.4E1 | 8.3E0 | 5.3E1 | 3.7E1 | 1.1E2 | 7.8E1 | 2.9E-2 | 1.3E-1 | 5.9E2 | 2.9E2 | 82 | 14 | 62 | 14 | 0.44 |
| Sj | ng/ml | 4.2E-1 | 4.1E-1 | 4.2E-1 | 4.3E-1 | 9.9E-2 | 9.7E-2 | 1.1E-1 | 3.2E-1 | 6.2E-1 | 7.0E-1 | 82 | 14 | 62 | 14 | 0.50 |
| Rc | pg/ml | 5.7E3 | 7.6E3 | 7.2E3 | 7.2E3 | 5.4E3 | 3.8E3 | 1.9E2 | 2.1E3 | 2.3E4 | 1.7E4 | 216 | 29 | 133 | 29 | 0.56 |
| Rb | pg/ml | 7.6E-1 | 5.6E-1 | 2.7E0 | 3.7E0 | 4.5E0 | 1.0E1 | 1.0E-9 | 1.0E-9 | 4.3E1 | 5.6E1 | 216 | 29 | 133 | 29 | 0.48 |
| Zq | 2.6ng/ml | 2.2E2 | 4.6E2 | 2.8E2 | 5.2E2 | 2.3E2 | 3.3E2 | 8.3E0 | 1.8E1 | 9.7E2 | 9.7E2 | 82 | 14 | 62 | 14 | 0.72 |
| Zw | 2.5ng/ml | 5.6E0 | 4.6E0 | 1.1E1 | 1.6E1 | 1.4E1 | 2.2E1 | 6.3E-2 | 7.3E-2 | 5.9E1 | 6.3E1 | 85 | 14 | 65 | 14 | 0.55 |
| Zx | 2.3mU/ml | 1.2E-1 | 1.0E-1 | 2.6E-1 | 1.6E-1 | 5.0E-1 | 1.6E-1 | 3.2E-2 | 6.1E-2 | 3.0E0 | 6.7E-1 | 85 | 14 | 65 | 14 | 0.48 |
| Pz | ng/ml | 3.7E3 | 1.0E4 | 7.4E3 | 7.4E3 | 1.9E4 | 5.5E3 | 1.3E1 | 4.6E2 | 2.8E5 | 2.8E4 | 577 | 43 | 206 | 43 | 0.61 |
| Qa | ng/ml | 3.2E3 | 9.4E3 | 6.1E3 | 1.6E4 | 7.4E3 | 3.3E4 | 1.5E2 | 6.8E2 | 5.2E4 | 2.2E5 | 577 | 43 | 206 | 43 | 0.72 |
| Qb | ng/ml | 9.9E1 | 2.0E2 | 2.2E2 | 3.6E2 | 5.2E2 | 6.3E2 | 7.9E-1 | 6.2E0 | 8.3E3 | 4.1E3 | 577 | 43 | 206 | 43 | 0.65 |
| Qc | ng/ml | 2.5E2 | 4.8E2 | 4.7E2 | 6.6E2 | 8.0E2 | 8.1E2 | 1.0E-9 | 3.2E0 | 1.1E4 | 4.3E3 | 577 | 43 | 206 | 43 | 0.60 |
| Qd | ng/ml | 1.0E4 | 1.9E4 | 2.3E4 | 3.9E4 | 9.4E4 | 5.2E4 | 2.4E2 | 1.7E3 | 2.0E6 | 2.3E5 | 577 | 43 | 206 | 43 | 0.66 |
| Qe | ng/ml | 8.8E2 | 2.6E3 | 1.9E3 | 3.4E3 | 4.6E3 | 3.5E3 | 7.6E0 | 1.5E2 | 9.7E4 | 1.8E4 | 577 | 43 | 206 | 43 | 0.72 |
| Jd | ng/ml | 9.6E-1 | 2.5E0 | 7.2E0 | 4.1E0 | 4.7E1 | 7.2E0 | 1.0E-9 | 1.0E-9 | 6.5E2 | 3.5E1 | 217 | 29 | 135 | 29 | 0.60 |
| Je | ng/ml | 1.0E-9 | 5.7E-1 | 2.5E0 | 2.1E0 | 8.5E0 | 2.9E0 | 1.0E-9 | 1.0E-9 | 8.8E1 | 1.1E1 | 217 | 29 | 135 | 29 | 0.55 |
| Jf | ng/ml | 1.0E-9 | 1.0E-9 | 1.2E0 | 1.0E0 | 2.4E0 | 1.7E0 | 1.0E-9 | 1.0E-9 | 1.2E1 | 7.7E0 | 217 | 29 | 135 | 29 | 0.52 |
| Jg | ng/ml | 4.7E2 | 1.1E3 | 7.6E2 | 1.5E3 | 9.6E2 | 1.5E3 | 1.0E-9 | 3.7E1 | 1.0E4 | 7.1E3 | 581 | 43 | 208 | 43 | 0.68 |
| Jh | ng/ml | 3.1E0 | 6.6E0 | 2.6E1 | 5.6E1 | 1.1E2 | 1.3E2 | 1.0E-9 | 1.0E-9 | 1.6E3 | 5.4E2 | 581 | 43 | 208 | 43 | 0.59 |
| Ji | ng/ml | 5.0E1 | 1.2E2 | 7.1E1 | 2.0E2 | 7.0E1 | 2.3E2 | 1.0E-9 | 1.3E1 | 5.3E2 | 1.3E3 | 581 | 43 | 208 | 43 | 0.77 |
| Sr | pg/mL | 3.5E2 | 1.2E3 | 8.5E2 | 2.3E3 | 1.3E3 | 3.9E3 | 1.0E-9 | 1.0E-9 | 9.8E3 | 2.1E4 | 207 | 29 | 130 | 29 | 0.71 |
| Ss | pg/mL | 1.2E5 | 1.1E5 | 1.6E5 | 1.7E5 | 2.0E5 | 1.9E5 | 2.7E3 | 1.3E4 | 1.8E6 | 7.7E5 | 207 | 29 | 130 | 29 | 0.50 |
| St | pg/mL | 2.5E7 | 1.0E8 | 5.5E7 | 1.5E8 | 9.9E7 | 3.1E8 | 1.0E-9 | 2.3E6 | 1.2E9 | 1.7E9 | 212 | 29 | 131 | 29 | 0.69 |
| Wc | ng/ml | 1.0E-9 | 1.0E-9 | 5.3E-2 | 1.3E-1 | 1.3E-1 | 4.8E-1 | 1.0E-9 | 1.0E-9 | 9.8E-1 | 1.8E0 | 85 | 14 | 65 | 14 | 0.39 |
| Wd | ng/ml | 9.7E0 | 1.2E1 | 2.9E1 | 1.2E2 | 9.2E1 | 3.2E2 | 1.0E-9 | 3.5E0 | 7.9E2 | 1.2E3 | 85 | 14 | 65 | 14 | 0.60 |
| We | ng/ml | 4.7E-1 | 3.8E-1 | 8.3E-1 | 5.1E0 | 1.1E0 | 9.3E0 | 1.0E-9 | 2.0E-3 | 5.5E0 | 2.3E1 | 85 | 14 | 65 | 14 | 0.52 |
| Wg | ng/ml | 1.0E-9 | 1.0E-9 | 1.0E-9 | 3.8E-2 | 0.0E0 | 1.4E-1 | 1.0E-9 | 1.0E-9 | 1.0E-9 | 5.3E-1 | 85 | 14 | 65 | 14 | 0.54 |
| Wh | ng/ml | 1.1E-2 | 9.3E-3 | 6.7E-2 | 3.7E-1 | 2.8E-1 | 1.2E0 | 1.0E-9 | 3.7E-3 | 2.5E0 | 4.5E0 | 85 | 14 | 65 | 14 | 0.60 |
| Wf | ng/ml | 1.0E-9 | 1.0E-9 | 1.6E-1 | 2.3E-1 | 3.6E-1 | 6.2E-1 | 1.0E-9 | 1.0E-9 | 2.4E0 | 2.3E0 | 85 | 14 | 65 | 14 | 0.47 |
| Ra | pg/ml | 1.0E-9 | 1.0E-9 | 6.4E-1 | 2.8E0 | 1.3E0 | 1.2E1 | 1.0E-9 | 1.0E-9 | 7.3E0 | 6.4E1 | 216 | 29 | 133 | 29 | 0.51 |
| Qz | pg/ml | 1.0E1 | 1.1E1 | 6.0E1 | 5.3E1 | 1.0E2 | 7.8E1 | 1.0E-9 | 1.0E-9 | 7.1E2 | 2.3E2 | 216 | 29 | 133 | 29 | 0.53 |
| Qy | pg/ml | 4.6E-1 | 4.8E-1 | 1.5E1 | 4.4E0 | 7.2E1 | 1.8E1 | 1.0E-9 | 1.0E-9 | 6.5E2 | 9.7E1 | 216 | 29 | 133 | 29 | 0.49 |
| Qx | pg/ml | 1.0E-9 | 1.0E-9 | 7.6E0 | 6.0E0 | 5.5E1 | 2.1E1 | 1.0E-9 | 1.0E-9 | 5.8E2 | 1.1E2 | 216 | 29 | 133 | 29 | 0.50 |
| Qw | pg/ml | 4.5E-2 | 1.0E-9 | 2.2E0 | 7.2E-1 | 8.8E0 | 1.3E0 | 1.0E-9 | 1.0E-9 | 8.6E1 | 5.6E0 | 216 | 29 | 133 | 29 | 0.46 |
| Qv | pg/ml | 2.2E4 | 1.7E4 | 3.5E4 | 1.9E4 | 6.4E4 | 1.4E4 | 1.0E-9 | 4.0E2 | 7.4E5 | 5.0E4 | 216 | 29 | 133 | 29 | 0.39 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qu | pg/ml | 1.2E1 | 1.2E1 | 8.9E1 | 6.8E1 | 1.7E2 | 1.4E2 | 1.0E-9 | 1.0E-9 | 9.8E2 | 7.0E2 | 216 | 29 | 133 | 29 | 0.48 |
| Qt | pg/ml | 1.2E1 | 2.1E1 | 5.7E1 | 4.0E1 | 1.4E2 | 6.4E1 | 1.0E-9 | 1.0E-9 | 1.0E3 | 2.7E2 | 216 | 29 | 133 | 29 | 0.54 |
| Qh | ng/ml | 1.7E1 | 3.6E1 | 3.7E1 | 5.8E1 | 6.3E1 | 8.7E1 | 1.0E-9 | 3.8E0 | 6.4E2 | 4.6E2 | 216 | 29 | 133 | 29 | 0.63 |
| Qg | ng/ml | 7.9E0 | 4.9E0 | 2.0E1 | 1.0E1 | 7.5E1 | 1.6E1 | 5.1E-2 | 3.3E-1 | 1.0E3 | 8.1E1 | 216 | 29 | 133 | 29 | 0.40 |
| Jj | ng/ml | 7.5E2 | 2.9E2 | 2.2E3 | 5.4E2 | 1.5E4 | 6.3E2 | 1.7E1 | 1.2E1 | 3.4E5 | 3.3E3 | 581 | 43 | 208 | 43 | 0.32 |
| Jk | ng/ml | 3.3E0 | 3.2E0 | 2.4E1 | 2.6E1 | 4.9E1 | 5.2E1 | 1.0E-9 | 2.2E-1 | 3.9E2 | 2.4E2 | 581 | 43 | 208 | 43 | 0.53 |
| Jl | ng/ml | 4.4E-1 | 1.6E0 | 1.8E0 | 2.4E2 | 4.3E0 | 1.5E3 | 7.6E-4 | 1.5E-2 | 3.2E1 | 9.9E3 | 581 | 43 | 208 | 43 | 0.66 |
| Jm | ng/ml | 1.9E1 | 3.7E1 | 5.2E1 | 1.0E2 | 9.7E1 | 3.2E2 | 1.0E-9 | 4.1E-1 | 1.0E3 | 2.1E3 | 581 | 43 | 208 | 43 | 0.57 |
| Jn | pg/ml | 4.0E-1 | 8.4E-1 | 1.7E0 | 3.3E1 | 5.8E0 | 1.4E2 | 1.0E-9 | 1.0E-9 | 6.2E1 | 7.3E2 | 581 | 43 | 208 | 43 | 0.65 |
| Jo | pg/ml | 4.0E3 | 3.7E3 | 5.1E3 | 7.7E3 | 3.9E3 | 1.6E4 | 4.2E1 | 2.3E2 | 2.4E4 | 1.0E5 | 581 | 43 | 208 | 43 | 0.49 |
| Jp | pg/ml | 7.0E4 | 8.9E4 | 7.3E4 | 9.4E4 | 3.4E4 | 4.1E4 | 2.1E3 | 2.6E4 | 2.1E5 | 2.1E5 | 581 | 43 | 208 | 43 | 0.66 |
| Jq | pg/ml | 9.5E1 | 1.6E2 | 1.5E2 | 5.3E2 | 2.2E2 | 1.4E3 | 2.6E0 | 1.4E0 | 4.0E3 | 8.7E3 | 581 | 43 | 208 | 43 | 0.64 |
| Jr | pg/ml | 5.2E0 | 1.0E1 | 2.2E1 | 3.1E2 | 1.0E2 | 1.4E3 | 1.0E-9 | 1.0E-9 | 1.9E3 | 7.4E3 | 581 | 43 | 208 | 43 | 0.64 |
| Js | pg/ml | 1.3E1 | 1.8E1 | 4.2E1 | 1.7E2 | 1.5E2 | 6.3E2 | 1.0E-9 | 1.7E0 | 2.0E3 | 3.0E3 | 581 | 43 | 208 | 43 | 0.62 |
| Jt | pg/ml | 2.7E3 | 3.4E3 | 3.2E3 | 5.1E3 | 2.2E3 | 8.3E3 | 1.5E2 | 4.1E2 | 1.3E4 | 5.2E4 | 581 | 43 | 208 | 43 | 0.57 |
| Xa | pg/ml | 6.1E-1 | 7.9E0 | 1.0E1 | 1.1E2 | 2.0E1 | 3.2E2 | 1.0E-9 | 1.0E-9 | 9.9E1 | 1.2E3 | 84 | 14 | 65 | 14 | 0.67 |
| Ye | pg/ml | 1.0E-9 | 1.0E-9 | 9.2E0 | 1.3E0 | 4.0E1 | 4.8E0 | 1.0E-9 | 1.0E-9 | 3.5E2 | 1.8E1 | 84 | 14 | 65 | 14 | 0.37 |
| Tm | pg/ml | 1.0E-9 | 1.0E-9 | 4.7E0 | 1.2E0 | 1.0E1 | 3.0E0 | 1.0E-9 | 1.0E-9 | 6.8E1 | 8.4E0 | 84 | 14 | 65 | 14 | 0.39 |
| Tl | pg/ml | 1.3E-1 | 1.0E-9 | 3.3E-1 | 1.9E0 | 3.8E-1 | 6.6E0 | 1.0E-9 | 1.0E-9 | 1.8E0 | 2.5E1 | 84 | 14 | 65 | 14 | 0.37 |
| Ju | mIU/ml | 9.0E0 | 1.1E1 | 2.1E1 | 1.7E1 | 3.3E1 | 2.0E1 | 6.5E-2 | 1.9E-1 | 2.3E2 | 8.9E1 | 217 | 29 | 135 | 29 | 0.56 |
| Jv | mIU/ml | 1.2E1 | 1.0E1 | 3.7E1 | 2.9E1 | 6.6E1 | 4.1E1 | 1.0E-2 | 8.2E-2 | 4.4E2 | 1.9E2 | 217 | 29 | 135 | 29 | 0.52 |
| Jy | ng/ml | 1.6E-3 | 2.0E-3 | 2.3E-3 | 3.7E-3 | 4.6E-3 | 7.4E-3 | 1.7E-4 | 5.3E-4 | 5.2E-2 | 4.1E-2 | 217 | 29 | 135 | 29 | 0.61 |
| Kc | pg/ml | 2.3E1 | 3.4E1 | 4.0E1 | 5.2E1 | 4.3E1 | 6.3E1 | 1.0E-9 | 1.0E-9 | 1.9E2 | 2.7E2 | 218 | 29 | 134 | 29 | 0.53 |
| Kd | pg/ml | 1.0E-9 | 1.0E-9 | 2.0E2 | 1.9E3 | 6.0E2 | 7.1E3 | 1.0E-9 | 1.0E-9 | 4.8E3 | 3.8E4 | 218 | 29 | 134 | 29 | 0.58 |
| Ke | pg/ml | 1.3E4 | 2.0E4 | 1.4E4 | 3.7E4 | 1.1E4 | 6.0E4 | 3.4E2 | 1.8E3 | 7.0E4 | 3.2E5 | 218 | 29 | 134 | 29 | 0.69 |
| Kf | pg/mL | 6.7E0 | 7.3E0 | 7.3E0 | 1.1E1 | 5.9E0 | 1.5E1 | 1.0E-9 | 1.0E-9 | 2.7E1 | 7.8E1 | 218 | 29 | 134 | 29 | 0.57 |
| Kg | pg/mL | 1.2E3 | 8.2E2 | 2.0E3 | 2.3E3 | 2.4E3 | 5.1E3 | 7.3E1 | 1.3E2 | 1.7E4 | 2.7E4 | 218 | 29 | 134 | 29 | 0.43 |
| Ki | pg/ml | 5.9E1 | 6.4E1 | 7.0E1 | 7.8E1 | 5.5E1 | 6.1E1 | 1.0E-9 | 1.0E-9 | 3.8E2 | 2.5E2 | 217 | 29 | 134 | 29 | 0.55 |
| Kj | pg/ml | 1.1E3 | 7.7E2 | 1.7E3 | 1.7E3 | 1.7E3 | 2.9E3 | 1.4E1 | 3.3E1 | 1.0E4 | 1.5E4 | 218 | 29 | 134 | 29 | 0.40 |
| Kk | pg/ml | 6.8E0 | 1.0E1 | 1.1E1 | 1.8E1 | 1.6E1 | 1.9E1 | 1.0E-9 | 2.0E0 | 1.6E2 | 5.9E1 | 218 | 29 | 134 | 29 | 0.63 |
| Kl | pg/ml | 2.1E4 | 2.3E4 | 2.9E4 | 3.3E4 | 2.6E4 | 2.9E4 | 1.6E2 | 1.3E3 | 1.6E5 | 1.1E5 | 218 | 29 | 134 | 29 | 0.54 |
| Kn | pg/ml | 3.0E1 | 5.7E1 | 6.2E1 | 2.7E2 | 9.5E1 | 9.0E2 | 1.0E-9 | 1.0E-9 | 7.3E2 | 4.9E3 | 218 | 29 | 134 | 29 | 0.60 |
| Ko | pg/ml | 3.4E2 | 6.2E2 | 4.6E2 | 8.7E2 | 4.5E2 | 9.8E2 | 1.0E-9 | 1.0E-9 | 2.2E3 | 4.1E3 | 218 | 29 | 134 | 29 | 0.65 |
| Kp | pg/ml | 3.4E2 | 3.6E2 | 3.5E2 | 8.3E2 | 2.7E2 | 2.4E3 | 1.0E-9 | 3.7E1 | 1.7E3 | 1.3E4 | 218 | 29 | 134 | 29 | 0.55 |
| Kq | pg/ml | 3.2E2 | 5.7E2 | 5.1E2 | 6.4E3 | 9.1E2 | 2.9E4 | 1.6E0 | 4.8E1 | 9.8E3 | 1.6E5 | 209 | 29 | 128 | 29 | 0.69 |
| Kr | pg/ml | 5.6E-1 | 1.0E-9 | 2.7E0 | 1.7E1 | 5.2E0 | 7.7E1 | 1.0E-9 | 1.0E-9 | 3.9E1 | 4.2E2 | 209 | 29 | 128 | 29 | 0.47 |
| Ks | pg/ml | 1.4E4 | 1.7E4 | 2.1E4 | 2.0E4 | 1.9E4 | 1.7E4 | 5.1E1 | 9.9E2 | 1.1E5 | 5.1E4 | 209 | 29 | 128 | 29 | 0.50 |
| Ps | ng/ml | 1.7E2 | 4.2E2 | 4.3E2 | 7.5E2 | 1.0E3 | 9.6E2 | 4.1E-1 | 1.3E1 | 8.3E3 | 3.8E3 | 82 | 14 | 62 | 14 | 0.70 |
| Kx | ng/ml | 1.0E-9 | 8.3E-3 | 7.1E-3 | 1.5E-2 | 1.4E-2 | 2.0E-2 | 1.0E-9 | 1.0E-9 | 1.0E-1 | 6.5E-2 | 214 | 29 | 133 | 29 | 0.63 |
| Ky | ng/ml | 1.0E-1 | 2.2E-1 | 3.8E-1 | 7.5E-1 | 8.3E-1 | 1.1E0 | 1.0E-9 | 1.0E-9 | 5.4E0 | 5.0E0 | 214 | 29 | 133 | 29 | 0.64 |
| Kz | ng/ml | 1.0E-9 | 1.0E-9 | 3.4E-3 | 5.6E-3 | 5.7E-3 | 6.9E-3 | 1.0E-9 | 1.0E-9 | 3.6E-2 | 1.8E-2 | 214 | 29 | 133 | 29 | 0.58 |
| Rz | ng/ml | 3.3E-1 | 3.1E-1 | 8.0E-1 | 1.5E0 | 1.3E0 | 2.0E0 | 3.6E-3 | 1.7E-2 | 6.5E0 | 5.9E0 | 82 | 14 | 62 | 14 | 0.56 |
| Ry | ng/ml | 1.6E-2 | 2.0E-2 | 2.1E-2 | 4.7E-2 | 2.3E-2 | 8.9E-2 | 1.0E-9 | 1.0E-9 | 1.2E-1 | 3.5E-1 | 82 | 14 | 62 | 14 | 0.59 |
| Rx | ng/ml | 1.0E-9 | 3.5E-5 | 1.9E-3 | 1.3E-3 | 3.5E-3 | 1.9E-3 | 1.0E-9 | 1.0E-9 | 2.0E-2 | 4.7E-3 | 82 | 14 | 62 | 14 | 0.53 |
| Ld | pg/ml | 1.0E-9 | 7.5E-1 | 3.7E0 | 5.7E0 | 9.6E0 | 1.0E1 | 1.0E-9 | 1.0E-9 | 8.4E1 | 5.0E1 | 216 | 29 | 133 | 29 | 0.58 |
| Lh | pg/ml | 1.3E4 | 2.2E4 | 2.1E4 | 5.4E4 | 2.7E4 | 8.4E4 | 1.0E-9 | 6.7E2 | 2.6E5 | 4.1E5 | 580 | 43 | 209 | 43 | 0.68 |
| Li | pg/ml | 3.1E3 | 1.2E4 | 1.5E4 | 5.7E4 | 3.9E4 | 1.5E5 | 1.0E-9 | 3.6E1 | 3.6E5 | 9.2E5 | 580 | 43 | 209 | 43 | 0.72 |
| Lj | pg/ml | 2.4E3 | 9.5E3 | 2.1E4 | 4.8E4 | 6.5E4 | 8.9E4 | 1.0E-9 | 1.0E2 | 4.7E5 | 4.1E5 | 580 | 43 | 209 | 43 | 0.71 |
| Lp | pg/ml | 1.1E1 | 6.2E0 | 6.4E1 | 1.6E2 | 1.4E2 | 3.2E2 | 1.0E-9 | 1.0E-9 | 7.7E2 | 1.0E3 | 82 | 14 | 62 | 14 | 0.46 |
| Lt | pg/ml | 1.0E-9 | 1.0E-9 | 2.6E0 | 1.8E0 | 9.7E0 | 6.7E0 | 1.0E-9 | 1.0E-9 | 6.0E1 | 2.5E1 | 82 | 14 | 62 | 14 | 0.49 |
| Rv | ng/ml | 5.0E-4 | 6.9E-4 | 1.1E-3 | 2.4E-3 | 2.1E-3 | 3.8E-3 | 1.0E-9 | 1.0E-9 | 1.6E-2 | 1.2E-2 | 82 | 14 | 62 | 14 | 0.57 |
| Ru | ng/ml | 1.0E-9 | 1.0E-9 | 1.8E-2 | 5.4E-2 | 7.1E-2 | 1.2E-1 | 1.0E-9 | 1.0E-9 | 3.8E-1 | 3.5E-1 | 82 | 14 | 62 | 14 | 0.57 |
| Rt | ng/ml | 6.9E-2 | 3.4E-2 | 1.1E-1 | 5.9E-1 | 1.3E-1 | 2.0E0 | 1.0E-3 | 1.3E-3 | 5.8E-1 | 7.4E0 | 82 | 14 | 62 | 14 | 0.39 |

74

| Code | Units | Median | | Average | | Standard Deviation | | Minimum | | Maximum | | Number of Samples | | Number of Patients | | AUC |
|------|-------|--------|-----|---------|-----|--------|-----|--------|-----|--------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Yl | pg/ml | 1.1E1 | 1.2E1 | 1.6E1 | 3.4E1 | 1.8E1 | 5.6E1 | 1.0E-9 | 1.0E-9 | 1.1E2 | 2.2E2 | 85 | 14 | 65 | 14 | 0.58 |
| Rm | ng/ml | 1.9E1 | 4.0E1 | 5.1E1 | 8.8E1 | 7.7E1 | 1.3E2 | 2.2E-1 | 3.9E-1 | 4.0E2 | 6.5E2 | 213 | 29 | 132 | 29 | 0.59 |
| Rh | ng/ml | 1.3E2 | 1.7E2 | 2.8E2 | 1.2E3 | 5.0E2 | 3.7E3 | 4.7E0 | 2.5E1 | 3.8E3 | 1.7E4 | 213 | 29 | 132 | 29 | 0.59 |
| Ri | ng/ml | 1.0E-9 | 1.0E-9 | 4.3E0 | 1.4E0 | 1.7E1 | 3.3E0 | 1.0E-9 | 1.0E-9 | 2.2E2 | 1.6E1 | 214 | 29 | 133 | 29 | 0.42 |
| Rg | ng/ml | 1.0E-9 | 1.0E-9 | 6.8E-2 | 5.1E-2 | 3.9E-1 | 1.6E-1 | 1.0E-9 | 1.0E-9 | 3.3E0 | 6.2E-1 | 213 | 29 | 132 | 29 | 0.49 |
| Rj | ng/ml | 1.0E-9 | 1.0E-9 | 1.6E0 | 9.8E0 | 6.3E0 | 5.0E1 | 1.0E-9 | 1.0E-9 | 8.0E1 | 2.7E2 | 214 | 29 | 133 | 29 | 0.49 |
| Rf | ng/ml | 3.7E-1 | 9.7E-1 | 1.0E0 | 2.2E0 | 1.9E0 | 3.8E0 | 7.8E-3 | 1.4E-2 | 1.5E1 | 1.7E1 | 213 | 29 | 132 | 29 | 0.67 |
| Ql | pg/ml | 5.5E0 | 1.1E1 | 1.5E1 | 1.9E1 | 3.2E1 | 2.6E1 | 1.0E-9 | 1.0E-9 | 2.9E2 | 9.3E1 | 217 | 29 | 135 | 29 | 0.59 |
| Qm | pg/ml | 4.4E0 | 1.6E1 | 2.0E1 | 3.0E1 | 4.0E1 | 3.9E1 | 1.0E-9 | 1.0E-9 | 3.6E2 | 1.7E2 | 217 | 29 | 135 | 29 | 0.63 |
| Qn | pg/ml | 6.1E-1 | 6.1E-1 | 5.8E0 | 1.4E1 | 1.9E1 | 4.7E1 | 1.0E-9 | 1.0E-9 | 2.2E2 | 2.4E2 | 217 | 29 | 135 | 29 | 0.53 |
| Nv | pg/ml | 4.0E3 | 7.0E3 | 1.2E4 | 1.8E4 | 5.2E4 | 2.6E4 | 1.0E-9 | 1.5E2 | 1.1E6 | 1.1E5 | 585 | 43 | 209 | 43 | 0.64 |
| Nw | pg/ml | 8.5E3 | 1.7E4 | 1.3E4 | 3.0E4 | 1.8E4 | 4.4E4 | 8.6E1 | 1.7E3 | 2.1E5 | 2.2E5 | 585 | 43 | 209 | 43 | 0.73 |
| Nx | pg/ml | 2.2E2 | 2.6E2 | 4.1E2 | 6.7E2 | 6.9E2 | 8.6E2 | 1.0E-9 | 1.0E-9 | 7.4E3 | 4.1E3 | 585 | 43 | 209 | 43 | 0.61 |
| Ny | pg/ml | 5.7E0 | 1.6E1 | 7.2E1 | 1.3E2 | 1.0E3 | 4.3E2 | 1.0E-9 | 1.0E-9 | 2.5E4 | 2.8E3 | 585 | 43 | 209 | 43 | 0.67 |
| Oa | pg/ml | 1.6E2 | 4.1E2 | 4.1E2 | 9.5E2 | 7.0E2 | 1.1E3 | 1.0E-9 | 4.9E0 | 4.8E3 | 4.5E3 | 217 | 29 | 135 | 29 | 0.69 |
| Op | pg/ml | 4.1E5 | 4.1E5 | 4.0E5 | 3.8E5 | 1.5E5 | 1.8E5 | 3.3E4 | 6.7E4 | 7.3E5 | 6.6E5 | 82 | 14 | 62 | 14 | 0.48 |
| Wn | ng/ml | 1.4E1 | 2.7E1 | 6.5E1 | 9.3E1 | 2.3E2 | 1.3E2 | 1.2E0 | 2.7E0 | 1.8E3 | 3.4E2 | 71 | 8 | 53 | 8 | 0.67 |
| Tk | ng/ml | 1.9E2 | 1.5E2 | 3.9E2 | 4.1E2 | 6.5E2 | 5.4E2 | 3.0E0 | 1.0E1 | 4.2E3 | 1.4E3 | 79 | 9 | 58 | 9 | 0.48 |
| Oe | pg/ml | 9.1E1 | 9.4E0 | 3.1E2 | 2.7E2 | 8.7E2 | 4.7E2 | 1.0E-9 | 1.0E-9 | 1.9E4 | 2.2E3 | 580 | 43 | 209 | 43 | 0.45 |
| Of | pg/ml | 2.3E2 | 1.0E2 | 7.1E3 | 6.3E3 | 3.3E4 | 2.1E4 | 1.0E-9 | 1.0E-9 | 6.2E5 | 1.1E5 | 585 | 43 | 209 | 43 | 0.44 |
| Og | pg/ml | 9.2E-2 | 5.9E-2 | 9.9E-1 | 2.4E-1 | 5.8E0 | 7.7E-1 | 1.0E-9 | 1.0E-9 | 8.3E1 | 5.0E0 | 585 | 43 | 209 | 43 | 0.42 |
| Oh | pg/ml | 2.4E0 | 5.5E0 | 2.5E1 | 4.6E1 | 1.7E2 | 1.8E2 | 1.0E-9 | 1.0E-9 | 3.5E3 | 1.1E3 | 585 | 43 | 209 | 43 | 0.65 |
| Oi | pg/ml | 2.8E0 | 3.0E0 | 6.5E0 | 7.3E0 | 1.0E1 | 1.0E1 | 1.0E-9 | 1.0E-9 | 8.6E1 | 5.1E1 | 585 | 43 | 209 | 43 | 0.52 |
| Ok | pg/ml | 3.9E2 | 6.5E2 | 5.2E2 | 1.2E3 | 5.0E2 | 1.4E3 | 1.3E1 | 3.2E1 | 5.2E3 | 7.8E3 | 585 | 43 | 209 | 43 | 0.72 |
| Om | pg/ml | 3.8E2 | 5.7E2 | 9.0E2 | 2.5E3 | 2.5E3 | 7.8E3 | 1.0E-9 | 1.0E2 | 3.6E4 | 5.1E4 | 585 | 43 | 209 | 43 | 0.63 |
| On | pg/ml | 1.8E2 | 2.9E2 | 2.9E2 | 7.5E2 | 4.3E2 | 1.4E3 | 8.4E-1 | 1.5E1 | 4.5E3 | 8.5E3 | 585 | 43 | 209 | 43 | 0.67 |
| Or | pg/ml | 1.4E1 | 1.9E1 | 3.4E1 | 7.9E1 | 6.3E1 | 1.4E2 | 1.0E-9 | 1.0E-9 | 5.0E2 | 5.1E2 | 218 | 29 | 134 | 29 | 0.55 |
| Ow | pg/ml | 3.3E1 | 6.1E1 | 1.1E2 | 2.7E2 | 3.1E2 | 5.8E2 | 1.0E-9 | 1.0E-9 | 2.7E3 | 3.0E3 | 218 | 29 | 134 | 29 | 0.62 |
| Ou | pg/ml | 4.7E2 | 6.8E2 | 8.8E2 | 2.0E3 | 1.3E3 | 3.0E3 | 1.0E-9 | 1.0E-9 | 9.8E3 | 9.6E3 | 218 | 29 | 134 | 29 | 0.57 |
| Ul | ng/ml | 1.0E-9 | 1.0E-9 | 1.4E0 | 5.4E0 | 4.7E0 | 1.5E1 | 1.0E-9 | 1.0E-9 | 3.4E1 | 5.6E1 | 224 | 29 | 137 | 29 | 0.52 |
| Uo | ng/ml | 1.0E-9 | 1.0E-9 | 1.1E-1 | 5.4E-2 | 2.7E-1 | 1.5E-1 | 1.0E-9 | 1.0E-9 | 2.6E0 | 6.3E-1 | 224 | 29 | 137 | 29 | 0.46 |
| Uk | ng/ml | 1.0E-9 | 1.0E-9 | 8.7E-3 | 3.9E-3 | 2.9E-2 | 1.0E-2 | 1.0E-9 | 1.0E-9 | 3.2E-1 | 3.9E-2 | 224 | 29 | 137 | 29 | 0.38 |
| Um | ng/ml | 1.0E-9 | 1.0E-9 | 4.2E-1 | 2.2E-1 | 1.0E0 | 4.9E-1 | 1.0E-9 | 1.0E-9 | 7.2E0 | 2.3E0 | 224 | 29 | 137 | 29 | 0.45 |
| Uf | ng/ml | 5.3E-2 | 9.8E-2 | 1.5E-1 | 3.6E-1 | 2.7E-1 | 1.0E0 | 1.0E-3 | 9.8E-3 | 2.1E0 | 5.6E0 | 224 | 29 | 137 | 29 | 0.66 |
| Uh | ng/ml | 1.9E0 | 4.5E0 | 2.9E0 | 6.1E0 | 3.1E0 | 5.1E0 | 3.2E-2 | 9.5E-2 | 1.7E1 | 1.8E1 | 224 | 29 | 137 | 29 | 0.72 |
| Un | ng/ml | 1.9E0 | 2.5E0 | 2.1E0 | 3.7E0 | 1.3E0 | 4.4E0 | 2.0E-1 | 5.3E-1 | 8.0E0 | 2.5E1 | 224 | 29 | 137 | 29 | 0.67 |
| Ug | ng/ml | 1.4E1 | 1.1E1 | 2.8E1 | 2.5E1 | 2.9E1 | 3.5E1 | 6.9E-1 | 1.7E0 | 1.8E2 | 1.6E2 | 224 | 29 | 137 | 29 | 0.42 |
| Ur | ng/ml | 1.5E-1 | 8.5E-3 | 8.5E-1 | 7.9E-1 | 6.3E0 | 1.9E0 | 1.0E-9 | 1.0E-9 | 9.3E1 | 7.3E0 | 223 | 29 | 136 | 29 | 0.39 |
| Up | ng/ml | 1.0E-9 | 1.0E-9 | 6.0E-3 | 8.5E-2 | 2.6E-2 | 4.4E-1 | 1.0E-9 | 1.0E-9 | 3.3E-1 | 2.4E0 | 223 | 29 | 136 | 29 | 0.50 |
| Us | ng/ml | 4.3E-3 | 1.0E-4 | 1.9E-2 | 8.7E-2 | 4.6E-2 | 3.1E-1 | 1.0E-9 | 1.0E-9 | 4.0E-1 | 1.7E0 | 223 | 29 | 136 | 29 | 0.50 |
| Uv | ng/ml | 3.2E-3 | 2.6E-3 | 1.3E-2 | 2.6E-2 | 3.9E-2 | 8.4E-2 | 1.0E-9 | 1.0E-9 | 4.3E-1 | 4.1E-1 | 223 | 29 | 136 | 29 | 0.46 |
| Ut | ng/ml | 7.6E-1 | 1.2E0 | 3.2E0 | 5.0E0 | 9.7E0 | 1.3E1 | 1.0E-9 | 1.0E-9 | 7.8E1 | 6.5E1 | 223 | 29 | 136 | 29 | 0.59 |
| Uu | ng/ml | 7.2E0 | 5.7E0 | 7.8E0 | 9.0E0 | 4.8E0 | 9.4E0 | 5.7E-1 | 1.2E0 | 2.6E1 | 4.0E1 | 223 | 29 | 136 | 29 | 0.47 |
| Uw | ng/ml | 2.2E0 | 3.1E0 | 2.9E0 | 5.8E0 | 4.2E0 | 9.8E0 | 1.0E-9 | 9.9E-1 | 3.7E1 | 3.9E1 | 90 | 14 | 70 | 14 | 0.65 |
| Vb | ng/ml | 1.0E0 | 9.2E-1 | 1.0E0 | 1.3E0 | 4.3E-1 | 1.6E0 | 8.5E-2 | 2.6E-1 | 2.5E0 | 6.4E0 | 90 | 14 | 70 | 14 | 0.46 |
| Vc | ng/ml | 1.0E-9 | 1.0E-9 | 1.6E-3 | 1.0E-9 | 1.2E-2 | 0.0E0 | 1.0E-9 | 1.0E-9 | 1.1E-1 | 1.0E-9 | 90 | 14 | 70 | 14 | 0.48 |
| Uy | ng/ml | 1.0E0 | 1.4E0 | 4.1E0 | 1.2E1 | 1.2E1 | 2.2E1 | 3.1E-2 | 2.0E-2 | 9.9E1 | 6.4E1 | 90 | 14 | 70 | 14 | 0.58 |
| Uz | ng/ml | 1.0E-9 | 1.0E-9 | 4.9E-3 | 2.4E0 | 4.6E-2 | 8.8E0 | 1.0E-9 | 1.0E-9 | 4.4E-1 | 3.3E1 | 90 | 14 | 70 | 14 | 0.53 |
| Ux | ng/ml | 1.7E2 | 2.0E2 | 1.9E2 | 2.2E2 | 1.3E2 | 1.3E2 | 4.5E0 | 3.5E1 | 5.3E2 | 4.6E2 | 90 | 14 | 70 | 14 | 0.58 |
| Va | ng/ml | 1.8E1 | 5.6E0 | 2.6E1 | 1.9E1 | 2.8E1 | 2.6E1 | 3.1E-1 | 1.2E0 | 1.2E2 | 7.8E1 | 90 | 14 | 70 | 14 | 0.39 |
| Vh | ng/ml | 1.0E-2 | 1.4E-2 | 1.6E-2 | 7.7E-2 | 2.1E-2 | 2.3E-1 | 1.0E-9 | 2.2E-3 | 1.2E-1 | 8.6E-1 | 90 | 14 | 70 | 14 | 0.62 |
| Vi | ng/ml | 3.0E-3 | 9.8E-3 | 1.6E-1 | 1.4E-1 | 1.4E0 | 4.8E-1 | 1.0E-9 | 1.0E-9 | 1.4E1 | 1.8E0 | 90 | 14 | 70 | 14 | 0.68 |
| Vj | ng/ml | 2.7E1 | 6.7E1 | 2.3E2 | 7.6E1 | 1.0E3 | 6.2E1 | 1.4E0 | 5.4E0 | 8.4E3 | 1.7E2 | 90 | 12 | 70 | 12 | 0.59 |

| Code | Units | Median | | Average | | Standard Deviation | | Minimum | | Maximum | | Number of Samples | | Number of Patients | | AUC |
|------|-------|--------|-----|---------|-----|-----|-----|--------|-----|--------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Vp | ng/ml | 1.0E-9 | 1.0E-9 | 4.6E-1 | 1.7E0 | 3.8E0 | 9.1E0 | 1.0E-9 | 1.0E-9 | 5.0E1 | 4.9E1 | 224 | 29 | 137 | 29 | 0.51 |
| Vt | ng/ml | 6.1E0 | 1.2E1 | 8.3E0 | 2.0E1 | 9.3E0 | 2.9E1 | 4.3E-1 | 1.4E0 | 8.6E1 | 1.6E2 | 224 | 29 | 137 | 29 | 0.71 |
| Vu | ng/ml | 1.0E-9 | 1.0E-9 | 2.9E0 | 2.0E0 | 7.5E0 | 4.4E0 | 1.0E-9 | 1.0E-9 | 7.1E1 | 2.2E1 | 219 | 27 | 137 | 27 | 0.51 |
| Vq | ng/ml | 1.5E2 | 6.4E2 | 6.3E2 | 1.5E3 | 1.4E3 | 3.0E3 | 2.0E-1 | 1.0E1 | 1.1E4 | 1.2E4 | 175 | 18 | 115 | 18 | 0.65 |
| Vo | ng/ml | 2.6E1 | 2.5E1 | 2.5E1 | 2.5E1 | 5.1E0 | 4.6E0 | 2.5E0 | 1.1E1 | 4.8E1 | 3.3E1 | 224 | 29 | 137 | 29 | 0.48 |
| Vs | ng/ml | 2.6E-1 | 1.0E-9 | 7.3E0 | 2.3E1 | 2.6E1 | 9.3E1 | 1.0E-9 | 1.0E-9 | 2.3E2 | 4.5E2 | 218 | 24 | 135 | 24 | 0.45 |
| Vv | ng/ml | 3.3E0 | 2.5E0 | 6.3E0 | 4.5E0 | 1.0E1 | 6.4E0 | 1.0E-9 | 1.0E-9 | 8.4E1 | 3.2E1 | 223 | 28 | 137 | 28 | 0.46 |
| Vw | ng/ml | 3.6E1 | 4.0E1 | 3.4E1 | 4.0E1 | 1.7E1 | 1.6E1 | 2.5E0 | 1.1E1 | 7.0E1 | 6.6E1 | 90 | 14 | 70 | 14 | 0.59 |
| Oy | pg/ml | 5.5E-1 | 4.8E-1 | 7.3E0 | 3.1E0 | 3.5E1 | 9.1E0 | 1.0E-9 | 1.0E-9 | 4.0E2 | 4.9E1 | 584 | 43 | 208 | 43 | 0.45 |
| Oz | pg/ml | 1.3E-2 | 1.4E-2 | 3.5E-1 | 9.4E-1 | 1.6E0 | 4.3E0 | 1.0E-9 | 1.0E-9 | 2.9E1 | 2.8E1 | 584 | 43 | 208 | 43 | 0.51 |
| Pa | pg/ml | 3.9E-1 | 5.7E-1 | 1.5E0 | 6.6E0 | 5.8E0 | 3.4E1 | 1.0E-9 | 1.0E-9 | 8.6E1 | 2.3E2 | 584 | 43 | 208 | 43 | 0.57 |
| Pb | pg/ml | 1.0E-9 | 1.0E-9 | 1.1E0 | 8.5E-1 | 2.0E1 | 4.8E0 | 1.0E-9 | 1.0E-9 | 4.9E2 | 3.2E1 | 584 | 43 | 208 | 43 | 0.45 |
| Pc | pg/ml | 4.9E-2 | 1.6E-1 | 3.8E-1 | 1.3E0 | 1.0E0 | 5.7E0 | 1.0E-9 | 1.0E-9 | 1.6E1 | 3.8E1 | 584 | 43 | 208 | 43 | 0.54 |
| Pd | pg/ml | 1.8E0 | 4.1E0 | 5.5E0 | 9.6E0 | 3.6E1 | 2.0E1 | 1.0E-9 | 1.0E-9 | 8.4E2 | 1.2E2 | 584 | 43 | 208 | 43 | 0.63 |
| Pe | pg/ml | 2.1E1 | 6.1E1 | 1.1E2 | 7.3E2 | 3.7E2 | 2.5E3 | 1.0E-9 | 1.0E-9 | 4.7E3 | 1.5E4 | 584 | 43 | 208 | 43 | 0.71 |
| Pf | pg/ml | 1.5E0 | 6.6E0 | 1.1E1 | 3.6E1 | 6.6E1 | 8.7E1 | 1.0E-9 | 1.0E-9 | 1.5E3 | 4.3E2 | 584 | 43 | 208 | 43 | 0.71 |
| Pg | pg/ml | 3.3E0 | 9.7E0 | 5.4E1 | 8.2E1 | 4.2E2 | 2.3E2 | 1.0E-9 | 1.0E-9 | 7.7E3 | 1.2E3 | 584 | 43 | 208 | 43 | 0.66 |
| Ph | ng/ml | 1.6E-1 | 2.2E-1 | 3.3E-1 | 5.2E-1 | 4.9E-1 | 1.1E0 | 1.0E-9 | 1.0E-9 | 3.1E0 | 5.4E0 | 218 | 29 | 134 | 29 | 0.54 |
| Pi | ng/ml | 1.9E-1 | 2.7E-1 | 2.8E-1 | 3.1E0 | 3.7E-1 | 1.5E1 | 1.0E-9 | 1.0E-9 | 3.2E0 | 8.2E1 | 218 | 29 | 134 | 29 | 0.61 |
| Pj | ng/mL | 5.2E0 | 7.4E0 | 6.1E0 | 7.9E0 | 4.7E0 | 4.3E0 | 3.8E-2 | 1.4E0 | 3.1E1 | 2.0E1 | 218 | 29 | 134 | 29 | 0.65 |
| Pk | ng/ml | 8.9E-3 | 1.0E-2 | 1.4E-2 | 6.3E-2 | 2.2E-2 | 2.8E-1 | 1.0E-9 | 1.0E-9 | 2.5E-1 | 1.5E0 | 218 | 29 | 134 | 29 | 0.52 |
| aA | mg/dL | 8.0E-1 | 1.0E0 | 9.1E-1 | 1.3E0 | 4.4E-1 | 9.8E-1 | 2.0E-1 | 4.0E-1 | 4.2E0 | 4.7E0 | 1815 | 54 | 323 | 54 | 0.63 |
| aC | mg/mL | 2.8E0 | 2.1E0 | 3.1E0 | 2.3E0 | 1.3E0 | 9.4E-1 | 8.5E-1 | 7.4E-1 | 8.2E0 | 5.5E0 | 363 | 31 | 141 | 31 | 0.32 |
| aD | ug/mL | 3.1E0 | 4.5E0 | 4.3E0 | 6.0E0 | 3.5E0 | 4.8E0 | 8.5E-1 | 7.7E-1 | 3.1E1 | 2.1E1 | 363 | 31 | 141 | 31 | 0.60 |
| aE | mg/mL | 5.7E-1 | 5.0E-1 | 5.8E-1 | 5.4E-1 | 1.5E-1 | 1.8E-1 | 2.1E-1 | 2.2E-1 | 1.1E0 | 1.2E0 | 363 | 31 | 141 | 31 | 0.40 |
| aF | ng/mL | 2.1E0 | 2.6E0 | 4.1E0 | 4.2E0 | 6.1E0 | 4.7E0 | 4.3E-3 | 3.7E-1 | 5.0E1 | 1.8E1 | 363 | 31 | 141 | 31 | 0.50 |
| aG | mg/mL | 1.4E-1 | 1.3E-1 | 1.6E-1 | 1.5E-1 | 8.4E-2 | 8.5E-2 | 5.0E-2 | 4.3E-2 | 5.0E-1 | 4.2E-1 | 363 | 31 | 141 | 31 | 0.45 |
| aH | ug/mL | 7.5E1 | 7.1E1 | 8.1E1 | 7.7E1 | 4.2E1 | 4.4E1 | 9.6E0 | 1.1E1 | 2.9E2 | 1.9E2 | 363 | 31 | 141 | 31 | 0.47 |
| aI | ug/mL | 1.9E2 | 1.6E2 | 1.9E2 | 1.6E2 | 6.0E1 | 5.6E1 | 4.7E1 | 7.6E1 | 3.7E2 | 2.9E2 | 363 | 31 | 141 | 31 | 0.36 |
| aJ | ug/mL | 2.4E0 | 3.3E0 | 3.0E0 | 4.2E0 | 2.2E0 | 2.8E0 | 9.0E-1 | 1.1E0 | 1.7E1 | 1.2E1 | 363 | 31 | 141 | 31 | 0.65 |
| aK | ng/mL | 1.6E0 | 1.4E0 | 2.5E0 | 1.9E0 | 2.7E0 | 1.7E0 | 2.9E-4 | 1.3E-1 | 1.8E1 | 6.5E0 | 363 | 31 | 141 | 31 | 0.47 |
| aL | mg/mL | 7.9E-1 | 7.6E-1 | 8.0E-1 | 7.6E-1 | 2.4E-1 | 2.5E-1 | 2.2E-1 | 2.7E-1 | 1.7E0 | 1.5E0 | 363 | 31 | 141 | 31 | 0.45 |
| aM | U/mL | 2.2E1 | 3.2E1 | 4.4E1 | 8.4E1 | 1.0E2 | 1.5E2 | 4.2E-2 | 4.2E-2 | 1.6E3 | 8.2E2 | 363 | 31 | 141 | 31 | 0.62 |
| aN | U/mL | 1.4E1 | 2.0E1 | 2.1E1 | 3.2E1 | 3.0E1 | 2.9E1 | 2.5E-3 | 2.5E-3 | 3.8E2 | 1.1E2 | 363 | 31 | 141 | 31 | 0.63 |
| aO | pg/mL | 3.5E1 | 4.3E1 | 3.4E2 | 4.7E2 | 8.8E2 | 7.8E2 | 6.0E-2 | 6.0E-2 | 6.6E3 | 2.5E3 | 363 | 31 | 141 | 31 | 0.54 |
| aP | ng/mL | 1.6E0 | 1.9E0 | 2.0E0 | 2.5E0 | 1.2E0 | 1.6E0 | 5.4E-1 | 6.3E-1 | 7.0E0 | 6.5E0 | 363 | 31 | 141 | 31 | 0.59 |
| aQ | ng/mL | 3.0E-1 | 2.6E-1 | 4.6E-1 | 3.2E-1 | 4.9E-1 | 2.6E-1 | 2.0E-4 | 5.1E-2 | 4.0E0 | 1.2E0 | 363 | 31 | 141 | 31 | 0.43 |
| aR | ng/mL | 1.8E0 | 2.3E0 | 2.9E0 | 2.9E0 | 3.5E0 | 3.0E0 | 1.8E-1 | 7.1E-1 | 3.4E1 | 1.7E1 | 363 | 31 | 141 | 31 | 0.54 |
| aS | ng/mL | 2.7E-1 | 4.0E-1 | 7.2E-1 | 6.3E-1 | 2.0E0 | 7.3E-1 | 4.2E-3 | 6.0E-2 | 3.3E1 | 2.8E0 | 363 | 31 | 141 | 31 | 0.55 |
| aU | pg/mL | 7.8E1 | 6.5E1 | 1.3E2 | 8.8E1 | 1.5E2 | 9.6E1 | 7.4E-2 | 9.6E0 | 1.3E3 | 5.1E2 | 363 | 31 | 141 | 31 | 0.42 |
| aV | ng/mL | 6.3E-1 | 4.2E-1 | 1.1E0 | 8.2E-1 | 2.1E0 | 1.0E0 | 7.6E-4 | 9.1E-2 | 3.3E1 | 5.4E0 | 363 | 31 | 141 | 31 | 0.42 |
| aW | pg/mL | 1.9E1 | 2.0E1 | 2.0E1 | 3.2E1 | 2.1E1 | 7.2E1 | 7.2E-2 | 7.2E-2 | 2.4E2 | 4.2E2 | 363 | 31 | 141 | 31 | 0.52 |
| aX | ng/mL | 9.5E0 | 1.2E1 | 1.4E1 | 1.2E1 | 1.4E1 | 9.7E0 | 3.0E-1 | 1.6E0 | 8.0E1 | 3.1E1 | 363 | 31 | 141 | 31 | 0.47 |
| aY | pg/mL | 6.0E1 | 6.1E1 | 8.0E1 | 6.7E1 | 9.2E1 | 4.8E1 | 4.1E-1 | 4.1E-1 | 1.2E3 | 2.0E2 | 363 | 31 | 141 | 31 | 0.48 |
| aZ | pg/mL | 2.2E2 | 2.9E2 | 5.2E2 | 5.0E2 | 1.0E3 | 6.3E2 | 1.7E0 | 1.7E0 | 1.2E4 | 2.6E3 | 363 | 31 | 141 | 31 | 0.54 |
| bA | ng/mL | 8.5E0 | 3.0E1 | 3.0E1 | 9.5E1 | 7.7E1 | 1.8E2 | 3.0E-2 | 3.0E-2 | 9.4E2 | 9.4E2 | 363 | 31 | 141 | 31 | 0.69 |
| bB | ng/mL | 3.0E2 | 2.4E2 | 3.2E2 | 2.5E2 | 1.6E2 | 1.7E2 | 1.6E1 | 2.3E1 | 1.0E3 | 6.3E2 | 363 | 31 | 141 | 31 | 0.38 |
| bC | ng/mL | 3.3E2 | 3.3E2 | 5.7E2 | 7.6E2 | 7.3E2 | 1.1E3 | 2.7E1 | 5.0E1 | 4.7E3 | 4.7E3 | 363 | 31 | 141 | 31 | 0.55 |
| bE | mg/mL | 5.4E0 | 5.3E0 | 5.7E0 | 5.3E0 | 1.9E0 | 2.0E0 | 1.4E0 | 1.3E0 | 1.3E1 | 1.1E1 | 363 | 31 | 141 | 31 | 0.44 |
| bF | pg/mL | 2.0E1 | 4.8E1 | 1.8E2 | 2.1E2 | 1.1E3 | 5.0E2 | 5.0E-2 | 7.7E0 | 1.1E4 | 2.2E3 | 363 | 31 | 141 | 31 | 0.69 |
| bG | ng/mL | 1.6E0 | 1.8E0 | 2.8E0 | 3.0E0 | 3.4E0 | 3.5E0 | 2.2E-2 | 1.1E-1 | 2.6E1 | 1.5E1 | 363 | 31 | 141 | 31 | 0.51 |
| bH | pg/mL | 5.7E-1 | 5.7E-1 | 5.2E0 | 4.0E0 | 1.7E1 | 5.4E0 | 5.7E-1 | 5.7E-1 | 2.8E2 | 2.0E1 | 363 | 31 | 141 | 31 | 0.51 |
| bI | ng/mL | 4.0E-3 | 4.0E-3 | 7.0E-2 | 7.9E-2 | 1.7E-1 | 2.1E-1 | 4.0E-3 | 4.0E-3 | 1.3E0 | 8.8E-1 | 363 | 31 | 141 | 31 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bJ | mg/mL | 2.2E0 | 2.2E0 | 2.5E0 | 2.5E0 | 1.9E0 | 1.8E0 | 2.5E-4 | 2.5E-4 | 1.3E1 | 7.0E0 | 363 | 31 | 141 | 31 | 0.49 |
| bL | pg/mL | 4.1E0 | 3.1E0 | 8.4E0 | 5.9E0 | 1.0E1 | 6.8E0 | 4.6E-2 | 4.6E-2 | 4.9E1 | 3.2E1 | 363 | 31 | 141 | 31 | 0.46 |
| bM | mg/mL | 1.7E0 | 2.3E0 | 2.1E0 | 2.6E0 | 1.4E0 | 1.7E0 | 9.2E-3 | 1.8E-2 | 7.9E0 | 8.4E0 | 363 | 31 | 141 | 31 | 0.61 |
| bN | ng/mL | 4.7E1 | 3.1E1 | 1.3E2 | 7.8E1 | 2.7E2 | 1.3E2 | 1.4E-1 | 5.9E-1 | 1.9E3 | 4.8E2 | 363 | 31 | 141 | 31 | 0.41 |
| bO | ng/mL | 4.0E-2 | 4.0E-2 | 9.2E0 | 1.0E1 | 2.1E1 | 1.9E1 | 4.0E-2 | 4.0E-2 | 1.9E2 | 6.6E1 | 363 | 31 | 141 | 31 | 0.49 |
| bP | mg/mL | 5.1E-1 | 6.1E-1 | 7.3E-1 | 8.1E-1 | 6.6E-1 | 7.5E-1 | 8.2E-2 | 1.4E-1 | 4.8E0 | 3.5E0 | 363 | 31 | 141 | 31 | 0.54 |
| bQ | pg/mL | 1.5E1 | 2.3E1 | 7.0E1 | 4.7E1 | 7.1E2 | 5.4E1 | 1.5E-1 | 1.5E-1 | 1.3E4 | 2.2E2 | 363 | 31 | 141 | 31 | 0.65 |
| bR | ng/mL | 1.2E-2 | 1.2E-2 | 1.4E-1 | 5.4E-2 | 5.0E-1 | 9.2E-2 | 1.2E-2 | 1.2E-2 | 8.7E0 | 3.9E-1 | 363 | 31 | 141 | 31 | 0.39 |
| bS | pg/mL | 9.4E-1 | 9.4E-1 | 7.8E0 | 2.6E0 | 3.1E1 | 6.4E0 | 9.4E-1 | 9.4E-1 | 3.9E2 | 2.9E1 | 363 | 31 | 141 | 31 | 0.46 |
| bU | ng/mL | 1.3E-1 | 5.5E-2 | 2.1E-1 | 1.2E-1 | 4.1E-1 | 1.5E-1 | 1.3E-2 | 1.3E-2 | 6.6E0 | 6.3E-1 | 363 | 31 | 141 | 31 | 0.42 |
| bV | pg/mL | 4.7E2 | 5.6E2 | 5.3E2 | 7.1E2 | 2.4E2 | 5.8E2 | 1.7E2 | 1.9E2 | 1.6E3 | 3.1E3 | 363 | 31 | 141 | 31 | 0.59 |
| bW | pg/mL | 3.4E2 | 3.3E2 | 4.9E2 | 1.3E3 | 5.0E2 | 4.4E3 | 8.4E1 | 1.2E2 | 4.8E3 | 2.5E4 | 363 | 31 | 141 | 31 | 0.53 |
| bX | ng/mL | 1.5E-3 | 2.5E-5 | 2.8E-3 | 2.3E-3 | 3.4E-3 | 2.6E-3 | 2.5E-5 | 2.5E-5 | 2.0E-2 | 7.1E-3 | 363 | 31 | 141 | 31 | 0.47 |
| bZ | pg/mL | 2.4E2 | 3.1E2 | 9.1E2 | 1.0E3 | 4.3E3 | 1.5E3 | 1.5E-1 | 1.5E1 | 5.8E4 | 5.6E3 | 363 | 31 | 141 | 31 | 0.65 |
| cA | pg/mL | 6.0E-1 | 6.0E-1 | 3.0E0 | 1.1E0 | 2.0E1 | 2.6E0 | 6.0E-1 | 6.0E-1 | 3.7E2 | 1.5E1 | 363 | 31 | 141 | 31 | 0.45 |
| cB | ng/mL | 5.4E-2 | 4.1E-2 | 8.6E-2 | 6.0E-2 | 1.0E-1 | 8.3E-2 | 1.7E-3 | 1.7E-3 | 5.7E-1 | 4.0E-1 | 363 | 31 | 141 | 31 | 0.41 |
| cC | pg/mL | 4.6E1 | 4.9E1 | 4.9E1 | 3.9E1 | 4.2E1 | 2.6E1 | 1.0E0 | 1.0E0 | 4.5E2 | 7.3E1 | 363 | 31 | 141 | 31 | 0.45 |
| cD | pg/mL | 5.2E0 | 4.9E0 | 1.3E1 | 1.2E1 | 3.9E1 | 1.9E1 | 3.3E-1 | 3.3E-1 | 4.8E2 | 9.0E1 | 363 | 31 | 141 | 31 | 0.51 |
| cE | pg/mL | 3.2E1 | 9.6E1 | 1.5E2 | 2.1E2 | 4.8E2 | 3.0E2 | 1.2E-1 | 3.6E0 | 3.8E3 | 1.3E3 | 363 | 31 | 141 | 31 | 0.69 |
| cF | pg/mL | 1.2E1 | 5.3E-1 | 2.1E1 | 9.0E0 | 3.4E1 | 1.3E1 | 5.3E-1 | 5.3E-1 | 2.7E2 | 5.0E1 | 363 | 31 | 141 | 31 | 0.39 |
| cG | pg/mL | 4.3E1 | 8.4E1 | 1.1E2 | 1.1E2 | 5.7E2 | 9.5E1 | 7.8E0 | 6.4E0 | 1.0E4 | 4.1E2 | 363 | 31 | 141 | 31 | 0.71 |
| cH | uIU/mL | 3.1E0 | 2.1E0 | 6.7E0 | 4.3E0 | 1.3E1 | 7.8E0 | 8.6E-3 | 8.6E-3 | 1.6E2 | 3.9E1 | 363 | 31 | 141 | 31 | 0.37 |
| cI | ng/mL | 5.6E0 | 5.7E0 | 1.1E1 | 1.6E1 | 1.5E1 | 2.5E1 | 1.0E-3 | 1.0E-3 | 9.4E1 | 1.2E2 | 363 | 31 | 141 | 31 | 0.51 |
| cJ | ug/mL | 6.5E1 | 4.8E1 | 1.2E2 | 8.1E1 | 1.5E2 | 9.1E1 | 4.0E0 | 7.2E0 | 9.6E2 | 3.4E2 | 363 | 31 | 141 | 31 | 0.43 |
| cK | ng/mL | 3.8E-3 | 3.8E-3 | 5.9E-2 | 1.6E-2 | 2.0E-1 | 4.1E-2 | 3.8E-3 | 3.8E-3 | 1.7E0 | 2.1E-1 | 363 | 31 | 141 | 31 | 0.48 |
| cL | pg/mL | 1.9E2 | 2.0E2 | 4.0E2 | 2.8E2 | 1.5E3 | 2.4E2 | 1.6E1 | 4.8E1 | 2.4E4 | 1.4E3 | 363 | 31 | 141 | 31 | 0.56 |
| cM | pg/mL | 2.8E2 | 2.6E2 | 3.1E2 | 2.6E2 | 2.1E2 | 1.2E2 | 8.7E0 | 5.7E1 | 1.6E3 | 5.1E2 | 363 | 31 | 141 | 31 | 0.45 |
| cN | pg/mL | 1.2E2 | 1.3E2 | 1.3E2 | 1.4E2 | 7.0E1 | 5.0E1 | 3.8E1 | 7.4E1 | 1.1E3 | 2.9E2 | 363 | 31 | 141 | 31 | 0.60 |
| cO | pg/mL | 2.2E2 | 2.4E2 | 3.3E2 | 2.5E2 | 1.0E3 | 9.7E1 | 5.4E1 | 1.2E2 | 1.9E4 | 5.0E2 | 363 | 31 | 141 | 31 | 0.52 |
| cP | ng/mL | 2.6E3 | 2.3E3 | 2.6E3 | 2.7E3 | 9.4E2 | 1.1E3 | 6.2E2 | 1.3E3 | 5.7E3 | 5.9E3 | 363 | 31 | 141 | 31 | 0.48 |
| cQ | ng/mL | 4.9E-2 | 4.5E-2 | 1.2E-1 | 1.5E-1 | 2.3E-1 | 2.8E-1 | 2.0E-3 | 2.0E-3 | 2.2E0 | 1.2E0 | 363 | 31 | 141 | 31 | 0.49 |
| cR | ng/mL | 2.8E2 | 3.8E2 | 4.3E2 | 5.1E2 | 6.0E2 | 5.0E2 | 2.0E1 | 7.0E1 | 7.7E3 | 2.2E3 | 363 | 31 | 141 | 31 | 0.58 |
| cS | ng/mL | 2.6E2 | 3.0E2 | 3.7E2 | 5.1E2 | 3.7E2 | 6.4E2 | 4.7E1 | 4.8E1 | 2.7E3 | 2.6E3 | 363 | 31 | 141 | 31 | 0.52 |
| cT | ng/mL | 3.1E1 | 8.4E1 | 8.6E1 | 1.7E2 | 1.9E2 | 3.1E2 | 4.6E0 | 4.0E0 | 2.1E3 | 1.4E3 | 363 | 31 | 141 | 31 | 0.65 |
| cU | ng/mL | 5.3E1 | 6.6E1 | 7.7E1 | 9.5E1 | 1.1E2 | 8.5E1 | 6.2E0 | 1.6E1 | 1.6E3 | 4.2E2 | 363 | 31 | 141 | 31 | 0.59 |
| cV | ng/mL | 1.7E-1 | 2.8E-1 | 4.2E-1 | 4.8E-1 | 2.5E0 | 7.5E-1 | 3.4E-4 | 3.6E-2 | 4.7E1 | 4.2E0 | 363 | 31 | 141 | 31 | 0.67 |
| cW | mIU/mL | 5.3E-2 | 4.2E-2 | 1.6E-1 | 8.3E-2 | 7.9E-1 | 8.7E-2 | 3.7E-4 | 1.4E-2 | 9.7E0 | 3.9E-1 | 363 | 31 | 141 | 31 | 0.50 |
| cX | ng/mL | 1.0E-1 | 1.2E-1 | 1.4E0 | 1.8E0 | 4.5E0 | 5.1E0 | 2.3E-4 | 2.3E-4 | 2.8E1 | 2.8E1 | 363 | 31 | 141 | 31 | 0.50 |
| cY | ng/mL | 8.8E0 | 9.0E0 | 1.3E1 | 1.0E1 | 1.4E1 | 7.8E0 | 1.5E-1 | 9.3E-1 | 8.3E1 | 3.6E1 | 363 | 31 | 141 | 31 | 0.47 |
| cZ | ug/mL | 1.4E1 | 1.3E1 | 1.5E1 | 1.4E1 | 6.1E0 | 6.3E0 | 2.7E0 | 3.3E0 | 3.9E1 | 3.1E1 | 363 | 31 | 141 | 31 | 0.43 |
| dA | pg/mL | 3.3E2 | 3.5E2 | 3.7E2 | 3.7E2 | 3.3E2 | 2.0E2 | 9.0E1 | 1.5E2 | 5.8E3 | 1.1E3 | 363 | 31 | 141 | 31 | 0.50 |
| dB | ug/mL | 1.7E1 | 2.1E1 | 1.8E1 | 1.7E1 | 1.8E1 | 1.0E1 | 9.4E-1 | 2.2E0 | 2.5E2 | 3.0E1 | 363 | 31 | 141 | 31 | 0.56 |
| dC | nmol/L | 3.5E1 | 3.8E1 | 4.0E1 | 3.9E1 | 1.9E1 | 1.5E1 | 9.1E0 | 1.6E1 | 1.4E2 | 7.9E1 | 363 | 31 | 141 | 31 | 0.51 |
| dD | ug/mL | 3.6E1 | 2.9E1 | 3.7E1 | 3.2E1 | 1.1E1 | 1.1E1 | 1.3E1 | 1.4E1 | 7.6E1 | 5.7E1 | 363 | 31 | 141 | 31 | 0.34 |
| dE | ng/mL | 4.7E-1 | 3.0E-1 | 6.1E-1 | 4.5E-1 | 7.4E-1 | 5.6E-1 | 8.4E-3 | 8.4E-3 | 7.2E0 | 2.4E0 | 363 | 31 | 141 | 31 | 0.43 |
| dF | ng/mL | 2.2E2 | 2.8E2 | 2.7E2 | 4.4E2 | 1.8E2 | 3.2E2 | 7.5E1 | 8.4E1 | 1.3E3 | 1.2E3 | 363 | 31 | 141 | 31 | 0.70 |
| dG | ng/mL | 1.1E1 | 1.6E1 | 1.4E1 | 2.1E1 | 1.3E1 | 1.8E1 | 3.1E0 | 3.9E0 | 1.8E2 | 8.7E1 | 363 | 31 | 141 | 31 | 0.67 |
| dH | pg/mL | 7.5E0 | 1.0E1 | 1.3E1 | 1.2E1 | 4.1E1 | 9.6E0 | 4.0E-2 | 4.0E-2 | 6.7E2 | 4.9E1 | 363 | 31 | 141 | 31 | 0.60 |
| dI | pg/mL | 4.6E-1 | 4.6E-1 | 2.5E0 | 1.6E0 | 1.8E1 | 2.1E0 | 4.6E-1 | 4.6E-1 | 3.3E2 | 7.5E0 | 363 | 31 | 141 | 31 | 0.53 |
| dJ | ng/mL | 1.9E0 | 1.9E0 | 2.2E0 | 1.8E0 | 1.2E0 | 1.2E0 | 3.2E-2 | 3.2E-2 | 6.9E0 | 4.0E0 | 363 | 31 | 141 | 31 | 0.44 |
| dK | uIU/mL | 1.9E0 | 1.3E0 | 3.2E0 | 2.7E0 | 6.5E0 | 4.0E0 | 2.8E-4 | 2.9E-2 | 7.9E1 | 2.2E1 | 363 | 31 | 141 | 31 | 0.46 |
| dL | ng/mL | 8.8E2 | 1.2E3 | 1.0E3 | 1.3E3 | 4.9E2 | 9.2E2 | 3.4E2 | 4.1E2 | 3.4E3 | 4.8E3 | 363 | 31 | 141 | 31 | 0.59 |
| dM | pg/mL | 9.7E2 | 1.1E3 | 1.2E3 | 2.0E3 | 9.3E2 | 2.0E3 | 3.9E2 | 3.9E2 | 1.2E4 | 9.6E3 | 363 | 31 | 141 | 31 | 0.65 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dN | ug/mL | 9.4E1 | 1.0E2 | 9.9E1 | 1.2E2 | 3.4E1 | 6.5E1 | 2.5E1 | 3.4E1 | 2.4E2 | 3.3E2 | 363 | 31 | 141 | 31 | 0.59 |
| dR | pg/ml | 1.6E3 | 1.1E3 | 2.4E3 | 1.6E3 | 2.5E3 | 1.5E3 | 1.4E2 | 3.4E2 | 1.5E4 | 5.3E3 | 245 | 28 | 138 | 28 | 0.42 |
| dU | pg/ml | 9.7E3 | 1.4E4 | 1.4E4 | 1.6E4 | 1.2E4 | 1.2E4 | 6.9E2 | 3.1E3 | 5.3E4 | 3.8E4 | 40 | 13 | 37 | 13 | 0.56 |
| dX | ng/ml | 3.4E-2 | 8.2E-2 | 1.2E-1 | 1.6E-1 | 2.0E-1 | 1.6E-1 | 2.6E-3 | 2.6E-3 | 1.2E0 | 4.2E-1 | 92 | 7 | 30 | 7 | 0.63 |
| cF | ng/ml | 4.1E0 | 4.7E0 | 4.6E0 | 5.6E0 | 2.2E0 | 2.8E0 | 1.4E0 | 2.0E0 | 1.8E1 | 1.5E1 | 257 | 28 | 138 | 28 | 0.62 |
| eC | pg/ml | 3.1E2 | 2.4E2 | 3.7E2 | 3.3E2 | 2.2E2 | 4.2E2 | 4.5E1 | 1.9E1 | 1.4E3 | 2.0E3 | 203 | 27 | 135 | 27 | 0.33 |
| eD | pg/ml | 2.1E2 | 1.8E2 | 5.9E2 | 4.4E2 | 1.2E3 | 8.9E2 | 5.2E-1 | 5.2E-1 | 7.0E3 | 3.8E3 | 173 | 16 | 111 | 16 | 0.47 |
| eM | ng/ml | 3.5E0 | 2.5E0 | 4.7E0 | 4.6E0 | 4.4E0 | 6.3E0 | 7.6E-1 | 7.1E-1 | 2.4E1 | 2.6E1 | 122 | 17 | 50 | 17 | 0.39 |
| eP | ng/ml | 3.7E-3 | 3.7E-3 | 7.3E-1 | 4.4E0 | 1.8E0 | 1.0E1 | 3.7E-3 | 3.7E-3 | 1.2E1 | 2.8E1 | 92 | 7 | 30 | 7 | 0.55 |
| eT | ng/ml | 2.9E2 | 6.1E2 | 6.4E2 | 1.0E3 | 7.2E2 | 1.0E3 | 1.0E2 | 7.1E1 | 2.9E3 | 2.9E3 | 100 | 10 | 81 | 10 | 0.59 |
| eQ | pg/ml | 1.0E0 | 1.0E0 | 8.6E1 | 1.7E1 | 2.7E2 | 4.3E1 | 1.0E0 | 1.0E0 | 1.6E3 | 1.5E2 | 40 | 13 | 37 | 13 | 0.41 |
| fA | ng/ml | 1.9E2 | 1.1E2 | 3.8E2 | 3.4E2 | 4.4E2 | 4.1E2 | 2.6E1 | 6.2E1 | 1.5E3 | 1.2E3 | 40 | 13 | 37 | 13 | 0.46 |
| eZ | ng/ml | 5.2E1 | 6.0E1 | 6.0E1 | 6.6E1 | 2.6E1 | 2.6E1 | 1.8E1 | 3.2E1 | 1.2E2 | 1.1E2 | 100 | 10 | 81 | 10 | 0.59 |
| fB | ng/ml | 6.1E2 | 6.8E2 | 6.9E2 | 7.2E2 | 3.1E2 | 2.6E2 | 1.6E2 | 3.5E2 | 1.5E3 | 1.3E3 | 40 | 14 | 37 | 14 | 0.55 |
| fN | ng/ml | 2.1E-1 | 2.3E0 | 2.6E0 | 5.2E0 | 5.2E0 | 5.9E0 | 2.1E-1 | 2.1E-1 | 3.2E1 | 1.4E1 | 100 | 10 | 81 | 10 | 0.62 |
| fP | ng/ml | 2.5E2 | 3.1E2 | 2.9E2 | 3.5E2 | 1.7E2 | 2.2E2 | 1.8E0 | 3.3E1 | 1.0E3 | 8.6E2 | 240 | 28 | 137 | 28 | 0.58 |
| fR | ng/ml | 1.2E5 | 2.0E5 | 1.7E5 | 2.6E5 | 1.4E5 | 1.9E5 | 3.1E4 | 1.9E2 | 7.2E5 | 6.8E5 | 236 | 19 | 68 | 19 | 0.67 |
| fY | ng/ml | 2.6E2 | 2.2E2 | 2.5E2 | 2.4E2 | 1.0E2 | 1.1E2 | 3.6E1 | 1.2E2 | 4.8E2 | 4.3E2 | 100 | 10 | 81 | 10 | 0.46 |
| gC | ng/ml | 2.3E2 | 2.4E2 | 2.5E2 | 2.6E2 | 1.0E2 | 8.4E1 | 8.3E1 | 1.6E2 | 6.4E2 | 4.5E2 | 103 | 15 | 58 | 15 | 0.58 |
| gL | pg/ml | 6.3E4 | 6.7E4 | 6.9E4 | 7.6E4 | 2.8E4 | 3.4E4 | 1.1E4 | 4.4E4 | 1.8E5 | 1.7E5 | 245 | 28 | 138 | 28 | 0.56 |
| gP | U/ml | 2.8E2 | 2.5E2 | 2.8E2 | 2.7E2 | 1.1E2 | 9.4E1 | 1.2E1 | 9.6E1 | 1.1E3 | 5.2E2 | 253 | 28 | 138 | 28 | 0.47 |
| gW | ng/ml | 5.7E2 | 3.7E2 | 1.2E3 | 9.0E2 | 1.7E3 | 1.2E3 | 3.1E-1 | 1.5E2 | 9.5E3 | 4.3E3 | 213 | 16 | 129 | 16 | 0.47 |
| tF | pg/mL | 1.5E3 | 1.1E3 | 1.6E4 | 4.2E3 | 4.7E4 | 5.7E3 | 1.2E1 | 1.8E1 | 3.2E5 | 2.0E4 | 203 | 27 | 135 | 27 | 0.50 |
| gZ | ug/ml | 8.5E-1 | 6.3E-1 | 4.2E1 | 7.7E1 | 1.1E2 | 1.5E2 | 8.7E-2 | 1.1E-1 | 4.1E2 | 4.1E2 | 40 | 13 | 37 | 13 | 0.54 |
| hA | ng/ml | 2.2E0 | 4.8E0 | 1.0E1 | 7.4E0 | 3.9E1 | 6.8E0 | 1.7E-2 | 6.3E-2 | 3.5E2 | 2.5E1 | 173 | 18 | 111 | 18 | 0.68 |
| nM | pg/ml | 1.0E-9 | 1.0E-9 | 9.1E1 | 1.0E-9 | 9.6E2 | 0.0E0 | 1.0E-9 | 1.0E-9 | 1.0E4 | 1.0E-9 | 113 | 21 | 88 | 21 | 0.49 |
| nN | pg/ml | 1.4E3 | 2.0E3 | 5.8E3 | 3.4E3 | 2.7E4 | 4.6E3 | 1.1E2 | 2.6E2 | 2.7E5 | 2.1E4 | 113 | 21 | 88 | 21 | 0.58 |
| nO | pg/ml | 2.6E1 | 2.9E1 | 4.1E1 | 4.5E1 | 4.3E1 | 5.1E1 | 3.5E0 | 8.1E0 | 2.4E2 | 2.4E2 | 113 | 21 | 88 | 21 | 0.55 |
| nR | pg/ml | 1.5E1 | 2.2E1 | 4.3E1 | 5.8E1 | 9.3E1 | 9.6E1 | 1.0E-9 | 1.9E0 | 8.2E2 | 4.2E2 | 113 | 21 | 88 | 21 | 0.59 |
| nT | pg/ml | 7.3E1 | 9.0E1 | 2.2E2 | 9.2E1 | 8.5E2 | 3.5E1 | 1.0E-9 | 1.4E1 | 6.6E3 | 1.8E2 | 113 | 21 | 88 | 21 | 0.55 |
| nU | pg/ml | 3.9E1 | 4.4E1 | 3.0E2 | 6.1E1 | 1.6E3 | 6.5E1 | 1.0E-9 | 1.0E-9 | 1.3E4 | 2.2E2 | 113 | 21 | 88 | 21 | 0.51 |
| lW | pg/ml | 1.0E-9 | 1.0E-9 | 1.5E1 | 5.6E0 | 5.1E1 | 1.1E1 | 1.0E-9 | 1.0E-9 | 3.9E2 | 4.4E1 | 113 | 21 | 88 | 21 | 0.50 |
| lX | pg/ml | 9.4E2 | 5.3E2 | 9.9E2 | 8.1E2 | 5.3E2 | 5.5E2 | 1.2E2 | 1.9E2 | 2.5E3 | 2.5E3 | 113 | 21 | 88 | 21 | 0.38 |
| lY | pg/ml | 1.9E1 | 1.7E1 | 2.3E1 | 1.6E1 | 2.2E1 | 1.1E1 | 1.0E-9 | 5.7E-1 | 1.4E2 | 4.5E1 | 113 | 21 | 88 | 21 | 0.41 |
| mE | pg/ml | 1.0E-9 | 1.0E-9 | 2.9E0 | 1.7E0 | 8.8E0 | 3.4E0 | 1.0E-9 | 1.0E-9 | 5.8E1 | 1.3E1 | 113 | 21 | 88 | 21 | 0.50 |
| mF | pg/ml | 1.0E-9 | 2.7E-1 | 4.3E0 | 2.0E0 | 2.5E1 | 4.0E0 | 1.0E-9 | 1.0E-9 | 2.5E2 | 1.3E1 | 113 | 21 | 88 | 21 | 0.56 |
| mH | pg/ml | 3.2E0 | 2.7E0 | 4.8E0 | 4.6E0 | 6.6E0 | 4.5E0 | 2.3E-1 | 9.0E-1 | 5.3E1 | 1.9E1 | 113 | 21 | 88 | 21 | 0.50 |
| mI | pg/ml | 1.0E-9 | 1.0E-9 | 1.3E1 | 1.4E1 | 2.9E1 | 2.3E1 | 1.0E-9 | 1.0E-9 | 1.8E2 | 7.8E1 | 113 | 21 | 88 | 21 | 0.53 |
| mM | pg/ml | 2.8E1 | 4.0E1 | 8.0E1 | 7.5E1 | 1.6E2 | 1.1E2 | 1.0E-9 | 1.0E-9 | 1.1E3 | 4.7E2 | 113 | 21 | 88 | 21 | 0.55 |
| mP | pg/ml | 1.4E1 | 1.5E1 | 2.0E1 | 1.4E1 | 2.6E1 | 6.8E0 | 1.0E-9 | 1.0E-9 | 1.9E2 | 2.4E1 | 112 | 21 | 87 | 21 | 0.48 |
| mS | pg/ml | 1.8E3 | 1.7E3 | 2.0E3 | 1.6E3 | 1.7E3 | 8.0E2 | 1.0E-9 | 1.0E-9 | 1.3E4 | 3.2E3 | 113 | 21 | 88 | 21 | 0.42 |
| mT | pg/ml | 5.4E1 | 5.9E1 | 1.2E2 | 6.4E1 | 1.9E2 | 3.3E1 | 1.0E1 | 1.2E1 | 1.4E3 | 1.4E2 | 112 | 21 | 87 | 21 | 0.49 |
| mU | pg/ml | 2.3E0 | 3.1E0 | 6.3E0 | 3.1E0 | 2.2E1 | 1.4E0 | 1.0E-9 | 6.0E-1 | 2.2E2 | 6.6E0 | 112 | 21 | 87 | 21 | 0.59 |
| mW | pg/ml | 2.5E3 | 2.2E3 | 2.8E3 | 2.4E3 | 1.6E3 | 1.5E3 | 4.3E2 | 1.7E2 | 1.0E4 | 6.2E3 | 112 | 21 | 87 | 21 | 0.44 |
| mY | pg/ml | 6.2E2 | 8.8E2 | 9.1E2 | 8.9E2 | 1.4E3 | 6.6E2 | 1.0E-9 | 1.0E-9 | 1.1E4 | 2.6E3 | 113 | 21 | 88 | 21 | 0.58 |
| mZ | pg/ml | 2.4E2 | 9.7E1 | 4.0E2 | 3.7E2 | 4.6E2 | 6.2E2 | 2.1E0 | 1.1E1 | 3.1E3 | 2.8E3 | 112 | 21 | 87 | 21 | 0.36 |
| nA | pg/ml | 1.6E2 | 4.8E-1 | 1.2E1 | 2.6E0 | 4.7E1 | 3.7E0 | 1.0E-9 | 1.0E-9 | 4.4E2 | 1.4E1 | 112 | 21 | 87 | 21 | 0.41 |
| nB | pg/ml | 3.1E2 | 3.2E2 | 3.3E2 | 3.4E2 | 1.5E2 | 1.9E2 | 3.0E1 | 7.9E1 | 8.2E2 | 1.0E3 | 113 | 21 | 88 | 21 | 0.52 |
| nC | pg/ml | 1.0E-9 | 7.5E1 | 4.3E3 | 7.4E4 | 3.4E4 | 3.3E5 | 1.0E-9 | 1.0E-9 | 3.7E5 | 1.5E6 | 113 | 21 | 88 | 21 | 0.58 |
| nD | pg/ml | 7.9E0 | 3.8E0 | 3.8E1 | 9.0E0 | 2.2E2 | 1.0E1 | 1.0E-9 | 1.0E-9 | 1.7E3 | 2.9E1 | 112 | 21 | 87 | 21 | 0.44 |
| nF | pg/ml | 1.0E-9 | 1.0E-9 | 5.0E0 | 2.0E0 | 2.8E1 | 6.2E0 | 1.0E-9 | 1.0E-9 | 2.4E2 | 2.1E1 | 113 | 21 | 88 | 21 | 0.50 |
| nH | pg/ml | 3.8E-1 | 2.0E0 | 1.0E2 | 1.4E2 | 9.5E2 | 5.7E2 | 1.0E-9 | 1.0E-9 | 1.0E4 | 2.6E3 | 112 | 21 | 87 | 21 | 0.57 |
| nI | pg/ml | 2.8E0 | 1.0E-9 | 1.7E2 | 2.6E1 | 9.0E2 | 4.2E1 | 1.0E-9 | 1.0E-9 | 9.2E3 | 1.3E2 | 113 | 21 | 88 | 21 | 0.37 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| nJ | pg/ml | 1.7E-1 | 4.0E-1 | 4.8E1 | 6.5E-1 | 4.8E2 | 8.0E-1 | 1.0E-9 | 1.0E-9 | 5.2E3 | 2.4E0 | 113 | 21 | 88 | 21 | 0.48 |
| nK | pg/ml | 1.0E-9 | 1.0E-9 | 6.2E1 | 1.0E1 | 3.7E2 | 1.4E1 | 1.0E-9 | 1.0E-9 | 3.2E3 | 4.2E1 | 112 | 21 | 87 | 21 | 0.52 |
| nL | pg/ml | 1.0E-9 | 1.4E0 | 4.5E2 | 3.0E2 | 4.2E3 | 1.1E3 | 1.0E-9 | 1.0E-9 | 4.5E4 | 5.2E3 | 113 | 21 | 88 | 21 | 0.59 |
| hL | pg/ml | 1.9E4 | 2.6E4 | 2.3E4 | 2.9E4 | 1.8E4 | 1.7E4 | 2.6E3 | 7.9E3 | 1.4E5 | 6.0E4 | 100 | 10 | 81 | 10 | 0.63 |
| hO | pg/ml | 1.6E4 | 1.5E4 | 1.7E4 | 1.5E4 | 3.2E3 | 3.3E3 | 1.1E4 | 1.1E4 | 2.8E4 | 2.3E4 | 100 | 10 | 81 | 10 | 0.38 |
| hP | ng/ml | 4.4E5 | 4.4E5 | 4.3E5 | 6.1E5 | 3.4E5 | 7.9E5 | 1.7E4 | 3.4E4 | 2.6E6 | 2.8E6 | 100 | 10 | 81 | 10 | 0.52 |
| wJ | pg/ml | 1.5E5 | 1.0E5 | 1.8E5 | 1.2E5 | 1.1E5 | 7.9E4 | 1.1E4 | 2.3E4 | 5.8E5 | 2.5E5 | 99 | 9 | 76 | 9 | 0.34 |
| wK | pg/ml | 3.5E4 | 3.5E4 | 4.9E4 | 4.0E4 | 5.6E4 | 3.3E4 | 3.7E3 | 1.1E4 | 5.0E5 | 1.2E5 | 99 | 9 | 76 | 9 | 0.44 |
| wL | pg/ml | 4.0E0 | 5.3E0 | 4.6E1 | 4.9E1 | 1.1E2 | 1.1E2 | 1.0E-9 | 1.0E-9 | 8.4E2 | 3.5E2 | 99 | 9 | 76 | 9 | 0.47 |
| wP | pg/ml | 2.9E4 | 7.0E4 | 4.4E4 | 8.7E4 | 4.8E4 | 8.1E4 | 1.3E3 | 1.8E4 | 3.0E5 | 2.6E5 | 99 | 9 | 76 | 9 | 0.71 |
| wQ | pg/ml | 3.8E1 | 2.9E1 | 6.2E1 | 4.0E1 | 8.3E1 | 4.9E1 | 1.0E-9 | 1.0E-9 | 5.1E2 | 1.6E2 | 99 | 9 | 76 | 9 | 0.44 |
| hR | pg/ml | 2.6E4 | 2.9E4 | 2.7E4 | 2.9E4 | 1.1E4 | 9.4E3 | 1.1E1 | 1.3E4 | 5.8E4 | 4.4E4 | 165 | 17 | 109 | 17 | 0.56 |
| hV | pg/ml | 4.3E2 | 3.6E2 | 4.5E2 | 4.3E2 | 2.4E2 | 2.5E2 | 6.8E1 | 9.5E1 | 1.5E3 | 9.6E2 | 165 | 17 | 109 | 17 | 0.46 |
| hW | pg/ml | 1.6E3 | 2.4E3 | 2.1E3 | 4.9E3 | 1.7E3 | 9.1E3 | 2.2E2 | 1.2E3 | 1.0E4 | 4.0E4 | 165 | 17 | 109 | 17 | 0.72 |
| hX | pg/ml | 9.0E2 | 1.2E3 | 1.0E3 | 1.4E3 | 8.2E2 | 1.4E3 | 1.3E2 | 3.1E2 | 8.6E3 | 6.6E3 | 165 | 17 | 109 | 17 | 0.63 |
| iA | pg/ml | 1.5E2 | 2.0E2 | 3.0E2 | 3.4E2 | 6.6E2 | 2.6E2 | 1.1E1 | 7.0E1 | 7.1E3 | 8.7E2 | 203 | 26 | 135 | 26 | 0.65 |
| iB | ng/ml | 5.0E0 | 7.7E0 | 6.3E0 | 8.8E0 | 5.0E0 | 6.4E0 | 3.3E-2 | 1.1E0 | 3.8E1 | 2.2E1 | 173 | 18 | 111 | 18 | 0.61 |
| iC | U/ml | 2.3E-1 | 6.1E-1 | 1.0E0 | 1.5E0 | 4.9E0 | 2.8E0 | 1.0E-9 | 6.8E-2 | 5.5E1 | 1.2E1 | 173 | 18 | 111 | 18 | 0.68 |
| tQ | pg/ml | 1.1E3 | 1.3E3 | 1.2E3 | 1.5E3 | 5.7E2 | 8.8E2 | 2.8E2 | 6.3E2 | 2.5E3 | 3.3E3 | 94 | 8 | 73 | 8 | 0.55 |
| tT | pg/ml | 1.7E1 | 2.6E1 | 2.0E1 | 2.9E1 | 1.5E1 | 2.1E1 | 5.4E0 | 7.2E0 | 1.2E2 | 6.7E1 | 95 | 8 | 74 | 8 | 0.60 |
| tS | pg/ml | 1.0E0 | 1.1E0 | 1.1E0 | 1.5E0 | 1.0E0 | 2.2E0 | 1.0E-9 | 1.0E-9 | 5.5E0 | 7.0E0 | 95 | 9 | 74 | 9 | 0.49 |
| tX | pg/ml | 9.9E-1 | 1.2E0 | 1.1E0 | 2.3E0 | 9.4E-1 | 2.4E0 | 2.5E-2 | 9.3E-2 | 7.0E0 | 6.2E0 | 95 | 8 | 74 | 8 | 0.61 |
| tO | pg/ml | 4.4E0 | 3.9E0 | 4.9E0 | 4.5E0 | 3.2E0 | 2.7E0 | 1.0E-9 | 1.7E0 | 1.8E1 | 9.6E0 | 95 | 9 | 74 | 9 | 0.46 |
| tR | pg/ml | 1.9E-1 | 2.2E-1 | 2.7E-1 | 3.3E-1 | 2.7E-1 | 3.9E-1 | 1.0E-9 | 1.4E-2 | 1.6E0 | 1.3E0 | 94 | 9 | 73 | 9 | 0.52 |
| tU | pg/ml | 9.1E0 | 1.2E1 | 1.1E1 | 1.9E1 | 6.9E0 | 2.3E1 | 2.2E-1 | 5.0E0 | 4.4E1 | 8.0E1 | 97 | 9 | 75 | 9 | 0.62 |
| tN | pg/ml | 1.8E1 | 3.2E1 | 2.2E1 | 3.3E1 | 1.9E1 | 2.0E1 | 1.0E-9 | 6.1E0 | 1.5E2 | 6.5E1 | 95 | 8 | 73 | 8 | 0.68 |
| tV | ng/ml | 4.8E2 | 8.7E2 | 6.3E2 | 9.2E2 | 4.7E2 | 5.0E2 | 5.3E1 | 3.9E2 | 2.6E3 | 1.7E3 | 99 | 8 | 76 | 8 | 0.70 |
| iH | ng/ml | 1.6E5 | 1.5E5 | 1.5E5 | 1.5E5 | 4.7E4 | 4.7E4 | 5.1E4 | 5.1E4 | 2.7E5 | 2.4E5 | 203 | 26 | 135 | 26 | 0.49 |
| iJ | ng/ml | 5.3E4 | 4.6E4 | 5.5E4 | 5.1E4 | 3.0E4 | 3.2E4 | 7.7E3 | 1.1E4 | 2.5E5 | 1.8E5 | 203 | 26 | 135 | 26 | 0.43 |
| hB | ng/ml | 4.3E-1 | 5.6E-1 | 5.4E-1 | 8.8E-1 | 4.1E-1 | 7.6E-1 | 1.2E-1 | 1.6E-1 | 3.0E0 | 3.2E0 | 203 | 26 | 135 | 26 | 0.65 |
| hC | pg/ml | 4.1E3 | 7.6E3 | 7.1E3 | 1.2E4 | 1.0E4 | 1.4E4 | 1.0E-9 | 4.5E1 | 1.1E5 | 5.7E4 | 203 | 26 | 135 | 26 | 0.59 |
| hF | ng/ml | 1.0E-9 | 1.0E-9 | 2.4E1 | 3.7E-1 | 2.9E2 | 1.9E0 | 1.0E-9 | 1.0E-9 | 4.0E3 | 9.6E0 | 203 | 26 | 135 | 26 | 0.51 |
| hG | pg/ml | 7.0E3 | 7.4E3 | 7.7E3 | 8.5E3 | 3.3E3 | 4.2E3 | 1.7E3 | 3.6E3 | 2.0E4 | 2.0E4 | 203 | 26 | 135 | 26 | 0.53 |
| iO | ng/ml | 3.8E5 | 3.7E5 | 4.0E5 | 4.0E5 | 1.8E5 | 1.9E5 | 8.3E4 | 8.3E4 | 1.1E6 | 8.2E5 | 203 | 26 | 135 | 26 | 0.50 |
| iP | ng/ml | 5.1E4 | 7.0E4 | 5.7E4 | 7.2E4 | 5.5E4 | 1.0E5 | 2.4E3 | 7.2E3 | 5.5E5 | 5.7E5 | 203 | 26 | 135 | 26 | 0.54 |
| iZ | ng/ml | 1.6E3 | 2.0E3 | 1.8E3 | 2.1E3 | 7.6E2 | 1.0E3 | 4.7E2 | 9.8E2 | 5.7E3 | 4.6E3 | 201 | 25 | 133 | 25 | 0.55 |
| yH | pg/ml | 1.1E3 | 1.4E3 | 3.4E3 | 1.9E3 | 1.3E4 | 2.3E3 | 1.0E-9 | 3.0E2 | 1.2E5 | 7.7E3 | 99 | 9 | 77 | 9 | 0.54 |
| yK | U/ml | 2.1E1 | 2.8E1 | 4.5E1 | 3.3E1 | 7.1E1 | 3.2E1 | 1.0E-9 | 1.0E-9 | 3.8E2 | 1.0E2 | 99 | 9 | 77 | 9 | 0.50 |
| yJ | pg/ml | 3.3E4 | 4.0E4 | 4.4E4 | 4.6E4 | 3.4E4 | 2.9E4 | 1.7E3 | 2.7E3 | 1.6E5 | 8.5E4 | 99 | 9 | 77 | 9 | 0.54 |
| yD | ng/ml | 1.5E-2 | 1.5E-2 | 1.5E-2 | 1.6E-2 | 6.3E-3 | 7.5E-3 | 1.0E-9 | 6.3E-3 | 3.2E-2 | 3.0E-2 | 99 | 9 | 76 | 9 | 0.53 |
| jB | ng/ml | 2.5E5 | 1.9E5 | 2.5E5 | 2.7E5 | 9.0E4 | 1.6E5 | 5.7E4 | 1.2E5 | 4.1E5 | 6.2E5 | 40 | 13 | 37 | 13 | 0.46 |
| wB | pg/ml | 8.3E3 | 1.6E4 | 9.6E3 | 1.7E4 | 7.4E3 | 9.6E3 | 1.7E3 | 5.3E3 | 4.1E4 | 3.0E4 | 99 | 9 | 76 | 9 | 0.73 |
| pY | pg/ml | 6.2E0 | 7.3E0 | 9.0E0 | 7.7E0 | 2.0E1 | 3.5E0 | 2.1E0 | 4.7E0 | 2.0E2 | 1.7E1 | 100 | 10 | 81 | 10 | 0.59 |
| rC | pg/ml | 1.5E3 | 1.4E3 | 2.2E3 | 1.6E3 | 2.3E3 | 9.6E2 | 1.0E-9 | 7.0E1 | 1.5E4 | 3.4E3 | 163 | 16 | 105 | 16 | 0.45 |
| rB | pg/ml | 2.4E1 | 5.3E1 | 4.7E1 | 6.4E1 | 9.5E1 | 6.4E1 | 1.0E-9 | 1.0E-9 | 9.5E2 | 2.1E2 | 163 | 16 | 105 | 16 | 0.60 |
| zG | 2.5ng/ml | 2.2E-1 | 5.3E-1 | 4.6E-1 | 6.9E-1 | 8.4E-1 | 8.6E-1 | 1.0E-9 | 1.0E-9 | 4.8E0 | 2.7E0 | 99 | 9 | 77 | 9 | 0.60 |
| zH | 2.3mU/ml | 1.1E-1 | 9.0E-2 | 1.1E-1 | 9.3E-2 | 5.4E-2 | 2.5E-2 | 1.0E-2 | 4.4E-2 | 4.4E-1 | 1.4E-1 | 99 | 9 | 77 | 9 | 0.39 |
| zI | 2.6ng/ml | 2.1E0 | 4.5E0 | 3.9E0 | 6.3E0 | 4.5E0 | 5.9E0 | 5.4E-1 | 7.8E-1 | 2.7E1 | 1.6E1 | 99 | 9 | 77 | 9 | 0.62 |
| qA | ng/ml | 9.9E6 | 1.3E7 | 1.2E7 | 1.6E7 | 7.9E6 | 8.7E6 | 2.0E6 | 4.3E6 | 4.6E7 | 3.0E7 | 100 | 10 | 81 | 10 | 0.65 |
| qB | ng/ml | 6.4E5 | 6.7E5 | 8.1E5 | 1.1E6 | 5.5E5 | 1.1E6 | 2.1E5 | 2.4E5 | 2.9E6 | 3.8E6 | 100 | 10 | 81 | 10 | 0.53 |
| qC | ng/ml | 3.8E5 | 2.3E5 | 7.0E5 | 2.4E5 | 1.0E6 | 1.7E5 | 3.4E3 | 2.1E4 | 7.1E6 | 5.2E5 | 100 | 10 | 81 | 10 | 0.29 |
| qD | ng/ml | 1.6E7 | 1.2E7 | 1.8E7 | 1.4E7 | 8.5E6 | 4.9E6 | 4.9E6 | 1.0E7 | 5.2E7 | 2.6E7 | 100 | 10 | 81 | 10 | 0.33 |
| jD | ng/ml | 3.6E1 | 4.0E1 | 5.2E1 | 5.0E1 | 6.3E1 | 5.0E1 | 1.0E-9 | 4.2E0 | 5.1E2 | 1.8E2 | 172 | 18 | 111 | 18 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| jE | ng/ml | 1.0E-9 | 1.0E-9 | 6.2E0 | 1.1E1 | 1.6E1 | 1.7E1 | 1.0E-9 | 1.0E-9 | 1.2E2 | 5.6E1 | 172 | 18 | 111 | 18 | 0.59 |
| jF | ng/ml | 3.1E1 | 3.1E1 | 5.0E1 | 4.0E1 | 6.1E1 | 4.5E1 | 1.0E-9 | 1.0E-9 | 3.5E2 | 1.3E2 | 172 | 18 | 111 | 18 | 0.46 |
| jG | ng/ml | 4.4E3 | 4.4E3 | 4.5E3 | 4.4E3 | 1.9E3 | 2.0E3 | 7.6E2 | 6.0E2 | 9.6E3 | 7.9E3 | 173 | 18 | 111 | 18 | 0.48 |
| jH | ng/ml | 7.7E1 | 7.6E1 | 8.6E1 | 9.8E1 | 4.8E1 | 8.7E1 | 1.3E1 | 3.3E1 | 3.3E2 | 4.3E2 | 173 | 18 | 111 | 18 | 0.50 |
| jI | ng/ml | 7.3E1 | 9.8E1 | 7.7E1 | 1.2E2 | 3.3E1 | 9.3E1 | 2.8E1 | 4.4E1 | 2.5E2 | 4.4E2 | 173 | 18 | 111 | 18 | 0.65 |
| sK | pg/mL | 4.0E3 | 4.7E3 | 4.1E3 | 6.7E3 | 1.7E3 | 6.5E3 | 1.1E3 | 2.1E3 | 1.0E4 | 2.3E4 | 100 | 9 | 78 | 9 | 0.62 |
| sM | pg/mL | 8.1E4 | 9.2E4 | 8.1E4 | 1.0E5 | 2.7E4 | 4.3E4 | 3.3E4 | 5.1E4 | 1.6E5 | 2.0E5 | 100 | 9 | 78 | 9 | 0.65 |
| sO | pg/mL | 2.7E8 | 2.5E8 | 2.7E8 | 2.3E8 | 9.9E7 | 1.2E8 | 4.9E7 | 6.6E7 | 5.0E8 | 4.0E8 | 100 | 9 | 78 | 9 | 0.42 |
| wC | ng/ml | 1.5E0 | 1.7E0 | 1.9E0 | 1.7E0 | 1.7E0 | 8.4E-1 | 2.5E-1 | 3.1E-1 | 1.5E1 | 2.9E0 | 99 | 9 | 76 | 9 | 0.52 |
| wD | ng/ml | 2.0E1 | 4.0E1 | 5.3E1 | 8.9E1 | 2.2E2 | 9.2E1 | 2.8E0 | 1.2E1 | 2.1E3 | 2.9E2 | 99 | 9 | 76 | 9 | 0.78 |
| wE | ng/ml | 4.8E1 | 4.5E1 | 5.0E1 | 5.1E1 | 2.3E1 | 1.7E1 | 3.2E0 | 3.6E1 | 1.4E2 | 8.9E1 | 99 | 9 | 76 | 9 | 0.50 |
| wG | ng/ml | 4.9E-2 | 5.9E-2 | 9.5E-2 | 1.5E-1 | 1.1E-1 | 2.3E-1 | 1.0E-9 | 1.0E-9 | 4.8E-1 | 6.8E-1 | 99 | 9 | 76 | 9 | 0.52 |
| wH | ng/ml | 2.0E-2 | 5.9E-2 | 1.8E-1 | 7.4E-1 | 5.7E-1 | 1.8E0 | 1.0E-9 | 1.0E-9 | 4.2E0 | 5.6E0 | 99 | 9 | 76 | 9 | 0.63 |
| wF | ng/ml | 1.8E-1 | 8.1E-1 | 1.8E0 | 3.2E0 | 7.6E0 | 3.9E0 | 1.0E-9 | 1.0E-9 | 5.7E1 | 9.6E0 | 99 | 9 | 76 | 9 | 0.64 |
| rA | pg/ml | 2.5E1 | 2.4E1 | 3.0E1 | 3.0E1 | 2.3E1 | 1.9E1 | 1.0E-9 | 6.9E0 | 1.2E2 | 6.8E1 | 171 | 17 | 108 | 17 | 0.52 |
| qZ | pg/ml | 4.3E1 | 9.9E1 | 4.3E2 | 1.2E3 | 1.9E3 | 2.8E3 | 1.0E-9 | 1.5E-3 | 1.0E4 | 1.0E4 | 132 | 13 | 96 | 13 | 0.67 |
| qY | pg/ml | 2.3E1 | 1.1E1 | 5.3E1 | 1.9E1 | 7.1E1 | 1.8E1 | 8.7E-1 | 2.1E0 | 5.3E2 | 7.8E1 | 171 | 17 | 108 | 17 | 0.33 |
| qX | pg/ml | 5.3E1 | 6.8E1 | 6.3E1 | 8.5E1 | 4.2E1 | 6.3E1 | 1.0E-9 | 1.7E1 | 2.1E2 | 2.1E2 | 171 | 17 | 108 | 17 | 0.58 |
| qW | pg/ml | 8.6E0 | 6.0E0 | 1.2E1 | 1.0E1 | 1.2E1 | 1.0E1 | 1.0E-9 | 1.0E-9 | 7.1E1 | 3.1E1 | 171 | 17 | 108 | 17 | 0.45 |
| qV | pg/ml | 2.1E3 | 1.7E3 | 2.8E3 | 2.5E3 | 2.1E3 | 2.3E3 | 1.0E2 | 6.3E2 | 1.1E4 | 9.6E3 | 171 | 17 | 108 | 17 | 0.45 |
| qU | pg/ml | 5.6E1 | 1.2E2 | 1.6E2 | 2.3E2 | 2.8E2 | 3.1E2 | 1.0E-9 | 3.5E0 | 2.1E3 | 1.1E3 | 171 | 17 | 108 | 17 | 0.57 |
| qT | pg/ml | 4.1E1 | 4.1E1 | 6.9E1 | 8.8E1 | 1.0E2 | 8.2E1 | 1.0E-9 | 6.9E0 | 9.0E2 | 3.0E2 | 171 | 17 | 108 | 17 | 0.59 |
| qI | ng/ml | 6.9E4 | 5.9E4 | 6.8E4 | 5.7E4 | 3.1E4 | 2.0E4 | 5.4E3 | 2.7E4 | 1.6E5 | 9.2E4 | 98 | 9 | 79 | 9 | 0.39 |
| qH | ng/ml | 6.2E4 | 5.5E4 | 6.9E4 | 7.1E4 | 4.1E4 | 3.5E4 | 7.6E3 | 3.5E4 | 1.8E5 | 1.4E5 | 98 | 9 | 79 | 9 | 0.54 |
| qG | ng/ml | 1.9E5 | 1.7E5 | 2.0E5 | 1.8E5 | 7.3E4 | 6.1E4 | 1.7E4 | 9.9E4 | 4.2E5 | 2.7E5 | 98 | 9 | 79 | 9 | 0.41 |
| jK | ng/ml | 1.6E3 | 1.3E3 | 1.7E3 | 1.5E3 | 6.6E2 | 6.3E2 | 2.8E2 | 7.5E2 | 4.3E3 | 2.9E3 | 173 | 18 | 111 | 18 | 0.37 |
| jL | ng/ml | 2.0E2 | 2.5E2 | 3.0E2 | 3.7E2 | 2.6E2 | 3.7E2 | 3.6E1 | 1.2E2 | 2.1E3 | 1.7E3 | 173 | 18 | 111 | 18 | 0.60 |
| jM | ng/ml | 7.0E4 | 6.2E4 | 7.3E4 | 7.0E4 | 3.7E4 | 4.3E4 | 3.9E2 | 5.7E3 | 1.7E5 | 1.4E5 | 173 | 18 | 111 | 18 | 0.47 |
| jO | pg/ml | 2.2E5 | 2.3E5 | 2.7E5 | 2.4E5 | 1.5E5 | 1.1E5 | 5.2E4 | 9.8E4 | 8.0E5 | 5.2E5 | 173 | 18 | 111 | 18 | 0.48 |
| jP | pg/ml | 2.4E5 | 2.6E5 | 2.7E5 | 3.4E5 | 1.6E5 | 2.7E5 | 5.8E4 | 8.4E4 | 9.2E5 | 1.2E6 | 173 | 18 | 111 | 18 | 0.56 |
| jQ | pg/ml | 2.5E3 | 1.9E3 | 3.4E3 | 2.5E3 | 3.4E3 | 2.3E3 | 1.0E-9 | 4.6E2 | 1.8E4 | 9.2E3 | 173 | 18 | 111 | 18 | 0.43 |
| jR | pg/ml | 5.9E3 | 3.5E3 | 1.0E4 | 8.9E3 | 1.2E4 | 1.2E4 | 1.0E-9 | 3.0E1 | 9.0E4 | 4.6E4 | 173 | 18 | 111 | 18 | 0.42 |
| jT | pg/ml | 1.7E5 | 1.6E5 | 1.8E5 | 1.5E5 | 6.8E4 | 5.4E4 | 6.8E4 | 7.5E4 | 4.5E5 | 2.8E5 | 173 | 18 | 111 | 18 | 0.39 |
| xA | pg/ml | 4.7E0 | 7.3E0 | 1.5E1 | 1.9E1 | 4.5E1 | 3.4E1 | 1.0E-9 | 1.0E-9 | 3.9E2 | 1.1E2 | 99 | 9 | 77 | 9 | 0.58 |
| yE | pg/ml | 7.8E1 | 7.9E1 | 8.5E1 | 1.1E2 | 4.3E1 | 7.3E1 | 6.4E0 | 1.6E1 | 3.0E2 | 2.5E2 | 99 | 9 | 77 | 9 | 0.58 |
| tM | pg/ml | 4.0E1 | 4.4E1 | 4.1E1 | 5.1E1 | 1.8E1 | 3.1E1 | 1.0E-9 | 1.6E1 | 1.0E2 | 1.1E2 | 99 | 9 | 77 | 9 | 0.56 |
| tL | pg/ml | 1.0E-9 | 1.0E-9 | 4.0E0 | 3.0E0 | 2.7E1 | 8.7E0 | 1.0E-9 | 1.0E-9 | 2.6E2 | 2.6E1 | 99 | 9 | 77 | 9 | 0.53 |
| jU | mIU/ml | 4.6E0 | 4.8E0 | 1.0E1 | 1.0E1 | 1.7E1 | 1.3E1 | 6.2E-2 | 4.2E-2 | 1.1E2 | 5.3E1 | 173 | 18 | 111 | 18 | 0.51 |
| jV | mIU/ml | 1.5E0 | 1.1E0 | 3.4E0 | 2.4E0 | 5.8E0 | 2.8E0 | 1.7E-3 | 7.6E-2 | 3.3E1 | 1.0E1 | 173 | 18 | 111 | 18 | 0.48 |
| jY | ng/ml | 7.4E-4 | 4.0E-3 | 6.5E-3 | 7.5E-3 | 2.9E-2 | 8.3E-3 | 1.0E-9 | 1.0E-9 | 3.0E-1 | 2.6E-2 | 173 | 18 | 111 | 18 | 0.65 |
| kC | pg/ml | 9.6E1 | 9.7E1 | 1.9E2 | 1.4E2 | 4.1E2 | 1.4E2 | 2.1E1 | 3.6E1 | 3.5E3 | 5.9E2 | 113 | 21 | 88 | 21 | 0.48 |
| kE | pg/ml | 1.4E5 | 1.4E5 | 1.4E5 | 1.4E5 | 3.9E4 | 5.0E4 | 1.2E4 | 5.1E4 | 2.3E5 | 2.7E5 | 113 | 21 | 88 | 21 | 0.49 |
| kF | pg/mL | 6.3E1 | 6.5E1 | 7.2E1 | 7.0E1 | 5.2E1 | 2.3E1 | 2.6E1 | 3.4E1 | 5.1E2 | 1.2E2 | 113 | 21 | 88 | 21 | 0.55 |
| kG | pg/mL | 9.2E3 | 9.2E3 | 1.4E4 | 1.4E4 | 1.7E4 | 1.2E4 | 7.5E2 | 1.1E3 | 1.2E5 | 5.0E4 | 113 | 21 | 88 | 21 | 0.54 |
| kI | pg/ml | 2.0E2 | 1.8E2 | 2.2E2 | 2.0E2 | 1.3E2 | 1.2E2 | 4.4E1 | 1.0E-9 | 8.7E2 | 6.2E2 | 113 | 21 | 88 | 21 | 0.45 |
| kK | pg/ml | 1.0E2 | 1.4E2 | 1.8E2 | 1.9E2 | 2.3E2 | 1.6E2 | 2.1E1 | 2.9E1 | 1.6E3 | 6.2E2 | 113 | 21 | 88 | 21 | 0.56 |
| kN | pg/ml | 1.0E3 | 8.9E2 | 2.1E3 | 3.2E3 | 5.6E3 | 8.4E3 | 7.6E1 | 3.8E2 | 5.5E4 | 3.9E4 | 113 | 21 | 88 | 21 | 0.51 |
| kO | pg/ml | 7.1E3 | 7.1E3 | 8.7E3 | 7.9E3 | 1.2E4 | 3.4E3 | 3.4E3 | 4.2E3 | 1.3E5 | 1.9E4 | 113 | 21 | 88 | 21 | 0.50 |
| kP | pg/ml | 6.4E3 | 5.4E3 | 7.5E3 | 6.5E3 | 6.3E3 | 4.3E3 | 8.6E2 | 1.4E3 | 4.8E4 | 1.7E4 | 113 | 21 | 88 | 21 | 0.45 |
| kQ | pg/ml | 4.3E3 | 4.3E3 | 5.0E3 | 5.4E3 | 2.9E3 | 4.0E3 | 5.6E2 | 1.4E3 | 2.5E4 | 2.2E4 | 203 | 26 | 135 | 26 | 0.51 |
| kR | pg/ml | 2.3E1 | 1.8E1 | 3.3E1 | 2.7E1 | 7.3E1 | 2.6E1 | 1.0E-9 | 5.5E0 | 1.0E3 | 1.1E2 | 203 | 26 | 135 | 26 | 0.43 |
| kS | pg/ml | 8.0E2 | 9.0E2 | 9.6E2 | 9.9E2 | 1.1E3 | 6.6E2 | 8.2E1 | 2.9E2 | 1.4E4 | 3.0E3 | 203 | 26 | 135 | 26 | 0.53 |
| pS | ng/ml | 1.9E5 | 1.4E5 | 2.2E5 | 1.6E5 | 1.1E5 | 9.2E4 | 7.5E4 | 6.0E4 | 8.3E5 | 3.6E5 | 100 | 9 | 78 | 9 | 0.30 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| rZ | ng/ml | 6.0E-4 | 4.0E-3 | 6.3E-3 | 1.6E-2 | 1.6E-2 | 2.8E-2 | 1.0E-9 | 1.0E-9 | 1.1E-1 | 1.1E-1 | 165 | 16 | 104 | 16 | 0.59 |
| rY | ng/ml | 6.1E-2 | 6.5E-2 | 4.1E-1 | 7.9E-2 | 2.4E0 | 5.3E-2 | 1.0E-9 | 1.0E-9 | 2.3E1 | 2.1E-1 | 165 | 16 | 104 | 16 | 0.54 |
| rX | ng/ml | 1.0E-9 | 1.0E-9 | 7.9E-2 | 1.4E-2 | 4.5E-1 | 3.2E-2 | 1.0E-9 | 1.0E-9 | 3.7E0 | 1.2E-1 | 165 | 16 | 104 | 16 | 0.60 |
| lK | pg/ml | 8.1E1 | 5.2E1 | 1.8E2 | 1.2E2 | 3.2E2 | 1.7E2 | 1.0E-9 | 2.3E0 | 3.3E3 | 5.6E2 | 172 | 18 | 111 | 18 | 0.44 |
| lL | pg/ml | 1.6E3 | 1.8E3 | 2.7E3 | 2.3E3 | 4.0E3 | 1.9E3 | 1.5E1 | 1.9E2 | 4.2E4 | 6.8E3 | 173 | 18 | 111 | 18 | 0.52 |
| lM | pg/ml | 1.1E3 | 2.9E3 | 4.1E3 | 7.6E3 | 8.6E3 | 1.2E4 | 3.9E1 | 2.4E1 | 5.1E4 | 4.0E4 | 173 | 18 | 111 | 18 | 0.62 |
| lN | pg/ml | 1.0E-9 | 3.8E0 | 4.5E0 | 5.4E0 | 1.6E1 | 9.0E0 | 1.0E-9 | 1.0E-9 | 1.5E2 | 3.5E1 | 173 | 18 | 111 | 18 | 0.60 |
| lO | pg/ml | 1.0E-9 | 1.0E-9 | 1.8E0 | 9.5E0 | 1.2E1 | 3.3E1 | 1.0E-9 | 1.0E-9 | 1.3E2 | 1.4E2 | 172 | 18 | 111 | 18 | 0.54 |
| zA | ng/ml | 1.9E7 | 1.8E7 | 2.0E7 | 1.7E7 | 6.6E6 | 5.9E6 | 5.9E6 | 7.5E6 | 3.6E7 | 2.5E7 | 95 | 9 | 72 | 9 | 0.38 |
| rW | ng/ml | 1.3E-2 | 1.5E-2 | 3.5E-2 | 1.8E-2 | 5.4E-2 | 1.1E-2 | 1.0E-9 | 7.4E-3 | 3.2E-1 | 4.3E-2 | 98 | 9 | 78 | 9 | 0.54 |
| rV | ng/ml | 1.0E-9 | 1.0E-9 | 1.8E-2 | 1.2E-2 | 6.1E-2 | 2.1E-2 | 1.0E-9 | 1.0E-9 | 2.8E-1 | 5.6E-2 | 98 | 9 | 78 | 9 | 0.57 |
| rU | ng/ml | 7.6E-2 | 7.6E-2 | 2.7E-1 | 1.3E-1 | 6.8E-1 | 1.2E-1 | 1.0E-9 | 1.0E-9 | 4.4E0 | 4.0E-1 | 98 | 9 | 78 | 9 | 0.56 |
| rT | ng/ml | 5.4E0 | 6.9E0 | 6.1E0 | 7.8E0 | 4.0E0 | 4.3E0 | 8.9E-2 | 1.0E0 | 2.1E1 | 1.3E1 | 98 | 9 | 78 | 9 | 0.64 |
| rS | ng/ml | 3.6E0 | 5.2E0 | 5.3E0 | 1.4E1 | 5.3E0 | 1.9E1 | 3.9E-1 | 2.0E0 | 3.8E1 | 5.9E1 | 98 | 9 | 78 | 9 | 0.66 |
| sC | pg/mL | 7.4E3 | 6.2E3 | 1.3E4 | 9.1E3 | 1.5E4 | 7.7E3 | 1.7E3 | 3.4E3 | 8.0E4 | 2.8E4 | 100 | 9 | 78 | 9 | 0.48 |
| yL | pg/ml | 3.1E1 | 4.1E1 | 3.5E1 | 5.7E1 | 2.4E1 | 5.5E1 | 5.6E0 | 1.6E1 | 1.8E2 | 1.9E2 | 98 | 9 | 76 | 9 | 0.63 |
| rP | ng/ml | 1.1E2 | 7.4E1 | 3.1E2 | 2.5E2 | 9.9E2 | 2.4E2 | 1.0E-9 | 1.2E1 | 9.7E3 | 5.0E2 | 98 | 9 | 78 | 9 | 0.52 |
| rQ | ng/ml | 1.0E-9 | 1.0E-9 | 7.5E0 | 1.9E1 | 2.9E1 | 5.6E1 | 1.0E-9 | 1.0E-9 | 1.7E2 | 1.7E2 | 98 | 9 | 78 | 9 | 0.50 |
| rO | ng/ml | 3.0E-2 | 1.5E-2 | 6.2E-2 | 3.9E-2 | 1.0E-1 | 5.5E-2 | 1.0E-9 | 1.0E-9 | 4.9E-1 | 1.4E-1 | 98 | 9 | 78 | 9 | 0.43 |
| rR | ng/ml | 3.9E0 | 4.0E0 | 1.8E1 | 8.8E0 | 4.9E1 | 1.3E1 | 1.0E-9 | 1.0E-9 | 4.5E2 | 3.9E1 | 98 | 9 | 78 | 9 | 0.47 |
| rN | ng/ml | 6.0E-1 | 7.9E-1 | 7.2E-1 | 2.4E0 | 5.1E-1 | 4.1E0 | 5.1E-2 | 3.1E-1 | 3.0E0 | 1.3E1 | 98 | 9 | 78 | 9 | 0.63 |
| qO | pg/ml | 1.0E4 | 1.1E4 | 1.4E4 | 1.2E4 | 1.2E4 | 6.2E3 | 7.4E2 | 5.4E3 | 6.4E4 | 2.3E4 | 100 | 9 | 79 | 9 | 0.53 |
| qP | pg/ml | 3.6E2 | 3.2E2 | 4.7E2 | 3.9E2 | 3.8E2 | 2.0E2 | 1.0E-9 | 1.5E2 | 2.2E3 | 7.5E2 | 100 | 9 | 79 | 9 | 0.47 |
| qQ | pg/ml | 1.5E1 | 1.5E1 | 1.7E1 | 2.1E1 | 3.9E1 | 2.0E1 | 1.0E-9 | 1.0E-9 | 2.8E2 | 4.3E1 | 100 | 9 | 79 | 9 | 0.61 |
| nW | pg/ml | 1.1E5 | 1.1E5 | 1.1E5 | 1.1E5 | 2.5E4 | 3.4E4 | 5.8E4 | 3.6E4 | 1.8E5 | 1.9E5 | 203 | 26 | 135 | 26 | 0.47 |
| nY | pg/ml | 2.1E3 | 2.1E3 | 2.4E3 | 2.7E3 | 1.6E3 | 1.7E3 | 5.1E2 | 6.3E2 | 1.3E4 | 8.1E3 | 203 | 26 | 135 | 26 | 0.54 |
| oO | pg/ml | 8.6E4 | 9.6E4 | 1.2E5 | 1.0E5 | 1.0E5 | 5.2E4 | 2.6E4 | 3.3E3 | 6.2E5 | 2.1E5 | 105 | 20 | 82 | 20 | 0.55 |
| oP | pg/ml | 1.4E5 | 1.4E5 | 1.5E5 | 1.5E5 | 9.3E4 | 9.3E4 | 4.2E4 | 2.4E4 | 4.5E5 | 4.6E5 | 105 | 20 | 82 | 20 | 0.51 |
| oQ | pg/ml | 3.0E3 | 3.2E3 | 3.9E3 | 4.0E3 | 3.0E3 | 2.4E3 | 1.1E3 | 8.7E2 | 2.1E4 | 9.6E3 | 105 | 20 | 82 | 20 | 0.54 |
| oE | pg/ml | 1.5E2 | 6.2E2 | 3.8E2 | 9.0E2 | 5.5E2 | 9.0E2 | 1.0E-9 | 1.0E-9 | 4.7E3 | 3.4E3 | 203 | 26 | 135 | 26 | 0.69 |
| oF | pg/ml | 8.5E3 | 1.9E4 | 2.2E4 | 4.4E4 | 3.6E4 | 5.4E4 | 6.4E1 | 1.3E2 | 2.5E5 | 1.8E5 | 203 | 26 | 135 | 26 | 0.66 |
| oH | pg/ml | 4.1E1 | 3.5E1 | 9.4E1 | 4.9E1 | 1.4E2 | 4.8E1 | 4.2E0 | 7.3E0 | 9.9E2 | 1.8E2 | 203 | 26 | 135 | 26 | 0.40 |
| oK | pg/ml | 7.6E2 | 1.1E3 | 1.9E3 | 3.7E3 | 2.6E3 | 1.0E4 | 5.2E1 | 2.3E2 | 1.8E4 | 5.3E4 | 203 | 26 | 135 | 26 | 0.58 |
| oN | pg/ml | 5.1E2 | 5.1E2 | 7.9E2 | 8.0E2 | 1.5E3 | 9.5E2 | 1.5E2 | 1.1E2 | 1.8E4 | 5.0E3 | 203 | 26 | 135 | 26 | 0.53 |
| oW | pg/ml | 2.1E2 | 3.5E2 | 4.2E2 | 5.7E2 | 9.3E2 | 7.8E2 | 7.7E1 | 9.5E1 | 6.0E3 | 2.7E3 | 40 | 13 | 37 | 13 | 0.55 |
| oT | pg/ml | 3.2E2 | 3.0E2 | 3.7E2 | 3.4E2 | 2.0E2 | 1.2E2 | 9.9E1 | 1.4E2 | 7.9E2 | 5.4E2 | 40 | 13 | 37 | 13 | 0.49 |
| oV | pg/ml | 1.2E2 | 8.1E1 | 2.7E2 | 1.4E2 | 4.3E2 | 1.6E2 | 1.0E-9 | 1.1E1 | 2.2E3 | 6.3E2 | 40 | 13 | 37 | 13 | 0.41 |
| oD | pg/ml | 1.6E4 | 1.6E4 | 1.7E4 | 1.7E4 | 7.5E3 | 5.8E3 | 6.6E3 | 1.1E4 | 4.6E4 | 3.3E4 | 40 | 13 | 37 | 13 | 0.49 |
| uL | ng/ml | 3.8E1 | 4.3E1 | 5.5E1 | 4.5E1 | 7.2E1 | 2.4E1 | 1.0E-9 | 2.1E1 | 4.0E2 | 9.2E1 | 99 | 9 | 77 | 9 | 0.54 |
| uO | ng/ml | 4.0E-1 | 4.5E-1 | 9.0E-1 | 6.5E-1 | 1.4E0 | 7.4E-1 | 1.0E-9 | 1.0E-9 | 9.3E0 | 1.7E0 | 99 | 9 | 77 | 9 | 0.46 |
| uM | ng/ml | 5.9E-1 | 3.2E-1 | 9.4E-1 | 6.6E-1 | 1.7E0 | 8.6E-1 | 1.0E-9 | 1.0E-9 | 1.3E1 | 2.5E0 | 99 | 9 | 77 | 9 | 0.40 |
| uI | ng/ml | 7.3E-2 | 5.9E-2 | 1.2E-1 | 8.2E-2 | 1.6E-1 | 5.0E-2 | 1.6E-2 | 3.4E-2 | 1.1E0 | 1.8E-1 | 98 | 9 | 76 | 9 | 0.47 |
| uN | ng/ml | 1.5E1 | 1.9E1 | 1.7E1 | 2.0E1 | 6.9E0 | 8.2E0 | 6.3E0 | 9.2E0 | 4.1E1 | 3.3E1 | 99 | 9 | 77 | 9 | 0.63 |
| uG | ng/ml | 1.9E1 | 2.2E1 | 2.4E1 | 3.7E1 | 1.6E1 | 3.9E1 | 1.2E0 | 6.5E0 | 8.9E1 | 1.3E2 | 99 | 9 | 77 | 9 | 0.59 |
| uR | ng/ml | 2.5E0 | 3.3E0 | 3.6E0 | 3.4E0 | 6.5E0 | 2.0E0 | 7.3E-1 | 1.3E0 | 6.4E1 | 6.6E0 | 99 | 9 | 77 | 9 | 0.55 |
| uP | ng/ml | 2.2E0 | 2.5E0 | 2.6E0 | 2.8E0 | 1.3E0 | 1.3E0 | 1.1E0 | 9.3E-1 | 9.1E0 | 4.8E0 | 99 | 9 | 77 | 9 | 0.59 |
| uV | ng/ml | 1.1E-3 | 1.3E-3 | 1.3E-2 | 4.1E-3 | 2.6E-2 | 5.9E-3 | 1.0E-9 | 1.0E-9 | 2.0E-1 | 1.8E-2 | 99 | 9 | 77 | 9 | 0.44 |
| uT | ng/ml | 6.4E1 | 1.1E2 | 8.8E1 | 1.5E2 | 8.4E1 | 1.2E2 | 1.2E1 | 4.7E1 | 5.8E2 | 4.1E2 | 99 | 9 | 77 | 9 | 0.71 |
| uU | ng/ml | 1.7E0 | 2.7E0 | 2.0E0 | 4.0E0 | 1.2E0 | 6.0E0 | 5.9E-1 | 5.4E-1 | 7.0E0 | 2.0E1 | 99 | 9 | 77 | 9 | 0.55 |
| uW | ng/ml | 7.6E0 | 8.1E0 | 8.1E0 | 8.0E0 | 2.9E0 | 2.4E0 | 3.5E0 | 3.1E0 | 2.2E1 | 1.2E1 | 99 | 9 | 77 | 9 | 0.56 |
| vB | ng/ml | 2.9E0 | 3.2E0 | 3.1E0 | 3.4E0 | 1.7E0 | 1.6E0 | 5.9E-1 | 1.3E0 | 1.0E1 | 6.6E0 | 99 | 9 | 77 | 9 | 0.55 |
| vC | ng/ml | 1.0E-9 | 1.0E-9 | 4.1E-3 | 1.0E-9 | 2.5E-2 | 0.0E0 | 1.0E-9 | 1.0E-9 | 2.0E-1 | 1.0E-9 | 99 | 9 | 77 | 9 | 0.47 |
| uY | ng/ml | 6.9E-1 | 7.5E-1 | 1.2E0 | 1.3E0 | 1.0E0 | 1.4E0 | 6.8E-2 | 1.8E-1 | 4.9E0 | 4.4E0 | 99 | 9 | 77 | 9 | 0.47 |

| Code | Units | Median | | Average | | Standard Deviation | | Minimum | | Maximum | | Number of Samples | | Number of Patients | | AUC |
|------|-------|--------|-----|---------|-----|--------|-----|--------|-----|--------|-----|--------|-----|--------|-----|-----|
|      |       | NonDis | Dis | NonDis | Dis | NonDis | Dis | NonDis | Dis | NonDis | Dis | NonDis | Dis | NonDis | Dis |     |
| uZ | ng/ml | 5.4E-1 | 5.9E-1 | 7.6E-1 | 7.9E-1 | 9.1E-1 | 6.9E-1 | 1.0E-1 | 1.0E-1 | 7.2E0 | 2.0E0 | 99 | 9 | 77 | 9 | 0.52 |
| uX | ng/ml | 1.2E1 | 1.7E1 | 1.3E1 | 3.0E1 | 7.6E0 | 2.9E1 | 3.6E0 | 4.0E0 | 4.4E1 | 7.8E1 | 99 | 9 | 77 | 9 | 0.63 |
| vA | ng/ml | 7.2E-2 | 7.3E-2 | 8.5E-2 | 1.0E-1 | 5.4E-2 | 1.2E-1 | 1.7E-2 | 2.5E-2 | 3.0E-1 | 4.2E-1 | 99 | 9 | 77 | 9 | 0.47 |
| vH | ng/ml | 1.2E-1 | 1.1E-1 | 1.7E-1 | 3.5E-1 | 1.6E-1 | 5.9E-1 | 9.9E-3 | 4.7E-2 | 9.6E-1 | 1.9E0 | 100 | 9 | 78 | 9 | 0.53 |
| vI | ng/ml | 1.7E0 | 2.9E0 | 2.1E0 | 3.0E0 | 1.9E0 | 3.1E0 | 4.2E-3 | 1.7E-3 | 1.0E1 | 1.0E1 | 100 | 9 | 78 | 9 | 0.59 |
| vP | ng/ml | 4.2E2 | 3.1E2 | 5.1E2 | 4.7E2 | 4.5E2 | 4.5E2 | 4.0E1 | 1.5E2 | 2.4E3 | 1.6E3 | 99 | 9 | 77 | 9 | 0.49 |
| vT | ng/ml | 8.2E1 | 7.7E1 | 1.1E2 | 8.9E1 | 1.0E2 | 4.1E1 | 2.4E1 | 4.6E1 | 6.9E2 | 1.6E2 | 99 | 9 | 77 | 9 | 0.49 |
| vU | ng/ml | 1.0E-9 | 1.0E-9 | 2.1E1 | 2.5E1 | 3.1E1 | 4.5E1 | 1.0E-9 | 1.0E-9 | 1.2E2 | 1.1E2 | 99 | 9 | 77 | 9 | 0.49 |
| vQ | ng/ml | 3.5E2 | 3.2E2 | 3.7E2 | 3.7E2 | 1.4E2 | 1.7E2 | 6.7E1 | 1.9E2 | 8.4E2 | 6.0E2 | 99 | 9 | 77 | 9 | 0.50 |
| vO | ng/ml | 1.8E3 | 2.0E3 | 1.8E3 | 2.1E3 | 4.4E2 | 6.9E2 | 1.0E3 | 1.0E3 | 3.0E3 | 3.2E3 | 99 | 9 | 77 | 9 | 0.61 |
| vS | ng/ml | 1.3E3 | 1.4E3 | 1.2E3 | 1.5E3 | 4.7E2 | 3.1E2 | 1.0E-9 | 1.1E3 | 2.1E3 | 1.9E3 | 99 | 9 | 77 | 9 | 0.70 |
| vV | ng/ml | 9.0E2 | 9.4E2 | 1.4E3 | 2.4E4 | 1.7E3 | 7.0E4 | 2.1E1 | 2.3E2 | 1.1E4 | 2.1E5 | 99 | 9 | 77 | 9 | 0.50 |
| vW | ng/ml | 1.4E2 | 1.6E2 | 1.7E2 | 2.7E2 | 1.2E2 | 2.3E2 | 4.3E1 | 6.0E1 | 6.7E2 | 7.7E2 | 99 | 9 | 77 | 9 | 0.60 |
| pF | pg/ml | 4.8E-1 | 7.5E-1 | 1.1E0 | 1.1E0 | 6.1E0 | 9.4E-1 | 1.0E-9 | 1.8E-1 | 8.7E1 | 4.4E0 | 203 | 26 | 135 | 26 | 0.66 |
| pH | ng/ml | 7.9E0 | 1.2E1 | 9.5E0 | 1.3E1 | 6.6E0 | 1.1E1 | 3.0E0 | 3.3E0 | 4.2E1 | 4.7E1 | 40 | 13 | 37 | 13 | 0.62 |
| pI | ng/ml | 7.0E1 | 6.0E1 | 7.6E1 | 6.6E1 | 4.1E1 | 2.7E1 | 2.6E1 | 2.5E1 | 2.0E2 | 1.2E2 | 40 | 13 | 37 | 13 | 0.44 |
| pK | ng/ml | 4.9E-1 | 4.2E-1 | 5.3E-1 | 5.3E-1 | 2.7E-1 | 3.2E-1 | 1.7E-1 | 1.6E-1 | 1.6E0 | 1.4E0 | 40 | 13 | 37 | 13 | 0.48 |

Figure 4.

Descriptive statistics for the assays used to form the panels presented in this figure.

| Code | Units | Median | | Average | | Standard Deviation | | Minimum | | Maximum | | Number of Samples | | Number of Patients | | AUC |
|------|-------|--------|-----|---------|-----|-----|-----|-----|-----|-----|-----|-----|-----|-----|-----|-----|
| | | NonDis | Dis | NonDis | Dis | NonDis | Dis | NonDis | Dis | NonDis | Dis | NonDis | Dis | NonDis | Dis | |
| Aa | mg/dL | 6.4E1 | 6.4E1 | 7.5E1 | 8.0E1 | 5.1E1 | 5.6E1 | 2.0E0 | 2.0E0 | 4.4E2 | 2.6E2 | 965 | 67 | 171 | 67 | 0.52 |
| Ad | ug/mL | 2.5E-2 | 6.9E-2 | 5.8E-2 | 2.4E-1 | 7.7E-2 | 1.1E0 | 6.8E-4 | 2.6E-3 | 3.7E-1 | 8.5E0 | 255 | 57 | 103 | 57 | 0.67 |
| Af | ng/mL | 9.2E-1 | 1.6E0 | 1.8E1 | 1.9E1 | 7.2E1 | 5.1E1 | 1.7E-3 | 1.7E-3 | 5.3E2 | 2.6E2 | 255 | 57 | 103 | 57 | 0.59 |
| Aj | ug/mL | 1.4E0 | 2.6E0 | 2.6E0 | 2.8E0 | 2.4E0 | 2.5E0 | 1.5E-3 | 2.1E-3 | 6.1E0 | 6.1E0 | 255 | 57 | 103 | 57 | 0.51 |
| Al | mg/mL | 9.0E-5 | 7.0E-5 | 2.5E-4 | 2.5E-4 | 4.2E-4 | 3.9E-4 | 2.5E-6 | 6.6E-6 | 1.9E-3 | 1.7E-3 | 255 | 57 | 103 | 57 | 0.51 |
| An | U/mL | 4.0E1 | 6.5E1 | 1.5E2 | 3.4E2 | 4.7E2 | 1.1E3 | 9.8E-4 | 9.0E-1 | 5.5E3 | 7.8E3 | 255 | 57 | 103 | 57 | 0.61 |
| Ao | pg/mL | 8.5E1 | 1.1E2 | 2.1E2 | 9.8E2 | 1.0E3 | 5.1E3 | 1.5E0 | 4.1E0 | 1.6E4 | 3.8E4 | 255 | 57 | 103 | 57 | 0.56 |
| Ap | ng/mL | 2.7E1 | 4.3E1 | 4.3E1 | 6.0E1 | 5.0E1 | 6.2E1 | 9.9E-1 | 2.7E0 | 3.3E2 | 2.9E2 | 255 | 57 | 103 | 57 | 0.61 |
| Ar | ng/mL | 6.5E-1 | 1.3E0 | 2.0E0 | 5.0E0 | 4.1E0 | 1.1E1 | 3.4E-3 | 4.1E-2 | 4.3E1 | 5.4E1 | 255 | 57 | 103 | 57 | 0.62 |
| As | ng/mL | 8.7E-3 | 1.1E-2 | 1.2E-2 | 3.4E-2 | 1.6E-2 | 1.6E-1 | 1.7E-3 | 1.7E-3 | 1.1E-1 | 1.2E0 | 255 | 57 | 103 | 57 | 0.56 |
| Aw | pg/mL | 1.6E1 | 1.8E1 | 1.6E1 | 2.0E1 | 5.4E0 | 7.9E0 | 3.5E0 | 1.1E1 | 3.3E1 | 5.1E1 | 255 | 57 | 103 | 57 | 0.66 |
| Ax | ng/mL | 2.1E0 | 2.6E0 | 8.2E0 | 2.6E1 | 1.8E1 | 1.1E2 | 1.9E-2 | 4.1E-2 | 1.5E2 | 7.7E2 | 255 | 57 | 103 | 57 | 0.53 |
| Ba | ng/mL | 3.7E1 | 1.4E2 | 2.8E2 | 9.8E2 | 7.3E2 | 2.5E3 | 3.7E-1 | 1.1E0 | 8.1E3 | 1.5E4 | 255 | 57 | 103 | 57 | 0.64 |
| Bb | ng/mL | 2.6E0 | 5.1E0 | 5.3E0 | 8.7E0 | 8.5E0 | 1.1E1 | 4.1E-3 | 4.1E-3 | 6.6E1 | 6.4E1 | 255 | 57 | 103 | 57 | 0.65 |
| Bc | ng/mL | 3.3E1 | 5.0E1 | 9.7E1 | 1.7E2 | 1.8E2 | 2.9E2 | 1.1E-1 | 2.9E-1 | 1.0E3 | 1.2E3 | 255 | 57 | 103 | 57 | 0.58 |
| Bg | ng/mL | 6.4E-2 | 2.1E-1 | 4.4E0 | 7.8E0 | 2.2E1 | 2.7E1 | 5.3E-4 | 5.3E-4 | 2.5E2 | 1.5E2 | 255 | 57 | 103 | 57 | 0.62 |
| Bn | ng/mL | 5.6E-2 | 5.6E-2 | 8.5E-1 | 2.9E0 | 1.6E0 | 7.9E0 | 5.6E-2 | 5.6E-2 | 8.5E0 | 5.8E1 | 255 | 57 | 103 | 57 | 0.59 |
| Bo | ng/mL | 1.1E1 | 1.5E1 | 1.3E1 | 2.2E1 | 9.6E0 | 3.7E1 | 1.6E-2 | 1.6E-2 | 7.4E1 | 2.8E2 | 255 | 57 | 103 | 57 | 0.60 |
| Ch | uIU/mL | 9.0E-1 | 1.4E0 | 6.5E0 | 3.3E1 | 2.1E1 | 1.0E2 | 3.4E-3 | 3.9E-2 | 2.3E2 | 5.3E2 | 255 | 57 | 103 | 57 | 0.61 |
| Co | pg/mL | 3.1E1 | 4.9E1 | 9.1E1 | 1.8E2 | 2.1E2 | 5.9E2 | 3.9E0 | 1.5E-1 | 1.9E3 | 4.4E3 | 255 | 57 | 103 | 57 | 0.63 |
| Cp | ng/mL | 1.9E1 | 2.6E1 | 2.5E1 | 6.3E1 | 2.5E1 | 1.7E2 | 6.0E-1 | 6.0E-1 | 2.9E2 | 1.3E3 | 255 | 57 | 103 | 57 | 0.68 |
| Cq | ng/mL | 2.4E-2 | 3.4E-2 | 1.6E-1 | 9.5E-1 | 1.1E0 | 6.5E0 | 8.0E-4 | 8.0E-4 | 1.7E1 | 4.9E1 | 255 | 57 | 103 | 57 | 0.56 |
| Cs | ng/mL | 4.5E1 | 9.6E1 | 2.1E2 | 4.7E2 | 4.0E2 | 1.5E3 | 2.7E-2 | 8.9E-1 | 2.9E3 | 1.1E4 | 255 | 57 | 103 | 57 | 0.57 |
| Ct | ng/mL | 1.2E0 | 4.3E-1 | 2.6E1 | 6.3E1 | 8.2E1 | 1.4E2 | 1.1E-4 | 2.8E-2 | 6.2E2 | 6.2E2 | 255 | 57 | 103 | 57 | 0.49 |
| Cu | ng/mL | 2.1E-1 | 3.4E-1 | 4.2E-1 | 1.6E0 | 9.2E-1 | 8.7E0 | 9.6E-3 | 1.5E-2 | 9.2E0 | 6.6E1 | 255 | 57 | 103 | 57 | 0.63 |
| Cv | ng/mL | 4.6E0 | 8.0E0 | 2.4E1 | 4.2E1 | 7.0E1 | 1.0E2 | 5.6E-3 | 2.4E-2 | 5.3E2 | 5.2E2 | 255 | 57 | 103 | 57 | 0.54 |
| Cw | mIU/mL | 2.8E-2 | 4.3E-2 | 3.8E-2 | 1.7E-1 | 3.0E-2 | 8.9E-1 | 2.3E-3 | 4.7E-3 | 1.9E-1 | 6.8E0 | 255 | 57 | 103 | 57 | 0.61 |
| Cx | ng/mL | 1.8E-1 | 2.0E0 | 5.4E1 | 8.4E1 | 1.0E2 | 1.3E2 | 9.3E-5 | 9.3E-5 | 3.9E2 | 3.9E2 | 255 | 57 | 103 | 57 | 0.55 |
| Db | ug/mL | 8.2E0 | 7.5E0 | 9.4E0 | 9.4E0 | 8.8E0 | 7.9E0 | 5.3E-1 | 8.7E-1 | 1.0E2 | 4.1E1 | 255 | 57 | 103 | 57 | 0.49 |
| Dc | nmol/L | 1.8E-2 | 2.5E-2 | 5.7E-2 | 3.3E-1 | 1.3E-1 | 1.9E0 | 5.2E-6 | 3.6E-4 | 1.2E0 | 1.4E1 | 255 | 57 | 103 | 57 | 0.56 |
| Dd | ug/mL | 6.9E-2 | 1.1E-1 | 1.8E-1 | 2.5E-1 | 2.6E-1 | 5.1E-1 | 1.9E-4 | 1.6E-4 | 1.6E0 | 3.6E0 | 255 | 57 | 103 | 57 | 0.52 |
| De | ng/mL | 3.4E-3 | 3.4E-3 | 5.6E-2 | 1.4E-1 | 1.1E-1 | 2.0E-1 | 3.4E-3 | 3.4E-3 | 5.9E-1 | 9.0E-1 | 255 | 57 | 103 | 57 | 0.62 |
| Dg | ng/mL | 2.5E1 | 4.6E1 | 3.9E1 | 5.6E1 | 3.8E1 | 4.2E1 | 2.8E-1 | 7.8E-1 | 1.9E2 | 1.9E2 | 255 | 57 | 103 | 57 | 0.63 |
| Di | pg/mL | 1.6E0 | 3.1E0 | 2.0E0 | 3.1E0 | 1.8E0 | 2.2E0 | 1.8E-1 | 1.8E-1 | 1.3E1 | 9.3E0 | 255 | 57 | 103 | 57 | 0.66 |
| Dk | uIU/mL | 1.4E-2 | 1.9E-2 | 9.3E-2 | 1.1E-1 | 6.3E-1 | 3.2E-1 | 1.1E-4 | 1.1E-4 | 8.9E0 | 2.3E0 | 255 | 57 | 103 | 57 | 0.60 |
| Dl | ng/mL | 2.0E2 | 2.8E2 | 2.7E2 | 4.2E2 | 2.4E2 | 3.6E2 | 3.1E0 | 4.7E0 | 1.4E3 | 1.6E3 | 255 | 57 | 103 | 57 | 0.63 |
| Dp | ng/ml | 2.5E0 | 2.2E0 | 4.9E0 | 9.3E0 | 6.8E0 | 2.9E1 | 3.7E-3 | 3.7E-3 | 4.3E1 | 2.0E2 | 122 | 51 | 99 | 51 | 0.47 |
| Dr | pg/ml | 3.4E1 | 3.4E1 | 5.7E1 | 6.0E2 | 6.6E1 | 2.3E3 | 7.5E-1 | 7.5E-1 | 2.9E2 | 1.0E4 | 99 | 21 | 50 | 21 | 0.56 |
| Du | pg/ml | 4.2E1 | 1.9E2 | 5.0E2 | 1.8E3 | 1.3E3 | 5.5E3 | 1.2E0 | 1.2E0 | 7.0E3 | 2.4E4 | 53 | 18 | 42 | 18 | 0.60 |
| Dw | ng/ml | 2.6E-2 | 9.2E-3 | 7.8E-2 | 2.2E-2 | 1.4E-1 | 1.7E-2 | 9.2E-3 | 9.2E-3 | 6.7E-1 | 4.9E-2 | 35 | 7 | 7 | 7 | 0.37 |
| Ef | ng/ml | 9.5E-2 | 2.3E-1 | 6.9E-1 | 1.4E0 | 1.7E0 | 2.5E0 | 5.7E-4 | 5.7E-3 | 1.0E1 | 9.5E0 | 165 | 53 | 102 | 53 | 0.61 |
| Wm | % | 8.2E-1 | 4.9E-1 | 3.0E1 | 2.5E1 | 1.9E2 | 1.2E2 | 5.4E-2 | 8.5E-2 | 2.4E3 | 8.7E2 | 204 | 56 | 119 | 56 | 0.50 |
| Ed | pg/ml | 5.2E-1 | 5.2E-1 | 9.3E1 | 2.8E1 | 6.6E2 | 4.0E1 | 5.2E-1 | 5.2E-1 | 7.3E3 | 1.5E2 | 122 | 51 | 98 | 51 | 0.49 |
| Eo | ng/ml | 2.3E0 | 1.8E0 | 4.7E0 | 3.0E0 | 5.2E0 | 3.5E0 | 1.3E-1 | 3.6E-1 | 1.7E1 | 9.2E0 | 35 | 7 | 7 | 7 | 0.43 |
| Yf | ng/mL | 1.6E1 | 1.5E1 | 1.4E2 | 3.0E1 | 8.4E2 | 4.6E1 | 2.9E-1 | 2.9E-1 | 6.6E3 | 1.7E2 | 61 | 18 | 50 | 18 | 0.42 |
| Tj | pg/mL | 3.7E-1 | 3.7E-1 | 7.6E1 | 9.0E0 | 3.7E2 | 2.3E1 | 3.6E-1 | 3.7E-1 | 3.5E3 | 1.4E2 | 165 | 52 | 103 | 52 | 0.47 |
| Po | pg/ml | 1.3E-1 | 3.6E0 | 8.0E0 | 1.2E1 | 2.7E1 | 3.1E1 | 8.0E-3 | 2.6E-2 | 2.7E2 | 2.2E2 | 359 | 90 | 189 | 90 | 0.61 |
| Ti | ug/mL | 2.8E0 | 4.4E0 | 4.1E0 | 6.7E0 | 3.7E0 | 7.7E0 | 1.2E-1 | 8.4E-1 | 1.6E1 | 3.7E1 | 86 | 24 | 69 | 24 | 0.62 |
| Em | ng/ml | 2.9E-3 | 1.3E-2 | 5.3E-2 | 1.2E-1 | 1.1E-1 | 3.8E-1 | 2.8E-16 | 2.8E-16 | 5.4E-1 | 1.9E0 | 123 | 27 | 51 | 27 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Et | ng/ml | 1.0E3 | 2.0E3 | 1.3E3 | 2.1E3 | 1.0E3 | 1.3E3 | 7.5E1 | 7.9E1 | 4.9E3 | 5.0E3 | 359 | 90 | 189 | 90 | 0.68 |
| Eq | pg/ml | 1.3E2 | 4.3E2 | 2.7E2 | 4.3E2 | 3.8E2 | 4.2E2 | 1.0E0 | 1.0E0 | 1.8E3 | 1.3E3 | 53 | 18 | 42 | 18 | 0.60 |
| Ew | U/ml | 1.7E0 | 1.9E0 | 1.8E0 | 2.0E0 | 8.2E-1 | 8.5E-1 | 6.5E-1 | 1.3E0 | 4.0E0 | 3.5E0 | 35 | 7 | 7 | 7 | 0.58 |
| Th | ug/mL | 1.1E0 | 1.1E0 | 1.5E0 | 1.9E0 | 1.6E0 | 1.8E0 | 2.6E-3 | 2.9E-1 | 1.2E1 | 7.5E0 | 86 | 24 | 69 | 24 | 0.57 |
| Fa | ng/ml | 3.9E1 | 4.0E1 | 1.2E2 | 1.6E2 | 7.3E2 | 3.4E2 | 3.4E-2 | 6.0E-1 | 8.0E3 | 2.1E3 | 120 | 51 | 98 | 51 | 0.60 |
| Ez | ng/ml | 3.7E0 | 5.0E0 | 1.6E1 | 2.1E1 | 3.9E1 | 6.3E1 | 1.3E-2 | 1.3E-2 | 3.0E2 | 4.5E2 | 122 | 51 | 99 | 51 | 0.56 |
| Fb | ng/ml | 2.3E1 | 2.8E1 | 2.1E1 | 2.7E1 | 1.2E1 | 9.1E0 | 1.0E0 | 6.6E-1 | 5.7E1 | 4.3E1 | 120 | 52 | 98 | 52 | 0.66 |
| Ex | ng/ml | 6.0E-2 | 9.7E-2 | 2.4E-1 | 3.1E-1 | 8.4E-1 | 6.6E-1 | 3.5E-5 | 1.7E-4 | 8.9E0 | 4.1E0 | 126 | 43 | 69 | 43 | 0.63 |
| Fc | pg/ml | 2.2E-1 | 2.2E-1 | 2.9E2 | 1.9E1 | 2.0E3 | 7.1E1 | 2.2E-1 | 2.2E-1 | 1.5E4 | 3.1E2 | 53 | 19 | 42 | 19 | 0.51 |
| Fd | pg/ml | 2.9E1 | 7.3E1 | 9.7E2 | 1.5E3 | 4.6E3 | 5.7E3 | 9.8E-1 | 9.8E-1 | 3.3E4 | 2.5E4 | 53 | 19 | 42 | 19 | 0.51 |
| Fi | pg/ml | 2.5E-1 | 2.5E-1 | 3.3E2 | 2.1E1 | 1.9E3 | 6.2E1 | 2.5E-1 | 2.5E-1 | 1.4E4 | 2.5E2 | 53 | 19 | 42 | 19 | 0.47 |
| Fn | ng/ml | 2.1E-1 | 2.1E-1 | 8.5E0 | 2.9E0 | 3.8E1 | 5.5E0 | 1.1E-14 | 1.1E-14 | 4.2E2 | 2.7E1 | 122 | 51 | 99 | 51 | 0.46 |
| Fp | ng/ml | 9.4E0 | 2.1E1 | 2.0E1 | 3.1E1 | 2.6E1 | 3.3E1 | 6.0E-3 | 5.8E-2 | 1.4E2 | 1.3E2 | 380 | 90 | 189 | 90 | 0.60 |
| Fr | ng/ml | 2.5E4 | 5.5E4 | 9.9E4 | 1.8E5 | 1.7E5 | 2.4E5 | 6.4E2 | 1.3E3 | 8.4E5 | 8.4E5 | 464 | 95 | 193 | 95 | 0.62 |
| Fw | pg/ml | 7.1E-1 | 2.9E0 | 1.0E2 | 2.0E1 | 6.7E2 | 4.9E1 | 1.1E-14 | 1.7E-14 | 6.9E3 | 3.3E2 | 167 | 55 | 103 | 55 | 0.54 |
| Fy | ng/ml | 3.1E1 | 4.2E1 | 4.9E1 | 7.2E1 | 5.5E1 | 1.0E2 | 1.2E-1 | 1.2E-1 | 3.3E2 | 6.5E2 | 121 | 49 | 98 | 49 | 0.57 |
| Gh | pg/ml | 2.3E0 | 2.0E0 | 2.5E1 | 8.8E1 | 5.0E1 | 3.5E2 | 2.9E-2 | 2.9E-2 | 2.7E2 | 1.5E3 | 53 | 18 | 42 | 18 | 0.44 |
| Gb | % | 3.2E1 | 5.7E1 | 4.3E1 | 6.2E1 | 3.9E1 | 6.4E1 | 2.7E0 | 2.2E0 | 2.3E2 | 3.0E2 | 53 | 19 | 41 | 19 | 0.64 |
| Gc | ng/ml | 9.9E1 | 1.3E2 | 1.4E2 | 1.8E2 | 1.4E2 | 1.7E2 | 6.4E0 | 2.7E1 | 7.9E2 | 6.3E2 | 104 | 21 | 52 | 21 | 0.58 |
| Gd | ng/ml | 2.9E1 | 3.8E1 | 3.0E1 | 3.7E1 | 1.6E1 | 2.3E1 | 5.4E0 | 3.0E0 | 8.1E1 | 8.0E1 | 119 | 25 | 50 | 25 | 0.58 |
| Gn | U/ml | 5.0E-1 | 2.2E-1 | 1.7E0 | 5.8E0 | 3.7E0 | 2.5E1 | 1.3E-3 | 5.6E-3 | 3.0E1 | 1.1E2 | 95 | 21 | 50 | 21 | 0.42 |
| Gl | pg/ml | 6.1E3 | 1.1E4 | 9.8E3 | 1.3E4 | 9.0E3 | 9.8E3 | 2.3E2 | 5.3E2 | 3.4E4 | 3.2E4 | 162 | 55 | 102 | 55 | 0.61 |
| Gp | U/ml | 1.8E0 | 1.1E0 | 4.4E0 | 2.8E0 | 7.6E0 | 4.1E0 | 1.3E-3 | 1.5E-2 | 6.7E1 | 2.0E1 | 167 | 53 | 103 | 53 | 0.42 |
| Gz | ug/ml | 1.2E0 | 3.0E0 | 1.1E1 | 8.7E0 | 5.3E1 | 2.2E1 | 2.9E-16 | 4.2E-2 | 4.8E2 | 1.5E2 | 81 | 42 | 64 | 42 | 0.52 |
| IIa | ng/ml | 2.7E0 | 2.7E0 | 8.4E0 | 1.1E1 | 1.9E1 | 2.1E1 | 1.7E-2 | 1.4E-3 | 1.0E2 | 1.1E2 | 120 | 51 | 98 | 51 | 0.52 |
| Nm | pg/ml | 1.2E4 | 2.0E4 | 2.4E4 | 5.3E4 | 4.9E4 | 1.3E5 | 1.0E-9 | 1.0E-9 | 7.8E5 | 9.6E5 | 358 | 92 | 190 | 92 | 0.58 |
| Nn | pg/ml | 1.3E2 | 2.4E2 | 2.5E3 | 3.1E3 | 1.1E4 | 1.4E4 | 1.0E-9 | 1.0E-9 | 1.0E5 | 1.1E5 | 358 | 92 | 190 | 92 | 0.59 |
| No | pg/ml | 1.3E1 | 1.9E1 | 2.7E1 | 5.7E1 | 5.9E1 | 1.3E2 | 1.0E-9 | 1.0E-9 | 5.9E2 | 7.7E2 | 358 | 92 | 190 | 92 | 0.59 |
| Nq | pg/ml | 2.2E0 | 2.4E0 | 2.1E1 | 1.8E1 | 8.4E1 | 4.5E1 | 1.0E-9 | 1.0E-9 | 1.2E3 | 2.9E2 | 358 | 92 | 190 | 92 | 0.52 |
| Nr | pg/ml | 4.7E-1 | 2.5E0 | 1.6E1 | 3.7E1 | 7.0E1 | 1.6E2 | 1.0E-9 | 1.0E-9 | 9.8E2 | 1.4E3 | 358 | 92 | 190 | 92 | 0.61 |
| Ns | pg/ml | 1.0E-9 | 1.0E-9 | 8.9E0 | 7.0E0 | 4.5E1 | 3.4E1 | 1.0E-9 | 1.0E-9 | 6.8E2 | 2.4E2 | 358 | 92 | 190 | 92 | 0.48 |
| Nt | pg/ml | 9.4E1 | 1.3E2 | 1.2E2 | 1.7E2 | 9.2E1 | 1.6E2 | 1.0E-9 | 1.2E1 | 5.9E2 | 1.2E3 | 358 | 92 | 190 | 92 | 0.61 |
| Nu | pg/ml | 1.7E1 | 3.2E1 | 4.9E1 | 7.2E1 | 8.8E1 | 1.0E2 | 1.0E-9 | 1.0E-9 | 8.9E2 | 6.3E2 | 358 | 92 | 190 | 92 | 0.58 |
| Lu | pg/ml | 1.0E4 | 1.0E4 | 1.5E4 | 1.2E4 | 3.2E4 | 1.2E4 | 6.7E2 | 1.1E3 | 3.9E5 | 7.5E4 | 360 | 92 | 190 | 92 | 0.47 |
| Lv | pg/ml | 1.0E-9 | 6.9E-1 | 1.1E1 | 2.2E1 | 2.2E1 | 3.4E1 | 1.0E-9 | 1.0E-9 | 2.4E2 | 1.9E2 | 360 | 92 | 190 | 92 | 0.59 |
| Lw | pg/ml | 1.0E-9 | 1.0E-9 | 1.4E-1 | 2.4E0 | 1.8E0 | 1.1E1 | 1.0E-9 | 1.0E-9 | 3.2E1 | 8.0E1 | 360 | 92 | 190 | 92 | 0.53 |
| Lx | pg/ml | 1.0E-9 | 4.7E1 | 1.1E2 | 2.7E2 | 4.2E2 | 5.4E2 | 1.0E-9 | 1.0E-9 | 6.2E3 | 2.8E3 | 360 | 92 | 190 | 92 | 0.63 |
| Ly | pg/ml | 1.0E-9 | 1.0E-9 | 9.1E0 | 1.3E1 | 1.8E1 | 2.2E1 | 1.0E-9 | 1.0E-9 | 1.2E2 | 9.6E1 | 360 | 92 | 190 | 92 | 0.53 |
| Lz | pg/ml | 1.0E-9 | 1.0E-9 | 2.9E0 | 5.5E0 | 2.4E1 | 3.0E1 | 1.0E-9 | 1.0E-9 | 4.3E2 | 2.6E2 | 360 | 92 | 190 | 92 | 0.51 |
| Ma | pg/ml | 2.4E2 | 4.7E2 | 1.5E3 | 1.8E3 | 4.7E3 | 3.4E3 | 1.0E-9 | 1.0E-9 | 6.5E4 | 2.2E4 | 360 | 92 | 190 | 92 | 0.60 |
| Mb | pg/ml | 2.5E1 | 3.2E1 | 3.1E1 | 3.7E1 | 1.4E1 | 2.4E1 | 5.4E0 | 4.1E0 | 9.3E1 | 2.1E2 | 360 | 92 | 190 | 92 | 0.56 |
| Mc | pg/ml | 1.0E-9 | 1.0E-9 | 1.7E-2 | 1.7E-1 | 2.3E-1 | 1.4E0 | 1.0E-9 | 1.0E-9 | 4.0E0 | 1.3E1 | 360 | 92 | 190 | 92 | 0.51 |
| Md | pg/ml | 1.0E-9 | 1.0E-9 | 6.2E-1 | 7.2E-1 | 5.2E0 | 3.7E0 | 1.0E-9 | 1.0E-9 | 6.8E1 | 2.9E1 | 360 | 92 | 190 | 92 | 0.53 |
| Me | pg/ml | 3.2E1 | 2.8E1 | 2.9E1 | 3.1E1 | 1.6E1 | 3.8E1 | 1.0E-9 | 1.0E-9 | 1.2E2 | 3.2E2 | 360 | 92 | 190 | 92 | 0.44 |
| Mf | pg/ml | 1.0E-9 | 1.0E-9 | 3.7E-1 | 1.1E0 | 1.6E0 | 6.0E0 | 1.0E-9 | 1.0E-9 | 2.0E1 | 5.6E1 | 360 | 92 | 190 | 92 | 0.54 |
| Mg | pg/ml | 1.9E0 | 1.7E0 | 6.4E0 | 1.0E1 | 1.1E1 | 1.6E1 | 1.0E-9 | 1.0E-9 | 8.8E1 | 9.2E1 | 360 | 92 | 190 | 92 | 0.55 |
| Mh | pg/ml | 1.0E-9 | 1.0E-9 | 1.1E0 | 8.8E-1 | 8.4E0 | 3.1E0 | 1.0E-9 | 1.0E-9 | 1.1E2 | 1.8E1 | 360 | 92 | 190 | 92 | 0.53 |
| Mi | pg/ml | 1.0E-9 | 1.0E-9 | 6.9E-1 | 1.8E0 | 7.1E0 | 1.2E1 | 1.0E-9 | 1.0E-9 | 1.2E2 | 1.1E2 | 360 | 92 | 190 | 92 | 0.51 |
| Mj | pg/ml | 1.0E-9 | 1.0E-9 | 5.2E0 | 7.4E0 | 3.1E1 | 2.9E1 | 1.0E-9 | 1.0E-9 | 4.6E2 | 2.1E2 | 360 | 92 | 190 | 92 | 0.52 |
| Mk | pg/ml | 6.5E-1 | 1.5E0 | 1.6E1 | 1.3E1 | 9.9E1 | 5.4E1 | 1.0E-9 | 1.0E-9 | 1.7E3 | 5.0E2 | 360 | 92 | 190 | 92 | 0.50 |
| Ml | pg/ml | 1.0E-9 | 1.0E-9 | 9.1E0 | 9.4E0 | 1.1E2 | 5.5E1 | 1.0E-9 | 1.0E-9 | 2.1E3 | 5.1E2 | 360 | 92 | 190 | 92 | 0.55 |
| Mm | pg/ml | 4.0E2 | 8.3E2 | 8.0E2 | 1.5E3 | 9.7E2 | 1.9E3 | 1.0E-9 | 1.0E-9 | 6.0E3 | 1.2E4 | 360 | 92 | 190 | 92 | 0.60 |
| Mn | pg/ml | 4.8E0 | 7.3E0 | 1.0E1 | 1.2E1 | 2.5E1 | 1.3E1 | 1.0E-9 | 1.0E-9 | 3.5E2 | 7.8E1 | 360 | 92 | 190 | 92 | 0.62 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Mp | pg/ml | 1.0E-9 | 2.7E0 | 9.7E0 | 1.2E1 | 2.2E1 | 2.3E1 | 1.0E-9 | 1.0E-9 | 1.8E2 | 1.3E2 | 360 | 92 | 190 | 92 | 0.57 |
| Mq | pg/ml | 1.0E-9 | 1.0E-9 | 2.8E0 | 5.7E0 | 1.7E1 | 1.9E1 | 1.0E-9 | 1.0E-9 | 2.1E2 | 1.0E2 | 360 | 92 | 190 | 92 | 0.54 |
| Mr | pg/ml | 1.0E-9 | 1.0E-9 | 1.4E1 | 7.0E1 | 8.7E1 | 3.8E2 | 1.0E-9 | 1.0E-9 | 1.4E3 | 3.4E3 | 360 | 92 | 190 | 92 | 0.56 |
| Ms | pg/ml | 3.8E2 | 3.8E2 | 5.6E2 | 4.7E2 | 7.1E2 | 4.3E2 | 1.0E-9 | 1.0E-9 | 5.6E3 | 2.0E3 | 360 | 92 | 190 | 92 | 0.49 |
| Mt | pg/ml | 1.0E-9 | 7.6E-1 | 1.1E1 | 4.2E1 | 6.3E1 | 3.4E2 | 1.0E-9 | 1.0E-9 | 8.7E2 | 3.2E3 | 360 | 92 | 190 | 92 | 0.59 |
| Mu | pg/ml | 1.0E-9 | 1.0E-9 | 1.5E0 | 1.1E0 | 1.0E1 | 4.4E0 | 1.0E-9 | 1.0E-9 | 9.9E1 | 3.5E1 | 360 | 92 | 190 | 92 | 0.55 |
| Mv | pg/ml | 1.0E-9 | 1.0E-9 | 8.2E1 | 7.7E1 | 4.2E2 | 1.8E2 | 1.0E-9 | 1.0E-9 | 4.5E3 | 9.2E2 | 360 | 92 | 190 | 92 | 0.55 |
| Mw | pg/ml | 2.5E1 | 3.8E1 | 5.8E2 | 4.1E2 | 3.9E3 | 1.2E3 | 1.0E-9 | 1.0E-9 | 6.2E4 | 7.9E3 | 360 | 92 | 190 | 92 | 0.55 |
| Mx | pg/ml | 1.0E-9 | 1.0E-9 | 1.8E-1 | 5.0E-1 | 8.2E-1 | 2.2E0 | 1.0E-9 | 1.0E-9 | 9.2E0 | 2.0E1 | 360 | 92 | 190 | 92 | 0.55 |
| My | pg/ml | 1.0E-9 | 1.0E-9 | 5.6E2 | 3.2E2 | 3.5E3 | 1.4E3 | 1.0E-9 | 1.0E-9 | 3.9E4 | 1.1E4 | 360 | 92 | 190 | 92 | 0.50 |
| Mz | pg/ml | 8.5E0 | 1.3E1 | 2.1E1 | 6.3E1 | 5.9E1 | 2.4E2 | 1.0E-9 | 1.0E-9 | 8.4E2 | 1.9E3 | 360 | 92 | 190 | 92 | 0.61 |
| Na | pg/ml | 1.0E-9 | 1.0E-9 | 5.7E-1 | 1.2E0 | 1.8E0 | 4.7E0 | 1.0E-9 | 1.0E-9 | 7.8E0 | 4.2E1 | 360 | 92 | 190 | 92 | 0.52 |
| Nb | pg/ml | 1.8E0 | 2.4E0 | 4.6E0 | 6.9E0 | 1.6E1 | 2.5E1 | 1.0E-9 | 1.0E-9 | 2.3E2 | 2.1E2 | 360 | 92 | 190 | 92 | 0.55 |
| Nc | pg/ml | 4.5E2 | 2.2E2 | 6.7E2 | 3.7E2 | 8.4E2 | 4.4E2 | 1.0E-9 | 1.0E-9 | 9.2E3 | 1.8E3 | 360 | 92 | 190 | 92 | 0.39 |
| Nd | pg/ml | 3.1E1 | 6.7E0 | 2.6E1 | 1.6E1 | 2.0E1 | 1.7E1 | 1.0E-9 | 1.0E-9 | 8.4E1 | 5.8E1 | 360 | 92 | 190 | 92 | 0.36 |
| Ne | pg/ml | 4.9E2 | 3.2E2 | 6.3E2 | 4.2E2 | 6.4E2 | 4.1E2 | 1.0E-9 | 1.0E-9 | 7.0E3 | 1.8E3 | 360 | 92 | 190 | 92 | 0.39 |
| Nf | pg/ml | 1.0E-9 | 1.0E-9 | 2.9E0 | 3.6E0 | 8.8E0 | 1.8E1 | 1.0E-9 | 1.0E-9 | 8.2E1 | 1.3E2 | 360 | 92 | 190 | 92 | 0.46 |
| Ng | pg/ml | 2.1E1 | 2.9E1 | 1.1E2 | 1.3E2 | 2.3E2 | 2.3E2 | 1.0E-9 | 1.0E-9 | 1.6E3 | 1.2E3 | 360 | 92 | 190 | 92 | 0.50 |
| Nh | pg/ml | 7.7E1 | 4.7E1 | 9.8E1 | 7.0E1 | 8.7E1 | 7.3E1 | 1.0E-9 | 3.1E0 | 5.6E2 | 3.5E2 | 360 | 92 | 190 | 92 | 0.38 |
| Ni | pg/ml | 4.5E0 | 1.4E1 | 7.4E1 | 1.1E2 | 1.1E2 | 1.7E2 | 1.0E-9 | 1.0E-9 | 7.1E2 | 1.1E3 | 360 | 92 | 190 | 92 | 0.54 |
| Nj | pg/ml | 9.0E0 | 3.8E0 | 1.2E1 | 7.6E0 | 1.2E1 | 9.6E0 | 1.0E-9 | 1.0E-9 | 8.3E1 | 5.8E1 | 360 | 92 | 190 | 92 | 0.37 |
| Nk | pg/ml | 2.2E1 | 1.4E1 | 3.6E1 | 2.5E1 | 4.0E1 | 3.2E1 | 1.0E-9 | 1.0E-9 | 1.8E2 | 1.5E2 | 360 | 92 | 190 | 92 | 0.43 |
| Nl | pg/ml | 5.2E1 | 2.7E1 | 7.0E1 | 4.0E1 | 8.2E1 | 4.1E1 | 1.0E-9 | 1.0E-9 | 1.1E3 | 1.6E2 | 360 | 92 | 190 | 92 | 0.37 |
| Hl | pg/ml | 1.3E1 | 1.4E1 | 4.5E1 | 2.1E2 | 7.9E1 | 8.2E2 | 1.0E-9 | 1.0E-9 | 3.5E2 | 3.6E3 | 53 | 19 | 42 | 19 | 0.46 |
| Ilo | pg/ml | 1.5E1 | 1.9E1 | 3.2E1 | 3.8E1 | 9.6E1 | 8.6E1 | 1.0E-9 | 6.7E0 | 7.0E2 | 3.9E2 | 53 | 19 | 42 | 19 | 0.62 |
| Hp | ng/ml | 1.7E0 | 1.6E0 | 7.2E1 | 2.4E2 | 2.4E2 | 4.0E2 | 1.0E-9 | 1.0E-9 | 8.9E2 | 8.9E2 | 53 | 19 | 42 | 19 | 0.53 |
| Tz | pg/ml | 3.7E3 | 7.4E3 | 7.5E3 | 1.1E4 | 1.1E4 | 1.2E4 | 7.4E1 | 9.8E1 | 8.8E4 | 6.7E4 | 124 | 50 | 98 | 50 | 0.62 |
| Ua | pg/ml | 3.5E3 | 5.1E3 | 3.0E4 | 1.6E4 | 1.9E5 | 2.8E4 | 3.5E2 | 2.7E2 | 2.1E6 | 1.3E5 | 124 | 50 | 98 | 50 | 0.58 |
| Ub | pg/ml | 5.8E2 | 4.0E2 | 8.7E2 | 6.5E2 | 1.2E3 | 8.1E2 | 1.0E-9 | 1.2E1 | 9.8E3 | 4.4E3 | 124 | 50 | 98 | 50 | 0.43 |
| Ue | pg/ml | 3.1E1 | 2.7E1 | 3.5E1 | 4.6E1 | 2.4E1 | 4.7E1 | 4.2E0 | 5.2E0 | 1.2E2 | 2.7E2 | 124 | 50 | 98 | 50 | 0.52 |
| Uc | pg/ml | 7.8E2 | 1.1E3 | 1.6E3 | 2.9E3 | 3.3E3 | 8.2E3 | 3.3E1 | 1.5E1 | 2.9E4 | 5.7E4 | 124 | 50 | 98 | 50 | 0.57 |
| Ud | pg/ml | 1.0E-9 | 1.0E-9 | 3.4E0 | 1.1E0 | 3.5E1 | 7.5E0 | 1.0E-9 | 1.0E-9 | 3.9E2 | 5.3E1 | 124 | 50 | 98 | 50 | 0.50 |
| Hq | pg/ml | 1.1E0 | 1.8E0 | 1.1E1 | 1.3E1 | 5.9E1 | 4.3E1 | 1.0E-9 | 1.0E-9 | 9.5E2 | 3.4E2 | 360 | 90 | 190 | 90 | 0.58 |
| Hr | pg/ml | 1.3E2 | 8.8E1 | 8.3E2 | 7.6E2 | 1.6E3 | 1.9E3 | 1.0E-9 | 1.0E-9 | 1.2E4 | 1.4E4 | 360 | 90 | 190 | 90 | 0.46 |
| Hu | pg/ml | 7.9E0 | 2.1E1 | 3.5E3 | 1.5E3 | 3.5E4 | 5.3E3 | 1.0E-9 | 1.0E-9 | 6.3E5 | 3.5E4 | 360 | 90 | 190 | 90 | 0.54 |
| Hv | pg/ml | 1.3E0 | 1.6E0 | 3.4E0 | 1.3E1 | 1.5E1 | 9.4E1 | 1.0E-9 | 1.0E-9 | 2.5E2 | 8.9E2 | 360 | 90 | 190 | 90 | 0.56 |
| Hw | pg/ml | 7.2E0 | 6.5E0 | 2.5E1 | 1.2E2 | 1.1E2 | 9.9E2 | 1.0E-9 | 1.0E-9 | 1.7E3 | 9.4E3 | 360 | 90 | 190 | 90 | 0.47 |
| Hx | pg/ml | 7.2E0 | 1.3E1 | 6.0E1 | 4.9E1 | 5.0E2 | 1.6E2 | 1.0E-9 | 1.0E-9 | 9.3E3 | 1.3E3 | 360 | 90 | 190 | 90 | 0.56 |
| Ib | ng/ml | 6.9E-2 | 4.4E-2 | 6.9E-1 | 3.2E0 | 3.0E0 | 1.0E1 | 1.0E-9 | 1.0E-9 | 3.0E1 | 5.6E1 | 122 | 51 | 99 | 51 | 0.48 |
| Ic | U/ml | 2.2E2 | 2.2E2 | 3.1E2 | 2.1E3 | 1.4E3 | 9.5E3 | 2.9E0 | 6.6E0 | 1.5E4 | 6.5E4 | 122 | 51 | 99 | 51 | 0.63 |
| Id | U/ml | 6.2E-1 | 6.9E-1 | 1.0E0 | 1.0E1 | 1.2E0 | 6.0E1 | 1.0E-9 | 2.2E-1 | 6.9E0 | 4.3E2 | 122 | 51 | 99 | 51 | 0.57 |
| Tt | pg/ml | 1.6E2 | 1.9E2 | 1.6E2 | 2.0E2 | 4.4E1 | 6.7E1 | 8.0E1 | 7.6E1 | 3.0E2 | 4.4E2 | 114 | 43 | 92 | 43 | 0.67 |
| To | pg/ml | 1.6E0 | 1.8E0 | 1.9E0 | 2.1E0 | 2.6E0 | 2.7E0 | 1.0E-9 | 1.0E-9 | 2.3E1 | 1.2E1 | 119 | 47 | 95 | 47 | 0.49 |
| Tr | pg/ml | 2.8E0 | 4.1E0 | 4.1E0 | 7.1E0 | 4.7E0 | 1.1E1 | 3.5E-2 | 1.0E-9 | 2.9E1 | 7.6E1 | 118 | 46 | 94 | 46 | 0.61 |
| Tn | pg/ml | 2.1E1 | 4.4E1 | 5.9E1 | 1.4E2 | 1.5E2 | 3.8E2 | 2.6E0 | 6.6E0 | 1.5E3 | 2.3E3 | 119 | 47 | 95 | 47 | 0.67 |
| Tv | ng/ml | 1.2E1 | 1.2E1 | 2.0E1 | 1.7E2 | 4.8E1 | 1.0E3 | 1.0E-9 | 1.0E-9 | 4.9E2 | 7.1E3 | 119 | 47 | 95 | 47 | 0.54 |
| Ih | ng/ml | 5.7E1 | 7.5E1 | 2.0E2 | 2.9E2 | 3.6E2 | 5.0E2 | 1.0E-9 | 1.0E-9 | 2.5E3 | 2.8E3 | 360 | 92 | 190 | 92 | 0.54 |
| Ii | ng/ml | 7.0E1 | 9.5E1 | 2.7E2 | 2.6E2 | 8.1E2 | 6.2E2 | 7.5E-1 | 1.3E0 | 8.4E3 | 4.5E3 | 360 | 92 | 190 | 92 | 0.53 |
| Ij | ng/ml | 7.0E1 | 7.1E1 | 2.0E2 | 3.9E2 | 7.3E2 | 2.6E3 | 2.1E0 | 9.3E0 | 6.4E3 | 2.4E4 | 358 | 89 | 190 | 89 | 0.53 |
| Ik | ng/ml | 1.2E1 | 6.1E1 | 9.2E2 | 1.7E3 | 9.2E3 | 1.3E4 | 5.9E-1 | 1.7E0 | 1.2E5 | 1.2E5 | 358 | 91 | 190 | 91 | 0.58 |
| Il | ng/ml | 3.3E2 | 2.6E2 | 1.4E3 | 1.1E3 | 3.0E3 | 2.6E3 | 1.0E-9 | 1.0E-9 | 1.2E4 | 1.3E4 | 354 | 89 | 190 | 89 | 0.46 |
| Im | ng/ml | 1.9E2 | 2.3E2 | 3.0E2 | 4.8E2 | 3.4E2 | 9.3E2 | 1.3E1 | 2.0E1 | 3.1E3 | 6.2E3 | 357 | 90 | 190 | 90 | 0.56 |
| In | ng/ml | 4.2E0 | 3.0E0 | 3.6E1 | 5.6E1 | 2.3E2 | 4.7E2 | 1.0E-9 | 1.0E-9 | 3.9E3 | 4.5E3 | 360 | 92 | 190 | 92 | 0.45 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Hb | ng/ml | 2.0E1 | 2.9E1 | 2.7E1 | 3.7E1 | 2.6E1 | 3.4E1 | 1.1E0 | 4.8E-1 | 1.5E2 | 2.0E2 | 120 | 53 | 98 | 53 | 0.62 |
| Hc | pg/ml | 6.1E2 | 5.8E2 | 2.2E3 | 4.3E3 | 4.9E3 | 1.4E4 | 1.0E-9 | 1.0E-9 | 3.6E4 | 1.0E5 | 120 | 53 | 98 | 53 | 0.55 |
| Hf | ng/ml | 1.2E2 | 1.5E2 | 3.6E2 | 2.5E2 | 5.3E2 | 3.2E2 | 1.0E-9 | 1.0E-9 | 2.5E3 | 1.8E3 | 120 | 53 | 98 | 53 | 0.51 |
| Io | ng/ml | 6.9E3 | 8.2E3 | 1.7E4 | 1.7E4 | 5.1E4 | 2.4E4 | 6.6E1 | 6.6E1 | 8.8E5 | 1.1E5 | 357 | 90 | 189 | 90 | 0.53 |
| Ip | ng/ml | 8.1E0 | 1.9E1 | 1.8E1 | 2.6E1 | 2.7E1 | 3.0E1 | 1.0E-9 | 4.9E-3 | 2.6E2 | 1.6E2 | 357 | 90 | 189 | 90 | 0.55 |
| Iq | ug/ml | 9.1E-2 | 1.1E-1 | 3.9E1 | 3.7E0 | 7.2E2 | 2.3E1 | 1.0E-9 | 1.0E-9 | 1.4E4 | 2.2E2 | 357 | 90 | 189 | 90 | 0.52 |
| Ir | ug/ml | 2.9E-1 | 5.5E-1 | 4.4E0 | 8.1E0 | 3.3E1 | 4.3E1 | 1.0E-9 | 1.0E-9 | 5.1E2 | 3.7E2 | 356 | 90 | 189 | 90 | 0.60 |
| Is | ng/ml | 1.3E0 | 3.1E0 | 6.4E0 | 1.2E1 | 3.1E1 | 3.3E1 | 1.0E-9 | 2.2E-3 | 5.5E2 | 2.6E2 | 357 | 90 | 189 | 90 | 0.62 |
| It | ng/ml | 1.9E0 | 2.5E0 | 2.6E1 | 1.6E1 | 1.3E2 | 7.5E1 | 1.0E-9 | 1.0E-9 | 1.2E3 | 6.8E2 | 357 | 90 | 189 | 90 | 0.57 |
| Iu | ng/ml | 1.9E2 | 2.4E2 | 1.6E3 | 1.5E3 | 5.0E3 | 3.8E3 | 1.0E-9 | 1.0E-9 | 2.9E4 | 2.4E4 | 357 | 90 | 189 | 90 | 0.52 |
| Iv | ng/ml | 1.1E1 | 2.3E1 | 8.5E1 | 1.3E2 | 8.5E2 | 5.5E2 | 1.0E-9 | 1.0E-9 | 1.6E4 | 3.8E3 | 357 | 90 | 189 | 90 | 0.63 |
| Iz | ng/ml | 1.2E2 | 1.7E2 | 4.2E2 | 1.5E3 | 9.7E2 | 8.5E3 | 1.5E0 | 4.1E0 | 8.4E3 | 6.2E4 | 120 | 53 | 98 | 53 | 0.58 |
| Yg | pg/ml | 2.5E2 | 3.5E2 | 5.7E2 | 4.0E3 | 8.6E2 | 1.2E4 | 1.0E-9 | 1.0E-9 | 4.2E3 | 5.0E4 | 51 | 18 | 41 | 18 | 0.58 |
| Yh | pg/ml | 2.6E2 | 3.1E2 | 5.6E2 | 5.5E2 | 1.1E3 | 7.5E2 | 1.0E-9 | 1.0E-9 | 7.8E3 | 3.0E3 | 51 | 18 | 41 | 18 | 0.50 |
| Yi | pg/ml | 2.7E2 | 5.0E2 | 5.7E2 | 7.6E2 | 1.1E3 | 1.1E3 | 1.0E-9 | 1.0E-9 | 7.6E3 | 4.6E3 | 51 | 18 | 41 | 18 | 0.57 |
| Yk | U/ml | 1.0E-9 | 1.0E-9 | 9.9E-2 | 6.4E-1 | 2.9E-1 | 1.5E0 | 1.0E-9 | 1.0E-9 | 1.5E0 | 5.6E0 | 51 | 18 | 41 | 18 | 0.57 |
| Yj | pg/ml | 1.9E2 | 1.4E2 | 4.2E2 | 6.2E2 | 5.7E2 | 1.6E3 | 9.7E0 | 3.9E1 | 3.2E3 | 7.0E3 | 51 | 18 | 41 | 18 | 0.41 |
| Yd | ng/ml | 2.0E-1 | 2.7E-1 | 3.2E-1 | 3.5E-1 | 3.8E-1 | 3.8E-1 | 6.6E-3 | 1.7E-2 | 1.8E0 | 1.5E0 | 55 | 19 | 44 | 19 | 0.54 |
| Wb | pg/ml | 3.1E4 | 3.4E4 | 3.4E4 | 4.2E4 | 1.8E4 | 3.2E4 | 2.2E3 | 7.5E3 | 8.5E4 | 1.5E5 | 55 | 19 | 44 | 19 | 0.56 |
| Vz | pg/ml | 3.6E0 | 2.7E0 | 5.6E0 | 4.4E0 | 6.6E0 | 4.8E0 | 1.0E-9 | 7.3E-1 | 4.0E1 | 2.2E1 | 55 | 19 | 44 | 19 | 0.44 |
| Si | ng/ml | 9.1E-1 | 1.2E0 | 1.7E0 | 2.0E0 | 2.2E0 | 2.4E0 | 2.0E-2 | 8.6E-3 | 1.0E1 | 1.0E1 | 53 | 19 | 42 | 19 | 0.57 |
| Sf | mIU/mL | 1.1E1 | 1.9E1 | 4.8E1 | 3.3E1 | 1.1E2 | 4.3E1 | 8.1E-2 | 2.5E0 | 7.2E2 | 1.7E2 | 53 | 19 | 42 | 19 | 0.58 |
| Sh | mIU/mL | 7.7E0 | 1.7E1 | 4.1E1 | 6.3E1 | 1.1E2 | 1.1E2 | 2.9E-2 | 7.8E-2 | 5.9E2 | 3.7E2 | 53 | 19 | 42 | 19 | 0.60 |
| Sj | ng/ml | 4.0E-1 | 4.0E-1 | 4.0E-1 | 4.3E-1 | 9.0E-2 | 1.1E-1 | 1.9E-1 | 2.5E-1 | 5.7E-1 | 6.6E-1 | 53 | 19 | 42 | 19 | 0.56 |
| Rc | pg/ml | 5.0E3 | 7.6E3 | 6.4E3 | 8.6E3 | 5.0E3 | 7.2E3 | 1.9E2 | 2.4E2 | 3.0E4 | 3.9E4 | 122 | 50 | 98 | 50 | 0.60 |
| Rb | pg/ml | 7.5E-1 | 6.1E-1 | 2.4E0 | 3.1E0 | 3.6E0 | 8.4E0 | 1.0E-9 | 1.0E-9 | 1.6E1 | 5.6E1 | 122 | 50 | 98 | 50 | 0.50 |
| Zq | 2.6ng/ml | 1.6E2 | 3.9E2 | 2.5E2 | 4.2E2 | 2.5E2 | 3.0E2 | 8.3E0 | 1.7E1 | 9.7E2 | 9.7E2 | 52 | 19 | 41 | 19 | 0.69 |
| Zw | 2.5ng/ml | 4.3E0 | 6.7E0 | 1.0E1 | 1.3E1 | 1.4E1 | 1.8E1 | 6.3E-2 | 2.4E-1 | 5.9E1 | 5.9E1 | 55 | 19 | 44 | 19 | 0.51 |
| Zx | 2.3mU/ml | 1.1E-1 | 1.4E-1 | 4.0E-1 | 2.8E-1 | 1.6E0 | 6.3E-1 | 4.1E-2 | 3.2E-2 | 1.2E1 | 2.9E0 | 55 | 19 | 44 | 19 | 0.52 |
| Pz | ng/ml | 3.1E3 | 4.2E3 | 6.1E3 | 6.6E3 | 9.7E3 | 8.8E3 | 1.3E1 | 4.7E1 | 9.5E4 | 7.0E4 | 358 | 89 | 189 | 89 | 0.54 |
| Qa | ng/ml | 2.7E3 | 5.2E3 | 5.9E3 | 9.9E3 | 8.0E3 | 2.4E4 | 1.5E2 | 2.3E2 | 5.2E4 | 2.2E5 | 358 | 89 | 189 | 89 | 0.62 |
| Qb | ng/ml | 8.3E1 | 1.2E2 | 2.5E2 | 2.0E2 | 7.0E2 | 2.1E2 | 7.9E-1 | 4.4E0 | 8.3E3 | 1.1E3 | 358 | 89 | 189 | 89 | 0.57 |
| Qc | ng/ml | 1.9E2 | 2.9E2 | 4.7E2 | 4.6E2 | 9.5E2 | 5.2E2 | 8.1E-1 | 3.0E0 | 1.1E4 | 2.8E3 | 358 | 89 | 189 | 89 | 0.55 |
| Qd | ng/ml | 7.9E3 | 1.1E4 | 2.2E4 | 2.7E4 | 1.1E5 | 4.1E4 | 1.5E2 | 6.9E2 | 2.0E6 | 2.3E5 | 358 | 89 | 189 | 89 | 0.60 |
| Qe | ng/ml | 6.9E2 | 1.5E3 | 1.9E3 | 2.2E3 | 5.6E3 | 2.7E3 | 1.0E-9 | 4.7E1 | 9.7E4 | 1.8E4 | 358 | 89 | 189 | 89 | 0.61 |
| Jd | ng/ml | 4.3E-1 | 1.7E0 | 7.5E0 | 5.3E0 | 5.9E1 | 1.4E1 | 1.0E-9 | 1.0E-9 | 6.5E2 | 9.1E1 | 122 | 51 | 99 | 51 | 0.71 |
| Je | ng/ml | 1.0E-9 | 7.3E-1 | 1.4E0 | 3.0E0 | 5.4E0 | 6.6E0 | 1.0E-9 | 1.0E-9 | 5.6E1 | 4.5E1 | 122 | 51 | 99 | 51 | 0.63 |
| Jf | ng/ml | 1.0E-9 | 1.0E-9 | 7.4E-1 | 1.3E0 | 1.9E0 | 2.5E0 | 1.0E-9 | 1.0E-9 | 1.2E1 | 1.0E1 | 122 | 51 | 99 | 51 | 0.54 |
| Jg | ng/ml | 3.7E2 | 7.5E2 | 6.8E2 | 1.1E3 | 1.0E3 | 1.2E3 | 5.8E0 | 1.3E1 | 1.0E4 | 7.1E3 | 360 | 90 | 190 | 90 | 0.63 |
| Jh | ng/ml | 2.3E0 | 4.4E0 | 2.4E1 | 4.6E1 | 9.5E1 | 1.4E2 | 1.0E-9 | 1.0E-9 | 1.2E3 | 1.1E3 | 360 | 90 | 190 | 90 | 0.58 |
| Ji | ng/ml | 4.6E1 | 7.3E1 | 6.6E1 | 1.3E2 | 7.0E1 | 1.8E2 | 1.1E0 | 5.2E0 | 5.3E2 | 1.3E3 | 360 | 90 | 190 | 90 | 0.65 |
| Sr | pg/mL | 3.2E2 | 4.8E2 | 7.5E2 | 1.4E3 | 1.3E3 | 3.1E3 | 1.0E-9 | 1.0E-9 | 9.8E3 | 2.1E4 | 121 | 50 | 98 | 50 | 0.57 |
| Ss | pg/mL | 6.2E4 | 1.6E5 | 1.2E5 | 1.7E5 | 1.4E5 | 1.7E5 | 9.5E3 | 7.1E3 | 7.1E5 | 8.5E5 | 121 | 50 | 98 | 50 | 0.60 |
| St | pg/mL | 1.7E7 | 4.2E7 | 4.0E7 | 9.1E7 | 5.3E7 | 2.4E8 | 7.8E5 | 1.0E-9 | 4.1E8 | 1.7E9 | 119 | 49 | 96 | 49 | 0.61 |
| Wc | ng/ml | 1.0E-9 | 1.9E-2 | 6.2E-2 | 1.3E-1 | 1.6E-1 | 4.0E-1 | 1.0E-9 | 1.0E-9 | 9.8E-1 | 1.8E0 | 55 | 19 | 43 | 19 | 0.56 |
| Wd | ng/ml | 8.8E0 | 1.2E1 | 2.5E1 | 1.2E2 | 7.8E1 | 2.8E2 | 1.0E-9 | 1.5E0 | 5.4E2 | 1.2E3 | 55 | 19 | 43 | 19 | 0.63 |
| We | ng/ml | 2.6E-1 | 7.7E-1 | 6.0E-1 | 3.6E0 | 9.0E-1 | 6.8E0 | 1.0E-9 | 1.0E-9 | 3.9E0 | 2.3E1 | 55 | 19 | 43 | 19 | 0.67 |
| Wg | ng/ml | 1.0E-9 | 1.0E-9 | 2.9E-4 | 2.8E-2 | 2.2E-3 | 1.2E-1 | 1.0E-9 | 1.0E-9 | 1.6E-2 | 5.3E-1 | 55 | 19 | 43 | 19 | 0.52 |
| Wh | ng/ml | 8.7E-3 | 1.6E-2 | 3.4E-2 | 3.3E-1 | 1.1E-1 | 1.0E0 | 1.0E-9 | 1.0E-9 | 7.6E-1 | 4.5E0 | 55 | 19 | 43 | 19 | 0.67 |
| Wf | ng/ml | 1.0E-9 | 1.7E-2 | 2.0E-1 | 2.6E-1 | 6.5E-1 | 5.8E-1 | 1.0E-9 | 1.0E-9 | 4.6E0 | 2.3E0 | 55 | 19 | 43 | 19 | 0.58 |
| Ra | pg/ml | 1.0E-9 | 1.0E-9 | 6.8E-1 | 1.8E0 | 1.3E0 | 9.0E0 | 1.0E-9 | 1.0E-9 | 7.3E0 | 6.4E1 | 122 | 50 | 98 | 50 | 0.49 |
| Qz | pg/ml | 1.1E1 | 9.4E0 | 7.1E1 | 3.5E1 | 1.1E2 | 5.8E1 | 1.0E-9 | 1.0E-9 | 7.1E2 | 2.6E2 | 122 | 50 | 98 | 50 | 0.41 |
| Qy | pg/ml | 3.9E-1 | 6.4E-1 | 3.6E0 | 1.2E1 | 1.6E1 | 4.8E1 | 1.0E-9 | 1.0E-9 | 1.6E2 | 3.2E2 | 122 | 50 | 98 | 50 | 0.58 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|-----|--------|-----|-----|
| | | NonDis | Dis | NonDis | Dis | NonDis | Dis | NonDis | Dis | NonDis | Dis | NonDis | Dis | NonDis | Dis | |
| Qx | pg/ml | 1.0E-9 | 1.0E-9 | 5.6E0 | 3.8E0 | 4.9E1 | 1.6E1 | 1.0E-9 | 1.0E-9 | 5.4E2 | 1.1E2 | 122 | 50 | 98 | 50 | 0.52 |
| Qw | pg/ml | 9.0E-2 | 1.0E-9 | 2.2E0 | 2.9E0 | 1.1E1 | 1.1E1 | 1.0E-9 | 1.0E-9 | 1.2E2 | 5.6E1 | 122 | 50 | 98 | 50 | 0.43 |
| Qv | pg/ml | 2.8E4 | 1.5E4 | 3.9E4 | 2.5E4 | 7.4E4 | 2.5E4 | 1.2E3 | 4.0E2 | 7.4E5 | 1.2E5 | 122 | 50 | 98 | 50 | 0.38 |
| Qu | pg/ml | 1.7E0 | 2.1E1 | 8.6E1 | 7.5E1 | 1.8E2 | 1.4E2 | 1.0E-9 | 1.0E-9 | 8.0E2 | 7.0E2 | 122 | 50 | 98 | 50 | 0.56 |
| Qt | pg/ml | 9.9E0 | 1.5E1 | 3.6E1 | 6.3E1 | 7.7E1 | 1.6E2 | 1.0E-9 | 1.0E-9 | 4.1E2 | 1.0E3 | 122 | 50 | 98 | 50 | 0.57 |
| Qh | ng/ml | 1.3E1 | 2.2E1 | 3.5E1 | 3.9E1 | 7.0E1 | 5.5E1 | 1.2E-1 | 7.8E-1 | 6.4E2 | 3.4E2 | 122 | 50 | 98 | 50 | 0.59 |
| Qg | ng/ml | 9.0E0 | 6.0E0 | 1.7E1 | 1.5E1 | 2.5E1 | 3.1E1 | 1.5E-1 | 3.3E-1 | 1.8E2 | 2.0E2 | 122 | 50 | 98 | 50 | 0.41 |
| Jj | ng/ml | 7.1E2 | 4.2E2 | 2.7E3 | 1.0E3 | 1.9E4 | 1.9E3 | 2.3E0 | 1.2E1 | 3.4E5 | 1.2E4 | 360 | 90 | 190 | 90 | 0.40 |
| Jk | ng/ml | 3.0E0 | 3.2E0 | 2.1E1 | 2.5E1 | 4.6E1 | 4.7E1 | 1.0E-9 | 4.3E-2 | 2.8E2 | 2.4E2 | 360 | 90 | 190 | 90 | 0.54 |
| Jl | ng/ml | 3.4E-1 | 5.8E-1 | 1.9E0 | 1.1E2 | 4.7E0 | 1.0E3 | 7.6E-4 | 1.9E-3 | 3.2E1 | 9.9E3 | 360 | 90 | 190 | 90 | 0.59 |
| Jm | ng/ml | 1.5E1 | 2.3E1 | 5.3E1 | 5.7E1 | 1.2E2 | 1.0E2 | 1.0E-9 | 2.3E-1 | 1.4E3 | 6.1E2 | 360 | 90 | 190 | 90 | 0.54 |
| Jn | pg/ml | 3.2E-1 | 5.5E-1 | 3.2E0 | 1.6E1 | 3.3E1 | 1.0E2 | 1.0E-9 | 1.0E-9 | 6.2E2 | 7.3E2 | 360 | 90 | 190 | 90 | 0.57 |
| Jo | pg/ml | 3.5E3 | 3.9E3 | 4.8E3 | 6.5E3 | 3.9E3 | 1.1E4 | 2.0E1 | 2.3E2 | 2.0E4 | 1.0E5 | 360 | 90 | 190 | 90 | 0.54 |
| Jp | pg/ml | 6.3E4 | 8.1E4 | 6.6E4 | 7.9E4 | 3.7E4 | 3.4E4 | 5.8E2 | 3.1E3 | 3.0E5 | 2.1E5 | 360 | 90 | 190 | 90 | 0.64 |
| Jq | pg/ml | 9.1E1 | 1.2E2 | 1.4E2 | 3.3E2 | 1.8E2 | 9.9E2 | 1.0E0 | 7.4E0 | 2.0E3 | 8.7E3 | 360 | 90 | 190 | 90 | 0.58 |
| Jr | pg/ml | 3.3E0 | 6.7E0 | 4.5E1 | 1.6E2 | 5.6E2 | 9.4E2 | 1.0E-9 | 1.0E-9 | 1.1E4 | 7.4E3 | 360 | 90 | 190 | 90 | 0.58 |
| Js | pg/ml | 1.2E1 | 1.4E1 | 6.5E1 | 9.5E1 | 5.4E2 | 4.4E2 | 1.0E-9 | 4.5E-1 | 1.0E4 | 3.0E3 | 360 | 90 | 190 | 90 | 0.55 |
| Jt | pg/ml | 2.2E3 | 2.9E3 | 2.8E3 | 4.3E3 | 2.2E3 | 6.1E3 | 2.2E1 | 1.9E2 | 2.2E4 | 5.2E4 | 360 | 90 | 190 | 90 | 0.60 |
| Xa | pg/ml | 1.0E-9 | 8.7E0 | 7.0E0 | 8.2E1 | 1.5E1 | 2.8E2 | 1.0E-9 | 1.0E-9 | 7.6E1 | 1.2E3 | 55 | 19 | 44 | 19 | 0.69 |
| Ye | pg/ml | 1.0E-9 | 1.0E-9 | 3.9E0 | 7.7E0 | 1.3E1 | 1.6E1 | 1.0E-9 | 1.0E-9 | 6.9E1 | 6.1E1 | 55 | 19 | 44 | 19 | 0.56 |
| Tm | pg/ml | 1.0E-9 | 1.0E-9 | 2.4E0 | 3.5E0 | 4.8E0 | 6.3E0 | 1.0E-9 | 1.0E-9 | 1.8E1 | 2.5E1 | 55 | 19 | 44 | 19 | 0.54 |
| Tl | pg/ml | 1.1E-1 | 4.7E-1 | 2.3E-1 | 1.6E0 | 3.6E-1 | 5.6E0 | 1.0E-9 | 1.0E-9 | 1.8E0 | 2.5E1 | 55 | 19 | 44 | 19 | 0.63 |
| Ju | mIU/ml | 7.2E0 | 1.0E1 | 2.0E1 | 1.5E1 | 3.4E1 | 1.9E1 | 6.5E-2 | 1.9E-1 | 2.3E2 | 9.3E1 | 122 | 51 | 99 | 51 | 0.55 |
| Jv | mIU/ml | 9.3E0 | 1.5E1 | 3.1E1 | 3.1E1 | 6.1E1 | 4.7E1 | 1.0E-2 | 2.4E-1 | 4.4E2 | 2.2E2 | 122 | 51 | 99 | 51 | 0.55 |
| Jy | ng/ml | 1.7E-3 | 1.6E-3 | 2.3E-3 | 2.8E-3 | 4.7E-3 | 5.7E-3 | 1.0E-9 | 4.5E-4 | 5.2E-2 | 4.1E-2 | 122 | 51 | 99 | 51 | 0.51 |
| Kc | pg/ml | 2.1E1 | 3.4E1 | 3.9E1 | 5.4E1 | 4.4E1 | 5.0E1 | 1.0E-9 | 1.0E-9 | 1.7E2 | 1.8E2 | 121 | 53 | 98 | 53 | 0.64 |
| Kd | pg/ml | 1.0E-9 | 1.0E-9 | 1.9E2 | 9.6E2 | 6.7E2 | 5.3E3 | 1.0E-9 | 1.0E-9 | 4.8E3 | 3.8E4 | 121 | 53 | 98 | 53 | 0.52 |
| Ke | pg/ml | 9.5E3 | 1.4E4 | 1.3E4 | 2.5E4 | 9.6E3 | 4.6E4 | 3.4E2 | 6.7E2 | 5.9E4 | 3.2E5 | 121 | 53 | 98 | 53 | 0.63 |
| Kf | pg/mL | 5.5E0 | 8.9E0 | 6.3E0 | 1.0E1 | 5.7E0 | 1.1E1 | 1.0E-9 | 1.0E-9 | 2.6E1 | 7.8E1 | 121 | 53 | 98 | 53 | 0.64 |
| Kg | pg/mL | 9.9E2 | 1.4E3 | 1.7E3 | 2.7E3 | 2.4E3 | 4.5E3 | 7.7E1 | 1.3E2 | 2.2E4 | 2.7E4 | 121 | 53 | 98 | 53 | 0.57 |
| Ki | pg/ml | 6.5E1 | 4.9E1 | 7.2E1 | 6.1E1 | 5.2E1 | 4.5E1 | 1.0E-9 | 1.0E-9 | 2.9E2 | 2.1E2 | 121 | 53 | 98 | 53 | 0.41 |
| Kj | pg/ml | 9.3E2 | 1.3E3 | 1.5E3 | 2.0E3 | 1.6E3 | 2.5E3 | 6.6E1 | 1.2E2 | 1.0E4 | 1.5E4 | 121 | 53 | 98 | 53 | 0.56 |
| Kk | pg/ml | 6.8E0 | 7.8E0 | 1.2E1 | 1.4E1 | 1.8E1 | 1.6E1 | 1.0E-9 | 1.0E-9 | 1.6E2 | 6.1E1 | 121 | 53 | 98 | 53 | 0.54 |
| Kl | pg/ml | 1.7E4 | 2.8E4 | 2.4E4 | 3.2E4 | 2.3E4 | 2.7E4 | 3.5E2 | 2.4E2 | 1.1E5 | 1.3E5 | 121 | 53 | 98 | 53 | 0.60 |
| Kn | pg/ml | 1.5E1 | 3.0E1 | 5.4E1 | 1.6E2 | 1.1E2 | 6.7E2 | 1.0E-9 | 1.0E-9 | 7.3E2 | 4.9E3 | 121 | 53 | 98 | 53 | 0.61 |
| Ko | pg/ml | 3.0E2 | 5.0E2 | 3.9E2 | 7.4E2 | 4.0E2 | 8.2E2 | 1.0E-9 | 1.0E-9 | 1.8E3 | 4.1E3 | 121 | 53 | 98 | 53 | 0.65 |
| Kp | pg/ml | 2.7E2 | 3.5E2 | 3.2E2 | 6.4E2 | 2.9E2 | 1.8E3 | 1.0E-9 | 1.0E-9 | 1.7E3 | 1.3E4 | 121 | 53 | 98 | 53 | 0.61 |
| Kq | pg/ml | 2.8E2 | 4.7E2 | 4.2E2 | 3.7E3 | 9.3E2 | 2.2E4 | 5.1E0 | 1.6E0 | 9.8E3 | 1.6E5 | 117 | 53 | 95 | 53 | 0.67 |
| Kr | pg/ml | 3.6E-1 | 2.9E-1 | 2.4E0 | 1.0E1 | 4.8E0 | 5.7E1 | 1.0E-9 | 1.0E-9 | 3.5E1 | 4.2E2 | 117 | 53 | 95 | 53 | 0.52 |
| Ks | pg/ml | 1.5E4 | 1.7E4 | 2.0E4 | 2.0E4 | 1.9E4 | 1.7E4 | 3.8E2 | 5.1E1 | 1.1E5 | 6.3E4 | 117 | 53 | 95 | 53 | 0.50 |
| Ps | ng/ml | 1.4E2 | 2.9E2 | 5.2E2 | 6.6E2 | 1.6E3 | 9.2E2 | 1.6E0 | 5.5E0 | 9.0E3 | 3.8E3 | 53 | 19 | 41 | 19 | 0.68 |
| Kx | ng/ml | 1.0E-9 | 3.3E-3 | 4.4E-3 | 9.9E-3 | 8.5E-3 | 1.8E-2 | 1.0E-9 | 1.0E-9 | 5.1E-2 | 9.5E-2 | 120 | 53 | 98 | 53 | 0.60 |
| Ky | ng/ml | 7.7E-2 | 1.8E-1 | 3.2E-1 | 4.3E-1 | 6.9E-1 | 7.1E-1 | 1.0E-9 | 1.0E-9 | 5.1E0 | 3.7E0 | 120 | 53 | 98 | 53 | 0.61 |
| Kz | ng/ml | 1.0E-9 | 1.0E-9 | 4.7E-3 | 4.1E-3 | 6.8E-3 | 5.8E-3 | 1.0E-9 | 1.0E-9 | 3.6E-2 | 1.8E-2 | 120 | 53 | 98 | 53 | 0.50 |
| Rz | ng/ml | 1.4E-1 | 3.3E-1 | 8.8E-1 | 1.1E0 | 1.5E0 | 1.9E0 | 3.6E-3 | 4.6E-3 | 6.7E0 | 6.5E0 | 53 | 19 | 42 | 19 | 0.61 |
| Ry | ng/ml | 1.6E-2 | 2.7E-2 | 1.9E-2 | 4.5E-2 | 1.8E-2 | 7.9E-2 | 1.0E-9 | 1.0E-9 | 1.0E-1 | 3.5E-1 | 53 | 19 | 42 | 19 | 0.60 |
| Rx | ng/ml | 3.5E-5 | 1.0E-9 | 2.1E-3 | 8.9E-4 | 3.8E-3 | 1.8E-3 | 1.0E-9 | 1.0E-9 | 2.0E-2 | 6.1E-3 | 53 | 19 | 42 | 19 | 0.41 |
| Ld | pg/ml | 1.0E-9 | 7.5E-1 | 3.6E0 | 3.5E0 | 9.5E0 | 5.6E0 | 1.0E-9 | 1.0E-9 | 6.9E1 | 2.3E1 | 119 | 53 | 97 | 53 | 0.56 |
| Lh | pg/ml | 1.0E4 | 1.6E4 | 1.8E4 | 3.3E4 | 2.6E4 | 5.9E4 | 1.0E-9 | 1.0E-9 | 2.6E5 | 4.1E5 | 359 | 90 | 190 | 90 | 0.62 |
| Li | pg/ml | 2.5E3 | 5.0E3 | 1.3E4 | 2.3E4 | 7.2E4 | 5.1E4 | 1.0E-9 | 1.3E1 | 1.3E6 | 3.1E5 | 359 | 90 | 190 | 90 | 0.62 |
| Lj | pg/ml | 1.7E3 | 4.5E3 | 1.4E4 | 2.3E4 | 5.1E4 | 6.1E4 | 1.0E-9 | 3.4E1 | 4.4E5 | 3.9E5 | 359 | 90 | 190 | 90 | 0.59 |
| Lp | pg/ml | 9.4E0 | 1.4E1 | 5.6E1 | 1.6E2 | 1.6E2 | 2.8E2 | 1.0E-9 | 1.0E-9 | 1.1E3 | 1.0E3 | 53 | 19 | 42 | 19 | 0.53 |
| Lt | pg/ml | 1.0E-9 | 1.0E-9 | 2.6E0 | 1.0E-9 | 9.6E0 | 0.0E0 | 1.0E-9 | 1.0E-9 | 6.0E1 | 1.0E-9 | 53 | 19 | 42 | 19 | 0.45 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Rv | ng/ml | 2.2E-4 | 1.1E-3 | 1.1E-3 | 2.6E-3 | 1.8E-3 | 4.4E-3 | 1.0E-9 | 1.0E-9 | 9.2E-3 | 1.6E-2 | 53 | 19 | 41 | 19 | 0.62 |
| Ru | ng/ml | 1.0E-9 | 1.0E-9 | 8.1E-3 | 3.7E-2 | 4.5E-2 | 1.1E-1 | 1.0E-9 | 1.0E-9 | 3.1E-1 | 3.6E-1 | 53 | 19 | 41 | 19 | 0.54 |
| Rt | ng/ml | 6.2E-2 | 1.0E-1 | 9.3E-2 | 5.3E-1 | 1.1E-1 | 1.7E0 | 1.6E-3 | 2.2E-3 | 4.5E-1 | 7.4E0 | 53 | 19 | 41 | 19 | 0.63 |
| Yl | pg/ml | 1.4E1 | 1.3E1 | 1.9E1 | 3.0E1 | 1.7E1 | 4.9E1 | 1.0E-9 | 2.1E0 | 6.5E1 | 2.2E2 | 55 | 19 | 44 | 19 | 0.53 |
| Rm | ng/ml | 1.6E1 | 2.5E1 | 5.2E1 | 5.4E1 | 8.3E1 | 6.8E1 | 2.2E-1 | 3.9E-1 | 4.0E2 | 3.2E2 | 121 | 49 | 97 | 49 | 0.56 |
| Rh | ng/ml | 1.3E2 | 1.7E2 | 4.7E2 | 2.8E2 | 1.6E3 | 4.6E2 | 4.7E0 | 7.6E0 | 1.7E4 | 3.0E3 | 121 | 49 | 97 | 49 | 0.53 |
| Ri | ng/ml | 1.0E-9 | 1.0E-9 | 4.3E0 | 2.8E0 | 1.0E1 | 7.7E0 | 1.0E-9 | 1.0E-9 | 7.4E1 | 4.5E1 | 122 | 49 | 98 | 49 | 0.45 |
| Rg | ng/ml | 1.0E-9 | 1.0E-9 | 3.2E-2 | 9.8E-2 | 2.6E-1 | 4.4E-1 | 1.0E-9 | 1.0E-9 | 2.7E0 | 3.0E0 | 121 | 49 | 97 | 49 | 0.54 |
| Rj | ng/ml | 1.0E-9 | 1.0E-9 | 1.8E0 | 6.3E0 | 7.6E0 | 3.8E1 | 1.0E-9 | 1.0E-9 | 8.0E1 | 2.7E2 | 122 | 49 | 98 | 49 | 0.50 |
| Rf | ng/ml | 3.4E-1 | 3.9E-1 | 8.0E-1 | 9.6E-1 | 1.5E0 | 2.0E0 | 7.8E-3 | 3.5E-2 | 1.4E1 | 1.4E1 | 121 | 49 | 97 | 49 | 0.54 |
| Ql | pg/ml | 5.0E0 | 4.5E0 | 1.1E1 | 1.5E1 | 1.7E1 | 3.2E1 | 1.0E-9 | 1.0E-9 | 1.2E2 | 1.8E2 | 122 | 51 | 99 | 51 | 0.47 |
| Qm | pg/ml | 1.7E0 | 1.5E1 | 2.0E1 | 2.7E1 | 4.4E1 | 3.3E1 | 1.0E-9 | 1.0E-9 | 3.6E2 | 1.3E2 | 122 | 51 | 99 | 51 | 0.62 |
| Qn | pg/ml | 6.1E-1 | 1.0E0 | 7.2E0 | 1.0E1 | 2.4E1 | 2.9E1 | 1.0E-9 | 1.0E-9 | 2.2E2 | 2.0E2 | 122 | 51 | 99 | 51 | 0.52 |
| Nv | pg/ml | 3.0E3 | 4.7E3 | 1.1E4 | 1.4E4 | 5.8E4 | 2.5E4 | 1.0E-9 | 1.9E1 | 1.1E6 | 1.6E5 | 362 | 92 | 190 | 92 | 0.61 |
| Nw | pg/ml | 7.1E3 | 1.2E4 | 1.2E4 | 1.9E4 | 2.1E4 | 3.2E4 | 8.6E1 | 1.9E2 | 2.1E5 | 2.2E5 | 362 | 92 | 190 | 92 | 0.66 |
| Nx | pg/ml | 1.2E2 | 2.3E2 | 3.6E2 | 5.3E2 | 6.8E2 | 7.5E2 | 1.0E-9 | 1.0E-9 | 7.4E3 | 4.1E3 | 362 | 92 | 190 | 92 | 0.62 |
| Ny | pg/ml | 5.2E0 | 1.0E1 | 9.8E1 | 7.9E1 | 1.3E3 | 3.2E2 | 1.0E-9 | 1.0E-9 | 2.5E4 | 2.8E3 | 362 | 92 | 190 | 92 | 0.59 |
| Oa | pg/ml | 1.6E2 | 2.5E2 | 4.0E2 | 4.9E2 | 7.6E2 | 6.2E2 | 1.0E-9 | 1.0E-9 | 4.8E3 | 2.4E3 | 122 | 51 | 99 | 51 | 0.57 |
| Op | pg/ml | 4.1E5 | 4.5E5 | 3.9E5 | 4.3E5 | 1.6E5 | 1.6E5 | 3.3E4 | 9.4E4 | 7.3E5 | 6.8E5 | 53 | 19 | 42 | 19 | 0.58 |
| Wn | ng/ml | 1.2E1 | 1.4E1 | 9.2E1 | 1.8E2 | 3.0E2 | 6.0E2 | 2.2E0 | 1.5E0 | 1.8E3 | 2.4E3 | 35 | 15 | 29 | 15 | 0.52 |
| Tk | ng/ml | 1.3E2 | 9.5E1 | 3.8E2 | 2.7E2 | 7.3E2 | 4.5E2 | 1.9E1 | 1.0E1 | 4.2E3 | 1.4E3 | 37 | 17 | 30 | 17 | 0.42 |
| Oe | pg/ml | 1.1E1 | 5.6E1 | 2.3E2 | 2.8E2 | 3.7E2 | 4.8E2 | 1.0E-9 | 1.0E-9 | 2.7E3 | 2.3E3 | 357 | 92 | 190 | 92 | 0.52 |
| Of | pg/ml | 1.6E2 | 1.9E2 | 4.0E3 | 8.8E3 | 1.5E4 | 2.5E4 | 1.0E-9 | 1.0E-9 | 1.8E5 | 1.7E5 | 360 | 92 | 190 | 92 | 0.51 |
| Og | pg/ml | 8.4E-2 | 7.0E-2 | 5.8E-1 | 2.8E-1 | 1.9E0 | 6.4E-1 | 1.0E-9 | 1.0E-9 | 2.0E1 | 3.5E0 | 360 | 92 | 190 | 92 | 0.44 |
| Oh | pg/ml | 1.9E0 | 3.6E0 | 2.2E1 | 2.3E1 | 1.2E2 | 1.2E2 | 1.0E-9 | 1.0E-9 | 1.4E3 | 1.1E3 | 360 | 92 | 190 | 92 | 0.63 |
| Oi | pg/ml | 2.0E0 | 2.9E0 | 6.3E0 | 6.8E0 | 1.1E1 | 8.8E0 | 1.0E-9 | 1.0E-9 | 8.6E1 | 4.8E1 | 360 | 92 | 190 | 92 | 0.54 |
| Ok | pg/ml | 2.9E2 | 5.6E2 | 3.9E2 | 8.9E2 | 3.7E2 | 1.2E3 | 1.3E1 | 1.5E1 | 2.8E3 | 7.8E3 | 360 | 92 | 190 | 92 | 0.68 |
| Om | pg/ml | 3.6E2 | 5.6E2 | 7.4E2 | 1.8E3 | 2.0E3 | 5.7E3 | 1.0E-9 | 1.0E-9 | 3.0E4 | 5.1E4 | 360 | 92 | 190 | 92 | 0.61 |
| On | pg/ml | 1.3E2 | 2.6E2 | 2.5E2 | 5.1E2 | 4.5E2 | 1.0E3 | 1.0E-9 | 7.6E0 | 4.5E3 | 8.5E3 | 360 | 92 | 190 | 92 | 0.64 |
| Or | pg/ml | 1.0E1 | 2.1E1 | 2.9E1 | 4.3E1 | 6.1E1 | 8.3E1 | 1.0E-9 | 1.0E-9 | 5.0E2 | 5.1E2 | 120 | 54 | 98 | 54 | 0.54 |
| Ow | pg/ml | 2.7E1 | 5.3E1 | 9.9E1 | 1.9E2 | 2.9E2 | 6.4E2 | 1.0E-9 | 1.0E-9 | 2.3E3 | 4.7E3 | 120 | 54 | 98 | 54 | 0.62 |
| Ou | pg/ml | 5.0E2 | 6.0E2 | 8.2E2 | 1.3E3 | 1.2E3 | 1.9E3 | 3.5E1 | 1.0E-9 | 9.4E3 | 8.8E3 | 120 | 54 | 98 | 54 | 0.58 |
| Ul | ng/ml | 1.0E-9 | 1.0E-9 | 1.2E0 | 2.2E0 | 4.3E0 | 9.2E0 | 1.0E-9 | 1.0E-9 | 2.9E1 | 5.6E1 | 131 | 50 | 102 | 50 | 0.50 |
| Uo | ng/ml | 1.0E-9 | 1.0E-9 | 1.0E-1 | 7.1E-2 | 2.3E-1 | 1.7E-1 | 1.0E-9 | 1.0E-9 | 1.3E0 | 6.8E-1 | 131 | 50 | 102 | 50 | 0.48 |
| Uk | ng/ml | 1.0E-9 | 1.0E-9 | 4.7E-3 | 7.9E-3 | 1.1E-2 | 2.3E-2 | 1.0E-9 | 1.0E-9 | 9.9E-2 | 1.4E-1 | 131 | 50 | 102 | 50 | 0.46 |
| Um | ng/ml | 1.0E-9 | 1.0E-9 | 4.0E-1 | 3.9E-1 | 9.8E-1 | 7.6E-1 | 1.0E-9 | 1.0E-9 | 6.8E0 | 2.7E0 | 131 | 50 | 102 | 50 | 0.50 |
| Uf | ng/ml | 4.1E-2 | 1.0E-1 | 1.0E-1 | 3.3E-1 | 1.4E-1 | 8.4E-1 | 2.7E-3 | 1.0E-3 | 7.0E-1 | 5.6E0 | 131 | 50 | 102 | 50 | 0.66 |
| Uh | ng/ml | 1.6E0 | 2.9E0 | 2.7E0 | 3.9E0 | 2.7E0 | 4.0E0 | 3.2E-2 | 4.7E-2 | 1.5E1 | 1.7E1 | 131 | 50 | 102 | 50 | 0.60 |
| Un | ng/ml | 1.7E0 | 2.3E0 | 2.0E0 | 3.0E0 | 1.3E0 | 3.5E0 | 2.0E-1 | 3.4E-1 | 7.0E0 | 2.5E1 | 131 | 50 | 102 | 50 | 0.64 |
| Ug | ng/ml | 1.5E1 | 1.2E1 | 2.7E1 | 2.8E1 | 2.7E1 | 3.7E1 | 6.9E-1 | 8.8E-1 | 1.3E2 | 1.6E2 | 131 | 50 | 102 | 50 | 0.44 |
| Ur | ng/ml | 1.7E-1 | 9.6E-2 | 1.2E0 | 6.6E-1 | 8.2E0 | 1.5E0 | 1.0E-9 | 1.0E-9 | 9.3E1 | 7.3E0 | 131 | 50 | 102 | 50 | 0.44 |
| Up | ng/ml | 1.0E-9 | 1.0E-9 | 6.9E-3 | 5.0E-2 | 3.2E-2 | 3.4E-1 | 1.0E-9 | 1.0E-9 | 3.3E-1 | 2.4E0 | 131 | 50 | 102 | 50 | 0.50 |
| Us | ng/ml | 2.6E-3 | 1.8E-3 | 2.0E-2 | 4.6E-2 | 5.4E-2 | 2.3E-1 | 1.0E-9 | 1.0E-9 | 4.0E-1 | 1.7E0 | 131 | 50 | 102 | 50 | 0.48 |
| Uv | ng/ml | 3.5E-3 | 2.7E-3 | 1.3E-2 | 1.4E-2 | 4.0E-2 | 5.8E-2 | 1.0E-9 | 1.0E-9 | 3.5E-1 | 4.1E-1 | 131 | 50 | 102 | 50 | 0.46 |
| Ut | ng/ml | 5.0E-1 | 1.2E0 | 2.3E0 | 5.0E0 | 8.5E0 | 1.2E1 | 1.0E-9 | 1.0E-9 | 7.2E1 | 6.5E1 | 131 | 50 | 102 | 50 | 0.63 |
| Uu | ng/ml | 6.3E0 | 7.9E0 | 7.3E0 | 9.0E0 | 4.7E0 | 7.1E0 | 8.1E-1 | 1.0E0 | 2.4E1 | 4.0E1 | 131 | 50 | 102 | 50 | 0.57 |
| Uw | ng/ml | 2.0E0 | 2.2E0 | 3.1E0 | 5.0E0 | 5.0E0 | 8.7E0 | 1.0E-9 | 1.0E-9 | 3.7E1 | 3.9E1 | 62 | 19 | 50 | 19 | 0.59 |
| Vb | ng/ml | 1.1E0 | 1.1E0 | 1.1E0 | 1.2E0 | 4.3E-1 | 1.3E0 | 2.5E-1 | 8.5E-2 | 2.5E0 | 6.4E0 | 62 | 19 | 50 | 19 | 0.47 |
| Vc | ng/ml | 1.0E-9 | 1.0E-9 | 1.4E-2 | 1.0E-9 | 8.4E-2 | 0.0E0 | 1.0E-9 | 1.0E-9 | 6.5E-1 | 1.0E-9 | 62 | 19 | 50 | 19 | 0.47 |
| Uy | ng/ml | 1.3E0 | 1.0E0 | 4.1E0 | 5.5E0 | 9.0E0 | 1.3E1 | 5.3E-2 | 9.0E-2 | 5.1E1 | 4.6E1 | 62 | 19 | 50 | 19 | 0.47 |
| Uz | ng/ml | 1.0E-9 | 1.0E-9 | 7.1E-3 | 1.7E0 | 5.5E-2 | 7.6E0 | 1.0E-9 | 1.0E-9 | 4.4E-1 | 3.3E1 | 62 | 19 | 50 | 19 | 0.51 |
| Ux | ng/ml | 1.2E2 | 1.7E2 | 1.7E2 | 2.0E2 | 1.4E2 | 1.2E2 | 5.8E0 | 4.5E0 | 4.8E2 | 4.7E2 | 62 | 19 | 50 | 19 | 0.58 |
| Va | ng/ml | 1.8E1 | 2.6E1 | 2.5E1 | 2.9E1 | 2.6E1 | 2.4E1 | 1.2E-1 | 3.6E-1 | 1.2E2 | 7.8E1 | 62 | 19 | 50 | 19 | 0.56 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vh | ng/ml | 5.2E-3 | 1.7E-2 | 1.3E-2 | 7.1E-2 | 1.8E-2 | 1.9E-1 | 1.0E-9 | 2.2E-3 | 1.2E-1 | 8.6E-1 | 62 | 19 | 50 | 19 | 0.73 |
| Vi | ng/ml | 2.5E-3 | 5.3E-3 | 2.3E-1 | 1.1E-1 | 1.7E0 | 4.2E-1 | 1.0E-9 | 2.8E-4 | 1.4E1 | 1.8E0 | 62 | 19 | 50 | 19 | 0.63 |
| Vj | ng/ml | 1.9E1 | 5.7E1 | 1.6E2 | 1.1E2 | 6.8E2 | 1.5E2 | 3.2E0 | 1.4E0 | 5.2E3 | 6.5E2 | 62 | 19 | 50 | 19 | 0.68 |
| Vp | ng/ml | 1.0E-9 | 1.0E-9 | 6.8E-1 | 1.0E0 | 5.0E0 | 6.9E0 | 1.0E-9 | 1.0E-9 | 5.0E1 | 4.9E1 | 131 | 50 | 102 | 50 | 0.50 |
| Vt | ng/ml | 6.1E0 | 6.7E0 | 8.2E0 | 1.3E1 | 6.9E0 | 2.4E1 | 6.0E-1 | 4.3E-1 | 3.2E1 | 1.6E2 | 131 | 50 | 102 | 50 | 0.55 |
| Vu | ng/ml | 1.0E-9 | 1.0E-9 | 1.9E0 | 2.3E0 | 5.4E0 | 4.5E0 | 1.0E-9 | 1.0E-9 | 5.1E1 | 2.4E1 | 130 | 48 | 102 | 48 | 0.55 |
| Vq | ng/ml | 1.5E2 | 2.4E2 | 7.5E3 | 4.2E2 | 6.7E4 | 4.7E2 | 2.0E-1 | 3.6E0 | 6.8E5 | 1.5E3 | 102 | 36 | 82 | 36 | 0.53 |
| Vo | ng/ml | 2.6E1 | 2.5E1 | 2.5E1 | 2.5E1 | 4.3E0 | 5.6E0 | 1.1E1 | 4.7E0 | 3.5E1 | 3.5E1 | 131 | 50 | 102 | 50 | 0.48 |
| Vs | ng/ml | 1.0E-9 | 1.0E-9 | 6.0E0 | 1.5E1 | 2.6E1 | 6.7E1 | 1.0E-9 | 1.0E-9 | 2.3E2 | 4.5E2 | 127 | 46 | 99 | 46 | 0.54 |
| Vv | ng/ml | 2.2E0 | 4.7E0 | 5.7E0 | 7.1E0 | 1.0E1 | 8.2E0 | 1.0E-9 | 1.0E-9 | 8.1E1 | 3.5E1 | 130 | 49 | 102 | 49 | 0.60 |
| Vw | ng/ml | 3.4E1 | 3.9E1 | 3.3E1 | 3.8E1 | 1.7E1 | 1.6E1 | 3.1E0 | 3.1E0 | 6.7E1 | 6.4E1 | 62 | 19 | 50 | 19 | 0.58 |
| Oy | pg/ml | 5.4E-1 | 8.7E-1 | 7.9E0 | 4.5E0 | 3.9E1 | 1.0E1 | 1.0E-9 | 1.0E-9 | 4.0E2 | 6.5E1 | 360 | 92 | 190 | 92 | 0.55 |
| Oz | pg/ml | 1.4E-3 | 1.6E-1 | 2.3E-1 | 6.1E-1 | 3.8E-1 | 2.9E0 | 1.0E-9 | 1.0E-9 | 2.6E0 | 2.8E1 | 360 | 92 | 190 | 92 | 0.57 |
| Pa | pg/ml | 3.3E-1 | 4.4E-1 | 1.3E0 | 3.6E0 | 5.6E0 | 2.4E1 | 1.0E-9 | 1.0E-9 | 8.6E1 | 2.3E2 | 360 | 92 | 190 | 92 | 0.59 |
| Pb | pg/ml | 1.0E-9 | 1.0E-9 | 1.6E0 | 7.6E-1 | 2.6E1 | 4.4E0 | 1.0E-9 | 1.0E-9 | 4.9E2 | 3.2E1 | 360 | 92 | 190 | 92 | 0.52 |
| Pc | pg/ml | 2.4E-2 | 2.4E-1 | 4.2E-1 | 8.4E-1 | 1.2E0 | 3.9E0 | 1.0E-9 | 1.0E-9 | 1.6E1 | 3.8E1 | 360 | 92 | 190 | 92 | 0.56 |
| Pd | pg/ml | 1.7E0 | 2.9E0 | 3.7E0 | 6.9E0 | 8.5E0 | 1.5E1 | 1.0E-9 | 1.0E-9 | 9.4E1 | 1.2E2 | 360 | 92 | 190 | 92 | 0.60 |
| Pe | pg/ml | 1.7E1 | 3.1E1 | 8.8E1 | 3.5E2 | 3.4E2 | 1.7E3 | 1.0E-9 | 1.0E-9 | 4.7E3 | 1.5E4 | 360 | 92 | 190 | 92 | 0.59 |
| Pf | pg/ml | 1.2E0 | 2.8E0 | 8.1E0 | 1.7E1 | 3.9E1 | 5.3E1 | 1.0E-9 | 1.0E-9 | 4.8E2 | 4.3E2 | 360 | 92 | 190 | 92 | 0.61 |
| Pg | pg/ml | 2.9E0 | 4.5E0 | 3.5E1 | 4.7E1 | 1.9E2 | 1.6E2 | 1.0E-9 | 1.0E-9 | 3.2E3 | 1.2E3 | 360 | 92 | 190 | 92 | 0.57 |
| Ph | ng/ml | 1.5E-1 | 2.4E-1 | 2.6E-1 | 4.5E-1 | 3.0E-1 | 8.1E-1 | 1.0E-9 | 1.0E-9 | 1.6E0 | 5.4E0 | 120 | 54 | 98 | 54 | 0.56 |
| Pi | ng/ml | 2.0E-1 | 2.4E-1 | 2.7E-1 | 1.8E0 | 4.1E-1 | 1.1E1 | 1.0E-9 | 1.0E-9 | 3.2E0 | 8.2E1 | 120 | 54 | 98 | 54 | 0.57 |
| Pj | ng/mL | 4.5E0 | 6.2E0 | 5.1E0 | 8.1E0 | 3.3E0 | 8.4E0 | 3.8E-2 | 4.0E-1 | 1.6E1 | 5.5E1 | 120 | 54 | 98 | 54 | 0.63 |
| Pk | ng/ml | 8.1E-3 | 1.1E-2 | 1.2E-2 | 4.8E-2 | 1.2E-2 | 2.1E-1 | 1.0E-9 | 1.0E-9 | 5.9E-2 | 1.5E0 | 120 | 54 | 98 | 54 | 0.58 |
| aA | mg/dL | 8.0E-1 | 9.9E-1 | 9.1E-1 | 1.2E0 | 4.4E-1 | 8.4E-1 | 3.0E-1 | 3.0E-1 | 4.1E0 | 4.7E0 | 1529 | 119 | 315 | 119 | 0.59 |
| aC | mg/mL | 3.2E0 | 2.2E0 | 3.4E0 | 2.4E0 | 1.5E0 | 9.7E-1 | 1.1E0 | 8.5E-1 | 8.9E0 | 5.6E0 | 262 | 56 | 110 | 56 | 0.29 |
| aD | ug/mL | 3.1E0 | 3.5E0 | 4.5E0 | 5.1E0 | 4.0E0 | 4.4E0 | 4.3E-1 | 7.7E-1 | 3.5E1 | 2.1E1 | 262 | 56 | 110 | 56 | 0.53 |
| aE | mg/mL | 5.8E-1 | 5.5E-1 | 5.8E-1 | 5.8E-1 | 1.4E-1 | 1.5E-1 | 2.1E-1 | 3.4E-1 | 1.1E0 | 1.2E0 | 262 | 56 | 110 | 56 | 0.47 |
| aF | ng/mL | 2.0E0 | 2.2E0 | 4.1E0 | 4.1E0 | 6.5E0 | 4.9E0 | 4.3E-3 | 3.7E-1 | 5.0E1 | 2.4E1 | 262 | 56 | 110 | 56 | 0.52 |
| aG | mg/mL | 1.3E-1 | 1.3E-1 | 1.6E-1 | 1.5E-1 | 9.4E-2 | 7.6E-2 | 1.7E-2 | 5.0E-2 | 5.4E-1 | 3.7E-1 | 262 | 56 | 110 | 56 | 0.48 |
| aH | ug/mL | 7.0E1 | 7.5E1 | 7.8E1 | 8.1E1 | 3.8E1 | 5.1E1 | 4.6E0 | 1.1E1 | 2.7E2 | 2.9E2 | 262 | 56 | 110 | 56 | 0.50 |
| aI | ug/mL | 1.9E2 | 1.7E2 | 1.9E2 | 1.7E2 | 6.0E1 | 5.4E1 | 2.8E1 | 4.7E1 | 3.7E2 | 2.6E2 | 262 | 56 | 110 | 56 | 0.41 |
| aJ | ug/mL | 2.4E0 | 2.4E0 | 2.9E0 | 3.9E0 | 1.9E0 | 3.8E0 | 8.5E-1 | 1.1E0 | 1.2E1 | 2.3E1 | 262 | 56 | 110 | 56 | 0.55 |
| aK | ng/mL | 1.8E0 | 1.2E0 | 2.7E0 | 2.3E0 | 2.8E0 | 3.1E0 | 2.8E-2 | 2.9E-4 | 1.8E1 | 1.8E1 | 262 | 56 | 110 | 56 | 0.41 |
| aL | mg/mL | 8.3E-1 | 7.8E-1 | 8.4E-1 | 7.8E-1 | 2.7E-1 | 2.3E-1 | 1.9E-1 | 2.7E-1 | 1.7E0 | 1.6E0 | 262 | 56 | 110 | 56 | 0.43 |
| aM | U/mL | 1.9E1 | 2.6E1 | 4.2E1 | 5.3E1 | 1.1E2 | 1.1E2 | 4.2E-2 | 4.2E-2 | 1.6E3 | 8.2E2 | 262 | 56 | 110 | 56 | 0.57 |
| aN | U/mL | 9.3E0 | 1.7E1 | 1.4E1 | 2.6E1 | 1.5E1 | 2.8E1 | 2.5E-3 | 2.5E-3 | 9.8E1 | 1.1E2 | 262 | 56 | 110 | 56 | 0.67 |
| aO | pg/mL | 2.9E1 | 5.4E1 | 3.0E2 | 3.7E2 | 8.5E2 | 7.9E2 | 6.0E-2 | 6.0E-2 | 6.6E3 | 3.9E3 | 262 | 56 | 110 | 56 | 0.57 |
| aP | ng/mL | 1.7E0 | 1.7E0 | 2.1E0 | 2.7E0 | 2.1E0 | 3.8E0 | 4.5E-1 | 8.0E-1 | 2.8E1 | 2.8E1 | 262 | 56 | 110 | 56 | 0.54 |
| aQ | ng/mL | 3.2E-1 | 2.9E-1 | 4.9E-1 | 3.6E-1 | 5.0E-1 | 3.7E-1 | 1.9E-2 | 2.0E-4 | 4.0E0 | 2.5E0 | 262 | 56 | 110 | 56 | 0.42 |
| aR | ng/mL | 1.7E0 | 2.4E0 | 2.3E0 | 3.9E0 | 2.3E0 | 5.4E0 | 1.8E-1 | 4.5E-1 | 2.1E1 | 3.4E1 | 262 | 56 | 110 | 56 | 0.62 |
| aS | ng/mL | 2.4E-1 | 3.0E-1 | 6.5E-1 | 6.3E-1 | 2.3E0 | 7.3E-1 | 4.2E-3 | 2.8E-2 | 3.3E1 | 3.1E0 | 262 | 56 | 110 | 56 | 0.59 |
| aU | pg/mL | 8.7E1 | 5.7E1 | 1.4E2 | 9.6E1 | 1.6E2 | 1.2E2 | 7.4E-2 | 7.4E-2 | 1.3E3 | 8.5E2 | 262 | 56 | 110 | 56 | 0.39 |
| aV | ng/mL | 7.0E-1 | 4.9E-1 | 1.1E0 | 9.0E-1 | 1.2E0 | 1.8E0 | 2.2E-2 | 7.6E-4 | 8.7E0 | 1.3E1 | 262 | 56 | 110 | 56 | 0.38 |
| aW | pg/mL | 1.7E1 | 2.3E1 | 1.9E1 | 2.9E1 | 2.4E1 | 5.3E1 | 7.2E-2 | 7.2E-2 | 2.4E2 | 4.2E2 | 262 | 56 | 110 | 56 | 0.66 |
| aX | ng/mL | 1.0E1 | 8.5E0 | 1.6E1 | 1.7E1 | 2.2E1 | 2.6E1 | 3.0E-1 | 6.2E-1 | 2.2E2 | 1.3E2 | 262 | 56 | 110 | 56 | 0.49 |
| aY | pg/mL | 5.3E1 | 6.5E1 | 7.3E1 | 7.7E1 | 7.0E1 | 5.7E1 | 4.1E-1 | 4.1E-1 | 4.4E2 | 3.4E2 | 262 | 56 | 110 | 56 | 0.56 |
| aZ | pg/mL | 2.3E2 | 2.3E2 | 4.4E2 | 5.2E2 | 5.6E2 | 1.2E3 | 1.7E0 | 1.7E0 | 3.4E3 | 8.3E3 | 262 | 56 | 110 | 56 | 0.50 |
| bA | ng/mL | 6.8E0 | 1.2E1 | 2.4E1 | 8.3E1 | 6.9E1 | 2.3E2 | 3.0E-2 | 3.0E-2 | 7.1E2 | 1.5E3 | 262 | 56 | 110 | 56 | 0.65 |
| bB | ng/mL | 3.1E2 | 2.8E2 | 3.4E2 | 2.8E2 | 1.6E2 | 1.6E2 | 2.1E0 | 1.2E1 | 7.4E2 | 7.2E2 | 262 | 56 | 110 | 56 | 0.40 |
| bC | ng/mL | 3.4E2 | 4.0E2 | 5.2E2 | 8.8E2 | 6.0E2 | 1.1E3 | 2.7E1 | 1.3E1 | 4.0E3 | 4.7E3 | 262 | 56 | 110 | 56 | 0.58 |
| bE | mg/mL | 5.8E0 | 5.5E0 | 6.1E0 | 5.6E0 | 2.0E0 | 2.1E0 | 9.8E-1 | 1.3E0 | 1.2E1 | 1.1E1 | 262 | 56 | 110 | 56 | 0.42 |
| bF | pg/mL | 1.9E1 | 3.0E1 | 5.7E1 | 2.5E2 | 2.6E2 | 6.4E2 | 5.0E-2 | 6.1E0 | 3.3E3 | 3.3E3 | 262 | 56 | 110 | 56 | 0.64 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bG | ng/mL | 1.7E0 | 2.0E0 | 2.9E0 | 3.0E0 | 3.3E0 | 3.9E0 | 2.2E-2 | 1.1E-1 | 2.3E1 | 2.6E1 | 262 | 56 | 110 | 56 | 0.52 |
| bH | pg/mL | 5.7E-1 | 5.7E-1 | 5.1E0 | 4.5E0 | 1.8E1 | 6.3E0 | 5.7E-1 | 5.7E-1 | 2.8E2 | 2.7E1 | 262 | 56 | 110 | 56 | 0.52 |
| bI | ng/mL | 4.0E-3 | 4.0E-3 | 6.0E-2 | 8.7E-2 | 1.5E-1 | 1.9E-1 | 4.0E-3 | 4.0E-3 | 1.3E0 | 8.8E-1 | 262 | 56 | 110 | 56 | 0.53 |
| bJ | mg/mL | 2.6E0 | 2.1E0 | 3.0E0 | 2.4E0 | 2.2E0 | 1.9E0 | 2.5E-4 | 2.5E-4 | 1.3E1 | 8.5E0 | 262 | 56 | 110 | 56 | 0.43 |
| bL | pg/mL | 4.1E0 | 3.3E0 | 8.5E0 | 8.4E0 | 1.1E1 | 9.4E0 | 4.6E-2 | 4.6E-2 | 8.0E1 | 3.2E1 | 262 | 56 | 110 | 56 | 0.49 |
| bM | mg/mL | 1.5E0 | 2.1E0 | 1.9E0 | 2.5E0 | 1.3E0 | 1.6E0 | 9.2E-3 | 1.8E-2 | 8.8E0 | 8.4E0 | 262 | 56 | 110 | 56 | 0.63 |
| bN | ng/mL | 3.1E1 | 5.9E1 | 1.3E2 | 1.1E2 | 3.1E2 | 1.4E2 | 1.4E-1 | 5.9E-1 | 1.9E3 | 7.5E2 | 262 | 56 | 110 | 56 | 0.57 |
| bO | ng/mL | 4.0E-2 | 4.0E-2 | 1.1E1 | 1.3E1 | 2.4E1 | 2.4E1 | 4.0E-2 | 4.0E-2 | 1.9E2 | 1.2E2 | 262 | 56 | 110 | 56 | 0.49 |
| bP | mg/mL | 6.3E-1 | 5.0E-1 | 8.5E-1 | 7.6E-1 | 7.0E-1 | 7.4E-1 | 4.9E-2 | 9.7E-2 | 3.8E0 | 3.5E0 | 262 | 56 | 110 | 56 | 0.44 |
| bQ | pg/mL | 1.5E1 | 2.1E1 | 2.7E1 | 9.0E1 | 5.0E1 | 3.3E2 | 1.5E-1 | 1.5E-1 | 4.8E2 | 2.4E3 | 262 | 56 | 110 | 56 | 0.60 |
| bR | ng/mL | 1.2E-2 | 1.2E-2 | 1.3E-1 | 6.2E-2 | 2.5E-1 | 1.1E-1 | 1.2E-2 | 1.2E-2 | 3.4E0 | 4.8E-1 | 262 | 56 | 110 | 56 | 0.40 |
| bS | pg/mL | 9.4E-1 | 9.4E-1 | 7.8E0 | 6.7E0 | 2.7E1 | 1.5E1 | 9.4E-1 | 9.4E-1 | 3.4E2 | 6.7E1 | 262 | 56 | 110 | 56 | 0.50 |
| bU | ng/mL | 1.6E-1 | 3.4E-2 | 2.1E-1 | 1.2E-1 | 2.3E-1 | 1.4E-1 | 1.3E-2 | 1.3E-2 | 1.8E0 | 6.2E-1 | 262 | 56 | 110 | 56 | 0.36 |
| bV | pg/mL | 4.7E2 | 5.1E2 | 5.7E2 | 6.3E2 | 7.2E2 | 4.1E2 | 1.6E2 | 2.2E2 | 1.2E4 | 2.0E3 | 262 | 56 | 110 | 56 | 0.54 |
| bW | pg/mL | 3.5E2 | 3.4E2 | 5.2E2 | 1.0E3 | 4.8E2 | 3.3E3 | 1.1E2 | 1.1E2 | 3.4E3 | 2.5E4 | 262 | 56 | 110 | 56 | 0.53 |
| bX | ng/mL | 1.8E-3 | 2.5E-5 | 3.0E-3 | 2.3E-3 | 3.7E-3 | 2.8E-3 | 2.5E-5 | 2.5E-5 | 2.0E-2 | 8.5E-3 | 262 | 56 | 110 | 56 | 0.46 |
| bZ | pg/mL | 2.5E2 | 2.7E2 | 8.0E2 | 1.0E3 | 3.3E3 | 4.1E3 | 1.5E-1 | 3.5E1 | 4.4E4 | 3.1E4 | 262 | 56 | 110 | 56 | 0.52 |
| cA | pg/mL | 6.0E-1 | 6.0E-1 | 2.0E0 | 2.8E0 | 3.5E0 | 5.3E0 | 6.0E-1 | 6.0E-1 | 1.5E1 | 2.2E1 | 262 | 56 | 110 | 56 | 0.52 |
| cB | ng/mL | 6.7E-2 | 3.4E-2 | 9.8E-2 | 4.3E-2 | 1.0E-1 | 4.5E-2 | 1.7E-3 | 1.7E-3 | 5.4E-1 | 2.1E-1 | 262 | 56 | 110 | 56 | 0.32 |
| cC | pg/mL | 4.6E1 | 4.1E1 | 4.9E1 | 4.3E1 | 3.6E1 | 2.7E1 | 1.0E0 | 1.0E0 | 3.7E2 | 9.4E1 | 262 | 56 | 110 | 56 | 0.45 |
| cD | pg/mL | 6.1E0 | 4.5E0 | 1.3E1 | 2.1E1 | 5.9E1 | 5.2E1 | 3.3E-1 | 3.3E-1 | 7.2E2 | 2.9E2 | 262 | 56 | 110 | 56 | 0.44 |
| cE | pg/mL | 3.1E1 | 7.2E1 | 1.1E2 | 2.6E2 | 2.8E2 | 5.5E2 | 1.2E-1 | 1.2E-1 | 3.1E3 | 3.1E3 | 262 | 56 | 110 | 56 | 0.61 |
| cF | pg/mL | 1.4E1 | 5.3E-1 | 2.3E1 | 9.6E0 | 3.2E1 | 2.0E1 | 5.3E-1 | 5.3E-1 | 2.2E2 | 1.3E2 | 262 | 56 | 110 | 56 | 0.34 |
| cG | pg/mL | 4.2E1 | 5.5E1 | 6.7E1 | 1.3E2 | 9.1E1 | 1.9E2 | 9.6E0 | 1.8E1 | 1.1E3 | 1.2E3 | 262 | 56 | 110 | 56 | 0.61 |
| cH | uIU/mL | 2.8E0 | 2.5E0 | 5.6E0 | 5.8E0 | 8.3E0 | 7.3E0 | 8.6E-3 | 8.6E-3 | 8.7E1 | 3.9E1 | 262 | 56 | 110 | 56 | 0.50 |
| cI | ng/mL | 5.5E0 | 4.5E0 | 1.1E1 | 1.2E1 | 1.4E1 | 1.9E1 | 1.0E-3 | 1.0E-3 | 1.0E2 | 1.2E2 | 262 | 56 | 110 | 56 | 0.45 |
| cJ | ug/mL | 7.0E1 | 5.7E1 | 1.2E2 | 9.0E1 | 1.4E2 | 1.1E2 | 4.0E0 | 5.6E0 | 9.6E2 | 6.3E2 | 262 | 56 | 110 | 56 | 0.44 |
| cK | ng/mL | 3.8E-3 | 3.8E-3 | 6.9E-2 | 1.0E-2 | 2.1E-1 | 1.8E-2 | 3.8E-3 | 3.8E-3 | 1.7E0 | 8.2E-2 | 262 | 56 | 110 | 56 | 0.45 |
| cL | pg/mL | 1.9E2 | 2.5E2 | 2.7E2 | 4.1E2 | 4.7E2 | 8.1E2 | 2.5E1 | 3.7E1 | 7.1E3 | 5.9E3 | 262 | 56 | 110 | 56 | 0.56 |
| cM | pg/mL | 2.8E2 | 2.6E2 | 3.1E2 | 2.8E2 | 1.8E2 | 2.0E2 | 2.5E1 | 4.7E1 | 1.2E3 | 1.4E3 | 262 | 56 | 110 | 56 | 0.43 |
| cN | pg/mL | 1.2E2 | 1.2E2 | 1.2E2 | 1.4E2 | 4.2E1 | 5.7E1 | 3.8E1 | 6.5E1 | 3.0E2 | 3.2E2 | 262 | 56 | 110 | 56 | 0.60 |
| cO | pg/mL | 2.3E2 | 2.4E2 | 2.9E2 | 2.9E2 | 2.0E2 | 3.1E2 | 5.4E1 | 9.9E1 | 1.7E3 | 2.4E3 | 262 | 56 | 110 | 56 | 0.48 |
| cP | ng/mL | 2.4E3 | 2.8E3 | 2.5E3 | 2.7E3 | 8.7E2 | 1.0E3 | 6.2E2 | 1.3E3 | 5.7E3 | 5.6E3 | 262 | 56 | 110 | 56 | 0.57 |
| cQ | ng/mL | 3.9E-2 | 5.4E-2 | 1.2E-1 | 1.4E-1 | 2.1E-1 | 3.1E-1 | 2.0E-3 | 2.0E-3 | 1.5E0 | 2.1E0 | 262 | 56 | 110 | 56 | 0.55 |
| cR | ng/mL | 3.2E2 | 3.5E2 | 5.4E2 | 6.2E2 | 8.7E2 | 1.1E3 | 2.3E1 | 2.0E1 | 8.9E3 | 7.7E3 | 262 | 56 | 110 | 56 | 0.51 |
| cS | ng/mL | 2.5E2 | 3.2E2 | 4.2E2 | 5.3E2 | 4.6E2 | 9.4E2 | 4.1E1 | 4.8E1 | 2.7E3 | 7.1E3 | 262 | 56 | 110 | 56 | 0.56 |
| cT | ng/mL | 2.7E1 | 4.0E1 | 6.4E1 | 1.6E2 | 1.3E2 | 3.0E2 | 4.6E0 | 4.4E0 | 1.7E3 | 1.5E3 | 262 | 56 | 110 | 56 | 0.64 |
| cU | ng/mL | 5.6E1 | 4.4E1 | 7.3E1 | 7.2E1 | 6.8E1 | 6.9E1 | 9.2E0 | 1.4E1 | 7.7E2 | 3.5E2 | 262 | 56 | 110 | 56 | 0.47 |
| cV | ng/mL | 1.6E-1 | 2.2E-1 | 4.4E-1 | 5.1E-1 | 2.9E0 | 1.3E0 | 2.5E-2 | 3.4E-2 | 4.7E1 | 8.7E0 | 262 | 56 | 110 | 56 | 0.54 |
| cW | mIU/mL | 5.1E-2 | 6.0E-2 | 2.0E-1 | 8.2E-2 | 9.3E-1 | 7.3E-2 | 3.7E-4 | 1.6E-2 | 9.7E0 | 3.9E-1 | 262 | 56 | 110 | 56 | 0.56 |
| cX | ng/mL | 9.7E-2 | 2.0E-1 | 1.3E0 | 1.4E0 | 4.3E0 | 4.3E0 | 2.3E-4 | 2.3E-4 | 2.8E1 | 2.8E1 | 262 | 56 | 110 | 56 | 0.55 |
| cY | ng/mL | 9.9E0 | 6.8E0 | 1.4E1 | 1.0E1 | 1.3E1 | 1.2E1 | 2.5E-1 | 1.7E-1 | 8.3E1 | 7.5E1 | 262 | 56 | 110 | 56 | 0.39 |
| cZ | ug/mL | 1.5E1 | 1.5E1 | 1.6E1 | 1.5E1 | 7.5E0 | 7.0E0 | 2.3E0 | 2.8E0 | 5.7E1 | 3.4E1 | 262 | 56 | 110 | 56 | 0.49 |
| dA | pg/mL | 3.3E2 | 3.2E2 | 3.6E2 | 3.6E2 | 1.8E2 | 1.8E2 | 9.0E1 | 1.4E2 | 1.3E3 | 1.1E3 | 262 | 56 | 110 | 56 | 0.48 |
| dB | ug/mL | 1.7E1 | 2.1E1 | 1.9E1 | 1.9E1 | 2.0E1 | 9.0E0 | 2.1E0 | 2.2E0 | 2.5E2 | 4.0E1 | 262 | 56 | 110 | 56 | 0.63 |
| dC | nmol/L | 3.5E1 | 3.6E1 | 4.0E1 | 3.8E1 | 1.9E1 | 1.6E1 | 7.9E0 | 1.5E1 | 1.4E2 | 9.2E1 | 262 | 56 | 110 | 56 | 0.49 |
| dD | ug/mL | 3.8E1 | 3.3E1 | 3.9E1 | 3.5E1 | 1.1E1 | 9.7E0 | 1.3E1 | 1.4E1 | 7.6E1 | 5.6E1 | 262 | 56 | 110 | 56 | 0.38 |
| dE | ng/mL | 5.1E-1 | 4.6E-1 | 6.9E-1 | 5.6E-1 | 8.1E-1 | 5.8E-1 | 8.4E-3 | 8.4E-3 | 7.2E0 | 2.4E0 | 262 | 56 | 110 | 56 | 0.46 |
| dF | ng/mL | 2.1E2 | 2.4E2 | 2.6E2 | 3.5E2 | 1.8E2 | 2.4E2 | 7.5E1 | 1.0E2 | 1.2E3 | 1.2E3 | 262 | 56 | 110 | 56 | 0.61 |
| dG | ng/mL | 1.1E1 | 1.2E1 | 1.4E1 | 1.8E1 | 1.0E1 | 1.6E1 | 2.5E0 | 5.5E0 | 8.1E1 | 8.7E1 | 262 | 56 | 110 | 56 | 0.58 |
| dH | pg/mL | 7.5E0 | 8.4E0 | 1.2E1 | 1.6E1 | 2.8E1 | 2.9E1 | 4.0E-2 | 4.0E-2 | 3.1E2 | 2.0E2 | 262 | 56 | 110 | 56 | 0.54 |
| dI | pg/mL | 4.6E-1 | 4.6E-1 | 1.9E0 | 2.3E0 | 4.8E0 | 4.5E0 | 4.6E-1 | 4.6E-1 | 4.2E1 | 2.3E1 | 262 | 56 | 110 | 56 | 0.52 |
| dJ | ng/mL | 1.9E0 | 2.0E0 | 2.2E0 | 2.3E0 | 1.1E0 | 1.6E0 | 3.2E-2 | 3.2E-2 | 5.9E0 | 6.9E0 | 262 | 56 | 110 | 56 | 0.50 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|------|--------|------|------|
| | | NonDis | Dis | NonDis | Dis | NonDis | Dis | NonDis | Dis | NonDis | Dis | NonDis | Dis | NonDis | Dis | |
| dK | uIU/mL | 2.0E0 | 1.5E0 | 2.6E0 | 2.6E0 | 2.6E0 | 3.5E0 | 2.8E-4 | 1.4E-2 | 1.6E1 | 2.2E1 | 262 | 56 | 110 | 56 | 0.48 |
| dL | ng/mL | 8.9E2 | 9.1E2 | 1.0E3 | 1.2E3 | 5.0E2 | 8.1E2 | 3.4E2 | 4.5E2 | 3.4E3 | 4.8E3 | 262 | 56 | 110 | 56 | 0.54 |
| dM | pg/mL | 9.7E2 | 9.1E2 | 1.2E3 | 1.5E3 | 1.0E3 | 1.5E3 | 3.5E2 | 4.1E2 | 1.2E4 | 9.6E3 | 262 | 56 | 110 | 56 | 0.51 |
| dN | ug/ml | 9.0E1 | 1.1E2 | 9.9E1 | 1.2E2 | 3.7E1 | 5.0E1 | 2.5E1 | 4.1E1 | 2.8E2 | 3.3E2 | 262 | 56 | 110 | 56 | 0.63 |
| dR | pg/ml | 1.8E3 | 1.2E3 | 2.5E3 | 2.2E3 | 2.4E3 | 2.6E3 | 1.9E2 | 1.4E2 | 1.5E4 | 9.4E3 | 150 | 51 | 104 | 51 | 0.41 |
| dU | pg/ml | 9.1E3 | 1.7E4 | 1.2E4 | 1.6E4 | 1.1E4 | 1.4E4 | 2.5E3 | 6.9E2 | 5.3E4 | 4.6E4 | 27 | 13 | 26 | 13 | 0.56 |
| dX | ng/ml | 5.2E-2 | 3.4E-2 | 1.1E-1 | 1.6E-1 | 1.5E-1 | 2.6E-1 | 2.6E-3 | 2.6E-3 | 7.4E-1 | 8.1E-1 | 89 | 13 | 31 | 13 | 0.47 |
| dW | ng/ml | 1.7E-1 | 2.7E-1 | 2.1E-1 | 3.3E-1 | 1.5E-1 | 2.7E-1 | 5.0E-2 | 6.8E-2 | 5.8E-1 | 8.0E-1 | 36 | 7 | 7 | 7 | 0.61 |
| eF | ng/ml | 4.0E0 | 4.4E0 | 5.2E0 | 5.6E0 | 5.6E0 | 4.2E0 | 1.2E0 | 2.1E0 | 4.6E1 | 2.9E1 | 158 | 51 | 105 | 51 | 0.60 |
| eC | pg/ml | 3.0E2 | 2.6E2 | 3.7E2 | 3.6E2 | 2.5E2 | 3.5E2 | 9.9E0 | 7.1E1 | 1.4E3 | 2.0E3 | 105 | 51 | 97 | 51 | 0.44 |
| eD | pg/ml | 2.3E2 | 1.7E2 | 7.6E2 | 2.5E2 | 1.5E3 | 2.7E2 | 5.2E-1 | 5.2E-1 | 8.3E3 | 1.5E3 | 81 | 44 | 74 | 44 | 0.38 |
| eO | ng/ml | 5.8E1 | 6.1E1 | 3.8E2 | 1.2E2 | 4.1E2 | 1.1E2 | 2.0E1 | 4.1E1 | 1.2E3 | 3.3E2 | 36 | 7 | 7 | 7 | 0.46 |
| eM | ng/ml | 3.2E0 | 2.2E0 | 4.3E0 | 6.4E0 | 4.2E0 | 9.4E0 | 7.6E-1 | 3.3E-1 | 2.7E1 | 3.9E1 | 110 | 22 | 44 | 22 | 0.46 |
| eP | ng/ml | 3.7E-3 | 5.9E-1 | 8.1E-1 | 2.8E0 | 1.9E0 | 7.5E0 | 3.7E-3 | 3.7E-3 | 1.2E1 | 2.8E1 | 89 | 13 | 31 | 13 | 0.61 |
| eT | ng/ml | 2.8E2 | 3.0E2 | 6.0E2 | 7.0E2 | 6.5E2 | 8.4E2 | 1.0E2 | 7.1E1 | 2.5E3 | 2.9E3 | 55 | 24 | 54 | 24 | 0.52 |
| eQ | pg/ml | 1.0E0 | 1.0E0 | 1.2E2 | 3.6E1 | 3.3E2 | 7.1E1 | 1.0E0 | 1.0E0 | 1.6E3 | 2.2E2 | 27 | 13 | 26 | 13 | 0.47 |
| eW | U/ml | 6.7E-3 | 6.7E-3 | 8.2E-2 | 2.6E-1 | 1.5E-1 | 5.8E-1 | 6.7E-3 | 6.7E-3 | 6.6E-1 | 1.6E0 | 36 | 7 | 7 | 7 | 0.53 |
| fA | ng/ml | 2.0E2 | 1.8E2 | 4.4E2 | 4.0E2 | 4.9E2 | 4.3E2 | 2.6E1 | 4.0E1 | 1.5E3 | 1.4E3 | 27 | 14 | 26 | 14 | 0.51 |
| eZ | ng/ml | 5.4E1 | 5.5E1 | 6.2E1 | 5.8E1 | 2.5E1 | 2.4E1 | 2.3E1 | 1.8E1 | 1.2E2 | 1.2E2 | 55 | 24 | 54 | 24 | 0.47 |
| fB | ng/ml | 6.0E2 | 5.4E2 | 6.8E2 | 5.8E2 | 2.9E2 | 2.2E2 | 1.6E2 | 2.6E2 | 1.3E3 | 1.0E3 | 28 | 14 | 27 | 14 | 0.40 |
| fN | ng/ml | 2.1E-1 | 2.1E-1 | 3.9E0 | 1.7E1 | 9.2E0 | 6.4E1 | 2.1E-1 | 2.1E-1 | 5.4E1 | 3.2E2 | 55 | 24 | 54 | 24 | 0.55 |
| fP | ng/ml | 2.3E2 | 3.6E2 | 2.5E2 | 3.4E2 | 1.4E2 | 1.8E2 | 8.4E0 | 1.8E0 | 1.0E3 | 8.2E2 | 140 | 51 | 100 | 51 | 0.67 |
| fR | ng/ml | 1.2E5 | 1.7E5 | 1.7E5 | 2.9E5 | 1.3E5 | 2.3E5 | 3.1E4 | 1.9E2 | 7.7E5 | 8.7E5 | 202 | 32 | 60 | 32 | 0.64 |
| fY | ng/ml | 2.6E2 | 2.6E2 | 2.5E2 | 2.6E2 | 9.4E1 | 1.0E2 | 6.5E1 | 3.6E1 | 4.7E2 | 4.7E2 | 55 | 24 | 54 | 24 | 0.53 |
| gC | ng/ml | 2.3E2 | 2.0E2 | 2.7E2 | 2.4E2 | 1.4E2 | 1.3E2 | 1.2E2 | 8.3E1 | 1.1E3 | 5.9E2 | 84 | 15 | 49 | 15 | 0.41 |
| gL | pg/ml | 6.3E4 | 6.6E4 | 7.0E4 | 7.5E4 | 3.5E4 | 3.6E4 | 1.4E4 | 2.7E4 | 2.0E5 | 2.2E5 | 150 | 51 | 104 | 51 | 0.56 |
| gP | U/ml | 2.7E2 | 2.8E2 | 2.8E2 | 2.9E2 | 9.5E1 | 1.5E2 | 8.5E1 | 1.2E1 | 8.0E2 | 1.1E3 | 157 | 51 | 105 | 51 | 0.53 |
| gW | ng/ml | 8.5E2 | 5.1E2 | 1.5E3 | 1.0E3 | 1.9E3 | 1.4E3 | 3.7E1 | 3.1E-1 | 9.5E3 | 5.4E3 | 130 | 39 | 96 | 39 | 0.39 |
| gV | ng/ml | 2.1E1 | 1.7E1 | 2.2E1 | 1.9E1 | 7.5E0 | 9.9E0 | 2.9E-3 | 8.1E-2 | 3.9E1 | 3.4E1 | 77 | 10 | 19 | 10 | 0.42 |
| tF | pg/mL | 1.6E3 | 1.1E3 | 2.0E4 | 6.7E3 | 5.4E4 | 1.9E4 | 1.2E1 | 1.8E1 | 3.2E5 | 1.2E5 | 105 | 50 | 97 | 50 | 0.43 |
| gZ | ug/ml | 8.0E-1 | 8.8E-1 | 5.4E1 | 5.4E1 | 1.2E2 | 1.2E2 | 8.7E-2 | 1.1E-1 | 4.1E2 | 4.0E2 | 27 | 13 | 26 | 13 | 0.55 |
| hA | ng/ml | 2.1E0 | 2.2E0 | 7.1E0 | 6.1E0 | 2.4E1 | 1.1E1 | 1.7E-2 | 1.7E-2 | 1.6E2 | 6.1E1 | 81 | 45 | 74 | 45 | 0.52 |
| nM | pg/ml | 1.0E-9 | 1.0E-9 | 1.9E2 | 1.0E-9 | 1.4E3 | 0.0E0 | 1.0E-9 | 1.0E-9 | 1.0E4 | 1.0E-9 | 53 | 34 | 51 | 34 | 0.48 |
| nN | pg/ml | 1.0E3 | 2.6E3 | 1.8E3 | 1.1E4 | 2.6E3 | 4.5E4 | 1.1E2 | 2.3E2 | 1.7E4 | 2.7E5 | 53 | 34 | 51 | 34 | 0.74 |
| nO | pg/ml | 3.2E1 | 3.6E1 | 4.9E1 | 5.1E1 | 4.9E1 | 6.2E1 | 5.5E0 | 4.0E0 | 2.4E2 | 3.1E2 | 53 | 34 | 51 | 34 | 0.48 |
| nR | pg/ml | 1.3E1 | 2.0E1 | 2.9E1 | 7.0E1 | 4.6E1 | 1.8E2 | 1.0E-9 | 1.0E0 | 2.6E2 | 1.1E3 | 53 | 34 | 51 | 34 | 0.61 |
| nT | pg/ml | 1.1E2 | 7.3E1 | 3.7E2 | 1.1E2 | 1.2E3 | 1.3E2 | 1.0E-9 | 1.0E-9 | 6.6E3 | 6.9E2 | 53 | 34 | 51 | 34 | 0.45 |
| nU | pg/ml | 1.4E1 | 4.5E1 | 5.3E1 | 8.3E1 | 2.3E1 | 1.0E2 | 1.0E-9 | 1.0E-9 | 1.3E4 | 4.3E2 | 53 | 34 | 51 | 34 | 0.57 |
| lW | pg/ml | 1.0E-9 | 1.0E-9 | 1.5E1 | 1.8E1 | 5.5E1 | 5.6E1 | 1.0E-9 | 1.0E-9 | 3.9E2 | 2.9E2 | 53 | 34 | 51 | 34 | 0.51 |
| lX | pg/ml | 1.1E3 | 7.5E2 | 1.1E3 | 8.9E2 | 5.5E2 | 5.5E2 | 2.3E2 | 1.3E2 | 2.6E3 | 2.5E3 | 53 | 34 | 51 | 34 | 0.38 |
| lY | pg/ml | 1.8E1 | 1.9E1 | 2.2E1 | 2.0E1 | 2.3E1 | 1.5E1 | 1.0E-9 | 1.0E-9 | 1.4E2 | 5.9E1 | 53 | 34 | 51 | 34 | 0.51 |
| mE | pg/ml | 1.0E-9 | 1.0E-9 | 3.6E0 | 1.5E0 | 1.0E1 | 3.0E0 | 1.0E-9 | 1.0E-9 | 5.8E1 | 1.6E1 | 53 | 34 | 51 | 34 | 0.52 |
| mF | pg/ml | 1.0E-9 | 9.3E-2 | 1.8E0 | 9.0E0 | 3.3E0 | 4.2E1 | 1.0E-9 | 1.0E-9 | 1.5E1 | 2.5E2 | 53 | 34 | 51 | 34 | 0.53 |
| mH | pg/ml | 4.4E0 | 3.2E0 | 5.6E0 | 4.4E0 | 5.2E0 | 5.6E0 | 2.3E-1 | 4.0E-1 | 3.2E1 | 3.2E1 | 53 | 34 | 51 | 34 | 0.39 |
| mI | pg/ml | 1.0E-9 | 3.7E0 | 1.4E1 | 1.2E1 | 3.1E1 | 1.6E1 | 1.0E-9 | 1.0E-9 | 1.6E2 | 6.1E1 | 53 | 34 | 51 | 34 | 0.55 |
| mM | pg/ml | 1.8E1 | 3.5E1 | 4.3E1 | 1.0E2 | 5.9E1 | 1.9E2 | 1.0E-9 | 1.0E-9 | 2.3E2 | 9.8E2 | 53 | 34 | 51 | 34 | 0.59 |
| mP | pg/ml | 1.4E1 | 1.5E1 | 1.8E1 | 2.1E1 | 2.2E1 | 3.0E1 | 1.0E-9 | 1.6E0 | 1.5E2 | 1.9E2 | 52 | 34 | 50 | 34 | 0.56 |
| mS | pg/ml | 1.8E3 | 1.5E3 | 2.1E3 | 1.8E3 | 2.3E3 | 9.8E2 | 1.0E-9 | 1.0E-9 | 1.3E4 | 4.2E3 | 53 | 34 | 51 | 34 | 0.47 |
| mT | pg/ml | 5.3E1 | 7.3E1 | 1.5E2 | 9.6E1 | 2.8E2 | 1.2E2 | 9.7E0 | 1.2E1 | 1.4E3 | 6.8E2 | 52 | 34 | 50 | 34 | 0.51 |
| mU | pg/ml | 2.5E0 | 2.6E0 | 4.1E0 | 5.1E0 | 8.7E0 | 1.2E1 | 1.0E-9 | 5.5E-1 | 5.8E1 | 6.8E1 | 52 | 34 | 50 | 34 | 0.55 |
| mW | pg/ml | 2.0E3 | 2.1E3 | 2.6E3 | 2.6E3 | 1.7E3 | 2.0E3 | 3.1E2 | 1.0E-9 | 1.0E4 | 1.1E4 | 52 | 34 | 50 | 34 | 0.50 |
| mY | pg/ml | 4.8E2 | 6.6E2 | 9.9E2 | 8.7E2 | 1.8E3 | 1.0E3 | 1.0E-9 | 1.0E-9 | 1.1E4 | 5.6E3 | 53 | 34 | 51 | 34 | 0.57 |
| mZ | pg/ml | 1.8E2 | 2.8E2 | 3.1E2 | 5.0E2 | 2.9E2 | 5.4E2 | 1.0E-9 | 5.2E1 | 1.2E3 | 2.6E3 | 52 | 34 | 50 | 34 | 0.61 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| nA | pg/ml | 1.3E0 | 2.6E0 | 1.6E1 | 8.7E0 | 6.7E1 | 1.5E1 | 1.0E-9 | 1.0E-9 | 4.4E2 | 5.5E1 | 52 | 34 | 50 | 34 | 0.57 |
| nB | pg/ml | 2.9E2 | 3.0E2 | 3.0E2 | 3.0E2 | 1.6E2 | 1.4E2 | 3.0E1 | 3.7E1 | 7.0E2 | 6.4E2 | 53 | 34 | 51 | 34 | 0.52 |
| nC | pg/ml | 1.0E-9 | 6.1E1 | 8.3E3 | 1.3E4 | 5.0E4 | 6.6E4 | 1.0E-9 | 1.0E-9 | 3.7E5 | 3.8E5 | 53 | 34 | 51 | 34 | 0.55 |
| nD | pg/ml | 6.7E0 | 8.2E0 | 7.2E1 | 1.4E1 | 3.2E2 | 2.3E1 | 1.0E-9 | 1.0E-9 | 1.7E3 | 1.3E2 | 52 | 34 | 50 | 34 | 0.51 |
| nF | pg/ml | 1.0E-9 | 1.0E-9 | 7.7E0 | 3.6E0 | 3.9E1 | 1.0E1 | 1.0E-9 | 1.0E-9 | 2.4E2 | 3.8E1 | 53 | 34 | 51 | 34 | 0.53 |
| nH | pg/ml | 3.8E-1 | 3.5E0 | 2.1E2 | 1.0E2 | 1.4E3 | 4.5E2 | 1.0E-9 | 1.0E-9 | 1.0E4 | 2.6E3 | 52 | 34 | 50 | 34 | 0.58 |
| nI | pg/ml | 4.6E1 | 4.6E1 | 2.9E2 | 8.7E1 | 1.3E3 | 1.6E2 | 1.0E-9 | 1.0E-9 | 9.2E3 | 7.9E2 | 53 | 34 | 51 | 34 | 0.47 |
| nJ | pg/ml | 1.0E-9 | 5.1E-1 | 1.0E2 | 1.4E0 | 7.1E2 | 3.0E0 | 1.0E-9 | 1.0E-9 | 5.2E3 | 1.6E1 | 53 | 34 | 51 | 34 | 0.58 |
| nK | pg/ml | 1.0E-9 | 1.0E-9 | 1.2E2 | 2.2E1 | 5.4E2 | 4.8E1 | 1.0E-9 | 1.0E-9 | 3.2E3 | 2.3E2 | 52 | 34 | 50 | 34 | 0.54 |
| nL | pg/ml | 1.0E-9 | 2.6E0 | 9.2E2 | 2.1E2 | 6.1E3 | 7.6E2 | 1.0E-9 | 1.0E-9 | 4.5E4 | 4.3E3 | 53 | 34 | 51 | 34 | 0.57 |
| hL | pg/ml | 1.8E4 | 1.7E4 | 2.4E4 | 2.0E4 | 2.2E4 | 1.4E4 | 1.0E-9 | 1.0E-9 | 1.4E5 | 6.0E4 | 55 | 24 | 54 | 24 | 0.48 |
| hO | pg/ml | 1.6E4 | 1.6E4 | 1.7E4 | 1.6E4 | 3.2E3 | 3.7E3 | 1.1E4 | 1.1E4 | 2.8E4 | 2.7E4 | 55 | 24 | 54 | 24 | 0.41 |
| hP | ng/ml | 4.4E5 | 4.4E5 | 3.5E5 | 4.2E5 | 1.8E5 | 5.3E5 | 2.3E4 | 2.5E4 | 9.0E5 | 2.8E6 | 55 | 24 | 54 | 24 | 0.48 |
| wJ | pg/ml | 1.5E5 | 1.0E5 | 1.6E5 | 1.5E5 | 6.9E4 | 1.0E5 | 3.2E4 | 3.6E4 | 3.1E5 | 4.7E5 | 54 | 24 | 53 | 24 | 0.42 |
| wK | pg/ml | 3.4E4 | 3.9E4 | 4.2E4 | 4.7E4 | 2.5E4 | 4.1E4 | 5.2E3 | 8.1E3 | 1.1E5 | 2.0E5 | 54 | 24 | 53 | 24 | 0.50 |
| wL | pg/ml | 5.8E0 | 1.6E0 | 5.6E1 | 7.5E0 | 1.3E2 | 1.0E1 | 1.0E-9 | 1.0E-9 | 8.4E2 | 3.1E1 | 54 | 24 | 53 | 24 | 0.38 |
| wP | pg/ml | 2.8E4 | 2.9E4 | 4.2E4 | 6.7E4 | 4.0E4 | 8.4E4 | 1.1E3 | 4.5E3 | 1.6E5 | 3.0E5 | 54 | 24 | 53 | 24 | 0.54 |
| wQ | pg/ml | 3.5E1 | 3.5E1 | 6.3E1 | 5.2E1 | 7.8E1 | 6.1E1 | 1.0E-9 | 1.0E-9 | 3.7E2 | 2.4E2 | 54 | 24 | 53 | 24 | 0.47 |
| hR | pg/ml | 2.9E4 | 2.6E4 | 3.0E4 | 2.5E4 | 1.2E4 | 1.0E4 | 1.8E3 | 1.0E-9 | 5.8E4 | 4.5E4 | 79 | 41 | 72 | 41 | 0.40 |
| hV | pg/ml | 4.7E2 | 3.8E2 | 5.1E2 | 4.6E2 | 2.5E2 | 4.1E2 | 1.3E2 | 1.0E-9 | 1.5E3 | 2.5E3 | 79 | 41 | 72 | 41 | 0.39 |
| hW | pg/ml | 1.5E3 | 1.7E3 | 1.8E3 | 3.0E3 | 9.5E2 | 6.1E3 | 5.7E2 | 1.0E-9 | 4.8E3 | 4.0E4 | 79 | 41 | 72 | 41 | 0.56 |
| hX | pg/ml | 9.3E2 | 8.1E2 | 1.1E3 | 9.4E2 | 1.0E3 | 4.6E2 | 3.6E2 | 2.5E0 | 8.6E3 | 2.6E3 | 79 | 41 | 72 | 41 | 0.42 |
| iA | pg/ml | 1.4E2 | 1.8E2 | 4.0E2 | 2.3E2 | 9.0E2 | 2.0E2 | 1.1E1 | 1.1E1 | 7.1E3 | 7.8E2 | 105 | 49 | 97 | 49 | 0.52 |
| iB | ng/ml | 4.9E0 | 6.1E0 | 6.3E0 | 7.5E0 | 4.7E0 | 5.9E0 | 3.7E-2 | 8.8E-2 | 1.9E1 | 2.4E1 | 81 | 45 | 74 | 45 | 0.57 |
| iC | U/ml | 2.2E-1 | 4.6E-1 | 4.4E-1 | 1.6E0 | 8.3E-1 | 5.0E0 | 1.0E-9 | 1.0E-9 | 6.4E0 | 3.2E1 | 81 | 45 | 74 | 45 | 0.66 |
| tQ | pg/ml | 1.1E3 | 1.6E3 | 1.2E3 | 1.5E3 | 5.3E2 | 6.9E2 | 2.8E2 | 5.3E2 | 2.5E3 | 3.3E3 | 53 | 23 | 52 | 23 | 0.59 |
| tT | pg/ml | 1.7E1 | 2.3E1 | 1.8E1 | 3.2E1 | 9.7E0 | 2.8E1 | 7.4E0 | 6.7E0 | 6.9E1 | 1.2E2 | 53 | 23 | 52 | 23 | 0.67 |
| tS | pg/ml | 1.1E0 | 1.1E0 | 1.4E0 | 2.3E0 | 1.4E0 | 3.0E0 | 1.0E-9 | 1.0E-9 | 8.5E0 | 1.0E1 | 53 | 23 | 52 | 23 | 0.54 |
| tX | pg/ml | 8.6E-1 | 1.2E0 | 1.1E0 | 2.4E0 | 8.6E-1 | 2.7E0 | 2.5E-2 | 3.7E-1 | 4.4E0 | 1.0E1 | 53 | 23 | 52 | 23 | 0.65 |
| tO | pg/ml | 3.9E0 | 4.4E0 | 4.7E0 | 6.3E0 | 3.1E0 | 5.0E0 | 1.0E-9 | 1.6E0 | 1.4E1 | 1.9E1 | 53 | 23 | 52 | 23 | 0.55 |
| tR | pg/ml | 2.2E-1 | 2.3E-1 | 2.9E-1 | 5.1E-1 | 2.9E-1 | 6.8E-1 | 1.0E-9 | 1.0E-9 | 1.5E0 | 2.5E0 | 53 | 23 | 52 | 23 | 0.55 |
| tU | pg/ml | 8.7E0 | 1.1E1 | 1.1E1 | 1.5E1 | 7.1E0 | 1.6E1 | 1.6E0 | 1.5E0 | 3.1E1 | 8.0E1 | 53 | 24 | 52 | 24 | 0.57 |
| tN | pg/ml | 1.7E1 | 1.9E1 | 2.1E1 | 4.0E1 | 1.4E1 | 3.9E1 | 1.0E-9 | 9.4E0 | 8.0E1 | 1.6E2 | 53 | 22 | 52 | 22 | 0.61 |
| tV | ng/ml | 4.2E2 | 9.0E2 | 5.3E2 | 9.3E2 | 5.0E2 | 6.5E2 | 1.5E2 | 2.2E2 | 2.9E3 | 3.1E3 | 54 | 24 | 53 | 24 | 0.71 |
| iH | ng/ml | 1.5E5 | 1.8E5 | 1.5E5 | 1.8E5 | 4.4E4 | 4.3E4 | 7.1E4 | 6.7E4 | 2.4E5 | 2.5E5 | 105 | 49 | 97 | 49 | 0.67 |
| iJ | ng/ml | 5.1E4 | 4.4E4 | 5.2E4 | 5.0E4 | 2.2E4 | 3.6E4 | 5.5E3 | 7.7E3 | 1.0E5 | 2.5E5 | 105 | 49 | 97 | 49 | 0.44 |
| hB | ng/ml | 4.4E-1 | 5.5E-1 | 5.0E-1 | 7.4E-1 | 3.2E-1 | 6.4E-1 | 1.0E-9 | 1.2E-1 | 1.7E0 | 3.0E0 | 105 | 49 | 97 | 49 | 0.60 |
| hC | pg/ml | 3.7E3 | 4.8E3 | 5.7E3 | 8.8E3 | 7.5E3 | 1.1E4 | 1.0E-9 | 4.5E1 | 5.5E4 | 5.7E4 | 105 | 49 | 97 | 49 | 0.59 |
| hF | ng/ml | 1.0E-9 | 1.0E-9 | 4.7E1 | 1.0E-9 | 4.0E2 | 0.0E0 | 1.0E-9 | 1.0E-9 | 4.0E3 | 1.0E-9 | 105 | 49 | 97 | 49 | 0.48 |
| hG | pg/ml | 7.0E3 | 7.6E3 | 7.3E3 | 8.3E3 | 3.1E3 | 3.8E3 | 2.8E1 | 3.3E3 | 1.8E4 | 2.0E4 | 105 | 49 | 97 | 49 | 0.56 |
| iO | ng/ml | 3.8E5 | 3.7E5 | 4.1E5 | 3.9E5 | 1.9E5 | 1.8E5 | 1.1E4 | 8.3E4 | 1.1E6 | 9.2E5 | 105 | 49 | 97 | 49 | 0.48 |
| iP | ng/ml | 6.0E4 | 4.1E4 | 5.5E4 | 7.5E4 | 3.2E4 | 1.2E5 | 1.0E-9 | 3.8E3 | 2.5E5 | 5.7E5 | 105 | 49 | 97 | 49 | 0.45 |
| iZ | ng/ml | 1.6E3 | 1.6E3 | 1.8E3 | 2.0E3 | 7.5E2 | 1.0E3 | 4.7E2 | 7.5E2 | 5.1E3 | 5.7E3 | 105 | 49 | 97 | 49 | 0.54 |
| yH | pg/ml | 1.1E3 | 1.2E3 | 1.9E3 | 3.1E3 | 2.9E3 | 5.1E3 | 1.0E-9 | 2.4E2 | 1.5E4 | 2.5E4 | 54 | 24 | 53 | 24 | 0.59 |
| yK | U/ml | 1.9E1 | 2.9E1 | 4.8E1 | 5.3E1 | 8.2E1 | 6.4E1 | 1.0E-9 | 1.0E-9 | 3.8E2 | 3.0E2 | 54 | 24 | 53 | 24 | 0.62 |
| yJ | pg/ml | 3.4E4 | 3.8E4 | 4.6E4 | 4.5E4 | 3.4E4 | 3.4E4 | 1.7E3 | 2.7E3 | 1.6E5 | 1.3E5 | 54 | 24 | 53 | 24 | 0.49 |
| yD | ng/ml | 1.5E-2 | 1.6E-2 | 1.5E-2 | 1.7E-2 | 6.9E-3 | 8.0E-3 | 1.0E-9 | 1.0E-9 | 4.3E-2 | 3.0E-2 | 54 | 24 | 53 | 24 | 0.56 |
| jB | ng/ml | 2.8E5 | 2.3E5 | 2.8E5 | 2.3E5 | 9.6E4 | 8.7E4 | 5.7E4 | 9.9E4 | 4.7E5 | 3.9E5 | 27 | 13 | 26 | 13 | 0.34 |
| wB | pg/ml | 8.1E3 | 1.1E4 | 9.5E3 | 1.4E4 | 7.2E3 | 1.0E4 | 1.7E3 | 3.1E3 | 4.1E4 | 4.2E4 | 54 | 24 | 53 | 24 | 0.62 |
| pY | pg/ml | 6.0E0 | 6.2E0 | 1.1E1 | 6.9E0 | 2.6E1 | 3.1E0 | 2.1E0 | 2.6E0 | 2.0E2 | 1.7E1 | 55 | 24 | 54 | 24 | 0.53 |
| sI | ng/ml | 5.2E-2 | 5.6E-2 | 5.9E-2 | 6.0E-2 | 4.5E-2 | 2.3E-2 | 1.6E-2 | 2.5E-2 | 2.5E-1 | 1.2E-1 | 28 | 14 | 28 | 14 | 0.58 |
| sF | mIU/mL | 5.8E0 | 5.2E0 | 1.4E1 | 1.1E1 | 2.0E1 | 1.4E1 | 1.4E-1 | 9.3E-2 | 8.1E1 | 4.5E1 | 28 | 14 | 28 | 14 | 0.48 |
| sH | mIU/mL | 2.5E0 | 3.8E0 | 5.1E0 | 4.1E0 | 7.7E0 | 4.4E0 | 1.0E-9 | 1.0E-9 | 3.2E1 | 1.7E1 | 28 | 14 | 28 | 14 | 0.53 |

| Code | Units | Median | | Average | | Standard Deviation | | Minimum | | Maximum | | Number of Samples | | Number of Patients | | AUC |
|------|-------|--------|----|---------|----|----|----|----|----|----|----|--------|-----|--------|-----|-----|
| | | NonDis | Dis | NonDis | Dis | NonDis | Dis | NonDis | Dis | NonDis | Dis | NonDis | Dis | NonDis | Dis | |
| sJ | ng/ml | 1.5E-1 | 1.5E-1 | 5.7E-1 | 1.3E-1 | 1.3E0 | 9.6E-2 | 1.0E-9 | 1.0E-9 | 5.9E0 | 3.2E-1 | 28 | 14 | 28 | 14 | 0.47 |
| rC | pg/ml | 1.9E3 | 1.1E3 | 2.3E3 | 1.6E3 | 2.2E3 | 1.5E3 | 9.3E1 | 1.9E2 | 1.5E4 | 7.3E3 | 78 | 41 | 72 | 41 | 0.39 |
| rB | pg/ml | 2.2E1 | 2.6E1 | 4.6E1 | 4.8E1 | 1.2E2 | 6.1E1 | 1.0E-9 | 1.0E-9 | 9.5E2 | 2.8E2 | 78 | 41 | 72 | 41 | 0.58 |
| zG | 2.5ng/ml | 2.2E-1 | 1.9E-1 | 4.8E-1 | 4.8E-1 | 7.5E-1 | 7.7E-1 | 1.0E-9 | 1.0E-9 | 4.4E0 | 2.7E0 | 54 | 24 | 53 | 24 | 0.47 |
| zH | 2.3mU/ml | 1.1E-1 | 8.8E-2 | 1.2E-1 | 8.8E-2 | 6.9E-2 | 3.1E-2 | 1.0E-2 | 1.9E-2 | 4.4E-1 | 1.4E-1 | 54 | 24 | 53 | 24 | 0.34 |
| zI | 2.6ng/ml | 1.9E0 | 2.2E0 | 3.4E0 | 5.7E0 | 3.6E0 | 7.3E0 | 6.3E-1 | 4.3E-1 | 1.6E1 | 2.7E1 | 54 | 24 | 53 | 24 | 0.58 |
| qA | ng/ml | 1.1E7 | 9.9E6 | 1.3E7 | 1.5E7 | 7.4E6 | 1.1E7 | 3.7E6 | 3.4E6 | 3.7E7 | 4.6E7 | 55 | 24 | 54 | 24 | 0.53 |
| qB | ng/ml | 6.1E5 | 5.3E5 | 8.3E5 | 9.2E5 | 5.9E5 | 8.4E5 | 2.1E5 | 1.9E5 | 2.9E6 | 3.8E6 | 55 | 24 | 54 | 24 | 0.48 |
| qC | ng/ml | 4.5E5 | 3.1E5 | 9.1E5 | 3.8E5 | 1.3E6 | 3.3E5 | 2.0E4 | 2.1E4 | 7.1E6 | 1.5E6 | 55 | 24 | 54 | 24 | 0.38 |
| qD | ng/ml | 1.6E7 | 1.5E7 | 2.0E7 | 1.6E7 | 1.0E7 | 5.8E6 | 1.2E6 | 4.9E6 | 5.2E7 | 2.9E7 | 55 | 24 | 54 | 24 | 0.38 |
| jD | ng/ml | 2.0E1 | 3.0E1 | 4.1E1 | 5.8E1 | 7.0E1 | 8.1E1 | 1.0E-9 | 1.0E-9 | 5.1E2 | 5.1E2 | 81 | 45 | 74 | 45 | 0.62 |
| jE | ng/ml | 1.0E-9 | 1.0E-9 | 8.5E0 | 7.8E0 | 2.1E1 | 1.5E1 | 1.0E-9 | 1.0E-9 | 1.2E2 | 5.1E1 | 81 | 45 | 74 | 45 | 0.51 |
| jF | ng/ml | 4.9E1 | 2.8E1 | 5.8E1 | 5.3E1 | 5.8E1 | 7.0E1 | 1.0E-9 | 1.0E-9 | 2.5E2 | 3.5E2 | 81 | 45 | 74 | 45 | 0.44 |
| jG | ng/ml | 4.4E3 | 4.3E3 | 4.4E3 | 4.7E3 | 1.9E3 | 2.1E3 | 7.6E2 | 6.7E2 | 8.9E3 | 1.1E4 | 81 | 45 | 74 | 45 | 0.53 |
| jH | ng/ml | 7.6E1 | 7.9E1 | 8.6E1 | 9.8E1 | 4.9E1 | 6.5E1 | 1.9E1 | 3.0E1 | 2.8E2 | 4.3E2 | 81 | 45 | 74 | 45 | 0.56 |
| jI | ng/ml | 6.3E1 | 8.2E1 | 6.9E1 | 9.4E1 | 3.5E1 | 6.8E1 | 1.9E1 | 3.1E1 | 2.5E2 | 4.4E2 | 81 | 45 | 74 | 45 | 0.65 |
| sK | pg/mL | 3.7E3 | 3.4E3 | 4.0E3 | 4.5E3 | 1.3E3 | 4.3E3 | 1.7E3 | 2.1E3 | 8.0E3 | 2.3E4 | 54 | 24 | 53 | 24 | 0.45 |
| sM | pg/mL | 7.3E4 | 8.4E4 | 7.5E4 | 9.0E4 | 2.1E4 | 4.0E4 | 3.3E4 | 4.5E4 | 1.5E5 | 2.0E5 | 54 | 24 | 53 | 24 | 0.61 |
| sO | pg/mL | 3.0E8 | 2.3E8 | 3.0E8 | 2.3E8 | 9.2E7 | 8.9E7 | 7.9E7 | 6.6E7 | 4.9E8 | 4.4E8 | 54 | 24 | 53 | 24 | 0.30 |
| wC | ng/ml | 1.6E0 | 1.4E0 | 2.2E0 | 1.7E0 | 2.3E0 | 1.1E0 | 2.5E-1 | 6.1E-2 | 1.5E1 | 4.8E0 | 54 | 24 | 53 | 24 | 0.48 |
| wD | ng/ml | 1.6E1 | 2.3E1 | 7.3E1 | 5.2E1 | 2.9E2 | 6.5E1 | 2.1E0 | 5.0E0 | 2.1E3 | 2.9E2 | 54 | 24 | 53 | 24 | 0.69 |
| wE | ng/ml | 4.9E1 | 5.0E1 | 5.3E1 | 4.7E1 | 2.5E1 | 1.8E1 | 7.0E0 | 3.2E0 | 1.4E2 | 8.4E1 | 54 | 24 | 53 | 24 | 0.44 |
| wG | ng/ml | 8.2E-2 | 3.0E-2 | 1.2E-1 | 7.2E-2 | 1.5E-1 | 1.4E-1 | 1.0E-9 | 1.0E-9 | 7.1E-1 | 6.8E-1 | 54 | 24 | 53 | 24 | 0.37 |
| wH | ng/ml | 1.9E-2 | 2.2E-2 | 1.6E-1 | 2.0E-1 | 4.9E-1 | 4.5E-1 | 1.0E-9 | 1.0E-9 | 2.9E0 | 1.9E0 | 54 | 24 | 53 | 24 | 0.53 |
| wF | ng/ml | 1.7E-1 | 1.3E-1 | 2.5E0 | 1.2E0 | 9.9E0 | 2.3E0 | 1.0E-9 | 1.0E-9 | 5.7E1 | 9.6E0 | 54 | 24 | 53 | 24 | 0.52 |
| rA | pg/ml | 2.4E1 | 2.6E1 | 3.0E1 | 3.2E1 | 2.7E1 | 2.2E1 | 1.0E-9 | 1.0E-9 | 2.0E2 | 9.6E1 | 80 | 45 | 74 | 45 | 0.53 |
| qZ | pg/ml | 4.3E1 | 4.5E1 | 1.9E2 | 7.0E2 | 1.2E3 | 2.4E3 | 2.8E-4 | 5.9E-4 | 1.0E4 | 1.0E4 | 71 | 36 | 68 | 36 | 0.53 |
| qY | pg/ml | 2.9E1 | 3.0E1 | 5.1E1 | 5.3E1 | 7.4E1 | 5.8E1 | 8.7E-1 | 2.1E0 | 5.3E2 | 2.8E2 | 80 | 45 | 74 | 45 | 0.53 |
| qX | pg/ml | 5.4E1 | 6.8E1 | 6.0E1 | 7.8E1 | 3.8E1 | 5.4E1 | 1.0E-9 | 1.0E-9 | 1.8E2 | 2.0E2 | 80 | 45 | 74 | 45 | 0.57 |
| qW | pg/ml | 9.7E0 | 7.3E0 | 1.5E1 | 1.1E1 | 2.0E1 | 1.1E1 | 1.0E-9 | 1.0E-9 | 1.2E2 | 3.9E1 | 80 | 45 | 74 | 45 | 0.43 |
| qV | pg/ml | 2.2E3 | 1.9E3 | 2.8E3 | 2.7E3 | 2.0E3 | 2.1E3 | 2.3E2 | 3.5E2 | 8.5E3 | 1.1E4 | 80 | 45 | 74 | 45 | 0.47 |
| qU | pg/ml | 5.5E1 | 5.6E1 | 1.7E2 | 1.3E2 | 2.6E2 | 1.8E2 | 1.0E-9 | 1.0E-9 | 1.4E3 | 8.0E2 | 80 | 45 | 74 | 45 | 0.50 |
| qT | pg/ml | 3.7E1 | 4.3E1 | 6.5E1 | 7.1E1 | 1.2E2 | 7.1E1 | 1.0E-9 | 1.0E-9 | 9.0E2 | 3.1E2 | 80 | 45 | 74 | 45 | 0.59 |
| qI | ng/ml | 5.4E4 | 6.5E4 | 6.2E4 | 7.0E4 | 3.1E4 | 3.4E4 | 1.1E4 | 2.5E4 | 1.6E5 | 1.6E5 | 54 | 24 | 54 | 24 | 0.58 |
| qH | ng/ml | 6.6E4 | 5.4E4 | 7.2E4 | 5.5E4 | 3.8E4 | 3.3E4 | 1.5E4 | 1.0E4 | 1.8E5 | 1.6E5 | 54 | 24 | 54 | 24 | 0.36 |
| qG | ng/ml | 1.8E5 | 1.9E5 | 1.9E5 | 1.9E5 | 5.9E4 | 5.3E4 | 5.8E4 | 1.0E5 | 3.3E5 | 2.9E5 | 54 | 24 | 54 | 24 | 0.49 |
| jK | ng/ml | 1.6E3 | 1.5E3 | 1.7E3 | 1.6E3 | 5.3E2 | 5.2E2 | 5.5E2 | 7.5E2 | 3.5E3 | 3.1E3 | 81 | 45 | 74 | 45 | 0.46 |
| jL | ng/ml | 1.7E2 | 2.1E2 | 2.5E2 | 3.4E2 | 1.9E2 | 3.3E2 | 3.6E1 | 3.7E1 | 9.6E2 | 1.7E3 | 81 | 45 | 74 | 45 | 0.58 |
| jM | ng/ml | 7.1E4 | 8.2E4 | 7.3E4 | 7.8E4 | 4.0E4 | 3.7E4 | 3.9E2 | 1.1E4 | 1.9E5 | 1.7E5 | 81 | 45 | 74 | 45 | 0.55 |
| jO | pg/ml | 2.1E5 | 2.3E5 | 2.7E5 | 2.6E5 | 1.7E5 | 1.3E5 | 5.2E4 | 9.6E4 | 1.1E6 | 5.9E5 | 81 | 45 | 74 | 45 | 0.52 |
| jP | pg/ml | 2.2E5 | 2.6E5 | 2.5E5 | 2.8E5 | 1.5E5 | 1.5E5 | 3.6E4 | 5.8E4 | 9.1E5 | 7.0E5 | 81 | 45 | 74 | 45 | 0.57 |
| jQ | pg/ml | 2.8E3 | 2.5E3 | 3.7E3 | 4.8E3 | 3.1E3 | 8.9E3 | 4.2E1 | 1.0E-9 | 1.3E4 | 5.6E4 | 81 | 45 | 74 | 45 | 0.45 |
| jR | pg/ml | 8.6E3 | 5.1E3 | 1.3E4 | 1.4E4 | 1.3E4 | 3.0E4 | 1.0E-9 | 1.0E-9 | 6.8E4 | 1.8E5 | 81 | 45 | 74 | 45 | 0.43 |
| jT | pg/ml | 1.7E5 | 1.7E5 | 1.7E5 | 1.8E5 | 6.3E4 | 7.7E4 | 6.8E4 | 8.8E4 | 3.9E5 | 4.7E5 | 81 | 45 | 74 | 45 | 0.51 |
| xA | pg/ml | 3.9E0 | 5.2E0 | 1.5E1 | 1.2E1 | 5.4E1 | 2.4E1 | 1.0E-9 | 1.0E-9 | 3.9E2 | 1.2E2 | 54 | 24 | 53 | 24 | 0.55 |
| yE | pg/ml | 7.9E1 | 7.7E1 | 8.3E1 | 8.8E1 | 4.4E1 | 4.7E1 | 1.8E1 | 1.4E1 | 3.0E2 | 2.5E2 | 54 | 24 | 53 | 24 | 0.52 |
| tM | pg/ml | 3.9E1 | 3.8E1 | 4.2E1 | 3.9E1 | 2.1E1 | 2.3E1 | 1.0E-9 | 1.0E-9 | 1.0E2 | 1.1E2 | 54 | 24 | 53 | 24 | 0.45 |
| tL | pg/ml | 1.0E-9 | 1.0E-9 | 6.9E0 | 1.3E-1 | 3.6E1 | 3.9E-1 | 1.0E-9 | 1.0E-9 | 2.6E2 | 1.8E0 | 54 | 24 | 53 | 24 | 0.45 |
| jU | mIU/ml | 3.5E0 | 4.3E0 | 1.0E1 | 5.8E0 | 1.8E1 | 5.2E0 | 8.9E-2 | 4.2E-2 | 8.1E1 | 1.8E1 | 81 | 45 | 74 | 45 | 0.50 |
| jV | mIU/ml | 1.5E0 | 1.4E0 | 3.4E0 | 3.3E0 | 5.5E0 | 5.7E0 | 3.4E-2 | 1.7E-3 | 3.1E1 | 3.5E1 | 81 | 45 | 74 | 45 | 0.48 |
| jY | ng/ml | 7.4E-4 | 1.0E-9 | 9.8E-3 | 2.9E-3 | 4.1E-2 | 4.9E-3 | 1.0E-9 | 1.0E-9 | 3.0E-1 | 2.1E-2 | 81 | 45 | 74 | 45 | 0.46 |
| kC | pg/ml | 9.7E1 | 1.2E2 | 2.3E2 | 1.8E2 | 5.6E2 | 2.0E2 | 2.9E1 | 2.1E1 | 3.5E3 | 1.1E3 | 53 | 34 | 51 | 34 | 0.57 |
| kE | pg/ml | 1.2E5 | 1.4E5 | 1.3E5 | 1.4E5 | 3.2E4 | 4.4E4 | 4.1E4 | 1.2E4 | 2.0E5 | 2.2E5 | 53 | 34 | 51 | 34 | 0.59 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| kF | pg/mL | 6.0E1 | 6.6E1 | 7.4E1 | 6.9E1 | 7.1E1 | 2.0E1 | 2.7E1 | 2.6E1 | 5.1E2 | 1.2E2 | 53 | 34 | 51 | 34 | 0.59 |
| kG | pg/mL | 8.5E3 | 9.9E3 | 9.4E3 | 1.2E4 | 7.8E3 | 1.1E4 | 7.5E2 | 1.1E3 | 4.3E4 | 5.8E4 | 53 | 34 | 51 | 34 | 0.60 |
| kI | pg/ml | 2.0E2 | 2.0E2 | 2.4E2 | 2.2E2 | 1.5E2 | 1.3E2 | 7.9E1 | 7.6E1 | 8.7E2 | 6.7E2 | 53 | 34 | 51 | 34 | 0.44 |
| kK | pg/ml | 1.0E2 | 1.4E2 | 1.6E2 | 2.4E2 | 1.9E2 | 3.5E2 | 6.4E0 | 2.1E1 | 1.2E3 | 1.9E3 | 53 | 34 | 51 | 34 | 0.58 |
| kN | pg/ml | 9.2E2 | 1.1E3 | 1.4E3 | 1.9E3 | 1.9E3 | 3.3E3 | 2.4E2 | 7.3E1 | 1.3E4 | 1.7E4 | 53 | 34 | 51 | 34 | 0.52 |
| kO | pg/ml | 7.7E3 | 7.8E3 | 1.0E4 | 8.7E3 | 1.8E4 | 4.3E3 | 3.7E3 | 3.7E3 | 1.3E5 | 2.5E4 | 53 | 34 | 51 | 34 | 0.52 |
| kP | pg/ml | 4.8E3 | 5.9E3 | 6.8E3 | 7.1E3 | 6.1E3 | 4.8E3 | 8.6E2 | 1.5E3 | 3.3E4 | 2.0E4 | 53 | 34 | 51 | 34 | 0.55 |
| kQ | pg/ml | 4.2E3 | 4.5E3 | 4.9E3 | 5.9E3 | 2.6E3 | 4.7E3 | 5.6E2 | 2.0E3 | 1.4E4 | 2.5E4 | 105 | 49 | 97 | 49 | 0.54 |
| kR | pg/ml | 2.0E1 | 2.0E1 | 3.6E1 | 2.8E1 | 1.0E2 | 2.3E1 | 1.0E-9 | 4.8E0 | 1.0E3 | 1.1E2 | 105 | 49 | 97 | 49 | 0.50 |
| kS | pg/ml | 7.6E2 | 8.8E2 | 8.9E2 | 9.2E2 | 5.2E2 | 4.9E2 | 1.3E2 | 2.6E2 | 3.2E3 | 2.5E3 | 105 | 49 | 97 | 49 | 0.53 |
| pS | ng/ml | 1.8E5 | 1.3E5 | 2.1E5 | 1.9E5 | 9.0E4 | 1.6E5 | 9.7E4 | 6.0E4 | 5.0E5 | 8.3E5 | 54 | 24 | 53 | 24 | 0.32 |
| rZ | ng/ml | 1.0E-9 | 2.4E-3 | 7.1E-3 | 1.3E-2 | 2.0E-2 | 4.5E-2 | 1.0E-9 | 1.0E-9 | 1.1E-1 | 2.9E-1 | 78 | 41 | 71 | 41 | 0.62 |
| rY | ng/ml | 5.3E-2 | 6.1E-2 | 2.2E-1 | 4.1E-1 | 8.1E-1 | 2.1E0 | 1.0E-9 | 1.0E-9 | 6.3E0 | 1.4E1 | 78 | 41 | 71 | 41 | 0.54 |
| rX | ng/ml | 1.0E-9 | 1.0E-9 | 8.4E-2 | 6.4E-2 | 4.4E-1 | 3.7E-1 | 1.0E-9 | 1.0E-9 | 3.7E0 | 2.3E0 | 78 | 41 | 71 | 41 | 0.46 |
| lK | pg/ml | 7.5E1 | 7.1E1 | 1.6E2 | 1.6E2 | 1.9E2 | 2.0E2 | 1.0E-9 | 1.0E-9 | 7.4E2 | 7.9E2 | 81 | 44 | 74 | 44 | 0.46 |
| lL | pg/ml | 1.7E3 | 1.4E3 | 2.7E3 | 2.1E3 | 3.0E3 | 1.8E3 | 7.5E1 | 8.9E1 | 1.9E4 | 7.7E3 | 81 | 45 | 74 | 45 | 0.44 |
| lM | pg/ml | 1.1E3 | 1.3E3 | 2.2E3 | 5.9E3 | 3.0E3 | 1.1E4 | 1.3E2 | 2.6E2 | 1.6E4 | 5.1E4 | 81 | 45 | 74 | 45 | 0.58 |
| lN | pg/ml | 1.0E-9 | 1.0E-9 | 6.9E0 | 2.3E0 | 2.2E1 | 3.9E0 | 1.0E-9 | 1.0E-9 | 1.5E2 | 1.3E1 | 81 | 45 | 74 | 45 | 0.46 |
| lO | pg/ml | 1.0E-9 | 1.0E-9 | 5.0E-1 | 3.4E0 | 4.5E0 | 2.1E1 | 1.0E-9 | 1.0E-9 | 4.0E1 | 1.4E2 | 81 | 44 | 74 | 44 | 0.52 |
| zA | ng/ml | 1.9E7 | 2.0E7 | 1.9E7 | 1.9E7 | 6.6E6 | 5.9E6 | 6.7E6 | 5.9E6 | 3.4E7 | 2.8E7 | 50 | 24 | 49 | 24 | 0.49 |
| rW | ng/ml | 1.7E-2 | 2.2E-2 | 2.7E-2 | 3.5E-2 | 3.2E-2 | 4.4E-2 | 1.0E-9 | 1.0E-9 | 1.7E-1 | 1.9E-1 | 54 | 23 | 54 | 23 | 0.55 |
| rV | ng/ml | 1.0E-9 | 1.0E-9 | 1.2E-2 | 2.0E-2 | 4.0E-2 | 5.7E-2 | 1.0E-9 | 1.0E-9 | 2.2E-1 | 2.4E-1 | 54 | 23 | 54 | 23 | 0.52 |
| rU | ng/ml | 1.1E-1 | 7.6E-2 | 1.8E-1 | 1.9E-1 | 2.7E-1 | 3.9E-1 | 1.0E-9 | 1.0E-9 | 1.4E0 | 1.9E0 | 54 | 23 | 54 | 23 | 0.51 |
| rT | ng/ml | 6.5E0 | 5.6E0 | 6.8E0 | 7.4E0 | 4.2E0 | 5.3E0 | 7.3E-1 | 1.0E0 | 2.1E1 | 2.0E1 | 54 | 23 | 54 | 23 | 0.51 |
| rS | ng/ml | 3.5E0 | 5.8E0 | 5.8E0 | 1.3E1 | 6.6E0 | 1.8E1 | 7.6E-1 | 1.0E0 | 3.8E1 | 7.0E1 | 54 | 23 | 54 | 23 | 0.61 |
| sC | pg/mL | 5.8E3 | 8.3E3 | 9.5E3 | 1.7E4 | 8.6E3 | 2.0E4 | 1.7E3 | 2.2E3 | 4.4E4 | 8.0E4 | 54 | 24 | 53 | 24 | 0.61 |
| yL | pg/ml | 3.2E1 | 2.7E1 | 3.9E1 | 8.4E1 | 2.8E1 | 2.8E2 | 5.6E0 | 1.2E1 | 1.8E2 | 1.4E3 | 52 | 24 | 51 | 24 | 0.34 |
| rP | ng/ml | 9.5E1 | 8.7E1 | 1.6E2 | 2.2E2 | 2.2E2 | 2.3E2 | 1.0E-9 | 1.3E0 | 1.2E3 | 7.4E2 | 54 | 23 | 54 | 23 | 0.56 |
| rQ | ng/ml | 1.0E-9 | 1.0E-9 | 3.6E0 | 7.7E-2 | 1.6E1 | 3.7E-1 | 1.0E-9 | 1.0E-9 | 8.9E1 | 1.8E0 | 54 | 23 | 54 | 23 | 0.47 |
| rO | ng/ml | 2.5E-2 | 3.5E-2 | 4.8E-2 | 5.4E-2 | 8.5E-2 | 7.2E-2 | 1.0E-9 | 1.0E-9 | 4.4E-1 | 3.3E-1 | 54 | 23 | 54 | 23 | 0.57 |
| rR | ng/ml | 3.9E0 | 3.9E0 | 2.3E1 | 1.1E1 | 6.8E1 | 2.1E1 | 1.0E-9 | 1.0E-9 | 4.5E2 | 9.5E1 | 54 | 23 | 54 | 23 | 0.49 |
| rN | ng/ml | 6.6E-1 | 6.8E-1 | 7.4E-1 | 1.1E0 | 4.4E-1 | 1.0E0 | 5.1E-2 | 2.4E-1 | 2.1E0 | 4.4E0 | 54 | 23 | 54 | 23 | 0.57 |
| qO | pg/ml | 9.8E3 | 1.1E4 | 1.3E4 | 2.0E4 | 9.6E3 | 1.7E4 | 2.2E3 | 1.9E3 | 4.6E4 | 6.4E4 | 55 | 24 | 54 | 24 | 0.60 |
| qP | pg/ml | 3.6E2 | 3.6E2 | 4.4E2 | 6.2E2 | 3.0E2 | 6.0E2 | 1.0E-9 | 1.2E2 | 1.5E3 | 2.5E3 | 55 | 24 | 54 | 24 | 0.55 |
| qQ | pg/ml | 1.5E1 | 6.3E0 | 1.7E1 | 9.9E0 | 3.8E1 | 1.2E1 | 1.0E-9 | 1.0E-9 | 2.8E2 | 4.3E1 | 55 | 24 | 54 | 24 | 0.44 |
| nW | pg/ml | 1.1E5 | 1.2E5 | 1.1E5 | 1.2E5 | 2.4E4 | 3.1E4 | 5.8E4 | 6.7E4 | 1.8E5 | 2.1E5 | 105 | 49 | 97 | 49 | 0.52 |
| nY | pg/ml | 1.9E3 | 2.0E3 | 2.2E3 | 2.6E3 | 1.4E3 | 1.7E3 | 6.5E2 | 5.7E2 | 9.9E3 | 1.0E4 | 105 | 49 | 97 | 49 | 0.55 |
| oO | pg/ml | 7.5E4 | 9.8E4 | 1.0E5 | 1.3E5 | 1.1E5 | 9.1E4 | 1.5E3 | 3.3E3 | 6.2E5 | 3.4E5 | 51 | 30 | 49 | 30 | 0.59 |
| oP | pg/ml | 1.1E5 | 1.4E5 | 1.3E5 | 1.7E5 | 7.7E4 | 1.1E5 | 2.4E4 | 2.4E4 | 3.6E5 | 4.2E5 | 51 | 30 | 49 | 30 | 0.60 |
| oQ | pg/ml | 2.5E3 | 3.1E3 | 2.9E3 | 4.3E3 | 1.7E3 | 3.4E3 | 9.3E2 | 9.1E2 | 1.0E4 | 1.7E4 | 51 | 30 | 49 | 30 | 0.63 |
| oE | pg/ml | 1.8E2 | 2.3E2 | 4.0E2 | 4.5E2 | 6.0E2 | 5.1E2 | 1.0E-9 | 1.0E-9 | 4.7E3 | 2.0E3 | 105 | 49 | 97 | 49 | 0.54 |
| oF | pg/ml | 7.4E3 | 8.2E3 | 1.7E4 | 2.3E4 | 3.0E4 | 3.0E4 | 6.4E1 | 1.8E2 | 1.7E5 | 1.4E5 | 105 | 49 | 97 | 49 | 0.59 |
| oH | pg/ml | 4.4E1 | 4.5E1 | 9.5E1 | 7.2E1 | 1.5E2 | 7.3E1 | 4.2E0 | 4.4E0 | 8.6E2 | 2.8E2 | 105 | 49 | 97 | 49 | 0.49 |
| oK | pg/ml | 6.4E2 | 1.0E3 | 2.0E3 | 1.6E3 | 3.1E3 | 1.6E3 | 5.2E1 | 1.9E2 | 1.8E4 | 7.3E3 | 105 | 49 | 97 | 49 | 0.57 |
| oN | pg/ml | 4.9E2 | 5.6E2 | 7.8E2 | 7.2E2 | 1.8E3 | 6.5E2 | 1.5E2 | 1.6E2 | 1.8E4 | 4.6E3 | 105 | 49 | 97 | 49 | 0.59 |
| oW | pg/ml | 2.1E2 | 2.2E2 | 4.8E2 | 3.3E2 | 1.1E3 | 2.4E2 | 7.7E1 | 9.0E1 | 6.0E3 | 8.5E2 | 27 | 13 | 26 | 13 | 0.54 |
| oT | pg/ml | 3.5E2 | 2.8E2 | 3.6E2 | 3.2E2 | 1.8E2 | 1.8E2 | 9.9E1 | 1.1E2 | 7.8E2 | 7.9E2 | 27 | 13 | 26 | 13 | 0.41 |
| oV | pg/ml | 1.4E2 | 9.8E1 | 2.1E2 | 4.4E2 | 2.3E2 | 8.3E2 | 1.0E-9 | 1.1E1 | 9.9E2 | 2.4E3 | 27 | 13 | 26 | 13 | 0.48 |
| oD | pg/ml | 1.7E4 | 1.4E4 | 1.9E4 | 1.4E4 | 8.2E3 | 5.7E3 | 9.3E3 | 6.6E3 | 4.6E4 | 2.5E4 | 27 | 13 | 26 | 13 | 0.30 |
| uL | ng/ml | 3.6E1 | 3.3E1 | 4.4E1 | 5.3E1 | 3.0E1 | 7.1E1 | 1.0E-9 | 1.1E1 | 1.6E2 | 3.7E2 | 52 | 24 | 51 | 24 | 0.49 |
| uO | ng/ml | 3.0E-1 | 4.2E-1 | 7.8E-1 | 7.7E-1 | 1.4E0 | 1.1E0 | 1.0E-9 | 1.0E-9 | 9.3E0 | 5.0E0 | 52 | 24 | 51 | 24 | 0.53 |
| uM | ng/ml | 6.4E-1 | 7.5E-1 | 1.1E0 | 7.9E-1 | 2.2E0 | 5.6E-1 | 1.0E-9 | 1.0E-9 | 1.3E1 | 2.5E0 | 52 | 24 | 51 | 24 | 0.52 |
| uI | ng/ml | 7.7E-2 | 8.9E-2 | 1.4E-1 | 1.1E-1 | 1.9E-1 | 8.3E-2 | 1.6E-2 | 1.5E-2 | 1.1E0 | 3.8E-1 | 52 | 24 | 51 | 24 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| uN | ng/ml | 1.5E1 | 1.3E1 | 1.7E1 | 1.6E1 | 6.7E0 | 8.3E0 | 7.7E0 | 6.4E0 | 4.1E1 | 4.1E1 | 52 | 24 | 51 | 24 | 0.43 |
| uG | ng/ml | 2.1E1 | 1.9E1 | 2.5E1 | 2.7E1 | 1.4E1 | 2.7E1 | 7.6E0 | 6.7E0 | 6.9E1 | 1.3E2 | 52 | 24 | 51 | 24 | 0.44 |
| uR | ng/ml | 2.3E0 | 2.6E0 | 3.9E0 | 3.3E0 | 8.6E0 | 3.0E0 | 9.9E-1 | 7.3E-1 | 6.4E1 | 1.4E1 | 54 | 24 | 53 | 24 | 0.51 |
| uP | ng/ml | 2.2E0 | 2.4E0 | 2.5E0 | 2.9E0 | 1.3E0 | 1.3E0 | 1.1E0 | 1.3E0 | 9.1E0 | 6.1E0 | 54 | 24 | 53 | 24 | 0.63 |
| uV | ng/ml | 2.3E-4 | 1.2E-3 | 1.6E-2 | 1.1E-2 | 4.0E-2 | 1.7E-2 | 1.0E-9 | 1.0E-9 | 2.0E-1 | 6.1E-2 | 54 | 24 | 53 | 24 | 0.51 |
| uT | ng/ml | 5.8E1 | 6.8E1 | 8.3E1 | 9.6E1 | 9.2E1 | 8.8E1 | 1.2E1 | 1.3E1 | 5.8E2 | 4.1E2 | 54 | 24 | 53 | 24 | 0.58 |
| uU | ng/ml | 1.7E0 | 1.4E0 | 2.0E0 | 2.6E0 | 1.2E0 | 3.9E0 | 5.2E-1 | 5.9E-1 | 5.6E0 | 2.0E1 | 54 | 24 | 53 | 24 | 0.44 |
| uW | ng/ml | 7.2E0 | 7.1E0 | 7.6E0 | 8.5E0 | 2.9E0 | 3.4E0 | 4.0E0 | 4.4E0 | 2.2E1 | 1.9E1 | 52 | 24 | 51 | 24 | 0.56 |
| vB | ng/ml | 2.6E0 | 3.3E0 | 2.7E0 | 3.2E0 | 1.2E0 | 1.5E0 | 6.9E-1 | 1.2E0 | 5.6E0 | 7.7E0 | 52 | 24 | 51 | 24 | 0.60 |
| vC | ng/ml | 1.0E-9 | 1.0E-9 | 3.0E-3 | 1.0E-9 | 2.0E-2 | 0.0E0 | 1.0E-9 | 1.0E-9 | 1.4E-1 | 1.0E-9 | 52 | 24 | 51 | 24 | 0.48 |
| uY | ng/ml | 7.4E-1 | 8.3E-1 | 1.2E0 | 1.2E0 | 1.1E0 | 1.1E0 | 8.7E-2 | 1.9E-1 | 4.9E0 | 4.4E0 | 52 | 24 | 51 | 24 | 0.52 |
| uZ | ng/ml | 5.8E-1 | 4.0E-1 | 8.2E-1 | 7.3E-1 | 1.1E0 | 1.0E0 | 4.7E-2 | 1.2E-1 | 7.2E0 | 4.9E0 | 52 | 24 | 51 | 24 | 0.39 |
| uX | ng/ml | 1.1E1 | 1.2E1 | 1.3E1 | 1.9E1 | 7.2E0 | 1.9E1 | 4.0E0 | 5.1E0 | 3.3E1 | 7.8E1 | 52 | 24 | 51 | 24 | 0.57 |
| vA | ng/ml | 7.4E-2 | 7.0E-2 | 8.6E-2 | 1.0E-1 | 5.8E-2 | 9.3E-2 | 2.4E-2 | 2.6E-2 | 3.0E-1 | 4.2E-1 | 52 | 24 | 51 | 24 | 0.51 |
| vH | ng/ml | 1.2E-1 | 1.1E-1 | 1.6E-1 | 2.1E-1 | 1.4E-1 | 3.7E-1 | 1.5E-2 | 1.4E-2 | 8.0E-1 | 1.9E0 | 54 | 24 | 53 | 24 | 0.48 |
| vI | ng/ml | 1.4E0 | 2.3E0 | 1.7E0 | 2.5E0 | 1.1E0 | 2.4E0 | 6.2E-3 | 6.3E-3 | 4.5E0 | 1.0E1 | 54 | 24 | 53 | 24 | 0.59 |
| vP | ng/ml | 4.3E2 | 5.1E2 | 5.1E2 | 6.3E2 | 3.9E2 | 4.9E2 | 7.0E1 | 1.3E2 | 2.0E3 | 2.2E3 | 54 | 24 | 53 | 24 | 0.58 |
| vT | ng/ml | 7.7E1 | 9.2E1 | 1.1E2 | 1.0E2 | 1.2E2 | 5.9E1 | 3.7E1 | 4.5E1 | 6.9E2 | 3.3E2 | 54 | 24 | 53 | 24 | 0.55 |
| vU | ng/ml | 1.0E-9 | 1.0E-9 | 2.3E1 | 2.5E1 | 3.5E1 | 3.6E1 | 1.0E-9 | 1.0E-9 | 1.3E2 | 1.4E2 | 54 | 24 | 53 | 24 | 0.52 |
| vQ | ng/ml | 3.5E2 | 3.4E2 | 3.6E2 | 3.8E2 | 1.5E2 | 1.4E2 | 6.7E1 | 1.9E2 | 8.1E2 | 6.5E2 | 54 | 24 | 53 | 24 | 0.53 |
| vO | ng/ml | 1.7E3 | 1.8E3 | 1.8E3 | 1.9E3 | 4.5E2 | 4.3E2 | 1.0E3 | 1.1E3 | 3.0E3 | 2.7E3 | 54 | 24 | 53 | 24 | 0.57 |
| vS | ng/ml | 1.3E3 | 1.4E3 | 1.3E3 | 1.3E3 | 3.6E2 | 5.0E2 | 1.0E-9 | 1.0E-9 | 1.8E3 | 1.9E3 | 54 | 24 | 53 | 24 | 0.58 |
| vV | ng/ml | 9.7E2 | 1.2E3 | 1.4E3 | 1.7E3 | 1.6E3 | 1.9E3 | 1.1E2 | 2.2E2 | 1.1E4 | 9.0E3 | 54 | 24 | 53 | 24 | 0.55 |
| vW | ng/ml | 1.4E2 | 1.9E2 | 1.8E2 | 2.2E2 | 1.4E2 | 1.7E2 | 4.3E1 | 6.0E1 | 6.7E2 | 7.7E2 | 54 | 24 | 53 | 24 | 0.61 |
| pF | pg/ml | 5.1E-1 | 6.1E-1 | 6.9E-1 | 9.4E-1 | 9.9E-1 | 9.0E-1 | 1.0E-9 | 1.0E-9 | 9.4E0 | 4.4E0 | 105 | 49 | 97 | 49 | 0.59 |
| pH | ng/ml | 8.9E0 | 8.9E0 | 9.4E0 | 1.3E1 | 4.5E0 | 1.1E1 | 3.4E0 | 3.0E0 | 1.8E1 | 4.7E1 | 27 | 13 | 26 | 13 | 0.58 |
| pI | ng/ml | 7.0E1 | 6.7E1 | 7.0E1 | 7.1E1 | 3.2E1 | 4.3E1 | 2.6E1 | 2.7E1 | 1.5E2 | 2.0E2 | 27 | 13 | 26 | 13 | 0.46 |
| pK | ng/ml | 4.6E-1 | 4.0E-1 | 5.2E-1 | 4.5E-1 | 2.9E-1 | 2.2E-1 | 2.3E-1 | 1.7E-1 | 1.6E0 | 8.6E-1 | 27 | 13 | 26 | 13 | 0.45 |

Figure 5.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.0E1 | 6.0E1 | 8.1E1 | 7.4E1 | 5.8E1 | 5.5E1 | 1.0E0 | 7.0E0 | 4.8E2 | 2.4E2 | 1595 | 22 | 266 | 22 | 0.46 |
| Ad | ug/mL | 3.8E-2 | 4.6E-2 | 9.4E-2 | 6.7E-2 | 4.1E-1 | 5.7E-2 | 2.7E-4 | 2.6E-3 | 8.5E0 | 1.9E-1 | 447 | 21 | 170 | 21 | 0.54 |
| Af | ng/mL | 1.2E0 | 1.2E0 | 1.8E1 | 1.7E1 | 6.7E1 | 5.1E1 | 1.7E-3 | 1.7E-3 | 5.3E2 | 2.4E2 | 447 | 21 | 170 | 21 | 0.54 |
| Aj | ug/mL | 1.4E0 | 7.6E-1 | 2.6E0 | 2.5E0 | 2.4E0 | 2.7E0 | 1.5E-3 | 1.1E-2 | 6.1E0 | 5.8E0 | 447 | 21 | 170 | 21 | 0.46 |
| Al | mg/mL | 8.7E-5 | 8.7E-5 | 2.5E-4 | 2.9E-4 | 4.1E-4 | 3.8E-4 | 2.3E-6 | 6.6E-6 | 1.9E-3 | 1.4E-3 | 447 | 21 | 170 | 21 | 0.54 |
| An | U/mL | 4.8E1 | 8.4E1 | 1.8E2 | 4.3E2 | 5.5E2 | 9.5E2 | 9.8E-4 | 1.4E1 | 7.8E3 | 4.0E3 | 447 | 21 | 170 | 21 | 0.64 |
| Ao | pg/mL | 9.2E1 | 1.3E2 | 5.1E2 | 5.6E2 | 3.5E3 | 1.2E3 | 1.5E0 | 4.1E0 | 3.9E4 | 4.5E3 | 447 | 21 | 170 | 21 | 0.61 |
| Ap | ng/mL | 3.3E1 | 4.5E1 | 4.7E1 | 6.1E1 | 5.0E1 | 6.8E1 | 8.4E-5 | 2.7E0 | 3.3E2 | 2.4E2 | 447 | 21 | 170 | 21 | 0.54 |
| Ar | ng/mL | 9.5E-1 | 1.7E0 | 1.2E1 | 7.0E0 | 1.9E2 | 1.3E1 | 3.4E-3 | 4.1E-2 | 4.1E3 | 5.1E1 | 447 | 21 | 170 | 21 | 0.62 |
| As | ng/mL | 8.7E-3 | 1.3E-2 | 1.6E-2 | 1.6E-2 | 6.1E-2 | 1.5E-2 | 1.7E-3 | 1.7E-3 | 1.2E0 | 7.0E-2 | 447 | 21 | 170 | 21 | 0.61 |
| Aw | pg/mL | 1.6E1 | 2.1E1 | 1.6E1 | 2.1E1 | 6.4E0 | 6.1E0 | 2.9E-2 | 1.1E1 | 5.1E1 | 3.2E1 | 447 | 21 | 170 | 21 | 0.74 |
| Ax | ng/mL | 2.1E0 | 1.7E0 | 1.6E1 | 9.0E0 | 6.3E1 | 1.3E1 | 1.2E-2 | 4.1E-2 | 7.7E2 | 5.2E1 | 447 | 21 | 170 | 21 | 0.50 |
| Ba | ng/mL | 6.1E1 | 9.4E1 | 4.1E2 | 2.1E3 | 1.1E3 | 3.9E3 | 3.7E-1 | 1.1E0 | 8.1E3 | 1.5E4 | 447 | 21 | 170 | 21 | 0.58 |
| Bb | ng/mL | 3.4E0 | 6.4E0 | 6.8E0 | 9.7E0 | 1.4E1 | 1.0E1 | 4.1E-3 | 4.1E-3 | 2.5E2 | 3.2E1 | 447 | 21 | 170 | 21 | 0.61 |
| Bc | ng/mL | 3.8E1 | 8.6E1 | 1.1E2 | 1.8E2 | 2.0E2 | 2.9E2 | 1.1E-1 | 2.9E-1 | 1.2E3 | 1.2E3 | 447 | 21 | 170 | 21 | 0.62 |
| Bg | ng/mL | 7.7E-2 | 4.1E-1 | 5.4E0 | 1.0E1 | 3.0E1 | 3.3E1 | 5.3E-4 | 5.3E-4 | 4.4E2 | 1.5E2 | 447 | 21 | 170 | 21 | 0.64 |
| Bn | ng/mL | 5.6E-2 | 7.5E-1 | 1.3E0 | 1.9E0 | 3.3E0 | 2.5E0 | 5.6E-2 | 5.6E-2 | 5.8E1 | 8.6E0 | 447 | 21 | 170 | 21 | 0.58 |
| Bo | ng/mL | 1.2E1 | 1.5E1 | 1.4E1 | 1.9E1 | 1.9E1 | 1.7E1 | 1.6E-2 | 1.6E-2 | 2.8E2 | 5.5E1 | 447 | 21 | 170 | 21 | 0.58 |
| Ch | uIU/mL | 9.7E-1 | 1.6E0 | 1.7E1 | 1.5E1 | 1.0E2 | 2.9E1 | 3.4E-3 | 1.5E-1 | 1.8E3 | 1.2E2 | 447 | 21 | 170 | 21 | 0.59 |
| Co | pg/mL | 3.8E1 | 6.7E1 | 1.8E2 | 2.3E2 | 9.6E2 | 4.5E2 | 1.5E-1 | 7.3E0 | 1.7E4 | 2.1E3 | 447 | 21 | 170 | 21 | 0.64 |
| Cp | ng/mL | 2.1E1 | 5.0E1 | 3.0E1 | 4.7E1 | 6.7E1 | 2.7E1 | 6.0E-1 | 4.7E0 | 1.3E3 | 9.6E1 | 447 | 21 | 170 | 21 | 0.75 |
| Cq | ng/mL | 2.8E-2 | 3.4E-2 | 2.5E-1 | 1.1E-1 | 2.5E0 | 2.4E-1 | 8.0E-4 | 8.0E-4 | 4.9E1 | 1.1E0 | 447 | 21 | 170 | 21 | 0.56 |
| Cs | ng/mL | 5.9E1 | 1.0E2 | 3.2E2 | 2.5E2 | 1.2E3 | 3.8E2 | 2.7E-2 | 8.9E-1 | 1.8E4 | 1.6E3 | 447 | 21 | 170 | 21 | 0.54 |
| Ct | ng/mL | 6.1E-1 | 3.8E-1 | 3.2E1 | 5.9E1 | 9.9E1 | 1.3E2 | 1.1E-4 | 3.8E-2 | 6.2E2 | 4.4E2 | 447 | 21 | 170 | 21 | 0.53 |
| Cu | ng/mL | 2.4E-1 | 4.8E-1 | 5.5E-1 | 7.0E-1 | 3.2E0 | 6.9E-1 | 9.6E-3 | 2.7E-2 | 6.6E1 | 2.4E0 | 447 | 21 | 170 | 21 | 0.65 |
| Cv | ng/mL | 5.8E0 | 2.4E0 | 2.8E1 | 3.6E1 | 6.9E1 | 1.0E2 | 1.4E-4 | 1.6E-1 | 5.3E2 | 4.7E2 | 447 | 21 | 170 | 21 | 0.45 |
| Cw | mIU/mL | 3.0E-2 | 4.6E-2 | 5.4E-2 | 5.2E-2 | 3.2E-1 | 3.5E-2 | 1.5E-4 | 5.5E-3 | 6.8E0 | 1.2E-1 | 447 | 21 | 170 | 21 | 0.61 |
| Cx | ng/mL | 4.6E-1 | 3.8E-2 | 6.0E1 | 9.0E1 | 1.1E2 | 1.4E2 | 9.3E-5 | 9.3E-5 | 3.9E2 | 3.9E2 | 447 | 21 | 170 | 21 | 0.48 |
| Db | ug/mL | 7.6E0 | 8.0E0 | 9.6E0 | 9.1E0 | 1.1E1 | 6.3E0 | 4.5E-1 | 1.2E0 | 1.4E2 | 2.9E1 | 447 | 21 | 170 | 21 | 0.53 |
| Dc | nmol/L | 1.9E-2 | 2.5E-2 | 8.6E-2 | 2.3E-1 | 6.7E-1 | 5.7E-1 | 5.2E-6 | 3.6E-4 | 1.4E1 | 2.2E0 | 447 | 21 | 170 | 21 | 0.54 |
| Dd | ug/mL | 7.8E-2 | 9.8E-2 | 1.9E-1 | 1.9E-1 | 3.1E-1 | 2.5E-1 | 1.9E-4 | 1.6E-4 | 3.6E0 | 7.9E-1 | 447 | 21 | 170 | 21 | 0.49 |
| De | ng/mL | 3.4E-3 | 1.7E-1 | 7.6E-2 | 1.6E-1 | 1.5E-1 | 2.4E-1 | 3.4E-3 | 3.4E-3 | 9.2E-1 | 1.1E0 | 447 | 21 | 170 | 21 | 0.65 |
| Dg | ng/mL | 3.4E1 | 3.8E1 | 4.6E1 | 4.2E1 | 4.1E1 | 3.5E1 | 1.0E-1 | 7.8E-1 | 1.9E2 | 1.2E2 | 447 | 21 | 170 | 21 | 0.49 |
| Di | pg/mL | 1.9E0 | 3.7E0 | 2.2E0 | 3.5E0 | 2.1E0 | 2.0E0 | 1.8E-1 | 1.8E-1 | 1.3E1 | 9.3E0 | 447 | 21 | 170 | 21 | 0.70 |
| Dk | uIU/mL | 1.6E-2 | 3.6E-2 | 8.7E-2 | 8.0E-2 | 5.1E-1 | 1.5E-1 | 1.1E-4 | 1.1E-4 | 8.9E0 | 6.3E-1 | 447 | 21 | 170 | 21 | 0.57 |
| Dl | ng/mL | 2.4E2 | 2.2E2 | 3.2E2 | 3.1E2 | 2.9E2 | 2.7E2 | 2.5E0 | 4.7E0 | 1.6E3 | 8.6E2 | 447 | 21 | 170 | 21 | 0.50 |
| Dp | ng/ml | 2.5E0 | 1.8E0 | 6.4E0 | 4.3E0 | 1.5E1 | 6.8E0 | 3.7E-3 | 7.7E-2 | 2.0E2 | 2.6E1 | 262 | 19 | 160 | 19 | 0.41 |
| Dr | pg/ml | 2.9E1 | 1.3E1 | 1.2E2 | 1.7E1 | 8.3E2 | 1.6E1 | 7.5E-1 | 7.5E-1 | 1.0E4 | 3.9E1 | 158 | 8 | 91 | 8 | 0.32 |
| Ef | ng/ml | 1.3E-1 | 2.6E-1 | 8.6E-1 | 2.2E0 | 1.9E0 | 3.2E0 | 5.7E-4 | 5.7E-3 | 1.0E1 | 9.9E0 | 325 | 19 | 167 | 19 | 0.59 |
| Wm | % | 7.2E-1 | 7.0E-1 | 3.2E1 | 3.8E1 | 1.8E2 | 1.3E2 | 5.4E-2 | 8.5E-2 | 2.4E3 | 6.1E2 | 353 | 25 | 182 | 25 | 0.53 |
| Ed | pg/ml | 5.2E-1 | 5.1E0 | 5.9E1 | 3.2E1 | 4.5E2 | 4.4E1 | 5.2E-1 | 5.2E-1 | 7.3E3 | 1.3E2 | 262 | 19 | 159 | 19 | 0.55 |
| Tj | pg/mL | 3.7E-1 | 3.7E-1 | 5.6E1 | 5.5E0 | 3.0E2 | 1.1E1 | 3.6E-1 | 3.7E-1 | 3.5E3 | 4.3E1 | 323 | 19 | 169 | 19 | 0.45 |
| Po | pg/ml | 5.4E-1 | 4.4E0 | 8.9E0 | 1.7E1 | 2.6E1 | 4.1E1 | 8.0E-3 | 2.6E-2 | 2.7E2 | 2.2E2 | 755 | 37 | 295 | 37 | 0.61 |
| Em | ng/ml | 2.9E-3 | 1.3E-2 | 7.8E-2 | 2.4E-2 | 1.9E-1 | 2.9E-2 | 1.9E-16 | 2.9E-3 | 1.9E0 | 9.1E-2 | 204 | 9 | 91 | 9 | 0.50 |
| Et | ng/ml | 1.4E3 | 2.0E3 | 1.7E3 | 2.2E3 | 1.2E3 | 1.4E3 | 7.5E1 | 7.9E1 | 5.0E3 | 5.0E3 | 754 | 37 | 295 | 37 | 0.60 |
| Fa | ng/ml | 4.0E1 | 5.9E1 | 1.3E2 | 1.9E2 | 5.6E2 | 4.8E2 | 3.4E-2 | 4.5E0 | 8.0E3 | 2.1E3 | 261 | 19 | 158 | 19 | 0.58 |
| Ez | ng/ml | 3.8E0 | 5.4E0 | 1.5E1 | 2.7E1 | 3.2E1 | 5.0E1 | 1.3E-2 | 1.3E-2 | 3.0E2 | 2.0E2 | 262 | 19 | 160 | 19 | 0.57 |
| Fb | ng/ml | 2.5E1 | 3.3E1 | 2.2E1 | 2.8E1 | 1.1E1 | 1.3E1 | 5.9E-1 | 8.9E-1 | 5.7E1 | 4.3E1 | 262 | 19 | 158 | 19 | 0.65 |
| Ex | ng/ml | 7.8E-2 | 1.6E-1 | 2.2E-1 | 5.5E-1 | 6.9E-1 | 1.0E0 | 3.5E-5 | 1.7E-4 | 8.9E0 | 4.1E0 | 245 | 16 | 117 | 16 | 0.67 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fn | ng/ml | 2.1E-1 | 1.6E0 | 5.9E0 | 3.0E0 | 2.7E1 | 4.9E0 | 1.1E-14 | 2.1E-1 | 4.2E2 | 2.1E1 | 262 | 19 | 160 | 19 | 0.53 |
| Fp | ng/ml | 1.3E1 | 2.3E1 | 2.5E1 | 3.3E1 | 2.8E1 | 3.4E1 | 6.0E-3 | 4.3E-1 | 1.4E2 | 1.3E2 | 787 | 37 | 296 | 37 | 0.56 |
| Fr | ng/ml | 3.5E4 | 1.1E5 | 1.1E5 | 2.7E5 | 1.7E5 | 3.0E5 | 1.9E2 | 1.3E3 | 9.0E5 | 8.4E5 | 890 | 39 | 300 | 39 | 0.64 |
| Fw | pg/ml | 1.1E0 | 1.1E1 | 6.2E1 | 1.9E1 | 4.8E2 | 2.1E1 | 1.1E-14 | 1.7E-14 | 6.9E3 | 7.1E1 | 325 | 20 | 168 | 20 | 0.62 |
| Fy | ng/ml | 3.8E1 | 3.3E1 | 6.0E1 | 4.8E1 | 7.3E1 | 4.3E1 | 1.2E-1 | 1.2E-1 | 6.5E2 | 1.3E2 | 260 | 17 | 159 | 17 | 0.48 |
| Gc | ng/ml | 1.1E2 | 5.8E1 | 1.7E2 | 1.2E2 | 1.8E2 | 1.6E2 | 6.4E0 | 2.3E1 | 1.2E3 | 5.1E2 | 169 | 8 | 94 | 8 | 0.37 |
| Gd | ng/ml | 3.1E1 | 2.9E1 | 3.3E1 | 2.8E1 | 1.7E1 | 2.0E1 | 5.0E0 | 3.0E0 | 8.1E1 | 5.6E1 | 189 | 9 | 83 | 9 | 0.42 |
| Gn | U/ml | 2.8E-1 | 1.6E-1 | 2.1E0 | 3.6E-1 | 9.6E0 | 4.3E-1 | 1.3E-3 | 1.2E-2 | 1.1E2 | 1.0E0 | 152 | 8 | 89 | 8 | 0.41 |
| Gl | pg/ml | 7.1E3 | 1.3E4 | 1.1E4 | 1.5E4 | 9.1E3 | 1.1E4 | 9.1E1 | 5.3E2 | 3.4E4 | 3.2E4 | 316 | 20 | 166 | 20 | 0.62 |
| Gp | U/ml | 1.5E0 | 8.0E-1 | 4.1E0 | 2.3E0 | 7.0E0 | 3.0E0 | 1.3E-3 | 1.5E-2 | 6.7E1 | 9.2E0 | 327 | 19 | 168 | 19 | 0.41 |
| Gz | ug/ml | 1.4E0 | 1.6E0 | 8.9E0 | 5.0E0 | 3.7E1 | 5.4E0 | 2.9E-16 | 4.2E-2 | 4.8E2 | 1.3E1 | 181 | 16 | 106 | 16 | 0.45 |
| Ha | ng/ml | 2.3E0 | 3.2E0 | 9.5E0 | 1.2E1 | 2.1E1 | 2.5E1 | 6.4E-3 | 1.7E-2 | 1.3E2 | 1.1E2 | 260 | 19 | 159 | 19 | 0.57 |
| Nm | pg/ml | 1.5E4 | 1.4E4 | 3.4E4 | 3.2E4 | 8.7E4 | 4.2E4 | 1.0E-9 | 1.0E-9 | 1.6E6 | 1.5E5 | 758 | 37 | 297 | 37 | 0.50 |
| Nn | pg/ml | 1.5E2 | 7.3E2 | 1.7E3 | 8.4E3 | 7.6E3 | 2.3E4 | 1.0E-9 | 1.0E-9 | 1.0E5 | 1.1E5 | 758 | 37 | 297 | 37 | 0.68 |
| No | pg/ml | 1.5E1 | 1.9E1 | 3.9E1 | 8.6E1 | 1.2E2 | 1.8E2 | 1.0E-9 | 1.0E-9 | 2.5E3 | 7.7E2 | 758 | 37 | 297 | 37 | 0.57 |
| Nq | pg/ml | 1.7E0 | 6.3E0 | 1.7E1 | 4.2E1 | 6.8E1 | 7.6E1 | 1.0E-9 | 1.0E-9 | 1.2E3 | 3.0E2 | 758 | 37 | 297 | 37 | 0.62 |
| Nr | pg/ml | 9.7E-1 | 2.8E0 | 3.1E1 | 4.7E1 | 1.9E2 | 2.2E2 | 1.0E-9 | 1.0E-9 | 4.1E3 | 1.4E3 | 758 | 37 | 297 | 37 | 0.57 |
| Ns | pg/ml | 1.0E-9 | 1.0E-9 | 7.1E0 | 1.9E0 | 3.5E1 | 8.8E0 | 1.0E-9 | 1.0E-9 | 6.8E2 | 5.1E1 | 758 | 37 | 297 | 37 | 0.49 |
| Nt | pg/ml | 1.0E2 | 1.3E2 | 1.3E2 | 1.9E2 | 1.1E2 | 2.1E2 | 1.0E-9 | 1.4E1 | 1.7E3 | 1.2E3 | 758 | 37 | 297 | 37 | 0.59 |
| Nu | pg/ml | 2.0E1 | 3.8E1 | 5.3E1 | 9.5E1 | 8.9E1 | 1.3E2 | 1.0E-9 | 1.0E-9 | 8.9E2 | 6.3E2 | 758 | 37 | 297 | 37 | 0.61 |
| Lu | pg/ml | 1.0E4 | 1.0E4 | 1.8E4 | 1.0E4 | 6.5E4 | 1.2E4 | 3.5E2 | 1.3E3 | 1.3E6 | 7.5E4 | 761 | 37 | 297 | 37 | 0.42 |
| Lv | pg/ml | 1.0E-9 | 7.5E0 | 1.1E1 | 2.9E1 | 2.1E1 | 4.0E1 | 1.0E-9 | 1.0E-9 | 2.4E2 | 1.6E2 | 761 | 37 | 297 | 37 | 0.63 |
| Lw | pg/ml | 1.0E-9 | 1.0E-9 | 4.3E-1 | 2.4E0 | 4.1E0 | 9.1E0 | 1.0E-9 | 1.0E-9 | 8.0E1 | 4.7E1 | 761 | 37 | 297 | 37 | 0.53 |
| Lx | pg/ml | 1.0E-9 | 1.3E2 | 1.8E2 | 3.8E2 | 9.3E2 | 6.8E2 | 1.0E-9 | 1.0E-9 | 2.2E4 | 2.8E3 | 761 | 37 | 297 | 37 | 0.68 |
| Ly | pg/ml | 1.0E-9 | 1.0E-9 | 1.0E1 | 1.2E1 | 2.0E1 | 2.0E1 | 1.0E-9 | 1.0E-9 | 1.2E2 | 7.2E1 | 761 | 37 | 297 | 37 | 0.53 |
| Lz | pg/ml | 1.0E-9 | 1.0E-9 | 4.0E0 | 4.3E0 | 3.2E1 | 1.5E1 | 1.0E-9 | 1.0E-9 | 6.0E2 | 6.2E1 | 761 | 37 | 297 | 37 | 0.53 |
| Ma | pg/ml | 2.9E2 | 4.6E2 | 1.3E3 | 2.7E3 | 3.6E3 | 6.2E3 | 1.0E-9 | 1.0E-9 | 6.5E4 | 3.6E4 | 761 | 37 | 297 | 37 | 0.55 |
| Mb | pg/ml | 2.5E1 | 3.4E1 | 3.1E1 | 3.7E1 | 1.5E1 | 2.1E1 | 4.1E0 | 1.3E1 | 2.1E2 | 1.1E2 | 761 | 37 | 297 | 37 | 0.57 |
| Mc | pg/ml | 1.0E-9 | 1.0E-9 | 4.3E-2 | 3.8E-2 | 6.1E-1 | 2.3E-1 | 1.0E-9 | 1.0E-9 | 1.3E1 | 1.4E0 | 761 | 37 | 297 | 37 | 0.51 |
| Md | pg/ml | 1.0E-9 | 1.0E-9 | 4.5E-1 | 2.6E-1 | 3.9E0 | 1.1E0 | 1.0E-9 | 1.0E-9 | 6.8E1 | 5.9E0 | 761 | 37 | 297 | 37 | 0.52 |
| Me | pg/ml | 3.3E1 | 2.7E1 | 3.2E1 | 3.0E1 | 2.0E1 | 3.0E1 | 1.0E-9 | 2.4E-1 | 3.2E2 | 1.8E2 | 761 | 37 | 297 | 37 | 0.40 |
| Mf | pg/ml | 1.0E-9 | 1.0E-9 | 5.0E-1 | 4.1E-1 | 2.8E0 | 1.4E0 | 1.0E-9 | 1.0E-9 | 5.6E1 | 6.9E0 | 761 | 37 | 297 | 37 | 0.51 |
| Mg | pg/ml | 1.6E0 | 8.7E-1 | 7.0E0 | 1.1E1 | 1.2E1 | 2.6E1 | 1.0E-9 | 1.0E-9 | 9.2E1 | 1.5E2 | 761 | 37 | 297 | 37 | 0.49 |
| Mh | pg/ml | 1.0E-9 | 1.0E-9 | 1.5E0 | 1.3E0 | 1.0E1 | 3.9E0 | 1.0E-9 | 1.0E-9 | 1.6E2 | 1.8E1 | 761 | 37 | 297 | 37 | 0.55 |
| Mi | pg/ml | 1.0E-9 | 1.0E-9 | 9.4E-1 | 5.9E0 | 1.3E1 | 2.0E1 | 1.0E-9 | 1.0E-9 | 3.2E2 | 1.1E2 | 761 | 37 | 297 | 37 | 0.56 |
| Mj | pg/ml | 1.0E-9 | 1.0E-9 | 4.7E0 | 1.3E1 | 2.7E1 | 3.8E1 | 1.0E-9 | 1.0E-9 | 4.6E2 | 2.1E2 | 761 | 37 | 297 | 37 | 0.56 |
| Mk | pg/ml | 2.8E-1 | 4.3E0 | 1.4E1 | 2.3E1 | 9.2E1 | 8.3E1 | 1.0E-9 | 1.0E-9 | 1.7E3 | 5.0E2 | 761 | 37 | 297 | 37 | 0.57 |
| Ml | pg/ml | 1.0E-9 | 1.0E-9 | 6.0E0 | 2.6E0 | 8.1E1 | 8.2E0 | 1.0E-9 | 1.0E-9 | 2.1E3 | 4.3E1 | 761 | 37 | 297 | 37 | 0.50 |
| Mm | pg/ml | 6.1E2 | 8.4E2 | 1.1E3 | 1.4E3 | 1.4E3 | 1.7E3 | 1.0E-9 | 1.0E-9 | 1.2E4 | 6.5E3 | 761 | 37 | 297 | 37 | 0.54 |
| Mn | pg/ml | 5.4E0 | 7.2E0 | 1.0E1 | 1.0E1 | 2.0E1 | 1.1E1 | 1.0E-9 | 1.0E-9 | 3.5E2 | 5.1E1 | 761 | 37 | 297 | 37 | 0.55 |
| Mp | pg/ml | 1.0E-9 | 9.4E0 | 1.0E1 | 2.4E1 | 3.6E1 | 5.0E1 | 1.0E-9 | 1.0E-9 | 6.3E2 | 2.7E2 | 760 | 37 | 297 | 37 | 0.64 |
| Mq | pg/ml | 1.0E-9 | 1.0E-9 | 2.8E0 | 6.2E0 | 1.6E1 | 1.8E1 | 1.0E-9 | 1.0E-9 | 2.1E2 | 8.6E1 | 760 | 37 | 297 | 37 | 0.55 |
| Mr | pg/ml | 1.0E-9 | 1.0E-9 | 3.2E1 | 1.2E2 | 1.8E2 | 5.6E2 | 1.0E-9 | 1.0E-9 | 2.2E3 | 3.4E3 | 760 | 37 | 297 | 37 | 0.58 |
| Ms | pg/ml | 4.1E2 | 4.3E2 | 5.6E2 | 5.5E2 | 6.6E2 | 5.1E2 | 1.0E-9 | 1.0E-9 | 5.9E3 | 2.0E3 | 760 | 37 | 297 | 37 | 0.52 |
| Mt | pg/ml | 2.4E-1 | 1.9E0 | 7.3E0 | 9.8E1 | 4.5E1 | 5.3E2 | 1.0E-9 | 1.0E-9 | 8.7E2 | 3.2E3 | 760 | 37 | 297 | 37 | 0.63 |
| Mu | pg/ml | 1.0E-9 | 1.0E-9 | 9.0E-1 | 4.2E0 | 7.0E0 | 1.0E1 | 1.0E-9 | 1.0E-9 | 9.9E1 | 4.8E1 | 760 | 37 | 297 | 37 | 0.66 |
| Mv | pg/ml | 1.0E-9 | 1.0E-9 | 5.8E0 | 2.0E2 | 3.1E2 | 4.7E2 | 1.0E-9 | 1.0E-9 | 4.5E3 | 2.5E3 | 760 | 37 | 297 | 37 | 0.62 |
| Mw | pg/ml | 3.8E1 | 7.5E1 | 4.0E2 | 7.2E2 | 2.8E3 | 1.5E3 | 1.0E-9 | 1.0E-9 | 6.2E4 | 5.9E3 | 760 | 37 | 297 | 37 | 0.60 |
| Mx | pg/ml | 1.0E-9 | 1.0E-9 | 2.2E-1 | 7.8E-1 | 9.1E-1 | 3.2E0 | 1.0E-9 | 1.0E-9 | 9.6E0 | 2.0E1 | 760 | 37 | 297 | 37 | 0.58 |
| My | pg/ml | 1.0E-9 | 1.8E1 | 3.9E2 | 3.3E2 | 2.7E3 | 8.4E2 | 1.0E-9 | 1.0E-9 | 3.9E4 | 4.6E3 | 760 | 37 | 297 | 37 | 0.60 |
| Mz | pg/ml | 1.1E1 | 2.6E1 | 2.6E1 | 9.5E1 | 7.9E1 | 3.2E2 | 1.0E-9 | 1.0E-9 | 1.3E3 | 1.9E3 | 760 | 37 | 297 | 37 | 0.66 |
| Na | pg/ml | 1.0E-9 | 1.0E-9 | 7.4E-1 | 7.8E-1 | 3.2E0 | 2.9E0 | 1.0E-9 | 1.0E-9 | 4.2E1 | 1.6E1 | 760 | 37 | 297 | 37 | 0.50 |
| Nb | pg/ml | 1.9E0 | 3.2E0 | 4.0E0 | 9.8E0 | 1.3E1 | 3.4E1 | 1.0E-9 | 1.0E-9 | 2.3E2 | 2.1E2 | 760 | 37 | 297 | 37 | 0.62 |

| Code | Units | Median | | Average | | Standard Deviation | | Minimum | | Maximum | | Number of Samples | | Number of Patients | | AUC |
|------|-------|--------|-----|---------|-----|------|------|---------|--------|---------|--------|--------|-----|---------|-----|-----|
| | | NonDis | Dis | NonDis | Dis | NonDis | Dis | NonDis | Dis | NonDis | Dis | NonDis | Dis | NonDis | Dis | |
| Nc | pg/ml | 3.4E2 | 1.5E2 | 5.8E2 | 3.5E2 | 7.5E2 | 4.0E2 | 1.0E-9 | 1.0E-9 | 9.2E3 | 1.3E3 | 760 | 37 | 297 | 37 | 0.42 |
| Nd | pg/ml | 2.9E1 | 1.6E1 | 3.0E1 | 2.1E1 | 9.0E1 | 2.1E1 | 1.0E-9 | 1.0E-9 | 2.1E3 | 9.4E1 | 760 | 37 | 297 | 37 | 0.43 |
| Ne | pg/ml | 4.5E2 | 3.2E2 | 5.9E2 | 4.0E2 | 5.9E2 | 3.8E2 | 1.0E-9 | 1.0E-9 | 7.0E3 | 1.8E3 | 760 | 37 | 297 | 37 | 0.40 |
| Nf | pg/ml | 1.0E-9 | 1.0E-9 | 2.9E0 | 4.2E0 | 1.1E1 | 1.8E1 | 1.0E-9 | 1.0E-9 | 1.6E2 | 1.1E2 | 760 | 37 | 297 | 37 | 0.47 |
| Ng | pg/ml | 1.9E1 | 3.2E0 | 1.1E2 | 1.0E2 | 2.2E2 | 2.2E2 | 1.0E-9 | 1.0E-9 | 1.6E3 | 1.2E3 | 760 | 37 | 297 | 37 | 0.44 |
| Nh | pg/ml | 6.9E1 | 5.2E1 | 9.2E1 | 6.3E1 | 8.3E1 | 6.5E1 | 1.0E-9 | 1.0E-9 | 5.6E2 | 3.5E2 | 760 | 37 | 297 | 37 | 0.39 |
| Ni | pg/ml | 1.0E-9 | 3.9E1 | 7.2E1 | 1.2E2 | 1.2E2 | 1.5E2 | 1.0E-9 | 1.0E-9 | 1.1E3 | 4.8E2 | 760 | 37 | 297 | 37 | 0.57 |
| Nj | pg/ml | 7.3E0 | 4.9E0 | 1.1E1 | 8.0E0 | 1.2E1 | 6.9E0 | 1.0E-9 | 1.0E-9 | 8.3E1 | 2.2E1 | 760 | 37 | 297 | 37 | 0.46 |
| Nk | pg/ml | 1.8E1 | 1.6E1 | 3.4E1 | 2.6E1 | 4.0E1 | 2.8E1 | 1.0E-9 | 1.0E-9 | 2.0E2 | 1.1E2 | 760 | 37 | 297 | 37 | 0.47 |
| Nl | pg/ml | 4.6E1 | 3.2E1 | 6.2E1 | 3.9E1 | 7.0E1 | 3.5E1 | 1.0E-9 | 1.0E-9 | 1.1E3 | 1.3E2 | 760 | 37 | 297 | 37 | 0.40 |
| Tz | pg/ml | 4.6E3 | 8.9E3 | 1.3E4 | 1.4E4 | 6.3E4 | 1.8E4 | 1.0E-9 | 9.8E1 | 1.0E6 | 6.7E4 | 264 | 19 | 159 | 19 | 0.61 |
| Ua | pg/ml | 3.7E3 | 6.3E3 | 2.0E4 | 3.0E4 | 1.3E5 | 4.3E4 | 1.0E-9 | 2.7E2 | 2.1E6 | 1.3E5 | 264 | 19 | 159 | 19 | 0.60 |
| Ub | pg/ml | 5.7E2 | 2.7E2 | 8.7E2 | 5.0E2 | 1.1E3 | 5.1E2 | 1.0E-9 | 3.4E1 | 9.8E3 | 1.7E3 | 264 | 19 | 159 | 19 | 0.38 |
| Ue | pg/ml | 3.1E1 | 1.6E1 | 4.0E1 | 2.9E1 | 4.0E1 | 2.8E1 | 9.8E-2 | 6.7E0 | 4.4E2 | 1.1E2 | 264 | 19 | 159 | 19 | 0.35 |
| Uc | pg/ml | 9.2E2 | 1.0E3 | 2.0E3 | 1.2E3 | 4.4E3 | 1.2E3 | 1.0E-9 | 1.5E1 | 5.7E4 | 5.4E3 | 264 | 19 | 159 | 19 | 0.47 |
| Ud | pg/ml | 1.0E-9 | 1.0E-9 | 1.8E0 | 1.0E-9 | 2.4E1 | 0.0E0 | 1.0E-9 | 1.0E-9 | 3.9E2 | 1.0E-9 | 264 | 19 | 159 | 19 | 0.49 |
| Hq | pg/ml | 1.0E0 | 2.5E0 | 1.2E2 | 1.6E1 | 1.8E3 | 4.7E1 | 1.0E-9 | 1.0E-9 | 3.4E4 | 2.3E2 | 756 | 37 | 296 | 37 | 0.66 |
| Hr | pg/ml | 1.1E2 | 1.0E2 | 8.0E2 | 4.5E2 | 1.7E3 | 8.4E2 | 1.0E-9 | 1.0E-9 | 1.7E4 | 3.8E3 | 756 | 37 | 296 | 37 | 0.48 |
| Hu | pg/ml | 5.1E0 | 1.3E1 | 2.6E3 | 1.5E3 | 2.6E4 | 3.2E3 | 1.0E-9 | 1.0E-9 | 6.3E5 | 1.1E4 | 756 | 37 | 296 | 37 | 0.55 |
| Hv | pg/ml | 1.3E0 | 2.3E0 | 4.2E0 | 4.4E0 | 3.4E1 | 7.1E0 | 1.0E-9 | 1.0E-9 | 8.9E2 | 3.3E1 | 756 | 37 | 296 | 37 | 0.64 |
| Hw | pg/ml | 6.6E0 | 4.5E0 | 3.1E1 | 2.3E1 | 3.5E2 | 8.2E1 | 1.0E-9 | 1.0E-9 | 9.4E3 | 5.0E2 | 756 | 37 | 296 | 37 | 0.43 |
| Hx | pg/ml | 8.3E0 | 1.5E1 | 4.0E1 | 6.6E1 | 3.5E2 | 2.1E2 | 1.0E-9 | 1.0E-9 | 9.3E3 | 1.3E3 | 756 | 37 | 296 | 37 | 0.60 |
| Ib | ng/ml | 4.9E-2 | 1.4E-2 | 1.2E0 | 3.0E0 | 5.3E0 | 8.5E0 | 1.0E-9 | 1.0E-9 | 5.6E1 | 3.6E1 | 259 | 19 | 159 | 19 | 0.42 |
| Ic | U/ml | 2.2E2 | 2.2E2 | 1.1E3 | 2.0E2 | 7.3E3 | 1.1E2 | 1.5E0 | 2.1E1 | 9.3E4 | 3.9E2 | 259 | 19 | 159 | 19 | 0.49 |
| Id | U/ml | 6.8E-1 | 8.9E-1 | 3.0E0 | 2.1E0 | 2.7E1 | 2.5E0 | 1.0E-9 | 2.7E-1 | 4.3E2 | 9.4E0 | 259 | 19 | 159 | 19 | 0.58 |
| Tt | pg/ml | 1.6E2 | 1.8E2 | 1.7E2 | 1.8E2 | 5.4E1 | 5.1E1 | 4.3E1 | 1.0E2 | 4.4E2 | 2.8E2 | 246 | 17 | 153 | 17 | 0.54 |
| To | pg/ml | 1.6E0 | 1.8E0 | 1.9E0 | 1.9E0 | 2.3E0 | 1.8E0 | 1.0E-9 | 1.0E-9 | 2.3E1 | 6.0E0 | 254 | 19 | 156 | 19 | 0.52 |
| Tr | pg/ml | 3.3E0 | 3.9E0 | 6.6E0 | 5.5E0 | 2.1E1 | 4.2E0 | 1.0E-9 | 1.0E-9 | 3.1E2 | 1.4E1 | 251 | 19 | 155 | 19 | 0.57 |
| Tn | pg/ml | 2.8E1 | 7.2E1 | 8.2E1 | 1.5E2 | 2.5E2 | 2.5E2 | 2.4E0 | 6.6E0 | 2.3E3 | 1.1E3 | 254 | 19 | 156 | 19 | 0.65 |
| Tv | ng/ml | 1.2E1 | 1.2E1 | 5.2E1 | 1.5E1 | 4.5E2 | 1.9E1 | 1.0E-9 | 1.0E-9 | 7.1E3 | 6.5E1 | 254 | 19 | 156 | 19 | 0.44 |
| Ih | ng/ml | 6.8E1 | 8.9E1 | 2.4E2 | 3.1E2 | 4.3E2 | 5.3E2 | 1.0E-9 | 1.0E-9 | 3.6E3 | 2.8E3 | 760 | 37 | 296 | 37 | 0.58 |
| Ii | ng/ml | 9.1E1 | 7.2E1 | 2.4E2 | 3.2E2 | 6.3E2 | 7.8E2 | 1.0E-9 | 4.6E0 | 8.4E3 | 4.5E3 | 760 | 37 | 296 | 37 | 0.51 |
| Ij | ng/ml | 7.6E1 | 8.6E1 | 2.0E2 | 1.3E2 | 1.1E3 | 1.4E2 | 1.0E-9 | 9.4E0 | 2.4E4 | 6.8E2 | 750 | 37 | 294 | 37 | 0.54 |
| Ik | ng/ml | 1.1E1 | 7.3E1 | 9.1E2 | 2.4E2 | 8.9E3 | 4.1E2 | 5.9E-1 | 2.4E0 | 1.2E5 | 1.5E3 | 755 | 37 | 294 | 37 | 0.59 |
| Il | ng/ml | 3.2E2 | 2.3E2 | 1.3E3 | 1.0E3 | 2.8E3 | 2.8E3 | 1.0E-9 | 1.0E-9 | 1.3E4 | 1.2E4 | 744 | 35 | 295 | 35 | 0.44 |
| Im | ng/ml | 2.1E2 | 2.5E2 | 3.7E2 | 6.9E2 | 5.3E2 | 1.2E3 | 1.3E1 | 3.4E1 | 5.8E3 | 6.0E3 | 754 | 37 | 295 | 37 | 0.57 |
| In | ng/ml | 3.6E0 | 1.5E0 | 3.0E1 | 7.3E0 | 2.3E2 | 1.3E1 | 1.0E-9 | 1.0E-9 | 4.5E3 | 5.7E1 | 760 | 37 | 296 | 37 | 0.38 |
| Hb | ng/ml | 2.6E1 | 2.6E1 | 3.7E1 | 3.6E1 | 3.5E1 | 3.2E1 | 6.2E-1 | 4.8E-1 | 2.1E2 | 1.2E2 | 264 | 19 | 159 | 19 | 0.50 |
| Hc | pg/ml | 6.3E2 | 8.9E2 | 3.4E3 | 3.8E3 | 1.3E4 | 6.1E3 | 1.0E-9 | 2.2E2 | 1.0E5 | 2.2E4 | 264 | 19 | 159 | 19 | 0.60 |
| Hf | ng/ml | 1.5E2 | 1.1E2 | 3.5E2 | 2.3E2 | 4.9E2 | 4.0E2 | 1.0E-9 | 1.0E-9 | 2.5E3 | 1.8E3 | 264 | 19 | 159 | 19 | 0.45 |
| Io | ng/ml | 8.2E3 | 5.7E3 | 2.6E4 | 1.2E4 | 1.6E5 | 1.2E4 | 1.0E-9 | 2.4E2 | 4.0E6 | 4.3E4 | 752 | 37 | 296 | 37 | 0.46 |
| Ip | ng/ml | 8.8E0 | 3.0E1 | 1.9E1 | 3.4E1 | 2.4E1 | 4.7E1 | 1.0E-9 | 2.1E-1 | 2.6E2 | 2.4E2 | 752 | 37 | 296 | 37 | 0.62 |
| Iq | ug/ml | 9.6E-2 | 1.1E-1 | 1.9E1 | 6.8E0 | 5.0E2 | 3.6E1 | 1.0E-9 | 1.0E-9 | 1.4E4 | 2.2E2 | 752 | 37 | 296 | 37 | 0.50 |
| Ir | ug/ml | 3.4E-1 | 6.8E-1 | 3.7E0 | 7.9E0 | 2.7E1 | 2.8E1 | 1.0E-9 | 1.0E-9 | 5.1E2 | 1.3E2 | 751 | 37 | 296 | 37 | 0.61 |
| Is | ng/ml | 1.4E0 | 7.9E0 | 6.2E0 | 1.8E1 | 2.4E1 | 4.4E1 | 1.0E-9 | 1.4E-1 | 5.5E2 | 2.6E2 | 752 | 37 | 296 | 37 | 0.72 |
| It | ng/ml | 2.0E0 | 2.3E0 | 2.4E1 | 1.2E1 | 1.4E2 | 3.5E1 | 1.0E-9 | 1.0E-9 | 2.8E3 | 2.1E2 | 752 | 37 | 296 | 37 | 0.55 |
| Iu | ng/ml | 2.2E2 | 2.8E2 | 1.5E3 | 1.3E3 | 4.3E3 | 4.3E3 | 1.0E-9 | 1.0E-9 | 2.9E4 | 2.4E4 | 752 | 37 | 296 | 37 | 0.48 |
| Iv | ng/ml | 1.2E1 | 2.4E1 | 6.5E1 | 1.7E2 | 6.0E2 | 6.3E2 | 1.0E-9 | 1.0E-9 | 1.6E4 | 3.8E3 | 751 | 37 | 296 | 37 | 0.64 |
| Iz | ng/ml | 1.4E2 | 2.6E2 | 6.3E2 | 6.0E2 | 3.9E3 | 6.5E2 | 9.2E-1 | 4.1E0 | 6.2E4 | 2.1E3 | 264 | 19 | 159 | 19 | 0.60 |
| Rc | pg/ml | 6.1E3 | 5.5E3 | 7.3E3 | 9.0E3 | 5.5E3 | 9.5E3 | 1.9E2 | 5.5E2 | 3.0E4 | 3.9E4 | 261 | 19 | 159 | 19 | 0.51 |
| Rb | pg/ml | 8.6E-1 | 4.7E-1 | 2.8E0 | 1.3E0 | 5.5E0 | 2.0E0 | 1.0E-9 | 1.0E-9 | 5.6E1 | 7.8E0 | 261 | 19 | 159 | 19 | 0.46 |
| Pz | ng/ml | 4.4E3 | 3.3E3 | 8.3E3 | 7.0E3 | 4.0E4 | 1.1E4 | 1.3E1 | 6.3E1 | 1.0E6 | 7.0E4 | 753 | 37 | 295 | 37 | 0.50 |
| Qa | ng/ml | 3.4E3 | 8.3E3 | 6.3E3 | 1.1E4 | 1.1E4 | 9.2E3 | 1.2E1 | 4.3E2 | 2.2E5 | 3.2E4 | 753 | 37 | 295 | 37 | 0.65 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qb | ng/ml | 8.7E1 | 2.1E2 | 2.1E2 | 2.5E2 | 5.0E2 | 2.1E2 | 7.9E-1 | 1.3E1 | 8.3E3 | 6.5E2 | 753 | 37 | 295 | 37 | 0.64 |
| Qc | ng/ml | 2.1E2 | 4.4E2 | 4.4E2 | 5.5E2 | 7.6E2 | 5.7E2 | 1.0E-9 | 5.8E0 | 1.1E4 | 2.8E3 | 753 | 37 | 295 | 37 | 0.60 |
| Qd | ng/ml | 8.9E3 | 1.4E4 | 1.9E4 | 4.9E4 | 7.8E4 | 6.5E4 | 1.5E2 | 1.2E3 | 2.0E6 | 2.3E5 | 753 | 37 | 295 | 37 | 0.64 |
| Qe | ng/ml | 9.1E2 | 1.7E3 | 1.8E3 | 2.8E3 | 4.0E3 | 3.7E3 | 1.0E-9 | 8.7E1 | 9.7E4 | 1.8E4 | 753 | 37 | 295 | 37 | 0.60 |
| Jd | ng/ml | 9.0E-1 | 4.6E0 | 6.6E0 | 4.8E0 | 4.3E1 | 4.7E0 | 1.0E-9 | 1.0E-9 | 6.5E2 | 1.8E1 | 262 | 19 | 160 | 19 | 0.69 |
| Je | ng/ml | 1.0E-9 | 1.0E-9 | 2.1E0 | 1.7E0 | 7.8E0 | 2.5E0 | 1.0E-9 | 1.0E-9 | 8.8E1 | 8.2E0 | 262 | 19 | 160 | 19 | 0.53 |
| Jf | ng/ml | 1.0E-9 | 1.0E-9 | 1.0E0 | 8.4E-1 | 2.2E0 | 1.4E0 | 1.0E-9 | 1.0E-9 | 1.2E1 | 4.1E0 | 262 | 19 | 160 | 19 | 0.52 |
| Jg | ng/ml | 5.0E2 | 8.9E2 | 7.7E2 | 1.3E3 | 9.2E2 | 1.6E3 | 1.0E-9 | 2.0E1 | 1.0E4 | 7.1E3 | 756 | 37 | 296 | 37 | 0.59 |
| Jh | ng/ml | 2.8E0 | 5.9E0 | 2.4E1 | 5.4E1 | 1.1E2 | 9.8E1 | 1.0E-9 | 1.0E-9 | 1.6E3 | 4.7E2 | 756 | 37 | 296 | 37 | 0.65 |
| Ji | ng/ml | 5.2E1 | 1.1E2 | 7.7E1 | 1.7E2 | 8.9E1 | 1.6E2 | 1.0E-9 | 8.5E0 | 1.3E3 | 6.7E2 | 756 | 37 | 296 | 37 | 0.71 |
| Sr | pg/mL | 3.9E2 | 5.7E2 | 9.8E2 | 1.3E3 | 1.8E3 | 1.7E3 | 1.0E-9 | 1.0E-9 | 2.1E4 | 5.4E3 | 259 | 19 | 159 | 19 | 0.56 |
| Ss | pg/mL | 9.4E4 | 2.0E5 | 1.5E5 | 1.2E5 | 1.9E5 | 9.0E4 | 2.7E3 | 7.1E3 | 1.8E6 | 2.4E5 | 259 | 19 | 159 | 19 | 0.49 |
| St | pg/mL | 2.6E7 | 5.3E7 | 4.8E7 | 2.3E8 | 6.1E7 | 4.5E8 | 1.0E-9 | 9.9E5 | 5.4E8 | 1.7E9 | 257 | 19 | 157 | 19 | 0.59 |
| Ra | pg/ml | 1.0E-9 | 1.0E-9 | 8.4E-1 | 6.7E-1 | 4.1E0 | 1.1E0 | 1.0E-9 | 1.0E-9 | 6.4E1 | 3.4E0 | 261 | 19 | 159 | 19 | 0.55 |
| Qz | pg/ml | 1.0E1 | 9.8E0 | 6.0E1 | 4.8E1 | 9.9E1 | 7.0E1 | 1.0E-9 | 1.0E-9 | 7.1E2 | 2.6E2 | 261 | 19 | 159 | 19 | 0.47 |
| Qy | pg/ml | 4.4E-1 | 1.2E0 | 9.8E1 | 4.5E1 | 5.8E1 | 1.7E2 | 1.0E-9 | 1.0E-9 | 6.5E2 | 7.3E2 | 261 | 19 | 159 | 19 | 0.66 |
| Qx | pg/ml | 1.0E-9 | 1.0E-9 | 6.6E0 | 2.4E0 | 5.1E1 | 5.4E0 | 1.0E-9 | 1.0E-9 | 5.8E2 | 2.1E1 | 261 | 19 | 159 | 19 | 0.54 |
| Qw | pg/ml | 1.0E-9 | 1.0E-9 | 1.9E0 | 3.6E0 | 9.3E0 | 1.2E1 | 1.0E-9 | 1.0E-9 | 1.2E2 | 5.1E1 | 261 | 19 | 159 | 19 | 0.41 |
| Qv | pg/ml | 2.3E4 | 1.5E4 | 3.6E4 | 2.0E4 | 7.8E4 | 2.1E4 | 6.0E1 | 1.0E-9 | 9.4E5 | 9.1E4 | 261 | 19 | 159 | 19 | 0.38 |
| Qu | pg/ml | 7.8E0 | 2.0E1 | 8.6E1 | 1.1E2 | 1.8E2 | 1.9E2 | 1.0E-9 | 1.0E-9 | 9.8E2 | 7.3E2 | 261 | 19 | 159 | 19 | 0.53 |
| Qt | pg/ml | 1.0E1 | 1.5E1 | 5.0E1 | 4.8E1 | 1.3E2 | 8.7E1 | 1.0E-9 | 1.0E-9 | 1.0E3 | 3.3E2 | 261 | 19 | 159 | 19 | 0.53 |
| Qh | ng/ml | 1.6E1 | 2.7E1 | 3.8E1 | 5.3E1 | 6.6E1 | 7.8E1 | 1.0E-9 | 2.5E0 | 6.4E2 | 3.3E2 | 261 | 19 | 159 | 19 | 0.59 |
| Qg | ng/ml | 7.8E0 | 6.6E0 | 1.6E1 | 1.5E1 | 2.6E1 | 2.1E1 | 5.1E-2 | 1.4E0 | 2.2E2 | 7.5E1 | 261 | 19 | 159 | 19 | 0.48 |
| Jj | ng/ml | 6.3E2 | 1.9E2 | 1.8E3 | 3.5E2 | 1.3E4 | 3.4E2 | 1.5E0 | 2.5E1 | 3.4E5 | 1.5E3 | 756 | 37 | 296 | 37 | 0.27 |
| Jk | ng/ml | 3.0E0 | 4.1E0 | 1.9E1 | 3.5E1 | 4.1E1 | 6.3E1 | 1.0E-9 | 4.3E-2 | 2.8E2 | 2.4E2 | 756 | 37 | 296 | 37 | 0.57 |
| Jl | ng/ml | 3.8E-1 | 1.1E0 | 1.8E0 | 2.7E2 | 4.6E0 | 1.6E3 | 7.6E-4 | 5.4E-3 | 4.0E1 | 9.9E3 | 756 | 37 | 296 | 37 | 0.62 |
| Jm | ng/ml | 1.6E1 | 2.3E1 | 5.9E1 | 3.5E1 | 1.4E2 | 3.9E1 | 1.0E-9 | 2.3E-1 | 2.1E3 | 1.5E2 | 756 | 37 | 296 | 37 | 0.52 |
| Jn | pg/ml | 4.0E-1 | 9.0E-1 | 3.2E0 | 2.1E1 | 3.2E1 | 1.2E2 | 1.0E-9 | 1.0E-9 | 6.2E2 | 7.3E2 | 755 | 37 | 296 | 37 | 0.59 |
| Jo | pg/ml | 3.6E3 | 3.4E3 | 5.0E3 | 5.3E3 | 5.3E3 | 6.4E3 | 2.0E1 | 2.7E2 | 1.0E5 | 3.6E4 | 756 | 37 | 296 | 37 | 0.49 |
| Jp | pg/ml | 6.9E4 | 9.4E4 | 7.2E4 | 9.4E4 | 3.8E4 | 4.8E4 | 5.8E2 | 4.6E3 | 3.8E5 | 2.1E5 | 756 | 37 | 296 | 37 | 0.67 |
| Jq | pg/ml | 9.4E1 | 1.7E2 | 1.6E2 | 3.5E2 | 3.5E2 | 6.5E2 | 1.0E0 | 7.4E0 | 8.7E3 | 3.3E3 | 756 | 37 | 296 | 37 | 0.64 |
| Jr | pg/ml | 5.2E0 | 7.0E0 | 4.0E1 | 2.2E2 | 4.4E2 | 1.2E3 | 1.0E-9 | 1.0E-9 | 1.1E4 | 7.4E3 | 756 | 37 | 296 | 37 | 0.57 |
| Js | pg/ml | 1.3E1 | 1.6E1 | 5.5E1 | 1.0E2 | 4.0E2 | 4.7E2 | 1.0E-9 | 2.2E0 | 1.0E4 | 2.9E3 | 756 | 37 | 296 | 37 | 0.60 |
| Jt | pg/ml | 2.7E3 | 3.1E3 | 3.3E3 | 4.0E3 | 2.8E3 | 4.5E3 | 2.2E1 | 1.9E2 | 5.2E4 | 2.5E4 | 756 | 37 | 296 | 37 | 0.53 |
| Ju | mIU/ml | 9.1E0 | 6.4E0 | 2.0E1 | 1.1E1 | 3.1E1 | 1.4E1 | 4.8E-2 | 3.9E-1 | 2.3E2 | 6.2E1 | 262 | 19 | 160 | 19 | 0.44 |
| Jv | mIU/ml | 1.3E1 | 1.5E1 | 3.3E1 | 3.1E1 | 5.7E1 | 4.4E1 | 1.0E-2 | 2.4E-2 | 4.4E2 | 1.4E2 | 262 | 19 | 160 | 19 | 0.50 |
| Jy | ng/ml | 1.6E-3 | 1.4E-3 | 2.3E-3 | 4.5E-3 | 4.2E-3 | 9.1E-3 | 1.0E-9 | 4.5E-4 | 5.2E-2 | 4.1E-2 | 262 | 19 | 160 | 19 | 0.55 |
| Kc | pg/ml | 2.6E1 | 5.7E1 | 4.5E1 | 9.4E1 | 4.8E1 | 8.3E1 | 1.0E-9 | 1.1E1 | 2.7E2 | 3.2E2 | 264 | 19 | 159 | 19 | 0.72 |
| Kd | pg/ml | 1.0E-9 | 1.0E-9 | 3.3E2 | 7.1E2 | 2.4E3 | 1.4E3 | 1.0E-9 | 1.0E-9 | 3.8E4 | 5.2E3 | 264 | 19 | 159 | 19 | 0.63 |
| Ke | pg/ml | 1.3E4 | 1.9E4 | 1.6E4 | 2.4E4 | 2.2E4 | 2.5E4 | 3.4E2 | 6.7E2 | 3.2E5 | 1.1E5 | 264 | 19 | 159 | 19 | 0.62 |
| Kf | pg/mL | 7.4E0 | 9.6E0 | 7.8E0 | 9.2E0 | 7.4E0 | 7.5E0 | 1.0E-9 | 1.0E-9 | 7.8E1 | 2.2E1 | 264 | 19 | 159 | 19 | 0.57 |
| Kg | pg/mL | 1.2E3 | 1.5E3 | 2.0E3 | 3.7E3 | 2.9E3 | 8.4E3 | 7.7E1 | 1.7E2 | 2.7E4 | 3.6E4 | 264 | 19 | 159 | 19 | 0.49 |
| Ki | pg/ml | 5.9E1 | 6.1E1 | 6.8E1 | 7.4E1 | 5.2E1 | 4.5E1 | 1.0E-9 | 6.0E0 | 3.8E2 | 2.1E2 | 263 | 19 | 159 | 19 | 0.56 |
| Kj | pg/ml | 9.8E2 | 9.3E2 | 1.7E3 | 1.6E3 | 1.9E3 | 1.9E3 | 3.0E1 | 1.5E2 | 1.5E4 | 7.7E3 | 264 | 19 | 159 | 19 | 0.48 |
| Kk | pg/ml | 6.8E0 | 1.1E1 | 1.2E1 | 2.0E1 | 1.6E1 | 1.9E1 | 1.0E-9 | 1.0E-9 | 1.6E2 | 5.8E1 | 264 | 19 | 159 | 19 | 0.64 |
| Kl | pg/ml | 2.1E4 | 3.0E4 | 2.8E4 | 2.9E4 | 2.6E4 | 2.3E4 | 2.3E2 | 2.4E2 | 1.6E5 | 6.8E4 | 264 | 19 | 159 | 19 | 0.52 |
| Kn | pg/ml | 3.0E1 | 3.0E1 | 8.1E1 | 1.1E2 | 3.1E2 | 1.8E2 | 1.0E-9 | 1.0E-9 | 4.9E3 | 6.3E2 | 264 | 19 | 159 | 19 | 0.54 |
| Ko | pg/ml | 4.1E2 | 5.0E2 | 5.2E2 | 5.2E2 | 5.2E2 | 3.7E2 | 1.0E-9 | 1.0E-9 | 4.1E3 | 1.2E3 | 264 | 19 | 159 | 19 | 0.53 |
| Kp | pg/ml | 3.4E2 | 3.7E2 | 4.1E2 | 4.8E2 | 8.4E2 | 3.1E2 | 1.0E-9 | 1.0E-9 | 1.3E4 | 1.1E3 | 264 | 19 | 159 | 19 | 0.61 |
| Kq | pg/ml | 3.2E2 | 5.2E2 | 1.1E3 | 1.5E3 | 9.9E3 | 2.8E3 | 5.1E0 | 1.6E0 | 1.6E5 | 1.2E4 | 257 | 19 | 154 | 19 | 0.67 |
| Kr | pg/ml | 5.6E-1 | 2.9E-1 | 4.0E0 | 1.7E0 | 2.6E1 | 2.6E0 | 1.0E-9 | 1.0E-9 | 4.2E2 | 8.2E0 | 257 | 19 | 154 | 19 | 0.49 |
| Ks | pg/ml | 1.4E4 | 7.4E3 | 2.0E4 | 1.3E4 | 1.9E4 | 1.3E4 | 2.2E2 | 5.1E1 | 1.1E5 | 5.0E4 | 257 | 19 | 154 | 19 | 0.38 |
| Kx | ng/ml | 1.0E-9 | 4.6E-3 | 6.9E-3 | 1.0E-2 | 1.4E-2 | 1.8E-2 | 1.0E-9 | 1.0E-9 | 1.0E-1 | 7.9E-2 | 263 | 19 | 159 | 19 | 0.60 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Ky | ng/ml | 9.8E-2 | 2.1E-1 | 3.8E-1 | 6.4E-1 | 8.4E-1 | 1.1E0 | 1.0E-9 | 1.0E-9 | 6.4E0 | 4.4E0 | 263 | 19 | 159 | 19 | 0.62 |
| Kz | ng/ml | 1.0E-9 | 1.0E-9 | 3.7E-3 | 3.0E-3 | 5.8E-3 | 6.5E-3 | 1.0E-9 | 1.0E-9 | 3.6E-2 | 2.5E-2 | 263 | 19 | 159 | 19 | 0.45 |
| Ld | pg/ml | 1.0E-9 | 7.5E-1 | 3.7E0 | 4.2E0 | 9.2E0 | 7.6E0 | 1.0E-9 | 1.0E-9 | 8.4E1 | 2.9E1 | 264 | 18 | 158 | 18 | 0.59 |
| Lh | pg/ml | 1.3E4 | 2.6E4 | 2.1E4 | 4.2E4 | 3.3E4 | 6.9E4 | 1.0E-9 | 1.0E-9 | 4.8E5 | 4.1E5 | 756 | 37 | 297 | 37 | 0.64 |
| Li | pg/ml | 3.2E3 | 1.1E4 | 1.7E4 | 4.4E4 | 6.6E4 | 9.0E4 | 1.0E-9 | 1.3E1 | 1.3E6 | 4.1E5 | 756 | 37 | 297 | 37 | 0.61 |
| Lj | pg/ml | 2.9E3 | 4.3E3 | 2.4E4 | 3.0E4 | 6.8E4 | 6.9E4 | 1.0E-9 | 4.5E1 | 5.2E5 | 3.9E5 | 756 | 37 | 297 | 37 | 0.53 |
| Rm | ng/ml | 1.9E1 | 4.0E1 | 5.2E1 | 5.5E1 | 8.4E1 | 4.8E1 | 2.2E-1 | 1.9E0 | 6.5E2 | 1.6E2 | 256 | 19 | 158 | 19 | 0.61 |
| Rh | ng/ml | 1.3E2 | 9.2E1 | 4.0E2 | 1.7E2 | 1.4E3 | 1.4E2 | 4.7E0 | 7.6E0 | 1.7E4 | 4.0E2 | 256 | 19 | 158 | 19 | 0.44 |
| Ri | ng/ml | 1.0E-9 | 1.0E-9 | 4.4E0 | 3.8E0 | 1.6E1 | 1.1E1 | 1.0E-9 | 1.0E-9 | 2.2E2 | 4.5E1 | 257 | 19 | 159 | 19 | 0.44 |
| Rg | ng/ml | 1.0E-9 | 1.0E-9 | 5.0E-2 | 5.4E-3 | 3.0E-1 | 1.1E-2 | 1.0E-9 | 1.0E-9 | 3.0E0 | 3.7E-2 | 256 | 19 | 158 | 19 | 0.60 |
| Rj | ng/ml | 1.0E-9 | 1.0E-9 | 2.4E0 | 3.7E-1 | 1.8E1 | 8.6E-1 | 1.0E-9 | 1.0E-9 | 2.7E2 | 3.2E0 | 257 | 19 | 159 | 19 | 0.45 |
| Rf | ng/ml | 4.1E-1 | 3.7E-1 | 1.0E0 | 2.0E0 | 1.8E0 | 3.7E0 | 7.8E-3 | 1.8E-2 | 1.5E1 | 1.4E1 | 256 | 19 | 158 | 19 | 0.53 |
| Ql | pg/ml | 4.5E0 | 5.5E0 | 1.3E1 | 1.3E1 | 2.4E1 | 1.7E1 | 1.0E-9 | 1.0E-9 | 1.8E2 | 5.6E1 | 262 | 19 | 160 | 19 | 0.55 |
| Qm | pg/ml | 4.1E0 | 1.8E1 | 2.2E1 | 2.3E1 | 4.0E1 | 2.6E1 | 1.0E-9 | 1.0E-9 | 3.6E2 | 8.5E1 | 262 | 19 | 160 | 19 | 0.54 |
| Qn | pg/ml | 6.1E-1 | 6.0E-1 | 7.4E0 | 1.6E1 | 2.4E1 | 4.5E1 | 1.0E-9 | 1.0E-9 | 2.4E2 | 2.0E2 | 262 | 19 | 160 | 19 | 0.49 |
| Nv | pg/ml | 3.8E3 | 9.4E3 | 1.0E4 | 2.0E4 | 4.5E4 | 2.9E4 | 1.0E-9 | 1.9E1 | 1.1E6 | 1.1E5 | 762 | 37 | 297 | 37 | 0.66 |
| Nw | pg/ml | 8.8E3 | 1.8E4 | 1.3E4 | 3.2E4 | 1.7E4 | 4.8E4 | 8.6E1 | 1.9E2 | 2.1E5 | 2.2E5 | 762 | 37 | 297 | 37 | 0.70 |
| Nx | pg/ml | 2.2E2 | 2.4E2 | 4.1E2 | 5.9E2 | 6.6E2 | 6.4E2 | 1.0E-9 | 1.0E-9 | 7.4E3 | 2.3E3 | 762 | 37 | 297 | 37 | 0.61 |
| Ny | pg/ml | 6.0E0 | 1.0E1 | 6.1E1 | 7.5E1 | 9.2E2 | 1.5E2 | 1.0E-9 | 1.0E-9 | 2.5E4 | 6.1E2 | 762 | 37 | 297 | 37 | 0.57 |
| Oa | pg/ml | 1.8E2 | 2.9E2 | 4.4E2 | 5.8E2 | 7.4E2 | 6.8E2 | 1.0E-9 | 1.0E-9 | 4.8E3 | 2.0E3 | 262 | 19 | 160 | 19 | 0.58 |
| Tk | ng/ml | 1.4E2 | 9.5E1 | 3.2E2 | 3.2E2 | 5.5E2 | 5.2E2 | 3.0E0 | 1.9E1 | 4.2E3 | 1.4E3 | 93 | 7 | 66 | 7 | 0.44 |
| Oe | pg/ml | 4.2E1 | 9.4E0 | 2.7E2 | 2.1E2 | 7.9E2 | 3.3E2 | 1.0E-9 | 1.0E-9 | 1.9E4 | 1.3E3 | 755 | 37 | 296 | 37 | 0.47 |
| Of | pg/ml | 1.6E2 | 9.7E1 | 5.3E3 | 3.6E3 | 2.9E4 | 9.7E3 | 1.0E-9 | 1.0E-9 | 6.2E5 | 4.7E4 | 761 | 37 | 297 | 37 | 0.46 |
| Og | pg/ml | 7.9E-2 | 6.4E-2 | 4.6E-1 | 1.2E-1 | 1.5E0 | 1.5E-1 | 1.0E-9 | 1.0E-9 | 2.0E1 | 5.0E-1 | 761 | 37 | 297 | 37 | 0.44 |
| Oh | pg/ml | 2.5E0 | 5.7E0 | 2.0E1 | 4.3E1 | 1.5E2 | 1.9E2 | 1.0E-9 | 1.0E-9 | 3.5E3 | 1.1E3 | 761 | 37 | 297 | 37 | 0.69 |
| Oi | pg/ml | 2.4E0 | 5.0E0 | 5.9E0 | 8.9E0 | 9.6E0 | 1.0E1 | 1.0E-9 | 1.0E-9 | 8.6E1 | 3.6E1 | 761 | 37 | 297 | 37 | 0.57 |
| Ok | pg/ml | 3.9E2 | 6.4E2 | 5.3E2 | 9.6E2 | 5.7E2 | 1.1E3 | 1.3E1 | 1.5E1 | 7.8E3 | 4.7E3 | 761 | 37 | 297 | 37 | 0.64 |
| Om | pg/ml | 3.8E2 | 9.9E2 | 8.2E2 | 1.7E3 | 2.6E3 | 1.9E3 | 1.0E-9 | 1.0E-9 | 5.1E4 | 6.9E3 | 761 | 37 | 297 | 37 | 0.69 |
| On | pg/ml | 1.8E2 | 3.1E2 | 2.8E2 | 7.1E2 | 4.2E2 | 1.4E3 | 1.0E-9 | 7.6E0 | 4.5E3 | 8.5E3 | 761 | 37 | 297 | 37 | 0.64 |
| Or | pg/ml | 1.4E1 | 1.5E1 | 3.5E1 | 4.7E1 | 6.7E1 | 7.6E1 | 1.0E-9 | 1.0E-9 | 5.1E2 | 2.9E2 | 265 | 19 | 159 | 19 | 0.53 |
| Ow | pg/ml | 3.3E1 | 5.6E1 | 1.5E2 | 1.9E2 | 5.7E2 | 3.6E2 | 1.0E-9 | 1.0E-9 | 8.1E3 | 1.5E3 | 265 | 19 | 159 | 19 | 0.53 |
| Ou | pg/ml | 5.1E2 | 6.4E2 | 9.5E2 | 1.8E3 | 1.5E3 | 2.3E3 | 1.0E-9 | 1.0E-9 | 9.8E3 | 9.3E3 | 265 | 19 | 159 | 19 | 0.58 |
| Ul | ng/ml | 1.0E-9 | 1.0E-9 | 1.8E0 | 9.7E-1 | 8.3E0 | 2.5E0 | 1.0E-9 | 1.0E-9 | 1.0E2 | 9.6E0 | 270 | 19 | 163 | 19 | 0.52 |
| Uo | ng/ml | 1.0E-9 | 1.0E-9 | 9.7E-2 | 6.0E-2 | 2.2E-1 | 1.8E-1 | 1.0E-9 | 1.0E-9 | 1.5E0 | 6.5E-1 | 270 | 19 | 163 | 19 | 0.41 |
| Uk | ng/ml | 1.0E-9 | 1.0E-9 | 8.5E-3 | 6.6E-4 | 2.7E-2 | 2.4E-3 | 1.0E-9 | 1.0E-9 | 3.2E-1 | 1.1E-2 | 270 | 19 | 163 | 19 | 0.34 |
| Um | ng/ml | 1.0E-9 | 1.0E-9 | 4.2E-1 | 2.8E-1 | 9.1E-1 | 6.9E-1 | 1.0E-9 | 1.0E-9 | 6.8E0 | 2.6E0 | 270 | 19 | 163 | 19 | 0.42 |
| Uf | ng/ml | 6.5E-2 | 1.6E-1 | 1.8E-1 | 5.5E-1 | 6.4E-1 | 1.2E0 | 1.3E-3 | 1.0E-3 | 8.6E0 | 5.1E0 | 270 | 19 | 163 | 19 | 0.63 |
| Uh | ng/ml | 2.1E0 | 2.8E0 | 3.4E0 | 3.5E0 | 3.7E0 | 3.8E0 | 1.3E-2 | 4.7E-2 | 2.1E1 | 1.5E1 | 270 | 19 | 163 | 19 | 0.52 |
| Un | ng/ml | 1.9E0 | 2.6E0 | 2.2E0 | 3.3E0 | 1.9E0 | 2.2E0 | 1.3E-1 | 3.4E-1 | 2.5E1 | 8.0E0 | 270 | 19 | 163 | 19 | 0.66 |
| Ug | ng/ml | 1.5E1 | 9.2E0 | 2.9E1 | 1.2E1 | 3.2E1 | 1.1E1 | 6.9E-1 | 8.8E-1 | 2.1E2 | 4.0E1 | 270 | 19 | 163 | 19 | 0.31 |
| Ur | ng/ml | 1.5E-1 | 7.8E-2 | 8.9E-1 | 2.3E-1 | 5.8E0 | 5.2E-1 | 1.0E-9 | 1.0E-9 | 9.3E1 | 2.3E0 | 269 | 19 | 162 | 19 | 0.40 |
| Up | ng/ml | 1.0E-9 | 1.0E-9 | 1.4E-2 | 3.9E-3 | 1.5E-1 | 9.6E-3 | 1.0E-9 | 1.0E-9 | 2.4E0 | 4.1E-2 | 269 | 19 | 162 | 19 | 0.53 |
| Us | ng/ml | 2.9E-3 | 2.8E-4 | 2.6E-2 | 5.0E-3 | 1.1E-1 | 7.4E-3 | 1.0E-9 | 1.0E-9 | 1.7E0 | 2.0E-2 | 269 | 19 | 162 | 19 | 0.41 |
| Uv | ng/ml | 3.1E-3 | 3.9E-3 | 1.4E-2 | 5.7E-3 | 4.7E-2 | 6.1E-3 | 1.0E-9 | 1.0E-9 | 4.3E-1 | 1.9E-2 | 269 | 19 | 162 | 19 | 0.51 |
| Ut | ng/ml | 6.6E-1 | 1.9E0 | 2.9E0 | 5.0E0 | 9.6E0 | 7.5E0 | 1.0E-9 | 1.0E-9 | 7.8E1 | 3.0E1 | 269 | 19 | 162 | 19 | 0.67 |
| Uu | ng/ml | 7.1E0 | 8.2E0 | 7.9E0 | 8.3E0 | 5.6E0 | 5.8E0 | 5.5E-1 | 1.0E0 | 4.0E1 | 2.3E1 | 269 | 19 | 162 | 19 | 0.53 |
| Vp | ng/ml | 1.0E-9 | 1.0E-9 | 5.6E-1 | 7.9E-2 | 4.6E0 | 2.1E-1 | 1.0E-9 | 1.0E-9 | 5.0E1 | 8.4E-1 | 270 | 19 | 163 | 19 | 0.54 |
| Vt | ng/ml | 6.8E0 | 6.1E0 | 9.9E0 | 1.3E1 | 1.3E1 | 1.8E1 | 4.3E-1 | 5.6E-1 | 1.6E2 | 7.6E1 | 270 | 19 | 163 | 19 | 0.51 |
| Vu | ng/ml | 1.0E-9 | 1.0E0 | 2.1E0 | 2.9E0 | 5.4E0 | 4.1E0 | 1.0E-9 | 1.0E-9 | 5.1E1 | 1.3E1 | 264 | 18 | 162 | 18 | 0.60 |
| Vq | ng/ml | 2.7E2 | 1.3E2 | 4.3E3 | 6.7E2 | 4.8E4 | 1.3E3 | 2.0E-1 | 1.8E1 | 6.8E5 | 4.5E3 | 201 | 12 | 131 | 12 | 0.49 |
| Vo | ng/ml | 2.6E1 | 2.5E1 | 2.5E1 | 2.3E1 | 4.7E0 | 6.8E0 | 6.7E0 | 4.7E0 | 3.5E1 | 3.4E1 | 270 | 19 | 163 | 19 | 0.45 |
| Vs | ng/ml | 1.0E-9 | 3.3E0 | 8.3E0 | 8.2E0 | 3.7E1 | 1.1E1 | 1.0E-9 | 1.0E-9 | 4.5E2 | 3.7E1 | 260 | 18 | 159 | 18 | 0.60 |
| Vv | ng/ml | 2.9E0 | 3.0E0 | 5.8E0 | 7.4E0 | 9.7E0 | 9.4E0 | 1.0E-9 | 1.0E-9 | 8.4E1 | 3.5E1 | 268 | 19 | 162 | 19 | 0.55 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Oy | pg/ml | 4.9E-1 | 7.1E-1 | 5.9E0 | 4.9E0 | 3.1E1 | 1.2E1 | 1.0E-9 | 1.0E-9 | 4.0E2 | 5.3E1 | 760 | 37 | 296 | 37 | 0.58 |
| Oz | pg/ml | 1.4E-3 | 2.4E-1 | 3.2E-1 | 1.1E0 | 1.4E0 | 4.6E0 | 1.0E-9 | 1.0E-9 | 2.9E1 | 2.8E1 | 760 | 37 | 296 | 37 | 0.64 |
| Pa | pg/ml | 3.8E-1 | 5.1E-1 | 1.7E0 | 7.6E0 | 6.5E0 | 3.7E1 | 1.0E-9 | 1.0E-9 | 1.0E2 | 2.3E2 | 760 | 37 | 296 | 37 | 0.58 |
| Pb | pg/ml | 1.0E-9 | 5.6E-2 | 9.0E-1 | 1.8E-1 | 1.8E1 | 3.4E-1 | 1.0E-9 | 1.0E-9 | 4.9E2 | 1.9E0 | 760 | 37 | 296 | 37 | 0.58 |
| Pc | pg/ml | 4.2E-2 | 3.8E-1 | 3.6E-1 | 1.6E0 | 9.3E-1 | 6.1E0 | 1.0E-9 | 1.0E-9 | 1.6E1 | 3.8E1 | 760 | 37 | 296 | 37 | 0.63 |
| Pd | pg/ml | 1.9E0 | 2.3E0 | 5.4E0 | 6.6E0 | 3.2E1 | 1.1E1 | 1.0E-9 | 1.0E-9 | 8.4E2 | 5.5E1 | 760 | 37 | 296 | 37 | 0.57 |
| Pe | pg/ml | 2.1E1 | 3.8E1 | 1.2E2 | 5.5E2 | 4.8E2 | 2.5E3 | 1.0E-9 | 1.0E-9 | 6.7E3 | 1.5E4 | 760 | 37 | 296 | 37 | 0.57 |
| Pf | pg/ml | 1.6E0 | 6.2E0 | 1.2E1 | 2.3E1 | 6.3E1 | 7.3E1 | 1.0E-9 | 1.0E-9 | 1.5E3 | 4.3E2 | 760 | 37 | 296 | 37 | 0.60 |
| Pg | pg/ml | 3.3E0 | 8.8E0 | 4.8E1 | 7.5E1 | 3.7E2 | 2.3E2 | 1.0E-9 | 1.0E-9 | 7.7E3 | 1.2E3 | 760 | 37 | 296 | 37 | 0.63 |
| Ph | ng/ml | 1.8E-1 | 2.8E-1 | 3.5E-1 | 5.7E-1 | 5.7E-1 | 7.2E-1 | 1.0E-9 | 1.0E-9 | 5.4E0 | 2.8E0 | 265 | 19 | 159 | 19 | 0.57 |
| Pi | ng/ml | 2.0E-1 | 2.4E-1 | 5.8E-1 | 3.3E-1 | 5.0E0 | 3.2E-1 | 1.0E-9 | 1.0E-9 | 8.2E1 | 1.2E0 | 265 | 19 | 159 | 19 | 0.54 |
| Pj | ng/mL | 5.7E0 | 4.8E0 | 6.4E0 | 8.7E0 | 4.5E0 | 1.2E1 | 3.8E-2 | 4.0E-1 | 3.1E1 | 5.5E1 | 265 | 19 | 159 | 19 | 0.47 |
| Pk | ng/ml | 8.8E-3 | 1.3E-2 | 1.8E-2 | 2.1E-2 | 9.4E-2 | 2.6E-2 | 1.0E-9 | 1.0E-9 | 1.5E0 | 1.1E-1 | 265 | 19 | 159 | 19 | 0.59 |
| aA | mg/dL | 8.0E-1 | 1.1E0 | 9.0E-1 | 1.5E0 | 4.3E-1 | 1.0E0 | 2.0E-1 | 5.0E-1 | 4.1E0 | 4.7E0 | 2356 | 52 | 452 | 52 | 0.74 |
| aC | mg/mL | 2.9E0 | 2.4E0 | 3.2E0 | 2.7E0 | 1.4E0 | 1.4E0 | 7.7E-1 | 9.7E-1 | 8.9E0 | 5.6E0 | 466 | 21 | 180 | 21 | 0.40 |
| aD | ug/mL | 3.2E0 | 3.2E0 | 4.5E0 | 5.5E0 | 4.0E0 | 5.6E0 | 4.3E-1 | 1.1E0 | 3.5E1 | 2.1E1 | 466 | 21 | 180 | 21 | 0.49 |
| aE | mg/mL | 5.6E-1 | 5.8E-1 | 5.7E-1 | 6.0E-1 | 1.5E-1 | 1.8E-1 | 1.8E-1 | 3.4E-1 | 1.1E0 | 1.0E0 | 466 | 21 | 180 | 21 | 0.55 |
| aF | ng/mL | 2.2E0 | 1.7E0 | 3.8E0 | 4.8E0 | 5.4E0 | 6.8E0 | 4.3E-3 | 4.3E-3 | 5.0E1 | 2.4E1 | 466 | 21 | 180 | 21 | 0.44 |
| aG | mg/mL | 1.3E-1 | 1.4E-1 | 1.5E-1 | 1.8E-1 | 8.7E-2 | 1.2E-1 | 1.7E-2 | 5.0E-2 | 5.4E-1 | 5.2E-1 | 466 | 21 | 180 | 21 | 0.53 |
| aH | ug/mL | 7.5E1 | 7.5E1 | 8.0E1 | 9.4E1 | 4.3E1 | 5.1E1 | 4.6E0 | 2.6E1 | 2.9E2 | 2.0E2 | 466 | 21 | 180 | 21 | 0.56 |
| aI | ug/mL | 1.9E2 | 1.9E2 | 1.9E2 | 1.8E2 | 6.0E1 | 5.7E1 | 2.8E1 | 8.0E1 | 3.7E2 | 2.5E2 | 466 | 21 | 180 | 21 | 0.49 |
| aJ | ug/mL | 2.4E0 | 4.2E0 | 3.0E0 | 5.8E0 | 2.0E0 | 5.0E0 | 7.3E-1 | 1.5E0 | 1.7E1 | 2.3E1 | 466 | 21 | 180 | 21 | 0.72 |
| aK | ng/mL | 1.5E0 | 1.6E0 | 2.4E0 | 2.5E0 | 2.6E0 | 2.5E0 | 2.9E-4 | 1.3E-1 | 1.8E1 | 1.0E1 | 466 | 21 | 180 | 21 | 0.52 |
| aL | mg/mL | 8.0E-1 | 8.3E-1 | 8.1E-1 | 8.4E-1 | 2.7E-1 | 2.0E-1 | 1.9E-1 | 4.4E-1 | 1.7E0 | 1.2E0 | 466 | 21 | 180 | 21 | 0.54 |
| aM | U/mL | 2.2E1 | 2.3E1 | 5.1E1 | 3.0E1 | 1.1E2 | 2.6E1 | 4.2E-2 | 4.2E-2 | 1.6E3 | 8.3E1 | 466 | 21 | 180 | 21 | 0.48 |
| aN | U/mL | 1.3E1 | 2.3E1 | 2.0E1 | 2.8E1 | 2.6E1 | 2.8E1 | 2.5E-3 | 2.8E0 | 3.3E2 | 1.1E2 | 466 | 21 | 180 | 21 | 0.63 |
| aO | pg/mL | 2.6E1 | 7.2E1 | 2.8E2 | 6.0E2 | 7.5E2 | 1.1E3 | 6.0E-2 | 6.0E-2 | 6.6E3 | 3.9E3 | 466 | 21 | 180 | 21 | 0.58 |
| aP | ng/mL | 1.6E0 | 2.4E0 | 2.0E0 | 3.9E0 | 1.8E0 | 5.7E0 | 4.5E-1 | 1.1E0 | 2.8E1 | 2.8E1 | 466 | 21 | 180 | 21 | 0.70 |
| aQ | ng/mL | 2.9E-1 | 4.5E-1 | 4.4E-1 | 3.9E-1 | 4.6E-1 | 2.7E-1 | 2.0E-4 | 5.2E-2 | 4.0E0 | 1.1E0 | 466 | 21 | 180 | 21 | 0.52 |
| aR | ng/mL | 1.8E0 | 2.0E0 | 2.8E0 | 3.2E0 | 3.3E0 | 3.3E0 | 1.8E-1 | 3.6E-1 | 3.4E1 | 1.5E1 | 466 | 21 | 180 | 21 | 0.54 |
| aS | ng/mL | 2.6E-1 | 3.8E-1 | 6.4E-1 | 7.6E-1 | 1.8E0 | 1.4E0 | 4.2E-3 | 2.8E-2 | 3.3E1 | 6.6E0 | 466 | 21 | 180 | 21 | 0.55 |
| aU | pg/mL | 7.5E1 | 8.4E1 | 1.3E2 | 1.0E2 | 1.5E2 | 7.7E1 | 7.4E-2 | 7.4E-2 | 1.3E3 | 2.4E2 | 466 | 21 | 180 | 21 | 0.50 |
| aV | ng/mL | 6.1E-1 | 5.3E-1 | 1.1E0 | 8.6E-1 | 2.0E0 | 7.7E-1 | 7.6E-4 | 1.0E-1 | 3.3E1 | 3.1E0 | 466 | 21 | 180 | 21 | 0.50 |
| aW | pg/mL | 1.8E1 | 2.4E1 | 1.9E1 | 3.9E1 | 1.9E1 | 9.0E1 | 7.2E-2 | 7.2E-2 | 2.4E2 | 4.3E2 | 466 | 21 | 180 | 21 | 0.57 |
| aX | ng/mL | 9.8E0 | 1.3E1 | 1.5E1 | 3.5E1 | 1.9E1 | 5.4E1 | 3.0E-1 | 2.1E0 | 2.2E2 | 2.1E2 | 466 | 21 | 180 | 21 | 0.59 |
| aY | pg/mL | 6.0E1 | 4.8E1 | 7.7E1 | 7.7E1 | 8.6E1 | 5.8E1 | 4.1E-1 | 1.2E1 | 1.2E3 | 2.1E2 | 466 | 21 | 180 | 21 | 0.52 |
| aZ | pg/mL | 2.3E2 | 3.0E2 | 4.9E2 | 3.9E2 | 9.2E2 | 4.0E2 | 1.7E0 | 2.4E1 | 1.2E4 | 1.8E3 | 466 | 21 | 180 | 21 | 0.54 |
| bA | ng/mL | 8.4E0 | 3.2E1 | 3.5E1 | 1.7E2 | 1.0E2 | 3.6E2 | 3.0E-2 | 3.0E-2 | 9.4E2 | 1.5E3 | 466 | 21 | 180 | 21 | 0.68 |
| bB | ng/mL | 3.0E2 | 3.2E2 | 3.1E2 | 3.6E2 | 1.6E2 | 2.2E2 | 2.1E0 | 6.6E1 | 8.2E2 | 7.8E2 | 466 | 21 | 180 | 21 | 0.55 |
| bC | ng/mL | 3.5E2 | 7.0E2 | 6.2E2 | 1.0E3 | 8.2E2 | 1.1E3 | 2.7E1 | 1.3E1 | 4.7E3 | 4.0E3 | 466 | 21 | 180 | 21 | 0.59 |
| bE | mg/mL | 5.6E0 | 5.5E0 | 5.8E0 | 6.7E0 | 2.1E0 | 2.7E0 | 9.8E-1 | 3.1E0 | 1.3E1 | 1.3E1 | 466 | 21 | 180 | 21 | 0.56 |
| bF | pg/mL | 2.1E1 | 2.1E1 | 1.6E2 | 2.2E2 | 9.3E2 | 4.8E2 | 5.0E-2 | 8.1E0 | 1.1E4 | 1.9E3 | 466 | 21 | 180 | 21 | 0.55 |
| bG | ng/mL | 1.6E0 | 2.4E0 | 2.7E0 | 3.6E0 | 3.2E0 | 5.6E0 | 2.2E-2 | 1.9E-1 | 2.3E1 | 2.6E1 | 466 | 21 | 180 | 21 | 0.55 |
| bH | pg/mL | 5.7E-1 | 5.7E-1 | 5.0E0 | 4.8E0 | 1.5E1 | 5.3E0 | 5.7E-1 | 5.7E-1 | 2.8E2 | 1.7E1 | 466 | 21 | 180 | 21 | 0.56 |
| bI | ng/mL | 4.0E-3 | 4.0E-3 | 6.5E-2 | 7.2E-2 | 1.6E-1 | 1.4E-1 | 4.0E-3 | 4.0E-3 | 1.3E0 | 5.4E-1 | 466 | 21 | 180 | 21 | 0.52 |
| bJ | mg/mL | 2.4E0 | 2.3E0 | 2.7E0 | 3.0E0 | 2.1E0 | 2.2E0 | 2.5E-4 | 2.5E-4 | 1.3E1 | 8.9E0 | 466 | 21 | 180 | 21 | 0.53 |
| bL | pg/mL | 3.7E0 | 5.6E0 | 8.4E0 | 8.8E0 | 1.1E1 | 8.6E0 | 4.6E-2 | 4.6E-2 | 8.0E1 | 3.0E1 | 466 | 21 | 180 | 21 | 0.56 |
| bM | mg/mL | 1.8E0 | 2.3E0 | 2.1E0 | 2.2E0 | 1.4E0 | 1.1E0 | 9.2E-3 | 5.1E-1 | 8.8E0 | 4.7E0 | 466 | 21 | 180 | 21 | 0.55 |
| bN | ng/mL | 4.6E1 | 2.6E1 | 1.3E2 | 9.8E1 | 2.8E2 | 1.5E2 | 1.4E-1 | 2.2E0 | 1.9E3 | 5.6E2 | 466 | 21 | 180 | 21 | 0.44 |
| bO | ng/mL | 4.0E-2 | 4.0E-2 | 1.1E1 | 1.1E1 | 2.5E1 | 2.7E1 | 4.0E-2 | 4.0E-2 | 2.0E2 | 1.2E2 | 466 | 21 | 180 | 21 | 0.47 |
| bP | mg/mL | 5.4E-1 | 8.6E-1 | 7.7E-1 | 1.0E0 | 6.9E-1 | 7.6E-1 | 4.9E-2 | 1.4E-1 | 4.8E0 | 2.7E0 | 466 | 21 | 180 | 21 | 0.62 |
| bQ | pg/mL | 1.6E1 | 1.8E1 | 6.2E1 | 4.7E1 | 6.3E2 | 5.8E1 | 1.5E-1 | 1.5E-1 | 1.3E4 | 1.9E2 | 466 | 21 | 180 | 21 | 0.56 |
| bR | ng/mL | 1.2E-2 | 1.2E-2 | 1.3E-1 | 1.1E-1 | 4.5E-1 | 1.7E-1 | 1.2E-2 | 1.2E-2 | 8.7E0 | 4.8E-1 | 466 | 21 | 180 | 21 | 0.47 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| bS | pg/mL | 9.4E-1 | 9.4E-1 | 7.1E0 | 7.3E0 | 2.8E1 | 1.6E1 | 9.4E-1 | 9.4E-1 | 3.9E2 | 6.7E1 | 466 | 21 | 180 | 21 | 0.52 |
| bU | ng/mL | 1.2E-1 | 1.3E-2 | 2.0E-1 | 1.4E-1 | 3.7E-1 | 2.0E-1 | 1.3E-2 | 1.3E-2 | 6.6E0 | 6.2E-1 | 466 | 21 | 180 | 21 | 0.42 |
| bV | pg/mL | 4.6E2 | 6.3E2 | 5.5E2 | 7.6E2 | 5.8E2 | 4.3E2 | 1.5E2 | 3.0E2 | 1.2E4 | 1.9E3 | 466 | 21 | 180 | 21 | 0.68 |
| bW | pg/mL | 3.4E2 | 4.2E2 | 6.4E2 | 9.1E2 | 1.7E3 | 1.2E3 | 8.4E1 | 1.1E2 | 2.5E4 | 3.9E3 | 466 | 21 | 180 | 21 | 0.59 |
| bX | ng/mL | 6.9E-4 | 2.5E-5 | 2.7E-3 | 2.3E-3 | 3.4E-3 | 2.8E-3 | 2.5E-5 | 2.5E-5 | 2.0E-2 | 8.5E-3 | 466 | 21 | 180 | 21 | 0.48 |
| bZ | pg/mL | 2.3E2 | 3.9E2 | 8.5E2 | 1.1E3 | 4.0E3 | 1.7E3 | 1.5E-1 | 1.2E2 | 5.8E4 | 7.4E3 | 466 | 21 | 180 | 21 | 0.65 |
| cA | pg/mL | 6.0E-1 | 6.0E-1 | 2.8E0 | 2.1E0 | 1.8E1 | 4.9E0 | 6.0E-1 | 6.0E-1 | 3.7E2 | 2.2E1 | 466 | 21 | 180 | 21 | 0.48 |
| cB | ng/mL | 5.7E-2 | 5.1E-2 | 9.0E-2 | 6.7E-2 | 1.0E-1 | 6.2E-2 | 1.7E-3 | 1.7E-3 | 5.7E-1 | 2.1E-1 | 466 | 21 | 180 | 21 | 0.46 |
| cC | pg/mL | 4.6E1 | 3.8E1 | 4.8E1 | 4.2E1 | 4.0E1 | 2.7E1 | 1.0E0 | 1.0E0 | 4.5E2 | 9.4E1 | 466 | 21 | 180 | 21 | 0.46 |
| cD | pg/mL | 5.6E0 | 4.9E0 | 1.3E1 | 2.5E1 | 4.9E1 | 5.4E1 | 3.3E-1 | 3.3E-1 | 7.2E2 | 2.2E2 | 466 | 21 | 180 | 21 | 0.47 |
| cE | pg/mL | 3.7E1 | 4.7E1 | 1.7E2 | 2.0E2 | 4.8E2 | 3.6E2 | 1.2E-1 | 1.7E0 | 3.8E3 | 1.4E3 | 466 | 21 | 180 | 21 | 0.54 |
| cF | pg/mL | 1.3E1 | 5.3E-1 | 2.0E1 | 1.1E1 | 3.0E1 | 1.9E1 | 5.3E-1 | 5.3E-1 | 2.7E2 | 6.8E1 | 466 | 21 | 180 | 21 | 0.39 |
| cG | pg/mL | 4.5E1 | 5.7E1 | 1.1E2 | 1.3E2 | 5.1E2 | 1.5E2 | 6.4E0 | 1.8E1 | 1.0E4 | 4.9E2 | 466 | 21 | 180 | 21 | 0.57 |
| cH | uIU/mL | 2.8E0 | 3.7E0 | 5.8E0 | 1.1E1 | 1.1E1 | 2.6E1 | 8.6E-3 | 5.8E-1 | 1.6E2 | 1.2E2 | 466 | 21 | 180 | 21 | 0.56 |
| cI | ng/mL | 5.5E0 | 1.2E1 | 1.1E1 | 2.1E1 | 1.5E1 | 2.8E1 | 1.0E-3 | 2.3E-1 | 1.2E2 | 1.2E2 | 466 | 21 | 180 | 21 | 0.60 |
| cJ | ug/mL | 5.6E1 | 7.0E1 | 1.1E2 | 1.2E2 | 1.4E2 | 1.1E2 | 4.0E0 | 5.6E0 | 9.6E2 | 3.4E2 | 466 | 21 | 180 | 21 | 0.56 |
| cK | ng/mL | 3.8E-3 | 3.8E-3 | 5.1E-2 | 2.9E-2 | 1.8E-1 | 7.4E-2 | 3.8E-3 | 3.8E-3 | 1.7E0 | 3.4E-1 | 466 | 21 | 180 | 21 | 0.53 |
| cL | pg/mL | 2.0E2 | 2.3E2 | 3.7E2 | 4.4E2 | 1.3E3 | 5.1E2 | 1.6E1 | 7.5E1 | 2.4E4 | 2.0E3 | 466 | 21 | 180 | 21 | 0.58 |
| cM | pg/mL | 2.6E2 | 3.0E2 | 2.9E2 | 2.9E2 | 2.0E2 | 1.5E2 | 8.7E0 | 1.2E2 | 1.6E3 | 7.2E2 | 466 | 21 | 180 | 21 | 0.51 |
| cN | pg/mL | 1.2E2 | 1.4E2 | 1.3E2 | 1.6E2 | 6.5E1 | 5.4E1 | 3.8E1 | 8.6E1 | 1.1E3 | 2.8E2 | 466 | 21 | 180 | 21 | 0.69 |
| cO | pg/mL | 2.3E2 | 2.4E2 | 3.2E2 | 3.0E2 | 9.0E2 | 2.9E2 | 5.4E1 | 9.2E1 | 1.9E4 | 1.5E3 | 466 | 21 | 180 | 21 | 0.51 |
| cP | ng/mL | 2.5E3 | 2.9E3 | 2.6E3 | 3.0E3 | 9.1E2 | 1.3E3 | 6.2E2 | 1.4E3 | 5.7E3 | 5.6E3 | 466 | 21 | 180 | 21 | 0.58 |
| cQ | ng/mL | 5.3E-2 | 4.5E-2 | 1.5E-1 | 1.0E-1 | 3.0E-1 | 2.0E-1 | 2.0E-3 | 2.0E-3 | 2.2E0 | 9.3E-1 | 466 | 21 | 180 | 21 | 0.47 |
| cR | ng/mL | 3.0E2 | 3.4E2 | 5.2E2 | 4.7E2 | 8.2E2 | 4.0E2 | 2.0E1 | 4.9E1 | 8.9E3 | 1.5E3 | 466 | 21 | 180 | 21 | 0.53 |
| cS | ng/mL | 2.5E2 | 4.3E2 | 4.0E2 | 8.4E2 | 4.0E2 | 1.5E3 | 4.1E1 | 6.6E1 | 2.7E3 | 7.1E3 | 466 | 21 | 180 | 21 | 0.65 |
| cT | ng/mL | 3.3E1 | 6.5E1 | 8.4E1 | 2.7E2 | 1.9E2 | 4.3E2 | 3.7E0 | 4.4E0 | 2.1E3 | 1.5E3 | 466 | 21 | 180 | 21 | 0.61 |
| cU | ng/mL | 5.3E1 | 7.5E1 | 7.6E1 | 9.2E1 | 1.0E2 | 7.7E1 | 5.4E0 | 1.7E1 | 1.6E3 | 3.5E2 | 466 | 21 | 180 | 21 | 0.61 |
| cV | ng/mL | 1.8E-1 | 1.5E-1 | 4.0E-1 | 1.9E-1 | 2.2E0 | 1.2E-1 | 2.5E-2 | 3.4E-2 | 4.7E1 | 4.9E-1 | 466 | 21 | 180 | 21 | 0.44 |
| cW | mIU/mL | 5.3E-2 | 4.9E-2 | 1.5E-1 | 7.8E-2 | 7.0E-1 | 6.5E-2 | 3.7E-4 | 6.4E-3 | 9.7E0 | 2.9E-1 | 466 | 21 | 180 | 21 | 0.53 |
| cX | ng/mL | 1.1E-1 | 6.4E-2 | 1.2E0 | 4.0E-1 | 3.8E0 | 8.1E-1 | 2.3E-4 | 2.3E-4 | 2.8E1 | 3.0E0 | 466 | 21 | 180 | 21 | 0.43 |
| cY | ng/mL | 8.6E0 | 6.8E0 | 1.3E1 | 1.1E1 | 1.3E1 | 1.0E1 | 1.5E-1 | 6.0E-1 | 8.3E1 | 3.9E1 | 466 | 21 | 180 | 21 | 0.46 |
| cZ | ug/mL | 1.5E1 | 1.6E1 | 1.6E1 | 1.8E1 | 7.4E0 | 8.0E0 | 2.3E0 | 7.5E0 | 5.7E1 | 4.0E1 | 466 | 21 | 180 | 21 | 0.59 |
| dA | pg/mL | 3.2E2 | 3.9E2 | 3.7E2 | 4.2E2 | 3.1E2 | 1.9E2 | 9.0E1 | 1.7E2 | 5.8E3 | 8.7E2 | 466 | 21 | 180 | 21 | 0.61 |
| dB | ug/mL | 1.7E1 | 1.9E1 | 1.8E1 | 2.0E1 | 1.6E1 | 8.7E0 | 9.4E-1 | 2.4E0 | 2.5E2 | 3.7E1 | 466 | 21 | 180 | 21 | 0.63 |
| dC | nmol/L | 3.5E1 | 3.1E1 | 3.8E1 | 4.0E1 | 1.8E1 | 2.2E1 | 7.6E0 | 1.5E1 | 1.4E2 | 9.1E1 | 466 | 21 | 180 | 21 | 0.46 |
| dD | ug/mL | 3.6E1 | 3.4E1 | 3.7E1 | 3.5E1 | 1.1E1 | 9.6E0 | 1.3E1 | 2.3E1 | 7.6E1 | 6.2E1 | 466 | 21 | 180 | 21 | 0.44 |
| dE | ng/mL | 4.8E-1 | 4.8E-1 | 6.1E-1 | 6.6E-1 | 7.0E-1 | 7.5E-1 | 8.4E-3 | 8.4E-3 | 7.2E0 | 2.4E0 | 466 | 21 | 180 | 21 | 0.50 |
| dF | ng/mL | 2.3E2 | 2.5E2 | 2.8E2 | 3.4E2 | 1.9E2 | 2.3E2 | 5.6E1 | 1.0E2 | 1.3E3 | 8.3E2 | 466 | 21 | 180 | 21 | 0.57 |
| dG | ng/mL | 1.1E1 | 1.5E1 | 1.5E1 | 1.9E1 | 1.4E1 | 1.3E1 | 2.2E0 | 6.8E0 | 1.8E2 | 6.5E1 | 466 | 21 | 180 | 21 | 0.64 |
| dH | pg/mL | 7.5E0 | 8.9E0 | 1.3E1 | 1.5E1 | 3.9E1 | 1.7E1 | 4.0E-2 | 8.3E-1 | 6.7E2 | 7.6E1 | 466 | 21 | 180 | 21 | 0.58 |
| dI | pg/mL | 4.6E-1 | 4.6E-1 | 2.3E0 | 2.5E0 | 1.6E1 | 4.4E0 | 4.6E-1 | 4.6E-1 | 3.3E2 | 1.9E1 | 466 | 21 | 180 | 21 | 0.55 |
| dJ | ng/mL | 1.9E0 | 1.9E0 | 2.2E0 | 2.4E0 | 1.1E0 | 1.6E0 | 3.2E-2 | 5.6E-1 | 6.9E0 | 5.7E0 | 466 | 21 | 180 | 21 | 0.51 |
| dK | uIU/mL | 1.9E0 | 1.6E0 | 3.1E0 | 2.3E0 | 6.5E0 | 2.6E0 | 2.8E-4 | 1.4E-2 | 7.9E1 | 1.2E1 | 466 | 21 | 180 | 21 | 0.47 |
| dL | ng/mL | 8.8E2 | 1.2E3 | 1.0E3 | 1.2E3 | 5.9E2 | 4.1E2 | 2.6E2 | 5.9E2 | 4.8E3 | 2.3E3 | 466 | 21 | 180 | 21 | 0.64 |
| dM | pg/mL | 9.5E2 | 1.3E3 | 1.2E3 | 2.0E3 | 1.4E3 | 1.9E3 | 3.4E2 | 5.2E2 | 1.6E4 | 8.8E3 | 466 | 21 | 180 | 21 | 0.66 |
| dN | ug/mL | 9.4E1 | 1.2E2 | 1.0E2 | 1.3E2 | 4.1E1 | 4.2E1 | 1.6E1 | 6.6E1 | 3.3E2 | 1.9E2 | 466 | 21 | 180 | 21 | 0.67 |
| dR | pg/ml | 1.6E3 | 1.2E3 | 2.4E3 | 1.8E3 | 2.4E3 | 1.8E3 | 1.4E2 | 1.3E2 | 1.5E4 | 7.3E3 | 312 | 19 | 171 | 19 | 0.45 |
| dU | pg/ml | 1.1E4 | 8.5E3 | 1.6E4 | 1.2E4 | 1.5E4 | 1.2E4 | 6.9E2 | 1.7E3 | 8.1E4 | 3.5E4 | 47 | 7 | 44 | 7 | 0.40 |
| dX | ng/ml | 5.2E-2 | 1.0E-1 | 1.5E-1 | 9.4E-2 | 2.5E-1 | 8.7E-2 | 2.6E-3 | 2.6E-3 | 1.5E0 | 2.3E-1 | 140 | 8 | 46 | 8 | 0.48 |
| eF | ng/ml | 4.1E0 | 5.4E0 | 5.0E0 | 6.8E0 | 4.2E0 | 5.9E0 | 1.2E0 | 2.1E0 | 4.6E1 | 2.9E1 | 324 | 19 | 172 | 19 | 0.64 |
| eC | pg/ml | 3.0E2 | 2.5E2 | 3.8E2 | 3.3E2 | 2.9E2 | 1.8E2 | 9.9E0 | 1.1E2 | 2.0E3 | 7.1E2 | 246 | 16 | 160 | 16 | 0.45 |
| eD | pg/ml | 2.1E2 | 1.7E2 | 5.0E2 | 2.7E2 | 1.1E3 | 3.7E2 | 5.2E-1 | 5.1E1 | 8.3E3 | 1.5E3 | 196 | 14 | 131 | 14 | 0.40 |
| cM | ng/ml | 3.4E0 | 3.9E0 | 4.7E0 | 1.0E1 | 4.7E0 | 1.4E1 | 6.9E-1 | 3.3E-1 | 2.7E1 | 3.9E1 | 176 | 12 | 70 | 12 | 0.56 |

| Code | Units | Median | | Average | | Standard Deviation | | Minimum | | Maximum | | Number of Samples | | Number of Patients | | AUC |
|------|-------|--------|------|---------|------|--------|------|--------|------|--------|------|--------|-----|--------|-----|-----|
| | | NonDis | Dis | NonDis | Dis | NonDis | Dis | NonDis | Dis | NonDis | Dis | NonDis | Dis | NonDis | Dis | |
| eP | ng/ml | 3.7E-3 | 4.8E-1 | 6.4E-1 | 3.5E0 | 1.5E0 | 7.8E0 | 3.7E-3 | 3.7E-3 | 1.2E1 | 2.2E1 | 140 | 8 | 46 | 8 | 0.64 |
| cQ | pg/ml | 1.0E0 | 1.0E0 | 7.8E1 | 3.9E1 | 2.5E2 | 8.3E1 | 1.0E0 | 1.0E0 | 1.6E3 | 2.2E2 | 47 | 7 | 44 | 7 | 0.49 |
| fA | ng/ml | 1.5E2 | 3.6E2 | 4.0E2 | 5.7E2 | 4.5E2 | 4.9E2 | 2.6E1 | 7.6E1 | 1.5E3 | 1.3E3 | 47 | 7 | 45 | 7 | 0.65 |
| fB | ng/ml | 6.1E2 | 5.1E2 | 6.9E2 | 6.4E2 | 2.9E2 | 3.6E2 | 1.6E2 | 3.5E2 | 1.3E3 | 1.4E3 | 48 | 7 | 46 | 7 | 0.40 |
| fP | ng/ml | 2.6E2 | 3.1E2 | 2.9E2 | 2.9E2 | 1.7E2 | 1.9E2 | 8.4E0 | 1.8E0 | 1.0E3 | 6.9E2 | 298 | 18 | 163 | 18 | 0.52 |
| fR | ng/ml | 1.3E5 | 1.9E5 | 1.8E5 | 3.1E5 | 1.5E5 | 2.5E5 | 2.9E4 | 1.9E2 | 8.3E5 | 8.7E5 | 320 | 15 | 97 | 15 | 0.66 |
| gC | ng/ml | 2.3E2 | 2.6E2 | 2.6E2 | 2.8E2 | 1.3E2 | 1.6E2 | 8.3E1 | 9.7E1 | 1.1E3 | 5.9E2 | 135 | 10 | 79 | 10 | 0.51 |
| gL | pg/ml | 6.4E4 | 6.6E4 | 7.0E4 | 9.2E4 | 2.9E4 | 5.3E4 | 1.4E4 | 2.7E4 | 2.0E5 | 2.2E5 | 312 | 19 | 171 | 19 | 0.60 |
| gP | U/ml | 2.7E2 | 3.0E2 | 2.7E2 | 3.2E2 | 9.4E1 | 2.1E2 | 1.2E1 | 6.5E1 | 8.0E2 | 1.1E3 | 320 | 19 | 172 | 19 | 0.57 |
| gW | ng/ml | 6.1E2 | 6.6E2 | 1.3E3 | 1.1E3 | 1.7E3 | 1.2E3 | 3.1E-1 | 7.4E1 | 9.5E3 | 3.8E3 | 273 | 12 | 163 | 12 | 0.53 |
| gV | ng/ml | 2.0E1 | 2.5E1 | 2.1E1 | 2.5E1 | 7.4E0 | 6.6E0 | 2.9E-3 | 1.6E1 | 4.0E1 | 3.4E1 | 110 | 7 | 25 | 7 | 0.65 |
| tF | pg/mL | 1.5E3 | 2.0E3 | 1.2E4 | 1.2E4 | 3.8E4 | 2.2E4 | 1.2E1 | 1.8E1 | 3.2E5 | 7.6E4 | 246 | 16 | 160 | 16 | 0.55 |
| gZ | ug/ml | 8.0E-1 | 6.0E0 | 6.1E1 | 4.4E1 | 1.3E2 | 8.8E1 | 8.7E-2 | 4.6E-1 | 4.1E2 | 2.4E2 | 47 | 7 | 44 | 7 | 0.66 |
| hA | ng/ml | 2.1E0 | 2.4E0 | 9.9E0 | 4.5E0 | 3.7E1 | 5.6E0 | 1.7E-2 | 2.0E-1 | 3.5E2 | 2.0E1 | 197 | 14 | 131 | 14 | 0.54 |
| nM | pg/ml | 1.0E-9 | 1.0E-9 | 7.7E1 | 1.0E-9 | 8.9E2 | 0.0E0 | 1.0E-9 | 1.0E-9 | 1.0E4 | 1.0E-9 | 133 | 17 | 101 | 17 | 0.49 |
| nN | pg/ml | 1.2E3 | 2.4E3 | 4.2E3 | 6.1E3 | 2.3E4 | 1.1E4 | 1.1E2 | 2.3E2 | 2.7E5 | 4.4E4 | 133 | 17 | 101 | 17 | 0.66 |
| nO | pg/ml | 2.6E1 | 4.4E1 | 4.5E1 | 7.0E1 | 5.3E1 | 7.9E1 | 3.5E0 | 8.9E0 | 3.1E2 | 3.4E2 | 133 | 17 | 101 | 17 | 0.63 |
| nR | pg/ml | 1.5E1 | 1.9E1 | 3.2E1 | 1.3E2 | 4.5E1 | 2.6E2 | 1.0E-9 | 1.8E0 | 2.6E2 | 1.1E3 | 133 | 17 | 101 | 17 | 0.62 |
| nT | pg/ml | 8.0E1 | 7.2E1 | 2.0E2 | 4.7E2 | 7.9E2 | 1.3E3 | 1.0E-9 | 1.0E-9 | 6.6E3 | 5.5E3 | 133 | 17 | 101 | 17 | 0.57 |
| nU | pg/ml | 2.9E1 | 1.2E2 | 2.5E2 | 7.6E2 | 1.4E3 | 2.6E3 | 1.0E-9 | 1.0E-9 | 1.3E4 | 1.1E4 | 133 | 17 | 101 | 17 | 0.68 |
| lW | pg/ml | 1.0E-9 | 1.0E-9 | 1.4E1 | 1.2E1 | 4.9E1 | 2.6E1 | 1.0E-9 | 1.0E-9 | 3.9E2 | 9.7E1 | 133 | 17 | 101 | 17 | 0.51 |
| lX | pg/ml | 9.5E2 | 1.1E3 | 1.0E3 | 9.8E2 | 5.8E2 | 3.8E2 | 1.2E2 | 4.3E2 | 2.6E3 | 1.6E3 | 133 | 17 | 101 | 17 | 0.50 |
| lY | pg/ml | 1.9E1 | 2.3E1 | 2.2E1 | 2.7E1 | 1.9E1 | 3.6E1 | 1.0E-9 | 1.0E-9 | 1.4E2 | 1.5E2 | 133 | 17 | 101 | 17 | 0.49 |
| mE | pg/ml | 1.0E-9 | 1.0E-9 | 2.9E0 | 3.7E0 | 8.5E0 | 1.0E1 | 1.0E-9 | 1.0E-9 | 5.8E1 | 4.2E1 | 133 | 17 | 101 | 17 | 0.51 |
| mF | pg/ml | 1.0E-9 | 1.0E-9 | 2.4E0 | 1.6E1 | 9.4E0 | 5.9E1 | 1.0E-9 | 1.0E-9 | 1.1E2 | 2.5E2 | 133 | 17 | 101 | 17 | 0.52 |
| mH | pg/ml | 3.6E0 | 2.3E0 | 5.3E0 | 5.0E0 | 6.6E0 | 7.5E0 | 2.3E-1 | 5.4E-1 | 5.3E1 | 3.2E1 | 133 | 17 | 101 | 17 | 0.43 |
| mI | pg/ml | 1.0E-9 | 3.7E0 | 1.5E1 | 2.5E1 | 3.1E1 | 5.4E1 | 1.0E-9 | 1.0E-9 | 1.8E2 | 2.1E2 | 133 | 17 | 101 | 17 | 0.56 |
| mM | pg/ml | 2.2E1 | 2.7E1 | 6.9E1 | 5.6E1 | 1.3E2 | 8.0E1 | 1.0E-9 | 1.0E-9 | 9.8E2 | 3.3E2 | 133 | 17 | 101 | 17 | 0.54 |
| mP | pg/ml | 1.4E1 | 1.5E1 | 1.8E1 | 2.6E1 | 2.2E1 | 3.8E1 | 1.0E-9 | 1.0E-9 | 1.9E2 | 1.7E2 | 132 | 17 | 100 | 17 | 0.60 |
| mS | pg/ml | 1.6E3 | 1.9E3 | 1.8E3 | 2.1E3 | 1.6E3 | 1.3E3 | 1.0E-9 | 6.4E2 | 1.3E4 | 4.7E3 | 133 | 17 | 101 | 17 | 0.56 |
| mT | pg/ml | 5.4E1 | 7.4E1 | 1.3E2 | 1.3E2 | 2.2E2 | 2.3E2 | 9.7E0 | 2.0E1 | 1.4E3 | 1.0E3 | 132 | 17 | 100 | 17 | 0.55 |
| mU | pg/ml | 2.2E0 | 2.9E0 | 4.0E0 | 3.8E0 | 8.3E0 | 3.7E0 | 1.0E-9 | 1.0E-9 | 6.8E1 | 1.6E1 | 132 | 17 | 100 | 17 | 0.59 |
| mW | pg/ml | 2.3E3 | 2.7E3 | 2.7E3 | 3.4E3 | 2.1E3 | 2.6E3 | 1.0E-9 | 2.0E2 | 1.8E4 | 1.1E4 | 132 | 17 | 100 | 17 | 0.60 |
| mY | pg/ml | 5.5E2 | 5.1E2 | 8.5E2 | 1.5E3 | 1.3E3 | 3.4E3 | 1.0E-9 | 6.1E1 | 1.1E4 | 1.4E4 | 133 | 17 | 101 | 17 | 0.47 |
| mZ | pg/ml | 1.7E2 | 5.5E2 | 2.9E2 | 6.3E2 | 3.0E2 | 6.1E2 | 1.0E-9 | 8.4E1 | 1.5E3 | 2.6E3 | 132 | 17 | 100 | 17 | 0.72 |
| nA | pg/ml | 1.6E0 | 3.4E0 | 1.0E1 | 1.2E1 | 4.3E1 | 1.8E1 | 1.0E-9 | 1.0E-9 | 4.4E2 | 5.5E1 | 132 | 17 | 100 | 17 | 0.64 |
| nB | pg/ml | 3.1E2 | 2.9E2 | 3.2E2 | 3.2E2 | 1.7E2 | 1.4E2 | 3.0E1 | 1.6E2 | 9.1E2 | 6.4E2 | 133 | 17 | 101 | 17 | 0.52 |
| nC | pg/ml | 1.0E-9 | 1.0E-9 | 2.5E4 | 4.3E4 | 1.5E5 | 1.7E5 | 1.0E-9 | 1.0E-9 | 1.5E6 | 6.8E5 | 133 | 17 | 101 | 17 | 0.55 |
| nD | pg/ml | 7.9E0 | 1.0E1 | 3.6E1 | 2.3E1 | 2.0E2 | 3.4E1 | 1.0E-9 | 1.0E-9 | 1.7E3 | 1.3E2 | 132 | 17 | 100 | 17 | 0.60 |
| nF | pg/ml | 1.0E-9 | 1.0E-9 | 4.5E0 | 5.6E0 | 2.5E1 | 1.3E1 | 1.0E-9 | 1.0E-9 | 2.4E2 | 3.8E1 | 133 | 17 | 101 | 17 | 0.53 |
| nH | pg/ml | 1.0E0 | 3.8E-1 | 2.2E2 | 6.2E2 | 1.3E3 | 2.4E3 | 1.0E-9 | 1.0E-9 | 1.0E4 | 1.0E4 | 132 | 17 | 100 | 17 | 0.54 |
| nI | pg/ml | 3.0E1 | 5.0E1 | 1.5E2 | 1.5E2 | 8.3E2 | 2.7E2 | 1.0E-9 | 1.0E-9 | 9.2E3 | 9.1E2 | 133 | 17 | 101 | 17 | 0.56 |
| nJ | pg/ml | 5.9E-2 | 6.1E-1 | 4.1E1 | 3.6E0 | 4.5E2 | 7.3E0 | 1.0E-9 | 1.0E-9 | 5.2E3 | 2.7E1 | 133 | 17 | 101 | 17 | 0.61 |
| nK | pg/ml | 1.0E-9 | 1.0E-9 | 5.6E1 | 1.4E2 | 3.4E2 | 4.8E2 | 1.0E-9 | 1.0E-9 | 3.2E3 | 2.0E3 | 132 | 17 | 100 | 17 | 0.51 |
| nL | pg/ml | 1.0E-9 | 1.0E-9 | 5.9E2 | 8.6E2 | 4.1E3 | 3.1E3 | 1.0E-9 | 1.0E-9 | 4.5E4 | 1.3E4 | 133 | 17 | 101 | 17 | 0.55 |
| hR | pg/ml | 2.6E4 | 2.4E4 | 2.7E4 | 2.3E4 | 1.1E4 | 5.9E3 | 1.0E-9 | 1.3E4 | 5.8E4 | 3.2E4 | 187 | 13 | 127 | 13 | 0.37 |
| hV | pg/ml | 4.4E2 | 3.3E2 | 4.6E2 | 5.4E2 | 2.4E2 | 6.3E2 | 1.0E-9 | 1.5E2 | 1.5E3 | 2.5E3 | 187 | 13 | 127 | 13 | 0.42 |
| hW | pg/ml | 1.6E3 | 2.0E3 | 2.1E3 | 3.0E3 | 3.0E3 | 3.1E3 | 1.0E-9 | 7.9E2 | 4.0E4 | 1.0E4 | 187 | 13 | 127 | 13 | 0.56 |
| hX | pg/ml | 9.0E2 | 9.9E2 | 1.0E3 | 1.1E3 | 7.3E2 | 6.7E2 | 2.5E0 | 5.2E2 | 8.6E3 | 2.6E3 | 187 | 13 | 127 | 13 | 0.51 |
| iA | pg/ml | 1.5E2 | 1.7E2 | 3.0E2 | 4.1E2 | 6.1E2 | 9.0E2 | 8.2E0 | 1.7E1 | 7.1E3 | 3.8E3 | 246 | 16 | 160 | 16 | 0.52 |
| iB | ng/ml | 4.8E0 | 6.7E0 | 6.1E0 | 1.0E1 | 5.0E0 | 9.3E0 | 3.3E-2 | 1.1E0 | 2.6E1 | 3.8E1 | 197 | 14 | 131 | 14 | 0.66 |
| iC | U/ml | 2.3E-1 | 5.6E-1 | 1.1E0 | 1.2E0 | 4.8E0 | 2.7E0 | 1.0E-9 | 5.5E-2 | 5.5E1 | 1.0E1 | 197 | 14 | 131 | 14 | 0.63 |
| iH | ng/ml | 1.6E5 | 1.6E5 | 1.6E5 | 1.6E5 | 4.8E4 | 5.0E4 | 2.9E3 | 8.1E4 | 2.7E5 | 2.5E5 | 246 | 16 | 160 | 16 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| iJ | ng/ml | 4.9E4 | 5.0E4 | 5.2E4 | 6.4E4 | 2.6E4 | 5.5E4 | 1.8E3 | 1.2E4 | 2.5E5 | 2.5E5 | 246 | 16 | 160 | 16 | 0.55 |
| hB | ng/ml | 4.5E-1 | 5.6E-1 | 5.6E-1 | 7.1E-1 | 4.5E-1 | 7.0E-1 | 1.0E-9 | 1.2E-1 | 3.2E0 | 3.0E0 | 246 | 16 | 160 | 16 | 0.55 |
| hC | pg/ml | 3.8E3 | 1.0E4 | 6.8E3 | 1.0E4 | 8.7E3 | 1.2E4 | 1.0E-9 | 4.5E1 | 5.7E4 | 5.0E4 | 246 | 16 | 160 | 16 | 0.58 |
| hF | ng/ml | 1.0E-9 | 1.0E-9 | 2.0E1 | 1.0E-9 | 2.6E2 | 0.0E0 | 1.0E-9 | 1.0E-9 | 4.0E3 | 1.0E-9 | 246 | 16 | 160 | 16 | 0.49 |
| hG | pg/ml | 6.8E3 | 6.9E3 | 7.2E3 | 9.0E3 | 3.0E3 | 4.6E3 | 2.8E1 | 3.9E3 | 2.0E4 | 1.8E4 | 246 | 16 | 160 | 16 | 0.59 |
| iO | ng/ml | 3.7E5 | 3.7E5 | 3.9E5 | 3.9E5 | 1.8E5 | 1.7E5 | 1.1E4 | 1.0E5 | 1.1E6 | 6.6E5 | 246 | 16 | 160 | 16 | 0.52 |
| iP | ng/ml | 4.8E4 | 6.6E4 | 5.6E4 | 7.6E4 | 5.6E4 | 1.0E5 | 1.0E-9 | 1.1E4 | 5.7E5 | 4.4E5 | 246 | 16 | 160 | 16 | 0.56 |
| iZ | ng/ml | 1.6E3 | 2.1E3 | 1.8E3 | 2.3E3 | 7.4E2 | 1.3E3 | 4.7E2 | 8.4E2 | 5.1E3 | 5.7E3 | 247 | 16 | 160 | 16 | 0.63 |
| jB | ng/ml | 2.4E5 | 3.4E5 | 2.6E5 | 3.0E5 | 1.2E5 | 9.2E4 | 5.7E4 | 1.3E5 | 6.2E5 | 3.9E5 | 47 | 7 | 44 | 7 | 0.64 |
| rC | pg/ml | 1.7E3 | 1.2E3 | 2.1E3 | 2.5E3 | 1.8E3 | 2.9E3 | 1.0E-9 | 3.1E2 | 1.5E4 | 1.1E4 | 187 | 13 | 127 | 13 | 0.51 |
| rB | pg/ml | 2.6E1 | 5.8E1 | 4.6E1 | 7.5E1 | 8.9E1 | 7.3E1 | 1.0E-9 | 1.0E-9 | 9.5E2 | 2.8E2 | 187 | 13 | 127 | 13 | 0.70 |
| jD | ng/ml | 3.2E1 | 3.0E1 | 4.9E1 | 6.3E1 | 6.3E1 | 1.3E2 | 1.0E-9 | 1.0E-9 | 5.1E2 | 5.1E2 | 196 | 14 | 131 | 14 | 0.47 |
| jE | ng/ml | 1.0E-9 | 1.0E-9 | 6.9E0 | 1.0E1 | 1.6E1 | 1.7E1 | 1.0E-9 | 1.0E-9 | 1.2E2 | 5.1E1 | 196 | 14 | 131 | 14 | 0.57 |
| jF | ng/ml | 3.9E1 | 2.6E1 | 5.2E1 | 5.5E1 | 5.8E1 | 6.6E1 | 1.0E-9 | 1.0E-9 | 3.5E2 | 1.9E2 | 196 | 14 | 131 | 14 | 0.49 |
| jG | ng/ml | 4.2E3 | 6.0E3 | 4.3E3 | 6.1E3 | 1.9E3 | 2.0E3 | 6.0E2 | 2.6E3 | 9.6E3 | 1.1E4 | 197 | 14 | 131 | 14 | 0.75 |
| jH | ng/ml | 7.4E1 | 9.5E1 | 8.4E1 | 1.1E2 | 5.1E1 | 5.1E1 | 1.3E1 | 5.0E1 | 4.3E2 | 2.2E2 | 197 | 14 | 131 | 14 | 0.67 |
| jI | ng/ml | 6.8E1 | 9.2E1 | 7.6E1 | 1.1E2 | 4.1E1 | 6.0E1 | 1.9E1 | 3.1E1 | 4.4E2 | 2.3E2 | 197 | 14 | 131 | 14 | 0.72 |
| rA | pg/ml | 2.6E1 | 2.5E1 | 3.1E1 | 3.1E1 | 2.4E1 | 2.5E1 | 1.0E-9 | 6.9E0 | 2.0E2 | 9.6E1 | 197 | 14 | 131 | 14 | 0.48 |
| qZ | pg/ml | 4.4E1 | 5.9E-3 | 5.4E2 | 2.9E1 | 2.1E3 | 6.6E1 | 1.0E-9 | 6.5E-4 | 1.0E4 | 2.2E2 | 152 | 11 | 115 | 11 | 0.29 |
| qY | pg/ml | 2.6E1 | 1.8E1 | 5.0E1 | 5.8E1 | 6.4E1 | 7.7E1 | 8.7E-1 | 6.9E0 | 5.3E2 | 2.8E2 | 197 | 14 | 131 | 14 | 0.52 |
| qX | pg/ml | 6.0E1 | 8.6E1 | 6.7E1 | 8.6E1 | 4.7E1 | 4.8E1 | 1.0E-9 | 1.7E1 | 2.5E2 | 1.6E2 | 197 | 14 | 131 | 14 | 0.62 |
| qW | pg/ml | 8.8E0 | 7.1E0 | 1.3E1 | 1.2E1 | 1.5E1 | 1.3E1 | 1.0E-9 | 1.0E-9 | 1.2E2 | 3.9E1 | 197 | 14 | 131 | 14 | 0.46 |
| qV | pg/ml | 2.2E3 | 2.4E3 | 2.8E3 | 3.3E3 | 2.1E3 | 2.9E3 | 1.7E2 | 1.0E2 | 9.6E3 | 1.1E4 | 197 | 14 | 131 | 14 | 0.53 |
| qU | pg/ml | 6.4E1 | 4.4E1 | 1.8E2 | 8.6E1 | 2.8E2 | 1.2E2 | 1.0E-9 | 7.1E0 | 2.1E3 | 4.5E2 | 197 | 14 | 131 | 14 | 0.41 |
| qT | pg/ml | 4.1E1 | 6.2E1 | 7.6E1 | 7.5E1 | 1.1E2 | 7.3E1 | 1.0E-9 | 2.2E1 | 9.0E2 | 3.1E2 | 197 | 14 | 131 | 14 | 0.56 |
| jK | ng/ml | 1.6E3 | 1.7E3 | 1.7E3 | 1.7E3 | 6.6E2 | 6.0E2 | 2.8E2 | 8.0E2 | 4.3E3 | 2.6E3 | 197 | 14 | 131 | 14 | 0.52 |
| jL | ng/ml | 2.0E2 | 2.8E2 | 3.0E2 | 2.9E2 | 3.0E2 | 1.8E2 | 3.5E1 | 3.7E1 | 2.1E3 | 7.1E2 | 197 | 14 | 131 | 14 | 0.57 |
| jM | ng/ml | 7.0E4 | 8.6E4 | 7.5E4 | 8.7E4 | 3.9E4 | 4.3E4 | 3.9E2 | 5.7E3 | 1.9E5 | 1.5E5 | 197 | 14 | 131 | 14 | 0.60 |
| jO | pg/ml | 2.1E5 | 2.7E5 | 2.6E5 | 2.9E5 | 1.5E5 | 1.2E5 | 5.2E4 | 1.3E5 | 1.1E6 | 4.9E5 | 197 | 14 | 131 | 14 | 0.61 |
| jP | pg/ml | 2.3E5 | 2.7E5 | 2.7E5 | 2.9E5 | 1.9E5 | 1.5E5 | 3.6E4 | 7.4E4 | 1.9E6 | 5.6E5 | 197 | 14 | 131 | 14 | 0.56 |
| jQ | pg/ml | 2.5E3 | 4.5E3 | 3.5E3 | 8.7E3 | 3.3E3 | 1.4E4 | 1.0E-9 | 5.1E2 | 1.8E4 | 5.6E4 | 197 | 14 | 131 | 14 | 0.65 |
| jR | pg/ml | 5.9E3 | 1.0E4 | 1.1E4 | 2.4E4 | 1.3E4 | 4.6E4 | 1.0E-9 | 3.0E1 | 9.0E4 | 1.8E5 | 197 | 14 | 131 | 14 | 0.62 |
| jT | pg/ml | 1.7E5 | 1.8E5 | 1.7E5 | 2.1E5 | 6.2E4 | 1.0E5 | 6.8E4 | 9.9E4 | 3.9E5 | 4.7E5 | 197 | 14 | 131 | 14 | 0.59 |
| jU | mIU/ml | 4.3E0 | 3.7E0 | 1.0E1 | 5.1E0 | 1.7E1 | 5.1E0 | 4.2E-2 | 1.1E-1 | 1.1E2 | 1.6E1 | 197 | 14 | 131 | 14 | 0.43 |
| jV | mIU/ml | 1.3E0 | 2.1E0 | 3.3E0 | 4.9E0 | 5.7E0 | 9.1E0 | 1.7E-3 | 2.2E-3 | 3.3E1 | 3.5E1 | 197 | 14 | 131 | 14 | 0.56 |
| jY | ng/ml | 7.3E-4 | 2.1E-3 | 6.3E-3 | 4.6E-3 | 2.8E-2 | 5.9E-3 | 1.0E-9 | 1.0E-9 | 3.0E-1 | 2.0E-2 | 197 | 14 | 131 | 14 | 0.57 |
| kC | pg/ml | 9.7E1 | 1.4E2 | 1.9E2 | 3.1E2 | 4.3E2 | 4.1E2 | 2.1E1 | 3.7E1 | 3.5E3 | 1.5E3 | 133 | 17 | 101 | 17 | 0.62 |
| kE | pg/ml | 1.3E5 | 1.2E5 | 1.3E5 | 1.3E5 | 4.0E4 | 3.8E4 | 1.2E4 | 5.1E4 | 2.3E5 | 1.9E5 | 133 | 17 | 101 | 17 | 0.49 |
| kF | pg/mL | 6.1E1 | 6.9E1 | 6.9E1 | 7.8E1 | 4.9E1 | 2.7E1 | 2.6E1 | 5.3E1 | 5.1E2 | 1.3E2 | 133 | 17 | 101 | 17 | 0.64 |
| kG | pg/mL | 9.1E3 | 1.1E4 | 1.2E4 | 2.1E4 | 1.3E4 | 3.6E4 | 7.5E2 | 4.4E3 | 1.2E5 | 1.6E5 | 133 | 17 | 101 | 17 | 0.60 |
| kI | pg/ml | 1.9E2 | 2.3E2 | 2.2E2 | 2.5E2 | 1.4E2 | 1.4E2 | 4.4E1 | 7.6E1 | 8.7E2 | 6.7E2 | 133 | 17 | 101 | 17 | 0.58 |
| kK | pg/ml | 1.0E2 | 1.5E2 | 1.5E2 | 4.0E2 | 1.6E2 | 5.4E2 | 6.4E0 | 3.4E1 | 1.2E3 | 1.9E3 | 133 | 17 | 101 | 17 | 0.63 |
| kN | pg/ml | 9.9E2 | 1.1E3 | 1.5E3 | 4.9E3 | 2.2E3 | 1.3E4 | 7.6E1 | 7.3E1 | 1.7E4 | 5.5E4 | 133 | 17 | 101 | 17 | 0.53 |
| kO | pg/ml | 7.1E3 | 7.2E3 | 9.6E3 | 2.0E4 | 1.7E4 | 5.0E4 | 3.4E3 | 3.7E3 | 1.5E5 | 2.1E5 | 133 | 17 | 101 | 17 | 0.53 |
| kP | pg/ml | 5.8E3 | 4.4E3 | 7.2E3 | 6.5E3 | 6.2E3 | 5.1E3 | 8.6E2 | 1.3E3 | 4.8E4 | 2.0E4 | 133 | 17 | 101 | 17 | 0.44 |
| kQ | pg/ml | 4.1E3 | 4.7E3 | 5.2E3 | 5.2E3 | 3.8E3 | 2.5E3 | 5.6E2 | 2.1E3 | 2.5E4 | 1.1E4 | 246 | 16 | 160 | 16 | 0.55 |
| kR | pg/ml | 2.1E1 | 2.0E1 | 3.0E1 | 3.2E1 | 6.8E1 | 2.7E1 | 1.0E-9 | 8.3E0 | 1.0E3 | 9.5E1 | 246 | 16 | 160 | 16 | 0.52 |
| kS | pg/ml | 7.8E2 | 9.3E2 | 9.2E2 | 1.0E3 | 6.5E2 | 5.6E2 | 7.9E1 | 3.4E2 | 4.8E3 | 2.3E3 | 246 | 16 | 160 | 16 | 0.58 |
| rZ | ng/ml | 1.0E-9 | 5.1E-3 | 5.8E-3 | 3.2E-2 | 1.5E-2 | 8.5E-2 | 1.0E-9 | 1.0E-9 | 1.1E-1 | 2.9E-1 | 187 | 11 | 127 | 11 | 0.62 |
| rY | ng/ml | 6.1E-2 | 5.3E-2 | 1.4E-1 | 1.3E0 | 5.3E-1 | 4.1E0 | 1.0E-9 | 1.0E-9 | 6.3E0 | 1.4E1 | 187 | 11 | 127 | 11 | 0.41 |
| rX | ng/ml | 1.0E-9 | 1.0E-9 | 4.0E-2 | 2.1E-1 | 2.9E-1 | 7.1E-1 | 1.0E-9 | 1.0E-9 | 3.7E0 | 2.3E0 | 187 | 11 | 127 | 11 | 0.45 |
| lK | pg/ml | 7.9E1 | 1.1E2 | 1.7E2 | 1.8E2 | 3.1E2 | 2.3E2 | 1.0E-9 | 1.0E-9 | 3.3E3 | 7.9E2 | 196 | 14 | 131 | 14 | 0.49 |
| lL | pg/ml | 1.5E3 | 3.9E3 | 2.1E3 | 3.5E3 | 2.3E3 | 2.0E3 | 1.5E1 | 2.9E2 | 1.9E4 | 7.3E3 | 197 | 14 | 131 | 14 | 0.73 |

| Code | Units | Median | | Average | | Standard Deviation | | Minimum | | Maximum | | Number of Samples | | Number of Patients | | AUC |
|------|-------|--------|------|---------|------|-------|------|---------|------|---------|------|--------|------|--------|------|------|
| | | NonDis | Dis | NonDis | Dis | NonDis | Dis | NonDis | Dis | NonDis | Dis | NonDis | Dis | NonDis | Dis | |
| lM | pg/ml | 1.1E3 | 1.6E3 | 3.6E3 | 8.6E3 | 7.0E3 | 1.6E4 | 1.3E2 | 4.1E2 | 4.4E4 | 5.1E4 | 197 | 14 | 131 | 14 | 0.56 |
| lN | pg/ml | 1.0E-9 | 1.0E-9 | 4.3E0 | 2.4E0 | 1.5E1 | 4.2E0 | 1.0E-9 | 1.0E-9 | 1.5E2 | 1.2E1 | 197 | 14 | 131 | 14 | 0.49 |
| lO | pg/ml | 1.0E-9 | 1.0E-9 | 1.8E0 | 8.2E0 | 1.4E1 | 2.3E1 | 1.0E-9 | 1.0E-9 | 1.4E2 | 8.1E1 | 196 | 14 | 131 | 14 | 0.56 |
| nW | pg/ml | 1.1E5 | 1.1E5 | 1.1E5 | 1.2E5 | 2.6E4 | 3.4E4 | 3.6E4 | 8.1E4 | 2.0E5 | 2.1E5 | 246 | 16 | 160 | 16 | 0.51 |
| nY | pg/ml | 2.0E3 | 2.6E3 | 2.3E3 | 3.4E3 | 1.3E3 | 2.7E3 | 5.1E2 | 5.7E2 | 9.9E3 | 1.0E4 | 246 | 16 | 160 | 16 | 0.61 |
| oO | pg/ml | 8.7E4 | 7.3E4 | 1.2E5 | 1.2E5 | 9.5E4 | 9.5E4 | 3.3E3 | 3.1E4 | 6.2E5 | 3.4E5 | 123 | 16 | 96 | 16 | 0.47 |
| oP | pg/ml | 1.2E5 | 1.5E5 | 1.4E5 | 2.1E5 | 8.2E4 | 1.3E5 | 2.4E4 | 4.3E4 | 4.5E5 | 4.6E5 | 123 | 16 | 96 | 16 | 0.64 |
| oQ | pg/ml | 2.9E3 | 3.6E3 | 3.8E3 | 4.6E3 | 3.0E3 | 3.8E3 | 7.7E2 | 1.4E3 | 2.0E4 | 1.7E4 | 123 | 16 | 96 | 16 | 0.57 |
| oE | pg/ml | 1.3E2 | 2.6E2 | 3.9E2 | 5.1E2 | 5.8E2 | 5.9E2 | 1.0E-9 | 1.0E-9 | 4.7E3 | 1.9E3 | 246 | 16 | 160 | 16 | 0.56 |
| oF | pg/ml | 8.0E3 | 1.1E4 | 2.1E4 | 3.4E4 | 3.3E4 | 6.1E4 | 6.4E1 | 1.8E2 | 1.7E5 | 2.5E5 | 246 | 16 | 160 | 16 | 0.55 |
| oH | pg/ml | 4.0E1 | 6.3E1 | 9.3E1 | 9.5E1 | 1.5E2 | 8.5E1 | 4.3E-1 | 1.1E1 | 9.9E2 | 3.1E2 | 246 | 16 | 160 | 16 | 0.61 |
| oK | pg/ml | 8.5E2 | 1.3E3 | 2.0E3 | 2.1E3 | 3.0E3 | 2.5E3 | 5.2E1 | 2.1E2 | 2.5E4 | 8.8E3 | 246 | 16 | 160 | 16 | 0.57 |
| oN | pg/ml | 5.1E2 | 5.5E2 | 7.6E2 | 6.3E2 | 1.4E3 | 2.9E2 | 1.1E2 | 1.6E2 | 1.8E4 | 1.2E3 | 246 | 16 | 160 | 16 | 0.55 |
| oW | pg/ml | 2.1E2 | 2.7E2 | 4.4E2 | 1.4E3 | 9.2E2 | 2.8E3 | 2.9E1 | 1.8E2 | 6.0E3 | 7.6E3 | 47 | 7 | 44 | 7 | 0.64 |
| oT | pg/ml | 3.5E2 | 2.8E2 | 3.6E2 | 3.0E2 | 1.9E2 | 1.6E2 | 9.9E1 | 1.1E2 | 7.9E2 | 5.3E2 | 47 | 7 | 44 | 7 | 0.41 |
| oV | pg/ml | 1.2E2 | 9.8E1 | 2.3E2 | 4.5E2 | 3.6E2 | 8.6E2 | 1.0E-9 | 1.0E-9 | 2.2E3 | 2.4E3 | 47 | 7 | 44 | 7 | 0.47 |
| oD | pg/ml | 1.6E4 | 1.7E4 | 1.8E4 | 2.0E4 | 7.7E3 | 9.1E3 | 6.6E3 | 8.7E3 | 4.6E4 | 3.2E4 | 47 | 7 | 44 | 7 | 0.55 |
| pF | pg/ml | 5.1E-1 | 8.3E-1 | 1.1E0 | 8.4E-1 | 5.6E0 | 5.9E-1 | 1.0E-9 | 1.4E-1 | 8.7E1 | 2.5E0 | 246 | 16 | 160 | 16 | 0.60 |
| pH | ng/ml | 8.9E0 | 8.7E0 | 1.1E1 | 1.0E1 | 9.1E0 | 7.1E0 | 1.2E0 | 3.0E0 | 4.7E1 | 2.3E1 | 47 | 7 | 44 | 7 | 0.49 |
| pI | ng/ml | 7.0E1 | 4.9E1 | 7.5E1 | 6.0E1 | 4.0E1 | 2.3E1 | 2.3E1 | 3.5E1 | 2.0E2 | 9.9E1 | 47 | 7 | 44 | 7 | 0.41 |
| pK | ng/ml | 4.8E-1 | 3.3E-1 | 5.3E-1 | 3.8E-1 | 2.9E-1 | 2.0E-1 | 1.6E-1 | 1.7E-1 | 1.6E0 | 7.7E-1 | 47 | 7 | 44 | 7 | 0.32 |

Figure 6.

Descriptive statistics for the assays used to form the panels presented in this figure.

| Code | Units | Median | | Average | | Standard Deviation | | Minimum | | Maximum | | Number of Samples | | Number of Patients | | AUC |
|------|-------|--------|--------|---------|--------|--------|--------|--------|--------|--------|--------|--------|--------|--------|--------|-----|
| | | NonDis | Dis | NonDis | Dis | NonDis | Dis | NonDis | Dis | NonDis | Dis | NonDis | Dis | NonDis | Dis | |
| Aa | mg/dL | 6.2E1 | 7.6E1 | 7.3E1 | 8.2E1 | 5.0E1 | 5.6E1 | 2.0E0 | 2.0E0 | 2.9E2 | 2.6E2 | 936 | 64 | 159 | 64 | 0.54 |
| Ad | ug/mL | 2.4E-2 | 7.3E-2 | 5.2E-2 | 2.7E-1 | 7.0E-2 | 1.2E0 | 6.8E-4 | 4.3E-3 | 3.7E-1 | 8.5E0 | 218 | 51 | 87 | 51 | 0.71 |
| Af | ng/mL | 8.9E-1 | 1.2E0 | 1.6E1 | 1.5E1 | 7.0E1 | 4.4E1 | 1.7E-3 | 1.7E-3 | 5.3E2 | 2.6E2 | 218 | 51 | 87 | 51 | 0.58 |
| Aj | ug/mL | 1.4E0 | 2.6E0 | 2.6E0 | 2.9E0 | 2.4E0 | 2.5E0 | 1.5E-3 | 2.1E-3 | 6.1E0 | 6.1E0 | 218 | 51 | 87 | 51 | 0.51 |
| Al | mg/mL | 8.8E-5 | 7.2E-5 | 2.5E-4 | 2.4E-4 | 4.2E-4 | 3.9E-4 | 2.5E-6 | 1.3E-5 | 1.9E-3 | 1.7E-3 | 218 | 51 | 87 | 51 | 0.52 |
| An | U/mL | 4.1E1 | 6.0E1 | 1.8E2 | 2.9E2 | 5.5E2 | 1.1E3 | 9.8E-4 | 9.0E-1 | 5.5E3 | 7.8E3 | 218 | 51 | 87 | 51 | 0.57 |
| Ao | pg/mL | 8.1E1 | 1.0E2 | 2.3E2 | 9.2E2 | 1.1E3 | 5.4E3 | 2.8E0 | 5.4E0 | 1.6E4 | 3.8E4 | 218 | 51 | 87 | 51 | 0.58 |
| Ap | ng/mL | 2.6E1 | 4.3E1 | 3.6E1 | 5.9E1 | 3.2E1 | 5.6E1 | 9.9E-1 | 3.2E0 | 1.7E2 | 2.9E2 | 218 | 51 | 87 | 51 | 0.66 |
| Ar | ng/mL | 6.3E-1 | 1.3E0 | 2.3E0 | 4.8E0 | 4.7E0 | 1.1E1 | 3.4E-3 | 9.5E-2 | 4.3E1 | 5.4E1 | 218 | 51 | 87 | 51 | 0.61 |
| As | ng/mL | 8.6E-3 | 1.1E-2 | 1.3E-2 | 3.6E-2 | 1.7E-2 | 1.7E-1 | 1.7E-3 | 1.7E-3 | 1.1E-1 | 1.2E0 | 218 | 51 | 87 | 51 | 0.54 |
| Aw | pg/mL | 1.5E1 | 1.8E1 | 1.6E1 | 2.0E1 | 4.9E0 | 7.7E0 | 5.0E0 | 1.1E1 | 3.2E1 | 5.1E1 | 218 | 51 | 87 | 51 | 0.67 |
| Ax | ng/mL | 2.2E0 | 4.1E0 | 9.2E0 | 3.4E1 | 1.9E1 | 1.2E2 | 1.9E-2 | 4.2E-2 | 1.5E2 | 7.7E2 | 218 | 51 | 87 | 51 | 0.55 |
| Ba | ng/mL | 4.2E1 | 1.8E2 | 3.5E2 | 6.8E2 | 9.5E2 | 1.4E3 | 3.7E-1 | 2.4E0 | 8.1E3 | 8.1E3 | 218 | 51 | 87 | 51 | 0.67 |
| Bb | ng/mL | 2.3E0 | 4.7E0 | 4.8E0 | 8.1E0 | 8.2E0 | 1.1E1 | 4.1E-3 | 3.8E-1 | 6.6E1 | 6.4E1 | 218 | 51 | 87 | 51 | 0.66 |
| Bc | ng/mL | 3.2E1 | 4.4E1 | 9.6E1 | 1.5E2 | 1.8E2 | 2.6E2 | 1.1E-1 | 2.4E0 | 1.0E3 | 1.0E3 | 218 | 51 | 87 | 51 | 0.58 |
| Bg | ng/mL | 7.1E-2 | 2.9E-1 | 3.4E0 | 1.3E1 | 2.1E1 | 5.8E1 | 5.3E-4 | 5.3E-4 | 2.5E2 | 4.0E2 | 218 | 51 | 87 | 51 | 0.65 |
| Bn | ng/mL | 5.6E-2 | 5.6E-2 | 8.6E-1 | 2.7E0 | 1.6E0 | 8.3E0 | 5.6E-2 | 5.6E-2 | 9.7E0 | 5.8E1 | 218 | 51 | 87 | 51 | 0.56 |
| Bo | ng/mL | 1.1E1 | 1.9E1 | 1.2E1 | 2.4E1 | 8.8E0 | 3.8E1 | 1.6E-2 | 1.6E-2 | 4.4E1 | 2.8E2 | 218 | 51 | 87 | 51 | 0.64 |
| Ch | uIU/mL | 1.1E0 | 1.4E0 | 7.6E0 | 5.7E1 | 2.3E1 | 1.9E2 | 3.4E-3 | 3.9E-2 | 2.1E2 | 1.2E3 | 218 | 51 | 87 | 51 | 0.60 |
| Co | pg/mL | 3.0E1 | 5.3E1 | 8.0E1 | 1.8E2 | 2.0E2 | 6.2E2 | 3.9E0 | 1.5E-1 | 1.9E3 | 4.4E3 | 218 | 51 | 87 | 51 | 0.64 |
| Cp | ng/mL | 2.0E1 | 2.5E1 | 2.4E1 | 6.3E1 | 2.5E1 | 1.8E2 | 6.0E-1 | 6.0E-1 | 2.9E2 | 1.3E3 | 218 | 51 | 87 | 51 | 0.66 |
| Cq | ng/mL | 2.4E-2 | 4.2E-2 | 1.7E-1 | 1.0E0 | 1.2E0 | 6.9E0 | 8.0E-4 | 8.0E-4 | 1.7E1 | 4.9E1 | 218 | 51 | 87 | 51 | 0.59 |
| Cs | ng/mL | 6.0E1 | 1.2E2 | 2.4E2 | 6.0E2 | 4.4E2 | 1.7E3 | 3.9E-1 | 2.2E0 | 2.9E3 | 1.1E4 | 218 | 51 | 87 | 51 | 0.57 |
| Ct | ng/mL | 1.2E0 | 8.5E-1 | 3.1E1 | 6.9E1 | 9.4E1 | 1.5E2 | 6.2E-3 | 1.1E-4 | 6.2E2 | 6.2E2 | 218 | 51 | 87 | 51 | 0.50 |
| Cu | ng/mL | 2.1E-1 | 3.6E-1 | 4.2E-1 | 1.8E0 | 8.2E-1 | 9.2E0 | 9.6E-3 | 1.5E-2 | 9.2E0 | 6.6E1 | 218 | 51 | 87 | 51 | 0.67 |
| Cv | ng/mL | 3.9E0 | 8.0E0 | 1.6E1 | 4.4E1 | 5.3E1 | 1.1E2 | 5.6E-3 | 2.4E-2 | 5.3E2 | 5.2E2 | 218 | 51 | 87 | 51 | 0.55 |
| Cw | mIU/mL | 2.7E-2 | 4.4E-2 | 3.4E-2 | 1.8E-1 | 2.4E-2 | 9.4E-1 | 2.3E-3 | 4.7E-3 | 1.4E-1 | 6.8E0 | 218 | 51 | 87 | 51 | 0.67 |
| Cx | ng/mL | 1.7E-1 | 2.0E0 | 5.1E1 | 6.7E1 | 1.0E2 | 1.1E2 | 9.3E-5 | 9.3E-5 | 3.9E2 | 3.9E2 | 218 | 51 | 87 | 51 | 0.53 |
| Db | ug/mL | 7.3E0 | 8.4E0 | 8.3E0 | 1.1E1 | 5.7E0 | 8.3E0 | 5.0E-1 | 8.7E-1 | 3.9E1 | 4.1E1 | 218 | 51 | 87 | 51 | 0.58 |
| Dc | nmol/L | 1.8E-2 | 2.8E-2 | 5.7E-2 | 3.6E-1 | 1.4E-1 | 2.0E0 | 5.2E-6 | 1.3E-3 | 1.2E0 | 1.4E1 | 218 | 51 | 87 | 51 | 0.59 |
| Dd | ug/mL | 6.8E-2 | 1.1E-1 | 1.7E-1 | 2.5E-1 | 2.6E-1 | 5.4E-1 | 1.9E-4 | 3.2E-3 | 1.6E0 | 3.6E0 | 218 | 51 | 87 | 51 | 0.53 |
| De | ng/mL | 3.4E-3 | 3.4E-3 | 6.0E-2 | 1.3E-1 | 1.1E-1 | 2.1E-1 | 3.4E-3 | 3.4E-3 | 5.9E-1 | 9.0E-1 | 218 | 51 | 87 | 51 | 0.59 |
| Dg | ng/mL | 2.5E1 | 5.4E1 | 3.7E1 | 6.1E1 | 3.4E1 | 4.1E1 | 2.4E-1 | 2.3E0 | 1.9E2 | 1.9E2 | 218 | 51 | 87 | 51 | 0.70 |
| Di | pg/mL | 1.7E0 | 2.7E0 | 2.0E0 | 3.1E0 | 1.8E0 | 2.4E0 | 1.8E-1 | 1.8E-1 | 1.3E1 | 1.1E1 | 218 | 51 | 87 | 51 | 0.65 |
| Dk | uIU/mL | 1.5E-2 | 2.3E-2 | 1.0E-1 | 1.2E-1 | 6.8E-1 | 3.4E-1 | 1.1E-4 | 1.1E-4 | 8.9E0 | 2.3E0 | 218 | 51 | 87 | 51 | 0.66 |
| Dl | ng/mL | 2.1E2 | 3.9E2 | 2.7E2 | 4.6E2 | 2.4E2 | 3.7E2 | 1.7E0 | 1.0E1 | 1.4E3 | 1.6E3 | 218 | 51 | 87 | 51 | 0.66 |
| Dp | ng/ml | 2.4E0 | 2.6E0 | 4.5E0 | 9.5E0 | 6.6E0 | 3.0E1 | 3.7E-3 | 3.7E-3 | 4.3E1 | 2.0E2 | 111 | 47 | 86 | 47 | 0.49 |
| Dr | pg/ml | 2.2E1 | 3.2E1 | 4.6E1 | 5.9E2 | 6.4E1 | 2.2E3 | 7.5E-1 | 3.5E0 | 2.9E2 | 1.0E4 | 78 | 22 | 39 | 22 | 0.66 |
| Du | pg/ml | 2.7E1 | 6.3E2 | 5.5E2 | 1.9E3 | 1.3E3 | 5.3E3 | 1.2E0 | 1.2E0 | 7.0E3 | 2.4E4 | 45 | 19 | 35 | 19 | 0.65 |
| Dw | ng/ml | 2.6E-2 | 2.0E-2 | 8.9E-2 | 4.3E-2 | 1.5E-1 | 6.1E-2 | 9.2E-3 | 9.2E-3 | 6.7E-1 | 1.9E-1 | 29 | 8 | 6 | 8 | 0.39 |
| Ef | ng/ml | 9.5E-2 | 2.6E-1 | 5.6E-1 | 1.4E0 | 1.3E0 | 2.5E0 | 5.7E-4 | 1.1E-2 | 9.4E0 | 9.5E0 | 143 | 49 | 86 | 49 | 0.65 |
| Wm | % | 7.0E-1 | 1.1E0 | 3.4E1 | 4.8E1 | 2.1E2 | 1.9E2 | 8.5E-2 | 8.5E-2 | 2.4E3 | 1.0E3 | 173 | 51 | 101 | 51 | 0.54 |
| Ed | pg/ml | 7.1E0 | 4.4E0 | 1.0E2 | 3.6E1 | 6.9E2 | 5.0E1 | 5.2E-1 | 5.2E-1 | 7.3E3 | 1.9E2 | 111 | 47 | 85 | 47 | 0.51 |
| Eo | ng/ml | 2.3E0 | 1.8E0 | 4.1E0 | 2.8E0 | 4.6E0 | 2.6E0 | 1.3E-1 | 3.6E-1 | 1.6E1 | 6.9E0 | 29 | 8 | 6 | 8 | 0.47 |
| Yf | ng/mL | 1.7E1 | 1.4E1 | 1.6E2 | 7.3E1 | 9.1E2 | 1.5E2 | 2.9E-1 | 2.9E-1 | 6.6E3 | 5.9E2 | 52 | 19 | 41 | 19 | 0.43 |
| Tj | pg/mL | 3.7E-1 | 3.7E-1 | 8.9E1 | 1.4E1 | 4.0E2 | 3.8E1 | 3.6E-1 | 3.7E-1 | 3.5E3 | 2.1E2 | 141 | 49 | 86 | 49 | 0.45 |
| Po | pg/ml | 2.6E-1 | 5.2E0 | 7.7E0 | 1.5E1 | 2.5E1 | 3.3E1 | 8.0E-3 | 2.6E-2 | 2.7E2 | 2.2E2 | 316 | 77 | 135 | 77 | 0.64 |
| Ti | ug/mL | 2.5E0 | 4.9E0 | 3.7E0 | 5.8E0 | 3.4E0 | 4.2E0 | 1.2E-1 | 9.7E-1 | 1.5E1 | 1.8E1 | 77 | 24 | 61 | 24 | 0.69 |
| Em | ng/ml | 2.9E-3 | 8.2E-3 | 6.8E-2 | 1.1E-1 | 1.2E-1 | 3.7E-1 | 2.8E-16 | 2.8E-16 | 5.4E-1 | 1.9E0 | 93 | 28 | 40 | 28 | 0.47 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|--------|--------|--------|-----|
| | | NonDis | Dis | NonDis | Dis | NonDis | Dis | NonDis | Dis | NonDis | Dis | NonDis | Dis | NonDis | Dis | |
| Et | ng/ml | 1.1E3 | 2.3E3 | 1.4E3 | 2.3E3 | 1.0E3 | 1.3E3 | 7.7E1 | 1.3E2 | 4.2E3 | 5.0E3 | 316 | 77 | 135 | 77 | 0.70 |
| Eq | pg/ml | 1.3E2 | 3.2E2 | 3.0E2 | 3.9E2 | 3.9E2 | 4.0E2 | 1.0E0 | 1.0E0 | 1.8E3 | 1.3E3 | 45 | 19 | 35 | 19 | 0.54 |
| Ew | U/ml | 2.0E0 | 1.9E0 | 2.2E0 | 2.1E0 | 1.5E0 | 8.8E-1 | 6.5E-1 | 1.3E0 | 8.8E0 | 3.5E0 | 29 | 8 | 6 | 8 | 0.54 |
| Th | ug/mL | 1.1E0 | 1.6E0 | 1.6E0 | 2.1E0 | 1.7E0 | 1.8E0 | 2.6E-3 | 3.7E-1 | 1.2E1 | 7.5E0 | 77 | 24 | 61 | 24 | 0.59 |
| Fa | ng/ml | 4.0E1 | 5.3E1 | 1.4E2 | 1.3E2 | 7.9E2 | 2.1E2 | 3.4E-2 | 6.0E-1 | 8.0E3 | 1.0E3 | 109 | 45 | 86 | 45 | 0.62 |
| Ez | ng/ml | 5.0E0 | 5.0E0 | 2.4E1 | 2.4E1 | 7.6E1 | 6.7E1 | 1.3E-2 | 1.3E-2 | 7.1E2 | 4.5E2 | 111 | 47 | 86 | 47 | 0.55 |
| Fb | ng/ml | 2.3E1 | 2.7E1 | 2.2E1 | 2.7E1 | 1.2E1 | 6.9E0 | 1.0E0 | 6.6E-1 | 5.7E1 | 4.3E1 | 109 | 46 | 86 | 46 | 0.64 |
| Ex | ng/ml | 6.2E-2 | 1.2E-1 | 2.6E-1 | 2.3E-1 | 9.1E-1 | 3.0E-1 | 3.5E-5 | 1.7E-4 | 8.9E0 | 1.2E0 | 102 | 42 | 53 | 42 | 0.62 |
| Fc | pg/ml | 2.2E-1 | 2.2E-1 | 3.3E2 | 3.8E2 | 2.2E3 | 1.1E2 | 2.2E-1 | 2.2E-1 | 1.5E4 | 3.9E2 | 45 | 20 | 35 | 20 | 0.52 |
| Fd | pg/ml | 9.8E-1 | 1.2E2 | 1.0E3 | 1.6E3 | 4.9E3 | 5.6E3 | 4.5E-1 | 9.8E-1 | 3.3E4 | 2.5E4 | 45 | 20 | 35 | 20 | 0.55 |
| Fi | pg/ml | 2.5E-1 | 2.5E-1 | 3.5E2 | 1.1E2 | 2.1E3 | 4.1E2 | 2.5E-1 | 2.5E-1 | 1.4E4 | 1.8E3 | 45 | 20 | 35 | 20 | 0.51 |
| Fn | ng/ml | 2.1E-1 | 2.1E-1 | 9.2E0 | 2.6E0 | 4.0E1 | 5.1E1 | 1.1E-14 | 1.1E-14 | 4.2E2 | 2.7E1 | 111 | 47 | 86 | 47 | 0.44 |
| Fp | ng/ml | 1.0E1 | 2.3E1 | 2.1E1 | 3.4E1 | 2.7E1 | 3.4E1 | 6.0E-3 | 5.8E-2 | 1.4E2 | 1.3E2 | 333 | 77 | 136 | 77 | 0.62 |
| Fr | ng/ml | 2.5E4 | 6.2E4 | 9.7E4 | 2.1E5 | 1.7E5 | 2.6E5 | 6.4E2 | 1.8E3 | 8.4E5 | 8.4E5 | 398 | 81 | 137 | 81 | 0.67 |
| Fw | pg/ml | 8.5E-1 | 2.7E0 | 1.1E2 | 2.4E1 | 7.2E2 | 6.1E1 | 1.1E-14 | 1.2E-1 | 6.9E3 | 3.3E2 | 144 | 50 | 87 | 50 | 0.55 |
| Fy | ng/ml | 3.1E1 | 4.4E1 | 4.9E1 | 8.6E1 | 5.5E1 | 1.3E2 | 1.8E-1 | 1.2E-1 | 3.3E2 | 6.5E2 | 110 | 46 | 85 | 46 | 0.61 |
| Gh | pg/ml | 2.3E0 | 2.0E0 | 6.2E1 | 8.5E1 | 2.7E2 | 3.4E2 | 2.9E-2 | 2.9E-2 | 1.8E3 | 1.5E3 | 45 | 19 | 35 | 19 | 0.43 |
| Gb | % | 3.3E1 | 4.5E1 | 4.1E1 | 5.9E1 | 3.6E1 | 6.3E1 | 3.1E0 | 1.4E1 | 1.9E2 | 3.0E2 | 45 | 20 | 34 | 20 | 0.62 |
| Gc | ng/ml | 8.4E1 | 1.4E2 | 1.1E2 | 2.0E2 | 1.1E2 | 1.7E2 | 6.4E0 | 3.8E1 | 7.3E2 | 6.3E2 | 82 | 22 | 40 | 22 | 0.71 |
| Gd | ng/ml | 3.1E1 | 3.8E1 | 3.1E1 | 3.9E1 | 1.5E1 | 2.3E1 | 5.4E0 | 7.6E0 | 6.9E1 | 8.0E1 | 95 | 24 | 40 | 24 | 0.59 |
| Gn | U/ml | 5.1E-1 | 2.0E-1 | 1.8E0 | 5.6E0 | 4.1E0 | 2.4E1 | 1.3E-3 | 5.6E-3 | 3.0E1 | 1.1E2 | 74 | 22 | 39 | 22 | 0.41 |
| Gl | pg/ml | 6.0E3 | 1.1E4 | 9.8E3 | 1.3E4 | 9.0E3 | 9.3E3 | 2.3E2 | 6.7E2 | 3.2E4 | 3.1E4 | 140 | 50 | 87 | 50 | 0.63 |
| Gp | U/ml | 1.8E0 | 1.1E0 | 4.4E0 | 2.5E0 | 8.0E0 | 4.1E0 | 1.3E-3 | 1.5E-2 | 6.7E1 | 2.0E1 | 144 | 49 | 87 | 49 | 0.40 |
| Gz | ug/ml | 1.2E0 | 1.6E0 | 1.2E1 | 8.5E0 | 5.8E1 | 2.3E1 | 2.9E-16 | 8.0E-2 | 4.8E2 | 1.5E2 | 69 | 40 | 52 | 40 | 0.54 |
| IIa | ng/ml | 2.7E0 | 3.4E0 | 9.0E0 | 8.7E0 | 2.0E1 | 1.6E1 | 1.7E-2 | 1.4E-3 | 1.0E2 | 1.0E2 | 109 | 47 | 85 | 47 | 0.53 |
| Nm | pg/ml | 1.4E4 | 2.2E4 | 2.6E4 | 5.8E4 | 5.1E4 | 1.4E5 | 1.0E-9 | 1.0E-9 | 7.8E5 | 9.6E5 | 315 | 79 | 136 | 79 | 0.58 |
| Nn | pg/ml | 1.2E2 | 1.9E2 | 2.7E3 | 4.7E3 | 1.1E4 | 1.9E4 | 1.0E-9 | 1.0E-9 | 1.0E5 | 1.1E5 | 315 | 79 | 136 | 79 | 0.60 |
| No | pg/ml | 1.4E1 | 2.1E1 | 2.7E1 | 6.3E1 | 5.7E1 | 1.4E2 | 1.0E-9 | 3.2E-1 | 5.9E2 | 7.7E2 | 315 | 79 | 136 | 79 | 0.59 |
| Nq | pg/ml | 2.8E0 | 4.6E0 | 2.2E1 | 3.3E1 | 8.9E1 | 9.2E1 | 1.0E-9 | 1.0E-9 | 1.2E3 | 6.7E2 | 315 | 79 | 136 | 79 | 0.53 |
| Nr | pg/ml | 6.0E-1 | 3.2E0 | 1.4E1 | 4.5E1 | 6.9E1 | 1.7E2 | 1.0E-9 | 1.0E-9 | 9.8E2 | 1.4E3 | 315 | 79 | 136 | 79 | 0.63 |
| Ns | pg/ml | 1.0E-9 | 1.0E-9 | 1.3E1 | 7.5E0 | 7.8E1 | 3.6E1 | 1.0E-9 | 1.0E-9 | 1.1E3 | 2.4E2 | 315 | 79 | 136 | 79 | 0.49 |
| Nt | pg/ml | 9.9E1 | 1.3E2 | 1.4E2 | 1.8E2 | 1.3E2 | 1.7E2 | 1.5E1 | 1.2E1 | 1.5E3 | 1.2E3 | 315 | 79 | 136 | 79 | 0.61 |
| Nu | pg/ml | 2.0E1 | 4.3E1 | 5.2E1 | 6.5E1 | 8.8E1 | 7.9E1 | 1.0E-9 | 1.0E-9 | 8.9E2 | 3.7E2 | 315 | 79 | 136 | 79 | 0.58 |
| Lu | pg/ml | 1.0E4 | 1.0E4 | 1.6E4 | 1.1E4 | 4.2E4 | 1.1E4 | 6.7E2 | 1.1E3 | 5.6E5 | 5.9E4 | 317 | 79 | 136 | 79 | 0.46 |
| Lv | pg/ml | 1.0E-9 | 9.9E0 | 1.1E1 | 2.7E1 | 2.2E1 | 4.0E1 | 1.0E-9 | 1.0E-9 | 2.4E2 | 1.9E2 | 317 | 79 | 136 | 79 | 0.63 |
| Lw | pg/ml | 1.0E-9 | 1.0E-9 | 1.3E-1 | 2.2E0 | 1.8E0 | 1.0E1 | 1.0E-9 | 1.0E-9 | 3.2E1 | 8.0E1 | 317 | 79 | 136 | 79 | 0.53 |
| Lx | pg/ml | 1.0E-9 | 6.3E1 | 1.2E2 | 3.2E2 | 4.3E2 | 5.9E2 | 1.0E-9 | 1.0E-9 | 6.2E3 | 2.8E3 | 317 | 79 | 136 | 79 | 0.65 |
| Ly | pg/ml | 1.0E-9 | 1.0E-9 | 9.2E0 | 1.1E1 | 1.8E1 | 2.1E1 | 1.0E-9 | 1.0E-9 | 1.2E2 | 9.6E1 | 317 | 79 | 136 | 79 | 0.51 |
| Lz | pg/ml | 1.0E-9 | 1.0E-9 | 2.5E0 | 7.0E0 | 2.5E1 | 3.2E1 | 1.0E-9 | 1.0E-9 | 4.3E2 | 2.6E2 | 317 | 79 | 136 | 79 | 0.53 |
| Ma | pg/ml | 2.7E2 | 7.3E2 | 1.4E3 | 3.0E3 | 4.6E3 | 7.3E3 | 1.0E-9 | 1.0E-9 | 6.5E4 | 5.2E4 | 317 | 79 | 136 | 79 | 0.63 |
| Mb | pg/ml | 2.5E1 | 3.7E1 | 3.0E1 | 3.9E1 | 1.3E1 | 2.5E1 | 5.4E0 | 4.1E0 | 6.9E1 | 2.1E2 | 317 | 79 | 136 | 79 | 0.60 |
| Mc | pg/ml | 1.0E-9 | 1.0E-9 | 2.0E-2 | 1.8E-1 | 2.5E-1 | 1.5E0 | 1.0E-9 | 1.0E-9 | 4.0E0 | 1.3E1 | 317 | 79 | 136 | 79 | 0.51 |
| Md | pg/ml | 1.0E-9 | 1.0E-9 | 5.0E-1 | 9.6E-1 | 4.0E0 | 4.2E0 | 1.0E-9 | 1.0E-9 | 6.5E1 | 2.9E1 | 317 | 79 | 136 | 79 | 0.53 |
| Me | pg/ml | 3.2E1 | 2.7E1 | 2.9E1 | 3.2E1 | 1.6E1 | 4.3E1 | 1.0E-9 | 1.0E-9 | 1.2E2 | 3.2E2 | 317 | 79 | 136 | 79 | 0.42 |
| Mf | pg/ml | 1.0E-9 | 1.0E-9 | 3.8E-1 | 1.9E0 | 1.7E0 | 7.6E0 | 1.0E-9 | 1.0E-9 | 2.0E1 | 5.6E1 | 317 | 79 | 136 | 79 | 0.59 |
| Mg | pg/ml | 2.2E0 | 4.6E0 | 7.3E0 | 1.1E1 | 1.2E1 | 1.6E1 | 1.0E-9 | 1.0E-9 | 8.8E1 | 9.2E1 | 317 | 79 | 136 | 79 | 0.57 |
| Mh | pg/ml | 1.0E-9 | 1.0E-9 | 9.0E-1 | 1.2E0 | 8.0E0 | 3.6E0 | 1.0E-9 | 1.0E-9 | 1.1E2 | 1.8E1 | 317 | 79 | 136 | 79 | 0.57 |
| Mi | pg/ml | 1.0E-9 | 1.0E-9 | 7.8E-1 | 2.1E0 | 7.5E0 | 1.3E1 | 1.0E-9 | 1.0E-9 | 1.2E2 | 1.1E2 | 317 | 79 | 136 | 79 | 0.51 |
| Mj | pg/ml | 1.0E-9 | 1.0E-9 | 4.6E0 | 9.7E0 | 2.8E1 | 3.2E1 | 1.0E-9 | 1.0E-9 | 4.6E2 | 2.1E2 | 317 | 79 | 136 | 79 | 0.53 |
| Mk | pg/ml | 1.0E-9 | 3.7E0 | 1.7E1 | 1.8E1 | 1.1E2 | 6.4E1 | 1.0E-9 | 1.0E-9 | 1.7E3 | 5.0E2 | 317 | 79 | 136 | 79 | 0.54 |
| Ml | pg/ml | 1.0E-9 | 1.0E-9 | 9.5E0 | 1.6E1 | 1.2E2 | 7.1E1 | 1.0E-9 | 1.0E-9 | 2.1E3 | 5.1E2 | 317 | 79 | 136 | 79 | 0.57 |
| Mm | pg/ml | 4.6E2 | 9.4E2 | 8.4E2 | 1.6E3 | 9.7E2 | 2.0E3 | 1.0E-9 | 1.0E-9 | 6.0E3 | 1.2E4 | 317 | 79 | 136 | 79 | 0.61 |
| Mn | pg/ml | 4.9E0 | 9.7E0 | 1.0E1 | 1.2E1 | 2.6E1 | 1.3E1 | 1.0E-9 | 1.0E-9 | 3.5E2 | 7.8E1 | 317 | 79 | 136 | 79 | 0.64 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Mp | pg/ml | 1.0E-9 | 2.3E0 | 1.0E1 | 1.4E1 | 2.3E1 | 2.5E1 | 1.0E-9 | 1.0E-9 | 1.8E2 | 1.3E2 | 317 | 79 | 136 | 79 | 0.57 |
| Mq | pg/ml | 1.0E-9 | 1.0E-9 | 2.9E0 | 6.3E0 | 1.8E1 | 2.0E1 | 1.0E-9 | 1.0E-9 | 2.1E2 | 1.0E2 | 317 | 79 | 136 | 79 | 0.54 |
| Mr | pg/ml | 1.0E-9 | 1.0E-9 | 1.6E1 | 7.8E1 | 9.3E1 | 4.1E2 | 1.0E-9 | 1.0E-9 | 1.4E3 | 3.4E3 | 317 | 79 | 136 | 79 | 0.56 |
| Ms | pg/ml | 4.0E2 | 3.7E2 | 5.5E2 | 4.3E2 | 7.0E2 | 3.7E2 | 1.0E-9 | 1.0E-9 | 5.6E3 | 1.5E3 | 317 | 79 | 136 | 79 | 0.48 |
| Mt | pg/ml | 1.0E-9 | 1.2E0 | 1.1E1 | 4.9E1 | 6.7E1 | 3.6E2 | 1.0E-9 | 1.0E-9 | 8.7E2 | 3.2E3 | 317 | 79 | 136 | 79 | 0.63 |
| Mu | pg/ml | 1.0E-9 | 1.0E-9 | 1.7E0 | 4.3E0 | 1.1E1 | 2.7E1 | 1.0E-9 | 1.0E-9 | 9.9E1 | 2.3E2 | 317 | 79 | 136 | 79 | 0.56 |
| Mv | pg/ml | 1.0E-9 | 1.0E-9 | 9.5E1 | 9.7E1 | 4.5E2 | 2.1E2 | 1.0E-9 | 1.0E-9 | 4.5E3 | 9.2E2 | 317 | 79 | 136 | 79 | 0.58 |
| Mw | pg/ml | 2.3E1 | 4.6E1 | 6.3E2 | 4.9E2 | 4.1E3 | 1.3E3 | 1.0E-9 | 1.0E-9 | 6.2E4 | 7.9E3 | 317 | 79 | 136 | 79 | 0.59 |
| Mx | pg/ml | 1.0E-9 | 1.0E-9 | 2.1E-1 | 9.8E-1 | 8.3E-1 | 4.2E0 | 1.0E-9 | 1.0E-9 | 7.4E0 | 3.2E1 | 317 | 79 | 136 | 79 | 0.56 |
| My | pg/ml | 1.0E-9 | 1.0E-9 | 6.1E2 | 3.8E2 | 3.7E3 | 1.5E3 | 1.0E-9 | 1.0E-9 | 3.9E4 | 1.1E4 | 317 | 79 | 136 | 79 | 0.50 |
| Mz | pg/ml | 9.2E0 | 1.2E1 | 2.3E1 | 6.9E1 | 6.3E1 | 2.6E2 | 1.0E-9 | 1.0E-9 | 8.4E2 | 1.9E3 | 317 | 79 | 136 | 79 | 0.60 |
| Na | pg/ml | 1.0E-9 | 1.0E-9 | 5.3E-1 | 1.3E0 | 1.7E0 | 5.1E0 | 1.0E-9 | 1.0E-9 | 9.6E0 | 4.2E1 | 317 | 79 | 136 | 79 | 0.52 |
| Nb | pg/ml | 1.9E0 | 2.4E0 | 4.9E0 | 7.9E0 | 1.7E1 | 2.6E1 | 1.0E-9 | 1.0E-9 | 2.3E2 | 2.1E2 | 317 | 79 | 136 | 79 | 0.57 |
| Nc | pg/ml | 4.8E2 | 1.9E2 | 7.1E2 | 3.3E2 | 8.7E2 | 4.3E2 | 1.0E-9 | 1.0E-9 | 9.2E3 | 1.8E3 | 317 | 79 | 136 | 79 | 0.34 |
| Nd | pg/ml | 3.0E1 | 5.8E0 | 2.6E1 | 1.6E1 | 2.0E1 | 1.7E1 | 1.0E-9 | 1.0E-9 | 8.4E1 | 5.8E1 | 317 | 79 | 136 | 79 | 0.34 |
| Ne | pg/ml | 5.2E2 | 2.9E2 | 6.7E2 | 3.8E2 | 6.7E2 | 3.9E2 | 1.0E-9 | 1.0E-9 | 7.0E3 | 1.7E3 | 317 | 79 | 136 | 79 | 0.35 |
| Nf | pg/ml | 1.0E-9 | 1.0E-9 | 2.8E0 | 3.8E0 | 8.7E0 | 1.9E1 | 1.0E-9 | 1.0E-9 | 8.2E1 | 1.3E2 | 317 | 79 | 136 | 79 | 0.46 |
| Ng | pg/ml | 3.8E1 | 4.9E1 | 1.5E2 | 1.4E2 | 2.9E2 | 2.4E2 | 1.0E-9 | 1.0E-9 | 2.3E3 | 1.2E3 | 317 | 79 | 136 | 79 | 0.52 |
| Nh | pg/ml | 7.7E1 | 4.2E1 | 1.0E2 | 6.5E1 | 9.1E1 | 6.9E1 | 1.0E-9 | 3.1E0 | 5.6E2 | 3.1E2 | 317 | 79 | 136 | 79 | 0.35 |
| Ni | pg/ml | 4.5E0 | 1.4E1 | 8.0E1 | 1.1E2 | 1.3E2 | 1.8E2 | 1.0E-9 | 1.0E-9 | 7.1E2 | 1.1E3 | 317 | 79 | 136 | 79 | 0.54 |
| Nj | pg/ml | 8.9E0 | 3.5E0 | 1.2E1 | 7.4E0 | 1.2E1 | 1.0E1 | 1.0E-9 | 1.0E-9 | 8.3E1 | 5.8E1 | 317 | 79 | 136 | 79 | 0.35 |
| Nk | pg/ml | 2.4E1 | 1.4E1 | 3.7E1 | 2.4E1 | 4.1E1 | 3.1E1 | 1.0E-9 | 1.0E-9 | 2.0E2 | 1.5E2 | 317 | 79 | 136 | 79 | 0.42 |
| Nl | pg/ml | 5.6E1 | 2.5E1 | 7.3E1 | 3.8E1 | 8.5E1 | 4.1E1 | 1.0E-9 | 1.0E-9 | 1.1E3 | 1.6E2 | 317 | 79 | 136 | 79 | 0.34 |
| Hl | pg/ml | 1.2E1 | 1.7E1 | 5.0E1 | 2.0E2 | 8.5E1 | 8.0E2 | 1.0E-9 | 1.0E-9 | 3.5E2 | 3.6E3 | 45 | 20 | 35 | 20 | 0.48 |
| Ilo | pg/ml | 1.5E1 | 1.9E1 | 3.4E1 | 4.0E1 | 1.0E2 | 8.4E1 | 1.0E-9 | 6.7E0 | 7.0E2 | 3.9E2 | 45 | 20 | 35 | 20 | 0.63 |
| Hp | ng/ml | 1.7E0 | 1.6E0 | 6.4E1 | 1.8E2 | 2.2E2 | 3.6E2 | 1.0E-9 | 3.5E-1 | 8.9E2 | 8.9E2 | 45 | 20 | 35 | 20 | 0.53 |
| Tz | pg/ml | 4.8E3 | 7.5E3 | 8.4E3 | 1.7E4 | 1.2E4 | 5.4E4 | 7.4E1 | 3.3E2 | 8.8E4 | 3.7E5 | 113 | 46 | 84 | 46 | 0.60 |
| Ua | pg/ml | 3.8E3 | 5.6E3 | 1.3E4 | 1.4E4 | 2.5E4 | 1.7E4 | 3.5E2 | 5.3E2 | 1.4E5 | 6.6E4 | 113 | 46 | 84 | 46 | 0.61 |
| Ub | pg/ml | 5.3E2 | 4.4E2 | 7.9E2 | 7.0E2 | 1.2E3 | 8.1E2 | 1.0E-9 | 1.2E1 | 9.8E3 | 4.4E3 | 113 | 46 | 84 | 46 | 0.50 |
| Ue | pg/ml | 3.0E1 | 2.9E1 | 3.2E1 | 4.7E1 | 1.9E1 | 4.8E1 | 4.2E0 | 5.2E0 | 9.5E1 | 2.7E2 | 113 | 46 | 84 | 46 | 0.55 |
| Uc | pg/ml | 8.9E2 | 1.2E3 | 1.8E3 | 3.2E3 | 3.4E3 | 8.5E3 | 6.1E-1 | 5.5E1 | 2.9E4 | 5.7E4 | 113 | 46 | 84 | 46 | 0.59 |
| Ud | pg/ml | 1.0E-9 | 1.0E-9 | 3.7E0 | 1.2E0 | 3.7E1 | 7.9E0 | 1.0E-9 | 1.0E-9 | 3.9E2 | 5.3E1 | 113 | 46 | 84 | 46 | 0.50 |
| Hq | pg/ml | 1.1E0 | 1.9E0 | 1.2E1 | 1.5E1 | 6.3E1 | 4.7E1 | 1.0E-9 | 1.0E-9 | 9.5E2 | 3.4E2 | 317 | 77 | 136 | 77 | 0.58 |
| Hr | pg/ml | 1.3E2 | 9.0E1 | 8.2E2 | 7.9E2 | 1.5E3 | 2.1E3 | 1.0E-9 | 1.0E-9 | 9.7E3 | 1.4E4 | 317 | 77 | 136 | 77 | 0.47 |
| Hu | pg/ml | 1.4E1 | 4.0E1 | 3.5E3 | 4.8E3 | 3.7E4 | 3.0E4 | 1.0E-9 | 1.0E-9 | 6.3E5 | 2.6E5 | 317 | 77 | 136 | 77 | 0.57 |
| Hv | pg/ml | 1.3E0 | 1.8E0 | 3.6E0 | 1.5E1 | 1.5E1 | 1.0E2 | 1.0E-9 | 1.0E-9 | 2.5E2 | 8.9E2 | 317 | 77 | 136 | 77 | 0.54 |
| Hw | pg/ml | 6.8E0 | 7.3E0 | 2.7E1 | 1.4E2 | 1.1E2 | 1.1E3 | 1.0E-9 | 1.0E-9 | 1.7E3 | 9.4E3 | 317 | 77 | 136 | 77 | 0.49 |
| Hx | pg/ml | 8.3E0 | 1.4E1 | 6.7E1 | 5.5E1 | 5.3E2 | 1.8E2 | 1.0E-9 | 1.0E-9 | 9.3E3 | 1.3E3 | 317 | 77 | 136 | 77 | 0.55 |
| Ib | ng/ml | 6.6E-2 | 7.0E-2 | 7.9E-1 | 3.5E0 | 3.4E0 | 1.2E1 | 1.0E-9 | 1.0E-9 | 3.0E1 | 5.6E1 | 109 | 46 | 85 | 46 | 0.53 |
| Ic | U/ml | 2.2E2 | 2.2E2 | 3.3E2 | 2.3E3 | 1.5E3 | 1.0E4 | 2.9E0 | 6.6E0 | 1.5E4 | 6.5E4 | 109 | 46 | 85 | 46 | 0.66 |
| Id | U/ml | 6.2E-1 | 7.6E-1 | 1.1E0 | 1.1E1 | 1.3E0 | 6.4E1 | 1.0E-9 | 1.5E-1 | 7.2E0 | 4.3E2 | 109 | 46 | 85 | 46 | 0.57 |
| Tt | pg/ml | 1.6E2 | 2.0E2 | 1.7E2 | 2.0E2 | 4.3E1 | 6.4E1 | 8.0E1 | 7.6E1 | 3.0E2 | 4.4E2 | 101 | 40 | 78 | 40 | 0.69 |
| To | pg/ml | 1.6E0 | 1.8E0 | 1.7E0 | 2.3E0 | 1.8E0 | 2.7E0 | 1.0E-9 | 1.0E-9 | 7.1E0 | 1.2E1 | 108 | 43 | 81 | 43 | 0.55 |
| Tr | pg/ml | 2.8E0 | 5.1E0 | 4.0E0 | 8.6E0 | 4.2E0 | 1.4E1 | 3.5E-2 | 9.5E-2 | 2.4E1 | 7.6E1 | 106 | 41 | 80 | 41 | 0.63 |
| Tn | pg/ml | 2.5E1 | 4.6E1 | 4.8E1 | 1.8E2 | 7.0E1 | 4.6E2 | 2.6E0 | 1.1E1 | 3.7E2 | 2.3E3 | 108 | 43 | 81 | 43 | 0.71 |
| Tv | ng/ml | 1.1E1 | 1.5E1 | 2.0E1 | 1.8E2 | 5.0E1 | 1.1E3 | 1.0E-9 | 1.0E-9 | 4.9E2 | 7.1E3 | 108 | 43 | 81 | 43 | 0.57 |
| Ih | ng/ml | 6.8E1 | 7.7E1 | 1.9E2 | 3.2E2 | 3.2E2 | 5.3E2 | 1.0E-9 | 1.0E-9 | 2.5E3 | 2.8E3 | 317 | 79 | 136 | 79 | 0.55 |
| Ii | ng/ml | 8.1E1 | 1.1E2 | 2.9E2 | 2.8E2 | 8.2E2 | 6.5E2 | 7.5E-1 | 1.3E0 | 8.4E3 | 4.5E3 | 317 | 79 | 136 | 79 | 0.53 |
| Ij | ng/ml | 7.2E1 | 8.2E1 | 2.1E2 | 4.7E2 | 7.5E2 | 2.8E3 | 2.1E0 | 9.3E0 | 6.4E3 | 2.4E4 | 317 | 76 | 136 | 76 | 0.56 |
| Ik | ng/ml | 1.1E1 | 6.8E1 | 1.0E3 | 2.0E3 | 9.8E3 | 1.4E4 | 5.9E-1 | 1.7E0 | 1.2E5 | 1.2E5 | 316 | 78 | 136 | 78 | 0.61 |
| Il | ng/ml | 3.8E2 | 4.1E2 | 1.5E3 | 1.6E3 | 3.1E3 | 3.2E3 | 1.0E-9 | 1.0E-9 | 1.2E4 | 1.3E4 | 314 | 76 | 136 | 76 | 0.50 |
| Im | ng/ml | 1.9E2 | 2.7E2 | 3.1E2 | 5.2E2 | 3.5E2 | 8.2E2 | 1.3E1 | 2.0E1 | 3.1E3 | 6.2E3 | 316 | 77 | 136 | 77 | 0.61 |
| In | ng/ml | 4.2E0 | 3.3E0 | 3.5E1 | 6.4E1 | 2.4E2 | 5.0E2 | 1.0E-9 | 1.0E-9 | 3.9E3 | 4.5E3 | 317 | 79 | 136 | 79 | 0.44 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Hb | ng/ml | 2.0E1 | 3.0E1 | 2.6E1 | 4.0E1 | 2.4E1 | 4.0E1 | 1.1E0 | 6.2E-1 | 1.3E2 | 2.0E2 | 110 | 48 | 86 | 48 | 0.65 |
| Hc | pg/ml | 6.9E2 | 6.0E2 | 2.0E3 | 5.2E3 | 4.7E3 | 1.6E4 | 1.0E-9 | 1.0E-9 | 3.6E4 | 1.0E5 | 110 | 48 | 86 | 48 | 0.53 |
| Hf | ng/ml | 1.4E2 | 1.2E2 | 4.0E2 | 2.3E2 | 5.5E2 | 2.6E2 | 1.0E-9 | 1.0E-9 | 2.5E3 | 1.0E3 | 110 | 48 | 86 | 48 | 0.47 |
| Io | ng/ml | 8.4E3 | 1.1E4 | 1.8E4 | 1.9E4 | 5.5E4 | 2.6E4 | 6.6E1 | 6.6E1 | 8.8E5 | 1.1E5 | 315 | 77 | 135 | 77 | 0.54 |
| Ip | ng/ml | 9.7E0 | 2.0E1 | 1.9E1 | 2.7E1 | 2.6E1 | 3.1E1 | 1.0E-9 | 4.9E-3 | 2.6E2 | 1.6E2 | 315 | 77 | 135 | 77 | 0.54 |
| Iq | ug/ml | 1.1E-1 | 1.5E-1 | 4.4E1 | 4.3E0 | 7.7E2 | 2.5E1 | 1.0E-9 | 1.0E-9 | 1.4E4 | 2.2E2 | 315 | 77 | 135 | 77 | 0.56 |
| Ir | ug/ml | 3.2E-1 | 6.5E-1 | 3.2E0 | 9.4E0 | 2.1E1 | 4.6E1 | 1.0E-9 | 1.0E-9 | 2.4E2 | 3.7E2 | 314 | 77 | 135 | 77 | 0.62 |
| Is | ng/ml | 1.4E0 | 3.8E0 | 5.6E0 | 1.4E1 | 1.2E1 | 3.5E1 | 1.0E-9 | 2.2E-3 | 8.8E1 | 2.6E2 | 315 | 77 | 135 | 77 | 0.61 |
| It | ng/ml | 2.0E0 | 2.5E0 | 2.2E1 | 1.6E1 | 1.1E2 | 7.8E1 | 1.0E-9 | 1.0E-9 | 1.2E3 | 6.8E2 | 315 | 77 | 135 | 77 | 0.56 |
| Iu | ng/ml | 2.1E2 | 2.7E2 | 1.5E3 | 1.7E3 | 4.8E3 | 4.1E3 | 1.0E-9 | 1.0E-9 | 2.9E4 | 2.4E4 | 315 | 77 | 135 | 77 | 0.55 |
| Iv | ng/ml | 1.3E1 | 2.3E1 | 4.7E1 | 1.4E2 | 1.3E2 | 5.9E2 | 1.0E-9 | 1.0E-9 | 1.6E3 | 3.8E3 | 315 | 77 | 135 | 77 | 0.61 |
| Iz | ng/ml | 1.3E2 | 1.9E2 | 4.0E2 | 1.6E3 | 9.6E2 | 8.9E3 | 1.5E0 | 4.9E0 | 8.4E3 | 6.2E4 | 110 | 48 | 86 | 48 | 0.58 |
| Yg | pg/ml | 2.5E2 | 3.2E2 | 1.6E3 | 8.1E2 | 7.4E3 | 1.2E3 | 1.0E-9 | 1.0E-9 | 5.0E4 | 5.0E3 | 45 | 19 | 35 | 19 | 0.57 |
| Yh | pg/ml | 2.1E2 | 3.0E2 | 5.1E2 | 5.0E2 | 1.2E3 | 6.1E2 | 1.0E-9 | 1.0E-9 | 7.8E3 | 2.3E3 | 45 | 19 | 35 | 19 | 0.56 |
| Yi | pg/ml | 2.2E2 | 4.3E2 | 5.6E2 | 2.1E3 | 1.2E3 | 5.9E3 | 1.0E-9 | 1.0E-9 | 7.6E3 | 2.6E4 | 45 | 19 | 35 | 19 | 0.64 |
| Yk | U/ml | 1.0E-9 | 1.0E-9 | 1.8E-1 | 3.4E-1 | 5.3E-1 | 1.3E0 | 1.0E-9 | 1.0E-9 | 3.0E0 | 5.6E0 | 45 | 19 | 35 | 19 | 0.48 |
| Yj | pg/ml | 1.7E2 | 1.6E2 | 3.9E2 | 6.3E2 | 5.6E2 | 1.6E3 | 1.0E-9 | 3.9E1 | 3.2E3 | 7.0E3 | 45 | 19 | 35 | 19 | 0.49 |
| Yd | ng/ml | 2.0E-1 | 2.8E-1 | 3.1E-1 | 4.4E-1 | 4.0E-1 | 5.7E-1 | 6.6E-3 | 1.7E-2 | 1.8E0 | 2.3E0 | 47 | 20 | 37 | 20 | 0.59 |
| Wb | pg/ml | 3.0E4 | 3.3E4 | 3.4E4 | 4.2E4 | 1.8E4 | 3.0E4 | 2.2E3 | 1.0E4 | 8.5E4 | 1.5E5 | 47 | 20 | 37 | 20 | 0.58 |
| Vz | pg/ml | 3.2E0 | 2.8E0 | 4.9E0 | 4.5E0 | 5.8E0 | 4.6E0 | 1.0E-9 | 8.3E-1 | 2.9E1 | 2.2E1 | 47 | 20 | 37 | 20 | 0.52 |
| Si | ng/ml | 8.0E-1 | 1.1E0 | 1.5E0 | 2.2E0 | 2.2E0 | 2.4E0 | 1.9E-2 | 3.6E-1 | 1.0E1 | 1.0E1 | 45 | 20 | 35 | 20 | 0.65 |
| Sf | mIU/mL | 1.2E1 | 2.0E1 | 5.4E1 | 3.4E1 | 1.2E2 | 4.2E1 | 8.1E-2 | 2.5E0 | 7.2E2 | 1.7E2 | 45 | 20 | 35 | 20 | 0.56 |
| Sh | mIU/mL | 1.3E1 | 1.4E1 | 5.2E1 | 5.4E1 | 1.2E2 | 1.0E2 | 2.9E-2 | 7.8E-2 | 5.9E2 | 3.7E2 | 45 | 20 | 35 | 20 | 0.55 |
| Sj | ng/ml | 4.4E-1 | 4.1E-1 | 4.2E-1 | 4.3E-1 | 8.5E-2 | 1.0E-1 | 2.5E-1 | 2.5E-1 | 6.1E-1 | 7.0E-1 | 45 | 20 | 35 | 20 | 0.53 |
| Rc | pg/ml | 5.2E3 | 7.6E3 | 6.3E3 | 8.1E3 | 4.4E3 | 5.6E3 | 1.9E2 | 2.4E2 | 2.2E4 | 2.7E4 | 111 | 46 | 84 | 46 | 0.60 |
| Rb | pg/ml | 7.6E-1 | 6.3E-1 | 2.5E0 | 3.3E0 | 3.6E0 | 8.8E0 | 1.0E-9 | 1.0E-9 | 1.6E1 | 5.6E1 | 111 | 46 | 84 | 46 | 0.48 |
| Zq | 2.6ng/ml | 1.5E2 | 4.4E2 | 2.0E2 | 4.8E2 | 1.5E2 | 3.0E2 | 8.3E0 | 4.8E1 | 5.2E2 | 9.7E2 | 44 | 20 | 34 | 20 | 0.79 |
| Zw | 2.5ng/ml | 3.6E0 | 1.0E1 | 1.0E1 | 1.7E1 | 1.5E1 | 2.0E1 | 6.3E-2 | 2.4E-1 | 5.9E1 | 6.3E1 | 47 | 20 | 37 | 20 | 0.60 |
| Zx | 2.3mU/ml | 1.0E-1 | 1.3E-1 | 3.0E-1 | 2.4E-1 | 5.9E-1 | 4.1E-1 | 4.1E-2 | 3.2E-2 | 3.0E0 | 1.9E0 | 47 | 20 | 37 | 20 | 0.53 |
| Pz | ng/ml | 3.3E3 | 5.9E3 | 7.5E3 | 6.4E3 | 1.9E4 | 5.7E3 | 1.3E1 | 4.7E1 | 2.8E5 | 3.9E4 | 314 | 76 | 134 | 76 | 0.56 |
| Qa | ng/ml | 2.8E3 | 6.3E3 | 6.2E3 | 1.1E4 | 8.3E3 | 2.6E4 | 1.5E2 | 2.3E2 | 5.2E4 | 2.2E5 | 314 | 76 | 134 | 76 | 0.65 |
| Qb | ng/ml | 1.0E2 | 1.2E2 | 2.6E2 | 2.5E2 | 6.8E2 | 4.9E2 | 7.9E-1 | 4.4E0 | 8.3E3 | 4.1E3 | 314 | 76 | 134 | 76 | 0.55 |
| Qc | ng/ml | 2.5E2 | 2.8E2 | 5.2E2 | 4.8E2 | 1.0E3 | 5.4E2 | 8.1E-1 | 3.0E0 | 1.1E4 | 2.8E3 | 314 | 76 | 134 | 76 | 0.53 |
| Qd | ng/ml | 9.5E3 | 1.2E4 | 2.8E4 | 2.8E4 | 1.3E5 | 4.3E4 | 2.4E2 | 6.9E2 | 2.0E6 | 2.3E5 | 314 | 76 | 134 | 76 | 0.58 |
| Qe | ng/ml | 7.8E2 | 1.7E3 | 2.0E3 | 2.4E3 | 5.9E3 | 2.8E3 | 7.6E0 | 4.7E1 | 9.7E4 | 1.8E4 | 314 | 76 | 134 | 76 | 0.63 |
| Jd | ng/ml | 3.6E-1 | 1.8E0 | 7.1E0 | 5.9E0 | 6.2E1 | 1.4E1 | 1.0E-9 | 1.0E-9 | 6.5E2 | 9.1E1 | 111 | 47 | 86 | 47 | 0.74 |
| Je | ng/ml | 1.0E-9 | 8.0E-1 | 1.4E0 | 3.3E0 | 5.6E0 | 6.9E0 | 1.0E-9 | 1.0E-9 | 5.6E1 | 4.5E1 | 111 | 47 | 86 | 47 | 0.64 |
| Jf | ng/ml | 1.0E-9 | 1.0E-9 | 8.1E-1 | 1.4E0 | 2.1E0 | 2.6E0 | 1.0E-9 | 1.0E-9 | 1.2E1 | 1.0E1 | 111 | 47 | 86 | 47 | 0.54 |
| Jg | ng/ml | 3.7E2 | 9.7E2 | 7.2E2 | 1.2E3 | 1.1E3 | 1.2E3 | 5.8E0 | 1.3E1 | 1.0E4 | 7.1E3 | 317 | 77 | 136 | 77 | 0.67 |
| Jh | ng/ml | 2.3E0 | 5.7E0 | 2.5E1 | 5.1E1 | 1.0E2 | 1.5E2 | 1.0E-9 | 1.0E-9 | 1.2E3 | 1.1E3 | 317 | 77 | 136 | 77 | 0.61 |
| Ji | ng/ml | 4.6E1 | 7.1E1 | 6.7E1 | 1.3E2 | 6.9E1 | 1.9E2 | 1.1E0 | 5.2E0 | 5.3E2 | 1.3E3 | 317 | 77 | 136 | 77 | 0.64 |
| Sr | pg/mL | 3.0E2 | 6.1E2 | 7.6E2 | 1.5E3 | 1.3E3 | 3.2E3 | 1.0E-9 | 1.0E-9 | 9.8E3 | 2.1E4 | 106 | 46 | 82 | 46 | 0.63 |
| Ss | pg/mL | 7.6E4 | 1.9E5 | 1.3E5 | 1.9E5 | 1.3E5 | 1.7E5 | 9.5E3 | 1.1E4 | 6.7E5 | 8.5E5 | 106 | 46 | 82 | 46 | 0.61 |
| St | pg/mL | 2.2E7 | 5.4E7 | 4.7E7 | 1.0E8 | 6.0E7 | 2.5E8 | 7.8E5 | 1.0E-9 | 4.1E8 | 1.7E9 | 108 | 45 | 82 | 45 | 0.62 |
| Wc | ng/ml | 1.0E-9 | 1.0E-9 | 5.2E-2 | 1.2E-1 | 1.5E-1 | 4.0E-1 | 1.0E-9 | 1.0E-9 | 9.8E-1 | 1.8E0 | 47 | 20 | 36 | 20 | 0.53 |
| Wd | ng/ml | 8.9E0 | 1.2E1 | 1.8E1 | 9.0E1 | 4.3E1 | 2.7E2 | 1.0E-9 | 1.5E0 | 2.9E2 | 1.2E3 | 47 | 20 | 36 | 20 | 0.61 |
| We | ng/ml | 3.5E-1 | 7.1E-1 | 6.9E-1 | 4.1E0 | 9.7E-1 | 7.9E0 | 1.0E-9 | 6.6E-2 | 3.9E0 | 2.3E1 | 47 | 20 | 36 | 20 | 0.65 |
| Wg | ng/ml | 1.0E-9 | 1.0E-9 | 1.0E-9 | 2.7E-2 | 0.0E0 | 1.2E-1 | 1.0E-9 | 1.0E-9 | 1.0E-9 | 5.3E-1 | 47 | 20 | 36 | 20 | 0.53 |
| Wh | ng/ml | 8.7E-3 | 1.8E-2 | 1.9E-2 | 2.9E-1 | 5.1E-2 | 1.0E0 | 1.0E-9 | 1.0E-9 | 3.5E-1 | 4.5E0 | 47 | 20 | 36 | 20 | 0.70 |
| Wf | ng/ml | 1.0E-9 | 1.0E-9 | 1.4E-1 | 2.1E-1 | 2.6E-1 | 5.2E-1 | 1.0E-9 | 1.0E-9 | 1.1E0 | 2.3E0 | 47 | 20 | 36 | 20 | 0.50 |
| Ra | pg/ml | 1.0E-9 | 1.0E-9 | 7.5E-1 | 1.8E0 | 1.4E0 | 9.5E0 | 1.0E-9 | 1.0E-9 | 7.3E0 | 6.4E1 | 111 | 46 | 84 | 46 | 0.45 |
| Qz | pg/ml | 1.1E1 | 1.1E1 | 6.9E1 | 3.1E1 | 1.1E2 | 4.9E1 | 1.0E-9 | 1.0E-9 | 7.1E2 | 2.2E2 | 111 | 46 | 84 | 46 | 0.45 |
| Qy | pg/ml | 4.1E-1 | 5.4E-1 | 2.9E0 | 2.5E1 | 1.5E1 | 8.5E1 | 1.0E-9 | 1.0E-9 | 1.6E2 | 4.3E2 | 111 | 46 | 84 | 46 | 0.57 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qx | pg/ml | 1.0E-9 | 1.0E-9 | 6.4E0 | 4.5E0 | 5.1E1 | 1.7E1 | 1.0E-9 | 1.0E-9 | 5.4E2 | 1.1E2 | 111 | 46 | 84 | 46 | 0.51 |
| Qw | pg/ml | 1.8E-1 | 1.0E-9 | 1.4E0 | 2.3E0 | 3.7E0 | 8.7E0 | 1.0E-9 | 1.0E-9 | 3.0E1 | 5.6E1 | 111 | 46 | 84 | 46 | 0.45 |
| Qv | pg/ml | 2.3E4 | 1.0E4 | 3.7E4 | 2.2E4 | 7.7E4 | 2.4E4 | 1.2E3 | 4.0E2 | 7.4E5 | 1.2E5 | 111 | 46 | 84 | 46 | 0.37 |
| Qu | pg/ml | 5.1E0 | 2.8E1 | 7.9E1 | 9.3E1 | 1.7E2 | 1.6E2 | 1.0E-9 | 1.0E-9 | 8.0E2 | 7.0E2 | 111 | 46 | 84 | 46 | 0.58 |
| Qt | pg/ml | 1.2E1 | 1.9E1 | 3.0E1 | 7.1E1 | 5.9E1 | 1.6E2 | 1.0E-9 | 1.0E-9 | 3.8E2 | 1.0E3 | 111 | 46 | 84 | 46 | 0.59 |
| Qh | ng/ml | 1.5E1 | 2.3E1 | 3.7E1 | 4.2E1 | 7.2E1 | 5.7E1 | 1.2E-1 | 7.8E-1 | 6.4E2 | 3.4E2 | 111 | 46 | 84 | 46 | 0.59 |
| Qg | ng/ml | 8.9E0 | 7.1E0 | 2.6E1 | 1.5E1 | 1.0E2 | 3.2E1 | 1.5E-1 | 3.3E-1 | 1.0E3 | 2.0E2 | 111 | 46 | 84 | 46 | 0.42 |
| Jj | ng/ml | 8.4E2 | 6.0E2 | 2.9E3 | 1.2E3 | 2.0E4 | 2.0E3 | 2.0E1 | 1.2E1 | 3.4E5 | 1.2E4 | 317 | 77 | 136 | 77 | 0.41 |
| Jk | ng/ml | 3.3E0 | 4.7E0 | 2.4E1 | 3.1E1 | 5.0E1 | 6.1E1 | 1.0E-9 | 1.1E-1 | 2.8E2 | 3.9E2 | 317 | 77 | 136 | 77 | 0.55 |
| Jl | ng/ml | 3.7E-1 | 8.5E-1 | 1.9E0 | 1.3E2 | 4.7E0 | 1.1E3 | 7.6E-4 | 1.9E-3 | 3.2E1 | 9.9E3 | 317 | 77 | 136 | 77 | 0.61 |
| Jm | ng/ml | 1.9E1 | 2.7E1 | 5.1E1 | 6.6E1 | 9.4E1 | 1.1E2 | 1.0E-9 | 3.0E-1 | 1.0E3 | 6.1E2 | 317 | 77 | 136 | 77 | 0.53 |
| Jn | pg/ml | 3.8E-1 | 4.6E-1 | 1.9E0 | 1.9E1 | 7.0E0 | 1.1E2 | 1.0E-9 | 1.0E-9 | 6.2E1 | 7.3E2 | 317 | 77 | 136 | 77 | 0.56 |
| Jo | pg/ml | 4.0E3 | 4.3E3 | 5.0E3 | 7.0E3 | 3.7E3 | 1.2E4 | 4.2E1 | 2.3E2 | 2.0E4 | 1.0E5 | 317 | 77 | 136 | 77 | 0.53 |
| Jp | pg/ml | 6.6E4 | 8.2E4 | 7.0E4 | 8.3E4 | 3.4E4 | 3.4E4 | 2.1E3 | 3.1E3 | 2.1E5 | 2.1E5 | 317 | 77 | 136 | 77 | 0.63 |
| Jq | pg/ml | 9.2E1 | 1.2E2 | 1.4E2 | 3.5E2 | 1.6E2 | 1.1E3 | 2.6E0 | 1.1E1 | 1.1E3 | 8.7E3 | 317 | 77 | 136 | 77 | 0.58 |
| Jr | pg/ml | 3.8E0 | 8.3E0 | 1.8E1 | 1.8E2 | 6.6E1 | 1.0E3 | 1.0E-9 | 1.0E-9 | 7.9E2 | 7.4E3 | 317 | 77 | 136 | 77 | 0.59 |
| Js | pg/ml | 1.3E1 | 1.4E1 | 4.5E1 | 1.1E2 | 1.5E2 | 4.8E2 | 1.0E-9 | 4.5E-1 | 1.6E3 | 3.0E3 | 317 | 77 | 136 | 77 | 0.53 |
| Jt | pg/ml | 2.4E3 | 3.1E3 | 3.0E3 | 4.5E3 | 2.1E3 | 6.4E3 | 1.5E2 | 2.3E2 | 1.2E4 | 5.2E4 | 317 | 77 | 136 | 77 | 0.59 |
| Xa | pg/ml | 1.0E-9 | 8.7E0 | 9.8E0 | 7.7E1 | 2.1E1 | 2.7E2 | 1.0E-9 | 1.0E-9 | 9.6E1 | 1.2E3 | 47 | 20 | 37 | 20 | 0.65 |
| Ye | pg/ml | 1.0E-9 | 1.0E-9 | 3.0E0 | 4.8E0 | 1.0E1 | 9.1E0 | 1.0E-9 | 1.0E-9 | 6.9E1 | 2.7E1 | 47 | 20 | 37 | 20 | 0.53 |
| Tm | pg/ml | 1.0E-9 | 1.0E-9 | 3.2E0 | 2.9E0 | 5.4E0 | 6.1E0 | 1.0E-9 | 1.0E-9 | 1.8E1 | 2.5E1 | 47 | 20 | 37 | 20 | 0.48 |
| Tl | pg/ml | 1.1E-1 | 1.3E-1 | 2.7E-1 | 1.5E0 | 3.8E-1 | 5.5E0 | 1.0E-9 | 1.0E-9 | 1.8E0 | 2.5E1 | 47 | 20 | 37 | 20 | 0.53 |
| Ju | mIU/ml | 7.2E0 | 1.1E1 | 2.2E1 | 1.7E1 | 3.6E1 | 1.9E1 | 6.5E-2 | 1.9E-1 | 2.3E2 | 9.3E1 | 111 | 47 | 86 | 47 | 0.56 |
| Jv | mIU/ml | 1.1E1 | 1.5E1 | 3.9E1 | 3.2E1 | 7.4E1 | 4.6E1 | 1.0E-2 | 5.0E-2 | 4.4E2 | 2.2E2 | 111 | 47 | 86 | 47 | 0.53 |
| Jy | ng/ml | 1.5E-3 | 1.8E-3 | 2.3E-3 | 3.5E-3 | 4.9E-3 | 6.5E-3 | 1.7E-4 | 4.5E-4 | 5.2E-2 | 4.1E-2 | 111 | 47 | 86 | 47 | 0.57 |
| Kc | pg/ml | 2.1E1 | 3.4E1 | 3.5E1 | 5.0E1 | 4.0E1 | 4.5E1 | 1.0E-9 | 1.0E-9 | 1.7E2 | 1.6E2 | 111 | 48 | 86 | 48 | 0.64 |
| Kd | pg/ml | 1.0E-9 | 1.0E-9 | 2.4E2 | 9.6E2 | 7.3E2 | 5.6E3 | 1.0E-9 | 1.0E-9 | 4.8E3 | 3.8E4 | 111 | 48 | 86 | 48 | 0.49 |
| Ke | pg/ml | 9.6E3 | 1.4E4 | 1.3E4 | 2.6E4 | 9.5E3 | 4.8E4 | 3.4E2 | 2.0E3 | 5.9E4 | 3.2E5 | 111 | 48 | 86 | 48 | 0.62 |
| Kf | pg/mL | 5.9E0 | 8.6E0 | 6.5E0 | 1.1E1 | 5.7E0 | 1.1E1 | 1.0E-9 | 1.0E-9 | 2.6E1 | 7.8E1 | 111 | 48 | 86 | 48 | 0.65 |
| Kg | pg/mL | 9.9E2 | 1.3E3 | 1.5E3 | 2.7E3 | 1.5E3 | 4.5E3 | 7.3E1 | 1.3E2 | 8.4E3 | 2.7E4 | 111 | 48 | 86 | 48 | 0.58 |
| Ki | pg/ml | 6.4E1 | 4.7E1 | 7.2E1 | 5.7E1 | 5.2E1 | 4.1E1 | 1.0E-9 | 1.0E-9 | 2.9E2 | 2.0E2 | 111 | 48 | 86 | 48 | 0.39 |
| Kj | pg/ml | 9.3E2 | 1.3E3 | 1.5E3 | 2.1E3 | 1.7E3 | 2.6E3 | 1.4E1 | 3.3E1 | 1.0E4 | 1.5E4 | 111 | 48 | 86 | 48 | 0.57 |
| Kk | pg/ml | 6.9E0 | 6.8E0 | 1.1E1 | 1.3E1 | 1.8E1 | 1.5E1 | 1.0E-9 | 1.0E-9 | 1.6E2 | 6.1E1 | 111 | 48 | 86 | 48 | 0.51 |
| Kl | pg/ml | 1.8E4 | 2.9E4 | 2.5E4 | 3.4E4 | 2.2E4 | 2.7E4 | 1.6E2 | 1.6E3 | 1.1E5 | 1.3E5 | 111 | 48 | 86 | 48 | 0.61 |
| Kn | pg/ml | 1.5E1 | 3.0E1 | 5.8E1 | 1.7E2 | 1.1E2 | 7.0E2 | 1.0E-9 | 1.0E-9 | 7.3E2 | 4.9E3 | 111 | 48 | 86 | 48 | 0.60 |
| Ko | pg/ml | 3.0E2 | 5.3E2 | 3.9E2 | 7.6E2 | 4.0E2 | 8.5E2 | 1.0E-9 | 1.0E-9 | 1.8E3 | 4.1E3 | 111 | 48 | 86 | 48 | 0.66 |
| Kp | pg/ml | 3.2E2 | 3.4E2 | 3.4E2 | 6.5E2 | 3.0E2 | 1.9E3 | 1.0E-9 | 3.6E0 | 1.7E3 | 1.3E4 | 111 | 48 | 86 | 48 | 0.57 |
| Kq | pg/ml | 2.8E2 | 4.7E2 | 4.2E2 | 4.2E3 | 9.7E2 | 2.3E4 | 5.1E0 | 8.8E1 | 9.8E3 | 1.6E5 | 106 | 48 | 82 | 48 | 0.69 |
| Kr | pg/ml | 5.0E-1 | 2.9E-1 | 2.5E0 | 1.1E1 | 4.9E0 | 6.0E1 | 1.0E-9 | 1.0E-9 | 3.5E1 | 4.2E2 | 106 | 48 | 82 | 48 | 0.49 |
| Ks | pg/ml | 1.5E4 | 1.7E4 | 2.1E4 | 2.1E4 | 2.0E4 | 1.8E4 | 3.8E2 | 6.8E2 | 1.1E5 | 6.3E4 | 106 | 48 | 82 | 48 | 0.51 |
| Ps | ng/ml | 1.4E2 | 2.9E2 | 4.3E2 | 6.1E2 | 1.2E3 | 8.6E2 | 6.9E0 | 6.6E1 | 8.3E3 | 3.8E3 | 45 | 20 | 34 | 20 | 0.69 |
| Kx | ng/ml | 1.0E-9 | 3.4E-3 | 4.6E-3 | 1.2E-2 | 8.6E-3 | 2.0E-2 | 1.0E-9 | 1.0E-9 | 5.1E-2 | 9.5E-2 | 110 | 48 | 86 | 48 | 0.62 |
| Ky | ng/ml | 1.0E-1 | 2.0E-1 | 3.5E-1 | 5.3E-1 | 7.1E-1 | 7.7E-1 | 1.0E-9 | 1.0E-9 | 5.1E0 | 3.7E0 | 110 | 48 | 86 | 48 | 0.62 |
| Kz | ng/ml | 1.0E-9 | 1.7E-3 | 3.7E-3 | 5.1E-3 | 6.3E-3 | 6.1E-3 | 1.0E-9 | 1.0E-9 | 3.6E-2 | 1.8E-2 | 110 | 48 | 86 | 48 | 0.58 |
| Rz | ng/ml | 1.4E-1 | 3.9E-1 | 6.0E-1 | 1.3E0 | 1.0E0 | 2.0E0 | 3.6E-3 | 1.7E-2 | 4.4E0 | 6.5E0 | 45 | 20 | 35 | 20 | 0.66 |
| Ry | ng/ml | 1.6E-2 | 2.0E-2 | 1.8E-2 | 3.8E-2 | 2.0E-2 | 7.6E-2 | 1.0E-9 | 1.0E-9 | 1.0E-1 | 3.5E-1 | 45 | 20 | 35 | 20 | 0.58 |
| Rx | ng/ml | 1.0E-9 | 1.8E-5 | 2.6E-3 | 1.2E-3 | 4.4E-3 | 1.9E-3 | 1.0E-9 | 1.0E-9 | 2.0E-2 | 6.1E-3 | 45 | 20 | 35 | 20 | 0.47 |
| Ld | pg/ml | 1.0E-9 | 1.0E-9 | 4.0E0 | 3.7E0 | 9.9E0 | 5.9E0 | 1.0E-9 | 1.0E-9 | 6.9E1 | 2.3E1 | 108 | 49 | 85 | 49 | 0.54 |
| Lh | pg/ml | 1.1E4 | 2.2E4 | 2.0E4 | 3.7E4 | 2.9E4 | 6.4E4 | 1.0E-9 | 1.1E2 | 2.6E5 | 4.1E5 | 315 | 77 | 136 | 77 | 0.65 |
| Li | pg/ml | 2.6E3 | 5.7E3 | 8.5E3 | 2.6E4 | 2.3E4 | 5.3E4 | 1.0E-9 | 3.7E1 | 2.9E5 | 3.1E5 | 315 | 77 | 136 | 77 | 0.65 |
| Lj | pg/ml | 1.8E3 | 4.8E3 | 1.1E4 | 2.7E4 | 4.2E4 | 6.7E4 | 1.4E1 | 3.4E1 | 4.4E5 | 3.9E5 | 315 | 77 | 136 | 77 | 0.62 |
| Lp | pg/ml | 9.4E0 | 1.4E1 | 3.1E1 | 1.3E2 | 5.7E1 | 2.6E2 | 1.0E-9 | 1.0E-9 | 2.4E2 | 1.0E3 | 45 | 20 | 35 | 20 | 0.54 |
| Lt | pg/ml | 1.0E-9 | 1.0E-9 | 2.6E0 | 1.0E-9 | 1.0E1 | 0.0E0 | 1.0E-9 | 1.0E-9 | 6.0E1 | 1.0E-9 | 45 | 20 | 35 | 20 | 0.46 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Rv | ng/ml | 5.0E-4 | 8.4E-4 | 8.5E-4 | 2.8E-3 | 1.1E-3 | 4.6E-3 | 1.0E-9 | 1.0E-9 | 5.9E-3 | 1.6E-2 | 45 | 20 | 34 | 20 | 0.60 |
| Ru | ng/ml | 1.0E-9 | 1.0E-9 | 9.6E-3 | 3.9E-2 | 4.9E-2 | 1.1E-1 | 1.0E-9 | 1.0E-9 | 3.1E-1 | 3.6E-1 | 45 | 20 | 34 | 20 | 0.55 |
| Rt | ng/ml | 6.8E-2 | 1.0E-1 | 9.0E-2 | 5.1E-1 | 1.1E-1 | 1.6E0 | 1.6E-3 | 1.3E-3 | 4.5E-1 | 7.4E0 | 45 | 20 | 34 | 20 | 0.62 |
| Yl | pg/ml | 1.1E1 | 1.2E1 | 1.8E1 | 3.1E1 | 1.9E1 | 4.8E1 | 1.0E-9 | 1.0E-9 | 1.1E2 | 2.2E2 | 47 | 20 | 37 | 20 | 0.58 |
| Rm | ng/ml | 1.7E1 | 2.1E1 | 5.8E1 | 5.6E1 | 8.8E1 | 6.8E1 | 2.2E-1 | 3.9E-1 | 4.0E2 | 3.2E2 | 110 | 45 | 83 | 45 | 0.55 |
| Rh | ng/ml | 1.4E2 | 1.7E2 | 2.9E2 | 6.7E2 | 5.7E2 | 2.6E3 | 4.7E0 | 7.6E0 | 3.8E3 | 1.7E4 | 110 | 45 | 83 | 45 | 0.55 |
| Ri | ng/ml | 1.0E-9 | 1.0E-9 | 3.9E0 | 1.7E0 | 9.2E0 | 4.4E0 | 1.0E-9 | 1.0E-9 | 7.4E1 | 2.5E1 | 111 | 45 | 84 | 45 | 0.42 |
| Rg | ng/ml | 1.0E-9 | 1.0E-9 | 2.7E-2 | 1.1E-1 | 2.6E-1 | 4.6E-1 | 1.0E-9 | 1.0E-9 | 2.7E0 | 3.0E0 | 110 | 45 | 83 | 45 | 0.54 |
| Rj | ng/ml | 1.0E-9 | 1.0E-9 | 1.7E0 | 6.7E0 | 7.9E0 | 4.0E1 | 1.0E-9 | 1.0E-9 | 8.0E1 | 2.7E2 | 111 | 45 | 84 | 45 | 0.50 |
| Rf | ng/ml | 3.6E-1 | 5.0E-1 | 8.3E-1 | 1.3E0 | 1.6E0 | 3.1E0 | 7.8E-3 | 3.6E-2 | 1.4E1 | 1.7E1 | 110 | 45 | 83 | 45 | 0.56 |
| Ql | pg/ml | 7.3E0 | 7.3E0 | 1.4E1 | 1.8E1 | 3.1E1 | 3.4E1 | 1.0E-9 | 1.0E-9 | 2.9E2 | 1.8E2 | 111 | 47 | 86 | 47 | 0.48 |
| Qm | pg/ml | 1.7E0 | 1.7E1 | 1.9E1 | 2.8E1 | 4.4E1 | 3.3E1 | 1.0E-9 | 1.0E-9 | 3.6E2 | 1.3E2 | 111 | 47 | 86 | 47 | 0.63 |
| Qn | pg/ml | 6.1E-1 | 1.0E0 | 7.5E0 | 5.6E0 | 2.6E1 | 1.1E1 | 1.0E-9 | 1.0E-9 | 2.2E2 | 6.0E1 | 111 | 47 | 86 | 47 | 0.54 |
| Nv | pg/ml | 3.5E3 | 5.2E3 | 1.3E4 | 1.5E4 | 6.3E4 | 2.5E4 | 1.0E-9 | 3.4E1 | 1.1E6 | 1.6E5 | 318 | 79 | 136 | 79 | 0.61 |
| Nw | pg/ml | 7.4E3 | 1.3E4 | 1.2E4 | 2.0E4 | 2.2E4 | 3.4E4 | 8.6E1 | 5.7E2 | 2.1E5 | 2.2E5 | 318 | 79 | 136 | 79 | 0.65 |
| Nx | pg/ml | 1.7E2 | 2.6E2 | 3.9E2 | 5.5E2 | 7.1E2 | 7.7E2 | 1.0E-9 | 1.0E-9 | 7.4E3 | 4.1E3 | 318 | 79 | 136 | 79 | 0.60 |
| Ny | pg/ml | 4.7E0 | 1.2E1 | 1.1E2 | 8.8E1 | 1.4E3 | 3.5E2 | 1.0E-9 | 1.0E-9 | 2.5E4 | 2.8E3 | 318 | 79 | 136 | 79 | 0.62 |
| Oa | pg/ml | 1.6E2 | 2.5E2 | 4.4E2 | 5.1E2 | 8.0E2 | 6.1E2 | 1.0E-9 | 1.0E-9 | 4.8E3 | 2.4E3 | 111 | 47 | 86 | 47 | 0.58 |
| Op | pg/ml | 3.3E5 | 4.9E5 | 3.7E5 | 4.8E5 | 1.6E5 | 1.5E5 | 3.3E4 | 9.4E4 | 7.3E5 | 7.5E5 | 45 | 20 | 35 | 20 | 0.71 |
| Wn | ng/ml | 1.2E1 | 1.4E1 | 9.7E1 | 2.7E1 | 3.1E2 | 2.9E1 | 2.5E0 | 2.7E0 | 1.8E3 | 1.0E2 | 33 | 11 | 27 | 11 | 0.52 |
| Tk | ng/ml | 2.0E2 | 1.0E2 | 4.3E2 | 3.1E2 | 7.4E2 | 5.1E2 | 2.4E1 | 1.0E1 | 4.2E3 | 1.4E3 | 36 | 13 | 29 | 13 | 0.41 |
| Oe | pg/ml | 9.7E1 | 6.6E1 | 2.7E2 | 3.2E2 | 4.2E2 | 5.5E2 | 1.0E-9 | 1.0E-9 | 2.7E3 | 2.3E3 | 314 | 79 | 136 | 79 | 0.50 |
| Of | pg/ml | 2.1E2 | 4.8E2 | 5.6E3 | 1.2E4 | 1.8E4 | 3.0E4 | 1.0E-9 | 1.0E-9 | 1.8E5 | 1.7E5 | 317 | 79 | 136 | 79 | 0.53 |
| Og | pg/ml | 9.4E-2 | 8.3E-2 | 7.2E-1 | 1.3E0 | 2.2E0 | 8.1E0 | 1.0E-9 | 1.0E-9 | 2.0E1 | 7.2E1 | 317 | 79 | 136 | 79 | 0.43 |
| Oh | pg/ml | 2.0E0 | 4.2E0 | 2.5E1 | 3.0E1 | 1.2E2 | 1.3E2 | 1.0E-9 | 1.0E-9 | 1.4E3 | 1.1E3 | 317 | 79 | 136 | 79 | 0.63 |
| Oi | pg/ml | 2.9E0 | 2.6E0 | 7.2E0 | 6.7E0 | 1.1E1 | 8.6E0 | 1.0E-9 | 1.0E-9 | 8.6E1 | 4.8E1 | 317 | 79 | 136 | 79 | 0.51 |
| Ok | pg/ml | 3.2E2 | 6.0E2 | 4.3E2 | 9.2E2 | 3.9E2 | 1.2E3 | 1.3E1 | 2.9E1 | 3.1E3 | 7.8E3 | 317 | 79 | 136 | 79 | 0.68 |
| Om | pg/ml | 3.6E2 | 5.5E2 | 7.6E2 | 2.0E3 | 2.1E3 | 6.2E3 | 1.0E-9 | 1.0E-9 | 3.0E4 | 5.1E4 | 317 | 79 | 136 | 79 | 0.63 |
| On | pg/ml | 1.4E2 | 2.7E2 | 2.8E2 | 5.7E2 | 4.8E2 | 1.1E3 | 8.4E-1 | 1.7E1 | 4.5E3 | 8.5E3 | 317 | 79 | 136 | 79 | 0.66 |
| Or | pg/ml | 1.0E1 | 2.2E1 | 3.1E1 | 5.7E1 | 6.4E1 | 1.1E2 | 1.0E-9 | 1.0E-9 | 5.0E2 | 5.1E2 | 110 | 49 | 86 | 49 | 0.55 |
| Ow | pg/ml | 2.8E1 | 5.5E1 | 1.0E2 | 2.8E2 | 3.0E2 | 7.9E2 | 1.0E-9 | 1.3E0 | 2.3E3 | 4.7E3 | 110 | 49 | 86 | 49 | 0.67 |
| Ou | pg/ml | 4.4E2 | 7.4E2 | 8.4E2 | 1.7E3 | 1.2E3 | 2.5E3 | 1.0E-9 | 5.4E1 | 9.4E3 | 1.1E4 | 110 | 49 | 86 | 49 | 0.63 |
| Ul | ng/ml | 1.0E-9 | 1.0E-9 | 1.1E0 | 3.3E0 | 3.7E0 | 1.2E1 | 1.0E-9 | 1.0E-9 | 2.2E1 | 5.6E1 | 119 | 46 | 88 | 46 | 0.50 |
| Uo | ng/ml | 1.0E-9 | 1.0E-9 | 1.1E-1 | 7.7E-2 | 2.4E-1 | 1.8E-1 | 1.0E-9 | 1.0E-9 | 1.3E0 | 6.8E-1 | 119 | 46 | 88 | 46 | 0.49 |
| Uk | ng/ml | 1.0E-9 | 1.0E-9 | 4.8E-3 | 8.9E-3 | 1.2E-2 | 2.4E-2 | 1.0E-9 | 1.0E-9 | 9.9E-2 | 1.4E-1 | 119 | 46 | 88 | 46 | 0.49 |
| Um | ng/ml | 1.0E-9 | 1.0E-9 | 3.8E-1 | 4.1E-1 | 9.8E-1 | 7.8E-1 | 1.0E-9 | 1.0E-9 | 6.8E0 | 2.7E0 | 119 | 46 | 88 | 46 | 0.51 |
| Uf | ng/ml | 4.5E-2 | 9.8E-2 | 9.4E-2 | 3.2E-1 | 1.2E-1 | 8.5E-1 | 2.8E-3 | 6.6E-3 | 6.6E-1 | 5.6E0 | 119 | 46 | 88 | 46 | 0.68 |
| Uh | ng/ml | 1.8E0 | 3.2E0 | 2.5E0 | 4.7E0 | 2.3E0 | 4.5E0 | 3.2E-2 | 1.3E-1 | 1.1E1 | 1.8E1 | 119 | 46 | 88 | 46 | 0.67 |
| Un | ng/ml | 1.6E0 | 2.5E0 | 1.9E0 | 3.1E0 | 1.2E0 | 3.6E0 | 2.0E-1 | 3.4E-1 | 7.0E0 | 2.5E1 | 119 | 46 | 88 | 46 | 0.68 |
| Ug | ng/ml | 1.4E1 | 1.3E1 | 2.7E1 | 3.2E1 | 2.7E1 | 3.9E1 | 6.9E-1 | 1.2E0 | 1.3E2 | 1.6E2 | 119 | 46 | 88 | 46 | 0.49 |
| Ur | ng/ml | 1.6E-1 | 7.6E-2 | 1.2E0 | 7.0E-1 | 8.6E0 | 1.6E0 | 1.0E-9 | 1.0E-9 | 9.3E1 | 7.3E0 | 119 | 46 | 88 | 46 | 0.45 |
| Up | ng/ml | 1.0E-9 | 1.0E-9 | 7.5E-3 | 5.3E-2 | 3.4E-2 | 3.5E-1 | 1.0E-9 | 1.0E-9 | 3.3E-1 | 2.4E0 | 119 | 46 | 88 | 46 | 0.49 |
| Us | ng/ml | 4.4E-3 | 1.7E-3 | 2.1E-2 | 5.0E-2 | 5.6E-2 | 2.4E-1 | 1.0E-9 | 1.0E-9 | 4.0E-1 | 1.7E0 | 119 | 46 | 88 | 46 | 0.46 |
| Uv | ng/ml | 3.2E-3 | 2.9E-3 | 1.2E-2 | 1.7E-2 | 3.1E-2 | 6.0E-2 | 1.0E-9 | 1.0E-9 | 2.3E-1 | 4.1E-1 | 119 | 46 | 88 | 46 | 0.50 |
| Ut | ng/ml | 5.3E-1 | 1.3E0 | 2.3E0 | 5.8E0 | 8.9E0 | 1.3E1 | 1.0E-9 | 1.0E-9 | 7.2E1 | 6.5E1 | 119 | 46 | 88 | 46 | 0.65 |
| Uu | ng/ml | 6.9E0 | 8.0E0 | 7.8E0 | 9.1E0 | 5.1E0 | 6.7E0 | 8.1E-1 | 1.2E0 | 2.6E1 | 4.0E1 | 119 | 46 | 88 | 46 | 0.56 |
| Uw | ng/ml | 1.8E0 | 2.4E0 | 2.9E0 | 5.0E0 | 5.2E0 | 8.4E0 | 1.0E-9 | 3.7E-1 | 3.7E1 | 3.9E1 | 53 | 20 | 42 | 20 | 0.63 |
| Vb | ng/ml | 1.0E0 | 1.1E0 | 1.0E0 | 1.3E0 | 4.2E-1 | 1.3E0 | 2.1E-1 | 2.6E-1 | 2.5E0 | 6.4E0 | 53 | 20 | 42 | 20 | 0.50 |
| Vc | ng/ml | 1.0E-9 | 1.0E-9 | 2.3E-3 | 1.0E-9 | 1.5E-2 | 0.0E0 | 1.0E-9 | 1.0E-9 | 1.1E-1 | 1.0E-9 | 53 | 20 | 42 | 20 | 0.47 |
| Uy | ng/ml | 1.0E0 | 9.0E-1 | 2.9E0 | 5.2E0 | 6.6E0 | 1.3E1 | 5.3E-2 | 2.0E-2 | 3.5E1 | 4.6E1 | 53 | 20 | 42 | 20 | 0.50 |
| Uz | ng/ml | 1.0E-9 | 1.0E-9 | 8.3E-3 | 1.7E0 | 6.0E-2 | 7.4E0 | 1.0E-9 | 1.0E-9 | 4.4E-1 | 3.3E1 | 53 | 20 | 42 | 20 | 0.51 |
| Ux | ng/ml | 1.2E2 | 2.0E2 | 1.6E2 | 2.1E2 | 1.3E2 | 1.2E2 | 5.8E0 | 3.2E1 | 4.6E2 | 4.7E2 | 53 | 20 | 42 | 20 | 0.64 |
| Va | ng/ml | 1.6E1 | 2.9E1 | 2.3E1 | 2.9E1 | 2.5E1 | 2.5E1 | 3.1E-1 | 1.2E0 | 1.2E2 | 7.8E1 | 53 | 20 | 42 | 20 | 0.58 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vh | ng/ml | 4.4E-3 | 2.0E-2 | 1.1E-2 | 6.9E-2 | 1.9E-2 | 1.9E-1 | 1.0E-9 | 2.2E-3 | 1.2E-1 | 8.6E-1 | 53 | 20 | 42 | 20 | 0.81 |
| Vi | ng/ml | 2.4E-3 | 6.3E-3 | 2.6E-1 | 1.1E-1 | 1.9E0 | 4.0E-1 | 1.0E-9 | 2.8E-4 | 1.4E1 | 1.8E0 | 53 | 20 | 42 | 20 | 0.67 |
| Vj | ng/ml | 2.2E1 | 5.7E1 | 1.8E2 | 1.1E2 | 7.4E2 | 1.5E2 | 3.2E0 | 8.0E0 | 5.2E3 | 6.5E2 | 53 | 19 | 42 | 19 | 0.68 |
| Vp | ng/ml | 1.0E-9 | 1.0E-9 | 7.4E-1 | 1.1E0 | 5.2E0 | 7.2E0 | 1.0E-9 | 1.0E-9 | 5.0E1 | 4.9E1 | 119 | 46 | 88 | 46 | 0.51 |
| Vt | ng/ml | 6.1E0 | 8.0E0 | 7.6E0 | 1.3E1 | 6.1E0 | 2.3E1 | 6.0E-1 | 4.3E-1 | 3.2E1 | 1.6E2 | 119 | 46 | 88 | 46 | 0.59 |
| Vu | ng/ml | 1.0E-9 | 6.1E-1 | 2.3E0 | 4.5E0 | 6.0E0 | 1.1E1 | 1.0E-9 | 1.0E-9 | 5.1E1 | 7.1E1 | 118 | 44 | 88 | 44 | 0.57 |
| Vq | ng/ml | 1.2E2 | 3.1E2 | 5.5E2 | 4.6E2 | 1.2E3 | 4.5E2 | 2.0E-1 | 3.6E0 | 1.0E4 | 1.4E3 | 94 | 35 | 72 | 35 | 0.59 |
| Vo | ng/ml | 2.6E1 | 2.6E1 | 2.5E1 | 2.5E1 | 4.9E0 | 5.0E0 | 2.5E0 | 1.1E1 | 3.5E1 | 3.5E1 | 119 | 46 | 88 | 46 | 0.49 |
| Vs | ng/ml | 1.0E-9 | 2.7E-1 | 6.5E0 | 1.6E1 | 2.7E1 | 7.1E1 | 1.0E-9 | 1.0E-9 | 2.3E2 | 4.5E2 | 116 | 41 | 86 | 41 | 0.54 |
| Vv | ng/ml | 2.7E0 | 4.6E0 | 6.0E0 | 7.2E0 | 1.0E1 | 7.9E0 | 1.0E-9 | 1.0E-9 | 8.1E1 | 3.2E1 | 118 | 45 | 88 | 45 | 0.59 |
| Vw | ng/ml | 3.3E1 | 4.0E1 | 3.1E1 | 4.0E1 | 1.7E1 | 1.5E1 | 2.5E0 | 1.1E1 | 6.7E1 | 6.6E1 | 53 | 20 | 42 | 20 | 0.64 |
| Oy | pg/ml | 5.5E-1 | 9.2E-1 | 8.7E0 | 5.1E0 | 4.2E1 | 1.1E1 | 1.0E-9 | 1.0E-9 | 4.0E2 | 6.5E1 | 317 | 79 | 136 | 79 | 0.55 |
| Oz | pg/ml | 4.5E-3 | 1.1E-1 | 2.2E-1 | 6.4E-1 | 3.7E-1 | 3.1E0 | 1.0E-9 | 1.0E-9 | 2.6E0 | 2.8E1 | 317 | 79 | 136 | 79 | 0.55 |
| Pa | pg/ml | 3.4E-1 | 4.5E-1 | 1.3E0 | 4.1E0 | 5.8E0 | 2.5E1 | 1.0E-9 | 1.0E-9 | 8.6E1 | 2.3E2 | 317 | 79 | 136 | 79 | 0.58 |
| Pb | pg/ml | 1.0E-9 | 1.0E-9 | 1.8E0 | 8.5E-1 | 2.7E1 | 4.7E0 | 1.0E-9 | 1.0E-9 | 4.9E2 | 3.2E1 | 317 | 79 | 136 | 79 | 0.50 |
| Pc | pg/ml | 4.4E-2 | 2.1E-1 | 4.3E-1 | 9.0E-1 | 1.3E0 | 4.2E0 | 1.0E-9 | 1.0E-9 | 1.6E1 | 3.8E1 | 317 | 79 | 136 | 79 | 0.54 |
| Pd | pg/ml | 1.8E0 | 3.0E0 | 3.8E0 | 8.0E0 | 8.2E0 | 1.6E1 | 1.0E-9 | 1.0E-9 | 9.4E1 | 1.2E2 | 317 | 79 | 136 | 79 | 0.61 |
| Pe | pg/ml | 1.8E1 | 3.9E1 | 8.9E1 | 4.0E2 | 3.5E2 | 1.8E3 | 1.0E-9 | 1.0E-9 | 4.7E3 | 1.5E4 | 317 | 79 | 136 | 79 | 0.64 |
| Pf | pg/ml | 1.2E0 | 3.0E0 | 6.2E0 | 2.1E1 | 2.6E1 | 5.8E1 | 1.0E-9 | 1.0E-9 | 3.3E2 | 4.3E2 | 317 | 79 | 136 | 79 | 0.64 |
| Pg | pg/ml | 3.2E0 | 4.9E0 | 4.0E1 | 5.3E1 | 2.1E2 | 1.7E2 | 1.0E-9 | 1.0E-9 | 3.2E3 | 1.2E3 | 317 | 79 | 136 | 79 | 0.59 |
| Ph | ng/ml | 1.4E-1 | 2.5E-1 | 2.7E-1 | 4.5E-1 | 3.5E-1 | 8.3E-1 | 1.0E-9 | 1.0E-9 | 2.2E0 | 5.4E0 | 110 | 49 | 86 | 49 | 0.58 |
| Pi | ng/ml | 1.9E-1 | 2.4E-1 | 2.8E-1 | 2.0E0 | 4.4E-1 | 1.2E1 | 1.0E-9 | 1.0E-9 | 3.2E0 | 8.2E1 | 110 | 49 | 86 | 49 | 0.57 |
| Pj | ng/mL | 4.4E0 | 7.6E0 | 5.1E0 | 7.9E0 | 3.3E0 | 5.4E0 | 3.8E-2 | 5.0E-1 | 1.6E1 | 3.1E1 | 110 | 49 | 86 | 49 | 0.68 |
| Pk | ng/ml | 8.1E-3 | 1.2E-2 | 1.1E-2 | 4.9E-2 | 1.2E-2 | 2.2E-1 | 1.0E-9 | 1.0E-9 | 5.9E-2 | 1.5E0 | 110 | 49 | 86 | 49 | 0.58 |
| aA | mg/dL | 8.0E-1 | 9.2E-1 | 9.2E-1 | 1.2E0 | 4.3E-1 | 8.3E-1 | 3.0E-1 | 3.0E-1 | 4.2E0 | 4.7E0 | 1242 | 101 | 226 | 101 | 0.56 |
| aC | mg/mL | 3.1E0 | 2.2E0 | 3.3E0 | 2.5E0 | 1.4E0 | 9.8E-1 | 1.1E0 | 8.5E-1 | 8.2E0 | 5.6E0 | 221 | 52 | 91 | 52 | 0.32 |
| aD | ug/mL | 2.9E0 | 4.0E0 | 4.1E0 | 5.3E0 | 3.2E0 | 3.8E0 | 8.5E-1 | 7.7E-1 | 2.8E1 | 1.9E1 | 221 | 52 | 91 | 52 | 0.60 |
| aE | mg/mL | 5.8E-1 | 5.5E-1 | 5.8E-1 | 5.8E-1 | 1.5E-1 | 1.5E-1 | 2.1E-1 | 3.7E-1 | 1.1E0 | 1.2E0 | 221 | 52 | 91 | 52 | 0.49 |
| aF | ng/mL | 2.1E0 | 2.3E0 | 4.4E0 | 3.9E0 | 7.0E0 | 3.9E0 | 4.3E-3 | 5.2E-1 | 5.0E1 | 1.9E1 | 221 | 52 | 91 | 52 | 0.53 |
| aG | mg/mL | 1.3E-1 | 1.3E-1 | 1.5E-1 | 1.5E-1 | 7.8E-2 | 8.6E-2 | 5.0E-2 | 4.3E-2 | 5.0E-1 | 4.2E-1 | 221 | 52 | 91 | 52 | 0.46 |
| aH | ug/mL | 6.8E1 | 7.3E1 | 7.8E1 | 7.9E1 | 3.5E1 | 4.9E1 | 1.5E1 | 1.1E1 | 2.7E2 | 2.9E2 | 221 | 52 | 91 | 52 | 0.48 |
| aI | ug/mL | 1.8E2 | 1.7E2 | 1.9E2 | 1.7E2 | 6.0E1 | 5.6E1 | 5.8E1 | 4.7E1 | 3.7E2 | 3.0E2 | 221 | 52 | 91 | 52 | 0.43 |
| aJ | ug/mL | 2.5E0 | 2.5E0 | 3.0E0 | 3.8E0 | 1.9E0 | 3.2E0 | 9.0E-1 | 1.1E0 | 1.2E1 | 1.7E1 | 221 | 52 | 91 | 52 | 0.55 |
| aK | ng/mL | 1.8E0 | 1.2E0 | 2.7E0 | 2.4E0 | 2.7E0 | 3.2E0 | 8.4E-2 | 2.9E-4 | 1.8E1 | 1.8E1 | 221 | 52 | 91 | 52 | 0.41 |
| aL | mg/mL | 8.1E-1 | 7.7E-1 | 8.3E-1 | 7.7E-1 | 2.4E-1 | 2.2E-1 | 2.2E-1 | 2.7E-1 | 1.6E0 | 1.6E0 | 221 | 52 | 91 | 52 | 0.43 |
| aM | U/mL | 1.9E1 | 3.7E1 | 3.7E1 | 6.7E1 | 1.1E2 | 1.2E2 | 4.2E-2 | 4.2E-2 | 1.6E3 | 8.2E2 | 221 | 52 | 91 | 52 | 0.65 |
| aN | U/mL | 1.0E1 | 1.5E1 | 1.8E1 | 2.5E1 | 2.1E1 | 2.7E1 | 2.5E-3 | 2.5E-3 | 1.3E2 | 1.1E2 | 221 | 52 | 91 | 52 | 0.60 |
| aO | pg/mL | 3.9E1 | 5.8E1 | 3.7E2 | 3.2E2 | 9.9E2 | 6.0E2 | 6.0E-2 | 6.0E-2 | 6.6E3 | 2.4E3 | 221 | 52 | 91 | 52 | 0.55 |
| aP | ng/mL | 1.7E0 | 1.7E0 | 1.9E0 | 2.3E0 | 1.1E0 | 1.5E0 | 5.4E-1 | 8.0E-1 | 6.6E0 | 6.6E0 | 221 | 52 | 91 | 52 | 0.55 |
| aQ | ng/mL | 3.2E-1 | 2.5E-1 | 5.0E-1 | 3.8E-1 | 5.2E-1 | 4.0E-1 | 1.9E-2 | 2.0E-4 | 4.0E0 | 2.5E0 | 221 | 52 | 91 | 52 | 0.42 |
| aR | ng/mL | 1.6E0 | 2.4E0 | 2.4E0 | 3.9E0 | 2.5E0 | 5.3E0 | 1.8E-1 | 5.6E-1 | 2.1E1 | 3.4E1 | 221 | 52 | 91 | 52 | 0.64 |
| aS | ng/mL | 2.4E-1 | 3.0E-1 | 7.8E-1 | 6.6E-1 | 2.5E0 | 7.5E-1 | 4.2E-3 | 6.0E-2 | 3.3E1 | 3.1E0 | 221 | 52 | 91 | 52 | 0.58 |
| aU | pg/mL | 8.8E1 | 6.4E1 | 1.4E2 | 1.1E2 | 1.6E2 | 1.4E2 | 7.4E0 | 7.4E-2 | 1.3E3 | 8.5E2 | 221 | 52 | 91 | 52 | 0.40 |
| aV | ng/mL | 7.3E-1 | 4.9E-1 | 1.1E0 | 9.4E-1 | 1.1E0 | 1.8E0 | 3.1E-2 | 7.6E-4 | 8.7E0 | 1.3E1 | 221 | 52 | 91 | 52 | 0.38 |
| aW | pg/mL | 1.7E1 | 2.2E1 | 2.0E1 | 3.0E1 | 2.6E1 | 5.5E1 | 7.2E-2 | 7.2E-2 | 2.4E2 | 4.2E2 | 221 | 52 | 91 | 52 | 0.63 |
| aX | ng/mL | 1.0E1 | 8.2E0 | 1.3E1 | 1.5E1 | 1.2E1 | 2.0E1 | 3.0E-1 | 6.2E-1 | 6.7E1 | 1.3E2 | 221 | 52 | 91 | 52 | 0.48 |
| aY | pg/mL | 5.0E1 | 6.6E1 | 7.2E1 | 8.2E1 | 7.1E1 | 6.0E1 | 4.1E-1 | 4.1E-1 | 4.4E2 | 3.4E2 | 221 | 52 | 91 | 52 | 0.59 |
| aZ | pg/mL | 2.2E2 | 2.4E2 | 4.8E2 | 6.0E2 | 7.2E2 | 1.3E3 | 1.7E0 | 1.7E0 | 5.4E3 | 8.3E3 | 221 | 52 | 91 | 52 | 0.51 |
| bA | ng/mL | 8.0E0 | 1.3E1 | 2.6E1 | 6.1E1 | 6.2E1 | 1.4E2 | 3.0E-2 | 3.0E-2 | 7.1E2 | 9.4E2 | 221 | 52 | 91 | 52 | 0.64 |
| bB | ng/mL | 3.1E2 | 2.6E2 | 3.4E2 | 2.6E2 | 1.6E2 | 1.4E2 | 1.6E1 | 1.2E1 | 1.0E3 | 5.7E2 | 221 | 52 | 91 | 52 | 0.36 |
| bC | ng/mL | 3.5E2 | 3.6E2 | 5.1E2 | 7.9E2 | 5.3E2 | 1.1E3 | 2.7E1 | 3.5E1 | 4.0E3 | 4.7E3 | 221 | 52 | 91 | 52 | 0.54 |
| bE | mg/mL | 5.6E0 | 5.6E0 | 5.9E0 | 5.5E0 | 1.7E0 | 2.1E0 | 1.9E0 | 1.3E0 | 1.3E1 | 1.1E1 | 221 | 52 | 91 | 52 | 0.43 |
| bF | pg/mL | 2.0E1 | 3.0E1 | 8.6E1 | 3.5E2 | 4.4E2 | 1.1E3 | 5.0E-2 | 6.1E0 | 5.0E3 | 6.3E3 | 221 | 52 | 91 | 52 | 0.63 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bG | ng/mL | 1.8E0 | 1.7E0 | 3.0E0 | 3.2E0 | 3.7E0 | 4.7E0 | 2.2E-2 | 1.1E-1 | 2.6E1 | 3.0E1 | 221 | 52 | 91 | 52 | 0.49 |
| bH | pg/mL | 5.7E-1 | 5.7E-1 | 5.4E0 | 4.7E0 | 2.0E1 | 6.3E0 | 5.7E-1 | 5.7E-1 | 2.8E2 | 2.7E1 | 221 | 52 | 91 | 52 | 0.53 |
| bI | ng/mL | 4.0E-3 | 4.0E-3 | 6.8E-2 | 8.5E-2 | 1.7E-1 | 1.9E-1 | 4.0E-3 | 4.0E-3 | 1.3E0 | 8.8E-1 | 221 | 52 | 91 | 52 | 0.51 |
| bJ | mg/mL | 2.2E0 | 2.2E0 | 2.5E0 | 2.5E0 | 1.9E0 | 1.9E0 | 2.5E-4 | 2.5E-4 | 1.3E1 | 8.5E0 | 221 | 52 | 91 | 52 | 0.49 |
| bL | pg/mL | 4.1E0 | 3.1E0 | 7.8E0 | 8.1E0 | 9.3E0 | 8.8E0 | 4.6E-2 | 4.6E-2 | 4.9E1 | 3.2E1 | 221 | 52 | 91 | 52 | 0.50 |
| bM | mg/mL | 1.5E0 | 2.2E0 | 1.8E0 | 2.6E0 | 1.2E0 | 1.6E0 | 9.2E-3 | 1.8E-2 | 7.1E0 | 8.4E0 | 221 | 52 | 91 | 52 | 0.66 |
| bN | ng/mL | 3.2E1 | 5.8E1 | 1.1E2 | 9.5E1 | 2.6E2 | 1.4E2 | 9.7E-1 | 5.9E-1 | 1.9E3 | 7.5E2 | 221 | 52 | 91 | 52 | 0.55 |
| bO | ng/mL | 4.0E-2 | 4.0E-2 | 1.1E1 | 1.0E1 | 2.4E1 | 2.0E1 | 4.0E-2 | 4.0E-2 | 1.9E2 | 7.1E1 | 221 | 52 | 91 | 52 | 0.46 |
| bP | mg/mL | 5.6E-1 | 4.6E-1 | 7.9E-1 | 7.5E-1 | 6.6E-1 | 7.7E-1 | 8.2E-2 | 9.7E-2 | 3.8E0 | 3.5E0 | 221 | 52 | 91 | 52 | 0.46 |
| bQ | pg/mL | 1.4E1 | 2.3E1 | 2.4E1 | 9.4E1 | 3.5E1 | 3.4E2 | 1.5E-1 | 1.5E-1 | 3.2E2 | 2.4E3 | 221 | 52 | 91 | 52 | 0.63 |
| bR | ng/mL | 1.2E-2 | 1.2E-2 | 1.4E-1 | 5.9E-2 | 2.8E-1 | 1.0E-1 | 1.2E-2 | 1.2E-2 | 3.4E0 | 4.8E-1 | 221 | 52 | 91 | 52 | 0.39 |
| bS | pg/mL | 9.4E-1 | 9.4E-1 | 8.1E0 | 5.9E0 | 2.9E1 | 1.3E1 | 9.4E-1 | 9.4E-1 | 3.4E2 | 6.1E1 | 221 | 52 | 91 | 52 | 0.50 |
| bU | ng/mL | 1.6E-1 | 6.1E-2 | 2.1E-1 | 1.1E-1 | 2.6E-1 | 1.3E-1 | 1.3E-2 | 1.3E-2 | 1.8E0 | 4.7E-1 | 221 | 52 | 91 | 52 | 0.38 |
| bV | pg/mL | 4.7E2 | 5.2E2 | 5.3E2 | 6.1E2 | 2.5E2 | 3.7E2 | 1.7E2 | 2.2E2 | 1.6E3 | 2.0E3 | 221 | 52 | 91 | 52 | 0.54 |
| bW | pg/mL | 3.5E2 | 3.4E2 | 4.7E2 | 9.7E2 | 4.2E2 | 3.4E3 | 1.1E2 | 1.2E2 | 3.4E3 | 2.5E4 | 221 | 52 | 91 | 52 | 0.52 |
| bX | ng/mL | 3.1E-3 | 2.5E-5 | 3.2E-3 | 2.3E-3 | 3.8E-3 | 2.8E-3 | 2.5E-5 | 2.5E-5 | 2.0E-2 | 8.5E-3 | 221 | 52 | 91 | 52 | 0.44 |
| bZ | pg/mL | 2.5E2 | 2.7E2 | 7.5E2 | 1.9E3 | 3.2E3 | 7.2E3 | 1.5E-1 | 3.5E1 | 4.4E4 | 4.3E4 | 221 | 52 | 91 | 52 | 0.53 |
| cA | pg/mL | 6.0E-1 | 6.0E-1 | 1.9E0 | 2.4E0 | 3.4E0 | 4.6E0 | 6.0E-1 | 6.0E-1 | 1.4E1 | 1.8E1 | 221 | 52 | 91 | 52 | 0.51 |
| cB | ng/mL | 6.0E-2 | 2.7E-2 | 9.0E-2 | 4.0E-2 | 1.0E-1 | 4.3E-2 | 1.7E-3 | 1.7E-3 | 5.4E-1 | 2.1E-1 | 221 | 52 | 91 | 52 | 0.33 |
| cC | pg/mL | 4.6E1 | 4.9E1 | 4.9E1 | 4.2E1 | 3.9E1 | 2.9E1 | 1.0E0 | 1.0E0 | 3.7E2 | 1.1E2 | 221 | 52 | 91 | 52 | 0.46 |
| cD | pg/mL | 6.1E0 | 4.5E0 | 1.3E1 | 1.5E1 | 4.0E1 | 4.3E1 | 3.3E-1 | 3.3E-1 | 4.8E2 | 2.9E2 | 221 | 52 | 91 | 52 | 0.44 |
| cE | pg/mL | 3.1E1 | 7.3E1 | 9.3E1 | 2.5E2 | 2.7E2 | 5.5E2 | 1.5E0 | 1.2E-1 | 3.1E3 | 3.1E3 | 221 | 52 | 91 | 52 | 0.64 |
| cF | pg/mL | 1.3E1 | 5.3E-1 | 2.4E1 | 9.1E0 | 3.6E1 | 2.0E1 | 5.3E-1 | 5.3E-1 | 2.2E2 | 1.3E2 | 221 | 52 | 91 | 52 | 0.34 |
| cG | pg/mL | 4.2E1 | 5.8E1 | 6.4E1 | 1.2E2 | 8.8E1 | 1.9E2 | 1.1E1 | 1.8E1 | 1.1E3 | 1.2E3 | 221 | 52 | 91 | 52 | 0.63 |
| cII | uIU/mL | 2.9E0 | 2.2E0 | 6.0E0 | 5.7E0 | 8.9E0 | 9.7E0 | 8.6E-3 | 8.6E-3 | 8.7E1 | 5.3E1 | 221 | 52 | 91 | 52 | 0.44 |
| cI | ng/mL | 6.0E0 | 3.8E0 | 1.1E1 | 1.0E1 | 1.3E1 | 1.9E1 | 1.0E-3 | 1.0E-3 | 9.4E1 | 1.2E2 | 221 | 52 | 91 | 52 | 0.41 |
| cJ | ug/mL | 7.2E1 | 5.1E1 | 1.2E2 | 8.4E1 | 1.5E2 | 1.1E2 | 4.0E0 | 8.4E0 | 9.6E2 | 6.3E2 | 221 | 52 | 91 | 52 | 0.42 |
| cK | ng/mL | 3.8E-3 | 3.8E-3 | 7.5E-2 | 2.0E-2 | 2.2E-1 | 5.4E-2 | 3.8E-3 | 3.8E-3 | 1.7E0 | 3.1E-1 | 221 | 52 | 91 | 52 | 0.46 |
| cL | pg/mL | 1.9E2 | 2.4E2 | 2.7E2 | 3.6E2 | 5.1E2 | 8.0E2 | 2.5E1 | 3.7E1 | 7.1E3 | 5.9E3 | 221 | 52 | 91 | 52 | 0.56 |
| cM | pg/mL | 2.9E2 | 2.6E2 | 3.2E2 | 2.9E2 | 1.8E2 | 2.1E2 | 3.7E1 | 4.7E1 | 1.2E3 | 1.4E3 | 221 | 52 | 91 | 52 | 0.43 |
| cN | pg/mL | 1.2E2 | 1.2E2 | 1.2E2 | 1.4E2 | 4.4E1 | 5.7E1 | 3.8E1 | 6.5E1 | 2.7E2 | 3.2E2 | 221 | 52 | 91 | 52 | 0.59 |
| cO | pg/mL | 2.3E2 | 2.4E2 | 2.8E2 | 3.0E2 | 2.0E2 | 3.2E2 | 5.4E1 | 9.9E1 | 1.7E3 | 2.4E3 | 221 | 52 | 91 | 52 | 0.50 |
| cP | ng/mL | 2.4E3 | 2.8E3 | 2.5E3 | 2.8E3 | 8.9E2 | 9.3E2 | 6.2E2 | 1.3E3 | 5.7E3 | 5.3E3 | 221 | 52 | 91 | 52 | 0.59 |
| cQ | ng/mL | 4.0E-2 | 5.5E-2 | 1.1E-1 | 1.5E-1 | 1.8E-1 | 3.2E-1 | 2.0E-3 | 2.0E-3 | 1.2E0 | 2.1E0 | 221 | 52 | 91 | 52 | 0.56 |
| cR | ng/mL | 2.8E2 | 3.7E2 | 4.3E2 | 6.5E2 | 4.6E2 | 1.2E3 | 2.3E1 | 2.0E1 | 3.8E3 | 7.7E3 | 221 | 52 | 91 | 52 | 0.55 |
| cS | ng/mL | 2.4E2 | 3.1E2 | 3.9E2 | 4.3E2 | 4.2E2 | 3.2E2 | 5.3E1 | 4.8E1 | 2.7E3 | 1.3E3 | 221 | 52 | 91 | 52 | 0.58 |
| cT | ng/mL | 2.8E1 | 4.0E1 | 7.4E1 | 1.4E2 | 1.5E2 | 2.6E2 | 4.6E0 | 8.8E0 | 1.7E3 | 1.4E3 | 221 | 52 | 91 | 52 | 0.63 |
| cU | ng/mL | 5.5E1 | 5.5E1 | 7.4E1 | 7.8E1 | 7.2E1 | 6.7E1 | 1.0E1 | 1.4E1 | 7.7E2 | 3.4E2 | 221 | 52 | 91 | 52 | 0.51 |
| cV | ng/mL | 1.7E-1 | 2.2E-1 | 4.9E-1 | 6.0E-1 | 3.2E0 | 1.3E0 | 3.4E-4 | 3.6E-2 | 4.7E1 | 8.7E0 | 221 | 52 | 91 | 52 | 0.58 |
| cW | mIU/mL | 4.6E-2 | 6.5E-2 | 2.2E-1 | 8.7E-2 | 1.0E0 | 7.5E-2 | 3.7E-4 | 1.9E-2 | 9.7E0 | 3.9E-1 | 221 | 52 | 91 | 52 | 0.63 |
| cX | ng/mL | 1.1E-1 | 2.2E-1 | 1.8E0 | 1.5E0 | 5.4E0 | 4.5E0 | 2.3E-4 | 2.3E-4 | 2.8E1 | 2.8E1 | 221 | 52 | 91 | 52 | 0.53 |
| cY | ng/mL | 1.0E1 | 7.3E0 | 1.4E1 | 1.1E1 | 1.4E1 | 1.2E1 | 4.4E-1 | 1.7E-1 | 8.3E1 | 7.5E1 | 221 | 52 | 91 | 52 | 0.40 |
| cZ | ug/mL | 1.4E1 | 1.4E1 | 1.5E1 | 1.5E1 | 5.8E0 | 7.1E0 | 5.3E0 | 2.8E0 | 3.9E1 | 3.4E1 | 221 | 52 | 91 | 52 | 0.49 |
| dA | pg/mL | 3.3E2 | 3.1E2 | 3.6E2 | 3.6E2 | 1.6E2 | 1.9E2 | 9.0E1 | 1.4E2 | 1.3E3 | 1.1E3 | 221 | 52 | 91 | 52 | 0.47 |
| dB | ug/mL | 1.7E1 | 2.1E1 | 1.9E1 | 1.9E1 | 2.1E1 | 8.7E0 | 1.9E0 | 2.2E0 | 2.5E2 | 4.0E1 | 221 | 52 | 91 | 52 | 0.60 |
| dC | nmol/L | 3.4E1 | 3.8E1 | 4.1E1 | 4.0E1 | 2.0E1 | 1.5E1 | 9.1E0 | 1.9E1 | 1.4E2 | 9.2E1 | 221 | 52 | 91 | 52 | 0.53 |
| dD | ug/mL | 3.7E1 | 3.3E1 | 3.8E1 | 3.4E1 | 1.0E1 | 1.0E1 | 1.3E1 | 1.4E1 | 7.6E1 | 5.6E1 | 221 | 52 | 91 | 52 | 0.40 |
| dE | ng/mL | 4.8E-1 | 4.5E-1 | 6.7E-1 | 5.7E-1 | 8.3E-1 | 6.0E-1 | 8.4E-3 | 8.4E-3 | 7.2E0 | 2.8E0 | 221 | 52 | 91 | 52 | 0.47 |
| dF | ng/mL | 2.1E2 | 2.5E2 | 2.6E2 | 3.7E2 | 1.8E2 | 2.7E2 | 7.5E1 | 9.7E1 | 1.2E3 | 1.2E3 | 221 | 52 | 91 | 52 | 0.63 |
| dG | ng/mL | 1.1E1 | 1.3E1 | 1.3E1 | 1.8E1 | 8.4E0 | 1.5E1 | 3.1E0 | 5.5E0 | 6.4E1 | 8.7E1 | 221 | 52 | 91 | 52 | 0.59 |
| dH | pg/mL | 7.5E0 | 9.2E0 | 1.2E1 | 1.4E1 | 2.4E1 | 2.8E1 | 4.0E-2 | 4.0E-2 | 3.1E2 | 2.0E2 | 221 | 52 | 91 | 52 | 0.56 |
| dI | pg/mL | 4.6E-1 | 4.6E-1 | 1.9E0 | 2.1E0 | 4.4E0 | 4.0E0 | 4.6E-1 | 4.6E-1 | 3.4E1 | 2.3E1 | 221 | 52 | 91 | 52 | 0.53 |
| dJ | ng/mL | 1.9E0 | 2.0E0 | 2.1E0 | 2.2E0 | 1.1E0 | 1.6E0 | 3.2E-2 | 3.2E-2 | 5.9E0 | 6.9E0 | 221 | 52 | 91 | 52 | 0.50 |

113

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dK | uIU/mL | 2.0E0 | 1.5E0 | 2.7E0 | 2.6E0 | 2.9E0 | 3.4E0 | 2.8E-4 | 2.9E-2 | 1.6E1 | 2.2E1 | 221 | 52 | 91 | 52 | 0.47 |
| dL | ng/mL | 8.8E2 | 9.1E2 | 1.0E3 | 1.2E3 | 4.8E2 | 8.2E2 | 3.4E2 | 4.5E2 | 3.4E3 | 4.8E3 | 221 | 52 | 91 | 52 | 0.53 |
| dM | pg/mL | 9.7E2 | 9.2E2 | 1.2E3 | 1.5E3 | 1.1E3 | 1.7E3 | 4.4E2 | 4.1E2 | 1.2E4 | 9.6E3 | 221 | 52 | 91 | 52 | 0.49 |
| dN | ug/mL | 9.0E1 | 1.1E2 | 9.7E1 | 1.2E2 | 3.4E1 | 4.9E1 | 2.5E1 | 4.1E1 | 2.4E2 | 3.3E2 | 221 | 52 | 91 | 52 | 0.64 |
| dR | pg/ml | 1.6E3 | 1.2E3 | 2.5E3 | 2.2E3 | 2.6E3 | 2.6E3 | 1.9E2 | 1.4E2 | 1.5E4 | 9.4E3 | 131 | 45 | 88 | 45 | 0.41 |
| dU | pg/ml | 8.8E3 | 2.0E4 | 1.3E4 | 1.9E4 | 1.2E4 | 1.4E4 | 2.5E3 | 6.9E2 | 5.3E4 | 4.6E4 | 28 | 10 | 26 | 10 | 0.64 |
| dX | ng/ml | 3.4E-2 | 6.7E-2 | 9.5E-2 | 1.8E-1 | 1.5E-1 | 2.6E-1 | 2.6E-3 | 2.6E-3 | 7.4E-1 | 8.1E-1 | 65 | 14 | 21 | 14 | 0.56 |
| dW | ng/ml | 1.9E-1 | 2.0E-1 | 2.3E-1 | 3.0E-1 | 1.6E-1 | 2.6E-1 | 5.0E-2 | 6.8E-2 | 5.8E-1 | 8.0E-1 | 29 | 8 | 6 | 8 | 0.55 |
| eF | ng/ml | 4.0E0 | 4.3E0 | 4.6E0 | 5.1E0 | 2.6E0 | 2.5E0 | 1.5E0 | 2.0E0 | 1.8E1 | 1.5E1 | 138 | 45 | 88 | 45 | 0.58 |
| eC | pg/ml | 3.1E2 | 2.6E2 | 3.8E2 | 3.7E2 | 2.5E2 | 3.7E2 | 4.5E1 | 7.1E1 | 1.4E3 | 2.0E3 | 100 | 44 | 86 | 44 | 0.41 |
| eD | pg/ml | 2.3E2 | 1.9E2 | 7.7E2 | 4.3E2 | 1.4E3 | 1.1E3 | 5.2E-1 | 5.2E-1 | 6.8E3 | 7.0E3 | 77 | 41 | 64 | 41 | 0.43 |
| eO | ng/ml | 5.7E1 | 8.0E1 | 3.5E2 | 1.2E2 | 4.1E2 | 9.6E1 | 2.0E1 | 4.3E1 | 1.2E3 | 3.3E2 | 29 | 8 | 6 | 8 | 0.54 |
| eM | ng/ml | 3.6E0 | 2.5E0 | 4.4E0 | 5.0E0 | 3.5E0 | 6.2E0 | 8.1E-1 | 7.2E-1 | 2.2E1 | 2.6E1 | 87 | 19 | 34 | 19 | 0.44 |
| eP | ng/ml | 3.7E-3 | 3.7E-3 | 1.0E0 | 2.3E0 | 2.1E0 | 7.3E0 | 3.7E-3 | 3.7E-3 | 1.2E1 | 2.8E1 | 65 | 14 | 21 | 14 | 0.47 |
| eT | ng/ml | 2.7E2 | 2.5E2 | 5.6E2 | 7.0E2 | 6.1E2 | 8.6E2 | 1.0E2 | 7.1E1 | 2.5E3 | 2.9E3 | 51 | 23 | 49 | 23 | 0.53 |
| eQ | pg/ml | 1.0E0 | 1.0E0 | 1.1E2 | 3.9E1 | 3.2E2 | 6.0E1 | 1.0E0 | 1.0E0 | 1.6E3 | 1.5E2 | 28 | 10 | 26 | 10 | 0.52 |
| eW | U/ml | 6.7E-3 | 3.9E-2 | 5.5E-2 | 2.5E-1 | 9.2E-2 | 5.3E-1 | 6.7E-3 | 6.7E-3 | 3.1E-1 | 1.6E0 | 29 | 8 | 6 | 8 | 0.59 |
| fA | ng/ml | 2.2E2 | 1.2E2 | 4.0E2 | 3.1E2 | 4.6E2 | 4.4E2 | 2.6E1 | 4.0E1 | 1.5E3 | 1.4E3 | 28 | 10 | 26 | 10 | 0.43 |
| eZ | ng/ml | 6.1E1 | 5.4E1 | 6.4E1 | 5.7E1 | 2.6E1 | 2.4E1 | 2.3E1 | 1.8E1 | 1.2E2 | 1.2E2 | 51 | 23 | 49 | 23 | 0.43 |
| fB | ng/ml | 6.1E2 | 6.6E2 | 6.8E2 | 6.7E2 | 2.8E2 | 2.4E2 | 1.6E2 | 2.6E2 | 1.3E3 | 1.0E3 | 28 | 12 | 26 | 12 | 0.52 |
| fN | ng/ml | 2.1E-1 | 2.1E-1 | 2.7E0 | 1.8E1 | 5.8E0 | 6.5E1 | 2.1E-1 | 2.1E-1 | 3.2E1 | 3.2E2 | 51 | 23 | 49 | 23 | 0.58 |
| fP | ng/ml | 2.3E2 | 3.6E2 | 2.7E2 | 3.5E2 | 1.7E2 | 1.6E2 | 8.4E0 | 3.7E1 | 1.0E3 | 8.2E2 | 127 | 45 | 88 | 45 | 0.67 |
| fR | ng/ml | 1.3E5 | 1.7E5 | 1.6E5 | 2.7E5 | 1.1E5 | 2.1E5 | 3.1E4 | 1.9E2 | 6.1E5 | 6.8E5 | 162 | 31 | 44 | 31 | 0.65 |
| fY | ng/ml | 2.5E2 | 2.6E2 | 2.4E2 | 2.6E2 | 8.9E1 | 1.0E2 | 6.5E1 | 3.6E1 | 4.2E2 | 4.7E2 | 51 | 23 | 49 | 23 | 0.56 |
| gC | ng/ml | 2.3E2 | 2.4E2 | 2.5E2 | 2.5E2 | 9.4E1 | 1.0E2 | 1.2E2 | 8.3E1 | 6.4E2 | 4.0E2 | 71 | 12 | 39 | 12 | 0.54 |
| gL | pg/ml | 6.1E4 | 6.9E4 | 6.8E4 | 7.4E4 | 2.9E4 | 3.1E4 | 2.2E4 | 3.4E4 | 1.8E5 | 1.7E5 | 131 | 45 | 88 | 45 | 0.57 |
| gP | U/ml | 2.5E2 | 3.0E2 | 2.6E2 | 2.8E2 | 7.9E1 | 1.1E2 | 8.5E1 | 1.2E1 | 5.3E2 | 6.5E2 | 137 | 45 | 88 | 45 | 0.59 |
| gW | ng/ml | 7.0E2 | 5.1E2 | 1.5E3 | 1.1E3 | 1.9E3 | 1.6E3 | 3.7E1 | 3.1E-1 | 9.5E3 | 5.8E3 | 109 | 36 | 79 | 36 | 0.42 |
| gV | ng/ml | 2.0E1 | 1.6E1 | 2.1E1 | 1.7E1 | 6.7E0 | 8.7E0 | 1.0E1 | 8.1E-2 | 3.9E1 | 3.0E1 | 59 | 9 | 15 | 9 | 0.37 |
| tF | pg/mL | 2.6E3 | 1.2E3 | 2.2E4 | 1.1E4 | 5.6E4 | 4.2E4 | 1.2E1 | 1.8E1 | 3.2E5 | 2.5E5 | 100 | 44 | 86 | 44 | 0.39 |
| gZ | ug/ml | 8.9E-1 | 6.1E-1 | 4.5E1 | 4.1E1 | 1.1E2 | 1.3E2 | 8.7E-2 | 1.1E-1 | 4.1E2 | 4.0E2 | 28 | 10 | 26 | 10 | 0.45 |
| hA | ng/ml | 2.3E0 | 2.6E0 | 7.4E0 | 6.8E0 | 2.4E1 | 1.1E1 | 1.7E-2 | 1.7E-2 | 1.6E2 | 6.1E1 | 77 | 43 | 64 | 43 | 0.53 |
| nM | pg/ml | 1.0E-9 | 1.0E-9 | 1.9E2 | 1.0E-9 | 1.4E3 | 0.0E0 | 1.0E-9 | 1.0E-9 | 1.0E4 | 1.0E-9 | 54 | 30 | 47 | 30 | 0.48 |
| nN | pg/ml | 1.1E3 | 3.2E3 | 2.1E3 | 1.6E4 | 2.7E3 | 5.1E4 | 1.1E2 | 6.8E2 | 1.7E4 | 2.7E5 | 54 | 30 | 47 | 30 | 0.72 |
| nO | pg/ml | 3.1E1 | 3.6E1 | 4.5E1 | 5.2E1 | 4.7E1 | 6.5E1 | 5.5E0 | 4.0E0 | 2.4E2 | 3.1E2 | 54 | 30 | 47 | 30 | 0.50 |
| nR | pg/ml | 1.6E1 | 3.0E1 | 4.5E1 | 9.3E1 | 7.3E1 | 1.9E2 | 1.0E-9 | 1.0E0 | 3.6E2 | 8.2E2 | 54 | 30 | 47 | 30 | 0.61 |
| nT | pg/ml | 8.5E1 | 8.6E1 | 3.5E2 | 1.1E2 | 1.2E3 | 1.2E2 | 1.0E-9 | 1.0E-9 | 6.6E3 | 6.4E2 | 54 | 30 | 47 | 30 | 0.53 |
| nU | pg/ml | 2.9E1 | 6.0E1 | 5.1E2 | 1.3E2 | 2.2E3 | 2.8E2 | 1.0E-9 | 1.0E-9 | 1.3E4 | 1.5E3 | 54 | 30 | 47 | 30 | 0.60 |
| lW | pg/ml | 1.0E-9 | 1.0E-9 | 1.6E1 | 1.9E1 | 5.5E1 | 5.9E1 | 1.0E-9 | 1.0E-9 | 3.9E2 | 2.9E2 | 54 | 30 | 47 | 30 | 0.47 |
| lX | pg/ml | 9.5E2 | 8.2E2 | 9.2E2 | 9.2E2 | 4.4E2 | 6.3E2 | 2.3E2 | 1.3E2 | 1.9E3 | 2.5E3 | 54 | 30 | 47 | 30 | 0.45 |
| lY | pg/ml | 1.7E1 | 1.9E1 | 2.2E1 | 2.1E1 | 2.5E1 | 1.4E1 | 1.0E-9 | 1.0E-9 | 1.4E2 | 5.5E1 | 54 | 30 | 47 | 30 | 0.55 |
| mE | pg/ml | 1.0E-9 | 1.0E-9 | 3.3E0 | 1.7E0 | 1.0E1 | 3.3E0 | 1.0E-9 | 1.0E-9 | 5.8E1 | 1.6E1 | 54 | 30 | 47 | 30 | 0.57 |
| mF | pg/ml | 1.0E-9 | 3.6E-1 | 1.4E0 | 2.1E0 | 3.0E0 | 3.7E0 | 1.0E-9 | 1.0E-9 | 1.5E1 | 1.3E1 | 54 | 30 | 47 | 30 | 0.60 |
| mH | pg/ml | 3.6E0 | 2.9E0 | 4.9E0 | 3.9E0 | 5.0E0 | 3.8E0 | 2.3E-1 | 4.0E-1 | 3.2E1 | 1.9E1 | 54 | 30 | 47 | 30 | 0.44 |
| mI | pg/ml | 1.0E-9 | 4.9E0 | 1.2E1 | 1.4E1 | 3.1E1 | 1.9E1 | 1.0E-9 | 1.0E-9 | 1.6E2 | 6.7E1 | 54 | 30 | 47 | 30 | 0.61 |
| mM | pg/ml | 3.0E1 | 5.2E1 | 5.2E1 | 1.6E2 | 5.9E1 | 2.7E2 | 1.0E-9 | 1.0E-9 | 2.3E2 | 1.1E3 | 54 | 30 | 47 | 30 | 0.60 |
| mP | pg/ml | 1.5E1 | 1.5E1 | 2.0E1 | 2.4E1 | 2.2E1 | 3.7E1 | 1.0E-9 | 1.6E0 | 1.5E2 | 1.9E2 | 53 | 30 | 46 | 30 | 0.50 |
| mS | pg/ml | 1.8E3 | 1.8E3 | 2.2E3 | 1.8E3 | 2.2E3 | 8.9E2 | 1.0E-9 | 1.0E-9 | 1.3E4 | 3.5E3 | 54 | 30 | 47 | 30 | 0.47 |
| mT | pg/ml | 5.0E1 | 7.6E1 | 1.2E2 | 1.4E2 | 2.2E2 | 2.0E2 | 1.0E1 | 1.2E1 | 1.4E3 | 9.7E2 | 53 | 30 | 46 | 30 | 0.56 |
| mU | pg/ml | 2.5E0 | 2.2E0 | 4.2E0 | 1.2E1 | 8.5E0 | 4.2E1 | 1.0E-9 | 5.5E-1 | 5.8E1 | 2.2E2 | 53 | 30 | 46 | 30 | 0.52 |
| mW | pg/ml | 2.5E3 | 2.1E3 | 2.7E3 | 2.5E3 | 1.7E3 | 1.6E3 | 4.3E2 | 1.0E-9 | 1.0E4 | 6.2E3 | 53 | 30 | 46 | 30 | 0.45 |
| mY | pg/ml | 4.7E2 | 7.9E2 | 1.0E3 | 9.5E2 | 1.8E3 | 1.0E3 | 1.0E-9 | 1.0E-9 | 1.1E4 | 5.6E3 | 54 | 30 | 47 | 30 | 0.61 |
| mZ | pg/ml | 2.6E2 | 2.4E2 | 5.0E2 | 3.8E2 | 6.0E2 | 3.8E2 | 2.1E1 | 3.9E1 | 3.1E3 | 1.5E3 | 53 | 30 | 46 | 30 | 0.45 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| nA | pg/ml | 1.3E0 | 2.1E0 | 1.6E1 | 7.0E0 | 6.6E1 | 1.3E1 | 1.0E-9 | 1.0E-9 | 4.4E2 | 5.1E1 | 53 | 30 | 46 | 30 | 0.52 |
| nB | pg/ml | 2.6E2 | 3.2E2 | 3.1E2 | 3.2E2 | 1.6E2 | 1.6E2 | 3.0E1 | 3.7E1 | 7.2E2 | 8.2E2 | 54 | 30 | 47 | 30 | 0.55 |
| nC | pg/ml | 1.0E-9 | 7.9E1 | 8.7E3 | 1.4E4 | 5.0E4 | 7.0E4 | 1.0E-9 | 1.0E-9 | 3.7E5 | 3.8E5 | 54 | 30 | 47 | 30 | 0.58 |
| nD | pg/ml | 7.2E0 | 7.6E0 | 7.0E1 | 1.0E1 | 3.1E2 | 9.6E0 | 1.0E-9 | 1.0E-9 | 1.7E3 | 3.0E1 | 53 | 30 | 46 | 30 | 0.52 |
| nF | pg/ml | 1.0E-9 | 1.0E-9 | 9.5E0 | 1.9E0 | 4.0E1 | 6.9E0 | 1.0E-9 | 1.0E-9 | 2.4E2 | 3.5E1 | 54 | 30 | 47 | 30 | 0.51 |
| nH | pg/ml | 3.0E-2 | 3.9E0 | 2.1E2 | 1.0E2 | 1.4E3 | 4.8E2 | 1.0E-9 | 1.0E-9 | 1.0E4 | 2.6E3 | 53 | 30 | 46 | 30 | 0.60 |
| nI | pg/ml | 4.6E1 | 1.6E1 | 2.9E2 | 5.6E1 | 1.3E3 | 1.0E2 | 1.0E-9 | 1.0E-9 | 9.2E3 | 5.2E2 | 54 | 30 | 47 | 30 | 0.41 |
| nJ | pg/ml | 1.7E-1 | 5.1E-1 | 9.9E1 | 1.4E0 | 7.0E2 | 3.1E0 | 1.0E-9 | 1.0E-9 | 5.2E3 | 1.7E1 | 54 | 30 | 47 | 30 | 0.52 |
| nK | pg/ml | 1.0E-9 | 5.4E0 | 1.2E2 | 1.7E1 | 5.4E2 | 2.6E1 | 1.0E-9 | 1.0E-9 | 3.2E3 | 1.2E2 | 53 | 30 | 46 | 30 | 0.61 |
| nL | pg/ml | 1.0E-9 | 4.3E0 | 9.3E2 | 2.0E2 | 6.1E3 | 7.9E2 | 1.0E-9 | 1.0E-9 | 4.5E4 | 4.3E3 | 54 | 30 | 47 | 30 | 0.58 |
| hL | pg/ml | 1.7E4 | 1.8E4 | 2.3E4 | 2.1E4 | 2.2E4 | 1.3E4 | 2.6E3 | 2.6E3 | 1.4E5 | 6.0E4 | 51 | 23 | 49 | 23 | 0.51 |
| hO | pg/ml | 1.6E4 | 1.6E4 | 1.7E4 | 1.6E4 | 3.2E3 | 3.1E3 | 1.1E4 | 1.1E4 | 2.8E4 | 2.3E4 | 51 | 23 | 49 | 23 | 0.44 |
| hP | ng/ml | 4.4E5 | 4.4E5 | 3.7E5 | 4.8E5 | 1.7E5 | 5.4E5 | 2.8E4 | 3.4E4 | 9.0E5 | 2.8E6 | 51 | 23 | 49 | 23 | 0.51 |
| wJ | pg/ml | 1.5E5 | 1.0E5 | 1.6E5 | 1.7E5 | 8.7E4 | 1.3E5 | 2.8E4 | 3.6E4 | 4.0E5 | 5.8E5 | 48 | 25 | 44 | 25 | 0.44 |
| wK | pg/ml | 3.4E4 | 4.2E4 | 4.3E4 | 5.0E4 | 2.7E4 | 4.2E4 | 5.2E3 | 8.1E3 | 1.2E5 | 2.0E5 | 48 | 25 | 44 | 25 | 0.51 |
| wL | pg/ml | 6.5E0 | 1.9E0 | 7.0E1 | 2.0E1 | 1.5E2 | 6.4E1 | 1.0E-9 | 1.0E-9 | 8.4E2 | 3.2E2 | 48 | 25 | 44 | 25 | 0.37 |
| wP | pg/ml | 2.8E4 | 2.8E4 | 4.3E4 | 6.5E4 | 4.3E4 | 8.2E4 | 2.8E3 | 4.5E3 | 1.6E5 | 3.0E5 | 48 | 25 | 44 | 25 | 0.55 |
| wQ | pg/ml | 3.4E1 | 4.1E1 | 6.0E1 | 5.4E1 | 7.7E1 | 6.1E1 | 1.0E-9 | 1.0E-9 | 3.7E2 | 2.4E2 | 48 | 25 | 44 | 25 | 0.50 |
| hR | pg/ml | 2.7E4 | 2.5E4 | 3.0E4 | 2.5E4 | 1.1E4 | 1.0E4 | 1.8E3 | 1.0E-9 | 5.8E4 | 4.5E4 | 75 | 39 | 62 | 39 | 0.40 |
| hV | pg/ml | 4.4E2 | 3.9E2 | 4.7E2 | 4.4E2 | 2.4E2 | 2.6E2 | 1.3E2 | 1.0E-9 | 1.5E3 | 1.3E3 | 75 | 39 | 62 | 39 | 0.46 |
| hW | pg/ml | 1.6E3 | 1.9E3 | 2.1E3 | 3.1E3 | 1.6E3 | 6.2E3 | 5.7E2 | 1.0E-9 | 1.0E4 | 4.0E4 | 75 | 39 | 62 | 39 | 0.56 |
| hX | pg/ml | 9.6E2 | 8.4E2 | 1.2E3 | 9.8E2 | 1.1E3 | 5.2E2 | 4.8E2 | 2.5E0 | 8.6E3 | 2.9E3 | 75 | 39 | 62 | 39 | 0.43 |
| iA | pg/ml | 1.7E2 | 1.9E2 | 4.2E2 | 2.7E2 | 9.2E2 | 2.1E2 | 1.8E1 | 1.1E1 | 7.1E3 | 7.8E2 | 100 | 43 | 86 | 43 | 0.55 |
| iB | ng/ml | 5.4E0 | 6.5E0 | 6.7E0 | 7.9E0 | 4.7E0 | 6.1E0 | 2.5E-1 | 8.8E-2 | 2.0E1 | 2.4E1 | 77 | 43 | 64 | 43 | 0.54 |
| iC | U/ml | 2.4E-1 | 4.6E-1 | 5.0E-1 | 1.6E0 | 8.7E-1 | 5.1E0 | 1.0E-9 | 1.0E-9 | 6.4E0 | 3.2E1 | 77 | 43 | 64 | 43 | 0.65 |
| tQ | pg/ml | 1.1E3 | 1.4E3 | 1.2E3 | 1.4E3 | 5.5E2 | 6.8E2 | 2.8E2 | 5.3E2 | 2.5E3 | 3.3E3 | 46 | 24 | 43 | 24 | 0.58 |
| tT | pg/ml | 1.5E1 | 2.1E1 | 1.7E1 | 3.2E1 | 7.4E0 | 2.7E1 | 7.4E0 | 6.7E0 | 3.9E1 | 1.2E2 | 46 | 24 | 43 | 24 | 0.70 |
| tS | pg/ml | 1.0E0 | 1.2E0 | 1.1E0 | 2.3E0 | 8.6E-1 | 2.9E0 | 1.0E-9 | 1.0E-9 | 4.0E0 | 1.0E1 | 46 | 24 | 43 | 24 | 0.59 |
| tX | pg/ml | 8.1E-1 | 1.3E0 | 9.5E-1 | 2.4E0 | 7.0E-1 | 2.6E0 | 2.5E-2 | 3.7E-1 | 3.3E0 | 1.0E1 | 46 | 24 | 43 | 24 | 0.68 |
| tO | pg/ml | 4.0E0 | 4.7E0 | 4.4E0 | 6.3E0 | 2.5E0 | 4.9E0 | 1.0E-9 | 1.6E0 | 1.1E1 | 1.9E1 | 46 | 24 | 43 | 24 | 0.57 |
| tR | pg/ml | 2.1E-1 | 2.4E-1 | 2.5E-1 | 5.0E-1 | 2.1E-1 | 6.7E-1 | 1.0E-9 | 1.0E-9 | 9.1E-1 | 2.5E0 | 46 | 24 | 43 | 24 | 0.58 |
| tU | pg/ml | 9.0E0 | 1.1E1 | 1.1E1 | 1.5E1 | 6.9E0 | 1.6E1 | 1.6E0 | 1.5E0 | 3.1E1 | 8.0E1 | 46 | 25 | 43 | 25 | 0.58 |
| tN | pg/ml | 1.7E1 | 1.9E1 | 1.9E1 | 3.9E1 | 1.0E1 | 3.9E1 | 1.0E-9 | 9.4E0 | 5.4E1 | 1.6E2 | 46 | 23 | 43 | 23 | 0.64 |
| tV | ng/ml | 3.7E2 | 9.5E2 | 4.8E2 | 9.4E2 | 4.0E2 | 6.4E2 | 6.6E1 | 2.2E2 | 2.6E3 | 3.1E3 | 48 | 25 | 44 | 25 | 0.74 |
| iH | ng/ml | 1.5E5 | 1.8E5 | 1.5E5 | 1.8E5 | 4.3E4 | 4.3E4 | 7.1E4 | 6.7E4 | 2.4E5 | 2.4E5 | 100 | 43 | 86 | 43 | 0.69 |
| iJ | ng/ml | 5.1E4 | 4.3E4 | 5.3E4 | 5.1E4 | 2.0E4 | 3.8E4 | 8.7E3 | 7.7E3 | 1.2E5 | 2.5E5 | 100 | 43 | 86 | 43 | 0.42 |
| hB | ng/ml | 4.3E-1 | 6.2E-1 | 5.2E-1 | 7.5E-1 | 3.5E-1 | 5.7E-1 | 1.2E-1 | 1.4E-1 | 1.9E0 | 2.4E0 | 100 | 43 | 86 | 43 | 0.64 |
| hC | pg/ml | 3.6E3 | 5.7E3 | 5.9E3 | 9.5E3 | 7.5E3 | 1.2E4 | 1.0E-9 | 2.3E2 | 5.5E4 | 5.7E4 | 100 | 43 | 86 | 43 | 0.61 |
| hF | ng/ml | 1.0E-9 | 1.0E-9 | 4.9E1 | 1.0E-9 | 4.1E2 | 0.0E0 | 1.0E-9 | 1.0E-9 | 4.0E3 | 1.0E-9 | 100 | 43 | 86 | 43 | 0.49 |
| hG | pg/ml | 7.4E3 | 7.6E3 | 7.8E3 | 8.5E3 | 2.9E3 | 3.8E3 | 1.7E3 | 3.3E3 | 1.8E4 | 2.0E4 | 100 | 43 | 86 | 43 | 0.54 |
| iO | ng/ml | 4.1E5 | 3.7E5 | 4.2E5 | 4.0E5 | 2.0E5 | 1.9E5 | 1.1E5 | 8.3E4 | 1.1E6 | 9.2E5 | 100 | 43 | 86 | 43 | 0.47 |
| iP | ng/ml | 6.1E4 | 4.1E4 | 5.5E4 | 7.0E4 | 3.1E4 | 1.1E5 | 5.8E3 | 3.8E3 | 2.5E5 | 5.7E5 | 100 | 43 | 86 | 43 | 0.44 |
| iZ | ng/ml | 1.7E3 | 1.7E3 | 1.8E3 | 2.0E3 | 6.8E2 | 9.3E2 | 4.7E2 | 7.5E2 | 3.5E3 | 4.6E3 | 100 | 43 | 86 | 43 | 0.55 |
| yH | pg/ml | 1.2E3 | 1.4E3 | 2.0E3 | 3.9E3 | 3.0E3 | 6.5E3 | 1.0E-9 | 2.4E2 | 1.5E4 | 2.5E4 | 48 | 25 | 44 | 25 | 0.61 |
| yK | U/ml | 1.8E1 | 2.8E1 | 4.9E1 | 5.1E1 | 8.5E1 | 6.3E1 | 1.0E-9 | 1.0E-9 | 3.8E2 | 3.0E2 | 48 | 25 | 44 | 25 | 0.62 |
| yJ | pg/ml | 3.6E4 | 3.6E4 | 4.7E4 | 4.4E4 | 3.5E4 | 3.4E4 | 1.7E3 | 2.7E3 | 1.6E5 | 1.3E5 | 48 | 25 | 44 | 25 | 0.47 |
| yD | ng/ml | 1.5E-2 | 1.8E-2 | 1.5E-2 | 1.7E-2 | 5.8E-3 | 7.9E-3 | 1.0E-9 | 1.0E-9 | 2.9E-2 | 3.0E-2 | 48 | 25 | 44 | 25 | 0.59 |
| jB | ng/ml | 2.6E5 | 1.8E5 | 2.7E5 | 1.8E5 | 8.8E4 | 4.6E4 | 5.7E4 | 9.9E4 | 4.1E5 | 2.3E5 | 28 | 10 | 26 | 10 | 0.18 |
| wB | pg/ml | 8.8E3 | 1.1E4 | 9.8E3 | 1.3E4 | 7.3E3 | 1.0E4 | 1.7E3 | 3.1E3 | 4.1E4 | 4.2E4 | 48 | 25 | 44 | 25 | 0.59 |
| pY | pg/ml | 6.0E0 | 6.6E0 | 1.1E1 | 7.7E0 | 2.7E1 | 3.7E0 | 2.1E0 | 3.5E0 | 2.0E2 | 1.8E1 | 51 | 23 | 49 | 23 | 0.57 |
| sI | ng/ml | 5.4E-2 | 5.6E-2 | 6.4E-2 | 6.5E-2 | 4.6E-2 | 3.4E-2 | 1.6E-2 | 2.5E-2 | 2.5E-1 | 1.5E-1 | 25 | 14 | 25 | 14 | 0.54 |
| sF | mIU/mL | 4.8E0 | 7.1E0 | 1.2E1 | 1.2E1 | 1.9E1 | 1.4E1 | 1.4E-1 | 9.3E-2 | 7.5E1 | 4.5E1 | 25 | 14 | 25 | 14 | 0.56 |
| sH | mIU/mL | 2.3E0 | 3.8E0 | 4.7E0 | 4.1E0 | 7.2E0 | 4.4E0 | 7.9E-2 | 1.0E-9 | 3.2E1 | 1.7E1 | 25 | 14 | 25 | 14 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| sJ | ng/ml | 1.5E-1 | 1.5E-1 | 8.5E-1 | 1.7E-1 | 1.8E0 | 1.6E-1 | 1.0E-9 | 1.0E-9 | 6.4E0 | 6.1E-1 | 25 | 14 | 25 | 14 | 0.45 |
| rC | pg/ml | 1.9E3 | 1.2E3 | 2.6E3 | 1.6E3 | 2.8E3 | 1.5E3 | 1.0E2 | 1.9E2 | 1.5E4 | 7.3E3 | 72 | 39 | 59 | 39 | 0.38 |
| rB | pg/ml | 2.2E1 | 2.6E1 | 5.5E1 | 4.5E1 | 1.4E2 | 5.1E1 | 1.0E-9 | 1.0E-9 | 9.5E2 | 2.1E2 | 72 | 39 | 59 | 39 | 0.56 |
| zG | 2.5ng/ml | 2.0E-1 | 2.2E-1 | 4.5E-1 | 4.7E-1 | 7.8E-1 | 7.5E-1 | 1.0E-9 | 1.0E-9 | 4.4E0 | 2.7E0 | 48 | 25 | 44 | 25 | 0.49 |
| zH | 2.3mU/ml | 1.1E-1 | 8.8E-2 | 1.2E-1 | 9.1E-2 | 6.9E-2 | 3.5E-2 | 1.0E-2 | 1.9E-2 | 4.4E-1 | 1.7E-1 | 48 | 25 | 44 | 25 | 0.37 |
| zI | 2.6ng/ml | 1.8E0 | 2.5E0 | 3.2E0 | 5.6E0 | 3.3E0 | 7.2E0 | 6.1E-1 | 4.3E-1 | 1.5E1 | 2.7E1 | 48 | 25 | 44 | 25 | 0.60 |
| qA | ng/ml | 1.0E7 | 9.4E6 | 1.1E7 | 1.4E7 | 7.3E6 | 1.0E7 | 3.7E6 | 3.4E6 | 3.7E7 | 4.6E7 | 51 | 23 | 49 | 23 | 0.57 |
| qB | ng/ml | 6.3E5 | 5.4E5 | 8.3E5 | 8.9E5 | 5.8E5 | 8.2E5 | 2.1E5 | 1.9E5 | 2.9E6 | 3.8E6 | 51 | 23 | 49 | 23 | 0.48 |
| qC | ng/ml | 4.8E5 | 3.4E5 | 8.2E5 | 5.8E5 | 1.2E6 | 9.5E5 | 2.0E4 | 2.1E4 | 7.1E6 | 4.7E6 | 51 | 23 | 49 | 23 | 0.42 |
| qD | ng/ml | 1.6E7 | 1.5E7 | 1.9E7 | 1.7E7 | 9.4E6 | 6.9E6 | 4.9E6 | 4.9E6 | 5.2E7 | 3.4E7 | 51 | 23 | 49 | 23 | 0.44 |
| jD | ng/ml | 2.2E1 | 3.2E1 | 4.4E1 | 5.7E1 | 7.2E1 | 5.0E1 | 1.0E-9 | 1.0E-9 | 5.1E2 | 1.8E2 | 77 | 43 | 64 | 43 | 0.64 |
| jE | ng/ml | 1.0E-9 | 1.0E-9 | 8.0E0 | 6.5E0 | 2.1E1 | 1.2E1 | 1.0E-9 | 1.0E-9 | 1.2E2 | 3.9E1 | 77 | 43 | 64 | 43 | 0.52 |
| jF | ng/ml | 3.5E1 | 2.8E1 | 5.0E1 | 5.0E1 | 5.8E1 | 6.7E1 | 1.0E-9 | 1.0E-9 | 2.5E2 | 3.5E2 | 77 | 43 | 64 | 43 | 0.48 |
| jG | ng/ml | 4.5E3 | 4.2E3 | 4.6E3 | 4.5E3 | 1.9E3 | 1.9E3 | 7.6E2 | 6.7E2 | 9.5E3 | 8.7E3 | 77 | 43 | 64 | 43 | 0.48 |
| jH | ng/ml | 7.9E1 | 7.9E1 | 8.6E1 | 9.6E1 | 4.5E1 | 6.8E1 | 1.9E1 | 1.5E1 | 2.3E2 | 4.3E2 | 77 | 43 | 64 | 43 | 0.53 |
| jI | ng/ml | 6.9E1 | 8.2E1 | 7.4E1 | 9.2E1 | 3.4E1 | 6.5E1 | 2.8E1 | 3.6E1 | 2.5E2 | 4.4E2 | 77 | 43 | 64 | 43 | 0.60 |
| sK | pg/mL | 4.2E3 | 3.4E3 | 4.3E3 | 4.5E3 | 1.6E3 | 4.3E3 | 1.7E3 | 2.1E3 | 9.2E3 | 2.3E4 | 49 | 24 | 45 | 24 | 0.41 |
| sM | pg/mL | 8.0E4 | 8.4E4 | 7.9E4 | 9.1E4 | 2.4E4 | 4.1E4 | 3.3E4 | 4.5E4 | 1.5E5 | 2.0E5 | 49 | 24 | 45 | 24 | 0.57 |
| sO | pg/mL | 2.8E8 | 2.4E8 | 2.9E8 | 2.3E8 | 9.9E7 | 8.9E7 | 7.9E7 | 6.6E7 | 4.9E8 | 4.4E8 | 49 | 24 | 45 | 24 | 0.35 |
| wC | ng/ml | 1.6E0 | 1.4E0 | 2.1E0 | 1.7E0 | 2.2E0 | 1.1E0 | 2.5E-1 | 6.1E-2 | 1.5E1 | 4.8E0 | 48 | 25 | 44 | 25 | 0.46 |
| wD | ng/ml | 2.0E1 | 2.3E1 | 8.1E1 | 5.1E1 | 3.1E2 | 6.5E1 | 2.8E0 | 5.0E0 | 2.1E3 | 2.9E2 | 48 | 25 | 44 | 25 | 0.65 |
| wE | ng/ml | 5.0E1 | 5.0E1 | 5.4E1 | 4.7E1 | 2.4E1 | 1.8E1 | 7.0E0 | 3.2E0 | 1.4E2 | 8.4E1 | 48 | 25 | 44 | 25 | 0.42 |
| wG | ng/ml | 9.6E-2 | 3.8E-2 | 1.3E-1 | 7.6E-2 | 1.3E-1 | 1.4E-1 | 1.0E-9 | 1.0E-9 | 4.8E-1 | 6.8E-1 | 48 | 25 | 44 | 25 | 0.37 |
| wH | ng/ml | 2.3E-2 | 2.6E-2 | 2.1E-1 | 2.1E-1 | 5.5E-1 | 4.4E-1 | 1.0E-9 | 1.0E-9 | 2.9E0 | 1.9E0 | 48 | 25 | 44 | 25 | 0.50 |
| wF | ng/ml | 2.1E-1 | 1.3E-1 | 2.9E0 | 1.2E0 | 1.0E1 | 2.3E0 | 1.0E-9 | 1.0E-9 | 5.7E1 | 9.6E0 | 48 | 25 | 44 | 25 | 0.49 |
| rA | pg/ml | 2.4E1 | 2.6E1 | 2.7E1 | 3.1E1 | 2.2E1 | 2.1E1 | 1.0E-9 | 1.0E-9 | 1.2E2 | 9.3E1 | 74 | 43 | 61 | 43 | 0.56 |
| qZ | pg/ml | 4.3E1 | 5.3E1 | 2.1E2 | 1.1E3 | 1.3E3 | 2.9E3 | 2.8E-4 | 3.2E-4 | 1.0E4 | 1.0E4 | 63 | 33 | 54 | 33 | 0.55 |
| qY | pg/ml | 2.1E1 | 2.6E1 | 4.6E1 | 4.3E1 | 7.5E1 | 4.3E1 | 8.7E-1 | 2.1E0 | 5.3E2 | 1.6E2 | 74 | 43 | 61 | 43 | 0.56 |
| qX | pg/ml | 5.1E1 | 6.8E1 | 5.9E1 | 7.7E1 | 3.9E1 | 5.6E1 | 1.0E-9 | 1.0E-9 | 1.8E2 | 2.1E2 | 74 | 43 | 61 | 43 | 0.56 |
| qW | pg/ml | 9.4E0 | 7.6E0 | 1.2E1 | 9.8E0 | 1.3E1 | 9.4E0 | 1.0E-9 | 1.0E-9 | 7.1E1 | 3.6E1 | 74 | 43 | 61 | 43 | 0.44 |
| qV | pg/ml | 2.1E3 | 2.1E3 | 2.7E3 | 2.7E3 | 1.9E3 | 1.8E3 | 2.3E2 | 4.3E2 | 8.5E3 | 6.9E3 | 74 | 43 | 61 | 43 | 0.49 |
| qU | pg/ml | 4.5E1 | 8.3E1 | 1.3E2 | 1.6E2 | 2.2E2 | 2.3E2 | 1.0E-9 | 1.0E-9 | 1.4E3 | 1.1E3 | 74 | 43 | 61 | 43 | 0.58 |
| qT | pg/ml | 3.8E1 | 4.1E1 | 6.4E1 | 6.7E1 | 1.2E2 | 6.4E1 | 1.0E-9 | 1.0E-9 | 9.0E2 | 3.0E2 | 74 | 43 | 61 | 43 | 0.57 |
| qI | ng/ml | 5.8E4 | 6.4E4 | 6.4E4 | 6.5E4 | 3.2E4 | 2.9E4 | 5.4E3 | 2.5E4 | 1.6E5 | 1.6E5 | 48 | 23 | 46 | 23 | 0.53 |
| qH | ng/ml | 6.5E4 | 5.5E4 | 7.3E4 | 5.9E4 | 3.9E4 | 3.2E4 | 1.0E4 | 1.1E4 | 1.8E5 | 1.6E5 | 48 | 23 | 46 | 23 | 0.40 |
| qG | ng/ml | 1.8E5 | 1.9E5 | 1.9E5 | 2.0E5 | 6.2E4 | 5.9E4 | 3.1E4 | 1.0E5 | 3.3E5 | 3.2E5 | 48 | 23 | 46 | 23 | 0.51 |
| jK | ng/ml | 1.6E3 | 1.4E3 | 1.7E3 | 1.6E3 | 5.7E2 | 5.7E2 | 5.5E2 | 7.5E2 | 4.1E3 | 3.1E3 | 77 | 43 | 64 | 43 | 0.44 |
| jL | ng/ml | 1.8E2 | 2.1E2 | 2.6E2 | 3.4E2 | 1.9E2 | 3.3E2 | 3.6E1 | 6.4E1 | 7.9E2 | 1.7E3 | 77 | 43 | 64 | 43 | 0.57 |
| jM | ng/ml | 7.1E4 | 7.5E4 | 7.0E4 | 7.7E4 | 3.4E4 | 3.8E4 | 3.9E2 | 1.1E4 | 1.5E5 | 1.7E5 | 77 | 43 | 64 | 43 | 0.55 |
| jO | pg/ml | 2.2E5 | 2.5E5 | 2.8E5 | 2.6E5 | 1.5E5 | 1.3E5 | 5.2E4 | 9.6E4 | 7.7E5 | 5.9E5 | 77 | 43 | 64 | 43 | 0.49 |
| jP | pg/ml | 2.3E5 | 3.1E5 | 2.6E5 | 3.0E5 | 1.5E5 | 1.6E5 | 7.0E4 | 5.8E4 | 9.1E5 | 7.0E5 | 77 | 43 | 64 | 43 | 0.58 |
| jQ | pg/ml | 2.5E3 | 2.4E3 | 3.0E3 | 3.6E3 | 2.3E3 | 4.4E3 | 4.2E1 | 1.0E-9 | 1.2E4 | 1.8E4 | 77 | 43 | 64 | 43 | 0.48 |
| jR | pg/ml | 6.5E3 | 4.7E3 | 9.5E3 | 1.1E4 | 9.9E3 | 1.6E4 | 1.0E-9 | 1.0E-9 | 5.5E4 | 9.0E4 | 77 | 43 | 64 | 43 | 0.46 |
| jT | pg/ml | 1.6E5 | 1.8E5 | 1.7E5 | 1.8E5 | 6.9E4 | 5.9E4 | 6.8E4 | 8.8E4 | 4.5E5 | 3.5E5 | 77 | 43 | 64 | 43 | 0.53 |
| xA | pg/ml | 3.9E0 | 5.7E0 | 1.7E1 | 1.2E1 | 5.7E1 | 2.3E1 | 1.0E-9 | 1.0E-9 | 3.9E2 | 1.2E2 | 48 | 25 | 44 | 25 | 0.54 |
| yE | pg/ml | 7.8E1 | 7.9E1 | 8.2E1 | 8.9E1 | 4.9E1 | 4.6E1 | 6.4E0 | 1.4E1 | 3.0E2 | 2.5E2 | 48 | 25 | 44 | 25 | 0.55 |
| tM | pg/ml | 4.3E1 | 3.9E1 | 4.3E1 | 3.9E1 | 2.0E1 | 2.3E1 | 1.0E-9 | 1.0E-9 | 1.0E2 | 1.1E2 | 48 | 25 | 44 | 25 | 0.44 |
| tL | pg/ml | 1.0E-9 | 1.0E-9 | 7.8E0 | 1.5E-1 | 3.8E1 | 3.9E-1 | 1.0E-9 | 1.0E-9 | 2.6E2 | 1.8E0 | 48 | 25 | 44 | 25 | 0.46 |
| jU | mIU/ml | 3.8E0 | 5.1E0 | 1.0E1 | 8.0E0 | 1.7E1 | 9.1E0 | 8.9E-2 | 4.2E-2 | 8.1E1 | 5.3E1 | 77 | 43 | 64 | 43 | 0.56 |
| jV | mIU/ml | 1.6E0 | 1.3E0 | 3.5E0 | 2.7E0 | 5.4E0 | 3.1E0 | 2.1E-2 | 1.7E-3 | 3.1E1 | 1.4E1 | 77 | 43 | 64 | 43 | 0.47 |
| jY | ng/ml | 9.7E-4 | 7.4E-4 | 1.1E-2 | 3.7E-3 | 4.2E-2 | 6.4E-3 | 1.0E-9 | 1.0E-9 | 3.0E-1 | 2.6E-2 | 77 | 43 | 64 | 43 | 0.46 |
| kC | pg/ml | 9.7E1 | 1.1E2 | 2.4E2 | 1.4E2 | 5.6E2 | 1.1E2 | 2.1E1 | 2.1E1 | 3.5E3 | 5.9E2 | 54 | 30 | 47 | 30 | 0.53 |
| kE | pg/ml | 1.3E5 | 1.4E5 | 1.3E5 | 1.5E5 | 3.3E4 | 4.9E4 | 4.1E4 | 1.2E4 | 2.0E5 | 2.7E5 | 54 | 30 | 47 | 30 | 0.60 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| kF | pg/mL | 6.6E1 | 6.6E1 | 8.0E1 | 6.8E1 | 7.0E1 | 2.0E1 | 3.2E1 | 2.6E1 | 5.1E2 | 1.2E2 | 54 | 30 | 47 | 30 | 0.48 |
| kG | pg/mL | 9.1E3 | 9.2E3 | 1.2E4 | 1.2E4 | 1.4E4 | 1.1E4 | 7.5E2 | 1.1E3 | 9.1E4 | 5.8E4 | 54 | 30 | 47 | 30 | 0.52 |
| kI | pg/ml | 2.2E2 | 1.8E2 | 2.4E2 | 2.1E2 | 1.4E2 | 1.2E2 | 5.4E1 | 1.0E-9 | 8.7E2 | 6.2E2 | 54 | 30 | 47 | 30 | 0.40 |
| kK | pg/ml | 1.2E2 | 1.4E2 | 1.7E2 | 1.7E2 | 1.8E2 | 1.7E2 | 2.2E1 | 2.1E1 | 1.2E3 | 9.1E2 | 54 | 30 | 47 | 30 | 0.51 |
| kN | pg/ml | 1.0E3 | 9.4E2 | 1.5E3 | 2.2E3 | 1.9E3 | 3.5E3 | 2.1E2 | 3.0E2 | 1.3E4 | 1.7E4 | 54 | 30 | 47 | 30 | 0.55 |
| kO | pg/ml | 7.7E3 | 7.7E3 | 1.1E4 | 8.3E3 | 1.8E4 | 3.7E3 | 4.0E3 | 4.2E3 | 1.3E5 | 2.5E4 | 54 | 30 | 47 | 30 | 0.49 |
| kP | pg/ml | 5.4E3 | 6.6E3 | 6.7E3 | 7.1E3 | 5.5E3 | 4.4E3 | 8.6E2 | 9.6E2 | 3.3E4 | 1.7E4 | 54 | 30 | 47 | 30 | 0.56 |
| kQ | pg/ml | 4.3E3 | 5.0E3 | 4.9E3 | 6.3E3 | 2.3E3 | 4.9E3 | 5.6E2 | 2.0E3 | 1.2E4 | 2.5E4 | 100 | 43 | 86 | 43 | 0.57 |
| kR | pg/ml | 2.4E1 | 2.1E1 | 4.0E1 | 2.8E1 | 1.0E2 | 2.4E1 | 1.0E-9 | 4.8E0 | 1.0E3 | 1.1E2 | 100 | 43 | 86 | 43 | 0.46 |
| kS | pg/ml | 7.7E2 | 9.3E2 | 9.9E2 | 9.9E2 | 1.4E3 | 5.5E2 | 8.2E1 | 2.6E2 | 1.4E4 | 2.5E3 | 100 | 43 | 86 | 43 | 0.57 |
| pS | ng/ml | 2.0E5 | 1.3E5 | 2.2E5 | 1.9E5 | 9.5E4 | 1.6E5 | 7.5E4 | 6.0E4 | 5.0E5 | 8.3E5 | 49 | 24 | 45 | 24 | 0.31 |
| rZ | ng/ml | 1.2E-3 | 2.0E-3 | 7.9E-3 | 1.0E-2 | 2.1E-2 | 2.1E-2 | 1.0E-9 | 1.0E-9 | 1.1E-1 | 1.1E-1 | 73 | 40 | 58 | 40 | 0.58 |
| rY | ng/ml | 5.4E-2 | 6.5E-2 | 2.5E-1 | 5.7E-1 | 8.3E-1 | 3.2E0 | 1.0E-9 | 1.0E-9 | 6.3E0 | 2.0E1 | 73 | 40 | 58 | 40 | 0.53 |
| rX | ng/ml | 1.0E-9 | 1.0E-9 | 9.2E-2 | 8.5E-2 | 4.5E-1 | 4.9E-1 | 1.0E-9 | 1.0E-9 | 3.7E0 | 3.1E0 | 73 | 40 | 58 | 40 | 0.46 |
| lK | pg/ml | 7.1E1 | 7.1E1 | 1.4E2 | 1.5E2 | 1.8E2 | 1.9E2 | 1.0E-9 | 1.0E-9 | 7.0E2 | 7.2E2 | 77 | 42 | 64 | 42 | 0.49 |
| lL | pg/ml | 1.8E3 | 1.4E3 | 3.4E3 | 2.1E3 | 5.5E3 | 2.0E3 | 7.5E1 | 8.9E1 | 4.2E4 | 7.7E3 | 77 | 43 | 64 | 43 | 0.40 |
| lM | pg/ml | 1.0E3 | 1.3E3 | 3.3E3 | 5.1E3 | 7.5E3 | 9.1E3 | 3.9E1 | 9.5E0 | 5.1E4 | 4.0E4 | 77 | 43 | 64 | 43 | 0.54 |
| lN | pg/ml | 1.0E-9 | 1.0E-9 | 7.3E0 | 3.2E0 | 2.2E1 | 6.3E0 | 1.0E-9 | 1.0E-9 | 1.5E2 | 2.9E1 | 77 | 43 | 64 | 43 | 0.46 |
| lO | pg/ml | 1.0E-9 | 1.0E-9 | 5.2E-1 | 3.5E0 | 4.6E0 | 2.1E1 | 1.0E-9 | 1.0E-9 | 4.0E1 | 1.4E2 | 77 | 42 | 64 | 42 | 0.52 |
| zA | ng/ml | 1.9E7 | 2.1E7 | 2.0E7 | 1.9E7 | 6.8E6 | 6.0E6 | 6.7E6 | 5.9E6 | 3.6E7 | 2.8E7 | 44 | 25 | 40 | 25 | 0.51 |
| rW | ng/ml | 1.3E-2 | 2.2E-2 | 2.4E-2 | 4.2E-2 | 3.1E-2 | 5.4E-2 | 1.0E-9 | 1.0E-9 | 1.7E-1 | 1.9E-1 | 49 | 23 | 46 | 23 | 0.61 |
| rV | ng/ml | 1.0E-9 | 1.0E-9 | 9.2E-3 | 2.0E-2 | 3.7E-2 | 5.7E-2 | 1.0E-9 | 1.0E-9 | 2.2E-1 | 2.4E-1 | 49 | 23 | 46 | 23 | 0.55 |
| rU | ng/ml | 9.5E-2 | 8.1E-2 | 1.4E-1 | 1.9E-1 | 2.3E-1 | 3.9E-1 | 1.0E-9 | 1.0E-9 | 1.4E0 | 1.9E0 | 49 | 23 | 46 | 23 | 0.57 |
| rT | ng/ml | 6.7E0 | 5.3E0 | 6.8E0 | 6.8E0 | 4.1E0 | 5.0E0 | 7.5E-1 | 1.0E0 | 2.1E1 | 2.0E1 | 49 | 23 | 46 | 23 | 0.47 |
| rS | ng/ml | 3.4E0 | 5.8E0 | 5.6E0 | 1.2E1 | 6.8E0 | 1.8E1 | 1.0E0 | 1.0E0 | 3.8E1 | 7.0E1 | 49 | 23 | 46 | 23 | 0.61 |
| sC | pg/mL | 5.6E3 | 8.3E3 | 8.5E3 | 2.0E4 | 7.1E3 | 2.3E4 | 1.7E3 | 2.2E3 | 3.2E4 | 8.0E4 | 49 | 24 | 45 | 24 | 0.66 |
| yL | pg/ml | 3.4E1 | 2.7E1 | 4.2E1 | 8.3E1 | 2.8E1 | 2.8E2 | 5.6E0 | 1.2E1 | 1.8E2 | 1.4E3 | 47 | 25 | 43 | 25 | 0.32 |
| rP | ng/ml | 7.7E1 | 8.7E1 | 1.7E2 | 2.4E2 | 2.3E2 | 2.6E2 | 1.0E-9 | 1.3E0 | 1.2E3 | 8.0E2 | 49 | 23 | 46 | 23 | 0.55 |
| rQ | ng/ml | 1.0E-9 | 1.0E-9 | 3.4E0 | 5.4E0 | 1.5E1 | 2.6E1 | 1.0E-9 | 1.0E-9 | 8.9E1 | 1.2E2 | 49 | 23 | 46 | 23 | 0.50 |
| rO | ng/ml | 2.5E-2 | 3.5E-2 | 3.9E-2 | 5.6E-2 | 7.1E-2 | 7.1E-2 | 1.0E-9 | 1.0E-9 | 4.4E-1 | 3.3E-1 | 49 | 23 | 46 | 23 | 0.61 |
| rR | ng/ml | 3.9E0 | 3.9E0 | 2.2E1 | 1.4E1 | 6.6E1 | 2.5E1 | 1.0E-9 | 1.0E-9 | 4.5E2 | 9.5E1 | 49 | 23 | 46 | 23 | 0.50 |
| rN | ng/ml | 6.4E-1 | 6.8E-1 | 7.0E-1 | 1.1E0 | 3.8E-1 | 1.0E0 | 5.1E-2 | 2.4E-1 | 2.1E0 | 4.4E0 | 49 | 23 | 46 | 23 | 0.57 |
| qO | pg/ml | 9.7E3 | 1.2E4 | 1.2E4 | 1.8E4 | 8.2E3 | 1.5E4 | 2.2E3 | 1.9E3 | 3.9E4 | 6.4E4 | 50 | 23 | 47 | 23 | 0.62 |
| qP | pg/ml | 3.6E2 | 3.6E2 | 3.9E2 | 5.6E2 | 2.5E2 | 4.6E2 | 1.0E-9 | 1.2E2 | 1.1E3 | 2.2E3 | 50 | 23 | 47 | 23 | 0.59 |
| qQ | pg/ml | 3.1E0 | 6.3E0 | 1.5E1 | 1.0E1 | 4.0E1 | 1.2E1 | 1.0E-9 | 1.0E-9 | 2.8E2 | 4.3E1 | 50 | 23 | 47 | 23 | 0.51 |
| nW | pg/ml | 1.1E5 | 1.2E5 | 1.1E5 | 1.2E5 | 2.5E4 | 2.9E4 | 5.8E4 | 6.7E4 | 1.8E5 | 1.7E5 | 100 | 43 | 86 | 43 | 0.56 |
| nY | pg/ml | 1.9E3 | 2.2E3 | 2.3E3 | 2.5E3 | 1.4E3 | 1.3E3 | 6.5E2 | 5.7E2 | 9.9E3 | 5.5E3 | 100 | 43 | 86 | 43 | 0.57 |
| oO | pg/ml | 8.2E4 | 1.0E5 | 1.2E5 | 1.2E5 | 1.1E5 | 8.0E4 | 4.0E4 | 3.3E3 | 6.2E5 | 3.0E5 | 52 | 26 | 45 | 26 | 0.57 |
| oP | pg/ml | 1.3E5 | 1.6E5 | 1.4E5 | 1.7E5 | 8.4E4 | 1.1E5 | 4.8E4 | 2.4E4 | 3.5E5 | 4.2E5 | 52 | 26 | 45 | 26 | 0.58 |
| oQ | pg/ml | 3.0E3 | 3.5E3 | 3.2E3 | 4.9E3 | 1.6E3 | 4.2E3 | 1.1E3 | 9.1E2 | 1.0E4 | 2.1E4 | 52 | 26 | 45 | 26 | 0.61 |
| oE | pg/ml | 2.1E2 | 2.7E2 | 4.6E2 | 5.7E2 | 6.4E2 | 7.0E2 | 1.0E-9 | 1.0E-9 | 4.7E3 | 3.4E3 | 100 | 43 | 86 | 43 | 0.55 |
| oF | pg/ml | 8.6E3 | 1.5E4 | 2.0E4 | 2.9E4 | 3.3E4 | 3.5E4 | 6.4E1 | 1.0E3 | 2.3E5 | 1.4E5 | 100 | 43 | 86 | 43 | 0.61 |
| oH | pg/ml | 4.2E1 | 4.1E1 | 9.3E1 | 7.3E1 | 1.5E2 | 8.3E1 | 4.2E0 | 4.4E0 | 8.6E2 | 3.2E2 | 100 | 43 | 86 | 43 | 0.48 |
| oK | pg/ml | 6.2E2 | 1.2E3 | 1.9E3 | 1.7E3 | 3.2E3 | 1.6E3 | 5.2E1 | 1.9E2 | 1.8E4 | 6.8E3 | 100 | 43 | 86 | 43 | 0.59 |
| oN | pg/ml | 4.7E2 | 6.3E2 | 8.0E2 | 7.8E2 | 1.9E3 | 7.1E2 | 1.5E2 | 2.5E2 | 1.8E4 | 4.6E3 | 100 | 43 | 86 | 43 | 0.64 |
| oW | pg/ml | 2.0E2 | 3.6E2 | 4.7E2 | 4.7E2 | 1.1E3 | 5.1E2 | 7.7E1 | 9.0E1 | 6.0E3 | 1.8E3 | 28 | 10 | 26 | 10 | 0.60 |
| oT | pg/ml | 3.1E2 | 2.7E2 | 3.5E2 | 3.4E2 | 1.8E2 | 1.9E2 | 9.9E1 | 1.3E2 | 7.8E2 | 7.9E2 | 28 | 10 | 26 | 10 | 0.47 |
| oV | pg/ml | 1.4E2 | 1.1E2 | 2.4E2 | 3.1E2 | 3.2E2 | 6.8E2 | 1.0E-9 | 1.1E1 | 1.4E3 | 2.2E3 | 28 | 10 | 26 | 10 | 0.50 |
| oD | pg/ml | 1.6E4 | 1.3E4 | 1.8E4 | 1.4E4 | 8.1E3 | 5.3E3 | 8.7E3 | 6.6E3 | 4.6E4 | 2.5E4 | 28 | 10 | 26 | 10 | 0.30 |
| uL | ng/ml | 3.8E1 | 3.3E1 | 4.1E1 | 5.3E1 | 2.4E1 | 7.1E1 | 1.0E-9 | 1.1E1 | 1.6E2 | 3.7E2 | 48 | 24 | 44 | 24 | 0.47 |
| uO | ng/ml | 3.5E-1 | 4.2E-1 | 8.5E-1 | 7.6E-1 | 1.5E0 | 1.1E0 | 1.0E-9 | 1.0E-9 | 9.3E0 | 5.0E0 | 48 | 24 | 44 | 24 | 0.50 |
| uM | ng/ml | 6.3E-1 | 6.6E-1 | 1.2E0 | 7.6E-1 | 2.3E0 | 5.5E-1 | 1.0E-9 | 1.0E-9 | 1.3E1 | 2.5E0 | 48 | 24 | 44 | 24 | 0.50 |
| uI | ng/ml | 6.5E-2 | 8.9E-2 | 1.3E-1 | 1.1E-1 | 1.9E-1 | 8.3E-2 | 1.6E-2 | 1.5E-2 | 1.1E0 | 3.8E-1 | 48 | 24 | 44 | 24 | 0.53 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| uN | ng/ml | 1.5E1 | 1.5E1 | 1.6E1 | 1.7E1 | 6.5E0 | 8.5E0 | 7.8E0 | 6.4E0 | 4.1E1 | 4.1E1 | 48 | 24 | 44 | 24 | 0.46 |
| uG | ng/ml | 2.2E1 | 1.9E1 | 2.5E1 | 2.6E1 | 1.3E1 | 2.7E1 | 9.8E0 | 6.7E0 | 6.9E1 | 1.3E2 | 48 | 24 | 44 | 24 | 0.42 |
| uR | ng/ml | 2.3E0 | 2.7E0 | 4.2E0 | 3.3E0 | 9.1E0 | 3.0E0 | 9.9E-1 | 7.3E-1 | 6.4E1 | 1.4E1 | 48 | 25 | 44 | 25 | 0.51 |
| uP | ng/ml | 1.8E0 | 2.4E0 | 2.2E0 | 2.9E0 | 1.3E0 | 1.3E0 | 1.1E0 | 1.3E0 | 9.1E0 | 6.1E0 | 48 | 25 | 44 | 25 | 0.72 |
| uV | ng/ml | 1.0E-9 | 1.1E-3 | 1.1E-2 | 1.1E-2 | 3.3E-2 | 1.6E-2 | 1.0E-9 | 1.0E-9 | 2.0E-1 | 6.1E-2 | 48 | 25 | 44 | 25 | 0.55 |
| uT | ng/ml | 6.2E1 | 6.4E1 | 8.8E1 | 9.5E1 | 9.6E1 | 8.7E1 | 1.2E1 | 1.3E1 | 5.8E2 | 4.1E2 | 48 | 25 | 44 | 25 | 0.56 |
| uU | ng/ml | 1.6E0 | 1.4E0 | 2.0E0 | 2.5E0 | 1.1E0 | 3.8E0 | 6.0E-1 | 5.9E-1 | 5.4E0 | 2.0E1 | 48 | 25 | 44 | 25 | 0.45 |
| uW | ng/ml | 7.5E0 | 7.1E0 | 7.9E0 | 8.1E0 | 2.8E0 | 2.7E0 | 4.0E0 | 4.4E0 | 2.2E1 | 1.4E1 | 48 | 24 | 44 | 24 | 0.50 |
| vB | ng/ml | 2.7E0 | 3.3E0 | 2.7E0 | 3.2E0 | 1.3E0 | 1.5E0 | 5.9E-1 | 1.2E0 | 5.6E0 | 7.7E0 | 48 | 24 | 44 | 24 | 0.60 |
| vC | ng/ml | 1.0E-9 | 1.0E-9 | 7.5E-3 | 1.0E-9 | 3.5E-2 | 0.0E0 | 1.0E-9 | 1.0E-9 | 2.0E-1 | 1.0E-9 | 48 | 24 | 44 | 24 | 0.47 |
| uY | ng/ml | 8.3E-1 | 7.7E-1 | 1.3E0 | 1.1E0 | 1.2E0 | 1.1E0 | 8.7E-2 | 1.9E-1 | 4.9E0 | 4.4E0 | 48 | 24 | 44 | 24 | 0.45 |
| uZ | ng/ml | 5.9E-1 | 4.2E-1 | 8.8E-1 | 7.5E-1 | 1.1E0 | 1.0E0 | 1.4E-1 | 1.2E-1 | 7.2E0 | 4.9E0 | 48 | 24 | 44 | 24 | 0.39 |
| uX | ng/ml | 1.1E1 | 1.2E1 | 1.2E1 | 1.8E1 | 6.7E0 | 1.8E1 | 3.6E0 | 5.1E0 | 3.3E1 | 7.8E1 | 48 | 24 | 44 | 24 | 0.57 |
| vA | ng/ml | 6.8E-2 | 7.0E-2 | 8.5E-2 | 9.9E-2 | 5.8E-2 | 9.2E-2 | 2.5E-2 | 2.6E-2 | 3.0E-1 | 4.2E-1 | 48 | 24 | 44 | 24 | 0.49 |
| vH | ng/ml | 1.2E-1 | 1.2E-1 | 1.7E-1 | 2.2E-1 | 1.6E-1 | 3.7E-1 | 1.5E-2 | 1.4E-2 | 8.0E-1 | 1.9E0 | 49 | 24 | 45 | 24 | 0.48 |
| vI | ng/ml | 1.8E0 | 2.3E0 | 1.9E0 | 2.6E0 | 1.2E0 | 2.4E0 | 6.2E-3 | 6.3E-3 | 5.1E0 | 1.0E1 | 49 | 24 | 45 | 24 | 0.56 |
| vP | ng/ml | 3.8E2 | 5.6E2 | 4.4E2 | 6.4E2 | 3.2E2 | 4.8E2 | 7.0E1 | 1.3E2 | 1.5E3 | 2.2E3 | 48 | 25 | 44 | 25 | 0.62 |
| vT | ng/ml | 7.7E1 | 9.2E1 | 1.2E2 | 1.0E2 | 1.2E2 | 5.8E1 | 4.1E1 | 4.5E1 | 6.9E2 | 3.3E2 | 48 | 25 | 44 | 25 | 0.54 |
| vU | ng/ml | 1.0E-9 | 1.0E-9 | 2.3E1 | 2.6E1 | 3.2E1 | 3.5E1 | 1.0E-9 | 1.0E-9 | 1.0E2 | 1.4E2 | 48 | 25 | 44 | 25 | 0.52 |
| vQ | ng/ml | 3.4E2 | 3.4E2 | 3.4E2 | 3.8E2 | 1.3E2 | 1.4E2 | 6.7E1 | 1.9E2 | 7.0E2 | 6.5E2 | 48 | 25 | 44 | 25 | 0.58 |
| vO | ng/ml | 1.7E3 | 1.8E3 | 1.8E3 | 1.9E3 | 4.6E2 | 4.3E2 | 1.0E3 | 1.1E3 | 3.0E3 | 2.7E3 | 48 | 25 | 44 | 25 | 0.58 |
| vS | ng/ml | 1.3E3 | 1.4E3 | 1.3E3 | 1.3E3 | 3.5E2 | 5.0E2 | 1.0E-9 | 1.0E-9 | 1.8E3 | 1.9E3 | 48 | 25 | 44 | 25 | 0.59 |
| vV | ng/ml | 8.4E2 | 1.2E3 | 1.1E3 | 1.7E3 | 9.9E2 | 1.9E3 | 1.1E2 | 2.2E2 | 5.0E3 | 9.0E3 | 48 | 25 | 44 | 25 | 0.61 |
| vW | ng/ml | 1.5E2 | 1.7E2 | 1.7E2 | 2.2E2 | 1.3E2 | 1.6E2 | 4.3E1 | 6.0E1 | 6.7E2 | 7.7E2 | 48 | 25 | 44 | 25 | 0.62 |
| pF | pg/ml | 5.0E-1 | 7.1E-1 | 6.8E-1 | 9.9E-1 | 1.0E0 | 9.4E-1 | 1.0E-9 | 1.0E-9 | 9.4E0 | 4.4E0 | 100 | 43 | 86 | 43 | 0.61 |
| pH | ng/ml | 7.3E0 | 1.2E1 | 8.6E0 | 1.4E1 | 4.0E0 | 1.2E1 | 3.4E0 | 5.9E0 | 1.8E1 | 4.7E1 | 28 | 10 | 26 | 10 | 0.74 |
| pI | ng/ml | 7.1E1 | 6.8E1 | 7.0E1 | 7.6E1 | 3.2E1 | 4.6E1 | 2.6E1 | 2.7E1 | 1.5E2 | 2.0E2 | 28 | 10 | 26 | 10 | 0.48 |
| pK | ng/ml | 4.5E-1 | 5.5E-1 | 5.0E-1 | 5.5E-1 | 2.9E-1 | 1.9E-1 | 2.0E-1 | 2.7E-1 | 1.6E0 | 8.6E-1 | 28 | 10 | 26 | 10 | 0.63 |

Figure 7.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ad | ug/mL | 3.9E-2 | 6.7E-2 | 7.4E-2 | 8.5E-1 | 8.9E-2 | 2.5E0 | 2.7E-4 | 4.3E-3 | 5.4E-1 | 8.5E0 | 534 | 11 | 204 | 11 | 0.60 |
| Af | ng/mL | 1.2E0 | 7.5E-1 | 1.6E1 | 2.8E1 | 6.0E1 | 7.7E1 | 1.7E-3 | 1.7E-3 | 5.3E2 | 2.6E2 | 534 | 11 | 204 | 11 | 0.51 |
| Aj | ug/mL | 1.5E0 | 2.0E-1 | 2.6E0 | 1.8E0 | 2.4E0 | 2.6E0 | 1.5E-3 | 2.1E-3 | 6.1E0 | 5.8E0 | 534 | 11 | 204 | 11 | 0.40 |
| Al | mg/mL | 8.8E-5 | 9.3E-5 | 2.5E-4 | 2.7E-4 | 4.1E-4 | 3.5E-4 | 2.3E-6 | 3.9E-5 | 2.2E-3 | 1.1E-3 | 534 | 11 | 204 | 11 | 0.59 |
| An | U/mL | 5.0E1 | 9.5E1 | 1.7E2 | 9.3E2 | 4.4E2 | 2.3E3 | 9.8E-4 | 4.3E1 | 5.5E3 | 7.8E3 | 534 | 11 | 204 | 11 | 0.73 |
| Ao | pg/mL | 8.8E1 | 1.3E2 | 4.6E2 | 5.8E2 | 3.2E3 | 1.3E3 | 1.5E0 | 5.4E0 | 3.9E4 | 4.5E3 | 534 | 11 | 204 | 11 | 0.54 |
| Ap | ng/mL | 3.3E1 | 2.7E1 | 4.8E1 | 6.0E1 | 5.1E1 | 7.5E1 | 8.4E-5 | 8.4E0 | 3.3E2 | 2.4E2 | 534 | 11 | 204 | 11 | 0.51 |
| Ar | ng/mL | 9.7E-1 | 4.0E0 | 1.1E1 | 4.3E0 | 1.8E2 | 4.0E0 | 3.4E-3 | 2.2E-1 | 4.1E3 | 1.4E1 | 534 | 11 | 204 | 11 | 0.71 |
| As | ng/mL | 8.7E-3 | 1.0E-2 | 1.3E-2 | 1.2E-1 | 1.8E-2 | 3.7E-1 | 1.7E-3 | 1.7E-3 | 1.3E-1 | 1.2E0 | 534 | 11 | 204 | 11 | 0.49 |
| Aw | pg/mL | 1.6E1 | 2.1E1 | 1.7E1 | 2.3E1 | 6.2E0 | 1.1E1 | 2.9E-2 | 1.3E1 | 4.8E1 | 5.1E1 | 534 | 11 | 204 | 11 | 0.71 |
| Ax | ng/mL | 2.1E0 | 7.2E0 | 1.6E1 | 7.0E1 | 6.0E1 | 1.2E2 | 1.2E-2 | 6.1E-1 | 7.7E2 | 3.7E2 | 534 | 11 | 204 | 11 | 0.69 |
| Ba | ng/mL | 6.5E1 | 5.8E2 | 4.5E2 | 3.1E3 | 1.1E3 | 4.6E3 | 2.7E-1 | 1.6E1 | 8.1E3 | 1.5E4 | 534 | 11 | 204 | 11 | 0.77 |
| Bb | ng/mL | 3.1E0 | 2.8E0 | 6.6E0 | 7.1E0 | 1.4E1 | 8.2E0 | 4.1E-3 | 6.8E-1 | 2.5E2 | 2.5E1 | 534 | 11 | 204 | 11 | 0.55 |
| Bc | ng/mL | 3.9E1 | 8.9E1 | 1.1E2 | 2.2E2 | 2.0E2 | 3.0E2 | 1.1E-1 | 9.6E0 | 1.2E3 | 1.0E3 | 534 | 11 | 204 | 11 | 0.66 |
| Bg | ng/mL | 8.5E-2 | 7.3E-1 | 5.4E0 | 3.7E1 | 2.9E1 | 1.2E2 | 5.3E-4 | 1.0E-2 | 4.4E2 | 4.0E2 | 534 | 11 | 204 | 11 | 0.66 |
| Bn | ng/mL | 5.6E-2 | 1.4E-1 | 1.2E0 | 6.3E0 | 2.0E0 | 1.7E1 | 5.6E-2 | 5.6E-2 | 9.7E0 | 5.8E1 | 534 | 11 | 204 | 11 | 0.55 |
| Bo | ng/mL | 1.2E1 | 2.2E1 | 1.4E1 | 2.4E1 | 1.8E1 | 1.7E1 | 1.6E-2 | 1.6E-2 | 2.8E2 | 5.3E1 | 534 | 11 | 204 | 11 | 0.68 |
| Ch | uIU/mL | 1.0E0 | 9.5E-1 | 1.6E1 | 1.1E2 | 9.4E1 | 3.6E2 | 3.4E-3 | 1.8E-1 | 1.8E3 | 1.2E3 | 534 | 11 | 204 | 11 | 0.49 |
| Co | pg/mL | 3.8E1 | 6.6E1 | 1.7E2 | 1.5E2 | 8.9E2 | 2.0E2 | 1.5E-1 | 1.5E-1 | 1.7E4 | 5.6E2 | 534 | 11 | 204 | 11 | 0.59 |
| Cp | ng/mL | 2.2E1 | 5.6E1 | 2.9E1 | 1.5E2 | 3.1E1 | 3.7E2 | 6.0E-1 | 1.4E1 | 3.7E2 | 1.3E3 | 534 | 11 | 204 | 11 | 0.69 |
| Cq | ng/mL | 3.0E-2 | 3.9E-2 | 1.4E-1 | 4.6E0 | 8.1E-1 | 1.5E1 | 8.0E-4 | 8.0E-4 | 1.7E1 | 4.9E1 | 534 | 11 | 204 | 11 | 0.57 |
| Cs | ng/mL | 6.1E1 | 2.2E2 | 3.1E2 | 1.1E3 | 1.1E3 | 1.8E3 | 2.7E-2 | 2.9E1 | 1.8E4 | 5.1E3 | 534 | 11 | 204 | 11 | 0.71 |
| Ct | ng/mL | 5.9E-1 | 1.3E-1 | 3.8E1 | 8.9E1 | 1.1E2 | 1.8E2 | 1.1E-4 | 5.7E-2 | 6.2E2 | 4.7E2 | 534 | 11 | 204 | 11 | 0.53 |
| Cu | ng/mL | 2.5E-1 | 6.4E-1 | 5.1E-1 | 6.9E0 | 1.4E0 | 2.0E1 | 9.0E-5 | 4.6E-2 | 2.1E1 | 6.6E1 | 534 | 11 | 204 | 11 | 0.71 |
| Cv | ng/mL | 5.5E0 | 8.5E0 | 2.5E1 | 1.2E2 | 6.3E1 | 2.0E2 | 1.4E-4 | 1.9E-1 | 5.3E2 | 5.2E2 | 534 | 11 | 204 | 11 | 0.54 |
| Cw | mIU/mL | 3.0E-2 | 5.1E-2 | 3.9E-2 | 6.6E-1 | 3.3E-2 | 2.0E0 | 1.5E-4 | 1.4E-2 | 2.4E-1 | 6.8E0 | 534 | 11 | 204 | 11 | 0.66 |
| Cx | ng/mL | 3.2E-1 | 1.6E-2 | 6.1E1 | 7.6E1 | 1.1E2 | 1.4E2 | 9.3E-5 | 9.3E-5 | 3.9E2 | 3.9E2 | 534 | 11 | 204 | 11 | 0.44 |
| Db | ug/mL | 7.4E0 | 1.3E1 | 9.1E0 | 1.1E1 | 1.0E1 | 5.7E0 | 4.5E-1 | 1.4E0 | 1.4E2 | 1.9E1 | 534 | 11 | 204 | 11 | 0.66 |
| Dc | nmol/L | 1.9E-2 | 2.0E-2 | 6.0E-2 | 1.5E0 | 1.4E-1 | 4.2E0 | 5.2E-6 | 1.3E-3 | 1.6E0 | 1.4E1 | 534 | 11 | 204 | 11 | 0.61 |
| Dd | ug/mL | 7.0E-2 | 8.2E-2 | 1.7E-1 | 4.7E-1 | 2.6E-1 | 1.1E0 | 8.3E-5 | 6.2E-3 | 1.9E0 | 3.6E0 | 534 | 11 | 204 | 11 | 0.53 |
| De | ng/mL | 3.4E-3 | 3.4E-3 | 8.1E-2 | 1.6E-1 | 1.5E-1 | 2.4E-1 | 3.4E-3 | 3.4E-3 | 1.2E0 | 7.9E-1 | 534 | 11 | 204 | 11 | 0.59 |
| Dg | ng/mL | 3.3E1 | 4.4E1 | 4.5E1 | 5.7E1 | 4.0E1 | 5.4E1 | 1.0E-1 | 4.7E0 | 1.9E2 | 1.9E2 | 534 | 11 | 204 | 11 | 0.56 |
| Di | pg/mL | 1.9E0 | 4.2E0 | 2.2E0 | 3.7E0 | 2.0E0 | 2.3E0 | 1.8E-1 | 1.8E-1 | 1.3E1 | 9.0E0 | 534 | 11 | 204 | 11 | 0.72 |
| Dk | uIU/mL | 1.7E-2 | 4.6E-2 | 7.9E-2 | 1.8E-1 | 4.7E-1 | 2.4E-1 | 1.1E-4 | 5.8E-3 | 8.9E0 | 6.3E-1 | 534 | 11 | 204 | 11 | 0.66 |
| Dl | ng/mL | 2.3E2 | 2.3E2 | 3.1E2 | 3.8E2 | 2.9E2 | 4.5E2 | 1.7E0 | 1.8E1 | 1.5E3 | 1.6E3 | 534 | 11 | 204 | 11 | 0.52 |
| Dp | ng/ml | 2.4E0 | 1.0E0 | 5.4E0 | 2.5E1 | 8.0E0 | 6.3E1 | 3.7E-3 | 3.7E-3 | 5.6E1 | 2.0E2 | 332 | 10 | 193 | 10 | 0.40 |
| Dr | pg/ml | 2.5E1 | 2.8E2 | 5.0E1 | 1.5E3 | 7.0E1 | 3.6E3 | 7.5E-1 | 4.6E0 | 5.2E2 | 1.0E4 | 185 | 8 | 107 | 8 | 0.78 |
| Du | pg/ml | 5.2E1 | 1.2E0 | 7.3E2 | 4.8E3 | 2.7E3 | 8.8E3 | 1.2E0 | 1.2E0 | 2.6E4 | 2.4E4 | 111 | 7 | 87 | 7 | 0.54 |
| Ef | ng/ml | 1.3E-1 | 1.3E0 | 8.8E-1 | 3.2E0 | 1.9E0 | 3.6E0 | 5.7E-4 | 1.1E-2 | 1.0E1 | 9.4E0 | 398 | 10 | 200 | 10 | 0.68 |
| Wm | % | 4.9E-1 | 5.6E0 | 3.3E1 | 9.8E1 | 1.8E2 | 2.8E2 | 5.4E-2 | 8.5E-2 | 2.4E3 | 1.0E3 | 432 | 14 | 217 | 14 | 0.66 |
| Ed | pg/ml | 5.2E-1 | 6.8E1 | 5.5E1 | 7.3E1 | 4.0E2 | 4.7E1 | 5.2E-1 | 4.4E0 | 7.3E3 | 1.5E2 | 332 | 10 | 192 | 10 | 0.80 |
| Tj | pg/mL | 3.7E-1 | 3.7E-1 | 4.9E1 | 3.0E1 | 2.7E2 | 5.0E1 | 3.6E-1 | 3.7E-1 | 3.5E3 | 1.4E2 | 396 | 10 | 203 | 10 | 0.54 |
| Po | pg/ml | 6.9E-1 | 2.8E1 | 8.0E0 | 4.6E1 | 2.2E1 | 6.3E1 | 8.0E-3 | 5.3E-1 | 2.7E2 | 2.2E2 | 932 | 16 | 343 | 16 | 0.83 |
| Ti | ug/mL | 3.4E0 | 5.5E0 | 4.8E0 | 7.1E0 | 4.6E0 | 6.2E0 | 8.7E-3 | 8.7E-3 | 3.7E1 | 1.8E1 | 185 | 8 | 137 | 8 | 0.60 |
| Em | ng/ml | 2.9E-3 | 6.0E-2 | 6.7E-2 | 2.6E-1 | 1.4E-1 | 6.3E-1 | 1.9E-16 | 8.4E-4 | 7.6E-1 | 1.9E0 | 236 | 9 | 107 | 9 | 0.62 |
| Et | ng/ml | 1.4E3 | 3.3E3 | 1.7E3 | 3.2E3 | 1.2E3 | 1.4E3 | 7.5E1 | 7.9E2 | 5.0E3 | 5.0E3 | 931 | 16 | 343 | 16 | 0.81 |
| Eq | pg/ml | 1.4E2 | 3.1E1 | 3.1E2 | 2.0E2 | 3.9E2 | 3.6E2 | 1.0E0 | 1.0E0 | 1.8E3 | 1.0E3 | 111 | 7 | 87 | 7 | 0.38 |
| Th | ug/mL | 1.1E0 | 2.2E0 | 1.6E0 | 2.3E0 | 1.5E0 | 1.1E0 | 2.6E-3 | 4.8E-1 | 1.2E1 | 4.2E0 | 185 | 8 | 137 | 8 | 0.72 |
| Fa | ng/ml | 4.4E1 | 2.2E2 | 1.2E2 | 2.4E2 | 5.1E2 | 2.1E2 | 3.4E-2 | 7.5E0 | 8.0E3 | 7.3E2 | 327 | 10 | 190 | 10 | 0.80 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ez | ng/ml | 3.8E0 | 2.2E1 | 1.7E1 | 3.1E1 | 5.0E1 | 3.2E1 | 1.3E-2 | 1.1E-1 | 7.1E2 | 8.8E1 | 332 | 10 | 193 | 10 | 0.71 |
| Fb | ng/ml | 2.5E1 | 2.7E1 | 2.3E1 | 2.9E1 | 1.2E1 | 5.5E0 | 5.9E-1 | 2.4E1 | 5.7E1 | 4.1E1 | 328 | 10 | 190 | 10 | 0.64 |
| Ex | ng/ml | 7.6E-2 | 2.4E-1 | 2.2E-1 | 8.3E-1 | 6.5E-1 | 1.3E0 | 3.5E-5 | 4.2E-2 | 8.9E0 | 4.1E0 | 293 | 9 | 138 | 9 | 0.78 |
| Fc | pg/ml | 2.2E-1 | 6.3E0 | 1.4E2 | 5.2E1 | 1.4E3 | 1.1E2 | 2.2E-1 | 2.2E-1 | 1.5E4 | 3.1E2 | 113 | 7 | 87 | 7 | 0.69 |
| Fd | pg/ml | 1.4E1 | 2.8E2 | 1.0E3 | 4.1E3 | 3.9E3 | 9.3E3 | 4.5E-1 | 9.8E-1 | 3.3E4 | 2.5E4 | 113 | 7 | 87 | 7 | 0.71 |
| Fi | pg/ml | 2.5E-1 | 2.5E-1 | 1.8E2 | 3.7E1 | 1.3E3 | 9.5E1 | 2.5E-1 | 2.5E-1 | 1.4E4 | 2.5E2 | 113 | 7 | 87 | 7 | 0.54 |
| Fn | ng/ml | 2.1E-1 | 2.1E-1 | 5.5E0 | 2.9E0 | 2.4E1 | 8.3E0 | 1.1E-14 | 2.1E-1 | 4.2E2 | 2.7E1 | 332 | 10 | 193 | 10 | 0.38 |
| Fp | ng/ml | 1.3E1 | 6.2E1 | 2.5E1 | 6.0E1 | 2.8E1 | 3.8E1 | 6.0E-3 | 6.8E0 | 1.4E2 | 1.3E2 | 965 | 15 | 344 | 15 | 0.79 |
| Fr | ng/ml | 3.9E4 | 5.5E5 | 1.1E5 | 4.5E5 | 1.7E5 | 3.1E5 | 1.9E2 | 1.0E4 | 9.0E5 | 8.4E5 | 1079 | 17 | 348 | 17 | 0.80 |
| Fw | pg/ml | 1.1E0 | 9.4E0 | 5.6E1 | 4.0E1 | 4.4E2 | 9.6E1 | 1.1E-14 | 1.2E-1 | 6.9E3 | 3.3E2 | 398 | 11 | 201 | 11 | 0.65 |
| Fy | ng/ml | 3.3E1 | 1.1E2 | 5.6E1 | 2.1E2 | 6.6E1 | 2.3E2 | 1.2E-1 | 1.2E1 | 5.3E2 | 6.5E2 | 328 | 9 | 192 | 9 | 0.75 |
| Gh | pg/ml | 3.9E0 | 2.3E0 | 6.6E1 | 1.3E1 | 2.5E2 | 3.0E1 | 2.9E-2 | 2.9E-2 | 1.8E3 | 8.0E1 | 111 | 7 | 87 | 7 | 0.41 |
| Gb | % | 3.9E1 | 3.7E1 | 4.6E1 | 8.0E1 | 3.9E1 | 1.0E2 | 2.2E0 | 2.8E1 | 2.3E2 | 3.0E2 | 113 | 7 | 86 | 7 | 0.62 |
| Gc | ng/ml | 1.0E2 | 1.2E2 | 1.5E2 | 1.5E2 | 1.7E2 | 1.4E2 | 6.4E0 | 3.1E1 | 1.2E3 | 4.7E2 | 198 | 8 | 111 | 8 | 0.53 |
| Gd | ng/ml | 3.0E1 | 1.2E1 | 3.1E1 | 2.9E1 | 1.7E1 | 2.7E1 | 3.0E0 | 7.6E0 | 8.1E1 | 8.0E1 | 221 | 9 | 98 | 9 | 0.40 |
| Gn | U/ml | 2.8E-1 | 1.3E-1 | 1.2E0 | 1.5E1 | 2.9E0 | 4.0E1 | 1.3E-3 | 5.6E-3 | 3.0E1 | 1.1E2 | 179 | 8 | 105 | 8 | 0.48 |
| Gl | pg/ml | 7.2E3 | 2.3E4 | 1.1E4 | 2.1E4 | 9.3E3 | 9.9E3 | 9.1E1 | 1.3E3 | 3.4E4 | 3.2E4 | 388 | 11 | 199 | 11 | 0.77 |
| Gp | U/ml | 1.5E0 | 6.1E-1 | 3.9E0 | 1.6E0 | 6.6E0 | 2.4E0 | 1.3E-3 | 1.5E-2 | 6.7E1 | 7.3E0 | 400 | 10 | 201 | 10 | 0.36 |
| Gz | ug/ml | 1.2E0 | 1.4E0 | 7.8E0 | 3.5E0 | 3.4E1 | 4.5E0 | 2.9E-16 | 1.0E-1 | 4.8E2 | 1.1E1 | 224 | 9 | 126 | 9 | 0.46 |
| Ha | ng/ml | 2.7E0 | 5.6E0 | 9.4E0 | 1.8E1 | 2.0E1 | 3.2E1 | 6.4E-3 | 6.4E-1 | 1.3E2 | 1.0E2 | 330 | 10 | 192 | 10 | 0.61 |
| Nm | pg/ml | 1.4E4 | 3.3E4 | 3.3E4 | 9.0E4 | 7.8E4 | 2.0E5 | 1.0E-9 | 1.0E-9 | 1.6E6 | 8.2E5 | 935 | 16 | 345 | 16 | 0.63 |
| Nn | pg/ml | 1.6E2 | 2.1E3 | 1.7E3 | 1.5E4 | 7.6E3 | 3.2E4 | 1.0E-9 | 1.1E2 | 1.0E5 | 1.1E5 | 935 | 16 | 345 | 16 | 0.81 |
| No | pg/ml | 1.6E1 | 8.2E1 | 3.6E1 | 2.0E2 | 1.1E2 | 2.5E2 | 1.0E-9 | 9.0E0 | 2.5E3 | 7.7E2 | 935 | 16 | 345 | 16 | 0.83 |
| Nq | pg/ml | 2.0E0 | 2.4E1 | 1.8E1 | 5.9E1 | 7.2E1 | 7.7E1 | 1.0E-9 | 1.0E-9 | 1.2E3 | 2.4E2 | 935 | 16 | 345 | 16 | 0.71 |
| Nr | pg/ml | 9.9E-1 | 7.4E0 | 2.8E1 | 1.6E2 | 1.7E2 | 3.7E2 | 1.0E-9 | 1.0E-9 | 4.1E3 | 1.4E3 | 935 | 16 | 345 | 16 | 0.67 |
| Ns | pg/ml | 1.0E-9 | 1.0E-9 | 9.0E0 | 1.0E-9 | 5.1E1 | 0.0E0 | 1.0E-9 | 1.0E-9 | 1.1E3 | 1.0E-9 | 935 | 16 | 345 | 16 | 0.45 |
| Nt | pg/ml | 1.0E2 | 2.2E2 | 1.3E2 | 3.0E2 | 1.1E2 | 3.0E2 | 1.0E-9 | 4.4E1 | 1.5E3 | 1.2E3 | 935 | 16 | 345 | 16 | 0.74 |
| Nu | pg/ml | 2.0E1 | 7.4E1 | 5.4E1 | 1.2E2 | 8.9E1 | 1.1E2 | 1.0E-9 | 1.0E-9 | 8.9E2 | 3.7E2 | 935 | 16 | 345 | 16 | 0.71 |
| Lu | pg/ml | 1.0E4 | 5.5E3 | 1.8E4 | 7.0E3 | 6.1E4 | 5.8E3 | 3.5E2 | 1.3E3 | 1.3E6 | 2.2E4 | 938 | 16 | 345 | 16 | 0.31 |
| Lv | pg/ml | 1.0E-9 | 4.2E1 | 1.1E1 | 6.2E1 | 2.2E1 | 6.3E1 | 1.0E-9 | 1.0E-9 | 2.6E2 | 1.8E2 | 938 | 16 | 345 | 16 | 0.76 |
| Lw | pg/ml | 1.0E-9 | 1.0E-9 | 3.9E-1 | 5.7E0 | 4.0E0 | 1.2E1 | 1.0E-9 | 1.0E-9 | 8.0E1 | 4.0E1 | 938 | 16 | 345 | 16 | 0.62 |
| Lx | pg/ml | 1.0E-9 | 6.3E2 | 1.4E2 | 9.7E2 | 4.1E2 | 9.2E2 | 1.0E-9 | 1.0E-9 | 6.2E3 | 2.8E3 | 938 | 16 | 345 | 16 | 0.82 |
| Ly | pg/ml | 1.0E-9 | 1.0E-9 | 1.0E1 | 3.4E0 | 2.0E1 | 7.4E0 | 1.0E-9 | 1.0E-9 | 1.6E2 | 2.6E1 | 938 | 16 | 345 | 16 | 0.43 |
| Lz | pg/ml | 1.0E-9 | 1.0E-9 | 3.8E0 | 9.2E0 | 3.0E1 | 2.0E1 | 1.0E-9 | 1.0E-9 | 6.0E2 | 6.2E1 | 938 | 16 | 345 | 16 | 0.56 |
| Ma | pg/ml | 2.8E2 | 2.7E3 | 1.3E3 | 7.4E3 | 3.5E3 | 1.4E4 | 1.0E-9 | 8.7E1 | 6.5E4 | 5.2E4 | 938 | 16 | 345 | 16 | 0.74 |
| Mb | pg/ml | 2.5E1 | 3.7E1 | 3.1E1 | 3.7E1 | 1.5E1 | 2.0E1 | 5.4E0 | 4.1E0 | 2.1E2 | 7.1E1 | 938 | 16 | 345 | 16 | 0.56 |
| Mc | pg/ml | 1.0E-9 | 1.0E-9 | 4.1E-2 | 1.0E-9 | 5.6E-1 | 0.0E0 | 1.0E-9 | 1.0E-9 | 1.3E1 | 1.0E-9 | 938 | 16 | 345 | 16 | 0.49 |
| Md | pg/ml | 1.0E-9 | 1.0E-9 | 3.9E-1 | 2.2E0 | 3.4E0 | 7.4E0 | 1.0E-9 | 1.0E-9 | 6.8E1 | 2.9E1 | 938 | 16 | 345 | 16 | 0.56 |
| Me | pg/ml | 3.3E1 | 8.7E0 | 3.2E1 | 2.5E1 | 2.0E1 | 4.5E1 | 1.0E-9 | 1.0E-9 | 3.2E2 | 1.8E2 | 938 | 16 | 345 | 16 | 0.27 |
| Mf | pg/ml | 1.0E-9 | 1.0E-9 | 5.2E-1 | 1.0E0 | 2.9E0 | 2.0E0 | 1.0E-9 | 1.0E-9 | 5.6E1 | 5.5E0 | 938 | 16 | 345 | 16 | 0.59 |
| Mg | pg/ml | 1.6E0 | 2.0E0 | 7.4E0 | 7.8E0 | 1.3E1 | 9.9E0 | 1.0E-9 | 1.0E-9 | 9.4E1 | 2.7E1 | 938 | 16 | 345 | 16 | 0.53 |
| Mh | pg/ml | 1.0E-9 | 2.9E-2 | 1.3E0 | 3.4E0 | 9.4E0 | 6.1E0 | 1.0E-9 | 1.0E-9 | 1.6E2 | 1.8E1 | 938 | 16 | 345 | 16 | 0.65 |
| Mi | pg/ml | 1.0E-9 | 1.0E-9 | 4.8E-1 | 1.0E1 | 5.2E0 | 2.8E1 | 1.0E-9 | 1.0E-9 | 1.2E2 | 1.1E2 | 938 | 16 | 345 | 16 | 0.61 |
| Mj | pg/ml | 1.0E-9 | 1.2E0 | 4.3E0 | 3.5E1 | 2.4E1 | 6.6E1 | 1.0E-9 | 1.0E-9 | 4.6E2 | 2.1E2 | 938 | 16 | 345 | 16 | 0.67 |
| Mk | pg/ml | 9.1E-1 | 5.7E0 | 1.4E1 | 4.0E1 | 8.4E1 | 1.2E2 | 1.0E-9 | 1.0E-9 | 1.7E3 | 5.0E2 | 938 | 16 | 345 | 16 | 0.62 |
| Ml | pg/ml | 1.0E-9 | 1.0E-9 | 4.9E0 | 4.3E1 | 7.2E1 | 1.3E2 | 1.0E-9 | 1.0E-9 | 2.1E3 | 5.1E2 | 938 | 16 | 345 | 16 | 0.62 |
| Mm | pg/ml | 5.9E2 | 1.7E3 | 1.0E3 | 2.5E3 | 1.2E3 | 3.1E3 | 1.0E-9 | 6.2E1 | 1.1E4 | 1.2E4 | 938 | 16 | 345 | 16 | 0.68 |
| Mn | pg/ml | 5.5E0 | 1.1E1 | 1.0E1 | 1.8E1 | 2.2E1 | 1.4E1 | 1.0E-9 | 2.8E0 | 3.5E2 | 5.1E1 | 938 | 16 | 345 | 16 | 0.73 |
| Mp | pg/ml | 1.0E-9 | 2.3E1 | 8.9E0 | 3.7E1 | 2.9E1 | 4.2E1 | 1.0E-9 | 1.0E-9 | 6.3E2 | 1.3E2 | 937 | 16 | 345 | 16 | 0.75 |
| Mq | pg/ml | 1.0E-9 | 3.5E0 | 2.7E0 | 2.2E1 | 1.6E1 | 3.5E1 | 1.0E-9 | 1.0E-9 | 2.4E2 | 1.0E2 | 937 | 16 | 345 | 16 | 0.72 |
| Mr | pg/ml | 1.0E-9 | 1.2E1 | 2.5E1 | 3.4E2 | 1.4E2 | 8.8E2 | 1.0E-9 | 1.0E-9 | 2.2E3 | 3.4E3 | 937 | 16 | 345 | 16 | 0.75 |
| Ms | pg/ml | 4.1E2 | 2.2E2 | 5.6E2 | 3.1E2 | 6.6E2 | 3.0E2 | 1.0E-9 | 1.0E-9 | 5.9E3 | 9.8E2 | 937 | 16 | 345 | 16 | 0.39 |
| Mt | pg/ml | 2.5E-1 | 9.7E0 | 6.8E0 | 2.3E2 | 4.1E1 | 8.0E2 | 1.0E-9 | 1.0E-9 | 8.7E2 | 3.2E3 | 937 | 16 | 345 | 16 | 0.79 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Mu | pg/ml | 1.0E-9 | 3.3E0 | 1.2E0 | 5.9E0 | 1.0E1 | 9.4E0 | 1.0E-9 | 1.0E-9 | 2.3E2 | 3.5E1 | 937 | 16 | 345 | 16 | 0.82 |
| Mv | pg/ml | 1.0E-9 | 1.6E1 | 6.8E1 | 1.9E2 | 3.1E2 | 3.0E2 | 1.0E-9 | 1.0E-9 | 4.5E3 | 9.2E2 | 937 | 16 | 345 | 16 | 0.71 |
| Mw | pg/ml | 3.8E1 | 4.2E2 | 5.0E2 | 1.2E3 | 3.2E3 | 1.8E3 | 1.0E-9 | 1.0E-9 | 6.2E4 | 5.3E3 | 937 | 16 | 345 | 16 | 0.80 |
| Mx | pg/ml | 1.0E-9 | 1.0E-9 | 2.4E-1 | 2.1E0 | 1.3E0 | 5.0E0 | 1.0E-9 | 1.0E-9 | 3.2E1 | 2.0E1 | 937 | 16 | 345 | 16 | 0.66 |
| My | pg/ml | 1.0E-9 | 3.7E1 | 4.5E2 | 2.7E2 | 2.9E3 | 5.3E2 | 1.0E-9 | 1.0E-9 | 3.9E4 | 1.9E3 | 937 | 16 | 345 | 16 | 0.60 |
| Mz | pg/ml | 1.1E1 | 2.9E1 | 2.7E1 | 2.5E2 | 6.3E1 | 5.5E2 | 1.0E-9 | 1.0E-9 | 1.2E3 | 1.9E3 | 937 | 16 | 345 | 16 | 0.71 |
| Na | pg/ml | 1.0E-9 | 1.0E-9 | 6.6E-1 | 4.9E0 | 2.6E0 | 1.1E1 | 1.0E-9 | 1.0E-9 | 3.6E1 | 4.2E1 | 937 | 16 | 345 | 16 | 0.61 |
| Nb | pg/ml | 2.1E0 | 5.4E0 | 3.8E0 | 2.5E1 | 1.1E1 | 5.5E1 | 1.0E-9 | 1.0E-9 | 2.3E2 | 2.1E2 | 937 | 16 | 345 | 16 | 0.69 |
| Nc | pg/ml | 3.4E2 | 2.7E2 | 5.6E2 | 3.7E2 | 7.2E2 | 3.7E2 | 1.0E-9 | 1.0E-9 | 9.2E3 | 1.1E3 | 937 | 16 | 345 | 16 | 0.47 |
| Nd | pg/ml | 2.9E1 | 1.1E1 | 2.7E1 | 2.2E1 | 4.4E1 | 2.6E1 | 1.0E-9 | 1.0E-9 | 1.2E3 | 9.4E1 | 937 | 16 | 345 | 16 | 0.43 |
| Ne | pg/ml | 4.4E2 | 2.9E2 | 5.7E2 | 2.7E2 | 5.7E2 | 1.8E2 | 1.0E-9 | 1.0E-9 | 7.0E3 | 5.8E2 | 937 | 16 | 345 | 16 | 0.34 |
| Nf | pg/ml | 1.0E-9 | 8.7E-1 | 2.6E0 | 1.6E1 | 9.4E0 | 4.1E1 | 1.0E-9 | 1.0E-9 | 1.6E2 | 1.3E2 | 937 | 16 | 345 | 16 | 0.63 |
| Ng | pg/ml | 1.9E1 | 7.6E0 | 1.3E2 | 3.9E1 | 2.5E2 | 5.8E1 | 1.0E-9 | 1.0E-9 | 2.3E3 | 1.7E2 | 937 | 16 | 345 | 16 | 0.42 |
| Nh | pg/ml | 6.9E1 | 3.7E1 | 9.0E1 | 4.0E1 | 8.2E1 | 2.3E1 | 1.0E-9 | 4.1E0 | 5.6E2 | 7.5E1 | 937 | 16 | 345 | 16 | 0.30 |
| Ni | pg/ml | 1.0E-9 | 2.2E2 | 7.2E1 | 2.5E2 | 1.2E2 | 2.9E2 | 1.0E-9 | 1.0E-9 | 7.1E2 | 1.1E3 | 937 | 16 | 345 | 16 | 0.69 |
| Nj | pg/ml | 7.5E0 | 4.9E0 | 1.1E1 | 7.2E0 | 1.2E1 | 6.5E0 | 1.0E-9 | 1.0E-9 | 1.1E2 | 2.0E1 | 937 | 16 | 345 | 16 | 0.43 |
| Nk | pg/ml | 1.7E1 | 2.8E1 | 3.3E1 | 2.7E1 | 3.9E1 | 2.2E1 | 1.0E-9 | 1.0E-9 | 2.0E2 | 6.6E1 | 937 | 16 | 345 | 16 | 0.52 |
| Nl | pg/ml | 4.5E1 | 3.0E1 | 6.1E1 | 3.3E1 | 6.8E1 | 2.5E1 | 1.0E-9 | 1.0E-9 | 1.1E3 | 8.2E1 | 937 | 16 | 345 | 16 | 0.38 |
| Hl | pg/ml | 1.4E1 | 2.4E1 | 3.6E1 | 5.6E2 | 6.2E1 | 1.3E3 | 1.0E-9 | 1.0E-9 | 3.5E2 | 3.6E3 | 113 | 7 | 87 | 7 | 0.56 |
| Ho | pg/ml | 1.7E1 | 2.2E1 | 2.8E1 | 7.1E1 | 6.7E1 | 1.4E2 | 1.0E-9 | 2.0E0 | 7.0E2 | 3.9E2 | 113 | 7 | 87 | 7 | 0.58 |
| Hp | ng/ml | 1.6E0 | 7.7E0 | 1.2E2 | 3.8E2 | 3.0E2 | 4.7E2 | 1.0E-9 | 1.4E0 | 8.9E2 | 8.9E2 | 113 | 7 | 87 | 7 | 0.76 |
| Tz | pg/ml | 5.1E3 | 1.0E4 | 2.0E4 | 1.1E4 | 1.3E5 | 7.9E3 | 1.0E-9 | 8.1E2 | 2.1E6 | 2.5E4 | 334 | 10 | 191 | 10 | 0.64 |
| Ua | pg/ml | 3.8E3 | 9.5E3 | 2.0E4 | 1.7E4 | 1.2E5 | 1.9E4 | 1.0E-9 | 1.1E3 | 2.1E6 | 5.8E4 | 334 | 10 | 191 | 10 | 0.66 |
| Ub | pg/ml | 5.6E2 | 3.5E2 | 8.3E2 | 6.1E2 | 1.0E3 | 8.3E2 | 1.0E-9 | 1.2E1 | 9.8E3 | 2.8E3 | 334 | 10 | 191 | 10 | 0.39 |
| Ue | pg/ml | 2.7E1 | 2.0E1 | 3.8E1 | 3.5E1 | 4.1E1 | 4.0E1 | 9.8E-2 | 5.9E0 | 4.4E2 | 1.4E2 | 334 | 10 | 191 | 10 | 0.41 |
| Uc | pg/ml | 8.9E2 | 1.6E3 | 1.7E3 | 7.4E3 | 2.6E3 | 1.8E4 | 1.0E-9 | 5.5E1 | 2.9E4 | 5.7E4 | 334 | 10 | 191 | 10 | 0.57 |
| Ud | pg/ml | 1.0E-9 | 1.0E-9 | 1.3E0 | 5.8E0 | 2.1E1 | 1.7E1 | 1.0E-9 | 1.0E-9 | 3.9E2 | 5.3E1 | 334 | 10 | 191 | 10 | 0.60 |
| Hq | pg/ml | 1.1E0 | 2.2E0 | 9.6E1 | 1.9E1 | 1.6E3 | 4.6E1 | 1.0E-9 | 1.0E-9 | 3.4E4 | 1.8E2 | 933 | 16 | 344 | 16 | 0.59 |
| Hr | pg/ml | 1.1E2 | 1.2E2 | 7.5E2 | 1.3E3 | 1.6E3 | 2.5E3 | 1.0E-9 | 1.0E-9 | 1.7E4 | 8.9E3 | 933 | 16 | 344 | 16 | 0.55 |
| Hu | pg/ml | 5.3E0 | 1.8E2 | 3.0E3 | 4.8E2 | 2.6E4 | 6.6E2 | 1.0E-9 | 1.0E-9 | 6.3E5 | 2.4E3 | 933 | 16 | 344 | 16 | 0.69 |
| Hv | pg/ml | 1.4E0 | 1.7E0 | 3.3E0 | 6.1E1 | 1.2E1 | 2.2E2 | 1.0E-9 | 1.0E-9 | 2.5E2 | 8.9E2 | 933 | 16 | 344 | 16 | 0.58 |
| Hw | pg/ml | 6.4E0 | 8.8E0 | 1.8E1 | 6.2E2 | 6.9E1 | 2.3E3 | 1.0E-9 | 5.1E-1 | 1.7E3 | 9.4E3 | 933 | 16 | 344 | 16 | 0.54 |
| Hx | pg/ml | 8.8E0 | 1.7E1 | 3.7E1 | 1.6E2 | 3.1E2 | 3.6E2 | 1.0E-9 | 1.0E-9 | 9.3E3 | 1.3E3 | 933 | 16 | 344 | 16 | 0.70 |
| Ib | ng/ml | 4.9E-2 | 1.2E-1 | 1.7E0 | 5.8E0 | 6.5E0 | 1.8E1 | 1.0E-9 | 1.0E-9 | 5.3E1 | 5.6E1 | 324 | 10 | 190 | 10 | 0.58 |
| Ic | U/ml | 2.2E2 | 2.2E2 | 1.0E3 | 2.1E2 | 6.6E3 | 1.5E2 | 1.5E0 | 4.2E1 | 9.3E4 | 4.2E2 | 324 | 10 | 190 | 10 | 0.52 |
| Id | U/ml | 6.9E-1 | 1.0E0 | 1.4E0 | 4.5E1 | 2.4E0 | 1.4E2 | 1.0E-9 | 3.0E-1 | 2.3E1 | 4.3E2 | 324 | 10 | 190 | 10 | 0.67 |
| Tt | pg/ml | 1.6E2 | 1.7E2 | 1.7E2 | 2.1E2 | 5.1E1 | 1.0E2 | 4.3E1 | 1.1E2 | 3.6E2 | 4.4E2 | 308 | 8 | 184 | 8 | 0.59 |
| To | pg/ml | 1.6E0 | 1.8E0 | 1.9E0 | 2.7E0 | 2.3E0 | 3.6E0 | 1.0E-9 | 1.0E-9 | 2.3E1 | 1.2E1 | 321 | 9 | 188 | 9 | 0.56 |
| Tr | pg/ml | 3.0E0 | 5.6E0 | 5.9E0 | 1.9E1 | 1.8E1 | 2.9E1 | 1.0E-9 | 7.6E-1 | 3.1E2 | 7.6E1 | 317 | 8 | 187 | 8 | 0.62 |
| Tn | pg/ml | 2.8E1 | 1.6E2 | 7.4E1 | 5.7E2 | 1.9E2 | 9.0E2 | 1.0E-9 | 2.1E1 | 1.8E3 | 2.3E3 | 321 | 9 | 188 | 9 | 0.77 |
| Tv | ng/ml | 1.2E1 | 1.5E1 | 2.3E1 | 8.0E2 | 6.2E1 | 2.4E3 | 1.0E-9 | 1.0E-9 | 7.9E2 | 7.1E3 | 321 | 9 | 188 | 9 | 0.54 |
| Ih | ng/ml | 7.2E1 | 3.5E2 | 2.4E2 | 6.3E2 | 4.8E2 | 7.3E2 | 1.0E-9 | 1.0E-9 | 7.4E3 | 2.8E3 | 937 | 16 | 344 | 16 | 0.70 |
| Ii | ng/ml | 9.5E1 | 1.8E2 | 2.4E2 | 6.8E2 | 6.6E2 | 1.3E3 | 1.0E-9 | 2.3E0 | 1.0E4 | 4.5E3 | 937 | 16 | 344 | 16 | 0.59 |
| Ij | ng/ml | 7.6E1 | 1.4E2 | 1.7E2 | 1.7E3 | 5.7E2 | 6.0E3 | 1.6E-1 | 2.5E1 | 6.4E3 | 2.4E4 | 924 | 16 | 342 | 16 | 0.71 |
| Ik | ng/ml | 1.3E1 | 6.0E1 | 7.9E2 | 4.1E2 | 8.0E3 | 7.0E2 | 5.9E-1 | 5.5E0 | 1.2E5 | 2.5E3 | 932 | 16 | 342 | 16 | 0.67 |
| Il | ng/ml | 3.3E2 | 5.4E2 | 1.3E3 | 2.3E3 | 2.8E3 | 3.9E3 | 1.0E-9 | 1.0E-9 | 1.3E4 | 1.2E4 | 916 | 16 | 342 | 16 | 0.56 |
| Im | ng/ml | 2.1E2 | 7.0E2 | 4.0E2 | 1.2E3 | 7.6E2 | 1.5E3 | 1.3E1 | 4.7E1 | 1.5E4 | 6.2E3 | 931 | 16 | 343 | 16 | 0.80 |
| In | ng/ml | 3.3E0 | 4.1E0 | 2.1E1 | 3.0E2 | 1.5E2 | 1.1E3 | 1.0E-9 | 1.0E-9 | 3.9E3 | 4.5E3 | 937 | 16 | 344 | 16 | 0.50 |
| Hb | ng/ml | 2.5E1 | 2.3E1 | 3.6E1 | 3.3E1 | 3.5E1 | 2.4E1 | 4.8E-1 | 6.2E-1 | 2.1E2 | 8.0E1 | 333 | 10 | 191 | 10 | 0.52 |
| Hc | pg/ml | 6.6E2 | 5.7E2 | 3.4E3 | 7.0E3 | 1.2E4 | 1.6E4 | 1.0E-9 | 1.0E-9 | 1.0E5 | 5.0E4 | 333 | 10 | 191 | 10 | 0.47 |
| Hf | ng/ml | 1.6E2 | 9.7E1 | 3.7E2 | 2.1E2 | 5.1E2 | 2.5E2 | 1.0E-9 | 1.0E-9 | 3.2E3 | 6.7E2 | 333 | 10 | 191 | 10 | 0.41 |
| Io | ng/ml | 8.3E3 | 6.1E3 | 2.5E4 | 1.1E4 | 1.4E5 | 1.1E4 | 1.0E-9 | 1.3E3 | 4.0E6 | 3.3E4 | 928 | 16 | 344 | 16 | 0.47 |
| Ip | ng/ml | 1.0E1 | 3.0E1 | 2.0E1 | 2.9E1 | 2.4E1 | 2.2E1 | 1.0E-9 | 3.7E-2 | 2.6E2 | 5.7E1 | 928 | 16 | 344 | 16 | 0.61 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Iq | ug/ml | 1.0E-1 | 3.9E-1 | 3.0E1 | 1.6E1 | 6.3E2 | 5.5E1 | 1.0E-9 | 1.0E-9 | 1.4E4 | 2.2E2 | 928 | 16 | 344 | 16 | 0.63 |
| Ir | ug/ml | 3.5E-1 | 1.3E0 | 3.4E0 | 4.0E1 | 2.4E1 | 9.7E1 | 1.0E-9 | 1.0E-9 | 5.1E2 | 3.7E2 | 927 | 16 | 344 | 16 | 0.74 |
| Is | ng/ml | 1.6E0 | 1.3E1 | 6.4E0 | 4.4E1 | 2.2E1 | 6.9E1 | 1.0E-9 | 4.9E-1 | 5.5E2 | 2.6E2 | 928 | 16 | 344 | 16 | 0.81 |
| It | ng/ml | 2.0E0 | 3.9E0 | 2.3E1 | 5.9E1 | 1.4E2 | 1.7E2 | 1.0E-9 | 1.0E-9 | 2.8E3 | 6.8E2 | 928 | 16 | 344 | 16 | 0.60 |
| Iu | ng/ml | 2.2E2 | 4.8E2 | 1.3E3 | 2.3E3 | 4.0E3 | 6.0E3 | 1.0E-9 | 1.0E-9 | 2.9E4 | 2.4E4 | 928 | 16 | 344 | 16 | 0.56 |
| Iv | ng/ml | 1.3E1 | 7.8E1 | 6.0E1 | 5.5E2 | 5.4E2 | 1.2E3 | 1.0E-9 | 1.0E-9 | 1.6E4 | 3.8E3 | 927 | 16 | 344 | 16 | 0.73 |
| Iz | ng/ml | 1.4E2 | 2.0E2 | 5.9E2 | 4.8E2 | 3.5E3 | 5.6E2 | 9.2E-1 | 4.9E0 | 6.2E4 | 1.7E3 | 333 | 10 | 191 | 10 | 0.57 |
| Yd | ng/ml | 2.0E-1 | 1.9E-1 | 3.4E-1 | 4.6E-1 | 3.9E-1 | 8.3E-1 | 6.6E-3 | 1.7E-2 | 1.9E0 | 2.3E0 | 116 | 7 | 90 | 7 | 0.46 |
| Wb | pg/ml | 3.1E4 | 4.6E4 | 4.1E4 | 6.3E4 | 6.1E4 | 4.0E4 | 2.2E3 | 3.3E4 | 6.4E5 | 1.5E5 | 115 | 7 | 90 | 7 | 0.77 |
| Vz | pg/ml | 3.0E0 | 6.3E0 | 4.4E0 | 7.9E0 | 5.7E0 | 6.8E0 | 1.0E-9 | 1.9E0 | 4.0E1 | 2.2E1 | 115 | 7 | 90 | 7 | 0.73 |
| Si | ng/ml | 1.0E0 | 7.9E-1 | 1.9E0 | 1.7E0 | 2.6E0 | 2.0E0 | 8.6E-3 | 2.4E-1 | 1.3E1 | 5.6E0 | 113 | 7 | 87 | 7 | 0.50 |
| Sf | mIU/mL | 1.3E1 | 8.1E0 | 3.8E1 | 1.3E1 | 8.1E1 | 1.1E1 | 8.1E-2 | 9.4E-1 | 7.2E2 | 3.3E1 | 113 | 7 | 87 | 7 | 0.40 |
| Sh | mIU/mL | 1.2E1 | 4.2E0 | 4.6E1 | 1.2E1 | 1.0E2 | 2.1E1 | 2.9E-2 | 3.4E-1 | 5.9E2 | 5.8E1 | 113 | 7 | 87 | 7 | 0.33 |
| Sj | ng/ml | 4.0E-1 | 4.3E-1 | 4.1E-1 | 4.4E-1 | 1.0E-1 | 5.1E-2 | 1.1E-1 | 4.0E-1 | 7.2E-1 | 5.1E-1 | 113 | 7 | 87 | 7 | 0.61 |
| Rc | pg/ml | 5.6E3 | 7.1E3 | 7.1E3 | 8.2E3 | 5.7E3 | 5.4E3 | 1.9E2 | 2.1E3 | 3.9E4 | 1.7E4 | 331 | 10 | 191 | 10 | 0.57 |
| Rb | pg/ml | 8.2E-1 | 7.2E-1 | 2.6E0 | 8.3E0 | 4.2E0 | 1.7E1 | 1.0E-9 | 1.0E-9 | 4.3E1 | 5.6E1 | 331 | 10 | 191 | 10 | 0.55 |
| Zq | 2.6ng/ml | 2.4E2 | 3.9E2 | 3.1E2 | 5.0E2 | 2.7E2 | 2.4E2 | 8.3E0 | 3.0E2 | 9.7E2 | 9.7E2 | 113 | 7 | 87 | 7 | 0.75 |
| Zw | 2.5ng/ml | 4.4E0 | 1.3E1 | 1.0E1 | 2.6E1 | 1.3E1 | 2.6E1 | 6.3E-2 | 7.7E-1 | 5.9E1 | 6.3E1 | 116 | 7 | 90 | 7 | 0.70 |
| Zx | 2.3mU/ml | 1.1E-1 | 1.3E-1 | 3.4E-1 | 4.1E-1 | 1.1E0 | 6.5E-1 | 3.2E-2 | 7.0E-2 | 1.2E1 | 1.9E0 | 116 | 7 | 90 | 7 | 0.61 |
| Pz | ng/ml | 3.8E3 | 1.0E4 | 8.1E3 | 7.0E3 | 3.7E4 | 4.1E3 | 1.3E1 | 6.5E2 | 1.0E6 | 1.3E4 | 929 | 16 | 342 | 16 | 0.62 |
| Qa | ng/ml | 3.5E3 | 1.5E4 | 6.3E3 | 2.9E4 | 7.4E3 | 5.3E4 | 1.2E1 | 1.5E3 | 5.2E4 | 2.2E5 | 929 | 16 | 342 | 16 | 0.83 |
| Qb | ng/ml | 9.7E1 | 2.8E2 | 2.1E2 | 3.5E2 | 4.8E2 | 2.4E2 | 7.9E-1 | 5.1E1 | 8.3E3 | 8.8E2 | 929 | 16 | 342 | 16 | 0.76 |
| Qc | ng/ml | 2.3E2 | 5.4E2 | 6.3E2 | 6.5E2 | 5.5E3 | 7.2E2 | 1.0E-9 | 1.3E1 | 1.7E5 | 2.8E3 | 929 | 16 | 342 | 16 | 0.61 |
| Qd | ng/ml | 9.2E3 | 3.5E4 | 2.1E4 | 6.3E4 | 7.7E4 | 7.0E4 | 1.5E2 | 4.6E3 | 2.0E6 | 2.3E5 | 929 | 16 | 342 | 16 | 0.77 |
| Qe | ng/ml | 9.2E2 | 3.6E3 | 1.8E3 | 5.3E3 | 3.8E3 | 4.9E3 | 1.0E-9 | 4.1E2 | 9.7E4 | 1.8E4 | 929 | 16 | 342 | 16 | 0.82 |
| Jd | ng/ml | 9.4E-1 | 4.7E0 | 5.8E0 | 7.3E0 | 3.8E1 | 6.1E0 | 1.0E-9 | 1.4E0 | 6.5E2 | 2.1E1 | 332 | 10 | 193 | 10 | 0.84 |
| Je | ng/ml | 1.0E-9 | 4.3E0 | 2.0E0 | 4.4E0 | 7.0E0 | 4.3E0 | 1.0E-9 | 1.0E-9 | 8.8E1 | 1.1E1 | 332 | 10 | 193 | 10 | 0.70 |
| Jf | ng/ml | 1.0E-9 | 8.4E-1 | 1.1E0 | 1.5E0 | 2.2E0 | 1.7E0 | 1.0E-9 | 1.0E-9 | 1.2E1 | 3.7E0 | 332 | 10 | 193 | 10 | 0.59 |
| Jg | ng/ml | 4.9E2 | 1.8E3 | 8.2E2 | 2.0E3 | 1.0E3 | 1.8E3 | 1.0E-9 | 8.7E1 | 1.0E4 | 7.1E3 | 933 | 16 | 344 | 16 | 0.74 |
| Jh | ng/ml | 3.0E0 | 5.2E1 | 2.7E1 | 8.5E1 | 1.1E2 | 1.2E2 | 1.0E-9 | 1.0E-9 | 1.6E3 | 4.9E2 | 933 | 16 | 344 | 16 | 0.76 |
| Ji | ng/ml | 5.3E1 | 2.3E2 | 7.7E1 | 3.3E2 | 8.0E1 | 3.2E2 | 1.0E-9 | 2.0E1 | 6.9E2 | 1.3E3 | 933 | 16 | 344 | 16 | 0.86 |
| Sr | pg/mL | 3.8E2 | 3.1E3 | 8.5E2 | 4.4E3 | 1.3E3 | 6.1E3 | 1.0E-9 | 2.3E2 | 9.8E3 | 2.1E4 | 322 | 10 | 188 | 10 | 0.82 |
| Ss | pg/mL | 9.5E4 | 1.7E5 | 1.5E5 | 1.4E5 | 1.8E5 | 1.1E5 | 2.7E3 | 1.4E4 | 1.8E6 | 3.6E5 | 322 | 10 | 188 | 10 | 0.54 |
| St | pg/mL | 2.6E7 | 1.0E8 | 5.6E7 | 2.3E8 | 9.4E7 | 5.0E8 | 1.0E-9 | 2.3E6 | 1.2E9 | 1.7E9 | 326 | 10 | 189 | 10 | 0.75 |
| Wc | ng/ml | 1.0E-9 | 1.0E-9 | 5.8E-2 | 2.6E-1 | 1.3E-1 | 6.7E-1 | 1.0E-9 | 1.0E-9 | 9.8E-1 | 1.8E0 | 116 | 7 | 90 | 7 | 0.44 |
| Wd | ng/ml | 9.4E0 | 1.8E1 | 4.3E1 | 1.1E2 | 1.4E2 | 1.6E2 | 1.0E-9 | 4.3E0 | 1.2E3 | 3.8E2 | 116 | 7 | 90 | 7 | 0.73 |
| We | ng/ml | 3.8E-1 | 4.7E-1 | 1.2E0 | 4.1E0 | 3.0E0 | 8.4E0 | 1.0E-9 | 2.0E-3 | 2.3E1 | 2.3E1 | 116 | 7 | 90 | 7 | 0.58 |
| Wg | ng/ml | 1.0E-9 | 1.0E-9 | 1.4E-4 | 7.6E-2 | 1.5E-3 | 2.0E-1 | 1.0E-9 | 1.0E-9 | 1.6E-2 | 5.3E-1 | 116 | 7 | 90 | 7 | 0.57 |
| Wh | ng/ml | 9.8E-3 | 4.5E-2 | 1.1E-1 | 9.1E-2 | 4.9E-1 | 1.2E-1 | 1.0E-9 | 3.7E-3 | 4.5E0 | 3.4E-1 | 116 | 7 | 90 | 7 | 0.70 |
| Wf | ng/ml | 1.0E-9 | 1.0E-9 | 1.7E-1 | 4.7E-1 | 5.2E-1 | 8.4E-1 | 1.0E-9 | 1.0E-9 | 4.6E0 | 2.3E0 | 116 | 7 | 90 | 7 | 0.58 |
| Ra | pg/ml | 1.0E-9 | 1.0E-9 | 6.1E-1 | 7.0E0 | 1.2E0 | 2.0E1 | 1.0E-9 | 1.0E-9 | 7.3E0 | 6.4E1 | 331 | 10 | 191 | 10 | 0.48 |
| Qz | pg/ml | 1.0E1 | 1.4E1 | 6.3E1 | 3.9E1 | 1.0E2 | 4.8E1 | 1.0E-9 | 1.0E-9 | 7.1E2 | 1.4E2 | 331 | 10 | 191 | 10 | 0.58 |
| Qy | pg/ml | 4.4E-1 | 3.1E0 | 1.6E1 | 8.4E0 | 7.6E1 | 1.6E1 | 1.0E-9 | 1.1E-1 | 6.5E2 | 5.4E1 | 331 | 10 | 191 | 10 | 0.75 |
| Qx | pg/ml | 1.0E-9 | 1.0E-9 | 5.4E0 | 1.4E1 | 4.5E1 | 3.4E1 | 1.0E-9 | 1.0E-9 | 5.8E2 | 1.1E2 | 331 | 10 | 191 | 10 | 0.59 |
| Qw | pg/ml | 1.0E-9 | 1.0E0 | 2.9E0 | 3.6E0 | 1.4E1 | 6.9E0 | 1.0E-9 | 1.0E-9 | 1.3E2 | 2.3E1 | 331 | 10 | 191 | 10 | 0.66 |
| Qv | pg/ml | 2.3E4 | 7.9E3 | 3.5E4 | 1.1E4 | 5.5E4 | 1.2E4 | 1.0E-9 | 4.0E2 | 7.4E5 | 3.3E4 | 331 | 10 | 191 | 10 | 0.24 |
| Qu | pg/ml | 7.8E0 | 5.8E0 | 8.2E1 | 1.1E2 | 1.7E2 | 2.1E2 | 1.0E-9 | 1.0E-9 | 9.8E2 | 6.7E2 | 331 | 10 | 191 | 10 | 0.51 |
| Qt | pg/ml | 1.0E1 | 5.4E1 | 4.9E1 | 9.2E1 | 1.2E2 | 1.1E2 | 1.0E-9 | 1.0E-9 | 1.0E3 | 3.1E2 | 331 | 10 | 191 | 10 | 0.68 |
| Qh | ng/ml | 1.7E1 | 5.3E1 | 4.0E1 | 6.4E1 | 7.7E1 | 5.5E1 | 1.0E-9 | 5.1E0 | 8.0E2 | 1.6E2 | 331 | 10 | 191 | 10 | 0.70 |
| Qg | ng/ml | 8.3E0 | 4.1E0 | 2.0E1 | 2.1E1 | 6.3E1 | 4.3E1 | 5.1E-2 | 1.5E0 | 1.0E3 | 1.4E2 | 331 | 10 | 191 | 10 | 0.40 |
| Jj | ng/ml | 6.0E2 | 1.1E2 | 1.7E3 | 4.2E2 | 1.2E4 | 8.0E2 | 2.3E0 | 1.2E1 | 3.4E5 | 3.3E3 | 933 | 16 | 344 | 16 | 0.23 |
| Jk | ng/ml | 3.1E0 | 1.6E1 | 2.1E1 | 4.8E1 | 4.7E1 | 6.5E1 | 1.0E-9 | 2.4E-1 | 3.9E2 | 2.4E2 | 933 | 16 | 344 | 16 | 0.69 |
| Jl | ng/ml | 4.4E-1 | 1.5E1 | 1.9E0 | 6.3E2 | 5.6E0 | 2.5E3 | 7.6E-4 | 1.4E-1 | 1.1E2 | 9.9E3 | 933 | 16 | 344 | 16 | 0.82 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Jm | ng/ml | 1.8E1 | 4.7E1 | 5.8E1 | 5.6E1 | 1.3E2 | 5.3E1 | 1.0E-9 | 4.7E-1 | 2.1E3 | 1.5E2 | 933 | 16 | 344 | 16 | 0.58 |
| Jn | pg/ml | 4.0E-1 | 1.4E0 | 2.5E0 | 8.7E1 | 2.2E1 | 2.3E2 | 1.0E-9 | 1.6E-1 | 6.2E2 | 7.3E2 | 932 | 16 | 344 | 16 | 0.76 |
| Jo | pg/ml | 3.6E3 | 3.7E3 | 4.7E3 | 1.2E4 | 3.8E3 | 2.5E4 | 2.0E1 | 2.3E2 | 2.4E4 | 1.0E5 | 933 | 16 | 344 | 16 | 0.50 |
| Jp | pg/ml | 7.0E4 | 9.8E4 | 7.4E4 | 1.1E5 | 3.8E4 | 4.7E4 | 5.8E2 | 4.5E4 | 3.8E5 | 2.1E5 | 933 | 16 | 344 | 16 | 0.75 |
| Jq | pg/ml | 9.4E1 | 3.1E2 | 1.5E2 | 1.1E3 | 2.1E2 | 2.2E3 | 1.0E0 | 1.1E1 | 4.0E3 | 8.7E3 | 933 | 16 | 344 | 16 | 0.76 |
| Jr | pg/ml | 5.2E0 | 2.3E1 | 3.3E1 | 8.2E2 | 3.6E2 | 2.2E3 | 1.0E-9 | 2.6E0 | 1.1E4 | 7.4E3 | 933 | 16 | 344 | 16 | 0.81 |
| Js | pg/ml | 1.3E1 | 2.0E1 | 4.9E1 | 4.0E2 | 3.6E2 | 1.0E3 | 1.0E-9 | 3.0E0 | 1.0E4 | 3.0E3 | 933 | 16 | 344 | 16 | 0.65 |
| Jt | pg/ml | 2.6E3 | 4.4E3 | 3.2E3 | 8.0E3 | 2.4E3 | 1.3E4 | 2.2E1 | 4.1E2 | 2.2E4 | 5.2E4 | 933 | 16 | 344 | 16 | 0.60 |
| Xa | pg/ml | 1.0E-9 | 1.7E1 | 1.5E1 | 2.0E2 | 5.5E1 | 4.5E2 | 1.0E-9 | 4.9E0 | 5.6E2 | 1.2E3 | 115 | 7 | 90 | 7 | 0.81 |
| Yc | pg/ml | 1.0E-9 | 1.0E-9 | 7.7E0 | 1.0E-9 | 3.5E1 | 0.0E0 | 1.0E-9 | 1.0E-9 | 3.5E2 | 1.0E-9 | 115 | 7 | 90 | 7 | 0.35 |
| Tm | pg/ml | 1.0E-9 | 1.0E-9 | 3.8E0 | 1.0E-9 | 8.8E0 | 0.0E0 | 1.0E-9 | 1.0E-9 | 6.8E1 | 1.0E-9 | 115 | 7 | 90 | 7 | 0.35 |
| Tl | pg/ml | 1.3E-1 | 1.0E-9 | 2.9E-1 | 3.7E0 | 3.6E-1 | 9.3E0 | 1.0E-9 | 1.0E-9 | 1.8E0 | 2.5E1 | 115 | 7 | 90 | 7 | 0.45 |
| Ju | mIU/ml | 8.7E0 | 8.2E0 | 1.9E1 | 1.0E1 | 2.9E1 | 8.4E0 | 4.8E-2 | 1.9E-1 | 2.3E2 | 2.6E1 | 332 | 10 | 193 | 10 | 0.47 |
| Jv | mIU/ml | 1.2E1 | 4.6E0 | 3.3E1 | 9.1E0 | 5.7E1 | 1.2E1 | 9.4E-3 | 2.1E-1 | 4.4E2 | 3.7E1 | 332 | 10 | 193 | 10 | 0.36 |
| Jy | ng/ml | 1.6E-3 | 4.0E-3 | 2.1E-3 | 9.3E-3 | 3.8E-3 | 1.3E-2 | 1.0E-9 | 8.6E-4 | 5.2E-2 | 4.1E-2 | 332 | 10 | 193 | 10 | 0.79 |
| Kc | pg/ml | 2.6E1 | 2.6E1 | 4.6E1 | 5.3E1 | 4.9E1 | 5.7E1 | 1.0E-9 | 6.9E0 | 2.7E2 | 1.6E2 | 333 | 10 | 191 | 10 | 0.49 |
| Kd | pg/ml | 1.0E-9 | 1.0E-9 | 2.1E2 | 4.4E3 | 6.4E2 | 1.2E4 | 1.0E-9 | 1.0E-9 | 5.0E3 | 3.8E4 | 333 | 10 | 191 | 10 | 0.52 |
| Kc | pg/ml | 1.3E4 | 1.4E4 | 1.5E4 | 5.8E4 | 1.1E4 | 9.9E4 | 3.4E2 | 4.2E3 | 7.0E4 | 3.2E5 | 333 | 10 | 191 | 10 | 0.60 |
| Kf | pg/mL | 7.2E0 | 6.7E0 | 7.4E0 | 1.5E1 | 6.0E0 | 2.3E1 | 1.0E-9 | 1.7E0 | 4.4E1 | 7.8E1 | 333 | 10 | 191 | 10 | 0.55 |
| Kg | pg/mL | 1.1E3 | 6.9E2 | 1.9E3 | 3.5E3 | 2.4E3 | 8.3E3 | 7.3E1 | 1.3E2 | 2.2E4 | 2.7E4 | 333 | 10 | 191 | 10 | 0.37 |
| Ki | pg/ml | 5.9E1 | 9.5E1 | 7.0E1 | 1.0E2 | 5.3E1 | 4.2E1 | 1.0E-9 | 5.9E1 | 3.8E2 | 2.0E2 | 332 | 10 | 191 | 10 | 0.74 |
| Kj | pg/ml | 1.0E3 | 4.0E2 | 1.6E3 | 2.0E3 | 1.6E3 | 4.5E3 | 1.4E1 | 1.2E2 | 1.0E4 | 1.5E4 | 333 | 10 | 191 | 10 | 0.31 |
| Kk | pg/ml | 6.9E0 | 1.2E1 | 1.2E1 | 2.3E1 | 1.5E1 | 2.1E1 | 1.0E-9 | 5.0E0 | 1.6E2 | 5.9E1 | 333 | 10 | 191 | 10 | 0.72 |
| Kl | pg/ml | 2.0E4 | 2.7E4 | 2.8E4 | 3.0E4 | 2.5E4 | 2.6E4 | 1.6E2 | 1.6E3 | 1.6E5 | 7.8E4 | 333 | 10 | 191 | 10 | 0.52 |
| Kn | pg/ml | 3.0E1 | 6.7E1 | 6.3E1 | 5.7E2 | 1.0E2 | 1.5E3 | 1.0E-9 | 1.0E-9 | 7.3E2 | 4.9E3 | 333 | 10 | 191 | 10 | 0.65 |
| Ko | pg/ml | 3.5E2 | 2.9E2 | 5.0E2 | 7.7E2 | 5.1E2 | 1.2E3 | 1.0E-9 | 6.5E1 | 3.8E3 | 4.1E3 | 333 | 10 | 191 | 10 | 0.55 |
| Kp | pg/ml | 3.4E2 | 3.8E2 | 3.6E2 | 1.6E3 | 2.6E2 | 4.1E3 | 1.0E-9 | 3.7E1 | 1.7E3 | 1.3E4 | 333 | 10 | 191 | 10 | 0.53 |
| Kq | pg/ml | 3.3E2 | 1.3E3 | 4.7E2 | 1.8E4 | 7.6E2 | 5.0E4 | 1.6E0 | 1.7E2 | 9.8E3 | 1.6E5 | 325 | 10 | 185 | 10 | 0.81 |
| Kr | pg/ml | 4.5E-1 | 1.0E-9 | 2.4E0 | 4.2E1 | 4.6E0 | 1.3E2 | 1.0E-9 | 1.0E-9 | 3.9E1 | 4.2E2 | 325 | 10 | 185 | 10 | 0.39 |
| Ks | pg/ml | 1.4E4 | 1.7E4 | 2.0E4 | 1.9E4 | 1.8E4 | 1.7E4 | 5.1E1 | 1.3E3 | 1.1E5 | 5.0E4 | 325 | 10 | 185 | 10 | 0.49 |
| Ps | ng/ml | 1.6E2 | 9.4E2 | 6.2E2 | 1.3E3 | 1.8E3 | 1.1E3 | 4.1E-1 | 4.5E2 | 1.2E4 | 3.8E3 | 113 | 7 | 87 | 7 | 0.88 |
| Kx | ng/ml | 1.1E-4 | 1.2E-2 | 6.7E-3 | 1.8E-2 | 1.3E-2 | 2.3E-2 | 1.0E-9 | 1.0E-9 | 1.0E-1 | 6.4E-2 | 330 | 10 | 190 | 10 | 0.72 |
| Ky | ng/ml | 9.8E-2 | 7.4E-1 | 3.9E-1 | 8.4E-1 | 8.5E-1 | 8.1E-1 | 1.0E-9 | 3.0E-2 | 6.3E0 | 2.7E0 | 330 | 10 | 190 | 10 | 0.76 |
| Kz | ng/ml | 1.0E-9 | 1.4E-2 | 3.4E-3 | 8.9E-3 | 5.6E-3 | 7.8E-3 | 1.0E-9 | 1.0E-9 | 3.6E-2 | 1.8E-2 | 330 | 10 | 190 | 10 | 0.68 |
| Rz | ng/ml | 3.1E-1 | 3.3E-1 | 1.0E0 | 1.1E0 | 1.5E0 | 1.5E0 | 3.6E-3 | 1.0E-1 | 6.7E0 | 4.2E0 | 113 | 7 | 87 | 7 | 0.59 |
| Ry | ng/ml | 1.6E-2 | 1.6E-2 | 2.2E-2 | 6.7E-2 | 2.2E-2 | 1.3E-1 | 1.0E-9 | 1.6E-2 | 1.2E-1 | 3.5E-1 | 113 | 7 | 87 | 7 | 0.59 |
| Rx | ng/ml | 1.0E-9 | 3.5E-5 | 1.8E-3 | 1.7E-3 | 3.2E-3 | 3.2E-3 | 1.0E-9 | 1.0E-9 | 2.0E-2 | 8.4E-3 | 113 | 7 | 87 | 7 | 0.51 |
| Ld | pg/ml | 1.0E-9 | 7.5E-1 | 3.6E0 | 5.5E0 | 8.6E0 | 8.5E0 | 1.0E-9 | 1.0E-9 | 8.4E1 | 2.3E1 | 331 | 10 | 190 | 10 | 0.58 |
| Lh | pg/ml | 1.3E4 | 4.0E4 | 2.1E4 | 8.5E4 | 2.6E4 | 1.2E5 | 1.0E-9 | 1.8E3 | 2.6E5 | 4.1E5 | 932 | 16 | 345 | 16 | 0.75 |
| Li | pg/ml | 3.5E3 | 1.7E4 | 1.7E4 | 6.1E4 | 6.2E4 | 8.6E4 | 1.0E-9 | 3.7E1 | 1.3E6 | 3.1E5 | 932 | 16 | 345 | 16 | 0.71 |
| Lj | pg/ml | 2.8E3 | 1.3E4 | 2.3E4 | 4.8E4 | 6.4E4 | 9.7E4 | 1.0E-9 | 2.4E2 | 5.2E5 | 3.9E5 | 932 | 16 | 345 | 16 | 0.69 |
| Lp | pg/ml | 9.5E0 | 1.8E1 | 8.2E1 | 2.0E2 | 2.1E2 | 3.8E2 | 1.0E-9 | 1.0E-9 | 1.4E3 | 1.0E3 | 113 | 7 | 87 | 7 | 0.53 |
| Lt | pg/ml | 1.0E-9 | 1.0E-9 | 2.4E0 | 1.0E-9 | 8.8E0 | 0.0E0 | 1.0E-9 | 1.0E-9 | 6.0E1 | 1.0E-9 | 113 | 7 | 87 | 7 | 0.45 |
| Rv | ng/ml | 5.0E-4 | 1.0E-9 | 1.3E-3 | 2.1E-3 | 2.2E-3 | 4.6E-3 | 1.0E-9 | 1.0E-9 | 1.6E-2 | 1.2E-2 | 113 | 7 | 87 | 7 | 0.41 |
| Ru | ng/ml | 1.0E-9 | 1.0E-9 | 1.7E-2 | 5.8E-2 | 7.0E-2 | 1.3E-1 | 1.0E-9 | 1.0E-9 | 3.8E-1 | 3.4E-1 | 113 | 7 | 87 | 7 | 0.60 |
| Rt | ng/ml | 6.2E-2 | 3.6E-2 | 1.1E-1 | 1.2E0 | 1.4E-1 | 2.8E0 | 1.0E-3 | 8.6E-3 | 6.3E-1 | 7.4E0 | 113 | 7 | 87 | 7 | 0.53 |
| Yl | pg/ml | 1.1E1 | 4.0E1 | 1.7E1 | 5.8E1 | 1.8E1 | 7.3E1 | 1.0E-9 | 1.0E-9 | 1.1E2 | 2.2E2 | 116 | 7 | 90 | 7 | 0.76 |
| Rm | ng/ml | 1.9E1 | 8.6E1 | 5.2E1 | 8.9E1 | 8.1E1 | 8.1E1 | 2.2E-1 | 3.9E-1 | 6.5E2 | 2.5E2 | 326 | 10 | 190 | 10 | 0.70 |
| Rh | ng/ml | 1.3E2 | 1.5E2 | 3.5E2 | 1.9E3 | 1.2E3 | 5.3E3 | 3.6E0 | 2.8E1 | 1.7E4 | 1.7E4 | 326 | 10 | 190 | 10 | 0.56 |
| Ri | ng/ml | 1.0E-9 | 1.0E-9 | 4.3E0 | 4.1E-2 | 1.5E1 | 1.3E-1 | 1.0E-9 | 1.0E-9 | 2.2E2 | 4.1E-1 | 327 | 10 | 191 | 10 | 0.30 |
| Rg | ng/ml | 1.0E-9 | 1.0E-9 | 6.7E-2 | 6.4E-2 | 4.1E-1 | 1.9E-1 | 1.0E-9 | 1.0E-9 | 4.6E0 | 5.9E-1 | 326 | 10 | 190 | 10 | 0.50 |
| Rj | ng/ml | 1.0E-9 | 1.0E-9 | 1.3E0 | 2.7E1 | 5.2E0 | 8.5E1 | 1.0E-9 | 1.0E-9 | 8.0E1 | 2.7E2 | 327 | 10 | 191 | 10 | 0.47 |
| Rf | ng/ml | 3.9E-1 | 1.5E0 | 9.7E-1 | 4.1E0 | 1.7E0 | 6.0E0 | 7.8E-3 | 1.8E-1 | 1.5E1 | 1.7E1 | 326 | 10 | 190 | 10 | 0.76 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ql | pg/ml | 4.5E0 | 1.4E1 | 1.4E1 | 3.0E1 | 2.9E1 | 3.1E1 | 1.0E-9 | 4.3E-1 | 2.9E2 | 9.3E1 | 332 | 10 | 193 | 10 | 0.73 |
| Qm | pg/ml | 3.2E0 | 2.0E1 | 2.0E1 | 2.7E1 | 3.7E1 | 2.3E1 | 1.0E-9 | 1.0E-9 | 3.6E2 | 6.9E1 | 332 | 10 | 193 | 10 | 0.67 |
| Qn | pg/ml | 6.1E-1 | 8.6E-1 | 8.0E0 | 3.6E0 | 2.6E1 | 4.7E0 | 1.0E-9 | 1.0E-9 | 2.4E2 | 1.2E1 | 332 | 10 | 193 | 10 | 0.59 |
| Nv | pg/ml | 4.0E3 | 1.3E4 | 1.1E4 | 2.7E4 | 4.2E4 | 3.0E4 | 1.0E-9 | 1.3E3 | 1.1E6 | 9.4E4 | 939 | 16 | 345 | 16 | 0.72 |
| Nw | pg/ml | 8.9E3 | 2.4E4 | 1.3E4 | 5.2E4 | 1.6E4 | 6.7E4 | 8.6E1 | 6.2E3 | 2.1E5 | 2.2E5 | 939 | 16 | 345 | 16 | 0.81 |
| Nx | pg/ml | 2.2E2 | 7.5E2 | 4.1E2 | 9.4E2 | 6.4E2 | 1.1E3 | 1.0E-9 | 1.0E-9 | 7.4E3 | 4.1E3 | 939 | 16 | 345 | 16 | 0.68 |
| Ny | pg/ml | 6.2E0 | 2.9E1 | 5.2E1 | 2.8E2 | 8.3E2 | 6.9E2 | 1.0E-9 | 1.0E-9 | 2.5E4 | 2.8E3 | 939 | 16 | 345 | 16 | 0.80 |
| Oa | pg/ml | 1.8E2 | 4.8E2 | 4.6E2 | 9.3E2 | 7.3E2 | 9.0E2 | 1.0E-9 | 6.5E1 | 4.8E3 | 2.4E3 | 332 | 10 | 193 | 10 | 0.71 |
| Op | pg/ml | 4.1E5 | 4.9E5 | 4.1E5 | 4.5E5 | 1.6E5 | 2.0E5 | 3.3E4 | 9.4E4 | 7.3E5 | 7.5E5 | 113 | 7 | 87 | 7 | 0.58 |
| Oc | pg/ml | 7.1E1 | 4.7E0 | 2.9E2 | 1.7E2 | 7.4E2 | 3.3E2 | 1.0E-9 | 1.0E-9 | 1.9E4 | 1.1E3 | 930 | 16 | 344 | 16 | 0.42 |
| Of | pg/ml | 1.8E2 | 6.4E1 | 5.9E3 | 6.6E3 | 2.8E4 | 1.7E4 | 1.0E-9 | 3.5E0 | 6.2E5 | 6.6E4 | 938 | 16 | 345 | 16 | 0.45 |
| Og | pg/ml | 8.2E-2 | 2.6E-2 | 7.1E-1 | 6.2E-2 | 4.7E0 | 8.7E-1 | 1.0E-9 | 1.0E-9 | 8.3E1 | 3.2E-1 | 938 | 16 | 345 | 16 | 0.35 |
| Oh | pg/ml | 2.6E0 | 1.5E1 | 1.8E1 | 1.1E2 | 1.4E2 | 2.8E2 | 1.0E-9 | 1.1E0 | 3.5E3 | 1.1E3 | 938 | 16 | 345 | 16 | 0.77 |
| Oi | pg/ml | 2.6E0 | 1.0E-9 | 6.3E0 | 4.2E0 | 9.8E0 | 8.1E0 | 1.0E-9 | 1.0E-9 | 8.6E1 | 3.1E1 | 938 | 16 | 345 | 16 | 0.38 |
| Ok | pg/ml | 3.9E2 | 1.1E3 | 5.4E2 | 1.9E3 | 5.7E2 | 2.0E3 | 1.3E1 | 1.8E2 | 7.0E3 | 7.8E3 | 938 | 16 | 345 | 16 | 0.79 |
| Om | pg/ml | 3.9E2 | 1.6E3 | 8.2E2 | 5.3E3 | 2.1E3 | 1.2E4 | 1.0E-9 | 2.5E2 | 3.6E4 | 5.1E4 | 938 | 16 | 345 | 16 | 0.79 |
| On | pg/ml | 1.8E2 | 9.3E2 | 2.8E2 | 1.5E3 | 3.9E2 | 2.1E3 | 1.0E-9 | 2.6E1 | 4.5E3 | 8.5E3 | 938 | 16 | 345 | 16 | 0.80 |
| Or | pg/ml | 1.4E1 | 1.1E2 | 3.5E1 | 1.5E2 | 6.6E1 | 1.6E2 | 1.0E-9 | 1.0E-9 | 5.0E2 | 5.1E2 | 334 | 10 | 191 | 10 | 0.77 |
| Ow | pg/ml | 3.3E1 | 1.1E2 | 1.2E2 | 4.9E2 | 3.3E2 | 9.3E2 | 1.0E-9 | 1.8E1 | 3.2E3 | 3.0E3 | 334 | 10 | 191 | 10 | 0.72 |
| Ou | pg/ml | 4.8E2 | 8.3E2 | 9.8E2 | 3.7E3 | 1.6E3 | 4.2E3 | 1.0E-9 | 3.4E2 | 9.8E3 | 1.1E4 | 334 | 10 | 191 | 10 | 0.71 |
| Ul | ng/ml | 1.0E-9 | 1.0E-9 | 1.6E0 | 8.9E0 | 7.5E0 | 1.9E1 | 1.0E-9 | 1.0E-9 | 1.0E2 | 5.6E1 | 340 | 10 | 195 | 10 | 0.56 |
| Uo | ng/ml | 1.0E-9 | 1.0E-9 | 9.9E-2 | 1.0E-1 | 2.6E-1 | 2.0E-1 | 1.0E-9 | 1.0E-9 | 2.6E0 | 6.3E-1 | 340 | 10 | 195 | 10 | 0.55 |
| Uk | ng/ml | 1.0E-9 | 1.0E-9 | 7.2E-3 | 1.4E-3 | 2.4E-2 | 3.4E-3 | 1.0E-9 | 1.0E-9 | 3.2E-1 | 1.1E-2 | 340 | 10 | 195 | 10 | 0.39 |
| Um | ng/ml | 1.0E-9 | 1.0E-9 | 4.0E-1 | 3.0E-1 | 9.5E-1 | 7.1E-1 | 1.0E-9 | 1.0E-9 | 7.2E0 | 2.3E0 | 340 | 10 | 195 | 10 | 0.47 |
| Uf | ng/ml | 6.0E-2 | 2.1E-1 | 1.4E-1 | 7.2E-1 | 2.7E-1 | 1.7E0 | 1.0E-3 | 3.6E-2 | 2.5E0 | 5.6E0 | 340 | 10 | 195 | 10 | 0.72 |
| Uh | ng/ml | 2.0E0 | 3.6E0 | 3.2E0 | 5.7E0 | 3.4E0 | 5.8E0 | 1.3E-2 | 7.1E-1 | 1.8E1 | 1.7E1 | 340 | 10 | 195 | 10 | 0.65 |
| Un | ng/ml | 1.9E0 | 3.6E0 | 2.2E0 | 5.7E0 | 1.3E0 | 7.1E0 | 1.3E-1 | 1.8E0 | 8.0E0 | 2.5E1 | 340 | 10 | 195 | 10 | 0.78 |
| Ug | ng/ml | 1.5E1 | 9.6E0 | 2.8E1 | 1.8E1 | 3.0E1 | 2.5E1 | 6.9E-1 | 1.7E0 | 2.1E2 | 8.5E1 | 340 | 10 | 195 | 10 | 0.39 |
| Ur | ng/ml | 1.6E-1 | 1.0E-9 | 7.6E-1 | 7.8E-1 | 5.2E0 | 2.3E0 | 1.0E-9 | 1.0E-9 | 9.3E1 | 7.3E0 | 339 | 10 | 194 | 10 | 0.31 |
| Up | ng/ml | 1.0E-9 | 1.0E-9 | 5.0E-3 | 2.4E-1 | 2.2E-2 | 7.5E-1 | 1.0E-9 | 1.0E-9 | 3.3E-1 | 2.4E0 | 339 | 10 | 194 | 10 | 0.55 |
| Us | ng/ml | 3.5E-3 | 1.0E-9 | 1.9E-2 | 1.7E-1 | 4.4E-2 | 5.2E-1 | 1.0E-9 | 1.0E-9 | 4.0E-1 | 1.7E0 | 339 | 10 | 194 | 10 | 0.39 |
| Uv | ng/ml | 2.7E-3 | 4.2E-3 | 1.1E-2 | 6.9E-2 | 3.8E-2 | 1.4E-1 | 1.0E-9 | 1.0E-9 | 4.3E-1 | 4.1E-1 | 339 | 10 | 194 | 10 | 0.58 |
| Ut | ng/ml | 6.3E-1 | 2.8E0 | 2.6E0 | 1.3E1 | 8.0E0 | 2.1E1 | 1.0E-9 | 1.0E-9 | 7.8E1 | 6.5E1 | 339 | 10 | 194 | 10 | 0.75 |
| Uu | ng/ml | 7.1E0 | 5.6E0 | 8.1E0 | 5.9E0 | 5.8E0 | 3.8E0 | 4.5E-1 | 1.2E0 | 4.0E1 | 1.2E1 | 339 | 10 | 194 | 10 | 0.40 |
| Uw | ng/ml | 2.3E0 | 5.8E0 | 3.0E0 | 9.0E0 | 3.8E0 | 1.4E1 | 1.0E-9 | 1.1E0 | 3.7E1 | 3.9E1 | 122 | 7 | 96 | 7 | 0.70 |
| Vb | ng/ml | 1.0E0 | 1.3E0 | 1.0E0 | 1.8E0 | 4.4E-1 | 2.1E0 | 8.5E-2 | 3.5E-1 | 2.5E0 | 6.4E0 | 122 | 7 | 96 | 7 | 0.60 |
| Vc | ng/ml | 1.0E-9 | 1.0E-9 | 7.1E-3 | 1.0E-9 | 6.0E-2 | 0.0E0 | 1.0E-9 | 1.0E-9 | 6.5E-1 | 1.0E-9 | 122 | 7 | 96 | 7 | 0.48 |
| Uy | ng/ml | 1.3E0 | 7.0E-1 | 4.9E0 | 1.3E1 | 1.3E1 | 2.1E1 | 3.1E-2 | 2.0E-2 | 9.9E1 | 4.6E1 | 122 | 7 | 96 | 7 | 0.44 |
| Uz | ng/ml | 1.0E-9 | 1.0E-9 | 6.2E-3 | 4.7E0 | 4.8E-2 | 1.2E1 | 1.0E-9 | 1.0E-9 | 4.4E-1 | 3.3E1 | 122 | 7 | 96 | 7 | 0.56 |
| Ux | ng/ml | 1.7E2 | 1.9E2 | 1.9E2 | 2.2E2 | 1.4E2 | 1.1E2 | 4.5E0 | 1.0E2 | 5.3E2 | 3.9E2 | 122 | 7 | 96 | 7 | 0.59 |
| Va | ng/ml | 1.7E1 | 3.1E0 | 2.6E1 | 1.4E1 | 2.7E1 | 2.8E1 | 1.2E-1 | 1.2E0 | 1.2E2 | 7.8E1 | 122 | 7 | 96 | 7 | 0.30 |
| Vh | ng/ml | 1.0E-2 | 1.8E-2 | 1.6E-2 | 1.3E-1 | 2.0E-2 | 3.2E-1 | 1.0E-9 | 2.2E-3 | 1.2E-1 | 8.6E-1 | 122 | 7 | 96 | 7 | 0.61 |
| Vi | ng/ml | 3.1E-3 | 4.3E-2 | 1.2E-1 | 2.9E-1 | 1.2E0 | 6.8E-1 | 1.0E-9 | 2.0E-4 | 1.4E1 | 1.8E0 | 122 | 7 | 96 | 7 | 0.75 |
| Vj | ng/ml | 2.5E1 | 8.6E1 | 1.9E2 | 8.4E1 | 9.0E2 | 5.4E1 | 1.4E0 | 1.3E1 | 8.4E3 | 1.7E2 | 120 | 7 | 94 | 7 | 0.70 |
| Vp | ng/ml | 1.0E-9 | 3.6E-2 | 3.5E-1 | 5.0E0 | 3.1E0 | 1.6E1 | 1.0E-9 | 1.0E-9 | 5.0E1 | 4.9E1 | 340 | 10 | 195 | 10 | 0.67 |
| Vt | ng/ml | 6.7E0 | 9.5E0 | 9.4E0 | 2.9E1 | 9.8E0 | 4.7E1 | 4.3E-1 | 1.8E0 | 8.6E1 | 1.6E2 | 340 | 10 | 195 | 10 | 0.63 |
| Vu | ng/ml | 1.0E-9 | 3.1E0 | 2.3E0 | 4.8E0 | 6.4E0 | 5.1E0 | 1.0E-9 | 1.0E-9 | 7.1E1 | 1.4E1 | 334 | 8 | 195 | 8 | 0.72 |
| Vq | ng/ml | 2.0E2 | 1.0E3 | 3.4E3 | 2.0E3 | 4.2E4 | 2.6E3 | 2.0E-1 | 1.0E1 | 6.8E5 | 7.1E3 | 255 | 8 | 158 | 8 | 0.71 |
| Vo | ng/ml | 2.5E1 | 2.6E1 | 2.4E1 | 2.4E1 | 5.2E0 | 6.0E0 | 2.4E0 | 1.1E1 | 4.8E1 | 3.1E1 | 340 | 10 | 195 | 10 | 0.50 |
| Vs | ng/ml | 1.0E-9 | 1.4E0 | 6.1E0 | 5.9E1 | 2.2E1 | 1.6E2 | 1.0E-9 | 1.0E-9 | 2.3E2 | 4.5E2 | 325 | 8 | 190 | 8 | 0.60 |
| Vv | ng/ml | 3.0E0 | 2.6E0 | 5.9E0 | 6.7E0 | 9.5E0 | 8.0E0 | 1.0E-9 | 1.0E-9 | 8.4E1 | 2.1E1 | 339 | 9 | 195 | 9 | 0.53 |
| Vw | ng/ml | 3.6E1 | 4.6E1 | 3.5E1 | 4.8E1 | 1.7E1 | 1.3E1 | 2.5E0 | 3.0E1 | 7.0E1 | 6.6E1 | 122 | 7 | 96 | 7 | 0.73 |
| Oy | pg/ml | 4.9E-1 | 3.7E-1 | 5.7E0 | 3.5E0 | 2.8E1 | 8.6E0 | 1.0E-9 | 1.0E-9 | 4.0E2 | 3.3E1 | 937 | 16 | 344 | 16 | 0.45 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Oz | pg/ml | 1.2E-2 | 1.0E-9 | 3.2E-1 | 1.8E0 | 1.3E0 | 7.0E0 | 1.0E-9 | 1.0E-9 | 2.9E1 | 2.8E1 | 937 | 16 | 344 | 16 | 0.40 |
| Pa | pg/ml | 3.9E-1 | 4.2E-1 | 1.4E0 | 1.6E1 | 4.9E0 | 5.6E1 | 1.0E-9 | 1.0E-9 | 8.6E1 | 2.3E2 | 937 | 16 | 344 | 16 | 0.55 |
| Pb | pg/ml | 1.0E-9 | 1.0E-9 | 7.4E-1 | 2.1E0 | 1.6E1 | 7.9E0 | 1.0E-9 | 1.0E-9 | 4.9E2 | 3.2E1 | 937 | 16 | 344 | 16 | 0.42 |
| Pc | pg/ml | 5.4E-2 | 1.0E-9 | 3.5E-1 | 2.9E0 | 8.4E-1 | 9.3E0 | 1.0E-9 | 1.0E-9 | 1.6E1 | 3.8E1 | 937 | 16 | 344 | 16 | 0.47 |
| Pd | pg/ml | 1.8E0 | 4.8E0 | 4.8E0 | 1.6E1 | 2.8E1 | 3.0E1 | 1.0E-9 | 7.3E-2 | 8.4E2 | 1.2E2 | 937 | 16 | 344 | 16 | 0.66 |
| Pe | pg/ml | 2.2E1 | 1.1E2 | 1.0E2 | 1.6E3 | 3.4E2 | 3.9E3 | 1.0E-9 | 2.0E1 | 4.7E3 | 1.5E4 | 937 | 16 | 344 | 16 | 0.78 |
| Pf | pg/ml | 1.6E0 | 1.6E1 | 1.0E1 | 5.8E1 | 5.7E1 | 1.1E2 | 1.0E-9 | 3.3E-1 | 1.5E3 | 4.3E2 | 937 | 16 | 344 | 16 | 0.79 |
| Pg | pg/ml | 3.5E0 | 1.6E1 | 4.1E1 | 1.7E2 | 3.3E2 | 3.5E2 | 1.0E-9 | 4.6E-1 | 7.7E3 | 1.2E3 | 937 | 16 | 344 | 16 | 0.71 |
| Ph | ng/ml | 1.8E-1 | 2.4E-1 | 3.5E-1 | 8.1E-1 | 5.5E-1 | 1.6E0 | 1.0E-9 | 3.5E-3 | 4.4E0 | 5.4E0 | 334 | 10 | 191 | 10 | 0.58 |
| Pi | ng/ml | 2.0E-1 | 4.3E-1 | 2.9E-1 | 8.5E0 | 4.2E-1 | 2.6E1 | 1.0E-9 | 1.0E-9 | 4.8E0 | 8.2E1 | 334 | 10 | 191 | 10 | 0.72 |
| Pj | ng/mL | 5.4E0 | 7.1E0 | 6.2E0 | 6.9E0 | 5.2E0 | 4.6E0 | 3.8E-2 | 1.4E0 | 5.5E1 | 1.7E1 | 334 | 10 | 191 | 10 | 0.56 |
| Pk | ng/mL | 8.9E-3 | 2.2E-2 | 1.3E-2 | 1.7E-1 | 2.0E-2 | 4.8E-1 | 1.0E-9 | 1.1E-3 | 2.5E-1 | 1.5E0 | 334 | 10 | 191 | 10 | 0.68 |
| aA | mg/dL | 8.1E-1 | 1.6E0 | 9.5E-1 | 2.1E0 | 4.9E-1 | 1.3E0 | 2.0E-1 | 5.5E-1 | 4.2E0 | 4.7E0 | 2722 | 21 | 517 | 21 | 0.80 |
| aC | mg/mL | 2.8E0 | 2.0E0 | 3.1E0 | 2.6E0 | 1.4E0 | 1.5E0 | 7.7E-1 | 7.4E-1 | 8.9E0 | 5.5E0 | 551 | 11 | 213 | 11 | 0.38 |
| aD | ug/mL | 3.1E0 | 6.0E0 | 4.4E0 | 7.7E0 | 3.9E0 | 6.6E0 | 4.3E-1 | 1.1E0 | 3.5E1 | 2.1E1 | 551 | 11 | 213 | 11 | 0.62 |
| aE | mg/mL | 5.6E-1 | 5.8E-1 | 5.7E-1 | 6.3E-1 | 1.5E-1 | 2.5E-1 | 1.8E-1 | 3.9E-1 | 1.1E0 | 1.2E0 | 551 | 11 | 213 | 11 | 0.54 |
| aF | ng/mL | 2.2E0 | 1.6E0 | 4.0E0 | 5.0E0 | 5.7E0 | 5.0E0 | 4.3E-3 | 5.2E-1 | 5.0E1 | 1.3E1 | 551 | 11 | 213 | 11 | 0.52 |
| aG | mg/mL | 1.4E-1 | 9.4E-2 | 1.6E-1 | 1.5E-1 | 8.7E-2 | 1.1E-1 | 1.7E-2 | 7.0E-2 | 5.4E-1 | 4.2E-1 | 551 | 11 | 213 | 11 | 0.44 |
| aH | ug/mL | 7.5E1 | 6.3E1 | 8.2E1 | 7.0E1 | 4.4E1 | 4.3E1 | 4.6E0 | 1.1E1 | 2.9E2 | 1.5E2 | 551 | 11 | 213 | 11 | 0.42 |
| aI | ug/mL | 1.9E2 | 1.6E2 | 1.9E2 | 1.5E2 | 6.0E1 | 5.5E1 | 2.8E1 | 7.5E1 | 3.7E2 | 2.4E2 | 551 | 11 | 213 | 11 | 0.34 |
| aJ | ug/mL | 2.5E0 | 5.6E0 | 3.1E0 | 7.5E0 | 2.2E0 | 6.1E0 | 7.3E-1 | 1.5E0 | 1.7E1 | 2.3E1 | 551 | 11 | 213 | 11 | 0.78 |
| aK | ng/mL | 1.5E0 | 1.4E0 | 2.4E0 | 2.0E0 | 2.6E0 | 1.9E0 | 2.9E-4 | 1.3E-1 | 1.8E1 | 5.5E0 | 551 | 11 | 213 | 11 | 0.48 |
| aL | mg/mL | 8.0E-1 | 8.0E-1 | 8.1E-1 | 7.5E-1 | 2.6E-1 | 2.5E-1 | 1.9E-1 | 2.7E-1 | 1.7E0 | 1.0E0 | 551 | 11 | 213 | 11 | 0.46 |
| aM | U/mL | 2.2E1 | 5.3E1 | 4.6E1 | 1.2E2 | 9.3E1 | 2.3E2 | 4.2E-2 | 5.2E0 | 1.6E3 | 8.2E2 | 551 | 11 | 213 | 11 | 0.69 |
| aN | U/mL | 1.4E1 | 2.6E1 | 2.2E1 | 3.3E1 | 3.0E1 | 3.2E1 | 2.5E-3 | 1.9E0 | 3.8E2 | 1.1E2 | 551 | 11 | 213 | 11 | 0.62 |
| aO | pg/mL | 3.3E1 | 2.4E2 | 3.0E2 | 6.4E2 | 7.7E2 | 8.7E2 | 6.0E-2 | 2.1E0 | 6.6E3 | 2.4E3 | 551 | 11 | 213 | 11 | 0.69 |
| aP | ng/mL | 1.7E0 | 3.2E0 | 2.1E0 | 5.6E0 | 1.8E0 | 7.6E0 | 4.5E-1 | 1.4E0 | 2.8E1 | 2.8E1 | 551 | 11 | 213 | 11 | 0.78 |
| aQ | ng/mL | 3.0E-1 | 4.4E-1 | 4.5E-1 | 3.6E-1 | 4.5E-1 | 1.8E-1 | 2.0E-4 | 5.1E-2 | 4.0E0 | 6.2E-1 | 551 | 11 | 213 | 11 | 0.51 |
| aR | ng/mL | 1.8E0 | 3.1E0 | 2.8E0 | 3.0E0 | 3.3E0 | 1.9E0 | 1.8E-1 | 2.5E-1 | 3.4E1 | 5.9E0 | 551 | 11 | 213 | 11 | 0.59 |
| aS | ng/mL | 2.7E-1 | 4.0E-1 | 6.3E-1 | 9.6E-1 | 1.7E0 | 1.0E0 | 4.2E-3 | 8.5E-2 | 3.3E1 | 2.8E0 | 551 | 11 | 213 | 11 | 0.58 |
| aU | pg/mL | 7.5E1 | 6.8E1 | 1.2E2 | 8.8E1 | 1.5E2 | 7.8E1 | 7.4E-2 | 9.6E0 | 1.3E3 | 2.3E2 | 551 | 11 | 213 | 11 | 0.44 |
| aV | ng/mL | 6.2E-1 | 4.2E-1 | 1.0E0 | 1.1E0 | 1.8E0 | 1.5E0 | 7.6E-4 | 1.0E-1 | 3.3E1 | 5.4E0 | 551 | 11 | 213 | 11 | 0.49 |
| aW | pg/mL | 1.8E1 | 2.1E1 | 1.9E1 | 5.5E1 | 1.8E1 | 1.2E2 | 7.2E-2 | 7.7E0 | 2.4E2 | 4.2E2 | 551 | 11 | 213 | 11 | 0.57 |
| aX | ng/mL | 9.5E0 | 1.4E1 | 1.6E1 | 3.7E1 | 2.6E1 | 5.7E1 | 3.0E-1 | 2.6E0 | 3.1E2 | 1.7E2 | 551 | 11 | 213 | 11 | 0.59 |
| aY | pg/mL | 5.7E1 | 9.2E1 | 7.5E1 | 1.1E2 | 8.2E1 | 6.7E1 | 4.1E-1 | 1.2E1 | 1.2E3 | 2.0E2 | 551 | 11 | 213 | 11 | 0.70 |
| aZ | pg/mL | 2.2E2 | 4.2E2 | 5.4E2 | 4.5E2 | 1.1E3 | 3.0E2 | 1.7E0 | 8.2E1 | 1.2E4 | 1.3E3 | 551 | 11 | 213 | 11 | 0.64 |
| bA | ng/mL | 9.0E0 | 1.6E2 | 3.5E1 | 4.0E2 | 9.2E1 | 5.5E2 | 3.0E-2 | 2.0E0 | 9.7E2 | 1.5E3 | 551 | 11 | 213 | 11 | 0.84 |
| bB | ng/mL | 3.0E2 | 1.9E2 | 3.2E2 | 2.2E2 | 1.7E2 | 1.4E2 | 2.1E0 | 3.3E1 | 1.0E3 | 4.2E2 | 551 | 11 | 213 | 11 | 0.33 |
| bC | ng/mL | 3.4E2 | 4.3E2 | 6.0E2 | 1.2E3 | 7.9E2 | 1.4E3 | 9.8E0 | 1.4E2 | 4.7E3 | 4.7E3 | 551 | 11 | 213 | 11 | 0.65 |
| bE | mg/mL | 5.5E0 | 5.9E0 | 5.8E0 | 6.7E0 | 2.1E0 | 3.2E0 | 9.8E-1 | 1.3E0 | 1.3E1 | 1.2E1 | 551 | 11 | 213 | 11 | 0.58 |
| bF | pg/mL | 2.0E1 | 5.7E1 | 1.5E2 | 7.9E2 | 8.8E2 | 1.9E3 | 5.0E-2 | 1.5E1 | 1.1E4 | 6.3E3 | 551 | 11 | 213 | 11 | 0.74 |
| bG | ng/mL | 1.6E0 | 1.7E0 | 2.7E0 | 4.6E0 | 3.2E0 | 8.4E0 | 2.2E-2 | 5.8E-1 | 2.6E1 | 3.0E1 | 551 | 11 | 213 | 11 | 0.56 |
| bH | pg/mL | 5.7E-1 | 9.2E0 | 4.7E0 | 8.8E0 | 1.4E1 | 7.9E0 | 5.7E-1 | 5.7E-1 | 2.8E2 | 2.2E1 | 551 | 11 | 213 | 11 | 0.70 |
| bI | ng/mL | 4.0E-3 | 4.0E-3 | 6.0E-2 | 1.6E-1 | 1.5E-1 | 2.9E-1 | 4.0E-3 | 4.0E-3 | 1.3E0 | 8.8E-1 | 551 | 11 | 213 | 11 | 0.60 |
| bJ | mg/mL | 2.3E0 | 2.4E0 | 2.6E0 | 3.1E0 | 2.0E0 | 2.1E0 | 2.5E-4 | 2.5E-4 | 1.3E1 | 7.0E0 | 551 | 11 | 213 | 11 | 0.57 |
| bL | pg/mL | 3.8E0 | 3.2E0 | 8.5E0 | 9.0E0 | 1.1E1 | 1.0E1 | 4.6E-2 | 1.1E0 | 8.0E1 | 3.2E1 | 551 | 11 | 213 | 11 | 0.54 |
| bM | mg/mL | 1.7E0 | 2.3E0 | 2.1E0 | 3.3E0 | 1.4E0 | 2.3E0 | 9.2E-3 | 1.8E-2 | 8.9E0 | 8.4E0 | 551 | 11 | 213 | 11 | 0.68 |
| bN | ng/mL | 4.2E1 | 3.1E1 | 1.3E2 | 1.0E2 | 2.7E2 | 1.5E2 | 1.4E-1 | 1.4E0 | 1.9E3 | 4.8E2 | 551 | 11 | 213 | 11 | 0.45 |
| bO | ng/mL | 4.0E-2 | 4.0E-2 | 1.0E1 | 1.5E1 | 2.3E1 | 2.5E1 | 4.0E-2 | 4.0E-2 | 2.0E2 | 6.1E1 | 551 | 11 | 213 | 11 | 0.52 |
| bP | mg/mL | 5.3E-1 | 9.0E-1 | 7.6E-1 | 1.1E0 | 6.9E-1 | 9.0E-1 | 4.9E-2 | 2.9E-1 | 4.8E0 | 3.5E0 | 551 | 11 | 213 | 11 | 0.66 |
| bQ | pg/mL | 1.6E1 | 6.2E1 | 5.8E1 | 8.4E1 | 5.8E2 | 6.8E1 | 1.5E-1 | 1.2E1 | 1.3E4 | 2.2E2 | 551 | 11 | 213 | 11 | 0.84 |
| bR | ng/mL | 1.2E-2 | 1.2E-2 | 1.3E-1 | 4.5E-2 | 4.2E-1 | 8.2E-2 | 1.2E-2 | 1.2E-2 | 8.7E0 | 2.8E-1 | 551 | 11 | 213 | 11 | 0.38 |
| bS | pg/mL | 9.4E-1 | 9.4E-1 | 7.0E0 | 6.9E0 | 2.6E1 | 2.0E1 | 9.4E-1 | 9.4E-1 | 3.9E2 | 6.7E1 | 551 | 11 | 213 | 11 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bU | ng/mL | 1.2E-1 | 1.3E-2 | 1.9E-1 | 1.2E-1 | 3.5E-1 | 1.5E-1 | 1.3E-2 | 1.3E-2 | 6.6E0 | 4.1E-1 | 551 | 11 | 213 | 11 | 0.41 |
| bV | pg/mL | 4.7E2 | 1.1E3 | 5.9E2 | 1.0E3 | 8.9E2 | 4.7E2 | 1.5E2 | 3.6E2 | 1.7E4 | 2.0E3 | 551 | 11 | 213 | 11 | 0.82 |
| bW | pg/mL | 3.3E2 | 4.9E2 | 5.1E2 | 3.0E3 | 5.5E2 | 7.4E3 | 8.4E1 | 1.5E2 | 6.4E3 | 2.5E4 | 551 | 11 | 213 | 11 | 0.62 |
| bX | ng/mL | 2.5E-5 | 2.5E-5 | 2.7E-3 | 2.0E-3 | 3.4E-3 | 2.5E-3 | 2.5E-5 | 2.5E-5 | 2.0E-2 | 7.1E-3 | 551 | 11 | 213 | 11 | 0.45 |
| bZ | pg/mL | 2.5E2 | 8.9E2 | 8.5E2 | 5.2E3 | 3.7E3 | 1.2E4 | 1.5E-1 | 1.9E2 | 5.8E4 | 4.3E4 | 551 | 11 | 213 | 11 | 0.77 |
| cA | pg/mL | 6.0E-1 | 6.0E-1 | 2.7E0 | 6.0E-1 | 1.6E1 | 0.0E0 | 6.0E-1 | 6.0E-1 | 3.7E2 | 6.0E-1 | 551 | 11 | 213 | 11 | 0.43 |
| cB | ng/mL | 5.5E-2 | 6.4E-2 | 8.7E-2 | 6.6E-2 | 1.0E-1 | 6.7E-2 | 1.7E-3 | 1.7E-3 | 5.7E-1 | 2.1E-1 | 551 | 11 | 213 | 11 | 0.46 |
| cC | pg/mL | 4.6E1 | 5.4E1 | 4.7E1 | 4.6E1 | 3.9E1 | 2.5E1 | 1.0E0 | 1.0E0 | 4.5E2 | 7.3E1 | 551 | 11 | 213 | 11 | 0.54 |
| cD | pg/mL | 5.2E0 | 8.5E0 | 1.5E1 | 1.2E1 | 5.2E1 | 1.6E1 | 3.3E-1 | 3.3E-1 | 7.2E2 | 4.9E1 | 551 | 11 | 213 | 11 | 0.53 |
| cE | pg/mL | 3.9E1 | 2.4E2 | 1.5E2 | 3.8E2 | 4.3E2 | 4.6E2 | 1.2E-1 | 1.2E1 | 3.8E3 | 1.3E3 | 551 | 11 | 213 | 11 | 0.74 |
| cF | pg/mL | 1.3E1 | 5.3E-1 | 2.0E1 | 1.0E1 | 3.0E1 | 1.5E1 | 5.3E-1 | 5.3E-1 | 2.7E2 | 3.8E1 | 551 | 11 | 213 | 11 | 0.39 |
| cG | pg/mL | 4.6E1 | 1.2E2 | 1.1E2 | 1.9E2 | 4.7E2 | 1.7E2 | 6.4E0 | 3.5E1 | 1.0E4 | 4.9E2 | 551 | 11 | 213 | 11 | 0.74 |
| cH | uIU/mL | 2.8E0 | 1.4E0 | 6.0E0 | 1.1E1 | 1.1E1 | 1.8E1 | 8.6E-3 | 8.6E-3 | 1.6E2 | 5.3E1 | 551 | 11 | 213 | 11 | 0.45 |
| cI | ng/mL | 5.7E0 | 9.6E0 | 1.2E1 | 1.9E1 | 1.7E1 | 3.3E1 | 1.0E-3 | 1.2E0 | 1.2E2 | 1.2E2 | 551 | 11 | 213 | 11 | 0.56 |
| cJ | ug/mL | 6.2E1 | 5.0E1 | 1.1E2 | 6.5E1 | 1.4E2 | 5.9E1 | 4.0E0 | 5.6E0 | 9.6E2 | 1.9E2 | 551 | 11 | 213 | 11 | 0.41 |
| cK | ng/mL | 3.8E-3 | 3.8E-3 | 4.7E-2 | 2.5E-2 | 1.7E-1 | 3.1E-2 | 3.8E-3 | 3.8E-3 | 1.7E0 | 8.2E-2 | 551 | 11 | 213 | 11 | 0.59 |
| cL | pg/mL | 2.0E2 | 2.4E2 | 3.9E2 | 3.7E2 | 1.3E3 | 3.6E2 | 1.6E1 | 1.4E2 | 2.4E4 | 1.4E3 | 551 | 11 | 213 | 11 | 0.61 |
| cM | pg/mL | 2.7E2 | 2.3E2 | 2.9E2 | 2.4E2 | 1.9E2 | 1.3E2 | 8.7E0 | 5.7E1 | 1.6E3 | 4.7E2 | 551 | 11 | 213 | 11 | 0.42 |
| cN | pg/mL | 1.2E2 | 1.8E2 | 1.3E2 | 1.8E2 | 6.2E1 | 7.5E1 | 3.8E1 | 8.6E1 | 1.1E3 | 2.9E2 | 551 | 11 | 213 | 11 | 0.70 |
| cO | pg/mL | 2.2E2 | 3.1E2 | 3.0E2 | 3.1E2 | 8.3E2 | 9.0E1 | 5.4E1 | 1.6E2 | 1.9E4 | 4.4E2 | 551 | 11 | 213 | 11 | 0.70 |
| cP | ng/mL | 2.5E3 | 3.4E3 | 2.6E3 | 3.3E3 | 9.0E2 | 1.1E3 | 6.2E2 | 2.0E3 | 5.7E3 | 5.0E3 | 551 | 11 | 213 | 11 | 0.69 |
| cQ | ng/mL | 5.1E-2 | 6.7E-2 | 1.4E-1 | 2.0E-1 | 2.8E-1 | 3.0E-1 | 2.0E-3 | 2.0E-3 | 2.2E0 | 8.7E-1 | 551 | 11 | 213 | 11 | 0.58 |
| cR | ng/mL | 3.0E2 | 5.0E2 | 5.2E2 | 4.5E2 | 8.3E2 | 1.9E2 | 2.0E1 | 1.4E2 | 8.9E3 | 6.9E2 | 551 | 11 | 213 | 11 | 0.61 |
| cS | ng/mL | 2.6E2 | 7.2E2 | 4.4E2 | 1.3E3 | 1.0E3 | 2.0E3 | 4.1E1 | 1.6E2 | 2.2E4 | 7.1E3 | 551 | 11 | 213 | 11 | 0.77 |
| cT | ng/mL | 3.3E1 | 1.7E2 | 8.8E1 | 6.0E2 | 2.0E2 | 7.2E2 | 3.6E0 | 1.1E1 | 2.1E3 | 1.9E3 | 551 | 11 | 213 | 11 | 0.79 |
| cU | ng/mL | 5.4E1 | 8.8E1 | 7.7E1 | 1.1E2 | 9.6E1 | 6.7E1 | 5.4E0 | 4.0E1 | 1.6E3 | 2.3E2 | 551 | 11 | 213 | 11 | 0.71 |
| cV | ng/mL | 1.8E-1 | 2.8E-1 | 4.0E-1 | 5.3E-1 | 2.1E0 | 7.0E-1 | 3.4E-4 | 7.6E-2 | 4.7E1 | 2.5E0 | 551 | 11 | 213 | 11 | 0.67 |
| cW | mIU/mL | 5.2E-2 | 9.9E-2 | 1.3E-1 | 1.4E-1 | 6.5E-1 | 1.2E-1 | 3.7E-4 | 3.6E-2 | 9.7E0 | 3.9E-1 | 551 | 11 | 213 | 11 | 0.72 |
| cX | ng/mL | 1.1E-1 | 1.2E-1 | 1.3E0 | 4.2E-1 | 4.3E0 | 7.4E-1 | 2.3E-4 | 2.3E-4 | 2.8E1 | 2.5E0 | 551 | 11 | 213 | 11 | 0.47 |
| cY | ng/mL | 8.6E0 | 6.9E0 | 1.2E1 | 9.2E0 | 1.3E1 | 8.1E0 | 1.5E-1 | 9.3E-1 | 8.3E1 | 2.7E1 | 551 | 11 | 213 | 11 | 0.43 |
| cZ | ug/mL | 1.4E1 | 1.6E1 | 1.6E1 | 1.6E1 | 7.1E0 | 6.6E0 | 2.3E0 | 3.3E0 | 5.7E1 | 2.6E1 | 551 | 11 | 213 | 11 | 0.55 |
| dA | pg/mL | 3.3E2 | 5.4E2 | 3.7E2 | 5.5E2 | 2.8E2 | 2.6E2 | 9.0E1 | 1.7E2 | 5.8E3 | 1.1E3 | 551 | 11 | 213 | 11 | 0.73 |
| dB | ug/mL | 1.7E1 | 2.2E1 | 1.7E1 | 1.9E1 | 1.5E1 | 8.2E0 | 9.4E-1 | 3.7E0 | 2.5E2 | 2.7E1 | 551 | 11 | 213 | 11 | 0.64 |
| dC | nmol/L | 3.5E1 | 3.6E1 | 3.8E1 | 4.0E1 | 1.8E1 | 1.7E1 | 7.6E0 | 2.1E1 | 1.4E2 | 7.9E1 | 551 | 11 | 213 | 11 | 0.52 |
| dD | ug/mL | 3.6E1 | 3.0E1 | 3.7E1 | 3.6E1 | 1.1E1 | 1.5E1 | 1.3E1 | 2.1E1 | 7.6E1 | 6.4E1 | 551 | 11 | 213 | 11 | 0.43 |
| dE | ng/mL | 4.7E-1 | 7.0E-1 | 6.0E-1 | 8.9E-1 | 6.9E-1 | 8.6E-1 | 8.4E-3 | 8.4E-3 | 7.2E0 | 2.4E0 | 551 | 11 | 213 | 11 | 0.61 |
| dF | ng/mL | 2.3E2 | 6.7E2 | 2.8E2 | 5.7E2 | 1.9E2 | 3.3E2 | 5.6E1 | 2.0E2 | 1.3E3 | 1.2E3 | 551 | 11 | 213 | 11 | 0.79 |
| dG | ng/mL | 1.2E1 | 2.4E1 | 1.5E1 | 3.3E1 | 1.3E1 | 2.7E1 | 2.2E0 | 9.8E0 | 1.8E2 | 8.7E1 | 551 | 11 | 213 | 11 | 0.77 |
| dH | pg/mL | 7.9E0 | 1.4E1 | 1.3E1 | 1.8E1 | 3.6E1 | 2.1E1 | 4.0E-2 | 3.2E0 | 6.7E2 | 7.6E1 | 551 | 11 | 213 | 11 | 0.64 |
| dI | pg/mL | 4.6E-1 | 5.0E0 | 2.2E0 | 5.0E0 | 1.5E1 | 5.3E0 | 4.6E-1 | 4.6E-1 | 3.3E2 | 1.9E1 | 551 | 11 | 213 | 11 | 0.75 |
| dJ | ng/mL | 1.9E0 | 2.7E0 | 2.2E0 | 2.4E0 | 1.2E0 | 1.1E0 | 3.2E-2 | 4.9E-1 | 6.9E0 | 3.7E0 | 551 | 11 | 213 | 11 | 0.59 |
| dK | uIU/mL | 1.9E0 | 1.2E0 | 3.0E0 | 3.3E0 | 6.0E0 | 6.3E0 | 2.8E-4 | 1.4E-2 | 7.9E1 | 2.2E1 | 551 | 11 | 213 | 11 | 0.42 |
| dL | ng/mL | 8.8E2 | 1.3E3 | 1.0E3 | 1.7E3 | 5.2E2 | 1.3E3 | 2.6E2 | 5.3E2 | 3.8E3 | 4.8E3 | 551 | 11 | 213 | 11 | 0.62 |
| dM | pg/mL | 9.7E2 | 2.5E3 | 1.2E3 | 3.2E3 | 1.3E3 | 2.7E3 | 3.4E2 | 7.1E2 | 1.6E4 | 9.6E3 | 551 | 11 | 213 | 11 | 0.78 |
| dN | ug/mL | 9.3E1 | 1.7E2 | 9.9E1 | 1.7E2 | 3.7E1 | 7.0E1 | 1.6E1 | 9.4E1 | 2.8E2 | 3.3E2 | 551 | 11 | 213 | 11 | 0.86 |
| dR | pg/ml | 1.6E3 | 9.9E2 | 2.3E3 | 1.4E3 | 2.3E3 | 1.2E3 | 1.4E2 | 4.0E2 | 1.5E4 | 4.1E3 | 378 | 8 | 202 | 8 | 0.38 |
| eF | ng/ml | 4.1E0 | 8.9E0 | 5.0E0 | 1.1E1 | 4.3E0 | 8.2E0 | 1.2E0 | 3.4E0 | 4.6E1 | 2.9E1 | 393 | 8 | 203 | 8 | 0.83 |
| cC | pg/ml | 3.0E2 | 1.9E2 | 3.7E2 | 4.3E2 | 2.6E2 | 7.1E2 | 9.9E0 | 7.1E1 | 1.6E3 | 2.0E3 | 304 | 7 | 190 | 7 | 0.27 |
| fP | ng/ml | 2.6E2 | 4.0E2 | 2.9E2 | 3.2E2 | 1.9E2 | 1.4E2 | 1.8E0 | 9.5E1 | 1.6E3 | 4.4E2 | 358 | 8 | 193 | 8 | 0.58 |
| fR | ng/ml | 1.4E5 | 3.6E5 | 1.9E5 | 3.8E5 | 1.5E5 | 2.8E5 | 2.9E4 | 1.9E2 | 8.3E5 | 8.7E5 | 363 | 10 | 111 | 10 | 0.72 |
| gL | pg/ml | 6.4E4 | 1.0E5 | 7.1E4 | 1.2E5 | 3.2E4 | 6.1E4 | 1.1E4 | 4.5E4 | 3.2E5 | 2.2E5 | 378 | 8 | 202 | 8 | 0.77 |
| gP | U/ml | 2.7E2 | 2.7E2 | 2.8E2 | 3.0E2 | 1.1E2 | 8.8E1 | 1.2E1 | 1.9E2 | 1.1E3 | 4.4E2 | 389 | 8 | 203 | 8 | 0.57 |
| tF | pg/mL | 1.4E3 | 2.1E3 | 1.3E4 | 2.0E3 | 4.0E4 | 1.3E3 | 1.2E1 | 2.6E2 | 3.2E5 | 3.7E3 | 305 | 7 | 190 | 7 | 0.52 |

EP 3 070 474 A2

Figure 8.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ad | ug/mL | 4.1E-2 | 7.1E-2 | 7.5E-2 | 1.1E0 | 9.0E-2 | 3.0E0 | 2.7E-4 | 4.3E-3 | 5.4E-1 | 8.5E0 | 454 | 8 | 168 | 8 | 0.59 |
| Af | ng/mL | 1.1E0 | 4.4E-1 | 1.5E1 | 3.7E1 | 6.0E1 | 9.0E1 | 1.7E-3 | 1.7E-3 | 5.3E2 | 2.6E2 | 454 | 8 | 168 | 8 | 0.48 |
| Aj | ug/mL | 1.6E0 | 1.6E-1 | 2.6E0 | 1.0E0 | 2.4E0 | 2.0E0 | 1.5E-3 | 2.1E-3 | 6.1E0 | 5.8E0 | 454 | 8 | 168 | 8 | 0.29 |
| Al | mg/mL | 8.7E-5 | 8.4E-5 | 2.5E-4 | 1.3E-4 | 4.0E-4 | 1.3E-4 | 2.3E-6 | 3.9E-5 | 1.9E-3 | 4.4E-4 | 454 | 8 | 168 | 8 | 0.52 |
| An | U/mL | 5.1E1 | 8.1E1 | 1.8E2 | 1.2E3 | 4.4E2 | 2.7E3 | 9.8E-4 | 4.3E1 | 5.5E3 | 7.8E3 | 454 | 8 | 168 | 8 | 0.69 |
| Ao | pg/mL | 8.9E1 | 6.3E1 | 5.1E2 | 1.4E2 | 3.4E3 | 2.0E2 | 2.8E0 | 5.4E0 | 3.9E4 | 6.0E2 | 454 | 8 | 168 | 8 | 0.42 |
| Ap | ng/mL | 3.3E1 | 1.9E1 | 4.5E1 | 4.3E1 | 4.4E1 | 5.2E1 | 8.4E-5 | 8.4E0 | 2.9E2 | 1.6E2 | 454 | 8 | 168 | 8 | 0.43 |
| Ar | ng/mL | 9.7E-1 | 3.3E0 | 1.2E1 | 4.7E0 | 1.9E2 | 4.4E0 | 3.4E-3 | 5.0E-1 | 4.1E3 | 1.4E1 | 454 | 8 | 168 | 8 | 0.74 |
| As | ng/mL | 9.0E-3 | 1.1E-2 | 1.3E-2 | 1.6E-1 | 1.9E-2 | 4.3E-1 | 1.7E-3 | 1.7E-3 | 1.3E-1 | 1.2E0 | 454 | 8 | 168 | 8 | 0.54 |
| Aw | pg/mL | 1.6E1 | 1.8E1 | 1.6E1 | 2.2E1 | 6.1E0 | 1.3E1 | 2.9E-2 | 1.3E1 | 4.8E1 | 5.1E1 | 454 | 8 | 168 | 8 | 0.64 |
| Ax | ng/mL | 2.4E0 | 7.9E0 | 1.7E1 | 9.3E1 | 6.5E1 | 1.4E2 | 1.2E-2 | 1.7E0 | 7.7E2 | 3.7E2 | 454 | 8 | 168 | 8 | 0.73 |
| Ba | ng/mL | 7.0E1 | 3.8E2 | 4.4E2 | 1.3E3 | 1.2E3 | 1.8E3 | 3.7E-1 | 1.6E1 | 8.1E3 | 4.4E3 | 454 | 8 | 168 | 8 | 0.70 |
| Bb | ng/mL | 3.1E0 | 2.8E0 | 6.5E0 | 5.7E0 | 1.4E1 | 6.7E0 | 4.1E-3 | 6.8E-1 | 2.5E2 | 1.9E1 | 454 | 8 | 168 | 8 | 0.52 |
| Bc | ng/mL | 3.9E1 | 1.1E2 | 1.1E2 | 2.4E2 | 2.0E2 | 3.3E2 | 1.1E-1 | 9.6E0 | 1.2E3 | 1.0E3 | 454 | 8 | 168 | 8 | 0.68 |
| Bg | ng/mL | 9.1E-2 | 1.7E-1 | 5.9E0 | 7.0E-1 | 3.5E1 | 1.0E0 | 5.3E-4 | 1.0E-2 | 4.4E2 | 2.9E0 | 454 | 8 | 168 | 8 | 0.56 |
| Bn | ng/mL | 5.6E-2 | 9.7E-2 | 1.2E0 | 8.3E0 | 2.0E0 | 2.0E1 | 5.6E-2 | 5.6E-2 | 9.7E0 | 5.8E1 | 454 | 8 | 168 | 8 | 0.55 |
| Bo | ng/mL | 1.2E1 | 2.2E1 | 1.5E1 | 2.6E1 | 1.9E1 | 1.9E1 | 1.6E-2 | 1.6E-2 | 2.8E2 | 5.3E1 | 454 | 8 | 168 | 8 | 0.69 |
| Ch | uIU/mL | 1.2E0 | 8.4E-1 | 2.1E1 | 8.1E-1 | 1.1E2 | 3.8E-1 | 3.4E-3 | 1.8E-1 | 1.8E3 | 1.4E0 | 454 | 8 | 168 | 8 | 0.36 |
| Co | pg/mL | 4.0E1 | 4.2E1 | 1.7E2 | 6.4E1 | 9.5E2 | 7.4E1 | 1.5E-1 | 1.5E-1 | 1.7E4 | 2.2E2 | 454 | 8 | 168 | 8 | 0.47 |
| Cp | ng/mL | 2.2E1 | 2.1E1 | 2.9E1 | 1.9E2 | 3.2E1 | 4.4E2 | 6.0E-1 | 1.4E1 | 3.7E2 | 1.3E3 | 454 | 8 | 168 | 8 | 0.60 |
| Cq | ng/mL | 3.0E-2 | 3.5E-2 | 1.6E-1 | 6.1E0 | 8.8E-1 | 1.7E1 | 8.0E-4 | 8.0E-4 | 1.7E1 | 4.9E1 | 454 | 8 | 168 | 8 | 0.49 |
| Cs | ng/mL | 7.3E1 | 4.6E2 | 3.4E2 | 1.5E3 | 1.1E3 | 2.0E3 | 2.7E-2 | 3.0E1 | 1.8E4 | 5.1E3 | 454 | 8 | 168 | 8 | 0.78 |
| Ct | ng/mL | 6.0E-1 | 1.1E-1 | 4.0E1 | 6.9E1 | 1.1E2 | 1.6E2 | 1.1E-4 | 5.7E-2 | 6.2E2 | 4.7E2 | 454 | 8 | 168 | 8 | 0.47 |
| Cu | ng/mL | 2.5E-1 | 3.6E-1 | 5.0E-1 | 8.8E0 | 1.4E0 | 2.3E1 | 9.6E-3 | 4.6E-2 | 2.1E1 | 6.6E1 | 454 | 8 | 168 | 8 | 0.62 |
| Cv | ng/mL | 5.1E0 | 7.3E0 | 2.1E1 | 1.6E2 | 5.2E1 | 2.3E2 | 1.4E-4 | 1.9E-1 | 5.3E2 | 5.2E2 | 454 | 8 | 168 | 8 | 0.58 |
| Cw | mIU/mL | 3.0E-2 | 3.4E-2 | 3.8E-2 | 8.8E-1 | 3.1E-2 | 2.4E0 | 1.5E-4 | 1.4E-2 | 2.4E-1 | 6.8E0 | 454 | 8 | 168 | 8 | 0.61 |
| Cx | ng/mL | 2.2E-1 | 7.8E-3 | 5.6E1 | 1.0E2 | 1.0E2 | 1.6E2 | 9.3E-5 | 9.3E-5 | 3.9E2 | 3.9E2 | 454 | 8 | 168 | 8 | 0.45 |
| Db | ug/mL | 7.3E0 | 1.1E1 | 8.8E0 | 1.1E1 | 9.4E0 | 6.6E0 | 4.5E-1 | 1.4E0 | 1.4E2 | 1.9E1 | 454 | 8 | 168 | 8 | 0.62 |
| Dc | nmol/L | 1.9E-2 | 1.6E-2 | 6.2E-2 | 2.0E0 | 1.5E-1 | 4.9E0 | 5.2E-6 | 1.3E-3 | 1.6E0 | 1.4E1 | 454 | 8 | 168 | 8 | 0.56 |
| Dd | ug/mL | 6.9E-2 | 6.1E-2 | 1.7E-1 | 5.4E-1 | 2.6E-1 | 1.2E0 | 1.9E-4 | 6.2E-3 | 1.9E0 | 3.6E0 | 454 | 8 | 168 | 8 | 0.52 |
| De | ng/mL | 3.4E-3 | 3.4E-3 | 8.0E-2 | 1.5E-1 | 1.4E-1 | 2.7E-1 | 3.4E-3 | 3.4E-3 | 9.2E-1 | 7.9E-1 | 454 | 8 | 168 | 8 | 0.54 |
| Dg | ng/mL | 3.5E1 | 2.5E1 | 4.6E1 | 5.2E1 | 4.0E1 | 6.2E1 | 1.0E-1 | 4.7E0 | 1.9E2 | 1.9E2 | 454 | 8 | 168 | 8 | 0.48 |
| Di | pg/mL | 1.9E0 | 3.3E0 | 2.2E0 | 3.5E0 | 2.1E0 | 2.8E0 | 1.8E-1 | 1.8E-1 | 1.3E1 | 9.0E0 | 454 | 8 | 168 | 8 | 0.66 |
| Dk | uIU/mL | 1.7E-2 | 3.2E-2 | 8.5E-2 | 2.0E-1 | 5.1E-1 | 2.7E-1 | 1.1E-4 | 5.8E-3 | 8.9E0 | 6.3E-1 | 454 | 8 | 168 | 8 | 0.64 |
| Dl | ng/mL | 2.4E2 | 3.0E2 | 3.3E2 | 4.6E2 | 3.0E2 | 5.0E2 | 1.7E0 | 1.8E1 | 1.5E3 | 1.6E3 | 454 | 8 | 168 | 8 | 0.56 |
| Dp | ng/ml | 2.4E0 | 1.0E0 | 5.3E0 | 3.1E1 | 8.2E0 | 7.0E1 | 3.7E-3 | 3.7E-3 | 5.6E1 | 2.0E2 | 290 | 8 | 162 | 8 | 0.42 |
| Ef | ng/ml | 1.5E-1 | 2.4E-1 | 8.9E-1 | 1.4E0 | 1.8E0 | 2.5E0 | 5.7E-4 | 1.1E-2 | 1.0E1 | 7.0E0 | 344 | 7 | 165 | 7 | 0.54 |
| Wm | % | 4.7E-1 | 7.5E0 | 3.9E1 | 1.1E2 | 1.9E2 | 3.0E2 | 5.4E-2 | 8.5E-2 | 2.4E3 | 1.0E3 | 358 | 12 | 178 | 12 | 0.69 |
| Ed | pg/ml | 5.2E-1 | 6.8E1 | 6.1E1 | 7.1E1 | 4.3E2 | 4.7E1 | 5.2E-1 | 4.4E0 | 7.3E3 | 1.5E2 | 290 | 8 | 161 | 8 | 0.79 |
| Tj | pg/mL | 3.7E-1 | 3.0E0 | 5.6E1 | 3.8E1 | 2.9E2 | 5.4E1 | 3.6E-1 | 3.7E-1 | 3.5E3 | 1.4E2 | 339 | 8 | 167 | 8 | 0.59 |
| Po | pg/ml | 8.0E-1 | 2.9E1 | 8.3E0 | 5.0E1 | 2.2E1 | 6.6E1 | 8.0E-3 | 5.3E-1 | 2.7E2 | 2.2E2 | 799 | 14 | 262 | 14 | 0.82 |
| Et | ng/ml | 1.5E3 | 3.5E3 | 1.7E3 | 3.4E3 | 1.2E3 | 1.4E3 | 7.7E1 | 7.9E2 | 5.0E3 | 5.0E3 | 798 | 14 | 262 | 14 | 0.81 |
| Fa | ng/ml | 4.8E1 | 2.5E2 | 1.3E2 | 2.8E2 | 5.4E2 | 2.2E2 | 3.4E-2 | 7.5E0 | 8.0E3 | 7.3E2 | 286 | 8 | 160 | 8 | 0.83 |
| Ez | ng/ml | 4.5E0 | 1.4E1 | 1.8E1 | 2.4E1 | 5.3E1 | 2.9E1 | 1.3E-2 | 1.1E-1 | 7.1E2 | 8.8E1 | 290 | 8 | 162 | 8 | 0.65 |
| Fb | ng/ml | 2.6E1 | 2.6E1 | 2.4E1 | 2.9E1 | 1.1E1 | 5.8E0 | 5.9E-1 | 2.4E1 | 5.7E1 | 4.1E1 | 287 | 8 | 160 | 8 | 0.61 |
| Fn | ng/ml | 2.1E-1 | 2.1E-1 | 5.8E0 | 3.5E0 | 2.5E1 | 9.3E0 | 1.1E-14 | 2.1E-1 | 4.2E2 | 2.7E1 | 290 | 8 | 162 | 8 | 0.36 |
| Fp | ng/ml | 1.4E1 | 6.2E1 | 2.6E1 | 6.1E1 | 2.9E1 | 3.9E1 | 6.0E-3 | 6.8E0 | 1.4E2 | 1.3E2 | 828 | 13 | 264 | 13 | 0.78 |
| Fr | ng/ml | 4.1E4 | 4.2E5 | 1.2E5 | 4.0E5 | 1.8E5 | 3.2E5 | 1.9E2 | 1.0E4 | 9.0E5 | 8.4E5 | 916 | 14 | 265 | 14 | 0.76 |
| Fw | pg/ml | 1.1E0 | 8.2E0 | 6.3E1 | 4.8E1 | 4.7E2 | 1.1E2 | 1.1E-14 | 1.2E-1 | 6.9E3 | 3.3E2 | 342 | 8 | 166 | 8 | 0.61 |

127

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fy | ng/ml | 3.5E1 | 7.3E1 | 5.8E1 | 2.2E2 | 6.9E1 | 2.6E2 | 1.2E-1 | 1.2E1 | 5.3E2 | 6.5E2 | 288 | 7 | 161 | 7 | 0.70 |
| Gl | pg/ml | 7.5E3 | 1.9E4 | 1.1E4 | 1.9E4 | 9.3E3 | 1.1E4 | 9.1E1 | 1.3E3 | 3.3E4 | 3.1E4 | 334 | 8 | 165 | 8 | 0.71 |
| Gp | U/ml | 1.4E0 | 1.2E0 | 3.9E0 | 2.1E0 | 6.8E0 | 2.5E0 | 1.3E-3 | 1.5E-2 | 6.7E1 | 7.3E0 | 344 | 8 | 166 | 8 | 0.44 |
| Gz | ug/ml | 1.2E0 | 2.2E0 | 8.2E0 | 4.2E0 | 3.6E1 | 5.0E0 | 2.9E-16 | 1.0E-1 | 4.8E2 | 1.1E1 | 196 | 7 | 104 | 7 | 0.47 |
| Ha | ng/ml | 2.6E0 | 5.8E0 | 9.4E0 | 2.2E1 | 2.0E1 | 3.5E1 | 6.4E-3 | 6.4E-1 | 1.3E2 | 1.0E2 | 288 | 8 | 161 | 8 | 0.62 |
| Nm | pg/ml | 1.7E4 | 3.4E4 | 3.4E4 | 1.0E5 | 8.2E4 | 2.1E5 | 1.0E-9 | 1.0E-9 | 1.6E6 | 8.2E5 | 802 | 14 | 264 | 14 | 0.66 |
| Nn | pg/ml | 1.7E2 | 2.1E3 | 1.9E3 | 1.7E4 | 8.2E3 | 3.5E4 | 1.0E-9 | 1.1E2 | 1.0E5 | 1.1E5 | 802 | 14 | 264 | 14 | 0.79 |
| No | pg/ml | 1.7E1 | 1.0E2 | 3.7E1 | 2.2E2 | 1.1E2 | 2.6E2 | 1.0E-9 | 9.0E0 | 2.5E3 | 7.7E2 | 802 | 14 | 264 | 14 | 0.84 |
| Nq | pg/ml | 2.3E0 | 2.0E1 | 2.0E1 | 4.3E1 | 7.7E1 | 6.2E1 | 1.0E-9 | 1.0E-9 | 1.2E3 | 2.2E2 | 802 | 14 | 264 | 14 | 0.67 |
| Nr | pg/ml | 1.3E0 | 7.4E0 | 3.0E1 | 1.7E2 | 1.9E2 | 3.9E2 | 1.0E-9 | 1.0E-9 | 4.1E3 | 1.4E3 | 802 | 14 | 264 | 14 | 0.65 |
| Ns | pg/ml | 1.0E-9 | 1.0E-9 | 9.6E0 | 1.0E-9 | 5.5E1 | 0.0E0 | 1.0E-9 | 1.0E-9 | 1.1E3 | 1.0E-9 | 802 | 14 | 264 | 14 | 0.45 |
| Nt | pg/ml | 1.1E2 | 1.9E2 | 1.4E2 | 3.0E2 | 1.1E2 | 3.2E2 | 1.0E-9 | 4.4E1 | 1.5E3 | 1.2E3 | 802 | 14 | 264 | 14 | 0.70 |
| Nu | pg/ml | 2.4E1 | 7.4E1 | 5.6E1 | 1.2E2 | 8.9E1 | 1.2E2 | 1.0E-9 | 1.0E-9 | 8.9E2 | 3.7E2 | 802 | 14 | 264 | 14 | 0.69 |
| Lu | pg/ml | 1.0E4 | 5.6E3 | 1.8E4 | 7.8E3 | 6.5E4 | 5.7E3 | 3.5E2 | 2.6E3 | 1.3E6 | 2.2E4 | 805 | 14 | 264 | 14 | 0.36 |
| Lv | pg/ml | 1.0E-9 | 3.8E1 | 1.1E1 | 5.0E1 | 2.3E1 | 5.7E1 | 1.0E-9 | 1.0E-9 | 2.6E2 | 1.6E2 | 805 | 14 | 264 | 14 | 0.72 |
| Lw | pg/ml | 1.0E-9 | 1.0E-9 | 3.2E-1 | 6.6E0 | 3.7E0 | 1.2E1 | 1.0E-9 | 1.0E-9 | 8.0E1 | 4.0E1 | 805 | 14 | 264 | 14 | 0.64 |
| Lx | pg/ml | 1.0E-9 | 9.8E2 | 1.5E2 | 1.1E3 | 4.3E2 | 9.5E2 | 1.0E-9 | 1.0E-9 | 6.2E3 | 2.8E3 | 805 | 14 | 264 | 14 | 0.81 |
| Ly | pg/ml | 1.0E-9 | 1.0E-9 | 1.0E1 | 3.9E0 | 2.0E1 | 7.8E0 | 1.0E-9 | 1.0E-9 | 1.6E2 | 2.6E1 | 805 | 14 | 264 | 14 | 0.44 |
| Lz | pg/ml | 1.0E-9 | 1.0E-9 | 3.8E0 | 1.0E1 | 3.1E1 | 2.1E1 | 1.0E-9 | 1.0E-9 | 6.0E2 | 6.2E1 | 805 | 14 | 264 | 14 | 0.58 |
| Ma | pg/ml | 3.0E2 | 1.7E3 | 1.3E3 | 4.5E3 | 3.8E3 | 8.2E3 | 1.0E-9 | 8.7E1 | 6.5E4 | 3.1E4 | 805 | 14 | 264 | 14 | 0.70 |
| Mb | pg/ml | 2.5E1 | 3.0E1 | 3.1E1 | 3.4E1 | 1.5E1 | 2.0E1 | 5.4E0 | 4.1E0 | 2.1E2 | 7.1E1 | 805 | 14 | 264 | 14 | 0.51 |
| Mc | pg/ml | 1.0E-9 | 1.0E-9 | 4.3E-2 | 1.0E-9 | 6.0E-1 | 0.0E0 | 1.0E-9 | 1.0E-9 | 1.3E1 | 1.0E-9 | 805 | 14 | 264 | 14 | 0.49 |
| Md | pg/ml | 1.0E-9 | 1.0E-9 | 3.7E-1 | 2.5E0 | 2.8E0 | 7.9E0 | 1.0E-9 | 1.0E-9 | 6.5E1 | 2.9E1 | 805 | 14 | 264 | 14 | 0.57 |
| Me | pg/ml | 3.3E1 | 1.6E1 | 3.2E1 | 2.8E1 | 2.0E1 | 4.7E1 | 1.0E-9 | 1.0E-9 | 3.2E2 | 1.8E2 | 805 | 14 | 264 | 14 | 0.29 |
| Mf | pg/ml | 1.0E-9 | 1.0E-9 | 5.5E-1 | 8.1E-1 | 3.0E0 | 1.8E0 | 1.0E-9 | 1.0E-9 | 5.6E1 | 5.5E0 | 805 | 14 | 264 | 14 | 0.57 |
| Mg | pg/ml | 1.8E0 | 9.2E-1 | 7.6E0 | 7.6E0 | 1.2E1 | 1.0E1 | 1.0E-9 | 1.0E-9 | 9.2E1 | 2.7E1 | 805 | 14 | 264 | 14 | 0.50 |
| Mh | pg/ml | 1.0E-9 | 1.5E-2 | 1.3E0 | 3.9E0 | 9.8E0 | 6.4E0 | 1.0E-9 | 1.0E-9 | 1.6E2 | 1.8E1 | 805 | 14 | 264 | 14 | 0.64 |
| Mi | pg/ml | 1.0E-9 | 1.0E-9 | 4.8E-1 | 1.1E1 | 5.4E0 | 3.0E1 | 1.0E-9 | 1.0E-9 | 1.2E2 | 1.1E2 | 805 | 14 | 264 | 14 | 0.60 |
| Mj | pg/ml | 1.0E-9 | 1.0E-9 | 4.3E0 | 3.8E1 | 2.4E1 | 7.0E1 | 1.0E-9 | 1.0E-9 | 4.6E2 | 2.1E2 | 805 | 14 | 264 | 14 | 0.63 |
| Mk | pg/ml | 9.1E-1 | 5.7E0 | 1.5E1 | 4.4E1 | 9.1E1 | 1.3E2 | 1.0E-9 | 1.0E-9 | 1.7E3 | 5.0E2 | 805 | 14 | 264 | 14 | 0.60 |
| Ml | pg/ml | 1.0E-9 | 1.0E-9 | 5.2E0 | 4.9E1 | 7.7E1 | 1.4E2 | 1.0E-9 | 1.0E-9 | 2.1E3 | 5.1E2 | 805 | 14 | 264 | 14 | 0.65 |
| Mm | pg/ml | 6.4E2 | 1.8E3 | 1.1E3 | 2.7E3 | 1.3E3 | 3.2E3 | 1.0E-9 | 7.1E1 | 1.1E4 | 1.2E4 | 805 | 14 | 264 | 14 | 0.69 |
| Mn | pg/ml | 5.7E0 | 1.1E1 | 1.1E1 | 1.7E1 | 2.4E1 | 1.5E1 | 1.0E-9 | 2.8E0 | 3.5E2 | 5.1E1 | 805 | 14 | 264 | 14 | 0.73 |
| Mp | pg/ml | 1.0E-9 | 2.7E1 | 9.3E0 | 4.2E1 | 3.0E1 | 4.3E1 | 1.0E-9 | 1.0E-9 | 6.3E2 | 1.3E2 | 804 | 14 | 264 | 14 | 0.79 |
| Mq | pg/ml | 1.0E-9 | 3.5E0 | 2.7E0 | 2.5E1 | 1.7E1 | 3.7E1 | 1.0E-9 | 1.0E-9 | 2.4E2 | 1.0E2 | 804 | 14 | 264 | 14 | 0.72 |
| Mr | pg/ml | 1.0E-9 | 7.2E0 | 2.8E1 | 3.9E2 | 1.5E2 | 9.4E2 | 1.0E-9 | 1.0E-9 | 2.2E3 | 3.4E3 | 804 | 14 | 264 | 14 | 0.72 |
| Ms | pg/ml | 4.1E2 | 2.2E2 | 5.5E2 | 3.3E2 | 6.5E2 | 3.1E2 | 1.0E-9 | 1.0E-9 | 5.9E3 | 9.8E2 | 804 | 14 | 264 | 14 | 0.41 |
| Mt | pg/ml | 4.8E-1 | 9.7E0 | 7.2E0 | 2.6E2 | 4.4E1 | 8.6E2 | 1.0E-9 | 1.0E-9 | 8.7E2 | 3.2E3 | 804 | 14 | 264 | 14 | 0.78 |
| Mu | pg/ml | 1.0E-9 | 2.4E0 | 1.4E0 | 5.8E0 | 1.1E1 | 1.0E1 | 1.0E-9 | 1.0E-9 | 2.3E2 | 3.5E1 | 804 | 14 | 264 | 14 | 0.79 |
| Mv | pg/ml | 1.0E-9 | 5.9E0 | 7.6E0 | 1.4E2 | 3.3E2 | 2.8E2 | 1.0E-9 | 1.0E-9 | 4.5E3 | 9.2E2 | 804 | 14 | 264 | 14 | 0.67 |
| Mw | pg/ml | 4.0E1 | 2.8E2 | 5.5E2 | 1.2E3 | 3.5E3 | 1.9E3 | 1.0E-9 | 1.0E-9 | 6.2E4 | 5.3E3 | 804 | 14 | 264 | 14 | 0.78 |
| Mx | pg/ml | 1.0E-9 | 1.0E-9 | 2.6E-1 | 2.4E0 | 1.4E0 | 5.3E0 | 1.0E-9 | 1.0E-9 | 3.2E1 | 2.0E1 | 804 | 14 | 264 | 14 | 0.66 |
| My | pg/ml | 1.0E-9 | 1.0E-9 | 5.1E2 | 2.1E2 | 3.1E3 | 5.1E2 | 1.0E-9 | 1.0E-9 | 3.9E4 | 1.9E3 | 804 | 14 | 264 | 14 | 0.56 |
| Mz | pg/ml | 1.2E1 | 2.9E1 | 2.8E1 | 2.7E2 | 6.5E1 | 5.8E2 | 1.0E-9 | 1.0E-9 | 1.2E3 | 1.9E3 | 804 | 14 | 264 | 14 | 0.71 |
| Na | pg/ml | 1.0E-9 | 1.0E-9 | 6.3E-1 | 5.5E0 | 2.7E0 | 1.1E1 | 1.0E-9 | 1.0E-9 | 3.6E1 | 4.2E1 | 804 | 14 | 264 | 14 | 0.64 |
| Nb | pg/ml | 2.1E0 | 4.4E0 | 4.0E0 | 2.7E1 | 1.2E1 | 5.9E1 | 1.0E-9 | 1.0E-9 | 2.3E2 | 2.1E2 | 804 | 14 | 264 | 14 | 0.66 |
| Nc | pg/ml | 3.4E2 | 2.0E2 | 5.6E2 | 3.6E2 | 7.4E2 | 3.8E2 | 1.0E-9 | 1.0E-9 | 9.2E3 | 1.1E3 | 804 | 14 | 264 | 14 | 0.45 |
| Nd | pg/ml | 2.8E1 | 1.4E1 | 2.7E1 | 2.4E1 | 4.7E1 | 2.8E1 | 1.0E-9 | 1.0E-9 | 1.2E3 | 9.4E1 | 804 | 14 | 264 | 14 | 0.45 |
| Ne | pg/ml | 4.5E2 | 2.6E2 | 5.8E2 | 2.6E2 | 5.9E2 | 1.9E2 | 1.0E-9 | 1.0E-9 | 7.0E3 | 5.8E2 | 804 | 14 | 264 | 14 | 0.33 |
| Nf | pg/ml | 1.0E-9 | 8.7E-1 | 2.5E0 | 1.8E1 | 9.5E0 | 4.4E1 | 1.0E-9 | 1.0E-9 | 1.6E2 | 1.3E2 | 804 | 14 | 264 | 14 | 0.64 |
| Ng | pg/ml | 2.8E1 | 7.6E0 | 1.3E2 | 3.7E1 | 2.5E2 | 6.0E1 | 1.0E-9 | 1.0E-9 | 2.3E3 | 1.7E2 | 804 | 14 | 264 | 14 | 0.39 |
| Nh | pg/ml | 6.8E1 | 3.6E1 | 9.1E1 | 4.0E1 | 8.5E1 | 2.4E1 | 1.0E-9 | 4.1E0 | 5.6E2 | 7.5E1 | 804 | 14 | 264 | 14 | 0.30 |
| Ni | pg/ml | 1.0E-9 | 1.5E2 | 7.4E1 | 2.2E2 | 1.2E2 | 3.0E2 | 1.0E-9 | 1.0E-9 | 7.1E2 | 1.1E3 | 804 | 14 | 264 | 14 | 0.64 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nj | pg/ml | 7.3E0 | 3.4E0 | 1.1E1 | 7.0E0 | 1.2E1 | 7.0E0 | 1.0E-9 | 1.0E-9 | 1.1E2 | 2.0E1 | 804 | 14 | 264 | 14 | 0.42 |
| Nk | pg/ml | 1.7E1 | 2.4E1 | 3.3E1 | 2.6E1 | 4.0E1 | 2.3E1 | 1.0E-9 | 1.0E-9 | 2.0E2 | 6.6E1 | 804 | 14 | 264 | 14 | 0.51 |
| Nl | pg/ml | 4.6E1 | 2.4E1 | 6.1E1 | 3.1E1 | 6.9E1 | 2.7E1 | 1.0E-9 | 1.0E-9 | 1.1E3 | 8.2E1 | 804 | 14 | 264 | 14 | 0.37 |
| Tz | pg/ml | 5.6E3 | 1.0E4 | 2.2E4 | 1.0E4 | 1.4E5 | 8.1E3 | 1.0E-9 | 8.1E2 | 2.1E6 | 2.5E4 | 292 | 8 | 160 | 8 | 0.59 |
| Ua | pg/ml | 3.9E3 | 6.5E3 | 1.4E4 | 8.4E3 | 2.7E4 | 6.3E3 | 1.0E-9 | 1.1E3 | 1.9E5 | 1.9E4 | 292 | 8 | 160 | 8 | 0.58 |
| Ub | pg/ml | 5.5E2 | 3.5E2 | 8.1E2 | 6.8E2 | 1.0E3 | 9.2E2 | 1.0E-9 | 1.2E1 | 9.8E3 | 2.8E3 | 292 | 8 | 160 | 8 | 0.42 |
| Ue | pg/ml | 2.7E1 | 1.6E1 | 3.8E1 | 3.7E1 | 4.2E1 | 4.6E1 | 9.8E-2 | 5.9E0 | 4.4E2 | 1.4E2 | 292 | 8 | 160 | 8 | 0.39 |
| Uc | pg/ml | 9.8E2 | 9.8E2 | 1.9E3 | 8.1E3 | 2.7E3 | 2.0E4 | 1.0E-9 | 5.5E1 | 2.9E4 | 5.7E4 | 292 | 8 | 160 | 8 | 0.48 |
| Ud | pg/ml | 1.0E-9 | 1.0E-9 | 1.4E0 | 6.7E0 | 2.3E1 | 1.9E1 | 1.0E-9 | 1.0E-9 | 3.9E2 | 5.3E1 | 292 | 8 | 160 | 8 | 0.56 |
| Hq | pg/ml | 1.1E0 | 2.2E0 | 1.1E2 | 1.8E1 | 1.7E3 | 4.8E1 | 1.0E-9 | 1.0E-9 | 3.4E4 | 1.8E2 | 800 | 14 | 263 | 14 | 0.59 |
| Hr | pg/ml | 1.1E2 | 1.4E2 | 7.4E2 | 1.5E3 | 1.6E3 | 2.6E3 | 1.0E-9 | 1.0E-9 | 1.7E4 | 8.9E3 | 800 | 14 | 263 | 14 | 0.59 |
| Hu | pg/ml | 7.3E0 | 1.3E2 | 3.1E2 | 3.3E2 | 2.7E4 | 4.3E2 | 1.0E-9 | 1.0E-9 | 6.3E5 | 1.3E3 | 800 | 14 | 263 | 14 | 0.66 |
| Hv | pg/ml | 1.5E0 | 2.5E0 | 3.3E0 | 7.0E1 | 1.2E1 | 2.4E2 | 1.0E-9 | 1.0E-9 | 2.5E2 | 8.9E2 | 800 | 14 | 263 | 14 | 0.60 |
| Hw | pg/ml | 6.3E0 | 1.2E1 | 1.9E1 | 7.1E2 | 7.4E1 | 2.5E3 | 1.0E-9 | 5.1E-1 | 1.7E3 | 9.4E3 | 800 | 14 | 263 | 14 | 0.58 |
| Hx | pg/ml | 9.2E0 | 2.1E1 | 4.0E1 | 1.8E2 | 3.4E2 | 3.8E2 | 1.0E-9 | 1.0E-9 | 9.3E3 | 1.3E3 | 800 | 14 | 263 | 14 | 0.71 |
| Ib | ng/ml | 4.9E-2 | 7.6E-2 | 1.9E0 | 7.2E0 | 7.0E0 | 2.0E1 | 1.0E-9 | 1.0E-9 | 5.3E1 | 5.6E1 | 283 | 8 | 159 | 8 | 0.55 |
| Ic | U/ml | 2.2E2 | 2.2E2 | 1.2E3 | 2.5E2 | 7.1E3 | 1.4E2 | 1.5E0 | 5.2E1 | 9.3E4 | 4.2E2 | 283 | 8 | 159 | 8 | 0.59 |
| Id | U/ml | 7.1E-1 | 1.2E0 | 1.4E0 | 5.7E1 | 2.5E0 | 1.5E2 | 1.0E-9 | 5.5E-1 | 2.3E1 | 4.3E2 | 283 | 8 | 159 | 8 | 0.73 |
| To | pg/ml | 1.6E0 | 1.8E0 | 1.9E0 | 3.0E0 | 2.0E0 | 4.1E0 | 1.0E-9 | 1.0E-9 | 1.0E1 | 1.2E1 | 280 | 7 | 157 | 7 | 0.56 |
| Tn | pg/ml | 3.1E1 | 5.2E1 | 7.9E1 | 4.2E2 | 2.1E2 | 8.4E2 | 2.4E0 | 2.1E1 | 2.0E3 | 2.3E3 | 280 | 7 | 157 | 7 | 0.69 |
| Tv | ng/ml | 1.2E1 | 1.0E1 | 2.5E1 | 1.0E3 | 6.5E1 | 2.7E3 | 1.0E-9 | 1.0E-9 | 7.9E2 | 7.1E3 | 280 | 7 | 157 | 7 | 0.47 |
| Ih | ng/ml | 7.7E1 | 4.9E2 | 2.4E2 | 7.0E2 | 4.9E2 | 7.6E2 | 1.0E-9 | 1.0E-9 | 7.4E3 | 2.8E3 | 804 | 14 | 263 | 14 | 0.71 |
| Ii | ng/ml | 9.8E1 | 2.1E2 | 2.5E2 | 7.7E2 | 6.8E2 | 1.4E3 | 1.0E-9 | 2.3E0 | 1.0E4 | 4.5E3 | 804 | 14 | 263 | 14 | 0.61 |
| Ij | ng/ml | 8.1E1 | 1.4E2 | 1.8E2 | 1.9E3 | 5.5E2 | 6.5E3 | 1.6E-1 | 2.5E1 | 6.4E3 | 2.4E4 | 794 | 14 | 261 | 14 | 0.68 |
| Ik | ng/ml | 1.3E1 | 4.2E1 | 9.0E2 | 3.6E2 | 8.7E3 | 6.9E2 | 5.9E-1 | 5.5E0 | 1.2E5 | 2.5E3 | 800 | 14 | 261 | 14 | 0.65 |
| Il | ng/ml | 3.7E2 | 5.4E2 | 1.3E3 | 1.7E3 | 2.8E3 | 3.2E3 | 1.0E-9 | 1.0E-9 | 1.3E4 | 1.2E4 | 787 | 14 | 261 | 14 | 0.53 |
| Im | ng/ml | 2.3E2 | 7.0E2 | 4.1E2 | 1.3E3 | 6.1E2 | 1.6E3 | 1.3E1 | 4.7E1 | 6.8E3 | 6.2E3 | 800 | 14 | 262 | 14 | 0.78 |
| In | ng/ml | 3.3E0 | 5.5E0 | 2.2E1 | 3.4E2 | 1.5E2 | 1.2E3 | 1.0E-9 | 1.0E-9 | 3.9E3 | 4.5E3 | 804 | 14 | 263 | 14 | 0.57 |
| Hb | ng/ml | 2.5E1 | 2.3E1 | 3.6E1 | 3.1E1 | 3.5E1 | 2.4E1 | 4.8E-1 | 6.2E-1 | 2.1E2 | 8.0E1 | 292 | 8 | 161 | 8 | 0.49 |
| Hc | pg/ml | 6.7E2 | 3.6E2 | 3.7E3 | 2.3E3 | 1.3E4 | 4.7E3 | 1.0E-9 | 1.0E-9 | 1.0E5 | 1.4E4 | 292 | 8 | 161 | 8 | 0.37 |
| Hf | ng/ml | 1.6E2 | 2.1E2 | 3.7E2 | 2.5E2 | 5.2E2 | 2.7E2 | 1.0E-9 | 1.0E-9 | 3.2E3 | 6.7E2 | 292 | 8 | 161 | 8 | 0.45 |
| Io | ng/ml | 9.2E3 | 6.1E3 | 2.7E4 | 1.1E4 | 1.6E5 | 1.1E4 | 1.0E-9 | 1.3E3 | 4.0E6 | 3.3E4 | 796 | 14 | 263 | 14 | 0.44 |
| Ip | ng/ml | 1.2E1 | 3.0E1 | 2.0E1 | 3.1E1 | 2.4E1 | 2.2E1 | 1.0E-9 | 3.7E-2 | 2.6E2 | 5.7E1 | 796 | 14 | 263 | 14 | 0.63 |
| Iq | ug/ml | 1.1E-1 | 3.9E-1 | 1.8E1 | 1.8E1 | 4.8E2 | 5.8E1 | 1.0E-9 | 1.0E-9 | 1.4E4 | 2.2E2 | 796 | 14 | 263 | 14 | 0.65 |
| Ir | ug/ml | 3.7E-1 | 2.2E0 | 2.6E0 | 4.6E1 | 1.5E1 | 1.0E2 | 1.0E-9 | 1.0E-9 | 2.4E2 | 3.7E2 | 795 | 14 | 263 | 14 | 0.73 |
| Is | ng/ml | 1.6E0 | 1.3E1 | 6.0E0 | 4.7E1 | 1.3E1 | 7.4E1 | 1.0E-9 | 4.9E-1 | 1.5E2 | 2.6E2 | 796 | 14 | 263 | 14 | 0.80 |
| It | ng/ml | 2.0E0 | 6.0E0 | 2.0E1 | 6.8E1 | 1.3E2 | 1.8E2 | 1.0E-9 | 1.0E-9 | 2.8E3 | 6.8E2 | 796 | 14 | 263 | 14 | 0.66 |
| Iu | ng/ml | 2.3E2 | 4.8E2 | 1.3E3 | 2.6E3 | 3.8E3 | 6.4E3 | 1.0E-9 | 1.0E-9 | 2.9E4 | 2.4E4 | 796 | 14 | 263 | 14 | 0.56 |
| Iv | ng/ml | 1.4E1 | 9.8E1 | 4.5E1 | 6.3E2 | 1.6E2 | 1.3E3 | 1.0E-9 | 1.0E-9 | 3.8E3 | 3.8E3 | 795 | 14 | 263 | 14 | 0.76 |
| Iz | ng/ml | 1.6E2 | 1.7E2 | 6.3E2 | 2.6E2 | 3.7E3 | 3.3E2 | 9.2E-1 | 4.9E0 | 6.2E4 | 1.0E3 | 292 | 8 | 161 | 8 | 0.47 |
| Rc | pg/ml | 6.0E3 | 4.5E3 | 7.1E3 | 7.0E3 | 5.2E3 | 5.5E3 | 1.9E2 | 2.1E3 | 2.7E4 | 1.7E4 | 289 | 8 | 160 | 8 | 0.48 |
| Rb | pg/ml | 9.7E-1 | 6.4E-1 | 2.7E0 | 9.1E0 | 4.4E0 | 2.0E1 | 1.0E-9 | 1.0E-9 | 4.3E1 | 5.6E1 | 289 | 8 | 160 | 8 | 0.50 |
| Pz | ng/ml | 4.7E3 | 1.0E4 | 8.8E3 | 6.8E3 | 4.0E4 | 4.3E3 | 1.3E1 | 6.5E2 | 1.0E6 | 1.3E4 | 796 | 14 | 261 | 14 | 0.59 |
| Qa | ng/ml | 3.6E3 | 1.5E4 | 6.5E3 | 3.0E4 | 7.6E3 | 5.6E4 | 1.2E1 | 1.5E3 | 5.2E4 | 2.2E5 | 796 | 14 | 261 | 14 | 0.82 |
| Qb | ng/ml | 1.0E2 | 3.0E2 | 2.2E2 | 3.8E2 | 4.8E2 | 2.5E2 | 7.9E-1 | 5.1E1 | 8.3E3 | 8.8E2 | 796 | 14 | 261 | 14 | 0.77 |
| Qc | ng/ml | 2.6E2 | 4.4E2 | 6.8E2 | 6.1E2 | 5.9E3 | 7.5E2 | 1.0E-9 | 1.3E1 | 1.7E5 | 2.8E3 | 796 | 14 | 261 | 14 | 0.56 |
| Qd | ng/ml | 1.0E4 | 3.2E4 | 2.2E4 | 6.5E4 | 8.3E4 | 7.5E4 | 2.4E2 | 4.6E3 | 2.0E6 | 2.3E5 | 796 | 14 | 261 | 14 | 0.73 |
| Qc | ng/ml | 1.1E3 | 3.6E3 | 1.9E3 | 5.5E3 | 4.1E3 | 5.2E3 | 7.6E0 | 4.1E2 | 9.7E4 | 1.8E4 | 796 | 14 | 261 | 14 | 0.80 |
| Jd | ng/ml | 1.0E0 | 4.1E0 | 6.1E0 | 6.2E0 | 4.0E1 | 6.2E0 | 1.0E-9 | 1.4E0 | 6.5E2 | 2.1E1 | 290 | 8 | 162 | 8 | 0.81 |
| Je | ng/ml | 1.0E-9 | 4.3E0 | 2.2E0 | 4.3E0 | 7.5E0 | 4.1E0 | 1.0E-9 | 1.0E-9 | 8.8E1 | 1.1E1 | 290 | 8 | 162 | 8 | 0.71 |
| Jf | ng/ml | 1.0E-9 | 8.4E-1 | 1.2E0 | 1.5E0 | 2.4E0 | 1.7E0 | 1.0E-9 | 1.0E-9 | 1.2E1 | 3.7E0 | 290 | 8 | 162 | 8 | 0.59 |
| Jg | ng/ml | 5.4E2 | 1.8E3 | 8.6E2 | 2.0E3 | 1.0E3 | 1.9E3 | 1.0E-9 | 8.7E1 | 1.0E4 | 7.1E3 | 800 | 14 | 263 | 14 | 0.70 |
| Jh | ng/ml | 3.3E0 | 3.2E1 | 2.9E1 | 8.7E1 | 1.2E2 | 1.3E2 | 1.0E-9 | 1.0E-9 | 1.6E3 | 4.9E2 | 800 | 14 | 263 | 14 | 0.73 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ji | ng/ml | 5.4E1 | 2.8E2 | 7.6E1 | 3.5E2 | 7.6E1 | 3.3E2 | 1.0E-9 | 2.0E1 | 5.4E2 | 1.3E3 | 800 | 14 | 263 | 14 | 0.85 |
| Sr | pg/mL | 4.0E2 | 3.3E3 | 9.0E2 | 4.9E3 | 1.3E3 | 6.8E3 | 1.0E-9 | 2.3E2 | 9.8E3 | 2.1E4 | 280 | 8 | 157 | 8 | 0.81 |
| Ss | pg/mL | 1.1E5 | 1.1E5 | 1.6E5 | 1.3E5 | 1.9E5 | 1.2E5 | 2.7E3 | 1.4E4 | 1.8E6 | 3.6E5 | 280 | 8 | 157 | 8 | 0.46 |
| St | pg/mL | 3.3E7 | 1.0E8 | 6.3E7 | 2.7E8 | 1.0E8 | 5.6E8 | 1.0E-9 | 2.3E6 | 1.2E9 | 1.7E9 | 284 | 8 | 158 | 8 | 0.72 |
| Ra | pg/ml | 1.0E-9 | 1.0E-9 | 6.1E-1 | 8.8E0 | 1.2E0 | 2.2E1 | 1.0E-9 | 1.0E-9 | 7.3E0 | 6.4E1 | 289 | 8 | 160 | 8 | 0.51 |
| Qz | pg/ml | 1.0E1 | 1.3E1 | 6.2E1 | 4.2E1 | 1.1E2 | 5.3E1 | 1.0E-9 | 1.0E-9 | 7.1E2 | 1.4E2 | 289 | 8 | 160 | 8 | 0.57 |
| Qy | pg/ml | 4.8E-1 | 1.8E0 | 1.7E1 | 2.7E0 | 8.1E1 | 3.1E0 | 1.0E-9 | 1.1E-1 | 6.5E2 | 9.7E0 | 289 | 8 | 160 | 8 | 0.70 |
| Qx | pg/ml | 1.0E-9 | 1.0E-9 | 5.9E0 | 1.8E1 | 4.8E1 | 3.7E1 | 1.0E-9 | 1.0E-9 | 5.8E2 | 1.1E2 | 289 | 8 | 160 | 8 | 0.63 |
| Qw | pg/ml | 1.0E-9 | 8.1E-1 | 2.9E0 | 1.2E0 | 1.4E1 | 1.9E0 | 1.0E-9 | 1.0E-9 | 1.3E2 | 5.6E0 | 289 | 8 | 160 | 8 | 0.59 |
| Qv | pg/ml | 2.1E4 | 4.2E3 | 3.3E4 | 9.8E3 | 5.7E4 | 1.2E4 | 1.0E-9 | 4.0E2 | 7.4E5 | 3.3E4 | 289 | 8 | 160 | 8 | 0.22 |
| Qu | pg/ml | 1.2E1 | 1.0E-9 | 8.7E1 | 3.2E1 | 1.7E2 | 5.8E1 | 1.0E-9 | 1.0E-9 | 9.8E2 | 1.5E2 | 289 | 8 | 160 | 8 | 0.39 |
| Qt | pg/ml | 1.2E1 | 1.5E1 | 5.1E1 | 5.6E1 | 1.2E2 | 7.6E1 | 1.0E-9 | 1.0E-9 | 1.0E3 | 2.2E2 | 289 | 8 | 160 | 8 | 0.60 |
| Qh | ng/ml | 1.7E1 | 4.3E1 | 4.3E1 | 5.4E1 | 8.1E1 | 5.2E1 | 1.0E-9 | 5.1E0 | 8.0E2 | 1.6E2 | 289 | 8 | 160 | 8 | 0.64 |
| Qg | ng/ml | 7.7E0 | 4.0E0 | 1.8E1 | 8.2E0 | 6.5E1 | 1.2E1 | 5.1E-2 | 1.5E0 | 1.0E3 | 3.7E1 | 289 | 8 | 160 | 8 | 0.35 |
| Jj | ng/ml | 6.6E2 | 1.1E2 | 1.8E3 | 4.0E2 | 1.3E4 | 8.4E2 | 4.8E0 | 1.2E1 | 3.4E5 | 3.3E3 | 800 | 14 | 263 | 14 | 0.20 |
| Jk | ng/ml | 3.4E0 | 1.4E1 | 2.3E1 | 4.7E1 | 4.9E1 | 6.9E1 | 1.0E-9 | 2.4E-1 | 3.9E2 | 2.4E2 | 800 | 14 | 263 | 14 | 0.66 |
| Jl | ng/ml | 4.7E-1 | 6.7E0 | 2.1E0 | 7.2E2 | 6.0E0 | 2.7E3 | 7.6E-4 | 1.4E-1 | 1.1E2 | 9.9E3 | 800 | 14 | 263 | 14 | 0.79 |
| Jm | ng/ml | 1.9E1 | 4.7E1 | 6.0E1 | 5.5E1 | 1.3E2 | 5.1E1 | 1.0E-9 | 4.7E-1 | 2.1E3 | 1.5E2 | 800 | 14 | 263 | 14 | 0.57 |
| Jn | pg/ml | 4.0E-1 | 2.3E0 | 2.0E0 | 9.9E1 | 1.0E1 | 2.5E2 | 1.0E-9 | 1.6E-1 | 2.4E2 | 7.3E2 | 799 | 14 | 263 | 14 | 0.75 |
| Jo | pg/ml | 3.8E3 | 3.7E3 | 4.9E3 | 1.3E4 | 3.8E3 | 2.7E4 | 4.2E1 | 2.3E2 | 2.4E4 | 1.0E5 | 800 | 14 | 263 | 14 | 0.49 |
| Jp | pg/ml | 7.3E4 | 9.8E4 | 7.6E4 | 1.1E5 | 3.7E4 | 4.4E4 | 2.1E3 | 4.5E4 | 3.8E5 | 2.1E5 | 800 | 14 | 263 | 14 | 0.74 |
| Jq | pg/ml | 9.3E1 | 3.4E2 | 1.5E2 | 1.2E3 | 2.1E2 | 2.4E3 | 1.4E0 | 1.1E1 | 4.0E3 | 8.7E3 | 800 | 14 | 263 | 14 | 0.76 |
| Jr | pg/ml | 5.4E0 | 2.3E1 | 2.4E1 | 9.3E2 | 1.3E2 | 2.3E3 | 1.0E-9 | 2.6E0 | 2.4E3 | 7.4E3 | 800 | 14 | 263 | 14 | 0.79 |
| Js | pg/ml | 1.3E1 | 2.9E1 | 4.2E1 | 4.5E2 | 1.7E2 | 1.1E3 | 1.0E-9 | 3.0E0 | 3.0E3 | 3.0E3 | 800 | 14 | 263 | 14 | 0.67 |
| Jt | pg/ml | 2.8E3 | 4.7E3 | 3.3E3 | 8.9E3 | 2.2E3 | 1.4E4 | 7.7E1 | 4.1E2 | 1.8E4 | 5.2E4 | 800 | 14 | 263 | 14 | 0.62 |
| Ju | mIU/ml | 1.0E1 | 8.2E0 | 2.0E1 | 9.6E0 | 3.0E1 | 6.4E0 | 4.8E-2 | 1.9E-1 | 2.3E2 | 1.8E1 | 290 | 8 | 162 | 8 | 0.46 |
| Jv | mIU/ml | 1.4E1 | 4.6E0 | 3.6E1 | 6.8E0 | 6.0E1 | 6.6E0 | 1.0E-2 | 5.5E-1 | 4.4E2 | 1.9E1 | 290 | 8 | 162 | 8 | 0.32 |
| Jy | ng/ml | 1.6E-3 | 3.6E-3 | 2.3E-3 | 8.2E-3 | 4.2E-3 | 1.4E-2 | 8.7E-5 | 8.6E-4 | 5.2E-2 | 4.1E-2 | 290 | 8 | 162 | 8 | 0.73 |
| Kc | pg/ml | 2.6E1 | 2.4E1 | 4.5E1 | 5.9E1 | 4.8E1 | 6.3E1 | 1.0E-9 | 6.9E0 | 2.7E2 | 1.6E2 | 292 | 8 | 161 | 8 | 0.51 |
| Kd | pg/ml | 1.0E-9 | 1.0E-9 | 2.4E2 | 5.4E3 | 6.7E2 | 1.3E4 | 1.0E-9 | 1.0E-9 | 5.0E3 | 3.8E4 | 292 | 8 | 161 | 8 | 0.55 |
| Ke | pg/ml | 1.3E4 | 1.7E4 | 1.5E4 | 7.0E4 | 1.1E4 | 1.1E5 | 3.4E2 | 4.2E3 | 7.0E4 | 3.2E5 | 292 | 8 | 161 | 8 | 0.64 |
| Kf | pg/mL | 7.7E0 | 5.4E0 | 7.7E0 | 1.7E1 | 6.0E0 | 2.6E1 | 1.0E-9 | 1.7E0 | 4.4E1 | 7.8E1 | 292 | 8 | 161 | 8 | 0.53 |
| Kg | pg/mL | 1.2E3 | 6.9E2 | 1.9E3 | 4.0E3 | 2.2E3 | 9.4E3 | 7.3E1 | 1.3E2 | 1.7E4 | 2.7E4 | 292 | 8 | 161 | 8 | 0.35 |
| Ki | pg/ml | 5.9E1 | 9.4E1 | 6.9E1 | 1.0E2 | 5.5E1 | 4.7E1 | 1.0E-9 | 5.9E1 | 3.8E2 | 2.0E2 | 291 | 8 | 161 | 8 | 0.74 |
| Kj | pg/ml | 1.1E3 | 3.7E2 | 1.6E3 | 2.1E3 | 1.7E3 | 5.1E3 | 1.4E1 | 1.2E2 | 1.0E4 | 1.5E4 | 292 | 8 | 161 | 8 | 0.24 |
| Kk | pg/ml | 6.9E0 | 9.8E0 | 1.2E1 | 2.5E1 | 1.6E1 | 2.4E1 | 1.0E-9 | 5.0E0 | 1.6E2 | 5.9E1 | 292 | 8 | 161 | 8 | 0.71 |
| Kl | pg/ml | 2.3E4 | 1.6E4 | 3.0E4 | 2.5E4 | 2.6E4 | 2.7E4 | 1.6E2 | 1.6E3 | 1.6E5 | 7.8E4 | 292 | 8 | 161 | 8 | 0.42 |
| Kn | pg/ml | 3.0E1 | 6.7E1 | 6.6E1 | 7.0E2 | 1.0E2 | 1.7E3 | 1.0E-9 | 1.0E-9 | 7.3E2 | 4.9E3 | 292 | 8 | 161 | 8 | 0.64 |
| Ko | pg/ml | 4.3E2 | 2.2E2 | 5.4E2 | 8.3E2 | 5.3E2 | 1.4E3 | 1.0E-9 | 6.5E1 | 3.8E3 | 4.1E3 | 292 | 8 | 161 | 8 | 0.51 |
| Kp | pg/ml | 3.4E2 | 3.5E2 | 3.7E2 | 2.0E3 | 2.6E2 | 4.6E3 | 1.0E-9 | 3.7E1 | 1.7E3 | 1.3E4 | 292 | 8 | 161 | 8 | 0.52 |
| Kq | pg/ml | 3.6E2 | 8.2E2 | 5.1E2 | 2.2E4 | 8.2E2 | 5.5E4 | 1.6E0 | 1.7E2 | 9.8E3 | 1.6E5 | 283 | 8 | 155 | 8 | 0.75 |
| Kr | pg/ml | 5.6E-1 | 1.0E-9 | 2.6E0 | 5.2E1 | 4.8E0 | 1.5E2 | 1.0E-9 | 1.0E-9 | 3.9E1 | 4.2E2 | 283 | 8 | 155 | 8 | 0.36 |
| Ks | pg/ml | 1.4E4 | 1.7E4 | 2.0E4 | 1.7E4 | 1.9E4 | 1.4E4 | 5.1E1 | 1.3E3 | 1.1E5 | 3.7E4 | 283 | 8 | 155 | 8 | 0.48 |
| Kx | ng/ml | 1.0E-9 | 1.3E-2 | 7.1E-3 | 2.1E-2 | 1.4E-2 | 2.4E-2 | 1.0E-9 | 1.3E-3 | 1.0E-1 | 6.4E-2 | 289 | 8 | 160 | 8 | 0.77 |
| Ky | ng/ml | 1.1E-1 | 5.6E-1 | 4.4E-1 | 7.9E-1 | 9.0E-1 | 9.0E-1 | 1.0E-9 | 3.0E-2 | 6.3E0 | 2.7E0 | 289 | 8 | 160 | 8 | 0.71 |
| Kz | ng/ml | 1.0E-9 | 1.4E-2 | 3.2E-3 | 9.4E-3 | 5.4E-3 | 7.9E-3 | 1.0E-9 | 1.0E-9 | 3.6E-2 | 1.8E-2 | 289 | 8 | 160 | 8 | 0.70 |
| Ld | pg/ml | 1.0E-9 | 1.7E0 | 3.7E0 | 6.8E0 | 8.9E0 | 9.0E0 | 1.0E-9 | 1.0E-9 | 8.4E1 | 2.3E1 | 291 | 8 | 160 | 8 | 0.66 |
| Lh | pg/ml | 1.4E4 | 3.4E4 | 2.2E4 | 8.6E4 | 2.7E4 | 1.3E5 | 1.0E-9 | 1.8E3 | 2.6E5 | 4.1E5 | 799 | 14 | 264 | 14 | 0.71 |
| Li | pg/ml | 3.6E3 | 3.1E4 | 1.6E4 | 6.8E4 | 4.8E4 | 9.0E4 | 1.0E-9 | 5.5E2 | 9.2E5 | 3.1E5 | 799 | 14 | 264 | 14 | 0.75 |
| Lj | pg/ml | 3.0E3 | 1.7E4 | 2.3E4 | 5.4E4 | 6.4E4 | 1.0E5 | 1.0E-9 | 1.5E3 | 5.2E5 | 3.9E5 | 799 | 14 | 264 | 14 | 0.73 |
| Rm | ng/ml | 1.9E1 | 8.6E1 | 5.5E1 | 9.5E1 | 8.5E1 | 8.8E1 | 2.2E-1 | 3.9E-1 | 6.5E2 | 2.5E2 | 284 | 8 | 159 | 8 | 0.68 |
| Rh | ng/ml | 1.3E2 | 1.5E2 | 3.0E2 | 2.3E3 | 8.1E2 | 6.0E3 | 4.7E0 | 5.4E1 | 1.2E4 | 1.7E4 | 284 | 8 | 159 | 8 | 0.57 |
| Ri | ng/ml | 1.0E-9 | 1.0E-9 | 3.9E0 | 5.1E-2 | 1.5E1 | 1.4E-1 | 1.0E-9 | 1.0E-9 | 2.2E2 | 4.1E-1 | 285 | 8 | 160 | 8 | 0.32 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Rg | ng/ml | 1.0E-9 | 1.0E-9 | 5.5E-2 | 7.4E-2 | 3.4E-1 | 2.1E-1 | 1.0E-9 | 1.0E-9 | 3.3E0 | 5.9E-1 | 284 | 8 | 159 | 8 | 0.46 |
| Rj | ng/ml | 1.0E-9 | 1.0E-9 | 1.3E0 | 3.4E1 | 5.5E0 | 9.5E1 | 1.0E-9 | 1.0E-9 | 8.0E1 | 2.7E2 | 285 | 8 | 160 | 8 | 0.50 |
| Rf | ng/ml | 3.9E-1 | 1.5E0 | 1.0E0 | 4.7E0 | 1.8E0 | 6.6E0 | 7.8E-3 | 5.0E-1 | 1.5E1 | 1.7E1 | 284 | 8 | 159 | 8 | 0.80 |
| Ql | pg/ml | 5.5E0 | 1.4E1 | 1.5E1 | 2.8E1 | 3.1E1 | 3.1E1 | 1.0E-9 | 4.3E-1 | 2.9E2 | 9.3E1 | 290 | 8 | 162 | 8 | 0.71 |
| Qm | pg/ml | 4.4E0 | 1.6E1 | 2.0E1 | 2.6E1 | 3.8E1 | 2.5E1 | 1.0E-9 | 1.0E-9 | 3.6E2 | 6.9E1 | 290 | 8 | 162 | 8 | 0.63 |
| Qn | pg/ml | 6.1E-1 | 8.6E-1 | 7.3E0 | 4.1E0 | 2.4E1 | 5.2E0 | 1.0E-9 | 1.0E-9 | 2.4E2 | 1.2E1 | 290 | 8 | 162 | 8 | 0.60 |
| Nv | pg/ml | 4.1E3 | 1.3E4 | 1.2E4 | 2.9E4 | 4.6E4 | 3.2E4 | 1.0E-9 | 1.3E3 | 1.1E6 | 9.4E4 | 805 | 14 | 264 | 14 | 0.69 |
| Nw | pg/ml | 9.4E3 | 2.7E4 | 1.3E4 | 5.7E4 | 1.6E4 | 7.0E4 | 8.6E1 | 6.2E3 | 2.1E5 | 2.2E5 | 805 | 14 | 264 | 14 | 0.81 |
| Nx | pg/ml | 2.2E2 | 7.5E2 | 4.2E2 | 9.7E2 | 6.6E2 | 1.1E3 | 1.0E-9 | 1.0E-9 | 7.4E3 | 4.1E3 | 805 | 14 | 264 | 14 | 0.68 |
| Ny | pg/ml | 7.1E0 | 3.5E1 | 5.9E1 | 3.2E2 | 8.9E2 | 7.3E2 | 1.0E-9 | 1.0E-9 | 2.5E4 | 2.8E3 | 805 | 14 | 264 | 14 | 0.79 |
| Oa | pg/ml | 1.9E2 | 4.8E2 | 4.7E2 | 9.9E2 | 7.6E2 | 9.9E2 | 1.0E-9 | 6.5E1 | 4.8E3 | 2.4E3 | 290 | 8 | 162 | 8 | 0.70 |
| Oe | pg/ml | 8.8E1 | 4.7E0 | 3.0E2 | 1.9E2 | 7.9E2 | 3.5E2 | 1.0E-9 | 1.0E-9 | 1.9E4 | 1.1E3 | 797 | 14 | 263 | 14 | 0.42 |
| Of | pg/ml | 2.1E2 | 5.8E1 | 6.5E3 | 6.6E3 | 2.9E4 | 1.8E4 | 1.0E-9 | 3.5E0 | 6.2E5 | 6.6E4 | 805 | 14 | 264 | 14 | 0.40 |
| Og | pg/ml | 8.5E-2 | 4.5E-2 | 7.9E-1 | 7.0E-2 | 5.0E0 | 9.0E-2 | 1.0E-9 | 1.0E-9 | 8.3E1 | 3.2E-1 | 805 | 14 | 264 | 14 | 0.37 |
| Oh | pg/ml | 2.7E0 | 2.4E1 | 2.0E1 | 1.2E2 | 1.5E2 | 3.0E2 | 1.0E-9 | 1.1E0 | 3.5E3 | 1.1E3 | 805 | 14 | 264 | 14 | 0.78 |
| Oi | pg/ml | 2.9E0 | 1.0E-9 | 6.6E0 | 4.8E0 | 9.9E0 | 8.6E0 | 1.0E-9 | 1.0E-9 | 8.6E1 | 3.1E1 | 805 | 14 | 264 | 14 | 0.39 |
| Ok | pg/ml | 4.2E2 | 1.1E3 | 5.6E2 | 2.0E3 | 5.2E2 | 2.1E3 | 1.3E1 | 1.8E2 | 5.2E3 | 7.8E3 | 805 | 14 | 264 | 14 | 0.78 |
| Om | pg/ml | 3.9E2 | 1.6E3 | 8.5E2 | 5.8E3 | 2.2E3 | 1.3E4 | 1.0E-9 | 2.5E2 | 3.6E4 | 5.1E4 | 805 | 14 | 264 | 14 | 0.77 |
| On | pg/ml | 1.9E2 | 9.3E2 | 3.0E2 | 1.6E3 | 4.0E2 | 2.2E3 | 1.0E-9 | 2.6E1 | 4.5E3 | 8.5E3 | 805 | 14 | 264 | 14 | 0.78 |
| Or | pg/ml | 1.6E1 | 8.0E1 | 3.9E1 | 1.2E2 | 7.2E1 | 1.7E2 | 1.0E-9 | 1.0E-9 | 5.0E2 | 5.1E2 | 293 | 8 | 161 | 8 | 0.70 |
| Ow | pg/ml | 3.6E1 | 1.5E2 | 1.2E2 | 6.0E2 | 3.0E2 | 1.0E3 | 1.0E-9 | 1.8E1 | 2.7E3 | 3.0E3 | 293 | 8 | 161 | 8 | 0.72 |
| Ou | pg/ml | 5.4E2 | 6.0E2 | 1.1E3 | 2.3E3 | 1.8E3 | 3.2E3 | 1.0E-9 | 3.4E2 | 1.1E4 | 8.2E3 | 293 | 8 | 161 | 8 | 0.62 |
| Ul | ng/ml | 1.0E-9 | 1.0E-9 | 1.7E0 | 1.1E1 | 7.8E0 | 2.1E1 | 1.0E-9 | 1.0E-9 | 1.0E2 | 5.6E1 | 297 | 8 | 164 | 8 | 0.58 |
| Uo | ng/ml | 1.0E-9 | 1.0E-9 | 1.0E-1 | 1.2E-1 | 2.6E-1 | 2.2E-1 | 1.0E-9 | 1.0E-9 | 2.6E0 | 6.3E-1 | 297 | 8 | 164 | 8 | 0.54 |
| Uk | ng/ml | 1.0E-9 | 1.0E-9 | 8.0E-3 | 1.8E-3 | 2.6E-2 | 3.8E-3 | 1.0E-9 | 1.0E-9 | 3.2E-1 | 1.1E-2 | 297 | 8 | 164 | 8 | 0.41 |
| Um | ng/ml | 1.0E-9 | 1.0E-9 | 4.1E-1 | 3.8E-1 | 9.9E-1 | 7.8E-1 | 1.0E-9 | 1.0E-9 | 7.2E0 | 2.3E0 | 297 | 8 | 164 | 8 | 0.51 |
| Uf | ng/ml | 6.8E-2 | 1.2E-1 | 1.5E-1 | 8.3E-1 | 2.8E-1 | 1.9E0 | 1.0E-3 | 3.6E-2 | 2.5E0 | 5.6E0 | 297 | 8 | 164 | 8 | 0.67 |
| Uh | ng/ml | 2.2E0 | 4.1E0 | 3.3E0 | 6.4E0 | 3.4E0 | 6.3E0 | 1.3E-2 | 7.1E-1 | 1.8E1 | 1.7E1 | 297 | 8 | 164 | 8 | 0.65 |
| Un | ng/ml | 2.0E0 | 3.8E0 | 2.2E0 | 6.6E0 | 1.3E0 | 7.8E0 | 1.3E-1 | 1.8E0 | 8.0E0 | 2.5E1 | 297 | 8 | 164 | 8 | 0.81 |
| Ug | ng/ml | 1.5E1 | 1.1E1 | 2.9E1 | 2.0E1 | 3.2E1 | 2.8E1 | 6.9E-1 | 1.7E0 | 2.1E2 | 8.5E1 | 297 | 8 | 164 | 8 | 0.40 |
| Ur | ng/ml | 1.5E-1 | 1.0E-9 | 8.3E-1 | 9.4E-1 | 5.5E0 | 2.6E0 | 1.0E-9 | 1.0E-9 | 9.3E1 | 7.3E0 | 296 | 8 | 163 | 8 | 0.30 |
| Up | ng/ml | 1.0E-9 | 1.0E-9 | 5.2E-3 | 3.0E-1 | 2.3E-2 | 8.4E-1 | 1.0E-9 | 1.0E-9 | 3.3E-1 | 2.4E0 | 296 | 8 | 163 | 8 | 0.55 |
| Us | ng/ml | 4.3E-3 | 1.0E-9 | 2.0E-2 | 2.1E-1 | 4.6E-2 | 5.9E-1 | 1.0E-9 | 1.0E-9 | 4.0E-1 | 1.7E0 | 296 | 8 | 163 | 8 | 0.35 |
| Uv | ng/ml | 2.9E-3 | 2.3E-3 | 1.1E-2 | 8.0E-2 | 3.5E-2 | 1.5E-1 | 1.0E-9 | 1.0E-9 | 4.3E-1 | 4.1E-1 | 296 | 8 | 163 | 8 | 0.50 |
| Ut | ng/ml | 7.2E-1 | 2.3E0 | 2.9E0 | 1.4E1 | 8.6E0 | 2.3E1 | 1.0E-9 | 1.0E-9 | 7.8E1 | 6.5E1 | 296 | 8 | 163 | 8 | 0.70 |
| Uu | ng/ml | 7.5E0 | 3.4E0 | 8.4E0 | 4.7E0 | 5.7E0 | 3.1E0 | 5.5E-1 | 1.2E0 | 4.0E1 | 8.8E0 | 296 | 8 | 163 | 8 | 0.29 |
| Vp | ng/ml | 1.0E-9 | 1.0E-9 | 3.8E-1 | 6.2E0 | 3.4E0 | 1.7E1 | 1.0E-9 | 1.0E-9 | 5.0E1 | 4.9E1 | 297 | 8 | 164 | 8 | 0.61 |
| Vt | ng/ml | 6.8E0 | 1.2E1 | 9.3E0 | 3.5E1 | 9.4E0 | 5.1E1 | 4.3E-1 | 4.5E0 | 8.6E1 | 1.6E2 | 297 | 8 | 164 | 8 | 0.73 |
| Vo | ng/ml | 2.5E1 | 2.5E1 | 2.5E1 | 2.3E1 | 5.1E0 | 5.9E0 | 2.5E0 | 1.1E1 | 4.8E1 | 3.0E1 | 297 | 8 | 164 | 8 | 0.41 |
| Vv | ng/ml | 3.3E0 | 2.0E0 | 6.2E0 | 3.4E0 | 9.7E0 | 5.1E0 | 1.0E-9 | 1.0E-9 | 8.4E1 | 1.4E1 | 296 | 7 | 164 | 7 | 0.38 |
| Oy | pg/ml | 5.1E-1 | 3.7E-1 | 6.0E0 | 3.8E0 | 3.0E1 | 9.1E0 | 1.0E-9 | 1.0E-9 | 4.0E2 | 3.3E1 | 804 | 14 | 263 | 14 | 0.45 |
| Oz | pg/ml | 1.2E-2 | 1.0E-9 | 3.2E-1 | 2.1E0 | 1.4E0 | 7.5E0 | 1.0E-9 | 1.0E-9 | 2.9E1 | 2.8E1 | 804 | 14 | 263 | 14 | 0.42 |
| Pa | pg/ml | 4.0E-1 | 5.8E-1 | 1.5E0 | 1.9E1 | 5.2E0 | 6.0E1 | 1.0E-9 | 1.0E-9 | 8.6E1 | 2.3E2 | 804 | 14 | 263 | 14 | 0.59 |
| Pb | pg/ml | 1.0E-9 | 1.0E-9 | 8.5E-1 | 2.4E0 | 1.7E1 | 8.4E0 | 1.0E-9 | 1.0E-9 | 4.9E2 | 3.2E1 | 804 | 14 | 263 | 14 | 0.44 |
| Pc | pg/ml | 6.4E-2 | 1.0E-9 | 3.6E-1 | 3.3E0 | 8.9E-1 | 9.9E0 | 1.0E-9 | 1.0E-9 | 1.6E1 | 3.8E1 | 804 | 14 | 263 | 14 | 0.50 |
| Pd | pg/ml | 2.0E0 | 5.3E0 | 5.0E0 | 1.8E1 | 3.0E1 | 3.2E1 | 1.0E-9 | 7.3E-2 | 8.4E2 | 1.2E2 | 804 | 14 | 263 | 14 | 0.72 |
| Pe | pg/ml | 2.3E1 | 1.3E2 | 1.1E2 | 1.8E3 | 3.6E2 | 4.2E3 | 1.0E-9 | 2.0E1 | 4.7E3 | 1.5E4 | 804 | 14 | 263 | 14 | 0.78 |
| Pf | pg/ml | 1.8E0 | 1.6E1 | 1.0E1 | 6.1E1 | 5.8E1 | 1.1E2 | 1.0E-9 | 3.3E-1 | 1.5E3 | 4.3E2 | 804 | 14 | 263 | 14 | 0.78 |
| Pg | pg/ml | 3.8E0 | 1.6E1 | 4.6E1 | 1.8E2 | 3.6E2 | 3.7E2 | 1.0E-9 | 4.6E-1 | 7.7E3 | 1.2E3 | 804 | 14 | 263 | 14 | 0.68 |
| Ph | ng/ml | 1.8E-1 | 2.4E-1 | 3.6E-1 | 9.1E-1 | 5.7E-1 | 1.8E0 | 1.0E-9 | 3.5E-3 | 4.4E0 | 5.4E0 | 293 | 8 | 161 | 8 | 0.57 |
| Pi | ng/ml | 2.0E-1 | 4.3E-1 | 3.0E-1 | 1.1E1 | 4.4E-1 | 2.9E1 | 1.0E-9 | 1.0E-9 | 4.8E0 | 8.2E1 | 293 | 8 | 161 | 8 | 0.71 |
| Pj | ng/mL | 5.7E0 | 7.3E0 | 6.3E0 | 7.9E0 | 4.6E0 | 4.6E0 | 3.8E-2 | 1.5E0 | 3.1E1 | 1.7E1 | 293 | 8 | 161 | 8 | 0.62 |
| Pk | ng/ml | 8.8E-3 | 2.7E-2 | 1.3E-2 | 2.1E-1 | 2.0E-2 | 5.3E-1 | 1.0E-9 | 5.4E-3 | 2.5E-1 | 1.5E0 | 293 | 8 | 161 | 8 | 0.79 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| aA | mg/dL | 8.1E-1 | 1.6E0 | 9.4E-1 | 2.1E0 | 4.7E-1 | 1.3E0 | 2.0E-1 | 5.5E-1 | 4.2E0 | 4.7E0 | 2235 | 17 | 391 | 17 | 0.80 |
| aC | mg/mL | 2.7E0 | 2.0E0 | 3.0E0 | 2.4E0 | 1.3E0 | 1.4E0 | 7.7E-1 | 7.4E-1 | 8.2E0 | 5.5E0 | 465 | 9 | 174 | 9 | 0.37 |
| aD | ug/mL | 3.1E0 | 6.0E0 | 4.4E0 | 8.7E0 | 3.4E0 | 6.8E0 | 7.5E-1 | 2.1E0 | 3.1E1 | 2.1E1 | 465 | 9 | 174 | 9 | 0.71 |
| aE | mg/mL | 5.5E-1 | 5.0E-1 | 5.7E-1 | 6.3E-1 | 1.5E-1 | 2.8E-1 | 1.8E-1 | 3.9E-1 | 1.1E0 | 1.2E0 | 465 | 9 | 174 | 9 | 0.49 |
| aF | ng/mL | 2.3E0 | 1.4E0 | 4.1E0 | 4.7E0 | 5.8E0 | 5.6E0 | 4.3E-3 | 5.2E-1 | 5.0E1 | 1.5E1 | 465 | 9 | 174 | 9 | 0.45 |
| aG | mg/mL | 1.3E-1 | 1.3E-1 | 1.5E-1 | 1.7E-1 | 8.1E-2 | 1.2E-1 | 4.3E-2 | 7.3E-2 | 5.0E-1 | 4.2E-1 | 465 | 9 | 174 | 9 | 0.49 |
| aH | ug/mL | 7.5E1 | 6.8E1 | 8.0E1 | 7.7E1 | 4.2E1 | 4.7E1 | 9.6E0 | 1.1E1 | 2.9E2 | 1.5E2 | 465 | 9 | 174 | 9 | 0.48 |
| aI | ug/mL | 1.8E2 | 1.6E2 | 1.8E2 | 1.6E2 | 6.0E1 | 5.2E1 | 4.7E1 | 7.6E1 | 3.7E2 | 2.4E2 | 465 | 9 | 174 | 9 | 0.41 |
| aJ | ug/mL | 2.5E0 | 5.6E0 | 3.2E0 | 6.5E0 | 2.2E0 | 3.6E0 | 7.3E-1 | 1.5E0 | 1.7E1 | 1.2E1 | 465 | 9 | 174 | 9 | 0.79 |
| aK | ng/mL | 1.5E0 | 1.7E0 | 2.3E0 | 3.0E0 | 2.6E0 | 2.3E0 | 2.9E-4 | 1.3E-1 | 1.8E1 | 6.5E0 | 465 | 9 | 174 | 9 | 0.61 |
| aL | mg/mL | 7.9E-1 | 8.0E-1 | 8.0E-1 | 7.9E-1 | 2.5E-1 | 2.4E-1 | 2.2E-1 | 2.7E-1 | 1.7E0 | 1.0E0 | 465 | 9 | 174 | 9 | 0.52 |
| aM | U/mL | 2.2E1 | 6.3E1 | 4.5E1 | 1.8E2 | 9.2E1 | 2.6E2 | 4.2E-2 | 1.9E1 | 1.6E3 | 8.2E2 | 465 | 9 | 174 | 9 | 0.77 |
| aN | U/mL | 1.5E1 | 2.9E1 | 2.3E1 | 3.7E1 | 3.2E1 | 3.4E1 | 2.5E-3 | 1.9E0 | 3.8E2 | 1.1E2 | 465 | 9 | 174 | 9 | 0.65 |
| aO | pg/mL | 3.5E1 | 1.5E2 | 3.3E2 | 6.2E2 | 8.2E2 | 9.4E2 | 6.0E-2 | 2.1E0 | 6.6E3 | 2.4E3 | 465 | 9 | 174 | 9 | 0.63 |
| aP | ng/mL | 1.7E0 | 4.1E0 | 2.0E0 | 3.7E0 | 1.3E0 | 1.7E0 | 5.4E-1 | 1.4E0 | 1.1E1 | 5.8E0 | 465 | 9 | 174 | 9 | 0.80 |
| aQ | ng/mL | 2.9E-1 | 4.8E-1 | 4.4E-1 | 4.4E-1 | 4.6E-1 | 2.5E-1 | 2.0E-4 | 5.1E-2 | 4.0E0 | 9.0E-1 | 465 | 9 | 174 | 9 | 0.58 |
| aR | ng/mL | 1.8E0 | 3.1E0 | 2.8E0 | 3.4E0 | 3.4E0 | 1.7E0 | 1.8E-1 | 9.8E-1 | 3.4E1 | 5.9E0 | 465 | 9 | 174 | 9 | 0.69 |
| aS | ng/mL | 2.8E-1 | 4.0E-1 | 6.9E-1 | 9.0E-1 | 1.8E0 | 1.1E0 | 4.2E-3 | 8.5E-2 | 3.3E1 | 2.8E0 | 465 | 9 | 174 | 9 | 0.52 |
| aU | pg/mL | 7.4E1 | 9.3E1 | 1.2E2 | 1.5E2 | 1.5E2 | 1.6E2 | 7.4E-2 | 9.6E0 | 1.3E3 | 5.1E2 | 465 | 9 | 174 | 9 | 0.58 |
| aV | ng/mL | 6.0E-1 | 1.3E0 | 1.0E0 | 1.5E0 | 1.9E0 | 1.6E0 | 7.6E-4 | 1.0E-1 | 3.3E1 | 5.4E0 | 465 | 9 | 174 | 9 | 0.62 |
| aW | pg/mL | 1.9E1 | 2.6E1 | 2.0E1 | 6.8E1 | 1.9E1 | 1.3E2 | 7.2E-2 | 7.7E0 | 2.4E2 | 4.2E2 | 465 | 9 | 174 | 9 | 0.67 |
| aX | ng/mL | 9.6E0 | 1.2E1 | 1.4E1 | 1.0E1 | 1.5E1 | 8.0E0 | 3.0E-1 | 2.6E0 | 1.4E2 | 2.6E1 | 465 | 9 | 174 | 9 | 0.43 |
| aY | pg/mL | 6.0E1 | 6.6E1 | 7.7E1 | 9.7E1 | 8.4E1 | 6.6E1 | 4.1E-1 | 1.2E1 | 1.2E3 | 2.0E2 | 465 | 9 | 174 | 9 | 0.62 |
| aZ | pg/mL | 2.3E2 | 4.2E2 | 5.3E2 | 4.2E2 | 1.0E3 | 3.4E2 | 1.7E0 | 8.2E1 | 1.2E4 | 1.3E3 | 465 | 9 | 174 | 9 | 0.60 |
| bA | ng/mL | 9.5E0 | 1.2E2 | 3.4E1 | 2.0E2 | 8.4E1 | 2.9E2 | 3.0E-2 | 2.0E0 | 9.4E2 | 9.4E2 | 465 | 9 | 174 | 9 | 0.83 |
| bB | ng/mL | 2.9E2 | 2.9E2 | 3.1E2 | 2.3E2 | 1.7E2 | 1.4E2 | 8.6E0 | 3.3E1 | 1.0E3 | 4.2E2 | 465 | 9 | 174 | 9 | 0.38 |
| bC | ng/mL | 3.5E2 | 3.3E2 | 5.9E2 | 1.1E3 | 7.6E2 | 1.5E3 | 2.7E1 | 1.4E2 | 4.7E3 | 4.7E3 | 465 | 9 | 174 | 9 | 0.58 |
| bE | mg/mL | 5.4E0 | 5.7E0 | 5.7E0 | 5.9E0 | 2.0E0 | 2.7E0 | 1.4E0 | 1.3E0 | 1.3E1 | 1.1E1 | 465 | 9 | 174 | 9 | 0.54 |
| bF | pg/mL | 2.2E1 | 5.7E1 | 1.9E2 | 2.7E2 | 1.0E3 | 6.0E2 | 5.0E-2 | 1.5E1 | 1.1E4 | 1.9E3 | 465 | 9 | 174 | 9 | 0.72 |
| bG | ng/mL | 1.7E0 | 2.6E0 | 2.9E0 | 3.6E0 | 3.6E0 | 4.5E0 | 2.2E-2 | 5.8E-1 | 3.0E1 | 1.5E1 | 465 | 9 | 174 | 9 | 0.56 |
| bH | pg/mL | 5.7E-1 | 9.2E0 | 4.9E0 | 7.0E0 | 1.5E1 | 6.9E0 | 5.7E-1 | 5.7E-1 | 2.8E2 | 2.0E1 | 465 | 9 | 174 | 9 | 0.64 |
| bI | ng/mL | 4.0E-3 | 4.0E-3 | 6.5E-2 | 1.8E-1 | 1.6E-1 | 3.1E-1 | 4.0E-3 | 4.0E-3 | 1.3E0 | 8.8E-1 | 465 | 9 | 174 | 9 | 0.59 |
| bJ | mg/mL | 2.1E0 | 2.9E0 | 2.4E0 | 3.5E0 | 1.9E0 | 2.1E0 | 2.5E-4 | 2.5E-4 | 1.3E1 | 7.0E0 | 465 | 9 | 174 | 9 | 0.66 |
| bL | pg/mL | 3.8E0 | 3.2E0 | 8.4E0 | 8.6E0 | 1.0E1 | 1.0E1 | 4.6E-2 | 1.1E0 | 4.9E1 | 3.2E1 | 465 | 9 | 174 | 9 | 0.55 |
| bM | mg/mL | 1.8E0 | 2.3E0 | 2.1E0 | 3.3E0 | 1.4E0 | 2.7E0 | 9.2E-3 | 1.8E-2 | 8.9E0 | 8.4E0 | 465 | 9 | 174 | 9 | 0.63 |
| bN | ng/mL | 4.6E1 | 3.1E1 | 1.3E2 | 9.4E1 | 2.5E2 | 1.6E2 | 1.4E-1 | 1.4E0 | 1.9E3 | 4.8E2 | 465 | 9 | 174 | 9 | 0.41 |
| bO | ng/mL | 4.0E-2 | 4.0E-2 | 9.5E0 | 1.7E1 | 2.3E1 | 2.7E1 | 4.0E-2 | 4.0E-2 | 2.0E2 | 6.1E1 | 465 | 9 | 174 | 9 | 0.53 |
| bP | mg/mL | 5.2E-1 | 8.2E-1 | 7.3E-1 | 1.0E0 | 6.7E-1 | 9.9E-1 | 8.2E-2 | 2.9E-1 | 4.8E0 | 3.5E0 | 465 | 9 | 174 | 9 | 0.62 |
| bQ | pg/mL | 1.6E1 | 3.5E1 | 6.1E1 | 7.8E1 | 6.3E2 | 7.0E1 | 1.5E-1 | 1.2E1 | 1.3E4 | 2.2E2 | 465 | 9 | 174 | 9 | 0.81 |
| bR | ng/mL | 1.2E-2 | 1.2E-2 | 1.3E-1 | 2.2E-2 | 4.5E-1 | 3.0E-2 | 1.2E-2 | 1.2E-2 | 8.7E0 | 1.0E-1 | 465 | 9 | 174 | 9 | 0.34 |
| bS | pg/mL | 9.4E-1 | 9.4E-1 | 7.2E0 | 3.5E0 | 2.8E1 | 7.8E0 | 9.4E-1 | 9.4E-1 | 3.9E2 | 2.4E1 | 465 | 9 | 174 | 9 | 0.48 |
| bU | ng/mL | 1.1E-1 | 1.3E-2 | 1.9E-1 | 1.1E-1 | 3.7E-1 | 1.4E-1 | 1.3E-2 | 1.3E-2 | 6.6E0 | 4.1E-1 | 465 | 9 | 174 | 9 | 0.41 |
| bV | pg/mL | 4.6E2 | 1.0E3 | 5.7E2 | 9.5E2 | 8.0E2 | 5.1E2 | 1.5E2 | 3.6E2 | 1.7E4 | 2.0E3 | 465 | 9 | 174 | 9 | 0.78 |
| bW | pg/mL | 3.4E2 | 3.7E2 | 5.0E2 | 3.1E3 | 5.6E2 | 8.2E3 | 8.4E1 | 1.5E2 | 6.4E3 | 2.5E4 | 465 | 9 | 174 | 9 | 0.56 |
| bX | ng/mL | 2.5E-5 | 3.9E-3 | 2.7E-3 | 3.2E-3 | 3.4E-3 | 2.6E-3 | 2.5E-5 | 2.5E-5 | 2.0E-2 | 7.1E-3 | 465 | 9 | 174 | 9 | 0.56 |
| bZ | pg/mL | 2.4E2 | 5.1E2 | 9.4E2 | 1.3E3 | 4.3E3 | 1.7E3 | 1.5E-1 | 1.9E2 | 5.8E4 | 5.1E3 | 465 | 9 | 174 | 9 | 0.72 |
| cA | pg/mL | 6.0E-1 | 6.0E-1 | 2.8E0 | 6.0E-1 | 1.8E1 | 0.0E0 | 6.0E-1 | 6.0E-1 | 3.7E2 | 6.0E-1 | 465 | 9 | 174 | 9 | 0.44 |
| cB | ng/mL | 5.1E-2 | 4.7E-2 | 8.2E-2 | 5.5E-2 | 1.0E-1 | 5.0E-2 | 1.7E-3 | 1.7E-3 | 5.7E-1 | 1.6E-1 | 465 | 9 | 174 | 9 | 0.46 |
| cC | pg/mL | 4.6E1 | 5.4E1 | 4.8E1 | 4.1E1 | 4.0E1 | 3.1E1 | 1.0E0 | 1.0E0 | 4.5E2 | 7.3E1 | 465 | 9 | 174 | 9 | 0.49 |
| cD | pg/mL | 5.2E0 | 8.7E0 | 1.3E1 | 1.0E1 | 3.6E1 | 1.5E1 | 3.3E-1 | 3.3E-1 | 4.8E2 | 4.9E1 | 465 | 9 | 174 | 9 | 0.52 |
| cE | pg/mL | 3.9E1 | 1.6E2 | 1.5E2 | 2.9E2 | 4.5E2 | 4.0E2 | 1.2E-1 | 1.2E1 | 3.8E3 | 1.3E3 | 465 | 9 | 174 | 9 | 0.69 |
| cF | pg/mL | 1.2E1 | 5.3E-1 | 2.0E1 | 8.3E0 | 3.1E1 | 1.3E1 | 5.3E-1 | 5.3E-1 | 2.7E2 | 3.6E1 | 465 | 9 | 174 | 9 | 0.38 |
| cG | pg/mL | 4.7E1 | 8.4E1 | 1.1E2 | 1.5E2 | 5.1E2 | 1.5E2 | 6.4E0 | 3.5E1 | 1.0E4 | 4.1E2 | 465 | 9 | 174 | 9 | 0.67 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| cH | uIU/mL | 2.8E0 | 1.4E0 | 6.2E0 | 6.0E0 | 1.2E1 | 1.3E1 | 8.6E-3 | 8.6E-3 | 1.6E2 | 3.9E1 | 465 | 9 | 174 | 9 | 0.37 |
| cI | ng/mL | 5.5E0 | 9.6E0 | 1.2E1 | 2.0E1 | 1.7E1 | 3.6E1 | 1.0E-3 | 1.1E0 | 1.2E2 | 1.2E2 | 465 | 9 | 174 | 9 | 0.54 |
| cJ | ug/mL | 5.6E1 | 7.6E1 | 1.1E2 | 8.9E1 | 1.4E2 | 6.7E1 | 4.0E0 | 8.4E0 | 9.6E2 | 1.9E2 | 465 | 9 | 174 | 9 | 0.52 |
| cK | ng/mL | 3.8E-3 | 3.8E-3 | 4.9E-2 | 4.7E-2 | 1.7E-1 | 6.8E-2 | 3.8E-3 | 3.8E-3 | 1.7E0 | 2.1E-1 | 465 | 9 | 174 | 9 | 0.63 |
| cL | pg/mL | 2.0E2 | 1.9E2 | 4.0E2 | 3.2E2 | 1.3E3 | 3.9E2 | 1.6E1 | 1.4E2 | 2.4E4 | 1.4E3 | 465 | 9 | 174 | 9 | 0.53 |
| cM | pg/mL | 2.7E2 | 2.3E2 | 3.0E2 | 2.5E2 | 1.9E2 | 1.4E2 | 8.7E0 | 5.7E1 | 1.6E3 | 4.7E2 | 465 | 9 | 174 | 9 | 0.43 |
| cN | pg/mL | 1.2E2 | 1.8E2 | 1.3E2 | 1.8E2 | 6.5E1 | 6.6E1 | 3.8E1 | 1.0E2 | 1.1E3 | 2.9E2 | 465 | 9 | 174 | 9 | 0.76 |
| cO | pg/mL | 2.2E2 | 3.0E2 | 3.1E2 | 3.2E2 | 9.1E2 | 1.1E2 | 5.4E1 | 1.6E2 | 1.9E4 | 5.0E2 | 465 | 9 | 174 | 9 | 0.69 |
| cP | ng/mL | 2.5E3 | 3.1E3 | 2.6E3 | 3.2E3 | 9.5E2 | 1.1E3 | 6.2E2 | 2.0E3 | 7.3E3 | 4.5E3 | 465 | 9 | 174 | 9 | 0.66 |
| cQ | ng/mL | 5.3E-2 | 6.7E-2 | 1.4E-1 | 1.9E-1 | 2.8E-1 | 2.8E-1 | 2.0E-3 | 2.0E-3 | 2.2E0 | 8.7E-1 | 465 | 9 | 174 | 9 | 0.60 |
| cR | ng/mL | 2.9E2 | 5.0E2 | 4.8E2 | 6.1E2 | 6.9E2 | 6.4E2 | 2.0E1 | 1.4E2 | 7.7E3 | 2.2E3 | 465 | 9 | 174 | 9 | 0.62 |
| cS | ng/mL | 2.6E2 | 6.0E2 | 4.3E2 | 5.3E2 | 1.1E3 | 3.3E2 | 4.7E1 | 1.4E2 | 2.2E4 | 9.9E2 | 465 | 9 | 174 | 9 | 0.65 |
| cT | ng/mL | 3.5E1 | 1.5E2 | 8.9E1 | 3.7E2 | 2.0E2 | 5.2E2 | 3.7E0 | 1.1E1 | 2.1E3 | 1.4E3 | 465 | 9 | 174 | 9 | 0.78 |
| cU | ng/mL | 5.4E1 | 8.8E1 | 7.7E1 | 1.1E2 | 1.0E2 | 6.1E1 | 5.4E0 | 4.0E1 | 1.6E3 | 2.3E2 | 465 | 9 | 174 | 9 | 0.73 |
| cV | ng/mL | 1.8E-1 | 2.8E-1 | 4.4E-1 | 3.4E-1 | 2.3E0 | 2.7E-1 | 3.4E-4 | 7.6E-2 | 4.7E1 | 9.3E-1 | 465 | 9 | 174 | 9 | 0.62 |
| cW | mIU/mL | 5.1E-2 | 9.4E-2 | 1.4E-1 | 1.5E-1 | 7.0E-1 | 1.2E-1 | 3.7E-4 | 3.6E-2 | 9.7E0 | 3.9E-1 | 465 | 9 | 174 | 9 | 0.74 |
| cX | ng/mL | 1.2E-1 | 6.9E-2 | 1.5E0 | 4.5E-1 | 4.6E0 | 8.2E-1 | 2.3E-4 | 2.3E-4 | 2.8E1 | 2.5E0 | 465 | 9 | 174 | 9 | 0.45 |
| cY | ng/mL | 8.5E0 | 1.2E1 | 1.2E1 | 1.5E1 | 1.3E1 | 1.1E1 | 1.5E-1 | 9.3E-1 | 8.3E1 | 3.6E1 | 465 | 9 | 174 | 9 | 0.60 |
| cZ | ug/mL | 1.4E1 | 1.5E1 | 1.5E1 | 1.5E1 | 6.4E0 | 5.8E0 | 2.7E0 | 3.3E0 | 3.9E1 | 2.3E1 | 465 | 9 | 174 | 9 | 0.55 |
| dΛ | pg/mL | 3.3E2 | 5.4E2 | 3.6E2 | 5.7E2 | 3.0E2 | 2.6E2 | 9.0E1 | 1.9E2 | 5.8E3 | 1.1E3 | 465 | 9 | 174 | 9 | 0.79 |
| dB | ug/mL | 1.7E1 | 2.4E1 | 1.8E1 | 1.9E1 | 1.6E1 | 9.2E0 | 9.4E-1 | 3.7E0 | 2.5E2 | 2.7E1 | 465 | 9 | 174 | 9 | 0.60 |
| dC | nmol/L | 3.5E1 | 3.6E1 | 3.9E1 | 4.1E1 | 1.8E1 | 1.8E1 | 7.6E0 | 2.1E1 | 1.4E2 | 7.9E1 | 465 | 9 | 174 | 9 | 0.54 |
| dD | ug/mL | 3.5E1 | 2.9E1 | 3.6E1 | 3.3E1 | 1.1E1 | 1.2E1 | 1.3E1 | 2.1E1 | 7.6E1 | 5.6E1 | 465 | 9 | 174 | 9 | 0.38 |
| dE | ng/mL | 4.6E-1 | 7.8E-1 | 5.8E-1 | 8.5E-1 | 6.9E-1 | 7.5E-1 | 8.4E-3 | 8.4E-3 | 7.2E0 | 2.4E0 | 465 | 9 | 174 | 9 | 0.64 |
| dF | ng/mL | 2.3E2 | 3.7E2 | 2.9E2 | 5.2E2 | 2.0E2 | 3.5E2 | 5.6E1 | 2.0E2 | 1.3E3 | 1.2E3 | 465 | 9 | 174 | 9 | 0.74 |
| dG | ng/mL | 1.2E1 | 2.4E1 | 1.5E1 | 3.1E1 | 1.3E1 | 2.7E1 | 2.2E0 | 9.8E0 | 1.8E2 | 8.7E1 | 465 | 9 | 174 | 9 | 0.77 |
| dH | pg/mL | 7.5E0 | 1.4E1 | 1.3E1 | 1.3E1 | 3.7E1 | 8.7E0 | 4.0E-2 | 3.2E0 | 6.7E2 | 2.8E1 | 465 | 9 | 174 | 9 | 0.63 |
| dI | pg/mL | 4.6E-1 | 5.0E0 | 2.3E0 | 3.4E0 | 1.6E1 | 2.9E0 | 4.6E-1 | 4.6E-1 | 3.3E2 | 7.5E0 | 465 | 9 | 174 | 9 | 0.70 |
| dJ | ng/mL | 1.9E0 | 2.9E0 | 2.1E0 | 2.7E0 | 1.2E0 | 1.2E0 | 3.2E-2 | 4.9E-1 | 6.9E0 | 4.0E0 | 465 | 9 | 174 | 9 | 0.67 |
| dK | uIU/mL | 1.9E0 | 8.2E-1 | 3.1E0 | 3.6E0 | 6.4E0 | 7.0E0 | 2.8E-4 | 2.9E-2 | 7.9E1 | 2.2E1 | 465 | 9 | 174 | 9 | 0.41 |
| dL | ng/mL | 8.9E2 | 1.3E3 | 1.0E3 | 1.7E3 | 5.3E2 | 1.4E3 | 2.6E2 | 5.3E2 | 3.8E3 | 4.8E3 | 465 | 9 | 174 | 9 | 0.63 |
| dM | pg/mL | 9.7E2 | 2.5E3 | 1.3E3 | 3.6E3 | 1.4E3 | 3.0E3 | 3.4E2 | 8.3E2 | 1.6E4 | 9.6E3 | 465 | 9 | 174 | 9 | 0.81 |
| dN | ug/mL | 9.3E1 | 1.9E2 | 9.8E1 | 1.9E2 | 3.5E1 | 7.1E1 | 1.6E1 | 1.1E2 | 2.4E2 | 3.3E2 | 465 | 9 | 174 | 9 | 0.90 |
| eC | pg/ml | 3.0E2 | 1.9E2 | 3.7E2 | 4.3E2 | 2.6E2 | 7.1E2 | 1.9E1 | 7.1E1 | 1.6E3 | 2.0E3 | 268 | 7 | 162 | 7 | 0.28 |
| fP | ng/ml | 2.7E2 | 3.9E2 | 3.1E2 | 3.0E2 | 2.0E2 | 1.5E2 | 1.8E0 | 9.5E1 | 1.6E3 | 4.4E2 | 314 | 7 | 164 | 7 | 0.53 |
| fR | ng/ml | 1.4E5 | 2.7E5 | 1.8E5 | 3.1E5 | 1.4E5 | 2.3E5 | 2.9E4 | 1.9E2 | 8.3E5 | 6.8E5 | 302 | 8 | 87 | 8 | 0.67 |
| tF | pg/mL | 1.6E3 | 2.1E3 | 1.4E4 | 2.0E3 | 4.2E4 | 1.3E3 | 1.2E1 | 2.6E2 | 3.2E5 | 3.7E3 | 269 | 7 | 162 | 7 | 0.49 |

Figure 9.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 6.6E1 | 7.3E1 | 7.7E1 | 1.1E2 | 5.2E1 | 8.9E1 | 2.0E0 | 7.0E0 | 4.4E2 | 4.0E2 | 1475 | 35 | 252 | 35 | 0.58 |
| Ad | ug/mL | 3.4E-2 | 6.2E-2 | 6.8E-2 | 9.1E-2 | 8.7E-2 | 8.4E-2 | 6.8E-4 | 2.7E-4 | 5.4E-1 | 3.5E-1 | 421 | 25 | 165 | 25 | 0.65 |
| Af | ng/mL | 1.1E0 | 4.5E-1 | 1.6E1 | 1.3E1 | 6.2E1 | 4.3E1 | 1.7E-3 | 1.7E-3 | 5.3E2 | 2.2E2 | 421 | 25 | 165 | 25 | 0.46 |
| Aj | ug/mL | 1.8E0 | 3.3E0 | 2.7E0 | 2.8E0 | 2.5E0 | 2.4E0 | 1.5E-3 | 1.8E-3 | 6.1E0 | 6.1E0 | 421 | 25 | 165 | 25 | 0.52 |
| Al | mg/mL | 8.7E-5 | 9.0E-5 | 2.5E-4 | 1.9E-4 | 4.1E-4 | 3.7E-4 | 2.5E-6 | 8.0E-6 | 2.2E-3 | 1.9E-3 | 421 | 25 | 165 | 25 | 0.53 |
| An | U/mL | 4.9E1 | 7.7E1 | 1.6E2 | 2.1E2 | 4.5E2 | 2.7E2 | 9.8E-4 | 8.6E-1 | 5.5E3 | 9.8E2 | 421 | 25 | 165 | 25 | 0.60 |
| Ao | pg/mL | 8.6E1 | 1.2E2 | 5.4E2 | 1.3E2 | 3.6E3 | 1.2E2 | 1.5E0 | 6.1E0 | 3.9E4 | 6.1E2 | 421 | 25 | 165 | 25 | 0.56 |
| Ap | ng/mL | 2.9E1 | 4.6E1 | 4.5E1 | 5.5E1 | 5.0E1 | 4.1E1 | 8.4E-5 | 2.1E0 | 3.3E2 | 1.8E2 | 421 | 25 | 165 | 25 | 0.63 |
| Ar | ng/mL | 8.5E-1 | 1.9E0 | 1.3E1 | 4.2E0 | 2.0E2 | 5.0E0 | 3.4E-3 | 3.4E-3 | 4.1E3 | 2.0E1 | 421 | 25 | 165 | 25 | 0.64 |
| As | ng/mL | 8.7E-3 | 1.2E-2 | 1.3E-2 | 2.0E-2 | 1.7E-2 | 3.2E-2 | 1.7E-3 | 1.7E-3 | 1.3E-1 | 1.2E-1 | 421 | 25 | 165 | 25 | 0.55 |
| Aw | pg/mL | 1.6E1 | 1.7E1 | 1.6E1 | 1.8E1 | 6.0E0 | 6.9E0 | 2.9E-2 | 2.9E-2 | 4.8E1 | 3.8E1 | 421 | 25 | 165 | 25 | 0.58 |
| Ax | ng/mL | 2.0E0 | 4.5E0 | 1.3E1 | 2.1E1 | 5.3E1 | 4.4E1 | 1.9E-2 | 3.9E-2 | 7.7E2 | 2.0E2 | 421 | 25 | 165 | 25 | 0.59 |
| Ba | ng/mL | 5.7E1 | 1.7E2 | 4.1E2 | 7.4E2 | 1.1E3 | 1.7E3 | 2.7E-1 | 6.9E-1 | 8.1E3 | 8.1E3 | 421 | 25 | 165 | 25 | 0.64 |
| Bb | ng/mL | 2.9E0 | 4.9E0 | 6.3E0 | 5.8E0 | 1.5E1 | 4.3E0 | 4.1E-3 | 4.2E-1 | 2.5E2 | 1.6E1 | 421 | 25 | 165 | 25 | 0.60 |
| Bc | ng/mL | 3.4E1 | 7.2E1 | 9.9E1 | 1.2E2 | 1.9E2 | 2.0E2 | 1.1E-1 | 2.4E0 | 1.2E3 | 1.0E3 | 421 | 25 | 165 | 25 | 0.64 |
| Bg | ng/mL | 7.4E-2 | 2.9E-1 | 4.5E0 | 7.3E-1 | 2.1E1 | 1.2E0 | 5.3E-4 | 5.3E-4 | 2.5E2 | 4.8E0 | 421 | 25 | 165 | 25 | 0.61 |
| Bn | ng/mL | 5.6E-2 | 5.6E-2 | 1.2E0 | 1.4E0 | 2.0E0 | 2.4E0 | 5.6E-2 | 5.6E-2 | 9.7E0 | 7.6E0 | 421 | 25 | 165 | 25 | 0.50 |
| Bo | ng/mL | 1.2E1 | 1.7E1 | 1.4E1 | 1.7E1 | 1.9E1 | 1.2E1 | 1.6E-2 | 1.6E-2 | 2.8E2 | 3.6E1 | 421 | 25 | 165 | 25 | 0.60 |
| Ch | uIU/mL | 1.1E0 | 9.8E-1 | 1.9E1 | 6.7E0 | 1.1E2 | 2.1E1 | 3.4E-3 | 1.0E-1 | 1.8E3 | 1.1E2 | 421 | 25 | 165 | 25 | 0.48 |
| Co | pg/mL | 3.6E1 | 6.3E1 | 1.8E2 | 7.9E1 | 9.9E2 | 7.7E1 | 1.5E-1 | 9.1E0 | 1.7E4 | 3.9E2 | 421 | 25 | 165 | 25 | 0.62 |
| Cp | ng/mL | 2.2E1 | 2.4E1 | 2.8E1 | 2.9E1 | 3.2E1 | 2.1E1 | 6.0E-1 | 6.0E-1 | 3.7E2 | 9.9E1 | 421 | 25 | 165 | 25 | 0.57 |
| Cq | ng/mL | 2.8E-2 | 3.9E-2 | 1.4E-1 | 6.6E-2 | 8.8E-1 | 1.1E-1 | 8.0E-4 | 8.0E-4 | 1.7E1 | 4.9E-1 | 421 | 25 | 165 | 25 | 0.54 |
| Cs | ng/mL | 5.3E1 | 1.6E2 | 2.7E2 | 4.6E2 | 8.0E2 | 1.1E3 | 2.7E-2 | 1.5E0 | 1.1E4 | 5.3E3 | 421 | 25 | 165 | 25 | 0.60 |
| Ct | ng/mL | 8.6E-1 | 3.8E-1 | 3.5E1 | 1.7E1 | 1.0E2 | 3.8E1 | 1.1E-4 | 1.5E-2 | 6.2E2 | 1.6E2 | 421 | 25 | 165 | 25 | 0.46 |
| Cu | ng/mL | 2.3E-1 | 3.8E-1 | 4.1E-1 | 3.9E-1 | 7.8E-1 | 2.4E-1 | 9.0E-5 | 1.8E-2 | 9.2E0 | 9.4E-1 | 421 | 25 | 165 | 25 | 0.63 |
| Cv | ng/mL | 4.7E0 | 9.5E0 | 2.2E1 | 3.3E1 | 6.0E1 | 9.2E1 | 1.4E-4 | 1.8E-2 | 5.3E2 | 4.7E2 | 421 | 25 | 165 | 25 | 0.57 |
| Cw | mIU/mL | 3.0E-2 | 3.4E-2 | 3.9E-2 | 3.7E-2 | 3.3E-2 | 2.2E-2 | 8.9E-4 | 1.5E-4 | 2.4E-1 | 9.0E-2 | 421 | 25 | 165 | 25 | 0.52 |
| Cx | ng/mL | 2.6E-1 | 5.6E-2 | 5.8E1 | 6.4E1 | 1.1E2 | 1.3E2 | 9.3E-5 | 9.3E-5 | 3.9E2 | 3.9E2 | 421 | 25 | 165 | 25 | 0.51 |
| Db | ug/mL | 7.5E0 | 6.6E0 | 9.1E0 | 1.4E1 | 8.7E0 | 2.8E1 | 4.5E-1 | 8.5E-1 | 1.0E2 | 1.4E2 | 421 | 25 | 165 | 25 | 0.49 |
| Dc | nmol/L | 1.8E-2 | 1.7E-2 | 5.5E-2 | 2.5E-2 | 1.3E-1 | 2.5E-2 | 5.2E-6 | 3.0E-4 | 1.6E0 | 9.9E-2 | 421 | 25 | 165 | 25 | 0.48 |
| Dd | ug/mL | 7.1E-2 | 3.7E-2 | 1.8E-1 | 1.1E-1 | 2.6E-1 | 2.0E-1 | 8.3E-5 | 4.8E-4 | 1.9E0 | 8.9E-1 | 421 | 25 | 165 | 25 | 0.38 |
| De | ng/mL | 3.4E-3 | 3.4E-3 | 7.6E-2 | 5.8E-2 | 1.4E-1 | 1.1E-1 | 3.4E-3 | 3.4E-3 | 9.2E-1 | 5.0E-1 | 421 | 25 | 165 | 25 | 0.47 |
| Dg | ng/mL | 2.8E1 | 5.7E1 | 4.1E1 | 6.5E1 | 3.9E1 | 4.6E1 | 1.0E-1 | 2.1E0 | 1.9E2 | 1.9E2 | 421 | 25 | 165 | 25 | 0.66 |
| Di | pg/mL | 1.9E0 | 1.3E0 | 2.2E0 | 1.6E0 | 2.0E0 | 1.4E0 | 1.8E-1 | 1.8E-1 | 1.3E1 | 4.0E0 | 421 | 25 | 165 | 25 | 0.43 |
| Dk | uIU/mL | 1.6E-2 | 2.1E-2 | 9.0E-2 | 2.9E-2 | 5.3E-1 | 3.1E-2 | 1.1E-4 | 1.1E-4 | 8.9E0 | 1.4E-1 | 421 | 25 | 165 | 25 | 0.54 |
| Dl | ng/mL | 2.1E2 | 3.2E2 | 3.0E2 | 4.7E2 | 2.8E2 | 4.0E2 | 1.7E0 | 6.4E0 | 1.5E3 | 1.3E3 | 421 | 25 | 165 | 25 | 0.62 |
| Do | ng/ml | 4.7E-1 | 6.3E-1 | 9.5E-1 | 1.1E0 | 2.4E0 | 1.6E0 | 3.6E-2 | 3.6E-2 | 1.9E1 | 4.6E0 | 79 | 7 | 57 | 7 | 0.60 |
| Dp | ng/ml | 2.4E0 | 2.8E0 | 5.2E0 | 4.9E0 | 7.5E0 | 6.1E0 | 3.7E-3 | 2.1E-1 | 4.6E1 | 2.2E1 | 249 | 22 | 162 | 22 | 0.51 |
| Dr | pg/ml | 2.5E1 | 1.7E1 | 5.2E1 | 1.9E1 | 7.1E1 | 2.1E1 | 7.5E-1 | 7.5E-1 | 5.2E2 | 6.0E1 | 154 | 8 | 87 | 8 | 0.34 |
| Dq | Absorbance | 1.7E-3 | 1.7E-3 | 3.0E-2 | 1.7E-3 | 1.3E-1 | 0.0E0 | 1.7E-3 | 1.7E-3 | 8.3E-1 | 1.7E-3 | 77 | 7 | 56 | 7 | 0.36 |
| Dv | pg/ml | 1.0E0 | 7.6E-1 | 1.2E0 | 9.0E-1 | 1.2E0 | 7.4E-1 | 2.2E-2 | 2.2E-2 | 4.7E0 | 2.3E0 | 56 | 7 | 36 | 7 | 0.46 |
| Ef | ng/ml | 1.3E-1 | 1.8E-1 | 8.3E-1 | 7.6E-1 | 1.8E0 | 1.8E0 | 5.7E-4 | 1.3E-3 | 1.0E1 | 8.4E0 | 304 | 23 | 164 | 23 | 0.54 |
| Wm | % | 5.9E-1 | 7.6E-1 | 2.0E1 | 1.3E2 | 1.5E2 | 3.4E2 | 5.4E-2 | 8.5E-2 | 2.4E3 | 1.3E3 | 341 | 25 | 184 | 25 | 0.55 |
| Ed | pg/ml | 5.2E-1 | 5.2E-1 | 5.7E1 | 4.1E1 | 4.6E2 | 8.5E1 | 5.2E-1 | 5.2E-1 | 7.3E3 | 3.5E2 | 249 | 22 | 161 | 22 | 0.49 |
| Tj | pg/mL | 3.7E-1 | 3.7E-1 | 5.9E1 | 7.2E0 | 3.1E2 | 1.2E1 | 3.6E-1 | 3.7E-1 | 3.5E3 | 4.0E1 | 304 | 23 | 167 | 23 | 0.50 |
| Po | pg/ml | 4.8E-1 | 1.9E0 | 8.6E0 | 1.7E1 | 2.5E1 | 4.0E1 | 8.0E-3 | 2.6E-2 | 2.7E2 | 2.2E2 | 690 | 37 | 279 | 37 | 0.58 |
| Em | ng/ml | 2.9E-3 | 2.9E-3 | 5.5E-2 | 1.2E-1 | 1.1E-1 | 2.0E-1 | 1.9E-16 | 2.9E-3 | 6.0E-1 | 4.7E-1 | 190 | 9 | 87 | 9 | 0.55 |
| Et | ng/ml | 1.3E3 | 2.8E3 | 1.5E3 | 2.6E3 | 1.1E3 | 1.4E3 | 7.5E1 | 9.1E1 | 5.0E3 | 5.0E3 | 689 | 37 | 279 | 37 | 0.73 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fa | ng/ml | 3.9E1 | 7.6E1 | 1.2E2 | 8.4E1 | 5.6E2 | 7.1E1 | 3.4E-2 | 2.6E-1 | 8.0E3 | 2.9E2 | 243 | 22 | 159 | 22 | 0.62 |
| Ez | ng/ml | 3.8E0 | 3.7E0 | 1.8E1 | 1.1E1 | 5.5E1 | 1.4E1 | 1.3E-2 | 1.3E-2 | 7.1E2 | 4.6E1 | 249 | 22 | 162 | 22 | 0.50 |
| Fb | ng/ml | 2.5E1 | 2.3E1 | 2.2E1 | 2.3E1 | 1.2E1 | 1.0E1 | 6.6E-1 | 8.1E-1 | 5.7E1 | 4.1E1 | 244 | 22 | 159 | 22 | 0.51 |
| Ex | ng/ml | 7.4E-2 | 1.3E-1 | 2.4E-1 | 2.1E-1 | 7.2E-1 | 2.3E-1 | 3.5E-5 | 1.7E-4 | 8.9E0 | 9.2E-1 | 224 | 17 | 113 | 17 | 0.63 |
| Fn | ng/ml | 2.1E-1 | 2.1E-1 | 6.1E0 | 5.0E0 | 2.7E1 | 7.8E0 | 1.1E-14 | 1.1E-14 | 4.2E2 | 2.6E1 | 249 | 22 | 162 | 22 | 0.50 |
| Fp | ng/ml | 1.2E1 | 3.1E1 | 2.3E1 | 4.3E1 | 2.8E1 | 3.6E1 | 6.0E-3 | 2.8E-1 | 1.4E2 | 1.4E2 | 720 | 37 | 280 | 37 | 0.68 |
| Fr | ng/ml | 3.2E4 | 9.6E4 | 1.1E5 | 2.2E5 | 1.7E5 | 2.8E5 | 1.9E2 | 1.2E3 | 9.0E5 | 8.9E5 | 823 | 39 | 284 | 39 | 0.66 |
| Fw | pg/ml | 1.1E0 | 2.9E0 | 6.2E1 | 4.4E1 | 5.0E2 | 1.4E2 | 1.1E-14 | 1.7E-14 | 6.9E3 | 6.3E2 | 304 | 23 | 165 | 23 | 0.54 |
| Fy | ng/ml | 3.5E1 | 4.8E1 | 5.5E1 | 6.0E1 | 5.6E1 | 4.9E1 | 1.2E-1 | 9.7E-1 | 3.3E2 | 2.1E2 | 246 | 22 | 161 | 22 | 0.57 |
| Gc | ng/ml | 9.9E1 | 1.2E2 | 1.5E2 | 1.6E2 | 1.7E2 | 1.4E2 | 6.4E0 | 2.2E1 | 1.2E3 | 4.4E2 | 166 | 8 | 90 | 8 | 0.54 |
| Gn | U/ml | 3.6E-1 | 5.6E-3 | 1.3E0 | 7.6E-2 | 3.1E0 | 1.8E-1 | 1.3E-3 | 1.3E-3 | 3.0E1 | 5.3E-1 | 148 | 8 | 85 | 8 | 0.15 |
| Gl | pg/ml | 7.4E3 | 6.2E3 | 1.1E4 | 8.4E3 | 9.4E3 | 7.0E3 | 9.1E1 | 1.0E3 | 3.4E4 | 2.9E4 | 295 | 23 | 164 | 23 | 0.46 |
| Gp | U/ml | 1.7E0 | 1.8E0 | 4.1E0 | 3.7E0 | 6.6E0 | 5.4E0 | 1.3E-3 | 1.5E-2 | 6.7E1 | 2.1E1 | 306 | 23 | 165 | 23 | 0.47 |
| Gt | ng/ml | 2.2E-3 | 2.2E-3 | 1.3E-1 | 2.2E-3 | 4.5E-1 | 0.0E0 | 2.2E-3 | 2.2E-3 | 3.3E0 | 2.2E-3 | 61 | 7 | 42 | 7 | 0.33 |
| Gw | ng/ml | 5.5E0 | 9.6E0 | 1.6E1 | 1.2E1 | 5.1E1 | 7.8E0 | 8.3E-1 | 3.6E0 | 4.4E2 | 2.2E1 | 79 | 7 | 57 | 7 | 0.67 |
| Gz | ug/ml | 1.4E0 | 1.2E0 | 9.2E0 | 5.4E0 | 3.9E1 | 6.4E0 | 2.9E-16 | 3.8E-3 | 4.8E2 | 2.1E1 | 165 | 16 | 106 | 16 | 0.49 |
| Ha | ng/ml | 2.6E0 | 2.2E0 | 9.9E0 | 3.9E0 | 2.1E1 | 6.4E0 | 1.7E-2 | 1.7E-2 | 1.3E2 | 3.0E1 | 247 | 22 | 161 | 22 | 0.43 |
| Nm | pg/ml | 1.4E4 | 3.1E4 | 3.0E4 | 5.4E4 | 8.2E4 | 8.0E4 | 1.0E-9 | 1.0E-9 | 1.6E6 | 4.4E5 | 693 | 37 | 281 | 37 | 0.63 |
| Nn | pg/ml | 1.5E2 | 4.6E2 | 1.9E3 | 4.3E3 | 8.6E3 | 1.3E4 | 1.0E-9 | 1.0E-9 | 1.0E5 | 6.9E4 | 693 | 37 | 281 | 37 | 0.60 |
| No | pg/ml | 1.4E1 | 3.5E1 | 3.5E1 | 1.0E2 | 1.2E2 | 2.8E2 | 1.0E-9 | 1.0E-9 | 2.5E3 | 1.4E3 | 693 | 37 | 281 | 37 | 0.64 |
| Nq | pg/ml | 1.9E0 | 8.9E-2 | 1.7E1 | 2.8E1 | 7.0E1 | 6.4E1 | 1.0E-9 | 1.0E-9 | 1.2E3 | 2.7E2 | 693 | 37 | 281 | 37 | 0.48 |
| Nr | pg/ml | 6.5E-1 | 4.0E0 | 3.1E1 | 2.5E2 | 2.0E2 | 1.4E3 | 1.0E-9 | 1.0E-9 | 4.1E3 | 8.5E3 | 693 | 37 | 281 | 37 | 0.62 |
| Ns | pg/ml | 1.0E-9 | 1.0E-9 | 9.7E0 | 4.0E0 | 5.6E1 | 2.0E1 | 1.0E-9 | 1.0E-9 | 1.1E3 | 1.2E2 | 693 | 37 | 281 | 37 | 0.51 |
| Nt | pg/ml | 1.0E2 | 1.5E2 | 1.3E2 | 2.0E2 | 1.1E2 | 1.5E2 | 1.0E-9 | 2.6E1 | 1.5E3 | 6.8E2 | 693 | 37 | 281 | 37 | 0.68 |
| Nu | pg/ml | 1.9E1 | 8.8E1 | 5.4E1 | 1.0E2 | 9.4E1 | 1.1E2 | 1.0E-9 | 1.0E-9 | 8.9E2 | 5.8E2 | 693 | 37 | 281 | 37 | 0.66 |
| Lu | pg/ml | 1.0E4 | 1.0E4 | 1.6E4 | 2.0E4 | 4.6E4 | 3.9E4 | 3.5E2 | 1.0E3 | 7.5E5 | 2.3E5 | 695 | 37 | 281 | 37 | 0.50 |
| Lv | pg/ml | 1.0E-9 | 2.5E1 | 1.1E1 | 2.8E1 | 2.1E1 | 3.8E1 | 1.0E-9 | 1.0E-9 | 2.4E2 | 1.9E2 | 695 | 37 | 281 | 37 | 0.66 |
| Lw | pg/ml | 1.0E-9 | 1.0E-9 | 3.7E-1 | 1.0E0 | 4.0E0 | 4.4E0 | 1.0E-9 | 1.0E-9 | 8.0E1 | 2.1E1 | 695 | 37 | 281 | 37 | 0.53 |
| Lx | pg/ml | 1.0E-9 | 1.1E2 | 1.3E2 | 6.3E2 | 4.2E2 | 1.8E3 | 1.0E-9 | 1.0E-9 | 6.2E3 | 1.0E4 | 695 | 37 | 281 | 37 | 0.72 |
| Ly | pg/ml | 1.0E-9 | 1.4E1 | 1.0E1 | 2.0E1 | 2.0E1 | 2.4E1 | 1.0E-9 | 1.0E-9 | 1.6E2 | 8.7E1 | 695 | 37 | 281 | 37 | 0.65 |
| Lz | pg/ml | 1.0E-9 | 1.0E-9 | 4.9E0 | 5.7E0 | 3.4E1 | 3.4E1 | 1.0E-9 | 1.0E-9 | 6.0E2 | 2.1E2 | 695 | 37 | 281 | 37 | 0.48 |
| Ma | pg/ml | 2.7E2 | 3.0E2 | 1.4E3 | 1.5E3 | 3.8E3 | 2.4E3 | 1.0E-9 | 3.0E0 | 6.5E4 | 1.0E4 | 695 | 37 | 281 | 37 | 0.56 |
| Mb | pg/ml | 2.5E1 | 2.9E1 | 3.1E1 | 3.5E1 | 1.6E1 | 1.7E1 | 5.4E0 | 1.6E1 | 2.1E2 | 8.7E1 | 695 | 37 | 281 | 37 | 0.57 |
| Mc | pg/ml | 1.0E-9 | 1.0E-9 | 3.6E-2 | 1.0E-9 | 5.3E-1 | 0.0E0 | 1.0E-9 | 1.0E-9 | 1.3E1 | 1.0E-9 | 695 | 37 | 281 | 37 | 0.49 |
| Md | pg/ml | 1.0E-9 | 1.0E-9 | 5.0E-1 | 2.1E-2 | 4.0E0 | 9.7E-2 | 1.0E-9 | 1.0E-9 | 6.8E1 | 5.5E-1 | 695 | 37 | 281 | 37 | 0.49 |
| Me | pg/ml | 3.2E1 | 2.8E1 | 3.1E1 | 2.9E1 | 2.0E1 | 1.4E1 | 1.0E-9 | 3.2E0 | 3.2E2 | 7.9E1 | 695 | 37 | 281 | 37 | 0.45 |
| Mf | pg/ml | 1.0E-9 | 1.0E-9 | 5.0E-1 | 5.5E-1 | 2.9E0 | 1.6E0 | 1.0E-9 | 1.0E-9 | 5.6E1 | 8.4E0 | 695 | 37 | 281 | 37 | 0.56 |
| Mg | pg/ml | 1.7E0 | 6.4E0 | 7.3E0 | 9.1E0 | 1.2E1 | 1.1E1 | 1.0E-9 | 1.0E-9 | 9.2E1 | 4.0E1 | 695 | 37 | 281 | 37 | 0.60 |
| Mh | pg/ml | 1.0E-9 | 1.0E-9 | 1.5E0 | 1.9E0 | 1.1E1 | 6.7E0 | 1.0E-9 | 1.0E-9 | 1.6E2 | 3.8E1 | 695 | 37 | 281 | 37 | 0.50 |
| Mi | pg/ml | 1.0E-9 | 1.0E-9 | 4.9E-1 | 8.8E0 | 5.8E0 | 2.9E1 | 1.0E-9 | 1.0E-9 | 1.2E2 | 1.6E2 | 695 | 37 | 281 | 37 | 0.57 |
| Mj | pg/ml | 1.0E-9 | 1.0E-9 | 4.8E0 | 1.6E1 | 2.7E1 | 8.9E1 | 1.0E-9 | 1.0E-9 | 4.6E2 | 5.4E2 | 695 | 37 | 281 | 37 | 0.53 |
| Mk | pg/ml | 9.1E-1 | 3.8E0 | 1.5E1 | 1.6E2 | 9.7E1 | 9.1E2 | 1.0E-9 | 1.0E-9 | 1.7E3 | 5.6E3 | 695 | 37 | 281 | 37 | 0.52 |
| Ml | pg/ml | 1.0E-9 | 1.0E-9 | 5.8E0 | 1.3E0 | 8.2E1 | 3.3E0 | 1.0E-9 | 1.0E-9 | 2.1E3 | 1.6E1 | 695 | 37 | 281 | 37 | 0.53 |
| Mm | pg/ml | 5.4E2 | 1.0E3 | 9.2E2 | 1.8E3 | 1.1E3 | 2.0E3 | 1.0E-9 | 1.0E-9 | 7.3E3 | 6.9E3 | 695 | 37 | 281 | 37 | 0.63 |
| Mn | pg/ml | 5.3E0 | 6.7E0 | 1.0E1 | 1.1E1 | 2.4E1 | 1.4E1 | 1.0E-9 | 1.0E-9 | 3.5E2 | 6.6E1 | 695 | 37 | 281 | 37 | 0.56 |
| Mp | pg/ml | 1.0E-9 | 4.8E0 | 8.4E0 | 2.1E1 | 2.9E1 | 4.4E1 | 1.0E-9 | 1.0E-9 | 6.3E2 | 1.8E2 | 694 | 37 | 281 | 37 | 0.59 |
| Mq | pg/ml | 1.0E-9 | 1.0E-9 | 2.8E0 | 7.5E0 | 1.6E1 | 2.9E1 | 1.0E-9 | 1.0E-9 | 2.1E2 | 1.4E2 | 694 | 37 | 281 | 37 | 0.51 |
| Mr | pg/ml | 1.0E-9 | 1.0E-9 | 1.6E1 | 3.8E2 | 7.9E1 | 2.0E3 | 1.0E-9 | 1.0E-9 | 1.4E3 | 1.2E4 | 694 | 37 | 281 | 37 | 0.55 |
| Ms | pg/ml | 4.1E2 | 5.0E2 | 5.6E2 | 5.4E2 | 6.4E2 | 4.9E2 | 1.0E-9 | 1.0E-9 | 5.6E3 | 1.7E3 | 694 | 37 | 281 | 37 | 0.51 |
| Mt | pg/ml | 1.0E-9 | 1.8E0 | 6.8E0 | 9.7E0 | 4.6E1 | 1.8E1 | 1.0E-9 | 1.0E-9 | 8.7E2 | 7.8E1 | 694 | 37 | 281 | 37 | 0.72 |
| Mu | pg/ml | 1.0E-9 | 1.0E-9 | 1.3E0 | 2.2E0 | 1.2E1 | 5.6E0 | 1.0E-9 | 1.0E-9 | 2.3E2 | 2.7E1 | 694 | 37 | 281 | 37 | 0.56 |
| Mv | pg/ml | 1.0E-9 | 1.0E-9 | 6.8E1 | 7.3E1 | 3.3E2 | 1.9E2 | 1.0E-9 | 1.0E-9 | 4.5E3 | 9.3E2 | 694 | 37 | 281 | 37 | 0.54 |
| Mw | pg/ml | 3.2E1 | 6.3E1 | 4.3E2 | 1.0E3 | 2.9E3 | 3.1E3 | 1.0E-9 | 1.0E-9 | 6.2E4 | 1.7E4 | 694 | 37 | 281 | 37 | 0.60 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Mx | pg/ml | 1.0E-9 | 1.0E-9 | 2.3E-1 | 2.3E-1 | 1.4E0 | 5.8E-1 | 1.0E-9 | 1.0E-9 | 3.2E1 | 3.0E0 | 694 | 37 | 281 | 37 | 0.56 |
| My | pg/ml | 1.0E-9 | 1.0E-9 | 4.3E2 | 3.5E2 | 2.9E3 | 8.8E2 | 1.0E-9 | 1.0E-9 | 3.9E4 | 4.1E3 | 694 | 37 | 281 | 37 | 0.57 |
| Mz | pg/ml | 1.0E1 | 2.1E1 | 2.4E1 | 4.5E1 | 6.8E1 | 6.3E1 | 1.0E-9 | 1.0E-9 | 1.2E3 | 3.0E2 | 694 | 37 | 281 | 37 | 0.64 |
| Na | pg/ml | 1.0E-9 | 1.0E-9 | 7.7E-1 | 7.8E-1 | 2.9E0 | 2.5E0 | 1.0E-9 | 1.0E-9 | 3.6E1 | 1.1E1 | 694 | 37 | 281 | 37 | 0.51 |
| Nb | pg/ml | 1.9E0 | 2.8E0 | 4.0E0 | 1.1E1 | 1.3E1 | 3.9E1 | 1.0E-9 | 1.0E-9 | 2.3E2 | 2.4E2 | 694 | 37 | 281 | 37 | 0.59 |
| Nc | pg/ml | 3.8E2 | 1.5E2 | 6.2E2 | 2.4E2 | 7.7E2 | 3.4E2 | 1.0E-9 | 1.0E-9 | 9.2E3 | 1.4E3 | 694 | 37 | 281 | 37 | 0.34 |
| Nd | pg/ml | 2.9E1 | 6.8E0 | 2.7E1 | 2.5E1 | 5.0E1 | 3.7E1 | 1.0E-9 | 1.0E-9 | 1.2E3 | 1.4E2 | 694 | 37 | 281 | 37 | 0.40 |
| Ne | pg/ml | 4.7E2 | 2.5E2 | 6.0E2 | 3.1E2 | 5.9E2 | 3.1E2 | 1.0E-9 | 1.0E-9 | 7.0E3 | 1.6E3 | 694 | 37 | 281 | 37 | 0.34 |
| Nf | pg/ml | 1.0E-9 | 1.0E-9 | 3.2E0 | 3.2E0 | 1.0E1 | 1.4E1 | 1.0E-9 | 1.0E-9 | 1.6E2 | 8.2E1 | 694 | 37 | 281 | 37 | 0.42 |
| Ng | pg/ml | 2.1E1 | 2.7E1 | 1.3E2 | 1.2E2 | 2.6E2 | 1.7E2 | 1.0E-9 | 1.0E-9 | 2.3E3 | 5.0E2 | 694 | 37 | 281 | 37 | 0.53 |
| Nh | pg/ml | 7.1E1 | 3.3E1 | 9.4E1 | 5.6E1 | 8.6E1 | 5.7E1 | 1.0E-9 | 1.0E-9 | 5.6E2 | 2.1E2 | 694 | 37 | 281 | 37 | 0.34 |
| Ni | pg/ml | 1.0E-9 | 2.5E1 | 7.6E1 | 9.6E1 | 1.2E2 | 1.2E2 | 1.0E-9 | 1.0E-9 | 7.1E2 | 3.9E2 | 694 | 37 | 281 | 37 | 0.55 |
| Nj | pg/ml | 8.2E0 | 2.8E0 | 1.2E1 | 6.7E0 | 1.2E1 | 1.1E1 | 1.0E-9 | 1.0E-9 | 8.3E1 | 5.7E1 | 694 | 37 | 281 | 37 | 0.34 |
| Nk | pg/ml | 1.9E1 | 1.6E1 | 3.4E1 | 2.3E1 | 4.0E1 | 3.2E1 | 1.0E-9 | 1.0E-9 | 2.0E2 | 1.7E2 | 694 | 37 | 281 | 37 | 0.45 |
| Nl | pg/ml | 4.9E1 | 1.6E1 | 6.5E1 | 2.7E1 | 7.2E1 | 2.9E1 | 1.0E-9 | 1.0E-9 | 1.1E3 | 1.0E2 | 694 | 37 | 281 | 37 | 0.31 |
| Tz | pg/ml | 5.2E3 | 8.8E3 | 1.3E4 | 9.3E3 | 6.4E4 | 1.2E4 | 1.0E-9 | 2.3E2 | 1.0E6 | 5.6E4 | 251 | 22 | 160 | 22 | 0.53 |
| Ua | pg/ml | 3.8E3 | 5.4E3 | 2.3E4 | 8.4E3 | 1.4E5 | 1.1E4 | 1.0E-9 | 4.3E2 | 2.1E6 | 4.3E4 | 251 | 22 | 160 | 22 | 0.53 |
| Ub | pg/ml | 5.7E2 | 5.4E2 | 8.6E2 | 8.6E2 | 1.0E3 | 1.2E3 | 1.0E-9 | 3.4E1 | 9.8E3 | 4.9E3 | 251 | 22 | 160 | 22 | 0.47 |
| Ue | pg/ml | 3.0E1 | 2.2E1 | 3.7E1 | 5.2E1 | 3.2E1 | 9.3E1 | 9.8E-2 | 5.1E0 | 3.5E2 | 4.4E2 | 251 | 22 | 160 | 22 | 0.43 |
| Uc | pg/ml | 8.6E2 | 9.6E2 | 1.6E3 | 1.7E3 | 2.7E3 | 1.8E3 | 1.0E-9 | 6.0E1 | 2.9E4 | 7.2E3 | 251 | 22 | 160 | 22 | 0.54 |
| Ud | pg/ml | 1.0E-9 | 1.0E-9 | 1.7E0 | 1.0E-9 | 2.5E1 | 0.0E0 | 1.0E-9 | 1.0E-9 | 3.9E2 | 1.0E-9 | 251 | 22 | 160 | 22 | 0.49 |
| Hq | pg/ml | 1.1E0 | 1.9E0 | 1.3E2 | 1.1E1 | 1.8E3 | 4.9E1 | 1.0E-9 | 1.0E-9 | 3.4E4 | 3.0E2 | 691 | 37 | 280 | 37 | 0.55 |
| Hr | pg/ml | 1.3E2 | 9.6E1 | 8.2E2 | 9.6E2 | 1.6E3 | 3.3E3 | 1.0E-9 | 1.0E-9 | 1.4E4 | 1.7E4 | 691 | 37 | 280 | 37 | 0.42 |
| Hu | pg/ml | 7.1E0 | 9.0E0 | 3.0E3 | 1.7E3 | 2.9E4 | 7.8E3 | 1.0E-9 | 1.0E-9 | 6.3E5 | 4.7E4 | 691 | 37 | 280 | 37 | 0.53 |
| Hv | pg/ml | 1.4E0 | 2.6E0 | 3.3E0 | 4.9E0 | 1.2E1 | 1.0E1 | 1.0E-9 | 1.0E-9 | 2.5E2 | 5.9E1 | 691 | 37 | 280 | 37 | 0.65 |
| Hw | pg/ml | 7.1E0 | 3.7E0 | 2.0E1 | 1.1E2 | 8.0E1 | 5.6E2 | 1.0E-9 | 1.0E-9 | 1.7E3 | 3.4E3 | 691 | 37 | 280 | 37 | 0.43 |
| Hx | pg/ml | 8.8E0 | 1.1E1 | 4.4E1 | 7.0E1 | 3.6E2 | 3.2E2 | 1.0E-9 | 1.0E-9 | 9.3E3 | 2.0E3 | 691 | 37 | 280 | 37 | 0.49 |
| Ib | ng/ml | 6.2E-2 | 2.9E-2 | 1.4E0 | 4.5E-1 | 5.0E0 | 1.3E0 | 1.0E-9 | 1.0E-9 | 3.6E1 | 6.1E0 | 241 | 22 | 159 | 22 | 0.42 |
| Ic | U/ml | 2.2E2 | 2.2E2 | 7.3E2 | 1.6E3 | 6.1E3 | 6.2E3 | 2.4E0 | 1.5E0 | 9.3E4 | 3.0E4 | 241 | 22 | 159 | 22 | 0.59 |
| Id | U/ml | 6.4E-1 | 1.2E0 | 1.2E0 | 2.3E0 | 2.0E0 | 4.3E0 | 1.0E-9 | 5.6E-2 | 2.3E1 | 2.1E1 | 241 | 22 | 159 | 22 | 0.65 |
| Tt | pg/ml | 1.6E2 | 1.9E2 | 1.7E2 | 1.9E2 | 5.1E1 | 4.1E1 | 4.3E1 | 1.2E2 | 3.6E2 | 2.6E2 | 231 | 22 | 153 | 22 | 0.62 |
| To | pg/ml | 1.6E0 | 1.9E0 | 1.9E0 | 2.4E0 | 2.4E0 | 1.8E0 | 1.0E-9 | 1.0E-9 | 2.3E1 | 6.3E0 | 242 | 22 | 157 | 22 | 0.62 |
| Tr | pg/ml | 2.8E0 | 4.3E0 | 5.8E0 | 5.8E0 | 2.0E1 | 4.9E0 | 1.0E-9 | 9.5E-2 | 3.1E2 | 2.1E1 | 238 | 22 | 156 | 22 | 0.61 |
| Tn | pg/ml | 2.6E1 | 3.2E1 | 8.0E1 | 4.7E1 | 2.2E2 | 4.9E1 | 2.4E0 | 1.1E1 | 1.8E3 | 2.3E2 | 242 | 22 | 157 | 22 | 0.58 |
| Tv | ng/ml | 1.2E1 | 1.6E1 | 1.9E1 | 2.0E1 | 3.7E1 | 2.1E1 | 1.0E-9 | 1.0E-9 | 4.9E2 | 7.0E1 | 242 | 22 | 157 | 22 | 0.52 |
| Ih | ng/ml | 6.9E1 | 1.8E2 | 2.1E2 | 4.3E2 | 3.7E2 | 7.1E2 | 1.0E-9 | 1.0E-9 | 2.6E3 | 2.9E3 | 694 | 37 | 280 | 37 | 0.58 |
| Ii | ng/ml | 9.0E1 | 1.3E2 | 2.6E2 | 5.3E2 | 7.4E2 | 1.7E3 | 7.3E-1 | 3.5E0 | 1.0E4 | 9.2E3 | 694 | 37 | 280 | 37 | 0.56 |
| Ij | ng/ml | 7.3E1 | 9.9E1 | 1.8E2 | 2.8E2 | 6.4E2 | 1.0E3 | 2.1E0 | 8.7E0 | 6.4E3 | 6.4E3 | 686 | 37 | 279 | 37 | 0.59 |
| Ik | ng/ml | 1.4E1 | 2.4E2 | 9.8E2 | 5.3E2 | 9.3E3 | 8.4E2 | 5.9E-1 | 1.3E0 | 1.2E5 | 4.5E3 | 690 | 37 | 279 | 37 | 0.66 |
| Il | ng/ml | 3.3E2 | 3.1E2 | 1.2E3 | 1.2E3 | 2.7E3 | 2.8E3 | 1.0E-9 | 1.0E-9 | 1.2E4 | 1.2E4 | 680 | 34 | 279 | 34 | 0.48 |
| Im | ng/ml | 1.9E2 | 3.3E2 | 3.4E2 | 6.1E2 | 5.0E2 | 1.1E3 | 1.3E1 | 2.4E1 | 6.0E3 | 6.8E3 | 689 | 37 | 279 | 37 | 0.65 |
| In | ng/ml | 3.9E0 | 3.3E0 | 2.5E1 | 1.0E1 | 1.7E2 | 1.8E1 | 1.0E-9 | 1.0E-9 | 3.9E3 | 7.5E1 | 694 | 37 | 280 | 37 | 0.45 |
| Hb | ng/ml | 2.4E1 | 3.3E1 | 3.2E1 | 4.0E1 | 2.9E1 | 3.3E1 | 4.8E-1 | 1.5E0 | 1.5E2 | 1.1E2 | 248 | 22 | 160 | 22 | 0.57 |
| Hc | pg/ml | 6.7E2 | 5.9E2 | 3.7E3 | 2.2E3 | 1.3E4 | 3.3E3 | 1.0E-9 | 2.6E2 | 1.0E5 | 1.1E4 | 248 | 22 | 160 | 22 | 0.53 |
| Hf | ng/ml | 1.5E2 | 2.7E2 | 3.8E2 | 3.2E2 | 5.3E2 | 3.4E2 | 1.0E-9 | 1.0E-9 | 2.5E3 | 1.3E3 | 248 | 22 | 160 | 22 | 0.53 |
| Io | ng/ml | 7.5E3 | 1.0E4 | 2.4E4 | 5.1E4 | 1.6E5 | 1.3E5 | 1.0E-9 | 2.2E2 | 4.0E6 | 5.5E5 | 687 | 36 | 280 | 36 | 0.57 |
| Ip | ng/ml | 8.7E0 | 1.8E1 | 1.9E1 | 2.7E1 | 2.4E1 | 3.2E1 | 1.0E-9 | 5.6E-3 | 2.6E2 | 1.4E2 | 687 | 36 | 280 | 36 | 0.55 |
| Iq | ug/ml | 9.5E-2 | 3.6E-2 | 4.0E1 | 2.3E1 | 7.3E2 | 1.3E2 | 1.0E-9 | 1.0E-9 | 1.4E4 | 7.5E2 | 687 | 36 | 280 | 36 | 0.45 |
| Ir | ug/ml | 3.3E-1 | 7.7E-1 | 3.8E0 | 4.9E0 | 2.8E1 | 1.9E1 | 1.0E-9 | 1.0E-9 | 5.1E2 | 1.1E2 | 686 | 36 | 280 | 36 | 0.64 |
| Is | ng/ml | 1.4E0 | 2.1E0 | 5.7E0 | 1.4E1 | 2.3E1 | 3.9E1 | 1.0E-9 | 1.0E-9 | 5.5E2 | 2.3E2 | 687 | 36 | 280 | 36 | 0.60 |
| It | ng/ml | 2.0E0 | 2.6E0 | 2.6E1 | 2.5E1 | 1.5E2 | 9.9E1 | 1.0E-9 | 1.0E-9 | 2.8E3 | 5.9E2 | 687 | 36 | 280 | 36 | 0.57 |
| Iu | ng/ml | 2.1E2 | 2.9E2 | 1.4E3 | 2.4E3 | 4.3E3 | 6.7E3 | 1.0E-9 | 1.0E-9 | 2.9E4 | 2.4E4 | 687 | 36 | 280 | 36 | 0.53 |
| Iv | ng/ml | 1.2E1 | 2.6E1 | 6.1E1 | 2.4E2 | 6.1E2 | 1.1E3 | 1.0E-9 | 1.0E-9 | 1.6E4 | 6.4E3 | 686 | 36 | 280 | 36 | 0.64 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Iz | ng/ml | 1.4E2 | 2.1E2 | 6.8E2 | 3.4E2 | 4.0E3 | 4.2E2 | 1.5E0 | 9.2E-1 | 6.2E4 | 1.6E3 | 248 | 22 | 160 | 22 | 0.56 |
| Rc | pg/ml | 5.5E3 | 6.1E3 | 7.3E3 | 6.3E3 | 6.0E3 | 3.8E3 | 1.9E2 | 7.5E2 | 3.9E4 | 1.6E4 | 248 | 22 | 160 | 22 | 0.49 |
| Rb | pg/ml | 7.9E-1 | 1.9E0 | 2.6E0 | 3.9E0 | 4.4E0 | 5.4E0 | 1.0E-9 | 1.0E-9 | 4.3E1 | 2.1E1 | 248 | 22 | 160 | 22 | 0.62 |
| Pz | ng/ml | 3.3E3 | 1.0E4 | 6.8E3 | 9.3E3 | 1.8E4 | 1.5E4 | 1.3E1 | 1.5E2 | 2.8E5 | 9.5E4 | 687 | 37 | 278 | 37 | 0.62 |
| Qa | ng/ml | 3.1E3 | 6.6E3 | 5.8E3 | 8.5E3 | 7.1E3 | 6.6E3 | 1.5E2 | 3.8E2 | 5.2E4 | 2.4E4 | 687 | 37 | 278 | 37 | 0.65 |
| Qb | ng/ml | 9.1E1 | 1.4E2 | 2.1E2 | 2.2E2 | 5.2E2 | 2.0E2 | 7.9E-1 | 1.0E1 | 8.3E3 | 6.0E2 | 687 | 37 | 278 | 37 | 0.60 |
| Qc | ng/ml | 2.1E2 | 3.2E2 | 4.4E2 | 4.3E2 | 7.6E2 | 4.3E2 | 1.0E-9 | 6.8E0 | 1.1E4 | 1.6E3 | 687 | 37 | 278 | 37 | 0.55 |
| Qd | ng/ml | 8.7E3 | 1.3E4 | 1.9E4 | 2.2E4 | 8.2E4 | 2.4E4 | 1.5E2 | 9.0E2 | 2.0E6 | 1.2E5 | 687 | 37 | 278 | 37 | 0.63 |
| Qe | ng/ml | 8.0E2 | 1.7E3 | 1.7E3 | 2.6E3 | 4.2E3 | 2.6E3 | 1.0E-9 | 8.2E1 | 9.7E4 | 1.4E4 | 687 | 37 | 278 | 37 | 0.69 |
| Jd | ng/ml | 9.2E-1 | 1.1E0 | 6.7E0 | 2.3E0 | 4.4E1 | 3.9E0 | 1.0E-9 | 1.0E-9 | 6.5E2 | 1.9E1 | 249 | 22 | 162 | 22 | 0.55 |
| Je | ng/ml | 1.0E-9 | 3.9E-1 | 2.3E0 | 1.2E0 | 8.0E0 | 1.5E0 | 1.0E-9 | 1.0E-9 | 8.8E1 | 4.5E0 | 249 | 22 | 162 | 22 | 0.54 |
| Jf | ng/ml | 1.0E-9 | 7.4E-1 | 1.1E0 | 1.7E0 | 2.3E0 | 2.4E0 | 1.0E-9 | 1.0E-9 | 1.2E1 | 8.7E0 | 249 | 22 | 162 | 22 | 0.64 |
| Jg | ng/ml | 4.4E2 | 1.1E3 | 7.3E2 | 1.6E3 | 9.6E2 | 1.6E3 | 1.0E-9 | 2.1E1 | 1.0E4 | 6.8E3 | 691 | 37 | 280 | 37 | 0.70 |
| Jh | ng/ml | 2.9E0 | 5.6E0 | 2.4E1 | 4.2E1 | 1.1E2 | 9.4E1 | 1.0E-9 | 1.0E-9 | 1.6E3 | 4.7E2 | 691 | 37 | 280 | 37 | 0.59 |
| Ji | ng/ml | 5.0E1 | 7.4E1 | 7.0E1 | 1.8E2 | 6.9E1 | 3.0E2 | 1.0E-9 | 1.0E1 | 5.3E2 | 1.8E3 | 691 | 37 | 280 | 37 | 0.69 |
| Sr | pg/mL | 3.4E2 | 6.7E2 | 8.1E2 | 1.1E3 | 1.3E3 | 1.1E3 | 1.0E-9 | 1.9E1 | 9.8E3 | 4.1E3 | 239 | 22 | 157 | 22 | 0.64 |
| Ss | pg/mL | 9.2E4 | 1.0E5 | 1.5E5 | 1.3E5 | 1.9E5 | 1.1E5 | 2.7E3 | 1.4E4 | 1.8E6 | 4.7E5 | 239 | 22 | 157 | 22 | 0.53 |
| St | pg/mL | 2.2E7 | 4.6E7 | 4.9E7 | 8.7E7 | 9.0E7 | 1.1E8 | 1.0E-9 | 1.5E6 | 1.2E9 | 4.2E8 | 244 | 22 | 158 | 22 | 0.64 |
| Ra | pg/ml | 1.0E-9 | 2.6E-1 | 6.4E-1 | 5.3E-1 | 1.3E0 | 9.9E-1 | 1.0E-9 | 1.0E-9 | 7.3E0 | 4.1E0 | 248 | 22 | 160 | 22 | 0.57 |
| Qz | pg/ml | 1.1E1 | 4.9E0 | 6.1E1 | 5.2E1 | 1.0E2 | 6.8E1 | 1.0E-9 | 1.0E-9 | 7.1E2 | 2.0E2 | 248 | 22 | 160 | 22 | 0.46 |
| Qy | pg/ml | 4.3E-1 | 6.2E-1 | 1.0E1 | 2.5E1 | 5.7E1 | 1.1E2 | 1.0E-9 | 1.1E-2 | 6.5E2 | 5.1E2 | 248 | 22 | 160 | 22 | 0.58 |
| Qx | pg/ml | 1.0E-9 | 1.0E-9 | 6.8E0 | 8.3E-1 | 5.2E1 | 1.9E0 | 1.0E-9 | 1.0E-9 | 5.8E2 | 7.1E0 | 248 | 22 | 160 | 22 | 0.55 |
| Qw | pg/ml | 1.0E-9 | 1.0E-9 | 2.5E0 | 2.8E-1 | 1.1E1 | 7.2E-1 | 1.0E-9 | 1.0E-9 | 1.2E2 | 2.4E0 | 248 | 22 | 160 | 22 | 0.37 |
| Qv | pg/ml | 2.4E4 | 1.9E4 | 3.4E4 | 3.0E4 | 5.7E4 | 3.4E4 | 1.0E-9 | 6.0E1 | 7.4E5 | 1.4E5 | 248 | 22 | 160 | 22 | 0.45 |
| Qu | pg/ml | 7.7E0 | 1.7E1 | 8.8E1 | 9.1E1 | 1.7E2 | 2.0E2 | 1.0E-9 | 1.0E-9 | 9.8E2 | 9.2E2 | 248 | 22 | 160 | 22 | 0.54 |
| Qt | pg/ml | 1.0E1 | 1.0E-9 | 5.3E1 | 2.6E1 | 1.3E2 | 5.6E1 | 1.0E-9 | 1.0E-9 | 1.0E3 | 1.9E2 | 248 | 22 | 160 | 22 | 0.41 |
| Qh | ng/ml | 1.7E1 | 1.7E1 | 3.6E1 | 3.0E1 | 6.1E1 | 2.9E1 | 1.0E-9 | 3.9E0 | 6.4E2 | 1.1E2 | 248 | 22 | 160 | 22 | 0.54 |
| Qg | ng/ml | 8.5E0 | 6.5E0 | 2.1E1 | 9.6E0 | 7.1E1 | 9.9E0 | 5.1E-2 | 1.4E-1 | 1.0E3 | 4.2E1 | 248 | 22 | 160 | 22 | 0.43 |
| Jj | ng/ml | 6.7E2 | 2.8E2 | 1.9E3 | 8.8E2 | 1.4E4 | 1.9E3 | 2.3E0 | 1.7E1 | 3.4E5 | 1.1E4 | 691 | 37 | 280 | 37 | 0.36 |
| Jk | ng/ml | 3.0E0 | 4.2E0 | 2.2E1 | 3.0E1 | 4.7E1 | 6.9E1 | 1.0E-9 | 1.0E-1 | 3.9E2 | 3.5E2 | 691 | 37 | 280 | 37 | 0.54 |
| Jl | ng/ml | 3.9E-1 | 9.5E-1 | 1.7E0 | 7.2E0 | 4.2E0 | 2.6E1 | 7.6E-4 | 2.9E-3 | 3.2E1 | 1.6E2 | 691 | 37 | 280 | 37 | 0.62 |
| Jm | ng/ml | 1.7E1 | 3.3E1 | 5.2E1 | 5.3E1 | 1.1E2 | 6.4E1 | 1.0E-9 | 7.7E-1 | 1.4E3 | 2.3E2 | 691 | 37 | 280 | 37 | 0.57 |
| Jn | pg/ml | 3.8E-1 | 1.0E0 | 2.4E0 | 2.3E0 | 2.4E1 | 4.8E0 | 1.0E-9 | 1.0E-9 | 6.2E2 | 2.7E1 | 691 | 37 | 280 | 37 | 0.63 |
| Jo | pg/ml | 3.6E3 | 4.7E3 | 4.9E3 | 5.3E3 | 3.9E3 | 6.2E3 | 2.0E1 | 4.9E1 | 2.4E4 | 3.8E4 | 691 | 37 | 280 | 37 | 0.51 |
| Jp | pg/ml | 6.8E4 | 1.0E5 | 7.1E4 | 9.5E4 | 3.6E4 | 3.8E4 | 5.8E2 | 3.5E3 | 3.0E5 | 1.7E5 | 691 | 37 | 280 | 37 | 0.70 |
| Jq | pg/ml | 9.5E1 | 1.1E2 | 1.5E2 | 2.5E2 | 2.2E2 | 6.0E2 | 1.0E0 | 8.1E0 | 4.0E3 | 3.7E3 | 691 | 37 | 280 | 37 | 0.52 |
| Jr | pg/ml | 5.1E0 | 6.1E0 | 3.5E1 | 2.1E1 | 4.1E2 | 4.5E1 | 1.0E-9 | 1.0E-9 | 1.1E4 | 2.0E2 | 691 | 37 | 280 | 37 | 0.59 |
| Js | pg/ml | 1.3E1 | 1.6E1 | 5.2E1 | 3.5E1 | 4.0E2 | 9.6E1 | 1.0E-9 | 1.0E-9 | 1.0E4 | 5.9E2 | 691 | 37 | 280 | 37 | 0.57 |
| Jt | pg/ml | 2.5E3 | 2.9E3 | 3.1E3 | 4.3E3 | 2.3E3 | 5.3E3 | 2.2E1 | 7.7E1 | 2.2E4 | 3.3E4 | 691 | 37 | 280 | 37 | 0.57 |
| Ju | mIU/ml | 8.5E0 | 1.1E1 | 2.0E1 | 1.5E1 | 3.2E1 | 1.3E1 | 6.5E-2 | 6.5E-1 | 2.3E2 | 4.9E1 | 249 | 22 | 162 | 22 | 0.54 |
| Jv | mIU/ml | 1.1E1 | 1.3E1 | 3.5E1 | 2.3E1 | 6.3E1 | 2.4E1 | 1.0E-2 | 1.8E-1 | 4.4E2 | 9.1E1 | 249 | 22 | 162 | 22 | 0.53 |
| Jy | ng/ml | 1.6E-3 | 1.6E-3 | 2.2E-3 | 1.8E-3 | 4.3E-3 | 1.2E-3 | 1.0E-9 | 8.7E-5 | 5.2E-2 | 6.4E-3 | 249 | 22 | 162 | 22 | 0.48 |
| Kc | pg/ml | 2.3E1 | 5.7E1 | 4.1E1 | 7.8E1 | 4.3E1 | 6.9E1 | 1.0E-9 | 1.0E-9 | 1.9E2 | 2.0E2 | 249 | 22 | 160 | 22 | 0.62 |
| Kd | pg/ml | 1.0E-9 | 1.0E-9 | 1.6E2 | 3.7E2 | 5.4E2 | 6.9E2 | 1.0E-9 | 1.0E-9 | 4.8E3 | 2.3E3 | 249 | 22 | 160 | 22 | 0.58 |
| Kc | pg/ml | 1.2E4 | 1.5E4 | 1.4E4 | 1.5E4 | 1.1E4 | 7.7E3 | 3.4E2 | 1.1E3 | 7.0E4 | 3.6E4 | 249 | 22 | 160 | 22 | 0.58 |
| Kf | pg/mL | 6.4E0 | 9.4E0 | 6.8E0 | 9.1E0 | 5.6E0 | 5.1E0 | 1.0E-9 | 1.0E-9 | 2.6E1 | 1.8E1 | 249 | 22 | 160 | 22 | 0.64 |
| Kg | pg/mL | 1.1E3 | 1.4E3 | 1.9E3 | 1.7E3 | 2.6E3 | 1.3E3 | 7.3E1 | 1.3E2 | 2.2E4 | 4.4E3 | 249 | 22 | 160 | 22 | 0.53 |
| Ki | pg/ml | 6.1E1 | 5.2E1 | 7.0E1 | 5.6E1 | 5.2E1 | 3.8E1 | 1.0E-9 | 1.0E-9 | 3.8E2 | 1.4E2 | 248 | 22 | 160 | 22 | 0.43 |
| Kj | pg/ml | 1.0E3 | 1.2E3 | 1.6E3 | 1.5E3 | 1.6E3 | 1.4E3 | 1.4E1 | 3.0E1 | 1.0E4 | 4.5E3 | 249 | 22 | 160 | 22 | 0.49 |
| Kk | pg/ml | 6.9E0 | 9.0E0 | 1.1E1 | 2.0E1 | 1.5E1 | 2.1E1 | 1.0E-9 | 1.0E-9 | 1.6E2 | 6.1E1 | 249 | 22 | 160 | 22 | 0.63 |
| Kl | pg/ml | 2.0E4 | 3.3E4 | 2.7E4 | 3.2E4 | 2.5E4 | 2.4E4 | 1.6E2 | 1.4E3 | 1.6E5 | 1.0E5 | 249 | 22 | 160 | 22 | 0.57 |
| Kn | pg/ml | 2.9E1 | 5.8E1 | 5.8E1 | 9.0E1 | 9.0E1 | 1.1E2 | 1.0E-9 | 1.0E-9 | 7.3E2 | 4.1E2 | 249 | 22 | 160 | 22 | 0.61 |
| Ko | pg/ml | 3.2E2 | 7.4E2 | 4.3E2 | 7.8E2 | 4.4E2 | 6.2E2 | 1.0E-9 | 1.0E-9 | 2.2E3 | 2.4E3 | 249 | 22 | 160 | 22 | 0.69 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Kp | pg/ml | 3.0E2 | 3.6E2 | 3.4E2 | 4.4E2 | 2.6E2 | 2.5E2 | 1.0E-9 | 6.4E1 | 1.7E3 | 9.1E2 | 249 | 22 | 160 | 22 | 0.62 |
| Kq | pg/ml | 3.0E2 | 4.7E2 | 4.8E2 | 4.9E2 | 8.7E2 | 2.3E2 | 1.6E0 | 2.5E1 | 9.8E3 | 8.7E2 | 240 | 22 | 154 | 22 | 0.65 |
| Kr | pg/ml | 3.8E-1 | 9.0E-1 | 2.2E0 | 2.6E0 | 4.2E0 | 3.3E0 | 1.0E-9 | 1.0E-9 | 3.5E1 | 9.6E0 | 240 | 22 | 154 | 22 | 0.55 |
| Ks | pg/ml | 1.4E4 | 1.5E4 | 2.0E4 | 1.9E4 | 1.8E4 | 1.7E4 | 5.1E1 | 2.2E2 | 1.1E5 | 5.0E4 | 240 | 22 | 154 | 22 | 0.51 |
| Kx | ng/ml | 1.0E-9 | 1.0E-9 | 6.6E-3 | 5.1E-3 | 1.4E-2 | 6.2E-3 | 1.0E-9 | 1.0E-9 | 1.0E-1 | 1.5E-2 | 247 | 22 | 160 | 22 | 0.51 |
| Ky | ng/ml | 8.5E-2 | 1.1E-1 | 3.5E-1 | 4.5E-1 | 7.8E-1 | 5.9E-1 | 1.0E-9 | 1.0E-9 | 5.4E0 | 2.0E0 | 247 | 22 | 160 | 22 | 0.60 |
| Kz | ng/ml | 1.0E-9 | 1.0E-9 | 3.7E-3 | 1.9E-3 | 5.9E-3 | 2.6E-3 | 1.0E-9 | 1.0E-9 | 3.6E-2 | 6.6E-3 | 247 | 22 | 160 | 22 | 0.46 |
| Ld | pg/ml | 1.0E-9 | 3.7E-1 | 3.6E0 | 2.6E0 | 9.2E0 | 3.8E0 | 1.0E-9 | 1.0E-9 | 8.4E1 | 1.2E1 | 246 | 22 | 159 | 22 | 0.53 |
| Lh | pg/ml | 1.2E4 | 2.1E4 | 2.0E4 | 4.1E4 | 2.5E4 | 7.3E4 | 1.0E-9 | 1.8E2 | 2.6E5 | 4.1E5 | 691 | 37 | 281 | 37 | 0.65 |
| Li | pg/ml | 2.8E3 | 6.6E3 | 1.5E4 | 4.0E4 | 5.9E4 | 1.0E5 | 1.0E-9 | 1.6E1 | 1.3E6 | 5.9E5 | 691 | 37 | 281 | 37 | 0.67 |
| Lj | pg/ml | 2.3E3 | 8.0E3 | 2.1E4 | 3.5E4 | 6.5E4 | 7.3E4 | 1.0E-9 | 8.9E1 | 4.7E5 | 3.5E5 | 691 | 37 | 281 | 37 | 0.65 |
| Rm | ng/ml | 1.9E1 | 3.7E1 | 4.9E1 | 4.5E1 | 7.4E1 | 5.5E1 | 2.2E-1 | 7.0E-1 | 4.0E2 | 2.5E2 | 245 | 21 | 159 | 21 | 0.54 |
| Rh | ng/ml | 1.3E2 | 1.6E2 | 3.6E2 | 3.3E2 | 1.2E3 | 5.3E2 | 3.6E0 | 1.4E1 | 1.7E4 | 2.5E3 | 245 | 21 | 159 | 21 | 0.57 |
| Ri | ng/ml | 1.0E-9 | 4.1E-1 | 4.4E0 | 2.9E0 | 1.6E1 | 4.7E0 | 1.0E-9 | 1.0E-9 | 2.2E2 | 2.0E1 | 246 | 21 | 160 | 21 | 0.54 |
| Rg | ng/ml | 1.0E-9 | 1.0E-9 | 7.1E-2 | 2.1E-3 | 4.2E-1 | 5.1E-3 | 1.0E-9 | 1.0E-9 | 4.6E0 | 2.2E-2 | 245 | 21 | 159 | 21 | 0.53 |
| Rj | ng/ml | 1.0E-9 | 1.0E-9 | 1.6E0 | 3.0E-1 | 6.0E0 | 8.1E-1 | 1.0E-9 | 1.0E-9 | 8.0E1 | 3.2E0 | 246 | 21 | 160 | 21 | 0.44 |
| Rf | ng/ml | 3.7E-1 | 6.0E-1 | 9.8E-1 | 1.6E0 | 1.9E0 | 2.2E0 | 7.8E-3 | 2.2E-2 | 1.5E1 | 7.5E0 | 245 | 21 | 159 | 21 | 0.63 |
| Ql | pg/ml | 4.5E0 | 3.1E0 | 1.4E1 | 9.9E0 | 3.1E1 | 1.4E1 | 1.0E-9 | 1.0E-9 | 2.9E2 | 5.9E1 | 249 | 22 | 162 | 22 | 0.48 |
| Qm | pg/ml | 3.9E0 | 7.1E0 | 2.0E1 | 1.7E1 | 3.9E1 | 2.0E1 | 1.0E-9 | 1.0E-9 | 3.6E2 | 5.9E1 | 249 | 22 | 162 | 22 | 0.51 |
| Qn | pg/ml | 6.1E-1 | 5.6E-1 | 6.9E0 | 6.5E0 | 2.2E1 | 2.2E1 | 1.0E-9 | 1.0E-9 | 2.2E2 | 1.0E2 | 249 | 22 | 162 | 22 | 0.41 |
| Nv | pg/ml | 3.8E3 | 9.0E3 | 1.1E4 | 1.8E4 | 4.8E4 | 2.8E4 | 1.0E-9 | 3.9E1 | 1.1E6 | 1.3E5 | 696 | 37 | 281 | 37 | 0.67 |
| Nw | pg/ml | 8.1E3 | 1.7E4 | 1.2E4 | 2.7E4 | 1.7E4 | 3.9E4 | 8.6E1 | 7.3E2 | 2.1E5 | 2.1E5 | 696 | 37 | 281 | 37 | 0.75 |
| Nx | pg/ml | 2.0E2 | 4.7E2 | 3.8E2 | 7.6E2 | 6.6E2 | 7.8E2 | 1.0E-9 | 1.0E-9 | 7.4E3 | 2.8E3 | 696 | 37 | 281 | 37 | 0.67 |
| Ny | pg/ml | 5.5E0 | 1.0E1 | 6.2E1 | 3.9E1 | 9.6E2 | 1.0E2 | 1.0E-9 | 1.0E-9 | 2.5E4 | 6.2E2 | 696 | 37 | 281 | 37 | 0.62 |
| Oa | pg/ml | 1.6E2 | 3.0E2 | 4.0E2 | 5.1E2 | 6.9E2 | 5.8E2 | 1.0E-9 | 1.0E-9 | 4.8E3 | 2.4E3 | 249 | 22 | 162 | 22 | 0.62 |
| Oe | pg/ml | 4.9E1 | 3.5E1 | 2.9E2 | 3.1E2 | 8.1E2 | 8.7E2 | 1.0E-9 | 1.0E-9 | 1.9E4 | 5.1E3 | 688 | 37 | 281 | 37 | 0.48 |
| Of | pg/ml | 1.8E2 | 2.1E2 | 6.4E3 | 5.1E3 | 3.1E4 | 1.6E4 | 1.0E-9 | 1.0E-9 | 6.2E5 | 7.4E4 | 695 | 37 | 281 | 37 | 0.52 |
| Og | pg/ml | 8.4E-2 | 9.0E-2 | 5.3E-1 | 3.9E-1 | 1.7E0 | 1.5E0 | 1.0E-9 | 1.0E-9 | 2.0E1 | 9.0E0 | 695 | 37 | 281 | 37 | 0.46 |
| Oh | pg/ml | 2.4E0 | 3.4E0 | 2.3E1 | 1.9E1 | 1.6E2 | 4.8E1 | 1.0E-9 | 1.0E-9 | 3.5E3 | 2.2E2 | 695 | 37 | 281 | 37 | 0.60 |
| Oi | pg/ml | 2.3E0 | 5.4E0 | 6.1E0 | 1.0E1 | 9.9E0 | 1.2E1 | 1.0E-9 | 1.0E-9 | 8.6E1 | 4.2E1 | 695 | 37 | 281 | 37 | 0.59 |
| Ok | pg/ml | 3.5E2 | 7.6E2 | 5.0E2 | 3.0E3 | 5.2E2 | 1.1E4 | 1.3E1 | 2.2E1 | 5.2E3 | 7.0E4 | 695 | 37 | 281 | 37 | 0.74 |
| Om | pg/ml | 3.8E2 | 6.8E2 | 8.5E2 | 1.7E3 | 2.3E3 | 3.4E3 | 1.0E-9 | 1.0E-9 | 3.6E4 | 1.7E4 | 695 | 37 | 281 | 37 | 0.61 |
| On | pg/ml | 1.6E2 | 3.8E2 | 2.7E2 | 1.1E3 | 4.1E2 | 2.8E3 | 1.0E-9 | 6.1E0 | 4.5E3 | 1.5E4 | 695 | 37 | 281 | 37 | 0.70 |
| Or | pg/ml | 1.2E1 | 1.7E1 | 3.1E1 | 6.6E1 | 6.0E1 | 1.6E2 | 1.0E-9 | 1.0E-9 | 5.0E2 | 7.6E2 | 249 | 22 | 160 | 22 | 0.56 |
| Ow | pg/ml | 3.3E1 | 4.8E1 | 1.2E2 | 1.4E2 | 3.5E2 | 2.3E2 | 1.0E-9 | 1.0E-9 | 3.2E3 | 7.9E2 | 249 | 22 | 160 | 22 | 0.57 |
| Ou | pg/ml | 4.6E2 | 7.9E2 | 8.6E2 | 2.5E3 | 1.3E3 | 3.4E3 | 1.0E-9 | 1.0E-9 | 9.8E3 | 9.6E3 | 249 | 22 | 160 | 22 | 0.61 |
| Ul | ng/ml | 1.0E-9 | 1.0E-9 | 1.2E0 | 5.2E0 | 4.6E0 | 2.2E1 | 1.0E-9 | 1.0E-9 | 3.4E1 | 1.0E2 | 257 | 22 | 164 | 22 | 0.52 |
| Uo | ng/ml | 1.0E-9 | 1.0E-9 | 9.2E-2 | 1.6E-1 | 2.1E-1 | 3.5E-1 | 1.0E-9 | 1.0E-9 | 1.3E0 | 1.5E0 | 257 | 22 | 164 | 22 | 0.51 |
| Uk | ng/ml | 1.0E-9 | 1.0E-9 | 7.8E-3 | 5.4E-3 | 2.7E-2 | 1.2E-2 | 1.0E-9 | 1.0E-9 | 3.2E-1 | 4.2E-2 | 257 | 22 | 164 | 22 | 0.43 |
| Um | ng/ml | 1.0E-9 | 1.0E-9 | 3.7E-1 | 3.5E-1 | 8.5E-1 | 9.7E-1 | 1.0E-9 | 1.0E-9 | 6.8E0 | 4.4E0 | 257 | 22 | 164 | 22 | 0.45 |
| Uf | ng/ml | 5.1E-2 | 1.0E-1 | 1.2E-1 | 2.3E-1 | 1.9E-1 | 5.1E-1 | 1.0E-3 | 1.3E-3 | 1.7E0 | 2.5E0 | 257 | 22 | 164 | 22 | 0.61 |
| Uh | ng/ml | 1.8E0 | 3.2E0 | 2.9E0 | 3.8E0 | 3.2E0 | 3.0E0 | 3.2E-2 | 2.3E-1 | 1.7E1 | 1.2E1 | 257 | 22 | 164 | 22 | 0.63 |
| Un | ng/ml | 1.8E0 | 2.1E0 | 2.1E0 | 2.3E0 | 1.3E0 | 1.3E0 | 2.0E-1 | 1.8E-1 | 8.0E0 | 5.3E0 | 257 | 22 | 164 | 22 | 0.56 |
| Ug | ng/ml | 1.4E1 | 2.2E1 | 2.7E1 | 3.7E1 | 2.8E1 | 4.7E1 | 6.9E-1 | 1.1E0 | 1.8E2 | 2.1E2 | 257 | 22 | 164 | 22 | 0.54 |
| Ur | ng/ml | 1.5E-1 | 2.0E-1 | 7.7E-1 | 8.6E-1 | 5.9E0 | 1.9E0 | 1.0E-9 | 1.0E-9 | 9.3E1 | 7.2E0 | 256 | 22 | 163 | 22 | 0.50 |
| Up | ng/ml | 1.0E-9 | 1.0E-9 | 5.4E-3 | 2.4E-3 | 2.5E-2 | 6.6E-3 | 1.0E-9 | 1.0E-9 | 3.3E-1 | 2.5E-2 | 256 | 22 | 163 | 22 | 0.47 |
| Us | ng/ml | 3.2E-3 | 5.1E-3 | 1.8E-2 | 1.9E-2 | 4.4E-2 | 4.5E-2 | 1.0E-9 | 1.0E-9 | 4.0E-1 | 2.1E-1 | 256 | 22 | 163 | 22 | 0.51 |
| Uv | ng/ml | 3.0E-3 | 2.7E-3 | 1.3E-2 | 1.0E-2 | 4.2E-2 | 3.0E-2 | 1.0E-9 | 1.0E-9 | 4.3E-1 | 1.4E-1 | 256 | 22 | 163 | 22 | 0.48 |
| Ut | ng/ml | 6.6E-1 | 9.1E-1 | 2.9E0 | 1.2E0 | 9.1E0 | 1.3E0 | 1.0E-9 | 1.0E-9 | 7.8E1 | 5.5E0 | 256 | 22 | 163 | 22 | 0.50 |
| Uu | ng/ml | 7.0E0 | 7.8E0 | 7.7E0 | 8.9E0 | 5.0E0 | 6.3E0 | 4.5E-1 | 5.5E-1 | 2.6E1 | 2.2E1 | 256 | 22 | 163 | 22 | 0.54 |
| Vp | ng/ml | 1.0E-9 | 1.0E-9 | 4.0E-1 | 4.9E-3 | 3.6E0 | 2.3E-2 | 1.0E-9 | 1.0E-9 | 5.0E1 | 1.1E-1 | 257 | 22 | 164 | 22 | 0.45 |
| Vt | ng/ml | 6.0E0 | 7.9E0 | 8.4E0 | 1.1E1 | 9.1E0 | 8.8E0 | 4.3E-1 | 9.2E-1 | 8.6E1 | 3.4E1 | 257 | 22 | 164 | 22 | 0.62 |
| Vu | ng/ml | 1.0E-9 | 1.0E-9 | 2.3E0 | 8.2E-1 | 5.6E0 | 1.7E0 | 1.0E-9 | 1.0E-9 | 5.1E1 | 6.2E0 | 253 | 21 | 164 | 21 | 0.45 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|-----|--------|-----|-----|
| | | NonDis | Dis | NonDis | Dis | NonDis | Dis | NonDis | Dis | NonDis | Dis | NonDis | Dis | NonDis | Dis | |
| Vq | ng/ml | 1.6E2 | 4.7E2 | 4.1E3 | 1.2E3 | 4.8E4 | 1.6E3 | 2.0E-1 | 7.9E0 | 6.8E5 | 5.4E3 | 203 | 16 | 135 | 16 | 0.61 |
| Vo | ng/ml | 2.6E1 | 2.5E1 | 2.4E1 | 2.3E1 | 5.4E0 | 6.1E0 | 2.4E0 | 6.7E0 | 4.8E1 | 3.1E1 | 257 | 22 | 164 | 22 | 0.44 |
| Vs | ng/ml | 1.0E-9 | 1.0E-9 | 6.7E0 | 2.5E0 | 2.4E1 | 5.2E0 | 1.0E-9 | 1.0E-9 | 2.3E2 | 1.9E1 | 253 | 21 | 161 | 21 | 0.42 |
| Vv | ng/ml | 2.9E0 | 3.4E0 | 6.1E0 | 9.1E0 | 1.0E1 | 1.7E1 | 1.0E-9 | 1.0E-9 | 8.4E1 | 8.0E1 | 256 | 22 | 164 | 22 | 0.56 |
| Oy | pg/ml | 5.3E-1 | 5.8E-1 | 6.6E0 | 8.7E0 | 3.2E1 | 4.4E1 | 1.0E-9 | 1.0E-9 | 4.0E2 | 2.7E2 | 694 | 37 | 280 | 37 | 0.52 |
| Oz | pg/ml | 1.3E-2 | 2.4E-1 | 3.4E-1 | 3.2E-1 | 1.5E0 | 3.2E-1 | 1.0E-9 | 1.0E-9 | 2.9E1 | 1.0E0 | 694 | 37 | 280 | 37 | 0.61 |
| Pa | pg/ml | 3.8E-1 | 4.5E-1 | 1.5E0 | 9.4E0 | 5.5E0 | 4.8E1 | 1.0E-9 | 2.6E-2 | 8.6E1 | 2.9E2 | 694 | 37 | 280 | 37 | 0.59 |
| Pb | pg/ml | 1.0E-9 | 8.2E-2 | 9.3E-1 | 5.0E0 | 1.9E1 | 3.0E1 | 1.0E-9 | 1.0E-9 | 4.9E2 | 1.8E2 | 694 | 37 | 280 | 37 | 0.59 |
| Pc | pg/ml | 4.7E-2 | 3.2E-1 | 3.7E-1 | 8.6E-1 | 9.4E-1 | 2.1E0 | 1.0E-9 | 1.0E-9 | 1.6E1 | 1.1E1 | 694 | 37 | 280 | 37 | 0.59 |
| Pd | pg/ml | 1.7E0 | 2.8E0 | 5.1E0 | 4.8E0 | 3.3E1 | 5.2E0 | 1.0E-9 | 1.0E-9 | 8.4E2 | 1.9E1 | 694 | 37 | 280 | 37 | 0.60 |
| Pe | pg/ml | 2.0E1 | 3.6E1 | 1.0E2 | 5.0E2 | 3.5E2 | 2.3E3 | 1.0E-9 | 3.7E-1 | 4.7E3 | 1.4E4 | 694 | 37 | 280 | 37 | 0.65 |
| Pf | pg/ml | 1.4E0 | 2.4E0 | 1.1E1 | 1.6E1 | 6.4E1 | 4.1E1 | 1.0E-9 | 1.0E-9 | 1.5E3 | 2.3E2 | 694 | 37 | 280 | 37 | 0.60 |
| Pg | pg/ml | 3.0E0 | 6.4E0 | 4.7E1 | 7.2E1 | 3.8E2 | 3.1E2 | 1.0E-9 | 1.3E-1 | 7.7E3 | 1.9E3 | 694 | 37 | 280 | 37 | 0.63 |
| Ph | ng/ml | 1.7E-1 | 2.3E-1 | 3.2E-1 | 7.2E-1 | 4.5E-1 | 1.1E0 | 1.0E-9 | 1.0E-9 | 2.9E0 | 4.4E0 | 249 | 22 | 160 | 22 | 0.59 |
| Pi | ng/ml | 2.0E-1 | 2.4E-1 | 2.8E-1 | 2.6E-1 | 3.6E-1 | 2.3E-1 | 1.0E-9 | 1.0E-9 | 3.2E0 | 1.0E0 | 249 | 22 | 160 | 22 | 0.53 |
| Pj | ng/mL | 4.8E0 | 7.6E0 | 5.9E0 | 8.3E0 | 4.5E0 | 5.1E0 | 3.8E-2 | 1.6E-1 | 3.1E1 | 1.7E1 | 249 | 22 | 160 | 22 | 0.65 |
| Pk | ng/mL | 8.9E-3 | 9.3E-3 | 1.4E-2 | 1.2E-2 | 2.2E-2 | 1.1E-2 | 1.0E-9 | 1.0E-9 | 2.5E-1 | 3.8E-2 | 249 | 22 | 160 | 22 | 0.50 |
| aA | mg/dL | 8.0E-1 | 1.0E0 | 9.2E-1 | 1.4E0 | 4.7E-1 | 1.0E0 | 2.0E-1 | 4.0E-1 | 4.2E0 | 5.4E0 | 2240 | 57 | 436 | 57 | 0.66 |
| aC | mg/mL | 2.9E0 | 2.5E0 | 3.2E0 | 2.8E0 | 1.4E0 | 1.2E0 | 8.5E-1 | 1.3E0 | 8.9E0 | 6.1E0 | 437 | 26 | 174 | 26 | 0.44 |
| aD | ug/mL | 3.1E0 | 4.8E0 | 4.4E0 | 5.2E0 | 3.9E0 | 3.9E0 | 4.3E-1 | 8.4E-1 | 3.5E1 | 1.7E1 | 437 | 26 | 174 | 26 | 0.55 |
| aE | mg/mL | 5.6E-1 | 5.7E-1 | 5.8E-1 | 5.8E-1 | 1.5E-1 | 1.7E-1 | 2.1E-1 | 2.8E-1 | 1.1E0 | 1.2E0 | 437 | 26 | 174 | 26 | 0.51 |
| aF | ng/mL | 2.1E0 | 2.4E0 | 4.1E0 | 4.0E0 | 6.1E0 | 4.4E0 | 4.3E-3 | 4.3E-3 | 5.0E1 | 1.8E1 | 437 | 26 | 174 | 26 | 0.53 |
| aG | mg/mL | 1.4E-1 | 1.4E-1 | 1.6E-1 | 1.6E-1 | 9.1E-2 | 1.0E-1 | 1.7E-2 | 5.9E-2 | 5.4E-1 | 5.2E-1 | 437 | 26 | 174 | 26 | 0.48 |
| aH | ug/mL | 7.5E1 | 7.8E1 | 8.3E1 | 8.9E1 | 4.4E1 | 5.0E1 | 4.6E0 | 2.5E1 | 2.9E2 | 2.3E2 | 437 | 26 | 174 | 26 | 0.53 |
| aI | ug/mL | 1.9E2 | 1.8E2 | 1.9E2 | 1.9E2 | 6.0E1 | 4.8E1 | 2.8E1 | 9.9E1 | 3.7E2 | 2.7E2 | 437 | 26 | 174 | 26 | 0.48 |
| aJ | ug/mL | 2.4E0 | 3.8E0 | 2.9E0 | 4.8E0 | 2.1E0 | 3.9E0 | 7.6E-1 | 7.8E-1 | 1.7E1 | 1.7E1 | 437 | 26 | 174 | 26 | 0.69 |
| aK | ng/mL | 1.6E0 | 1.0E0 | 2.5E0 | 2.2E0 | 2.7E0 | 2.6E0 | 2.9E-4 | 1.1E-1 | 1.8E1 | 1.1E1 | 437 | 26 | 174 | 26 | 0.44 |
| aL | mg/mL | 8.1E-1 | 8.2E-1 | 8.2E-1 | 8.2E-1 | 2.5E-1 | 2.5E-1 | 1.9E-1 | 4.0E-1 | 1.7E0 | 1.6E0 | 437 | 26 | 174 | 26 | 0.49 |
| aM | U/mL | 2.2E1 | 2.7E1 | 4.5E1 | 4.4E1 | 9.6E1 | 4.2E1 | 4.2E-2 | 4.2E-2 | 1.6E3 | 1.5E2 | 437 | 26 | 174 | 26 | 0.56 |
| aN | U/mL | 1.3E1 | 2.2E1 | 2.0E1 | 2.8E1 | 2.8E1 | 2.5E1 | 2.5E-3 | 2.5E-3 | 3.8E2 | 9.7E1 | 437 | 26 | 174 | 26 | 0.63 |
| aO | pg/mL | 3.1E1 | 5.7E1 | 3.2E2 | 4.4E2 | 8.3E2 | 8.6E2 | 6.0E-2 | 6.0E-2 | 6.6E3 | 3.5E3 | 437 | 26 | 174 | 26 | 0.59 |
| aP | ng/mL | 1.6E0 | 2.0E0 | 2.0E0 | 2.8E0 | 1.8E0 | 2.2E0 | 4.5E-1 | 7.2E-1 | 2.8E1 | 1.1E1 | 437 | 26 | 174 | 26 | 0.62 |
| aQ | ng/mL | 3.0E-1 | 2.6E-1 | 4.7E-1 | 4.5E-1 | 4.8E-1 | 4.9E-1 | 2.0E-4 | 3.9E-2 | 4.0E0 | 1.8E0 | 437 | 26 | 174 | 26 | 0.45 |
| aR | ng/mL | 1.7E0 | 2.1E0 | 2.7E0 | 2.7E0 | 3.3E0 | 2.8E0 | 1.8E-1 | 3.2E-1 | 3.4E1 | 1.4E1 | 437 | 26 | 174 | 26 | 0.53 |
| aS | ng/mL | 2.6E-1 | 3.3E-1 | 6.5E-1 | 6.0E-1 | 1.9E0 | 1.1E0 | 4.2E-3 | 4.2E-3 | 3.3E1 | 5.6E0 | 437 | 26 | 174 | 26 | 0.52 |
| aU | pg/mL | 7.8E1 | 8.0E1 | 1.3E2 | 1.5E2 | 1.5E2 | 1.7E2 | 7.4E-2 | 1.3E1 | 1.3E3 | 6.0E2 | 437 | 26 | 174 | 26 | 0.50 |
| aV | ng/mL | 6.3E-1 | 3.6E-1 | 1.1E0 | 1.0E0 | 2.0E0 | 1.2E0 | 7.6E-4 | 3.1E-2 | 3.3E1 | 4.2E0 | 437 | 26 | 174 | 26 | 0.43 |
| aW | pg/mL | 1.9E1 | 1.7E1 | 2.0E1 | 3.6E1 | 2.0E1 | 8.4E1 | 7.2E-2 | 7.2E-2 | 2.4E2 | 4.5E2 | 437 | 26 | 174 | 26 | 0.54 |
| aX | ng/mL | 9.4E0 | 1.6E1 | 1.5E1 | 2.8E1 | 1.9E1 | 3.4E1 | 3.0E-1 | 2.5E0 | 2.2E2 | 1.4E2 | 437 | 26 | 174 | 26 | 0.62 |
| aY | pg/mL | 5.4E1 | 6.7E1 | 7.7E1 | 8.1E1 | 8.7E1 | 6.0E1 | 4.1E-1 | 4.1E-1 | 1.2E3 | 2.7E2 | 437 | 26 | 174 | 26 | 0.58 |
| aZ | pg/mL | 2.2E2 | 3.7E2 | 5.0E2 | 6.2E2 | 9.8E2 | 8.2E2 | 1.7E0 | 1.7E0 | 1.2E4 | 3.7E3 | 437 | 26 | 174 | 26 | 0.58 |
| bA | ng/mL | 8.0E0 | 2.8E1 | 3.0E1 | 8.9E1 | 7.8E1 | 1.9E2 | 3.0E-2 | 1.4E0 | 9.4E2 | 8.1E2 | 437 | 26 | 174 | 26 | 0.71 |
| bB | ng/mL | 3.1E2 | 2.8E2 | 3.4E2 | 2.9E2 | 1.7E2 | 1.5E2 | 2.1E0 | 5.1E1 | 1.0E3 | 5.5E2 | 437 | 26 | 174 | 26 | 0.43 |
| bC | ng/mL | 3.3E2 | 4.2E2 | 5.6E2 | 8.4E2 | 7.4E2 | 1.1E3 | 9.8E0 | 7.3E1 | 4.7E3 | 4.7E3 | 437 | 26 | 174 | 26 | 0.62 |
| bE | mg/mL | 5.5E0 | 6.1E0 | 5.8E0 | 6.5E0 | 2.0E0 | 2.5E0 | 9.8E-1 | 2.8E0 | 1.3E1 | 1.3E1 | 437 | 26 | 174 | 26 | 0.55 |
| bF | pg/mL | 1.9E1 | 3.2E1 | 1.6E2 | 5.6E1 | 9.8E2 | 6.7E1 | 5.0E-2 | 8.1E0 | 1.1E4 | 3.2E2 | 437 | 26 | 174 | 26 | 0.63 |
| bG | ng/mL | 1.6E0 | 2.0E0 | 2.7E0 | 3.3E0 | 3.3E0 | 3.4E0 | 2.2E-2 | 2.4E-1 | 2.6E1 | 1.3E1 | 437 | 26 | 174 | 26 | 0.56 |
| bH | pg/mL | 5.7E-1 | 5.7E-1 | 4.9E0 | 3.5E0 | 1.6E1 | 5.5E0 | 5.7E-1 | 5.7E-1 | 2.8E2 | 2.1E1 | 437 | 26 | 174 | 26 | 0.48 |
| bI | ng/mL | 4.0E-3 | 4.0E-3 | 6.5E-2 | 6.7E-2 | 1.6E-1 | 1.1E-1 | 4.0E-3 | 4.0E-3 | 1.3E0 | 3.4E-1 | 437 | 26 | 174 | 26 | 0.54 |
| bJ | mg/mL | 2.4E0 | 2.5E0 | 2.7E0 | 2.6E0 | 2.1E0 | 1.7E0 | 2.5E-4 | 2.5E-1 | 1.3E1 | 7.1E0 | 437 | 26 | 174 | 26 | 0.51 |
| bL | pg/mL | 4.0E0 | 2.6E0 | 8.3E0 | 8.1E0 | 1.1E1 | 1.1E1 | 4.6E-2 | 4.6E-2 | 8.0E1 | 4.4E1 | 437 | 26 | 174 | 26 | 0.45 |
| bM | mg/mL | 1.7E0 | 2.2E0 | 2.0E0 | 2.3E0 | 1.4E0 | 1.2E0 | 9.2E-3 | 6.3E-1 | 8.8E0 | 6.1E0 | 437 | 26 | 174 | 26 | 0.61 |
| bN | ng/mL | 4.3E1 | 2.9E1 | 1.4E2 | 6.9E1 | 2.9E2 | 1.2E2 | 1.4E-1 | 1.4E-1 | 1.9E3 | 5.0E2 | 437 | 26 | 174 | 26 | 0.43 |

| Code | Units | Median | | Average | | Standard Deviation | | Minimum | | Maximum | | Number of Samples | | Number of Patients | | AUC |
|------|-------|--------|-----|---------|-----|----|-----|--------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| bO | ng/mL | 4.0E-2 | 4.0E-2 | 1.0E1 | 1.4E1 | 2.3E1 | 3.9E1 | 4.0E-2 | 4.0E-2 | 1.9E2 | 1.9E2 | 437 | 26 | 174 | 26 | 0.46 |
| bP | mg/mL | 5.3E-1 | 6.1E-1 | 7.6E-1 | 7.4E-1 | 6.8E-1 | 6.0E-1 | 4.9E-2 | 1.4E-1 | 4.8E0 | 2.9E0 | 437 | 26 | 174 | 26 | 0.51 |
| bQ | pg/mL | 1.5E1 | 1.9E1 | 6.4E1 | 2.7E1 | 6.5E2 | 3.0E1 | 1.5E-1 | 1.5E-1 | 1.3E4 | 1.5E2 | 437 | 26 | 174 | 26 | 0.56 |
| bR | ng/mL | 1.2E-2 | 1.2E-2 | 1.4E-1 | 6.5E-2 | 4.6E-1 | 1.0E-1 | 1.2E-2 | 1.2E-2 | 8.7E0 | 3.9E-1 | 437 | 26 | 174 | 26 | 0.41 |
| bS | pg/mL | 9.4E-1 | 9.4E-1 | 7.2E0 | 2.5E0 | 2.9E1 | 5.5E0 | 9.4E-1 | 9.4E-1 | 3.9E2 | 2.1E1 | 437 | 26 | 174 | 26 | 0.47 |
| bU | ng/mL | 1.5E-1 | 3.4E-2 | 2.0E-1 | 1.0E-1 | 3.8E-1 | 1.4E-1 | 1.3E-2 | 1.3E-2 | 6.6E0 | 5.5E-1 | 437 | 26 | 174 | 26 | 0.38 |
| bV | pg/mL | 4.7E2 | 6.2E2 | 5.5E2 | 1.3E3 | 5.8E2 | 3.2E3 | 1.6E2 | 1.5E2 | 1.2E4 | 1.7E4 | 437 | 26 | 174 | 26 | 0.62 |
| bW | pg/mL | 3.2E2 | 3.3E2 | 4.9E2 | 6.4E2 | 4.8E2 | 1.2E3 | 8.4E1 | 1.4E2 | 4.8E3 | 6.4E3 | 437 | 26 | 174 | 26 | 0.50 |
| bX | ng/mL | 1.5E-3 | 2.5E-5 | 2.8E-3 | 1.9E-3 | 3.5E-3 | 2.6E-3 | 2.5E-5 | 2.5E-5 | 2.0E-2 | 8.8E-3 | 437 | 26 | 174 | 26 | 0.43 |
| bZ | pg/mL | 2.3E2 | 3.2E2 | 9.1E2 | 1.0E3 | 4.1E3 | 1.9E3 | 1.5E-1 | 5.9E1 | 5.8E4 | 8.2E3 | 437 | 26 | 174 | 26 | 0.60 |
| cA | pg/mL | 6.0E-1 | 6.0E-1 | 2.8E0 | 1.8E0 | 1.8E1 | 3.3E0 | 6.0E-1 | 6.0E-1 | 3.7E2 | 1.3E1 | 437 | 26 | 174 | 26 | 0.49 |
| cB | ng/mL | 6.0E-2 | 3.5E-2 | 9.3E-2 | 6.3E-2 | 1.0E-1 | 1.1E-1 | 1.7E-3 | 1.7E-3 | 5.7E-1 | 5.5E-1 | 437 | 26 | 174 | 26 | 0.39 |
| cC | pg/mL | 4.6E1 | 3.7E1 | 4.8E1 | 4.0E1 | 4.0E1 | 2.6E1 | 1.0E0 | 1.0E0 | 4.5E2 | 1.1E2 | 437 | 26 | 174 | 26 | 0.43 |
| cD | pg/mL | 5.4E0 | 4.7E0 | 1.5E1 | 1.2E1 | 5.7E1 | 1.9E1 | 3.3E-1 | 3.3E-1 | 7.2E2 | 8.4E1 | 437 | 26 | 174 | 26 | 0.47 |
| cE | pg/mL | 3.2E1 | 5.6E1 | 1.5E2 | 8.7E1 | 4.6E2 | 1.1E2 | 1.2E-1 | 1.2E-1 | 3.8E3 | 4.2E2 | 437 | 26 | 174 | 26 | 0.58 |
| cF | pg/mL | 1.3E1 | 5.3E-1 | 2.1E1 | 1.1E1 | 3.2E1 | 1.8E1 | 5.3E-1 | 5.3E-1 | 2.7E2 | 7.2E1 | 437 | 26 | 174 | 26 | 0.40 |
| cG | pg/mL | 4.3E1 | 7.2E1 | 1.1E2 | 8.4E1 | 5.2E2 | 5.7E1 | 7.8E0 | 7.7E0 | 1.0E4 | 2.4E2 | 437 | 26 | 174 | 26 | 0.65 |
| cH | uIU/mL | 3.1E0 | 1.5E0 | 6.5E0 | 3.8E0 | 1.2E1 | 6.2E0 | 8.6E-3 | 8.6E-3 | 1.6E2 | 2.4E1 | 437 | 26 | 174 | 26 | 0.33 |
| cI | ng/mL | 5.7E0 | 5.3E0 | 1.1E1 | 1.4E1 | 1.5E1 | 2.4E1 | 1.0E-3 | 1.1E-1 | 1.0E2 | 1.2E2 | 437 | 26 | 174 | 26 | 0.50 |
| cJ | ug/mL | 7.0E1 | 4.6E1 | 1.2E2 | 7.0E1 | 1.5E2 | 8.0E1 | 4.0E0 | 7.6E0 | 9.6E2 | 3.9E2 | 437 | 26 | 174 | 26 | 0.40 |
| cK | ng/mL | 3.8E-3 | 3.8E-3 | 5.5E-2 | 3.6E-2 | 1.9E-1 | 8.0E-2 | 3.8E-3 | 3.8E-3 | 1.7E0 | 3.0E-1 | 437 | 26 | 174 | 26 | 0.50 |
| cL | pg/mL | 1.9E2 | 2.7E2 | 3.6E2 | 2.7E2 | 1.3E3 | 1.5E2 | 1.6E1 | 4.1E1 | 2.4E4 | 6.4E2 | 437 | 26 | 174 | 26 | 0.62 |
| cM | pg/mL | 2.8E2 | 2.5E2 | 3.1E2 | 2.5E2 | 2.0E2 | 9.6E1 | 8.7E0 | 6.7E1 | 1.6E3 | 4.7E2 | 437 | 26 | 174 | 26 | 0.42 |
| cN | pg/mL | 1.2E2 | 1.3E2 | 1.3E2 | 1.4E2 | 6.4E1 | 5.0E1 | 3.8E1 | 6.4E1 | 1.1E3 | 2.4E2 | 437 | 26 | 174 | 26 | 0.59 |
| cO | pg/mL | 2.2E2 | 2.5E2 | 3.1E2 | 2.9E2 | 9.3E2 | 1.6E2 | 5.4E1 | 1.1E2 | 1.9E4 | 7.5E2 | 437 | 26 | 174 | 26 | 0.56 |
| cP | ng/mL | 2.5E3 | 2.8E3 | 2.6E3 | 3.0E3 | 9.2E2 | 1.1E3 | 6.2E2 | 1.6E3 | 5.7E3 | 5.9E3 | 437 | 26 | 174 | 26 | 0.59 |
| cQ | ng/mL | 4.3E-2 | 6.3E-2 | 1.3E-1 | 1.9E-1 | 2.4E-1 | 3.1E-1 | 2.0E-3 | 2.0E-3 | 2.2E0 | 1.2E0 | 437 | 26 | 174 | 26 | 0.57 |
| cR | ng/mL | 2.8E2 | 3.8E2 | 4.9E2 | 8.0E2 | 8.0E2 | 1.5E3 | 2.0E1 | 4.0E1 | 8.9E3 | 7.7E3 | 437 | 26 | 174 | 26 | 0.58 |
| cS | ng/mL | 2.6E2 | 3.4E2 | 3.8E2 | 1.3E3 | 3.8E2 | 4.3E3 | 4.1E1 | 9.7E1 | 2.7E3 | 2.2E4 | 437 | 26 | 174 | 26 | 0.58 |
| cT | ng/mL | 2.9E1 | 6.0E1 | 8.3E1 | 1.7E2 | 1.8E2 | 3.8E2 | 3.6E0 | 4.2E0 | 2.1E3 | 1.9E3 | 437 | 26 | 174 | 26 | 0.65 |
| cU | ng/mL | 5.4E1 | 7.0E1 | 7.5E1 | 1.1E2 | 9.9E1 | 1.0E2 | 6.2E0 | 1.2E1 | 1.6E3 | 4.8E2 | 437 | 26 | 174 | 26 | 0.59 |
| cV | ng/mL | 1.7E-1 | 1.7E-1 | 3.8E-1 | 4.7E-1 | 2.3E0 | 1.1E0 | 3.4E-4 | 4.7E-2 | 4.7E1 | 6.0E0 | 437 | 26 | 174 | 26 | 0.54 |
| cW | mIU/mL | 5.3E-2 | 5.6E-2 | 1.5E-1 | 7.7E-2 | 7.2E-1 | 6.5E-2 | 3.7E-4 | 1.1E-2 | 9.7E0 | 2.6E-1 | 437 | 26 | 174 | 26 | 0.52 |
| cX | ng/mL | 9.9E-2 | 1.4E-1 | 1.3E0 | 5.5E-1 | 4.2E0 | 8.8E-1 | 2.3E-4 | 2.3E-4 | 2.8E1 | 3.0E0 | 437 | 26 | 174 | 26 | 0.49 |
| cY | ng/mL | 8.9E0 | 7.4E0 | 1.3E1 | 1.4E1 | 1.3E1 | 1.5E1 | 1.5E-1 | 3.7E-1 | 8.3E1 | 5.4E1 | 437 | 26 | 174 | 26 | 0.48 |
| cZ | ug/mL | 1.5E1 | 1.6E1 | 1.6E1 | 1.6E1 | 7.1E0 | 6.3E0 | 2.3E0 | 6.3E0 | 5.7E1 | 3.6E1 | 437 | 26 | 174 | 26 | 0.52 |
| dA | pg/mL | 3.3E2 | 3.7E2 | 3.8E2 | 4.2E2 | 3.1E2 | 2.1E2 | 9.0E1 | 1.7E2 | 5.8E3 | 8.6E2 | 437 | 26 | 174 | 26 | 0.58 |
| dB | ug/mL | 1.7E1 | 1.8E1 | 1.7E1 | 1.8E1 | 1.6E1 | 9.2E0 | 9.4E-1 | 1.9E0 | 2.5E2 | 4.1E1 | 437 | 26 | 174 | 26 | 0.55 |
| dC | nmol/L | 3.5E1 | 3.6E1 | 3.9E1 | 3.9E1 | 1.8E1 | 2.0E1 | 7.9E0 | 1.3E1 | 1.4E2 | 8.3E1 | 437 | 26 | 174 | 26 | 0.48 |
| dD | ug/mL | 3.7E1 | 3.4E1 | 3.8E1 | 3.6E1 | 1.1E1 | 1.0E1 | 1.3E1 | 1.5E1 | 7.6E1 | 5.8E1 | 437 | 26 | 174 | 26 | 0.44 |
| dE | ng/mL | 4.7E-1 | 6.6E-1 | 6.1E-1 | 8.4E-1 | 7.3E-1 | 7.8E-1 | 8.4E-3 | 8.4E-3 | 7.2E0 | 3.3E0 | 437 | 26 | 174 | 26 | 0.60 |
| dF | ng/mL | 2.2E2 | 3.1E2 | 2.6E2 | 3.6E2 | 1.8E2 | 2.1E2 | 7.5E1 | 8.3E1 | 1.3E3 | 9.7E2 | 437 | 26 | 174 | 26 | 0.67 |
| dG | ng/mL | 1.1E1 | 1.6E1 | 1.4E1 | 1.9E1 | 1.2E1 | 1.2E1 | 2.5E0 | 2.2E0 | 1.8E2 | 6.9E1 | 437 | 26 | 174 | 26 | 0.70 |
| dH | pg/mL | 7.5E0 | 8.6E0 | 1.3E1 | 1.1E1 | 4.0E1 | 8.1E0 | 4.0E-2 | 4.0E-2 | 6.7E2 | 3.5E1 | 437 | 26 | 174 | 26 | 0.58 |
| dI | pg/mL | 4.6E-1 | 4.6E-1 | 2.3E0 | 2.7E0 | 1.6E1 | 8.0E0 | 4.6E-1 | 4.6E-1 | 3.3E2 | 4.1E1 | 437 | 26 | 174 | 26 | 0.48 |
| dJ | ng/mL | 1.9E0 | 1.8E0 | 2.2E0 | 2.0E0 | 1.2E0 | 1.2E0 | 3.2E-2 | 4.0E-1 | 6.9E0 | 4.4E0 | 437 | 26 | 174 | 26 | 0.44 |
| dK | uIU/mL | 1.9E0 | 1.7E0 | 3.1E0 | 4.2E0 | 6.0E0 | 1.1E1 | 2.8E-4 | 3.8E-2 | 7.9E1 | 6.0E1 | 437 | 26 | 174 | 26 | 0.48 |
| dL | ng/mL | 8.7E2 | 9.9E2 | 1.0E3 | 1.1E3 | 4.9E2 | 3.8E2 | 3.4E2 | 3.3E2 | 3.4E3 | 1.8E3 | 437 | 26 | 174 | 26 | 0.61 |
| dM | pg/mL | 9.6E2 | 1.1E3 | 1.1E3 | 1.8E3 | 8.8E2 | 1.8E3 | 3.5E2 | 3.7E2 | 1.2E4 | 8.3E3 | 437 | 26 | 174 | 26 | 0.65 |
| dN | ug/mL | 9.3E1 | 9.6E1 | 9.9E1 | 1.1E2 | 3.6E1 | 4.2E1 | 2.5E1 | 3.7E1 | 2.8E2 | 2.0E2 | 437 | 26 | 174 | 26 | 0.56 |
| dO | ng/ml | 2.1E1 | 9.3E0 | 4.2E1 | 2.5E1 | 5.9E1 | 3.7E1 | 4.0E-1 | 3.8E0 | 3.7E2 | 1.1E2 | 83 | 8 | 60 | 8 | 0.34 |
| dR | pg/ml | 1.6E3 | 1.3E3 | 2.4E3 | 2.3E3 | 2.4E3 | 2.4E3 | 1.4E2 | 1.9E2 | 1.5E4 | 8.9E3 | 287 | 25 | 167 | 25 | 0.45 |
| dV | pg/ml | 7.0E1 | 6.0E1 | 9.0E1 | 7.2E1 | 5.9E1 | 4.8E1 | 2.1E1 | 3.6E1 | 3.3E2 | 1.9E2 | 61 | 8 | 38 | 8 | 0.35 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dX | ng/ml | 8.1E-2 | 7.4E-3 | 1.2E-1 | 1.4E-1 | 1.9E-1 | 2.8E-1 | 2.6E-3 | 2.6E-3 | 1.2E0 | 8.6E-1 | 121 | 9 | 43 | 9 | 0.41 |
| cF | ng/ml | 4.0E0 | 5.6E0 | 4.9E0 | 5.8E0 | 4.2E0 | 2.3E0 | 1.2E0 | 2.6E0 | 4.6E1 | 1.1E1 | 302 | 25 | 168 | 25 | 0.67 |
| cC | pg/ml | 3.1E2 | 2.7E2 | 3.6E2 | 3.2E2 | 2.2E2 | 2.3E2 | 9.9E0 | 1.6E2 | 1.4E3 | 1.2E3 | 231 | 18 | 159 | 18 | 0.41 |
| eD | pg/ml | 2.3E2 | 2.5E2 | 5.8E2 | 2.5E2 | 1.2E3 | 4.7E1 | 5.2E-1 | 1.9E2 | 8.3E3 | 3.4E2 | 197 | 7 | 131 | 7 | 0.57 |
| cM | ng/ml | 3.2E0 | 3.8E0 | 4.9E0 | 8.0E0 | 5.5E0 | 7.4E0 | 3.3E-1 | 1.8E0 | 3.9E1 | 2.5E1 | 155 | 9 | 67 | 9 | 0.66 |
| eP | ng/ml | 3.7E-3 | 3.1E-2 | 6.4E-1 | 7.5E0 | 1.6E0 | 2.2E1 | 3.7E-3 | 3.7E-3 | 1.2E1 | 6.5E1 | 121 | 9 | 43 | 9 | 0.59 |
| eX | ng/ml | 4.0E0 | 1.3E1 | 1.5E1 | 2.1E1 | 2.0E1 | 2.1E1 | 8.6E-4 | 1.8E0 | 7.3E1 | 4.9E1 | 83 | 8 | 60 | 8 | 0.65 |
| fP | ng/ml | 2.4E2 | 2.9E2 | 2.8E2 | 3.9E2 | 1.7E2 | 3.4E2 | 1.8E0 | 1.2E2 | 1.0E3 | 1.6E3 | 276 | 21 | 162 | 21 | 0.61 |
| fR | ng/ml | 1.2E5 | 2.0E5 | 1.7E5 | 2.9E5 | 1.4E5 | 2.2E5 | 3.1E4 | 2.9E4 | 7.7E5 | 8.3E5 | 295 | 17 | 90 | 17 | 0.70 |
| gC | ng/ml | 2.3E2 | 2.3E2 | 2.6E2 | 2.5E2 | 1.3E2 | 8.6E1 | 8.3E1 | 1.7E2 | 1.1E3 | 4.4E2 | 127 | 8 | 74 | 8 | 0.53 |
| gN | U/ml | 3.6E2 | 4.0E2 | 4.4E2 | 5.2E2 | 2.9E2 | 2.6E2 | 1.9E0 | 3.1E2 | 1.3E3 | 9.3E2 | 61 | 8 | 38 | 8 | 0.61 |
| gL | pg/ml | 6.3E4 | 7.9E4 | 7.0E4 | 8.6E4 | 3.1E4 | 3.4E4 | 1.4E4 | 3.1E4 | 2.0E5 | 1.6E5 | 287 | 25 | 167 | 25 | 0.66 |
| gP | U/ml | 2.8E2 | 2.3E2 | 2.9E2 | 2.2E2 | 1.1E2 | 8.2E1 | 1.2E1 | 7.9E1 | 1.1E3 | 3.9E2 | 298 | 25 | 168 | 25 | 0.32 |
| gT | ng/ml | 2.1E1 | 2.0E1 | 2.1E1 | 2.2E1 | 5.0E0 | 4.7E0 | 1.2E1 | 1.7E1 | 3.6E1 | 3.0E1 | 64 | 8 | 44 | 8 | 0.56 |
| gW | ng/ml | 6.8E2 | 5.2E2 | 1.3E3 | 1.4E3 | 1.7E3 | 2.1E3 | 3.1E-1 | 8.3E1 | 9.5E3 | 8.2E3 | 252 | 25 | 157 | 25 | 0.50 |
| tF | pg/mL | 1.4E3 | 1.7E3 | 1.5E4 | 1.0E4 | 4.5E4 | 2.2E4 | 1.2E1 | 1.8E1 | 3.2E5 | 9.4E4 | 231 | 18 | 159 | 18 | 0.52 |
| hA | ng/ml | 2.0E0 | 3.8E0 | 9.2E0 | 8.1E0 | 3.7E1 | 9.1E0 | 1.7E-2 | 5.7E-1 | 3.5E2 | 2.7E1 | 197 | 7 | 131 | 7 | 0.69 |
| nM | pg/ml | 1.0E-9 | 1.0E-9 | 7.9E1 | 1.0E-9 | 9.0E2 | 0.0E0 | 1.0E-9 | 1.0E-9 | 1.0E4 | 1.0E-9 | 131 | 7 | 103 | 7 | 0.49 |
| nN | pg/ml | 1.2E3 | 2.0E3 | 5.1E3 | 2.0E3 | 2.5E4 | 1.0E3 | 1.1E2 | 7.3E2 | 2.7E5 | 3.5E3 | 131 | 7 | 103 | 7 | 0.61 |
| nO | pg/ml | 2.7E1 | 4.4E1 | 4.3E1 | 8.8E1 | 4.2E1 | 8.6E1 | 3.5E0 | 8.1E0 | 2.4E2 | 2.6E2 | 131 | 7 | 103 | 7 | 0.68 |
| nR | pg/ml | 1.3E1 | 6.6E1 | 3.8E1 | 8.6E1 | 8.7E1 | 7.1E1 | 1.0E-9 | 2.9E0 | 8.2E2 | 2.2E2 | 131 | 7 | 103 | 7 | 0.76 |
| nT | pg/ml | 8.5E1 | 6.7E1 | 2.2E2 | 3.4E2 | 8.0E2 | 6.4E2 | 1.0E-9 | 1.0E1 | 6.6E3 | 1.8E3 | 131 | 7 | 103 | 7 | 0.53 |
| nU | pg/ml | 2.9E1 | 7.9E1 | 2.6E2 | 5.0E2 | 1.5E3 | 1.1E3 | 1.0E-9 | 2.9E1 | 1.3E4 | 3.0E3 | 131 | 7 | 103 | 7 | 0.73 |
| lW | pg/ml | 1.0E-9 | 1.0E-9 | 1.3E1 | 1.7E1 | 4.7E1 | 2.9E1 | 1.0E-9 | 1.0E-9 | 3.9E2 | 7.4E1 | 131 | 7 | 103 | 7 | 0.58 |
| lX | pg/ml | 1.0E3 | 5.4E2 | 1.1E3 | 8.0E2 | 5.6E2 | 6.6E2 | 1.2E2 | 3.8E2 | 2.6E3 | 2.3E3 | 131 | 7 | 103 | 7 | 0.32 |
| lY | pg/ml | 2.0E1 | 2.2E1 | 2.3E1 | 2.1E1 | 2.1E1 | 5.7E0 | 1.0E-9 | 1.2E1 | 1.4E2 | 2.8E1 | 131 | 7 | 103 | 7 | 0.52 |
| mE | pg/ml | 1.0E-9 | 1.2E0 | 2.9E0 | 3.5E0 | 8.3E0 | 5.1E0 | 1.0E-9 | 1.0E-9 | 5.8E1 | 1.3E1 | 131 | 7 | 103 | 7 | 0.60 |
| mF | pg/ml | 1.0E-9 | 4.8E0 | 4.0E0 | 4.6E0 | 2.3E1 | 4.6E0 | 1.0E-9 | 1.0E-9 | 2.5E2 | 1.3E1 | 131 | 7 | 103 | 7 | 0.72 |
| mH | pg/ml | 3.6E0 | 1.3E0 | 5.1E0 | 4.8E0 | 6.7E0 | 8.2E0 | 2.3E-1 | 4.2E-1 | 5.3E1 | 2.3E1 | 131 | 7 | 103 | 7 | 0.33 |
| mI | pg/ml | 1.0E-9 | 2.3E1 | 1.2E1 | 4.4E1 | 2.7E1 | 6.3E1 | 1.0E-9 | 1.0E-9 | 1.8E2 | 1.8E2 | 131 | 7 | 103 | 7 | 0.71 |
| mM | pg/ml | 2.2E1 | 3.5E1 | 6.5E1 | 9.3E1 | 1.5E2 | 1.1E2 | 1.0E-9 | 1.8E0 | 1.1E3 | 2.6E2 | 131 | 7 | 103 | 7 | 0.63 |
| mP | pg/ml | 1.4E1 | 1.7E1 | 1.8E1 | 1.9E1 | 2.3E1 | 9.9E0 | 1.0E-9 | 1.0E1 | 1.9E2 | 3.7E1 | 130 | 7 | 102 | 7 | 0.58 |
| mS | pg/ml | 1.8E3 | 1.7E3 | 2.0E3 | 2.7E3 | 1.6E3 | 1.8E3 | 1.0E-9 | 1.0E3 | 1.3E4 | 5.9E3 | 131 | 7 | 103 | 7 | 0.62 |
| mT | pg/ml | 4.8E1 | 8.5E1 | 1.2E2 | 3.4E2 | 2.1E2 | 7.1E2 | 9.7E0 | 1.4E1 | 1.4E3 | 1.9E3 | 130 | 7 | 102 | 7 | 0.55 |
| mU | pg/ml | 2.2E0 | 2.9E0 | 5.5E0 | 3.3E0 | 2.1E1 | 1.9E0 | 1.0E-9 | 1.5E0 | 2.2E2 | 7.2E0 | 130 | 7 | 102 | 7 | 0.64 |
| mW | pg/ml | 2.3E3 | 2.4E3 | 2.6E3 | 3.7E3 | 1.4E3 | 2.4E3 | 3.1E2 | 1.2E3 | 1.0E4 | 7.0E3 | 130 | 7 | 102 | 7 | 0.61 |
| mY | pg/ml | 5.6E2 | 4.8E2 | 8.6E2 | 9.8E2 | 1.3E3 | 9.0E2 | 1.0E-9 | 1.5E2 | 1.1E4 | 2.4E3 | 131 | 7 | 103 | 7 | 0.53 |
| mZ | pg/ml | 2.2E2 | 6.9E2 | 3.8E2 | 8.7E2 | 4.4E2 | 9.1E2 | 1.0E-9 | 6.6E1 | 3.1E3 | 2.8E3 | 130 | 7 | 102 | 7 | 0.69 |
| nA | pg/ml | 2.0E0 | 4.0E0 | 1.1E1 | 6.7E0 | 4.4E1 | 1.1E1 | 1.0E-9 | 1.0E-9 | 4.4E2 | 3.1E1 | 130 | 7 | 102 | 7 | 0.54 |
| nB | pg/ml | 3.0E2 | 4.1E2 | 3.1E2 | 4.3E2 | 1.5E2 | 1.7E2 | 3.0E1 | 2.5E2 | 8.2E2 | 7.6E2 | 131 | 7 | 103 | 7 | 0.71 |
| nC | pg/ml | 1.0E-9 | 2.8E3 | 3.7E3 | 9.9E4 | 3.2E4 | 2.5E5 | 1.0E-9 | 1.0E-9 | 3.7E5 | 6.7E5 | 131 | 7 | 103 | 7 | 0.75 |
| nD | pg/ml | 8.5E0 | 2.5E1 | 3.5E1 | 4.0E1 | 2.0E2 | 4.8E1 | 1.0E-9 | 8.5E0 | 1.7E3 | 1.5E2 | 130 | 7 | 102 | 7 | 0.84 |
| nF | pg/ml | 1.0E-9 | 1.0E-9 | 5.0E0 | 5.6E0 | 2.6E1 | 1.3E1 | 1.0E-9 | 1.0E-9 | 2.4E2 | 3.6E1 | 131 | 7 | 103 | 7 | 0.57 |
| nH | pg/ml | 3.8E-1 | 3.0E1 | 8.9E1 | 4.3E2 | 8.8E2 | 9.6E2 | 1.0E-9 | 1.0E-9 | 1.0E4 | 2.6E3 | 130 | 7 | 102 | 7 | 0.71 |
| nI | pg/ml | 4.6E1 | 6.6E1 | 1.6E2 | 2.1E2 | 8.4E2 | 4.4E2 | 1.0E-9 | 1.0E-9 | 9.2E3 | 1.2E3 | 131 | 7 | 103 | 7 | 0.56 |
| nJ | pg/ml | 1.7E-1 | 1.0E-9 | 4.1E1 | 2.5E0 | 4.5E2 | 4.9E0 | 1.0E-9 | 1.0E-9 | 5.2E3 | 1.3E1 | 131 | 7 | 103 | 7 | 0.50 |
| nK | pg/ml | 1.0E-9 | 1.6E1 | 5.8E1 | 4.4E1 | 3.4E2 | 8.4E1 | 1.0E-9 | 1.0E-9 | 3.2E3 | 2.3E2 | 130 | 7 | 102 | 7 | 0.64 |
| nL | pg/ml | 1.0E-9 | 7.4E1 | 3.9E2 | 8.6E2 | 3.9E3 | 1.8E3 | 1.0E-9 | 1.0E-9 | 4.5E4 | 4.8E3 | 131 | 7 | 103 | 7 | 0.69 |
| hR | pg/ml | 2.6E4 | 2.5E4 | 2.7E4 | 2.7E4 | 1.1E4 | 9.2E3 | 1.1E1 | 1.2E4 | 5.8E4 | 4.3E4 | 189 | 7 | 129 | 7 | 0.52 |
| hV | pg/ml | 4.4E2 | 2.3E2 | 4.7E2 | 2.4E2 | 2.4E2 | 5.8E1 | 6.8E1 | 1.7E2 | 1.5E3 | 3.6E2 | 189 | 7 | 129 | 7 | 0.16 |
| hW | pg/ml | 1.5E3 | 2.1E3 | 2.0E3 | 2.4E3 | 1.6E3 | 2.0E3 | 2.2E2 | 5.7E2 | 1.0E4 | 6.4E3 | 189 | 7 | 129 | 7 | 0.59 |
| hX | pg/ml | 9.0E2 | 8.2E2 | 1.0E3 | 1.7E3 | 7.8E2 | 2.2E3 | 1.3E2 | 4.6E2 | 8.6E3 | 6.6E3 | 189 | 7 | 129 | 7 | 0.45 |
| iA | pg/ml | 1.4E2 | 2.1E2 | 2.8E2 | 3.5E2 | 6.2E2 | 5.0E2 | 5.8E0 | 8.2E0 | 7.1E3 | 2.2E3 | 231 | 18 | 159 | 18 | 0.63 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| iB | ng/ml | 4.8E0 | 6.3E0 | 6.0E0 | 9.3E0 | 4.9E0 | 8.4E0 | 3.3E-2 | 1.8E0 | 3.8E1 | 2.6E1 | 197 | 7 | 131 | 7 | 0.62 |
| iC | U/ml | 2.1E-1 | 5.9E-1 | 9.0E-1 | 2.6E0 | 4.6E0 | 4.9E0 | 1.0E-9 | 1.0E-9 | 5.5E1 | 1.4E1 | 197 | 7 | 131 | 7 | 0.73 |
| iH | ng/ml | 1.6E5 | 1.9E5 | 1.5E5 | 1.7E5 | 4.7E4 | 5.1E4 | 5.1E4 | 7.8E4 | 2.7E5 | 2.5E5 | 231 | 18 | 159 | 18 | 0.63 |
| iJ | ng/ml | 5.4E4 | 5.0E4 | 5.5E4 | 5.9E4 | 2.9E4 | 2.0E4 | 5.5E3 | 3.3E4 | 2.5E5 | 9.7E4 | 231 | 18 | 159 | 18 | 0.55 |
| hB | ng/ml | 4.1E-1 | 4.8E-1 | 5.0E-1 | 7.3E-1 | 3.3E-1 | 7.7E-1 | 1.0E-9 | 1.5E-1 | 2.3E0 | 3.4E0 | 231 | 18 | 159 | 18 | 0.58 |
| hC | pg/ml | 3.9E3 | 3.2E3 | 6.8E3 | 7.0E3 | 1.0E4 | 7.5E3 | 1.0E-9 | 1.0E-9 | 1.1E5 | 2.5E4 | 231 | 18 | 159 | 18 | 0.51 |
| hF | ng/ml | 1.0E-9 | 1.0E-9 | 2.1E1 | 1.0E-9 | 2.7E2 | 0.0E0 | 1.0E-9 | 1.0E-9 | 4.0E3 | 1.0E-9 | 231 | 18 | 159 | 18 | 0.49 |
| hG | pg/ml | 6.9E3 | 6.8E3 | 7.4E3 | 8.0E3 | 3.2E3 | 3.7E3 | 2.8E1 | 3.6E3 | 1.9E4 | 1.7E4 | 231 | 18 | 159 | 18 | 0.53 |
| iO | ng/ml | 3.8E5 | 3.4E5 | 4.0E5 | 3.6E5 | 1.8E5 | 1.2E5 | 1.1E4 | 1.4E5 | 1.1E6 | 5.4E5 | 231 | 18 | 159 | 18 | 0.46 |
| iP | ng/ml | 5.0E4 | 4.0E4 | 5.5E4 | 6.1E4 | 5.2E4 | 4.5E4 | 1.0E-9 | 5.6E3 | 5.5E5 | 1.6E5 | 231 | 18 | 159 | 18 | 0.52 |
| iZ | ng/ml | 1.6E3 | 2.0E3 | 1.8E3 | 2.4E3 | 7.8E2 | 1.3E3 | 4.7E2 | 9.8E2 | 5.7E3 | 6.5E3 | 230 | 18 | 158 | 18 | 0.65 |
| rC | pg/ml | 1.6E3 | 1.6E3 | 2.2E3 | 1.6E3 | 2.2E3 | 9.0E2 | 1.0E-9 | 4.8E2 | 1.5E4 | 2.5E3 | 189 | 7 | 128 | 7 | 0.47 |
| rB | pg/ml | 2.4E1 | 7.1E1 | 4.3E1 | 7.3E1 | 8.9E1 | 3.6E1 | 1.0E-9 | 2.6E1 | 9.5E2 | 1.4E2 | 189 | 7 | 128 | 7 | 0.84 |
| jD | ng/ml | 3.1E1 | 4.4E1 | 4.8E1 | 7.5E1 | 6.1E1 | 9.9E1 | 1.0E-9 | 1.0E-9 | 5.1E2 | 2.9E2 | 196 | 7 | 131 | 7 | 0.59 |
| jE | ng/ml | 1.0E-9 | 1.0E-9 | 7.1E0 | 1.1E1 | 1.7E1 | 1.9E1 | 1.0E-9 | 1.0E-9 | 1.2E2 | 4.6E1 | 196 | 7 | 131 | 7 | 0.54 |
| jF | ng/ml | 4.2E1 | 1.0E-9 | 5.7E1 | 2.6E1 | 6.3E1 | 5.6E1 | 1.0E-9 | 1.0E-9 | 3.5E2 | 1.5E2 | 196 | 7 | 131 | 7 | 0.29 |
| jG | ng/ml | 4.6E3 | 6.4E3 | 4.7E3 | 5.4E3 | 2.0E3 | 2.6E3 | 7.6E2 | 6.4E2 | 1.1E4 | 7.9E3 | 197 | 7 | 131 | 7 | 0.63 |
| jH | ng/ml | 7.6E1 | 1.1E2 | 8.5E1 | 9.8E1 | 4.8E1 | 3.3E1 | 1.3E1 | 4.8E1 | 3.3E2 | 1.4E2 | 197 | 7 | 131 | 7 | 0.65 |
| jI | ng/ml | 6.8E1 | 7.7E1 | 7.3E1 | 9.3E1 | 3.3E1 | 5.0E1 | 1.9E1 | 3.8E1 | 2.5E2 | 1.9E2 | 197 | 7 | 131 | 7 | 0.63 |
| rA | pg/ml | 2.6E1 | 1.5E1 | 3.1E1 | 1.6E1 | 2.5E1 | 8.8E0 | 1.0E-9 | 6.9E0 | 2.0E2 | 3.1E1 | 197 | 7 | 131 | 7 | 0.27 |
| qY | pg/ml | 2.6E1 | 7.6E1 | 5.1E1 | 6.5E1 | 6.6E1 | 5.9E1 | 8.7E-1 | 3.3E0 | 5.3E2 | 1.5E2 | 197 | 7 | 131 | 7 | 0.54 |
| qX | pg/ml | 5.9E1 | 6.0E1 | 6.5E1 | 4.9E1 | 4.2E1 | 3.2E1 | 1.0E-9 | 1.0E-9 | 2.1E2 | 8.5E1 | 197 | 7 | 131 | 7 | 0.41 |
| qW | pg/ml | 9.2E0 | 6.3E0 | 1.4E1 | 9.1E0 | 1.6E1 | 1.0E1 | 1.0E-9 | 4.0E-1 | 1.2E2 | 3.1E1 | 197 | 7 | 131 | 7 | 0.39 |
| qV | pg/ml | 2.2E3 | 2.8E3 | 2.9E3 | 4.4E3 | 2.1E3 | 5.7E3 | 1.0E2 | 8.8E2 | 1.1E4 | 1.7E4 | 197 | 7 | 131 | 7 | 0.52 |
| qU | pg/ml | 6.1E1 | 1.4E2 | 1.6E2 | 2.3E2 | 2.8E2 | 3.1E2 | 1.0E-9 | 3.0E1 | 2.1E3 | 9.1E2 | 197 | 7 | 131 | 7 | 0.64 |
| qT | pg/ml | 4.0E1 | 4.3E1 | 6.8E1 | 5.9E1 | 9.8E1 | 5.2E1 | 1.0E-9 | 1.1E1 | 9.0E2 | 1.7E2 | 197 | 7 | 131 | 7 | 0.54 |
| jK | ng/ml | 1.6E3 | 2.0E3 | 1.7E3 | 1.7E3 | 6.5E2 | 9.8E2 | 2.8E2 | 4.7E2 | 4.3E3 | 3.3E3 | 197 | 7 | 131 | 7 | 0.53 |
| jL | ng/ml | 1.9E2 | 2.7E2 | 2.8E2 | 2.6E2 | 2.5E2 | 8.5E1 | 3.6E1 | 1.6E2 | 2.1E3 | 3.9E2 | 197 | 7 | 131 | 7 | 0.62 |
| jM | ng/ml | 7.4E4 | 5.0E4 | 7.8E4 | 6.4E4 | 3.9E4 | 4.6E4 | 3.9E2 | 7.8E3 | 1.9E5 | 1.5E5 | 197 | 7 | 131 | 7 | 0.39 |
| jO | pg/ml | 2.1E5 | 2.0E5 | 2.6E5 | 2.7E5 | 1.5E5 | 1.6E5 | 5.2E4 | 1.2E5 | 1.1E6 | 4.8E5 | 197 | 7 | 131 | 7 | 0.50 |
| jP | pg/ml | 2.2E5 | 2.2E5 | 2.6E5 | 3.5E5 | 1.6E5 | 3.9E5 | 3.6E4 | 1.1E5 | 9.2E5 | 1.2E6 | 197 | 7 | 131 | 7 | 0.49 |
| jQ | pg/ml | 2.7E3 | 1.3E3 | 3.8E3 | 1.9E3 | 3.5E3 | 2.0E3 | 1.0E-9 | 4.6E2 | 1.8E4 | 6.3E3 | 197 | 7 | 131 | 7 | 0.33 |
| jR | pg/ml | 7.7E3 | 3.3E3 | 1.2E4 | 5.8E3 | 1.3E4 | 8.4E3 | 1.0E-9 | 1.0E-9 | 9.0E4 | 2.5E4 | 197 | 7 | 131 | 7 | 0.32 |
| jT | pg/ml | 1.8E5 | 1.7E5 | 1.8E5 | 1.9E5 | 6.6E4 | 8.1E4 | 6.8E4 | 8.9E4 | 4.5E5 | 3.0E5 | 197 | 7 | 131 | 7 | 0.53 |
| jU | mIU/ml | 4.6E0 | 6.2E0 | 1.1E1 | 8.7E0 | 1.7E1 | 7.2E0 | 6.2E-2 | 1.9E-1 | 1.1E2 | 1.7E1 | 197 | 7 | 131 | 7 | 0.55 |
| jV | mIU/ml | 1.5E0 | 1.1E0 | 3.8E0 | 3.7E0 | 6.4E0 | 5.6E0 | 1.7E-3 | 6.9E-4 | 3.5E1 | 1.4E1 | 197 | 7 | 131 | 7 | 0.42 |
| jY | ng/ml | 5.9E-4 | 3.3E-3 | 5.8E-3 | 6.2E-3 | 2.7E-2 | 5.3E-3 | 1.0E-9 | 1.0E-9 | 3.0E-1 | 1.3E-2 | 197 | 7 | 131 | 7 | 0.74 |
| kC | pg/ml | 9.7E1 | 9.7E1 | 1.8E2 | 1.9E2 | 3.8E2 | 2.0E2 | 2.1E1 | 5.9E1 | 3.5E3 | 6.0E2 | 131 | 7 | 103 | 7 | 0.54 |
| kE | pg/ml | 1.3E5 | 1.5E5 | 1.3E5 | 1.5E5 | 3.8E4 | 3.8E4 | 1.2E4 | 9.0E4 | 2.3E5 | 2.0E5 | 131 | 7 | 103 | 7 | 0.60 |
| kF | pg/mL | 6.0E1 | 7.2E1 | 6.8E1 | 7.6E1 | 4.8E1 | 3.1E1 | 2.6E1 | 5.0E1 | 5.1E2 | 1.4E2 | 131 | 7 | 103 | 7 | 0.62 |
| kG | pg/mL | 9.2E3 | 7.1E3 | 1.2E4 | 1.5E4 | 1.4E4 | 1.7E4 | 7.5E2 | 1.1E3 | 1.2E5 | 4.3E4 | 131 | 7 | 103 | 7 | 0.45 |
| kI | pg/ml | 1.8E2 | 1.9E2 | 2.2E2 | 2.9E2 | 1.3E2 | 2.7E2 | 4.4E1 | 9.5E1 | 8.7E2 | 8.7E2 | 131 | 7 | 103 | 7 | 0.52 |
| kK | pg/ml | 1.0E2 | 1.7E2 | 1.6E2 | 2.6E2 | 1.9E2 | 2.5E2 | 6.4E0 | 3.2E1 | 1.6E3 | 6.9E2 | 131 | 7 | 103 | 7 | 0.62 |
| kN | pg/ml | 9.5E2 | 4.4E2 | 2.0E3 | 6.1E3 | 5.2E3 | 1.5E4 | 7.6E1 | 2.9E2 | 5.5E4 | 3.9E4 | 131 | 7 | 103 | 7 | 0.33 |
| kO | pg/ml | 7.2E3 | 9.5E3 | 8.6E3 | 1.1E4 | 1.1E4 | 5.3E3 | 3.4E3 | 5.2E3 | 1.3E5 | 1.7E4 | 131 | 7 | 103 | 7 | 0.63 |
| kP | pg/ml | 6.3E3 | 4.7E3 | 7.5E3 | 5.0E3 | 6.2E3 | 3.1E3 | 8.6E2 | 1.2E3 | 4.8E4 | 9.8E3 | 131 | 7 | 103 | 7 | 0.36 |
| kQ | pg/ml | 4.1E3 | 4.8E3 | 4.9E3 | 5.8E3 | 3.0E3 | 4.6E3 | 5.6E2 | 1.6E3 | 2.5E4 | 2.1E4 | 231 | 18 | 159 | 18 | 0.53 |
| kR | pg/ml | 2.1E1 | 2.1E1 | 3.1E1 | 2.5E1 | 6.9E1 | 1.8E1 | 1.0E-9 | 1.2E0 | 1.0E3 | 7.1E1 | 231 | 18 | 159 | 18 | 0.48 |
| kS | pg/ml | 8.0E2 | 7.0E2 | 9.7E2 | 1.1E3 | 1.0E3 | 1.0E3 | 8.2E1 | 7.9E1 | 1.4E4 | 3.6E3 | 231 | 18 | 159 | 18 | 0.50 |
| rZ | ng/ml | 1.0E-9 | 6.9E-3 | 5.4E-3 | 4.2E-2 | 1.5E-2 | 9.7E-2 | 1.0E-9 | 1.0E-9 | 1.1E-1 | 2.6E-1 | 190 | 7 | 126 | 7 | 0.63 |
| rY | ng/ml | 5.7E-2 | 6.1E-2 | 3.6E-1 | 2.5E0 | 2.3E0 | 6.3E0 | 1.0E-9 | 1.2E-2 | 2.3E1 | 1.7E1 | 190 | 7 | 126 | 7 | 0.61 |
| rX | ng/ml | 1.0E-9 | 1.0E-9 | 6.9E-2 | 2.9E-1 | 4.2E-1 | 7.0E-1 | 1.0E-9 | 1.0E-9 | 3.7E0 | 1.9E0 | 190 | 7 | 126 | 7 | 0.64 |
| lK | pg/ml | 9.9E1 | 1.3E1 | 1.9E2 | 5.9E1 | 3.1E2 | 7.8E1 | 1.0E-9 | 1.0E-9 | 3.3E3 | 2.1E2 | 196 | 7 | 131 | 7 | 0.30 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| lL | pg/ml | 1.6E3 | 3.2E3 | 2.6E3 | 3.5E3 | 3.7E3 | 2.1E3 | 1.5E1 | 2.1E2 | 4.2E4 | 6.3E3 | 197 | 7 | 131 | 7 | 0.69 |
| lM | pg/ml | 1.1E3 | 1.7E3 | 3.5E3 | 1.9E3 | 7.5E3 | 1.8E3 | 1.2E2 | 2.4E1 | 5.1E4 | 4.9E3 | 197 | 7 | 131 | 7 | 0.46 |
| lN | pg/ml | 1.0E-9 | 1.0E-9 | 4.2E0 | 6.9E0 | 1.5E1 | 1.3E1 | 1.0E-9 | 1.0E-9 | 1.5E2 | 3.5E1 | 197 | 7 | 131 | 7 | 0.56 |
| lO | pg/ml | 1.0E-9 | 1.0E-9 | 1.6E0 | 1.2E1 | 1.1E1 | 3.2E1 | 1.0E-9 | 1.0E-9 | 1.3E2 | 8.4E1 | 196 | 7 | 131 | 7 | 0.56 |
| nW | pg/ml | 1.1E5 | 1.1E5 | 1.1E5 | 1.1E5 | 2.5E4 | 2.8E4 | 5.8E4 | 7.2E4 | 1.8E5 | 1.9E5 | 231 | 18 | 159 | 18 | 0.49 |
| nY | pg/ml | 2.1E3 | 2.1E3 | 2.4E3 | 2.3E3 | 1.6E3 | 1.2E3 | 5.1E2 | 5.4E2 | 1.3E4 | 5.7E3 | 231 | 18 | 159 | 18 | 0.49 |
| oO | pg/ml | 7.8E4 | 1.2E5 | 1.1E5 | 1.3E5 | 9.7E4 | 5.7E4 | 1.5E4 | 5.2E4 | 6.2E5 | 2.3E5 | 123 | 7 | 97 | 7 | 0.67 |
| oP | pg/ml | 1.2E5 | 2.5E5 | 1.4E5 | 2.5E5 | 9.1E4 | 7.1E4 | 2.4E4 | 1.5E5 | 4.5E5 | 3.3E5 | 123 | 7 | 97 | 7 | 0.83 |
| oQ | pg/ml | 2.8E3 | 5.8E3 | 3.5E3 | 7.2E3 | 2.8E3 | 5.8E3 | 9.3E2 | 2.6E3 | 2.1E4 | 2.0E4 | 123 | 7 | 97 | 7 | 0.82 |
| oE | pg/ml | 1.3E2 | 1.4E2 | 3.6E2 | 2.8E2 | 5.4E2 | 3.4E2 | 1.0E-9 | 2.8E0 | 4.7E3 | 1.4E3 | 231 | 18 | 159 | 18 | 0.49 |
| oF | pg/ml | 7.7E3 | 1.2E4 | 2.0E4 | 3.7E4 | 3.6E4 | 5.7E4 | 6.4E1 | 4.8E2 | 2.5E5 | 1.8E5 | 231 | 18 | 159 | 18 | 0.60 |
| oH | pg/ml | 4.4E1 | 4.8E1 | 9.3E1 | 1.1E2 | 1.4E2 | 1.7E2 | 4.2E0 | 1.6E1 | 9.9E2 | 6.3E2 | 231 | 18 | 159 | 18 | 0.53 |
| oK | pg/ml | 7.6E2 | 8.5E2 | 1.8E3 | 4.6E3 | 2.5E3 | 1.2E4 | 5.2E1 | 3.1E2 | 1.8E4 | 5.3E4 | 231 | 18 | 159 | 18 | 0.58 |
| oN | pg/ml | 5.0E2 | 5.6E2 | 7.5E2 | 8.1E2 | 1.4E3 | 1.1E3 | 1.5E2 | 2.4E2 | 1.8E4 | 5.0E3 | 231 | 18 | 159 | 18 | 0.53 |
| pF | pg/ml | 4.5E-1 | 5.5E-1 | 1.0E0 | 1.2E0 | 5.7E0 | 2.3E0 | 1.0E-9 | 1.0E-9 | 8.7E1 | 1.0E1 | 231 | 18 | 159 | 18 | 0.52 |

Figure 10.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.0E1 | 6.5E1 | 8.2E1 | 7.4E1 | 5.8E1 | 6.9E1 | 1.0E0 | 1.4E1 | 4.8E2 | 2.4E2 | 1777 | 8 | 298 | 8 | 0.43 |
| Po | pg/ml | 6.9E-1 | 1.2E1 | 8.6E0 | 4.2E1 | 2.4E1 | 7.0E1 | 8.0E-3 | 2.6E-2 | 2.7E2 | 2.2E2 | 899 | 9 | 335 | 9 | 0.72 |
| Et | ng/ml | 1.4E3 | 2.4E3 | 1.7E3 | 2.6E3 | 1.2E3 | 1.1E3 | 7.5E1 | 1.4E3 | 5.0E3 | 4.4E3 | 898 | 9 | 335 | 9 | 0.73 |
| Fp | ng/ml | 1.4E1 | 2.5E1 | 2.5E1 | 3.2E1 | 2.9E1 | 2.4E1 | 6.0E-3 | 3.8E0 | 1.4E2 | 6.7E1 | 931 | 9 | 336 | 9 | 0.63 |
| Fr | ng/ml | 3.7E4 | 5.4E5 | 1.2E5 | 4.8E5 | 1.8E5 | 3.0E5 | 1.9E2 | 4.1E4 | 9.0E5 | 8.5E5 | 1043 | 10 | 340 | 10 | 0.87 |
| Nm | pg/ml | 1.4E4 | 3.9E4 | 3.3E4 | 3.6E4 | 8.2E4 | 3.0E4 | 1.0E-9 | 1.4E3 | 1.6E6 | 7.9E4 | 902 | 9 | 337 | 9 | 0.60 |
| Nn | pg/ml | 1.6E2 | 2.0E3 | 1.9E3 | 1.5E4 | 8.2E3 | 2.4E4 | 1.0E-9 | 6.0E1 | 1.0E5 | 6.9E4 | 902 | 9 | 337 | 9 | 0.77 |
| No | pg/ml | 1.6E1 | 6.7E1 | 3.8E1 | 2.4E2 | 1.1E2 | 4.7E2 | 1.0E-9 | 4.7E0 | 2.5E3 | 1.4E3 | 902 | 9 | 337 | 9 | 0.75 |
| Nq | pg/ml | 2.0E0 | 1.0E1 | 2.0E1 | 2.7E1 | 7.6E1 | 4.0E1 | 1.0E-9 | 1.0E-9 | 1.2E3 | 1.1E2 | 902 | 9 | 337 | 9 | 0.69 |
| Nr | pg/ml | 1.2E0 | 1.0E-9 | 2.9E1 | 9.6E2 | 1.8E2 | 2.8E3 | 1.0E-9 | 1.0E-9 | 4.1E3 | 8.5E3 | 902 | 9 | 337 | 9 | 0.47 |
| Ns | pg/ml | 1.0E-9 | 1.0E-9 | 9.2E0 | 1.3E1 | 5.2E1 | 4.0E1 | 1.0E-9 | 1.0E-9 | 1.1E3 | 1.2E2 | 902 | 9 | 337 | 9 | 0.51 |
| Nt | pg/ml | 1.0E2 | 1.4E2 | 1.4E2 | 1.9E2 | 1.2E2 | 2.0E2 | 1.0E-9 | 3.5E1 | 1.7E3 | 6.8E2 | 902 | 9 | 337 | 9 | 0.56 |
| Nu | pg/ml | 2.0E1 | 5.0E1 | 5.5E1 | 1.0E2 | 9.0E1 | 1.8E2 | 1.0E-9 | 1.0E-9 | 8.9E2 | 5.8E2 | 902 | 9 | 337 | 9 | 0.59 |
| Lu | pg/ml | 1.0E4 | 7.8E3 | 1.8E4 | 7.8E3 | 6.2E4 | 6.4E3 | 3.5E2 | 1.0E3 | 1.3E6 | 2.1E4 | 905 | 9 | 337 | 9 | 0.35 |
| Lv | pg/ml | 1.0E-9 | 3.3E1 | 1.1E1 | 4.2E1 | 2.2E1 | 4.0E1 | 1.0E-9 | 1.0E-9 | 2.6E2 | 1.0E2 | 905 | 9 | 337 | 9 | 0.74 |
| Lw | pg/ml | 1.0E-9 | 1.0E-9 | 3.7E-1 | 1.9E0 | 3.8E0 | 5.6E0 | 1.0E-9 | 1.0E-9 | 8.0E1 | 1.7E1 | 905 | 9 | 337 | 9 | 0.60 |
| Lx | pg/ml | 1.0E-9 | 4.9E2 | 1.7E2 | 1.9E3 | 8.6E2 | 3.2E3 | 1.0E-9 | 6.0E1 | 2.2E4 | 1.0E4 | 905 | 9 | 337 | 9 | 0.89 |
| Ly | pg/ml | 1.0E-9 | 1.0E-9 | 1.0E1 | 6.4E0 | 2.0E1 | 1.4E1 | 1.0E-9 | 1.0E-9 | 1.6E2 | 4.3E1 | 905 | 9 | 337 | 9 | 0.48 |
| Lz | pg/ml | 1.0E-9 | 1.0E-9 | 4.0E0 | 2.4E1 | 3.0E1 | 6.9E1 | 1.0E-9 | 1.0E-9 | 6.0E2 | 2.1E2 | 905 | 9 | 337 | 9 | 0.57 |
| Ma | pg/ml | 2.9E2 | 2.7E3 | 1.4E3 | 3.1E3 | 3.6E3 | 3.0E3 | 1.0E-9 | 5.8E1 | 6.5E4 | 9.5E3 | 905 | 9 | 337 | 9 | 0.74 |
| Mb | pg/ml | 2.5E1 | 2.2E1 | 3.1E1 | 2.7E1 | 1.5E1 | 1.0E1 | 4.1E0 | 1.5E1 | 2.1E2 | 4.3E1 | 905 | 9 | 337 | 9 | 0.42 |
| Mc | pg/ml | 1.0E-9 | 1.0E-9 | 3.8E-2 | 1.0E-9 | 5.7E-1 | 0.0E0 | 1.0E-9 | 1.0E-9 | 1.3E1 | 1.0E-9 | 905 | 9 | 337 | 9 | 0.49 |
| Md | pg/ml | 1.0E-9 | 1.0E-9 | 4.4E-1 | 1.3E0 | 3.6E0 | 3.6E0 | 1.0E-9 | 1.0E-9 | 6.8E1 | 1.1E1 | 905 | 9 | 337 | 9 | 0.57 |
| Me | pg/ml | 3.3E1 | 3.0E1 | 3.2E1 | 3.1E1 | 2.0E1 | 2.2E1 | 1.0E-9 | 3.2E0 | 3.2E2 | 7.9E1 | 905 | 9 | 337 | 9 | 0.43 |
| Mf | pg/ml | 1.0E-9 | 1.0E-9 | 5.4E-1 | 1.0E-9 | 2.9E0 | 0.0E0 | 1.0E-9 | 1.0E-9 | 5.6E1 | 1.0E-9 | 905 | 9 | 337 | 9 | 0.43 |
| Mg | pg/ml | 1.6E0 | 3.6E0 | 7.4E0 | 4.3E0 | 1.2E1 | 4.5E0 | 1.0E-9 | 1.0E-9 | 9.2E1 | 1.3E1 | 905 | 9 | 337 | 9 | 0.51 |
| Mh | pg/ml | 1.0E-9 | 1.0E-9 | 1.3E0 | 4.5E0 | 9.6E0 | 1.2E1 | 1.0E-9 | 1.0E-9 | 1.6E2 | 3.8E1 | 905 | 9 | 337 | 9 | 0.54 |
| Mi | pg/ml | 1.0E-9 | 1.0E-9 | 9.0E-1 | 3.2E1 | 1.2E1 | 5.4E1 | 1.0E-9 | 1.0E-9 | 3.2E2 | 1.6E2 | 905 | 9 | 337 | 9 | 0.71 |
| Mj | pg/ml | 1.0E-9 | 1.0E-9 | 4.5E0 | 6.3E1 | 2.5E1 | 1.8E2 | 1.0E-9 | 1.0E-9 | 4.6E2 | 5.4E2 | 905 | 9 | 337 | 9 | 0.58 |
| Mk | pg/ml | 1.0E0 | 1.0E-9 | 1.3E1 | 6.2E2 | 8.5E1 | 1.9E3 | 1.0E-9 | 1.0E-9 | 1.7E3 | 5.6E3 | 905 | 9 | 337 | 9 | 0.50 |
| Ml | pg/ml | 1.0E-9 | 1.0E-9 | 5.6E0 | 5.7E-1 | 7.5E1 | 1.1E0 | 1.0E-9 | 1.0E-9 | 2.1E3 | 2.8E0 | 905 | 9 | 337 | 9 | 0.52 |
| Mm | pg/ml | 6.1E2 | 1.1E3 | 1.1E3 | 1.7E3 | 1.5E3 | 2.1E3 | 1.0E-9 | 2.0E2 | 1.2E4 | 6.9E3 | 905 | 9 | 337 | 9 | 0.62 |
| Mn | pg/ml | 5.6E0 | 1.2E1 | 1.0E1 | 1.7E1 | 2.3E1 | 1.9E1 | 1.0E-9 | 1.0E-9 | 3.5E2 | 6.6E1 | 905 | 9 | 337 | 9 | 0.73 |
| Mp | pg/ml | 1.0E-9 | 1.7E1 | 1.0E1 | 5.1E1 | 3.4E1 | 6.4E1 | 1.0E-9 | 1.0E-9 | 6.3E2 | 1.8E2 | 904 | 9 | 337 | 9 | 0.70 |
| Mq | pg/ml | 1.0E-9 | 1.0E-9 | 2.9E0 | 1.2E1 | 1.7E1 | 3.5E1 | 1.0E-9 | 1.0E-9 | 2.4E2 | 1.1E2 | 904 | 9 | 337 | 9 | 0.52 |
| Mr | pg/ml | 1.0E-9 | 1.0E-9 | 2.8E1 | 1.3E3 | 1.6E2 | 3.9E3 | 1.0E-9 | 1.0E-9 | 2.2E3 | 1.2E4 | 904 | 9 | 337 | 9 | 0.57 |
| Ms | pg/ml | 4.1E2 | 2.0E2 | 5.6E2 | 3.4E2 | 6.5E2 | 3.0E2 | 1.0E-9 | 1.0E-9 | 5.9E3 | 8.6E2 | 904 | 9 | 337 | 9 | 0.42 |
| Mt | pg/ml | 2.5E-1 | 9.0E0 | 1.1E1 | 2.3E1 | 1.2E2 | 3.2E1 | 1.0E-9 | 1.3E0 | 3.2E3 | 1.0E2 | 904 | 9 | 337 | 9 | 0.87 |
| Mu | pg/ml | 1.0E-9 | 1.4E0 | 1.3E0 | 6.8E0 | 1.0E1 | 9.7E0 | 1.0E-9 | 1.0E-9 | 2.3E2 | 2.7E1 | 904 | 9 | 337 | 9 | 0.72 |
| Mv | pg/ml | 1.0E-9 | 2.6E1 | 7.0E1 | 1.2E2 | 3.2E2 | 2.1E2 | 1.0E-9 | 1.0E-9 | 4.5E3 | 6.2E2 | 904 | 9 | 337 | 9 | 0.72 |
| Mw | pg/ml | 3.8E1 | 1.7E2 | 5.2E2 | 1.4E3 | 3.3E3 | 2.8E3 | 1.0E-9 | 1.0E-9 | 6.2E4 | 8.5E3 | 904 | 9 | 337 | 9 | 0.73 |
| Mx | pg/ml | 1.0E-9 | 1.0E-9 | 2.8E-1 | 4.0E-1 | 1.5E0 | 9.9E-1 | 1.0E-9 | 1.0E-9 | 3.2E1 | 3.0E0 | 904 | 9 | 337 | 9 | 0.63 |
| My | pg/ml | 1.0E-9 | 1.7E1 | 4.7E2 | 4.3E2 | 3.0E3 | 1.1E3 | 1.0E-9 | 1.0E-9 | 3.9E4 | 3.4E3 | 904 | 9 | 337 | 9 | 0.62 |
| Mz | pg/ml | 1.1E1 | 6.3E1 | 3.0E1 | 1.3E2 | 9.9E1 | 1.3E2 | 1.0E-9 | 1.0E0 | 1.9E3 | 3.6E2 | 904 | 9 | 337 | 9 | 0.83 |
| Na | pg/ml | 1.0E-9 | 1.0E-9 | 7.3E-1 | 1.3E0 | 3.0E0 | 3.5E0 | 1.0E-9 | 1.0E-9 | 4.2E1 | 1.1E1 | 904 | 9 | 337 | 9 | 0.52 |
| Nb | pg/ml | 2.0E0 | 2.6E0 | 3.9E0 | 2.9E1 | 1.2E1 | 7.9E1 | 1.0E-9 | 1.0E-9 | 2.3E2 | 2.4E2 | 904 | 9 | 337 | 9 | 0.57 |
| Nc | pg/ml | 3.4E2 | 1.5E2 | 5.6E2 | 3.5E2 | 7.3E2 | 6.7E2 | 1.0E-9 | 1.0E-9 | 9.2E3 | 2.1E3 | 904 | 9 | 337 | 9 | 0.39 |
| Nd | pg/ml | 2.9E1 | 4.1E1 | 2.9E1 | 6.2E1 | 8.3E1 | 5.1E1 | 1.0E-9 | 6.8E0 | 2.1E3 | 1.4E2 | 904 | 9 | 337 | 9 | 0.71 |
| Ne | pg/ml | 4.4E2 | 3.6E2 | 5.8E2 | 4.0E2 | 5.7E2 | 3.1E2 | 1.0E-9 | 1.8E1 | 7.0E3 | 1.1E3 | 904 | 9 | 337 | 9 | 0.43 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nf | pg/ml | 1.0E-9 | 1.0E-9 | 3.0E0 | 9.2E0 | 1.1E1 | 2.7E1 | 1.0E-9 | 1.0E-9 | 1.6E2 | 8.2E1 | 904 | 9 | 337 | 9 | 0.49 |
| Ng | pg/ml | 1.9E1 | 4.4E0 | 1.3E2 | 3.5E1 | 2.5E2 | 7.5E1 | 1.0E-9 | 1.0E-9 | 2.3E3 | 2.3E2 | 904 | 9 | 337 | 9 | 0.38 |
| Nh | pg/ml | 6.9E1 | 3.5E1 | 9.0E1 | 7.0E1 | 8.2E1 | 6.5E1 | 1.0E-9 | 2.2E0 | 5.6E2 | 1.6E2 | 904 | 9 | 337 | 9 | 0.43 |
| Ni | pg/ml | 1.0E-9 | 5.3E1 | 7.3E1 | 8.7E1 | 1.2E2 | 1.1E2 | 1.0E-9 | 1.0E-9 | 1.1E3 | 3.4E2 | 904 | 9 | 337 | 9 | 0.61 |
| Nj | pg/ml | 7.3E0 | 9.0E0 | 1.1E1 | 1.4E1 | 1.2E1 | 1.8E1 | 1.0E-9 | 9.4E-1 | 1.1E2 | 5.7E1 | 904 | 9 | 337 | 9 | 0.55 |
| Nk | pg/ml | 1.7E1 | 2.7E1 | 3.3E1 | 2.7E1 | 4.0E1 | 2.1E1 | 1.0E-9 | 1.5E0 | 2.0E2 | 6.6E1 | 904 | 9 | 337 | 9 | 0.55 |
| Nl | pg/ml | 4.6E1 | 3.3E1 | 6.1E1 | 4.1E1 | 6.8E1 | 3.8E1 | 1.0E-9 | 1.0E-9 | 1.1E3 | 1.1E2 | 904 | 9 | 337 | 9 | 0.43 |
| Hq | pg/ml | 1.1E0 | 1.7E0 | 1.0E2 | 3.5E1 | 1.6E3 | 9.8E1 | 1.0E-9 | 1.0E-9 | 3.4E4 | 3.0E2 | 900 | 9 | 336 | 9 | 0.54 |
| Hr | pg/ml | 1.2E2 | 4.5E1 | 7.9E2 | 1.2E3 | 1.6E3 | 3.6E3 | 1.0E-9 | 2.1E1 | 1.7E4 | 1.1E4 | 900 | 9 | 336 | 9 | 0.28 |
| Hu | pg/ml | 5.6E0 | 6.1E0 | 3.0E3 | 3.5E2 | 2.6E4 | 8.0E2 | 1.0E-9 | 1.0E-9 | 6.3E5 | 2.4E3 | 900 | 9 | 336 | 9 | 0.50 |
| Hv | pg/ml | 1.4E0 | 2.3E0 | 4.1E0 | 8.8E0 | 3.2E1 | 1.9E1 | 1.0E-9 | 2.1E-1 | 8.9E2 | 5.9E1 | 900 | 9 | 336 | 9 | 0.67 |
| Hw | pg/ml | 6.5E0 | 2.7E0 | 2.9E1 | 3.9E2 | 3.2E2 | 1.1E3 | 1.0E-9 | 3.2E-1 | 9.4E3 | 3.4E3 | 900 | 9 | 336 | 9 | 0.40 |
| Hx | pg/ml | 8.8E0 | 2.7E1 | 3.9E1 | 2.5E2 | 3.2E2 | 6.4E2 | 1.0E-9 | 7.9E0 | 9.3E3 | 2.0E3 | 900 | 9 | 336 | 9 | 0.76 |
| Ih | ng/ml | 7.2E1 | 2.6E2 | 2.4E2 | 4.0E2 | 4.3E2 | 4.8E2 | 1.0E-9 | 1.0E-9 | 3.6E3 | 1.2E3 | 904 | 9 | 336 | 9 | 0.58 |
| Ii | ng/ml | 9.4E1 | 9.1E1 | 2.5E2 | 1.2E3 | 6.8E2 | 3.0E3 | 1.0E-9 | 7.0E0 | 1.0E4 | 9.2E3 | 903 | 9 | 336 | 9 | 0.54 |
| Ij | ng/ml | 7.7E1 | 1.1E2 | 2.0E2 | 8.9E2 | 9.9E2 | 2.2E3 | 1.0E-9 | 3.5E1 | 2.4E4 | 6.4E3 | 891 | 8 | 334 | 8 | 0.60 |
| Ik | ng/ml | 1.3E1 | 2.8E1 | 8.1E2 | 2.5E2 | 8.2E3 | 4.0E2 | 5.9E-1 | 1.5E0 | 1.2E5 | 1.1E3 | 898 | 8 | 334 | 8 | 0.53 |
| Il | ng/ml | 3.4E2 | 1.9E2 | 1.3E3 | 1.9E3 | 2.8E3 | 4.2E3 | 1.0E-9 | 1.6E1 | 1.3E4 | 1.2E4 | 881 | 8 | 334 | 8 | 0.41 |
| Im | ng/ml | 2.1E2 | 5.5E2 | 3.9E2 | 5.6E2 | 6.0E2 | 3.4E2 | 1.3E1 | 1.8E2 | 6.2E3 | 1.2E3 | 897 | 8 | 335 | 8 | 0.75 |
| In | ng/ml | 3.7E0 | 7.8E-1 | 2.7E1 | 8.6E0 | 2.1E2 | 2.2E1 | 1.0E-9 | 3.1E-2 | 4.5E3 | 6.8E1 | 904 | 9 | 336 | 9 | 0.31 |
| Io | ng/ml | 8.1E3 | 8.4E3 | 2.4E4 | 7.8E4 | 1.5E5 | 1.9E5 | 1.0E-9 | 5.9E3 | 4.0E6 | 5.5E5 | 896 | 8 | 336 | 8 | 0.59 |
| Ip | ng/ml | 9.7E0 | 3.0E1 | 1.9E1 | 3.8E1 | 2.4E1 | 4.7E1 | 1.0E-9 | 5.6E-3 | 2.6E2 | 1.4E2 | 896 | 8 | 336 | 8 | 0.57 |
| Iq | ug/ml | 1.0E-1 | 2.6E-1 | 3.2E1 | 9.5E1 | 6.4E2 | 2.7E2 | 1.0E-9 | 1.0E-9 | 1.4E4 | 7.5E2 | 896 | 8 | 336 | 8 | 0.55 |
| Ir | ug/ml | 3.5E-1 | 2.8E0 | 3.9E0 | 1.9E1 | 2.8E1 | 3.9E1 | 1.0E-9 | 1.9E-1 | 5.1E2 | 1.1E2 | 895 | 8 | 336 | 8 | 0.82 |
| Is | ng/ml | 1.5E0 | 1.6E1 | 6.6E0 | 5.3E1 | 2.3E1 | 8.0E1 | 1.0E-9 | 3.1E0 | 5.5E2 | 2.3E2 | 896 | 8 | 336 | 8 | 0.88 |
| It | ng/ml | 2.0E0 | 1.8E0 | 2.4E1 | 7.9E1 | 1.4E2 | 2.1E2 | 1.0E-9 | 1.0E-9 | 2.8E3 | 5.9E2 | 896 | 8 | 336 | 8 | 0.55 |
| Iu | ng/ml | 2.2E2 | 2.6E2 | 1.4E3 | 3.6E3 | 4.2E3 | 8.4E3 | 1.0E-9 | 1.0E-9 | 2.9E4 | 2.4E4 | 896 | 8 | 336 | 8 | 0.48 |
| Iv | ng/ml | 1.3E1 | 4.1E1 | 6.1E1 | 8.5E2 | 5.5E2 | 2.3E3 | 1.0E-9 | 3.3E0 | 1.6E4 | 6.4E3 | 895 | 8 | 336 | 8 | 0.70 |
| Pz | ng/ml | 3.9E3 | 4.7E3 | 8.3E3 | 6.4E3 | 3.8E4 | 4.8E3 | 1.3E1 | 2.6E2 | 1.0E6 | 1.4E4 | 896 | 9 | 334 | 9 | 0.59 |
| Qa | ng/ml | 3.5E3 | 1.4E4 | 6.5E3 | 1.3E4 | 1.0E4 | 7.4E3 | 1.2E1 | 2.5E3 | 2.2E5 | 2.5E4 | 896 | 9 | 334 | 9 | 0.78 |
| Qb | ng/ml | 9.7E1 | 2.9E2 | 2.1E2 | 3.8E2 | 4.9E2 | 2.8E2 | 7.9E-1 | 8.6E1 | 8.3E3 | 9.1E2 | 896 | 9 | 334 | 9 | 0.77 |
| Qc | ng/ml | 2.3E2 | 4.1E2 | 6.3E2 | 6.3E2 | 5.6E3 | 7.4E2 | 1.0E-9 | 3.8E1 | 1.7E5 | 2.4E3 | 896 | 9 | 334 | 9 | 0.62 |
| Qd | ng/ml | 9.2E3 | 1.1E4 | 1.9E4 | 4.2E4 | 7.3E4 | 6.0E4 | 1.5E2 | 7.9E3 | 2.0E6 | 1.7E5 | 896 | 9 | 334 | 9 | 0.66 |
| Qe | ng/ml | 9.2E2 | 2.3E3 | 1.9E3 | 4.4E3 | 4.7E3 | 4.2E3 | 1.0E-9 | 8.7E2 | 9.7E4 | 1.4E4 | 896 | 9 | 334 | 9 | 0.80 |
| Jg | ng/ml | 5.0E2 | 1.6E3 | 8.3E2 | 2.3E3 | 1.0E3 | 1.8E3 | 1.0E-9 | 2.2E2 | 1.0E4 | 5.4E3 | 900 | 9 | 336 | 9 | 0.79 |
| Jh | ng/ml | 3.0E0 | 2.4E1 | 2.9E1 | 5.6E1 | 1.2E2 | 6.9E1 | 1.0E-9 | 1.0E-9 | 1.6E3 | 2.1E2 | 900 | 9 | 336 | 9 | 0.71 |
| Ji | ng/ml | 5.3E1 | 1.9E2 | 7.9E1 | 2.4E2 | 9.0E1 | 1.7E2 | 1.0E-9 | 5.5E1 | 1.3E3 | 5.9E2 | 900 | 9 | 336 | 9 | 0.86 |
| Jj | ng/ml | 6.1E2 | 8.7E1 | 1.6E3 | 1.8E2 | 1.2E4 | 2.7E2 | 1.5E0 | 1.7E1 | 3.4E5 | 8.7E2 | 900 | 9 | 336 | 9 | 0.14 |
| Jk | ng/ml | 3.0E0 | 4.5E0 | 2.1E1 | 3.6E1 | 4.7E1 | 5.7E1 | 1.0E-9 | 2.2E0 | 3.9E2 | 1.7E2 | 900 | 9 | 336 | 9 | 0.67 |
| Jl | ng/ml | 4.1E-1 | 2.0E1 | 2.6E0 | 2.8E1 | 1.9E1 | 4.9E1 | 7.6E-4 | 9.5E-1 | 5.4E2 | 1.6E2 | 900 | 9 | 336 | 9 | 0.90 |
| Jm | ng/ml | 1.8E1 | 1.5E1 | 5.8E1 | 1.9E1 | 1.3E2 | 1.8E1 | 1.0E-9 | 6.7E-1 | 2.1E3 | 5.1E1 | 900 | 9 | 336 | 9 | 0.41 |
| Jn | pg/ml | 4.0E-1 | 2.0E0 | 3.7E0 | 3.6E0 | 3.8E1 | 3.2E0 | 1.0E-9 | 2.8E-2 | 7.3E2 | 8.9E0 | 899 | 9 | 336 | 9 | 0.80 |
| Jo | pg/ml | 3.6E3 | 1.5E3 | 4.9E3 | 5.7E3 | 5.0E3 | 1.2E4 | 2.0E1 | 7.5E1 | 1.0E5 | 3.8E4 | 900 | 9 | 336 | 9 | 0.29 |
| Jp | pg/ml | 7.0E4 | 1.1E5 | 7.3E4 | 1.3E5 | 3.8E4 | 4.2E4 | 5.8E2 | 8.0E4 | 3.8E5 | 2.2E5 | 900 | 9 | 336 | 9 | 0.86 |
| Jq | pg/ml | 9.6E1 | 6.1E1 | 1.6E2 | 6.2E2 | 3.6E2 | 1.3E3 | 1.0E0 | 3.1E1 | 8.7E3 | 4.1E3 | 900 | 9 | 336 | 9 | 0.53 |
| Jr | pg/ml | 5.3E0 | 4.0E1 | 4.4E1 | 6.2E1 | 4.7E2 | 6.9E1 | 1.0E-9 | 7.7E-1 | 1.1E4 | 1.9E2 | 900 | 9 | 336 | 9 | 0.82 |
| Js | pg/ml | 1.3E1 | 1.9E1 | 5.3E1 | 2.9E1 | 3.8E2 | 2.8E1 | 1.0E-9 | 3.5E0 | 1.0E4 | 9.4E1 | 900 | 9 | 336 | 9 | 0.62 |
| Jt | pg/ml | 2.6E3 | 2.3E3 | 3.2E3 | 5.7E3 | 2.8E3 | 1.0E4 | 2.2E1 | 6.4E2 | 5.2E4 | 3.3E4 | 900 | 9 | 336 | 9 | 0.47 |
| Lh | pg/ml | 1.3E4 | 3.4E4 | 2.2E4 | 9.2E4 | 3.2E4 | 1.3E5 | 1.0E-9 | 5.5E3 | 4.8E5 | 4.1E5 | 900 | 9 | 337 | 9 | 0.79 |
| Li | pg/ml | 3.3E3 | 1.1E4 | 1.7E4 | 8.5E4 | 6.2E4 | 1.9E5 | 1.0E-9 | 3.6E1 | 1.3E6 | 5.9E5 | 900 | 9 | 337 | 9 | 0.64 |
| Lj | pg/ml | 2.8E3 | 8.3E3 | 2.3E4 | 5.9E4 | 6.6E4 | 1.1E5 | 1.0E-9 | 1.0E2 | 5.2E5 | 3.5E5 | 900 | 9 | 337 | 9 | 0.64 |
| Nv | pg/ml | 4.0E3 | 1.4E4 | 1.1E4 | 2.7E4 | 4.3E4 | 3.1E4 | 1.0E-9 | 2.6E3 | 1.1E6 | 8.4E4 | 906 | 9 | 337 | 9 | 0.82 |
| Nw | pg/ml | 8.9E3 | 3.5E4 | 1.3E4 | 5.7E4 | 1.7E4 | 6.0E4 | 8.6E1 | 9.1E3 | 2.1E5 | 1.7E5 | 906 | 9 | 337 | 9 | 0.87 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nx | pg/ml | 2.2E2 | 4.7E2 | 4.1E2 | 7.4E2 | 6.5E2 | 8.1E2 | 1.0E-9 | 1.0E-9 | 7.4E3 | 2.2E3 | 906 | 9 | 337 | 9 | 0.65 |
| Ny | pg/ml | 6.4E0 | 2.3E1 | 5.7E1 | 1.3E2 | 8.5E2 | 2.2E2 | 1.0E-9 | 1.0E-9 | 2.5E4 | 6.2E2 | 906 | 9 | 337 | 9 | 0.72 |
| Oe | pg/ml | 6.8E1 | 1.0E-9 | 2.9E2 | 3.7E2 | 7.5E2 | 5.5E2 | 1.0E-9 | 1.0E-9 | 1.9E4 | 1.6E3 | 897 | 9 | 336 | 9 | 0.49 |
| Of | pg/ml | 1.7E2 | 9.1E1 | 6.1E3 | 8.5E3 | 2.8E4 | 2.4E4 | 1.0E-9 | 7.2E0 | 6.2E5 | 7.4E4 | 905 | 9 | 337 | 9 | 0.47 |
| Og | pg/ml | 8.2E-2 | 5.6E-2 | 4.8E-1 | 9.8E-2 | 1.6E0 | 1.1E-1 | 1.0E-9 | 1.0E-9 | 2.0E1 | 2.7E-1 | 905 | 9 | 337 | 9 | 0.41 |
| Oh | pg/ml | 2.7E0 | 1.0E1 | 2.0E1 | 4.8E1 | 1.5E2 | 7.8E1 | 1.0E-9 | 9.0E-2 | 3.5E3 | 2.2E2 | 905 | 9 | 337 | 9 | 0.74 |
| Oi | pg/ml | 2.6E0 | 5.1E0 | 6.3E0 | 4.3E0 | 9.8E0 | 4.1E0 | 1.0E-9 | 1.0E-9 | 8.6E1 | 1.1E1 | 905 | 9 | 337 | 9 | 0.52 |
| Ok | pg/ml | 3.9E2 | 9.8E2 | 5.6E2 | 1.9E3 | 6.0E2 | 3.0E3 | 1.3E1 | 4.5E2 | 7.8E3 | 9.8E3 | 905 | 9 | 337 | 9 | 0.81 |
| Om | pg/ml | 4.0E2 | 8.0E2 | 8.9E2 | 5.2E3 | 2.7E3 | 7.6E3 | 1.0E-9 | 2.0E2 | 5.1E4 | 2.0E4 | 905 | 9 | 337 | 9 | 0.75 |
| On | pg/ml | 1.8E2 | 5.3E2 | 3.0E2 | 2.4E3 | 4.2E2 | 4.8E3 | 1.0E-9 | 1.5E2 | 4.5E3 | 1.5E4 | 905 | 9 | 337 | 9 | 0.84 |
| Oy | pg/ml | 4.9E-1 | 3.3E-1 | 5.7E0 | 3.0E1 | 2.9E1 | 9.0E1 | 1.0E-9 | 1.0E-9 | 4.0E2 | 2.7E2 | 904 | 9 | 336 | 9 | 0.42 |
| Oz | pg/ml | 3.1E-3 | 1.1E-1 | 3.1E-1 | 2.1E-1 | 1.3E0 | 2.8E-1 | 1.0E-9 | 1.0E-9 | 2.9E1 | 6.9E-1 | 904 | 9 | 336 | 9 | 0.52 |
| Pa | pg/ml | 3.9E-1 | 1.5E0 | 1.6E0 | 3.6E1 | 6.1E0 | 9.6E1 | 1.0E-9 | 1.7E-1 | 1.0E2 | 2.9E2 | 904 | 9 | 336 | 9 | 0.79 |
| Pb | pg/ml | 1.0E-9 | 6.9E-2 | 8.0E-1 | 2.0E1 | 1.6E1 | 6.0E1 | 1.0E-9 | 1.0E-9 | 4.9E2 | 1.8E2 | 904 | 9 | 336 | 9 | 0.59 |
| Pc | pg/ml | 4.4E-2 | 1.0E0 | 3.6E-1 | 2.4E0 | 8.8E-1 | 3.9E0 | 1.0E-9 | 1.0E-9 | 1.6E1 | 1.1E1 | 904 | 9 | 336 | 9 | 0.68 |
| Pd | pg/ml | 1.9E0 | 2.5E0 | 5.1E0 | 4.0E0 | 2.9E1 | 5.4E0 | 1.0E-9 | 1.0E-9 | 8.4E2 | 1.8E1 | 904 | 9 | 336 | 9 | 0.56 |
| Pe | pg/ml | 2.2E1 | 9.4E1 | 1.2E2 | 1.7E3 | 4.4E2 | 4.7E3 | 1.0E-9 | 1.4E1 | 6.7E3 | 1.4E4 | 904 | 9 | 336 | 9 | 0.75 |
| Pf | pg/ml | 1.6E0 | 1.3E1 | 1.1E1 | 3.6E1 | 6.0E1 | 7.4E1 | 1.0E-9 | 2.8E-1 | 1.5E3 | 2.3E2 | 904 | 9 | 336 | 9 | 0.73 |
| Pg | pg/ml | 3.4E0 | 8.7E0 | 4.3E1 | 2.3E2 | 3.4E2 | 6.2E2 | 1.0E-9 | 1.7E0 | 7.7E3 | 1.9E3 | 904 | 9 | 336 | 9 | 0.68 |
| aA | mg/dL | 8.0E-1 | 1.8E0 | 9.4E-1 | 2.2E0 | 4.9E-1 | 1.4E0 | 2.0E-1 | 6.0E-1 | 4.2E0 | 5.4E0 | 2667 | 20 | 507 | 20 | 0.85 |

Figure 11.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 6.5E1 | 9.0E1 | 7.6E1 | 1.1E2 | 5.2E1 | 8.7E1 | 2.0E0 | 7.0E0 | 4.0E2 | 4.0E2 | 1392 | 32 | 230 | 32 | 0.63 |
| Ad | ug/mL | 3.4E-2 | 6.4E-2 | 6.5E-2 | 9.7E-2 | 8.4E-2 | 8.3E-2 | 6.8E-4 | 2.7E-4 | 5.4E-1 | 3.5E-1 | 357 | 25 | 135 | 25 | 0.68 |
| Af | ng/mL | 1.0E0 | 4.5E-1 | 1.4E1 | 1.3E1 | 6.0E1 | 4.3E1 | 1.7E-3 | 1.7E-3 | 5.3E2 | 2.2E2 | 357 | 25 | 135 | 25 | 0.46 |
| Aj | ug/mL | 1.8E0 | 3.3E0 | 2.7E0 | 2.9E0 | 2.5E0 | 2.3E0 | 1.5E-3 | 1.8E-3 | 6.1E0 | 6.1E0 | 357 | 25 | 135 | 25 | 0.51 |
| Al | mg/mL | 8.7E-5 | 9.0E-5 | 2.5E-4 | 1.9E-4 | 4.1E-4 | 3.7E-4 | 2.5E-6 | 8.0E-6 | 1.9E-3 | 1.9E-3 | 357 | 25 | 135 | 25 | 0.52 |
| An | U/mL | 4.9E1 | 6.9E1 | 1.6E2 | 2.0E2 | 4.6E2 | 2.7E2 | 9.8E-4 | 8.6E-1 | 5.5E3 | 9.8E2 | 357 | 25 | 135 | 25 | 0.59 |
| Ao | pg/mL | 8.5E1 | 1.1E2 | 6.0E2 | 1.3E2 | 3.9E3 | 1.2E2 | 2.8E0 | 6.1E0 | 3.9E4 | 6.1E2 | 357 | 25 | 135 | 25 | 0.55 |
| Ap | ng/mL | 3.1E1 | 5.0E1 | 4.2E1 | 6.2E1 | 4.3E1 | 4.5E1 | 8.4E-5 | 2.1E0 | 2.9E2 | 1.8E2 | 357 | 25 | 135 | 25 | 0.67 |
| Ar | ng/mL | 8.5E-1 | 1.5E0 | 1.5E1 | 4.0E0 | 2.2E2 | 5.1E0 | 3.4E-3 | 5.7E-2 | 4.1E3 | 2.0E1 | 357 | 25 | 135 | 25 | 0.62 |
| As | ng/mL | 9.5E-3 | 1.0E-2 | 1.3E-2 | 2.0E-2 | 1.8E-2 | 3.2E-2 | 1.7E-3 | 1.7E-3 | 1.3E-1 | 1.2E-1 | 357 | 25 | 135 | 25 | 0.51 |
| Aw | pg/mL | 1.6E1 | 1.7E1 | 1.6E1 | 1.7E1 | 5.9E0 | 6.7E0 | 2.9E-2 | 2.9E-2 | 4.8E1 | 3.8E1 | 357 | 25 | 135 | 25 | 0.57 |
| Ax | ng/mL | 2.2E0 | 4.5E0 | 1.4E1 | 2.1E1 | 5.8E1 | 4.4E1 | 1.3E-2 | 3.9E-2 | 7.7E2 | 2.0E2 | 357 | 25 | 135 | 25 | 0.57 |
| Ba | ng/mL | 6.2E1 | 1.7E2 | 4.2E2 | 4.9E2 | 1.2E3 | 6.8E2 | 3.7E-1 | 6.9E-1 | 8.1E3 | 3.0E3 | 357 | 25 | 135 | 25 | 0.63 |
| Bb | ng/mL | 2.8E0 | 5.2E0 | 6.1E0 | 6.1E0 | 1.5E1 | 4.2E0 | 4.1E-3 | 5.5E-1 | 2.5E2 | 1.6E1 | 357 | 25 | 135 | 25 | 0.64 |
| Bc | ng/mL | 3.4E1 | 7.2E1 | 1.0E2 | 1.5E2 | 1.9E2 | 2.6E2 | 1.1E-1 | 2.4E0 | 1.2E3 | 1.0E3 | 357 | 25 | 135 | 25 | 0.64 |
| Bg | ng/mL | 7.7E-2 | 2.9E-1 | 4.0E0 | 6.7E-1 | 1.9E1 | 1.1E0 | 5.3E-4 | 5.3E-4 | 2.5E2 | 4.8E0 | 357 | 25 | 135 | 25 | 0.60 |
| Bn | ng/mL | 5.6E-2 | 5.6E-2 | 1.1E0 | 1.4E0 | 1.9E0 | 2.4E0 | 5.6E-2 | 5.6E-2 | 9.7E0 | 7.6E0 | 357 | 25 | 135 | 25 | 0.51 |
| Bo | ng/mL | 1.2E1 | 1.7E1 | 1.4E1 | 1.7E1 | 2.0E1 | 1.2E1 | 1.6E-2 | 1.6E-2 | 2.8E2 | 3.6E1 | 357 | 25 | 135 | 25 | 0.59 |
| Ch | ulU/mL | 1.2E0 | 8.5E-1 | 2.1E1 | 5.3E0 | 1.1E2 | 1.6E1 | 3.4E-3 | 1.0E-1 | 1.8E3 | 8.0E1 | 357 | 25 | 135 | 25 | 0.44 |
| Co | pg/mL | 3.8E1 | 5.5E1 | 1.8E2 | 7.6E1 | 1.1E3 | 7.7E1 | 1.5E-1 | 9.1E0 | 1.7E4 | 3.9E2 | 357 | 25 | 135 | 25 | 0.59 |
| Cp | ng/mL | 2.2E1 | 2.3E1 | 2.8E1 | 2.7E1 | 3.3E1 | 2.0E1 | 6.0E-1 | 6.0E-1 | 3.7E2 | 9.9E1 | 357 | 25 | 135 | 25 | 0.55 |
| Cq | ng/mL | 2.8E-2 | 3.4E-2 | 1.5E-1 | 6.5E-2 | 9.5E-1 | 1.1E-1 | 8.0E-4 | 8.0E-4 | 1.7E1 | 4.9E-1 | 357 | 25 | 135 | 25 | 0.53 |
| Cs | ng/mL | 6.6E1 | 1.6E2 | 3.0E2 | 4.5E2 | 8.6E2 | 1.1E3 | 2.7E-2 | 1.5E0 | 1.1E4 | 5.3E3 | 357 | 25 | 135 | 25 | 0.57 |
| Ct | ng/mL | 8.9E-1 | 3.3E-1 | 4.0E1 | 1.7E1 | 1.1E2 | 3.9E1 | 1.1E-4 | 1.5E-2 | 6.2E2 | 1.6E2 | 357 | 25 | 135 | 25 | 0.43 |
| Cu | ng/mL | 2.4E-1 | 3.8E-1 | 4.0E-1 | 4.0E-1 | 7.0E-1 | 2.4E-1 | 9.6E-3 | 1.8E-2 | 9.2E0 | 9.9E-1 | 357 | 25 | 135 | 25 | 0.63 |
| Cv | ng/mL | 4.1E0 | 1.2E1 | 1.8E1 | 3.8E1 | 4.9E1 | 9.3E1 | 1.4E-4 | 1.8E-2 | 5.3E2 | 4.7E2 | 357 | 25 | 135 | 25 | 0.62 |
| Cw | mIU/mL | 3.0E-2 | 4.0E-2 | 3.8E-2 | 4.0E-2 | 3.2E-2 | 2.9E-2 | 8.9E-4 | 1.5E-4 | 2.4E-1 | 1.3E-1 | 357 | 25 | 135 | 25 | 0.54 |
| Cx | ng/mL | 1.5E-1 | 4.6E-2 | 5.1E1 | 6.4E1 | 9.9E1 | 1.3E2 | 9.3E-5 | 9.3E-5 | 3.9E2 | 3.9E2 | 357 | 25 | 135 | 25 | 0.50 |
| Db | ug/mL | 7.2E0 | 6.8E0 | 8.6E0 | 1.4E1 | 7.3E0 | 2.8E1 | 4.5E-1 | 8.5E-1 | 5.9E1 | 1.4E2 | 357 | 25 | 135 | 25 | 0.52 |
| Dc | nmol/L | 1.9E-2 | 1.7E-2 | 5.7E-2 | 2.5E-2 | 1.4E-1 | 2.4E-2 | 5.2E-6 | 3.0E-4 | 1.6E0 | 9.9E-2 | 357 | 25 | 135 | 25 | 0.47 |
| Dd | ug/mL | 6.9E-2 | 4.2E-2 | 1.8E-1 | 1.2E-1 | 2.7E-1 | 2.0E-1 | 1.9E-4 | 4.8E-4 | 1.9E0 | 8.9E-1 | 357 | 25 | 135 | 25 | 0.41 |
| De | ng/mL | 3.4E-3 | 3.4E-3 | 7.8E-2 | 5.8E-2 | 1.4E-1 | 1.1E-1 | 3.4E-3 | 3.4E-3 | 9.2E-1 | 5.0E-1 | 357 | 25 | 135 | 25 | 0.47 |
| Dg | ng/mL | 2.9E1 | 6.6E1 | 4.2E1 | 7.0E1 | 3.8E1 | 4.6E1 | 1.0E-1 | 2.1E0 | 1.9E2 | 1.9E2 | 357 | 25 | 135 | 25 | 0.69 |
| Di | pg/mL | 2.0E0 | 1.3E0 | 2.2E0 | 1.5E0 | 2.0E0 | 1.4E0 | 1.8E-1 | 1.8E-1 | 1.3E1 | 4.0E0 | 357 | 25 | 135 | 25 | 0.39 |
| Dk | uIU/mL | 1.7E-2 | 1.9E-2 | 9.6E-2 | 2.5E-2 | 5.7E-1 | 2.1E-2 | 1.1E-4 | 1.1E-4 | 8.9E0 | 9.0E-2 | 357 | 25 | 135 | 25 | 0.50 |
| Dl | ng/mL | 2.3E2 | 4.3E2 | 3.1E2 | 5.1E2 | 2.8E2 | 4.0E2 | 1.7E0 | 6.4E0 | 1.5E3 | 1.3E3 | 357 | 25 | 135 | 25 | 0.65 |
| Do | ng/ml | 4.7E-1 | 6.3E-1 | 1.1E0 | 1.1E0 | 2.7E0 | 1.6E0 | 3.6E-2 | 3.6E-2 | 1.9E1 | 4.6E0 | 62 | 7 | 44 | 7 | 0.57 |
| Dp | ng/ml | 2.3E0 | 2.4E0 | 5.1E0 | 4.7E0 | 7.9E0 | 6.1E0 | 3.7E-3 | 3.7E-3 | 4.6E1 | 2.2E1 | 217 | 22 | 135 | 22 | 0.50 |
| Dr | pg/ml | 2.1E1 | 1.7E1 | 4.7E1 | 1.9E1 | 7.1E1 | 2.1E1 | 7.5E-1 | 7.5E-1 | 5.2E2 | 6.0E1 | 125 | 8 | 71 | 8 | 0.38 |
| Dq | Absorbance | 1.7E-3 | 1.7E-3 | 3.2E-2 | 1.7E-3 | 1.4E-1 | 0.0E0 | 1.7E-3 | 1.7E-3 | 8.3E-1 | 1.7E-3 | 60 | 7 | 43 | 7 | 0.39 |
| Dv | pg/ml | 1.1E0 | 7.6E-1 | 1.2E0 | 9.0E-1 | 1.2E0 | 7.4E-1 | 2.2E-2 | 2.2E-2 | 4.5E0 | 2.3E0 | 42 | 7 | 25 | 7 | 0.46 |
| Ef | ng/ml | 1.5E-1 | 1.8E-1 | 8.1E-1 | 7.9E-1 | 1.6E0 | 1.8E0 | 5.7E-4 | 1.3E-3 | 9.5E0 | 8.4E0 | 261 | 23 | 134 | 23 | 0.53 |
| Wm | % | 4.9E-1 | 7.6E-1 | 2.3E1 | 1.3E2 | 1.6E2 | 3.4E2 | 5.4E-2 | 8.5E-2 | 2.4E3 | 1.3E3 | 283 | 25 | 151 | 25 | 0.57 |
| Ed | pg/ml | 5.2E-1 | 5.2E-1 | 6.3E1 | 4.3E1 | 5.0E2 | 8.4E1 | 5.2E-1 | 5.2E-1 | 7.3E3 | 3.5E2 | 217 | 22 | 134 | 22 | 0.50 |
| Tj | pg/mL | 3.7E-1 | 3.7E-1 | 6.8E1 | 7.2E0 | 3.3E2 | 1.2E1 | 3.6E-1 | 3.7E-1 | 3.5E3 | 4.0E1 | 258 | 23 | 136 | 23 | 0.50 |
| Po | pg/ml | 6.7E-1 | 1.6E0 | 9.1E0 | 1.6E1 | 2.5E1 | 4.1E1 | 8.0E-3 | 2.6E-2 | 2.7E2 | 2.2E2 | 580 | 35 | 207 | 35 | 0.55 |
| Em | ng/ml | 9.2E-3 | 2.9E-3 | 6.7E-2 | 1.2E-1 | 1.2E-1 | 2.0E-1 | 1.9E-16 | 2.9E-3 | 6.0E-1 | 4.7E-1 | 149 | 9 | 71 | 9 | 0.52 |
| Et | ng/ml | 1.3E3 | 3.0E3 | 1.6E3 | 2.7E3 | 1.1E3 | 1.4E3 | 7.7E1 | 9.1E1 | 5.0E3 | 5.0E3 | 579 | 35 | 207 | 35 | 0.73 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fa | ng/ml | 4.0E1 | 7.5E1 | 1.3E2 | 8.2E1 | 6.0E2 | 7.2E1 | 3.4E-2 | 2.6E-1 | 8.0E3 | 2.9E2 | 212 | 22 | 133 | 22 | 0.60 |
| Ez | ng/ml | 5.0E0 | 3.9E0 | 2.0E1 | 1.1E1 | 5.9E1 | 1.4E1 | 1.3E-2 | 1.3E-2 | 7.1E2 | 4.6E1 | 217 | 22 | 135 | 22 | 0.50 |
| Fb | ng/ml | 2.5E1 | 2.3E1 | 2.3E1 | 2.3E1 | 1.1E1 | 1.1E1 | 6.6E-1 | 8.1E-1 | 5.7E1 | 4.1E1 | 213 | 22 | 133 | 22 | 0.47 |
| Ex | ng/ml | 7.8E-2 | 1.2E-1 | 2.5E-1 | 1.9E-1 | 7.6E-1 | 2.3E-1 | 3.5E-5 | 1.7E-4 | 8.9E0 | 9.2E-1 | 190 | 17 | 89 | 17 | 0.58 |
| Fn | ng/ml | 2.1E-1 | 2.1E-1 | 6.3E0 | 4.8E0 | 2.9E1 | 7.8E0 | 1.1E-14 | 1.1E-14 | 4.2E2 | 2.6E1 | 217 | 22 | 135 | 22 | 0.49 |
| Fp | ng/ml | 1.2E1 | 3.3E1 | 2.4E1 | 4.4E1 | 2.8E1 | 3.7E1 | 6.0E-3 | 2.8E-1 | 1.4E2 | 1.4E2 | 606 | 35 | 209 | 35 | 0.68 |
| Fr | ng/ml | 3.3E4 | 9.6E4 | 1.1E5 | 2.1E5 | 1.7E5 | 2.7E5 | 1.9E2 | 1.2E3 | 9.0E5 | 8.9E5 | 686 | 38 | 210 | 38 | 0.65 |
| Fw | pg/ml | 8.5E-1 | 2.9E0 | 7.0E1 | 4.4E1 | 5.4E2 | 1.4E2 | 1.1E-14 | 1.7E-14 | 6.9E3 | 6.3E2 | 259 | 23 | 135 | 23 | 0.53 |
| Fy | ng/ml | 3.5E1 | 4.3E1 | 5.5E1 | 5.7E1 | 5.7E1 | 5.0E1 | 1.2E-1 | 9.7E-1 | 3.3E2 | 2.1E2 | 216 | 22 | 134 | 22 | 0.55 |
| Gc | ng/ml | 9.2E1 | 1.2E2 | 1.4E2 | 1.6E2 | 1.7E2 | 1.4E2 | 6.4E0 | 2.2E1 | 1.2E3 | 4.4E2 | 134 | 8 | 72 | 8 | 0.55 |
| Gn | U/ml | 3.6E-1 | 5.6E-3 | 1.3E0 | 7.6E-2 | 3.3E0 | 1.8E-1 | 1.3E-3 | 1.3E-3 | 3.0E1 | 5.3E-1 | 119 | 8 | 69 | 8 | 0.14 |
| Gl | pg/ml | 7.8E3 | 6.2E3 | 1.1E4 | 8.3E3 | 9.4E3 | 6.2E3 | 9.1E1 | 1.0E3 | 3.3E4 | 2.3E4 | 252 | 23 | 135 | 23 | 0.46 |
| Gp | U/ml | 1.5E0 | 1.8E0 | 4.0E0 | 3.6E0 | 6.8E0 | 5.4E0 | 1.3E-3 | 1.5E-2 | 6.7E1 | 2.1E1 | 261 | 23 | 135 | 23 | 0.47 |
| Gt | ng/ml | 2.2E-3 | 2.2E-3 | 1.3E-1 | 2.2E-3 | 5.0E-1 | 0.0E0 | 2.2E-3 | 2.2E-3 | 3.3E0 | 2.2E-3 | 47 | 7 | 31 | 7 | 0.36 |
| Gw | ng/ml | 4.9E0 | 9.6E0 | 8.0E0 | 1.2E1 | 1.3E1 | 7.8E0 | 8.3E-1 | 3.6E0 | 9.3E1 | 2.2E1 | 62 | 7 | 44 | 7 | 0.72 |
| Gz | ug/ml | 1.4E0 | 1.2E0 | 9.7E0 | 5.4E0 | 4.2E1 | 6.4E0 | 2.9E-16 | 1.2E-1 | 4.8E2 | 2.1E1 | 143 | 16 | 86 | 16 | 0.51 |
| Ha | ng/ml | 2.7E0 | 2.2E0 | 9.9E0 | 3.9E0 | 2.1E1 | 6.5E0 | 1.7E-2 | 1.7E-2 | 1.3E2 | 3.0E1 | 215 | 22 | 134 | 22 | 0.42 |
| Nm | pg/ml | 1.6E4 | 3.3E4 | 3.2E4 | 5.9E4 | 8.8E4 | 8.2E4 | 1.0E-9 | 1.0E-9 | 1.6E6 | 4.4E5 | 583 | 35 | 209 | 35 | 0.63 |
| Nn | pg/ml | 1.5E2 | 3.2E2 | 2.2E3 | 4.5E3 | 9.4E3 | 1.3E4 | 1.0E-9 | 1.0E-9 | 1.0E5 | 6.9E4 | 583 | 35 | 209 | 35 | 0.59 |
| No | pg/ml | 1.5E1 | 2.8E1 | 3.7E1 | 1.0E2 | 1.3E2 | 2.9E2 | 1.0E-9 | 1.0E-9 | 2.5E3 | 1.4E3 | 583 | 35 | 209 | 35 | 0.63 |
| Nq | pg/ml | 2.0E0 | 8.9E-2 | 1.9E1 | 2.8E1 | 7.6E1 | 6.5E1 | 1.0E-9 | 1.0E-9 | 1.2E3 | 2.7E2 | 583 | 35 | 209 | 35 | 0.48 |
| Nr | pg/ml | 8.8E-1 | 4.6E0 | 3.3E1 | 2.6E2 | 2.1E2 | 1.4E3 | 1.0E-9 | 1.0E-9 | 4.1E3 | 8.5E3 | 583 | 35 | 209 | 35 | 0.63 |
| Ns | pg/ml | 1.0E-9 | 1.0E-9 | 1.1E1 | 8.0E-1 | 6.1E1 | 2.9E0 | 1.0E-9 | 1.0E-9 | 1.1E3 | 1.6E1 | 583 | 35 | 209 | 35 | 0.50 |
| Nt | pg/ml | 1.0E2 | 1.6E2 | 1.4E2 | 2.2E2 | 1.1E2 | 1.5E2 | 1.0E-9 | 2.6E1 | 1.5E3 | 6.8E2 | 583 | 35 | 209 | 35 | 0.70 |
| Nu | pg/ml | 2.3E1 | 1.0E2 | 5.7E1 | 1.1E2 | 9.5E1 | 1.1E2 | 1.0E-9 | 1.0E-9 | 8.9E2 | 5.8E2 | 583 | 35 | 209 | 35 | 0.69 |
| Lu | pg/ml | 1.0E4 | 1.0E4 | 1.7E4 | 2.0E4 | 4.7E4 | 4.0E4 | 3.5E2 | 1.6E3 | 7.5E5 | 2.3E5 | 585 | 35 | 209 | 35 | 0.50 |
| Lv | pg/ml | 1.0E-9 | 2.1E1 | 1.1E1 | 2.9E1 | 2.1E1 | 4.0E1 | 1.0E-9 | 1.0E-9 | 2.4E2 | 1.9E2 | 585 | 35 | 209 | 35 | 0.66 |
| Lw | pg/ml | 1.0E-9 | 1.0E-9 | 4.0E-1 | 1.1E0 | 4.3E0 | 4.5E0 | 1.0E-9 | 1.0E-9 | 8.0E1 | 2.1E1 | 585 | 35 | 209 | 35 | 0.53 |
| Lx | pg/ml | 1.0E-9 | 9.2E1 | 1.5E2 | 5.8E2 | 4.5E2 | 1.8E3 | 1.0E-9 | 1.0E-9 | 6.2E3 | 1.0E4 | 585 | 35 | 209 | 35 | 0.69 |
| Ly | pg/ml | 1.0E-9 | 1.5E1 | 1.0E1 | 2.2E1 | 2.0E1 | 2.5E1 | 1.0E-9 | 1.0E-9 | 1.6E2 | 8.7E1 | 585 | 35 | 209 | 35 | 0.67 |
| Lz | pg/ml | 1.0E-9 | 1.0E-9 | 4.9E0 | 6.0E0 | 3.6E1 | 3.5E1 | 1.0E-9 | 1.0E-9 | 6.0E2 | 2.1E2 | 585 | 35 | 209 | 35 | 0.49 |
| Ma | pg/ml | 3.1E2 | 3.0E2 | 1.3E3 | 1.3E3 | 3.8E3 | 2.4E3 | 1.0E-9 | 3.0E0 | 6.5E4 | 1.0E4 | 585 | 35 | 209 | 35 | 0.54 |
| Mb | pg/ml | 2.5E1 | 2.9E1 | 3.1E1 | 3.6E1 | 1.6E1 | 1.7E1 | 5.4E0 | 1.6E1 | 2.1E2 | 8.7E1 | 585 | 35 | 209 | 35 | 0.58 |
| Mc | pg/ml | 1.0E-9 | 1.0E-9 | 4.2E-2 | 1.0E-9 | 5.8E-1 | 0.0E0 | 1.0E-9 | 1.0E-9 | 1.3E1 | 1.0E-9 | 585 | 35 | 209 | 35 | 0.49 |
| Md | pg/ml | 1.0E-9 | 1.0E-9 | 4.8E-1 | 6.3E-3 | 3.3E0 | 3.8E-2 | 1.0E-9 | 1.0E-9 | 6.5E1 | 2.2E-1 | 585 | 35 | 209 | 35 | 0.47 |
| Me | pg/ml | 3.2E1 | 2.7E1 | 3.1E1 | 2.9E1 | 2.0E1 | 1.4E1 | 1.0E-9 | 3.2E0 | 3.2E2 | 7.9E1 | 585 | 35 | 209 | 35 | 0.45 |
| Mf | pg/ml | 1.0E-9 | 1.0E-9 | 5.2E-1 | 5.8E-1 | 3.1E0 | 1.6E0 | 1.0E-9 | 1.0E-9 | 5.6E1 | 8.4E0 | 585 | 35 | 209 | 35 | 0.56 |
| Mg | pg/ml | 2.0E0 | 6.5E0 | 7.7E0 | 9.7E0 | 1.3E1 | 1.1E1 | 1.0E-9 | 1.0E-9 | 9.2E1 | 4.0E1 | 585 | 35 | 209 | 35 | 0.60 |
| Mh | pg/ml | 1.0E-9 | 1.0E-9 | 1.5E0 | 2.0E0 | 1.1E1 | 6.9E0 | 1.0E-9 | 1.0E-9 | 1.6E2 | 3.8E1 | 585 | 35 | 209 | 35 | 0.50 |
| Mi | pg/ml | 1.0E-9 | 1.0E-9 | 5.9E-1 | 8.6E0 | 6.3E0 | 3.0E1 | 1.0E-9 | 1.0E-9 | 1.2E2 | 1.6E2 | 585 | 35 | 209 | 35 | 0.56 |
| Mj | pg/ml | 1.0E-9 | 1.0E-9 | 4.8E0 | 1.7E1 | 2.7E1 | 9.2E1 | 1.0E-9 | 1.0E-9 | 4.6E2 | 5.4E2 | 585 | 35 | 209 | 35 | 0.52 |
| Mk | pg/ml | 5.3E-1 | 8.4E-1 | 1.8E1 | 1.6E2 | 1.1E2 | 9.4E2 | 1.0E-9 | 1.0E-9 | 1.7E3 | 5.6E3 | 585 | 35 | 209 | 35 | 0.50 |
| Ml | pg/ml | 1.0E-9 | 1.0E-9 | 6.3E0 | 1.3E0 | 9.0E1 | 3.4E0 | 1.0E-9 | 1.0E-9 | 2.1E3 | 1.6E1 | 585 | 35 | 209 | 35 | 0.55 |
| Mm | pg/ml | 5.9E2 | 1.2E3 | 9.8E2 | 1.9E3 | 1.1E3 | 2.0E3 | 1.0E-9 | 1.0E-9 | 7.3E3 | 6.9E3 | 585 | 35 | 209 | 35 | 0.63 |
| Mn | pg/ml | 5.4E0 | 6.7E0 | 1.1E1 | 1.1E1 | 2.6E1 | 1.4E1 | 1.0E-9 | 1.0E-9 | 3.5E2 | 6.6E1 | 585 | 35 | 209 | 35 | 0.56 |
| Mp | pg/ml | 1.0E-9 | 3.3E0 | 9.2E0 | 1.9E1 | 3.2E1 | 4.4E1 | 1.0E-9 | 1.0E-9 | 6.3E2 | 1.8E2 | 584 | 35 | 209 | 35 | 0.57 |
| Mq | pg/ml | 1.0E-9 | 1.0E-9 | 2.9E0 | 4.8E0 | 1.7E1 | 2.4E1 | 1.0E-9 | 1.0E-9 | 2.1E2 | 1.4E2 | 584 | 35 | 209 | 35 | 0.50 |
| Mr | pg/ml | 1.0E-9 | 1.0E-9 | 1.9E1 | 4.0E2 | 8.5E1 | 2.0E3 | 1.0E-9 | 1.0E-9 | 1.4E3 | 1.2E4 | 584 | 35 | 209 | 35 | 0.55 |
| Ms | pg/ml | 4.1E2 | 6.1E2 | 5.6E2 | 5.6E2 | 6.5E2 | 4.9E2 | 1.0E-9 | 1.0E-9 | 5.6E3 | 1.7E3 | 584 | 35 | 209 | 35 | 0.53 |
| Mt | pg/ml | 2.2E-1 | 1.8E0 | 7.4E0 | 9.7E0 | 5.0E1 | 1.9E1 | 1.0E-9 | 1.0E-9 | 8.7E2 | 7.8E1 | 584 | 35 | 209 | 35 | 0.71 |
| Mu | pg/ml | 1.0E-9 | 1.0E-9 | 1.6E0 | 1.8E0 | 1.3E1 | 5.1E0 | 1.0E-9 | 1.0E-9 | 2.3E2 | 2.7E1 | 584 | 35 | 209 | 35 | 0.56 |
| Mv | pg/ml | 1.0E-9 | 1.0E-9 | 7.6E1 | 5.7E1 | 3.6E2 | 1.8E2 | 1.0E-9 | 1.0E-9 | 4.5E3 | 9.3E2 | 584 | 35 | 209 | 35 | 0.51 |
| Mw | pg/ml | 3.4E1 | 5.4E1 | 4.8E2 | 9.8E2 | 3.1E3 | 3.2E3 | 1.0E-9 | 1.0E-9 | 6.2E4 | 1.7E4 | 584 | 35 | 209 | 35 | 0.58 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Mx | pg/ml | 1.0E-9 | 1.0E-9 | 2.5E-1 | 2.4E-1 | 1.5E0 | 5.9E-1 | 1.0E-9 | 1.0E-9 | 3.2E1 | 3.0E0 | 584 | 35 | 209 | 35 | 0.56 |
| My | pg/ml | 1.0E-9 | 1.0E-9 | 4.7E2 | 3.5E2 | 3.1E3 | 9.1E2 | 1.0E-9 | 1.0E-9 | 3.9E4 | 4.1E3 | 584 | 35 | 209 | 35 | 0.56 |
| Mz | pg/ml | 1.0E1 | 2.0E1 | 2.5E1 | 3.9E1 | 7.2E1 | 4.7E1 | 1.0E-9 | 1.0E-9 | 1.2E3 | 2.0E2 | 584 | 35 | 209 | 35 | 0.63 |
| Na | pg/ml | 1.0E-9 | 1.0E-9 | 7.4E-1 | 8.3E-1 | 3.0E0 | 2.6E0 | 1.0E-9 | 1.0E-9 | 3.6E1 | 1.1E1 | 584 | 35 | 209 | 35 | 0.52 |
| Nb | pg/ml | 1.9E0 | 3.0E0 | 4.2E0 | 1.2E1 | 1.4E1 | 4.0E1 | 1.0E-9 | 1.0E-9 | 2.3E2 | 2.4E2 | 584 | 35 | 209 | 35 | 0.62 |
| Nc | pg/ml | 4.0E2 | 1.9E2 | 6.3E2 | 2.7E2 | 7.9E2 | 3.5E2 | 1.0E-9 | 1.0E-9 | 9.2E3 | 1.4E3 | 584 | 35 | 209 | 35 | 0.35 |
| Nd | pg/ml | 2.9E1 | 4.4E0 | 2.7E1 | 2.2E1 | 5.4E1 | 3.3E1 | 1.0E-9 | 1.0E-9 | 1.2E3 | 1.4E2 | 584 | 35 | 209 | 35 | 0.37 |
| Ne | pg/ml | 4.7E2 | 2.5E2 | 6.1E2 | 3.1E2 | 6.0E2 | 3.2E2 | 1.0E-9 | 1.0E-9 | 7.0E3 | 1.6E3 | 584 | 35 | 209 | 35 | 0.33 |
| Nf | pg/ml | 1.0E-9 | 1.0E-9 | 3.0E0 | 3.4E0 | 1.1E1 | 1.4E1 | 1.0E-9 | 1.0E-9 | 1.6E2 | 8.2E1 | 584 | 35 | 209 | 35 | 0.43 |
| Ng | pg/ml | 3.6E1 | 5.8E1 | 1.4E2 | 1.3E2 | 2.6E2 | 1.7E2 | 1.0E-9 | 1.0E-9 | 2.3E3 | 5.0E2 | 584 | 35 | 209 | 35 | 0.53 |
| Nh | pg/ml | 7.1E1 | 3.3E1 | 9.5E1 | 5.0E1 | 8.8E1 | 5.2E1 | 1.0E-9 | 1.0E-9 | 5.6E2 | 2.1E2 | 584 | 35 | 209 | 35 | 0.32 |
| Ni | pg/ml | 1.0E-9 | 2.3E1 | 7.8E1 | 1.0E2 | 1.2E2 | 1.4E2 | 1.0E-9 | 1.0E-9 | 7.1E2 | 5.5E2 | 584 | 35 | 209 | 35 | 0.54 |
| Nj | pg/ml | 7.9E0 | 2.8E0 | 1.1E1 | 5.5E0 | 1.2E1 | 6.3E0 | 1.0E-9 | 1.0E-9 | 8.3E1 | 2.3E1 | 584 | 35 | 209 | 35 | 0.34 |
| Nk | pg/ml | 2.0E1 | 1.6E1 | 3.5E1 | 2.5E1 | 4.0E1 | 3.4E1 | 1.0E-9 | 1.0E-9 | 2.0E2 | 1.7E2 | 584 | 35 | 209 | 35 | 0.45 |
| Nl | pg/ml | 4.9E1 | 1.6E1 | 6.6E1 | 2.6E1 | 7.4E1 | 2.7E1 | 1.0E-9 | 1.0E-9 | 1.1E3 | 9.1E1 | 584 | 35 | 209 | 35 | 0.30 |
| Tz | pg/ml | 5.3E3 | 8.8E3 | 1.4E4 | 9.3E3 | 6.9E4 | 1.2E4 | 1.0E-9 | 2.3E2 | 1.0E6 | 5.6E4 | 219 | 22 | 133 | 22 | 0.52 |
| Ua | pg/ml | 3.9E3 | 5.6E3 | 1.6E4 | 8.4E3 | 2.9E4 | 1.1E4 | 1.0E-9 | 4.3E2 | 1.9E5 | 4.3E4 | 219 | 22 | 133 | 22 | 0.51 |
| Ub | pg/ml | 5.7E2 | 5.7E2 | 8.5E2 | 8.8E2 | 1.1E3 | 1.2E3 | 1.0E-9 | 3.4E1 | 9.8E3 | 4.9E3 | 219 | 22 | 133 | 22 | 0.49 |
| Uc | pg/ml | 2.9E1 | 2.2E1 | 3.6E1 | 5.3E1 | 3.2E1 | 9.3E1 | 9.8E-2 | 5.1E0 | 3.5E2 | 4.4E2 | 219 | 22 | 133 | 22 | 0.45 |
| Uc | pg/ml | 9.2E2 | 1.0E3 | 1.7E3 | 1.8E3 | 2.9E3 | 1.8E3 | 1.0E-9 | 6.0E1 | 2.9E4 | 7.2E3 | 219 | 22 | 133 | 22 | 0.54 |
| Ud | pg/ml | 1.0E-9 | 1.0E-9 | 1.9E0 | 1.0E-9 | 2.6E1 | 0.0E0 | 1.0E-9 | 1.0E-9 | 3.9E2 | 1.0E-9 | 219 | 22 | 133 | 22 | 0.49 |
| Hq | pg/ml | 1.1E0 | 1.9E0 | 1.5E2 | 1.1E1 | 2.0E3 | 5.0E1 | 1.0E-9 | 1.0E-9 | 3.4E4 | 3.0E2 | 581 | 35 | 208 | 35 | 0.55 |
| Hr | pg/ml | 1.3E2 | 1.1E2 | 8.0E2 | 1.0E3 | 1.6E3 | 3.4E3 | 1.0E-9 | 1.0E-9 | 1.4E4 | 1.7E4 | 581 | 35 | 208 | 35 | 0.45 |
| Hu | pg/ml | 1.1E1 | 1.2E1 | 3.2E3 | 1.7E3 | 3.1E4 | 8.0E3 | 1.0E-9 | 1.0E-9 | 6.3E5 | 4.7E4 | 581 | 35 | 208 | 35 | 0.51 |
| Hv | pg/ml | 1.5E0 | 3.0E0 | 3.4E0 | 5.0E0 | 1.2E1 | 1.0E1 | 1.0E-9 | 1.0E-9 | 2.5E2 | 5.9E1 | 581 | 35 | 208 | 35 | 0.64 |
| Hw | pg/ml | 7.0E0 | 3.7E0 | 2.2E1 | 1.1E2 | 8.6E1 | 5.8E2 | 1.0E-9 | 1.0E-9 | 1.7E3 | 3.4E3 | 581 | 35 | 208 | 35 | 0.42 |
| Hx | pg/ml | 9.6E0 | 1.0E1 | 4.7E1 | 7.3E1 | 4.0E2 | 3.3E2 | 1.0E-9 | 1.0E-9 | 9.3E3 | 2.0E3 | 581 | 35 | 208 | 35 | 0.47 |
| Ib | ng/ml | 6.7E-2 | 3.1E-2 | 2.3E0 | 4.5E-1 | 7.8E0 | 1.3E0 | 1.0E-9 | 1.0E-9 | 5.3E1 | 6.1E0 | 210 | 22 | 132 | 22 | 0.42 |
| Ic | U/ml | 2.2E2 | 2.2E2 | 8.1E2 | 2.1E3 | 6.5E3 | 6.6E3 | 2.4E0 | 1.5E0 | 9.3E4 | 3.0E4 | 210 | 22 | 132 | 22 | 0.60 |
| Id | U/ml | 6.5E-1 | 1.2E0 | 1.2E0 | 2.3E0 | 2.0E0 | 4.3E0 | 1.0E-9 | 5.6E-2 | 2.3E1 | 2.1E1 | 210 | 22 | 132 | 22 | 0.67 |
| Tt | pg/ml | 1.7E2 | 1.8E2 | 1.7E2 | 1.8E2 | 5.1E1 | 4.0E1 | 4.3E1 | 1.2E2 | 3.6E2 | 2.6E2 | 202 | 22 | 127 | 22 | 0.58 |
| To | pg/ml | 1.6E0 | 1.9E0 | 1.9E0 | 2.2E0 | 2.0E0 | 1.8E0 | 1.0E-9 | 1.0E-9 | 1.0E1 | 6.3E0 | 211 | 22 | 130 | 22 | 0.58 |
| Tr | pg/ml | 3.0E0 | 4.3E0 | 6.2E0 | 5.8E0 | 2.2E1 | 5.1E0 | 1.0E-9 | 9.5E-2 | 3.1E2 | 2.1E1 | 207 | 22 | 129 | 22 | 0.58 |
| Tn | pg/ml | 2.9E1 | 3.2E1 | 7.5E1 | 4.6E1 | 2.0E2 | 4.8E1 | 2.4E0 | 1.1E1 | 1.8E3 | 2.3E2 | 211 | 22 | 130 | 22 | 0.55 |
| Tv | ng/ml | 1.2E1 | 1.6E1 | 2.0E1 | 2.0E1 | 3.9E1 | 2.1E1 | 1.0E-9 | 1.0E-9 | 4.9E2 | 7.0E1 | 211 | 22 | 130 | 22 | 0.51 |
| Ih | ng/ml | 7.5E1 | 1.8E2 | 2.1E2 | 4.4E2 | 3.6E2 | 7.2E2 | 1.0E-9 | 1.0E-9 | 2.6E3 | 2.9E3 | 584 | 35 | 208 | 35 | 0.58 |
| Ii | ng/ml | 9.8E1 | 1.3E2 | 2.7E2 | 5.4E2 | 7.6E2 | 1.7E3 | 7.3E-1 | 3.5E0 | 1.0E4 | 9.2E3 | 584 | 35 | 208 | 35 | 0.54 |
| Ij | ng/ml | 7.6E1 | 1.0E2 | 1.9E2 | 2.9E2 | 6.3E2 | 1.1E3 | 2.1E0 | 8.7E0 | 6.4E3 | 6.4E3 | 579 | 35 | 207 | 35 | 0.59 |
| Ik | ng/ml | 1.4E1 | 2.8E1 | 1.1E3 | 5.6E2 | 1.0E4 | 8.5E2 | 5.9E-1 | 1.3E0 | 1.2E5 | 4.5E3 | 581 | 35 | 207 | 35 | 0.68 |
| Il | ng/ml | 3.4E2 | 3.8E2 | 1.3E3 | 1.3E3 | 2.7E3 | 2.9E3 | 1.0E-9 | 1.0E-9 | 1.2E4 | 1.2E4 | 574 | 32 | 207 | 32 | 0.50 |
| Im | ng/ml | 2.0E2 | 3.7E2 | 3.5E2 | 6.3E2 | 5.2E2 | 1.1E3 | 1.3E1 | 2.4E1 | 6.0E3 | 6.8E3 | 581 | 35 | 207 | 35 | 0.64 |
| In | ng/ml | 3.9E0 | 4.7E0 | 2.5E1 | 1.1E1 | 1.8E2 | 1.9E1 | 1.0E-9 | 1.0E-9 | 3.9E3 | 7.5E1 | 584 | 35 | 208 | 35 | 0.49 |
| IIb | ng/ml | 2.4E1 | 3.7E1 | 3.2E1 | 4.2E1 | 2.9E1 | 3.3E1 | 4.8E-1 | 1.5E0 | 1.4E2 | 1.1E2 | 217 | 22 | 134 | 22 | 0.59 |
| Hc | pg/ml | 7.6E2 | 5.8E2 | 4.0E3 | 2.4E3 | 1.4E4 | 3.6E3 | 1.0E-9 | 2.6E2 | 1.0E5 | 1.1E4 | 217 | 22 | 134 | 22 | 0.50 |
| Hf | ng/ml | 1.5E2 | 2.2E2 | 4.1E2 | 3.1E2 | 5.8E2 | 3.4E2 | 1.0E-9 | 1.0E-9 | 3.2E3 | 1.3E3 | 217 | 22 | 134 | 22 | 0.51 |
| Io | ng/ml | 8.4E3 | 8.5E3 | 2.7E4 | 5.1E4 | 1.8E5 | 1.3E5 | 1.0E-9 | 2.2E2 | 4.0E6 | 5.5E5 | 578 | 35 | 208 | 35 | 0.54 |
| Ip | ng/ml | 1.0E1 | 2.5E1 | 1.9E1 | 3.0E1 | 2.4E1 | 3.3E1 | 1.0E-9 | 1.2E-2 | 2.6E2 | 1.4E2 | 578 | 35 | 208 | 35 | 0.58 |
| Iq | ug/ml | 9.8E-2 | 3.0E-2 | 2.4E1 | 2.4E1 | 5.7E2 | 1.3E2 | 1.0E-9 | 1.0E-9 | 1.4E4 | 7.5E2 | 578 | 35 | 208 | 35 | 0.44 |
| Ir | ug/ml | 3.4E-1 | 7.7E-1 | 2.7E0 | 5.0E0 | 1.7E1 | 1.9E1 | 1.0E-9 | 1.0E-9 | 2.4E2 | 1.1E2 | 577 | 35 | 208 | 35 | 0.63 |
| Is | ng/ml | 1.5E0 | 1.9E0 | 5.4E0 | 1.4E1 | 1.1E1 | 4.0E1 | 1.0E-9 | 1.0E-9 | 8.8E1 | 2.3E2 | 578 | 35 | 208 | 35 | 0.59 |
| It | ng/ml | 1.9E0 | 3.9E0 | 2.2E1 | 2.6E1 | 1.5E2 | 1.0E2 | 1.0E-9 | 1.0E-9 | 2.8E3 | 5.9E2 | 578 | 35 | 208 | 35 | 0.59 |
| Iu | ng/ml | 2.2E2 | 2.6E2 | 1.3E3 | 2.5E3 | 4.0E3 | 6.8E3 | 1.0E-9 | 1.0E-9 | 2.9E4 | 2.4E4 | 578 | 35 | 208 | 35 | 0.52 |
| Iv | ng/ml | 1.4E1 | 3.0E1 | 4.1E1 | 2.5E2 | 1.0E2 | 1.1E3 | 1.0E-9 | 1.0E-9 | 1.6E3 | 6.4E3 | 577 | 35 | 208 | 35 | 0.63 |

| Code | Units | Median | | Average | | Standard Deviation | | Minimum | | Maximum | | Number of Samples | | Number of Patients | | AUC |
|------|-------|--------|----|---------|----|----|----|---------|----|---------|----|--------|----|--------|----|-----|
| | | NonDis | Dis | NonDis | Dis | NonDis | Dis | NonDis | Dis | NonDis | Dis | NonDis | Dis | NonDis | Dis | |
| Iz | ng/ml | 1.5E2 | 2.1E2 | 7.3E2 | 3.6E2 | 4.3E3 | 4.2E2 | 1.5E0 | 9.2E-1 | 6.2E4 | 1.6E3 | 217 | 22 | 134 | 22 | 0.55 |
| Rc | pg/ml | 5.7E3 | 6.1E3 | 7.2E3 | 6.3E3 | 5.4E3 | 3.8E3 | 1.9E2 | 7.5E2 | 2.3E4 | 1.6E4 | 216 | 22 | 133 | 22 | 0.49 |
| Rb | pg/ml | 7.6E-1 | 1.9E0 | 2.7E0 | 4.0E0 | 4.5E0 | 5.4E0 | 1.0E-9 | 1.0E-9 | 4.3E1 | 2.1E1 | 216 | 22 | 133 | 22 | 0.62 |
| Pz | ng/ml | 3.7E3 | 1.0E4 | 7.4E3 | 9.6E3 | 1.9E4 | 1.6E4 | 1.3E1 | 1.5E2 | 2.8E5 | 9.5E4 | 577 | 35 | 206 | 35 | 0.60 |
| Qa | ng/ml | 3.2E3 | 6.6E3 | 6.1E3 | 8.5E3 | 7.4E3 | 6.8E3 | 1.5E2 | 3.8E2 | 5.2E4 | 2.4E4 | 577 | 35 | 206 | 35 | 0.64 |
| Qb | ng/ml | 9.9E1 | 1.3E2 | 2.2E2 | 2.2E2 | 5.2E2 | 2.0E2 | 7.9E-1 | 1.0E1 | 8.3E3 | 6.0E2 | 577 | 35 | 206 | 35 | 0.57 |
| Qc | ng/ml | 2.5E2 | 3.2E2 | 4.7E2 | 4.3E2 | 8.0E2 | 4.3E2 | 1.0E-9 | 6.8E0 | 1.1E4 | 1.6E3 | 577 | 35 | 206 | 35 | 0.53 |
| Qd | ng/ml | 1.0E4 | 1.3E4 | 2.3E4 | 2.3E4 | 9.4E4 | 2.5E4 | 2.4E2 | 9.0E2 | 2.0E6 | 1.2E5 | 577 | 35 | 206 | 35 | 0.59 |
| Qe | ng/ml | 8.8E2 | 1.7E3 | 1.9E3 | 2.7E3 | 4.6E3 | 2.7E3 | 7.6E0 | 8.2E1 | 9.7E4 | 1.4E4 | 577 | 35 | 206 | 35 | 0.67 |
| Jd | ng/ml | 9.6E-1 | 1.1E0 | 7.2E0 | 3.8E0 | 4.7E1 | 7.7E0 | 1.0E-9 | 1.0E-9 | 6.5E2 | 3.3E1 | 217 | 22 | 135 | 22 | 0.55 |
| Je | ng/ml | 1.0E-9 | 3.9E-1 | 2.5E0 | 1.7E0 | 8.5E0 | 2.8E0 | 1.0E-9 | 1.0E-9 | 8.8E1 | 1.1E1 | 217 | 22 | 135 | 22 | 0.55 |
| Jf | ng/ml | 1.0E-9 | 7.4E-1 | 1.2E0 | 1.7E0 | 2.4E0 | 2.5E0 | 1.0E-9 | 1.0E-9 | 1.2E1 | 9.2E0 | 217 | 22 | 135 | 22 | 0.64 |
| Jg | ng/ml | 4.7E2 | 1.1E3 | 7.6E2 | 1.6E3 | 9.6E2 | 1.6E3 | 1.0E-9 | 2.1E1 | 1.0E4 | 6.8E3 | 581 | 35 | 208 | 35 | 0.69 |
| Jh | ng/ml | 3.1E0 | 6.7E0 | 2.6E1 | 4.2E1 | 1.1E2 | 9.6E1 | 1.0E-9 | 1.0E-9 | 1.6E3 | 4.7E2 | 581 | 35 | 208 | 35 | 0.60 |
| Ji | ng/ml | 5.0E1 | 7.4E1 | 7.1E1 | 1.7E2 | 7.0E1 | 3.1E2 | 1.0E-9 | 1.0E1 | 5.3E2 | 1.8E3 | 581 | 35 | 208 | 35 | 0.67 |
| Sr | pg/mL | 3.5E2 | 7.0E2 | 8.5E2 | 1.2E3 | 1.3E3 | 1.1E3 | 1.0E-9 | 1.9E1 | 9.8E3 | 4.1E3 | 207 | 22 | 130 | 22 | 0.64 |
| Ss | pg/mL | 1.2E5 | 1.0E5 | 1.6E5 | 1.3E5 | 2.0E5 | 1.1E5 | 2.7E3 | 1.4E4 | 1.8E6 | 4.7E5 | 207 | 22 | 130 | 22 | 0.50 |
| St | pg/mL | 2.5E7 | 5.1E7 | 5.5E7 | 8.6E7 | 9.9E7 | 1.1E8 | 1.0E-9 | 1.5E6 | 1.2E9 | 4.2E8 | 212 | 22 | 131 | 22 | 0.61 |
| Ra | pg/ml | 1.0E-9 | 1.0E-9 | 6.4E-1 | 6.5E-1 | 1.3E0 | 1.2E0 | 1.0E-9 | 1.0E-9 | 7.3E0 | 4.1E0 | 216 | 22 | 133 | 22 | 0.56 |
| Qz | pg/ml | 1.0E1 | 2.0E1 | 6.0E1 | 5.4E1 | 1.0E2 | 6.7E1 | 1.0E-9 | 1.0E-9 | 7.1E2 | 2.0E2 | 216 | 22 | 133 | 22 | 0.49 |
| Qy | pg/ml | 4.6E-1 | 5.0E-1 | 1.5E1 | 2.5E1 | 7.2E1 | 1.1E2 | 1.0E-9 | 1.1E-2 | 6.5E2 | 5.1E2 | 216 | 22 | 133 | 22 | 0.54 |
| Qx | pg/ml | 1.0E-9 | 1.0E-9 | 7.6E0 | 1.2E0 | 5.5E1 | 2.3E0 | 1.0E-9 | 1.0E-9 | 5.8E2 | 7.3E0 | 216 | 22 | 133 | 22 | 0.57 |
| Qw | pg/ml | 4.5E-2 | 1.0E-9 | 2.2E0 | 2.8E-1 | 8.8E0 | 7.2E-1 | 1.0E-9 | 1.0E-9 | 8.6E1 | 2.4E0 | 216 | 22 | 133 | 22 | 0.36 |
| Qv | pg/ml | 2.2E4 | 1.9E4 | 3.5E4 | 3.1E4 | 6.4E4 | 3.3E4 | 1.0E-9 | 6.0E1 | 7.4E5 | 1.4E5 | 216 | 22 | 133 | 22 | 0.48 |
| Qu | pg/ml | 1.2E1 | 1.7E1 | 8.9E1 | 9.1E1 | 1.7E2 | 2.0E2 | 1.0E-9 | 1.0E-9 | 9.8E2 | 9.2E2 | 216 | 22 | 133 | 22 | 0.52 |
| Qt | pg/ml | 1.2E1 | 5.9E-1 | 5.7E1 | 2.7E1 | 1.4E2 | 5.5E1 | 1.0E-9 | 1.0E-9 | 1.0E3 | 1.9E2 | 216 | 22 | 133 | 22 | 0.41 |
| Qh | ng/ml | 1.7E1 | 1.5E1 | 3.7E1 | 2.9E1 | 6.3E1 | 2.9E1 | 1.0E-9 | 3.9E0 | 6.4E2 | 1.1E2 | 216 | 22 | 133 | 22 | 0.52 |
| Qg | ng/ml | 7.9E0 | 6.5E0 | 2.0E1 | 1.0E1 | 7.5E1 | 9.9E0 | 5.1E-2 | 1.4E-1 | 1.0E3 | 4.2E1 | 216 | 22 | 133 | 22 | 0.46 |
| Jj | ng/ml | 7.5E2 | 3.0E2 | 2.2E3 | 9.1E2 | 1.5E4 | 1.9E3 | 1.7E1 | 1.7E1 | 3.4E5 | 1.1E4 | 581 | 35 | 208 | 35 | 0.34 |
| Jk | ng/ml | 3.3E0 | 3.9E0 | 2.4E1 | 3.0E1 | 4.9E1 | 7.0E1 | 1.0E-9 | 1.0E-1 | 3.9E2 | 3.5E2 | 581 | 35 | 208 | 35 | 0.51 |
| Jl | ng/ml | 4.4E-1 | 8.5E-1 | 1.8E0 | 6.8E0 | 4.3E0 | 2.6E1 | 7.6E-4 | 2.9E-3 | 3.2E1 | 1.6E2 | 581 | 35 | 208 | 35 | 0.58 |
| Jm | ng/ml | 1.9E1 | 3.2E1 | 5.2E1 | 5.5E1 | 9.7E1 | 6.5E1 | 1.0E-9 | 8.3E-1 | 1.0E3 | 2.3E2 | 581 | 35 | 208 | 35 | 0.57 |
| Jn | pg/ml | 4.0E-1 | 7.6E-1 | 1.7E0 | 2.4E0 | 5.8E0 | 4.9E0 | 1.0E-9 | 1.0E-9 | 6.2E1 | 2.7E1 | 581 | 35 | 208 | 35 | 0.60 |
| Jo | pg/ml | 4.0E3 | 4.8E3 | 5.1E3 | 5.6E3 | 3.9E3 | 6.2E3 | 4.2E1 | 4.9E1 | 2.4E4 | 3.8E4 | 581 | 35 | 208 | 35 | 0.51 |
| Jp | pg/ml | 7.0E4 | 1.0E5 | 7.3E4 | 9.5E4 | 3.4E4 | 3.9E4 | 2.1E3 | 3.5E3 | 2.1E5 | 1.7E5 | 581 | 35 | 208 | 35 | 0.68 |
| Jq | pg/ml | 9.5E1 | 1.1E2 | 1.5E2 | 2.5E2 | 2.2E2 | 6.1E2 | 2.6E0 | 8.1E0 | 4.0E3 | 3.7E3 | 581 | 35 | 208 | 35 | 0.52 |
| Jr | pg/ml | 5.2E0 | 5.6E0 | 2.2E1 | 2.0E1 | 1.0E2 | 4.6E1 | 1.0E-9 | 1.0E-9 | 1.9E3 | 2.0E2 | 581 | 35 | 208 | 35 | 0.56 |
| Js | pg/ml | 1.3E1 | 1.6E1 | 4.2E1 | 3.6E1 | 1.5E2 | 9.8E1 | 1.0E-9 | 1.0E-9 | 2.0E3 | 5.9E2 | 581 | 35 | 208 | 35 | 0.56 |
| Jt | pg/ml | 2.7E3 | 3.8E3 | 3.2E3 | 4.6E3 | 2.2E3 | 5.4E3 | 1.5E2 | 7.7E1 | 1.3E4 | 3.3E4 | 581 | 35 | 208 | 35 | 0.59 |
| Ju | mIU/ml | 9.0E0 | 9.6E0 | 2.1E1 | 1.4E1 | 3.3E1 | 1.3E1 | 6.5E-2 | 6.5E-1 | 2.3E2 | 4.9E1 | 217 | 22 | 135 | 22 | 0.53 |
| Jv | mIU/ml | 1.2E1 | 1.3E1 | 3.7E1 | 2.3E1 | 6.6E1 | 2.3E1 | 1.0E-2 | 1.8E-1 | 4.4E2 | 9.1E1 | 217 | 22 | 135 | 22 | 0.52 |
| Jy | ng/ml | 1.6E-3 | 1.5E-3 | 2.3E-3 | 1.8E-3 | 4.6E-3 | 1.3E-3 | 1.7E-4 | 8.7E-5 | 5.2E-2 | 6.4E-3 | 217 | 22 | 135 | 22 | 0.47 |
| Kc | pg/ml | 2.3E1 | 5.7E1 | 4.0E1 | 7.8E1 | 4.3E1 | 6.9E1 | 1.0E-9 | 1.0E-9 | 1.9E2 | 2.0E2 | 218 | 22 | 134 | 22 | 0.62 |
| Kd | pg/ml | 1.0E-9 | 1.0E-9 | 2.0E2 | 3.7E2 | 6.0E2 | 6.9E2 | 1.0E-9 | 1.0E-9 | 4.8E3 | 2.3E3 | 218 | 22 | 134 | 22 | 0.57 |
| Kc | pg/ml | 1.3E4 | 1.6E4 | 1.4E4 | 1.6E4 | 1.1E4 | 7.7E3 | 3.4E2 | 1.1E3 | 7.0E4 | 3.6E4 | 218 | 22 | 134 | 22 | 0.59 |
| Kf | pg/mL | 6.7E0 | 9.4E0 | 7.3E0 | 9.2E0 | 5.9E0 | 5.0E0 | 1.0E-9 | 1.0E-9 | 2.7E1 | 1.8E1 | 218 | 22 | 134 | 22 | 0.63 |
| Kg | pg/mL | 1.2E3 | 1.4E3 | 2.0E3 | 1.6E3 | 2.4E3 | 1.3E3 | 7.3E1 | 1.3E2 | 1.7E4 | 4.4E3 | 218 | 22 | 134 | 22 | 0.50 |
| Ki | pg/ml | 5.9E1 | 5.2E1 | 7.0E1 | 5.7E1 | 5.5E1 | 3.7E1 | 1.0E-9 | 1.0E-9 | 3.8E2 | 1.4E2 | 217 | 22 | 134 | 22 | 0.44 |
| Kj | pg/ml | 1.1E3 | 1.2E3 | 1.7E3 | 1.5E3 | 1.7E3 | 1.3E3 | 1.4E1 | 3.0E1 | 1.0E4 | 4.5E3 | 218 | 22 | 134 | 22 | 0.47 |
| Kk | pg/ml | 6.8E0 | 9.0E0 | 1.1E1 | 2.0E1 | 1.6E1 | 2.0E1 | 1.0E-9 | 1.0E-9 | 1.6E2 | 6.1E1 | 218 | 22 | 134 | 22 | 0.66 |
| Kl | pg/ml | 2.1E4 | 3.3E4 | 2.9E4 | 3.3E4 | 2.6E4 | 2.4E4 | 1.6E2 | 1.4E3 | 1.6E5 | 1.0E5 | 218 | 22 | 134 | 22 | 0.56 |
| Kn | pg/ml | 3.0E1 | 5.5E1 | 6.2E1 | 8.5E1 | 9.5E1 | 1.1E2 | 1.0E-9 | 1.0E-9 | 7.3E2 | 4.1E2 | 218 | 22 | 134 | 22 | 0.55 |
| Ko | pg/ml | 3.4E2 | 7.4E2 | 4.6E2 | 8.1E2 | 4.5E2 | 5.9E2 | 1.0E-9 | 4.2E1 | 2.2E3 | 2.4E3 | 218 | 22 | 134 | 22 | 0.70 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Kp | pg/ml | 3.4E2 | 3.3E2 | 3.5E2 | 4.2E2 | 2.7E2 | 2.4E2 | 1.0E-9 | 6.4E1 | 1.7E3 | 9.1E2 | 218 | 22 | 134 | 22 | 0.57 |
| Kq | pg/ml | 3.2E2 | 4.7E2 | 5.1E2 | 5.0E2 | 9.1E2 | 2.4E2 | 1.6E0 | 2.5E1 | 9.8E3 | 8.7E2 | 209 | 22 | 128 | 22 | 0.65 |
| Kr | pg/ml | 5.6E-1 | 9.0E-1 | 2.7E0 | 2.6E0 | 5.2E0 | 3.3E0 | 1.0E-9 | 1.0E-9 | 3.9E1 | 9.6E0 | 209 | 22 | 128 | 22 | 0.53 |
| Ks | pg/ml | 1.4E4 | 1.5E4 | 2.1E4 | 2.0E4 | 1.9E4 | 1.7E4 | 5.1E1 | 2.2E2 | 1.1E5 | 5.0E4 | 209 | 22 | 128 | 22 | 0.51 |
| Kx | ng/ml | 1.0E-9 | 1.0E-9 | 7.1E-3 | 5.1E-3 | 1.4E-2 | 6.2E-3 | 1.0E-9 | 1.0E-9 | 1.0E-1 | 1.5E-2 | 214 | 22 | 133 | 22 | 0.50 |
| Ky | ng/ml | 1.0E-1 | 1.1E-1 | 3.8E-1 | 7.2E-1 | 8.3E-1 | 1.4E0 | 1.0E-9 | 1.0E-9 | 5.4E0 | 6.3E0 | 214 | 22 | 133 | 22 | 0.59 |
| Kz | ng/ml | 1.0E-9 | 1.0E-9 | 3.4E-3 | 1.7E-3 | 5.7E-3 | 2.4E-3 | 1.0E-9 | 1.0E-9 | 3.6E-2 | 6.6E-3 | 214 | 22 | 133 | 22 | 0.47 |
| Ld | pg/ml | 1.0E-9 | 3.7E-1 | 3.7E0 | 2.6E0 | 9.6E0 | 3.8E0 | 1.0E-9 | 1.0E-9 | 8.4E1 | 1.2E1 | 216 | 22 | 133 | 22 | 0.53 |
| Lh | pg/ml | 1.3E4 | 2.2E4 | 2.1E4 | 4.1E4 | 2.7E4 | 7.3E4 | 1.0E-9 | 1.8E2 | 2.6E5 | 4.1E5 | 580 | 35 | 209 | 35 | 0.64 |
| Li | pg/ml | 3.1E3 | 6.6E3 | 1.5E4 | 4.2E4 | 3.9E4 | 1.0E5 | 1.0E-9 | 1.6E1 | 3.6E5 | 5.9E5 | 580 | 35 | 209 | 35 | 0.66 |
| Lj | pg/ml | 2.4E3 | 8.0E3 | 2.1E4 | 3.6E4 | 6.5E4 | 7.4E4 | 1.0E-9 | 2.9E1 | 4.7E5 | 3.5E5 | 580 | 35 | 209 | 35 | 0.65 |
| Rm | ng/ml | 1.9E1 | 3.7E1 | 5.1E1 | 4.4E1 | 7.7E1 | 5.5E1 | 2.2E-1 | 7.0E-1 | 4.0E2 | 2.5E2 | 213 | 21 | 132 | 21 | 0.54 |
| Rh | ng/ml | 1.3E2 | 1.6E2 | 2.8E2 | 3.1E2 | 5.0E2 | 5.2E2 | 4.7E0 | 1.4E1 | 3.8E3 | 2.5E3 | 213 | 21 | 132 | 21 | 0.58 |
| Ri | ng/ml | 1.0E-9 | 1.3E0 | 4.3E0 | 3.1E0 | 1.7E1 | 4.6E0 | 1.0E-9 | 1.0E-9 | 2.2E2 | 2.0E1 | 214 | 21 | 133 | 21 | 0.59 |
| Rg | ng/ml | 1.0E-9 | 1.0E-9 | 6.8E-2 | 2.1E-3 | 3.9E-1 | 5.1E-3 | 1.0E-9 | 1.0E-9 | 3.3E0 | 2.2E-2 | 213 | 21 | 132 | 21 | 0.53 |
| Rj | ng/ml | 1.0E-9 | 1.0E-9 | 1.6E0 | 3.0E-1 | 6.3E0 | 8.1E-1 | 1.0E-9 | 1.0E-9 | 8.0E1 | 3.2E0 | 214 | 21 | 133 | 21 | 0.44 |
| Rf | ng/ml | 3.7E-1 | 6.0E-1 | 1.0E0 | 1.6E0 | 1.9E0 | 2.2E0 | 7.8E-3 | 3.8E-2 | 1.5E1 | 7.5E0 | 213 | 21 | 132 | 21 | 0.63 |
| Ql | pg/ml | 5.5E0 | 4.5E0 | 1.5E1 | 1.1E1 | 3.2E1 | 1.5E1 | 1.0E-9 | 1.0E-9 | 2.9E2 | 5.9E1 | 217 | 22 | 135 | 22 | 0.51 |
| Qm | pg/ml | 4.4E0 | 1.4E1 | 2.0E1 | 1.9E1 | 4.0E1 | 1.9E1 | 1.0E-9 | 1.0E-9 | 3.6E2 | 5.9E1 | 217 | 22 | 135 | 22 | 0.56 |
| Qn | pg/ml | 6.1E-1 | 5.6E-1 | 5.8E0 | 6.5E0 | 1.9E1 | 2.2E1 | 1.0E-9 | 1.0E-9 | 2.2E2 | 1.0E2 | 217 | 22 | 135 | 22 | 0.44 |
| Nv | pg/ml | 4.0E3 | 7.9E3 | 1.2E4 | 1.8E4 | 5.2E4 | 2.9E4 | 1.0E-9 | 3.9E1 | 1.1E6 | 1.3E5 | 585 | 35 | 209 | 35 | 0.66 |
| Nw | pg/ml | 8.5E3 | 1.7E4 | 1.3E4 | 2.7E4 | 1.8E4 | 3.9E4 | 8.6E1 | 7.3E2 | 2.1E5 | 2.1E5 | 585 | 35 | 209 | 35 | 0.74 |
| Nx | pg/ml | 2.2E2 | 5.2E2 | 4.1E2 | 8.2E2 | 6.9E2 | 8.1E2 | 1.0E-9 | 1.0E-9 | 7.4E3 | 2.8E3 | 585 | 35 | 209 | 35 | 0.69 |
| Ny | pg/ml | 5.7E0 | 1.6E1 | 7.2E1 | 4.1E1 | 1.0E3 | 1.1E2 | 1.0E-9 | 1.0E-9 | 2.5E4 | 6.2E2 | 585 | 35 | 209 | 35 | 0.64 |
| Oa | pg/ml | 1.6E2 | 3.0E2 | 4.1E2 | 5.1E2 | 7.0E2 | 5.8E2 | 1.0E-9 | 1.0E-9 | 4.8E3 | 2.4E3 | 217 | 22 | 135 | 22 | 0.61 |
| Oe | pg/ml | 9.1E1 | 3.5E1 | 3.1E2 | 3.0E2 | 8.7E2 | 8.9E2 | 1.0E-9 | 1.0E-9 | 1.9E4 | 5.1E3 | 580 | 35 | 209 | 35 | 0.45 |
| Of | pg/ml | 2.3E2 | 3.2E2 | 7.1E3 | 5.4E3 | 3.3E4 | 1.7E4 | 1.0E-9 | 1.0E-9 | 6.2E5 | 7.4E4 | 585 | 35 | 209 | 35 | 0.51 |
| Og | pg/ml | 9.2E-2 | 9.0E-2 | 9.9E-1 | 4.1E-1 | 5.8E0 | 1.5E0 | 1.0E-9 | 1.0E-9 | 8.3E1 | 9.0E0 | 585 | 35 | 209 | 35 | 0.44 |
| Oh | pg/ml | 2.4E0 | 3.4E0 | 2.5E1 | 2.0E1 | 1.7E2 | 4.9E1 | 1.0E-9 | 1.0E-9 | 3.5E3 | 2.2E2 | 585 | 35 | 209 | 35 | 0.60 |
| Oi | pg/ml | 2.8E0 | 5.6E0 | 6.5E0 | 1.0E1 | 1.0E1 | 1.2E1 | 1.0E-9 | 1.0E-9 | 8.6E1 | 4.2E1 | 585 | 35 | 209 | 35 | 0.57 |
| Ok | pg/ml | 3.9E2 | 8.2E2 | 5.2E2 | 3.2E3 | 5.0E2 | 1.2E4 | 1.3E1 | 2.2E1 | 5.2E3 | 7.0E4 | 585 | 35 | 209 | 35 | 0.73 |
| Om | pg/ml | 3.8E2 | 6.8E2 | 9.0E2 | 1.7E3 | 2.5E3 | 3.5E3 | 1.0E-9 | 1.0E-9 | 3.6E4 | 1.7E4 | 585 | 35 | 209 | 35 | 0.60 |
| On | pg/ml | 1.8E2 | 3.7E2 | 2.9E2 | 1.1E3 | 4.3E2 | 2.9E3 | 8.4E-1 | 6.1E0 | 4.5E3 | 1.5E4 | 585 | 35 | 209 | 35 | 0.68 |
| Or | pg/ml | 1.4E1 | 1.7E1 | 3.4E1 | 5.7E1 | 6.3E1 | 1.6E2 | 1.0E-9 | 1.0E-9 | 5.0E2 | 7.6E2 | 218 | 22 | 134 | 22 | 0.54 |
| Ow | pg/ml | 3.3E1 | 2.6E1 | 1.1E2 | 1.3E2 | 3.1E2 | 2.3E2 | 1.0E-9 | 1.0E-9 | 2.7E3 | 7.9E2 | 218 | 22 | 134 | 22 | 0.54 |
| Ou | pg/ml | 4.7E2 | 7.2E2 | 8.8E2 | 2.1E3 | 1.3E3 | 3.1E3 | 1.0E-9 | 1.0E-9 | 9.8E3 | 9.3E3 | 218 | 22 | 134 | 22 | 0.57 |
| Ul | ng/ml | 1.0E-9 | 1.0E-9 | 1.4E0 | 5.2E0 | 4.7E0 | 2.2E1 | 1.0E-9 | 1.0E-9 | 3.4E1 | 1.0E2 | 224 | 22 | 137 | 22 | 0.51 |
| Uo | ng/ml | 1.0E-9 | 1.0E-9 | 1.1E-1 | 1.6E-1 | 2.7E-1 | 3.5E-1 | 1.0E-9 | 1.0E-9 | 2.6E0 | 1.5E0 | 224 | 22 | 137 | 22 | 0.52 |
| Uk | ng/ml | 1.0E-9 | 1.0E-9 | 8.7E-3 | 5.4E-3 | 2.9E-2 | 1.2E-2 | 1.0E-9 | 1.0E-9 | 3.2E-1 | 4.2E-2 | 224 | 22 | 137 | 22 | 0.42 |
| Um | ng/ml | 1.0E-9 | 1.0E-9 | 4.2E-1 | 3.5E-1 | 1.0E0 | 9.7E-1 | 1.0E-9 | 1.0E-9 | 7.2E0 | 4.4E0 | 224 | 22 | 137 | 22 | 0.45 |
| Uf | ng/ml | 5.3E-2 | 9.7E-2 | 1.5E-1 | 2.3E-1 | 2.7E-1 | 5.1E-1 | 1.0E-3 | 1.3E-3 | 2.1E0 | 2.5E0 | 224 | 22 | 137 | 22 | 0.59 |
| Uh | ng/ml | 1.9E0 | 3.3E0 | 2.9E0 | 3.9E0 | 3.1E0 | 3.0E0 | 3.2E-2 | 2.3E-1 | 1.7E1 | 1.2E1 | 224 | 22 | 137 | 22 | 0.64 |
| Un | ng/ml | 1.9E0 | 2.1E0 | 2.1E0 | 2.4E0 | 1.3E0 | 1.4E0 | 2.0E-1 | 1.8E-1 | 8.0E0 | 6.4E0 | 224 | 22 | 137 | 22 | 0.56 |
| Ug | ng/ml | 1.4E1 | 1.9E1 | 2.8E1 | 3.5E1 | 2.9E1 | 4.7E1 | 6.9E-1 | 1.1E0 | 1.8E2 | 2.1E2 | 224 | 22 | 137 | 22 | 0.51 |
| Ur | ng/ml | 1.5E-1 | 1.2E-1 | 8.5E-1 | 8.4E-1 | 6.3E0 | 1.9E0 | 1.0E-9 | 1.0E-9 | 9.3E1 | 7.2E0 | 223 | 22 | 136 | 22 | 0.46 |
| Up | ng/ml | 1.0E-9 | 1.0E-9 | 6.0E-3 | 2.1E-3 | 2.6E-2 | 6.6E-3 | 1.0E-9 | 1.0E-9 | 3.3E-1 | 2.5E-2 | 223 | 22 | 136 | 22 | 0.44 |
| Us | ng/ml | 4.3E-3 | 4.5E-3 | 1.9E-2 | 1.8E-2 | 4.6E-2 | 4.5E-2 | 1.0E-9 | 1.0E-9 | 4.0E-1 | 2.1E-1 | 223 | 22 | 136 | 22 | 0.49 |
| Uv | ng/ml | 3.2E-3 | 2.5E-3 | 1.3E-2 | 1.0E-2 | 3.9E-2 | 3.0E-2 | 1.0E-9 | 1.0E-9 | 4.3E-1 | 1.4E-1 | 223 | 22 | 136 | 22 | 0.46 |
| Ut | ng/ml | 7.6E-1 | 9.1E-1 | 3.2E0 | 1.1E0 | 9.7E0 | 1.3E0 | 1.0E-9 | 1.0E-9 | 7.8E1 | 5.3E0 | 223 | 22 | 136 | 22 | 0.47 |
| Uu | ng/ml | 7.2E0 | 8.8E0 | 7.8E0 | 8.9E0 | 4.8E0 | 6.3E0 | 5.7E-1 | 5.5E-1 | 2.6E1 | 2.2E1 | 223 | 22 | 136 | 22 | 0.54 |
| Vp | ng/ml | 1.0E-9 | 1.0E-9 | 4.6E-1 | 4.9E-3 | 3.8E0 | 2.3E-2 | 1.0E-9 | 1.0E-9 | 5.0E1 | 1.1E-1 | 224 | 22 | 137 | 22 | 0.44 |
| Vt | ng/ml | 6.1E0 | 7.9E0 | 8.3E0 | 1.1E1 | 9.3E0 | 9.3E0 | 4.3E-1 | 7.7E-1 | 8.6E1 | 3.4E1 | 224 | 22 | 137 | 22 | 0.62 |
| Vu | ng/ml | 1.0E-9 | 1.0E-9 | 2.9E0 | 9.0E-1 | 7.5E0 | 1.7E0 | 1.0E-9 | 1.0E-9 | 7.1E1 | 6.2E0 | 219 | 21 | 137 | 21 | 0.45 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vq | ng/ml | 1.5E2 | 6.0E2 | 6.3E2 | 1.2E3 | 1.4E3 | 1.6E3 | 2.0E-1 | 7.9E0 | 1.1E4 | 5.4E3 | 175 | 16 | 115 | 16 | 0.63 |
| Vo | ng/ml | 2.6E1 | 2.5E1 | 2.5E1 | 2.3E1 | 5.1E0 | 6.1E0 | 2.5E0 | 6.7E0 | 4.8E1 | 3.1E1 | 224 | 22 | 137 | 22 | 0.43 |
| Vs | ng/ml | 2.6E-1 | 1.0E-9 | 7.3E0 | 2.5E0 | 2.6E1 | 5.2E0 | 1.0E-9 | 1.0E-9 | 2.3E2 | 1.9E1 | 218 | 21 | 135 | 21 | 0.41 |
| Vv | ng/ml | 3.3E0 | 4.8E0 | 6.3E0 | 9.3E0 | 1.0E1 | 1.7E1 | 1.0E-9 | 1.0E-9 | 8.4E1 | 8.0E1 | 223 | 22 | 137 | 22 | 0.56 |
| Oy | pg/ml | 5.5E-1 | 5.8E-1 | 7.3E0 | 9.1E0 | 3.5E1 | 4.6E1 | 1.0E-9 | 1.0E-9 | 4.0E2 | 2.7E2 | 584 | 35 | 208 | 35 | 0.52 |
| Oz | pg/ml | 1.3E-2 | 2.4E-1 | 3.5E-1 | 3.1E-1 | 1.6E0 | 3.1E-1 | 1.0E-9 | 1.0E-9 | 2.9E1 | 1.0E0 | 584 | 35 | 208 | 35 | 0.61 |
| Pa | pg/ml | 3.9E-1 | 3.8E-1 | 1.5E0 | 9.8E0 | 5.8E0 | 4.9E1 | 1.0E-9 | 2.6E-2 | 8.6E1 | 2.9E2 | 584 | 35 | 208 | 35 | 0.56 |
| Pb | pg/ml | 1.0E-9 | 8.2E-2 | 1.1E0 | 5.3E0 | 2.0E1 | 3.0E1 | 1.0E-9 | 1.0E-9 | 4.9E2 | 1.8E2 | 584 | 35 | 208 | 35 | 0.59 |
| Pc | pg/ml | 4.9E-2 | 3.5E-1 | 3.8E-1 | 8.8E-1 | 1.0E0 | 2.1E0 | 1.0E-9 | 1.0E-9 | 1.6E1 | 1.1E1 | 584 | 35 | 208 | 35 | 0.59 |
| Pd | pg/ml | 1.8E0 | 3.0E0 | 5.5E0 | 5.1E0 | 3.6E1 | 5.3E0 | 1.0E-9 | 1.0E-9 | 8.4E2 | 1.9E1 | 584 | 35 | 208 | 35 | 0.61 |
| Pe | pg/ml | 2.1E1 | 3.6E1 | 1.1E2 | 5.3E2 | 3.7E2 | 2.4E3 | 1.0E-9 | 3.7E-1 | 4.7E3 | 1.4E4 | 584 | 35 | 208 | 35 | 0.64 |
| Pf | pg/ml | 1.5E0 | 2.5E0 | 1.1E1 | 1.6E1 | 6.6E1 | 4.2E1 | 1.0E-9 | 1.0E-9 | 1.5E3 | 2.3E2 | 584 | 35 | 208 | 35 | 0.60 |
| Pg | pg/ml | 3.3E0 | 6.2E0 | 5.4E1 | 7.6E1 | 4.2E2 | 3.2E2 | 1.0E-9 | 1.3E-1 | 7.7E3 | 1.9E3 | 584 | 35 | 208 | 35 | 0.61 |
| Ph | ng/ml | 1.6E-1 | 2.3E-1 | 3.3E-1 | 7.1E-1 | 4.9E-1 | 1.1E0 | 1.0E-9 | 1.0E-9 | 3.1E0 | 4.4E0 | 218 | 22 | 134 | 22 | 0.57 |
| Pi | ng/ml | 1.9E-1 | 2.2E-1 | 2.8E-1 | 2.5E-1 | 3.7E-1 | 2.3E-1 | 1.0E-9 | 1.0E-9 | 3.2E0 | 1.0E0 | 218 | 22 | 134 | 22 | 0.52 |
| Pj | ng/mL | 5.2E0 | 8.1E0 | 6.1E0 | 8.5E0 | 4.7E0 | 5.0E0 | 3.8E-2 | 1.6E-1 | 3.1E1 | 1.7E1 | 218 | 22 | 134 | 22 | 0.66 |
| Pk | ng/ml | 8.9E-3 | 1.1E-2 | 1.4E-2 | 1.2E-2 | 2.2E-2 | 1.1E-2 | 1.0E-9 | 1.0E-9 | 2.5E-1 | 3.8E-2 | 218 | 22 | 134 | 22 | 0.51 |
| aA | mg/dL | 8.0E-1 | 1.0E0 | 9.1E-1 | 1.3E0 | 4.4E-1 | 9.1E-1 | 2.0E-1 | 4.0E-1 | 4.2E0 | 5.4E0 | 1815 | 49 | 323 | 49 | 0.64 |
| aC | mg/mL | 2.8E0 | 2.5E0 | 3.1E0 | 2.9E0 | 1.3E0 | 1.3E0 | 8.5E-1 | 1.3E0 | 8.2E0 | 6.3E0 | 363 | 26 | 141 | 26 | 0.46 |
| aD | ug/mL | 3.1E0 | 4.8E0 | 4.3E0 | 5.3E0 | 3.5E0 | 3.8E0 | 8.5E-1 | 8.4E-1 | 3.1E1 | 1.7E1 | 363 | 26 | 141 | 26 | 0.57 |
| aE | mg/mL | 5.7E-1 | 5.6E-1 | 5.8E-1 | 5.7E-1 | 1.5E-1 | 1.6E-1 | 2.1E-1 | 2.8E-1 | 1.1E0 | 1.2E0 | 363 | 26 | 141 | 26 | 0.47 |
| aF | ng/mL | 2.1E0 | 2.6E0 | 4.1E0 | 4.1E0 | 6.1E0 | 4.3E0 | 4.3E-3 | 4.3E-3 | 5.0E1 | 1.5E1 | 363 | 26 | 141 | 26 | 0.53 |
| aG | mg/mL | 1.4E-1 | 1.4E-1 | 1.6E-1 | 1.6E-1 | 8.4E-2 | 1.0E-1 | 5.0E-2 | 6.6E-2 | 5.0E-1 | 5.2E-1 | 363 | 26 | 141 | 26 | 0.47 |
| aH | ug/mL | 7.5E1 | 8.2E1 | 8.1E1 | 9.1E1 | 4.2E1 | 5.0E1 | 9.6E0 | 2.5E1 | 2.9E2 | 2.3E2 | 363 | 26 | 141 | 26 | 0.56 |
| aI | ug/mL | 1.9E2 | 1.9E2 | 1.9E2 | 1.9E2 | 6.0E1 | 4.6E1 | 4.7E1 | 1.2E2 | 3.7E2 | 2.7E2 | 363 | 26 | 141 | 26 | 0.52 |
| aJ | ug/mL | 2.4E0 | 3.8E0 | 3.0E0 | 4.8E0 | 2.2E0 | 3.9E0 | 9.0E-1 | 7.8E-1 | 1.7E1 | 1.7E1 | 363 | 26 | 141 | 26 | 0.69 |
| aK | ng/mL | 1.6E0 | 8.9E-1 | 2.5E0 | 1.7E0 | 2.7E0 | 1.8E0 | 2.9E-4 | 8.8E-2 | 1.8E1 | 7.4E0 | 363 | 26 | 141 | 26 | 0.40 |
| aL | mg/mL | 7.9E-1 | 8.5E-1 | 8.0E-1 | 8.3E-1 | 2.4E-1 | 2.5E-1 | 2.2E-1 | 4.0E-1 | 1.7E0 | 1.6E0 | 363 | 26 | 141 | 26 | 0.53 |
| aM | U/mL | 2.2E1 | 3.3E1 | 4.4E1 | 5.9E1 | 1.0E2 | 8.0E1 | 4.2E-2 | 4.2E-2 | 1.6E3 | 4.0E2 | 363 | 26 | 141 | 26 | 0.60 |
| aN | U/mL | 1.4E1 | 2.5E1 | 2.1E1 | 2.9E1 | 3.0E1 | 2.5E1 | 2.5E-3 | 2.5E-3 | 3.8E2 | 9.7E1 | 363 | 26 | 141 | 26 | 0.64 |
| aO | pg/mL | 3.5E1 | 4.8E1 | 3.4E2 | 4.6E2 | 8.8E2 | 8.3E2 | 6.0E-2 | 6.0E-2 | 6.6E3 | 3.5E3 | 363 | 26 | 141 | 26 | 0.57 |
| aP | ng/mL | 1.6E0 | 2.0E0 | 2.0E0 | 2.7E0 | 1.2E0 | 2.2E0 | 5.4E-1 | 7.2E-1 | 7.0E0 | 1.1E1 | 363 | 26 | 141 | 26 | 0.62 |
| aQ | ng/mL | 3.0E-1 | 2.2E-1 | 4.6E-1 | 3.6E-1 | 4.9E-1 | 3.8E-1 | 2.0E-4 | 3.9E-2 | 4.0E0 | 1.7E0 | 363 | 26 | 141 | 26 | 0.41 |
| aR | ng/mL | 1.8E0 | 2.3E0 | 2.9E0 | 2.9E0 | 3.5E0 | 2.8E0 | 1.8E-1 | 4.9E-1 | 3.4E1 | 1.4E1 | 363 | 26 | 141 | 26 | 0.54 |
| aS | ng/mL | 2.7E-1 | 3.6E-1 | 7.2E-1 | 6.3E-1 | 2.0E0 | 1.1E0 | 4.2E-3 | 4.2E-3 | 3.3E1 | 5.6E0 | 363 | 26 | 141 | 26 | 0.52 |
| aU | pg/mL | 7.8E1 | 6.8E1 | 1.3E2 | 1.2E2 | 1.5E2 | 1.4E2 | 7.4E-2 | 1.1E1 | 1.3E3 | 6.0E2 | 363 | 26 | 141 | 26 | 0.46 |
| aV | ng/mL | 6.3E-1 | 3.2E-1 | 1.1E0 | 8.2E-1 | 2.1E0 | 1.0E0 | 7.6E-4 | 3.1E-2 | 3.3E1 | 4.2E0 | 363 | 26 | 141 | 26 | 0.39 |
| aW | pg/mL | 1.9E1 | 1.9E1 | 2.0E1 | 3.8E1 | 2.1E1 | 8.4E1 | 7.2E-2 | 7.2E-2 | 2.4E2 | 4.5E2 | 363 | 26 | 141 | 26 | 0.56 |
| aX | ng/mL | 9.5E0 | 1.1E1 | 1.4E1 | 2.0E1 | 1.4E1 | 2.3E1 | 3.0E-1 | 2.5E0 | 8.0E1 | 1.1E2 | 363 | 26 | 141 | 26 | 0.57 |
| aY | pg/mL | 6.0E1 | 6.5E1 | 8.0E1 | 7.8E1 | 9.2E1 | 6.0E1 | 4.1E-1 | 4.1E-1 | 1.2E3 | 2.7E2 | 363 | 26 | 141 | 26 | 0.54 |
| aZ | pg/mL | 2.2E2 | 3.7E2 | 5.2E2 | 5.8E2 | 1.0E3 | 8.0E2 | 1.7E0 | 1.7E0 | 1.2E4 | 3.7E3 | 363 | 26 | 141 | 26 | 0.57 |
| bA | ng/mL | 8.5E0 | 2.8E1 | 3.0E1 | 6.0E1 | 7.7E1 | 1.2E2 | 3.0E-2 | 1.4E0 | 9.4E2 | 6.2E2 | 363 | 26 | 141 | 26 | 0.69 |
| bB | ng/mL | 3.0E2 | 2.9E2 | 3.2E2 | 3.1E2 | 1.6E2 | 1.6E2 | 1.6E1 | 5.1E1 | 1.0E3 | 6.1E2 | 363 | 26 | 141 | 26 | 0.49 |
| bC | ng/mL | 3.3E2 | 4.2E2 | 5.7E2 | 8.0E2 | 7.3E2 | 1.1E3 | 2.7E1 | 7.3E1 | 4.7E3 | 4.7E3 | 363 | 26 | 141 | 26 | 0.60 |
| bE | mg/mL | 5.4E0 | 6.1E0 | 5.7E0 | 6.3E0 | 1.9E0 | 2.4E0 | 1.4E0 | 2.8E0 | 1.3E1 | 1.3E1 | 363 | 26 | 141 | 26 | 0.56 |
| bF | pg/mL | 2.0E1 | 3.3E1 | 1.8E2 | 5.4E1 | 1.1E3 | 6.4E1 | 5.0E-2 | 8.1E0 | 1.1E4 | 3.2E2 | 363 | 26 | 141 | 26 | 0.63 |
| bG | ng/mL | 1.6E0 | 2.0E0 | 2.8E0 | 3.2E0 | 3.4E0 | 3.1E0 | 2.2E-2 | 2.4E-1 | 2.6E1 | 1.0E1 | 363 | 26 | 141 | 26 | 0.55 |
| bH | pg/mL | 5.7E-1 | 5.7E-1 | 5.2E0 | 4.0E0 | 1.7E1 | 6.0E0 | 5.7E-1 | 5.7E-1 | 2.8E2 | 2.1E1 | 363 | 26 | 141 | 26 | 0.50 |
| bI | ng/mL | 4.0E-3 | 4.0E-3 | 7.0E-2 | 9.5E-2 | 1.7E-1 | 1.3E-1 | 4.0E-3 | 4.0E-3 | 1.3E0 | 3.4E-1 | 363 | 26 | 141 | 26 | 0.57 |
| bJ | mg/mL | 2.2E0 | 2.6E0 | 2.5E0 | 2.8E0 | 1.9E0 | 1.7E0 | 2.5E-4 | 2.5E-1 | 1.3E1 | 7.1E0 | 363 | 26 | 141 | 26 | 0.56 |
| bL | pg/mL | 4.1E0 | 2.6E0 | 8.4E0 | 7.9E0 | 1.0E1 | 1.1E1 | 4.6E-2 | 4.6E-2 | 4.9E1 | 4.4E1 | 363 | 26 | 141 | 26 | 0.46 |
| bM | mg/mL | 1.7E0 | 2.1E0 | 2.1E0 | 2.3E0 | 1.4E0 | 1.3E0 | 9.2E-3 | 6.3E-1 | 7.9E0 | 6.1E0 | 363 | 26 | 141 | 26 | 0.58 |
| bN | ng/mL | 4.7E1 | 2.8E1 | 1.3E2 | 7.0E1 | 2.7E2 | 1.2E2 | 1.4E-1 | 1.4E-1 | 1.9E3 | 5.0E2 | 363 | 26 | 141 | 26 | 0.41 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bO | ng/mL | 4.0E-2 | 4.0E-2 | 9.2E0 | 1.4E1 | 2.1E1 | 3.9E1 | 4.0E-2 | 4.0E-2 | 1.9E2 | 1.9E2 | 363 | 26 | 141 | 26 | 0.47 |
| bP | mg/mL | 5.1E-1 | 5.2E-1 | 7.3E-1 | 7.3E-1 | 6.6E-1 | 6.0E-1 | 8.2E-2 | 1.4E-1 | 4.8E0 | 2.9E0 | 363 | 26 | 141 | 26 | 0.52 |
| bQ | pg/mL | 1.5E1 | 1.9E1 | 7.0E1 | 2.6E1 | 7.1E2 | 3.0E1 | 1.5E-1 | 1.5E-1 | 1.3E4 | 1.5E2 | 363 | 26 | 141 | 26 | 0.55 |
| bR | ng/mL | 1.2E-2 | 1.2E-2 | 1.4E-1 | 6.2E-2 | 5.0E-1 | 1.0E-1 | 1.2E-2 | 1.2E-2 | 8.7E0 | 3.9E-1 | 363 | 26 | 141 | 26 | 0.41 |
| bS | pg/mL | 9.4E-1 | 9.4E-1 | 7.8E0 | 4.3E0 | 3.1E1 | 1.1E1 | 9.4E-1 | 9.4E-1 | 3.9E2 | 4.8E1 | 363 | 26 | 141 | 26 | 0.49 |
| bU | ng/mL | 1.3E-1 | 1.3E-2 | 2.1E-1 | 9.3E-2 | 4.1E-1 | 1.3E-1 | 1.3E-2 | 1.3E-2 | 6.6E0 | 5.5E-1 | 363 | 26 | 141 | 26 | 0.37 |
| bV | pg/mL | 4.7E2 | 5.7E2 | 5.3E2 | 6.3E2 | 2.4E2 | 3.3E2 | 1.7E2 | 1.5E2 | 1.6E3 | 1.6E3 | 363 | 26 | 141 | 26 | 0.59 |
| bW | pg/mL | 3.4E2 | 3.3E2 | 4.9E2 | 6.3E2 | 5.0E2 | 1.2E3 | 8.4E1 | 1.4E2 | 4.8E3 | 6.4E3 | 363 | 26 | 141 | 26 | 0.50 |
| bX | ng/mL | 1.5E-3 | 2.5E-5 | 2.8E-3 | 2.0E-3 | 3.4E-3 | 2.6E-3 | 2.5E-5 | 2.5E-5 | 2.0E-2 | 8.8E-3 | 363 | 26 | 141 | 26 | 0.45 |
| bZ | pg/mL | 2.4E2 | 2.7E2 | 9.1E2 | 4.4E2 | 4.3E3 | 4.5E2 | 1.5E-1 | 5.9E1 | 5.8E4 | 1.7E3 | 363 | 26 | 141 | 26 | 0.55 |
| cA | pg/mL | 6.0E-1 | 6.0E-1 | 3.0E0 | 1.3E0 | 2.0E1 | 2.5E0 | 6.0E-1 | 6.0E-1 | 3.7E2 | 1.1E1 | 363 | 26 | 141 | 26 | 0.47 |
| cB | ng/mL | 5.4E-2 | 3.3E-2 | 8.6E-2 | 4.3E-2 | 1.0E-1 | 3.6E-2 | 1.7E-3 | 1.7E-3 | 5.7E-1 | 1.2E-1 | 363 | 26 | 141 | 26 | 0.39 |
| cC | pg/mL | 4.6E1 | 3.5E1 | 4.9E1 | 3.6E1 | 4.2E1 | 2.6E1 | 1.0E0 | 1.0E0 | 4.5E2 | 1.1E2 | 363 | 26 | 141 | 26 | 0.40 |
| cD | pg/mL | 5.2E0 | 4.7E0 | 1.3E1 | 1.1E1 | 3.9E1 | 1.8E1 | 3.3E-1 | 3.3E-1 | 4.8E2 | 8.4E1 | 363 | 26 | 141 | 26 | 0.46 |
| cE | pg/mL | 3.2E1 | 4.0E1 | 1.5E2 | 7.8E1 | 4.8E2 | 1.0E2 | 1.2E-1 | 1.2E-1 | 3.8E3 | 4.2E2 | 363 | 26 | 141 | 26 | 0.55 |
| cF | pg/mL | 1.2E1 | 5.3E-1 | 2.1E1 | 1.1E1 | 3.4E1 | 1.8E1 | 5.3E-1 | 5.3E-1 | 2.7E2 | 7.2E1 | 363 | 26 | 141 | 26 | 0.41 |
| cG | pg/mL | 4.3E1 | 6.4E1 | 1.1E2 | 7.4E1 | 5.7E2 | 4.3E1 | 7.8E0 | 7.7E0 | 1.0E4 | 2.0E2 | 363 | 26 | 141 | 26 | 0.63 |
| cH | uIU/mL | 3.1E0 | 1.4E0 | 6.7E0 | 3.2E0 | 1.3E1 | 5.4E0 | 8.6E-3 | 8.6E-3 | 1.6E2 | 2.4E1 | 363 | 26 | 141 | 26 | 0.31 |
| cI | ng/mL | 5.6E0 | 4.7E0 | 1.1E1 | 1.4E1 | 1.5E1 | 2.4E1 | 1.0E-3 | 1.1E-1 | 9.4E1 | 1.2E2 | 363 | 26 | 141 | 26 | 0.50 |
| cJ | ug/mL | 6.5E1 | 4.6E1 | 1.2E2 | 7.4E1 | 1.5E2 | 8.3E1 | 4.0E0 | 7.6E0 | 9.6E2 | 3.9E2 | 363 | 26 | 141 | 26 | 0.43 |
| cK | ng/mL | 3.8E-3 | 3.8E-3 | 5.9E-2 | 1.5E-2 | 2.0E-1 | 4.0E-2 | 3.8E-3 | 3.8E-3 | 1.7E0 | 1.6E-1 | 363 | 26 | 141 | 26 | 0.46 |
| cL | pg/mL | 1.9E2 | 2.2E2 | 4.0E2 | 2.5E2 | 1.5E3 | 1.5E2 | 1.6E1 | 4.1E1 | 2.4E4 | 6.4E2 | 363 | 26 | 141 | 26 | 0.56 |
| cM | pg/mL | 2.8E2 | 2.4E2 | 3.1E2 | 2.4E2 | 2.1E2 | 9.0E1 | 8.7E0 | 6.7E1 | 1.6E3 | 4.7E2 | 363 | 26 | 141 | 26 | 0.39 |
| cN | pg/mL | 1.2E2 | 1.3E2 | 1.3E2 | 1.4E2 | 7.0E1 | 4.9E1 | 3.8E1 | 6.4E1 | 1.1E3 | 2.4E2 | 363 | 26 | 141 | 26 | 0.58 |
| cO | pg/mL | 2.2E2 | 2.5E2 | 3.3E2 | 2.7E2 | 1.0E3 | 1.5E2 | 5.4E1 | 1.1E2 | 1.9E4 | 7.5E2 | 363 | 26 | 141 | 26 | 0.54 |
| cP | ng/mL | 2.6E3 | 2.8E3 | 2.6E3 | 3.1E3 | 9.4E2 | 1.3E3 | 6.2E2 | 1.6E3 | 5.7E3 | 7.3E3 | 363 | 26 | 141 | 26 | 0.59 |
| cQ | ng/mL | 4.9E-2 | 6.3E-2 | 1.2E-1 | 1.9E-1 | 2.3E-1 | 3.1E-1 | 2.0E-3 | 2.0E-3 | 2.2E0 | 1.2E0 | 363 | 26 | 141 | 26 | 0.57 |
| cR | ng/mL | 2.8E2 | 4.3E2 | 4.3E2 | 8.1E2 | 6.0E2 | 1.5E3 | 2.0E1 | 4.0E1 | 7.7E3 | 7.7E3 | 363 | 26 | 141 | 26 | 0.61 |
| cS | ng/mL | 2.6E2 | 3.0E2 | 3.7E2 | 3.9E2 | 3.7E2 | 2.8E2 | 4.7E1 | 9.7E1 | 2.7E3 | 1.2E3 | 363 | 26 | 141 | 26 | 0.55 |
| cT | ng/mL | 3.1E1 | 5.6E1 | 8.6E1 | 1.0E2 | 1.9E2 | 1.5E2 | 4.6E0 | 4.2E0 | 2.1E3 | 6.5E2 | 363 | 26 | 141 | 26 | 0.63 |
| cU | ng/mL | 5.3E1 | 5.8E1 | 7.7E1 | 8.9E1 | 1.1E2 | 9.4E1 | 6.2E0 | 1.2E1 | 1.6E3 | 4.8E2 | 363 | 26 | 141 | 26 | 0.53 |
| cV | ng/mL | 1.7E-1 | 1.7E-1 | 4.2E-1 | 2.7E-1 | 2.5E0 | 2.6E-1 | 3.4E-4 | 4.7E-2 | 4.7E1 | 1.2E0 | 363 | 26 | 141 | 26 | 0.53 |
| cW | mIU/mL | 5.3E-2 | 5.6E-2 | 1.6E-1 | 7.6E-2 | 7.9E-1 | 6.3E-2 | 3.7E-4 | 1.1E-2 | 9.7E0 | 2.6E-1 | 363 | 26 | 141 | 26 | 0.52 |
| cX | ng/mL | 1.0E-1 | 3.1E-2 | 1.4E0 | 7.6E-1 | 4.5E0 | 1.5E0 | 2.3E-4 | 2.3E-4 | 2.8E1 | 6.9E0 | 363 | 26 | 141 | 26 | 0.47 |
| cY | ng/mL | 8.8E0 | 7.1E0 | 1.3E1 | 1.2E1 | 1.4E1 | 1.3E1 | 1.5E-1 | 3.1E-1 | 8.3E1 | 5.4E1 | 363 | 26 | 141 | 26 | 0.45 |
| cZ | ug/mL | 1.4E1 | 1.5E1 | 1.5E1 | 1.6E1 | 6.1E0 | 6.3E0 | 2.7E0 | 6.3E0 | 3.9E1 | 3.6E1 | 363 | 26 | 141 | 26 | 0.53 |
| dA | pg/mL | 3.3E2 | 3.7E2 | 3.7E2 | 3.9E2 | 3.3E2 | 1.7E2 | 9.0E1 | 1.7E2 | 5.8E3 | 8.2E2 | 363 | 26 | 141 | 26 | 0.56 |
| dB | ug/mL | 1.7E1 | 1.8E1 | 1.8E1 | 1.7E1 | 1.8E1 | 9.2E0 | 9.4E-1 | 1.9E0 | 2.5E2 | 4.1E1 | 363 | 26 | 141 | 26 | 0.53 |
| dC | nmol/L | 3.5E1 | 3.6E1 | 4.0E1 | 3.9E1 | 1.9E1 | 2.0E1 | 9.1E0 | 1.3E1 | 1.4E2 | 8.3E1 | 363 | 26 | 141 | 26 | 0.48 |
| dD | ug/mL | 3.6E1 | 3.3E1 | 3.7E1 | 3.5E1 | 1.1E1 | 9.6E0 | 1.3E1 | 1.5E1 | 7.6E1 | 5.8E1 | 363 | 26 | 141 | 26 | 0.43 |
| dE | ng/mL | 4.7E-1 | 5.4E-1 | 6.1E-1 | 7.8E-1 | 7.4E-1 | 7.7E-1 | 8.4E-3 | 8.4E-3 | 7.2E0 | 3.3E0 | 363 | 26 | 141 | 26 | 0.58 |
| dF | ng/mL | 2.2E2 | 3.1E2 | 2.7E2 | 3.5E2 | 1.8E2 | 2.0E2 | 7.5E1 | 8.3E1 | 1.3E3 | 8.6E2 | 363 | 26 | 141 | 26 | 0.67 |
| dG | ng/mL | 1.1E1 | 1.6E1 | 1.4E1 | 1.9E1 | 1.3E1 | 1.2E1 | 3.1E0 | 2.2E0 | 1.8E2 | 6.9E1 | 363 | 26 | 141 | 26 | 0.69 |
| dH | pg/mL | 7.5E0 | 7.8E0 | 1.3E1 | 1.1E1 | 4.1E1 | 8.0E0 | 4.0E-2 | 4.0E-2 | 6.7E2 | 3.5E1 | 363 | 26 | 141 | 26 | 0.56 |
| dI | pg/mL | 4.6E-1 | 4.6E-1 | 2.5E0 | 1.1E0 | 1.8E1 | 2.0E0 | 4.6E-1 | 4.6E-1 | 3.3E2 | 8.8E0 | 363 | 26 | 141 | 26 | 0.46 |
| dJ | ng/mL | 1.9E0 | 1.3E0 | 2.2E0 | 1.8E0 | 1.2E0 | 1.1E0 | 3.2E-2 | 4.0E-1 | 6.9E0 | 4.0E0 | 363 | 26 | 141 | 26 | 0.39 |
| dK | uIU/mL | 1.9E0 | 1.6E0 | 3.2E0 | 4.1E0 | 6.5E0 | 1.1E1 | 2.8E-4 | 3.8E-2 | 7.9E1 | 6.0E1 | 363 | 26 | 141 | 26 | 0.45 |
| dL | ng/mL | 8.8E2 | 1.1E3 | 1.0E3 | 1.1E3 | 4.9E2 | 3.8E2 | 3.4E2 | 3.3E2 | 3.4E3 | 1.8E3 | 363 | 26 | 141 | 26 | 0.61 |
| dM | pg/mL | 9.7E2 | 1.1E3 | 1.2E3 | 1.8E3 | 9.3E2 | 1.8E3 | 3.9E2 | 3.7E2 | 1.2E4 | 8.3E3 | 363 | 26 | 141 | 26 | 0.64 |
| dN | ug/mL | 9.4E1 | 9.6E1 | 9.9E1 | 1.1E2 | 3.4E1 | 4.2E1 | 2.5E1 | 3.7E1 | 2.4E2 | 2.0E2 | 363 | 26 | 141 | 26 | 0.56 |
| dO | ng/ml | 2.3E1 | 9.3E0 | 4.6E1 | 2.5E1 | 6.5E1 | 3.7E1 | 4.0E-1 | 3.8E0 | 3.7E2 | 1.1E2 | 65 | 8 | 47 | 8 | 0.31 |
| dR | pg/ml | 1.6E3 | 1.3E3 | 2.4E3 | 2.0E3 | 2.5E3 | 2.1E3 | 1.4E2 | 1.9E2 | 1.5E4 | 8.9E3 | 245 | 25 | 138 | 25 | 0.44 |
| dV | pg/ml | 7.1E1 | 6.0E1 | 9.0E1 | 7.2E1 | 5.5E1 | 4.8E1 | 3.4E1 | 3.6E1 | 2.7E2 | 1.9E2 | 46 | 8 | 27 | 8 | 0.34 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dX | ng/ml | 3.4E-2 | 7.4E-3 | 1.2E-1 | 1.4E-1 | 2.0E-1 | 2.8E-1 | 2.6E-3 | 2.6E-3 | 1.2E0 | 8.6E-1 | 92 | 9 | 30 | 9 | 0.43 |
| cF | ng/ml | 4.1E0 | 5.6E0 | 4.6E0 | 5.6E0 | 2.2E0 | 2.1E0 | 1.4E0 | 2.6E0 | 1.8E1 | 1.1E1 | 257 | 25 | 138 | 25 | 0.67 |
| eC | pg/ml | 3.1E2 | 2.7E2 | 3.7E2 | 3.3E2 | 2.2E2 | 2.3E2 | 4.5E1 | 1.6E2 | 1.4E3 | 1.2E3 | 203 | 18 | 135 | 18 | 0.42 |
| eM | ng/ml | 3.5E0 | 3.8E0 | 4.7E0 | 8.0E0 | 4.4E0 | 7.4E0 | 7.6E-1 | 1.8E0 | 2.4E1 | 2.5E1 | 122 | 9 | 50 | 9 | 0.65 |
| cP | ng/ml | 3.7E-3 | 3.1E-2 | 7.3E-1 | 7.5E0 | 1.8E0 | 2.2E1 | 3.7E-3 | 3.7E-3 | 1.2E1 | 6.5E1 | 92 | 9 | 30 | 9 | 0.57 |
| eX | ng/ml | 4.7E0 | 1.3E1 | 1.6E1 | 2.1E1 | 2.1E1 | 2.1E1 | 8.6E-4 | 1.8E0 | 7.3E1 | 4.9E1 | 65 | 8 | 47 | 8 | 0.63 |
| fP | ng/ml | 2.5E2 | 2.9E2 | 2.9E2 | 3.3E2 | 1.7E2 | 2.1E2 | 1.8E0 | 1.2E2 | 1.0E3 | 9.6E2 | 240 | 21 | 137 | 21 | 0.56 |
| fR | ng/ml | 1.2E5 | 2.0E5 | 1.7E5 | 2.6E5 | 1.4E5 | 1.9E5 | 3.1E4 | 2.9E4 | 7.2E5 | 8.3E5 | 236 | 17 | 68 | 17 | 0.69 |
| gC | ng/ml | 2.3E2 | 2.3E2 | 2.5E2 | 2.5E2 | 1.0E2 | 8.6E1 | 8.3E1 | 1.7E2 | 6.4E2 | 4.4E2 | 103 | 8 | 58 | 8 | 0.53 |
| gN | U/ml | 3.6E2 | 4.0E2 | 4.7E2 | 5.2E2 | 3.0E2 | 2.6E2 | 3.5E1 | 3.1E2 | 1.3E3 | 9.3E2 | 46 | 8 | 27 | 8 | 0.60 |
| gL | pg/ml | 6.3E4 | 7.9E4 | 6.9E4 | 8.7E4 | 2.8E4 | 3.2E4 | 1.1E4 | 3.8E4 | 1.8E5 | 1.6E5 | 245 | 25 | 138 | 25 | 0.67 |
| gP | U/ml | 2.8E2 | 2.3E2 | 2.8E2 | 2.3E2 | 1.1E2 | 8.4E1 | 1.2E1 | 7.9E1 | 1.1E3 | 3.9E2 | 253 | 25 | 138 | 25 | 0.34 |
| gT | ng/ml | 2.1E1 | 2.0E1 | 2.1E1 | 2.2E1 | 4.5E0 | 4.7E0 | 1.2E1 | 1.7E1 | 3.3E1 | 3.0E1 | 49 | 8 | 33 | 8 | 0.54 |
| gW | ng/ml | 5.7E2 | 5.2E2 | 1.2E3 | 1.2E3 | 1.7E3 | 2.0E3 | 3.1E-1 | 8.3E1 | 9.5E3 | 8.2E3 | 213 | 25 | 129 | 25 | 0.50 |
| tF | pg/mL | 1.5E3 | 1.5E3 | 1.6E4 | 1.0E4 | 4.7E4 | 2.2E4 | 1.2E1 | 1.8E1 | 3.2E5 | 9.4E4 | 203 | 18 | 135 | 18 | 0.51 |
| iA | pg/ml | 1.5E2 | 2.0E2 | 3.0E2 | 3.5E2 | 6.6E2 | 5.0E2 | 1.1E1 | 8.2E0 | 7.1E3 | 2.2E3 | 203 | 18 | 135 | 18 | 0.60 |
| iH | ng/ml | 1.6E5 | 1.9E5 | 1.5E5 | 1.7E5 | 4.7E4 | 5.2E4 | 5.1E4 | 7.8E4 | 2.7E5 | 2.5E5 | 203 | 18 | 135 | 18 | 0.63 |
| iJ | ng/ml | 5.3E4 | 5.1E4 | 5.5E4 | 5.8E4 | 3.0E4 | 2.2E4 | 7.7E3 | 2.3E4 | 2.5E5 | 9.7E4 | 203 | 18 | 135 | 18 | 0.55 |
| hB | ng/ml | 4.3E-1 | 4.6E-1 | 5.4E-1 | 5.8E-1 | 4.1E-1 | 4.4E-1 | 1.2E-1 | 1.5E-1 | 3.0E0 | 2.0E0 | 203 | 18 | 135 | 18 | 0.52 |
| hC | pg/ml | 4.1E3 | 3.2E3 | 7.1E3 | 6.8E3 | 1.0E4 | 7.2E3 | 1.0E-9 | 1.0E-9 | 1.1E5 | 2.5E4 | 203 | 18 | 135 | 18 | 0.49 |
| hF | ng/ml | 1.0E-9 | 1.0E-9 | 2.4E1 | 1.0E-9 | 2.9E2 | 0.0E0 | 1.0E-9 | 1.0E-9 | 4.0E3 | 1.0E-9 | 203 | 18 | 135 | 18 | 0.49 |
| hG | pg/ml | 7.0E3 | 6.7E3 | 7.7E3 | 8.2E3 | 3.3E3 | 3.9E3 | 1.7E3 | 3.6E3 | 2.0E4 | 1.7E4 | 203 | 18 | 135 | 18 | 0.51 |
| iO | ng/ml | 3.8E5 | 3.5E5 | 4.0E5 | 3.6E5 | 1.8E5 | 1.2E5 | 8.3E4 | 1.4E5 | 1.1E6 | 5.4E5 | 203 | 18 | 135 | 18 | 0.46 |
| iP | ng/ml | 5.1E4 | 4.7E4 | 5.7E4 | 6.3E4 | 5.5E4 | 4.5E4 | 2.4E3 | 5.6E3 | 5.5E5 | 1.6E5 | 203 | 18 | 135 | 18 | 0.52 |
| iZ | ng/ml | 1.6E3 | 2.0E3 | 1.8E3 | 2.4E3 | 7.6E2 | 1.3E3 | 4.7E2 | 9.8E2 | 5.7E3 | 6.5E3 | 201 | 18 | 133 | 18 | 0.67 |
| kQ | pg/ml | 4.3E3 | 4.8E3 | 5.0E3 | 6.0E3 | 2.9E3 | 4.8E3 | 5.6E2 | 1.6E3 | 2.5E4 | 2.1E4 | 203 | 18 | 135 | 18 | 0.51 |
| kR | pg/ml | 2.3E1 | 2.2E1 | 3.3E1 | 2.4E1 | 7.3E1 | 1.9E1 | 1.0E-9 | 1.2E0 | 1.0E3 | 7.1E1 | 203 | 18 | 135 | 18 | 0.44 |
| kS | pg/ml | 8.0E2 | 7.0E2 | 9.6E2 | 9.7E2 | 1.1E3 | 8.1E2 | 8.2E1 | 7.9E1 | 1.4E4 | 2.9E3 | 203 | 18 | 135 | 18 | 0.47 |
| nW | pg/ml | 1.1E5 | 1.1E5 | 1.1E5 | 1.2E5 | 2.5E4 | 3.6E4 | 5.8E4 | 7.2E4 | 1.8E5 | 2.3E5 | 203 | 18 | 135 | 18 | 0.49 |
| nY | pg/ml | 2.1E3 | 2.1E3 | 2.4E3 | 2.4E3 | 1.6E3 | 1.4E3 | 5.1E2 | 5.4E2 | 1.3E4 | 5.8E3 | 203 | 18 | 135 | 18 | 0.51 |
| oE | pg/ml | 1.5E2 | 1.5E2 | 3.8E2 | 4.7E2 | 5.5E2 | 8.7E2 | 1.0E-9 | 2.0E1 | 4.7E3 | 3.7E3 | 203 | 18 | 135 | 18 | 0.51 |
| oF | pg/ml | 8.5E3 | 1.6E4 | 2.2E4 | 3.1E4 | 3.6E4 | 4.4E4 | 6.4E1 | 4.8E2 | 2.5E5 | 1.8E5 | 203 | 18 | 135 | 18 | 0.58 |
| oH | pg/ml | 4.1E1 | 4.6E1 | 9.4E1 | 1.0E2 | 1.4E2 | 1.7E2 | 4.2E0 | 9.1E0 | 9.9E2 | 6.3E2 | 203 | 18 | 135 | 18 | 0.51 |
| oK | pg/ml | 7.6E2 | 7.8E2 | 1.9E3 | 1.7E3 | 2.6E3 | 2.0E3 | 5.2E1 | 3.1E2 | 1.8E4 | 7.9E3 | 203 | 18 | 135 | 18 | 0.55 |
| oN | pg/ml | 5.1E2 | 5.6E2 | 7.9E2 | 6.0E2 | 1.5E3 | 2.9E2 | 1.5E2 | 2.4E2 | 1.8E4 | 1.2E3 | 203 | 18 | 135 | 18 | 0.52 |
| pF | pg/ml | 4.8E-1 | 4.1E-1 | 1.1E0 | 1.0E0 | 6.1E0 | 2.3E0 | 1.0E-9 | 1.0E-9 | 8.7E1 | 1.0E1 | 203 | 18 | 135 | 18 | 0.44 |

Figure 12.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 6.4E1 | 7.2E1 | 7.5E1 | 9.0E1 | 5.1E1 | 6.1E1 | 2.0E0 | 1.0E1 | 4.4E2 | 2.7E2 | 965 | 60 | 171 | 60 | 0.58 |
| Ad | ug/mL | 2.5E-2 | 9.1E-2 | 5.8E-2 | 1.1E-1 | 7.7E-2 | 1.0E-1 | 6.8E-4 | 1.9E-3 | 3.7E-1 | 3.6E-1 | 255 | 48 | 103 | 48 | 0.67 |
| Af | ng/mL | 9.2E-1 | 1.3E0 | 1.8E1 | 2.9E1 | 7.2E1 | 7.8E1 | 1.7E-3 | 1.7E-3 | 5.3E2 | 4.0E2 | 255 | 48 | 103 | 48 | 0.57 |
| Aj | ug/mL | 1.4E0 | 4.8E0 | 2.6E0 | 3.5E0 | 2.4E0 | 2.6E0 | 1.5E-3 | 4.0E-3 | 6.1E0 | 6.1E0 | 255 | 48 | 103 | 48 | 0.59 |
| Al | mg/mL | 9.0E-5 | 1.2E-4 | 2.5E-4 | 2.5E-4 | 4.2E-4 | 4.0E-4 | 2.5E-6 | 9.0E-6 | 1.9E-3 | 1.9E-3 | 255 | 48 | 103 | 48 | 0.54 |
| An | U/mL | 4.0E1 | 7.2E1 | 1.5E2 | 2.5E2 | 4.7E2 | 4.8E2 | 9.8E-4 | 7.7E-1 | 5.5E3 | 3.0E3 | 255 | 48 | 103 | 48 | 0.64 |
| Ao | pg/mL | 8.5E1 | 1.1E2 | 2.1E2 | 2.3E2 | 1.0E3 | 5.4E2 | 1.5E0 | 1.2E1 | 1.6E4 | 3.8E3 | 255 | 48 | 103 | 48 | 0.60 |
| Ap | ng/mL | 2.7E1 | 4.5E1 | 4.3E1 | 5.9E1 | 5.0E1 | 5.1E1 | 9.9E-1 | 8.4E-5 | 3.3E2 | 2.5E2 | 255 | 48 | 103 | 48 | 0.64 |
| Ar | ng/mL | 6.5E-1 | 1.3E0 | 2.0E0 | 4.6E0 | 4.1E0 | 7.8E0 | 3.4E-3 | 3.4E-3 | 4.3E1 | 2.9E1 | 255 | 48 | 103 | 48 | 0.61 |
| As | ng/mL | 8.7E-3 | 7.3E-3 | 1.2E-2 | 1.4E-2 | 1.6E-2 | 2.2E-2 | 1.7E-3 | 1.7E-3 | 1.1E-1 | 1.2E-1 | 255 | 48 | 103 | 48 | 0.49 |
| Aw | pg/mL | 1.6E1 | 1.7E1 | 1.6E1 | 1.8E1 | 5.4E0 | 5.8E0 | 3.5E0 | 9.4E0 | 3.3E1 | 3.8E1 | 255 | 48 | 103 | 48 | 0.63 |
| Ax | ng/mL | 2.1E0 | 2.3E0 | 8.2E0 | 1.2E1 | 1.8E1 | 2.3E1 | 1.9E-2 | 4.9E-2 | 1.5E2 | 1.3E2 | 255 | 48 | 103 | 48 | 0.56 |
| Ba | ng/mL | 3.7E1 | 1.7E2 | 2.8E2 | 5.7E2 | 7.3E2 | 1.0E3 | 3.7E-1 | 2.7E-1 | 8.1E3 | 4.5E3 | 255 | 48 | 103 | 48 | 0.65 |
| Bb | ng/mL | 2.6E0 | 4.8E0 | 5.3E0 | 6.4E0 | 8.5E0 | 5.4E0 | 4.1E-3 | 4.1E-3 | 6.6E1 | 2.0E1 | 255 | 48 | 103 | 48 | 0.62 |
| Bc | ng/mL | 3.3E1 | 4.3E1 | 9.7E1 | 1.0E2 | 1.8E2 | 2.0E2 | 1.1E-1 | 2.2E-1 | 1.0E3 | 1.0E3 | 255 | 48 | 103 | 48 | 0.53 |
| Bg | ng/mL | 6.4E-2 | 2.9E-1 | 4.4E0 | 1.7E0 | 2.2E1 | 3.8E0 | 5.3E-4 | 5.3E-4 | 2.5E2 | 2.1E1 | 255 | 48 | 103 | 48 | 0.62 |
| Bn | ng/mL | 5.6E-2 | 4.2E-1 | 8.5E-1 | 2.1E0 | 1.6E0 | 2.6E0 | 5.6E-2 | 5.6E-2 | 8.5E0 | 7.6E0 | 255 | 48 | 103 | 48 | 0.62 |
| Bo | ng/mL | 1.1E1 | 1.4E1 | 1.3E1 | 1.6E1 | 9.6E0 | 1.4E1 | 1.6E-2 | 1.6E-2 | 7.4E1 | 4.6E1 | 255 | 48 | 103 | 48 | 0.55 |
| Ch | uIU/mL | 9.0E-1 | 1.9E0 | 6.5E0 | 1.9E1 | 2.1E1 | 4.2E1 | 3.4E-3 | 3.4E-3 | 2.3E2 | 1.9E2 | 255 | 48 | 103 | 48 | 0.62 |
| Co | pg/mL | 3.1E1 | 3.9E1 | 9.1E1 | 1.2E2 | 2.1E2 | 3.0E2 | 3.9E0 | 1.5E-1 | 1.9E3 | 2.1E3 | 255 | 48 | 103 | 48 | 0.60 |
| Cp | ng/mL | 1.9E1 | 2.8E1 | 2.5E1 | 3.6E1 | 2.5E1 | 2.9E1 | 6.0E-1 | 6.0E-1 | 2.9E2 | 1.9E2 | 255 | 48 | 103 | 48 | 0.70 |
| Cq | ng/mL | 2.4E-2 | 3.1E-2 | 1.6E-1 | 6.1E-2 | 1.1E0 | 9.4E-2 | 8.0E-4 | 8.0E-4 | 1.7E1 | 5.2E-1 | 255 | 48 | 103 | 48 | 0.57 |
| Cs | ng/mL | 4.5E1 | 7.7E1 | 2.1E2 | 2.9E2 | 4.0E2 | 5.4E2 | 2.7E-2 | 1.3E0 | 2.9E3 | 3.0E3 | 255 | 48 | 103 | 48 | 0.55 |
| Ct | ng/mL | 1.2E0 | 7.6E-1 | 2.6E1 | 3.8E1 | 8.2E1 | 9.8E1 | 1.1E-4 | 1.1E-4 | 6.2E2 | 4.7E2 | 255 | 48 | 103 | 48 | 0.50 |
| Cu | ng/mL | 2.1E-1 | 3.0E-1 | 4.2E-1 | 3.5E-1 | 9.2E-1 | 2.1E-1 | 9.6E-3 | 9.0E-5 | 9.2E0 | 8.6E-1 | 255 | 48 | 103 | 48 | 0.60 |
| Cv | ng/mL | 4.6E0 | 9.4E0 | 2.4E1 | 3.1E1 | 7.0E1 | 6.3E1 | 5.6E-3 | 2.6E-2 | 5.3E2 | 3.1E2 | 255 | 48 | 103 | 48 | 0.58 |
| Cw | mIU/mL | 2.8E-2 | 3.2E-2 | 3.8E-2 | 4.1E-2 | 3.0E-2 | 2.9E-2 | 2.3E-3 | 4.8E-3 | 1.9E-1 | 1.4E-1 | 255 | 48 | 103 | 48 | 0.54 |
| Cx | ng/mL | 1.8E-1 | 4.2E-1 | 5.4E1 | 9.6E1 | 1.0E2 | 1.3E2 | 9.3E-5 | 9.3E-5 | 3.9E2 | 3.9E2 | 255 | 48 | 103 | 48 | 0.56 |
| Db | ug/mL | 8.2E0 | 6.8E0 | 9.4E0 | 8.0E0 | 8.8E0 | 7.4E0 | 5.3E-1 | 5.0E-1 | 1.0E2 | 3.6E1 | 255 | 48 | 103 | 48 | 0.43 |
| Dc | nmol/L | 1.8E-2 | 2.8E-2 | 5.7E-2 | 5.9E-2 | 1.3E-1 | 8.4E-2 | 5.2E-6 | 3.0E-4 | 1.2E0 | 4.0E-1 | 255 | 48 | 103 | 48 | 0.56 |
| Dd | ug/mL | 6.9E-2 | 9.0E-2 | 1.8E-1 | 2.4E-1 | 2.6E-1 | 3.8E-1 | 1.9E-4 | 8.3E-5 | 1.6E0 | 1.9E0 | 255 | 48 | 103 | 48 | 0.52 |
| De | ng/mL | 3.4E-3 | 3.4E-3 | 5.6E-2 | 1.1E-1 | 1.1E-1 | 1.5E-1 | 3.4E-3 | 3.4E-3 | 5.9E-1 | 6.2E-1 | 255 | 48 | 103 | 48 | 0.60 |
| Dg | ng/mL | 2.5E1 | 5.0E1 | 3.9E1 | 6.3E1 | 3.8E1 | 5.0E1 | 2.8E-1 | 1.0E-1 | 1.9E2 | 1.9E2 | 255 | 48 | 103 | 48 | 0.64 |
| Di | pg/mL | 1.6E0 | 1.7E0 | 2.0E0 | 2.5E0 | 1.8E0 | 2.3E0 | 1.8E-1 | 1.8E-1 | 1.3E1 | 7.8E0 | 255 | 48 | 103 | 48 | 0.54 |
| Dk | uIU/mL | 1.4E-2 | 2.5E-2 | 9.3E-2 | 4.4E-2 | 6.3E-1 | 6.2E-2 | 1.1E-4 | 1.1E-4 | 8.9E0 | 3.4E-1 | 255 | 48 | 103 | 48 | 0.61 |
| Dl | ng/mL | 2.0E2 | 3.4E2 | 2.7E2 | 4.4E2 | 2.4E2 | 3.5E2 | 3.1E0 | 2.5E0 | 1.4E3 | 1.3E3 | 255 | 48 | 103 | 48 | 0.64 |
| Do | ng/ml | 4.7E-1 | 9.5E-1 | 1.1E0 | 9.0E-1 | 2.9E0 | 1.1E0 | 3.6E-2 | 3.6E-2 | 1.9E1 | 3.7E0 | 55 | 11 | 37 | 11 | 0.58 |
| Dp | ng/ml | 2.5E0 | 2.2E0 | 4.9E0 | 5.3E0 | 6.8E0 | 7.0E0 | 3.7E-3 | 5.3E-3 | 4.3E1 | 3.5E1 | 122 | 47 | 99 | 47 | 0.49 |
| Dr | pg/ml | 3.4E1 | 1.3E1 | 5.7E1 | 3.5E1 | 6.6E1 | 4.7E1 | 7.5E-1 | 7.5E-1 | 2.9E2 | 1.6E2 | 99 | 25 | 50 | 25 | 0.39 |
| Dq | Absorbance | 1.7E-3 | 1.7E-3 | 4.1E-2 | 2.5E-3 | 1.5E-1 | 2.6E-3 | 1.7E-3 | 1.7E-3 | 8.3E-1 | 1.1E-2 | 55 | 11 | 37 | 11 | 0.38 |
| Du | pg/ml | 4.2E1 | 1.2E0 | 5.0E2 | 1.7E3 | 1.3E3 | 6.0E3 | 1.2E0 | 1.2E0 | 7.0E3 | 2.6E4 | 53 | 19 | 42 | 19 | 0.46 |
| Ef | ng/ml | 9.5E-2 | 3.1E-1 | 6.9E-1 | 1.1E0 | 1.7E0 | 1.9E0 | 5.7E-4 | 5.7E-4 | 1.0E1 | 8.6E0 | 165 | 48 | 102 | 48 | 0.62 |
| Wm | % | 8.2E-1 | 8.0E-1 | 3.0E1 | 5.4E1 | 1.9E2 | 2.1E2 | 5.4E-2 | 8.5E-2 | 2.4E3 | 9.6E2 | 204 | 53 | 119 | 53 | 0.51 |
| Ed | pg/ml | 5.2E-1 | 5.2E-1 | 9.3E1 | 1.7E1 | 6.6E2 | 4.0E1 | 5.2E-1 | 5.2E-1 | 7.3E3 | 1.6E2 | 122 | 47 | 98 | 47 | 0.38 |
| Yf | ng/mL | 1.6E1 | 1.8E1 | 1.4E2 | 3.4E1 | 8.4E2 | 3.5E1 | 2.9E-1 | 2.2E0 | 6.6E3 | 1.2E2 | 61 | 19 | 50 | 19 | 0.53 |
| Tj | pg/mL | 3.7E-1 | 3.7E-1 | 7.6E1 | 5.0E0 | 3.7E2 | 1.2E1 | 3.6E-1 | 3.6E-1 | 3.5E3 | 5.2E1 | 165 | 49 | 103 | 49 | 0.40 |
| Po | pg/ml | 1.3E-1 | 2.5E0 | 8.0E0 | 1.1E1 | 2.7E1 | 2.8E1 | 8.0E-3 | 2.6E-2 | 2.7E2 | 2.2E2 | 359 | 75 | 189 | 75 | 0.61 |
| Ti | ug/mL | 2.8E0 | 5.2E0 | 4.1E0 | 5.7E0 | 3.7E0 | 4.9E0 | 1.2E-1 | 8.7E-3 | 1.6E1 | 1.7E1 | 86 | 32 | 69 | 32 | 0.58 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Em | ng/ml | 2.9E-3 | 6.1E-3 | 5.3E-2 | 4.5E-2 | 1.1E-1 | 9.6E-2 | 2.8E-16 | 1.9E-16 | 5.4E-1 | 4.7E-1 | 123 | 26 | 51 | 26 | 0.48 |
| Et | ng/ml | 1.0E3 | 2.1E3 | 1.3E3 | 2.2E3 | 1.0E3 | 1.3E3 | 7.5E1 | 1.5E2 | 4.9E3 | 5.0E3 | 359 | 75 | 189 | 75 | 0.69 |
| Eq | pg/ml | 1.3E2 | 2.5E2 | 2.7E2 | 3.4E2 | 3.8E2 | 4.3E2 | 1.0E0 | 1.0E0 | 1.8E3 | 1.8E3 | 53 | 19 | 42 | 19 | 0.56 |
| Th | ug/mL | 1.1E0 | 1.1E0 | 1.5E0 | 1.5E0 | 1.6E0 | 1.0E0 | 2.6E-3 | 6.5E-2 | 1.2E1 | 4.6E0 | 86 | 32 | 69 | 32 | 0.54 |
| Fa | ng/ml | 3.9E1 | 4.2E1 | 1.2E2 | 8.1E1 | 7.3E2 | 1.3E2 | 3.4E-2 | 2.6E-1 | 8.0E3 | 7.5E2 | 120 | 47 | 98 | 47 | 0.56 |
| Ez | ng/ml | 3.7E0 | 7.0E0 | 1.6E1 | 3.3E1 | 3.9E1 | 1.1E2 | 1.3E-2 | 1.3E-2 | 3.0E2 | 7.1E2 | 122 | 47 | 99 | 47 | 0.57 |
| Fb | ng/ml | 2.3E1 | 2.6E1 | 2.1E1 | 2.4E1 | 1.2E1 | 1.1E1 | 1.0E0 | 5.9E-1 | 5.7E1 | 4.0E1 | 120 | 47 | 98 | 47 | 0.57 |
| Ex | ng/ml | 6.0E-2 | 8.2E-2 | 2.4E-1 | 1.3E-1 | 8.4E-1 | 1.5E-1 | 3.5E-5 | 1.5E-4 | 8.9E0 | 5.5E-1 | 126 | 33 | 69 | 33 | 0.55 |
| Fc | pg/ml | 2.2E-1 | 2.2E-1 | 2.9E2 | 9.5E-1 | 2.0E3 | 3.2E0 | 2.2E-1 | 2.2E-1 | 1.5E4 | 1.4E1 | 53 | 19 | 42 | 19 | 0.41 |
| Fd | pg/ml | 2.9E1 | 9.8E-1 | 9.7E2 | 8.2E2 | 4.6E3 | 2.0E3 | 9.8E-1 | 9.8E-1 | 3.3E4 | 8.2E3 | 53 | 19 | 42 | 19 | 0.47 |
| Fi | pg/ml | 2.5E-1 | 2.5E-1 | 3.3E2 | 5.5E0 | 1.9E3 | 2.3E1 | 2.5E-1 | 2.5E-1 | 1.4E4 | 1.0E2 | 53 | 19 | 42 | 19 | 0.40 |
| Fn | ng/ml | 2.1E-1 | 2.1E-1 | 8.5E0 | 2.7E0 | 3.8E1 | 4.9E0 | 1.1E-14 | 2.1E-1 | 4.2E2 | 2.1E1 | 122 | 47 | 99 | 47 | 0.45 |
| Fp | ng/ml | 9.4E0 | 2.1E1 | 2.0E1 | 3.7E1 | 2.6E1 | 3.6E1 | 6.0E-3 | 2.8E-1 | 1.4E2 | 1.2E2 | 380 | 76 | 189 | 76 | 0.64 |
| Fr | ng/ml | 2.5E4 | 5.0E4 | 9.9E4 | 1.6E5 | 1.7E5 | 2.2E5 | 6.4E2 | 7.8E2 | 8.4E5 | 8.5E5 | 464 | 78 | 193 | 78 | 0.63 |
| Fw | pg/ml | 7.1E-1 | 3.9E-1 | 1.0E2 | 8.9E0 | 6.7E2 | 2.0E1 | 1.1E-14 | 1.7E-14 | 6.9E3 | 1.1E2 | 167 | 48 | 103 | 48 | 0.46 |
| Fy | ng/ml | 3.1E1 | 4.4E1 | 4.9E1 | 7.0E1 | 5.5E1 | 6.1E1 | 1.2E-1 | 2.7E0 | 3.3E2 | 2.1E2 | 121 | 46 | 98 | 46 | 0.62 |
| Gh | pg/ml | 2.3E0 | 1.1E1 | 2.5E1 | 1.0E2 | 5.0E1 | 3.0E2 | 2.9E-2 | 2.9E-2 | 2.7E2 | 1.2E3 | 53 | 18 | 42 | 18 | 0.54 |
| Gb | % | 3.2E1 | 3.9E1 | 4.3E1 | 4.4E1 | 3.9E1 | 3.2E1 | 2.7E0 | 4.6E0 | 2.3E2 | 1.0E2 | 53 | 19 | 41 | 19 | 0.52 |
| Gc | ng/ml | 9.9E1 | 7.7E1 | 1.4E2 | 1.7E2 | 1.4E2 | 2.1E2 | 6.4E0 | 9.7E0 | 7.9E2 | 9.2E2 | 104 | 27 | 52 | 27 | 0.49 |
| Gd | ng/ml | 2.9E1 | 2.8E1 | 3.0E1 | 3.2E1 | 1.6E1 | 2.1E1 | 5.4E0 | 6.3E0 | 8.1E1 | 8.1E1 | 119 | 24 | 50 | 24 | 0.50 |
| Gn | U/ml | 5.0E-1 | 1.8E-1 | 1.7E0 | 8.6E-1 | 3.7E0 | 2.4E0 | 1.3E-3 | 1.3E-3 | 3.0E1 | 1.2E1 | 95 | 25 | 50 | 25 | 0.38 |
| Gl | pg/ml | 6.1E3 | 1.0E4 | 9.8E3 | 1.2E4 | 9.0E3 | 9.0E3 | 2.3E2 | 6.3E2 | 3.4E4 | 3.3E4 | 162 | 48 | 102 | 48 | 0.60 |
| Gp | U/ml | 1.8E0 | 1.1E0 | 4.4E0 | 4.5E0 | 7.6E0 | 8.5E0 | 1.3E-3 | 1.3E-3 | 6.7E1 | 4.8E1 | 167 | 48 | 103 | 48 | 0.45 |
| Gt | ng/ml | 2.2E-3 | 2.2E-3 | 9.4E-2 | 1.3E-2 | 2.1E-1 | 1.9E-2 | 2.2E-3 | 2.2E-3 | 1.1E0 | 5.0E-2 | 48 | 7 | 31 | 7 | 0.43 |
| Gw | ng/ml | 6.7E0 | 5.7E0 | 2.0E1 | 7.6E0 | 6.1E1 | 6.9E0 | 8.3E-1 | 8.3E-1 | 4.4E2 | 2.6E1 | 55 | 11 | 37 | 11 | 0.49 |
| Gz | ug/ml | 1.2E0 | 1.0E0 | 1.1E1 | 5.5E0 | 5.3E1 | 6.2E0 | 2.9E-16 | 1.3E-1 | 4.8E2 | 2.1E1 | 81 | 31 | 64 | 31 | 0.50 |
| Ha | ng/ml | 2.7E0 | 2.6E0 | 8.4E0 | 7.5E0 | 1.9E1 | 1.3E1 | 1.7E-2 | 1.7E-2 | 1.0E2 | 6.7E1 | 120 | 47 | 98 | 47 | 0.50 |
| Nm | pg/ml | 1.2E4 | 2.6E4 | 2.4E4 | 3.9E4 | 4.9E4 | 4.4E4 | 1.0E-9 | 1.0E-9 | 7.8E5 | 1.9E5 | 358 | 75 | 190 | 75 | 0.62 |
| Nn | pg/ml | 1.3E2 | 2.0E2 | 2.5E3 | 2.3E3 | 1.1E4 | 9.3E3 | 1.0E-9 | 1.0E-9 | 1.0E5 | 6.9E4 | 358 | 75 | 190 | 75 | 0.58 |
| No | pg/ml | 1.3E1 | 2.4E1 | 2.7E1 | 5.2E1 | 5.9E1 | 1.7E2 | 1.0E-9 | 2.4E-1 | 5.9E2 | 1.4E3 | 358 | 75 | 190 | 75 | 0.59 |
| Nq | pg/ml | 2.2E0 | 1.4E0 | 2.1E1 | 1.6E1 | 8.4E1 | 4.1E1 | 1.0E-9 | 1.0E-9 | 1.2E3 | 2.6E2 | 358 | 75 | 190 | 75 | 0.48 |
| Nr | pg/ml | 4.7E-1 | 1.7E0 | 1.6E1 | 1.3E2 | 7.0E1 | 9.9E2 | 1.0E-9 | 1.0E-9 | 9.8E2 | 8.5E3 | 358 | 75 | 190 | 75 | 0.58 |
| Ns | pg/ml | 1.0E-9 | 1.0E-9 | 8.9E0 | 3.3E0 | 4.5E1 | 1.8E1 | 1.0E-9 | 1.0E-9 | 6.8E2 | 1.2E2 | 358 | 75 | 190 | 75 | 0.48 |
| Nt | pg/ml | 9.4E1 | 1.5E2 | 1.2E2 | 1.7E2 | 9.2E1 | 1.1E2 | 1.0E-9 | 1.5E1 | 5.9E2 | 6.8E2 | 358 | 75 | 190 | 75 | 0.66 |
| Nu | pg/ml | 1.7E1 | 2.8E1 | 4.9E1 | 7.2E1 | 8.8E1 | 9.8E1 | 1.0E-9 | 1.0E-9 | 8.9E2 | 5.8E2 | 358 | 75 | 190 | 75 | 0.60 |
| Lu | pg/ml | 1.0E4 | 1.0E4 | 1.5E4 | 1.1E4 | 3.2E4 | 1.1E4 | 6.7E2 | 7.1E2 | 3.9E5 | 8.4E4 | 360 | 76 | 190 | 76 | 0.50 |
| Lv | pg/ml | 1.0E-9 | 1.0E-9 | 1.1E1 | 1.8E1 | 2.2E1 | 3.0E1 | 1.0E-9 | 1.0E-9 | 2.4E2 | 1.6E2 | 360 | 76 | 190 | 76 | 0.56 |
| Lw | pg/ml | 1.0E-9 | 1.0E-9 | 1.4E-1 | 4.2E-1 | 1.8E0 | 2.5E0 | 1.0E-9 | 1.0E-9 | 3.2E1 | 1.7E1 | 360 | 76 | 190 | 76 | 0.51 |
| Lx | pg/ml | 1.0E-9 | 1.1E1 | 1.1E2 | 2.9E2 | 4.2E2 | 1.2E3 | 1.0E-9 | 1.0E-9 | 6.2E3 | 1.0E4 | 360 | 76 | 190 | 76 | 0.60 |
| Ly | pg/ml | 1.0E-9 | 1.0E-9 | 9.1E0 | 1.7E1 | 1.8E1 | 2.6E1 | 1.0E-9 | 1.0E-9 | 1.2E2 | 1.2E2 | 360 | 76 | 190 | 76 | 0.56 |
| Lz | pg/ml | 1.0E-9 | 1.0E-9 | 2.9E0 | 3.3E0 | 2.4E1 | 2.4E1 | 1.0E-9 | 1.0E-9 | 4.3E2 | 2.1E2 | 360 | 76 | 190 | 76 | 0.49 |
| Ma | pg/ml | 2.4E2 | 4.0E2 | 1.5E3 | 1.1E3 | 4.7E3 | 1.9E3 | 1.0E-9 | 1.0E-9 | 6.5E4 | 9.5E3 | 360 | 76 | 190 | 76 | 0.56 |
| Mb | pg/ml | 2.5E1 | 2.9E1 | 3.1E1 | 3.7E1 | 1.4E1 | 1.8E1 | 5.4E0 | 1.4E1 | 9.3E1 | 8.7E1 | 360 | 76 | 190 | 76 | 0.61 |
| Mc | pg/ml | 1.0E-9 | 1.0E-9 | 1.7E-2 | 1.0E-9 | 2.3E-1 | 0.0E0 | 1.0E-9 | 1.0E-9 | 4.0E0 | 1.0E-9 | 360 | 76 | 190 | 76 | 0.50 |
| Md | pg/ml | 1.0E-9 | 1.0E-9 | 6.2E-1 | 5.8E-1 | 5.2E0 | 2.6E0 | 1.0E-9 | 1.0E-9 | 6.8E1 | 1.6E1 | 360 | 76 | 190 | 76 | 0.50 |
| Me | pg/ml | 3.2E1 | 2.5E1 | 2.9E1 | 2.5E1 | 1.6E1 | 1.8E1 | 1.0E-9 | 6.1E-1 | 1.2E2 | 7.9E1 | 360 | 76 | 190 | 76 | 0.41 |
| Mf | pg/ml | 1.0E-9 | 1.0E-9 | 3.7E-1 | 5.3E-1 | 1.6E0 | 1.7E0 | 1.0E-9 | 1.0E-9 | 2.0E1 | 8.4E0 | 360 | 76 | 190 | 76 | 0.54 |
| Mg | pg/ml | 1.9E0 | 3.1E0 | 6.4E0 | 1.0E1 | 1.1E1 | 1.3E1 | 1.0E-9 | 1.0E-9 | 8.8E1 | 5.9E1 | 360 | 76 | 190 | 76 | 0.58 |
| Mh | pg/ml | 1.0E-9 | 1.0E-9 | 1.1E0 | 1.3E0 | 8.4E0 | 5.6E0 | 1.0E-9 | 1.0E-9 | 1.1E2 | 3.8E1 | 360 | 76 | 190 | 76 | 0.49 |
| Mi | pg/ml | 1.0E-9 | 1.0E-9 | 6.9E-1 | 3.1E0 | 7.1E0 | 1.9E1 | 1.0E-9 | 1.0E-9 | 1.2E2 | 1.6E2 | 360 | 76 | 190 | 76 | 0.51 |
| Mj | pg/ml | 1.0E-9 | 1.0E-9 | 5.2E0 | 1.0E1 | 3.1E1 | 6.3E1 | 1.0E-9 | 1.0E-9 | 4.6E2 | 5.4E2 | 360 | 76 | 190 | 76 | 0.52 |
| Mk | pg/ml | 6.5E-1 | 3.5E0 | 1.6E1 | 8.0E1 | 9.9E1 | 6.4E2 | 1.0E-9 | 1.0E-9 | 1.7E3 | 5.6E3 | 360 | 76 | 190 | 76 | 0.53 |
| Ml | pg/ml | 1.0E-9 | 1.0E-9 | 9.1E0 | 1.1E0 | 1.1E2 | 2.9E0 | 1.0E-9 | 1.0E-9 | 2.1E3 | 1.6E1 | 360 | 76 | 190 | 76 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Mm | pg/ml | 4.0E2 | 8.0E2 | 8.0E2 | 1.3E3 | 9.7E2 | 1.5E3 | 1.0E-9 | 1.0E-9 | 6.0E3 | 6.9E3 | 360 | 76 | 190 | 76 | 0.61 |
| Mn | pg/ml | 4.8E0 | 5.2E0 | 1.0E1 | 1.1E1 | 2.5E1 | 1.8E1 | 1.0E-9 | 1.0E-9 | 3.5E2 | 1.3E2 | 360 | 76 | 190 | 76 | 0.54 |
| Mp | pg/ml | 1.0E-9 | 1.0E-9 | 9.7E0 | 9.9E0 | 2.2E1 | 2.7E1 | 1.0E-9 | 1.0E-9 | 1.8E2 | 1.8E2 | 360 | 76 | 190 | 76 | 0.51 |
| Mq | pg/ml | 1.0E-9 | 1.0E-9 | 2.8E0 | 4.7E0 | 1.7E1 | 1.7E1 | 1.0E-9 | 1.0E-9 | 2.1E2 | 1.1E2 | 360 | 76 | 190 | 76 | 0.53 |
| Mr | pg/ml | 1.0E-9 | 1.0E-9 | 1.4E1 | 1.7E2 | 8.7E1 | 1.4E3 | 1.0E-9 | 1.0E-9 | 1.4E3 | 1.2E4 | 360 | 76 | 190 | 76 | 0.54 |
| Ms | pg/ml | 3.8E2 | 4.2E2 | 5.6E2 | 5.6E2 | 7.1E2 | 5.4E2 | 1.0E-9 | 1.0E-9 | 5.6E3 | 2.2E3 | 360 | 76 | 190 | 76 | 0.52 |
| Mt | pg/ml | 1.0E-9 | 1.3E0 | 1.1E1 | 4.8E0 | 6.3E1 | 1.2E1 | 1.0E-9 | 1.0E-9 | 8.7E2 | 7.8E1 | 360 | 76 | 190 | 76 | 0.62 |
| Mu | pg/ml | 1.0E-9 | 1.0E-9 | 1.5E0 | 1.1E0 | 1.0E1 | 3.7E0 | 1.0E-9 | 1.0E-9 | 9.9E1 | 2.7E1 | 360 | 76 | 190 | 76 | 0.57 |
| Mv | pg/ml | 1.0E-9 | 1.0E-9 | 8.2E1 | 8.5E1 | 4.2E2 | 3.2E2 | 1.0E-9 | 1.0E-9 | 4.5E3 | 2.5E3 | 360 | 76 | 190 | 76 | 0.57 |
| Mw | pg/ml | 2.5E1 | 4.3E1 | 5.8E2 | 4.1E2 | 3.9E3 | 1.2E3 | 1.0E-9 | 1.0E-9 | 6.2E4 | 8.5E3 | 360 | 76 | 190 | 76 | 0.58 |
| Mx | pg/ml | 1.0E-9 | 1.0E-9 | 1.8E-1 | 1.7E-1 | 8.2E-1 | 4.9E-1 | 1.0E-9 | 1.0E-9 | 9.2E0 | 3.0E0 | 360 | 76 | 190 | 76 | 0.53 |
| My | pg/ml | 1.0E-9 | 1.0E-9 | 5.6E2 | 2.8E2 | 3.5E3 | 8.2E2 | 1.0E-9 | 1.0E-9 | 3.9E4 | 4.6E3 | 360 | 76 | 190 | 76 | 0.53 |
| Mz | pg/ml | 8.5E0 | 1.5E1 | 2.1E1 | 3.1E1 | 5.9E1 | 4.9E1 | 1.0E-9 | 1.0E-9 | 8.4E2 | 3.0E2 | 360 | 76 | 190 | 76 | 0.62 |
| Na | pg/ml | 1.0E-9 | 1.0E-9 | 5.7E-1 | 9.1E-1 | 1.8E0 | 2.7E0 | 1.0E-9 | 1.0E-9 | 7.8E0 | 1.6E1 | 360 | 76 | 190 | 76 | 0.55 |
| Nb | pg/ml | 1.8E0 | 2.1E0 | 4.6E0 | 6.2E0 | 1.6E1 | 2.7E1 | 1.0E-9 | 1.0E-9 | 2.3E2 | 2.4E2 | 360 | 76 | 190 | 76 | 0.53 |
| Nc | pg/ml | 4.5E2 | 1.7E2 | 6.7E2 | 3.7E2 | 8.4E2 | 4.8E2 | 1.0E-9 | 1.0E-9 | 9.2E3 | 2.1E3 | 360 | 76 | 190 | 76 | 0.39 |
| Nd | pg/ml | 3.1E1 | 6.5E0 | 2.6E1 | 1.9E1 | 2.0E1 | 2.8E1 | 1.0E-9 | 1.0E-9 | 8.4E1 | 1.4E2 | 360 | 76 | 190 | 76 | 0.37 |
| Ne | pg/ml | 4.9E2 | 2.8E2 | 6.3E2 | 4.1E2 | 6.4E2 | 4.1E2 | 1.0E-9 | 1.0E-9 | 7.0E3 | 1.7E3 | 360 | 76 | 190 | 76 | 0.39 |
| Nf | pg/ml | 1.0E-9 | 1.0E-9 | 2.9E0 | 2.7E0 | 8.8E0 | 1.1E1 | 1.0E-9 | 1.0E-9 | 8.2E1 | 8.2E1 | 360 | 76 | 190 | 76 | 0.47 |
| Ng | pg/ml | 2.1E1 | 6.7E1 | 1.1E2 | 1.3E2 | 2.3E2 | 2.0E2 | 1.0E-9 | 1.0E-9 | 1.6E3 | 1.2E3 | 360 | 76 | 190 | 76 | 0.55 |
| Nh | pg/ml | 7.7E1 | 4.2E1 | 9.8E1 | 6.0E1 | 8.7E1 | 6.1E1 | 1.0E-9 | 1.0E-9 | 5.6E2 | 3.4E2 | 360 | 76 | 190 | 76 | 0.35 |
| Ni | pg/ml | 4.5E0 | 7.1E0 | 7.4E1 | 7.8E1 | 1.1E2 | 1.2E2 | 1.0E-9 | 1.0E-9 | 7.1E2 | 5.7E2 | 360 | 76 | 190 | 76 | 0.51 |
| Nj | pg/ml | 9.0E0 | 3.5E0 | 1.2E1 | 7.9E0 | 1.2E1 | 9.9E0 | 1.0E-9 | 1.0E-9 | 8.3E1 | 5.7E1 | 360 | 76 | 190 | 76 | 0.37 |
| Nk | pg/ml | 2.2E1 | 1.7E1 | 3.6E1 | 2.9E1 | 4.0E1 | 3.3E1 | 1.0E-9 | 1.0E-9 | 1.8E2 | 1.7E2 | 360 | 76 | 190 | 76 | 0.48 |
| Nl | pg/ml | 5.2E1 | 2.6E1 | 7.0E1 | 4.1E1 | 8.2E1 | 4.1E1 | 1.0E-9 | 1.0E-9 | 1.1E3 | 1.9E2 | 360 | 76 | 190 | 76 | 0.37 |
| Hl | pg/ml | 1.3E1 | 1.3E1 | 4.5E1 | 3.3E1 | 7.9E1 | 5.5E1 | 1.0E-9 | 1.0E-9 | 3.5E2 | 2.3E2 | 53 | 19 | 42 | 19 | 0.49 |
| Ho | pg/ml | 1.5E1 | 1.8E1 | 3.2E1 | 2.3E1 | 9.6E1 | 1.2E1 | 1.0E-9 | 8.1E0 | 7.0E2 | 6.0E1 | 53 | 19 | 42 | 19 | 0.63 |
| Hp | ng/ml | 1.7E0 | 1.9E0 | 7.2E1 | 2.8E2 | 2.4E2 | 4.2E2 | 1.0E-9 | 4.2E-1 | 8.9E2 | 8.9E2 | 53 | 19 | 42 | 19 | 0.54 |
| Tz | pg/ml | 3.7E3 | 4.4E3 | 7.5E3 | 3.5E4 | 1.1E4 | 1.5E5 | 7.4E1 | 1.5E2 | 8.8E4 | 1.0E6 | 124 | 47 | 98 | 47 | 0.61 |
| Ua | pg/ml | 3.5E3 | 4.8E3 | 3.0E4 | 1.6E4 | 1.9E5 | 3.0E4 | 3.5E2 | 3.2E2 | 2.1E6 | 1.8E5 | 124 | 47 | 98 | 47 | 0.58 |
| Ub | pg/ml | 5.8E2 | 6.7E2 | 8.7E2 | 1.0E3 | 1.2E3 | 1.1E3 | 1.0E-9 | 1.9E1 | 9.8E3 | 6.4E3 | 124 | 47 | 98 | 47 | 0.56 |
| Ue | pg/ml | 3.1E1 | 3.2E1 | 3.5E1 | 4.2E1 | 2.4E1 | 2.9E1 | 4.2E0 | 7.7E0 | 1.2E2 | 1.6E2 | 124 | 47 | 98 | 47 | 0.58 |
| Uc | pg/ml | 7.8E2 | 1.3E3 | 1.6E3 | 2.1E3 | 3.3E3 | 2.3E3 | 3.3E1 | 1.4E1 | 2.9E4 | 9.2E3 | 124 | 47 | 98 | 47 | 0.60 |
| Ud | pg/ml | 1.0E-9 | 1.0E-9 | 3.4E0 | 1.0E-9 | 3.5E1 | 0.0E0 | 1.0E-9 | 1.0E-9 | 3.9E2 | 1.0E-9 | 124 | 47 | 98 | 47 | 0.49 |
| Hq | pg/ml | 1.1E0 | 1.3E0 | 1.1E1 | 1.1E1 | 5.9E1 | 4.4E1 | 1.0E-9 | 1.0E-9 | 9.5E2 | 3.0E2 | 360 | 75 | 190 | 75 | 0.50 |
| Hr | pg/ml | 1.3E2 | 1.3E2 | 8.3E2 | 6.7E2 | 1.6E3 | 1.5E3 | 1.0E-9 | 1.0E-9 | 1.2E4 | 1.1E4 | 360 | 75 | 190 | 75 | 0.49 |
| Hu | pg/ml | 7.9E0 | 2.1E1 | 3.5E3 | 1.8E3 | 3.5E4 | 6.6E3 | 1.0E-9 | 1.0E-9 | 6.3E5 | 4.7E4 | 360 | 75 | 190 | 75 | 0.58 |
| Hv | pg/ml | 1.3E0 | 1.6E0 | 3.4E0 | 4.3E0 | 1.5E1 | 1.2E1 | 1.0E-9 | 1.0E-9 | 2.5E2 | 8.4E1 | 360 | 75 | 190 | 75 | 0.58 |
| Hw | pg/ml | 7.2E0 | 6.2E0 | 2.5E1 | 5.8E1 | 1.1E2 | 4.0E2 | 1.0E-9 | 1.0E-9 | 1.7E3 | 3.4E3 | 360 | 75 | 190 | 75 | 0.46 |
| Hx | pg/ml | 7.2E0 | 1.2E1 | 6.0E1 | 4.8E1 | 5.0E2 | 2.3E2 | 1.0E-9 | 1.0E-9 | 9.3E3 | 2.0E3 | 360 | 75 | 190 | 75 | 0.57 |
| Ib | ng/ml | 6.9E-2 | 6.7E-2 | 6.9E-1 | 2.4E0 | 3.0E0 | 8.4E0 | 1.0E-9 | 1.0E-9 | 3.0E1 | 5.2E1 | 122 | 45 | 99 | 45 | 0.53 |
| Ic | U/ml | 2.2E2 | 2.2E2 | 3.1E2 | 3.6E2 | 1.4E3 | 6.6E2 | 2.9E0 | 1.1E1 | 1.5E4 | 4.2E3 | 122 | 45 | 99 | 45 | 0.60 |
| Id | U/ml | 6.2E-1 | 8.9E-1 | 1.0E0 | 1.9E0 | 1.2E0 | 3.7E0 | 1.0E-9 | 1.0E-9 | 6.9E0 | 2.1E1 | 122 | 45 | 99 | 45 | 0.58 |
| Tt | pg/ml | 1.6E2 | 1.7E2 | 1.6E2 | 1.8E2 | 4.4E1 | 4.9E1 | 8.0E1 | 9.9E1 | 3.0E2 | 2.8E2 | 114 | 46 | 92 | 46 | 0.61 |
| To | pg/ml | 1.6E0 | 1.6E0 | 1.9E0 | 1.7E0 | 2.6E0 | 1.6E0 | 1.0E-9 | 1.0E-9 | 2.3E1 | 5.5E0 | 119 | 46 | 95 | 46 | 0.49 |
| Tr | pg/ml | 2.8E0 | 3.9E0 | 4.1E0 | 5.8E0 | 4.7E0 | 5.8E0 | 3.5E-2 | 1.0E-9 | 2.9E1 | 2.6E1 | 118 | 46 | 94 | 46 | 0.59 |
| Tn | pg/ml | 2.1E1 | 2.9E1 | 5.9E1 | 4.5E1 | 1.5E2 | 5.4E1 | 2.6E0 | 8.3E0 | 1.5E3 | 3.1E2 | 119 | 46 | 95 | 46 | 0.57 |
| Tv | ng/ml | 1.2E1 | 1.2E1 | 2.0E1 | 1.8E1 | 4.8E1 | 2.3E1 | 1.0E-9 | 1.0E-9 | 4.9E2 | 1.3E2 | 119 | 46 | 95 | 46 | 0.51 |
| Ih | ng/ml | 5.7E1 | 1.1E2 | 2.0E2 | 2.6E2 | 3.6E2 | 4.5E2 | 1.0E-9 | 1.0E-9 | 2.5E3 | 2.8E3 | 360 | 76 | 190 | 76 | 0.57 |
| Ii | ng/ml | 7.0E1 | 1.0E2 | 2.7E2 | 3.3E2 | 8.1E2 | 1.1E3 | 7.5E-1 | 2.9E0 | 8.4E3 | 9.2E3 | 360 | 76 | 190 | 76 | 0.57 |
| Ij | ng/ml | 7.0E1 | 8.5E1 | 2.0E2 | 2.3E2 | 7.3E2 | 7.6E2 | 2.1E0 | 4.7E0 | 6.4E3 | 6.4E3 | 358 | 75 | 190 | 75 | 0.57 |
| Ik | ng/ml | 1.2E1 | 2.4E2 | 9.2E2 | 6.9E2 | 9.2E3 | 1.3E3 | 5.9E-1 | 1.3E0 | 1.2E5 | 9.7E3 | 358 | 76 | 190 | 76 | 0.67 |
| Il | ng/ml | 3.3E2 | 5.5E2 | 1.4E3 | 1.3E3 | 3.0E3 | 2.7E3 | 1.0E-9 | 1.0E-9 | 1.2E4 | 1.2E4 | 354 | 75 | 190 | 75 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Im | ng/ml | 1.9E2 | 2.3E2 | 3.0E2 | 5.2E2 | 3.4E2 | 8.6E2 | 1.3E1 | 2.7E1 | 3.1E3 | 5.6E3 | 357 | 76 | 190 | 76 | 0.58 |
| In | ng/ml | 4.2E0 | 4.7E0 | 3.6E1 | 1.1E1 | 2.3E2 | 1.7E1 | 1.0E-9 | 1.0E-9 | 3.9E3 | 8.4E1 | 360 | 76 | 190 | 76 | 0.52 |
| Hb | ng/ml | 2.0E1 | 2.8E1 | 2.7E1 | 3.8E1 | 2.6E1 | 3.8E1 | 1.1E0 | 3.3E0 | 1.5E2 | 2.1E2 | 120 | 47 | 98 | 47 | 0.60 |
| Hc | pg/ml | 6.1E2 | 7.5E2 | 2.2E3 | 4.4E3 | 4.9E3 | 1.5E4 | 1.0E-9 | 1.0E-9 | 3.6E4 | 1.0E5 | 120 | 47 | 98 | 47 | 0.56 |
| Hf | ng/ml | 1.2E2 | 1.3E2 | 3.6E2 | 3.3E2 | 5.3E2 | 4.6E2 | 1.0E-9 | 1.0E-9 | 2.5E3 | 2.2E3 | 120 | 47 | 98 | 47 | 0.53 |
| Io | ng/ml | 6.9E3 | 7.8E3 | 1.7E4 | 2.1E4 | 5.1E4 | 6.5E4 | 6.6E1 | 6.2E1 | 8.8E5 | 5.5E5 | 357 | 75 | 189 | 75 | 0.54 |
| Ip | ng/ml | 8.1E0 | 1.4E1 | 1.8E1 | 2.3E1 | 2.7E1 | 2.6E1 | 1.0E-9 | 5.6E-3 | 2.6E2 | 1.4E2 | 357 | 75 | 189 | 75 | 0.54 |
| Iq | ug/ml | 9.1E-2 | 4.6E-2 | 3.9E1 | 1.1E1 | 7.2E2 | 8.7E1 | 1.0E-9 | 1.0E-9 | 1.4E4 | 7.5E2 | 357 | 75 | 189 | 75 | 0.45 |
| Ir | ug/ml | 2.9E-1 | 3.6E-1 | 4.4E0 | 2.8E0 | 3.3E1 | 1.3E1 | 1.0E-9 | 1.0E-9 | 5.1E2 | 1.1E2 | 356 | 75 | 189 | 75 | 0.57 |
| Is | ng/ml | 1.3E0 | 2.1E0 | 6.4E0 | 9.0E0 | 3.1E1 | 2.8E1 | 1.0E-9 | 1.0E-9 | 5.5E2 | 2.3E2 | 357 | 75 | 189 | 75 | 0.57 |
| It | ng/ml | 1.9E0 | 1.9E0 | 2.6E1 | 2.3E1 | 1.3E2 | 1.1E2 | 1.0E-9 | 1.0E-9 | 1.2E3 | 7.7E2 | 357 | 75 | 189 | 75 | 0.51 |
| Iu | ng/ml | 1.9E2 | 1.6E2 | 1.6E3 | 1.6E3 | 5.0E3 | 5.0E3 | 1.0E-9 | 1.0E-9 | 2.9E4 | 2.4E4 | 357 | 75 | 189 | 75 | 0.48 |
| Iv | ng/ml | 1.1E1 | 2.0E1 | 8.5E1 | 1.3E2 | 8.5E2 | 7.4E2 | 1.0E-9 | 1.0E-9 | 1.6E4 | 6.4E3 | 357 | 75 | 189 | 75 | 0.60 |
| Iz | ng/ml | 1.2E2 | 3.1E2 | 4.2E2 | 4.6E2 | 9.7E2 | 4.9E2 | 1.5E0 | 8.7E0 | 8.4E3 | 2.3E3 | 120 | 47 | 98 | 47 | 0.61 |
| Yg | pg/ml | 2.5E2 | 6.7E2 | 5.7E2 | 1.5E3 | 8.6E2 | 2.8E3 | 1.0E-9 | 4.1E1 | 4.2E3 | 1.2E4 | 51 | 18 | 41 | 18 | 0.65 |
| Yh | pg/ml | 2.6E2 | 2.9E2 | 5.6E2 | 4.2E2 | 1.1E3 | 3.5E2 | 1.0E-9 | 1.0E-9 | 7.8E3 | 1.0E3 | 51 | 18 | 41 | 18 | 0.52 |
| Yi | pg/ml | 2.7E2 | 4.3E2 | 5.7E2 | 4.3E2 | 1.1E3 | 4.2E2 | 1.0E-9 | 1.0E-9 | 7.6E3 | 1.8E3 | 51 | 18 | 41 | 18 | 0.52 |
| Yk | U/ml | 1.0E-9 | 1.0E-9 | 9.9E-2 | 3.1E-2 | 2.9E-1 | 9.4E-2 | 1.0E-9 | 1.0E-9 | 1.5E0 | 3.6E-1 | 51 | 18 | 41 | 18 | 0.47 |
| Yj | pg/ml | 1.9E2 | 1.2E2 | 4.2E2 | 3.9E2 | 5.7E2 | 9.4E2 | 9.7E0 | 1.7E1 | 3.2E3 | 4.1E3 | 51 | 18 | 41 | 18 | 0.38 |
| Yd | ng/ml | 2.0E-1 | 1.5E-1 | 3.2E-1 | 2.9E-1 | 3.8E-1 | 2.5E-1 | 6.6E-3 | 7.9E-3 | 1.8E0 | 9.6E-1 | 55 | 19 | 44 | 19 | 0.52 |
| Wb | pg/ml | 3.1E4 | 3.1E4 | 3.4E4 | 4.6E4 | 1.8E4 | 3.8E4 | 2.2E3 | 1.0E4 | 8.5E4 | 1.6E5 | 55 | 18 | 44 | 18 | 0.55 |
| Vz | pg/ml | 3.6E0 | 2.3E0 | 5.6E0 | 2.4E0 | 6.6E0 | 1.9E0 | 1.0E-9 | 1.0E-9 | 4.0E1 | 7.6E0 | 55 | 18 | 44 | 18 | 0.31 |
| Si | ng/ml | 9.1E-1 | 8.2E-1 | 1.7E0 | 2.7E0 | 2.2E0 | 3.8E0 | 2.0E-2 | 7.8E-2 | 1.0E1 | 1.3E1 | 53 | 19 | 42 | 19 | 0.57 |
| Sf | mIU/mL | 1.1E1 | 1.4E1 | 4.8E1 | 2.2E1 | 1.1E2 | 3.4E1 | 8.1E-2 | 2.0E-1 | 7.2E2 | 1.5E2 | 53 | 19 | 42 | 19 | 0.50 |
| Sh | mIU/mL | 7.7E0 | 9.0E0 | 4.1E1 | 2.6E1 | 1.1E2 | 4.3E1 | 2.9E-2 | 1.3E-1 | 5.9E2 | 1.8E2 | 53 | 19 | 42 | 19 | 0.51 |
| Sj | ng/ml | 4.0E-1 | 3.8E-1 | 4.0E-1 | 3.9E-1 | 9.0E-2 | 1.3E-1 | 1.9E-1 | 1.1E-1 | 5.7E-1 | 6.2E-1 | 53 | 19 | 42 | 19 | 0.48 |
| Rc | pg/ml | 5.0E3 | 6.9E3 | 6.4E3 | 8.6E3 | 5.0E3 | 6.2E3 | 1.9E2 | 1.1E3 | 3.0E4 | 2.3E4 | 122 | 47 | 98 | 47 | 0.59 |
| Rb | pg/ml | 7.5E-1 | 1.1E0 | 2.4E0 | 3.1E0 | 3.6E0 | 4.4E0 | 1.0E-9 | 1.0E-9 | 1.6E1 | 2.1E1 | 122 | 47 | 98 | 47 | 0.54 |
| Zq | 2.6ng/ml | 1.6E2 | 2.0E2 | 2.5E2 | 4.0E2 | 2.5E2 | 3.6E2 | 8.3E0 | 6.9E1 | 9.7E2 | 9.7E2 | 52 | 19 | 41 | 19 | 0.64 |
| Zw | 2.5ng/ml | 4.3E0 | 3.8E0 | 1.0E1 | 9.1E0 | 1.4E1 | 1.2E1 | 6.3E-2 | 3.5E-1 | 5.9E1 | 4.6E1 | 55 | 19 | 44 | 19 | 0.47 |
| Zx | 2.3mU/ml | 1.1E-1 | 9.5E-2 | 4.0E-1 | 3.2E-1 | 1.6E0 | 6.6E-1 | 4.1E-2 | 5.6E-2 | 1.2E1 | 3.0E0 | 55 | 19 | 44 | 19 | 0.53 |
| Pz | ng/ml | 3.1E3 | 5.7E3 | 6.1E3 | 2.1E4 | 9.7E3 | 1.2E5 | 1.3E1 | 3.6E1 | 9.5E4 | 1.0E6 | 358 | 75 | 189 | 75 | 0.56 |
| Qa | ng/ml | 2.7E3 | 4.5E3 | 5.9E3 | 6.7E3 | 8.0E3 | 6.2E3 | 1.5E2 | 3.4E2 | 5.2E4 | 2.7E4 | 358 | 75 | 189 | 75 | 0.62 |
| Qb | ng/ml | 8.3E1 | 1.3E2 | 2.5E2 | 1.7E2 | 7.0E2 | 1.6E2 | 7.9E-1 | 1.0E1 | 8.3E3 | 7.2E2 | 358 | 75 | 189 | 75 | 0.57 |
| Qc | ng/ml | 1.9E2 | 3.2E2 | 4.7E2 | 4.4E2 | 9.5E2 | 4.8E2 | 8.1E-1 | 1.1E1 | 1.1E4 | 3.1E3 | 358 | 75 | 189 | 75 | 0.56 |
| Qd | ng/ml | 7.9E3 | 1.2E4 | 2.2E4 | 2.0E4 | 1.1E5 | 2.5E4 | 1.5E2 | 9.8E2 | 2.0E6 | 1.5E5 | 358 | 75 | 189 | 75 | 0.60 |
| Qe | ng/ml | 6.9E2 | 1.3E3 | 1.9E3 | 2.0E3 | 5.6E3 | 2.4E3 | 1.0E-9 | 6.8E1 | 9.7E4 | 1.4E4 | 358 | 75 | 189 | 75 | 0.62 |
| Jd | ng/ml | 4.3E-1 | 2.1E0 | 7.5E0 | 4.4E0 | 5.9E1 | 1.1E1 | 1.0E-9 | 1.0E-9 | 6.5E2 | 7.3E1 | 122 | 47 | 99 | 47 | 0.69 |
| Je | ng/ml | 1.0E-9 | 2.9E-1 | 1.4E0 | 1.4E0 | 5.4E0 | 2.1E0 | 1.0E-9 | 1.0E-9 | 5.6E1 | 7.0E0 | 122 | 47 | 99 | 47 | 0.59 |
| Jf | ng/ml | 1.0E-9 | 1.0E-9 | 7.4E-1 | 1.2E0 | 1.9E0 | 2.4E0 | 1.0E-9 | 1.0E-9 | 1.2E1 | 9.6E0 | 122 | 47 | 99 | 47 | 0.55 |
| Jg | ng/ml | 3.7E2 | 8.0E2 | 6.8E2 | 1.2E3 | 1.0E3 | 1.1E3 | 5.8E0 | 1.1E1 | 1.0E4 | 5.4E3 | 360 | 75 | 190 | 75 | 0.67 |
| Jh | ng/ml | 2.3E0 | 5.5E0 | 2.4E1 | 3.6E1 | 9.5E1 | 8.8E1 | 1.0E-9 | 1.0E-9 | 1.2E3 | 4.7E2 | 360 | 75 | 190 | 75 | 0.60 |
| Ji | ng/ml | 4.6E1 | 7.0E1 | 6.6E1 | 1.1E2 | 7.0E1 | 1.0E2 | 1.1E0 | 8.3E0 | 5.3E2 | 5.9E2 | 360 | 75 | 190 | 75 | 0.67 |
| Sr | pg/mL | 3.2E2 | 4.9E2 | 7.5E2 | 8.9E2 | 1.3E3 | 1.1E3 | 1.0E-9 | 1.0E-9 | 9.8E3 | 5.1E3 | 121 | 45 | 98 | 45 | 0.58 |
| Ss | pg/mL | 6.2E4 | 2.0E5 | 1.2E5 | 1.7E5 | 1.4E5 | 1.3E5 | 9.5E3 | 6.5E3 | 7.1E5 | 5.3E5 | 121 | 45 | 98 | 45 | 0.62 |
| St | pg/mL | 1.7E7 | 2.9E7 | 4.0E7 | 4.8E7 | 5.3E7 | 6.0E7 | 7.8E5 | 1.1E6 | 4.1E8 | 2.9E8 | 119 | 47 | 96 | 47 | 0.57 |
| Wc | ng/ml | 1.0E-9 | 1.2E-2 | 6.2E-2 | 5.3E-2 | 1.6E-1 | 8.4E-2 | 1.0E-9 | 1.0E-9 | 9.8E-1 | 2.8E-1 | 55 | 19 | 43 | 19 | 0.55 |
| Wd | ng/ml | 8.8E0 | 1.7E1 | 2.5E1 | 7.0E1 | 7.8E1 | 1.8E2 | 1.0E-9 | 5.0E-1 | 5.4E2 | 7.9E2 | 55 | 19 | 43 | 19 | 0.62 |
| We | ng/ml | 2.6E-1 | 7.7E-1 | 6.0E-1 | 8.9E-1 | 9.0E-1 | 1.0E0 | 1.0E-9 | 1.0E-9 | 3.9E0 | 3.5E0 | 55 | 19 | 43 | 19 | 0.58 |
| Wg | ng/ml | 1.0E-9 | 1.0E-9 | 2.9E-4 | 1.0E-9 | 2.2E-3 | 0.0E0 | 1.0E-9 | 1.0E-9 | 1.6E-2 | 1.0E-9 | 55 | 19 | 43 | 19 | 0.49 |
| Wh | ng/ml | 8.7E-3 | 1.5E-2 | 3.4E-2 | 1.9E-1 | 1.1E-1 | 5.6E-1 | 1.0E-9 | 1.0E-9 | 7.6E-1 | 2.5E0 | 55 | 19 | 43 | 19 | 0.59 |
| Wf | ng/ml | 1.0E-9 | 1.0E-9 | 2.0E-1 | 2.2E-1 | 6.5E-1 | 5.9E-1 | 1.0E-9 | 1.0E-9 | 4.6E0 | 2.4E0 | 55 | 19 | 43 | 19 | 0.45 |
| Ra | pg/ml | 1.0E-9 | 1.0E-9 | 6.8E-1 | 2.7E-1 | 1.3E0 | 7.4E-1 | 1.0E-9 | 1.0E-9 | 7.3E0 | 2.6E0 | 122 | 47 | 98 | 47 | 0.43 |

| Code | Units | Median | | Average | | Standard Deviation | | Minimum | | Maximum | | Number of Samples | | Number of Patients | | AUC |
|------|-------|--------|----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Qz | pg/ml | 1.1E1 | 1.3E1 | 7.1E1 | 5.0E1 | 1.1E2 | 6.4E1 | 1.0E-9 | 1.0E-9 | 7.1E2 | 2.2E2 | 122 | 47 | 98 | 47 | 0.48 |
| Qy | pg/ml | 3.9E-1 | 5.4E-1 | 3.6E0 | 2.7E1 | 1.6E1 | 1.0E2 | 1.0E-9 | 1.0E-9 | 1.6E2 | 5.1E2 | 122 | 47 | 98 | 47 | 0.56 |
| Qx | pg/ml | 1.0E-9 | 1.0E-9 | 5.6E0 | 5.2E-1 | 4.9E1 | 2.0E0 | 1.0E-9 | 1.0E-9 | 5.4E2 | 9.4E0 | 122 | 47 | 98 | 47 | 0.48 |
| Qw | pg/ml | 9.0E-2 | 1.0E-9 | 2.2E0 | 2.6E0 | 1.1E1 | 1.3E1 | 1.0E-9 | 1.0E-9 | 1.2E2 | 8.6E1 | 122 | 47 | 98 | 47 | 0.48 |
| Qv | pg/ml | 2.8E4 | 2.1E4 | 3.9E4 | 2.9E4 | 7.4E4 | 3.6E4 | 1.2E3 | 1.1E3 | 7.4E5 | 2.3E5 | 122 | 47 | 98 | 47 | 0.41 |
| Qu | pg/ml | 1.7E0 | 2.2E1 | 8.6E1 | 1.1E2 | 1.8E2 | 2.0E2 | 1.0E-9 | 1.0E-9 | 8.0E2 | 9.2E2 | 122 | 47 | 98 | 47 | 0.58 |
| Qt | pg/ml | 9.9E0 | 1.3E1 | 3.6E1 | 7.2E1 | 7.7E1 | 1.5E2 | 1.0E-9 | 1.0E-9 | 4.1E2 | 7.0E2 | 122 | 47 | 98 | 47 | 0.57 |
| Qh | ng/ml | 1.3E1 | 1.7E1 | 3.5E1 | 3.4E1 | 7.0E1 | 4.7E1 | 1.2E-1 | 1.4E0 | 6.4E2 | 2.6E2 | 122 | 47 | 98 | 47 | 0.57 |
| Qg | ng/ml | 9.0E0 | 7.0E0 | 1.7E1 | 1.5E1 | 2.5E1 | 3.2E1 | 1.5E-1 | 1.3E-1 | 1.8E2 | 2.2E2 | 122 | 47 | 98 | 47 | 0.43 |
| Jj | ng/ml | 7.1E2 | 5.7E2 | 2.7E3 | 1.1E3 | 1.9E4 | 1.7E3 | 2.3E0 | 4.9E0 | 3.4E5 | 1.1E4 | 360 | 75 | 190 | 75 | 0.43 |
| Jk | ng/ml | 3.0E0 | 3.8E0 | 2.1E1 | 2.6E1 | 4.6E1 | 4.9E1 | 1.0E-9 | 1.2E-1 | 2.8E2 | 2.7E2 | 360 | 75 | 190 | 75 | 0.55 |
| Jl | ng/ml | 3.4E-1 | 6.6E-1 | 1.9E0 | 4.5E0 | 4.7E0 | 1.8E1 | 7.6E-4 | 1.1E-3 | 3.2E1 | 1.6E2 | 360 | 75 | 190 | 75 | 0.60 |
| Jm | ng/ml | 1.5E1 | 2.0E1 | 5.3E1 | 5.7E1 | 1.2E2 | 9.1E1 | 1.0E-9 | 4.1E-1 | 1.4E3 | 6.1E2 | 360 | 75 | 190 | 75 | 0.55 |
| Jn | pg/ml | 3.2E-1 | 4.2E-1 | 3.2E0 | 1.4E0 | 3.3E1 | 3.5E0 | 1.0E-9 | 1.0E-9 | 6.2E2 | 2.7E1 | 360 | 75 | 190 | 75 | 0.57 |
| Jo | pg/ml | 3.5E3 | 4.2E3 | 4.8E3 | 5.8E3 | 3.9E3 | 5.5E3 | 2.0E1 | 7.5E1 | 2.0E4 | 3.8E4 | 360 | 75 | 190 | 75 | 0.55 |
| Jp | pg/ml | 6.3E4 | 8.3E4 | 6.6E4 | 8.5E4 | 3.7E4 | 3.9E4 | 5.8E2 | 2.8E3 | 3.0E5 | 2.1E5 | 360 | 75 | 190 | 75 | 0.65 |
| Jq | pg/ml | 9.1E1 | 1.1E2 | 1.4E2 | 2.0E2 | 1.8E2 | 4.8E2 | 1.0E0 | 5.4E0 | 2.0E3 | 4.0E3 | 360 | 75 | 190 | 75 | 0.55 |
| Jr | pg/ml | 3.3E0 | 6.7E0 | 4.5E1 | 1.6E1 | 5.6E2 | 3.5E1 | 1.0E-9 | 1.0E-9 | 1.1E4 | 2.0E2 | 360 | 75 | 190 | 75 | 0.58 |
| Js | pg/ml | 1.2E1 | 1.4E1 | 6.5E1 | 2.6E1 | 5.4E2 | 6.9E1 | 1.0E-9 | 1.0E-9 | 1.0E4 | 5.9E2 | 360 | 75 | 190 | 75 | 0.54 |
| Jt | pg/ml | 2.2E3 | 3.3E3 | 2.8E3 | 3.9E3 | 2.2E3 | 4.0E3 | 2.2E1 | 2.6E2 | 2.2E4 | 3.3E4 | 360 | 75 | 190 | 75 | 0.61 |
| Xa | pg/ml | 1.0E-9 | 4.8E0 | 7.0E0 | 1.8E1 | 1.5E1 | 2.7E1 | 1.0E-9 | 1.0E-9 | 7.6E1 | 9.9E1 | 55 | 18 | 44 | 18 | 0.63 |
| Ye | pg/ml | 1.0E-9 | 1.0E-9 | 3.9E0 | 2.5E1 | 1.3E1 | 8.2E1 | 1.0E-9 | 1.0E-9 | 6.9E1 | 3.5E2 | 55 | 18 | 44 | 18 | 0.52 |
| Tm | pg/ml | 1.0E-9 | 1.0E-9 | 2.4E0 | 9.2E0 | 4.8E0 | 1.9E1 | 1.0E-9 | 1.0E-9 | 1.8E1 | 6.8E1 | 55 | 18 | 44 | 18 | 0.59 |
| Tl | pg/ml | 1.1E-1 | 4.7E-1 | 2.3E-1 | 4.0E-1 | 3.6E-1 | 3.5E-1 | 1.0E-9 | 1.0E-9 | 1.8E0 | 1.2E0 | 55 | 18 | 44 | 18 | 0.66 |
| Ju | mIU/ml | 7.2E0 | 1.1E1 | 2.0E1 | 2.0E1 | 3.4E1 | 3.2E1 | 6.5E-2 | 1.5E-1 | 2.3E2 | 2.0E2 | 122 | 47 | 99 | 47 | 0.58 |
| Jv | mIU/ml | 9.3E0 | 1.6E1 | 3.1E1 | 3.2E1 | 6.1E1 | 5.6E1 | 1.0E-2 | 8.2E-2 | 4.4E2 | 3.4E2 | 122 | 47 | 99 | 47 | 0.55 |
| Jy | ng/ml | 1.7E-3 | 1.6E-3 | 2.3E-3 | 1.7E-3 | 4.7E-3 | 8.5E-4 | 1.0E-9 | 1.0E-9 | 5.2E-2 | 3.8E-3 | 122 | 47 | 99 | 47 | 0.47 |
| Kc | pg/ml | 2.1E1 | 3.9E1 | 3.9E1 | 5.8E1 | 4.4E1 | 5.1E1 | 1.0E-9 | 1.0E-9 | 1.7E2 | 1.8E2 | 121 | 46 | 98 | 46 | 0.67 |
| Kd | pg/ml | 1.0E-9 | 1.0E-9 | 1.9E2 | 2.4E2 | 6.7E2 | 5.7E2 | 1.0E-9 | 1.0E-9 | 4.8E3 | 2.3E3 | 121 | 46 | 98 | 46 | 0.52 |
| Ke | pg/ml | 9.5E3 | 1.6E4 | 1.3E4 | 1.7E4 | 9.6E3 | 1.0E4 | 3.4E2 | 5.8E2 | 5.9E4 | 5.6E4 | 121 | 46 | 98 | 46 | 0.63 |
| Kf | pg/mL | 5.5E0 | 9.4E0 | 6.3E0 | 8.7E0 | 5.7E0 | 5.0E0 | 1.0E-9 | 1.0E-9 | 2.6E1 | 2.4E1 | 121 | 46 | 98 | 46 | 0.66 |
| Kg | pg/mL | 9.9E2 | 1.6E3 | 1.7E3 | 2.2E3 | 2.4E3 | 2.2E3 | 7.7E1 | 1.4E2 | 2.2E4 | 1.1E4 | 121 | 46 | 98 | 46 | 0.60 |
| Ki | pg/ml | 6.5E1 | 5.6E1 | 7.2E1 | 6.5E1 | 5.2E1 | 3.0E1 | 1.0E-9 | 1.0E-9 | 2.9E2 | 1.2E2 | 121 | 45 | 98 | 45 | 0.48 |
| Kj | pg/ml | 9.3E2 | 1.5E3 | 1.5E3 | 1.8E3 | 1.6E3 | 1.5E3 | 6.6E1 | 1.1E2 | 1.0E4 | 6.1E3 | 121 | 46 | 98 | 46 | 0.61 |
| Kk | pg/ml | 6.8E0 | 7.0E0 | 1.2E1 | 1.2E1 | 1.8E1 | 1.5E1 | 1.0E-9 | 1.0E-9 | 1.6E2 | 6.1E1 | 121 | 46 | 98 | 46 | 0.52 |
| Kl | pg/ml | 1.7E4 | 3.4E4 | 2.4E4 | 3.8E4 | 2.3E4 | 3.1E4 | 3.5E2 | 2.1E2 | 1.1E5 | 1.6E5 | 121 | 46 | 98 | 46 | 0.64 |
| Kn | pg/ml | 1.5E1 | 4.1E1 | 5.4E1 | 6.2E1 | 1.1E2 | 8.1E1 | 1.0E-9 | 1.0E-9 | 7.3E2 | 4.7E2 | 121 | 46 | 98 | 46 | 0.62 |
| Ko | pg/ml | 3.0E2 | 5.7E2 | 3.9E2 | 6.3E2 | 4.0E2 | 6.1E2 | 1.0E-9 | 1.0E-9 | 1.8E3 | 2.4E3 | 121 | 46 | 98 | 46 | 0.61 |
| Kp | pg/ml | 2.7E2 | 3.6E2 | 3.2E2 | 4.1E2 | 2.9E2 | 2.4E2 | 1.0E-9 | 1.0E-9 | 1.7E3 | 9.1E2 | 121 | 46 | 98 | 46 | 0.62 |
| Kq | pg/ml | 2.8E2 | 3.9E2 | 4.2E2 | 4.8E2 | 9.3E2 | 3.8E2 | 5.1E0 | 7.0E0 | 9.8E3 | 2.1E3 | 117 | 47 | 95 | 47 | 0.63 |
| Kr | pg/ml | 3.6E-1 | 2.5E-1 | 2.4E0 | 2.4E0 | 4.8E0 | 3.5E0 | 1.0E-9 | 1.0E-9 | 3.5E1 | 1.2E1 | 117 | 47 | 95 | 47 | 0.53 |
| Ks | pg/ml | 1.5E4 | 1.1E4 | 2.0E4 | 2.0E4 | 1.9E4 | 1.8E4 | 3.8E2 | 2.7E2 | 1.1E5 | 5.0E4 | 117 | 47 | 95 | 47 | 0.49 |
| Ps | ng/ml | 1.4E2 | 1.9E2 | 5.2E2 | 5.2E2 | 1.6E3 | 7.5E2 | 1.6E0 | 4.1E-1 | 9.0E3 | 2.9E3 | 53 | 19 | 41 | 19 | 0.60 |
| Kx | ng/ml | 1.0E-9 | 3.4E-3 | 4.4E-3 | 6.4E-3 | 8.5E-3 | 9.2E-3 | 1.0E-9 | 1.0E-9 | 5.1E-2 | 4.4E-2 | 120 | 47 | 98 | 47 | 0.58 |
| Ky | ng/ml | 7.7E-2 | 1.1E-1 | 3.2E-1 | 3.2E-1 | 6.9E-1 | 5.1E-1 | 1.0E-9 | 1.0E-9 | 5.1E0 | 2.4E0 | 120 | 47 | 98 | 47 | 0.52 |
| Kz | ng/ml | 1.0E-9 | 1.0E-9 | 4.7E-3 | 2.6E-3 | 6.8E-3 | 5.1E-3 | 1.0E-9 | 1.0E-9 | 3.6E-2 | 1.4E-2 | 120 | 47 | 98 | 47 | 0.42 |
| Rz | ng/ml | 1.4E-1 | 5.6E-1 | 8.8E-1 | 1.2E0 | 1.5E0 | 1.6E0 | 3.6E-3 | 1.8E-2 | 6.7E0 | 4.7E0 | 53 | 19 | 42 | 19 | 0.63 |
| Ry | ng/ml | 1.6E-2 | 1.6E-2 | 1.9E-2 | 2.1E-2 | 1.8E-2 | 1.7E-2 | 1.0E-9 | 1.0E-9 | 1.0E-1 | 6.5E-2 | 53 | 19 | 42 | 19 | 0.57 |
| Rx | ng/ml | 3.5E-5 | 3.5E-5 | 2.1E-3 | 1.0E-3 | 3.8E-3 | 1.6E-3 | 1.0E-9 | 1.0E-9 | 2.0E-2 | 4.7E-3 | 53 | 19 | 42 | 19 | 0.47 |
| Ld | pg/ml | 1.0E-9 | 1.0E-9 | 3.6E0 | 1.8E0 | 9.5E0 | 2.9E0 | 1.0E-9 | 1.0E-9 | 6.9E1 | 1.2E1 | 119 | 46 | 97 | 46 | 0.47 |
| Lh | pg/ml | 1.0E4 | 1.4E4 | 1.8E4 | 2.8E4 | 2.6E4 | 4.9E4 | 1.0E-9 | 1.8E2 | 2.6E5 | 4.1E5 | 359 | 75 | 190 | 75 | 0.63 |
| Li | pg/ml | 2.5E3 | 5.7E3 | 1.3E4 | 2.4E4 | 7.2E4 | 7.2E4 | 1.0E-9 | 1.7E2 | 1.3E6 | 5.9E5 | 359 | 75 | 190 | 75 | 0.64 |
| Lj | pg/ml | 1.7E3 | 4.4E3 | 1.4E4 | 2.7E4 | 5.1E4 | 7.7E4 | 1.0E-9 | 4.1E1 | 4.4E5 | 4.7E5 | 359 | 75 | 190 | 75 | 0.61 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Lp | pg/ml | 9.4E0 | 5.7E0 | 5.6E1 | 8.1E1 | 1.6E2 | 1.9E2 | 1.0E-9 | 1.0E-9 | 1.1E3 | 7.7E2 | 53 | 19 | 42 | 19 | 0.48 |
| Lt | pg/ml | 1.0E-9 | 1.0E-9 | 2.6E0 | 1.8E0 | 9.6E0 | 6.0E0 | 1.0E-9 | 1.0E-9 | 6.0E1 | 2.5E1 | 53 | 19 | 42 | 19 | 0.50 |
| Rv | ng/ml | 2.2E-4 | 9.8E-4 | 1.1E-3 | 1.1E-3 | 1.8E-3 | 1.2E-3 | 1.0E-9 | 1.0E-9 | 9.2E-3 | 5.0E-3 | 53 | 19 | 41 | 19 | 0.56 |
| Ru | ng/ml | 1.0E-9 | 1.0E-9 | 8.1E-3 | 2.3E-2 | 4.5E-2 | 8.8E-2 | 1.0E-9 | 1.0E-9 | 3.1E-1 | 3.8E-1 | 53 | 19 | 41 | 19 | 0.53 |
| Rt | ng/ml | 6.2E-2 | 4.0E-2 | 9.3E-2 | 1.1E-1 | 1.1E-1 | 1.6E-1 | 1.6E-3 | 1.0E-3 | 4.5E-1 | 5.6E-1 | 53 | 19 | 41 | 19 | 0.46 |
| Yl | pg/ml | 1.4E1 | 7.9E0 | 1.9E1 | 1.0E1 | 1.7E1 | 1.5E1 | 1.0E-9 | 1.0E-9 | 6.5E1 | 6.6E1 | 55 | 19 | 44 | 19 | 0.30 |
| Rm | ng/ml | 1.6E1 | 1.7E1 | 5.2E1 | 3.8E1 | 8.3E1 | 5.3E1 | 2.2E-1 | 1.3E0 | 4.0E2 | 2.5E2 | 121 | 46 | 97 | 46 | 0.50 |
| Rh | ng/ml | 1.3E2 | 1.5E2 | 4.7E2 | 2.8E2 | 1.6E3 | 3.5E2 | 4.7E0 | 3.6E0 | 1.7E4 | 2.0E3 | 121 | 46 | 97 | 46 | 0.53 |
| Ri | ng/ml | 1.0E-9 | 1.0E-9 | 4.3E0 | 3.5E0 | 1.0E1 | 7.8E0 | 1.0E-9 | 1.0E-9 | 7.4E1 | 4.5E1 | 122 | 46 | 98 | 46 | 0.48 |
| Rg | ng/ml | 1.0E-9 | 1.0E-9 | 3.2E-2 | 1.9E-2 | 2.6E-1 | 1.0E-1 | 1.0E-9 | 1.0E-9 | 2.7E0 | 6.8E-1 | 121 | 46 | 97 | 46 | 0.55 |
| Rj | ng/ml | 1.0E-9 | 1.0E-9 | 1.8E0 | 9.4E-1 | 7.6E0 | 1.6E0 | 1.0E-9 | 1.0E-9 | 8.0E1 | 6.3E0 | 122 | 46 | 98 | 46 | 0.52 |
| Rf | ng/ml | 3.4E-1 | 5.7E-1 | 8.0E-1 | 1.4E0 | 1.5E0 | 2.2E0 | 7.8E-3 | 1.8E-2 | 1.4E1 | 1.2E1 | 121 | 46 | 97 | 46 | 0.61 |
| Ql | pg/ml | 5.0E0 | 4.5E0 | 1.1E1 | 1.6E1 | 1.7E1 | 3.0E1 | 1.0E-9 | 1.0E-9 | 1.2E2 | 1.8E2 | 122 | 47 | 99 | 47 | 0.51 |
| Qm | pg/ml | 1.7E0 | 9.6E0 | 2.0E1 | 2.5E1 | 4.4E1 | 3.6E1 | 1.0E-9 | 1.0E-9 | 3.6E2 | 1.7E2 | 122 | 47 | 99 | 47 | 0.59 |
| Qn | pg/ml | 6.1E-1 | 1.4E0 | 7.2E0 | 1.1E1 | 2.4E1 | 3.6E1 | 1.0E-9 | 1.0E-9 | 2.2E2 | 2.4E2 | 122 | 47 | 99 | 47 | 0.54 |
| Nv | pg/ml | 3.0E3 | 4.7E3 | 1.1E4 | 1.2E4 | 5.8E4 | 1.9E4 | 1.0E-9 | 8.4E1 | 1.1E6 | 1.1E5 | 362 | 76 | 190 | 76 | 0.63 |
| Nw | pg/ml | 7.1E3 | 1.4E4 | 1.2E4 | 1.6E4 | 2.1E4 | 1.8E4 | 8.6E1 | 5.7E2 | 2.1E5 | 1.4E5 | 362 | 76 | 190 | 76 | 0.66 |
| Nx | pg/ml | 1.2E2 | 2.4E2 | 3.6E2 | 5.7E2 | 6.8E2 | 7.2E2 | 1.0E-9 | 1.0E-9 | 7.4E3 | 2.6E3 | 362 | 76 | 190 | 76 | 0.63 |
| Ny | pg/ml | 5.2E0 | 9.6E0 | 9.8E1 | 2.8E1 | 1.3E3 | 8.6E1 | 1.0E-9 | 1.0E-9 | 2.5E4 | 6.2E2 | 362 | 76 | 190 | 76 | 0.55 |
| Oa | pg/ml | 1.6E2 | 2.5E2 | 4.0E2 | 3.7E2 | 7.6E2 | 4.4E2 | 1.0E-9 | 1.0E-9 | 4.8E3 | 2.3E3 | 122 | 47 | 99 | 47 | 0.56 |
| Op | pg/ml | 4.1E5 | 4.0E5 | 3.9E5 | 4.1E5 | 1.6E5 | 1.5E5 | 3.3E4 | 1.1E5 | 7.3E5 | 6.6E5 | 53 | 19 | 42 | 19 | 0.54 |
| Wn | ng/ml | 1.2E1 | 1.7E1 | 9.2E1 | 5.2E1 | 3.0E2 | 1.1E2 | 2.2E0 | 8.9E-1 | 1.8E3 | 4.9E2 | 35 | 17 | 29 | 17 | 0.55 |
| Tk | ng/ml | 1.3E2 | 1.6E2 | 3.8E2 | 2.5E2 | 7.3E2 | 2.8E2 | 1.9E1 | 2.2E1 | 4.2E3 | 1.2E3 | 37 | 17 | 30 | 17 | 0.50 |
| Oe | pg/ml | 1.1E1 | 4.3E1 | 2.3E2 | 3.6E2 | 3.7E2 | 7.8E2 | 1.0E-9 | 1.0E-9 | 2.7E3 | 5.1E3 | 357 | 76 | 190 | 76 | 0.52 |
| Of | pg/ml | 1.6E2 | 3.4E2 | 4.0E3 | 1.4E4 | 1.5E4 | 7.2E4 | 1.0E-9 | 1.0E-9 | 1.8E5 | 6.2E5 | 360 | 76 | 190 | 76 | 0.53 |
| Og | pg/ml | 8.4E-2 | 1.0E-1 | 5.8E-1 | 7.1E-1 | 1.9E0 | 1.8E0 | 1.0E-9 | 1.0E-9 | 2.0E1 | 1.0E1 | 360 | 76 | 190 | 76 | 0.55 |
| Oh | pg/ml | 1.9E0 | 3.2E0 | 2.2E1 | 1.0E1 | 1.2E2 | 3.4E1 | 1.0E-9 | 1.2E-2 | 1.4E3 | 2.2E2 | 360 | 76 | 190 | 76 | 0.60 |
| Oi | pg/ml | 2.0E0 | 4.4E0 | 6.3E0 | 8.5E0 | 1.1E1 | 1.0E1 | 1.0E-9 | 1.0E-9 | 8.6E1 | 4.1E1 | 360 | 76 | 190 | 76 | 0.59 |
| Ok | pg/ml | 2.9E2 | 5.4E2 | 3.9E2 | 8.3E2 | 3.7E2 | 1.2E3 | 1.3E1 | 4.0E1 | 2.8E3 | 9.8E3 | 360 | 76 | 190 | 76 | 0.70 |
| Om | pg/ml | 3.6E2 | 5.6E2 | 7.4E2 | 1.6E3 | 2.0E3 | 4.6E3 | 1.0E-9 | 1.0E-9 | 3.0E4 | 3.6E4 | 360 | 76 | 190 | 76 | 0.62 |
| On | pg/ml | 1.3E2 | 2.2E2 | 2.5E2 | 5.3E2 | 4.5E2 | 1.7E3 | 1.0E-9 | 7.2E0 | 4.5E3 | 1.5E4 | 360 | 76 | 190 | 76 | 0.65 |
| Or | pg/ml | 1.0E1 | 1.3E1 | 2.9E1 | 4.2E1 | 6.1E1 | 1.2E2 | 1.0E-9 | 1.0E-9 | 5.0E2 | 7.6E2 | 120 | 47 | 98 | 47 | 0.49 |
| Ow | pg/ml | 2.7E1 | 4.1E1 | 9.9E1 | 1.5E2 | 2.9E2 | 4.8E2 | 1.0E-9 | 1.0E-9 | 2.3E3 | 3.2E3 | 120 | 47 | 98 | 47 | 0.57 |
| Ou | pg/ml | 5.0E2 | 5.6E2 | 8.2E2 | 1.2E3 | 1.2E3 | 2.0E3 | 3.5E1 | 1.0E-9 | 9.4E3 | 9.3E3 | 120 | 47 | 98 | 47 | 0.56 |
| Ul | ng/ml | 1.0E-9 | 1.0E-9 | 1.2E0 | 8.5E-1 | 4.3E0 | 3.9E0 | 1.0E-9 | 1.0E-9 | 2.9E1 | 2.4E1 | 131 | 47 | 102 | 47 | 0.48 |
| Uo | ng/ml | 1.0E-9 | 1.0E-9 | 1.0E-1 | 7.5E-2 | 2.3E-1 | 1.9E-1 | 1.0E-9 | 1.0E-9 | 1.3E0 | 8.5E-1 | 131 | 47 | 102 | 47 | 0.46 |
| Uk | ng/ml | 1.0E-9 | 1.0E-9 | 4.7E-3 | 1.0E-2 | 1.1E-2 | 2.2E-2 | 1.0E-9 | 1.0E-9 | 9.9E-2 | 1.1E-1 | 131 | 47 | 102 | 47 | 0.52 |
| Um | ng/ml | 1.0E-9 | 1.0E-9 | 4.0E-1 | 2.0E-1 | 9.8E-1 | 3.3E-1 | 1.0E-9 | 1.0E-9 | 6.8E0 | 1.3E0 | 131 | 47 | 102 | 47 | 0.50 |
| Uf | ng/ml | 4.1E-2 | 1.0E-1 | 1.0E-1 | 1.9E-1 | 1.4E-1 | 3.7E-1 | 2.7E-3 | 1.1E-3 | 7.0E-1 | 2.5E0 | 131 | 47 | 102 | 47 | 0.65 |
| Uh | ng/ml | 1.6E0 | 2.2E0 | 2.7E0 | 4.0E0 | 2.7E0 | 4.5E0 | 3.2E-2 | 6.0E-2 | 1.5E1 | 1.7E1 | 131 | 47 | 102 | 47 | 0.57 |
| Un | ng/ml | 1.7E0 | 2.5E0 | 2.0E0 | 2.6E0 | 1.3E0 | 1.3E0 | 2.0E-1 | 5.4E-1 | 7.0E0 | 5.6E0 | 131 | 47 | 102 | 47 | 0.66 |
| Ug | ng/ml | 1.5E1 | 2.3E1 | 2.7E1 | 3.7E1 | 2.7E1 | 4.4E1 | 6.9E-1 | 1.1E0 | 1.3E2 | 2.1E2 | 131 | 47 | 102 | 47 | 0.53 |
| Ur | ng/ml | 1.7E-1 | 9.6E-2 | 1.2E0 | 5.7E-1 | 8.2E0 | 1.3E0 | 1.0E-9 | 1.0E-9 | 9.3E1 | 7.2E0 | 131 | 47 | 102 | 47 | 0.43 |
| Up | ng/ml | 1.0E-9 | 1.0E-9 | 6.9E-3 | 4.3E-3 | 3.2E-2 | 1.1E-2 | 1.0E-9 | 1.0E-9 | 3.3E-1 | 6.0E-2 | 131 | 47 | 102 | 47 | 0.54 |
| Us | ng/ml | 2.6E-3 | 6.6E-3 | 2.0E-2 | 1.8E-2 | 5.4E-2 | 2.5E-2 | 1.0E-9 | 1.0E-9 | 4.0E-1 | 1.0E-1 | 131 | 47 | 102 | 47 | 0.54 |
| Uv | ng/ml | 3.5E-3 | 2.8E-3 | 1.3E-2 | 6.9E-3 | 4.0E-2 | 1.2E-2 | 1.0E-9 | 1.0E-9 | 3.5E-1 | 7.1E-2 | 131 | 47 | 102 | 47 | 0.48 |
| Ut | ng/ml | 5.0E-1 | 6.3E-1 | 2.3E0 | 1.8E0 | 8.5E0 | 2.7E0 | 1.0E-9 | 1.0E-9 | 7.2E1 | 1.1E1 | 131 | 47 | 102 | 47 | 0.56 |
| Uu | ng/ml | 6.3E0 | 8.7E0 | 7.3E0 | 9.3E0 | 4.7E0 | 5.7E0 | 8.1E-1 | 4.5E-1 | 2.4E1 | 2.1E1 | 131 | 47 | 102 | 47 | 0.60 |
| Uw | ng/ml | 2.0E0 | 3.2E0 | 3.1E0 | 2.9E0 | 5.0E0 | 2.1E0 | 1.0E-9 | 2.0E-1 | 3.7E1 | 7.1E0 | 62 | 19 | 50 | 19 | 0.58 |
| Vb | ng/ml | 1.1E0 | 9.0E-1 | 1.1E0 | 1.0E0 | 4.3E-1 | 4.8E-1 | 2.5E-1 | 2.8E-1 | 2.5E0 | 1.9E0 | 62 | 19 | 50 | 19 | 0.45 |
| Vc | ng/ml | 1.0E-9 | 1.0E-9 | 1.4E-2 | 1.0E-9 | 8.4E-2 | 0.0E0 | 1.0E-9 | 1.0E-9 | 6.5E-1 | 1.0E-9 | 62 | 19 | 50 | 19 | 0.47 |
| Uy | ng/ml | 1.3E0 | 9.5E-1 | 4.1E0 | 2.3E0 | 9.0E0 | 3.7E0 | 5.3E-2 | 3.1E-2 | 5.1E1 | 1.6E1 | 62 | 19 | 50 | 19 | 0.43 |
| Uz | ng/ml | 1.0E-9 | 1.0E-9 | 7.1E-3 | 1.0E-9 | 5.5E-2 | 0.0E0 | 1.0E-9 | 1.0E-9 | 4.4E-1 | 1.0E-9 | 62 | 19 | 50 | 19 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ux | ng/ml | 1.2E2 | 1.8E2 | 1.7E2 | 2.3E2 | 1.4E2 | 1.5E2 | 5.8E0 | 4.9E1 | 4.8E2 | 5.3E2 | 62 | 19 | 50 | 19 | 0.63 |
| Va | ng/ml | 1.8E1 | 1.0E1 | 2.5E1 | 2.2E1 | 2.6E1 | 3.0E1 | 1.2E-1 | 8.1E-1 | 1.2E2 | 1.2E2 | 62 | 19 | 50 | 19 | 0.42 |
| Vh | ng/ml | 5.2E-3 | 7.8E-3 | 1.3E-2 | 1.6E-2 | 1.8E-2 | 2.0E-2 | 1.0E-9 | 5.2E-4 | 1.2E-1 | 7.6E-2 | 62 | 19 | 50 | 19 | 0.54 |
| Vi | ng/ml | 2.5E-3 | 5.7E-3 | 2.3E-1 | 1.3E-2 | 1.7E0 | 2.0E-2 | 1.0E-9 | 9.0E-5 | 1.4E1 | 7.5E-2 | 62 | 19 | 50 | 19 | 0.60 |
| Vj | ng/ml | 1.9E1 | 5.3E1 | 1.6E2 | 5.3E2 | 6.8E2 | 2.0E3 | 3.2E0 | 6.5E0 | 5.2E3 | 8.4E3 | 62 | 18 | 50 | 18 | 0.66 |
| Vp | ng/ml | 1.0E-9 | 1.0E-9 | 6.8E-1 | 3.3E-2 | 5.0E0 | 1.7E-1 | 1.0E-9 | 1.0E-9 | 5.0E1 | 1.2E0 | 131 | 47 | 102 | 47 | 0.46 |
| Vt | ng/ml | 6.1E0 | 7.6E0 | 8.2E0 | 1.1E1 | 6.9E0 | 1.2E1 | 6.0E-1 | 8.8E-1 | 3.2E1 | 6.5E1 | 131 | 47 | 102 | 47 | 0.56 |
| Vu | ng/ml | 1.0E-9 | 1.0E-9 | 1.9E0 | 1.8E0 | 5.4E0 | 4.0E0 | 1.0E-9 | 1.0E-9 | 5.1E1 | 2.1E1 | 130 | 47 | 102 | 47 | 0.50 |
| Vq | ng/ml | 1.5E2 | 4.3E2 | 7.5E3 | 1.1E3 | 6.7E4 | 2.2E3 | 2.0E-1 | 9.0E-1 | 6.8E5 | 1.1E4 | 102 | 31 | 82 | 31 | 0.57 |
| Vo | ng/ml | 2.6E1 | 2.6E1 | 2.5E1 | 2.5E1 | 4.3E0 | 4.6E0 | 1.1E1 | 2.4E0 | 3.5E1 | 3.4E1 | 131 | 47 | 102 | 47 | 0.52 |
| Vs | ng/ml | 1.0E-9 | 1.6E0 | 6.0E0 | 4.6E0 | 2.6E1 | 8.3E0 | 1.0E-9 | 1.0E-9 | 2.3E2 | 4.2E1 | 127 | 45 | 99 | 45 | 0.59 |
| Vv | ng/ml | 2.2E0 | 4.1E0 | 5.7E0 | 6.1E0 | 1.0E1 | 6.9E0 | 1.0E-9 | 1.0E-9 | 8.1E1 | 2.8E1 | 130 | 47 | 102 | 47 | 0.57 |
| Vw | ng/ml | 3.4E1 | 4.1E1 | 3.3E1 | 4.1E1 | 1.7E1 | 1.7E1 | 3.1E0 | 9.9E0 | 6.7E1 | 7.0E1 | 62 | 19 | 50 | 19 | 0.62 |
| Oy | pg/ml | 5.4E-1 | 6.8E-1 | 7.9E0 | 7.3E0 | 3.9E1 | 3.2E1 | 1.0E-9 | 1.0E-9 | 4.0E2 | 2.7E2 | 360 | 76 | 190 | 76 | 0.56 |
| Oz | pg/ml | 1.4E-3 | 2.2E-1 | 2.3E-1 | 3.9E-1 | 3.8E-1 | 7.5E-1 | 1.0E-9 | 1.0E-9 | 2.6E0 | 5.8E0 | 360 | 76 | 190 | 76 | 0.60 |
| Pa | pg/ml | 3.3E-1 | 4.1E-1 | 1.3E0 | 4.8E0 | 5.6E0 | 3.4E1 | 1.0E-9 | 1.0E-9 | 8.6E1 | 2.9E2 | 360 | 76 | 190 | 76 | 0.57 |
| Pb | pg/ml | 1.0E-9 | 3.3E-2 | 1.6E0 | 2.5E0 | 2.6E1 | 2.1E1 | 1.0E-9 | 1.0E-9 | 4.9E2 | 1.8E2 | 360 | 76 | 190 | 76 | 0.56 |
| Pc | pg/ml | 2.4E-2 | 3.6E-1 | 4.2E-1 | 6.3E-1 | 1.2E0 | 1.5E0 | 1.0E-9 | 1.0E-9 | 1.6E1 | 1.1E1 | 360 | 76 | 190 | 76 | 0.60 |
| Pd | pg/ml | 1.7E0 | 2.7E0 | 3.7E0 | 4.7E0 | 8.5E0 | 5.5E0 | 1.0E-9 | 1.0E-9 | 9.4E1 | 2.7E1 | 360 | 76 | 190 | 76 | 0.62 |
| Pe | pg/ml | 1.7E1 | 3.1E1 | 8.8E1 | 2.7E2 | 3.4E2 | 1.6E3 | 1.0E-9 | 1.0E-9 | 4.7E3 | 1.4E4 | 360 | 76 | 190 | 76 | 0.63 |
| Pf | pg/ml | 1.2E0 | 2.6E0 | 8.1E0 | 1.0E1 | 3.9E1 | 3.1E1 | 1.0E-9 | 1.0E-9 | 4.8E2 | 2.3E2 | 360 | 76 | 190 | 76 | 0.62 |
| Pg | pg/ml | 2.9E0 | 5.0E0 | 3.5E1 | 4.3E1 | 1.9E2 | 2.2E2 | 1.0E-9 | 1.0E-9 | 3.2E3 | 1.9E3 | 360 | 76 | 190 | 76 | 0.58 |
| Ph | ng/ml | 1.5E-1 | 2.5E-1 | 2.6E-1 | 4.5E-1 | 3.0E-1 | 6.1E-1 | 1.0E-9 | 1.0E-9 | 1.6E0 | 2.8E0 | 120 | 47 | 98 | 47 | 0.60 |
| Pi | ng/ml | 2.0E-1 | 1.9E-1 | 2.7E-1 | 2.3E-1 | 4.1E-1 | 2.0E-1 | 1.0E-9 | 1.0E-9 | 3.2E0 | 9.7E-1 | 120 | 47 | 98 | 47 | 0.50 |
| Pj | ng/mL | 4.5E0 | 7.0E0 | 5.1E0 | 8.7E0 | 3.3E0 | 6.4E0 | 3.8E-2 | 3.8E-1 | 1.6E1 | 2.5E1 | 120 | 47 | 98 | 47 | 0.65 |
| Pk | ng/ml | 8.1E-3 | 6.5E-3 | 1.2E-2 | 9.4E-3 | 1.2E-2 | 1.2E-2 | 1.0E-9 | 1.0E-9 | 5.9E-2 | 7.0E-2 | 120 | 47 | 98 | 47 | 0.44 |
| aA | mg/dL | 8.0E-1 | 9.8E-1 | 9.1E-1 | 1.2E0 | 4.4E-1 | 8.9E-1 | 3.0E-1 | 2.6E-1 | 4.1E0 | 5.4E0 | 1529 | 110 | 315 | 110 | 0.59 |
| aC | mg/mL | 3.2E0 | 2.4E0 | 3.4E0 | 2.6E0 | 1.5E0 | 9.9E-1 | 1.1E0 | 1.3E0 | 8.9E0 | 5.5E0 | 262 | 51 | 110 | 51 | 0.33 |
| aD | ug/mL | 3.1E0 | 3.6E0 | 4.5E0 | 4.9E0 | 4.0E0 | 3.8E0 | 4.3E-1 | 8.4E-1 | 3.5E1 | 2.0E1 | 262 | 51 | 110 | 51 | 0.56 |
| aE | mg/mL | 5.8E-1 | 5.3E-1 | 5.8E-1 | 5.4E-1 | 1.4E-1 | 1.4E-1 | 2.1E-1 | 3.1E-1 | 1.1E0 | 1.2E0 | 262 | 51 | 110 | 51 | 0.40 |
| aF | ng/mL | 2.0E0 | 1.6E0 | 4.1E0 | 3.6E0 | 6.5E0 | 4.5E0 | 4.3E-3 | 4.3E-3 | 5.0E1 | 1.8E1 | 262 | 51 | 110 | 51 | 0.45 |
| aG | mg/mL | 1.3E-1 | 1.4E-1 | 1.6E-1 | 1.5E-1 | 9.4E-2 | 8.2E-2 | 1.7E-2 | 5.8E-2 | 5.4E-1 | 3.8E-1 | 262 | 51 | 110 | 51 | 0.51 |
| aH | ug/mL | 7.0E1 | 8.0E1 | 7.8E1 | 9.1E1 | 3.8E1 | 5.1E1 | 4.6E0 | 1.1E1 | 2.7E2 | 2.6E2 | 262 | 51 | 110 | 51 | 0.57 |
| aI | ug/mL | 1.9E2 | 2.0E2 | 1.9E2 | 1.9E2 | 6.0E1 | 5.5E1 | 2.8E1 | 4.8E1 | 3.7E2 | 3.0E2 | 262 | 51 | 110 | 51 | 0.51 |
| aJ | ug/mL | 2.4E0 | 2.6E0 | 2.9E0 | 3.7E0 | 1.9E0 | 3.1E0 | 8.5E-1 | 7.6E-1 | 1.2E1 | 1.6E1 | 262 | 51 | 110 | 51 | 0.57 |
| aK | ng/mL | 1.8E0 | 1.6E0 | 2.7E0 | 1.9E0 | 2.8E0 | 1.7E0 | 2.8E-2 | 2.9E-4 | 1.8E1 | 7.0E0 | 262 | 51 | 110 | 51 | 0.43 |
| aL | mg/mL | 8.3E-1 | 8.0E-1 | 8.4E-1 | 8.2E-1 | 2.7E-1 | 2.4E-1 | 1.9E-1 | 3.0E-1 | 1.7E0 | 1.5E0 | 262 | 51 | 110 | 51 | 0.47 |
| aM | U/mL | 1.9E1 | 3.0E1 | 4.2E1 | 4.2E1 | 1.1E2 | 3.9E1 | 4.2E-2 | 4.2E-2 | 1.6E3 | 1.5E2 | 262 | 51 | 110 | 51 | 0.59 |
| aN | U/mL | 9.3E0 | 1.8E1 | 1.4E1 | 2.8E1 | 1.5E1 | 3.0E1 | 2.5E-3 | 2.5E-3 | 9.8E1 | 1.1E2 | 262 | 51 | 110 | 51 | 0.67 |
| aO | pg/mL | 2.9E1 | 5.1E1 | 3.0E2 | 4.5E2 | 8.5E2 | 9.7E2 | 6.0E-2 | 6.0E-2 | 6.6E3 | 3.6E3 | 262 | 51 | 110 | 51 | 0.56 |
| aP | ng/mL | 1.7E0 | 1.7E0 | 2.1E0 | 2.2E0 | 2.1E0 | 1.5E0 | 4.5E-1 | 7.5E-1 | 2.8E1 | 6.5E0 | 262 | 51 | 110 | 51 | 0.53 |
| aQ | ng/mL | 3.2E-1 | 2.9E-1 | 4.9E-1 | 4.1E-1 | 5.0E-1 | 3.5E-1 | 1.9E-2 | 2.5E-2 | 4.0E0 | 1.3E0 | 262 | 51 | 110 | 51 | 0.45 |
| aR | ng/mL | 1.7E0 | 2.1E0 | 2.3E0 | 3.6E0 | 2.3E0 | 4.9E0 | 1.8E-1 | 4.5E-1 | 2.1E1 | 3.0E1 | 262 | 51 | 110 | 51 | 0.59 |
| aS | ng/mL | 2.4E-1 | 3.0E-1 | 6.5E-1 | 5.1E-1 | 2.3E0 | 8.9E-1 | 4.2E-3 | 4.2E-3 | 3.3E1 | 6.1E0 | 262 | 51 | 110 | 51 | 0.55 |
| aU | pg/mL | 8.7E1 | 7.7E1 | 1.4E2 | 1.0E2 | 1.6E2 | 9.9E1 | 7.4E-2 | 7.4E-2 | 1.3E3 | 4.8E2 | 262 | 51 | 110 | 51 | 0.42 |
| aV | ng/mL | 7.0E-1 | 6.1E-1 | 1.1E0 | 8.5E-1 | 1.2E0 | 8.5E-1 | 2.2E-2 | 3.8E-2 | 8.7E0 | 4.2E0 | 262 | 51 | 110 | 51 | 0.44 |
| aW | pg/mL | 1.7E1 | 2.2E1 | 1.9E1 | 2.8E1 | 2.4E1 | 6.0E1 | 7.2E-2 | 7.2E-2 | 2.4E2 | 4.5E2 | 262 | 51 | 110 | 51 | 0.61 |
| aX | ng/mL | 1.0E1 | 7.9E0 | 1.6E1 | 1.2E1 | 2.2E1 | 1.0E1 | 3.0E-1 | 1.4E0 | 2.2E2 | 4.6E1 | 262 | 51 | 110 | 51 | 0.46 |
| aY | pg/mL | 5.3E1 | 6.5E1 | 7.3E1 | 8.3E1 | 7.0E1 | 7.1E1 | 4.1E-1 | 4.1E-1 | 4.4E2 | 3.7E2 | 262 | 51 | 110 | 51 | 0.57 |
| aZ | pg/mL | 2.3E2 | 1.9E2 | 4.4E2 | 3.7E2 | 5.6E2 | 8.4E2 | 1.7E0 | 1.7E0 | 3.4E3 | 5.9E3 | 262 | 51 | 110 | 51 | 0.44 |
| bA | ng/mL | 6.8E0 | 1.2E1 | 2.4E1 | 4.5E1 | 6.9E1 | 9.6E1 | 3.0E-2 | 3.0E-2 | 7.1E2 | 6.2E2 | 262 | 51 | 110 | 51 | 0.60 |
| bB | ng/mL | 3.1E2 | 3.1E2 | 3.4E2 | 3.2E2 | 1.6E2 | 1.8E2 | 2.1E0 | 2.3E1 | 7.4E2 | 7.6E2 | 262 | 51 | 110 | 51 | 0.47 |
| bC | ng/mL | 3.4E2 | 4.1E2 | 5.2E2 | 8.0E2 | 6.0E2 | 1.1E3 | 2.7E1 | 9.8E0 | 4.0E3 | 4.7E3 | 262 | 51 | 110 | 51 | 0.57 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bE | mg/mL | 5.8E0 | 5.3E0 | 6.1E0 | 6.1E0 | 2.0E0 | 2.3E0 | 9.8E-1 | 1.4E0 | 1.2E1 | 1.2E1 | 262 | 51 | 110 | 51 | 0.48 |
| bF | pg/mL | 1.9E1 | 2.5E1 | 5.7E1 | 9.4E1 | 2.6E2 | 3.1E2 | 5.0E-2 | 2.1E0 | 3.3E3 | 2.2E3 | 262 | 51 | 110 | 51 | 0.59 |
| bG | ng/mL | 1.7E0 | 1.3E0 | 2.9E0 | 2.5E0 | 3.3E0 | 3.0E0 | 2.2E-2 | 2.4E-1 | 2.3E1 | 1.5E1 | 262 | 51 | 110 | 51 | 0.45 |
| bH | pg/mL | 5.7E-1 | 5.7E-1 | 5.1E0 | 2.5E0 | 1.8E1 | 4.8E0 | 5.7E-1 | 5.7E-1 | 2.8E2 | 2.2E1 | 262 | 51 | 110 | 51 | 0.42 |
| bI | ng/mL | 4.0E-3 | 4.0E-3 | 6.0E-2 | 4.6E-2 | 1.5E-1 | 1.1E-1 | 4.0E-3 | 4.0E-3 | 1.3E0 | 4.5E-1 | 262 | 51 | 110 | 51 | 0.49 |
| bJ | mg/mL | 2.6E0 | 2.1E0 | 3.0E0 | 2.5E0 | 2.2E0 | 2.0E0 | 2.5E-4 | 2.5E-4 | 1.3E1 | 1.0E1 | 262 | 51 | 110 | 51 | 0.44 |
| bL | pg/mL | 4.1E0 | 2.4E0 | 8.5E0 | 5.7E0 | 1.1E1 | 7.5E0 | 4.6E-2 | 4.6E-2 | 8.0E1 | 2.7E1 | 262 | 51 | 110 | 51 | 0.39 |
| bM | mg/mL | 1.5E0 | 2.0E0 | 1.9E0 | 2.5E0 | 1.3E0 | 1.4E0 | 9.2E-3 | 4.1E-1 | 8.8E0 | 6.3E0 | 262 | 51 | 110 | 51 | 0.65 |
| bN | ng/mL | 3.1E1 | 5.4E1 | 1.3E2 | 1.1E2 | 3.1E2 | 1.9E2 | 1.4E-1 | 1.4E-1 | 1.9E3 | 1.2E3 | 262 | 51 | 110 | 51 | 0.55 |
| bO | ng/mL | 4.0E-2 | 4.0E-2 | 1.1E1 | 1.3E1 | 2.4E1 | 3.2E1 | 4.0E-2 | 4.0E-2 | 1.9E2 | 1.9E2 | 262 | 51 | 110 | 51 | 0.49 |
| bP | mg/mL | 6.3E-1 | 4.9E-1 | 8.5E-1 | 7.1E-1 | 7.0E-1 | 6.5E-1 | 4.9E-2 | 1.3E-1 | 3.8E0 | 3.1E0 | 262 | 51 | 110 | 51 | 0.43 |
| bQ | pg/mL | 1.5E1 | 1.6E1 | 2.7E1 | 2.2E1 | 5.0E1 | 3.1E1 | 1.5E-1 | 1.5E-1 | 4.8E2 | 2.2E2 | 262 | 51 | 110 | 51 | 0.49 |
| bR | ng/mL | 1.2E-2 | 1.2E-2 | 1.3E-1 | 9.3E-2 | 2.5E-1 | 1.8E-1 | 1.2E-2 | 1.2E-2 | 3.4E0 | 1.2E0 | 262 | 51 | 110 | 51 | 0.44 |
| bS | pg/mL | 9.4E-1 | 9.4E-1 | 7.8E0 | 3.8E0 | 2.7E1 | 1.1E1 | 9.4E-1 | 9.4E-1 | 3.4E2 | 5.4E1 | 262 | 51 | 110 | 51 | 0.46 |
| bU | ng/mL | 1.6E-1 | 1.3E-2 | 2.1E-1 | 1.5E-1 | 2.3E-1 | 2.7E-1 | 1.3E-2 | 1.3E-2 | 1.8E0 | 1.7E0 | 262 | 51 | 110 | 51 | 0.37 |
| bV | pg/mL | 4.7E2 | 4.5E2 | 5.7E2 | 6.0E2 | 7.2E2 | 4.6E2 | 1.6E2 | 1.7E2 | 1.2E4 | 3.1E3 | 262 | 51 | 110 | 51 | 0.52 |
| bW | pg/mL | 3.5E2 | 3.5E2 | 5.2E2 | 5.9E2 | 4.8E2 | 9.0E2 | 1.1E2 | 9.2E1 | 3.4E3 | 6.4E3 | 262 | 51 | 110 | 51 | 0.51 |
| bX | ng/mL | 1.8E-3 | 2.5E-5 | 3.0E-3 | 1.9E-3 | 3.7E-3 | 2.8E-3 | 2.5E-5 | 2.5E-5 | 2.0E-2 | 1.1E-2 | 262 | 51 | 110 | 51 | 0.42 |
| bZ | pg/mL | 2.5E2 | 2.5E2 | 8.0E2 | 4.7E2 | 3.3E3 | 6.1E2 | 1.5E-1 | 1.5E-1 | 4.4E4 | 2.7E3 | 262 | 51 | 110 | 51 | 0.49 |
| cA | pg/mL | 6.0E-1 | 6.0E-1 | 2.0E0 | 1.4E0 | 3.5E0 | 2.5E0 | 6.0E-1 | 6.0E-1 | 1.5E1 | 1.0E1 | 262 | 51 | 110 | 51 | 0.48 |
| cB | ng/mL | 6.7E-2 | 4.0E-2 | 9.8E-2 | 7.4E-2 | 1.0E-1 | 1.0E-1 | 1.7E-3 | 1.7E-3 | 5.4E-1 | 5.3E-1 | 262 | 51 | 110 | 51 | 0.39 |
| cC | pg/mL | 4.6E1 | 3.6E1 | 4.9E1 | 3.6E1 | 3.6E1 | 3.0E1 | 1.0E0 | 1.0E0 | 3.7E2 | 1.4E2 | 262 | 51 | 110 | 51 | 0.38 |
| cD | pg/mL | 6.1E0 | 3.4E0 | 1.3E1 | 1.2E1 | 5.9E1 | 2.4E1 | 3.3E-1 | 3.3E-1 | 7.2E2 | 1.4E2 | 262 | 51 | 110 | 51 | 0.39 |
| cE | pg/mL | 3.1E1 | 3.9E1 | 1.1E2 | 1.3E2 | 2.8E2 | 4.4E2 | 1.2E-1 | 1.2E-1 | 3.1E3 | 3.1E3 | 262 | 51 | 110 | 51 | 0.50 |
| cF | pg/mL | 1.4E1 | 9.4E0 | 2.3E1 | 1.7E1 | 3.2E1 | 3.2E1 | 5.3E-1 | 5.3E-1 | 2.2E2 | 2.2E2 | 262 | 51 | 110 | 51 | 0.43 |
| cG | pg/mL | 4.2E1 | 4.9E1 | 6.7E1 | 1.1E2 | 9.1E1 | 2.4E2 | 9.6E0 | 1.1E1 | 1.1E3 | 1.6E3 | 262 | 51 | 110 | 51 | 0.56 |
| cH | uIU/mL | 2.8E0 | 3.2E0 | 5.6E0 | 7.0E0 | 8.3E0 | 1.1E1 | 8.6E-3 | 8.6E-3 | 8.7E1 | 5.2E1 | 262 | 51 | 110 | 51 | 0.51 |
| cI | ng/mL | 5.5E0 | 6.0E0 | 1.1E1 | 1.6E1 | 1.4E1 | 2.3E1 | 1.0E-3 | 7.1E-2 | 1.0E2 | 1.2E2 | 262 | 51 | 110 | 51 | 0.51 |
| cJ | ug/mL | 7.0E1 | 5.7E1 | 1.2E2 | 1.3E2 | 1.4E2 | 1.7E2 | 4.0E0 | 1.1E1 | 9.6E2 | 6.9E2 | 262 | 51 | 110 | 51 | 0.49 |
| cK | ng/mL | 3.8E-3 | 3.8E-3 | 6.9E-2 | 8.3E-3 | 2.1E-1 | 2.4E-2 | 3.8E-3 | 3.8E-3 | 1.7E0 | 1.6E-1 | 262 | 51 | 110 | 51 | 0.42 |
| cL | pg/mL | 1.9E2 | 2.0E2 | 2.7E2 | 3.6E2 | 4.7E2 | 8.2E2 | 2.5E1 | 1.6E1 | 7.1E3 | 5.8E3 | 262 | 51 | 110 | 51 | 0.50 |
| cM | pg/mL | 2.8E2 | 2.5E2 | 3.1E2 | 2.9E2 | 1.8E2 | 2.2E2 | 2.5E1 | 3.3E1 | 1.2E3 | 1.5E3 | 262 | 51 | 110 | 51 | 0.44 |
| cN | pg/mL | 1.2E2 | 1.2E2 | 1.2E2 | 1.4E2 | 4.2E1 | 5.0E1 | 3.8E1 | 4.3E1 | 3.0E2 | 2.7E2 | 262 | 51 | 110 | 51 | 0.59 |
| cO | pg/mL | 2.3E2 | 2.1E2 | 2.9E2 | 2.4E2 | 2.0E2 | 1.1E2 | 5.4E1 | 6.1E1 | 1.7E3 | 6.3E2 | 262 | 51 | 110 | 51 | 0.44 |
| cP | ng/mL | 2.4E3 | 2.8E3 | 2.5E3 | 2.8E3 | 8.7E2 | 9.2E2 | 6.2E2 | 1.5E3 | 5.7E3 | 5.5E3 | 262 | 51 | 110 | 51 | 0.61 |
| cQ | ng/mL | 3.9E-2 | 5.9E-2 | 1.2E-1 | 1.4E-1 | 2.1E-1 | 2.4E-1 | 2.0E-3 | 2.0E-3 | 1.5E0 | 1.4E0 | 262 | 51 | 110 | 51 | 0.56 |
| cR | ng/mL | 3.2E2 | 3.2E2 | 5.4E2 | 4.7E2 | 8.7E2 | 4.4E2 | 2.3E1 | 3.0E1 | 8.9E3 | 2.3E3 | 262 | 51 | 110 | 51 | 0.51 |
| cS | ng/mL | 2.5E2 | 2.5E2 | 4.2E2 | 4.1E2 | 4.6E2 | 4.2E2 | 4.1E1 | 7.0E1 | 2.7E3 | 2.6E3 | 262 | 51 | 110 | 51 | 0.50 |
| cT | ng/mL | 2.7E1 | 3.4E1 | 6.4E1 | 1.0E2 | 1.3E2 | 1.7E2 | 4.6E0 | 6.9E0 | 1.7E3 | 7.4E2 | 262 | 51 | 110 | 51 | 0.59 |
| cU | ng/mL | 5.6E1 | 5.4E1 | 7.3E1 | 6.6E1 | 6.8E1 | 4.6E1 | 9.2E0 | 1.4E1 | 7.7E2 | 2.7E2 | 262 | 51 | 110 | 51 | 0.49 |
| cV | ng/mL | 1.6E-1 | 1.8E-1 | 4.4E-1 | 2.5E-1 | 2.9E0 | 3.1E-1 | 2.5E-2 | 3.7E-2 | 4.7E1 | 2.0E0 | 262 | 51 | 110 | 51 | 0.49 |
| cW | mIU/mL | 5.1E-2 | 4.9E-2 | 2.0E-1 | 6.8E-2 | 9.3E-1 | 6.0E-2 | 3.7E-4 | 1.0E-2 | 9.7E0 | 2.7E-1 | 262 | 51 | 110 | 51 | 0.48 |
| cX | ng/mL | 9.7E-2 | 1.8E-1 | 1.3E0 | 1.3E0 | 4.3E0 | 3.3E0 | 2.3E-4 | 2.3E-4 | 2.8E1 | 2.2E1 | 262 | 51 | 110 | 51 | 0.54 |
| cY | ng/mL | 9.9E0 | 7.8E0 | 1.4E1 | 1.1E1 | 1.3E1 | 8.8E0 | 2.5E-1 | 1.5E-1 | 8.3E1 | 3.2E1 | 262 | 51 | 110 | 51 | 0.45 |
| cZ | ug/mL | 1.5E1 | 1.6E1 | 1.6E1 | 1.6E1 | 7.5E0 | 7.3E0 | 2.3E0 | 3.1E0 | 5.7E1 | 3.7E1 | 262 | 51 | 110 | 51 | 0.52 |
| dA | pg/mL | 3.3E2 | 3.3E2 | 3.6E2 | 3.7E2 | 1.8E2 | 1.6E2 | 9.0E1 | 1.3E2 | 1.3E3 | 8.2E2 | 262 | 51 | 110 | 51 | 0.51 |
| dB | ug/mL | 1.7E1 | 1.5E1 | 1.9E1 | 1.5E1 | 2.0E1 | 1.2E1 | 2.1E0 | 1.8E0 | 2.5E2 | 4.1E1 | 262 | 51 | 110 | 51 | 0.45 |
| dC | nmol/L | 3.5E1 | 3.7E1 | 4.0E1 | 3.9E1 | 1.9E1 | 1.6E1 | 7.9E0 | 1.5E1 | 1.4E2 | 8.7E1 | 262 | 51 | 110 | 51 | 0.50 |
| dD | ug/mL | 3.8E1 | 3.7E1 | 3.9E1 | 3.7E1 | 1.1E1 | 1.0E1 | 1.3E1 | 1.3E1 | 7.6E1 | 5.7E1 | 262 | 51 | 110 | 51 | 0.44 |
| dE | ng/mL | 5.1E-1 | 8.4E-3 | 6.9E-1 | 4.0E-1 | 8.1E-1 | 6.4E-1 | 8.4E-3 | 8.4E-3 | 7.2E0 | 3.3E0 | 262 | 51 | 110 | 51 | 0.34 |
| dF | ng/mL | 2.1E2 | 2.5E2 | 2.6E2 | 2.7E2 | 1.8E2 | 1.4E2 | 7.5E1 | 8.6E1 | 1.2E3 | 6.6E2 | 262 | 51 | 110 | 51 | 0.55 |
| dG | ng/mL | 1.1E1 | 1.3E1 | 1.4E1 | 1.5E1 | 1.0E1 | 7.8E0 | 2.5E0 | 3.3E0 | 8.1E1 | 3.4E1 | 262 | 51 | 110 | 51 | 0.58 |
| dH | pg/mL | 7.5E0 | 7.5E0 | 1.2E1 | 9.9E0 | 2.8E1 | 9.2E0 | 4.0E-2 | 4.0E-2 | 3.1E2 | 5.0E1 | 262 | 51 | 110 | 51 | 0.50 |

| Code | Units | Median | | Average | | Standard Deviation | | Minimum | | Maximum | | Number of Samples | | Number of Patients | | AUC |
|------|-------|--------|--------|---------|--------|------|------|--------|--------|--------|--------|--------|--------|--------|--------|------|
| | | NonDis | Dis | NonDis | Dis | NonDis | Dis | NonDis | Dis | NonDis | Dis | NonDis | Dis | NonDis | Dis | |
| dI | pg/mL | 4.6E-1 | 4.6E-1 | 1.9E0 | 8.6E-1 | 4.8E0 | 1.1E0 | 4.6E-1 | 4.6E-1 | 4.2E1 | 6.0E0 | 262 | 51 | 110 | 51 | 0.46 |
| dJ | ng/mL | 1.9E0 | 2.2E0 | 2.2E0 | 2.2E0 | 1.1E0 | 1.1E0 | 3.2E-2 | 4.9E-1 | 5.9E0 | 5.1E0 | 262 | 51 | 110 | 51 | 0.52 |
| dK | uIU/mL | 2.0E0 | 1.6E0 | 2.6E0 | 2.7E0 | 2.6E0 | 3.7E0 | 2.8E-4 | 3.8E-2 | 1.6E1 | 2.1E1 | 262 | 51 | 110 | 51 | 0.47 |
| dL | ng/mL | 8.9E2 | 9.4E2 | 1.0E3 | 1.1E3 | 5.0E2 | 5.2E2 | 3.4E2 | 4.7E2 | 3.4E3 | 3.3E3 | 262 | 51 | 110 | 51 | 0.56 |
| dM | pg/mL | 9.7E2 | 1.1E3 | 1.2E3 | 1.3E3 | 1.0E3 | 1.2E3 | 3.5E2 | 4.2E2 | 1.2E4 | 8.3E3 | 262 | 51 | 110 | 51 | 0.53 |
| dN | ug/mL | 9.0E1 | 9.8E1 | 9.9E1 | 1.1E2 | 3.7E1 | 3.7E1 | 2.5E1 | 3.7E1 | 2.8E2 | 2.1E2 | 262 | 51 | 110 | 51 | 0.57 |
| dO | ng/ml | 2.5E1 | 1.5E1 | 4.4E1 | 2.4E1 | 6.0E1 | 2.6E1 | 4.0E-1 | 4.6E0 | 3.7E2 | 7.9E1 | 58 | 11 | 38 | 11 | 0.39 |
| dR | pg/ml | 1.8E3 | 1.7E3 | 2.5E3 | 2.1E3 | 2.4E3 | 1.7E3 | 1.9E2 | 1.7E2 | 1.5E4 | 8.1E3 | 150 | 50 | 104 | 50 | 0.47 |
| dU | pg/ml | 9.1E3 | 1.9E4 | 1.2E4 | 2.3E4 | 1.1E4 | 1.2E4 | 2.5E3 | 6.7E3 | 5.3E4 | 3.8E4 | 27 | 8 | 26 | 8 | 0.80 |
| dV | pg/ml | 7.0E1 | 1.0E2 | 8.9E1 | 2.0E2 | 6.1E1 | 2.8E2 | 2.1E1 | 3.8E1 | 3.3E2 | 8.9E2 | 48 | 8 | 27 | 8 | 0.62 |
| dX | ng/ml | 5.2E-2 | 5.2E-2 | 1.1E-1 | 2.2E-1 | 1.5E-1 | 3.8E-1 | 2.6E-3 | 2.6E-3 | 7.4E-1 | 9.4E-1 | 89 | 9 | 31 | 9 | 0.46 |
| eF | ng/ml | 4.0E0 | 4.6E0 | 5.2E0 | 5.1E0 | 5.6E0 | 2.3E0 | 1.2E0 | 1.6E0 | 4.6E1 | 1.2E1 | 158 | 51 | 105 | 51 | 0.59 |
| eC | pg/ml | 3.0E2 | 3.1E2 | 3.7E2 | 3.4E2 | 2.5E2 | 1.5E2 | 9.9E0 | 5.1E1 | 1.4E3 | 6.2E2 | 105 | 45 | 97 | 45 | 0.51 |
| eD | pg/ml | 2.3E2 | 2.2E2 | 7.6E2 | 3.0E2 | 1.5E3 | 2.8E2 | 5.2E-1 | 5.2E-1 | 8.3E3 | 1.4E3 | 81 | 37 | 74 | 37 | 0.47 |
| eM | ng/ml | 3.2E0 | 4.2E0 | 4.3E0 | 7.3E0 | 4.2E0 | 6.8E0 | 7.6E-1 | 9.0E-1 | 2.7E1 | 2.5E1 | 110 | 17 | 44 | 17 | 0.64 |
| eP | ng/ml | 3.7E-3 | 4.5E-1 | 8.1E-1 | 7.8E0 | 1.9E0 | 2.1E1 | 3.7E-3 | 3.7E-3 | 1.2E1 | 6.5E1 | 89 | 9 | 31 | 9 | 0.66 |
| eT | ng/ml | 2.8E2 | 2.4E2 | 6.0E2 | 3.9E2 | 6.5E2 | 4.5E2 | 1.0E2 | 1.2E2 | 2.5E3 | 1.9E3 | 55 | 16 | 54 | 16 | 0.44 |
| eQ | pg/ml | 1.0E0 | 1.0E0 | 1.2E2 | 5.8E0 | 3.3E2 | 1.4E1 | 1.0E0 | 1.0E0 | 1.6E3 | 4.0E1 | 27 | 8 | 26 | 8 | 0.38 |
| fA | ng/ml | 2.0E2 | 1.7E2 | 4.4E2 | 2.2E2 | 4.9E2 | 1.5E2 | 2.6E1 | 6.8E1 | 1.5E3 | 4.7E2 | 27 | 8 | 26 | 8 | 0.48 |
| eZ | ng/ml | 5.4E1 | 5.2E1 | 6.2E1 | 5.6E1 | 2.5E1 | 2.3E1 | 2.3E1 | 2.5E1 | 1.2E2 | 1.1E2 | 55 | 16 | 54 | 16 | 0.45 |
| fB | ng/ml | 6.0E2 | 7.0E2 | 6.8E2 | 8.5E2 | 2.9E2 | 3.9E2 | 1.6E2 | 4.4E2 | 1.3E3 | 1.5E3 | 28 | 8 | 27 | 8 | 0.62 |
| eX | ng/ml | 5.0E0 | 3.3E0 | 1.7E1 | 9.8E0 | 2.2E1 | 1.2E1 | 8.6E-4 | 3.3E-1 | 7.3E1 | 3.4E1 | 58 | 11 | 38 | 11 | 0.42 |
| fN | ng/ml | 2.1E-1 | 2.1E-1 | 3.9E0 | 1.4E0 | 9.2E0 | 2.8E0 | 2.1E-1 | 2.1E-1 | 5.4E1 | 8.9E0 | 55 | 16 | 54 | 16 | 0.42 |
| fP | ng/ml | 2.3E2 | 2.9E2 | 2.5E2 | 3.2E2 | 1.4E2 | 1.8E2 | 8.4E0 | 2.7E1 | 1.0E3 | 8.7E2 | 140 | 48 | 100 | 48 | 0.61 |
| fR | ng/ml | 1.2E5 | 1.4E5 | 1.7E5 | 2.2E5 | 1.3E5 | 1.8E5 | 3.1E4 | 3.6E4 | 7.7E5 | 7.2E5 | 202 | 27 | 60 | 27 | 0.58 |
| fY | ng/ml | 2.6E2 | 3.2E2 | 2.5E2 | 2.7E2 | 9.4E1 | 1.3E2 | 6.5E1 | 4.2E1 | 4.7E2 | 4.5E2 | 55 | 16 | 54 | 16 | 0.59 |
| gC | ng/ml | 2.3E2 | 2.4E2 | 2.7E2 | 2.7E2 | 1.4E2 | 1.3E2 | 1.2E2 | 1.3E2 | 1.1E3 | 5.9E2 | 84 | 19 | 49 | 19 | 0.51 |
| gN | U/ml | 3.7E2 | 4.6E2 | 4.5E2 | 5.4E2 | 3.1E2 | 3.3E2 | 1.9E0 | 1.2E2 | 1.3E3 | 9.2E2 | 48 | 8 | 27 | 8 | 0.59 |
| gL | pg/ml | 6.3E4 | 6.5E4 | 7.0E4 | 7.2E4 | 3.5E4 | 2.8E4 | 1.4E4 | 3.1E4 | 2.0E5 | 1.6E5 | 150 | 50 | 104 | 50 | 0.54 |
| gP | U/ml | 2.7E2 | 3.0E2 | 2.8E2 | 3.0E2 | 9.5E1 | 1.3E2 | 8.5E1 | 7.1E1 | 8.0E2 | 8.5E2 | 157 | 51 | 105 | 51 | 0.57 |
| gT | ng/ml | 2.0E1 | 2.3E1 | 2.0E1 | 2.4E1 | 5.0E0 | 5.1E0 | 1.2E1 | 1.8E1 | 3.6E1 | 3.2E1 | 49 | 8 | 31 | 8 | 0.72 |
| gW | ng/ml | 8.5E2 | 5.5E2 | 1.5E3 | 9.1E2 | 1.9E3 | 1.0E3 | 3.7E1 | 2.3E0 | 9.5E3 | 4.3E3 | 130 | 43 | 96 | 43 | 0.40 |
| gV | ng/ml | 2.1E1 | 1.7E1 | 2.2E1 | 2.3E1 | 7.5E0 | 9.8E0 | 2.9E-3 | 1.4E1 | 3.9E1 | 3.9E1 | 77 | 8 | 19 | 8 | 0.46 |
| tF | pg/mL | 1.6E3 | 9.0E2 | 2.0E4 | 7.3E3 | 5.4E4 | 1.8E4 | 1.2E1 | 1.8E1 | 3.2E5 | 9.4E4 | 105 | 45 | 97 | 45 | 0.45 |
| gZ | ug/ml | 8.0E-1 | 1.0E0 | 5.4E1 | 5.2E1 | 1.2E2 | 1.3E2 | 8.7E-2 | 1.1E-1 | 4.1E2 | 3.8E2 | 27 | 8 | 26 | 8 | 0.56 |
| hA | ng/ml | 2.1E0 | 2.1E0 | 7.1E0 | 8.1E0 | 2.4E1 | 2.1E1 | 1.7E-2 | 1.7E-2 | 1.6E2 | 1.0E2 | 81 | 37 | 74 | 37 | 0.52 |
| nM | pg/ml | 1.0E-9 | 1.0E-9 | 1.9E2 | 1.0E-9 | 1.4E3 | 0.0E0 | 1.0E-9 | 1.0E-9 | 1.0E4 | 1.0E-9 | 53 | 29 | 51 | 29 | 0.48 |
| nN | pg/ml | 1.0E3 | 1.8E3 | 1.8E3 | 2.5E3 | 2.6E3 | 2.7E3 | 1.1E2 | 1.2E2 | 1.7E4 | 1.3E4 | 53 | 29 | 51 | 29 | 0.62 |
| nO | pg/ml | 3.2E1 | 3.1E1 | 4.9E1 | 5.0E1 | 4.9E1 | 5.2E1 | 5.5E0 | 3.5E0 | 2.4E2 | 2.4E2 | 53 | 29 | 51 | 29 | 0.48 |
| nR | pg/ml | 1.3E1 | 1.9E1 | 2.9E1 | 6.9E1 | 4.6E1 | 1.5E2 | 1.0E-9 | 5.5E-1 | 2.6E2 | 7.1E2 | 53 | 29 | 51 | 29 | 0.59 |
| nT | pg/ml | 1.1E2 | 8.5E1 | 3.7E2 | 1.5E2 | 1.2E3 | 3.2E2 | 1.0E-9 | 1.0E-9 | 6.6E3 | 1.8E3 | 53 | 29 | 51 | 29 | 0.46 |
| nU | pg/ml | 1.4E1 | 3.1E1 | 5.3E2 | 1.7E2 | 2.3E3 | 5.4E2 | 1.0E-9 | 1.0E-9 | 1.3E4 | 3.0E3 | 53 | 29 | 51 | 29 | 0.58 |
| lW | pg/ml | 1.0E-9 | 1.0E-9 | 1.5E1 | 4.2E0 | 5.5E1 | 1.1E1 | 1.0E-9 | 1.0E-9 | 3.9E2 | 4.2E1 | 53 | 29 | 51 | 29 | 0.45 |
| lX | pg/ml | 1.1E3 | 9.9E2 | 1.1E3 | 1.0E3 | 5.5E2 | 5.5E2 | 2.3E2 | 1.2E2 | 2.6E3 | 2.3E3 | 53 | 29 | 51 | 29 | 0.47 |
| lY | pg/ml | 1.8E1 | 2.4E1 | 2.2E1 | 2.6E1 | 2.3E1 | 1.7E1 | 1.0E-9 | 5.4E0 | 1.4E2 | 9.6E1 | 53 | 29 | 51 | 29 | 0.62 |
| mE | pg/ml | 1.0E-9 | 1.0E-9 | 3.6E0 | 1.1E0 | 1.0E1 | 2.1E0 | 1.0E-9 | 1.0E-9 | 5.8E1 | 8.9E0 | 53 | 29 | 51 | 29 | 0.48 |
| mF | pg/ml | 1.0E-9 | 1.0E-9 | 1.8E0 | 1.1E0 | 3.3E0 | 1.9E0 | 1.0E-9 | 1.0E-9 | 1.5E1 | 6.1E0 | 53 | 29 | 51 | 29 | 0.47 |
| mH | pg/ml | 4.4E0 | 3.0E0 | 5.6E0 | 3.8E0 | 5.2E0 | 2.7E0 | 2.3E-1 | 3.2E-1 | 3.2E1 | 1.1E1 | 53 | 29 | 51 | 29 | 0.41 |
| mI | pg/ml | 1.0E-9 | 1.0E-9 | 1.4E1 | 9.4E0 | 3.1E1 | 1.6E1 | 1.0E-9 | 1.0E-9 | 1.6E2 | 7.8E1 | 53 | 29 | 51 | 29 | 0.50 |
| mM | pg/ml | 1.8E1 | 2.8E1 | 4.3E1 | 5.4E1 | 5.9E1 | 7.6E1 | 1.0E-9 | 1.0E-9 | 2.3E2 | 2.9E2 | 53 | 29 | 51 | 29 | 0.55 |
| mP | pg/ml | 1.4E1 | 1.5E1 | 1.8E1 | 1.5E1 | 2.2E1 | 8.9E0 | 1.0E-9 | 1.0E-9 | 1.5E2 | 3.7E1 | 52 | 29 | 50 | 29 | 0.48 |
| mS | pg/ml | 1.8E3 | 1.7E3 | 2.1E3 | 2.0E3 | 2.3E3 | 1.2E3 | 1.0E-9 | 1.0E-9 | 1.3E4 | 5.9E3 | 53 | 29 | 51 | 29 | 0.52 |
| mT | pg/ml | 5.3E1 | 5.2E1 | 1.5E2 | 1.4E2 | 2.8E2 | 3.5E2 | 9.7E0 | 1.6E1 | 1.4E3 | 1.9E3 | 52 | 29 | 50 | 29 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| mU | pg/ml | 2.5E0 | 2.7E0 | 4.1E0 | 2.9E0 | 8.7E0 | 2.1E0 | 1.0E-9 | 1.9E-1 | 5.8E1 | 1.1E1 | 52 | 29 | 50 | 29 | 0.54 |
| mW | pg/ml | 2.0E3 | 2.4E3 | 2.6E3 | 3.0E3 | 1.7E3 | 1.5E3 | 3.1E2 | 6.7E2 | 1.0E4 | 7.0E3 | 52 | 29 | 50 | 29 | 0.60 |
| mY | pg/ml | 4.8E2 | 6.4E2 | 9.9E2 | 8.2E2 | 1.8E3 | 6.3E2 | 1.0E-9 | 2.1E2 | 1.1E4 | 2.7E3 | 53 | 29 | 51 | 29 | 0.59 |
| mZ | pg/ml | 1.8E2 | 4.4E2 | 3.1E2 | 4.8E2 | 2.9E2 | 3.8E2 | 1.0E-9 | 5.3E1 | 1.2E3 | 1.7E3 | 52 | 29 | 50 | 29 | 0.66 |
| nA | pg/ml | 1.3E0 | 2.0E0 | 1.6E1 | 6.1E0 | 6.7E1 | 1.3E1 | 1.0E-9 | 1.0E-9 | 4.4E2 | 6.2E1 | 52 | 29 | 50 | 29 | 0.55 |
| nB | pg/ml | 2.9E2 | 3.3E2 | 3.0E2 | 3.7E2 | 1.6E2 | 1.4E2 | 3.0E1 | 1.4E2 | 7.0E2 | 7.2E2 | 53 | 29 | 51 | 29 | 0.65 |
| nC | pg/ml | 1.0E-9 | 1.0E-9 | 8.3E3 | 5.4E4 | 5.0E4 | 2.8E5 | 1.0E-9 | 1.0E-9 | 3.7E5 | 1.5E6 | 53 | 29 | 51 | 29 | 0.50 |
| nD | pg/ml | 6.7E0 | 8.5E0 | 7.2E1 | 1.3E1 | 3.2E2 | 2.7E1 | 1.0E-9 | 1.0E-9 | 1.7E3 | 1.5E2 | 52 | 29 | 50 | 29 | 0.50 |
| nF | pg/ml | 1.0E-9 | 1.0E-9 | 7.7E0 | 2.3E0 | 3.9E1 | 7.6E0 | 1.0E-9 | 1.0E-9 | 2.4E2 | 3.6E1 | 53 | 29 | 51 | 29 | 0.51 |
| nH | pg/ml | 3.8E-1 | 3.0E-2 | 2.1E2 | 1.1E2 | 1.4E3 | 4.9E2 | 1.0E-9 | 1.0E-9 | 1.0E4 | 2.6E3 | 52 | 29 | 50 | 29 | 0.51 |
| nI | pg/ml | 4.6E1 | 4.6E1 | 2.9E2 | 9.7E1 | 1.3E3 | 2.2E2 | 1.0E-9 | 1.0E-9 | 9.2E3 | 1.2E3 | 53 | 29 | 51 | 29 | 0.50 |
| nJ | pg/ml | 1.0E-9 | 5.9E-2 | 1.0E2 | 1.5E0 | 7.1E2 | 3.7E0 | 1.0E-9 | 1.0E-9 | 5.2E3 | 1.6E1 | 53 | 29 | 51 | 29 | 0.52 |
| nK | pg/ml | 1.0E-9 | 3.1E0 | 1.2E2 | 2.7E1 | 5.4E2 | 5.8E1 | 1.0E-9 | 1.0E-9 | 3.2E3 | 2.3E2 | 52 | 29 | 50 | 29 | 0.56 |
| nL | pg/ml | 1.0E-9 | 1.0E-9 | 9.2E2 | 2.6E2 | 6.1E3 | 9.8E2 | 1.0E-9 | 1.0E-9 | 4.5E4 | 5.2E3 | 53 | 29 | 51 | 29 | 0.49 |
| hL | pg/ml | 1.8E4 | 2.1E4 | 2.4E4 | 2.4E4 | 2.2E4 | 1.3E4 | 1.0E-9 | 5.7E3 | 1.4E5 | 4.6E4 | 55 | 16 | 54 | 16 | 0.57 |
| hO | pg/ml | 1.6E4 | 1.6E4 | 1.7E4 | 1.7E4 | 3.2E3 | 3.8E3 | 1.1E4 | 1.1E4 | 2.8E4 | 2.6E4 | 55 | 16 | 54 | 16 | 0.48 |
| hP | ng/ml | 4.4E5 | 4.4E5 | 3.5E5 | 4.3E5 | 1.8E5 | 2.9E5 | 2.3E4 | 1.7E4 | 9.0E5 | 1.1E6 | 55 | 16 | 54 | 16 | 0.56 |
| wJ | pg/ml | 1.5E5 | 1.8E5 | 1.6E5 | 1.9E5 | 6.9E4 | 1.2E5 | 3.2E4 | 6.0E4 | 3.1E5 | 5.1E5 | 54 | 13 | 53 | 13 | 0.56 |
| wK | pg/ml | 3.4E4 | 2.9E4 | 4.2E4 | 4.5E4 | 2.5E4 | 3.5E4 | 5.2E3 | 9.2E3 | 1.1E5 | 1.4E5 | 54 | 13 | 53 | 13 | 0.50 |
| wL | pg/ml | 5.8E0 | 7.9E-1 | 5.6E1 | 4.4E0 | 1.3E2 | 7.5E0 | 1.0E-9 | 1.0E-9 | 8.4E2 | 2.6E1 | 54 | 13 | 53 | 13 | 0.33 |
| wP | pg/ml | 2.8E4 | 2.2E4 | 4.2E4 | 5.0E4 | 4.0E4 | 8.1E4 | 1.1E3 | 2.9E3 | 1.6E5 | 3.0E5 | 54 | 13 | 53 | 13 | 0.43 |
| wQ | pg/ml | 3.5E1 | 3.9E1 | 6.3E1 | 5.9E1 | 7.8E1 | 8.1E1 | 1.0E-9 | 1.0E-9 | 3.7E2 | 3.0E2 | 54 | 13 | 53 | 13 | 0.49 |
| hR | pg/ml | 2.9E4 | 2.4E4 | 3.0E4 | 2.4E4 | 1.2E4 | 8.9E3 | 1.8E3 | 3.6E3 | 5.8E4 | 4.1E4 | 79 | 36 | 72 | 36 | 0.34 |
| hV | pg/ml | 4.7E2 | 3.5E2 | 5.1E2 | 4.1E2 | 2.5E2 | 2.1E2 | 1.3E2 | 6.8E1 | 1.5E3 | 8.2E2 | 79 | 36 | 72 | 36 | 0.38 |
| hW | pg/ml | 1.5E3 | 1.4E3 | 1.8E3 | 2.2E3 | 9.5E2 | 2.1E3 | 5.7E2 | 2.2E2 | 4.8E3 | 1.0E4 | 79 | 36 | 72 | 36 | 0.50 |
| hX | pg/ml | 9.3E2 | 8.7E2 | 1.1E3 | 1.0E3 | 1.0E3 | 7.8E2 | 3.6E2 | 2.3E2 | 8.6E3 | 4.9E3 | 79 | 36 | 72 | 36 | 0.44 |
| iA | pg/ml | 1.4E2 | 1.6E2 | 4.0E2 | 2.3E2 | 9.0E2 | 3.3E2 | 1.1E1 | 5.8E0 | 7.1E3 | 2.2E3 | 105 | 45 | 97 | 45 | 0.50 |
| iB | ng/ml | 4.9E0 | 4.3E0 | 6.3E0 | 5.8E0 | 4.7E0 | 5.0E0 | 3.7E-2 | 4.5E-2 | 1.9E1 | 2.0E1 | 81 | 37 | 74 | 37 | 0.47 |
| iC | U/ml | 2.2E-1 | 2.0E-1 | 4.4E-1 | 7.7E-1 | 8.3E-1 | 2.1E0 | 1.0E-9 | 1.0E-9 | 6.4E0 | 1.2E1 | 81 | 37 | 74 | 37 | 0.52 |
| tQ | pg/ml | 1.1E3 | 1.0E3 | 1.2E3 | 1.2E3 | 5.3E2 | 5.3E2 | 2.8E2 | 6.4E2 | 2.5E3 | 2.5E3 | 53 | 12 | 52 | 12 | 0.47 |
| tT | pg/ml | 1.7E1 | 1.4E1 | 1.8E1 | 1.9E1 | 9.7E0 | 1.5E1 | 7.4E0 | 9.0E0 | 6.9E1 | 6.1E1 | 53 | 12 | 52 | 12 | 0.45 |
| tS | pg/ml | 1.1E0 | 8.5E-1 | 1.4E0 | 8.5E-1 | 1.4E0 | 6.1E-1 | 1.0E-9 | 1.0E-9 | 8.5E0 | 1.9E0 | 53 | 12 | 52 | 12 | 0.38 |
| tX | pg/ml | 8.6E-1 | 8.7E-1 | 1.1E0 | 8.3E-1 | 8.6E-1 | 4.5E-1 | 2.5E-2 | 7.6E-2 | 4.4E0 | 1.5E0 | 53 | 12 | 52 | 12 | 0.45 |
| tO | pg/ml | 3.9E0 | 4.2E0 | 4.7E0 | 4.2E0 | 3.1E0 | 2.7E0 | 1.0E-9 | 8.6E-1 | 1.4E1 | 9.0E0 | 53 | 12 | 52 | 12 | 0.47 |
| tR | pg/ml | 2.2E-1 | 1.2E-1 | 2.9E-1 | 1.7E-1 | 2.9E-1 | 1.6E-1 | 1.0E-9 | 1.0E-9 | 1.5E0 | 5.0E-1 | 53 | 12 | 52 | 12 | 0.38 |
| tU | pg/ml | 8.7E0 | 1.0E1 | 1.1E1 | 1.1E1 | 7.1E0 | 6.4E0 | 1.6E0 | 3.6E0 | 3.1E1 | 2.7E1 | 53 | 12 | 52 | 12 | 0.52 |
| tN | pg/ml | 1.7E1 | 1.7E1 | 2.1E1 | 1.9E1 | 1.4E1 | 1.1E1 | 1.0E-9 | 6.0E0 | 8.0E1 | 4.2E1 | 53 | 12 | 52 | 12 | 0.46 |
| tV | ng/ml | 4.2E2 | 6.2E2 | 5.3E2 | 6.1E2 | 5.0E2 | 3.4E2 | 1.5E2 | 1.5E2 | 2.9E3 | 1.1E3 | 54 | 13 | 53 | 13 | 0.60 |
| iH | ng/ml | 1.5E5 | 1.5E5 | 1.5E5 | 1.5E5 | 4.4E4 | 5.1E4 | 7.1E4 | 7.2E4 | 2.4E5 | 2.6E5 | 105 | 45 | 97 | 45 | 0.52 |
| iJ | ng/ml | 5.1E4 | 5.0E4 | 5.2E4 | 5.2E4 | 2.2E4 | 2.1E4 | 5.5E3 | 8.0E3 | 1.0E5 | 9.7E4 | 105 | 45 | 97 | 45 | 0.50 |
| hB | ng/ml | 4.4E-1 | 3.7E-1 | 5.0E-1 | 4.6E-1 | 3.2E-1 | 2.7E-1 | 1.0E-9 | 1.2E-1 | 1.7E0 | 1.3E0 | 105 | 45 | 97 | 45 | 0.46 |
| hC | pg/ml | 3.7E3 | 4.1E3 | 5.7E3 | 7.3E3 | 7.5E3 | 7.8E3 | 1.0E-9 | 1.0E-9 | 5.5E4 | 3.0E4 | 105 | 45 | 97 | 45 | 0.57 |
| hF | ng/ml | 1.0E-9 | 1.0E-9 | 4.7E1 | 2.3E-1 | 4.0E2 | 1.4E0 | 1.0E-9 | 1.0E-9 | 4.0E3 | 9.6E0 | 105 | 45 | 97 | 45 | 0.50 |
| hG | pg/ml | 7.0E3 | 6.9E3 | 7.3E3 | 7.7E3 | 3.1E3 | 3.5E3 | 2.8E1 | 3.3E3 | 1.8E4 | 1.9E4 | 105 | 45 | 97 | 45 | 0.52 |
| iO | ng/ml | 3.8E5 | 3.2E5 | 4.1E5 | 3.6E5 | 1.9E5 | 1.8E5 | 1.1E4 | 8.3E4 | 1.1E6 | 9.0E5 | 105 | 45 | 97 | 45 | 0.43 |
| iP | ng/ml | 6.0E4 | 5.0E4 | 5.5E4 | 5.2E4 | 3.2E4 | 3.2E4 | 1.0E-9 | 2.4E3 | 2.5E5 | 1.5E5 | 105 | 45 | 97 | 45 | 0.47 |
| iZ | ng/ml | 1.6E3 | 1.6E3 | 1.8E3 | 2.0E3 | 7.5E2 | 1.2E3 | 4.7E2 | 8.8E2 | 5.1E3 | 6.5E3 | 105 | 45 | 97 | 45 | 0.54 |
| yH | pg/ml | 1.1E3 | 1.3E3 | 1.9E3 | 1.0E4 | 2.9E3 | 3.3E4 | 1.0E-9 | 1.6E2 | 1.5E4 | 1.2E5 | 54 | 14 | 53 | 14 | 0.51 |
| yK | U/ml | 1.9E1 | 3.7E1 | 4.8E1 | 5.8E1 | 8.2E1 | 6.9E1 | 1.0E-9 | 1.0E-9 | 3.8E2 | 2.5E2 | 54 | 14 | 53 | 14 | 0.61 |
| yJ | pg/ml | 3.4E4 | 5.3E4 | 4.6E4 | 5.6E4 | 3.4E4 | 3.3E4 | 1.7E3 | 1.7E4 | 1.6E5 | 1.1E5 | 54 | 14 | 53 | 14 | 0.59 |
| yD | ng/ml | 1.5E-2 | 1.4E-2 | 1.5E-2 | 1.4E-2 | 6.9E-3 | 4.7E-3 | 1.0E-9 | 7.6E-3 | 4.3E-2 | 2.2E-2 | 54 | 13 | 53 | 13 | 0.48 |
| jB | ng/ml | 2.8E5 | 2.3E5 | 2.8E5 | 2.7E5 | 9.6E4 | 1.7E5 | 5.7E4 | 1.2E5 | 4.7E5 | 6.2E5 | 27 | 8 | 26 | 8 | 0.40 |
| wB | pg/ml | 8.1E3 | 6.1E3 | 9.5E3 | 9.4E3 | 7.2E3 | 8.6E3 | 1.7E3 | 2.5E3 | 4.1E4 | 3.4E4 | 54 | 13 | 53 | 13 | 0.47 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| pY | pg/ml | 6.0E0 | 5.7E0 | 1.1E1 | 6.6E0 | 2.6E1 | 3.0E0 | 2.1E0 | 2.6E0 | 2.0E2 | 1.2E1 | 55 | 16 | 54 | 16 | 0.51 |
| rC | pg/ml | 1.9E3 | 1.3E3 | 2.3E3 | 2.2E3 | 2.2E3 | 2.5E3 | 9.3E1 | 6.6E1 | 1.5E4 | 1.5E4 | 78 | 37 | 72 | 37 | 0.48 |
| rB | pg/ml | 2.2E1 | 2.9E1 | 4.6E1 | 4.2E1 | 1.2E2 | 5.1E1 | 1.0E-9 | 1.0E-9 | 9.5E2 | 2.9E2 | 78 | 37 | 72 | 37 | 0.60 |
| zG | 2.5ng/ml | 2.2E-1 | 1.1E-1 | 4.8E-1 | 3.7E-1 | 7.5E-1 | 8.6E-1 | 1.0E-9 | 1.0E-9 | 4.4E0 | 3.3E0 | 54 | 14 | 53 | 14 | 0.36 |
| zH | 2.3mU/ml | 1.1E-1 | 9.8E-2 | 1.2E-1 | 9.3E-2 | 6.9E-2 | 3.1E-2 | 1.0E-2 | 1.6E-2 | 4.4E-1 | 1.3E-1 | 54 | 14 | 53 | 14 | 0.41 |
| zI | 2.6ng/ml | 1.9E0 | 1.6E0 | 3.4E0 | 3.3E0 | 3.6E0 | 4.0E0 | 6.3E-1 | 5.4E-1 | 1.6E1 | 1.6E1 | 54 | 14 | 53 | 14 | 0.45 |
| qA | ng/ml | 1.1E7 | 8.7E6 | 1.3E7 | 1.0E7 | 7.4E6 | 5.2E6 | 3.7E6 | 2.0E6 | 3.7E7 | 2.4E7 | 55 | 16 | 54 | 16 | 0.41 |
| qB | ng/ml | 6.1E5 | 5.7E5 | 8.3E5 | 7.1E5 | 5.9E5 | 5.0E5 | 2.1E5 | 2.3E5 | 2.9E6 | 2.2E6 | 55 | 16 | 54 | 16 | 0.44 |
| qC | ng/ml | 4.5E5 | 3.6E5 | 9.1E5 | 3.6E5 | 1.3E6 | 2.9E5 | 2.0E4 | 3.4E3 | 7.1E6 | 9.3E5 | 55 | 16 | 54 | 16 | 0.37 |
| qD | ng/ml | 1.6E7 | 1.5E7 | 2.0E7 | 1.6E7 | 1.0E7 | 8.0E6 | 1.2E6 | 4.9E6 | 5.2E7 | 4.2E7 | 55 | 16 | 54 | 16 | 0.38 |
| jD | ng/ml | 2.0E1 | 4.1E1 | 4.1E1 | 5.3E1 | 7.0E1 | 5.5E1 | 1.0E-9 | 1.0E-9 | 5.1E2 | 2.9E2 | 81 | 37 | 74 | 37 | 0.61 |
| jE | ng/ml | 1.0E-9 | 1.0E-9 | 8.5E0 | 7.9E0 | 2.1E1 | 1.6E1 | 1.0E-9 | 1.0E-9 | 1.2E2 | 5.6E1 | 81 | 37 | 74 | 37 | 0.51 |
| jF | ng/ml | 4.9E1 | 3.7E1 | 5.8E1 | 5.0E1 | 5.8E1 | 6.1E1 | 1.0E-9 | 1.0E-9 | 2.5E2 | 1.8E2 | 81 | 37 | 74 | 37 | 0.43 |
| jG | ng/ml | 4.4E3 | 4.9E3 | 4.4E3 | 4.7E3 | 1.9E3 | 1.7E3 | 7.6E2 | 6.0E2 | 8.9E3 | 7.9E3 | 81 | 37 | 74 | 37 | 0.55 |
| jH | ng/ml | 7.6E1 | 8.2E1 | 8.6E1 | 9.3E1 | 4.9E1 | 5.9E1 | 1.9E1 | 2.5E1 | 2.8E2 | 3.3E2 | 81 | 37 | 74 | 37 | 0.53 |
| jI | ng/ml | 6.3E1 | 7.5E1 | 6.9E1 | 7.7E1 | 3.5E1 | 3.1E1 | 1.9E1 | 3.5E1 | 2.5E2 | 1.9E2 | 81 | 37 | 74 | 37 | 0.61 |
| sK | pg/mL | 3.7E3 | 3.9E3 | 4.0E3 | 4.1E3 | 1.3E3 | 1.3E3 | 1.7E3 | 1.1E3 | 8.0E3 | 6.1E3 | 54 | 15 | 53 | 15 | 0.57 |
| sM | pg/mL | 7.3E4 | 7.7E4 | 7.5E4 | 7.9E4 | 2.1E4 | 2.2E4 | 3.3E4 | 3.9E4 | 1.5E5 | 1.2E5 | 54 | 15 | 53 | 15 | 0.57 |
| sO | pg/mL | 3.0E8 | 3.0E8 | 3.0E8 | 2.8E8 | 9.2E7 | 1.2E8 | 7.9E7 | 9.1E7 | 4.9E8 | 4.9E8 | 54 | 15 | 53 | 15 | 0.46 |
| wC | ng/ml | 1.6E0 | 1.7E0 | 2.2E0 | 1.9E0 | 2.3E0 | 1.0E0 | 2.5E-1 | 5.9E-1 | 1.5E1 | 3.7E0 | 54 | 13 | 53 | 13 | 0.54 |
| wD | ng/ml | 1.6E1 | 2.0E1 | 7.3E1 | 2.5E1 | 2.9E2 | 2.1E1 | 2.1E0 | 6.0E0 | 2.1E3 | 7.2E1 | 54 | 13 | 53 | 13 | 0.55 |
| wE | ng/ml | 4.9E1 | 4.9E1 | 5.3E1 | 4.8E1 | 2.5E1 | 1.3E1 | 7.0E0 | 3.0E1 | 1.4E2 | 7.2E1 | 54 | 13 | 53 | 13 | 0.44 |
| wG | ng/ml | 8.2E-2 | 1.0E-9 | 1.2E-1 | 5.1E-2 | 1.5E-1 | 8.6E-2 | 1.0E-9 | 1.0E-9 | 7.1E-1 | 2.8E-1 | 54 | 13 | 53 | 13 | 0.35 |
| wH | ng/ml | 1.9E-2 | 3.0E-3 | 1.6E-1 | 2.8E-2 | 4.9E-1 | 5.3E-2 | 1.0E-9 | 1.0E-9 | 2.9E0 | 1.7E-1 | 54 | 13 | 53 | 13 | 0.35 |
| wF | ng/ml | 1.7E-1 | 9.7E-3 | 2.5E0 | 1.6E-1 | 9.9E0 | 3.1E-1 | 1.0E-9 | 1.0E-9 | 5.7E1 | 1.1E0 | 54 | 13 | 53 | 13 | 0.36 |
| rA | pg/ml | 2.4E1 | 2.3E1 | 3.0E1 | 3.0E1 | 2.7E1 | 2.0E1 | 1.0E-9 | 4.2E0 | 2.0E2 | 8.5E1 | 80 | 38 | 74 | 38 | 0.52 |
| qZ | pg/ml | 4.3E1 | 4.0E1 | 1.9E2 | 7.3E2 | 1.2E3 | 2.6E3 | 2.8E-4 | 4.8E-4 | 1.0E4 | 1.0E4 | 71 | 29 | 68 | 29 | 0.47 |
| qY | pg/ml | 2.9E1 | 2.8E1 | 5.1E1 | 5.5E1 | 7.4E1 | 5.5E1 | 8.7E-1 | 3.3E0 | 5.3E2 | 2.2E2 | 80 | 38 | 74 | 38 | 0.55 |
| qX | pg/ml | 5.4E1 | 6.0E1 | 6.0E1 | 6.7E1 | 3.8E1 | 4.4E1 | 1.0E-9 | 1.0E-9 | 1.8E2 | 2.1E2 | 80 | 38 | 74 | 38 | 0.54 |
| qW | pg/ml | 9.7E0 | 9.1E0 | 1.5E1 | 1.4E1 | 2.0E1 | 1.6E1 | 1.0E-9 | 1.0E-9 | 1.2E2 | 8.1E1 | 80 | 38 | 74 | 38 | 0.50 |
| qV | pg/ml | 2.2E3 | 2.2E3 | 2.8E3 | 3.1E3 | 2.0E3 | 3.0E3 | 2.3E2 | 5.6E2 | 8.5E3 | 1.7E4 | 80 | 38 | 74 | 38 | 0.49 |
| qU | pg/ml | 5.5E1 | 5.5E1 | 1.7E2 | 9.3E1 | 2.6E2 | 1.1E2 | 1.0E-9 | 1.0E-9 | 1.4E3 | 4.7E2 | 80 | 38 | 74 | 38 | 0.45 |
| qT | pg/ml | 3.7E1 | 4.7E1 | 6.5E1 | 6.1E1 | 1.2E2 | 4.9E1 | 1.0E-9 | 1.0E-9 | 9.0E2 | 2.0E2 | 80 | 38 | 74 | 38 | 0.59 |
| qI | ng/ml | 5.4E4 | 7.6E4 | 6.2E4 | 6.7E4 | 3.1E4 | 3.0E4 | 1.1E4 | 5.9E3 | 1.6E5 | 1.0E5 | 54 | 17 | 54 | 17 | 0.59 |
| qH | ng/ml | 6.6E4 | 6.7E4 | 7.2E4 | 6.6E4 | 3.8E4 | 4.5E4 | 1.5E4 | 7.6E3 | 1.8E5 | 1.6E5 | 54 | 17 | 54 | 17 | 0.44 |
| qG | ng/ml | 1.8E5 | 2.2E5 | 1.9E5 | 2.0E5 | 5.9E4 | 8.8E4 | 5.8E4 | 1.7E4 | 3.3E5 | 3.1E5 | 54 | 17 | 54 | 17 | 0.59 |
| jK | ng/ml | 1.6E3 | 1.8E3 | 1.7E3 | 1.8E3 | 5.3E2 | 7.7E2 | 5.5E2 | 6.4E2 | 3.5E3 | 4.0E3 | 81 | 37 | 74 | 37 | 0.54 |
| jL | ng/ml | 1.7E2 | 2.5E2 | 2.5E2 | 3.5E2 | 1.9E2 | 3.6E2 | 3.6E1 | 4.8E1 | 9.6E2 | 2.1E3 | 81 | 37 | 74 | 37 | 0.61 |
| jM | ng/ml | 7.1E4 | 6.4E4 | 7.3E4 | 7.5E4 | 4.0E4 | 4.1E4 | 3.9E2 | 1.1E3 | 1.9E5 | 1.7E5 | 81 | 37 | 74 | 37 | 0.52 |
| jO | pg/ml | 2.1E5 | 2.2E5 | 2.7E5 | 2.5E5 | 1.7E5 | 1.3E5 | 5.2E4 | 7.7E4 | 1.1E6 | 6.4E5 | 81 | 37 | 74 | 37 | 0.49 |
| jP | pg/ml | 2.2E5 | 2.1E5 | 2.5E5 | 2.3E5 | 1.5E5 | 1.5E5 | 3.6E4 | 5.8E4 | 9.1E5 | 7.2E5 | 81 | 37 | 74 | 37 | 0.45 |
| jQ | pg/ml | 2.8E3 | 2.8E3 | 3.7E3 | 3.7E3 | 3.1E3 | 3.3E3 | 4.2E1 | 1.4E2 | 1.3E4 | 1.4E4 | 81 | 37 | 74 | 37 | 0.48 |
| jR | pg/ml | 8.6E3 | 5.9E3 | 1.3E4 | 1.0E4 | 1.3E4 | 9.4E3 | 1.0E-9 | 1.0E-9 | 6.8E4 | 3.0E4 | 81 | 37 | 74 | 37 | 0.46 |
| jT | pg/ml | 1.7E5 | 1.7E5 | 1.7E5 | 1.9E5 | 6.3E4 | 7.9E4 | 6.8E4 | 7.9E4 | 3.9E5 | 3.8E5 | 81 | 37 | 74 | 37 | 0.55 |
| xA | pg/ml | 3.9E0 | 4.7E0 | 1.5E1 | 7.5E0 | 5.4E1 | 1.0E1 | 1.0E-9 | 1.0E-9 | 3.9E2 | 3.0E1 | 54 | 14 | 53 | 14 | 0.50 |
| yE | pg/ml | 7.9E1 | 7.2E1 | 8.3E1 | 8.7E1 | 4.4E1 | 3.5E1 | 1.8E1 | 5.2E1 | 3.0E2 | 1.5E2 | 54 | 14 | 53 | 14 | 0.55 |
| tM | pg/ml | 3.9E1 | 3.5E1 | 4.2E1 | 3.9E1 | 2.1E1 | 1.6E1 | 1.0E-9 | 2.2E1 | 1.0E2 | 6.4E1 | 54 | 14 | 53 | 14 | 0.46 |
| tL | pg/ml | 1.0E-9 | 1.0E-9 | 6.9E0 | 1.9E-1 | 3.6E1 | 5.7E-1 | 1.0E-9 | 1.0E-9 | 2.6E2 | 2.1E0 | 54 | 14 | 53 | 14 | 0.44 |
| jU | mIU/ml | 3.5E0 | 4.8E0 | 1.0E1 | 7.8E0 | 1.8E1 | 1.1E1 | 8.9E-2 | 6.2E-2 | 8.1E1 | 6.7E1 | 81 | 37 | 74 | 37 | 0.52 |
| jV | mIU/ml | 1.5E0 | 1.2E0 | 3.4E0 | 3.3E0 | 5.5E0 | 5.9E0 | 3.4E-2 | 6.9E-4 | 3.1E1 | 2.6E1 | 81 | 37 | 74 | 37 | 0.44 |
| jY | ng/ml | 7.4E-4 | 7.6E-4 | 9.8E-3 | 3.7E-3 | 4.1E-2 | 6.7E-3 | 1.0E-9 | 1.0E-9 | 3.0E-1 | 2.7E-2 | 81 | 37 | 74 | 37 | 0.50 |
| kC | pg/ml | 9.7E1 | 8.9E1 | 2.3E2 | 1.3E2 | 5.6E2 | 1.2E2 | 2.9E1 | 2.1E1 | 3.5E3 | 6.8E2 | 53 | 29 | 51 | 29 | 0.49 |
| kE | pg/ml | 1.2E5 | 1.3E5 | 1.3E5 | 1.3E5 | 3.2E4 | 4.4E4 | 4.1E4 | 4.1E4 | 2.0E5 | 2.1E5 | 53 | 29 | 51 | 29 | 0.55 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| kF | pg/mL | 6.0E1 | 6.6E1 | 7.4E1 | 6.5E1 | 7.1E1 | 2.1E1 | 2.7E1 | 2.8E1 | 5.1E2 | 1.2E2 | 53 | 29 | 51 | 29 | 0.54 |
| kG | pg/mL | 8.5E3 | 1.0E4 | 9.4E3 | 1.6E4 | 7.8E3 | 1.8E4 | 7.5E2 | 1.1E3 | 4.3E4 | 9.1E4 | 53 | 29 | 51 | 29 | 0.61 |
| kI | pg/ml | 2.0E2 | 1.9E2 | 2.4E2 | 1.9E2 | 1.5E2 | 7.8E1 | 7.9E1 | 5.4E1 | 8.7E2 | 4.2E2 | 53 | 29 | 51 | 29 | 0.39 |
| kK | pg/ml | 1.0E2 | 9.8E1 | 1.6E2 | 1.6E2 | 1.9E2 | 1.7E2 | 6.4E0 | 2.9E1 | 1.2E3 | 6.9E2 | 53 | 29 | 51 | 29 | 0.48 |
| kN | pg/ml | 9.2E2 | 8.1E2 | 1.4E3 | 1.2E3 | 1.9E3 | 1.3E3 | 2.4E2 | 1.2E2 | 1.3E4 | 6.3E3 | 53 | 29 | 51 | 29 | 0.45 |
| kO | pg/ml | 7.7E3 | 7.1E3 | 1.0E4 | 8.2E3 | 1.8E4 | 3.9E3 | 3.7E3 | 3.4E3 | 1.3E5 | 1.9E4 | 53 | 29 | 51 | 29 | 0.46 |
| kP | pg/ml | 4.8E3 | 5.4E3 | 6.8E3 | 8.5E3 | 6.1E3 | 9.2E3 | 8.6E2 | 1.4E3 | 3.3E4 | 4.8E4 | 53 | 29 | 51 | 29 | 0.57 |
| kQ | pg/ml | 4.2E3 | 4.4E3 | 4.9E3 | 5.3E3 | 2.6E3 | 3.4E3 | 5.6E2 | 1.3E3 | 1.4E4 | 1.8E4 | 105 | 45 | 97 | 45 | 0.50 |
| kR | pg/ml | 2.0E1 | 2.0E1 | 3.6E1 | 2.2E1 | 1.0E2 | 1.4E1 | 1.0E-9 | 1.4E-1 | 1.0E3 | 6.9E1 | 105 | 45 | 97 | 45 | 0.48 |
| kS | pg/ml | 7.6E2 | 9.7E2 | 8.9E2 | 1.4E3 | 5.2E2 | 2.1E3 | 1.3E2 | 2.1E2 | 3.2E3 | 1.4E4 | 105 | 45 | 97 | 45 | 0.59 |
| pS | ng/ml | 1.8E5 | 2.6E5 | 2.1E5 | 2.6E5 | 9.0E4 | 8.6E4 | 9.7E4 | 1.4E5 | 5.0E5 | 3.6E5 | 54 | 15 | 53 | 15 | 0.68 |
| rZ | ng/ml | 1.0E-9 | 1.0E-9 | 7.1E-3 | 1.0E-2 | 2.0E-2 | 4.4E-2 | 1.0E-9 | 1.0E-9 | 1.1E-1 | 2.6E-1 | 78 | 35 | 71 | 35 | 0.46 |
| rY | ng/ml | 5.3E-2 | 6.1E-2 | 2.2E-1 | 5.6E-1 | 8.1E-1 | 2.8E0 | 1.0E-9 | 1.0E-9 | 6.3E0 | 1.7E1 | 78 | 35 | 71 | 35 | 0.51 |
| rX | ng/ml | 1.0E-9 | 1.0E-9 | 8.4E-2 | 5.8E-2 | 4.4E-1 | 3.2E-1 | 1.0E-9 | 1.0E-9 | 3.7E0 | 1.9E0 | 78 | 35 | 71 | 35 | 0.47 |
| lK | pg/ml | 7.5E1 | 1.6E2 | 1.6E2 | 2.5E2 | 1.9E2 | 5.4E2 | 1.0E-9 | 1.0E-9 | 7.4E2 | 3.3E3 | 81 | 37 | 74 | 37 | 0.54 |
| lL | pg/ml | 1.7E3 | 1.8E3 | 2.7E3 | 2.3E3 | 3.0E3 | 2.1E3 | 7.5E1 | 1.5E1 | 1.9E4 | 7.2E3 | 81 | 37 | 74 | 37 | 0.47 |
| lM | pg/ml | 1.1E3 | 1.3E3 | 2.2E3 | 3.7E3 | 3.0E3 | 7.1E3 | 1.3E2 | 2.2E2 | 1.6E4 | 3.4E4 | 81 | 37 | 74 | 37 | 0.56 |
| lN | pg/ml | 1.0E-9 | 1.0E-9 | 6.9E0 | 2.8E0 | 2.2E1 | 3.7E0 | 1.0E-9 | 1.0E-9 | 1.5E2 | 1.2E1 | 81 | 37 | 74 | 37 | 0.52 |
| lO | pg/ml | 1.0E-9 | 1.0E-9 | 5.0E-1 | 3.2E0 | 4.5E0 | 1.5E1 | 1.0E-9 | 1.0E-9 | 4.0E1 | 8.4E1 | 81 | 37 | 74 | 37 | 0.52 |
| zA | ng/ml | 1.9E7 | 1.9E7 | 1.9E7 | 1.9E7 | 6.6E6 | 6.0E6 | 6.7E6 | 6.7E6 | 3.4E7 | 2.8E7 | 50 | 12 | 49 | 12 | 0.50 |
| rW | ng/ml | 1.7E-2 | 3.7E-2 | 2.7E-2 | 4.3E-2 | 3.2E-2 | 4.4E-2 | 1.0E-9 | 1.0E-9 | 1.7E-1 | 1.8E-1 | 54 | 16 | 54 | 16 | 0.63 |
| rV | ng/ml | 1.0E-9 | 1.0E-9 | 1.2E-2 | 2.5E-3 | 4.0E-2 | 7.4E-3 | 1.0E-9 | 1.0E-9 | 2.2E-1 | 2.9E-2 | 54 | 16 | 54 | 16 | 0.47 |
| rU | ng/ml | 1.1E-1 | 5.5E-2 | 1.8E-1 | 9.3E-2 | 2.7E-1 | 9.5E-2 | 1.0E-9 | 1.0E-9 | 1.4E0 | 3.6E-1 | 54 | 16 | 54 | 16 | 0.42 |
| rT | ng/ml | 6.5E0 | 5.4E0 | 6.8E0 | 5.5E0 | 4.2E0 | 2.0E0 | 7.3E-1 | 1.1E0 | 2.1E1 | 1.1E1 | 54 | 16 | 54 | 16 | 0.40 |
| rS | ng/ml | 3.5E0 | 3.6E0 | 5.8E0 | 4.3E0 | 6.6E0 | 2.6E0 | 7.6E-1 | 3.9E-1 | 3.8E1 | 9.4E0 | 54 | 16 | 54 | 16 | 0.49 |
| sC | pg/mL | 5.8E3 | 1.2E4 | 9.5E3 | 1.5E4 | 8.6E3 | 1.2E4 | 1.7E3 | 2.5E3 | 4.4E4 | 4.2E4 | 54 | 15 | 53 | 15 | 0.65 |
| yL | pg/ml | 3.2E1 | 2.6E1 | 3.9E1 | 2.8E1 | 2.8E1 | 1.1E1 | 5.6E0 | 1.6E1 | 1.8E2 | 4.5E1 | 52 | 13 | 51 | 13 | 0.38 |
| rP | ng/ml | 9.5E1 | 1.5E2 | 1.6E2 | 2.3E2 | 2.2E2 | 2.1E2 | 1.0E-9 | 1.0E-9 | 1.2E3 | 5.0E2 | 54 | 16 | 54 | 16 | 0.60 |
| rQ | ng/ml | 1.0E-9 | 1.0E-9 | 3.6E0 | 8.4E0 | 1.6E1 | 1.8E1 | 1.0E-9 | 1.0E-9 | 8.9E1 | 5.6E1 | 54 | 16 | 54 | 16 | 0.58 |
| rO | ng/ml | 2.5E-2 | 3.8E-2 | 4.8E-2 | 4.5E-2 | 8.5E-2 | 3.7E-2 | 1.0E-9 | 1.0E-9 | 4.4E-1 | 9.6E-2 | 54 | 16 | 54 | 16 | 0.58 |
| rR | ng/ml | 3.9E0 | 1.3E1 | 2.3E1 | 1.9E1 | 6.8E1 | 1.9E1 | 1.0E-9 | 1.0E-9 | 4.5E2 | 6.6E1 | 54 | 16 | 54 | 16 | 0.63 |
| rN | ng/ml | 6.6E-1 | 5.2E-1 | 7.4E-1 | 6.5E-1 | 4.4E-1 | 4.8E-1 | 5.1E-2 | 1.5E-1 | 2.1E0 | 2.2E0 | 54 | 16 | 54 | 16 | 0.41 |
| qO | pg/ml | 9.8E3 | 1.5E4 | 1.3E4 | 1.7E4 | 9.6E3 | 1.4E4 | 2.2E3 | 1.1E3 | 4.6E4 | 4.5E4 | 55 | 15 | 54 | 15 | 0.56 |
| qP | pg/ml | 3.6E2 | 5.3E2 | 4.4E2 | 5.5E2 | 3.0E2 | 3.9E2 | 1.0E-9 | 1.1E2 | 1.5E3 | 1.5E3 | 55 | 15 | 54 | 15 | 0.58 |
| qQ | pg/ml | 1.5E1 | 1.5E1 | 1.7E1 | 1.6E1 | 3.8E1 | 2.7E1 | 1.0E-9 | 1.0E-9 | 2.8E2 | 9.9E1 | 55 | 15 | 54 | 15 | 0.46 |
| nW | pg/ml | 1.1E5 | 1.1E5 | 1.1E5 | 1.1E5 | 2.4E4 | 2.4E4 | 5.8E4 | 5.9E4 | 1.8E5 | 1.5E5 | 105 | 45 | 97 | 45 | 0.45 |
| nY | pg/ml | 1.9E3 | 2.1E3 | 2.2E3 | 2.5E3 | 1.4E3 | 2.0E3 | 6.5E2 | 9.8E2 | 9.9E3 | 1.3E4 | 105 | 45 | 97 | 45 | 0.53 |
| oO | pg/ml | 7.5E4 | 8.8E4 | 1.0E5 | 1.3E5 | 1.1E5 | 8.8E4 | 1.5E4 | 3.5E4 | 6.2E5 | 3.3E5 | 51 | 28 | 49 | 28 | 0.62 |
| oP | pg/ml | 1.1E5 | 1.6E5 | 1.3E5 | 1.8E5 | 7.7E4 | 1.2E5 | 2.4E4 | 4.8E4 | 3.6E5 | 4.6E5 | 51 | 28 | 49 | 28 | 0.66 |
| oQ | pg/ml | 2.5E3 | 3.9E3 | 2.9E3 | 4.4E3 | 1.7E3 | 2.9E3 | 9.3E2 | 1.7E3 | 1.0E4 | 1.4E4 | 51 | 28 | 49 | 28 | 0.67 |
| oE | pg/ml | 1.8E2 | 1.3E2 | 4.0E2 | 4.0E2 | 6.0E2 | 5.2E2 | 1.0E-9 | 1.0E-9 | 4.7E3 | 1.9E3 | 105 | 45 | 97 | 45 | 0.48 |
| oF | pg/ml | 7.4E3 | 8.0E3 | 1.7E4 | 2.6E4 | 3.0E4 | 4.9E4 | 6.4E1 | 6.9E2 | 1.7E5 | 2.3E5 | 105 | 45 | 97 | 45 | 0.53 |
| oH | pg/ml | 4.4E1 | 4.9E1 | 9.5E1 | 7.7E1 | 1.5E2 | 7.2E1 | 4.2E0 | 6.2E0 | 8.6E2 | 3.2E2 | 105 | 45 | 97 | 45 | 0.54 |
| oK | pg/ml | 6.4E2 | 6.6E2 | 2.0E3 | 1.6E3 | 3.1E3 | 1.9E3 | 5.2E1 | 1.4E2 | 1.8E4 | 7.2E3 | 105 | 45 | 97 | 45 | 0.53 |
| oN | pg/ml | 4.9E2 | 4.6E2 | 7.8E2 | 5.3E2 | 1.8E3 | 2.2E2 | 1.5E2 | 2.0E2 | 1.8E4 | 1.0E3 | 105 | 45 | 97 | 45 | 0.51 |
| oW | pg/ml | 2.1E2 | 2.5E2 | 4.8E2 | 2.6E2 | 1.1E3 | 1.5E2 | 7.7E1 | 8.5E1 | 6.0E3 | 5.4E2 | 27 | 8 | 26 | 8 | 0.51 |
| oT | pg/ml | 3.5E2 | 3.8E2 | 3.6E2 | 3.9E2 | 1.8E2 | 1.5E2 | 9.9E1 | 1.9E2 | 7.8E2 | 7.1E2 | 27 | 8 | 26 | 8 | 0.55 |
| oV | pg/ml | 1.4E2 | 1.0E2 | 2.1E2 | 1.3E2 | 2.3E2 | 7.0E1 | 1.0E-9 | 5.8E1 | 9.9E2 | 2.2E2 | 27 | 8 | 26 | 8 | 0.46 |
| oD | pg/ml | 1.7E4 | 1.8E4 | 1.9E4 | 1.9E4 | 8.2E3 | 6.7E3 | 9.3E3 | 1.2E4 | 4.6E4 | 3.3E4 | 27 | 8 | 26 | 8 | 0.54 |
| uL | ng/ml | 3.6E1 | 4.4E1 | 4.4E1 | 4.4E1 | 3.0E1 | 2.1E1 | 1.0E-9 | 1.5E1 | 1.6E2 | 8.6E1 | 52 | 15 | 51 | 15 | 0.54 |
| uO | ng/ml | 3.0E-1 | 1.3E0 | 7.8E-1 | 1.3E0 | 1.4E0 | 1.5E0 | 1.0E-9 | 1.0E-9 | 9.3E0 | 5.4E0 | 52 | 15 | 51 | 15 | 0.67 |
| uM | ng/ml | 6.4E-1 | 8.2E-1 | 1.1E0 | 8.6E-1 | 2.2E0 | 5.3E-1 | 1.0E-9 | 1.0E-9 | 1.3E1 | 2.2E0 | 52 | 15 | 51 | 15 | 0.57 |
| uI | ng/ml | 7.7E-2 | 9.6E-2 | 1.4E-1 | 1.4E-1 | 1.9E-1 | 1.6E-1 | 1.6E-2 | 3.7E-2 | 1.1E0 | 6.6E-1 | 52 | 15 | 51 | 15 | 0.52 |

EP 3 070 474 A2

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| uN | ng/ml | 1.5E1 | 1.2E1 | 1.7E1 | 1.4E1 | 6.7E0 | 6.4E0 | 7.7E0 | 6.3E0 | 4.1E1 | 3.0E1 | 52 | 15 | 51 | 15 | 0.38 |
| uG | ng/ml | 2.1E1 | 1.8E1 | 2.5E1 | 2.0E1 | 1.4E1 | 9.9E0 | 7.6E0 | 6.9E0 | 6.9E1 | 4.0E1 | 52 | 15 | 51 | 15 | 0.40 |
| uR | ng/ml | 2.3E0 | 3.7E0 | 3.9E0 | 3.6E0 | 8.6E0 | 2.0E0 | 9.9E-1 | 8.1E-1 | 6.4E1 | 8.0E0 | 54 | 14 | 53 | 14 | 0.64 |
| uP | ng/ml | 2.2E0 | 2.1E0 | 2.5E0 | 2.4E0 | 1.3E0 | 1.2E0 | 1.1E0 | 1.5E0 | 9.1E0 | 6.2E0 | 54 | 14 | 53 | 14 | 0.51 |
| uV | ng/ml | 2.3E-4 | 7.6E-3 | 1.6E-2 | 1.8E-2 | 4.0E-2 | 2.6E-2 | 1.0E-9 | 1.0E-9 | 2.0E-1 | 7.6E-2 | 54 | 14 | 53 | 14 | 0.56 |
| uT | ng/ml | 5.8E1 | 5.8E1 | 8.3E1 | 7.0E1 | 9.2E1 | 5.6E1 | 1.2E1 | 8.7E0 | 5.8E2 | 2.0E2 | 54 | 14 | 53 | 14 | 0.47 |
| uU | ng/ml | 1.7E0 | 2.0E0 | 2.0E0 | 1.9E0 | 1.2E0 | 9.1E-1 | 5.2E-1 | 6.9E-1 | 5.6E0 | 3.6E0 | 54 | 14 | 53 | 14 | 0.51 |
| uW | ng/ml | 7.2E0 | 7.3E0 | 7.6E0 | 7.5E0 | 2.9E0 | 2.8E0 | 4.0E0 | 3.5E0 | 2.2E1 | 1.5E1 | 52 | 15 | 51 | 15 | 0.49 |
| vB | ng/ml | 2.6E0 | 2.9E0 | 2.7E0 | 3.1E0 | 1.2E0 | 1.5E0 | 6.9E-1 | 9.9E-1 | 5.6E0 | 6.1E0 | 52 | 15 | 51 | 15 | 0.58 |
| vC | ng/ml | 1.0E-9 | 1.0E-9 | 3.0E-3 | 3.5E-3 | 2.0E-2 | 1.3E-2 | 1.0E-9 | 1.0E-9 | 1.4E-1 | 5.1E-2 | 52 | 15 | 51 | 15 | 0.55 |
| uY | ng/ml | 7.4E-1 | 9.0E-1 | 1.2E0 | 1.1E0 | 1.1E0 | 8.2E-1 | 8.7E-2 | 1.8E-1 | 4.9E0 | 2.6E0 | 52 | 15 | 51 | 15 | 0.52 |
| uZ | ng/ml | 5.8E-1 | 5.7E-1 | 8.2E-1 | 7.4E-1 | 1.1E0 | 3.9E-1 | 4.7E-2 | 1.2E-1 | 7.2E0 | 1.4E0 | 52 | 15 | 51 | 15 | 0.56 |
| uX | ng/ml | 1.1E1 | 1.3E1 | 1.3E1 | 1.3E1 | 7.2E0 | 7.0E0 | 4.0E0 | 3.7E0 | 3.3E1 | 2.7E1 | 52 | 15 | 51 | 15 | 0.54 |
| vA | ng/ml | 7.4E-2 | 6.8E-2 | 8.6E-2 | 7.7E-2 | 5.8E-2 | 4.2E-2 | 2.4E-2 | 1.7E-2 | 3.0E-1 | 1.6E-1 | 52 | 15 | 51 | 15 | 0.46 |
| vH | ng/ml | 1.2E-1 | 1.1E-1 | 1.6E-1 | 1.1E-1 | 1.4E-1 | 7.0E-2 | 1.5E-2 | 9.9E-3 | 8.0E-1 | 2.4E-1 | 54 | 15 | 53 | 15 | 0.44 |
| vI | ng/ml | 1.4E0 | 2.1E0 | 1.7E0 | 2.6E0 | 1.1E0 | 2.7E0 | 6.2E-3 | 4.2E-3 | 4.5E0 | 1.0E1 | 54 | 15 | 53 | 15 | 0.57 |
| vP | ng/ml | 4.3E2 | 7.3E2 | 5.1E2 | 6.7E2 | 3.9E2 | 5.0E2 | 7.0E1 | 4.0E1 | 2.0E3 | 1.6E3 | 54 | 14 | 53 | 14 | 0.60 |
| vT | ng/ml | 7.7E1 | 8.1E1 | 1.1E2 | 7.5E1 | 1.2E2 | 2.4E1 | 3.7E1 | 2.4E1 | 6.9E2 | 1.0E2 | 54 | 14 | 53 | 14 | 0.44 |
| vU | ng/ml | 1.0E-9 | 1.0E-9 | 2.3E1 | 7.2E0 | 3.5E1 | 1.6E1 | 1.0E-9 | 1.0E-9 | 1.3E2 | 4.7E1 | 54 | 14 | 53 | 14 | 0.38 |
| vQ | ng/ml | 3.5E2 | 3.7E2 | 3.6E2 | 3.9E2 | 1.5E2 | 8.4E1 | 6.7E1 | 3.0E2 | 8.1E2 | 5.5E2 | 54 | 14 | 53 | 14 | 0.59 |
| vO | ng/ml | 1.7E3 | 1.9E3 | 1.8E3 | 1.9E3 | 4.5E2 | 3.9E2 | 1.0E3 | 1.2E3 | 3.0E3 | 2.5E3 | 54 | 14 | 53 | 14 | 0.61 |
| vS | ng/ml | 1.3E3 | 1.1E3 | 1.3E3 | 9.8E2 | 3.6E2 | 4.7E2 | 1.0E-9 | 1.0E-9 | 1.8E3 | 1.5E3 | 54 | 14 | 53 | 14 | 0.28 |
| vV | ng/ml | 9.7E2 | 1.2E3 | 1.4E3 | 2.1E3 | 1.6E3 | 2.9E3 | 1.1E2 | 2.1E1 | 1.1E4 | 1.1E4 | 54 | 14 | 53 | 14 | 0.55 |
| vW | ng/ml | 1.4E2 | 1.2E2 | 1.8E2 | 1.9E2 | 1.4E2 | 1.2E2 | 4.3E1 | 7.1E1 | 6.7E2 | 4.0E2 | 54 | 14 | 53 | 14 | 0.55 |
| pF | pg/ml | 5.1E-1 | 6.1E-1 | 6.9E-1 | 9.3E-1 | 9.9E-1 | 1.5E0 | 1.0E-9 | 1.0E-9 | 9.4E0 | 1.0E1 | 105 | 45 | 97 | 45 | 0.57 |
| pH | ng/ml | 8.9E0 | 1.1E1 | 9.4E0 | 1.4E1 | 4.5E0 | 1.2E1 | 3.4E0 | 4.7E0 | 1.8E1 | 4.2E1 | 27 | 8 | 26 | 8 | 0.60 |
| pI | ng/ml | 7.0E1 | 8.4E1 | 7.0E1 | 8.5E1 | 3.2E1 | 2.5E1 | 2.6E1 | 5.6E1 | 1.5E2 | 1.2E2 | 27 | 8 | 26 | 8 | 0.64 |
| pK | ng/ml | 4.6E-1 | 5.1E-1 | 5.2E-1 | 6.3E-1 | 2.9E-1 | 3.7E-1 | 2.3E-1 | 2.6E-1 | 1.6E0 | 1.4E0 | 27 | 8 | 26 | 8 | 0.61 |

167

Figure 13.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.0E1 | 6.1E1 | 8.1E1 | 8.6E1 | 5.8E1 | 7.3E1 | 1.0E0 | 1.0E1 | 4.8E2 | 2.6E2 | 1595 | 20 | 266 | 20 | 0.49 |
| Ad | ug/mL | 3.8E-2 | 2.3E-2 | 9.4E-2 | 4.8E-2 | 4.1E-1 | 4.3E-2 | 2.7E-4 | 1.9E-3 | 8.5E0 | 1.3E-1 | 447 | 16 | 170 | 16 | 0.45 |
| Af | ng/mL | 1.2E0 | 4.7E-1 | 1.8E1 | 4.3E0 | 6.7E1 | 7.4E0 | 1.7E-3 | 1.7E-3 | 5.3E2 | 2.2E1 | 447 | 16 | 170 | 16 | 0.40 |
| Aj | ug/mL | 1.4E0 | 4.6E-1 | 2.6E0 | 2.5E0 | 2.4E0 | 2.8E0 | 1.5E-3 | 9.3E-3 | 6.1E0 | 6.1E0 | 447 | 16 | 170 | 16 | 0.45 |
| Al | mg/mL | 8.7E-5 | 1.1E-4 | 2.5E-4 | 1.0E-4 | 4.1E-4 | 4.6E-5 | 2.3E-6 | 9.5E-6 | 1.9E-3 | 1.9E-4 | 447 | 16 | 170 | 16 | 0.51 |
| An | U/mL | 4.8E1 | 1.6E2 | 1.8E2 | 2.1E2 | 5.5E2 | 2.0E2 | 9.8E-4 | 1.3E1 | 7.8E3 | 6.1E2 | 447 | 16 | 170 | 16 | 0.66 |
| Ao | pg/mL | 9.2E1 | 1.1E2 | 5.1E2 | 2.1E2 | 3.5E3 | 2.9E2 | 1.5E0 | 2.5E1 | 3.9E4 | 1.2E3 | 447 | 16 | 170 | 16 | 0.56 |
| Ap | ng/mL | 3.3E1 | 3.8E1 | 4.7E1 | 4.0E1 | 5.0E1 | 2.8E1 | 8.4E-5 | 3.2E0 | 3.3E2 | 9.5E1 | 447 | 16 | 170 | 16 | 0.51 |
| Ar | ng/mL | 9.5E-1 | 1.5E0 | 1.2E1 | 3.8E0 | 1.9E2 | 5.6E0 | 3.4E-3 | 3.4E-3 | 4.1E3 | 2.1E1 | 447 | 16 | 170 | 16 | 0.56 |
| As | ng/mL | 8.7E-3 | 4.5E-3 | 1.6E-2 | 8.0E-3 | 6.1E-2 | 8.4E-3 | 1.7E-3 | 1.7E-3 | 1.2E0 | 3.3E-2 | 447 | 16 | 170 | 16 | 0.42 |
| Aw | pg/mL | 1.6E1 | 1.8E1 | 1.6E1 | 1.9E1 | 6.4E0 | 4.8E0 | 2.9E-2 | 1.2E1 | 5.1E1 | 3.0E1 | 447 | 16 | 170 | 16 | 0.66 |
| Ax | ng/mL | 2.1E0 | 4.1E0 | 1.6E1 | 2.6E1 | 6.3E1 | 7.1E1 | 1.2E-2 | 1.7E-1 | 7.7E2 | 2.9E2 | 447 | 16 | 170 | 16 | 0.57 |
| Ba | ng/mL | 6.1E1 | 1.6E2 | 4.1E2 | 1.4E3 | 1.1E3 | 2.7E3 | 3.7E-1 | 2.7E-1 | 8.1E3 | 8.1E3 | 447 | 16 | 170 | 16 | 0.61 |
| Bb | ng/mL | 3.4E0 | 2.5E0 | 6.8E0 | 7.4E0 | 1.4E1 | 1.2E1 | 4.1E-3 | 4.1E-3 | 2.5E2 | 4.9E1 | 447 | 16 | 170 | 16 | 0.48 |
| Bc | ng/mL | 3.8E1 | 5.5E1 | 1.1E2 | 1.2E2 | 2.0E2 | 1.8E2 | 1.1E-1 | 4.3E-1 | 1.2E3 | 6.9E2 | 447 | 16 | 170 | 16 | 0.52 |
| Bg | ng/mL | 7.7E-2 | 5.4E-1 | 5.4E0 | 2.9E0 | 3.0E1 | 5.4E0 | 5.3E-4 | 5.3E-4 | 4.4E2 | 2.1E1 | 447 | 16 | 170 | 16 | 0.58 |
| Bn | ng/mL | 5.6E-2 | 1.1E-1 | 1.3E0 | 1.1E0 | 3.3E0 | 2.0E0 | 5.6E-2 | 5.6E-2 | 5.8E1 | 7.0E0 | 447 | 16 | 170 | 16 | 0.51 |
| Bo | ng/mL | 1.2E1 | 8.8E0 | 1.4E1 | 1.5E1 | 1.9E1 | 1.5E1 | 1.6E-2 | 1.6E-2 | 2.8E2 | 4.8E1 | 447 | 16 | 170 | 16 | 0.49 |
| Ch | uIU/mL | 9.7E-1 | 1.3E0 | 1.7E1 | 1.7E1 | 1.0E2 | 3.3E1 | 3.4E-3 | 3.4E-3 | 1.8E3 | 9.8E1 | 447 | 16 | 170 | 16 | 0.50 |
| Co | pg/mL | 3.8E1 | 3.4E1 | 1.8E2 | 1.8E2 | 9.6E2 | 5.1E2 | 1.5E-1 | 1.5E-1 | 1.7E4 | 2.1E3 | 447 | 16 | 170 | 16 | 0.51 |
| Cp | ng/mL | 2.1E1 | 3.0E1 | 3.0E1 | 3.6E1 | 6.7E1 | 1.7E1 | 6.0E-1 | 1.4E1 | 1.3E3 | 7.7E1 | 447 | 16 | 170 | 16 | 0.73 |
| Cq | ng/mL | 2.8E-2 | 3.1E-2 | 2.5E-1 | 8.1E-2 | 2.5E0 | 1.3E-1 | 8.0E-4 | 8.0E-4 | 4.9E1 | 5.2E-1 | 447 | 16 | 170 | 16 | 0.56 |
| Cs | ng/mL | 5.9E1 | 7.7E1 | 3.2E2 | 6.6E2 | 1.2E3 | 1.3E3 | 2.7E-2 | 3.6E0 | 1.8E4 | 5.1E3 | 447 | 16 | 170 | 16 | 0.54 |
| Ct | ng/mL | 6.1E-1 | 1.9E-1 | 3.2E1 | 6.7E1 | 9.9E1 | 1.6E2 | 1.1E-4 | 1.1E-4 | 6.2E2 | 4.7E2 | 447 | 16 | 170 | 16 | 0.48 |
| Cu | ng/mL | 2.4E-1 | 3.0E-1 | 5.5E-1 | 4.3E-1 | 3.2E0 | 5.5E-1 | 9.6E-3 | 9.0E-5 | 6.6E1 | 2.3E0 | 447 | 16 | 170 | 16 | 0.52 |
| Cv | ng/mL | 5.8E0 | 3.5E0 | 2.8E1 | 8.3E0 | 6.9E1 | 1.3E1 | 1.4E-4 | 1.4E-4 | 5.3E2 | 4.3E1 | 447 | 16 | 170 | 16 | 0.39 |
| Cw | mIU/mL | 3.0E-2 | 2.8E-2 | 5.4E-2 | 3.3E-2 | 3.2E-1 | 2.4E-2 | 1.5E-4 | 4.8E-3 | 6.8E0 | 9.2E-2 | 447 | 16 | 170 | 16 | 0.46 |
| Cx | ng/mL | 4.6E-1 | 2.4E-2 | 6.0E1 | 4.7E1 | 1.1E2 | 1.0E2 | 9.3E-5 | 9.3E-5 | 3.9E2 | 2.8E2 | 447 | 16 | 170 | 16 | 0.42 |
| Db | ug/mL | 7.6E0 | 6.7E0 | 9.6E0 | 7.8E0 | 1.1E1 | 6.5E0 | 4.5E-1 | 4.8E-1 | 1.4E2 | 2.3E1 | 447 | 16 | 170 | 16 | 0.45 |
| Dc | nmol/L | 1.9E-2 | 1.7E-2 | 8.6E-2 | 5.7E-2 | 6.7E-1 | 1.0E-1 | 5.2E-6 | 6.0E-4 | 1.4E1 | 4.0E-1 | 447 | 16 | 170 | 16 | 0.53 |
| Dd | ug/mL | 7.8E-2 | 6.2E-2 | 1.9E-1 | 8.0E-2 | 3.1E-1 | 7.7E-2 | 1.9E-4 | 8.3E-5 | 3.6E0 | 2.5E-1 | 447 | 16 | 170 | 16 | 0.41 |
| De | ng/mL | 3.4E-3 | 3.4E-3 | 7.6E-2 | 1.0E-1 | 1.5E-1 | 1.5E-1 | 3.4E-3 | 3.4E-3 | 9.2E-1 | 4.7E-1 | 447 | 16 | 170 | 16 | 0.55 |
| Dg | ng/mL | 3.4E1 | 3.6E1 | 4.6E1 | 3.3E1 | 4.1E1 | 2.6E1 | 1.0E-1 | 9.3E-1 | 1.9E2 | 7.5E1 | 447 | 16 | 170 | 16 | 0.42 |
| Di | pg/mL | 1.9E0 | 1.7E0 | 2.2E0 | 2.8E0 | 2.1E0 | 3.7E0 | 1.8E-1 | 1.8E-1 | 1.3E1 | 1.6E1 | 447 | 16 | 170 | 16 | 0.51 |
| Dk | uIU/mL | 1.6E-2 | 3.2E-2 | 8.7E-2 | 4.9E-2 | 5.1E-1 | 4.5E-2 | 1.1E-4 | 1.1E-4 | 8.9E0 | 1.4E-1 | 447 | 16 | 170 | 16 | 0.61 |
| Dl | ng/mL | 2.4E2 | 1.8E2 | 3.2E2 | 2.5E2 | 2.9E2 | 2.5E2 | 2.5E0 | 4.0E0 | 1.6E3 | 9.0E2 | 447 | 16 | 170 | 16 | 0.43 |
| Dp | ng/ml | 2.5E0 | 7.9E-1 | 6.4E0 | 3.1E0 | 1.5E1 | 5.8E0 | 3.7E-3 | 5.3E-3 | 2.0E2 | 1.9E1 | 262 | 14 | 160 | 14 | 0.31 |
| Dr | pg/ml | 2.9E1 | 7.8E0 | 1.2E2 | 3.6E1 | 8.3E2 | 4.7E1 | 7.5E-1 | 7.5E-1 | 1.0E4 | 1.2E2 | 158 | 9 | 91 | 9 | 0.40 |
| Ef | ng/ml | 1.3E-1 | 4.4E-1 | 8.6E-1 | 1.7E0 | 1.9E0 | 2.9E0 | 5.7E-4 | 5.7E-4 | 1.0E1 | 1.1E1 | 325 | 16 | 167 | 16 | 0.57 |
| Wm | % | 7.2E-1 | 1.3E0 | 3.2E1 | 1.2E2 | 1.8E2 | 3.4E2 | 5.4E-2 | 8.5E-2 | 2.4E3 | 1.3E3 | 353 | 19 | 182 | 19 | 0.56 |
| Ed | pg/ml | 5.2E-1 | 5.2E-1 | 5.9E1 | 4.1E1 | 4.5E2 | 6.6E1 | 5.2E-1 | 5.2E-1 | 7.3E3 | 1.6E2 | 262 | 14 | 159 | 14 | 0.49 |
| Tj | pg/mL | 3.7E-1 | 3.7E-1 | 5.6E1 | 4.5E0 | 3.0E2 | 1.1E1 | 3.6E-1 | 3.7E-1 | 3.5E3 | 4.2E1 | 323 | 15 | 169 | 15 | 0.40 |
| Po | pg/ml | 5.4E-1 | 2.6E0 | 8.9E0 | 1.8E1 | 2.6E1 | 4.2E1 | 8.0E-3 | 2.6E-2 | 2.7E2 | 2.2E2 | 755 | 29 | 295 | 29 | 0.61 |
| Ti | ug/mL | 3.6E0 | 1.4E0 | 5.0E0 | 2.4E0 | 4.2E0 | 2.5E0 | 1.2E-1 | 8.7E-3 | 1.8E1 | 6.7E0 | 152 | 10 | 114 | 10 | 0.29 |
| Em | ng/ml | 2.9E-3 | 2.9E-3 | 7.8E-2 | 2.1E-2 | 1.9E-1 | 4.7E-2 | 1.9E-16 | 2.8E-16 | 1.9E0 | 1.3E-1 | 204 | 8 | 91 | 8 | 0.30 |
| Et | ng/ml | 1.4E3 | 1.5E3 | 1.7E3 | 1.8E3 | 1.2E3 | 1.4E3 | 7.5E1 | 1.5E2 | 5.0E3 | 5.0E3 | 754 | 29 | 295 | 29 | 0.50 |
| Th | ug/mL | 1.1E0 | 6.8E-1 | 1.6E0 | 1.4E0 | 1.6E0 | 1.3E0 | 2.6E-3 | 6.5E-2 | 1.2E1 | 3.5E0 | 152 | 10 | 114 | 10 | 0.44 |
| Fa | ng/ml | 4.0E1 | 4.9E1 | 1.3E2 | 5.2E1 | 5.6E2 | 3.5E1 | 3.4E-2 | 2.0E0 | 8.0E3 | 1.0E2 | 261 | 14 | 158 | 14 | 0.50 |
| Ez | ng/ml | 3.8E0 | 6.5E0 | 1.5E1 | 6.7E1 | 3.2E1 | 1.9E2 | 1.3E-2 | 1.3E-2 | 3.0E2 | 7.1E2 | 262 | 14 | 160 | 14 | 0.57 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fb | ng/ml | 2.5E1 | 2.8E1 | 2.2E1 | 2.2E1 | 1.1E1 | 1.4E1 | 5.9E-1 | 1.0E0 | 5.7E1 | 3.9E1 | 262 | 14 | 158 | 14 | 0.53 |
| Ex | ng/ml | 7.8E-2 | 1.2E-1 | 2.2E-1 | 4.2E-1 | 6.9E-1 | 8.8E-1 | 3.5E-5 | 1.5E-4 | 8.9E0 | 3.1E0 | 245 | 12 | 117 | 12 | 0.53 |
| Fn | ng/ml | 2.1E-1 | 2.1E-1 | 5.9E0 | 3.2E-1 | 2.7E1 | 3.9E-1 | 1.1E-14 | 2.1E-1 | 4.2E2 | 1.7E0 | 262 | 14 | 160 | 14 | 0.31 |
| Fp | ng/ml | 1.3E1 | 1.4E1 | 2.5E1 | 3.4E1 | 2.8E1 | 3.4E1 | 6.0E-3 | 2.8E-1 | 1.4E2 | 1.0E2 | 787 | 30 | 296 | 30 | 0.57 |
| Fr | ng/ml | 3.5E4 | 1.1E5 | 1.1E5 | 2.7E5 | 1.7E5 | 3.0E5 | 1.9E2 | 7.8E2 | 9.0E5 | 8.9E5 | 890 | 31 | 300 | 31 | 0.63 |
| Fw | pg/ml | 1.1E0 | 5.3E0 | 6.2E1 | 1.6E1 | 4.8E2 | 2.9E1 | 1.1E-14 | 1.2E-1 | 6.9E3 | 1.1E2 | 325 | 16 | 168 | 16 | 0.61 |
| Fy | ng/ml | 3.8E1 | 3.1E1 | 6.0E1 | 4.7E1 | 7.3E1 | 4.6E1 | 1.2E-1 | 3.8E0 | 6.5E2 | 1.5E2 | 260 | 13 | 159 | 13 | 0.47 |
| Gc | ng/ml | 1.1E2 | 4.0E1 | 1.7E2 | 5.3E1 | 1.8E2 | 6.1E1 | 6.4E0 | 9.7E0 | 1.2E3 | 2.2E2 | 169 | 10 | 94 | 10 | 0.19 |
| Gd | ng/ml | 3.1E1 | 1.5E1 | 3.3E1 | 1.9E1 | 1.7E1 | 1.2E1 | 5.0E0 | 8.0E0 | 8.1E1 | 4.5E1 | 189 | 8 | 83 | 8 | 0.24 |
| Gn | U/ml | 2.8E-1 | 3.8E-1 | 2.1E0 | 4.1E-1 | 9.6E0 | 4.7E-1 | 1.3E-3 | 5.6E-3 | 1.1E2 | 1.3E0 | 152 | 9 | 89 | 9 | 0.38 |
| Gl | pg/ml | 7.1E3 | 1.6E4 | 1.1E4 | 1.4E4 | 9.1E3 | 1.0E4 | 9.1E1 | 6.3E2 | 3.4E4 | 2.8E4 | 316 | 16 | 166 | 16 | 0.59 |
| Gp | U/ml | 1.5E0 | 9.3E-1 | 4.1E0 | 3.6E0 | 7.0E0 | 6.2E0 | 1.3E-3 | 1.5E-2 | 6.7E1 | 2.0E1 | 327 | 16 | 168 | 16 | 0.44 |
| Gz | ug/ml | 1.4E0 | 8.7E-1 | 8.9E0 | 3.8E0 | 3.7E1 | 4.7E0 | 2.9E-16 | 3.8E-3 | 4.8E2 | 1.1E1 | 181 | 9 | 106 | 9 | 0.42 |
| Ha | ng/ml | 2.3E0 | 4.3E0 | 9.5E0 | 7.4E0 | 2.1E1 | 9.3E0 | 6.4E-3 | 2.8E-1 | 1.3E2 | 3.2E1 | 260 | 14 | 159 | 14 | 0.60 |
| Nm | pg/ml | 1.5E4 | 8.9E3 | 3.4E4 | 3.4E4 | 8.7E4 | 8.2E4 | 1.0E-9 | 1.0E-9 | 1.6E6 | 4.4E5 | 758 | 29 | 297 | 29 | 0.43 |
| Nn | pg/ml | 1.5E2 | 5.2E2 | 1.7E3 | 5.1E3 | 7.6E3 | 1.4E4 | 1.0E-9 | 3.0E0 | 1.0E5 | 6.9E4 | 758 | 29 | 297 | 29 | 0.64 |
| No | pg/ml | 1.5E1 | 2.8E1 | 3.9E1 | 1.2E2 | 1.2E2 | 3.1E2 | 1.0E-9 | 2.4E-1 | 2.5E3 | 1.4E3 | 758 | 29 | 297 | 29 | 0.55 |
| Nq | pg/ml | 1.7E0 | 4.5E0 | 1.7E1 | 2.9E1 | 6.8E1 | 5.9E1 | 1.0E-9 | 1.0E-9 | 1.2E3 | 2.7E2 | 758 | 29 | 297 | 29 | 0.59 |
| Nr | pg/ml | 9.7E-1 | 3.5E-1 | 3.1E1 | 3.0E2 | 1.9E2 | 1.6E3 | 1.0E-9 | 1.0E-9 | 4.1E3 | 8.5E3 | 758 | 29 | 297 | 29 | 0.46 |
| Ns | pg/ml | 1.0E-9 | 1.0E-9 | 7.1E0 | 4.4E0 | 3.5E1 | 2.2E1 | 1.0E-9 | 1.0E-9 | 6.8E2 | 1.2E2 | 758 | 29 | 297 | 29 | 0.50 |
| Nt | pg/ml | 1.0E2 | 1.4E2 | 1.3E2 | 1.8E2 | 1.1E2 | 1.5E2 | 1.0E-9 | 1.5E1 | 1.7E3 | 6.8E2 | 758 | 29 | 297 | 29 | 0.58 |
| Nu | pg/ml | 2.0E1 | 1.2E1 | 5.3E1 | 1.0E2 | 8.9E1 | 1.5E2 | 1.0E-9 | 1.0E-9 | 8.9E2 | 5.8E2 | 758 | 29 | 297 | 29 | 0.55 |
| Lu | pg/ml | 1.0E4 | 1.0E4 | 1.8E4 | 9.9E3 | 6.5E4 | 1.1E4 | 3.5E2 | 1.3E3 | 1.3E6 | 6.1E4 | 761 | 29 | 297 | 29 | 0.43 |
| Lv | pg/ml | 1.0E-9 | 1.0E-9 | 1.1E1 | 2.8E1 | 2.1E1 | 4.6E1 | 1.0E-9 | 1.0E-9 | 2.4E2 | 1.9E2 | 761 | 29 | 297 | 29 | 0.59 |
| Lw | pg/ml | 1.0E-9 | 1.0E-9 | 4.3E-1 | 1.3E0 | 4.1E0 | 4.9E0 | 1.0E-9 | 1.0E-9 | 8.0E1 | 2.1E1 | 761 | 29 | 297 | 29 | 0.54 |
| Lx | pg/ml | 1.0E-9 | 6.5E1 | 1.8E2 | 7.4E2 | 9.3E2 | 2.0E3 | 1.0E-9 | 1.0E-9 | 2.2E4 | 1.0E4 | 761 | 29 | 297 | 29 | 0.61 |
| Ly | pg/ml | 1.0E-9 | 1.0E-9 | 1.0E1 | 1.0E1 | 2.0E1 | 1.7E1 | 1.0E-9 | 1.0E-9 | 1.2E2 | 5.8E1 | 761 | 29 | 297 | 29 | 0.52 |
| Lz | pg/ml | 1.0E-9 | 1.0E-9 | 4.0E0 | 7.6E0 | 3.2E1 | 3.9E1 | 1.0E-9 | 1.0E-9 | 6.0E2 | 2.1E2 | 761 | 29 | 297 | 29 | 0.50 |
| Ma | pg/ml | 2.9E2 | 2.0E2 | 1.3E3 | 9.9E2 | 3.6E3 | 2.0E3 | 1.0E-9 | 1.0E-9 | 6.5E4 | 9.5E3 | 761 | 29 | 297 | 29 | 0.45 |
| Mb | pg/ml | 2.5E1 | 2.5E1 | 3.1E1 | 3.2E1 | 1.5E1 | 1.5E1 | 4.1E0 | 1.8E1 | 2.1E2 | 6.9E1 | 761 | 29 | 297 | 29 | 0.53 |
| Mc | pg/ml | 1.0E-9 | 1.0E-9 | 4.3E-2 | 1.0E-9 | 6.1E-1 | 0.0E0 | 1.0E-9 | 1.0E-9 | 1.3E1 | 1.0E-9 | 761 | 29 | 297 | 29 | 0.49 |
| Md | pg/ml | 1.0E-9 | 1.0E-9 | 4.5E-1 | 2.8E-1 | 3.9E0 | 1.4E0 | 1.0E-9 | 1.0E-9 | 6.8E1 | 7.4E0 | 761 | 29 | 297 | 29 | 0.52 |
| Me | pg/ml | 3.3E1 | 2.6E1 | 3.2E1 | 2.6E1 | 2.0E1 | 1.8E1 | 1.0E-9 | 7.9E-1 | 3.2E2 | 7.9E1 | 761 | 29 | 297 | 29 | 0.36 |
| Mf | pg/ml | 1.0E-9 | 1.0E-9 | 5.0E-1 | 2.2E-2 | 2.8E0 | 1.0E-1 | 1.0E-9 | 1.0E-9 | 5.6E1 | 5.3E-1 | 761 | 29 | 297 | 29 | 0.46 |
| Mg | pg/ml | 1.6E0 | 6.7E-1 | 7.0E0 | 4.7E0 | 1.2E1 | 7.1E0 | 1.0E-9 | 1.0E-9 | 9.2E1 | 2.7E1 | 761 | 29 | 297 | 29 | 0.47 |
| Mh | pg/ml | 1.0E-9 | 1.0E-9 | 1.5E0 | 1.5E0 | 1.0E1 | 7.0E0 | 1.0E-9 | 1.0E-9 | 1.6E2 | 3.8E1 | 761 | 29 | 297 | 29 | 0.54 |
| Mi | pg/ml | 1.0E-9 | 1.0E-9 | 9.4E-1 | 1.0E1 | 1.3E1 | 3.2E1 | 1.0E-9 | 1.0E-9 | 3.2E2 | 1.6E2 | 761 | 29 | 297 | 29 | 0.58 |
| Mj | pg/ml | 1.0E-9 | 1.0E-9 | 4.7E0 | 2.0E1 | 2.7E1 | 1.0E2 | 1.0E-9 | 1.0E-9 | 4.6E2 | 5.4E2 | 761 | 29 | 297 | 29 | 0.51 |
| Mk | pg/ml | 2.8E-1 | 2.2E0 | 1.4E1 | 2.0E2 | 9.2E1 | 1.0E3 | 1.0E-9 | 1.0E-9 | 1.7E3 | 5.6E3 | 761 | 29 | 297 | 29 | 0.54 |
| Ml | pg/ml | 1.0E-9 | 1.0E-9 | 6.0E0 | 3.5E-1 | 8.1E1 | 1.3E0 | 1.0E-9 | 1.0E-9 | 2.1E3 | 6.6E0 | 761 | 29 | 297 | 29 | 0.45 |
| Mm | pg/ml | 6.1E2 | 4.0E2 | 1.1E3 | 1.3E3 | 1.4E3 | 2.1E3 | 1.0E-9 | 1.0E-9 | 1.2E4 | 7.7E3 | 761 | 29 | 297 | 29 | 0.45 |
| Mn | pg/ml | 5.4E0 | 4.9E0 | 1.0E1 | 1.0E1 | 2.0E1 | 1.3E1 | 1.0E-9 | 1.0E-9 | 3.5E2 | 6.6E1 | 761 | 29 | 297 | 29 | 0.50 |
| Mp | pg/ml | 1.0E-9 | 4.4E0 | 1.0E1 | 2.8E1 | 3.6E1 | 5.6E1 | 1.0E-9 | 1.0E-9 | 6.3E2 | 1.8E2 | 760 | 29 | 297 | 29 | 0.57 |
| Mq | pg/ml | 1.0E-9 | 1.0E-9 | 2.8E0 | 9.7E0 | 1.6E1 | 3.3E1 | 1.0E-9 | 1.0E-9 | 2.1E2 | 1.4E2 | 760 | 29 | 297 | 29 | 0.54 |
| Mr | pg/ml | 1.0E-9 | 1.0E-9 | 3.2E1 | 4.1E2 | 1.8E2 | 2.2E3 | 1.0E-9 | 1.0E-9 | 2.2E3 | 1.2E4 | 760 | 29 | 297 | 29 | 0.57 |
| Ms | pg/ml | 4.1E2 | 5.3E2 | 5.6E2 | 6.0E2 | 6.6E2 | 5.3E2 | 1.0E-9 | 1.0E-9 | 5.9E3 | 2.2E3 | 760 | 29 | 297 | 29 | 0.55 |
| Mt | pg/ml | 2.4E-1 | 1.3E0 | 7.3E0 | 1.5E1 | 4.5E1 | 4.7E1 | 1.0E-9 | 1.0E-9 | 8.7E2 | 2.5E2 | 760 | 29 | 297 | 29 | 0.61 |
| Mu | pg/ml | 1.0E-9 | 1.0E-9 | 9.0E-1 | 2.5E0 | 7.0E0 | 5.4E0 | 1.0E-9 | 1.0E-9 | 9.9E1 | 2.7E1 | 760 | 29 | 297 | 29 | 0.66 |
| Mv | pg/ml | 1.0E-9 | 3.2E0 | 5.8E1 | 1.1E2 | 3.1E2 | 2.2E2 | 1.0E-9 | 1.0E-9 | 4.5E3 | 9.3E2 | 760 | 29 | 297 | 29 | 0.66 |
| Mw | pg/ml | 3.8E1 | 1.2E2 | 4.0E2 | 1.3E3 | 2.8E3 | 3.5E3 | 1.0E-9 | 1.0E-9 | 6.2E4 | 1.7E4 | 760 | 29 | 297 | 29 | 0.63 |
| Mx | pg/ml | 1.0E-9 | 1.0E-9 | 2.2E-1 | 5.4E-1 | 9.1E-1 | 1.3E0 | 1.0E-9 | 1.0E-9 | 9.6E0 | 6.3E0 | 760 | 29 | 297 | 29 | 0.61 |
| My | pg/ml | 1.0E-9 | 1.8E1 | 3.9E2 | 3.8E2 | 2.7E3 | 9.6E2 | 1.0E-9 | 1.0E-9 | 3.9E4 | 4.1E3 | 760 | 29 | 297 | 29 | 0.62 |
| Mz | pg/ml | 1.1E1 | 2.3E1 | 2.6E1 | 5.4E1 | 7.9E1 | 7.5E1 | 1.0E-9 | 1.0E-9 | 1.3E3 | 3.0E2 | 760 | 29 | 297 | 29 | 0.61 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Na | pg/ml | 1.0E-9 | 1.0E-9 | 7.4E-1 | 9.9E-1 | 3.2E0 | 2.5E0 | 1.0E-9 | 1.0E-9 | 4.2E1 | 1.1E1 | 760 | 29 | 297 | 29 | 0.56 |
| Nb | pg/ml | 1.9E0 | 2.8E0 | 4.0E0 | 1.2E1 | 1.3E1 | 4.4E1 | 1.0E-9 | 1.0E-9 | 2.3E2 | 2.4E2 | 760 | 29 | 297 | 29 | 0.58 |
| Nc | pg/ml | 3.4E2 | 1.7E2 | 5.8E2 | 3.9E2 | 7.5E2 | 4.9E2 | 1.0E-9 | 1.0E-9 | 9.2E3 | 1.8E3 | 760 | 29 | 297 | 29 | 0.45 |
| Nd | pg/ml | 2.9E1 | 1.3E1 | 3.0E1 | 3.2E1 | 9.0E1 | 4.0E1 | 1.0E-9 | 1.0E-9 | 2.1E3 | 1.4E2 | 760 | 29 | 297 | 29 | 0.48 |
| Nc | pg/ml | 4.5E2 | 3.1E2 | 5.9E2 | 4.1E2 | 5.9E2 | 3.3E2 | 1.0E-9 | 1.0E-9 | 7.0E3 | 1.3E3 | 760 | 29 | 297 | 29 | 0.42 |
| Nf | pg/ml | 1.0E-9 | 1.0E-9 | 2.9E0 | 5.0E0 | 1.1E1 | 1.6E1 | 1.0E-9 | 1.0E-9 | 1.6E2 | 8.2E1 | 760 | 29 | 297 | 29 | 0.52 |
| Ng | pg/ml | 1.9E1 | 5.3E0 | 1.1E2 | 8.5E1 | 2.2E2 | 1.5E2 | 1.0E-9 | 1.0E-9 | 1.6E3 | 6.6E2 | 760 | 29 | 297 | 29 | 0.45 |
| Nh | pg/ml | 6.9E1 | 5.0E1 | 9.2E1 | 7.0E1 | 8.3E1 | 7.1E1 | 1.0E-9 | 2.2E0 | 5.6E2 | 3.4E2 | 760 | 29 | 297 | 29 | 0.41 |
| Ni | pg/ml | 1.0E-9 | 2.3E1 | 7.2E1 | 9.9E1 | 1.2E2 | 1.5E2 | 1.0E-9 | 1.0E-9 | 1.1E3 | 5.5E2 | 760 | 29 | 297 | 29 | 0.55 |
| Nj | pg/ml | 7.3E0 | 8.4E0 | 1.1E1 | 1.1E1 | 1.2E1 | 1.2E1 | 1.0E-9 | 1.0E-9 | 8.3E1 | 5.7E1 | 760 | 29 | 297 | 29 | 0.51 |
| Nk | pg/ml | 1.8E1 | 1.7E1 | 3.4E1 | 2.6E1 | 4.0E1 | 3.3E1 | 1.0E-9 | 1.0E-9 | 2.0E2 | 1.7E2 | 760 | 29 | 297 | 29 | 0.49 |
| Nl | pg/ml | 4.6E1 | 3.4E1 | 6.2E1 | 4.0E1 | 7.0E1 | 3.1E1 | 1.0E-9 | 1.0E-9 | 1.1E3 | 1.4E2 | 760 | 29 | 297 | 29 | 0.43 |
| Tz | pg/ml | 4.6E3 | 6.4E3 | 1.3E4 | 1.2E4 | 6.3E4 | 1.5E4 | 1.0E-9 | 6.5E2 | 1.0E6 | 5.2E4 | 264 | 14 | 159 | 14 | 0.56 |
| Ua | pg/ml | 3.7E3 | 5.6E3 | 2.0E4 | 3.9E4 | 1.3E5 | 5.8E4 | 1.0E-9 | 3.2E2 | 2.1E6 | 1.8E5 | 264 | 14 | 159 | 14 | 0.60 |
| Ub | pg/ml | 5.7E2 | 3.7E2 | 8.7E2 | 5.7E2 | 1.1E3 | 6.1E2 | 1.0E-9 | 3.9E1 | 9.8E3 | 2.4E3 | 264 | 14 | 159 | 14 | 0.41 |
| Ue | pg/ml | 3.1E1 | 2.1E1 | 4.0E1 | 1.9E1 | 4.0E1 | 6.6E0 | 9.8E-2 | 8.4E0 | 4.4E2 | 2.9E1 | 264 | 14 | 159 | 14 | 0.29 |
| Uc | pg/ml | 9.2E2 | 8.2E2 | 2.0E3 | 1.0E3 | 4.4E3 | 9.6E2 | 1.0E-9 | 1.4E1 | 5.7E4 | 3.1E3 | 264 | 14 | 159 | 14 | 0.43 |
| Ud | pg/ml | 1.0E-9 | 1.0E-9 | 1.8E0 | 3.4E-1 | 2.4E1 | 1.3E0 | 1.0E-9 | 1.0E-9 | 3.9E2 | 4.8E0 | 264 | 14 | 159 | 14 | 0.53 |
| Hq | pg/ml | 1.0E0 | 1.0E0 | 1.2E2 | 1.4E1 | 1.8E3 | 5.5E1 | 1.0E-9 | 1.0E-9 | 3.4E4 | 3.0E2 | 756 | 29 | 296 | 29 | 0.50 |
| Hr | pg/ml | 1.1E2 | 9.4E1 | 8.0E2 | 8.5E2 | 1.7E3 | 2.1E3 | 1.0E-9 | 2.2E1 | 1.7E4 | 1.1E4 | 756 | 29 | 296 | 29 | 0.50 |
| Hu | pg/ml | 5.1E1 | 8.8E1 | 2.6E3 | 1.3E3 | 2.6E4 | 2.5E3 | 1.0E-9 | 1.0E-9 | 6.3E5 | 8.8E3 | 756 | 29 | 296 | 29 | 0.60 |
| Hv | pg/ml | 1.3E0 | 2.3E0 | 4.2E0 | 7.6E0 | 3.4E1 | 1.8E1 | 1.0E-9 | 1.0E-9 | 8.9E2 | 8.4E1 | 756 | 29 | 296 | 29 | 0.64 |
| Hw | pg/ml | 6.6E0 | 4.0E0 | 3.1E1 | 1.3E2 | 3.5E2 | 6.4E2 | 1.0E-9 | 5.3E-1 | 9.4E3 | 3.4E3 | 756 | 29 | 296 | 29 | 0.44 |
| Hx | pg/ml | 8.3E0 | 1.4E1 | 4.0E1 | 9.6E1 | 3.5E2 | 3.6E2 | 1.0E-9 | 1.0E-9 | 9.3E3 | 2.0E3 | 756 | 29 | 296 | 29 | 0.64 |
| Ib | ng/ml | 4.9E-2 | 7.4E-2 | 1.2E0 | 6.1E0 | 5.3E0 | 1.5E1 | 1.0E-9 | 1.0E-9 | 5.6E1 | 5.2E1 | 259 | 12 | 159 | 12 | 0.55 |
| Ic | U/ml | 2.2E2 | 9.3E1 | 1.1E3 | 1.5E2 | 7.3E3 | 1.4E2 | 1.5E0 | 9.2E0 | 9.3E4 | 3.9E2 | 259 | 12 | 159 | 12 | 0.38 |
| Id | U/ml | 6.8E-1 | 8.3E-1 | 3.0E0 | 9.4E-1 | 2.7E1 | 9.0E-1 | 1.0E-9 | 5.1E-2 | 4.3E2 | 3.4E0 | 259 | 12 | 159 | 12 | 0.46 |
| Tt | pg/ml | 1.6E2 | 1.5E2 | 1.7E2 | 1.6E2 | 5.4E1 | 4.2E1 | 4.3E1 | 1.1E2 | 4.4E2 | 2.3E2 | 246 | 13 | 153 | 13 | 0.43 |
| To | pg/ml | 1.6E0 | 1.8E0 | 1.9E0 | 3.0E0 | 2.3E0 | 2.9E0 | 1.0E-9 | 1.0E-9 | 2.3E1 | 1.0E1 | 254 | 13 | 156 | 13 | 0.61 |
| Tr | pg/ml | 3.3E0 | 1.7E0 | 6.6E0 | 2.6E0 | 2.1E1 | 2.5E0 | 1.0E-9 | 1.0E-9 | 3.1E2 | 8.4E0 | 251 | 13 | 155 | 13 | 0.35 |
| Tn | pg/ml | 2.8E1 | 2.1E1 | 8.2E1 | 5.6E1 | 2.5E2 | 6.8E1 | 2.4E0 | 6.8E0 | 2.3E3 | 2.2E2 | 254 | 13 | 156 | 13 | 0.46 |
| Tv | ng/ml | 1.2E1 | 9.8E0 | 5.2E1 | 1.1E1 | 4.5E2 | 1.2E1 | 1.0E-9 | 1.0E-9 | 7.1E3 | 4.0E1 | 254 | 13 | 156 | 13 | 0.39 |
| Ih | ng/ml | 6.8E1 | 6.4E1 | 2.4E2 | 1.9E2 | 4.3E2 | 3.1E2 | 1.0E-9 | 1.0E-9 | 3.6E3 | 1.2E3 | 760 | 29 | 296 | 29 | 0.51 |
| Ii | ng/ml | 9.1E1 | 5.7E1 | 2.4E2 | 4.6E2 | 6.3E2 | 1.7E3 | 1.0E-9 | 2.9E0 | 8.4E3 | 9.2E3 | 760 | 28 | 296 | 28 | 0.46 |
| Ij | ng/ml | 7.6E1 | 8.5E1 | 2.0E2 | 3.6E2 | 1.1E3 | 1.2E3 | 1.0E-9 | 4.7E0 | 2.4E4 | 6.4E3 | 750 | 27 | 294 | 27 | 0.54 |
| Ik | ng/ml | 1.1E1 | 2.1E2 | 9.1E2 | 4.1E2 | 8.9E3 | 5.5E2 | 5.9E-1 | 1.3E0 | 1.2E5 | 1.5E3 | 755 | 28 | 294 | 28 | 0.60 |
| Il | ng/ml | 3.2E2 | 2.2E2 | 1.3E3 | 8.4E2 | 2.8E3 | 2.3E3 | 1.0E-9 | 1.0E-9 | 1.3E4 | 1.2E4 | 744 | 28 | 295 | 28 | 0.41 |
| Im | ng/ml | 2.1E2 | 2.2E2 | 3.7E2 | 7.5E2 | 5.3E2 | 1.5E3 | 1.3E1 | 2.7E1 | 5.8E3 | 6.8E3 | 754 | 28 | 295 | 28 | 0.52 |
| In | ng/ml | 3.6E0 | 2.8E0 | 3.0E1 | 7.3E0 | 2.3E2 | 1.4E1 | 1.0E-9 | 1.0E-9 | 4.5E3 | 6.8E1 | 760 | 29 | 296 | 29 | 0.43 |
| Hb | ng/ml | 2.6E1 | 1.3E1 | 3.7E1 | 2.5E1 | 3.5E1 | 3.1E1 | 6.2E-1 | 3.3E0 | 2.1E2 | 1.2E2 | 264 | 14 | 159 | 14 | 0.37 |
| Hc | pg/ml | 6.3E2 | 8.7E2 | 3.4E3 | 4.9E3 | 1.3E4 | 7.0E3 | 1.0E-9 | 1.0E-9 | 1.0E5 | 1.9E4 | 264 | 14 | 159 | 14 | 0.60 |
| Hf | ng/ml | 1.5E2 | 1.7E2 | 3.5E2 | 3.0E2 | 4.9E2 | 4.2E2 | 1.0E-9 | 1.0E-9 | 2.5E3 | 1.5E3 | 264 | 14 | 159 | 14 | 0.49 |
| Io | ng/ml | 8.2E3 | 7.2E3 | 2.6E4 | 3.1E4 | 1.6E5 | 1.0E5 | 1.0E-9 | 6.2E1 | 4.0E6 | 5.5E5 | 752 | 28 | 296 | 28 | 0.47 |
| Ip | ng/ml | 8.8E0 | 1.5E1 | 1.9E1 | 2.6E1 | 2.4E1 | 3.4E1 | 1.0E-9 | 1.0E-9 | 2.6E2 | 1.4E2 | 752 | 28 | 296 | 28 | 0.51 |
| Iq | ug/ml | 9.6E-2 | 1.2E-1 | 1.9E1 | 2.7E1 | 5.0E1 | 1.4E2 | 1.0E-9 | 1.0E-9 | 1.4E4 | 7.5E2 | 752 | 28 | 296 | 28 | 0.48 |
| Ir | ug/ml | 3.4E-1 | 3.8E-1 | 3.7E0 | 5.3E0 | 2.7E1 | 2.2E1 | 1.0E-9 | 5.7E-2 | 5.1E2 | 1.1E2 | 751 | 28 | 296 | 28 | 0.55 |
| Is | ng/ml | 1.4E0 | 2.7E0 | 6.2E0 | 1.9E1 | 2.4E1 | 4.4E1 | 1.0E-9 | 2.9E-2 | 5.5E2 | 2.3E2 | 752 | 28 | 296 | 28 | 0.63 |
| It | ng/ml | 2.0E0 | 9.6E-1 | 2.4E1 | 2.6E1 | 1.4E2 | 1.1E2 | 1.0E-9 | 1.0E-9 | 2.8E3 | 5.9E2 | 752 | 28 | 296 | 28 | 0.41 |
| Iu | ng/ml | 2.2E2 | 9.5E1 | 1.5E3 | 1.1E3 | 4.3E3 | 4.5E3 | 1.0E-9 | 1.0E-9 | 2.9E4 | 2.4E4 | 752 | 28 | 296 | 28 | 0.38 |
| Iv | ng/ml | 1.2E1 | 1.8E1 | 6.5E1 | 2.6E2 | 6.0E2 | 1.2E3 | 1.0E-9 | 1.0E-9 | 1.6E4 | 6.4E3 | 751 | 28 | 296 | 28 | 0.59 |
| Iz | ng/ml | 1.4E2 | 5.7E2 | 6.3E2 | 5.6E2 | 3.9E3 | 5.0E2 | 9.2E-1 | 1.8E1 | 6.2E4 | 1.2E3 | 264 | 14 | 159 | 14 | 0.60 |
| Rc | pg/ml | 6.1E3 | 4.3E3 | 7.3E3 | 7.5E3 | 5.5E3 | 7.4E3 | 1.9E2 | 1.1E3 | 3.0E4 | 2.9E4 | 261 | 14 | 159 | 14 | 0.46 |
| Rb | pg/ml | 8.6E-1 | 1.6E0 | 2.8E0 | 2.0E0 | 5.5E0 | 2.0E0 | 1.0E-9 | 1.0E-9 | 5.6E1 | 6.0E0 | 261 | 14 | 159 | 14 | 0.53 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pz | ng/ml | 4.4E3 | 1.8E3 | 8.3E3 | 4.2E3 | 4.0E4 | 4.2E3 | 1.3E1 | 3.6E1 | 1.0E6 | 1.4E4 | 753 | 29 | 295 | 29 | 0.42 |
| Qa | ng/ml | 3.4E3 | 5.3E3 | 6.3E3 | 8.3E3 | 1.1E4 | 7.8E3 | 1.2E1 | 3.4E2 | 2.2E5 | 2.8E4 | 753 | 29 | 295 | 29 | 0.61 |
| Qb | ng/ml | 8.7E1 | 1.5E2 | 2.1E2 | 2.0E2 | 5.0E2 | 1.7E2 | 7.9E-1 | 1.1E1 | 8.3E3 | 6.0E2 | 753 | 29 | 295 | 29 | 0.63 |
| Qc | ng/ml | 2.1E2 | 3.6E2 | 4.4E2 | 4.6E2 | 7.6E2 | 5.9E2 | 1.0E-9 | 2.7E1 | 1.1E4 | 3.1E3 | 753 | 29 | 295 | 29 | 0.57 |
| Qd | ng/ml | 8.9E3 | 1.1E4 | 1.9E4 | 2.4E4 | 7.8E4 | 3.4E4 | 1.5E2 | 9.8E2 | 2.0E6 | 1.5E5 | 753 | 29 | 295 | 29 | 0.56 |
| Qe | ng/ml | 9.1E2 | 9.5E2 | 1.8E3 | 2.1E3 | 4.0E3 | 2.8E3 | 1.0E-9 | 6.8E1 | 9.7E4 | 1.4E4 | 753 | 29 | 295 | 29 | 0.55 |
| Jd | ng/ml | 9.0E-1 | 3.1E0 | 6.6E0 | 3.8E0 | 4.3E1 | 4.3E0 | 1.0E-9 | 1.0E-9 | 6.5E2 | 1.5E1 | 262 | 14 | 160 | 14 | 0.67 |
| Je | ng/ml | 1.0E-9 | 1.7E0 | 2.1E0 | 2.3E0 | 7.8E0 | 2.8E0 | 1.0E-9 | 1.0E-9 | 8.8E1 | 9.9E0 | 262 | 14 | 160 | 14 | 0.65 |
| Jf | ng/ml | 1.0E-9 | 1.3E-1 | 1.0E0 | 1.5E0 | 2.2E0 | 2.4E0 | 1.0E-9 | 1.0E-9 | 1.2E1 | 8.7E0 | 262 | 14 | 160 | 14 | 0.59 |
| Jg | ng/ml | 5.0E2 | 6.1E2 | 7.7E2 | 1.5E3 | 9.2E2 | 1.8E3 | 1.0E-9 | 1.1E1 | 1.0E4 | 6.8E3 | 756 | 29 | 296 | 29 | 0.58 |
| Jh | ng/ml | 2.8E0 | 7.8E0 | 2.4E1 | 4.5E1 | 1.1E2 | 6.8E1 | 1.0E-9 | 1.0E-9 | 1.6E3 | 2.4E2 | 756 | 29 | 296 | 29 | 0.62 |
| Ji | ng/ml | 5.2E1 | 6.2E1 | 7.7E1 | 1.8E2 | 8.9E1 | 3.4E2 | 1.0E-9 | 8.3E0 | 1.3E3 | 1.8E3 | 756 | 29 | 296 | 29 | 0.61 |
| Sr | pg/mL | 3.9E2 | 2.5E2 | 9.8E2 | 1.2E3 | 1.8E3 | 1.8E3 | 1.0E-9 | 1.0E-9 | 2.1E4 | 4.6E3 | 259 | 12 | 159 | 12 | 0.46 |
| Ss | pg/mL | 9.4E4 | 2.0E5 | 1.5E5 | 1.5E5 | 1.9E5 | 8.8E4 | 2.7E3 | 6.5E3 | 1.8E6 | 2.5E5 | 259 | 12 | 159 | 12 | 0.58 |
| St | pg/mL | 2.6E7 | 2.8E7 | 4.8E7 | 5.5E7 | 6.1E7 | 7.0E7 | 1.0E-9 | 3.4E6 | 5.4E8 | 2.6E8 | 257 | 14 | 157 | 14 | 0.50 |
| Ra | pg/ml | 1.0E-9 | 1.0E-9 | 8.4E-1 | 4.5E-1 | 4.1E0 | 9.3E-1 | 1.0E-9 | 1.0E-9 | 6.4E1 | 2.6E0 | 261 | 14 | 159 | 14 | 0.48 |
| Qz | pg/ml | 1.0E1 | 1.5E1 | 6.0E1 | 7.3E1 | 9.9E1 | 8.4E1 | 1.0E-9 | 1.0E-9 | 7.1E2 | 2.0E2 | 261 | 14 | 159 | 14 | 0.58 |
| Qy | pg/ml | 4.4E-1 | 6.8E-1 | 9.8E0 | 5.0E1 | 5.8E1 | 1.4E2 | 1.0E-9 | 1.0E-9 | 6.5E2 | 5.1E2 | 261 | 14 | 159 | 14 | 0.61 |
| Qx | pg/ml | 1.0E-9 | 1.0E-9 | 6.6E0 | 5.1E-1 | 5.1E1 | 1.9E0 | 1.0E-9 | 1.0E-9 | 5.8E2 | 7.1E0 | 261 | 14 | 159 | 14 | 0.47 |
| Qw | pg/ml | 1.0E-9 | 7.8E-1 | 1.9E0 | 7.1E0 | 9.3E0 | 2.3E1 | 1.0E-9 | 1.0E-9 | 1.2E2 | 8.6E1 | 261 | 14 | 159 | 14 | 0.59 |
| Qv | pg/ml | 2.3E4 | 1.0E4 | 3.6E4 | 2.0E4 | 7.8E4 | 2.0E4 | 6.0E1 | 1.0E-9 | 9.4E5 | 5.1E4 | 261 | 14 | 159 | 14 | 0.38 |
| Qu | pg/ml | 7.8E0 | 2.3E1 | 8.6E1 | 8.7E1 | 1.8E2 | 1.3E2 | 1.0E-9 | 1.0E-9 | 9.8E2 | 4.2E2 | 261 | 14 | 159 | 14 | 0.55 |
| Qt | pg/ml | 1.0E1 | 1.2E1 | 5.0E1 | 8.0E1 | 1.3E2 | 1.5E2 | 1.0E-9 | 1.0E-9 | 1.0E3 | 5.1E2 | 261 | 14 | 159 | 14 | 0.57 |
| Qh | ng/ml | 1.6E1 | 3.1E1 | 3.8E1 | 3.6E1 | 6.6E1 | 3.1E1 | 1.0E-9 | 3.6E0 | 6.4E2 | 1.1E2 | 261 | 14 | 159 | 14 | 0.59 |
| Qg | ng/ml | 7.8E0 | 1.2E1 | 1.6E1 | 2.5E1 | 2.6E1 | 3.6E1 | 5.1E-2 | 3.9E0 | 2.2E2 | 1.4E2 | 261 | 14 | 159 | 14 | 0.67 |
| Jj | ng/ml | 6.3E2 | 2.6E2 | 1.8E3 | 7.6E2 | 1.3E4 | 1.5E3 | 1.5E0 | 4.9E0 | 3.4E5 | 7.7E3 | 756 | 29 | 296 | 29 | 0.33 |
| Jk | ng/ml | 3.0E0 | 4.5E0 | 1.9E1 | 4.2E1 | 4.1E1 | 7.8E1 | 1.0E-9 | 1.1E-1 | 2.8E2 | 3.5E2 | 756 | 29 | 296 | 29 | 0.58 |
| Jl | ng/ml | 3.8E-1 | 1.1E0 | 1.8E0 | 2.7E1 | 4.6E0 | 1.0E2 | 7.6E-4 | 1.1E-3 | 4.0E1 | 5.4E2 | 756 | 29 | 296 | 29 | 0.64 |
| Jm | ng/ml | 1.6E1 | 1.9E1 | 5.9E1 | 2.9E1 | 1.4E2 | 3.5E1 | 1.0E-9 | 7.7E-1 | 2.1E3 | 1.3E2 | 756 | 29 | 296 | 29 | 0.49 |
| Jn | pg/ml | 4.0E-1 | 4.0E-1 | 3.2E0 | 1.7E0 | 3.2E1 | 3.0E0 | 1.0E-9 | 1.0E-9 | 6.2E2 | 1.2E1 | 755 | 29 | 296 | 29 | 0.53 |
| Jo | pg/ml | 3.6E3 | 2.3E3 | 5.0E3 | 4.4E3 | 5.3E3 | 7.1E3 | 2.0E1 | 7.5E1 | 1.0E5 | 3.8E4 | 756 | 29 | 296 | 29 | 0.37 |
| Jp | pg/ml | 6.9E4 | 9.0E4 | 7.2E4 | 8.7E4 | 3.8E4 | 4.3E4 | 5.8E2 | 2.8E3 | 3.8E5 | 1.7E5 | 756 | 29 | 296 | 29 | 0.62 |
| Jq | pg/ml | 9.4E1 | 6.5E1 | 1.6E2 | 3.9E2 | 3.5E2 | 9.7E2 | 1.0E0 | 5.4E0 | 8.7E3 | 4.0E3 | 756 | 29 | 296 | 29 | 0.48 |
| Jr | pg/ml | 5.2E0 | 6.7E0 | 4.0E1 | 2.5E1 | 4.4E2 | 4.2E1 | 1.0E-9 | 1.0E-9 | 1.1E4 | 1.9E2 | 756 | 29 | 296 | 29 | 0.60 |
| Js | pg/ml | 1.3E1 | 1.2E1 | 5.5E1 | 2.0E1 | 4.0E2 | 2.3E1 | 1.0E-9 | 1.0E-9 | 1.0E4 | 9.4E1 | 756 | 29 | 296 | 29 | 0.49 |
| Jt | pg/ml | 2.7E3 | 2.0E3 | 3.3E3 | 3.4E3 | 2.8E3 | 5.9E3 | 2.2E1 | 2.6E2 | 5.2E4 | 3.3E4 | 756 | 29 | 296 | 29 | 0.41 |
| Ju | mIU/ml | 9.1E0 | 1.2E1 | 2.0E1 | 1.6E1 | 3.1E1 | 1.9E1 | 4.8E-2 | 1.5E-1 | 2.3E2 | 7.1E1 | 262 | 14 | 160 | 14 | 0.50 |
| Jv | mIU/ml | 1.3E1 | 2.0E1 | 3.3E1 | 3.1E1 | 5.7E1 | 4.2E1 | 1.0E-2 | 2.1E-1 | 4.4E2 | 1.5E2 | 262 | 14 | 160 | 14 | 0.51 |
| Jy | ng/ml | 1.6E-3 | 1.7E-3 | 2.3E-3 | 2.2E-3 | 4.2E-3 | 1.4E-3 | 1.0E-9 | 1.0E-9 | 5.2E-2 | 4.8E-3 | 262 | 14 | 160 | 14 | 0.57 |
| Kc | pg/ml | 2.6E1 | 2.1E1 | 4.5E1 | 5.0E1 | 4.8E1 | 5.5E1 | 1.0E-9 | 1.0E-9 | 2.7E2 | 1.7E2 | 264 | 14 | 159 | 14 | 0.50 |
| Kd | pg/ml | 1.0E-9 | 1.0E-9 | 3.3E2 | 1.0E-9 | 2.4E3 | 0.0E0 | 1.0E-9 | 1.0E-9 | 3.8E4 | 1.0E-9 | 264 | 14 | 159 | 14 | 0.41 |
| Ke | pg/ml | 1.3E4 | 9.7E3 | 1.6E4 | 1.3E4 | 2.2E4 | 9.6E3 | 3.4E2 | 5.8E2 | 3.2E5 | 3.3E4 | 264 | 14 | 159 | 14 | 0.45 |
| Kf | pg/mL | 7.4E0 | 7.4E0 | 7.8E0 | 6.7E0 | 7.4E0 | 5.3E0 | 1.0E-9 | 1.0E-9 | 7.8E1 | 1.7E1 | 264 | 14 | 159 | 14 | 0.48 |
| Kg | pg/mL | 1.2E3 | 6.4E2 | 2.0E3 | 8.8E2 | 2.9E3 | 7.2E2 | 7.7E1 | 1.6E2 | 2.7E4 | 2.5E3 | 264 | 14 | 159 | 14 | 0.33 |
| Ki | pg/ml | 5.9E1 | 7.1E1 | 6.8E1 | 6.9E1 | 5.2E1 | 3.0E1 | 1.0E-9 | 1.3E1 | 3.8E2 | 1.1E2 | 263 | 14 | 159 | 14 | 0.57 |
| Kj | pg/ml | 9.8E2 | 8.4E2 | 1.7E3 | 1.0E3 | 1.9E3 | 8.0E2 | 3.0E1 | 1.2E2 | 1.5E4 | 2.8E3 | 264 | 14 | 159 | 14 | 0.42 |
| Kk | pg/ml | 6.8E0 | 8.8E0 | 1.2E1 | 1.3E1 | 1.6E1 | 1.5E1 | 1.0E-9 | 1.0E-9 | 1.6E2 | 5.5E1 | 264 | 14 | 159 | 14 | 0.52 |
| Kl | pg/ml | 2.1E4 | 2.1E4 | 2.8E4 | 2.6E4 | 2.6E4 | 2.2E4 | 2.3E2 | 2.1E2 | 1.6E5 | 5.7E4 | 264 | 14 | 159 | 14 | 0.47 |
| Kn | pg/ml | 3.0E1 | 2.9E1 | 8.1E1 | 3.7E1 | 3.1E2 | 4.4E1 | 1.0E-9 | 1.0E-9 | 4.9E3 | 1.7E2 | 264 | 14 | 159 | 14 | 0.48 |
| Ko | pg/ml | 4.1E2 | 5.2E1 | 5.2E2 | 2.6E2 | 5.2E2 | 4.0E2 | 1.0E-9 | 1.0E-9 | 4.1E3 | 1.4E3 | 264 | 14 | 159 | 14 | 0.31 |
| Kp | pg/ml | 3.4E2 | 3.4E2 | 4.1E2 | 4.0E2 | 8.4E2 | 3.1E2 | 1.0E-9 | 1.0E-9 | 1.3E4 | 9.4E2 | 264 | 14 | 159 | 14 | 0.53 |
| Kq | pg/ml | 3.2E2 | 2.8E2 | 1.1E3 | 4.7E2 | 9.9E3 | 5.5E2 | 5.1E0 | 7.0E0 | 1.6E5 | 1.7E3 | 257 | 14 | 154 | 14 | 0.44 |
| Kr | pg/ml | 5.6E-1 | 1.0E-9 | 4.0E0 | 3.7E-1 | 2.6E1 | 9.6E-1 | 1.0E-9 | 1.0E-9 | 4.2E2 | 3.4E0 | 257 | 14 | 154 | 14 | 0.31 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Ks | pg/ml | 1.4E4 | 4.2E3 | 2.0E4 | 1.0E4 | 1.9E4 | 1.2E4 | 2.2E2 | 1.1E3 | 1.1E5 | 4.0E4 | 257 | 14 | 154 | 14 | 0.31 |
| Kx | ng/ml | 1.0E-9 | 1.0E-9 | 6.9E-3 | 2.9E-3 | 1.4E-2 | 4.8E-3 | 1.0E-9 | 1.0E-9 | 1.0E-1 | 1.4E-2 | 263 | 13 | 159 | 13 | 0.45 |
| Ky | ng/ml | 9.8E-2 | 5.2E-2 | 3.8E-1 | 2.2E-1 | 8.4E-1 | 3.2E-1 | 1.0E-9 | 1.0E-9 | 6.4E0 | 9.0E-1 | 263 | 14 | 159 | 14 | 0.42 |
| Kz | ng/ml | 1.0E-9 | 1.0E-9 | 3.7E-3 | 3.4E-3 | 5.8E-3 | 6.0E-3 | 1.0E-9 | 1.0E-9 | 3.6E-2 | 1.4E-2 | 263 | 14 | 159 | 14 | 0.46 |
| Ld | pg/ml | 1.0E-9 | 1.0E-9 | 3.7E0 | 1.7E0 | 9.2E0 | 3.2E0 | 1.0E-9 | 1.0E-9 | 8.4E1 | 7.3E0 | 264 | 13 | 158 | 13 | 0.42 |
| Lh | pg/ml | 1.3E4 | 1.4E4 | 2.1E4 | 3.7E4 | 3.3E4 | 7.5E4 | 1.0E-9 | 1.8E2 | 4.8E5 | 4.1E5 | 756 | 29 | 297 | 29 | 0.58 |
| Li | pg/ml | 3.2E3 | 5.8E3 | 1.7E4 | 3.8E4 | 6.6E4 | 1.1E5 | 1.0E-9 | 1.7E2 | 1.3E6 | 5.9E5 | 756 | 29 | 297 | 29 | 0.58 |
| Lj | pg/ml | 2.9E3 | 4.4E3 | 2.4E4 | 2.0E4 | 6.8E4 | 6.4E4 | 1.0E-9 | 8.9E1 | 5.2E5 | 3.5E5 | 756 | 29 | 297 | 29 | 0.51 |
| Rm | ng/ml | 1.9E1 | 1.2E1 | 5.2E1 | 3.2E1 | 8.4E1 | 3.8E1 | 2.2E-1 | 3.0E0 | 6.5E2 | 1.3E2 | 256 | 14 | 158 | 14 | 0.46 |
| Rh | ng/ml | 1.3E2 | 7.4E1 | 4.0E2 | 1.5E2 | 1.4E3 | 1.8E2 | 4.7E0 | 3.6E0 | 1.7E4 | 5.5E2 | 256 | 14 | 158 | 14 | 0.36 |
| Ri | ng/ml | 1.0E-9 | 1.0E-9 | 4.4E0 | 2.6E0 | 1.6E1 | 5.5E0 | 1.0E-9 | 1.0E-9 | 2.2E2 | 2.0E1 | 257 | 14 | 159 | 14 | 0.48 |
| Rg | ng/ml | 1.0E-9 | 1.0E-9 | 5.0E-2 | 2.2E-3 | 3.0E-1 | 4.7E-3 | 1.0E-9 | 1.0E-9 | 3.0E0 | 1.4E-2 | 256 | 14 | 158 | 14 | 0.53 |
| Rj | ng/ml | 1.0E-9 | 1.0E-9 | 2.4E0 | 6.9E-1 | 1.8E1 | 1.7E0 | 1.0E-9 | 1.0E-9 | 2.7E2 | 6.3E0 | 257 | 14 | 159 | 14 | 0.46 |
| Rf | ng/ml | 4.1E-1 | 6.1E-1 | 1.0E0 | 1.7E0 | 1.8E0 | 1.7E0 | 7.8E-3 | 2.2E-2 | 1.5E1 | 3.9E0 | 256 | 14 | 158 | 14 | 0.60 |
| Ql | pg/ml | 4.5E0 | 1.2E1 | 1.3E1 | 1.6E1 | 2.4E1 | 2.2E1 | 1.0E-9 | 1.0E-9 | 1.8E2 | 6.7E1 | 262 | 14 | 160 | 14 | 0.55 |
| Qm | pg/ml | 4.1E0 | 1.0E-9 | 2.2E1 | 1.9E1 | 4.0E1 | 3.3E1 | 1.0E-9 | 1.0E-9 | 3.6E2 | 1.2E2 | 262 | 14 | 160 | 14 | 0.45 |
| Qn | pg/ml | 6.1E-1 | 8.5E-1 | 7.4E0 | 4.8E0 | 2.4E1 | 9.4E0 | 1.0E-9 | 1.0E-9 | 2.4E2 | 3.3E1 | 262 | 14 | 160 | 14 | 0.53 |
| Nv | pg/ml | 3.8E3 | 6.6E3 | 1.0E4 | 1.9E4 | 4.5E4 | 3.0E4 | 1.0E-9 | 8.4E1 | 1.1E6 | 1.3E5 | 762 | 29 | 297 | 29 | 0.62 |
| Nw | pg/ml | 8.8E3 | 1.3E4 | 1.3E4 | 2.9E4 | 1.7E4 | 4.5E4 | 8.6E1 | 5.7E2 | 2.1E5 | 2.1E5 | 762 | 29 | 297 | 29 | 0.60 |
| Nx | pg/ml | 2.2E2 | 2.6E2 | 4.1E2 | 5.8E2 | 6.6E2 | 7.5E2 | 1.0E-9 | 1.0E-9 | 7.4E3 | 2.8E3 | 762 | 29 | 297 | 29 | 0.59 |
| Ny | pg/ml | 6.0E0 | 7.6E0 | 6.1E1 | 4.8E1 | 9.2E2 | 1.2E2 | 1.0E-9 | 1.0E-9 | 2.5E4 | 6.2E2 | 762 | 29 | 297 | 29 | 0.54 |
| Oa | pg/ml | 1.8E2 | 2.4E2 | 4.4E2 | 4.0E2 | 7.4E2 | 4.0E2 | 1.0E-9 | 9.8E-1 | 4.8E3 | 1.1E3 | 262 | 14 | 160 | 14 | 0.55 |
| Oe | pg/ml | 4.2E1 | 3.7E1 | 2.7E2 | 2.6E2 | 7.9E2 | 4.4E2 | 1.0E-9 | 1.0E-9 | 1.9E4 | 1.6E3 | 755 | 29 | 296 | 29 | 0.49 |
| Of | pg/ml | 1.6E2 | 8.9E1 | 5.3E3 | 6.7E3 | 2.9E4 | 1.9E4 | 1.0E-9 | 1.0E-9 | 6.2E5 | 7.6E4 | 761 | 29 | 297 | 29 | 0.45 |
| Og | pg/ml | 7.9E-2 | 1.0E-1 | 4.6E-1 | 5.0E-1 | 1.5E0 | 1.2E0 | 1.0E-9 | 1.0E-9 | 2.0E1 | 5.0E0 | 761 | 29 | 297 | 29 | 0.55 |
| Oh | pg/ml | 2.5E0 | 3.3E0 | 2.0E1 | 1.6E1 | 1.5E2 | 4.4E1 | 1.0E-9 | 1.2E-2 | 3.5E3 | 2.2E2 | 761 | 29 | 297 | 29 | 0.58 |
| Oi | pg/ml | 2.4E0 | 3.0E0 | 5.9E0 | 7.0E0 | 9.6E0 | 9.4E0 | 1.0E-9 | 1.0E-9 | 8.6E1 | 2.8E1 | 761 | 29 | 297 | 29 | 0.53 |
| Ok | pg/ml | 3.9E2 | 5.0E2 | 5.3E2 | 3.3E3 | 5.7E2 | 1.3E4 | 1.3E1 | 4.0E1 | 7.8E3 | 7.0E4 | 761 | 29 | 297 | 29 | 0.54 |
| Om | pg/ml | 3.8E2 | 4.4E2 | 8.2E2 | 3.1E3 | 2.6E3 | 7.4E3 | 1.0E-9 | 1.0E-9 | 5.1E4 | 3.6E4 | 761 | 29 | 297 | 29 | 0.60 |
| On | pg/ml | 1.8E2 | 3.0E2 | 2.8E2 | 1.3E3 | 4.2E2 | 3.2E3 | 1.0E-9 | 7.2E0 | 4.5E3 | 1.5E4 | 761 | 29 | 297 | 29 | 0.59 |
| Or | pg/ml | 1.4E1 | 2.2E1 | 3.5E1 | 4.4E1 | 6.7E1 | 8.7E1 | 1.0E-9 | 1.0E-9 | 5.1E2 | 3.4E2 | 265 | 14 | 159 | 14 | 0.53 |
| Ow | pg/ml | 3.3E1 | 4.5E1 | 1.5E2 | 2.6E2 | 5.7E2 | 8.5E2 | 1.0E-9 | 1.0E-9 | 8.1E3 | 3.2E3 | 265 | 14 | 159 | 14 | 0.45 |
| Ou | pg/ml | 5.1E2 | 6.8E2 | 9.5E2 | 1.5E3 | 1.5E3 | 2.2E3 | 1.0E-9 | 1.0E-9 | 9.8E3 | 8.1E3 | 265 | 14 | 159 | 14 | 0.57 |
| Ul | ng/ml | 1.0E-9 | 1.0E-9 | 1.8E0 | 1.7E0 | 8.3E0 | 6.5E0 | 1.0E-9 | 1.0E-9 | 1.0E2 | 2.4E1 | 270 | 14 | 163 | 14 | 0.49 |
| Uo | ng/ml | 1.0E-9 | 1.0E-9 | 9.7E-2 | 3.8E-2 | 2.2E-1 | 1.2E-1 | 1.0E-9 | 1.0E-9 | 1.5E0 | 4.6E-1 | 270 | 14 | 163 | 14 | 0.42 |
| Uk | ng/ml | 1.0E-9 | 1.0E-9 | 8.5E-3 | 5.0E-5 | 2.7E-2 | 1.3E-4 | 1.0E-9 | 1.0E-9 | 3.2E-1 | 4.4E-4 | 270 | 14 | 163 | 14 | 0.32 |
| Um | ng/ml | 1.0E-9 | 1.0E-9 | 4.2E-1 | 1.5E-1 | 9.1E-1 | 2.6E-1 | 1.0E-9 | 1.0E-9 | 6.8E0 | 8.4E-1 | 270 | 14 | 163 | 14 | 0.44 |
| Uf | ng/ml | 6.5E-2 | 5.4E-2 | 1.8E-1 | 1.2E-1 | 6.4E-1 | 1.4E-1 | 1.3E-3 | 1.1E-3 | 8.6E0 | 4.2E-1 | 270 | 14 | 163 | 14 | 0.49 |
| Uh | ng/ml | 2.1E0 | 1.3E0 | 3.4E0 | 1.8E0 | 3.7E0 | 1.7E0 | 1.3E-2 | 6.0E-2 | 2.1E1 | 5.3E0 | 270 | 14 | 163 | 14 | 0.37 |
| Un | ng/ml | 1.9E0 | 2.1E0 | 2.2E0 | 2.5E0 | 1.9E0 | 1.7E0 | 1.3E-1 | 5.4E-1 | 2.5E1 | 5.6E0 | 270 | 14 | 163 | 14 | 0.55 |
| Ug | ng/ml | 1.5E1 | 8.9E0 | 2.9E1 | 1.8E1 | 3.2E1 | 2.0E1 | 6.9E-1 | 1.3E0 | 2.1E2 | 6.2E1 | 270 | 14 | 163 | 14 | 0.38 |
| Ur | ng/ml | 1.5E-1 | 9.7E-2 | 8.9E-1 | 2.8E-1 | 5.8E0 | 4.9E-1 | 1.0E-9 | 1.0E-9 | 9.3E1 | 1.8E0 | 269 | 14 | 162 | 14 | 0.44 |
| Up | ng/ml | 1.0E-9 | 1.0E-9 | 1.4E-2 | 3.9E-3 | 1.5E-1 | 9.0E-3 | 1.0E-9 | 1.0E-9 | 2.4E0 | 3.3E-2 | 269 | 14 | 162 | 14 | 0.56 |
| Us | ng/ml | 2.9E-3 | 6.3E-3 | 2.6E-2 | 1.3E-2 | 1.1E-1 | 1.8E-2 | 1.0E-9 | 1.0E-9 | 1.7E0 | 6.2E-2 | 269 | 14 | 162 | 14 | 0.56 |
| Uv | ng/ml | 3.1E-3 | 2.6E-3 | 1.4E-2 | 3.8E-3 | 4.7E-2 | 3.6E-3 | 1.0E-9 | 1.0E-9 | 4.3E-1 | 1.3E-2 | 269 | 14 | 162 | 14 | 0.49 |
| Ut | ng/ml | 6.6E-1 | 4.9E-1 | 2.9E0 | 1.9E0 | 9.6E0 | 2.7E0 | 1.0E-9 | 1.0E-9 | 7.8E1 | 7.8E0 | 269 | 14 | 162 | 14 | 0.48 |
| Uu | ng/ml | 7.1E0 | 8.8E0 | 7.9E0 | 8.9E0 | 5.6E0 | 5.9E0 | 5.5E-1 | 4.5E-1 | 4.0E1 | 1.9E1 | 269 | 14 | 162 | 14 | 0.55 |
| Vp | ng/ml | 1.0E-9 | 1.0E-9 | 5.6E-1 | 9.3E-2 | 4.6E0 | 3.1E-1 | 1.0E-9 | 1.0E-9 | 5.0E1 | 1.2E0 | 270 | 14 | 163 | 14 | 0.54 |
| Vt | ng/ml | 6.8E0 | 3.9E0 | 9.9E0 | 6.5E0 | 1.3E1 | 6.0E0 | 4.3E-1 | 8.8E-1 | 1.6E2 | 1.9E1 | 270 | 14 | 163 | 14 | 0.37 |
| Vu | ng/ml | 1.0E-9 | 1.0E-9 | 2.1E0 | 2.2E0 | 5.4E0 | 3.7E0 | 1.0E-9 | 1.0E-9 | 5.1E1 | 1.4E1 | 264 | 14 | 162 | 14 | 0.54 |
| Vq | ng/ml | 2.7E2 | 1.5E2 | 4.3E3 | 9.3E2 | 4.8E4 | 2.1E3 | 2.0E-1 | 6.5E0 | 6.8E5 | 7.1E3 | 201 | 11 | 131 | 11 | 0.48 |
| Vo | ng/ml | 2.6E1 | 2.5E1 | 2.5E1 | 2.3E1 | 4.7E0 | 6.9E0 | 6.7E0 | 2.4E0 | 3.5E1 | 2.8E1 | 270 | 14 | 163 | 14 | 0.43 |
| Vs | ng/ml | 1.0E-9 | 1.3E0 | 8.3E0 | 4.6E0 | 3.7E1 | 5.4E0 | 1.0E-9 | 1.0E-9 | 4.5E2 | 1.5E1 | 260 | 14 | 159 | 14 | 0.57 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vv | ng/ml | 2.9E0 | 4.0E0 | 5.8E0 | 5.2E0 | 9.7E0 | 6.2E0 | 1.0E-9 | 1.0E-9 | 8.4E1 | 2.1E1 | 268 | 14 | 162 | 14 | 0.49 |
| Oy | pg/ml | 4.9E-1 | 4.4E-1 | 5.9E0 | 1.3E1 | 3.1E1 | 5.1E1 | 1.0E-9 | 1.0E-9 | 4.0E2 | 2.7E2 | 760 | 29 | 296 | 29 | 0.49 |
| Oz | pg/ml | 1.4E-3 | 1.0E-9 | 3.2E-1 | 2.1E-1 | 1.4E0 | 2.5E-1 | 1.0E-9 | 1.0E-9 | 2.9E1 | 6.9E-1 | 760 | 29 | 296 | 29 | 0.51 |
| Pa | pg/ml | 3.8E-1 | 4.1E-1 | 1.7E0 | 1.1E1 | 6.5E0 | 5.4E1 | 1.0E-9 | 1.0E-9 | 1.0E2 | 2.9E2 | 760 | 29 | 296 | 29 | 0.51 |
| Pb | pg/ml | 1.0E-9 | 1.0E-9 | 9.0E-1 | 6.3E0 | 1.8E1 | 3.3E1 | 1.0E-9 | 1.0E-9 | 4.9E2 | 1.8E2 | 760 | 29 | 296 | 29 | 0.55 |
| Pc | pg/ml | 4.2E-2 | 2.3E-1 | 3.6E-1 | 9.5E-1 | 9.3E-1 | 2.3E0 | 1.0E-9 | 1.0E-9 | 1.6E1 | 1.1E1 | 760 | 29 | 296 | 29 | 0.57 |
| Pd | pg/ml | 1.9E0 | 1.5E0 | 5.4E0 | 2.8E0 | 3.2E1 | 4.1E0 | 1.0E-9 | 1.0E-9 | 8.4E2 | 1.8E1 | 760 | 29 | 296 | 29 | 0.46 |
| Pe | pg/ml | 2.1E1 | 2.3E1 | 1.2E2 | 5.6E2 | 4.8E2 | 2.6E3 | 1.0E-9 | 1.0E-9 | 6.7E3 | 1.4E4 | 760 | 29 | 296 | 29 | 0.51 |
| Pf | pg/ml | 1.6E0 | 2.0E0 | 1.2E1 | 1.2E1 | 6.3E1 | 4.2E1 | 1.0E-9 | 1.0E-9 | 1.5E3 | 2.3E2 | 760 | 29 | 296 | 29 | 0.51 |
| Pg | pg/ml | 3.3E0 | 6.2E0 | 4.8E1 | 7.3E1 | 3.7E2 | 3.5E2 | 1.0E-9 | 1.0E-9 | 7.7E3 | 1.9E3 | 760 | 29 | 296 | 29 | 0.55 |
| Ph | ng/ml | 1.8E-1 | 1.0E-1 | 3.5E-1 | 3.3E-1 | 5.7E-1 | 4.6E-1 | 1.0E-9 | 1.0E-9 | 5.4E0 | 1.6E0 | 265 | 14 | 159 | 14 | 0.45 |
| Pi | ng/ml | 2.0E-1 | 1.4E-1 | 5.8E-1 | 1.9E-1 | 5.0E0 | 1.8E-1 | 1.0E-9 | 1.0E-9 | 8.2E1 | 5.3E-1 | 265 | 14 | 159 | 14 | 0.41 |
| Pj | ng/mL | 5.7E0 | 2.6E0 | 6.4E0 | 5.4E0 | 4.5E0 | 6.7E0 | 3.8E-2 | 3.8E-1 | 3.1E1 | 2.5E1 | 265 | 14 | 159 | 14 | 0.34 |
| Pk | ng/ml | 8.8E-3 | 2.3E-3 | 1.8E-2 | 8.7E-3 | 9.4E-2 | 1.2E-2 | 1.0E-9 | 1.0E-9 | 1.5E0 | 3.1E-2 | 265 | 14 | 159 | 14 | 0.38 |
| aA | mg/dL | 8.0E-1 | 1.5E0 | 9.0E-1 | 1.9E0 | 4.3E-1 | 1.1E0 | 2.0E-1 | 4.9E-1 | 4.1E0 | 5.4E0 | 2356 | 48 | 452 | 48 | 0.82 |
| aC | mg/mL | 2.9E0 | 2.4E0 | 3.2E0 | 2.7E0 | 1.4E0 | 1.4E0 | 7.7E-1 | 1.2E0 | 8.9E0 | 6.7E0 | 466 | 18 | 180 | 18 | 0.39 |
| aD | ug/mL | 3.2E0 | 2.9E0 | 4.5E0 | 4.5E0 | 4.0E0 | 3.9E0 | 4.3E-1 | 1.1E0 | 3.5E1 | 1.7E1 | 466 | 18 | 180 | 18 | 0.49 |
| aE | mg/mL | 5.6E-1 | 5.1E-1 | 5.7E-1 | 5.7E-1 | 1.5E-1 | 1.6E-1 | 1.8E-1 | 3.8E-1 | 1.1E0 | 9.6E-1 | 466 | 18 | 180 | 18 | 0.45 |
| aF | ng/mL | 2.2E0 | 2.1E0 | 3.8E0 | 4.5E0 | 5.4E0 | 5.1E0 | 4.3E-3 | 3.4E-1 | 5.0E1 | 1.6E1 | 466 | 18 | 180 | 18 | 0.51 |
| aG | mg/mL | 1.3E-1 | 1.2E-1 | 1.5E-1 | 1.7E-1 | 8.7E-2 | 1.1E-1 | 1.7E-2 | 5.4E-2 | 5.4E-1 | 4.3E-1 | 466 | 18 | 180 | 18 | 0.51 |
| aH | ug/mL | 7.5E1 | 7.0E1 | 8.0E1 | 9.1E1 | 4.3E1 | 5.2E1 | 4.6E0 | 3.4E1 | 2.9E2 | 2.1E2 | 466 | 18 | 180 | 18 | 0.54 |
| aI | ug/mL | 1.9E2 | 1.9E2 | 1.9E2 | 1.9E2 | 6.0E1 | 5.3E1 | 2.8E1 | 9.3E1 | 3.7E2 | 3.0E2 | 466 | 18 | 180 | 18 | 0.50 |
| aJ | ug/mL | 2.4E0 | 4.9E0 | 3.0E0 | 6.0E0 | 2.0E0 | 4.3E0 | 7.3E-1 | 7.6E-1 | 1.7E1 | 1.6E1 | 466 | 18 | 180 | 18 | 0.74 |
| aK | ng/mL | 1.5E0 | 1.5E0 | 2.4E0 | 2.5E0 | 2.6E0 | 2.8E0 | 2.9E-4 | 1.0E-1 | 1.8E1 | 1.1E1 | 466 | 18 | 180 | 18 | 0.52 |
| aL | mg/mL | 8.0E-1 | 7.8E-1 | 8.1E-1 | 8.0E-1 | 2.7E-1 | 2.2E-1 | 1.9E-1 | 4.9E-1 | 1.7E0 | 1.3E0 | 466 | 18 | 180 | 18 | 0.48 |
| aM | U/mL | 2.2E1 | 2.4E1 | 5.1E1 | 3.1E1 | 1.1E2 | 2.4E1 | 4.2E-2 | 5.2E0 | 1.6E3 | 9.5E1 | 466 | 18 | 180 | 18 | 0.51 |
| aN | U/mL | 1.3E1 | 1.7E1 | 2.0E1 | 2.5E1 | 2.6E1 | 2.7E1 | 2.5E-3 | 4.5E0 | 3.3E2 | 9.9E1 | 466 | 18 | 180 | 18 | 0.58 |
| aO | pg/mL | 2.6E1 | 7.8E1 | 2.8E2 | 6.3E2 | 7.5E2 | 1.3E3 | 6.0E-2 | 6.0E-2 | 6.6E3 | 3.6E3 | 466 | 18 | 180 | 18 | 0.60 |
| aP | ng/mL | 1.6E0 | 2.9E0 | 2.0E0 | 3.4E0 | 1.8E0 | 1.9E0 | 4.5E-1 | 9.6E-1 | 2.8E1 | 6.7E0 | 466 | 18 | 180 | 18 | 0.75 |
| aQ | ng/mL | 2.9E-1 | 3.0E-1 | 4.4E-1 | 5.2E-1 | 4.6E-1 | 4.9E-1 | 2.0E-4 | 6.9E-2 | 4.0E0 | 1.8E0 | 466 | 18 | 180 | 18 | 0.55 |
| aR | ng/mL | 1.8E0 | 1.7E0 | 2.8E0 | 3.1E0 | 3.3E0 | 4.1E0 | 1.8E-1 | 2.5E-1 | 3.4E1 | 1.7E1 | 466 | 18 | 180 | 18 | 0.48 |
| aS | ng/mL | 2.6E-1 | 3.1E-1 | 6.4E-1 | 8.0E-1 | 1.8E0 | 2.0E0 | 4.2E-3 | 4.2E-3 | 3.3E1 | 8.7E0 | 466 | 18 | 180 | 18 | 0.53 |
| aU | pg/mL | 7.5E1 | 8.3E1 | 1.3E2 | 1.3E2 | 1.5E2 | 1.5E2 | 7.4E-2 | 7.4E-2 | 1.3E3 | 6.0E2 | 466 | 18 | 180 | 18 | 0.51 |
| aV | ng/mL | 6.1E-1 | 7.4E-1 | 1.1E0 | 7.8E-1 | 2.0E0 | 5.6E-1 | 7.6E-4 | 5.0E-2 | 3.3E1 | 2.0E0 | 466 | 18 | 180 | 18 | 0.51 |
| aW | pg/mL | 1.8E1 | 2.1E1 | 1.9E1 | 2.7E1 | 1.9E1 | 3.1E1 | 7.2E-2 | 7.2E-2 | 2.4E2 | 1.5E2 | 466 | 18 | 180 | 18 | 0.59 |
| aX | ng/mL | 9.8E0 | 1.2E1 | 1.5E1 | 4.4E1 | 1.9E1 | 7.8E1 | 3.0E-1 | 2.7E0 | 2.2E2 | 2.9E2 | 466 | 18 | 180 | 18 | 0.59 |
| aY | pg/mL | 6.0E1 | 5.2E1 | 7.7E1 | 7.0E1 | 8.6E1 | 4.5E1 | 4.1E-1 | 2.5E1 | 1.2E3 | 2.0E2 | 466 | 18 | 180 | 18 | 0.51 |
| aZ | pg/mL | 2.3E2 | 1.4E2 | 4.9E2 | 2.0E3 | 9.2E2 | 7.9E3 | 1.7E0 | 1.7E0 | 1.2E4 | 3.4E4 | 466 | 18 | 180 | 18 | 0.39 |
| bA | ng/mL | 8.4E0 | 4.7E1 | 3.5E1 | 1.8E2 | 1.0E2 | 3.3E2 | 3.0E-2 | 3.0E-2 | 9.4E2 | 1.3E3 | 466 | 18 | 180 | 18 | 0.70 |
| bB | ng/mL | 3.0E2 | 3.4E2 | 3.1E2 | 3.6E2 | 1.6E2 | 1.9E2 | 2.1E0 | 1.1E2 | 8.2E2 | 7.6E2 | 466 | 18 | 180 | 18 | 0.56 |
| bC | ng/mL | 3.5E2 | 5.8E2 | 6.2E2 | 9.1E2 | 8.2E2 | 1.0E3 | 2.7E1 | 9.8E0 | 4.7E3 | 4.0E3 | 466 | 18 | 180 | 18 | 0.57 |
| bE | mg/mL | 5.6E0 | 6.4E0 | 5.8E0 | 6.6E0 | 2.1E0 | 2.5E0 | 9.8E-1 | 2.9E0 | 1.3E1 | 1.2E1 | 466 | 18 | 180 | 18 | 0.58 |
| bF | pg/mL | 2.1E1 | 2.4E1 | 1.6E2 | 5.0E1 | 9.3E2 | 6.8E1 | 5.0E-2 | 2.1E0 | 1.1E4 | 3.0E2 | 466 | 18 | 180 | 18 | 0.54 |
| bG | ng/mL | 1.6E0 | 1.7E0 | 2.7E0 | 1.5E0 | 3.2E0 | 9.3E-1 | 2.2E-2 | 3.5E-1 | 2.3E1 | 3.3E0 | 466 | 18 | 180 | 18 | 0.44 |
| bH | pg/mL | 5.7E-1 | 5.7E-1 | 5.0E0 | 5.0E0 | 1.5E1 | 7.5E0 | 5.7E-1 | 5.7E-1 | 2.8E2 | 2.2E1 | 466 | 18 | 180 | 18 | 0.51 |
| bI | ng/mL | 4.0E-3 | 4.0E-3 | 6.5E-2 | 6.4E-2 | 1.6E-1 | 1.9E-1 | 4.0E-3 | 4.0E-3 | 1.3E0 | 7.7E-1 | 466 | 18 | 180 | 18 | 0.47 |
| bJ | mg/mL | 2.4E0 | 1.9E0 | 2.7E0 | 2.7E0 | 2.1E0 | 2.3E0 | 2.5E-4 | 2.5E-4 | 1.3E1 | 8.3E0 | 466 | 18 | 180 | 18 | 0.47 |
| bL | pg/mL | 3.7E0 | 5.3E0 | 8.4E0 | 6.4E0 | 1.1E1 | 7.1E0 | 4.6E-2 | 4.6E-2 | 8.0E1 | 2.5E1 | 466 | 18 | 180 | 18 | 0.46 |
| bM | mg/mL | 1.8E0 | 1.6E0 | 2.1E0 | 1.9E0 | 1.4E0 | 1.1E0 | 9.2E-3 | 5.0E-1 | 8.8E0 | 5.1E0 | 466 | 18 | 180 | 18 | 0.48 |
| bN | ng/mL | 4.6E1 | 2.5E1 | 1.3E2 | 9.5E1 | 2.8E2 | 1.9E2 | 1.4E-1 | 1.4E-1 | 1.9E3 | 7.7E2 | 466 | 18 | 180 | 18 | 0.38 |
| bO | ng/mL | 4.0E-2 | 1.8E0 | 1.1E1 | 9.5E0 | 2.5E1 | 2.3E1 | 4.0E-2 | 4.0E-2 | 2.0E2 | 1.0E2 | 466 | 18 | 180 | 18 | 0.54 |
| bP | mg/mL | 5.4E-1 | 6.9E-1 | 7.7E-1 | 8.8E-1 | 6.9E-1 | 6.2E-1 | 4.9E-2 | 1.9E-1 | 4.8E0 | 2.2E0 | 466 | 18 | 180 | 18 | 0.57 |
| bQ | pg/mL | 1.6E1 | 1.5E1 | 6.2E1 | 3.4E1 | 6.3E2 | 5.2E1 | 1.5E-1 | 1.5E-1 | 1.3E4 | 2.2E2 | 466 | 18 | 180 | 18 | 0.50 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bR | ng/mL | 1.2E-2 | 1.2E-2 | 1.3E-1 | 1.4E-1 | 4.5E-1 | 2.8E-1 | 1.2E-2 | 1.2E-2 | 8.7E0 | 1.2E0 | 466 | 18 | 180 | 18 | 0.50 |
| bS | pg/mL | 9.4E-1 | 9.4E-1 | 7.1E0 | 3.9E0 | 2.8E1 | 1.2E1 | 9.4E-1 | 9.4E-1 | 3.9E2 | 5.4E1 | 466 | 18 | 180 | 18 | 0.46 |
| bU | ng/mL | 1.2E-1 | 1.4E-1 | 2.0E-1 | 2.6E-1 | 3.7E-1 | 4.0E-1 | 1.3E-2 | 1.3E-2 | 6.6E0 | 1.7E0 | 466 | 18 | 180 | 18 | 0.52 |
| bV | pg/mL | 4.6E2 | 6.3E2 | 5.5E2 | 1.6E3 | 5.8E2 | 3.8E3 | 1.5E2 | 1.7E2 | 1.2E4 | 1.7E4 | 466 | 18 | 180 | 18 | 0.67 |
| bW | pg/mL | 3.4E2 | 4.1E2 | 6.4E2 | 6.4E2 | 1.7E3 | 9.6E2 | 8.4E1 | 9.2E1 | 2.5E4 | 4.4E3 | 466 | 18 | 180 | 18 | 0.53 |
| bX | ng/mL | 6.9E-4 | 2.5E-5 | 2.7E-3 | 1.6E-3 | 3.4E-3 | 3.4E-3 | 2.5E-5 | 2.5E-5 | 2.0E-2 | 1.1E-2 | 466 | 18 | 180 | 18 | 0.38 |
| bZ | pg/mL | 2.3E2 | 3.8E2 | 8.5E2 | 1.2E3 | 4.0E3 | 1.9E3 | 1.5E-1 | 1.5E-1 | 5.8E4 | 8.2E3 | 466 | 18 | 180 | 18 | 0.63 |
| cA | pg/mL | 6.0E-1 | 6.0E-1 | 2.8E0 | 2.3E0 | 1.8E1 | 4.0E0 | 6.0E-1 | 6.0E-1 | 3.7E2 | 1.3E1 | 466 | 18 | 180 | 18 | 0.52 |
| cB | ng/mL | 5.7E-2 | 5.8E-2 | 9.0E-2 | 1.2E-1 | 1.0E-1 | 1.7E-1 | 1.7E-3 | 1.7E-3 | 5.7E-1 | 5.5E-1 | 466 | 18 | 180 | 18 | 0.51 |
| cC | pg/mL | 4.6E1 | 4.4E1 | 4.8E1 | 4.5E1 | 4.0E1 | 3.4E1 | 1.0E0 | 1.0E0 | 4.5E2 | 1.4E2 | 466 | 18 | 180 | 18 | 0.47 |
| cD | pg/mL | 5.6E0 | 3.7E0 | 1.3E1 | 9.8E0 | 4.9E1 | 1.8E1 | 3.3E-1 | 3.3E-1 | 7.2E2 | 6.9E1 | 466 | 18 | 180 | 18 | 0.40 |
| cE | pg/mL | 3.7E1 | 5.3E1 | 1.7E2 | 7.7E1 | 4.8E2 | 9.0E1 | 1.2E-1 | 1.2E-1 | 3.8E3 | 3.1E2 | 466 | 18 | 180 | 18 | 0.49 |
| cF | pg/mL | 1.3E1 | 6.6E0 | 2.0E1 | 2.4E1 | 3.0E1 | 5.0E1 | 5.3E-1 | 5.3E-1 | 2.7E2 | 2.2E2 | 466 | 18 | 180 | 18 | 0.47 |
| cG | pg/mL | 4.5E1 | 7.0E1 | 1.1E2 | 9.7E1 | 5.1E2 | 1.3E2 | 6.4E0 | 1.1E1 | 1.0E4 | 5.5E2 | 466 | 18 | 180 | 18 | 0.54 |
| cH | uIU/mL | 2.8E0 | 2.2E0 | 5.8E0 | 5.5E0 | 1.1E1 | 6.8E0 | 8.6E-3 | 2.5E-1 | 1.6E2 | 2.0E1 | 466 | 18 | 180 | 18 | 0.45 |
| cI | ng/mL | 5.5E0 | 1.0E1 | 1.1E1 | 3.1E1 | 1.5E1 | 4.7E1 | 1.0E-3 | 7.1E-2 | 1.2E2 | 1.9E2 | 466 | 18 | 180 | 18 | 0.65 |
| cJ | ug/mL | 5.6E1 | 8.9E1 | 1.1E2 | 1.5E2 | 1.4E2 | 1.6E2 | 4.0E0 | 6.5E0 | 9.6E2 | 6.2E2 | 466 | 18 | 180 | 18 | 0.61 |
| cK | ng/mL | 3.8E-3 | 3.8E-3 | 5.1E-2 | 2.0E-2 | 1.8E-1 | 6.9E-2 | 3.8E-3 | 3.8E-3 | 1.7E0 | 3.0E-1 | 466 | 18 | 180 | 18 | 0.45 |
| cL | pg/mL | 2.0E2 | 2.1E2 | 3.7E2 | 2.8E2 | 1.3E3 | 2.7E2 | 1.6E1 | 4.8E1 | 2.4E4 | 1.2E3 | 466 | 18 | 180 | 18 | 0.51 |
| cM | pg/mL | 2.6E2 | 2.2E2 | 2.9E2 | 2.7E2 | 2.0E2 | 1.6E2 | 8.7E0 | 4.0E1 | 1.6E3 | 5.3E2 | 466 | 18 | 180 | 18 | 0.47 |
| cN | pg/mL | 1.2E2 | 1.5E2 | 1.3E2 | 1.5E2 | 6.5E1 | 5.8E1 | 3.8E1 | 4.6E1 | 1.1E3 | 2.8E2 | 466 | 18 | 180 | 18 | 0.67 |
| cO | pg/mL | 2.3E2 | 2.0E2 | 3.2E2 | 2.5E2 | 9.0E2 | 1.9E2 | 5.4E1 | 6.1E1 | 1.9E4 | 8.8E2 | 466 | 18 | 180 | 18 | 0.42 |
| cP | ng/mL | 2.5E3 | 2.8E3 | 2.6E3 | 2.9E3 | 9.1E2 | 1.1E3 | 6.2E2 | 1.7E3 | 5.7E3 | 5.1E3 | 466 | 18 | 180 | 18 | 0.58 |
| cQ | ng/mL | 5.3E-2 | 8.3E-2 | 1.5E-1 | 1.5E-1 | 3.0E-1 | 1.9E-1 | 2.0E-3 | 2.0E-3 | 2.2E0 | 7.3E-1 | 466 | 18 | 180 | 18 | 0.54 |
| cR | ng/mL | 3.0E2 | 3.3E2 | 5.2E2 | 8.6E2 | 8.2E2 | 1.8E3 | 2.0E1 | 9.5E1 | 8.9E3 | 7.7E3 | 466 | 18 | 180 | 18 | 0.55 |
| cS | ng/mL | 2.5E2 | 4.8E2 | 4.0E2 | 1.9E3 | 4.0E2 | 5.2E3 | 4.1E1 | 8.1E1 | 2.7E3 | 2.2E4 | 466 | 18 | 180 | 18 | 0.62 |
| cT | ng/mL | 3.3E1 | 6.3E1 | 8.4E1 | 2.7E2 | 1.9E2 | 4.6E2 | 3.7E0 | 8.0E0 | 2.1E3 | 1.9E3 | 466 | 18 | 180 | 18 | 0.65 |
| cU | ng/mL | 5.3E1 | 6.5E1 | 7.6E1 | 1.0E2 | 1.0E2 | 1.2E2 | 5.4E0 | 1.6E1 | 1.6E3 | 5.1E2 | 466 | 18 | 180 | 18 | 0.61 |
| cV | ng/mL | 1.8E-1 | 2.1E-1 | 4.0E-1 | 5.2E-1 | 2.2E0 | 1.4E0 | 2.5E-2 | 4.1E-2 | 4.7E1 | 6.0E0 | 466 | 18 | 180 | 18 | 0.53 |
| cW | mIU/mL | 5.3E-2 | 6.0E-2 | 1.5E-1 | 6.3E-2 | 7.0E-1 | 4.3E-2 | 3.7E-4 | 1.0E-2 | 9.7E0 | 1.6E-1 | 466 | 18 | 180 | 18 | 0.47 |
| cX | ng/mL | 1.1E-1 | 6.9E-2 | 1.2E0 | 1.6E0 | 3.8E0 | 5.1E0 | 2.3E-4 | 2.3E-4 | 2.8E1 | 2.2E1 | 466 | 18 | 180 | 18 | 0.52 |
| cY | ng/mL | 8.6E0 | 7.9E0 | 1.3E1 | 1.2E1 | 1.3E1 | 1.3E1 | 1.5E-1 | 3.1E-1 | 8.3E1 | 5.2E1 | 466 | 18 | 180 | 18 | 0.49 |
| cZ | ug/mL | 1.5E1 | 1.6E1 | 1.6E1 | 1.8E1 | 7.4E0 | 7.6E0 | 2.3E0 | 1.1E1 | 5.7E1 | 4.3E1 | 466 | 18 | 180 | 18 | 0.61 |
| dA | pg/mL | 3.2E2 | 3.8E2 | 3.7E2 | 4.5E2 | 3.1E2 | 1.9E2 | 9.0E1 | 2.1E2 | 5.8E3 | 7.7E2 | 466 | 18 | 180 | 18 | 0.63 |
| dB | ug/mL | 1.7E1 | 2.2E1 | 1.8E1 | 1.8E1 | 1.6E1 | 1.1E1 | 9.4E-1 | 2.6E0 | 2.5E2 | 4.0E1 | 466 | 18 | 180 | 18 | 0.57 |
| dC | nmol/L | 3.5E1 | 3.2E1 | 3.8E1 | 3.9E1 | 1.8E1 | 2.1E1 | 7.6E0 | 1.5E1 | 1.4E2 | 8.7E1 | 466 | 18 | 180 | 18 | 0.47 |
| dD | ug/mL | 3.6E1 | 3.3E1 | 3.7E1 | 3.6E1 | 1.1E1 | 1.1E1 | 1.3E1 | 2.3E1 | 7.6E1 | 6.4E1 | 466 | 18 | 180 | 18 | 0.45 |
| dE | ng/mL | 4.8E-1 | 4.3E-1 | 6.1E-1 | 5.8E-1 | 7.0E-1 | 8.3E-1 | 8.4E-3 | 8.4E-3 | 7.2E0 | 3.3E0 | 466 | 18 | 180 | 18 | 0.45 |
| dF | ng/mL | 2.3E2 | 3.0E2 | 2.8E2 | 3.1E2 | 1.9E2 | 1.4E2 | 5.6E1 | 8.6E1 | 1.3E3 | 4.9E2 | 466 | 18 | 180 | 18 | 0.61 |
| dG | ng/mL | 1.1E1 | 1.7E1 | 1.5E1 | 1.9E1 | 1.4E1 | 9.1E0 | 2.2E0 | 3.3E0 | 1.8E2 | 3.3E1 | 466 | 18 | 180 | 18 | 0.67 |
| dH | pg/mL | 7.5E0 | 1.0E1 | 1.3E1 | 1.2E1 | 3.9E1 | 9.3E0 | 4.0E-2 | 4.0E-2 | 6.7E2 | 3.6E1 | 466 | 18 | 180 | 18 | 0.58 |
| dI | pg/mL | 4.6E-1 | 4.6E-1 | 2.3E0 | 3.9E0 | 1.6E1 | 9.4E0 | 4.6E-1 | 4.6E-1 | 3.3E2 | 4.1E1 | 466 | 18 | 180 | 18 | 0.60 |
| dJ | ng/mL | 1.9E0 | 2.5E0 | 2.2E0 | 2.3E0 | 1.1E0 | 1.1E0 | 3.2E-2 | 4.9E-1 | 6.9E0 | 4.4E0 | 466 | 18 | 180 | 18 | 0.56 |
| dK | uIU/mL | 1.9E0 | 2.1E0 | 3.1E0 | 2.7E0 | 6.5E0 | 3.6E0 | 2.8E-4 | 4.0E-2 | 7.9E1 | 1.6E1 | 466 | 18 | 180 | 18 | 0.50 |
| dL | ng/mL | 8.8E2 | 9.9E2 | 1.0E3 | 1.1E3 | 5.9E2 | 3.4E2 | 2.6E2 | 4.9E2 | 4.8E3 | 1.6E3 | 466 | 18 | 180 | 18 | 0.58 |
| dM | pg/mL | 9.5E2 | 1.4E3 | 1.2E3 | 2.1E3 | 1.4E3 | 1.6E3 | 3.4E2 | 4.7E2 | 1.6E4 | 7.2E3 | 466 | 18 | 180 | 18 | 0.73 |
| dN | ug/mL | 9.4E1 | 1.0E2 | 1.0E2 | 1.2E2 | 4.1E1 | 4.1E1 | 1.6E1 | 6.7E1 | 3.3E2 | 2.2E2 | 466 | 18 | 180 | 18 | 0.62 |
| dR | pg/ml | 1.6E3 | 1.6E3 | 2.4E3 | 2.2E3 | 2.4E3 | 1.8E3 | 1.4E2 | 2.9E2 | 1.5E4 | 8.2E3 | 312 | 17 | 171 | 17 | 0.54 |
| eF | ng/ml | 4.1E0 | 6.3E0 | 5.0E0 | 6.3E0 | 4.2E0 | 3.1E0 | 1.2E0 | 1.6E0 | 4.6E1 | 1.2E1 | 324 | 18 | 172 | 18 | 0.67 |
| eC | pg/ml | 3.0E2 | 3.0E2 | 3.8E2 | 3.2E2 | 2.9E2 | 1.6E2 | 9.9E0 | 5.1E1 | 2.0E3 | 6.1E2 | 246 | 13 | 160 | 13 | 0.47 |
| eD | pg/ml | 2.1E2 | 2.1E2 | 5.0E2 | 2.1E2 | 1.1E3 | 1.6E2 | 5.2E-1 | 2.8E1 | 8.3E3 | 6.3E2 | 196 | 12 | 131 | 12 | 0.43 |
| eM | ng/ml | 3.4E0 | 1.2E1 | 4.7E0 | 1.3E1 | 4.7E0 | 1.0E1 | 6.9E-1 | 2.5E0 | 2.7E1 | 3.2E1 | 176 | 7 | 70 | 7 | 0.81 |
| fP | ng/ml | 2.6E2 | 3.6E2 | 2.9E2 | 4.3E2 | 1.7E2 | 4.0E2 | 8.4E0 | 2.7E1 | 1.0E3 | 1.6E3 | 298 | 16 | 163 | 16 | 0.58 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| fR | ng/ml | 1.3E5 | 2.9E5 | 1.8E5 | 3.3E5 | 1.5E5 | 2.1E5 | 2.9E4 | 6.1E4 | 8.3E5 | 7.2E5 | 320 | 11 | 97 | 11 | 0.73 |
| gC | ng/ml | 2.3E2 | 2.8E2 | 2.6E2 | 2.9E2 | 1.3E2 | 1.3E2 | 8.3E1 | 1.7E2 | 1.1E3 | 5.9E2 | 135 | 8 | 79 | 8 | 0.61 |
| gL | pg/ml | 6.4E4 | 7.4E4 | 7.0E4 | 8.7E4 | 2.9E4 | 4.3E4 | 1.4E4 | 3.1E4 | 2.0E5 | 1.6E5 | 312 | 17 | 171 | 17 | 0.62 |
| gP | U/ml | 2.7E2 | 2.7E2 | 2.7E2 | 3.1E2 | 9.4E1 | 1.6E2 | 1.2E2 | 7.1E1 | 8.0E2 | 8.5E2 | 320 | 18 | 172 | 18 | 0.56 |
| gW | ng/ml | 6.1E2 | 8.5E2 | 1.3E3 | 1.2E3 | 1.7E3 | 1.3E3 | 3.1E-1 | 1.1E2 | 9.5E3 | 5.1E3 | 273 | 16 | 163 | 16 | 0.57 |
| tF | pg/mL | 1.5E3 | 1.6E3 | 1.2E4 | 4.0E3 | 3.8E4 | 5.0E3 | 1.2E1 | 1.8E1 | 3.2E5 | 1.5E4 | 246 | 13 | 160 | 13 | 0.56 |
| hA | ng/ml | 2.1E0 | 2.9E0 | 9.9E0 | 3.7E0 | 3.7E1 | 4.2E0 | 1.7E-2 | 5.7E-1 | 3.5E2 | 1.5E1 | 197 | 12 | 131 | 12 | 0.53 |
| nM | pg/ml | 1.0E-9 | 1.0E-9 | 7.7E1 | 1.0E-9 | 8.9E2 | 0.0E0 | 1.0E-9 | 1.0E-9 | 1.0E4 | 1.0E-9 | 133 | 10 | 101 | 10 | 0.49 |
| nN | pg/ml | 1.2E3 | 1.2E3 | 4.2E3 | 1.4E3 | 2.3E4 | 9.1E2 | 1.1E2 | 1.2E2 | 2.7E5 | 2.9E3 | 133 | 10 | 101 | 10 | 0.48 |
| nO | pg/ml | 2.6E1 | 3.2E1 | 4.5E1 | 4.7E1 | 5.3E1 | 4.1E1 | 3.5E0 | 1.1E1 | 3.1E2 | 1.3E2 | 133 | 10 | 101 | 10 | 0.54 |
| nR | pg/ml | 1.5E1 | 2.7E1 | 3.2E1 | 1.1E2 | 4.5E1 | 2.2E2 | 1.0E-9 | 5.5E-1 | 2.6E2 | 7.1E2 | 133 | 10 | 101 | 10 | 0.57 |
| nT | pg/ml | 8.0E1 | 8.8E1 | 2.0E2 | 2.7E2 | 7.9E2 | 5.3E2 | 1.0E-9 | 2.9E1 | 6.6E3 | 1.8E3 | 133 | 10 | 101 | 10 | 0.58 |
| nU | pg/ml | 2.9E1 | 6.7E1 | 2.5E2 | 3.7E2 | 1.4E3 | 9.2E2 | 1.0E-9 | 1.0E-9 | 1.3E4 | 3.0E3 | 133 | 10 | 101 | 10 | 0.63 |
| lW | pg/ml | 1.0E-9 | 1.0E-9 | 1.4E1 | 1.1E1 | 4.9E1 | 1.7E1 | 1.0E-9 | 1.0E-9 | 3.9E2 | 4.2E1 | 133 | 10 | 101 | 10 | 0.56 |
| lX | pg/ml | 9.5E2 | 9.9E2 | 1.0E3 | 1.1E3 | 5.8E2 | 5.6E2 | 1.2E2 | 3.5E2 | 2.6E3 | 2.3E3 | 133 | 10 | 101 | 10 | 0.56 |
| lY | pg/ml | 1.9E1 | 2.3E1 | 2.2E1 | 2.8E1 | 1.9E1 | 2.6E1 | 1.0E-9 | 5.4E0 | 1.4E2 | 9.6E1 | 133 | 10 | 101 | 10 | 0.56 |
| mE | pg/ml | 1.0E-9 | 6.0E-1 | 2.9E0 | 1.1E0 | 8.5E0 | 1.2E0 | 1.0E-9 | 1.0E-9 | 5.8E1 | 2.9E0 | 133 | 10 | 101 | 10 | 0.52 |
| mF | pg/ml | 1.0E-9 | 1.0E-9 | 2.4E0 | 1.1E0 | 9.4E0 | 2.0E0 | 1.0E-9 | 1.0E-9 | 1.1E2 | 6.1E0 | 133 | 10 | 101 | 10 | 0.46 |
| mH | pg/ml | 3.6E0 | 2.7E0 | 5.3E0 | 3.1E0 | 6.6E0 | 2.2E0 | 2.3E-1 | 4.0E-1 | 5.3E1 | 8.2E0 | 133 | 10 | 101 | 10 | 0.41 |
| mI | pg/ml | 1.0E-9 | 1.8E0 | 1.5E1 | 7.3E0 | 3.1E1 | 9.6E0 | 1.0E-9 | 1.0E-9 | 1.8E2 | 2.7E1 | 133 | 10 | 101 | 10 | 0.50 |
| mM | pg/ml | 2.2E1 | 4.0E1 | 6.9E1 | 4.7E1 | 1.3E2 | 4.4E1 | 1.0E-9 | 1.8E0 | 9.8E2 | 1.5E2 | 133 | 10 | 101 | 10 | 0.58 |
| mP | pg/ml | 1.4E1 | 1.4E1 | 1.8E1 | 1.7E1 | 2.2E1 | 1.3E1 | 1.0E-9 | 1.0E-9 | 1.9E2 | 3.7E1 | 132 | 10 | 100 | 10 | 0.55 |
| mS | pg/ml | 1.6E3 | 1.8E3 | 1.8E3 | 2.4E3 | 1.6E3 | 1.7E3 | 1.0E-9 | 7.9E2 | 1.3E4 | 5.9E3 | 133 | 10 | 101 | 10 | 0.59 |
| mT | pg/ml | 5.4E1 | 8.0E1 | 1.3E2 | 2.5E2 | 2.2E2 | 5.9E2 | 9.7E0 | 2.1E1 | 1.4E3 | 1.9E3 | 132 | 10 | 100 | 10 | 0.53 |
| mU | pg/ml | 2.2E0 | 3.0E0 | 4.0E0 | 3.3E0 | 8.3E0 | 2.0E0 | 1.0E-9 | 1.0E-9 | 6.8E1 | 7.2E0 | 132 | 10 | 100 | 10 | 0.63 |
| mW | pg/ml | 2.3E3 | 3.9E3 | 2.7E3 | 3.5E3 | 2.1E3 | 1.8E3 | 1.0E-9 | 6.7E2 | 1.8E4 | 7.0E3 | 132 | 10 | 100 | 10 | 0.67 |
| mY | pg/ml | 5.5E2 | 7.3E2 | 8.5E2 | 9.6E2 | 1.3E3 | 9.0E2 | 1.0E-9 | 6.1E1 | 1.1E4 | 2.7E3 | 133 | 10 | 101 | 10 | 0.56 |
| mZ | pg/ml | 1.7E2 | 5.8E2 | 2.9E2 | 6.3E2 | 3.0E2 | 4.5E2 | 1.0E-9 | 9.2E1 | 1.5E3 | 1.7E3 | 132 | 10 | 100 | 10 | 0.77 |
| nA | pg/ml | 1.6E0 | 4.3E0 | 1.0E1 | 7.2E0 | 4.3E1 | 9.4E0 | 1.0E-9 | 1.0E-9 | 4.4E2 | 3.1E1 | 132 | 10 | 100 | 10 | 0.62 |
| nB | pg/ml | 3.1E2 | 4.1E2 | 3.2E2 | 4.2E2 | 1.7E2 | 1.6E2 | 3.0E1 | 2.2E2 | 9.1E2 | 7.2E2 | 133 | 10 | 101 | 10 | 0.68 |
| nC | pg/ml | 1.0E-9 | 1.0E-9 | 2.5E4 | 3.8E3 | 1.5E5 | 7.7E3 | 1.0E-9 | 1.0E-9 | 1.5E6 | 2.0E4 | 133 | 10 | 101 | 10 | 0.47 |
| nD | pg/ml | 7.9E0 | 1.0E1 | 3.6E1 | 2.4E1 | 2.0E2 | 4.4E1 | 1.0E-9 | 1.0E-9 | 1.7E3 | 1.5E2 | 132 | 10 | 100 | 10 | 0.55 |
| nF | pg/ml | 1.0E-9 | 1.0E-9 | 4.5E0 | 3.6E0 | 2.5E1 | 1.1E1 | 1.0E-9 | 1.0E-9 | 2.4E2 | 3.6E1 | 133 | 10 | 101 | 10 | 0.48 |
| nH | pg/ml | 1.0E0 | 1.0E-9 | 2.2E2 | 5.5E1 | 1.3E3 | 1.1E2 | 1.0E-9 | 1.0E-9 | 1.0E4 | 2.9E2 | 132 | 10 | 100 | 10 | 0.43 |
| nI | pg/ml | 3.0E1 | 9.2E1 | 1.5E2 | 1.8E2 | 8.3E2 | 3.6E2 | 1.0E-9 | 1.0E-9 | 9.2E3 | 1.2E3 | 133 | 10 | 101 | 10 | 0.61 |
| nJ | pg/ml | 5.9E-2 | 7.4E-1 | 4.1E1 | 3.5E0 | 4.5E2 | 5.9E0 | 1.0E-9 | 1.0E-9 | 5.2E3 | 1.6E1 | 133 | 10 | 101 | 10 | 0.61 |
| nK | pg/ml | 1.0E-9 | 7.9E0 | 5.6E1 | 5.4E1 | 3.4E2 | 9.2E1 | 1.0E-9 | 1.0E-9 | 3.2E3 | 2.3E2 | 132 | 10 | 100 | 10 | 0.56 |
| nL | pg/ml | 1.0E-9 | 1.0E-9 | 5.9E2 | 2.0E2 | 4.1E3 | 4.3E2 | 1.0E-9 | 1.0E-9 | 4.5E4 | 1.2E3 | 133 | 10 | 101 | 10 | 0.50 |
| hR | pg/ml | 2.6E4 | 2.4E4 | 2.7E4 | 2.3E4 | 1.1E4 | 7.1E3 | 1.0E-9 | 1.2E4 | 5.8E4 | 3.9E4 | 187 | 12 | 127 | 12 | 0.38 |
| hV | pg/ml | 4.4E2 | 3.7E2 | 4.6E2 | 4.2E2 | 2.4E2 | 1.9E2 | 1.0E-9 | 1.9E2 | 1.5E3 | 7.8E2 | 187 | 12 | 127 | 12 | 0.45 |
| hW | pg/ml | 1.6E3 | 1.3E3 | 2.1E3 | 2.1E3 | 3.0E3 | 2.7E3 | 1.0E-9 | 5.7E2 | 4.0E4 | 1.0E4 | 187 | 12 | 127 | 12 | 0.43 |
| hX | pg/ml | 9.0E2 | 9.1E2 | 1.0E3 | 1.2E3 | 7.3E2 | 1.2E3 | 2.5E0 | 4.6E2 | 8.6E3 | 4.9E3 | 187 | 12 | 127 | 12 | 0.51 |
| iA | pg/ml | 1.5E2 | 1.4E2 | 3.0E2 | 1.6E2 | 6.1E2 | 1.2E2 | 8.2E0 | 5.8E0 | 7.1E3 | 4.5E2 | 246 | 13 | 160 | 13 | 0.43 |
| iB | ng/ml | 4.8E0 | 5.6E0 | 6.1E0 | 7.4E0 | 5.0E0 | 5.5E0 | 3.3E-2 | 2.3E0 | 2.6E1 | 2.0E1 | 197 | 12 | 131 | 12 | 0.57 |
| iC | U/ml | 2.3E-1 | 2.0E-1 | 1.1E0 | 3.8E-1 | 4.8E0 | 6.5E-1 | 1.0E-9 | 1.0E-9 | 5.5E1 | 2.4E0 | 197 | 12 | 131 | 12 | 0.41 |
| iH | ng/ml | 1.6E5 | 1.8E5 | 1.6E5 | 1.6E5 | 4.8E4 | 5.4E4 | 2.9E3 | 7.2E4 | 2.7E5 | 2.6E5 | 246 | 13 | 160 | 13 | 0.51 |
| iJ | ng/ml | 4.9E4 | 6.0E4 | 5.2E4 | 6.0E4 | 2.6E4 | 1.8E4 | 1.8E3 | 3.4E4 | 2.5E5 | 9.5E4 | 246 | 13 | 160 | 13 | 0.63 |
| hB | ng/ml | 4.5E-1 | 5.0E-1 | 5.6E-1 | 7.5E-1 | 4.5E-1 | 8.6E-1 | 1.0E-9 | 2.2E-1 | 3.2E0 | 3.4E0 | 246 | 13 | 160 | 13 | 0.54 |
| hC | pg/ml | 3.8E3 | 4.2E3 | 6.8E3 | 6.8E3 | 8.7E3 | 6.9E3 | 1.0E-9 | 1.7E2 | 5.7E4 | 2.5E4 | 246 | 13 | 160 | 13 | 0.53 |
| hF | ng/ml | 1.0E-9 | 1.0E-9 | 2.0E1 | 1.0E-9 | 2.6E2 | 0.0E0 | 1.0E-9 | 1.0E-9 | 4.0E3 | 1.0E-9 | 246 | 13 | 160 | 13 | 0.49 |
| hG | pg/ml | 6.8E3 | 7.3E3 | 7.2E3 | 9.5E3 | 3.0E3 | 5.0E3 | 2.8E1 | 3.4E3 | 2.0E4 | 1.9E4 | 246 | 13 | 160 | 13 | 0.63 |
| iO | ng/ml | 3.7E5 | 2.7E5 | 3.9E5 | 3.3E5 | 1.8E5 | 2.0E5 | 1.1E4 | 1.3E5 | 1.1E6 | 9.0E5 | 246 | 13 | 160 | 13 | 0.35 |
| iP | ng/ml | 4.8E4 | 3.7E4 | 5.6E4 | 4.2E4 | 5.6E4 | 3.0E4 | 1.0E-9 | 5.2E3 | 5.7E5 | 1.2E5 | 246 | 13 | 160 | 13 | 0.40 |

| Code | Units | Median | | Average | | Standard Deviation | | Minimum | | Maximum | | Number of Samples | | Number of Patients | | AUC |
|------|-------|--------|--------|---------|--------|--------|--------|--------|--------|--------|--------|--------|--------|--------|--------|------|
| | | NonDis | Dis | NonDis | Dis | NonDis | Dis | NonDis | Dis | NonDis | Dis | NonDis | Dis | NonDis | Dis | |
| iZ | ng/ml | 1.6E3 | 2.2E3 | 1.8E3 | 2.8E3 | 7.4E2 | 1.6E3 | 4.7E2 | 9.8E2 | 5.1E3 | 6.5E3 | 247 | 13 | 160 | 13 | 0.71 |
| rC | pg/ml | 1.7E3 | 1.3E3 | 2.1E3 | 2.7E3 | 1.8E3 | 3.8E3 | 1.0E-9 | 7.3E2 | 1.5E4 | 1.5E4 | 187 | 12 | 127 | 12 | 0.51 |
| rB | pg/ml | 2.6E1 | 2.4E1 | 4.6E1 | 5.2E1 | 8.9E1 | 7.9E1 | 1.0E-9 | 1.0E-9 | 9.5E2 | 2.9E2 | 187 | 12 | 127 | 12 | 0.49 |
| jD | ng/ml | 3.2E1 | 3.7E1 | 4.9E1 | 4.5E1 | 6.3E1 | 4.3E1 | 1.0E-9 | 1.0E-9 | 5.1E2 | 1.4E2 | 196 | 12 | 131 | 12 | 0.50 |
| jE | ng/ml | 1.0E-9 | 1.0E-9 | 6.9E0 | 5.6E0 | 1.6E1 | 1.4E1 | 1.0E-9 | 1.0E-9 | 1.2E2 | 4.6E1 | 196 | 12 | 131 | 12 | 0.50 |
| jF | ng/ml | 3.9E1 | 2.8E1 | 5.2E1 | 4.7E1 | 5.8E1 | 5.8E1 | 1.0E-9 | 1.0E-9 | 3.5E2 | 1.6E2 | 196 | 12 | 131 | 12 | 0.48 |
| jG | ng/ml | 4.2E3 | 6.4E3 | 4.3E3 | 6.0E3 | 1.9E3 | 1.7E3 | 6.0E2 | 3.0E3 | 9.6E3 | 7.9E3 | 197 | 12 | 131 | 12 | 0.76 |
| jH | ng/ml | 7.4E1 | 1.0E2 | 8.4E1 | 1.1E2 | 5.1E1 | 5.1E1 | 1.3E1 | 4.8E1 | 4.3E2 | 2.5E2 | 197 | 12 | 131 | 12 | 0.71 |
| jI | ng/ml | 6.8E1 | 7.5E1 | 7.6E1 | 7.8E1 | 4.1E1 | 3.9E1 | 1.9E1 | 3.5E1 | 4.4E2 | 1.9E2 | 197 | 12 | 131 | 12 | 0.52 |
| rA | pg/ml | 2.6E1 | 3.0E1 | 3.1E1 | 3.1E1 | 2.4E1 | 1.8E1 | 1.0E-9 | 7.5E0 | 2.0E2 | 6.5E1 | 197 | 12 | 131 | 12 | 0.53 |
| qZ | pg/ml | 4.4E1 | 4.0E-3 | 5.4E2 | 3.4E1 | 2.1E3 | 6.1E1 | 1.0E-9 | 5.8E-4 | 1.0E4 | 1.9E2 | 152 | 9 | 115 | 9 | 0.30 |
| qY | pg/ml | 2.6E1 | 3.7E1 | 5.0E1 | 5.8E1 | 6.4E1 | 5.8E1 | 8.7E-1 | 1.1E1 | 5.3E2 | 1.8E2 | 197 | 12 | 131 | 12 | 0.58 |
| qX | pg/ml | 6.0E1 | 7.3E1 | 6.7E1 | 7.4E1 | 4.7E1 | 3.0E1 | 1.0E-9 | 2.3E1 | 2.5E2 | 1.3E2 | 197 | 12 | 131 | 12 | 0.61 |
| qW | pg/ml | 8.8E0 | 1.1E1 | 1.3E1 | 2.2E1 | 1.5E1 | 2.2E1 | 1.0E-9 | 4.0E-1 | 1.2E2 | 8.1E1 | 197 | 12 | 131 | 12 | 0.63 |
| qV | pg/ml | 2.2E3 | 2.7E3 | 2.8E3 | 4.1E3 | 2.1E3 | 4.4E3 | 1.7E2 | 6.1E2 | 9.6E3 | 1.7E4 | 197 | 12 | 131 | 12 | 0.58 |
| qU | pg/ml | 6.4E1 | 4.0E1 | 1.8E2 | 7.1E1 | 2.8E2 | 7.7E1 | 1.0E-9 | 1.0E-9 | 2.1E3 | 2.7E2 | 197 | 12 | 131 | 12 | 0.39 |
| qT | pg/ml | 4.1E1 | 5.0E1 | 7.6E1 | 5.3E1 | 1.1E2 | 2.5E1 | 1.0E-9 | 1.1E1 | 9.0E2 | 8.8E1 | 197 | 12 | 131 | 12 | 0.53 |
| jK | ng/ml | 1.6E3 | 1.6E3 | 1.7E3 | 1.8E3 | 6.6E2 | 8.8E2 | 2.8E2 | 6.4E2 | 4.3E3 | 3.5E3 | 197 | 12 | 131 | 12 | 0.49 |
| jL | ng/ml | 2.0E2 | 2.5E2 | 3.0E2 | 2.8E2 | 3.0E2 | 1.9E2 | 3.5E1 | 4.8E1 | 2.1E3 | 7.9E2 | 197 | 12 | 131 | 12 | 0.56 |
| jM | ng/ml | 7.0E4 | 9.0E4 | 7.5E4 | 8.4E4 | 3.9E4 | 4.5E4 | 3.9E2 | 7.8E3 | 1.9E5 | 1.5E5 | 197 | 12 | 131 | 12 | 0.58 |
| jO | pg/ml | 2.1E5 | 2.3E5 | 2.6E5 | 2.5E5 | 1.5E5 | 1.5E5 | 5.2E4 | 1.1E5 | 1.1E6 | 6.4E5 | 197 | 12 | 131 | 12 | 0.49 |
| jP | pg/ml | 2.3E5 | 1.9E5 | 2.7E5 | 1.7E5 | 1.9E5 | 7.6E4 | 3.6E4 | 5.8E4 | 1.9E6 | 3.4E5 | 197 | 12 | 131 | 12 | 0.31 |
| jQ | pg/ml | 2.5E3 | 3.3E3 | 3.5E3 | 4.0E3 | 3.3E3 | 3.0E3 | 1.0E-9 | 5.5E2 | 1.8E4 | 1.1E4 | 197 | 12 | 131 | 12 | 0.58 |
| jR | pg/ml | 5.9E3 | 8.1E3 | 1.1E4 | 1.0E4 | 1.3E4 | 8.0E3 | 1.0E-9 | 1.3E3 | 9.0E4 | 2.5E4 | 197 | 12 | 131 | 12 | 0.54 |
| jT | pg/ml | 1.7E5 | 1.8E5 | 1.7E5 | 2.0E5 | 6.2E4 | 7.4E4 | 6.8E4 | 1.1E5 | 3.9E5 | 3.3E5 | 197 | 12 | 131 | 12 | 0.60 |
| jU | mIU/ml | 4.3E0 | 3.7E0 | 1.0E1 | 6.5E0 | 1.7E1 | 5.7E0 | 4.2E-2 | 1.9E-1 | 1.1E2 | 1.6E1 | 197 | 12 | 131 | 12 | 0.50 |
| jV | mIU/ml | 1.3E0 | 2.1E0 | 3.3E0 | 4.9E0 | 5.7E0 | 6.8E0 | 1.7E-3 | 6.9E-4 | 3.3E1 | 2.1E1 | 197 | 12 | 131 | 12 | 0.57 |
| jY | ng/ml | 7.3E-4 | 1.5E-3 | 6.3E-3 | 5.3E-3 | 2.8E-2 | 7.8E-3 | 1.0E-9 | 1.0E-9 | 3.0E-1 | 2.7E-2 | 197 | 12 | 131 | 12 | 0.60 |
| kC | pg/ml | 9.7E1 | 1.0E2 | 1.9E2 | 1.7E2 | 4.3E2 | 1.9E2 | 2.1E1 | 5.2E1 | 3.5E3 | 6.8E2 | 133 | 10 | 101 | 10 | 0.51 |
| kE | pg/ml | 1.3E5 | 1.2E5 | 1.3E5 | 1.3E5 | 4.0E4 | 4.0E4 | 1.2E4 | 5.5E4 | 2.3E5 | 1.9E5 | 133 | 10 | 101 | 10 | 0.49 |
| kF | pg/mL | 6.1E1 | 7.3E1 | 6.9E1 | 7.5E1 | 4.9E1 | 2.1E1 | 2.6E1 | 5.0E1 | 5.1E2 | 1.2E2 | 133 | 10 | 101 | 10 | 0.65 |
| kG | pg/mL | 9.1E3 | 8.8E3 | 1.2E4 | 1.6E4 | 1.3E4 | 2.6E4 | 7.5E2 | 4.6E3 | 1.2E5 | 9.1E4 | 133 | 10 | 101 | 10 | 0.50 |
| kI | pg/ml | 1.9E2 | 1.9E2 | 2.2E2 | 1.9E2 | 1.4E2 | 9.0E1 | 4.4E1 | 5.4E1 | 8.7E2 | 3.6E2 | 133 | 10 | 101 | 10 | 0.47 |
| kK | pg/ml | 1.0E2 | 1.0E2 | 1.5E2 | 2.4E2 | 1.6E2 | 2.5E2 | 6.4E0 | 3.2E1 | 1.2E3 | 6.9E2 | 133 | 10 | 101 | 10 | 0.51 |
| kN | pg/ml | 9.9E2 | 6.5E2 | 1.5E3 | 9.0E2 | 2.2E3 | 9.2E2 | 7.6E1 | 1.2E2 | 1.7E4 | 3.3E3 | 133 | 10 | 101 | 10 | 0.34 |
| kO | pg/ml | 7.1E3 | 7.8E3 | 9.6E3 | 9.1E3 | 1.7E4 | 4.4E3 | 3.4E3 | 5.2E3 | 1.5E5 | 1.8E4 | 133 | 10 | 101 | 10 | 0.58 |
| kP | pg/ml | 5.8E3 | 5.8E3 | 7.2E3 | 6.2E3 | 6.2E3 | 2.7E3 | 8.6E2 | 2.3E3 | 4.8E4 | 1.2E4 | 133 | 10 | 101 | 10 | 0.49 |
| kQ | pg/ml | 4.1E3 | 4.5E3 | 5.2E3 | 4.9E3 | 3.8E3 | 2.4E3 | 5.6E2 | 1.3E3 | 2.5E4 | 1.0E4 | 246 | 13 | 160 | 13 | 0.53 |
| kR | pg/ml | 2.1E1 | 1.7E1 | 3.0E1 | 2.0E1 | 6.8E1 | 1.3E1 | 1.0E-9 | 1.8E0 | 1.0E3 | 5.7E1 | 246 | 13 | 160 | 13 | 0.42 |
| kS | pg/ml | 7.8E2 | 1.1E3 | 9.2E2 | 2.3E3 | 6.5E2 | 3.7E3 | 7.9E1 | 3.4E2 | 4.8E3 | 1.4E4 | 246 | 13 | 160 | 13 | 0.71 |
| rZ | ng/ml | 1.0E-9 | 1.0E-9 | 5.8E-3 | 2.6E-2 | 1.5E-2 | 7.8E-2 | 1.0E-9 | 1.0E-9 | 1.1E-1 | 2.6E-1 | 187 | 11 | 127 | 11 | 0.44 |
| rY | ng/ml | 6.1E-2 | 4.1E-2 | 1.4E-1 | 1.6E0 | 5.3E-1 | 5.0E0 | 1.0E-9 | 1.0E-9 | 6.3E0 | 1.7E1 | 187 | 11 | 127 | 11 | 0.42 |
| rX | ng/ml | 1.0E-9 | 1.0E-9 | 4.0E-2 | 1.7E-1 | 2.9E-1 | 5.6E-1 | 1.0E-9 | 1.0E-9 | 3.7E0 | 1.9E0 | 187 | 11 | 127 | 11 | 0.53 |
| lK | pg/ml | 7.9E1 | 1.3E2 | 1.7E2 | 1.3E2 | 3.1E2 | 1.3E2 | 1.0E-9 | 1.0E-9 | 3.3E3 | 3.3E2 | 196 | 12 | 131 | 12 | 0.49 |
| lL | pg/ml | 1.5E3 | 3.3E3 | 2.1E3 | 3.6E3 | 2.3E3 | 2.0E3 | 1.5E1 | 8.7E2 | 1.9E4 | 7.2E3 | 197 | 12 | 131 | 12 | 0.76 |
| lM | pg/ml | 1.1E3 | 1.4E3 | 3.6E3 | 4.0E3 | 7.0E3 | 9.4E3 | 1.3E2 | 3.5E2 | 4.4E4 | 3.4E4 | 197 | 12 | 131 | 12 | 0.53 |
| lN | pg/ml | 1.0E-9 | 1.0E-9 | 4.3E0 | 3.0E0 | 1.5E1 | 4.7E0 | 1.0E-9 | 1.0E-9 | 1.5E2 | 1.2E1 | 197 | 12 | 131 | 12 | 0.52 |
| lO | pg/ml | 1.0E-9 | 1.0E-9 | 1.8E0 | 7.0E0 | 1.4E1 | 2.4E1 | 1.0E-9 | 1.0E-9 | 1.4E2 | 8.4E1 | 196 | 12 | 131 | 12 | 0.53 |
| nW | pg/ml | 1.1E5 | 1.1E5 | 1.1E5 | 1.1E5 | 2.6E4 | 3.6E4 | 3.6E4 | 5.9E4 | 2.0E5 | 1.9E5 | 246 | 13 | 160 | 13 | 0.52 |
| nY | pg/ml | 2.0E3 | 1.9E3 | 2.3E3 | 3.3E3 | 1.3E3 | 3.3E3 | 5.1E2 | 1.0E3 | 9.9E3 | 1.3E4 | 246 | 13 | 160 | 13 | 0.54 |
| oO | pg/ml | 8.7E4 | 1.3E5 | 1.2E5 | 1.5E5 | 9.5E4 | 9.1E4 | 3.3E3 | 3.5E4 | 6.2E5 | 3.3E5 | 123 | 10 | 96 | 10 | 0.63 |
| oP | pg/ml | 1.2E5 | 1.7E5 | 1.4E5 | 2.1E5 | 8.2E4 | 1.1E5 | 2.4E4 | 1.0E5 | 4.5E5 | 4.2E5 | 123 | 10 | 96 | 10 | 0.71 |
| oQ | pg/ml | 2.9E3 | 4.1E3 | 3.8E3 | 4.3E3 | 3.0E3 | 1.6E3 | 7.7E2 | 2.1E3 | 2.0E4 | 6.7E3 | 123 | 10 | 96 | 10 | 0.66 |

| Code | Units | Median | | Average | | Standard Deviation | | Minimum | | Maximum | | Number of Samples | | Number of Patients | | AUC |
|------|-------|--------|-----|---------|-----|-----|-----|--------|------|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| oE | pg/ml | 1.3E2 | 3.4E2 | 3.9E2 | 5.0E2 | 5.8E2 | 5.1E2 | 1.0E-9 | 2.8E0 | 4.7E3 | 1.8E3 | 246 | 13 | 160 | 13 | 0.62 |
| oF | pg/ml | 8.0E3 | 1.3E4 | 2.1E4 | 4.2E4 | 3.3E4 | 7.3E4 | 6.4E1 | 6.9E2 | 1.7E5 | 2.3E5 | 246 | 13 | 160 | 13 | 0.57 |
| oH | pg/ml | 4.0E1 | 9.3E1 | 9.3E1 | 1.1E2 | 1.5E2 | 8.5E1 | 4.3E-1 | 1.9E1 | 9.9E2 | 3.2E2 | 246 | 13 | 160 | 13 | 0.69 |
| oK | pg/ml | 8.5E2 | 6.6E2 | 2.0E3 | 1.1E3 | 3.0E3 | 8.3E2 | 5.2E1 | 1.4E2 | 2.5E4 | 2.4E3 | 246 | 13 | 160 | 13 | 0.49 |
| oN | pg/ml | 5.1E2 | 5.6E2 | 7.6E2 | 5.2E2 | 1.4E3 | 2.2E2 | 1.1E2 | 2.0E2 | 1.8E4 | 8.0E2 | 246 | 13 | 160 | 13 | 0.48 |
| pF | pg/ml | 5.1E-1 | 5.0E-1 | 1.1E0 | 1.4E0 | 5.6E0 | 2.6E0 | 1.0E-9 | 3.1E-1 | 8.7E1 | 1.0E1 | 246 | 13 | 160 | 13 | 0.59 |

Figure 14.

Descriptive statistics for the assays used to form the panels presented in this figure.

| Code | Units | Median | | Average | | Standard Deviation | | Minimum | | Maximum | | Number of Samples | | Number of Patients | | AUC |
|------|-------|--------|------|---------|------|------|------|--------|------|--------|------|--------|------|--------|------|------|
| | | NonDis | Dis | NonDis | Dis | NonDis | Dis | NonDis | Dis | NonDis | Dis | NonDis | Dis | NonDis | Dis | |
| Aa | mg/dL | 6.2E1 | 7.6E1 | 7.3E1 | 9.2E1 | 5.0E1 | 5.8E1 | 2.0E0 | 1.0E1 | 2.9E2 | 2.7E2 | 936 | 59 | 159 | 59 | 0.61 |
| Ad | ug/mL | 2.4E-2 | 1.1E-1 | 5.2E-2 | 1.2E-1 | 7.0E-2 | 1.0E-1 | 6.8E-4 | 3.3E-3 | 3.7E-1 | 3.6E-1 | 218 | 46 | 87 | 46 | 0.74 |
| Af | ng/mL | 8.9E-1 | 1.3E0 | 1.6E1 | 3.0E1 | 7.0E1 | 8.0E1 | 1.7E-3 | 1.7E-3 | 5.3E2 | 4.0E2 | 218 | 46 | 87 | 46 | 0.58 |
| Aj | ug/mL | 1.4E0 | 4.6E0 | 2.6E0 | 3.6E0 | 2.4E0 | 2.4E0 | 1.5E-3 | 4.0E-3 | 6.1E0 | 6.1E0 | 218 | 46 | 87 | 46 | 0.60 |
| Al | mg/mL | 8.8E-5 | 1.2E-4 | 2.5E-4 | 2.7E-4 | 4.2E-4 | 4.1E-4 | 2.5E-6 | 9.0E-6 | 1.9E-3 | 1.9E-3 | 218 | 46 | 87 | 46 | 0.56 |
| An | U/mL | 4.1E1 | 6.9E1 | 1.8E2 | 2.3E2 | 5.5E2 | 4.9E2 | 9.8E-4 | 7.7E-1 | 5.5E3 | 3.0E3 | 218 | 46 | 87 | 46 | 0.61 |
| Ao | pg/mL | 8.1E1 | 1.1E2 | 2.3E2 | 2.3E2 | 1.1E3 | 5.5E2 | 2.8E0 | 1.2E1 | 1.6E4 | 3.8E3 | 218 | 46 | 87 | 46 | 0.61 |
| Ap | ng/mL | 2.6E1 | 5.8E1 | 3.6E1 | 6.7E1 | 3.2E1 | 5.0E1 | 9.9E-1 | 8.4E-5 | 1.7E2 | 2.5E2 | 218 | 46 | 87 | 46 | 0.73 |
| Ar | ng/mL | 6.3E-1 | 1.5E0 | 2.3E0 | 5.0E0 | 4.7E0 | 7.6E0 | 3.4E-3 | 3.4E-3 | 4.3E1 | 2.9E1 | 218 | 46 | 87 | 46 | 0.64 |
| As | ng/mL | 8.6E-3 | 9.8E-3 | 1.3E-2 | 1.5E-2 | 1.7E-2 | 2.2E-2 | 1.7E-3 | 1.7E-3 | 1.1E-1 | 1.2E-1 | 218 | 46 | 87 | 46 | 0.51 |
| Aw | pg/mL | 1.5E1 | 1.8E1 | 1.6E1 | 1.8E1 | 4.9E0 | 6.0E0 | 5.0E0 | 9.4E0 | 3.2E1 | 3.8E1 | 218 | 46 | 87 | 46 | 0.64 |
| Ax | ng/mL | 2.2E0 | 3.3E0 | 9.2E0 | 1.7E1 | 1.9E1 | 3.6E1 | 1.9E-2 | 4.9E-2 | 1.5E2 | 2.0E2 | 218 | 46 | 87 | 46 | 0.57 |
| Ba | ng/mL | 4.2E1 | 2.1E2 | 3.5E2 | 6.2E2 | 9.5E2 | 1.0E3 | 3.7E-1 | 5.5E0 | 8.1E3 | 4.5E3 | 218 | 46 | 87 | 46 | 0.69 |
| Bb | ng/mL | 2.3E0 | 5.4E0 | 4.8E0 | 6.6E0 | 8.2E0 | 5.2E0 | 4.1E-3 | 4.1E-1 | 6.6E1 | 2.0E1 | 218 | 46 | 87 | 46 | 0.67 |
| Bc | ng/mL | 3.2E1 | 5.0E1 | 9.6E1 | 1.2E2 | 1.8E2 | 2.0E2 | 1.1E-1 | 2.2E-1 | 1.0E3 | 1.0E3 | 218 | 46 | 87 | 46 | 0.59 |
| Bg | ng/mL | 7.1E-2 | 4.0E-1 | 3.4E0 | 1.5E0 | 2.1E1 | 2.7E0 | 5.3E-4 | 5.3E-4 | 2.5E2 | 1.3E1 | 218 | 46 | 87 | 46 | 0.67 |
| Bn | ng/mL | 5.6E-2 | 9.7E-2 | 8.6E-1 | 2.0E0 | 1.6E0 | 2.6E0 | 5.6E-2 | 5.6E-2 | 9.7E0 | 7.6E0 | 218 | 46 | 87 | 46 | 0.59 |
| Bo | ng/mL | 1.1E1 | 1.6E1 | 1.2E1 | 1.7E1 | 8.8E0 | 1.4E1 | 1.6E-2 | 1.6E-2 | 4.4E1 | 4.6E1 | 218 | 46 | 87 | 46 | 0.58 |
| Ch | uIU/mL | 1.1E0 | 2.3E0 | 7.6E0 | 2.0E1 | 2.3E1 | 4.2E1 | 3.4E-3 | 1.0E-1 | 2.1E2 | 1.9E2 | 218 | 46 | 87 | 46 | 0.62 |
| Co | pg/mL | 3.0E1 | 5.8E1 | 8.0E1 | 1.3E2 | 2.0E2 | 3.1E2 | 3.9E0 | 6.3E0 | 1.9E3 | 2.1E3 | 218 | 46 | 87 | 46 | 0.64 |
| Cp | ng/mL | 2.0E1 | 2.8E1 | 2.4E1 | 3.6E1 | 2.5E1 | 2.9E1 | 6.0E-1 | 6.0E-1 | 2.9E2 | 1.9E2 | 218 | 46 | 87 | 46 | 0.70 |
| Cq | ng/mL | 2.4E-2 | 3.7E-2 | 1.7E-1 | 6.0E-2 | 1.2E0 | 7.6E-2 | 8.0E-4 | 8.0E-4 | 1.7E1 | 4.0E-1 | 218 | 46 | 87 | 46 | 0.60 |
| Cs | ng/mL | 6.0E1 | 8.5E1 | 2.4E2 | 3.8E2 | 4.4E2 | 9.0E2 | 3.9E-1 | 1.3E0 | 2.9E3 | 5.3E3 | 218 | 46 | 87 | 46 | 0.55 |
| Ct | ng/mL | 1.2E0 | 9.7E-1 | 3.1E1 | 3.0E1 | 9.4E1 | 7.7E1 | 6.2E-3 | 2.7E-2 | 6.2E2 | 4.7E2 | 218 | 46 | 87 | 46 | 0.50 |
| Cu | ng/mL | 2.1E-1 | 3.4E-1 | 4.2E-1 | 4.0E-1 | 8.2E-1 | 2.3E-1 | 9.6E-3 | 8.1E-2 | 9.2E0 | 9.4E-1 | 218 | 46 | 87 | 46 | 0.65 |
| Cv | ng/mL | 3.9E0 | 1.1E1 | 1.6E1 | 3.5E1 | 5.3E1 | 6.5E1 | 5.6E-3 | 2.6E-2 | 5.3E2 | 3.1E2 | 218 | 46 | 87 | 46 | 0.62 |
| Cw | mIU/mL | 2.7E-2 | 3.8E-2 | 3.4E-2 | 4.6E-2 | 2.4E-2 | 3.1E-2 | 2.3E-3 | 7.9E-3 | 1.4E-1 | 1.4E-1 | 218 | 46 | 87 | 46 | 0.62 |
| Cx | ng/mL | 1.7E-1 | 4.2E-1 | 5.1E1 | 9.1E1 | 1.0E2 | 1.3E2 | 9.3E-5 | 9.3E-5 | 3.9E2 | 3.9E2 | 218 | 46 | 87 | 46 | 0.56 |
| Db | ug/mL | 7.3E0 | 6.7E0 | 8.3E0 | 7.8E0 | 5.7E0 | 7.2E0 | 5.0E-1 | 8.3E-1 | 3.9E1 | 3.6E1 | 218 | 46 | 87 | 46 | 0.46 |
| Dc | nmol/L | 1.8E-2 | 3.3E-2 | 5.7E-2 | 6.0E-2 | 1.4E-1 | 8.6E-2 | 5.2E-6 | 3.0E-4 | 1.2E0 | 4.0E-1 | 218 | 46 | 87 | 46 | 0.57 |
| Dd | ug/mL | 6.8E-2 | 5.6E-2 | 1.7E-1 | 2.4E-1 | 2.6E-1 | 3.9E-1 | 1.9E-4 | 1.1E-3 | 1.6E0 | 1.9E0 | 218 | 46 | 87 | 46 | 0.51 |
| De | ng/mL | 3.4E-3 | 3.4E-3 | 6.0E-2 | 1.0E-1 | 1.1E-1 | 1.4E-1 | 3.4E-3 | 3.4E-3 | 5.9E-1 | 6.2E-1 | 218 | 46 | 87 | 46 | 0.58 |
| Dg | ng/mL | 2.5E1 | 6.9E1 | 3.7E1 | 7.1E1 | 3.4E1 | 4.8E1 | 2.4E-1 | 1.0E-1 | 1.9E2 | 1.9E2 | 218 | 46 | 87 | 46 | 0.73 |
| Di | pg/mL | 1.7E0 | 2.1E0 | 2.0E0 | 2.4E0 | 1.8E0 | 2.3E0 | 1.8E-1 | 1.8E-1 | 1.3E1 | 7.8E0 | 218 | 46 | 87 | 46 | 0.54 |
| Dk | uIU/mL | 1.5E-2 | 2.6E-2 | 1.0E-1 | 4.4E-2 | 6.8E-1 | 6.2E-2 | 1.1E-4 | 1.1E-4 | 8.9E0 | 3.4E-1 | 218 | 46 | 87 | 46 | 0.63 |
| Dl | ng/mL | 2.1E2 | 4.1E2 | 2.7E2 | 5.1E2 | 2.4E2 | 3.7E2 | 1.7E0 | 2.5E0 | 1.4E3 | 1.3E3 | 218 | 46 | 87 | 46 | 0.70 |
| Do | ng/ml | 5.9E-1 | 6.3E-1 | 1.3E0 | 8.9E-1 | 3.2E0 | 1.0E0 | 3.6E-2 | 3.6E-2 | 1.9E1 | 3.7E0 | 42 | 11 | 28 | 11 | 0.53 |
| Dp | ng/ml | 2.4E0 | 2.5E0 | 4.5E0 | 5.1E0 | 6.6E0 | 6.7E0 | 3.7E-3 | 3.7E-3 | 4.3E1 | 3.5E1 | 111 | 44 | 86 | 44 | 0.52 |
| Dr | pg/ml | 2.2E1 | 1.3E1 | 4.6E1 | 3.2E1 | 6.4E1 | 4.4E1 | 7.5E-1 | 7.5E-1 | 2.9E2 | 1.6E2 | 78 | 22 | 39 | 22 | 0.45 |
| Dq | Absorbance | 1.7E-3 | 1.7E-3 | 4.4E-2 | 1.7E-3 | 1.7E-1 | 0.0E0 | 1.7E-3 | 1.7E-3 | 8.3E-1 | 1.7E-3 | 42 | 11 | 28 | 11 | 0.38 |
| Du | pg/ml | 2.7E1 | 1.2E0 | 5.5E2 | 1.9E3 | 1.3E3 | 6.8E3 | 1.2E0 | 1.2E0 | 7.0E3 | 2.6E4 | 45 | 15 | 35 | 15 | 0.42 |
| Dv | pg/ml | 1.0E0 | 1.3E0 | 1.2E0 | 1.1E0 | 1.3E0 | 9.1E-1 | 2.2E-2 | 2.2E-2 | 4.5E0 | 2.3E0 | 33 | 7 | 18 | 7 | 0.54 |
| Ef | ng/ml | 9.5E-2 | 4.4E-1 | 5.6E-1 | 1.2E0 | 1.3E0 | 2.0E0 | 5.7E-4 | 1.3E-3 | 9.4E0 | 8.6E0 | 143 | 45 | 86 | 45 | 0.67 |
| Wm | % | 7.0E-1 | 6.2E-1 | 3.4E1 | 6.1E1 | 2.1E2 | 2.3E2 | 8.5E-2 | 8.5E-2 | 2.4E3 | 9.6E2 | 173 | 47 | 101 | 47 | 0.53 |
| Ed | pg/ml | 7.1E0 | 5.2E-1 | 1.0E2 | 2.8E1 | 6.9E2 | 6.8E1 | 5.2E-1 | 5.2E-1 | 7.3E3 | 3.5E2 | 111 | 44 | 85 | 44 | 0.38 |
| Yf | ng/mL | 1.7E1 | 1.4E1 | 1.6E2 | 2.8E1 | 9.1E2 | 3.2E1 | 2.9E-1 | 2.2E0 | 6.6E3 | 1.2E2 | 52 | 15 | 41 | 15 | 0.49 |
| Tj | pg/mL | 3.7E-1 | 3.7E-1 | 8.9E1 | 8.4E0 | 4.0E2 | 2.4E1 | 3.6E-1 | 3.6E-1 | 3.5E3 | 1.5E2 | 141 | 46 | 86 | 46 | 0.41 |
| Po | pg/ml | 2.6E-1 | 2.6E0 | 7.7E0 | 1.2E1 | 2.5E1 | 3.0E1 | 8.0E-3 | 2.6E-2 | 2.7E2 | 2.2E2 | 316 | 65 | 135 | 65 | 0.62 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ti | ug/mL | 2.5E0 | 5.9E0 | 3.7E0 | 6.3E0 | 3.4E0 | 5.1E0 | 1.2E-1 | 1.9E-1 | 1.5E1 | 1.7E1 | 77 | 26 | 61 | 26 | 0.65 |
| Em | ng/ml | 2.9E-3 | 2.9E-3 | 6.8E-2 | 4.4E-2 | 1.2E-1 | 9.9E-2 | 2.8E-16 | 1.9E-16 | 5.4E-1 | 4.7E-1 | 93 | 23 | 40 | 23 | 0.45 |
| Et | ng/ml | 1.1E3 | 2.5E3 | 1.4E3 | 2.5E3 | 1.0E3 | 1.2E3 | 7.7E1 | 1.8E2 | 4.2E3 | 5.0E3 | 316 | 65 | 135 | 65 | 0.75 |
| Eq | pg/ml | 1.3E2 | 2.5E2 | 3.0E2 | 3.7E2 | 3.9E2 | 4.8E2 | 1.0E0 | 1.0E0 | 1.8E3 | 1.8E3 | 45 | 15 | 35 | 15 | 0.54 |
| Th | ug/mL | 1.1E0 | 1.4E0 | 1.6E0 | 1.6E0 | 1.7E0 | 9.7E-1 | 2.6E-3 | 5.6E-1 | 1.2E1 | 4.6E0 | 77 | 26 | 61 | 26 | 0.59 |
| Fa | ng/ml | 4.0E1 | 5.2E1 | 1.4E2 | 9.9E1 | 7.9E2 | 1.4E2 | 3.4E-2 | 2.6E-1 | 8.0E3 | 7.5E2 | 109 | 44 | 86 | 44 | 0.60 |
| Ez | ng/ml | 5.0E0 | 9.2E0 | 2.4E1 | 2.3E1 | 7.6E1 | 4.5E1 | 1.3E-2 | 1.3E-2 | 7.1E2 | 2.4E2 | 111 | 44 | 86 | 44 | 0.57 |
| Fb | ng/ml | 2.3E1 | 2.7E1 | 2.2E1 | 2.5E1 | 1.2E1 | 8.7E0 | 1.0E0 | 5.9E-1 | 5.7E1 | 4.0E1 | 109 | 44 | 86 | 44 | 0.59 |
| Ex | ng/ml | 6.2E-2 | 1.1E-1 | 2.6E-1 | 1.8E-1 | 9.1E-1 | 2.1E-1 | 3.5E-5 | 1.7E-4 | 8.9E0 | 9.2E-1 | 102 | 31 | 53 | 31 | 0.61 |
| Fc | pg/ml | 2.2E-1 | 2.2E-1 | 3.3E2 | 2.2E-1 | 2.2E3 | 0.0E0 | 2.2E-1 | 2.2E-1 | 1.5E4 | 2.2E-1 | 45 | 15 | 35 | 15 | 0.37 |
| Fd | pg/ml | 9.8E-1 | 9.8E-1 | 1.0E3 | 8.1E2 | 4.9E3 | 2.1E3 | 4.5E-1 | 9.8E-1 | 3.3E4 | 8.2E3 | 45 | 15 | 35 | 15 | 0.49 |
| Fi | pg/ml | 2.5E-1 | 2.5E-1 | 3.5E2 | 6.9E0 | 2.1E3 | 2.6E1 | 2.5E-1 | 2.5E-1 | 1.4E4 | 1.0E2 | 45 | 15 | 35 | 15 | 0.42 |
| Fn | ng/ml | 2.1E-1 | 2.1E-1 | 9.2E0 | 3.6E0 | 4.0E1 | 5.7E0 | 1.1E-14 | 1.1E-14 | 4.2E2 | 2.1E1 | 111 | 44 | 86 | 44 | 0.46 |
| Fp | ng/ml | 1.0E1 | 3.0E1 | 2.1E1 | 4.1E1 | 2.7E1 | 3.7E1 | 6.0E-3 | 6.6E-1 | 1.4E2 | 1.4E2 | 333 | 66 | 136 | 66 | 0.68 |
| Fr | ng/ml | 2.5E4 | 7.1E4 | 9.7E4 | 1.7E5 | 1.7E5 | 2.2E5 | 6.4E2 | 2.1E3 | 8.4E5 | 8.5E5 | 398 | 69 | 137 | 69 | 0.68 |
| Fw | pg/ml | 8.5E-1 | 1.2E-1 | 1.1E2 | 2.5E1 | 7.2E2 | 9.9E1 | 1.1E-14 | 1.7E-14 | 6.9E3 | 6.3E2 | 144 | 45 | 87 | 45 | 0.46 |
| Fy | ng/ml | 3.1E1 | 4.9E1 | 4.9E1 | 7.4E1 | 5.5E1 | 6.4E1 | 1.8E-1 | 1.2E-1 | 3.3E2 | 2.1E2 | 110 | 44 | 85 | 44 | 0.63 |
| Gh | pg/ml | 2.3E0 | 8.4E0 | 6.2E1 | 1.3E2 | 2.7E2 | 3.3E2 | 2.9E-2 | 2.9E-2 | 1.8E3 | 1.2E3 | 45 | 14 | 35 | 14 | 0.51 |
| Gb | % | 3.3E1 | 4.0E1 | 4.1E1 | 5.0E1 | 3.6E1 | 3.3E1 | 3.1E0 | 5.9E0 | 1.9E2 | 1.0E2 | 45 | 15 | 34 | 15 | 0.59 |
| Gc | ng/ml | 8.4E1 | 1.6E2 | 1.1E2 | 2.1E2 | 1.1E2 | 2.1E2 | 6.4E0 | 1.8E1 | 7.3E2 | 9.2E2 | 82 | 23 | 40 | 23 | 0.67 |
| Gd | ng/ml | 3.1E1 | 2.9E1 | 3.1E1 | 3.4E1 | 1.5E1 | 2.2E1 | 5.4E0 | 6.3E0 | 6.9E1 | 8.1E1 | 95 | 20 | 40 | 20 | 0.51 |
| Gn | U/ml | 5.1E-1 | 1.0E-1 | 1.8E0 | 8.1E-1 | 4.1E0 | 2.5E0 | 1.3E-3 | 1.3E-3 | 3.0E1 | 1.2E1 | 74 | 22 | 39 | 22 | 0.30 |
| Gl | pg/ml | 6.0E3 | 1.0E4 | 9.8E3 | 1.2E4 | 9.0E3 | 8.7E3 | 2.3E2 | 1.0E3 | 3.2E4 | 3.3E4 | 140 | 45 | 87 | 45 | 0.62 |
| Gp | U/ml | 1.8E0 | 1.1E0 | 4.4E0 | 3.9E0 | 8.0E0 | 8.2E0 | 1.3E-3 | 1.3E-3 | 6.7E1 | 4.8E1 | 144 | 45 | 87 | 45 | 0.44 |
| Gt | ng/ml | 2.2E-3 | 2.2E-3 | 7.7E-2 | 5.8E-3 | 2.1E-1 | 1.0E-2 | 2.2E-3 | 2.2E-3 | 1.1E0 | 3.1E-2 | 36 | 8 | 22 | 8 | 0.40 |
| Gw | ng/ml | 3.9E0 | 7.8E0 | 8.5E0 | 8.6E0 | 1.5E1 | 6.4E0 | 8.3E-1 | 8.3E-1 | 9.3E1 | 2.2E1 | 42 | 11 | 28 | 11 | 0.61 |
| Gz | ug/ml | 1.2E0 | 1.0E0 | 1.2E1 | 5.0E0 | 5.8E1 | 6.3E0 | 2.9E-16 | 1.3E-1 | 4.8E2 | 2.1E1 | 69 | 29 | 52 | 29 | 0.51 |
| Ha | ng/ml | 2.7E0 | 2.8E0 | 9.0E0 | 6.7E0 | 2.0E1 | 1.3E1 | 1.7E-2 | 1.7E-2 | 1.0E2 | 6.7E1 | 109 | 44 | 85 | 44 | 0.49 |
| Nm | pg/ml | 1.4E4 | 3.1E4 | 2.6E4 | 4.6E4 | 5.1E4 | 4.6E4 | 1.0E-9 | 1.0E-9 | 7.8E5 | 1.9E5 | 315 | 65 | 136 | 65 | 0.66 |
| Nn | pg/ml | 1.2E2 | 2.4E2 | 2.7E3 | 2.6E3 | 1.1E4 | 1.0E4 | 1.0E-9 | 1.0E-9 | 1.0E5 | 6.9E4 | 315 | 65 | 136 | 65 | 0.60 |
| No | pg/ml | 1.4E1 | 2.9E1 | 2.7E1 | 5.6E1 | 5.7E1 | 1.8E2 | 1.0E-9 | 3.2E-1 | 5.9E2 | 1.4E3 | 315 | 65 | 136 | 65 | 0.62 |
| Nq | pg/ml | 2.8E0 | 1.7E0 | 2.2E1 | 1.7E1 | 8.9E1 | 4.4E1 | 1.0E-9 | 1.0E-9 | 1.2E3 | 2.6E2 | 315 | 65 | 136 | 65 | 0.48 |
| Nr | pg/ml | 6.0E-1 | 2.5E0 | 1.4E1 | 1.5E2 | 6.9E1 | 1.1E3 | 1.0E-9 | 1.0E-9 | 9.8E2 | 8.5E3 | 315 | 65 | 136 | 65 | 0.63 |
| Ns | pg/ml | 1.0E-9 | 1.0E-9 | 1.3E1 | 3.2E0 | 7.8E1 | 1.6E1 | 1.0E-9 | 1.0E-9 | 1.1E3 | 1.0E2 | 315 | 65 | 136 | 65 | 0.49 |
| Nt | pg/ml | 9.9E1 | 1.6E2 | 1.4E2 | 1.8E2 | 1.3E2 | 1.1E2 | 1.5E1 | 4.7E1 | 1.5E3 | 6.8E2 | 315 | 65 | 136 | 65 | 0.68 |
| Nu | pg/ml | 2.0E1 | 4.4E1 | 5.2E1 | 8.6E1 | 8.8E1 | 1.1E2 | 1.0E-9 | 1.0E-9 | 8.9E2 | 5.8E2 | 315 | 65 | 136 | 65 | 0.62 |
| Lu | pg/ml | 1.0E4 | 1.0E4 | 1.6E4 | 1.2E4 | 4.2E4 | 1.5E4 | 6.7E2 | 7.1E2 | 5.6E5 | 9.6E4 | 317 | 66 | 136 | 66 | 0.50 |
| Lv | pg/ml | 1.0E-9 | 6.8E0 | 1.1E1 | 2.1E1 | 2.2E1 | 3.2E1 | 1.0E-9 | 1.0E-9 | 2.4E2 | 1.6E2 | 317 | 66 | 136 | 66 | 0.59 |
| Lw | pg/ml | 1.0E-9 | 1.0E-9 | 1.3E-1 | 4.8E-1 | 1.8E0 | 2.7E0 | 1.0E-9 | 1.0E-9 | 3.2E1 | 1.7E1 | 317 | 66 | 136 | 66 | 0.52 |
| Lx | pg/ml | 1.0E-9 | 5.5E1 | 1.2E2 | 3.1E2 | 4.3E2 | 1.2E3 | 1.0E-9 | 1.0E-9 | 6.2E3 | 1.0E4 | 317 | 66 | 136 | 66 | 0.64 |
| Ly | pg/ml | 1.0E-9 | 1.0E-9 | 9.2E0 | 2.1E1 | 1.8E1 | 3.2E1 | 1.0E-9 | 1.0E-9 | 1.2E2 | 1.6E2 | 317 | 66 | 136 | 66 | 0.58 |
| Lz | pg/ml | 1.0E-9 | 1.0E-9 | 2.5E0 | 6.5E0 | 2.5E1 | 3.5E1 | 1.0E-9 | 1.0E-9 | 4.3E2 | 2.1E2 | 317 | 66 | 136 | 66 | 0.51 |
| Ma | pg/ml | 2.7E2 | 4.7E2 | 1.4E3 | 1.3E3 | 4.6E3 | 2.0E3 | 1.0E-9 | 1.4E1 | 6.5E4 | 9.5E3 | 317 | 66 | 136 | 66 | 0.59 |
| Mb | pg/ml | 2.5E1 | 3.0E1 | 3.0E1 | 3.8E1 | 1.3E1 | 1.8E1 | 5.4E0 | 1.4E1 | 6.9E1 | 8.7E1 | 317 | 66 | 136 | 66 | 0.62 |
| Mc | pg/ml | 1.0E-9 | 1.0E-9 | 2.0E-2 | 1.0E-9 | 2.5E-1 | 0.0E0 | 1.0E-9 | 1.0E-9 | 4.0E0 | 1.0E-9 | 317 | 66 | 136 | 66 | 0.50 |
| Md | pg/ml | 1.0E-9 | 1.0E-9 | 5.0E-1 | 8.7E-1 | 4.0E0 | 3.2E0 | 1.0E-9 | 1.0E-9 | 6.5E1 | 1.6E1 | 317 | 66 | 136 | 66 | 0.51 |
| Me | pg/ml | 3.2E1 | 2.5E1 | 2.9E1 | 2.5E1 | 1.6E1 | 1.7E1 | 1.0E-9 | 6.1E-1 | 1.2E2 | 7.9E1 | 317 | 66 | 136 | 66 | 0.41 |
| Mf | pg/ml | 1.0E-9 | 1.0E-9 | 3.8E-1 | 6.5E-1 | 1.7E0 | 1.8E0 | 1.0E-9 | 1.0E-9 | 2.0E1 | 8.4E0 | 317 | 66 | 136 | 66 | 0.56 |
| Mg | pg/ml | 2.2E0 | 7.8E0 | 7.3E0 | 1.2E1 | 1.2E1 | 1.4E1 | 1.0E-9 | 1.0E-9 | 8.8E1 | 5.9E1 | 317 | 66 | 136 | 66 | 0.62 |
| Mh | pg/ml | 1.0E-9 | 1.0E-9 | 9.0E-1 | 1.7E0 | 8.0E0 | 6.1E0 | 1.0E-9 | 1.0E-9 | 1.1E2 | 3.8E1 | 317 | 66 | 136 | 66 | 0.51 |
| Mi | pg/ml | 1.0E-9 | 1.0E-9 | 7.8E-1 | 3.6E0 | 7.5E0 | 2.1E1 | 1.0E-9 | 1.0E-9 | 1.2E2 | 1.6E2 | 317 | 66 | 136 | 66 | 0.51 |
| Mj | pg/ml | 1.0E-9 | 1.0E-9 | 4.6E0 | 1.1E1 | 2.8E1 | 6.8E1 | 1.0E-9 | 1.0E-9 | 4.6E2 | 5.4E2 | 317 | 66 | 136 | 66 | 0.54 |
| Mk | pg/ml | 1.0E-9 | 4.8E0 | 1.7E1 | 9.4E1 | 1.1E2 | 6.8E2 | 1.0E-9 | 1.0E-9 | 1.7E3 | 5.6E3 | 317 | 66 | 136 | 66 | 0.55 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ml | pg/ml | 1.0E-9 | 1.0E-9 | 9.5E0 | 1.3E0 | 1.2E2 | 3.3E0 | 1.0E-9 | 1.0E-9 | 2.1E3 | 1.6E1 | 317 | 66 | 136 | 66 | 0.55 |
| Mm | pg/ml | 4.6E2 | 9.2E2 | 8.4E2 | 1.5E3 | 9.7E2 | 1.6E3 | 1.0E-9 | 1.3E1 | 6.0E3 | 6.9E3 | 317 | 66 | 136 | 66 | 0.65 |
| Mn | pg/ml | 4.9E0 | 6.7E0 | 1.0E1 | 1.2E1 | 2.6E1 | 1.9E1 | 1.0E-9 | 1.0E-9 | 3.5E2 | 1.3E2 | 317 | 66 | 136 | 66 | 0.57 |
| Mp | pg/ml | 1.0E-9 | 1.0E-9 | 1.0E1 | 1.0E1 | 2.3E1 | 2.7E1 | 1.0E-9 | 1.0E-9 | 1.8E2 | 1.8E2 | 317 | 66 | 136 | 66 | 0.51 |
| Mq | pg/ml | 1.0E-9 | 1.0E-9 | 2.9E0 | 5.3E0 | 1.8E1 | 1.9E1 | 1.0E-9 | 1.0E-9 | 2.1E2 | 1.2E2 | 317 | 66 | 136 | 66 | 0.53 |
| Mr | pg/ml | 1.0E-9 | 1.0E-9 | 1.6E1 | 1.9E2 | 9.3E1 | 1.5E3 | 1.0E-9 | 1.0E-9 | 1.4E3 | 1.2E4 | 317 | 66 | 136 | 66 | 0.54 |
| Ms | pg/ml | 4.0E2 | 4.5E2 | 5.5E2 | 5.4E2 | 7.0E2 | 5.0E2 | 1.0E-9 | 1.0E-9 | 5.6E3 | 2.2E3 | 317 | 66 | 136 | 66 | 0.52 |
| Mt | pg/ml | 1.0E-9 | 2.0E0 | 1.1E1 | 5.7E0 | 6.7E1 | 1.3E1 | 1.0E-9 | 1.0E-9 | 8.7E2 | 7.8E1 | 317 | 66 | 136 | 66 | 0.69 |
| Mu | pg/ml | 1.0E-9 | 1.0E-9 | 1.7E0 | 1.2E0 | 1.1E1 | 3.9E0 | 1.0E-9 | 1.0E-9 | 9.9E1 | 2.7E1 | 317 | 66 | 136 | 66 | 0.58 |
| Mv | pg/ml | 1.0E-9 | 1.0E-9 | 9.5E1 | 8.7E1 | 4.5E2 | 3.4E2 | 1.0E-9 | 1.0E-9 | 4.5E3 | 2.5E3 | 317 | 66 | 136 | 66 | 0.56 |
| Mw | pg/ml | 2.3E1 | 4.5E1 | 6.3E2 | 4.3E2 | 4.1E3 | 1.3E3 | 1.0E-9 | 1.0E-9 | 6.2E4 | 8.5E3 | 317 | 66 | 136 | 66 | 0.61 |
| Mx | pg/ml | 1.0E-9 | 1.0E-9 | 2.1E-1 | 1.5E-1 | 8.3E-1 | 4.9E-1 | 1.0E-9 | 1.0E-9 | 7.4E0 | 3.0E0 | 317 | 66 | 136 | 66 | 0.52 |
| My | pg/ml | 1.0E-9 | 1.0E-9 | 6.1E2 | 3.0E2 | 3.7E3 | 8.7E2 | 1.0E-9 | 1.0E-9 | 3.9E4 | 4.6E3 | 317 | 66 | 136 | 66 | 0.55 |
| Mz | pg/ml | 9.2E0 | 1.7E1 | 2.3E1 | 3.1E1 | 6.3E1 | 3.8E1 | 1.0E-9 | 1.0E-9 | 8.4E2 | 2.0E2 | 317 | 66 | 136 | 66 | 0.66 |
| Na | pg/ml | 1.0E-9 | 1.0E-9 | 5.3E-1 | 8.0E-1 | 1.7E0 | 2.7E0 | 1.0E-9 | 1.0E-9 | 9.6E0 | 1.6E1 | 317 | 66 | 136 | 66 | 0.53 |
| Nb | pg/ml | 1.9E0 | 2.2E0 | 4.9E0 | 6.8E0 | 1.7E1 | 2.9E1 | 1.0E-9 | 1.0E-9 | 2.3E2 | 2.4E2 | 317 | 66 | 136 | 66 | 0.54 |
| Nc | pg/ml | 4.8E2 | 1.6E2 | 7.1E2 | 3.1E2 | 8.7E2 | 4.3E2 | 1.0E-9 | 1.0E-9 | 9.2E3 | 2.1E3 | 317 | 66 | 136 | 66 | 0.33 |
| Nd | pg/ml | 3.0E1 | 6.0E0 | 2.6E1 | 1.8E1 | 2.0E1 | 2.6E1 | 1.0E-9 | 1.0E-9 | 8.4E1 | 1.4E2 | 317 | 66 | 136 | 66 | 0.35 |
| Ne | pg/ml | 5.2E2 | 2.5E2 | 6.7E2 | 3.8E2 | 6.7E2 | 4.1E2 | 1.0E-9 | 1.0E-9 | 7.0E3 | 1.7E3 | 317 | 66 | 136 | 66 | 0.34 |
| Nf | pg/ml | 1.0E-9 | 1.0E-9 | 2.8E0 | 2.7E0 | 8.7E0 | 1.1E1 | 1.0E-9 | 1.0E-9 | 8.2E1 | 8.2E1 | 317 | 66 | 136 | 66 | 0.45 |
| Ng | pg/ml | 3.8E1 | 8.1E1 | 1.5E2 | 1.6E2 | 2.9E2 | 2.1E2 | 1.0E-9 | 1.0E-9 | 2.3E3 | 1.2E3 | 317 | 66 | 136 | 66 | 0.57 |
| Nh | pg/ml | 7.7E1 | 3.4E1 | 1.0E2 | 4.9E1 | 9.1E1 | 4.8E1 | 1.0E-9 | 1.0E-9 | 5.6E2 | 2.2E2 | 317 | 66 | 136 | 66 | 0.30 |
| Ni | pg/ml | 4.5E0 | 7.1E0 | 8.0E1 | 8.6E1 | 1.3E2 | 1.3E2 | 1.0E-9 | 1.0E-9 | 7.1E2 | 5.7E2 | 317 | 66 | 136 | 66 | 0.51 |
| Nj | pg/ml | 8.9E0 | 2.8E0 | 1.2E1 | 6.2E0 | 1.2E1 | 7.3E0 | 1.0E-9 | 1.0E-9 | 8.3E1 | 2.9E1 | 317 | 66 | 136 | 66 | 0.33 |
| Nk | pg/ml | 2.4E1 | 1.6E1 | 3.7E1 | 2.8E1 | 4.1E1 | 3.1E1 | 1.0E-9 | 1.0E-9 | 2.0E2 | 1.2E2 | 317 | 66 | 136 | 66 | 0.47 |
| Nl | pg/ml | 5.6E1 | 1.8E1 | 7.3E1 | 3.7E1 | 8.5E1 | 4.1E1 | 1.0E-9 | 1.0E-9 | 1.1E3 | 1.9E2 | 317 | 66 | 136 | 66 | 0.33 |
| Hl | pg/ml | 1.2E1 | 2.1E1 | 5.0E1 | 3.9E1 | 8.5E1 | 6.1E1 | 1.0E-9 | 1.0E-9 | 3.5E2 | 2.3E2 | 45 | 15 | 35 | 15 | 0.50 |
| Ho | pg/ml | 1.5E1 | 1.8E1 | 3.4E1 | 2.3E1 | 1.0E2 | 1.4E1 | 1.0E-9 | 8.1E0 | 7.0E2 | 6.0E1 | 45 | 15 | 35 | 15 | 0.62 |
| Hp | ng/ml | 1.7E0 | 1.9E0 | 6.4E1 | 3.0E2 | 2.2E2 | 4.3E2 | 1.0E-9 | 4.2E-1 | 8.9E2 | 8.9E2 | 45 | 15 | 35 | 15 | 0.54 |
| Tz | pg/ml | 4.8E3 | 7.1E3 | 8.4E3 | 3.7E4 | 1.2E4 | 1.5E5 | 7.4E1 | 1.5E2 | 8.8E4 | 1.0E6 | 113 | 44 | 84 | 44 | 0.60 |
| Ua | pg/ml | 3.8E3 | 5.0E3 | 1.3E4 | 1.4E4 | 2.5E4 | 2.9E4 | 3.5E2 | 1.0E3 | 1.4E5 | 1.9E5 | 113 | 44 | 84 | 44 | 0.60 |
| Ub | pg/ml | 5.3E2 | 6.6E2 | 7.9E2 | 1.0E3 | 1.2E3 | 1.2E3 | 1.0E-9 | 1.9E1 | 9.8E3 | 6.4E3 | 113 | 44 | 84 | 44 | 0.60 |
| Ue | pg/ml | 3.0E1 | 3.7E1 | 3.2E1 | 5.2E1 | 1.9E1 | 5.5E1 | 4.2E0 | 7.7E0 | 9.5E1 | 3.5E2 | 113 | 44 | 84 | 44 | 0.64 |
| Uc | pg/ml | 8.9E2 | 1.5E3 | 1.8E3 | 2.3E3 | 3.4E3 | 2.3E3 | 6.1E-1 | 9.2E1 | 2.9E4 | 9.2E3 | 113 | 44 | 84 | 44 | 0.62 |
| Ud | pg/ml | 1.0E-9 | 1.0E-9 | 3.7E0 | 1.0E-9 | 3.7E1 | 0.0E0 | 1.0E-9 | 1.0E-9 | 3.9E2 | 1.0E-9 | 113 | 44 | 84 | 44 | 0.49 |
| Hq | pg/ml | 1.1E0 | 1.7E0 | 1.2E1 | 1.2E1 | 6.3E1 | 4.6E1 | 1.0E-9 | 1.0E-9 | 9.5E2 | 3.0E2 | 317 | 65 | 136 | 65 | 0.52 |
| Hr | pg/ml | 1.3E2 | 1.1E2 | 8.2E2 | 6.1E2 | 1.5E3 | 1.6E3 | 1.0E-9 | 1.0E-9 | 9.7E3 | 1.1E4 | 317 | 65 | 136 | 65 | 0.47 |
| Hu | pg/ml | 1.4E1 | 1.9E1 | 3.5E3 | 1.7E3 | 3.7E4 | 6.9E3 | 1.0E-9 | 1.0E-9 | 6.3E5 | 4.7E4 | 317 | 65 | 136 | 65 | 0.57 |
| Hv | pg/ml | 1.3E0 | 1.7E0 | 3.6E0 | 3.7E0 | 1.5E1 | 8.0E0 | 1.0E-9 | 1.0E-9 | 2.5E2 | 5.9E1 | 317 | 65 | 136 | 65 | 0.58 |
| Hw | pg/ml | 6.8E0 | 8.4E0 | 2.7E1 | 6.8E1 | 1.1E2 | 4.2E2 | 1.0E-9 | 1.0E-9 | 1.7E3 | 3.4E3 | 317 | 65 | 136 | 65 | 0.47 |
| Hx | pg/ml | 8.3E0 | 1.3E1 | 6.7E1 | 5.1E1 | 5.3E2 | 2.4E2 | 1.0E-9 | 1.0E-9 | 9.3E3 | 2.0E3 | 317 | 65 | 136 | 65 | 0.56 |
| Ib | ng/ml | 6.6E-2 | 7.8E-2 | 7.9E-1 | 1.9E0 | 3.4E0 | 6.5E0 | 1.0E-9 | 1.0E-9 | 3.0E1 | 3.9E1 | 109 | 43 | 85 | 43 | 0.56 |
| Ic | U/ml | 2.2E2 | 2.2E2 | 3.3E2 | 3.8E2 | 1.5E3 | 6.7E2 | 2.9E0 | 1.2E1 | 1.5E4 | 4.2E3 | 109 | 43 | 85 | 43 | 0.63 |
| Id | U/ml | 6.2E-1 | 1.0E0 | 1.1E0 | 2.1E0 | 1.3E0 | 3.8E0 | 1.0E-9 | 1.0E-9 | 7.2E0 | 2.1E1 | 109 | 43 | 85 | 43 | 0.60 |
| Tt | pg/ml | 1.6E2 | 1.8E2 | 1.7E2 | 1.8E2 | 4.3E1 | 4.8E1 | 8.0E1 | 9.9E1 | 3.0E2 | 2.8E2 | 101 | 44 | 78 | 44 | 0.62 |
| To | pg/ml | 1.6E0 | 1.6E0 | 1.7E0 | 1.9E0 | 1.8E0 | 1.8E0 | 1.0E-9 | 1.0E-9 | 7.1E0 | 7.1E0 | 108 | 44 | 81 | 44 | 0.54 |
| Tr | pg/ml | 2.8E0 | 4.4E0 | 4.0E0 | 6.2E0 | 4.2E0 | 5.8E0 | 3.5E-2 | 4.1E-1 | 2.4E1 | 2.6E1 | 106 | 44 | 80 | 44 | 0.63 |
| Tn | pg/ml | 2.5E1 | 3.3E1 | 4.8E1 | 5.0E1 | 7.0E1 | 5.5E1 | 2.6E0 | 8.7E0 | 3.7E2 | 3.1E2 | 108 | 44 | 81 | 44 | 0.61 |
| Tv | ng/ml | 1.1E1 | 1.3E1 | 2.0E1 | 2.0E1 | 5.0E1 | 2.4E1 | 1.0E-9 | 1.0E-9 | 4.9E2 | 1.3E2 | 108 | 44 | 81 | 44 | 0.53 |
| Ih | ng/ml | 6.8E1 | 1.2E2 | 1.9E2 | 3.3E2 | 3.2E2 | 5.7E2 | 1.0E-9 | 1.0E-9 | 2.5E3 | 2.9E3 | 317 | 66 | 136 | 66 | 0.61 |
| Ii | ng/ml | 8.1E1 | 1.1E2 | 2.9E2 | 5.1E2 | 8.2E2 | 1.7E3 | 7.5E-1 | 7.5E0 | 8.4E3 | 1.0E4 | 317 | 66 | 136 | 66 | 0.59 |
| Ij | ng/ml | 7.2E1 | 9.1E1 | 2.1E2 | 2.5E2 | 7.5E2 | 8.1E2 | 2.1E0 | 1.9E1 | 6.4E3 | 6.4E3 | 317 | 65 | 136 | 65 | 0.60 |
| Ik | ng/ml | 1.1E1 | 2.7E2 | 1.0E3 | 7.6E2 | 9.8E3 | 1.3E3 | 5.9E-1 | 1.6E0 | 1.2E5 | 9.7E3 | 316 | 66 | 136 | 66 | 0.70 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Il | ng/ml | 3.8E2 | 5.9E2 | 1.5E3 | 1.4E3 | 3.1E3 | 2.8E3 | 1.0E-9 | 1.0E-9 | 1.2E4 | 1.2E4 | 314 | 64 | 136 | 64 | 0.52 |
| Im | ng/ml | 1.9E2 | 2.7E2 | 3.1E2 | 5.6E2 | 3.5E2 | 8.4E2 | 1.3E1 | 6.4E1 | 3.1E3 | 5.6E3 | 316 | 66 | 136 | 66 | 0.64 |
| In | ng/ml | 4.2E0 | 5.0E0 | 3.5E1 | 1.2E1 | 2.4E2 | 1.8E1 | 1.0E-9 | 1.0E-9 | 3.9E3 | 8.4E1 | 317 | 66 | 136 | 66 | 0.52 |
| Hb | ng/ml | 2.0E1 | 2.9E1 | 2.6E1 | 3.8E1 | 2.4E1 | 3.6E1 | 1.1E0 | 5.2E0 | 1.3E2 | 2.1E2 | 110 | 44 | 86 | 44 | 0.65 |
| Hc | pg/ml | 6.9E2 | 9.0E2 | 2.0E3 | 4.3E3 | 4.7E3 | 1.5E4 | 1.0E-9 | 1.0E-9 | 3.6E4 | 1.0E5 | 110 | 44 | 86 | 44 | 0.58 |
| Hf | ng/ml | 1.4E2 | 1.9E2 | 4.0E2 | 4.0E2 | 5.5E2 | 5.2E2 | 1.0E-9 | 1.0E-9 | 2.5E3 | 2.2E3 | 110 | 44 | 86 | 44 | 0.53 |
| Io | ng/ml | 8.4E3 | 8.2E3 | 1.8E4 | 2.2E4 | 5.5E4 | 6.9E4 | 6.6E1 | 3.2E2 | 8.8E5 | 5.5E5 | 315 | 65 | 135 | 65 | 0.54 |
| Ip | ng/ml | 9.7E0 | 2.3E1 | 1.9E1 | 2.5E1 | 2.6E1 | 2.6E1 | 1.0E-9 | 4.1E-2 | 2.6E2 | 1.4E2 | 315 | 65 | 135 | 65 | 0.57 |
| Iq | ug/ml | 1.1E-1 | 3.0E-2 | 4.4E1 | 1.3E1 | 7.7E2 | 9.4E1 | 1.0E-9 | 1.0E-9 | 1.4E4 | 7.5E2 | 315 | 65 | 135 | 65 | 0.44 |
| Ir | ug/ml | 3.2E-1 | 4.7E-1 | 3.2E0 | 3.2E0 | 2.1E1 | 1.4E1 | 1.0E-9 | 1.0E-9 | 2.4E2 | 1.1E2 | 314 | 65 | 135 | 65 | 0.60 |
| Is | ng/ml | 1.4E0 | 2.5E0 | 5.6E0 | 9.8E0 | 1.2E1 | 3.0E1 | 1.0E-9 | 1.0E-9 | 8.8E1 | 2.3E2 | 315 | 65 | 135 | 65 | 0.58 |
| It | ng/ml | 2.0E0 | 2.7E0 | 2.2E1 | 2.8E1 | 1.1E2 | 1.2E2 | 1.0E-9 | 1.0E-9 | 1.2E3 | 7.7E2 | 315 | 65 | 135 | 65 | 0.53 |
| Iu | ng/ml | 2.1E2 | 2.6E2 | 1.5E3 | 1.8E3 | 4.8E3 | 5.4E3 | 1.0E-9 | 1.0E-9 | 2.9E4 | 2.4E4 | 315 | 65 | 135 | 65 | 0.50 |
| Iv | ng/ml | 1.3E1 | 2.6E1 | 4.7E1 | 1.5E2 | 1.3E2 | 8.0E2 | 1.0E-9 | 1.0E-9 | 1.6E3 | 6.4E3 | 315 | 65 | 135 | 65 | 0.61 |
| Iz | ng/ml | 1.3E2 | 3.1E2 | 4.0E2 | 5.2E2 | 9.6E2 | 6.8E2 | 1.5E0 | 8.7E0 | 8.4E3 | 3.6E3 | 110 | 44 | 86 | 44 | 0.64 |
| Yg | pg/ml | 2.5E2 | 5.2E2 | 1.6E3 | 1.4E3 | 7.4E3 | 3.1E3 | 1.0E-9 | 4.1E1 | 5.0E4 | 1.2E4 | 45 | 14 | 35 | 14 | 0.62 |
| Yh | pg/ml | 2.1E2 | 2.9E2 | 5.1E2 | 4.1E2 | 1.2E3 | 3.8E2 | 1.0E-9 | 1.0E-9 | 7.8E3 | 1.0E3 | 45 | 14 | 35 | 14 | 0.55 |
| Yi | pg/ml | 2.2E2 | 4.4E2 | 5.6E2 | 3.6E2 | 1.2E3 | 2.7E2 | 1.0E-9 | 1.0E-9 | 7.6E3 | 8.7E2 | 45 | 14 | 35 | 14 | 0.53 |
| Yk | U/ml | 1.0E-9 | 1.0E-9 | 1.8E-1 | 4.0E-2 | 5.3E-1 | 1.1E-1 | 1.0E-9 | 1.0E-9 | 3.0E0 | 3.6E-1 | 45 | 14 | 35 | 14 | 0.46 |
| Yj | pg/ml | 1.7E2 | 1.2E2 | 3.9E2 | 4.7E2 | 5.6E2 | 1.1E3 | 1.0E-9 | 1.7E1 | 3.2E3 | 4.1E3 | 45 | 14 | 35 | 14 | 0.44 |
| Yd | ng/ml | 2.0E-1 | 1.4E-1 | 3.1E-1 | 2.4E-1 | 4.0E-1 | 2.0E-1 | 6.6E-3 | 4.2E-2 | 1.8E0 | 6.0E-1 | 47 | 15 | 37 | 15 | 0.52 |
| Wb | pg/ml | 3.0E4 | 2.9E4 | 3.4E4 | 3.5E4 | 1.8E4 | 2.0E4 | 2.2E3 | 1.0E4 | 8.5E4 | 8.5E4 | 47 | 15 | 37 | 15 | 0.50 |
| Vz | pg/ml | 3.2E0 | 2.3E0 | 4.9E0 | 2.2E0 | 5.8E0 | 1.9E0 | 1.0E-9 | 1.0E-9 | 2.9E1 | 7.6E0 | 47 | 15 | 37 | 15 | 0.34 |
| Si | ng/ml | 8.0E-1 | 1.4E0 | 1.5E0 | 3.3E0 | 2.2E0 | 4.2E0 | 1.9E-2 | 7.8E-2 | 1.0E1 | 1.3E1 | 45 | 15 | 35 | 15 | 0.65 |
| Sf | mIU/mL | 1.2E1 | 1.2E1 | 5.4E1 | 2.3E1 | 1.2E2 | 3.7E1 | 8.1E-2 | 1.1E0 | 7.2E2 | 1.5E2 | 45 | 15 | 35 | 15 | 0.48 |
| Sh | mIU/mL | 1.3E1 | 9.0E0 | 5.2E1 | 2.0E1 | 1.2E2 | 2.3E1 | 2.9E-2 | 1.3E-1 | 5.9E2 | 7.5E1 | 45 | 15 | 35 | 15 | 0.47 |
| Sj | ng/ml | 4.4E-1 | 3.8E-1 | 4.2E-1 | 3.8E-1 | 8.5E-2 | 1.4E-1 | 2.5E-1 | 1.1E-1 | 6.1E-1 | 6.2E-1 | 45 | 15 | 35 | 15 | 0.39 |
| Rc | pg/ml | 5.2E3 | 7.2E3 | 6.3E3 | 9.1E3 | 4.4E3 | 6.5E3 | 1.9E2 | 1.4E3 | 2.2E4 | 2.3E4 | 111 | 44 | 84 | 44 | 0.61 |
| Rb | pg/ml | 7.6E-1 | 1.1E0 | 2.5E0 | 3.3E0 | 3.6E0 | 4.6E0 | 1.0E-9 | 1.0E-9 | 1.6E1 | 2.1E1 | 111 | 44 | 84 | 44 | 0.55 |
| Zq | 2.6ng/ml | 1.5E2 | 3.5E2 | 2.0E2 | 4.6E2 | 1.5E2 | 3.8E2 | 8.3E0 | 6.9E1 | 5.2E2 | 9.7E2 | 44 | 15 | 34 | 15 | 0.71 |
| Zw | 2.5ng/ml | 3.6E0 | 3.8E0 | 1.0E1 | 8.9E0 | 1.5E1 | 1.2E1 | 6.3E-2 | 3.5E-1 | 5.9E1 | 4.6E1 | 47 | 15 | 37 | 15 | 0.48 |
| Zx | 2.3mU/ml | 1.0E-1 | 9.5E-2 | 3.0E-1 | 1.8E-1 | 5.9E-1 | 1.6E-1 | 4.1E-2 | 5.6E-2 | 3.0E0 | 5.5E-1 | 47 | 15 | 37 | 15 | 0.51 |
| Pz | ng/ml | 3.3E3 | 1.0E4 | 7.5E3 | 2.5E4 | 1.9E4 | 1.3E5 | 1.3E1 | 3.5E2 | 2.8E5 | 1.0E6 | 314 | 65 | 134 | 65 | 0.61 |
| Qa | ng/ml | 2.8E3 | 4.6E3 | 6.2E3 | 7.4E3 | 8.3E3 | 5.8E3 | 1.5E2 | 9.5E2 | 5.2E4 | 2.3E4 | 314 | 65 | 134 | 65 | 0.65 |
| Qb | ng/ml | 1.0E2 | 1.3E2 | 2.6E2 | 1.9E2 | 6.8E2 | 1.8E2 | 7.9E-1 | 1.0E1 | 8.3E3 | 7.2E2 | 314 | 65 | 134 | 65 | 0.56 |
| Qc | ng/ml | 2.5E2 | 3.4E2 | 5.2E2 | 4.5E2 | 1.0E3 | 4.0E2 | 8.1E-1 | 1.1E1 | 1.1E4 | 1.6E3 | 314 | 65 | 134 | 65 | 0.56 |
| Qd | ng/ml | 9.5E3 | 1.4E4 | 2.8E4 | 2.2E4 | 1.3E5 | 2.4E4 | 2.4E2 | 2.7E3 | 2.0E6 | 1.2E5 | 314 | 65 | 134 | 65 | 0.62 |
| Qe | ng/ml | 7.8E2 | 1.6E3 | 2.0E3 | 2.3E3 | 5.9E3 | 2.5E3 | 7.6E0 | 2.5E2 | 9.7E4 | 1.4E4 | 314 | 65 | 134 | 65 | 0.66 |
| Jd | ng/ml | 3.6E-1 | 2.5E0 | 7.1E0 | 4.6E0 | 6.2E1 | 1.1E1 | 1.0E-9 | 1.0E-9 | 6.5E2 | 7.3E1 | 111 | 44 | 86 | 44 | 0.73 |
| Je | ng/ml | 1.0E-9 | 6.1E-1 | 1.4E0 | 1.6E0 | 5.6E0 | 2.1E0 | 1.0E-9 | 1.0E-9 | 5.6E1 | 7.0E0 | 111 | 44 | 86 | 44 | 0.63 |
| Jf | ng/ml | 1.0E-9 | 1.0E-9 | 8.1E-1 | 1.4E0 | 2.1E0 | 2.5E0 | 1.0E-9 | 1.0E-9 | 1.2E1 | 9.6E0 | 111 | 44 | 86 | 44 | 0.58 |
| Jg | ng/ml | 3.7E2 | 1.2E3 | 7.2E2 | 1.4E3 | 1.1E3 | 1.2E3 | 5.8E0 | 4.0E1 | 1.0E4 | 5.4E3 | 317 | 65 | 136 | 65 | 0.72 |
| Jh | ng/ml | 2.3E0 | 7.7E0 | 2.5E1 | 3.9E1 | 1.0E2 | 9.3E1 | 1.0E-9 | 1.0E-9 | 1.2E3 | 4.7E2 | 317 | 65 | 136 | 65 | 0.62 |
| Ji | ng/ml | 4.6E1 | 7.9E1 | 6.7E1 | 1.2E2 | 6.9E1 | 1.0E2 | 1.1E0 | 1.3E1 | 5.3E2 | 5.9E2 | 317 | 65 | 136 | 65 | 0.71 |
| Sr | pg/mL | 3.0E2 | 6.4E2 | 7.6E2 | 1.0E3 | 1.3E3 | 1.1E3 | 1.0E-9 | 1.0E-9 | 9.8E3 | 5.1E3 | 106 | 42 | 82 | 42 | 0.64 |
| Ss | pg/mL | 7.6E4 | 2.0E5 | 1.3E5 | 1.9E5 | 1.3E5 | 1.5E5 | 9.5E3 | 1.4E4 | 6.7E5 | 6.8E5 | 106 | 42 | 82 | 42 | 0.63 |
| St | pg/mL | 2.2E7 | 3.6E7 | 4.7E7 | 6.5E7 | 6.0E7 | 9.0E7 | 7.8E5 | 1.1E6 | 4.1E8 | 4.2E8 | 108 | 44 | 82 | 44 | 0.58 |
| Wc | ng/ml | 1.0E-9 | 2.6E-2 | 5.2E-2 | 6.4E-2 | 1.5E-1 | 9.1E-2 | 1.0E-9 | 1.0E-9 | 9.8E-1 | 2.8E-1 | 47 | 15 | 36 | 15 | 0.58 |
| Wd | ng/ml | 8.9E0 | 9.9E0 | 1.8E1 | 7.6E1 | 4.3E1 | 2.0E2 | 1.0E-9 | 7.8E-1 | 2.9E2 | 7.9E2 | 47 | 15 | 36 | 15 | 0.59 |
| We | ng/ml | 3.5E-1 | 4.7E-1 | 6.9E-1 | 8.1E-1 | 9.7E-1 | 1.0E0 | 1.0E-9 | 1.0E-9 | 3.9E0 | 3.5E0 | 47 | 15 | 36 | 15 | 0.54 |
| Wg | ng/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 47 | 15 | 36 | 15 | 0.50 |
| Wh | ng/ml | 8.7E-3 | 8.7E-3 | 1.9E-2 | 2.0E-1 | 5.1E-2 | 6.3E-1 | 1.0E-9 | 1.0E-9 | 3.5E-1 | 2.5E0 | 47 | 15 | 36 | 15 | 0.56 |
| Wf | ng/ml | 1.0E-9 | 1.0E-9 | 1.4E-1 | 2.2E-1 | 2.6E-1 | 6.3E-1 | 1.0E-9 | 1.0E-9 | 1.1E0 | 2.4E0 | 47 | 15 | 36 | 15 | 0.42 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ra | pg/ml | 1.0E-9 | 1.0E-9 | 7.5E-1 | 2.4E-1 | 1.4E0 | 6.7E-1 | 1.0E-9 | 1.0E-9 | 7.3E0 | 2.6E0 | 111 | 44 | 84 | 44 | 0.42 |
| Qz | pg/ml | 1.1E1 | 9.6E0 | 6.9E1 | 3.7E1 | 1.1E2 | 5.5E1 | 1.0E-9 | 1.0E-9 | 7.1E2 | 2.2E2 | 111 | 44 | 84 | 44 | 0.43 |
| Qy | pg/ml | 4.1E-1 | 5.7E-1 | 2.9E0 | 1.6E1 | 1.5E1 | 7.7E1 | 1.0E-9 | 1.0E-9 | 1.6E2 | 5.1E2 | 111 | 44 | 84 | 44 | 0.57 |
| Qx | pg/ml | 1.0E-9 | 1.0E-9 | 6.4E0 | 4.1E-1 | 5.1E1 | 1.8E0 | 1.0E-9 | 1.0E-9 | 5.4E2 | 9.4E0 | 111 | 44 | 84 | 44 | 0.46 |
| Qw | pg/ml | 1.8E-1 | 1.0E-9 | 1.4E0 | 1.3E0 | 3.7E0 | 4.1E0 | 1.0E-9 | 1.0E-9 | 3.0E1 | 2.7E1 | 111 | 44 | 84 | 44 | 0.44 |
| Qv | pg/ml | 2.3E4 | 2.5E4 | 3.7E4 | 3.5E4 | 7.7E4 | 4.1E4 | 1.2E3 | 1.1E3 | 7.4E5 | 2.3E5 | 111 | 44 | 84 | 44 | 0.49 |
| Qu | pg/ml | 5.1E0 | 2.6E1 | 7.9E1 | 1.1E2 | 1.7E2 | 2.0E2 | 1.0E-9 | 1.0E-9 | 8.0E2 | 9.2E2 | 111 | 44 | 84 | 44 | 0.60 |
| Qt | pg/ml | 1.2E1 | 1.2E1 | 3.0E1 | 7.4E1 | 5.9E1 | 1.5E2 | 1.0E-9 | 1.0E-9 | 3.8E2 | 7.0E2 | 111 | 44 | 84 | 44 | 0.57 |
| Qh | ng/ml | 1.5E1 | 1.7E1 | 3.7E1 | 3.4E1 | 7.2E1 | 4.8E1 | 1.2E-1 | 1.4E0 | 6.4E2 | 2.6E2 | 111 | 44 | 84 | 44 | 0.54 |
| Qg | ng/ml | 8.9E0 | 6.3E0 | 2.6E1 | 1.3E1 | 1.0E2 | 3.3E1 | 1.5E-1 | 1.3E-1 | 1.0E3 | 2.2E2 | 111 | 44 | 84 | 44 | 0.39 |
| Jj | ng/ml | 8.4E2 | 5.2E2 | 2.9E3 | 1.0E3 | 2.0E4 | 1.6E3 | 2.0E1 | 1.7E1 | 3.4E5 | 1.1E4 | 317 | 65 | 136 | 65 | 0.41 |
| Jk | ng/ml | 3.3E0 | 3.8E0 | 2.4E1 | 2.5E1 | 5.0E1 | 4.8E1 | 1.0E-9 | 1.2E-1 | 2.8E2 | 2.7E2 | 317 | 65 | 136 | 65 | 0.55 |
| Jl | ng/ml | 3.7E-1 | 8.2E-1 | 1.9E0 | 4.9E0 | 4.7E0 | 2.0E1 | 7.6E-4 | 1.6E-2 | 3.2E1 | 1.6E2 | 317 | 65 | 136 | 65 | 0.63 |
| Jm | ng/ml | 1.9E1 | 3.3E1 | 5.1E1 | 6.4E1 | 9.4E1 | 9.7E1 | 1.0E-9 | 4.1E-1 | 1.0E3 | 6.1E2 | 317 | 65 | 136 | 65 | 0.55 |
| Jn | pg/ml | 3.8E-1 | 7.7E-1 | 1.9E0 | 1.6E0 | 7.0E0 | 3.6E0 | 1.0E-9 | 1.0E-9 | 6.2E1 | 2.7E1 | 317 | 65 | 136 | 65 | 0.60 |
| Jo | pg/ml | 4.0E3 | 5.0E3 | 5.0E3 | 6.4E3 | 3.7E3 | 5.7E3 | 4.2E1 | 4.8E2 | 2.0E4 | 3.8E4 | 317 | 65 | 136 | 65 | 0.57 |
| Jp | pg/ml | 6.6E4 | 8.9E4 | 7.0E4 | 9.0E4 | 3.4E4 | 3.7E4 | 2.1E3 | 1.4E4 | 2.1E5 | 2.1E5 | 317 | 65 | 136 | 65 | 0.66 |
| Jq | pg/ml | 9.2E1 | 1.1E2 | 1.4E2 | 2.2E2 | 1.6E2 | 5.1E2 | 2.6E0 | 8.1E0 | 1.1E3 | 4.0E3 | 317 | 65 | 136 | 65 | 0.56 |
| Jr | pg/ml | 3.8E0 | 7.0E0 | 1.8E1 | 1.8E1 | 6.6E1 | 3.7E1 | 1.0E-9 | 1.0E-9 | 7.9E2 | 2.0E2 | 317 | 65 | 136 | 65 | 0.58 |
| Js | pg/ml | 1.3E1 | 1.5E1 | 4.5E1 | 2.8E1 | 1.5E2 | 7.3E1 | 1.0E-9 | 2.6E0 | 1.6E3 | 5.9E2 | 317 | 65 | 136 | 65 | 0.55 |
| Jt | pg/ml | 2.4E3 | 3.8E3 | 3.0E3 | 4.4E3 | 2.1E3 | 4.2E3 | 1.5E2 | 6.4E2 | 1.2E4 | 3.3E4 | 317 | 65 | 136 | 65 | 0.64 |
| Xa | pg/ml | 1.0E-9 | 7.0E-1 | 9.8E0 | 1.2E1 | 2.1E1 | 2.0E1 | 1.0E-9 | 1.0E-9 | 9.6E1 | 6.4E1 | 47 | 14 | 37 | 14 | 0.54 |
| Ye | pg/ml | 1.0E-9 | 1.0E-9 | 3.0E0 | 5.4E0 | 1.0E1 | 1.2E1 | 1.0E-9 | 1.0E-9 | 6.9E1 | 4.1E1 | 47 | 14 | 37 | 14 | 0.47 |
| Tm | pg/ml | 1.0E-9 | 1.0E-9 | 3.2E0 | 1.8E0 | 5.4E0 | 3.6E0 | 1.0E-9 | 1.0E-9 | 1.8E1 | 8.4E0 | 47 | 14 | 37 | 14 | 0.45 |
| Tl | pg/ml | 1.1E-1 | 4.7E-1 | 2.7E-1 | 4.2E-1 | 3.8E-1 | 3.5E-1 | 1.0E-9 | 1.0E-9 | 1.8E0 | 1.2E0 | 47 | 14 | 37 | 14 | 0.65 |
| Ju | mIU/ml | 7.2E0 | 1.2E1 | 2.2E1 | 1.9E1 | 3.6E1 | 3.2E1 | 6.5E-2 | 3.4E-1 | 2.3E2 | 2.0E2 | 111 | 44 | 86 | 44 | 0.57 |
| Jv | mIU/ml | 1.1E1 | 1.6E1 | 3.9E1 | 3.0E1 | 7.4E1 | 5.4E1 | 1.0E-2 | 8.2E-2 | 4.4E2 | 3.4E2 | 111 | 44 | 86 | 44 | 0.53 |
| Jy | ng/ml | 1.5E-3 | 1.6E-3 | 2.3E-3 | 1.6E-3 | 4.9E-3 | 6.9E-4 | 1.7E-4 | 4.5E-4 | 5.2E-2 | 3.7E-3 | 111 | 44 | 86 | 44 | 0.46 |
| Kc | pg/ml | 2.1E1 | 4.3E1 | 3.5E1 | 6.3E1 | 4.0E1 | 5.3E1 | 1.0E-9 | 1.0E-9 | 1.7E2 | 1.8E2 | 111 | 43 | 86 | 43 | 0.70 |
| Kd | pg/ml | 1.0E-9 | 1.0E-9 | 2.4E2 | 2.9E2 | 7.3E2 | 6.1E2 | 1.0E-9 | 1.0E-9 | 4.8E3 | 2.3E3 | 111 | 43 | 86 | 43 | 0.53 |
| Ke | pg/ml | 9.6E3 | 1.6E4 | 1.3E4 | 1.8E4 | 9.5E3 | 1.0E4 | 3.4E2 | 2.9E3 | 5.9E4 | 5.6E4 | 111 | 43 | 86 | 43 | 0.66 |
| Kf | pg/mL | 5.9E0 | 9.5E0 | 6.5E0 | 9.6E0 | 5.7E0 | 4.7E0 | 1.0E-9 | 2.2E-1 | 2.6E1 | 2.4E1 | 111 | 43 | 86 | 43 | 0.70 |
| Kg | pg/mL | 9.9E2 | 1.8E3 | 1.5E3 | 2.5E3 | 1.5E3 | 2.3E3 | 7.3E1 | 1.4E2 | 8.4E3 | 1.1E4 | 111 | 43 | 86 | 43 | 0.65 |
| Ki | pg/ml | 6.4E1 | 5.6E1 | 7.2E1 | 6.7E1 | 5.2E1 | 4.5E1 | 1.0E-9 | 1.0E-9 | 2.9E2 | 2.8E2 | 111 | 42 | 86 | 42 | 0.47 |
| Kj | pg/ml | 9.3E2 | 1.5E3 | 1.5E3 | 2.0E3 | 1.7E3 | 1.5E3 | 1.4E1 | 1.1E2 | 1.0E4 | 6.1E3 | 111 | 43 | 86 | 43 | 0.63 |
| Kk | pg/ml | 6.9E0 | 7.1E0 | 1.1E1 | 1.4E1 | 1.8E1 | 1.6E1 | 1.0E-9 | 1.0E-9 | 1.6E2 | 6.1E1 | 111 | 43 | 86 | 43 | 0.53 |
| Kl | pg/ml | 1.8E4 | 4.5E4 | 2.5E4 | 4.2E4 | 2.2E4 | 3.2E4 | 1.6E2 | 2.7E3 | 1.1E5 | 1.6E5 | 111 | 43 | 86 | 43 | 0.68 |
| Kn | pg/ml | 1.5E1 | 5.7E1 | 5.8E1 | 8.6E1 | 1.1E2 | 1.1E2 | 1.0E-9 | 1.0E-9 | 7.3E2 | 4.7E2 | 111 | 43 | 86 | 43 | 0.64 |
| Ko | pg/ml | 3.0E2 | 7.0E2 | 3.9E2 | 7.3E2 | 4.0E2 | 6.1E2 | 1.0E-9 | 1.0E-9 | 1.8E3 | 2.4E3 | 111 | 43 | 86 | 43 | 0.67 |
| Kp | pg/ml | 3.2E2 | 3.6E2 | 3.4E2 | 4.2E2 | 3.0E2 | 2.5E2 | 1.0E-9 | 1.0E-9 | 1.7E3 | 9.1E2 | 111 | 43 | 86 | 43 | 0.61 |
| Kq | pg/ml | 2.8E2 | 5.0E2 | 4.2E2 | 5.6E2 | 9.7E2 | 3.9E2 | 5.1E0 | 1.1E2 | 9.8E3 | 2.1E3 | 106 | 44 | 82 | 44 | 0.71 |
| Kr | pg/ml | 5.0E-1 | 2.0E0 | 2.5E0 | 3.0E0 | 4.9E0 | 3.5E0 | 1.0E-9 | 1.0E-9 | 3.5E1 | 1.2E1 | 106 | 44 | 82 | 44 | 0.58 |
| Ks | pg/ml | 1.5E4 | 1.5E4 | 2.1E4 | 2.1E4 | 2.0E4 | 1.7E4 | 3.8E2 | 2.7E2 | 1.1E5 | 5.0E4 | 106 | 44 | 82 | 44 | 0.53 |
| Ps | ng/ml | 1.4E2 | 1.9E2 | 4.3E2 | 5.3E2 | 1.2E3 | 8.2E2 | 6.9E0 | 1.7E1 | 8.3E3 | 2.9E3 | 45 | 15 | 34 | 15 | 0.56 |
| Kx | ng/ml | 1.0E-9 | 7.4E-3 | 4.6E-3 | 8.4E-3 | 8.6E-3 | 9.9E-3 | 1.0E-9 | 1.0E-9 | 5.1E-2 | 4.4E-2 | 110 | 44 | 86 | 44 | 0.64 |
| Ky | ng/ml | 1.0E-1 | 1.3E-1 | 3.5E-1 | 3.7E-1 | 7.1E-1 | 5.5E-1 | 1.0E-9 | 1.0E-9 | 5.1E0 | 2.4E0 | 110 | 44 | 86 | 44 | 0.54 |
| Kz | ng/ml | 1.0E-9 | 1.0E-9 | 3.7E-3 | 2.3E-3 | 6.3E-3 | 4.7E-3 | 1.0E-9 | 1.0E-9 | 3.6E-2 | 1.4E-2 | 110 | 44 | 86 | 44 | 0.45 |
| Rz | ng/ml | 1.4E-1 | 5.6E-1 | 6.0E-1 | 1.4E0 | 1.0E0 | 1.7E0 | 3.6E-3 | 5.7E-2 | 4.4E0 | 4.7E0 | 45 | 15 | 35 | 15 | 0.69 |
| Ry | ng/ml | 1.6E-2 | 2.0E-2 | 1.8E-2 | 2.5E-2 | 2.0E-2 | 1.7E-2 | 1.0E-9 | 1.0E-9 | 1.0E-1 | 6.5E-2 | 45 | 15 | 35 | 15 | 0.65 |
| Rx | ng/ml | 1.0E-9 | 3.5E-5 | 2.6E-3 | 9.5E-4 | 4.4E-3 | 1.6E-3 | 1.0E-9 | 1.0E-9 | 2.0E-2 | 4.7E-3 | 45 | 15 | 35 | 15 | 0.46 |
| Ld | pg/ml | 1.0E-9 | 1.0E-9 | 4.0E0 | 2.6E0 | 9.9E0 | 4.7E0 | 1.0E-9 | 1.0E-9 | 6.9E1 | 2.5E1 | 108 | 44 | 85 | 44 | 0.50 |
| Lh | pg/ml | 1.1E4 | 2.3E4 | 2.0E4 | 3.4E4 | 2.9E4 | 5.3E4 | 1.0E-9 | 1.8E3 | 2.6E5 | 4.1E5 | 315 | 65 | 136 | 65 | 0.67 |
| Li | pg/ml | 2.6E3 | 6.7E3 | 8.5E3 | 2.9E4 | 2.3E4 | 7.8E4 | 1.0E-9 | 3.6E1 | 2.9E5 | 5.9E5 | 315 | 65 | 136 | 65 | 0.68 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Lj | pg/ml | 1.8E3 | 5.0E3 | 1.1E4 | 3.4E4 | 4.2E4 | 8.4E4 | 1.4E1 | 4.1E1 | 4.4E5 | 4.7E5 | 315 | 65 | 136 | 65 | 0.65 |
| Lp | pg/ml | 9.4E0 | 4.0E0 | 3.1E1 | 8.3E1 | 5.7E1 | 2.2E2 | 1.0E-9 | 1.0E-9 | 2.4E2 | 7.7E2 | 45 | 15 | 35 | 15 | 0.41 |
| Lt | pg/ml | 1.0E-9 | 1.0E-9 | 2.6E0 | 2.2E0 | 1.0E1 | 6.7E0 | 1.0E-9 | 1.0E-9 | 6.0E1 | 2.5E1 | 45 | 15 | 35 | 15 | 0.52 |
| Rv | ng/ml | 5.0E-4 | 5.0E-4 | 8.5E-4 | 6.9E-4 | 1.1E-3 | 7.4E-4 | 1.0E-9 | 1.0E-9 | 5.9E-3 | 2.2E-3 | 45 | 15 | 34 | 15 | 0.49 |
| Ru | ng/ml | 1.0E-9 | 1.0E-9 | 9.6E-3 | 3.0E-2 | 4.9E-2 | 9.9E-2 | 1.0E-9 | 1.0E-9 | 3.1E-1 | 3.8E-1 | 45 | 15 | 34 | 15 | 0.54 |
| Rt | ng/ml | 6.8E-2 | 3.2E-2 | 9.0E-2 | 1.2E-1 | 1.1E-1 | 1.8E-1 | 1.6E-3 | 1.0E-3 | 4.5E-1 | 5.6E-1 | 45 | 15 | 34 | 15 | 0.45 |
| Yl | pg/ml | 1.1E1 | 6.4E0 | 1.8E1 | 9.4E0 | 1.9E1 | 1.6E1 | 1.0E-9 | 1.0E-9 | 1.1E2 | 6.6E1 | 47 | 15 | 37 | 15 | 0.31 |
| Rm | ng/ml | 1.7E1 | 2.1E1 | 5.8E1 | 5.1E1 | 8.8E1 | 6.9E1 | 2.2E-1 | 1.3E0 | 4.0E2 | 2.5E2 | 110 | 43 | 83 | 43 | 0.53 |
| Rh | ng/ml | 1.4E2 | 2.0E2 | 2.9E2 | 3.5E2 | 5.7E2 | 4.9E2 | 4.7E0 | 4.3E1 | 3.8E3 | 2.5E3 | 110 | 43 | 83 | 43 | 0.58 |
| Ri | ng/ml | 1.0E-9 | 1.0E-9 | 3.9E0 | 3.5E0 | 9.2E0 | 8.0E0 | 1.0E-9 | 1.0E-9 | 7.4E1 | 4.5E1 | 111 | 43 | 84 | 43 | 0.46 |
| Rg | ng/ml | 1.0E-9 | 1.0E-9 | 2.7E-2 | 2.0E-2 | 2.6E-1 | 1.0E-1 | 1.0E-9 | 1.0E-9 | 2.7E0 | 6.8E-1 | 110 | 43 | 83 | 43 | 0.56 |
| Rj | ng/ml | 1.0E-9 | 1.0E-9 | 1.7E0 | 1.3E0 | 7.9E0 | 3.8E0 | 1.0E-9 | 1.0E-9 | 8.0E1 | 2.4E1 | 111 | 43 | 84 | 43 | 0.53 |
| Rf | ng/ml | 3.6E-1 | 5.7E-1 | 8.3E-1 | 1.4E0 | 1.6E0 | 2.4E0 | 7.8E-3 | 1.8E-2 | 1.4E1 | 1.2E1 | 110 | 43 | 83 | 43 | 0.62 |
| Ql | pg/ml | 7.3E0 | 7.8E0 | 1.4E1 | 2.0E1 | 3.1E1 | 3.7E1 | 1.0E-9 | 1.0E-9 | 2.9E2 | 1.8E2 | 111 | 44 | 86 | 44 | 0.54 |
| Qm | pg/ml | 1.7E0 | 1.6E1 | 1.9E1 | 2.6E1 | 4.4E1 | 3.7E1 | 1.0E-9 | 1.0E-9 | 3.6E2 | 1.7E2 | 111 | 44 | 86 | 44 | 0.61 |
| Qn | pg/ml | 6.1E-1 | 1.4E0 | 7.5E0 | 1.0E1 | 2.6E1 | 3.7E1 | 1.0E-9 | 1.0E-9 | 2.2E2 | 2.4E2 | 111 | 44 | 86 | 44 | 0.55 |
| Nv | pg/ml | 3.5E3 | 6.2E3 | 1.3E4 | 1.5E4 | 6.3E4 | 2.2E4 | 1.0E-9 | 4.9E2 | 1.1E6 | 1.1E5 | 318 | 66 | 136 | 66 | 0.66 |
| Nw | pg/ml | 7.4E3 | 1.6E4 | 1.2E4 | 1.8E4 | 2.2E4 | 1.8E4 | 8.6E1 | 2.6E3 | 2.1E5 | 1.4E5 | 318 | 66 | 136 | 66 | 0.71 |
| Nx | pg/ml | 1.7E2 | 2.6E2 | 3.9E2 | 6.5E2 | 7.1E2 | 7.6E2 | 1.0E-9 | 1.0E-9 | 7.4E3 | 2.6E3 | 318 | 66 | 136 | 66 | 0.65 |
| Ny | pg/ml | 4.7E0 | 1.0E1 | 1.1E2 | 3.2E1 | 1.4E3 | 9.2E1 | 1.0E-9 | 1.0E-9 | 2.5E4 | 6.2E2 | 318 | 66 | 136 | 66 | 0.60 |
| Oa | pg/ml | 1.6E2 | 2.8E2 | 4.4E2 | 4.4E2 | 8.0E2 | 5.5E2 | 1.0E-9 | 1.0E-9 | 4.8E3 | 2.4E3 | 111 | 44 | 86 | 44 | 0.56 |
| Op | pg/ml | 3.3E5 | 4.0E5 | 3.7E5 | 4.2E5 | 1.6E5 | 1.5E5 | 3.3E4 | 1.1E5 | 7.3E5 | 6.6E5 | 45 | 15 | 35 | 15 | 0.61 |
| Wn | ng/ml | 1.2E1 | 2.4E1 | 9.7E1 | 7.5E1 | 3.1E2 | 1.3E2 | 2.5E0 | 4.4E0 | 1.8E3 | 4.9E2 | 33 | 17 | 27 | 17 | 0.63 |
| Tk | ng/ml | 2.0E2 | 1.8E2 | 4.3E2 | 2.7E2 | 7.4E2 | 2.7E2 | 2.4E1 | 3.0E1 | 4.2E3 | 1.2E3 | 36 | 16 | 29 | 16 | 0.49 |
| Oe | pg/ml | 9.7E1 | 5.1E1 | 2.7E2 | 3.6E2 | 4.2E2 | 8.1E2 | 1.0E-9 | 1.0E-9 | 2.7E3 | 5.1E3 | 314 | 66 | 136 | 66 | 0.49 |
| Of | pg/ml | 2.1E2 | 5.7E2 | 5.6E3 | 1.6E4 | 1.8E4 | 7.7E4 | 1.0E-9 | 1.0E-9 | 1.8E5 | 6.2E5 | 317 | 66 | 136 | 66 | 0.55 |
| Og | pg/ml | 9.4E-2 | 1.1E-1 | 7.2E-1 | 1.9E0 | 2.2E0 | 9.8E0 | 1.0E-9 | 1.0E-9 | 2.0E1 | 7.9E1 | 317 | 66 | 136 | 66 | 0.54 |
| Oh | pg/ml | 2.0E0 | 3.8E0 | 2.5E1 | 1.4E1 | 1.2E2 | 4.2E1 | 1.0E-9 | 9.0E-2 | 1.4E3 | 2.2E2 | 317 | 66 | 136 | 66 | 0.63 |
| Oi | pg/ml | 2.9E0 | 5.2E0 | 7.2E0 | 9.7E0 | 1.1E1 | 1.1E1 | 1.0E-9 | 1.0E-9 | 8.6E1 | 4.2E1 | 317 | 66 | 136 | 66 | 0.59 |
| Ok | pg/ml | 3.2E2 | 7.2E2 | 4.3E2 | 9.6E2 | 3.9E2 | 1.2E3 | 1.3E1 | 7.1E1 | 3.1E3 | 9.8E3 | 317 | 66 | 136 | 66 | 0.75 |
| Om | pg/ml | 3.6E2 | 6.6E2 | 7.6E2 | 1.8E3 | 2.1E3 | 4.9E3 | 1.0E-9 | 1.0E-9 | 3.0E4 | 3.6E4 | 317 | 66 | 136 | 66 | 0.67 |
| On | pg/ml | 1.4E2 | 2.7E2 | 2.8E2 | 5.9E2 | 4.8E2 | 1.8E3 | 8.4E-1 | 2.7E1 | 4.5E3 | 1.5E4 | 317 | 66 | 136 | 66 | 0.68 |
| Or | pg/ml | 1.0E1 | 1.4E1 | 3.1E1 | 4.9E1 | 6.4E1 | 1.3E2 | 1.0E-9 | 1.0E-9 | 5.0E2 | 7.6E2 | 110 | 44 | 86 | 44 | 0.51 |
| Ow | pg/ml | 2.8E1 | 4.8E1 | 1.0E2 | 9.7E1 | 3.0E2 | 1.4E2 | 1.0E-9 | 1.0E-9 | 2.3E3 | 7.7E2 | 110 | 44 | 86 | 44 | 0.63 |
| Ou | pg/ml | 4.4E2 | 6.8E2 | 8.4E2 | 1.8E3 | 1.2E3 | 2.7E3 | 1.0E-9 | 1.0E-9 | 9.4E3 | 9.6E3 | 110 | 44 | 86 | 44 | 0.58 |
| Ul | ng/ml | 1.0E-9 | 1.0E-9 | 1.1E0 | 1.1E0 | 3.7E0 | 5.3E0 | 1.0E-9 | 1.0E-9 | 2.2E1 | 3.4E1 | 119 | 44 | 88 | 44 | 0.48 |
| Uo | ng/ml | 1.0E-9 | 1.0E-9 | 1.1E-1 | 7.0E-2 | 2.4E-1 | 1.8E-1 | 1.0E-9 | 1.0E-9 | 1.3E0 | 8.5E-1 | 119 | 44 | 88 | 44 | 0.46 |
| Uk | ng/ml | 1.0E-9 | 1.0E-9 | 4.8E-3 | 1.1E-2 | 1.2E-2 | 2.2E-2 | 1.0E-9 | 1.0E-9 | 9.9E-2 | 1.1E-1 | 119 | 44 | 88 | 44 | 0.53 |
| Um | ng/ml | 1.0E-9 | 1.0E-9 | 3.8E-1 | 1.7E-1 | 9.8E-1 | 3.2E-1 | 1.0E-9 | 1.0E-9 | 6.8E0 | 1.3E0 | 119 | 44 | 88 | 44 | 0.48 |
| Uf | ng/ml | 4.5E-2 | 1.1E-1 | 9.4E-2 | 1.9E-1 | 1.2E-1 | 3.7E-1 | 2.8E-3 | 8.3E-3 | 6.6E-1 | 2.5E0 | 119 | 44 | 88 | 44 | 0.69 |
| Uh | ng/ml | 1.8E0 | 3.0E0 | 2.5E0 | 4.6E0 | 2.3E0 | 4.5E0 | 3.2E-2 | 3.0E-1 | 1.1E1 | 1.7E1 | 119 | 44 | 88 | 44 | 0.65 |
| Un | ng/ml | 1.6E0 | 2.6E0 | 1.9E0 | 2.8E0 | 1.2E0 | 1.2E0 | 2.0E-1 | 7.4E-1 | 7.0E0 | 5.6E0 | 119 | 44 | 88 | 44 | 0.70 |
| Ug | ng/ml | 1.4E1 | 2.5E1 | 2.7E1 | 4.0E1 | 2.7E1 | 4.6E1 | 6.9E-1 | 1.1E0 | 1.3E2 | 2.1E2 | 119 | 44 | 88 | 44 | 0.56 |
| Ur | ng/ml | 1.6E-1 | 1.1E-1 | 1.2E0 | 5.7E-1 | 8.6E0 | 1.3E0 | 1.0E-9 | 1.0E-9 | 9.3E1 | 7.2E0 | 119 | 44 | 88 | 44 | 0.45 |
| Up | ng/ml | 1.0E-9 | 1.0E-9 | 7.5E-3 | 6.4E-3 | 3.4E-2 | 1.8E-2 | 1.0E-9 | 1.0E-9 | 3.3E-1 | 9.5E-2 | 119 | 44 | 88 | 44 | 0.53 |
| Us | ng/ml | 4.4E-3 | 5.9E-3 | 2.1E-2 | 1.7E-2 | 5.6E-2 | 2.5E-2 | 1.0E-9 | 1.0E-9 | 4.0E-1 | 1.0E-1 | 119 | 44 | 88 | 44 | 0.51 |
| Uv | ng/ml | 3.2E-3 | 3.9E-3 | 1.2E-2 | 1.0E-2 | 3.1E-2 | 2.2E-2 | 1.0E-9 | 1.0E-9 | 2.3E-1 | 1.3E-1 | 119 | 44 | 88 | 44 | 0.52 |
| Ut | ng/ml | 5.3E-1 | 9.5E-1 | 2.3E0 | 2.2E0 | 8.9E0 | 2.9E0 | 1.0E-9 | 1.0E-9 | 7.2E1 | 1.2E1 | 119 | 44 | 88 | 44 | 0.62 |
| Uu | ng/ml | 6.9E0 | 8.4E0 | 7.8E0 | 9.5E0 | 5.1E0 | 5.7E0 | 8.1E-1 | 6.0E-1 | 2.6E1 | 2.2E1 | 119 | 44 | 88 | 44 | 0.59 |
| Uw | ng/ml | 1.8E0 | 1.9E0 | 2.9E0 | 3.0E0 | 5.2E0 | 2.3E0 | 1.0E-9 | 2.0E-1 | 3.7E1 | 7.1E0 | 53 | 15 | 42 | 15 | 0.59 |
| Vb | ng/ml | 1.0E0 | 9.6E-1 | 1.0E0 | 1.0E0 | 4.2E-1 | 4.4E-1 | 2.1E-1 | 4.2E-1 | 2.5E0 | 1.8E0 | 53 | 15 | 42 | 15 | 0.46 |
| Vc | ng/ml | 1.0E-9 | 1.0E-9 | 2.3E-3 | 1.0E-9 | 1.5E-2 | 0.0E0 | 1.0E-9 | 1.0E-9 | 1.1E-1 | 1.0E-9 | 53 | 15 | 42 | 15 | 0.47 |
| Uy | ng/ml | 1.0E0 | 9.5E-1 | 2.9E0 | 2.5E0 | 6.6E0 | 4.1E0 | 5.3E-2 | 3.1E-2 | 3.5E1 | 1.6E1 | 53 | 15 | 42 | 15 | 0.46 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Uz | ng/ml | 1.0E-9 | 1.0E-9 | 8.3E-3 | 1.0E-9 | 6.0E-2 | 0.0E0 | 1.0E-9 | 1.0E-9 | 4.4E-1 | 1.0E-9 | 53 | 15 | 42 | 15 | 0.48 |
| Ux | ng/ml | 1.2E2 | 2.5E2 | 1.6E2 | 2.5E2 | 1.3E2 | 1.6E2 | 5.8E0 | 5.8E1 | 4.6E2 | 5.3E2 | 53 | 15 | 42 | 15 | 0.68 |
| Va | ng/ml | 1.6E1 | 1.4E1 | 2.3E1 | 2.6E1 | 2.5E1 | 3.2E1 | 3.1E-1 | 1.5E0 | 1.2E2 | 1.2E2 | 53 | 15 | 42 | 15 | 0.49 |
| Vh | ng/ml | 4.4E-3 | 1.1E-2 | 1.1E-2 | 1.8E-2 | 1.9E-2 | 2.1E-2 | 1.0E-9 | 5.2E-4 | 1.2E-1 | 7.6E-2 | 53 | 15 | 42 | 15 | 0.63 |
| Vi | ng/ml | 2.4E-3 | 3.9E-3 | 2.6E-1 | 1.4E-2 | 1.9E0 | 2.3E-2 | 1.0E-9 | 9.0E-5 | 1.4E1 | 7.5E-2 | 53 | 15 | 42 | 15 | 0.58 |
| Vj | ng/ml | 2.2E1 | 5.7E1 | 1.8E2 | 6.6E2 | 7.4E2 | 2.2E3 | 3.2E0 | 9.7E0 | 5.2E3 | 8.4E3 | 53 | 14 | 42 | 14 | 0.67 |
| Vp | ng/ml | 1.0E-9 | 1.0E-9 | 7.4E-1 | 1.1E-2 | 5.2E0 | 4.5E-2 | 1.0E-9 | 1.0E-9 | 5.0E1 | 2.6E-1 | 119 | 44 | 88 | 44 | 0.44 |
| Vt | ng/ml | 6.1E0 | 7.9E0 | 7.6E0 | 1.1E1 | 6.1E0 | 1.2E1 | 6.0E-1 | 1.1E0 | 3.2E1 | 6.5E1 | 119 | 44 | 88 | 44 | 0.60 |
| Vu | ng/ml | 1.0E-9 | 1.0E-9 | 2.3E0 | 2.0E0 | 6.0E0 | 4.1E0 | 1.0E-9 | 1.0E-9 | 5.1E1 | 2.1E1 | 118 | 43 | 88 | 43 | 0.50 |
| Vq | ng/ml | 1.2E2 | 5.8E2 | 5.5E2 | 1.3E3 | 1.2E3 | 2.3E3 | 2.0E-1 | 9.0E-1 | 1.0E4 | 1.1E4 | 94 | 28 | 72 | 28 | 0.63 |
| Vo | ng/ml | 2.6E1 | 2.6E1 | 2.5E1 | 2.5E1 | 4.9E0 | 3.2E0 | 2.5E0 | 1.5E1 | 3.5E1 | 3.4E1 | 119 | 44 | 88 | 44 | 0.52 |
| Vs | ng/ml | 1.0E-9 | 1.4E0 | 6.5E0 | 4.5E0 | 2.7E1 | 8.5E0 | 1.0E-9 | 1.0E-9 | 2.3E2 | 4.2E1 | 116 | 41 | 86 | 41 | 0.57 |
| Vv | ng/ml | 2.7E0 | 4.5E0 | 6.0E0 | 8.1E0 | 1.0E1 | 1.3E1 | 1.0E-9 | 1.0E-9 | 8.1E1 | 8.0E1 | 118 | 44 | 88 | 44 | 0.59 |
| Vw | ng/ml | 3.3E1 | 4.3E1 | 3.1E1 | 4.4E1 | 1.7E1 | 1.6E1 | 2.5E0 | 1.8E1 | 6.7E1 | 7.0E1 | 53 | 15 | 42 | 15 | 0.70 |
| Oy | pg/ml | 5.5E-1 | 6.8E-1 | 8.7E0 | 7.4E0 | 4.2E1 | 3.4E1 | 1.0E-9 | 1.0E-9 | 4.0E2 | 2.7E2 | 317 | 66 | 136 | 66 | 0.57 |
| Oz | pg/ml | 4.5E-3 | 2.0E-1 | 2.2E-1 | 4.0E-1 | 3.7E-1 | 7.9E-1 | 1.0E-9 | 1.0E-9 | 2.6E0 | 5.8E0 | 317 | 66 | 136 | 66 | 0.61 |
| Pa | pg/ml | 3.4E-1 | 4.2E-1 | 1.3E0 | 5.4E0 | 5.8E0 | 3.6E1 | 1.0E-9 | 1.0E-9 | 8.6E1 | 2.9E2 | 317 | 66 | 136 | 66 | 0.58 |
| Pb | pg/ml | 1.0E-9 | 3.3E-2 | 1.8E0 | 2.9E0 | 2.7E1 | 2.2E1 | 1.0E-9 | 1.0E-9 | 4.9E2 | 1.8E2 | 317 | 66 | 136 | 66 | 0.56 |
| Pc | pg/ml | 4.4E-2 | 3.6E-1 | 4.3E-1 | 6.5E-1 | 1.3E0 | 1.6E0 | 1.0E-9 | 1.0E-9 | 1.6E1 | 1.1E1 | 317 | 66 | 136 | 66 | 0.60 |
| Pd | pg/ml | 1.8E0 | 3.1E0 | 3.8E0 | 5.3E0 | 8.2E0 | 5.7E0 | 1.0E-9 | 1.2E-1 | 9.4E1 | 2.7E1 | 317 | 66 | 136 | 66 | 0.65 |
| Pe | pg/ml | 1.8E1 | 3.6E1 | 8.9E1 | 3.0E2 | 3.5E2 | 1.7E3 | 1.0E-9 | 3.1E0 | 4.7E3 | 1.4E4 | 317 | 66 | 136 | 66 | 0.68 |
| Pf | pg/ml | 1.2E0 | 2.7E0 | 6.2E0 | 1.2E1 | 2.6E1 | 3.4E1 | 1.0E-9 | 8.1E-2 | 3.3E2 | 2.3E2 | 317 | 66 | 136 | 66 | 0.66 |
| Pg | pg/ml | 3.2E0 | 4.7E0 | 4.0E1 | 5.0E1 | 2.1E2 | 2.3E2 | 1.0E-9 | 2.7E-1 | 3.2E3 | 1.9E3 | 317 | 66 | 136 | 66 | 0.58 |
| Ph | ng/ml | 1.4E-1 | 2.5E-1 | 2.7E-1 | 5.7E-1 | 3.5E-1 | 8.9E-1 | 1.0E-9 | 1.0E-9 | 2.2E0 | 4.4E0 | 110 | 44 | 86 | 44 | 0.61 |
| Pi | ng/ml | 1.9E-1 | 2.1E-1 | 2.8E-1 | 2.6E-1 | 4.4E-1 | 2.3E-1 | 1.0E-9 | 1.0E-9 | 3.2E0 | 1.0E0 | 110 | 44 | 86 | 44 | 0.53 |
| Pj | ng/mL | 4.4E0 | 7.9E0 | 5.1E0 | 9.3E0 | 3.3E0 | 6.2E0 | 3.8E-2 | 9.9E-1 | 1.6E1 | 2.5E1 | 110 | 44 | 86 | 44 | 0.70 |
| Pk | ng/ml | 8.1E-3 | 7.1E-3 | 1.1E-2 | 1.0E-2 | 1.2E-2 | 1.3E-2 | 1.0E-9 | 1.0E-9 | 5.9E-2 | 7.0E-2 | 110 | 44 | 86 | 44 | 0.48 |
| aA | mg/dL | 8.0E-1 | 9.2E-1 | 9.2E-1 | 1.1E0 | 4.3E-1 | 7.6E-1 | 3.0E-1 | 2.6E-1 | 4.2E0 | 5.4E0 | 1242 | 95 | 226 | 95 | 0.55 |
| aC | mg/mL | 3.1E0 | 2.5E0 | 3.3E0 | 2.8E0 | 1.4E0 | 1.1E0 | 1.1E0 | 1.3E0 | 8.2E0 | 6.3E0 | 221 | 50 | 91 | 50 | 0.39 |
| aD | ug/mL | 2.9E0 | 4.2E0 | 4.1E0 | 5.3E0 | 3.2E0 | 3.9E0 | 8.5E-1 | 8.4E-1 | 2.8E1 | 2.0E1 | 221 | 50 | 91 | 50 | 0.61 |
| aE | mg/mL | 5.8E-1 | 5.5E-1 | 5.8E-1 | 5.6E-1 | 1.5E-1 | 1.4E-1 | 2.1E-1 | 3.1E-1 | 1.1E0 | 1.2E0 | 221 | 50 | 91 | 50 | 0.44 |
| aF | ng/mL | 2.1E0 | 1.6E0 | 4.4E0 | 3.6E0 | 7.0E0 | 4.5E0 | 4.3E-3 | 4.3E-3 | 5.0E1 | 1.8E1 | 221 | 50 | 91 | 50 | 0.45 |
| aG | mg/mL | 1.3E-1 | 1.4E-1 | 1.5E-1 | 1.5E-1 | 7.8E-2 | 8.4E-2 | 5.0E-2 | 5.8E-2 | 5.0E-1 | 4.0E-1 | 221 | 50 | 91 | 50 | 0.49 |
| aH | ug/mL | 6.8E1 | 8.0E1 | 7.8E1 | 8.8E1 | 3.5E1 | 5.2E1 | 1.5E1 | 1.1E1 | 2.7E2 | 2.6E2 | 221 | 50 | 91 | 50 | 0.54 |
| aI | ug/mL | 1.8E2 | 1.9E2 | 1.9E2 | 1.9E2 | 6.0E1 | 5.5E1 | 5.8E1 | 4.8E1 | 3.7E2 | 2.8E2 | 221 | 50 | 91 | 50 | 0.51 |
| aJ | ug/mL | 2.5E0 | 3.0E0 | 3.0E0 | 3.8E0 | 1.9E0 | 2.9E0 | 9.0E-1 | 1.0E0 | 1.2E1 | 1.5E1 | 221 | 50 | 91 | 50 | 0.59 |
| aK | ng/mL | 1.8E0 | 1.2E0 | 2.7E0 | 1.7E0 | 2.7E0 | 1.5E0 | 8.4E-2 | 2.9E-4 | 1.8E1 | 5.7E0 | 221 | 50 | 91 | 50 | 0.38 |
| aL | mg/mL | 8.1E-1 | 8.0E-1 | 8.3E-1 | 8.0E-1 | 2.4E-1 | 2.4E-1 | 2.2E-1 | 3.0E-1 | 1.6E0 | 1.5E0 | 221 | 50 | 91 | 50 | 0.47 |
| aM | U/mL | 1.9E1 | 3.9E1 | 3.7E1 | 5.8E1 | 1.1E2 | 6.5E1 | 4.2E-2 | 4.2E-2 | 1.6E3 | 4.0E2 | 221 | 50 | 91 | 50 | 0.68 |
| aN | U/mL | 1.0E1 | 1.8E1 | 1.8E1 | 2.6E1 | 2.1E1 | 2.7E1 | 2.5E-3 | 2.5E-3 | 1.3E2 | 1.1E2 | 221 | 50 | 91 | 50 | 0.61 |
| aO | pg/mL | 3.9E1 | 7.3E1 | 3.7E2 | 4.5E2 | 9.9E2 | 8.7E2 | 6.0E-2 | 2.1E0 | 6.6E3 | 3.6E3 | 221 | 50 | 91 | 50 | 0.56 |
| aP | ng/mL | 1.7E0 | 1.7E0 | 1.9E0 | 2.2E0 | 1.1E0 | 1.5E0 | 5.4E-1 | 7.5E-1 | 6.6E0 | 6.5E0 | 221 | 50 | 91 | 50 | 0.54 |
| aQ | ng/mL | 3.2E-1 | 2.2E-1 | 5.0E-1 | 3.4E-1 | 5.2E-1 | 3.1E-1 | 1.9E-2 | 2.5E-2 | 4.0E0 | 1.3E0 | 221 | 50 | 91 | 50 | 0.38 |
| aR | ng/mL | 1.6E0 | 2.3E0 | 2.4E0 | 3.9E0 | 2.5E0 | 4.9E0 | 1.8E-1 | 6.2E-1 | 2.1E1 | 3.0E1 | 221 | 50 | 91 | 50 | 0.64 |
| aS | ng/mL | 2.4E-1 | 3.3E-1 | 7.8E-1 | 5.6E-1 | 2.5E0 | 9.1E-1 | 4.2E-3 | 4.2E-3 | 3.3E1 | 6.1E0 | 221 | 50 | 91 | 50 | 0.55 |
| aU | pg/mL | 8.8E1 | 6.1E1 | 1.4E2 | 8.6E1 | 1.6E2 | 8.7E1 | 7.4E0 | 7.4E-2 | 1.3E3 | 4.8E2 | 221 | 50 | 91 | 50 | 0.37 |
| aV | ng/mL | 7.3E-1 | 4.6E-1 | 1.1E0 | 7.6E-1 | 1.1E0 | 8.5E-1 | 3.1E-2 | 3.8E-2 | 8.7E0 | 4.2E0 | 221 | 50 | 91 | 50 | 0.38 |
| aW | pg/mL | 1.7E1 | 2.3E1 | 2.0E1 | 3.0E1 | 2.6E1 | 6.1E1 | 7.2E-2 | 7.2E-2 | 2.4E2 | 4.5E2 | 221 | 50 | 91 | 50 | 0.61 |
| aX | ng/mL | 1.0E1 | 9.5E0 | 1.3E1 | 1.3E1 | 1.2E1 | 1.1E1 | 3.0E-1 | 1.4E0 | 6.7E1 | 4.6E1 | 221 | 50 | 91 | 50 | 0.49 |
| aY | pg/mL | 5.0E1 | 7.2E1 | 7.2E1 | 9.0E1 | 7.1E1 | 7.0E1 | 4.1E-1 | 4.1E-1 | 4.4E2 | 3.7E2 | 221 | 50 | 91 | 50 | 0.62 |
| aZ | pg/mL | 2.2E2 | 2.4E2 | 4.8E2 | 4.8E2 | 7.2E2 | 9.0E2 | 1.7E0 | 1.7E0 | 5.4E3 | 5.9E3 | 221 | 50 | 91 | 50 | 0.49 |
| bA | ng/mL | 8.0E0 | 1.3E1 | 2.6E1 | 4.7E1 | 6.2E1 | 9.7E1 | 3.0E-2 | 3.0E-2 | 7.1E2 | 6.2E2 | 221 | 50 | 91 | 50 | 0.62 |
| bB | ng/mL | 3.1E2 | 3.0E2 | 3.4E2 | 3.0E2 | 1.6E2 | 1.7E2 | 1.6E1 | 2.3E1 | 1.0E3 | 7.2E2 | 221 | 50 | 91 | 50 | 0.45 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|------|--------|------|------|
| | | NonDis | Dis | NonDis | Dis | NonDis | Dis | NonDis | Dis | NonDis | Dis | NonDis | Dis | NonDis | Dis | |
| bC | ng/mL | 3.5E2 | 4.1E2 | 5.1E2 | 7.8E2 | 5.3E2 | 1.1E3 | 2.7E1 | 4.6E1 | 4.0E3 | 4.7E3 | 221 | 50 | 91 | 50 | 0.57 |
| bE | mg/mL | 5.6E0 | 5.4E0 | 5.9E0 | 6.0E0 | 1.7E0 | 2.3E0 | 1.9E0 | 1.4E0 | 1.3E1 | 1.2E1 | 221 | 50 | 91 | 50 | 0.50 |
| bF | pg/mL | 2.0E1 | 2.6E1 | 8.6E1 | 9.2E1 | 4.4E2 | 3.1E2 | 5.0E-2 | 2.8E0 | 5.0E3 | 2.2E3 | 221 | 50 | 91 | 50 | 0.59 |
| bG | ng/mL | 1.8E0 | 1.3E0 | 3.0E0 | 2.9E0 | 3.7E0 | 3.4E0 | 2.2E-2 | 2.4E-1 | 2.6E1 | 1.5E1 | 221 | 50 | 91 | 50 | 0.45 |
| bH | pg/mL | 5.7E-1 | 5.7E-1 | 5.4E0 | 3.0E0 | 2.0E1 | 5.1E0 | 5.7E-1 | 5.7E-1 | 2.8E2 | 2.2E1 | 221 | 50 | 91 | 50 | 0.45 |
| bI | ng/mL | 4.0E-3 | 4.0E-3 | 6.8E-2 | 5.5E-2 | 1.7E-1 | 1.1E-1 | 4.0E-3 | 4.0E-3 | 1.3E0 | 4.5E-1 | 221 | 50 | 91 | 50 | 0.50 |
| bJ | mg/mL | 2.2E0 | 2.1E0 | 2.5E0 | 2.5E0 | 1.9E0 | 2.0E0 | 2.5E-4 | 2.5E-4 | 1.3E1 | 1.0E1 | 221 | 50 | 91 | 50 | 0.49 |
| bL | pg/mL | 4.1E0 | 2.9E0 | 7.8E0 | 6.2E0 | 9.3E0 | 7.7E0 | 4.6E-2 | 4.6E-2 | 4.9E1 | 2.7E1 | 221 | 50 | 91 | 50 | 0.43 |
| bM | mg/mL | 1.5E0 | 2.2E0 | 1.8E0 | 2.5E0 | 1.2E0 | 1.4E0 | 9.2E-3 | 4.1E-1 | 7.1E0 | 6.3E0 | 221 | 50 | 91 | 50 | 0.67 |
| bN | ng/mL | 3.2E1 | 5.9E1 | 1.1E2 | 1.1E2 | 2.6E2 | 1.8E2 | 9.7E-1 | 5.9E-1 | 1.9E3 | 1.2E3 | 221 | 50 | 91 | 50 | 0.56 |
| bO | ng/mL | 4.0E-2 | 4.0E-2 | 1.1E1 | 1.0E1 | 2.4E1 | 2.9E1 | 4.0E-2 | 4.0E-2 | 1.9E2 | 1.9E2 | 221 | 50 | 91 | 50 | 0.44 |
| bP | mg/mL | 5.6E-1 | 5.0E-1 | 7.9E-1 | 7.4E-1 | 6.6E-1 | 6.9E-1 | 8.2E-2 | 1.3E-1 | 3.8E0 | 3.1E0 | 221 | 50 | 91 | 50 | 0.47 |
| bQ | pg/mL | 1.4E1 | 1.8E1 | 2.4E1 | 2.1E1 | 3.5E1 | 1.6E1 | 1.5E-1 | 1.5E-1 | 3.2E2 | 6.6E1 | 221 | 50 | 91 | 50 | 0.53 |
| bR | ng/mL | 1.2E-2 | 1.2E-2 | 1.4E-1 | 7.0E-2 | 2.8E-1 | 9.7E-2 | 1.2E-2 | 1.2E-2 | 3.4E0 | 3.9E-1 | 221 | 50 | 91 | 50 | 0.42 |
| bS | pg/mL | 9.4E-1 | 9.4E-1 | 8.1E0 | 4.4E0 | 2.9E1 | 1.1E1 | 9.4E-1 | 9.4E-1 | 3.4E2 | 5.9E1 | 221 | 50 | 91 | 50 | 0.48 |
| bU | ng/mL | 1.6E-1 | 1.3E-2 | 2.1E-1 | 1.0E-1 | 2.6E-1 | 1.3E-1 | 1.3E-2 | 1.3E-2 | 1.8E0 | 5.7E-1 | 221 | 50 | 91 | 50 | 0.36 |
| bV | pg/mL | 4.7E2 | 4.7E2 | 5.3E2 | 6.0E2 | 2.5E2 | 4.4E2 | 1.7E2 | 2.5E2 | 1.6E3 | 3.1E3 | 221 | 50 | 91 | 50 | 0.53 |
| bW | pg/mL | 3.5E2 | 3.9E2 | 4.7E2 | 6.1E2 | 4.2E2 | 9.1E2 | 1.1E2 | 1.2E2 | 3.4E3 | 6.4E3 | 221 | 50 | 91 | 50 | 0.54 |
| bX | ng/mL | 3.1E-3 | 2.5E-5 | 3.2E-3 | 1.9E-3 | 3.8E-3 | 2.6E-3 | 2.5E-5 | 2.5E-5 | 2.0E-2 | 8.8E-3 | 221 | 50 | 91 | 50 | 0.41 |
| bZ | pg/mL | 2.5E2 | 2.5E2 | 7.5E2 | 5.0E2 | 3.2E3 | 8.4E2 | 1.5E-1 | 2.4E1 | 4.4E4 | 5.6E3 | 221 | 50 | 91 | 50 | 0.51 |
| cA | pg/mL | 6.0E-1 | 6.0E-1 | 1.9E0 | 1.3E0 | 3.4E0 | 2.3E0 | 6.0E-1 | 6.0E-1 | 1.4E1 | 1.0E1 | 221 | 50 | 91 | 50 | 0.47 |
| cB | ng/mL | 6.0E-2 | 3.4E-2 | 9.0E-2 | 5.8E-2 | 1.0E-1 | 7.2E-2 | 1.7E-3 | 1.7E-3 | 5.4E-1 | 3.0E-1 | 221 | 50 | 91 | 50 | 0.39 |
| cC | pg/mL | 4.6E1 | 3.3E1 | 4.9E1 | 3.3E1 | 3.9E1 | 2.7E1 | 1.0E0 | 1.0E0 | 3.7E2 | 1.1E2 | 221 | 50 | 91 | 50 | 0.37 |
| cD | pg/mL | 6.1E0 | 4.0E0 | 1.3E1 | 1.2E1 | 4.0E1 | 2.3E1 | 3.3E-1 | 3.3E-1 | 4.8E2 | 1.4E2 | 221 | 50 | 91 | 50 | 0.43 |
| cE | pg/mL | 3.1E1 | 4.2E1 | 9.3E1 | 1.4E2 | 2.7E2 | 4.4E2 | 1.5E0 | 1.2E-1 | 3.1E3 | 3.1E3 | 221 | 50 | 91 | 50 | 0.56 |
| cF | pg/mL | 1.3E1 | 9.4E0 | 2.4E1 | 1.2E1 | 3.6E1 | 1.5E1 | 5.3E-1 | 5.3E-1 | 2.2E2 | 7.2E1 | 221 | 50 | 91 | 50 | 0.42 |
| cG | pg/mL | 4.2E1 | 5.6E1 | 6.4E1 | 1.2E2 | 8.8E1 | 2.4E2 | 1.1E1 | 1.5E1 | 1.1E3 | 1.6E3 | 221 | 50 | 91 | 50 | 0.63 |
| cH | uIU/mL | 2.9E0 | 3.7E0 | 6.0E0 | 7.1E0 | 8.9E0 | 1.1E1 | 8.6E-3 | 8.6E-3 | 8.7E1 | 5.2E1 | 221 | 50 | 91 | 50 | 0.51 |
| cI | ng/mL | 6.0E0 | 4.7E0 | 1.1E1 | 1.4E1 | 1.3E1 | 2.3E1 | 1.0E-3 | 8.0E-2 | 9.4E1 | 1.2E2 | 221 | 50 | 91 | 50 | 0.47 |
| cJ | ug/mL | 7.2E1 | 4.9E1 | 1.2E2 | 9.5E1 | 1.5E2 | 1.4E2 | 4.0E0 | 1.1E1 | 9.6E2 | 6.9E2 | 221 | 50 | 91 | 50 | 0.42 |
| cK | ng/mL | 3.8E-3 | 3.8E-3 | 7.5E-2 | 8.4E-3 | 2.2E-1 | 2.4E-2 | 3.8E-3 | 3.8E-3 | 1.7E0 | 1.6E-1 | 221 | 50 | 91 | 50 | 0.42 |
| cL | pg/mL | 1.9E2 | 2.0E2 | 2.7E2 | 3.7E2 | 5.1E2 | 8.3E2 | 2.5E1 | 1.6E1 | 7.1E3 | 5.8E3 | 221 | 50 | 91 | 50 | 0.53 |
| cM | pg/mL | 2.9E2 | 2.6E2 | 3.2E2 | 2.9E2 | 1.8E2 | 2.1E2 | 3.7E1 | 3.3E1 | 1.2E3 | 1.5E3 | 221 | 50 | 91 | 50 | 0.43 |
| cN | pg/mL | 1.2E2 | 1.2E2 | 1.2E2 | 1.4E2 | 4.4E1 | 4.8E1 | 3.8E1 | 4.3E1 | 2.7E2 | 2.7E2 | 221 | 50 | 91 | 50 | 0.57 |
| cO | pg/mL | 2.3E2 | 2.4E2 | 2.8E2 | 2.5E2 | 2.0E2 | 1.1E2 | 5.4E1 | 1.0E2 | 1.7E3 | 6.3E2 | 221 | 50 | 91 | 50 | 0.50 |
| cP | ng/mL | 2.4E3 | 2.9E3 | 2.5E3 | 3.0E3 | 8.9E2 | 9.9E2 | 6.2E2 | 1.5E3 | 5.7E3 | 5.9E3 | 221 | 50 | 91 | 50 | 0.65 |
| cQ | ng/mL | 4.0E-2 | 6.4E-2 | 1.1E-1 | 1.4E-1 | 1.8E-1 | 2.5E-1 | 2.0E-3 | 2.0E-3 | 1.2E0 | 1.4E0 | 221 | 50 | 91 | 50 | 0.57 |
| cR | ng/mL | 2.8E2 | 3.8E2 | 4.3E2 | 6.7E2 | 4.6E2 | 1.1E3 | 2.3E1 | 3.0E1 | 3.8E3 | 7.7E3 | 221 | 50 | 91 | 50 | 0.59 |
| cS | ng/mL | 2.4E2 | 2.9E2 | 3.9E2 | 4.5E2 | 4.2E2 | 5.2E2 | 5.3E1 | 7.0E1 | 2.7E3 | 2.6E3 | 221 | 50 | 91 | 50 | 0.55 |
| cT | ng/mL | 2.8E1 | 3.7E1 | 7.4E1 | 1.0E2 | 1.5E2 | 1.6E2 | 4.6E0 | 6.9E0 | 1.7E3 | 7.4E2 | 221 | 50 | 91 | 50 | 0.58 |
| cU | ng/mL | 5.5E1 | 5.8E1 | 7.4E1 | 7.2E1 | 7.2E1 | 5.3E1 | 1.0E1 | 1.4E1 | 7.7E2 | 2.7E2 | 221 | 50 | 91 | 50 | 0.51 |
| cV | ng/mL | 1.7E-1 | 1.9E-1 | 4.9E-1 | 2.8E-1 | 3.2E0 | 3.1E-1 | 3.4E-4 | 3.7E-2 | 4.7E1 | 2.0E0 | 221 | 50 | 91 | 50 | 0.53 |
| cW | mIU/mL | 4.6E-2 | 5.5E-2 | 2.2E-1 | 8.1E-2 | 1.0E0 | 7.1E-2 | 3.7E-4 | 1.0E-2 | 9.7E0 | 2.9E-1 | 221 | 50 | 91 | 50 | 0.56 |
| cX | ng/mL | 1.1E-1 | 1.8E-1 | 1.8E0 | 8.7E-1 | 5.4E0 | 1.6E0 | 2.3E-4 | 2.3E-4 | 2.8E1 | 8.5E0 | 221 | 50 | 91 | 50 | 0.52 |
| cY | ng/mL | 1.0E1 | 7.2E0 | 1.4E1 | 9.8E0 | 1.4E1 | 8.4E0 | 4.4E-1 | 1.5E-1 | 8.3E1 | 3.2E1 | 221 | 50 | 91 | 50 | 0.39 |
| cZ | ug/mL | 1.4E1 | 1.5E1 | 1.5E1 | 1.6E1 | 5.8E0 | 7.6E0 | 5.3E0 | 3.1E0 | 3.9E1 | 3.7E1 | 221 | 50 | 91 | 50 | 0.52 |
| dA | pg/mL | 3.3E2 | 3.3E2 | 3.6E2 | 3.6E2 | 1.6E2 | 1.6E2 | 9.0E1 | 1.3E2 | 1.3E3 | 8.2E2 | 221 | 50 | 91 | 50 | 0.51 |
| dB | ug/mL | 1.7E1 | 1.6E1 | 1.9E1 | 1.5E1 | 2.1E1 | 1.0E1 | 1.9E0 | 1.8E0 | 2.5E2 | 4.1E1 | 221 | 50 | 91 | 50 | 0.44 |
| dC | nmol/L | 3.4E1 | 3.7E1 | 4.1E1 | 3.9E1 | 2.0E1 | 1.5E1 | 9.1E0 | 1.9E1 | 1.4E2 | 8.3E1 | 221 | 50 | 91 | 50 | 0.50 |
| dD | ug/mL | 3.7E1 | 3.4E1 | 3.8E1 | 3.6E1 | 1.0E1 | 1.1E1 | 1.3E1 | 1.3E1 | 7.6E1 | 5.7E1 | 221 | 50 | 91 | 50 | 0.44 |
| dE | ng/mL | 4.8E-1 | 2.7E-1 | 6.7E-1 | 4.6E-1 | 8.3E-1 | 6.4E-1 | 8.4E-3 | 8.4E-3 | 7.2E0 | 3.3E0 | 221 | 50 | 91 | 50 | 0.40 |
| dF | ng/mL | 2.1E2 | 2.6E2 | 2.6E2 | 3.1E2 | 1.8E2 | 1.8E2 | 7.5E1 | 1.0E2 | 1.2E3 | 9.7E2 | 221 | 50 | 91 | 50 | 0.60 |
| dG | ng/mL | 1.1E1 | 1.4E1 | 1.3E1 | 1.6E1 | 8.4E0 | 7.3E0 | 3.1E0 | 6.4E0 | 6.4E1 | 3.4E1 | 221 | 50 | 91 | 50 | 0.61 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dH | pg/mL | 7.5E0 | 7.9E0 | 1.2E1 | 9.9E0 | 2.4E1 | 8.6E0 | 4.0E-2 | 4.0E-2 | 3.1E2 | 5.0E1 | 221 | 50 | 91 | 50 | 0.52 |
| dI | pg/mL | 4.6E-1 | 4.6E-1 | 1.9E0 | 7.9E-1 | 4.4E0 | 1.1E0 | 4.6E-1 | 4.6E-1 | 3.4E1 | 6.0E0 | 221 | 50 | 91 | 50 | 0.44 |
| dJ | ng/mL | 1.9E0 | 1.9E0 | 2.1E0 | 2.0E0 | 1.1E0 | 1.1E0 | 3.2E-2 | 4.0E-1 | 5.9E0 | 5.1E0 | 221 | 50 | 91 | 50 | 0.48 |
| dK | uIU/mL | 2.0E0 | 1.6E0 | 2.7E0 | 2.4E0 | 2.9E0 | 3.2E0 | 2.8E-4 | 3.8E-2 | 1.6E1 | 2.1E1 | 221 | 50 | 91 | 50 | 0.46 |
| dL | ng/mL | 8.8E2 | 1.0E3 | 1.0E3 | 1.1E3 | 4.8E2 | 5.2E2 | 3.4E2 | 4.7E2 | 3.4E3 | 3.3E3 | 221 | 50 | 91 | 50 | 0.60 |
| dM | pg/mL | 9.7E2 | 1.1E3 | 1.2E3 | 1.4E3 | 1.1E3 | 1.2E3 | 4.4E2 | 4.2E2 | 1.2E4 | 8.3E3 | 221 | 50 | 91 | 50 | 0.54 |
| dN | ug/mL | 9.0E1 | 1.0E2 | 9.7E1 | 1.0E2 | 3.4E1 | 3.8E1 | 2.5E1 | 3.7E1 | 2.4E2 | 2.1E2 | 221 | 50 | 91 | 50 | 0.58 |
| dO | ng/ml | 2.8E1 | 9.0E0 | 4.9E1 | 2.0E1 | 6.6E1 | 2.5E1 | 6.1E0 | 3.8E0 | 3.7E2 | 7.9E1 | 44 | 13 | 29 | 13 | 0.25 |
| dR | pg/ml | 1.6E3 | 1.6E3 | 2.5E3 | 1.9E3 | 2.6E3 | 1.7E3 | 1.9E2 | 1.7E2 | 1.5E4 | 8.1E3 | 131 | 47 | 88 | 47 | 0.44 |
| dQ | Absorbance | 5.2E-1 | 2.1E-1 | 6.2E-1 | 2.7E-1 | 5.0E-1 | 1.7E-1 | 7.9E-2 | 1.2E-1 | 2.5E0 | 6.5E-1 | 28 | 7 | 18 | 7 | 0.24 |
| dU | pg/ml | 8.8E3 | 1.8E4 | 1.3E4 | 2.1E4 | 1.2E4 | 1.2E4 | 2.5E3 | 6.7E3 | 5.3E4 | 3.8E4 | 28 | 7 | 26 | 7 | 0.76 |
| dV | pg/ml | 7.2E1 | 6.8E1 | 9.0E1 | 1.7E2 | 5.5E1 | 2.6E2 | 3.4E1 | 3.6E1 | 2.7E2 | 8.9E2 | 35 | 10 | 18 | 10 | 0.49 |
| dX | ng/ml | 3.4E-2 | 7.4E-3 | 9.5E-2 | 1.8E-1 | 1.5E-1 | 3.5E-1 | 2.6E-3 | 2.6E-3 | 7.4E-1 | 9.4E-1 | 65 | 11 | 21 | 11 | 0.43 |
| eF | ng/ml | 4.0E0 | 4.6E0 | 4.6E0 | 5.2E0 | 2.6E0 | 2.3E0 | 1.5E0 | 2.0E0 | 1.8E1 | 1.2E1 | 138 | 48 | 88 | 48 | 0.61 |
| eC | pg/ml | 3.1E2 | 3.0E2 | 3.8E2 | 3.2E2 | 2.5E2 | 1.4E2 | 4.5E1 | 5.1E1 | 1.4E3 | 6.2E2 | 100 | 39 | 86 | 39 | 0.45 |
| eD | pg/ml | 2.3E2 | 2.3E2 | 7.7E2 | 5.0E2 | 1.4E3 | 1.0E3 | 5.2E-1 | 5.2E-1 | 6.8E3 | 5.5E3 | 77 | 29 | 64 | 29 | 0.51 |
| cM | ng/ml | 3.6E0 | 4.1E0 | 4.4E0 | 6.7E0 | 3.5E0 | 6.2E0 | 8.1E-1 | 9.0E-1 | 2.2E1 | 2.5E1 | 87 | 18 | 34 | 18 | 0.61 |
| eP | ng/ml | 3.7E-3 | 1.2E-1 | 1.0E0 | 6.3E0 | 2.1E0 | 1.9E1 | 3.7E-3 | 3.7E-3 | 1.2E1 | 6.5E1 | 65 | 11 | 21 | 11 | 0.53 |
| eT | ng/ml | 2.7E2 | 2.7E2 | 5.6E2 | 4.5E2 | 6.1E2 | 4.8E2 | 1.0E2 | 1.6E2 | 2.5E3 | 1.9E3 | 51 | 13 | 49 | 13 | 0.54 |
| eQ | pg/ml | 1.0E0 | 1.0E0 | 1.1E2 | 6.5E0 | 3.2E2 | 1.5E1 | 1.0E0 | 1.0E0 | 1.6E3 | 4.0E1 | 28 | 7 | 26 | 7 | 0.40 |
| fA | ng/ml | 2.2E2 | 1.5E2 | 4.0E2 | 2.1E2 | 4.6E2 | 1.5E2 | 2.6E1 | 6.8E1 | 1.5E3 | 4.7E2 | 28 | 7 | 26 | 7 | 0.46 |
| eZ | ng/ml | 6.1E1 | 5.4E1 | 6.4E1 | 5.8E1 | 2.6E1 | 2.4E1 | 2.3E1 | 2.5E1 | 1.2E2 | 1.1E2 | 51 | 13 | 49 | 13 | 0.45 |
| fB | ng/ml | 6.1E2 | 6.5E2 | 6.8E2 | 8.7E2 | 2.8E2 | 4.2E2 | 1.6E2 | 4.4E2 | 1.3E3 | 1.5E3 | 28 | 7 | 26 | 7 | 0.61 |
| cX | ng/ml | 6.0E0 | 3.3E0 | 1.9E1 | 1.2E1 | 2.3E1 | 1.5E1 | 8.6E-4 | 3.3E-1 | 7.3E1 | 4.9E1 | 44 | 13 | 29 | 13 | 0.44 |
| fN | ng/ml | 2.1E-1 | 2.1E-1 | 2.7E0 | 1.7E0 | 5.8E0 | 3.1E0 | 2.1E-1 | 2.1E-1 | 3.2E1 | 8.9E0 | 51 | 13 | 49 | 13 | 0.45 |
| fP | ng/ml | 2.3E2 | 2.9E2 | 2.7E2 | 3.1E2 | 1.7E2 | 1.6E2 | 8.4E0 | 4.8E1 | 1.0E3 | 8.6E2 | 127 | 43 | 88 | 43 | 0.60 |
| fR | ng/ml | 1.3E5 | 1.6E5 | 1.6E5 | 2.3E5 | 1.1E5 | 1.7E5 | 3.1E4 | 3.6E4 | 6.1E5 | 6.9E5 | 162 | 28 | 44 | 28 | 0.62 |
| fY | ng/ml | 2.5E2 | 2.7E2 | 2.4E2 | 2.6E2 | 8.9E1 | 1.4E2 | 6.5E1 | 4.2E1 | 4.2E2 | 4.5E2 | 51 | 13 | 49 | 13 | 0.54 |
| gC | ng/ml | 2.3E2 | 2.3E2 | 2.5E2 | 2.7E2 | 9.4E1 | 1.2E2 | 1.2E2 | 1.3E2 | 6.4E2 | 5.9E2 | 71 | 20 | 39 | 20 | 0.52 |
| gN | U/ml | 3.5E2 | 4.7E2 | 4.8E2 | 5.2E2 | 3.2E2 | 2.9E2 | 3.5E1 | 1.2E2 | 1.3E3 | 9.2E2 | 35 | 10 | 18 | 10 | 0.57 |
| gL | pg/ml | 6.1E4 | 6.9E4 | 6.8E4 | 7.7E4 | 2.9E4 | 3.0E4 | 2.2E4 | 3.8E4 | 1.8E5 | 1.6E5 | 131 | 47 | 88 | 47 | 0.59 |
| gP | U/ml | 2.5E2 | 2.8E2 | 2.6E2 | 2.8E2 | 7.9E1 | 1.1E2 | 8.5E1 | 8.6E1 | 5.3E2 | 6.5E2 | 137 | 48 | 88 | 48 | 0.57 |
| gT | ng/ml | 2.0E1 | 2.3E1 | 2.0E1 | 2.4E1 | 4.1E0 | 5.1E0 | 1.2E1 | 1.7E1 | 2.9E1 | 3.2E1 | 36 | 10 | 22 | 10 | 0.68 |
| gW | ng/ml | 7.0E2 | 4.1E2 | 1.5E3 | 7.9E2 | 1.9E3 | 9.5E2 | 3.7E1 | 2.3E0 | 9.5E3 | 4.3E3 | 109 | 41 | 79 | 41 | 0.39 |
| gV | ng/ml | 2.0E1 | 1.6E1 | 2.1E1 | 1.5E1 | 6.7E0 | 7.5E0 | 1.0E1 | 8.1E-2 | 3.9E1 | 2.5E1 | 59 | 7 | 15 | 7 | 0.29 |
| tF | pg/mL | 2.6E3 | 9.0E2 | 2.2E4 | 1.4E4 | 5.6E4 | 4.1E4 | 1.2E1 | 1.8E1 | 3.2E5 | 2.3E5 | 100 | 39 | 86 | 39 | 0.41 |
| gZ | ug/ml | 8.9E-1 | 9.5E-1 | 4.5E1 | 5.8E1 | 1.1E2 | 1.4E2 | 8.7E-2 | 1.1E-1 | 4.1E2 | 3.8E2 | 28 | 7 | 26 | 7 | 0.52 |
| hA | ng/ml | 2.3E0 | 2.4E0 | 7.4E0 | 9.7E0 | 2.4E1 | 2.4E1 | 1.7E-2 | 1.7E-2 | 1.6E2 | 1.0E2 | 77 | 29 | 64 | 29 | 0.51 |
| nM | pg/ml | 1.0E-9 | 1.0E-9 | 1.9E2 | 1.0E-9 | 1.4E3 | 0.0E0 | 1.0E-9 | 1.0E-9 | 1.0E4 | 1.0E-9 | 54 | 23 | 47 | 23 | 0.48 |
| nN | pg/ml | 1.1E3 | 1.9E3 | 2.1E3 | 2.7E3 | 2.7E3 | 2.9E3 | 1.1E2 | 1.9E2 | 1.7E4 | 1.3E4 | 54 | 23 | 47 | 23 | 0.58 |
| nO | pg/ml | 3.1E1 | 2.9E1 | 4.5E1 | 4.6E1 | 4.7E1 | 5.3E1 | 5.5E0 | 3.5E0 | 2.4E2 | 2.4E2 | 54 | 23 | 47 | 23 | 0.48 |
| nR | pg/ml | 1.6E1 | 2.0E1 | 4.5E1 | 5.6E1 | 7.3E1 | 9.5E1 | 1.0E-9 | 1.7E0 | 3.6E2 | 4.2E2 | 54 | 23 | 47 | 23 | 0.56 |
| nT | pg/ml | 8.5E1 | 8.0E1 | 3.5E2 | 8.8E1 | 1.2E3 | 6.2E1 | 1.0E-9 | 1.0E-9 | 6.6E3 | 2.0E2 | 54 | 23 | 47 | 23 | 0.48 |
| nU | pg/ml | 2.9E1 | 2.9E1 | 5.1E2 | 6.4E1 | 2.2E3 | 6.8E1 | 1.0E-9 | 1.0E-9 | 1.3E4 | 2.2E2 | 54 | 23 | 47 | 23 | 0.56 |
| lW | pg/ml | 1.0E-9 | 1.0E-9 | 1.6E1 | 6.1E-1 | 5.5E1 | 2.0E0 | 1.0E-9 | 1.0E-9 | 3.9E2 | 8.1E0 | 54 | 23 | 47 | 23 | 0.36 |
| lX | pg/ml | 9.5E2 | 9.6E2 | 9.2E2 | 1.0E3 | 4.4E2 | 5.5E2 | 2.3E2 | 1.2E2 | 1.9E3 | 2.1E3 | 54 | 23 | 47 | 23 | 0.54 |
| lY | pg/ml | 1.7E1 | 2.4E1 | 2.2E1 | 2.4E1 | 2.5E1 | 1.2E1 | 1.0E-9 | 5.4E0 | 1.4E2 | 4.4E1 | 54 | 23 | 47 | 23 | 0.63 |
| mE | pg/ml | 1.0E-9 | 1.0E-9 | 3.3E0 | 1.4E0 | 1.0E1 | 3.4E0 | 1.0E-9 | 1.0E-9 | 5.8E1 | 1.3E1 | 54 | 23 | 47 | 23 | 0.49 |
| mF | pg/ml | 1.0E-9 | 1.0E-9 | 1.4E0 | 1.5E0 | 3.0E0 | 3.1E0 | 1.0E-9 | 1.0E-9 | 1.5E1 | 1.3E1 | 54 | 23 | 47 | 23 | 0.54 |
| mH | pg/ml | 3.6E0 | 2.9E0 | 4.9E0 | 3.7E0 | 5.0E0 | 2.8E0 | 2.3E-1 | 3.2E-1 | 3.2E1 | 1.1E1 | 54 | 23 | 47 | 23 | 0.45 |
| mI | pg/ml | 1.0E-9 | 1.0E-9 | 1.2E1 | 8.8E0 | 3.1E1 | 1.7E1 | 1.0E-9 | 1.0E-9 | 1.6E2 | 7.8E1 | 54 | 23 | 47 | 23 | 0.53 |
| mM | pg/ml | 3.0E1 | 1.2E1 | 5.2E1 | 5.6E1 | 5.9E1 | 8.1E1 | 1.0E-9 | 1.0E-9 | 2.3E2 | 2.9E2 | 54 | 23 | 47 | 23 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| mP | pg/ml | 1.5E1 | 1.4E1 | 2.0E1 | 1.3E1 | 2.2E1 | 7.0E0 | 1.0E-9 | 1.0E-9 | 1.5E2 | 2.9E1 | 53 | 23 | 46 | 23 | 0.40 |
| mS | pg/ml | 1.8E3 | 1.7E3 | 2.2E3 | 1.8E3 | 2.2E3 | 8.3E2 | 1.0E-9 | 1.0E-9 | 1.3E4 | 3.9E3 | 54 | 23 | 47 | 23 | 0.45 |
| mT | pg/ml | 5.0E1 | 5.9E1 | 1.2E2 | 8.3E1 | 2.2E2 | 6.1E1 | 1.0E1 | 1.6E1 | 1.4E3 | 2.8E2 | 53 | 23 | 46 | 23 | 0.54 |
| mU | pg/ml | 2.5E0 | 2.5E0 | 4.2E0 | 2.7E0 | 8.5E0 | 2.0E0 | 1.0E-9 | 1.9E-1 | 5.8E1 | 1.1E1 | 53 | 23 | 46 | 23 | 0.48 |
| mW | pg/ml | 2.5E3 | 2.4E3 | 2.7E3 | 2.8E3 | 1.7E3 | 1.3E3 | 4.3E2 | 7.7E2 | 1.0E4 | 5.7E3 | 53 | 23 | 46 | 23 | 0.53 |
| mY | pg/ml | 4.7E2 | 6.2E2 | 1.0E3 | 7.7E2 | 1.8E3 | 5.2E2 | 1.0E-9 | 2.1E2 | 1.1E4 | 2.2E3 | 54 | 23 | 47 | 23 | 0.58 |
| mZ | pg/ml | 2.6E2 | 3.4E2 | 5.0E2 | 5.2E2 | 6.0E2 | 5.8E2 | 2.1E1 | 5.3E1 | 3.1E3 | 2.8E3 | 53 | 23 | 46 | 23 | 0.53 |
| nA | pg/ml | 1.3E0 | 1.5E0 | 1.6E1 | 4.8E0 | 6.6E1 | 1.3E1 | 1.0E-9 | 1.0E-9 | 4.4E2 | 6.2E1 | 53 | 23 | 46 | 23 | 0.48 |
| nB | pg/ml | 2.6E2 | 3.2E2 | 3.1E2 | 3.4E2 | 1.6E2 | 1.2E2 | 3.0E1 | 1.4E2 | 7.2E2 | 6.4E2 | 54 | 23 | 47 | 23 | 0.60 |
| nC | pg/ml | 1.0E-9 | 1.0E-9 | 8.7E3 | 6.7E4 | 5.0E4 | 3.2E5 | 1.0E-9 | 1.0E-9 | 3.7E5 | 1.5E6 | 54 | 23 | 47 | 23 | 0.49 |
| nD | pg/ml | 7.2E0 | 7.9E0 | 7.0E1 | 8.1E0 | 3.1E2 | 7.8E0 | 1.0E-9 | 1.0E-9 | 1.7E3 | 2.9E1 | 53 | 23 | 46 | 23 | 0.46 |
| nF | pg/ml | 1.0E-9 | 1.0E-9 | 9.5E0 | 1.3E0 | 4.0E1 | 4.7E0 | 1.0E-9 | 1.0E-9 | 2.4E2 | 2.1E1 | 54 | 23 | 47 | 23 | 0.49 |
| nH | pg/ml | 3.0E-2 | 3.7E-1 | 2.1E2 | 1.2E2 | 1.4E3 | 5.5E2 | 1.0E-9 | 1.0E-9 | 1.0E4 | 2.6E3 | 53 | 23 | 46 | 23 | 0.51 |
| nI | pg/ml | 4.6E1 | 2.8E0 | 2.9E2 | 5.0E1 | 1.3E3 | 6.4E1 | 1.0E-9 | 1.0E-9 | 9.2E3 | 1.9E2 | 54 | 23 | 47 | 23 | 0.46 |
| nJ | pg/ml | 1.7E-1 | 1.0E-9 | 9.9E1 | 5.2E-1 | 7.0E2 | 8.5E-1 | 1.0E-9 | 1.0E-9 | 5.2E3 | 2.4E0 | 54 | 23 | 47 | 23 | 0.41 |
| nK | pg/ml | 1.0E-9 | 1.0E-9 | 1.2E2 | 1.0E1 | 5.4E2 | 1.8E1 | 1.0E-9 | 1.0E-9 | 3.2E3 | 6.4E1 | 53 | 23 | 46 | 23 | 0.55 |
| nL | pg/ml | 1.0E-9 | 1.0E-9 | 9.3E2 | 2.4E2 | 6.1E3 | 1.1E3 | 1.0E-9 | 1.0E-9 | 4.5E4 | 5.2E3 | 54 | 23 | 47 | 23 | 0.45 |
| hL | pg/ml | 1.7E4 | 2.6E4 | 2.3E4 | 2.6E4 | 2.2E4 | 1.2E4 | 2.6E3 | 5.7E3 | 1.4E5 | 4.6E4 | 51 | 13 | 49 | 13 | 0.64 |
| hO | pg/ml | 1.6E4 | 1.6E4 | 1.7E4 | 1.8E4 | 3.2E3 | 4.4E3 | 1.1E4 | 1.4E4 | 2.8E4 | 2.6E4 | 51 | 13 | 49 | 13 | 0.59 |
| hP | ng/ml | 4.4E5 | 4.4E5 | 3.7E5 | 4.5E5 | 1.7E5 | 2.7E5 | 2.8E4 | 1.7E4 | 9.0E5 | 1.1E6 | 51 | 13 | 49 | 13 | 0.57 |
| wJ | pg/ml | 1.5E5 | 1.6E5 | 1.6E5 | 1.9E5 | 8.7E4 | 1.2E5 | 2.8E4 | 6.0E4 | 4.0E5 | 5.1E5 | 48 | 12 | 44 | 12 | 0.56 |
| wK | pg/ml | 3.4E4 | 2.7E4 | 4.3E4 | 4.1E4 | 2.7E4 | 3.6E4 | 5.2E3 | 9.2E3 | 1.2E5 | 1.4E5 | 48 | 12 | 44 | 12 | 0.45 |
| wL | pg/ml | 6.5E0 | 1.7E0 | 7.0E1 | 2.5E1 | 1.5E2 | 7.1E1 | 1.0E-9 | 1.0E-9 | 8.4E2 | 2.5E2 | 48 | 12 | 44 | 12 | 0.35 |
| wP | pg/ml | 2.8E4 | 3.1E4 | 4.3E4 | 5.7E4 | 4.3E4 | 8.2E4 | 2.8E3 | 2.9E3 | 1.6E5 | 3.0E5 | 48 | 12 | 44 | 12 | 0.50 |
| wQ | pg/ml | 3.4E1 | 4.1E1 | 6.0E1 | 6.1E1 | 7.7E1 | 8.5E1 | 1.0E-9 | 1.0E-9 | 3.7E2 | 3.0E2 | 48 | 12 | 44 | 12 | 0.50 |
| hR | pg/ml | 2.7E4 | 2.6E4 | 3.0E4 | 2.5E4 | 1.1E4 | 9.7E3 | 1.8E3 | 3.6E3 | 5.8E4 | 4.1E4 | 75 | 28 | 62 | 28 | 0.39 |
| hV | pg/ml | 4.4E2 | 3.2E2 | 4.7E2 | 4.1E2 | 2.4E2 | 2.2E2 | 1.3E2 | 6.8E1 | 1.5E3 | 8.2E2 | 75 | 28 | 62 | 28 | 0.43 |
| hW | pg/ml | 1.6E3 | 1.7E3 | 2.1E3 | 2.4E3 | 1.6E3 | 1.9E3 | 5.7E2 | 2.2E2 | 1.0E4 | 6.7E3 | 75 | 28 | 62 | 28 | 0.51 |
| hX | pg/ml | 9.6E2 | 8.9E2 | 1.2E3 | 1.1E3 | 1.1E3 | 1.1E3 | 4.8E2 | 2.3E2 | 8.6E3 | 6.6E3 | 75 | 28 | 62 | 28 | 0.43 |
| iA | pg/ml | 1.7E2 | 1.6E2 | 4.2E2 | 2.5E2 | 9.2E2 | 3.4E2 | 1.8E1 | 1.2E1 | 7.1E3 | 2.2E3 | 100 | 39 | 86 | 39 | 0.50 |
| iB | ng/ml | 5.4E0 | 3.7E0 | 6.7E0 | 5.0E0 | 4.7E0 | 4.6E0 | 2.5E-1 | 4.5E-2 | 2.0E1 | 1.9E1 | 77 | 29 | 64 | 29 | 0.36 |
| iC | U/ml | 2.4E-1 | 2.0E-1 | 5.0E-1 | 8.9E-1 | 8.7E-1 | 2.3E0 | 1.0E-9 | 1.0E-9 | 6.4E0 | 1.2E1 | 77 | 29 | 64 | 29 | 0.52 |
| tQ | pg/ml | 1.1E3 | 1.0E3 | 1.2E3 | 1.1E3 | 5.5E2 | 5.3E2 | 2.8E2 | 6.4E2 | 2.5E3 | 2.5E3 | 46 | 12 | 43 | 12 | 0.42 |
| tT | pg/ml | 1.5E1 | 1.3E1 | 1.7E1 | 1.8E1 | 7.4E0 | 1.5E1 | 7.4E0 | 7.9E0 | 3.9E1 | 6.1E1 | 46 | 12 | 43 | 12 | 0.42 |
| tS | pg/ml | 1.0E0 | 8.6E-1 | 1.1E0 | 8.9E-1 | 8.6E-1 | 6.3E-1 | 1.0E-9 | 1.0E-9 | 4.0E0 | 1.9E0 | 46 | 12 | 43 | 12 | 0.45 |
| tX | pg/ml | 8.1E-1 | 8.7E-1 | 9.5E-1 | 7.8E-1 | 7.0E-1 | 4.0E-1 | 2.5E-2 | 7.6E-2 | 3.3E0 | 1.3E0 | 46 | 12 | 43 | 12 | 0.47 |
| tO | pg/ml | 4.0E0 | 3.8E0 | 4.4E0 | 4.0E0 | 2.5E0 | 2.6E0 | 1.0E-9 | 8.6E-1 | 1.1E1 | 9.0E0 | 46 | 12 | 43 | 12 | 0.45 |
| tR | pg/ml | 2.1E-1 | 1.2E-1 | 2.5E-1 | 1.5E-1 | 2.1E-1 | 1.2E-1 | 1.0E-9 | 1.0E-9 | 9.1E-1 | 3.4E-1 | 46 | 12 | 43 | 12 | 0.37 |
| tU | pg/ml | 9.0E0 | 9.2E0 | 1.1E1 | 1.0E1 | 6.9E0 | 6.2E0 | 1.6E0 | 3.6E0 | 3.1E1 | 2.7E1 | 46 | 12 | 43 | 12 | 0.50 |
| tN | pg/ml | 1.7E1 | 1.5E1 | 1.9E1 | 1.7E1 | 1.0E1 | 9.9E0 | 1.0E-9 | 6.0E0 | 5.4E1 | 4.2E1 | 46 | 12 | 43 | 12 | 0.42 |
| tV | ng/ml | 3.7E2 | 6.6E2 | 4.8E2 | 6.5E2 | 4.0E2 | 3.4E2 | 6.6E1 | 1.5E2 | 2.6E3 | 1.1E3 | 48 | 12 | 44 | 12 | 0.66 |
| iH | ng/ml | 1.5E5 | 1.5E5 | 1.5E5 | 1.5E5 | 4.3E4 | 5.0E4 | 7.1E4 | 7.4E4 | 2.4E5 | 2.5E5 | 100 | 39 | 86 | 39 | 0.54 |
| iJ | ng/ml | 5.1E4 | 4.9E4 | 5.3E4 | 5.0E4 | 2.0E4 | 2.1E4 | 8.7E3 | 8.0E3 | 1.2E5 | 9.7E4 | 100 | 39 | 86 | 39 | 0.46 |
| hB | ng/ml | 4.3E-1 | 3.7E-1 | 5.2E-1 | 4.9E-1 | 3.5E-1 | 3.4E-1 | 1.2E-1 | 1.2E-1 | 1.9E0 | 1.6E0 | 100 | 39 | 86 | 39 | 0.47 |
| hC | pg/ml | 3.6E3 | 4.2E3 | 5.9E3 | 8.2E3 | 7.5E3 | 8.9E3 | 1.0E-9 | 1.0E-9 | 5.5E4 | 3.4E4 | 100 | 39 | 86 | 39 | 0.59 |
| hF | ng/ml | 1.0E-9 | 1.0E-9 | 4.9E1 | 2.6E-1 | 4.1E2 | 1.5E0 | 1.0E-9 | 1.0E-9 | 4.0E3 | 9.6E0 | 100 | 39 | 86 | 39 | 0.51 |
| hG | pg/ml | 7.4E3 | 7.8E3 | 7.8E3 | 7.8E3 | 2.9E3 | 3.5E3 | 1.7E3 | 3.3E3 | 1.8E4 | 1.9E4 | 100 | 39 | 86 | 39 | 0.48 |
| iO | ng/ml | 4.1E5 | 3.4E5 | 4.2E5 | 3.8E5 | 2.0E5 | 1.8E5 | 1.1E5 | 8.3E4 | 1.1E6 | 9.0E5 | 100 | 39 | 86 | 39 | 0.44 |
| iP | ng/ml | 6.1E4 | 5.7E4 | 5.5E4 | 5.6E4 | 3.1E4 | 3.3E4 | 5.8E3 | 2.4E3 | 2.5E5 | 1.5E5 | 100 | 39 | 86 | 39 | 0.50 |
| iZ | ng/ml | 1.7E3 | 1.6E3 | 1.8E3 | 2.0E3 | 6.8E2 | 1.1E3 | 4.7E2 | 8.8E2 | 3.5E3 | 6.5E3 | 100 | 39 | 86 | 39 | 0.51 |
| yH | pg/ml | 1.2E3 | 1.5E3 | 2.0E3 | 1.2E4 | 3.0E3 | 3.5E4 | 1.0E-9 | 1.6E2 | 1.5E4 | 1.2E5 | 48 | 12 | 44 | 12 | 0.53 |
| yK | U/ml | 1.8E1 | 3.2E1 | 4.9E1 | 5.2E1 | 8.5E1 | 6.9E1 | 1.0E-9 | 1.0E-9 | 3.8E2 | 2.5E2 | 48 | 12 | 44 | 12 | 0.58 |
| yJ | pg/ml | 3.6E4 | 5.0E4 | 4.7E4 | 5.3E4 | 3.5E4 | 3.2E4 | 1.7E3 | 1.7E4 | 1.6E5 | 1.1E5 | 48 | 12 | 44 | 12 | 0.57 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| yD | ng/ml | 1.5E-2 | 1.4E-2 | 1.5E-2 | 1.4E-2 | 5.8E-3 | 4.7E-3 | 1.0E-9 | 7.6E-3 | 2.9E-2 | 2.2E-2 | 48 | 12 | 44 | 12 | 0.48 |
| jB | ng/ml | 2.6E5 | 2.4E5 | 2.7E5 | 2.8E5 | 8.8E4 | 1.8E5 | 5.7E4 | 1.2E5 | 4.1E5 | 6.2E5 | 28 | 7 | 26 | 7 | 0.43 |
| wB | pg/ml | 8.8E3 | 6.5E3 | 9.8E3 | 9.2E3 | 7.3E3 | 8.7E3 | 1.7E3 | 2.5E3 | 4.1E4 | 3.4E4 | 48 | 12 | 44 | 12 | 0.44 |
| pY | pg/ml | 6.0E0 | 6.3E0 | 1.1E1 | 7.1E0 | 2.7E1 | 3.4E0 | 2.1E0 | 2.6E0 | 2.0E2 | 1.4E1 | 51 | 13 | 49 | 13 | 0.53 |
| rC | pg/ml | 1.9E3 | 1.2E3 | 2.6E3 | 1.9E3 | 2.8E3 | 1.4E3 | 1.0E2 | 6.6E1 | 1.5E4 | 5.3E3 | 72 | 29 | 59 | 29 | 0.45 |
| rB | pg/ml | 2.2E1 | 3.0E1 | 5.5E1 | 3.8E1 | 1.4E2 | 2.8E1 | 1.0E-9 | 1.0E-9 | 9.5E2 | 1.4E2 | 72 | 29 | 59 | 29 | 0.62 |
| zG | 2.5ng/ml | 2.0E-1 | 1.6E-1 | 4.5E-1 | 4.2E-1 | 7.8E-1 | 9.3E-1 | 1.0E-9 | 1.0E-9 | 4.4E0 | 3.3E0 | 48 | 12 | 44 | 12 | 0.42 |
| zH | 2.3mU/ml | 1.1E-1 | 9.8E-2 | 1.2E-1 | 1.0E-1 | 6.9E-2 | 1.7E-2 | 1.0E-2 | 6.3E-2 | 4.4E-1 | 1.3E-1 | 48 | 12 | 44 | 12 | 0.48 |
| zI | 2.6ng/ml | 1.8E0 | 2.6E0 | 3.2E0 | 4.0E0 | 3.3E0 | 4.2E0 | 6.1E-1 | 9.5E-1 | 1.5E1 | 1.6E1 | 48 | 12 | 44 | 12 | 0.54 |
| qA | ng/ml | 1.0E7 | 8.6E6 | 1.1E7 | 9.0E6 | 7.3E6 | 3.9E6 | 3.7E6 | 2.0E6 | 3.7E7 | 1.6E7 | 51 | 13 | 49 | 13 | 0.43 |
| qB | ng/ml | 6.3E5 | 5.5E5 | 8.3E5 | 6.4E5 | 5.8E5 | 3.4E5 | 2.1E5 | 2.3E5 | 2.9E6 | 1.4E6 | 51 | 13 | 49 | 13 | 0.42 |
| qC | ng/ml | 4.8E5 | 2.8E5 | 8.2E5 | 5.4E5 | 1.2E6 | 9.1E5 | 2.0E4 | 3.4E3 | 7.1E6 | 3.4E6 | 51 | 13 | 49 | 13 | 0.35 |
| qD | ng/ml | 1.6E7 | 1.3E7 | 1.9E7 | 1.5E7 | 9.4E6 | 5.5E6 | 4.9E6 | 4.9E6 | 5.2E7 | 2.8E7 | 51 | 13 | 49 | 13 | 0.37 |
| jD | ng/ml | 2.2E1 | 4.8E1 | 4.4E1 | 5.6E1 | 7.2E1 | 5.6E1 | 1.0E-9 | 1.0E-9 | 5.1E2 | 2.9E2 | 77 | 29 | 64 | 29 | 0.64 |
| jE | ng/ml | 1.0E-9 | 1.0E-9 | 8.0E0 | 8.5E0 | 2.1E1 | 1.6E1 | 1.0E-9 | 1.0E-9 | 1.2E2 | 5.6E1 | 77 | 29 | 64 | 29 | 0.53 |
| jF | ng/ml | 3.5E1 | 3.7E1 | 5.0E1 | 5.0E1 | 5.8E1 | 6.1E1 | 1.0E-9 | 1.0E-9 | 2.5E2 | 1.8E2 | 77 | 29 | 64 | 29 | 0.48 |
| jG | ng/ml | 4.5E3 | 4.7E3 | 4.6E3 | 4.4E3 | 1.9E3 | 1.6E3 | 7.6E2 | 6.0E2 | 9.5E3 | 7.5E3 | 77 | 29 | 64 | 29 | 0.49 |
| jH | ng/ml | 7.9E1 | 7.4E1 | 8.6E1 | 8.6E1 | 4.5E1 | 5.8E1 | 1.9E1 | 2.5E1 | 2.3E2 | 3.3E2 | 77 | 29 | 64 | 29 | 0.47 |
| jI | ng/ml | 6.9E1 | 7.7E1 | 7.4E1 | 8.1E1 | 3.4E1 | 3.3E1 | 2.8E1 | 3.9E1 | 2.5E2 | 1.9E2 | 77 | 29 | 64 | 29 | 0.58 |
| sK | pg/mL | 4.2E3 | 3.9E3 | 4.3E3 | 4.2E3 | 1.6E3 | 1.5E3 | 1.7E3 | 1.1E3 | 9.2E3 | 6.1E3 | 49 | 13 | 45 | 13 | 0.51 |
| sM | pg/mL | 8.0E4 | 8.5E4 | 7.9E4 | 8.1E4 | 2.4E4 | 2.5E4 | 3.3E4 | 3.9E4 | 1.5E5 | 1.3E5 | 49 | 13 | 45 | 13 | 0.55 |
| sO | pg/mL | 2.8E8 | 2.4E8 | 2.9E8 | 2.4E8 | 9.9E7 | 9.8E7 | 7.9E7 | 9.1E7 | 4.9E8 | 3.9E8 | 49 | 13 | 45 | 13 | 0.39 |
| wC | ng/ml | 1.6E0 | 1.7E0 | 2.1E0 | 1.9E0 | 2.2E0 | 9.9E-1 | 2.5E-1 | 5.9E-1 | 1.5E1 | 3.7E0 | 48 | 12 | 44 | 12 | 0.53 |
| wD | ng/ml | 2.0E1 | 2.1E1 | 8.1E1 | 2.7E1 | 3.1E2 | 2.1E1 | 2.8E0 | 6.0E0 | 2.1E3 | 7.2E1 | 48 | 12 | 44 | 12 | 0.56 |
| wE | ng/ml | 5.0E1 | 5.0E1 | 5.4E1 | 4.8E1 | 2.4E1 | 1.3E1 | 7.0E0 | 3.0E1 | 1.4E2 | 7.2E1 | 48 | 12 | 44 | 12 | 0.43 |
| wG | ng/ml | 9.6E-2 | 1.1E-2 | 1.3E-1 | 5.0E-2 | 1.3E-1 | 7.4E-2 | 1.0E-9 | 1.0E-9 | 4.8E-1 | 2.1E-1 | 48 | 12 | 44 | 12 | 0.34 |
| wH | ng/ml | 2.3E-2 | 9.1E-3 | 2.1E-1 | 5.0E-2 | 5.5E-1 | 8.1E-2 | 1.0E-9 | 1.0E-9 | 2.9E0 | 2.4E-1 | 48 | 12 | 44 | 12 | 0.37 |
| wF | ng/ml | 2.1E-1 | 4.5E-2 | 2.9E0 | 2.3E-1 | 1.0E1 | 3.7E-1 | 1.0E-9 | 1.0E-9 | 5.7E1 | 1.1E0 | 48 | 12 | 44 | 12 | 0.36 |
| rA | pg/ml | 2.4E1 | 2.2E1 | 2.7E1 | 3.1E1 | 2.2E1 | 2.5E1 | 1.0E-9 | 4.2E0 | 1.2E2 | 9.4E1 | 74 | 30 | 61 | 30 | 0.53 |
| qZ | pg/ml | 4.3E1 | 4.2E1 | 2.1E2 | 9.5E2 | 1.3E3 | 2.9E3 | 2.8E-4 | 4.8E-4 | 1.0E4 | 1.0E4 | 63 | 22 | 54 | 22 | 0.50 |
| qY | pg/ml | 2.1E1 | 2.7E1 | 4.6E1 | 5.7E1 | 7.5E1 | 6.1E1 | 8.7E-1 | 3.3E0 | 5.3E2 | 2.3E2 | 74 | 30 | 61 | 30 | 0.58 |
| qX | pg/ml | 5.1E1 | 5.3E1 | 5.9E1 | 6.5E1 | 3.9E1 | 4.9E1 | 1.0E-9 | 1.0E-9 | 1.8E2 | 2.1E2 | 74 | 30 | 61 | 30 | 0.50 |
| qW | pg/ml | 9.4E0 | 8.1E0 | 1.2E1 | 1.1E1 | 1.3E1 | 1.1E1 | 1.0E-9 | 1.0E-9 | 7.1E1 | 5.1E1 | 74 | 30 | 61 | 30 | 0.48 |
| qV | pg/ml | 2.1E3 | 2.0E3 | 2.7E3 | 2.8E3 | 1.9E3 | 2.0E3 | 2.3E2 | 5.6E2 | 8.5E3 | 8.2E3 | 74 | 30 | 61 | 30 | 0.50 |
| qU | pg/ml | 4.5E1 | 6.2E1 | 1.3E2 | 1.2E2 | 2.2E2 | 1.6E2 | 1.0E-9 | 1.0E-9 | 1.4E3 | 7.3E2 | 74 | 30 | 61 | 30 | 0.54 |
| qT | pg/ml | 3.8E1 | 4.4E1 | 6.4E1 | 6.4E1 | 1.2E2 | 5.5E1 | 1.0E-9 | 1.0E-9 | 9.0E2 | 2.0E2 | 74 | 30 | 61 | 30 | 0.57 |
| qI | ng/ml | 5.8E4 | 8.1E4 | 6.4E4 | 7.3E4 | 3.2E4 | 3.1E4 | 5.4E3 | 5.9E3 | 1.6E5 | 1.0E5 | 48 | 14 | 46 | 14 | 0.64 |
| qH | ng/ml | 6.5E4 | 4.6E4 | 7.3E4 | 5.3E4 | 3.9E4 | 3.6E4 | 1.0E4 | 7.6E3 | 1.8E5 | 1.2E5 | 48 | 14 | 46 | 14 | 0.35 |
| qG | ng/ml | 1.8E5 | 2.4E5 | 1.9E5 | 2.1E5 | 6.2E4 | 9.9E4 | 3.1E4 | 1.7E4 | 3.3E5 | 3.1E5 | 48 | 14 | 46 | 14 | 0.60 |
| jK | ng/ml | 1.6E3 | 1.8E3 | 1.7E3 | 1.9E3 | 5.7E2 | 7.7E2 | 5.5E2 | 7.2E2 | 4.1E3 | 4.0E3 | 77 | 29 | 64 | 29 | 0.58 |
| jL | ng/ml | 1.8E2 | 2.5E2 | 2.6E2 | 3.6E2 | 1.9E2 | 4.0E2 | 3.6E1 | 4.9E1 | 7.9E2 | 2.1E3 | 77 | 29 | 64 | 29 | 0.60 |
| jM | ng/ml | 7.1E4 | 6.3E4 | 7.0E4 | 7.3E4 | 3.4E4 | 4.2E4 | 3.9E2 | 1.1E3 | 1.5E5 | 1.7E5 | 77 | 29 | 64 | 29 | 0.52 |
| jO | pg/ml | 2.2E5 | 2.3E5 | 2.8E5 | 2.5E5 | 1.5E5 | 1.2E5 | 5.2E4 | 7.7E4 | 7.7E5 | 5.2E5 | 77 | 29 | 64 | 29 | 0.47 |
| jP | pg/ml | 2.3E5 | 2.5E5 | 2.6E5 | 2.9E5 | 1.5E5 | 2.4E5 | 7.0E4 | 7.4E4 | 9.1E5 | 1.2E6 | 77 | 29 | 64 | 29 | 0.50 |
| jQ | pg/ml | 2.5E3 | 2.7E3 | 3.0E3 | 3.5E3 | 2.3E3 | 3.4E3 | 4.2E1 | 1.4E2 | 1.2E4 | 1.4E4 | 77 | 29 | 64 | 29 | 0.50 |
| jR | pg/ml | 6.5E3 | 5.9E3 | 9.5E3 | 9.6E3 | 9.9E3 | 9.6E3 | 1.0E-9 | 1.0E-9 | 5.5E4 | 3.0E4 | 77 | 29 | 64 | 29 | 0.49 |
| jT | pg/ml | 1.6E5 | 1.7E5 | 1.7E5 | 1.9E5 | 6.9E4 | 8.1E4 | 6.8E4 | 7.9E4 | 4.5E5 | 3.8E5 | 77 | 29 | 64 | 29 | 0.53 |
| xA | pg/ml | 3.9E0 | 4.7E0 | 1.7E1 | 7.5E0 | 5.7E1 | 1.1E1 | 1.0E-9 | 1.0E-9 | 3.9E2 | 3.0E1 | 48 | 12 | 44 | 12 | 0.47 |
| yE | pg/ml | 7.8E1 | 7.2E1 | 8.2E1 | 8.2E1 | 4.9E1 | 2.9E1 | 6.4E0 | 5.2E1 | 3.0E2 | 1.3E2 | 48 | 12 | 44 | 12 | 0.53 |
| tM | pg/ml | 4.3E1 | 3.5E1 | 4.3E1 | 3.7E1 | 2.0E1 | 1.4E1 | 1.0E-9 | 2.2E1 | 1.0E2 | 6.3E1 | 48 | 12 | 44 | 12 | 0.39 |
| tL | pg/ml | 1.0E-9 | 1.0E-9 | 7.8E0 | 3.1E-1 | 3.8E1 | 6.6E-1 | 1.0E-9 | 1.0E-9 | 2.6E2 | 2.1E0 | 48 | 12 | 44 | 12 | 0.49 |
| jU | mIU/ml | 3.8E0 | 5.5E0 | 1.0E1 | 8.8E0 | 1.7E1 | 1.3E1 | 8.9E-2 | 6.2E-2 | 8.1E1 | 6.7E1 | 77 | 29 | 64 | 29 | 0.55 |
| jV | mIU/ml | 1.6E0 | 1.1E0 | 3.5E0 | 2.4E0 | 5.4E0 | 5.1E0 | 2.1E-2 | 1.7E-3 | 3.1E1 | 2.6E1 | 77 | 29 | 64 | 29 | 0.36 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| jY | ng/ml | 9.7E-4 | 7.6E-4 | 1.1E-2 | 3.0E-3 | 4.2E-2 | 5.7E-3 | 1.0E-9 | 1.0E-9 | 3.0E-1 | 2.4E-2 | 77 | 29 | 64 | 29 | 0.46 |
| kC | pg/ml | 9.7E1 | 8.8E1 | 2.4E2 | 1.0E2 | 5.6E2 | 4.8E1 | 2.1E1 | 2.1E1 | 3.5E3 | 2.5E2 | 54 | 23 | 47 | 23 | 0.46 |
| kE | pg/ml | 1.3E5 | 1.4E5 | 1.3E5 | 1.4E5 | 3.3E4 | 4.6E4 | 4.1E4 | 4.1E4 | 2.0E5 | 2.1E5 | 54 | 23 | 47 | 23 | 0.54 |
| kF | pg/mL | 6.6E1 | 6.5E1 | 8.0E1 | 6.2E1 | 7.0E1 | 1.8E1 | 3.2E1 | 2.8E1 | 5.1E2 | 9.3E1 | 54 | 23 | 47 | 23 | 0.42 |
| kG | pg/mL | 9.1E3 | 1.1E4 | 1.2E4 | 1.5E4 | 1.4E4 | 1.2E4 | 7.5E2 | 1.1E3 | 9.1E4 | 5.0E4 | 54 | 23 | 47 | 23 | 0.57 |
| kI | pg/ml | 2.2E2 | 1.7E2 | 2.4E2 | 1.8E2 | 1.4E2 | 7.1E1 | 5.4E1 | 7.2E1 | 8.7E2 | 4.2E2 | 54 | 23 | 47 | 23 | 0.32 |
| kK | pg/ml | 1.2E2 | 8.5E1 | 1.7E2 | 1.3E2 | 1.8E2 | 1.2E2 | 2.2E1 | 2.9E1 | 1.2E3 | 5.1E2 | 54 | 23 | 47 | 23 | 0.38 |
| kN | pg/ml | 1.0E3 | 1.0E3 | 1.5E3 | 2.9E3 | 1.9E3 | 8.0E3 | 2.1E2 | 2.3E2 | 1.3E4 | 3.9E4 | 54 | 23 | 47 | 23 | 0.51 |
| kO | pg/ml | 7.7E3 | 6.9E3 | 1.1E4 | 7.6E3 | 1.8E4 | 3.4E3 | 4.0E3 | 3.4E3 | 1.3E5 | 1.9E4 | 54 | 23 | 47 | 23 | 0.41 |
| kP | pg/ml | 5.4E3 | 5.4E3 | 6.7E3 | 8.9E3 | 5.5E3 | 1.0E4 | 8.6E2 | 1.4E3 | 3.3E4 | 4.8E4 | 54 | 23 | 47 | 23 | 0.54 |
| kQ | pg/ml | 4.3E3 | 5.2E3 | 4.9E3 | 5.6E3 | 2.3E3 | 3.5E3 | 5.6E2 | 1.8E3 | 1.2E4 | 1.8E4 | 100 | 39 | 86 | 39 | 0.52 |
| kR | pg/ml | 2.4E1 | 2.1E1 | 4.0E1 | 2.3E1 | 1.0E2 | 1.4E1 | 1.0E-9 | 1.4E-1 | 1.0E3 | 6.9E1 | 100 | 39 | 86 | 39 | 0.43 |
| kS | pg/ml | 7.7E2 | 9.1E2 | 9.9E2 | 1.1E3 | 1.4E3 | 8.5E2 | 8.2E1 | 2.1E2 | 1.4E4 | 4.1E3 | 100 | 39 | 86 | 39 | 0.56 |
| pS | ng/ml | 2.0E5 | 2.5E5 | 2.2E5 | 2.4E5 | 9.5E4 | 7.9E4 | 7.5E4 | 1.4E5 | 5.0E5 | 3.6E5 | 49 | 13 | 45 | 13 | 0.62 |
| rZ | ng/ml | 1.2E-3 | 1.0E-9 | 7.9E-3 | 3.9E-3 | 2.1E-2 | 7.1E-3 | 1.0E-9 | 1.0E-9 | 1.1E-1 | 3.0E-2 | 73 | 28 | 58 | 28 | 0.45 |
| rY | ng/ml | 5.4E-2 | 6.1E-2 | 2.5E-1 | 9.1E-1 | 8.3E-1 | 4.4E0 | 1.0E-9 | 1.0E-9 | 6.3E0 | 2.3E1 | 73 | 28 | 58 | 28 | 0.52 |
| rX | ng/ml | 1.0E-9 | 1.0E-9 | 9.2E-2 | 1.1E-1 | 4.5E-1 | 5.6E-1 | 1.0E-9 | 1.0E-9 | 3.7E0 | 3.0E0 | 73 | 28 | 58 | 28 | 0.45 |
| lK | pg/ml | 7.1E1 | 1.8E2 | 1.4E2 | 2.9E2 | 1.8E2 | 6.0E2 | 1.0E-9 | 1.0E-9 | 7.0E2 | 3.3E3 | 77 | 29 | 64 | 29 | 0.61 |
| lL | pg/ml | 1.8E3 | 1.4E3 | 3.4E3 | 2.2E3 | 5.5E3 | 2.2E3 | 7.5E1 | 1.5E1 | 4.2E4 | 6.9E3 | 77 | 29 | 64 | 29 | 0.41 |
| lM | pg/ml | 1.0E3 | 1.3E3 | 3.3E3 | 3.3E3 | 7.5E3 | 5.6E3 | 3.9E1 | 2.4E1 | 5.1E4 | 2.9E4 | 77 | 29 | 64 | 29 | 0.53 |
| lN | pg/ml | 1.0E-9 | 4.8E-1 | 7.3E0 | 3.4E0 | 2.2E1 | 5.1E0 | 1.0E-9 | 1.0E-9 | 1.5E2 | 2.4E1 | 77 | 29 | 64 | 29 | 0.53 |
| lO | pg/ml | 1.0E-9 | 1.0E-9 | 5.2E-1 | 1.2E0 | 4.6E0 | 6.2E0 | 1.0E-9 | 1.0E-9 | 4.0E1 | 3.4E1 | 77 | 29 | 64 | 29 | 0.51 |
| zA | ng/ml | 1.9E7 | 2.0E7 | 2.0E7 | 1.9E7 | 6.8E6 | 6.3E6 | 6.7E6 | 6.7E6 | 3.6E7 | 2.8E7 | 44 | 11 | 40 | 11 | 0.52 |
| rW | ng/ml | 1.3E-2 | 3.0E-2 | 2.4E-2 | 5.2E-2 | 3.1E-2 | 5.9E-2 | 1.0E-9 | 1.0E-9 | 1.7E-1 | 1.8E-1 | 49 | 13 | 46 | 13 | 0.65 |
| rV | ng/ml | 1.0E-9 | 1.0E-9 | 9.2E-3 | 2.6E-3 | 3.7E-2 | 8.1E-3 | 1.0E-9 | 1.0E-9 | 2.2E-1 | 2.9E-2 | 49 | 13 | 46 | 13 | 0.48 |
| rU | ng/ml | 9.5E-2 | 5.5E-2 | 1.4E-1 | 1.1E-1 | 2.3E-1 | 1.2E-1 | 1.0E-9 | 1.0E-9 | 1.4E0 | 3.6E-1 | 49 | 13 | 46 | 13 | 0.49 |
| rT | ng/ml | 6.7E0 | 5.0E0 | 6.8E0 | 5.6E0 | 4.1E0 | 1.8E0 | 7.5E-1 | 3.7E0 | 2.1E1 | 1.1E1 | 49 | 13 | 46 | 13 | 0.41 |
| rS | ng/ml | 3.4E0 | 3.5E0 | 5.6E0 | 3.9E0 | 6.8E0 | 2.5E0 | 1.0E0 | 3.9E-1 | 3.8E1 | 8.4E0 | 49 | 13 | 46 | 13 | 0.46 |
| sC | pg/mL | 5.6E3 | 1.3E4 | 8.5E3 | 1.8E4 | 7.1E3 | 1.4E4 | 1.7E3 | 2.5E3 | 3.2E4 | 4.4E4 | 49 | 13 | 45 | 13 | 0.70 |
| yL | pg/ml | 3.4E1 | 3.3E1 | 4.2E1 | 3.2E1 | 2.8E1 | 1.7E1 | 5.6E0 | 1.6E1 | 1.8E2 | 7.3E1 | 47 | 11 | 43 | 11 | 0.38 |
| rP | ng/ml | 7.7E1 | 3.2E2 | 1.7E2 | 3.1E2 | 2.3E2 | 1.9E2 | 1.0E-9 | 6.5E1 | 1.2E3 | 5.0E2 | 49 | 13 | 46 | 13 | 0.76 |
| rQ | ng/ml | 1.0E-9 | 1.0E-9 | 3.4E0 | 1.9E1 | 1.5E1 | 4.7E1 | 1.0E-9 | 1.0E-9 | 8.9E1 | 1.7E2 | 49 | 13 | 46 | 13 | 0.61 |
| rO | ng/ml | 2.5E-2 | 5.0E-2 | 3.9E-2 | 5.2E-2 | 7.1E-2 | 3.9E-2 | 1.0E-9 | 1.0E-9 | 4.4E-1 | 1.2E-1 | 49 | 13 | 46 | 13 | 0.66 |
| rR | ng/ml | 3.9E0 | 1.3E1 | 2.2E1 | 2.5E1 | 6.6E1 | 3.1E1 | 1.0E-9 | 1.0E-9 | 4.5E2 | 1.1E2 | 49 | 13 | 46 | 13 | 0.68 |
| rN | ng/ml | 6.4E-1 | 5.1E-1 | 7.0E-1 | 6.9E-1 | 3.8E-1 | 5.2E-1 | 5.1E-2 | 3.1E-1 | 2.1E0 | 2.2E0 | 49 | 13 | 46 | 13 | 0.41 |
| qO | pg/ml | 9.7E3 | 1.3E4 | 1.2E4 | 1.6E4 | 8.2E3 | 1.4E4 | 2.2E3 | 1.1E3 | 3.9E4 | 4.5E4 | 50 | 13 | 47 | 13 | 0.53 |
| qP | pg/ml | 3.6E2 | 4.1E2 | 3.9E2 | 5.3E2 | 2.5E2 | 4.1E2 | 1.0E-9 | 1.1E2 | 1.1E3 | 1.5E3 | 50 | 13 | 47 | 13 | 0.57 |
| qQ | pg/ml | 3.1E0 | 1.5E1 | 1.5E1 | 1.9E1 | 4.0E1 | 2.8E1 | 1.0E-9 | 1.0E-9 | 2.8E2 | 9.9E1 | 50 | 13 | 47 | 13 | 0.57 |
| nW | pg/ml | 1.1E5 | 1.1E5 | 1.1E5 | 1.1E5 | 2.5E4 | 2.5E4 | 5.8E4 | 7.9E4 | 1.8E5 | 1.9E5 | 100 | 39 | 86 | 39 | 0.49 |
| nY | pg/ml | 1.9E3 | 2.4E3 | 2.3E3 | 2.5E3 | 1.4E3 | 1.2E3 | 6.5E2 | 9.8E2 | 9.9E3 | 5.9E3 | 100 | 39 | 86 | 39 | 0.56 |
| oO | pg/ml | 8.2E4 | 8.8E4 | 1.2E5 | 1.2E5 | 1.1E5 | 8.5E4 | 4.0E4 | 3.8E4 | 6.2E5 | 3.1E5 | 52 | 22 | 45 | 22 | 0.54 |
| oP | pg/ml | 1.3E5 | 1.6E5 | 1.4E5 | 1.7E5 | 8.4E4 | 1.1E5 | 4.8E4 | 4.8E4 | 3.5E5 | 4.6E5 | 52 | 22 | 45 | 22 | 0.57 |
| oQ | pg/ml | 3.0E3 | 3.5E3 | 3.2E3 | 4.4E3 | 1.6E3 | 3.1E3 | 1.1E3 | 1.7E3 | 1.0E4 | 1.4E4 | 52 | 22 | 45 | 22 | 0.58 |
| oE | pg/ml | 2.1E2 | 1.1E2 | 4.6E2 | 3.5E2 | 6.4E2 | 4.9E2 | 1.0E-9 | 1.0E-9 | 4.7E3 | 1.9E3 | 100 | 39 | 86 | 39 | 0.43 |
| oF | pg/ml | 8.6E3 | 7.6E3 | 2.0E4 | 2.6E4 | 3.3E4 | 4.8E4 | 6.4E1 | 7.7E2 | 2.3E5 | 1.8E5 | 100 | 39 | 86 | 39 | 0.48 |
| oH | pg/ml | 4.2E1 | 4.6E1 | 9.3E1 | 7.3E1 | 1.5E2 | 6.7E1 | 4.2E0 | 6.2E0 | 8.6E2 | 2.9E2 | 100 | 39 | 86 | 39 | 0.54 |
| oK | pg/ml | 6.2E2 | 9.5E2 | 1.9E3 | 3.1E3 | 3.2E3 | 8.4E3 | 5.2E1 | 2.3E2 | 1.8E4 | 5.3E4 | 100 | 39 | 86 | 39 | 0.58 |
| oN | pg/ml | 4.7E2 | 5.1E2 | 8.0E2 | 6.7E2 | 1.9E3 | 7.5E2 | 1.5E2 | 2.6E2 | 1.8E4 | 5.0E3 | 100 | 39 | 86 | 39 | 0.56 |
| oW | pg/ml | 2.0E2 | 2.8E2 | 4.7E2 | 2.7E2 | 1.1E3 | 1.6E2 | 7.7E1 | 8.5E1 | 6.0E3 | 5.4E2 | 28 | 7 | 26 | 7 | 0.54 |
| oT | pg/ml | 3.1E2 | 3.7E2 | 3.5E2 | 3.8E2 | 1.8E2 | 1.6E2 | 9.9E1 | 1.9E2 | 7.8E2 | 7.1E2 | 28 | 7 | 26 | 7 | 0.56 |
| oV | pg/ml | 1.4E2 | 1.1E2 | 2.4E2 | 1.4E2 | 3.2E2 | 7.3E1 | 1.0E-9 | 5.8E1 | 1.4E3 | 2.2E2 | 28 | 7 | 26 | 7 | 0.51 |
| oD | pg/ml | 1.6E4 | 1.8E4 | 1.8E4 | 2.0E4 | 8.1E3 | 6.9E3 | 8.7E3 | 1.2E4 | 4.6E4 | 3.3E4 | 28 | 7 | 26 | 7 | 0.61 |
| uL | ng/ml | 3.8E1 | 3.9E1 | 4.1E1 | 4.2E1 | 2.4E1 | 2.2E1 | 1.0E-9 | 1.5E1 | 1.6E2 | 8.6E1 | 48 | 13 | 44 | 13 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| uO | ng/ml | 3.5E-1 | 1.3E0 | 8.5E-1 | 1.4E0 | 1.5E0 | 1.5E0 | 1.0E-9 | 1.0E-9 | 9.3E0 | 5.4E0 | 48 | 13 | 44 | 13 | 0.64 |
| uM | ng/ml | 6.3E-1 | 7.9E-1 | 1.2E0 | 8.1E-1 | 2.3E0 | 5.4E-1 | 1.0E-9 | 1.0E-9 | 1.3E1 | 2.2E0 | 48 | 13 | 44 | 13 | 0.54 |
| uI | ng/ml | 6.5E-2 | 1.0E-1 | 1.3E-1 | 1.5E-1 | 1.9E-1 | 1.7E-1 | 1.6E-2 | 3.7E-2 | 1.1E0 | 6.6E-1 | 48 | 13 | 44 | 13 | 0.61 |
| uN | ng/ml | 1.5E1 | 1.2E1 | 1.6E1 | 1.5E1 | 6.5E0 | 7.2E0 | 7.8E0 | 6.3E0 | 4.1E1 | 3.0E1 | 48 | 13 | 44 | 13 | 0.40 |
| uG | ng/ml | 2.2E1 | 1.8E1 | 2.5E1 | 2.1E1 | 1.3E1 | 9.8E0 | 9.8E0 | 7.5E0 | 6.9E1 | 3.9E1 | 48 | 13 | 44 | 13 | 0.39 |
| uR | ng/ml | 2.3E0 | 4.0E0 | 4.2E0 | 4.0E0 | 9.1E0 | 1.9E0 | 9.9E-1 | 9.8E-1 | 6.4E1 | 8.0E0 | 48 | 12 | 44 | 12 | 0.71 |
| uP | ng/ml | 1.8E0 | 2.3E0 | 2.2E0 | 2.7E0 | 1.3E0 | 1.2E0 | 1.1E0 | 1.6E0 | 9.1E0 | 6.2E0 | 48 | 12 | 44 | 12 | 0.70 |
| uV | ng/ml | 1.0E-9 | 7.6E-3 | 1.1E-2 | 2.2E-2 | 3.3E-2 | 2.8E-2 | 1.0E-9 | 1.0E-9 | 2.0E-1 | 7.6E-2 | 48 | 12 | 44 | 12 | 0.63 |
| uT | ng/ml | 6.2E1 | 6.0E1 | 8.8E1 | 7.8E1 | 9.6E1 | 5.6E1 | 1.2E1 | 2.0E1 | 5.8E2 | 2.0E2 | 48 | 12 | 44 | 12 | 0.50 |
| uU | ng/ml | 1.6E0 | 2.0E0 | 2.0E0 | 1.8E0 | 1.1E0 | 7.3E-1 | 6.0E-1 | 8.6E-1 | 5.4E0 | 3.0E0 | 48 | 12 | 44 | 12 | 0.50 |
| uW | ng/ml | 7.5E0 | 7.3E0 | 7.9E0 | 7.5E0 | 2.8E0 | 2.9E0 | 4.0E0 | 3.5E0 | 2.2E1 | 1.5E1 | 48 | 13 | 44 | 13 | 0.45 |
| vB | ng/ml | 2.7E0 | 3.1E0 | 2.7E0 | 3.2E0 | 1.3E0 | 1.5E0 | 5.9E-1 | 9.9E-1 | 5.6E0 | 6.1E0 | 48 | 13 | 44 | 13 | 0.60 |
| vC | ng/ml | 1.0E-9 | 1.0E-9 | 7.5E-3 | 4.0E-3 | 3.5E-2 | 1.4E-2 | 1.0E-9 | 1.0E-9 | 2.0E-1 | 5.1E-2 | 48 | 13 | 44 | 13 | 0.54 |
| uY | ng/ml | 8.3E-1 | 7.8E-1 | 1.3E0 | 9.4E-1 | 1.2E0 | 7.5E-1 | 8.7E-2 | 1.8E-1 | 4.9E0 | 2.6E0 | 48 | 13 | 44 | 13 | 0.41 |
| uZ | ng/ml | 5.9E-1 | 5.1E-1 | 8.8E-1 | 6.4E-1 | 1.1E0 | 3.7E-1 | 1.4E-1 | 1.2E-1 | 7.2E0 | 1.4E0 | 48 | 13 | 44 | 13 | 0.46 |
| uX | ng/ml | 1.1E1 | 1.3E1 | 1.2E1 | 1.4E1 | 6.7E0 | 7.2E0 | 3.6E0 | 3.7E0 | 3.3E1 | 2.7E1 | 48 | 13 | 44 | 13 | 0.58 |
| vA | ng/ml | 6.8E-2 | 6.9E-2 | 8.5E-2 | 8.3E-2 | 5.8E-2 | 4.2E-2 | 2.5E-2 | 1.7E-2 | 3.0E-1 | 1.6E-1 | 48 | 13 | 44 | 13 | 0.53 |
| vH | ng/ml | 1.2E-1 | 1.5E-1 | 1.7E-1 | 1.4E-1 | 1.6E-1 | 8.1E-2 | 1.5E-2 | 9.9E-3 | 8.0E-1 | 2.8E-1 | 49 | 13 | 45 | 13 | 0.48 |
| vI | ng/ml | 1.8E0 | 2.1E0 | 1.9E0 | 2.4E0 | 1.2E0 | 2.9E0 | 6.2E-3 | 4.2E-3 | 5.1E0 | 1.0E1 | 49 | 13 | 45 | 13 | 0.46 |
| vP | ng/ml | 3.8E2 | 5.5E2 | 4.4E2 | 5.7E2 | 3.2E2 | 4.5E2 | 7.0E1 | 4.0E1 | 1.5E3 | 1.3E3 | 48 | 12 | 44 | 12 | 0.57 |
| vT | ng/ml | 7.7E1 | 8.2E1 | 1.2E2 | 7.5E1 | 1.2E2 | 2.6E1 | 4.1E1 | 2.4E1 | 6.9E2 | 1.0E2 | 48 | 12 | 44 | 12 | 0.44 |
| vU | ng/ml | 1.0E-9 | 1.0E-9 | 2.3E1 | 1.3E1 | 3.2E1 | 2.1E1 | 1.0E-9 | 1.0E-9 | 1.0E2 | 5.0E1 | 48 | 12 | 44 | 12 | 0.43 |
| vQ | ng/ml | 3.4E2 | 4.1E2 | 3.4E2 | 4.1E2 | 1.3E2 | 8.4E1 | 6.7E1 | 3.0E2 | 7.0E2 | 5.5E2 | 48 | 12 | 44 | 12 | 0.68 |
| vO | ng/ml | 1.7E3 | 1.9E3 | 1.8E3 | 1.9E3 | 4.6E2 | 4.3E2 | 1.0E3 | 1.2E3 | 3.0E3 | 2.5E3 | 48 | 12 | 44 | 12 | 0.60 |
| vS | ng/ml | 1.3E3 | 1.1E3 | 1.3E3 | 9.9E2 | 3.5E2 | 5.2E2 | 1.0E-9 | 1.0E-9 | 1.8E3 | 1.5E3 | 48 | 12 | 44 | 12 | 0.31 |
| vV | ng/ml | 8.4E2 | 1.0E3 | 1.1E3 | 2.1E3 | 9.9E2 | 3.1E3 | 1.1E2 | 2.1E1 | 5.0E3 | 1.1E4 | 48 | 12 | 44 | 12 | 0.53 |
| vW | ng/ml | 1.5E2 | 1.9E2 | 1.7E2 | 2.1E2 | 1.3E2 | 1.2E2 | 4.3E1 | 7.1E1 | 6.7E2 | 4.0E2 | 48 | 12 | 44 | 12 | 0.61 |
| pF | pg/ml | 5.0E-1 | 6.6E-1 | 6.8E-1 | 9.7E-1 | 1.0E0 | 1.6E0 | 1.0E-9 | 1.0E-9 | 9.4E0 | 1.0E1 | 100 | 39 | 86 | 39 | 0.59 |
| pH | ng/ml | 7.3E0 | 1.2E1 | 8.6E0 | 1.5E1 | 4.0E0 | 1.3E1 | 3.4E0 | 4.7E0 | 1.8E1 | 4.2E1 | 28 | 7 | 26 | 7 | 0.66 |
| pI | ng/ml | 7.1E1 | 9.7E1 | 7.0E1 | 8.7E1 | 3.2E1 | 2.6E1 | 2.6E1 | 5.6E1 | 1.5E2 | 1.2E2 | 28 | 7 | 26 | 7 | 0.67 |
| pK | ng/ml | 4.5E-1 | 6.1E-1 | 5.0E-1 | 6.9E-1 | 2.9E-1 | 3.6E-1 | 2.0E-1 | 3.6E-1 | 1.6E0 | 1.4E0 | 28 | 7 | 26 | 7 | 0.69 |

Figure 15.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 6.9E1 | 4.8E1 | 8.1E1 | 5.1E1 | 5.8E1 | 3.3E1 | 1.0E0 | 1.2E1 | 4.8E2 | 1.1E2 | 1864 | 8 | 312 | 8 | 0.35 |
| Ad | ug/mL | 3.9E-2 | 5.0E-2 | 7.4E-2 | 5.1E-2 | 8.9E-2 | 3.8E-2 | 2.7E-4 | 7.8E-4 | 5.4E-1 | 1.0E-1 | 534 | 10 | 204 | 10 | 0.49 |
| Af | ng/mL | 1.2E0 | 3.6E-1 | 1.6E1 | 4.9E-1 | 6.0E1 | 5.7E-1 | 1.7E-3 | 1.7E-3 | 5.3E2 | 1.9E0 | 534 | 10 | 204 | 10 | 0.29 |
| Aj | ug/mL | 1.5E0 | 9.9E-1 | 2.6E0 | 2.0E0 | 2.4E0 | 2.2E0 | 1.5E-3 | 1.3E-3 | 6.1E0 | 5.6E0 | 534 | 10 | 204 | 10 | 0.40 |
| Al | mg/mL | 8.8E-5 | 7.8E-5 | 2.5E-4 | 2.3E-4 | 4.1E-4 | 4.4E-4 | 2.3E-6 | 7.8E-6 | 2.2E-3 | 1.5E-3 | 534 | 10 | 204 | 10 | 0.48 |
| An | U/mL | 5.0E1 | 7.5E1 | 1.7E2 | 1.3E2 | 4.4E2 | 1.1E2 | 9.8E-4 | 2.7E1 | 5.5E3 | 3.4E2 | 534 | 10 | 204 | 10 | 0.65 |
| Ao | pg/mL | 8.8E1 | 8.7E1 | 4.6E2 | 1.4E2 | 3.2E3 | 2.0E2 | 1.5E0 | 4.1E0 | 3.9E4 | 6.9E2 | 534 | 10 | 204 | 10 | 0.44 |
| Ap | ng/mL | 3.3E1 | 4.1E1 | 4.8E1 | 5.7E1 | 5.1E1 | 6.2E1 | 8.4E-5 | 4.4E0 | 3.3E2 | 2.1E2 | 534 | 10 | 204 | 10 | 0.53 |
| Ar | ng/mL | 9.7E-1 | 2.1E0 | 1.1E1 | 8.4E0 | 1.8E2 | 1.5E1 | 3.4E-3 | 3.4E-3 | 4.1E3 | 5.0E1 | 534 | 10 | 204 | 10 | 0.61 |
| As | ng/mL | 8.7E-3 | 1.1E-2 | 1.3E-2 | 9.9E-3 | 1.8E-2 | 6.3E-3 | 1.7E-3 | 1.7E-3 | 1.3E-1 | 1.9E-2 | 534 | 10 | 204 | 10 | 0.52 |
| Aw | pg/mL | 1.6E1 | 1.7E1 | 1.7E1 | 1.9E1 | 6.2E0 | 5.2E0 | 2.9E-2 | 1.4E1 | 4.8E1 | 2.9E1 | 534 | 10 | 204 | 10 | 0.64 |
| Ax | ng/mL | 2.1E0 | 1.9E0 | 1.6E1 | 1.1E2 | 6.0E1 | 2.7E2 | 1.2E-2 | 1.0E-4 | 7.7E2 | 8.5E2 | 534 | 10 | 204 | 10 | 0.56 |
| Ba | ng/mL | 6.5E1 | 7.6E2 | 4.5E2 | 1.6E3 | 1.1E3 | 2.5E3 | 2.7E-1 | 4.1E0 | 8.1E3 | 8.1E3 | 534 | 10 | 204 | 10 | 0.67 |
| Bb | ng/mL | 3.1E0 | 3.7E0 | 6.6E0 | 5.1E0 | 1.4E1 | 5.6E0 | 4.1E-3 | 3.5E-1 | 2.5E2 | 1.9E1 | 534 | 10 | 204 | 10 | 0.48 |
| Bc | ng/mL | 3.9E1 | 9.8E1 | 1.1E2 | 2.0E2 | 2.0E2 | 3.0E2 | 1.1E-1 | 2.9E0 | 1.2E3 | 9.9E2 | 534 | 10 | 204 | 10 | 0.64 |
| Bg | ng/mL | 8.5E-2 | 6.7E-1 | 5.4E0 | 1.4E0 | 2.9E1 | 1.6E0 | 5.3E-4 | 2.2E-2 | 4.4E2 | 4.1E0 | 534 | 10 | 204 | 10 | 0.63 |
| Bn | ng/mL | 5.6E-2 | 5.6E-2 | 1.2E0 | 1.4E-1 | 2.0E0 | 1.6E-1 | 5.6E-2 | 5.6E-2 | 9.7E0 | 5.2E-1 | 534 | 10 | 204 | 10 | 0.36 |
| Bo | ng/mL | 1.2E1 | 1.9E1 | 1.4E1 | 2.2E1 | 1.8E1 | 9.2E0 | 1.6E-2 | 1.0E1 | 2.8E2 | 3.6E1 | 534 | 10 | 204 | 10 | 0.73 |
| Ch | ulU/mL | 1.0E0 | 1.3E0 | 1.6E1 | 1.4E1 | 9.4E1 | 3.3E1 | 3.4E-3 | 1.7E-1 | 1.8E3 | 1.1E2 | 534 | 10 | 204 | 10 | 0.55 |
| Co | pg/mL | 3.8E1 | 6.6E1 | 1.7E2 | 2.7E2 | 8.9E2 | 6.3E2 | 1.5E-1 | 8.8E0 | 1.7E4 | 2.1E3 | 534 | 10 | 204 | 10 | 0.57 |
| Cp | ng/mL | 2.2E1 | 2.2E1 | 2.9E1 | 3.4E1 | 3.1E1 | 2.3E1 | 6.0E-1 | 1.1E1 | 3.7E2 | 7.2E1 | 534 | 10 | 204 | 10 | 0.58 |
| Cq | ng/mL | 3.0E-2 | 4.2E-2 | 1.4E-1 | 4.9E-2 | 8.1E-1 | 4.2E-2 | 8.0E-4 | 8.0E-4 | 1.7E1 | 1.3E-1 | 534 | 10 | 204 | 10 | 0.55 |
| Cs | ng/mL | 6.1E1 | 1.8E2 | 3.1E2 | 1.2E3 | 1.1E3 | 2.1E3 | 2.7E-2 | 5.7E0 | 1.8E4 | 5.3E3 | 534 | 10 | 204 | 10 | 0.62 |
| Ct | ng/mL | 5.9E-1 | 5.8E0 | 3.8E1 | 1.6E1 | 1.1E2 | 2.5E1 | 1.1E-4 | 3.8E-2 | 6.2E2 | 7.2E1 | 534 | 10 | 204 | 10 | 0.55 |
| Cu | ng/mL | 2.5E-1 | 7.3E-1 | 5.1E-1 | 8.4E-1 | 1.4E0 | 8.5E-1 | 9.0E-5 | 1.7E-2 | 2.1E1 | 2.9E0 | 534 | 10 | 204 | 10 | 0.66 |
| Cv | ng/mL | 5.5E0 | 3.8E0 | 2.5E1 | 7.6E0 | 6.3E1 | 1.1E1 | 1.4E-4 | 1.0E-1 | 5.3E2 | 3.5E1 | 534 | 10 | 204 | 10 | 0.42 |
| Cw | mIU/mL | 3.0E-2 | 4.1E-2 | 3.9E-2 | 5.0E-2 | 3.3E-2 | 3.3E-2 | 1.5E-4 | 1.1E-2 | 2.4E-1 | 1.2E-1 | 534 | 10 | 204 | 10 | 0.62 |
| Cx | ng/mL | 3.2E-1 | 1.4E-2 | 6.1E1 | 1.1E-1 | 1.1E2 | 2.1E-1 | 9.3E-5 | 9.3E-5 | 3.9E2 | 6.2E-1 | 534 | 10 | 204 | 10 | 0.32 |
| Db | ug/mL | 7.4E0 | 7.8E0 | 9.1E0 | 1.0E1 | 1.0E1 | 5.6E0 | 4.5E-1 | 4.3E0 | 1.4E2 | 2.3E1 | 534 | 10 | 204 | 10 | 0.58 |
| Dc | nmol/L | 1.9E-2 | 2.3E-2 | 6.0E-2 | 4.1E-2 | 1.4E-1 | 5.7E-2 | 5.2E-6 | 1.6E-3 | 1.6E0 | 2.0E-1 | 534 | 10 | 204 | 10 | 0.51 |
| Dd | ug/mL | 7.0E-2 | 4.4E-2 | 1.7E-1 | 1.5E-1 | 2.6E-1 | 2.1E-1 | 8.3E-5 | 6.4E-3 | 1.9E0 | 6.9E-1 | 534 | 10 | 204 | 10 | 0.49 |
| De | ng/mL | 3.4E-3 | 3.4E-3 | 8.1E-2 | 1.2E-1 | 1.5E-1 | 3.5E-1 | 3.4E-3 | 3.4E-3 | 1.2E0 | 1.1E0 | 534 | 10 | 204 | 10 | 0.40 |
| Dg | ng/mL | 3.3E1 | 4.0E1 | 4.5E1 | 4.0E1 | 4.0E1 | 3.6E1 | 1.0E-1 | 1.9E0 | 1.9E2 | 1.2E2 | 534 | 10 | 204 | 10 | 0.47 |
| Di | pg/mL | 1.9E0 | 3.0E0 | 2.2E0 | 2.8E0 | 2.0E0 | 1.5E0 | 1.8E-1 | 1.8E-1 | 1.3E1 | 5.4E0 | 534 | 10 | 204 | 10 | 0.63 |
| Dk | uIU/mL | 1.7E-2 | 2.5E-2 | 7.9E-2 | 1.3E-1 | 4.7E-1 | 3.0E-1 | 1.1E-4 | 5.8E-3 | 8.9E0 | 9.8E-1 | 534 | 10 | 204 | 10 | 0.58 |
| Dl | ng/mL | 2.3E2 | 3.3E2 | 3.1E2 | 3.5E2 | 2.9E2 | 3.3E2 | 1.7E0 | 4.4E0 | 1.5E3 | 8.3E2 | 534 | 10 | 204 | 10 | 0.51 |
| Dp | ng/ml | 2.4E0 | 1.8E0 | 5.4E0 | 1.8E0 | 8.0E0 | 1.5E0 | 3.7E-3 | 4.9E-2 | 5.6E1 | 4.0E0 | 332 | 7 | 193 | 7 | 0.36 |
| Ef | ng/ml | 1.3E-1 | 5.6E-1 | 8.8E-1 | 1.8E0 | 1.9E0 | 3.6E0 | 5.7E-4 | 1.3E-3 | 1.0E1 | 9.9E0 | 398 | 7 | 200 | 7 | 0.49 |
| Wm | % | 4.9E-1 | 3.4E0 | 3.3E1 | 9.4E1 | 1.8E2 | 2.7E2 | 5.4E-2 | 8.5E-2 | 2.4E3 | 8.6E2 | 432 | 10 | 217 | 10 | 0.70 |
| Ed | pg/ml | 5.2E-1 | 5.2E-1 | 5.5E1 | 2.9E1 | 4.0E2 | 4.0E1 | 5.2E-1 | 5.2E-1 | 7.3E3 | 1.0E2 | 332 | 7 | 192 | 7 | 0.51 |
| Tj | pg/mL | 3.7E-1 | 3.7E-1 | 4.9E1 | 8.4E0 | 2.7E2 | 1.4E1 | 3.6E-1 | 3.7E-1 | 3.5E3 | 3.2E1 | 396 | 7 | 203 | 7 | 0.51 |
| Po | pg/ml | 6.9E-1 | 1.2E1 | 8.0E0 | 3.4E1 | 2.2E1 | 6.0E1 | 8.0E-2 | 2.6E-2 | 2.7E2 | 2.2E2 | 932 | 13 | 343 | 13 | 0.63 |
| Et | ng/ml | 1.4E3 | 2.7E3 | 1.7E3 | 2.5E3 | 1.2E3 | 1.6E3 | 7.5E1 | 4.0E2 | 5.0E3 | 5.0E3 | 931 | 13 | 343 | 13 | 0.64 |
| Fa | ng/ml | 4.4E1 | 5.9E1 | 1.2E2 | 8.5E1 | 5.1E2 | 7.0E1 | 3.4E-2 | 1.6E0 | 8.0E3 | 2.2E2 | 327 | 7 | 190 | 7 | 0.61 |
| Ez | ng/ml | 3.8E0 | 6.6E0 | 1.7E1 | 4.1E1 | 5.0E1 | 7.4E1 | 1.3E-2 | 1.3E-2 | 7.1E2 | 2.0E2 | 332 | 7 | 193 | 7 | 0.53 |
| Fb | ng/ml | 2.5E1 | 2.9E1 | 2.3E1 | 2.7E1 | 1.2E1 | 1.3E1 | 5.9E-1 | 4.6E0 | 5.7E1 | 4.0E1 | 328 | 7 | 190 | 7 | 0.62 |
| Ex | ng/ml | 7.6E-2 | 1.2E-1 | 2.2E-1 | 3.2E-1 | 6.5E-1 | 3.3E-1 | 3.5E-5 | 6.2E-2 | 8.9E0 | 9.2E-1 | 293 | 7 | 138 | 7 | 0.70 |
| Fn | ng/ml | 2.1E-1 | 1.8E0 | 5.5E0 | 2.8E0 | 2.4E1 | 3.0E0 | 1.1E-14 | 2.1E-1 | 4.2E2 | 8.7E0 | 332 | 7 | 193 | 7 | 0.58 |
| Fp | ng/ml | 1.3E1 | 4.5E1 | 2.5E1 | 4.9E1 | 2.8E1 | 4.2E1 | 6.0E-3 | 2.3E0 | 1.4E2 | 1.4E2 | 965 | 13 | 344 | 13 | 0.68 |

| Code | Units | Median | | Average | | Standard Deviation | | Minimum | | Maximum | | Number of Samples | | Number of Patients | | AUC |
|------|-------|--------|-----|---------|-----|--------|-----|--------|-----|--------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Fr | ng/ml | 3.9E4 | 3.7E5 | 1.1E5 | 3.9E5 | 1.7E5 | 3.5E5 | 1.9E2 | 7.0E3 | 9.0E5 | 8.9E5 | 1079 | 15 | 348 | 15 | 0.75 |
| Fw | pg/ml | 1.1E0 | 1.1E0 | 5.6E1 | 1.3E1 | 4.4E2 | 1.8E1 | 1.1E-14 | 1.7E-14 | 6.9E3 | 4.2E1 | 398 | 7 | 201 | 7 | 0.53 |
| Gl | pg/ml | 7.2E3 | 1.1E4 | 1.1E4 | 1.4E4 | 9.3E3 | 1.1E4 | 9.1E1 | 1.0E3 | 3.4E4 | 3.0E4 | 388 | 7 | 199 | 7 | 0.60 |
| Gp | U/ml | 1.5E0 | 1.8E0 | 3.9E0 | 1.7E0 | 6.6E0 | 2.0E0 | 1.3E-3 | 1.5E-2 | 6.7E1 | 5.4E0 | 400 | 7 | 201 | 7 | 0.41 |
| Gz | ug/ml | 1.2E0 | 8.4E-1 | 7.8E0 | 5.4E0 | 3.4E1 | 7.9E0 | 2.9E-16 | 3.1E-1 | 4.8E2 | 2.1E1 | 224 | 7 | 126 | 7 | 0.48 |
| Ha | ng/ml | 2.7E0 | 1.3E0 | 9.4E0 | 2.7E0 | 2.0E1 | 3.3E0 | 6.4E-3 | 1.7E-2 | 1.3E2 | 9.7E0 | 330 | 7 | 192 | 7 | 0.38 |
| Nm | pg/ml | 1.4E4 | 1.7E4 | 3.3E4 | 6.5E4 | 7.8E4 | 1.2E5 | 1.0E-9 | 1.0E-9 | 1.6E6 | 4.4E5 | 935 | 13 | 345 | 13 | 0.53 |
| Nn | pg/ml | 1.6E2 | 2.0E3 | 1.7E3 | 1.4E4 | 7.6E3 | 2.3E4 | 1.0E-9 | 1.0E-9 | 1.0E5 | 6.9E4 | 935 | 13 | 345 | 13 | 0.70 |
| No | pg/ml | 1.6E1 | 1.9E1 | 3.6E1 | 2.1E2 | 1.1E2 | 4.5E2 | 1.0E-9 | 1.6E0 | 2.5E3 | 1.4E3 | 935 | 13 | 345 | 13 | 0.58 |
| Nq | pg/ml | 2.0E0 | 5.4E0 | 1.8E1 | 6.5E1 | 7.2E1 | 1.0E2 | 1.0E-9 | 1.0E-9 | 1.2E3 | 3.0E2 | 935 | 13 | 345 | 13 | 0.63 |
| Nr | pg/ml | 9.9E-1 | 2.4E0 | 2.8E1 | 6.8E2 | 1.7E2 | 2.4E3 | 1.0E-9 | 1.0E-9 | 4.1E3 | 8.5E3 | 935 | 13 | 345 | 13 | 0.58 |
| Ns | pg/ml | 1.0E-9 | 1.0E-9 | 9.0E0 | 1.5E0 | 5.1E1 | 4.6E0 | 1.0E-9 | 1.0E-9 | 1.1E3 | 1.6E1 | 935 | 13 | 345 | 13 | 0.52 |
| Nt | pg/ml | 1.0E2 | 1.3E2 | 1.3E2 | 2.0E2 | 1.1E2 | 1.8E2 | 1.0E-9 | 6.6E1 | 1.5E3 | 6.8E2 | 935 | 13 | 345 | 13 | 0.65 |
| Nu | pg/ml | 2.0E1 | 1.2E2 | 5.4E1 | 1.3E2 | 8.9E1 | 1.5E2 | 1.0E-9 | 1.0E-9 | 8.9E2 | 5.8E2 | 935 | 13 | 345 | 13 | 0.76 |
| Lu | pg/ml | 1.0E4 | 5.7E3 | 1.8E4 | 1.1E4 | 6.1E4 | 1.6E4 | 3.5E2 | 1.0E3 | 1.3E6 | 6.1E4 | 938 | 13 | 345 | 13 | 0.35 |
| Lv | pg/ml | 1.0E-9 | 4.3E1 | 1.1E1 | 6.1E1 | 2.2E1 | 4.9E1 | 1.0E-9 | 1.0E-9 | 2.6E2 | 1.9E2 | 938 | 13 | 345 | 13 | 0.88 |
| Lw | pg/ml | 1.0E-9 | 1.0E-9 | 3.9E-1 | 3.0E0 | 4.0E0 | 7.2E0 | 1.0E-9 | 1.0E-9 | 8.0E1 | 2.1E1 | 938 | 13 | 345 | 13 | 0.60 |
| Lx | pg/ml | 1.0E-9 | 2.5E2 | 1.4E2 | 1.4E3 | 4.1E2 | 2.8E3 | 1.0E-9 | 1.0E-9 | 6.2E3 | 1.0E4 | 938 | 13 | 345 | 13 | 0.78 |
| Ly | pg/ml | 1.0E-9 | 1.1E1 | 1.0E1 | 1.0E1 | 2.0E1 | 1.0E1 | 1.0E-9 | 1.0E-9 | 1.6E2 | 2.6E1 | 938 | 13 | 345 | 13 | 0.60 |
| Lz | pg/ml | 1.0E-9 | 1.0E-9 | 3.8E0 | 1.6E1 | 3.0E1 | 5.8E1 | 1.0E-9 | 1.0E-9 | 6.0E2 | 2.1E2 | 938 | 13 | 345 | 13 | 0.50 |
| Ma | pg/ml | 2.8E2 | 2.1E3 | 1.3E3 | 5.0E3 | 3.5E3 | 9.7E3 | 1.0E-9 | 2.4E1 | 6.5E4 | 3.6E4 | 938 | 13 | 345 | 13 | 0.66 |
| Mb | pg/ml | 2.5E1 | 3.4E1 | 3.1E1 | 4.1E1 | 1.5E1 | 2.1E1 | 5.4E0 | 1.9E1 | 2.1E2 | 8.7E1 | 938 | 13 | 345 | 13 | 0.65 |
| Mc | pg/ml | 1.0E-9 | 1.0E-9 | 4.1E-2 | 1.0E-9 | 5.6E-1 | 0.0E0 | 1.0E-9 | 1.0E-9 | 1.3E1 | 1.0E-9 | 938 | 13 | 345 | 13 | 0.49 |
| Md | pg/ml | 1.0E-9 | 1.0E-9 | 3.9E-1 | 1.0E-9 | 3.4E0 | 0.0E0 | 1.0E-9 | 1.0E-9 | 6.8E1 | 1.0E-9 | 938 | 13 | 345 | 13 | 0.47 |
| Me | pg/ml | 3.3E1 | 2.5E1 | 3.2E1 | 2.5E1 | 2.0E1 | 1.9E1 | 1.0E-9 | 3.2E0 | 3.2E2 | 7.9E1 | 938 | 13 | 345 | 13 | 0.31 |
| Mf | pg/ml | 1.0E-9 | 1.0E-9 | 5.2E-1 | 2.6E-1 | 2.9E0 | 6.1E-1 | 1.0E-9 | 1.0E-9 | 5.6E1 | 2.2E0 | 938 | 13 | 345 | 13 | 0.57 |
| Mg | pg/ml | 1.6E0 | 3.6E0 | 7.4E0 | 1.9E1 | 1.3E1 | 4.0E1 | 1.0E-9 | 1.0E-9 | 9.4E1 | 1.5E2 | 938 | 13 | 345 | 13 | 0.58 |
| Mh | pg/ml | 1.0E-9 | 1.0E-9 | 1.3E0 | 3.8E0 | 9.4E0 | 1.1E1 | 1.0E-9 | 1.0E-9 | 1.6E2 | 3.8E1 | 938 | 13 | 345 | 13 | 0.56 |
| Mi | pg/ml | 1.0E-9 | 1.0E-9 | 4.8E-1 | 2.6E1 | 5.2E0 | 4.6E1 | 1.0E-9 | 1.0E-9 | 1.2E2 | 1.6E2 | 938 | 13 | 345 | 13 | 0.72 |
| Mj | pg/ml | 1.0E-9 | 1.0E-9 | 4.3E0 | 4.5E1 | 2.4E1 | 1.5E2 | 1.0E-9 | 1.0E-9 | 4.6E2 | 5.4E2 | 938 | 13 | 345 | 13 | 0.56 |
| Mk | pg/ml | 9.1E-1 | 9.3E0 | 1.4E1 | 4.4E2 | 8.4E1 | 1.5E3 | 1.0E-9 | 1.0E-9 | 1.7E3 | 5.6E3 | 938 | 13 | 345 | 13 | 0.62 |
| Ml | pg/ml | 1.0E-9 | 1.0E-9 | 4.9E0 | 2.5E0 | 7.2E1 | 5.0E0 | 1.0E-9 | 1.0E-9 | 2.1E3 | 1.6E1 | 938 | 13 | 345 | 13 | 0.57 |
| Mm | pg/ml | 5.9E2 | 4.8E2 | 1.0E3 | 2.0E3 | 1.2E3 | 2.5E3 | 1.0E-9 | 1.0E-9 | 1.1E4 | 6.9E3 | 938 | 13 | 345 | 13 | 0.55 |
| Mn | pg/ml | 5.5E0 | 9.3E0 | 1.0E1 | 1.4E1 | 2.2E1 | 1.7E1 | 1.0E-9 | 6.9E-1 | 3.5E2 | 6.6E1 | 938 | 13 | 345 | 13 | 0.63 |
| Mp | pg/ml | 1.0E-9 | 7.1E0 | 8.9E0 | 6.2E1 | 2.9E1 | 9.1E1 | 1.0E-9 | 1.0E-9 | 6.3E2 | 2.7E2 | 937 | 13 | 345 | 13 | 0.64 |
| Mq | pg/ml | 1.0E-9 | 1.0E-9 | 2.7E0 | 1.4E1 | 1.6E1 | 4.0E1 | 1.0E-9 | 1.0E-9 | 2.4E2 | 1.4E2 | 937 | 13 | 345 | 13 | 0.55 |
| Mr | pg/ml | 1.0E-9 | 1.0E-9 | 2.5E1 | 9.1E2 | 1.4E2 | 3.3E3 | 1.0E-9 | 1.0E-9 | 2.2E3 | 1.2E4 | 937 | 13 | 345 | 13 | 0.57 |
| Ms | pg/ml | 4.1E2 | 2.6E2 | 5.6E2 | 3.3E2 | 6.6E2 | 3.4E2 | 1.0E-9 | 1.0E-9 | 5.9E3 | 8.6E2 | 937 | 13 | 345 | 13 | 0.39 |
| Mt | pg/ml | 2.5E-1 | 9.0E0 | 6.8E0 | 2.1E1 | 4.1E1 | 2.8E1 | 1.0E-9 | 1.0E-9 | 8.7E2 | 7.8E1 | 937 | 13 | 345 | 13 | 0.76 |
| Mu | pg/ml | 1.0E-9 | 8.4E-1 | 1.2E0 | 9.2E0 | 1.0E1 | 1.5E1 | 1.0E-9 | 1.0E-9 | 2.3E2 | 4.8E1 | 937 | 13 | 345 | 13 | 0.72 |
| Mv | pg/ml | 1.0E-9 | 5.9E1 | 6.8E1 | 1.9E2 | 3.1E2 | 2.9E2 | 1.0E-9 | 1.0E-9 | 4.5E3 | 9.0E2 | 937 | 13 | 345 | 13 | 0.71 |
| Mw | pg/ml | 3.8E1 | 6.6E2 | 5.0E2 | 1.4E3 | 3.2E3 | 2.3E3 | 1.0E-9 | 1.0E-9 | 6.2E4 | 8.5E3 | 937 | 13 | 345 | 13 | 0.68 |
| Mx | pg/ml | 1.0E-9 | 1.0E-9 | 2.4E-1 | 3.6E-1 | 1.3E0 | 8.6E-1 | 1.0E-9 | 1.0E-9 | 3.2E1 | 3.0E0 | 937 | 13 | 345 | 13 | 0.62 |
| My | pg/ml | 1.0E-9 | 2.1E2 | 4.5E2 | 5.3E2 | 2.9E3 | 9.3E2 | 1.0E-9 | 1.0E-9 | 3.9E4 | 3.4E3 | 937 | 13 | 345 | 13 | 0.75 |
| Mz | pg/ml | 1.1E1 | 2.2E1 | 2.7E1 | 5.3E1 | 6.3E1 | 6.3E1 | 1.0E-9 | 5.1E0 | 1.2E3 | 2.0E2 | 937 | 13 | 345 | 13 | 0.68 |
| Na | pg/ml | 1.0E-9 | 1.0E-9 | 6.6E-1 | 8.8E-1 | 2.6E0 | 3.0E0 | 1.0E-9 | 1.0E-9 | 3.6E1 | 1.1E1 | 937 | 13 | 345 | 13 | 0.48 |
| Nb | pg/ml | 2.1E0 | 4.6E0 | 3.8E0 | 2.4E1 | 1.1E1 | 6.5E1 | 1.0E-9 | 1.0E-9 | 2.3E2 | 2.4E2 | 937 | 13 | 345 | 13 | 0.68 |
| Nc | pg/ml | 3.4E2 | 1.7E2 | 5.6E2 | 2.3E2 | 7.2E2 | 2.3E2 | 1.0E-9 | 1.0E-9 | 9.2E3 | 6.7E2 | 937 | 13 | 345 | 13 | 0.40 |
| Nd | pg/ml | 2.9E1 | 1.0E1 | 2.7E1 | 4.6E1 | 4.4E1 | 5.8E1 | 1.0E-9 | 1.0E-9 | 1.2E3 | 1.5E2 | 937 | 13 | 345 | 13 | 0.51 |
| Ne | pg/ml | 4.4E2 | 2.7E2 | 5.7E2 | 5.0E2 | 5.7E2 | 9.6E2 | 1.0E-9 | 1.3E1 | 7.0E3 | 3.6E3 | 937 | 13 | 345 | 13 | 0.37 |
| Nf | pg/ml | 1.0E-9 | 1.0E-9 | 2.6E0 | 7.9E0 | 9.4E0 | 2.3E1 | 1.0E-9 | 1.0E-9 | 1.6E2 | 8.2E1 | 937 | 13 | 345 | 13 | 0.51 |
| Ng | pg/ml | 1.9E1 | 4.4E0 | 1.3E2 | 1.1E2 | 2.5E2 | 1.5E2 | 1.0E-9 | 1.0E-9 | 2.3E3 | 4.7E2 | 937 | 13 | 345 | 13 | 0.47 |
| Nh | pg/ml | 6.9E1 | 3.2E1 | 9.0E1 | 8.0E1 | 8.2E1 | 1.3E2 | 1.0E-9 | 2.2E0 | 5.6E2 | 5.1E2 | 937 | 13 | 345 | 13 | 0.35 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ni | pg/ml | 1.0E-9 | 4.4E1 | 7.2E1 | 1.3E2 | 1.2E2 | 1.5E2 | 1.0E-9 | 1.0E-9 | 7.1E2 | 3.9E2 | 937 | 13 | 345 | 13 | 0.63 |
| Nj | pg/ml | 7.5E0 | 4.8E0 | 1.1E1 | 5.9E0 | 1.2E1 | 5.5E0 | 1.0E-9 | 1.0E-9 | 1.1E2 | 2.0E1 | 937 | 13 | 345 | 13 | 0.38 |
| Nk | pg/ml | 1.7E1 | 2.0E1 | 3.3E1 | 1.9E1 | 3.9E1 | 1.1E1 | 1.0E-9 | 1.0E-9 | 2.0E2 | 3.7E1 | 937 | 13 | 345 | 13 | 0.48 |
| Nl | pg/ml | 4.5E1 | 2.2E1 | 6.1E1 | 4.3E1 | 6.8E1 | 5.0E1 | 1.0E-9 | 1.0E-9 | 1.1E3 | 1.8E2 | 937 | 13 | 345 | 13 | 0.41 |
| Tz | pg/ml | 5.1E3 | 6.2E3 | 2.0E4 | 5.8E3 | 1.3E5 | 4.7E3 | 1.0E-9 | 6.3E2 | 2.1E6 | 1.1E4 | 334 | 7 | 191 | 7 | 0.46 |
| Ua | pg/ml | 3.8E3 | 4.7E3 | 2.0E4 | 2.1E4 | 1.2E5 | 3.6E4 | 1.0E-9 | 2.0E3 | 2.1E6 | 9.9E4 | 334 | 7 | 191 | 7 | 0.57 |
| Ub | pg/ml | 5.6E2 | 2.4E2 | 8.3E2 | 3.4E2 | 1.0E3 | 2.3E2 | 1.0E-9 | 1.4E2 | 9.8E3 | 7.8E2 | 334 | 7 | 191 | 7 | 0.33 |
| Ue | pg/ml | 2.7E1 | 1.5E1 | 3.8E1 | 2.6E1 | 4.1E1 | 3.8E1 | 9.8E-2 | 4.5E0 | 4.4E2 | 1.1E2 | 334 | 7 | 191 | 7 | 0.27 |
| Uc | pg/ml | 8.9E2 | 5.0E2 | 1.7E3 | 1.2E3 | 2.6E3 | 1.9E3 | 1.0E-9 | 4.1E1 | 2.9E4 | 5.4E3 | 334 | 7 | 191 | 7 | 0.36 |
| Ud | pg/ml | 1.0E-9 | 1.0E-9 | 1.3E0 | 1.0E-9 | 2.1E1 | 0.0E0 | 1.0E-9 | 1.0E-9 | 3.9E2 | 1.0E-9 | 334 | 7 | 191 | 7 | 0.50 |
| Hq | pg/ml | 1.1E0 | 2.4E0 | 9.6E1 | 2.6E1 | 1.6E3 | 8.2E1 | 1.0E-9 | 1.0E-9 | 3.4E4 | 3.0E2 | 933 | 13 | 344 | 13 | 0.61 |
| Hr | pg/ml | 1.1E2 | 8.2E1 | 7.5E2 | 9.6E2 | 1.6E3 | 2.9E3 | 1.0E-9 | 2.7E1 | 1.7E4 | 1.1E4 | 933 | 13 | 344 | 13 | 0.43 |
| Hu | pg/ml | 5.3E0 | 3.2E2 | 3.0E3 | 1.2E3 | 2.6E4 | 1.8E3 | 1.0E-9 | 1.0E-9 | 6.3E5 | 5.9E3 | 933 | 13 | 344 | 13 | 0.73 |
| Hv | pg/ml | 1.4E0 | 3.4E0 | 3.3E0 | 8.2E0 | 1.2E1 | 1.6E1 | 1.0E-9 | 9.7E-1 | 2.5E2 | 5.9E1 | 933 | 13 | 344 | 13 | 0.74 |
| Hw | pg/ml | 6.4E0 | 3.2E0 | 1.8E1 | 2.8E2 | 6.9E1 | 9.5E2 | 1.0E-9 | 1.0E-9 | 1.7E3 | 3.4E3 | 933 | 13 | 344 | 13 | 0.42 |
| Hx | pg/ml | 8.8E0 | 2.2E1 | 3.7E1 | 1.8E2 | 3.1E2 | 5.4E2 | 1.0E-9 | 2.9E0 | 9.3E3 | 2.0E3 | 933 | 13 | 344 | 13 | 0.71 |
| Ib | ng/ml | 4.9E-2 | 2.0E-2 | 1.7E0 | 9.2E-1 | 6.5E0 | 2.3E0 | 1.0E-9 | 1.0E-9 | 5.3E1 | 6.1E0 | 324 | 7 | 190 | 7 | 0.45 |
| Ic | U/ml | 2.2E2 | 2.2E2 | 1.0E3 | 2.6E2 | 6.6E3 | 1.6E2 | 1.5E0 | 2.4E1 | 9.3E4 | 5.4E2 | 324 | 7 | 190 | 7 | 0.60 |
| Id | U/ml | 6.9E-1 | 9.5E-1 | 1.4E0 | 1.1E0 | 2.4E0 | 1.0E0 | 1.0E-9 | 2.4E-1 | 2.3E1 | 3.2E0 | 324 | 7 | 190 | 7 | 0.51 |
| Tt | pg/ml | 1.6E2 | 1.8E2 | 1.7E2 | 1.7E2 | 5.1E1 | 4.2E1 | 4.3E1 | 1.2E2 | 3.6E2 | 2.3E2 | 308 | 7 | 184 | 7 | 0.50 |
| To | pg/ml | 1.6E0 | 2.7E0 | 1.9E0 | 2.6E0 | 2.3E0 | 8.6E-1 | 1.0E-9 | 1.8E0 | 2.3E1 | 4.1E0 | 321 | 7 | 188 | 7 | 0.71 |
| Tr | pg/ml | 3.0E0 | 3.1E0 | 5.9E0 | 3.9E0 | 1.8E1 | 3.6E0 | 1.0E-9 | 1.4E0 | 3.1E2 | 1.2E1 | 317 | 7 | 187 | 7 | 0.50 |
| Tn | pg/ml | 2.8E1 | 3.7E1 | 7.4E1 | 9.0E1 | 1.9E2 | 9.7E1 | 1.0E-9 | 1.3E1 | 1.8E3 | 2.3E2 | 321 | 7 | 188 | 7 | 0.61 |
| Tv | ng/ml | 1.2E1 | 6.2E0 | 2.3E1 | 1.2E1 | 6.2E1 | 1.4E1 | 1.0E-9 | 1.0E-9 | 7.9E2 | 3.1E1 | 321 | 7 | 188 | 7 | 0.43 |
| Ih | ng/ml | 7.2E1 | 1.9E2 | 2.4E2 | 3.2E2 | 4.8E2 | 3.9E2 | 1.0E-9 | 2.4E0 | 7.4E3 | 1.2E3 | 937 | 13 | 344 | 13 | 0.63 |
| Ii | ng/ml | 9.5E1 | 7.2E1 | 2.4E2 | 8.5E2 | 6.6E2 | 2.5E3 | 1.0E-9 | 7.5E-1 | 1.0E4 | 9.2E3 | 937 | 13 | 344 | 13 | 0.47 |
| Ij | ng/ml | 7.6E1 | 1.3E2 | 1.7E2 | 7.4E2 | 5.7E2 | 1.8E3 | 1.6E-1 | 9.5E0 | 6.4E3 | 6.4E3 | 924 | 13 | 342 | 13 | 0.63 |
| Ik | ng/ml | 1.3E1 | 1.9E2 | 7.9E2 | 3.8E2 | 8.0E3 | 5.0E2 | 5.9E-1 | 6.2E-1 | 1.2E5 | 1.5E3 | 932 | 13 | 342 | 13 | 0.66 |
| Il | ng/ml | 3.3E2 | 2.0E2 | 1.3E3 | 2.3E3 | 2.8E3 | 4.6E3 | 1.0E-9 | 1.0E-9 | 1.3E4 | 1.2E4 | 916 | 12 | 342 | 12 | 0.42 |
| Im | ng/ml | 2.1E2 | 7.0E2 | 4.0E2 | 8.7E2 | 7.6E2 | 1.1E3 | 1.3E1 | 2.2E1 | 1.5E4 | 4.0E3 | 931 | 13 | 343 | 13 | 0.68 |
| In | ng/ml | 3.3E0 | 5.4E-1 | 2.1E1 | 5.3E1 | 1.5E2 | 1.6E2 | 1.0E-9 | 1.0E-9 | 3.9E3 | 5.9E2 | 937 | 13 | 344 | 13 | 0.40 |
| Hb | ng/ml | 2.5E1 | 8.9E0 | 3.6E1 | 1.5E1 | 3.5E1 | 1.2E1 | 4.8E-1 | 1.5E0 | 2.1E2 | 3.2E1 | 333 | 7 | 191 | 7 | 0.30 |
| Hc | pg/ml | 6.6E2 | 1.7E3 | 3.4E3 | 3.5E3 | 1.2E4 | 4.1E3 | 1.0E-9 | 3.4E2 | 1.0E5 | 1.1E4 | 333 | 7 | 191 | 7 | 0.64 |
| Hf | ng/ml | 1.6E2 | 1.6E2 | 3.7E2 | 2.8E2 | 5.1E2 | 3.8E2 | 1.0E-9 | 1.0E-9 | 3.2E3 | 1.0E3 | 333 | 7 | 191 | 7 | 0.46 |
| Io | ng/ml | 8.3E3 | 8.0E3 | 2.5E4 | 5.0E4 | 1.4E5 | 1.5E5 | 1.0E-9 | 9.1E2 | 4.0E6 | 5.5E5 | 928 | 13 | 344 | 13 | 0.47 |
| Ip | ng/ml | 1.0E1 | 3.0E1 | 2.0E1 | 3.9E1 | 2.4E1 | 4.3E1 | 1.0E-9 | 1.1E-2 | 2.6E2 | 1.4E2 | 928 | 13 | 344 | 13 | 0.63 |
| Iq | ug/ml | 1.0E-1 | 2.5E-1 | 3.0E1 | 5.9E1 | 6.3E2 | 2.1E2 | 1.0E-9 | 1.0E-9 | 1.4E4 | 7.5E2 | 928 | 13 | 344 | 13 | 0.49 |
| Ir | ug/ml | 3.5E-1 | 1.3E0 | 3.4E0 | 1.1E1 | 2.4E1 | 3.1E1 | 1.0E-9 | 3.5E-2 | 5.1E2 | 1.1E2 | 927 | 13 | 344 | 13 | 0.69 |
| Is | ng/ml | 1.6E0 | 4.3E1 | 6.4E0 | 2.7E1 | 2.2E1 | 6.2E1 | 1.0E-9 | 4.3E-1 | 5.5E2 | 2.3E2 | 928 | 13 | 344 | 13 | 0.70 |
| It | ng/ml | 2.0E0 | 1.6E0 | 2.3E1 | 5.7E1 | 1.4E2 | 1.6E2 | 1.0E-9 | 1.0E-9 | 2.8E3 | 5.9E2 | 928 | 13 | 344 | 13 | 0.52 |
| Iu | ng/ml | 2.2E2 | 2.6E2 | 1.3E3 | 3.1E3 | 4.0E3 | 7.0E3 | 1.0E-9 | 1.0E-9 | 2.9E4 | 2.4E4 | 928 | 13 | 344 | 13 | 0.53 |
| Iv | ng/ml | 1.3E1 | 3.0E1 | 6.0E1 | 5.4E2 | 5.4E2 | 1.8E3 | 1.0E-9 | 4.1E-1 | 1.6E4 | 6.4E3 | 927 | 13 | 344 | 13 | 0.66 |
| Iz | ng/ml | 1.4E2 | 2.8E2 | 5.9E2 | 4.1E2 | 3.5E3 | 4.7E2 | 9.2E-1 | 8.8E-1 | 6.2E4 | 1.1E3 | 333 | 7 | 191 | 7 | 0.48 |
| Rc | pg/ml | 5.6E3 | 6.8E3 | 7.1E3 | 6.2E3 | 5.7E3 | 5.3E3 | 1.9E2 | 9.6E2 | 3.9E4 | 1.6E4 | 331 | 7 | 191 | 7 | 0.45 |
| Rb | pg/ml | 8.2E-1 | 1.1E0 | 2.6E0 | 1.7E0 | 4.2E0 | 1.9E0 | 1.0E-9 | 1.0E-9 | 4.3E1 | 5.4E0 | 331 | 7 | 191 | 7 | 0.54 |
| Pz | ng/ml | 3.8E3 | 1.0E4 | 8.1E3 | 6.6E3 | 3.7E4 | 4.8E3 | 1.3E1 | 4.0E1 | 1.0E6 | 1.4E4 | 929 | 13 | 342 | 13 | 0.58 |
| Qa | ng/ml | 3.5E3 | 1.1E4 | 6.3E3 | 1.2E4 | 7.4E3 | 8.8E3 | 1.2E1 | 9.4E2 | 5.2E4 | 3.1E4 | 929 | 13 | 342 | 13 | 0.72 |
| Qb | ng/ml | 9.7E1 | 2.1E2 | 2.1E2 | 3.5E2 | 4.8E2 | 4.2E2 | 7.9E-1 | 3.2E1 | 8.3E3 | 1.6E3 | 929 | 13 | 342 | 13 | 0.65 |
| Qc | ng/ml | 2.3E2 | 4.1E2 | 6.3E2 | 4.1E2 | 5.5E3 | 3.5E2 | 1.0E-9 | 3.2E1 | 1.7E5 | 1.2E3 | 929 | 13 | 342 | 13 | 0.55 |
| Qd | ng/ml | 9.2E3 | 2.3E4 | 2.1E4 | 4.8E4 | 7.7E4 | 6.2E4 | 1.5E2 | 1.9E3 | 2.0E6 | 2.2E5 | 929 | 13 | 342 | 13 | 0.68 |
| Qe | ng/ml | 9.2E2 | 2.3E3 | 1.8E3 | 3.8E3 | 3.8E3 | 3.7E3 | 1.0E-9 | 1.2E2 | 9.7E4 | 1.4E4 | 929 | 13 | 342 | 13 | 0.73 |
| Jd | ng/ml | 9.4E-1 | 3.1E0 | 5.8E0 | 4.5E0 | 3.8E1 | 6.2E0 | 1.0E-9 | 1.0E-9 | 6.5E2 | 1.8E1 | 332 | 7 | 193 | 7 | 0.63 |
| Jc | ng/ml | 1.0E-9 | 1.5E0 | 2.0E0 | 1.5E0 | 7.0E0 | 1.3E0 | 1.0E-9 | 1.0E-9 | 8.8E1 | 2.9E0 | 332 | 7 | 193 | 7 | 0.62 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Jf | ng/ml | 1.0E-9 | 8.7E-1 | 1.1E0 | 1.5E0 | 2.2E0 | 2.0E0 | 1.0E-9 | 1.0E-9 | 1.2E1 | 5.6E0 | 332 | 7 | 193 | 7 | 0.65 |
| Jg | ng/ml | 4.9E2 | 1.3E3 | 8.2E2 | 2.0E3 | 1.0E3 | 1.9E3 | 1.0E-9 | 8.4E1 | 1.0E4 | 5.4E3 | 933 | 13 | 344 | 13 | 0.68 |
| Jh | ng/ml | 3.0E0 | 2.9E1 | 2.7E1 | 7.8E1 | 1.1E2 | 9.6E1 | 1.0E-9 | 1.0E-9 | 1.6E3 | 2.9E2 | 933 | 13 | 344 | 13 | 0.73 |
| Ji | ng/ml | 5.3E1 | 6.9E1 | 7.7E1 | 2.8E2 | 8.0E1 | 4.9E2 | 1.0E-9 | 2.3E1 | 6.9E2 | 1.8E3 | 933 | 13 | 344 | 13 | 0.69 |
| Sr | pg/mL | 3.8E2 | 6.7E2 | 8.5E2 | 1.3E3 | 1.3E3 | 1.5E3 | 1.0E-9 | 7.9E1 | 9.8E3 | 4.1E3 | 322 | 7 | 188 | 7 | 0.60 |
| Ss | pg/mL | 9.5E4 | 2.0E5 | 1.5E5 | 1.2E5 | 1.8E5 | 1.0E5 | 2.7E3 | 7.8E3 | 1.8E6 | 2.0E5 | 322 | 7 | 188 | 7 | 0.45 |
| St | pg/mL | 2.6E7 | 5.3E7 | 5.6E7 | 1.1E8 | 9.4E7 | 1.3E8 | 1.0E-9 | 4.4E6 | 1.2E9 | 3.0E8 | 326 | 7 | 189 | 7 | 0.55 |
| Ra | pg/ml | 1.0E-9 | 1.0E-9 | 6.1E-1 | 1.6E-1 | 1.2E0 | 2.7E-1 | 1.0E-9 | 1.0E-9 | 7.3E0 | 5.6E-1 | 331 | 7 | 191 | 7 | 0.47 |
| Qz | pg/ml | 1.0E1 | 3.9E1 | 6.3E1 | 5.9E1 | 1.0E2 | 6.5E1 | 1.0E-9 | 1.0E-9 | 7.1E2 | 1.5E2 | 331 | 7 | 191 | 7 | 0.51 |
| Qy | pg/ml | 4.4E-1 | 5.3E-1 | 1.6E1 | 1.1E2 | 7.6E1 | 2.7E2 | 1.0E-9 | 1.0E-9 | 6.5E2 | 7.3E2 | 331 | 7 | 191 | 7 | 0.57 |
| Qx | pg/ml | 1.0E-9 | 1.0E-9 | 5.4E0 | 1.2E0 | 4.5E1 | 2.6E0 | 1.0E-9 | 1.0E-9 | 5.8E2 | 7.1E0 | 331 | 7 | 191 | 7 | 0.63 |
| Qw | pg/ml | 1.0E-9 | 1.0E-9 | 2.9E0 | 4.5E-1 | 1.4E1 | 1.2E0 | 1.0E-9 | 1.0E-9 | 1.3E2 | 3.2E0 | 331 | 7 | 191 | 7 | 0.37 |
| Qv | pg/ml | 2.3E4 | 4.9E3 | 3.5E4 | 6.4E3 | 5.5E4 | 5.1E3 | 1.0E-9 | 1.5E3 | 7.4E5 | 1.5E4 | 331 | 7 | 191 | 7 | 0.15 |
| Qu | pg/ml | 7.8E0 | 5.3E1 | 8.2E1 | 1.4E2 | 1.7E2 | 2.6E2 | 1.0E-9 | 1.0E-9 | 9.8E2 | 7.3E2 | 331 | 7 | 191 | 7 | 0.57 |
| Qt | pg/ml | 1.0E1 | 5.7E0 | 4.9E1 | 5.6E1 | 1.2E2 | 8.7E1 | 1.0E-9 | 1.0E-9 | 1.0E3 | 2.2E2 | 331 | 7 | 191 | 7 | 0.52 |
| Qh | ng/ml | 1.7E1 | 3.5E1 | 4.0E1 | 3.3E1 | 7.7E1 | 2.6E1 | 1.0E-9 | 3.3E0 | 8.0E2 | 7.0E1 | 331 | 7 | 191 | 7 | 0.57 |
| Qg | ng/ml | 8.3E0 | 8.4E0 | 2.0E1 | 8.1E0 | 6.3E1 | 2.3E0 | 5.1E-2 | 4.9E0 | 1.0E3 | 1.2E1 | 331 | 7 | 191 | 7 | 0.48 |
| Jj | ng/ml | 6.0E2 | 1.4E2 | 1.7E3 | 2.3E2 | 1.2E4 | 2.7E2 | 2.3E0 | 8.7E0 | 3.4E5 | 1.0E3 | 933 | 13 | 344 | 13 | 0.19 |
| Jk | ng/ml | 3.1E0 | 2.3E1 | 2.1E1 | 4.6E1 | 4.7E1 | 6.4E1 | 1.0E-9 | 1.0E-1 | 3.9E2 | 1.8E2 | 933 | 13 | 344 | 13 | 0.63 |
| Jl | ng/ml | 4.4E-1 | 2.5E0 | 1.9E0 | 1.7E1 | 5.6E0 | 4.3E1 | 7.6E-4 | 7.2E-2 | 1.1E2 | 1.6E2 | 933 | 13 | 344 | 13 | 0.73 |
| Jm | ng/ml | 1.8E1 | 8.8E0 | 5.8E1 | 4.4E1 | 1.3E2 | 6.7E1 | 1.0E-9 | 4.0E-1 | 2.1E3 | 2.0E2 | 933 | 13 | 344 | 13 | 0.45 |
| Jn | pg/ml | 4.0E-1 | 1.1E0 | 2.5E0 | 2.4E0 | 2.2E1 | 3.5E0 | 1.0E-9 | 1.0E-9 | 6.2E2 | 9.5E0 | 932 | 13 | 344 | 13 | 0.62 |
| Jo | pg/ml | 3.6E3 | 1.9E3 | 4.7E3 | 6.2E3 | 3.8E3 | 1.0E4 | 2.0E1 | 2.4E1 | 2.4E4 | 3.8E4 | 933 | 13 | 344 | 13 | 0.41 |
| Jp | pg/ml | 7.0E4 | 1.0E5 | 7.4E4 | 1.0E5 | 3.8E4 | 2.9E4 | 5.8E2 | 6.8E4 | 3.8E5 | 1.7E5 | 933 | 13 | 344 | 13 | 0.77 |
| Jq | pg/ml | 9.4E1 | 7.5E1 | 1.5E2 | 3.8E2 | 2.1E2 | 1.0E3 | 1.0E0 | 1.3E1 | 4.0E3 | 3.7E3 | 933 | 13 | 344 | 13 | 0.47 |
| Jr | pg/ml | 5.2E0 | 1.2E1 | 3.3E1 | 3.1E1 | 3.6E2 | 5.2E1 | 1.0E-9 | 1.0E-9 | 1.1E4 | 1.9E2 | 933 | 13 | 344 | 13 | 0.67 |
| Js | pg/ml | 1.3E1 | 1.2E1 | 4.9E1 | 2.2E1 | 3.6E2 | 2.4E1 | 1.0E-9 | 2.7E0 | 1.0E4 | 9.4E1 | 933 | 13 | 344 | 13 | 0.52 |
| Jt | pg/ml | 2.6E3 | 2.3E3 | 3.2E3 | 4.9E3 | 2.4E3 | 8.6E3 | 2.2E1 | 1.5E2 | 2.2E4 | 3.3E4 | 933 | 13 | 344 | 13 | 0.46 |
| Ju | mIU/ml | 8.7E0 | 8.1E0 | 1.9E1 | 1.1E1 | 2.9E1 | 1.1E1 | 4.8E-2 | 1.0E0 | 2.3E2 | 3.5E1 | 332 | 7 | 193 | 7 | 0.48 |
| Jv | mIU/ml | 1.2E1 | 8.7E0 | 3.3E1 | 7.7E0 | 5.7E1 | 5.3E0 | 9.4E-3 | 1.8E-1 | 4.4E2 | 1.3E1 | 332 | 7 | 193 | 7 | 0.38 |
| Jy | ng/ml | 1.6E-3 | 1.6E-3 | 2.1E-3 | 1.9E-3 | 3.8E-3 | 1.1E-3 | 1.0E-9 | 8.6E-4 | 5.2E-2 | 4.0E-3 | 332 | 7 | 193 | 7 | 0.51 |
| Kc | pg/ml | 2.6E1 | 1.3E2 | 4.6E1 | 1.4E2 | 4.9E1 | 1.0E2 | 1.0E-9 | 1.0E-9 | 2.7E2 | 3.2E2 | 333 | 7 | 191 | 7 | 0.77 |
| Kd | pg/ml | 1.0E-9 | 5.7E2 | 2.1E2 | 9.0E2 | 6.4E2 | 9.8E2 | 1.0E-9 | 1.0E-9 | 5.0E3 | 2.3E3 | 333 | 7 | 191 | 7 | 0.77 |
| Ke | pg/ml | 1.3E4 | 1.4E4 | 1.5E4 | 1.6E4 | 1.1E4 | 1.5E4 | 3.4E2 | 3.5E3 | 7.0E4 | 4.9E4 | 333 | 7 | 191 | 7 | 0.49 |
| Kf | pg/mL | 7.2E0 | 9.4E0 | 7.4E0 | 7.8E0 | 6.0E0 | 7.1E0 | 1.0E-9 | 1.0E-9 | 4.4E1 | 1.8E1 | 333 | 7 | 191 | 7 | 0.53 |
| Kg | pg/mL | 1.1E3 | 7.6E2 | 1.9E3 | 6.3E3 | 2.4E3 | 1.3E4 | 7.3E1 | 2.1E2 | 2.2E4 | 3.6E4 | 333 | 7 | 191 | 7 | 0.46 |
| Ki | pg/ml | 5.9E1 | 6.4E1 | 7.0E1 | 7.4E1 | 5.3E1 | 1.9E1 | 1.0E-9 | 5.9E1 | 3.8E2 | 1.1E2 | 332 | 7 | 191 | 7 | 0.61 |
| Kj | pg/ml | 1.0E3 | 4.6E2 | 1.6E3 | 1.5E3 | 1.6E3 | 2.8E3 | 1.4E1 | 3.9E1 | 1.0E4 | 7.7E3 | 333 | 7 | 191 | 7 | 0.34 |
| Kk | pg/ml | 6.9E0 | 3.3E1 | 1.2E1 | 3.4E1 | 1.5E1 | 2.5E1 | 1.0E-9 | 7.8E0 | 1.6E2 | 6.1E1 | 333 | 7 | 191 | 7 | 0.80 |
| Kl | pg/ml | 2.0E4 | 1.7E4 | 2.8E4 | 2.4E4 | 2.5E4 | 2.3E4 | 1.6E2 | 6.2E2 | 1.6E5 | 5.0E4 | 333 | 7 | 191 | 7 | 0.41 |
| Kn | pg/ml | 3.0E1 | 6.2E1 | 6.3E1 | 1.2E2 | 1.0E2 | 1.5E2 | 1.0E-9 | 1.0E-9 | 7.3E2 | 4.1E2 | 333 | 7 | 191 | 7 | 0.60 |
| Ko | pg/ml | 3.5E2 | 7.0E2 | 5.0E2 | 6.7E2 | 5.1E2 | 4.4E2 | 1.0E-9 | 1.5E2 | 3.8E3 | 1.5E3 | 333 | 7 | 191 | 7 | 0.66 |
| Kp | pg/ml | 3.4E2 | 5.9E2 | 3.6E2 | 4.6E2 | 2.6E2 | 3.3E2 | 1.0E-9 | 6.4E1 | 1.7E3 | 7.9E2 | 333 | 7 | 191 | 7 | 0.60 |
| Kq | pg/ml | 3.3E2 | 6.1E2 | 4.7E2 | 1.2E3 | 7.6E2 | 1.9E3 | 1.6E0 | 7.0E1 | 9.8E3 | 5.5E3 | 325 | 7 | 185 | 7 | 0.61 |
| Kr | pg/ml | 4.5E-1 | 1.0E-9 | 2.4E0 | 1.2E0 | 4.6E0 | 1.5E0 | 1.0E-9 | 1.0E-9 | 3.9E1 | 3.3E0 | 325 | 7 | 185 | 7 | 0.44 |
| Ks | pg/ml | 1.4E4 | 4.9E3 | 2.0E4 | 9.6E3 | 1.8E4 | 9.3E3 | 5.1E1 | 4.2E2 | 1.1E5 | 2.4E4 | 325 | 7 | 185 | 7 | 0.33 |
| Kx | ng/ml | 1.1E-4 | 8.9E-3 | 6.7E-3 | 7.8E-3 | 1.3E-2 | 6.4E-3 | 1.0E-9 | 1.0E-9 | 1.0E-1 | 1.5E-2 | 330 | 7 | 190 | 7 | 0.63 |
| Ky | ng/ml | 9.8E-2 | 3.8E-1 | 3.9E-1 | 3.6E-1 | 8.5E-1 | 2.1E-1 | 1.0E-9 | 8.8E-2 | 6.3E0 | 6.0E-1 | 330 | 7 | 190 | 7 | 0.72 |
| Kz | ng/ml | 1.0E-9 | 1.0E-9 | 3.4E-3 | 5.0E-3 | 5.6E-3 | 9.3E-3 | 1.0E-9 | 1.0E-9 | 3.6E-2 | 2.5E-2 | 330 | 7 | 190 | 7 | 0.53 |
| Ld | pg/ml | 1.0E-9 | 7.3E0 | 3.6E0 | 8.0E0 | 8.6E0 | 1.0E1 | 1.0E-9 | 1.0E-9 | 8.4E1 | 2.9E1 | 331 | 7 | 190 | 7 | 0.68 |
| Lh | pg/ml | 1.3E4 | 3.0E4 | 2.1E4 | 6.3E4 | 2.6E4 | 1.1E5 | 1.0E-9 | 2.0E3 | 2.6E5 | 4.1E5 | 932 | 13 | 345 | 13 | 0.68 |
| Li | pg/ml | 3.5E3 | 1.6E4 | 1.7E4 | 1.0E5 | 6.2E4 | 1.8E5 | 1.0E-9 | 3.6E1 | 1.3E6 | 5.9E5 | 932 | 13 | 345 | 13 | 0.67 |
| Lj | pg/ml | 2.8E3 | 1.1E4 | 2.3E4 | 5.0E4 | 6.4E4 | 1.0E5 | 1.0E-9 | 8.9E1 | 5.2E5 | 3.5E5 | 932 | 13 | 345 | 13 | 0.65 |

| Code | Units | Median | | Average | | Standard Deviation | | Minimum | | Maximum | | Number of Samples | | Number of Patients | | AUC |
|------|-------|--------|------|---------|------|-----|------|--------|------|--------|------|--------|-----|--------|-----|-----|
| | | NonDis | Dis | NonDis | Dis | NonDis | Dis | NonDis | Dis | NonDis | Dis | NonDis | Dis | NonDis | Dis | |
| Rm | ng/ml | 1.9E1 | 3.7E1 | 5.2E1 | 4.0E1 | 8.1E1 | 4.2E1 | 2.2E-1 | 2.3E-1 | 6.5E2 | 1.0E2 | 326 | 7 | 190 | 7 | 0.47 |
| Rh | ng/ml | 1.3E2 | 5.0E2 | 3.5E2 | 6.3E2 | 1.2E3 | 8.7E2 | 3.6E0 | 2.5E1 | 1.7E4 | 2.5E3 | 326 | 7 | 190 | 7 | 0.62 |
| Ri | ng/ml | 1.0E-9 | 1.0E-9 | 4.3E0 | 2.0E0 | 1.5E1 | 2.8E0 | 1.0E-9 | 1.0E-9 | 2.2E2 | 7.1E0 | 327 | 7 | 191 | 7 | 0.48 |
| Rg | ng/ml | 1.0E-9 | 3.0E-3 | 6.7E-2 | 6.6E-3 | 4.1E-1 | 8.4E-3 | 1.0E-9 | 1.0E-9 | 4.6E0 | 2.2E-2 | 326 | 7 | 190 | 7 | 0.66 |
| Rj | ng/ml | 1.0E-9 | 1.0E-9 | 1.3E0 | 1.0E-9 | 5.2E0 | 0.0E0 | 1.0E-9 | 1.0E-9 | 8.0E1 | 1.0E-9 | 327 | 7 | 191 | 7 | 0.36 |
| Rf | ng/ml | 3.9E-1 | 5.3E-1 | 9.7E-1 | 1.6E0 | 1.7E0 | 2.6E0 | 7.8E-3 | 7.0E-2 | 1.5E1 | 7.5E0 | 326 | 7 | 190 | 7 | 0.58 |
| Ql | pg/ml | 4.5E0 | 4.5E0 | 1.4E1 | 1.4E1 | 2.9E1 | 2.0E1 | 1.0E-9 | 1.0E-9 | 2.9E2 | 5.5E1 | 332 | 7 | 193 | 7 | 0.52 |
| Qm | pg/ml | 3.2E0 | 1.0E-9 | 2.0E1 | 1.3E1 | 3.7E1 | 1.8E1 | 1.0E-9 | 1.0E-9 | 3.6E2 | 4.4E1 | 332 | 7 | 193 | 7 | 0.46 |
| Qn | pg/ml | 6.1E-1 | 1.0E-9 | 8.0E0 | 2.6E-1 | 2.6E1 | 3.2E-1 | 1.0E-9 | 1.0E-9 | 2.4E2 | 6.1E-1 | 332 | 7 | 193 | 7 | 0.28 |
| Nv | pg/ml | 4.0E3 | 1.1E4 | 1.1E4 | 2.4E4 | 4.2E4 | 3.7E4 | 1.0E-9 | 1.6E2 | 1.1E6 | 1.3E5 | 939 | 13 | 345 | 13 | 0.72 |
| Nw | pg/ml | 8.9E3 | 1.7E4 | 1.3E4 | 4.2E4 | 1.6E4 | 6.2E4 | 8.6E1 | 3.6E3 | 2.1E5 | 2.1E5 | 939 | 13 | 345 | 13 | 0.72 |
| Nx | pg/ml | 2.2E2 | 2.2E2 | 4.1E2 | 5.5E2 | 6.4E2 | 8.7E2 | 1.0E-9 | 1.0E-9 | 7.4E3 | 2.8E3 | 939 | 13 | 345 | 13 | 0.51 |
| Ny | pg/ml | 6.2E0 | 2.3E1 | 5.2E1 | 7.2E1 | 8.3E2 | 1.7E2 | 1.0E-9 | 1.0E-9 | 2.5E4 | 6.2E2 | 939 | 13 | 345 | 13 | 0.65 |
| Oa | pg/ml | 1.8E2 | 3.5E2 | 4.6E2 | 5.6E2 | 7.3E2 | 8.1E2 | 1.0E-9 | 1.0E-9 | 4.8E3 | 2.4E3 | 332 | 7 | 193 | 7 | 0.55 |
| Oe | pg/ml | 7.1E1 | 1.0E-9 | 2.9E2 | 1.9E2 | 7.4E2 | 4.5E2 | 1.0E-9 | 1.0E-9 | 1.9E4 | 1.6E3 | 930 | 13 | 344 | 13 | 0.40 |
| Of | pg/ml | 1.8E2 | 9.1E1 | 5.9E3 | 6.4E3 | 2.8E4 | 2.0E4 | 1.0E-9 | 1.0E-9 | 6.2E5 | 7.4E4 | 938 | 13 | 345 | 13 | 0.46 |
| Og | pg/ml | 8.2E-2 | 1.0E-9 | 7.1E-1 | 1.5E-1 | 4.7E0 | 3.3E-1 | 1.0E-9 | 1.0E-9 | 8.3E1 | 1.2E0 | 938 | 13 | 345 | 13 | 0.34 |
| Oh | pg/ml | 2.6E0 | 5.3E0 | 1.8E1 | 3.7E1 | 1.4E2 | 7.5E1 | 1.0E-9 | 1.0E-9 | 3.5E3 | 2.2E2 | 938 | 13 | 345 | 13 | 0.58 |
| Oi | pg/ml | 2.6E0 | 1.4E0 | 6.3E0 | 4.3E0 | 9.8E0 | 5.9E0 | 1.0E-9 | 1.0E-9 | 8.6E1 | 1.9E1 | 938 | 13 | 345 | 13 | 0.46 |
| Ok | pg/ml | 3.9E2 | 6.5E2 | 5.4E2 | 6.8E3 | 5.7E2 | 1.9E4 | 1.3E1 | 5.3E1 | 7.0E3 | 7.0E4 | 938 | 13 | 345 | 13 | 0.66 |
| Om | pg/ml | 3.9E2 | 6.9E2 | 8.2E2 | 3.3E3 | 2.1E3 | 5.4E3 | 1.0E-9 | 1.0E-9 | 3.6E4 | 1.7E4 | 938 | 13 | 345 | 13 | 0.59 |
| On | pg/ml | 1.8E2 | 5.3E2 | 2.8E2 | 2.3E3 | 3.9E2 | 4.6E3 | 1.0E-9 | 1.6E1 | 4.5E3 | 1.5E4 | 938 | 13 | 345 | 13 | 0.75 |
| Or | pg/ml | 1.4E1 | 1.8E1 | 3.5E1 | 1.5E2 | 6.6E1 | 2.8E2 | 1.0E-9 | 1.0E-9 | 5.0E2 | 7.6E2 | 334 | 7 | 191 | 7 | 0.57 |
| Ow | pg/ml | 3.3E1 | 1.1E2 | 1.2E2 | 4.2E2 | 3.3E2 | 5.4E2 | 1.0E-9 | 4.7E1 | 3.2E3 | 1.5E3 | 334 | 7 | 191 | 7 | 0.81 |
| Ou | pg/ml | 4.8E2 | 6.4E3 | 9.8E2 | 5.5E3 | 1.6E3 | 3.4E3 | 1.0E-9 | 3.1E2 | 9.8E3 | 9.6E3 | 334 | 7 | 191 | 7 | 0.88 |
| Ul | ng/ml | 1.0E-9 | 1.0E-9 | 1.6E0 | 6.7E-1 | 7.5E0 | 1.8E0 | 1.0E-9 | 1.0E-9 | 1.0E2 | 4.7E0 | 340 | 7 | 195 | 7 | 0.52 |
| Uo | ng/ml | 1.0E-9 | 1.0E-9 | 9.9E-2 | 1.2E-1 | 2.6E-1 | 2.2E-1 | 1.0E-9 | 1.0E-9 | 2.6E0 | 5.0E-1 | 340 | 7 | 195 | 7 | 0.52 |
| Uk | ng/ml | 1.0E-9 | 1.0E-9 | 7.2E-3 | 2.0E-4 | 2.4E-2 | 5.2E-4 | 1.0E-9 | 1.0E-9 | 3.2E-1 | 1.4E-3 | 340 | 7 | 195 | 7 | 0.35 |
| Um | ng/ml | 1.0E-9 | 1.0E-9 | 4.0E-1 | 2.2E-1 | 9.5E-1 | 5.8E-1 | 1.0E-9 | 1.0E-9 | 7.2E0 | 1.5E0 | 340 | 7 | 195 | 7 | 0.41 |
| Uf | ng/ml | 6.0E-2 | 9.6E-2 | 1.4E-1 | 8.0E-1 | 2.7E-1 | 1.9E0 | 1.0E-3 | 3.5E-3 | 2.5E0 | 5.1E0 | 340 | 7 | 195 | 7 | 0.54 |
| Uh | ng/ml | 2.0E0 | 2.2E0 | 3.2E0 | 3.8E0 | 3.4E0 | 3.5E0 | 1.3E-2 | 7.0E-1 | 1.8E1 | 1.1E1 | 340 | 7 | 195 | 7 | 0.59 |
| Un | ng/ml | 1.9E0 | 1.9E0 | 2.2E0 | 2.3E0 | 1.3E0 | 1.3E0 | 1.3E-1 | 7.4E-1 | 8.0E0 | 4.4E0 | 340 | 7 | 195 | 7 | 0.53 |
| Ug | ng/ml | 1.5E1 | 2.3E0 | 2.8E1 | 6.7E0 | 3.0E1 | 7.6E0 | 6.9E-1 | 1.0E0 | 2.1E2 | 2.2E1 | 340 | 7 | 195 | 7 | 0.20 |
| Ur | ng/ml | 1.6E-1 | 1.0E-9 | 7.6E-1 | 2.7E-2 | 5.2E0 | 7.2E-2 | 1.0E-9 | 1.0E-9 | 9.3E1 | 1.9E-1 | 339 | 7 | 194 | 7 | 0.16 |
| Up | ng/ml | 1.0E-9 | 1.0E-9 | 5.0E-3 | 1.0E-9 | 2.2E-2 | 0.0E0 | 1.0E-9 | 1.0E-9 | 3.3E-1 | 1.0E-9 | 339 | 7 | 194 | 7 | 0.34 |
| Us | ng/ml | 3.5E-3 | 1.0E-9 | 1.9E-2 | 2.4E-4 | 4.4E-2 | 4.1E-4 | 1.0E-9 | 1.0E-9 | 4.0E-1 | 8.6E-4 | 339 | 7 | 194 | 7 | 0.24 |
| Uv | ng/ml | 2.7E-3 | 1.0E-3 | 1.1E-2 | 4.4E-3 | 3.8E-2 | 7.2E-3 | 1.0E-9 | 1.0E-9 | 4.3E-1 | 1.9E-2 | 339 | 7 | 194 | 7 | 0.40 |
| Ut | ng/ml | 6.3E-1 | 1.9E0 | 2.6E0 | 3.3E0 | 8.0E0 | 4.6E0 | 1.0E-9 | 1.0E-9 | 7.8E1 | 1.3E1 | 339 | 7 | 194 | 7 | 0.58 |
| Uu | ng/ml | 7.1E0 | 3.6E0 | 8.1E0 | 4.7E0 | 5.8E0 | 3.4E0 | 4.5E-1 | 8.1E-1 | 4.0E1 | 9.1E0 | 339 | 7 | 194 | 7 | 0.32 |
| Vp | ng/ml | 1.0E-9 | 1.0E-9 | 3.5E-1 | 1.0E-9 | 3.1E0 | 0.0E0 | 1.0E-9 | 1.0E-9 | 5.0E1 | 1.0E-9 | 340 | 7 | 195 | 7 | 0.43 |
| Vt | ng/ml | 6.7E0 | 5.4E0 | 9.4E0 | 9.3E0 | 9.8E0 | 8.2E0 | 4.3E-1 | 1.4E0 | 8.6E1 | 2.3E1 | 340 | 7 | 195 | 7 | 0.50 |
| Vu | ng/ml | 1.0E-9 | 1.3E0 | 2.3E0 | 3.1E0 | 6.4E0 | 4.8E0 | 1.0E-9 | 1.0E-9 | 7.1E1 | 1.3E1 | 334 | 7 | 195 | 7 | 0.60 |
| Vo | ng/ml | 2.5E1 | 2.2E1 | 2.4E1 | 1.8E1 | 5.2E0 | 9.4E0 | 2.4E0 | 1.9E0 | 4.8E1 | 2.6E1 | 340 | 7 | 195 | 7 | 0.26 |
| Vs | ng/ml | 1.0E-9 | 1.0E-9 | 6.1E0 | 2.6E0 | 2.2E1 | 4.0E0 | 1.0E-9 | 1.0E-9 | 2.3E2 | 9.4E0 | 325 | 7 | 190 | 7 | 0.49 |
| Vv | ng/ml | 3.0E0 | 3.0E0 | 5.9E0 | 1.4E1 | 9.5E0 | 2.9E1 | 1.0E-9 | 1.0E-9 | 8.4E1 | 8.0E1 | 339 | 7 | 195 | 7 | 0.51 |
| Oy | pg/ml | 4.9E-1 | 6.5E-1 | 5.7E0 | 2.3E1 | 2.8E1 | 7.5E1 | 1.0E-9 | 1.0E-9 | 4.0E2 | 2.7E2 | 937 | 13 | 344 | 13 | 0.57 |
| Oz | pg/ml | 1.2E-2 | 3.0E-2 | 3.2E-1 | 2.4E-1 | 1.3E0 | 3.0E-1 | 1.0E-9 | 1.0E-9 | 2.9E1 | 7.1E-1 | 937 | 13 | 344 | 13 | 0.52 |
| Pa | pg/ml | 3.9E-1 | 6.1E-1 | 1.4E0 | 2.6E1 | 4.9E0 | 8.1E1 | 1.0E-9 | 1.0E-9 | 8.6E1 | 2.9E2 | 937 | 13 | 344 | 13 | 0.63 |
| Pb | pg/ml | 1.0E-9 | 1.3E-1 | 7.4E-1 | 1.4E1 | 1.6E1 | 5.0E1 | 1.0E-9 | 1.0E-9 | 4.9E2 | 1.8E2 | 937 | 13 | 344 | 13 | 0.65 |
| Pc | pg/ml | 5.4E-2 | 4.2E-1 | 3.5E-1 | 1.7E0 | 8.4E-1 | 3.4E0 | 1.0E-9 | 1.0E-9 | 1.6E1 | 1.1E1 | 937 | 13 | 344 | 13 | 0.62 |
| Pd | pg/ml | 1.8E0 | 2.5E0 | 4.8E0 | 8.3E0 | 2.8E1 | 1.5E1 | 1.0E-9 | 1.0E-9 | 8.4E2 | 5.5E1 | 937 | 13 | 344 | 13 | 0.55 |
| Pe | pg/ml | 2.2E1 | 9.4E1 | 1.0E2 | 1.3E3 | 3.4E2 | 3.9E3 | 1.0E-9 | 3.3E0 | 4.7E3 | 1.4E4 | 937 | 13 | 344 | 13 | 0.69 |
| Pf | pg/ml | 1.6E0 | 6.3E0 | 1.0E1 | 2.8E1 | 5.7E1 | 6.2E1 | 1.0E-9 | 1.0E-9 | 1.5E3 | 2.3E2 | 937 | 13 | 344 | 13 | 0.68 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pg | pg/ml | 3.5E0 | 6.4E0 | 4.1E1 | 1.9E2 | 3.3E2 | 5.2E2 | 1.0E-9 | 5.5E-1 | 7.7E3 | 1.9E3 | 937 | 13 | 344 | 13 | 0.67 |
| Ph | ng/ml | 1.8E-1 | 5.8E-1 | 3.5E-1 | 8.3E-1 | 5.5E-1 | 9.3E-1 | 1.0E-9 | 1.0E-9 | 4.4E0 | 2.3E0 | 334 | 7 | 191 | 7 | 0.56 |
| Pi | ng/ml | 2.0E-1 | 3.5E-1 | 2.9E-1 | 3.3E-1 | 4.2E-1 | 2.0E-1 | 1.0E-9 | 1.0E-9 | 4.8E0 | 6.4E-1 | 334 | 7 | 191 | 7 | 0.64 |
| Pj | ng/mL | 5.4E0 | 5.1E0 | 6.2E0 | 6.6E0 | 5.2E0 | 7.7E0 | 3.8E-2 | 6.6E-1 | 5.5E1 | 2.3E1 | 334 | 7 | 191 | 7 | 0.43 |
| Pk | ng/ml | 8.9E-3 | 9.0E-3 | 1.3E-2 | 1.1E-2 | 2.0E-2 | 8.5E-3 | 1.0E-9 | 1.0E-9 | 2.5E-1 | 2.5E-2 | 334 | 7 | 191 | 7 | 0.52 |
| aA | mg/dL | 8.1E-1 | 1.3E0 | 9.5E-1 | 2.0E0 | 4.9E-1 | 1.4E0 | 2.0E-1 | 6.2E-1 | 4.2E0 | 5.4E0 | 2722 | 25 | 517 | 25 | 0.82 |
| aC | mg/mL | 2.8E0 | 2.4E0 | 3.1E0 | 2.6E0 | 1.4E0 | 1.3E0 | 7.7E-1 | 1.1E0 | 8.9E0 | 5.5E0 | 551 | 12 | 213 | 12 | 0.39 |
| aD | ug/mL | 3.1E0 | 4.2E0 | 4.4E0 | 5.7E0 | 3.9E0 | 4.6E0 | 4.3E-1 | 9.9E-1 | 3.5E1 | 1.7E1 | 551 | 12 | 213 | 12 | 0.58 |
| aE | mg/mL | 5.6E-1 | 6.3E-1 | 5.7E-1 | 6.4E-1 | 1.5E-1 | 1.9E-1 | 1.8E-1 | 4.3E-1 | 1.1E0 | 1.2E0 | 551 | 12 | 213 | 12 | 0.61 |
| aF | ng/mL | 2.2E0 | 2.4E0 | 4.0E0 | 5.8E0 | 5.7E0 | 6.7E0 | 4.3E-3 | 4.3E-3 | 5.0E1 | 1.8E1 | 551 | 12 | 213 | 12 | 0.48 |
| aG | mg/mL | 1.4E-1 | 1.2E-1 | 1.6E-1 | 1.6E-1 | 8.7E-2 | 1.4E-1 | 1.7E-2 | 5.9E-2 | 5.4E-1 | 5.2E-1 | 551 | 12 | 213 | 12 | 0.43 |
| aH | ug/mL | 7.5E1 | 6.4E1 | 8.2E1 | 8.1E1 | 4.4E1 | 4.4E1 | 4.6E0 | 3.6E1 | 2.9E2 | 1.8E2 | 551 | 12 | 213 | 12 | 0.48 |
| aI | ug/mL | 1.9E2 | 1.5E2 | 1.9E2 | 1.7E2 | 6.0E1 | 5.1E1 | 2.8E1 | 9.7E1 | 3.7E2 | 2.7E2 | 551 | 12 | 213 | 12 | 0.39 |
| aJ | ug/mL | 2.5E0 | 5.0E0 | 3.1E0 | 6.5E0 | 2.2E0 | 3.8E0 | 7.3E-1 | 2.2E0 | 1.7E1 | 1.5E1 | 551 | 12 | 213 | 12 | 0.82 |
| aK | ng/mL | 1.5E0 | 1.6E0 | 2.4E0 | 2.4E0 | 2.6E0 | 2.2E0 | 2.9E-4 | 1.3E-1 | 1.8E1 | 6.5E0 | 551 | 12 | 213 | 12 | 0.52 |
| aL | mg/mL | 8.0E-1 | 6.9E-1 | 8.1E-1 | 7.3E-1 | 2.6E-1 | 2.5E-1 | 1.9E-1 | 4.0E-1 | 1.7E0 | 1.2E0 | 551 | 12 | 213 | 12 | 0.41 |
| aM | U/mL | 2.2E1 | 4.5E1 | 4.6E1 | 6.6E1 | 9.3E1 | 1.0E2 | 4.2E-2 | 4.2E-2 | 1.6E3 | 3.8E2 | 551 | 12 | 213 | 12 | 0.60 |
| aN | U/mL | 1.4E1 | 2.4E1 | 2.2E1 | 3.1E1 | 3.0E1 | 3.0E1 | 2.5E-3 | 3.9E0 | 3.8E2 | 9.2E1 | 551 | 12 | 213 | 12 | 0.63 |
| aO | pg/mL | 3.3E1 | 1.6E2 | 3.0E2 | 8.7E2 | 7.7E2 | 1.2E3 | 6.0E-2 | 6.0E-2 | 6.6E3 | 3.5E3 | 551 | 12 | 213 | 12 | 0.69 |
| aP | ng/mL | 1.7E0 | 3.3E0 | 2.1E0 | 3.5E0 | 1.8E0 | 1.7E0 | 4.5E-1 | 1.6E0 | 2.8E1 | 6.1E0 | 551 | 12 | 213 | 12 | 0.76 |
| aQ | ng/mL | 3.0E-1 | 3.6E-1 | 4.5E-1 | 3.6E-1 | 4.5E-1 | 3.1E-1 | 2.0E-4 | 5.2E-2 | 4.0E0 | 9.9E-1 | 551 | 12 | 213 | 12 | 0.44 |
| aR | ng/mL | 1.8E0 | 2.9E0 | 2.8E0 | 2.8E0 | 3.3E0 | 1.8E0 | 1.8E-1 | 3.2E-1 | 3.4E1 | 5.0E0 | 551 | 12 | 213 | 12 | 0.56 |
| aS | ng/mL | 2.7E-1 | 3.6E-1 | 6.3E-1 | 6.2E-1 | 1.7E0 | 7.4E-1 | 4.2E-3 | 8.0E-2 | 3.3E1 | 2.6E0 | 551 | 12 | 213 | 12 | 0.54 |
| aU | pg/mL | 7.5E1 | 8.5E1 | 1.2E2 | 1.5E2 | 1.5E2 | 1.5E2 | 7.4E-2 | 7.4E-2 | 1.3E3 | 5.1E2 | 551 | 12 | 213 | 12 | 0.56 |
| aV | ng/mL | 6.2E-1 | 5.9E-1 | 1.0E0 | 1.2E0 | 1.8E0 | 1.5E0 | 7.6E-4 | 1.0E-1 | 3.3E1 | 4.2E0 | 551 | 12 | 213 | 12 | 0.50 |
| aW | pg/mL | 1.8E1 | 2.5E1 | 1.9E1 | 5.8E1 | 1.8E1 | 1.2E2 | 7.2E-2 | 7.2E-2 | 2.4E2 | 4.5E2 | 551 | 12 | 213 | 12 | 0.65 |
| aX | ng/mL | 9.5E0 | 1.5E1 | 1.6E1 | 2.9E1 | 2.6E1 | 3.4E1 | 3.0E-1 | 3.3E0 | 3.1E2 | 1.1E2 | 551 | 12 | 213 | 12 | 0.63 |
| aY | pg/mL | 5.7E1 | 6.6E1 | 7.5E1 | 9.0E1 | 8.2E1 | 7.2E1 | 4.1E-1 | 2.7E1 | 1.2E3 | 2.7E2 | 551 | 12 | 213 | 12 | 0.58 |
| aZ | pg/mL | 2.2E2 | 3.3E2 | 5.4E2 | 6.8E2 | 1.1E3 | 7.5E2 | 1.7E0 | 7.2E1 | 1.2E4 | 2.1E3 | 551 | 12 | 213 | 12 | 0.63 |
| bA | ng/mL | 9.0E0 | 1.1E2 | 3.5E1 | 2.0E2 | 9.2E1 | 2.5E2 | 3.0E-2 | 4.7E0 | 9.7E2 | 8.1E2 | 551 | 12 | 213 | 12 | 0.86 |
| bB | ng/mL | 3.0E2 | 2.7E2 | 3.2E2 | 2.8E2 | 1.7E2 | 1.4E2 | 2.1E0 | 7.8E1 | 1.0E3 | 5.5E2 | 551 | 12 | 213 | 12 | 0.45 |
| bC | ng/mL | 3.4E2 | 4.2E2 | 6.0E2 | 1.2E3 | 7.9E2 | 1.4E3 | 9.8E0 | 1.8E2 | 4.7E3 | 4.0E3 | 551 | 12 | 213 | 12 | 0.64 |
| bE | mg/mL | 5.5E0 | 5.7E0 | 5.8E0 | 6.7E0 | 2.1E0 | 2.8E0 | 9.8E-1 | 3.2E0 | 1.3E1 | 1.0E1 | 551 | 12 | 213 | 12 | 0.57 |
| bF | pg/mL | 2.0E1 | 4.9E1 | 1.5E2 | 6.3E1 | 8.8E2 | 4.4E1 | 5.0E-2 | 8.1E0 | 1.1E4 | 1.5E2 | 551 | 12 | 213 | 12 | 0.72 |
| bG | ng/mL | 1.6E0 | 2.0E0 | 2.7E0 | 5.5E0 | 3.2E0 | 5.7E0 | 2.2E-2 | 2.4E-1 | 2.6E1 | 1.5E1 | 551 | 12 | 213 | 12 | 0.59 |
| bH | pg/mL | 5.7E-1 | 5.7E-1 | 4.7E0 | 6.5E0 | 1.4E1 | 8.1E0 | 5.7E-1 | 5.7E-1 | 2.8E2 | 2.3E1 | 551 | 12 | 213 | 12 | 0.57 |
| bI | ng/mL | 4.0E-3 | 4.0E-3 | 6.0E-2 | 4.7E-2 | 1.5E-1 | 1.1E-1 | 4.0E-3 | 4.0E-3 | 1.3E0 | 3.9E-1 | 551 | 12 | 213 | 12 | 0.50 |
| bJ | mg/mL | 2.3E0 | 2.4E0 | 2.6E0 | 3.2E0 | 2.0E0 | 2.4E0 | 2.5E-4 | 9.6E-1 | 1.3E1 | 8.9E0 | 551 | 12 | 213 | 12 | 0.55 |
| bL | pg/mL | 3.8E0 | 6.5E0 | 8.5E0 | 8.5E0 | 1.1E1 | 7.1E0 | 4.6E-2 | 4.6E-2 | 8.0E1 | 2.0E1 | 551 | 12 | 213 | 12 | 0.58 |
| bM | mg/mL | 1.7E0 | 2.1E0 | 2.1E0 | 2.1E0 | 1.4E0 | 9.3E-1 | 9.2E-3 | 5.5E-1 | 8.9E0 | 3.5E0 | 551 | 12 | 213 | 12 | 0.56 |
| bN | ng/mL | 4.2E1 | 2.6E1 | 1.3E2 | 3.1E1 | 2.7E2 | 2.4E1 | 1.4E-1 | 2.2E0 | 1.9E3 | 7.7E1 | 551 | 12 | 213 | 12 | 0.36 |
| bO | ng/mL | 4.0E-2 | 4.0E-2 | 1.0E1 | 1.8E1 | 2.3E1 | 5.5E1 | 4.0E-2 | 4.0E-2 | 2.0E2 | 1.9E2 | 551 | 12 | 213 | 12 | 0.42 |
| bP | mg/mL | 5.3E-1 | 7.3E-1 | 7.6E-1 | 8.8E-1 | 6.9E-1 | 7.1E-1 | 4.9E-2 | 9.2E-2 | 4.8E0 | 2.9E0 | 551 | 12 | 213 | 12 | 0.60 |
| bQ | pg/mL | 1.6E1 | 4.1E1 | 5.8E1 | 4.8E1 | 5.8E2 | 4.0E1 | 1.5E-1 | 6.7E0 | 1.3E4 | 1.4E2 | 551 | 12 | 213 | 12 | 0.69 |
| bR | ng/mL | 1.2E-2 | 1.2E-2 | 1.3E-1 | 7.5E-2 | 4.2E-1 | 1.5E-1 | 1.2E-2 | 1.2E-2 | 8.7E0 | 3.9E-1 | 551 | 12 | 213 | 12 | 0.41 |
| bS | pg/mL | 9.4E-1 | 9.4E-1 | 7.0E0 | 8.8E0 | 2.6E1 | 2.1E1 | 9.4E-1 | 9.4E-1 | 3.9E2 | 7.2E1 | 551 | 12 | 213 | 12 | 0.52 |
| bU | ng/mL | 1.2E-1 | 1.3E-2 | 1.9E-1 | 9.5E-2 | 3.5E-1 | 1.2E-1 | 1.3E-2 | 1.3E-2 | 6.6E0 | 3.4E-1 | 551 | 12 | 213 | 12 | 0.38 |
| bV | pg/mL | 4.7E2 | 8.6E2 | 5.9E2 | 8.9E2 | 8.9E2 | 4.8E2 | 1.5E2 | 3.5E2 | 1.7E4 | 2.2E3 | 551 | 12 | 213 | 12 | 0.79 |
| bW | pg/mL | 3.3E2 | 4.2E2 | 5.1E2 | 7.4E2 | 5.5E2 | 1.0E3 | 8.4E1 | 1.8E2 | 6.4E3 | 3.9E3 | 551 | 12 | 213 | 12 | 0.60 |
| bX | ng/mL | 2.5E-5 | 3.1E-3 | 2.7E-3 | 2.8E-3 | 3.4E-3 | 2.7E-3 | 2.5E-5 | 2.5E-5 | 2.0E-2 | 7.2E-3 | 551 | 12 | 213 | 12 | 0.52 |
| bZ | pg/mL | 2.5E2 | 6.2E2 | 8.5E2 | 2.0E3 | 3.7E3 | 2.5E3 | 1.5E-1 | 1.6E2 | 5.8E4 | 7.4E3 | 551 | 12 | 213 | 12 | 0.73 |
| cA | pg/mL | 6.0E-1 | 6.0E-1 | 2.7E0 | 2.3E0 | 1.6E1 | 5.9E0 | 6.0E-1 | 6.0E-1 | 3.7E2 | 2.1E1 | 551 | 12 | 213 | 12 | 0.48 |
| cB | ng/mL | 5.5E-2 | 4.1E-2 | 8.7E-2 | 6.8E-2 | 1.0E-1 | 7.2E-2 | 1.7E-3 | 1.7E-3 | 5.7E-1 | 2.6E-1 | 551 | 12 | 213 | 12 | 0.47 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| cC | pg/mL | 4.6E1 | 3.7E1 | 4.7E1 | 3.8E1 | 3.9E1 | 2.4E1 | 1.0E0 | 1.0E0 | 4.5E2 | 6.7E1 | 551 | 12 | 213 | 12 | 0.44 |
| cD | pg/mL | 5.2E0 | 6.7E0 | 1.5E1 | 8.6E0 | 5.2E1 | 1.0E1 | 3.3E-1 | 3.3E-1 | 7.2E2 | 3.8E1 | 551 | 12 | 213 | 12 | 0.51 |
| cE | pg/mL | 3.9E1 | 6.4E1 | 1.5E2 | 9.1E1 | 4.3E2 | 8.7E1 | 1.2E-1 | 2.0E0 | 3.8E3 | 2.8E2 | 551 | 12 | 213 | 12 | 0.58 |
| cF | pg/mL | 1.3E1 | 5.3E-1 | 2.0E1 | 7.5E0 | 3.0E1 | 2.0E1 | 5.3E-1 | 5.3E-1 | 2.7E2 | 7.2E1 | 551 | 12 | 213 | 12 | 0.30 |
| cG | pg/mL | 4.6E1 | 8.4E1 | 1.1E2 | 1.1E2 | 4.7E2 | 9.6E1 | 6.4E0 | 1.5E1 | 1.0E4 | 3.8E2 | 551 | 12 | 213 | 12 | 0.66 |
| cH | uIU/mL | 2.8E0 | 2.3E0 | 6.0E0 | 7.7E0 | 1.1E1 | 1.0E1 | 8.6E-3 | 8.6E-3 | 1.6E2 | 3.4E1 | 551 | 12 | 213 | 12 | 0.51 |
| cI | ng/mL | 5.7E0 | 9.2E0 | 1.2E1 | 1.9E1 | 1.7E1 | 3.2E1 | 1.0E-3 | 1.1E0 | 1.2E2 | 1.2E2 | 551 | 12 | 213 | 12 | 0.59 |
| cJ | ug/mL | 6.2E1 | 6.1E1 | 1.1E2 | 7.4E1 | 1.4E2 | 4.7E1 | 4.0E0 | 1.5E1 | 9.6E2 | 1.7E2 | 551 | 12 | 213 | 12 | 0.50 |
| cK | ng/mL | 3.8E-3 | 3.8E-3 | 4.7E-2 | 5.3E-2 | 1.7E-1 | 9.1E-2 | 3.8E-3 | 3.8E-3 | 1.7E0 | 2.4E-1 | 551 | 12 | 213 | 12 | 0.55 |
| cL | pg/mL | 2.0E2 | 2.2E2 | 3.9E2 | 3.0E2 | 1.3E3 | 3.4E2 | 1.6E1 | 6.7E1 | 2.4E4 | 1.3E3 | 551 | 12 | 213 | 12 | 0.53 |
| cM | pg/mL | 2.7E2 | 2.5E2 | 2.9E2 | 2.7E2 | 1.9E2 | 7.7E1 | 8.7E0 | 1.5E2 | 1.6E3 | 4.3E2 | 551 | 12 | 213 | 12 | 0.48 |
| cN | pg/mL | 1.2E2 | 1.5E2 | 1.3E2 | 1.5E2 | 6.2E1 | 4.4E1 | 3.8E1 | 9.6E1 | 1.1E3 | 2.2E2 | 551 | 12 | 213 | 12 | 0.68 |
| cO | pg/mL | 2.2E2 | 3.1E2 | 3.0E2 | 4.4E2 | 8.3E2 | 3.9E2 | 5.4E1 | 9.6E1 | 1.9E4 | 1.5E3 | 551 | 12 | 213 | 12 | 0.65 |
| cP | ng/mL | 2.5E3 | 3.7E3 | 2.6E3 | 3.6E3 | 9.0E2 | 1.4E3 | 6.2E2 | 1.4E3 | 5.7E3 | 5.9E3 | 551 | 12 | 213 | 12 | 0.72 |
| cQ | ng/mL | 5.1E-2 | 1.3E-1 | 1.4E-1 | 1.8E-1 | 2.8E-1 | 1.7E-1 | 2.0E-3 | 2.0E-3 | 2.2E0 | 4.9E-1 | 551 | 12 | 213 | 12 | 0.65 |
| cR | ng/mL | 3.0E2 | 4.9E2 | 5.2E2 | 6.6E2 | 8.3E2 | 5.7E2 | 2.0E1 | 1.6E2 | 8.9E3 | 2.2E3 | 551 | 12 | 213 | 12 | 0.67 |
| cS | ng/mL | 2.6E2 | 5.7E2 | 4.4E2 | 8.8E2 | 1.0E3 | 8.2E2 | 4.1E1 | 1.4E2 | 2.2E4 | 2.6E3 | 551 | 12 | 213 | 12 | 0.75 |
| cT | ng/mL | 3.3E1 | 1.4E2 | 8.8E1 | 3.5E2 | 2.0E2 | 5.2E2 | 3.6E0 | 1.9E1 | 2.1E3 | 1.9E3 | 551 | 12 | 213 | 12 | 0.83 |
| cU | ng/mL | 5.4E1 | 1.2E2 | 7.7E1 | 1.4E2 | 9.6E1 | 9.2E1 | 5.4E0 | 3.0E1 | 1.6E3 | 3.3E2 | 551 | 12 | 213 | 12 | 0.75 |
| cV | ng/mL | 1.8E-1 | 1.6E-1 | 4.0E-1 | 2.2E-1 | 2.1E0 | 1.5E-1 | 3.4E-4 | 6.4E-2 | 4.7E1 | 4.5E-1 | 551 | 12 | 213 | 12 | 0.50 |
| cW | mIU/mL | 5.2E-2 | 9.0E-2 | 1.3E-1 | 9.8E-2 | 6.5E-1 | 5.7E-2 | 3.7E-4 | 2.7E-2 | 9.7E0 | 2.0E-1 | 551 | 12 | 213 | 12 | 0.68 |
| cX | ng/mL | 1.1E-1 | 2.9E-2 | 1.3E0 | 1.5E-1 | 4.3E0 | 3.1E-1 | 2.3E-4 | 2.3E-4 | 2.8E1 | 1.1E0 | 551 | 12 | 213 | 12 | 0.35 |
| cY | ng/mL | 8.6E0 | 9.0E0 | 1.2E1 | 1.2E1 | 1.3E1 | 1.1E1 | 1.5E-1 | 6.0E-1 | 8.3E1 | 3.6E1 | 551 | 12 | 213 | 12 | 0.50 |
| cZ | ug/mL | 1.4E1 | 1.6E1 | 1.6E1 | 1.6E1 | 7.1E0 | 6.4E0 | 2.3E0 | 8.0E0 | 5.7E1 | 3.0E1 | 551 | 12 | 213 | 12 | 0.54 |
| dA | pg/mL | 3.3E2 | 5.1E2 | 3.7E2 | 5.2E2 | 2.8E2 | 2.8E2 | 9.0E1 | 1.7E2 | 5.8E3 | 8.8E2 | 551 | 12 | 213 | 12 | 0.65 |
| dB | ug/mL | 1.7E1 | 2.1E1 | 1.7E1 | 2.3E1 | 1.5E1 | 7.8E0 | 9.4E-1 | 1.4E1 | 2.5E2 | 4.1E1 | 551 | 12 | 213 | 12 | 0.69 |
| dC | nmol/L | 3.5E1 | 3.2E1 | 3.8E1 | 3.7E1 | 1.8E1 | 1.8E1 | 7.6E0 | 2.1E1 | 1.4E2 | 8.2E1 | 551 | 12 | 213 | 12 | 0.44 |
| dD | ug/mL | 3.6E1 | 2.9E1 | 3.7E1 | 3.3E1 | 1.1E1 | 1.1E1 | 1.3E1 | 1.5E1 | 7.6E1 | 5.6E1 | 551 | 12 | 213 | 12 | 0.38 |
| dE | ng/mL | 4.7E-1 | 7.3E-1 | 6.0E-1 | 9.5E-1 | 6.9E-1 | 8.5E-1 | 8.4E-3 | 3.0E-1 | 7.2E0 | 3.3E0 | 551 | 12 | 213 | 12 | 0.66 |
| dF | ng/mL | 2.3E2 | 3.2E2 | 2.8E2 | 4.2E2 | 1.9E2 | 2.6E2 | 5.6E1 | 1.7E2 | 1.3E3 | 9.7E2 | 551 | 12 | 213 | 12 | 0.71 |
| dG | ng/mL | 1.2E1 | 1.6E1 | 1.5E1 | 2.0E1 | 1.3E1 | 8.9E0 | 2.2E0 | 6.7E0 | 1.8E2 | 3.5E1 | 551 | 12 | 213 | 12 | 0.70 |
| dH | pg/mL | 7.9E0 | 1.0E1 | 1.3E1 | 1.1E1 | 3.6E1 | 6.4E0 | 4.0E-2 | 8.3E-1 | 6.7E2 | 2.2E1 | 551 | 12 | 213 | 12 | 0.58 |
| dI | pg/mL | 4.6E-1 | 4.6E-1 | 2.2E0 | 2.1E0 | 1.5E1 | 2.7E0 | 4.6E-1 | 4.6E-1 | 3.3E2 | 8.4E0 | 551 | 12 | 213 | 12 | 0.57 |
| dJ | ng/mL | 1.9E0 | 2.6E0 | 2.2E0 | 2.5E0 | 1.2E0 | 1.0E0 | 3.2E-2 | 8.4E-1 | 6.9E0 | 4.0E0 | 551 | 12 | 213 | 12 | 0.60 |
| dK | uIU/mL | 1.9E0 | 1.0E0 | 3.0E0 | 1.8E0 | 6.0E0 | 1.9E0 | 2.8E-4 | 3.8E-2 | 7.9E1 | 6.1E0 | 551 | 12 | 213 | 12 | 0.41 |
| dL | ng/mL | 8.8E2 | 1.2E3 | 1.0E3 | 1.1E3 | 5.2E2 | 2.8E2 | 2.6E2 | 6.3E2 | 3.8E3 | 1.4E3 | 551 | 12 | 213 | 12 | 0.59 |
| dM | pg/mL | 9.7E2 | 1.9E3 | 1.2E3 | 2.9E3 | 1.3E3 | 2.4E3 | 3.4E2 | 7.1E2 | 1.6E4 | 8.3E3 | 551 | 12 | 213 | 12 | 0.78 |
| dN | ug/mL | 9.3E1 | 1.4E2 | 9.9E1 | 1.4E2 | 3.7E1 | 3.8E1 | 1.6E1 | 6.9E1 | 2.8E2 | 2.0E2 | 551 | 12 | 213 | 12 | 0.77 |
| dR | pg/ml | 1.6E3 | 8.3E2 | 2.3E3 | 1.4E3 | 2.3E3 | 1.5E3 | 1.4E2 | 1.3E2 | 1.5E4 | 5.1E3 | 378 | 9 | 202 | 9 | 0.36 |
| eF | ng/ml | 4.1E0 | 7.2E0 | 5.0E0 | 6.9E0 | 4.3E0 | 2.6E0 | 1.2E0 | 2.8E0 | 4.6E1 | 1.1E1 | 393 | 9 | 203 | 9 | 0.75 |
| eC | pg/ml | 3.0E2 | 2.6E2 | 3.7E2 | 2.6E2 | 2.6E2 | 1.5E2 | 9.9E0 | 1.1E2 | 1.6E3 | 5.4E2 | 304 | 7 | 190 | 7 | 0.36 |
| fP | ng/ml | 2.6E2 | 2.6E2 | 2.9E2 | 3.1E2 | 1.9E2 | 2.0E2 | 1.8E0 | 1.2E2 | 1.6E3 | 7.5E2 | 358 | 9 | 193 | 9 | 0.52 |
| fR | ng/ml | 1.4E5 | 2.9E5 | 1.9E5 | 3.2E5 | 1.5E5 | 1.9E5 | 2.9E4 | 1.2E5 | 8.3E5 | 7.2E5 | 363 | 11 | 111 | 11 | 0.75 |
| gL | pg/ml | 6.4E4 | 1.1E5 | 7.1E4 | 1.1E5 | 3.2E4 | 3.2E4 | 1.1E4 | 6.5E4 | 3.2E5 | 1.6E5 | 378 | 9 | 202 | 9 | 0.84 |
| gP | U/ml | 2.7E2 | 2.6E2 | 2.8E2 | 2.7E2 | 1.1E2 | 5.1E1 | 1.2E1 | 2.1E2 | 1.1E3 | 3.7E2 | 389 | 9 | 203 | 9 | 0.51 |
| gW | ng/ml | 6.1E2 | 5.1E2 | 1.3E3 | 7.2E2 | 1.6E3 | 5.4E2 | 3.1E-1 | 2.4E2 | 9.5E3 | 1.7E3 | 329 | 7 | 192 | 7 | 0.49 |
| tF | pg/mL | 1.4E3 | 5.8E3 | 1.3E4 | 1.9E4 | 4.0E4 | 3.4E4 | 1.2E1 | 1.8E1 | 3.2E5 | 9.4E4 | 305 | 7 | 190 | 7 | 0.63 |
| iA | pg/ml | 1.4E2 | 1.6E2 | 2.7E2 | 4.7E2 | 5.5E2 | 7.6E2 | 5.8E0 | 5.6E1 | 7.1E3 | 2.2E3 | 306 | 7 | 190 | 7 | 0.61 |
| iH | ng/ml | 1.6E5 | 2.0E5 | 1.6E5 | 1.9E5 | 4.9E4 | 5.3E4 | 2.9E3 | 8.1E4 | 2.7E5 | 2.5E5 | 306 | 7 | 190 | 7 | 0.70 |
| iJ | ng/ml | 5.0E4 | 5.0E4 | 5.4E4 | 5.6E4 | 2.8E4 | 2.8E4 | 1.8E3 | 1.5E4 | 2.5E5 | 9.7E4 | 306 | 7 | 190 | 7 | 0.55 |
| hB | ng/ml | 4.3E-1 | 9.4E-1 | 5.5E-1 | 8.4E-1 | 4.5E-1 | 4.7E-1 | 1.0E-1 | 1.5E-1 | 3.4E0 | 1.6E0 | 306 | 7 | 190 | 7 | 0.72 |
| hC | pg/ml | 3.7E3 | 1.0E4 | 6.6E3 | 8.0E3 | 9.4E3 | 5.9E3 | 1.0E-9 | 1.7E3 | 1.1E5 | 1.7E4 | 306 | 7 | 190 | 7 | 0.64 |
| hF | ng/ml | 1.0E-9 | 1.0E-9 | 1.6E1 | 1.0E-9 | 2.3E2 | 0.0E0 | 1.0E-9 | 1.0E-9 | 4.0E3 | 1.0E-9 | 306 | 7 | 190 | 7 | 0.49 |

| Code | Units | Median | | Average | | Standard Deviation | | Minimum | | Maximum | | Number of Samples | | Number of Patients | | AUC |
|------|-------|--------|-----|---------|-----|-----|-----|--------|--------|---------|--------|--------|-----|----------|-----|------|
| | | NonDis | Dis | NonDis | Dis | NonDis | Dis | NonDis | Dis | NonDis | Dis | NonDis | Dis | NonDis | Dis | |
| hG | pg/ml | 6.8E3 | 8.7E3 | 7.4E3 | 9.7E3 | 3.2E3 | 4.5E3 | 2.8E1 | 5.3E3 | 2.0E4 | 1.7E4 | 306 | 7 | 190 | 7 | 0.66 |
| iO | ng/ml | 3.7E5 | 3.4E5 | 3.9E5 | 3.5E5 | 1.7E5 | 1.3E5 | 1.1E4 | 1.0E5 | 1.1E6 | 4.6E5 | 306 | 7 | 190 | 7 | 0.47 |
| iP | ng/ml | 4.5E4 | 7.0E4 | 5.4E4 | 6.9E4 | 4.8E4 | 3.1E4 | 1.0E-9 | 3.2E4 | 5.5E5 | 1.2E5 | 306 | 7 | 190 | 7 | 0.68 |
| iZ | ng/ml | 1.7E3 | 2.5E3 | 1.8E3 | 3.0E3 | 7.5E2 | 1.7E3 | 4.7E2 | 1.5E3 | 5.7E3 | 6.5E3 | 305 | 8 | 188 | 8 | 0.73 |
| kQ | pg/ml | 4.1E3 | 6.1E3 | 5.0E3 | 6.5E3 | 3.4E3 | 2.6E3 | 5.6E2 | 2.7E3 | 2.5E4 | 1.0E4 | 306 | 7 | 190 | 7 | 0.71 |
| kR | pg/ml | 2.1E1 | 2.1E1 | 3.0E1 | 3.1E1 | 6.1E1 | 2.6E1 | 1.0E-9 | 2.9E0 | 1.0E3 | 6.9E1 | 306 | 7 | 190 | 7 | 0.53 |
| kS | pg/ml | 8.1E2 | 6.9E2 | 9.6E2 | 1.1E3 | 9.6E2 | 8.3E2 | 7.9E1 | 5.1E2 | 1.4E4 | 2.8E3 | 306 | 7 | 190 | 7 | 0.54 |
| nW | pg/ml | 1.1E5 | 1.0E5 | 1.1E5 | 1.2E5 | 2.7E4 | 3.8E4 | 3.6E4 | 8.4E4 | 2.1E5 | 1.9E5 | 306 | 7 | 190 | 7 | 0.52 |
| nY | pg/ml | 2.0E3 | 3.6E3 | 2.3E3 | 3.6E3 | 1.5E3 | 1.5E3 | 5.1E2 | 1.3E3 | 1.3E4 | 5.7E3 | 306 | 7 | 190 | 7 | 0.75 |
| oE | pg/ml | 1.5E2 | 3.9E2 | 3.9E2 | 4.0E2 | 5.6E2 | 3.3E2 | 1.0E-9 | 2.7E1 | 4.7E3 | 1.1E3 | 306 | 7 | 190 | 7 | 0.61 |
| oF | pg/ml | 8.8E3 | 1.7E4 | 2.2E4 | 6.0E4 | 3.5E4 | 8.3E4 | 6.4E1 | 7.8E2 | 2.5E5 | 1.8E5 | 306 | 7 | 190 | 7 | 0.62 |
| oH | pg/ml | 4.3E1 | 5.6E1 | 9.3E1 | 8.3E1 | 1.4E2 | 1.0E2 | 3.5E0 | 1.3E1 | 9.9E2 | 3.1E2 | 306 | 7 | 190 | 7 | 0.51 |
| oK | pg/ml | 8.5E2 | 1.5E3 | 1.9E3 | 9.1E3 | 2.8E3 | 1.9E4 | 5.2E1 | 5.5E2 | 2.5E4 | 5.3E4 | 306 | 7 | 190 | 7 | 0.65 |
| oN | pg/ml | 5.1E2 | 5.8E2 | 7.2E2 | 1.2E3 | 1.2E3 | 1.7E3 | 1.1E2 | 2.8E2 | 1.8E4 | 5.0E3 | 306 | 7 | 190 | 7 | 0.57 |
| pF | pg/ml | 4.9E-1 | 9.3E-1 | 9.9E-1 | 2.1E0 | 5.0E0 | 3.5E0 | 1.0E-9 | 2.0E-1 | 8.7E1 | 1.0E1 | 306 | 7 | 190 | 7 | 0.66 |

Figure 16.

Descriptive statistics for the assays used to form the panels presented in this figure.

| Code | Units | Median | | Average | | Standard Deviation | | Minimum | | Maximum | | Number of Samples | | Number of Patients | | AUC |
|------|-------|--------|-----|---------|-----|-------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Po | pg/ml | 9.0E-1 | 2.8E1 | 8.7E0 | 5.3E1 | 2.3E1 | 7.7E1 | 8.0E-3 | 2.6E-2 | 2.7E2 | 2.2E2 | 969 | 7 | 353 | 7 | 0.73 |
| Et | ng/ml | 1.4E3 | 2.8E3 | 1.7E3 | 2.4E3 | 1.2E3 | 1.6E3 | 7.5E1 | 5.9E2 | 5.0E3 | 4.4E3 | 968 | 7 | 353 | 7 | 0.64 |
| Fp | ng/ml | 1.4E1 | 2.5E1 | 2.6E1 | 3.2E1 | 2.9E1 | 2.6E1 | 6.0E-3 | 2.3E0 | 1.4E2 | 6.7E1 | 1004 | 7 | 354 | 7 | 0.59 |
| Fr | ng/ml | 4.1E4 | 6.5E5 | 1.3E5 | 5.1E5 | 1.9E5 | 3.3E5 | 1.9E2 | 7.0E3 | 9.0E5 | 8.5E5 | 1121 | 8 | 359 | 8 | 0.81 |
| Nm | pg/ml | 1.4E4 | 5.7E3 | 3.4E4 | 3.0E4 | 8.2E4 | 3.5E4 | 1.0E-9 | 1.0E-9 | 1.6E6 | 7.9E4 | 972 | 7 | 355 | 7 | 0.49 |
| Nn | pg/ml | 1.7E2 | 2.0E3 | 1.9E3 | 1.8E4 | 8.0E3 | 2.7E4 | 1.0E-9 | 1.0E-9 | 1.0E5 | 6.9E4 | 972 | 7 | 355 | 7 | 0.70 |
| No | pg/ml | 1.6E1 | 6.1E1 | 4.0E1 | 2.8E2 | 1.2E2 | 5.3E2 | 1.0E-9 | 1.6E0 | 2.5E3 | 1.4E3 | 972 | 7 | 355 | 7 | 0.65 |
| Nq | pg/ml | 2.0E0 | 1.6E1 | 1.9E1 | 3.3E1 | 7.4E1 | 4.4E1 | 1.0E-9 | 1.0E-9 | 1.2E3 | 1.1E2 | 972 | 7 | 355 | 7 | 0.70 |
| Nr | pg/ml | 1.1E0 | 1.2E0 | 2.9E1 | 1.2E3 | 1.7E2 | 3.2E3 | 1.0E-9 | 1.0E-9 | 4.1E3 | 8.5E3 | 972 | 7 | 355 | 7 | 0.55 |
| Ns | pg/ml | 1.0E-9 | 1.0E-9 | 8.7E0 | 5.1E-1 | 5.1E1 | 1.4E0 | 1.0E-9 | 1.0E-9 | 1.1E3 | 3.6E0 | 972 | 7 | 355 | 7 | 0.51 |
| Nt | pg/ml | 1.0E2 | 1.2E2 | 1.4E2 | 1.9E2 | 1.2E2 | 2.2E2 | 1.0E-9 | 6.6E1 | 1.7E3 | 6.8E2 | 972 | 7 | 355 | 7 | 0.57 |
| Nu | pg/ml | 2.2E1 | 6.5E1 | 5.5E1 | 1.3E2 | 8.9E1 | 2.0E2 | 1.0E-9 | 1.0E-9 | 8.9E2 | 5.8E2 | 972 | 7 | 355 | 7 | 0.68 |
| Lu | pg/ml | 1.0E4 | 3.0E3 | 1.7E4 | 6.2E3 | 6.0E4 | 7.1E3 | 3.5E2 | 1.0E3 | 1.3E6 | 2.1E4 | 975 | 7 | 355 | 7 | 0.26 |
| Lv | pg/ml | 1.0E-9 | 6.3E1 | 1.2E1 | 6.8E1 | 2.4E1 | 3.0E1 | 1.0E-9 | 3.3E1 | 2.6E2 | 1.0E2 | 975 | 7 | 355 | 7 | 0.95 |
| Lw | pg/ml | 1.0E-9 | 1.0E-9 | 4.6E-1 | 2.5E0 | 4.2E0 | 6.3E0 | 1.0E-9 | 1.0E-9 | 8.0E1 | 1.7E1 | 975 | 7 | 355 | 7 | 0.63 |
| Lx | pg/ml | 1.0E-9 | 4.9E2 | 1.9E2 | 2.1E3 | 8.4E2 | 3.6E3 | 1.0E-9 | 1.0E-9 | 2.2E4 | 1.0E4 | 975 | 7 | 355 | 7 | 0.82 |
| Ly | pg/ml | 1.0E-9 | 4.2E0 | 1.0E1 | 7.1E0 | 2.0E1 | 8.8E0 | 1.0E-9 | 1.0E-9 | 1.6E2 | 2.4E1 | 975 | 7 | 355 | 7 | 0.57 |
| Lz | pg/ml | 1.0E-9 | 1.0E-9 | 3.9E0 | 3.1E1 | 2.9E1 | 7.8E1 | 1.0E-9 | 1.0E-9 | 6.0E2 | 2.1E2 | 975 | 7 | 355 | 7 | 0.61 |
| Ma | pg/ml | 3.0E2 | 3.6E3 | 1.4E3 | 3.4E3 | 3.6E3 | 3.3E3 | 1.0E-9 | 2.4E1 | 6.5E4 | 9.5E3 | 975 | 7 | 355 | 7 | 0.71 |
| Mb | pg/ml | 2.5E1 | 2.2E1 | 3.1E1 | 3.0E1 | 1.5E1 | 1.2E1 | 4.1E0 | 2.1E1 | 2.1E2 | 5.0E1 | 975 | 7 | 355 | 7 | 0.49 |
| Mc | pg/ml | 1.0E-9 | 1.0E-9 | 3.9E-2 | 1.0E-9 | 5.5E-1 | 0.0E0 | 1.0E-9 | 1.0E-9 | 1.3E1 | 1.0E-9 | 975 | 7 | 355 | 7 | 0.49 |
| Md | pg/ml | 1.0E-9 | 1.0E-9 | 4.1E-1 | 1.6E0 | 3.5E0 | 4.1E0 | 1.0E-9 | 1.0E-9 | 6.8E1 | 1.1E1 | 975 | 7 | 355 | 7 | 0.54 |
| Me | pg/ml | 3.3E1 | 2.6E1 | 3.2E1 | 2.9E1 | 1.9E1 | 2.5E1 | 1.0E-9 | 3.2E0 | 3.2E2 | 7.9E1 | 975 | 7 | 355 | 7 | 0.37 |
| Mf | pg/ml | 1.0E-9 | 1.0E-9 | 5.1E-1 | 1.0E-9 | 2.8E0 | 0.0E0 | 1.0E-9 | 1.0E-9 | 5.6E1 | 1.0E-9 | 975 | 7 | 355 | 7 | 0.43 |
| Mg | pg/ml | 1.6E0 | 1.8E0 | 7.5E0 | 3.0E0 | 1.3E1 | 3.6E0 | 1.0E-9 | 1.0E-9 | 9.4E1 | 9.0E0 | 975 | 7 | 355 | 7 | 0.44 |
| Mh | pg/ml | 1.0E-9 | 1.0E-9 | 1.3E0 | 5.4E0 | 9.3E0 | 1.4E1 | 1.0E-9 | 1.0E-9 | 1.6E2 | 3.8E1 | 975 | 7 | 355 | 7 | 0.51 |
| Mi | pg/ml | 1.0E-9 | 2.7E1 | 9.8E-1 | 4.1E1 | 1.2E1 | 5.9E1 | 1.0E-9 | 1.0E-9 | 3.2E2 | 1.6E2 | 975 | 7 | 355 | 7 | 0.78 |
| Mj | pg/ml | 1.0E-9 | 7.8E0 | 4.5E0 | 8.4E1 | 2.4E1 | 2.0E2 | 1.0E-9 | 1.0E-9 | 4.6E2 | 5.4E2 | 975 | 7 | 355 | 7 | 0.71 |
| Mk | pg/ml | 9.1E-1 | 5.3E0 | 1.4E1 | 8.0E2 | 8.3E1 | 2.1E3 | 1.0E-9 | 1.0E-9 | 1.7E3 | 5.6E3 | 975 | 7 | 355 | 7 | 0.58 |
| Ml | pg/ml | 1.0E-9 | 1.0E-9 | 5.5E0 | 3.9E-1 | 7.2E1 | 1.0E0 | 1.0E-9 | 1.0E-9 | 2.1E3 | 2.8E0 | 975 | 7 | 355 | 7 | 0.48 |
| Mm | pg/ml | 6.1E2 | 4.8E2 | 1.1E3 | 1.7E3 | 1.5E3 | 2.5E3 | 1.0E-9 | 1.0E-9 | 1.2E4 | 6.9E3 | 975 | 7 | 355 | 7 | 0.50 |
| Mn | pg/ml | 5.7E0 | 1.2E1 | 1.0E1 | 1.9E1 | 2.2E1 | 2.1E1 | 1.0E-9 | 1.1E0 | 3.5E2 | 6.6E1 | 975 | 7 | 355 | 7 | 0.72 |
| Mp | pg/ml | 1.0E-9 | 2.7E1 | 1.0E1 | 5.5E1 | 3.4E1 | 6.9E1 | 1.0E-9 | 1.0E-9 | 6.3E2 | 1.8E2 | 974 | 7 | 355 | 7 | 0.73 |
| Mq | pg/ml | 1.0E-9 | 1.0E-9 | 3.2E0 | 1.0E-9 | 1.8E1 | 0.0E0 | 1.0E-9 | 1.0E-9 | 2.4E2 | 1.0E-9 | 974 | 7 | 355 | 7 | 0.46 |
| Mr | pg/ml | 1.0E-9 | 1.0E-9 | 2.8E1 | 1.7E3 | 1.6E2 | 4.5E3 | 1.0E-9 | 1.0E-9 | 2.2E3 | 1.2E4 | 974 | 7 | 355 | 7 | 0.58 |
| Ms | pg/ml | 3.9E2 | 8.0E1 | 5.4E2 | 2.3E2 | 6.5E2 | 3.3E2 | 1.0E-9 | 1.0E-9 | 5.9E3 | 8.6E2 | 974 | 7 | 355 | 7 | 0.31 |
| Mt | pg/ml | 3.1E-1 | 9.0E0 | 1.1E1 | 2.5E1 | 1.1E2 | 3.7E1 | 1.0E-9 | 1.0E-9 | 3.2E3 | 1.0E2 | 974 | 7 | 355 | 7 | 0.79 |
| Mu | pg/ml | 1.0E-9 | 7.1E0 | 1.2E0 | 8.6E0 | 1.0E1 | 1.0E1 | 1.0E-9 | 1.0E-9 | 2.3E2 | 2.7E1 | 974 | 7 | 355 | 7 | 0.74 |
| Mv | pg/ml | 1.0E-9 | 5.9E1 | 7.0E1 | 1.5E2 | 3.1E2 | 2.3E2 | 1.0E-9 | 1.0E-9 | 4.5E3 | 6.2E2 | 974 | 7 | 355 | 7 | 0.75 |
| Mw | pg/ml | 4.0E1 | 6.6E2 | 5.0E2 | 1.9E3 | 3.1E3 | 3.1E3 | 1.0E-9 | 1.0E-9 | 6.2E4 | 8.5E3 | 974 | 7 | 355 | 7 | 0.75 |
| Mx | pg/ml | 1.0E-9 | 1.0E-9 | 2.8E-1 | 4.7E-1 | 1.5E0 | 1.1E0 | 1.0E-9 | 1.0E-9 | 3.2E1 | 3.0E0 | 974 | 7 | 355 | 7 | 0.63 |
| My | pg/ml | 1.0E-9 | 8.8E1 | 4.4E2 | 5.7E2 | 2.9E3 | 1.2E3 | 1.0E-9 | 1.0E-9 | 3.9E4 | 3.4E3 | 974 | 7 | 355 | 7 | 0.68 |
| Mz | pg/ml | 1.2E1 | 6.3E1 | 3.1E1 | 1.1E2 | 9.7E1 | 1.3E2 | 1.0E-9 | 8.0E0 | 1.9E3 | 3.6E2 | 974 | 7 | 355 | 7 | 0.82 |
| Na | pg/ml | 1.0E-9 | 1.0E-9 | 7.1E-1 | 1.5E0 | 2.9E0 | 4.0E0 | 1.0E-9 | 1.0E-9 | 4.2E1 | 1.1E1 | 974 | 7 | 355 | 7 | 0.48 |
| Nb | pg/ml | 2.1E0 | 4.6E0 | 4.0E0 | 3.7E1 | 1.2E1 | 8.9E1 | 1.0E-9 | 9.2E-2 | 2.3E2 | 2.4E2 | 974 | 7 | 355 | 7 | 0.63 |
| Nc | pg/ml | 3.3E2 | 1.5E2 | 5.5E2 | 1.7E2 | 7.2E2 | 2.3E2 | 1.0E-9 | 1.0E-9 | 9.2E3 | 6.7E2 | 974 | 7 | 355 | 7 | 0.35 |
| Nd | pg/ml | 2.9E1 | 4.1E1 | 2.9E1 | 6.9E1 | 8.0E1 | 6.2E1 | 1.0E-9 | 7.2E-1 | 2.1E3 | 1.5E2 | 974 | 7 | 355 | 7 | 0.69 |
| Ne | pg/ml | 4.3E2 | 2.7E2 | 5.6E2 | 7.0E2 | 5.7E2 | 1.3E3 | 1.0E-9 | 1.8E1 | 7.0E3 | 3.6E3 | 974 | 7 | 355 | 7 | 0.40 |
| Nf | pg/ml | 1.0E-9 | 1.0E-9 | 2.8E0 | 1.2E1 | 1.1E1 | 3.1E1 | 1.0E-9 | 1.0E-9 | 1.6E2 | 8.2E1 | 974 | 7 | 355 | 7 | 0.47 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ng | pg/ml | 1.8E1 | 1.0E-9 | 1.2E2 | 3.8E1 | 2.5E2 | 8.6E1 | 1.0E-9 | 1.0E-9 | 2.3E3 | 2.3E2 | 974 | 7 | 355 | 7 | 0.34 |
| Nh | pg/ml | 6.7E1 | 3.5E1 | 8.9E1 | 1.1E2 | 8.1E1 | 1.8E2 | 1.0E-9 | 2.2E0 | 5.6E2 | 5.1E2 | 974 | 7 | 355 | 7 | 0.40 |
| Ni | pg/ml | 1.0E-9 | 2.3E1 | 7.4E1 | 7.2E1 | 1.2E2 | 1.2E2 | 1.0E-9 | 1.0E-9 | 1.1E3 | 3.4E2 | 974 | 7 | 355 | 7 | 0.53 |
| Nj | pg/ml | 7.4E0 | 5.8E0 | 1.1E1 | 5.0E0 | 1.2E1 | 3.8E0 | 1.0E-9 | 1.0E-9 | 1.1E2 | 9.5E0 | 974 | 7 | 355 | 7 | 0.36 |
| Nk | pg/ml | 1.7E1 | 2.3E1 | 3.2E1 | 1.6E1 | 3.9E1 | 1.3E1 | 1.0E-9 | 1.0E-9 | 2.0E2 | 2.7E1 | 974 | 7 | 355 | 7 | 0.42 |
| Nl | pg/ml | 4.5E1 | 2.3E1 | 6.0E1 | 5.0E1 | 6.7E1 | 6.6E1 | 1.0E-9 | 1.0E-9 | 1.1E3 | 1.8E2 | 974 | 7 | 355 | 7 | 0.42 |
| Hq | pg/ml | 1.1E0 | 1.7E0 | 9.6E1 | 4.5E1 | 1.6E3 | 1.1E2 | 1.0E-9 | 1.0E-9 | 3.4E4 | 3.0E2 | 970 | 7 | 354 | 7 | 0.56 |
| Hr | pg/ml | 1.1E2 | 4.5E1 | 7.6E2 | 1.6E3 | 1.6E3 | 4.0E3 | 1.0E-9 | 2.1E1 | 1.7E4 | 1.1E4 | 970 | 7 | 354 | 7 | 0.36 |
| Hu | pg/ml | 7.1E0 | 8.5E1 | 2.9E3 | 8.2E2 | 2.5E4 | 1.1E3 | 1.0E-9 | 1.0E-9 | 6.3E5 | 2.5E3 | 970 | 7 | 354 | 7 | 0.69 |
| Hv | pg/ml | 1.5E0 | 2.3E0 | 4.2E0 | 1.0E1 | 3.1E1 | 2.1E1 | 1.0E-9 | 9.7E-1 | 8.9E2 | 5.9E1 | 970 | 7 | 354 | 7 | 0.67 |
| Hw | pg/ml | 6.3E0 | 2.1E0 | 2.7E1 | 5.0E2 | 3.1E2 | 1.3E3 | 1.0E-9 | 3.2E-1 | 9.4E3 | 3.4E3 | 970 | 7 | 354 | 7 | 0.36 |
| Hx | pg/ml | 9.2E0 | 3.9E1 | 3.8E1 | 3.2E2 | 3.1E2 | 7.3E2 | 1.0E-9 | 8.8E0 | 9.3E3 | 2.0E3 | 970 | 7 | 354 | 7 | 0.79 |
| Ih | ng/ml | 7.4E1 | 2.9E2 | 2.6E2 | 4.0E2 | 5.8E2 | 4.3E2 | 1.0E-9 | 2.4E0 | 8.1E3 | 1.2E3 | 974 | 7 | 354 | 7 | 0.66 |
| Ii | ng/ml | 9.3E1 | 8.9E1 | 2.4E2 | 1.5E3 | 6.6E2 | 3.4E3 | 1.0E-9 | 7.5E-1 | 1.0E4 | 9.2E3 | 973 | 7 | 354 | 7 | 0.54 |
| In | ng/ml | 3.3E0 | 5.4E-1 | 2.5E1 | 9.4E1 | 2.0E2 | 2.2E2 | 1.0E-9 | 3.1E-2 | 4.5E3 | 5.9E2 | 974 | 7 | 354 | 7 | 0.41 |
| Io | ng/ml | 8.2E3 | 8.0E3 | 2.4E4 | 8.7E4 | 1.4E5 | 2.1E5 | 1.0E-9 | 1.3E3 | 4.0E6 | 5.5E5 | 965 | 7 | 354 | 7 | 0.55 |
| Ip | ng/ml | 1.0E1 | 3.0E1 | 2.0E1 | 4.0E1 | 2.5E1 | 4.6E1 | 1.0E-9 | 1.1E-2 | 2.6E2 | 1.4E2 | 965 | 7 | 354 | 7 | 0.65 |
| Iq | ug/ml | 1.0E-1 | 2.5E-1 | 2.9E1 | 1.1E2 | 6.2E2 | 2.8E2 | 1.0E-9 | 1.0E-9 | 1.4E4 | 7.5E2 | 965 | 7 | 354 | 7 | 0.53 |
| Ir | ug/ml | 3.7E-1 | 1.7E0 | 4.4E0 | 2.1E1 | 2.8E1 | 4.2E1 | 1.0E-9 | 3.5E-2 | 5.1E2 | 1.1E2 | 964 | 7 | 354 | 7 | 0.77 |
| Is | ng/ml | 1.7E0 | 1.3E1 | 7.1E0 | 5.5E1 | 2.3E1 | 8.7E1 | 1.0E-9 | 4.3E-1 | 5.5E2 | 2.3E2 | 965 | 7 | 354 | 7 | 0.76 |
| It | ng/ml | 2.0E0 | 2.0E0 | 2.3E1 | 8.7E1 | 1.3E2 | 2.2E2 | 1.0E-9 | 1.0E-9 | 2.8E3 | 5.9E2 | 965 | 7 | 354 | 7 | 0.55 |
| Iu | ng/ml | 2.1E2 | 1.0E3 | 1.4E3 | 4.5E3 | 4.1E3 | 8.8E3 | 1.0E-9 | 1.0E-9 | 2.9E4 | 2.4E4 | 965 | 7 | 354 | 7 | 0.59 |
| Iv | ng/ml | 1.4E1 | 1.8E1 | 6.8E1 | 9.6E2 | 5.5E2 | 2.4E3 | 1.0E-9 | 5.7E0 | 1.6E4 | 6.4E3 | 964 | 7 | 354 | 7 | 0.64 |
| Pz | ng/ml | 3.9E3 | 4.3E3 | 8.1E3 | 6.4E3 | 3.6E4 | 5.1E3 | 1.3E1 | 4.0E1 | 1.0E6 | 1.4E4 | 966 | 7 | 352 | 7 | 0.59 |
| Qa | ng/ml | 3.6E3 | 1.6E4 | 6.8E3 | 1.5E4 | 1.0E4 | 1.0E4 | 1.2E1 | 9.4E2 | 2.2E5 | 3.1E4 | 966 | 7 | 352 | 7 | 0.76 |
| Qb | ng/ml | 1.0E2 | 6.0E2 | 2.2E2 | 5.8E2 | 4.8E2 | 5.3E2 | 7.9E-1 | 3.2E1 | 8.3E3 | 1.6E3 | 966 | 7 | 352 | 7 | 0.75 |
| Qc | ng/ml | 2.3E2 | 5.0E2 | 6.2E2 | 7.2E2 | 5.4E2 | 8.3E2 | 1.0E-9 | 3.2E1 | 1.7E5 | 2.4E3 | 966 | 7 | 352 | 7 | 0.61 |
| Qd | ng/ml | 9.4E3 | 2.6E4 | 2.2E4 | 6.2E4 | 7.6E4 | 6.5E4 | 1.5E2 | 1.9E3 | 2.0E6 | 1.7E5 | 966 | 7 | 352 | 7 | 0.72 |
| Qe | ng/ml | 9.7E2 | 3.7E3 | 2.0E3 | 4.9E3 | 4.7E3 | 4.6E3 | 1.0E-9 | 1.2E2 | 9.7E4 | 1.4E4 | 966 | 7 | 352 | 7 | 0.76 |
| Jg | ng/ml | 5.1E2 | 1.4E3 | 8.4E2 | 2.1E3 | 1.0E3 | 2.0E3 | 1.0E-9 | 8.4E1 | 1.0E4 | 5.4E3 | 970 | 7 | 354 | 7 | 0.68 |
| Jh | ng/ml | 3.2E0 | 6.0E1 | 2.8E1 | 6.8E1 | 1.1E2 | 7.5E1 | 1.0E-9 | 1.0E-9 | 1.6E3 | 2.1E2 | 970 | 7 | 354 | 7 | 0.74 |
| Ji | ng/ml | 5.4E1 | 1.6E2 | 8.4E1 | 2.0E2 | 1.1E2 | 2.0E2 | 1.0E-9 | 2.3E1 | 1.8E3 | 5.9E2 | 970 | 7 | 354 | 7 | 0.70 |
| Jj | ng/ml | 5.7E2 | 9.8E1 | 1.6E3 | 2.4E2 | 1.2E4 | 3.5E2 | 1.5E0 | 8.7E0 | 3.4E5 | 1.0E3 | 970 | 7 | 354 | 7 | 0.20 |
| Jk | ng/ml | 3.3E0 | 4.2E1 | 2.2E1 | 7.1E1 | 4.6E1 | 7.8E1 | 1.0E-9 | 1.2E-1 | 3.9E2 | 1.8E2 | 970 | 7 | 354 | 7 | 0.70 |
| Jl | ng/ml | 4.5E-1 | 2.0E1 | 2.6E0 | 3.3E1 | 1.8E1 | 5.6E1 | 7.6E-4 | 2.5E-1 | 5.4E2 | 1.6E2 | 970 | 7 | 354 | 7 | 0.87 |
| Jm | ng/ml | 1.8E1 | 1.9E1 | 5.8E1 | 4.4E1 | 1.3E2 | 7.2E1 | 1.0E-9 | 4.0E-1 | 2.1E3 | 2.0E2 | 970 | 7 | 354 | 7 | 0.48 |
| Jn | pg/ml | 4.0E-1 | 1.2E0 | 3.9E0 | 2.5E0 | 3.8E1 | 3.1E0 | 1.0E-9 | 2.8E-2 | 7.3E2 | 7.2E0 | 969 | 7 | 354 | 7 | 0.67 |
| Jo | pg/ml | 3.5E3 | 1.6E3 | 4.8E3 | 6.8E3 | 4.9E3 | 1.4E4 | 2.0E1 | 2.4E1 | 1.0E5 | 3.8E4 | 970 | 7 | 354 | 7 | 0.32 |
| Jp | pg/ml | 7.2E4 | 1.1E5 | 7.5E4 | 1.3E5 | 3.8E4 | 5.2E4 | 5.8E2 | 6.8E4 | 3.8E5 | 2.2E5 | 970 | 7 | 354 | 7 | 0.81 |
| Jq | pg/ml | 9.5E1 | 1.2E2 | 1.7E2 | 6.9E2 | 3.7E2 | 1.5E3 | 1.0E0 | 1.3E1 | 8.7E3 | 4.1E3 | 970 | 7 | 354 | 7 | 0.51 |
| Jr | pg/ml | 5.5E0 | 2.6E1 | 4.6E1 | 6.3E1 | 4.6E2 | 7.9E1 | 1.0E-9 | 9.5E0 | 1.1E4 | 1.9E2 | 970 | 7 | 354 | 7 | 0.84 |
| Js | pg/ml | 1.3E1 | 1.6E1 | 5.5E1 | 2.9E1 | 3.8E2 | 3.2E1 | 1.0E-9 | 2.7E0 | 1.0E4 | 9.4E1 | 970 | 7 | 354 | 7 | 0.58 |
| Jt | pg/ml | 2.6E3 | 2.1E3 | 3.3E3 | 6.2E3 | 2.8E3 | 1.2E4 | 2.2E1 | 1.5E2 | 5.2E4 | 3.3E4 | 970 | 7 | 354 | 7 | 0.40 |
| Lh | pg/ml | 1.3E4 | 3.4E4 | 2.2E4 | 1.1E5 | 3.2E4 | 1.4E5 | 1.0E-9 | 2.0E3 | 4.8E5 | 4.1E5 | 969 | 7 | 355 | 7 | 0.78 |
| Li | pg/ml | 3.7E3 | 5.4E3 | 1.8E4 | 1.1E5 | 6.4E4 | 2.2E5 | 1.0E-9 | 3.6E1 | 1.3E6 | 5.9E5 | 969 | 7 | 355 | 7 | 0.57 |
| Lj | pg/ml | 2.9E3 | 8.3E3 | 2.5E4 | 5.9E4 | 7.0E4 | 1.3E5 | 1.0E-9 | 8.9E1 | 6.1E5 | 3.5E5 | 969 | 7 | 355 | 7 | 0.58 |
| Nv | pg/ml | 4.0E3 | 1.2E4 | 1.1E4 | 3.0E4 | 4.2E4 | 3.5E4 | 1.0E-9 | 1.6E2 | 1.1E6 | 8.4E4 | 976 | 7 | 355 | 7 | 0.75 |
| Nw | pg/ml | 9.2E3 | 1.9E4 | 1.4E4 | 5.8E4 | 1.8E4 | 7.1E4 | 8.6E1 | 4.5E3 | 2.1E5 | 1.7E5 | 976 | 7 | 355 | 7 | 0.73 |
| Nx | pg/ml | 2.2E2 | 4.6E1 | 4.2E2 | 4.7E2 | 6.6E2 | 7.2E2 | 1.0E-9 | 1.0E-9 | 7.4E3 | 1.9E3 | 976 | 7 | 355 | 7 | 0.47 |
| Ny | pg/ml | 6.7E0 | 2.3E1 | 5.5E1 | 1.6E2 | 8.2E2 | 2.5E2 | 1.0E-9 | 1.0E-9 | 2.5E4 | 6.2E2 | 976 | 7 | 355 | 7 | 0.68 |
| Oe | pg/ml | 7.0E1 | 1.0E-9 | 2.8E2 | 2.3E2 | 7.3E2 | 6.1E2 | 1.0E-9 | 1.0E-9 | 1.9E4 | 1.6E3 | 967 | 7 | 354 | 7 | 0.31 |
| Of | pg/ml | 1.7E2 | 8.1E1 | 5.8E3 | 1.1E4 | 2.7E4 | 2.8E4 | 1.0E-9 | 1.0E-9 | 6.2E5 | 7.4E4 | 975 | 7 | 355 | 7 | 0.44 |
| Og | pg/ml | 8.2E-2 | 1.0E-9 | 7.0E-1 | 4.7E-2 | 4.6E0 | 1.0E-1 | 1.0E-9 | 1.0E-9 | 8.3E1 | 2.7E-1 | 975 | 7 | 355 | 7 | 0.24 |

200

| Code | Units | Median | | Average | | Standard Deviation | | Minimum | | Maximum | | Number of Samples | | Number of Patients | | AUC |
|------|-------|--------|------|---------|------|-------|------|---------|--------|---------|------|--------|-----|--------|-----|-----|
| | | NonDis | Dis | NonDis | Dis | NonDis | Dis | NonDis | Dis | NonDis | Dis | NonDis | Dis | NonDis | Dis | |
| Oh | pg/ml | 2.8E0 | 5.3E0 | 2.1E1 | 3.9E1 | 1.4E2 | 8.3E1 | 1.0E-9 | 1.0E-9 | 3.5E3 | 2.2E2 | 975 | 7 | 355 | 7 | 0.53 |
| Oi | pg/ml | 2.5E0 | 3.3E-1 | 6.2E0 | 1.9E0 | 9.7E0 | 2.7E0 | 1.0E-9 | 1.0E-9 | 8.6E1 | 6.4E0 | 975 | 7 | 355 | 7 | 0.38 |
| Ok | pg/ml | 3.9E2 | 6.5E2 | 6.5E2 | 1.9E3 | 2.3E3 | 3.5E3 | 1.3E1 | 5.3E1 | 7.0E4 | 9.8E3 | 975 | 7 | 355 | 7 | 0.61 |
| Om | pg/ml | 4.0E2 | 8.0E2 | 8.9E2 | 5.9E3 | 2.6E3 | 8.5E3 | 1.0E-9 | 7.0E1 | 5.1E4 | 2.0E4 | 975 | 7 | 355 | 7 | 0.63 |
| On | pg/ml | 1.9E2 | 7.5E2 | 3.2E2 | 2.9E3 | 5.2E2 | 5.4E3 | 1.0E-9 | 1.6E1 | 9.8E3 | 1.5E4 | 975 | 7 | 355 | 7 | 0.78 |
| Oy | pg/ml | 4.8E-1 | 2.4E-1 | 5.5E0 | 4.1E1 | 2.8E1 | 1.0E2 | 1.0E-9 | 1.0E-9 | 4.0E2 | 2.7E2 | 974 | 7 | 354 | 7 | 0.48 |
| Oz | pg/ml | 7.0E-3 | 1.0E-9 | 3.1E-1 | 2.3E-1 | 1.3E0 | 3.3E-1 | 1.0E-9 | 1.0E-9 | 2.9E1 | 7.0E-1 | 974 | 7 | 354 | 7 | 0.49 |
| Pa | pg/ml | 4.0E-1 | 3.5E0 | 1.5E0 | 5.0E1 | 5.9E0 | 1.1E2 | 1.0E-9 | 1.7E-1 | 1.0E2 | 2.9E2 | 974 | 7 | 354 | 7 | 0.82 |
| Pb | pg/ml | 1.0E-9 | 6.9E-2 | 7.5E-1 | 2.6E1 | 1.6E1 | 6.8E1 | 1.0E-9 | 1.0E-9 | 4.9E2 | 1.8E2 | 974 | 7 | 354 | 7 | 0.57 |
| Pc | pg/ml | 4.7E-2 | 4.2E-1 | 3.6E-1 | 2.8E0 | 8.6E-1 | 4.4E0 | 1.0E-9 | 1.0E-9 | 1.6E1 | 1.1E1 | 974 | 7 | 354 | 7 | 0.62 |
| Pd | pg/ml | 1.9E0 | 2.3E0 | 4.9E0 | 3.7E0 | 2.8E1 | 6.4E0 | 1.0E-9 | 1.0E-9 | 8.4E2 | 1.8E1 | 974 | 7 | 354 | 7 | 0.44 |
| Pe | pg/ml | 2.3E1 | 1.2E2 | 1.2E2 | 2.4E3 | 4.3E2 | 5.3E3 | 1.0E-9 | 3.3E0 | 6.7E3 | 1.4E4 | 974 | 7 | 354 | 7 | 0.75 |
| Pf | pg/ml | 1.7E0 | 1.3E1 | 1.1E1 | 4.1E1 | 5.8E1 | 8.4E1 | 1.0E-9 | 1.0E-9 | 1.5E3 | 2.3E2 | 974 | 7 | 354 | 7 | 0.70 |
| Pg | pg/ml | 3.6E0 | 4.9E1 | 4.2E1 | 3.5E2 | 3.3E2 | 6.9E2 | 1.0E-9 | 1.9E0 | 7.7E3 | 1.9E3 | 974 | 7 | 354 | 7 | 0.80 |
| aA | mg/dL | 8.3E-1 | 1.9E0 | 9.7E-1 | 2.6E0 | 5.2E-1 | 1.7E0 | 2.0E-1 | 1.1E0 | 4.2E0 | 5.4E0 | 2812 | 12 | 531 | 12 | 0.89 |

Figure 17.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ad | ug/mL | 4.1E-2 | 6.4E-2 | 7.5E-2 | 6.5E-2 | 9.0E-2 | 4.8E-2 | 2.7E-4 | 1.1E-2 | 5.4E-1 | 1.5E-1 | 454 | 7 | 168 | 7 | 0.55 |
| Af | ng/mL | 1.1E0 | 3.6E-1 | 1.5E1 | 3.2E-1 | 6.0E1 | 1.5E-1 | 1.7E-3 | 1.7E-3 | 5.3E2 | 4.6E-1 | 454 | 7 | 168 | 7 | 0.28 |
| Aj | ug/mL | 1.6E0 | 4.8E-1 | 2.6E0 | 1.9E0 | 2.4E0 | 2.1E0 | 1.5E-3 | 4.0E-3 | 6.1E0 | 4.8E0 | 454 | 7 | 168 | 7 | 0.38 |
| Al | mg/mL | 8.7E-5 | 9.7E-5 | 2.5E-4 | 3.1E-4 | 4.0E-4 | 5.2E-4 | 2.3E-6 | 3.7E-5 | 1.9E-3 | 1.5E-3 | 454 | 7 | 168 | 7 | 0.58 |
| An | U/mL | 5.1E1 | 7.2E1 | 1.8E2 | 1.4E2 | 4.4E2 | 1.1E2 | 9.8E-4 | 2.7E1 | 5.5E3 | 3.4E2 | 454 | 7 | 168 | 7 | 0.65 |
| Ao | pg/mL | 8.9E1 | 5.0E1 | 5.1E2 | 7.6E1 | 3.4E3 | 7.9E1 | 2.8E0 | 6.1E0 | 3.9E4 | 2.4E2 | 454 | 7 | 168 | 7 | 0.35 |
| Ap | ng/mL | 3.3E1 | 5.9E1 | 4.5E1 | 5.1E1 | 4.4E1 | 3.1E1 | 8.4E-5 | 5.7E0 | 2.9E2 | 9.7E1 | 454 | 7 | 168 | 7 | 0.60 |
| Ar | ng/mL | 9.7E-1 | 1.9E0 | 1.2E1 | 1.1E1 | 1.9E2 | 1.8E1 | 3.4E-3 | 3.6E-1 | 4.1E3 | 5.0E1 | 454 | 7 | 168 | 7 | 0.66 |
| As | ng/mL | 9.0E-3 | 1.5E-2 | 1.3E-2 | 1.2E-2 | 1.9E-2 | 7.3E-3 | 1.7E-3 | 1.7E-3 | 1.3E-1 | 1.9E-2 | 454 | 7 | 168 | 7 | 0.57 |
| Aw | pg/mL | 1.6E1 | 1.7E1 | 1.6E1 | 1.7E1 | 6.1E0 | 3.1E0 | 2.9E-2 | 1.4E1 | 4.8E1 | 2.3E1 | 454 | 7 | 168 | 7 | 0.56 |
| Ax | ng/mL | 2.4E0 | 1.5E1 | 1.7E1 | 1.6E2 | 6.5E1 | 3.1E2 | 1.2E-2 | 5.8E-1 | 7.7E2 | 8.5E2 | 454 | 7 | 168 | 7 | 0.70 |
| Ba | ng/mL | 7.0E1 | 5.8E2 | 4.4E2 | 9.0E2 | 1.2E3 | 1.0E3 | 3.7E-1 | 5.5E0 | 8.1E3 | 3.0E3 | 454 | 7 | 168 | 7 | 0.68 |
| Bb | ng/mL | 3.1E0 | 4.3E0 | 6.5E0 | 4.8E0 | 1.4E1 | 2.6E0 | 4.1E-3 | 5.5E-1 | 2.5E2 | 8.2E0 | 454 | 7 | 168 | 7 | 0.57 |
| Bc | ng/mL | 3.9E1 | 1.2E2 | 1.1E2 | 2.6E2 | 2.0E2 | 3.4E2 | 1.1E-1 | 3.7E1 | 1.2E3 | 9.9E2 | 454 | 7 | 168 | 7 | 0.78 |
| Bg | ng/mL | 9.1E-2 | 5.4E-1 | 5.9E0 | 7.9E-1 | 3.5E1 | 1.1E0 | 5.3E-4 | 2.2E-2 | 4.4E2 | 3.3E0 | 454 | 7 | 168 | 7 | 0.59 |
| Bn | ng/mL | 5.6E-2 | 5.6E-2 | 1.2E0 | 1.1E-1 | 2.0E0 | 1.0E-1 | 5.6E-2 | 5.6E-2 | 9.7E0 | 3.2E-1 | 454 | 7 | 168 | 7 | 0.36 |
| Bo | ng/mL | 1.2E1 | 2.0E1 | 1.5E1 | 2.3E1 | 1.9E1 | 9.5E0 | 1.6E-2 | 1.1E1 | 2.8E2 | 3.6E1 | 454 | 7 | 168 | 7 | 0.76 |
| Ch | uIU/mL | 1.2E0 | 7.8E-1 | 2.1E1 | 1.1E0 | 1.1E2 | 1.0E0 | 3.4E-3 | 1.7E-1 | 1.8E3 | 3.2E0 | 454 | 7 | 168 | 7 | 0.38 |
| Co | pg/mL | 4.0E1 | 5.0E1 | 1.7E2 | 5.6E1 | 9.5E2 | 5.7E1 | 1.5E-1 | 1.1E1 | 1.7E4 | 1.7E2 | 454 | 7 | 168 | 7 | 0.45 |
| Cp | ng/mL | 2.2E1 | 2.1E1 | 2.9E1 | 2.6E1 | 3.2E1 | 1.4E1 | 6.0E-1 | 1.8E1 | 3.7E2 | 5.6E1 | 454 | 7 | 168 | 7 | 0.53 |
| Cq | ng/mL | 3.0E-2 | 5.3E-2 | 1.6E-1 | 5.7E-2 | 8.8E-1 | 4.6E-2 | 8.0E-4 | 7.8E-3 | 1.7E1 | 1.3E-1 | 454 | 7 | 168 | 7 | 0.59 |
| Cs | ng/mL | 7.3E1 | 2.7E2 | 3.4E2 | 1.7E3 | 1.1E3 | 2.4E3 | 2.7E-2 | 3.0E1 | 1.8E4 | 5.3E3 | 454 | 7 | 168 | 7 | 0.74 |
| Ct | ng/mL | 6.0E-1 | 3.8E-1 | 4.0E1 | 1.8E1 | 1.1E2 | 3.1E1 | 1.1E-4 | 3.8E-2 | 6.2E2 | 7.2E1 | 454 | 7 | 168 | 7 | 0.48 |
| Cu | ng/mL | 2.5E-1 | 6.4E-1 | 5.0E-1 | 8.2E-1 | 1.4E0 | 9.6E-1 | 9.6E-3 | 8.1E-2 | 2.1E1 | 2.9E0 | 454 | 7 | 168 | 7 | 0.66 |
| Cv | ng/mL | 5.1E0 | 1.1E1 | 2.1E1 | 2.5E1 | 5.2E1 | 3.8E1 | 1.4E-4 | 2.3E0 | 5.3E2 | 1.1E2 | 454 | 7 | 168 | 7 | 0.62 |
| Cw | mIU/mL | 3.0E-2 | 4.3E-2 | 3.8E-2 | 5.9E-2 | 3.1E-2 | 3.9E-2 | 1.5E-4 | 1.6E-2 | 2.4E-1 | 1.3E-1 | 454 | 7 | 168 | 7 | 0.68 |
| Cx | ng/mL | 2.2E-1 | 7.4E-3 | 5.6E1 | 6.8E-2 | 1.0E2 | 1.3E-1 | 9.3E-5 | 9.3E-5 | 3.9E2 | 3.6E-1 | 454 | 7 | 168 | 7 | 0.33 |
| Db | ug/mL | 7.3E0 | 6.6E0 | 8.8E0 | 9.4E0 | 9.4E0 | 6.3E0 | 4.5E-1 | 4.3E0 | 1.4E2 | 2.3E1 | 454 | 7 | 168 | 7 | 0.54 |
| Dc | nmol/L | 1.9E-2 | 3.4E-2 | 6.2E-2 | 5.0E-2 | 1.5E-1 | 6.7E-2 | 5.2E-6 | 1.6E-3 | 1.6E0 | 2.0E-1 | 454 | 7 | 168 | 7 | 0.54 |
| Dd | ug/mL | 6.9E-2 | 1.8E-1 | 1.7E-1 | 2.0E-1 | 2.6E-1 | 2.3E-1 | 1.9E-4 | 1.5E-2 | 1.9E0 | 6.9E-1 | 454 | 7 | 168 | 7 | 0.60 |
| De | ng/mL | 3.4E-3 | 3.4E-3 | 8.0E-2 | 3.4E-3 | 1.4E-1 | 0.0E0 | 3.4E-3 | 3.4E-3 | 9.2E-1 | 3.4E-3 | 454 | 7 | 168 | 7 | 0.33 |
| Dg | ng/mL | 3.5E1 | 4.6E1 | 4.6E1 | 4.4E1 | 4.0E1 | 3.0E1 | 1.0E-1 | 4.4E0 | 1.9E2 | 8.4E1 | 454 | 7 | 168 | 7 | 0.52 |
| Di | pg/mL | 1.9E0 | 2.9E0 | 2.2E0 | 1.9E0 | 2.1E0 | 1.6E0 | 1.8E-1 | 1.8E-1 | 1.3E1 | 3.5E0 | 454 | 7 | 168 | 7 | 0.48 |
| Dk | uIU/mL | 1.7E-2 | 1.8E-2 | 8.5E-2 | 1.6E-1 | 5.1E-1 | 3.6E-1 | 1.1E-4 | 5.8E-3 | 8.9E0 | 9.8E-1 | 454 | 7 | 168 | 7 | 0.51 |
| Dl | ng/mL | 2.4E2 | 5.6E2 | 3.3E2 | 4.7E2 | 3.0E2 | 3.2E2 | 1.7E0 | 2.5E1 | 1.5E3 | 8.3E2 | 454 | 7 | 168 | 7 | 0.63 |
| Wm | % | 4.7E-1 | 6.4E0 | 3.9E1 | 1.3E2 | 1.9E2 | 3.2E2 | 5.4E-2 | 8.5E-2 | 2.4E3 | 8.6E2 | 358 | 7 | 178 | 7 | 0.76 |
| Po | pg/ml | 8.0E-1 | 9.0E0 | 8.3E0 | 4.6E1 | 2.2E1 | 7.5E1 | 8.0E-3 | 2.6E-2 | 2.7E2 | 2.2E2 | 799 | 8 | 262 | 8 | 0.62 |
| Et | ng/ml | 1.5E3 | 2.7E3 | 1.7E3 | 2.8E3 | 1.2E3 | 1.7E3 | 7.7E1 | 4.0E2 | 5.0E3 | 5.0E3 | 798 | 8 | 262 | 8 | 0.69 |
| Fp | ng/ml | 1.4E1 | 6.2E1 | 2.6E1 | 6.2E1 | 2.9E1 | 4.6E1 | 6.0E-3 | 3.3E0 | 1.4E2 | 1.4E2 | 828 | 8 | 264 | 8 | 0.76 |
| Fr | ng/ml | 4.1E4 | 2.9E5 | 1.2E5 | 4.0E5 | 1.8E5 | 3.5E5 | 1.9E2 | 2.6E4 | 9.0E5 | 8.9E5 | 916 | 11 | 265 | 11 | 0.78 |
| Nm | pg/ml | 1.7E4 | 3.8E4 | 3.4E4 | 8.6E4 | 8.2E4 | 1.5E5 | 1.0E-9 | 1.0E-9 | 1.6E6 | 4.4E5 | 802 | 8 | 264 | 8 | 0.63 |
| Nn | pg/ml | 1.7E2 | 4.8E3 | 1.9E3 | 1.6E4 | 8.2E3 | 2.5E4 | 1.0E-9 | 2.6E1 | 1.0E5 | 6.9E4 | 802 | 8 | 264 | 8 | 0.76 |
| No | pg/ml | 1.7E1 | 4.0E1 | 3.7E1 | 3.2E2 | 1.1E2 | 5.6E2 | 1.0E-9 | 4.0E0 | 2.5E3 | 1.4E3 | 802 | 8 | 264 | 8 | 0.62 |
| Nq | pg/ml | 2.3E0 | 1.1E1 | 2.0E1 | 5.6E1 | 7.7E1 | 9.0E1 | 1.0E-9 | 1.0E-9 | 1.2E3 | 2.6E2 | 802 | 8 | 264 | 8 | 0.61 |
| Nr | pg/ml | 1.3E0 | 1.2E0 | 3.0E1 | 1.1E3 | 1.9E2 | 3.0E3 | 1.0E-9 | 1.0E-9 | 4.1E3 | 8.5E3 | 802 | 8 | 264 | 8 | 0.53 |
| Ns | pg/ml | 1.0E-9 | 1.0E-9 | 9.6E0 | 2.1E0 | 5.5E1 | 5.8E0 | 1.0E-9 | 1.0E-9 | 1.1E3 | 1.6E1 | 802 | 8 | 264 | 8 | 0.51 |
| Nt | pg/ml | 1.1E2 | 1.4E2 | 1.4E2 | 2.3E2 | 1.1E2 | 2.0E2 | 1.0E-9 | 1.1E2 | 1.5E3 | 6.8E2 | 802 | 8 | 264 | 8 | 0.70 |
| Nu | pg/ml | 2.4E1 | 1.3E2 | 5.6E1 | 1.8E2 | 8.9E1 | 1.7E2 | 1.0E-9 | 2.8E1 | 8.9E2 | 5.8E2 | 802 | 8 | 264 | 8 | 0.84 |
| Lu | pg/ml | 1.0E4 | 6.8E3 | 1.8E4 | 1.4E4 | 6.5E4 | 2.0E4 | 3.5E2 | 1.6E3 | 1.3E6 | 6.1E4 | 805 | 8 | 264 | 8 | 0.42 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|-----|--------|-----|-----|
| | | NonDis | Dis | NonDis | Dis | NonDis | Dis | NonDis | Dis | NonDis | Dis | NonDis | Dis | NonDis | Dis | |
| Lv | pg/ml | 1.0E-9 | 4.1E1 | 1.1E1 | 6.7E1 | 2.3E1 | 6.1E1 | 1.0E-9 | 1.0E-9 | 2.6E2 | 1.9E2 | 805 | 8 | 264 | 8 | 0.86 |
| Lw | pg/ml | 1.0E-9 | 1.0E-9 | 3.2E-1 | 4.8E0 | 3.7E0 | 8.9E0 | 1.0E-9 | 1.0E-9 | 8.0E1 | 2.1E1 | 805 | 8 | 264 | 8 | 0.68 |
| Lx | pg/ml | 1.0E-9 | 5.0E2 | 1.5E2 | 2.1E3 | 4.3E2 | 3.5E3 | 1.0E-9 | 1.0E-9 | 6.2E3 | 1.0E4 | 805 | 8 | 264 | 8 | 0.79 |
| Ly | pg/ml | 1.0E-9 | 9.4E0 | 1.0E1 | 1.1E1 | 2.0E1 | 1.1E1 | 1.0E-9 | 1.0E-9 | 1.6E2 | 2.6E1 | 805 | 8 | 264 | 8 | 0.60 |
| Lz | pg/ml | 1.0E-9 | 1.0E-9 | 3.8E0 | 2.6E1 | 3.1E1 | 7.4E1 | 1.0E-9 | 1.0E-9 | 6.0E2 | 2.1E2 | 805 | 8 | 264 | 8 | 0.53 |
| Ma | pg/ml | 3.0E2 | 1.8E3 | 1.3E3 | 2.7E3 | 3.8E3 | 3.3E3 | 1.0E-9 | 4.0E1 | 6.5E4 | 9.5E3 | 805 | 8 | 264 | 8 | 0.65 |
| Mb | pg/ml | 2.5E1 | 3.1E1 | 3.1E1 | 4.2E1 | 1.5E1 | 2.6E1 | 5.4E0 | 1.9E1 | 2.1E2 | 8.7E1 | 805 | 8 | 264 | 8 | 0.61 |
| Mc | pg/ml | 1.0E-9 | 1.0E-9 | 4.3E-2 | 1.0E-9 | 6.0E-1 | 0.0E0 | 1.0E-9 | 1.0E-9 | 1.3E1 | 1.0E-9 | 805 | 8 | 264 | 8 | 0.49 |
| Md | pg/ml | 1.0E-9 | 1.0E-9 | 3.7E-1 | 1.0E-9 | 2.8E0 | 0.0E0 | 1.0E-9 | 1.0E-9 | 6.5E1 | 1.0E-9 | 805 | 8 | 264 | 8 | 0.46 |
| Me | pg/ml | 3.3E1 | 2.3E1 | 3.2E1 | 2.7E1 | 2.0E1 | 2.3E1 | 1.0E-9 | 3.2E0 | 3.2E2 | 7.9E1 | 805 | 8 | 264 | 8 | 0.32 |
| Mf | pg/ml | 1.0E-9 | 1.0E-9 | 5.5E-1 | 3.4E-1 | 3.0E0 | 7.5E-1 | 1.0E-9 | 1.0E-9 | 5.6E1 | 2.2E0 | 805 | 8 | 264 | 8 | 0.60 |
| Mg | pg/ml | 1.8E0 | 7.7E0 | 7.6E0 | 1.2E1 | 1.2E1 | 1.4E1 | 1.0E-9 | 1.0E-9 | 9.2E1 | 4.0E1 | 805 | 8 | 264 | 8 | 0.62 |
| Mh | pg/ml | 1.0E-9 | 4.2E-2 | 1.3E0 | 6.1E0 | 9.8E0 | 1.3E1 | 1.0E-9 | 1.0E-9 | 1.6E2 | 3.8E1 | 805 | 8 | 264 | 8 | 0.63 |
| Mi | pg/ml | 1.0E-9 | 1.4E1 | 4.8E-1 | 3.7E1 | 5.4E0 | 5.6E1 | 1.0E-9 | 1.0E-9 | 1.2E2 | 1.6E2 | 805 | 8 | 264 | 8 | 0.74 |
| Mj | pg/ml | 1.0E-9 | 1.0E-9 | 4.3E0 | 6.8E1 | 2.4E1 | 1.9E2 | 1.0E-9 | 1.0E-9 | 4.6E2 | 5.4E2 | 805 | 8 | 264 | 8 | 0.53 |
| Mk | pg/ml | 9.1E-1 | 4.6E0 | 1.5E1 | 7.0E2 | 9.1E1 | 2.0E3 | 1.0E-9 | 1.0E-9 | 1.7E3 | 5.6E3 | 805 | 8 | 264 | 8 | 0.57 |
| Ml | pg/ml | 1.0E-9 | 1.0E-9 | 5.2E0 | 3.7E0 | 7.7E1 | 6.2E0 | 1.0E-9 | 1.0E-9 | 2.1E3 | 1.6E1 | 805 | 8 | 264 | 8 | 0.61 |
| Mm | pg/ml | 6.4E2 | 1.6E3 | 1.1E3 | 2.5E3 | 1.3E3 | 2.6E3 | 1.0E-9 | 8.0E1 | 1.1E4 | 6.9E3 | 805 | 8 | 264 | 8 | 0.65 |
| Mn | pg/ml | 5.7E0 | 7.2E0 | 1.1E1 | 1.8E1 | 2.4E1 | 2.2E1 | 1.0E-9 | 6.9E-1 | 3.5E2 | 6.6E1 | 805 | 8 | 264 | 8 | 0.62 |
| Mp | pg/ml | 1.0E-9 | 2.1E1 | 9.3E0 | 6.3E1 | 3.0E1 | 7.9E1 | 1.0E-9 | 1.0E-9 | 6.3E2 | 1.8E2 | 804 | 8 | 264 | 8 | 0.63 |
| Mq | pg/ml | 1.0E-9 | 1.0E-9 | 2.7E0 | 1.8E1 | 1.7E1 | 5.1E1 | 1.0E-9 | 1.0E-9 | 2.4E2 | 1.4E2 | 804 | 8 | 264 | 8 | 0.53 |
| Mr | pg/ml | 1.0E-9 | 5.8E-1 | 2.8E1 | 1.5E3 | 1.5E2 | 4.2E3 | 1.0E-9 | 1.0E-9 | 2.2E3 | 1.2E4 | 804 | 8 | 264 | 8 | 0.58 |
| Ms | pg/ml | 4.1E2 | 4.7E2 | 5.5E2 | 4.4E2 | 6.5E2 | 3.7E2 | 1.0E-9 | 1.0E-9 | 5.9E3 | 8.6E2 | 804 | 8 | 264 | 8 | 0.48 |
| Mt | pg/ml | 4.8E-1 | 1.5E1 | 7.2E0 | 2.2E1 | 4.4E1 | 2.7E1 | 1.0E-9 | 1.0E-9 | 8.7E2 | 7.8E1 | 804 | 8 | 264 | 8 | 0.76 |
| Mu | pg/ml | 1.0E-9 | 4.0E0 | 1.4E0 | 6.6E0 | 1.1E1 | 9.3E0 | 1.0E-9 | 1.0E-9 | 2.3E2 | 2.7E1 | 804 | 8 | 264 | 8 | 0.76 |
| Mv | pg/ml | 1.0E-9 | 3.5E1 | 7.6E1 | 1.2E2 | 3.3E2 | 1.6E2 | 1.0E-9 | 1.0E-9 | 4.5E3 | 4.4E2 | 804 | 8 | 264 | 8 | 0.70 |
| Mw | pg/ml | 4.0E1 | 4.9E2 | 5.5E2 | 1.7E3 | 3.5E3 | 2.9E3 | 1.0E-9 | 1.0E-9 | 6.2E4 | 8.5E3 | 804 | 8 | 264 | 8 | 0.68 |
| Mx | pg/ml | 1.0E-9 | 1.0E-1 | 2.6E-1 | 5.7E-1 | 1.4E0 | 1.1E0 | 1.0E-9 | 1.0E-9 | 3.2E1 | 3.0E0 | 804 | 8 | 264 | 8 | 0.67 |
| My | pg/ml | 1.0E-9 | 3.7E2 | 5.1E2 | 7.6E2 | 3.1E3 | 1.2E3 | 1.0E-9 | 1.0E-9 | 3.9E4 | 3.4E3 | 804 | 8 | 264 | 8 | 0.75 |
| Mz | pg/ml | 1.2E1 | 3.0E1 | 2.8E1 | 6.4E1 | 6.5E1 | 7.7E1 | 1.0E-9 | 5.1E0 | 1.2E3 | 2.0E2 | 804 | 8 | 264 | 8 | 0.65 |
| Na | pg/ml | 1.0E-9 | 1.0E-9 | 6.3E-1 | 1.3E0 | 2.7E0 | 3.8E0 | 1.0E-9 | 1.0E-9 | 3.6E1 | 1.1E1 | 804 | 8 | 264 | 8 | 0.48 |
| Nb | pg/ml | 2.1E0 | 5.8E0 | 4.0E1 | 3.6E1 | 1.2E2 | 8.3E1 | 1.0E-9 | 1.0E-9 | 2.3E2 | 2.4E2 | 804 | 8 | 264 | 8 | 0.70 |
| Nc | pg/ml | 3.4E2 | 1.8E2 | 5.6E2 | 2.0E2 | 7.4E2 | 1.8E2 | 1.0E-9 | 1.0E-9 | 9.2E3 | 5.2E2 | 804 | 8 | 264 | 8 | 0.37 |
| Nd | pg/ml | 2.8E1 | 2.0E1 | 2.7E1 | 4.9E1 | 4.7E1 | 5.8E1 | 1.0E-9 | 1.0E-9 | 1.2E3 | 1.4E2 | 804 | 8 | 264 | 8 | 0.52 |
| Ne | pg/ml | 4.5E2 | 1.7E2 | 5.8E2 | 2.4E2 | 5.9E2 | 2.6E2 | 1.0E-9 | 1.3E1 | 7.0E3 | 7.6E2 | 804 | 8 | 264 | 8 | 0.31 |
| Nf | pg/ml | 1.0E-9 | 1.0E-9 | 2.5E0 | 1.2E1 | 9.5E0 | 2.9E1 | 1.0E-9 | 1.0E-9 | 1.6E2 | 8.2E1 | 804 | 8 | 264 | 8 | 0.53 |
| Ng | pg/ml | 2.8E1 | 6.2E1 | 1.3E2 | 1.4E2 | 2.5E2 | 1.8E2 | 1.0E-9 | 1.0E-9 | 2.3E3 | 4.7E2 | 804 | 8 | 264 | 8 | 0.49 |
| Nh | pg/ml | 6.8E1 | 3.1E1 | 9.1E1 | 3.5E1 | 8.5E1 | 2.2E1 | 1.0E-9 | 2.2E0 | 5.6E2 | 6.9E1 | 804 | 8 | 264 | 8 | 0.27 |
| Ni | pg/ml | 1.0E-9 | 6.4E1 | 7.4E1 | 1.1E2 | 1.2E2 | 1.4E2 | 1.0E-9 | 1.0E-9 | 7.1E2 | 3.9E2 | 804 | 8 | 264 | 8 | 0.63 |
| Nj | pg/ml | 7.3E0 | 6.9E0 | 1.1E1 | 7.4E0 | 1.2E1 | 6.4E0 | 1.0E-9 | 6.8E-1 | 1.1E2 | 2.0E1 | 804 | 8 | 264 | 8 | 0.45 |
| Nk | pg/ml | 1.7E1 | 1.9E1 | 3.3E1 | 2.0E1 | 4.0E1 | 7.4E0 | 1.0E-9 | 5.7E0 | 2.0E2 | 2.8E1 | 804 | 8 | 264 | 8 | 0.52 |
| Nl | pg/ml | 4.6E1 | 1.9E1 | 6.1E1 | 2.8E1 | 6.9E1 | 2.5E1 | 1.0E-9 | 1.0E-9 | 1.1E3 | 7.0E1 | 804 | 8 | 264 | 8 | 0.34 |
| Hq | pg/ml | 1.1E0 | 2.4E0 | 1.1E2 | 3.9E1 | 1.7E3 | 1.0E2 | 1.0E-9 | 4.8E-1 | 3.4E4 | 3.0E2 | 800 | 8 | 263 | 8 | 0.65 |
| Hr | pg/ml | 1.1E2 | 1.2E2 | 7.4E2 | 1.4E3 | 1.6E3 | 3.8E3 | 1.0E-9 | 2.7E1 | 1.7E4 | 1.1E4 | 800 | 8 | 263 | 8 | 0.46 |
| Hu | pg/ml | 7.3E0 | 2.0E2 | 3.1E3 | 6.1E2 | 2.7E4 | 1.0E3 | 1.0E-9 | 1.0E-9 | 6.3E5 | 3.0E3 | 800 | 8 | 263 | 8 | 0.69 |
| Hv | pg/ml | 1.5E0 | 3.8E0 | 3.3E0 | 1.0E1 | 1.2E1 | 2.0E1 | 1.0E-9 | 1.3E0 | 2.5E2 | 5.9E1 | 800 | 8 | 263 | 8 | 0.80 |
| Hw | pg/ml | 6.3E0 | 3.2E0 | 1.9E1 | 4.4E2 | 7.4E1 | 1.2E3 | 1.0E-9 | 1.0E-9 | 1.7E3 | 3.4E3 | 800 | 8 | 263 | 8 | 0.39 |
| Hx | pg/ml | 9.2E0 | 1.9E1 | 4.0E1 | 2.7E2 | 3.4E2 | 6.9E2 | 1.0E-9 | 2.9E0 | 9.3E3 | 2.0E3 | 800 | 8 | 263 | 8 | 0.69 |
| Ih | ng/ml | 7.7E1 | 1.4E2 | 2.4E2 | 3.5E2 | 4.9E2 | 4.9E2 | 1.0E-9 | 2.4E1 | 7.4E3 | 1.2E3 | 804 | 8 | 263 | 8 | 0.60 |
| Ii | ng/ml | 9.8E1 | 7.3E1 | 2.5E2 | 1.2E3 | 6.8E2 | 3.2E3 | 1.0E-9 | 7.4E0 | 1.0E4 | 9.2E3 | 804 | 8 | 263 | 8 | 0.45 |
| Ij | ng/ml | 8.1E1 | 1.1E2 | 1.8E2 | 8.9E2 | 5.5E2 | 2.2E3 | 1.6E-1 | 2.8E1 | 6.4E3 | 6.4E3 | 794 | 8 | 261 | 8 | 0.57 |
| Ik | ng/ml | 1.3E1 | 1.4E2 | 9.0E2 | 3.3E2 | 8.7E3 | 4.8E2 | 5.9E-1 | 4.5E0 | 1.2E5 | 1.5E3 | 800 | 8 | 261 | 8 | 0.67 |
| Il | ng/ml | 3.7E2 | 1.8E2 | 1.3E3 | 1.8E3 | 2.8E3 | 4.5E3 | 1.0E-9 | 1.0E-9 | 1.3E4 | 1.2E4 | 787 | 7 | 261 | 7 | 0.34 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Im | ng/ml | 2.3E2 | 7.0E2 | 4.1E2 | 7.4E2 | 6.1E2 | 5.7E2 | 1.3E1 | 4.2E1 | 6.8E3 | 1.7E3 | 800 | 8 | 262 | 8 | 0.69 |
| In | ng/ml | 3.3E0 | 4.7E-1 | 2.2E1 | 1.0E1 | 1.5E2 | 2.4E1 | 1.0E-9 | 1.0E-9 | 3.9E3 | 6.8E1 | 804 | 8 | 263 | 8 | 0.33 |
| Io | ng/ml | 9.2E3 | 6.7E3 | 2.7E4 | 7.7E4 | 1.6E5 | 1.9E5 | 1.0E-9 | 9.1E2 | 4.0E6 | 5.5E5 | 796 | 8 | 263 | 8 | 0.49 |
| Ip | ng/ml | 1.2E1 | 3.0E1 | 2.0E1 | 4.6E1 | 2.4E1 | 5.2E1 | 1.0E-9 | 4.1E-1 | 2.6E2 | 1.4E2 | 796 | 8 | 263 | 8 | 0.64 |
| Iq | ug/ml | 1.1E-1 | 2.8E-1 | 1.8E1 | 9.5E1 | 4.8E2 | 2.7E2 | 1.0E-9 | 1.0E-9 | 1.4E4 | 7.5E2 | 796 | 8 | 263 | 8 | 0.47 |
| Ir | ug/ml | 3.7E-1 | 1.1E0 | 2.6E0 | 1.6E1 | 1.5E1 | 4.0E1 | 1.0E-9 | 1.3E-1 | 2.4E2 | 1.1E2 | 795 | 8 | 263 | 8 | 0.68 |
| Is | ng/ml | 1.6E0 | 8.4E0 | 6.0E0 | 3.9E1 | 1.3E1 | 7.8E1 | 1.0E-9 | 6.2E-1 | 1.5E2 | 2.3E2 | 796 | 8 | 263 | 8 | 0.72 |
| It | ng/ml | 2.0E0 | 9.5E-1 | 2.0E1 | 8.9E1 | 1.3E2 | 2.1E2 | 1.0E-9 | 1.0E-9 | 2.8E3 | 5.9E2 | 796 | 8 | 263 | 8 | 0.47 |
| Iu | ng/ml | 2.3E2 | 2.0E2 | 1.3E3 | 3.2E3 | 3.8E3 | 8.5E3 | 1.0E-9 | 1.0E-9 | 2.9E4 | 2.4E4 | 796 | 8 | 263 | 8 | 0.45 |
| Iv | ng/ml | 1.4E1 | 5.1E1 | 4.5E1 | 8.5E2 | 1.6E2 | 2.3E3 | 1.0E-9 | 4.1E-1 | 3.8E3 | 6.4E3 | 795 | 8 | 263 | 8 | 0.67 |
| Pz | ng/ml | 4.7E3 | 1.0E4 | 8.8E3 | 7.3E3 | 4.0E4 | 5.1E3 | 1.3E1 | 3.1E2 | 1.0E6 | 1.4E4 | 796 | 8 | 261 | 8 | 0.59 |
| Qa | ng/ml | 3.6E3 | 1.3E4 | 6.5E3 | 1.1E4 | 7.6E3 | 6.5E3 | 1.2E1 | 1.8E3 | 5.2E4 | 1.8E4 | 796 | 8 | 261 | 8 | 0.71 |
| Qb | ng/ml | 1.0E2 | 2.2E2 | 2.2E2 | 3.0E2 | 4.8E2 | 2.5E2 | 7.9E-1 | 5.2E1 | 8.3E3 | 6.0E2 | 796 | 8 | 261 | 8 | 0.66 |
| Qc | ng/ml | 2.6E2 | 3.5E2 | 6.8E2 | 3.7E2 | 5.9E3 | 2.7E2 | 1.0E-9 | 3.9E1 | 1.7E5 | 8.6E2 | 796 | 8 | 261 | 8 | 0.53 |
| Qd | ng/ml | 1.0E4 | 1.8E4 | 2.2E4 | 3.5E4 | 8.3E4 | 3.9E4 | 2.4E2 | 4.8E3 | 2.0E6 | 1.2E5 | 796 | 8 | 261 | 8 | 0.66 |
| Qe | ng/ml | 1.1E3 | 2.3E3 | 1.9E3 | 4.2E3 | 4.1E3 | 4.5E3 | 7.6E0 | 7.8E2 | 9.7E4 | 1.4E4 | 796 | 8 | 261 | 8 | 0.73 |
| Jg | ng/ml | 5.4E2 | 1.8E3 | 8.6E2 | 2.5E3 | 1.0E3 | 2.2E3 | 1.0E-9 | 1.0E2 | 1.0E4 | 5.4E3 | 800 | 8 | 263 | 8 | 0.73 |
| Jh | ng/ml | 3.3E0 | 4.5E1 | 2.9E1 | 7.7E1 | 1.2E2 | 8.3E1 | 1.0E-9 | 1.0E-9 | 1.6E3 | 2.1E2 | 800 | 8 | 263 | 8 | 0.76 |
| Ji | ng/ml | 5.4E1 | 1.1E2 | 7.6E1 | 3.7E2 | 7.6E1 | 6.2E2 | 1.0E-9 | 4.6E1 | 5.4E2 | 1.8E3 | 800 | 8 | 263 | 8 | 0.73 |
| Jj | ng/ml | 6.6E2 | 1.3E2 | 1.8E3 | 1.5E2 | 1.3E4 | 1.1E2 | 4.8E0 | 1.7E1 | 3.4E5 | 2.7E2 | 800 | 8 | 263 | 8 | 0.12 |
| Jk | ng/ml | 3.4E0 | 1.9E1 | 2.3E1 | 3.8E1 | 4.9E1 | 5.9E1 | 1.0E-9 | 1.0E-1 | 3.9E2 | 1.7E2 | 800 | 8 | 263 | 8 | 0.61 |
| Jl | ng/ml | 4.7E-1 | 1.9E0 | 2.1E0 | 2.4E1 | 6.0E0 | 5.4E1 | 7.6E-4 | 7.2E-2 | 1.1E2 | 1.6E2 | 800 | 8 | 263 | 8 | 0.69 |
| Jm | ng/ml | 1.9E1 | 1.4E1 | 6.0E1 | 3.9E1 | 1.3E2 | 5.8E1 | 1.0E-9 | 9.6E-1 | 2.1E3 | 1.5E2 | 800 | 8 | 263 | 8 | 0.45 |
| Jn | pg/ml | 4.0E-1 | 7.0E-1 | 2.0E0 | 3.3E0 | 1.0E1 | 4.2E0 | 1.0E-9 | 1.0E-9 | 2.4E2 | 9.5E0 | 799 | 8 | 263 | 8 | 0.57 |
| Jo | pg/ml | 3.8E3 | 1.9E3 | 4.9E3 | 7.4E3 | 3.8E3 | 1.2E4 | 4.2E1 | 7.2E2 | 2.4E4 | 3.8E4 | 800 | 8 | 263 | 8 | 0.42 |
| Jp | pg/ml | 7.3E4 | 1.2E5 | 7.6E4 | 1.1E5 | 3.7E4 | 3.2E4 | 2.1E3 | 7.0E4 | 3.8E5 | 1.7E5 | 800 | 8 | 263 | 8 | 0.81 |
| Jq | pg/ml | 9.3E1 | 6.6E1 | 1.5E2 | 5.5E2 | 2.1E2 | 1.3E3 | 1.4E0 | 2.7E1 | 4.0E3 | 3.7E3 | 800 | 8 | 263 | 8 | 0.49 |
| Jr | pg/ml | 5.4E0 | 6.1E0 | 2.4E1 | 4.0E1 | 1.3E2 | 6.7E1 | 1.0E-9 | 1.0E-9 | 2.4E3 | 1.9E2 | 800 | 8 | 263 | 8 | 0.59 |
| Js | pg/ml | 1.3E1 | 1.4E1 | 4.2E1 | 2.6E1 | 1.7E2 | 3.0E1 | 1.0E-9 | 3.5E0 | 3.0E3 | 9.4E1 | 800 | 8 | 263 | 8 | 0.54 |
| Jt | pg/ml | 2.8E3 | 3.3E3 | 3.3E3 | 6.7E3 | 2.2E3 | 1.1E4 | 7.7E1 | 4.2E2 | 1.8E4 | 3.3E4 | 800 | 8 | 263 | 8 | 0.54 |
| Lh | pg/ml | 1.4E4 | 2.2E4 | 2.2E4 | 7.7E4 | 2.7E4 | 1.4E5 | 1.0E-9 | 5.6E3 | 2.6E5 | 4.1E5 | 799 | 8 | 264 | 8 | 0.65 |
| Li | pg/ml | 3.6E3 | 3.3E4 | 1.6E4 | 1.2E5 | 4.8E4 | 2.0E5 | 1.0E-9 | 1.5E3 | 9.2E5 | 5.9E5 | 799 | 8 | 264 | 8 | 0.78 |
| Lj | pg/ml | 3.0E3 | 1.7E4 | 2.3E4 | 7.6E4 | 6.4E4 | 1.2E5 | 1.0E-9 | 1.1E3 | 5.2E5 | 3.5E5 | 799 | 8 | 264 | 8 | 0.76 |
| Nv | pg/ml | 4.1E3 | 1.5E4 | 1.2E4 | 3.4E4 | 4.6E4 | 4.5E4 | 1.0E-9 | 1.8E3 | 1.1E6 | 1.3E5 | 805 | 8 | 264 | 8 | 0.74 |
| Nw | pg/ml | 9.4E3 | 2.5E4 | 1.3E4 | 6.0E4 | 1.6E4 | 7.5E4 | 8.6E1 | 3.6E3 | 2.1E5 | 2.1E5 | 805 | 8 | 264 | 8 | 0.78 |
| Nx | pg/ml | 2.2E2 | 3.6E2 | 4.2E2 | 8.7E2 | 6.6E2 | 9.9E2 | 1.0E-9 | 1.0E-9 | 7.4E3 | 2.8E3 | 805 | 8 | 264 | 8 | 0.67 |
| Ny | pg/ml | 7.1E0 | 3.2E1 | 5.9E1 | 1.1E2 | 8.9E2 | 2.1E2 | 1.0E-9 | 1.0E-9 | 2.5E4 | 6.2E2 | 805 | 8 | 264 | 8 | 0.72 |
| Oe | pg/ml | 8.8E1 | 9.6E1 | 3.0E2 | 3.1E2 | 7.9E2 | 5.5E2 | 1.0E-9 | 1.0E-9 | 1.9E4 | 1.6E3 | 797 | 8 | 263 | 8 | 0.51 |
| Of | pg/ml | 2.1E2 | 8.4E1 | 6.5E3 | 1.0E4 | 2.9E4 | 2.6E4 | 1.0E-9 | 1.0E-9 | 6.2E5 | 7.4E4 | 805 | 8 | 264 | 8 | 0.43 |
| Og | pg/ml | 8.5E-2 | 8.2E-2 | 7.9E-1 | 2.3E-1 | 5.0E0 | 4.1E-1 | 1.0E-9 | 1.0E-9 | 8.3E1 | 1.2E0 | 805 | 8 | 264 | 8 | 0.43 |
| Oh | pg/ml | 2.7E0 | 1.3E1 | 2.0E1 | 5.9E1 | 1.5E2 | 9.1E1 | 1.0E-9 | 9.7E-1 | 3.5E3 | 2.2E2 | 805 | 8 | 264 | 8 | 0.73 |
| Oi | pg/ml | 2.9E0 | 2.5E0 | 6.6E0 | 5.5E0 | 9.9E0 | 7.1E0 | 1.0E-9 | 1.0E-9 | 8.6E1 | 1.9E1 | 805 | 8 | 264 | 8 | 0.47 |
| Ok | pg/ml | 4.2E2 | 9.9E2 | 5.6E2 | 1.1E4 | 5.2E2 | 2.4E4 | 1.3E1 | 1.5E2 | 5.2E3 | 7.0E4 | 805 | 8 | 264 | 8 | 0.78 |
| Om | pg/ml | 3.9E2 | 1.3E3 | 8.5E2 | 4.6E3 | 2.2E3 | 6.6E3 | 1.0E-9 | 1.0E-9 | 3.6E4 | 1.7E4 | 805 | 8 | 264 | 8 | 0.65 |
| On | pg/ml | 1.9E2 | 7.3E2 | 3.0E2 | 3.6E3 | 4.0E2 | 5.7E3 | 1.0E-9 | 9.9E1 | 4.5E3 | 1.5E4 | 805 | 8 | 264 | 8 | 0.79 |
| Oy | pg/ml | 5.1E-1 | 6.2E-1 | 6.0E0 | 3.4E1 | 3.0E1 | 9.5E1 | 1.0E-9 | 2.4E-1 | 4.0E2 | 2.7E2 | 804 | 8 | 263 | 8 | 0.59 |
| Oz | pg/ml | 1.2E-2 | 1.2E-1 | 3.2E-1 | 2.2E-1 | 1.4E0 | 2.6E-1 | 1.0E-9 | 1.0E-9 | 2.9E1 | 7.1E-1 | 804 | 8 | 263 | 8 | 0.54 |
| Pa | pg/ml | 4.0E-1 | 5.6E-1 | 1.5E0 | 3.8E1 | 5.2E0 | 1.0E2 | 1.0E-9 | 9.6E-2 | 8.6E1 | 2.9E2 | 804 | 8 | 263 | 8 | 0.64 |
| Pb | pg/ml | 1.0E-9 | 1.7E-1 | 8.5E-1 | 2.3E1 | 1.7E1 | 6.4E1 | 1.0E-9 | 1.0E-9 | 4.9E2 | 1.8E2 | 804 | 8 | 263 | 8 | 0.73 |
| Pc | pg/ml | 6.4E-2 | 5.2E-1 | 3.6E-1 | 2.5E0 | 8.9E-1 | 4.1E0 | 1.0E-9 | 1.0E-9 | 1.6E1 | 1.1E1 | 804 | 8 | 263 | 8 | 0.71 |
| Pd | pg/ml | 2.0E0 | 3.2E0 | 5.0E0 | 6.2E0 | 3.0E1 | 6.7E0 | 1.0E-9 | 6.7E-1 | 8.4E2 | 1.8E1 | 804 | 8 | 263 | 8 | 0.64 |
| Pe | pg/ml | 2.3E1 | 6.1E1 | 1.1E2 | 1.9E3 | 3.6E2 | 5.0E3 | 1.0E-9 | 1.5E1 | 4.7E3 | 1.4E4 | 804 | 8 | 263 | 8 | 0.69 |
| Pf | pg/ml | 1.8E0 | 1.5E1 | 1.0E1 | 4.0E1 | 5.8E1 | 7.8E1 | 1.0E-9 | 5.2E-1 | 1.5E3 | 2.3E2 | 804 | 8 | 263 | 8 | 0.71 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pg | pg/ml | 3.8E0 | 6.3E0 | 4.6E1 | 2.4E2 | 3.6E2 | 6.6E2 | 1.0E-9 | 5.5E-1 | 7.7E3 | 1.9E3 | 804 | 8 | 263 | 8 | 0.62 |
| aA | mg/dL | 8.1E-1 | 1.4E0 | 9.4E-1 | 1.9E0 | 4.7E-1 | 1.3E0 | 2.0E-1 | 6.2E-1 | 4.2E0 | 5.4E0 | 2235 | 16 | 391 | 16 | 0.81 |
| aC | mg/mL | 2.7E0 | 2.4E0 | 3.0E0 | 2.6E0 | 1.3E0 | 1.5E0 | 7.7E-1 | 1.3E0 | 8.2E0 | 6.3E0 | 465 | 8 | 174 | 8 | 0.39 |
| aD | ug/mL | 3.1E0 | 5.6E0 | 4.4E0 | 7.1E0 | 3.4E0 | 5.0E0 | 7.5E-1 | 2.2E0 | 3.1E1 | 1.7E1 | 465 | 8 | 174 | 8 | 0.70 |
| aE | mg/mL | 5.5E-1 | 6.8E-1 | 5.7E-1 | 6.9E-1 | 1.5E-1 | 2.0E-1 | 1.8E-1 | 4.5E-1 | 1.1E0 | 1.2E0 | 465 | 8 | 174 | 8 | 0.71 |
| aF | ng/mL | 2.3E0 | 8.9E-1 | 4.1E0 | 3.9E0 | 5.8E0 | 5.4E0 | 4.3E-3 | 4.3E-3 | 5.0E1 | 1.5E1 | 465 | 8 | 174 | 8 | 0.38 |
| aG | mg/mL | 1.3E-1 | 1.2E-1 | 1.5E-1 | 1.9E-1 | 8.1E-2 | 1.6E-1 | 4.3E-2 | 7.3E-2 | 5.0E-1 | 5.2E-1 | 465 | 8 | 174 | 8 | 0.49 |
| aH | ug/mL | 7.5E1 | 7.6E1 | 8.0E1 | 9.1E1 | 4.2E1 | 5.1E1 | 9.6E0 | 3.6E1 | 2.9E2 | 1.8E2 | 465 | 8 | 174 | 8 | 0.55 |
| aI | ug/mL | 1.8E2 | 2.1E2 | 1.8E2 | 1.9E2 | 6.0E1 | 4.7E1 | 4.7E1 | 1.4E2 | 3.7E2 | 2.7E2 | 465 | 8 | 174 | 8 | 0.55 |
| aJ | ug/mL | 2.5E0 | 6.2E0 | 3.2E0 | 7.2E0 | 2.2E0 | 3.9E0 | 7.3E-1 | 3.3E0 | 1.7E1 | 1.5E1 | 465 | 8 | 174 | 8 | 0.88 |
| aK | ng/mL | 1.5E0 | 1.6E0 | 2.3E0 | 1.9E0 | 2.6E0 | 1.4E0 | 2.9E-4 | 2.1E-1 | 1.8E1 | 4.7E0 | 465 | 8 | 174 | 8 | 0.51 |
| aL | mg/mL | 7.9E-1 | 8.7E-1 | 8.0E-1 | 7.9E-1 | 2.5E-1 | 2.8E-1 | 2.2E-1 | 4.0E-1 | 1.7E0 | 1.2E0 | 465 | 8 | 174 | 8 | 0.52 |
| aM | U/mL | 2.2E1 | 4.5E1 | 4.5E1 | 8.5E1 | 9.2E1 | 1.3E2 | 4.2E-2 | 1.5E1 | 1.6E3 | 4.0E2 | 465 | 8 | 174 | 8 | 0.68 |
| aN | U/mL | 1.5E1 | 3.0E1 | 2.3E1 | 4.0E1 | 3.2E1 | 3.3E1 | 2.5E-3 | 3.9E0 | 3.8E2 | 9.2E1 | 465 | 8 | 174 | 8 | 0.69 |
| aO | pg/mL | 3.5E1 | 9.8E1 | 3.3E2 | 6.7E2 | 8.2E2 | 1.2E3 | 6.0E-2 | 6.0E-2 | 6.6E3 | 3.5E3 | 465 | 8 | 174 | 8 | 0.60 |
| aP | ng/mL | 1.7E0 | 3.2E0 | 2.0E0 | 3.6E0 | 1.3E0 | 1.8E0 | 5.4E-1 | 1.6E0 | 1.1E1 | 6.1E0 | 465 | 8 | 174 | 8 | 0.80 |
| aQ | ng/mL | 2.9E-1 | 2.8E-1 | 4.4E-1 | 2.7E-1 | 4.6E-1 | 1.6E-1 | 2.0E-4 | 6.1E-2 | 4.0E0 | 4.5E-1 | 465 | 8 | 174 | 8 | 0.41 |
| aR | ng/mL | 1.8E0 | 3.5E0 | 2.8E0 | 3.4E0 | 3.4E0 | 1.4E0 | 1.8E-1 | 7.7E-1 | 3.4E1 | 5.0E0 | 465 | 8 | 174 | 8 | 0.70 |
| aS | ng/mL | 2.8E-1 | 3.6E-1 | 6.9E-1 | 5.6E-1 | 1.8E0 | 5.6E-1 | 4.2E-3 | 9.1E-2 | 3.3E1 | 1.6E0 | 465 | 8 | 174 | 8 | 0.54 |
| aU | pg/mL | 7.4E1 | 1.0E2 | 1.2E2 | 1.2E2 | 1.5E2 | 7.6E1 | 7.4E-2 | 1.3E1 | 1.3E3 | 2.6E2 | 465 | 8 | 174 | 8 | 0.60 |
| aV | ng/mL | 6.0E-1 | 5.9E-1 | 1.0E0 | 1.0E0 | 1.9E0 | 1.3E0 | 7.6E-4 | 1.0E-1 | 3.3E1 | 4.2E0 | 465 | 8 | 174 | 8 | 0.51 |
| aW | pg/mL | 1.9E1 | 3.0E1 | 2.0E1 | 7.8E1 | 1.9E1 | 1.5E2 | 7.2E-2 | 7.2E-2 | 2.4E2 | 4.5E2 | 465 | 8 | 174 | 8 | 0.72 |
| aX | ng/mL | 9.6E0 | 9.2E0 | 1.4E1 | 1.4E1 | 1.5E1 | 1.2E1 | 3.0E-1 | 3.6E0 | 1.4E2 | 3.5E1 | 465 | 8 | 174 | 8 | 0.53 |
| aY | pg/mL | 6.0E1 | 6.2E1 | 7.7E1 | 1.0E2 | 8.4E1 | 8.3E1 | 4.1E1 | 4.4E1 | 1.2E3 | 2.7E2 | 465 | 8 | 174 | 8 | 0.60 |
| aZ | pg/mL | 2.3E2 | 4.5E2 | 5.3E2 | 7.6E2 | 1.0E3 | 8.3E2 | 1.7E0 | 1.2E2 | 1.2E4 | 2.1E3 | 465 | 8 | 174 | 8 | 0.65 |
| bA | ng/mL | 9.5E0 | 9.1E1 | 3.4E1 | 1.6E2 | 8.4E1 | 2.0E2 | 3.0E-2 | 2.2E1 | 9.4E2 | 6.2E2 | 465 | 8 | 174 | 8 | 0.88 |
| bB | ng/mL | 2.9E2 | 3.8E2 | 3.1E2 | 3.8E2 | 1.7E2 | 1.5E2 | 8.6E0 | 1.6E2 | 1.0E3 | 6.1E2 | 465 | 8 | 174 | 8 | 0.65 |
| bC | ng/mL | 3.5E2 | 4.2E2 | 5.9E2 | 1.0E3 | 7.6E2 | 1.3E3 | 2.7E1 | 1.8E2 | 4.7E3 | 4.0E3 | 465 | 8 | 174 | 8 | 0.64 |
| bE | mg/mL | 5.4E0 | 6.3E0 | 5.7E0 | 6.5E0 | 2.0E0 | 2.6E0 | 1.4E0 | 3.2E0 | 1.3E1 | 9.6E0 | 465 | 8 | 174 | 8 | 0.58 |
| bF | pg/mL | 2.2E1 | 4.0E1 | 1.9E2 | 3.7E1 | 1.0E3 | 1.5E1 | 5.0E-2 | 8.1E0 | 1.1E4 | 5.3E1 | 465 | 8 | 174 | 8 | 0.63 |
| bG | ng/mL | 1.7E0 | 1.9E0 | 2.9E0 | 4.7E0 | 3.6E0 | 5.0E0 | 2.2E-2 | 2.4E-1 | 3.0E1 | 1.3E1 | 465 | 8 | 174 | 8 | 0.56 |
| bH | pg/mL | 5.7E-1 | 5.7E-1 | 4.9E0 | 6.2E0 | 1.5E1 | 8.9E0 | 5.7E-1 | 5.7E-1 | 2.8E2 | 2.3E1 | 465 | 8 | 174 | 8 | 0.54 |
| bI | ng/mL | 4.0E-3 | 4.0E-3 | 6.5E-2 | 3.9E-2 | 1.6E-1 | 1.0E-1 | 4.0E-3 | 4.0E-3 | 1.3E0 | 2.9E-1 | 465 | 8 | 174 | 8 | 0.47 |
| bJ | mg/mL | 2.1E0 | 2.6E0 | 2.4E0 | 3.0E0 | 1.9E0 | 1.9E0 | 2.5E-4 | 1.2E0 | 1.3E1 | 7.1E0 | 465 | 8 | 174 | 8 | 0.61 |
| bL | pg/mL | 3.8E0 | 7.5E0 | 8.4E0 | 9.1E0 | 1.0E1 | 6.8E0 | 4.6E-2 | 4.6E-2 | 4.9E1 | 2.0E1 | 465 | 8 | 174 | 8 | 0.60 |
| bM | mg/mL | 1.8E0 | 2.1E0 | 2.1E0 | 2.2E0 | 1.4E0 | 1.0E0 | 9.2E-3 | 6.4E-1 | 8.9E0 | 3.5E0 | 465 | 8 | 174 | 8 | 0.55 |
| bN | ng/mL | 4.6E1 | 2.2E1 | 1.3E2 | 2.7E1 | 2.5E2 | 2.2E1 | 1.4E-1 | 2.2E0 | 1.9E3 | 6.7E1 | 465 | 8 | 174 | 8 | 0.32 |
| bO | ng/mL | 4.0E-2 | 4.0E-2 | 9.5E0 | 2.7E1 | 2.3E1 | 6.7E1 | 4.0E-2 | 4.0E-2 | 2.0E2 | 1.9E2 | 465 | 8 | 174 | 8 | 0.48 |
| bP | mg/mL | 5.2E-1 | 8.2E-1 | 7.3E-1 | 9.8E-1 | 6.7E-1 | 8.5E-1 | 8.2E-2 | 9.2E-2 | 4.8E0 | 2.9E0 | 465 | 8 | 174 | 8 | 0.61 |
| bQ | pg/mL | 1.6E1 | 1.8E1 | 6.1E1 | 3.5E1 | 6.3E2 | 3.2E1 | 1.5E-1 | 6.7E0 | 1.3E4 | 9.3E1 | 465 | 8 | 174 | 8 | 0.59 |
| bR | ng/mL | 1.2E-2 | 1.2E-2 | 1.3E-1 | 1.2E-1 | 4.5E-1 | 1.7E-1 | 1.2E-2 | 1.2E-2 | 8.7E0 | 3.9E-1 | 465 | 8 | 174 | 8 | 0.51 |
| bS | pg/mL | 9.4E-1 | 9.4E-1 | 7.2E0 | 9.9E0 | 2.8E1 | 2.5E1 | 9.4E-1 | 9.4E-1 | 3.9E2 | 7.2E1 | 465 | 8 | 174 | 8 | 0.50 |
| bU | ng/mL | 1.1E-1 | 6.8E-2 | 1.9E-1 | 1.1E-1 | 3.7E-1 | 1.3E-1 | 1.3E-2 | 1.3E-2 | 6.6E0 | 3.4E-1 | 465 | 8 | 174 | 8 | 0.43 |
| bV | pg/mL | 4.6E2 | 9.6E2 | 5.7E2 | 9.8E2 | 8.0E2 | 5.6E2 | 1.5E2 | 3.5E2 | 1.7E4 | 2.2E3 | 465 | 8 | 174 | 8 | 0.81 |
| bW | pg/mL | 3.4E2 | 4.4E2 | 5.0E2 | 4.1E2 | 5.6E2 | 1.7E2 | 8.4E1 | 1.8E2 | 6.4E3 | 6.8E2 | 465 | 8 | 174 | 8 | 0.55 |
| bX | ng/mL | 2.5E-5 | 3.2E-3 | 2.7E-3 | 2.9E-3 | 3.4E-3 | 2.7E-3 | 2.5E-5 | 2.5E-5 | 2.0E-2 | 7.2E-3 | 465 | 8 | 174 | 8 | 0.53 |
| bZ | pg/mL | 2.4E2 | 3.4E2 | 9.4E2 | 9.9E2 | 4.3E3 | 1.5E3 | 1.5E-1 | 1.6E2 | 5.8E4 | 4.6E3 | 465 | 8 | 174 | 8 | 0.65 |
| cA | pg/mL | 6.0E-1 | 6.0E-1 | 2.8E0 | 3.1E0 | 1.8E1 | 7.2E0 | 6.0E-1 | 6.0E-1 | 3.7E2 | 2.1E1 | 465 | 8 | 174 | 8 | 0.51 |
| cB | ng/mL | 5.1E-2 | 4.1E-2 | 8.2E-2 | 8.1E-2 | 1.0E-1 | 8.3E-2 | 1.7E-3 | 1.1E-2 | 5.7E-1 | 2.6E-1 | 465 | 8 | 174 | 8 | 0.54 |
| cC | pg/mL | 4.6E1 | 4.6E1 | 4.8E1 | 4.2E1 | 4.0E1 | 2.2E1 | 1.0E0 | 1.0E0 | 4.5E2 | 6.4E1 | 465 | 8 | 174 | 8 | 0.48 |
| cD | pg/mL | 5.2E0 | 8.4E0 | 1.3E1 | 6.9E0 | 3.6E1 | 5.0E0 | 3.3E-1 | 3.3E-1 | 4.8E2 | 1.2E1 | 465 | 8 | 174 | 8 | 0.54 |
| cE | pg/mL | 3.9E1 | 4.0E1 | 1.5E2 | 4.3E1 | 4.5E2 | 3.3E1 | 1.2E-1 | 2.0E0 | 3.8E3 | 9.9E1 | 465 | 8 | 174 | 8 | 0.45 |
| cF | pg/mL | 1.2E1 | 5.3E-1 | 2.0E1 | 1.2E1 | 3.1E1 | 2.5E1 | 5.3E-1 | 5.3E-1 | 2.7E2 | 7.2E1 | 465 | 8 | 174 | 8 | 0.39 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| cG | pg/mL | 4.7E1 | 6.4E1 | 1.1E2 | 7.2E1 | 5.1E2 | 4.2E1 | 6.4E0 | 1.5E1 | 1.0E4 | 1.4E2 | 465 | 8 | 174 | 8 | 0.59 |
| cH | uIU/mL | 2.8E0 | 1.8E0 | 6.2E0 | 6.9E0 | 1.2E1 | 1.2E1 | 8.6E-3 | 8.6E-3 | 1.6E2 | 3.4E1 | 465 | 8 | 174 | 8 | 0.41 |
| cI | ng/mL | 5.5E0 | 9.9E0 | 1.2E1 | 2.1E1 | 1.7E1 | 3.8E1 | 1.0E-3 | 9.7E-1 | 1.2E2 | 1.2E2 | 465 | 8 | 174 | 8 | 0.58 |
| cJ | ug/mL | 5.6E1 | 7.6E1 | 1.1E2 | 8.9E1 | 1.4E2 | 5.2E1 | 4.0E0 | 3.8E1 | 9.6E2 | 1.7E2 | 465 | 8 | 174 | 8 | 0.60 |
| cK | ng/mL | 3.8E-3 | 3.8E-3 | 4.9E-2 | 2.3E-2 | 1.7E-1 | 5.3E-2 | 3.8E-3 | 3.8E-3 | 1.7E0 | 1.6E-1 | 465 | 8 | 174 | 8 | 0.49 |
| cL | pg/mL | 2.0E2 | 1.6E2 | 4.0E2 | 1.7E2 | 1.3E3 | 6.8E1 | 1.6E1 | 8.4E1 | 2.4E4 | 2.9E2 | 465 | 8 | 174 | 8 | 0.40 |
| cM | pg/mL | 2.7E2 | 2.4E2 | 3.0E2 | 2.5E2 | 1.9E2 | 6.9E1 | 8.7E0 | 1.5E2 | 1.6E3 | 3.7E2 | 465 | 8 | 174 | 8 | 0.44 |
| cN | pg/mL | 1.2E2 | 1.7E2 | 1.3E2 | 1.6E2 | 6.5E1 | 4.3E1 | 3.8E1 | 1.0E2 | 1.1E3 | 2.2E2 | 465 | 8 | 174 | 8 | 0.75 |
| cO | pg/mL | 2.2E2 | 2.5E2 | 3.1E2 | 3.3E2 | 9.1E2 | 2.4E2 | 5.4E1 | 1.3E2 | 1.9E4 | 8.8E2 | 465 | 8 | 174 | 8 | 0.59 |
| cP | ng/mL | 2.5E3 | 3.7E3 | 2.6E3 | 3.7E3 | 9.5E2 | 1.1E3 | 6.2E2 | 2.1E3 | 7.3E3 | 5.1E3 | 465 | 8 | 174 | 8 | 0.78 |
| cQ | ng/mL | 5.3E-2 | 1.6E-1 | 1.4E-1 | 1.6E-1 | 2.8E-1 | 1.4E-1 | 2.0E-3 | 2.0E-3 | 2.2E0 | 3.9E-1 | 465 | 8 | 174 | 8 | 0.66 |
| cR | ng/mL | 2.9E2 | 4.9E2 | 4.8E2 | 1.4E3 | 6.9E2 | 2.6E3 | 2.0E1 | 1.6E2 | 7.7E3 | 7.7E3 | 465 | 8 | 174 | 8 | 0.68 |
| cS | ng/mL | 2.6E2 | 5.2E2 | 4.3E2 | 6.0E2 | 1.1E3 | 3.9E2 | 4.7E1 | 2.3E2 | 2.2E4 | 1.2E3 | 465 | 8 | 174 | 8 | 0.73 |
| cT | ng/mL | 3.5E1 | 1.2E2 | 8.9E1 | 2.3E2 | 2.0E2 | 2.3E2 | 3.7E0 | 5.7E1 | 2.1E3 | 6.5E2 | 465 | 8 | 174 | 8 | 0.84 |
| cU | ng/mL | 5.4E1 | 6.9E1 | 7.7E1 | 1.1E2 | 1.0E2 | 1.0E2 | 5.4E0 | 3.0E1 | 1.6E3 | 3.3E2 | 465 | 8 | 174 | 8 | 0.65 |
| cV | ng/mL | 1.8E-1 | 1.4E-1 | 4.4E-1 | 1.8E-1 | 2.3E0 | 1.5E-1 | 3.4E-4 | 6.4E-2 | 4.7E1 | 5.1E-1 | 465 | 8 | 174 | 8 | 0.39 |
| cW | mIU/mL | 5.1E-2 | 9.7E-2 | 1.4E-1 | 1.1E-1 | 7.0E-1 | 5.8E-2 | 3.7E-4 | 2.7E-2 | 9.7E0 | 2.0E-1 | 465 | 8 | 174 | 8 | 0.73 |
| cX | ng/mL | 1.2E-1 | 2.8E-2 | 1.5E0 | 1.6E-1 | 4.6E0 | 3.7E-1 | 2.3E-4 | 2.3E-4 | 2.8E1 | 1.1E0 | 465 | 8 | 174 | 8 | 0.33 |
| cY | ng/mL | 8.5E0 | 9.0E0 | 1.2E1 | 1.2E1 | 1.3E1 | 8.8E0 | 1.5E-1 | 6.6E-1 | 8.3E1 | 2.8E1 | 465 | 8 | 174 | 8 | 0.53 |
| cZ | ug/mL | 1.4E1 | 1.6E1 | 1.5E1 | 1.6E1 | 6.4E0 | 5.1E0 | 2.7E0 | 8.0E0 | 3.9E1 | 2.2E1 | 465 | 8 | 174 | 8 | 0.56 |
| dA | pg/mL | 3.3E2 | 5.1E2 | 3.6E2 | 5.4E2 | 3.0E2 | 2.6E2 | 9.0E1 | 1.7E2 | 5.8E3 | 8.8E2 | 465 | 8 | 174 | 8 | 0.73 |
| dB | ug/mL | 1.7E1 | 1.9E1 | 1.8E1 | 2.2E1 | 1.6E1 | 9.4E0 | 9.4E-1 | 1.3E1 | 2.5E2 | 4.1E1 | 465 | 8 | 174 | 8 | 0.61 |
| dC | nmol/L | 3.5E1 | 3.5E1 | 3.9E1 | 4.1E1 | 1.8E1 | 2.1E1 | 7.6E0 | 2.2E1 | 1.4E2 | 8.2E1 | 465 | 8 | 174 | 8 | 0.51 |
| dD | ug/mL | 3.5E1 | 2.8E1 | 3.6E1 | 3.1E1 | 1.1E1 | 1.0E1 | 1.3E1 | 1.5E1 | 7.6E1 | 4.8E1 | 465 | 8 | 174 | 8 | 0.38 |
| dE | ng/mL | 4.6E-1 | 4.3E-1 | 5.8E-1 | 9.0E-1 | 6.9E-1 | 1.0E0 | 8.4E-3 | 3.5E-1 | 7.2E0 | 3.3E0 | 465 | 8 | 174 | 8 | 0.61 |
| dF | ng/mL | 2.3E2 | 3.1E2 | 2.9E2 | 3.8E2 | 2.0E2 | 2.3E2 | 5.6E1 | 1.7E2 | 1.3E3 | 8.9E2 | 465 | 8 | 174 | 8 | 0.66 |
| dG | ng/mL | 1.2E1 | 1.7E1 | 1.5E1 | 2.0E1 | 1.3E1 | 9.1E0 | 2.2E0 | 1.1E1 | 1.8E2 | 3.5E1 | 465 | 8 | 174 | 8 | 0.73 |
| dH | pg/mL | 7.5E0 | 8.3E0 | 1.3E1 | 1.0E1 | 3.7E1 | 5.9E0 | 4.0E-2 | 3.0E0 | 6.7E2 | 2.2E1 | 465 | 8 | 174 | 8 | 0.57 |
| dI | pg/mL | 4.6E-1 | 4.6E-1 | 2.3E0 | 2.6E0 | 1.6E1 | 3.2E0 | 4.6E-1 | 4.6E-1 | 3.3E2 | 8.4E0 | 465 | 8 | 174 | 8 | 0.61 |
| dJ | ng/mL | 1.9E0 | 2.6E0 | 2.1E0 | 2.5E0 | 1.2E0 | 9.5E-1 | 3.2E-2 | 1.1E0 | 6.9E0 | 3.8E0 | 465 | 8 | 174 | 8 | 0.61 |
| dK | uIU/mL | 1.9E0 | 1.4E0 | 3.1E0 | 2.1E0 | 6.4E0 | 2.2E0 | 2.8E-4 | 3.8E-2 | 7.9E1 | 6.1E0 | 465 | 8 | 174 | 8 | 0.43 |
| dL | ng/mL | 8.9E2 | 1.3E3 | 1.0E3 | 1.1E3 | 5.3E2 | 2.4E2 | 2.6E2 | 7.7E2 | 3.8E3 | 1.4E3 | 465 | 8 | 174 | 8 | 0.65 |
| dM | pg/mL | 9.7E2 | 2.5E3 | 1.3E3 | 3.2E3 | 1.4E3 | 2.4E3 | 3.4E2 | 1.1E3 | 1.6E4 | 8.3E3 | 465 | 8 | 174 | 8 | 0.88 |
| dN | ug/mL | 9.3E1 | 1.3E2 | 9.8E1 | 1.4E2 | 3.5E1 | 4.5E1 | 1.6E1 | 6.9E1 | 2.4E2 | 2.0E2 | 465 | 8 | 174 | 8 | 0.75 |
| fR | ng/ml | 1.4E5 | 2.2E5 | 1.8E5 | 2.7E5 | 1.4E5 | 1.5E5 | 2.9E4 | 1.4E5 | 8.3E5 | 4.8E5 | 302 | 7 | 87 | 7 | 0.72 |

Figure 18.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 6.6E1 | 7.1E1 | 7.7E1 | 6.6E1 | 5.2E1 | 4.4E1 | 2.0E0 | 1.2E1 | 4.4E2 | 1.5E2 | 1475 | 23 | 252 | 23 | 0.44 |
| Ad | ug/mL | 3.4E-2 | 4.9E-2 | 6.8E-2 | 7.4E-2 | 8.7E-2 | 8.1E-2 | 6.8E-4 | 6.9E-3 | 5.4E-1 | 3.2E-1 | 421 | 17 | 165 | 17 | 0.57 |
| Af | ng/mL | 1.1E0 | 5.6E-1 | 1.6E1 | 4.7E1 | 6.2E1 | 1.3E2 | 1.7E-3 | 1.7E-3 | 5.3E2 | 4.8E2 | 421 | 17 | 165 | 17 | 0.48 |
| Aj | ug/mL | 1.8E0 | 3.6E-1 | 2.7E0 | 1.5E0 | 2.5E0 | 1.9E0 | 1.5E-3 | 5.5E-3 | 6.1E0 | 5.8E0 | 421 | 17 | 165 | 17 | 0.33 |
| Al | mg/mL | 8.7E-5 | 8.2E-5 | 2.5E-4 | 2.6E-4 | 4.1E-4 | 4.7E-4 | 2.5E-6 | 2.3E-6 | 2.2E-3 | 1.5E-3 | 421 | 17 | 165 | 17 | 0.49 |
| An | U/mL | 4.9E1 | 4.4E1 | 1.6E2 | 2.8E2 | 4.5E2 | 3.4E2 | 9.8E-4 | 9.0E-1 | 5.5E3 | 9.1E2 | 421 | 17 | 165 | 17 | 0.57 |
| Ao | pg/mL | 8.6E1 | 8.1E1 | 5.4E2 | 9.4E1 | 3.6E3 | 8.3E1 | 1.5E0 | 5.4E0 | 3.9E4 | 3.5E2 | 421 | 17 | 165 | 17 | 0.44 |
| Ap | ng/mL | 2.9E1 | 4.3E1 | 4.5E1 | 4.3E1 | 5.0E1 | 3.3E1 | 8.4E-5 | 3.2E0 | 3.3E2 | 1.3E2 | 421 | 17 | 165 | 17 | 0.54 |
| Ar | ng/mL | 8.5E-1 | 1.5E0 | 1.3E1 | 4.9E0 | 2.0E2 | 1.1E1 | 3.4E-3 | 1.5E-1 | 4.1E3 | 4.7E1 | 421 | 17 | 165 | 17 | 0.57 |
| As | ng/mL | 8.7E-3 | 1.0E-2 | 1.3E-2 | 1.2E-2 | 1.7E-2 | 1.4E-2 | 1.7E-3 | 1.7E-3 | 1.3E-1 | 6.1E-2 | 421 | 17 | 165 | 17 | 0.52 |
| Aw | pg/mL | 1.6E1 | 1.7E1 | 1.6E1 | 1.7E1 | 6.0E0 | 5.1E0 | 2.9E-2 | 1.1E1 | 4.8E1 | 2.7E1 | 421 | 17 | 165 | 17 | 0.57 |
| Ax | ng/mL | 2.0E0 | 6.1E0 | 1.3E1 | 1.1E1 | 5.3E1 | 1.7E1 | 1.9E-2 | 9.6E-2 | 7.7E2 | 6.0E1 | 421 | 17 | 165 | 17 | 0.59 |
| Ba | ng/mL | 5.7E1 | 1.3E2 | 4.1E2 | 6.7E2 | 1.1E3 | 1.4E3 | 2.7E-1 | 4.1E0 | 8.1E3 | 5.2E3 | 421 | 17 | 165 | 17 | 0.58 |
| Bb | ng/mL | 2.9E0 | 3.8E0 | 6.3E0 | 4.3E0 | 1.5E1 | 4.0E0 | 4.1E-3 | 4.1E-3 | 2.5E2 | 1.2E1 | 421 | 17 | 165 | 17 | 0.48 |
| Bc | ng/mL | 3.4E1 | 6.9E1 | 9.9E1 | 1.2E2 | 1.9E2 | 2.1E2 | 1.1E-1 | 7.0E0 | 1.2E3 | 8.9E2 | 421 | 17 | 165 | 17 | 0.63 |
| Bg | ng/mL | 7.4E-2 | 1.6E-1 | 4.5E0 | 7.3E-1 | 2.1E1 | 1.4E0 | 5.3E-4 | 5.3E-4 | 2.5E2 | 5.3E0 | 421 | 17 | 165 | 17 | 0.53 |
| Bn | ng/mL | 5.6E-2 | 5.6E-2 | 1.2E0 | 1.4E0 | 2.0E0 | 2.1E0 | 5.6E-2 | 5.6E-2 | 9.7E0 | 6.5E0 | 421 | 17 | 165 | 17 | 0.50 |
| Bo | ng/mL | 1.2E1 | 2.2E1 | 1.4E1 | 1.9E1 | 1.9E1 | 1.1E1 | 1.6E-2 | 1.6E-2 | 2.8E2 | 3.9E1 | 421 | 17 | 165 | 17 | 0.67 |
| Ch | uIU/mL | 1.1E0 | 9.5E-1 | 1.9E1 | 1.9E0 | 1.1E2 | 3.5E0 | 3.4E-3 | 3.4E-3 | 1.8E3 | 1.4E1 | 421 | 17 | 165 | 17 | 0.39 |
| Co | pg/mL | 3.6E1 | 3.3E1 | 1.8E2 | 4.7E1 | 9.9E2 | 4.9E1 | 1.5E-1 | 6.2E0 | 1.7E4 | 2.1E2 | 421 | 17 | 165 | 17 | 0.47 |
| Cp | ng/mL | 2.2E1 | 2.3E1 | 2.8E1 | 2.8E1 | 3.2E1 | 2.0E1 | 6.0E-1 | 2.5E0 | 3.7E2 | 8.1E1 | 421 | 17 | 165 | 17 | 0.54 |
| Cq | ng/mL | 2.8E-2 | 4.5E-2 | 1.4E-1 | 7.9E-2 | 8.8E-1 | 1.1E-1 | 8.0E-4 | 8.0E-4 | 1.7E1 | 4.0E-1 | 421 | 17 | 165 | 17 | 0.55 |
| Cs | ng/mL | 5.3E1 | 2.1E2 | 2.7E2 | 2.6E2 | 8.0E2 | 3.1E2 | 2.7E-2 | 4.4E0 | 1.1E4 | 1.2E3 | 421 | 17 | 165 | 17 | 0.58 |
| Ct | ng/mL | 8.6E-1 | 1.8E-1 | 3.5E1 | 6.8E0 | 1.0E2 | 1.6E1 | 1.1E-4 | 2.5E-2 | 6.2E2 | 4.9E1 | 421 | 17 | 165 | 17 | 0.40 |
| Cu | ng/mL | 2.3E-1 | 2.8E-1 | 4.1E-1 | 3.7E-1 | 7.8E-1 | 3.0E-1 | 9.0E-5 | 4.6E-2 | 9.2E0 | 1.1E0 | 421 | 17 | 165 | 17 | 0.56 |
| Cv | ng/mL | 4.7E0 | 8.7E0 | 2.2E1 | 3.2E1 | 6.0E1 | 5.1E1 | 1.4E-4 | 2.4E-2 | 5.3E2 | 1.7E2 | 421 | 17 | 165 | 17 | 0.59 |
| Cw | mIU/mL | 3.0E-2 | 3.6E-2 | 3.9E-2 | 3.4E-2 | 3.3E-2 | 1.8E-2 | 8.9E-4 | 3.5E-3 | 2.4E-1 | 6.4E-2 | 421 | 17 | 165 | 17 | 0.50 |
| Cx | ng/mL | 2.6E-1 | 4.6E-1 | 5.8E1 | 9.0E1 | 1.1E2 | 1.5E2 | 9.3E-5 | 9.3E-5 | 3.9E2 | 3.9E2 | 421 | 17 | 165 | 17 | 0.56 |
| Db | ug/mL | 7.5E0 | 7.3E0 | 9.1E0 | 9.5E0 | 8.7E0 | 8.9E0 | 4.5E-1 | 9.7E-1 | 1.0E2 | 3.1E1 | 421 | 17 | 165 | 17 | 0.49 |
| Dc | nmol/L | 1.8E-2 | 2.4E-2 | 5.5E-2 | 7.1E-2 | 1.3E-1 | 1.5E-1 | 5.2E-6 | 1.6E-3 | 1.6E0 | 6.3E-1 | 421 | 17 | 165 | 17 | 0.58 |
| Dd | ug/mL | 7.1E-2 | 1.4E-1 | 1.8E-1 | 1.6E-1 | 2.6E-1 | 1.7E-1 | 8.3E-5 | 3.2E-3 | 1.9E0 | 6.0E-1 | 421 | 17 | 165 | 17 | 0.56 |
| De | ng/mL | 3.4E-3 | 3.4E-3 | 7.6E-2 | 5.2E-2 | 1.4E-1 | 8.1E-2 | 3.4E-3 | 3.4E-3 | 9.2E-1 | 2.4E-1 | 421 | 17 | 165 | 17 | 0.48 |
| Dg | ng/mL | 2.8E1 | 4.6E1 | 4.1E1 | 5.5E1 | 3.9E1 | 4.3E1 | 1.0E-1 | 2.2E0 | 1.9E2 | 1.9E2 | 421 | 17 | 165 | 17 | 0.61 |
| Di | pg/mL | 1.9E0 | 1.5E0 | 2.2E0 | 2.1E0 | 2.0E0 | 2.3E0 | 1.8E-1 | 1.8E-1 | 1.3E1 | 9.2E0 | 421 | 17 | 165 | 17 | 0.47 |
| Dk | uIU/mL | 1.6E-2 | 1.5E-2 | 9.0E-2 | 3.8E-2 | 5.3E-1 | 5.6E-2 | 1.1E-4 | 1.1E-4 | 8.9E0 | 2.1E-1 | 421 | 17 | 165 | 17 | 0.47 |
| Dl | ng/mL | 2.1E2 | 2.7E2 | 3.0E2 | 3.3E2 | 2.8E2 | 3.1E2 | 1.7E0 | 1.7E1 | 1.5E3 | 1.3E3 | 421 | 17 | 165 | 17 | 0.53 |
| Dp | ng/ml | 2.4E0 | 2.7E0 | 5.2E0 | 5.9E0 | 7.5E0 | 7.7E0 | 3.7E-3 | 3.7E-3 | 4.6E1 | 2.6E1 | 249 | 16 | 162 | 16 | 0.49 |
| Ef | ng/ml | 1.3E-1 | 9.4E-2 | 8.3E-1 | 6.8E-1 | 1.8E0 | 1.5E0 | 5.7E-4 | 4.1E-3 | 1.0E1 | 5.2E0 | 304 | 17 | 164 | 17 | 0.48 |
| Wm | % | 5.9E-1 | 1.6E0 | 2.0E1 | 4.5E1 | 1.5E2 | 1.8E2 | 5.4E-2 | 8.5E-2 | 2.4E3 | 7.7E2 | 341 | 18 | 184 | 18 | 0.55 |
| Ed | pg/ml | 5.2E-1 | 2.4E1 | 5.7E1 | 4.6E1 | 4.6E2 | 4.8E1 | 5.2E-1 | 5.2E-1 | 7.3E3 | 1.3E2 | 249 | 16 | 161 | 16 | 0.65 |
| Tj | pg/mL | 3.7E-1 | 3.7E-1 | 5.9E1 | 6.3E0 | 3.1E2 | 1.5E1 | 3.6E-1 | 3.7E-1 | 3.5E3 | 5.6E1 | 304 | 16 | 167 | 16 | 0.45 |
| Po | pg/ml | 4.8E-1 | 8.9E-1 | 8.6E0 | 5.4E0 | 2.5E1 | 7.3E0 | 8.0E-3 | 2.6E-2 | 2.7E2 | 2.1E1 | 690 | 28 | 279 | 28 | 0.52 |
| Em | ng/ml | 2.9E-3 | 2.9E-3 | 5.5E-2 | 8.4E-2 | 1.1E-1 | 1.9E-1 | 1.9E-16 | 2.9E-3 | 6.0E-1 | 5.0E-1 | 190 | 7 | 87 | 7 | 0.50 |
| Et | ng/ml | 1.3E3 | 2.1E3 | 1.5E3 | 2.1E3 | 1.1E3 | 1.2E3 | 7.5E1 | 1.4E2 | 5.0E3 | 4.1E3 | 689 | 28 | 279 | 28 | 0.63 |
| Fa | ng/ml | 3.9E1 | 8.8E1 | 1.2E2 | 8.2E1 | 5.6E2 | 6.1E1 | 3.4E-2 | 1.6E0 | 8.0E3 | 2.2E2 | 243 | 17 | 159 | 17 | 0.64 |
| Ez | ng/ml | 3.8E0 | 3.4E0 | 1.8E1 | 1.4E1 | 5.5E1 | 2.3E1 | 1.3E-2 | 1.3E-2 | 7.1E2 | 8.9E1 | 249 | 16 | 162 | 16 | 0.51 |
| Fb | ng/ml | 2.5E1 | 2.6E1 | 2.2E1 | 2.3E1 | 1.2E1 | 1.3E1 | 6.6E-1 | 8.1E-1 | 5.7E1 | 4.3E1 | 244 | 17 | 159 | 17 | 0.52 |
| Ex | ng/ml | 7.4E-2 | 9.5E-2 | 2.4E-1 | 1.5E-1 | 7.2E-1 | 1.5E-1 | 3.5E-5 | 1.7E-4 | 8.9E0 | 5.0E-1 | 224 | 13 | 113 | 13 | 0.57 |
| Fn | ng/ml | 2.1E-1 | 2.1E-1 | 6.1E0 | 2.4E0 | 2.7E1 | 4.0E0 | 1.1E-14 | 1.1E-14 | 4.2E2 | 1.6E1 | 249 | 16 | 162 | 16 | 0.46 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fp | ng/ml | 1.2E1 | 1.9E1 | 2.3E1 | 2.8E1 | 2.8E1 | 3.0E1 | 6.0E-3 | 1.3E-1 | 1.4E2 | 1.3E2 | 720 | 28 | 280 | 28 | 0.53 |
| Fr | ng/ml | 3.2E4 | 5.2E4 | 1.1E5 | 1.7E5 | 1.7E5 | 2.4E5 | 1.9E2 | 3.2E2 | 9.0E5 | 7.4E5 | 823 | 28 | 284 | 28 | 0.60 |
| Fw | pg/ml | 1.1E0 | 2.5E0 | 6.2E1 | 1.3E1 | 5.0E2 | 1.8E1 | 1.1E-14 | 1.2E-1 | 6.9E3 | 5.3E1 | 304 | 17 | 165 | 17 | 0.55 |
| Fy | ng/ml | 3.5E1 | 2.3E1 | 5.5E1 | 3.9E1 | 5.6E1 | 5.6E1 | 1.2E-1 | 1.5E0 | 3.3E2 | 2.3E2 | 246 | 15 | 161 | 15 | 0.41 |
| Gl | pg/ml | 7.4E3 | 5.7E3 | 1.1E4 | 1.0E4 | 9.4E3 | 9.0E3 | 9.1E1 | 2.5E2 | 3.4E4 | 2.8E4 | 295 | 17 | 164 | 17 | 0.49 |
| Gp | U/ml | 1.7E0 | 9.8E-1 | 4.1E0 | 2.8E0 | 6.6E0 | 5.1E0 | 1.3E-3 | 1.5E-2 | 6.7E1 | 2.0E1 | 306 | 17 | 165 | 17 | 0.41 |
| Gz | ug/ml | 1.4E0 | 9.2E-1 | 9.2E0 | 5.6E0 | 3.9E1 | 6.6E0 | 2.9E-16 | 4.6E-1 | 4.8E2 | 1.9E1 | 165 | 13 | 106 | 13 | 0.53 |
| Ha | ng/ml | 2.6E0 | 2.5E0 | 9.9E0 | 5.3E0 | 2.1E1 | 8.2E0 | 1.7E-2 | 1.7E-2 | 1.3E2 | 3.3E1 | 247 | 16 | 161 | 16 | 0.46 |
| Nm | pg/ml | 1.4E4 | 1.4E4 | 3.0E4 | 2.7E4 | 8.2E4 | 3.2E4 | 1.0E-9 | 1.0E-9 | 1.6E6 | 1.2E5 | 693 | 28 | 281 | 28 | 0.52 |
| Nn | pg/ml | 1.5E2 | 1.5E2 | 1.9E3 | 6.5E2 | 8.6E3 | 1.0E3 | 1.0E-9 | 1.0E-9 | 1.0E5 | 4.0E3 | 693 | 28 | 281 | 28 | 0.53 |
| No | pg/ml | 1.4E1 | 1.3E1 | 3.5E1 | 2.7E1 | 1.2E2 | 3.5E1 | 1.0E-9 | 1.0E-9 | 2.5E3 | 1.4E2 | 693 | 28 | 281 | 28 | 0.49 |
| Nq | pg/ml | 1.9E0 | 2.6E0 | 1.7E1 | 9.6E0 | 7.0E1 | 1.6E1 | 1.0E-9 | 1.0E-9 | 1.2E3 | 7.3E1 | 693 | 28 | 281 | 28 | 0.53 |
| Nr | pg/ml | 6.5E-1 | 1.3E0 | 3.1E1 | 1.0E1 | 2.0E2 | 2.7E1 | 1.0E-9 | 1.0E-9 | 4.1E3 | 1.4E2 | 693 | 28 | 281 | 28 | 0.51 |
| Ns | pg/ml | 1.0E-9 | 1.0E-9 | 9.7E0 | 8.8E0 | 5.6E1 | 4.5E1 | 1.0E-9 | 1.0E-9 | 1.1E3 | 2.4E2 | 693 | 28 | 281 | 28 | 0.50 |
| Nt | pg/ml | 1.0E2 | 1.0E2 | 1.3E2 | 1.3E2 | 1.1E2 | 7.1E1 | 1.0E-9 | 2.3E1 | 1.5E3 | 3.5E2 | 693 | 28 | 281 | 28 | 0.54 |
| Nu | pg/ml | 1.9E1 | 2.0E1 | 5.4E1 | 6.2E1 | 9.4E1 | 7.8E1 | 1.0E-9 | 1.0E-9 | 8.9E2 | 2.6E2 | 693 | 28 | 281 | 28 | 0.52 |
| Lu | pg/ml | 1.0E4 | 8.5E3 | 1.6E4 | 1.0E4 | 4.6E4 | 1.1E4 | 3.5E2 | 2.1E3 | 7.5E5 | 5.5E4 | 695 | 28 | 281 | 28 | 0.42 |
| Lv | pg/ml | 1.0E-9 | 1.0E-9 | 1.1E1 | 3.3E1 | 2.1E1 | 6.3E1 | 1.0E-9 | 1.0E-9 | 2.4E2 | 2.6E2 | 695 | 28 | 281 | 28 | 0.56 |
| Lw | pg/ml | 1.0E-9 | 1.0E-9 | 3.7E-1 | 3.7E-1 | 4.0E0 | 1.9E0 | 1.0E-9 | 1.0E-9 | 8.0E1 | 9.9E0 | 695 | 28 | 281 | 28 | 0.53 |
| Lx | pg/ml | 1.0E-9 | 2.8E1 | 1.3E2 | 1.8E2 | 4.2E2 | 3.1E2 | 1.0E-9 | 1.0E-9 | 6.2E3 | 1.4E3 | 695 | 28 | 281 | 28 | 0.59 |
| Ly | pg/ml | 1.0E-9 | 9.9E0 | 1.0E1 | 1.5E1 | 2.0E1 | 1.7E1 | 1.0E-9 | 1.0E-9 | 1.6E2 | 7.0E1 | 695 | 28 | 281 | 28 | 0.63 |
| Lz | pg/ml | 1.0E-9 | 1.0E-9 | 4.9E0 | 1.0E-9 | 3.4E1 | 0.0E0 | 1.0E-9 | 1.0E-9 | 6.0E2 | 1.0E-9 | 695 | 28 | 281 | 28 | 0.46 |
| Ma | pg/ml | 2.7E2 | 3.2E2 | 1.4E3 | 1.8E3 | 3.8E3 | 3.7E3 | 1.0E-9 | 1.0E-9 | 6.5E4 | 1.7E4 | 695 | 28 | 281 | 28 | 0.52 |
| Mb | pg/ml | 2.5E1 | 2.6E1 | 3.1E1 | 3.1E1 | 1.6E1 | 1.5E1 | 5.4E0 | 1.4E1 | 2.1E2 | 5.5E1 | 695 | 28 | 281 | 28 | 0.47 |
| Mc | pg/ml | 1.0E-9 | 1.0E-9 | 3.6E-2 | 8.9E-2 | 5.3E-1 | 4.7E-1 | 1.0E-9 | 1.0E-9 | 1.3E1 | 2.5E0 | 695 | 28 | 281 | 28 | 0.51 |
| Md | pg/ml | 1.0E-9 | 1.0E-9 | 5.0E-1 | 1.7E-2 | 4.0E0 | 9.2E-2 | 1.0E-9 | 1.0E-9 | 6.8E1 | 4.9E-1 | 695 | 28 | 281 | 28 | 0.48 |
| Me | pg/ml | 3.2E1 | 2.9E1 | 3.1E1 | 3.0E1 | 2.0E1 | 1.2E1 | 1.0E-9 | 2.4E-1 | 3.2E2 | 5.6E1 | 695 | 28 | 281 | 28 | 0.47 |
| Mf | pg/ml | 1.0E-9 | 1.0E-9 | 5.0E-1 | 1.0E-1 | 2.9E0 | 4.2E-1 | 1.0E-9 | 1.0E-9 | 5.6E1 | 2.2E0 | 695 | 28 | 281 | 28 | 0.46 |
| Mg | pg/ml | 1.7E0 | 8.3E-1 | 7.3E0 | 5.7E0 | 1.2E1 | 7.4E0 | 1.0E-9 | 1.0E-9 | 9.2E1 | 2.6E1 | 695 | 28 | 281 | 28 | 0.51 |
| Mh | pg/ml | 1.0E-9 | 1.0E-9 | 1.5E0 | 3.7E-1 | 1.1E1 | 1.5E0 | 1.0E-9 | 1.0E-9 | 1.6E2 | 7.8E0 | 695 | 28 | 281 | 28 | 0.49 |
| Mi | pg/ml | 1.0E-9 | 1.0E-9 | 4.9E-1 | 1.1E0 | 5.8E0 | 5.9E0 | 1.0E-9 | 1.0E-9 | 1.2E2 | 3.1E1 | 695 | 28 | 281 | 28 | 0.51 |
| Mj | pg/ml | 1.0E-9 | 1.0E-9 | 4.8E0 | 2.2E0 | 2.7E1 | 5.8E0 | 1.0E-9 | 1.0E-9 | 4.6E2 | 2.2E1 | 695 | 28 | 281 | 28 | 0.51 |
| Mk | pg/ml | 9.1E-1 | 1.0E-9 | 1.5E1 | 4.3E0 | 9.7E1 | 8.8E0 | 1.0E-9 | 1.0E-9 | 1.7E3 | 4.0E1 | 695 | 28 | 281 | 28 | 0.44 |
| Ml | pg/ml | 1.0E-9 | 1.0E-9 | 5.8E0 | 3.3E-1 | 8.2E1 | 1.5E0 | 1.0E-9 | 1.0E-9 | 2.1E3 | 8.0E0 | 695 | 28 | 281 | 28 | 0.44 |
| Mm | pg/ml | 5.4E2 | 7.3E2 | 9.2E2 | 1.4E3 | 1.1E3 | 2.1E3 | 1.0E-9 | 8.8E-2 | 7.3E3 | 9.9E3 | 695 | 28 | 281 | 28 | 0.55 |
| Mn | pg/ml | 5.3E0 | 5.8E0 | 1.0E1 | 9.8E0 | 2.4E1 | 1.1E1 | 1.0E-9 | 1.0E-9 | 3.5E2 | 5.2E1 | 695 | 28 | 281 | 28 | 0.56 |
| Mp | pg/ml | 1.0E-9 | 1.0E-9 | 8.4E0 | 1.8E1 | 2.9E1 | 3.6E1 | 1.0E-9 | 1.0E-9 | 6.3E2 | 1.5E2 | 694 | 28 | 281 | 28 | 0.55 |
| Mq | pg/ml | 1.0E-9 | 1.0E-9 | 2.8E0 | 3.4E0 | 1.6E1 | 1.3E1 | 1.0E-9 | 1.0E-9 | 2.1E2 | 6.2E1 | 694 | 28 | 281 | 28 | 0.52 |
| Mr | pg/ml | 1.0E-9 | 1.0E-9 | 1.6E1 | 1.2E1 | 7.9E1 | 5.5E1 | 1.0E-9 | 1.0E-9 | 1.4E3 | 2.9E2 | 694 | 28 | 281 | 28 | 0.48 |
| Ms | pg/ml | 4.1E2 | 2.9E2 | 5.6E2 | 4.0E2 | 6.4E2 | 3.8E2 | 1.0E-9 | 1.0E-9 | 5.6E3 | 1.5E3 | 694 | 28 | 281 | 28 | 0.44 |
| Mt | pg/ml | 1.0E-9 | 7.9E-1 | 6.8E0 | 4.3E0 | 4.6E1 | 1.3E1 | 1.0E-9 | 1.0E-9 | 8.7E2 | 7.0E1 | 694 | 28 | 281 | 28 | 0.60 |
| Mu | pg/ml | 1.0E-9 | 1.0E-9 | 1.3E0 | 6.2E-1 | 1.2E1 | 1.6E0 | 1.0E-9 | 1.0E-9 | 2.3E2 | 6.2E0 | 694 | 28 | 281 | 28 | 0.54 |
| Mv | pg/ml | 1.0E-9 | 1.0E-9 | 6.8E1 | 5.1E1 | 3.3E2 | 1.2E2 | 1.0E-9 | 1.0E-9 | 4.5E3 | 5.3E2 | 694 | 28 | 281 | 28 | 0.53 |
| Mw | pg/ml | 3.2E1 | 3.4E1 | 4.3E2 | 2.0E2 | 2.9E3 | 3.5E2 | 1.0E-9 | 1.0E-9 | 6.2E4 | 1.4E3 | 694 | 28 | 281 | 28 | 0.51 |
| Mx | pg/ml | 1.0E-9 | 1.0E-9 | 2.3E-1 | 2.8E-1 | 1.4E0 | 7.7E-1 | 1.0E-9 | 1.0E-9 | 3.2E1 | 3.4E0 | 694 | 28 | 281 | 28 | 0.53 |
| My | pg/ml | 1.0E-9 | 1.0E-9 | 4.3E2 | 1.5E2 | 2.9E3 | 3.5E2 | 1.0E-9 | 1.0E-9 | 3.9E4 | 1.7E3 | 694 | 28 | 281 | 28 | 0.53 |
| Mz | pg/ml | 1.0E1 | 1.3E1 | 2.4E1 | 3.7E1 | 6.8E1 | 6.4E1 | 1.0E-9 | 1.0E-9 | 1.2E3 | 2.7E2 | 694 | 28 | 281 | 28 | 0.56 |
| Na | pg/ml | 1.0E-9 | 1.0E-9 | 7.7E-1 | 5.7E-1 | 2.9E0 | 1.7E0 | 1.0E-9 | 1.0E-9 | 3.6E1 | 7.2E0 | 694 | 28 | 281 | 28 | 0.50 |
| Nb | pg/ml | 1.9E0 | 3.0E0 | 4.0E0 | 3.1E0 | 1.3E1 | 2.6E0 | 1.0E-9 | 1.0E-9 | 2.3E2 | 1.0E1 | 694 | 28 | 281 | 28 | 0.56 |
| Nc | pg/ml | 3.8E2 | 1.2E2 | 6.2E2 | 3.6E2 | 7.7E2 | 5.4E2 | 1.0E-9 | 1.0E-9 | 9.2E3 | 2.1E3 | 694 | 28 | 281 | 28 | 0.39 |
| Nd | pg/ml | 2.9E1 | 6.4E0 | 2.7E1 | 1.4E1 | 5.0E1 | 1.6E1 | 1.0E-9 | 1.0E-9 | 1.2E3 | 5.2E1 | 694 | 28 | 281 | 28 | 0.33 |
| Ne | pg/ml | 4.7E2 | 2.0E2 | 6.0E2 | 3.2E2 | 5.9E2 | 3.7E2 | 1.0E-9 | 1.0E-9 | 7.0E3 | 1.5E3 | 694 | 28 | 281 | 28 | 0.34 |
| Nf | pg/ml | 1.0E-9 | 1.0E-9 | 3.2E0 | 5.5E-1 | 1.0E1 | 1.6E0 | 1.0E-9 | 1.0E-9 | 1.6E2 | 6.9E0 | 694 | 28 | 281 | 28 | 0.44 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|--------|--------|--------|------|
| | | NonDis | Dis | NonDis | Dis | NonDis | Dis | NonDis | Dis | NonDis | Dis | NonDis | Dis | NonDis | Dis | |
| Ng | pg/ml | 2.1E1 | 2.5E0 | 1.3E2 | 9.8E1 | 2.6E2 | 1.8E2 | 1.0E-9 | 1.0E-9 | 2.3E3 | 6.4E2 | 694 | 28 | 281 | 28 | 0.44 |
| Nh | pg/ml | 7.1E1 | 3.9E1 | 9.4E1 | 5.8E1 | 8.6E1 | 5.7E1 | 1.0E-9 | 3.1E0 | 5.6E2 | 2.2E2 | 694 | 28 | 281 | 28 | 0.36 |
| Ni | pg/ml | 1.0E-9 | 1.7E1 | 7.6E1 | 1.0E2 | 1.2E2 | 1.5E2 | 1.0E-9 | 1.0E-9 | 7.1E2 | 4.8E2 | 694 | 28 | 281 | 28 | 0.54 |
| Nj | pg/ml | 8.2E0 | 2.8E0 | 1.2E1 | 6.8E0 | 1.2E1 | 7.8E0 | 1.0E-9 | 1.0E-9 | 8.3E1 | 2.8E1 | 694 | 28 | 281 | 28 | 0.36 |
| Nk | pg/ml | 1.9E1 | 1.4E1 | 3.4E1 | 2.3E1 | 4.0E1 | 2.5E1 | 1.0E-9 | 1.0E-9 | 2.0E2 | 8.7E1 | 694 | 28 | 281 | 28 | 0.45 |
| Nl | pg/ml | 4.9E1 | 2.1E1 | 6.5E1 | 3.7E1 | 7.2E1 | 4.4E1 | 1.0E-9 | 1.0E-9 | 1.1E3 | 1.4E2 | 694 | 28 | 281 | 28 | 0.35 |
| Tz | pg/ml | 5.2E3 | 4.9E3 | 1.3E4 | 1.9E4 | 6.4E4 | 4.2E4 | 1.0E-9 | 7.5E2 | 1.0E6 | 1.7E5 | 251 | 16 | 160 | 16 | 0.49 |
| Ua | pg/ml | 3.8E3 | 2.8E3 | 2.3E4 | 6.3E3 | 1.4E5 | 7.6E3 | 1.0E-9 | 2.7E2 | 2.1E6 | 2.4E4 | 251 | 16 | 160 | 16 | 0.43 |
| Ub | pg/ml | 5.7E2 | 2.9E2 | 8.6E2 | 3.8E2 | 1.0E3 | 3.7E2 | 1.0E-9 | 1.5E1 | 9.8E3 | 1.4E3 | 251 | 16 | 160 | 16 | 0.32 |
| Ue | pg/ml | 3.0E1 | 2.0E1 | 3.7E1 | 2.3E1 | 3.2E1 | 1.7E1 | 9.8E-2 | 2.9E0 | 3.5E2 | 7.4E1 | 251 | 16 | 160 | 16 | 0.34 |
| Uc | pg/ml | 8.6E2 | 8.6E2 | 1.6E3 | 2.0E3 | 2.7E3 | 2.8E3 | 1.0E-9 | 2.1E1 | 2.9E4 | 9.4E3 | 251 | 16 | 160 | 16 | 0.52 |
| Ud | pg/ml | 1.0E-9 | 1.0E-9 | 1.7E0 | 1.0E-9 | 2.5E1 | 0.0E0 | 1.0E-9 | 1.0E-9 | 3.9E2 | 1.0E-9 | 251 | 16 | 160 | 16 | 0.49 |
| Hq | pg/ml | 1.1E0 | 1.3E0 | 1.3E2 | 1.6E1 | 1.8E3 | 6.5E1 | 1.0E-9 | 1.0E-9 | 3.4E4 | 3.4E2 | 691 | 28 | 280 | 28 | 0.51 |
| Hr | pg/ml | 1.3E2 | 1.0E2 | 8.2E2 | 7.7E2 | 1.6E3 | 1.2E3 | 1.0E-9 | 2.3E1 | 1.4E4 | 4.6E3 | 691 | 28 | 280 | 28 | 0.49 |
| Hu | pg/ml | 7.1E0 | 3.1E0 | 3.0E3 | 3.8E2 | 2.9E4 | 1.3E3 | 1.0E-9 | 1.0E-9 | 6.3E5 | 5.8E3 | 691 | 28 | 280 | 28 | 0.47 |
| Hv | pg/ml | 1.4E0 | 2.5E0 | 3.3E0 | 2.3E0 | 1.2E1 | 1.6E0 | 1.0E-9 | 1.0E-9 | 2.5E2 | 6.9E0 | 691 | 28 | 280 | 28 | 0.60 |
| Hw | pg/ml | 7.1E0 | 5.0E0 | 2.0E1 | 7.4E0 | 8.0E1 | 7.3E0 | 1.0E-9 | 1.0E-9 | 1.7E3 | 2.3E1 | 691 | 28 | 280 | 28 | 0.40 |
| Hx | pg/ml | 8.8E0 | 1.4E1 | 4.4E1 | 2.8E1 | 3.6E2 | 4.3E1 | 1.0E-9 | 1.0E-9 | 9.3E3 | 2.2E2 | 691 | 28 | 280 | 28 | 0.55 |
| Ib | ng/ml | 6.2E-2 | 8.4E-3 | 1.4E0 | 6.8E-2 | 5.0E0 | 1.7E-1 | 1.0E-9 | 1.0E-9 | 3.6E1 | 6.8E-1 | 241 | 16 | 159 | 16 | 0.27 |
| Ic | U/ml | 2.2E2 | 2.2E2 | 7.3E2 | 5.5E3 | 6.1E3 | 1.7E4 | 2.4E0 | 2.2E1 | 9.3E4 | 6.5E4 | 241 | 16 | 159 | 16 | 0.55 |
| Id | U/ml | 6.4E-1 | 9.1E-1 | 1.2E0 | 2.2E0 | 2.0E0 | 3.1E0 | 1.0E-9 | 2.7E-1 | 2.3E1 | 1.2E1 | 241 | 16 | 159 | 16 | 0.60 |
| Tt | pg/ml | 1.6E2 | 1.5E2 | 1.7E2 | 1.6E2 | 5.1E1 | 4.0E1 | 4.3E1 | 1.1E2 | 3.6E2 | 2.3E2 | 231 | 14 | 153 | 14 | 0.42 |
| To | pg/ml | 1.6E0 | 1.9E0 | 1.9E0 | 2.3E0 | 2.4E0 | 1.9E0 | 1.0E-9 | 1.0E-9 | 2.3E1 | 6.0E0 | 242 | 14 | 157 | 14 | 0.61 |
| Tr | pg/ml | 2.8E0 | 2.7E0 | 5.8E0 | 5.7E0 | 2.0E1 | 8.4E0 | 1.0E-9 | 3.3E-1 | 3.1E2 | 3.2E1 | 238 | 14 | 156 | 14 | 0.50 |
| Tn | pg/ml | 2.6E1 | 3.3E1 | 8.0E1 | 6.8E1 | 2.2E2 | 8.4E1 | 2.4E0 | 8.8E0 | 1.8E3 | 3.0E2 | 242 | 14 | 157 | 14 | 0.59 |
| Tv | ng/ml | 1.2E1 | 3.7E0 | 1.9E1 | 4.2E1 | 3.7E1 | 8.8E1 | 1.0E-9 | 1.0E-9 | 4.9E2 | 3.2E2 | 242 | 14 | 157 | 14 | 0.43 |
| Ih | ng/ml | 6.9E1 | 4.4E1 | 2.1E2 | 1.6E2 | 3.7E2 | 2.5E2 | 1.0E-9 | 1.0E-9 | 2.6E3 | 9.0E2 | 694 | 28 | 280 | 28 | 0.46 |
| Ii | ng/ml | 9.0E1 | 6.3E1 | 2.6E2 | 1.5E2 | 7.4E2 | 2.1E2 | 7.3E-1 | 9.1E0 | 1.0E4 | 8.1E2 | 694 | 28 | 280 | 28 | 0.46 |
| Ij | ng/ml | 7.3E1 | 7.7E1 | 1.8E2 | 8.7E1 | 6.4E2 | 6.6E1 | 2.1E0 | 1.0E-9 | 6.4E3 | 3.0E2 | 686 | 28 | 279 | 28 | 0.48 |
| Ik | ng/ml | 1.4E1 | 4.9E1 | 9.8E2 | 2.3E2 | 9.3E3 | 3.9E2 | 5.9E-1 | 2.1E0 | 1.2E5 | 1.5E3 | 690 | 28 | 279 | 28 | 0.54 |
| Il | ng/ml | 3.3E2 | 3.3E2 | 1.2E3 | 6.4E2 | 2.7E3 | 8.1E2 | 1.0E-9 | 1.0E-9 | 1.2E4 | 3.2E3 | 680 | 28 | 279 | 28 | 0.48 |
| Im | ng/ml | 1.9E2 | 2.1E2 | 3.4E2 | 3.5E2 | 5.0E2 | 3.2E2 | 1.3E1 | 2.5E1 | 6.0E3 | 1.1E3 | 689 | 28 | 279 | 28 | 0.53 |
| In | ng/ml | 3.9E0 | 1.6E0 | 2.5E1 | 6.1E0 | 1.7E2 | 1.6E1 | 1.0E-9 | 1.0E-9 | 3.9E3 | 8.4E1 | 694 | 28 | 280 | 28 | 0.37 |
| Hb | ng/ml | 2.4E1 | 2.0E1 | 3.2E1 | 3.1E1 | 2.9E1 | 3.4E1 | 4.8E-1 | 2.1E0 | 1.5E2 | 1.2E2 | 248 | 17 | 160 | 17 | 0.45 |
| Hc | pg/ml | 6.7E2 | 4.6E2 | 3.7E3 | 9.8E2 | 1.3E4 | 1.6E3 | 1.0E-9 | 2.2E2 | 1.0E5 | 6.8E3 | 248 | 17 | 160 | 17 | 0.44 |
| Hf | ng/ml | 1.5E2 | 1.3E2 | 3.8E2 | 2.7E2 | 5.3E2 | 3.2E2 | 1.0E-9 | 1.0E-9 | 2.5E3 | 8.5E2 | 248 | 17 | 160 | 17 | 0.48 |
| Io | ng/ml | 7.5E3 | 8.8E3 | 2.4E4 | 1.5E4 | 1.6E5 | 2.2E4 | 1.0E-9 | 9.0E2 | 4.0E6 | 1.1E5 | 687 | 27 | 280 | 27 | 0.52 |
| Ip | ng/ml | 8.7E0 | 1.3E1 | 1.9E1 | 3.0E1 | 2.4E1 | 4.1E1 | 1.0E-9 | 1.6E-2 | 2.6E2 | 1.6E2 | 687 | 27 | 280 | 27 | 0.55 |
| Iq | ug/ml | 9.5E-2 | 8.1E-2 | 4.0E1 | 1.8E-1 | 7.3E2 | 2.8E-1 | 1.0E-9 | 1.0E-9 | 1.4E4 | 1.4E0 | 687 | 27 | 280 | 27 | 0.45 |
| Ir | ug/ml | 3.3E-1 | 4.8E-1 | 3.8E0 | 1.5E0 | 2.8E1 | 3.1E0 | 1.0E-9 | 1.0E-9 | 5.1E2 | 1.6E1 | 686 | 27 | 280 | 27 | 0.59 |
| Is | ng/ml | 1.4E0 | 3.7E0 | 5.7E0 | 9.9E0 | 2.3E1 | 2.1E1 | 1.0E-9 | 2.7E-1 | 5.5E2 | 1.1E2 | 687 | 27 | 280 | 27 | 0.67 |
| It | ng/ml | 2.0E0 | 1.7E0 | 2.6E1 | 4.7E0 | 1.5E2 | 9.1E0 | 1.0E-9 | 1.0E-9 | 2.8E3 | 4.7E1 | 687 | 27 | 280 | 27 | 0.53 |
| Iu | ng/ml | 2.1E2 | 1.9E2 | 1.4E3 | 1.7E3 | 4.3E3 | 4.7E3 | 1.0E-9 | 1.0E-9 | 2.9E4 | 2.4E4 | 687 | 27 | 280 | 27 | 0.53 |
| Iv | ng/ml | 1.2E1 | 2.4E1 | 6.1E1 | 3.7E1 | 6.1E2 | 3.4E1 | 1.0E-9 | 1.0E-9 | 1.6E4 | 1.0E2 | 686 | 27 | 280 | 27 | 0.59 |
| Iz | ng/ml | 1.4E2 | 7.5E1 | 6.8E2 | 2.8E2 | 4.0E3 | 4.2E2 | 1.5E0 | 1.3E1 | 6.2E4 | 1.5E3 | 248 | 17 | 160 | 17 | 0.41 |
| Rc | pg/ml | 5.5E3 | 6.1E3 | 7.3E3 | 7.8E3 | 6.0E3 | 5.4E3 | 1.9E2 | 3.0E2 | 3.9E4 | 1.6E4 | 248 | 16 | 160 | 16 | 0.54 |
| Rb | pg/ml | 7.9E-1 | 8.6E-1 | 2.6E0 | 2.1E0 | 4.4E0 | 2.8E0 | 1.0E-9 | 1.0E-9 | 4.3E1 | 9.1E0 | 248 | 16 | 160 | 16 | 0.52 |
| Pz | ng/ml | 3.3E3 | 7.8E3 | 6.8E3 | 4.2E4 | 1.8E4 | 1.9E5 | 1.3E1 | 9.5E1 | 2.8E5 | 1.0E6 | 687 | 28 | 278 | 28 | 0.57 |
| Qa | ng/ml | 3.1E3 | 6.0E3 | 5.8E3 | 7.8E3 | 7.1E3 | 6.7E3 | 1.5E2 | 2.9E2 | 5.2E4 | 2.5E4 | 687 | 28 | 278 | 28 | 0.61 |
| Qb | ng/ml | 9.1E1 | 1.2E2 | 2.1E2 | 1.8E2 | 5.2E2 | 1.9E2 | 7.9E-1 | 2.4E0 | 8.3E3 | 7.2E2 | 687 | 28 | 278 | 28 | 0.53 |
| Qc | ng/ml | 2.1E2 | 1.9E2 | 4.4E2 | 2.7E2 | 7.6E2 | 2.7E2 | 1.0E-9 | 2.0E0 | 1.1E4 | 1.2E3 | 687 | 28 | 278 | 28 | 0.45 |
| Qd | ng/ml | 8.7E3 | 9.1E3 | 1.9E4 | 2.5E4 | 8.2E4 | 4.0E4 | 1.5E2 | 1.1E3 | 2.0E6 | 1.9E5 | 687 | 28 | 278 | 28 | 0.56 |
| Qe | ng/ml | 8.0E2 | 1.5E3 | 1.7E3 | 2.2E3 | 4.2E3 | 2.4E3 | 1.0E-9 | 5.5E1 | 9.7E4 | 9.3E3 | 687 | 28 | 278 | 28 | 0.60 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Jd | ng/ml | 9.2E-1 | 8.0E-1 | 6.7E0 | 1.7E0 | 4.4E1 | 2.3E0 | 1.0E-9 | 1.0E-9 | 6.5E2 | 8.0E0 | 249 | 16 | 162 | 16 | 0.48 |
| Jc | ng/ml | 1.0E-9 | 2.2E-1 | 2.3E0 | 8.4E-1 | 8.0E0 | 1.2E0 | 1.0E-9 | 1.0E-9 | 8.8E1 | 3.6E0 | 249 | 16 | 162 | 16 | 0.49 |
| Jf | ng/ml | 1.0E-9 | 1.6E-1 | 1.1E0 | 9.2E-1 | 2.3E0 | 1.3E0 | 1.0E-9 | 1.0E-9 | 1.2E1 | 3.9E0 | 249 | 16 | 162 | 16 | 0.56 |
| Jg | ng/ml | 4.4E2 | 7.3E2 | 7.3E2 | 9.2E2 | 9.6E2 | 7.3E2 | 1.0E-9 | 2.1E1 | 1.0E4 | 2.5E3 | 691 | 28 | 280 | 28 | 0.60 |
| Jh | ng/ml | 2.9E0 | 3.2E0 | 2.4E1 | 1.3E1 | 1.1E2 | 1.9E1 | 1.0E-9 | 1.0E-9 | 1.6E3 | 6.8E1 | 691 | 28 | 280 | 28 | 0.54 |
| Ji | ng/ml | 5.0E1 | 7.6E1 | 7.0E1 | 1.1E2 | 6.9E1 | 9.7E1 | 1.0E-9 | 8.5E0 | 5.3E2 | 3.8E2 | 691 | 28 | 280 | 28 | 0.65 |
| Sr | pg/mL | 3.4E2 | 3.8E2 | 8.1E2 | 1.2E3 | 1.3E3 | 1.8E3 | 1.0E-9 | 1.0E-9 | 9.8E3 | 5.5E3 | 239 | 16 | 157 | 16 | 0.52 |
| Ss | pg/mL | 9.2E4 | 9.2E4 | 1.5E5 | 1.2E5 | 1.9E5 | 1.0E5 | 2.7E3 | 9.6E3 | 1.8E6 | 3.5E5 | 239 | 16 | 157 | 16 | 0.50 |
| St | pg/mL | 2.2E7 | 3.8E7 | 4.9E7 | 5.0E7 | 9.0E7 | 4.7E7 | 1.0E-9 | 1.6E6 | 1.2E9 | 1.3E8 | 244 | 15 | 158 | 15 | 0.54 |
| Ra | pg/ml | 1.0E-9 | 1.0E-9 | 6.4E-1 | 3.2E-1 | 1.3E0 | 7.5E-1 | 1.0E-9 | 1.0E-9 | 7.3E0 | 3.0E0 | 248 | 16 | 160 | 16 | 0.49 |
| Qz | pg/ml | 1.1E1 | 1.0E-9 | 6.1E1 | 4.0E1 | 1.0E2 | 8.5E1 | 1.0E-9 | 1.0E-9 | 7.1E2 | 3.2E2 | 248 | 16 | 160 | 16 | 0.38 |
| Qy | pg/ml | 4.3E-1 | 5.8E-1 | 1.0E1 | 1.5E0 | 5.7E1 | 2.9E0 | 1.0E-9 | 1.0E-9 | 6.5E2 | 1.2E1 | 248 | 16 | 160 | 16 | 0.54 |
| Qx | pg/ml | 1.0E-9 | 1.0E-9 | 6.8E0 | 5.0E-2 | 5.2E1 | 2.0E-1 | 1.0E-9 | 1.0E-9 | 5.8E2 | 7.9E-1 | 248 | 16 | 160 | 16 | 0.46 |
| Qw | pg/ml | 1.0E-9 | 1.0E-9 | 2.5E0 | 1.1E-2 | 1.1E1 | 3.1E-2 | 1.0E-9 | 1.0E-9 | 1.2E2 | 9.0E-2 | 248 | 16 | 160 | 16 | 0.30 |
| Qv | pg/ml | 2.4E4 | 9.5E3 | 3.4E4 | 7.3E4 | 5.7E4 | 2.3E5 | 1.0E-9 | 1.6E3 | 7.4E5 | 9.4E5 | 248 | 16 | 160 | 16 | 0.33 |
| Qu | pg/ml | 7.7E0 | 1.9E0 | 8.8E1 | 2.7E1 | 1.7E2 | 5.5E1 | 1.0E-9 | 1.0E-9 | 9.8E2 | 2.1E2 | 248 | 16 | 160 | 16 | 0.43 |
| Qt | pg/ml | 1.0E1 | 6.3E0 | 5.3E1 | 3.7E1 | 1.3E2 | 8.2E1 | 1.0E-9 | 1.0E-9 | 1.0E3 | 3.3E2 | 248 | 16 | 160 | 16 | 0.49 |
| Qh | ng/ml | 1.7E1 | 2.3E1 | 3.6E1 | 4.2E1 | 6.1E1 | 5.1E1 | 1.0E-9 | 7.8E-1 | 6.4E2 | 2.0E2 | 248 | 16 | 160 | 16 | 0.56 |
| Qg | ng/ml | 8.5E0 | 8.3E0 | 2.1E1 | 1.5E1 | 7.1E1 | 2.2E1 | 5.1E-2 | 6.9E-1 | 1.0E3 | 7.4E1 | 248 | 16 | 160 | 16 | 0.46 |
| Jj | ng/ml | 6.7E2 | 4.1E2 | 1.9E3 | 1.0E3 | 1.4E4 | 1.5E3 | 2.3E0 | 1.3E1 | 3.4E5 | 6.7E3 | 691 | 28 | 280 | 28 | 0.43 |
| Jk | ng/ml | 3.0E0 | 3.0E0 | 2.2E1 | 2.0E1 | 4.7E1 | 3.2E1 | 1.0E-9 | 1.2E-1 | 3.9E2 | 1.2E2 | 691 | 28 | 280 | 28 | 0.50 |
| Jl | ng/ml | 3.9E-1 | 5.1E-1 | 1.7E0 | 4.4E0 | 4.2E0 | 7.5E0 | 7.6E-4 | 2.3E-3 | 3.2E1 | 2.0E1 | 691 | 28 | 280 | 28 | 0.60 |
| Jm | ng/ml | 1.7E1 | 1.5E1 | 5.2E1 | 4.5E1 | 1.1E2 | 6.8E1 | 1.0E-9 | 3.8E-1 | 1.4E3 | 3.2E2 | 691 | 28 | 280 | 28 | 0.50 |
| Jn | pg/ml | 3.8E-1 | 4.4E-1 | 2.4E0 | 8.5E-1 | 2.4E1 | 9.0E-1 | 1.0E-9 | 4.7E-2 | 6.2E2 | 3.2E0 | 691 | 28 | 280 | 28 | 0.59 |
| Jo | pg/ml | 3.6E3 | 3.6E3 | 4.9E3 | 4.2E3 | 3.9E3 | 3.3E3 | 2.0E1 | 2.7E2 | 2.4E4 | 1.3E4 | 691 | 28 | 280 | 28 | 0.46 |
| Jp | pg/ml | 6.8E4 | 7.5E4 | 7.1E4 | 8.0E4 | 3.6E4 | 3.0E4 | 5.8E2 | 8.9E3 | 3.0E5 | 1.4E5 | 691 | 28 | 280 | 28 | 0.61 |
| Jq | pg/ml | 9.5E1 | 1.4E2 | 1.5E2 | 1.9E2 | 2.2E2 | 1.9E2 | 1.0E0 | 2.6E1 | 4.0E3 | 7.5E2 | 691 | 28 | 280 | 28 | 0.58 |
| Jr | pg/ml | 5.1E0 | 6.2E0 | 3.5E1 | 1.0E1 | 4.1E2 | 1.2E1 | 1.0E-9 | 1.0E-9 | 1.1E4 | 4.8E1 | 691 | 28 | 280 | 28 | 0.56 |
| Js | pg/ml | 1.3E1 | 1.3E1 | 5.2E1 | 2.1E1 | 4.0E2 | 2.6E1 | 1.0E-9 | 4.5E-1 | 1.0E4 | 1.1E2 | 691 | 28 | 280 | 28 | 0.49 |
| Jt | pg/ml | 2.5E3 | 2.5E3 | 3.1E3 | 2.9E3 | 2.3E3 | 2.0E3 | 2.2E1 | 3.5E2 | 2.2E4 | 7.8E3 | 691 | 28 | 280 | 28 | 0.49 |
| Ju | mIU/ml | 8.5E0 | 6.7E0 | 2.0E1 | 1.4E1 | 3.2E1 | 2.5E1 | 6.5E-2 | 1.7E-1 | 2.3E2 | 1.0E2 | 249 | 16 | 162 | 16 | 0.43 |
| Jv | mIU/ml | 1.1E1 | 1.3E1 | 3.5E1 | 2.7E1 | 6.3E1 | 4.6E1 | 1.0E-2 | 9.4E-3 | 4.4E2 | 1.8E2 | 249 | 16 | 162 | 16 | 0.45 |
| Jy | ng/ml | 1.6E-3 | 1.6E-3 | 2.2E-3 | 1.7E-3 | 4.3E-3 | 9.2E-4 | 1.0E-9 | 4.5E-4 | 5.2E-2 | 3.5E-3 | 249 | 16 | 162 | 16 | 0.49 |
| Kc | pg/ml | 2.3E1 | 4.6E1 | 4.1E1 | 7.0E1 | 4.3E1 | 5.7E1 | 1.0E-9 | 6.1E0 | 1.9E2 | 1.6E2 | 249 | 17 | 160 | 17 | 0.64 |
| Kd | pg/ml | 1.0E-9 | 1.0E-9 | 1.6E2 | 2.0E2 | 5.4E2 | 5.3E2 | 1.0E-9 | 1.0E-9 | 4.8E3 | 1.8E3 | 249 | 17 | 160 | 17 | 0.53 |
| Ke | pg/ml | 1.2E4 | 1.2E4 | 1.4E4 | 1.5E4 | 1.1E4 | 1.3E4 | 3.4E2 | 1.3E3 | 7.0E4 | 4.4E4 | 249 | 17 | 160 | 17 | 0.49 |
| Kf | pg/mL | 6.4E0 | 8.7E0 | 6.8E0 | 9.5E0 | 5.6E0 | 5.4E0 | 1.0E-9 | 1.0E-9 | 2.6E1 | 1.6E1 | 249 | 17 | 160 | 17 | 0.65 |
| Kg | pg/mL | 1.1E3 | 5.1E2 | 1.9E3 | 1.3E3 | 2.6E3 | 1.4E3 | 7.3E1 | 2.1E2 | 2.2E4 | 4.6E3 | 249 | 17 | 160 | 17 | 0.38 |
| Ki | pg/ml | 6.1E1 | 5.6E1 | 7.0E1 | 5.6E1 | 5.2E1 | 2.3E1 | 1.0E-9 | 1.8E1 | 3.8E2 | 1.0E2 | 248 | 17 | 160 | 17 | 0.43 |
| Kj | pg/ml | 1.0E3 | 6.3E2 | 1.6E3 | 1.1E3 | 1.6E3 | 1.4E3 | 1.4E1 | 9.4E1 | 1.0E4 | 6.1E3 | 249 | 17 | 160 | 17 | 0.36 |
| Kk | pg/ml | 6.9E0 | 8.2E0 | 1.1E1 | 1.6E1 | 1.5E1 | 1.7E1 | 1.0E-9 | 1.0E-9 | 1.6E2 | 4.8E1 | 249 | 17 | 160 | 17 | 0.53 |
| Kl | pg/ml | 2.0E4 | 1.7E4 | 2.7E4 | 2.9E4 | 2.5E4 | 2.4E4 | 1.6E2 | 8.3E2 | 1.6E5 | 6.9E4 | 249 | 17 | 160 | 17 | 0.53 |
| Kn | pg/ml | 2.9E1 | 2.9E1 | 5.8E1 | 8.4E1 | 9.0E1 | 1.2E2 | 1.0E-9 | 1.0E-9 | 7.3E2 | 3.8E2 | 249 | 17 | 160 | 17 | 0.52 |
| Ko | pg/ml | 3.2E2 | 4.4E2 | 4.3E2 | 5.5E2 | 4.4E2 | 4.9E2 | 1.0E-9 | 1.0E-9 | 2.2E3 | 1.5E3 | 249 | 17 | 160 | 17 | 0.57 |
| Kp | pg/ml | 3.0E2 | 4.0E2 | 3.4E2 | 4.5E2 | 2.6E2 | 2.7E2 | 1.0E-9 | 1.0E-9 | 1.7E3 | 1.1E3 | 249 | 17 | 160 | 17 | 0.62 |
| Kq | pg/ml | 3.0E2 | 3.4E2 | 4.8E2 | 5.8E2 | 8.7E2 | 6.1E2 | 1.6E0 | 8.8E1 | 9.8E3 | 2.1E3 | 240 | 17 | 154 | 17 | 0.57 |
| Kr | pg/ml | 3.8E-1 | 2.9E-1 | 2.2E0 | 1.2E0 | 4.2E0 | 2.2E0 | 1.0E-9 | 1.0E-9 | 3.5E1 | 8.0E0 | 240 | 17 | 154 | 17 | 0.47 |
| Ks | pg/ml | 1.4E4 | 8.0E3 | 2.0E4 | 1.3E4 | 1.8E4 | 1.4E4 | 5.1E1 | 4.1E2 | 1.1E5 | 5.0E4 | 240 | 17 | 154 | 17 | 0.37 |
| Kx | ng/ml | 1.0E-9 | 4.2E-3 | 6.6E-3 | 7.4E-3 | 1.4E-2 | 1.0E-2 | 1.0E-9 | 1.0E-9 | 1.0E-1 | 4.1E-2 | 247 | 17 | 160 | 17 | 0.57 |
| Ky | ng/ml | 8.5E-2 | 1.5E-1 | 3.5E-1 | 8.3E-1 | 7.8E-1 | 1.6E0 | 1.0E-9 | 1.0E-9 | 5.4E0 | 6.4E0 | 247 | 17 | 160 | 17 | 0.67 |
| Kz | ng/ml | 1.0E-9 | 1.0E-9 | 3.7E-3 | 2.7E-3 | 5.9E-3 | 3.9E-3 | 1.0E-9 | 1.0E-9 | 3.6E-2 | 1.4E-2 | 247 | 17 | 160 | 17 | 0.50 |
| Ld | pg/ml | 1.0E-9 | 3.6E0 | 3.6E0 | 4.3E0 | 9.2E0 | 5.1E0 | 1.0E-9 | 1.0E-9 | 8.4E1 | 1.9E1 | 246 | 17 | 159 | 17 | 0.63 |
| Lh | pg/ml | 1.2E4 | 1.7E4 | 2.0E4 | 2.4E4 | 2.5E4 | 2.4E4 | 1.0E-9 | 4.0E2 | 2.6E5 | 1.0E5 | 691 | 27 | 281 | 27 | 0.58 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|------|--------|------|-----|
| | | NonDis | Dis | NonDis | Dis | NonDis | Dis | NonDis | Dis | NonDis | Dis | NonDis | Dis | NonDis | Dis | |
| Li | pg/ml | 2.8E3 | 4.0E3 | 1.5E4 | 1.0E4 | 5.9E4 | 1.6E4 | 1.0E-9 | 1.2E2 | 1.3E6 | 6.8E4 | 691 | 27 | 281 | 27 | 0.52 |
| Lj | pg/ml | 2.3E3 | 3.9E3 | 2.1E4 | 1.4E4 | 6.5E4 | 2.5E4 | 1.0E-9 | 3.4E1 | 4.7E5 | 9.4E4 | 691 | 27 | 281 | 27 | 0.52 |
| Rm | ng/ml | 1.9E1 | 2.4E1 | 4.9E1 | 6.1E1 | 7.4E1 | 8.0E1 | 2.2E-1 | 4.0E-1 | 4.0E2 | 2.5E2 | 245 | 16 | 159 | 16 | 0.53 |
| Rh | ng/ml | 1.3E2 | 7.9E1 | 3.6E2 | 2.1E2 | 1.2E3 | 2.5E2 | 3.6E0 | 1.8E1 | 1.7E4 | 8.5E2 | 245 | 16 | 159 | 16 | 0.42 |
| Ri | ng/ml | 1.0E-9 | 4.1E-2 | 4.4E0 | 6.4E0 | 1.6E1 | 1.3E1 | 1.0E-9 | 1.0E-9 | 2.2E2 | 4.1E1 | 246 | 16 | 160 | 16 | 0.54 |
| Rg | ng/ml | 1.0E-9 | 1.0E-9 | 7.1E-2 | 3.3E-3 | 4.2E-1 | 7.1E-3 | 1.0E-9 | 1.0E-9 | 4.6E0 | 2.8E-2 | 245 | 16 | 159 | 16 | 0.57 |
| Rj | ng/ml | 1.0E-9 | 1.0E-9 | 1.6E0 | 4.4E-2 | 6.0E0 | 1.8E-1 | 1.0E-9 | 1.0E-9 | 8.0E1 | 7.0E-1 | 246 | 16 | 160 | 16 | 0.38 |
| Rf | ng/ml | 3.7E-1 | 3.2E-1 | 9.8E-1 | 6.4E-1 | 1.9E0 | 6.9E-1 | 7.8E-3 | 3.9E-2 | 1.5E1 | 2.1E0 | 245 | 16 | 159 | 16 | 0.48 |
| Ql | pg/ml | 4.5E0 | 4.3E-1 | 1.4E1 | 6.0E0 | 3.1E1 | 7.8E0 | 1.0E-9 | 1.0E-9 | 2.9E2 | 2.4E1 | 249 | 16 | 162 | 16 | 0.42 |
| Qm | pg/ml | 3.9E0 | 1.0E-9 | 2.0E1 | 1.9E1 | 3.9E1 | 3.3E1 | 1.0E-9 | 1.0E-9 | 3.6E2 | 1.1E2 | 249 | 16 | 162 | 16 | 0.47 |
| Qn | pg/ml | 6.1E-1 | 1.2E0 | 6.9E0 | 1.4E1 | 2.2E1 | 2.5E1 | 1.0E-9 | 1.0E-9 | 2.2E2 | 7.5E1 | 249 | 16 | 162 | 16 | 0.57 |
| Nv | pg/ml | 3.8E3 | 5.0E3 | 1.1E4 | 1.5E4 | 4.8E4 | 3.1E4 | 1.0E-9 | 9.8E1 | 1.1E6 | 1.6E5 | 696 | 28 | 281 | 28 | 0.57 |
| Nw | pg/ml | 8.1E3 | 1.3E4 | 1.2E4 | 1.5E4 | 1.7E4 | 1.1E4 | 8.6E1 | 7.5E2 | 2.1E5 | 5.0E4 | 696 | 28 | 281 | 28 | 0.64 |
| Nx | pg/ml | 2.0E2 | 3.7E2 | 3.8E2 | 6.8E2 | 6.6E2 | 6.3E2 | 1.0E-9 | 1.0E-9 | 7.4E3 | 2.1E3 | 696 | 28 | 281 | 28 | 0.69 |
| Ny | pg/ml | 5.5E0 | 9.6E0 | 6.2E1 | 6.2E1 | 9.6E2 | 2.3E2 | 1.0E-9 | 1.0E-9 | 2.5E4 | 1.2E3 | 696 | 28 | 281 | 28 | 0.56 |
| Oa | pg/ml | 1.6E2 | 5.7E2 | 4.0E2 | 6.9E2 | 6.9E2 | 7.7E2 | 1.0E-9 | 1.0E-9 | 4.8E3 | 2.9E3 | 249 | 16 | 162 | 16 | 0.65 |
| Wn | ng/ml | 1.3E1 | 1.1E1 | 5.8E1 | 4.0E1 | 2.1E2 | 4.6E1 | 8.9E-1 | 9.8E-1 | 1.8E3 | 1.0E2 | 86 | 7 | 63 | 7 | 0.49 |
| Tk | ng/ml | 1.3E2 | 7.1E1 | 3.1E2 | 1.5E2 | 5.6E2 | 1.9E2 | 3.0E0 | 5.8E0 | 4.2E3 | 5.6E2 | 92 | 7 | 66 | 7 | 0.37 |
| Oe | pg/ml | 4.9E1 | 1.1E2 | 2.9E2 | 3.0E2 | 8.1E2 | 4.4E2 | 1.0E-9 | 1.0E-9 | 1.9E4 | 1.6E3 | 688 | 28 | 281 | 28 | 0.51 |
| Of | pg/ml | 1.8E2 | 1.1E2 | 6.4E3 | 8.6E3 | 3.1E4 | 2.5E4 | 1.0E-9 | 1.0E-9 | 6.2E5 | 1.2E5 | 695 | 28 | 281 | 28 | 0.47 |
| Og | pg/ml | 8.4E-2 | 1.0E-1 | 5.3E-1 | 2.9E-1 | 1.7E0 | 7.5E-1 | 1.0E-9 | 1.0E-9 | 2.0E1 | 4.0E0 | 695 | 28 | 281 | 28 | 0.48 |
| Oh | pg/ml | 2.4E0 | 4.5E0 | 2.3E1 | 8.0E0 | 1.6E2 | 1.2E1 | 1.0E-9 | 1.0E-9 | 3.5E3 | 5.6E1 | 695 | 28 | 281 | 28 | 0.56 |
| Oi | pg/ml | 2.3E0 | 3.1E0 | 6.1E0 | 5.5E0 | 9.9E0 | 7.7E0 | 1.0E-9 | 1.0E-9 | 8.6E1 | 3.6E1 | 695 | 28 | 281 | 28 | 0.52 |
| Ok | pg/ml | 3.5E2 | 6.1E2 | 5.0E2 | 8.1E2 | 5.2E2 | 9.7E2 | 1.3E1 | 4.2E1 | 5.2E3 | 5.2E3 | 695 | 28 | 281 | 28 | 0.63 |
| Om | pg/ml | 3.8E2 | 5.7E2 | 8.5E2 | 6.9E2 | 2.3E3 | 6.3E2 | 1.0E-9 | 1.0E-9 | 3.6E4 | 2.5E3 | 695 | 28 | 281 | 28 | 0.55 |
| On | pg/ml | 1.6E2 | 2.6E2 | 2.7E2 | 4.0E2 | 4.1E2 | 4.1E2 | 1.0E-9 | 1.2E1 | 4.5E3 | 1.7E3 | 695 | 28 | 281 | 28 | 0.62 |
| Or | pg/ml | 1.2E1 | 2.7E1 | 3.1E1 | 6.4E1 | 6.0E1 | 8.7E1 | 1.0E-9 | 1.0E-9 | 5.0E2 | 3.4E2 | 249 | 17 | 160 | 17 | 0.66 |
| Ow | pg/ml | 3.3E1 | 9.7E1 | 1.2E2 | 7.2E2 | 3.5E2 | 1.9E3 | 1.0E-9 | 1.0E-9 | 3.2E3 | 8.1E3 | 249 | 17 | 160 | 17 | 0.72 |
| Ou | pg/ml | 4.6E2 | 8.4E2 | 8.6E2 | 1.7E3 | 1.3E3 | 1.8E3 | 1.0E-9 | 1.0E-9 | 9.8E3 | 6.6E3 | 249 | 17 | 160 | 17 | 0.66 |
| Ul | ng/ml | 1.0E-9 | 1.0E-9 | 1.2E0 | 1.3E0 | 4.6E0 | 3.5E0 | 1.0E-9 | 1.0E-9 | 3.4E1 | 1.0E1 | 257 | 16 | 164 | 16 | 0.52 |
| Uo | ng/ml | 1.0E-9 | 1.0E-9 | 9.2E-2 | 9.1E-2 | 2.1E-1 | 1.7E-1 | 1.0E-9 | 1.0E-9 | 1.3E0 | 5.8E-1 | 257 | 16 | 164 | 16 | 0.54 |
| Uk | ng/ml | 1.0E-9 | 1.0E-9 | 7.8E-3 | 4.7E-3 | 2.7E-2 | 1.8E-2 | 1.0E-9 | 1.0E-9 | 3.2E-1 | 7.1E-2 | 257 | 16 | 164 | 16 | 0.41 |
| Um | ng/ml | 1.0E-9 | 1.0E-9 | 3.7E-1 | 6.7E-1 | 8.5E-1 | 1.1E0 | 1.0E-9 | 1.0E-9 | 6.8E0 | 2.7E0 | 257 | 16 | 164 | 16 | 0.53 |
| Uf | ng/ml | 5.1E-2 | 8.6E-2 | 1.2E-1 | 6.2E-1 | 1.9E-1 | 2.1E0 | 1.0E-3 | 3.5E-3 | 1.7E0 | 8.6E0 | 257 | 16 | 164 | 16 | 0.56 |
| Uh | ng/ml | 1.8E0 | 2.0E0 | 2.9E0 | 4.5E0 | 3.2E0 | 5.4E0 | 3.2E-2 | 1.3E-2 | 1.7E1 | 2.1E1 | 257 | 16 | 164 | 16 | 0.57 |
| Un | ng/ml | 1.8E0 | 2.3E0 | 2.1E0 | 2.0E0 | 1.3E0 | 1.1E0 | 2.0E-1 | 1.3E-1 | 8.0E0 | 3.9E0 | 257 | 16 | 164 | 16 | 0.52 |
| Ug | ng/ml | 1.4E1 | 6.2E0 | 2.7E1 | 1.9E1 | 2.8E1 | 2.8E1 | 6.9E-1 | 1.8E0 | 1.8E2 | 1.1E2 | 257 | 16 | 164 | 16 | 0.34 |
| Ur | ng/ml | 1.5E-1 | 6.5E-2 | 7.7E-1 | 5.8E-1 | 5.9E0 | 1.2E0 | 1.0E-9 | 1.0E-9 | 9.3E1 | 4.7E0 | 256 | 16 | 163 | 16 | 0.41 |
| Up | ng/ml | 1.0E-9 | 1.0E-9 | 5.4E-3 | 2.6E-3 | 2.5E-2 | 8.6E-3 | 1.0E-9 | 1.0E-9 | 3.3E-1 | 3.5E-2 | 256 | 16 | 163 | 16 | 0.48 |
| Us | ng/ml | 3.2E-3 | 1.0E-9 | 1.8E-2 | 7.9E-3 | 4.4E-2 | 1.7E-2 | 1.0E-9 | 1.0E-9 | 4.0E-1 | 6.3E-2 | 256 | 16 | 163 | 16 | 0.37 |
| Uv | ng/ml | 3.0E-3 | 1.5E-3 | 1.3E-2 | 5.3E-3 | 4.2E-2 | 8.9E-3 | 1.0E-9 | 1.0E-9 | 4.3E-1 | 3.3E-2 | 256 | 16 | 163 | 16 | 0.42 |
| Ut | ng/ml | 6.6E-1 | 3.1E-1 | 2.9E0 | 1.3E0 | 9.1E0 | 1.9E0 | 1.0E-9 | 1.0E-9 | 7.8E1 | 5.0E0 | 256 | 16 | 163 | 16 | 0.42 |
| Uu | ng/ml | 7.0E0 | 7.6E0 | 7.7E0 | 8.3E0 | 5.0E0 | 5.3E0 | 4.5E-1 | 1.3E0 | 2.6E1 | 2.1E1 | 256 | 16 | 163 | 16 | 0.54 |
| Vp | ng/ml | 1.0E-9 | 1.0E-9 | 4.0E-1 | 7.3E-2 | 3.6E0 | 1.7E-1 | 1.0E-9 | 1.0E-9 | 5.0E1 | 6.0E-1 | 257 | 16 | 164 | 16 | 0.52 |
| Vt | ng/ml | 6.0E0 | 8.4E0 | 8.4E0 | 9.3E0 | 9.1E0 | 6.7E0 | 4.3E-1 | 1.7E0 | 8.6E1 | 2.2E1 | 257 | 16 | 164 | 16 | 0.57 |
| Vu | ng/ml | 1.0E-9 | 5.3E-1 | 2.3E0 | 1.7E0 | 5.6E0 | 2.6E0 | 1.0E-9 | 1.0E-9 | 5.1E1 | 9.3E0 | 253 | 15 | 164 | 15 | 0.55 |
| Vq | ng/ml | 1.6E2 | 4.5E2 | 4.1E3 | 7.6E2 | 4.8E4 | 8.3E2 | 2.0E-1 | 1.7E1 | 6.8E5 | 2.3E3 | 203 | 11 | 135 | 11 | 0.61 |
| Vo | ng/ml | 2.6E1 | 2.5E1 | 2.4E1 | 2.3E1 | 5.4E0 | 5.1E0 | 2.4E0 | 8.0E0 | 4.8E1 | 3.0E1 | 257 | 16 | 164 | 16 | 0.41 |
| Vs | ng/ml | 1.0E-9 | 1.0E-9 | 6.7E0 | 9.5E0 | 2.4E1 | 2.3E1 | 1.0E-9 | 1.0E-9 | 2.3E2 | 9.4E1 | 253 | 16 | 161 | 16 | 0.53 |
| Vv | ng/ml | 2.9E0 | 3.0E0 | 6.1E0 | 4.2E0 | 1.0E1 | 6.5E0 | 1.0E-9 | 1.0E-9 | 8.4E1 | 2.6E1 | 256 | 16 | 164 | 16 | 0.47 |
| Oy | pg/ml | 5.3E-1 | 4.9E-1 | 6.6E0 | 2.2E0 | 3.2E1 | 4.0E0 | 1.0E-9 | 1.0E-9 | 4.0E2 | 1.5E1 | 694 | 28 | 280 | 28 | 0.50 |
| Oz | pg/ml | 1.3E-2 | 5.4E-2 | 3.4E-1 | 2.4E-1 | 1.5E0 | 3.9E-1 | 1.0E-9 | 1.0E-9 | 2.9E1 | 1.7E0 | 694 | 28 | 280 | 28 | 0.51 |
| Pa | pg/ml | 3.8E-1 | 4.1E-1 | 1.5E0 | 8.1E-1 | 5.5E0 | 1.0E0 | 1.0E-9 | 1.0E-9 | 8.6E1 | 4.4E0 | 694 | 28 | 280 | 28 | 0.55 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pb | pg/ml | 1.0E-9 | 1.0E-9 | 9.3E-1 | 1.1E0 | 1.9E1 | 5.3E0 | 1.0E-9 | 1.0E-9 | 4.9E2 | 2.8E1 | 694 | 28 | 280 | 28 | 0.51 |
| Pc | pg/ml | 4.7E-2 | 2.7E-1 | 3.7E-1 | 3.8E-1 | 9.4E-1 | 4.7E-1 | 1.0E-9 | 1.0E-9 | 1.6E1 | 1.8E0 | 694 | 28 | 280 | 28 | 0.57 |
| Pd | pg/ml | 1.7E0 | 2.5E0 | 5.1E0 | 3.4E0 | 3.3E1 | 3.4E0 | 1.0E-9 | 1.0E-9 | 8.4E2 | 1.4E1 | 694 | 28 | 280 | 28 | 0.57 |
| Pe | pg/ml | 2.0E1 | 3.4E1 | 1.0E2 | 5.5E1 | 3.5E2 | 6.2E1 | 1.0E-9 | 1.0E-9 | 4.7E3 | 2.5E2 | 694 | 28 | 280 | 28 | 0.56 |
| Pf | pg/ml | 1.4E0 | 2.3E0 | 1.1E1 | 7.5E0 | 6.4E1 | 1.5E1 | 1.0E-9 | 1.0E-9 | 1.5E3 | 7.7E1 | 694 | 28 | 280 | 28 | 0.57 |
| Pg | pg/ml | 3.0E0 | 4.8E0 | 4.7E1 | 2.9E1 | 3.8E2 | 5.2E1 | 1.0E-9 | 1.0E-9 | 7.7E3 | 1.9E2 | 694 | 28 | 280 | 28 | 0.58 |
| Ph | ng/ml | 1.7E-1 | 1.7E-1 | 3.2E-1 | 3.6E-1 | 4.5E-1 | 5.5E-1 | 1.0E-9 | 1.0E-9 | 2.9E0 | 2.3E0 | 249 | 17 | 160 | 17 | 0.49 |
| Pi | ng/ml | 2.0E-1 | 2.2E-1 | 2.8E-1 | 2.9E-1 | 3.6E-1 | 2.6E-1 | 1.0E-9 | 1.0E-9 | 3.2E0 | 8.6E-1 | 249 | 17 | 160 | 17 | 0.53 |
| Pj | ng/mL | 4.8E0 | 5.1E0 | 5.9E0 | 5.5E0 | 4.5E0 | 3.7E0 | 3.8E-2 | 8.8E-1 | 3.1E1 | 1.3E1 | 249 | 17 | 160 | 17 | 0.48 |
| Pk | ng/ml | 8.9E-3 | 7.6E-3 | 1.4E-2 | 1.0E-2 | 2.2E-2 | 1.2E-2 | 1.0E-9 | 1.0E-9 | 2.5E-1 | 4.3E-2 | 249 | 17 | 160 | 17 | 0.42 |
| aA | mg/dL | 8.0E-1 | 9.0E-1 | 9.2E-1 | 1.0E0 | 4.7E-1 | 5.5E-1 | 2.0E-1 | 4.0E-1 | 4.2E0 | 3.2E0 | 2240 | 35 | 436 | 35 | 0.57 |
| aC | mg/mL | 2.9E0 | 1.9E0 | 3.2E0 | 2.4E0 | 1.4E0 | 1.3E0 | 8.5E-1 | 1.1E0 | 8.9E0 | 5.6E0 | 437 | 17 | 174 | 17 | 0.31 |
| aD | ug/mL | 3.1E0 | 4.1E0 | 4.4E0 | 5.3E0 | 3.9E0 | 4.2E0 | 4.3E-1 | 1.1E0 | 3.5E1 | 1.7E1 | 437 | 17 | 174 | 17 | 0.54 |
| aE | mg/mL | 5.6E-1 | 5.5E-1 | 5.8E-1 | 5.5E-1 | 1.5E-1 | 1.6E-1 | 2.1E-1 | 2.5E-1 | 1.1E0 | 8.6E-1 | 437 | 17 | 174 | 17 | 0.44 |
| aF | ng/mL | 2.1E0 | 2.0E0 | 4.1E0 | 5.1E0 | 6.1E0 | 7.8E0 | 4.3E-3 | 4.3E-3 | 5.0E1 | 2.9E1 | 437 | 17 | 174 | 17 | 0.49 |
| aG | mg/mL | 1.4E-1 | 1.3E-1 | 1.6E-1 | 1.5E-1 | 9.1E-2 | 8.2E-2 | 1.7E-2 | 5.6E-2 | 5.4E-1 | 3.8E-1 | 437 | 17 | 174 | 17 | 0.45 |
| aH | ug/mL | 7.5E1 | 7.6E1 | 8.3E1 | 8.5E1 | 4.4E1 | 4.1E1 | 4.6E0 | 2.3E1 | 2.9E2 | 1.4E2 | 437 | 17 | 174 | 17 | 0.54 |
| aI | ug/mL | 1.9E2 | 1.7E2 | 1.9E2 | 1.7E2 | 6.0E1 | 6.7E1 | 2.8E1 | 7.7E1 | 3.7E2 | 2.8E2 | 437 | 17 | 174 | 17 | 0.43 |
| aJ | ug/mL | 2.4E0 | 3.7E0 | 2.9E0 | 4.2E0 | 2.1E0 | 2.8E0 | 7.6E-1 | 9.5E-1 | 1.7E1 | 1.1E1 | 437 | 17 | 174 | 17 | 0.65 |
| aK | ng/mL | 1.6E0 | 8.3E-1 | 2.5E0 | 1.8E0 | 2.7E0 | 1.9E0 | 2.9E-4 | 9.1E-2 | 1.8E1 | 5.6E0 | 437 | 17 | 174 | 17 | 0.42 |
| aL | mg/mL | 8.1E-1 | 8.3E-1 | 8.2E-1 | 8.5E-1 | 2.5E-1 | 3.2E-1 | 1.9E-1 | 2.9E-1 | 1.7E0 | 1.6E0 | 437 | 17 | 174 | 17 | 0.52 |
| aM | U/mL | 2.2E1 | 2.1E1 | 4.5E1 | 4.5E1 | 9.6E1 | 4.3E1 | 4.2E-2 | 4.2E-2 | 1.6E3 | 1.3E2 | 437 | 17 | 174 | 17 | 0.55 |
| aN | U/mL | 1.3E1 | 2.1E1 | 2.0E1 | 3.1E1 | 2.8E1 | 2.6E1 | 2.5E-3 | 2.5E-3 | 3.8E2 | 9.5E1 | 437 | 17 | 174 | 17 | 0.66 |
| aO | pg/mL | 3.1E1 | 3.2E1 | 3.2E2 | 2.1E2 | 8.3E2 | 4.0E2 | 6.0E-2 | 6.0E-2 | 6.6E3 | 1.3E3 | 437 | 17 | 174 | 17 | 0.52 |
| aP | ng/mL | 1.6E0 | 1.8E0 | 2.0E0 | 2.2E0 | 1.8E0 | 1.4E0 | 4.5E-1 | 7.0E-1 | 2.8E1 | 6.6E0 | 437 | 17 | 174 | 17 | 0.55 |
| aQ | ng/mL | 3.0E-1 | 2.8E-1 | 4.7E-1 | 3.0E-1 | 4.8E-1 | 2.5E-1 | 2.0E-4 | 6.7E-2 | 4.0E0 | 9.2E-1 | 437 | 17 | 174 | 17 | 0.39 |
| aR | ng/mL | 1.7E0 | 2.4E0 | 2.7E0 | 2.4E0 | 3.3E0 | 1.7E0 | 1.8E-1 | 3.6E-1 | 3.4E1 | 7.9E0 | 437 | 17 | 174 | 17 | 0.54 |
| aS | ng/mL | 2.6E-1 | 2.9E-1 | 6.5E-1 | 4.5E-1 | 1.9E0 | 4.0E-1 | 4.2E-3 | 7.2E-2 | 3.3E1 | 1.3E0 | 437 | 17 | 174 | 17 | 0.53 |
| aU | pg/mL | 7.8E1 | 5.3E1 | 1.3E2 | 9.4E1 | 1.5E2 | 8.7E1 | 7.4E-2 | 6.5E0 | 1.3E3 | 3.3E2 | 437 | 17 | 174 | 17 | 0.44 |
| aV | ng/mL | 6.3E-1 | 3.6E-1 | 1.1E0 | 8.3E-1 | 2.0E0 | 1.4E0 | 7.6E-4 | 3.3E-2 | 3.3E1 | 6.0E0 | 437 | 17 | 174 | 17 | 0.37 |
| aW | pg/mL | 1.9E1 | 1.9E1 | 2.0E1 | 1.8E1 | 2.0E1 | 9.1E0 | 7.2E-2 | 7.2E-2 | 2.4E2 | 3.1E1 | 437 | 17 | 174 | 17 | 0.50 |
| aX | ng/mL | 9.4E0 | 7.1E0 | 1.5E1 | 2.3E1 | 1.9E1 | 5.0E1 | 3.0E-1 | 1.4E0 | 2.2E2 | 2.1E2 | 437 | 17 | 174 | 17 | 0.47 |
| aY | pg/mL | 5.4E1 | 7.8E1 | 7.7E1 | 9.6E1 | 8.7E1 | 6.9E1 | 4.1E-1 | 1.7E1 | 1.2E3 | 2.4E2 | 437 | 17 | 174 | 17 | 0.62 |
| aZ | pg/mL | 2.2E2 | 1.7E2 | 5.0E2 | 5.0E2 | 9.8E2 | 1.0E3 | 1.7E0 | 1.7E0 | 1.2E4 | 4.3E3 | 437 | 17 | 174 | 17 | 0.48 |
| bA | ng/mL | 8.0E0 | 2.7E1 | 3.0E1 | 8.7E1 | 7.8E1 | 1.3E2 | 3.0E-2 | 1.4E0 | 9.4E2 | 4.6E2 | 437 | 17 | 174 | 17 | 0.69 |
| bB | ng/mL | 3.1E2 | 2.2E2 | 3.4E2 | 2.8E2 | 1.7E2 | 1.9E2 | 2.1E0 | 1.2E1 | 1.0E3 | 6.2E2 | 437 | 17 | 174 | 17 | 0.40 |
| bC | ng/mL | 3.3E2 | 4.7E2 | 5.6E2 | 1.2E3 | 7.4E2 | 1.4E3 | 9.8E0 | 1.5E2 | 4.7E3 | 4.7E3 | 437 | 17 | 174 | 17 | 0.66 |
| bE | mg/mL | 5.5E0 | 5.9E0 | 5.8E0 | 6.2E0 | 2.0E0 | 3.1E0 | 9.8E-1 | 1.9E0 | 1.3E1 | 1.3E1 | 437 | 17 | 174 | 17 | 0.50 |
| bF | pg/mL | 1.9E1 | 2.3E1 | 1.6E2 | 2.4E2 | 9.8E2 | 4.7E2 | 5.0E-2 | 8.5E0 | 1.1E4 | 1.7E3 | 437 | 17 | 174 | 17 | 0.62 |
| bG | ng/mL | 1.6E0 | 2.8E0 | 2.7E0 | 3.2E0 | 3.3E0 | 3.3E0 | 2.2E-2 | 1.6E-1 | 2.6E1 | 1.1E1 | 437 | 17 | 174 | 17 | 0.54 |
| bH | pg/mL | 5.7E-1 | 5.7E-1 | 4.9E0 | 2.6E0 | 1.6E1 | 3.4E0 | 5.7E-1 | 5.7E-1 | 2.8E2 | 1.1E1 | 437 | 17 | 174 | 17 | 0.46 |
| bI | ng/mL | 4.0E-3 | 4.0E-3 | 6.5E-2 | 4.7E-2 | 1.6E-1 | 1.0E-1 | 4.0E-3 | 4.0E-3 | 1.3E0 | 3.1E-1 | 437 | 17 | 174 | 17 | 0.50 |
| bJ | mg/mL | 2.4E0 | 1.6E0 | 2.7E0 | 2.4E0 | 2.1E0 | 2.3E0 | 2.5E-4 | 2.1E-2 | 1.3E1 | 9.0E0 | 437 | 17 | 174 | 17 | 0.43 |
| bL | pg/mL | 4.0E0 | 4.7E0 | 8.3E0 | 8.1E0 | 1.1E1 | 9.2E0 | 4.6E-2 | 4.6E-2 | 8.0E1 | 3.2E1 | 437 | 17 | 174 | 17 | 0.50 |
| bM | mg/mL | 1.7E0 | 2.4E0 | 2.0E0 | 2.6E0 | 1.4E0 | 1.9E0 | 9.2E-3 | 7.1E-1 | 8.8E0 | 8.4E0 | 437 | 17 | 174 | 17 | 0.61 |
| bN | ng/mL | 4.3E1 | 3.9E1 | 1.4E2 | 8.4E1 | 2.9E2 | 1.4E2 | 1.4E-1 | 1.8E0 | 1.9E3 | 5.7E2 | 437 | 17 | 174 | 17 | 0.48 |
| bO | ng/mL | 4.0E-2 | 4.0E-2 | 1.0E1 | 8.5E0 | 2.3E1 | 1.4E1 | 4.0E-2 | 4.0E-2 | 1.9E2 | 3.8E1 | 437 | 17 | 174 | 17 | 0.51 |
| bP | mg/mL | 5.3E-1 | 6.8E-1 | 7.6E-1 | 8.2E-1 | 6.8E-1 | 7.4E-1 | 4.9E-2 | 9.7E-2 | 4.8E0 | 3.1E0 | 437 | 17 | 174 | 17 | 0.52 |
| bQ | pg/mL | 1.5E1 | 1.4E1 | 6.4E1 | 3.5E1 | 6.5E2 | 4.4E1 | 1.5E-1 | 4.4E0 | 1.3E4 | 1.8E2 | 437 | 17 | 174 | 17 | 0.52 |
| bR | ng/mL | 1.2E-2 | 1.2E-2 | 1.4E-1 | 6.4E-2 | 4.6E-1 | 1.0E-1 | 1.2E-2 | 1.2E-2 | 8.7E0 | 3.2E-1 | 437 | 17 | 174 | 17 | 0.41 |
| bS | pg/mL | 9.4E-1 | 9.4E-1 | 7.2E0 | 3.3E0 | 2.9E1 | 9.9E0 | 9.4E-1 | 9.4E-1 | 3.9E2 | 4.2E1 | 437 | 17 | 174 | 17 | 0.46 |
| bU | ng/mL | 1.5E-1 | 6.8E-2 | 2.0E-1 | 1.4E-1 | 3.8E-1 | 1.6E-1 | 1.3E-2 | 1.3E-2 | 6.6E0 | 4.9E-1 | 437 | 17 | 174 | 17 | 0.44 |
| bV | pg/mL | 4.7E2 | 4.9E2 | 5.5E2 | 6.2E2 | 5.8E2 | 3.9E2 | 1.6E2 | 2.6E2 | 1.2E4 | 1.6E3 | 437 | 17 | 174 | 17 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bW | pg/mL | 3.2E2 | 3.7E2 | 4.9E2 | 1.7E3 | 4.8E2 | 4.7E3 | 8.4E1 | 1.3E2 | 4.8E3 | 2.0E4 | 437 | 17 | 174 | 17 | 0.55 |
| bX | ng/mL | 1.5E-3 | 2.5E-5 | 2.8E-3 | 1.6E-3 | 3.5E-3 | 3.2E-3 | 2.5E-5 | 2.5E-5 | 2.0E-2 | 1.2E-2 | 437 | 17 | 174 | 17 | 0.38 |
| bZ | pg/mL | 2.3E2 | 2.8E2 | 9.1E2 | 7.0E2 | 4.1E3 | 7.6E2 | 1.5E-1 | 7.4E1 | 5.8E4 | 1.9E3 | 437 | 17 | 174 | 17 | 0.57 |
| cA | pg/mL | 6.0E-1 | 6.0E-1 | 2.8E0 | 1.2E0 | 1.8E1 | 2.5E0 | 6.0E-1 | 6.0E-1 | 3.7E2 | 1.1E1 | 437 | 17 | 174 | 17 | 0.46 |
| cB | ng/mL | 6.0E-2 | 3.6E-2 | 9.3E-2 | 5.1E-2 | 1.0E-1 | 5.0E-2 | 1.7E-3 | 1.7E-3 | 5.7E-1 | 1.6E-1 | 437 | 17 | 174 | 17 | 0.39 |
| cC | pg/mL | 4.6E1 | 3.8E1 | 4.8E1 | 4.1E1 | 4.0E1 | 2.4E1 | 1.0E0 | 1.0E0 | 4.5E2 | 9.6E1 | 437 | 17 | 174 | 17 | 0.44 |
| cD | pg/mL | 5.4E0 | 3.8E0 | 1.5E1 | 8.5E0 | 5.7E1 | 1.3E1 | 3.3E-1 | 3.3E-1 | 7.2E2 | 4.1E1 | 437 | 17 | 174 | 17 | 0.41 |
| cE | pg/mL | 3.2E1 | 6.7E1 | 1.5E2 | 5.1E2 | 4.6E2 | 1.0E3 | 1.2E-1 | 1.2E-1 | 3.8E3 | 3.8E3 | 437 | 17 | 174 | 17 | 0.63 |
| cF | pg/mL | 1.3E1 | 9.4E0 | 2.1E1 | 1.3E1 | 3.2E1 | 1.3E1 | 5.3E-1 | 5.3E-1 | 2.7E2 | 4.1E1 | 437 | 17 | 174 | 17 | 0.46 |
| cG | pg/mL | 4.3E1 | 6.0E1 | 1.1E2 | 1.3E2 | 5.2E2 | 1.5E2 | 7.8E0 | 1.0E1 | 1.0E4 | 5.6E2 | 437 | 17 | 174 | 17 | 0.65 |
| cH | uIU/mL | 3.1E0 | 2.9E0 | 6.5E0 | 3.0E0 | 1.2E1 | 2.7E0 | 8.6E-3 | 8.6E-3 | 1.6E2 | 1.0E1 | 437 | 17 | 174 | 17 | 0.40 |
| cI | ng/mL | 5.7E0 | 4.4E0 | 1.1E1 | 1.7E1 | 1.5E1 | 2.6E1 | 1.0E-3 | 3.2E-2 | 1.0E2 | 1.0E2 | 437 | 17 | 174 | 17 | 0.50 |
| cJ | ug/mL | 7.0E1 | 4.0E1 | 1.2E2 | 6.9E1 | 1.5E2 | 8.7E1 | 4.0E0 | 1.2E1 | 9.6E2 | 3.3E2 | 437 | 17 | 174 | 17 | 0.37 |
| cK | ng/mL | 3.8E-3 | 3.8E-3 | 5.5E-2 | 7.8E-3 | 1.9E-1 | 1.7E-2 | 3.8E-3 | 3.8E-3 | 1.7E0 | 7.3E-2 | 437 | 17 | 174 | 17 | 0.45 |
| cL | pg/mL | 1.9E2 | 2.2E2 | 3.6E2 | 5.4E2 | 1.3E3 | 6.7E2 | 1.6E1 | 6.5E1 | 2.4E4 | 2.6E3 | 437 | 17 | 174 | 17 | 0.64 |
| cM | pg/mL | 2.8E2 | 2.2E2 | 3.1E2 | 2.3E2 | 2.0E2 | 1.3E2 | 8.7E0 | 4.7E1 | 1.6E3 | 4.8E2 | 437 | 17 | 174 | 17 | 0.39 |
| cN | pg/mL | 1.2E2 | 1.3E2 | 1.3E2 | 1.6E2 | 6.4E1 | 7.3E1 | 3.8E1 | 6.8E1 | 1.1E3 | 3.5E2 | 437 | 17 | 174 | 17 | 0.64 |
| cO | pg/mL | 2.2E2 | 2.3E2 | 3.1E2 | 2.8E2 | 9.3E2 | 2.2E2 | 5.4E1 | 1.0E2 | 1.9E4 | 1.1E3 | 437 | 17 | 174 | 17 | 0.52 |
| cP | ng/mL | 2.5E3 | 2.8E3 | 2.6E3 | 2.8E3 | 9.2E2 | 9.6E2 | 6.2E2 | 1.4E3 | 5.7E3 | 4.8E3 | 437 | 17 | 174 | 17 | 0.55 |
| cQ | ng/mL | 4.3E-2 | 5.9E-2 | 1.3E-1 | 2.6E-1 | 2.4E-1 | 4.8E-1 | 2.0E-3 | 2.0E-3 | 2.2E0 | 1.9E0 | 437 | 17 | 174 | 17 | 0.56 |
| cR | ng/mL | 2.8E2 | 3.7E2 | 4.9E2 | 6.5E2 | 8.0E2 | 1.1E3 | 2.0E1 | 3.6E1 | 8.9E3 | 4.8E3 | 437 | 17 | 174 | 17 | 0.56 |
| cS | ng/mL | 2.6E2 | 2.4E2 | 3.8E2 | 4.0E2 | 3.8E2 | 3.2E2 | 4.1E1 | 9.1E1 | 2.7E3 | 1.2E3 | 437 | 17 | 174 | 17 | 0.52 |
| cT | ng/mL | 2.9E1 | 8.2E1 | 8.3E1 | 2.2E2 | 1.8E2 | 4.0E2 | 3.6E0 | 5.1E0 | 2.1E3 | 1.3E3 | 437 | 17 | 174 | 17 | 0.64 |
| cU | ng/mL | 5.4E1 | 7.8E1 | 7.5E1 | 7.2E1 | 9.9E1 | 4.6E1 | 6.2E0 | 5.4E0 | 1.6E3 | 1.5E2 | 437 | 17 | 174 | 17 | 0.54 |
| cV | ng/mL | 1.7E-1 | 2.8E-1 | 3.8E-1 | 1.0E0 | 2.3E0 | 2.4E0 | 3.4E-4 | 3.0E-2 | 4.7E1 | 9.7E0 | 437 | 17 | 174 | 17 | 0.63 |
| cW | mIU/mL | 5.3E-2 | 4.2E-2 | 1.5E-1 | 6.4E-2 | 7.2E-1 | 5.5E-2 | 3.7E-4 | 1.9E-2 | 9.7E0 | 2.1E-1 | 437 | 17 | 174 | 17 | 0.46 |
| cX | ng/mL | 9.9E-2 | 3.6E-1 | 1.3E0 | 4.9E0 | 4.2E0 | 9.9E0 | 2.3E-4 | 2.3E-4 | 2.8E1 | 2.8E1 | 437 | 17 | 174 | 17 | 0.57 |
| cY | ng/mL | 8.9E0 | 7.9E0 | 1.3E1 | 1.1E1 | 1.3E1 | 1.1E1 | 1.5E-1 | 6.1E-1 | 8.3E1 | 3.7E1 | 437 | 17 | 174 | 17 | 0.45 |
| cZ | ug/mL | 1.5E1 | 1.5E1 | 1.6E1 | 1.7E1 | 7.1E0 | 9.2E0 | 2.3E0 | 5.6E0 | 5.7E1 | 4.0E1 | 437 | 17 | 174 | 17 | 0.54 |
| dA | pg/mL | 3.3E2 | 3.2E2 | 3.8E2 | 3.6E2 | 3.1E2 | 2.1E2 | 9.0E1 | 1.6E2 | 5.8E3 | 9.3E2 | 437 | 17 | 174 | 17 | 0.46 |
| dB | ug/mL | 1.7E1 | 2.1E1 | 1.7E1 | 1.9E1 | 1.6E1 | 9.9E0 | 9.4E-1 | 2.5E0 | 2.5E2 | 3.2E1 | 437 | 17 | 174 | 17 | 0.63 |
| dC | nmol/L | 3.5E1 | 3.6E1 | 3.9E1 | 3.9E1 | 1.8E1 | 1.5E1 | 7.9E0 | 1.3E1 | 1.4E2 | 7.5E1 | 437 | 17 | 174 | 17 | 0.52 |
| dD | ug/mL | 3.7E1 | 3.0E1 | 3.8E1 | 3.5E1 | 1.1E1 | 1.3E1 | 1.3E1 | 1.6E1 | 7.6E1 | 6.2E1 | 437 | 17 | 174 | 17 | 0.42 |
| dE | ng/mL | 4.7E-1 | 3.8E-1 | 6.1E-1 | 3.3E-1 | 7.3E-1 | 2.8E-1 | 8.4E-3 | 8.4E-3 | 7.2E0 | 7.4E-1 | 437 | 17 | 174 | 17 | 0.39 |
| dF | ng/mL | 2.2E2 | 2.7E2 | 2.6E2 | 3.7E2 | 1.8E2 | 2.9E2 | 7.5E1 | 6.1E1 | 1.3E3 | 1.2E3 | 437 | 17 | 174 | 17 | 0.63 |
| dG | ng/mL | 1.1E1 | 1.6E1 | 1.4E1 | 2.1E1 | 1.2E1 | 1.8E1 | 2.5E0 | 4.2E0 | 1.8E2 | 7.6E1 | 437 | 17 | 174 | 17 | 0.65 |
| dH | pg/mL | 7.5E0 | 9.0E0 | 1.3E1 | 1.7E1 | 4.0E1 | 1.9E1 | 4.0E-2 | 2.2E0 | 6.7E2 | 7.9E1 | 437 | 17 | 174 | 17 | 0.63 |
| dI | pg/mL | 4.6E-1 | 4.6E-1 | 2.3E0 | 1.8E0 | 1.6E1 | 2.1E0 | 4.6E-1 | 4.6E-1 | 3.3E2 | 6.2E0 | 437 | 17 | 174 | 17 | 0.58 |
| dJ | ng/mL | 1.9E0 | 2.3E0 | 2.2E0 | 2.4E0 | 1.2E0 | 1.2E0 | 3.2E-2 | 3.2E-2 | 6.9E0 | 4.4E0 | 437 | 17 | 174 | 17 | 0.56 |
| dK | uIU/mL | 1.9E0 | 2.4E0 | 3.1E0 | 3.0E0 | 6.0E0 | 3.4E0 | 2.8E-4 | 4.3E-1 | 7.9E1 | 1.5E1 | 437 | 17 | 174 | 17 | 0.54 |
| dL | ng/mL | 8.7E2 | 1.2E3 | 1.0E3 | 1.3E3 | 4.9E2 | 8.1E2 | 3.4E2 | 3.3E2 | 3.4E3 | 3.8E3 | 437 | 17 | 174 | 17 | 0.62 |
| dM | pg/mL | 9.6E2 | 9.5E2 | 1.1E3 | 1.4E3 | 8.8E2 | 8.8E2 | 3.5E2 | 3.4E2 | 1.2E4 | 3.6E3 | 437 | 17 | 174 | 17 | 0.57 |
| dN | ug/mL | 9.3E1 | 1.2E2 | 9.9E1 | 1.2E2 | 3.6E1 | 5.0E1 | 2.5E1 | 1.6E1 | 2.8E2 | 2.0E2 | 437 | 17 | 174 | 17 | 0.62 |
| dR | pg/ml | 1.6E3 | 1.3E3 | 2.4E3 | 2.4E3 | 2.4E3 | 2.7E3 | 1.4E2 | 1.8E2 | 1.5E4 | 8.9E3 | 287 | 17 | 167 | 17 | 0.46 |
| dX | ng/ml | 8.1E-2 | 8.2E-2 | 1.2E-1 | 2.1E-1 | 1.9E-1 | 2.8E-1 | 2.6E-3 | 2.6E-3 | 1.2E0 | 7.6E-1 | 121 | 7 | 43 | 7 | 0.53 |
| eF | ng/ml | 4.0E0 | 4.5E0 | 4.9E0 | 5.8E0 | 4.2E0 | 2.8E0 | 1.2E0 | 2.6E0 | 4.6E1 | 1.2E1 | 302 | 17 | 168 | 17 | 0.65 |
| eC | pg/ml | 3.1E2 | 2.2E2 | 3.6E2 | 4.4E2 | 2.2E2 | 4.2E2 | 9.9E0 | 1.3E2 | 1.4E3 | 1.6E3 | 231 | 15 | 159 | 15 | 0.45 |
| cD | pg/ml | 2.3E2 | 2.0E2 | 5.8E2 | 1.9E2 | 1.2E3 | 7.8E1 | 5.2E-1 | 3.1E1 | 8.3E3 | 3.0E2 | 197 | 16 | 131 | 16 | 0.41 |
| eM | ng/ml | 3.2E0 | 8.0E0 | 4.9E0 | 7.7E0 | 5.5E0 | 5.6E0 | 3.3E-1 | 1.6E0 | 3.9E1 | 1.7E1 | 155 | 7 | 67 | 7 | 0.69 |
| eP | ng/ml | 3.7E-3 | 1.7E-1 | 6.4E-1 | 4.5E-1 | 1.6E0 | 8.0E-1 | 3.7E-3 | 3.7E-3 | 1.2E1 | 2.2E0 | 121 | 7 | 43 | 7 | 0.58 |
| fP | ng/ml | 2.4E2 | 3.1E2 | 2.8E2 | 3.4E2 | 1.7E2 | 1.5E2 | 1.8E0 | 1.1E2 | 1.0E3 | 5.9E2 | 276 | 17 | 162 | 17 | 0.63 |
| fR | ng/ml | 1.2E5 | 2.1E5 | 1.7E5 | 3.0E5 | 1.4E5 | 2.1E5 | 3.1E4 | 1.0E5 | 7.7E5 | 6.3E5 | 295 | 10 | 90 | 10 | 0.71 |
| gL | pg/ml | 6.3E4 | 7.1E4 | 7.0E4 | 7.9E4 | 3.1E4 | 3.3E4 | 1.4E4 | 4.3E4 | 2.0E5 | 1.7E5 | 287 | 17 | 167 | 17 | 0.60 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| gP | U/ml | 2.8E2 | 2.5E2 | 2.9E2 | 2.4E2 | 1.1E2 | 8.0E1 | 1.2E1 | 7.1E1 | 1.1E3 | 3.9E2 | 298 | 17 | 168 | 17 | 0.38 |
| gW | ng/ml | 6.8E2 | 6.8E2 | 1.3E3 | 9.6E2 | 1.7E3 | 1.3E3 | 3.1E-1 | 3.5E1 | 9.5E3 | 5.4E3 | 252 | 16 | 157 | 16 | 0.46 |
| tF | pg/mL | 1.4E3 | 1.9E3 | 1.5E4 | 5.5E3 | 4.5E4 | 7.6E3 | 1.2E1 | 1.8E1 | 3.2E5 | 2.3E4 | 231 | 15 | 159 | 15 | 0.52 |
| hA | ng/ml | 2.0E0 | 2.5E0 | 9.2E0 | 1.1E1 | 3.7E1 | 1.9E1 | 1.7E-2 | 1.7E-2 | 3.5E2 | 6.1E1 | 197 | 16 | 131 | 16 | 0.57 |
| nM | pg/ml | 1.0E-9 | 1.0E-9 | 7.9E1 | 1.0E-9 | 9.0E2 | 0.0E0 | 1.0E-9 | 1.0E-9 | 1.0E4 | 1.0E-9 | 131 | 13 | 103 | 13 | 0.49 |
| nN | pg/ml | 1.2E3 | 1.8E3 | 5.1E3 | 2.1E3 | 2.5E4 | 1.8E3 | 1.1E2 | 1.1E2 | 2.7E5 | 6.2E3 | 131 | 13 | 103 | 13 | 0.55 |
| nO | pg/ml | 2.7E1 | 3.3E1 | 4.3E1 | 6.7E1 | 4.2E1 | 8.3E1 | 3.5E0 | 9.6E0 | 2.4E2 | 3.1E2 | 131 | 13 | 103 | 13 | 0.59 |
| nR | pg/ml | 1.3E1 | 1.9E1 | 3.8E1 | 9.6E1 | 8.7E1 | 1.1E2 | 1.0E-9 | 2.2E0 | 8.2E2 | 3.1E2 | 131 | 13 | 103 | 13 | 0.66 |
| nT | pg/ml | 8.5E1 | 4.3E1 | 2.2E2 | 1.1E2 | 8.0E2 | 1.3E2 | 1.0E-9 | 2.0E1 | 6.6E3 | 4.9E2 | 131 | 13 | 103 | 13 | 0.48 |
| nU | pg/ml | 2.9E1 | 1.2E2 | 2.6E2 | 1.5E2 | 1.5E3 | 1.9E2 | 1.0E-9 | 1.0E-9 | 1.3E4 | 7.5E2 | 131 | 13 | 103 | 13 | 0.71 |
| lW | pg/ml | 1.0E-9 | 1.0E-9 | 1.3E1 | 1.9E1 | 4.7E1 | 4.7E1 | 1.0E-9 | 1.0E-9 | 3.9E2 | 1.6E2 | 131 | 13 | 103 | 13 | 0.51 |
| lX | pg/ml | 1.0E3 | 8.6E2 | 1.1E3 | 9.1E2 | 5.6E2 | 5.1E2 | 1.2E2 | 3.3E2 | 2.6E3 | 1.8E3 | 131 | 13 | 103 | 13 | 0.41 |
| lY | pg/ml | 2.0E1 | 1.8E1 | 2.3E1 | 1.9E1 | 2.1E1 | 9.5E0 | 1.0E-9 | 4.2E0 | 1.4E2 | 3.9E1 | 131 | 13 | 103 | 13 | 0.45 |
| mE | pg/ml | 1.0E-9 | 1.0E-9 | 2.9E0 | 3.4E0 | 8.3E0 | 7.7E0 | 1.0E-9 | 1.0E-9 | 5.8E1 | 2.8E1 | 131 | 13 | 103 | 13 | 0.51 |
| mF | pg/ml | 1.0E-9 | 8.7E-1 | 4.0E0 | 2.8E0 | 2.3E1 | 3.4E0 | 1.0E-9 | 1.0E-9 | 2.5E2 | 9.6E0 | 131 | 13 | 103 | 13 | 0.63 |
| mH | pg/ml | 3.6E0 | 2.7E0 | 5.1E0 | 4.6E0 | 6.7E0 | 5.0E0 | 2.3E-1 | 7.6E-1 | 5.3E1 | 1.8E1 | 131 | 13 | 103 | 13 | 0.46 |
| mI | pg/ml | 1.0E-9 | 1.0E-9 | 1.2E1 | 1.2E1 | 2.7E1 | 2.1E1 | 1.0E-9 | 1.0E-9 | 1.8E2 | 6.1E1 | 131 | 13 | 103 | 13 | 0.47 |
| mM | pg/ml | 2.2E1 | 3.2E1 | 6.5E1 | 9.3E1 | 1.5E2 | 1.3E2 | 1.0E-9 | 1.0E-9 | 1.1E3 | 4.0E2 | 131 | 13 | 103 | 13 | 0.58 |
| mP | pg/ml | 1.4E1 | 1.9E1 | 1.8E1 | 2.6E1 | 2.3E1 | 1.7E1 | 1.0E-9 | 1.3E1 | 1.9E2 | 7.7E1 | 130 | 13 | 102 | 13 | 0.73 |
| mS | pg/ml | 1.8E3 | 2.0E3 | 2.0E3 | 2.0E3 | 1.6E3 | 1.2E3 | 1.0E-9 | 6.6E2 | 1.3E4 | 5.1E3 | 131 | 13 | 103 | 13 | 0.51 |
| mT | pg/ml | 4.8E1 | 3.5E1 | 1.2E2 | 1.6E2 | 2.1E2 | 2.4E2 | 9.7E0 | 1.2E1 | 1.4E3 | 8.0E2 | 130 | 13 | 102 | 13 | 0.48 |
| mU | pg/ml | 2.2E0 | 3.2E0 | 5.5E0 | 5.2E0 | 2.1E1 | 5.6E0 | 1.0E-9 | 1.7E0 | 2.2E2 | 2.2E1 | 130 | 13 | 102 | 13 | 0.66 |
| mW | pg/ml | 2.3E3 | 2.6E3 | 2.6E3 | 3.0E3 | 1.4E3 | 2.3E3 | 3.1E2 | 5.7E2 | 1.0E4 | 9.8E3 | 130 | 13 | 102 | 13 | 0.55 |
| mY | pg/ml | 5.6E2 | 8.9E2 | 8.6E2 | 1.0E3 | 1.3E3 | 7.7E2 | 1.0E-9 | 1.5E2 | 1.1E4 | 2.6E3 | 131 | 13 | 103 | 13 | 0.65 |
| mZ | pg/ml | 2.2E2 | 2.7E2 | 3.8E2 | 4.3E2 | 4.4E2 | 4.8E2 | 1.0E-9 | 1.6E1 | 3.1E3 | 1.5E3 | 130 | 13 | 102 | 13 | 0.49 |
| nA | pg/ml | 2.0E0 | 3.3E0 | 1.1E1 | 1.3E1 | 4.4E1 | 2.2E1 | 1.0E-9 | 1.0E-9 | 4.4E2 | 6.5E1 | 130 | 13 | 102 | 13 | 0.61 |
| nB | pg/ml | 3.0E2 | 4.2E2 | 3.1E2 | 4.2E2 | 1.5E2 | 1.7E2 | 3.0E1 | 1.5E2 | 8.2E2 | 7.8E2 | 131 | 13 | 103 | 13 | 0.69 |
| nC | pg/ml | 1.0E-9 | 1.5E2 | 3.7E3 | 4.9E4 | 3.2E4 | 1.2E5 | 1.0E-9 | 1.0E-9 | 3.7E5 | 3.8E5 | 131 | 13 | 103 | 13 | 0.65 |
| nD | pg/ml | 8.5E0 | 2.0E1 | 3.5E1 | 3.9E1 | 2.0E2 | 7.3E1 | 1.0E-9 | 1.0E-9 | 1.7E3 | 2.6E2 | 130 | 13 | 102 | 13 | 0.64 |
| nF | pg/ml | 1.0E-9 | 1.0E-9 | 5.0E0 | 8.2E0 | 2.6E1 | 1.4E1 | 1.0E-9 | 1.0E-9 | 2.4E2 | 4.7E1 | 131 | 13 | 103 | 13 | 0.63 |
| nH | pg/ml | 3.8E-1 | 5.4E0 | 8.9E1 | 1.0E3 | 8.8E2 | 2.8E3 | 1.0E-9 | 1.0E-9 | 1.0E4 | 1.0E4 | 130 | 13 | 102 | 13 | 0.62 |
| nI | pg/ml | 4.6E1 | 4.6E1 | 1.6E2 | 1.5E2 | 8.4E2 | 3.3E2 | 1.0E-9 | 1.0E-9 | 9.2E3 | 1.2E3 | 131 | 13 | 103 | 13 | 0.54 |
| nJ | pg/ml | 1.7E-1 | 1.1E0 | 4.1E1 | 1.2E1 | 4.5E2 | 3.6E1 | 1.0E-9 | 1.0E-9 | 5.2E3 | 1.3E2 | 131 | 13 | 103 | 13 | 0.62 |
| nK | pg/ml | 1.0E-9 | 2.3E1 | 5.8E1 | 4.3E1 | 3.4E2 | 6.5E1 | 1.0E-9 | 1.0E-9 | 3.2E3 | 2.2E2 | 130 | 13 | 102 | 13 | 0.64 |
| nL | pg/ml | 1.0E-9 | 5.2E0 | 3.9E2 | 1.5E3 | 3.9E3 | 3.8E3 | 1.0E-9 | 1.0E-9 | 4.5E4 | 1.4E4 | 131 | 13 | 103 | 13 | 0.66 |
| wJ | pg/ml | 1.5E5 | 9.6E4 | 1.7E5 | 1.2E5 | 1.0E5 | 5.5E4 | 1.1E4 | 8.4E4 | 5.8E5 | 2.4E5 | 113 | 7 | 91 | 7 | 0.31 |
| wK | pg/ml | 3.5E4 | 6.2E4 | 4.8E4 | 5.5E4 | 5.4E4 | 2.9E4 | 3.7E3 | 1.6E4 | 5.0E5 | 1.0E5 | 113 | 7 | 91 | 7 | 0.62 |
| wL | pg/ml | 3.9E0 | 1.3E0 | 4.1E1 | 7.7E0 | 1.1E2 | 1.1E1 | 1.0E-9 | 1.0E-9 | 8.4E2 | 2.9E1 | 113 | 7 | 91 | 7 | 0.43 |
| wP | pg/ml | 2.6E4 | 2.6E4 | 4.2E4 | 8.5E4 | 4.6E4 | 1.1E5 | 1.1E3 | 2.0E4 | 3.0E5 | 3.0E5 | 113 | 7 | 91 | 7 | 0.61 |
| wQ | pg/ml | 3.7E1 | 4.3E1 | 6.1E1 | 8.0E1 | 8.1E1 | 7.6E1 | 1.0E-9 | 1.7E1 | 5.1E2 | 2.4E2 | 113 | 7 | 91 | 7 | 0.64 |
| hR | pg/ml | 2.6E4 | 2.3E4 | 2.7E4 | 2.5E4 | 1.1E4 | 9.9E3 | 1.1E1 | 1.3E4 | 5.8E4 | 4.5E4 | 189 | 15 | 129 | 15 | 0.43 |
| hV | pg/ml | 4.4E2 | 4.9E2 | 4.7E2 | 4.2E2 | 2.4E2 | 2.0E2 | 6.8E1 | 9.6E1 | 1.5E3 | 7.1E2 | 189 | 15 | 129 | 15 | 0.45 |
| hW | pg/ml | 1.5E3 | 1.9E3 | 2.0E3 | 2.0E3 | 1.6E3 | 9.2E2 | 2.2E2 | 7.1E2 | 1.0E4 | 3.5E3 | 189 | 15 | 129 | 15 | 0.58 |
| hX | pg/ml | 9.0E2 | 1.1E3 | 1.0E3 | 1.1E3 | 7.8E2 | 5.3E2 | 1.3E2 | 2.1E2 | 8.6E3 | 2.2E3 | 189 | 15 | 129 | 15 | 0.57 |
| iA | pg/ml | 1.4E2 | 1.9E2 | 2.8E2 | 3.1E2 | 6.2E2 | 3.0E2 | 5.8E0 | 4.1E1 | 7.1E3 | 9.5E2 | 231 | 15 | 159 | 15 | 0.61 |
| iB | ng/ml | 4.8E0 | 5.5E0 | 6.0E0 | 8.3E0 | 4.9E0 | 7.1E0 | 3.3E-2 | 1.6E0 | 3.8E1 | 2.4E1 | 197 | 16 | 131 | 16 | 0.60 |
| iC | U/ml | 2.1E-1 | 4.9E-1 | 9.0E-1 | 1.1E0 | 4.6E0 | 2.6E0 | 1.0E-9 | 1.0E-9 | 5.5E1 | 1.1E1 | 197 | 16 | 131 | 16 | 0.68 |
| tQ | pg/ml | 1.1E3 | 1.7E3 | 1.2E3 | 1.6E3 | 5.5E2 | 4.0E2 | 2.8E2 | 1.0E3 | 2.5E3 | 2.0E3 | 108 | 7 | 88 | 7 | 0.73 |
| tT | pg/ml | 1.7E1 | 3.0E1 | 2.0E1 | 3.8E1 | 1.5E1 | 2.8E1 | 5.4E0 | 1.3E1 | 1.2E2 | 9.3E1 | 109 | 7 | 89 | 7 | 0.76 |
| tS | pg/ml | 1.1E0 | 7.7E-1 | 1.3E0 | 3.2E0 | 1.3E0 | 4.6E0 | 1.0E-9 | 1.0E-9 | 8.5E0 | 1.0E1 | 109 | 7 | 89 | 7 | 0.47 |
| tX | pg/ml | 9.9E-1 | 2.1E0 | 1.2E0 | 3.7E0 | 9.8E-1 | 3.9E0 | 2.5E-2 | 7.6E-2 | 7.0E0 | 1.0E1 | 109 | 7 | 89 | 7 | 0.66 |
| tO | pg/ml | 4.4E0 | 3.8E0 | 5.1E0 | 7.0E0 | 3.3E0 | 7.1E0 | 1.0E-9 | 8.6E-1 | 1.8E1 | 1.9E1 | 109 | 7 | 89 | 7 | 0.49 |
| tR | pg/ml | 2.1E-1 | 2.5E-1 | 2.9E-1 | 7.9E-1 | 2.9E-1 | 1.1E0 | 1.0E-9 | 3.1E-2 | 1.6E0 | 2.5E0 | 108 | 7 | 88 | 7 | 0.56 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| tU | pg/ml | 9.1E0 | 1.3E1 | 1.1E1 | 1.3E1 | 6.9E0 | 8.5E0 | 2.2E-1 | 1.5E0 | 4.4E1 | 2.5E1 | 111 | 7 | 90 | 7 | 0.59 |
| tN | pg/ml | 1.8E1 | 4.8E1 | 2.3E1 | 6.2E1 | 1.9E1 | 5.9E1 | 1.0E-9 | 6.0E0 | 1.5E2 | 1.6E2 | 109 | 7 | 88 | 7 | 0.63 |
| tV | ng/ml | 4.7E2 | 9.5E2 | 6.4E2 | 1.2E3 | 5.0E2 | 8.6E2 | 5.3E1 | 4.8E2 | 2.9E3 | 3.1E3 | 113 | 7 | 91 | 7 | 0.78 |
| iH | ng/ml | 1.6E5 | 2.0E5 | 1.5E5 | 1.8E5 | 4.7E4 | 6.7E4 | 5.1E4 | 2.9E3 | 2.7E5 | 2.5E5 | 231 | 15 | 159 | 15 | 0.67 |
| iJ | ng/ml | 5.4E4 | 4.3E4 | 5.5E4 | 4.7E4 | 2.9E4 | 3.4E4 | 5.5E3 | 1.8E3 | 2.5E5 | 1.5E5 | 231 | 15 | 159 | 15 | 0.35 |
| hB | ng/ml | 4.1E-1 | 6.2E-1 | 5.0E-1 | 8.0E-1 | 3.3E-1 | 6.8E-1 | 1.0E-9 | 1.2E-1 | 2.3E0 | 2.4E0 | 231 | 15 | 159 | 15 | 0.64 |
| hC | pg/ml | 3.9E3 | 5.2E3 | 6.8E3 | 8.9E3 | 1.0E4 | 1.1E4 | 1.0E-9 | 1.0E-9 | 1.1E5 | 4.3E4 | 231 | 15 | 159 | 15 | 0.55 |
| hF | ng/ml | 1.0E-9 | 1.0E-9 | 2.1E1 | 1.0E-9 | 2.7E2 | 0.0E0 | 1.0E-9 | 1.0E-9 | 4.0E3 | 1.0E-9 | 231 | 15 | 159 | 15 | 0.49 |
| hG | pg/ml | 6.9E3 | 6.9E3 | 7.4E3 | 7.3E3 | 3.2E3 | 2.4E3 | 2.8E1 | 3.7E3 | 1.9E4 | 1.2E4 | 231 | 15 | 159 | 15 | 0.51 |
| iO | ng/ml | 3.8E5 | 3.2E5 | 4.0E5 | 3.3E5 | 1.8E5 | 1.5E5 | 1.1E4 | 6.4E4 | 1.1E6 | 6.7E5 | 231 | 15 | 159 | 15 | 0.40 |
| iP | ng/ml | 5.0E4 | 4.2E4 | 5.5E4 | 4.7E4 | 5.2E4 | 2.5E4 | 1.0E-9 | 3.4E3 | 5.5E5 | 9.7E4 | 231 | 15 | 159 | 15 | 0.48 |
| iZ | ng/ml | 1.6E3 | 1.8E3 | 1.8E3 | 2.0E3 | 7.8E2 | 7.6E2 | 4.7E2 | 1.1E3 | 5.7E3 | 4.1E3 | 230 | 15 | 158 | 15 | 0.59 |
| yD | ng/ml | 1.5E-2 | 1.3E-2 | 1.5E-2 | 1.7E-2 | 6.7E-3 | 7.9E-3 | 1.0E-9 | 6.3E-3 | 4.3E-2 | 2.9E-2 | 113 | 7 | 91 | 7 | 0.54 |
| wB | pg/ml | 7.4E3 | 1.2E4 | 9.4E3 | 1.7E4 | 7.2E3 | 1.3E4 | 1.7E3 | 5.1E3 | 4.1E4 | 4.2E4 | 113 | 7 | 91 | 7 | 0.71 |
| rC | pg/ml | 1.6E3 | 1.5E3 | 2.2E3 | 2.0E3 | 2.2E3 | 1.7E3 | 1.0E-9 | 1.9E2 | 1.5E4 | 7.3E3 | 189 | 16 | 128 | 16 | 0.48 |
| rB | pg/ml | 2.4E1 | 3.5E1 | 4.3E1 | 5.7E1 | 8.9E1 | 6.5E1 | 1.0E-9 | 1.0E-9 | 9.5E2 | 2.5E2 | 189 | 16 | 128 | 16 | 0.60 |
| jD | ng/ml | 3.1E1 | 2.4E1 | 4.8E1 | 3.4E1 | 6.1E1 | 3.3E1 | 1.0E-9 | 1.0E-9 | 5.1E2 | 1.0E2 | 196 | 16 | 131 | 16 | 0.45 |
| jE | ng/ml | 1.0E-9 | 1.0E-9 | 7.1E0 | 6.5E0 | 1.7E1 | 1.3E1 | 1.0E-9 | 1.0E-9 | 1.2E2 | 3.6E1 | 196 | 16 | 131 | 16 | 0.51 |
| jF | ng/ml | 4.2E1 | 3.4E1 | 5.7E1 | 3.8E1 | 6.3E1 | 4.2E1 | 1.0E-9 | 1.0E-9 | 3.5E2 | 1.5E2 | 196 | 16 | 131 | 16 | 0.43 |
| jG | ng/ml | 4.6E3 | 3.7E3 | 4.7E3 | 4.1E3 | 2.0E3 | 2.4E3 | 7.6E2 | 6.7E2 | 1.1E4 | 7.8E3 | 197 | 16 | 131 | 16 | 0.42 |
| jH | ng/ml | 7.6E1 | 6.5E1 | 8.5E1 | 7.4E1 | 4.8E1 | 3.1E1 | 1.3E1 | 4.2E1 | 3.3E2 | 1.3E2 | 197 | 16 | 131 | 16 | 0.44 |
| jI | ng/ml | 6.8E1 | 7.5E1 | 7.3E1 | 8.4E1 | 3.3E1 | 3.5E1 | 1.9E1 | 5.0E1 | 2.5E2 | 1.8E2 | 197 | 16 | 131 | 16 | 0.61 |
| wC | ng/ml | 1.5E0 | 1.5E0 | 1.9E0 | 1.7E0 | 1.7E0 | 1.3E0 | 2.5E-1 | 6.1E-2 | 1.5E1 | 4.3E0 | 113 | 7 | 91 | 7 | 0.48 |
| wD | ng/ml | 1.7E1 | 4.8E1 | 4.8E1 | 6.0E1 | 2.0E2 | 5.4E1 | 2.1E0 | 9.1E0 | 2.1E3 | 1.5E2 | 113 | 7 | 91 | 7 | 0.73 |
| wE | ng/ml | 4.8E1 | 5.0E1 | 4.9E1 | 4.5E1 | 2.3E1 | 1.6E1 | 3.2E0 | 2.0E1 | 1.4E2 | 6.3E1 | 113 | 7 | 91 | 7 | 0.47 |
| wG | ng/ml | 6.4E-2 | 2.3E-2 | 1.0E-1 | 6.0E-2 | 1.3E-1 | 7.4E-2 | 1.0E-9 | 1.0E-9 | 7.1E-1 | 1.8E-1 | 113 | 7 | 91 | 7 | 0.44 |
| wH | ng/ml | 1.8E-2 | 9.5E-2 | 1.5E-1 | 5.4E-1 | 5.2E-1 | 7.4E-1 | 1.0E-9 | 1.0E-9 | 4.2E0 | 1.9E0 | 113 | 7 | 91 | 7 | 0.63 |
| wF | ng/ml | 1.4E-1 | 4.3E-1 | 1.5E0 | 1.4E0 | 7.1E0 | 1.8E0 | 1.0E-9 | 1.0E-9 | 5.7E1 | 4.7E0 | 113 | 7 | 91 | 7 | 0.65 |
| rA | pg/ml | 2.6E1 | 3.8E1 | 3.1E1 | 4.0E1 | 2.5E1 | 2.4E1 | 1.0E-9 | 5.8E0 | 2.0E2 | 9.3E1 | 197 | 17 | 131 | 17 | 0.61 |
| qZ | pg/ml | 4.1E1 | 2.7E1 | 3.7E2 | 1.0E3 | 1.8E3 | 3.1E3 | 1.0E-9 | 5.2E-4 | 1.0E4 | 1.0E4 | 155 | 10 | 118 | 10 | 0.49 |
| qY | pg/ml | 2.6E1 | 2.3E1 | 5.1E1 | 3.3E1 | 6.6E1 | 2.7E1 | 8.7E-1 | 5.1E0 | 5.3E2 | 9.8E1 | 197 | 17 | 131 | 17 | 0.48 |
| qX | pg/ml | 5.9E1 | 8.7E1 | 6.5E1 | 9.5E1 | 4.2E1 | 6.7E1 | 1.0E-9 | 2.9E0 | 2.1E2 | 2.1E2 | 197 | 17 | 131 | 17 | 0.61 |
| qW | pg/ml | 9.2E0 | 1.0E1 | 1.4E1 | 1.1E1 | 1.6E1 | 7.7E0 | 1.0E-9 | 1.0E-9 | 1.2E2 | 2.8E1 | 197 | 17 | 131 | 17 | 0.49 |
| qV | pg/ml | 2.2E3 | 2.2E3 | 2.9E3 | 2.7E3 | 2.1E3 | 1.5E3 | 1.0E2 | 1.7E2 | 1.1E4 | 5.2E3 | 197 | 17 | 131 | 17 | 0.51 |
| qU | pg/ml | 6.1E1 | 1.0E2 | 1.6E2 | 1.3E2 | 2.8E2 | 1.5E2 | 1.0E-9 | 1.0E-9 | 2.1E3 | 5.1E2 | 197 | 17 | 131 | 17 | 0.52 |
| qT | pg/ml | 4.0E1 | 8.7E1 | 6.8E1 | 1.1E2 | 9.8E1 | 1.0E2 | 1.0E-9 | 1.2E1 | 9.0E2 | 4.4E2 | 197 | 17 | 131 | 17 | 0.67 |
| jK | ng/ml | 1.6E3 | 1.7E3 | 1.7E3 | 1.7E3 | 6.5E2 | 5.0E2 | 2.8E2 | 8.7E2 | 4.3E3 | 2.4E3 | 197 | 16 | 131 | 16 | 0.52 |
| jL | ng/ml | 1.9E2 | 2.3E2 | 2.8E2 | 3.4E2 | 2.5E2 | 3.8E2 | 3.6E1 | 9.8E1 | 2.1E3 | 1.7E3 | 197 | 16 | 131 | 16 | 0.57 |
| jM | ng/ml | 7.4E4 | 6.7E4 | 7.8E4 | 7.5E4 | 3.9E4 | 4.2E4 | 3.9E2 | 1.1E4 | 1.9E5 | 1.6E5 | 197 | 16 | 131 | 16 | 0.48 |
| jO | pg/ml | 2.1E5 | 2.2E5 | 2.6E5 | 2.7E5 | 1.5E5 | 1.8E5 | 5.2E4 | 1.2E5 | 1.1E6 | 8.1E5 | 197 | 16 | 131 | 16 | 0.50 |
| jP | pg/ml | 2.2E5 | 2.4E5 | 2.6E5 | 2.5E5 | 1.6E5 | 1.1E5 | 3.6E4 | 7.8E4 | 9.2E5 | 5.0E5 | 197 | 16 | 131 | 16 | 0.53 |
| jQ | pg/ml | 2.7E3 | 2.7E3 | 3.8E3 | 3.0E3 | 3.5E3 | 2.0E3 | 1.0E-9 | 2.9E2 | 1.8E4 | 7.1E3 | 197 | 16 | 131 | 16 | 0.49 |
| jR | pg/ml | 7.7E3 | 7.0E3 | 1.2E4 | 9.7E3 | 1.3E4 | 9.5E3 | 1.0E-9 | 2.9E2 | 9.0E4 | 3.4E4 | 197 | 16 | 131 | 16 | 0.47 |
| jT | pg/ml | 1.8E5 | 1.7E5 | 1.8E5 | 1.7E5 | 6.6E4 | 6.0E4 | 6.8E4 | 8.8E4 | 4.5E5 | 3.1E5 | 197 | 16 | 131 | 16 | 0.44 |
| jU | mIU/ml | 4.6E0 | 2.6E0 | 1.1E1 | 7.3E0 | 1.7E1 | 1.1E1 | 6.2E-2 | 6.3E-2 | 1.1E2 | 4.3E1 | 197 | 16 | 131 | 16 | 0.40 |
| jV | mIU/ml | 1.5E0 | 1.7E0 | 3.8E0 | 3.4E0 | 6.4E0 | 4.9E0 | 1.7E-3 | 1.1E-3 | 3.5E1 | 1.8E1 | 197 | 16 | 131 | 16 | 0.46 |
| jY | ng/ml | 5.9E-4 | 2.7E-3 | 5.8E-3 | 9.8E-3 | 2.7E-2 | 2.3E-2 | 1.0E-9 | 1.0E-9 | 3.0E-1 | 9.4E-2 | 197 | 16 | 131 | 16 | 0.59 |
| kC | pg/ml | 9.7E1 | 1.0E2 | 1.8E2 | 3.1E2 | 3.8E2 | 7.3E2 | 2.1E1 | 3.6E1 | 3.5E3 | 2.7E3 | 131 | 13 | 103 | 13 | 0.48 |
| kE | pg/ml | 1.3E5 | 1.2E5 | 1.3E5 | 1.2E5 | 3.8E4 | 4.1E4 | 1.2E4 | 3.8E4 | 2.3E5 | 1.9E5 | 131 | 13 | 103 | 13 | 0.44 |
| kF | pg/mL | 6.0E1 | 6.5E1 | 6.8E1 | 7.0E1 | 4.8E1 | 2.5E1 | 2.6E1 | 4.0E1 | 5.1E2 | 1.3E2 | 131 | 13 | 103 | 13 | 0.57 |
| kG | pg/mL | 9.2E3 | 8.4E3 | 1.2E4 | 1.3E4 | 1.4E4 | 1.4E4 | 7.5E2 | 3.2E3 | 1.2E5 | 5.8E4 | 131 | 13 | 103 | 13 | 0.51 |
| kI | pg/ml | 1.8E2 | 2.0E2 | 2.2E2 | 2.6E2 | 1.3E2 | 1.4E2 | 4.4E1 | 8.8E1 | 8.7E2 | 5.5E2 | 131 | 13 | 103 | 13 | 0.58 |
| kK | pg/ml | 1.0E2 | 1.2E2 | 1.6E2 | 2.3E2 | 1.9E2 | 2.6E2 | 6.4E0 | 5.1E1 | 1.6E3 | 9.1E2 | 131 | 13 | 103 | 13 | 0.59 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|---------|-----|------|
|      |       | NonDis | Dis | NonDis | Dis | NonDis | Dis | NonDis | Dis | NonDis | Dis | NonDis | Dis | NonDis | Dis |      |
| kN | pg/ml | 9.5E2 | 8.8E2 | 2.0E3 | 1.1E3 | 5.2E3 | 9.4E2 | 7.6E1 | 9.5E1 | 5.5E4 | 3.8E3 | 131 | 13 | 103 | 13 | 0.47 |
| kO | pg/ml | 7.2E3 | 6.9E3 | 8.6E3 | 1.9E4 | 1.1E4 | 3.9E4 | 3.4E3 | 3.3E3 | 1.3E5 | 1.5E5 | 131 | 13 | 103 | 13 | 0.49 |
| kP | pg/ml | 6.3E3 | 5.4E3 | 7.5E3 | 6.4E3 | 6.2E3 | 3.5E3 | 8.6E2 | 1.5E3 | 4.8E4 | 1.3E4 | 131 | 13 | 103 | 13 | 0.48 |
| kQ | pg/ml | 4.1E3 | 4.3E3 | 4.9E3 | 4.4E3 | 3.0E3 | 1.4E3 | 5.6E2 | 2.5E3 | 2.5E4 | 7.0E3 | 231 | 15 | 159 | 15 | 0.50 |
| kR | pg/ml | 2.1E1 | 2.0E1 | 3.1E1 | 2.8E1 | 6.9E1 | 2.1E1 | 1.0E-9 | 5.6E0 | 1.0E3 | 7.7E1 | 231 | 15 | 159 | 15 | 0.50 |
| kS | pg/ml | 8.0E2 | 1.0E3 | 9.7E2 | 1.0E3 | 1.0E3 | 4.5E2 | 8.2E1 | 2.5E2 | 1.4E4 | 1.7E3 | 231 | 15 | 159 | 15 | 0.61 |
| rZ | ng/ml | 1.0E-9 | 5.7E-3 | 5.4E-3 | 2.6E-2 | 1.5E-2 | 7.4E-2 | 1.0E-9 | 1.0E-9 | 1.1E-1 | 3.0E-1 | 190 | 16 | 126 | 16 | 0.65 |
| rY | ng/ml | 5.7E-2 | 5.1E-2 | 3.6E-1 | 1.2E0 | 2.3E0 | 4.4E0 | 1.0E-9 | 1.0E-9 | 2.3E1 | 1.8E1 | 190 | 16 | 126 | 16 | 0.55 |
| rX | ng/ml | 1.0E-9 | 1.0E-9 | 6.9E-2 | 1.7E-1 | 4.2E-1 | 6.0E-1 | 1.0E-9 | 1.0E-9 | 3.7E0 | 2.4E0 | 190 | 16 | 126 | 16 | 0.54 |
| lK | pg/ml | 9.9E1 | 4.0E1 | 1.9E2 | 9.8E1 | 3.1E2 | 1.4E2 | 1.0E-9 | 1.0E-9 | 3.3E3 | 5.0E2 | 196 | 16 | 131 | 16 | 0.36 |
| lL | pg/ml | 1.6E3 | 1.1E3 | 2.6E3 | 1.7E3 | 3.7E3 | 1.8E3 | 1.5E1 | 3.8E2 | 4.2E4 | 7.7E3 | 197 | 16 | 131 | 16 | 0.40 |
| lM | pg/ml | 1.1E3 | 1.2E3 | 3.5E3 | 5.0E3 | 7.5E3 | 7.7E3 | 1.2E2 | 2.8E2 | 5.1E4 | 2.7E4 | 197 | 16 | 131 | 16 | 0.58 |
| lN | pg/ml | 1.0E-9 | 1.0E-9 | 4.2E0 | 3.8E0 | 1.5E1 | 6.0E0 | 1.0E-9 | 1.0E-9 | 1.5E2 | 2.0E1 | 197 | 16 | 131 | 16 | 0.53 |
| lO | pg/ml | 1.0E-9 | 1.0E-9 | 1.6E0 | 1.0E-9 | 1.1E1 | 0.0E0 | 1.0E-9 | 1.0E-9 | 1.3E2 | 1.0E-9 | 196 | 16 | 131 | 16 | 0.48 |
| nW | pg/ml | 1.1E5 | 1.1E5 | 1.1E5 | 1.2E5 | 2.5E4 | 3.5E4 | 5.8E4 | 6.7E4 | 1.8E5 | 1.7E5 | 231 | 15 | 159 | 15 | 0.52 |
| nY | pg/ml | 2.1E3 | 2.0E3 | 2.4E3 | 2.4E3 | 1.6E3 | 1.4E3 | 5.1E2 | 7.6E2 | 1.3E4 | 5.0E3 | 231 | 15 | 159 | 15 | 0.50 |
| oO | pg/ml | 7.8E4 | 1.2E5 | 1.1E5 | 1.5E5 | 9.7E4 | 8.2E4 | 1.5E4 | 5.1E4 | 6.2E5 | 2.8E5 | 123 | 12 | 97 | 12 | 0.69 |
| oP | pg/ml | 1.2E5 | 2.0E5 | 1.4E5 | 2.2E5 | 9.1E4 | 1.2E5 | 2.4E4 | 7.8E4 | 4.5E5 | 4.8E5 | 123 | 12 | 97 | 12 | 0.72 |
| oQ | pg/ml | 2.8E3 | 4.4E3 | 3.5E3 | 5.4E3 | 2.8E3 | 3.2E3 | 9.3E2 | 1.9E3 | 2.1E4 | 1.2E4 | 123 | 12 | 97 | 12 | 0.72 |
| oE | pg/ml | 1.3E2 | 1.4E2 | 3.6E2 | 5.4E2 | 5.4E2 | 8.4E2 | 1.0E-9 | 2.7E1 | 4.7E3 | 2.8E3 | 231 | 15 | 159 | 15 | 0.52 |
| oF | pg/ml | 7.7E3 | 1.3E4 | 2.0E4 | 2.6E4 | 3.6E4 | 3.2E4 | 6.4E1 | 4.6E2 | 2.5E5 | 1.1E5 | 231 | 15 | 159 | 15 | 0.55 |
| oH | pg/ml | 4.4E1 | 2.7E1 | 9.3E1 | 6.7E1 | 1.4E2 | 8.0E1 | 4.2E0 | 4.3E-1 | 9.9E2 | 2.6E2 | 231 | 15 | 159 | 15 | 0.40 |
| oK | pg/ml | 7.6E2 | 1.3E3 | 1.8E3 | 1.5E3 | 2.5E3 | 1.6E3 | 5.2E1 | 1.8E2 | 1.8E4 | 6.8E3 | 231 | 15 | 159 | 15 | 0.54 |
| oN | pg/ml | 5.0E2 | 5.8E2 | 7.5E2 | 6.9E2 | 1.4E3 | 4.3E2 | 1.5E2 | 2.2E2 | 1.8E4 | 1.9E3 | 231 | 15 | 159 | 15 | 0.57 |
| pF | pg/ml | 4.5E-1 | 3.8E-1 | 1.0E0 | 1.3E0 | 5.7E0 | 2.1E0 | 1.0E-9 | 1.0E-9 | 8.7E1 | 7.8E0 | 231 | 15 | 159 | 15 | 0.54 |

Figure 19.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.0E1 | 5.6E1 | 8.2E1 | 5.6E1 | 5.8E1 | 3.2E1 | 1.0E0 | 1.6E1 | 4.8E2 | 1.1E2 | 1777 | 11 | 298 | 11 | 0.38 |
| Ad | ug/mL | 3.8E-2 | 6.2E-2 | 9.1E-2 | 7.3E-2 | 3.8E-1 | 6.1E-2 | 2.7E-4 | 9.4E-3 | 8.5E0 | 1.9E-1 | 511 | 7 | 198 | 7 | 0.58 |
| Af | ng/mL | 1.2E0 | 3.6E-1 | 1.8E1 | 2.2E0 | 6.6E1 | 5.1E0 | 1.7E-3 | 1.7E-3 | 5.3E2 | 1.4E1 | 511 | 7 | 198 | 7 | 0.32 |
| Aj | ug/mL | 1.5E0 | 7.6E-1 | 2.6E0 | 1.3E0 | 2.5E0 | 1.4E0 | 1.5E-3 | 3.2E-2 | 6.1E0 | 3.5E0 | 511 | 7 | 198 | 7 | 0.39 |
| Al | mg/mL | 8.8E-5 | 8.5E-5 | 2.4E-4 | 1.3E-4 | 4.0E-4 | 1.4E-4 | 2.3E-6 | 2.2E-5 | 2.2E-3 | 4.4E-4 | 511 | 7 | 198 | 7 | 0.50 |
| An | U/mL | 5.0E1 | 2.9E2 | 1.9E2 | 3.7E2 | 5.6E2 | 4.5E2 | 9.8E-4 | 7.4E0 | 7.8E3 | 1.3E3 | 511 | 7 | 198 | 7 | 0.69 |
| Ao | pg/mL | 9.0E1 | 8.9E1 | 4.7E2 | 1.4E2 | 3.2E2 | 2.1E2 | 1.5E0 | 7.8E0 | 3.9E4 | 6.0E2 | 511 | 7 | 198 | 7 | 0.42 |
| Ap | ng/mL | 3.2E1 | 4.7E1 | 4.7E1 | 4.8E1 | 4.9E1 | 2.9E1 | 8.4E-5 | 6.8E0 | 3.3E2 | 9.7E1 | 511 | 7 | 198 | 7 | 0.59 |
| Ar | ng/mL | 9.8E-1 | 5.9E0 | 1.1E1 | 6.4E0 | 1.8E2 | 4.9E0 | 3.4E-3 | 1.7E-1 | 4.1E3 | 1.5E1 | 511 | 7 | 198 | 7 | 0.76 |
| As | ng/mL | 8.7E-3 | 1.0E-2 | 1.5E-2 | 9.4E-3 | 5.7E-2 | 5.7E-3 | 1.7E-3 | 1.7E-3 | 1.2E0 | 1.5E-2 | 511 | 7 | 198 | 7 | 0.51 |
| Aw | pg/mL | 1.6E1 | 1.8E1 | 1.6E1 | 2.0E1 | 6.3E0 | 7.5E0 | 2.9E-2 | 1.1E1 | 5.1E1 | 3.0E1 | 511 | 7 | 198 | 7 | 0.64 |
| Ax | ng/mL | 2.1E0 | 8.1E0 | 1.5E1 | 3.9E1 | 6.0E1 | 7.2E1 | 1.2E-2 | 3.9E-1 | 7.7E2 | 2.0E2 | 511 | 7 | 198 | 7 | 0.67 |
| Ba | ng/mL | 6.2E1 | 5.8E2 | 4.4E2 | 2.4E3 | 1.1E3 | 3.2E3 | 2.7E-1 | 1.1E1 | 8.1E3 | 8.1E3 | 511 | 7 | 198 | 7 | 0.70 |
| Bb | ng/mL | 3.2E0 | 6.3E0 | 6.6E0 | 6.9E0 | 1.4E1 | 6.5E0 | 4.1E-3 | 4.1E-3 | 2.5E2 | 1.9E1 | 511 | 7 | 198 | 7 | 0.58 |
| Bc | ng/mL | 3.8E1 | 8.9E1 | 1.1E2 | 1.5E2 | 1.9E2 | 1.3E2 | 1.1E-1 | 9.6E0 | 1.2E3 | 3.6E2 | 511 | 7 | 198 | 7 | 0.70 |
| Bg | ng/mL | 8.1E-2 | 6.2E-1 | 5.3E0 | 1.7E0 | 2.9E1 | 2.2E0 | 5.3E-4 | 3.1E-2 | 4.4E2 | 5.3E0 | 511 | 7 | 198 | 7 | 0.62 |
| Bn | ng/mL | 5.6E-2 | 5.6E-2 | 1.4E0 | 4.6E-1 | 3.2E0 | 1.0E0 | 5.6E-2 | 5.6E-2 | 5.8E1 | 2.8E0 | 511 | 7 | 198 | 7 | 0.38 |
| Bo | ng/mL | 1.2E1 | 2.7E1 | 1.4E1 | 2.9E1 | 1.8E1 | 1.8E1 | 1.6E-2 | 1.6E-2 | 2.8E2 | 5.3E1 | 511 | 7 | 198 | 7 | 0.78 |
| Ch | uIU/mL | 9.8E-1 | 9.2E-1 | 1.7E1 | 1.5E0 | 9.6E1 | 1.7E0 | 3.4E-3 | 1.2E-1 | 1.8E3 | 5.2E0 | 511 | 7 | 198 | 7 | 0.44 |
| Co | pg/mL | 3.7E1 | 5.5E1 | 1.7E2 | 8.1E1 | 9.1E2 | 7.9E1 | 1.5E-1 | 8.1E0 | 1.7E4 | 2.1E2 | 511 | 7 | 198 | 7 | 0.56 |
| Cp | ng/mL | 2.2E1 | 3.3E1 | 3.0E1 | 4.7E1 | 6.3E1 | 3.2E1 | 6.0E-1 | 8.6E0 | 1.3E3 | 8.4E1 | 511 | 7 | 198 | 7 | 0.69 |
| Cq | ng/mL | 3.0E-2 | 3.9E-2 | 2.3E-1 | 4.3E-2 | 2.3E0 | 3.3E-2 | 8.0E-4 | 8.0E-4 | 4.9E1 | 1.0E-1 | 511 | 7 | 198 | 7 | 0.54 |
| Cs | ng/mL | 6.6E1 | 2.7E2 | 3.2E2 | 1.1E3 | 1.1E3 | 1.9E3 | 2.7E-2 | 5.8E0 | 1.8E4 | 5.3E3 | 511 | 7 | 198 | 7 | 0.65 |
| Ct | ng/mL | 6.0E-1 | 3.5E-1 | 3.7E1 | 2.0E1 | 1.1E2 | 2.8E1 | 1.1E-4 | 6.8E-2 | 6.2E2 | 7.2E1 | 511 | 7 | 198 | 7 | 0.55 |
| Cu | ng/mL | 2.4E-1 | 6.8E-1 | 5.3E-1 | 8.3E-1 | 3.0E0 | 7.6E-1 | 9.0E-5 | 9.8E-2 | 6.6E1 | 2.4E0 | 511 | 7 | 198 | 7 | 0.72 |
| Cv | ng/mL | 5.6E0 | 6.5E0 | 2.6E1 | 7.5E1 | 6.5E1 | 1.7E2 | 1.4E-4 | 5.7E-2 | 5.3E2 | 4.7E2 | 511 | 7 | 198 | 7 | 0.50 |
| Cw | mIU/mL | 3.0E-2 | 4.3E-2 | 5.1E-2 | 4.0E-2 | 3.0E-1 | 1.8E-2 | 1.5E-4 | 3.5E-3 | 6.8E0 | 5.7E-2 | 511 | 7 | 198 | 7 | 0.58 |
| Cx | ng/mL | 4.6E-1 | 1.3E-2 | 6.3E1 | 4.1E1 | 1.1E2 | 1.1E2 | 9.3E-5 | 9.3E-5 | 3.9E2 | 2.8E2 | 511 | 7 | 198 | 7 | 0.38 |
| Db | ug/mL | 7.4E0 | 8.6E0 | 9.2E0 | 1.1E1 | 1.0E1 | 9.0E0 | 4.5E-1 | 1.3E0 | 1.4E2 | 2.9E1 | 511 | 7 | 198 | 7 | 0.57 |
| Dc | nmol/L | 1.9E-2 | 3.2E-2 | 8.4E-2 | 3.6E-1 | 6.3E-1 | 8.2E-1 | 5.2E-6 | 6.3E-3 | 1.4E1 | 2.2E0 | 511 | 7 | 198 | 7 | 0.61 |
| Dd | ug/mL | 7.5E-2 | 2.0E-2 | 1.8E-1 | 8.1E-2 | 2.9E-1 | 1.1E-1 | 8.3E-5 | 3.2E-3 | 3.6E0 | 2.5E-1 | 511 | 7 | 198 | 7 | 0.38 |
| De | ng/mL | 3.4E-3 | 3.4E-3 | 8.0E-2 | 6.5E-2 | 1.4E-1 | 8.1E-2 | 3.4E-3 | 3.4E-3 | 9.2E-1 | 2.0E-1 | 511 | 7 | 198 | 7 | 0.52 |
| Dg | ng/mL | 3.2E1 | 5.9E1 | 4.5E1 | 4.7E1 | 4.1E1 | 2.7E1 | 1.0E-1 | 1.1E1 | 1.9E2 | 7.6E1 | 511 | 7 | 198 | 7 | 0.58 |
| Di | pg/mL | 1.8E0 | 2.2E0 | 2.2E0 | 2.3E0 | 2.1E0 | 1.6E0 | 1.8E-1 | 1.8E-1 | 1.3E1 | 4.4E0 | 511 | 7 | 198 | 7 | 0.55 |
| Dk | uIU/mL | 1.6E-2 | 1.8E-2 | 8.3E-2 | 1.2E-1 | 4.8E-1 | 2.3E-1 | 1.1E-4 | 1.1E-4 | 8.9E0 | 6.3E-1 | 511 | 7 | 198 | 7 | 0.59 |
| Dl | ng/mL | 2.4E2 | 2.3E2 | 3.2E2 | 3.0E2 | 3.0E2 | 2.5E2 | 1.7E0 | 5.9E1 | 1.6E3 | 7.8E2 | 511 | 7 | 198 | 7 | 0.52 |
| Dp | ng/ml | 2.4E0 | 1.8E0 | 6.0E0 | 6.3E0 | 1.4E1 | 9.4E0 | 3.7E-3 | 1.9E-1 | 2.0E2 | 2.6E1 | 315 | 7 | 189 | 7 | 0.48 |
| Ef | ng/ml | 1.3E-1 | 5.6E-1 | 8.9E-1 | 1.1E0 | 1.9E0 | 1.4E0 | 5.7E-4 | 2.1E-2 | 1.0E1 | 3.9E0 | 379 | 7 | 195 | 7 | 0.62 |
| Wm | % | 7.0E-1 | 1.3E0 | 3.4E1 | 1.5E2 | 1.8E2 | 4.2E2 | 5.4E-2 | 8.5E-2 | 2.4E3 | 1.3E3 | 416 | 9 | 214 | 9 | 0.57 |
| Ed | pg/ml | 5.2E-1 | 1.1E1 | 5.6E1 | 4.7E1 | 4.1E2 | 6.9E1 | 5.2E-1 | 5.2E-1 | 7.3E3 | 1.6E2 | 315 | 7 | 188 | 7 | 0.61 |
| Tj | pg/mL | 3.7E-1 | 3.7E-1 | 5.1E1 | 8.7E0 | 2.8E2 | 1.7E1 | 3.6E-1 | 3.7E-1 | 3.5E3 | 4.4E1 | 378 | 7 | 198 | 7 | 0.46 |
| Po | pg/ml | 6.9E-1 | 3.3E0 | 8.6E0 | 1.0E1 | 2.4E1 | 1.3E1 | 8.0E-3 | 2.6E-2 | 2.7E2 | 3.1E1 | 899 | 13 | 335 | 13 | 0.58 |
| Et | ng/ml | 1.4E3 | 2.0E3 | 1.7E3 | 2.2E3 | 1.2E3 | 1.5E3 | 7.5E1 | 5.0E2 | 5.0E3 | 5.0E3 | 898 | 13 | 335 | 13 | 0.60 |
| Fa | ng/ml | 4.4E1 | 8.9E1 | 1.3E2 | 1.2E2 | 5.2E2 | 8.2E1 | 3.4E-2 | 3.4E0 | 8.0E3 | 2.3E2 | 311 | 7 | 186 | 7 | 0.69 |
| Ez | ng/ml | 4.1E0 | 7.4E0 | 1.7E1 | 2.7E1 | 5.0E1 | 3.3E1 | 1.3E-2 | 3.8E-1 | 7.1E2 | 8.9E1 | 315 | 7 | 189 | 7 | 0.64 |
| Fb | ng/ml | 2.5E1 | 3.5E1 | 2.3E1 | 3.2E1 | 1.1E1 | 1.3E1 | 5.9E-1 | 4.0E0 | 5.7E1 | 4.3E1 | 312 | 7 | 186 | 7 | 0.74 |
| Fn | ng/ml | 2.1E-1 | 2.1E-1 | 5.8E0 | 1.8E0 | 2.5E1 | 2.3E0 | 1.1E-14 | 1.1E-14 | 4.2E2 | 5.3E0 | 315 | 7 | 189 | 7 | 0.43 |
| Fp | ng/ml | 1.4E1 | 2.4E1 | 2.5E1 | 3.8E1 | 2.9E1 | 4.6E1 | 6.0E-3 | 1.3E-1 | 1.4E2 | 1.4E2 | 931 | 13 | 336 | 13 | 0.54 |
| Fr | ng/ml | 3.7E4 | 1.1E5 | 1.2E5 | 2.9E5 | 1.8E5 | 3.0E5 | 1.9E2 | 4.5E3 | 9.0E5 | 7.4E5 | 1043 | 13 | 340 | 13 | 0.69 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|-----|---------|-----|-----|
| | | NonDis | Dis | NonDis | Dis | NonDis | Dis | NonDis | Dis | NonDis | Dis | NonDis | Dis | NonDis | Dis | |
| Fw | pg/ml | 1.2E0 | 9.8E0 | 5.8E1 | 1.5E1 | 4.5E2 | 1.7E1 | 1.1E-14 | 1.2E-1 | 6.9E3 | 3.9E1 | 380 | 7 | 196 | 7 | 0.60 |
| Gl | pg/ml | 7.2E3 | 8.6E3 | 1.1E4 | 1.2E4 | 9.2E3 | 1.2E4 | 9.1E1 | 4.9E2 | 3.4E4 | 3.1E4 | 370 | 7 | 194 | 7 | 0.51 |
| Gp | U/ml | 1.6E0 | 4.7E-1 | 4.0E0 | 8.9E-1 | 6.7E0 | 1.2E0 | 1.3E-3 | 1.5E-2 | 6.7E1 | 3.2E0 | 382 | 7 | 196 | 7 | 0.30 |
| Ha | ng/ml | 2.7E0 | 3.0E0 | 9.6E0 | 3.5E0 | 2.1E1 | 3.1E0 | 6.4E-3 | 1.7E-2 | 1.3E2 | 9.0E0 | 313 | 7 | 188 | 7 | 0.47 |
| Nm | pg/ml | 1.4E4 | 2.0E4 | 3.3E4 | 2.6E4 | 8.2E4 | 3.1E4 | 1.0E-9 | 1.0E-9 | 1.6E6 | 1.0E5 | 902 | 13 | 337 | 13 | 0.48 |
| Nn | pg/ml | 1.6E2 | 4.9E2 | 1.9E3 | 1.7E3 | 8.2E3 | 2.4E3 | 1.0E-9 | 1.0E-9 | 1.0E5 | 7.7E3 | 902 | 13 | 337 | 13 | 0.57 |
| No | pg/ml | 1.6E1 | 1.5E1 | 3.8E1 | 9.8E1 | 1.1E2 | 2.1E2 | 1.0E-9 | 6.4E-1 | 2.5E3 | 7.7E2 | 902 | 13 | 337 | 13 | 0.53 |
| Nq | pg/ml | 2.0E0 | 5.8E0 | 2.0E1 | 1.9E1 | 7.6E1 | 3.2E1 | 1.0E-9 | 1.0E-9 | 1.2E3 | 9.9E1 | 902 | 13 | 337 | 13 | 0.56 |
| Nr | pg/ml | 1.2E0 | 1.0E-9 | 2.9E1 | 1.6E1 | 1.8E2 | 2.8E1 | 1.0E-9 | 1.0E-9 | 4.1E3 | 8.7E1 | 902 | 13 | 337 | 13 | 0.49 |
| Ns | pg/ml | 1.0E-9 | 1.0E-9 | 9.2E0 | 5.5E-1 | 5.2E1 | 1.3E0 | 1.0E-9 | 1.0E-9 | 1.1E3 | 3.6E0 | 902 | 13 | 337 | 13 | 0.52 |
| Nt | pg/ml | 1.0E2 | 1.5E2 | 1.4E2 | 1.6E2 | 1.2E2 | 9.1E1 | 1.0E-9 | 5.4E1 | 1.7E3 | 3.7E2 | 902 | 13 | 337 | 13 | 0.62 |
| Nu | pg/ml | 2.0E1 | 5.4E1 | 5.5E1 | 9.0E1 | 9.0E1 | 9.3E1 | 1.0E-9 | 1.0E-9 | 8.9E2 | 2.9E2 | 902 | 13 | 337 | 13 | 0.63 |
| Lu | pg/ml | 1.0E4 | 6.8E3 | 1.8E4 | 9.8E3 | 6.2E4 | 8.8E3 | 3.5E2 | 2.9E3 | 1.3E6 | 3.1E4 | 905 | 13 | 337 | 13 | 0.38 |
| Lv | pg/ml | 1.0E-9 | 2.2E1 | 1.1E1 | 4.3E1 | 2.2E1 | 5.5E1 | 1.0E-9 | 1.0E-9 | 2.6E2 | 1.6E2 | 905 | 13 | 337 | 13 | 0.66 |
| Lw | pg/ml | 1.0E-9 | 1.0E-9 | 3.7E-1 | 1.3E0 | 3.8E0 | 4.6E0 | 1.0E-9 | 1.0E-9 | 8.0E1 | 1.7E1 | 905 | 13 | 337 | 13 | 0.53 |
| Lx | pg/ml | 1.0E-9 | 2.2E2 | 1.7E2 | 4.4E2 | 8.6E2 | 6.7E2 | 1.0E-9 | 1.0E-9 | 2.2E4 | 2.3E3 | 905 | 13 | 337 | 13 | 0.69 |
| Ly | pg/ml | 1.0E-9 | 2.7E0 | 1.0E1 | 9.2E0 | 2.0E1 | 1.2E1 | 1.0E-9 | 1.0E-9 | 1.6E2 | 4.0E1 | 905 | 13 | 337 | 13 | 0.59 |
| Lz | pg/ml | 1.0E-9 | 1.0E-9 | 4.0E0 | 1.0E-9 | 3.0E1 | 0.0E0 | 1.0E-9 | 1.0E-9 | 6.0E2 | 1.0E-9 | 905 | 13 | 337 | 13 | 0.46 |
| Ma | pg/ml | 2.9E2 | 5.3E2 | 1.4E3 | 1.3E3 | 3.6E3 | 1.8E3 | 1.0E-9 | 2.0E1 | 6.5E4 | 6.8E3 | 905 | 13 | 337 | 13 | 0.58 |
| Mb | pg/ml | 2.5E1 | 4.3E1 | 3.1E1 | 4.0E1 | 1.5E1 | 1.7E1 | 4.1E0 | 1.9E1 | 2.1E2 | 7.3E1 | 905 | 13 | 337 | 13 | 0.64 |
| Mc | pg/ml | 1.0E-9 | 1.0E-9 | 3.8E-2 | 1.9E-1 | 5.7E-1 | 6.9E-1 | 1.0E-9 | 1.0E-9 | 1.3E1 | 2.5E0 | 905 | 13 | 337 | 13 | 0.53 |
| Md | pg/ml | 1.0E-9 | 1.0E-9 | 4.4E-1 | 4.6E-1 | 3.6E0 | 1.6E0 | 1.0E-9 | 1.0E-9 | 6.8E1 | 5.9E0 | 905 | 13 | 337 | 13 | 0.50 |
| Me | pg/ml | 3.3E1 | 3.0E1 | 3.2E1 | 2.9E1 | 2.0E1 | 1.1E1 | 1.0E-9 | 9.4E0 | 3.2E2 | 4.4E1 | 905 | 13 | 337 | 13 | 0.44 |
| Mf | pg/ml | 1.0E-9 | 1.0E-9 | 5.4E-1 | 1.7E-1 | 2.9E0 | 6.0E-1 | 1.0E-9 | 1.0E-9 | 5.6E1 | 2.2E0 | 905 | 13 | 337 | 13 | 0.46 |
| Mg | pg/ml | 1.6E0 | 3.5E0 | 7.4E0 | 6.4E0 | 1.2E1 | 1.1E1 | 1.0E-9 | 1.0E-9 | 9.2E1 | 4.0E1 | 905 | 13 | 337 | 13 | 0.50 |
| Mh | pg/ml | 1.0E-9 | 1.0E-9 | 1.3E0 | 2.0E0 | 9.6E0 | 4.1E0 | 1.0E-9 | 1.0E-9 | 1.6E2 | 1.1E1 | 905 | 13 | 337 | 13 | 0.53 |
| Mi | pg/ml | 1.0E-9 | 1.0E-9 | 9.0E-1 | 2.4E0 | 1.2E1 | 8.6E0 | 1.0E-9 | 1.0E-9 | 3.2E2 | 3.1E1 | 905 | 13 | 337 | 13 | 0.53 |
| Mj | pg/ml | 1.0E-9 | 1.0E-9 | 4.5E0 | 1.0E1 | 2.5E1 | 3.0E1 | 1.0E-9 | 1.0E-9 | 4.6E2 | 1.1E2 | 905 | 13 | 337 | 13 | 0.56 |
| Mk | pg/ml | 1.0E0 | 1.0E-9 | 1.3E1 | 5.6E0 | 8.5E1 | 1.0E1 | 1.0E-9 | 1.0E-9 | 1.7E3 | 3.6E1 | 905 | 13 | 337 | 13 | 0.47 |
| Ml | pg/ml | 1.0E-9 | 1.0E-9 | 5.6E0 | 2.0E0 | 7.5E1 | 4.8E0 | 1.0E-9 | 1.0E-9 | 2.1E3 | 1.4E1 | 905 | 13 | 337 | 13 | 0.49 |
| Mm | pg/ml | 6.1E2 | 5.3E2 | 1.1E3 | 8.5E2 | 1.5E3 | 7.8E2 | 1.0E-9 | 4.4E1 | 1.2E4 | 2.2E3 | 905 | 13 | 337 | 13 | 0.50 |
| Mn | pg/ml | 5.6E0 | 8.4E0 | 1.0E1 | 9.9E0 | 2.3E1 | 1.1E1 | 1.0E-9 | 1.0E-9 | 3.5E2 | 4.2E1 | 905 | 13 | 337 | 13 | 0.56 |
| Mp | pg/ml | 1.0E-9 | 6.4E0 | 1.0E1 | 1.1E1 | 3.4E1 | 1.3E1 | 1.0E-9 | 1.0E-9 | 6.3E2 | 4.0E1 | 904 | 13 | 337 | 13 | 0.61 |
| Mq | pg/ml | 1.0E-9 | 1.0E-9 | 2.9E0 | 9.4E0 | 1.7E1 | 2.5E1 | 1.0E-9 | 1.0E-9 | 2.4E2 | 8.6E1 | 904 | 13 | 337 | 13 | 0.54 |
| Mr | pg/ml | 1.0E-9 | 3.5E0 | 2.8E1 | 5.2E0 | 1.6E2 | 6.4E0 | 1.0E-9 | 1.0E-9 | 2.2E3 | 1.7E1 | 904 | 13 | 337 | 13 | 0.60 |
| Ms | pg/ml | 4.1E2 | 5.0E2 | 5.6E2 | 4.4E2 | 6.5E2 | 3.3E2 | 1.0E-9 | 1.0E-9 | 5.9E3 | 1.1E3 | 904 | 13 | 337 | 13 | 0.49 |
| Mt | pg/ml | 2.5E-1 | 2.6E0 | 1.1E1 | 9.6E0 | 1.2E2 | 2.0E1 | 1.0E-9 | 1.0E-9 | 3.2E3 | 7.3E1 | 904 | 13 | 337 | 13 | 0.64 |
| Mu | pg/ml | 1.0E-9 | 1.0E-9 | 1.3E0 | 3.3E0 | 1.0E1 | 6.0E0 | 1.0E-9 | 1.0E-9 | 2.3E2 | 2.1E1 | 904 | 13 | 337 | 13 | 0.63 |
| Mv | pg/ml | 1.0E-9 | 1.0E-9 | 7.0E1 | 6.0E1 | 3.2E2 | 1.1E2 | 1.0E-9 | 1.0E-9 | 4.5E3 | 3.1E2 | 904 | 13 | 337 | 13 | 0.58 |
| Mw | pg/ml | 3.8E1 | 6.4E1 | 5.2E2 | 3.5E2 | 3.3E3 | 4.2E2 | 1.0E-9 | 1.0E-9 | 6.2E4 | 1.2E3 | 904 | 13 | 337 | 13 | 0.65 |
| Mx | pg/ml | 1.0E-9 | 1.0E-9 | 2.8E-1 | 1.7E-1 | 1.5E0 | 3.7E-1 | 1.0E-9 | 1.0E-9 | 3.2E1 | 1.2E0 | 904 | 13 | 337 | 13 | 0.54 |
| My | pg/ml | 1.0E-9 | 7.5E0 | 4.7E2 | 1.8E2 | 3.0E3 | 2.5E2 | 1.0E-9 | 1.0E-9 | 3.9E4 | 7.7E2 | 904 | 13 | 337 | 13 | 0.63 |
| Mz | pg/ml | 1.1E1 | 2.6E1 | 3.0E1 | 4.6E1 | 9.9E1 | 6.4E1 | 1.0E-9 | 1.0E-9 | 1.9E3 | 2.1E2 | 904 | 13 | 337 | 13 | 0.67 |
| Na | pg/ml | 1.0E-9 | 1.0E-9 | 7.3E-1 | 1.8E0 | 3.0E0 | 4.7E0 | 1.0E-9 | 1.0E-9 | 4.2E1 | 1.6E1 | 904 | 13 | 337 | 13 | 0.52 |
| Nb | pg/ml | 2.0E0 | 4.1E0 | 3.9E0 | 5.5E0 | 1.2E1 | 3.9E0 | 1.0E-9 | 1.0E-9 | 2.3E2 | 1.3E1 | 904 | 13 | 337 | 13 | 0.72 |
| Nc | pg/ml | 3.4E2 | 3.0E2 | 5.6E2 | 4.9E2 | 7.3E2 | 5.0E2 | 1.0E-9 | 1.0E-9 | 9.2E3 | 1.6E3 | 904 | 13 | 337 | 13 | 0.53 |
| Nd | pg/ml | 2.9E1 | 4.8E0 | 2.9E1 | 2.2E1 | 8.3E1 | 2.8E1 | 1.0E-9 | 1.0E-9 | 2.1E3 | 9.4E1 | 904 | 13 | 337 | 13 | 0.40 |
| Nc | pg/ml | 4.4E2 | 3.1E2 | 5.8E2 | 4.1E2 | 5.7E2 | 3.5E2 | 1.0E-9 | 5.5E1 | 7.0E3 | 1.2E3 | 904 | 13 | 337 | 13 | 0.42 |
| Nf | pg/ml | 1.0E-9 | 1.0E-9 | 3.0E0 | 2.1E-1 | 1.1E1 | 7.6E-1 | 1.0E-9 | 1.0E-9 | 1.6E2 | 2.7E0 | 904 | 13 | 337 | 13 | 0.41 |
| Ng | pg/ml | 1.9E1 | 1.4E-1 | 1.3E2 | 9.0E1 | 2.5E2 | 1.6E2 | 1.0E-9 | 1.0E-9 | 2.3E3 | 4.7E2 | 904 | 13 | 337 | 13 | 0.41 |
| Nh | pg/ml | 6.9E1 | 5.3E1 | 9.0E1 | 6.4E1 | 8.2E1 | 4.7E1 | 1.0E-9 | 8.6E0 | 5.6E2 | 1.5E2 | 904 | 13 | 337 | 13 | 0.43 |
| Ni | pg/ml | 1.0E-9 | 1.5E2 | 7.3E1 | 2.1E2 | 1.2E2 | 1.7E2 | 1.0E-9 | 1.0E-9 | 1.1E3 | 4.8E2 | 904 | 13 | 337 | 13 | 0.75 |
| Nj | pg/ml | 7.3E0 | 5.4E0 | 1.1E1 | 1.1E1 | 1.2E1 | 9.1E0 | 1.0E-9 | 1.2E0 | 1.1E2 | 2.8E1 | 904 | 13 | 337 | 13 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nk | pg/ml | 1.7E1 | 3.6E1 | 3.3E1 | 3.8E1 | 4.0E1 | 2.6E1 | 1.0E-9 | 1.2E1 | 2.0E2 | 8.7E1 | 904 | 13 | 337 | 13 | 0.63 |
| Nl | pg/ml | 4.6E1 | 4.1E1 | 6.1E1 | 5.7E1 | 6.8E1 | 4.9E1 | 1.0E-9 | 9.3E-1 | 1.1E3 | 1.4E2 | 904 | 13 | 337 | 13 | 0.50 |
| Tz | pg/ml | 5.3E3 | 8.5E3 | 1.4E4 | 6.6E3 | 6.2E4 | 4.0E3 | 1.0E-9 | 1.8E3 | 1.0E6 | 1.0E4 | 317 | 7 | 187 | 7 | 0.52 |
| Ua | pg/ml | 3.9E3 | 4.7E3 | 2.0E4 | 2.7E4 | 1.2E5 | 4.9E4 | 1.0E-9 | 8.8E2 | 2.1E6 | 1.4E5 | 317 | 7 | 187 | 7 | 0.58 |
| Ub | pg/ml | 5.6E2 | 2.3E2 | 8.4E2 | 2.5E2 | 1.0E3 | 1.8E2 | 1.0E-9 | 3.9E1 | 9.8E3 | 5.8E2 | 317 | 7 | 187 | 7 | 0.25 |
| Ue | pg/ml | 2.8E1 | 1.6E1 | 3.9E1 | 1.9E1 | 4.2E1 | 6.5E0 | 9.8E-2 | 1.0E1 | 4.4E2 | 3.0E1 | 317 | 7 | 187 | 7 | 0.27 |
| Uc | pg/ml | 9.2E2 | 1.0E3 | 1.9E3 | 9.5E2 | 4.1E3 | 6.5E2 | 1.0E-9 | 1.2E2 | 5.7E4 | 2.0E3 | 317 | 7 | 187 | 7 | 0.45 |
| Ud | pg/ml | 1.0E-9 | 1.0E-9 | 1.5E0 | 1.0E-9 | 2.2E1 | 0.0E0 | 1.0E-9 | 1.0E-9 | 3.9E2 | 1.0E-9 | 317 | 7 | 187 | 7 | 0.49 |
| Hq | pg/ml | 1.1E0 | 1.5E0 | 1.0E2 | 3.4E0 | 1.6E3 | 6.3E0 | 1.0E-9 | 1.0E-9 | 3.4E4 | 2.4E1 | 900 | 13 | 336 | 13 | 0.57 |
| Hr | pg/ml | 1.2E2 | 8.9E1 | 7.9E2 | 5.3E2 | 1.6E3 | 1.3E3 | 1.0E-9 | 2.5E1 | 1.7E4 | 4.6E3 | 900 | 13 | 336 | 13 | 0.48 |
| Hu | pg/ml | 5.6E0 | 1.2E1 | 3.0E3 | 6.4E2 | 2.6E4 | 2.0E3 | 1.0E-9 | 1.0E-9 | 6.3E5 | 7.2E3 | 900 | 13 | 336 | 13 | 0.50 |
| Hv | pg/ml | 1.4E0 | 2.0E0 | 4.1E0 | 2.4E0 | 3.2E1 | 2.5E0 | 1.0E-9 | 1.0E-9 | 8.9E2 | 9.4E0 | 900 | 13 | 336 | 13 | 0.58 |
| Hw | pg/ml | 6.5E0 | 5.1E0 | 2.9E1 | 9.8E0 | 3.2E2 | 1.8E1 | 1.0E-9 | 1.0E-9 | 9.4E3 | 6.8E1 | 900 | 13 | 336 | 13 | 0.38 |
| Hx | pg/ml | 8.8E0 | 2.5E1 | 3.9E1 | 4.1E1 | 3.2E2 | 5.6E1 | 1.0E-9 | 1.0E-9 | 9.3E3 | 2.1E2 | 900 | 13 | 336 | 13 | 0.67 |
| Ib | ng/ml | 4.9E-2 | 1.8E-2 | 1.4E0 | 8.8E-2 | 5.6E0 | 1.3E-1 | 1.0E-9 | 1.0E-9 | 5.6E1 | 3.1E-1 | 307 | 7 | 186 | 7 | 0.40 |
| Ic | U/ml | 2.2E2 | 2.2E2 | 9.5E2 | 3.1E3 | 6.7E3 | 7.7E3 | 1.5E0 | 4.2E1 | 9.3E4 | 2.1E4 | 307 | 7 | 186 | 7 | 0.60 |
| Id | U/ml | 6.9E-1 | 1.2E0 | 2.9E0 | 2.5E0 | 2.5E1 | 3.2E0 | 1.0E-9 | 2.7E-1 | 4.3E2 | 9.4E0 | 307 | 7 | 186 | 7 | 0.64 |
| Ih | ng/ml | 7.2E1 | 2.1E2 | 2.4E2 | 5.7E2 | 4.3E2 | 9.6E2 | 1.0E-9 | 1.0E-9 | 3.6E3 | 3.5E3 | 904 | 13 | 336 | 13 | 0.59 |
| Ii | ng/ml | 9.4E1 | 3.7E1 | 2.5E2 | 1.7E2 | 6.8E2 | 2.7E2 | 1.0E-9 | 2.5E0 | 1.0E4 | 7.9E2 | 903 | 13 | 336 | 13 | 0.40 |
| Ij | ng/ml | 7.7E1 | 9.9E1 | 2.0E2 | 1.1E2 | 9.9E2 | 8.4E1 | 1.0E-9 | 6.2E0 | 2.4E4 | 2.9E2 | 891 | 13 | 334 | 13 | 0.51 |
| Ik | ng/ml | 1.3E1 | 5.7E1 | 8.1E2 | 2.2E2 | 8.2E3 | 4.0E2 | 5.9E-1 | 2.8E0 | 1.2E5 | 1.5E3 | 898 | 13 | 334 | 13 | 0.57 |
| Il | ng/ml | 3.4E2 | 1.8E2 | 1.3E3 | 3.5E2 | 2.8E3 | 6.4E2 | 1.0E-9 | 1.0E-9 | 1.3E4 | 2.4E3 | 881 | 13 | 334 | 13 | 0.31 |
| Im | ng/ml | 2.1E2 | 5.7E2 | 3.9E2 | 9.4E2 | 6.0E2 | 1.8E3 | 1.3E1 | 3.2E1 | 6.2E3 | 6.8E3 | 897 | 13 | 335 | 13 | 0.58 |
| In | ng/ml | 3.7E0 | 8.9E-1 | 2.7E1 | 2.9E0 | 2.1E2 | 3.6E0 | 1.0E-9 | 1.0E-9 | 4.5E3 | 9.4E0 | 904 | 13 | 336 | 13 | 0.32 |
| Hb | ng/ml | 2.5E1 | 2.3E1 | 3.6E1 | 4.1E1 | 3.5E1 | 4.3E1 | 4.8E-1 | 4.1E0 | 2.1E2 | 1.2E2 | 316 | 7 | 187 | 7 | 0.52 |
| Hc | pg/ml | 6.4E2 | 8.3E2 | 3.4E3 | 5.0E3 | 1.2E4 | 6.0E3 | 1.0E-9 | 2.6E2 | 1.0E5 | 1.5E4 | 316 | 7 | 187 | 7 | 0.62 |
| Hf | ng/ml | 1.6E2 | 1.4E2 | 3.6E2 | 3.6E2 | 4.9E2 | 4.1E2 | 1.0E-9 | 6.8E1 | 2.5E3 | 1.0E3 | 316 | 7 | 187 | 7 | 0.57 |
| Io | ng/ml | 8.1E3 | 5.1E3 | 2.4E4 | 9.7E3 | 1.5E5 | 1.1E4 | 1.0E-9 | 9.0E2 | 4.0E6 | 3.3E4 | 896 | 13 | 336 | 13 | 0.43 |
| Ip | ng/ml | 9.7E0 | 5.2E0 | 1.9E1 | 2.9E1 | 2.4E1 | 4.0E1 | 1.0E-9 | 8.1E-2 | 2.6E2 | 1.4E2 | 896 | 13 | 336 | 13 | 0.54 |
| Iq | ug/ml | 1.0E-1 | 1.3E-1 | 3.2E1 | 2.3E-1 | 6.4E2 | 3.5E-1 | 1.0E-9 | 1.0E-9 | 1.4E4 | 1.3E0 | 896 | 13 | 336 | 13 | 0.44 |
| Ir | ug/ml | 3.5E-1 | 1.6E0 | 3.9E0 | 4.9E0 | 2.8E1 | 9.2E0 | 1.0E-9 | 7.7E-2 | 5.1E2 | 3.4E1 | 895 | 13 | 336 | 13 | 0.70 |
| Is | ng/ml | 1.5E0 | 9.0E0 | 6.6E0 | 1.2E1 | 2.3E1 | 1.2E1 | 1.0E-9 | 2.7E-1 | 5.5E2 | 3.5E1 | 896 | 13 | 336 | 13 | 0.73 |
| It | ng/ml | 2.0E0 | 2.9E0 | 2.4E1 | 1.8E1 | 1.4E2 | 3.3E1 | 1.0E-9 | 1.0E-9 | 2.8E3 | 1.2E2 | 896 | 13 | 336 | 13 | 0.53 |
| Iu | ng/ml | 2.2E2 | 1.7E2 | 1.4E3 | 4.2E2 | 4.2E3 | 6.3E2 | 1.0E-9 | 1.0E-9 | 2.9E4 | 2.3E3 | 896 | 13 | 336 | 13 | 0.46 |
| Iv | ng/ml | 1.3E1 | 5.0E1 | 6.1E1 | 1.2E2 | 5.5E2 | 2.3E2 | 1.0E-9 | 1.9E0 | 1.6E4 | 8.7E2 | 895 | 13 | 336 | 13 | 0.73 |
| Iz | ng/ml | 1.4E2 | 4.3E2 | 6.0E2 | 5.0E2 | 3.6E3 | 5.6E2 | 9.2E-1 | 2.1E1 | 6.2E4 | 1.5E3 | 316 | 7 | 187 | 7 | 0.55 |
| Rc | pg/ml | 5.9E3 | 5.3E3 | 7.3E3 | 9.8E3 | 5.8E3 | 9.2E3 | 1.9E2 | 2.8E3 | 3.9E4 | 2.9E4 | 314 | 7 | 187 | 7 | 0.58 |
| Rb | pg/ml | 8.7E-1 | 1.5E0 | 2.8E0 | 2.5E0 | 5.3E0 | 2.1E0 | 1.0E-9 | 8.6E-1 | 5.6E1 | 5.6E0 | 314 | 7 | 187 | 7 | 0.64 |
| Pz | ng/ml | 3.9E3 | 1.0E4 | 8.3E3 | 6.4E3 | 3.8E4 | 4.6E3 | 1.3E1 | 9.5E1 | 1.0E6 | 1.2E4 | 896 | 13 | 334 | 13 | 0.56 |
| Qa | ng/ml | 3.5E3 | 9.9E3 | 6.5E3 | 1.1E4 | 1.0E4 | 9.3E3 | 1.2E1 | 2.9E2 | 2.2E5 | 2.9E4 | 896 | 13 | 334 | 13 | 0.63 |
| Qb | ng/ml | 9.7E1 | 2.1E2 | 2.1E2 | 2.7E2 | 4.9E2 | 3.3E2 | 7.9E-1 | 8.7E0 | 8.3E3 | 1.2E3 | 896 | 13 | 334 | 13 | 0.58 |
| Qc | ng/ml | 2.3E2 | 4.8E2 | 6.3E2 | 4.3E2 | 5.6E3 | 3.7E2 | 1.0E-9 | 8.6E0 | 1.7E5 | 1.2E3 | 896 | 13 | 334 | 13 | 0.54 |
| Qd | ng/ml | 9.2E3 | 4.4E4 | 1.9E4 | 6.8E4 | 7.3E4 | 7.8E4 | 1.5E2 | 1.7E3 | 2.0E6 | 2.3E5 | 896 | 13 | 334 | 13 | 0.69 |
| Qe | ng/ml | 9.2E2 | 2.6E3 | 1.9E3 | 3.6E3 | 4.7E3 | 3.9E3 | 1.0E-9 | 1.2E2 | 9.7E4 | 1.3E4 | 896 | 13 | 334 | 13 | 0.65 |
| Jd | ng/ml | 9.5E-1 | 3.0E0 | 5.9E0 | 3.3E0 | 3.9E1 | 3.1E0 | 1.0E-9 | 1.0E-9 | 6.5E2 | 8.5E0 | 315 | 7 | 189 | 7 | 0.63 |
| Je | ng/ml | 1.0E-9 | 2.8E0 | 2.1E0 | 2.3E0 | 7.2E0 | 2.3E0 | 1.0E-9 | 1.0E-9 | 8.8E1 | 6.4E0 | 315 | 7 | 189 | 7 | 0.66 |
| Jf | ng/ml | 1.0E-9 | 2.7E-1 | 1.1E0 | 8.5E-1 | 2.2E0 | 1.2E0 | 1.0E-9 | 1.0E-9 | 1.2E1 | 3.0E0 | 315 | 7 | 189 | 7 | 0.58 |
| Jg | ng/ml | 5.0E2 | 1.3E3 | 8.3E2 | 1.2E3 | 1.0E3 | 1.0E3 | 1.0E-9 | 4.5E1 | 1.0E4 | 3.5E3 | 900 | 13 | 336 | 13 | 0.64 |
| Jh | ng/ml | 3.0E0 | 1.2E1 | 2.9E1 | 3.9E1 | 1.2E2 | 6.2E1 | 1.0E-9 | 1.0E-9 | 1.6E3 | 2.1E2 | 900 | 13 | 336 | 13 | 0.66 |
| Ji | ng/ml | 5.3E1 | 9.0E1 | 7.9E1 | 1.6E2 | 9.0E1 | 1.4E2 | 1.0E-9 | 2.7E1 | 1.3E3 | 4.6E2 | 900 | 13 | 336 | 13 | 0.72 |
| Sr | pg/mL | 3.9E2 | 2.1E3 | 9.5E2 | 2.6E3 | 1.7E3 | 2.1E3 | 1.0E-9 | 1.0E-9 | 2.1E4 | 5.4E3 | 305 | 7 | 184 | 7 | 0.68 |
| Ss | pg/mL | 9.4E4 | 2.0E5 | 1.5E5 | 1.4E5 | 1.8E5 | 8.4E4 | 2.7E3 | 2.6E4 | 1.8E6 | 2.0E5 | 305 | 7 | 184 | 7 | 0.55 |
| St | pg/mL | 2.7E7 | 7.5E7 | 5.5E7 | 3.4E8 | 9.2E7 | 5.9E8 | 1.0E-9 | 1.6E6 | 1.2E9 | 1.7E9 | 309 | 7 | 185 | 7 | 0.65 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ra | pg/ml | 1.0E-9 | 5.2E-1 | 8.1E-1 | 6.5E-1 | 3.8E0 | 1.1E0 | 1.0E-9 | 1.0E-9 | 6.4E1 | 3.0E0 | 314 | 7 | 187 | 7 | 0.59 |
| Qz | pg/ml | 1.0E1 | 1.0E-9 | 6.2E1 | 5.3E1 | 1.0E2 | 1.2E2 | 1.0E-9 | 1.0E-9 | 7.1E2 | 3.2E2 | 314 | 7 | 187 | 7 | 0.37 |
| Qy | pg/ml | 4.4E-1 | 8.8E-1 | 1.3E1 | 6.9E0 | 7.1E1 | 1.2E1 | 1.0E-9 | 2.5E-1 | 6.5E2 | 3.1E1 | 314 | 7 | 187 | 7 | 0.68 |
| Qx | pg/ml | 1.0E-9 | 1.0E-9 | 5.9E0 | 1.6E0 | 4.6E1 | 3.9E0 | 1.0E-9 | 1.0E-9 | 5.8E2 | 1.0E1 | 314 | 7 | 187 | 7 | 0.57 |
| Qw | pg/ml | 1.0E-9 | 1.0E-9 | 3.1E0 | 1.0E-9 | 1.5E1 | 0.0E0 | 1.0E-9 | 1.0E-9 | 1.3E2 | 1.0E-9 | 314 | 7 | 187 | 7 | 0.28 |
| Qv | pg/ml | 2.3E4 | 6.6E3 | 3.6E4 | 8.2E3 | 7.3E4 | 5.7E3 | 1.0E-9 | 2.1E3 | 9.4E5 | 2.0E4 | 314 | 7 | 187 | 7 | 0.19 |
| Qu | pg/ml | 6.8E0 | 4.4E1 | 8.4E1 | 8.1E1 | 1.7E2 | 9.9E1 | 1.0E-9 | 1.0E-9 | 9.8E2 | 2.2E2 | 314 | 7 | 187 | 7 | 0.56 |
| Qt | pg/ml | 1.0E1 | 2.1E1 | 5.0E1 | 4.1E1 | 1.2E2 | 4.9E1 | 1.0E-9 | 1.0E-9 | 1.0E3 | 1.4E2 | 314 | 7 | 187 | 7 | 0.62 |
| Qh | ng/ml | 1.7E1 | 3.3E1 | 3.8E1 | 4.8E1 | 6.4E1 | 4.2E1 | 1.0E-9 | 7.8E-1 | 6.4E2 | 1.1E2 | 314 | 7 | 187 | 7 | 0.60 |
| Qg | ng/ml | 8.2E0 | 1.2E1 | 1.9E1 | 1.8E1 | 6.3E1 | 2.2E1 | 5.1E-2 | 7.7E-1 | 1.0E3 | 6.3E1 | 314 | 7 | 187 | 7 | 0.53 |
| Jj | ng/ml | 6.1E2 | 2.0E2 | 1.6E3 | 3.8E2 | 1.2E4 | 5.0E2 | 1.5E0 | 3.3E1 | 3.4E5 | 1.8E3 | 900 | 13 | 336 | 13 | 0.26 |
| Jk | ng/ml | 3.0E0 | 7.9E0 | 2.1E1 | 2.4E1 | 4.7E1 | 3.1E1 | 1.0E-9 | 2.0E-1 | 3.9E2 | 1.0E2 | 900 | 13 | 336 | 13 | 0.56 |
| Jl | ng/ml | 4.1E-1 | 1.3E0 | 2.6E0 | 4.3E0 | 1.9E1 | 7.1E0 | 7.6E-4 | 3.9E-2 | 5.4E2 | 2.0E1 | 900 | 13 | 336 | 13 | 0.68 |
| Jm | ng/ml | 1.8E1 | 2.1E1 | 5.8E1 | 4.6E1 | 1.3E2 | 6.1E1 | 1.0E-9 | 4.1E-1 | 2.1E3 | 1.5E2 | 900 | 13 | 336 | 13 | 0.48 |
| Jn | pg/ml | 4.0E-1 | 1.0E0 | 3.7E0 | 2.0E0 | 3.8E1 | 2.3E0 | 1.0E-9 | 2.5E-2 | 7.3E2 | 8.3E0 | 899 | 13 | 336 | 13 | 0.65 |
| Jo | pg/ml | 3.6E3 | 1.8E3 | 4.9E3 | 3.2E3 | 5.0E3 | 2.9E3 | 2.0E1 | 2.6E2 | 1.0E5 | 9.2E3 | 900 | 13 | 336 | 13 | 0.36 |
| Jp | pg/ml | 7.0E4 | 1.1E5 | 7.3E4 | 1.0E5 | 3.8E4 | 3.4E4 | 5.8E2 | 2.3E4 | 3.8E5 | 1.5E5 | 900 | 13 | 336 | 13 | 0.76 |
| Jq | pg/ml | 9.6E1 | 1.4E2 | 1.6E2 | 3.7E2 | 3.6E2 | 8.9E2 | 1.0E0 | 1.5E1 | 8.7E3 | 3.3E3 | 900 | 13 | 336 | 13 | 0.52 |
| Jr | pg/ml | 5.3E0 | 1.7E1 | 4.4E1 | 2.3E1 | 4.7E2 | 2.7E1 | 1.0E-9 | 1.0E-9 | 1.1E4 | 1.0E2 | 900 | 13 | 336 | 13 | 0.70 |
| Js | pg/ml | 1.3E1 | 1.4E1 | 5.3E1 | 2.0E1 | 3.8E2 | 1.6E1 | 1.0E-9 | 3.5E0 | 1.0E4 | 5.6E1 | 900 | 13 | 336 | 13 | 0.53 |
| Jt | pg/ml | 2.6E3 | 2.6E3 | 3.2E3 | 3.1E3 | 2.8E3 | 2.1E3 | 2.2E1 | 8.5E2 | 5.2E4 | 7.5E3 | 900 | 13 | 336 | 13 | 0.50 |
| Ju | mIU/ml | 9.0E0 | 1.3E1 | 1.9E1 | 1.1E1 | 3.0E1 | 8.8E0 | 4.8E-2 | 2.7E-1 | 2.3E2 | 2.4E1 | 315 | 7 | 189 | 7 | 0.47 |
| Jv | mIU/ml | 1.2E1 | 8.9E0 | 3.4E1 | 1.2E1 | 5.9E1 | 1.2E1 | 1.0E-2 | 9.4E-3 | 4.4E2 | 2.9E1 | 315 | 7 | 189 | 7 | 0.41 |
| Jy | ng/ml | 1.6E-3 | 2.9E-3 | 2.2E-3 | 8.0E-3 | 3.9E-3 | 1.5E-2 | 1.0E-9 | 8.6E-4 | 5.2E-2 | 4.1E-2 | 315 | 7 | 189 | 7 | 0.73 |
| Kc | pg/ml | 2.6E1 | 1.4E2 | 4.5E1 | 1.2E2 | 4.8E1 | 6.0E1 | 1.0E-9 | 2.2E1 | 2.7E2 | 1.7E2 | 316 | 7 | 187 | 7 | 0.84 |
| Kd | pg/ml | 1.0E-9 | 1.0E-9 | 3.0E2 | 9.5E2 | 2.2E3 | 1.9E3 | 1.0E-9 | 1.0E-9 | 3.8E4 | 5.2E3 | 316 | 7 | 187 | 7 | 0.57 |
| Ke | pg/ml | 1.3E4 | 1.3E4 | 1.6E4 | 2.6E4 | 2.1E4 | 3.8E4 | 3.4E2 | 2.2E3 | 3.2E5 | 1.1E5 | 316 | 7 | 187 | 7 | 0.51 |
| Kf | pg/mL | 7.3E0 | 1.7E1 | 7.6E0 | 1.4E1 | 7.1E0 | 7.4E0 | 1.0E-9 | 1.0E-9 | 7.8E1 | 2.2E1 | 316 | 7 | 187 | 7 | 0.76 |
| Kg | pg/mL | 1.1E3 | 5.2E2 | 1.9E3 | 1.1E3 | 2.8E3 | 1.5E3 | 7.3E1 | 2.9E2 | 2.7E4 | 4.4E3 | 316 | 7 | 187 | 7 | 0.34 |
| Ki | pg/ml | 5.9E1 | 8.8E1 | 7.0E1 | 8.7E1 | 5.4E1 | 2.6E1 | 1.0E-9 | 4.2E1 | 3.8E2 | 1.2E2 | 315 | 7 | 187 | 7 | 0.69 |
| Kj | pg/ml | 9.7E2 | 3.2E2 | 1.6E3 | 6.9E2 | 1.8E3 | 6.5E2 | 1.4E1 | 2.1E2 | 1.5E4 | 1.8E3 | 316 | 7 | 187 | 7 | 0.29 |
| Kk | pg/ml | 7.0E0 | 5.5E1 | 1.2E1 | 4.0E1 | 1.6E1 | 2.6E1 | 1.0E-9 | 1.0E-9 | 1.6E2 | 6.4E1 | 316 | 7 | 187 | 7 | 0.78 |
| Kl | pg/ml | 2.0E4 | 3.3E4 | 2.8E4 | 3.5E4 | 2.5E4 | 2.5E4 | 1.6E2 | 3.2E3 | 1.6E5 | 6.8E4 | 316 | 7 | 187 | 7 | 0.60 |
| Kn | pg/ml | 3.0E1 | 6.2E1 | 7.7E1 | 1.5E2 | 2.9E2 | 2.0E2 | 1.0E-9 | 1.0E-9 | 4.9E3 | 4.7E2 | 316 | 7 | 187 | 7 | 0.58 |
| Ko | pg/ml | 3.6E2 | 7.5E2 | 5.2E2 | 6.8E2 | 5.5E2 | 5.1E2 | 1.0E-9 | 1.0E-9 | 4.1E3 | 1.4E3 | 316 | 7 | 187 | 7 | 0.62 |
| Kp | pg/ml | 3.4E2 | 7.9E2 | 4.0E2 | 7.4E2 | 7.8E2 | 3.1E2 | 1.0E-9 | 2.8E2 | 1.3E4 | 1.1E3 | 316 | 7 | 187 | 7 | 0.83 |
| Kq | pg/ml | 3.2E2 | 8.7E2 | 1.0E3 | 2.5E2 | 9.1E3 | 4.1E3 | 1.6E0 | 2.9E2 | 1.6E5 | 1.2E4 | 308 | 7 | 181 | 7 | 0.77 |
| Kr | pg/ml | 5.0E-1 | 1.0E-9 | 3.6E0 | 5.6E-1 | 2.4E1 | 1.2E0 | 1.0E-9 | 1.0E-9 | 4.2E2 | 3.3E0 | 308 | 7 | 181 | 7 | 0.37 |
| Ks | pg/ml | 1.4E4 | 1.2E4 | 2.0E4 | 1.5E4 | 1.8E4 | 1.4E4 | 5.1E1 | 3.6E3 | 1.1E5 | 4.3E4 | 308 | 7 | 181 | 7 | 0.45 |
| Ky | ng/ml | 9.5E-2 | 9.4E-1 | 3.8E-1 | 1.1E0 | 8.2E-1 | 1.0E0 | 1.0E-9 | 1.0E-9 | 6.4E0 | 2.7E0 | 315 | 7 | 187 | 7 | 0.76 |
| Kz | ng/ml | 1.0E-9 | 1.0E-9 | 3.6E-3 | 6.7E-4 | 5.7E-3 | 1.8E-3 | 1.0E-9 | 1.0E-9 | 3.6E-2 | 4.7E-3 | 315 | 7 | 187 | 7 | 0.38 |
| Ld | pg/ml | 1.0E-9 | 3.6E0 | 3.7E0 | 4.5E0 | 8.8E0 | 4.4E0 | 1.0E-9 | 1.0E-9 | 8.4E1 | 1.2E1 | 314 | 7 | 186 | 7 | 0.68 |
| Lh | pg/ml | 1.3E4 | 2.3E4 | 2.2E4 | 2.8E4 | 3.2E4 | 2.6E4 | 1.0E-9 | 4.0E2 | 4.8E5 | 7.3E4 | 900 | 12 | 337 | 12 | 0.57 |
| Li | pg/ml | 3.3E3 | 9.7E3 | 1.7E4 | 2.3E4 | 6.2E4 | 3.3E4 | 1.0E-9 | 1.2E2 | 1.3E6 | 1.1E5 | 900 | 12 | 337 | 12 | 0.61 |
| Lj | pg/ml | 2.8E3 | 8.3E3 | 2.3E4 | 7.4E4 | 6.6E4 | 1.8E5 | 1.0E-9 | 1.9E2 | 5.2E5 | 6.1E5 | 900 | 12 | 337 | 12 | 0.58 |
| Rm | ng/ml | 2.0E1 | 5.2E1 | 5.3E1 | 5.1E1 | 8.2E1 | 3.6E1 | 2.2E-1 | 1.4E0 | 6.5E2 | 1.0E2 | 309 | 7 | 186 | 7 | 0.62 |
| Rh | ng/ml | 1.3E2 | 3.9E2 | 3.6E2 | 6.4E2 | 1.2E3 | 8.6E2 | 3.6E0 | 8.1E1 | 1.7E4 | 2.5E3 | 309 | 7 | 186 | 7 | 0.68 |
| Ri | ng/ml | 1.0E-9 | 1.0E-9 | 4.2E0 | 6.8E0 | 1.5E1 | 1.3E1 | 1.0E-9 | 1.0E-9 | 2.2E2 | 3.6E1 | 310 | 7 | 187 | 7 | 0.53 |
| Rg | ng/ml | 1.0E-9 | 3.0E-3 | 6.0E-2 | 3.1E-3 | 3.8E-1 | 4.5E-3 | 1.0E-9 | 1.0E-9 | 4.6E0 | 1.3E-2 | 309 | 7 | 186 | 7 | 0.65 |
| Rj | ng/ml | 1.0E-9 | 1.0E-9 | 2.2E0 | 1.0E-9 | 1.6E1 | 0.0E0 | 1.0E-9 | 1.0E-9 | 2.7E2 | 1.0E-9 | 310 | 7 | 187 | 7 | 0.36 |
| Rf | ng/ml | 4.2E-1 | 2.6E0 | 1.0E0 | 4.5E0 | 1.8E0 | 4.8E0 | 7.8E-3 | 3.9E-2 | 1.5E1 | 1.4E1 | 309 | 7 | 186 | 7 | 0.80 |
| Ql | pg/ml | 5.5E0 | 7.3E0 | 1.5E1 | 1.1E1 | 2.9E1 | 1.1E1 | 1.0E-9 | 1.0E-9 | 2.9E2 | 2.6E1 | 315 | 7 | 189 | 7 | 0.53 |
| Qm | pg/ml | 4.4E0 | 2.3E1 | 2.1E1 | 3.1E1 | 3.8E1 | 4.3E1 | 1.0E-9 | 1.0E-9 | 3.6E2 | 1.2E2 | 315 | 7 | 189 | 7 | 0.56 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|------|--------|------|------|
| | | NonDis | Dis | NonDis | Dis | NonDis | Dis | NonDis | Dis | NonDis | Dis | NonDis | Dis | NonDis | Dis | |
| Qn | pg/ml | 6.1E-1 | 6.0E-1 | 8.4E0 | 1.9E0 | 2.6E1 | 4.2E0 | 1.0E-9 | 1.0E-9 | 2.4E2 | 1.1E1 | 315 | 7 | 189 | 7 | 0.38 |
| Nv | pg/ml | 4.0E3 | 5.1E3 | 1.1E4 | 1.8E4 | 4.3E4 | 2.5E4 | 1.0E-9 | 4.7E2 | 1.1E6 | 7.7E4 | 906 | 13 | 337 | 13 | 0.64 |
| Nw | pg/ml | 8.9E3 | 1.6E4 | 1.3E4 | 3.5E4 | 1.7E4 | 5.7E4 | 8.6E1 | 3.9E3 | 2.1E5 | 2.2E5 | 906 | 13 | 337 | 13 | 0.70 |
| Nx | pg/ml | 2.2E2 | 1.1E3 | 4.1E2 | 8.2E2 | 6.5E2 | 6.3E2 | 1.0E-9 | 3.6E1 | 7.4E3 | 2.2E3 | 906 | 13 | 337 | 13 | 0.75 |
| Ny | pg/ml | 6.4E0 | 1.8E0 | 5.7E1 | 6.6E1 | 8.5E2 | 1.4E2 | 1.0E-9 | 1.0E-9 | 2.5E4 | 4.9E2 | 906 | 13 | 337 | 13 | 0.53 |
| Oa | pg/ml | 1.8E2 | 5.5E2 | 4.6E2 | 7.0E2 | 7.3E2 | 8.1E2 | 1.0E-9 | 2.6E1 | 4.8E3 | 2.4E3 | 315 | 7 | 189 | 7 | 0.63 |
| Oe | pg/ml | 6.8E1 | 7.8E1 | 2.9E2 | 1.9E2 | 7.5E2 | 2.5E2 | 1.0E-9 | 1.0E-9 | 1.9E4 | 7.7E2 | 897 | 13 | 336 | 13 | 0.49 |
| Of | pg/ml | 1.7E2 | 9.7E1 | 6.1E3 | 1.2E3 | 2.8E4 | 1.9E3 | 1.0E-9 | 1.0E-9 | 6.2E5 | 6.0E3 | 905 | 13 | 337 | 13 | 0.45 |
| Og | pg/ml | 8.2E-2 | 7.6E-2 | 4.8E-1 | 1.2E-1 | 1.6E0 | 1.6E-1 | 1.0E-9 | 1.0E-9 | 2.0E1 | 5.3E-1 | 905 | 13 | 337 | 13 | 0.44 |
| Oh | pg/ml | 2.7E0 | 4.8E0 | 2.0E1 | 2.9E1 | 1.5E2 | 5.9E1 | 1.0E-9 | 1.0E-9 | 3.5E3 | 1.9E2 | 905 | 13 | 337 | 13 | 0.60 |
| Oi | pg/ml | 2.6E0 | 3.3E-1 | 6.3E0 | 6.3E0 | 9.8E0 | 9.2E0 | 1.0E-9 | 1.0E-9 | 8.6E1 | 2.8E1 | 905 | 13 | 337 | 13 | 0.47 |
| Ok | pg/ml | 3.9E2 | 8.3E2 | 5.6E2 | 1.3E3 | 6.0E2 | 1.5E3 | 1.3E1 | 6.4E1 | 7.8E3 | 5.2E3 | 905 | 13 | 337 | 13 | 0.67 |
| Om | pg/ml | 4.0E2 | 5.9E2 | 8.9E2 | 1.1E3 | 2.7E3 | 1.5E3 | 1.0E-9 | 1.0E2 | 5.1E4 | 5.8E3 | 905 | 13 | 337 | 13 | 0.63 |
| On | pg/ml | 1.8E2 | 5.1E2 | 3.0E2 | 5.7E2 | 4.2E2 | 4.9E2 | 1.0E-9 | 4.2E1 | 4.5E3 | 1.5E3 | 905 | 13 | 337 | 13 | 0.69 |
| Or | pg/ml | 1.4E1 | 1.5E1 | 3.8E1 | 2.8E1 | 7.3E1 | 3.9E1 | 1.0E-9 | 1.0E-9 | 5.1E2 | 1.1E2 | 317 | 7 | 187 | 7 | 0.53 |
| Ow | pg/ml | 3.6E1 | 1.5E1 | 1.5E2 | 7.1E1 | 5.6E2 | 1.5E2 | 1.0E-9 | 1.0E-9 | 8.1E3 | 4.0E2 | 317 | 7 | 187 | 7 | 0.37 |
| Ou | pg/ml | 4.9E2 | 9.7E2 | 1.0E3 | 2.8E3 | 1.6E3 | 3.3E3 | 1.0E-9 | 1.0E-9 | 9.8E3 | 7.5E3 | 317 | 7 | 187 | 7 | 0.70 |
| Ul | ng/ml | 1.0E-9 | 1.0E-9 | 1.8E0 | 1.0E-9 | 8.3E0 | 0.0E0 | 1.0E-9 | 1.0E-9 | 1.0E2 | 1.0E-9 | 323 | 7 | 191 | 7 | 0.45 |
| Uo | ng/ml | 1.0E-9 | 1.0E-9 | 9.2E-2 | 8.3E-2 | 2.2E-1 | 2.2E-1 | 1.0E-9 | 1.0E-9 | 1.5E0 | 5.8E-1 | 323 | 7 | 191 | 7 | 0.44 |
| Uk | ng/ml | 1.0E-9 | 1.0E-9 | 7.6E-3 | 3.6E-4 | 2.5E-2 | 6.2E-4 | 1.0E-9 | 1.0E-9 | 3.2E-1 | 1.4E-3 | 323 | 7 | 191 | 7 | 0.39 |
| Um | ng/ml | 1.0E-9 | 1.0E-9 | 3.9E-1 | 4.0E-2 | 8.7E-1 | 1.0E-1 | 1.0E-9 | 1.0E-9 | 6.8E0 | 2.8E-1 | 323 | 7 | 191 | 7 | 0.37 |
| Uf | ng/ml | 6.0E-2 | 1.5E-1 | 1.7E-1 | 1.5E-1 | 6.0E-1 | 1.2E-1 | 1.0E-3 | 8.6E-3 | 8.6E0 | 3.7E-1 | 323 | 7 | 191 | 7 | 0.64 |
| Uh | ng/ml | 2.0E0 | 2.0E0 | 3.3E0 | 3.5E0 | 3.6E0 | 5.1E0 | 1.3E-2 | 6.3E-2 | 2.1E1 | 1.5E1 | 323 | 7 | 191 | 7 | 0.48 |
| Un | ng/ml | 1.9E0 | 3.0E0 | 2.2E0 | 3.4E0 | 1.8E0 | 2.1E0 | 1.3E-1 | 8.5E-1 | 2.5E1 | 7.4E0 | 323 | 7 | 191 | 7 | 0.71 |
| Ug | ng/ml | 1.5E1 | 2.4E0 | 2.9E1 | 9.5E0 | 3.1E1 | 1.1E1 | 6.9E-1 | 1.3E0 | 2.1E2 | 2.7E1 | 323 | 7 | 191 | 7 | 0.24 |
| Ur | ng/ml | 1.5E-1 | 1.0E-9 | 8.0E-1 | 3.0E-1 | 5.3E0 | 6.8E-1 | 1.0E-9 | 1.0E-9 | 9.3E1 | 1.8E0 | 322 | 7 | 190 | 7 | 0.30 |
| Up | ng/ml | 1.0E-9 | 1.0E-9 | 1.2E-2 | 6.5E-3 | 1.3E-1 | 1.3E-2 | 1.0E-9 | 1.0E-9 | 2.4E0 | 3.5E-2 | 322 | 7 | 190 | 7 | 0.51 |
| Us | ng/ml | 3.6E-3 | 1.0E-9 | 2.4E-2 | 1.1E-3 | 1.0E-1 | 1.8E-3 | 1.0E-9 | 1.0E-9 | 1.7E0 | 4.8E-3 | 322 | 7 | 190 | 7 | 0.30 |
| Uv | ng/ml | 2.9E-3 | 2.0E-3 | 1.3E-2 | 4.4E-3 | 4.4E-2 | 4.9E-3 | 1.0E-9 | 1.0E-9 | 4.3E-1 | 1.3E-2 | 322 | 7 | 190 | 7 | 0.49 |
| Ut | ng/ml | 6.5E-1 | 2.3E0 | 2.8E0 | 5.5E0 | 8.9E0 | 1.1E1 | 1.0E-9 | 6.8E-2 | 7.8E1 | 3.0E1 | 322 | 7 | 190 | 7 | 0.60 |
| Uu | ng/ml | 7.1E0 | 7.2E0 | 8.2E0 | 7.0E0 | 5.8E0 | 4.7E0 | 4.5E-1 | 2.3E0 | 4.0E1 | 1.5E1 | 322 | 7 | 190 | 7 | 0.45 |
| Vp | ng/ml | 1.0E-9 | 1.0E-9 | 4.8E-1 | 5.1E-2 | 4.2E0 | 1.3E-1 | 1.0E-9 | 1.0E-9 | 5.0E1 | 3.6E-1 | 323 | 7 | 191 | 7 | 0.50 |
| Vt | ng/ml | 6.7E0 | 9.8E0 | 9.6E0 | 1.2E1 | 1.2E1 | 1.3E1 | 4.3E-1 | 1.7E0 | 1.6E2 | 3.8E1 | 323 | 7 | 191 | 7 | 0.54 |
| Vo | ng/ml | 2.5E1 | 2.5E1 | 2.4E1 | 2.3E1 | 5.3E0 | 3.1E0 | 2.4E0 | 1.7E1 | 4.8E1 | 2.6E1 | 323 | 7 | 191 | 7 | 0.37 |
| Vv | ng/ml | 2.9E0 | 3.4E0 | 5.9E0 | 1.5E1 | 9.4E0 | 2.9E1 | 1.0E-9 | 1.0E-9 | 8.4E1 | 8.0E1 | 321 | 7 | 190 | 7 | 0.54 |
| Oy | pg/ml | 4.9E-1 | 4.7E-1 | 5.7E0 | 5.5E-1 | 2.9E1 | 5.6E-1 | 1.0E-9 | 1.0E-9 | 4.0E2 | 2.1E0 | 904 | 13 | 336 | 13 | 0.45 |
| Oz | pg/ml | 3.1E-3 | 1.4E-1 | 3.1E-1 | 1.8E-1 | 1.3E0 | 1.9E-1 | 1.0E-9 | 1.0E-9 | 2.9E1 | 6.2E-1 | 904 | 13 | 336 | 13 | 0.55 |
| Pa | pg/ml | 3.9E-1 | 3.5E-1 | 1.6E0 | 1.2E0 | 6.1E0 | 2.0E0 | 1.0E-9 | 1.0E-9 | 1.0E2 | 6.5E0 | 904 | 13 | 336 | 13 | 0.47 |
| Pb | pg/ml | 1.0E-9 | 1.0E-9 | 8.0E-1 | 6.0E-2 | 1.6E1 | 1.0E-1 | 1.0E-9 | 1.0E-9 | 4.9E2 | 3.2E-1 | 904 | 13 | 336 | 13 | 0.44 |
| Pc | pg/ml | 4.4E-2 | 1.4E-1 | 3.6E-1 | 2.1E-1 | 8.8E-1 | 2.6E-1 | 1.0E-9 | 1.0E-9 | 1.6E1 | 8.5E-1 | 904 | 13 | 336 | 13 | 0.47 |
| Pd | pg/ml | 1.9E0 | 2.1E0 | 5.1E0 | 3.3E0 | 2.9E1 | 5.2E0 | 1.0E-9 | 1.0E-9 | 8.4E2 | 2.0E1 | 904 | 13 | 336 | 13 | 0.48 |
| Pe | pg/ml | 2.2E1 | 5.1E1 | 1.2E2 | 1.2E2 | 4.4E2 | 1.5E2 | 1.0E-9 | 1.0E-9 | 6.7E3 | 4.4E2 | 904 | 13 | 336 | 13 | 0.55 |
| Pf | pg/ml | 1.6E0 | 6.6E0 | 1.1E1 | 8.6E0 | 6.0E1 | 9.6E0 | 1.0E-9 | 1.0E-9 | 1.5E3 | 2.9E1 | 904 | 13 | 336 | 13 | 0.58 |
| Pg | pg/ml | 3.4E0 | 4.0E0 | 4.3E1 | 9.9E1 | 3.4E2 | 3.3E2 | 1.0E-9 | 1.0E-9 | 7.7E3 | 1.2E3 | 904 | 13 | 336 | 13 | 0.49 |
| Ph | ng/ml | 1.8E-1 | 8.8E-1 | 3.6E-1 | 1.0E0 | 6.1E-1 | 8.4E-1 | 1.0E-9 | 1.9E-2 | 5.4E0 | 2.3E0 | 317 | 7 | 187 | 7 | 0.75 |
| Pi | ng/ml | 2.0E-1 | 3.1E-1 | 5.4E-1 | 3.1E-1 | 4.6E0 | 2.2E-1 | 1.0E-9 | 1.1E-2 | 8.2E1 | 5.3E-1 | 317 | 7 | 187 | 7 | 0.59 |
| Pj | ng/mL | 5.6E0 | 5.8E0 | 6.3E0 | 7.1E0 | 4.5E0 | 5.3E0 | 3.8E-2 | 1.3E0 | 3.1E1 | 1.7E1 | 317 | 7 | 187 | 7 | 0.55 |
| Pk | ng/ml | 8.8E-3 | 1.3E-2 | 1.8E-2 | 1.5E-2 | 8.7E-2 | 1.1E-2 | 1.0E-9 | 1.0E-9 | 1.5E0 | 2.8E-2 | 317 | 7 | 187 | 7 | 0.61 |
| aA | mg/dL | 8.0E-1 | 1.3E0 | 9.4E-1 | 1.4E0 | 4.9E-1 | 8.1E-1 | 2.0E-1 | 5.0E-1 | 4.2E0 | 3.4E0 | 2667 | 17 | 507 | 17 | 0.71 |
| aC | mg/mL | 2.8E0 | 2.4E0 | 3.1E0 | 2.9E0 | 1.4E0 | 1.3E0 | 7.7E-1 | 1.7E0 | 8.9E0 | 5.6E0 | 535 | 7 | 207 | 7 | 0.47 |
| aD | ug/mL | 3.2E0 | 3.7E0 | 4.5E0 | 5.3E0 | 3.8E0 | 3.8E0 | 4.3E-1 | 1.3E0 | 3.5E1 | 1.1E1 | 535 | 7 | 207 | 7 | 0.58 |
| aE | mg/mL | 5.6E-1 | 6.3E-1 | 5.7E-1 | 6.6E-1 | 1.5E-1 | 1.7E-1 | 1.8E-1 | 4.8E-1 | 1.1E0 | 1.0E0 | 535 | 7 | 207 | 7 | 0.65 |
| aF | ng/mL | 2.2E0 | 1.1E0 | 4.0E0 | 2.8E0 | 5.7E0 | 4.4E0 | 4.3E-3 | 3.5E-1 | 5.0E1 | 1.3E1 | 535 | 7 | 207 | 7 | 0.34 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| aG | mg/mL | 1.4E-1 | 1.3E-1 | 1.6E-1 | 1.5E-1 | 8.7E-2 | 8.6E-2 | 1.7E-2 | 8.0E-2 | 5.4E-1 | 3.3E-1 | 535 | 7 | 207 | 7 | 0.49 |
| aH | ug/mL | 7.5E1 | 6.3E1 | 8.2E1 | 8.0E1 | 4.4E1 | 4.3E1 | 4.6E0 | 3.6E1 | 2.9E2 | 1.5E2 | 535 | 7 | 207 | 7 | 0.48 |
| aI | ug/mL | 1.9E2 | 1.6E2 | 1.9E2 | 1.7E2 | 6.0E1 | 5.0E1 | 2.8E1 | 1.1E2 | 3.7E2 | 2.6E2 | 535 | 7 | 207 | 7 | 0.40 |
| aJ | ug/mL | 2.5E0 | 4.6E0 | 3.1E0 | 5.8E0 | 2.2E0 | 3.6E0 | 7.3E-1 | 1.9E0 | 1.7E1 | 1.1E1 | 535 | 7 | 207 | 7 | 0.75 |
| aK | ng/mL | 1.6E0 | 1.6E0 | 2.4E0 | 1.8E0 | 2.6E0 | 1.5E0 | 2.9E-4 | 2.1E-1 | 1.8E1 | 5.0E0 | 535 | 7 | 207 | 7 | 0.48 |
| aL | mg/mL | 8.0E-1 | 8.3E-1 | 8.1E-1 | 7.7E-1 | 2.6E-1 | 1.9E-1 | 1.9E-1 | 4.0E-1 | 1.7E0 | 9.9E-1 | 535 | 7 | 207 | 7 | 0.48 |
| aM | U/mL | 2.2E1 | 1.9E1 | 4.9E1 | 2.4E1 | 1.1E2 | 1.5E1 | 4.2E-2 | 5.8E0 | 1.6E3 | 5.2E1 | 535 | 7 | 207 | 7 | 0.46 |
| aN | U/mL | 1.4E1 | 1.7E1 | 2.2E1 | 1.8E1 | 3.1E1 | 1.2E1 | 2.5E-3 | 3.9E0 | 3.8E2 | 3.7E1 | 535 | 7 | 207 | 7 | 0.52 |
| aO | pg/mL | 3.1E1 | 1.8E2 | 3.0E2 | 4.4E2 | 7.8E2 | 8.6E2 | 6.0E-2 | 1.2E1 | 6.6E3 | 2.4E3 | 535 | 7 | 207 | 7 | 0.68 |
| aP | ng/mL | 1.7E0 | 2.4E0 | 2.1E0 | 3.0E0 | 1.8E0 | 1.6E0 | 4.5E-1 | 1.3E0 | 2.8E1 | 5.3E0 | 535 | 7 | 207 | 7 | 0.71 |
| aQ | ng/mL | 3.0E-1 | 2.3E-1 | 4.5E-1 | 3.3E-1 | 4.5E-1 | 2.7E-1 | 2.0E-4 | 6.1E-2 | 4.0E0 | 7.8E-1 | 535 | 7 | 207 | 7 | 0.43 |
| aR | ng/mL | 1.7E0 | 2.6E0 | 2.8E0 | 3.1E0 | 3.3E0 | 1.7E0 | 1.8E-1 | 3.6E-1 | 3.4E1 | 5.4E0 | 535 | 7 | 207 | 7 | 0.65 |
| aS | ng/mL | 2.6E-1 | 1.2E0 | 6.3E-1 | 2.0E0 | 1.7E0 | 3.0E0 | 4.2E-3 | 8.5E-2 | 3.3E1 | 8.7E0 | 535 | 7 | 207 | 7 | 0.70 |
| aU | pg/mL | 7.5E1 | 1.0E2 | 1.3E2 | 1.0E2 | 1.5E2 | 6.1E1 | 7.4E-2 | 1.3E1 | 1.3E3 | 1.9E2 | 535 | 7 | 207 | 7 | 0.52 |
| aV | ng/mL | 6.2E-1 | 3.6E-1 | 1.1E0 | 6.6E-1 | 1.8E0 | 5.3E-1 | 7.6E-4 | 1.8E-1 | 3.3E1 | 1.5E0 | 535 | 7 | 207 | 7 | 0.44 |
| aW | pg/mL | 1.9E1 | 2.0E1 | 2.0E1 | 4.9E1 | 1.8E1 | 7.9E1 | 7.2E-2 | 7.2E-2 | 2.4E2 | 2.3E2 | 535 | 7 | 207 | 7 | 0.61 |
| aX | ng/mL | 9.5E0 | 1.2E1 | 1.5E1 | 3.9E1 | 1.8E1 | 7.7E1 | 3.0E-1 | 2.5E0 | 2.2E2 | 2.1E2 | 535 | 7 | 207 | 7 | 0.55 |
| aY | pg/mL | 5.7E1 | 1.1E2 | 7.5E1 | 1.6E2 | 8.1E1 | 1.2E2 | 4.1E-1 | 4.7E1 | 1.2E3 | 3.9E2 | 535 | 7 | 207 | 7 | 0.76 |
| aZ | pg/mL | 2.2E2 | 2.2E2 | 5.0E2 | 7.7E2 | 9.5E2 | 8.4E2 | 1.7E0 | 4.6E1 | 1.2E4 | 2.1E3 | 535 | 7 | 207 | 7 | 0.61 |
| bA | ng/mL | 8.8E0 | 9.0E1 | 3.5E1 | 2.0E2 | 9.7E1 | 2.2E2 | 3.0E-2 | 1.4E1 | 9.4E2 | 5.7E2 | 535 | 7 | 207 | 7 | 0.89 |
| bB | ng/mL | 3.0E2 | 1.9E2 | 3.2E2 | 3.2E2 | 1.7E2 | 2.2E2 | 2.1E0 | 1.1E2 | 1.0E3 | 6.2E2 | 535 | 7 | 207 | 7 | 0.46 |
| bC | ng/mL | 3.5E2 | 3.6E2 | 6.1E2 | 7.5E2 | 8.1E2 | 9.8E2 | 9.8E0 | 1.4E2 | 4.7E3 | 2.9E3 | 535 | 7 | 207 | 7 | 0.56 |
| bE | mg/mL | 5.5E0 | 6.7E0 | 5.8E0 | 7.6E0 | 2.1E0 | 3.3E0 | 9.8E-1 | 3.7E0 | 1.3E1 | 1.3E1 | 535 | 7 | 207 | 7 | 0.66 |
| bF | pg/mL | 2.1E1 | 2.3E1 | 1.6E2 | 2.9E2 | 8.9E2 | 7.0E2 | 5.0E-2 | 9.4E0 | 1.1E4 | 1.9E3 | 535 | 7 | 207 | 7 | 0.55 |
| bG | ng/mL | 1.6E0 | 1.9E0 | 2.7E0 | 2.2E0 | 3.2E0 | 1.8E0 | 2.2E-2 | 4.0E-1 | 2.6E1 | 4.8E0 | 535 | 7 | 207 | 7 | 0.51 |
| bH | pg/mL | 5.7E-1 | 6.8E0 | 4.8E0 | 5.6E0 | 1.4E1 | 5.8E0 | 5.7E-1 | 5.7E-1 | 2.8E2 | 1.7E1 | 535 | 7 | 207 | 7 | 0.60 |
| bI | ng/mL | 4.0E-3 | 1.4E-1 | 6.2E-2 | 2.0E-1 | 1.6E-1 | 2.8E-1 | 4.0E-3 | 4.0E-3 | 1.3E0 | 7.7E-1 | 535 | 7 | 207 | 7 | 0.70 |
| bJ | mg/mL | 2.3E0 | 2.6E0 | 2.7E0 | 2.8E0 | 2.1E0 | 9.7E-1 | 2.5E-4 | 1.3E0 | 1.3E1 | 4.0E0 | 535 | 7 | 207 | 7 | 0.59 |
| bL | pg/mL | 3.7E0 | 5.8E0 | 8.1E0 | 9.4E0 | 1.0E1 | 1.1E1 | 4.6E-2 | 4.6E-2 | 8.0E1 | 3.2E1 | 535 | 7 | 207 | 7 | 0.59 |
| bM | mg/mL | 1.7E0 | 2.0E0 | 2.1E0 | 2.1E0 | 1.5E0 | 8.6E-1 | 9.2E-3 | 1.2E0 | 8.9E0 | 3.7E0 | 535 | 7 | 207 | 7 | 0.56 |
| bN | ng/mL | 4.5E1 | 2.7E1 | 1.3E2 | 3.1E1 | 2.8E2 | 2.2E1 | 1.4E-1 | 2.9E0 | 1.9E3 | 6.2E1 | 535 | 7 | 207 | 7 | 0.37 |
| bO | ng/mL | 4.0E-2 | 4.0E-2 | 1.1E1 | 3.0E0 | 2.5E1 | 6.2E0 | 4.0E-2 | 4.0E-2 | 2.0E2 | 1.7E1 | 535 | 7 | 207 | 7 | 0.43 |
| bP | mg/mL | 5.4E-1 | 1.0E0 | 7.7E-1 | 1.0E0 | 6.8E-1 | 9.5E-1 | 4.9E-2 | 9.7E-2 | 4.8E0 | 2.9E0 | 535 | 7 | 207 | 7 | 0.57 |
| bQ | pg/mL | 1.6E1 | 1.5E1 | 5.8E1 | 3.7E1 | 5.9E2 | 3.3E1 | 1.5E-1 | 4.4E0 | 1.3E4 | 8.8E1 | 535 | 7 | 207 | 7 | 0.59 |
| bR | ng/mL | 1.2E-2 | 1.2E-2 | 1.3E-1 | 4.2E-2 | 4.2E-1 | 8.0E-2 | 1.2E-2 | 1.2E-2 | 8.7E0 | 2.2E-1 | 535 | 7 | 207 | 7 | 0.38 |
| bS | pg/mL | 9.4E-1 | 9.4E-1 | 6.8E0 | 9.4E-1 | 2.6E1 | 0.0E0 | 9.4E-1 | 9.4E-1 | 3.9E2 | 9.4E-1 | 535 | 7 | 207 | 7 | 0.43 |
| bU | ng/mL | 1.2E-1 | 1.2E-1 | 1.9E-1 | 1.2E-1 | 3.5E-1 | 1.2E-1 | 1.3E-2 | 1.3E-2 | 6.6E0 | 3.4E-1 | 535 | 7 | 207 | 7 | 0.44 |
| bV | pg/mL | 4.7E2 | 1.0E3 | 5.6E2 | 9.3E2 | 5.6E2 | 4.2E2 | 1.5E2 | 3.7E2 | 1.2E4 | 1.6E3 | 535 | 7 | 207 | 7 | 0.77 |
| bW | pg/mL | 3.3E2 | 3.7E2 | 6.1E2 | 4.2E2 | 1.7E3 | 2.2E2 | 8.4E1 | 1.8E2 | 2.5E4 | 8.4E2 | 535 | 7 | 207 | 7 | 0.54 |
| bX | ng/mL | 1.4E-3 | 2.5E-5 | 2.8E-3 | 4.5E-4 | 3.4E-3 | 1.1E-3 | 2.5E-5 | 2.5E-5 | 2.0E-2 | 3.0E-3 | 535 | 7 | 207 | 7 | 0.29 |
| bZ | pg/mL | 2.4E2 | 2.8E2 | 8.3E2 | 9.5E2 | 3.7E3 | 1.2E3 | 1.5E-1 | 2.6E2 | 5.8E4 | 3.4E3 | 535 | 7 | 207 | 7 | 0.69 |
| cA | pg/mL | 6.0E-1 | 6.0E-1 | 2.6E0 | 6.0E-1 | 1.6E1 | 0.0E0 | 6.0E-1 | 6.0E-1 | 3.7E2 | 6.0E-1 | 535 | 7 | 207 | 7 | 0.44 |
| cB | ng/mL | 5.7E-2 | 5.3E-2 | 8.8E-2 | 5.4E-2 | 1.0E-1 | 4.1E-2 | 1.7E-3 | 1.7E-3 | 5.7E-1 | 1.2E-1 | 535 | 7 | 207 | 7 | 0.44 |
| cC | pg/mL | 4.6E1 | 3.4E1 | 4.7E1 | 3.9E1 | 3.9E1 | 1.6E1 | 1.0E0 | 2.1E1 | 4.5E2 | 5.8E1 | 535 | 7 | 207 | 7 | 0.44 |
| cD | pg/mL | 5.3E0 | 3.1E0 | 1.5E1 | 8.8E0 | 5.2E1 | 1.4E1 | 3.3E-1 | 3.3E-1 | 7.2E2 | 3.9E1 | 535 | 7 | 207 | 7 | 0.42 |
| cE | pg/mL | 3.8E1 | 9.1E1 | 1.6E2 | 1.5E2 | 4.6E2 | 1.7E2 | 1.2E-1 | 6.4E0 | 3.8E3 | 4.5E2 | 535 | 7 | 207 | 7 | 0.58 |
| cF | pg/mL | 1.2E1 | 5.3E-1 | 2.0E1 | 5.9E0 | 3.0E1 | 7.1E0 | 5.3E-1 | 5.3E-1 | 2.7E2 | 1.7E1 | 535 | 7 | 207 | 7 | 0.35 |
| cG | pg/mL | 4.6E1 | 6.5E1 | 1.0E2 | 1.0E2 | 4.8E2 | 1.1E2 | 6.4E0 | 2.0E1 | 1.0E4 | 3.3E2 | 535 | 7 | 207 | 7 | 0.61 |
| cH | uIU/mL | 2.8E0 | 1.6E0 | 6.0E0 | 4.0E0 | 1.2E1 | 5.7E0 | 8.6E-3 | 8.6E-3 | 1.6E2 | 1.6E1 | 535 | 7 | 207 | 7 | 0.39 |
| cI | ng/mL | 5.7E0 | 1.0E1 | 1.2E1 | 4.4E1 | 1.6E1 | 6.9E1 | 1.0E-3 | 7.9E-1 | 1.2E2 | 1.9E2 | 535 | 7 | 207 | 7 | 0.64 |
| cJ | ug/mL | 6.2E1 | 7.6E1 | 1.1E2 | 7.9E1 | 1.4E2 | 4.7E1 | 4.0E0 | 1.8E1 | 9.6E2 | 1.6E2 | 535 | 7 | 207 | 7 | 0.52 |
| cK | ng/mL | 3.8E-3 | 3.8E-3 | 4.8E-2 | 1.4E-2 | 1.7E-1 | 2.6E-2 | 3.8E-3 | 3.8E-3 | 1.7E0 | 7.3E-2 | 535 | 7 | 207 | 7 | 0.49 |
| cL | pg/mL | 2.0E2 | 2.2E2 | 3.8E2 | 2.9E2 | 1.2E3 | 2.6E2 | 1.6E1 | 1.2E2 | 2.4E4 | 8.7E2 | 535 | 7 | 207 | 7 | 0.55 |

| Code | Units | Median | | Average | | Standard Deviation | | Minimum | | Maximum | | Number of Samples | | Number of Patients | | AUC |
|------|-------|--------|------|---------|------|-------|------|---------|------|---------|------|--------|-----|----------|-----|-----|
| | | NonDis | Dis | NonDis | Dis | NonDis | Dis | NonDis | Dis | NonDis | Dis | NonDis | Dis | NonDis | Dis | |
| cM | pg/mL | 2.7E2 | 2.1E2 | 2.9E2 | 2.5E2 | 1.9E2 | 1.3E2 | 8.7E0 | 1.2E2 | 1.6E3 | 4.9E2 | 535 | 7 | 207 | 7 | 0.42 |
| cN | pg/mL | 1.2E2 | 1.7E2 | 1.3E2 | 1.7E2 | 6.2E1 | 6.2E1 | 3.8E1 | 1.0E2 | 1.1E3 | 2.8E2 | 535 | 7 | 207 | 7 | 0.73 |
| cO | pg/mL | 2.2E2 | 2.4E2 | 3.0E2 | 2.7E2 | 8.5E2 | 8.3E1 | 5.4E1 | 1.7E2 | 1.9E4 | 3.9E2 | 535 | 7 | 207 | 7 | 0.61 |
| cP | ng/mL | 2.5E3 | 3.8E3 | 2.6E3 | 3.8E3 | 9.0E2 | 1.0E3 | 6.2E2 | 2.3E3 | 5.7E3 | 5.1E3 | 535 | 7 | 207 | 7 | 0.82 |
| cQ | ng/mL | 4.9E-2 | 1.9E-1 | 1.4E-1 | 3.3E-1 | 2.8E-1 | 3.1E-1 | 2.0E-3 | 6.7E-2 | 2.2E0 | 9.3E-1 | 535 | 7 | 207 | 7 | 0.80 |
| cR | ng/mL | 2.9E2 | 6.9E2 | 5.0E2 | 6.8E2 | 7.8E2 | 2.4E2 | 2.0E1 | 3.4E2 | 8.9E3 | 1.0E3 | 535 | 7 | 207 | 7 | 0.77 |
| cS | ng/mL | 2.6E2 | 6.1E2 | 3.8E2 | 5.6E2 | 3.8E2 | 2.2E2 | 4.1E1 | 2.4E2 | 2.7E3 | 8.7E2 | 535 | 7 | 207 | 7 | 0.77 |
| cT | ng/mL | 3.3E1 | 1.5E2 | 8.7E1 | 4.0E2 | 1.9E2 | 4.3E2 | 3.6E0 | 1.8E1 | 2.1E3 | 1.2E3 | 535 | 7 | 207 | 7 | 0.81 |
| cU | ng/mL | 5.4E1 | 8.0E1 | 7.5E1 | 8.9E1 | 9.4E1 | 4.5E1 | 5.4E0 | 5.2E1 | 1.6E3 | 1.8E2 | 535 | 7 | 207 | 7 | 0.67 |
| cV | ng/mL | 1.8E-1 | 1.1E-1 | 3.9E-1 | 1.8E-1 | 2.1E0 | 1.2E-1 | 3.4E-4 | 7.6E-2 | 4.7E1 | 3.8E-1 | 535 | 7 | 207 | 7 | 0.44 |
| cW | mIU/mL | 5.2E-2 | 6.5E-2 | 1.3E-1 | 9.2E-2 | 6.5E-1 | 6.4E-2 | 3.7E-4 | 4.0E-2 | 9.7E0 | 2.0E-1 | 535 | 7 | 207 | 7 | 0.63 |
| cX | ng/mL | 1.1E-1 | 3.2E-2 | 1.3E0 | 5.4E-1 | 4.2E0 | 9.0E-1 | 2.3E-4 | 1.4E-2 | 2.8E1 | 2.5E0 | 535 | 7 | 207 | 7 | 0.49 |
| cY | ng/mL | 8.6E0 | 1.0E1 | 1.3E1 | 1.1E1 | 1.3E1 | 9.1E0 | 1.5E-1 | 6.6E-1 | 8.3E1 | 2.3E1 | 535 | 7 | 207 | 7 | 0.48 |
| cZ | ug/mL | 1.5E1 | 1.5E1 | 1.6E1 | 1.9E1 | 7.2E0 | 1.0E1 | 2.3E0 | 8.0E0 | 5.7E1 | 4.0E1 | 535 | 7 | 207 | 7 | 0.58 |
| dA | pg/mL | 3.3E2 | 5.4E2 | 3.7E2 | 5.3E2 | 2.9E2 | 1.5E2 | 9.0E1 | 3.2E2 | 5.8E3 | 7.7E2 | 535 | 7 | 207 | 7 | 0.80 |
| dB | ug/mL | 1.7E1 | 1.9E1 | 1.7E1 | 1.8E1 | 1.5E1 | 8.4E0 | 9.4E-1 | 2.8E0 | 2.5E2 | 2.9E1 | 535 | 7 | 207 | 7 | 0.60 |
| dC | nmol/L | 3.5E1 | 4.0E1 | 3.9E1 | 4.2E1 | 1.8E1 | 1.8E1 | 7.6E0 | 2.2E1 | 1.4E2 | 7.5E1 | 535 | 7 | 207 | 7 | 0.57 |
| dD | ug/mL | 3.6E1 | 3.3E1 | 3.7E1 | 3.4E1 | 1.1E1 | 1.4E1 | 1.3E1 | 1.5E1 | 7.6E1 | 6.2E1 | 535 | 7 | 207 | 7 | 0.40 |
| dE | ng/mL | 4.7E-1 | 4.2E-1 | 5.9E-1 | 6.4E-1 | 6.9E-1 | 8.1E-1 | 8.4E-3 | 8.4E-3 | 7.2E0 | 2.4E0 | 535 | 7 | 207 | 7 | 0.49 |
| dF | ng/mL | 2.3E2 | 3.5E2 | 2.8E2 | 3.7E2 | 2.0E2 | 2.2E2 | 5.6E1 | 1.3E2 | 1.3E3 | 7.8E2 | 535 | 7 | 207 | 7 | 0.65 |
| dG | ng/mL | 1.1E1 | 1.4E1 | 1.5E1 | 1.9E1 | 1.3E1 | 8.7E0 | 2.2E0 | 9.2E0 | 1.8E2 | 3.2E1 | 535 | 7 | 207 | 7 | 0.70 |
| dH | pg/mL | 7.7E0 | 8.0E0 | 1.3E1 | 1.0E1 | 3.6E1 | 7.7E0 | 4.0E-2 | 2.2E0 | 6.7E2 | 2.3E1 | 535 | 7 | 207 | 7 | 0.54 |
| dI | pg/mL | 4.6E-1 | 2.1E0 | 2.1E0 | 2.8E0 | 1.5E1 | 2.9E0 | 4.6E-1 | 4.6E-1 | 3.3E2 | 7.5E0 | 535 | 7 | 207 | 7 | 0.68 |
| dJ | ng/mL | 1.9E0 | 2.1E0 | 2.1E0 | 2.2E0 | 1.2E0 | 1.0E0 | 3.2E-2 | 7.9E-1 | 6.9E0 | 3.4E0 | 535 | 7 | 207 | 7 | 0.53 |
| dK | uIU/mL | 1.9E0 | 1.4E0 | 3.1E0 | 1.5E0 | 6.1E0 | 1.1E0 | 2.8E-4 | 4.2E-2 | 7.9E1 | 3.1E0 | 535 | 7 | 207 | 7 | 0.39 |
| dL | ng/mL | 8.8E2 | 1.2E3 | 1.0E3 | 1.1E3 | 5.7E2 | 1.9E2 | 2.6E2 | 7.5E2 | 4.8E3 | 1.3E3 | 535 | 7 | 207 | 7 | 0.64 |
| dM | pg/mL | 9.7E2 | 2.5E3 | 1.3E3 | 2.5E3 | 1.3E3 | 1.8E3 | 3.4E2 | 7.3E2 | 1.6E4 | 5.9E3 | 535 | 7 | 207 | 7 | 0.75 |
| dN | ug/mL | 9.3E1 | 1.4E2 | 1.0E2 | 1.3E2 | 4.0E1 | 4.6E1 | 1.6E1 | 6.9E1 | 3.3E2 | 1.9E2 | 535 | 7 | 207 | 7 | 0.72 |
| dR | pg/ml | 1.6E3 | 1.6E3 | 2.3E3 | 2.7E3 | 2.3E3 | 2.9E3 | 1.4E2 | 4.8E2 | 1.5E4 | 8.7E3 | 365 | 7 | 197 | 7 | 0.52 |
| eF | ng/ml | 4.1E0 | 6.9E0 | 5.0E0 | 7.2E0 | 4.0E0 | 2.4E0 | 1.2E0 | 4.1E0 | 4.6E1 | 1.2E1 | 380 | 7 | 198 | 7 | 0.81 |
| fP | ng/ml | 2.6E2 | 3.3E2 | 2.9E2 | 3.6E2 | 1.7E2 | 9.4E1 | 1.8E0 | 2.4E2 | 1.0E3 | 4.8E2 | 346 | 7 | 189 | 7 | 0.68 |
| gL | pg/ml | 6.5E4 | 1.1E5 | 7.0E4 | 1.1E5 | 2.9E4 | 5.2E4 | 1.4E4 | 5.2E4 | 2.0E5 | 1.7E5 | 365 | 7 | 197 | 7 | 0.74 |
| gP | U/ml | 2.7E2 | 2.6E2 | 2.8E2 | 2.8E2 | 1.1E2 | 7.2E1 | 1.2E1 | 2.0E2 | 1.1E3 | 3.8E2 | 376 | 7 | 198 | 7 | 0.51 |
| gW | ng/ml | 6.1E2 | 8.2E2 | 1.2E3 | 1.3E3 | 1.6E3 | 1.8E3 | 3.1E-1 | 3.1E2 | 9.5E3 | 5.4E3 | 318 | 7 | 187 | 7 | 0.57 |

Figure 20.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 6.5E1 | 7.3E1 | 7.6E1 | 7.1E1 | 5.2E1 | 4.6E1 | 2.0E0 | 1.2E1 | 4.0E2 | 1.5E2 | 1392 | 18 | 230 | 18 | 0.48 |
| Ad | ug/mL | 3.4E-2 | 5.0E-2 | 6.5E-2 | 9.6E-2 | 8.4E-2 | 1.0E-1 | 6.8E-4 | 6.9E-3 | 5.4E-1 | 3.5E-1 | 357 | 17 | 135 | 17 | 0.62 |
| Af | ng/mL | 1.0E0 | 9.8E-1 | 1.4E1 | 4.7E1 | 6.0E1 | 1.3E2 | 1.7E-3 | 1.7E-3 | 5.3E2 | 4.8E2 | 357 | 17 | 135 | 17 | 0.54 |
| Aj | ug/mL | 1.8E0 | 2.6E-1 | 2.7E0 | 1.8E0 | 2.5E0 | 2.4E0 | 1.5E-3 | 5.5E-3 | 6.1E0 | 5.8E0 | 357 | 17 | 135 | 17 | 0.35 |
| Al | mg/mL | 8.7E-5 | 8.2E-5 | 2.5E-4 | 3.0E-4 | 4.1E-4 | 4.8E-4 | 2.5E-6 | 2.3E-6 | 1.9E-3 | 1.5E-3 | 357 | 17 | 135 | 17 | 0.51 |
| An | U/mL | 4.9E1 | 6.9E1 | 1.6E2 | 2.8E2 | 4.6E2 | 3.3E2 | 9.8E-4 | 9.0E-1 | 5.5E3 | 9.1E2 | 357 | 17 | 135 | 17 | 0.60 |
| Ao | pg/mL | 8.5E1 | 8.2E1 | 6.0E2 | 1.3E2 | 3.9E3 | 1.5E2 | 2.8E0 | 5.4E0 | 3.9E4 | 6.5E2 | 357 | 17 | 135 | 17 | 0.51 |
| Ap | ng/mL | 3.1E1 | 4.1E1 | 4.2E1 | 4.5E1 | 4.3E1 | 3.2E1 | 8.4E-5 | 3.2E0 | 2.9E2 | 1.3E2 | 357 | 17 | 135 | 17 | 0.56 |
| Ar | ng/mL | 8.5E-1 | 1.1E0 | 1.5E1 | 4.4E0 | 2.2E2 | 1.1E1 | 3.4E-3 | 1.5E-1 | 4.1E3 | 4.7E1 | 357 | 17 | 135 | 17 | 0.54 |
| As | ng/mL | 9.5E-3 | 1.2E-2 | 1.3E-2 | 1.3E-2 | 1.8E-2 | 1.4E-2 | 1.7E-3 | 1.7E-3 | 1.3E-1 | 6.1E-2 | 357 | 17 | 135 | 17 | 0.54 |
| Aw | pg/mL | 1.6E1 | 2.0E1 | 1.6E1 | 1.8E1 | 5.9E0 | 4.8E0 | 2.9E-2 | 1.1E1 | 4.8E1 | 2.5E1 | 357 | 17 | 135 | 17 | 0.63 |
| Ax | ng/mL | 2.2E0 | 6.1E0 | 1.4E1 | 1.1E1 | 5.8E1 | 1.7E1 | 1.3E-2 | 9.6E-2 | 7.7E2 | 6.0E1 | 357 | 17 | 135 | 17 | 0.58 |
| Ba | ng/mL | 6.2E1 | 1.7E2 | 4.2E2 | 8.8E2 | 1.2E3 | 2.0E3 | 3.7E-1 | 4.1E0 | 8.1E3 | 8.1E3 | 357 | 17 | 135 | 17 | 0.62 |
| Bb | ng/mL | 2.8E0 | 3.8E0 | 6.1E0 | 4.4E0 | 1.5E1 | 3.8E0 | 4.1E-3 | 5.2E-1 | 2.5E2 | 1.2E1 | 357 | 17 | 135 | 17 | 0.51 |
| Bc | ng/mL | 3.4E1 | 6.9E1 | 1.0E2 | 1.3E2 | 1.9E2 | 2.1E2 | 1.1E-1 | 7.0E0 | 1.2E3 | 8.9E2 | 357 | 17 | 135 | 17 | 0.64 |
| Bg | ng/mL | 7.7E-2 | 2.8E-1 | 4.0E0 | 2.4E1 | 1.9E1 | 9.6E1 | 5.3E-4 | 5.3E-4 | 2.5E2 | 4.0E2 | 357 | 17 | 135 | 17 | 0.58 |
| Bn | ng/mL | 5.6E-2 | 5.6E-2 | 1.1E0 | 1.5E0 | 1.9E0 | 2.1E0 | 5.6E-2 | 5.6E-2 | 9.7E0 | 6.5E0 | 357 | 17 | 135 | 17 | 0.54 |
| Bo | ng/mL | 1.2E1 | 2.2E1 | 1.4E1 | 2.0E1 | 2.0E1 | 1.1E1 | 1.6E-2 | 1.6E-2 | 2.8E2 | 3.9E1 | 357 | 17 | 135 | 17 | 0.69 |
| Ch | uIU/mL | 1.2E0 | 9.6E-1 | 2.1E1 | 7.2E1 | 1.1E2 | 2.9E2 | 3.4E-3 | 3.4E-3 | 1.8E3 | 1.2E3 | 357 | 17 | 135 | 17 | 0.42 |
| Co | pg/mL | 3.8E1 | 4.6E1 | 1.8E2 | 7.4E1 | 1.1E3 | 1.1E2 | 1.5E-1 | 6.2E0 | 1.7E4 | 5.0E2 | 357 | 17 | 135 | 17 | 0.52 |
| Cp | ng/mL | 2.2E1 | 2.9E1 | 2.8E1 | 3.1E1 | 3.3E1 | 1.8E1 | 6.0E-1 | 2.5E0 | 3.7E2 | 7.0E1 | 357 | 17 | 135 | 17 | 0.60 |
| Cq | ng/mL | 2.8E-2 | 5.0E-2 | 1.5E-1 | 8.7E-2 | 9.5E-1 | 1.1E-1 | 8.0E-4 | 8.0E-4 | 1.7E1 | 4.0E-1 | 357 | 17 | 135 | 17 | 0.59 |
| Cs | ng/mL | 6.6E1 | 2.1E2 | 3.0E2 | 2.5E2 | 8.6E2 | 3.0E2 | 2.7E-2 | 4.4E0 | 1.1E4 | 1.2E3 | 357 | 17 | 135 | 17 | 0.58 |
| Ct | ng/mL | 8.9E-1 | 1.8E-1 | 4.0E1 | 5.3E0 | 1.1E2 | 1.2E1 | 1.1E-4 | 2.5E-2 | 6.2E2 | 4.9E1 | 357 | 17 | 135 | 17 | 0.39 |
| Cu | ng/mL | 2.4E-1 | 3.1E-1 | 4.0E-1 | 4.3E-1 | 7.0E-1 | 4.1E-1 | 9.6E-3 | 4.6E-2 | 9.2E0 | 1.7E0 | 357 | 17 | 135 | 17 | 0.58 |
| Cv | ng/mL | 4.1E0 | 8.7E0 | 1.8E1 | 3.2E1 | 4.9E1 | 5.1E1 | 1.4E-4 | 2.4E-2 | 5.3E2 | 1.7E2 | 357 | 17 | 135 | 17 | 0.59 |
| Cw | mIU/mL | 3.0E-2 | 3.7E-2 | 3.8E-2 | 3.8E-2 | 3.2E-2 | 1.8E-2 | 8.9E-4 | 9.0E-3 | 2.4E-1 | 6.4E-2 | 357 | 17 | 135 | 17 | 0.56 |
| Cx | ng/mL | 1.5E-1 | 6.2E-1 | 5.1E1 | 9.0E1 | 9.9E1 | 1.5E2 | 9.3E-5 | 9.3E-5 | 3.9E2 | 3.9E2 | 357 | 17 | 135 | 17 | 0.58 |
| Db | ug/mL | 7.2E0 | 7.3E0 | 8.6E0 | 8.8E0 | 7.3E0 | 7.2E0 | 4.5E-1 | 9.7E-1 | 5.9E1 | 3.1E1 | 357 | 17 | 135 | 17 | 0.51 |
| Dc | nmol/L | 1.9E-2 | 2.4E-2 | 5.7E-2 | 7.9E-2 | 1.4E-1 | 1.5E-1 | 5.2E-6 | 1.6E-3 | 1.6E0 | 6.3E-1 | 357 | 17 | 135 | 17 | 0.61 |
| Dd | ug/mL | 6.9E-2 | 1.4E-1 | 1.8E-1 | 1.7E-1 | 2.7E-1 | 1.7E-1 | 1.9E-4 | 7.7E-3 | 1.9E0 | 6.0E-1 | 357 | 17 | 135 | 17 | 0.60 |
| De | ng/mL | 3.4E-3 | 3.4E-3 | 7.8E-2 | 7.1E-2 | 1.4E-1 | 1.0E-1 | 3.4E-3 | 3.4E-3 | 9.2E-1 | 3.2E-1 | 357 | 17 | 135 | 17 | 0.51 |
| Dg | ng/mL | 2.9E1 | 4.7E1 | 4.2E1 | 5.8E1 | 3.8E1 | 4.2E1 | 1.0E-1 | 2.2E0 | 1.9E2 | 1.9E2 | 357 | 17 | 135 | 17 | 0.63 |
| Di | pg/mL | 2.0E0 | 1.6E0 | 2.2E0 | 2.4E0 | 2.0E0 | 2.3E0 | 1.8E-1 | 1.8E-1 | 1.3E1 | 9.2E0 | 357 | 17 | 135 | 17 | 0.52 |
| Dk | uIU/mL | 1.7E-2 | 1.8E-2 | 9.6E-2 | 6.3E-2 | 5.7E-1 | 9.2E-2 | 1.1E-4 | 1.1E-4 | 8.9E0 | 3.3E-1 | 357 | 17 | 135 | 17 | 0.53 |
| Dl | ng/mL | 2.3E2 | 2.8E2 | 3.1E2 | 3.3E2 | 2.8E2 | 3.1E2 | 1.7E0 | 1.7E1 | 1.5E3 | 1.3E3 | 357 | 17 | 135 | 17 | 0.52 |
| Dp | ng/ml | 2.3E0 | 3.0E0 | 5.1E0 | 4.6E0 | 7.9E0 | 5.6E0 | 3.7E-3 | 3.7E-3 | 4.6E1 | 1.8E1 | 217 | 15 | 135 | 15 | 0.48 |
| Ef | ng/ml | 1.5E-1 | 1.3E-1 | 8.1E-1 | 1.1E0 | 1.6E0 | 2.6E0 | 5.7E-4 | 4.1E-3 | 9.5E0 | 9.4E0 | 261 | 16 | 134 | 16 | 0.48 |
| Wm | % | 4.9E-1 | 2.4E0 | 2.3E1 | 4.8E1 | 1.6E2 | 1.9E2 | 5.4E-2 | 8.5E-2 | 2.4E3 | 7.7E2 | 283 | 17 | 151 | 17 | 0.58 |
| Ed | pg/ml | 5.2E-1 | 2.9E1 | 6.3E1 | 4.2E1 | 5.0E2 | 4.2E1 | 5.2E-1 | 5.2E-1 | 7.3E3 | 1.1E2 | 217 | 15 | 134 | 15 | 0.62 |
| Tj | pg/mL | 3.7E-1 | 3.7E-1 | 6.8E1 | 6.7E0 | 3.3E2 | 1.5E1 | 3.6E-1 | 3.7E-1 | 3.5E3 | 5.6E1 | 258 | 15 | 136 | 15 | 0.46 |
| Po | pg/ml | 6.7E-1 | 2.6E0 | 9.1E0 | 6.0E0 | 2.5E1 | 7.4E0 | 8.0E-3 | 2.6E-2 | 2.7E2 | 2.1E1 | 580 | 25 | 207 | 25 | 0.55 |
| Em | ng/ml | 9.2E-3 | 1.4E-2 | 6.7E-2 | 9.5E-2 | 1.2E-1 | 1.8E-1 | 1.9E-16 | 2.9E-3 | 6.0E-1 | 5.0E-1 | 149 | 7 | 71 | 7 | 0.54 |
| Et | ng/ml | 1.3E3 | 2.2E3 | 1.6E3 | 2.2E3 | 1.1E3 | 1.3E3 | 7.7E1 | 1.4E2 | 5.0E3 | 4.1E3 | 579 | 25 | 207 | 25 | 0.65 |
| Fa | ng/ml | 4.0E1 | 8.9E1 | 1.3E2 | 8.8E1 | 6.0E2 | 5.9E1 | 3.4E-2 | 1.6E0 | 8.0E3 | 2.2E2 | 212 | 16 | 133 | 16 | 0.67 |
| Ez | ng/ml | 5.0E0 | 4.1E0 | 2.0E1 | 1.1E1 | 5.9E1 | 1.3E1 | 1.3E-2 | 1.3E-2 | 7.1E2 | 3.4E1 | 217 | 15 | 135 | 15 | 0.49 |
| Fb | ng/ml | 2.5E1 | 2.7E1 | 2.3E1 | 2.3E1 | 1.1E1 | 1.1E1 | 6.6E-1 | 8.1E-1 | 5.7E1 | 3.5E1 | 213 | 16 | 133 | 16 | 0.50 |
| Ex | ng/ml | 7.8E-2 | 9.5E-2 | 2.5E-1 | 1.4E-1 | 7.6E-1 | 1.4E-1 | 3.5E-5 | 1.7E-4 | 8.9E0 | 5.0E-1 | 190 | 13 | 89 | 13 | 0.54 |
| Fn | ng/ml | 2.1E-1 | 2.1E-1 | 6.3E0 | 2.2E0 | 2.9E1 | 4.1E0 | 1.1E-14 | 1.1E-14 | 4.2E2 | 1.6E1 | 217 | 15 | 135 | 15 | 0.43 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fp | ng/ml | 1.2E1 | 2.2E1 | 2.4E1 | 3.1E1 | 2.8E1 | 2.9E1 | 6.0E-3 | 2.8E-1 | 1.4E2 | 1.3E2 | 606 | 25 | 209 | 25 | 0.59 |
| Fr | ng/ml | 3.3E4 | 5.6E4 | 1.1E5 | 2.0E5 | 1.7E5 | 2.7E5 | 1.9E2 | 3.2E2 | 9.0E5 | 8.3E5 | 686 | 25 | 210 | 25 | 0.62 |
| Fw | pg/ml | 8.5E-1 | 2.7E0 | 7.0E1 | 1.2E1 | 5.4E2 | 1.8E1 | 1.1E-14 | 1.2E-1 | 6.9E3 | 5.3E1 | 259 | 16 | 135 | 16 | 0.59 |
| Fy | ng/ml | 3.5E1 | 2.2E1 | 5.5E1 | 5.6E1 | 5.7E1 | 7.7E1 | 1.2E-1 | 1.5E0 | 3.3E2 | 2.3E2 | 216 | 14 | 134 | 14 | 0.47 |
| Gl | pg/ml | 7.8E3 | 7.9E3 | 1.1E4 | 1.1E4 | 9.4E3 | 8.8E3 | 9.1E1 | 2.5E2 | 3.3E4 | 2.8E4 | 252 | 16 | 135 | 16 | 0.51 |
| Gp | U/ml | 1.5E0 | 9.1E-1 | 4.0E0 | 2.9E0 | 6.8E0 | 5.3E0 | 1.3E-3 | 1.5E-2 | 6.7E1 | 2.0E1 | 261 | 16 | 135 | 16 | 0.43 |
| Gz | ug/ml | 1.4E0 | 8.1E-1 | 9.7E0 | 3.8E0 | 4.2E1 | 6.0E0 | 2.9E-16 | 3.8E-3 | 4.8E2 | 1.9E1 | 143 | 13 | 86 | 13 | 0.45 |
| Ha | ng/ml | 2.7E0 | 2.4E0 | 9.9E0 | 5.7E0 | 2.1E1 | 8.3E0 | 1.7E-2 | 1.7E-2 | 1.3E2 | 3.3E1 | 215 | 15 | 134 | 15 | 0.50 |
| Nm | pg/ml | 1.6E4 | 1.4E4 | 3.2E4 | 2.9E4 | 8.8E4 | 3.3E4 | 1.0E-9 | 1.0E-9 | 1.6E6 | 1.2E5 | 583 | 25 | 209 | 25 | 0.52 |
| Nn | pg/ml | 1.5E2 | 4.7E2 | 2.2E3 | 8.3E2 | 9.4E3 | 1.2E3 | 1.0E-9 | 1.0E-9 | 1.0E5 | 4.7E3 | 583 | 25 | 209 | 25 | 0.58 |
| No | pg/ml | 1.5E1 | 2.1E1 | 3.7E1 | 3.0E1 | 1.3E2 | 3.7E1 | 1.0E-9 | 1.0E-9 | 2.5E3 | 1.4E2 | 583 | 25 | 209 | 25 | 0.52 |
| Nq | pg/ml | 2.0E0 | 4.0E0 | 1.9E1 | 2.0E1 | 7.6E1 | 5.0E1 | 1.0E-9 | 1.0E-9 | 1.2E3 | 2.4E2 | 583 | 25 | 209 | 25 | 0.56 |
| Nr | pg/ml | 8.8E-1 | 1.5E0 | 3.3E1 | 1.4E1 | 2.1E2 | 3.1E1 | 1.0E-9 | 1.0E-9 | 4.1E3 | 1.4E2 | 583 | 25 | 209 | 25 | 0.54 |
| Ns | pg/ml | 1.0E-9 | 1.0E-9 | 1.1E1 | 9.9E0 | 6.1E1 | 4.8E1 | 1.0E-9 | 1.0E-9 | 1.1E3 | 2.4E2 | 583 | 25 | 209 | 25 | 0.51 |
| Nt | pg/ml | 1.0E2 | 1.0E2 | 1.4E2 | 1.3E2 | 1.1E2 | 7.7E1 | 1.0E-9 | 2.3E1 | 1.5E3 | 3.5E2 | 583 | 25 | 209 | 25 | 0.53 |
| Nu | pg/ml | 2.3E1 | 2.0E1 | 5.7E1 | 5.8E1 | 9.5E1 | 7.5E1 | 1.0E-9 | 1.0E-9 | 8.9E2 | 2.6E2 | 583 | 25 | 209 | 25 | 0.50 |
| Lu | pg/ml | 1.0E4 | 9.8E3 | 1.7E4 | 1.0E4 | 4.7E4 | 1.1E4 | 3.5E2 | 1.0E3 | 7.5E5 | 5.5E4 | 585 | 25 | 209 | 25 | 0.43 |
| Lv | pg/ml | 1.0E-9 | 1.0E-9 | 1.1E1 | 3.8E1 | 2.1E1 | 6.9E1 | 1.0E-9 | 1.0E-9 | 2.4E2 | 2.6E2 | 585 | 25 | 209 | 25 | 0.59 |
| Lw | pg/ml | 1.0E-9 | 1.0E-9 | 4.0E-1 | 4.1E-1 | 4.3E0 | 2.0E0 | 1.0E-9 | 1.0E-9 | 8.0E1 | 9.9E0 | 585 | 25 | 209 | 25 | 0.53 |
| Lx | pg/ml | 1.0E-9 | 6.0E1 | 1.5E2 | 2.1E2 | 4.5E2 | 3.3E2 | 1.0E-9 | 1.0E-9 | 6.2E3 | 1.4E3 | 585 | 25 | 209 | 25 | 0.63 |
| Ly | pg/ml | 1.0E-9 | 1.5E1 | 1.0E1 | 1.5E1 | 2.0E1 | 1.8E1 | 1.0E-9 | 1.0E-9 | 1.6E2 | 7.0E1 | 585 | 25 | 209 | 25 | 0.62 |
| Lz | pg/ml | 1.0E-9 | 1.0E-9 | 4.9E0 | 1.0E-9 | 3.6E1 | 0.0E0 | 1.0E-9 | 1.0E-9 | 6.0E2 | 1.0E-9 | 585 | 25 | 209 | 25 | 0.46 |
| Ma | pg/ml | 3.1E2 | 3.3E2 | 1.3E3 | 4.0E3 | 3.8E3 | 1.1E4 | 1.0E-9 | 1.0E-9 | 6.5E4 | 5.2E4 | 585 | 25 | 209 | 25 | 0.55 |
| Mb | pg/ml | 2.5E1 | 2.8E1 | 3.1E1 | 3.2E1 | 1.6E1 | 1.5E1 | 5.4E0 | 1.4E1 | 2.1E2 | 5.8E1 | 585 | 25 | 209 | 25 | 0.48 |
| Mc | pg/ml | 1.0E-9 | 1.0E-9 | 4.2E-2 | 1.0E-9 | 5.8E-1 | 0.0E0 | 1.0E-9 | 1.0E-9 | 1.3E1 | 1.0E-9 | 585 | 25 | 209 | 25 | 0.49 |
| Md | pg/ml | 1.0E-9 | 1.0E-9 | 4.8E-1 | 2.0E-2 | 3.3E0 | 9.8E-2 | 1.0E-9 | 1.0E-9 | 6.5E1 | 4.9E-1 | 585 | 25 | 209 | 25 | 0.47 |
| Me | pg/ml | 3.2E1 | 2.9E1 | 3.1E1 | 2.8E1 | 2.0E1 | 1.3E1 | 1.0E-9 | 2.4E-1 | 3.2E2 | 5.6E1 | 585 | 25 | 209 | 25 | 0.43 |
| Mf | pg/ml | 1.0E-9 | 1.0E-9 | 5.2E-1 | 3.2E-1 | 3.1E0 | 1.1E0 | 1.0E-9 | 1.0E-9 | 5.6E1 | 5.0E0 | 585 | 25 | 209 | 25 | 0.49 |
| Mg | pg/ml | 2.0E0 | 1.1E0 | 7.7E0 | 6.6E0 | 1.3E1 | 7.9E0 | 1.0E-9 | 1.0E-9 | 9.2E1 | 2.6E1 | 585 | 25 | 209 | 25 | 0.51 |
| Mh | pg/ml | 1.0E-9 | 1.0E-9 | 1.5E0 | 4.1E-1 | 1.1E1 | 1.6E0 | 1.0E-9 | 1.0E-9 | 1.6E2 | 7.8E0 | 585 | 25 | 209 | 25 | 0.51 |
| Mi | pg/ml | 1.0E-9 | 1.0E-9 | 5.9E-1 | 1.1E0 | 6.3E0 | 5.4E0 | 1.0E-9 | 1.0E-9 | 1.2E2 | 2.7E1 | 585 | 25 | 209 | 25 | 0.51 |
| Mj | pg/ml | 1.0E-9 | 1.0E-9 | 4.8E0 | 3.8E0 | 2.7E1 | 7.7E0 | 1.0E-9 | 1.0E-9 | 4.6E2 | 2.4E1 | 585 | 25 | 209 | 25 | 0.54 |
| Mk | pg/ml | 5.3E-1 | 2.0E0 | 1.8E1 | 5.8E0 | 1.1E2 | 9.6E0 | 1.0E-9 | 1.0E-9 | 1.7E3 | 4.0E1 | 585 | 25 | 209 | 25 | 0.50 |
| Ml | pg/ml | 1.0E-9 | 1.0E-9 | 6.3E0 | 3.7E-1 | 9.0E1 | 1.6E0 | 1.0E-9 | 1.0E-9 | 2.1E3 | 8.0E0 | 585 | 25 | 209 | 25 | 0.45 |
| Mm | pg/ml | 5.9E2 | 8.2E2 | 9.8E2 | 1.6E3 | 1.1E3 | 2.2E3 | 1.0E-9 | 8.8E-2 | 7.3E3 | 9.9E3 | 585 | 25 | 209 | 25 | 0.57 |
| Mn | pg/ml | 5.4E0 | 6.0E0 | 1.1E1 | 1.1E1 | 2.6E1 | 1.2E1 | 1.0E-9 | 1.0E-9 | 3.5E2 | 5.2E1 | 585 | 25 | 209 | 25 | 0.57 |
| Mp | pg/ml | 1.0E-9 | 1.0E-9 | 9.2E0 | 1.9E1 | 3.2E1 | 3.8E1 | 1.0E-9 | 1.0E-9 | 6.3E2 | 1.5E2 | 584 | 25 | 209 | 25 | 0.52 |
| Mq | pg/ml | 1.0E-9 | 1.0E-9 | 2.9E0 | 3.8E0 | 1.7E1 | 1.3E1 | 1.0E-9 | 1.0E-9 | 2.1E2 | 6.2E1 | 584 | 25 | 209 | 25 | 0.53 |
| Mr | pg/ml | 1.0E-9 | 1.0E-9 | 1.9E1 | 1.3E1 | 8.5E1 | 5.8E1 | 1.0E-9 | 1.0E-9 | 1.4E3 | 2.9E2 | 584 | 25 | 209 | 25 | 0.48 |
| Ms | pg/ml | 4.1E2 | 1.7E2 | 5.6E2 | 3.5E2 | 6.5E2 | 3.6E2 | 1.0E-9 | 1.0E-9 | 5.6E3 | 1.5E3 | 584 | 25 | 209 | 25 | 0.40 |
| Mt | pg/ml | 2.2E-1 | 1.2E0 | 7.4E0 | 5.3E0 | 5.0E1 | 1.4E1 | 1.0E-9 | 1.0E-9 | 8.7E2 | 7.0E1 | 584 | 25 | 209 | 25 | 0.64 |
| Mu | pg/ml | 1.0E-9 | 1.0E-9 | 1.6E0 | 1.2E0 | 1.3E1 | 3.7E0 | 1.0E-9 | 1.0E-9 | 2.3E2 | 1.8E1 | 584 | 25 | 209 | 25 | 0.55 |
| Mv | pg/ml | 1.0E-9 | 1.0E-9 | 7.6E1 | 8.5E1 | 3.6E2 | 2.0E2 | 1.0E-9 | 1.0E-9 | 4.5E3 | 6.5E2 | 584 | 25 | 209 | 25 | 0.55 |
| Mw | pg/ml | 3.4E1 | 5.9E1 | 4.8E2 | 3.0E2 | 3.1E3 | 6.1E2 | 1.0E-9 | 1.0E-9 | 6.2E4 | 2.7E3 | 584 | 25 | 209 | 25 | 0.53 |
| Mx | pg/ml | 1.0E-9 | 1.0E-9 | 2.5E-1 | 3.1E-1 | 1.5E0 | 8.1E-1 | 1.0E-9 | 1.0E-9 | 3.2E1 | 3.4E0 | 584 | 25 | 209 | 25 | 0.54 |
| My | pg/ml | 1.0E-9 | 1.0E-9 | 4.7E2 | 1.9E2 | 3.1E3 | 4.2E2 | 1.0E-9 | 1.0E-9 | 3.9E4 | 1.7E3 | 584 | 25 | 209 | 25 | 0.57 |
| Mz | pg/ml | 1.0E1 | 1.2E1 | 2.5E1 | 4.4E1 | 7.2E1 | 6.9E1 | 1.0E-9 | 1.0E-9 | 1.2E3 | 2.7E2 | 584 | 25 | 209 | 25 | 0.57 |
| Na | pg/ml | 1.0E-9 | 1.0E-9 | 7.4E-1 | 3.7E-1 | 3.0E0 | 1.4E0 | 1.0E-9 | 1.0E-9 | 3.6E1 | 7.2E0 | 584 | 25 | 209 | 25 | 0.47 |
| Nb | pg/ml | 1.9E0 | 2.6E0 | 4.2E0 | 3.3E0 | 1.4E1 | 2.6E0 | 1.0E-9 | 1.0E-9 | 2.3E2 | 1.0E1 | 584 | 25 | 209 | 25 | 0.58 |
| Nc | pg/ml | 4.0E2 | 1.1E2 | 6.3E2 | 2.8E2 | 7.9E2 | 4.8E2 | 1.0E-9 | 1.0E-9 | 9.2E3 | 2.1E3 | 584 | 25 | 209 | 25 | 0.33 |
| Nd | pg/ml | 2.9E1 | 6.8E0 | 2.7E1 | 1.5E1 | 5.4E1 | 1.7E1 | 1.0E-9 | 1.0E-9 | 1.2E3 | 5.2E1 | 584 | 25 | 209 | 25 | 0.36 |
| Ne | pg/ml | 4.7E2 | 2.6E2 | 6.1E2 | 3.0E2 | 6.0E2 | 3.3E2 | 1.0E-9 | 1.0E-9 | 7.0E3 | 1.5E3 | 584 | 25 | 209 | 25 | 0.33 |
| Nf | pg/ml | 1.0E-9 | 1.0E-9 | 3.0E0 | 5.1E-1 | 1.1E1 | 1.7E0 | 1.0E-9 | 1.0E-9 | 1.6E2 | 6.9E0 | 584 | 25 | 209 | 25 | 0.44 |

| Code | Units | Median | | Average | | Standard Deviation | | Minimum | | Maximum | | Number of Samples | | Number of Patients | | AUC |
|------|-------|--------|-----|---------|-----|-----|-----|---------|-----|---------|-----|--------|-----|---------|-----|-----|
| | | NonDis | Dis | NonDis | Dis | NonDis | Dis | NonDis | Dis | NonDis | Dis | NonDis | Dis | NonDis | Dis | |
| Ng | pg/ml | 3.6E1 | 6.3E0 | 1.4E2 | 9.1E1 | 2.6E2 | 1.8E2 | 1.0E-9 | 1.0E-9 | 2.3E3 | 6.4E2 | 584 | 25 | 209 | 25 | 0.43 |
| Nh | pg/ml | 7.1E1 | 3.2E1 | 9.5E1 | 5.5E1 | 8.8E1 | 5.8E1 | 1.0E-9 | 3.1E0 | 5.6E2 | 2.2E2 | 584 | 25 | 209 | 25 | 0.34 |
| Ni | pg/ml | 1.0E-9 | 1.0E-9 | 7.8E1 | 8.4E1 | 1.2E2 | 1.3E2 | 1.0E-9 | 1.0E-9 | 7.1E2 | 3.7E2 | 584 | 25 | 209 | 25 | 0.51 |
| Nj | pg/ml | 7.9E0 | 2.4E0 | 1.1E1 | 5.5E0 | 1.2E1 | 6.6E0 | 1.0E-9 | 1.0E-9 | 8.3E1 | 2.0E1 | 584 | 25 | 209 | 25 | 0.33 |
| Nk | pg/ml | 2.0E1 | 1.2E1 | 3.5E1 | 1.7E1 | 4.0E1 | 1.9E1 | 1.0E-9 | 1.0E-9 | 2.0E2 | 6.9E1 | 584 | 25 | 209 | 25 | 0.40 |
| Nl | pg/ml | 4.9E1 | 1.6E1 | 6.6E1 | 3.3E1 | 7.4E1 | 4.1E1 | 1.0E-9 | 1.0E-9 | 1.1E3 | 1.4E2 | 584 | 25 | 209 | 25 | 0.33 |
| Tz | pg/ml | 5.3E3 | 5.4E3 | 1.4E4 | 2.0E4 | 6.9E4 | 4.4E4 | 1.0E-9 | 7.5E2 | 1.0E6 | 1.7E5 | 219 | 15 | 133 | 15 | 0.51 |
| Ua | pg/ml | 3.9E3 | 3.7E3 | 1.6E4 | 9.4E3 | 2.9E4 | 1.5E4 | 1.0E-9 | 2.7E2 | 1.9E5 | 5.8E4 | 219 | 15 | 133 | 15 | 0.46 |
| Ub | pg/ml | 5.7E2 | 3.9E2 | 8.5E2 | 4.0E2 | 1.1E3 | 3.7E2 | 1.0E-9 | 1.5E1 | 9.8E3 | 1.4E3 | 219 | 15 | 133 | 15 | 0.35 |
| Ue | pg/ml | 2.9E1 | 2.1E1 | 3.6E1 | 2.4E1 | 3.2E1 | 1.7E1 | 9.8E-2 | 2.9E0 | 3.5E2 | 7.4E1 | 219 | 15 | 133 | 15 | 0.34 |
| Uc | pg/ml | 9.2E2 | 1.4E3 | 1.7E3 | 2.5E3 | 2.9E3 | 3.1E3 | 1.0E-9 | 2.1E1 | 2.9E4 | 9.4E3 | 219 | 15 | 133 | 15 | 0.56 |
| Ud | pg/ml | 1.0E-9 | 1.0E-9 | 1.9E0 | 1.0E-9 | 2.6E1 | 0.0E0 | 1.0E-9 | 1.0E-9 | 3.9E2 | 1.0E-9 | 219 | 15 | 133 | 15 | 0.49 |
| Hq | pg/ml | 1.1E0 | 1.8E0 | 1.5E2 | 2.1E1 | 2.0E3 | 6.9E1 | 1.0E-9 | 1.0E-9 | 3.4E4 | 3.4E2 | 581 | 25 | 208 | 25 | 0.54 |
| Hr | pg/ml | 1.3E2 | 7.3E1 | 8.0E2 | 6.4E2 | 1.6E3 | 1.0E3 | 1.0E-9 | 2.3E1 | 1.4E4 | 3.7E3 | 581 | 25 | 208 | 25 | 0.46 |
| Hu | pg/ml | 1.1E1 | 1.5E1 | 3.2E3 | 6.2E2 | 3.1E4 | 1.4E3 | 1.0E-9 | 1.0E-9 | 6.3E5 | 5.8E3 | 581 | 25 | 208 | 25 | 0.53 |
| Hv | pg/ml | 1.5E0 | 2.4E0 | 3.4E0 | 2.3E0 | 1.2E1 | 1.7E0 | 1.0E-9 | 1.0E-9 | 2.5E2 | 6.9E0 | 581 | 25 | 208 | 25 | 0.60 |
| Hw | pg/ml | 7.0E0 | 5.0E0 | 2.2E1 | 8.1E0 | 8.6E1 | 7.4E0 | 1.0E-9 | 1.0E-9 | 1.7E3 | 2.3E1 | 581 | 25 | 208 | 25 | 0.43 |
| Hx | pg/ml | 9.6E0 | 1.4E1 | 4.7E1 | 2.9E1 | 4.0E2 | 4.5E1 | 1.0E-9 | 1.0E-9 | 9.3E3 | 2.2E2 | 581 | 25 | 208 | 25 | 0.56 |
| Ib | ng/ml | 6.7E-2 | 1.5E-2 | 2.3E0 | 8.6E-2 | 7.8E0 | 1.8E-1 | 1.0E-9 | 1.0E-9 | 5.3E1 | 6.8E-1 | 210 | 15 | 132 | 15 | 0.33 |
| Ic | U/ml | 2.2E2 | 2.2E2 | 8.1E2 | 4.5E3 | 6.5E3 | 1.7E4 | 2.4E0 | 2.2E1 | 9.3E4 | 6.5E4 | 210 | 15 | 132 | 15 | 0.50 |
| Id | U/ml | 6.5E-1 | 9.5E-1 | 1.2E0 | 2.3E0 | 2.0E0 | 3.2E0 | 1.0E-9 | 2.7E-1 | 2.3E1 | 1.2E1 | 210 | 15 | 132 | 15 | 0.61 |
| Tt | pg/ml | 1.7E2 | 1.6E2 | 1.7E2 | 1.7E2 | 5.1E1 | 5.1E1 | 4.3E1 | 1.1E2 | 3.6E2 | 2.6E2 | 202 | 14 | 127 | 14 | 0.48 |
| To | pg/ml | 1.6E0 | 1.8E0 | 1.9E0 | 2.3E0 | 2.0E0 | 2.0E0 | 1.0E-9 | 1.0E-9 | 1.0E1 | 6.0E0 | 211 | 14 | 130 | 14 | 0.59 |
| Tr | pg/ml | 3.0E0 | 3.0E0 | 6.2E0 | 9.6E0 | 2.2E1 | 1.5E1 | 1.0E-9 | 3.3E-1 | 3.1E2 | 5.4E1 | 207 | 14 | 129 | 14 | 0.54 |
| Tn | pg/ml | 2.9E1 | 4.2E1 | 7.5E1 | 2.0E2 | 2.0E2 | 5.1E2 | 2.4E0 | 1.3E1 | 1.8E3 | 2.0E3 | 211 | 14 | 130 | 14 | 0.62 |
| Tv | ng/ml | 1.2E1 | 8.0E0 | 2.0E1 | 4.5E1 | 3.9E1 | 8.8E1 | 1.0E-9 | 1.0E-9 | 4.9E2 | 3.2E2 | 211 | 14 | 130 | 14 | 0.48 |
| Ih | ng/ml | 7.5E1 | 4.5E1 | 2.1E2 | 1.8E2 | 3.6E2 | 2.5E2 | 1.0E-9 | 1.0E-9 | 2.6E3 | 9.0E2 | 584 | 25 | 208 | 25 | 0.50 |
| Ii | ng/ml | 9.8E1 | 9.1E1 | 2.7E2 | 1.5E2 | 7.6E2 | 2.0E2 | 7.3E-1 | 9.1E0 | 1.0E4 | 8.1E2 | 584 | 25 | 208 | 25 | 0.48 |
| Ij | ng/ml | 7.6E1 | 8.7E1 | 1.9E2 | 1.0E2 | 6.3E2 | 7.1E1 | 2.1E0 | 1.0E-9 | 6.4E3 | 3.0E2 | 579 | 25 | 207 | 25 | 0.52 |
| Ik | ng/ml | 1.4E1 | 4.2E1 | 1.1E3 | 2.7E2 | 1.0E4 | 4.4E2 | 5.9E-1 | 2.1E0 | 1.2E5 | 1.5E3 | 581 | 25 | 207 | 25 | 0.55 |
| Il | ng/ml | 3.4E2 | 5.3E2 | 1.3E3 | 1.1E3 | 2.7E3 | 2.4E3 | 1.0E-9 | 1.0E-9 | 1.2E4 | 1.2E4 | 574 | 25 | 207 | 25 | 0.49 |
| Im | ng/ml | 2.0E2 | 2.4E2 | 3.5E2 | 3.8E2 | 5.2E2 | 3.1E2 | 1.3E1 | 2.5E1 | 6.0E3 | 1.1E3 | 581 | 25 | 207 | 25 | 0.58 |
| In | ng/ml | 3.9E0 | 1.6E0 | 2.5E1 | 6.4E0 | 1.8E2 | 1.7E1 | 1.0E-9 | 1.0E-9 | 3.9E3 | 8.4E1 | 584 | 25 | 208 | 25 | 0.37 |
| Hb | ng/ml | 2.4E1 | 2.0E1 | 3.2E1 | 2.7E1 | 2.9E1 | 2.5E1 | 4.8E-1 | 2.1E0 | 1.4E2 | 9.0E1 | 217 | 16 | 134 | 16 | 0.45 |
| Hc | pg/ml | 7.6E2 | 5.3E2 | 4.0E3 | 3.7E3 | 1.4E4 | 1.2E4 | 1.0E-9 | 2.2E2 | 1.0E5 | 5.0E4 | 217 | 16 | 134 | 16 | 0.43 |
| Hf | ng/ml | 1.5E2 | 1.1E2 | 4.1E2 | 2.4E2 | 5.8E2 | 2.9E2 | 1.0E-9 | 1.0E-9 | 3.2E3 | 8.2E2 | 217 | 16 | 134 | 16 | 0.46 |
| Io | ng/ml | 8.4E3 | 1.2E4 | 2.7E4 | 1.7E4 | 1.8E5 | 2.3E4 | 1.0E-9 | 1.3E3 | 4.0E6 | 1.1E5 | 578 | 23 | 208 | 23 | 0.56 |
| Ip | ng/ml | 1.0E1 | 2.5E1 | 1.9E1 | 3.0E1 | 2.4E1 | 3.7E1 | 1.0E-9 | 1.1E-2 | 2.6E2 | 1.6E2 | 578 | 23 | 208 | 23 | 0.57 |
| Iq | ug/ml | 9.8E-2 | 1.1E-1 | 2.4E1 | 2.4E-1 | 5.7E2 | 3.6E-1 | 1.0E-9 | 1.0E-9 | 1.4E4 | 1.4E0 | 578 | 23 | 208 | 23 | 0.50 |
| Ir | ug/ml | 3.4E-1 | 6.0E-1 | 2.7E0 | 1.5E0 | 1.7E1 | 3.2E0 | 1.0E-9 | 1.0E-9 | 2.4E2 | 1.6E1 | 577 | 23 | 208 | 23 | 0.62 |
| Is | ng/ml | 1.5E0 | 4.1E0 | 5.4E0 | 1.1E1 | 1.1E1 | 2.3E1 | 1.0E-9 | 2.7E-1 | 8.8E1 | 1.1E2 | 578 | 23 | 208 | 23 | 0.68 |
| It | ng/ml | 1.9E0 | 1.7E0 | 2.2E1 | 5.3E0 | 1.5E2 | 9.7E0 | 1.0E-9 | 1.0E-9 | 2.8E3 | 4.7E1 | 578 | 23 | 208 | 23 | 0.57 |
| Iu | ng/ml | 2.2E2 | 2.0E2 | 1.3E3 | 2.0E3 | 4.0E3 | 5.1E3 | 1.0E-9 | 1.0E-9 | 2.9E4 | 2.4E4 | 578 | 23 | 208 | 23 | 0.56 |
| Iv | ng/ml | 1.4E1 | 1.8E1 | 4.1E1 | 3.1E1 | 1.0E2 | 3.1E1 | 1.0E-9 | 1.0E-9 | 1.6E3 | 9.1E1 | 577 | 23 | 208 | 23 | 0.54 |
| Iz | ng/ml | 1.5E2 | 9.6E1 | 7.3E2 | 3.1E2 | 4.3E3 | 4.5E2 | 1.5E0 | 1.3E1 | 6.2E4 | 1.7E3 | 217 | 16 | 134 | 16 | 0.44 |
| Rc | pg/ml | 5.7E3 | 6.3E3 | 7.2E3 | 8.0E3 | 5.4E3 | 5.3E3 | 1.9E2 | 3.0E2 | 2.3E4 | 1.6E4 | 216 | 15 | 133 | 15 | 0.56 |
| Rb | pg/ml | 7.6E-1 | 8.1E-1 | 2.7E0 | 2.4E0 | 4.5E0 | 3.3E0 | 1.0E-9 | 1.0E-9 | 4.3E1 | 9.4E0 | 216 | 15 | 133 | 15 | 0.53 |
| Pz | ng/ml | 3.7E3 | 1.0E4 | 7.4E3 | 4.7E4 | 1.9E4 | 2.0E5 | 1.3E1 | 2.7E2 | 2.8E5 | 1.0E6 | 577 | 25 | 206 | 25 | 0.60 |
| Qa | ng/ml | 3.2E3 | 7.0E3 | 6.1E3 | 8.6E3 | 7.4E3 | 6.6E3 | 1.5E2 | 3.6E2 | 5.2E4 | 2.5E4 | 577 | 25 | 206 | 25 | 0.67 |
| Qb | ng/ml | 9.9E1 | 1.5E2 | 2.2E2 | 2.1E2 | 5.2E2 | 1.9E2 | 7.9E-1 | 2.4E0 | 8.3E3 | 7.2E2 | 577 | 25 | 206 | 25 | 0.58 |
| Qc | ng/ml | 2.5E2 | 2.1E2 | 4.7E2 | 3.2E2 | 8.0E2 | 3.3E2 | 1.0E-9 | 2.0E0 | 1.1E4 | 1.2E3 | 577 | 25 | 206 | 25 | 0.47 |
| Qd | ng/ml | 1.0E4 | 1.1E4 | 2.3E4 | 2.1E4 | 9.4E4 | 2.3E4 | 2.4E2 | 1.1E3 | 2.0E6 | 8.7E4 | 577 | 25 | 206 | 25 | 0.57 |
| Qc | ng/ml | 8.8E2 | 1.8E3 | 1.9E3 | 2.4E3 | 4.6E3 | 2.4E3 | 7.6E0 | 5.5E1 | 9.7E4 | 9.3E3 | 577 | 25 | 206 | 25 | 0.64 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Jd | ng/ml | 9.6E-1 | 1.1E0 | 7.2E0 | 2.2E0 | 4.7E1 | 2.7E0 | 1.0E-9 | 1.0E-9 | 6.5E2 | 8.3E0 | 217 | 15 | 135 | 15 | 0.53 |
| Jc | ng/ml | 1.0E-9 | 4.3E-1 | 2.5E0 | 9.8E-1 | 8.5E0 | 1.4E0 | 1.0E-9 | 1.0E-9 | 8.8E1 | 4.1E0 | 217 | 15 | 135 | 15 | 0.50 |
| Jf | ng/ml | 1.0E-9 | 1.0E-9 | 1.2E0 | 1.3E0 | 2.4E0 | 2.4E0 | 1.0E-9 | 1.0E-9 | 1.2E1 | 8.7E0 | 217 | 15 | 135 | 15 | 0.56 |
| Jg | ng/ml | 4.7E2 | 1.3E3 | 7.6E2 | 1.1E3 | 9.6E2 | 8.3E2 | 1.0E-9 | 2.1E1 | 1.0E4 | 3.0E3 | 581 | 25 | 208 | 25 | 0.64 |
| Jh | ng/ml | 3.1E0 | 3.9E0 | 2.6E1 | 1.6E1 | 1.1E2 | 2.3E1 | 1.0E-9 | 1.0E-9 | 1.6E3 | 9.1E1 | 581 | 25 | 208 | 25 | 0.55 |
| Ji | ng/ml | 5.0E1 | 8.5E1 | 7.1E1 | 1.2E2 | 7.0E1 | 9.7E1 | 1.0E-9 | 8.5E0 | 5.3E2 | 3.8E2 | 581 | 25 | 208 | 25 | 0.68 |
| Sr | pg/mL | 3.5E2 | 4.3E2 | 8.5E2 | 1.2E3 | 1.3E3 | 1.8E3 | 1.0E-9 | 1.0E-9 | 9.8E3 | 5.5E3 | 207 | 15 | 130 | 15 | 0.54 |
| Ss | pg/mL | 1.2E5 | 1.2E5 | 1.6E5 | 1.4E5 | 2.0E5 | 1.1E5 | 2.7E3 | 9.6E3 | 1.8E6 | 3.5E5 | 207 | 15 | 130 | 15 | 0.50 |
| St | pg/mL | 2.5E7 | 3.9E7 | 5.5E7 | 5.6E7 | 9.9E7 | 4.7E7 | 1.0E-9 | 2.1E6 | 1.2E9 | 1.3E8 | 212 | 14 | 131 | 14 | 0.58 |
| Ra | pg/ml | 1.0E-9 | 1.0E-9 | 6.4E-1 | 1.5E-1 | 1.3E0 | 2.5E-1 | 1.0E-9 | 1.0E-9 | 7.3E0 | 5.6E-1 | 216 | 15 | 133 | 15 | 0.46 |
| Qz | pg/ml | 1.0E1 | 1.0E-9 | 6.0E1 | 2.1E1 | 1.0E2 | 4.0E1 | 1.0E-9 | 1.0E-9 | 7.1E2 | 1.2E2 | 216 | 15 | 133 | 15 | 0.35 |
| Qy | pg/ml | 4.6E-1 | 5.4E-1 | 1.5E1 | 1.4E0 | 7.2E1 | 2.2E0 | 1.0E-9 | 1.0E-9 | 6.5E2 | 8.5E0 | 216 | 15 | 133 | 15 | 0.52 |
| Qx | pg/ml | 1.0E-9 | 1.0E-9 | 7.6E0 | 5.3E-2 | 5.5E1 | 2.0E-1 | 1.0E-9 | 1.0E-9 | 5.8E2 | 7.9E-1 | 216 | 15 | 133 | 15 | 0.46 |
| Qw | pg/ml | 4.5E-2 | 1.0E-9 | 2.2E0 | 1.5E0 | 8.8E0 | 5.8E0 | 1.0E-9 | 1.0E-9 | 8.6E1 | 2.3E1 | 216 | 15 | 133 | 15 | 0.33 |
| Qv | pg/ml | 2.2E4 | 8.6E3 | 3.5E4 | 7.7E4 | 6.4E4 | 2.4E5 | 1.0E-9 | 1.6E3 | 7.4E5 | 9.4E5 | 216 | 15 | 133 | 15 | 0.33 |
| Qu | pg/ml | 1.2E1 | 3.8E0 | 8.9E1 | 6.1E1 | 1.7E2 | 1.7E2 | 1.0E-9 | 1.0E-9 | 9.8E2 | 6.7E2 | 216 | 15 | 133 | 15 | 0.44 |
| Qt | pg/ml | 1.2E1 | 9.7E0 | 5.7E1 | 4.6E1 | 1.4E2 | 8.9E1 | 1.0E-9 | 1.0E-9 | 1.0E3 | 3.3E2 | 216 | 15 | 133 | 15 | 0.50 |
| Qh | ng/ml | 1.7E1 | 3.0E1 | 3.7E1 | 5.3E1 | 6.3E1 | 5.7E1 | 1.0E-9 | 5.4E0 | 6.4E2 | 2.0E2 | 216 | 15 | 133 | 15 | 0.63 |
| Qg | ng/ml | 7.9E0 | 4.6E0 | 2.0E1 | 1.2E1 | 7.5E1 | 1.8E1 | 5.1E-2 | 6.9E-1 | 1.0E3 | 7.4E1 | 216 | 15 | 133 | 15 | 0.43 |
| Jj | ng/ml | 7.5E2 | 7.9E2 | 2.2E3 | 1.1E3 | 1.5E4 | 1.5E3 | 1.7E1 | 1.3E1 | 3.4E5 | 6.7E3 | 581 | 25 | 208 | 25 | 0.43 |
| Jk | ng/ml | 3.3E0 | 4.2E0 | 2.4E1 | 2.3E1 | 4.9E1 | 3.4E1 | 1.0E-9 | 1.2E-1 | 3.9E2 | 1.2E2 | 581 | 25 | 208 | 25 | 0.54 |
| Jl | ng/ml | 4.4E-1 | 5.5E-1 | 1.8E0 | 6.6E0 | 4.3E0 | 9.5E0 | 7.6E-4 | 2.3E-3 | 3.2E1 | 2.6E1 | 581 | 25 | 208 | 25 | 0.63 |
| Jm | ng/ml | 1.9E1 | 1.6E1 | 5.2E1 | 5.0E1 | 9.7E1 | 7.0E1 | 1.0E-9 | 3.8E-1 | 1.0E3 | 3.2E2 | 581 | 25 | 208 | 25 | 0.52 |
| Jn | pg/ml | 4.0E-1 | 5.5E-1 | 1.7E0 | 9.7E-1 | 5.8E0 | 9.1E-1 | 1.0E-9 | 4.8E-2 | 6.2E1 | 3.2E0 | 581 | 25 | 208 | 25 | 0.61 |
| Jo | pg/ml | 4.0E3 | 4.7E3 | 5.1E3 | 4.6E3 | 3.9E3 | 3.4E3 | 4.2E1 | 2.7E2 | 2.4E4 | 1.3E4 | 581 | 25 | 208 | 25 | 0.47 |
| Jp | pg/ml | 7.0E4 | 7.8E4 | 7.3E4 | 8.6E4 | 3.4E4 | 3.5E4 | 2.1E3 | 8.9E3 | 2.1E5 | 1.9E5 | 581 | 25 | 208 | 25 | 0.61 |
| Jq | pg/ml | 9.5E1 | 1.4E2 | 1.5E2 | 2.0E2 | 2.2E2 | 2.0E2 | 2.6E0 | 2.6E1 | 4.0E3 | 7.5E2 | 581 | 25 | 208 | 25 | 0.59 |
| Jr | pg/ml | 5.2E0 | 7.7E0 | 2.2E1 | 1.2E1 | 1.0E2 | 1.4E1 | 1.0E-9 | 1.0E-9 | 1.9E3 | 4.8E1 | 581 | 25 | 208 | 25 | 0.58 |
| Js | pg/ml | 1.3E1 | 1.3E1 | 4.2E1 | 2.0E1 | 1.5E2 | 2.7E1 | 1.0E-9 | 4.5E-1 | 2.0E3 | 1.1E2 | 581 | 25 | 208 | 25 | 0.47 |
| Jt | pg/ml | 2.7E3 | 2.7E3 | 3.2E3 | 3.0E3 | 2.2E3 | 2.0E3 | 1.5E2 | 3.5E2 | 1.3E4 | 7.8E3 | 581 | 25 | 208 | 25 | 0.49 |
| Ju | mIU/ml | 9.0E0 | 1.3E1 | 2.1E1 | 1.7E1 | 3.3E1 | 2.5E1 | 6.5E-2 | 1.7E-1 | 2.3E2 | 1.0E2 | 217 | 15 | 135 | 15 | 0.51 |
| Jv | mIU/ml | 1.2E1 | 1.8E1 | 3.7E1 | 3.3E1 | 6.6E1 | 4.6E1 | 1.0E-2 | 1.4E-1 | 4.4E2 | 1.8E2 | 217 | 15 | 135 | 15 | 0.52 |
| Jy | ng/ml | 1.6E-3 | 1.6E-3 | 2.3E-3 | 2.9E-3 | 4.6E-3 | 5.5E-3 | 1.7E-4 | 4.5E-4 | 5.2E-2 | 2.3E-2 | 217 | 15 | 135 | 15 | 0.47 |
| Kc | pg/ml | 2.3E1 | 3.6E1 | 4.0E1 | 6.4E1 | 4.3E1 | 5.7E1 | 1.0E-9 | 6.1E0 | 1.9E2 | 1.6E2 | 218 | 16 | 134 | 16 | 0.60 |
| Kd | pg/ml | 1.0E-9 | 1.0E-9 | 2.0E2 | 2.1E2 | 6.0E2 | 5.5E2 | 1.0E-9 | 1.0E-9 | 4.8E3 | 1.8E3 | 218 | 16 | 134 | 16 | 0.53 |
| Ke | pg/ml | 1.3E4 | 1.3E4 | 1.4E4 | 1.7E4 | 1.1E4 | 1.2E4 | 3.4E2 | 1.3E3 | 7.0E4 | 4.4E4 | 218 | 16 | 134 | 16 | 0.55 |
| Kf | pg/mL | 6.7E0 | 8.8E0 | 7.3E0 | 9.9E0 | 5.9E0 | 4.9E0 | 1.0E-9 | 1.0E-9 | 2.7E1 | 1.6E1 | 218 | 16 | 134 | 16 | 0.65 |
| Kg | pg/mL | 1.2E3 | 6.9E2 | 2.0E3 | 1.6E3 | 2.4E3 | 1.5E3 | 7.3E1 | 2.1E2 | 1.7E4 | 4.6E3 | 218 | 16 | 134 | 16 | 0.44 |
| Ki | pg/ml | 5.9E1 | 5.3E1 | 7.0E1 | 5.2E1 | 5.5E1 | 2.4E1 | 1.0E-9 | 1.3E1 | 3.8E2 | 1.0E2 | 217 | 16 | 134 | 16 | 0.41 |
| Kj | pg/ml | 1.1E3 | 6.9E2 | 1.7E3 | 1.4E3 | 1.7E3 | 1.6E3 | 1.4E1 | 9.4E1 | 1.0E4 | 6.1E3 | 218 | 16 | 134 | 16 | 0.42 |
| Kk | pg/ml | 6.8E0 | 9.2E0 | 1.1E1 | 1.5E1 | 1.6E1 | 1.7E1 | 1.0E-9 | 1.0E-9 | 1.6E2 | 4.8E1 | 218 | 16 | 134 | 16 | 0.52 |
| Kl | pg/ml | 2.1E4 | 2.1E4 | 2.9E4 | 3.0E4 | 2.6E4 | 2.4E4 | 1.6E2 | 8.3E2 | 1.6E5 | 6.9E4 | 218 | 16 | 134 | 16 | 0.53 |
| Kn | pg/ml | 3.0E1 | 5.7E1 | 6.2E1 | 9.7E1 | 9.5E1 | 1.2E2 | 1.0E-9 | 1.0E-9 | 7.3E2 | 3.8E2 | 218 | 16 | 134 | 16 | 0.59 |
| Ko | pg/ml | 3.4E2 | 5.1E2 | 4.6E2 | 5.8E2 | 4.5E2 | 4.8E2 | 1.0E-9 | 1.0E-9 | 2.2E3 | 1.5E3 | 218 | 16 | 134 | 16 | 0.57 |
| Kp | pg/ml | 3.4E2 | 4.3E2 | 3.5E2 | 4.5E2 | 2.7E2 | 2.4E2 | 1.0E-9 | 1.0E-9 | 1.7E3 | 8.6E2 | 218 | 16 | 134 | 16 | 0.62 |
| Kq | pg/ml | 3.2E2 | 3.8E2 | 5.1E2 | 6.9E2 | 9.1E2 | 9.1E2 | 1.6E0 | 8.8E1 | 9.8E3 | 3.6E3 | 209 | 16 | 128 | 16 | 0.56 |
| Kr | pg/ml | 5.6E-1 | 6.4E-1 | 2.7E0 | 1.4E0 | 5.2E0 | 2.2E0 | 1.0E-9 | 1.0E-9 | 3.9E1 | 8.0E0 | 209 | 16 | 128 | 16 | 0.45 |
| Ks | pg/ml | 1.4E4 | 9.0E3 | 2.1E4 | 1.6E4 | 1.9E4 | 1.7E4 | 5.1E1 | 4.1E2 | 1.1E5 | 5.0E4 | 209 | 16 | 128 | 16 | 0.43 |
| Kx | ng/ml | 1.0E-9 | 1.7E-3 | 7.1E-3 | 6.3E-3 | 1.4E-2 | 1.1E-2 | 1.0E-9 | 1.0E-9 | 1.0E-1 | 4.1E-2 | 214 | 16 | 133 | 16 | 0.51 |
| Ky | ng/ml | 1.0E-1 | 1.8E-1 | 3.8E-1 | 7.9E-1 | 8.3E-1 | 1.6E0 | 1.0E-9 | 6.9E-2 | 5.4E0 | 6.4E0 | 214 | 16 | 133 | 16 | 0.67 |
| Kz | ng/ml | 1.0E-9 | 1.0E-9 | 3.4E-3 | 2.8E-3 | 5.7E-3 | 4.0E-3 | 1.0E-9 | 1.0E-9 | 3.6E-2 | 1.4E-2 | 214 | 16 | 133 | 16 | 0.52 |
| Ld | pg/ml | 1.0E-9 | 2.8E0 | 3.7E0 | 4.3E0 | 9.6E0 | 5.3E0 | 1.0E-9 | 1.0E-9 | 8.4E1 | 1.9E1 | 216 | 16 | 133 | 16 | 0.60 |
| Lh | pg/ml | 1.3E4 | 2.2E4 | 2.1E4 | 3.1E4 | 2.7E4 | 2.8E4 | 1.0E-9 | 5.3E2 | 2.6E5 | 1.1E5 | 580 | 25 | 209 | 25 | 0.64 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|------|--------|------|-----|
| | | NonDis | Dis | NonDis | Dis | NonDis | Dis | NonDis | Dis | NonDis | Dis | NonDis | Dis | NonDis | Dis | |
| Li | pg/ml | 3.1E3 | 4.9E3 | 1.5E4 | 1.1E4 | 3.9E4 | 1.6E4 | 1.0E-9 | 3.7E1 | 3.6E5 | 6.8E4 | 580 | 25 | 209 | 25 | 0.54 |
| Lj | pg/ml | 2.4E3 | 5.3E3 | 2.1E4 | 1.6E4 | 6.5E4 | 2.5E4 | 1.0E-9 | 3.4E1 | 4.7E5 | 9.4E4 | 580 | 25 | 209 | 25 | 0.56 |
| Rm | ng/ml | 1.9E1 | 2.8E1 | 5.1E1 | 6.8E1 | 7.7E1 | 8.1E1 | 2.2E-1 | 4.0E-1 | 4.0E2 | 2.5E2 | 213 | 15 | 132 | 15 | 0.56 |
| Rh | ng/ml | 1.3E2 | 6.0E1 | 2.8E2 | 2.0E2 | 5.0E2 | 2.4E2 | 4.7E0 | 1.8E1 | 3.8E3 | 6.9E2 | 213 | 15 | 132 | 15 | 0.42 |
| Ri | ng/ml | 1.0E-9 | 1.0E-9 | 4.3E0 | 4.1E0 | 1.7E1 | 1.1E1 | 1.0E-9 | 1.0E-9 | 2.2E2 | 4.1E1 | 214 | 15 | 133 | 15 | 0.48 |
| Rg | ng/ml | 1.0E-9 | 1.0E-9 | 6.8E-2 | 6.6E-3 | 3.9E-1 | 1.4E-2 | 1.0E-9 | 1.0E-9 | 3.3E0 | 4.8E-2 | 213 | 15 | 132 | 15 | 0.59 |
| Rj | ng/ml | 1.0E-9 | 1.0E-9 | 1.6E0 | 4.7E-2 | 6.3E0 | 1.8E-1 | 1.0E-9 | 1.0E-9 | 8.0E1 | 7.0E-1 | 214 | 15 | 133 | 15 | 0.39 |
| Rf | ng/ml | 3.7E-1 | 2.4E-1 | 1.0E0 | 4.5E-1 | 1.9E0 | 5.1E-1 | 7.8E-3 | 2.2E-2 | 1.5E1 | 2.1E0 | 213 | 15 | 132 | 15 | 0.41 |
| Ql | pg/ml | 5.5E0 | 4.3E-1 | 1.5E1 | 6.4E0 | 3.2E1 | 7.9E0 | 1.0E-9 | 1.0E-9 | 2.9E2 | 2.4E1 | 217 | 15 | 135 | 15 | 0.44 |
| Qm | pg/ml | 4.4E0 | 1.0E-9 | 2.0E1 | 2.1E1 | 4.0E1 | 3.4E1 | 1.0E-9 | 1.0E-9 | 3.6E2 | 1.1E2 | 217 | 15 | 135 | 15 | 0.48 |
| Qn | pg/ml | 6.1E-1 | 1.6E0 | 5.8E0 | 1.5E1 | 1.9E1 | 2.6E1 | 1.0E-9 | 1.0E-9 | 2.2E2 | 7.5E1 | 217 | 15 | 135 | 15 | 0.64 |
| Nv | pg/ml | 4.0E3 | 6.2E3 | 1.2E4 | 1.8E4 | 5.2E4 | 3.3E4 | 1.0E-9 | 9.8E1 | 1.1E6 | 1.6E5 | 585 | 25 | 209 | 25 | 0.60 |
| Nw | pg/ml | 8.5E3 | 1.6E4 | 1.3E4 | 1.6E4 | 1.8E4 | 1.1E4 | 8.6E1 | 7.5E2 | 2.1E5 | 5.0E4 | 585 | 25 | 209 | 25 | 0.66 |
| Nx | pg/ml | 2.2E2 | 3.5E2 | 4.1E2 | 6.8E2 | 6.9E2 | 6.7E2 | 1.0E-9 | 1.0E-9 | 7.4E3 | 2.1E3 | 585 | 25 | 209 | 25 | 0.66 |
| Ny | pg/ml | 5.7E0 | 1.0E1 | 7.2E1 | 7.1E1 | 1.0E3 | 2.4E2 | 1.0E-9 | 1.0E-9 | 2.5E4 | 1.2E3 | 585 | 25 | 209 | 25 | 0.61 |
| Oa | pg/ml | 1.6E2 | 5.4E2 | 4.1E2 | 7.2E2 | 7.0E2 | 7.8E2 | 1.0E-9 | 1.0E-9 | 4.8E3 | 2.9E3 | 217 | 15 | 135 | 15 | 0.68 |
| Oe | pg/ml | 9.1E1 | 7.1E0 | 3.1E2 | 3.0E2 | 8.7E2 | 4.6E2 | 1.0E-9 | 1.0E-9 | 1.9E4 | 1.6E3 | 580 | 25 | 209 | 25 | 0.47 |
| Of | pg/ml | 2.3E2 | 1.2E2 | 7.1E3 | 9.8E3 | 3.3E4 | 2.6E4 | 1.0E-9 | 1.0E-9 | 6.2E5 | 1.2E5 | 585 | 25 | 209 | 25 | 0.45 |
| Og | pg/ml | 9.2E-2 | 9.3E-2 | 9.9E-1 | 2.8E-1 | 5.8E0 | 8.0E-1 | 1.0E-9 | 1.0E-9 | 8.3E1 | 4.0E0 | 585 | 25 | 209 | 25 | 0.42 |
| Oh | pg/ml | 2.4E0 | 5.3E0 | 2.5E1 | 8.9E0 | 1.7E2 | 1.2E1 | 1.0E-9 | 1.0E-9 | 3.5E3 | 5.6E1 | 585 | 25 | 209 | 25 | 0.61 |
| Oi | pg/ml | 2.8E0 | 2.5E0 | 6.5E0 | 5.0E0 | 1.0E1 | 7.5E0 | 1.0E-9 | 1.0E-9 | 8.6E1 | 3.6E1 | 585 | 25 | 209 | 25 | 0.48 |
| Ok | pg/ml | 3.9E2 | 6.0E2 | 5.2E2 | 7.4E2 | 5.0E2 | 5.3E2 | 1.3E1 | 4.2E1 | 5.2E3 | 2.0E3 | 585 | 25 | 209 | 25 | 0.64 |
| Om | pg/ml | 3.8E2 | 5.5E2 | 9.0E2 | 7.2E2 | 2.5E3 | 6.6E2 | 1.0E-9 | 1.0E-9 | 3.6E4 | 2.5E3 | 585 | 25 | 209 | 25 | 0.55 |
| On | pg/ml | 1.8E2 | 3.1E2 | 2.9E2 | 4.6E2 | 4.3E2 | 4.6E2 | 8.4E-1 | 1.2E1 | 4.5E3 | 1.7E3 | 585 | 25 | 209 | 25 | 0.63 |
| Or | pg/ml | 1.4E1 | 5.8E1 | 3.4E1 | 8.3E1 | 6.3E1 | 9.5E1 | 1.0E-9 | 1.0E-9 | 5.0E2 | 3.4E2 | 218 | 16 | 134 | 16 | 0.72 |
| Ow | pg/ml | 3.3E1 | 1.0E2 | 1.1E2 | 7.7E2 | 3.1E2 | 2.0E3 | 1.0E-9 | 2.5E1 | 2.7E3 | 8.1E3 | 218 | 16 | 134 | 16 | 0.80 |
| Ou | pg/ml | 4.7E2 | 1.3E3 | 8.8E2 | 2.3E3 | 1.3E3 | 2.8E3 | 1.0E-9 | 1.4E2 | 9.8E3 | 1.1E4 | 218 | 16 | 134 | 16 | 0.72 |
| Ul | ng/ml | 1.0E-9 | 1.0E-9 | 1.4E0 | 1.4E0 | 4.7E0 | 3.6E0 | 1.0E-9 | 1.0E-9 | 3.4E1 | 1.0E1 | 224 | 15 | 137 | 15 | 0.51 |
| Uo | ng/ml | 1.0E-9 | 1.0E-9 | 1.1E-1 | 5.8E-2 | 2.7E-1 | 1.1E-1 | 1.0E-9 | 1.0E-9 | 2.6E0 | 3.2E-1 | 224 | 15 | 137 | 15 | 0.50 |
| Uk | ng/ml | 1.0E-9 | 1.0E-9 | 8.7E-3 | 4.8E-3 | 2.9E-2 | 1.8E-2 | 1.0E-9 | 1.0E-9 | 3.2E-1 | 7.1E-2 | 224 | 15 | 137 | 15 | 0.37 |
| Um | ng/ml | 1.0E-9 | 1.0E-9 | 4.2E-1 | 7.0E-1 | 1.0E0 | 1.1E0 | 1.0E-9 | 1.0E-9 | 7.2E0 | 2.7E0 | 224 | 15 | 137 | 15 | 0.52 |
| Uf | ng/ml | 5.3E-2 | 1.0E-1 | 1.5E-1 | 6.7E-1 | 2.7E-1 | 2.2E0 | 1.0E-3 | 3.5E-3 | 2.1E0 | 8.6E0 | 224 | 15 | 137 | 15 | 0.60 |
| Uh | ng/ml | 1.9E0 | 3.1E0 | 2.9E0 | 5.0E0 | 3.1E0 | 5.4E0 | 3.2E-2 | 1.3E-2 | 1.7E1 | 2.1E1 | 224 | 15 | 137 | 15 | 0.62 |
| Un | ng/ml | 1.9E0 | 2.3E0 | 2.1E0 | 2.2E0 | 1.3E0 | 1.3E0 | 2.0E-1 | 1.3E-1 | 8.0E0 | 4.7E0 | 224 | 15 | 137 | 15 | 0.53 |
| Ug | ng/ml | 1.4E1 | 6.8E0 | 2.8E1 | 2.1E1 | 2.9E1 | 2.9E1 | 6.9E-1 | 2.2E0 | 1.8E2 | 1.1E2 | 224 | 15 | 137 | 15 | 0.36 |
| Ur | ng/ml | 1.5E-1 | 9.6E-2 | 8.5E-1 | 5.1E-1 | 6.3E0 | 1.2E0 | 1.0E-9 | 1.0E-9 | 9.3E1 | 4.7E0 | 223 | 15 | 136 | 15 | 0.41 |
| Up | ng/ml | 1.0E-9 | 1.0E-9 | 6.0E-3 | 9.0E-4 | 2.6E-2 | 1.5E-3 | 1.0E-9 | 1.0E-9 | 3.3E-1 | 4.9E-3 | 223 | 15 | 136 | 15 | 0.50 |
| Us | ng/ml | 4.3E-3 | 1.0E-9 | 1.9E-2 | 1.2E-2 | 4.6E-2 | 2.1E-2 | 1.0E-9 | 1.0E-9 | 4.0E-1 | 6.3E-2 | 223 | 15 | 136 | 15 | 0.43 |
| Uv | ng/ml | 3.2E-3 | 1.8E-3 | 1.3E-2 | 8.1E-3 | 3.9E-2 | 1.4E-2 | 1.0E-9 | 1.0E-9 | 4.3E-1 | 4.4E-2 | 223 | 15 | 136 | 15 | 0.43 |
| Ut | ng/ml | 7.6E-1 | 6.1E-1 | 3.2E0 | 2.9E0 | 9.7E0 | 6.2E0 | 1.0E-9 | 1.0E-9 | 7.8E1 | 2.4E1 | 223 | 15 | 136 | 15 | 0.44 |
| Uu | ng/ml | 7.2E0 | 8.6E0 | 7.8E0 | 9.0E0 | 4.8E0 | 5.3E0 | 5.7E-1 | 1.3E0 | 2.6E1 | 2.1E1 | 223 | 15 | 136 | 15 | 0.57 |
| Vp | ng/ml | 1.0E-9 | 1.0E-9 | 4.6E-1 | 8.2E-2 | 3.8E0 | 1.7E-1 | 1.0E-9 | 1.0E-9 | 5.0E1 | 6.0E-1 | 224 | 15 | 137 | 15 | 0.55 |
| Vt | ng/ml | 6.1E0 | 8.6E0 | 8.3E0 | 1.1E1 | 9.3E0 | 6.7E0 | 4.3E-1 | 1.9E0 | 8.6E1 | 2.2E1 | 224 | 15 | 137 | 15 | 0.64 |
| Vu | ng/ml | 1.0E-9 | 5.6E-1 | 2.9E0 | 1.9E0 | 7.5E0 | 3.1E0 | 1.0E-9 | 1.4E-1 | 7.1E1 | 1.1E1 | 219 | 14 | 137 | 14 | 0.55 |
| Vq | ng/ml | 1.5E2 | 5.6E2 | 6.3E2 | 8.4E2 | 1.4E3 | 7.8E2 | 2.0E-1 | 1.7E1 | 1.1E4 | 2.3E3 | 175 | 11 | 115 | 11 | 0.67 |
| Vo | ng/ml | 2.6E1 | 2.5E1 | 2.5E1 | 2.4E1 | 5.1E0 | 5.6E0 | 2.5E0 | 8.0E0 | 4.8E1 | 3.1E1 | 224 | 15 | 137 | 15 | 0.45 |
| Vs | ng/ml | 2.6E-1 | 1.0E-9 | 7.3E0 | 1.0E1 | 2.6E1 | 2.4E1 | 1.0E-9 | 1.0E-9 | 2.3E2 | 9.4E1 | 218 | 15 | 135 | 15 | 0.54 |
| Vv | ng/ml | 3.3E0 | 3.5E0 | 6.3E0 | 4.7E0 | 1.0E1 | 6.8E0 | 1.0E-9 | 1.4E-1 | 8.4E1 | 2.6E1 | 223 | 15 | 137 | 15 | 0.47 |
| Oy | pg/ml | 5.5E-1 | 5.0E-1 | 7.3E0 | 2.4E0 | 3.5E1 | 4.2E0 | 1.0E-9 | 1.0E-9 | 4.0E2 | 1.5E1 | 584 | 25 | 208 | 25 | 0.52 |
| Oz | pg/ml | 1.3E-2 | 5.8E-2 | 3.5E-1 | 2.6E-1 | 1.6E0 | 4.1E-1 | 1.0E-9 | 1.0E-9 | 2.9E1 | 1.7E0 | 584 | 25 | 208 | 25 | 0.52 |
| Pa | pg/ml | 3.9E-1 | 5.0E-1 | 1.5E0 | 1.0E0 | 5.8E0 | 1.1E0 | 1.0E-9 | 1.0E-9 | 8.6E1 | 4.4E0 | 584 | 25 | 208 | 25 | 0.61 |
| Pb | pg/ml | 1.0E-9 | 1.0E-9 | 1.1E0 | 1.2E0 | 2.0E1 | 5.6E0 | 1.0E-9 | 1.0E-9 | 4.9E2 | 2.8E1 | 584 | 25 | 208 | 25 | 0.51 |
| Pc | pg/ml | 4.9E-2 | 2.7E-1 | 3.8E-1 | 4.1E-1 | 1.0E0 | 4.9E-1 | 1.0E-9 | 1.0E-9 | 1.6E1 | 1.8E0 | 584 | 25 | 208 | 25 | 0.57 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pd | pg/ml | 1.8E0 | 2.8E0 | 5.5E0 | 3.6E0 | 3.6E1 | 3.5E0 | 1.0E-9 | 1.0E-9 | 8.4E2 | 1.4E1 | 584 | 25 | 208 | 25 | 0.57 |
| Pc | pg/ml | 2.1E1 | 4.4E1 | 1.1E2 | 6.1E1 | 3.7E2 | 6.0E1 | 1.0E-9 | 1.0E-9 | 4.7E3 | 2.5E2 | 584 | 25 | 208 | 25 | 0.62 |
| Pf | pg/ml | 1.5E0 | 2.7E0 | 1.1E1 | 1.0E1 | 6.6E1 | 1.8E1 | 1.0E-9 | 1.0E-9 | 1.5E3 | 7.7E1 | 584 | 25 | 208 | 25 | 0.61 |
| Pg | pg/ml | 3.3E0 | 1.0E1 | 5.4E1 | 3.7E1 | 4.2E2 | 5.7E1 | 1.0E-9 | 1.0E-9 | 7.7E3 | 1.9E2 | 584 | 25 | 208 | 25 | 0.66 |
| Ph | ng/ml | 1.6E-1 | 2.2E-1 | 3.3E-1 | 3.8E-1 | 4.9E-1 | 5.5E-1 | 1.0E-9 | 1.0E-9 | 3.1E0 | 2.3E0 | 218 | 16 | 134 | 16 | 0.51 |
| Pi | ng/ml | 1.9E-1 | 2.5E-1 | 2.8E-1 | 3.2E-1 | 3.7E-1 | 2.6E-1 | 1.0E-9 | 1.0E-9 | 3.2E0 | 8.6E-1 | 218 | 16 | 134 | 16 | 0.59 |
| Pj | ng/mL | 5.2E0 | 5.4E0 | 6.1E0 | 5.8E0 | 4.7E0 | 3.6E0 | 3.8E-2 | 8.8E-1 | 3.1E1 | 1.3E1 | 218 | 16 | 134 | 16 | 0.51 |
| Pk | ng/ml | 8.9E-3 | 6.5E-3 | 1.4E-2 | 1.0E-2 | 2.2E-2 | 1.3E-2 | 1.0E-9 | 1.0E-9 | 2.5E-1 | 4.3E-2 | 218 | 16 | 134 | 16 | 0.42 |
| aA | mg/dL | 8.0E-1 | 9.0E-1 | 9.1E-1 | 1.1E0 | 4.4E-1 | 7.7E-1 | 2.0E-1 | 4.0E-1 | 4.2E0 | 4.1E0 | 1815 | 29 | 323 | 29 | 0.56 |
| aC | mg/mL | 2.8E0 | 1.8E0 | 3.1E0 | 2.2E0 | 1.3E0 | 1.1E0 | 8.5E-1 | 1.1E0 | 8.2E0 | 4.8E0 | 363 | 17 | 141 | 17 | 0.29 |
| aD | ug/mL | 3.1E0 | 5.8E0 | 4.3E0 | 5.5E0 | 3.5E0 | 4.2E0 | 8.5E-1 | 9.9E-1 | 3.1E1 | 1.7E1 | 363 | 17 | 141 | 17 | 0.56 |
| aE | mg/mL | 5.7E-1 | 5.7E-1 | 5.8E-1 | 5.7E-1 | 1.5E-1 | 1.7E-1 | 2.1E-1 | 2.5E-1 | 1.1E0 | 8.6E-1 | 363 | 17 | 141 | 17 | 0.47 |
| aF | ng/mL | 2.1E0 | 2.4E0 | 4.1E0 | 5.9E0 | 6.1E0 | 7.8E0 | 4.3E-3 | 4.3E-3 | 5.0E1 | 2.9E1 | 363 | 17 | 141 | 17 | 0.55 |
| aG | mg/mL | 1.4E-1 | 1.1E-1 | 1.6E-1 | 1.4E-1 | 8.4E-2 | 8.4E-2 | 5.0E-2 | 5.6E-2 | 5.0E-1 | 3.8E-1 | 363 | 17 | 141 | 17 | 0.39 |
| aH | ug/mL | 7.5E1 | 7.6E1 | 8.1E1 | 7.7E1 | 4.2E1 | 4.2E1 | 9.6E0 | 2.3E1 | 2.9E2 | 1.4E2 | 363 | 17 | 141 | 17 | 0.48 |
| aI | ug/mL | 1.9E2 | 1.5E2 | 1.9E2 | 1.7E2 | 6.0E1 | 7.3E1 | 4.7E1 | 7.5E1 | 3.7E2 | 2.8E2 | 363 | 17 | 141 | 17 | 0.41 |
| aJ | ug/mL | 2.4E0 | 3.7E0 | 3.0E0 | 4.3E0 | 2.2E0 | 2.9E0 | 9.0E-1 | 9.5E-1 | 1.7E1 | 1.1E1 | 363 | 17 | 141 | 17 | 0.66 |
| aK | ng/mL | 1.6E0 | 1.0E0 | 2.5E0 | 2.4E0 | 2.7E0 | 2.9E0 | 2.9E-4 | 9.1E-2 | 1.8E1 | 1.1E1 | 363 | 17 | 141 | 17 | 0.46 |
| aL | mg/mL | 7.9E-1 | 8.3E-1 | 8.0E-1 | 8.2E-1 | 2.4E-1 | 3.3E-1 | 2.2E-1 | 2.9E-1 | 1.7E0 | 1.6E0 | 363 | 17 | 141 | 17 | 0.49 |
| aM | U/mL | 2.2E1 | 2.4E1 | 4.4E1 | 4.8E1 | 1.0E2 | 4.4E1 | 4.2E-2 | 4.2E-2 | 1.6E3 | 1.3E2 | 363 | 17 | 141 | 17 | 0.57 |
| aN | U/mL | 1.4E1 | 2.1E1 | 2.1E1 | 3.1E1 | 3.0E1 | 2.6E1 | 2.5E-3 | 2.5E-3 | 3.8E2 | 9.5E1 | 363 | 17 | 141 | 17 | 0.65 |
| aO | pg/mL | 3.5E1 | 3.3E1 | 3.4E2 | 3.3E2 | 8.8E2 | 5.9E2 | 6.0E-2 | 6.0E-2 | 6.6E3 | 2.1E3 | 363 | 17 | 141 | 17 | 0.52 |
| aP | ng/mL | 1.6E0 | 1.8E0 | 2.0E0 | 2.3E0 | 1.2E0 | 1.5E0 | 5.4E-1 | 7.0E-1 | 7.0E0 | 6.6E0 | 363 | 17 | 141 | 17 | 0.58 |
| aQ | ng/mL | 3.0E-1 | 2.8E-1 | 4.6E-1 | 4.0E-1 | 4.9E-1 | 4.5E-1 | 2.0E-4 | 6.7E-2 | 4.0E0 | 1.8E0 | 363 | 17 | 141 | 17 | 0.44 |
| aR | ng/mL | 1.8E0 | 1.9E0 | 2.9E0 | 2.3E0 | 3.5E0 | 1.8E0 | 1.8E-1 | 3.2E-1 | 3.4E1 | 7.9E0 | 363 | 17 | 141 | 17 | 0.48 |
| aS | ng/mL | 2.7E-1 | 2.9E-1 | 7.2E-1 | 4.8E-1 | 2.0E0 | 4.7E-1 | 4.2E-3 | 7.2E-2 | 3.3E1 | 1.7E0 | 363 | 17 | 141 | 17 | 0.52 |
| aU | pg/mL | 7.8E1 | 5.3E1 | 1.3E2 | 1.3E2 | 1.5E2 | 1.6E2 | 7.4E-2 | 6.5E0 | 1.3E3 | 6.0E2 | 363 | 17 | 141 | 17 | 0.46 |
| aV | ng/mL | 6.3E-1 | 3.7E-1 | 1.1E0 | 1.1E0 | 2.1E0 | 1.6E0 | 7.6E-4 | 3.3E-2 | 3.3E1 | 6.0E0 | 363 | 17 | 141 | 17 | 0.42 |
| aW | pg/mL | 1.9E1 | 1.8E1 | 2.0E1 | 1.8E1 | 2.1E1 | 8.0E0 | 7.2E-2 | 7.2E-2 | 2.4E2 | 3.1E1 | 363 | 17 | 141 | 17 | 0.49 |
| aX | ng/mL | 9.5E0 | 1.6E1 | 1.4E1 | 2.4E1 | 1.4E1 | 3.5E1 | 3.0E-1 | 1.4E0 | 8.0E1 | 1.4E2 | 363 | 17 | 141 | 17 | 0.56 |
| aY | pg/mL | 6.0E1 | 6.6E1 | 8.0E1 | 9.4E1 | 9.2E1 | 6.9E1 | 4.1E-1 | 1.7E1 | 1.2E3 | 2.4E2 | 363 | 17 | 141 | 17 | 0.59 |
| aZ | pg/mL | 2.2E2 | 1.7E2 | 5.2E2 | 4.7E2 | 1.0E3 | 1.0E3 | 1.7E0 | 1.7E0 | 1.2E4 | 4.3E3 | 363 | 17 | 141 | 17 | 0.45 |
| bA | ng/mL | 8.5E0 | 2.7E1 | 3.0E1 | 1.1E2 | 7.7E1 | 2.0E2 | 3.0E-2 | 1.4E0 | 9.4E2 | 8.1E2 | 363 | 17 | 141 | 17 | 0.68 |
| bB | ng/mL | 3.0E2 | 1.9E2 | 3.2E2 | 2.5E2 | 1.6E2 | 1.7E2 | 1.6E1 | 1.2E1 | 1.0E3 | 6.2E2 | 363 | 17 | 141 | 17 | 0.36 |
| bC | ng/mL | 3.3E2 | 4.7E2 | 5.7E2 | 1.1E3 | 7.3E2 | 1.3E3 | 2.7E1 | 1.5E2 | 4.7E3 | 4.7E3 | 363 | 17 | 141 | 17 | 0.66 |
| bE | mg/mL | 5.4E0 | 4.9E0 | 5.7E0 | 5.8E0 | 1.9E0 | 2.8E0 | 1.4E0 | 1.9E0 | 1.3E1 | 1.1E1 | 363 | 17 | 141 | 17 | 0.46 |
| bF | pg/mL | 2.0E1 | 3.3E1 | 1.8E2 | 6.0E2 | 1.1E3 | 1.5E3 | 5.0E-2 | 8.5E0 | 1.1E4 | 6.3E3 | 363 | 17 | 141 | 17 | 0.66 |
| bG | ng/mL | 1.6E0 | 2.2E0 | 2.8E0 | 4.6E0 | 3.4E0 | 7.2E0 | 2.2E-2 | 1.6E-1 | 2.6E1 | 3.0E1 | 363 | 17 | 141 | 17 | 0.54 |
| bH | pg/mL | 5.7E-1 | 5.7E-1 | 5.2E0 | 2.7E0 | 1.7E1 | 3.6E0 | 5.7E-1 | 5.7E-1 | 2.8E2 | 1.1E1 | 363 | 17 | 141 | 17 | 0.46 |
| bI | ng/mL | 4.0E-3 | 4.0E-3 | 7.0E-2 | 2.2E-2 | 1.7E-1 | 7.4E-2 | 4.0E-3 | 4.0E-3 | 1.3E0 | 3.1E-1 | 363 | 17 | 141 | 17 | 0.43 |
| bJ | mg/mL | 2.2E0 | 1.1E0 | 2.5E0 | 2.1E0 | 1.9E0 | 2.3E0 | 2.5E-4 | 2.1E-2 | 1.3E1 | 9.0E0 | 363 | 17 | 141 | 17 | 0.41 |
| bL | pg/mL | 4.1E0 | 5.8E0 | 8.4E0 | 8.4E0 | 1.0E1 | 8.1E0 | 4.6E-2 | 4.6E-2 | 4.9E1 | 2.5E1 | 363 | 17 | 141 | 17 | 0.53 |
| bM | mg/mL | 1.7E0 | 2.4E0 | 2.1E0 | 2.7E0 | 1.4E0 | 1.9E0 | 9.2E-3 | 7.1E-1 | 7.9E0 | 8.4E0 | 363 | 17 | 141 | 17 | 0.61 |
| bN | ng/mL | 4.7E1 | 3.9E1 | 1.3E2 | 8.3E1 | 2.7E2 | 1.4E2 | 1.4E-1 | 1.8E0 | 1.9E3 | 5.7E2 | 363 | 17 | 141 | 17 | 0.44 |
| bO | ng/mL | 4.0E-2 | 4.0E-2 | 9.2E0 | 8.3E0 | 2.1E1 | 1.4E1 | 4.0E-2 | 4.0E-2 | 1.9E2 | 3.8E1 | 363 | 17 | 141 | 17 | 0.51 |
| bP | mg/mL | 5.1E-1 | 6.8E-1 | 7.3E-1 | 8.2E-1 | 6.6E-1 | 7.3E-1 | 8.2E-2 | 2.3E-1 | 4.8E0 | 3.1E0 | 363 | 17 | 141 | 17 | 0.55 |
| bQ | pg/mL | 1.5E1 | 2.0E1 | 7.0E1 | 4.5E1 | 7.1E2 | 5.2E1 | 1.5E-1 | 6.4E0 | 1.3E4 | 1.8E2 | 363 | 17 | 141 | 17 | 0.59 |
| bR | ng/mL | 1.2E-2 | 1.2E-2 | 1.4E-1 | 7.6E-2 | 5.0E-1 | 1.1E-1 | 1.2E-2 | 1.2E-2 | 8.7E0 | 3.2E-1 | 363 | 17 | 141 | 17 | 0.45 |
| bS | pg/mL | 9.4E-1 | 9.4E-1 | 7.8E0 | 3.3E0 | 3.1E1 | 9.9E0 | 9.4E-1 | 9.4E-1 | 3.9E2 | 4.2E1 | 363 | 17 | 141 | 17 | 0.46 |
| bU | ng/mL | 1.3E-1 | 6.8E-2 | 2.1E-1 | 1.5E-1 | 4.1E-1 | 1.7E-1 | 1.3E-2 | 1.3E-2 | 6.6E0 | 4.9E-1 | 363 | 17 | 141 | 17 | 0.46 |
| bV | pg/mL | 4.7E2 | 5.7E2 | 5.3E2 | 1.6E3 | 2.4E2 | 3.9E3 | 1.7E2 | 2.6E2 | 1.6E3 | 1.7E4 | 363 | 17 | 141 | 17 | 0.58 |
| bW | pg/mL | 3.4E2 | 4.0E2 | 4.9E2 | 1.7E3 | 5.0E2 | 4.7E3 | 8.4E1 | 1.3E2 | 4.8E3 | 2.0E4 | 363 | 17 | 141 | 17 | 0.56 |
| bX | ng/mL | 1.5E-3 | 2.5E-5 | 2.8E-3 | 1.6E-3 | 3.4E-3 | 3.2E-3 | 2.5E-5 | 2.5E-5 | 2.0E-2 | 1.2E-2 | 363 | 17 | 141 | 17 | 0.38 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bZ | pg/mL | 2.4E2 | 3.4E2 | 9.1E2 | 3.7E3 | 4.3E3 | 1.0E4 | 1.5E-1 | 7.4E1 | 5.8E4 | 4.3E4 | 363 | 17 | 141 | 17 | 0.61 |
| cA | pg/mL | 6.0E-1 | 6.0E-1 | 3.0E0 | 1.9E0 | 2.0E1 | 3.7E0 | 6.0E-1 | 6.0E-1 | 3.7E2 | 1.3E1 | 363 | 17 | 141 | 17 | 0.50 |
| cB | ng/mL | 5.4E-2 | 3.6E-2 | 8.6E-2 | 8.2E-2 | 1.0E-1 | 1.3E-1 | 1.7E-3 | 1.7E-3 | 5.7E-1 | 5.5E-1 | 363 | 17 | 141 | 17 | 0.45 |
| cC | pg/mL | 4.6E1 | 4.8E1 | 4.9E1 | 4.6E1 | 4.2E1 | 2.6E1 | 1.0E0 | 1.0E0 | 4.5E2 | 9.6E1 | 363 | 17 | 141 | 17 | 0.50 |
| cD | pg/mL | 5.2E0 | 3.8E0 | 1.3E1 | 8.6E0 | 3.9E1 | 1.2E1 | 3.3E-1 | 3.3E-1 | 4.8E2 | 4.1E1 | 363 | 17 | 141 | 17 | 0.43 |
| cE | pg/mL | 3.2E1 | 7.4E1 | 1.5E2 | 5.9E2 | 4.8E2 | 1.0E3 | 1.2E-1 | 1.2E-1 | 3.8E3 | 3.8E3 | 363 | 17 | 141 | 17 | 0.68 |
| cF | pg/mL | 1.2E1 | 9.4E0 | 2.1E1 | 1.2E1 | 3.4E1 | 1.3E1 | 5.3E-1 | 5.3E-1 | 2.7E2 | 4.1E1 | 363 | 17 | 141 | 17 | 0.45 |
| cG | pg/mL | 4.3E1 | 8.6E1 | 1.1E2 | 1.5E2 | 5.7E2 | 1.5E2 | 7.8E0 | 1.0E1 | 1.0E4 | 5.6E2 | 363 | 17 | 141 | 17 | 0.69 |
| cH | uIU/mL | 3.1E0 | 1.9E0 | 6.7E0 | 5.7E0 | 1.3E1 | 1.2E1 | 8.6E-3 | 8.6E-3 | 1.6E2 | 5.3E1 | 363 | 17 | 141 | 17 | 0.40 |
| cI | ng/mL | 5.6E0 | 7.4E0 | 1.1E1 | 1.6E1 | 1.5E1 | 2.5E1 | 1.0E-3 | 3.2E-2 | 9.4E1 | 1.0E2 | 363 | 17 | 141 | 17 | 0.52 |
| cJ | ug/mL | 6.5E1 | 3.1E1 | 1.2E2 | 6.9E1 | 1.5E2 | 8.7E1 | 4.0E0 | 1.2E1 | 9.6E2 | 3.3E2 | 363 | 17 | 141 | 17 | 0.39 |
| cK | ng/mL | 3.8E-3 | 3.8E-3 | 5.9E-2 | 3.9E-2 | 2.0E-1 | 8.9E-2 | 3.8E-3 | 3.8E-3 | 1.7E0 | 3.0E-1 | 363 | 17 | 141 | 17 | 0.50 |
| cL | pg/mL | 1.9E2 | 2.4E2 | 4.0E2 | 5.2E2 | 1.5E3 | 6.7E2 | 1.6E1 | 6.5E1 | 2.4E4 | 2.6E3 | 363 | 17 | 141 | 17 | 0.66 |
| cM | pg/mL | 2.8E2 | 2.5E2 | 3.1E2 | 2.5E2 | 2.1E2 | 1.3E2 | 8.7E0 | 4.7E1 | 1.6E3 | 4.8E2 | 363 | 17 | 141 | 17 | 0.41 |
| cN | pg/mL | 1.2E2 | 1.2E2 | 1.3E2 | 1.5E2 | 7.0E1 | 6.7E1 | 3.8E1 | 6.8E1 | 1.1E3 | 3.5E2 | 363 | 17 | 141 | 17 | 0.61 |
| cO | pg/mL | 2.2E2 | 2.1E2 | 3.3E2 | 2.9E2 | 1.0E3 | 2.3E2 | 5.4E1 | 1.0E2 | 1.9E4 | 1.1E3 | 363 | 17 | 141 | 17 | 0.51 |
| cP | ng/mL | 2.6E3 | 2.2E3 | 2.6E3 | 2.7E3 | 9.4E2 | 9.7E2 | 6.2E2 | 1.4E3 | 5.7E3 | 4.8E3 | 363 | 17 | 141 | 17 | 0.48 |
| cQ | ng/mL | 4.9E-2 | 5.3E-2 | 1.2E-1 | 1.9E-1 | 2.3E-1 | 4.5E-1 | 2.0E-3 | 2.0E-3 | 2.2E0 | 1.9E0 | 363 | 17 | 141 | 17 | 0.49 |
| cR | ng/mL | 2.8E2 | 3.1E2 | 4.3E2 | 1.0E3 | 6.0E2 | 2.0E3 | 2.0E1 | 3.6E1 | 7.7E3 | 7.7E3 | 363 | 17 | 141 | 17 | 0.56 |
| cS | ng/mL | 2.6E2 | 3.4E2 | 3.7E2 | 1.7E3 | 3.7E2 | 5.3E3 | 4.7E1 | 9.1E1 | 2.7E3 | 2.2E4 | 363 | 17 | 141 | 17 | 0.57 |
| cT | ng/mL | 3.1E1 | 7.9E1 | 8.6E1 | 2.6E2 | 1.9E2 | 5.2E2 | 4.6E0 | 5.1E0 | 2.1E3 | 1.9E3 | 363 | 17 | 141 | 17 | 0.65 |
| cU | ng/mL | 5.3E1 | 8.4E1 | 7.7E1 | 9.6E1 | 1.1E2 | 7.7E1 | 6.2E0 | 5.4E0 | 1.6E3 | 2.9E2 | 363 | 17 | 141 | 17 | 0.58 |
| cV | ng/mL | 1.7E-1 | 2.9E-1 | 4.2E-1 | 1.5E0 | 2.5E0 | 2.6E0 | 3.4E-4 | 3.0E-2 | 4.7E1 | 9.7E0 | 363 | 17 | 141 | 17 | 0.69 |
| cW | mIU/mL | 5.3E-2 | 4.5E-2 | 1.6E-1 | 6.8E-2 | 7.9E-1 | 5.6E-2 | 3.7E-4 | 1.9E-2 | 9.7E0 | 2.1E-1 | 363 | 17 | 141 | 17 | 0.48 |
| cX | ng/mL | 1.0E-1 | 3.6E-1 | 1.4E0 | 4.9E0 | 4.5E0 | 9.9E0 | 2.3E-4 | 2.3E-4 | 2.8E1 | 2.8E1 | 363 | 17 | 141 | 17 | 0.56 |
| cY | ng/mL | 8.8E0 | 7.9E0 | 1.3E1 | 1.3E1 | 1.4E1 | 1.4E1 | 1.5E-1 | 6.1E-1 | 8.3E1 | 5.2E1 | 363 | 17 | 141 | 17 | 0.47 |
| cZ | ug/mL | 1.4E1 | 1.4E1 | 1.5E1 | 1.5E1 | 6.1E0 | 7.4E0 | 2.7E0 | 5.6E0 | 3.9E1 | 3.4E1 | 363 | 17 | 141 | 17 | 0.49 |
| dA | pg/mL | 3.3E2 | 3.2E2 | 3.7E2 | 3.7E2 | 3.3E2 | 2.4E2 | 9.0E1 | 1.6E2 | 5.8E3 | 9.3E2 | 363 | 17 | 141 | 17 | 0.44 |
| dB | ug/mL | 1.7E1 | 2.2E1 | 1.8E1 | 2.0E1 | 1.8E1 | 9.0E0 | 9.4E-1 | 2.5E0 | 2.5E2 | 3.2E1 | 363 | 17 | 141 | 17 | 0.67 |
| dC | nmol/L | 3.5E1 | 3.5E1 | 4.0E1 | 3.8E1 | 1.9E1 | 1.5E1 | 9.1E0 | 1.3E1 | 1.4E2 | 7.5E1 | 363 | 17 | 141 | 17 | 0.49 |
| dD | ug/mL | 3.6E1 | 3.0E1 | 3.7E1 | 3.4E1 | 1.1E1 | 1.3E1 | 1.3E1 | 1.6E1 | 7.6E1 | 5.6E1 | 363 | 17 | 141 | 17 | 0.42 |
| dE | ng/mL | 4.7E-1 | 3.8E-1 | 6.1E-1 | 4.4E-1 | 7.4E-1 | 4.4E-1 | 8.4E-3 | 8.4E-3 | 7.2E0 | 1.8E0 | 363 | 17 | 141 | 17 | 0.45 |
| dF | ng/mL | 2.2E2 | 3.1E2 | 2.7E2 | 4.3E2 | 1.8E2 | 3.0E2 | 7.5E1 | 6.1E1 | 1.3E3 | 1.2E3 | 363 | 17 | 141 | 17 | 0.69 |
| dG | ng/mL | 1.1E1 | 1.6E1 | 1.4E1 | 2.1E1 | 1.3E1 | 1.8E1 | 3.1E0 | 4.2E0 | 1.8E2 | 7.6E1 | 363 | 17 | 141 | 17 | 0.65 |
| dH | pg/mL | 7.5E0 | 1.1E1 | 1.3E1 | 1.8E1 | 4.1E1 | 1.9E1 | 4.0E-2 | 2.2E0 | 6.7E2 | 7.9E1 | 363 | 17 | 141 | 17 | 0.66 |
| dI | pg/mL | 4.6E-1 | 4.6E-1 | 2.5E0 | 3.6E0 | 1.8E1 | 9.7E0 | 4.6E-1 | 4.6E-1 | 3.3E2 | 4.1E1 | 363 | 17 | 141 | 17 | 0.53 |
| dJ | ng/mL | 1.9E0 | 2.6E0 | 2.2E0 | 2.6E0 | 1.2E0 | 1.3E0 | 3.2E-2 | 3.2E-2 | 6.9E0 | 4.4E0 | 363 | 17 | 141 | 17 | 0.61 |
| dK | uIU/mL | 1.9E0 | 2.4E0 | 3.2E0 | 3.0E0 | 6.5E0 | 3.4E0 | 2.8E-4 | 4.3E-1 | 7.9E1 | 1.5E1 | 363 | 17 | 141 | 17 | 0.54 |
| dL | ng/mL | 8.8E2 | 1.0E3 | 1.0E3 | 1.3E3 | 4.9E2 | 8.3E2 | 3.4E2 | 3.3E2 | 3.4E3 | 3.8E3 | 363 | 17 | 141 | 17 | 0.56 |
| dM | pg/mL | 9.7E2 | 1.1E3 | 1.2E3 | 1.5E3 | 9.3E2 | 1.1E3 | 3.9E2 | 3.4E2 | 1.2E4 | 4.3E3 | 363 | 17 | 141 | 17 | 0.60 |
| dN | ug/mL | 9.4E1 | 1.1E2 | 9.9E1 | 1.1E2 | 3.4E1 | 4.8E1 | 2.5E1 | 1.6E1 | 2.4E2 | 2.0E2 | 363 | 17 | 141 | 17 | 0.60 |
| dR | pg/ml | 1.6E3 | 1.3E3 | 2.4E3 | 2.4E3 | 2.5E3 | 2.8E3 | 1.4E2 | 1.8E2 | 1.5E4 | 8.9E3 | 245 | 15 | 138 | 15 | 0.46 |
| eF | ng/ml | 4.1E0 | 4.4E0 | 4.6E0 | 5.3E0 | 2.2E0 | 2.5E0 | 1.4E0 | 2.6E0 | 1.8E1 | 1.2E1 | 257 | 15 | 138 | 15 | 0.61 |
| eC | pg/ml | 3.1E2 | 2.7E2 | 3.7E2 | 4.5E2 | 2.2E2 | 4.3E2 | 4.5E1 | 1.3E2 | 1.4E3 | 1.6E3 | 203 | 14 | 135 | 14 | 0.47 |
| cD | pg/ml | 2.1E2 | 1.7E2 | 5.9E2 | 1.9E2 | 1.2E3 | 8.6E1 | 5.2E-1 | 3.1E1 | 7.0E3 | 3.0E2 | 173 | 13 | 111 | 13 | 0.41 |
| fP | ng/ml | 2.5E2 | 3.1E2 | 2.9E2 | 4.1E2 | 1.7E2 | 3.7E2 | 1.8E0 | 1.1E2 | 1.0E3 | 1.6E3 | 240 | 15 | 137 | 15 | 0.61 |
| fR | ng/ml | 1.2E5 | 2.3E5 | 1.7E5 | 3.2E5 | 1.4E5 | 2.3E5 | 3.1E4 | 1.0E5 | 7.2E5 | 7.2E5 | 236 | 11 | 68 | 11 | 0.74 |
| gL | pg/ml | 6.3E4 | 7.1E4 | 6.9E4 | 7.2E4 | 2.8E4 | 2.6E4 | 1.1E4 | 3.1E4 | 1.8E5 | 1.4E5 | 245 | 15 | 138 | 15 | 0.55 |
| gP | U/ml | 2.8E2 | 2.7E2 | 2.8E2 | 2.4E2 | 1.1E2 | 8.4E1 | 1.2E1 | 7.1E1 | 1.1E3 | 3.9E2 | 253 | 15 | 138 | 15 | 0.40 |
| gW | ng/ml | 5.7E2 | 5.4E2 | 1.2E3 | 9.5E2 | 1.7E3 | 1.3E3 | 3.1E-1 | 3.5E1 | 9.5E3 | 5.1E3 | 213 | 14 | 129 | 14 | 0.46 |
| tF | pg/mL | 1.5E3 | 2.1E3 | 1.6E4 | 5.9E3 | 4.7E4 | 7.8E3 | 1.2E1 | 1.8E1 | 3.2E5 | 2.3E4 | 203 | 14 | 135 | 14 | 0.54 |
| hA | ng/ml | 2.2E0 | 2.6E0 | 1.0E1 | 1.4E1 | 3.9E1 | 2.1E1 | 1.7E-2 | 1.7E-2 | 3.5E2 | 6.1E1 | 173 | 13 | 111 | 13 | 0.61 |
| nM | pg/ml | 1.0E-9 | 1.0E-9 | 9.1E1 | 1.0E-9 | 9.6E2 | 0.0E0 | 1.0E-9 | 1.0E-9 | 1.0E4 | 1.0E-9 | 113 | 11 | 88 | 11 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| nN | pg/ml | 1.4E3 | 1.8E3 | 5.8E3 | 2.2E3 | 2.7E4 | 1.8E3 | 1.1E2 | 4.1E2 | 2.7E5 | 6.2E3 | 113 | 11 | 88 | 11 | 0.53 |
| nO | pg/ml | 2.6E1 | 2.4E1 | 4.1E1 | 6.1E1 | 4.3E1 | 8.7E1 | 3.5E0 | 9.7E0 | 2.4E2 | 3.1E2 | 113 | 11 | 88 | 11 | 0.57 |
| nR | pg/ml | 1.5E1 | 8.6E1 | 4.3E1 | 1.6E2 | 9.3E1 | 2.1E2 | 1.0E-9 | 2.2E0 | 8.2E2 | 7.1E2 | 113 | 11 | 88 | 11 | 0.69 |
| nT | pg/ml | 7.3E1 | 5.3E1 | 2.2E2 | 8.9E1 | 8.5E2 | 7.4E1 | 1.0E-9 | 2.3E1 | 6.6E3 | 2.3E2 | 113 | 11 | 88 | 11 | 0.50 |
| nU | pg/ml | 3.9E1 | 1.2E2 | 3.0E2 | 1.4E2 | 1.6E3 | 1.3E2 | 1.0E-9 | 1.0E-9 | 1.3E4 | 4.4E2 | 113 | 11 | 88 | 11 | 0.71 |
| lW | pg/ml | 1.0E-9 | 1.0E-9 | 1.5E1 | 2.2E1 | 5.1E1 | 5.1E1 | 1.0E-9 | 1.0E-9 | 3.9E2 | 1.6E2 | 113 | 11 | 88 | 11 | 0.50 |
| lX | pg/ml | 9.4E2 | 8.9E2 | 9.9E2 | 9.2E2 | 5.3E2 | 5.5E2 | 1.2E2 | 3.3E2 | 2.5E3 | 1.8E3 | 113 | 11 | 88 | 11 | 0.46 |
| lY | pg/ml | 1.9E1 | 1.8E1 | 2.3E1 | 2.0E1 | 2.2E1 | 1.0E1 | 1.0E-9 | 4.2E0 | 1.4E2 | 3.9E1 | 113 | 11 | 88 | 11 | 0.51 |
| mE | pg/ml | 1.0E-9 | 1.0E-9 | 2.9E0 | 3.9E0 | 8.8E0 | 8.4E0 | 1.0E-9 | 1.0E-9 | 5.8E1 | 2.8E1 | 113 | 11 | 88 | 11 | 0.52 |
| mF | pg/ml | 1.0E-9 | 8.7E-1 | 4.3E0 | 3.9E0 | 2.5E1 | 4.7E0 | 1.0E-9 | 1.0E-9 | 2.5E2 | 1.3E1 | 113 | 11 | 88 | 11 | 0.69 |
| mH | pg/ml | 3.2E0 | 2.0E0 | 4.8E0 | 4.7E0 | 6.6E0 | 5.5E0 | 2.3E-1 | 7.6E-1 | 5.3E1 | 1.8E1 | 113 | 11 | 88 | 11 | 0.45 |
| mI | pg/ml | 1.0E-9 | 1.0E-9 | 1.3E1 | 1.2E1 | 2.9E1 | 2.2E1 | 1.0E-9 | 1.0E-9 | 1.8E2 | 6.1E1 | 113 | 11 | 88 | 11 | 0.46 |
| mM | pg/ml | 2.8E1 | 3.8E1 | 8.0E1 | 1.1E2 | 1.6E2 | 1.4E2 | 1.0E-9 | 1.0E-9 | 1.1E3 | 4.0E2 | 113 | 11 | 88 | 11 | 0.58 |
| mP | pg/ml | 1.4E1 | 1.8E1 | 2.0E1 | 2.5E1 | 2.6E1 | 1.9E1 | 1.0E-9 | 1.3E1 | 1.9E2 | 7.7E1 | 112 | 11 | 87 | 11 | 0.70 |
| mS | pg/ml | 1.8E3 | 2.0E3 | 2.0E3 | 2.0E3 | 1.7E3 | 1.2E3 | 1.0E-9 | 9.9E2 | 1.3E4 | 5.1E3 | 113 | 11 | 88 | 11 | 0.52 |
| mT | pg/ml | 5.4E1 | 4.9E1 | 1.2E2 | 1.2E2 | 1.9E2 | 1.5E2 | 1.0E1 | 1.2E1 | 1.4E3 | 5.0E2 | 112 | 11 | 87 | 11 | 0.48 |
| mU | pg/ml | 2.3E0 | 3.2E0 | 6.3E0 | 5.3E0 | 2.2E1 | 6.0E0 | 1.0E-9 | 1.7E0 | 2.2E2 | 2.2E1 | 112 | 11 | 87 | 11 | 0.63 |
| mW | pg/ml | 2.5E3 | 2.3E3 | 2.8E3 | 2.9E3 | 1.6E3 | 2.5E3 | 4.3E2 | 3.7E2 | 1.0E4 | 9.8E3 | 112 | 11 | 87 | 11 | 0.46 |
| mY | pg/ml | 6.2E2 | 8.9E2 | 9.1E2 | 9.8E2 | 1.4E3 | 6.0E2 | 1.0E-9 | 2.4E2 | 1.1E4 | 2.5E3 | 113 | 11 | 88 | 11 | 0.67 |
| mZ | pg/ml | 2.4E2 | 2.7E2 | 4.0E2 | 4.9E2 | 4.6E2 | 5.5E2 | 2.1E0 | 1.6E1 | 3.1E3 | 1.5E3 | 112 | 11 | 87 | 11 | 0.46 |
| nA | pg/ml | 1.6E0 | 2.5E0 | 1.2E1 | 1.0E1 | 4.7E1 | 1.9E1 | 1.0E-9 | 1.0E-9 | 4.4E2 | 6.5E1 | 112 | 11 | 87 | 11 | 0.55 |
| nB | pg/ml | 3.1E2 | 3.6E2 | 3.3E2 | 3.8E2 | 1.5E2 | 1.9E2 | 3.0E1 | 1.5E2 | 8.2E2 | 7.8E2 | 113 | 11 | 88 | 11 | 0.57 |
| nC | pg/ml | 1.0E-9 | 1.5E2 | 4.3E3 | 5.6E4 | 3.4E4 | 1.3E5 | 1.0E-9 | 1.0E-9 | 3.7E5 | 3.8E5 | 113 | 11 | 88 | 11 | 0.67 |
| nD | pg/ml | 7.9E0 | 1.1E1 | 3.8E1 | 3.5E1 | 2.2E2 | 7.5E1 | 1.0E-9 | 1.0E-9 | 1.7E3 | 2.6E2 | 112 | 11 | 87 | 11 | 0.65 |
| nF | pg/ml | 1.0E-9 | 1.0E-9 | 5.0E0 | 4.5E0 | 2.8E1 | 9.0E0 | 1.0E-9 | 1.0E-9 | 2.4E2 | 2.7E1 | 113 | 11 | 88 | 11 | 0.58 |
| nH | pg/ml | 3.8E-1 | 5.4E0 | 1.0E2 | 1.2E3 | 9.5E2 | 3.0E3 | 1.0E-9 | 1.0E-9 | 1.0E4 | 1.0E4 | 112 | 11 | 87 | 11 | 0.64 |
| nI | pg/ml | 2.8E0 | 4.6E1 | 1.7E2 | 6.8E1 | 9.0E2 | 9.9E1 | 1.0E-9 | 1.0E-9 | 9.2E3 | 3.5E2 | 113 | 11 | 88 | 11 | 0.54 |
| nJ | pg/ml | 1.7E-1 | 6.3E-1 | 4.8E1 | 1.3E1 | 4.8E2 | 4.0E1 | 1.0E-9 | 1.0E-9 | 5.2E3 | 1.3E2 | 113 | 11 | 88 | 11 | 0.60 |
| nK | pg/ml | 1.0E-9 | 1.0E-9 | 6.2E1 | 2.8E1 | 3.7E2 | 4.0E1 | 1.0E-9 | 1.0E-9 | 3.2E3 | 1.0E2 | 112 | 11 | 87 | 11 | 0.58 |
| nL | pg/ml | 1.0E-9 | 5.2E0 | 4.5E2 | 1.6E3 | 4.2E3 | 4.2E3 | 1.0E-9 | 1.0E-9 | 4.5E4 | 1.4E4 | 113 | 11 | 88 | 11 | 0.65 |
| hR | pg/ml | 2.6E4 | 2.2E4 | 2.7E4 | 2.6E4 | 1.1E4 | 1.1E4 | 1.1E1 | 1.3E4 | 5.8E4 | 4.5E4 | 165 | 12 | 109 | 12 | 0.44 |
| hV | pg/ml | 4.3E2 | 4.7E2 | 4.5E2 | 4.1E2 | 2.4E2 | 2.1E2 | 6.8E1 | 9.6E1 | 1.5E3 | 7.1E2 | 165 | 12 | 109 | 12 | 0.45 |
| hW | pg/ml | 1.6E3 | 2.4E3 | 2.1E3 | 2.3E3 | 1.7E3 | 8.4E2 | 2.2E2 | 1.1E3 | 1.0E4 | 3.5E3 | 165 | 12 | 109 | 12 | 0.65 |
| hX | pg/ml | 9.0E2 | 1.3E3 | 1.0E3 | 1.4E3 | 8.2E2 | 7.2E2 | 1.3E2 | 2.1E2 | 8.6E3 | 2.9E3 | 165 | 12 | 109 | 12 | 0.68 |
| iA | pg/ml | 1.5E2 | 2.3E2 | 3.0E2 | 3.4E2 | 6.6E2 | 3.0E2 | 1.1E1 | 4.1E1 | 7.1E3 | 9.5E2 | 203 | 14 | 135 | 14 | 0.64 |
| iB | ng/ml | 5.0E0 | 5.9E0 | 6.3E0 | 9.5E0 | 5.0E0 | 7.8E0 | 3.3E-2 | 1.6E0 | 3.8E1 | 2.4E1 | 173 | 13 | 111 | 13 | 0.61 |
| iC | U/ml | 2.3E-1 | 5.8E-1 | 1.0E0 | 1.3E0 | 4.9E0 | 2.8E0 | 1.0E-9 | 1.0E-1 | 5.5E1 | 1.1E1 | 173 | 13 | 111 | 13 | 0.71 |
| iH | ng/ml | 1.6E5 | 1.9E5 | 1.5E5 | 1.8E5 | 4.7E4 | 6.6E4 | 5.1E4 | 2.9E3 | 2.7E5 | 2.5E5 | 203 | 14 | 135 | 14 | 0.69 |
| iJ | ng/ml | 5.3E4 | 4.3E4 | 5.5E4 | 4.7E4 | 3.0E4 | 3.5E4 | 7.7E3 | 1.8E3 | 2.5E5 | 1.5E5 | 203 | 14 | 135 | 14 | 0.37 |
| hB | ng/ml | 4.3E-1 | 6.5E-1 | 5.4E-1 | 1.1E0 | 4.1E-1 | 9.5E-1 | 1.2E-1 | 1.2E-1 | 3.0E0 | 3.4E0 | 203 | 14 | 135 | 14 | 0.72 |
| hC | pg/ml | 4.1E3 | 7.6E3 | 7.1E3 | 9.4E3 | 1.0E4 | 1.1E4 | 1.0E-9 | 1.0E-9 | 1.1E5 | 4.3E4 | 203 | 14 | 135 | 14 | 0.55 |
| hF | ng/ml | 1.0E-9 | 1.0E-9 | 2.4E1 | 1.0E-9 | 2.9E2 | 0.0E0 | 1.0E-9 | 1.0E-9 | 4.0E3 | 1.0E-9 | 203 | 14 | 135 | 14 | 0.49 |
| hG | pg/ml | 7.0E3 | 7.0E3 | 7.7E3 | 7.3E3 | 3.3E3 | 2.5E3 | 1.7E3 | 3.7E3 | 2.0E4 | 1.2E4 | 203 | 14 | 135 | 14 | 0.49 |
| iO | ng/ml | 3.8E5 | 3.2E5 | 4.0E5 | 3.3E5 | 1.8E5 | 1.6E5 | 8.3E4 | 6.4E4 | 1.1E6 | 6.7E5 | 203 | 14 | 135 | 14 | 0.37 |
| iP | ng/ml | 5.1E4 | 3.5E4 | 5.7E4 | 4.6E4 | 5.5E4 | 2.6E4 | 2.4E3 | 3.4E3 | 5.5E5 | 9.7E4 | 203 | 14 | 135 | 14 | 0.44 |
| iZ | ng/ml | 1.6E3 | 1.8E3 | 1.8E3 | 2.0E3 | 7.6E2 | 7.8E2 | 4.7E2 | 1.1E3 | 5.7E3 | 4.1E3 | 201 | 14 | 133 | 14 | 0.59 |
| rC | pg/ml | 1.5E3 | 1.2E3 | 2.2E3 | 2.0E3 | 2.3E3 | 1.8E3 | 1.0E-9 | 1.9E2 | 1.5E4 | 7.3E3 | 163 | 13 | 105 | 13 | 0.49 |
| rB | pg/ml | 2.4E1 | 3.2E1 | 4.7E1 | 5.4E1 | 9.5E1 | 6.7E1 | 1.0E-9 | 1.0E-9 | 9.5E2 | 2.5E2 | 163 | 13 | 105 | 13 | 0.57 |
| jD | ng/ml | 3.6E1 | 2.2E1 | 5.2E1 | 3.0E1 | 6.3E1 | 3.0E1 | 1.0E-9 | 1.0E-9 | 5.1E2 | 1.0E2 | 172 | 13 | 111 | 13 | 0.39 |
| jE | ng/ml | 1.0E-9 | 1.0E-9 | 6.2E0 | 3.8E0 | 1.6E1 | 1.0E1 | 1.0E-9 | 1.0E-9 | 1.2E2 | 3.4E1 | 172 | 13 | 111 | 13 | 0.47 |
| jF | ng/ml | 3.1E1 | 2.3E1 | 5.0E1 | 2.4E1 | 6.1E1 | 2.3E1 | 1.0E-9 | 1.0E-9 | 3.5E2 | 5.7E1 | 172 | 13 | 111 | 13 | 0.40 |
| jG | ng/ml | 4.4E3 | 3.9E3 | 4.5E3 | 3.8E3 | 1.9E3 | 2.3E3 | 7.6E2 | 6.7E2 | 9.6E3 | 7.8E3 | 173 | 13 | 111 | 13 | 0.40 |
| jH | ng/ml | 7.7E1 | 5.5E1 | 8.6E1 | 6.2E1 | 4.8E1 | 2.9E1 | 1.3E1 | 1.5E1 | 3.3E2 | 1.3E2 | 173 | 13 | 111 | 13 | 0.33 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| jI | ng/ml | 7.3E1 | 8.9E1 | 7.7E1 | 9.3E1 | 3.3E1 | 3.9E1 | 2.8E1 | 5.4E1 | 2.5E2 | 1.8E2 | 173 | 13 | 111 | 13 | 0.65 |
| rA | pg/ml | 2.5E1 | 3.0E1 | 3.0E1 | 3.6E1 | 2.3E1 | 2.2E1 | 1.0E-9 | 5.8E0 | 1.2E2 | 7.2E1 | 171 | 14 | 108 | 14 | 0.59 |
| qZ | pg/ml | 4.3E1 | 2.7E1 | 4.3E2 | 1.5E3 | 1.9E3 | 3.8E3 | 1.0E-9 | 5.2E-4 | 1.0E4 | 1.0E4 | 132 | 7 | 96 | 7 | 0.53 |
| qY | pg/ml | 2.3E1 | 1.9E1 | 5.3E1 | 3.3E1 | 7.1E1 | 3.1E1 | 8.7E-1 | 5.1E0 | 5.3E2 | 9.8E1 | 171 | 14 | 108 | 14 | 0.47 |
| qX | pg/ml | 5.3E1 | 7.0E1 | 6.3E1 | 9.0E1 | 4.2E1 | 7.0E1 | 1.0E-9 | 2.9E0 | 2.1E2 | 2.1E2 | 171 | 14 | 108 | 14 | 0.58 |
| qW | pg/ml | 8.6E0 | 8.0E0 | 1.2E1 | 1.0E1 | 1.2E1 | 8.3E0 | 1.0E-9 | 1.0E-9 | 7.1E1 | 2.8E1 | 171 | 14 | 108 | 14 | 0.48 |
| qV | pg/ml | 2.1E3 | 2.5E3 | 2.8E3 | 2.6E3 | 2.1E3 | 1.5E3 | 1.0E2 | 1.7E2 | 1.1E4 | 4.9E3 | 171 | 14 | 108 | 14 | 0.51 |
| qU | pg/ml | 5.6E1 | 1.2E2 | 1.6E2 | 1.6E2 | 2.8E2 | 1.6E2 | 1.0E-9 | 1.0E-9 | 2.1E3 | 5.1E2 | 171 | 14 | 108 | 14 | 0.57 |
| qT | pg/ml | 4.1E1 | 7.3E1 | 6.9E1 | 1.0E2 | 1.0E2 | 1.1E2 | 1.0E-9 | 1.2E1 | 9.0E2 | 4.4E2 | 171 | 14 | 108 | 14 | 0.65 |
| jK | ng/ml | 1.6E3 | 1.5E3 | 1.7E3 | 1.6E3 | 6.6E2 | 5.4E2 | 2.8E2 | 8.7E2 | 4.3E3 | 2.4E3 | 173 | 13 | 111 | 13 | 0.46 |
| jL | ng/ml | 2.0E2 | 2.0E2 | 3.0E2 | 3.6E2 | 2.6E2 | 4.3E2 | 3.6E1 | 9.8E1 | 2.1E3 | 1.7E3 | 173 | 13 | 111 | 13 | 0.51 |
| jM | ng/ml | 7.0E4 | 6.7E4 | 7.3E4 | 7.1E4 | 3.7E4 | 3.5E4 | 3.9E2 | 1.1E4 | 1.7E5 | 1.4E5 | 173 | 13 | 111 | 13 | 0.49 |
| jO | pg/ml | 2.2E5 | 2.5E5 | 2.7E5 | 2.5E5 | 1.5E5 | 1.3E5 | 5.2E4 | 1.2E5 | 8.0E5 | 5.6E5 | 173 | 13 | 111 | 13 | 0.48 |
| jP | pg/ml | 2.4E5 | 2.6E5 | 2.7E5 | 2.8E5 | 1.6E5 | 1.3E5 | 5.8E4 | 7.8E4 | 9.2E5 | 5.8E5 | 173 | 13 | 111 | 13 | 0.57 |
| jQ | pg/ml | 2.5E3 | 2.4E3 | 3.4E3 | 2.6E3 | 3.4E3 | 1.8E3 | 1.0E-9 | 2.9E2 | 1.8E4 | 6.1E3 | 173 | 13 | 111 | 13 | 0.47 |
| jR | pg/ml | 5.9E3 | 3.7E3 | 1.0E4 | 8.9E3 | 1.2E4 | 1.0E4 | 1.0E-9 | 2.9E2 | 9.0E4 | 3.4E4 | 173 | 13 | 111 | 13 | 0.46 |
| jT | pg/ml | 1.7E5 | 1.6E5 | 1.8E5 | 1.6E5 | 6.8E4 | 5.1E4 | 6.8E4 | 8.8E4 | 4.5E5 | 2.5E5 | 173 | 13 | 111 | 13 | 0.42 |
| jU | mIU/ml | 4.6E0 | 3.0E0 | 1.0E1 | 8.6E0 | 1.7E1 | 1.2E1 | 6.2E-2 | 6.3E-2 | 1.1E2 | 4.3E1 | 173 | 13 | 111 | 13 | 0.47 |
| jV | mIU/ml | 1.5E0 | 1.2E0 | 3.4E0 | 3.3E0 | 5.8E0 | 5.0E0 | 1.7E-3 | 4.1E-2 | 3.3E1 | 1.8E1 | 173 | 13 | 111 | 13 | 0.49 |
| jY | ng/ml | 7.4E-4 | 2.7E-3 | 6.5E-3 | 1.2E-2 | 2.9E-2 | 2.6E-2 | 1.0E-9 | 1.0E-9 | 3.0E-1 | 9.4E-2 | 173 | 13 | 111 | 13 | 0.66 |
| kC | pg/ml | 9.6E1 | 8.2E1 | 1.9E2 | 3.3E2 | 4.1E2 | 8.0E2 | 2.1E1 | 3.6E1 | 3.5E3 | 2.7E3 | 113 | 11 | 88 | 11 | 0.43 |
| kE | pg/ml | 1.4E5 | 1.4E5 | 1.4E5 | 1.3E5 | 3.9E4 | 4.2E4 | 1.2E4 | 3.8E4 | 2.3E5 | 1.9E5 | 113 | 11 | 88 | 11 | 0.48 |
| kF | pg/mL | 6.3E1 | 7.5E1 | 7.2E1 | 7.4E1 | 5.2E1 | 2.5E1 | 2.6E1 | 4.0E1 | 5.1E2 | 1.3E2 | 113 | 11 | 88 | 11 | 0.60 |
| kG | pg/mL | 9.2E3 | 1.2E4 | 1.4E4 | 1.4E4 | 1.7E4 | 1.5E4 | 7.5E2 | 3.2E3 | 1.2E5 | 5.8E4 | 113 | 11 | 88 | 11 | 0.56 |
| kI | pg/ml | 2.0E2 | 2.0E2 | 2.2E2 | 2.5E2 | 1.3E2 | 1.5E2 | 4.4E1 | 7.2E1 | 8.7E2 | 5.5E2 | 113 | 11 | 88 | 11 | 0.55 |
| kK | pg/ml | 1.0E2 | 1.2E2 | 1.8E2 | 2.1E2 | 2.3E2 | 2.6E2 | 2.1E1 | 5.2E1 | 1.6E3 | 9.1E2 | 113 | 11 | 88 | 11 | 0.56 |
| kN | pg/ml | 1.0E3 | 8.8E2 | 2.1E3 | 1.9E3 | 5.6E3 | 2.5E3 | 7.6E1 | 4.4E2 | 5.5E4 | 8.7E3 | 113 | 11 | 88 | 11 | 0.52 |
| kO | pg/ml | 7.1E3 | 6.9E3 | 8.7E3 | 2.1E4 | 1.2E4 | 4.2E4 | 3.4E3 | 4.4E3 | 1.3E5 | 1.5E5 | 113 | 11 | 88 | 11 | 0.49 |
| kP | pg/ml | 6.4E3 | 5.4E3 | 7.5E3 | 6.7E3 | 6.3E3 | 3.6E3 | 8.6E2 | 1.5E3 | 4.8E4 | 1.3E4 | 113 | 11 | 88 | 11 | 0.51 |
| kQ | pg/ml | 4.3E3 | 4.0E3 | 5.0E3 | 4.4E3 | 2.9E3 | 1.4E3 | 5.6E2 | 2.5E3 | 2.5E4 | 7.0E3 | 203 | 14 | 135 | 14 | 0.47 |
| kR | pg/ml | 2.3E1 | 2.0E1 | 3.3E1 | 2.8E1 | 7.3E1 | 2.2E1 | 1.0E-9 | 5.6E0 | 1.0E3 | 7.7E1 | 203 | 14 | 135 | 14 | 0.47 |
| kS | pg/ml | 8.0E2 | 1.1E3 | 9.6E2 | 1.2E3 | 1.1E3 | 8.3E2 | 8.2E1 | 2.5E2 | 1.4E4 | 3.6E3 | 203 | 14 | 135 | 14 | 0.63 |
| rZ | ng/ml | 6.0E-4 | 2.2E-3 | 6.3E-3 | 6.0E-3 | 1.6E-2 | 7.6E-3 | 1.0E-9 | 1.0E-9 | 1.1E-1 | 2.2E-2 | 165 | 13 | 104 | 13 | 0.57 |
| rY | ng/ml | 6.1E-2 | 5.4E-2 | 4.1E-1 | 1.1E-1 | 2.4E0 | 1.3E-1 | 1.0E-9 | 1.0E-9 | 2.3E1 | 5.0E-1 | 165 | 13 | 104 | 13 | 0.53 |
| rX | ng/ml | 1.0E-9 | 1.0E-9 | 7.9E-2 | 2.8E-2 | 4.5E-1 | 7.9E-2 | 1.0E-9 | 1.0E-9 | 3.7E0 | 2.9E-1 | 165 | 13 | 104 | 13 | 0.52 |
| lK | pg/ml | 8.1E1 | 8.4E1 | 1.8E2 | 1.2E2 | 3.2E2 | 1.4E2 | 1.0E-9 | 1.0E-9 | 3.3E3 | 5.0E2 | 172 | 13 | 111 | 13 | 0.44 |
| lL | pg/ml | 1.6E3 | 1.1E3 | 2.7E3 | 2.1E3 | 4.0E3 | 2.3E3 | 1.5E1 | 3.8E2 | 4.2E4 | 7.7E3 | 173 | 13 | 111 | 13 | 0.44 |
| lM | pg/ml | 1.1E3 | 1.2E3 | 4.1E3 | 4.8E3 | 8.6E3 | 7.9E3 | 3.9E1 | 9.5E0 | 5.1E4 | 2.7E4 | 173 | 13 | 111 | 13 | 0.56 |
| lN | pg/ml | 1.0E-9 | 1.0E-9 | 4.5E0 | 3.0E0 | 1.6E1 | 5.4E0 | 1.0E-9 | 1.0E-9 | 1.5E2 | 2.0E1 | 173 | 13 | 111 | 13 | 0.52 |
| lO | pg/ml | 1.0E-9 | 1.0E-9 | 1.8E0 | 1.0E-9 | 1.2E1 | 0.0E0 | 1.0E-9 | 1.0E-9 | 1.3E2 | 1.0E-9 | 172 | 13 | 111 | 13 | 0.48 |
| nW | pg/ml | 1.1E5 | 1.1E5 | 1.1E5 | 1.2E5 | 2.5E4 | 3.4E4 | 5.8E4 | 6.7E4 | 1.8E5 | 1.7E5 | 203 | 14 | 135 | 14 | 0.52 |
| nY | pg/ml | 2.1E3 | 2.1E3 | 2.4E3 | 2.5E3 | 1.6E3 | 1.4E3 | 5.1E2 | 7.6E2 | 1.3E4 | 5.0E3 | 203 | 14 | 135 | 14 | 0.52 |
| oO | pg/ml | 8.6E4 | 1.1E5 | 1.2E5 | 1.3E5 | 1.0E5 | 8.0E4 | 2.6E4 | 5.1E4 | 6.2E5 | 2.8E5 | 105 | 10 | 82 | 10 | 0.58 |
| oP | pg/ml | 1.4E5 | 2.1E5 | 1.5E5 | 2.1E5 | 9.3E4 | 9.3E4 | 4.2E4 | 1.1E5 | 4.5E5 | 4.2E5 | 105 | 10 | 82 | 10 | 0.71 |
| oQ | pg/ml | 3.0E3 | 4.4E3 | 3.9E3 | 5.3E3 | 3.0E3 | 3.2E3 | 1.1E3 | 1.9E3 | 2.1E4 | 1.2E4 | 105 | 10 | 82 | 10 | 0.68 |
| oE | pg/ml | 1.5E2 | 1.1E2 | 3.8E2 | 5.6E2 | 5.5E2 | 8.8E2 | 1.0E-9 | 2.8E0 | 4.7E3 | 2.8E3 | 203 | 14 | 135 | 14 | 0.49 |
| oF | pg/ml | 8.5E3 | 1.3E4 | 2.2E4 | 2.7E4 | 3.6E4 | 3.3E4 | 6.4E1 | 4.6E2 | 2.5E5 | 1.1E5 | 203 | 14 | 135 | 14 | 0.53 |
| oH | pg/ml | 4.1E1 | 2.4E1 | 9.4E1 | 8.0E1 | 1.4E2 | 8.9E1 | 4.2E0 | 4.3E-1 | 9.9E2 | 2.6E2 | 203 | 14 | 135 | 14 | 0.43 |
| oK | pg/ml | 7.6E2 | 1.3E3 | 1.9E3 | 1.6E3 | 2.6E3 | 1.6E3 | 5.2E1 | 1.8E2 | 1.8E4 | 6.8E3 | 203 | 14 | 135 | 14 | 0.55 |
| oN | pg/ml | 5.1E2 | 6.0E2 | 7.9E2 | 7.1E2 | 1.5E3 | 4.4E2 | 1.5E2 | 2.2E2 | 1.8E4 | 1.9E3 | 203 | 14 | 135 | 14 | 0.57 |
| pF | pg/ml | 4.8E-1 | 6.6E-1 | 1.1E0 | 1.5E0 | 6.1E0 | 2.1E0 | 1.0E-9 | 1.0E-9 | 8.7E1 | 7.8E0 | 203 | 14 | 135 | 14 | 0.61 |

Figure 21.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 6.4E1 | 5.3E1 | 7.5E1 | 7.1E1 | 5.1E1 | 5.3E1 | 2.0E0 | 5.0E0 | 4.4E2 | 2.0E2 | 965 | 33 | 171 | 33 | 0.47 |
| Ad | ug/mL | 2.5E-2 | 5.8E-2 | 5.8E-2 | 8.3E-2 | 7.7E-2 | 8.9E-2 | 6.8E-4 | 3.6E-3 | 3.7E-1 | 3.6E-1 | 255 | 27 | 103 | 27 | 0.64 |
| Af | ng/mL | 9.2E-1 | 9.5E-1 | 1.8E1 | 2.8E1 | 7.2E1 | 9.4E1 | 1.7E-3 | 1.7E-3 | 5.3E2 | 4.8E2 | 255 | 27 | 103 | 27 | 0.51 |
| Aj | ug/mL | 1.4E0 | 4.7E0 | 2.6E0 | 3.4E0 | 2.4E0 | 2.4E0 | 1.5E-3 | 3.8E-2 | 6.1E0 | 5.8E0 | 255 | 27 | 103 | 27 | 0.57 |
| Al | mg/mL | 9.0E-5 | 1.1E-4 | 2.5E-4 | 3.1E-4 | 4.2E-4 | 5.3E-4 | 2.5E-6 | 2.1E-5 | 1.9E-3 | 2.2E-3 | 255 | 27 | 103 | 27 | 0.55 |
| An | U/mL | 4.0E1 | 6.6E1 | 1.5E2 | 2.2E2 | 4.7E2 | 4.9E2 | 9.8E-4 | 6.1E-1 | 5.5E3 | 2.5E3 | 255 | 27 | 103 | 27 | 0.59 |
| Ao | pg/mL | 8.5E1 | 7.2E1 | 2.1E2 | 1.6E3 | 1.0E3 | 7.4E3 | 1.5E0 | 7.4E0 | 1.6E4 | 3.9E4 | 255 | 27 | 103 | 27 | 0.48 |
| Ap | ng/mL | 2.7E1 | 5.2E1 | 4.3E1 | 6.0E1 | 5.0E1 | 4.6E1 | 9.9E-1 | 1.8E0 | 3.3E2 | 1.6E2 | 255 | 27 | 103 | 27 | 0.63 |
| Ar | ng/mL | 6.5E-1 | 1.4E0 | 2.0E0 | 4.7E0 | 4.1E0 | 9.7E0 | 3.4E-3 | 8.4E-2 | 4.3E1 | 4.7E1 | 255 | 27 | 103 | 27 | 0.60 |
| As | ng/mL | 8.7E-3 | 8.7E-3 | 1.2E-2 | 1.1E-2 | 1.6E-2 | 9.9E-3 | 1.7E-3 | 1.7E-3 | 1.1E-1 | 4.2E-2 | 255 | 27 | 103 | 27 | 0.51 |
| Aw | pg/mL | 1.6E1 | 1.6E1 | 1.6E1 | 1.8E1 | 5.4E0 | 8.2E0 | 3.5E0 | 6.7E0 | 3.3E1 | 4.8E1 | 255 | 27 | 103 | 27 | 0.57 |
| Ax | ng/mL | 2.1E0 | 1.6E0 | 8.2E0 | 9.3E0 | 1.8E1 | 1.5E1 | 1.9E-2 | 4.9E-2 | 1.5E2 | 5.1E1 | 255 | 27 | 103 | 27 | 0.52 |
| Ba | ng/mL | 3.7E1 | 1.2E2 | 2.8E2 | 7.9E2 | 7.3E2 | 1.7E3 | 3.7E-1 | 1.2E0 | 8.1E3 | 8.1E3 | 255 | 27 | 103 | 27 | 0.62 |
| Bb | ng/mL | 2.6E0 | 3.7E0 | 5.3E0 | 1.4E1 | 8.5E0 | 4.7E1 | 4.1E-3 | 4.1E-3 | 6.6E1 | 2.5E2 | 255 | 27 | 103 | 27 | 0.58 |
| Bc | ng/mL | 3.3E1 | 4.7E1 | 9.7E1 | 1.1E2 | 1.8E2 | 1.8E2 | 1.1E-1 | 4.2E-1 | 1.0E3 | 8.9E2 | 255 | 27 | 103 | 27 | 0.55 |
| Bg | ng/mL | 6.4E-2 | 1.6E-1 | 4.4E0 | 2.7E0 | 2.2E1 | 9.4E0 | 5.3E-4 | 5.3E-4 | 2.5E2 | 4.9E1 | 255 | 27 | 103 | 27 | 0.58 |
| Bn | ng/mL | 5.6E-2 | 5.6E-2 | 8.5E-1 | 1.4E0 | 1.6E0 | 2.2E0 | 5.6E-2 | 5.6E-2 | 8.5E0 | 7.2E0 | 255 | 27 | 103 | 27 | 0.55 |
| Bo | ng/mL | 1.1E1 | 1.9E1 | 1.3E1 | 2.5E1 | 9.6E0 | 3.9E1 | 1.6E-2 | 1.6E-2 | 7.4E1 | 2.1E2 | 255 | 27 | 103 | 27 | 0.61 |
| Ch | uIU/mL | 9.0E-1 | 1.5E0 | 6.5E0 | 2.3E1 | 2.1E1 | 5.7E1 | 3.4E-3 | 3.4E-3 | 2.3E2 | 2.1E2 | 255 | 27 | 103 | 27 | 0.57 |
| Co | pg/mL | 3.1E1 | 4.6E1 | 9.1E1 | 4.1E2 | 2.1E2 | 1.7E3 | 3.9E0 | 5.4E0 | 1.9E3 | 9.0E3 | 255 | 27 | 103 | 27 | 0.60 |
| Cp | ng/mL | 1.9E1 | 2.4E1 | 2.5E1 | 3.5E1 | 2.5E1 | 4.3E1 | 6.0E-1 | 2.5E0 | 2.9E2 | 2.4E2 | 255 | 27 | 103 | 27 | 0.60 |
| Cq | ng/mL | 2.4E-2 | 2.2E-2 | 1.6E-1 | 1.2E-1 | 1.1E0 | 4.1E-1 | 8.0E-4 | 8.0E-4 | 1.7E1 | 2.1E0 | 255 | 27 | 103 | 27 | 0.48 |
| Cs | ng/mL | 4.5E1 | 4.7E1 | 2.1E2 | 2.1E2 | 4.0E2 | 3.9E2 | 2.7E-2 | 2.8E0 | 2.9E3 | 1.6E3 | 255 | 27 | 103 | 27 | 0.50 |
| Ct | ng/mL | 1.2E0 | 3.5E-1 | 2.6E1 | 4.6E1 | 8.2E1 | 1.3E2 | 1.1E-4 | 2.5E-2 | 6.2E2 | 4.7E2 | 255 | 27 | 103 | 27 | 0.46 |
| Cu | ng/mL | 2.1E-1 | 2.3E-1 | 4.2E-1 | 3.7E-1 | 9.2E-1 | 3.4E-1 | 9.6E-3 | 3.6E-2 | 9.2E0 | 1.4E0 | 255 | 27 | 103 | 27 | 0.56 |
| Cv | ng/mL | 4.6E0 | 8.7E0 | 2.4E1 | 2.4E1 | 7.0E1 | 4.8E1 | 5.6E-3 | 2.9E-1 | 5.3E2 | 2.4E2 | 255 | 27 | 103 | 27 | 0.59 |
| Cw | mIU/mL | 2.8E-2 | 4.1E-2 | 3.8E-2 | 5.5E-2 | 3.0E-2 | 5.2E-2 | 2.3E-3 | 2.8E-3 | 1.9E-1 | 2.4E-1 | 255 | 27 | 103 | 27 | 0.61 |
| Cx | ng/mL | 1.8E-1 | 3.2E-2 | 5.4E1 | 7.0E1 | 1.0E2 | 1.3E2 | 9.3E-5 | 9.3E-5 | 3.9E2 | 3.9E2 | 255 | 27 | 103 | 27 | 0.48 |
| Db | ug/mL | 8.2E0 | 8.3E0 | 9.4E0 | 1.0E1 | 8.8E0 | 7.9E0 | 5.3E-1 | 9.3E-1 | 1.0E2 | 3.2E1 | 255 | 27 | 103 | 27 | 0.54 |
| Dc | nmol/L | 1.8E-2 | 2.5E-2 | 5.7E-2 | 4.1E-2 | 1.3E-1 | 5.5E-2 | 5.2E-6 | 1.4E-3 | 1.2E0 | 2.3E-1 | 255 | 27 | 103 | 27 | 0.54 |
| Dd | ug/mL | 6.9E-2 | 1.5E-1 | 1.8E-1 | 1.7E-1 | 2.6E-1 | 1.7E-1 | 1.9E-4 | 1.9E-3 | 1.6E0 | 7.8E-1 | 255 | 27 | 103 | 27 | 0.57 |
| De | ng/mL | 3.4E-3 | 3.4E-3 | 5.6E-2 | 9.1E-2 | 1.1E-1 | 1.9E-1 | 3.4E-3 | 3.4E-3 | 5.9E-1 | 8.2E-1 | 255 | 27 | 103 | 27 | 0.51 |
| Dg | ng/mL | 2.5E1 | 5.5E1 | 3.9E1 | 5.5E1 | 3.8E1 | 4.3E1 | 2.8E-1 | 2.9E0 | 1.9E2 | 1.9E2 | 255 | 27 | 103 | 27 | 0.61 |
| Di | pg/mL | 1.6E0 | 2.0E0 | 2.0E0 | 2.6E0 | 1.8E0 | 2.7E0 | 1.8E-1 | 1.8E-1 | 1.3E1 | 9.2E0 | 255 | 27 | 103 | 27 | 0.54 |
| Dk | uIU/mL | 1.4E-2 | 1.2E-2 | 9.3E-2 | 1.4E-1 | 6.3E-1 | 5.1E-1 | 1.1E-4 | 1.1E-4 | 8.9E0 | 2.7E0 | 255 | 27 | 103 | 27 | 0.53 |
| Dl | ng/mL | 2.0E2 | 2.9E2 | 2.7E2 | 3.6E2 | 2.4E2 | 3.4E2 | 3.1E0 | 1.3E1 | 1.4E3 | 1.3E3 | 255 | 27 | 103 | 27 | 0.57 |
| Dp | ng/ml | 2.5E0 | 1.3E0 | 4.9E0 | 4.6E0 | 6.8E0 | 7.1E0 | 3.7E-3 | 3.7E-3 | 4.3E1 | 2.7E1 | 122 | 26 | 99 | 26 | 0.43 |
| Ef | ng/ml | 9.5E-2 | 1.9E-1 | 6.9E-1 | 8.6E-1 | 1.7E0 | 2.0E0 | 5.7E-4 | 1.1E-2 | 1.0E1 | 9.5E0 | 165 | 26 | 102 | 26 | 0.58 |
| Wm | % | 8.2E-1 | 4.9E-1 | 3.0E1 | 3.8E0 | 1.9E2 | 1.1E1 | 5.4E-2 | 5.4E-2 | 2.4E3 | 5.5E1 | 204 | 27 | 119 | 27 | 0.41 |
| Ed | pg/ml | 5.2E-1 | 5.2E-1 | 9.3E1 | 9.8E0 | 6.6E2 | 2.4E1 | 5.2E-1 | 5.2E-1 | 7.3E3 | 1.0E2 | 122 | 26 | 98 | 26 | 0.38 |
| Tj | pg/mL | 3.7E-1 | 3.7E-1 | 7.6E1 | 2.9E1 | 3.7E2 | 1.1E2 | 3.6E-1 | 3.7E-1 | 3.5E3 | 5.9E2 | 165 | 26 | 103 | 26 | 0.48 |
| Po | pg/ml | 1.3E-1 | 2.6E-2 | 8.0E0 | 8.2E0 | 2.7E1 | 2.0E1 | 8.0E-3 | 2.6E-2 | 2.7E2 | 1.1E2 | 359 | 43 | 189 | 43 | 0.49 |
| Et | ng/ml | 1.0E3 | 1.4E3 | 1.3E3 | 1.9E3 | 1.0E3 | 1.4E3 | 7.5E1 | 1.6E2 | 4.9E3 | 4.9E3 | 359 | 43 | 189 | 43 | 0.61 |
| Fa | ng/ml | 3.9E1 | 3.7E1 | 1.2E2 | 1.6E2 | 7.3E2 | 5.5E2 | 3.4E-2 | 3.4E0 | 8.0E3 | 2.8E3 | 120 | 26 | 98 | 26 | 0.55 |
| Ez | ng/ml | 3.7E0 | 3.5E0 | 1.6E1 | 1.1E1 | 3.9E1 | 1.8E1 | 1.3E-2 | 1.3E-2 | 3.0E2 | 6.6E1 | 122 | 26 | 99 | 26 | 0.48 |
| Fb | ng/ml | 2.3E1 | 2.8E1 | 2.1E1 | 2.3E1 | 1.2E1 | 1.2E1 | 1.0E0 | 8.1E-1 | 5.7E1 | 4.0E1 | 120 | 26 | 98 | 26 | 0.57 |
| Ex | ng/ml | 6.0E-2 | 1.0E-1 | 2.4E-1 | 3.7E-1 | 8.4E-1 | 1.0E0 | 3.5E-5 | 1.7E-4 | 8.9E0 | 4.9E0 | 126 | 22 | 69 | 22 | 0.60 |
| Fn | ng/ml | 2.1E-1 | 2.1E-1 | 8.5E0 | 3.7E0 | 3.8E1 | 7.1E0 | 1.1E-14 | 1.1E-14 | 4.2E2 | 3.2E1 | 122 | 26 | 99 | 26 | 0.46 |
| Fp | ng/ml | 9.4E0 | 1.3E1 | 2.0E1 | 2.6E1 | 2.6E1 | 3.3E1 | 6.0E-3 | 1.3E-1 | 1.4E2 | 1.2E2 | 380 | 43 | 189 | 43 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fr | ng/ml | 2.5E4 | 3.7E4 | 9.9E4 | 1.1E5 | 1.7E5 | 1.5E5 | 6.4E2 | 1.9E2 | 8.4E5 | 6.0E5 | 464 | 43 | 193 | 43 | 0.54 |
| Fw | pg/ml | 7.1E-1 | 1.1E0 | 1.0E2 | 4.8E0 | 6.7E2 | 9.0E0 | 1.1E-14 | 1.7E-14 | 6.9E3 | 4.2E1 | 167 | 26 | 103 | 26 | 0.46 |
| Fy | ng/ml | 3.1E1 | 2.8E1 | 4.9E1 | 3.8E1 | 5.5E1 | 3.8E1 | 1.2E-1 | 1.2E-1 | 3.3E2 | 1.8E2 | 121 | 25 | 98 | 25 | 0.47 |
| Gl | pg/ml | 6.1E3 | 6.7E3 | 9.8E3 | 9.8E3 | 9.0E3 | 9.6E3 | 2.3E2 | 2.0E2 | 3.4E4 | 3.0E4 | 162 | 26 | 102 | 26 | 0.49 |
| Gp | U/ml | 1.8E0 | 8.2E-1 | 4.4E0 | 3.3E0 | 7.6E0 | 5.7E0 | 1.3E-3 | 5.7E-3 | 6.7E1 | 2.3E1 | 167 | 26 | 103 | 26 | 0.40 |
| Gz | ug/ml | 1.2E0 | 6.6E0 | 1.1E1 | 6.3E0 | 5.3E1 | 6.0E0 | 2.9E-16 | 1.5E-1 | 4.8E2 | 1.9E1 | 81 | 22 | 64 | 22 | 0.54 |
| Ha | ng/ml | 2.7E0 | 2.3E0 | 8.4E0 | 1.1E1 | 1.9E1 | 2.1E1 | 1.7E-2 | 1.7E-2 | 1.0E2 | 7.7E1 | 120 | 26 | 98 | 26 | 0.50 |
| Nm | pg/ml | 1.2E4 | 1.5E4 | 2.4E4 | 2.3E4 | 4.9E4 | 2.8E4 | 1.0E-9 | 1.0E-9 | 7.8E5 | 1.2E5 | 358 | 43 | 190 | 43 | 0.51 |
| Nn | pg/ml | 1.3E2 | 9.7E1 | 2.5E3 | 6.7E2 | 1.1E4 | 1.2E3 | 1.0E-9 | 1.0E-9 | 1.0E5 | 4.9E3 | 358 | 43 | 190 | 43 | 0.53 |
| No | pg/ml | 1.3E1 | 8.7E0 | 2.7E1 | 2.4E1 | 5.9E1 | 3.5E1 | 1.0E-9 | 1.0E-9 | 5.9E2 | 1.4E2 | 358 | 43 | 190 | 43 | 0.46 |
| Nq | pg/ml | 2.2E0 | 1.0E-9 | 2.1E1 | 1.2E1 | 8.4E1 | 5.0E1 | 1.0E-9 | 1.0E-9 | 1.2E3 | 3.3E2 | 358 | 43 | 190 | 43 | 0.42 |
| Nr | pg/ml | 4.7E-1 | 6.1E-1 | 1.6E1 | 1.3E1 | 7.0E1 | 3.4E1 | 1.0E-9 | 1.0E-9 | 9.8E2 | 1.7E2 | 358 | 43 | 190 | 43 | 0.51 |
| Ns | pg/ml | 1.0E-9 | 1.0E-9 | 8.9E0 | 6.9E0 | 4.5E1 | 2.7E1 | 1.0E-9 | 1.0E-9 | 6.8E2 | 1.3E2 | 358 | 43 | 190 | 43 | 0.50 |
| Nt | pg/ml | 9.4E1 | 1.0E2 | 1.2E2 | 1.2E2 | 9.2E1 | 7.5E1 | 1.0E-9 | 1.0E-9 | 5.9E2 | 3.4E2 | 358 | 43 | 190 | 43 | 0.54 |
| Nu | pg/ml | 1.7E1 | 4.3E1 | 4.9E1 | 6.5E1 | 8.8E1 | 7.3E1 | 1.0E-9 | 1.0E-9 | 8.9E2 | 2.6E2 | 358 | 43 | 190 | 43 | 0.57 |
| Lu | pg/ml | 1.0E4 | 1.0E4 | 1.5E4 | 9.0E3 | 3.2E4 | 5.8E3 | 6.7E2 | 1.4E3 | 3.9E5 | 2.1E4 | 360 | 43 | 190 | 43 | 0.45 |
| Lv | pg/ml | 1.0E-9 | 1.0E-9 | 1.1E1 | 1.4E1 | 2.2E1 | 2.0E1 | 1.0E-9 | 1.0E-9 | 2.4E2 | 7.5E1 | 360 | 43 | 190 | 43 | 0.55 |
| Lw | pg/ml | 1.0E-9 | 1.0E-9 | 1.4E-1 | 2.3E-1 | 1.8E0 | 1.5E0 | 1.0E-9 | 1.0E-9 | 3.2E1 | 9.9E0 | 360 | 43 | 190 | 43 | 0.50 |
| Lx | pg/ml | 1.0E-9 | 1.0E-9 | 1.1E2 | 1.6E2 | 4.2E2 | 3.6E2 | 1.0E-9 | 1.0E-9 | 6.2E3 | 1.8E3 | 360 | 43 | 190 | 43 | 0.54 |
| Ly | pg/ml | 1.0E-9 | 1.1E1 | 9.1E0 | 1.7E1 | 1.8E1 | 1.8E1 | 1.0E-9 | 1.0E-9 | 1.2E2 | 5.6E1 | 360 | 43 | 190 | 43 | 0.65 |
| Lz | pg/ml | 1.0E-9 | 1.0E-9 | 2.9E0 | 1.5E0 | 2.4E1 | 7.1E0 | 1.0E-9 | 1.0E-9 | 4.3E2 | 4.3E1 | 360 | 43 | 190 | 43 | 0.49 |
| Ma | pg/ml | 2.4E2 | 3.3E2 | 1.5E3 | 1.3E3 | 4.7E3 | 2.3E3 | 1.0E-9 | 1.0E-9 | 6.5E4 | 1.2E4 | 360 | 43 | 190 | 43 | 0.50 |
| Mb | pg/ml | 2.5E1 | 2.7E1 | 3.1E1 | 3.1E1 | 1.4E1 | 1.2E1 | 5.4E0 | 1.4E1 | 9.3E1 | 5.9E1 | 360 | 43 | 190 | 43 | 0.54 |
| Mc | pg/ml | 1.0E-9 | 1.0E-9 | 1.7E-2 | 5.8E-2 | 2.3E-1 | 3.8E-1 | 1.0E-9 | 1.0E-9 | 4.0E0 | 2.5E0 | 360 | 43 | 190 | 43 | 0.51 |
| Md | pg/ml | 1.0E-9 | 1.0E-9 | 6.2E-1 | 2.4E-1 | 5.2E0 | 1.2E0 | 1.0E-9 | 1.0E-9 | 6.8E1 | 7.3E0 | 360 | 43 | 190 | 43 | 0.51 |
| Me | pg/ml | 3.2E1 | 2.4E1 | 2.9E1 | 2.6E1 | 1.6E1 | 1.3E1 | 1.0E-9 | 4.2E0 | 1.2E2 | 5.5E1 | 360 | 43 | 190 | 43 | 0.42 |
| Mf | pg/ml | 1.0E-9 | 1.0E-9 | 3.7E-1 | 3.8E-1 | 1.6E0 | 1.3E0 | 1.0E-9 | 1.0E-9 | 2.0E1 | 7.7E0 | 360 | 43 | 190 | 43 | 0.54 |
| Mg | pg/ml | 1.9E0 | 3.3E0 | 6.4E0 | 7.1E0 | 1.1E1 | 9.5E0 | 1.0E-9 | 1.0E-9 | 8.8E1 | 3.5E1 | 360 | 43 | 190 | 43 | 0.55 |
| Mh | pg/ml | 1.0E-9 | 1.0E-9 | 1.1E0 | 3.4E-1 | 8.4E0 | 1.3E0 | 1.0E-9 | 1.0E-9 | 1.1E2 | 7.8E0 | 360 | 43 | 190 | 43 | 0.48 |
| Mi | pg/ml | 1.0E-9 | 1.0E-9 | 6.9E-1 | 1.0E-9 | 7.1E0 | 0.0E0 | 1.0E-9 | 1.0E-9 | 1.2E2 | 1.0E-9 | 360 | 43 | 190 | 43 | 0.49 |
| Mj | pg/ml | 1.0E-9 | 1.0E-9 | 5.2E0 | 1.9E0 | 3.1E1 | 5.6E0 | 1.0E-9 | 1.0E-9 | 4.6E2 | 2.9E1 | 360 | 43 | 190 | 43 | 0.50 |
| Mk | pg/ml | 6.5E-1 | 1.0E-9 | 1.6E1 | 1.5E1 | 9.9E1 | 5.4E1 | 1.0E-9 | 1.0E-9 | 1.7E3 | 3.3E2 | 360 | 43 | 190 | 43 | 0.46 |
| Ml | pg/ml | 1.0E-9 | 1.0E-9 | 9.1E0 | 3.2E-1 | 1.1E2 | 8.0E-1 | 1.0E-9 | 1.0E-9 | 2.1E3 | 2.8E0 | 360 | 43 | 190 | 43 | 0.48 |
| Mm | pg/ml | 4.0E2 | 5.3E2 | 8.0E2 | 9.2E2 | 9.7E2 | 1.6E3 | 1.0E-9 | 1.0E-9 | 6.0E3 | 9.9E3 | 360 | 43 | 190 | 43 | 0.51 |
| Mn | pg/ml | 4.8E0 | 4.4E0 | 1.0E1 | 6.1E0 | 2.5E1 | 6.4E0 | 1.0E-9 | 1.0E-9 | 3.5E2 | 3.3E1 | 360 | 43 | 190 | 43 | 0.46 |
| Mp | pg/ml | 1.0E-9 | 1.0E-9 | 9.7E0 | 5.3E0 | 2.2E1 | 1.6E1 | 1.0E-9 | 1.0E-9 | 1.8E2 | 1.0E2 | 360 | 43 | 190 | 43 | 0.44 |
| Mq | pg/ml | 1.0E-9 | 1.0E-9 | 2.8E0 | 4.1E0 | 1.7E1 | 1.5E1 | 1.0E-9 | 1.0E-9 | 2.1E2 | 6.5E1 | 360 | 43 | 190 | 43 | 0.50 |
| Mr | pg/ml | 1.0E-9 | 1.0E-9 | 1.4E1 | 2.4E1 | 8.7E1 | 7.2E1 | 1.0E-9 | 1.0E-9 | 1.4E3 | 3.1E2 | 360 | 43 | 190 | 43 | 0.54 |
| Ms | pg/ml | 3.8E2 | 4.3E2 | 5.6E2 | 5.2E2 | 7.1E2 | 5.3E2 | 1.0E-9 | 1.0E-9 | 5.6E3 | 2.5E3 | 360 | 43 | 190 | 43 | 0.50 |
| Mt | pg/ml | 1.0E-9 | 4.8E-1 | 1.1E1 | 3.5E0 | 6.3E1 | 1.2E1 | 1.0E-9 | 1.0E-9 | 8.7E2 | 7.0E1 | 360 | 43 | 190 | 43 | 0.51 |
| Mu | pg/ml | 1.0E-9 | 1.0E-9 | 1.5E0 | 6.7E-1 | 1.0E1 | 3.5E0 | 1.0E-9 | 1.0E-9 | 9.9E1 | 2.3E1 | 360 | 43 | 190 | 43 | 0.50 |
| Mv | pg/ml | 1.0E-9 | 1.0E-9 | 8.2E1 | 3.3E1 | 4.2E2 | 1.5E2 | 1.0E-9 | 1.0E-9 | 4.5E3 | 9.4E2 | 360 | 43 | 190 | 43 | 0.48 |
| Mw | pg/ml | 2.5E1 | 1.6E1 | 5.8E2 | 4.2E2 | 3.9E3 | 2.4E3 | 1.0E-9 | 1.0E-9 | 6.2E4 | 1.6E4 | 360 | 43 | 190 | 43 | 0.46 |
| Mx | pg/ml | 1.0E-9 | 1.0E-9 | 1.8E-1 | 1.6E-1 | 8.2E-1 | 5.7E-1 | 1.0E-9 | 1.0E-9 | 9.2E0 | 3.4E0 | 360 | 43 | 190 | 43 | 0.51 |
| My | pg/ml | 1.0E-9 | 1.0E-9 | 5.6E2 | 7.5E2 | 3.5E3 | 4.7E3 | 1.0E-9 | 1.0E-9 | 3.9E4 | 3.1E4 | 360 | 43 | 190 | 43 | 0.49 |
| Mz | pg/ml | 8.5E0 | 7.3E0 | 2.1E1 | 1.8E1 | 5.9E1 | 3.7E1 | 1.0E-9 | 1.0E-9 | 8.4E2 | 2.1E2 | 360 | 43 | 190 | 43 | 0.48 |
| Na | pg/ml | 1.0E-9 | 1.0E-9 | 5.7E-1 | 6.9E-1 | 1.8E0 | 1.9E0 | 1.0E-9 | 1.0E-9 | 7.8E0 | 7.2E0 | 360 | 43 | 190 | 43 | 0.53 |
| Nb | pg/ml | 1.8E0 | 1.6E0 | 4.6E0 | 4.2E0 | 1.6E1 | 1.2E1 | 1.0E-9 | 1.0E-9 | 2.3E2 | 8.1E1 | 360 | 43 | 190 | 43 | 0.47 |
| Nc | pg/ml | 4.5E2 | 1.7E2 | 6.7E2 | 4.2E2 | 8.4E2 | 5.7E2 | 1.0E-9 | 1.0E-9 | 9.2E3 | 1.8E3 | 360 | 43 | 190 | 43 | 0.39 |
| Nd | pg/ml | 3.1E1 | 9.3E0 | 2.6E1 | 1.7E1 | 2.0E1 | 1.7E1 | 1.0E-9 | 1.0E-9 | 8.4E1 | 5.5E1 | 360 | 43 | 190 | 43 | 0.36 |
| Ne | pg/ml | 4.9E2 | 2.7E2 | 6.3E2 | 3.4E2 | 6.4E2 | 3.9E2 | 1.0E-9 | 1.0E-9 | 7.0E3 | 1.5E3 | 360 | 43 | 190 | 43 | 0.34 |
| Nf | pg/ml | 1.0E-9 | 1.0E-9 | 2.9E0 | 1.8E0 | 8.8E0 | 7.0E0 | 1.0E-9 | 1.0E-9 | 8.2E1 | 4.4E1 | 360 | 43 | 190 | 43 | 0.43 |
| Ng | pg/ml | 2.1E1 | 2.6E1 | 1.1E2 | 1.0E2 | 2.3E2 | 2.0E2 | 1.0E-9 | 1.0E-9 | 1.6E3 | 1.1E3 | 360 | 43 | 190 | 43 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nh | pg/ml | 7.7E1 | 3.8E1 | 9.8E1 | 5.6E1 | 8.7E1 | 5.1E1 | 1.0E-9 | 2.5E0 | 5.6E2 | 2.2E2 | 360 | 43 | 190 | 43 | 0.34 |
| Ni | pg/ml | 4.5E0 | 1.0E-9 | 7.4E1 | 1.0E2 | 1.1E2 | 1.7E2 | 1.0E-9 | 1.0E-9 | 7.1E2 | 7.1E2 | 360 | 43 | 190 | 43 | 0.51 |
| Nj | pg/ml | 9.0E0 | 2.4E0 | 1.2E1 | 8.1E0 | 1.2E1 | 1.1E1 | 1.0E-9 | 1.0E-9 | 8.3E1 | 4.6E1 | 360 | 43 | 190 | 43 | 0.35 |
| Nk | pg/ml | 2.2E1 | 1.5E1 | 3.6E1 | 2.9E1 | 4.0E1 | 3.8E1 | 1.0E-9 | 1.0E-9 | 1.8E2 | 1.7E2 | 360 | 43 | 190 | 43 | 0.44 |
| Nl | pg/ml | 5.2E1 | 1.6E1 | 7.0E1 | 3.5E1 | 8.2E1 | 4.2E1 | 1.0E-9 | 1.0E-9 | 1.1E3 | 1.5E2 | 360 | 43 | 190 | 43 | 0.33 |
| Tz | pg/ml | 3.7E3 | 5.2E3 | 7.5E3 | 7.3E3 | 1.1E4 | 6.9E3 | 7.4E1 | 6.3E2 | 8.8E4 | 3.3E4 | 124 | 27 | 98 | 27 | 0.57 |
| Ua | pg/ml | 3.5E3 | 3.4E3 | 3.0E4 | 1.2E4 | 1.9E5 | 1.9E4 | 3.5E2 | 4.8E2 | 2.1E6 | 6.4E4 | 124 | 27 | 98 | 27 | 0.48 |
| Ub | pg/ml | 5.8E2 | 5.7E2 | 8.7E2 | 7.5E2 | 1.2E3 | 7.9E2 | 1.0E-9 | 1.0E-9 | 9.8E3 | 3.3E3 | 124 | 27 | 98 | 27 | 0.47 |
| Ue | pg/ml | 3.1E1 | 2.3E1 | 3.5E1 | 2.8E1 | 2.4E1 | 2.9E1 | 4.2E0 | 5.1E0 | 1.2E2 | 1.5E2 | 124 | 27 | 98 | 27 | 0.37 |
| Uc | pg/ml | 7.8E2 | 6.9E2 | 1.6E3 | 1.3E3 | 3.3E3 | 1.8E3 | 3.3E1 | 8.9E1 | 2.9E4 | 9.6E3 | 124 | 27 | 98 | 27 | 0.49 |
| Ud | pg/ml | 1.0E-9 | 1.0E-9 | 3.4E0 | 1.0E-9 | 3.5E1 | 0.0E0 | 1.0E-9 | 1.0E-9 | 3.9E2 | 1.0E-9 | 124 | 27 | 98 | 27 | 0.49 |
| Hq | pg/ml | 1.1E0 | 1.5E0 | 1.1E1 | 7.9E2 | 5.9E1 | 5.2E3 | 1.0E-9 | 1.0E-9 | 9.5E2 | 3.4E4 | 360 | 43 | 190 | 43 | 0.51 |
| Hr | pg/ml | 1.3E2 | 1.2E2 | 8.3E2 | 7.2E2 | 1.6E3 | 9.5E2 | 1.0E-9 | 1.0E-9 | 1.2E4 | 3.7E3 | 360 | 43 | 190 | 43 | 0.53 |
| Hu | pg/ml | 7.9E0 | 1.8E1 | 3.5E3 | 1.4E3 | 3.5E4 | 8.4E3 | 1.0E-9 | 1.0E-9 | 6.3E5 | 5.5E4 | 360 | 43 | 190 | 43 | 0.49 |
| Hv | pg/ml | 1.3E0 | 2.1E0 | 3.4E0 | 4.1E0 | 1.5E1 | 7.1E0 | 1.0E-9 | 1.0E-9 | 2.5E2 | 3.5E1 | 360 | 43 | 190 | 43 | 0.58 |
| Hw | pg/ml | 7.2E0 | 6.7E0 | 2.5E1 | 1.6E1 | 1.1E2 | 2.6E1 | 1.0E-9 | 1.0E-9 | 1.7E3 | 1.5E2 | 360 | 43 | 190 | 43 | 0.51 |
| Hx | pg/ml | 7.2E0 | 1.2E1 | 6.0E1 | 2.0E1 | 5.0E2 | 2.6E1 | 1.0E-9 | 1.0E-9 | 9.3E3 | 1.5E2 | 360 | 43 | 190 | 43 | 0.57 |
| Ib | ng/ml | 6.9E-2 | 2.7E-2 | 6.9E-1 | 1.9E0 | 3.0E0 | 6.2E0 | 1.0E-9 | 1.0E-9 | 3.0E1 | 3.0E1 | 122 | 24 | 99 | 24 | 0.44 |
| Ic | U/ml | 2.2E2 | 2.2E2 | 3.1E2 | 4.7E3 | 1.4E3 | 1.9E4 | 2.9E0 | 2.2E1 | 1.5E4 | 9.3E4 | 122 | 24 | 99 | 24 | 0.68 |
| Id | U/ml | 6.2E-1 | 7.1E-1 | 1.0E0 | 1.6E0 | 1.2E0 | 2.7E0 | 1.0E-9 | 2.7E-1 | 6.9E0 | 1.2E1 | 122 | 24 | 99 | 24 | 0.58 |
| Tt | pg/ml | 1.6E2 | 1.6E2 | 1.6E2 | 1.8E2 | 4.4E1 | 5.2E1 | 8.0E1 | 1.1E2 | 3.0E2 | 2.9E2 | 114 | 21 | 92 | 21 | 0.60 |
| To | pg/ml | 1.6E0 | 1.8E0 | 1.9E0 | 2.0E0 | 2.6E0 | 1.9E0 | 1.0E-9 | 1.0E-9 | 2.3E1 | 5.1E0 | 119 | 23 | 95 | 23 | 0.55 |
| Tr | pg/ml | 2.8E0 | 2.7E0 | 4.1E0 | 4.5E0 | 4.7E0 | 4.1E0 | 3.5E-2 | 3.9E-1 | 2.9E1 | 1.3E1 | 118 | 21 | 94 | 21 | 0.53 |
| Tn | pg/ml | 2.1E1 | 4.3E1 | 5.9E1 | 9.4E1 | 1.5E2 | 2.5E2 | 2.6E0 | 7.4E0 | 1.5E3 | 1.2E3 | 119 | 23 | 95 | 23 | 0.62 |
| Tv | ng/ml | 1.2E1 | 1.2E1 | 2.0E1 | 3.1E1 | 4.8E1 | 6.6E1 | 1.0E-9 | 1.0E-9 | 4.9E2 | 3.2E2 | 119 | 23 | 95 | 23 | 0.53 |
| Ih | ng/ml | 5.7E1 | 4.5E1 | 2.0E2 | 1.8E2 | 3.6E2 | 2.9E2 | 1.0E-9 | 1.0E-9 | 2.5E3 | 1.3E3 | 360 | 43 | 190 | 43 | 0.48 |
| Ii | ng/ml | 7.0E1 | 6.4E1 | 2.7E2 | 1.8E2 | 8.1E2 | 3.9E2 | 7.5E-1 | 7.3E-1 | 8.4E3 | 2.5E3 | 360 | 43 | 190 | 43 | 0.48 |
| Ij | ng/ml | 7.0E1 | 7.4E1 | 2.0E2 | 1.3E2 | 7.3E2 | 2.2E2 | 2.1E0 | 1.0E-9 | 6.4E3 | 1.4E3 | 358 | 43 | 190 | 43 | 0.52 |
| Ik | ng/ml | 1.2E1 | 1.6E1 | 9.2E2 | 3.0E2 | 9.2E3 | 5.2E2 | 5.9E-1 | 8.8E-1 | 1.2E5 | 2.1E3 | 358 | 43 | 190 | 43 | 0.54 |
| Il | ng/ml | 3.3E2 | 2.9E2 | 1.4E3 | 8.2E2 | 3.0E3 | 1.9E3 | 1.0E-9 | 1.0E-9 | 1.2E4 | 1.2E4 | 354 | 43 | 190 | 43 | 0.47 |
| Im | ng/ml | 1.9E2 | 1.8E2 | 3.0E2 | 3.0E2 | 3.4E2 | 3.5E2 | 1.3E1 | 3.0E1 | 3.1E3 | 1.8E3 | 357 | 43 | 190 | 43 | 0.49 |
| In | ng/ml | 4.2E0 | 2.4E0 | 3.6E1 | 7.0E0 | 2.3E2 | 1.0E1 | 1.0E-9 | 1.0E-9 | 3.9E3 | 5.2E1 | 360 | 43 | 190 | 43 | 0.43 |
| Hb | ng/ml | 2.0E1 | 2.2E1 | 2.7E1 | 3.3E1 | 2.6E1 | 3.0E1 | 1.1E0 | 1.3E0 | 1.5E2 | 1.1E2 | 120 | 26 | 98 | 26 | 0.55 |
| Hc | pg/ml | 6.1E2 | 7.6E2 | 2.2E3 | 5.0E3 | 4.9E3 | 1.9E4 | 1.0E-9 | 2.2E2 | 3.6E4 | 1.0E5 | 120 | 26 | 98 | 26 | 0.55 |
| Hf | ng/ml | 1.2E2 | 1.7E2 | 3.6E2 | 2.7E2 | 5.3E2 | 3.7E2 | 1.0E-9 | 1.0E-9 | 2.5E3 | 1.3E3 | 120 | 26 | 98 | 26 | 0.51 |
| Io | ng/ml | 6.9E3 | 5.7E3 | 1.7E4 | 8.7E3 | 5.1E4 | 8.8E3 | 6.6E1 | 2.1E2 | 8.8E5 | 3.7E4 | 357 | 42 | 189 | 42 | 0.44 |
| Ip | ng/ml | 8.1E0 | 6.3E0 | 1.8E1 | 1.8E1 | 2.7E1 | 2.5E1 | 1.0E-9 | 1.4E-2 | 2.6E2 | 1.2E2 | 357 | 42 | 189 | 42 | 0.48 |
| Iq | ug/ml | 9.1E-2 | 1.0E-1 | 3.9E1 | 3.2E2 | 7.2E2 | 2.1E3 | 1.0E-9 | 1.0E-9 | 1.4E4 | 1.4E4 | 357 | 42 | 189 | 42 | 0.51 |
| Ir | ug/ml | 2.9E-1 | 4.4E-1 | 4.4E0 | 6.4E0 | 3.3E1 | 3.4E1 | 1.0E-9 | 1.0E-9 | 5.1E2 | 2.2E2 | 356 | 42 | 189 | 42 | 0.56 |
| Is | ng/ml | 1.3E0 | 1.8E0 | 6.4E0 | 4.4E0 | 3.1E1 | 5.8E0 | 1.0E-9 | 1.0E-9 | 5.5E2 | 2.2E1 | 357 | 42 | 189 | 42 | 0.53 |
| It | ng/ml | 1.9E0 | 2.9E0 | 2.6E1 | 1.1E1 | 1.3E2 | 2.7E1 | 1.0E-9 | 1.0E-9 | 1.2E3 | 1.5E2 | 357 | 42 | 189 | 42 | 0.58 |
| Iu | ng/ml | 1.9E2 | 3.5E2 | 1.6E3 | 1.5E3 | 5.0E3 | 4.1E3 | 1.0E-9 | 1.0E-9 | 2.9E4 | 2.4E4 | 357 | 42 | 189 | 42 | 0.55 |
| Iv | ng/ml | 1.1E1 | 1.2E1 | 8.5E1 | 3.8E1 | 8.5E2 | 7.6E1 | 1.0E-9 | 1.0E-9 | 1.6E4 | 4.5E2 | 357 | 41 | 189 | 41 | 0.53 |
| Iz | ng/ml | 1.2E2 | 1.4E2 | 4.2E2 | 3.5E2 | 9.7E2 | 5.7E2 | 1.5E0 | 9.4E0 | 8.4E3 | 2.7E3 | 120 | 26 | 98 | 26 | 0.54 |
| Rc | pg/ml | 5.0E3 | 5.9E3 | 6.4E3 | 7.9E3 | 5.0E3 | 6.0E3 | 1.9E2 | 1.0E3 | 3.0E4 | 2.2E4 | 122 | 26 | 98 | 26 | 0.55 |
| Rb | pg/ml | 7.5E-1 | 1.1E0 | 2.4E0 | 3.2E0 | 3.6E0 | 8.3E0 | 1.0E-9 | 1.0E-9 | 1.6E1 | 4.3E1 | 122 | 26 | 98 | 26 | 0.56 |
| Pz | ng/ml | 3.1E3 | 6.5E3 | 6.1E3 | 5.8E3 | 9.7E3 | 4.5E3 | 1.3E1 | 9.5E1 | 9.5E4 | 1.4E4 | 358 | 43 | 189 | 43 | 0.53 |
| Qa | ng/ml | 2.7E3 | 3.6E3 | 5.9E3 | 5.5E3 | 8.0E3 | 4.9E3 | 1.5E2 | 2.9E2 | 5.2E4 | 1.9E4 | 358 | 43 | 189 | 43 | 0.56 |
| Qb | ng/ml | 8.3E1 | 1.0E2 | 2.5E2 | 1.7E2 | 7.0E2 | 2.3E2 | 7.9E-1 | 8.7E0 | 8.3E3 | 1.1E3 | 358 | 43 | 189 | 43 | 0.52 |
| Qc | ng/ml | 1.9E2 | 1.8E2 | 4.7E2 | 3.8E2 | 9.5E2 | 4.6E2 | 8.1E-1 | 5.0E0 | 1.1E4 | 1.6E3 | 358 | 43 | 189 | 43 | 0.49 |
| Qd | ng/ml | 7.9E3 | 9.5E3 | 2.2E4 | 1.7E4 | 1.1E5 | 2.6E4 | 1.5E2 | 1.0E3 | 2.0E6 | 1.5E5 | 358 | 43 | 189 | 43 | 0.52 |
| Qe | ng/ml | 6.9E2 | 1.0E3 | 1.9E3 | 1.5E3 | 5.6E3 | 1.8E3 | 1.0E-9 | 7.9E1 | 9.7E4 | 9.3E3 | 358 | 43 | 189 | 43 | 0.54 |
| Jd | ng/ml | 4.3E-1 | 6.7E-1 | 7.5E0 | 5.1E0 | 5.9E1 | 1.8E1 | 1.0E-9 | 1.0E-9 | 6.5E2 | 9.1E1 | 122 | 26 | 99 | 26 | 0.57 |

| Code | Units | Median | | Average | | Standard Deviation | | Minimum | | Maximum | | Number of Samples | | Number of Patients | | AUC |
|------|-------|--------|-----|---------|-----|------|------|--------|--------|--------|--------|--------|-----|--------|-----|------|
| | | NonDis | Dis | NonDis | Dis | NonDis | Dis | NonDis | Dis | NonDis | Dis | NonDis | Dis | NonDis | Dis | |
| Je | ng/ml | 1.0E-9 | 1.4E-1 | 1.4E0 | 4.6E0 | 5.4E0 | 1.7E1 | 1.0E-9 | 1.0E-9 | 5.6E1 | 8.8E1 | 122 | 26 | 99 | 26 | 0.56 |
| Jf | ng/ml | 1.0E-9 | 1.0E-9 | 7.4E-1 | 1.4E0 | 1.9E0 | 2.8E0 | 1.0E-9 | 1.0E-9 | 1.2E1 | 8.6E0 | 122 | 26 | 99 | 26 | 0.56 |
| Jg | ng/ml | 3.7E2 | 7.2E2 | 6.8E2 | 7.9E2 | 1.0E3 | 8.2E2 | 5.8E0 | 3.8E0 | 1.0E4 | 4.0E3 | 360 | 43 | 190 | 43 | 0.56 |
| Jh | ng/ml | 2.3E0 | 4.1E0 | 2.4E1 | 4.4E1 | 9.5E1 | 2.4E2 | 1.0E-9 | 1.0E-9 | 1.2E3 | 1.6E3 | 360 | 43 | 190 | 43 | 0.51 |
| Ji | ng/ml | 4.6E1 | 4.3E1 | 6.6E1 | 7.7E1 | 7.0E1 | 7.6E1 | 1.1E0 | 4.6E0 | 5.3E2 | 3.8E2 | 360 | 43 | 190 | 43 | 0.54 |
| Sr | pg/mL | 3.2E2 | 3.2E2 | 7.5E2 | 1.1E3 | 1.3E3 | 1.7E3 | 1.0E-9 | 1.0E-9 | 9.8E3 | 5.5E3 | 121 | 26 | 98 | 26 | 0.53 |
| Ss | pg/mL | 6.2E4 | 1.9E5 | 1.2E5 | 2.0E5 | 1.4E5 | 3.4E5 | 9.5E3 | 1.1E4 | 7.1E5 | 1.8E6 | 121 | 26 | 98 | 26 | 0.59 |
| St | pg/mL | 1.7E7 | 1.9E7 | 4.0E7 | 4.5E7 | 5.3E7 | 4.9E7 | 7.8E5 | 1.6E6 | 4.1E8 | 1.9E8 | 119 | 25 | 96 | 25 | 0.55 |
| Ra | pg/ml | 1.0E-9 | 5.2E-1 | 6.8E-1 | 9.7E-1 | 1.3E0 | 1.5E0 | 1.0E-9 | 1.0E-9 | 7.3E0 | 4.9E0 | 122 | 26 | 98 | 26 | 0.61 |
| Qz | pg/ml | 1.1E1 | 1.0E-9 | 7.1E1 | 5.8E1 | 1.1E2 | 1.2E2 | 1.0E-9 | 1.0E-9 | 7.1E2 | 4.7E2 | 122 | 26 | 98 | 26 | 0.34 |
| Qy | pg/ml | 3.9E-1 | 5.9E-1 | 3.6E0 | 2.6E1 | 1.6E1 | 1.3E2 | 1.0E-9 | 1.0E-9 | 1.6E2 | 6.5E2 | 122 | 26 | 98 | 26 | 0.60 |
| Qx | pg/ml | 1.0E-9 | 1.0E-9 | 5.6E0 | 2.4E1 | 4.9E1 | 1.1E2 | 1.0E-9 | 1.0E-9 | 5.4E2 | 5.8E2 | 122 | 26 | 98 | 26 | 0.57 |
| Qw | pg/ml | 9.0E-2 | 1.0E-9 | 2.2E0 | 3.4E-1 | 1.1E1 | 9.7E-1 | 1.0E-9 | 1.0E-9 | 1.2E2 | 4.5E0 | 122 | 26 | 98 | 26 | 0.35 |
| Qv | pg/ml | 2.8E4 | 1.3E4 | 3.9E4 | 6.2E4 | 7.4E4 | 1.8E5 | 1.2E3 | 1.6E3 | 7.4E5 | 9.4E5 | 122 | 26 | 98 | 26 | 0.40 |
| Qu | pg/ml | 1.7E0 | 2.4E1 | 8.6E1 | 5.5E1 | 1.8E2 | 1.1E2 | 1.0E-9 | 1.0E-9 | 8.0E2 | 5.1E2 | 122 | 26 | 98 | 26 | 0.55 |
| Qt | pg/ml | 9.9E0 | 1.1E1 | 3.6E1 | 6.1E1 | 7.7E1 | 1.7E2 | 1.0E-9 | 1.0E-9 | 4.1E2 | 8.5E2 | 122 | 26 | 98 | 26 | 0.51 |
| Qh | ng/ml | 1.3E1 | 1.6E1 | 3.5E1 | 3.1E1 | 7.0E1 | 3.5E1 | 1.2E-1 | 1.0E-9 | 6.4E2 | 1.5E2 | 122 | 26 | 98 | 26 | 0.57 |
| Qg | ng/ml | 9.0E0 | 4.5E0 | 1.7E1 | 1.2E1 | 2.5E1 | 1.6E1 | 1.5E-1 | 5.1E-2 | 1.8E2 | 6.3E1 | 122 | 26 | 98 | 26 | 0.39 |
| Jj | ng/ml | 7.1E2 | 5.2E2 | 2.7E3 | 1.4E3 | 1.9E4 | 3.3E3 | 2.3E0 | 1.3E1 | 3.4E5 | 1.7E4 | 360 | 43 | 190 | 43 | 0.45 |
| Jk | ng/ml | 3.0E0 | 3.1E0 | 2.1E1 | 1.7E1 | 4.6E1 | 3.6E1 | 1.0E-9 | 2.0E-1 | 2.8E2 | 1.6E2 | 360 | 43 | 190 | 43 | 0.51 |
| Jl | ng/ml | 3.4E-1 | 4.8E-1 | 1.9E0 | 1.3E0 | 4.7E0 | 3.3E0 | 7.6E-4 | 1.6E-2 | 3.2E1 | 2.0E1 | 360 | 43 | 190 | 43 | 0.51 |
| Jm | ng/ml | 1.5E1 | 2.2E1 | 5.3E1 | 3.9E1 | 1.2E2 | 5.3E1 | 1.0E-9 | 4.1E-1 | 1.4E3 | 2.4E2 | 360 | 43 | 190 | 43 | 0.54 |
| Jn | pg/ml | 3.2E-1 | 2.4E-1 | 3.2E0 | 1.1E0 | 3.3E1 | 3.1E0 | 1.0E-9 | 1.0E-9 | 6.2E2 | 2.0E1 | 360 | 42 | 190 | 42 | 0.49 |
| Jo | pg/ml | 3.5E3 | 4.3E3 | 4.8E3 | 5.2E3 | 3.9E3 | 4.6E3 | 2.0E1 | 7.7E2 | 2.0E4 | 2.0E4 | 360 | 43 | 190 | 43 | 0.52 |
| Jp | pg/ml | 6.3E4 | 7.1E4 | 6.6E4 | 7.0E4 | 3.7E4 | 3.3E4 | 5.8E2 | 5.0E3 | 3.0E5 | 1.5E5 | 360 | 43 | 190 | 43 | 0.56 |
| Jq | pg/ml | 9.1E1 | 9.2E1 | 1.4E2 | 1.6E2 | 1.8E2 | 1.8E2 | 1.0E0 | 1.3E1 | 2.0E3 | 7.9E2 | 360 | 43 | 190 | 43 | 0.52 |
| Jr | pg/ml | 3.3E0 | 4.1E0 | 4.5E1 | 1.7E1 | 5.6E2 | 6.3E1 | 1.0E-9 | 1.0E-9 | 1.1E4 | 4.1E2 | 360 | 43 | 190 | 43 | 0.50 |
| Js | pg/ml | 1.2E1 | 1.2E1 | 6.5E1 | 2.2E1 | 5.4E2 | 4.7E1 | 1.0E-9 | 6.1E-1 | 1.0E4 | 2.9E2 | 360 | 43 | 190 | 43 | 0.47 |
| Jt | pg/ml | 2.2E3 | 2.8E3 | 2.8E3 | 3.1E3 | 2.2E3 | 1.8E3 | 2.2E1 | 3.7E2 | 2.2E4 | 6.6E3 | 360 | 43 | 190 | 43 | 0.57 |
| Ju | mIU/ml | 7.2E0 | 1.4E1 | 2.0E1 | 2.2E1 | 3.4E1 | 2.7E1 | 6.5E-2 | 1.7E-1 | 2.3E2 | 1.0E2 | 122 | 26 | 99 | 26 | 0.56 |
| Jv | mIU/ml | 9.3E0 | 1.0E1 | 3.1E1 | 3.7E1 | 6.1E1 | 5.8E1 | 1.0E-2 | 9.4E-3 | 4.4E2 | 2.2E2 | 122 | 26 | 99 | 26 | 0.52 |
| Jy | ng/ml | 1.7E-3 | 1.8E-3 | 2.3E-3 | 2.5E-3 | 4.7E-3 | 4.0E-3 | 1.0E-9 | 1.0E-9 | 5.2E-2 | 2.2E-2 | 122 | 26 | 99 | 26 | 0.50 |
| Kc | pg/ml | 2.1E1 | 3.8E1 | 3.9E1 | 6.1E1 | 4.4E1 | 6.1E1 | 1.0E-9 | 1.0E-9 | 1.7E2 | 1.9E2 | 121 | 26 | 98 | 26 | 0.63 |
| Kd | pg/ml | 1.0E-9 | 1.0E-9 | 1.9E2 | 2.7E2 | 6.7E2 | 6.3E2 | 1.0E-9 | 1.0E-9 | 4.8E3 | 2.4E3 | 121 | 26 | 98 | 26 | 0.56 |
| Ke | pg/ml | 9.5E3 | 1.2E4 | 1.3E4 | 1.5E4 | 9.6E3 | 1.2E4 | 3.4E2 | 1.3E3 | 5.9E4 | 5.0E4 | 121 | 26 | 98 | 26 | 0.54 |
| Kf | pg/mL | 5.5E0 | 9.0E0 | 6.3E0 | 8.8E0 | 5.7E0 | 6.7E0 | 1.0E-9 | 1.0E-9 | 2.6E1 | 2.2E1 | 121 | 26 | 98 | 26 | 0.61 |
| Kg | pg/mL | 9.9E2 | 1.3E3 | 1.7E3 | 2.6E3 | 2.4E3 | 3.1E3 | 7.7E1 | 1.3E2 | 2.2E4 | 1.3E4 | 121 | 26 | 98 | 26 | 0.57 |
| Ki | pg/ml | 6.5E1 | 4.9E1 | 7.2E1 | 4.9E1 | 5.2E1 | 3.2E1 | 1.0E-9 | 1.0E-9 | 2.9E2 | 1.5E2 | 121 | 26 | 98 | 26 | 0.34 |
| Kj | pg/ml | 9.3E2 | 1.1E3 | 1.5E3 | 1.7E3 | 1.6E3 | 1.5E3 | 6.6E1 | 9.4E1 | 1.0E4 | 5.0E3 | 121 | 26 | 98 | 26 | 0.55 |
| Kk | pg/ml | 6.8E0 | 6.7E0 | 1.2E1 | 1.4E1 | 1.8E1 | 1.6E1 | 1.0E-9 | 1.0E-9 | 1.6E2 | 4.8E1 | 121 | 26 | 98 | 26 | 0.50 |
| Kl | pg/ml | 1.7E4 | 2.8E4 | 2.4E4 | 3.3E4 | 2.3E4 | 2.9E4 | 3.5E2 | 1.0E3 | 1.1E5 | 1.1E5 | 121 | 26 | 98 | 26 | 0.58 |
| Kn | pg/ml | 1.5E1 | 4.1E1 | 5.4E1 | 8.6E1 | 1.1E2 | 1.1E2 | 1.0E-9 | 1.0E-9 | 7.3E2 | 3.8E2 | 121 | 26 | 98 | 26 | 0.61 |
| Ko | pg/ml | 3.0E2 | 4.9E2 | 3.9E2 | 5.3E2 | 4.0E2 | 4.7E2 | 1.0E-9 | 1.0E-9 | 1.8E3 | 1.5E3 | 121 | 26 | 98 | 26 | 0.58 |
| Kp | pg/ml | 2.7E2 | 3.6E2 | 3.2E2 | 4.0E2 | 2.9E2 | 2.7E2 | 1.0E-9 | 1.0E-9 | 1.7E3 | 9.8E2 | 121 | 26 | 98 | 26 | 0.59 |
| Kq | pg/ml | 2.8E2 | 3.5E2 | 4.2E2 | 6.7E2 | 9.3E2 | 1.4E3 | 5.1E0 | 8.8E1 | 9.8E3 | 7.1E3 | 117 | 26 | 95 | 26 | 0.62 |
| Kr | pg/ml | 3.6E-1 | 1.3E-1 | 2.4E0 | 1.8E0 | 4.8E0 | 4.3E0 | 1.0E-9 | 1.0E-9 | 3.5E1 | 2.1E1 | 117 | 26 | 95 | 26 | 0.47 |
| Ks | pg/ml | 1.5E4 | 1.2E4 | 2.0E4 | 1.5E4 | 1.9E4 | 1.2E4 | 3.8E2 | 3.2E2 | 1.1E5 | 5.0E4 | 117 | 26 | 95 | 26 | 0.44 |
| Kx | ng/ml | 1.0E-9 | 1.0E-9 | 4.4E-3 | 1.0E-2 | 8.5E-3 | 2.2E-2 | 1.0E-9 | 1.0E-9 | 5.1E-2 | 1.0E-1 | 120 | 25 | 98 | 25 | 0.52 |
| Ky | ng/ml | 7.7E-2 | 1.0E-1 | 3.2E-1 | 3.8E-1 | 6.9E-1 | 1.3E0 | 1.0E-9 | 1.0E-9 | 5.1E0 | 6.4E0 | 120 | 25 | 98 | 25 | 0.55 |
| Kz | ng/ml | 1.0E-9 | 4.7E-3 | 4.7E-3 | 3.2E-3 | 6.8E-3 | 3.5E-3 | 1.0E-9 | 1.0E-9 | 3.6E-2 | 1.4E-2 | 120 | 25 | 98 | 25 | 0.51 |
| Ld | pg/ml | 1.0E-9 | 2.2E0 | 3.6E0 | 3.6E0 | 9.5E0 | 4.8E0 | 1.0E-9 | 1.0E-9 | 6.9E1 | 1.9E1 | 119 | 26 | 97 | 26 | 0.60 |
| Lh | pg/ml | 1.0E4 | 1.4E4 | 1.8E4 | 2.0E4 | 2.6E4 | 2.5E4 | 1.0E-9 | 1.8E2 | 2.6E5 | 1.5E5 | 359 | 43 | 190 | 43 | 0.56 |
| Li | pg/ml | 2.5E3 | 3.1E3 | 1.3E4 | 1.5E4 | 7.2E4 | 3.8E4 | 1.0E-9 | 5.6E1 | 1.3E6 | 2.3E5 | 359 | 43 | 190 | 43 | 0.53 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Lj | pg/ml | 1.7E3 | 2.3E3 | 1.4E4 | 1.6E4 | 5.1E4 | 3.7E4 | 1.0E-9 | 1.0E-9 | 4.4E5 | 1.7E5 | 359 | 43 | 190 | 43 | 0.53 |
| Rm | ng/ml | 1.6E1 | 1.7E1 | 5.2E1 | 4.0E1 | 8.3E1 | 5.8E1 | 2.2E-1 | 1.4E0 | 4.0E2 | 2.5E2 | 121 | 26 | 97 | 26 | 0.50 |
| Rh | ng/ml | 1.3E2 | 1.2E2 | 4.7E2 | 2.2E2 | 1.6E3 | 2.2E2 | 4.7E0 | 7.5E0 | 1.7E4 | 8.5E2 | 121 | 26 | 97 | 26 | 0.48 |
| Ri | ng/ml | 1.0E-9 | 1.0E-9 | 4.3E0 | 3.5E0 | 1.0E1 | 1.0E1 | 1.0E-9 | 1.0E-9 | 7.4E1 | 4.1E1 | 122 | 26 | 98 | 26 | 0.42 |
| Rg | ng/ml | 1.0E-9 | 1.0E-9 | 3.2E-2 | 3.3E-2 | 2.6E-1 | 1.3E-1 | 1.0E-9 | 1.0E-9 | 2.7E0 | 6.9E-1 | 121 | 26 | 97 | 26 | 0.58 |
| Rj | ng/ml | 1.0E-9 | 1.0E-9 | 1.8E0 | 1.9E-1 | 7.6E0 | 5.9E-1 | 1.0E-9 | 1.0E-9 | 8.0E1 | 2.5E0 | 122 | 26 | 98 | 26 | 0.40 |
| Rf | ng/ml | 3.4E-1 | 3.9E-1 | 8.0E-1 | 5.8E-1 | 1.5E0 | 5.6E-1 | 7.8E-3 | 2.8E-2 | 1.4E1 | 2.0E0 | 121 | 26 | 97 | 26 | 0.50 |
| Ql | pg/ml | 5.0E0 | 5.5E0 | 1.1E1 | 5.8E0 | 1.7E1 | 7.5E0 | 1.0E-9 | 1.0E-9 | 1.2E2 | 3.6E1 | 122 | 26 | 99 | 26 | 0.43 |
| Qm | pg/ml | 1.7E0 | 1.0E-9 | 2.0E1 | 1.5E1 | 4.4E1 | 2.5E1 | 1.0E-9 | 1.0E-9 | 3.6E2 | 1.1E2 | 122 | 26 | 99 | 26 | 0.48 |
| Qn | pg/ml | 6.1E-1 | 5.6E-1 | 7.2E0 | 9.3E0 | 2.4E1 | 2.3E1 | 1.0E-9 | 1.0E-9 | 2.2E2 | 1.0E2 | 122 | 26 | 99 | 26 | 0.44 |
| Nv | pg/ml | 3.0E3 | 2.6E3 | 1.1E4 | 1.9E4 | 5.8E4 | 8.1E4 | 1.0E-9 | 7.5E1 | 1.1E6 | 5.3E5 | 362 | 43 | 190 | 43 | 0.52 |
| Nw | pg/ml | 7.1E3 | 9.5E3 | 1.2E4 | 1.3E4 | 2.1E4 | 1.6E4 | 8.6E1 | 7.4E2 | 2.1E5 | 8.9E4 | 362 | 43 | 190 | 43 | 0.56 |
| Nx | pg/ml | 1.2E2 | 2.2E2 | 3.6E2 | 4.8E2 | 6.8E2 | 9.0E2 | 1.0E-9 | 1.0E-9 | 7.4E3 | 5.3E3 | 362 | 43 | 190 | 43 | 0.55 |
| Ny | pg/ml | 5.2E0 | 2.9E0 | 9.8E1 | 6.1E1 | 1.3E3 | 3.1E2 | 1.0E-9 | 1.0E-9 | 2.5E4 | 2.1E3 | 362 | 43 | 190 | 43 | 0.47 |
| Oa | pg/ml | 1.6E2 | 1.3E2 | 4.0E2 | 4.1E2 | 7.6E2 | 6.5E2 | 1.0E-9 | 1.0E-9 | 4.8E3 | 2.7E3 | 122 | 26 | 99 | 26 | 0.48 |
| Wn | ng/ml | 1.2E1 | 1.2E1 | 9.2E1 | 6.7E1 | 3.0E2 | 1.7E2 | 2.2E0 | 1.5E0 | 1.8E3 | 6.1E2 | 35 | 12 | 29 | 12 | 0.46 |
| Tk | ng/ml | 1.3E2 | 8.7E1 | 3.8E2 | 1.8E2 | 7.3E2 | 2.0E2 | 1.9E1 | 5.8E0 | 4.2E3 | 5.3E2 | 37 | 12 | 30 | 12 | 0.36 |
| Oe | pg/ml | 1.1E1 | 1.0E1 | 2.3E2 | 2.0E2 | 3.7E2 | 3.1E2 | 1.0E-9 | 1.0E-9 | 2.7E3 | 1.5E3 | 357 | 43 | 190 | 43 | 0.50 |
| Of | pg/ml | 1.6E2 | 2.1E2 | 4.0E3 | 9.4E3 | 1.5E4 | 3.4E4 | 1.0E-9 | 1.0E-9 | 1.8E5 | 1.6E5 | 360 | 43 | 190 | 43 | 0.51 |
| Og | pg/ml | 8.4E-2 | 8.4E-2 | 5.8E-1 | 1.6E-1 | 1.9E0 | 2.5E-1 | 1.0E-9 | 1.0E-9 | 2.0E1 | 1.4E0 | 360 | 43 | 190 | 43 | 0.44 |
| Oh | pg/ml | 1.9E0 | 2.7E0 | 2.2E1 | 1.2E1 | 1.2E2 | 4.1E1 | 1.0E-9 | 1.0E-9 | 1.4E3 | 2.6E2 | 360 | 43 | 190 | 43 | 0.56 |
| Oi | pg/ml | 2.0E0 | 2.4E0 | 6.3E0 | 4.9E0 | 1.1E1 | 7.7E0 | 1.0E-9 | 1.0E-9 | 8.6E1 | 4.2E1 | 360 | 43 | 190 | 43 | 0.51 |
| Ok | pg/ml | 2.9E2 | 4.9E2 | 3.9E2 | 6.7E2 | 3.7E2 | 7.0E2 | 1.3E1 | 2.7E1 | 2.8E3 | 3.1E3 | 360 | 43 | 190 | 43 | 0.62 |
| Om | pg/ml | 3.6E2 | 3.9E2 | 7.4E2 | 1.0E3 | 2.0E3 | 3.0E3 | 1.0E-9 | 3.7E1 | 3.0E4 | 2.0E4 | 360 | 43 | 190 | 43 | 0.52 |
| On | pg/ml | 1.3E2 | 1.9E2 | 2.5E2 | 3.4E2 | 4.5E2 | 5.6E2 | 1.0E-9 | 1.0E-9 | 4.5E3 | 3.3E3 | 360 | 43 | 190 | 43 | 0.57 |
| Or | pg/ml | 1.0E1 | 2.2E1 | 2.9E1 | 3.4E1 | 6.1E1 | 4.6E1 | 1.0E-9 | 1.0E-9 | 5.0E2 | 2.0E2 | 120 | 26 | 98 | 26 | 0.57 |
| Ow | pg/ml | 2.7E1 | 3.2E1 | 9.9E1 | 4.0E2 | 2.9E2 | 1.6E3 | 1.0E-9 | 1.0E-9 | 2.3E3 | 8.1E3 | 120 | 26 | 98 | 26 | 0.54 |
| Ou | pg/ml | 5.0E2 | 4.1E2 | 8.2E2 | 7.9E2 | 1.2E3 | 1.0E3 | 3.5E1 | 1.0E-9 | 9.4E3 | 4.4E3 | 120 | 26 | 98 | 26 | 0.48 |
| Ul | ng/ml | 1.0E-9 | 1.0E-9 | 1.2E0 | 7.3E-1 | 4.3E0 | 3.7E0 | 1.0E-9 | 1.0E-9 | 2.9E1 | 1.9E1 | 131 | 26 | 102 | 26 | 0.47 |
| Uo | ng/ml | 1.0E-9 | 1.0E-9 | 1.0E-1 | 1.1E-1 | 2.3E-1 | 2.0E-1 | 1.0E-9 | 1.0E-9 | 1.3E0 | 5.8E-1 | 131 | 26 | 102 | 26 | 0.50 |
| Uk | ng/ml | 1.0E-9 | 1.0E-9 | 4.7E-3 | 2.0E-2 | 1.1E-2 | 6.6E-2 | 1.0E-9 | 1.0E-9 | 9.9E-2 | 3.2E-1 | 131 | 26 | 102 | 26 | 0.50 |
| Um | ng/ml | 1.0E-9 | 1.0E-9 | 4.0E-1 | 9.3E-1 | 9.8E-1 | 1.3E0 | 1.0E-9 | 1.0E-9 | 6.8E0 | 4.0E0 | 131 | 26 | 102 | 26 | 0.57 |
| Uf | ng/ml | 4.1E-2 | 6.1E-2 | 1.0E-1 | 5.0E-1 | 1.4E-1 | 1.7E0 | 2.7E-3 | 3.6E-3 | 7.0E-1 | 8.6E0 | 131 | 26 | 102 | 26 | 0.59 |
| Uh | ng/ml | 1.6E0 | 1.9E0 | 2.7E0 | 3.4E0 | 2.7E0 | 4.4E0 | 3.2E-2 | 6.3E-2 | 1.5E1 | 2.1E1 | 131 | 26 | 102 | 26 | 0.52 |
| Un | ng/ml | 1.7E0 | 2.2E0 | 2.0E0 | 2.0E0 | 1.3E0 | 1.0E0 | 2.0E-1 | 2.6E-1 | 7.0E0 | 3.9E0 | 131 | 26 | 102 | 26 | 0.54 |
| Ug | ng/ml | 1.5E1 | 1.3E1 | 2.7E1 | 2.0E1 | 2.7E1 | 2.0E1 | 6.9E-1 | 1.8E0 | 1.3E2 | 6.8E1 | 131 | 26 | 102 | 26 | 0.41 |
| Ur | ng/ml | 1.7E-1 | 1.6E-1 | 1.2E0 | 6.2E-1 | 8.2E0 | 1.1E0 | 1.0E-9 | 1.0E-9 | 9.3E1 | 4.1E0 | 131 | 26 | 102 | 26 | 0.51 |
| Up | ng/ml | 1.0E-9 | 1.0E-9 | 6.9E-3 | 2.1E-3 | 3.2E-2 | 7.0E-3 | 1.0E-9 | 1.0E-9 | 3.3E-1 | 3.5E-2 | 131 | 26 | 102 | 26 | 0.47 |
| Us | ng/ml | 2.6E-3 | 1.0E-9 | 2.0E-2 | 3.6E-3 | 5.4E-2 | 6.7E-3 | 1.0E-9 | 1.0E-9 | 4.0E-1 | 2.9E-2 | 131 | 26 | 102 | 26 | 0.35 |
| Uv | ng/ml | 3.5E-3 | 1.7E-3 | 1.3E-2 | 9.8E-3 | 4.0E-2 | 3.1E-2 | 1.0E-9 | 1.0E-9 | 3.5E-1 | 1.6E-1 | 131 | 26 | 102 | 26 | 0.38 |
| Ut | ng/ml | 5.0E-1 | 6.5E-1 | 2.3E0 | 4.1E0 | 8.5E0 | 1.5E1 | 1.0E-9 | 1.0E-9 | 7.2E1 | 7.8E1 | 131 | 26 | 102 | 26 | 0.50 |
| Uu | ng/ml | 6.3E0 | 7.6E0 | 7.3E0 | 8.0E0 | 4.7E0 | 5.0E0 | 8.1E-1 | 9.8E-1 | 2.4E1 | 2.3E1 | 131 | 26 | 102 | 26 | 0.55 |
| Vp | ng/ml | 1.0E-9 | 1.0E-9 | 6.8E-1 | 2.1E-1 | 5.0E0 | 1.0E0 | 1.0E-9 | 1.0E-9 | 5.0E1 | 5.2E0 | 131 | 26 | 102 | 26 | 0.46 |
| Vt | ng/ml | 6.1E0 | 6.4E0 | 8.2E0 | 7.9E0 | 6.9E0 | 6.6E0 | 6.0E-1 | 7.0E-1 | 3.2E1 | 3.0E1 | 131 | 26 | 102 | 26 | 0.49 |
| Vu | ng/ml | 1.0E-9 | 1.0E-9 | 1.9E0 | 2.4E0 | 5.4E0 | 7.7E0 | 1.0E-9 | 1.0E-9 | 5.1E1 | 3.8E1 | 130 | 25 | 102 | 25 | 0.50 |
| Vq | ng/ml | 1.5E2 | 2.4E2 | 7.5E3 | 8.5E2 | 6.7E4 | 1.1E3 | 2.0E-1 | 2.6E0 | 6.8E5 | 4.3E3 | 102 | 22 | 82 | 22 | 0.61 |
| Vo | ng/ml | 2.6E1 | 2.5E1 | 2.5E1 | 2.4E1 | 4.3E0 | 3.9E0 | 1.1E1 | 8.2E0 | 3.5E1 | 2.8E1 | 131 | 26 | 102 | 26 | 0.38 |
| Vs | ng/ml | 1.0E-9 | 1.0E-9 | 6.0E0 | 1.4E1 | 2.6E1 | 4.6E1 | 1.0E-9 | 1.0E-9 | 2.3E2 | 2.2E2 | 127 | 26 | 99 | 26 | 0.53 |
| Vv | ng/ml | 2.2E0 | 3.4E0 | 5.7E0 | 4.0E0 | 1.0E1 | 3.9E0 | 1.0E-9 | 1.0E-9 | 8.1E1 | 1.4E1 | 130 | 26 | 102 | 26 | 0.51 |
| Oy | pg/ml | 5.4E-1 | 5.3E-1 | 7.9E0 | 7.6E0 | 3.9E1 | 3.8E1 | 1.0E-9 | 1.0E-9 | 4.0E2 | 2.5E2 | 360 | 43 | 190 | 43 | 0.47 |
| Oz | pg/ml | 1.4E-3 | 5.3E-2 | 2.3E-1 | 1.7E-1 | 3.8E-1 | 2.3E-1 | 1.0E-9 | 1.0E-9 | 2.6E0 | 9.0E-1 | 360 | 43 | 190 | 43 | 0.51 |
| Pa | pg/ml | 3.3E-1 | 3.2E-1 | 1.3E0 | 8.8E-1 | 5.6E0 | 2.3E0 | 1.0E-9 | 1.0E-9 | 8.6E1 | 1.4E1 | 360 | 43 | 190 | 43 | 0.50 |
| Pb | pg/ml | 1.0E-9 | 1.0E-9 | 1.6E0 | 2.0E-1 | 2.6E1 | 8.2E-1 | 1.0E-9 | 1.0E-9 | 4.9E2 | 5.3E0 | 360 | 43 | 190 | 43 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pc | pg/ml | 2.4E-2 | 1.4E-1 | 4.2E-1 | 2.9E-1 | 1.2E0 | 3.9E-1 | 1.0E-9 | 1.0E-9 | 1.6E1 | 1.8E0 | 360 | 43 | 190 | 43 | 0.54 |
| Pd | pg/ml | 1.7E0 | 1.8E0 | 3.7E0 | 3.5E0 | 8.5E0 | 5.2E0 | 1.0E-9 | 1.0E-9 | 9.4E1 | 2.4E1 | 360 | 43 | 190 | 43 | 0.51 |
| Pe | pg/ml | 1.7E1 | 2.0E1 | 8.8E1 | 9.6E1 | 3.4E2 | 1.8E2 | 1.0E-9 | 1.0E-9 | 4.7E3 | 7.3E2 | 360 | 43 | 190 | 43 | 0.54 |
| Pf | pg/ml | 1.2E0 | 1.2E0 | 8.1E0 | 1.2E1 | 3.9E1 | 3.2E1 | 1.0E-9 | 1.0E-9 | 4.8E2 | 1.8E2 | 360 | 43 | 190 | 43 | 0.54 |
| Pg | pg/ml | 2.9E0 | 2.2E0 | 3.5E1 | 8.9E1 | 1.9E2 | 5.2E2 | 1.0E-9 | 1.0E-9 | 3.2E3 | 3.4E3 | 360 | 43 | 190 | 43 | 0.47 |
| Ph | ng/ml | 1.5E-1 | 1.7E-1 | 2.6E-1 | 4.3E-1 | 3.0E-1 | 6.9E-1 | 1.0E-9 | 1.0E-9 | 1.6E0 | 2.9E0 | 120 | 26 | 98 | 26 | 0.51 |
| Pi | ng/ml | 2.0E-1 | 1.3E-1 | 2.7E-1 | 1.9E-1 | 4.1E-1 | 2.2E-1 | 1.0E-9 | 1.0E-9 | 3.2E0 | 8.6E-1 | 120 | 26 | 98 | 26 | 0.41 |
| Pj | ng/mL | 4.5E0 | 5.3E0 | 5.1E0 | 5.4E0 | 3.3E0 | 3.2E0 | 3.8E-2 | 5.1E-1 | 1.6E1 | 1.5E1 | 120 | 26 | 98 | 26 | 0.53 |
| Pk | ng/ml | 8.1E-3 | 8.9E-3 | 1.2E-2 | 1.2E-2 | 1.2E-2 | 1.2E-2 | 1.0E-9 | 1.0E-9 | 5.9E-2 | 4.6E-2 | 120 | 26 | 98 | 26 | 0.51 |
| aA | mg/dL | 8.0E-1 | 8.0E-1 | 9.1E-1 | 9.6E-1 | 4.4E-1 | 5.7E-1 | 3.0E-1 | 3.0E-1 | 4.1E0 | 3.0E0 | 1529 | 54 | 315 | 54 | 0.50 |
| aC | mg/mL | 3.2E0 | 2.3E0 | 3.4E0 | 2.5E0 | 1.5E0 | 9.8E-1 | 1.1E0 | 1.1E0 | 8.9E0 | 5.9E0 | 262 | 26 | 110 | 26 | 0.30 |
| aD | ug/mL | 3.1E0 | 3.1E0 | 4.5E0 | 4.0E0 | 4.0E0 | 3.3E0 | 4.3E-1 | 1.1E0 | 3.5E1 | 1.5E1 | 262 | 26 | 110 | 26 | 0.45 |
| aE | mg/mL | 5.8E-1 | 5.5E-1 | 5.8E-1 | 5.6E-1 | 1.4E-1 | 1.1E-1 | 2.1E-1 | 3.5E-1 | 1.1E0 | 7.8E-1 | 262 | 26 | 110 | 26 | 0.44 |
| aF | ng/mL | 2.0E0 | 2.2E0 | 4.1E0 | 4.6E0 | 6.5E0 | 6.6E0 | 4.3E-3 | 4.3E-3 | 5.0E1 | 3.0E1 | 262 | 26 | 110 | 26 | 0.52 |
| aG | mg/mL | 1.3E-1 | 1.4E-1 | 1.6E-1 | 1.5E-1 | 9.4E-2 | 6.4E-2 | 1.7E-2 | 5.6E-2 | 5.4E-1 | 3.5E-1 | 262 | 26 | 110 | 26 | 0.53 |
| aH | ug/mL | 7.0E1 | 7.7E1 | 7.8E1 | 8.6E1 | 3.8E1 | 4.7E1 | 4.6E0 | 2.5E1 | 2.7E2 | 2.3E2 | 262 | 26 | 110 | 26 | 0.54 |
| aI | ug/mL | 1.9E2 | 1.9E2 | 1.9E2 | 1.8E2 | 6.0E1 | 5.8E1 | 2.8E1 | 7.7E1 | 3.7E2 | 2.7E2 | 262 | 26 | 110 | 26 | 0.47 |
| aJ | ug/mL | 2.4E0 | 2.5E0 | 2.9E0 | 3.2E0 | 1.9E0 | 2.1E0 | 8.5E-1 | 1.0E0 | 1.2E1 | 9.5E0 | 262 | 26 | 110 | 26 | 0.54 |
| aK | ng/mL | 1.8E0 | 1.4E0 | 2.7E0 | 2.0E0 | 2.8E0 | 1.9E0 | 2.8E-2 | 1.0E-1 | 1.8E1 | 7.5E0 | 262 | 26 | 110 | 26 | 0.44 |
| aL | mg/mL | 8.3E-1 | 7.9E-1 | 8.4E-1 | 8.2E-1 | 2.7E-1 | 2.8E-1 | 1.9E-1 | 2.9E-1 | 1.7E0 | 1.4E0 | 262 | 26 | 110 | 26 | 0.47 |
| aM | U/mL | 1.9E1 | 1.9E1 | 4.2E1 | 2.9E1 | 1.1E2 | 3.3E1 | 4.2E-2 | 4.2E-2 | 1.6E3 | 1.2E2 | 262 | 26 | 110 | 26 | 0.48 |
| aN | U/mL | 9.3E0 | 1.3E1 | 1.4E1 | 2.1E1 | 1.5E1 | 2.9E1 | 2.5E-3 | 2.5E-3 | 9.8E1 | 1.3E2 | 262 | 26 | 110 | 26 | 0.57 |
| aO | pg/mL | 2.9E1 | 9.1E1 | 3.0E2 | 4.5E2 | 8.5E2 | 9.1E2 | 6.0E-2 | 6.0E-2 | 6.6E3 | 3.9E3 | 262 | 26 | 110 | 26 | 0.57 |
| aP | ng/mL | 1.7E0 | 1.7E0 | 2.1E0 | 2.2E0 | 2.1E0 | 1.3E0 | 4.5E-1 | 7.9E-1 | 2.8E1 | 5.4E0 | 262 | 26 | 110 | 26 | 0.54 |
| aQ | ng/mL | 3.2E-1 | 3.0E-1 | 4.9E-1 | 3.6E-1 | 5.0E-1 | 3.0E-1 | 1.9E-2 | 6.3E-2 | 4.0E0 | 1.3E0 | 262 | 26 | 110 | 26 | 0.43 |
| aR | ng/mL | 1.7E0 | 2.2E0 | 2.3E0 | 2.9E0 | 2.3E0 | 2.7E0 | 1.8E-1 | 3.6E-1 | 2.1E1 | 1.3E1 | 262 | 26 | 110 | 26 | 0.58 |
| aS | ng/mL | 2.4E-1 | 2.0E-1 | 6.5E-1 | 4.7E-1 | 2.3E0 | 5.3E-1 | 4.2E-3 | 4.2E-3 | 3.3E1 | 2.1E0 | 262 | 26 | 110 | 26 | 0.54 |
| aU | pg/mL | 8.7E1 | 7.0E1 | 1.4E2 | 1.0E2 | 1.6E2 | 1.2E2 | 7.4E-2 | 6.5E0 | 1.3E3 | 5.8E2 | 262 | 26 | 110 | 26 | 0.41 |
| aV | ng/mL | 7.0E-1 | 5.8E-1 | 1.1E0 | 1.1E0 | 1.2E0 | 1.4E0 | 2.2E-2 | 9.2E-2 | 8.7E0 | 6.0E0 | 262 | 26 | 110 | 26 | 0.44 |
| aW | pg/mL | 1.7E1 | 2.2E1 | 1.9E1 | 2.1E1 | 2.4E1 | 7.7E0 | 7.2E-2 | 7.2E-2 | 2.4E2 | 3.4E1 | 262 | 26 | 110 | 26 | 0.63 |
| aX | ng/mL | 1.0E1 | 7.9E0 | 1.6E1 | 2.0E1 | 2.2E1 | 4.1E1 | 3.0E-1 | 2.5E0 | 2.2E2 | 2.1E2 | 262 | 26 | 110 | 26 | 0.46 |
| aY | pg/mL | 5.3E1 | 6.0E1 | 7.3E1 | 7.7E1 | 7.0E1 | 5.8E1 | 4.1E-1 | 4.1E-1 | 4.4E2 | 2.4E2 | 262 | 26 | 110 | 26 | 0.56 |
| aZ | pg/mL | 2.3E2 | 2.8E2 | 4.4E2 | 9.8E2 | 5.6E2 | 2.4E3 | 1.7E0 | 1.7E0 | 3.4E3 | 1.2E4 | 262 | 26 | 110 | 26 | 0.53 |
| bA | ng/mL | 6.8E0 | 1.6E1 | 2.4E1 | 3.8E1 | 6.9E1 | 5.4E1 | 3.0E-2 | 3.0E-2 | 7.1E2 | 2.1E2 | 262 | 26 | 110 | 26 | 0.64 |
| bB | ng/mL | 3.1E2 | 2.9E2 | 3.4E2 | 3.1E2 | 1.6E2 | 1.7E2 | 2.1E0 | 6.6E1 | 7.4E2 | 6.9E2 | 262 | 26 | 110 | 26 | 0.44 |
| bC | ng/mL | 3.4E2 | 3.5E2 | 5.2E2 | 7.2E2 | 6.0E2 | 1.1E3 | 2.7E1 | 1.4E1 | 4.0E3 | 4.7E3 | 262 | 26 | 110 | 26 | 0.52 |
| bE | mg/mL | 5.8E0 | 5.9E0 | 6.1E0 | 6.3E0 | 2.0E0 | 2.4E0 | 9.8E-1 | 1.9E0 | 1.2E1 | 1.2E1 | 262 | 26 | 110 | 26 | 0.50 |
| bF | pg/mL | 1.9E1 | 2.9E1 | 5.7E1 | 7.5E2 | 2.6E2 | 2.3E3 | 5.0E-2 | 4.4E0 | 3.3E3 | 1.1E4 | 262 | 26 | 110 | 26 | 0.64 |
| bG | ng/mL | 1.7E0 | 1.1E0 | 2.9E0 | 2.0E0 | 3.3E0 | 1.8E0 | 2.2E-2 | 1.1E-1 | 2.3E1 | 7.3E0 | 262 | 26 | 110 | 26 | 0.43 |
| bH | pg/mL | 5.7E-1 | 5.7E-1 | 5.1E0 | 2.7E0 | 1.8E1 | 4.1E0 | 5.7E-1 | 5.7E-1 | 2.8E2 | 1.4E1 | 262 | 26 | 110 | 26 | 0.45 |
| bI | ng/mL | 4.0E-3 | 4.0E-3 | 6.0E-2 | 5.4E-2 | 1.5E-1 | 1.1E-1 | 4.0E-3 | 4.0E-3 | 1.3E0 | 3.7E-1 | 262 | 26 | 110 | 26 | 0.52 |
| bJ | mg/mL | 2.6E0 | 2.6E0 | 3.0E0 | 2.8E0 | 2.2E0 | 2.0E0 | 2.5E-4 | 2.1E-2 | 1.3E1 | 9.0E0 | 262 | 26 | 110 | 26 | 0.48 |
| bL | pg/mL | 4.1E0 | 7.1E0 | 8.5E0 | 1.1E1 | 1.1E1 | 1.1E1 | 4.6E-2 | 4.6E-2 | 8.0E1 | 4.0E1 | 262 | 26 | 110 | 26 | 0.55 |
| bM | mg/mL | 1.5E0 | 1.9E0 | 1.9E0 | 2.2E0 | 1.3E0 | 1.5E0 | 9.2E-3 | 3.3E-1 | 8.8E0 | 8.4E0 | 262 | 26 | 110 | 26 | 0.61 |
| bN | ng/mL | 3.1E1 | 5.2E1 | 1.3E2 | 1.1E2 | 3.1E2 | 1.6E2 | 1.4E-1 | 4.9E-1 | 1.9E3 | 5.7E2 | 262 | 26 | 110 | 26 | 0.56 |
| bO | ng/mL | 4.0E-2 | 4.0E-2 | 1.1E1 | 1.6E1 | 2.4E1 | 2.3E1 | 4.0E-2 | 4.0E-2 | 1.9E2 | 7.3E1 | 262 | 26 | 110 | 26 | 0.55 |
| bP | mg/mL | 6.3E-1 | 4.6E-1 | 8.5E-1 | 6.0E-1 | 7.0E-1 | 4.3E-1 | 4.9E-2 | 9.7E-2 | 3.8E0 | 1.8E0 | 262 | 26 | 110 | 26 | 0.41 |
| bQ | pg/mL | 1.5E1 | 1.7E1 | 2.7E1 | 5.2E1 | 5.0E1 | 9.8E1 | 1.5E-1 | 4.4E0 | 4.8E2 | 4.0E2 | 262 | 26 | 110 | 26 | 0.52 |
| bR | ng/mL | 1.2E-2 | 1.2E-2 | 1.3E-1 | 7.6E-2 | 2.5E-1 | 1.0E-1 | 1.2E-2 | 1.2E-2 | 3.4E0 | 3.2E-1 | 262 | 26 | 110 | 26 | 0.44 |
| bS | pg/mL | 9.4E-1 | 9.4E-1 | 7.8E0 | 4.8E0 | 2.7E1 | 1.4E1 | 9.4E-1 | 9.4E-1 | 3.4E2 | 5.5E1 | 262 | 26 | 110 | 26 | 0.46 |
| bU | ng/mL | 1.6E-1 | 9.6E-2 | 2.1E-1 | 1.1E-1 | 2.3E-1 | 1.1E-1 | 1.3E-2 | 1.3E-2 | 1.8E0 | 3.6E-1 | 262 | 26 | 110 | 26 | 0.38 |
| bV | pg/mL | 4.7E2 | 4.3E2 | 5.7E2 | 5.3E2 | 7.2E2 | 3.3E2 | 1.6E2 | 2.3E2 | 1.2E4 | 1.5E3 | 262 | 26 | 110 | 26 | 0.45 |
| bW | pg/mL | 3.5E2 | 3.3E2 | 5.2E2 | 1.2E3 | 4.8E2 | 3.9E3 | 1.1E2 | 1.0E2 | 3.4E3 | 2.0E4 | 262 | 26 | 110 | 26 | 0.50 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bX | ng/mL | 1.8E-3 | 2.5E-5 | 3.0E-3 | 1.1E-3 | 3.7E-3 | 2.1E-3 | 2.5E-5 | 2.5E-5 | 2.0E-2 | 6.9E-3 | 262 | 26 | 110 | 26 | 0.36 |
| bZ | pg/mL | 2.5E2 | 2.8E2 | 8.0E2 | 9.9E2 | 3.3E3 | 2.2E3 | 1.5E-1 | 1.0E2 | 4.4E4 | 1.2E4 | 262 | 26 | 110 | 26 | 0.54 |
| cA | pg/mL | 6.0E-1 | 6.0E-1 | 2.0E0 | 2.7E0 | 3.5E0 | 5.8E0 | 6.0E-1 | 6.0E-1 | 1.5E1 | 2.5E1 | 262 | 26 | 110 | 26 | 0.51 |
| cB | ng/mL | 6.7E-2 | 3.1E-2 | 9.8E-2 | 4.3E-2 | 1.0E-1 | 5.3E-2 | 1.7E-3 | 1.7E-3 | 5.4E-1 | 2.2E-1 | 262 | 26 | 110 | 26 | 0.30 |
| cC | pg/mL | 4.6E1 | 4.8E1 | 4.9E1 | 4.5E1 | 3.6E1 | 2.0E1 | 1.0E0 | 1.0E0 | 3.7E2 | 8.4E1 | 262 | 26 | 110 | 26 | 0.48 |
| cD | pg/mL | 6.1E0 | 3.8E0 | 1.3E1 | 2.5E1 | 5.9E1 | 6.4E1 | 3.3E-1 | 3.3E-1 | 7.2E2 | 3.1E2 | 262 | 26 | 110 | 26 | 0.44 |
| cE | pg/mL | 3.1E1 | 4.6E1 | 1.1E2 | 4.5E2 | 2.8E2 | 9.2E2 | 1.2E-1 | 1.8E0 | 3.1E3 | 3.8E3 | 262 | 26 | 110 | 26 | 0.57 |
| cF | pg/mL | 1.4E1 | 5.3E-1 | 2.3E1 | 6.7E0 | 3.2E1 | 1.1E1 | 5.3E-1 | 5.3E-1 | 2.2E2 | 4.1E1 | 262 | 26 | 110 | 26 | 0.31 |
| cG | pg/mL | 4.2E1 | 3.5E1 | 6.7E1 | 1.1E2 | 9.1E1 | 1.4E2 | 9.6E0 | 1.7E1 | 1.1E3 | 5.6E2 | 262 | 26 | 110 | 26 | 0.50 |
| cH | uIU/mL | 2.8E0 | 2.6E0 | 5.6E0 | 5.4E0 | 8.3E0 | 1.0E1 | 8.6E-3 | 8.6E-3 | 8.7E1 | 4.2E1 | 262 | 26 | 110 | 26 | 0.44 |
| cI | ng/mL | 5.5E0 | 4.3E0 | 1.1E1 | 1.2E1 | 1.4E1 | 2.1E1 | 1.0E-3 | 3.2E-2 | 1.0E2 | 1.0E2 | 262 | 26 | 110 | 26 | 0.44 |
| cJ | ug/mL | 7.0E1 | 6.9E1 | 1.2E2 | 1.3E2 | 1.4E2 | 1.6E2 | 4.0E0 | 8.6E0 | 9.6E2 | 6.6E2 | 262 | 26 | 110 | 26 | 0.51 |
| cK | ng/mL | 3.8E-3 | 3.8E-3 | 6.9E-2 | 1.1E-2 | 2.1E-1 | 2.6E-2 | 3.8E-3 | 3.8E-3 | 1.7E0 | 1.2E-1 | 262 | 26 | 110 | 26 | 0.43 |
| cL | pg/mL | 1.9E2 | 2.2E2 | 2.7E2 | 6.3E2 | 4.7E2 | 1.0E3 | 2.5E1 | 3.6E1 | 7.1E3 | 4.2E3 | 262 | 26 | 110 | 26 | 0.58 |
| cM | pg/mL | 2.8E2 | 2.4E2 | 3.1E2 | 3.0E2 | 1.8E2 | 2.1E2 | 2.5E1 | 4.7E1 | 1.2E3 | 8.4E2 | 262 | 26 | 110 | 26 | 0.46 |
| cN | pg/mL | 1.2E2 | 1.2E2 | 1.2E2 | 1.4E2 | 4.2E1 | 6.8E1 | 3.8E1 | 5.7E1 | 3.0E2 | 3.5E2 | 262 | 26 | 110 | 26 | 0.57 |
| cO | pg/mL | 2.3E2 | 2.1E2 | 2.9E2 | 2.9E2 | 2.0E2 | 2.1E2 | 5.4E1 | 9.0E1 | 1.7E3 | 1.1E3 | 262 | 26 | 110 | 26 | 0.49 |
| cP | ng/mL | 2.4E3 | 2.8E3 | 2.5E3 | 2.8E3 | 8.7E2 | 8.7E2 | 6.2E2 | 1.4E3 | 5.7E3 | 5.1E3 | 262 | 26 | 110 | 26 | 0.62 |
| cQ | ng/mL | 3.9E-2 | 5.2E-2 | 1.2E-1 | 2.0E-1 | 2.1E-1 | 4.5E-1 | 2.0E-3 | 2.0E-3 | 1.5E0 | 2.2E0 | 262 | 26 | 110 | 26 | 0.55 |
| cR | ng/mL | 3.2E2 | 3.3E2 | 5.4E2 | 5.6E2 | 8.7E2 | 9.4E2 | 2.3E1 | 9.4E1 | 8.9E3 | 4.8E3 | 262 | 26 | 110 | 26 | 0.51 |
| cS | ng/mL | 2.5E2 | 3.3E2 | 4.2E2 | 4.3E2 | 4.6E2 | 2.9E2 | 4.1E1 | 5.0E1 | 2.7E3 | 1.1E3 | 262 | 26 | 110 | 26 | 0.56 |
| cT | ng/mL | 2.7E1 | 5.8E1 | 6.4E1 | 1.1E2 | 1.3E2 | 1.9E2 | 4.6E0 | 7.6E0 | 1.7E3 | 9.9E2 | 262 | 26 | 110 | 26 | 0.64 |
| cU | ng/mL | 5.6E1 | 5.2E1 | 7.3E1 | 6.1E1 | 6.8E1 | 4.1E1 | 9.2E0 | 9.0E0 | 7.7E2 | 1.5E2 | 262 | 26 | 110 | 26 | 0.46 |
| cV | ng/mL | 1.6E-1 | 1.5E-1 | 4.4E-1 | 2.1E-1 | 2.9E0 | 2.2E-1 | 2.5E-2 | 3.0E-2 | 4.7E1 | 1.0E0 | 262 | 26 | 110 | 26 | 0.44 |
| cW | mIU/mL | 5.1E-2 | 7.6E-2 | 2.0E-1 | 8.1E-2 | 9.3E-1 | 5.3E-2 | 3.7E-4 | 2.1E-2 | 9.7E0 | 2.1E-1 | 262 | 26 | 110 | 26 | 0.60 |
| cX | ng/mL | 9.7E-2 | 2.5E-1 | 1.3E0 | 1.2E0 | 4.3E0 | 3.8E0 | 2.3E-4 | 2.3E-4 | 2.8E1 | 1.9E1 | 262 | 26 | 110 | 26 | 0.53 |
| cY | ng/mL | 9.9E0 | 6.5E0 | 1.4E1 | 1.1E1 | 1.3E1 | 1.2E1 | 2.5E-1 | 1.3E0 | 8.3E1 | 5.3E1 | 262 | 26 | 110 | 26 | 0.41 |
| cZ | ug/mL | 1.5E1 | 1.7E1 | 1.6E1 | 1.8E1 | 7.5E0 | 7.5E0 | 2.3E0 | 6.6E0 | 5.7E1 | 4.2E1 | 262 | 26 | 110 | 26 | 0.57 |
| dA | pg/mL | 3.3E2 | 3.2E2 | 3.6E2 | 3.6E2 | 1.8E2 | 1.7E2 | 9.0E1 | 1.1E2 | 1.3E3 | 9.3E2 | 262 | 26 | 110 | 26 | 0.49 |
| dB | ug/mL | 1.7E1 | 2.2E1 | 1.9E1 | 2.0E1 | 2.0E1 | 8.6E0 | 2.1E0 | 2.8E0 | 2.5E2 | 3.2E1 | 262 | 26 | 110 | 26 | 0.64 |
| dC | nmol/L | 3.5E1 | 3.7E1 | 4.0E1 | 3.7E1 | 1.9E1 | 1.2E1 | 7.9E0 | 1.7E1 | 1.4E2 | 6.5E1 | 262 | 26 | 110 | 26 | 0.49 |
| dD | ug/mL | 3.8E1 | 3.2E1 | 3.9E1 | 3.4E1 | 1.1E1 | 9.3E0 | 1.3E1 | 1.6E1 | 7.6E1 | 5.1E1 | 262 | 26 | 110 | 26 | 0.37 |
| dE | ng/mL | 5.1E-1 | 4.6E-1 | 6.9E-1 | 5.5E-1 | 8.1E-1 | 5.1E-1 | 8.4E-3 | 8.4E-3 | 7.2E0 | 2.0E0 | 262 | 26 | 110 | 26 | 0.47 |
| dF | ng/mL | 2.1E2 | 2.3E2 | 2.6E2 | 3.1E2 | 1.8E2 | 2.3E2 | 7.5E1 | 1.2E2 | 1.2E3 | 1.1E3 | 262 | 26 | 110 | 26 | 0.55 |
| dG | ng/mL | 1.1E1 | 1.1E1 | 1.4E1 | 1.7E1 | 1.0E1 | 1.6E1 | 2.5E0 | 6.0E0 | 8.1E1 | 7.6E1 | 262 | 26 | 110 | 26 | 0.55 |
| dH | pg/mL | 7.5E0 | 7.2E0 | 1.2E1 | 1.4E1 | 2.8E1 | 2.1E1 | 4.0E-2 | 4.0E-2 | 3.1E2 | 9.7E1 | 262 | 26 | 110 | 26 | 0.50 |
| dI | pg/mL | 4.6E-1 | 4.6E-1 | 1.9E0 | 1.4E0 | 4.8E0 | 1.7E0 | 4.6E-1 | 4.6E-1 | 4.2E1 | 6.0E0 | 262 | 26 | 110 | 26 | 0.54 |
| dJ | ng/mL | 1.9E0 | 2.8E0 | 2.2E0 | 2.8E0 | 1.1E0 | 1.2E0 | 3.2E-2 | 1.1E0 | 5.9E0 | 5.2E0 | 262 | 26 | 110 | 26 | 0.65 |
| dK | uIU/mL | 2.0E0 | 1.6E0 | 2.6E0 | 2.9E0 | 2.6E0 | 3.9E0 | 2.8E-4 | 9.3E-2 | 1.6E1 | 1.5E1 | 262 | 26 | 110 | 26 | 0.47 |
| dL | ng/mL | 8.9E2 | 8.5E2 | 1.0E3 | 9.5E2 | 5.0E2 | 4.1E2 | 3.4E2 | 4.1E2 | 3.4E3 | 2.2E3 | 262 | 26 | 110 | 26 | 0.48 |
| dM | pg/mL | 9.7E2 | 8.4E2 | 1.2E3 | 1.1E3 | 1.0E3 | 7.8E2 | 3.5E2 | 3.9E2 | 1.2E4 | 3.6E3 | 262 | 26 | 110 | 26 | 0.44 |
| dN | ug/mL | 9.0E1 | 1.1E2 | 9.9E1 | 1.1E2 | 3.7E1 | 4.3E1 | 2.5E1 | 5.8E1 | 2.8E2 | 2.2E2 | 262 | 26 | 110 | 26 | 0.57 |
| dR | pg/ml | 1.8E3 | 1.6E3 | 2.5E3 | 2.1E3 | 2.4E3 | 2.0E3 | 1.9E2 | 1.4E2 | 1.5E4 | 8.6E3 | 150 | 25 | 104 | 25 | 0.46 |
| eF | ng/ml | 4.0E0 | 4.2E0 | 5.2E0 | 5.1E0 | 5.6E0 | 2.4E0 | 1.2E0 | 2.1E0 | 4.6E1 | 1.2E1 | 158 | 25 | 105 | 25 | 0.60 |
| cC | pg/ml | 3.0E2 | 3.0E2 | 3.7E2 | 3.1E2 | 2.5E2 | 1.3E2 | 9.9E0 | 1.6E2 | 1.4E3 | 7.1E2 | 105 | 25 | 97 | 25 | 0.46 |
| eD | pg/ml | 2.3E2 | 2.0E2 | 7.6E2 | 3.1E2 | 1.5E3 | 3.0E2 | 5.2E-1 | 4.5E1 | 8.3E3 | 1.1E3 | 81 | 26 | 74 | 26 | 0.46 |
| eT | ng/ml | 2.8E2 | 3.6E2 | 6.0E2 | 8.2E2 | 6.5E2 | 9.7E2 | 1.0E2 | 1.7E2 | 2.5E3 | 2.6E3 | 55 | 8 | 54 | 8 | 0.60 |
| cZ | ng/ml | 5.4E1 | 4.1E1 | 6.2E1 | 5.0E1 | 2.5E1 | 2.4E1 | 2.3E1 | 3.7E1 | 1.2E2 | 1.1E2 | 55 | 8 | 54 | 8 | 0.35 |
| fN | ng/ml | 2.1E-1 | 2.1E-1 | 3.9E0 | 1.5E0 | 9.2E0 | 2.8E0 | 2.1E-1 | 2.1E-1 | 5.4E1 | 8.1E0 | 55 | 8 | 54 | 8 | 0.43 |
| fP | ng/ml | 2.3E2 | 3.4E2 | 2.5E2 | 3.7E2 | 1.4E2 | 2.2E2 | 8.4E0 | 8.1E1 | 1.0E3 | 9.5E2 | 140 | 26 | 100 | 26 | 0.67 |
| fR | ng/ml | 1.2E5 | 1.2E5 | 1.7E5 | 2.3E5 | 1.3E5 | 1.8E5 | 3.1E4 | 6.5E4 | 7.7E5 | 5.8E5 | 202 | 7 | 60 | 7 | 0.60 |
| fY | ng/ml | 2.6E2 | 2.9E2 | 2.5E2 | 2.8E2 | 9.4E1 | 1.1E2 | 6.5E1 | 1.2E2 | 4.7E2 | 4.3E2 | 55 | 8 | 54 | 8 | 0.59 |
| gL | pg/ml | 6.3E4 | 7.1E4 | 7.0E4 | 7.7E4 | 3.5E4 | 3.3E4 | 1.4E4 | 3.3E4 | 2.0E5 | 1.5E5 | 150 | 25 | 104 | 25 | 0.58 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| gP | U/ml | 2.7E2 | 3.0E2 | 2.8E2 | 2.7E2 | 9.5E1 | 7.9E1 | 8.5E1 | 8.4E1 | 8.0E2 | 4.4E2 | 157 | 25 | 105 | 25 | 0.53 |
| gW | ng/ml | 8.5E2 | 4.4E2 | 1.5E3 | 6.7E2 | 1.9E3 | 7.3E2 | 3.7E1 | 2.3E0 | 9.5E3 | 3.1E3 | 130 | 25 | 96 | 25 | 0.36 |
| tF | pg/mL | 1.6E3 | 2.6E2 | 2.0E4 | 3.4E3 | 5.4E4 | 6.9E3 | 1.2E1 | 1.8E1 | 3.2E5 | 2.9E4 | 105 | 25 | 97 | 25 | 0.38 |
| hA | ng/ml | 2.1E0 | 1.7E0 | 7.1E0 | 7.1E0 | 2.4E1 | 1.3E1 | 1.7E-2 | 1.7E-2 | 1.6E2 | 5.7E1 | 81 | 26 | 74 | 26 | 0.48 |
| nM | pg/ml | 1.0E-9 | 1.0E-9 | 1.9E2 | 1.0E-9 | 1.4E3 | 0.0E0 | 1.0E-9 | 1.0E-9 | 1.0E4 | 1.0E-9 | 53 | 18 | 51 | 18 | 0.48 |
| nN | pg/ml | 1.0E3 | 9.3E2 | 1.8E3 | 1.6E3 | 2.6E3 | 1.9E3 | 1.1E2 | 1.1E2 | 1.7E4 | 7.1E3 | 53 | 18 | 51 | 18 | 0.49 |
| nO | pg/ml | 3.2E1 | 1.7E1 | 4.9E1 | 3.3E1 | 4.9E1 | 3.7E1 | 5.5E0 | 6.5E0 | 2.4E2 | 1.4E2 | 53 | 18 | 51 | 18 | 0.32 |
| nR | pg/ml | 1.3E1 | 1.7E1 | 2.9E1 | 6.1E1 | 4.6E1 | 9.8E1 | 1.0E-9 | 8.6E-1 | 2.6E2 | 3.6E2 | 53 | 18 | 51 | 18 | 0.54 |
| nT | pg/ml | 1.1E2 | 4.2E1 | 3.7E2 | 8.4E1 | 1.2E3 | 1.2E2 | 1.0E-9 | 4.8E0 | 6.6E3 | 4.9E2 | 53 | 18 | 51 | 18 | 0.37 |
| nU | pg/ml | 1.4E1 | 6.7E1 | 5.3E2 | 1.2E2 | 2.3E3 | 1.8E2 | 1.0E-9 | 1.0E-9 | 1.3E4 | 7.5E2 | 53 | 18 | 51 | 18 | 0.67 |
| lW | pg/ml | 1.0E-9 | 1.0E-9 | 1.5E1 | 1.1E1 | 5.5E1 | 3.8E1 | 1.0E-9 | 1.0E-9 | 3.9E2 | 1.6E2 | 53 | 18 | 51 | 18 | 0.47 |
| lX | pg/ml | 1.1E3 | 1.1E3 | 1.1E3 | 1.2E3 | 5.5E2 | 4.9E2 | 2.3E2 | 3.3E2 | 2.6E3 | 2.2E3 | 53 | 18 | 51 | 18 | 0.55 |
| lY | pg/ml | 1.8E1 | 2.2E1 | 2.2E1 | 2.2E1 | 2.3E1 | 8.4E0 | 1.0E-9 | 9.7E0 | 1.4E2 | 3.8E1 | 53 | 18 | 51 | 18 | 0.60 |
| mE | pg/ml | 1.0E-9 | 7.0E-1 | 3.6E0 | 3.1E0 | 1.0E1 | 6.5E0 | 1.0E-9 | 1.0E-9 | 5.8E1 | 2.8E1 | 53 | 18 | 51 | 18 | 0.57 |
| mF | pg/ml | 1.0E-9 | 8.7E-1 | 1.8E0 | 1.6E0 | 3.3E0 | 2.2E0 | 1.0E-9 | 1.0E-9 | 1.5E1 | 6.5E0 | 53 | 18 | 51 | 18 | 0.56 |
| mH | pg/ml | 4.4E0 | 3.2E0 | 5.6E0 | 4.6E0 | 5.2E0 | 4.1E0 | 2.3E-1 | 1.4E0 | 3.2E1 | 1.8E1 | 53 | 18 | 51 | 18 | 0.45 |
| mI | pg/ml | 1.0E-9 | 1.0E-9 | 1.4E1 | 1.3E1 | 3.1E1 | 2.1E1 | 1.0E-9 | 1.0E-9 | 1.6E2 | 7.6E1 | 53 | 18 | 51 | 18 | 0.50 |
| mM | pg/ml | 1.8E1 | 2.8E1 | 4.3E1 | 8.6E1 | 5.9E1 | 1.6E2 | 1.0E-9 | 1.0E-9 | 2.3E2 | 6.5E2 | 53 | 18 | 51 | 18 | 0.58 |
| mP | pg/ml | 1.4E1 | 1.9E1 | 1.8E1 | 2.3E1 | 2.2E1 | 1.9E1 | 1.0E-9 | 1.0E-9 | 1.5E2 | 7.7E1 | 52 | 18 | 50 | 18 | 0.64 |
| mS | pg/ml | 1.8E3 | 2.0E3 | 2.1E3 | 2.1E3 | 2.3E3 | 7.3E2 | 1.0E-9 | 7.3E2 | 1.3E4 | 3.2E3 | 53 | 18 | 51 | 18 | 0.62 |
| mT | pg/ml | 5.3E1 | 7.8E1 | 1.5E2 | 1.8E2 | 2.8E2 | 2.3E2 | 9.7E0 | 1.7E1 | 1.4E3 | 8.0E2 | 52 | 18 | 50 | 18 | 0.54 |
| mU | pg/ml | 2.5E0 | 2.0E0 | 4.1E0 | 4.7E0 | 8.7E0 | 6.1E0 | 1.0E-9 | 8.2E-1 | 5.8E1 | 2.2E1 | 52 | 18 | 50 | 18 | 0.52 |
| mW | pg/ml | 2.0E3 | 3.0E3 | 2.6E3 | 3.2E3 | 1.7E3 | 2.0E3 | 3.1E2 | 8.9E2 | 1.0E4 | 9.8E3 | 52 | 18 | 50 | 18 | 0.63 |
| mY | pg/ml | 4.8E2 | 7.5E2 | 9.9E2 | 1.1E3 | 1.8E3 | 7.7E2 | 1.0E-9 | 1.5E2 | 1.1E4 | 2.6E3 | 53 | 18 | 51 | 18 | 0.68 |
| mZ | pg/ml | 1.8E2 | 1.5E2 | 3.1E2 | 2.9E2 | 2.9E2 | 3.4E2 | 1.0E-9 | 2.1E0 | 1.2E3 | 1.4E3 | 52 | 18 | 50 | 18 | 0.46 |
| nA | pg/ml | 1.3E0 | 5.5E0 | 1.6E1 | 1.1E1 | 6.7E1 | 1.7E1 | 1.0E-9 | 1.0E-9 | 4.4E2 | 5.7E1 | 52 | 18 | 50 | 18 | 0.71 |
| nB | pg/ml | 2.9E2 | 3.9E2 | 3.0E2 | 4.1E2 | 1.6E2 | 1.6E2 | 3.0E1 | 1.5E2 | 7.0E2 | 7.8E2 | 53 | 18 | 51 | 18 | 0.71 |
| nC | pg/ml | 1.0E-9 | 1.0E-9 | 8.3E3 | 1.6E3 | 5.0E4 | 4.9E3 | 1.0E-9 | 1.0E-9 | 3.7E5 | 2.0E4 | 53 | 18 | 51 | 18 | 0.49 |
| nD | pg/ml | 6.7E0 | 1.3E1 | 7.2E1 | 1.9E1 | 3.2E2 | 2.5E1 | 1.0E-9 | 1.0E-9 | 1.7E3 | 1.2E2 | 52 | 18 | 50 | 18 | 0.69 |
| nF | pg/ml | 1.0E-9 | 1.0E-9 | 7.7E0 | 7.4E0 | 3.9E1 | 1.5E1 | 1.0E-9 | 1.0E-9 | 2.4E2 | 4.7E1 | 53 | 18 | 51 | 18 | 0.62 |
| nH | pg/ml | 3.8E-1 | 1.8E0 | 2.1E2 | 2.8E1 | 1.4E3 | 8.0E1 | 1.0E-9 | 1.0E-9 | 1.0E4 | 3.3E2 | 52 | 18 | 50 | 18 | 0.58 |
| nI | pg/ml | 4.6E1 | 4.0E1 | 2.9E2 | 1.1E2 | 1.3E3 | 2.8E2 | 1.0E-9 | 1.0E-9 | 9.2E3 | 1.2E3 | 53 | 18 | 51 | 18 | 0.48 |
| nJ | pg/ml | 1.0E-9 | 1.6E0 | 1.0E2 | 2.3E0 | 7.1E2 | 3.5E0 | 1.0E-9 | 1.0E-9 | 5.2E3 | 1.5E1 | 53 | 18 | 51 | 18 | 0.76 |
| nK | pg/ml | 1.0E-9 | 2.7E1 | 1.2E2 | 3.7E1 | 5.4E2 | 5.4E1 | 1.0E-9 | 1.0E-9 | 3.2E3 | 2.2E2 | 52 | 18 | 50 | 18 | 0.64 |
| nL | pg/ml | 1.0E-9 | 1.0E-9 | 9.2E2 | 6.4E1 | 6.1E3 | 2.1E2 | 1.0E-9 | 1.0E-9 | 4.5E4 | 9.0E2 | 53 | 18 | 51 | 18 | 0.51 |
| hL | pg/ml | 1.8E4 | 1.5E4 | 2.4E4 | 2.1E4 | 2.2E4 | 2.0E4 | 1.0E-9 | 5.4E3 | 1.4E5 | 7.0E4 | 55 | 8 | 54 | 8 | 0.45 |
| hO | pg/ml | 1.6E4 | 1.5E4 | 1.7E4 | 1.5E4 | 3.2E3 | 1.7E3 | 1.1E4 | 1.3E4 | 2.8E4 | 1.8E4 | 55 | 8 | 54 | 8 | 0.35 |
| hP | ng/ml | 4.4E5 | 4.4E5 | 3.5E5 | 5.6E5 | 1.8E5 | 6.2E5 | 2.3E4 | 3.4E4 | 9.0E5 | 2.0E6 | 55 | 8 | 54 | 8 | 0.57 |
| wJ | pg/ml | 1.5E5 | 1.8E5 | 1.6E5 | 1.9E5 | 6.9E4 | 1.1E5 | 3.2E4 | 3.3E4 | 3.1E5 | 4.0E5 | 54 | 9 | 53 | 9 | 0.57 |
| wK | pg/ml | 3.4E4 | 5.4E4 | 4.2E4 | 5.6E4 | 2.5E4 | 4.2E4 | 5.2E3 | 8.1E3 | 1.1E5 | 1.3E5 | 54 | 9 | 53 | 9 | 0.61 |
| wL | pg/ml | 5.8E0 | 1.3E0 | 5.6E1 | 6.2E0 | 1.3E2 | 9.3E0 | 1.0E-9 | 1.0E-9 | 8.4E2 | 2.8E1 | 54 | 9 | 53 | 9 | 0.38 |
| wP | pg/ml | 2.8E4 | 2.9E4 | 4.2E4 | 4.6E4 | 4.0E4 | 4.2E4 | 1.1E3 | 6.3E3 | 1.6E5 | 1.4E5 | 54 | 9 | 53 | 9 | 0.54 |
| wQ | pg/ml | 3.5E1 | 6.7E1 | 6.3E1 | 7.8E1 | 7.8E1 | 6.3E1 | 1.0E-9 | 3.6E0 | 3.7E2 | 1.7E2 | 54 | 9 | 53 | 9 | 0.61 |
| hR | pg/ml | 2.9E4 | 2.4E4 | 3.0E4 | 2.5E4 | 1.2E4 | 9.3E3 | 1.8E3 | 1.1E1 | 5.8E4 | 4.3E4 | 79 | 24 | 72 | 24 | 0.38 |
| hV | pg/ml | 4.7E2 | 4.5E2 | 5.1E2 | 4.6E2 | 2.5E2 | 1.9E2 | 1.3E2 | 2.2E2 | 1.5E3 | 8.1E2 | 79 | 24 | 72 | 24 | 0.45 |
| hW | pg/ml | 1.5E3 | 1.8E3 | 1.8E3 | 2.1E3 | 9.5E2 | 1.5E3 | 5.7E2 | 7.1E2 | 4.8E3 | 7.7E3 | 79 | 24 | 72 | 24 | 0.59 |
| hX | pg/ml | 9.3E2 | 1.1E3 | 1.1E3 | 1.1E3 | 1.0E3 | 4.0E2 | 3.6E2 | 5.2E2 | 8.6E3 | 2.2E3 | 79 | 24 | 72 | 24 | 0.57 |
| iA | pg/ml | 1.4E2 | 1.4E2 | 4.0E2 | 2.0E2 | 9.0E2 | 2.1E2 | 1.1E1 | 3.0E1 | 7.1E3 | 9.5E2 | 105 | 25 | 97 | 25 | 0.48 |
| iB | ng/ml | 4.9E0 | 4.1E0 | 6.3E0 | 5.3E0 | 4.7E0 | 4.4E0 | 3.7E-2 | 3.3E-2 | 1.9E1 | 2.1E1 | 81 | 26 | 74 | 26 | 0.43 |
| iC | U/ml | 2.2E-1 | 1.8E-1 | 4.4E-1 | 2.4E0 | 8.3E-1 | 1.1E1 | 1.0E-9 | 1.0E-9 | 6.4E0 | 5.5E1 | 81 | 26 | 74 | 26 | 0.52 |
| tQ | pg/ml | 1.1E3 | 1.1E3 | 1.2E3 | 1.2E3 | 5.3E2 | 6.3E2 | 2.8E2 | 5.0E2 | 2.5E3 | 2.5E3 | 53 | 8 | 52 | 8 | 0.51 |
| tT | pg/ml | 1.7E1 | 2.1E1 | 1.8E1 | 2.0E1 | 9.7E0 | 1.2E1 | 7.4E0 | 5.4E0 | 6.9E1 | 3.9E1 | 53 | 8 | 52 | 8 | 0.57 |
| tS | pg/ml | 1.1E0 | 1.3E0 | 1.4E0 | 1.4E0 | 1.4E0 | 1.3E0 | 1.0E-9 | 1.0E-9 | 8.5E0 | 3.2E0 | 53 | 8 | 52 | 8 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| tX | pg/ml | 8.6E-1 | 6.0E-1 | 1.1E0 | 9.5E-1 | 8.6E-1 | 7.6E-1 | 2.5E-2 | 1.6E-1 | 4.4E0 | 2.1E0 | 53 | 8 | 52 | 8 | 0.45 |
| tO | pg/ml | 3.9E0 | 4.4E0 | 4.7E0 | 5.3E0 | 3.1E0 | 3.6E0 | 1.0E-9 | 1.7E0 | 1.4E1 | 1.1E1 | 53 | 8 | 52 | 8 | 0.53 |
| tR | pg/ml | 2.2E-1 | 1.9E-1 | 2.9E-1 | 2.5E-1 | 2.9E-1 | 2.2E-1 | 1.0E-9 | 5.5E-2 | 1.5E0 | 7.1E-1 | 53 | 8 | 52 | 8 | 0.46 |
| tU | pg/ml | 8.7E0 | 8.9E0 | 1.1E1 | 9.7E0 | 7.1E0 | 4.6E0 | 1.6E0 | 3.0E0 | 3.1E1 | 1.7E1 | 53 | 9 | 52 | 9 | 0.48 |
| tN | pg/ml | 1.7E1 | 1.9E1 | 2.1E1 | 2.1E1 | 1.4E1 | 1.1E1 | 1.0E-9 | 9.4E0 | 8.0E1 | 4.5E1 | 53 | 9 | 52 | 9 | 0.50 |
| tV | ng/ml | 4.2E2 | 5.4E2 | 5.3E2 | 6.7E2 | 5.0E2 | 5.0E2 | 1.5E2 | 1.4E2 | 2.9E3 | 1.8E3 | 54 | 9 | 53 | 9 | 0.59 |
| iH | ng/ml | 1.5E5 | 1.8E5 | 1.5E5 | 1.8E5 | 4.4E4 | 5.2E4 | 7.1E4 | 5.1E4 | 2.4E5 | 2.7E5 | 105 | 25 | 97 | 25 | 0.69 |
| iJ | ng/ml | 5.1E4 | 5.7E4 | 5.2E4 | 5.9E4 | 2.2E4 | 3.2E4 | 5.5E3 | 8.6E3 | 1.0E5 | 1.5E5 | 105 | 25 | 97 | 25 | 0.56 |
| hB | ng/ml | 4.4E-1 | 4.3E-1 | 5.0E-1 | 6.9E-1 | 3.2E-1 | 6.2E-1 | 1.0E-9 | 2.0E-1 | 1.7E0 | 2.4E0 | 105 | 25 | 97 | 25 | 0.54 |
| hC | pg/ml | 3.7E3 | 3.4E3 | 5.7E3 | 7.1E3 | 7.5E3 | 1.0E4 | 1.0E-9 | 6.2E1 | 5.5E4 | 4.3E4 | 105 | 25 | 97 | 25 | 0.51 |
| hF | ng/ml | 1.0E-9 | 1.0E-9 | 4.7E1 | 1.0E-9 | 4.0E2 | 0.0E0 | 1.0E-9 | 1.0E-9 | 4.0E3 | 1.0E-9 | 105 | 25 | 97 | 25 | 0.48 |
| hG | pg/ml | 7.0E3 | 6.9E3 | 7.3E3 | 8.4E3 | 3.1E3 | 3.4E3 | 2.8E1 | 4.7E3 | 1.8E4 | 1.8E4 | 105 | 25 | 97 | 25 | 0.57 |
| iO | ng/ml | 3.8E5 | 4.0E5 | 4.1E5 | 3.8E5 | 1.9E5 | 1.7E5 | 1.1E4 | 6.4E4 | 1.1E6 | 8.6E5 | 105 | 25 | 97 | 25 | 0.46 |
| iP | ng/ml | 6.0E4 | 4.6E4 | 5.5E4 | 5.3E4 | 3.2E4 | 3.5E4 | 1.0E-9 | 1.3E4 | 2.5E5 | 1.9E5 | 105 | 25 | 97 | 25 | 0.44 |
| iZ | ng/ml | 1.6E3 | 1.7E3 | 1.8E3 | 1.8E3 | 7.5E2 | 5.9E2 | 4.7E2 | 6.5E2 | 5.1E3 | 3.1E3 | 105 | 25 | 97 | 25 | 0.52 |
| yH | pg/ml | 1.1E3 | 7.5E2 | 1.9E3 | 9.8E2 | 2.9E3 | 5.8E2 | 1.0E-9 | 1.6E2 | 1.5E4 | 1.9E3 | 54 | 9 | 53 | 9 | 0.41 |
| yK | U/ml | 1.9E1 | 4.7E1 | 4.8E1 | 4.6E1 | 8.2E1 | 3.6E1 | 1.0E-9 | 4.7E-1 | 3.8E2 | 1.0E2 | 54 | 9 | 53 | 9 | 0.61 |
| yJ | pg/ml | 3.4E4 | 3.2E4 | 4.6E4 | 4.4E4 | 3.4E4 | 4.3E4 | 1.7E3 | 7.1E3 | 1.6E5 | 1.4E5 | 54 | 9 | 53 | 9 | 0.44 |
| yD | ng/ml | 1.5E-2 | 1.7E-2 | 1.5E-2 | 1.7E-2 | 6.9E-3 | 8.4E-3 | 1.0E-9 | 5.4E-3 | 4.3E-2 | 3.2E-2 | 54 | 9 | 53 | 9 | 0.55 |
| wB | pg/ml | 8.1E3 | 8.3E3 | 9.5E3 | 8.4E3 | 7.2E3 | 5.3E3 | 1.7E3 | 2.7E3 | 4.1E4 | 2.1E4 | 54 | 9 | 53 | 9 | 0.47 |
| pY | pg/ml | 6.0E0 | 7.1E0 | 1.1E1 | 6.9E0 | 2.6E1 | 2.5E0 | 2.1E0 | 3.4E0 | 2.0E2 | 1.1E1 | 55 | 8 | 54 | 8 | 0.57 |
| rC | pg/ml | 1.9E3 | 1.7E3 | 2.3E3 | 2.1E3 | 2.2E3 | 1.9E3 | 9.3E1 | 2.5E2 | 1.5E4 | 8.4E3 | 78 | 25 | 72 | 25 | 0.49 |
| rB | pg/ml | 2.2E1 | 3.0E1 | 4.6E1 | 3.5E1 | 1.2E2 | 3.1E1 | 1.0E-9 | 1.0E-9 | 9.5E2 | 1.3E2 | 78 | 25 | 72 | 25 | 0.58 |
| zG | 2.5ng/ml | 2.2E-1 | 2.5E-1 | 4.8E-1 | 7.7E-1 | 7.5E-1 | 1.5E0 | 1.0E-9 | 5.1E-2 | 4.4E0 | 4.8E0 | 54 | 9 | 53 | 9 | 0.52 |
| zH | 2.3mU/ml | 1.1E-1 | 9.9E-2 | 1.2E-1 | 1.0E-1 | 6.9E-2 | 3.5E-2 | 1.0E-2 | 4.0E-2 | 4.4E-1 | 1.6E-1 | 54 | 9 | 53 | 9 | 0.44 |
| zI | 2.6ng/ml | 1.9E0 | 2.6E0 | 3.4E0 | 4.8E0 | 3.6E0 | 5.0E0 | 6.3E-1 | 1.5E0 | 1.6E1 | 1.4E1 | 54 | 9 | 53 | 9 | 0.64 |
| qA | ng/ml | 1.1E7 | 7.3E6 | 1.3E7 | 1.5E7 | 7.4E6 | 1.2E7 | 3.7E6 | 6.9E6 | 3.7E7 | 3.9E7 | 55 | 8 | 54 | 8 | 0.47 |
| qB | ng/ml | 6.1E5 | 4.5E5 | 8.3E5 | 8.2E5 | 5.9E5 | 9.0E5 | 2.1E5 | 2.7E5 | 2.9E6 | 2.9E6 | 55 | 8 | 54 | 8 | 0.40 |
| qC | ng/ml | 4.5E5 | 3.3E5 | 9.1E5 | 3.5E5 | 1.3E6 | 3.0E5 | 2.0E4 | 2.5E4 | 7.1E6 | 7.9E5 | 55 | 8 | 54 | 8 | 0.35 |
| qD | ng/ml | 1.6E7 | 1.3E7 | 2.0E7 | 1.2E7 | 1.0E7 | 4.5E6 | 1.2E6 | 6.2E6 | 5.2E7 | 1.9E7 | 55 | 8 | 54 | 8 | 0.27 |
| jD | ng/ml | 2.0E1 | 2.7E1 | 4.1E1 | 4.9E1 | 7.0E1 | 5.0E1 | 1.0E-9 | 1.0E-9 | 5.1E2 | 1.9E2 | 81 | 26 | 74 | 26 | 0.58 |
| jE | ng/ml | 1.0E-9 | 1.0E-9 | 8.5E0 | 3.8E0 | 2.1E1 | 8.3E0 | 1.0E-9 | 1.0E-9 | 1.2E2 | 2.9E1 | 81 | 26 | 74 | 26 | 0.47 |
| jF | ng/ml | 4.9E1 | 3.0E1 | 5.8E1 | 4.5E1 | 5.8E1 | 4.9E1 | 1.0E-9 | 1.0E-9 | 2.5E2 | 1.5E2 | 81 | 26 | 74 | 26 | 0.43 |
| jG | ng/ml | 4.4E3 | 4.8E3 | 4.4E3 | 4.7E3 | 1.9E3 | 2.1E3 | 7.6E2 | 1.5E3 | 8.9E3 | 8.6E3 | 81 | 26 | 74 | 26 | 0.54 |
| jH | ng/ml | 7.6E1 | 6.9E1 | 8.6E1 | 7.3E1 | 4.9E1 | 3.2E1 | 1.9E1 | 1.3E1 | 2.8E2 | 1.6E2 | 81 | 26 | 74 | 26 | 0.43 |
| jI | ng/ml | 6.3E1 | 7.5E1 | 6.9E1 | 7.4E1 | 3.5E1 | 2.0E1 | 1.9E1 | 2.8E1 | 2.5E2 | 1.1E2 | 81 | 26 | 74 | 26 | 0.62 |
| sK | pg/mL | 3.7E3 | 4.4E3 | 4.0E3 | 4.4E3 | 1.3E3 | 2.2E3 | 1.7E3 | 1.9E3 | 8.0E3 | 8.9E3 | 54 | 9 | 53 | 9 | 0.54 |
| sM | pg/mL | 7.3E4 | 8.9E4 | 7.5E4 | 8.6E4 | 2.1E4 | 3.2E4 | 3.3E4 | 4.3E4 | 1.5E5 | 1.4E5 | 54 | 9 | 53 | 9 | 0.62 |
| sO | pg/mL | 3.0E8 | 3.0E8 | 3.0E8 | 2.5E8 | 9.2E7 | 6.9E7 | 7.9E7 | 1.4E8 | 4.9E8 | 3.3E8 | 54 | 9 | 53 | 9 | 0.36 |
| wC | ng/ml | 1.6E0 | 1.5E0 | 2.2E0 | 1.9E0 | 2.3E0 | 1.1E0 | 2.5E-1 | 9.5E-1 | 1.5E1 | 4.2E0 | 54 | 9 | 53 | 9 | 0.54 |
| wD | ng/ml | 1.6E1 | 1.2E1 | 7.3E1 | 1.7E1 | 2.9E2 | 1.4E1 | 2.1E0 | 5.1E0 | 2.1E3 | 5.2E1 | 54 | 9 | 53 | 9 | 0.45 |
| wE | ng/ml | 4.9E1 | 4.5E1 | 5.3E1 | 4.3E1 | 2.5E1 | 2.5E1 | 7.0E0 | 4.0E0 | 1.4E2 | 8.9E1 | 54 | 9 | 53 | 9 | 0.40 |
| wG | ng/ml | 8.2E-2 | 1.2E-2 | 1.2E-1 | 5.8E-2 | 1.5E-1 | 1.1E-1 | 1.0E-9 | 1.0E-9 | 7.1E-1 | 3.3E-1 | 54 | 9 | 53 | 9 | 0.36 |
| wH | ng/ml | 1.9E-2 | 6.0E-3 | 1.6E-1 | 7.7E-2 | 4.9E-1 | 1.5E-1 | 1.0E-9 | 1.0E-9 | 2.9E0 | 4.0E-1 | 54 | 9 | 53 | 9 | 0.40 |
| wF | ng/ml | 1.7E-1 | 4.2E-2 | 2.5E0 | 2.7E-1 | 9.9E0 | 3.6E-1 | 1.0E-9 | 1.0E-9 | 5.7E1 | 1.1E0 | 54 | 9 | 53 | 9 | 0.51 |
| rA | pg/ml | 2.4E1 | 2.7E1 | 3.0E1 | 3.4E1 | 2.7E1 | 2.2E1 | 1.0E-9 | 1.0E-9 | 2.0E2 | 8.2E1 | 80 | 27 | 74 | 27 | 0.58 |
| qZ | pg/ml | 4.3E1 | 4.6E1 | 1.9E2 | 7.6E2 | 1.2E3 | 2.7E3 | 2.8E-4 | 7.2E-4 | 1.0E4 | 1.0E4 | 71 | 14 | 68 | 14 | 0.52 |
| qY | pg/ml | 2.9E1 | 1.8E1 | 5.1E1 | 3.9E1 | 7.4E1 | 4.7E1 | 8.7E-1 | 2.2E0 | 5.3E2 | 1.8E2 | 80 | 27 | 74 | 27 | 0.43 |
| qX | pg/ml | 5.4E1 | 7.5E1 | 6.0E1 | 8.8E1 | 3.8E1 | 5.7E1 | 1.0E-9 | 1.0E-9 | 1.8E2 | 2.1E2 | 80 | 27 | 74 | 27 | 0.65 |
| qW | pg/ml | 9.7E0 | 1.0E1 | 1.5E1 | 1.1E1 | 2.0E1 | 7.2E0 | 1.0E-9 | 1.0E-9 | 1.2E2 | 2.3E1 | 80 | 27 | 74 | 27 | 0.48 |
| qV | pg/ml | 2.2E3 | 1.7E3 | 2.8E3 | 2.6E3 | 2.0E3 | 2.0E3 | 2.3E2 | 1.7E2 | 8.5E3 | 7.1E3 | 80 | 27 | 74 | 27 | 0.46 |
| qU | pg/ml | 5.5E1 | 6.5E1 | 1.7E2 | 9.2E1 | 2.6E2 | 7.6E1 | 1.0E-9 | 1.0E1 | 1.4E3 | 2.8E2 | 80 | 27 | 74 | 27 | 0.52 |
| qT | pg/ml | 3.7E1 | 5.5E1 | 6.5E1 | 8.0E1 | 1.2E2 | 8.3E1 | 1.0E-9 | 5.6E0 | 9.0E2 | 4.4E2 | 80 | 27 | 74 | 27 | 0.65 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| qI | ng/ml | 5.4E4 | 6.9E4 | 6.2E4 | 6.1E4 | 3.1E4 | 2.7E4 | 1.1E4 | 2.5E4 | 1.6E5 | 9.8E4 | 54 | 9 | 54 | 9 | 0.50 |
| qH | ng/ml | 6.6E4 | 6.6E4 | 7.2E4 | 6.9E4 | 3.8E4 | 4.5E4 | 1.5E4 | 2.3E4 | 1.8E5 | 1.6E5 | 54 | 9 | 54 | 9 | 0.44 |
| qG | ng/ml | 1.8E5 | 2.0E5 | 1.9E5 | 2.2E5 | 5.9E4 | 5.6E4 | 5.8E4 | 1.5E5 | 3.3E5 | 2.9E5 | 54 | 9 | 54 | 9 | 0.61 |
| jK | ng/ml | 1.6E3 | 1.9E3 | 1.7E3 | 1.8E3 | 5.3E2 | 4.8E2 | 5.5E2 | 7.6E2 | 3.5E3 | 2.8E3 | 81 | 26 | 74 | 26 | 0.58 |
| jL | ng/ml | 1.7E2 | 2.0E2 | 2.5E2 | 3.3E2 | 1.9E2 | 3.5E2 | 3.6E1 | 8.8E1 | 9.6E2 | 1.7E3 | 81 | 26 | 74 | 26 | 0.55 |
| jM | ng/ml | 7.1E4 | 7.5E4 | 7.3E4 | 8.3E4 | 4.0E4 | 3.9E4 | 3.9E2 | 2.4E4 | 1.9E5 | 1.6E5 | 81 | 26 | 74 | 26 | 0.57 |
| jO | pg/ml | 2.1E5 | 2.0E5 | 2.7E5 | 2.7E5 | 1.7E5 | 1.8E5 | 5.2E4 | 7.4E4 | 1.1E6 | 8.0E5 | 81 | 26 | 74 | 26 | 0.49 |
| jP | pg/ml | 2.2E5 | 2.3E5 | 2.5E5 | 2.6E5 | 1.5E5 | 1.7E5 | 3.6E4 | 6.6E4 | 9.1E5 | 9.2E5 | 81 | 26 | 74 | 26 | 0.50 |
| jQ | pg/ml | 2.8E3 | 3.0E3 | 3.7E3 | 3.4E3 | 3.1E3 | 2.4E3 | 4.2E1 | 2.6E2 | 1.3E4 | 1.0E4 | 81 | 26 | 74 | 26 | 0.49 |
| jR | pg/ml | 8.6E3 | 7.8E3 | 1.3E4 | 1.0E4 | 1.3E4 | 9.4E3 | 1.0E-9 | 7.2E1 | 6.8E4 | 3.4E4 | 81 | 26 | 74 | 26 | 0.47 |
| jT | pg/ml | 1.7E5 | 1.9E5 | 1.7E5 | 1.9E5 | 6.3E4 | 5.7E4 | 6.8E4 | 9.0E4 | 3.9E5 | 3.1E5 | 81 | 26 | 74 | 26 | 0.59 |
| xA | pg/ml | 3.9E0 | 1.0E1 | 1.5E1 | 2.8E1 | 5.4E1 | 4.6E1 | 1.0E-9 | 1.0E-9 | 3.9E2 | 1.4E2 | 54 | 9 | 53 | 9 | 0.62 |
| yE | pg/ml | 7.9E1 | 7.0E1 | 8.3E1 | 8.7E1 | 4.4E1 | 4.0E1 | 1.8E1 | 4.0E1 | 3.0E2 | 1.6E2 | 54 | 9 | 53 | 9 | 0.53 |
| tM | pg/ml | 3.9E1 | 3.3E1 | 4.2E1 | 3.6E1 | 2.1E1 | 2.0E1 | 1.0E-9 | 1.0E-9 | 1.0E2 | 6.1E1 | 54 | 9 | 53 | 9 | 0.43 |
| tL | pg/ml | 1.0E-9 | 1.0E-9 | 6.9E0 | 1.5E-1 | 3.6E1 | 4.6E-1 | 1.0E-9 | 1.0E-9 | 2.6E2 | 1.4E0 | 54 | 9 | 53 | 9 | 0.43 |
| jU | mIU/ml | 3.5E0 | 6.4E0 | 1.0E1 | 1.3E1 | 1.8E1 | 1.7E1 | 8.9E-2 | 6.3E-2 | 8.1E1 | 7.5E1 | 81 | 26 | 74 | 26 | 0.61 |
| jV | mIU/ml | 1.5E0 | 2.1E0 | 3.4E0 | 4.9E0 | 5.5E0 | 7.8E0 | 3.4E-2 | 1.1E-3 | 3.1E1 | 3.3E1 | 81 | 26 | 74 | 26 | 0.52 |
| jY | ng/ml | 7.4E-4 | 7.5E-4 | 9.8E-3 | 6.7E-3 | 4.1E-2 | 1.9E-2 | 1.0E-9 | 1.0E-9 | 3.0E-1 | 9.4E-2 | 81 | 26 | 74 | 26 | 0.50 |
| kC | pg/ml | 9.7E1 | 1.2E2 | 2.3E2 | 1.2E2 | 5.6E2 | 6.4E1 | 2.9E1 | 3.6E1 | 3.5E3 | 2.9E2 | 53 | 18 | 51 | 18 | 0.53 |
| kE | pg/ml | 1.2E5 | 1.4E5 | 1.3E5 | 1.3E5 | 3.2E4 | 3.8E4 | 4.1E4 | 3.8E4 | 2.0E5 | 2.0E5 | 53 | 18 | 51 | 18 | 0.53 |
| kF | pg/mL | 6.0E1 | 5.6E1 | 7.4E1 | 6.7E1 | 7.1E1 | 2.5E1 | 2.7E1 | 3.8E1 | 5.1E2 | 1.4E2 | 53 | 18 | 51 | 18 | 0.52 |
| kG | pg/mL | 8.5E3 | 1.0E4 | 9.4E3 | 2.1E4 | 7.8E3 | 3.0E4 | 7.5E2 | 3.8E3 | 4.3E4 | 1.2E5 | 53 | 18 | 51 | 18 | 0.60 |
| kI | pg/ml | 2.0E2 | 1.7E2 | 2.4E2 | 2.1E2 | 1.5E2 | 1.3E2 | 7.9E1 | 8.8E1 | 8.7E2 | 5.5E2 | 53 | 18 | 51 | 18 | 0.40 |
| kK | pg/ml | 1.0E2 | 1.1E2 | 1.6E2 | 2.0E2 | 1.9E2 | 2.7E2 | 6.4E0 | 4.5E1 | 1.2E3 | 1.2E3 | 53 | 18 | 51 | 18 | 0.53 |
| kN | pg/ml | 9.2E2 | 9.8E2 | 1.4E3 | 1.3E3 | 1.9E3 | 1.1E3 | 2.4E2 | 2.4E2 | 1.3E4 | 3.9E3 | 53 | 18 | 51 | 18 | 0.52 |
| kO | pg/ml | 7.7E3 | 6.1E3 | 1.0E4 | 6.9E3 | 1.8E4 | 2.6E3 | 3.7E3 | 3.8E3 | 1.3E5 | 1.4E4 | 53 | 18 | 51 | 18 | 0.34 |
| kP | pg/ml | 4.8E3 | 6.2E3 | 6.8E3 | 6.5E3 | 6.1E3 | 2.7E3 | 8.6E2 | 2.7E3 | 3.3E4 | 1.2E4 | 53 | 18 | 51 | 18 | 0.58 |
| kQ | pg/ml | 4.2E3 | 3.8E3 | 4.9E3 | 4.4E3 | 2.6E3 | 2.1E3 | 5.6E2 | 1.6E3 | 1.4E4 | 9.3E3 | 105 | 25 | 97 | 25 | 0.43 |
| kR | pg/ml | 2.0E1 | 2.1E1 | 3.6E1 | 2.7E1 | 1.0E2 | 1.8E1 | 1.0E-9 | 5.1E0 | 1.0E3 | 6.9E1 | 105 | 25 | 97 | 25 | 0.53 |
| kS | pg/ml | 7.6E2 | 8.2E2 | 8.9E2 | 8.2E2 | 5.2E2 | 3.4E2 | 1.3E2 | 2.5E2 | 3.2E3 | 1.7E3 | 105 | 25 | 97 | 25 | 0.51 |
| pS | ng/ml | 1.8E5 | 2.1E5 | 2.1E5 | 2.4E5 | 9.0E4 | 1.4E5 | 9.7E4 | 1.0E5 | 5.0E5 | 5.7E5 | 54 | 9 | 53 | 9 | 0.56 |
| rZ | ng/ml | 1.0E-9 | 1.0E-9 | 7.1E-3 | 1.4E-2 | 2.0E-2 | 6.0E-2 | 1.0E-9 | 1.0E-9 | 1.1E-1 | 3.0E-1 | 78 | 25 | 71 | 25 | 0.48 |
| rY | ng/ml | 5.3E-2 | 5.4E-2 | 2.2E-1 | 7.8E-1 | 8.1E-1 | 3.6E0 | 1.0E-9 | 1.0E-9 | 6.3E0 | 1.8E1 | 78 | 25 | 71 | 25 | 0.46 |
| rX | ng/ml | 1.0E-9 | 1.0E-9 | 8.4E-2 | 1.1E-1 | 4.4E-1 | 4.8E-1 | 1.0E-9 | 1.0E-9 | 3.7E0 | 2.4E0 | 78 | 25 | 71 | 25 | 0.44 |
| lK | pg/ml | 7.5E1 | 6.6E1 | 1.6E2 | 1.7E2 | 1.9E2 | 3.0E2 | 1.0E-9 | 1.0E-9 | 7.4E2 | 1.3E3 | 81 | 26 | 74 | 26 | 0.45 |
| lL | pg/ml | 1.7E3 | 1.6E3 | 2.7E3 | 2.1E3 | 3.0E3 | 1.8E3 | 7.5E1 | 4.5E2 | 1.9E4 | 6.8E3 | 81 | 26 | 74 | 26 | 0.45 |
| lM | pg/ml | 1.1E3 | 1.3E3 | 2.2E3 | 6.9E3 | 3.0E3 | 1.2E4 | 1.3E2 | 2.7E2 | 1.6E4 | 4.4E4 | 81 | 26 | 74 | 26 | 0.56 |
| lN | pg/ml | 1.0E-9 | 1.0E-9 | 6.9E0 | 1.5E0 | 2.2E1 | 2.9E0 | 1.0E-9 | 1.0E-9 | 1.5E2 | 1.2E1 | 81 | 26 | 74 | 26 | 0.41 |
| lO | pg/ml | 1.0E-9 | 1.0E-9 | 5.0E-1 | 1.0E0 | 4.5E0 | 3.6E0 | 1.0E-9 | 1.0E-9 | 4.0E1 | 1.3E1 | 81 | 26 | 74 | 26 | 0.53 |
| zA | ng/ml | 1.9E7 | 1.9E7 | 1.9E7 | 1.9E7 | 6.6E6 | 4.0E6 | 6.7E6 | 1.1E7 | 3.4E7 | 2.6E7 | 50 | 9 | 49 | 9 | 0.49 |
| rW | ng/ml | 1.7E-2 | 8.7E-3 | 2.7E-2 | 3.3E-2 | 3.2E-2 | 5.2E-2 | 1.0E-9 | 1.0E-9 | 1.7E-1 | 1.6E-1 | 54 | 10 | 54 | 10 | 0.47 |
| rV | ng/ml | 1.0E-9 | 1.0E-9 | 1.2E-2 | 2.9E-2 | 4.0E-2 | 8.9E-2 | 1.0E-9 | 1.0E-9 | 2.2E-1 | 2.8E-1 | 54 | 10 | 54 | 10 | 0.49 |
| rU | ng/ml | 1.1E-1 | 2.0E-2 | 1.8E-1 | 6.9E-1 | 2.7E-1 | 1.5E0 | 1.0E-9 | 1.0E-9 | 1.4E0 | 4.4E0 | 54 | 10 | 54 | 10 | 0.38 |
| rT | ng/ml | 6.5E0 | 4.8E0 | 6.8E0 | 5.2E0 | 4.2E0 | 2.5E0 | 7.3E-1 | 1.3E0 | 2.1E1 | 1.0E1 | 54 | 10 | 54 | 10 | 0.38 |
| rS | ng/ml | 3.5E0 | 5.1E0 | 5.8E0 | 5.5E0 | 6.6E0 | 3.3E0 | 7.6E-1 | 1.5E0 | 3.8E1 | 1.3E1 | 54 | 10 | 54 | 10 | 0.60 |
| sC | pg/mL | 5.8E3 | 8.2E3 | 9.5E3 | 1.2E4 | 8.6E3 | 1.2E4 | 1.7E3 | 2.3E3 | 4.4E4 | 3.9E4 | 54 | 9 | 53 | 9 | 0.55 |
| yL | pg/ml | 3.2E1 | 3.0E1 | 3.9E1 | 2.8E1 | 2.8E1 | 8.4E0 | 5.6E0 | 1.1E1 | 1.8E2 | 3.8E1 | 52 | 9 | 51 | 9 | 0.37 |
| rP | ng/ml | 9.5E1 | 1.1E2 | 1.6E2 | 2.0E2 | 2.2E2 | 2.1E2 | 1.0E-9 | 3.7E0 | 1.2E3 | 5.0E2 | 54 | 10 | 54 | 10 | 0.56 |
| rQ | ng/ml | 1.0E-9 | 1.0E-9 | 3.6E0 | 1.7E1 | 1.6E1 | 5.3E1 | 1.0E-9 | 1.0E-9 | 8.9E1 | 1.7E2 | 54 | 10 | 54 | 10 | 0.51 |
| rO | ng/ml | 2.5E-2 | 3.0E-2 | 4.8E-2 | 8.6E-2 | 8.5E-2 | 1.4E-1 | 1.0E-9 | 1.0E-9 | 4.4E-1 | 4.7E-1 | 54 | 10 | 54 | 10 | 0.55 |
| rR | ng/ml | 3.9E0 | 2.0E0 | 2.3E1 | 5.8E0 | 6.8E1 | 1.1E1 | 1.0E-9 | 1.0E-9 | 4.5E2 | 3.6E1 | 54 | 10 | 54 | 10 | 0.40 |
| rN | ng/ml | 6.6E-1 | 6.4E-1 | 7.4E-1 | 8.1E-1 | 4.4E-1 | 6.1E-1 | 5.1E-2 | 2.7E-1 | 2.1E0 | 2.3E0 | 54 | 10 | 54 | 10 | 0.51 |
| qO | pg/ml | 9.8E3 | 1.1E4 | 1.3E4 | 1.5E4 | 9.6E3 | 1.5E4 | 2.2E3 | 7.4E2 | 4.6E4 | 5.0E4 | 55 | 9 | 54 | 9 | 0.49 |

| Code | Units | Median | | Average | | Standard Deviation | | Minimum | | Maximum | | Number of Samples | | Number of Patients | | AUC |
|------|-------|--------|-----|---------|-----|---------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| qP | pg/ml | 3.6E2 | 3.6E2 | 4.4E2 | 5.1E2 | 3.0E2 | 5.5E2 | 1.0E-9 | 1.1E2 | 1.5E3 | 1.9E3 | 55 | 9 | 54 | 9 | 0.46 |
| qQ | pg/ml | 1.5E1 | 1.0E-9 | 1.7E1 | 7.3E0 | 3.8E1 | 8.7E0 | 1.0E-9 | 1.0E-9 | 2.8E2 | 1.9E1 | 55 | 9 | 54 | 9 | 0.38 |
| nW | pg/ml | 1.1E5 | 1.1E5 | 1.1E5 | 1.2E5 | 2.4E4 | 2.8E4 | 5.8E4 | 6.9E4 | 1.8E5 | 1.6E5 | 105 | 25 | 97 | 25 | 0.52 |
| nY | pg/ml | 1.9E3 | 1.6E3 | 2.2E3 | 2.1E3 | 1.4E3 | 1.3E3 | 6.5E2 | 6.6E2 | 9.9E3 | 5.3E3 | 105 | 25 | 97 | 25 | 0.47 |
| oO | pg/ml | 7.5E4 | 9.9E4 | 1.0E5 | 1.3E5 | 1.1E5 | 9.2E4 | 1.5E4 | 2.6E4 | 6.2E5 | 2.8E5 | 51 | 16 | 49 | 16 | 0.62 |
| oP | pg/ml | 1.1E5 | 1.7E5 | 1.3E5 | 1.8E5 | 7.7E4 | 9.7E4 | 2.4E4 | 6.3E4 | 3.6E5 | 3.6E5 | 51 | 16 | 49 | 16 | 0.67 |
| oQ | pg/ml | 2.5E3 | 3.0E3 | 2.9E3 | 4.6E3 | 1.7E3 | 3.3E3 | 9.3E2 | 1.6E3 | 1.0E4 | 1.2E4 | 51 | 16 | 49 | 16 | 0.65 |
| oE | pg/ml | 1.8E2 | 7.1E1 | 4.0E2 | 3.0E2 | 6.0E2 | 5.9E2 | 1.0E-9 | 1.0E-9 | 4.7E3 | 2.8E3 | 105 | 25 | 97 | 25 | 0.39 |
| oF | pg/ml | 7.4E3 | 9.4E3 | 1.7E4 | 2.7E4 | 3.0E4 | 3.7E4 | 6.4E1 | 6.5E2 | 1.7E5 | 1.4E5 | 105 | 25 | 97 | 25 | 0.58 |
| oH | pg/ml | 4.4E1 | 3.9E1 | 9.5E1 | 1.0E2 | 1.5E2 | 1.9E2 | 4.2E0 | 4.3E-1 | 8.6E2 | 9.9E2 | 105 | 25 | 97 | 25 | 0.50 |
| oK | pg/ml | 6.4E2 | 9.0E2 | 2.0E3 | 1.9E3 | 3.1E3 | 3.2E3 | 5.2E1 | 1.8E2 | 1.8E4 | 1.2E4 | 105 | 25 | 97 | 25 | 0.50 |
| oN | pg/ml | 4.9E2 | 5.3E2 | 7.8E2 | 8.6E2 | 1.8E3 | 9.1E2 | 1.5E2 | 2.6E2 | 1.8E4 | 3.7E3 | 105 | 25 | 97 | 25 | 0.58 |
| uL | ng/ml | 3.6E1 | 3.9E1 | 4.4E1 | 7.1E1 | 3.0E1 | 1.0E2 | 1.0E-9 | 2.4E1 | 1.6E2 | 3.4E2 | 52 | 9 | 51 | 9 | 0.54 |
| uO | ng/ml | 3.0E-1 | 1.8E-1 | 7.8E-1 | 5.7E-1 | 1.4E0 | 6.9E-1 | 1.0E-9 | 1.0E-9 | 9.3E0 | 2.0E0 | 52 | 9 | 51 | 9 | 0.49 |
| uM | ng/ml | 6.4E-1 | 5.9E-1 | 1.1E0 | 7.9E-1 | 2.2E0 | 3.6E-1 | 1.0E-9 | 4.7E-1 | 1.3E1 | 1.6E0 | 52 | 9 | 51 | 9 | 0.56 |
| uI | ng/ml | 7.7E-2 | 5.8E-2 | 1.4E-1 | 1.0E-1 | 1.9E-1 | 1.1E-1 | 1.6E-2 | 3.6E-2 | 1.1E0 | 3.9E-1 | 52 | 9 | 51 | 9 | 0.43 |
| uN | ng/ml | 1.5E1 | 1.5E1 | 1.7E1 | 1.7E1 | 6.7E0 | 6.7E0 | 7.7E0 | 8.1E0 | 4.1E1 | 3.0E1 | 52 | 9 | 51 | 9 | 0.50 |
| uG | ng/ml | 2.1E1 | 1.7E1 | 2.5E1 | 2.7E1 | 1.4E1 | 2.4E1 | 7.6E0 | 6.1E0 | 6.9E1 | 7.9E1 | 52 | 9 | 51 | 9 | 0.46 |
| uR | ng/ml | 2.3E0 | 2.7E0 | 3.9E0 | 2.8E0 | 8.6E0 | 1.3E0 | 9.9E-1 | 8.9E-1 | 6.4E1 | 5.5E0 | 54 | 9 | 53 | 9 | 0.58 |
| uP | ng/ml | 2.2E0 | 2.7E0 | 2.5E0 | 2.9E0 | 1.3E0 | 1.3E0 | 1.1E0 | 1.6E0 | 9.1E0 | 6.0E0 | 54 | 9 | 53 | 9 | 0.64 |
| uV | ng/ml | 2.3E-4 | 1.0E-9 | 1.6E-2 | 7.1E-3 | 4.0E-2 | 1.1E-2 | 1.0E-9 | 1.0E-9 | 2.0E-1 | 2.5E-2 | 54 | 9 | 53 | 9 | 0.45 |
| uT | ng/ml | 5.8E1 | 7.0E1 | 8.3E1 | 8.0E1 | 9.2E1 | 4.5E1 | 1.2E1 | 3.3E1 | 5.8E2 | 1.6E2 | 54 | 9 | 53 | 9 | 0.57 |
| uU | ng/ml | 1.7E0 | 1.4E0 | 2.0E0 | 2.3E0 | 1.2E0 | 1.9E0 | 5.2E-1 | 7.5E-1 | 5.6E0 | 6.0E0 | 54 | 9 | 53 | 9 | 0.46 |
| uW | ng/ml | 7.2E0 | 8.4E0 | 7.6E0 | 9.1E0 | 2.9E0 | 2.3E0 | 4.0E0 | 5.6E0 | 2.2E1 | 1.3E1 | 52 | 9 | 51 | 9 | 0.72 |
| vB | ng/ml | 2.6E0 | 3.3E0 | 2.7E0 | 3.8E0 | 1.2E0 | 1.6E0 | 6.9E-1 | 1.5E0 | 5.6E0 | 6.6E0 | 52 | 9 | 51 | 9 | 0.70 |
| vC | ng/ml | 1.0E-9 | 1.0E-9 | 3.0E-3 | 1.0E-9 | 2.0E-2 | 0.0E0 | 1.0E-9 | 1.0E-9 | 1.4E-1 | 1.0E-9 | 52 | 9 | 51 | 9 | 0.48 |
| uY | ng/ml | 7.4E-1 | 6.1E-1 | 1.2E0 | 1.1E0 | 1.1E0 | 1.0E0 | 8.7E-2 | 2.4E-1 | 4.9E0 | 3.0E0 | 52 | 9 | 51 | 9 | 0.46 |
| uZ | ng/ml | 5.8E-1 | 6.0E-1 | 8.2E-1 | 7.2E-1 | 1.1E0 | 5.6E-1 | 4.7E-2 | 2.7E-1 | 7.2E0 | 2.1E0 | 52 | 9 | 51 | 9 | 0.51 |
| uX | ng/ml | 1.1E1 | 1.5E1 | 1.3E1 | 1.5E1 | 7.2E0 | 5.5E0 | 4.0E0 | 7.5E0 | 3.3E1 | 2.3E1 | 52 | 9 | 51 | 9 | 0.64 |
| vA | ng/ml | 7.4E-2 | 7.2E-2 | 8.6E-2 | 7.6E-2 | 5.8E-2 | 3.3E-2 | 2.4E-2 | 3.0E-2 | 3.0E-1 | 1.4E-1 | 52 | 9 | 51 | 9 | 0.49 |
| vH | ng/ml | 1.2E-1 | 1.5E-1 | 1.6E-1 | 1.6E-1 | 1.4E-1 | 7.4E-2 | 1.5E-2 | 5.8E-2 | 8.0E-1 | 2.6E-1 | 54 | 9 | 53 | 9 | 0.60 |
| vI | ng/ml | 1.4E0 | 2.0E0 | 1.7E0 | 2.6E0 | 1.1E0 | 2.0E0 | 6.2E-3 | 1.2E-2 | 4.5E0 | 6.2E0 | 54 | 9 | 53 | 9 | 0.63 |
| vP | ng/ml | 4.3E2 | 4.4E2 | 5.1E2 | 5.4E2 | 3.9E2 | 5.1E2 | 7.0E1 | 6.7E1 | 2.0E3 | 1.7E3 | 54 | 9 | 53 | 9 | 0.48 |
| vT | ng/ml | 7.7E1 | 9.9E1 | 1.1E2 | 1.1E2 | 1.2E2 | 7.6E1 | 3.7E1 | 3.0E1 | 6.9E2 | 2.8E2 | 54 | 9 | 53 | 9 | 0.50 |
| vU | ng/ml | 1.0E-9 | 1.0E-9 | 2.3E1 | 3.3E1 | 3.5E1 | 4.3E1 | 1.0E-9 | 1.0E-9 | 1.3E2 | 1.2E2 | 54 | 9 | 53 | 9 | 0.56 |
| vQ | ng/ml | 3.5E2 | 3.5E2 | 3.6E2 | 3.9E2 | 1.5E2 | 1.9E2 | 6.7E1 | 2.1E2 | 8.1E2 | 8.4E2 | 54 | 9 | 53 | 9 | 0.50 |
| vO | ng/ml | 1.7E3 | 2.0E3 | 1.8E3 | 2.0E3 | 4.5E2 | 4.7E2 | 1.0E3 | 1.2E3 | 3.0E3 | 2.8E3 | 54 | 9 | 53 | 9 | 0.63 |
| vS | ng/ml | 1.3E3 | 1.5E3 | 1.3E3 | 1.4E3 | 3.6E2 | 5.9E2 | 1.0E-9 | 1.0E-9 | 1.8E3 | 2.1E3 | 54 | 9 | 53 | 9 | 0.64 |
| vV | ng/ml | 9.7E2 | 1.3E3 | 1.4E3 | 1.4E3 | 1.6E3 | 1.3E3 | 1.1E2 | 1.1E2 | 1.1E4 | 4.1E3 | 54 | 9 | 53 | 9 | 0.52 |
| vW | ng/ml | 1.4E2 | 1.0E2 | 1.8E2 | 1.2E2 | 1.4E2 | 5.6E1 | 4.3E1 | 7.7E1 | 6.7E2 | 2.5E2 | 54 | 9 | 53 | 9 | 0.40 |
| pF | pg/ml | 5.1E-1 | 3.2E-1 | 6.9E-1 | 8.4E-1 | 9.9E-1 | 1.6E0 | 1.0E-9 | 1.0E-9 | 9.4E0 | 7.8E0 | 105 | 25 | 97 | 25 | 0.43 |

Figure 22.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.0E1 | 7.0E1 | 8.1E1 | 7.7E1 | 5.8E1 | 5.5E1 | 1.0E0 | 2.0E1 | 4.8E2 | 2.2E2 | 1595 | 17 | 266 | 17 | 0.48 |
| Ad | ug/mL | 3.8E-2 | 4.4E-2 | 9.4E-2 | 5.7E-2 | 4.1E-1 | 5.6E-2 | 2.7E-4 | 5.0E-3 | 8.5E0 | 2.0E-1 | 447 | 13 | 170 | 13 | 0.50 |
| Af | ng/mL | 1.2E0 | 3.3E-1 | 1.8E1 | 1.4E1 | 6.7E1 | 4.4E1 | 1.7E-3 | 1.7E-3 | 5.3E2 | 1.6E2 | 447 | 13 | 170 | 13 | 0.38 |
| Aj | ug/mL | 1.4E0 | 1.2E0 | 2.6E0 | 2.1E0 | 2.4E0 | 2.3E0 | 1.5E-3 | 3.2E-2 | 6.1E0 | 5.8E0 | 447 | 13 | 170 | 13 | 0.45 |
| Al | mg/mL | 8.7E-5 | 9.3E-5 | 2.5E-4 | 3.5E-4 | 4.1E-4 | 6.4E-4 | 2.3E-6 | 2.2E-5 | 1.9E-3 | 2.2E-3 | 447 | 13 | 170 | 13 | 0.55 |
| An | U/mL | 4.8E1 | 5.2E1 | 1.8E2 | 3.2E2 | 5.5E2 | 6.7E2 | 9.8E-4 | 7.4E0 | 7.8E3 | 2.5E3 | 447 | 13 | 170 | 13 | 0.60 |
| Ao | pg/mL | 9.2E1 | 5.7E1 | 5.1E1 | 1.1E2 | 3.5E3 | 1.5E2 | 1.5E0 | 7.8F0 | 3.9F4 | 5.8F2 | 447 | 13 | 170 | 13 | 0.37 |
| Ap | ng/mL | 3.3E1 | 4.2E1 | 4.7E1 | 5.6E1 | 5.0E1 | 5.2E1 | 8.4E-5 | 6.8E0 | 3.3E2 | 1.5E2 | 447 | 13 | 170 | 13 | 0.54 |
| Ar | ng/mL | 9.5E-1 | 2.3E0 | 1.2E1 | 3.9E0 | 1.9E2 | 4.5E0 | 3.4E-3 | 1.7E-1 | 4.1E3 | 1.5E1 | 447 | 13 | 170 | 13 | 0.65 |
| As | ng/mL | 8.7E-3 | 1.0E-2 | 1.6E-2 | 1.0E-2 | 6.1E-2 | 7.6E-3 | 1.7E-3 | 1.7E-3 | 1.2E0 | 2.9E-2 | 447 | 13 | 170 | 13 | 0.52 |
| Aw | pg/mL | 1.6E1 | 1.8E1 | 1.6E1 | 1.9E1 | 6.4E0 | 6.9E0 | 2.9E-2 | 1.1E1 | 5.1E1 | 3.4E1 | 447 | 13 | 170 | 13 | 0.63 |
| Ax | ng/mL | 2.1E0 | 4.2E0 | 1.6E1 | 1.4E1 | 6.3E1 | 1.8E1 | 1.2E-2 | 3.9E-1 | 7.7E2 | 5.1E1 | 447 | 13 | 170 | 13 | 0.61 |
| Ba | ng/mL | 6.1E1 | 4.5E1 | 4.1E2 | 6.8E2 | 1.1E3 | 9.8E2 | 3.7E-1 | 5.5E0 | 8.1E3 | 2.8E3 | 447 | 13 | 170 | 13 | 0.56 |
| Bb | ng/mL | 3.4E0 | 3.9E0 | 6.8E0 | 6.2E0 | 1.4E1 | 6.3E0 | 4.1E-3 | 4.1E-3 | 2.5E2 | 2.4E1 | 447 | 13 | 170 | 13 | 0.56 |
| Bc | ng/mL | 3.8E1 | 9.8E1 | 1.1E2 | 1.3E2 | 2.0E2 | 1.3E2 | 1.1E-1 | 4.2E-1 | 1.2E3 | 4.3E2 | 447 | 13 | 170 | 13 | 0.63 |
| Bg | ng/mL | 7.7E-2 | 5.2E-2 | 5.4E0 | 1.7E0 | 3.0E1 | 2.4E0 | 5.3E-4 | 5.3E-4 | 4.4E2 | 6.0E0 | 447 | 13 | 170 | 13 | 0.52 |
| Bn | ng/mL | 5.6E-2 | 5.6E-2 | 1.3E0 | 9.9E-1 | 3.3E0 | 2.1E0 | 5.6E-2 | 5.6E-2 | 5.8E1 | 7.1E0 | 447 | 13 | 170 | 13 | 0.43 |
| Bo | ng/mL | 1.2E1 | 2.1E1 | 1.4E1 | 1.9E1 | 1.9E1 | 1.3E1 | 1.6E-2 | 1.6E-2 | 2.8E2 | 3.7E1 | 447 | 13 | 170 | 13 | 0.62 |
| Ch | ulU/mL | 9.7E-1 | 1.2E0 | 1.7E1 | 2.2E1 | 1.0E2 | 5.9E1 | 3.4E-3 | 1.2E-1 | 1.8E3 | 2.1E2 | 447 | 13 | 170 | 13 | 0.57 |
| Co | pg/mL | 3.8E1 | 6.5E1 | 1.8E2 | 1.0E2 | 9.6E2 | 8.7E1 | 1.5E-1 | 8.1E0 | 1.7E4 | 2.3E2 | 447 | 13 | 170 | 13 | 0.60 |
| Cp | ng/mL | 2.1E1 | 2.8E1 | 3.0E1 | 3.7E1 | 6.7E1 | 2.6E1 | 6.0E-1 | 8.6E0 | 1.3E3 | 9.8E1 | 447 | 13 | 170 | 13 | 0.68 |
| Cq | ng/mL | 2.8E-2 | 3.1E-2 | 2.5E-1 | 6.0E-2 | 2.5E0 | 7.9E-2 | 8.0E-4 | 8.0E-4 | 4.9E1 | 2.8E-1 | 447 | 13 | 170 | 13 | 0.52 |
| Cs | ng/mL | 5.9E1 | 1.5E2 | 3.2E2 | 4.1E2 | 1.2E3 | 5.5E2 | 2.7E-2 | 5.8E0 | 1.8E4 | 1.6E3 | 447 | 13 | 170 | 13 | 0.64 |
| Ct | ng/mL | 6.1E-1 | 3.5E-1 | 3.2E1 | 6.3E1 | 9.9E1 | 1.7E2 | 1.1E-4 | 5.7E-2 | 6.2E2 | 6.2E2 | 447 | 13 | 170 | 13 | 0.52 |
| Cu | ng/mL | 2.4E-1 | 2.1E-1 | 5.5E-1 | 3.7E-1 | 3.2E0 | 3.8E-1 | 9.6E-3 | 5.3E-2 | 6.6E1 | 1.4E0 | 447 | 13 | 170 | 13 | 0.50 |
| Cv | ng/mL | 5.8E0 | 7.8E0 | 2.8E1 | 1.1E1 | 6.9E1 | 1.0E1 | 1.4E-4 | 5.7E-2 | 5.3E2 | 3.1E1 | 447 | 13 | 170 | 13 | 0.53 |
| Cw | mIU/mL | 3.0E-2 | 3.7E-2 | 5.4E-2 | 3.9E-2 | 3.2E-1 | 2.5E-2 | 1.5E-4 | 3.5E-3 | 6.8E0 | 8.2E-2 | 447 | 13 | 170 | 13 | 0.54 |
| Cx | ng/mL | 4.6E-1 | 1.3E-2 | 6.0E1 | 5.2E1 | 1.1E2 | 1.3E2 | 9.3E-5 | 9.3E-5 | 3.9E2 | 3.9E2 | 447 | 13 | 170 | 13 | 0.42 |
| Db | ug/mL | 7.6E0 | 8.2E0 | 9.6E0 | 1.0E1 | 1.1E1 | 1.1E1 | 4.5E-1 | 9.3E-1 | 1.4E2 | 4.1E1 | 447 | 13 | 170 | 13 | 0.51 |
| Dc | nmol/L | 1.9E-2 | 1.4E-2 | 8.6E-2 | 2.0E-2 | 6.7E-1 | 1.9E-2 | 5.2E-6 | 1.4E-3 | 1.4E1 | 7.4E-2 | 447 | 13 | 170 | 13 | 0.43 |
| Dd | ug/mL | 7.8E-2 | 3.9E-2 | 1.9E-1 | 6.7E-2 | 3.1E-1 | 6.7E-2 | 1.9E-4 | 1.9E-3 | 3.6E0 | 2.2E-1 | 447 | 13 | 170 | 13 | 0.37 |
| De | ng/mL | 3.4E-3 | 3.4E-3 | 7.6E-2 | 6.0E-2 | 1.5E-1 | 9.8E-2 | 3.4E-3 | 3.4E-3 | 9.2E-1 | 3.1E-1 | 447 | 13 | 170 | 13 | 0.50 |
| Dg | ng/mL | 3.4E1 | 3.8E1 | 4.6E1 | 5.1E1 | 4.1E1 | 5.5E1 | 1.0E-1 | 3.4E0 | 1.9E2 | 1.8E2 | 447 | 13 | 170 | 13 | 0.49 |
| Di | pg/mL | 1.9E0 | 1.5E0 | 2.2E0 | 2.1E0 | 2.1E0 | 2.6E0 | 1.8E-1 | 1.8E-1 | 1.3E1 | 9.0E0 | 447 | 13 | 170 | 13 | 0.44 |
| Dk | ulU/mL | 1.6E-2 | 1.5E-2 | 8.7E-2 | 3.9E-2 | 5.1E-1 | 6.1E-2 | 1.1E-4 | 1.1E-4 | 8.9E0 | 2.3E-1 | 447 | 13 | 170 | 13 | 0.50 |
| Dl | ng/mL | 2.4E2 | 2.0E2 | 3.2E2 | 3.2E2 | 2.9E2 | 3.9E2 | 2.5E0 | 1.3E1 | 1.6E3 | 1.4E3 | 447 | 13 | 170 | 13 | 0.45 |
| Dp | ng/ml | 2.5E0 | 2.8E0 | 6.4E0 | 8.6E0 | 1.5E1 | 1.3E1 | 3.7E-3 | 5.1E-1 | 2.0E2 | 4.4E1 | 262 | 13 | 160 | 13 | 0.55 |
| Ef | ng/ml | 1.3E-1 | 1.2E-1 | 8.6E-1 | 1.1E0 | 1.9E0 | 2.4E0 | 5.7E-4 | 1.8E-2 | 1.0E1 | 8.7E0 | 325 | 13 | 167 | 13 | 0.51 |
| Wm | % | 7.2E-1 | 4.9E-1 | 3.2E1 | 4.0E1 | 1.8E2 | 1.5E2 | 5.4E-2 | 8.5E-2 | 2.4E3 | 5.7E2 | 353 | 15 | 182 | 15 | 0.45 |
| Ed | pg/ml | 5.2E-1 | 5.2E-1 | 5.9E1 | 1.4E1 | 4.5E2 | 2.6E1 | 5.2E-1 | 5.2E-1 | 7.3E3 | 9.4E1 | 262 | 13 | 159 | 13 | 0.47 |
| Tj | pg/mL | 3.7E-1 | 9.3E-1 | 5.6E1 | 1.6E1 | 3.0E2 | 2.3E1 | 3.6E-1 | 3.7E-1 | 3.5E3 | 7.1E1 | 323 | 13 | 169 | 13 | 0.57 |
| Po | pg/ml | 5.4E-1 | 3.3E0 | 8.9E0 | 6.5E0 | 2.6E1 | 8.9E0 | 8.0E-3 | 2.6E-2 | 2.7E2 | 3.6E1 | 755 | 23 | 295 | 23 | 0.57 |
| Et | ng/ml | 1.4E3 | 1.4E3 | 1.7E3 | 1.7E3 | 1.2E3 | 1.3E3 | 7.5E1 | 1.6E2 | 5.0E3 | 4.7E3 | 754 | 23 | 295 | 23 | 0.50 |
| Fa | ng/ml | 4.0E1 | 8.6E1 | 1.3E2 | 1.0E2 | 5.6E2 | 8.3E1 | 3.4E-2 | 3.4E0 | 8.0E3 | 2.7E2 | 261 | 13 | 158 | 13 | 0.66 |
| Ez | ng/ml | 3.8E0 | 7.8E0 | 1.5E1 | 2.9E1 | 3.2E1 | 4.3E1 | 1.3E-2 | 1.3E-2 | 3.0E2 | 1.5E2 | 262 | 13 | 160 | 13 | 0.61 |
| Fb | ng/ml | 2.5E1 | 2.9E1 | 2.2E1 | 3.0E1 | 1.1E1 | 1.2E1 | 5.9E-1 | 4.0E0 | 5.7E1 | 4.5E1 | 262 | 13 | 158 | 13 | 0.69 |
| Ex | ng/ml | 7.8E-2 | 9.7E-2 | 2.2E-1 | 2.3E-1 | 6.9E-1 | 2.8E-1 | 3.5E-5 | 2.7E-3 | 8.9E0 | 8.2E-1 | 245 | 10 | 117 | 10 | 0.60 |
| Fn | ng/ml | 2.1E-1 | 2.1E-1 | 5.9E0 | 3.7E0 | 2.7E1 | 8.9E0 | 1.1E-14 | 1.1E-14 | 4.2E2 | 3.2E1 | 262 | 13 | 160 | 13 | 0.35 |
| Fp | ng/ml | 1.3E1 | 2.2E1 | 2.5E1 | 4.0E1 | 2.8E1 | 4.3E1 | 6.0E-3 | 1.3E-1 | 1.4E2 | 1.3E2 | 787 | 23 | 296 | 23 | 0.57 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|-----|--------|-----|-----|
| | | NonDis | Dis | NonDis | Dis | NonDis | Dis | NonDis | Dis | NonDis | Dis | NonDis | Dis | NonDis | Dis | |
| Fr | ng/ml | 3.5E4 | 7.0E4 | 1.1E5 | 1.4E5 | 1.7E5 | 1.7E5 | 1.9E2 | 2.8E3 | 9.0E5 | 6.0E5 | 890 | 23 | 300 | 23 | 0.57 |
| Fw | pg/ml | 1.1E0 | 2.5E0 | 6.2E1 | 6.8E0 | 4.8E2 | 1.2E1 | 1.1E-14 | 1.7E-14 | 6.9E3 | 4.4E1 | 325 | 13 | 168 | 13 | 0.46 |
| Fy | ng/ml | 3.8E1 | 3.4E1 | 6.0E1 | 6.0E1 | 7.3E1 | 6.5E1 | 1.2E-1 | 1.2E-1 | 6.5E2 | 2.1E2 | 260 | 13 | 159 | 13 | 0.51 |
| Gl | pg/ml | 7.1E3 | 5.9E3 | 1.1E4 | 1.1E4 | 9.1E3 | 1.1E4 | 9.1E1 | 4.9E2 | 3.4E4 | 3.0E4 | 316 | 13 | 166 | 13 | 0.49 |
| Gp | U/ml | 1.5E0 | 3.0E0 | 4.1E0 | 3.3E0 | 7.0E0 | 3.2E0 | 1.3E-3 | 1.5E-2 | 6.7E1 | 8.9E0 | 327 | 13 | 168 | 13 | 0.50 |
| Gz | ug/ml | 1.4E0 | 8.2E-1 | 8.9E0 | 5.4E0 | 3.7E1 | 9.0E0 | 2.9E-16 | 1.5E-1 | 4.8E2 | 2.8E1 | 181 | 10 | 106 | 10 | 0.41 |
| Ha | ng/ml | 2.3E0 | 4.6E0 | 9.5E0 | 1.3E1 | 2.1E1 | 2.0E1 | 6.4E-3 | 1.4E-3 | 1.3E2 | 6.1E1 | 260 | 13 | 159 | 13 | 0.59 |
| Nm | pg/ml | 1.5E4 | 9.9E3 | 3.4E4 | 2.2E4 | 8.7E4 | 3.6E4 | 1.0E-9 | 1.0E-9 | 1.6E6 | 1.7E5 | 758 | 23 | 297 | 23 | 0.42 |
| Nn | pg/ml | 1.5E2 | 3.6E2 | 1.7E3 | 1.2E3 | 7.6E3 | 1.6E3 | 1.0E-9 | 1.0E-9 | 1.0E5 | 5.3E3 | 758 | 23 | 297 | 23 | 0.60 |
| No | pg/ml | 1.5E1 | 9.1E0 | 3.9E1 | 2.3E1 | 1.2E2 | 2.6E1 | 1.0E-9 | 1.0E-9 | 2.5E3 | 7.9E1 | 758 | 23 | 297 | 23 | 0.44 |
| Nq | pg/ml | 1.7E0 | 4.4E0 | 1.7E1 | 1.5E1 | 6.8E1 | 2.6E1 | 1.0E-9 | 1.0E-9 | 1.2E3 | 1.1E2 | 758 | 23 | 297 | 23 | 0.55 |
| Nr | pg/ml | 9.7E-1 | 1.6E0 | 3.1E1 | 7.3E0 | 1.9E2 | 1.1E1 | 1.0E-9 | 1.0E-9 | 4.1E3 | 4.3E1 | 758 | 23 | 297 | 23 | 0.49 |
| Ns | pg/ml | 1.0E-9 | 1.0E-9 | 7.1E0 | 1.9E0 | 3.5E1 | 7.8E0 | 1.0E-9 | 1.0E-9 | 6.8E2 | 3.7E1 | 758 | 23 | 297 | 23 | 0.51 |
| Nt | pg/ml | 1.0E2 | 9.7E1 | 1.3E2 | 1.3E2 | 1.1E2 | 1.1E2 | 1.0E-9 | 1.0E-9 | 1.7E3 | 5.0E2 | 758 | 23 | 297 | 23 | 0.49 |
| Nu | pg/ml | 2.0E1 | 4.3E1 | 5.3E1 | 7.1E1 | 8.9E1 | 7.6E1 | 1.0E-9 | 1.0E-9 | 8.9E2 | 2.4E2 | 758 | 23 | 297 | 23 | 0.56 |
| Lu | pg/ml | 1.0E4 | 9.9E3 | 1.8E4 | 8.1E3 | 6.5E4 | 4.7E3 | 3.5E2 | 1.7E3 | 1.3E6 | 1.9E4 | 761 | 23 | 297 | 23 | 0.39 |
| Lv | pg/ml | 1.0E-9 | 1.0E-9 | 1.1E1 | 2.7E1 | 2.1E1 | 4.5E1 | 1.0E-9 | 1.0E-9 | 2.4E2 | 1.9E2 | 761 | 23 | 297 | 23 | 0.60 |
| Lw | pg/ml | 1.0E-9 | 1.0E-9 | 4.3E-1 | 1.0E-9 | 4.1E0 | 0.0E0 | 1.0E-9 | 1.0E-9 | 8.0E1 | 1.0E-9 | 761 | 23 | 297 | 23 | 0.49 |
| Lx | pg/ml | 1.0E-9 | 6.4E1 | 1.8E2 | 1.6E2 | 9.3E2 | 3.0E2 | 1.0E-9 | 1.0E-9 | 2.2E4 | 1.4E3 | 761 | 23 | 297 | 23 | 0.61 |
| Ly | pg/ml | 1.0E-9 | 7.0E0 | 1.0E1 | 1.6E1 | 2.0E1 | 1.9E1 | 1.0E-9 | 1.0E-9 | 1.2E2 | 5.6E1 | 761 | 23 | 297 | 23 | 0.61 |
| Lz | pg/ml | 1.0E-9 | 1.0E-9 | 4.0E0 | 8.5E-1 | 3.2E1 | 4.1E0 | 1.0E-9 | 1.0E-9 | 6.0E2 | 2.0E1 | 761 | 23 | 297 | 23 | 0.48 |
| Ma | pg/ml | 2.9E2 | 3.7E2 | 1.3E3 | 1.6E3 | 3.6E3 | 3.1E3 | 1.0E-9 | 1.0E-9 | 6.5E4 | 1.2E4 | 761 | 23 | 297 | 23 | 0.48 |
| Mb | pg/ml | 2.5E1 | 3.6E1 | 3.1E1 | 3.5E1 | 1.5E1 | 1.6E1 | 4.1E0 | 1.5E1 | 2.1E2 | 7.5E1 | 761 | 23 | 297 | 23 | 0.56 |
| Mc | pg/ml | 1.0E-9 | 1.0E-9 | 4.3E-2 | 1.1E-1 | 6.1E-1 | 5.2E-1 | 1.0E-9 | 1.0E-9 | 1.3E1 | 2.5E0 | 761 | 23 | 297 | 23 | 0.52 |
| Md | pg/ml | 1.0E-9 | 1.0E-9 | 4.5E-1 | 1.0E-9 | 3.9E0 | 0.0E0 | 1.0E-9 | 1.0E-9 | 6.8E1 | 1.0E-9 | 761 | 23 | 297 | 23 | 0.46 |
| Me | pg/ml | 3.3E1 | 2.6E1 | 3.2E1 | 2.7E1 | 2.0E1 | 1.5E1 | 1.0E-9 | 1.0E-9 | 3.2E2 | 5.5E1 | 761 | 23 | 297 | 23 | 0.40 |
| Mf | pg/ml | 1.0E-9 | 1.0E-9 | 5.0E-1 | 5.8E-1 | 2.8E0 | 1.7E0 | 1.0E-9 | 1.0E-9 | 5.6E1 | 7.7E0 | 761 | 23 | 297 | 23 | 0.53 |
| Mg | pg/ml | 1.6E0 | 7.3E-2 | 7.0E0 | 6.4E0 | 1.2E1 | 1.3E1 | 1.0E-9 | 1.0E-9 | 9.2E1 | 5.5E1 | 761 | 23 | 297 | 23 | 0.47 |
| Mh | pg/ml | 1.0E-9 | 1.0E-9 | 1.5E0 | 9.3E-2 | 1.0E1 | 3.1E-1 | 1.0E-9 | 1.0E-9 | 1.6E2 | 1.4E0 | 761 | 23 | 297 | 23 | 0.44 |
| Mi | pg/ml | 1.0E-9 | 1.0E-9 | 9.4E-1 | 1.0E-9 | 1.3E1 | 0.0E0 | 1.0E-9 | 1.0E-9 | 3.2E2 | 1.0E-9 | 761 | 23 | 297 | 23 | 0.49 |
| Mj | pg/ml | 1.0E-9 | 1.0E-9 | 4.7E0 | 3.5E0 | 2.7E1 | 7.6E0 | 1.0E-9 | 1.0E-9 | 4.6E2 | 2.9E1 | 761 | 23 | 297 | 23 | 0.56 |
| Mk | pg/ml | 2.8E-1 | 1.0E-9 | 1.4E1 | 1.2E1 | 9.2E1 | 2.3E1 | 1.0E-9 | 1.0E-9 | 1.7E3 | 9.4E1 | 761 | 23 | 297 | 23 | 0.50 |
| Ml | pg/ml | 1.0E-9 | 1.0E-9 | 6.0E0 | 2.3E0 | 8.1E1 | 7.4E0 | 1.0E-9 | 1.0E-9 | 2.1E3 | 3.3E1 | 761 | 23 | 297 | 23 | 0.50 |
| Mm | pg/ml | 6.1E2 | 5.3E2 | 1.1E3 | 8.2E2 | 1.4E3 | 1.1E3 | 1.0E-9 | 1.0E-9 | 1.2E4 | 5.3E3 | 761 | 23 | 297 | 23 | 0.46 |
| Mn | pg/ml | 5.4E0 | 4.8E0 | 1.0E1 | 6.2E0 | 2.0E1 | 6.1E0 | 1.0E-9 | 1.0E-9 | 3.5E2 | 2.2E1 | 761 | 23 | 297 | 23 | 0.43 |
| Mp | pg/ml | 1.0E-9 | 1.0E-9 | 1.0E1 | 1.1E1 | 3.6E1 | 2.5E1 | 1.0E-9 | 1.0E-9 | 6.3E2 | 9.7E1 | 760 | 23 | 297 | 23 | 0.51 |
| Mq | pg/ml | 1.0E-9 | 1.0E-9 | 2.8E0 | 4.1E0 | 1.6E1 | 1.3E1 | 1.0E-9 | 1.0E-9 | 2.1E2 | 5.1E1 | 760 | 23 | 297 | 23 | 0.53 |
| Mr | pg/ml | 1.0E-9 | 1.0E-9 | 3.2E1 | 7.2E0 | 1.8E2 | 1.5E1 | 1.0E-9 | 1.0E-9 | 2.2E3 | 6.8E1 | 760 | 23 | 297 | 23 | 0.56 |
| Ms | pg/ml | 4.1E2 | 3.7E2 | 5.6E2 | 5.5E2 | 6.6E2 | 6.3E2 | 1.0E-9 | 1.0E-9 | 5.9E3 | 3.0E3 | 760 | 23 | 297 | 23 | 0.51 |
| Mt | pg/ml | 2.4E-1 | 8.8E-1 | 7.3E0 | 5.1E0 | 4.5E1 | 1.6E1 | 1.0E-9 | 1.0E-9 | 8.7E2 | 7.9E1 | 760 | 23 | 297 | 23 | 0.55 |
| Mu | pg/ml | 1.0E-9 | 1.0E-9 | 9.0E-1 | 4.6E-1 | 7.0E0 | 9.3E-1 | 1.0E-9 | 1.0E-9 | 9.9E1 | 3.6E0 | 760 | 23 | 297 | 23 | 0.59 |
| Mv | pg/ml | 1.0E-9 | 1.0E-9 | 5.8E1 | 7.0E1 | 3.1E2 | 1.8E2 | 1.0E-9 | 1.0E-9 | 4.5E3 | 7.0E2 | 760 | 23 | 297 | 23 | 0.57 |
| Mw | pg/ml | 3.8E1 | 3.2E1 | 4.0E2 | 2.1E2 | 2.8E3 | 4.0E2 | 1.0E-9 | 1.0E-9 | 6.2E4 | 1.4E3 | 760 | 23 | 297 | 23 | 0.52 |
| Mx | pg/ml | 1.0E-9 | 1.0E-9 | 2.2E-1 | 1.3E-1 | 9.1E-1 | 3.3E-1 | 1.0E-9 | 1.0E-9 | 9.6E0 | 1.2E0 | 760 | 23 | 297 | 23 | 0.52 |
| My | pg/ml | 1.0E-9 | 1.0E-9 | 3.9E2 | 2.2E2 | 2.7E3 | 4.7E2 | 1.0E-9 | 1.0E-9 | 3.9E4 | 1.7E3 | 760 | 23 | 297 | 23 | 0.51 |
| Mz | pg/ml | 1.1E1 | 6.1E0 | 2.6E1 | 3.0E1 | 7.9E1 | 5.9E1 | 1.0E-9 | 1.0E-9 | 1.3E3 | 2.1E2 | 760 | 23 | 297 | 23 | 0.46 |
| Na | pg/ml | 1.0E-9 | 1.0E-9 | 7.4E-1 | 1.2E0 | 3.2E0 | 2.5E0 | 1.0E-9 | 1.0E-9 | 4.2E1 | 7.2E0 | 760 | 23 | 297 | 23 | 0.60 |
| Nb | pg/ml | 1.9E0 | 2.1E0 | 4.0E0 | 3.4E0 | 1.3E1 | 3.6E0 | 1.0E-9 | 1.0E-9 | 2.3E2 | 1.4E1 | 760 | 23 | 297 | 23 | 0.54 |
| Nc | pg/ml | 3.4E2 | 3.0E2 | 5.8E2 | 4.9E2 | 7.5E2 | 5.5E2 | 1.0E-9 | 1.0E-9 | 9.2E3 | 1.8E3 | 760 | 23 | 297 | 23 | 0.49 |
| Nd | pg/ml | 2.9E1 | 8.5E0 | 3.0E1 | 1.6E1 | 9.0E1 | 1.6E1 | 1.0E-9 | 1.0E-9 | 2.1E3 | 5.2E1 | 760 | 23 | 297 | 23 | 0.35 |
| Ne | pg/ml | 4.5E2 | 3.4E2 | 5.9E2 | 3.9E2 | 5.9E2 | 3.7E2 | 1.0E-9 | 1.0E-9 | 7.0E3 | 1.5E3 | 760 | 23 | 297 | 23 | 0.40 |
| Nf | pg/ml | 1.0E-9 | 1.0E-9 | 2.9E0 | 2.4E0 | 1.1E1 | 9.2E0 | 1.0E-9 | 1.0E-9 | 1.6E2 | 4.4E1 | 760 | 23 | 297 | 23 | 0.47 |
| Ng | pg/ml | 1.9E1 | 9.0E-1 | 1.1E2 | 1.2E2 | 2.2E2 | 2.3E2 | 1.0E-9 | 1.0E-9 | 1.6E3 | 8.6E2 | 760 | 23 | 297 | 23 | 0.44 |

| Code | Units | Median | | Average | | Standard Deviation | | Minimum | | Maximum | | Number of Samples | | Number of Patients | | AUC |
|------|-------|--------|------|---------|------|------|------|------|------|------|------|------|------|------|------|------|
| | | NonDis | Dis | NonDis | Dis | NonDis | Dis | NonDis | Dis | NonDis | Dis | NonDis | Dis | NonDis | Dis | |
| Nh | pg/ml | 6.9E1 | 4.8E1 | 9.2E1 | 6.9E1 | 8.3E1 | 5.8E1 | 1.0E-9 | 2.5E0 | 5.6E2 | 2.2E2 | 760 | 23 | 297 | 23 | 0.43 |
| Ni | pg/ml | 1.0E-9 | 1.1E1 | 7.2E1 | 1.4E2 | 1.2E2 | 2.2E2 | 1.0E-9 | 1.0E-9 | 1.1E3 | 7.1E2 | 760 | 23 | 297 | 23 | 0.58 |
| Nj | pg/ml | 7.3E1 | 5.1E0 | 1.1E1 | 7.4E0 | 1.2E1 | 6.7E0 | 1.0E-9 | 1.0E-9 | 8.3E1 | 2.0E1 | 760 | 23 | 297 | 23 | 0.43 |
| Nk | pg/ml | 1.8E1 | 1.6E1 | 3.4E1 | 3.0E1 | 4.0E1 | 3.3E1 | 1.0E-9 | 1.0E-9 | 2.0E2 | 1.1E2 | 760 | 23 | 297 | 23 | 0.50 |
| Nl | pg/ml | 4.6E1 | 3.6E1 | 6.2E1 | 4.5E1 | 7.0E1 | 4.2E1 | 1.0E-9 | 1.0E-9 | 1.1E3 | 1.4E2 | 760 | 23 | 297 | 23 | 0.43 |
| Tz | pg/ml | 4.6E3 | 6.1E3 | 1.3E4 | 7.6E3 | 6.3E4 | 5.5E3 | 1.0E-9 | 1.8E3 | 1.0E6 | 1.9E4 | 264 | 13 | 159 | 13 | 0.57 |
| Ua | pg/ml | 3.7E3 | 3.9E3 | 2.0E4 | 1.7E4 | 1.3E5 | 2.7E4 | 1.0E-9 | 8.8E2 | 2.1E6 | 9.0E4 | 264 | 13 | 159 | 13 | 0.52 |
| Ub | pg/ml | 5.7E2 | 4.5E2 | 8.7E2 | 8.9E2 | 1.1E3 | 1.1E3 | 1.0E-9 | 1.2E1 | 9.8E3 | 3.3E3 | 264 | 13 | 159 | 13 | 0.47 |
| Ue | pg/ml | 3.1E1 | 1.6E1 | 4.0E1 | 2.3E1 | 4.0E1 | 2.9E1 | 9.8E-2 | 5.1E0 | 4.4E2 | 1.2E2 | 264 | 13 | 159 | 13 | 0.27 |
| Uc | pg/ml | 9.2E2 | 6.4E2 | 2.0E3 | 9.2E2 | 4.4E3 | 1.2E3 | 1.0E-9 | 8.9E1 | 5.7E4 | 4.7E3 | 264 | 13 | 159 | 13 | 0.37 |
| Ud | pg/ml | 1.0E-9 | 1.0E-9 | 1.8E0 | 5.3E-1 | 2.4E1 | 1.9E0 | 1.0E-9 | 1.0E-9 | 3.9E2 | 6.9E0 | 264 | 13 | 159 | 13 | 0.53 |
| Hq | pg/ml | 1.0E0 | 1.5E0 | 1.2E2 | 2.1E1 | 1.8E3 | 7.1E1 | 1.0E-9 | 1.0E-9 | 3.4E4 | 3.4E2 | 756 | 23 | 296 | 23 | 0.53 |
| Hr | pg/ml | 1.1E2 | 3.1E2 | 8.0E2 | 1.1E3 | 1.7E3 | 1.6E3 | 1.0E-9 | 1.0E-9 | 1.7E4 | 6.0E3 | 756 | 23 | 296 | 23 | 0.62 |
| Hu | pg/ml | 5.1E0 | 3.1E1 | 2.6E3 | 1.6E3 | 2.6E4 | 4.4E3 | 1.0E-9 | 1.0E-9 | 6.3E5 | 1.9E4 | 756 | 23 | 296 | 23 | 0.58 |
| Hv | pg/ml | 1.3E0 | 2.7E0 | 4.2E0 | 4.2E0 | 3.4E1 | 7.4E0 | 1.0E-9 | 1.0E-9 | 8.9E2 | 3.5E1 | 756 | 23 | 296 | 23 | 0.65 |
| Hw | pg/ml | 6.6E0 | 5.5E0 | 3.1E1 | 1.6E1 | 3.5E2 | 3.2E1 | 1.0E-9 | 1.0E-9 | 9.4E3 | 1.5E2 | 756 | 23 | 296 | 23 | 0.49 |
| Hx | pg/ml | 8.3E0 | 1.4E1 | 4.0E1 | 3.2E1 | 3.5E2 | 5.3E1 | 1.0E-9 | 1.0E-9 | 9.3E3 | 2.2E2 | 756 | 23 | 296 | 23 | 0.60 |
| Ib | ng/ml | 4.9E-2 | 2.1E-2 | 1.2E0 | 9.7E0 | 5.3E0 | 2.5E1 | 1.0E-9 | 1.0E-9 | 5.6E1 | 8.8E1 | 259 | 12 | 159 | 12 | 0.47 |
| Ic | U/ml | 2.2E2 | 2.2E2 | 1.1E3 | 1.9E3 | 7.3E3 | 5.9E3 | 1.5E0 | 2.2E1 | 9.3E4 | 2.1E4 | 259 | 12 | 159 | 12 | 0.46 |
| Id | U/ml | 6.8E-1 | 8.4E-1 | 3.0E0 | 9.4E-1 | 2.7E1 | 4.9E-1 | 1.0E-9 | 3.3E-1 | 4.3E2 | 1.9E0 | 259 | 12 | 159 | 12 | 0.55 |
| Tt | pg/ml | 1.6E2 | 1.6E2 | 1.7E2 | 1.7E2 | 5.4E1 | 4.4E1 | 4.3E1 | 1.1E2 | 4.4E2 | 2.4E2 | 246 | 10 | 153 | 10 | 0.48 |
| To | pg/ml | 1.6E0 | 2.3E0 | 1.9E0 | 1.9E0 | 2.3E0 | 1.6E0 | 1.0E-9 | 1.0E-9 | 2.3E1 | 4.7E0 | 254 | 11 | 156 | 11 | 0.56 |
| Tr | pg/ml | 3.3E0 | 2.6E0 | 6.6E0 | 3.6E0 | 2.1E1 | 3.6E0 | 1.0E-9 | 4.4E-1 | 3.1E2 | 1.3E1 | 251 | 10 | 155 | 10 | 0.44 |
| Tn | pg/ml | 2.8E1 | 4.1E1 | 8.2E1 | 3.8E1 | 2.5E2 | 2.1E1 | 2.4E0 | 7.4E0 | 2.3E3 | 7.6E1 | 254 | 11 | 156 | 11 | 0.54 |
| Tv | ng/ml | 1.2E1 | 1.2E1 | 5.2E1 | 1.3E1 | 4.5E2 | 1.4E1 | 1.0E-9 | 1.0E-9 | 7.1E3 | 4.1E1 | 254 | 11 | 156 | 11 | 0.43 |
| Ih | ng/ml | 6.8E1 | 6.4E1 | 2.4E2 | 1.4E2 | 4.3E2 | 2.1E2 | 1.0E-9 | 1.0E-9 | 3.6E3 | 7.5E2 | 760 | 23 | 296 | 23 | 0.46 |
| Ii | ng/ml | 9.1E1 | 4.7E1 | 2.4E2 | 1.4E2 | 6.3E2 | 2.2E2 | 1.0E-9 | 2.3E0 | 8.4E3 | 8.8E2 | 760 | 23 | 296 | 23 | 0.44 |
| Ij | ng/ml | 7.6E1 | 7.5E1 | 2.0E2 | 1.2E2 | 1.1E3 | 1.3E2 | 1.0E-9 | 5.6E0 | 2.4E4 | 4.9E2 | 750 | 23 | 294 | 23 | 0.51 |
| Ik | ng/ml | 1.1E1 | 1.1E2 | 9.1E2 | 3.3E2 | 8.9E3 | 4.7E2 | 5.9E-1 | 2.8E0 | 1.2E5 | 1.5E3 | 755 | 23 | 294 | 23 | 0.61 |
| Il | ng/ml | 3.2E2 | 4.9E2 | 1.3E3 | 7.3E2 | 2.8E3 | 8.7E2 | 1.0E-9 | 1.0E-9 | 1.3E4 | 3.2E3 | 744 | 23 | 295 | 23 | 0.52 |
| Im | ng/ml | 2.1E2 | 2.4E2 | 3.7E2 | 5.5E2 | 5.3E2 | 1.3E3 | 1.3E1 | 3.2E1 | 5.8E3 | 6.2E3 | 754 | 23 | 295 | 23 | 0.49 |
| In | ng/ml | 3.6E0 | 4.3E0 | 3.0E1 | 1.1E1 | 2.3E2 | 2.0E1 | 1.0E-9 | 1.0E-9 | 4.5E3 | 9.6E1 | 760 | 23 | 296 | 23 | 0.50 |
| Hb | ng/ml | 2.6E1 | 2.5E1 | 3.7E1 | 3.0E1 | 3.5E1 | 2.0E1 | 6.2E-1 | 4.1E0 | 2.1E2 | 6.8E1 | 264 | 13 | 159 | 13 | 0.48 |
| Hc | pg/ml | 6.3E2 | 8.3E2 | 3.4E3 | 3.7E3 | 1.3E4 | 6.3E3 | 1.0E-9 | 1.0E-9 | 1.0E5 | 2.0E4 | 264 | 13 | 159 | 13 | 0.58 |
| Hf | ng/ml | 1.5E2 | 1.7E2 | 3.5E2 | 3.1E2 | 4.9E2 | 3.9E2 | 1.0E-9 | 1.0E-9 | 2.5E3 | 1.2E3 | 264 | 13 | 159 | 13 | 0.51 |
| Io | ng/ml | 8.2E3 | 3.9E3 | 2.6E4 | 1.2E4 | 1.6E5 | 2.5E4 | 1.0E-9 | 3.0E2 | 4.0E6 | 1.2E5 | 752 | 23 | 296 | 23 | 0.36 |
| Ip | ng/ml | 8.8E0 | 5.7E0 | 1.9E1 | 2.5E1 | 2.4E1 | 3.7E1 | 1.0E-9 | 1.4E-2 | 2.6E2 | 1.4E2 | 752 | 23 | 296 | 23 | 0.50 |
| Iq | ug/ml | 9.6E-2 | 8.1E-2 | 1.9E1 | 5.9E2 | 5.0E2 | 2.8E3 | 1.0E-9 | 1.0E-9 | 1.4E4 | 1.4E4 | 752 | 23 | 296 | 23 | 0.47 |
| Ir | ug/ml | 3.4E-1 | 6.3E-1 | 3.7E0 | 1.0E1 | 2.7E1 | 4.7E1 | 1.0E-9 | 1.0E-9 | 5.1E2 | 2.2E2 | 751 | 23 | 296 | 23 | 0.58 |
| Is | ng/ml | 1.4E0 | 3.0E0 | 6.2E0 | 6.8E0 | 2.4E1 | 1.1E1 | 1.0E-9 | 7.6E-2 | 5.5E2 | 4.6E1 | 752 | 23 | 296 | 23 | 0.58 |
| It | ng/ml | 2.0E0 | 3.3E0 | 2.4E1 | 1.3E1 | 1.4E2 | 3.1E1 | 1.0E-9 | 1.0E-9 | 2.8E3 | 1.5E2 | 752 | 23 | 296 | 23 | 0.61 |
| Iu | ng/ml | 2.2E2 | 4.1E2 | 1.5E3 | 1.5E3 | 4.3E3 | 5.0E3 | 1.0E-9 | 1.0E-9 | 2.9E4 | 2.4E4 | 752 | 23 | 296 | 23 | 0.53 |
| Iv | ng/ml | 1.2E1 | 1.2E1 | 6.5E1 | 4.8E1 | 6.0E2 | 9.8E1 | 1.0E-9 | 1.0E-9 | 1.6E4 | 4.5E2 | 751 | 23 | 296 | 23 | 0.52 |
| Iz | ng/ml | 1.4E2 | 1.1E2 | 6.3E2 | 5.3E2 | 3.9E3 | 8.1E2 | 9.2E-1 | 2.1E1 | 6.2E4 | 2.8E3 | 264 | 13 | 159 | 13 | 0.53 |
| Rc | pg/ml | 6.1E3 | 4.3E3 | 7.3E3 | 7.5E3 | 5.5E3 | 6.6E3 | 1.9E2 | 1.4E3 | 3.0E4 | 2.2E4 | 261 | 13 | 159 | 13 | 0.47 |
| Rb | pg/ml | 8.6E-1 | 1.1E0 | 2.8E0 | 1.8E0 | 5.5E0 | 1.9E0 | 1.0E-9 | 1.0E-9 | 5.6E1 | 5.6E0 | 261 | 13 | 159 | 13 | 0.52 |
| Pz | ng/ml | 4.4E3 | 1.8E3 | 8.3E3 | 4.4E3 | 4.0E4 | 4.2E3 | 1.3E1 | 9.5E1 | 1.0E6 | 1.0E4 | 753 | 23 | 295 | 23 | 0.42 |
| Qa | ng/ml | 3.4E3 | 5.2E3 | 6.3E3 | 6.8E3 | 1.1E4 | 5.4E3 | 1.2E1 | 2.9E2 | 2.2E5 | 1.9E4 | 753 | 23 | 295 | 23 | 0.58 |
| Qb | ng/ml | 8.7E1 | 1.7E2 | 2.1E2 | 2.1E2 | 5.0E2 | 2.4E2 | 7.9E-1 | 8.7E0 | 8.3E3 | 9.1E2 | 753 | 23 | 295 | 23 | 0.57 |
| Qc | ng/ml | 2.1E2 | 3.5E2 | 4.4E2 | 5.1E2 | 7.6E2 | 4.9E2 | 1.0E-9 | 8.6E0 | 1.1E4 | 1.6E3 | 753 | 23 | 295 | 23 | 0.57 |
| Qd | ng/ml | 8.9E3 | 1.4E4 | 1.9E4 | 2.4E4 | 7.8E4 | 3.3E4 | 1.5E2 | 1.7E3 | 2.0E6 | 1.5E5 | 753 | 23 | 295 | 23 | 0.61 |
| Qe | ng/ml | 9.1E2 | 1.2E3 | 1.8E3 | 1.9E3 | 4.0E3 | 2.2E3 | 1.0E-9 | 1.2E2 | 9.7E4 | 8.1E3 | 753 | 23 | 295 | 23 | 0.52 |
| Jd | ng/ml | 9.0E-1 | 2.0E0 | 6.6E0 | 2.1E0 | 4.3E1 | 2.3E0 | 1.0E-9 | 1.0E-9 | 6.5E2 | 8.3E0 | 262 | 13 | 160 | 13 | 0.56 |

| Code | Units | Median | | Average | | Standard Deviation | | Minimum | | Maximum | | Number of Samples | | Number of Patients | | AUC |
|------|-------|--------|------|---------|------|--------------------|------|---------|------|---------|------|-------------------|------|-------------------|------|------|
| | | NonDis | Dis | NonDis | Dis | NonDis | Dis | NonDis | Dis | NonDis | Dis | NonDis | Dis | NonDis | Dis | |
| Je | ng/ml | 1.0E-9 | 1.0E-9 | 2.1E0 | 1.3E0 | 7.8E0 | 2.3E0 | 1.0E-9 | 1.0E-9 | 8.8E1 | 7.7E0 | 262 | 13 | 160 | 13 | 0.50 |
| Jf | ng/ml | 1.0E-9 | 8.8E-1 | 1.0E0 | 1.8E0 | 2.2E0 | 2.5E0 | 1.0E-9 | 1.0E-9 | 1.2E1 | 8.6E0 | 262 | 13 | 160 | 13 | 0.65 |
| Jg | ng/ml | 5.0E2 | 3.6E2 | 7.7E2 | 7.5E2 | 9.2E2 | 7.4E2 | 1.0E-9 | 3.8E0 | 1.0E4 | 2.4E3 | 756 | 23 | 296 | 23 | 0.50 |
| Jh | ng/ml | 2.8E0 | 6.0E0 | 2.4E1 | 2.2E1 | 1.1E2 | 4.0E1 | 1.0E-9 | 1.0E-9 | 1.6E3 | 1.4E2 | 756 | 23 | 296 | 23 | 0.56 |
| Ji | ng/ml | 5.2E1 | 8.1E1 | 7.7E1 | 9.5E1 | 8.9E1 | 8.3E1 | 1.0E-9 | 4.6E0 | 1.3E3 | 3.4E2 | 756 | 23 | 296 | 23 | 0.59 |
| Sr | pg/mL | 3.9E2 | 3.0E2 | 9.8E2 | 9.9E2 | 1.8E3 | 1.5E3 | 1.0E-9 | 1.0E-9 | 2.1E4 | 4.2E3 | 259 | 13 | 159 | 13 | 0.47 |
| Ss | pg/mL | 9.4E4 | 9.3E4 | 1.5E5 | 1.1E5 | 1.9E5 | 9.0E4 | 2.7E3 | 1.1E4 | 1.8E6 | 2.0E5 | 259 | 13 | 159 | 13 | 0.43 |
| St | pg/mL | 2.6E7 | 4.8E7 | 4.8E7 | 8.6E7 | 6.1E7 | 1.1E8 | 1.0E-9 | 1.6E6 | 5.4E8 | 3.5E8 | 257 | 12 | 157 | 12 | 0.59 |
| Ra | pg/ml | 1.0E-9 | 5.2E-1 | 8.4E-1 | 1.1E0 | 4.1E0 | 1.5E0 | 1.0E-9 | 1.0E-9 | 6.4E1 | 4.9E0 | 261 | 13 | 159 | 13 | 0.66 |
| Qz | pg/ml | 1.0E1 | 9.0E0 | 6.0E1 | 1.1E2 | 9.9E1 | 1.7E2 | 1.0E-9 | 1.0E-9 | 7.1E2 | 5.5E2 | 261 | 13 | 159 | 13 | 0.54 |
| Qy | pg/ml | 4.4E-1 | 8.8E-1 | 9.8E0 | 5.0E1 | 5.8E1 | 1.2E2 | 1.0E-9 | 1.0E-9 | 6.5E2 | 3.9E2 | 261 | 13 | 159 | 13 | 0.66 |
| Qx | pg/ml | 1.0E-9 | 1.0E-9 | 6.6E0 | 2.8E0 | 5.1E1 | 6.3E0 | 1.0E-9 | 1.0E-9 | 5.8E2 | 2.1E1 | 261 | 13 | 159 | 13 | 0.55 |
| Qw | pg/ml | 1.0E-9 | 1.0E-9 | 1.9E0 | 1.1E1 | 9.3E0 | 3.6E1 | 1.0E-9 | 1.0E-9 | 1.2E2 | 1.3E2 | 261 | 13 | 159 | 13 | 0.46 |
| Qv | pg/ml | 2.3E4 | 2.1E4 | 3.6E4 | 3.7E4 | 7.8E4 | 4.4E4 | 6.0E1 | 3.5E3 | 9.4E5 | 1.7E5 | 261 | 13 | 159 | 13 | 0.51 |
| Qu | pg/ml | 7.8E0 | 1.6E1 | 8.6E1 | 7.6E1 | 1.8E2 | 1.3E2 | 1.0E-9 | 1.0E-9 | 9.8E2 | 4.1E2 | 261 | 13 | 159 | 13 | 0.51 |
| Qt | pg/ml | 1.0E1 | 9.3E0 | 5.0E1 | 3.5E1 | 1.3E2 | 6.1E1 | 1.0E-9 | 1.0E-9 | 1.0E3 | 2.0E2 | 261 | 13 | 159 | 13 | 0.51 |
| Qh | ng/ml | 1.6E1 | 2.4E1 | 3.8E1 | 3.8E1 | 6.6E1 | 4.3E1 | 1.0E-9 | 7.8E-1 | 6.4E2 | 1.5E2 | 261 | 13 | 159 | 13 | 0.53 |
| Qg | ng/ml | 7.8E0 | 1.1E1 | 1.6E1 | 1.6E1 | 2.6E1 | 1.7E1 | 5.1E-2 | 7.7E-1 | 2.2E2 | 6.3E1 | 261 | 13 | 159 | 13 | 0.57 |
| Jj | ng/ml | 6.3E2 | 5.0E2 | 1.8E3 | 9.1E2 | 1.3E4 | 1.4E3 | 1.5E0 | 1.2E1 | 3.4E5 | 6.8E3 | 756 | 23 | 296 | 23 | 0.44 |
| Jk | ng/ml | 3.0E0 | 2.2E0 | 1.9E1 | 2.7E1 | 4.1E1 | 5.5E1 | 1.0E-9 | 2.0E-1 | 2.8E2 | 2.2E2 | 756 | 23 | 296 | 23 | 0.50 |
| Jl | ng/ml | 3.8E-1 | 4.8E-1 | 1.8E0 | 2.5E0 | 4.6E0 | 5.6E0 | 7.6E-4 | 2.2E-2 | 4.0E1 | 2.0E1 | 756 | 23 | 296 | 23 | 0.51 |
| Jm | ng/ml | 1.6E1 | 2.4E1 | 5.9E1 | 5.6E1 | 1.4E2 | 6.8E1 | 1.0E-9 | 4.1E-1 | 2.1E3 | 2.4E2 | 756 | 23 | 296 | 23 | 0.56 |
| Jn | pg/ml | 4.0E-1 | 2.6E-1 | 3.2E0 | 5.9E-1 | 3.2E1 | 1.0E0 | 1.0E-9 | 1.0E-9 | 6.2E2 | 5.0E0 | 755 | 23 | 296 | 23 | 0.45 |
| Jo | pg/ml | 3.6E3 | 3.8E3 | 5.0E3 | 4.4E3 | 5.3E3 | 3.4E3 | 2.0E1 | 2.3E2 | 1.0E5 | 1.5E4 | 756 | 23 | 296 | 23 | 0.49 |
| Jp | pg/ml | 6.9E4 | 6.7E4 | 7.2E4 | 7.0E4 | 3.8E4 | 3.2E4 | 5.8E2 | 6.0E3 | 3.8E5 | 1.3E5 | 756 | 23 | 296 | 23 | 0.50 |
| Jq | pg/ml | 9.4E1 | 1.4E2 | 1.6E2 | 1.6E2 | 3.5E2 | 1.2E2 | 1.0E0 | 2.2E1 | 8.7E3 | 4.8E2 | 756 | 23 | 296 | 23 | 0.57 |
| Jr | pg/ml | 5.2E0 | 6.7E0 | 4.0E1 | 8.6E0 | 4.4E2 | 9.5E0 | 1.0E-9 | 1.0E-9 | 1.1E4 | 4.0E1 | 756 | 23 | 296 | 23 | 0.55 |
| Js | pg/ml | 1.3E1 | 8.5E0 | 5.5E1 | 1.9E1 | 4.0E2 | 2.8E1 | 1.0E-9 | 3.5E0 | 1.0E4 | 1.2E2 | 756 | 23 | 296 | 23 | 0.43 |
| Jt | pg/ml | 2.7E3 | 2.0E3 | 3.3E3 | 2.3E3 | 2.8E3 | 1.4E3 | 2.2E1 | 4.6E2 | 5.2E4 | 6.2E3 | 756 | 23 | 296 | 23 | 0.38 |
| Ju | mIU/ml | 9.1E0 | 5.3E0 | 2.0E1 | 9.3E0 | 3.1E1 | 9.5E0 | 4.8E-2 | 2.7E-1 | 2.3E2 | 2.9E1 | 262 | 13 | 160 | 13 | 0.41 |
| Jv | mIU/ml | 1.3E1 | 8.1E0 | 3.3E1 | 1.7E1 | 5.7E1 | 3.4E1 | 1.0E-2 | 9.4E-3 | 4.4E2 | 1.3E2 | 262 | 13 | 160 | 13 | 0.39 |
| Jy | ng/ml | 1.6E-3 | 1.8E-3 | 2.3E-3 | 2.2E-3 | 4.2E-3 | 1.3E-3 | 1.0E-9 | 1.0E-9 | 5.2E-2 | 4.4E-3 | 262 | 13 | 160 | 13 | 0.58 |
| Kc | pg/ml | 2.6E1 | 4.3E1 | 4.5E1 | 7.9E1 | 4.8E1 | 7.2E1 | 1.0E-9 | 6.9E0 | 2.7E2 | 1.9E2 | 264 | 13 | 159 | 13 | 0.65 |
| Kd | pg/ml | 1.0E-9 | 1.0E-9 | 3.3E2 | 5.3E2 | 2.4E3 | 1.2E3 | 1.0E-9 | 1.0E-9 | 3.8E4 | 3.7E3 | 264 | 13 | 159 | 13 | 0.57 |
| Ke | pg/ml | 1.3E4 | 9.6E3 | 1.6E4 | 1.3E4 | 2.2E4 | 8.5E3 | 3.4E2 | 2.2E3 | 3.2E5 | 3.1E4 | 264 | 13 | 159 | 13 | 0.47 |
| Kf | pg/mL | 7.4E0 | 8.9E0 | 7.8E0 | 8.5E0 | 7.4E0 | 6.8E0 | 1.0E-9 | 1.0E-9 | 7.8E1 | 2.1E1 | 264 | 13 | 159 | 13 | 0.54 |
| Kg | pg/mL | 1.2E3 | 1.1E3 | 2.0E3 | 1.7E3 | 2.9E3 | 2.2E3 | 7.7E1 | 3.0E2 | 2.7E4 | 8.5E3 | 264 | 13 | 159 | 13 | 0.47 |
| Ki | pg/ml | 5.9E1 | 8.8E1 | 6.8E1 | 9.6E1 | 5.2E1 | 7.1E1 | 1.0E-9 | 1.0E-9 | 3.8E2 | 2.9E2 | 263 | 13 | 159 | 13 | 0.66 |
| Kj | pg/ml | 9.8E2 | 7.6E2 | 1.7E3 | 1.4E3 | 1.9E3 | 1.5E3 | 3.0E1 | 2.1E2 | 1.5E4 | 5.2E3 | 264 | 13 | 159 | 13 | 0.43 |
| Kk | pg/ml | 6.8E0 | 1.7E1 | 1.2E1 | 2.5E1 | 1.6E1 | 2.3E1 | 1.0E-9 | 1.0E-9 | 1.6E2 | 6.4E1 | 264 | 13 | 159 | 13 | 0.66 |
| Kl | pg/ml | 2.1E4 | 3.3E4 | 2.8E4 | 3.3E4 | 2.6E4 | 3.3E4 | 2.3E2 | 1.0E3 | 1.6E5 | 1.1E5 | 264 | 13 | 159 | 13 | 0.51 |
| Kn | pg/ml | 3.0E1 | 3.0E1 | 8.1E1 | 5.3E1 | 3.1E2 | 6.5E1 | 1.0E-9 | 1.0E-9 | 4.9E3 | 1.9E2 | 264 | 13 | 159 | 13 | 0.48 |
| Ko | pg/ml | 4.1E2 | 3.2E2 | 5.2E2 | 7.0E2 | 5.2E2 | 8.3E2 | 1.0E-9 | 1.0E-9 | 4.1E3 | 2.5E3 | 264 | 13 | 159 | 13 | 0.52 |
| Kp | pg/ml | 3.4E2 | 2.8E2 | 4.1E2 | 4.5E2 | 8.4E2 | 3.6E2 | 1.0E-9 | 3.2E1 | 1.3E4 | 1.0E3 | 264 | 13 | 159 | 13 | 0.56 |
| Kq | pg/ml | 3.2E2 | 3.8E2 | 1.1E3 | 4.6E2 | 9.9E3 | 2.3E2 | 5.1E0 | 1.7E2 | 1.6E5 | 8.5E2 | 257 | 13 | 154 | 13 | 0.60 |
| Kr | pg/ml | 5.6E-1 | 1.0E-9 | 4.0E0 | 2.5E0 | 2.6E1 | 5.9E0 | 1.0E-9 | 1.0E-9 | 4.2E2 | 2.1E1 | 257 | 13 | 154 | 13 | 0.42 |
| Ks | pg/ml | 1.4E4 | 1.3E4 | 2.0E4 | 1.8E4 | 1.9E4 | 1.6E4 | 2.2E2 | 2.4E3 | 1.1E5 | 5.0E4 | 257 | 13 | 154 | 13 | 0.49 |
| Kx | ng/ml | 1.0E-9 | 6.8E-3 | 6.9E-3 | 8.1E-3 | 1.4E-2 | 8.3E-3 | 1.0E-9 | 1.0E-9 | 1.0E-1 | 2.7E-2 | 263 | 12 | 159 | 12 | 0.61 |
| Ky | ng/ml | 9.8E-2 | 1.6E-1 | 3.8E-1 | 3.9E-1 | 8.4E-1 | 6.3E-1 | 1.0E-9 | 1.0E-9 | 6.4E0 | 2.2E0 | 263 | 12 | 159 | 12 | 0.53 |
| Kz | ng/ml | 1.0E-9 | 1.0E-9 | 3.7E-3 | 3.5E-3 | 5.8E-3 | 5.4E-3 | 1.0E-9 | 1.0E-9 | 3.6E-2 | 1.4E-2 | 263 | 12 | 159 | 12 | 0.50 |
| Ld | pg/ml | 1.0E-9 | 3.6E0 | 3.7E0 | 4.4E0 | 9.2E0 | 4.5E0 | 1.0E-9 | 1.0E-9 | 8.4E1 | 1.5E1 | 264 | 13 | 158 | 13 | 0.66 |
| Lh | pg/ml | 1.3E4 | 1.2E4 | 2.1E4 | 2.2E4 | 3.3E4 | 2.2E4 | 1.0E-9 | 4.0E2 | 4.8E5 | 7.7E4 | 756 | 23 | 297 | 23 | 0.52 |
| Li | pg/ml | 3.2E3 | 4.9E3 | 1.7E4 | 1.7E4 | 6.6E4 | 4.8E4 | 1.0E-9 | 1.2E2 | 1.3E6 | 2.3E5 | 756 | 23 | 297 | 23 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Lj | pg/ml | 2.9E3 | 2.6E3 | 2.4E4 | 1.6E4 | 6.8E4 | 3.7E4 | 1.0E-9 | 1.5E2 | 5.2E5 | 1.7E5 | 756 | 23 | 297 | 23 | 0.50 |
| Rm | ng/ml | 1.9E1 | 2.7E1 | 5.2E1 | 4.6E1 | 8.4E1 | 4.5E1 | 2.2E-1 | 1.4E0 | 6.5E2 | 1.6E2 | 256 | 13 | 158 | 13 | 0.60 |
| Rh | ng/ml | 1.3E2 | 1.7E2 | 4.0E2 | 2.3E2 | 1.4E3 | 2.4E2 | 4.7E0 | 7.5E0 | 1.7E4 | 8.5E2 | 256 | 13 | 158 | 13 | 0.50 |
| Ri | ng/ml | 1.0E-9 | 1.0E-9 | 4.4E0 | 3.9E0 | 1.6E1 | 9.7E0 | 1.0E-9 | 1.0E-9 | 2.2E2 | 3.6E1 | 257 | 13 | 159 | 13 | 0.50 |
| Rg | ng/ml | 1.0E-9 | 1.0E-9 | 5.0E-2 | 5.8E-2 | 3.0E-1 | 1.9E-1 | 1.0E-9 | 1.0E-9 | 3.0E0 | 6.9E-1 | 256 | 13 | 158 | 13 | 0.56 |
| Rj | ng/ml | 1.0E-9 | 1.0E-9 | 2.4E0 | 3.2E-1 | 1.8E1 | 8.1E-1 | 1.0E-9 | 1.0E-9 | 2.7E2 | 2.5E0 | 257 | 13 | 159 | 13 | 0.43 |
| Rf | ng/ml | 4.1E-1 | 6.1E-1 | 1.0E0 | 8.4E-1 | 1.8E0 | 7.7E-1 | 7.8E-3 | 3.3E-2 | 1.5E1 | 2.6E0 | 256 | 13 | 158 | 13 | 0.54 |
| Ql | pg/ml | 4.5E0 | 7.3E0 | 1.3E1 | 1.6E1 | 2.4E1 | 3.6E1 | 1.0E-9 | 1.0E-9 | 1.8E2 | 1.3E2 | 262 | 13 | 160 | 13 | 0.50 |
| Qm | pg/ml | 4.1E0 | 1.0E-9 | 2.2E1 | 9.8E0 | 4.0E1 | 1.4E1 | 1.0E-9 | 1.0E-9 | 3.6E2 | 3.6E1 | 262 | 13 | 160 | 13 | 0.40 |
| Qn | pg/ml | 6.1E-1 | 5.9E-2 | 7.4E0 | 9.8E0 | 2.4E1 | 2.8E1 | 1.0E-9 | 1.0E-9 | 2.4E2 | 1.0E2 | 262 | 13 | 160 | 13 | 0.42 |
| Nv | pg/ml | 3.8E3 | 3.2E3 | 1.0E4 | 1.5E4 | 4.5E4 | 3.5E4 | 1.0E-9 | 3.2E2 | 1.1E6 | 1.6E5 | 762 | 23 | 297 | 23 | 0.52 |
| Nw | pg/ml | 8.8E3 | 1.3E4 | 1.3E4 | 1.4E4 | 1.7E4 | 1.0E4 | 8.6E1 | 9.3E2 | 2.1E5 | 3.7E4 | 762 | 23 | 297 | 23 | 0.58 |
| Nx | pg/ml | 2.2E2 | 2.2E2 | 4.1E2 | 5.4E2 | 6.6E2 | 6.0E2 | 1.0E-9 | 1.0E-9 | 7.4E3 | 1.9E3 | 762 | 23 | 297 | 23 | 0.58 |
| Ny | pg/ml | 6.0E0 | 1.8E0 | 6.1E1 | 6.7E1 | 9.2E2 | 2.5E2 | 1.0E-9 | 1.0E-9 | 2.5E4 | 1.2E3 | 762 | 23 | 297 | 23 | 0.49 |
| Oa | pg/ml | 1.8E2 | 3.5E2 | 4.4E2 | 6.8E2 | 7.4E2 | 8.7E2 | 1.0E-9 | 2.5E1 | 4.8E3 | 2.7E3 | 262 | 13 | 160 | 13 | 0.61 |
| Oe | pg/ml | 4.2E1 | 2.3E1 | 2.7E2 | 2.6E2 | 7.9E2 | 3.9E2 | 1.0E-9 | 1.0E-9 | 1.9E4 | 1.3E3 | 755 | 23 | 296 | 23 | 0.50 |
| Of | pg/ml | 1.6E2 | 9.7E1 | 5.3E3 | 9.5E3 | 2.9E4 | 2.7E4 | 1.0E-9 | 1.0E-9 | 6.2E5 | 1.2E5 | 761 | 23 | 297 | 23 | 0.47 |
| Og | pg/ml | 7.9E-2 | 7.9E-2 | 4.6E-1 | 1.7E-1 | 1.5E0 | 2.4E-1 | 1.0E-9 | 1.0E-9 | 2.0E1 | 9.6E-1 | 761 | 23 | 297 | 23 | 0.47 |
| Oh | pg/ml | 2.5E0 | 4.2E0 | 2.0E1 | 1.1E1 | 1.5E2 | 1.9E1 | 1.0E-9 | 3.0E-1 | 3.5E3 | 8.8E1 | 761 | 23 | 297 | 23 | 0.61 |
| Oi | pg/ml | 2.4E0 | 1.0E-9 | 5.9E0 | 7.5E0 | 9.6E0 | 1.2E1 | 1.0E-9 | 1.0E-9 | 8.6E1 | 4.9E1 | 761 | 23 | 297 | 23 | 0.48 |
| Ok | pg/ml | 3.9E2 | 4.9E2 | 5.3E2 | 7.3E2 | 5.7E2 | 7.5E2 | 1.3E1 | 4.3E1 | 7.8E3 | 3.1E3 | 761 | 23 | 297 | 23 | 0.56 |
| Om | pg/ml | 3.8E2 | 4.5E2 | 8.2E2 | 6.9E2 | 2.6E3 | 8.9E2 | 1.0E-9 | 1.0E-9 | 5.1E4 | 4.2E3 | 761 | 23 | 297 | 23 | 0.53 |
| On | pg/ml | 1.8E2 | 2.1E2 | 2.8E2 | 3.1E2 | 4.2E2 | 3.4E2 | 1.0E-9 | 1.0E-9 | 4.5E3 | 1.2E3 | 761 | 23 | 297 | 23 | 0.52 |
| Or | pg/ml | 1.4E1 | 1.2E1 | 3.5E1 | 3.3E1 | 6.7E1 | 5.3E1 | 1.0E-9 | 1.0E-9 | 5.1E2 | 1.8E2 | 265 | 13 | 159 | 13 | 0.51 |
| Ow | pg/ml | 3.3E1 | 2.4E1 | 1.5E2 | 7.1E1 | 5.7E2 | 1.1E2 | 1.0E-9 | 1.0E-9 | 8.1E3 | 4.0E2 | 265 | 13 | 159 | 13 | 0.43 |
| Ou | pg/ml | 5.1E2 | 2.8E2 | 9.5E2 | 1.0E3 | 1.5E3 | 2.0E3 | 1.0E-9 | 1.0E-9 | 9.8E3 | 7.5E3 | 265 | 13 | 159 | 13 | 0.42 |
| Ul | ng/ml | 1.0E-9 | 1.0E-9 | 1.8E0 | 1.0E-9 | 8.3E0 | 0.0E0 | 1.0E-9 | 1.0E-9 | 1.0E2 | 1.0E-9 | 270 | 13 | 163 | 13 | 0.45 |
| Uo | ng/ml | 1.0E-9 | 1.0E-9 | 9.7E-2 | 1.3E-1 | 2.2E-1 | 2.3E-1 | 1.0E-9 | 1.0E-9 | 1.5E0 | 5.8E-1 | 270 | 13 | 163 | 13 | 0.52 |
| Uk | ng/ml | 1.0E-9 | 1.0E-9 | 8.5E-3 | 2.5E-3 | 2.7E-2 | 6.6E-3 | 1.0E-9 | 1.0E-9 | 3.2E-1 | 2.4E-2 | 270 | 13 | 163 | 13 | 0.44 |
| Um | ng/ml | 1.0E-9 | 1.0E-9 | 4.2E-1 | 6.5E-1 | 9.1E-1 | 1.4E0 | 1.0E-9 | 1.0E-9 | 6.8E0 | 4.0E0 | 270 | 13 | 163 | 13 | 0.48 |
| Uf | ng/ml | 6.5E-2 | 6.8E-2 | 1.8E-1 | 2.0E-1 | 6.4E-1 | 4.5E-1 | 1.3E-3 | 5.0E-3 | 8.6E0 | 1.7E0 | 270 | 13 | 163 | 13 | 0.52 |
| Uh | ng/ml | 2.1E0 | 2.0E0 | 3.4E0 | 3.0E0 | 3.7E0 | 3.4E0 | 1.3E-2 | 6.3E-2 | 2.1E1 | 1.3E1 | 270 | 13 | 163 | 13 | 0.48 |
| Un | ng/ml | 1.9E0 | 2.7E0 | 2.2E0 | 2.7E0 | 1.9E0 | 1.3E0 | 1.3E-1 | 8.5E-1 | 2.5E1 | 5.2E0 | 270 | 13 | 163 | 13 | 0.65 |
| Ug | ng/ml | 1.5E1 | 9.7E0 | 2.9E1 | 2.0E1 | 3.2E1 | 3.2E1 | 6.9E-1 | 1.8E0 | 2.1E2 | 1.2E2 | 270 | 13 | 163 | 13 | 0.36 |
| Ur | ng/ml | 1.5E-1 | 1.7E-1 | 8.9E-1 | 2.9E-1 | 5.8E-1 | 4.8E-1 | 1.0E-9 | 1.0E-9 | 9.3E1 | 1.8E0 | 269 | 13 | 162 | 13 | 0.49 |
| Up | ng/ml | 1.0E-9 | 1.0E-9 | 1.4E-2 | 3.0E-3 | 1.5E-1 | 9.6E-3 | 1.0E-9 | 1.0E-9 | 2.4E0 | 3.5E-2 | 269 | 13 | 162 | 13 | 0.46 |
| Us | ng/ml | 2.9E-3 | 1.0E-9 | 2.6E-2 | 5.5E-3 | 1.1E-1 | 1.2E-2 | 1.0E-9 | 1.0E-9 | 1.7E0 | 4.4E-2 | 269 | 13 | 162 | 13 | 0.38 |
| Uv | ng/ml | 3.1E-3 | 1.9E-3 | 1.4E-2 | 2.1E-2 | 4.7E-2 | 6.1E-2 | 1.0E-9 | 1.0E-9 | 4.3E-1 | 2.2E-1 | 269 | 13 | 162 | 13 | 0.45 |
| Ut | ng/ml | 6.6E-1 | 3.4E-1 | 2.9E0 | 1.3E0 | 9.6E0 | 1.9E0 | 1.0E-9 | 6.8E-2 | 7.8E1 | 6.2E0 | 269 | 13 | 162 | 13 | 0.46 |
| Uu | ng/ml | 7.1E0 | 6.9E0 | 7.9E0 | 8.2E0 | 5.6E0 | 6.9E0 | 5.5E-1 | 1.2E0 | 4.0E1 | 2.3E1 | 269 | 13 | 162 | 13 | 0.48 |
| Vp | ng/ml | 1.0E-9 | 1.0E-9 | 5.6E-1 | 1.0E-9 | 4.6E0 | 0.0E0 | 1.0E-9 | 1.0E-9 | 5.0E1 | 1.0E-9 | 270 | 13 | 163 | 13 | 0.43 |
| Vt | ng/ml | 6.8E0 | 7.3E0 | 9.9E0 | 1.1E1 | 1.3E1 | 1.1E1 | 4.3E-1 | 1.7E0 | 1.6E2 | 4.2E1 | 270 | 13 | 163 | 13 | 0.54 |
| Vu | ng/ml | 1.0E-9 | 1.0E-9 | 2.1E0 | 1.2E0 | 5.4E0 | 1.8E0 | 1.0E-9 | 1.0E-9 | 5.1E1 | 5.3E0 | 264 | 13 | 162 | 13 | 0.52 |
| Vq | ng/ml | 2.7E2 | 9.7E1 | 4.3E3 | 1.0E3 | 4.8E4 | 1.7E3 | 2.0E-1 | 1.0E1 | 6.8E5 | 5.0E3 | 201 | 9 | 131 | 9 | 0.53 |
| Vo | ng/ml | 2.6E1 | 2.2E1 | 2.5E1 | 2.2E1 | 4.7E0 | 5.3E0 | 6.7E0 | 8.2E0 | 3.5E1 | 2.8E1 | 270 | 13 | 163 | 13 | 0.33 |
| Vs | ng/ml | 1.0E-9 | 6.3E-1 | 8.3E0 | 3.9E0 | 3.7E1 | 7.9E0 | 1.0E-9 | 1.0E-9 | 4.5E2 | 2.7E1 | 260 | 12 | 159 | 12 | 0.52 |
| Vv | ng/ml | 2.9E0 | 3.3E0 | 5.8E0 | 4.0E0 | 9.7E0 | 4.4E0 | 1.0E-9 | 1.0E-9 | 8.4E1 | 1.5E1 | 268 | 13 | 162 | 13 | 0.46 |
| Oy | pg/ml | 4.9E-1 | 3.3E-1 | 5.9E0 | 3.7E0 | 3.1E1 | 8.2E0 | 1.0E-9 | 1.0E-9 | 4.0E2 | 3.7E1 | 760 | 23 | 296 | 23 | 0.48 |
| Oz | pg/ml | 1.4E-3 | 1.5E-1 | 3.2E-1 | 2.1E-1 | 1.4E0 | 2.5E-1 | 1.0E-9 | 1.0E-9 | 2.9E1 | 8.2E-1 | 760 | 23 | 296 | 23 | 0.54 |
| Pa | pg/ml | 3.8E-1 | 1.9E-1 | 1.7E0 | 4.8E-1 | 6.5E0 | 7.4E-1 | 1.0E-9 | 1.0E-9 | 1.0E2 | 3.0E0 | 760 | 23 | 296 | 23 | 0.41 |
| Pb | pg/ml | 1.0E-9 | 1.0E-9 | 9.0E-1 | 1.3E0 | 1.8E1 | 5.8E0 | 1.0E-9 | 1.0E-9 | 4.9E2 | 2.8E1 | 760 | 23 | 296 | 23 | 0.49 |
| Pc | pg/ml | 4.2E-2 | 6.7E-2 | 3.6E-1 | 2.7E-1 | 9.3E-1 | 3.7E-1 | 1.0E-9 | 1.0E-9 | 1.6E1 | 1.4E0 | 760 | 23 | 296 | 23 | 0.51 |
| Pd | pg/ml | 1.9E0 | 1.4E0 | 5.4E0 | 2.4E0 | 3.2E1 | 2.6E0 | 1.0E-9 | 1.0E-9 | 8.4E2 | 9.4E0 | 760 | 23 | 296 | 23 | 0.45 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|--------|--------|--------|-----|
| | | NonDis | Dis | NonDis | Dis | NonDis | Dis | NonDis | Dis | NonDis | Dis | NonDis | Dis | NonDis | Dis | |
| Pe | pg/ml | 2.1E1 | 2.9E1 | 1.2E2 | 7.4E1 | 4.8E2 | 1.0E2 | 1.0E-9 | 1.0E-9 | 6.7E3 | 3.8E2 | 760 | 23 | 296 | 23 | 0.54 |
| Pf | pg/ml | 1.6E0 | 3.4E0 | 1.2E1 | 8.9E0 | 6.3E1 | 2.0E1 | 1.0E-9 | 1.0E-9 | 1.5E3 | 9.9E1 | 760 | 23 | 296 | 23 | 0.51 |
| Pg | pg/ml | 3.3E0 | 2.3E0 | 4.8E1 | 2.6E1 | 3.7E2 | 5.3E1 | 1.0E-9 | 1.0E-9 | 7.7E3 | 1.9E2 | 760 | 23 | 296 | 23 | 0.49 |
| Ph | ng/ml | 1.8E-1 | 1.7E-1 | 3.5E-1 | 5.1E-1 | 5.7E-1 | 7.2E-1 | 1.0E-9 | 1.9E-2 | 5.4E0 | 2.2E0 | 265 | 13 | 159 | 13 | 0.55 |
| Pi | ng/ml | 2.0E-1 | 2.5E-1 | 5.8E-1 | 2.2E-1 | 5.0E0 | 1.7E-1 | 1.0E-9 | 1.0E-9 | 8.2E1 | 5.4E-1 | 265 | 13 | 159 | 13 | 0.48 |
| Pj | ng/mL | 5.7E0 | 4.3E0 | 6.4E0 | 5.0E0 | 4.5E0 | 2.5E0 | 3.8E-2 | 1.3E0 | 3.1E1 | 9.9E0 | 265 | 13 | 159 | 13 | 0.43 |
| Pk | ng/ml | 8.8E-3 | 8.9E-3 | 1.8E-2 | 8.6E-3 | 9.4E-2 | 7.1E-3 | 1.0E-9 | 1.0E-9 | 1.5E0 | 2.6E-2 | 265 | 13 | 159 | 13 | 0.45 |
| aA | mg/dL | 8.0E-1 | 9.7E-1 | 9.0E-1 | 1.2E0 | 4.3E-1 | 7.7E-1 | 2.0E-1 | 4.5E-1 | 4.1E0 | 3.0E0 | 2356 | 29 | 452 | 29 | 0.60 |
| aC | mg/mL | 2.9E0 | 2.4E0 | 3.2E0 | 2.6E0 | 1.4E0 | 1.2E0 | 7.7E-1 | 7.4E-1 | 8.9E0 | 5.1E0 | 466 | 13 | 180 | 13 | 0.39 |
| aD | ug/mL | 3.2E0 | 3.0E0 | 4.5E0 | 4.8E0 | 4.0E0 | 5.3E0 | 4.3E-1 | 1.1E0 | 3.5E1 | 2.1E1 | 466 | 13 | 180 | 13 | 0.47 |
| aE | mg/mL | 5.6E-1 | 5.5E-1 | 5.7E-1 | 5.9E-1 | 1.5E-1 | 2.0E-1 | 1.8E-1 | 3.5E-1 | 1.1E0 | 1.0E0 | 466 | 13 | 180 | 13 | 0.48 |
| aF | ng/mL | 2.2E0 | 1.9E0 | 3.8E0 | 4.0E0 | 5.4E0 | 8.0E0 | 4.3E-3 | 3.5E-1 | 5.0E1 | 3.0E1 | 466 | 13 | 180 | 13 | 0.43 |
| aG | mg/mL | 1.3E-1 | 1.3E-1 | 1.5E-1 | 1.6E-1 | 8.7E-2 | 1.0E-1 | 1.7E-2 | 6.9E-2 | 5.4E-1 | 4.2E-1 | 466 | 13 | 180 | 13 | 0.51 |
| aH | ug/mL | 7.5E1 | 7.9E1 | 8.0E1 | 9.8E1 | 4.3E1 | 5.4E1 | 4.6E0 | 3.5E1 | 2.9E2 | 2.3E2 | 466 | 13 | 180 | 13 | 0.60 |
| aI | ug/mL | 1.9E2 | 2.0E2 | 1.9E2 | 1.9E2 | 6.0E1 | 5.5E1 | 2.8E1 | 1.1E2 | 3.7E2 | 2.7E2 | 466 | 13 | 180 | 13 | 0.52 |
| aJ | ug/mL | 2.4E0 | 4.4E0 | 3.0E0 | 4.2E0 | 2.0E0 | 2.0E0 | 7.3E-1 | 1.5E0 | 1.7E1 | 7.6E0 | 466 | 13 | 180 | 13 | 0.71 |
| aK | ng/mL | 1.5E0 | 1.9E0 | 2.4E0 | 2.1E0 | 2.6E0 | 1.6E0 | 2.9E-4 | 4.5E-1 | 1.8E1 | 6.1E0 | 466 | 13 | 180 | 13 | 0.54 |
| aL | mg/mL | 8.0E-1 | 8.8E-1 | 8.1E-1 | 8.9E-1 | 2.7E-1 | 2.2E-1 | 1.9E-1 | 5.7E-1 | 1.7E0 | 1.4E0 | 466 | 13 | 180 | 13 | 0.59 |
| aM | U/mL | 2.2E1 | 1.3E1 | 5.1E1 | 2.7E1 | 1.1E2 | 3.9E1 | 4.2E-2 | 4.2E-2 | 1.6E3 | 1.4E2 | 466 | 13 | 180 | 13 | 0.39 |
| aN | U/mL | 1.3E1 | 1.7E1 | 2.0E1 | 2.8E1 | 2.6E1 | 3.4E1 | 2.5E-3 | 5.0E0 | 3.3E2 | 1.3E2 | 466 | 13 | 180 | 13 | 0.58 |
| aO | pg/mL | 2.6E1 | 4.3E1 | 2.8E2 | 4.3E2 | 7.5E2 | 1.1E3 | 6.0E-2 | 2.1E0 | 6.6E3 | 3.9E3 | 466 | 13 | 180 | 13 | 0.56 |
| aP | ng/mL | 1.6E0 | 2.0E0 | 2.0E0 | 2.5E0 | 1.8E0 | 1.3E0 | 4.5E-1 | 1.2E0 | 2.8E1 | 5.4E0 | 466 | 13 | 180 | 13 | 0.66 |
| aQ | ng/mL | 2.9E-1 | 2.9E-1 | 4.4E-1 | 4.4E-1 | 4.6E-1 | 3.3E-1 | 2.0E-4 | 6.9E-2 | 4.0E0 | 1.1E0 | 466 | 13 | 180 | 13 | 0.54 |
| aR | ng/mL | 1.8E0 | 1.9E0 | 2.8E0 | 1.7E0 | 3.3E0 | 8.3E-1 | 1.8E-1 | 3.6E-1 | 3.4E1 | 2.7E0 | 466 | 13 | 180 | 13 | 0.43 |
| aS | ng/mL | 2.6E-1 | 4.8E-1 | 6.4E-1 | 6.4E-1 | 1.8E0 | 5.4E-1 | 4.2E-3 | 4.2E-3 | 3.3E1 | 1.7E0 | 466 | 13 | 180 | 13 | 0.61 |
| aU | pg/mL | 7.5E1 | 7.8E1 | 1.3E2 | 9.7E1 | 1.5E2 | 5.8E1 | 7.4E-2 | 3.5E1 | 1.3E3 | 2.2E2 | 466 | 13 | 180 | 13 | 0.53 |
| aV | ng/mL | 6.1E-1 | 5.9E-1 | 1.1E0 | 7.4E-1 | 2.0E0 | 6.1E-1 | 7.6E-4 | 1.2E-1 | 3.3E1 | 1.9E0 | 466 | 13 | 180 | 13 | 0.47 |
| aW | pg/mL | 1.8E1 | 2.1E1 | 1.9E1 | 3.5E1 | 1.9E1 | 5.8E1 | 7.2E-2 | 7.2E-2 | 2.4E2 | 2.3E2 | 466 | 13 | 180 | 13 | 0.59 |
| aX | ng/mL | 9.8E0 | 6.1E0 | 1.5E1 | 2.8E1 | 1.9E1 | 4.7E1 | 3.0E-1 | 2.5E0 | 2.2E2 | 1.7E2 | 466 | 13 | 180 | 13 | 0.50 |
| aY | pg/mL | 6.0E1 | 8.8E1 | 7.7E1 | 1.1E2 | 8.6E1 | 9.5E1 | 4.1E-1 | 2.5E1 | 1.2E3 | 3.9E2 | 466 | 13 | 180 | 13 | 0.65 |
| aZ | pg/mL | 2.3E2 | 1.9E2 | 4.9E2 | 4.0E2 | 9.2E2 | 5.1E2 | 1.7E0 | 3.2E1 | 1.2E4 | 1.8E3 | 466 | 13 | 180 | 13 | 0.50 |
| bA | ng/mL | 8.4E0 | 1.7E1 | 3.5E1 | 4.5E1 | 1.0E2 | 4.9E1 | 3.0E-2 | 2.9E-1 | 9.4E2 | 1.4E2 | 466 | 13 | 180 | 13 | 0.67 |
| bB | ng/mL | 3.0E2 | 4.2E2 | 3.1E2 | 3.9E2 | 1.6E2 | 2.1E2 | 2.1E0 | 6.2E1 | 8.2E2 | 6.9E2 | 466 | 13 | 180 | 13 | 0.62 |
| bC | ng/mL | 3.5E2 | 5.2E2 | 6.2E2 | 7.1E2 | 8.2E2 | 7.5E2 | 2.7E1 | 1.4E1 | 4.7E3 | 2.7E3 | 466 | 13 | 180 | 13 | 0.56 |
| bE | mg/mL | 5.6E0 | 6.1E0 | 5.8E0 | 6.5E0 | 2.1E0 | 1.6E0 | 9.8E-1 | 4.0E0 | 1.3E1 | 1.0E1 | 466 | 13 | 180 | 13 | 0.62 |
| bF | pg/mL | 2.1E1 | 1.5E1 | 1.6E2 | 4.0E2 | 9.3E2 | 1.4E3 | 5.0E-2 | 4.4E0 | 1.1E4 | 5.0E3 | 466 | 13 | 180 | 13 | 0.39 |
| bG | ng/mL | 1.6E0 | 1.1E0 | 2.7E0 | 1.5E0 | 3.2E0 | 1.4E0 | 2.2E-2 | 2.9E-1 | 2.3E1 | 4.8E0 | 466 | 13 | 180 | 13 | 0.38 |
| bH | pg/mL | 5.7E-1 | 5.7E-1 | 5.0E0 | 3.6E0 | 1.5E1 | 5.8E0 | 5.7E-1 | 5.7E-1 | 2.8E2 | 1.7E1 | 466 | 13 | 180 | 13 | 0.46 |
| bI | ng/mL | 4.0E-3 | 4.0E-3 | 6.5E-2 | 5.3E-2 | 1.6E-1 | 9.7E-2 | 4.0E-3 | 4.0E-3 | 1.3E0 | 2.9E-1 | 466 | 13 | 180 | 13 | 0.51 |
| bJ | mg/mL | 2.4E0 | 2.5E0 | 2.7E0 | 2.4E0 | 2.1E0 | 1.1E0 | 2.5E-4 | 7.0E-1 | 1.3E1 | 4.1E0 | 466 | 13 | 180 | 13 | 0.50 |
| bL | pg/mL | 3.7E0 | 6.3E0 | 8.4E0 | 1.0E1 | 1.1E1 | 1.2E1 | 4.6E-2 | 4.6E-2 | 8.0E1 | 4.0E1 | 466 | 13 | 180 | 13 | 0.54 |
| bM | mg/mL | 1.8E0 | 2.0E0 | 2.1E0 | 2.2E0 | 1.4E0 | 1.2E0 | 9.2E-3 | 1.0E0 | 8.8E0 | 5.3E0 | 466 | 13 | 180 | 13 | 0.55 |
| bN | ng/mL | 4.6E1 | 2.3E1 | 1.3E2 | 1.2E2 | 2.8E2 | 1.7E2 | 1.4E-1 | 4.9E-1 | 1.9E3 | 4.7E2 | 466 | 13 | 180 | 13 | 0.44 |
| bO | ng/mL | 4.0E-2 | 4.0E-2 | 1.1E1 | 1.5E1 | 2.5E1 | 2.1E1 | 4.0E-2 | 4.0E-2 | 2.0E2 | 5.0E1 | 466 | 13 | 180 | 13 | 0.57 |
| bP | mg/mL | 5.4E-1 | 6.6E-1 | 7.7E-1 | 8.6E-1 | 6.9E-1 | 7.5E-1 | 4.9E-2 | 9.7E-2 | 4.8E0 | 2.9E0 | 466 | 13 | 180 | 13 | 0.55 |
| bQ | pg/mL | 1.6E1 | 1.2E1 | 6.2E1 | 3.8E1 | 6.3E2 | 8.5E1 | 1.5E-1 | 4.4E0 | 1.3E4 | 3.2E2 | 466 | 13 | 180 | 13 | 0.41 |
| bR | ng/mL | 1.2E-2 | 1.0E-1 | 1.3E-1 | 1.4E-1 | 4.5E-1 | 1.5E-1 | 1.2E-2 | 1.2E-2 | 8.7E0 | 4.9E-1 | 466 | 13 | 180 | 13 | 0.58 |
| bS | pg/mL | 9.4E-1 | 9.4E-1 | 7.1E0 | 4.6E0 | 2.8E1 | 1.3E1 | 9.4E-1 | 9.4E-1 | 3.9E2 | 4.8E1 | 466 | 13 | 180 | 13 | 0.47 |
| bU | ng/mL | 1.2E-1 | 7.0E-2 | 2.0E-1 | 1.3E-1 | 3.7E-1 | 1.5E-1 | 1.3E-2 | 1.3E-2 | 6.6E0 | 5.0E-1 | 466 | 13 | 180 | 13 | 0.44 |
| bV | pg/mL | 4.6E2 | 5.0E2 | 5.5E2 | 6.6E2 | 5.8E2 | 3.3E2 | 1.5E2 | 3.1E2 | 1.2E4 | 1.2E3 | 466 | 13 | 180 | 13 | 0.61 |
| bW | pg/mL | 3.4E2 | 4.1E2 | 6.4E2 | 5.6E2 | 1.7E3 | 5.1E2 | 8.4E1 | 1.8E2 | 2.5E4 | 2.2E3 | 466 | 13 | 180 | 13 | 0.58 |
| bX | ng/mL | 6.9E-4 | 2.5E-5 | 2.7E-3 | 1.8E-3 | 3.4E-3 | 2.3E-3 | 2.5E-5 | 2.5E-5 | 2.0E-2 | 6.9E-3 | 466 | 13 | 180 | 13 | 0.44 |
| bZ | pg/mL | 2.3E2 | 2.7E2 | 8.5E2 | 1.3E3 | 4.0E3 | 3.1E3 | 1.5E-1 | 1.1E2 | 5.8E4 | 1.2E4 | 466 | 13 | 180 | 13 | 0.54 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|-----|---------|-----|------|
| | | NonDis | Dis | NonDis | Dis | NonDis | Dis | NonDis | Dis | NonDis | Dis | NonDis | Dis | NonDis | Dis | |
| cA | pg/mL | 6.0E-1 | 6.0E-1 | 2.8E0 | 3.1E0 | 1.8E1 | 6.9E0 | 6.0E-1 | 6.0E-1 | 3.7E2 | 2.5E1 | 466 | 13 | 180 | 13 | 0.51 |
| cB | ng/mL | 5.7E-2 | 4.6E-2 | 9.0E-2 | 6.7E-2 | 1.0E-1 | 8.8E-2 | 1.7E-3 | 1.7E-3 | 5.7E-1 | 2.7E-1 | 466 | 13 | 180 | 13 | 0.40 |
| cC | pg/mL | 4.6E1 | 3.4E1 | 4.8E1 | 3.6E1 | 4.0E1 | 2.0E1 | 1.0E0 | 1.0E0 | 4.5E2 | 6.9E1 | 466 | 13 | 180 | 13 | 0.40 |
| cD | pg/mL | 5.6E0 | 4.2E0 | 1.3E1 | 3.6E1 | 4.9E1 | 8.4E1 | 3.3E-1 | 3.3E-1 | 7.2E2 | 3.1E2 | 466 | 13 | 180 | 13 | 0.50 |
| cE | pg/mL | 3.7E1 | 3.3E1 | 1.7E2 | 2.0E2 | 4.8E2 | 4.6E2 | 1.2E-1 | 2.6E0 | 3.8E3 | 1.7E3 | 466 | 13 | 180 | 13 | 0.48 |
| cF | pg/mL | 1.3E1 | 1.3E1 | 2.0E1 | 1.3E1 | 3.0E1 | 1.3E1 | 5.3E-1 | 5.3E-1 | 2.7E2 | 4.1E1 | 466 | 13 | 180 | 13 | 0.46 |
| cG | pg/mL | 4.5E1 | 3.7E1 | 1.1E2 | 7.6E1 | 5.1E2 | 9.0E1 | 6.4E0 | 1.7E1 | 1.0E4 | 2.8E2 | 466 | 13 | 180 | 13 | 0.44 |
| cH | uIU/mL | 2.8E0 | 3.3E0 | 5.8E0 | 6.8E0 | 1.1E1 | 1.1E1 | 8.6E-3 | 4.8E-1 | 1.6E2 | 4.2E1 | 466 | 13 | 180 | 13 | 0.51 |
| cI | ng/mL | 5.5E0 | 1.3E1 | 1.1E1 | 3.0E1 | 1.5E1 | 5.2E1 | 1.0E-3 | 3.5E-1 | 1.2E2 | 1.9E2 | 466 | 13 | 180 | 13 | 0.60 |
| cJ | ug/mL | 5.6E1 | 8.2E1 | 1.1E2 | 1.4E2 | 1.4E2 | 1.7E2 | 4.0E0 | 8.6E0 | 9.6E2 | 6.6E2 | 466 | 13 | 180 | 13 | 0.58 |
| cK | ng/mL | 3.8E-3 | 3.8E-3 | 5.1E-2 | 1.3E-2 | 1.8E-1 | 3.2E-2 | 3.8E-3 | 3.8E-3 | 1.7E0 | 1.2E-1 | 466 | 13 | 180 | 13 | 0.46 |
| cL | pg/mL | 2.0E2 | 1.7E2 | 3.7E2 | 4.9E2 | 1.3E3 | 7.7E2 | 1.6E1 | 1.2E2 | 2.4E4 | 2.9E3 | 466 | 13 | 180 | 13 | 0.53 |
| cM | pg/mL | 2.6E2 | 2.6E2 | 2.9E2 | 3.0E2 | 2.0E2 | 1.6E2 | 8.7E0 | 1.2E2 | 1.6E3 | 6.1E2 | 466 | 13 | 180 | 13 | 0.52 |
| cN | pg/mL | 1.2E2 | 1.6E2 | 1.3E2 | 1.6E2 | 6.5E1 | 5.3E1 | 3.8E1 | 9.5E1 | 1.1E3 | 2.7E2 | 466 | 13 | 180 | 13 | 0.75 |
| cO | pg/mL | 2.3E2 | 2.1E2 | 3.2E2 | 2.8E2 | 9.0E2 | 1.7E2 | 5.4E1 | 9.0E1 | 1.9E4 | 7.2E2 | 466 | 13 | 180 | 13 | 0.49 |
| cP | ng/mL | 2.5E3 | 3.1E3 | 2.6E3 | 3.1E3 | 9.1E2 | 1.1E3 | 6.2E2 | 1.7E3 | 5.7E3 | 5.0E3 | 466 | 13 | 180 | 13 | 0.63 |
| cQ | ng/mL | 5.3E-2 | 6.2E-2 | 1.5E-1 | 1.6E-1 | 3.0E-1 | 2.6E-1 | 2.0E-3 | 2.0E-3 | 2.2E0 | 8.5E-1 | 466 | 13 | 180 | 13 | 0.54 |
| cR | ng/mL | 3.0E2 | 3.2E2 | 5.2E2 | 3.8E2 | 8.2E2 | 2.9E2 | 2.0E1 | 9.4E1 | 8.9E3 | 1.0E3 | 466 | 13 | 180 | 13 | 0.49 |
| cS | ng/mL | 2.5E2 | 3.6E2 | 4.0E2 | 4.7E2 | 4.0E2 | 3.0E2 | 4.1E1 | 1.1E2 | 2.7E3 | 1.1E3 | 466 | 13 | 180 | 13 | 0.63 |
| cT | ng/mL | 3.3E1 | 7.0E1 | 8.4E1 | 9.5E1 | 1.9E2 | 9.3E1 | 3.7E0 | 7.6E0 | 2.1E3 | 3.1E2 | 466 | 13 | 180 | 13 | 0.62 |
| cU | ng/mL | 5.3E1 | 7.0E1 | 7.6E1 | 7.7E1 | 1.0E2 | 3.6E1 | 5.4E0 | 3.1E1 | 1.6E3 | 1.5E2 | 466 | 13 | 180 | 13 | 0.62 |
| cV | ng/mL | 1.8E-1 | 1.4E-1 | 4.0E-1 | 2.2E-1 | 2.2E0 | 2.0E-1 | 2.5E-2 | 8.8E-2 | 4.7E1 | 7.5E-1 | 466 | 13 | 180 | 13 | 0.45 |
| cW | mIU/mL | 5.3E-2 | 8.2E-2 | 1.5E-1 | 7.4E-2 | 7.0E-1 | 4.6E-2 | 3.7E-4 | 1.0E-2 | 9.7E0 | 1.7E-1 | 466 | 13 | 180 | 13 | 0.56 |
| cX | ng/mL | 1.1E-1 | 4.3E-2 | 1.2E0 | 4.1E-1 | 3.8E0 | 7.1E-1 | 2.3E-4 | 2.3E-4 | 2.8E1 | 2.4E0 | 466 | 13 | 180 | 13 | 0.46 |
| cY | ng/mL | 8.6E0 | 7.4E0 | 1.3E1 | 1.0E1 | 1.3E1 | 7.2E0 | 1.5E-1 | 1.6E0 | 8.3E1 | 2.3E1 | 466 | 13 | 180 | 13 | 0.49 |
| cZ | ug/mL | 1.5E1 | 1.9E1 | 1.6E1 | 2.0E1 | 7.4E0 | 6.1E0 | 2.3E0 | 1.3E1 | 5.7E1 | 3.1E1 | 466 | 13 | 180 | 13 | 0.69 |
| dA | pg/mL | 3.2E2 | 4.3E2 | 3.7E2 | 4.3E2 | 3.1E2 | 1.8E2 | 9.0E1 | 1.9E2 | 5.8E3 | 9.0E2 | 466 | 13 | 180 | 13 | 0.64 |
| dB | ug/mL | 1.7E1 | 2.2E1 | 1.8E1 | 1.7E1 | 1.6E1 | 1.0E1 | 9.4E-1 | 2.8E0 | 2.5E2 | 3.0E1 | 466 | 13 | 180 | 13 | 0.58 |
| dC | nmol/L | 3.5E1 | 3.2E1 | 3.8E1 | 3.8E1 | 1.8E1 | 2.1E1 | 7.6E0 | 1.7E1 | 1.4E2 | 7.9E1 | 466 | 13 | 180 | 13 | 0.46 |
| dD | ug/mL | 3.6E1 | 3.3E1 | 3.7E1 | 3.6E1 | 1.1E1 | 7.6E0 | 1.3E1 | 2.7E1 | 7.6E1 | 5.1E1 | 466 | 13 | 180 | 13 | 0.46 |
| dE | ng/mL | 4.8E-1 | 4.8E-1 | 6.1E-1 | 5.6E-1 | 7.0E-1 | 6.0E-1 | 8.4E-3 | 8.4E-3 | 7.2E0 | 2.0E0 | 466 | 13 | 180 | 13 | 0.48 |
| dF | ng/mL | 2.3E2 | 2.4E2 | 2.8E2 | 3.1E2 | 1.9E2 | 2.6E2 | 5.6E1 | 1.2E2 | 1.3E3 | 1.1E3 | 466 | 13 | 180 | 13 | 0.51 |
| dG | ng/mL | 1.1E1 | 1.7E1 | 1.5E1 | 1.9E1 | 1.4E1 | 1.2E1 | 2.2E0 | 7.0E0 | 1.8E2 | 5.2E1 | 466 | 13 | 180 | 13 | 0.67 |
| dH | pg/mL | 7.5E0 | 1.2E1 | 1.3E1 | 1.9E1 | 3.9E1 | 2.6E1 | 4.0E-2 | 2.2E0 | 6.7E2 | 9.7E1 | 466 | 13 | 180 | 13 | 0.60 |
| dI | pg/mL | 4.6E-1 | 4.6E-1 | 2.3E0 | 1.6E0 | 1.6E1 | 2.5E0 | 4.6E-1 | 4.6E-1 | 3.3E2 | 9.5E0 | 466 | 13 | 180 | 13 | 0.54 |
| dJ | ng/mL | 1.9E0 | 2.4E0 | 2.2E0 | 2.5E0 | 1.1E0 | 1.3E0 | 3.2E-2 | 9.4E-1 | 6.9E0 | 5.2E0 | 466 | 13 | 180 | 13 | 0.58 |
| dK | uIU/mL | 1.9E0 | 1.7E0 | 3.1E0 | 2.4E0 | 6.5E0 | 2.5E0 | 2.8E-4 | 4.2E-2 | 7.9E1 | 1.0E1 | 466 | 13 | 180 | 13 | 0.50 |
| dL | ng/mL | 8.8E2 | 1.2E3 | 1.0E3 | 1.1E3 | 5.9E2 | 3.2E2 | 2.6E2 | 5.7E2 | 4.8E3 | 1.6E3 | 466 | 13 | 180 | 13 | 0.62 |
| dM | pg/mL | 9.5E2 | 1.2E3 | 1.2E3 | 1.8E3 | 1.4E3 | 1.4E3 | 3.4E2 | 7.2E2 | 1.6E4 | 5.9E3 | 466 | 13 | 180 | 13 | 0.66 |
| dN | ug/mL | 9.4E1 | 1.0E2 | 1.0E2 | 1.2E2 | 4.1E1 | 5.3E1 | 1.6E1 | 5.8E1 | 3.3E2 | 2.2E2 | 466 | 13 | 180 | 13 | 0.63 |
| dR | pg/ml | 1.6E3 | 2.0E3 | 2.4E3 | 2.6E3 | 2.4E3 | 2.2E3 | 1.4E2 | 5.5E2 | 1.5E4 | 8.7E3 | 312 | 12 | 171 | 12 | 0.57 |
| eF | ng/ml | 4.1E0 | 4.3E0 | 5.0E0 | 5.1E0 | 4.2E0 | 2.2E0 | 1.2E0 | 2.1E0 | 4.6E1 | 8.6E0 | 324 | 12 | 172 | 12 | 0.56 |
| eC | pg/ml | 3.0E2 | 2.7E2 | 3.8E2 | 3.2E2 | 2.9E2 | 1.5E2 | 9.9E0 | 1.3E2 | 2.0E3 | 6.2E2 | 246 | 11 | 160 | 11 | 0.46 |
| eD | pg/ml | 2.1E2 | 2.6E2 | 5.0E2 | 3.0E2 | 1.1E3 | 2.8E2 | 5.2E-1 | 4.5E1 | 8.3E3 | 9.9E2 | 196 | 9 | 131 | 9 | 0.53 |
| fP | ng/ml | 2.6E2 | 3.3E2 | 2.9E2 | 3.3E2 | 1.7E2 | 2.4E2 | 8.4E0 | 8.1E1 | 1.0E3 | 9.5E2 | 298 | 13 | 163 | 13 | 0.54 |
| gL | pg/ml | 6.4E4 | 6.6E4 | 7.0E4 | 7.7E4 | 2.9E4 | 3.5E4 | 1.4E4 | 4.4E4 | 2.0E5 | 1.5E5 | 312 | 12 | 171 | 12 | 0.54 |
| gP | U/ml | 2.7E2 | 2.5E2 | 2.7E2 | 2.7E2 | 9.4E1 | 7.9E1 | 1.2E1 | 1.5E2 | 8.0E2 | 4.4E2 | 320 | 12 | 172 | 12 | 0.49 |
| gW | ng/ml | 6.1E2 | 9.8E2 | 1.3E3 | 1.3E3 | 1.7E3 | 1.5E3 | 3.1E-1 | 3.3E1 | 9.5E3 | 5.4E3 | 273 | 11 | 163 | 11 | 0.56 |
| tF | pg/mL | 1.5E3 | 1.0E3 | 1.2E4 | 1.6E3 | 3.8E4 | 2.0E3 | 1.2E1 | 1.8E1 | 3.2E5 | 5.8E3 | 246 | 11 | 160 | 11 | 0.41 |
| hA | ng/ml | 2.1E0 | 2.5E0 | 9.9E0 | 1.0E1 | 3.7E1 | 2.0E1 | 1.7E-2 | 1.7E-2 | 3.5E2 | 6.1E1 | 197 | 9 | 131 | 9 | 0.54 |
| nM | pg/ml | 1.0E-9 | 1.0E-9 | 7.7E1 | 1.0E-9 | 8.9E2 | 0.0E0 | 1.0E-9 | 1.0E-9 | 1.0E4 | 1.0E-9 | 133 | 8 | 101 | 8 | 0.49 |
| nN | pg/ml | 1.2E3 | 3.3E3 | 4.2E3 | 2.9E3 | 2.3E4 | 2.4E3 | 1.1E2 | 1.1E2 | 2.7E5 | 7.1E3 | 133 | 8 | 101 | 8 | 0.60 |
| nO | pg/ml | 2.6E1 | 2.8E1 | 4.5E1 | 4.8E1 | 5.3E1 | 4.8E1 | 3.5E0 | 9.6E0 | 3.1E2 | 1.4E2 | 133 | 8 | 101 | 8 | 0.50 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| nR | pg/ml | 1.5E1 | 9.6E0 | 3.2E1 | 9.0E1 | 4.5E1 | 1.5E2 | 1.0E-9 | 8.6E-1 | 2.6E2 | 3.6E2 | 133 | 8 | 101 | 8 | 0.46 |
| nT | pg/ml | 8.0E1 | 7.0E1 | 2.0E2 | 1.2E2 | 7.9E2 | 1.5E2 | 1.0E-9 | 2.3E1 | 6.6E3 | 4.9E2 | 133 | 8 | 101 | 8 | 0.48 |
| nU | pg/ml | 2.9E1 | 1.2E2 | 2.5E2 | 2.0E2 | 1.4E3 | 2.4E2 | 1.0E-9 | 1.0E-9 | 1.3E4 | 7.5E2 | 133 | 8 | 101 | 8 | 0.71 |
| lW | pg/ml | 1.0E-9 | 1.0E-9 | 1.4E1 | 4.0E0 | 4.9E1 | 8.3E0 | 1.0E-9 | 1.0E-9 | 3.9E2 | 2.3E1 | 133 | 8 | 101 | 8 | 0.48 |
| lX | pg/ml | 9.5E2 | 8.9E2 | 1.0E3 | 9.6E2 | 5.8E2 | 3.7E2 | 1.2E2 | 5.1E2 | 2.6E3 | 1.5E3 | 133 | 8 | 101 | 8 | 0.49 |
| lY | pg/ml | 1.9E1 | 1.6E1 | 2.2E1 | 1.9E1 | 1.9E1 | 1.2E1 | 1.0E-9 | 4.8E0 | 1.4E2 | 3.9E1 | 133 | 8 | 101 | 8 | 0.44 |
| mE | pg/ml | 1.0E-9 | 1.0E-9 | 2.9E0 | 1.8E0 | 8.5E0 | 3.0E0 | 1.0E-9 | 1.0E-9 | 5.8E1 | 7.9E0 | 133 | 8 | 101 | 8 | 0.50 |
| mF | pg/ml | 1.0E-9 | 4.3E-1 | 2.4E0 | 1.2E0 | 9.4E0 | 1.8E0 | 1.0E-9 | 1.0E-9 | 1.1E2 | 5.4E0 | 133 | 8 | 101 | 8 | 0.51 |
| mH | pg/ml | 3.6E0 | 2.5E0 | 5.3E0 | 2.4E0 | 6.6E0 | 6.9E-1 | 2.3E-1 | 1.4E0 | 5.3E1 | 3.5E0 | 133 | 8 | 101 | 8 | 0.36 |
| mI | pg/ml | 1.0E-9 | 1.0E-9 | 1.5E1 | 1.3E1 | 3.1E1 | 2.7E1 | 1.0E-9 | 1.0E-9 | 1.8E2 | 7.6E1 | 133 | 8 | 101 | 8 | 0.46 |
| mM | pg/ml | 2.2E1 | 2.5E1 | 6.9E1 | 6.3E1 | 1.3E2 | 8.9E1 | 1.0E-9 | 1.0E-9 | 9.8E2 | 2.3E2 | 133 | 8 | 101 | 8 | 0.51 |
| mP | pg/ml | 1.4E1 | 2.3E1 | 1.8E1 | 2.7E1 | 2.2E1 | 1.7E1 | 1.0E-9 | 1.3E1 | 1.9E2 | 6.4E1 | 132 | 8 | 100 | 8 | 0.76 |
| mS | pg/ml | 1.6E3 | 1.9E3 | 1.8E3 | 1.9E3 | 1.6E3 | 8.8E2 | 1.0E-9 | 7.3E2 | 1.3E4 | 3.0E3 | 133 | 8 | 101 | 8 | 0.58 |
| mT | pg/ml | 5.4E1 | 3.9E1 | 1.3E2 | 1.3E2 | 2.2E2 | 2.7E2 | 9.7E0 | 1.8E1 | 1.4E3 | 8.0E2 | 132 | 8 | 100 | 8 | 0.42 |
| mU | pg/ml | 2.2E0 | 2.6E0 | 4.0E0 | 4.9E0 | 8.3E0 | 6.0E0 | 1.0E-9 | 1.3E0 | 6.8E1 | 1.9E1 | 132 | 8 | 100 | 8 | 0.58 |
| mW | pg/ml | 2.3E3 | 3.1E3 | 2.7E3 | 3.1E3 | 2.1E3 | 1.4E3 | 1.0E-9 | 1.4E3 | 1.8E4 | 5.4E3 | 132 | 8 | 100 | 8 | 0.63 |
| mY | pg/ml | 5.5E2 | 5.4E2 | 8.5E2 | 9.3E2 | 1.3E3 | 8.8E2 | 1.0E-9 | 1.5E2 | 1.1E4 | 2.6E3 | 133 | 8 | 101 | 8 | 0.54 |
| mZ | pg/ml | 1.7E2 | 4.9E2 | 2.9E2 | 6.2E2 | 3.0E2 | 4.7E2 | 1.0E-9 | 1.2E2 | 1.5E3 | 1.4E3 | 132 | 8 | 100 | 8 | 0.76 |
| nA | pg/ml | 1.6E0 | 3.9E0 | 1.0E1 | 1.0E1 | 4.3E1 | 1.9E1 | 1.0E-9 | 1.0E-9 | 4.4E2 | 5.7E1 | 132 | 8 | 100 | 8 | 0.58 |
| nB | pg/ml | 3.1E2 | 3.8E2 | 3.2E2 | 4.3E2 | 1.7E2 | 2.5E2 | 3.0E1 | 2.1E2 | 9.1E2 | 1.0E3 | 133 | 8 | 101 | 8 | 0.65 |
| nC | pg/ml | 1.0E-9 | 1.0E-9 | 2.5E4 | 2.5E3 | 1.5E5 | 7.1E3 | 1.0E-9 | 1.0E-9 | 1.5E6 | 2.0E4 | 133 | 8 | 101 | 8 | 0.36 |
| nD | pg/ml | 7.9E0 | 1.2E1 | 3.6E1 | 2.4E1 | 2.0E2 | 3.8E1 | 1.0E-9 | 3.1E0 | 1.7E3 | 1.2E2 | 132 | 8 | 100 | 8 | 0.65 |
| nF | pg/ml | 1.0E-9 | 8.3E0 | 4.5E0 | 1.5E1 | 2.5E1 | 1.9E1 | 1.0E-9 | 1.0E-9 | 2.4E2 | 4.7E1 | 133 | 8 | 101 | 8 | 0.75 |
| nH | pg/ml | 1.0E0 | 1.0E-9 | 2.2E2 | 4.1E1 | 1.3E3 | 1.2E2 | 1.0E-9 | 1.0E-9 | 1.0E4 | 3.3E2 | 132 | 8 | 100 | 8 | 0.36 |
| nI | pg/ml | 3.0E1 | 6.4E1 | 1.5E2 | 1.9E2 | 8.3E2 | 4.1E2 | 1.0E-9 | 1.0E-9 | 9.2E3 | 1.2E3 | 133 | 8 | 101 | 8 | 0.58 |
| nJ | pg/ml | 5.9E-2 | 1.6E0 | 4.1E1 | 2.9E0 | 4.5E2 | 5.0E0 | 1.0E-9 | 1.0E-9 | 5.2E3 | 1.5E1 | 133 | 8 | 101 | 8 | 0.70 |
| nK | pg/ml | 1.0E-9 | 1.0E-9 | 5.6E1 | 3.8E1 | 3.4E2 | 7.7E1 | 1.0E-9 | 1.0E-9 | 3.2E3 | 2.2E2 | 132 | 8 | 100 | 8 | 0.51 |
| nL | pg/ml | 1.0E-9 | 1.0E-9 | 5.9E2 | 1.1E2 | 4.1E3 | 3.2E2 | 1.0E-9 | 1.0E-9 | 4.5E4 | 9.0E2 | 133 | 8 | 101 | 8 | 0.38 |
| hR | pg/ml | 2.6E4 | 2.3E4 | 2.7E4 | 2.4E4 | 1.1E4 | 7.3E3 | 1.0E-9 | 1.4E4 | 5.8E4 | 3.7E4 | 187 | 9 | 127 | 9 | 0.41 |
| hV | pg/ml | 4.4E2 | 5.1E2 | 4.6E2 | 4.5E2 | 2.4E2 | 2.4E2 | 1.0E-9 | 1.3E2 | 1.5E3 | 7.9E2 | 187 | 9 | 127 | 9 | 0.49 |
| hW | pg/ml | 1.6E3 | 1.7E3 | 2.1E3 | 1.8E3 | 3.0E3 | 7.6E2 | 1.0E-9 | 7.1E2 | 4.0E4 | 3.3E3 | 187 | 9 | 127 | 9 | 0.51 |
| hX | pg/ml | 9.0E2 | 1.1E3 | 1.0E3 | 1.1E3 | 7.3E2 | 3.2E2 | 2.5E0 | 6.0E2 | 8.6E3 | 1.5E3 | 187 | 9 | 127 | 9 | 0.58 |
| iA | pg/ml | 1.5E2 | 1.5E2 | 3.0E2 | 2.1E2 | 6.1E2 | 1.8E2 | 8.2E0 | 4.2E1 | 7.1E3 | 6.8E2 | 246 | 11 | 160 | 11 | 0.52 |
| iB | ng/ml | 4.8E0 | 7.2E0 | 6.1E0 | 7.5E0 | 5.0E0 | 5.6E0 | 3.3E-2 | 1.9E0 | 2.6E1 | 1.9E1 | 197 | 9 | 131 | 9 | 0.58 |
| iC | U/ml | 2.3E-1 | 3.5E-1 | 1.1E0 | 3.6E-1 | 4.8E0 | 3.1E-1 | 1.0E-9 | 1.0E-9 | 5.5E1 | 7.7E-1 | 197 | 9 | 131 | 9 | 0.51 |
| iH | ng/ml | 1.6E5 | 1.6E5 | 1.6E5 | 1.5E5 | 4.8E4 | 5.9E4 | 2.9E3 | 5.1E4 | 2.7E5 | 2.1E5 | 246 | 11 | 160 | 11 | 0.46 |
| iJ | ng/ml | 4.9E4 | 5.7E4 | 5.2E4 | 6.7E4 | 2.6E4 | 4.4E4 | 1.8E3 | 1.1E4 | 2.5E5 | 1.8E5 | 246 | 11 | 160 | 11 | 0.60 |
| hB | ng/ml | 4.5E-1 | 3.2E-1 | 5.6E-1 | 6.0E-1 | 4.5E-1 | 6.1E-1 | 1.0E-9 | 2.0E-1 | 3.2E0 | 1.9E0 | 246 | 11 | 160 | 11 | 0.42 |
| hC | pg/ml | 3.8E3 | 3.4E3 | 6.8E3 | 6.7E3 | 8.7E3 | 7.3E3 | 1.0E-9 | 6.2E1 | 5.7E4 | 2.0E4 | 246 | 11 | 160 | 11 | 0.51 |
| hF | ng/ml | 1.0E-9 | 1.0E-9 | 2.0E1 | 1.0E-9 | 2.6E2 | 0.0E0 | 1.0E-9 | 1.0E-9 | 4.0E3 | 1.0E-9 | 246 | 11 | 160 | 11 | 0.49 |
| hG | pg/ml | 6.8E3 | 8.7E3 | 7.2E3 | 9.9E3 | 3.0E3 | 4.1E3 | 2.8E1 | 4.8E3 | 2.0E4 | 1.8E4 | 246 | 11 | 160 | 11 | 0.69 |
| iO | ng/ml | 3.7E5 | 4.0E5 | 3.9E5 | 3.9E5 | 1.8E5 | 1.5E5 | 1.1E4 | 1.3E5 | 1.1E6 | 6.2E5 | 246 | 11 | 160 | 11 | 0.52 |
| iP | ng/ml | 4.8E4 | 5.5E4 | 5.6E4 | 5.0E4 | 5.6E4 | 2.1E4 | 1.0E-9 | 1.9E4 | 5.7E5 | 7.7E4 | 246 | 11 | 160 | 11 | 0.49 |
| iZ | ng/ml | 1.6E3 | 2.1E3 | 1.8E3 | 2.3E3 | 7.4E2 | 7.6E2 | 4.7E2 | 1.5E3 | 5.1E3 | 4.2E3 | 247 | 11 | 160 | 11 | 0.71 |
| rC | pg/ml | 1.7E3 | 2.3E3 | 2.1E3 | 3.3E3 | 1.8E3 | 2.8E3 | 1.0E-9 | 2.5E2 | 1.5E4 | 8.4E3 | 187 | 10 | 127 | 10 | 0.61 |
| rB | pg/ml | 2.6E1 | 1.8E1 | 4.6E1 | 3.0E1 | 8.9E1 | 3.1E1 | 1.0E-9 | 1.0E-9 | 9.5E2 | 7.9E1 | 187 | 10 | 127 | 10 | 0.43 |
| jD | ng/ml | 3.2E1 | 1.8E1 | 4.9E1 | 2.2E1 | 6.3E1 | 2.6E1 | 1.0E-9 | 1.0E-9 | 5.1E2 | 8.4E1 | 196 | 9 | 131 | 9 | 0.35 |
| jE | ng/ml | 1.0E-9 | 1.0E-9 | 6.9E0 | 6.0E0 | 1.6E1 | 1.3E1 | 1.0E-9 | 1.0E-9 | 1.2E2 | 3.6E1 | 196 | 9 | 131 | 9 | 0.50 |
| jF | ng/ml | 3.9E1 | 1.6E1 | 5.2E1 | 5.1E1 | 5.8E1 | 6.4E1 | 1.0E-9 | 1.0E-9 | 3.5E2 | 1.5E2 | 196 | 9 | 131 | 9 | 0.46 |
| jG | ng/ml | 4.2E3 | 5.8E3 | 4.3E3 | 5.8E3 | 1.9E3 | 1.9E3 | 6.0E2 | 2.8E3 | 9.6E3 | 8.6E3 | 197 | 9 | 131 | 9 | 0.72 |
| jH | ng/ml | 7.4E1 | 6.7E1 | 8.4E1 | 7.5E1 | 5.1E1 | 3.0E1 | 1.3E1 | 4.3E1 | 4.3E2 | 1.3E2 | 197 | 9 | 131 | 9 | 0.46 |
| jI | ng/ml | 6.8E1 | 7.4E1 | 7.6E1 | 7.4E1 | 4.1E1 | 2.5E1 | 1.9E1 | 2.8E1 | 4.4E2 | 1.2E2 | 197 | 9 | 131 | 9 | 0.54 |
| rA | pg/ml | 2.6E1 | 2.4E1 | 3.1E1 | 3.3E1 | 2.4E1 | 2.7E1 | 1.0E-9 | 2.6E0 | 2.0E2 | 9.3E1 | 197 | 10 | 131 | 10 | 0.50 |

251

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| qY | pg/ml | 2.6E1 | 2.8E1 | 5.0E1 | 4.3E1 | 6.4E1 | 5.0E1 | 8.7E-1 | 3.4E0 | 5.3E2 | 1.8E2 | 197 | 10 | 131 | 10 | 0.52 |
| qX | pg/ml | 6.0E1 | 8.1E1 | 6.7E1 | 8.9E1 | 4.7E1 | 5.6E1 | 1.0E-9 | 1.3E1 | 2.5E2 | 1.8E2 | 197 | 10 | 131 | 10 | 0.62 |
| qW | pg/ml | 8.8E0 | 1.0E1 | 1.3E1 | 1.1E1 | 1.5E1 | 6.6E0 | 1.0E-9 | 1.0E-9 | 1.2E2 | 2.2E1 | 197 | 10 | 131 | 10 | 0.52 |
| qV | pg/ml | 2.2E3 | 3.7E3 | 2.8E3 | 3.6E3 | 2.1E3 | 1.9E3 | 1.7E2 | 1.4E3 | 9.6E3 | 7.1E3 | 197 | 10 | 131 | 10 | 0.64 |
| qU | pg/ml | 6.4E1 | 7.6E1 | 1.8E2 | 7.7E1 | 2.8E2 | 4.3E1 | 1.0E-9 | 2.2E1 | 2.1E3 | 1.7E2 | 197 | 10 | 131 | 10 | 0.49 |
| qT | pg/ml | 4.1E1 | 4.5E1 | 7.6E1 | 5.7E1 | 1.1E2 | 3.3E1 | 1.0E-9 | 2.3E1 | 9.0E2 | 1.3E2 | 197 | 10 | 131 | 10 | 0.55 |
| jK | ng/ml | 1.6E3 | 2.1E3 | 1.7E3 | 1.9E3 | 6.6E2 | 4.4E2 | 2.8E2 | 9.6E2 | 4.3E3 | 2.3E3 | 197 | 9 | 131 | 9 | 0.65 |
| jL | ng/ml | 2.0E2 | 2.1E2 | 3.0E2 | 2.7E2 | 3.0E2 | 1.8E2 | 3.5E1 | 9.6E1 | 2.1E3 | 5.7E2 | 197 | 9 | 131 | 9 | 0.51 |
| jM | ng/ml | 7.0E4 | 9.2E4 | 7.5E4 | 9.5E4 | 3.9E4 | 4.9E4 | 3.9E2 | 1.1E4 | 1.9E5 | 1.6E5 | 197 | 9 | 131 | 9 | 0.64 |
| jO | pg/ml | 2.1E5 | 2.0E5 | 2.6E5 | 2.9E5 | 1.5E5 | 2.3E5 | 5.2E4 | 1.3E5 | 1.1E6 | 8.0E5 | 197 | 9 | 131 | 9 | 0.50 |
| jP | pg/ml | 2.3E5 | 1.8E5 | 2.7E5 | 2.1E5 | 1.9E5 | 6.8E4 | 3.6E4 | 1.3E5 | 1.9E6 | 3.0E5 | 197 | 9 | 131 | 9 | 0.43 |
| jQ | pg/ml | 2.5E3 | 2.7E3 | 3.5E3 | 3.9E3 | 3.3E3 | 3.3E3 | 1.0E-9 | 6.4E2 | 1.8E4 | 1.0E4 | 197 | 9 | 131 | 9 | 0.56 |
| jR | pg/ml | 5.9E3 | 9.4E3 | 1.1E4 | 1.1E4 | 1.3E4 | 8.0E3 | 1.0E-9 | 2.0E3 | 9.0E4 | 2.2E4 | 197 | 9 | 131 | 9 | 0.56 |
| jT | pg/ml | 1.7E5 | 2.0E5 | 1.7E5 | 2.0E5 | 6.2E4 | 6.4E4 | 6.8E4 | 1.0E5 | 3.9E5 | 3.1E5 | 197 | 9 | 131 | 9 | 0.65 |
| jU | mIU/ml | 4.3E0 | 4.0E0 | 1.0E1 | 6.9E0 | 1.7E1 | 7.0E0 | 4.2E-2 | 3.0E-1 | 1.1E2 | 1.8E1 | 197 | 9 | 131 | 9 | 0.49 |
| jV | mIU/ml | 1.3E0 | 3.1E0 | 3.3E0 | 3.3E0 | 5.7E0 | 3.2E0 | 1.7E-3 | 1.1E-3 | 3.3E1 | 9.1E0 | 197 | 9 | 131 | 9 | 0.59 |
| jY | ng/ml | 7.3E-4 | 2.6E-3 | 6.3E-3 | 6.2E-3 | 2.8E-2 | 8.6E-3 | 1.0E-9 | 1.0E-9 | 3.0E-1 | 2.6E-2 | 197 | 9 | 131 | 9 | 0.63 |
| kC | pg/ml | 9.7E1 | 1.1E2 | 1.9E2 | 1.2E2 | 4.3E2 | 7.5E1 | 2.1E1 | 5.4E1 | 3.5E3 | 2.9E2 | 133 | 8 | 101 | 8 | 0.52 |
| kE | pg/ml | 1.3E5 | 1.3E5 | 1.3E5 | 1.3E5 | 4.0E4 | 2.7E4 | 1.2E4 | 1.0E5 | 2.3E5 | 1.8E5 | 133 | 8 | 101 | 8 | 0.52 |
| kF | pg/mL | 6.1E1 | 5.5E1 | 6.9E1 | 6.9E1 | 4.9E1 | 3.3E1 | 2.6E1 | 3.8E1 | 5.1E2 | 1.4E2 | 133 | 8 | 101 | 8 | 0.49 |
| kG | pg/mL | 9.1E3 | 8.2E3 | 1.2E4 | 1.6E4 | 1.3E4 | 2.3E4 | 7.5E2 | 4.2E3 | 1.2E5 | 7.1E4 | 133 | 8 | 101 | 8 | 0.50 |
| kI | pg/ml | 1.9E2 | 1.7E2 | 2.2E2 | 1.8E2 | 1.4E2 | 7.9E1 | 4.4E1 | 8.8E1 | 8.7E2 | 3.4E2 | 133 | 8 | 101 | 8 | 0.42 |
| kK | pg/ml | 1.0E2 | 1.8E2 | 1.5E2 | 3.6E2 | 1.6E2 | 4.0E2 | 6.4E0 | 5.1E1 | 1.2E3 | 1.2E3 | 133 | 8 | 101 | 8 | 0.65 |
| kN | pg/ml | 9.9E2 | 8.6E2 | 1.5E3 | 8.6E2 | 2.2E3 | 5.1E2 | 7.6E1 | 2.4E2 | 1.7E4 | 1.9E3 | 133 | 8 | 101 | 8 | 0.38 |
| kO | pg/ml | 7.1E3 | 5.7E3 | 9.6E3 | 7.3E3 | 1.7E4 | 3.1E3 | 3.4E3 | 5.3E3 | 1.5E5 | 1.4E4 | 133 | 8 | 101 | 8 | 0.42 |
| kP | pg/ml | 5.8E3 | 4.2E3 | 7.2E3 | 5.5E3 | 6.2E3 | 2.6E3 | 8.6E2 | 3.0E3 | 4.8E4 | 9.4E3 | 133 | 8 | 101 | 8 | 0.43 |
| kQ | pg/ml | 4.1E3 | 4.7E3 | 5.2E3 | 4.9E3 | 3.8E3 | 2.0E3 | 5.6E2 | 2.3E3 | 2.5E4 | 9.1E3 | 246 | 11 | 160 | 11 | 0.54 |
| kR | pg/ml | 2.1E1 | 2.7E1 | 3.0E1 | 2.9E1 | 6.8E1 | 1.5E1 | 1.0E-9 | 1.2E1 | 1.0E3 | 5.6E1 | 246 | 11 | 160 | 11 | 0.60 |
| kS | pg/ml | 7.8E2 | 9.1E2 | 9.2E2 | 8.3E2 | 6.5E2 | 2.5E2 | 7.9E1 | 4.2E2 | 4.8E3 | 1.2E3 | 246 | 11 | 160 | 11 | 0.53 |
| rZ | ng/ml | 1.0E-9 | 1.0E-9 | 5.8E-3 | 3.3E-2 | 1.5E-2 | 9.4E-2 | 1.0E-9 | 1.0E-9 | 1.1E-1 | 3.0E-1 | 187 | 10 | 127 | 10 | 0.51 |
| rY | ng/ml | 6.1E-2 | 6.6E-2 | 1.4E-1 | 1.8E0 | 5.3E-1 | 5.6E0 | 1.0E-9 | 3.2E-2 | 6.3E0 | 1.8E1 | 187 | 10 | 127 | 10 | 0.59 |
| rX | ng/ml | 1.0E-9 | 1.0E-9 | 4.0E-2 | 2.4E-1 | 2.9E-1 | 7.6E-1 | 1.0E-9 | 1.0E-9 | 3.7E0 | 2.4E0 | 187 | 10 | 127 | 10 | 0.45 |
| lK | pg/ml | 7.9E1 | 5.2E1 | 1.7E2 | 1.2E2 | 3.1E2 | 1.6E2 | 1.0E-9 | 1.0E-9 | 3.3E3 | 5.0E2 | 196 | 9 | 131 | 9 | 0.43 |
| lL | pg/ml | 1.5E3 | 2.1E3 | 2.1E3 | 3.1E3 | 2.3E3 | 2.3E3 | 1.5E1 | 8.1E2 | 1.9E4 | 7.7E3 | 197 | 9 | 131 | 9 | 0.69 |
| lM | pg/ml | 1.1E3 | 1.0E3 | 3.6E3 | 6.1E3 | 7.0E3 | 1.0E4 | 1.3E2 | 2.8E2 | 4.4E4 | 3.0E4 | 197 | 9 | 131 | 9 | 0.46 |
| lN | pg/ml | 1.0E-9 | 1.0E-9 | 4.3E0 | 3.2E0 | 1.5E1 | 5.4E0 | 1.0E-9 | 1.0E-9 | 1.5E2 | 1.3E1 | 197 | 9 | 131 | 9 | 0.49 |
| lO | pg/ml | 1.0E-9 | 1.0E-9 | 1.8E0 | 1.5E0 | 1.4E1 | 4.4E0 | 1.0E-9 | 1.0E-9 | 1.4E2 | 1.3E1 | 196 | 9 | 131 | 9 | 0.54 |
| nW | pg/ml | 1.1E5 | 1.1E5 | 1.1E5 | 1.2E5 | 2.6E4 | 3.7E4 | 3.6E4 | 6.9E4 | 2.0E5 | 1.8E5 | 246 | 11 | 160 | 11 | 0.53 |
| nY | pg/ml | 2.0E3 | 1.6E3 | 2.3E3 | 2.0E3 | 1.3E3 | 1.3E3 | 5.1E2 | 6.6E2 | 9.9E3 | 5.3E3 | 246 | 11 | 160 | 11 | 0.43 |
| oO | pg/ml | 8.7E4 | 1.1E5 | 1.2E5 | 1.2E5 | 9.5E4 | 7.4E4 | 3.3E3 | 5.6E4 | 6.2E5 | 2.8E5 | 123 | 8 | 96 | 8 | 0.56 |
| oP | pg/ml | 1.2E5 | 1.4E5 | 1.4E5 | 1.8E5 | 8.2E4 | 1.3E5 | 2.4E4 | 6.3E4 | 4.5E5 | 4.2E5 | 123 | 8 | 96 | 8 | 0.57 |
| oQ | pg/ml | 2.9E3 | 2.9E3 | 3.8E3 | 3.6E3 | 3.0E3 | 2.3E3 | 7.7E2 | 1.6E3 | 2.0E4 | 7.9E3 | 123 | 8 | 96 | 8 | 0.49 |
| oE | pg/ml | 1.3E2 | 6.7E1 | 3.9E2 | 1.6E2 | 5.8E2 | 1.9E2 | 1.0E-9 | 1.0E-9 | 4.7E3 | 6.3E2 | 246 | 11 | 160 | 11 | 0.38 |
| oF | pg/ml | 8.0E3 | 4.8E3 | 2.1E4 | 1.2E4 | 3.3E4 | 1.6E4 | 6.4E1 | 1.0E3 | 1.7E5 | 5.7E4 | 246 | 11 | 160 | 11 | 0.45 |
| oH | pg/ml | 4.0E1 | 1.0E2 | 9.3E1 | 1.0E2 | 1.5E2 | 5.9E1 | 4.3E-1 | 3.6E1 | 9.9E2 | 2.0E2 | 246 | 11 | 160 | 11 | 0.70 |
| oK | pg/ml | 8.5E2 | 1.1E3 | 2.0E3 | 1.4E3 | 3.0E3 | 1.3E3 | 5.2E1 | 1.8E2 | 2.5E4 | 4.8E3 | 246 | 11 | 160 | 11 | 0.53 |
| oN | pg/ml | 5.1E2 | 5.4E2 | 7.6E2 | 7.8E2 | 1.4E3 | 9.4E2 | 1.1E2 | 2.6E2 | 1.8E4 | 3.6E3 | 246 | 11 | 160 | 11 | 0.50 |
| pF | pg/ml | 5.1E-1 | 5.1E-1 | 1.1E0 | 7.9E-1 | 5.6E0 | 9.1E-1 | 1.0E-9 | 4.2E-2 | 8.7E1 | 3.1E0 | 246 | 11 | 160 | 11 | 0.50 |

Figure 23.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 6.2E1 | 4.9E1 | 7.3E1 | 6.9E1 | 5.0E1 | 5.1E1 | 2.0E0 | 5.0E0 | 2.9E2 | 2.0E2 | 936 | 29 | 159 | 29 | 0.47 |
| Ad | ug/mL | 2.4E-2 | 5.9E-2 | 5.2E-2 | 8.8E-2 | 7.0E-2 | 9.0E-2 | 6.8E-4 | 3.6E-3 | 3.7E-1 | 3.6E-1 | 218 | 25 | 87 | 25 | 0.68 |
| Af | ng/mL | 8.9E-1 | 6.0E-1 | 1.6E1 | 2.5E1 | 7.0E1 | 9.4E1 | 1.7E-3 | 1.7E-3 | 5.3E2 | 4.8E2 | 218 | 25 | 87 | 25 | 0.53 |
| Aj | ug/mL | 1.4E0 | 4.8E0 | 2.6E0 | 3.5E0 | 2.4E0 | 2.4E0 | 1.5E-3 | 3.8E-2 | 6.1E0 | 5.8E0 | 218 | 25 | 87 | 25 | 0.58 |
| Al | mg/mL | 8.8E-5 | 9.6E-5 | 2.5E-4 | 2.4E-4 | 4.2E-4 | 3.8E-4 | 2.5E-6 | 2.1E-5 | 1.9E-3 | 1.4E-3 | 218 | 25 | 87 | 25 | 0.54 |
| An | U/mL | 4.1E1 | 6.6E1 | 1.8E2 | 1.6E2 | 5.5E2 | 2.3E2 | 9.8E-4 | 6.1E-1 | 5.5E3 | 9.1E2 | 218 | 25 | 87 | 25 | 0.57 |
| Ao | pg/mL | 8.1E1 | 7.2E1 | 2.3E2 | 1.7E3 | 1.1E3 | 7.7E3 | 2.8E0 | 7.4E0 | 1.6E4 | 3.9E4 | 218 | 25 | 87 | 25 | 0.52 |
| Ap | ng/mL | 2.6E1 | 4.3E1 | 3.6E1 | 5.4E1 | 3.2E1 | 4.2E1 | 9.9E-1 | 1.8E0 | 1.7E2 | 1.6E2 | 218 | 25 | 87 | 25 | 0.64 |
| Ar | ng/mL | 6.3E-1 | 1.4E0 | 2.3E0 | 4.0E0 | 4.7E0 | 9.9E0 | 3.4E-3 | 3.4E-3 | 4.3E1 | 4.7E1 | 218 | 25 | 87 | 25 | 0.55 |
| As | ng/mL | 8.6E-3 | 8.7E-3 | 1.3E-2 | 1.1E-2 | 1.7E-2 | 9.7E-3 | 1.7E-3 | 1.7E-3 | 1.1E-1 | 4.2E-2 | 218 | 25 | 87 | 25 | 0.50 |
| Aw | pg/mL | 1.5E1 | 1.7E1 | 1.6E1 | 1.9E1 | 4.9E0 | 8.0E0 | 5.0E0 | 6.7E0 | 3.2E1 | 4.8E1 | 218 | 25 | 87 | 25 | 0.62 |
| Ax | ng/mL | 2.2E0 | 1.5E0 | 9.2E0 | 6.5E0 | 1.9E1 | 1.1E1 | 1.9E-2 | 4.9E-2 | 1.5E2 | 4.8E1 | 218 | 25 | 87 | 25 | 0.47 |
| Ba | ng/mL | 4.2E1 | 1.6E2 | 3.5E2 | 7.5E2 | 9.5E2 | 1.7E3 | 3.7E-1 | 1.2E0 | 8.1E3 | 8.1E3 | 218 | 25 | 87 | 25 | 0.64 |
| Bb | ng/mL | 2.3E0 | 3.8E0 | 4.8E0 | 1.4E1 | 8.2E0 | 4.9E1 | 4.1E-3 | 3.0E-1 | 6.6E1 | 2.5E2 | 218 | 25 | 87 | 25 | 0.60 |
| Bc | ng/mL | 3.2E1 | 4.5E1 | 9.6E1 | 1.1E2 | 1.8E2 | 1.9E2 | 1.1E-1 | 6.1E-1 | 1.0E3 | 8.9E2 | 218 | 25 | 87 | 25 | 0.56 |
| Bg | ng/mL | 7.1E-2 | 1.7E-1 | 3.4E0 | 3.4E0 | 2.1E1 | 1.0E1 | 5.3E-4 | 5.3E-4 | 2.5E2 | 4.9E1 | 218 | 25 | 87 | 25 | 0.62 |
| Bn | ng/mL | 5.6E-2 | 5.6E-2 | 8.6E-1 | 1.4E0 | 1.6E0 | 2.2E0 | 5.6E-2 | 5.6E-2 | 9.7E0 | 7.2E0 | 218 | 25 | 87 | 25 | 0.55 |
| Bo | ng/mL | 1.1E1 | 1.9E1 | 1.2E1 | 2.5E1 | 8.8E0 | 4.0E1 | 1.6E-2 | 1.6E-2 | 4.4E1 | 2.1E2 | 218 | 25 | 87 | 25 | 0.64 |
| Ch | uIU/mL | 1.1E0 | 1.5E0 | 7.6E0 | 1.9E1 | 2.3E1 | 4.6E1 | 3.4E-3 | 3.4E-3 | 2.1E2 | 2.1E2 | 218 | 25 | 87 | 25 | 0.55 |
| Co | pg/mL | 3.0E1 | 4.6E1 | 8.0E1 | 4.2E2 | 2.0E2 | 1.8E3 | 3.9E0 | 5.4E0 | 1.9E3 | 9.0E3 | 218 | 25 | 87 | 25 | 0.63 |
| Cp | ng/mL | 2.0E1 | 2.4E1 | 2.4E1 | 3.6E1 | 2.5E1 | 4.5E1 | 6.0E-1 | 2.5E0 | 2.9E2 | 2.4E2 | 218 | 25 | 87 | 25 | 0.61 |
| Cq | ng/mL | 2.4E-2 | 3.2E-2 | 1.7E-1 | 1.6E-1 | 1.2E0 | 4.3E-1 | 8.0E-4 | 8.0E-4 | 1.7E1 | 2.1E0 | 218 | 25 | 87 | 25 | 0.55 |
| Cs | ng/mL | 6.0E1 | 7.3E1 | 2.4E2 | 2.0E2 | 4.4E2 | 3.0E2 | 3.9E-1 | 2.8E0 | 2.9E3 | 1.2E3 | 218 | 25 | 87 | 25 | 0.47 |
| Ct | ng/mL | 1.2E0 | 3.7E-1 | 3.1E1 | 6.9E1 | 9.4E1 | 1.6E2 | 6.2E-3 | 2.5E-2 | 6.2E2 | 4.7E2 | 218 | 25 | 87 | 25 | 0.50 |
| Cu | ng/mL | 2.1E-1 | 2.8E-1 | 4.2E-1 | 4.0E-1 | 8.2E-1 | 3.1E-1 | 9.6E-3 | 3.6E-2 | 9.2E0 | 1.2E0 | 218 | 25 | 87 | 25 | 0.60 |
| Cv | ng/mL | 3.9E0 | 6.4E0 | 1.6E1 | 2.5E1 | 5.3E1 | 5.0E1 | 5.6E-3 | 2.9E-1 | 5.3E2 | 2.4E2 | 218 | 25 | 87 | 25 | 0.58 |
| Cw | mIU/mL | 2.7E-2 | 3.7E-2 | 3.4E-2 | 5.5E-2 | 2.4E-2 | 5.2E-2 | 2.3E-3 | 2.8E-3 | 1.4E-1 | 2.4E-1 | 218 | 25 | 87 | 25 | 0.65 |
| Cx | ng/mL | 1.7E-1 | 3.2E-2 | 5.1E1 | 7.0E1 | 1.0E2 | 1.2E2 | 9.3E-5 | 9.3E-5 | 3.9E2 | 3.9E2 | 218 | 25 | 87 | 25 | 0.48 |
| Db | ug/mL | 7.3E0 | 1.0E1 | 8.3E0 | 1.1E1 | 5.7E0 | 7.3E0 | 5.0E-1 | 9.7E-1 | 3.9E1 | 3.2E1 | 218 | 25 | 87 | 25 | 0.64 |
| Dc | nmol/L | 1.8E-2 | 2.9E-2 | 5.7E-2 | 7.0E-2 | 1.4E-1 | 1.3E-1 | 5.2E-6 | 2.7E-3 | 1.2E0 | 6.3E-1 | 218 | 25 | 87 | 25 | 0.62 |
| Dd | ug/mL | 6.8E-2 | 1.5E-1 | 1.7E-1 | 1.8E-1 | 2.6E-1 | 1.8E-1 | 1.9E-4 | 3.7E-3 | 1.6E0 | 7.8E-1 | 218 | 25 | 87 | 25 | 0.60 |
| De | ng/mL | 3.4E-3 | 3.4E-3 | 6.0E-2 | 1.1E-1 | 1.1E-1 | 2.1E-1 | 3.4E-3 | 3.4E-3 | 5.9E-1 | 8.2E-1 | 218 | 25 | 87 | 25 | 0.53 |
| Dg | ng/mL | 2.5E1 | 5.0E1 | 3.7E1 | 5.2E1 | 3.4E1 | 4.1E1 | 2.4E-1 | 2.9E0 | 1.9E2 | 1.9E2 | 218 | 25 | 87 | 25 | 0.62 |
| Di | pg/mL | 1.7E0 | 2.0E0 | 2.0E0 | 2.6E0 | 1.8E0 | 2.4E0 | 1.8E-1 | 1.8E-1 | 1.3E1 | 9.2E0 | 218 | 25 | 87 | 25 | 0.56 |
| Dk | uIU/mL | 1.5E-2 | 1.8E-2 | 1.0E-1 | 1.6E-1 | 6.8E-1 | 5.2E-1 | 1.1E-4 | 1.1E-4 | 8.9E0 | 2.7E0 | 218 | 25 | 87 | 25 | 0.58 |
| Dl | ng/mL | 2.1E2 | 2.9E2 | 2.7E2 | 3.8E2 | 2.4E2 | 3.4E2 | 1.7E0 | 2.2E1 | 1.4E3 | 1.3E3 | 218 | 25 | 87 | 25 | 0.59 |
| Dp | ng/ml | 2.4E0 | 1.0E0 | 4.5E0 | 4.7E0 | 6.6E0 | 7.4E0 | 3.7E-3 | 3.7E-3 | 4.3E1 | 2.7E1 | 111 | 23 | 86 | 23 | 0.44 |
| Ef | ng/ml | 9.5E-2 | 1.9E-1 | 5.6E-1 | 1.0E0 | 1.3E0 | 2.1E0 | 5.7E-4 | 1.1E-2 | 9.4E0 | 9.5E0 | 143 | 23 | 86 | 23 | 0.63 |
| Wm | % | 7.0E-1 | 8.5E-2 | 3.4E1 | 3.5E0 | 2.1E2 | 1.1E1 | 8.5E-2 | 5.4E-2 | 2.4E3 | 5.5E1 | 173 | 24 | 101 | 24 | 0.41 |
| Ed | pg/ml | 7.1E0 | 5.2E-1 | 1.0E2 | 2.0E1 | 6.9E2 | 4.2E1 | 5.2E-1 | 5.2E-1 | 7.3E3 | 1.5E2 | 111 | 23 | 85 | 23 | 0.39 |
| Tj | pg/ml | 3.7E-1 | 3.7E-1 | 8.9E1 | 3.1E1 | 4.0E2 | 1.2E2 | 3.6E-1 | 3.7E-1 | 3.5E3 | 5.9E2 | 141 | 23 | 86 | 23 | 0.45 |
| Po | pg/ml | 2.6E-1 | 2.6E-2 | 7.7E0 | 1.0E1 | 2.5E1 | 2.1E1 | 8.0E-3 | 2.6E-2 | 2.7E2 | 1.1E2 | 316 | 36 | 135 | 36 | 0.50 |
| Et | ng/ml | 1.1E3 | 1.5E3 | 1.4E3 | 2.0E3 | 1.0E3 | 1.4E3 | 7.7E1 | 1.6E2 | 4.2E3 | 4.9E3 | 316 | 36 | 135 | 36 | 0.62 |
| Fa | ng/ml | 4.0E1 | 3.4E1 | 1.4E2 | 1.8E2 | 7.9E2 | 5.8E2 | 3.4E-2 | 4.9E0 | 8.0E3 | 2.8E3 | 109 | 23 | 86 | 23 | 0.54 |
| Ez | ng/ml | 5.0E0 | 3.6E0 | 2.4E1 | 1.2E1 | 7.6E1 | 1.8E1 | 1.3E-2 | 1.3E-2 | 7.1E2 | 6.6E1 | 111 | 23 | 86 | 23 | 0.48 |
| Fb | ng/ml | 2.3E1 | 2.9E1 | 2.2E1 | 2.5E1 | 1.2E1 | 1.2E1 | 1.0E0 | 8.1E-1 | 5.7E1 | 4.0E1 | 109 | 23 | 86 | 23 | 0.59 |
| Ex | ng/ml | 6.2E-2 | 1.1E-1 | 2.6E-1 | 3.6E-1 | 9.1E-1 | 1.0E0 | 3.5E-5 | 1.5E-4 | 8.9E0 | 4.9E0 | 102 | 21 | 53 | 21 | 0.57 |
| Fn | ng/ml | 2.1E-1 | 2.1E-1 | 9.2E0 | 2.8E0 | 4.0E1 | 5.0E0 | 1.1E-14 | 1.1E-14 | 4.2E2 | 1.9E1 | 111 | 23 | 86 | 23 | 0.43 |
| Fp | ng/ml | 1.0E1 | 1.2E1 | 2.1E1 | 2.5E1 | 2.7E1 | 3.1E1 | 6.0E-3 | 1.3E-1 | 1.4E2 | 1.1E2 | 333 | 36 | 136 | 36 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fr | ng/ml | 2.5E4 | 5.2E4 | 9.7E4 | 1.3E5 | 1.7E5 | 1.6E5 | 6.4E2 | 1.9E2 | 8.4E5 | 6.0E5 | 398 | 37 | 137 | 37 | 0.59 |
| Fw | pg/ml | 8.5E-1 | 1.1E0 | 1.1E2 | 1.1E1 | 7.2E2 | 2.5E1 | 1.1E-14 | 1.2E-1 | 6.9E3 | 1.1E2 | 144 | 23 | 87 | 23 | 0.50 |
| Fy | ng/ml | 3.1E1 | 3.0E1 | 4.9E1 | 5.5E1 | 5.5E1 | 6.0E1 | 1.8E-1 | 1.2E-1 | 3.3E2 | 2.3E2 | 110 | 22 | 85 | 22 | 0.53 |
| Gl | pg/ml | 6.0E3 | 8.7E3 | 9.8E3 | 1.1E4 | 9.0E3 | 9.8E3 | 2.3E2 | 2.0E2 | 3.2E4 | 3.0E4 | 140 | 23 | 87 | 23 | 0.53 |
| Gp | U/ml | 1.8E0 | 8.5E-1 | 4.4E0 | 3.3E0 | 8.0E0 | 6.0E0 | 1.3E-3 | 5.7E-3 | 6.7E1 | 2.3E1 | 144 | 23 | 87 | 23 | 0.39 |
| Gz | ug/ml | 1.2E0 | 1.1E1 | 1.2E1 | 7.4E0 | 5.8E1 | 5.7E0 | 2.9E-16 | 1.6E-1 | 4.8E2 | 1.9E1 | 69 | 21 | 52 | 21 | 0.63 |
| Ha | ng/ml | 2.7E0 | 1.8E0 | 9.0E0 | 1.1E1 | 2.0E1 | 2.0E1 | 1.7E-2 | 1.7E-2 | 1.0E2 | 7.7E1 | 109 | 23 | 85 | 23 | 0.51 |
| Nm | pg/ml | 1.4E4 | 1.6E4 | 2.6E4 | 2.5E4 | 5.1E4 | 2.9E4 | 1.0E-9 | 1.0E-9 | 7.8E5 | 1.2E5 | 315 | 36 | 136 | 36 | 0.51 |
| Nn | pg/ml | 1.2E2 | 1.1E2 | 2.7E3 | 6.3E2 | 1.1E4 | 1.1E3 | 1.0E-9 | 1.0E-9 | 1.0E5 | 4.9E3 | 315 | 36 | 136 | 36 | 0.54 |
| No | pg/ml | 1.4E1 | 1.0E1 | 2.7E1 | 2.8E1 | 5.7E1 | 3.9E1 | 1.0E-9 | 1.0E-9 | 5.9E2 | 1.4E2 | 315 | 36 | 136 | 36 | 0.46 |
| Nq | pg/ml | 2.8E0 | 4.2E-1 | 2.2E1 | 1.3E1 | 8.9E1 | 5.4E1 | 1.0E-9 | 1.0E-9 | 1.2E3 | 3.3E2 | 315 | 36 | 136 | 36 | 0.42 |
| Nr | pg/ml | 6.0E-1 | 6.1E-1 | 1.4E1 | 1.4E1 | 6.9E1 | 3.7E1 | 1.0E-9 | 1.0E-9 | 9.8E2 | 1.7E2 | 315 | 36 | 136 | 36 | 0.52 |
| Ns | pg/ml | 1.0E-9 | 1.0E-9 | 1.3E1 | 7.2E0 | 7.8E1 | 2.9E1 | 1.0E-9 | 1.0E-9 | 1.1E3 | 1.3E2 | 315 | 36 | 136 | 36 | 0.51 |
| Nt | pg/ml | 9.9E1 | 1.2E2 | 1.4E2 | 1.3E2 | 1.3E2 | 7.6E1 | 1.5E1 | 1.5E1 | 1.5E3 | 3.4E2 | 315 | 36 | 136 | 36 | 0.52 |
| Nu | pg/ml | 2.0E1 | 4.3E1 | 5.2E1 | 6.8E1 | 8.8E1 | 7.8E1 | 1.0E-9 | 1.0E-9 | 8.9E2 | 2.6E2 | 315 | 36 | 136 | 36 | 0.56 |
| Lu | pg/ml | 1.0E4 | 1.0E4 | 1.6E4 | 9.2E3 | 4.2E4 | 6.1E3 | 6.7E2 | 1.4E3 | 5.6E5 | 2.1E4 | 317 | 36 | 136 | 36 | 0.46 |
| Lv | pg/ml | 1.0E-9 | 1.0E-9 | 1.1E1 | 2.0E1 | 2.2E1 | 4.5E1 | 1.0E-9 | 1.0E-9 | 2.4E2 | 2.6E2 | 317 | 36 | 136 | 36 | 0.55 |
| Lw | pg/ml | 1.0E-9 | 1.0E-9 | 1.3E-1 | 2.8E-1 | 1.8E0 | 1.7E0 | 1.0E-9 | 1.0E-9 | 3.2E1 | 9.9E0 | 317 | 36 | 136 | 36 | 0.51 |
| Lx | pg/ml | 1.0E-9 | 1.0E-9 | 1.2E2 | 1.8E2 | 4.3E2 | 4.0E2 | 1.0E-9 | 1.0E-9 | 6.2E3 | 1.8E3 | 317 | 36 | 136 | 36 | 0.55 |
| Ly | pg/ml | 1.0E-9 | 6.7E0 | 9.2E0 | 1.4E1 | 1.8E1 | 1.6E1 | 1.0E-9 | 1.0E-9 | 1.2E2 | 4.9E1 | 317 | 36 | 136 | 36 | 0.61 |
| Lz | pg/ml | 1.0E-9 | 1.0E-9 | 2.5E0 | 1.2E0 | 2.5E1 | 7.1E0 | 1.0E-9 | 1.0E-9 | 4.3E2 | 4.3E1 | 317 | 36 | 136 | 36 | 0.49 |
| Ma | pg/ml | 2.7E2 | 3.3E2 | 1.4E3 | 1.5E3 | 4.6E3 | 3.2E3 | 1.0E-9 | 1.0E-9 | 6.5E4 | 1.7E4 | 317 | 36 | 136 | 36 | 0.51 |
| Mb | pg/ml | 2.5E1 | 2.8E1 | 3.0E1 | 3.2E1 | 1.3E1 | 1.3E1 | 5.4E0 | 1.4E1 | 6.9E1 | 5.9E1 | 317 | 36 | 136 | 36 | 0.54 |
| Mc | pg/ml | 1.0E-9 | 1.0E-9 | 2.0E-2 | 4.6E-2 | 2.5E-1 | 2.8E-1 | 1.0E-9 | 1.0E-9 | 4.0E0 | 1.7E0 | 317 | 36 | 136 | 36 | 0.51 |
| Md | pg/ml | 1.0E-9 | 1.0E-9 | 5.0E-1 | 2.9E-1 | 4.0E0 | 1.3E0 | 1.0E-9 | 1.0E-9 | 6.5E1 | 7.3E0 | 317 | 36 | 136 | 36 | 0.51 |
| Me | pg/ml | 3.2E1 | 2.1E1 | 2.9E1 | 2.4E1 | 1.6E1 | 1.3E1 | 1.0E-9 | 2.4E-1 | 1.2E2 | 5.0E1 | 317 | 36 | 136 | 36 | 0.38 |
| Mf | pg/ml | 1.0E-9 | 1.0E-9 | 3.8E-1 | 1.6E-1 | 1.7E0 | 4.3E-1 | 1.0E-9 | 1.0E-9 | 2.0E1 | 2.2E0 | 317 | 36 | 136 | 36 | 0.55 |
| Mg | pg/ml | 2.2E0 | 4.0E0 | 7.3E0 | 7.4E0 | 1.2E1 | 9.5E0 | 1.0E-9 | 1.0E-9 | 8.8E1 | 3.5E1 | 317 | 36 | 136 | 36 | 0.56 |
| Mh | pg/ml | 1.0E-9 | 1.0E-9 | 9.0E-1 | 3.8E-1 | 8.0E0 | 1.4E0 | 1.0E-9 | 1.0E-9 | 1.1E2 | 7.8E0 | 317 | 36 | 136 | 36 | 0.51 |
| Mi | pg/ml | 1.0E-9 | 1.0E-9 | 7.8E-1 | 1.0E-9 | 7.5E0 | 0.0E0 | 1.0E-9 | 1.0E-9 | 1.2E2 | 1.0E-9 | 317 | 36 | 136 | 36 | 0.49 |
| Mj | pg/ml | 1.0E-9 | 1.0E-9 | 4.6E0 | 2.1E0 | 2.8E1 | 5.8E0 | 1.0E-9 | 1.0E-9 | 4.6E2 | 2.2E1 | 317 | 36 | 136 | 36 | 0.49 |
| Mk | pg/ml | 1.0E-9 | 4.6E-1 | 1.7E1 | 1.5E1 | 1.1E2 | 5.7E1 | 1.0E-9 | 1.0E-9 | 1.7E3 | 3.3E2 | 317 | 36 | 136 | 36 | 0.46 |
| Ml | pg/ml | 1.0E-9 | 1.0E-9 | 9.5E0 | 6.6E-1 | 1.2E2 | 2.1E0 | 1.0E-9 | 1.0E-9 | 2.1E3 | 1.2E1 | 317 | 36 | 136 | 36 | 0.51 |
| Mm | pg/ml | 4.6E2 | 4.7E2 | 8.4E2 | 9.7E2 | 9.7E2 | 1.7E3 | 1.0E-9 | 1.0E-9 | 6.0E3 | 9.9E3 | 317 | 36 | 136 | 36 | 0.48 |
| Mn | pg/ml | 4.9E0 | 4.9E0 | 1.0E1 | 7.0E0 | 2.6E1 | 6.9E0 | 1.0E-9 | 1.0E-9 | 3.5E2 | 3.3E1 | 317 | 36 | 136 | 36 | 0.49 |
| Mp | pg/ml | 1.0E-9 | 1.0E-9 | 1.0E1 | 9.1E0 | 2.3E1 | 2.9E1 | 1.0E-9 | 1.0E-9 | 1.8E2 | 1.5E2 | 317 | 36 | 136 | 36 | 0.44 |
| Mq | pg/ml | 1.0E-9 | 1.0E-9 | 2.9E0 | 4.4E0 | 1.8E1 | 1.5E1 | 1.0E-9 | 1.0E-9 | 2.1E2 | 6.5E1 | 317 | 36 | 136 | 36 | 0.52 |
| Mr | pg/ml | 1.0E-9 | 1.0E-9 | 1.6E1 | 2.7E1 | 9.3E1 | 7.7E1 | 1.0E-9 | 1.0E-9 | 1.4E3 | 3.1E2 | 317 | 36 | 136 | 36 | 0.54 |
| Ms | pg/ml | 4.0E2 | 4.2E2 | 5.5E2 | 6.1E2 | 7.0E2 | 7.3E2 | 1.0E-9 | 1.0E-9 | 5.6E3 | 3.3E3 | 317 | 36 | 136 | 36 | 0.51 |
| Mt | pg/ml | 1.0E-9 | 4.9E-1 | 1.1E1 | 4.1E0 | 6.7E1 | 1.3E1 | 1.0E-9 | 1.0E-9 | 8.7E2 | 7.0E1 | 317 | 36 | 136 | 36 | 0.55 |
| Mu | pg/ml | 1.0E-9 | 1.0E-9 | 1.7E0 | 9.1E-1 | 1.1E1 | 3.9E0 | 1.0E-9 | 1.0E-9 | 9.9E1 | 2.3E1 | 317 | 36 | 136 | 36 | 0.51 |
| Mv | pg/ml | 1.0E-9 | 1.0E-9 | 9.5E1 | 4.0E1 | 4.5E2 | 1.6E2 | 1.0E-9 | 1.0E-9 | 4.5E3 | 9.4E2 | 317 | 36 | 136 | 36 | 0.49 |
| Mw | pg/ml | 2.3E1 | 1.6E1 | 6.3E2 | 5.7E2 | 4.1E3 | 2.6E3 | 1.0E-9 | 1.0E-9 | 6.2E4 | 1.6E4 | 317 | 36 | 136 | 36 | 0.49 |
| Mx | pg/ml | 1.0E-9 | 1.0E-9 | 2.1E-1 | 2.2E-1 | 8.3E-1 | 6.4E-1 | 1.0E-9 | 1.0E-9 | 7.4E0 | 3.4E0 | 317 | 36 | 136 | 36 | 0.52 |
| My | pg/ml | 1.0E-9 | 1.0E-9 | 6.1E2 | 8.9E2 | 3.7E3 | 5.1E3 | 1.0E-9 | 1.0E-9 | 3.9E4 | 3.1E4 | 317 | 36 | 136 | 36 | 0.51 |
| Mz | pg/ml | 9.2E0 | 1.0E1 | 2.3E1 | 1.7E1 | 6.3E1 | 2.8E1 | 1.0E-9 | 1.0E-9 | 8.4E2 | 1.4E2 | 317 | 36 | 136 | 36 | 0.51 |
| Na | pg/ml | 1.0E-9 | 1.0E-9 | 5.3E-1 | 4.3E-1 | 1.7E0 | 1.5E0 | 1.0E-9 | 1.0E-9 | 9.6E0 | 7.2E0 | 317 | 36 | 136 | 36 | 0.48 |
| Nb | pg/ml | 1.9E0 | 2.1E0 | 4.9E0 | 4.6E0 | 1.7E1 | 1.3E1 | 1.0E-9 | 1.0E-9 | 2.3E2 | 8.1E1 | 317 | 36 | 136 | 36 | 0.49 |
| Nc | pg/ml | 4.8E2 | 1.4E2 | 7.1E2 | 3.6E2 | 8.7E2 | 5.0E2 | 1.0E-9 | 1.0E-9 | 9.2E3 | 1.8E3 | 317 | 36 | 136 | 36 | 0.34 |
| Nd | pg/ml | 3.0E1 | 9.2E0 | 2.6E1 | 1.7E1 | 2.0E1 | 1.8E1 | 1.0E-9 | 1.0E-9 | 8.4E1 | 5.5E1 | 317 | 36 | 136 | 36 | 0.36 |
| Ne | pg/ml | 5.2E2 | 1.8E2 | 6.7E2 | 2.9E2 | 6.7E2 | 3.3E2 | 1.0E-9 | 1.0E-9 | 7.0E3 | 1.1E3 | 317 | 36 | 136 | 36 | 0.29 |
| Nf | pg/ml | 1.0E-9 | 1.0E-9 | 2.8E0 | 1.1E0 | 8.7E0 | 2.9E0 | 1.0E-9 | 1.0E-9 | 8.2E1 | 1.3E1 | 317 | 36 | 136 | 36 | 0.45 |
| Ng | pg/ml | 3.8E1 | 4.2E1 | 1.5E2 | 1.1E2 | 2.9E2 | 2.0E2 | 1.0E-9 | 1.0E-9 | 2.3E3 | 1.1E3 | 317 | 36 | 136 | 36 | 0.49 |

| Code | Units | Median | | Average | | Standard Deviation | | Minimum | | Maximum | | Number of Samples | | Number of Patients | | AUC |
|------|-------|--------|--------|---------|--------|--------|--------|--------|--------|--------|--------|--------|--------|--------|--------|------|
| | | NonDis | Dis | NonDis | Dis | NonDis | Dis | NonDis | Dis | NonDis | Dis | NonDis | Dis | NonDis | Dis | |
| Nh | pg/ml | 7.7E1 | 3.4E1 | 1.0E2 | 4.8E1 | 9.1E1 | 4.2E1 | 1.0E-9 | 6.7E0 | 5.6E2 | 1.6E2 | 317 | 36 | 136 | 36 | 0.30 |
| Ni | pg/ml | 4.5E0 | 1.0E-9 | 8.0E1 | 1.0E2 | 1.3E2 | 1.5E2 | 1.0E-9 | 1.0E-9 | 7.1E2 | 5.9E2 | 317 | 36 | 136 | 36 | 0.52 |
| Nj | pg/ml | 8.9E0 | 2.4E0 | 1.2E1 | 7.9E0 | 1.2E1 | 1.1E1 | 1.0E-9 | 1.0E-9 | 8.3E1 | 4.6E1 | 317 | 36 | 136 | 36 | 0.34 |
| Nk | pg/ml | 2.4E1 | 1.4E1 | 3.7E1 | 2.7E1 | 4.1E1 | 3.9E1 | 1.0E-9 | 1.0E-9 | 2.0E2 | 1.7E2 | 317 | 36 | 136 | 36 | 0.42 |
| Nl | pg/ml | 5.6E1 | 1.5E1 | 7.3E1 | 3.0E1 | 8.5E1 | 3.8E1 | 1.0E-9 | 1.0E-9 | 1.1E3 | 1.5E2 | 317 | 36 | 136 | 36 | 0.29 |
| Tz | pg/ml | 4.8E3 | 5.2E3 | 8.4E3 | 1.5E4 | 1.2E4 | 3.4E4 | 7.4E1 | 6.3E2 | 8.8E4 | 1.7E5 | 113 | 24 | 84 | 24 | 0.54 |
| Ua | pg/ml | 3.8E3 | 4.0E3 | 1.3E4 | 2.2E4 | 2.5E4 | 4.0E4 | 3.5E2 | 4.8E2 | 1.4E5 | 1.8E5 | 113 | 24 | 84 | 24 | 0.55 |
| Ub | pg/ml | 5.3E2 | 5.6E2 | 7.9E2 | 7.7E2 | 1.2E3 | 8.0E2 | 1.0E-9 | 1.0E-9 | 9.8E3 | 3.3E3 | 113 | 24 | 84 | 24 | 0.52 |
| Ue | pg/ml | 3.0E1 | 2.4E1 | 3.2E1 | 3.1E1 | 1.9E1 | 2.9E1 | 4.2E0 | 5.9E0 | 9.5E1 | 1.5E2 | 113 | 24 | 84 | 24 | 0.44 |
| Uc | pg/ml | 8.9E2 | 8.4E2 | 1.8E3 | 1.8E3 | 3.4E3 | 2.5E3 | 6.1E-1 | 1.0E2 | 2.9E4 | 9.6E3 | 113 | 24 | 84 | 24 | 0.52 |
| Ud | pg/ml | 1.0E-9 | 1.0E-9 | 3.7E0 | 1.0E-9 | 3.7E1 | 0.0E0 | 1.0E-9 | 1.0E-9 | 3.9E2 | 1.0E-9 | 113 | 24 | 84 | 24 | 0.49 |
| Hq | pg/ml | 1.1E0 | 1.7E0 | 1.2E1 | 9.5E2 | 6.3E1 | 5.6E3 | 1.0E-9 | 1.0E-9 | 9.5E2 | 3.4E4 | 317 | 36 | 136 | 36 | 0.53 |
| Hr | pg/ml | 1.3E2 | 1.1E2 | 8.2E2 | 6.6E2 | 1.5E3 | 9.3E2 | 1.0E-9 | 1.5E1 | 9.7E3 | 3.7E3 | 317 | 36 | 136 | 36 | 0.51 |
| Hu | pg/ml | 1.4E1 | 7.4E-1 | 3.5E3 | 2.0E3 | 3.7E4 | 9.2E3 | 1.0E-9 | 1.0E-9 | 6.3E5 | 5.5E4 | 317 | 36 | 136 | 36 | 0.48 |
| Hv | pg/ml | 1.3E0 | 2.0E0 | 3.6E0 | 3.5E0 | 1.5E1 | 5.4E0 | 1.0E-9 | 1.0E-9 | 2.5E2 | 2.4E1 | 317 | 36 | 136 | 36 | 0.55 |
| Hw | pg/ml | 6.8E0 | 7.2E0 | 2.7E1 | 1.4E1 | 1.1E2 | 1.6E1 | 1.0E-9 | 1.9E-1 | 1.7E3 | 6.7E1 | 317 | 36 | 136 | 36 | 0.51 |
| Hx | pg/ml | 8.3E0 | 1.2E1 | 6.7E1 | 1.8E1 | 5.3E2 | 1.8E1 | 1.0E-9 | 1.0E-9 | 9.3E3 | 7.2E1 | 317 | 36 | 136 | 36 | 0.56 |
| Ib | ng/ml | 6.6E-2 | 5.5E-2 | 7.9E-1 | 4.9E0 | 3.4E0 | 1.3E1 | 1.0E-9 | 1.0E-9 | 3.0E1 | 5.2E1 | 109 | 22 | 85 | 22 | 0.54 |
| Ic | U/ml | 2.2E2 | 2.2E2 | 3.3E2 | 5.1E3 | 1.5E3 | 2.0E4 | 2.9E0 | 2.8E1 | 1.5E4 | 9.3E4 | 109 | 22 | 85 | 22 | 0.68 |
| Id | U/ml | 6.2E-1 | 7.1E-1 | 1.1E0 | 1.9E0 | 1.3E0 | 2.8E0 | 1.0E-9 | 2.7E-1 | 7.2E0 | 1.2E1 | 109 | 22 | 85 | 22 | 0.58 |
| Tt | pg/ml | 1.6E2 | 1.9E2 | 1.7E2 | 1.9E2 | 4.3E1 | 5.2E1 | 8.0E1 | 1.2E2 | 3.0E2 | 2.9E2 | 101 | 19 | 78 | 19 | 0.63 |
| To | pg/ml | 1.6E0 | 1.8E0 | 1.7E0 | 2.0E0 | 1.8E0 | 1.8E0 | 1.0E-9 | 1.0E-9 | 7.1E0 | 5.1E0 | 108 | 20 | 81 | 20 | 0.57 |
| Tr | pg/ml | 2.8E0 | 2.7E0 | 4.0E0 | 6.1E0 | 4.2E0 | 7.6E0 | 3.5E-2 | 3.9E-1 | 2.4E1 | 3.2E1 | 106 | 19 | 80 | 19 | 0.55 |
| Tn | pg/ml | 2.5E1 | 4.4E1 | 4.8E1 | 1.3E2 | 7.0E1 | 2.7E2 | 2.6E0 | 8.8E0 | 3.7E2 | 1.2E3 | 108 | 20 | 81 | 20 | 0.68 |
| Tv | ng/ml | 1.1E1 | 1.7E1 | 2.0E1 | 4.2E1 | 5.0E1 | 7.4E1 | 1.0E-9 | 1.0E-9 | 4.9E2 | 3.2E2 | 108 | 20 | 81 | 20 | 0.62 |
| Ih | ng/ml | 6.8E1 | 7.2E1 | 1.9E2 | 2.1E2 | 3.2E2 | 3.2E2 | 1.0E-9 | 1.0E-9 | 2.5E3 | 1.3E3 | 317 | 36 | 136 | 36 | 0.50 |
| Ii | ng/ml | 8.1E1 | 9.7E1 | 2.9E2 | 1.9E2 | 8.2E2 | 4.2E2 | 7.5E-1 | 7.3E-1 | 8.4E3 | 2.5E3 | 317 | 36 | 136 | 36 | 0.49 |
| Ij | ng/ml | 7.2E1 | 1.1E2 | 2.1E2 | 1.7E2 | 7.5E2 | 2.6E2 | 2.1E0 | 1.0E-9 | 6.4E3 | 1.4E3 | 317 | 36 | 136 | 36 | 0.58 |
| Ik | ng/ml | 1.1E1 | 1.5E1 | 1.0E3 | 3.6E2 | 9.8E3 | 5.9E2 | 5.9E-1 | 8.8E-1 | 1.2E5 | 2.1E3 | 316 | 36 | 136 | 36 | 0.55 |
| Il | ng/ml | 3.8E2 | 3.2E2 | 1.5E3 | 1.0E3 | 3.1E3 | 2.1E3 | 1.0E-9 | 1.0E-9 | 1.2E4 | 1.2E4 | 314 | 36 | 136 | 36 | 0.48 |
| Im | ng/ml | 1.9E2 | 2.3E2 | 3.1E2 | 4.4E2 | 3.5E2 | 6.0E2 | 1.3E1 | 3.0E1 | 3.1E3 | 3.2E3 | 316 | 36 | 136 | 36 | 0.55 |
| In | ng/ml | 4.2E0 | 2.4E0 | 3.5E1 | 7.9E0 | 2.4E2 | 1.2E1 | 1.0E-9 | 1.0E-9 | 3.9E3 | 5.2E1 | 317 | 36 | 136 | 36 | 0.43 |
| Hb | ng/ml | 2.0E1 | 1.8E1 | 2.6E1 | 3.4E1 | 2.4E1 | 3.3E1 | 1.1E0 | 1.3E0 | 1.3E2 | 1.1E2 | 110 | 23 | 86 | 23 | 0.53 |
| Hc | pg/ml | 6.9E2 | 7.5E2 | 2.0E3 | 6.7E3 | 4.7E3 | 2.1E4 | 1.0E-9 | 2.2E2 | 3.6E4 | 1.0E5 | 110 | 23 | 86 | 23 | 0.54 |
| Hf | ng/ml | 1.4E2 | 1.2E2 | 4.0E2 | 2.0E2 | 5.5E2 | 3.1E2 | 1.0E-9 | 1.0E-9 | 2.5E3 | 1.3E3 | 110 | 23 | 86 | 23 | 0.43 |
| Io | ng/ml | 8.4E3 | 6.1E3 | 1.8E4 | 9.7E3 | 5.5E4 | 8.7E3 | 6.6E1 | 2.1E2 | 8.8E5 | 3.3E4 | 315 | 35 | 135 | 35 | 0.45 |
| Ip | ng/ml | 9.7E0 | 7.7E0 | 1.9E1 | 2.1E1 | 2.6E1 | 2.3E1 | 1.0E-9 | 2.4E-2 | 2.6E2 | 8.3E1 | 315 | 35 | 135 | 35 | 0.52 |
| Iq | ug/ml | 1.1E-1 | 1.1E-1 | 4.4E1 | 4.3E-1 | 7.7E2 | 7.6E-1 | 1.0E-9 | 1.0E-9 | 1.4E4 | 3.9E0 | 315 | 35 | 135 | 35 | 0.54 |
| Ir | ug/ml | 3.2E-1 | 6.3E-1 | 3.2E0 | 1.4E0 | 2.1E1 | 2.7E0 | 1.0E-9 | 1.0E-9 | 2.4E2 | 1.6E1 | 314 | 35 | 135 | 35 | 0.58 |
| Is | ng/ml | 1.4E0 | 2.2E0 | 5.6E0 | 7.7E0 | 1.2E1 | 1.9E1 | 1.0E-9 | 1.0E-9 | 8.8E1 | 1.1E2 | 315 | 35 | 135 | 35 | 0.54 |
| It | ng/ml | 2.0E0 | 2.7E0 | 2.2E1 | 9.2E0 | 1.1E2 | 2.0E1 | 1.0E-9 | 1.0E-9 | 1.2E3 | 8.9E1 | 315 | 35 | 135 | 35 | 0.56 |
| Iu | ng/ml | 2.1E2 | 3.3E2 | 1.5E3 | 1.0E3 | 4.8E3 | 2.1E3 | 1.0E-9 | 1.0E-9 | 2.9E4 | 8.3E3 | 315 | 35 | 135 | 35 | 0.55 |
| Iv | ng/ml | 1.3E1 | 1.7E1 | 4.7E1 | 3.4E1 | 1.3E2 | 3.8E1 | 1.0E-9 | 1.0E-9 | 1.6E3 | 1.5E2 | 315 | 34 | 135 | 34 | 0.55 |
| Iz | ng/ml | 1.3E2 | 1.5E2 | 4.0E2 | 4.0E2 | 9.6E2 | 6.0E2 | 1.5E0 | 9.4E0 | 8.4E3 | 2.7E3 | 110 | 23 | 86 | 23 | 0.56 |
| Rc | pg/ml | 5.2E3 | 7.0E3 | 6.3E3 | 8.1E3 | 4.4E3 | 5.6E3 | 1.9E2 | 1.0E3 | 2.2E4 | 1.6E4 | 111 | 23 | 84 | 23 | 0.57 |
| Rb | pg/ml | 7.6E-1 | 9.7E-1 | 2.5E0 | 3.1E0 | 3.6E0 | 8.8E0 | 1.0E-9 | 1.0E-9 | 1.6E1 | 4.3E1 | 111 | 23 | 84 | 23 | 0.51 |
| Pz | ng/ml | 3.3E3 | 1.0E4 | 7.5E3 | 6.8E3 | 1.9E4 | 4.3E3 | 1.3E1 | 2.3E2 | 2.8E5 | 1.4E4 | 314 | 36 | 134 | 36 | 0.58 |
| Qa | ng/ml | 2.8E3 | 4.2E3 | 6.2E3 | 6.6E3 | 8.3E3 | 6.3E3 | 1.5E2 | 6.2E2 | 5.2E4 | 2.5E4 | 314 | 36 | 134 | 36 | 0.60 |
| Qb | ng/ml | 1.0E2 | 1.1E2 | 2.6E2 | 2.0E2 | 6.8E2 | 2.3E2 | 7.9E-1 | 1.6E1 | 8.3E3 | 1.1E3 | 314 | 36 | 134 | 36 | 0.53 |
| Qc | ng/ml | 2.5E2 | 1.9E2 | 5.2E2 | 3.7E2 | 1.0E3 | 4.3E2 | 8.1E-1 | 5.0E0 | 1.1E4 | 1.6E3 | 314 | 36 | 134 | 36 | 0.47 |
| Qd | ng/ml | 9.5E3 | 1.0E4 | 2.8E4 | 1.7E4 | 1.3E5 | 2.0E4 | 2.4E2 | 1.0E3 | 2.0E6 | 8.7E4 | 314 | 36 | 134 | 36 | 0.52 |
| Qe | ng/ml | 7.8E2 | 1.3E3 | 2.0E3 | 1.6E3 | 5.9E3 | 1.8E3 | 7.6E0 | 7.9E1 | 9.7E4 | 9.3E3 | 314 | 36 | 134 | 36 | 0.57 |
| Jd | ng/ml | 3.6E-1 | 7.8E-1 | 7.1E0 | 6.3E0 | 6.2E1 | 1.9E1 | 1.0E-9 | 1.0E-9 | 6.5E2 | 9.1E1 | 111 | 23 | 86 | 23 | 0.63 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|-----|--------|-----|-----|
| | | NonDis | Dis | NonDis | Dis | NonDis | Dis | NonDis | Dis | NonDis | Dis | NonDis | Dis | NonDis | Dis | |
| Je | ng/ml | 1.0E-9 | 8.1E-1 | 1.4E0 | 5.4E0 | 5.6E0 | 1.8E1 | 1.0E-9 | 1.0E-9 | 5.6E1 | 8.8E1 | 111 | 23 | 86 | 23 | 0.62 |
| Jf | ng/ml | 1.0E-9 | 1.0E-9 | 8.1E-1 | 1.5E0 | 2.1E0 | 2.9E0 | 1.0E-9 | 1.0E-9 | 1.2E1 | 8.6E0 | 111 | 23 | 86 | 23 | 0.54 |
| Jg | ng/ml | 3.7E2 | 7.4E2 | 7.2E2 | 8.9E2 | 1.1E3 | 8.3E2 | 5.8E0 | 1.4E1 | 1.0E4 | 4.0E3 | 317 | 36 | 136 | 36 | 0.61 |
| Jh | ng/ml | 2.3E0 | 5.2E0 | 2.5E1 | 5.4E1 | 1.0E2 | 2.6E2 | 1.0E-9 | 1.0E-9 | 1.2E3 | 1.6E3 | 317 | 36 | 136 | 36 | 0.55 |
| Ji | ng/ml | 4.6E1 | 4.4E1 | 6.7E1 | 8.1E1 | 6.9E1 | 8.1E1 | 1.1E0 | 5.1E0 | 5.3E2 | 3.8E2 | 317 | 36 | 136 | 36 | 0.54 |
| Sr | pg/mL | 3.0E2 | 3.8E2 | 7.6E2 | 1.3E3 | 1.3E3 | 1.7E3 | 1.0E-9 | 1.0E-9 | 9.8E3 | 5.5E3 | 106 | 23 | 82 | 23 | 0.59 |
| Ss | pg/mL | 7.6E4 | 2.0E5 | 1.3E5 | 2.2E5 | 1.3E5 | 3.5E5 | 9.5E3 | 1.3E4 | 6.7E5 | 1.8E6 | 106 | 23 | 82 | 23 | 0.61 |
| St | pg/mL | 2.2E7 | 2.0E7 | 4.7E7 | 4.9E7 | 6.0E7 | 5.3E7 | 7.8E5 | 3.4E6 | 4.1E8 | 1.9E8 | 108 | 22 | 82 | 22 | 0.53 |
| Ra | pg/ml | 1.0E-9 | 1.0E-9 | 7.5E-1 | 6.9E-1 | 1.4E0 | 1.3E0 | 1.0E-9 | 1.0E-9 | 7.3E0 | 3.8E0 | 111 | 23 | 84 | 23 | 0.53 |
| Qz | pg/ml | 1.1E1 | 1.0E-9 | 6.9E1 | 4.7E1 | 1.1E2 | 1.1E2 | 1.0E-9 | 1.0E-9 | 7.1E2 | 4.7E2 | 111 | 23 | 84 | 23 | 0.33 |
| Qy | pg/ml | 4.1E-1 | 5.6E-1 | 2.9E0 | 5.5E1 | 1.5E1 | 1.7E2 | 1.0E-9 | 1.0E-9 | 1.6E2 | 6.5E2 | 111 | 23 | 84 | 23 | 0.61 |
| Qx | pg/ml | 1.0E-9 | 1.0E-9 | 6.4E0 | 2.6E1 | 5.1E1 | 1.2E2 | 1.0E-9 | 1.0E-9 | 5.4E2 | 5.8E2 | 111 | 23 | 84 | 23 | 0.52 |
| Qw | pg/ml | 1.8E-1 | 1.0E-9 | 1.4E0 | 4.0E0 | 3.7E0 | 1.8E1 | 1.0E-9 | 1.0E-9 | 3.0E1 | 8.6E1 | 111 | 23 | 84 | 23 | 0.36 |
| Qv | pg/ml | 2.3E4 | 1.2E4 | 3.7E4 | 5.9E4 | 7.7E4 | 1.9E5 | 1.2E3 | 1.6E3 | 7.4E5 | 9.4E5 | 111 | 23 | 84 | 23 | 0.38 |
| Qu | pg/ml | 5.1E0 | 2.7E1 | 7.9E1 | 7.5E1 | 1.7E2 | 1.4E2 | 1.0E-9 | 1.0E-9 | 8.0E2 | 5.1E2 | 111 | 23 | 84 | 23 | 0.58 |
| Qt | pg/ml | 1.2E1 | 1.3E1 | 3.0E1 | 9.3E1 | 5.9E1 | 2.1E2 | 1.0E-9 | 1.0E-9 | 3.8E2 | 8.5E2 | 111 | 23 | 84 | 23 | 0.55 |
| Qh | ng/ml | 1.5E1 | 2.3E1 | 3.7E1 | 3.6E1 | 7.2E1 | 4.2E1 | 1.2E-1 | 1.0E-9 | 6.4E2 | 2.0E2 | 111 | 23 | 84 | 23 | 0.59 |
| Qg | ng/ml | 8.9E0 | 4.7E0 | 2.6E1 | 1.1E1 | 1.0E2 | 1.3E1 | 1.5E-1 | 5.1E-2 | 1.0E3 | 5.8E1 | 111 | 23 | 84 | 23 | 0.39 |
| Jj | ng/ml | 8.4E2 | 6.8E2 | 2.9E3 | 1.7E3 | 2.0E4 | 3.5E3 | 2.0E1 | 1.3E1 | 3.4E5 | 1.7E4 | 317 | 36 | 136 | 36 | 0.48 |
| Jk | ng/ml | 3.3E0 | 5.8E0 | 2.4E1 | 2.3E1 | 5.0E1 | 4.1E1 | 1.0E-9 | 2.6E-1 | 2.8E2 | 1.6E2 | 317 | 36 | 136 | 36 | 0.57 |
| Jl | ng/ml | 3.7E-1 | 6.8E-1 | 1.9E0 | 2.6E0 | 4.7E0 | 5.6E0 | 7.6E-4 | 1.6E-2 | 3.2E1 | 2.0E1 | 317 | 36 | 136 | 36 | 0.57 |
| Jm | ng/ml | 1.9E1 | 2.6E1 | 5.1E1 | 3.7E1 | 9.4E1 | 4.3E1 | 1.0E-9 | 6.7E-1 | 1.0E3 | 2.2E2 | 317 | 36 | 136 | 36 | 0.53 |
| Jn | pg/ml | 3.8E-1 | 4.0E-1 | 1.9E0 | 1.5E0 | 7.0E0 | 3.8E0 | 1.0E-9 | 1.0E-9 | 6.2E1 | 2.0E1 | 317 | 35 | 136 | 35 | 0.52 |
| Jo | pg/ml | 4.0E3 | 4.4E3 | 5.0E3 | 5.7E3 | 3.7E3 | 4.9E3 | 4.2E1 | 7.7E2 | 2.0E4 | 2.0E4 | 317 | 36 | 136 | 36 | 0.52 |
| Jp | pg/ml | 6.6E4 | 7.4E4 | 7.0E4 | 7.5E4 | 3.4E4 | 3.1E4 | 2.1E3 | 5.0E3 | 2.1E5 | 1.5E5 | 317 | 36 | 136 | 36 | 0.56 |
| Jq | pg/ml | 9.2E1 | 7.8E1 | 1.4E2 | 1.8E2 | 1.6E2 | 2.1E2 | 2.6E0 | 1.3E1 | 1.1E3 | 7.9E2 | 317 | 36 | 136 | 36 | 0.51 |
| Jr | pg/ml | 3.8E0 | 4.1E0 | 1.8E1 | 2.1E1 | 6.6E1 | 6.9E1 | 1.0E-9 | 1.0E-9 | 7.9E2 | 4.1E2 | 317 | 36 | 136 | 36 | 0.50 |
| Js | pg/ml | 1.3E1 | 1.1E1 | 4.5E1 | 2.6E1 | 1.5E2 | 5.2E1 | 1.0E-9 | 1.0E-9 | 1.6E3 | 2.9E2 | 317 | 36 | 136 | 36 | 0.47 |
| Jt | pg/ml | 2.4E3 | 2.9E3 | 3.0E3 | 3.2E3 | 2.1E3 | 1.7E3 | 1.5E2 | 2.6E2 | 1.2E4 | 6.6E3 | 317 | 36 | 136 | 36 | 0.57 |
| Ju | mIU/ml | 7.2E0 | 1.7E1 | 2.2E1 | 2.4E1 | 3.6E1 | 2.7E1 | 6.5E-2 | 1.5E-1 | 2.3E2 | 1.0E2 | 111 | 23 | 86 | 23 | 0.58 |
| Jv | mIU/ml | 1.1E1 | 1.2E1 | 3.9E1 | 3.7E1 | 7.4E1 | 5.7E1 | 1.0E-2 | 1.2E-1 | 4.4E2 | 2.2E2 | 111 | 23 | 86 | 23 | 0.51 |
| Jy | ng/ml | 1.5E-3 | 1.9E-3 | 2.3E-3 | 2.8E-3 | 4.9E-3 | 4.2E-3 | 1.7E-4 | 2.2E-4 | 5.2E-2 | 2.2E-2 | 111 | 23 | 86 | 23 | 0.56 |
| Kc | pg/ml | 2.1E1 | 3.2E1 | 3.5E1 | 5.3E1 | 4.0E1 | 5.5E1 | 1.0E-9 | 1.0E-9 | 1.7E2 | 1.9E2 | 111 | 23 | 86 | 23 | 0.60 |
| Kd | pg/ml | 1.0E-9 | 1.0E-9 | 2.4E2 | 1.7E2 | 7.3E2 | 4.6E2 | 1.0E-9 | 1.0E-9 | 4.8E3 | 1.8E3 | 111 | 23 | 86 | 23 | 0.51 |
| Ke | pg/ml | 9.6E3 | 1.4E4 | 1.3E4 | 1.6E4 | 9.5E3 | 1.2E4 | 3.4E2 | 1.3E3 | 5.9E4 | 5.0E4 | 111 | 23 | 86 | 23 | 0.54 |
| Kf | pg/mL | 5.9E0 | 1.0E1 | 6.5E0 | 9.1E0 | 5.7E0 | 6.0E0 | 1.0E-9 | 1.0E-9 | 2.6E1 | 2.2E1 | 111 | 23 | 86 | 23 | 0.64 |
| Kg | pg/mL | 9.9E2 | 1.3E3 | 1.5E3 | 2.5E3 | 1.5E3 | 3.0E3 | 7.3E1 | 1.3E2 | 8.4E3 | 1.3E4 | 111 | 23 | 86 | 23 | 0.57 |
| Ki | pg/ml | 6.4E1 | 4.9E1 | 7.2E1 | 4.7E1 | 5.2E1 | 2.6E1 | 1.0E-9 | 1.0E-9 | 2.9E2 | 1.1E2 | 111 | 23 | 86 | 23 | 0.33 |
| Kj | pg/ml | 9.3E2 | 1.1E3 | 1.5E3 | 1.8E3 | 1.7E3 | 1.5E3 | 1.4E1 | 9.4E1 | 1.0E4 | 5.0E3 | 111 | 23 | 86 | 23 | 0.59 |
| Kk | pg/ml | 6.9E0 | 5.2E0 | 1.1E1 | 1.4E1 | 1.8E1 | 1.6E1 | 1.0E-9 | 1.0E-9 | 1.6E2 | 4.8E1 | 111 | 23 | 86 | 23 | 0.49 |
| Kl | pg/ml | 1.8E4 | 2.9E4 | 2.5E4 | 3.5E4 | 2.2E4 | 2.9E4 | 1.6E2 | 1.3E3 | 1.1E5 | 1.1E5 | 111 | 23 | 86 | 23 | 0.61 |
| Kn | pg/ml | 1.5E1 | 5.2E1 | 5.8E1 | 9.1E1 | 1.1E2 | 1.1E2 | 1.0E-9 | 1.0E-9 | 7.3E2 | 3.8E2 | 111 | 23 | 86 | 23 | 0.64 |
| Ko | pg/ml | 3.0E2 | 5.4E2 | 3.9E2 | 5.1E2 | 4.0E2 | 4.0E2 | 1.0E-9 | 1.0E-9 | 1.8E3 | 1.5E3 | 111 | 23 | 86 | 23 | 0.58 |
| Kp | pg/ml | 3.2E2 | 4.0E2 | 3.4E2 | 3.9E2 | 3.0E2 | 2.4E2 | 1.0E-9 | 1.0E-9 | 1.7E3 | 8.6E2 | 111 | 23 | 86 | 23 | 0.58 |
| Kq | pg/ml | 2.8E2 | 3.6E2 | 4.2E2 | 7.7E2 | 9.7E2 | 1.4E3 | 5.1E0 | 8.8E1 | 9.8E3 | 7.1E3 | 106 | 23 | 82 | 23 | 0.64 |
| Kr | pg/ml | 5.0E-1 | 1.0E-9 | 2.5E0 | 1.1E0 | 4.9E0 | 1.8E0 | 1.0E-9 | 1.0E-9 | 3.5E1 | 5.5E0 | 106 | 23 | 82 | 23 | 0.42 |
| Ks | pg/ml | 1.5E4 | 1.4E4 | 2.1E4 | 1.7E4 | 2.0E4 | 1.4E4 | 3.8E2 | 3.2E2 | 1.1E5 | 5.0E4 | 106 | 23 | 82 | 23 | 0.45 |
| Kx | ng/ml | 1.0E-9 | 1.0E-9 | 4.6E-3 | 1.0E-2 | 8.6E-3 | 2.3E-2 | 1.0E-9 | 1.0E-9 | 5.1E-2 | 1.0E-1 | 110 | 23 | 86 | 23 | 0.54 |
| Ky | ng/ml | 1.0E-1 | 1.0E-1 | 3.5E-1 | 4.8E-1 | 7.1E-1 | 1.3E0 | 1.0E-9 | 1.0E-9 | 5.1E0 | 6.4E0 | 110 | 23 | 86 | 23 | 0.56 |
| Kz | ng/ml | 1.0E-9 | 4.7E-3 | 3.7E-3 | 4.5E-3 | 6.3E-3 | 4.6E-3 | 1.0E-9 | 1.0E-9 | 3.6E-2 | 1.4E-2 | 110 | 23 | 86 | 23 | 0.61 |
| Ld | pg/ml | 1.0E-9 | 7.5E-1 | 4.0E0 | 3.3E0 | 9.9E0 | 4.4E0 | 1.0E-9 | 1.0E-9 | 6.9E1 | 1.9E1 | 108 | 23 | 85 | 23 | 0.57 |
| Lh | pg/ml | 1.1E4 | 1.6E4 | 2.0E4 | 2.2E4 | 2.9E4 | 2.6E4 | 1.0E-9 | 1.8E2 | 2.6E5 | 1.5E5 | 315 | 36 | 136 | 36 | 0.59 |
| Li | pg/ml | 2.6E3 | 4.6E3 | 8.5E3 | 1.2E4 | 2.3E4 | 1.7E4 | 1.0E-9 | 5.6E1 | 2.9E5 | 6.8E4 | 315 | 36 | 136 | 36 | 0.56 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Lj | pg/ml | 1.8E3 | 2.5E3 | 1.1E4 | 1.3E4 | 4.2E4 | 3.0E4 | 1.4E1 | 1.0E-9 | 4.4E5 | 1.7E5 | 315 | 36 | 136 | 36 | 0.53 |
| Rm | ng/ml | 1.7E1 | 1.8E1 | 5.8E1 | 4.8E1 | 8.8E1 | 6.5E1 | 2.2E-1 | 3.0E0 | 4.0E2 | 2.5E2 | 110 | 23 | 83 | 23 | 0.50 |
| Rh | ng/ml | 1.4E2 | 7.9E1 | 2.9E2 | 1.7E2 | 5.7E2 | 1.9E2 | 4.7E0 | 7.5E0 | 3.8E3 | 5.8E2 | 110 | 23 | 83 | 23 | 0.40 |
| Ri | ng/ml | 1.0E-9 | 1.0E-9 | 3.9E0 | 2.4E0 | 9.2E0 | 8.6E0 | 1.0E-9 | 1.0E-9 | 7.4E1 | 4.1E1 | 111 | 23 | 84 | 23 | 0.38 |
| Rg | ng/ml | 1.0E-9 | 1.0E-9 | 2.7E-2 | 5.6E-3 | 2.6E-1 | 1.6E-2 | 1.0E-9 | 1.0E-9 | 2.7E0 | 6.7E-2 | 110 | 23 | 83 | 23 | 0.55 |
| Rj | ng/ml | 1.0E-9 | 1.0E-9 | 1.7E0 | 2.9E-1 | 7.9E0 | 6.9E-1 | 1.0E-9 | 1.0E-9 | 8.0E1 | 2.5E0 | 111 | 23 | 84 | 23 | 0.45 |
| Rf | ng/ml | 3.6E-1 | 4.0E-1 | 8.3E-1 | 7.3E-1 | 1.6E0 | 9.0E-1 | 7.8E-3 | 2.8E-2 | 1.4E1 | 3.8E0 | 110 | 23 | 83 | 23 | 0.50 |
| Ql | pg/ml | 7.3E0 | 5.5E0 | 1.4E1 | 9.1E0 | 3.1E1 | 1.4E1 | 1.0E-9 | 1.0E-9 | 2.9E2 | 6.3E1 | 111 | 23 | 86 | 23 | 0.45 |
| Qm | pg/ml | 1.7E0 | 1.0E-9 | 1.9E1 | 1.9E1 | 4.4E1 | 3.0E1 | 1.0E-9 | 1.0E-9 | 3.6E2 | 1.1E2 | 111 | 23 | 86 | 23 | 0.50 |
| Qn | pg/ml | 6.1E-1 | 5.6E-1 | 7.5E0 | 9.2E0 | 2.6E1 | 2.0E1 | 1.0E-9 | 1.0E-9 | 2.2E2 | 7.5E1 | 111 | 23 | 86 | 23 | 0.49 |
| Nv | pg/ml | 3.5E3 | 3.3E3 | 1.3E4 | 2.2E4 | 6.3E4 | 8.8E4 | 1.0E-9 | 7.5E1 | 1.1E6 | 5.3E5 | 318 | 36 | 136 | 36 | 0.53 |
| Nw | pg/ml | 7.4E3 | 1.0E4 | 1.2E4 | 1.5E4 | 2.2E4 | 1.7E4 | 8.6E1 | 7.4E2 | 2.1E5 | 8.9E4 | 318 | 36 | 136 | 36 | 0.55 |
| Nx | pg/ml | 1.7E2 | 2.2E2 | 3.9E2 | 5.4E2 | 7.1E2 | 9.8E2 | 1.0E-9 | 1.0E-9 | 7.4E3 | 5.3E3 | 318 | 36 | 136 | 36 | 0.56 |
| Ny | pg/ml | 4.7E0 | 5.4E0 | 1.1E2 | 7.7E1 | 1.4E3 | 3.4E2 | 1.0E-9 | 1.0E-9 | 2.5E4 | 2.1E3 | 318 | 36 | 136 | 36 | 0.53 |
| Oa | pg/ml | 1.6E2 | 1.1E2 | 4.4E2 | 3.7E2 | 8.0E2 | 5.7E2 | 1.0E-9 | 1.0E-9 | 4.8E3 | 1.8E3 | 111 | 23 | 86 | 23 | 0.44 |
| Wn | ng/ml | 1.2E1 | 1.5E1 | 9.7E1 | 8.0E1 | 3.1E2 | 1.9E2 | 2.5E0 | 3.1E0 | 1.8E3 | 6.1E2 | 33 | 10 | 27 | 10 | 0.50 |
| Tk | ng/ml | 2.0E2 | 1.3E2 | 4.3E2 | 2.1E2 | 7.4E2 | 2.1E2 | 2.4E1 | 2.0E1 | 4.2E3 | 5.3E2 | 36 | 10 | 29 | 10 | 0.38 |
| Oe | pg/ml | 9.7E1 | 4.3E1 | 2.7E2 | 2.5E2 | 4.2E2 | 3.5E2 | 1.0E-9 | 1.0E-9 | 2.7E3 | 1.5E3 | 314 | 36 | 136 | 36 | 0.51 |
| Of | pg/ml | 2.1E2 | 3.2E2 | 5.6E3 | 1.2E4 | 1.8E4 | 3.7E4 | 1.0E-9 | 9.7E0 | 1.8E5 | 1.6E5 | 317 | 36 | 136 | 36 | 0.55 |
| Og | pg/ml | 9.4E-2 | 9.5E-2 | 7.2E-1 | 3.2E-1 | 2.2E0 | 8.5E-1 | 1.0E-9 | 1.0E-9 | 2.0E1 | 5.0E0 | 317 | 36 | 136 | 36 | 0.46 |
| Oh | pg/ml | 2.0E0 | 2.8E0 | 2.5E1 | 1.4E1 | 1.2E2 | 4.5E1 | 1.0E-9 | 1.0E-9 | 1.4E3 | 2.6E2 | 317 | 36 | 136 | 36 | 0.56 |
| Oi | pg/ml | 2.9E0 | 2.7E0 | 7.2E0 | 5.2E0 | 1.1E1 | 8.0E0 | 1.0E-9 | 1.0E-9 | 8.6E1 | 4.2E1 | 317 | 36 | 136 | 36 | 0.49 |
| Ok | pg/ml | 3.2E2 | 5.4E2 | 4.3E2 | 6.9E2 | 3.9E2 | 6.1E2 | 1.3E1 | 2.7E1 | 3.1E3 | 3.1E3 | 317 | 36 | 136 | 36 | 0.64 |
| Om | pg/ml | 3.6E2 | 4.1E2 | 7.6E2 | 1.2E3 | 2.1E3 | 3.2E3 | 1.0E-9 | 4.6E1 | 3.0E4 | 2.0E4 | 317 | 36 | 136 | 36 | 0.55 |
| On | pg/ml | 1.4E2 | 2.2E2 | 2.8E2 | 3.8E2 | 4.8E2 | 5.9E2 | 8.4E-1 | 7.9E0 | 4.5E3 | 3.3E3 | 317 | 36 | 136 | 36 | 0.61 |
| Or | pg/ml | 1.0E1 | 2.5E1 | 3.1E1 | 5.4E1 | 6.4E1 | 7.7E1 | 1.0E-9 | 1.0E-9 | 5.0E2 | 3.4E2 | 110 | 23 | 86 | 23 | 0.64 |
| Ow | pg/ml | 2.8E1 | 4.4E1 | 1.0E2 | 5.3E2 | 3.0E2 | 1.7E3 | 1.0E-9 | 1.0E-9 | 2.3E3 | 8.1E3 | 110 | 23 | 86 | 23 | 0.60 |
| Ou | pg/ml | 4.4E2 | 7.4E2 | 8.4E2 | 1.2E3 | 1.2E3 | 1.6E3 | 1.0E-9 | 7.0E1 | 9.4E3 | 6.6E3 | 110 | 23 | 86 | 23 | 0.57 |
| Ul | ng/ml | 1.0E-9 | 1.0E-9 | 1.1E0 | 8.3E-1 | 3.7E0 | 4.0E0 | 1.0E-9 | 1.0E-9 | 2.2E1 | 1.9E1 | 119 | 23 | 88 | 23 | 0.47 |
| Uo | ng/ml | 1.0E-9 | 1.0E-9 | 1.1E-1 | 9.7E-2 | 2.4E-1 | 1.9E-1 | 1.0E-9 | 1.0E-9 | 1.3E0 | 5.5E-1 | 119 | 23 | 88 | 23 | 0.48 |
| Uk | ng/ml | 1.0E-9 | 1.0E-9 | 4.8E-3 | 2.3E-2 | 1.2E-2 | 7.0E-2 | 1.0E-9 | 1.0E-9 | 9.9E-2 | 3.2E-1 | 119 | 23 | 88 | 23 | 0.51 |
| Um | ng/ml | 1.0E-9 | 1.0E-9 | 3.8E-1 | 8.5E-1 | 9.8E-1 | 1.2E0 | 1.0E-9 | 1.0E-9 | 6.8E0 | 3.6E0 | 119 | 23 | 88 | 23 | 0.56 |
| Uf | ng/ml | 4.5E-2 | 6.3E-2 | 9.4E-2 | 4.9E-1 | 1.2E-1 | 1.8E0 | 2.8E-3 | 3.6E-3 | 6.6E-1 | 8.6E0 | 119 | 23 | 88 | 23 | 0.62 |
| Uh | ng/ml | 1.8E0 | 1.9E0 | 2.5E0 | 3.7E0 | 2.3E0 | 4.7E0 | 3.2E-2 | 6.8E-2 | 1.1E1 | 2.1E1 | 119 | 23 | 88 | 23 | 0.54 |
| Un | ng/ml | 1.6E0 | 2.2E0 | 1.9E0 | 2.1E0 | 1.2E0 | 1.1E0 | 2.0E-1 | 2.6E-1 | 7.0E0 | 3.9E0 | 119 | 23 | 88 | 23 | 0.56 |
| Ug | ng/ml | 1.4E1 | 1.5E1 | 2.7E1 | 2.3E1 | 2.7E1 | 2.0E1 | 6.9E-1 | 2.3E0 | 1.3E2 | 6.8E1 | 119 | 23 | 88 | 23 | 0.45 |
| Ur | ng/ml | 1.6E-1 | 1.6E-1 | 1.2E0 | 6.9E-1 | 8.6E0 | 1.2E0 | 1.0E-9 | 1.0E-9 | 9.3E1 | 4.1E0 | 119 | 23 | 88 | 23 | 0.55 |
| Up | ng/ml | 1.0E-9 | 1.0E-9 | 7.5E-3 | 8.4E-4 | 3.4E-2 | 2.3E-3 | 1.0E-9 | 1.0E-9 | 3.3E-1 | 1.1E-2 | 119 | 23 | 88 | 23 | 0.46 |
| Us | ng/ml | 4.4E-3 | 1.0E-9 | 2.1E-2 | 5.0E-3 | 5.6E-2 | 9.5E-3 | 1.0E-9 | 1.0E-9 | 4.0E-1 | 3.6E-2 | 119 | 23 | 88 | 23 | 0.35 |
| Uv | ng/ml | 3.2E-3 | 2.0E-3 | 1.2E-2 | 1.1E-2 | 3.1E-2 | 3.3E-2 | 1.0E-9 | 1.0E-9 | 2.3E-1 | 1.6E-1 | 119 | 23 | 88 | 23 | 0.42 |
| Ut | ng/ml | 5.3E-1 | 8.5E-1 | 2.3E0 | 5.2E0 | 8.9E0 | 1.6E1 | 1.0E-9 | 1.0E-9 | 7.2E1 | 7.8E1 | 119 | 23 | 88 | 23 | 0.56 |
| Uu | ng/ml | 6.9E0 | 7.3E0 | 7.8E0 | 8.0E0 | 5.1E0 | 3.7E0 | 8.1E-1 | 9.8E-1 | 2.6E1 | 1.4E1 | 119 | 23 | 88 | 23 | 0.55 |
| Vp | ng/ml | 1.0E-9 | 1.0E-9 | 7.4E-1 | 3.2E-1 | 5.2E0 | 1.1E0 | 1.0E-9 | 1.0E-9 | 5.0E1 | 5.2E0 | 119 | 23 | 88 | 23 | 0.53 |
| Vt | ng/ml | 6.1E0 | 6.2E0 | 7.6E0 | 8.0E0 | 6.1E0 | 7.4E0 | 6.0E-1 | 7.0E-1 | 3.2E1 | 3.0E1 | 119 | 23 | 88 | 23 | 0.48 |
| Vu | ng/ml | 1.0E-9 | 1.0E-9 | 2.3E0 | 3.2E0 | 6.0E0 | 8.2E0 | 1.0E-9 | 1.0E-9 | 5.1E1 | 3.8E1 | 118 | 22 | 88 | 22 | 0.53 |
| Vq | ng/ml | 1.2E2 | 3.3E2 | 5.5E2 | 8.9E2 | 1.2E3 | 1.1E3 | 2.0E-1 | 2.6E0 | 1.0E4 | 4.3E3 | 94 | 20 | 72 | 20 | 0.64 |
| Vo | ng/ml | 2.6E1 | 2.5E1 | 2.5E1 | 2.5E1 | 4.9E0 | 2.5E0 | 2.5E0 | 2.0E1 | 3.5E1 | 3.0E1 | 119 | 23 | 88 | 23 | 0.44 |
| Vs | ng/ml | 1.0E-9 | 1.9E0 | 6.5E0 | 1.7E1 | 2.7E1 | 4.8E1 | 1.0E-9 | 1.0E-9 | 2.3E2 | 2.2E2 | 116 | 23 | 86 | 23 | 0.57 |
| Vv | ng/ml | 2.7E0 | 4.1E0 | 6.0E0 | 5.4E0 | 1.0E1 | 6.0E0 | 1.0E-9 | 1.0E-9 | 8.1E1 | 2.6E1 | 118 | 23 | 88 | 23 | 0.54 |
| Oy | pg/ml | 5.5E-1 | 7.1E-1 | 8.7E0 | 9.2E0 | 4.2E1 | 4.2E1 | 1.0E-9 | 1.0E-9 | 4.0E2 | 2.5E2 | 317 | 36 | 136 | 36 | 0.50 |
| Oz | pg/ml | 4.5E-3 | 4.0E-2 | 2.2E-1 | 1.8E-1 | 3.7E-1 | 3.1E-1 | 1.0E-9 | 1.0E-9 | 2.6E0 | 1.5E0 | 317 | 36 | 136 | 36 | 0.50 |
| Pa | pg/ml | 3.4E-1 | 3.7E-1 | 1.3E0 | 1.0E0 | 5.8E0 | 2.5E0 | 1.0E-9 | 1.0E-9 | 8.6E1 | 1.4E1 | 317 | 36 | 136 | 36 | 0.54 |
| Pb | pg/ml | 1.0E-9 | 1.0E-9 | 1.8E0 | 2.4E-1 | 2.7E1 | 8.9E-1 | 1.0E-9 | 1.0E-9 | 4.9E2 | 5.3E0 | 317 | 36 | 136 | 36 | 0.51 |

| Code | Units | Median | | Average | | Standard Deviation | | Minimum | | Maximum | | Number of Samples | | Number of Patients | | AUC |
|------|-------|--------|--|---------|--|--------------------|--|---------|--|---------|--|-------------------|--|--------------------|--|-----|
| | | NonDis | Dis | NonDis | Dis | NonDis | Dis | NonDis | Dis | NonDis | Dis | NonDis | Dis | NonDis | Dis | |
| Pc | pg/ml | 4.4E-2 | 2.6E-1 | 4.3E-1 | 3.4E-1 | 1.3E0 | 4.3E-1 | 1.0E-9 | 1.0E-9 | 1.6E1 | 1.8E0 | 317 | 36 | 136 | 36 | 0.55 |
| Pd | pg/ml | 1.8E0 | 2.0E0 | 3.8E0 | 3.6E0 | 8.2E0 | 5.4E0 | 1.0E-9 | 1.0E-9 | 9.4E1 | 2.4E1 | 317 | 36 | 136 | 36 | 0.52 |
| Pe | pg/ml | 1.8E1 | 2.1E1 | 8.9E1 | 1.0E2 | 3.5E2 | 1.9E2 | 1.0E-9 | 1.0E-9 | 4.7E3 | 7.3E2 | 317 | 36 | 136 | 36 | 0.56 |
| Pf | pg/ml | 1.2E0 | 2.1E0 | 6.2E0 | 1.2E1 | 2.6E1 | 3.2E1 | 1.0E-9 | 1.0E-9 | 3.3E2 | 1.8E2 | 317 | 36 | 136 | 36 | 0.59 |
| Pg | pg/ml | 3.2E0 | 4.6E0 | 4.0E1 | 1.1E2 | 2.1E2 | 5.7E2 | 1.0E-9 | 1.0E-9 | 3.2E3 | 3.4E3 | 317 | 36 | 136 | 36 | 0.51 |
| Ph | ng/ml | 1.4E-1 | 1.8E-1 | 2.7E-1 | 3.6E-1 | 3.5E-1 | 6.2E-1 | 1.0E-9 | 1.0E-9 | 2.2E0 | 2.9E0 | 110 | 23 | 86 | 23 | 0.51 |
| Pi | ng/ml | 1.9E-1 | 1.5E-1 | 2.8E-1 | 2.4E-1 | 4.4E-1 | 2.6E-1 | 1.0E-9 | 1.0E-9 | 3.2E0 | 8.6E-1 | 110 | 23 | 86 | 23 | 0.46 |
| Pj | ng/mL | 4.4E0 | 5.4E0 | 5.1E0 | 5.4E0 | 3.3E0 | 3.4E0 | 3.8E-2 | 3.8E-1 | 1.6E1 | 1.5E1 | 110 | 23 | 86 | 23 | 0.53 |
| Pk | ng/ml | 8.1E-3 | 9.9E-3 | 1.1E-2 | 1.4E-2 | 1.2E-2 | 1.2E-2 | 1.0E-9 | 1.0E-9 | 5.9E-2 | 4.6E-2 | 110 | 23 | 86 | 23 | 0.57 |
| aA | mg/dL | 8.0E-1 | 8.3E-1 | 9.2E-1 | 1.0E0 | 4.3E-1 | 6.1E-1 | 3.0E-1 | 3.0E-1 | 4.2E0 | 3.2E0 | 1242 | 45 | 226 | 45 | 0.52 |
| aC | mg/mL | 3.1E0 | 2.2E0 | 3.3E0 | 2.5E0 | 1.4E0 | 1.0E0 | 1.1E0 | 1.1E0 | 8.2E0 | 5.9E0 | 221 | 23 | 91 | 23 | 0.31 |
| aD | ug/mL | 2.9E0 | 3.2E0 | 4.1E0 | 4.4E0 | 3.2E0 | 3.5E0 | 8.5E-1 | 1.1E0 | 2.8E1 | 1.5E1 | 221 | 23 | 91 | 23 | 0.50 |
| aE | mg/mL | 5.8E-1 | 5.5E-1 | 5.8E-1 | 5.6E-1 | 1.5E-1 | 1.0E-1 | 2.1E-1 | 4.4E-1 | 1.1E0 | 7.8E-1 | 221 | 23 | 91 | 23 | 0.45 |
| aF | ng/mL | 2.1E0 | 2.2E0 | 4.4E0 | 5.0E0 | 7.0E0 | 6.8E0 | 4.3E-3 | 4.3E-3 | 5.0E1 | 2.9E1 | 221 | 23 | 91 | 23 | 0.52 |
| aG | mg/mL | 1.3E-1 | 1.5E-1 | 1.5E-1 | 1.7E-1 | 7.8E-2 | 8.0E-2 | 5.0E-2 | 5.6E-2 | 5.0E-1 | 3.5E-1 | 221 | 23 | 91 | 23 | 0.58 |
| aH | ug/mL | 6.8E1 | 7.5E1 | 7.8E1 | 7.9E1 | 3.5E1 | 3.9E1 | 1.5E1 | 2.3E1 | 2.7E2 | 2.0E2 | 221 | 23 | 91 | 23 | 0.51 |
| aI | ug/mL | 1.8E2 | 1.9E2 | 1.9E2 | 1.8E2 | 6.0E1 | 5.6E1 | 5.8E1 | 7.7E1 | 3.7E2 | 2.7E2 | 221 | 23 | 91 | 23 | 0.48 |
| aJ | ug/mL | 2.5E0 | 2.5E0 | 3.0E0 | 3.4E0 | 1.9E0 | 3.3E0 | 9.0E-1 | 1.0E0 | 1.2E1 | 1.6E1 | 221 | 23 | 91 | 23 | 0.52 |
| aK | ng/mL | 1.8E0 | 1.2E0 | 2.7E0 | 2.3E0 | 2.7E0 | 2.3E0 | 8.4E-2 | 1.0E-1 | 1.8E1 | 7.5E0 | 221 | 23 | 91 | 23 | 0.43 |
| aL | mg/mL | 8.1E-1 | 8.0E-1 | 8.3E-1 | 7.8E-1 | 2.4E-1 | 2.6E-1 | 2.2E-1 | 2.9E-1 | 1.6E0 | 1.4E0 | 221 | 23 | 91 | 23 | 0.45 |
| aM | U/mL | 1.9E1 | 2.3E1 | 3.7E1 | 3.4E1 | 1.1E2 | 3.3E1 | 4.2E-2 | 4.2E-2 | 1.6E3 | 1.2E2 | 221 | 23 | 91 | 23 | 0.57 |
| aN | U/mL | 1.0E1 | 1.5E1 | 1.8E1 | 1.9E1 | 2.1E1 | 2.2E1 | 2.5E-3 | 2.5E-3 | 1.3E2 | 9.7E1 | 221 | 23 | 91 | 23 | 0.53 |
| aO | pg/mL | 3.9E1 | 7.5E1 | 3.7E2 | 3.5E2 | 9.9E2 | 6.5E2 | 6.0E-2 | 6.0E-2 | 6.6E3 | 2.4E3 | 221 | 23 | 91 | 23 | 0.52 |
| aP | ng/mL | 1.7E0 | 1.7E0 | 1.9E0 | 2.1E0 | 1.1E0 | 1.4E0 | 5.4E-1 | 7.9E-1 | 6.6E0 | 6.4E0 | 221 | 23 | 91 | 23 | 0.52 |
| aQ | ng/mL | 3.2E-1 | 3.0E-1 | 5.0E-1 | 3.2E-1 | 5.2E-1 | 2.8E-1 | 1.9E-2 | 6.3E-2 | 4.0E0 | 1.3E0 | 221 | 23 | 91 | 23 | 0.39 |
| aR | ng/mL | 1.6E0 | 2.3E0 | 2.4E0 | 3.4E0 | 2.5E0 | 3.0E0 | 1.8E-1 | 5.9E-1 | 2.1E1 | 1.3E1 | 221 | 23 | 91 | 23 | 0.62 |
| aS | ng/mL | 2.4E-1 | 2.1E-1 | 7.8E-1 | 4.6E-1 | 2.5E0 | 5.0E-1 | 4.2E-3 | 7.2E-2 | 3.3E1 | 2.1E0 | 221 | 23 | 91 | 23 | 0.53 |
| aU | pg/mL | 8.8E1 | 5.5E1 | 1.4E2 | 1.0E2 | 1.6E2 | 1.3E2 | 7.4E0 | 6.5E0 | 1.3E3 | 5.8E2 | 221 | 23 | 91 | 23 | 0.38 |
| aV | ng/mL | 7.3E-1 | 5.8E-1 | 1.1E0 | 1.1E0 | 1.1E0 | 1.5E0 | 3.1E-2 | 9.2E-2 | 8.7E0 | 6.0E0 | 221 | 23 | 91 | 23 | 0.43 |
| aW | pg/mL | 1.7E1 | 2.2E1 | 2.0E1 | 2.1E1 | 2.6E1 | 9.1E0 | 7.2E-2 | 7.2E-2 | 2.4E2 | 3.3E1 | 221 | 23 | 91 | 23 | 0.60 |
| aX | ng/mL | 1.0E1 | 8.2E0 | 1.3E1 | 1.9E1 | 1.2E1 | 4.1E1 | 3.0E-1 | 2.1E0 | 6.7E1 | 2.1E2 | 221 | 23 | 91 | 23 | 0.49 |
| aY | pg/mL | 5.0E1 | 6.0E1 | 7.2E1 | 8.3E1 | 7.1E1 | 6.2E1 | 4.1E-1 | 4.1E-1 | 4.4E2 | 2.4E2 | 221 | 23 | 91 | 23 | 0.59 |
| aZ | pg/mL | 2.2E2 | 3.1E2 | 4.8E2 | 1.2E3 | 7.2E2 | 2.6E3 | 1.7E0 | 1.7E0 | 5.4E3 | 1.2E4 | 221 | 23 | 91 | 23 | 0.55 |
| bA | ng/mL | 8.0E0 | 1.9E1 | 2.6E1 | 5.2E1 | 6.2E1 | 8.0E1 | 3.0E-2 | 3.0E-2 | 7.1E2 | 3.2E2 | 221 | 23 | 91 | 23 | 0.64 |
| bB | ng/mL | 3.1E2 | 2.6E2 | 3.4E2 | 2.7E2 | 1.6E2 | 1.2E2 | 1.6E1 | 6.6E1 | 1.0E3 | 4.9E2 | 221 | 23 | 91 | 23 | 0.40 |
| bC | ng/mL | 3.5E2 | 3.7E2 | 5.1E2 | 7.5E2 | 5.3E2 | 1.2E3 | 2.7E1 | 4.5E1 | 4.0E3 | 4.7E3 | 221 | 23 | 91 | 23 | 0.52 |
| bE | mg/mL | 5.6E0 | 5.4E0 | 5.9E0 | 6.0E0 | 1.7E0 | 2.6E0 | 1.9E0 | 1.9E0 | 1.3E1 | 1.2E1 | 221 | 23 | 91 | 23 | 0.47 |
| bF | pg/mL | 2.0E1 | 3.5E1 | 8.6E1 | 6.4E2 | 4.4E2 | 2.3E3 | 5.0E-2 | 9.1E0 | 5.0E3 | 1.1E4 | 221 | 23 | 91 | 23 | 0.67 |
| bG | ng/mL | 1.8E0 | 1.3E0 | 3.0E0 | 2.0E0 | 3.7E0 | 1.8E0 | 2.2E-2 | 1.1E-1 | 2.6E1 | 7.3E0 | 221 | 23 | 91 | 23 | 0.42 |
| bH | pg/mL | 5.7E-1 | 5.7E-1 | 5.4E0 | 2.3E0 | 2.0E1 | 3.5E0 | 5.7E-1 | 5.7E-1 | 2.8E2 | 1.1E1 | 221 | 23 | 91 | 23 | 0.43 |
| bI | ng/mL | 4.0E-3 | 4.0E-3 | 6.8E-2 | 6.1E-2 | 1.7E-1 | 1.1E-1 | 4.0E-3 | 4.0E-3 | 1.3E0 | 3.7E-1 | 221 | 23 | 91 | 23 | 0.53 |
| bJ | mg/mL | 2.2E0 | 2.7E0 | 2.5E0 | 2.9E0 | 1.9E0 | 2.2E0 | 2.5E-4 | 2.1E-2 | 1.3E1 | 9.0E0 | 221 | 23 | 91 | 23 | 0.55 |
| bL | pg/mL | 4.1E0 | 6.2E0 | 7.8E0 | 9.5E0 | 9.3E0 | 1.0E1 | 4.6E-2 | 4.6E-2 | 4.9E1 | 3.3E1 | 221 | 23 | 91 | 23 | 0.56 |
| bM | mg/mL | 1.5E0 | 1.8E0 | 1.8E0 | 2.4E0 | 1.2E0 | 1.7E0 | 9.2E-3 | 3.3E-1 | 7.1E0 | 8.4E0 | 221 | 23 | 91 | 23 | 0.63 |
| bN | ng/mL | 3.2E1 | 4.3E1 | 1.1E2 | 9.0E1 | 2.6E2 | 1.5E2 | 9.7E-1 | 1.4E-1 | 1.9E3 | 5.7E2 | 221 | 23 | 91 | 23 | 0.52 |
| bO | ng/mL | 4.0E-2 | 4.0E-2 | 1.1E1 | 1.4E1 | 2.4E1 | 2.3E1 | 4.0E-2 | 4.0E-2 | 1.9E2 | 7.3E1 | 221 | 23 | 91 | 23 | 0.51 |
| bP | mg/mL | 5.6E-1 | 4.6E-1 | 7.9E-1 | 6.7E-1 | 6.6E-1 | 5.6E-1 | 8.2E-2 | 1.3E-1 | 3.8E0 | 2.2E0 | 221 | 23 | 91 | 23 | 0.45 |
| bQ | pg/mL | 1.4E1 | 1.7E1 | 2.4E1 | 4.7E1 | 3.5E1 | 8.6E1 | 1.5E-1 | 5.1E0 | 3.2E2 | 4.0E2 | 221 | 23 | 91 | 23 | 0.56 |
| bR | ng/mL | 1.2E-2 | 1.2E-2 | 1.4E-1 | 4.6E-2 | 2.8E-1 | 7.5E-2 | 1.2E-2 | 1.2E-2 | 3.4E0 | 2.3E-1 | 221 | 23 | 91 | 23 | 0.37 |
| bS | pg/mL | 9.4E-1 | 9.4E-1 | 8.1E0 | 3.3E0 | 2.9E1 | 1.1E1 | 9.4E-1 | 9.4E-1 | 3.4E2 | 5.5E1 | 221 | 23 | 91 | 23 | 0.45 |
| bU | ng/mL | 1.6E-1 | 1.1E-1 | 2.1E-1 | 1.1E-1 | 2.6E-1 | 1.0E-1 | 1.3E-2 | 1.3E-2 | 1.8E0 | 3.6E-1 | 221 | 23 | 91 | 23 | 0.39 |
| bV | pg/mL | 4.7E2 | 4.5E2 | 5.3E2 | 5.4E2 | 2.5E2 | 3.2E2 | 1.7E2 | 2.3E2 | 1.6E3 | 1.5E3 | 221 | 23 | 91 | 23 | 0.47 |
| bW | pg/mL | 3.5E2 | 3.0E2 | 4.7E2 | 1.3E3 | 4.2E2 | 4.1E3 | 1.1E2 | 9.9E1 | 3.4E3 | 2.0E4 | 221 | 23 | 91 | 23 | 0.47 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bX | ng/mL | 3.1E-3 | 2.5E-5 | 3.2E-3 | 9.9E-4 | 3.8E-3 | 2.1E-3 | 2.5E-5 | 2.5E-5 | 2.0E-2 | 6.3E-3 | 221 | 23 | 91 | 23 | 0.33 |
| bZ | pg/mL | 2.5E2 | 2.9E2 | 7.5E2 | 6.0E2 | 3.2E2 | 6.9E2 | 1.5E-1 | 5.2E1 | 4.4E4 | 2.2E3 | 221 | 23 | 91 | 23 | 0.55 |
| cA | pg/mL | 6.0E-1 | 6.0E-1 | 1.9E0 | 2.0E0 | 3.4E0 | 3.8E0 | 6.0E-1 | 6.0E-1 | 1.4E1 | 1.6E1 | 221 | 23 | 91 | 23 | 0.50 |
| cB | pg/mL | 6.0E-2 | 2.8E-2 | 9.0E-2 | 3.9E-2 | 1.0E-1 | 4.2E-2 | 1.7E-3 | 1.7E-3 | 5.4E-1 | 1.4E-1 | 221 | 23 | 91 | 23 | 0.33 |
| cC | pg/mL | 4.6E1 | 4.8E1 | 4.9E1 | 4.8E1 | 3.9E1 | 1.8E1 | 1.0E0 | 1.0E0 | 3.7E2 | 8.4E1 | 221 | 23 | 91 | 23 | 0.52 |
| cD | pg/mL | 6.1E0 | 3.4E0 | 1.3E1 | 1.2E1 | 4.0E1 | 2.7E1 | 3.3E-1 | 3.3E-1 | 4.8E2 | 1.3E2 | 221 | 23 | 91 | 23 | 0.42 |
| cE | pg/mL | 3.1E1 | 4.7E1 | 9.3E1 | 4.4E2 | 2.7E2 | 9.4E2 | 1.5E0 | 1.8E0 | 3.1E3 | 3.8E3 | 221 | 23 | 91 | 23 | 0.60 |
| cF | pg/mL | 1.3E1 | 5.3E-1 | 2.4E1 | 5.7E0 | 3.6E1 | 1.0E1 | 5.3E-1 | 5.3E-1 | 2.2E2 | 3.6E1 | 221 | 23 | 91 | 23 | 0.30 |
| cG | pg/mL | 4.2E1 | 3.8E1 | 6.4E1 | 1.1E2 | 8.8E1 | 1.4E2 | 1.1E1 | 1.8E1 | 1.1E3 | 5.6E2 | 221 | 23 | 91 | 23 | 0.51 |
| cH | uIU/mL | 2.9E0 | 1.7E0 | 6.0E0 | 3.7E0 | 8.9E0 | 7.7E0 | 8.6E-3 | 8.6E-3 | 8.7E1 | 3.8E1 | 221 | 23 | 91 | 23 | 0.36 |
| cI | ng/mL | 6.0E0 | 4.2E0 | 1.1E1 | 1.2E1 | 1.3E1 | 2.2E1 | 1.0E-3 | 3.2E-2 | 9.4E1 | 1.0E2 | 221 | 23 | 91 | 23 | 0.41 |
| cJ | ug/mL | 7.2E1 | 6.5E1 | 1.2E2 | 1.0E2 | 1.5E2 | 1.2E2 | 4.0E0 | 1.2E1 | 9.6E2 | 5.5E2 | 221 | 23 | 91 | 23 | 0.48 |
| cK | ng/mL | 3.8E-3 | 3.8E-3 | 7.5E-2 | 6.8E-3 | 2.2E-1 | 1.4E-2 | 3.8E-3 | 3.8E-3 | 1.7E0 | 7.3E-2 | 221 | 23 | 91 | 23 | 0.42 |
| cL | pg/mL | 1.9E2 | 2.2E2 | 2.7E2 | 5.9E2 | 5.1E2 | 9.8E2 | 2.5E1 | 3.6E1 | 7.1E3 | 4.2E3 | 221 | 23 | 91 | 23 | 0.58 |
| cM | pg/mL | 2.9E2 | 2.7E2 | 3.2E2 | 3.0E2 | 1.8E2 | 2.1E2 | 3.7E1 | 4.7E1 | 1.2E3 | 8.4E2 | 221 | 23 | 91 | 23 | 0.44 |
| cN | pg/mL | 1.2E2 | 1.1E2 | 1.2E2 | 1.4E2 | 4.4E1 | 7.0E1 | 3.8E1 | 5.7E1 | 2.7E2 | 3.5E2 | 221 | 23 | 91 | 23 | 0.54 |
| cO | pg/mL | 2.3E2 | 2.1E2 | 2.8E2 | 2.7E2 | 2.0E2 | 2.0E2 | 5.4E1 | 1.2E2 | 1.7E3 | 1.1E3 | 221 | 23 | 91 | 23 | 0.48 |
| cP | ng/mL | 2.4E3 | 2.9E3 | 2.5E3 | 3.0E3 | 8.9E2 | 8.8E2 | 6.2E2 | 1.4E3 | 5.7E3 | 5.1E3 | 221 | 23 | 91 | 23 | 0.67 |
| cQ | ng/mL | 4.0E-2 | 3.9E-2 | 1.1E-1 | 1.9E-1 | 1.8E-1 | 4.7E-1 | 2.0E-3 | 2.0E-3 | 1.2E0 | 2.2E0 | 221 | 23 | 91 | 23 | 0.53 |
| cR | ng/mL | 2.8E2 | 3.0E2 | 4.3E2 | 5.8E2 | 4.6E2 | 9.9E2 | 2.3E1 | 7.8E1 | 3.8E3 | 4.8E3 | 221 | 23 | 91 | 23 | 0.53 |
| cS | ng/mL | 2.4E2 | 3.6E2 | 3.9E2 | 4.1E2 | 4.2E2 | 2.5E2 | 5.3E1 | 5.0E1 | 2.7E3 | 9.0E2 | 221 | 23 | 91 | 23 | 0.59 |
| cT | ng/mL | 2.8E1 | 6.2E1 | 7.4E1 | 1.7E2 | 1.5E2 | 3.2E2 | 4.6E0 | 8.4E0 | 1.7E3 | 1.3E3 | 221 | 23 | 91 | 23 | 0.62 |
| cU | ng/mL | 5.5E1 | 4.8E1 | 7.4E1 | 6.0E1 | 7.2E1 | 4.2E1 | 1.0E1 | 9.0E0 | 7.7E2 | 1.5E2 | 221 | 23 | 91 | 23 | 0.45 |
| cV | ng/mL | 1.7E-1 | 1.8E-1 | 4.9E-1 | 6.2E-1 | 3.2E0 | 2.0E0 | 3.4E-4 | 3.0E-2 | 4.7E1 | 9.7E0 | 221 | 23 | 91 | 23 | 0.48 |
| cW | mIU/mL | 4.6E-2 | 7.7E-2 | 2.2E-1 | 8.5E-2 | 1.0E0 | 5.5E-2 | 3.7E-4 | 2.1E-2 | 9.7E0 | 2.1E-1 | 221 | 23 | 91 | 23 | 0.65 |
| cX | ng/mL | 1.1E-1 | 2.7E-1 | 1.8E0 | 2.4E0 | 5.4E0 | 6.9E0 | 2.3E-4 | 2.3E-4 | 2.8E1 | 2.8E1 | 221 | 23 | 91 | 23 | 0.50 |
| cY | ng/mL | 1.0E1 | 6.1E0 | 1.4E1 | 1.1E1 | 1.4E1 | 1.3E1 | 4.4E-1 | 1.3E0 | 8.3E1 | 5.3E1 | 221 | 23 | 91 | 23 | 0.37 |
| cZ | ug/mL | 1.4E1 | 1.7E1 | 1.5E1 | 1.6E1 | 5.8E0 | 7.4E0 | 5.3E0 | 6.6E0 | 3.9E1 | 4.2E1 | 221 | 23 | 91 | 23 | 0.54 |
| dA | pg/mL | 3.3E2 | 2.9E2 | 3.6E2 | 3.5E2 | 1.6E2 | 1.7E2 | 9.0E1 | 1.1E2 | 1.3E3 | 9.3E2 | 221 | 23 | 91 | 23 | 0.45 |
| dB | ug/mL | 1.7E1 | 2.1E1 | 1.9E1 | 2.1E1 | 2.1E1 | 8.5E0 | 1.9E0 | 2.8E0 | 2.5E2 | 4.0E1 | 221 | 23 | 91 | 23 | 0.66 |
| dC | nmol/L | 3.4E1 | 3.8E1 | 4.1E1 | 4.0E1 | 2.0E1 | 1.3E1 | 9.1E0 | 2.3E1 | 1.4E2 | 7.3E1 | 221 | 23 | 91 | 23 | 0.54 |
| dD | ug/mL | 3.7E1 | 3.2E1 | 3.8E1 | 3.4E1 | 1.0E1 | 1.0E1 | 1.3E1 | 1.6E1 | 7.6E1 | 5.1E1 | 221 | 23 | 91 | 23 | 0.40 |
| dE | ng/mL | 4.8E-1 | 4.2E-1 | 6.7E-1 | 4.2E-1 | 8.3E-1 | 3.9E-1 | 8.4E-3 | 8.4E-3 | 7.2E0 | 1.4E0 | 221 | 23 | 91 | 23 | 0.42 |
| dF | ng/mL | 2.1E2 | 2.4E2 | 2.6E2 | 3.2E2 | 1.8E2 | 2.6E2 | 7.5E1 | 8.7E1 | 1.2E3 | 1.2E3 | 221 | 23 | 91 | 23 | 0.56 |
| dG | ng/mL | 1.1E1 | 1.1E1 | 1.3E1 | 1.6E1 | 8.4E0 | 1.5E1 | 3.1E0 | 6.0E0 | 6.4E1 | 7.6E1 | 221 | 23 | 91 | 23 | 0.53 |
| dH | pg/mL | 7.5E0 | 5.8E0 | 1.2E1 | 1.0E1 | 2.4E1 | 1.1E1 | 4.0E-2 | 4.0E-2 | 3.1E2 | 4.4E1 | 221 | 23 | 91 | 23 | 0.47 |
| dI | pg/mL | 4.6E-1 | 4.6E-1 | 1.9E0 | 1.5E0 | 4.4E0 | 1.8E0 | 4.6E-1 | 4.6E-1 | 3.4E1 | 6.0E0 | 221 | 23 | 91 | 23 | 0.54 |
| dJ | ng/mL | 1.9E0 | 2.5E0 | 2.1E0 | 2.6E0 | 1.1E0 | 1.1E0 | 3.2E-2 | 1.1E0 | 5.9E0 | 4.9E0 | 221 | 23 | 91 | 23 | 0.63 |
| dK | uIU/mL | 2.0E0 | 1.7E0 | 2.7E0 | 2.7E0 | 2.9E0 | 3.8E0 | 2.8E-4 | 9.4E-2 | 1.6E1 | 1.5E1 | 221 | 23 | 91 | 23 | 0.46 |
| dL | ng/mL | 8.8E2 | 8.3E2 | 1.0E3 | 9.6E2 | 4.8E2 | 4.5E2 | 3.4E2 | 4.1E2 | 3.4E3 | 2.2E3 | 221 | 23 | 91 | 23 | 0.47 |
| dM | pg/mL | 9.7E2 | 8.5E2 | 1.2E3 | 1.2E3 | 1.1E3 | 8.9E2 | 4.4E2 | 3.9E2 | 1.2E4 | 3.6E3 | 221 | 23 | 91 | 23 | 0.44 |
| dN | ug/mL | 9.0E1 | 1.2E2 | 9.7E1 | 1.1E2 | 3.4E1 | 3.7E1 | 2.5E1 | 5.9E1 | 2.4E2 | 2.0E2 | 221 | 23 | 91 | 23 | 0.60 |
| dR | pg/ml | 1.6E3 | 1.6E3 | 2.5E3 | 2.1E3 | 2.6E3 | 2.1E3 | 1.9E2 | 1.4E2 | 1.5E4 | 8.6E3 | 131 | 21 | 88 | 21 | 0.45 |
| eF | ng/ml | 4.0E0 | 4.6E0 | 4.6E0 | 5.3E0 | 2.6E0 | 2.4E0 | 1.5E0 | 2.9E0 | 1.8E1 | 1.2E1 | 138 | 21 | 88 | 21 | 0.63 |
| eC | pg/ml | 3.1E2 | 3.0E2 | 3.8E2 | 3.1E2 | 2.5E2 | 1.5E2 | 4.5E1 | 1.3E2 | 1.4E3 | 7.1E2 | 100 | 21 | 86 | 21 | 0.43 |
| eD | pg/ml | 2.3E2 | 1.8E2 | 7.7E2 | 3.3E2 | 1.4E3 | 3.3E2 | 5.2E-1 | 5.9E1 | 6.8E3 | 1.1E3 | 77 | 21 | 64 | 21 | 0.46 |
| eT | ng/ml | 2.7E2 | 3.7E2 | 5.6E2 | 7.9E2 | 6.1E2 | 9.1E2 | 1.0E2 | 1.7E2 | 2.5E3 | 2.6E3 | 51 | 9 | 49 | 9 | 0.63 |
| eZ | ng/ml | 6.1E1 | 4.0E1 | 6.4E1 | 4.9E1 | 2.6E1 | 2.3E1 | 2.3E1 | 3.6E1 | 1.2E2 | 1.1E2 | 51 | 9 | 49 | 9 | 0.31 |
| fN | ng/ml | 2.1E-1 | 2.1E-1 | 2.7E0 | 1.4E0 | 5.8E0 | 2.7E0 | 2.1E-1 | 2.1E-1 | 3.2E1 | 8.1E0 | 51 | 9 | 49 | 9 | 0.44 |
| fP | ng/ml | 2.3E2 | 3.9E2 | 2.7E2 | 3.8E2 | 1.7E2 | 1.7E2 | 8.4E0 | 8.2E1 | 1.0E3 | 7.4E2 | 127 | 22 | 88 | 22 | 0.70 |
| fR | ng/ml | 1.3E5 | 1.2E5 | 1.6E5 | 2.6E5 | 1.1E5 | 2.4E5 | 3.1E4 | 5.5E4 | 6.1E5 | 7.2E5 | 162 | 9 | 44 | 9 | 0.58 |
| fY | ng/ml | 2.5E2 | 3.2E2 | 2.4E2 | 2.8E2 | 8.9E1 | 1.0E2 | 6.5E1 | 1.2E2 | 4.2E2 | 4.3E2 | 51 | 9 | 49 | 9 | 0.63 |
| gL | pg/ml | 6.1E4 | 7.2E4 | 6.8E4 | 7.4E4 | 2.9E4 | 3.0E4 | 2.2E4 | 3.3E4 | 1.8E5 | 1.4E5 | 131 | 21 | 88 | 21 | 0.57 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|--------|--------|--------|-----|
| | | NonDis | Dis | NonDis | Dis | NonDis | Dis | NonDis | Dis | NonDis | Dis | NonDis | Dis | NonDis | Dis | |
| gP | U/ml | 2.5E2 | 3.0E2 | 2.6E2 | 3.1E2 | 7.9E1 | 1.5E2 | 8.5E1 | 8.4E1 | 5.3E2 | 8.5E2 | 137 | 21 | 88 | 21 | 0.61 |
| gW | ng/ml | 7.0E2 | 5.4E2 | 1.5E3 | 7.9E2 | 1.9E3 | 8.3E2 | 3.7E1 | 2.3E0 | 9.5E3 | 3.1E3 | 109 | 20 | 79 | 20 | 0.41 |
| tF | pg/mL | 2.6E3 | 1.0E3 | 2.2E4 | 4.0E3 | 5.6E4 | 7.4E3 | 1.2E1 | 1.8E1 | 3.2E5 | 2.9E4 | 100 | 21 | 86 | 21 | 0.38 |
| hA | ng/ml | 2.3E0 | 1.6E0 | 7.4E0 | 7.6E0 | 2.4E1 | 1.4E1 | 1.7E-2 | 1.7E-2 | 1.6E2 | 5.7E1 | 77 | 21 | 64 | 21 | 0.44 |
| nM | pg/ml | 1.0E-9 | 1.0E-9 | 1.9E2 | 1.0E-9 | 1.4E3 | 0.0E0 | 1.0E-9 | 1.0E-9 | 1.0E4 | 1.0E-9 | 54 | 13 | 47 | 13 | 0.48 |
| nN | pg/ml | 1.1E3 | 8.5E2 | 2.1E3 | 1.6E3 | 2.7E3 | 2.0E3 | 1.1E2 | 1.6E2 | 1.7E4 | 7.1E3 | 54 | 13 | 47 | 13 | 0.44 |
| nO | pg/ml | 3.1E1 | 2.0E1 | 4.5E1 | 2.6E1 | 4.7E1 | 1.9E1 | 5.5E0 | 6.5E0 | 2.4E2 | 6.6E1 | 54 | 13 | 47 | 13 | 0.34 |
| nR | pg/ml | 1.6E1 | 2.2E1 | 4.5E1 | 6.6E1 | 7.3E1 | 7.7E1 | 1.0E-9 | 1.8E0 | 3.6E2 | 2.2E2 | 54 | 13 | 47 | 13 | 0.58 |
| nT | pg/ml | 8.5E1 | 4.0E1 | 3.5E2 | 6.2E1 | 1.2E3 | 6.8E1 | 1.0E-9 | 4.8E0 | 6.6E3 | 2.3E2 | 54 | 13 | 47 | 13 | 0.38 |
| nU | pg/ml | 2.9E1 | 6.0E1 | 5.1E2 | 6.7E1 | 2.2E3 | 7.0E1 | 1.0E-9 | 1.0E-9 | 1.3E4 | 2.6E2 | 54 | 13 | 47 | 13 | 0.62 |
| lW | pg/ml | 1.0E-9 | 1.0E-9 | 1.6E1 | 1.9E1 | 5.5E1 | 4.7E1 | 1.0E-9 | 1.0E-9 | 3.9E2 | 1.6E2 | 54 | 13 | 47 | 13 | 0.47 |
| lX | pg/ml | 9.5E2 | 1.1E3 | 9.2E2 | 1.2E3 | 4.4E2 | 5.5E2 | 2.3E2 | 3.3E2 | 1.9E3 | 2.2E3 | 54 | 13 | 47 | 13 | 0.64 |
| lY | pg/ml | 1.7E1 | 2.2E1 | 2.2E1 | 2.3E1 | 2.5E1 | 9.3E0 | 1.0E-9 | 4.2E0 | 1.4E2 | 3.8E1 | 54 | 13 | 47 | 13 | 0.65 |
| mE | pg/ml | 1.0E-9 | 1.0E-9 | 3.3E0 | 3.1E0 | 1.0E1 | 7.5E0 | 1.0E-9 | 1.0E-9 | 5.8E1 | 2.8E1 | 54 | 13 | 47 | 13 | 0.58 |
| mF | pg/ml | 1.0E-9 | 5.7E-1 | 1.4E0 | 1.5E0 | 3.0E0 | 2.3E0 | 1.0E-9 | 1.0E-9 | 1.5E1 | 6.5E0 | 54 | 13 | 47 | 13 | 0.62 |
| mH | pg/ml | 3.6E0 | 3.7E0 | 4.9E0 | 5.4E0 | 5.0E0 | 4.6E0 | 2.3E-1 | 2.0E0 | 3.2E1 | 1.8E1 | 54 | 13 | 47 | 13 | 0.57 |
| mI | pg/ml | 1.0E-9 | 1.0E-9 | 1.2E1 | 9.7E0 | 3.1E1 | 1.6E1 | 1.0E-9 | 1.0E-9 | 1.6E2 | 4.7E1 | 54 | 13 | 47 | 13 | 0.52 |
| mM | pg/ml | 3.0E1 | 3.1E1 | 5.2E1 | 1.0E2 | 5.9E1 | 1.8E2 | 1.0E-9 | 1.0E-9 | 2.3E2 | 6.5E2 | 54 | 13 | 47 | 13 | 0.56 |
| mP | pg/ml | 1.5E1 | 1.8E1 | 2.0E1 | 2.0E1 | 2.2E1 | 1.8E1 | 1.0E-9 | 1.0E-9 | 1.5E2 | 7.7E1 | 53 | 13 | 46 | 13 | 0.55 |
| mS | pg/ml | 1.8E3 | 2.0E3 | 2.2E3 | 2.2E3 | 2.2E3 | 6.0E2 | 1.0E-9 | 1.3E3 | 1.3E4 | 3.2E3 | 54 | 13 | 47 | 13 | 0.61 |
| mT | pg/ml | 5.0E1 | 7.9E1 | 1.2E2 | 1.8E2 | 2.2E2 | 2.0E2 | 1.0E1 | 1.7E1 | 1.4E3 | 6.2E2 | 53 | 13 | 46 | 13 | 0.58 |
| mU | pg/ml | 2.5E0 | 2.0E0 | 4.2E0 | 4.4E0 | 8.5E0 | 5.8E0 | 1.0E-9 | 8.2E-1 | 5.8E1 | 2.2E1 | 53 | 13 | 46 | 13 | 0.51 |
| mW | pg/ml | 2.5E3 | 2.6E3 | 2.7E3 | 3.1E3 | 1.7E3 | 2.2E3 | 4.3E2 | 8.9E2 | 1.0E4 | 9.8E3 | 53 | 13 | 46 | 13 | 0.55 |
| mY | pg/ml | 4.7E2 | 8.9E2 | 1.0E3 | 1.2E3 | 1.8E3 | 6.7E2 | 1.0E-9 | 6.8E2 | 1.1E4 | 2.6E3 | 54 | 13 | 47 | 13 | 0.77 |
| mZ | pg/ml | 2.6E2 | 1.1E2 | 5.0E2 | 2.1E2 | 6.0E2 | 2.0E2 | 2.1E2 | 2.1E0 | 3.1E3 | 6.9E2 | 53 | 13 | 46 | 13 | 0.30 |
| nA | pg/ml | 1.3E0 | 5.0E0 | 1.6E1 | 9.4E0 | 6.6E1 | 1.4E1 | 1.0E-9 | 1.0E-9 | 4.4E2 | 5.4E1 | 53 | 13 | 46 | 13 | 0.67 |
| nB | pg/ml | 2.6E2 | 4.3E2 | 3.1E2 | 4.4E2 | 1.6E2 | 1.7E2 | 3.0E1 | 1.5E2 | 7.2E2 | 7.8E2 | 54 | 13 | 47 | 13 | 0.72 |
| nC | pg/ml | 1.0E-9 | 8.3E1 | 8.7E3 | 6.7E2 | 5.0E4 | 2.0E3 | 1.0E-9 | 1.0E-9 | 3.7E5 | 7.2E3 | 54 | 13 | 47 | 13 | 0.54 |
| nD | pg/ml | 7.2E0 | 1.2E1 | 7.0E1 | 1.3E1 | 3.1E2 | 8.5E0 | 1.0E-9 | 1.0E-9 | 1.7E3 | 2.6E1 | 53 | 13 | 46 | 13 | 0.63 |
| nF | pg/ml | 1.0E-9 | 1.0E-9 | 9.5E0 | 8.5E-1 | 4.0E1 | 3.1E0 | 1.0E-9 | 1.0E-9 | 2.4E2 | 1.1E1 | 54 | 13 | 47 | 13 | 0.48 |
| nH | pg/ml | 3.0E-2 | 5.4E0 | 2.1E2 | 1.4E1 | 1.4E3 | 3.1E1 | 1.0E-9 | 1.0E-9 | 1.0E4 | 1.2E2 | 53 | 13 | 46 | 13 | 0.68 |
| nI | pg/ml | 4.6E1 | 3.0E1 | 2.9E2 | 4.8E1 | 1.3E3 | 5.5E1 | 1.0E-9 | 1.0E-9 | 9.2E3 | 1.7E2 | 54 | 13 | 47 | 13 | 0.45 |
| nJ | pg/ml | 1.7E-1 | 1.1E0 | 9.9E1 | 1.5E0 | 7.0E2 | 1.6E0 | 1.0E-9 | 1.0E-9 | 5.2E3 | 6.3E0 | 54 | 13 | 47 | 13 | 0.69 |
| nK | pg/ml | 1.0E-9 | 3.0E1 | 1.2E2 | 2.7E1 | 5.4E2 | 3.1E1 | 1.0E-9 | 1.0E-9 | 3.2E3 | 9.6E1 | 53 | 13 | 46 | 13 | 0.65 |
| nL | pg/ml | 1.0E-9 | 2.5E0 | 9.3E2 | 2.0E1 | 6.1E3 | 3.9E1 | 1.0E-9 | 1.0E-9 | 4.5E4 | 1.4E2 | 54 | 13 | 47 | 13 | 0.56 |
| hL | pg/ml | 1.7E4 | 1.6E4 | 2.3E4 | 2.3E4 | 2.2E4 | 2.0E4 | 2.6E3 | 5.4E3 | 1.4E5 | 7.0E4 | 51 | 9 | 49 | 9 | 0.49 |
| hO | pg/ml | 1.6E4 | 1.5E4 | 1.7E4 | 1.5E4 | 3.2E3 | 1.8E3 | 1.1E4 | 1.3E4 | 2.8E4 | 1.8E4 | 51 | 9 | 49 | 9 | 0.42 |
| hP | ng/ml | 4.4E5 | 4.4E5 | 3.7E5 | 5.9E5 | 1.7E5 | 5.9E5 | 2.8E4 | 3.4E4 | 9.0E5 | 2.0E6 | 51 | 9 | 49 | 9 | 0.59 |
| wJ | pg/ml | 1.5E5 | 1.8E5 | 1.6E5 | 1.9E5 | 8.7E4 | 1.2E5 | 2.8E4 | 3.3E4 | 4.0E5 | 4.0E5 | 48 | 9 | 44 | 9 | 0.56 |
| wK | pg/ml | 3.4E4 | 5.4E4 | 4.3E4 | 9.9E4 | 2.7E4 | 1.6E5 | 5.2E3 | 8.1E3 | 1.2E5 | 5.0E5 | 48 | 9 | 44 | 9 | 0.60 |
| wL | pg/ml | 6.5E0 | 3.9E0 | 7.0E1 | 8.0E0 | 1.5E2 | 9.7E0 | 1.0E-9 | 1.0E-9 | 8.4E2 | 2.8E1 | 48 | 9 | 44 | 9 | 0.38 |
| wP | pg/ml | 2.8E4 | 4.3E4 | 4.3E4 | 4.9E4 | 4.3E4 | 4.0E4 | 2.8E3 | 6.3E3 | 1.6E5 | 1.4E5 | 48 | 9 | 44 | 9 | 0.59 |
| wQ | pg/ml | 3.4E1 | 6.7E1 | 6.0E1 | 7.3E1 | 7.7E1 | 6.1E1 | 1.0E-9 | 3.6E0 | 3.7E2 | 1.7E2 | 48 | 9 | 44 | 9 | 0.62 |
| hR | pg/ml | 2.7E4 | 2.2E4 | 3.0E4 | 2.5E4 | 1.1E4 | 1.0E4 | 1.8E3 | 1.1E1 | 5.8E4 | 4.3E4 | 75 | 19 | 62 | 19 | 0.37 |
| hV | pg/ml | 4.4E2 | 4.5E2 | 4.7E2 | 4.7E2 | 2.4E2 | 1.8E2 | 1.3E2 | 2.2E2 | 1.5E3 | 8.1E2 | 75 | 19 | 62 | 19 | 0.53 |
| hW | pg/ml | 1.6E3 | 1.8E3 | 2.1E3 | 2.2E3 | 1.6E3 | 1.6E3 | 5.7E2 | 1.1E3 | 1.0E4 | 7.7E3 | 75 | 19 | 62 | 19 | 0.54 |
| hX | pg/ml | 9.6E2 | 1.1E3 | 1.2E3 | 1.1E3 | 1.1E3 | 4.2E2 | 4.8E2 | 5.2E2 | 8.6E3 | 2.2E3 | 75 | 19 | 62 | 19 | 0.56 |
| iA | pg/ml | 1.7E2 | 1.4E2 | 4.2E2 | 2.0E2 | 9.2E2 | 1.9E2 | 1.8E1 | 3.0E1 | 7.1E3 | 9.5E2 | 100 | 21 | 86 | 21 | 0.46 |
| iB | ng/ml | 5.4E0 | 4.3E0 | 6.7E0 | 6.0E0 | 4.7E0 | 5.2E0 | 2.5E-1 | 3.3E-2 | 2.0E1 | 2.1E1 | 77 | 21 | 64 | 21 | 0.42 |
| iC | U/ml | 2.4E-1 | 1.8E-1 | 5.0E-1 | 3.0E0 | 8.7E-1 | 1.2E1 | 1.0E-9 | 1.0E-9 | 6.4E0 | 5.5E1 | 77 | 21 | 64 | 21 | 0.54 |
| tQ | pg/ml | 1.1E3 | 1.1E3 | 1.2E3 | 1.3E3 | 5.5E2 | 7.1E2 | 2.8E2 | 5.0E2 | 2.5E3 | 2.5E3 | 46 | 8 | 43 | 8 | 0.52 |
| tT | pg/ml | 1.5E1 | 2.1E1 | 1.7E1 | 1.8E1 | 7.4E0 | 9.8E0 | 7.4E0 | 5.4E0 | 3.9E1 | 3.3E1 | 46 | 8 | 43 | 8 | 0.56 |
| tS | pg/ml | 1.0E0 | 6.1E-1 | 1.1E0 | 9.8E-1 | 8.6E-1 | 1.1E0 | 1.0E-9 | 1.0E-9 | 4.0E0 | 3.2E0 | 46 | 8 | 43 | 8 | 0.46 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| tX | pg/ml | 8.1E-1 | 5.2E-1 | 9.5E-1 | 7.4E-1 | 7.0E-1 | 6.1E-1 | 2.5E-2 | 1.6E-1 | 3.3E0 | 1.9E0 | 46 | 8 | 43 | 8 | 0.40 |
| tO | pg/ml | 4.0E0 | 4.4E0 | 4.4E0 | 4.7E0 | 2.5E0 | 2.9E0 | 1.0E-9 | 1.7E0 | 1.1E1 | 1.1E1 | 46 | 8 | 43 | 8 | 0.52 |
| tR | pg/ml | 2.1E-1 | 1.9E-1 | 2.5E-1 | 2.4E-1 | 2.1E-1 | 2.1E-1 | 1.0E-9 | 5.5E-2 | 9.1E-1 | 7.1E-1 | 46 | 8 | 43 | 8 | 0.47 |
| tU | pg/ml | 9.0E0 | 8.8E0 | 1.1E1 | 8.6E0 | 6.9E0 | 4.2E0 | 1.6E0 | 3.0E0 | 3.1E1 | 1.7E1 | 46 | 9 | 43 | 9 | 0.43 |
| tN | pg/ml | 1.7E1 | 1.5E1 | 1.9E1 | 1.6E1 | 1.0E1 | 9.2E0 | 1.0E-9 | 1.0E-9 | 5.4E1 | 2.8E1 | 46 | 9 | 43 | 9 | 0.43 |
| tV | ng/ml | 3.7E2 | 8.1E2 | 4.8E2 | 7.5E2 | 4.0E2 | 5.1E2 | 6.6E1 | 1.4E2 | 2.6E3 | 1.8E3 | 48 | 9 | 44 | 9 | 0.67 |
| iH | ng/ml | 1.5E5 | 1.9E5 | 1.5E5 | 1.9E5 | 4.3E4 | 5.0E4 | 7.1E4 | 5.1E4 | 2.4E5 | 2.7E5 | 100 | 21 | 86 | 21 | 0.75 |
| iJ | ng/ml | 5.1E4 | 5.1E4 | 5.3E4 | 5.9E4 | 2.0E4 | 3.3E4 | 8.7E3 | 8.6E3 | 1.2E5 | 1.5E5 | 100 | 21 | 86 | 21 | 0.54 |
| hB | ng/ml | 4.3E-1 | 4.8E-1 | 5.2E-1 | 6.6E-1 | 3.5E-1 | 5.5E-1 | 1.2E-1 | 2.5E-1 | 1.9E0 | 2.4E0 | 100 | 21 | 86 | 21 | 0.58 |
| hC | pg/ml | 3.6E3 | 4.2E3 | 5.9E3 | 8.2E3 | 7.5E3 | 1.0E4 | 1.0E-9 | 3.3E2 | 5.5E4 | 4.3E4 | 100 | 21 | 86 | 21 | 0.57 |
| hF | ng/ml | 1.0E-9 | 1.0E-9 | 4.9E1 | 1.0E-9 | 4.1E2 | 0.0E0 | 1.0E-9 | 1.0E-9 | 4.0E3 | 1.0E-9 | 100 | 21 | 86 | 21 | 0.49 |
| hG | pg/ml | 7.4E3 | 6.9E3 | 7.8E3 | 8.1E3 | 2.9E3 | 3.1E3 | 1.7E3 | 4.7E3 | 1.8E4 | 1.8E4 | 100 | 21 | 86 | 21 | 0.52 |
| iO | ng/ml | 4.1E5 | 4.0E5 | 4.2E5 | 3.9E5 | 2.0E5 | 1.8E5 | 1.1E5 | 6.4E4 | 1.1E6 | 8.6E5 | 100 | 21 | 86 | 21 | 0.45 |
| iP | ng/ml | 6.1E4 | 4.6E4 | 5.5E4 | 5.6E4 | 3.1E4 | 3.6E4 | 5.8E3 | 1.3E4 | 2.5E5 | 1.9E5 | 100 | 21 | 86 | 21 | 0.47 |
| iZ | ng/ml | 1.7E3 | 1.7E3 | 1.8E3 | 1.8E3 | 6.8E2 | 8.8E2 | 4.7E2 | 6.5E2 | 3.5E3 | 4.5E3 | 100 | 21 | 86 | 21 | 0.48 |
| yH | pg/ml | 1.2E3 | 7.5E2 | 2.0E3 | 9.8E2 | 3.0E3 | 5.8E2 | 1.0E-9 | 1.6E2 | 1.5E4 | 1.9E3 | 48 | 9 | 44 | 9 | 0.41 |
| yK | U/ml | 1.8E1 | 2.3E1 | 4.9E1 | 4.0E1 | 8.5E1 | 3.9E1 | 1.0E-9 | 1.0E-9 | 3.8E2 | 1.0E2 | 48 | 9 | 44 | 9 | 0.53 |
| yJ | pg/ml | 3.6E4 | 3.2E4 | 4.7E4 | 4.4E4 | 3.5E4 | 4.3E4 | 1.7E3 | 7.1E3 | 1.6E5 | 1.4E5 | 48 | 9 | 44 | 9 | 0.44 |
| yD | ng/ml | 1.5E-2 | 1.7E-2 | 1.5E-2 | 1.6E-2 | 5.8E-3 | 8.3E-3 | 1.0E-9 | 5.4E-3 | 2.9E-2 | 3.2E-2 | 48 | 9 | 44 | 9 | 0.53 |
| wB | pg/ml | 8.8E3 | 8.3E3 | 9.8E3 | 8.7E3 | 7.3E3 | 5.4E3 | 1.7E3 | 2.7E3 | 4.1E4 | 2.1E4 | 48 | 9 | 44 | 9 | 0.47 |
| pY | pg/ml | 6.0E0 | 7.1E0 | 1.1E1 | 7.5E0 | 2.7E1 | 2.9E0 | 2.1E0 | 3.4E0 | 2.0E2 | 1.2E1 | 51 | 9 | 49 | 9 | 0.60 |
| rC | pg/ml | 1.9E3 | 1.4E3 | 2.6E3 | 1.9E3 | 2.8E3 | 1.4E3 | 1.0E2 | 3.2E2 | 1.5E4 | 4.9E3 | 72 | 18 | 59 | 18 | 0.43 |
| rB | pg/ml | 2.2E1 | 3.0E1 | 5.5E1 | 3.4E1 | 1.4E2 | 3.0E1 | 1.0E-9 | 1.0E-9 | 9.5E2 | 1.3E2 | 72 | 18 | 59 | 18 | 0.59 |
| zG | 2.5ng/ml | 2.0E-1 | 2.5E-1 | 4.5E-1 | 7.8E-1 | 7.8E-1 | 1.5E0 | 1.0E-9 | 5.1E-2 | 4.4E0 | 4.8E0 | 48 | 9 | 44 | 9 | 0.57 |
| zH | 2.3mU/ml | 1.1E-1 | 9.9E-2 | 1.2E-1 | 1.0E-1 | 6.9E-2 | 3.5E-2 | 1.0E-2 | 4.0E-2 | 4.4E-1 | 1.6E-1 | 48 | 9 | 44 | 9 | 0.48 |
| zI | 2.6ng/ml | 1.8E0 | 2.6E0 | 3.2E0 | 4.8E0 | 3.3E0 | 5.0E0 | 6.1E-1 | 1.5E0 | 1.5E1 | 1.4E1 | 48 | 9 | 44 | 9 | 0.66 |
| qA | ng/ml | 1.0E7 | 7.4E6 | 1.1E7 | 1.6E7 | 7.3E6 | 1.2E7 | 3.7E6 | 6.9E6 | 3.7E7 | 3.9E7 | 51 | 9 | 49 | 9 | 0.57 |
| qB | ng/ml | 6.3E5 | 4.8E5 | 8.3E5 | 9.8E5 | 5.8E5 | 9.6E5 | 2.1E5 | 2.7E5 | 2.9E6 | 2.9E6 | 51 | 9 | 49 | 9 | 0.46 |
| qC | ng/ml | 4.8E5 | 3.7E5 | 8.2E5 | 3.5E5 | 1.2E6 | 2.8E5 | 2.0E4 | 2.5E4 | 7.1E6 | 7.9E5 | 51 | 9 | 49 | 9 | 0.37 |
| qD | ng/ml | 1.6E7 | 1.3E7 | 1.9E7 | 1.6E7 | 9.4E6 | 1.1E7 | 4.9E6 | 6.2E6 | 5.2E7 | 4.2E7 | 51 | 9 | 49 | 9 | 0.36 |
| jD | ng/ml | 2.2E1 | 4.9E1 | 4.4E1 | 5.7E1 | 7.2E1 | 5.0E1 | 1.0E-9 | 1.0E-9 | 5.1E2 | 1.9E2 | 77 | 21 | 64 | 21 | 0.64 |
| jE | ng/ml | 1.0E-9 | 1.0E-9 | 8.0E0 | 3.9E0 | 2.1E1 | 8.6E0 | 1.0E-9 | 1.0E-9 | 1.2E2 | 2.9E1 | 77 | 21 | 64 | 21 | 0.48 |
| jF | ng/ml | 3.5E1 | 2.9E1 | 5.0E1 | 4.0E1 | 5.8E1 | 4.7E1 | 1.0E-9 | 1.0E-9 | 2.5E2 | 1.5E2 | 77 | 21 | 64 | 21 | 0.46 |
| jG | ng/ml | 4.5E3 | 4.3E3 | 4.6E3 | 4.4E3 | 1.9E3 | 2.1E3 | 7.6E2 | 1.5E3 | 9.5E3 | 8.6E3 | 77 | 21 | 64 | 21 | 0.47 |
| jH | ng/ml | 7.9E1 | 7.3E1 | 8.6E1 | 8.2E1 | 4.5E1 | 5.0E1 | 1.9E1 | 1.3E1 | 2.3E2 | 2.5E2 | 77 | 21 | 64 | 21 | 0.45 |
| jI | ng/ml | 6.9E1 | 7.8E1 | 7.4E1 | 8.2E1 | 3.4E1 | 3.1E1 | 2.8E1 | 4.0E1 | 2.5E2 | 1.9E2 | 77 | 21 | 64 | 21 | 0.61 |
| sK | pg/mL | 4.2E3 | 4.1E3 | 4.3E3 | 3.8E3 | 1.6E3 | 1.5E3 | 1.7E3 | 1.9E3 | 9.2E3 | 6.2E3 | 49 | 8 | 45 | 8 | 0.44 |
| sM | pg/mL | 8.0E4 | 8.5E4 | 7.9E4 | 7.8E4 | 2.4E4 | 2.5E4 | 3.3E4 | 4.3E4 | 1.5E5 | 1.1E5 | 49 | 8 | 45 | 8 | 0.53 |
| sO | pg/mL | 2.8E8 | 2.6E8 | 2.9E8 | 2.5E8 | 9.9E7 | 7.1E7 | 7.9E7 | 1.4E8 | 4.9E8 | 3.3E8 | 49 | 8 | 45 | 8 | 0.38 |
| wC | ng/ml | 1.6E0 | 1.5E0 | 2.1E0 | 1.8E0 | 2.2E0 | 1.2E0 | 2.5E-1 | 5.7E-1 | 1.5E1 | 4.2E0 | 48 | 9 | 44 | 9 | 0.47 |
| wD | ng/ml | 2.0E1 | 1.7E1 | 8.1E1 | 2.0E1 | 3.1E2 | 1.5E1 | 2.8E0 | 5.1E0 | 2.1E3 | 5.2E1 | 48 | 9 | 44 | 9 | 0.48 |
| wE | ng/ml | 5.0E1 | 4.3E1 | 5.4E1 | 4.2E1 | 2.4E1 | 2.5E1 | 7.0E0 | 4.0E0 | 1.4E2 | 8.9E1 | 48 | 9 | 44 | 9 | 0.36 |
| wG | ng/ml | 9.6E-2 | 1.2E-2 | 1.3E-1 | 6.5E-2 | 1.3E-1 | 1.1E-1 | 1.0E-9 | 1.0E-9 | 4.8E-1 | 3.3E-1 | 48 | 9 | 44 | 9 | 0.38 |
| wH | ng/ml | 2.3E-2 | 6.0E-3 | 2.1E-1 | 8.1E-2 | 5.5E-1 | 1.5E-1 | 1.0E-9 | 1.0E-9 | 2.9E0 | 4.0E-1 | 48 | 9 | 44 | 9 | 0.37 |
| wF | ng/ml | 2.1E-1 | 4.2E-2 | 2.9E0 | 2.7E-1 | 1.0E1 | 3.6E-1 | 1.0E-9 | 1.0E-9 | 5.7E1 | 1.1E0 | 48 | 9 | 44 | 9 | 0.44 |
| rA | pg/ml | 2.4E1 | 2.7E1 | 2.7E1 | 3.4E1 | 2.2E1 | 2.4E1 | 1.0E-9 | 1.0E-9 | 1.2E2 | 8.2E1 | 74 | 20 | 61 | 20 | 0.59 |
| qZ | pg/ml | 4.3E1 | 8.3E1 | 2.1E2 | 9.6E2 | 1.3E3 | 3.0E3 | 2.8E-4 | 7.2E-4 | 1.0E4 | 1.0E4 | 63 | 11 | 54 | 11 | 0.59 |
| qY | pg/ml | 2.1E1 | 1.4E1 | 4.6E1 | 3.6E1 | 7.5E1 | 4.3E1 | 8.7E-1 | 2.2E0 | 5.3E2 | 1.6E2 | 74 | 20 | 61 | 20 | 0.46 |
| qX | pg/ml | 5.1E1 | 7.2E1 | 5.9E1 | 8.6E1 | 3.9E1 | 6.3E1 | 1.0E-9 | 1.0E-9 | 1.8E2 | 2.1E2 | 74 | 20 | 61 | 20 | 0.62 |
| qW | pg/ml | 9.4E0 | 7.0E0 | 1.2E1 | 9.7E0 | 1.3E1 | 7.6E0 | 1.0E-9 | 1.0E-9 | 7.1E1 | 2.3E1 | 74 | 20 | 61 | 20 | 0.46 |
| qV | pg/ml | 2.1E3 | 1.4E3 | 2.7E3 | 2.0E3 | 1.9E3 | 1.7E3 | 2.3E2 | 1.7E2 | 8.5E3 | 6.4E3 | 74 | 20 | 61 | 20 | 0.38 |
| qU | pg/ml | 4.5E1 | 7.6E1 | 1.3E2 | 1.0E2 | 2.2E2 | 8.3E1 | 1.0E-9 | 1.0E1 | 1.4E3 | 2.8E2 | 74 | 20 | 61 | 20 | 0.58 |
| qT | pg/ml | 3.8E1 | 5.7E1 | 6.4E1 | 8.5E1 | 1.2E2 | 9.4E1 | 1.0E-9 | 5.6E0 | 9.0E2 | 4.4E2 | 74 | 20 | 61 | 20 | 0.64 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| qI | ng/ml | 5.8E4 | 7.2E4 | 6.4E4 | 6.5E4 | 3.2E4 | 2.5E4 | 5.4E3 | 3.0E4 | 1.6E5 | 9.8E4 | 48 | 8 | 46 | 8 | 0.55 |
| qH | ng/ml | 6.5E4 | 5.6E4 | 7.3E4 | 6.5E4 | 3.9E4 | 4.5E4 | 1.0E4 | 2.3E4 | 1.8E5 | 1.6E5 | 48 | 8 | 46 | 8 | 0.39 |
| qG | ng/ml | 1.8E5 | 2.2E5 | 1.9E5 | 2.2E5 | 6.2E4 | 5.8E4 | 3.1E4 | 1.5E5 | 3.3E5 | 2.9E5 | 48 | 8 | 46 | 8 | 0.62 |
| jK | ng/ml | 1.6E3 | 1.6E3 | 1.7E3 | 1.7E3 | 5.7E2 | 5.0E2 | 5.5E2 | 7.6E2 | 4.1E3 | 2.8E3 | 77 | 21 | 64 | 21 | 0.51 |
| jL | ng/ml | 1.8E2 | 2.1E2 | 2.6E2 | 3.5E2 | 1.9E2 | 3.7E2 | 3.6E1 | 8.8E1 | 7.9E2 | 1.7E3 | 77 | 21 | 64 | 21 | 0.56 |
| jM | ng/ml | 7.1E4 | 6.8E4 | 7.0E4 | 7.3E4 | 3.4E4 | 3.4E4 | 3.9E2 | 2.4E4 | 1.5E5 | 1.5E5 | 77 | 21 | 64 | 21 | 0.50 |
| jO | pg/ml | 2.2E5 | 2.1E5 | 2.8E5 | 2.6E5 | 1.5E5 | 1.4E5 | 5.2E4 | 7.4E4 | 7.7E5 | 6.2E5 | 77 | 21 | 64 | 21 | 0.46 |
| jP | pg/ml | 2.3E5 | 2.4E5 | 2.6E5 | 2.7E5 | 1.5E5 | 1.9E5 | 7.0E4 | 6.6E4 | 9.1E5 | 9.2E5 | 77 | 21 | 64 | 21 | 0.48 |
| jQ | pg/ml | 2.5E3 | 3.3E3 | 3.0E3 | 3.9E3 | 2.3E3 | 3.0E3 | 4.2E1 | 2.6E2 | 1.2E4 | 1.1E4 | 77 | 21 | 64 | 21 | 0.59 |
| jR | pg/ml | 6.5E3 | 7.9E3 | 9.5E3 | 1.1E4 | 9.9E3 | 1.0E4 | 1.0E-9 | 7.2E1 | 5.5E4 | 3.4E4 | 77 | 21 | 64 | 21 | 0.55 |
| jT | pg/ml | 1.6E5 | 1.7E5 | 1.7E5 | 1.8E5 | 6.9E4 | 5.6E4 | 6.8E4 | 9.0E4 | 4.5E5 | 2.8E5 | 77 | 21 | 64 | 21 | 0.58 |
| xA | pg/ml | 3.9E0 | 1.8E1 | 1.7E1 | 2.9E1 | 5.7E1 | 4.6E1 | 1.0E-9 | 1.0E-9 | 3.9E2 | 1.4E2 | 48 | 9 | 44 | 9 | 0.62 |
| yE | pg/ml | 7.8E1 | 6.6E1 | 8.2E1 | 7.9E1 | 4.9E1 | 3.7E1 | 6.4E0 | 4.0E1 | 3.0E2 | 1.6E2 | 48 | 9 | 44 | 9 | 0.47 |
| tM | pg/ml | 4.3E1 | 3.3E1 | 4.3E1 | 3.5E1 | 2.0E1 | 1.9E1 | 1.0E-9 | 1.0E-9 | 1.0E2 | 6.1E1 | 48 | 9 | 44 | 9 | 0.37 |
| tL | pg/ml | 1.0E-9 | 1.0E-9 | 7.8E0 | 3.0E-1 | 3.8E1 | 6.0E-1 | 1.0E-9 | 1.0E-9 | 2.6E2 | 1.4E0 | 48 | 9 | 44 | 9 | 0.48 |
| jU | mIU/ml | 3.8E0 | 6.7E0 | 1.0E1 | 1.4E1 | 1.7E1 | 1.9E1 | 8.9E-2 | 6.3E-2 | 8.1E1 | 7.5E1 | 77 | 21 | 64 | 21 | 0.63 |
| jV | mIU/ml | 1.6E0 | 2.2E0 | 3.5E0 | 5.4E0 | 5.4E0 | 8.5E0 | 2.1E-2 | 2.7E-3 | 3.1E1 | 3.3E1 | 77 | 21 | 64 | 21 | 0.52 |
| jY | ng/ml | 9.7E-4 | 4.3E-4 | 1.1E-2 | 7.3E-3 | 4.2E-2 | 2.1E-2 | 1.0E-9 | 1.0E-9 | 3.0E-1 | 9.4E-2 | 77 | 21 | 64 | 21 | 0.46 |
| kC | pg/ml | 9.7E1 | 1.2E2 | 2.4E2 | 1.1E2 | 5.6E2 | 5.7E1 | 2.1E1 | 3.6E1 | 3.5E3 | 2.1E2 | 54 | 13 | 47 | 13 | 0.47 |
| kE | pg/ml | 1.3E5 | 1.4E5 | 1.3E5 | 1.3E5 | 3.3E4 | 4.2E4 | 4.1E4 | 3.8E4 | 2.0E5 | 2.0E5 | 54 | 13 | 47 | 13 | 0.50 |
| kF | pg/mL | 6.6E1 | 5.6E1 | 8.0E1 | 6.5E1 | 7.0E1 | 1.8E1 | 3.2E1 | 4.0E1 | 5.1E2 | 9.2E1 | 54 | 13 | 47 | 13 | 0.44 |
| kG | pg/mL | 9.1E3 | 1.1E4 | 1.2E4 | 2.1E4 | 1.4E4 | 3.1E4 | 7.5E2 | 3.8E3 | 9.1E4 | 1.2E5 | 54 | 13 | 47 | 13 | 0.57 |
| kI | pg/ml | 2.2E2 | 1.8E2 | 2.4E2 | 2.3E2 | 1.4E2 | 1.4E2 | 5.4E1 | 9.2E1 | 8.7E2 | 5.5E2 | 54 | 13 | 47 | 13 | 0.40 |
| kK | pg/ml | 1.2E2 | 1.2E2 | 1.7E2 | 1.6E2 | 1.8E2 | 1.3E2 | 2.2E1 | 4.5E1 | 1.2E3 | 4.9E2 | 54 | 13 | 47 | 13 | 0.49 |
| kN | pg/ml | 1.0E3 | 1.2E3 | 1.5E3 | 1.7E3 | 1.9E3 | 1.2E3 | 2.1E2 | 4.4E2 | 1.3E4 | 3.9E3 | 54 | 13 | 47 | 13 | 0.62 |
| kO | pg/ml | 7.7E3 | 6.2E3 | 1.1E4 | 6.5E3 | 1.8E4 | 1.9E3 | 4.0E3 | 3.8E3 | 1.3E5 | 1.0E4 | 54 | 13 | 47 | 13 | 0.30 |
| kP | pg/ml | 5.4E3 | 7.0E3 | 6.7E3 | 6.9E3 | 5.5E3 | 2.6E3 | 8.6E2 | 2.7E3 | 3.3E4 | 1.2E4 | 54 | 13 | 47 | 13 | 0.60 |
| kQ | pg/ml | 4.3E3 | 3.8E3 | 4.9E3 | 4.4E3 | 2.3E3 | 2.1E3 | 5.6E2 | 1.6E3 | 1.2E4 | 9.3E3 | 100 | 21 | 86 | 21 | 0.42 |
| kR | pg/ml | 2.4E1 | 2.0E1 | 4.0E1 | 2.5E1 | 1.0E2 | 1.8E1 | 1.0E-9 | 5.1E0 | 1.0E3 | 6.9E1 | 100 | 21 | 86 | 21 | 0.44 |
| kS | pg/ml | 7.7E2 | 8.2E2 | 9.9E2 | 8.4E2 | 1.4E3 | 4.0E2 | 8.2E1 | 2.5E2 | 1.4E4 | 1.7E3 | 100 | 21 | 86 | 21 | 0.52 |
| pS | ng/ml | 2.0E5 | 2.1E5 | 2.2E5 | 2.4E5 | 9.5E4 | 1.4E5 | 7.5E4 | 1.0E5 | 5.0E5 | 5.7E5 | 49 | 8 | 45 | 8 | 0.55 |
| rZ | ng/ml | 1.2E-3 | 1.0E-9 | 7.9E-3 | 2.6E-3 | 2.1E-2 | 3.6E-3 | 1.0E-9 | 1.0E-9 | 1.1E-1 | 9.1E-3 | 73 | 18 | 58 | 18 | 0.46 |
| rY | ng/ml | 5.4E-2 | 4.7E-2 | 2.5E-1 | 6.7E-2 | 8.3E-1 | 1.1E-1 | 1.0E-9 | 1.0E-9 | 6.3E0 | 5.0E-1 | 73 | 18 | 58 | 18 | 0.38 |
| rX | ng/ml | 1.0E-9 | 1.0E-9 | 9.2E-2 | 1.7E-2 | 4.5E-1 | 6.7E-2 | 1.0E-9 | 1.0E-9 | 3.7E0 | 2.9E-1 | 73 | 18 | 58 | 18 | 0.42 |
| lK | pg/ml | 7.1E1 | 1.1E2 | 1.4E2 | 2.0E2 | 1.8E2 | 3.2E2 | 1.0E-9 | 4.1E0 | 7.0E2 | 1.3E3 | 77 | 21 | 64 | 21 | 0.53 |
| lL | pg/ml | 1.8E3 | 1.4E3 | 3.4E3 | 2.0E3 | 5.5E3 | 1.9E3 | 7.5E1 | 4.5E2 | 4.2E4 | 6.8E3 | 77 | 21 | 64 | 21 | 0.40 |
| lM | pg/ml | 1.0E3 | 1.5E3 | 3.3E3 | 6.7E3 | 7.5E3 | 1.2E4 | 3.9E1 | 2.7E2 | 5.1E4 | 4.4E4 | 77 | 21 | 64 | 21 | 0.58 |
| lN | pg/ml | 1.0E-9 | 1.0E-9 | 7.3E0 | 1.2E0 | 2.2E1 | 2.1E0 | 1.0E-9 | 1.0E-9 | 1.5E2 | 5.0E0 | 77 | 21 | 64 | 21 | 0.40 |
| lO | pg/ml | 1.0E-9 | 1.0E-9 | 5.2E-1 | 6.3E-1 | 4.6E0 | 2.9E0 | 1.0E-9 | 1.0E-9 | 4.0E1 | 1.3E1 | 77 | 21 | 64 | 21 | 0.52 |
| zA | ng/ml | 1.9E7 | 1.9E7 | 2.0E7 | 2.1E7 | 6.8E6 | 6.0E6 | 6.7E6 | 1.1E7 | 3.6E7 | 3.3E7 | 44 | 9 | 40 | 9 | 0.54 |
| rW | ng/ml | 1.3E-2 | 6.5E-3 | 2.4E-2 | 3.4E-2 | 3.1E-2 | 5.5E-2 | 1.0E-9 | 1.0E-9 | 1.7E-1 | 1.6E-1 | 49 | 9 | 46 | 9 | 0.50 |
| rV | ng/ml | 1.0E-9 | 1.0E-9 | 9.2E-3 | 3.2E-2 | 3.7E-2 | 9.4E-2 | 1.0E-9 | 1.0E-9 | 2.2E-1 | 2.8E-1 | 49 | 9 | 46 | 9 | 0.53 |
| rU | ng/ml | 9.5E-2 | 4.1E-2 | 1.4E-1 | 7.7E-1 | 2.3E-1 | 1.6E0 | 1.0E-9 | 1.0E-9 | 1.4E0 | 4.4E0 | 49 | 9 | 46 | 9 | 0.43 |
| rT | ng/ml | 6.7E0 | 4.0E0 | 6.8E0 | 5.0E0 | 4.1E0 | 2.5E0 | 7.5E-1 | 1.3E0 | 2.1E1 | 1.0E1 | 49 | 9 | 46 | 9 | 0.35 |
| rS | ng/ml | 3.4E0 | 4.9E0 | 5.6E0 | 5.6E0 | 6.8E0 | 3.5E0 | 1.0E0 | 1.5E0 | 3.8E1 | 1.3E1 | 49 | 9 | 46 | 9 | 0.62 |
| sC | pg/mL | 5.6E3 | 8.9E3 | 8.5E3 | 1.3E4 | 7.1E3 | 1.2E4 | 1.7E3 | 2.3E3 | 3.2E4 | 3.9E4 | 49 | 8 | 45 | 8 | 0.64 |
| yL | pg/ml | 3.4E1 | 2.6E1 | 4.2E1 | 2.7E1 | 2.8E1 | 8.7E0 | 5.6E0 | 1.1E1 | 1.8E2 | 3.8E1 | 47 | 9 | 43 | 9 | 0.29 |
| rP | ng/ml | 7.7E1 | 1.3E2 | 1.7E2 | 2.2E2 | 2.3E2 | 2.2E2 | 1.0E-9 | 3.7E0 | 1.2E3 | 5.0E2 | 49 | 9 | 46 | 9 | 0.58 |
| rQ | ng/ml | 1.0E-9 | 1.0E-9 | 3.4E0 | 1.9E1 | 1.5E1 | 5.6E1 | 1.0E-9 | 1.0E-9 | 8.9E1 | 1.7E2 | 49 | 9 | 46 | 9 | 0.52 |
| rO | ng/ml | 2.5E-2 | 4.5E-2 | 3.9E-2 | 9.6E-2 | 7.1E-2 | 1.5E-1 | 1.0E-9 | 1.0E-9 | 4.4E-1 | 4.7E-1 | 49 | 9 | 46 | 9 | 0.61 |
| rR | ng/ml | 3.9E0 | 1.0E-9 | 2.2E1 | 5.3E0 | 6.6E1 | 1.2E1 | 1.0E-9 | 1.0E-9 | 4.5E2 | 3.6E1 | 49 | 9 | 46 | 9 | 0.38 |
| rN | ng/ml | 6.4E-1 | 6.7E-1 | 7.0E-1 | 8.3E-1 | 3.8E-1 | 6.5E-1 | 5.1E-2 | 2.7E-1 | 2.1E0 | 2.3E0 | 49 | 9 | 46 | 9 | 0.50 |
| qO | pg/ml | 9.7E3 | 1.2E4 | 1.2E4 | 1.6E4 | 8.2E3 | 1.6E4 | 2.2E3 | 7.4E2 | 3.9E4 | 5.0E4 | 50 | 8 | 47 | 8 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| qP | pg/ml | 3.6E2 | 3.9E2 | 3.9E2 | 5.3E2 | 2.5E2 | 5.8E2 | 1.0E-9 | 1.1E2 | 1.1E3 | 1.9E3 | 50 | 8 | 47 | 8 | 0.49 |
| qQ | pg/ml | 3.1E0 | 1.0E-9 | 1.5E1 | 6.2E0 | 4.0E1 | 8.7E0 | 1.0E-9 | 1.0E-9 | 2.8E2 | 1.9E1 | 50 | 8 | 47 | 8 | 0.42 |
| nW | pg/ml | 1.1E5 | 1.3E5 | 1.1E5 | 1.2E5 | 2.5E4 | 2.6E4 | 5.8E4 | 8.2E4 | 1.8E5 | 1.6E5 | 100 | 21 | 86 | 21 | 0.61 |
| nY | pg/ml | 1.9E3 | 2.3E3 | 2.3E3 | 2.8E3 | 1.4E3 | 2.7E3 | 6.5E2 | 6.6E2 | 9.9E3 | 1.3E4 | 100 | 21 | 86 | 21 | 0.53 |
| oO | pg/ml | 8.2E4 | 8.9E4 | 1.2E5 | 1.4E5 | 1.1E5 | 9.4E4 | 4.0E4 | 2.6E4 | 6.2E5 | 2.8E5 | 52 | 11 | 45 | 11 | 0.58 |
| oP | pg/ml | 1.3E5 | 2.0E5 | 1.4E5 | 2.0E5 | 8.4E4 | 9.0E4 | 4.8E4 | 7.1E4 | 3.5E5 | 3.6E5 | 52 | 11 | 45 | 11 | 0.69 |
| oQ | pg/ml | 3.0E3 | 4.4E3 | 3.2E3 | 5.4E3 | 1.6E3 | 3.6E3 | 1.1E3 | 1.7E3 | 1.0E4 | 1.2E4 | 52 | 11 | 45 | 11 | 0.70 |
| oE | pg/ml | 2.1E2 | 1.4E2 | 4.6E2 | 4.6E2 | 6.4E2 | 7.3E2 | 1.0E-9 | 1.0E-9 | 4.7E3 | 2.8E3 | 100 | 21 | 86 | 21 | 0.46 |
| oF | pg/ml | 8.6E3 | 1.3E4 | 2.0E4 | 3.3E4 | 3.3E4 | 3.9E4 | 6.4E1 | 6.5E2 | 2.3E5 | 1.4E5 | 100 | 21 | 86 | 21 | 0.61 |
| oH | pg/ml | 4.2E1 | 2.7E1 | 9.3E1 | 9.7E1 | 1.5E2 | 2.1E2 | 4.2E0 | 4.3E-1 | 8.6E2 | 9.9E2 | 100 | 21 | 86 | 21 | 0.44 |
| oK | pg/ml | 6.2E2 | 9.8E2 | 1.9E3 | 2.2E3 | 3.2E3 | 3.4E3 | 5.2E1 | 1.8E2 | 1.8E4 | 1.2E4 | 100 | 21 | 86 | 21 | 0.55 |
| oN | pg/ml | 4.7E2 | 6.4E2 | 8.0E2 | 8.5E2 | 1.9E3 | 7.5E2 | 1.5E2 | 2.8E2 | 1.8E4 | 3.7E3 | 100 | 21 | 86 | 21 | 0.66 |
| uL | ng/ml | 3.8E1 | 3.5E1 | 4.1E1 | 7.5E1 | 2.4E1 | 1.1E2 | 1.0E-9 | 2.4E1 | 1.6E2 | 3.4E2 | 48 | 8 | 44 | 8 | 0.51 |
| uO | ng/ml | 3.5E-1 | 3.9E-1 | 8.5E-1 | 6.2E-1 | 1.5E0 | 7.2E-1 | 1.0E-9 | 1.0E-9 | 9.3E0 | 2.0E0 | 48 | 8 | 44 | 8 | 0.46 |
| uM | ng/ml | 6.3E-1 | 6.5E-1 | 1.2E0 | 8.1E-1 | 2.3E0 | 3.8E-1 | 1.0E-9 | 4.7E-1 | 1.3E1 | 1.6E0 | 48 | 8 | 44 | 8 | 0.57 |
| uI | ng/ml | 6.5E-2 | 6.9E-2 | 1.3E-1 | 1.1E-1 | 1.9E-1 | 1.2E-1 | 1.6E-2 | 3.6E-2 | 1.1E0 | 3.9E-1 | 48 | 8 | 44 | 8 | 0.52 |
| uN | ng/ml | 1.5E1 | 1.5E1 | 1.6E1 | 1.7E1 | 6.5E0 | 7.1E0 | 7.8E0 | 8.1E0 | 4.1E1 | 3.0E1 | 48 | 8 | 44 | 8 | 0.51 |
| uG | ng/ml | 2.2E1 | 1.6E1 | 2.5E1 | 2.7E1 | 1.3E1 | 2.5E1 | 9.8E0 | 6.1E0 | 6.9E1 | 7.9E1 | 48 | 8 | 44 | 8 | 0.40 |
| uR | ng/ml | 2.3E0 | 2.5E0 | 4.2E0 | 2.7E0 | 9.1E0 | 1.3E0 | 9.9E-1 | 8.9E-1 | 6.4E1 | 5.5E0 | 48 | 9 | 44 | 9 | 0.52 |
| uP | ng/ml | 1.8E0 | 3.0E0 | 2.2E0 | 3.1E0 | 1.3E0 | 1.2E0 | 1.1E0 | 1.8E0 | 9.1E0 | 6.0E0 | 48 | 9 | 44 | 9 | 0.78 |
| uV | ng/ml | 1.0E-9 | 1.5E-3 | 1.1E-2 | 1.1E-2 | 3.3E-2 | 1.3E-2 | 1.0E-9 | 1.0E-9 | 2.0E-1 | 3.1E-2 | 48 | 9 | 44 | 9 | 0.57 |
| uT | ng/ml | 6.2E1 | 7.8E1 | 8.8E1 | 8.6E1 | 9.6E1 | 4.2E1 | 1.2E1 | 3.3E1 | 5.8E2 | 1.6E2 | 48 | 9 | 44 | 9 | 0.60 |
| uU | ng/ml | 1.6E0 | 1.0E0 | 2.0E0 | 1.9E0 | 1.1E0 | 1.8E0 | 6.0E-1 | 6.3E-1 | 5.4E0 | 6.0E0 | 48 | 9 | 44 | 9 | 0.35 |
| uW | ng/ml | 7.5E0 | 8.4E0 | 7.9E0 | 9.2E0 | 2.8E0 | 2.5E0 | 4.0E0 | 5.6E0 | 2.2E1 | 1.3E1 | 48 | 8 | 44 | 8 | 0.68 |
| vB | ng/ml | 2.7E0 | 3.7E0 | 2.7E0 | 3.9E0 | 1.3E0 | 1.8E0 | 5.9E-1 | 1.5E0 | 5.6E0 | 6.6E0 | 48 | 8 | 44 | 8 | 0.68 |
| vC | ng/ml | 1.0E-9 | 1.0E-9 | 7.5E-3 | 1.0E-9 | 3.5E-2 | 0.0E0 | 1.0E-9 | 1.0E-9 | 2.0E-1 | 1.0E-9 | 48 | 8 | 44 | 8 | 0.47 |
| uY | ng/ml | 8.3E-1 | 6.1E-1 | 1.3E0 | 8.4E-1 | 1.2E0 | 8.7E-1 | 8.7E-2 | 2.4E-1 | 4.9E0 | 3.0E0 | 48 | 8 | 44 | 8 | 0.36 |
| uZ | ng/ml | 5.9E-1 | 5.6E-1 | 8.8E-1 | 5.4E-1 | 1.1E0 | 1.9E-1 | 1.4E-1 | 2.7E-1 | 7.2E0 | 8.5E-1 | 48 | 8 | 44 | 8 | 0.42 |
| uX | ng/ml | 1.1E1 | 1.4E1 | 1.2E1 | 1.4E1 | 6.7E0 | 5.7E0 | 3.6E0 | 7.5E0 | 3.3E1 | 2.3E1 | 48 | 8 | 44 | 8 | 0.65 |
| vA | ng/ml | 6.8E-2 | 7.2E-2 | 8.5E-2 | 8.1E-2 | 5.8E-2 | 3.2E-2 | 2.5E-2 | 3.0E-2 | 3.0E-1 | 1.4E-1 | 48 | 8 | 44 | 8 | 0.55 |
| vH | ng/ml | 1.2E-1 | 1.4E-1 | 1.7E-1 | 1.5E-1 | 1.6E-1 | 7.4E-2 | 1.5E-2 | 5.8E-2 | 8.0E-1 | 2.6E-1 | 49 | 8 | 45 | 8 | 0.56 |
| vI | ng/ml | 1.8E0 | 1.7E0 | 1.9E0 | 2.3E0 | 1.2E0 | 1.9E0 | 6.2E-3 | 1.2E-2 | 5.1E0 | 6.2E0 | 49 | 8 | 45 | 8 | 0.53 |
| vP | ng/ml | 3.8E2 | 4.8E2 | 4.4E2 | 5.6E2 | 3.2E2 | 5.0E2 | 7.0E1 | 6.7E1 | 1.5E3 | 1.7E3 | 48 | 9 | 44 | 9 | 0.55 |
| vT | ng/ml | 7.7E1 | 9.9E1 | 1.2E2 | 1.1E2 | 1.2E2 | 7.5E1 | 4.1E1 | 3.0E1 | 6.9E2 | 2.8E2 | 48 | 9 | 44 | 9 | 0.53 |
| vU | ng/ml | 1.0E-9 | 1.0E-9 | 2.3E1 | 2.6E1 | 3.2E1 | 4.2E1 | 1.0E-9 | 1.0E-9 | 1.0E2 | 1.2E2 | 48 | 9 | 44 | 9 | 0.48 |
| vQ | ng/ml | 3.4E2 | 3.5E2 | 3.4E2 | 4.0E2 | 1.3E2 | 2.0E2 | 6.7E1 | 2.1E2 | 7.0E2 | 8.4E2 | 48 | 9 | 44 | 9 | 0.57 |
| vO | ng/ml | 1.7E3 | 1.8E3 | 1.8E3 | 1.9E3 | 4.6E2 | 5.1E2 | 1.0E3 | 1.2E3 | 3.0E3 | 2.8E3 | 48 | 9 | 44 | 9 | 0.57 |
| vS | ng/ml | 1.3E3 | 1.6E3 | 1.3E3 | 1.4E3 | 3.5E2 | 5.9E2 | 1.0E-9 | 1.0E-9 | 1.8E3 | 2.1E3 | 48 | 9 | 44 | 9 | 0.65 |
| vV | ng/ml | 8.4E2 | 1.4E3 | 1.1E3 | 1.4E3 | 9.9E2 | 1.3E3 | 1.1E2 | 1.1E2 | 5.0E3 | 4.1E3 | 48 | 9 | 44 | 9 | 0.56 |
| vW | ng/ml | 1.5E2 | 1.0E2 | 1.7E2 | 1.2E2 | 1.3E2 | 5.6E1 | 4.3E1 | 7.7E1 | 6.7E2 | 2.5E2 | 48 | 9 | 44 | 9 | 0.38 |
| pF | pg/ml | 5.0E-1 | 4.1E-1 | 6.8E-1 | 1.0E0 | 1.0E0 | 1.8E0 | 1.0E-9 | 1.0E-9 | 9.4E0 | 7.8E0 | 100 | 21 | 86 | 21 | 0.50 |

Figure 24.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Po | pg/ml | 6.9E-1 | 3.6E0 | 8.0E0 | 7.2E0 | 2.2E1 | 8.3E0 | 8.0E-3 | 2.6E-2 | 2.7E2 | 1.9E1 | 932 | 7 | 343 | 7 | 0.59 |
| Et | ng/ml | 1.4E3 | 2.6E3 | 1.7E3 | 2.5E3 | 1.2E3 | 1.6E3 | 7.5E1 | 3.9E2 | 5.0E3 | 4.1E3 | 931 | 7 | 343 | 7 | 0.65 |
| Fp | ng/ml | 1.3E1 | 1.4E1 | 2.5E1 | 2.5E1 | 2.8E1 | 2.9E1 | 6.0E-3 | 2.4E-1 | 1.4E2 | 7.2E1 | 965 | 7 | 344 | 7 | 0.49 |
| Fr | ng/ml | 3.9E4 | 4.4E4 | 1.1E5 | 2.7E5 | 1.7E5 | 3.0E5 | 1.9E2 | 2.5E3 | 9.0E5 | 6.0E5 | 1079 | 7 | 348 | 7 | 0.63 |
| Nm | pg/ml | 1.4E4 | 1.5E4 | 3.3E4 | 2.2E4 | 7.8E4 | 2.2E4 | 1.0E-9 | 1.0E-9 | 1.6E6 | 4.9E4 | 935 | 7 | 345 | 7 | 0.47 |
| Nn | pg/ml | 1.6E2 | 3.9E1 | 1.7E3 | 5.5E2 | 7.6E3 | 7.8E2 | 1.0E-9 | 1.1E0 | 1.0E5 | 1.9E3 | 935 | 7 | 345 | 7 | 0.45 |
| No | pg/ml | 1.6E1 | 8.7E0 | 3.6E1 | 2.7E1 | 1.1E2 | 5.0E1 | 1.0E-9 | 5.1E-1 | 2.5E3 | 1.4E2 | 935 | 7 | 345 | 7 | 0.37 |
| Nq | pg/ml | 2.0E0 | 1.0E-9 | 1.8E1 | 1.5E1 | 7.2E1 | 2.8E1 | 1.0E-9 | 1.0E-9 | 1.2E3 | 7.3E1 | 935 | 7 | 345 | 7 | 0.46 |
| Nr | pg/ml | 9.9E-1 | 3.5E-1 | 2.8E1 | 1.1E0 | 1.7E2 | 1.4E0 | 1.0E-9 | 1.0E-9 | 4.1E3 | 3.2E0 | 935 | 7 | 345 | 7 | 0.40 |
| Ns | pg/ml | 1.0E-9 | 1.0E-9 | 9.0E0 | 5.1E-1 | 5.1E1 | 1.4E0 | 1.0E-9 | 1.0E-9 | 1.1E3 | 3.6E0 | 935 | 7 | 345 | 7 | 0.51 |
| Nt | pg/ml | 1.0E2 | 9.7E1 | 1.3E2 | 9.9E1 | 1.1E2 | 5.8E1 | 1.0E-9 | 2.3E1 | 1.5E3 | 2.1E2 | 935 | 7 | 345 | 7 | 0.43 |
| Nu | pg/ml | 2.0E1 | 7.6E1 | 5.4E1 | 8.5E1 | 8.9E1 | 6.9E1 | 1.0E-9 | 1.0E-9 | 8.9E2 | 2.0E2 | 935 | 7 | 345 | 7 | 0.69 |
| Lu | pg/ml | 1.0E4 | 6.8E3 | 1.8E4 | 6.7E3 | 6.1E4 | 3.9E3 | 3.5E2 | 2.1E3 | 1.3E6 | 1.1E4 | 938 | 7 | 345 | 7 | 0.36 |
| Lv | pg/ml | 1.0E-9 | 1.0E-9 | 1.1E1 | 4.4E1 | 2.2E1 | 7.7E1 | 1.0E-9 | 1.0E-9 | 2.6E2 | 1.9E2 | 938 | 7 | 345 | 7 | 0.52 |
| Lw | pg/ml | 1.0E-9 | 1.0E-9 | 3.9E-1 | 1.4E0 | 4.0E0 | 3.8E0 | 1.0E-9 | 1.0E-9 | 8.0E1 | 9.9E0 | 938 | 7 | 345 | 7 | 0.56 |
| Lx | pg/ml | 1.0E-9 | 1.0E-9 | 1.4E2 | 2.9E2 | 4.1E2 | 5.2E2 | 1.0E-9 | 1.0E-9 | 6.2E3 | 1.4E3 | 938 | 7 | 345 | 7 | 0.55 |
| Ly | pg/ml | 1.0E-9 | 3.9E0 | 1.0E1 | 8.2E0 | 2.0E1 | 1.0E1 | 1.0E-9 | 1.0E-9 | 1.6E2 | 2.9E1 | 938 | 7 | 345 | 7 | 0.67 |
| Lz | pg/ml | 1.0E-9 | 1.0E-9 | 3.8E0 | 1.0E-9 | 3.0E1 | 0.0E0 | 1.0E-9 | 1.0E-9 | 6.0E2 | 1.0E-9 | 938 | 7 | 345 | 7 | 0.46 |
| Ma | pg/ml | 2.8E2 | 4.8E2 | 1.3E3 | 9.2E2 | 3.5E3 | 8.8E2 | 1.0E-9 | 2.2E0 | 6.5E4 | 1.9E3 | 938 | 7 | 345 | 7 | 0.56 |
| Mb | pg/ml | 2.5E1 | 2.1E1 | 3.1E1 | 2.7E1 | 1.5E1 | 1.3E1 | 5.4E0 | 1.4E1 | 2.1E2 | 4.7E1 | 938 | 7 | 345 | 7 | 0.38 |
| Mc | pg/ml | 1.0E-9 | 1.0E-9 | 4.1E-2 | 1.0E-9 | 5.6E-1 | 0.0E0 | 1.0E-9 | 1.0E-9 | 1.3E1 | 1.0E-9 | 938 | 7 | 345 | 7 | 0.49 |
| Md | pg/ml | 1.0E-9 | 1.0E-9 | 3.9E-1 | 1.0E-9 | 3.4E0 | 0.0E0 | 1.0E-9 | 1.0E-9 | 6.8E1 | 1.0E-9 | 938 | 7 | 345 | 7 | 0.47 |
| Me | pg/ml | 3.3E1 | 2.9E1 | 3.2E1 | 2.9E1 | 2.0E1 | 9.4E0 | 1.0E-9 | 1.3E1 | 3.2E2 | 4.2E1 | 938 | 7 | 345 | 7 | 0.44 |
| Mf | pg/ml | 1.0E-9 | 1.0E-9 | 5.2E-1 | 1.0E-9 | 2.9E0 | 0.0E0 | 1.0E-9 | 1.0E-9 | 5.6E1 | 1.0E-9 | 938 | 7 | 345 | 7 | 0.43 |
| Mg | pg/ml | 1.6E0 | 1.0E1 | 7.4E0 | 7.5E0 | 1.3E1 | 6.0E0 | 1.0E-9 | 1.0E-9 | 9.4E1 | 1.4E1 | 938 | 7 | 345 | 7 | 0.59 |
| Mh | pg/ml | 1.0E-9 | 1.0E-9 | 1.3E0 | 2.2E-2 | 9.4E0 | 3.8E-2 | 1.0E-9 | 1.0E-9 | 1.6E2 | 8.5E-2 | 938 | 7 | 345 | 7 | 0.47 |
| Mi | pg/ml | 1.0E-9 | 1.0E-9 | 4.8E-1 | 4.4E0 | 5.2E0 | 1.2E1 | 1.0E-9 | 1.0E-9 | 1.2E2 | 3.1E1 | 938 | 7 | 345 | 7 | 0.56 |
| Mj | pg/ml | 1.0E-9 | 1.0E-9 | 4.3E0 | 3.3E-1 | 2.4E1 | 8.8E-1 | 1.0E-9 | 1.0E-9 | 4.6E2 | 2.3E0 | 938 | 7 | 345 | 7 | 0.46 |
| Mk | pg/ml | 9.1E-1 | 1.0E-9 | 1.4E1 | 1.1E0 | 8.4E1 | 2.8E0 | 1.0E-9 | 1.0E-9 | 1.7E3 | 7.4E0 | 938 | 7 | 345 | 7 | 0.31 |
| Ml | pg/ml | 1.0E-9 | 1.0E-9 | 4.9E0 | 1.0E-9 | 7.2E1 | 0.0E0 | 1.0E-9 | 1.0E-9 | 2.1E3 | 1.0E-9 | 938 | 7 | 345 | 7 | 0.41 |
| Mm | pg/ml | 5.9E2 | 7.7E2 | 1.0E3 | 2.2E3 | 1.2E3 | 3.6E3 | 1.0E-9 | 1.9E1 | 1.1E4 | 9.9E3 | 938 | 7 | 345 | 7 | 0.53 |
| Mn | pg/ml | 5.5E0 | 1.4E1 | 1.0E1 | 1.4E1 | 2.2E1 | 1.1E1 | 1.0E-9 | 4.9E-1 | 3.5E2 | 3.3E1 | 938 | 7 | 345 | 7 | 0.69 |
| Mp | pg/ml | 1.0E-9 | 1.0E-9 | 8.9E0 | 2.0E1 | 2.9E1 | 3.7E1 | 1.0E-9 | 1.0E-9 | 6.3E2 | 1.0E2 | 937 | 7 | 345 | 7 | 0.55 |
| Mq | pg/ml | 1.0E-9 | 1.0E-9 | 2.7E0 | 8.8E0 | 1.6E1 | 2.3E1 | 1.0E-9 | 1.0E-9 | 2.4E2 | 6.2E1 | 937 | 7 | 345 | 7 | 0.54 |
| Mr | pg/ml | 1.0E-9 | 1.0E-9 | 2.5E1 | 6.5E-2 | 1.4E2 | 1.7E-1 | 1.0E-9 | 1.0E-9 | 2.2E3 | 4.6E-1 | 937 | 7 | 345 | 7 | 0.40 |
| Ms | pg/ml | 4.1E2 | 3.1E2 | 5.6E2 | 3.5E2 | 6.6E2 | 2.8E2 | 1.0E-9 | 1.0E-9 | 5.9E3 | 7.9E2 | 937 | 7 | 345 | 7 | 0.43 |
| Mt | pg/ml | 2.5E-1 | 1.2E0 | 6.8E0 | 1.5E0 | 4.1E1 | 1.5E0 | 1.0E-9 | 1.0E-9 | 8.7E2 | 3.6E0 | 937 | 7 | 345 | 7 | 0.57 |
| Mu | pg/ml | 1.0E-9 | 1.0E-9 | 1.2E0 | 1.1E0 | 1.0E1 | 2.1E0 | 1.0E-9 | 1.0E-9 | 2.3E2 | 5.5E0 | 937 | 7 | 345 | 7 | 0.57 |
| Mv | pg/ml | 1.0E-9 | 1.0E-9 | 6.8E1 | 6.4E1 | 3.1E2 | 1.2E2 | 1.0E-9 | 1.0E-9 | 4.5E3 | 3.1E2 | 937 | 7 | 345 | 7 | 0.54 |
| Mw | pg/ml | 3.8E1 | 7.4E1 | 5.0E2 | 4.2E2 | 3.2E3 | 5.5E2 | 1.0E-9 | 1.0E-9 | 6.2E4 | 1.4E3 | 937 | 7 | 345 | 7 | 0.62 |
| Mx | pg/ml | 1.0E-9 | 1.0E-9 | 2.4E-1 | 6.0E-1 | 1.3E0 | 1.3E0 | 1.0E-9 | 1.0E-9 | 3.2E1 | 3.4E0 | 937 | 7 | 345 | 7 | 0.58 |
| My | pg/ml | 1.0E-9 | 7.4E1 | 4.5E2 | 1.1E2 | 2.9E3 | 1.4E2 | 1.0E-9 | 1.0E-9 | 3.9E4 | 3.8E2 | 937 | 7 | 345 | 7 | 0.67 |
| Mz | pg/ml | 1.1E1 | 1.2E1 | 2.7E1 | 9.8E0 | 6.3E1 | 4.9E0 | 1.0E-9 | 3.5E0 | 1.2E3 | 1.5E1 | 937 | 7 | 345 | 7 | 0.45 |
| Na | pg/ml | 1.0E-9 | 1.0E-9 | 6.6E-1 | 9.9E-1 | 2.6E0 | 2.2E0 | 1.0E-9 | 1.0E-9 | 3.6E1 | 6.0E0 | 937 | 7 | 345 | 7 | 0.55 |
| Nb | pg/ml | 2.1E0 | 9.9E-1 | 3.8E0 | 3.1E0 | 1.1E1 | 4.0E0 | 1.0E-9 | 1.0E-9 | 2.3E2 | 1.0E1 | 937 | 7 | 345 | 7 | 0.45 |
| Nc | pg/ml | 3.4E2 | 7.7E1 | 5.6E2 | 3.0E2 | 7.2E2 | 4.9E2 | 1.0E-9 | 1.0E-9 | 9.2E3 | 1.3E3 | 937 | 7 | 345 | 7 | 0.41 |
| Nd | pg/ml | 2.9E1 | 9.6E0 | 2.7E1 | 1.4E1 | 4.4E1 | 1.4E1 | 1.0E-9 | 1.0E-9 | 1.2E3 | 3.4E1 | 937 | 7 | 345 | 7 | 0.33 |
| Ne | pg/ml | 4.4E2 | 7.6E1 | 5.7E2 | 2.6E2 | 5.7E2 | 2.9E2 | 1.0E-9 | 1.3E1 | 7.0E3 | 7.3E2 | 937 | 7 | 345 | 7 | 0.33 |
| Nf | pg/ml | 1.0E-9 | 1.0E-9 | 2.6E0 | 3.9E-1 | 9.4E0 | 1.0E0 | 1.0E-9 | 1.0E-9 | 1.6E2 | 2.7E0 | 937 | 7 | 345 | 7 | 0.44 |
| Ng | pg/ml | 1.9E1 | 1.0E-9 | 1.3E2 | 5.5E1 | 2.5E2 | 8.1E1 | 1.0E-9 | 1.0E-9 | 2.3E3 | 1.9E2 | 937 | 7 | 345 | 7 | 0.40 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nh | pg/ml | 6.9E1 | 5.3E1 | 9.0E1 | 5.2E1 | 8.2E1 | 3.7E1 | 1.0E-9 | 1.1E1 | 5.6E2 | 9.3E1 | 937 | 7 | 345 | 7 | 0.37 |
| Ni | pg/ml | 1.0E-9 | 1.0E-9 | 7.2E1 | 1.1E2 | 1.2E2 | 1.8E2 | 1.0E-9 | 1.0E-9 | 7.1E2 | 4.8E2 | 937 | 7 | 345 | 7 | 0.52 |
| Nj | pg/ml | 7.5E0 | 2.7E0 | 1.1E1 | 6.2E0 | 1.2E1 | 6.2E0 | 1.0E-9 | 1.0E-9 | 1.1E2 | 1.7E1 | 937 | 7 | 345 | 7 | 0.39 |
| Nk | pg/ml | 1.7E1 | 1.4E1 | 3.3E1 | 2.2E1 | 3.9E1 | 2.3E1 | 1.0E-9 | 1.0E-9 | 2.0E2 | 6.9E1 | 937 | 7 | 345 | 7 | 0.48 |
| Nl | pg/ml | 4.5E1 | 2.7E1 | 6.1E1 | 4.3E1 | 6.8E1 | 4.7E1 | 1.0E-9 | 1.0E-9 | 1.1E3 | 1.4E2 | 937 | 7 | 345 | 7 | 0.41 |
| Hq | pg/ml | 1.1E0 | 1.3E0 | 9.6E1 | 1.1E1 | 1.6E3 | 2.6E1 | 1.0E-9 | 1.0E-9 | 3.4E4 | 7.0E1 | 933 | 7 | 344 | 7 | 0.51 |
| Hr | pg/ml | 1.1E2 | 1.0E3 | 7.5E2 | 1.9E3 | 1.6E3 | 1.8E3 | 1.0E-9 | 6.9E1 | 1.7E4 | 4.6E3 | 933 | 7 | 344 | 7 | 0.77 |
| Hu | pg/ml | 5.3E0 | 1.0E-9 | 3.0E3 | 7.1E1 | 2.6E4 | 1.6E2 | 1.0E-9 | 1.0E-9 | 6.3E5 | 4.4E2 | 933 | 7 | 344 | 7 | 0.42 |
| Hv | pg/ml | 1.4E0 | 2.7E0 | 3.3E0 | 2.1E0 | 1.2E1 | 1.5E0 | 1.0E-9 | 3.0E-1 | 2.5E2 | 4.2E0 | 933 | 7 | 344 | 7 | 0.58 |
| Hw | pg/ml | 6.4E0 | 4.9E0 | 1.8E1 | 5.8E0 | 6.9E1 | 5.6E0 | 1.0E-9 | 1.0E-9 | 1.7E3 | 1.5E1 | 933 | 7 | 344 | 7 | 0.37 |
| Hx | pg/ml | 8.8E0 | 2.6E1 | 3.7E1 | 2.9E1 | 3.1E1 | 2.0E1 | 1.0E-9 | 1.0E-9 | 9.3E3 | 6.4E1 | 933 | 7 | 344 | 7 | 0.70 |
| Ih | ng/ml | 7.2E1 | 4.5E1 | 2.4E2 | 6.9E1 | 4.8E2 | 7.1E1 | 1.0E-9 | 1.5E1 | 7.4E3 | 2.0E2 | 937 | 7 | 344 | 7 | 0.42 |
| Ii | ng/ml | 9.5E1 | 5.7E1 | 2.4E2 | 7.8E1 | 6.6E2 | 9.5E1 | 1.0E-9 | 1.6E1 | 1.0E4 | 2.9E2 | 937 | 7 | 344 | 7 | 0.37 |
| Ij | ng/ml | 7.6E1 | 7.8E1 | 1.7E2 | 8.2E1 | 5.7E2 | 6.6E1 | 1.6E-1 | 1.0E-9 | 6.4E3 | 1.7E2 | 924 | 7 | 342 | 7 | 0.44 |
| Ik | ng/ml | 1.3E1 | 6.2E1 | 7.9E2 | 1.2E2 | 8.0E3 | 1.7E2 | 5.9E-1 | 5.0E0 | 1.2E5 | 4.8E2 | 932 | 7 | 342 | 7 | 0.57 |
| Il | ng/ml | 3.3E2 | 5.3E2 | 1.3E3 | 6.3E2 | 2.8E3 | 7.0E2 | 1.0E-9 | 1.0E-9 | 1.3E4 | 2.1E3 | 916 | 7 | 342 | 7 | 0.53 |
| Im | ng/ml | 2.1E2 | 1.2E2 | 4.0E2 | 2.6E2 | 7.6E2 | 2.5E2 | 1.3E1 | 8.3E1 | 1.5E4 | 7.1E2 | 931 | 7 | 343 | 7 | 0.42 |
| In | ng/ml | 3.3E0 | 2.4E0 | 2.1E1 | 4.4E0 | 1.5E2 | 4.0E0 | 1.0E-9 | 1.0E-9 | 3.9E3 | 1.1E1 | 937 | 7 | 344 | 7 | 0.46 |
| Io | ng/ml | 8.3E3 | 3.6E3 | 2.5E4 | 7.3E3 | 1.4E5 | 6.8E3 | 1.0E-9 | 1.3E3 | 4.0E6 | 2.0E4 | 928 | 7 | 344 | 7 | 0.40 |
| Ip | ng/ml | 1.0E1 | 4.8E0 | 2.0E1 | 2.1E1 | 2.4E1 | 2.4E1 | 1.0E-9 | 6.4E-1 | 2.6E2 | 5.8E1 | 928 | 7 | 344 | 7 | 0.53 |
| Iq | ug/ml | 1.0E-1 | 1.8E-2 | 3.0E1 | 9.4E-2 | 6.3E2 | 1.2E-1 | 1.0E-9 | 1.0E-9 | 1.4E4 | 3.0E-1 | 928 | 7 | 344 | 7 | 0.35 |
| Ir | ug/ml | 3.5E-1 | 6.0E-1 | 3.4E0 | 7.0E-1 | 2.4E1 | 5.1E-1 | 1.0E-9 | 2.0E-1 | 5.1E2 | 1.6E0 | 927 | 7 | 344 | 7 | 0.59 |
| Is | ng/ml | 1.6E0 | 5.7E0 | 6.4E0 | 6.4E0 | 2.2E1 | 4.9E0 | 1.0E-9 | 9.9E-1 | 5.5E2 | 1.3E1 | 928 | 7 | 344 | 7 | 0.70 |
| It | ng/ml | 2.0E0 | 7.7E-1 | 2.3E1 | 4.2E0 | 1.4E2 | 5.7E0 | 1.0E-9 | 1.0E-9 | 2.8E3 | 1.4E1 | 928 | 7 | 344 | 7 | 0.47 |
| Iu | ng/ml | 2.2E2 | 2.0E2 | 1.3E3 | 3.3E2 | 4.0E3 | 3.7E2 | 1.0E-9 | 4.0E1 | 2.9E4 | 1.1E3 | 928 | 7 | 344 | 7 | 0.49 |
| Iv | ng/ml | 1.3E1 | 6.9E1 | 6.0E1 | 5.0E1 | 5.4E2 | 4.0E1 | 1.0E-9 | 1.0E-9 | 1.6E4 | 9.2E1 | 927 | 7 | 344 | 7 | 0.65 |
| Pz | ng/ml | 3.8E3 | 1.0E4 | 8.1E3 | 7.4E3 | 3.7E4 | 4.5E3 | 1.3E1 | 6.1E2 | 1.0E6 | 1.0E4 | 929 | 7 | 342 | 7 | 0.61 |
| Qa | ng/ml | 3.5E3 | 5.6E3 | 6.3E3 | 7.0E3 | 7.4E3 | 5.0E3 | 1.2E1 | 1.8E3 | 5.2E4 | 1.6E4 | 929 | 7 | 342 | 7 | 0.63 |
| Qb | ng/ml | 9.7E1 | 2.1E2 | 2.1E2 | 2.1E2 | 4.8E2 | 1.1E2 | 7.9E-1 | 5.7E1 | 8.3E3 | 3.8E2 | 929 | 7 | 342 | 7 | 0.68 |
| Qc | ng/ml | 2.3E2 | 1.2E2 | 6.3E2 | 1.6E2 | 5.5E3 | 1.5E2 | 1.0E-9 | 2.7E1 | 1.7E5 | 4.8E2 | 929 | 7 | 342 | 7 | 0.35 |
| Qd | ng/ml | 9.2E3 | 9.9E3 | 2.1E4 | 2.7E4 | 7.7E4 | 3.3E4 | 1.5E2 | 2.3E3 | 2.0E6 | 8.7E4 | 929 | 7 | 342 | 7 | 0.58 |
| Qe | ng/ml | 9.2E2 | 2.2E3 | 1.8E3 | 3.1E3 | 3.8E3 | 2.8E3 | 1.0E-9 | 5.0E2 | 9.7E4 | 8.1E3 | 929 | 7 | 342 | 7 | 0.70 |
| Jg | ng/ml | 4.9E2 | 7.2E2 | 8.2E2 | 8.6E2 | 1.0E3 | 7.3E2 | 1.0E-9 | 5.9E1 | 1.0E4 | 1.7E3 | 933 | 7 | 344 | 7 | 0.55 |
| Jh | ng/ml | 3.0E0 | 2.4E0 | 2.7E1 | 1.5E1 | 1.1E2 | 2.5E1 | 1.0E-9 | 1.0E-9 | 1.6E3 | 6.8E1 | 933 | 7 | 344 | 7 | 0.56 |
| Ji | ng/ml | 5.3E1 | 1.0E2 | 7.7E1 | 1.5E2 | 8.0E1 | 1.2E2 | 1.0E-9 | 3.4E1 | 6.9E2 | 3.8E2 | 933 | 7 | 344 | 7 | 0.73 |
| Jj | ng/ml | 6.0E2 | 9.8E2 | 1.7E3 | 8.5E2 | 1.2E4 | 5.5E2 | 2.3E0 | 1.3E1 | 3.4E5 | 1.4E3 | 933 | 7 | 344 | 7 | 0.54 |
| Jk | ng/ml | 3.1E0 | 7.9E0 | 2.1E1 | 1.5E1 | 4.7E1 | 2.1E1 | 1.0E-9 | 6.2E-1 | 3.9E2 | 6.1E1 | 933 | 7 | 344 | 7 | 0.59 |
| Jl | ng/ml | 4.4E-1 | 5.7E-1 | 1.9E0 | 6.5E0 | 5.6E0 | 9.4E0 | 7.6E-4 | 1.1E-1 | 1.1E2 | 2.0E1 | 933 | 7 | 344 | 7 | 0.58 |
| Jm | ng/ml | 1.8E1 | 1.5E1 | 5.8E1 | 2.5E1 | 1.3E2 | 2.4E1 | 1.0E-9 | 2.6E0 | 2.1E3 | 6.2E1 | 933 | 7 | 344 | 7 | 0.48 |
| Jn | pg/ml | 4.0E-1 | 8.7E-1 | 2.5E0 | 7.4E-1 | 2.2E1 | 5.9E-1 | 1.0E-9 | 4.7E-2 | 6.2E2 | 1.5E0 | 932 | 7 | 344 | 7 | 0.59 |
| Jo | pg/ml | 3.6E3 | 4.7E3 | 4.7E3 | 5.1E3 | 3.8E3 | 4.6E3 | 2.0E1 | 9.0E2 | 2.4E4 | 1.3E4 | 933 | 7 | 344 | 7 | 0.50 |
| Jp | pg/ml | 7.0E4 | 9.8E4 | 7.4E4 | 8.9E4 | 3.8E4 | 2.5E4 | 5.8E2 | 5.1E4 | 3.8E5 | 1.2E5 | 933 | 7 | 344 | 7 | 0.67 |
| Jq | pg/ml | 9.4E1 | 1.5E2 | 1.5E2 | 2.4E2 | 2.1E2 | 2.3E2 | 1.0E0 | 4.0E1 | 4.0E3 | 7.5E2 | 933 | 7 | 344 | 7 | 0.69 |
| Jr | pg/ml | 5.2E0 | 1.2E1 | 3.3E1 | 1.6E1 | 3.6E2 | 1.5E1 | 1.0E-9 | 1.0E-9 | 1.1E4 | 4.0E1 | 933 | 7 | 344 | 7 | 0.67 |
| Js | pg/ml | 1.3E1 | 9.4E0 | 4.9E1 | 1.1E1 | 3.6E2 | 6.3E0 | 1.0E-9 | 4.5E-1 | 1.0E4 | 2.0E1 | 933 | 7 | 344 | 7 | 0.41 |
| Jt | pg/ml | 2.6E3 | 2.3E3 | 3.2E3 | 2.3E3 | 2.4E3 | 1.9E3 | 2.2E1 | 3.5E2 | 2.2E4 | 5.9E3 | 933 | 7 | 344 | 7 | 0.38 |
| Lh | pg/ml | 1.3E4 | 1.7E4 | 2.1E4 | 2.7E4 | 2.6E4 | 2.5E4 | 1.0E-9 | 4.6E3 | 2.6E5 | 7.3E4 | 932 | 7 | 345 | 7 | 0.60 |
| Li | pg/ml | 3.5E3 | 1.3E3 | 1.7E4 | 1.5E4 | 6.2E4 | 2.5E4 | 1.0E-9 | 5.0E2 | 1.3E6 | 6.8E4 | 932 | 7 | 345 | 7 | 0.49 |
| Lj | pg/ml | 2.8E3 | 3.9E3 | 2.3E4 | 3.2E3 | 6.4E4 | 3.0E3 | 1.0E-9 | 1.9E2 | 5.2E5 | 8.1E3 | 932 | 7 | 345 | 7 | 0.42 |
| Nv | pg/ml | 4.0E3 | 5.1E3 | 1.1E4 | 1.7E4 | 4.2E4 | 2.6E4 | 1.0E-9 | 1.3E3 | 1.1E6 | 7.1E4 | 939 | 7 | 345 | 7 | 0.59 |
| Nw | pg/ml | 8.9E3 | 1.1E4 | 1.3E4 | 1.7E4 | 1.6E4 | 1.6E4 | 8.6E1 | 3.4E3 | 2.1E5 | 5.0E4 | 939 | 7 | 345 | 7 | 0.59 |
| Nx | pg/ml | 2.2E2 | 1.1E3 | 4.1E2 | 1.0E3 | 6.4E2 | 6.4E2 | 1.0E-9 | 2.3E2 | 7.4E3 | 1.8E3 | 939 | 7 | 345 | 7 | 0.82 |
| Ny | pg/ml | 6.2E0 | 7.6E0 | 5.2E1 | 3.0E1 | 8.3E2 | 4.4E1 | 1.0E-9 | 1.0E-9 | 2.5E4 | 1.0E2 | 939 | 7 | 345 | 7 | 0.55 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|-----|--------|-----|-----|
| | | NonDis | Dis | NonDis | Dis | NonDis | Dis | NonDis | Dis | NonDis | Dis | NonDis | Dis | NonDis | Dis | |
| Oe | pg/ml | 7.1E1 | 1.0E-9 | 2.9E2 | 2.9E2 | 7.4E2 | 4.4E2 | 1.0E-9 | 1.0E-9 | 1.9E4 | 1.1E3 | 930 | 7 | 344 | 7 | 0.46 |
| Of | pg/ml | 1.8E2 | 1.2E2 | 5.9E3 | 6.4E3 | 2.8E4 | 1.5E4 | 1.0E-9 | 1.0E-9 | 6.2E5 | 4.0E4 | 938 | 7 | 345 | 7 | 0.49 |
| Og | pg/ml | 8.2E-2 | 9.3E-2 | 7.1E-1 | 1.7E-1 | 4.7E0 | 2.0E-1 | 1.0E-9 | 1.0E-9 | 8.3E1 | 5.3E-1 | 938 | 7 | 345 | 7 | 0.46 |
| Oh | pg/ml | 2.6E0 | 4.2E0 | 1.8E1 | 5.0E0 | 1.4E2 | 6.6E0 | 1.0E-9 | 1.0E-9 | 3.5E3 | 1.9E1 | 938 | 7 | 345 | 7 | 0.49 |
| Oi | pg/ml | 2.6E0 | 3.6E0 | 6.3E0 | 6.0E0 | 9.8E0 | 7.1E0 | 1.0E-9 | 1.0E-9 | 8.6E1 | 1.9E1 | 938 | 7 | 345 | 7 | 0.53 |
| Ok | pg/ml | 3.9E2 | 9.8E2 | 5.4E2 | 1.4E3 | 5.7E2 | 1.8E3 | 1.3E1 | 1.4E2 | 7.0E3 | 5.2E3 | 938 | 7 | 345 | 7 | 0.66 |
| Om | pg/ml | 3.9E2 | 5.8E2 | 8.2E2 | 8.9E2 | 2.1E3 | 9.3E2 | 1.0E-9 | 1.0E-9 | 3.6E4 | 2.5E3 | 938 | 7 | 345 | 7 | 0.57 |
| On | pg/ml | 1.8E2 | 3.1E2 | 2.8E2 | 6.1E2 | 3.9E2 | 6.2E2 | 1.0E-9 | 8.9E1 | 4.5E3 | 1.7E3 | 938 | 7 | 345 | 7 | 0.69 |
| Oy | pg/ml | 4.9E-1 | 4.7E-1 | 5.7E0 | 1.3E0 | 2.8E1 | 2.4E0 | 1.0E-9 | 1.5E-1 | 4.0E2 | 6.6E0 | 937 | 7 | 344 | 7 | 0.51 |
| Oz | pg/ml | 1.2E-2 | 1.0E-1 | 3.2E-1 | 2.0E-1 | 1.3E0 | 2.3E-1 | 1.0E-9 | 1.0E-9 | 2.9E1 | 6.0E-1 | 937 | 7 | 344 | 7 | 0.56 |
| Pa | pg/ml | 3.9E-1 | 3.6E-1 | 1.4E0 | 4.1E-1 | 4.9E0 | 2.8E-1 | 1.0E-9 | 1.0E-9 | 8.6E1 | 9.2E-1 | 937 | 7 | 344 | 7 | 0.47 |
| Pb | pg/ml | 1.0E-9 | 1.0E-9 | 7.4E-1 | 1.0E-1 | 1.6E1 | 1.3E-1 | 1.0E-9 | 1.0E-9 | 4.9E2 | 2.9E-1 | 937 | 7 | 344 | 7 | 0.51 |
| Pc | pg/ml | 5.4E-2 | 2.7E-1 | 3.5E-1 | 3.1E-1 | 8.4E-1 | 2.6E-1 | 1.0E-9 | 1.0E-9 | 1.6E1 | 6.4E-1 | 937 | 7 | 344 | 7 | 0.56 |
| Pd | pg/ml | 1.8E0 | 1.9E0 | 4.8E0 | 2.7E0 | 2.8E1 | 1.7E0 | 1.0E-9 | 9.3E-1 | 8.4E2 | 5.4E0 | 937 | 7 | 344 | 7 | 0.56 |
| Pe | pg/ml | 2.2E1 | 4.6E1 | 1.0E2 | 5.1E1 | 3.4E2 | 5.3E1 | 1.0E-9 | 3.7E0 | 4.7E3 | 1.6E2 | 937 | 7 | 344 | 7 | 0.55 |
| Pf | pg/ml | 1.6E0 | 2.4E0 | 1.0E1 | 1.3E1 | 5.7E1 | 2.8E1 | 1.0E-9 | 3.6E-1 | 1.5E3 | 7.7E1 | 937 | 7 | 344 | 7 | 0.56 |
| Pg | pg/ml | 3.5E0 | 5.1E0 | 4.1E1 | 2.1E1 | 3.3E2 | 3.0E1 | 1.0E-9 | 1.3E0 | 7.7E3 | 7.8E1 | 937 | 7 | 344 | 7 | 0.61 |
| aA | mg/dL | 8.1E-1 | 1.2E0 | 9.5E-1 | 1.3E0 | 4.9E-1 | 5.2E-1 | 2.0E-1 | 6.2E-1 | 4.2E0 | 2.3E0 | 2722 | 8 | 517 | 8 | 0.74 |

Figure 25.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.8E1 | 8.8E1 | 9.2E1 | 9.4E1 | 6.7E1 | 5.5E1 | 1.0E1 | 8.0E0 | 4.0E2 | 2.4E2 | 69 | 29 | 69 | 29 | 0.53 |
| Ad | ug/mL | 5.2E-2 | 4.9E-2 | 8.4E-2 | 8.9E-2 | 9.2E-2 | 1.0E-1 | 9.4E-4 | 3.9E-3 | 3.6E-1 | 3.5E-1 | 52 | 23 | 52 | 23 | 0.53 |
| Af | ng/mL | 1.5E0 | 9.2E-1 | 1.9E1 | 1.6E1 | 6.3E1 | 5.1E1 | 1.7E-3 | 1.7E-3 | 4.0E2 | 2.5E2 | 52 | 23 | 52 | 23 | 0.48 |
| Aj | ug/mL | 1.3E0 | 7.2E-1 | 2.7E0 | 1.8E0 | 2.7E0 | 2.2E0 | 4.1E-3 | 2.3E-3 | 6.1E0 | 5.8E0 | 52 | 23 | 52 | 23 | 0.43 |
| Al | mg/mL | 1.1E-4 | 8.3E-5 | 2.6E-4 | 3.3E-4 | 4.0E-4 | 4.4E-4 | 8.0E-6 | 7.6E-6 | 1.9E-3 | 1.5E-3 | 52 | 23 | 52 | 23 | 0.54 |
| An | U/mL | 7.8E1 | 4.8E1 | 2.3E2 | 3.0E2 | 3.7E2 | 6.5E2 | 7.7E-1 | 9.6E-1 | 2.0E3 | 3.0E3 | 52 | 23 | 52 | 23 | 0.43 |
| Ao | pg/mL | 1.1E2 | 9.7E1 | 2.1E2 | 1.7E2 | 5.2E2 | 1.7E2 | 6.7E0 | 9.9E0 | 3.8E3 | 6.5E2 | 52 | 23 | 52 | 23 | 0.52 |
| Ap | ng/mL | 3.4E1 | 2.5E1 | 4.5E1 | 3.8E1 | 4.6E1 | 2.7E1 | 8.4E-5 | 5.7E0 | 2.5E2 | 1.0E2 | 52 | 23 | 52 | 23 | 0.48 |
| Ar | ng/mL | 7.5E-1 | 2.2E0 | 4.4E0 | 5.2E0 | 9.6E0 | 7.7E0 | 6.7E-2 | 3.4E-3 | 5.1E1 | 2.8E1 | 52 | 23 | 52 | 23 | 0.59 |
| As | ng/mL | 7.7E-3 | 1.1E-2 | 1.4E-2 | 1.6E-2 | 2.2E-2 | 2.0E-2 | 1.7E-3 | 1.7E-3 | 1.3E-1 | 8.2E-2 | 52 | 23 | 52 | 23 | 0.57 |
| Aw | pg/mL | 1.7E1 | 1.8E1 | 1.7E1 | 1.6E1 | 5.5E0 | 5.7E0 | 9.3E0 | 2.9E-2 | 3.7E1 | 2.5E1 | 52 | 23 | 52 | 23 | 0.52 |
| Ax | ng/mL | 2.2E0 | 8.7E0 | 8.1E0 | 5.9E1 | 1.9E1 | 1.5E2 | 2.5E-2 | 1.2E-2 | 1.3E2 | 6.2E2 | 52 | 23 | 52 | 23 | 0.64 |
| Ba | ng/mL | 7.1E1 | 9.4E1 | 6.4E2 | 3.8E2 | 1.4E3 | 9.2E2 | 2.7E-1 | 6.3E0 | 7.5E3 | 4.4E3 | 52 | 23 | 52 | 23 | 0.49 |
| Bb | ng/mL | 4.2E0 | 5.0E0 | 5.7E0 | 6.6E0 | 5.4E0 | 6.3E0 | 4.1E-3 | 5.4E-1 | 2.0E1 | 1.9E1 | 52 | 23 | 52 | 23 | 0.53 |
| Bc | ng/mL | 3.4E1 | 5.1E1 | 1.2E2 | 6.7E1 | 2.3E2 | 5.8E1 | 2.2E-1 | 2.3E0 | 1.2E3 | 2.2E2 | 52 | 23 | 52 | 23 | 0.55 |
| Bg | ng/mL | 7.1E-2 | 1.2E-1 | 2.2E0 | 1.9E1 | 9.9E0 | 8.2E1 | 5.3E-4 | 2.6E-2 | 7.1E1 | 4.0E2 | 52 | 23 | 52 | 23 | 0.57 |
| Bn | ng/mL | 6.1E-1 | 5.6E-2 | 1.9E0 | 1.6E0 | 2.4E0 | 2.5E0 | 5.6E-2 | 5.6E-2 | 8.6E0 | 7.4E0 | 52 | 23 | 52 | 23 | 0.43 |
| Bo | ng/mL | 9.1E0 | 2.2E1 | 1.3E1 | 1.8E1 | 1.3E1 | 1.3E1 | 1.6E-2 | 1.6E-2 | 4.6E1 | 4.1E1 | 52 | 23 | 52 | 23 | 0.64 |
| Ch | uIU/mL | 1.2E0 | 6.1E-1 | 1.8E1 | 5.4E1 | 4.1E1 | 2.5E2 | 3.4E-3 | 1.2E-1 | 1.9E2 | 1.2E3 | 52 | 23 | 52 | 23 | 0.39 |
| Co | pg/mL | 3.6E1 | 5.6E1 | 4.1E2 | 1.2E2 | 2.3E3 | 1.9E2 | 1.5E-1 | 3.6E0 | 1.7E4 | 8.2E2 | 52 | 23 | 52 | 23 | 0.60 |
| Cp | ng/mL | 2.6E1 | 2.3E1 | 3.2E1 | 2.9E1 | 2.8E1 | 1.6E1 | 1.1E1 | 1.0E1 | 1.9E2 | 6.9E1 | 52 | 23 | 52 | 23 | 0.47 |
| Cq | ng/mL | 3.0E-2 | 4.6E-2 | 1.4E-1 | 9.2E-2 | 7.1E-1 | 1.1E-1 | 8.0E-4 | 7.8E-3 | 5.1E0 | 4.0E-1 | 52 | 23 | 52 | 23 | 0.63 |
| Cs | ng/mL | 8.2E1 | 3.0E2 | 2.6E2 | 1.4E3 | 5.4E2 | 3.7E3 | 1.3E0 | 1.3E0 | 3.0E3 | 1.8E4 | 52 | 23 | 52 | 23 | 0.63 |
| Ct | ng/mL | 4.1E-1 | 8.7E-2 | 4.3E1 | 2.8E1 | 1.2E2 | 9.6E1 | 1.1E-4 | 1.1E-4 | 6.2E2 | 4.6E2 | 52 | 23 | 52 | 23 | 0.41 |
| Cu | ng/mL | 2.6E-1 | 3.1E-1 | 4.1E-1 | 6.1E-1 | 6.4E-1 | 9.4E-1 | 9.0E-5 | 5.6E-2 | 4.5E0 | 4.5E0 | 52 | 23 | 52 | 23 | 0.57 |
| Cv | ng/mL | 6.2E0 | 1.2E1 | 2.3E1 | 3.1E1 | 4.8E1 | 6.5E1 | 2.6E-2 | 5.1E-2 | 3.1E2 | 2.9E2 | 52 | 23 | 52 | 23 | 0.53 |
| Cw | mIU/mL | 2.4E-2 | 3.4E-2 | 3.6E-2 | 3.7E-2 | 2.9E-2 | 2.7E-2 | 2.8E-3 | 4.1E-3 | 1.4E-1 | 1.0E-1 | 52 | 23 | 52 | 23 | 0.51 |
| Cx | ng/mL | 1.1E0 | 3.7E-2 | 6.5E1 | 7.6E1 | 1.1E2 | 1.3E2 | 9.3E-5 | 9.3E-5 | 3.9E2 | 3.9E2 | 52 | 23 | 52 | 23 | 0.45 |
| Db | ug/mL | 6.8E0 | 6.7E0 | 8.0E0 | 7.3E0 | 1.0E1 | 5.5E0 | 5.0E-1 | 1.0E0 | 5.9E1 | 2.1E1 | 52 | 23 | 52 | 23 | 0.56 |
| Dc | nmol/L | 2.4E-2 | 2.4E-2 | 8.0E-2 | 1.0E-1 | 2.3E-1 | 1.9E-1 | 3.0E-4 | 1.0E-3 | 1.6E0 | 7.7E-1 | 52 | 23 | 52 | 23 | 0.59 |
| Dd | ug/mL | 7.5E-2 | 1.4E-1 | 2.2E-1 | 1.8E-1 | 3.7E-1 | 2.1E-1 | 8.3E-5 | 3.3E-3 | 1.9E0 | 7.5E-1 | 52 | 23 | 52 | 23 | 0.52 |
| De | ng/mL | 3.4E-3 | 3.4E-3 | 1.0E-1 | 7.8E-2 | 1.4E-1 | 1.0E-1 | 3.4E-3 | 3.4E-3 | 6.2E-1 | 3.2E-1 | 52 | 23 | 52 | 23 | 0.47 |
| Dg | ng/mL | 3.7E1 | 3.8E1 | 5.1E1 | 4.5E1 | 4.8E1 | 3.7E1 | 1.0E-1 | 1.1E0 | 1.9E2 | 1.2E2 | 52 | 23 | 52 | 23 | 0.49 |
| Di | pg/mL | 1.7E0 | 1.4E0 | 2.2E0 | 2.3E0 | 2.1E0 | 2.0E0 | 1.8E-1 | 1.8E-1 | 7.8E0 | 6.0E0 | 52 | 23 | 52 | 23 | 0.52 |
| Dk | uIU/mL | 1.6E-2 | 1.7E-2 | 4.0E-2 | 5.3E-2 | 6.5E-2 | 8.1E-2 | 1.1E-4 | 5.8E-3 | 3.4E-1 | 3.3E-1 | 52 | 23 | 52 | 23 | 0.55 |
| Dl | ng/mL | 2.4E2 | 1.8E2 | 3.2E2 | 2.5E2 | 2.7E2 | 2.6E2 | 2.5E0 | 5.5E0 | 1.1E3 | 1.1E3 | 52 | 23 | 52 | 23 | 0.41 |
| Dp | ng/ml | 2.1E0 | 1.8E0 | 5.1E0 | 8.1E0 | 6.8E0 | 1.4E1 | 3.7E-3 | 3.7E-3 | 3.5E1 | 5.6E1 | 53 | 23 | 53 | 23 | 0.51 |
| Dr | pg/ml | 1.6E1 | 2.6E1 | 3.5E1 | 5.6E1 | 4.5E1 | 9.9E1 | 7.5E-1 | 7.5E-1 | 1.6E2 | 3.6E2 | 27 | 12 | 27 | 12 | 0.53 |
| Du | pg/ml | 1.2E0 | 1.2E0 | 1.6E3 | 2.2E3 | 5.5E3 | 5.8E3 | 1.2E0 | 1.2E0 | 2.6E4 | 2.0E4 | 23 | 11 | 23 | 11 | 0.56 |
| Ef | ng/ml | 1.6E-1 | 8.6E-2 | 7.7E-1 | 8.3E-1 | 1.5E0 | 2.1E0 | 5.7E-4 | 5.7E-4 | 8.6E0 | 9.4E0 | 52 | 24 | 52 | 24 | 0.48 |
| Wm | % | 4.9E-1 | 4.5E-1 | 2.1E0 | 1.1E2 | 4.3E0 | 3.0E2 | 8.5E-2 | 8.5E-2 | 2.7E1 | 1.0E3 | 55 | 25 | 55 | 25 | 0.53 |
| Ed | pg/ml | 5.2E-1 | 3.1E1 | 2.1E1 | 7.1E1 | 4.2E1 | 1.1E2 | 5.2E-1 | 5.2E-1 | 1.9E2 | 5.0E2 | 53 | 23 | 53 | 23 | 0.69 |
| Yf | ng/mL | 1.7E1 | 1.4E1 | 5.4E1 | 4.2E1 | 1.2E2 | 8.5E1 | 2.2E0 | 2.9E-1 | 5.9E2 | 2.9E2 | 23 | 11 | 23 | 11 | 0.43 |
| Tj | pg/mL | 3.7E-1 | 3.7E-1 | 5.1E1 | 2.4E1 | 3.2E2 | 6.5E1 | 3.6E-1 | 3.7E-1 | 2.3E3 | 3.1E2 | 53 | 24 | 53 | 24 | 0.61 |
| Po | pg/ml | 5.7E-1 | 2.6E0 | 8.7E0 | 1.3E1 | 2.3E1 | 2.6E1 | 2.6E-2 | 2.6E-2 | 1.8E2 | 1.4E2 | 78 | 33 | 78 | 33 | 0.58 |
| Ti | ug/mL | 5.3E0 | 5.2E0 | 5.9E0 | 5.1E0 | 4.7E0 | 4.3E0 | 1.9E-1 | 8.7E-3 | 1.7E1 | 1.4E1 | 40 | 15 | 40 | 15 | 0.46 |
| Em | ng/ml | 1.3E-2 | 2.9E-3 | 3.0E-2 | 3.6E-2 | 3.7E-2 | 6.0E-2 | 1.9E-16 | 8.4E-4 | 1.3E-1 | 1.9E-1 | 29 | 12 | 29 | 12 | 0.44 |

| Code | Units | Median | | Average | | Standard Deviation | | Minimum | | Maximum | | Number of Samples | | Number of Patients | | AUC |
|------|-------|--------|------|---------|------|------|------|---------|------|---------|------|--------|-----|--------|-----|-----|
| | | NonDis | Dis | NonDis | Dis | NonDis | Dis | NonDis | Dis | NonDis | Dis | NonDis | Dis | NonDis | Dis | |
| Et | ng/ml | 1.6E3 | 2.3E3 | 1.8E3 | 2.3E3 | 1.1E3 | 1.3E3 | 1.5E2 | 1.8E2 | 4.4E3 | 5.0E3 | 78 | 33 | 78 | 33 | 0.60 |
| Eq | pg/ml | 2.5E2 | 1.4E2 | 3.4E2 | 1.9E2 | 4.2E2 | 2.8E2 | 1.0E0 | 1.0E0 | 1.8E3 | 1.0E3 | 23 | 11 | 23 | 11 | 0.41 |
| Th | ug/mL | 1.4E0 | 6.2E-1 | 1.6E0 | 1.2E0 | 1.1E0 | 1.4E0 | 1.3E-1 | 2.6E-3 | 4.6E0 | 4.2E0 | 40 | 15 | 40 | 15 | 0.31 |
| Fa | ng/ml | 3.1E1 | 8.7E1 | 7.1E1 | 3.5E2 | 1.2E2 | 8.6E2 | 1.5E0 | 5.9E0 | 7.5E2 | 3.7E3 | 50 | 24 | 50 | 24 | 0.69 |
| Ez | ng/ml | 5.3E0 | 4.1E0 | 2.9E1 | 1.3E1 | 1.0E2 | 2.0E1 | 1.3E-2 | 1.3E-2 | 7.1E2 | 8.8E1 | 53 | 23 | 53 | 23 | 0.49 |
| Fb | ng/ml | 2.4E1 | 3.0E1 | 2.1E1 | 2.8E1 | 1.1E1 | 9.4E0 | 1.0E0 | 5.9E-1 | 4.3E1 | 4.1E1 | 50 | 24 | 50 | 24 | 0.68 |
| Ex | ng/ml | 6.1E-2 | 1.4E-1 | 1.5E-1 | 1.4E-1 | 2.8E-1 | 1.1E-1 | 1.7E-4 | 1.5E-4 | 1.5E0 | 3.3E-1 | 31 | 17 | 31 | 17 | 0.61 |
| Fc | pg/ml | 2.2E-1 | 2.2E-1 | 1.9E1 | 4.1E0 | 8.2E1 | 1.1E1 | 2.2E-1 | 2.2E-1 | 3.9E2 | 3.6E1 | 23 | 11 | 23 | 11 | 0.50 |
| Fd | pg/ml | 9.8E-1 | 1.4E1 | 8.7E2 | 1.0E3 | 1.9E3 | 2.7E3 | 9.8E-1 | 9.8E-1 | 8.2E3 | 9.2E3 | 23 | 11 | 23 | 11 | 0.52 |
| Fi | pg/ml | 2.5E-1 | 2.5E-1 | 8.5E1 | 6.4E0 | 3.8E2 | 1.8E1 | 2.5E-1 | 2.5E-1 | 1.8E3 | 6.1E1 | 23 | 11 | 23 | 11 | 0.52 |
| Fn | ng/ml | 2.1E-1 | 1.7E0 | 3.0E0 | 5.4E0 | 5.2E0 | 6.4E0 | 2.1E-1 | 2.1E-1 | 2.1E1 | 1.8E1 | 53 | 23 | 53 | 23 | 0.64 |
| Fp | ng/ml | 1.6E1 | 3.9E1 | 3.1E1 | 4.4E1 | 3.3E1 | 3.9E1 | 2.8E-1 | 3.0E-1 | 1.3E2 | 1.2E2 | 79 | 34 | 79 | 34 | 0.58 |
| Fr | ng/ml | 3.6E4 | 6.8E4 | 1.0E5 | 1.9E5 | 1.6E5 | 2.4E5 | 7.8E2 | 2.5E3 | 8.4E5 | 8.3E5 | 79 | 34 | 79 | 34 | 0.64 |
| Fw | pg/ml | 1.4E0 | 4.0E0 | 1.3E1 | 6.9E1 | 3.9E1 | 2.0E2 | 1.2E-1 | 1.2E-1 | 2.5E2 | 9.1E2 | 51 | 24 | 51 | 24 | 0.62 |
| Fy | ng/ml | 4.1E1 | 5.4E1 | 6.4E1 | 1.2E2 | 5.9E1 | 1.5E2 | 1.2E-1 | 2.7E0 | 2.0E2 | 5.3E2 | 52 | 23 | 52 | 23 | 0.56 |
| Gh | pg/ml | 1.2E1 | 3.9E0 | 9.3E1 | 1.1E1 | 2.7E2 | 1.4E1 | 2.9E-2 | 2.9E-2 | 1.2E3 | 4.6E1 | 22 | 11 | 22 | 11 | 0.44 |
| Gb | % | 4.2E1 | 3.2E1 | 4.5E1 | 3.8E1 | 3.1E1 | 3.8E1 | 4.5E0 | 4.0E0 | 1.0E2 | 1.4E2 | 23 | 11 | 23 | 11 | 0.41 |
| Gc | ng/ml | 7.7E1 | 1.3E2 | 2.0E2 | 1.5E2 | 2.8E2 | 1.3E2 | 6.9E0 | 9.7E0 | 1.2E3 | 4.7E2 | 29 | 12 | 29 | 12 | 0.54 |
| Gd | ng/ml | 2.9E1 | 3.1E1 | 3.5E1 | 2.8E1 | 1.9E1 | 1.7E1 | 8.0E0 | 3.7E0 | 8.1E1 | 5.8E1 | 26 | 12 | 26 | 12 | 0.41 |
| Gn | U/ml | 1.6E-1 | 2.6E-1 | 5.6E-1 | 1.6E0 | 8.3E-1 | 3.4E0 | 5.6E-3 | 5.6E-3 | 3.4E0 | 1.2E1 | 27 | 12 | 27 | 12 | 0.56 |
| Gl | pg/ml | 1.1E4 | 1.0E4 | 1.3E4 | 1.3E4 | 9.7E3 | 9.8E3 | 4.0E2 | 7.2E2 | 3.3E4 | 3.0E4 | 51 | 24 | 51 | 24 | 0.47 |
| Gp | U/ml | 1.2E0 | 5.6E-1 | 3.9E0 | 3.5E0 | 5.9E0 | 9.7E0 | 1.3E-3 | 1.5E-2 | 2.3E1 | 4.8E1 | 52 | 24 | 52 | 24 | 0.38 |
| Gz | ug/ml | 1.0E0 | 1.0E0 | 4.8E0 | 5.1E0 | 5.1E0 | 6.3E0 | 1.6E-1 | 8.9E-2 | 1.1E1 | 1.9E1 | 30 | 17 | 30 | 17 | 0.53 |
| Ha | ng/ml | 2.4E0 | 6.0E0 | 9.2E0 | 1.1E1 | 1.9E1 | 2.1E1 | 1.7E-2 | 1.7E-2 | 1.0E2 | 1.0E2 | 53 | 23 | 53 | 23 | 0.60 |
| Nm | pg/ml | 2.0E4 | 1.7E4 | 3.0E4 | 3.2E4 | 3.4E4 | 3.5E4 | 1.0E-9 | 1.0E-9 | 1.5E5 | 1.2E5 | 79 | 33 | 79 | 33 | 0.50 |
| Nn | pg/ml | 1.4E2 | 4.7E2 | 1.9E3 | 1.6E3 | 1.1E4 | 3.7E3 | 1.0E-9 | 1.0E-9 | 9.5E4 | 1.7E4 | 79 | 33 | 79 | 33 | 0.60 |
| No | pg/ml | 1.5E1 | 2.5E1 | 3.1E1 | 4.3E1 | 4.5E1 | 7.5E1 | 1.0E-9 | 3.3E-1 | 2.6E2 | 4.1E2 | 79 | 33 | 79 | 33 | 0.55 |
| Nq | pg/ml | 1.0E-9 | 4.0E0 | 1.7E1 | 2.3E1 | 7.8E1 | 5.5E1 | 1.0E-9 | 1.0E-9 | 6.7E2 | 2.4E2 | 79 | 33 | 79 | 33 | 0.60 |
| Nr | pg/ml | 1.6E0 | 5.9E0 | 2.2E1 | 2.7E1 | 9.1E1 | 6.3E1 | 1.0E-9 | 1.0E-9 | 7.2E2 | 3.1E2 | 79 | 33 | 79 | 33 | 0.57 |
| Ns | pg/ml | 1.0E-9 | 1.0E-9 | 1.6E1 | 1.1E-1 | 1.3E2 | 6.2E-1 | 1.0E-9 | 1.0E-9 | 1.1E3 | 3.6E0 | 79 | 33 | 79 | 33 | 0.49 |
| Nt | pg/ml | 1.5E2 | 1.5E2 | 1.7E2 | 1.6E2 | 1.2E2 | 8.7E1 | 1.5E1 | 2.3E1 | 8.8E2 | 4.3E2 | 79 | 33 | 79 | 33 | 0.48 |
| Nu | pg/ml | 2.8E1 | 4.3E1 | 8.0E1 | 7.9E1 | 1.1E2 | 9.1E1 | 1.0E-9 | 1.0E-9 | 6.3E2 | 3.4E2 | 79 | 33 | 79 | 33 | 0.52 |
| Lu | pg/ml | 1.0E4 | 1.0E4 | 1.8E4 | 1.0E4 | 6.3E4 | 6.9E3 | 7.1E2 | 1.4E3 | 5.6E5 | 3.6E4 | 79 | 34 | 79 | 34 | 0.50 |
| Lv | pg/ml | 1.4E0 | 1.0E-9 | 1.7E1 | 3.0E1 | 2.5E1 | 5.8E1 | 1.0E-9 | 1.0E-9 | 1.2E2 | 2.6E2 | 79 | 34 | 79 | 34 | 0.51 |
| Lw | pg/ml | 1.0E-9 | 1.0E-9 | 2.5E-1 | 1.0E-9 | 1.7E0 | 0.0E0 | 1.0E-9 | 1.0E-9 | 1.4E1 | 1.0E-9 | 79 | 34 | 79 | 34 | 0.49 |
| Lx | pg/ml | 1.0E-9 | 1.3E2 | 1.7E2 | 3.3E2 | 5.5E2 | 8.0E2 | 1.0E-9 | 1.0E-9 | 4.4E3 | 4.5E3 | 79 | 34 | 79 | 34 | 0.65 |
| Ly | pg/ml | 1.0E-9 | 1.0E-9 | 1.6E1 | 1.4E1 | 2.4E1 | 1.9E1 | 1.0E-9 | 1.0E-9 | 1.2E2 | 7.0E1 | 79 | 34 | 79 | 34 | 0.52 |
| Lz | pg/ml | 1.0E-9 | 1.0E-9 | 2.2E0 | 3.9E0 | 1.5E1 | 1.3E1 | 1.0E-9 | 1.0E-9 | 1.3E2 | 5.6E1 | 79 | 34 | 79 | 34 | 0.52 |
| Ma | pg/ml | 2.6E2 | 5.2E2 | 1.2E3 | 3.6E3 | 2.3E3 | 1.0E4 | 1.0E-9 | 1.7E1 | 1.5E4 | 5.2E4 | 79 | 34 | 79 | 34 | 0.51 |
| Mb | pg/ml | 3.2E1 | 3.0E1 | 3.8E1 | 3.7E1 | 1.9E1 | 1.7E1 | 1.4E1 | 1.4E1 | 1.1E2 | 6.4E1 | 79 | 34 | 79 | 34 | 0.47 |
| Mc | pg/ml | 1.0E-9 | 1.0E-9 | 2.1E-2 | 1.0E-9 | 1.9E-1 | 0.0E0 | 1.0E-9 | 1.0E-9 | 1.7E0 | 1.0E-9 | 79 | 34 | 79 | 34 | 0.49 |
| Md | pg/ml | 1.0E-9 | 1.0E-9 | 6.8E-1 | 1.6E-1 | 2.7E0 | 9.4E-1 | 1.0E-9 | 1.0E-9 | 1.6E1 | 5.5E0 | 79 | 34 | 79 | 34 | 0.47 |
| Me | pg/ml | 2.4E1 | 2.4E1 | 2.6E1 | 2.3E1 | 2.3E1 | 1.6E1 | 1.0E-9 | 2.4E-1 | 1.6E2 | 6.0E1 | 79 | 34 | 79 | 34 | 0.47 |
| Mf | pg/ml | 1.0E-9 | 1.0E-9 | 1.0E0 | 5.4E-1 | 4.3E0 | 1.2E0 | 1.0E-9 | 1.0E-9 | 3.7E1 | 5.0E0 | 79 | 34 | 79 | 34 | 0.55 |
| Mg | pg/ml | 1.6E0 | 1.3E0 | 7.4E0 | 7.0E0 | 1.2E1 | 1.1E1 | 1.0E-9 | 1.0E-9 | 5.9E1 | 3.9E1 | 79 | 34 | 79 | 34 | 0.50 |
| Mh | pg/ml | 1.0E-9 | 1.0E-9 | 7.6E-1 | 2.2E0 | 3.5E0 | 7.5E0 | 1.0E-9 | 1.0E-9 | 2.9E1 | 4.2E1 | 79 | 34 | 79 | 34 | 0.60 |
| Mi | pg/ml | 1.0E-9 | 1.0E-9 | 9.4E-1 | 1.0E-9 | 8.0E0 | 0.0E0 | 1.0E-9 | 1.0E-9 | 7.1E1 | 1.0E-9 | 79 | 34 | 79 | 34 | 0.49 |
| Mj | pg/ml | 1.0E-9 | 1.0E-9 | 3.2E0 | 8.2E0 | 1.1E1 | 2.2E1 | 1.0E-9 | 1.0E-9 | 8.1E1 | 1.1E2 | 79 | 34 | 79 | 34 | 0.57 |
| Mk | pg/ml | 4.3E0 | 5.3E0 | 2.4E1 | 8.5E0 | 1.5E2 | 1.1E1 | 1.0E-9 | 1.0E-9 | 1.3E3 | 3.8E1 | 79 | 34 | 79 | 34 | 0.51 |
| Ml | pg/ml | 1.0E-9 | 1.0E-9 | 2.7E0 | 1.5E1 | 8.9E0 | 6.1E1 | 1.0E-9 | 1.0E-9 | 6.4E1 | 3.4E2 | 79 | 34 | 79 | 34 | 0.50 |
| Mm | pg/ml | 6.3E2 | 7.8E2 | 1.0E3 | 1.1E3 | 1.2E3 | 1.3E3 | 1.0E-9 | 1.9E1 | 6.3E3 | 6.1E3 | 79 | 34 | 79 | 34 | 0.52 |
| Mn | pg/ml | 5.6E0 | 7.0E0 | 9.3E0 | 1.3E1 | 1.2E1 | 2.2E1 | 1.0E-9 | 1.0E-9 | 6.8E1 | 1.3E2 | 79 | 34 | 79 | 34 | 0.56 |
| Mp | pg/ml | 1.0E-9 | 1.0E-9 | 1.2E1 | 1.3E1 | 7.1E1 | 3.0E1 | 1.0E-9 | 1.0E-9 | 6.3E2 | 1.5E2 | 79 | 34 | 79 | 34 | 0.55 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Mq | pg/ml | 1.0E-9 | 1.0E-9 | 1.8E0 | 5.7E0 | 7.3E0 | 1.6E1 | 1.0E-9 | 1.0E-9 | 4.6E1 | 8.6E1 | 79 | 34 | 79 | 34 | 0.56 |
| Mr | pg/ml | 1.0E-9 | 1.0E-9 | 1.5E1 | 5.1E1 | 5.3E1 | 2.6E2 | 1.0E-9 | 1.0E-9 | 3.9E2 | 1.5E3 | 79 | 34 | 79 | 34 | 0.50 |
| Ms | pg/ml | 4.1E2 | 1.8E2 | 5.8E2 | 3.8E2 | 5.9E2 | 4.8E2 | 1.0E-9 | 1.0E-9 | 3.3E3 | 2.2E3 | 79 | 34 | 79 | 34 | 0.38 |
| Mt | pg/ml | 8.1E-1 | 1.6E0 | 6.0E0 | 9.2E0 | 2.0E1 | 2.4E1 | 1.0E-9 | 1.0E-9 | 1.4E2 | 1.0E2 | 79 | 34 | 79 | 34 | 0.59 |
| Mu | pg/ml | 1.0E-9 | 1.0E-9 | 3.4E0 | 6.5E-1 | 2.6E1 | 1.7E0 | 1.0E-9 | 1.0E-9 | 2.3E2 | 7.3E0 | 79 | 34 | 79 | 34 | 0.52 |
| Mv | pg/ml | 1.0E-9 | 1.0E-9 | 4.0E1 | 1.2E2 | 1.5E2 | 4.6E2 | 1.0E-9 | 1.0E-9 | 8.4E2 | 2.5E3 | 79 | 34 | 79 | 34 | 0.56 |
| Mw | pg/ml | 2.2E1 | 7.3E1 | 1.8E2 | 3.1E2 | 4.9E2 | 1.0E3 | 1.0E-9 | 1.0E-9 | 2.9E3 | 5.9E3 | 79 | 34 | 79 | 34 | 0.62 |
| Mx | pg/ml | 1.0E-9 | 1.0E-9 | 7.0E-1 | 3.0E-1 | 3.6E0 | 5.7E-1 | 1.0E-9 | 1.0E-9 | 3.2E1 | 2.1E0 | 79 | 34 | 79 | 34 | 0.54 |
| My | pg/ml | 1.0E-9 | 1.0E-9 | 1.3E2 | 2.6E2 | 5.1E2 | 8.5E2 | 1.0E-9 | 1.0E-9 | 3.6E3 | 4.6E3 | 79 | 34 | 79 | 34 | 0.54 |
| Mz | pg/ml | 1.5E1 | 1.7E1 | 2.6E1 | 3.4E1 | 4.5E1 | 4.0E1 | 1.0E-9 | 1.0E-9 | 3.0E2 | 1.5E2 | 79 | 34 | 79 | 34 | 0.58 |
| Na | pg/ml | 1.0E-9 | 1.0E-9 | 6.0E-1 | 8.4E-1 | 1.8E0 | 3.0E0 | 1.0E-9 | 1.0E-9 | 9.6E0 | 1.6E1 | 79 | 34 | 79 | 34 | 0.49 |
| Nb | pg/ml | 2.0E0 | 3.0E0 | 2.7E0 | 3.7E0 | 3.3E0 | 3.3E0 | 1.0E-9 | 1.0E-9 | 2.3E1 | 1.7E1 | 79 | 34 | 79 | 34 | 0.63 |
| Nc | pg/ml | 1.6E2 | 3.0E2 | 3.6E2 | 4.9E2 | 4.4E2 | 4.9E2 | 1.0E-9 | 1.0E-9 | 1.8E3 | 2.1E3 | 79 | 34 | 79 | 34 | 0.59 |
| Nd | pg/ml | 7.0E0 | 6.5E0 | 3.0E1 | 1.6E1 | 1.4E2 | 2.0E1 | 1.0E-9 | 1.0E-9 | 1.2E3 | 9.4E1 | 79 | 34 | 79 | 34 | 0.51 |
| Nc | pg/ml | 2.8E2 | 3.7E2 | 3.8E2 | 4.5E2 | 3.4E2 | 3.9E2 | 1.0E-9 | 1.0E-9 | 1.5E3 | 1.7E3 | 79 | 34 | 79 | 34 | 0.56 |
| Nf | pg/ml | 1.0E-9 | 1.0E-9 | 1.4E0 | 2.6E0 | 5.0E0 | 8.9E0 | 1.0E-9 | 1.0E-9 | 3.5E1 | 5.1E1 | 79 | 34 | 79 | 34 | 0.56 |
| Ng | pg/ml | 1.7E1 | 7.2E0 | 9.1E1 | 1.0E2 | 1.4E2 | 2.4E2 | 1.0E-9 | 1.0E-9 | 6.2E2 | 1.2E3 | 79 | 34 | 79 | 34 | 0.46 |
| Nh | pg/ml | 4.2E1 | 5.4E1 | 5.8E1 | 6.0E1 | 5.4E1 | 4.7E1 | 1.0E-9 | 1.0E-9 | 3.4E2 | 2.2E2 | 79 | 34 | 79 | 34 | 0.55 |
| Ni | pg/ml | 2.2E1 | 1.2E1 | 9.4E1 | 9.6E1 | 1.2E2 | 1.5E2 | 1.0E-9 | 1.0E-9 | 5.7E2 | 5.7E2 | 79 | 34 | 79 | 34 | 0.48 |
| Nj | pg/ml | 3.5E0 | 3.0E0 | 7.3E0 | 6.3E0 | 8.3E0 | 7.0E0 | 1.0E-9 | 1.0E-9 | 3.6E1 | 2.9E1 | 79 | 34 | 79 | 34 | 0.47 |
| Nk | pg/ml | 1.7E1 | 2.2E1 | 3.0E1 | 3.6E1 | 3.5E1 | 4.8E1 | 1.0E-9 | 1.0E-9 | 1.7E2 | 2.0E2 | 79 | 34 | 79 | 34 | 0.53 |
| Nl | pg/ml | 2.4E1 | 4.0E1 | 3.8E1 | 4.7E1 | 3.8E1 | 3.8E1 | 1.0E-9 | 1.0E-9 | 1.9E2 | 1.4E2 | 79 | 34 | 79 | 34 | 0.59 |
| Hl | pg/ml | 1.3E1 | 2.9E1 | 2.4E1 | 5.7E1 | 2.8E1 | 7.7E1 | 1.0E-9 | 1.0E-9 | 9.7E1 | 2.3E2 | 23 | 11 | 23 | 11 | 0.61 |
| Ho | pg/ml | 1.7E1 | 2.7E1 | 2.4E1 | 2.5E1 | 1.8E1 | 8.9E0 | 8.1E0 | 7.6E0 | 8.7E1 | 4.0E1 | 23 | 11 | 23 | 11 | 0.62 |
| Hp | ng/ml | 1.3E0 | 3.3E0 | 1.9E2 | 8.3E1 | 3.7E2 | 2.7E2 | 4.2E-1 | 8.8E-1 | 8.9E2 | 8.9E2 | 23 | 11 | 23 | 11 | 0.61 |
| Tz | pg/ml | 7.2E3 | 6.2E3 | 3.2E4 | 3.1E4 | 1.4E5 | 8.2E4 | 1.0E-9 | 1.0E3 | 1.0E6 | 3.7E5 | 53 | 23 | 53 | 23 | 0.49 |
| Ua | pg/ml | 3.6E3 | 4.2E3 | 1.3E4 | 1.6E4 | 2.3E4 | 3.9E4 | 1.0E-9 | 8.4E2 | 1.2E5 | 1.8E5 | 53 | 23 | 53 | 23 | 0.51 |
| Ub | pg/ml | 6.9E2 | 5.6E2 | 9.3E2 | 8.7E2 | 1.0E3 | 9.3E2 | 1.0E-9 | 6.6E1 | 6.4E3 | 4.1E3 | 53 | 23 | 53 | 23 | 0.47 |
| Ue | pg/ml | 2.9E1 | 2.6E1 | 3.7E1 | 2.9E1 | 2.3E1 | 1.7E1 | 9.8E-2 | 8.8E0 | 1.0E2 | 7.4E1 | 53 | 23 | 53 | 23 | 0.41 |
| Uc | pg/ml | 9.6E2 | 9.6E2 | 1.6E3 | 2.1E3 | 2.0E3 | 2.8E3 | 1.0E-9 | 1.1E2 | 9.2E3 | 9.4E3 | 53 | 23 | 53 | 23 | 0.54 |
| Ud | pg/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 53 | 23 | 53 | 23 | 0.50 |
| Hq | pg/ml | 9.9E-1 | 1.3E0 | 3.7E2 | 1.2E1 | 3.2E3 | 4.1E1 | 1.0E-9 | 1.0E-9 | 2.8E4 | 2.3E2 | 78 | 33 | 78 | 33 | 0.57 |
| Hr | pg/ml | 1.0E2 | 1.4E2 | 4.8E2 | 8.2E2 | 9.3E2 | 1.5E3 | 1.0E-9 | 2.2E1 | 5.0E3 | 5.5E3 | 78 | 33 | 78 | 33 | 0.57 |
| Hu | pg/ml | 2.2E1 | 8.9E0 | 4.1E3 | 1.0E3 | 3.0E4 | 2.4E3 | 1.0E-9 | 1.0E-9 | 2.6E5 | 8.8E3 | 78 | 33 | 78 | 33 | 0.46 |
| Hv | pg/ml | 2.0E0 | 1.2E0 | 3.5E0 | 2.0E0 | 9.5E0 | 2.4E0 | 1.0E-9 | 1.0E-9 | 8.4E1 | 1.1E1 | 78 | 33 | 78 | 33 | 0.41 |
| Hw | pg/ml | 5.4E0 | 6.5E0 | 1.2E1 | 1.3E1 | 2.0E1 | 1.8E1 | 1.0E-9 | 1.0E-9 | 1.5E2 | 1.0E2 | 78 | 33 | 78 | 33 | 0.54 |
| Hx | pg/ml | 1.0E1 | 1.3E1 | 2.6E1 | 2.8E1 | 5.9E1 | 3.4E1 | 1.0E-9 | 1.0E-9 | 3.7E2 | 1.6E2 | 78 | 33 | 78 | 33 | 0.60 |
| Ib | ng/ml | 3.3E-2 | 2.7E-2 | 9.8E-1 | 2.3E0 | 3.4E0 | 1.1E1 | 1.0E-9 | 1.0E-9 | 1.7E1 | 5.2E1 | 49 | 23 | 49 | 23 | 0.43 |
| Ic | U/ml | 2.2E2 | 2.2E2 | 3.4E2 | 1.7E2 | 6.3E2 | 1.3E2 | 1.1E1 | 1.8E1 | 4.2E3 | 4.2E2 | 49 | 23 | 49 | 23 | 0.40 |
| Id | U/ml | 7.6E-1 | 8.9E-1 | 1.2E0 | 1.9E0 | 1.2E0 | 3.2E0 | 1.0E-9 | 5.6E-2 | 5.0E0 | 1.5E1 | 49 | 23 | 49 | 23 | 0.55 |
| Tt | pg/ml | 1.6E2 | 1.7E2 | 1.7E2 | 1.8E2 | 5.4E1 | 5.6E1 | 7.3E1 | 1.0E2 | 3.6E2 | 2.8E2 | 51 | 22 | 51 | 22 | 0.53 |
| To | pg/ml | 1.5E0 | 6.7E-1 | 1.8E0 | 1.3E0 | 1.9E0 | 1.5E0 | 1.0E-9 | 1.0E-9 | 6.5E0 | 5.1E0 | 52 | 23 | 52 | 23 | 0.44 |
| Tr | pg/ml | 2.6E0 | 4.7E0 | 1.0E1 | 1.0E1 | 4.3E1 | 1.4E1 | 1.0E-9 | 4.5E-1 | 3.1E2 | 5.4E1 | 52 | 22 | 52 | 22 | 0.59 |
| Tn | pg/ml | 2.4E1 | 4.3E1 | 7.9E1 | 1.5E2 | 2.5E2 | 4.0E2 | 2.4E0 | 1.1E1 | 1.8E3 | 2.0E3 | 52 | 23 | 52 | 23 | 0.63 |
| Tv | ng/ml | 1.1E1 | 1.2E1 | 1.7E1 | 5.2E1 | 2.2E1 | 1.6E2 | 1.0E-9 | 1.0E-9 | 1.3E2 | 7.9E2 | 52 | 23 | 52 | 23 | 0.52 |
| Ih | ng/ml | 9.3E1 | 1.3E2 | 2.2E2 | 2.4E2 | 3.5E2 | 2.8E2 | 1.0E-9 | 4.7E0 | 1.7E3 | 1.1E3 | 78 | 34 | 78 | 34 | 0.57 |
| Ii | ng/ml | 1.0E2 | 1.1E2 | 1.7E2 | 2.3E2 | 2.0E2 | 3.0E2 | 2.9E0 | 5.1E0 | 1.2E3 | 1.2E3 | 78 | 34 | 78 | 34 | 0.55 |
| Ij | ng/ml | 7.8E1 | 1.2E2 | 1.5E2 | 1.9E2 | 3.0E2 | 1.9E2 | 4.7E0 | 1.9E1 | 2.0E3 | 8.4E2 | 78 | 33 | 78 | 33 | 0.64 |
| Ik | ng/ml | 1.6E2 | 1.1E2 | 6.5E2 | 4.0E2 | 1.3E3 | 5.1E2 | 1.3E0 | 2.3E0 | 9.7E3 | 1.5E3 | 78 | 34 | 78 | 34 | 0.48 |
| Il | ng/ml | 4.1E2 | 5.5E2 | 1.1E3 | 1.9E3 | 2.3E3 | 3.5E3 | 1.0E-9 | 1.0E-9 | 1.2E4 | 1.2E4 | 78 | 34 | 78 | 34 | 0.55 |
| Im | ng/ml | 2.0E2 | 2.6E2 | 4.9E2 | 6.4E2 | 9.1E2 | 1.1E3 | 2.7E1 | 4.5E1 | 5.6E3 | 5.8E3 | 78 | 34 | 78 | 34 | 0.61 |
| In | ng/ml | 5.0E0 | 3.4E0 | 1.3E1 | 9.2E0 | 2.9E1 | 1.6E1 | 1.0E-9 | 1.0E-9 | 2.4E2 | 8.4E1 | 78 | 34 | 78 | 34 | 0.44 |
| Hb | ng/ml | 2.4E1 | 4.1E1 | 3.2E1 | 5.9E1 | 2.7E1 | 6.2E1 | 3.3E0 | 1.7E0 | 1.2E2 | 2.1E2 | 52 | 24 | 52 | 24 | 0.58 |

| Code | Units | Median | | Average | | Standard Deviation | | Minimum | | Maximum | | Number of Samples | | Number of Patients | | AUC |
|------|-------|--------|--------|---------|--------|--------|--------|--------|--------|--------|--------|--------|--------|--------|--------|------|
| | | NonDis | Dis | NonDis | Dis | NonDis | Dis | NonDis | Dis | NonDis | Dis | NonDis | Dis | NonDis | Dis | |
| Hc | pg/ml | 6.7E2 | 6.2E2 | 3.5E3 | 3.5E3 | 1.4E4 | 1.1E4 | 1.0E-9 | 1.0E-9 | 1.0E5 | 5.0E4 | 52 | 24 | 52 | 24 | 0.47 |
| Hf | ng/ml | 1.9E2 | 1.6E2 | 4.4E2 | 4.2E2 | 5.7E2 | 6.8E2 | 1.0E-9 | 1.0E-9 | 2.5E3 | 3.2E3 | 52 | 24 | 52 | 24 | 0.46 |
| Io | ng/ml | 7.7E3 | 7.9E3 | 1.5E4 | 1.5E4 | 2.7E4 | 1.8E4 | 1.0E-9 | 1.1E3 | 2.0E5 | 8.4E4 | 78 | 34 | 78 | 34 | 0.51 |
| Ip | ng/ml | 1.1E1 | 3.0E1 | 1.9E1 | 2.8E1 | 2.0E1 | 2.2E1 | 1.0E-9 | 1.8E-1 | 6.4E1 | 8.8E1 | 78 | 34 | 78 | 34 | 0.62 |
| Iq | ug/ml | 3.1E-2 | 1.7E-1 | 7.7E-1 | 9.4E-1 | 2.9E0 | 3.1E0 | 1.0E-9 | 1.0E-9 | 1.8E1 | 1.8E1 | 78 | 34 | 78 | 34 | 0.65 |
| Ir | ug/ml | 3.1E-1 | 8.9E-1 | 3.2E0 | 1.8E0 | 1.8E1 | 2.4E0 | 1.0E-9 | 5.0E-2 | 1.6E2 | 1.1E1 | 78 | 34 | 78 | 34 | 0.67 |
| Is | ng/ml | 2.1E0 | 4.6E0 | 6.4E0 | 1.3E1 | 1.4E1 | 2.1E1 | 1.0E-9 | 3.7E-1 | 8.8E1 | 1.1E2 | 78 | 34 | 78 | 34 | 0.72 |
| It | ng/ml | 1.8E0 | 2.7E0 | 2.6E1 | 1.4E1 | 1.3E2 | 3.7E1 | 1.0E-9 | 1.0E-9 | 8.3E2 | 1.8E2 | 78 | 34 | 78 | 34 | 0.55 |
| Iu | ng/ml | 1.0E2 | 2.4E2 | 9.0E2 | 1.3E3 | 3.3E3 | 4.2E3 | 1.0E-9 | 1.0E-9 | 2.4E4 | 2.4E4 | 78 | 34 | 78 | 34 | 0.58 |
| Iv | ng/ml | 1.8E1 | 3.2E1 | 4.9E1 | 4.3E1 | 1.1E2 | 4.2E1 | 1.0E-9 | 1.0E-9 | 7.7E2 | 1.6E2 | 78 | 34 | 78 | 34 | 0.58 |
| Iz | ng/ml | 1.5E2 | 1.1E2 | 3.4E2 | 3.0E2 | 4.3E2 | 4.1E2 | 8.3E0 | 8.8E0 | 2.3E3 | 1.7E3 | 52 | 24 | 52 | 24 | 0.49 |
| Yg | pg/ml | 2.6E2 | 3.8E2 | 1.2E3 | 8.2E2 | 2.6E3 | 8.1E2 | 2.6E1 | 1.5E1 | 1.2E4 | 2.3E3 | 22 | 11 | 22 | 11 | 0.55 |
| Yh | pg/ml | 2.1E2 | 6.9E2 | 3.9E2 | 5.9E2 | 5.0E2 | 4.5E2 | 1.0E-9 | 1.0E-9 | 2.1E3 | 1.4E3 | 22 | 11 | 22 | 11 | 0.67 |
| Yi | pg/ml | 2.7E2 | 7.5E2 | 3.7E2 | 3.1E3 | 4.2E2 | 7.6E3 | 1.0E-9 | 2.7E1 | 1.8E3 | 2.6E4 | 22 | 11 | 22 | 11 | 0.77 |
| Yk | U/ml | 1.0E-9 | 1.0E-9 | 3.0E-2 | 1.1E-1 | 8.7E-2 | 3.7E-1 | 1.0E-9 | 1.0E-9 | 3.6E-1 | 1.2E0 | 22 | 11 | 22 | 11 | 0.48 |
| Yj | pg/ml | 1.2E2 | 2.9E2 | 3.3E2 | 3.7E2 | 8.6E2 | 4.2E2 | 1.0E-9 | 1.7E1 | 4.1E3 | 1.5E3 | 22 | 11 | 22 | 11 | 0.66 |
| Yd | ng/ml | 2.9E-1 | 2.0E-1 | 3.2E-1 | 5.3E-1 | 2.6E-1 | 8.1E-1 | 1.6E-2 | 7.9E-3 | 9.6E-1 | 2.3E0 | 23 | 11 | 23 | 11 | 0.45 |
| Wb | pg/ml | 3.3E4 | 3.2E4 | 4.1E4 | 4.5E4 | 3.1E4 | 3.0E4 | 1.0E4 | 1.4E4 | 1.6E5 | 1.1E5 | 22 | 11 | 22 | 11 | 0.53 |
| Vz | pg/ml | 1.9E0 | 3.5E0 | 2.3E0 | 5.2E0 | 2.0E0 | 5.9E0 | 1.0E-9 | 7.6E-2 | 7.6E0 | 2.1E1 | 22 | 11 | 22 | 11 | 0.70 |
| Si | ng/ml | 1.1E0 | 6.9E-1 | 2.6E0 | 1.2E0 | 3.5E0 | 1.3E0 | 7.8E-2 | 2.5E-2 | 1.3E1 | 4.6E0 | 23 | 11 | 23 | 11 | 0.39 |
| Sf | mIU/mL | 1.4E1 | 1.2E1 | 2.1E1 | 2.1E1 | 3.1E1 | 2.5E1 | 1.3E0 | 2.0E-1 | 1.5E2 | 8.3E1 | 23 | 11 | 23 | 11 | 0.49 |
| Sh | mIU/mL | 1.4E1 | 6.6E0 | 2.7E1 | 1.4E1 | 3.9E1 | 1.8E1 | 7.9E-1 | 9.0E-2 | 1.8E2 | 5.8E1 | 23 | 11 | 23 | 11 | 0.35 |
| Sj | ng/ml | 3.8E-1 | 4.4E-1 | 4.0E-1 | 4.6E-1 | 1.2E-1 | 1.1E-1 | 1.1E-1 | 3.4E-1 | 6.2E-1 | 7.2E-1 | 23 | 11 | 23 | 11 | 0.60 |
| Rc | pg/ml | 5.3E3 | 5.3E3 | 7.9E3 | 6.0E3 | 6.1E3 | 3.7E3 | 1.1E3 | 6.7E2 | 2.3E4 | 1.4E4 | 53 | 23 | 53 | 23 | 0.44 |
| Rb | pg/ml | 1.2E0 | 3.3E-1 | 3.5E0 | 1.9E0 | 4.7E0 | 2.8E0 | 1.0E-9 | 1.0E-9 | 2.0E1 | 9.4E0 | 53 | 23 | 53 | 23 | 0.43 |
| Zq | 2.6ng/ml | 3.5E2 | 3.0E2 | 3.9E2 | 3.2E2 | 3.0E2 | 2.7E2 | 7.4E1 | 3.7E1 | 9.7E2 | 9.7E2 | 23 | 11 | 23 | 11 | 0.43 |
| Zw | 2.5ng/ml | 6.2E0 | 4.7E0 | 9.8E0 | 1.4E1 | 1.2E1 | 1.8E1 | 2.4E-1 | 1.7E0 | 4.6E1 | 6.3E1 | 23 | 11 | 23 | 11 | 0.62 |
| Zx | 2.3mU/ml | 1.4E-1 | 9.4E-2 | 4.0E-1 | 3.2E-1 | 7.1E-1 | 5.4E-1 | 5.6E-2 | 5.0E-2 | 3.0E0 | 1.9E0 | 23 | 11 | 23 | 11 | 0.40 |
| Pz | ng/ml | 3.3E3 | 4.8E3 | 6.0E3 | 3.6E4 | 7.3E3 | 1.8E5 | 3.6E1 | 6.2E2 | 4.8E4 | 1.0E6 | 77 | 33 | 77 | 33 | 0.55 |
| Qa | ng/ml | 3.8E3 | 7.5E3 | 6.3E3 | 1.1E4 | 6.7E3 | 9.4E3 | 3.4E2 | 6.8E2 | 3.2E4 | 3.6E4 | 77 | 33 | 77 | 33 | 0.66 |
| Qb | ng/ml | 1.2E2 | 2.0E2 | 1.7E2 | 3.8E2 | 1.6E2 | 7.1E2 | 6.7E0 | 1.8E1 | 7.3E2 | 4.1E3 | 77 | 33 | 77 | 33 | 0.63 |
| Qc | ng/ml | 2.3E2 | 4.6E2 | 4.0E2 | 6.9E2 | 4.3E2 | 8.9E2 | 1.9E0 | 1.3E1 | 2.1E3 | 4.3E3 | 77 | 33 | 77 | 33 | 0.61 |
| Qd | ng/ml | 1.1E4 | 1.5E4 | 1.8E4 | 3.1E4 | 2.3E4 | 3.8E4 | 9.8E2 | 1.7E3 | 1.3E5 | 1.5E5 | 77 | 33 | 77 | 33 | 0.61 |
| Qe | ng/ml | 1.3E3 | 1.8E3 | 1.6E3 | 2.6E3 | 1.5E3 | 2.5E3 | 6.8E1 | 1.8E2 | 7.4E3 | 1.3E4 | 77 | 33 | 77 | 33 | 0.64 |
| Jd | ng/ml | 1.4E0 | 2.1E0 | 3.7E0 | 2.3E0 | 1.0E1 | 2.9E0 | 1.0E-9 | 1.0E-9 | 7.3E1 | 1.1E1 | 53 | 23 | 53 | 23 | 0.48 |
| Je | ng/ml | 2.9E-1 | 1.0E-9 | 1.6E0 | 7.4E-1 | 2.4E0 | 1.3E0 | 1.0E-9 | 1.0E-9 | 1.1E1 | 4.3E0 | 53 | 23 | 53 | 23 | 0.39 |
| Jf | ng/ml | 1.0E-9 | 1.0E-9 | 1.2E0 | 1.3E0 | 2.3E0 | 2.5E0 | 1.0E-9 | 1.0E-9 | 9.6E0 | 9.1E0 | 53 | 23 | 53 | 23 | 0.48 |
| Jg | ng/ml | 6.4E2 | 6.7E2 | 8.6E2 | 9.8E2 | 7.7E2 | 1.1E3 | 1.1E1 | 5.9E1 | 3.4E3 | 5.3E3 | 78 | 33 | 78 | 33 | 0.52 |
| Jh | ng/ml | 5.0E0 | 3.0E0 | 1.7E1 | 2.7E1 | 4.2E1 | 8.4E1 | 1.0E-9 | 1.0E-9 | 2.6E2 | 4.7E2 | 78 | 33 | 78 | 33 | 0.51 |
| Ji | ng/ml | 7.0E1 | 8.4E1 | 9.7E1 | 1.3E2 | 8.1E1 | 1.2E2 | 8.3E0 | 8.9E0 | 3.6E2 | 5.4E2 | 78 | 33 | 78 | 33 | 0.57 |
| Sr | pg/mL | 4.9E2 | 9.2E2 | 8.4E2 | 1.2E3 | 1.0E3 | 1.2E3 | 1.0E-9 | 1.0E-9 | 4.8E3 | 5.1E3 | 50 | 23 | 50 | 23 | 0.59 |
| Ss | pg/mL | 1.4E5 | 5.5E4 | 1.7E5 | 1.0E5 | 2.1E5 | 8.9E4 | 2.7E3 | 1.1E4 | 1.3E6 | 3.0E5 | 50 | 23 | 50 | 23 | 0.42 |
| St | pg/mL | 2.4E7 | 6.5E7 | 6.2E7 | 9.0E7 | 1.6E8 | 1.3E8 | 1.1E6 | 2.8E6 | 1.2E9 | 5.4E8 | 53 | 23 | 53 | 23 | 0.62 |
| Wc | ng/ml | 2.9E-2 | 1.0E-9 | 6.3E-2 | 2.0E-2 | 8.4E-2 | 3.9E-2 | 1.0E-9 | 1.0E-9 | 2.8E-1 | 1.2E-1 | 23 | 11 | 23 | 11 | 0.35 |
| Wd | ng/ml | 1.1E1 | 8.4E0 | 5.7E1 | 1.8E1 | 1.6E2 | 2.2E1 | 5.0E-1 | 3.5E0 | 7.9E2 | 6.7E1 | 23 | 11 | 23 | 11 | 0.50 |
| We | ng/ml | 4.9E-1 | 4.9E-1 | 7.0E-1 | 9.1E-1 | 8.5E-1 | 1.3E0 | 1.0E-9 | 1.0E-9 | 3.5E0 | 4.1E0 | 23 | 11 | 23 | 11 | 0.52 |
| Wg | ng/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 23 | 11 | 23 | 11 | 0.50 |
| Wh | ng/ml | 1.1E-2 | 8.4E-3 | 1.4E-1 | 6.6E-2 | 5.1E-1 | 1.6E-1 | 1.0E-9 | 4.3E-3 | 2.5E0 | 5.4E-1 | 23 | 11 | 23 | 11 | 0.54 |
| Wf | ng/ml | 1.0E-9 | 1.0E-9 | 2.1E-1 | 1.0E-1 | 5.4E-1 | 1.6E-1 | 1.0E-9 | 1.0E-9 | 2.4E0 | 4.7E-1 | 23 | 11 | 23 | 11 | 0.53 |
| Ra | pg/ml | 1.0E-9 | 1.0E-9 | 4.0E-1 | 5.1E-1 | 9.5E-1 | 1.5E0 | 1.0E-9 | 1.0E-9 | 3.4E0 | 6.5E0 | 53 | 23 | 53 | 23 | 0.53 |
| Qz | pg/ml | 1.1E1 | 1.1E1 | 4.4E1 | 6.0E1 | 6.2E1 | 8.8E1 | 1.0E-9 | 1.0E-9 | 2.0E2 | 2.8E2 | 53 | 23 | 53 | 23 | 0.55 |
| Qy | pg/ml | 4.3E-1 | 4.7E-1 | 1.3E1 | 2.3E1 | 6.1E1 | 1.1E2 | 1.0E-9 | 1.0E-9 | 4.3E2 | 5.1E2 | 53 | 23 | 53 | 23 | 0.47 |
| Qx | pg/ml | 1.0E-9 | 1.0E-9 | 5.9E-1 | 2.4E0 | 2.1E0 | 8.0E0 | 1.0E-9 | 1.0E-9 | 9.4E0 | 3.1E1 | 53 | 23 | 53 | 23 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qw | pg/ml | 1.0E-9 | 1.0E-9 | 3.6E0 | 1.4E0 | 1.5E1 | 4.7E0 | 1.0E-9 | 1.0E-9 | 8.6E1 | 2.3E1 | 53 | 23 | 53 | 23 | 0.46 |
| Qv | pg/ml | 2.0E4 | 1.2E4 | 2.8E4 | 3.4E4 | 3.4E4 | 6.7E4 | 1.0E-9 | 8.5E2 | 2.3E5 | 3.3E5 | 53 | 23 | 53 | 23 | 0.43 |
| Qu | pg/ml | 7.8E0 | 1.0E-9 | 1.1E2 | 6.7E1 | 2.1E2 | 1.5E2 | 1.0E-9 | 1.0E-9 | 9.8E2 | 6.7E2 | 53 | 23 | 53 | 23 | 0.45 |
| Qt | pg/ml | 1.1E1 | 1.9E1 | 6.5E1 | 3.4E1 | 1.4E2 | 5.5E1 | 1.0E-9 | 1.0E-9 | 7.0E2 | 2.2E2 | 53 | 23 | 53 | 23 | 0.53 |
| Qh | ng/ml | 1.7E1 | 3.9E1 | 4.2E1 | 6.9E1 | 6.9E1 | 9.8E1 | 4.3E-1 | 4.9E0 | 3.3E2 | 4.6E2 | 53 | 23 | 53 | 23 | 0.67 |
| Qg | ng/ml | 8.1E0 | 8.1E0 | 1.6E1 | 1.2E1 | 3.1E1 | 1.7E1 | 1.3E-1 | 1.3E0 | 2.2E2 | 8.1E1 | 53 | 23 | 53 | 23 | 0.48 |
| Jj | ng/ml | 6.4E2 | 5.6E2 | 9.6E2 | 7.0E2 | 1.2E3 | 8.6E2 | 4.9E0 | 6.8E1 | 7.7E3 | 4.1E3 | 78 | 33 | 78 | 33 | 0.43 |
| Jk | ng/ml | 2.5E0 | 3.0E0 | 2.3E1 | 3.0E1 | 6.0E1 | 5.1E1 | 1.2E-1 | 3.8E-1 | 3.9E2 | 2.0E2 | 78 | 33 | 78 | 33 | 0.58 |
| Jl | ng/ml | 5.3E-1 | 7.3E-1 | 2.1E0 | 4.3E0 | 4.7E0 | 9.0E0 | 1.1E-3 | 4.8E-2 | 2.0E1 | 4.0E1 | 78 | 33 | 78 | 33 | 0.58 |
| Jm | ng/ml | 2.0E1 | 4.3E1 | 4.9E1 | 1.3E2 | 8.5E1 | 3.5E2 | 1.0E-9 | 9.2E-1 | 6.1E2 | 2.1E3 | 78 | 33 | 78 | 33 | 0.63 |
| Jn | pg/ml | 4.6E-1 | 6.5E-1 | 1.5E0 | 1.2E0 | 4.8E0 | 1.4E0 | 1.0E-9 | 1.0E-9 | 3.9E1 | 6.6E0 | 78 | 33 | 78 | 33 | 0.58 |
| Jo | pg/ml | 4.2E3 | 4.5E3 | 5.2E3 | 5.3E3 | 4.3E3 | 3.9E3 | 4.8E2 | 2.6E2 | 1.9E4 | 1.6E4 | 78 | 33 | 78 | 33 | 0.52 |
| Jp | pg/ml | 7.9E4 | 8.2E4 | 7.8E4 | 8.8E4 | 3.2E4 | 4.4E4 | 2.8E3 | 1.6E4 | 1.7E5 | 2.1E5 | 78 | 33 | 78 | 33 | 0.52 |
| Jq | pg/ml | 1.1E2 | 1.4E2 | 1.9E2 | 2.1E2 | 4.6E2 | 2.3E2 | 5.4E0 | 2.2E1 | 4.0E3 | 1.1E3 | 78 | 33 | 78 | 33 | 0.60 |
| Jr | pg/ml | 6.5E0 | 7.2E0 | 1.4E1 | 1.3E1 | 2.7E1 | 1.6E1 | 1.0E-9 | 1.0E-9 | 1.8E2 | 4.9E1 | 78 | 33 | 78 | 33 | 0.54 |
| Js | pg/ml | 1.5E1 | 1.9E1 | 2.9E1 | 2.9E1 | 7.5E1 | 3.0E1 | 1.3E0 | 1.0E-9 | 6.1E2 | 1.2E2 | 78 | 33 | 78 | 33 | 0.59 |
| Jt | pg/ml | 2.7E3 | 2.8E3 | 3.2E3 | 3.0E3 | 2.1E3 | 1.9E3 | 2.8E2 | 2.6E2 | 9.7E3 | 7.5E3 | 78 | 33 | 78 | 33 | 0.48 |
| Xa | pg/ml | 6.1E-1 | 4.9E0 | 1.0E1 | 2.1E1 | 1.7E1 | 3.5E1 | 1.0E-9 | 1.0E-9 | 6.4E1 | 9.9E1 | 22 | 11 | 22 | 11 | 0.61 |
| Ye | pg/ml | 1.0E-9 | 1.0E-9 | 4.5E0 | 3.3E1 | 1.1E1 | 1.1E2 | 1.0E-9 | 1.0E-9 | 4.1E1 | 3.5E2 | 22 | 11 | 22 | 11 | 0.52 |
| Tm | pg/ml | 1.0E-9 | 1.0E-9 | 5.6E0 | 7.0E0 | 1.0E1 | 2.0E1 | 1.0E-9 | 1.0E-9 | 4.6E1 | 6.8E1 | 22 | 11 | 22 | 11 | 0.40 |
| Tl | pg/ml | 4.7E-1 | 1.1E-1 | 4.2E-1 | 3.6E-1 | 3.3E-1 | 5.6E-1 | 1.0E-9 | 1.0E-9 | 1.2E0 | 1.8E0 | 22 | 11 | 22 | 11 | 0.36 |
| Ju | mIU/ml | 8.4E0 | 1.2E1 | 2.2E1 | 1.8E1 | 3.5E1 | 1.8E1 | 1.7E-1 | 1.5E-1 | 2.0E2 | 6.0E1 | 53 | 23 | 53 | 23 | 0.54 |
| Jv | mIU/ml | 1.1E1 | 1.7E1 | 3.2E1 | 2.5E1 | 5.5E1 | 2.6E1 | 1.7E-2 | 8.2E-2 | 3.4E2 | 8.9E1 | 53 | 23 | 53 | 23 | 0.51 |
| Jy | ng/ml | 1.5E-3 | 1.6E-3 | 2.4E-3 | 2.7E-3 | 5.2E-3 | 4.4E-3 | 1.0E-9 | 5.3E-4 | 3.9E-2 | 2.3E-2 | 53 | 23 | 53 | 23 | 0.55 |
| Kc | pg/ml | 2.8E1 | 2.3E1 | 4.3E1 | 4.1E1 | 4.1E1 | 6.2E1 | 1.0E-9 | 1.0E-9 | 1.7E2 | 2.7E2 | 52 | 23 | 52 | 23 | 0.43 |
| Kd | pg/ml | 1.0E-9 | 1.0E-9 | 1.8E2 | 3.5E2 | 4.4E2 | 7.3E2 | 1.0E-9 | 1.0E-9 | 1.9E3 | 2.4E3 | 52 | 23 | 52 | 23 | 0.53 |
| Ke | pg/ml | 1.3E4 | 1.7E4 | 1.6E4 | 2.2E4 | 1.1E4 | 1.7E4 | 5.8E2 | 2.3E3 | 5.6E4 | 6.3E4 | 52 | 23 | 52 | 23 | 0.58 |
| Kf | pg/mL | 8.2E0 | 7.2E0 | 7.3E0 | 8.0E0 | 4.9E0 | 8.7E0 | 1.0E-9 | 1.0E-9 | 2.4E1 | 4.4E1 | 52 | 23 | 52 | 23 | 0.47 |
| Kg | pg/mL | 1.2E3 | 9.6E2 | 1.9E3 | 1.8E3 | 2.1E3 | 1.9E3 | 1.4E2 | 2.4E2 | 1.1E4 | 7.7E3 | 52 | 23 | 52 | 23 | 0.46 |
| Ki | pg/ml | 5.9E1 | 8.5E1 | 7.5E1 | 8.7E1 | 4.6E1 | 5.6E1 | 1.0E-9 | 1.3E1 | 2.5E2 | 2.5E2 | 51 | 23 | 51 | 23 | 0.58 |
| Kj | pg/ml | 9.5E2 | 7.7E2 | 1.5E3 | 1.3E3 | 1.2E3 | 1.4E3 | 7.8E1 | 3.3E1 | 5.6E3 | 6.1E3 | 52 | 23 | 52 | 23 | 0.42 |
| Kk | pg/ml | 6.8E0 | 1.0E1 | 1.0E1 | 1.5E1 | 1.0E1 | 1.6E1 | 1.0E-9 | 8.4E-1 | 5.0E1 | 5.5E1 | 52 | 23 | 52 | 23 | 0.61 |
| Kl | pg/ml | 2.1E4 | 1.6E4 | 2.9E4 | 2.6E4 | 2.9E4 | 2.3E4 | 2.1E2 | 7.0E2 | 1.6E5 | 9.0E4 | 52 | 23 | 52 | 23 | 0.47 |
| Kn | pg/ml | 3.0E1 | 3.0E1 | 5.6E1 | 9.0E1 | 6.7E1 | 1.5E2 | 1.0E-9 | 1.0E-9 | 3.6E2 | 6.3E2 | 52 | 23 | 52 | 23 | 0.54 |
| Ko | pg/ml | 2.1E2 | 4.0E2 | 4.1E2 | 5.4E2 | 4.5E2 | 4.9E2 | 1.0E-9 | 1.0E-9 | 1.5E3 | 1.6E3 | 52 | 23 | 52 | 23 | 0.58 |
| Kp | pg/ml | 3.6E2 | 3.6E2 | 3.7E2 | 3.8E2 | 2.1E2 | 2.6E2 | 1.0E-9 | 1.0E-9 | 8.4E2 | 9.8E2 | 52 | 23 | 52 | 23 | 0.50 |
| Kq | pg/ml | 3.0E2 | 4.5E2 | 4.6E2 | 6.2E2 | 4.7E2 | 7.2E2 | 7.0E0 | 2.5E1 | 2.1E3 | 3.6E3 | 50 | 24 | 50 | 24 | 0.61 |
| Kr | pg/ml | 5.7E-1 | 7.2E-1 | 2.1E0 | 2.5E0 | 2.9E0 | 4.4E0 | 1.0E-9 | 1.0E-9 | 1.2E1 | 1.9E1 | 50 | 24 | 50 | 24 | 0.50 |
| Ks | pg/ml | 9.7E3 | 1.7E4 | 1.9E4 | 2.1E4 | 1.8E4 | 1.8E4 | 2.7E2 | 9.9E2 | 5.0E4 | 5.1E4 | 50 | 24 | 50 | 24 | 0.54 |
| Ps | ng/ml | 1.8E2 | 4.0E2 | 4.4E2 | 1.5E3 | 7.0E2 | 3.6E3 | 4.1E-1 | 1.6E1 | 2.9E3 | 1.2E4 | 23 | 11 | 23 | 11 | 0.60 |
| Kx | ng/ml | 1.0E-9 | 8.5E-3 | 7.1E-3 | 1.5E-2 | 1.4E-2 | 2.0E-2 | 1.0E-9 | 1.0E-9 | 7.9E-2 | 6.5E-2 | 52 | 23 | 52 | 23 | 0.60 |
| Ky | ng/ml | 8.7E-2 | 1.5E-1 | 3.8E-1 | 4.6E-1 | 7.3E-1 | 6.4E-1 | 1.0E-9 | 1.0E-9 | 4.4E0 | 2.4E0 | 52 | 23 | 52 | 23 | 0.58 |
| Kz | ng/ml | 1.0E-9 | 1.0E-9 | 1.9E-3 | 4.4E-3 | 4.6E-3 | 6.2E-3 | 1.0E-9 | 1.0E-9 | 1.4E-2 | 1.4E-2 | 52 | 23 | 52 | 23 | 0.60 |
| Rz | ng/ml | 5.6E-1 | 2.9E-1 | 1.1E0 | 7.3E-1 | 1.4E0 | 9.9E-1 | 1.8E-2 | 1.7E-2 | 4.7E0 | 3.1E0 | 23 | 11 | 23 | 11 | 0.39 |
| Ry | ng/ml | 1.6E-2 | 1.6E-2 | 2.4E-2 | 2.9E-2 | 2.9E-2 | 2.9E-2 | 1.0E-9 | 1.0E-9 | 1.2E-1 | 9.2E-2 | 23 | 11 | 23 | 11 | 0.54 |
| Rx | ng/ml | 1.0E-9 | 1.6E-3 | 1.3E-3 | 1.7E-3 | 2.3E-3 | 1.7E-3 | 1.0E-9 | 1.0E-9 | 8.6E-3 | 3.9E-3 | 23 | 11 | 23 | 11 | 0.62 |
| Ld | pg/ml | 1.0E-9 | 1.0E-9 | 2.0E0 | 5.6E0 | 3.5E0 | 1.1E1 | 1.0E-9 | 1.0E-9 | 1.5E1 | 5.0E1 | 51 | 24 | 51 | 24 | 0.59 |
| Lh | pg/ml | 1.4E4 | 1.9E4 | 2.2E4 | 3.6E4 | 2.1E4 | 4.2E4 | 1.8E2 | 5.5E2 | 1.1E5 | 2.1E5 | 79 | 33 | 79 | 33 | 0.60 |
| Li | pg/ml | 3.0E3 | 1.4E4 | 1.2E4 | 4.8E4 | 2.4E4 | 1.6E5 | 1.7E2 | 2.6E1 | 1.3E5 | 9.2E5 | 79 | 33 | 79 | 33 | 0.68 |
| Lj | pg/ml | 3.4E3 | 6.7E3 | 2.1E4 | 4.7E4 | 6.9E4 | 8.0E4 | 4.1E1 | 1.0E-9 | 4.7E5 | 4.1E5 | 79 | 33 | 79 | 33 | 0.59 |
| Lp | pg/ml | 5.7E0 | 9.5E0 | 7.4E1 | 7.6E1 | 1.8E2 | 2.0E2 | 1.0E-9 | 1.0E-9 | 7.7E2 | 6.8E2 | 23 | 11 | 23 | 11 | 0.51 |
| Lt | pg/ml | 1.0E-9 | 1.0E-9 | 2.3E0 | 2.3E0 | 6.7E0 | 7.6E0 | 1.0E-9 | 1.0E-9 | 2.5E1 | 2.5E1 | 23 | 11 | 23 | 11 | 0.48 |
| Rv | ng/ml | 5.0E-4 | 9.8E-4 | 8.0E-4 | 1.6E-3 | 1.1E-3 | 2.6E-3 | 1.0E-9 | 1.0E-9 | 5.0E-3 | 9.2E-3 | 23 | 11 | 23 | 11 | 0.65 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ru | ng/ml | 1.0E-9 | 1.0E-9 | 1.9E-2 | 3.8E-2 | 8.0E-2 | 1.0E-1 | 1.0E-9 | 1.0E-9 | 3.8E-1 | 3.5E-1 | 23 | 11 | 23 | 11 | 0.55 |
| Rt | ng/ml | 4.0E-2 | 5.2E-2 | 1.3E-1 | 9.1E-2 | 1.8E-1 | 1.1E-1 | 1.0E-3 | 1.3E-3 | 5.8E-1 | 3.6E-1 | 23 | 11 | 23 | 11 | 0.51 |
| Yl | pg/ml | 7.3E0 | 1.2E1 | 8.8E0 | 2.4E1 | 1.3E1 | 2.3E1 | 1.0E-9 | 1.0E-9 | 6.6E1 | 7.9E1 | 23 | 11 | 23 | 11 | 0.75 |
| Rm | ng/ml | 1.7E1 | 3.4E1 | 3.6E1 | 8.2E1 | 4.9E1 | 1.4E2 | 7.7E-1 | 1.2E0 | 2.5E2 | 6.5E2 | 52 | 22 | 52 | 22 | 0.61 |
| Rh | ng/ml | 1.6E2 | 1.1E2 | 2.6E2 | 9.6E2 | 3.3E2 | 3.6E3 | 3.6E0 | 2.5E1 | 2.0E3 | 1.7E4 | 52 | 22 | 52 | 22 | 0.44 |
| Ri | ng/ml | 1.0E-9 | 1.0E-9 | 3.5E0 | 4.3E0 | 7.4E0 | 1.0E1 | 1.0E-9 | 1.0E-9 | 4.5E1 | 4.5E1 | 52 | 22 | 52 | 22 | 0.48 |
| Rg | ng/ml | 1.0E-9 | 1.0E-9 | 5.1E-2 | 2.0E-1 | 2.7E-1 | 7.1E-1 | 1.0E-9 | 1.0E-9 | 1.9E0 | 3.3E0 | 52 | 22 | 52 | 22 | 0.64 |
| Rj | ng/ml | 1.0E-9 | 1.0E-9 | 1.0E0 | 6.5E-1 | 2.1E0 | 1.1E0 | 1.0E-9 | 1.0E-9 | 1.1E1 | 3.2E0 | 52 | 22 | 52 | 22 | 0.50 |
| Rf | ng/ml | 5.0E-1 | 5.6E-1 | 1.4E0 | 1.7E0 | 2.4E0 | 3.5E0 | 3.2E-2 | 1.8E-2 | 1.2E1 | 1.7E1 | 52 | 22 | 52 | 22 | 0.52 |
| Ql | pg/ml | 4.5E0 | 9.0E0 | 1.4E1 | 1.5E1 | 2.7E1 | 2.2E1 | 1.0E-9 | 1.0E-9 | 1.8E2 | 8.8E1 | 53 | 23 | 53 | 23 | 0.53 |
| Qm | pg/ml | 4.5E0 | 3.5E0 | 2.0E1 | 3.0E1 | 3.5E1 | 4.3E1 | 1.0E-9 | 1.0E-9 | 1.8E2 | 1.7E2 | 53 | 23 | 53 | 23 | 0.55 |
| Qn | pg/ml | 6.1E-1 | 6.1E-1 | 4.6E0 | 1.6E1 | 8.8E0 | 5.2E1 | 1.0E-9 | 1.0E-9 | 3.8E1 | 2.4E2 | 53 | 23 | 53 | 23 | 0.51 |
| Nv | pg/ml | 4.3E3 | 5.3E3 | 1.0E4 | 1.3E4 | 2.0E4 | 2.2E4 | 8.4E1 | 5.4E2 | 1.5E5 | 1.1E5 | 79 | 34 | 79 | 34 | 0.54 |
| Nw | pg/ml | 8.9E3 | 1.4E4 | 1.3E4 | 1.7E4 | 1.2E4 | 1.4E4 | 5.7E2 | 1.6E3 | 7.3E4 | 6.1E4 | 79 | 34 | 79 | 34 | 0.59 |
| Nx | pg/ml | 2.4E2 | 2.6E2 | 4.7E2 | 6.1E2 | 6.3E2 | 7.0E2 | 1.0E-9 | 1.0E-9 | 2.6E3 | 2.5E3 | 79 | 34 | 79 | 34 | 0.58 |
| Ny | pg/ml | 4.1E0 | 1.0E1 | 1.5E1 | 3.3E1 | 3.2E1 | 7.5E1 | 1.0E-9 | 1.0E-9 | 1.9E2 | 4.2E2 | 79 | 34 | 79 | 34 | 0.63 |
| Oa | pg/ml | 1.2E2 | 7.3E2 | 3.1E2 | 1.0E3 | 4.6E2 | 1.1E3 | 1.0E-9 | 4.9E0 | 2.3E3 | 4.5E3 | 53 | 23 | 53 | 23 | 0.68 |
| Op | pg/ml | 4.6E5 | 4.4E5 | 4.5E5 | 4.2E5 | 1.5E5 | 2.2E5 | 1.8E5 | 5.2E4 | 7.1E5 | 7.5E5 | 23 | 11 | 23 | 11 | 0.46 |
| Wn | ng/ml | 1.1E1 | 2.0E1 | 1.9E1 | 2.8E1 | 2.4E1 | 2.1E1 | 8.9E-1 | 4.8E0 | 1.1E2 | 6.2E1 | 19 | 8 | 19 | 8 | 0.69 |
| Tk | ng/ml | 1.5E2 | 1.2E2 | 3.2E2 | 2.0E2 | 5.4E2 | 2.3E2 | 1.4E1 | 3.1E1 | 2.3E3 | 7.7E2 | 20 | 9 | 20 | 9 | 0.44 |
| Oe | pg/ml | 2.2E1 | 5.3E1 | 2.6E2 | 2.9E2 | 4.9E2 | 4.5E2 | 1.0E-9 | 1.0E-9 | 3.4E3 | 1.6E3 | 79 | 34 | 79 | 34 | 0.53 |
| Of | pg/ml | 1.3E2 | 1.7E2 | 1.3E4 | 4.8E3 | 7.1E4 | 1.2E4 | 1.0E-9 | 1.0E-9 | 6.2E5 | 5.4E4 | 79 | 34 | 79 | 34 | 0.52 |
| Og | pg/ml | 8.7E-2 | 7.5E-2 | 4.0E-1 | 4.9E-1 | 1.3E0 | 1.2E0 | 1.0E-9 | 1.0E-9 | 1.0E1 | 5.0E0 | 79 | 34 | 79 | 34 | 0.45 |
| Oh | pg/ml | 2.6E0 | 4.2E0 | 9.6E0 | 1.7E1 | 2.5E1 | 4.9E1 | 1.0E-9 | 1.0E-9 | 2.0E2 | 2.8E2 | 79 | 34 | 79 | 34 | 0.59 |
| Oi | pg/ml | 3.2E0 | 2.9E0 | 6.2E0 | 6.7E0 | 8.2E0 | 8.9E0 | 1.0E-9 | 1.0E-9 | 4.1E1 | 3.6E1 | 79 | 34 | 79 | 34 | 0.51 |
| Ok | pg/ml | 4.6E2 | 5.3E2 | 5.7E2 | 8.3E2 | 4.6E2 | 7.5E2 | 4.0E1 | 3.9E1 | 2.4E3 | 3.2E3 | 79 | 34 | 79 | 34 | 0.60 |
| Om | pg/ml | 4.6E2 | 6.2E2 | 1.2E3 | 9.1E2 | 4.1E3 | 1.0E3 | 1.0E-9 | 8.4E1 | 3.6E4 | 5.0E3 | 79 | 34 | 79 | 34 | 0.57 |
| On | pg/ml | 1.9E2 | 2.3E2 | 2.6E2 | 4.0E2 | 2.6E2 | 4.0E2 | 7.2E0 | 3.9E1 | 1.6E3 | 1.5E3 | 79 | 34 | 79 | 34 | 0.60 |
| Or | pg/ml | 1.2E1 | 2.3E1 | 4.0E1 | 7.9E1 | 7.7E1 | 1.3E2 | 1.0E-9 | 1.0E-9 | 4.4E2 | 4.5E2 | 52 | 24 | 52 | 24 | 0.59 |
| Ow | pg/ml | 4.2E1 | 4.6E1 | 1.5E2 | 3.1E2 | 4.8E2 | 7.0E2 | 1.0E-9 | 1.0E-9 | 3.2E3 | 3.0E3 | 52 | 24 | 52 | 24 | 0.53 |
| Ou | pg/ml | 5.6E2 | 3.3E2 | 1.0E3 | 2.1E3 | 1.5E3 | 3.5E3 | 1.0E-9 | 1.0E-9 | 8.1E3 | 1.1E4 | 52 | 24 | 52 | 24 | 0.46 |
| Ul | ng/ml | 1.0E-9 | 1.0E-9 | 8.9E-1 | 1.6E0 | 3.8E0 | 5.4E0 | 1.0E-9 | 1.0E-9 | 2.4E1 | 2.1E1 | 53 | 23 | 53 | 23 | 0.51 |
| Uo | ng/ml | 1.0E-9 | 1.0E-9 | 7.3E-2 | 3.1E-2 | 1.9E-1 | 9.4E-2 | 1.0E-9 | 1.0E-9 | 8.5E-1 | 3.3E-1 | 53 | 23 | 53 | 23 | 0.46 |
| Uk | ng/ml | 1.0E-9 | 1.0E-9 | 9.8E-3 | 3.3E-3 | 2.5E-2 | 1.5E-2 | 1.0E-9 | 1.0E-9 | 1.3E-1 | 7.1E-2 | 53 | 23 | 53 | 23 | 0.34 |
| Um | ng/ml | 1.0E-9 | 1.0E-9 | 1.7E-1 | 1.4E-1 | 3.2E-1 | 3.8E-1 | 1.0E-9 | 1.0E-9 | 1.3E0 | 1.4E0 | 53 | 23 | 53 | 23 | 0.41 |
| Uf | ng/ml | 7.2E-2 | 9.5E-2 | 1.6E-1 | 1.3E-1 | 2.2E-1 | 1.3E-1 | 1.1E-3 | 9.8E-3 | 1.1E0 | 5.3E-1 | 53 | 23 | 53 | 23 | 0.53 |
| Uh | ng/ml | 2.2E0 | 3.4E0 | 3.8E0 | 5.0E0 | 4.2E0 | 5.0E0 | 6.0E-2 | 1.9E-1 | 1.7E1 | 1.8E1 | 53 | 23 | 53 | 23 | 0.58 |
| Un | ng/ml | 2.2E0 | 2.1E0 | 2.5E0 | 2.4E0 | 1.5E0 | 1.3E0 | 4.6E-1 | 3.6E-1 | 8.0E0 | 5.1E0 | 53 | 23 | 53 | 23 | 0.48 |
| Ug | ng/ml | 2.0E1 | 1.1E1 | 3.0E1 | 1.9E1 | 3.3E1 | 2.3E1 | 1.2E0 | 2.5E0 | 1.8E2 | 1.1E2 | 53 | 23 | 53 | 23 | 0.38 |
| Ur | ng/ml | 9.6E-2 | 1.1E-1 | 3.0E-1 | 4.5E-1 | 5.1E-1 | 9.7E-1 | 1.0E-9 | 1.0E-9 | 2.8E0 | 4.5E0 | 52 | 23 | 52 | 23 | 0.50 |
| Up | ng/ml | 1.0E-9 | 1.0E-9 | 6.0E-3 | 2.9E-3 | 1.3E-2 | 9.7E-3 | 1.0E-9 | 1.0E-9 | 6.0E-2 | 4.5E-2 | 52 | 23 | 52 | 23 | 0.39 |
| Us | ng/ml | 6.5E-3 | 3.6E-3 | 2.0E-2 | 4.3E-2 | 3.5E-2 | 8.3E-2 | 1.0E-9 | 1.0E-9 | 2.1E-1 | 3.6E-1 | 52 | 23 | 52 | 23 | 0.49 |
| Uv | ng/ml | 3.1E-3 | 3.5E-3 | 9.8E-3 | 6.8E-3 | 2.4E-2 | 1.1E-2 | 1.0E-9 | 1.0E-9 | 1.5E-1 | 4.4E-2 | 52 | 23 | 52 | 23 | 0.49 |
| Ut | ng/ml | 6.0E-1 | 9.0E-1 | 2.2E0 | 3.0E0 | 3.7E0 | 5.2E0 | 1.0E-9 | 9.2E-2 | 1.7E1 | 2.4E1 | 52 | 23 | 52 | 23 | 0.59 |
| Uu | ng/ml | 7.1E0 | 5.2E0 | 7.9E0 | 6.4E0 | 5.1E0 | 4.6E0 | 4.5E-1 | 1.5E0 | 2.0E1 | 2.1E1 | 52 | 23 | 52 | 23 | 0.41 |
| Uw | ng/ml | 2.5E0 | 4.0E0 | 3.0E0 | 3.5E0 | 2.2E0 | 1.9E0 | 2.0E-1 | 8.2E-1 | 7.9E0 | 6.4E0 | 23 | 11 | 23 | 11 | 0.61 |
| Vb | ng/ml | 9.6E-1 | 8.9E-1 | 9.8E-1 | 8.9E-1 | 4.3E-1 | 5.2E-1 | 2.1E-1 | 2.6E-1 | 1.8E0 | 1.9E0 | 23 | 11 | 23 | 11 | 0.45 |
| Vc | ng/ml | 1.0E-9 | 1.0E-9 | 5.8E-4 | 1.0E-9 | 2.8E-3 | 0.0E0 | 1.0E-9 | 1.0E-9 | 1.3E-2 | 1.0E-9 | 23 | 11 | 23 | 11 | 0.48 |
| Uy | ng/ml | 1.2E0 | 1.5E0 | 6.9E0 | 1.1E1 | 2.0E1 | 2.2E1 | 3.1E-2 | 2.0E-2 | 9.9E1 | 6.4E1 | 23 | 11 | 23 | 11 | 0.49 |
| Uz | ng/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 23 | 11 | 23 | 11 | 0.50 |
| Ux | ng/ml | 1.9E2 | 1.6E2 | 2.3E2 | 1.9E2 | 1.5E2 | 1.4E2 | 9.9E0 | 2.0E1 | 5.3E2 | 4.6E2 | 23 | 11 | 23 | 11 | 0.43 |
| Va | ng/ml | 1.8E1 | 2.8E0 | 2.8E1 | 1.3E1 | 3.2E1 | 2.2E1 | 8.1E-1 | 1.2E0 | 1.2E2 | 6.2E1 | 23 | 11 | 23 | 11 | 0.32 |
| Vh | ng/ml | 1.1E-2 | 1.3E-2 | 1.6E-2 | 1.2E-2 | 1.8E-2 | 9.0E-3 | 5.2E-4 | 1.0E-3 | 7.6E-2 | 2.7E-2 | 23 | 11 | 23 | 11 | 0.48 |

272

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vi | ng/ml | 3.0E-3 | 1.6E-2 | 1.1E-2 | 2.4E-2 | 1.9E-2 | 3.4E-2 | 1.6E-4 | 1.0E-9 | 7.5E-2 | 1.2E-1 | 23 | 11 | 23 | 11 | 0.60 |
| Vj | ng/ml | 5.5E1 | 1.4E1 | 4.4E2 | 4.2E1 | 1.7E3 | 5.3E1 | 6.5E0 | 4.2E0 | 8.4E3 | 1.7E2 | 23 | 9 | 23 | 9 | 0.32 |
| Vp | ng/ml | 1.0E-9 | 1.0E-9 | 6.1E-2 | 7.5E-2 | 2.1E-1 | 2.0E-1 | 1.0E-9 | 1.0E-9 | 1.2E0 | 7.4E-1 | 53 | 23 | 53 | 23 | 0.55 |
| Vt | ng/ml | 7.3E0 | 1.1E1 | 1.0E1 | 1.2E1 | 1.2E1 | 9.1E0 | 1.1E0 | 8.8E-1 | 6.5E1 | 3.8E1 | 53 | 23 | 53 | 23 | 0.61 |
| Vu | ng/ml | 1.0E-9 | 1.0E0 | 2.1E0 | 2.7E0 | 4.3E0 | 5.0E0 | 1.0E-9 | 1.0E-9 | 2.1E1 | 2.2E1 | 53 | 23 | 53 | 23 | 0.59 |
| Vq | ng/ml | 2.2E2 | 5.7E2 | 8.4E2 | 1.2E3 | 1.9E3 | 2.9E3 | 9.0E-1 | 1.8E1 | 1.1E4 | 1.2E4 | 38 | 18 | 38 | 18 | 0.61 |
| Vo | ng/ml | 2.6E1 | 2.5E1 | 2.5E1 | 2.4E1 | 6.0E0 | 5.3E0 | 2.4E0 | 4.9E0 | 4.8E1 | 3.1E1 | 53 | 23 | 53 | 23 | 0.43 |
| Vs | ng/ml | 1.0E-9 | 1.0E-9 | 4.7E0 | 2.8E0 | 8.9E0 | 5.1E0 | 1.0E-9 | 1.0E-9 | 4.2E1 | 1.5E1 | 53 | 21 | 53 | 21 | 0.45 |
| Vv | ng/ml | 4.3E0 | 1.5E0 | 5.9E0 | 4.0E0 | 6.4E0 | 6.4E0 | 1.0E-9 | 1.0E-9 | 2.8E1 | 2.6E1 | 53 | 23 | 53 | 23 | 0.39 |
| Vw | ng/ml | 4.5E1 | 2.6E1 | 4.2E1 | 2.9E1 | 1.8E1 | 1.8E1 | 2.5E0 | 4.4E0 | 7.0E1 | 6.6E1 | 23 | 11 | 23 | 11 | 0.29 |
| Oy | pg/ml | 4.7E-1 | 3.6E-1 | 3.5E0 | 4.6E0 | 1.2E1 | 1.3E1 | 1.0E-9 | 1.0E-9 | 9.9E1 | 5.5E1 | 78 | 34 | 78 | 34 | 0.46 |
| Oz | pg/ml | 2.4E-1 | 3.4E-2 | 7.5E-1 | 2.9E-1 | 3.3E0 | 5.5E-1 | 1.0E-9 | 1.0E-9 | 2.9E1 | 2.2E0 | 78 | 34 | 78 | 34 | 0.42 |
| Pa | pg/ml | 4.1E-1 | 4.5E-1 | 1.1E0 | 1.1E0 | 3.6E0 | 1.4E0 | 1.0E-9 | 1.0E-9 | 2.9E1 | 6.5E0 | 78 | 34 | 78 | 34 | 0.58 |
| Pb | pg/ml | 4.4E-2 | 1.0E-9 | 2.3E-1 | 9.4E-2 | 8.4E-1 | 2.0E-1 | 1.0E-9 | 1.0E-9 | 7.3E0 | 1.1E0 | 78 | 34 | 78 | 34 | 0.40 |
| Pc | pg/ml | 3.3E-1 | 3.2E-1 | 4.1E-1 | 3.7E-1 | 4.2E-1 | 4.0E-1 | 1.0E-9 | 1.0E-9 | 1.8E0 | 1.4E0 | 78 | 34 | 78 | 34 | 0.47 |
| Pd | pg/ml | 2.0E0 | 2.0E0 | 1.5E1 | 4.0E0 | 9.5E1 | 5.7E0 | 1.0E-9 | 1.0E-9 | 8.4E2 | 2.9E1 | 78 | 34 | 78 | 34 | 0.48 |
| Pe | pg/ml | 2.4E1 | 5.5E1 | 1.1E2 | 2.2E2 | 4.0E2 | 7.5E2 | 1.0E-9 | 1.0E-9 | 3.3E3 | 4.3E3 | 78 | 34 | 78 | 34 | 0.61 |
| Pf | pg/ml | 1.6E0 | 5.9E0 | 2.5E1 | 2.3E1 | 1.7E2 | 6.5E1 | 1.0E-9 | 1.0E-9 | 1.5E3 | 3.8E2 | 78 | 34 | 78 | 34 | 0.66 |
| Pg | pg/ml | 3.2E0 | 7.8E0 | 1.2E2 | 3.9E1 | 8.7E2 | 8.2E1 | 1.0E-9 | 1.0E-9 | 7.7E3 | 4.0E2 | 78 | 34 | 78 | 34 | 0.62 |
| Ph | ng/ml | 2.1E-1 | 1.8E-1 | 4.0E-1 | 3.1E-1 | 5.9E-1 | 4.8E-1 | 1.0E-9 | 1.0E-9 | 2.8E0 | 2.4E0 | 52 | 24 | 52 | 24 | 0.49 |
| Pi | ng/ml | 1.8E-1 | 2.3E-1 | 2.5E-1 | 3.0E-1 | 2.2E-1 | 3.1E-1 | 1.0E-9 | 1.0E-9 | 9.7E-1 | 1.4E0 | 52 | 24 | 52 | 24 | 0.54 |
| Pj | ng/mL | 6.7E0 | 5.7E0 | 7.8E0 | 6.5E0 | 6.0E0 | 4.5E0 | 1.3E0 | 3.8E-1 | 2.5E1 | 2.0E1 | 52 | 24 | 52 | 24 | 0.45 |
| Pk | ng/ml | 6.8E-3 | 1.1E-2 | 1.3E-2 | 1.4E-2 | 2.2E-2 | 1.1E-2 | 1.0E-9 | 1.0E-9 | 1.4E-1 | 3.9E-2 | 52 | 24 | 52 | 24 | 0.62 |
| aA | mg/dL | 8.9E-1 | 1.0E0 | 1.0E0 | 1.0E0 | 5.9E-1 | 5.6E-1 | 2.6E-1 | 4.0E-1 | 3.9E0 | 3.6E0 | 106 | 40 | 106 | 40 | 0.54 |
| aC | mg/mL | 2.2E0 | 1.9E0 | 2.4E0 | 2.1E0 | 1.0E0 | 1.0E0 | 8.7E-1 | 7.5E-1 | 5.5E0 | 4.8E0 | 55 | 22 | 55 | 22 | 0.40 |
| aD | ug/mL | 3.1E0 | 4.7E0 | 4.4E0 | 5.7E0 | 3.6E0 | 4.4E0 | 8.7E-1 | 7.5E-1 | 2.0E1 | 2.1E1 | 55 | 22 | 55 | 22 | 0.61 |
| aE | mg/mL | 5.3E-1 | 5.2E-1 | 5.5E-1 | 5.6E-1 | 1.5E-1 | 1.9E-1 | 2.5E-1 | 1.8E-1 | 1.0E0 | 9.3E-1 | 55 | 22 | 55 | 22 | 0.52 |
| aF | ng/mL | 1.7E0 | 2.2E0 | 4.1E0 | 4.3E0 | 5.9E0 | 6.2E0 | 4.3E-3 | 3.7E-1 | 3.5E1 | 2.9E1 | 55 | 22 | 55 | 22 | 0.50 |
| aG | mg/mL | 1.5E-1 | 1.3E-1 | 1.6E-1 | 1.6E-1 | 8.1E-2 | 1.0E-1 | 5.1E-2 | 6.4E-2 | 3.8E-1 | 4.8E-1 | 55 | 22 | 55 | 22 | 0.48 |
| aH | ug/mL | 8.1E1 | 7.3E1 | 9.6E1 | 7.7E1 | 5.1E1 | 4.1E1 | 1.1E1 | 2.0E1 | 2.6E2 | 1.7E2 | 55 | 22 | 55 | 22 | 0.39 |
| aI | ug/mL | 2.0E2 | 1.5E2 | 1.9E2 | 1.6E2 | 6.1E1 | 6.2E1 | 4.8E1 | 7.5E1 | 3.4E2 | 3.2E2 | 55 | 22 | 55 | 22 | 0.30 |
| aJ | ug/mL | 2.4E0 | 3.5E0 | 3.1E0 | 3.6E0 | 2.3E0 | 1.9E0 | 7.6E-1 | 8.2E-1 | 1.6E1 | 7.9E0 | 55 | 22 | 55 | 22 | 0.61 |
| aK | ng/mL | 1.4E0 | 1.3E0 | 2.0E0 | 1.5E0 | 2.0E0 | 1.0E0 | 2.9E-4 | 4.7E-1 | 1.0E1 | 4.2E0 | 55 | 22 | 55 | 22 | 0.49 |
| aL | mg/mL | 7.8E-1 | 7.7E-1 | 8.1E-1 | 7.5E-1 | 2.5E-1 | 2.6E-1 | 3.0E-1 | 4.1E-1 | 1.7E0 | 1.5E0 | 55 | 22 | 55 | 22 | 0.43 |
| aM | U/mL | 2.6E1 | 2.8E1 | 3.8E1 | 5.2E1 | 3.6E1 | 4.9E1 | 4.2E-2 | 4.2E-2 | 1.4E2 | 1.4E2 | 55 | 22 | 55 | 22 | 0.56 |
| aN | U/mL | 1.9E1 | 1.7E1 | 3.5E1 | 2.7E1 | 5.5E1 | 2.5E1 | 2.5E-3 | 2.5E-3 | 3.8E2 | 8.5E1 | 55 | 22 | 55 | 22 | 0.47 |
| aO | pg/mL | 7.3E1 | 3.6E1 | 3.7E2 | 4.2E2 | 8.4E2 | 6.9E2 | 6.0E-2 | 6.0E-2 | 3.6E3 | 2.1E3 | 55 | 22 | 55 | 22 | 0.47 |
| aP | ng/mL | 1.7E0 | 1.7E0 | 2.0E0 | 2.1E0 | 1.2E0 | 1.4E0 | 7.8E-1 | 6.8E-1 | 6.4E0 | 6.5E0 | 55 | 22 | 55 | 22 | 0.50 |
| aQ | ng/mL | 2.9E-1 | 2.2E-1 | 4.9E-1 | 2.7E-1 | 4.7E-1 | 1.9E-1 | 2.5E-2 | 5.9E-2 | 2.0E0 | 9.2E-1 | 55 | 22 | 55 | 22 | 0.41 |
| aR | ng/mL | 1.7E0 | 1.6E0 | 3.2E0 | 2.4E0 | 4.5E0 | 2.4E0 | 4.5E-1 | 5.6E-1 | 3.0E1 | 1.1E1 | 55 | 22 | 55 | 22 | 0.46 |
| aS | ng/mL | 3.5E-1 | 4.8E-1 | 8.9E-1 | 6.6E-1 | 1.4E0 | 5.9E-1 | 4.2E-3 | 7.0E-2 | 6.2E0 | 2.3E0 | 55 | 22 | 55 | 22 | 0.54 |
| aU | pg/mL | 6.3E1 | 6.0E1 | 1.1E2 | 6.4E1 | 1.3E2 | 3.4E1 | 7.4E-2 | 2.0E1 | 7.0E2 | 1.6E2 | 55 | 22 | 55 | 22 | 0.46 |
| aV | ng/mL | 6.9E-1 | 4.4E-1 | 1.6E0 | 6.3E-1 | 4.4E0 | 4.4E-1 | 3.8E-2 | 9.1E-2 | 3.3E1 | 1.6E0 | 55 | 22 | 55 | 22 | 0.44 |
| aW | pg/mL | 2.1E1 | 1.5E1 | 2.0E1 | 1.4E1 | 9.1E0 | 1.0E1 | 7.2E-2 | 7.2E-2 | 3.7E1 | 3.1E1 | 55 | 22 | 55 | 22 | 0.34 |
| aX | ng/mL | 6.8E0 | 9.8E0 | 1.1E1 | 1.1E1 | 1.0E1 | 9.0E0 | 7.0E-1 | 1.1E0 | 4.6E1 | 3.1E1 | 55 | 22 | 55 | 22 | 0.49 |
| aY | pg/mL | 5.3E1 | 5.0E1 | 9.3E1 | 5.9E1 | 1.7E2 | 5.0E1 | 4.1E-1 | 4.1E-1 | 1.2E3 | 2.0E2 | 55 | 22 | 55 | 22 | 0.43 |
| aZ | pg/mL | 1.5E2 | 2.6E2 | 5.4E2 | 6.3E2 | 1.2E3 | 1.1E3 | 1.7E0 | 1.5E1 | 5.9E3 | 4.3E3 | 55 | 22 | 55 | 22 | 0.57 |
| bA | ng/mL | 9.4E0 | 2.1E1 | 5.9E1 | 6.7E1 | 1.4E2 | 1.2E2 | 3.0E-2 | 3.0E-2 | 9.4E2 | 4.3E2 | 55 | 22 | 55 | 22 | 0.55 |
| bB | ng/mL | 3.1E2 | 2.5E2 | 3.4E2 | 2.6E2 | 1.9E2 | 1.7E2 | 3.6E1 | 2.3E1 | 8.1E2 | 6.2E2 | 55 | 22 | 55 | 22 | 0.38 |
| bC | ng/mL | 3.3E2 | 3.0E2 | 7.2E2 | 5.4E2 | 1.0E3 | 8.3E2 | 9.8E0 | 5.0E1 | 4.7E3 | 4.0E3 | 55 | 22 | 55 | 22 | 0.47 |
| bE | mg/mL | 5.1E0 | 5.0E0 | 5.6E0 | 5.1E0 | 2.2E0 | 1.8E0 | 1.4E0 | 1.8E0 | 1.2E1 | 9.6E0 | 55 | 22 | 55 | 22 | 0.44 |
| bF | pg/mL | 2.1E1 | 3.3E1 | 2.7E2 | 4.5E2 | 1.4E3 | 1.4E3 | 2.1E0 | 6.2E0 | 1.0E4 | 6.3E3 | 55 | 22 | 55 | 22 | 0.61 |
| bG | ng/mL | 1.2E0 | 1.5E0 | 2.3E0 | 3.7E0 | 3.2E0 | 6.4E0 | 2.5E-1 | 2.7E-1 | 1.7E1 | 3.0E1 | 55 | 22 | 55 | 22 | 0.59 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bH | pg/mL | 5.7E-1 | 5.7E-1 | 5.1E0 | 3.4E0 | 1.7E1 | 4.7E0 | 5.7E-1 | 5.7E-1 | 1.2E2 | 1.9E1 | 55 | 22 | 55 | 22 | 0.57 |
| bI | ng/mL | 4.0E-3 | 4.0E-3 | 1.1E-1 | 5.1E-2 | 2.2E-1 | 1.4E-1 | 4.0E-3 | 4.0E-3 | 9.8E-1 | 6.2E-1 | 55 | 22 | 55 | 22 | 0.45 |
| bJ | mg/mL | 1.9E0 | 1.9E0 | 2.4E0 | 2.0E0 | 2.2E0 | 1.3E0 | 2.5E-4 | 2.8E-1 | 1.1E1 | 5.3E0 | 55 | 22 | 55 | 22 | 0.48 |
| bL | pg/mL | 1.7E0 | 3.0E0 | 6.2E0 | 4.2E0 | 9.5E0 | 3.9E0 | 4.6E-2 | 4.6E-2 | 3.5E1 | 1.4E1 | 55 | 22 | 55 | 22 | 0.55 |
| bM | mg/mL | 2.0E0 | 2.2E0 | 2.7E0 | 2.7E0 | 1.6E0 | 1.8E0 | 4.1E-1 | 7.1E-1 | 7.9E0 | 8.6E0 | 55 | 22 | 55 | 22 | 0.51 |
| bN | ng/mL | 6.4E1 | 2.3E1 | 1.6E2 | 5.1E1 | 3.4E2 | 8.1E1 | 1.4E-1 | 1.4E-1 | 1.9E3 | 3.7E2 | 55 | 22 | 55 | 22 | 0.34 |
| bO | ng/mL | 4.0E-2 | 4.0E-2 | 6.3E0 | 4.3E0 | 1.6E1 | 9.6E0 | 4.0E-2 | 4.0E-2 | 1.0E2 | 3.7E1 | 55 | 22 | 55 | 22 | 0.46 |
| bP | mg/mL | 4.8E-1 | 6.8E-1 | 6.5E-1 | 8.6E-1 | 7.1E-1 | 7.2E-1 | 1.3E-1 | 1.1E-1 | 4.8E0 | 3.1E0 | 55 | 22 | 55 | 22 | 0.63 |
| bQ | pg/mL | 1.6E1 | 2.3E1 | 2.7E2 | 4.6E1 | 1.8E3 | 5.2E1 | 1.5E-1 | 6.4E0 | 1.3E4 | 1.8E2 | 55 | 22 | 55 | 22 | 0.60 |
| bR | ng/mL | 1.2E-2 | 1.2E-2 | 2.5E-1 | 9.1E-2 | 1.2E0 | 1.2E-1 | 1.2E-2 | 1.2E-2 | 8.7E0 | 3.9E-1 | 55 | 22 | 55 | 22 | 0.51 |
| bS | pg/mL | 9.4E-1 | 9.4E-1 | 9.9E0 | 2.8E0 | 5.3E1 | 8.9E0 | 9.4E-1 | 9.4E-1 | 3.9E2 | 4.3E1 | 55 | 22 | 55 | 22 | 0.49 |
| bU | ng/mL | 1.3E-2 | 1.1E-1 | 2.6E-1 | 1.3E-1 | 9.1E-1 | 1.5E-1 | 1.3E-2 | 1.3E-2 | 6.6E0 | 6.3E-1 | 55 | 22 | 55 | 22 | 0.54 |
| bV | pg/mL | 4.5E2 | 5.2E2 | 5.3E2 | 6.5E2 | 2.4E2 | 5.8E2 | 1.7E2 | 3.0E2 | 1.4E3 | 3.1E3 | 55 | 22 | 55 | 22 | 0.55 |
| bW | pg/mL | 3.1E2 | 3.3E2 | 4.9E2 | 4.6E2 | 6.6E2 | 4.0E2 | 9.2E1 | 1.3E2 | 4.8E3 | 1.8E3 | 55 | 22 | 55 | 22 | 0.51 |
| bX | ng/mL | 2.5E-5 | 6.9E-4 | 1.8E-3 | 2.5E-3 | 2.7E-3 | 3.0E-3 | 2.5E-5 | 2.5E-5 | 1.1E-2 | 8.7E-3 | 55 | 22 | 55 | 22 | 0.57 |
| bZ | pg/mL | 2.5E2 | 2.9E2 | 1.5E3 | 2.8E3 | 7.8E3 | 9.0E3 | 1.5E-1 | 1.5E-1 | 5.8E4 | 4.3E4 | 55 | 22 | 55 | 22 | 0.58 |
| cA | pg/mL | 6.0E-1 | 6.0E-1 | 8.0E0 | 6.0E-1 | 5.0E1 | 0.0E0 | 6.0E-1 | 6.0E-1 | 3.7E2 | 6.0E-1 | 55 | 22 | 55 | 22 | 0.45 |
| cB | ng/mL | 4.9E-2 | 3.6E-2 | 8.5E-2 | 6.8E-2 | 1.1E-1 | 9.9E-2 | 1.7E-3 | 1.7E-3 | 5.3E-1 | 4.0E-1 | 55 | 22 | 55 | 22 | 0.45 |
| cC | pg/mL | 2.4E1 | 4.6E1 | 3.8E1 | 3.8E1 | 6.5E1 | 2.3E1 | 1.0E0 | 1.0E0 | 4.5E2 | 6.7E1 | 55 | 22 | 55 | 22 | 0.59 |
| cD | pg/mL | 3.3E0 | 4.7E0 | 1.1E1 | 8.1E0 | 2.7E1 | 1.1E1 | 3.3E-1 | 3.3E-1 | 1.4E2 | 4.5E1 | 55 | 22 | 55 | 22 | 0.59 |
| cE | pg/mL | 4.6E1 | 6.5E1 | 1.9E2 | 2.4E2 | 6.5E2 | 3.4E2 | 1.2E-1 | 1.2E-1 | 3.8E3 | 1.3E3 | 55 | 22 | 55 | 22 | 0.65 |
| cF | pg/mL | 9.4E0 | 5.0E0 | 2.0E1 | 1.1E1 | 4.6E1 | 1.4E1 | 5.3E-1 | 5.3E-1 | 2.7E2 | 5.0E1 | 55 | 22 | 55 | 22 | 0.47 |
| cG | pg/mL | 4.6E1 | 6.6E1 | 2.9E2 | 9.9E1 | 1.4E3 | 7.6E1 | 7.8E0 | 7.8E0 | 1.0E4 | 2.6E2 | 55 | 22 | 55 | 22 | 0.64 |
| cH | uIU/mL | 4.7E0 | 2.6E0 | 1.0E1 | 5.9E0 | 1.8E1 | 1.2E1 | 8.6E-3 | 8.6E-3 | 1.2E2 | 5.3E1 | 55 | 22 | 55 | 22 | 0.36 |
| cI | ng/mL | 6.0E0 | 9.9E0 | 1.5E1 | 1.9E1 | 1.9E1 | 2.5E1 | 7.1E-2 | 5.1E-1 | 6.6E1 | 9.4E1 | 55 | 22 | 55 | 22 | 0.57 |
| cJ | ug/mL | 7.2E1 | 5.6E1 | 1.4E2 | 1.1E2 | 1.6E2 | 1.5E2 | 1.1E1 | 7.2E0 | 6.9E2 | 6.2E2 | 55 | 22 | 55 | 22 | 0.44 |
| cK | ng/mL | 3.8E-3 | 3.8E-3 | 3.2E-2 | 3.8E-3 | 2.0E-1 | 0.0E0 | 3.8E-3 | 3.8E-3 | 1.5E0 | 3.8E-3 | 55 | 22 | 55 | 22 | 0.47 |
| cL | pg/mL | 1.7E2 | 2.0E2 | 7.7E2 | 6.0E2 | 3.3E3 | 1.5E3 | 1.6E1 | 3.1E1 | 2.4E4 | 7.4E3 | 55 | 22 | 55 | 22 | 0.58 |
| cM | pg/mL | 2.5E2 | 2.8E2 | 2.8E2 | 2.9E2 | 2.2E2 | 1.1E2 | 3.3E1 | 4.2E1 | 1.5E3 | 4.5E2 | 55 | 22 | 55 | 22 | 0.59 |
| cN | pg/mL | 1.2E2 | 1.2E2 | 1.4E2 | 1.3E2 | 1.3E2 | 3.5E1 | 4.3E1 | 7.3E1 | 1.1E3 | 2.1E2 | 55 | 22 | 55 | 22 | 0.53 |
| cO | pg/mL | 2.0E2 | 2.4E2 | 5.7E2 | 2.4E2 | 2.6E3 | 9.0E1 | 6.1E1 | 8.2E1 | 1.9E4 | 4.3E2 | 55 | 22 | 55 | 22 | 0.58 |
| cP | ng/mL | 2.8E3 | 2.3E3 | 2.9E3 | 2.5E3 | 9.7E2 | 8.0E2 | 1.1E3 | 1.4E3 | 5.5E3 | 4.5E3 | 55 | 22 | 55 | 22 | 0.38 |
| cQ | ng/mL | 6.5E-2 | 3.6E-2 | 1.6E-1 | 1.6E-1 | 2.5E-1 | 3.1E-1 | 2.0E-3 | 2.0E-3 | 1.4E0 | 1.3E0 | 55 | 22 | 55 | 22 | 0.43 |
| cR | ng/mL | 2.8E2 | 5.4E2 | 4.2E2 | 6.2E2 | 4.4E2 | 4.8E2 | 3.0E1 | 3.6E1 | 2.3E3 | 2.0E3 | 55 | 22 | 55 | 22 | 0.66 |
| cS | ng/mL | 2.7E2 | 2.5E2 | 3.2E2 | 4.8E2 | 2.1E2 | 5.9E2 | 7.0E1 | 1.1E2 | 9.4E2 | 2.6E3 | 55 | 22 | 55 | 22 | 0.51 |
| cT | ng/mL | 3.9E1 | 6.2E1 | 1.5E2 | 1.8E2 | 3.3E2 | 3.5E2 | 7.2E0 | 4.2E0 | 2.1E3 | 1.3E3 | 55 | 22 | 55 | 22 | 0.54 |
| cU | ng/mL | 7.0E1 | 6.3E1 | 1.1E2 | 1.0E2 | 2.2E2 | 9.6E1 | 1.4E1 | 1.7E1 | 1.6E3 | 4.2E2 | 55 | 22 | 55 | 22 | 0.53 |
| cV | ng/mL | 1.8E-1 | 2.4E-1 | 2.8E-1 | 8.4E-1 | 4.2E-1 | 2.0E0 | 3.7E-2 | 4.1E-2 | 2.5E0 | 9.7E0 | 55 | 22 | 55 | 22 | 0.67 |
| cW | mIU/mL | 4.2E-2 | 4.0E-2 | 6.3E-2 | 5.2E-2 | 5.9E-2 | 3.7E-2 | 1.0E-2 | 4.8E-3 | 2.7E-1 | 1.5E-1 | 55 | 22 | 55 | 22 | 0.50 |
| cX | ng/mL | 1.4E-1 | 1.2E-1 | 2.0E0 | 2.0E0 | 5.5E0 | 5.9E0 | 2.3E-4 | 2.3E-4 | 2.8E1 | 2.8E1 | 55 | 22 | 55 | 22 | 0.45 |
| cY | ng/mL | 7.4E0 | 8.0E0 | 1.3E1 | 8.2E0 | 1.4E1 | 4.2E0 | 1.5E-1 | 9.8E-1 | 6.1E1 | 1.8E1 | 55 | 22 | 55 | 22 | 0.48 |
| cZ | ug/mL | 1.5E1 | 1.2E1 | 1.6E1 | 1.3E1 | 7.1E0 | 6.3E0 | 3.1E0 | 4.5E0 | 3.7E1 | 3.1E1 | 55 | 22 | 55 | 22 | 0.35 |
| dA | pg/mL | 3.4E2 | 3.0E2 | 4.7E2 | 3.2E2 | 7.6E2 | 1.3E2 | 1.3E2 | 1.5E2 | 5.8E3 | 5.5E2 | 55 | 22 | 55 | 22 | 0.41 |
| dB | ug/mL | 3.9E0 | 1.9E1 | 1.2E1 | 1.6E1 | 1.1E1 | 9.9E0 | 1.8E0 | 2.2E0 | 4.0E1 | 3.0E1 | 55 | 22 | 55 | 22 | 0.59 |
| dC | nmol/L | 3.3E1 | 3.7E1 | 3.7E1 | 3.7E1 | 1.4E1 | 1.5E1 | 1.5E1 | 7.8E0 | 8.7E1 | 6.9E1 | 55 | 22 | 55 | 22 | 0.54 |
| dD | ug/mL | 3.6E1 | 2.8E1 | 3.6E1 | 3.1E1 | 1.1E1 | 1.1E1 | 1.3E1 | 1.4E1 | 5.7E1 | 4.8E1 | 55 | 22 | 55 | 22 | 0.35 |
| dE | ng/mL | 8.4E-3 | 8.4E-3 | 3.4E-1 | 2.5E-1 | 5.2E-1 | 3.6E-1 | 8.4E-3 | 8.4E-3 | 2.0E0 | 1.4E0 | 55 | 22 | 55 | 22 | 0.50 |
| dF | ng/mL | 2.5E2 | 3.1E2 | 2.9E2 | 4.0E2 | 1.9E2 | 3.1E2 | 8.6E1 | 7.5E1 | 1.3E3 | 1.2E3 | 55 | 22 | 55 | 22 | 0.63 |
| dG | ng/mL | 1.2E1 | 1.4E1 | 1.6E1 | 1.7E1 | 2.4E1 | 1.1E1 | 3.3E0 | 3.0E0 | 1.8E2 | 5.8E1 | 55 | 22 | 55 | 22 | 0.60 |
| dH | pg/mL | 7.5E0 | 8.6E0 | 2.2E1 | 1.2E1 | 8.9E1 | 1.1E1 | 4.0E-2 | 4.0E-2 | 6.7E2 | 4.9E1 | 55 | 22 | 55 | 22 | 0.59 |
| dI | pg/mL | 4.6E-1 | 4.6E-1 | 6.9E0 | 1.2E0 | 4.4E1 | 2.2E0 | 4.6E-1 | 4.6E-1 | 3.3E2 | 1.0E1 | 55 | 22 | 55 | 22 | 0.50 |
| dJ | ng/mL | 2.3E0 | 1.8E0 | 2.3E0 | 1.8E0 | 1.2E0 | 6.7E-1 | 3.7E-1 | 3.7E-1 | 5.1E0 | 2.9E0 | 55 | 22 | 55 | 22 | 0.37 |
| dK | uIU/mL | 1.6E0 | 1.1E0 | 2.5E0 | 2.2E0 | 3.6E0 | 2.2E0 | 4.0E-2 | 1.9E-1 | 2.1E1 | 7.3E0 | 55 | 22 | 55 | 22 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dL | ng/mL | 8.8E2 | 1.2E3 | 1.1E3 | 1.2E3 | 5.4E2 | 6.3E2 | 4.7E2 | 2.8E2 | 3.2E3 | 3.3E3 | 55 | 22 | 55 | 22 | 0.57 |
| dM | pg/mL | 1.1E3 | 1.1E3 | 1.1E3 | 1.3E3 | 5.9E2 | 6.7E2 | 4.2E2 | 3.7E2 | 3.3E3 | 3.1E3 | 55 | 22 | 55 | 22 | 0.56 |
| dN | ug/mL | 9.8E1 | 8.9E1 | 1.0E2 | 1.0E2 | 3.1E1 | 4.7E1 | 3.7E1 | 2.4E1 | 1.9E2 | 2.1E2 | 55 | 22 | 55 | 22 | 0.45 |
| dR | pg/ml | 1.7E3 | 1.2E3 | 2.3E3 | 1.6E3 | 2.2E3 | 1.4E3 | 1.7E2 | 3.4E2 | 9.8E3 | 5.3E3 | 54 | 21 | 54 | 21 | 0.42 |
| dU | pg/ml | 1.4E4 | 1.8E4 | 1.6E4 | 2.5E4 | 9.1E3 | 2.5E4 | 6.7E3 | 3.1E3 | 3.5E4 | 8.1E4 | 7 | 9 | 7 | 9 | 0.57 |
| eF | ng/ml | 4.3E0 | 4.0E0 | 4.7E0 | 4.7E0 | 2.0E0 | 1.9E0 | 1.6E0 | 2.0E0 | 1.2E1 | 8.7E0 | 55 | 21 | 55 | 21 | 0.48 |
| eC | pg/ml | 3.3E2 | 2.7E2 | 3.6E2 | 2.9E2 | 1.5E2 | 2.0E2 | 5.1E1 | 1.9E1 | 7.5E2 | 7.3E2 | 51 | 17 | 51 | 17 | 0.37 |
| eD | pg/ml | 2.1E2 | 2.7E2 | 6.1E2 | 9.3E2 | 1.3E3 | 1.3E3 | 5.2E-1 | 1.4E2 | 7.0E3 | 3.8E3 | 43 | 12 | 43 | 12 | 0.66 |
| eM | ng/ml | 3.3E0 | 2.8E0 | 5.8E0 | 2.8E0 | 5.3E0 | 1.9E0 | 1.5E0 | 6.9E-1 | 1.8E1 | 7.3E0 | 15 | 12 | 15 | 12 | 0.31 |
| eQ | pg/ml | 1.0E0 | 1.0E0 | 6.5E0 | 2.8E1 | 1.5E1 | 5.4E1 | 1.0E0 | 1.0E0 | 4.0E1 | 1.5E2 | 7 | 9 | 7 | 9 | 0.56 |
| fA | ng/ml | 2.4E2 | 1.1E2 | 3.1E2 | 3.1E2 | 2.5E2 | 4.0E2 | 6.8E1 | 7.0E1 | 8.3E2 | 1.2E3 | 7 | 7 | 7 | 7 | 0.39 |
| fB | ng/ml | 7.4E2 | 7.3E2 | 8.3E2 | 8.3E2 | 4.3E2 | 2.5E2 | 3.8E2 | 5.8E2 | 1.5E3 | 1.3E3 | 7 | 8 | 7 | 8 | 0.55 |
| fP | ng/ml | 2.7E2 | 2.8E2 | 3.0E2 | 3.6E2 | 1.6E2 | 2.0E2 | 2.7E1 | 1.3E2 | 8.7E2 | 8.6E2 | 52 | 21 | 52 | 21 | 0.58 |
| fR | ng/ml | 1.4E5 | 2.0E5 | 1.8E5 | 2.4E5 | 1.6E5 | 1.4E5 | 3.6E4 | 9.8E4 | 7.2E5 | 4.8E5 | 25 | 11 | 25 | 11 | 0.65 |
| gC | ng/ml | 2.0E2 | 2.8E2 | 2.5E2 | 3.0E2 | 1.3E2 | 8.8E1 | 1.3E2 | 1.8E2 | 5.9E2 | 4.5E2 | 16 | 11 | 16 | 11 | 0.68 |
| gL | pg/ml | 6.2E4 | 6.0E4 | 7.0E4 | 6.3E4 | 2.8E4 | 3.1E4 | 3.1E4 | 1.1E4 | 1.6E5 | 1.7E5 | 54 | 21 | 54 | 21 | 0.43 |
| gP | U/ml | 3.1E2 | 2.4E2 | 3.2E2 | 2.6E2 | 1.2E2 | 9.7E1 | 7.1E1 | 9.6E1 | 8.5E2 | 5.2E2 | 54 | 21 | 54 | 21 | 0.34 |
| gW | ng/ml | 5.3E2 | 5.2E2 | 8.6E2 | 1.1E3 | 1.0E3 | 1.3E3 | 2.3E0 | 1.9E2 | 4.2E3 | 4.3E3 | 47 | 12 | 47 | 12 | 0.58 |
| tF | pg/mL | 8.8E2 | 2.5E3 | 1.1E4 | 8.4E3 | 3.7E4 | 1.6E4 | 1.2E1 | 1.8E1 | 2.5E5 | 6.9E4 | 51 | 19 | 51 | 19 | 0.60 |
| gZ | ug/ml | 1.1E0 | 1.2E0 | 5.3E0 | 6.6E1 | 9.1E0 | 1.3E2 | 1.1E-1 | 4.3E-1 | 2.5E1 | 3.8E2 | 7 | 9 | 7 | 9 | 0.52 |
| hA | ng/ml | 2.1E0 | 4.2E0 | 1.4E1 | 6.6E0 | 4.7E1 | 5.5E0 | 1.7E-2 | 1.7E-2 | 2.9E2 | 1.6E1 | 43 | 13 | 43 | 13 | 0.67 |
| nM | pg/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 26 | 16 | 26 | 16 | 0.50 |
| nN | pg/ml | 1.4E3 | 2.2E3 | 6.0E3 | 4.0E3 | 2.0E4 | 5.0E3 | 1.2E2 | 2.8E2 | 1.0E5 | 2.1E4 | 26 | 16 | 26 | 16 | 0.65 |
| nO | pg/ml | 2.8E1 | 2.5E1 | 4.2E1 | 4.2E1 | 3.9E1 | 5.7E1 | 3.5E0 | 9.7E0 | 1.5E2 | 2.4E2 | 26 | 16 | 26 | 16 | 0.49 |
| nR | pg/ml | 1.3E1 | 2.1E1 | 5.7E1 | 1.4E2 | 1.6E2 | 2.4E2 | 5.5E-1 | 1.0E0 | 8.2E2 | 7.1E2 | 26 | 16 | 26 | 16 | 0.63 |
| nT | pg/ml | 8.2E1 | 8.9E1 | 1.1E2 | 9.6E1 | 1.3E2 | 3.5E1 | 1.0E-9 | 5.7E1 | 6.4E2 | 1.9E2 | 26 | 16 | 26 | 16 | 0.53 |
| nU | pg/ml | 3.0E1 | 3.8E1 | 1.1E2 | 9.1E1 | 2.9E2 | 1.2E2 | 1.0E-9 | 1.0E-9 | 1.5E3 | 4.4E2 | 26 | 16 | 26 | 16 | 0.53 |
| lW | pg/ml | 1.0E-9 | 1.0E-9 | 9.5E0 | 5.8E0 | 3.3E1 | 1.8E1 | 1.0E-9 | 1.0E-9 | 1.6E2 | 7.0E1 | 26 | 16 | 26 | 16 | 0.42 |
| lX | pg/ml | 1.1E3 | 6.9E2 | 1.1E3 | 8.1E2 | 5.7E2 | 4.1E2 | 1.2E2 | 1.9E2 | 2.3E3 | 1.6E3 | 26 | 16 | 26 | 16 | 0.34 |
| lY | pg/ml | 2.7E1 | 1.9E1 | 3.1E1 | 1.7E1 | 2.5E1 | 9.3E0 | 4.2E0 | 3.7E0 | 1.2E2 | 3.0E1 | 26 | 16 | 26 | 16 | 0.27 |
| mE | pg/ml | 1.0E-9 | 1.0E-9 | 3.6E0 | 1.8E0 | 1.1E1 | 3.2E0 | 1.0E-9 | 1.0E-9 | 5.7E1 | 9.2E0 | 26 | 16 | 26 | 16 | 0.48 |
| mF | pg/ml | 1.0E-9 | 2.7E-1 | 5.2E0 | 2.1E0 | 2.1E1 | 4.0E0 | 1.0E-9 | 1.0E-9 | 1.1E2 | 1.3E1 | 26 | 16 | 26 | 16 | 0.55 |
| mH | pg/ml | 3.2E0 | 2.7E0 | 5.5E0 | 4.4E0 | 1.0E1 | 4.8E0 | 3.2E-1 | 9.0E-1 | 5.3E1 | 1.9E1 | 26 | 16 | 26 | 16 | 0.45 |
| mI | pg/ml | 1.0E-9 | 1.0E-9 | 1.5E1 | 1.3E1 | 3.7E1 | 2.4E1 | 1.0E-9 | 1.0E-9 | 1.8E2 | 7.8E1 | 26 | 16 | 26 | 16 | 0.48 |
| mM | pg/ml | 3.6E1 | 4.3E1 | 9.1E1 | 5.7E1 | 2.2E2 | 7.4E1 | 1.0E-9 | 1.0E-9 | 1.1E3 | 2.9E2 | 26 | 16 | 26 | 16 | 0.52 |
| mP | pg/ml | 1.5E1 | 1.5E1 | 1.8E1 | 1.5E1 | 2.2E1 | 9.7E0 | 1.0E-9 | 1.0E-9 | 1.2E2 | 3.8E1 | 26 | 16 | 26 | 16 | 0.51 |
| mS | pg/ml | 1.8E3 | 1.2E3 | 1.8E3 | 1.5E3 | 8.9E2 | 1.1E3 | 1.0E-9 | 1.0E-9 | 4.6E3 | 5.1E3 | 26 | 16 | 26 | 16 | 0.33 |
| mT | pg/ml | 4.3E1 | 5.9E1 | 1.0E2 | 6.6E1 | 1.9E2 | 3.8E1 | 1.6E1 | 1.9E1 | 9.7E2 | 1.4E2 | 26 | 16 | 26 | 16 | 0.54 |
| mU | pg/ml | 2.0E0 | 3.2E0 | 1.1E1 | 3.5E0 | 4.3E1 | 2.0E0 | 1.9E-1 | 1.0E-9 | 2.2E2 | 7.7E0 | 26 | 16 | 26 | 16 | 0.65 |
| mW | pg/ml | 2.5E3 | 2.2E3 | 2.9E3 | 2.2E3 | 1.3E3 | 1.3E3 | 1.1E3 | 3.7E2 | 5.6E3 | 5.7E3 | 26 | 16 | 26 | 16 | 0.38 |
| mY | pg/ml | 6.3E2 | 6.9E2 | 8.0E2 | 8.7E2 | 5.9E2 | 7.2E2 | 2.1E2 | 2.5E2 | 2.7E3 | 2.6E3 | 26 | 16 | 26 | 16 | 0.50 |
| mZ | pg/ml | 2.3E2 | 2.1E2 | 4.0E2 | 3.6E2 | 3.9E2 | 3.7E2 | 2.8E1 | 1.1E1 | 1.7E3 | 1.1E3 | 26 | 16 | 26 | 16 | 0.43 |
| nA | pg/ml | 1.7E0 | 4.8E-1 | 7.3E0 | 5.8E0 | 1.4E1 | 1.6E1 | 1.0E-9 | 1.0E-9 | 6.2E1 | 6.5E1 | 26 | 16 | 26 | 16 | 0.36 |
| nB | pg/ml | 3.2E2 | 3.3E2 | 3.6E2 | 3.6E2 | 1.7E2 | 1.8E2 | 9.7E1 | 3.8E1 | 8.2E2 | 6.9E2 | 26 | 16 | 26 | 16 | 0.51 |
| nC | pg/ml | 1.0E-9 | 3.7E1 | 8.7E2 | 1.1E5 | 3.4E3 | 3.8E5 | 1.0E-9 | 1.0E-9 | 1.7E4 | 1.5E6 | 26 | 16 | 26 | 16 | 0.57 |
| nD | pg/ml | 8.2E0 | 5.8E0 | 8.2E0 | 2.4E1 | 7.0E0 | 6.4E1 | 1.0E-9 | 1.0E-9 | 3.0E1 | 2.6E2 | 26 | 16 | 26 | 16 | 0.47 |
| nF | pg/ml | 1.0E-9 | 1.0E-9 | 1.2E0 | 1.7E0 | 4.4E0 | 6.9E0 | 1.0E-9 | 1.0E-9 | 2.1E1 | 2.7E1 | 26 | 16 | 26 | 16 | 0.50 |
| nH | pg/ml | 2.0E-1 | 8.8E-2 | 1.2E1 | 7.9E2 | 4.9E1 | 2.5E3 | 1.0E-9 | 1.0E-9 | 2.5E2 | 1.0E4 | 26 | 16 | 26 | 16 | 0.52 |
| nI | pg/ml | 1.7E1 | 4.8E1 | 6.0E1 | 6.3E1 | 7.6E1 | 9.0E1 | 1.0E-9 | 1.0E-9 | 2.6E2 | 3.5E2 | 26 | 16 | 26 | 16 | 0.48 |
| nJ | pg/ml | 3.0E-2 | 1.7E-1 | 1.3E0 | 8.7E0 | 3.2E0 | 3.3E1 | 1.0E-9 | 1.0E-9 | 1.6E1 | 1.3E2 | 26 | 16 | 26 | 16 | 0.50 |
| nK | pg/ml | 1.0E-9 | 1.0E-9 | 2.1E1 | 1.4E1 | 4.5E1 | 2.7E1 | 1.0E-9 | 1.0E-9 | 2.2E2 | 1.0E2 | 26 | 16 | 26 | 16 | 0.48 |
| nL | pg/ml | 1.0E-9 | 1.2E0 | 5.5E1 | 1.2E3 | 2.3E2 | 3.5E3 | 1.0E-9 | 1.0E-9 | 1.2E3 | 1.4E4 | 26 | 16 | 26 | 16 | 0.58 |
| hR | pg/ml | 2.4E4 | 2.4E4 | 2.4E4 | 2.6E4 | 9.0E3 | 9.9E3 | 3.6E3 | 1.6E4 | 4.9E4 | 4.3E4 | 42 | 12 | 42 | 12 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| hV | pg/ml | 4.3E2 | 3.7E2 | 4.6E2 | 4.0E2 | 2.3E2 | 1.8E2 | 6.8E1 | 1.5E2 | 9.6E2 | 7.4E2 | 42 | 12 | 42 | 12 | 0.44 |
| hW | pg/ml | 1.6E3 | 2.0E3 | 2.3E3 | 2.2E3 | 2.0E3 | 8.0E2 | 2.2E2 | 1.1E3 | 1.0E4 | 3.5E3 | 42 | 12 | 42 | 12 | 0.61 |
| hX | pg/ml | 8.9E2 | 1.0E3 | 1.0E3 | 1.2E3 | 7.5E2 | 6.7E2 | 1.3E2 | 3.1E2 | 4.9E3 | 2.9E3 | 42 | 12 | 42 | 12 | 0.63 |
| iA | pg/ml | 1.3E2 | 1.5E2 | 1.8E2 | 2.7E2 | 1.7E2 | 2.9E2 | 5.8E0 | 1.5E1 | 7.9E2 | 8.7E2 | 51 | 19 | 51 | 19 | 0.55 |
| iB | ng/ml | 4.2E0 | 7.8E0 | 5.1E0 | 8.6E0 | 3.9E0 | 5.5E0 | 4.5E-2 | 1.6E0 | 2.0E1 | 1.9E1 | 43 | 13 | 43 | 13 | 0.70 |
| iC | U/ml | 2.0E-1 | 3.5E-1 | 4.5E-1 | 1.5E0 | 8.3E-1 | 3.3E0 | 1.0E-9 | 6.8E-2 | 4.8E0 | 1.2E1 | 43 | 13 | 43 | 13 | 0.64 |
| iH | ng/ml | 1.4E5 | 1.6E5 | 1.5E5 | 1.5E5 | 5.0E4 | 5.6E4 | 7.2E4 | 2.9E3 | 2.6E5 | 2.4E5 | 51 | 19 | 51 | 19 | 0.53 |
| iJ | ng/ml | 5.7E4 | 4.9E4 | 5.6E4 | 5.2E4 | 3.6E4 | 2.4E4 | 8.0E3 | 1.8E3 | 2.5E5 | 1.0E5 | 51 | 19 | 51 | 19 | 0.48 |
| hB | ng/ml | 3.7E-1 | 5.1E-1 | 4.6E-1 | 7.1E-1 | 2.7E-1 | 6.6E-1 | 1.2E-1 | 1.2E-1 | 1.3E0 | 3.2E0 | 51 | 19 | 51 | 19 | 0.69 |
| hC | pg/ml | 4.5E3 | 7.5E3 | 9.5E3 | 8.3E3 | 1.6E4 | 7.9E3 | 1.0E-9 | 4.1E1 | 1.1E5 | 2.7E4 | 51 | 19 | 51 | 19 | 0.50 |
| hF | ng/ml | 1.0E-9 | 1.0E-9 | 1.3E-2 | 5.0E-1 | 9.2E-2 | 2.2E0 | 1.0E-9 | 1.0E-9 | 6.6E-1 | 9.6E0 | 51 | 19 | 51 | 19 | 0.52 |
| hG | pg/ml | 6.8E3 | 6.6E3 | 7.6E3 | 6.8E3 | 3.6E3 | 2.4E3 | 2.3E3 | 3.6E3 | 1.9E4 | 1.2E4 | 51 | 19 | 51 | 19 | 0.44 |
| iO | ng/ml | 3.2E5 | 4.6E5 | 3.7E5 | 4.7E5 | 1.8E5 | 1.9E5 | 8.3E4 | 1.8E5 | 9.0E5 | 8.2E5 | 51 | 19 | 51 | 19 | 0.65 |
| iP | ng/ml | 4.5E4 | 5.3E4 | 5.0E4 | 5.0E4 | 3.8E4 | 2.2E4 | 2.4E3 | 1.0E4 | 2.2E5 | 7.7E4 | 51 | 19 | 51 | 19 | 0.54 |
| iZ | ng/ml | 1.7E3 | 1.9E3 | 1.9E3 | 1.8E3 | 8.2E2 | 6.1E2 | 8.8E2 | 9.8E2 | 4.5E3 | 2.7E3 | 50 | 17 | 50 | 17 | 0.48 |
| jB | ng/ml | 2.7E5 | 1.9E5 | 2.6E5 | 2.4E5 | 8.0E4 | 1.6E5 | 1.3E5 | 1.2E5 | 3.9E5 | 6.2E5 | 7 | 9 | 7 | 9 | 0.33 |
| rC | pg/ml | 1.5E3 | 1.1E3 | 2.3E3 | 1.3E3 | 2.7E3 | 1.1E3 | 1.0E-9 | 7.0E1 | 1.5E4 | 3.8E3 | 42 | 12 | 42 | 12 | 0.39 |
| rB | pg/ml | 2.5E1 | 4.0E1 | 3.9E1 | 5.9E1 | 4.8E1 | 7.1E1 | 1.0E-9 | 1.0E-9 | 2.9E2 | 2.5E2 | 42 | 12 | 42 | 12 | 0.58 |
| jD | ng/ml | 4.1E1 | 4.5E1 | 4.7E1 | 4.0E1 | 3.9E1 | 3.2E1 | 1.0E-9 | 1.0E-9 | 1.5E2 | 9.9E1 | 43 | 13 | 43 | 13 | 0.46 |
| jE | ng/ml | 1.0E-9 | 1.0E-9 | 6.9E0 | 5.2E0 | 1.2E1 | 1.5E1 | 1.0E-9 | 1.0E-9 | 5.2E1 | 5.6E1 | 43 | 13 | 43 | 13 | 0.46 |
| jF | ng/ml | 3.7E1 | 3.8E1 | 5.8E1 | 3.6E1 | 6.4E1 | 3.5E1 | 1.0E-9 | 1.0E-9 | 1.9E2 | 1.1E2 | 43 | 13 | 43 | 13 | 0.45 |
| jG | ng/ml | 4.8E3 | 4.5E3 | 4.7E3 | 4.5E3 | 2.0E3 | 1.9E3 | 1.2E3 | 6.0E2 | 9.6E3 | 7.1E3 | 43 | 13 | 43 | 13 | 0.49 |
| jH | ng/ml | 7.9E1 | 7.1E1 | 9.5E1 | 6.2E1 | 6.3E1 | 3.0E1 | 2.5E1 | 1.5E1 | 3.3E2 | 1.2E2 | 43 | 13 | 43 | 13 | 0.34 |
| jI | ng/ml | 6.9E1 | 9.9E1 | 7.6E1 | 9.5E1 | 3.0E1 | 4.0E1 | 3.5E1 | 5.0E1 | 1.9E2 | 1.7E2 | 43 | 13 | 43 | 13 | 0.65 |
| rA | pg/ml | 2.7E1 | 3.0E1 | 3.2E1 | 2.7E1 | 2.5E1 | 1.3E1 | 1.0E-9 | 6.9E0 | 1.1E2 | 4.8E1 | 43 | 13 | 43 | 13 | 0.48 |
| qZ | pg/ml | 4.4E1 | 4.9E1 | 6.9E2 | 1.1E3 | 2.5E3 | 3.1E3 | 4.8E-4 | 4.0E-3 | 1.0E4 | 1.0E4 | 31 | 10 | 31 | 10 | 0.53 |
| qY | pg/ml | 2.3E1 | 1.3E1 | 5.5E1 | 2.8E1 | 6.7E1 | 4.6E1 | 2.9E0 | 5.1E0 | 3.3E2 | 1.8E2 | 43 | 13 | 43 | 13 | 0.33 |
| qX | pg/ml | 5.9E1 | 6.8E1 | 7.0E1 | 7.4E1 | 4.5E1 | 3.7E1 | 1.0E-9 | 1.7E1 | 2.1E2 | 1.3E2 | 43 | 13 | 43 | 13 | 0.57 |
| qW | pg/ml | 8.3E0 | 1.0E1 | 1.3E1 | 1.1E1 | 1.5E1 | 8.9E0 | 1.0E-9 | 1.0E-9 | 8.1E1 | 3.1E1 | 43 | 13 | 43 | 13 | 0.52 |
| qV | pg/ml | 1.9E3 | 2.4E3 | 2.6E3 | 2.9E3 | 2.0E3 | 2.3E3 | 1.0E2 | 9.5E2 | 8.2E3 | 9.6E3 | 43 | 13 | 43 | 13 | 0.56 |
| qU | pg/ml | 7.0E1 | 1.2E2 | 1.7E2 | 2.0E2 | 3.4E2 | 2.3E2 | 1.0E-9 | 1.0E-9 | 2.1E3 | 7.9E2 | 43 | 13 | 43 | 13 | 0.59 |
| qT | pg/ml | 4.0E1 | 8.2E1 | 5.6E1 | 9.5E1 | 4.4E1 | 7.3E1 | 1.0E-9 | 6.9E0 | 1.8E2 | 2.6E2 | 43 | 13 | 43 | 13 | 0.67 |
| jK | ng/ml | 1.7E3 | 1.4E3 | 1.8E3 | 1.5E3 | 8.0E2 | 7.6E2 | 6.4E2 | 8.0E2 | 4.1E3 | 3.6E3 | 43 | 13 | 43 | 13 | 0.35 |
| jL | ng/ml | 2.3E2 | 2.1E2 | 3.3E2 | 2.8E2 | 3.5E2 | 1.8E2 | 4.8E1 | 1.3E2 | 2.1E3 | 7.0E2 | 43 | 13 | 43 | 13 | 0.50 |
| jM | ng/ml | 8.2E4 | 5.2E4 | 8.0E4 | 5.9E4 | 4.4E4 | 3.3E4 | 1.1E3 | 5.7E3 | 1.7E5 | 1.2E5 | 43 | 13 | 43 | 13 | 0.35 |
| jO | pg/ml | 2.3E5 | 2.6E5 | 2.5E5 | 3.1E5 | 1.1E5 | 1.5E5 | 7.7E4 | 1.4E5 | 5.7E5 | 6.4E5 | 43 | 13 | 43 | 13 | 0.62 |
| jP | pg/ml | 2.4E5 | 2.8E5 | 2.6E5 | 3.0E5 | 1.6E5 | 1.4E5 | 5.8E4 | 1.3E5 | 7.2E5 | 5.8E5 | 43 | 13 | 43 | 13 | 0.61 |
| jQ | pg/ml | 2.9E3 | 1.7E3 | 4.0E3 | 2.3E3 | 3.6E3 | 1.6E3 | 1.4E2 | 5.1E2 | 1.4E4 | 6.1E3 | 43 | 13 | 43 | 13 | 0.40 |
| jR | pg/ml | 7.0E3 | 3.4E3 | 1.2E4 | 7.4E3 | 1.2E4 | 9.8E3 | 1.0E-9 | 3.0E1 | 5.6E4 | 3.4E4 | 43 | 13 | 43 | 13 | 0.38 |
| jT | pg/ml | 1.9E5 | 1.6E5 | 1.9E5 | 1.8E5 | 7.5E4 | 7.7E4 | 7.7E4 | 7.5E4 | 3.8E5 | 3.5E5 | 43 | 13 | 43 | 13 | 0.42 |
| jU | mIU/ml | 4.8E0 | 4.1E0 | 9.0E0 | 1.1E1 | 1.3E1 | 1.5E1 | 6.2E-2 | 2.7E-1 | 6.7E1 | 5.3E1 | 43 | 13 | 43 | 13 | 0.49 |
| jV | mIU/ml | 1.5E0 | 1.1E0 | 3.2E0 | 2.2E0 | 5.5E0 | 2.9E0 | 1.7E-3 | 7.6E-2 | 2.6E1 | 1.0E1 | 43 | 13 | 43 | 13 | 0.47 |
| jY | ng/ml | 5.9E-4 | 2.7E-3 | 4.0E-3 | 6.3E-3 | 9.2E-3 | 7.9E-3 | 1.0E-9 | 1.0E-9 | 5.1E-2 | 2.4E-2 | 43 | 13 | 43 | 13 | 0.67 |
| kC | pg/ml | 8.9E1 | 8.8E1 | 1.7E2 | 2.9E2 | 2.1E2 | 6.7E2 | 2.1E1 | 3.6E1 | 9.8E2 | 2.7E3 | 26 | 16 | 26 | 16 | 0.45 |
| kE | pg/ml | 1.4E5 | 1.3E5 | 1.4E5 | 1.3E5 | 4.1E4 | 5.7E4 | 4.1E4 | 5.5E4 | 2.1E5 | 2.7E5 | 26 | 16 | 26 | 16 | 0.42 |
| kF | pg/mL | 6.5E1 | 7.3E1 | 6.7E1 | 7.3E1 | 2.7E1 | 2.7E1 | 2.8E1 | 3.4E1 | 1.5E2 | 1.3E2 | 26 | 16 | 26 | 16 | 0.58 |
| kG | pg/mL | 1.0E4 | 1.1E4 | 1.5E4 | 1.4E4 | 1.7E4 | 1.2E4 | 3.3E3 | 3.8E3 | 9.1E4 | 5.0E4 | 26 | 16 | 26 | 16 | 0.51 |
| kI | pg/ml | 1.9E2 | 1.9E2 | 2.0E2 | 1.9E2 | 9.3E1 | 9.5E1 | 4.4E1 | 1.0E-9 | 4.6E2 | 3.5E2 | 26 | 16 | 26 | 16 | 0.49 |
| kK | pg/ml | 9.3E1 | 1.0E2 | 1.3E2 | 1.9E2 | 1.1E2 | 1.8E2 | 2.9E1 | 2.9E1 | 4.6E2 | 5.9E2 | 26 | 16 | 26 | 16 | 0.56 |
| kN | pg/ml | 9.1E2 | 1.1E3 | 1.5E3 | 1.9E3 | 1.5E3 | 2.4E3 | 2.1E2 | 1.2E2 | 6.3E3 | 8.7E3 | 26 | 16 | 26 | 16 | 0.54 |
| kO | pg/ml | 7.2E3 | 5.9E3 | 7.6E3 | 1.6E4 | 3.0E3 | 3.5E4 | 3.4E3 | 3.8E3 | 1.8E4 | 1.5E5 | 26 | 16 | 26 | 16 | 0.43 |
| kP | pg/ml | 5.8E3 | 5.1E3 | 8.9E3 | 5.6E3 | 9.7E3 | 2.9E3 | 9.6E2 | 1.4E3 | 4.8E4 | 1.1E4 | 26 | 16 | 26 | 16 | 0.41 |
| kQ | pg/ml | 4.2E3 | 3.7E3 | 5.1E3 | 3.9E3 | 3.2E3 | 1.6E3 | 1.1E3 | 1.4E3 | 1.8E4 | 7.8E3 | 51 | 19 | 51 | 19 | 0.41 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| kR | pg/ml | 2.2E1 | 2.0E1 | 2.4E1 | 2.8E1 | 1.5E1 | 2.2E1 | 1.4E-1 | 3.4E0 | 5.9E1 | 8.2E1 | 51 | 19 | 51 | 19 | 0.52 |
| kS | pg/ml | 9.7E2 | 8.7E2 | 1.3E3 | 9.7E2 | 2.0E3 | 7.1E2 | 8.2E1 | 2.9E2 | 1.4E4 | 3.0E3 | 51 | 19 | 51 | 19 | 0.40 |
| rZ | ng/ml | 1.0E-9 | 5.7E-3 | 6.1E-3 | 8.6E-3 | 1.3E-2 | 1.2E-2 | 1.0E-9 | 1.0E-9 | 7.9E-2 | 4.5E-2 | 41 | 12 | 41 | 12 | 0.62 |
| rY | ng/ml | 6.1E-2 | 8.2E-2 | 5.7E-1 | 1.1E-1 | 3.1E0 | 1.2E-1 | 1.0E-9 | 2.0E-2 | 2.0E1 | 4.4E-1 | 41 | 12 | 41 | 12 | 0.62 |
| rX | ng/ml | 1.0E-9 | 1.0E-9 | 7.7E-2 | 1.3E-2 | 4.8E-1 | 3.2E-2 | 1.0E-9 | 1.0E-9 | 3.1E0 | 1.1E-1 | 41 | 12 | 41 | 12 | 0.64 |
| lK | pg/ml | 1.6E2 | 6.8E1 | 2.6E2 | 8.7E1 | 5.1E2 | 8.9E1 | 1.0E-9 | 4.8E0 | 3.3E3 | 2.9E2 | 43 | 13 | 43 | 13 | 0.36 |
| lL | pg/ml | 1.4E3 | 1.8E3 | 3.2E3 | 2.8E3 | 6.4E3 | 1.9E3 | 1.5E1 | 1.9E2 | 4.2E4 | 6.3E3 | 43 | 13 | 43 | 13 | 0.62 |
| lM | pg/ml | 1.1E3 | 1.2E3 | 4.1E3 | 3.5E3 | 7.5E3 | 4.5E3 | 2.1E2 | 9.5E0 | 3.4E4 | 1.3E4 | 43 | 13 | 43 | 13 | 0.48 |
| lN | pg/ml | 1.0E-9 | 4.7E0 | 2.9E0 | 6.7E0 | 4.8E0 | 7.6E0 | 1.0E-9 | 1.0E-9 | 2.4E1 | 2.1E1 | 43 | 13 | 43 | 13 | 0.62 |
| lO | pg/ml | 1.0E-9 | 1.0E-9 | 2.9E0 | 2.6E0 | 1.9E1 | 9.3E0 | 1.0E-9 | 1.0E-9 | 1.3E2 | 3.4E1 | 43 | 13 | 43 | 13 | 0.53 |
| nW | pg/ml | 1.1E5 | 1.1E5 | 1.1E5 | 1.1E5 | 2.4E4 | 3.0E4 | 5.9E4 | 3.6E4 | 1.5E5 | 1.5E5 | 51 | 19 | 51 | 19 | 0.51 |
| nY | pg/ml | 2.1E3 | 2.8E3 | 2.6E3 | 3.0E3 | 2.0E3 | 1.9E3 | 1.0E3 | 6.3E2 | 1.3E4 | 8.1E3 | 51 | 19 | 51 | 19 | 0.58 |
| oO | pg/ml | 8.1E4 | 9.9E4 | 1.2E5 | 1.0E5 | 9.4E4 | 4.8E4 | 3.5E4 | 2.0E4 | 3.3E5 | 2.0E5 | 25 | 15 | 25 | 15 | 0.55 |
| oP | pg/ml | 1.2E5 | 1.8E5 | 1.5E5 | 1.8E5 | 1.1E5 | 1.2E5 | 4.8E4 | 5.6E4 | 4.5E5 | 4.6E5 | 25 | 15 | 25 | 15 | 0.59 |
| oQ | pg/ml | 2.8E3 | 3.4E3 | 4.4E3 | 4.0E3 | 4.4E3 | 2.2E3 | 1.6E3 | 7.7E2 | 2.1E4 | 8.4E3 | 25 | 15 | 25 | 15 | 0.56 |
| oE | pg/ml | 1.3E2 | 5.8E2 | 4.2E2 | 9.1E2 | 6.0E2 | 9.8E2 | 1.0E-9 | 5.4E1 | 1.9E3 | 3.4E3 | 51 | 19 | 51 | 19 | 0.71 |
| oF | pg/ml | 1.3E4 | 1.8E4 | 3.1E4 | 3.7E4 | 5.2E4 | 4.8E4 | 6.9E2 | 5.1E2 | 2.5E5 | 1.7E5 | 51 | 19 | 51 | 19 | 0.55 |
| oH | pg/ml | 4.9E1 | 3.4E1 | 9.2E1 | 5.1E1 | 1.0E2 | 5.3E1 | 6.2E0 | 6.3E0 | 4.8E2 | 1.8E2 | 51 | 19 | 51 | 19 | 0.37 |
| oK | pg/ml | 9.1E2 | 6.6E2 | 1.6E3 | 1.7E3 | 1.7E3 | 1.9E3 | 1.4E2 | 2.3E2 | 7.2E3 | 6.5E3 | 51 | 19 | 51 | 19 | 0.50 |
| oN | pg/ml | 5.6E2 | 5.0E2 | 6.7E2 | 6.2E2 | 7.1E2 | 4.4E2 | 2.0E2 | 1.1E2 | 5.3E3 | 1.8E3 | 51 | 19 | 51 | 19 | 0.47 |
| oW | pg/ml | 2.0E2 | 3.8E2 | 2.4E2 | 7.2E2 | 1.5E2 | 9.0E2 | 8.5E1 | 2.9E1 | 5.4E2 | 2.7E3 | 7 | 9 | 7 | 9 | 0.68 |
| oT | pg/ml | 3.7E2 | 3.0E2 | 4.3E2 | 3.3E2 | 2.3E2 | 1.2E2 | 1.9E2 | 1.3E2 | 7.7E2 | 5.4E2 | 7 | 9 | 7 | 9 | 0.40 |
| oV | pg/ml | 8.8E1 | 9.0E1 | 1.7E2 | 1.6E2 | 1.9E2 | 1.9E2 | 4.7E1 | 2.2E1 | 5.8E2 | 6.3E2 | 7 | 9 | 7 | 9 | 0.44 |
| oD | pg/ml | 1.8E4 | 1.4E4 | 1.7E4 | 1.6E4 | 4.1E3 | 7.4E3 | 1.1E4 | 9.3E3 | 2.2E4 | 3.3E4 | 7 | 9 | 7 | 9 | 0.38 |
| pF | pg/ml | 5.6E-1 | 5.0E-1 | 2.3E0 | 7.5E-1 | 1.2E1 | 6.0E-1 | 1.0E-9 | 1.0E-9 | 8.7E1 | 1.7E0 | 51 | 19 | 51 | 19 | 0.54 |
| pH | ng/ml | 7.2E0 | 1.1E1 | 1.3E1 | 1.0E1 | 1.3E1 | 5.4E0 | 4.7E0 | 1.2E0 | 4.2E1 | 2.0E1 | 7 | 9 | 7 | 9 | 0.57 |
| pI | ng/ml | 7.9E1 | 7.1E1 | 9.8E1 | 6.9E1 | 4.8E1 | 3.4E1 | 5.6E1 | 2.3E1 | 2.0E2 | 1.2E2 | 7 | 9 | 7 | 9 | 0.35 |
| pK | ng/ml | 4.6E-1 | 5.8E-1 | 5.7E-1 | 6.0E-1 | 2.4E-1 | 3.4E-1 | 2.6E-1 | 2.7E-1 | 8.5E-1 | 1.4E0 | 7 | 9 | 7 | 9 | 0.46 |

Figure 26.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.1E1 | 4.7E1 | 9.9E1 | 5.6E1 | 9.0E1 | 4.3E1 | 1.0E1 | 8.0E0 | 4.0E2 | 1.4E2 | 24 | 8 | 24 | 8 | 0.38 |
| Ad | ug/mL | 1.5E-2 | 7.6E-2 | 3.4E-2 | 9.3E-2 | 3.2E-2 | 1.2E-1 | 1.9E-3 | 3.9E-3 | 9.7E-2 | 3.5E-1 | 18 | 7 | 18 | 7 | 0.67 |
| Af | ng/mL | 6.6E-1 | 6.0E-1 | 6.8E0 | 3.2E0 | 8.5E0 | 5.8E0 | 1.7E-3 | 1.7E-1 | 2.2E1 | 1.6E1 | 18 | 7 | 18 | 7 | 0.50 |
| Aj | ug/mL | 3.4E-1 | 5.2E-1 | 1.7E0 | 2.1E0 | 2.4E0 | 2.6E0 | 2.3E-3 | 2.1E-2 | 6.1E0 | 5.8E0 | 18 | 7 | 18 | 7 | 0.61 |
| Al | mg/mL | 9.9E-5 | 9.3E-5 | 1.1E-4 | 1.9E-4 | 1.3E-4 | 2.4E-4 | 8.0E-6 | 7.2E-5 | 5.7E-4 | 7.2E-4 | 18 | 7 | 18 | 7 | 0.60 |
| An | U/mL | 1.1E2 | 8.8E1 | 2.1E2 | 1.7E2 | 2.5E2 | 1.7E2 | 1.1E0 | 1.3E1 | 9.1E2 | 4.3E2 | 18 | 7 | 18 | 7 | 0.46 |
| Ao | pg/mL | 8.3E1 | 1.3E2 | 1.3E2 | 1.8E2 | 1.4E2 | 2.2E2 | 2.2E1 | 1.8E1 | 6.4E2 | 6.5E2 | 18 | 7 | 18 | 7 | 0.52 |
| Ap | ng/mL | 2.2E1 | 4.3E1 | 2.6E1 | 4.0E1 | 2.0E1 | 2.2E1 | 3.2E0 | 7.2E0 | 8.2E1 | 6.6E1 | 18 | 7 | 18 | 7 | 0.72 |
| Ar | ng/mL | 7.6E-1 | 3.5E0 | 3.1E0 | 5.1E0 | 5.6E0 | 6.0E0 | 6.7E-2 | 3.4E-3 | 2.1E1 | 1.4E1 | 18 | 7 | 18 | 7 | 0.56 |
| As | ng/mL | 3.7E-3 | 1.1E-2 | 8.8E-3 | 9.1E-3 | 1.0E-2 | 5.5E-3 | 1.7E-3 | 1.7E-3 | 3.3E-2 | 1.5E-2 | 18 | 7 | 18 | 7 | 0.58 |
| Aw | pg/mL | 1.7E1 | 2.0E1 | 1.7E1 | 2.1E1 | 3.8E0 | 5.5E0 | 8.2E0 | 1.4E1 | 2.1E1 | 3.0E1 | 18 | 7 | 18 | 7 | 0.73 |
| Ax | ng/mL | 2.6E0 | 3.1E0 | 4.7E0 | 9.5E1 | 5.4E0 | 1.6E2 | 2.5E-2 | 4.2E-1 | 1.6E1 | 3.7E2 | 18 | 7 | 18 | 7 | 0.63 |
| Ba | ng/mL | 6.3E1 | 5.7E2 | 3.5E2 | 3.1E3 | 5.9E2 | 3.8E3 | 2.7E-1 | 1.0E1 | 2.2E3 | 8.1E3 | 18 | 7 | 18 | 7 | 0.72 |
| Bb | ng/mL | 1.4E0 | 2.2E0 | 3.3E0 | 4.9E0 | 3.9E0 | 5.7E0 | 4.1E-3 | 8.4E-1 | 1.5E1 | 1.7E1 | 18 | 7 | 18 | 7 | 0.60 |
| Bc | ng/mL | 2.6E1 | 6.6E0 | 6.7E1 | 1.9E2 | 1.0E2 | 2.5E2 | 4.3E-1 | 2.3E0 | 3.6E2 | 6.9E2 | 18 | 7 | 18 | 7 | 0.63 |
| Bg | ng/mL | 5.7E-2 | 4.1E0 | 5.8E-1 | 6.1E1 | 1.2E0 | 1.5E2 | 5.3E-4 | 2.8E-2 | 4.2E0 | 4.0E2 | 18 | 7 | 18 | 7 | 0.77 |
| Bn | ng/mL | 4.5E-1 | 5.6E-2 | 1.8E0 | 7.0E-1 | 2.5E0 | 1.3E0 | 5.6E-2 | 5.6E-2 | 7.4E0 | 3.4E0 | 18 | 7 | 18 | 7 | 0.30 |
| Bo | ng/mL | 8.8E0 | 2.6E1 | 1.3E1 | 2.8E1 | 1.1E1 | 1.5E1 | 1.6E-2 | 5.6E0 | 3.4E1 | 4.8E1 | 18 | 7 | 18 | 7 | 0.79 |
| Ch | uIU/mL | 7.3E-1 | 1.1E0 | 1.1E1 | 1.7E2 | 2.9E1 | 4.5E2 | 3.4E-3 | 3.3E-1 | 9.8E1 | 1.2E3 | 18 | 7 | 18 | 7 | 0.60 |
| Co | pg/mL | 2.6E1 | 9.7E1 | 3.8E1 | 1.3E2 | 3.8E1 | 1.6E2 | 1.5E-1 | 2.1E1 | 1.3E2 | 5.0E2 | 18 | 7 | 18 | 7 | 0.79 |
| Cp | ng/mL | 2.3E1 | 3.1E1 | 2.6E1 | 4.3E1 | 1.1E1 | 2.4E1 | 1.1E1 | 1.8E1 | 5.0E1 | 7.7E1 | 18 | 7 | 18 | 7 | 0.74 |
| Cq | ng/mL | 3.1E-2 | 4.7E-2 | 6.6E-2 | 7.8E-2 | 1.2E-1 | 6.7E-2 | 8.0E-4 | 1.5E-2 | 5.2E-1 | 1.9E-1 | 18 | 7 | 18 | 7 | 0.69 |
| Cs | ng/mL | 1.0E2 | 5.3E1 | 3.1E2 | 1.4E3 | 5.1E2 | 2.3E3 | 1.3E0 | 8.8E0 | 1.8E3 | 5.1E3 | 18 | 7 | 18 | 7 | 0.56 |
| Ct | ng/mL | 1.8E-1 | 9.7E0 | 3.3E1 | 8.1E1 | 1.1E2 | 1.7E2 | 1.1E-4 | 1.1E-4 | 4.7E2 | 4.6E2 | 18 | 7 | 18 | 7 | 0.66 |
| Cu | ng/mL | 2.0E-1 | 6.4E-1 | 3.4E-1 | 7.6E-1 | 3.9E-1 | 5.6E-1 | 9.0E-5 | 1.9E-1 | 1.5E0 | 1.7E0 | 18 | 7 | 18 | 7 | 0.79 |
| Cv | ng/mL | 3.8E0 | 2.1E0 | 7.1E0 | 1.0E1 | 1.1E1 | 1.5E1 | 1.4E-4 | 3.1E-1 | 4.3E1 | 4.1E1 | 18 | 7 | 18 | 7 | 0.57 |
| Cw | mIU/mL | 1.8E-2 | 5.1E-2 | 2.6E-2 | 4.5E-2 | 2.2E-2 | 1.6E-2 | 2.8E-3 | 2.0E-2 | 9.1E-2 | 6.8E-2 | 18 | 7 | 18 | 7 | 0.84 |
| Cx | ng/mL | 8.1E-1 | 4.7E-3 | 7.1E1 | 9.5E0 | 1.1E2 | 2.5E1 | 9.3E-5 | 9.3E-5 | 2.8E2 | 6.6E1 | 18 | 7 | 18 | 7 | 0.34 |
| Db | ug/mL | 6.6E0 | 1.2E1 | 7.1E0 | 1.1E1 | 7.0E0 | 4.6E0 | 4.8E-1 | 5.9E0 | 2.3E1 | 1.9E1 | 18 | 7 | 18 | 7 | 0.70 |
| Dc | nmol/L | 1.4E-2 | 2.0E-2 | 5.4E-2 | 1.1E-1 | 1.4E-1 | 1.5E-1 | 6.0E-4 | 7.7E-3 | 6.3E-1 | 4.0E-1 | 18 | 7 | 18 | 7 | 0.66 |
| Dd | ug/mL | 4.8E-2 | 4.2E-2 | 8.4E-2 | 8.1E-2 | 8.6E-2 | 9.1E-2 | 8.3E-5 | 6.2E-3 | 3.1E-1 | 2.5E-1 | 18 | 7 | 18 | 7 | 0.44 |
| De | ng/mL | 3.4E-3 | 1.3E-1 | 1.0E-1 | 1.1E-1 | 1.4E-1 | 1.2E-1 | 3.4E-3 | 3.4E-3 | 4.7E-1 | 3.2E-1 | 18 | 7 | 18 | 7 | 0.54 |
| Dg | ng/mL | 1.5E1 | 4.4E1 | 2.4E1 | 4.6E1 | 2.2E1 | 3.2E1 | 9.3E-1 | 7.7E0 | 7.5E1 | 9.3E1 | 18 | 7 | 18 | 7 | 0.73 |
| Di | pg/mL | 1.3E0 | 2.3E0 | 1.8E0 | 2.6E0 | 1.6E0 | 1.6E0 | 1.8E-1 | 1.8E-1 | 5.2E0 | 4.6E0 | 18 | 7 | 18 | 7 | 0.66 |
| Dk | uIU/mL | 1.2E-2 | 7.7E-2 | 3.5E-2 | 9.9E-2 | 4.3E-2 | 1.1E-1 | 1.1E-4 | 8.9E-3 | 1.3E-1 | 3.3E-1 | 18 | 7 | 18 | 7 | 0.76 |
| Dl | ng/mL | 1.4E2 | 1.9E2 | 2.2E2 | 2.5E2 | 2.4E2 | 2.2E2 | 4.0E0 | 4.7E1 | 9.0E2 | 6.1E2 | 18 | 7 | 18 | 7 | 0.58 |
| Ef | ng/ml | 1.0E-1 | 1.3E0 | 4.4E-1 | 2.8E0 | 7.4E-1 | 3.7E0 | 5.7E-4 | 5.7E-4 | 2.4E0 | 9.4E0 | 18 | 7 | 18 | 7 | 0.71 |
| Wm | % | 8.4E-1 | 3.6E0 | 1.1E1 | 2.9E2 | 4.2E1 | 5.3E2 | 8.5E-2 | 8.5E-2 | 1.9E2 | 1.3E3 | 21 | 8 | 21 | 8 | 0.65 |
| Po | pg/ml | 2.2E0 | 3.1E0 | 6.4E0 | 1.5E1 | 9.2E0 | 2.3E1 | 2.6E-2 | 2.6E-2 | 3.6E1 | 7.0E1 | 30 | 10 | 30 | 10 | 0.62 |
| Et | ng/ml | 1.4E3 | 2.1E3 | 1.7E3 | 2.1E3 | 1.3E3 | 1.6E3 | 1.5E2 | 1.8E2 | 5.0E3 | 5.0E3 | 30 | 10 | 30 | 10 | 0.56 |
| Ex | ng/ml | 5.5E-2 | 1.6E-1 | 1.3E-1 | 5.8E-1 | 1.3E-1 | 1.1E0 | 1.7E-4 | 1.5E-4 | 3.3E-1 | 3.1E0 | 11 | 7 | 11 | 7 | 0.56 |
| Fp | ng/ml | 1.4E1 | 3.2E1 | 2.8E1 | 4.3E1 | 3.3E1 | 4.0E1 | 2.8E-1 | 4.0E0 | 1.2E2 | 1.0E2 | 30 | 11 | 30 | 11 | 0.61 |
| Fr | ng/ml | 4.3E4 | 3.3E5 | 1.4E5 | 4.0E5 | 2.4E5 | 2.9E5 | 7.8E2 | 7.9E3 | 8.4E5 | 8.9E5 | 30 | 11 | 30 | 11 | 0.80 |
| Fw | pg/ml | 5.1E0 | 9.4E0 | 3.0E1 | 1.6E1 | 6.2E1 | 1.7E1 | 1.2E-1 | 1.2E-1 | 2.5E2 | 3.9E1 | 18 | 7 | 18 | 7 | 0.56 |
| Gl | pg/ml | 1.2E4 | 1.8E4 | 1.4E4 | 1.7E4 | 1.1E4 | 7.6E3 | 6.3E2 | 4.9E3 | 2.9E4 | 2.6E4 | 18 | 7 | 18 | 7 | 0.55 |
| Gp | U/ml | 1.3E0 | 1.5E-2 | 3.6E0 | 2.1E-1 | 5.9E0 | 3.9E-1 | 1.5E-2 | 1.5E-2 | 2.0E1 | 1.0E0 | 18 | 7 | 18 | 7 | 0.16 |
| Nm | pg/ml | 7.1E3 | 6.4E3 | 1.5E4 | 5.5E4 | 1.9E4 | 1.4E5 | 1.0E-9 | 1.0E-9 | 7.4E4 | 4.4E5 | 30 | 10 | 30 | 10 | 0.55 |
| Nn | pg/ml | 2.0E2 | 1.3E3 | 4.4E3 | 3.1E3 | 1.7E4 | 4.1E3 | 1.0E-9 | 1.0E-9 | 9.5E4 | 1.3E4 | 30 | 10 | 30 | 10 | 0.72 |
| No | pg/ml | 1.4E1 | 3.2E1 | 3.1E1 | 1.4E2 | 3.8E1 | 2.9E2 | 2.4E-1 | 3.3E-1 | 1.5E2 | 9.5E2 | 30 | 10 | 30 | 10 | 0.59 |

| Code | Units | Median | | Average | | Standard Deviation | | Minimum | | Maximum | | Number of Samples | | Number of Patients | | AUC |
|------|-------|--------|-----|---------|-----|--------|------|---------|--------|---------|--------|--------|-----|---------|-----|-----|
| | | NonDis | Dis | NonDis | Dis | NonDis | Dis | NonDis | Dis | NonDis | Dis | NonDis | Dis | NonDis | Dis | |
| Nq | pg/ml | 5.9E-1 | 2.1E1 | 4.5E1 | 5.1E1 | 1.3E2 | 7.4E1 | 1.0E-9 | 1.0E-9 | 6.7E2 | 2.4E2 | 30 | 10 | 30 | 10 | 0.74 |
| Nr | pg/ml | 1.2E0 | 3.0E0 | 5.5E0 | 3.4E1 | 8.2E0 | 6.2E1 | 1.0E-9 | 1.0E-9 | 2.5E1 | 2.0E2 | 30 | 10 | 30 | 10 | 0.59 |
| Ns | pg/ml | 1.0E-9 | 1.0E-9 | 3.7E1 | 3.6E-1 | 2.0E2 | 1.1E0 | 1.0E-9 | 1.0E-9 | 1.1E3 | 3.6E0 | 30 | 10 | 30 | 10 | 0.51 |
| Nt | pg/ml | 1.5E2 | 1.9E2 | 1.8E2 | 1.9E2 | 1.7E2 | 1.4E2 | 1.5E1 | 3.3E1 | 8.8E2 | 4.3E2 | 30 | 10 | 30 | 10 | 0.51 |
| Nu | pg/ml | 2.2E1 | 3.2E1 | 8.2E1 | 9.1E1 | 1.1E2 | 1.1E2 | 1.0E-9 | 1.0E-9 | 3.8E2 | 2.9E2 | 30 | 10 | 30 | 10 | 0.54 |
| Lu | pg/ml | 1.0E4 | 4.6E3 | 2.7E4 | 1.0E4 | 1.0E5 | 1.8E4 | 1.6E3 | 1.3E3 | 5.6E5 | 6.1E4 | 30 | 10 | 30 | 10 | 0.32 |
| Lv | pg/ml | 1.0E-9 | 4.0E1 | 2.2E1 | 6.3E1 | 5.0E1 | 7.2E1 | 1.0E-9 | 1.0E-9 | 2.6E2 | 1.9E2 | 30 | 10 | 30 | 10 | 0.71 |
| Lw | pg/ml | 1.0E-9 | 1.0E-9 | 1.0E-9 | 2.1E0 | 0.0E0 | 6.8E0 | 1.0E-9 | 1.0E-9 | 1.0E-9 | 2.1E1 | 30 | 10 | 30 | 10 | 0.55 |
| Lx | pg/ml | 5.4E0 | 1.8E2 | 1.7E2 | 6.6E2 | 3.5E2 | 1.3E3 | 1.0E-9 | 1.0E-9 | 1.5E3 | 4.0E3 | 30 | 10 | 30 | 10 | 0.65 |
| Ly | pg/ml | 1.0E-9 | 1.0E-9 | 9.9E0 | 4.3E0 | 1.6E1 | 1.0E1 | 1.0E-9 | 1.0E-9 | 5.8E1 | 3.0E1 | 30 | 10 | 30 | 10 | 0.41 |
| Lz | pg/ml | 1.0E-9 | 1.0E-9 | 2.3E0 | 3.7E0 | 1.0E1 | 1.2E1 | 1.0E-9 | 1.0E-9 | 5.6E1 | 3.7E1 | 30 | 10 | 30 | 10 | 0.52 |
| Ma | pg/ml | 1.2E2 | 1.5E3 | 9.9E2 | 9.2E3 | 3.1E3 | 1.8E4 | 1.0E-9 | 3.9E1 | 1.7E4 | 5.2E4 | 30 | 10 | 30 | 10 | 0.67 |
| Mb | pg/ml | 2.7E1 | 4.7E1 | 3.6E1 | 4.1E1 | 1.7E1 | 1.6E1 | 1.8E1 | 1.9E1 | 6.9E1 | 6.4E1 | 30 | 10 | 30 | 10 | 0.58 |
| Mc | pg/ml | 1.0E-9 | 1.0E-9 | 5.5E-2 | 1.0E-9 | 3.0E-1 | 0.0E0 | 1.0E-9 | 1.0E-9 | 1.7E0 | 1.0E-9 | 30 | 10 | 30 | 10 | 0.48 |
| Md | pg/ml | 1.0E-9 | 1.0E-9 | 7.0E-1 | 1.0E-9 | 2.3E0 | 0.0E0 | 1.0E-9 | 1.0E-9 | 9.8E0 | 1.0E-9 | 30 | 10 | 30 | 10 | 0.43 |
| Me | pg/ml | 2.4E1 | 1.0E1 | 2.6E1 | 1.8E1 | 2.8E1 | 1.9E1 | 2.4E-1 | 1.4E0 | 1.6E2 | 6.0E1 | 30 | 10 | 30 | 10 | 0.44 |
| Mf | pg/ml | 1.0E-9 | 1.0E-9 | 1.5E0 | 6.2E-1 | 6.7E0 | 1.6E0 | 1.0E-9 | 1.0E-9 | 3.7E1 | 5.0E0 | 30 | 10 | 30 | 10 | 0.56 |
| Mg | pg/ml | 2.7E-1 | 3.1E0 | 3.1E0 | 9.9E0 | 4.8E0 | 1.1E1 | 1.0E-9 | 1.0E-9 | 1.7E1 | 2.7E1 | 30 | 10 | 30 | 10 | 0.70 |
| Mh | pg/ml | 1.0E-9 | 3.5E-2 | 3.4E-1 | 1.4E0 | 6.8E-1 | 3.7E0 | 1.0E-9 | 1.0E-9 | 2.6E0 | 1.2E1 | 30 | 10 | 30 | 10 | 0.62 |
| Mi | pg/ml | 1.0E-9 | 1.0E-9 | 1.0E0 | 6.9E0 | 5.7E0 | 1.9E1 | 1.0E-9 | 1.0E-9 | 3.1E1 | 6.1E1 | 30 | 10 | 30 | 10 | 0.58 |
| Mj | pg/ml | 1.0E-9 | 1.2E0 | 5.9E0 | 4.9E0 | 1.7E1 | 7.9E0 | 1.0E-9 | 1.0E-9 | 8.1E1 | 2.4E1 | 30 | 10 | 30 | 10 | 0.63 |
| Mk | pg/ml | 7.0E0 | 4.5E0 | 9.7E0 | 1.0E1 | 1.4E1 | 1.3E1 | 1.0E-9 | 1.0E-9 | 6.6E1 | 3.6E1 | 30 | 10 | 30 | 10 | 0.51 |
| Ml | pg/ml | 1.0E-9 | 1.0E-9 | 1.2E1 | 1.2E1 | 6.2E1 | 3.6E1 | 1.0E-9 | 1.0E-9 | 3.4E2 | 1.1E2 | 30 | 10 | 30 | 10 | 0.58 |
| Mm | pg/ml | 2.5E2 | 4.4E2 | 9.4E2 | 1.1E3 | 1.7E3 | 1.5E3 | 1.0E-9 | 2.7E1 | 7.7E3 | 4.5E3 | 30 | 10 | 30 | 10 | 0.54 |
| Mn | pg/ml | 4.7E0 | 7.6E0 | 7.1E0 | 1.2E1 | 5.9E0 | 1.1E1 | 1.0E-9 | 1.9E0 | 2.1E1 | 3.2E1 | 30 | 10 | 30 | 10 | 0.64 |
| Mp | pg/ml | 1.0E-9 | 6.4E0 | 1.6E1 | 2.1E1 | 4.1E1 | 5.2E1 | 1.0E-9 | 1.0E-9 | 1.7E2 | 1.7E2 | 30 | 10 | 30 | 10 | 0.59 |
| Mq | pg/ml | 1.0E-9 | 1.0E-9 | 1.6E0 | 1.7E1 | 5.2E0 | 4.5E1 | 1.0E-9 | 1.0E-9 | 2.4E1 | 1.4E2 | 30 | 10 | 30 | 10 | 0.60 |
| Mr | pg/ml | 1.0E-9 | 1.4E0 | 6.4E0 | 8.4E0 | 1.5E1 | 1.3E1 | 1.0E-9 | 1.0E-9 | 8.0E1 | 3.3E1 | 30 | 10 | 30 | 10 | 0.55 |
| Ms | pg/ml | 5.0E2 | 3.3E2 | 6.2E2 | 5.1E2 | 6.7E2 | 6.5E2 | 1.0E-9 | 1.0E-9 | 3.3E3 | 2.2E3 | 30 | 10 | 30 | 10 | 0.42 |
| Mt | pg/ml | 1.1E0 | 2.8E0 | 1.3E1 | 6.7E0 | 4.6E1 | 7.9E0 | 1.0E-9 | 1.0E-9 | 2.5E2 | 2.1E1 | 30 | 10 | 30 | 10 | 0.60 |
| Mu | pg/ml | 1.0E-9 | 1.8E0 | 8.3E0 | 3.2E0 | 4.3E1 | 3.4E0 | 1.0E-9 | 1.0E-9 | 2.3E2 | 8.3E0 | 30 | 10 | 30 | 10 | 0.76 |
| Mv | pg/ml | 1.0E-9 | 1.1E2 | 6.9E1 | 2.2E2 | 2.2E2 | 2.7E2 | 1.0E-9 | 1.0E-9 | 9.3E2 | 7.2E2 | 30 | 10 | 30 | 10 | 0.78 |
| Mw | pg/ml | 1.5E1 | 4.4E2 | 7.9E2 | 7.8E2 | 3.2E3 | 8.0E2 | 1.0E-9 | 1.0E-9 | 1.7E4 | 2.2E3 | 30 | 10 | 30 | 10 | 0.79 |
| Mx | pg/ml | 1.0E-9 | 1.0E-9 | 1.9E0 | 2.7E-1 | 5.9E0 | 4.3E-1 | 1.0E-9 | 1.0E-9 | 3.2E1 | 1.2E0 | 30 | 10 | 30 | 10 | 0.46 |
| My | pg/ml | 1.0E-9 | 1.0E2 | 2.5E2 | 3.5E2 | 8.3E2 | 4.4E2 | 1.0E-9 | 1.0E-9 | 4.1E3 | 1.2E3 | 30 | 10 | 30 | 10 | 0.68 |
| Mz | pg/ml | 1.8E1 | 1.9E1 | 4.0E1 | 4.5E1 | 5.5E1 | 5.8E1 | 1.0E-9 | 1.0E-9 | 1.8E2 | 1.6E2 | 30 | 10 | 30 | 10 | 0.53 |
| Na | pg/ml | 1.0E-9 | 1.0E-9 | 9.6E-1 | 1.1E-1 | 2.3E0 | 3.4E-1 | 1.0E-9 | 1.0E-9 | 9.6E0 | 1.1E0 | 30 | 10 | 30 | 10 | 0.37 |
| Nb | pg/ml | 2.7E0 | 4.5E0 | 3.4E0 | 7.3E0 | 3.7E0 | 8.0E0 | 1.0E-9 | 1.3E0 | 1.5E1 | 2.8E1 | 30 | 10 | 30 | 10 | 0.73 |
| Nc | pg/ml | 1.5E2 | 4.6E2 | 3.2E2 | 5.3E2 | 4.6E2 | 3.4E2 | 1.0E-9 | 1.3E2 | 1.8E3 | 1.1E3 | 30 | 10 | 30 | 10 | 0.74 |
| Nd | pg/ml | 1.0E1 | 1.0E1 | 1.8E1 | 2.4E1 | 1.5E1 | 3.2E1 | 1.0E-9 | 5.6E-1 | 4.7E1 | 1.0E2 | 30 | 10 | 30 | 10 | 0.52 |
| Ne | pg/ml | 2.7E2 | 3.2E2 | 3.4E2 | 4.2E2 | 3.3E2 | 2.8E2 | 1.0E-9 | 1.4E2 | 1.3E3 | 9.9E2 | 30 | 10 | 30 | 10 | 0.60 |
| Nf | pg/ml | 1.0E-9 | 1.0E-9 | 1.1E0 | 3.3E0 | 3.7E0 | 5.7E0 | 1.0E-9 | 1.0E-9 | 1.5E1 | 1.8E1 | 30 | 10 | 30 | 10 | 0.62 |
| Ng | pg/ml | 3.5E0 | 6.6E1 | 5.8E1 | 1.3E2 | 9.8E1 | 2.0E2 | 1.0E-9 | 1.0E-9 | 4.3E2 | 6.6E2 | 30 | 10 | 30 | 10 | 0.62 |
| Nh | pg/ml | 4.3E1 | 5.7E1 | 6.5E1 | 5.3E1 | 7.0E1 | 1.9E1 | 7.5E0 | 2.7E1 | 3.4E2 | 7.4E1 | 30 | 10 | 30 | 10 | 0.57 |
| Ni | pg/ml | 5.8E1 | 1.8E2 | 8.2E1 | 2.2E2 | 1.1E2 | 2.3E2 | 1.0E-9 | 1.0E-9 | 4.8E2 | 5.5E2 | 30 | 10 | 30 | 10 | 0.64 |
| Nj | pg/ml | 3.8E0 | 8.4E0 | 7.8E0 | 8.7E0 | 8.4E0 | 5.4E0 | 1.0E-9 | 1.0E-9 | 3.5E1 | 2.0E1 | 30 | 10 | 30 | 10 | 0.61 |
| Nk | pg/ml | 1.3E1 | 2.5E1 | 2.3E1 | 2.8E1 | 3.3E1 | 1.9E1 | 1.0E-9 | 2.6E0 | 1.7E2 | 6.4E1 | 30 | 10 | 30 | 10 | 0.67 |
| Nl | pg/ml | 2.3E1 | 4.6E1 | 3.3E1 | 4.7E1 | 3.2E1 | 1.8E1 | 1.0E-9 | 1.9E1 | 1.4E2 | 7.7E1 | 30 | 10 | 30 | 10 | 0.70 |
| Hq | pg/ml | 1.1E0 | 1.3E0 | 4.1E0 | 9.4E0 | 9.5E0 | 1.9E1 | 1.0E-9 | 1.0E-9 | 5.0E1 | 6.1E1 | 30 | 10 | 30 | 10 | 0.59 |
| Hr | pg/ml | 9.8E1 | 1.3E2 | 4.7E2 | 3.2E2 | 8.1E2 | 5.0E2 | 3.5E1 | 2.2E1 | 3.7E3 | 1.7E3 | 30 | 10 | 30 | 10 | 0.53 |
| Hu | pg/ml | 1.8E1 | 5.4E2 | 9.2E3 | 2.7E3 | 4.8E4 | 3.6E3 | 1.0E-9 | 1.0E-9 | 2.6E5 | 8.8E3 | 30 | 10 | 30 | 10 | 0.74 |
| Hv | pg/ml | 2.9E0 | 1.1E0 | 6.1E0 | 1.9E0 | 1.5E1 | 2.9E0 | 1.0E-9 | 1.0E-9 | 8.4E1 | 9.4E0 | 30 | 10 | 30 | 10 | 0.24 |
| Hw | pg/ml | 5.2E0 | 7.1E0 | 1.6E1 | 8.1E0 | 2.8E1 | 6.6E0 | 1.3E0 | 5.3E-1 | 1.5E2 | 2.0E1 | 30 | 10 | 30 | 10 | 0.47 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Hx | pg/ml | 1.6E1 | 2.9E1 | 3.8E1 | 2.9E1 | 6.5E1 | 2.2E1 | 1.0E-9 | 5.0E0 | 3.1E2 | 5.5E1 | 30 | 10 | 30 | 10 | 0.61 |
| Ih | ng/ml | 6.8E1 | 1.3E2 | 2.1E2 | 2.6E2 | 3.5E2 | 3.7E2 | 1.4E0 | 4.7E0 | 1.7E3 | 1.1E3 | 30 | 10 | 30 | 10 | 0.54 |
| Ii | ng/ml | 9.4E1 | 5.4E1 | 1.3E2 | 1.1E2 | 1.5E2 | 1.3E2 | 2.9E0 | 9.8E0 | 5.3E2 | 4.3E2 | 29 | 10 | 29 | 10 | 0.47 |
| Ij | ng/ml | 7.8E1 | 2.5E2 | 1.7E2 | 3.1E2 | 3.7E2 | 2.9E2 | 4.7E0 | 2.3E1 | 2.0E3 | 8.4E2 | 29 | 9 | 29 | 9 | 0.76 |
| Ik | ng/ml | 1.6E2 | 2.6E2 | 3.5E2 | 4.9E2 | 4.9E2 | 5.7E2 | 1.3E0 | 2.7E0 | 1.5E3 | 1.5E3 | 29 | 10 | 29 | 10 | 0.59 |
| Il | ng/ml | 2.2E2 | 6.5E2 | 8.7E2 | 2.1E3 | 2.3E3 | 3.7E3 | 1.0E-9 | 2.5E1 | 1.2E4 | 1.2E4 | 29 | 10 | 29 | 10 | 0.65 |
| Im | ng/ml | 1.7E2 | 7.0E2 | 4.8E2 | 1.4E3 | 8.3E2 | 2.1E3 | 2.7E1 | 5.6E1 | 3.4E3 | 6.8E3 | 29 | 10 | 29 | 10 | 0.69 |
| In | ng/ml | 2.5E0 | 1.5E0 | 1.4E1 | 5.4E0 | 4.3E1 | 9.2E0 | 1.0E-9 | 1.0E-9 | 2.4E2 | 2.9E1 | 30 | 10 | 30 | 10 | 0.39 |
| Io | ng/ml | 7.0E3 | 1.0E4 | 1.7E4 | 1.8E4 | 3.7E4 | 2.5E4 | 6.2E1 | 2.2E3 | 2.0E5 | 8.4E4 | 30 | 10 | 30 | 10 | 0.56 |
| Ip | ng/ml | 1.0E1 | 3.0E1 | 1.9E1 | 3.3E1 | 2.1E1 | 3.1E1 | 1.0E-9 | 4.4E-2 | 6.1E1 | 1.0E2 | 30 | 10 | 30 | 10 | 0.64 |
| Iq | ug/ml | 7.6E-2 | 2.5E-1 | 3.0E-1 | 3.6E-1 | 5.5E-1 | 3.8E-1 | 1.0E-9 | 1.0E-9 | 2.1E0 | 1.2E0 | 30 | 10 | 30 | 10 | 0.63 |
| Ir | ug/ml | 3.0E-1 | 1.4E0 | 1.7E0 | 2.5E0 | 4.8E0 | 2.9E0 | 1.0E-9 | 8.4E-2 | 2.6E1 | 9.1E0 | 30 | 10 | 30 | 10 | 0.72 |
| Is | ng/ml | 2.3E0 | 1.2E1 | 1.5E1 | 1.8E1 | 2.8E1 | 1.4E1 | 2.9E-2 | 3.7E-1 | 1.1E2 | 3.9E1 | 30 | 10 | 30 | 10 | 0.74 |
| It | ng/ml | 1.3E0 | 4.9E-1 | 3.4E1 | 1.5E1 | 1.5E2 | 3.6E1 | 1.0E-9 | 1.0E-9 | 8.3E2 | 1.1E2 | 30 | 10 | 30 | 10 | 0.40 |
| Iu | ng/ml | 9.5E1 | 1.3E2 | 2.2E2 | 2.9E2 | 2.7E2 | 3.3E2 | 1.0E-9 | 1.0E-9 | 1.2E3 | 8.5E2 | 30 | 10 | 30 | 10 | 0.56 |
| Iv | ng/ml | 1.8E1 | 4.9E1 | 5.4E1 | 7.0E1 | 1.4E2 | 6.1E1 | 1.0E-9 | 1.9E0 | 7.7E2 | 1.6E2 | 30 | 10 | 30 | 10 | 0.69 |
| Pz | ng/ml | 2.1E3 | 1.0E4 | 3.5E3 | 7.0E3 | 3.5E3 | 4.1E3 | 3.6E1 | 6.5E2 | 1.0E4 | 1.0E4 | 29 | 10 | 29 | 10 | 0.72 |
| Qa | ng/ml | 5.3E3 | 1.3E4 | 8.9E3 | 1.3E4 | 1.0E4 | 9.5E3 | 3.4E2 | 7.2E2 | 3.6E4 | 2.8E4 | 29 | 10 | 29 | 10 | 0.64 |
| Qb | ng/ml | 1.5E2 | 1.7E2 | 3.3E2 | 2.2E2 | 7.5E2 | 1.7E2 | 1.1E1 | 2.9E1 | 4.1E3 | 6.0E2 | 29 | 10 | 29 | 10 | 0.52 |
| Qc | ng/ml | 2.5E2 | 5.9E2 | 3.7E2 | 8.4E2 | 4.0E2 | 8.5E2 | 2.7E1 | 1.7E2 | 1.4E3 | 3.1E3 | 29 | 10 | 29 | 10 | 0.77 |
| Qd | ng/ml | 9.3E3 | 2.5E4 | 2.3E4 | 3.8E4 | 3.6E4 | 4.3E4 | 9.8E2 | 3.4E3 | 1.5E5 | 1.5E5 | 29 | 10 | 29 | 10 | 0.66 |
| Qe | ng/ml | 9.4E2 | 3.2E3 | 1.6E3 | 3.0E3 | 1.7E3 | 2.1E3 | 6.8E1 | 2.8E2 | 6.0E3 | 6.0E3 | 29 | 10 | 29 | 10 | 0.69 |
| Jg | ng/ml | 3.6E2 | 9.5E2 | 8.3E2 | 1.5E3 | 1.3E3 | 1.6E3 | 1.1E1 | 1.1E2 | 6.8E3 | 5.3E3 | 30 | 10 | 30 | 10 | 0.69 |
| Jh | ng/ml | 4.9E0 | 4.1E1 | 2.2E1 | 6.1E1 | 4.9E1 | 6.1E1 | 1.0E-9 | 1.3E0 | 2.4E2 | 1.8E2 | 30 | 10 | 30 | 10 | 0.78 |
| Ji | ng/ml | 6.6E1 | 1.1E2 | 1.0E2 | 2.6E2 | 1.1E2 | 5.5E2 | 8.3E0 | 1.8E1 | 5.4E2 | 1.8E3 | 30 | 10 | 30 | 10 | 0.56 |
| Jj | ng/ml | 3.2E2 | 3.8E2 | 7.7E2 | 7.0E2 | 1.4E3 | 1.0E3 | 4.9E0 | 2.0E1 | 7.7E3 | 3.5E3 | 30 | 10 | 30 | 10 | 0.49 |
| Jk | ng/ml | 1.9E0 | 4.8E1 | 3.7E1 | 5.7E1 | 9.5E1 | 5.8E1 | 1.1E-1 | 6.5E-1 | 3.9E2 | 1.7E2 | 30 | 10 | 30 | 10 | 0.74 |
| Jl | ng/ml | 8.6E-1 | 2.7E0 | 2.1E1 | 6.1E0 | 9.8E1 | 9.3E0 | 1.1E-3 | 4.8E-2 | 5.4E2 | 2.6E1 | 30 | 10 | 30 | 10 | 0.67 |
| Jm | ng/ml | 1.6E1 | 3.4E1 | 4.0E1 | 4.6E1 | 6.0E1 | 5.0E1 | 1.0E-9 | 2.3E0 | 2.9E2 | 1.3E2 | 30 | 10 | 30 | 10 | 0.58 |
| Jn | pg/ml | 3.7E-1 | 7.9E-1 | 2.4E0 | 2.3E0 | 7.2E0 | 3.2E0 | 1.0E-9 | 1.0E-9 | 3.9E1 | 9.5E0 | 30 | 10 | 30 | 10 | 0.59 |
| Jo | pg/ml | 2.6E3 | 3.4E3 | 3.4E3 | 4.8E3 | 3.3E3 | 4.1E3 | 1.9E2 | 4.8E2 | 1.5E4 | 1.3E4 | 30 | 10 | 30 | 10 | 0.60 |
| Jp | pg/ml | 7.7E4 | 1.0E5 | 7.6E4 | 1.0E5 | 3.5E4 | 4.9E4 | 2.8E3 | 1.6E4 | 1.7E5 | 1.9E5 | 30 | 10 | 30 | 10 | 0.68 |
| Jq | pg/ml | 7.0E1 | 1.2E2 | 2.9E2 | 4.8E2 | 7.3E2 | 1.1E3 | 5.4E0 | 2.2E1 | 4.0E3 | 3.7E3 | 30 | 10 | 30 | 10 | 0.57 |
| Jr | pg/ml | 5.7E0 | 1.9E1 | 2.0E1 | 2.3E1 | 3.9E1 | 2.7E1 | 1.0E-9 | 1.0E-9 | 1.8E2 | 8.6E1 | 30 | 10 | 30 | 10 | 0.55 |
| Js | pg/ml | 1.4E1 | 1.3E1 | 4.1E1 | 2.6E1 | 1.1E2 | 3.6E1 | 1.3E0 | 1.0E-9 | 6.1E2 | 1.2E2 | 30 | 10 | 30 | 10 | 0.50 |
| Jt | pg/ml | 1.9E3 | 2.6E3 | 2.3E3 | 2.8E3 | 1.7E3 | 2.2E3 | 2.8E2 | 2.6E2 | 7.3E3 | 6.9E3 | 30 | 10 | 30 | 10 | 0.55 |
| Lh | pg/ml | 1.3E4 | 3.8E4 | 2.3E4 | 4.0E4 | 2.5E4 | 3.1E4 | 1.8E2 | 5.5E2 | 1.2E5 | 9.2E4 | 30 | 10 | 30 | 10 | 0.68 |
| Li | pg/ml | 1.8E3 | 2.0E4 | 6.9E3 | 3.3E4 | 1.3E4 | 4.0E4 | 2.6E1 | 3.7E1 | 6.3E4 | 1.3E5 | 30 | 10 | 30 | 10 | 0.74 |
| Lj | pg/ml | 2.3E3 | 6.8E3 | 7.6E3 | 2.1E4 | 1.7E4 | 3.5E4 | 1.0E-9 | 2.4E2 | 8.8E4 | 1.0E5 | 30 | 10 | 30 | 10 | 0.57 |
| Nv | pg/ml | 4.4E3 | 7.5E3 | 1.7E4 | 2.1E4 | 3.1E4 | 3.8E4 | 8.4E1 | 5.4E2 | 1.5E5 | 1.3E5 | 30 | 10 | 30 | 10 | 0.63 |
| Nw | pg/ml | 8.6E3 | 1.4E4 | 1.5E4 | 3.3E4 | 1.6E4 | 6.3E4 | 5.7E2 | 1.6E3 | 6.2E4 | 2.1E5 | 30 | 10 | 30 | 10 | 0.56 |
| Nx | pg/ml | 2.4E2 | 3.3E2 | 3.9E2 | 6.8E2 | 5.0E2 | 8.8E2 | 1.0E-9 | 1.7E1 | 1.9E3 | 2.8E3 | 30 | 10 | 30 | 10 | 0.60 |
| Ny | pg/ml | 1.5E0 | 1.4E1 | 2.6E1 | 4.0E1 | 4.7E1 | 5.8E1 | 1.0E-9 | 1.0E-9 | 1.9E2 | 1.8E2 | 30 | 10 | 30 | 10 | 0.69 |
| Oe | pg/ml | 5.5E1 | 4.6E1 | 2.2E2 | 2.7E2 | 3.4E2 | 5.1E2 | 1.0E-9 | 1.0E-9 | 1.4E3 | 1.6E3 | 30 | 10 | 30 | 10 | 0.49 |
| Of | pg/ml | 5.9E1 | 5.1E2 | 3.3E3 | 5.0E3 | 1.4E4 | 7.6E3 | 1.0E-9 | 8.6E0 | 7.6E4 | 1.9E4 | 30 | 10 | 30 | 10 | 0.71 |
| Og | pg/ml | 9.1E-2 | 6.2E-2 | 1.7E-1 | 1.1E0 | 2.4E-1 | 1.9E0 | 1.0E-9 | 1.0E-9 | 1.0E0 | 5.0E0 | 30 | 10 | 30 | 10 | 0.49 |
| Oh | pg/ml | 3.3E0 | 4.6E0 | 1.5E1 | 3.3E1 | 2.6E1 | 8.8E1 | 1.2E-2 | 1.0E-9 | 9.1E1 | 2.8E2 | 30 | 10 | 30 | 10 | 0.52 |
| Oi | pg/ml | 2.7E0 | 9.4E-1 | 6.1E0 | 5.8E0 | 8.4E0 | 1.0E1 | 1.0E-9 | 1.0E-9 | 2.7E1 | 2.8E1 | 30 | 10 | 30 | 10 | 0.43 |
| Ok | pg/ml | 3.5E2 | 5.3E2 | 5.8E2 | 7.6E3 | 6.9E2 | 2.2E4 | 4.0E1 | 3.9E1 | 3.0E3 | 7.0E4 | 30 | 10 | 30 | 10 | 0.62 |
| Om | pg/ml | 4.4E2 | 8.5E2 | 1.8E3 | 2.2E3 | 6.5E3 | 3.8E3 | 1.0E-9 | 8.4E1 | 3.6E4 | 1.3E4 | 30 | 10 | 30 | 10 | 0.65 |
| On | pg/ml | 1.7E2 | 5.2E2 | 3.3E2 | 1.4E3 | 4.6E2 | 3.0E3 | 7.2E0 | 4.8E1 | 2.4E3 | 9.8E3 | 30 | 10 | 30 | 10 | 0.71 |
| Oy | pg/ml | 2.2E-1 | 7.7E-1 | 2.2E0 | 7.6E0 | 7.4E0 | 1.7E1 | 1.0E-9 | 1.0E-9 | 4.0E1 | 5.5E1 | 30 | 10 | 30 | 10 | 0.60 |
| Oz | pg/ml | 2.5E-2 | 1.2E-1 | 2.8E-1 | 2.1E-1 | 3.8E-1 | 2.5E-1 | 1.0E-9 | 1.0E-9 | 1.5E0 | 6.2E-1 | 30 | 10 | 30 | 10 | 0.47 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pa | pg/ml | 3.4E-1 | 4.4E-1 | 5.8E-1 | 1.3E0 | 7.9E-1 | 2.6E0 | 1.0E-9 | 1.0E-9 | 3.8E0 | 8.8E0 | 30 | 10 | 30 | 10 | 0.60 |
| Pb | pg/ml | 1.0E-9 | 3.5E-2 | 1.0E-1 | 1.0E-1 | 1.4E-1 | 1.3E-1 | 1.0E-9 | 1.0E-9 | 4.1E-1 | 3.2E-1 | 30 | 10 | 30 | 10 | 0.51 |
| Pc | pg/ml | 1.3E-1 | 5.0E-1 | 3.2E-1 | 5.2E-1 | 3.8E-1 | 5.2E-1 | 1.0E-9 | 1.0E-9 | 1.1E0 | 1.4E0 | 30 | 10 | 30 | 10 | 0.59 |
| Pd | pg/ml | 1.4E0 | 1.5E0 | 3.0E0 | 4.0E0 | 7.0E0 | 5.2E0 | 1.0E-9 | 2.9E-1 | 3.9E1 | 1.5E1 | 30 | 10 | 30 | 10 | 0.56 |
| Pe | pg/ml | 1.7E1 | 5.2E1 | 5.9E1 | 1.9E2 | 1.0E2 | 3.0E2 | 1.0E-9 | 1.9E0 | 4.3E2 | 8.0E2 | 30 | 10 | 30 | 10 | 0.68 |
| Pf | pg/ml | 9.2E-1 | 8.5E0 | 3.8E0 | 2.2E1 | 1.1E1 | 2.9E1 | 1.0E-9 | 4.7E-1 | 6.0E1 | 8.8E1 | 30 | 10 | 30 | 10 | 0.86 |
| Pg | pg/ml | 4.2E0 | 7.9E0 | 3.4E1 | 2.0E1 | 1.1E2 | 3.6E1 | 1.0E-9 | 4.0E0 | 6.0E2 | 1.2E2 | 30 | 10 | 30 | 10 | 0.68 |
| aA | mg/dL | 1.3E0 | 1.3E0 | 1.5E0 | 1.7E0 | 8.3E-1 | 1.0E0 | 4.0E-1 | 4.0E-1 | 3.9E0 | 3.6E0 | 43 | 15 | 43 | 15 | 0.53 |
| aC | mg/mL | 2.0E0 | 2.3E0 | 2.3E0 | 2.4E0 | 8.4E-1 | 1.3E0 | 1.3E0 | 1.2E0 | 4.0E0 | 5.1E0 | 18 | 7 | 18 | 7 | 0.50 |
| aD | ug/mL | 2.9E0 | 6.0E0 | 3.8E0 | 6.8E0 | 2.5E0 | 5.2E0 | 1.2E0 | 1.1E0 | 9.8E0 | 1.7E1 | 18 | 7 | 18 | 7 | 0.70 |
| aE | mg/mL | 5.0E-1 | 5.9E-1 | 5.2E-1 | 6.4E-1 | 1.1E-1 | 1.5E-1 | 3.8E-1 | 4.7E-1 | 8.2E-1 | 9.6E-1 | 18 | 7 | 18 | 7 | 0.81 |
| aF | ng/mL | 2.2E0 | 3.9E0 | 7.2E0 | 5.6E0 | 1.0E1 | 5.5E0 | 3.4E-1 | 6.3E-1 | 3.5E1 | 1.5E1 | 18 | 7 | 18 | 7 | 0.51 |
| aG | mg/mL | 1.3E-1 | 1.1E-1 | 1.5E-1 | 1.6E-1 | 7.7E-2 | 1.3E-1 | 7.0E-2 | 7.0E-2 | 3.1E-1 | 4.3E-1 | 18 | 7 | 18 | 7 | 0.45 |
| aH | ug/mL | 7.4E1 | 6.3E1 | 8.6E1 | 6.6E1 | 4.6E1 | 2.7E1 | 2.3E1 | 3.2E1 | 1.9E2 | 1.2E2 | 18 | 7 | 18 | 7 | 0.36 |
| aI | ug/mL | 1.7E2 | 1.8E2 | 1.8E2 | 1.7E2 | 6.1E1 | 4.7E1 | 9.1E1 | 7.5E1 | 3.0E2 | 2.2E2 | 18 | 7 | 18 | 7 | 0.46 |
| aJ | ug/mL | 3.9E0 | 5.5E0 | 4.4E0 | 6.8E0 | 3.4E0 | 4.6E0 | 7.6E-1 | 2.0E0 | 1.6E1 | 1.5E1 | 18 | 7 | 18 | 7 | 0.66 |
| aK | ng/mL | 1.5E0 | 1.4E0 | 2.0E0 | 1.3E0 | 1.9E0 | 4.2E-1 | 2.1E-1 | 4.4E-1 | 7.0E0 | 1.7E0 | 18 | 7 | 18 | 7 | 0.42 |
| aL | mg/mL | 7.8E-1 | 7.1E-1 | 8.2E-1 | 6.9E-1 | 2.5E-1 | 1.4E-1 | 4.5E-1 | 4.7E-1 | 1.3E0 | 8.2E-1 | 18 | 7 | 18 | 7 | 0.35 |
| aM | U/mL | 2.2E1 | 8.4E1 | 3.1E1 | 6.2E1 | 3.3E1 | 3.8E1 | 6.3E0 | 5.2E0 | 1.4E2 | 1.0E2 | 18 | 7 | 18 | 7 | 0.76 |
| aN | U/mL | 2.2E1 | 1.3E1 | 3.5E1 | 1.4E1 | 3.7E1 | 8.4E0 | 4.6E0 | 5.4E0 | 1.3E2 | 2.8E1 | 18 | 7 | 18 | 7 | 0.34 |
| aO | pg/mL | 1.2E2 | 2.4E2 | 5.4E2 | 9.1E2 | 1.0E3 | 1.3E3 | 6.0E-2 | 6.0E-2 | 3.6E3 | 3.5E3 | 18 | 7 | 18 | 7 | 0.60 |
| aP | ng/mL | 2.1E0 | 3.2E0 | 2.6E0 | 3.5E0 | 1.5E0 | 1.8E0 | 9.6E-1 | 1.3E0 | 6.4E0 | 6.1E0 | 18 | 7 | 18 | 7 | 0.66 |
| aQ | ng/mL | 3.0E-1 | 2.4E-1 | 4.7E-1 | 2.5E-1 | 4.0E-1 | 1.1E-1 | 7.2E-2 | 1.2E-1 | 1.3E0 | 4.5E-1 | 18 | 7 | 18 | 7 | 0.40 |
| aR | ng/mL | 1.7E0 | 2.3E0 | 2.6E0 | 2.9E0 | 2.6E0 | 2.5E0 | 4.5E-1 | 2.5E-1 | 1.0E1 | 5.9E0 | 18 | 7 | 18 | 7 | 0.51 |
| aS | ng/mL | 2.8E-1 | 3.3E-1 | 7.7E-1 | 1.8E0 | 1.3E0 | 3.1E0 | 4.2E-3 | 9.1E-2 | 4.9E0 | 8.7E0 | 18 | 7 | 18 | 7 | 0.63 |
| aU | pg/mL | 6.6E1 | 6.8E1 | 9.1E1 | 7.0E1 | 9.9E1 | 2.6E1 | 8.9E0 | 3.3E1 | 4.1E2 | 1.1E2 | 18 | 7 | 18 | 7 | 0.52 |
| aV | ng/mL | 7.0E-1 | 8.1E-1 | 7.7E-1 | 6.9E-1 | 5.1E-1 | 4.6E-1 | 5.0E-2 | 9.4E-2 | 2.0E0 | 1.4E0 | 18 | 7 | 18 | 7 | 0.46 |
| aW | pg/mL | 2.1E1 | 1.7E1 | 2.0E1 | 1.8E1 | 9.9E0 | 9.8E0 | 7.2E-2 | 7.7E0 | 3.3E1 | 3.4E1 | 18 | 7 | 18 | 7 | 0.40 |
| aX | ng/mL | 6.0E0 | 1.4E1 | 9.2E0 | 3.6E1 | 7.7E0 | 6.0E1 | 1.7E0 | 3.8E0 | 2.5E1 | 1.7E2 | 18 | 7 | 18 | 7 | 0.72 |
| aY | pg/mL | 5.2E1 | 7.8E1 | 6.5E1 | 1.1E2 | 3.5E1 | 7.0E1 | 2.5E1 | 3.2E1 | 1.5E2 | 2.0E2 | 18 | 7 | 18 | 7 | 0.74 |
| aZ | pg/mL | 1.3E2 | 1.7E2 | 1.0E3 | 2.7E2 | 1.8E3 | 1.8E2 | 8.8E0 | 8.3E1 | 5.4E3 | 5.4E2 | 18 | 7 | 18 | 7 | 0.60 |
| bA | ng/mL | 1.2E1 | 2.4E2 | 5.5E1 | 4.0E2 | 8.2E1 | 4.6E2 | 3.0E-2 | 1.7E1 | 3.2E2 | 1.3E3 | 18 | 7 | 18 | 7 | 0.83 |
| bB | ng/mL | 2.4E2 | 3.3E2 | 3.3E2 | 2.8E2 | 2.1E2 | 1.4E2 | 6.5E1 | 7.6E1 | 7.6E2 | 4.5E2 | 18 | 7 | 18 | 7 | 0.46 |
| bC | ng/mL | 3.2E2 | 4.3E2 | 5.6E2 | 6.8E2 | 6.0E2 | 6.7E2 | 9.8E0 | 1.3E2 | 2.4E3 | 1.8E3 | 18 | 7 | 18 | 7 | 0.58 |
| bE | mg/mL | 5.2E0 | 5.6E0 | 5.6E0 | 6.8E0 | 1.9E0 | 3.0E0 | 2.9E0 | 3.4E0 | 9.4E0 | 1.2E1 | 18 | 7 | 18 | 7 | 0.61 |
| bF | pg/mL | 2.2E1 | 4.0E1 | 5.6E1 | 9.3E2 | 7.5E1 | 2.4E3 | 2.1E0 | 1.1E1 | 3.0E2 | 6.3E3 | 18 | 7 | 18 | 7 | 0.62 |
| bG | ng/mL | 1.5E0 | 1.7E0 | 1.5E0 | 5.5E0 | 8.9E-1 | 1.1E1 | 4.1E-1 | 5.1E-1 | 3.3E0 | 3.0E1 | 18 | 7 | 18 | 7 | 0.59 |
| bH | pg/mL | 5.7E-1 | 5.7E-1 | 1.7E0 | 5.7E0 | 2.6E0 | 7.8E0 | 5.7E-1 | 5.7E-1 | 8.9E0 | 2.2E1 | 18 | 7 | 18 | 7 | 0.65 |
| bI | ng/mL | 4.0E-3 | 4.0E-3 | 1.2E-1 | 1.1E-1 | 2.9E-1 | 2.9E-1 | 4.0E-3 | 4.0E-3 | 9.8E-1 | 7.7E-1 | 18 | 7 | 18 | 7 | 0.48 |
| bJ | mg/mL | 1.9E0 | 1.9E0 | 2.2E0 | 2.5E0 | 1.9E0 | 1.4E0 | 2.5E-4 | 1.2E0 | 7.2E0 | 5.3E0 | 18 | 7 | 18 | 7 | 0.58 |
| bL | pg/mL | 1.8E0 | 5.5E0 | 3.8E0 | 5.5E0 | 4.3E0 | 4.1E0 | 4.6E-2 | 1.2E0 | 1.5E1 | 1.4E1 | 18 | 7 | 18 | 7 | 0.62 |
| bM | mg/mL | 1.8E0 | 2.4E0 | 2.3E0 | 2.8E0 | 1.9E0 | 1.3E0 | 5.0E-1 | 1.2E0 | 8.6E0 | 5.2E0 | 18 | 7 | 18 | 7 | 0.70 |
| bN | ng/mL | 3.1E1 | 2.7E1 | 1.2E2 | 2.6E1 | 1.9E2 | 2.1E1 | 1.4E-1 | 2.1E0 | 7.7E2 | 5.7E1 | 18 | 7 | 18 | 7 | 0.41 |
| bO | ng/mL | 4.0E-2 | 4.0E-2 | 8.2E0 | 3.5E0 | 2.4E1 | 6.4E0 | 4.0E-2 | 4.0E-2 | 1.0E2 | 1.7E1 | 18 | 7 | 18 | 7 | 0.45 |
| bP | mg/mL | 4.5E-1 | 8.2E-1 | 6.8E-1 | 1.1E0 | 5.7E-1 | 6.9E-1 | 1.9E-1 | 4.3E-1 | 2.2E0 | 2.5E0 | 18 | 7 | 18 | 7 | 0.78 |
| bQ | pg/mL | 1.7E1 | 1.6E1 | 3.0E1 | 5.6E1 | 5.0E1 | 6.3E1 | 1.5E-1 | 4.9E0 | 2.2E2 | 1.6E2 | 18 | 7 | 18 | 7 | 0.56 |
| bR | ng/mL | 1.2E-2 | 1.2E-2 | 1.3E-1 | 6.6E-2 | 2.8E-1 | 9.3E-2 | 1.2E-2 | 1.2E-2 | 1.2E0 | 2.2E-1 | 18 | 7 | 18 | 7 | 0.47 |
| bS | pg/mL | 9.4E-1 | 9.4E-1 | 3.9E0 | 9.4E-1 | 1.2E1 | 0.0E0 | 9.4E-1 | 9.4E-1 | 5.4E1 | 9.4E-1 | 18 | 7 | 18 | 7 | 0.47 |
| bU | ng/mL | 8.3E-2 | 9.2E-2 | 2.2E-1 | 1.1E-1 | 4.1E-1 | 1.2E-1 | 1.3E-2 | 1.3E-2 | 1.7E0 | 3.4E-1 | 18 | 7 | 18 | 7 | 0.46 |
| bV | pg/mL | 4.9E2 | 9.6E2 | 6.2E2 | 8.3E2 | 3.3E2 | 3.6E2 | 1.7E2 | 3.7E2 | 1.4E3 | 1.3E3 | 18 | 7 | 18 | 7 | 0.66 |
| bW | pg/mL | 2.9E2 | 4.0E2 | 3.4E2 | 4.4E2 | 2.1E2 | 2.7E2 | 9.2E1 | 1.5E2 | 9.7E2 | 9.9E2 | 18 | 7 | 18 | 7 | 0.63 |
| bX | ng/mL | 2.5E-5 | 2.5E-5 | 2.5E-3 | 5.7E-4 | 3.6E-3 | 1.4E-3 | 2.5E-5 | 2.5E-5 | 1.1E-2 | 3.9E-3 | 18 | 7 | 18 | 7 | 0.37 |
| bZ | pg/mL | 2.9E2 | 1.6E3 | 6.0E2 | 7.4E3 | 8.4E2 | 1.6E4 | 1.5E-1 | 1.8E2 | 2.7E3 | 4.3E4 | 18 | 7 | 18 | 7 | 0.71 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| cA | pg/mL | 6.0E-1 | 6.0E-1 | 1.1E0 | 6.0E-1 | 1.9E0 | 0.0E0 | 6.0E-1 | 6.0E-1 | 8.8E0 | 6.0E-1 | 18 | 7 | 18 | 7 | 0.47 |
| cB | ng/mL | 2.8E-2 | 4.0E-2 | 7.8E-2 | 4.2E-2 | 1.3E-1 | 3.1E-2 | 1.7E-3 | 1.7E-3 | 5.3E-1 | 7.9E-2 | 18 | 7 | 18 | 7 | 0.51 |
| cC | pg/mL | 3.3E1 | 3.8E1 | 3.5E1 | 4.3E1 | 3.7E1 | 1.3E1 | 1.0E0 | 2.8E1 | 1.4E2 | 6.7E1 | 18 | 7 | 18 | 7 | 0.59 |
| cD | pg/mL | 3.1E0 | 7.6E0 | 9.4E0 | 9.8E0 | 1.7E1 | 1.3E1 | 3.3E-1 | 3.3E-1 | 6.9E1 | 3.9E1 | 18 | 7 | 18 | 7 | 0.61 |
| cE | pg/mL | 5.8E1 | 5.7E1 | 9.1E1 | 2.5E2 | 1.4E2 | 4.5E2 | 1.2E-1 | 6.5E0 | 6.1E2 | 1.3E3 | 18 | 7 | 18 | 7 | 0.60 |
| cF | pg/mL | 5.3E-1 | 5.3E-1 | 2.2E1 | 2.3E0 | 5.0E1 | 4.6E0 | 5.3E-1 | 5.3E-1 | 2.2E2 | 1.3E1 | 18 | 7 | 18 | 7 | 0.34 |
| cG | pg/mL | 5.8E1 | 1.1E2 | 7.1E1 | 1.0E2 | 6.0E1 | 7.1E1 | 1.1E1 | 2.6E1 | 2.6E2 | 2.3E2 | 18 | 7 | 18 | 7 | 0.66 |
| cH | uIU/mL | 2.8E0 | 9.9E-1 | 5.0E0 | 1.1E1 | 6.0E0 | 2.0E1 | 8.6E-3 | 2.5E-1 | 2.0E1 | 5.3E1 | 18 | 7 | 18 | 7 | 0.51 |
| cI | ng/mL | 6.0E0 | 9.2E0 | 1.5E1 | 1.9E1 | 2.2E1 | 2.3E1 | 7.1E-2 | 1.2E0 | 8.8E1 | 6.6E1 | 18 | 7 | 18 | 7 | 0.61 |
| cJ | ug/mL | 8.1E1 | 7.3E1 | 1.5E2 | 8.2E1 | 1.6E2 | 5.3E1 | 1.7E1 | 3.1E1 | 6.2E2 | 1.9E2 | 18 | 7 | 18 | 7 | 0.43 |
| cK | ng/mL | 3.8E-3 | 3.8E-3 | 3.8E-3 | 3.8E-3 | 0.0E0 | 0.0E0 | 3.8E-3 | 3.8E-3 | 3.8E-3 | 3.8E-3 | 18 | 7 | 18 | 7 | 0.50 |
| cL | pg/mL | 1.9E2 | 2.1E2 | 6.8E2 | 2.5E2 | 1.7E3 | 1.4E2 | 4.8E1 | 1.5E2 | 7.4E3 | 5.6E2 | 18 | 7 | 18 | 7 | 0.58 |
| cM | pg/mL | 3.3E2 | 3.2E2 | 3.1E2 | 3.1E2 | 1.4E2 | 1.2E2 | 4.0E1 | 1.4E2 | 5.3E2 | 4.9E2 | 18 | 7 | 18 | 7 | 0.48 |
| cN | pg/mL | 1.4E2 | 1.6E2 | 1.4E2 | 1.7E2 | 5.0E1 | 5.8E1 | 4.6E1 | 1.0E2 | 2.6E2 | 2.8E2 | 18 | 7 | 18 | 7 | 0.60 |
| cO | pg/mL | 1.7E2 | 2.1E2 | 1.9E2 | 2.5E2 | 8.8E1 | 1.1E2 | 6.1E1 | 1.1E2 | 3.7E2 | 4.2E2 | 18 | 7 | 18 | 7 | 0.70 |
| cP | ng/mL | 2.5E3 | 4.3E3 | 2.6E3 | 3.7E3 | 7.4E2 | 1.4E3 | 1.7E3 | 1.4E3 | 4.4E3 | 5.1E3 | 18 | 7 | 18 | 7 | 0.73 |
| cQ | ng/mL | 7.9E-2 | 1.5E-1 | 1.1E-1 | 1.9E-1 | 1.3E-1 | 2.6E-1 | 2.0E-3 | 2.0E-3 | 4.8E-1 | 7.3E-1 | 18 | 7 | 18 | 7 | 0.58 |
| cR | ng/mL | 2.8E2 | 5.5E2 | 2.9E2 | 4.6E2 | 1.5E2 | 2.0E2 | 9.5E1 | 1.2E2 | 6.2E2 | 6.6E2 | 18 | 7 | 18 | 7 | 0.75 |
| cS | ng/mL | 3.1E2 | 6.3E2 | 3.2E2 | 6.7E2 | 2.0E2 | 3.8E2 | 8.1E1 | 1.9E2 | 8.3E2 | 1.3E3 | 18 | 7 | 18 | 7 | 0.78 |
| cT | ng/mL | 3.6E1 | 6.5E2 | 1.7E2 | 6.6E2 | 3.3E2 | 6.7E2 | 8.0E0 | 3.2E1 | 1.3E3 | 1.9E3 | 18 | 7 | 18 | 7 | 0.81 |
| cU | ng/mL | 8.7E1 | 5.4E1 | 9.8E1 | 1.0E2 | 5.9E1 | 8.4E1 | 1.6E1 | 4.3E1 | 2.1E2 | 2.3E2 | 18 | 7 | 18 | 7 | 0.44 |
| cV | ng/mL | 1.9E-1 | 2.6E-1 | 7.8E-1 | 5.5E-1 | 2.2E0 | 8.7E-1 | 4.1E-2 | 7.4E-2 | 9.7E0 | 2.5E0 | 18 | 7 | 18 | 7 | 0.67 |
| cW | mIU/mL | 3.9E-2 | 8.9E-2 | 4.9E-2 | 9.4E-2 | 3.1E-2 | 4.8E-2 | 1.0E-2 | 3.6E-2 | 1.3E-1 | 1.6E-1 | 18 | 7 | 18 | 7 | 0.81 |
| cX | ng/mL | 3.5E-1 | 7.3E-2 | 4.7E0 | 2.8E-1 | 9.8E0 | 3.8E-1 | 2.3E-4 | 1.4E-2 | 2.8E1 | 1.1E0 | 18 | 7 | 18 | 7 | 0.44 |
| cY | ng/mL | 8.3E0 | 8.1E0 | 1.0E1 | 7.6E0 | 8.1E0 | 4.1E0 | 1.1E0 | 1.5E0 | 2.9E1 | 1.3E1 | 18 | 7 | 18 | 7 | 0.47 |
| cZ | ug/mL | 1.5E1 | 1.9E1 | 1.5E1 | 1.8E1 | 5.2E0 | 6.1E0 | 6.2E0 | 7.0E0 | 2.5E1 | 2.6E1 | 18 | 7 | 18 | 7 | 0.67 |
| dA | pg/mL | 3.1E2 | 5.5E2 | 3.5E2 | 4.7E2 | 1.4E2 | 2.4E2 | 1.7E2 | 1.7E2 | 5.8E2 | 7.7E2 | 18 | 7 | 18 | 7 | 0.67 |
| dB | ug/mL | 1.2E1 | 2.3E1 | 1.5E1 | 2.3E1 | 1.2E1 | 4.7E0 | 2.6E0 | 1.6E1 | 4.0E1 | 2.9E1 | 18 | 7 | 18 | 7 | 0.73 |
| dC | nmol/L | 3.1E1 | 4.1E1 | 3.6E1 | 4.3E1 | 1.7E1 | 2.0E1 | 1.5E1 | 2.3E1 | 8.7E1 | 8.6E1 | 18 | 7 | 18 | 7 | 0.65 |
| dD | ug/mL | 3.7E1 | 3.1E1 | 3.6E1 | 3.6E1 | 1.0E1 | 1.5E1 | 2.0E1 | 2.2E1 | 5.2E1 | 6.4E1 | 18 | 7 | 18 | 7 | 0.48 |
| dE | ng/mL | 8.4E-3 | 4.4E-1 | 1.7E-1 | 1.2E0 | 2.4E-1 | 1.1E0 | 8.4E-3 | 3.4E-1 | 6.6E-1 | 3.3E0 | 18 | 7 | 18 | 7 | 0.86 |
| dF | ng/mL | 2.9E2 | 4.7E2 | 3.3E2 | 4.7E2 | 2.5E2 | 2.2E2 | 8.6E1 | 1.9E2 | 1.2E3 | 7.7E2 | 18 | 7 | 18 | 7 | 0.70 |
| dG | ng/mL | 1.6E1 | 2.4E1 | 1.6E1 | 2.2E1 | 7.4E0 | 8.3E0 | 3.3E0 | 1.1E1 | 3.3E1 | 3.2E1 | 18 | 7 | 18 | 7 | 0.68 |
| dH | pg/mL | 7.7E0 | 9.1E0 | 1.1E1 | 1.1E1 | 8.6E0 | 4.9E0 | 4.0E-2 | 6.6E0 | 3.0E1 | 1.9E1 | 18 | 7 | 18 | 7 | 0.52 |
| dI | pg/mL | 4.6E-1 | 4.6E-1 | 8.4E-1 | 1.9E0 | 8.8E-1 | 2.4E0 | 4.6E-1 | 4.6E-1 | 2.9E0 | 6.2E0 | 18 | 7 | 18 | 7 | 0.58 |
| dJ | ng/mL | 1.9E0 | 2.5E0 | 2.0E0 | 2.2E0 | 1.0E0 | 9.5E-1 | 3.7E-1 | 8.9E-1 | 4.0E0 | 3.4E0 | 18 | 7 | 18 | 7 | 0.57 |
| dK | uIU/mL | 2.0E0 | 1.2E0 | 3.0E0 | 1.7E0 | 3.7E0 | 1.1E0 | 4.0E-2 | 4.3E-1 | 1.6E1 | 3.1E0 | 18 | 7 | 18 | 7 | 0.43 |
| dL | ng/mL | 9.9E2 | 1.3E3 | 1.1E3 | 1.2E3 | 3.7E2 | 3.3E2 | 4.9E2 | 5.8E2 | 1.8E3 | 1.5E3 | 18 | 7 | 18 | 7 | 0.59 |
| dM | pg/mL | 1.3E3 | 1.2E3 | 1.5E3 | 1.9E3 | 7.7E2 | 1.2E3 | 4.7E2 | 7.1E2 | 3.3E3 | 3.6E3 | 18 | 7 | 18 | 7 | 0.57 |
| dN | ug/mL | 9.6E1 | 1.3E2 | 9.9E1 | 1.4E2 | 2.3E1 | 4.7E1 | 6.4E1 | 9.4E1 | 1.5E2 | 2.2E2 | 18 | 7 | 18 | 7 | 0.81 |

Figure 27.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 8.3E1 | 9.2E1 | 9.1E1 | 1.0E2 | 5.5E1 | 5.8E1 | 1.0E1 | 2.6E1 | 2.6E2 | 2.4E2 | 61 | 21 | 61 | 21 | 0.57 |
| Ad | ug/mL | 5.6E-2 | 6.2E-2 | 9.5E-2 | 9.8E-2 | 9.6E-2 | 9.1E-2 | 9.4E-4 | 9.4E-3 | 3.6E-1 | 3.2E-1 | 43 | 16 | 43 | 16 | 0.54 |
| Af | ng/mL | 1.6E0 | 7.9E-1 | 2.1E1 | 1.9E1 | 6.9E1 | 6.1E1 | 1.7E-3 | 1.7E-3 | 4.0E2 | 2.5E2 | 43 | 16 | 43 | 16 | 0.40 |
| Aj | ug/mL | 2.7E0 | 5.0E-1 | 3.0E0 | 1.6E0 | 2.7E0 | 2.1E0 | 4.1E-3 | 3.5E-3 | 6.1E0 | 5.8E0 | 43 | 16 | 43 | 16 | 0.37 |
| Al | mg/mL | 1.2E-4 | 1.6E-4 | 3.2E-4 | 3.3E-4 | 4.6E-4 | 4.1E-4 | 9.0E-6 | 7.6E-6 | 1.9E-3 | 1.5E-3 | 43 | 16 | 43 | 16 | 0.54 |
| An | U/mL | 7.5E1 | 5.0E1 | 2.2E2 | 3.6E2 | 3.9E2 | 7.6E2 | 7.7E-1 | 9.6E-1 | 2.0E3 | 3.0E3 | 43 | 16 | 43 | 16 | 0.45 |
| Ao | pg/mL | 1.1E2 | 9.8E1 | 2.2E2 | 1.6E2 | 5.7E2 | 1.5E2 | 6.7E0 | 9.9E0 | 3.8E3 | 6.0E2 | 43 | 16 | 43 | 16 | 0.54 |
| Ap | ng/mL | 3.9E1 | 4.1E1 | 5.2E1 | 4.4E1 | 4.9E1 | 2.8E1 | 8.4E-5 | 8.7E0 | 2.5E2 | 1.0E2 | 43 | 16 | 43 | 16 | 0.49 |
| Ar | ng/mL | 1.2E0 | 2.2E0 | 5.6E0 | 3.8E0 | 1.1E1 | 5.8E0 | 1.1E-1 | 3.4E-3 | 5.1E1 | 1.9E1 | 43 | 16 | 43 | 16 | 0.52 |
| As | ng/mL | 1.0E-2 | 1.0E-2 | 1.7E-2 | 1.8E-2 | 2.4E-2 | 2.3E-2 | 1.7E-3 | 1.7E-3 | 1.3E-1 | 8.2E-2 | 43 | 16 | 43 | 16 | 0.52 |
| Aw | pg/mL | 1.6E1 | 1.6E1 | 1.7E1 | 1.6E1 | 5.9E0 | 5.9E0 | 9.3E0 | 2.9E-2 | 3.7E1 | 2.5E1 | 43 | 16 | 43 | 16 | 0.49 |
| Ax | ng/mL | 2.7E0 | 7.7E0 | 1.3E1 | 5.2E1 | 3.0E1 | 1.5E2 | 4.9E-2 | 1.2E-2 | 1.5E2 | 6.2E2 | 43 | 16 | 43 | 16 | 0.60 |
| Ba | ng/mL | 1.0E2 | 7.8E1 | 6.9E2 | 1.9E2 | 1.5E3 | 2.7E2 | 3.1E0 | 6.3E0 | 7.5E3 | 1.0E3 | 43 | 16 | 43 | 16 | 0.47 |
| Bb | ng/mL | 5.7E0 | 6.4E0 | 6.4E0 | 8.2E0 | 5.3E0 | 6.9E0 | 4.1E-1 | 4.2E-1 | 2.0E1 | 1.9E1 | 43 | 16 | 43 | 16 | 0.56 |
| Bc | ng/mL | 4.0E1 | 5.6E1 | 1.4E2 | 6.7E1 | 2.4E2 | 4.9E1 | 2.2E-1 | 8.0E0 | 1.2E3 | 1.6E2 | 43 | 16 | 43 | 16 | 0.55 |
| Bg | ng/mL | 7.2E-2 | 1.1E-1 | 2.8E0 | 7.8E-1 | 1.1E1 | 1.3E0 | 5.3E-4 | 2.6E-2 | 7.1E1 | 3.7E0 | 43 | 16 | 43 | 16 | 0.52 |
| Bn | ng/mL | 7.0E-1 | 5.6E-2 | 2.0E0 | 1.2E0 | 2.5E0 | 2.2E0 | 5.6E-2 | 5.6E-2 | 8.6E0 | 6.5E0 | 43 | 16 | 43 | 16 | 0.39 |
| Bo | ng/mL | 9.6E0 | 1.7E1 | 1.3E1 | 1.8E1 | 1.3E1 | 1.4E1 | 1.6E-2 | 1.6E-2 | 4.6E1 | 4.1E1 | 43 | 16 | 43 | 16 | 0.63 |
| Ch | uIU/mL | 2.2E0 | 7.3E-1 | 1.8E1 | 9.1E0 | 4.2E1 | 2.6E1 | 3.9E-2 | 1.2E-1 | 1.9E2 | 1.1E2 | 43 | 16 | 43 | 16 | 0.37 |
| Co | pg/mL | 4.2E1 | 5.3E1 | 5.0E2 | 1.2E2 | 2.5E3 | 2.0E2 | 1.5E-1 | 1.6E1 | 1.7E4 | 8.2E2 | 43 | 16 | 43 | 16 | 0.52 |
| Cp | ng/mL | 2.8E1 | 2.3E1 | 3.6E1 | 2.9E1 | 3.0E1 | 1.7E1 | 1.1E1 | 1.0E1 | 1.9E2 | 6.9E1 | 43 | 16 | 43 | 16 | 0.42 |
| Cq | ng/mL | 3.1E-2 | 3.5E-2 | 1.6E-1 | 8.8E-2 | 7.8E-1 | 1.3E-1 | 8.0E-4 | 8.0E-4 | 5.1E0 | 4.0E-1 | 43 | 16 | 43 | 16 | 0.54 |
| Cs | ng/mL | 7.9E1 | 3.2E2 | 3.0E2 | 1.6E3 | 5.8E2 | 4.4E3 | 1.3E0 | 9.1E0 | 3.0E3 | 1.8E4 | 43 | 16 | 43 | 16 | 0.62 |
| Ct | ng/mL | 5.9E-1 | 6.9E-2 | 5.0E1 | 3.9E0 | 1.3E2 | 1.2E1 | 1.8E-2 | 1.5E-2 | 6.2E2 | 4.9E1 | 43 | 16 | 43 | 16 | 0.30 |
| Cu | ng/mL | 2.8E-1 | 3.3E-1 | 4.2E-1 | 6.1E-1 | 6.7E-1 | 1.1E0 | 3.5E-2 | 5.6E-2 | 4.5E0 | 4.5E0 | 43 | 16 | 43 | 16 | 0.56 |
| Cv | ng/mL | 9.1E0 | 1.1E1 | 2.8E1 | 4.1E1 | 5.2E1 | 7.7E1 | 2.6E-2 | 5.1E-2 | 3.1E2 | 2.9E2 | 43 | 16 | 43 | 16 | 0.53 |
| Cw | mIU/mL | 2.9E-2 | 3.4E-2 | 3.8E-2 | 3.7E-2 | 2.9E-2 | 3.0E-2 | 4.8E-3 | 4.1E-3 | 1.4E-1 | 1.0E-1 | 43 | 16 | 43 | 16 | 0.48 |
| Cx | ng/mL | 1.3E0 | 2.9E-2 | 6.7E1 | 7.5E1 | 1.1E2 | 1.4E2 | 9.3E-5 | 9.3E-5 | 3.9E2 | 3.9E2 | 43 | 16 | 43 | 16 | 0.44 |
| Db | ug/mL | 5.3E0 | 6.7E0 | 7.6E0 | 6.9E0 | 1.1E1 | 5.1E0 | 8.2E-1 | 1.1E0 | 5.9E1 | 2.1E1 | 43 | 16 | 43 | 16 | 0.58 |
| Dc | nmol/L | 3.1E-2 | 4.2E-2 | 9.3E-2 | 9.5E-2 | 2.5E-1 | 1.9E-1 | 3.0E-4 | 1.0E-3 | 1.6E0 | 7.7E-1 | 43 | 16 | 43 | 16 | 0.58 |
| Dd | ug/mL | 8.9E-2 | 1.5E-1 | 2.6E-1 | 1.9E-1 | 4.1E-1 | 1.8E-1 | 1.1E-3 | 3.3E-3 | 1.9E0 | 5.6E-1 | 43 | 16 | 43 | 16 | 0.51 |
| De | ng/mL | 3.4E-3 | 3.4E-3 | 1.0E-1 | 5.1E-2 | 1.4E-1 | 8.5E-2 | 3.4E-3 | 3.4E-3 | 6.2E-1 | 3.1E-1 | 43 | 16 | 43 | 16 | 0.40 |
| Dg | ng/mL | 4.3E1 | 3.9E1 | 5.8E1 | 5.0E1 | 5.0E1 | 3.7E1 | 1.0E-1 | 1.1E0 | 1.9E2 | 1.2E2 | 43 | 16 | 43 | 16 | 0.49 |
| Di | pg/mL | 1.8E0 | 2.3E0 | 2.2E0 | 2.5E0 | 2.2E0 | 2.1E0 | 1.8E-1 | 1.8E-1 | 7.8E0 | 6.0E0 | 43 | 16 | 43 | 16 | 0.55 |
| Dk | uIU/mL | 1.8E-2 | 1.5E-2 | 4.4E-2 | 3.6E-2 | 6.9E-2 | 5.8E-2 | 1.1E-4 | 5.8E-3 | 3.4E-1 | 2.4E-1 | 43 | 16 | 43 | 16 | 0.46 |
| Dl | ng/mL | 2.6E2 | 2.0E2 | 3.7E2 | 2.8E2 | 2.9E2 | 2.9E2 | 2.5E0 | 5.5E0 | 1.1E3 | 1.1E3 | 43 | 16 | 43 | 16 | 0.40 |
| Dp | ng/ml | 2.7E0 | 3.3E0 | 5.9E0 | 8.8E0 | 8.2E0 | 1.4E1 | 3.7E-3 | 3.7E-3 | 3.7E1 | 5.6E1 | 43 | 16 | 43 | 16 | 0.53 |
| Ef | ng/ml | 2.0E-1 | 8.5E-2 | 9.6E-1 | 5.4E-1 | 1.7E0 | 1.3E0 | 2.1E-3 | 1.3E-2 | 8.6E0 | 5.2E0 | 43 | 17 | 43 | 17 | 0.43 |
| Wm | % | 3.8E-1 | 5.9E-1 | 3.1E0 | 1.0E2 | 7.4E0 | 2.8E2 | 8.5E-2 | 8.5E-2 | 4.0E1 | 9.3E2 | 43 | 17 | 43 | 17 | 0.55 |
| Ed | pg/ml | 5.2E-1 | 2.8E0 | 2.0E1 | 7.1E1 | 4.1E1 | 1.3E2 | 5.2E-1 | 5.2E-1 | 2.2E2 | 5.0E2 | 43 | 16 | 43 | 16 | 0.62 |
| Tj | pg/mL | 3.7E-1 | 3.7E-1 | 6.2E1 | 2.3E1 | 3.5E2 | 7.4E1 | 3.6E-1 | 3.7E-1 | 2.3E3 | 3.1E2 | 43 | 17 | 43 | 17 | 0.58 |
| Po | pg/ml | 8.6E-1 | 2.0E0 | 1.0E1 | 1.2E1 | 2.6E1 | 2.9E1 | 2.6E-2 | 2.6E-2 | 1.8E2 | 1.4E2 | 61 | 24 | 61 | 24 | 0.51 |
| Ti | ug/mL | 5.9E0 | 6.8E0 | 6.3E0 | 6.0E0 | 5.1E0 | 4.5E0 | 1.9E-1 | 8.0E-1 | 1.7E1 | 1.4E1 | 30 | 8 | 30 | 8 | 0.52 |
| Et | ng/ml | 1.9E3 | 2.3E3 | 2.0E3 | 2.5E3 | 1.1E3 | 1.2E3 | 1.8E2 | 5.0E2 | 4.4E3 | 5.0E3 | 61 | 24 | 61 | 24 | 0.61 |
| Th | ug/mL | 1.4E0 | 8.1E-1 | 1.6E0 | 1.0E0 | 9.9E-1 | 1.2E0 | 2.0E-1 | 2.6E-3 | 4.6E0 | 3.9E0 | 30 | 8 | 30 | 8 | 0.26 |
| Fa | ng/ml | 3.1E1 | 8.8E1 | 8.8E1 | 4.4E2 | 1.4E2 | 1.0E3 | 1.5E0 | 5.9E0 | 7.5E2 | 3.7E3 | 42 | 17 | 42 | 17 | 0.69 |
| Ez | ng/ml | 7.0E0 | 2.5E0 | 2.2E1 | 5.8E0 | 4.6E1 | 7.7E0 | 1.3E-2 | 9.5E-2 | 2.4E2 | 2.5E1 | 43 | 16 | 43 | 16 | 0.37 |
| Fb | ng/ml | 2.5E1 | 3.2E1 | 2.3E1 | 2.8E1 | 9.0E0 | 1.1E1 | 2.7E0 | 5.9E-1 | 3.8E1 | 4.0E1 | 42 | 17 | 42 | 17 | 0.69 |
| Ex | ng/ml | 7.1E-2 | 1.4E-1 | 1.7E-1 | 1.5E-1 | 3.1E-1 | 1.2E-1 | 1.7E-4 | 1.7E-4 | 1.5E0 | 4.4E-1 | 24 | 12 | 24 | 12 | 0.62 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Fn | ng/ml | 2.1E-1 | 3.6E0 | 4.0E0 | 5.6E0 | 6.5E0 | 5.9E0 | 2.1E-1 | 2.1E-1 | 2.1E1 | 1.6E1 | 43 | 16 | 43 | 16 | 0.63 |
| Fp | ng/ml | 2.1E1 | 4.6E1 | 3.5E1 | 4.4E1 | 3.4E1 | 3.6E1 | 6.6E-1 | 1.6E0 | 1.3E2 | 1.2E2 | 62 | 25 | 62 | 25 | 0.58 |
| Fr | ng/ml | 4.2E4 | 5.8E4 | 1.1E5 | 1.6E5 | 1.5E5 | 2.2E5 | 1.9E3 | 2.5E3 | 7.2E5 | 7.9E5 | 62 | 25 | 62 | 25 | 0.57 |
| Fw | pg/ml | 8.5E-1 | 1.1E0 | 1.3E1 | 8.7E1 | 3.7E1 | 2.4E2 | 1.2E-1 | 1.2E-1 | 2.3E2 | 9.1E2 | 42 | 17 | 42 | 17 | 0.59 |
| Fy | ng/ml | 4.7E1 | 4.7E1 | 6.9E1 | 9.3E1 | 6.0E1 | 1.3E2 | 1.2E-1 | 2.7E0 | 2.0E2 | 5.0E2 | 43 | 16 | 43 | 16 | 0.51 |
| Gl | pg/ml | 9.8E3 | 1.0E4 | 1.2E4 | 1.3E4 | 9.3E3 | 1.0E4 | 4.0E2 | 1.7E3 | 3.3E4 | 3.0E4 | 42 | 17 | 42 | 17 | 0.50 |
| Gp | U/ml | 1.3E0 | 8.5E-1 | 3.9E0 | 4.3E0 | 5.5E0 | 1.1E1 | 1.3E-3 | 1.5E-2 | 2.3E1 | 4.8E1 | 43 | 17 | 43 | 17 | 0.40 |
| Gz | ug/ml | 1.2E0 | 9.8E-1 | 5.4E0 | 5.1E0 | 5.3E0 | 6.8E0 | 1.6E-1 | 1.3E-1 | 1.1E1 | 1.9E1 | 23 | 12 | 23 | 12 | 0.47 |
| Ha | ng/ml | 2.7E0 | 5.0E0 | 1.0E1 | 1.3E1 | 2.0E1 | 2.5E1 | 1.7E-2 | 1.7E-2 | 1.0E2 | 1.0E2 | 43 | 16 | 43 | 16 | 0.58 |
| Nm | pg/ml | 2.4E4 | 2.3E4 | 3.5E4 | 3.6E4 | 3.6E4 | 3.8E4 | 1.0E-9 | 1.0E-9 | 1.5E5 | 1.2E5 | 62 | 24 | 62 | 24 | 0.49 |
| Nn | pg/ml | 1.7E2 | 1.6E2 | 6.4E2 | 1.2E3 | 1.3E3 | 3.4E3 | 1.0E-9 | 3.3E0 | 8.0E3 | 1.7E4 | 62 | 24 | 62 | 24 | 0.52 |
| No | pg/ml | 2.1E1 | 2.3E1 | 3.4E1 | 4.4E1 | 4.7E1 | 8.2E1 | 1.0E-9 | 3.3E-1 | 2.6E2 | 4.1E2 | 62 | 24 | 62 | 24 | 0.52 |
| Nq | pg/ml | 3.1E-1 | 1.7E0 | 8.9E0 | 1.2E1 | 2.6E1 | 3.4E1 | 1.0E-9 | 1.0E-9 | 1.7E2 | 1.7E2 | 62 | 24 | 62 | 24 | 0.57 |
| Nr | pg/ml | 1.8E0 | 6.2E0 | 2.8E1 | 2.3E1 | 1.0E2 | 6.3E1 | 1.0E-9 | 1.0E-9 | 7.2E2 | 3.1E2 | 62 | 24 | 62 | 24 | 0.55 |
| Ns | pg/ml | 1.0E-9 | 1.0E-9 | 4.3E0 | 1.5E-1 | 1.9E1 | 7.3E-1 | 1.0E-9 | 1.0E-9 | 1.0E2 | 3.6E0 | 62 | 24 | 62 | 24 | 0.49 |
| Nt | pg/ml | 1.5E2 | 1.4E2 | 1.7E2 | 1.5E2 | 9.6E1 | 7.1E1 | 4.7E1 | 2.3E1 | 4.3E2 | 3.1E2 | 62 | 24 | 62 | 24 | 0.45 |
| Nu | pg/ml | 4.0E1 | 4.8E1 | 8.9E1 | 8.1E1 | 1.2E2 | 9.7E1 | 1.0E-9 | 1.0E-9 | 6.3E2 | 3.4E2 | 62 | 24 | 62 | 24 | 0.49 |
| Lu | pg/ml | 1.0E4 | 1.0E4 | 1.2E4 | 1.1E4 | 1.3E4 | 7.1E3 | 7.1E2 | 1.9E3 | 8.4E4 | 3.6E4 | 62 | 25 | 62 | 25 | 0.58 |
| Lv | pg/ml | 8.8E0 | 1.0E-9 | 1.9E1 | 1.8E1 | 2.7E1 | 3.5E1 | 1.0E-9 | 1.0E-9 | 1.2E2 | 1.6E2 | 62 | 25 | 62 | 25 | 0.46 |
| Lw | pg/ml | 1.0E-9 | 1.0E-9 | 3.2E-1 | 1.0E-9 | 1.9E0 | 0.0E0 | 1.0E-9 | 1.0E-9 | 1.4E1 | 1.0E-9 | 62 | 25 | 62 | 25 | 0.48 |
| Lx | pg/ml | 1.1E1 | 1.1E2 | 2.1E2 | 2.9E2 | 6.2E2 | 8.8E2 | 1.0E-9 | 1.0E-9 | 4.4E3 | 4.5E3 | 62 | 25 | 62 | 25 | 0.62 |
| Ly | pg/ml | 1.0E-9 | 1.1E1 | 2.0E1 | 1.7E1 | 3.2E1 | 2.0E1 | 1.0E-9 | 1.0E-9 | 1.6E2 | 7.0E1 | 62 | 25 | 62 | 25 | 0.52 |
| Lz | pg/ml | 1.0E-9 | 1.0E-9 | 5.7E0 | 1.6E0 | 2.9E1 | 7.3E0 | 1.0E-9 | 1.0E-9 | 1.9E2 | 3.7E1 | 62 | 25 | 62 | 25 | 0.49 |
| Ma | pg/ml | 4.0E2 | 5.7E2 | 1.4E3 | 8.8E2 | 2.5E3 | 1.3E3 | 1.0E-9 | 2.6E1 | 1.5E4 | 5.9E3 | 62 | 25 | 62 | 25 | 0.49 |
| Mb | pg/ml | 3.3E1 | 2.8E1 | 3.8E1 | 3.5E1 | 1.9E1 | 1.6E1 | 1.4E1 | 1.4E1 | 1.1E2 | 6.1E1 | 62 | 25 | 62 | 25 | 0.44 |
| Mc | pg/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 62 | 25 | 62 | 25 | 0.50 |
| Md | pg/ml | 1.0E-9 | 1.0E-9 | 8.9E-1 | 2.2E-1 | 3.3E0 | 1.1E0 | 1.0E-9 | 1.0E-9 | 1.6E1 | 5.5E0 | 62 | 25 | 62 | 25 | 0.46 |
| Me | pg/ml | 2.6E1 | 2.8E1 | 2.6E1 | 2.5E1 | 1.8E1 | 1.4E1 | 1.0E-9 | 1.2E0 | 7.2E1 | 5.3E1 | 62 | 25 | 62 | 25 | 0.50 |
| Mf | pg/ml | 1.0E-9 | 1.0E-9 | 6.7E-1 | 3.2E-1 | 1.6E0 | 9.0E-1 | 1.0E-9 | 1.0E-9 | 7.1E0 | 4.4E0 | 62 | 25 | 62 | 25 | 0.50 |
| Mg | pg/ml | 2.0E0 | 1.8E0 | 8.6E0 | 6.6E0 | 1.3E1 | 1.0E1 | 1.0E-9 | 1.0E-9 | 5.9E1 | 3.9E1 | 62 | 25 | 62 | 25 | 0.46 |
| Mh | pg/ml | 1.0E-9 | 1.0E-9 | 9.1E-1 | 2.3E0 | 3.9E0 | 8.5E0 | 1.0E-9 | 1.0E-9 | 2.9E1 | 4.2E1 | 62 | 25 | 62 | 25 | 0.55 |
| Mi | pg/ml | 1.0E-9 | 1.0E-9 | 1.2E0 | 1.0E-9 | 9.1E0 | 0.0E0 | 1.0E-9 | 1.0E-9 | 7.1E1 | 1.0E-9 | 62 | 25 | 62 | 25 | 0.48 |
| Mj | pg/ml | 1.0E-9 | 1.0E-9 | 2.4E0 | 7.7E0 | 7.5E0 | 2.4E1 | 1.0E-9 | 1.0E-9 | 3.8E1 | 1.1E2 | 62 | 25 | 62 | 25 | 0.54 |
| Mk | pg/ml | 2.7E0 | 4.3E0 | 3.1E1 | 6.3E0 | 1.7E2 | 8.3E0 | 1.0E-9 | 1.0E-9 | 1.3E3 | 3.6E1 | 62 | 25 | 62 | 25 | 0.50 |
| Ml | pg/ml | 1.0E-9 | 1.0E-9 | 3.1E0 | 2.2E0 | 9.9E0 | 5.0E0 | 1.0E-9 | 1.0E-9 | 6.4E1 | 1.9E1 | 62 | 25 | 62 | 25 | 0.49 |
| Mm | pg/ml | 7.4E2 | 8.7E2 | 1.1E3 | 1.2E3 | 1.2E3 | 1.3E3 | 1.3E1 | 1.9E1 | 6.3E3 | 6.1E3 | 62 | 25 | 62 | 25 | 0.51 |
| Mn | pg/ml | 6.7E0 | 7.1E0 | 1.0E1 | 1.4E1 | 1.3E1 | 2.5E1 | 1.0E-9 | 1.0E-9 | 6.8E1 | 1.3E2 | 62 | 25 | 62 | 25 | 0.55 |
| Mp | pg/ml | 1.0E-9 | 1.0E-9 | 1.4E1 | 9.3E0 | 7.9E1 | 2.0E1 | 1.0E-9 | 1.0E-9 | 6.3E2 | 9.2E1 | 62 | 25 | 62 | 25 | 0.55 |
| Mq | pg/ml | 1.0E-9 | 1.0E-9 | 3.9E0 | 6.1E0 | 1.7E1 | 1.8E1 | 1.0E-9 | 1.0E-9 | 1.2E2 | 8.6E1 | 62 | 25 | 62 | 25 | 0.53 |
| Mr | pg/ml | 2.3E-1 | 1.0E-9 | 1.8E1 | 6.4E1 | 5.9E1 | 3.0E2 | 1.0E-9 | 1.0E-9 | 3.9E2 | 1.5E3 | 62 | 25 | 62 | 25 | 0.45 |
| Ms | pg/ml | 3.9E2 | 3.2E2 | 5.5E2 | 3.7E2 | 5.2E2 | 3.9E2 | 1.0E-9 | 1.0E-9 | 2.2E3 | 1.5E3 | 62 | 25 | 62 | 25 | 0.41 |
| Mt | pg/ml | 9.0E-1 | 1.6E0 | 6.9E0 | 1.0E1 | 2.2E1 | 2.8E1 | 1.0E-9 | 1.0E-9 | 1.4E2 | 1.0E2 | 62 | 25 | 62 | 25 | 0.56 |
| Mu | pg/ml | 1.0E-9 | 1.0E-9 | 5.9E-1 | 4.9E-1 | 1.8E0 | 1.6E0 | 1.0E-9 | 1.0E-9 | 1.0E1 | 7.3E0 | 62 | 25 | 62 | 25 | 0.47 |
| Mv | pg/ml | 1.0E-9 | 1.0E-9 | 3.9E1 | 1.1E2 | 1.3E2 | 5.1E2 | 1.0E-9 | 1.0E-9 | 8.4E2 | 2.5E3 | 62 | 25 | 62 | 25 | 0.47 |
| Mw | pg/ml | 3.1E1 | 7.2E1 | 1.7E2 | 3.2E2 | 4.2E2 | 1.2E3 | 1.0E-9 | 1.0E-9 | 2.2E3 | 5.9E3 | 62 | 25 | 62 | 25 | 0.60 |
| Mx | pg/ml | 1.0E-9 | 1.0E-9 | 1.8E-1 | 2.2E-1 | 5.3E-1 | 5.2E-1 | 1.0E-9 | 1.0E-9 | 2.7E0 | 2.1E0 | 62 | 25 | 62 | 25 | 0.54 |
| My | pg/ml | 1.0E-9 | 1.0E-9 | 1.2E2 | 2.7E2 | 5.1E2 | 9.6E2 | 1.0E-9 | 1.0E-9 | 3.6E3 | 4.6E3 | 62 | 25 | 62 | 25 | 0.53 |
| Mz | pg/ml | 1.5E1 | 1.7E1 | 2.6E1 | 3.2E1 | 4.4E1 | 3.4E1 | 1.0E-9 | 1.0E-9 | 3.0E2 | 1.5E2 | 62 | 25 | 62 | 25 | 0.59 |
| Na | pg/ml | 1.0E-9 | 1.0E-9 | 3.4E-1 | 1.1E0 | 1.2E0 | 3.5E0 | 1.0E-9 | 1.0E-9 | 7.2E0 | 1.6E1 | 62 | 25 | 62 | 25 | 0.50 |
| Nb | pg/ml | 2.0E0 | 2.5E0 | 2.6E0 | 3.7E0 | 3.2E0 | 3.8E0 | 1.0E-9 | 1.0E-9 | 2.3E1 | 1.7E1 | 62 | 25 | 62 | 25 | 0.60 |
| Nc | pg/ml | 1.8E2 | 2.9E2 | 3.6E2 | 4.9E2 | 4.0E2 | 5.3E2 | 1.0E-9 | 1.0E-9 | 1.4E3 | 2.1E3 | 62 | 25 | 62 | 25 | 0.57 |
| Nd | pg/ml | 5.7E0 | 6.5E0 | 3.3E1 | 1.8E1 | 1.6E2 | 2.2E1 | 1.0E-9 | 1.0E-9 | 1.2E3 | 9.4E1 | 62 | 25 | 62 | 25 | 0.56 |
| Ne | pg/ml | 3.2E2 | 4.0E2 | 3.7E2 | 4.9E2 | 3.3E2 | 4.4E2 | 1.0E-9 | 1.0E-9 | 1.5E3 | 1.7E3 | 62 | 25 | 62 | 25 | 0.56 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nf | pg/ml | 1.0E-9 | 1.0E-9 | 1.5E0 | 3.0E0 | 5.5E0 | 1.0E1 | 1.0E-9 | 1.0E-9 | 3.5E1 | 5.1E1 | 62 | 25 | 62 | 25 | 0.54 |
| Ng | pg/ml | 2.7E1 | 6.2E0 | 1.1E2 | 9.5E1 | 1.5E2 | 2.6E2 | 1.0E-9 | 1.0E-9 | 6.2E2 | 1.2E3 | 62 | 25 | 62 | 25 | 0.38 |
| Nh | pg/ml | 4.2E1 | 5.5E1 | 5.3E1 | 6.0E1 | 4.2E1 | 5.1E1 | 1.0E-9 | 1.0E-9 | 1.6E2 | 2.2E2 | 62 | 25 | 62 | 25 | 0.54 |
| Ni | pg/ml | 7.1E0 | 1.0E-9 | 9.7E1 | 8.8E1 | 1.4E2 | 1.4E2 | 1.0E-9 | 1.0E-9 | 5.7E2 | 5.7E2 | 62 | 25 | 62 | 25 | 0.47 |
| Nj | pg/ml | 3.2E0 | 2.9E0 | 6.7E0 | 6.2E0 | 7.9E0 | 7.4E0 | 1.0E-9 | 1.0E-9 | 3.6E1 | 2.9E1 | 62 | 25 | 62 | 25 | 0.50 |
| Nk | pg/ml | 1.6E1 | 2.7E1 | 3.1E1 | 4.2E1 | 3.5E1 | 5.4E1 | 1.0E-9 | 1.0E-9 | 1.2E2 | 2.0E2 | 62 | 25 | 62 | 25 | 0.55 |
| Nl | pg/ml | 2.3E1 | 4.2E1 | 3.8E1 | 5.0E1 | 3.9E1 | 4.2E1 | 1.0E-9 | 1.0E-9 | 1.9E2 | 1.4E2 | 62 | 25 | 62 | 25 | 0.60 |
| Tz | pg/ml | 7.0E3 | 6.0E3 | 3.6E4 | 1.0E4 | 1.5E5 | 1.6E4 | 1.0E-9 | 1.9E3 | 1.0E6 | 7.0E4 | 43 | 16 | 43 | 16 | 0.51 |
| Ua | pg/ml | 3.6E3 | 4.4E3 | 1.5E4 | 5.8E3 | 3.4E4 | 4.9E3 | 1.0E-9 | 1.4E3 | 1.9E5 | 2.1E4 | 43 | 16 | 43 | 16 | 0.51 |
| Ub | pg/ml | 6.9E2 | 5.1E2 | 1.0E3 | 9.9E2 | 1.1E3 | 1.1E3 | 1.0E-9 | 6.6E1 | 6.4E3 | 4.1E3 | 43 | 16 | 43 | 16 | 0.46 |
| Ue | pg/ml | 3.7E1 | 2.9E1 | 4.8E1 | 3.2E1 | 5.3E1 | 1.9E1 | 9.8E-2 | 8.8E0 | 3.5E2 | 7.4E1 | 43 | 16 | 43 | 16 | 0.39 |
| Uc | pg/ml | 1.1E3 | 9.4E2 | 1.8E3 | 1.9E3 | 2.1E3 | 2.3E3 | 1.0E-9 | 1.1E2 | 9.2E3 | 8.3E3 | 43 | 16 | 43 | 16 | 0.50 |
| Ud | pg/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 43 | 16 | 43 | 16 | 0.50 |
| Hq | pg/ml | 9.6E-1 | 1.2E0 | 4.7E2 | 1.2E1 | 3.6E3 | 4.7E1 | 1.0E-9 | 1.0E-9 | 2.8E4 | 2.3E2 | 61 | 24 | 61 | 24 | 0.53 |
| Hr | pg/ml | 1.0E2 | 1.3E2 | 4.5E2 | 9.7E2 | 9.1E2 | 1.7E3 | 1.0E-9 | 3.9E1 | 5.0E3 | 5.5E3 | 61 | 24 | 61 | 24 | 0.58 |
| Hu | pg/ml | 3.5E1 | 3.1E0 | 8.3E2 | 6.2E2 | 3.9E3 | 1.8E3 | 1.0E-9 | 1.0E-9 | 2.9E4 | 7.9E3 | 61 | 24 | 61 | 24 | 0.36 |
| Hv | pg/ml | 1.7E0 | 1.1E0 | 2.6E0 | 2.0E0 | 2.9E0 | 2.4E0 | 1.0E-9 | 1.0E-9 | 1.8E1 | 1.1E1 | 61 | 24 | 61 | 24 | 0.43 |
| Hw | pg/ml | 5.8E0 | 6.1E0 | 1.3E1 | 1.2E1 | 2.5E1 | 2.1E1 | 1.0E-9 | 1.0E-9 | 1.9E2 | 1.0E2 | 61 | 24 | 61 | 24 | 0.50 |
| Hx | pg/ml | 1.1E1 | 1.6E1 | 2.0E1 | 3.0E1 | 4.9E1 | 3.7E1 | 1.0E-9 | 1.0E-9 | 3.7E2 | 1.6E2 | 61 | 24 | 61 | 24 | 0.62 |
| Ib | ng/ml | 6.0E-2 | 2.5E-2 | 1.8E0 | 7.3E-2 | 6.6E0 | 1.7E-1 | 1.0E-9 | 1.0E-9 | 3.9E1 | 6.8E-1 | 41 | 16 | 41 | 16 | 0.33 |
| Ic | U/ml | 2.2E2 | 2.2E2 | 3.6E2 | 2.1E2 | 6.9E2 | 1.4E2 | 1.2E1 | 1.8E1 | 4.2E3 | 5.4E2 | 41 | 16 | 41 | 16 | 0.48 |
| Id | U/ml | 8.4E-1 | 7.9E-1 | 1.4E0 | 2.1E0 | 1.4E0 | 3.8E0 | 1.0E-9 | 2.4E-1 | 5.0E0 | 1.5E1 | 41 | 16 | 41 | 16 | 0.53 |
| Tt | pg/ml | 1.7E2 | 1.7E2 | 1.7E2 | 1.8E2 | 5.6E1 | 5.5E1 | 7.3E1 | 1.0E2 | 3.6E2 | 2.8E2 | 43 | 16 | 43 | 16 | 0.55 |
| To | pg/ml | 1.6E0 | 6.6E-1 | 1.9E0 | 1.3E0 | 2.0E0 | 1.6E0 | 1.0E-9 | 1.0E-9 | 7.1E0 | 5.1E0 | 43 | 16 | 43 | 16 | 0.42 |
| Tr | pg/ml | 2.7E0 | 5.1E0 | 1.2E1 | 8.5E0 | 4.7E1 | 9.9E0 | 2.4E-1 | 9.2E-1 | 3.1E2 | 3.8E1 | 43 | 16 | 43 | 16 | 0.62 |
| Tn | pg/ml | 2.4E1 | 3.9E1 | 8.6E1 | 5.0E1 | 2.8E2 | 4.1E1 | 2.4E0 | 1.6E1 | 1.8E3 | 1.9E2 | 43 | 16 | 43 | 16 | 0.64 |
| Tv | ng/ml | 1.1E1 | 1.2E1 | 1.7E1 | 6.0E1 | 2.3E1 | 1.9E2 | 1.0E-9 | 1.0E-9 | 1.3E2 | 7.9E2 | 43 | 16 | 43 | 16 | 0.49 |
| Ih | ng/ml | 9.8E1 | 1.4E2 | 2.1E2 | 2.5E2 | 3.2E2 | 2.5E2 | 1.0E-9 | 4.7E0 | 1.7E3 | 9.0E2 | 61 | 25 | 61 | 25 | 0.60 |
| Ii | ng/ml | 1.0E2 | 1.2E2 | 3.4E2 | 2.8E2 | 1.3E3 | 3.4E2 | 8.7E0 | 9.8E0 | 1.0E4 | 1.2E3 | 61 | 25 | 61 | 25 | 0.58 |
| Ij | ng/ml | 8.5E1 | 1.0E2 | 1.5E2 | 1.3E2 | 2.4E2 | 7.8E1 | 2.1E1 | 1.9E1 | 1.8E3 | 3.2E2 | 61 | 24 | 61 | 24 | 0.60 |
| Ik | ng/ml | 2.2E2 | 1.2E2 | 7.3E2 | 4.0E2 | 1.4E3 | 5.3E2 | 1.6E0 | 2.3E0 | 9.7E3 | 1.5E3 | 61 | 25 | 61 | 25 | 0.45 |
| Il | ng/ml | 5.1E2 | 4.7E2 | 1.1E3 | 1.7E3 | 2.2E3 | 3.5E3 | 1.0E-9 | 1.0E-9 | 1.2E4 | 1.2E4 | 61 | 25 | 61 | 25 | 0.46 |
| Im | ng/ml | 2.2E2 | 2.5E2 | 5.3E2 | 6.2E2 | 9.6E2 | 1.1E3 | 5.2E1 | 8.7E1 | 5.6E3 | 5.8E3 | 61 | 25 | 61 | 25 | 0.59 |
| In | ng/ml | 5.6E0 | 3.3E0 | 1.1E1 | 9.1E0 | 1.4E1 | 1.7E1 | 1.0E-9 | 1.0E-9 | 7.0E1 | 8.4E1 | 61 | 25 | 61 | 25 | 0.43 |
| Hb | ng/ml | 2.7E1 | 5.9E1 | 3.4E1 | 6.5E1 | 2.4E1 | 6.2E1 | 4.2E0 | 2.6E0 | 9.2E1 | 2.1E2 | 43 | 17 | 43 | 17 | 0.62 |
| Hc | pg/ml | 8.7E2 | 5.9E2 | 3.7E3 | 7.0E2 | 1.5E4 | 8.9E2 | 1.0E-9 | 1.0E-9 | 1.0E5 | 3.9E3 | 43 | 17 | 43 | 17 | 0.36 |
| Hf | ng/ml | 2.6E2 | 1.9E2 | 5.8E2 | 3.3E2 | 7.5E2 | 3.4E2 | 1.0E-9 | 1.0E-9 | 3.2E3 | 9.9E2 | 43 | 17 | 43 | 17 | 0.42 |
| Io | ng/ml | 8.0E3 | 9.1E3 | 1.4E4 | 1.4E4 | 1.8E4 | 1.3E4 | 1.0E-9 | 1.3E3 | 1.2E5 | 5.4E4 | 61 | 25 | 61 | 25 | 0.51 |
| Ip | ng/ml | 1.3E1 | 3.0E1 | 1.9E1 | 3.1E1 | 2.0E1 | 2.2E1 | 1.0E-9 | 4.2E-1 | 6.4E1 | 8.8E1 | 61 | 25 | 61 | 25 | 0.66 |
| Iq | ug/ml | 3.0E-2 | 1.9E-1 | 8.7E-1 | 1.1E0 | 3.3E0 | 3.6E0 | 1.0E-9 | 1.0E-9 | 1.8E1 | 1.8E1 | 61 | 25 | 61 | 25 | 0.66 |
| Ir | ug/ml | 3.1E-1 | 7.7E-1 | 3.6E0 | 1.9E0 | 2.0E1 | 2.6E0 | 1.0E-9 | 5.0E-2 | 1.6E2 | 1.1E1 | 61 | 25 | 61 | 25 | 0.67 |
| Is | ng/ml | 1.9E0 | 3.5E0 | 6.0E0 | 7.0E0 | 1.2E1 | 8.1E0 | 1.0E-9 | 1.0E0 | 6.2E1 | 3.0E1 | 61 | 25 | 61 | 25 | 0.68 |
| It | ng/ml | 1.9E0 | 3.0E0 | 1.9E1 | 7.3E0 | 1.0E2 | 1.3E1 | 1.0E-9 | 1.0E-9 | 7.7E2 | 5.4E1 | 61 | 25 | 61 | 25 | 0.59 |
| Iu | ng/ml | 1.1E2 | 2.2E2 | 1.1E3 | 1.6E3 | 3.7E3 | 4.9E3 | 1.0E-9 | 1.0E-9 | 2.4E4 | 2.4E4 | 61 | 25 | 61 | 25 | 0.58 |
| Iv | ng/ml | 2.0E1 | 3.2E1 | 4.3E1 | 3.4E1 | 7.4E1 | 2.9E1 | 1.0E-9 | 1.0E-9 | 4.9E2 | 1.0E2 | 61 | 25 | 61 | 25 | 0.55 |
| Iz | ng/ml | 1.6E2 | 1.2E2 | 4.2E2 | 2.5E2 | 6.7E2 | 2.8E2 | 1.8E1 | 8.8E0 | 3.6E3 | 9.2E2 | 43 | 17 | 43 | 17 | 0.46 |
| Rc | pg/ml | 7.1E3 | 5.6E3 | 8.7E3 | 5.9E3 | 6.7E3 | 3.1E3 | 1.1E3 | 6.7E2 | 2.3E4 | 1.0E4 | 43 | 16 | 43 | 16 | 0.42 |
| Rb | pg/ml | 1.2E0 | 1.1E0 | 3.5E0 | 1.8E0 | 4.8E0 | 2.5E0 | 1.0E-9 | 1.0E-9 | 2.0E1 | 9.1E0 | 43 | 16 | 43 | 16 | 0.45 |
| Pz | ng/ml | 4.2E3 | 5.8E3 | 6.7E3 | 4.8E4 | 7.7E3 | 2.1E5 | 1.9E2 | 6.2E2 | 4.8E4 | 1.0E6 | 61 | 24 | 61 | 24 | 0.52 |
| Qa | ng/ml | 3.9E3 | 7.3E3 | 6.2E3 | 8.9E3 | 6.0E3 | 6.5E3 | 4.2E2 | 6.8E2 | 2.9E4 | 2.3E4 | 61 | 24 | 61 | 24 | 0.64 |
| Qb | ng/ml | 1.2E2 | 2.0E2 | 1.6E2 | 2.8E2 | 1.7E2 | 2.4E2 | 6.7E0 | 1.8E1 | 7.3E2 | 8.7E2 | 61 | 24 | 61 | 24 | 0.63 |
| Qc | ng/ml | 3.3E2 | 3.7E2 | 4.3E2 | 6.3E2 | 4.4E2 | 8.9E2 | 1.9E0 | 1.3E1 | 2.1E3 | 4.3E3 | 61 | 24 | 61 | 24 | 0.55 |
| Qd | ng/ml | 1.2E4 | 1.5E4 | 2.0E4 | 2.2E4 | 2.5E4 | 2.2E4 | 2.0E3 | 1.7E3 | 1.3E5 | 9.1E4 | 61 | 24 | 61 | 24 | 0.52 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|-----|---------|-----|------|
| | | NonDis | Dis | NonDis | Dis | NonDis | Dis | NonDis | Dis | NonDis | Dis | NonDis | Dis | NonDis | Dis | |
| Qe | ng/ml | 1.5E3 | 1.8E3 | 1.8E3 | 2.5E3 | 1.5E3 | 2.6E3 | 1.4E2 | 1.8E2 | 7.4E3 | 1.3E4 | 61 | 24 | 61 | 24 | 0.60 |
| Jd | ng/ml | 2.0E0 | 1.5E0 | 4.2E0 | 1.6E0 | 1.1E1 | 1.4E0 | 1.0E-9 | 1.0E-9 | 7.3E1 | 4.3E0 | 43 | 16 | 43 | 16 | 0.40 |
| Je | ng/ml | 2.9E-1 | 5.7E-1 | 1.6E0 | 1.2E0 | 2.2E0 | 1.5E0 | 1.0E-9 | 1.0E-9 | 7.0E0 | 4.3E0 | 43 | 16 | 43 | 16 | 0.49 |
| Jf | ng/ml | 1.0E-9 | 7.3E-1 | 1.6E0 | 1.5E0 | 2.8E0 | 2.1E0 | 1.0E-9 | 1.0E-9 | 9.6E0 | 7.7E0 | 43 | 16 | 43 | 16 | 0.53 |
| Jg | ng/ml | 7.6E2 | 7.7E2 | 9.9E2 | 1.1E3 | 7.9E2 | 1.2E3 | 1.3E1 | 5.9E1 | 3.4E3 | 5.3E3 | 61 | 24 | 61 | 24 | 0.49 |
| Jh | ng/ml | 5.4E0 | 2.6E0 | 1.8E1 | 2.7E1 | 4.6E1 | 9.6E1 | 1.0E-9 | 1.0E-9 | 2.6E2 | 4.7E2 | 61 | 24 | 61 | 24 | 0.45 |
| Ji | ng/ml | 7.0E1 | 8.5E1 | 1.1E2 | 1.3E2 | 8.6E1 | 9.6E1 | 1.3E1 | 2.2E1 | 3.6E2 | 4.1E2 | 61 | 24 | 61 | 24 | 0.60 |
| Sr | pg/mL | 6.0E2 | 8.7E2 | 8.7E2 | 1.2E3 | 9.4E2 | 1.3E3 | 1.0E-9 | 1.0E-9 | 4.8E3 | 5.1E3 | 40 | 16 | 40 | 16 | 0.58 |
| Ss | pg/mL | 1.6E5 | 4.8E4 | 1.9E5 | 9.7E4 | 2.4E5 | 9.1E4 | 2.7E3 | 1.1E4 | 1.3E6 | 3.0E5 | 40 | 16 | 40 | 16 | 0.39 |
| St | pg/mL | 2.7E7 | 5.3E7 | 7.5E7 | 8.5E7 | 1.8E8 | 1.3E8 | 1.1E6 | 3.4E6 | 1.2E9 | 5.4E8 | 43 | 16 | 43 | 16 | 0.63 |
| Ra | pg/ml | 1.0E-9 | 1.0E-9 | 4.2E-1 | 3.6E-1 | 9.8E-1 | 7.5E-1 | 1.0E-9 | 1.0E-9 | 3.4E0 | 3.0E0 | 43 | 16 | 43 | 16 | 0.57 |
| Qz | pg/ml | 8.5E0 | 1.0E1 | 3.9E1 | 5.4E1 | 6.1E1 | 8.4E1 | 1.0E-9 | 1.0E-9 | 2.8E2 | 2.3E2 | 43 | 16 | 43 | 16 | 0.53 |
| Qy | pg/ml | 4.4E-1 | 5.2E-1 | 4.2E0 | 5.0E-1 | 1.4E1 | 4.5E-1 | 1.0E-9 | 1.0E-9 | 8.6E1 | 1.3E0 | 43 | 16 | 43 | 16 | 0.44 |
| Qx | pg/ml | 1.0E-9 | 1.0E-9 | 3.8E-1 | 1.0E-9 | 1.8E0 | 0.0E0 | 1.0E-9 | 1.0E-9 | 9.4E0 | 1.0E-9 | 43 | 16 | 43 | 16 | 0.48 |
| Qw | pg/ml | 9.0E-2 | 1.0E-9 | 2.9E0 | 1.3E-1 | 1.1E1 | 3.3E-1 | 1.0E-9 | 1.0E-9 | 6.6E1 | 1.2E0 | 43 | 16 | 43 | 16 | 0.33 |
| Qv | pg/ml | 2.6E4 | 1.8E4 | 4.3E4 | 2.4E4 | 6.0E4 | 2.5E4 | 1.0E-9 | 8.5E2 | 3.3E5 | 8.4E4 | 43 | 16 | 43 | 16 | 0.39 |
| Qu | pg/ml | 7.8E0 | 5.8E0 | 1.1E2 | 4.6E1 | 2.2E2 | 7.8E1 | 1.0E-9 | 1.0E-9 | 9.8E2 | 2.9E2 | 43 | 16 | 43 | 16 | 0.46 |
| Qt | pg/ml | 1.1E1 | 3.3E0 | 7.0E1 | 1.2E1 | 1.5E2 | 1.5E1 | 1.0E-9 | 1.0E-9 | 7.0E2 | 4.8E1 | 43 | 16 | 43 | 16 | 0.41 |
| Qh | ng/ml | 1.6E1 | 3.9E1 | 4.4E1 | 7.0E1 | 7.5E1 | 1.1E2 | 4.3E-1 | 4.9E0 | 3.3E2 | 4.6E2 | 43 | 16 | 43 | 16 | 0.68 |
| Qg | ng/ml | 7.5E0 | 8.2E0 | 1.6E1 | 9.7E0 | 3.3E1 | 8.1E0 | 1.3E-1 | 1.3E0 | 2.2E2 | 3.5E1 | 43 | 16 | 43 | 16 | 0.52 |
| Jj | ng/ml | 7.3E2 | 4.2E2 | 1.0E3 | 6.2E2 | 1.0E3 | 8.1E2 | 6.1E1 | 7.9E1 | 5.7E3 | 4.1E3 | 61 | 24 | 61 | 24 | 0.37 |
| Jk | ng/ml | 3.0E0 | 2.4E0 | 2.1E1 | 2.0E1 | 4.7E1 | 4.4E1 | 1.2E-1 | 3.8E-1 | 2.7E2 | 2.0E2 | 61 | 24 | 61 | 24 | 0.52 |
| Jl | ng/ml | 5.3E-1 | 5.9E-1 | 2.0E0 | 3.8E0 | 4.4E0 | 8.7E0 | 1.6E-2 | 7.8E-2 | 2.0E1 | 4.0E1 | 61 | 24 | 61 | 24 | 0.55 |
| Jm | ng/ml | 2.3E1 | 4.0E1 | 5.1E1 | 1.5E2 | 8.9E1 | 4.1E2 | 1.9E-1 | 9.2E-1 | 6.1E2 | 2.1E3 | 61 | 24 | 61 | 24 | 0.61 |
| Jn | pg/ml | 4.6E-1 | 7.8E-1 | 1.0E0 | 1.1E0 | 2.0E0 | 9.6E-1 | 1.0E-9 | 3.6E-2 | 1.4E1 | 3.9E0 | 61 | 24 | 61 | 24 | 0.61 |
| Jo | pg/ml | 4.9E3 | 4.8E3 | 5.8E3 | 5.3E3 | 4.3E3 | 4.0E3 | 4.8E2 | 4.8E2 | 1.9E4 | 1.6E4 | 61 | 24 | 61 | 24 | 0.47 |
| Jp | pg/ml | 8.2E4 | 8.2E4 | 8.2E4 | 9.0E4 | 3.1E4 | 4.1E4 | 1.4E4 | 2.6E4 | 1.7E5 | 2.1E5 | 61 | 24 | 61 | 24 | 0.52 |
| Jq | pg/ml | 1.1E2 | 1.4E2 | 2.1E2 | 2.0E2 | 5.2E2 | 2.3E2 | 1.1E1 | 3.0E1 | 4.0E3 | 1.1E3 | 61 | 24 | 61 | 24 | 0.58 |
| Jr | pg/ml | 6.7E0 | 8.0E0 | 1.4E1 | 1.2E1 | 2.1E1 | 1.3E1 | 1.0E-9 | 1.0E-9 | 1.1E2 | 4.9E1 | 61 | 24 | 61 | 24 | 0.55 |
| Js | pg/ml | 1.5E1 | 1.8E1 | 2.3E1 | 2.4E1 | 3.6E1 | 2.0E1 | 1.9E0 | 6.6E0 | 2.7E2 | 8.8E1 | 61 | 24 | 61 | 24 | 0.57 |
| Jt | pg/ml | 3.3E3 | 2.8E3 | 3.4E3 | 3.2E3 | 2.0E3 | 2.1E3 | 3.8E2 | 3.8E2 | 9.7E3 | 7.5E3 | 61 | 24 | 61 | 24 | 0.47 |
| Ju | mIU/ml | 9.3E0 | 1.1E1 | 2.4E1 | 1.7E1 | 3.7E1 | 1.7E1 | 1.7E-1 | 7.5E-1 | 2.0E2 | 6.0E1 | 43 | 16 | 43 | 16 | 0.53 |
| Jv | mIU/ml | 1.9E1 | 1.7E1 | 3.3E1 | 2.6E1 | 5.7E1 | 2.4E1 | 1.7E-2 | 8.2E-2 | 3.4E2 | 6.3E1 | 43 | 16 | 43 | 16 | 0.53 |
| Jy | ng/ml | 1.6E-3 | 1.6E-3 | 2.5E-3 | 1.6E-3 | 5.8E-3 | 5.4E-4 | 1.7E-4 | 5.3E-4 | 3.9E-2 | 2.6E-3 | 43 | 16 | 43 | 16 | 0.50 |
| Kc | pg/ml | 2.8E1 | 2.9E1 | 4.4E1 | 5.0E1 | 4.0E1 | 7.3E1 | 9.5E-1 | 1.0E-9 | 1.7E2 | 2.7E2 | 43 | 16 | 43 | 16 | 0.47 |
| Kd | pg/ml | 1.0E-9 | 1.0E-9 | 2.4E2 | 4.3E2 | 5.0E2 | 8.1E2 | 1.0E-9 | 1.0E-9 | 1.9E3 | 2.4E3 | 43 | 16 | 43 | 16 | 0.53 |
| Kc | pg/ml | 1.4E4 | 1.9E4 | 1.7E4 | 2.3E4 | 1.2E4 | 1.5E4 | 2.9E3 | 5.2E3 | 5.6E4 | 5.2E4 | 43 | 16 | 43 | 16 | 0.63 |
| Kf | pg/mL | 8.1E0 | 7.8E0 | 7.4E0 | 9.7E0 | 5.0E0 | 9.8E0 | 1.0E-9 | 1.0E-9 | 2.4E1 | 4.4E1 | 43 | 16 | 43 | 16 | 0.54 |
| Kg | pg/mL | 1.5E3 | 1.2E3 | 2.1E3 | 2.1E3 | 2.2E3 | 2.2E3 | 1.4E2 | 3.5E2 | 1.1E4 | 7.7E3 | 43 | 16 | 43 | 16 | 0.47 |
| Ki | pg/ml | 5.8E1 | 7.8E1 | 7.9E1 | 8.8E1 | 5.9E1 | 6.6E1 | 1.0E-9 | 1.3E1 | 2.8E2 | 2.5E2 | 42 | 16 | 42 | 16 | 0.55 |
| Kj | pg/ml | 1.3E3 | 7.7E2 | 1.7E3 | 1.4E3 | 1.3E3 | 1.5E3 | 7.8E1 | 1.5E2 | 5.6E3 | 6.1E3 | 43 | 16 | 43 | 16 | 0.40 |
| Kk | pg/ml | 5.9E0 | 1.2E1 | 1.0E1 | 1.8E1 | 1.1E1 | 1.8E1 | 1.0E-9 | 2.3E0 | 5.0E1 | 5.5E1 | 43 | 16 | 43 | 16 | 0.65 |
| Kl | pg/ml | 2.3E4 | 1.7E4 | 3.3E4 | 2.6E4 | 3.1E4 | 2.4E4 | 2.3E2 | 7.0E2 | 1.6E5 | 9.0E4 | 43 | 16 | 43 | 16 | 0.46 |
| Kn | pg/ml | 5.3E1 | 4.1E1 | 7.0E1 | 1.1E2 | 8.4E1 | 1.8E2 | 1.0E-9 | 1.0E-9 | 3.8E2 | 6.3E2 | 43 | 16 | 43 | 16 | 0.51 |
| Ko | pg/ml | 3.4E2 | 5.9E2 | 5.1E2 | 6.8E2 | 4.9E2 | 5.1E2 | 1.0E-9 | 1.0E-9 | 1.6E3 | 1.6E3 | 43 | 16 | 43 | 16 | 0.61 |
| Kp | pg/ml | 3.6E2 | 3.7E2 | 3.7E2 | 4.3E2 | 2.1E2 | 2.9E2 | 1.0E-9 | 1.0E-9 | 8.1E2 | 9.8E2 | 43 | 16 | 43 | 16 | 0.55 |
| Kq | pg/ml | 3.9E2 | 4.3E2 | 5.6E2 | 4.7E2 | 5.0E2 | 2.8E2 | 6.1E1 | 1.1E2 | 2.1E3 | 1.3E3 | 41 | 17 | 41 | 17 | 0.52 |
| Kr | pg/ml | 2.0E0 | 9.9E-1 | 2.7E0 | 3.0E0 | 3.0E0 | 5.1E0 | 1.0E-9 | 1.0E-9 | 1.2E1 | 1.9E1 | 41 | 17 | 41 | 17 | 0.44 |
| Ks | pg/ml | 1.5E4 | 1.1E4 | 2.2E4 | 2.1E4 | 1.8E4 | 1.8E4 | 2.7E2 | 9.9E2 | 5.0E4 | 5.1E4 | 41 | 17 | 41 | 17 | 0.50 |
| Kx | ng/ml | 2.8E-3 | 1.0E-9 | 9.5E-3 | 1.2E-2 | 1.6E-2 | 1.8E-2 | 1.0E-9 | 1.0E-9 | 7.9E-2 | 6.5E-2 | 42 | 17 | 42 | 17 | 0.50 |
| Ky | ng/ml | 7.6E-2 | 1.3E-1 | 4.0E-1 | 3.1E-1 | 8.0E-1 | 5.7E-1 | 1.0E-9 | 1.0E-9 | 4.4E0 | 2.4E0 | 42 | 17 | 42 | 17 | 0.54 |
| Kz | ng/ml | 1.0E-9 | 1.0E-9 | 2.0E-3 | 2.9E-3 | 4.7E-3 | 4.8E-3 | 1.0E-9 | 1.0E-9 | 1.4E-2 | 1.4E-2 | 42 | 17 | 42 | 17 | 0.57 |
| Ld | pg/ml | 1.0E-9 | 1.0E-9 | 2.9E0 | 5.0E0 | 5.3E0 | 1.2E1 | 1.0E-9 | 1.0E-9 | 2.5E1 | 5.0E1 | 43 | 17 | 43 | 17 | 0.52 |

| Code | Units | Median | | Average | | Standard Deviation | | Minimum | | Maximum | | Number of Samples | | Number of Patients | | AUC |
|------|-------|--------|------|---------|------|------|------|---------|------|---------|------|--------|------|--------|------|-----|
| | | NonDis | Dis | NonDis | Dis | NonDis | Dis | NonDis | Dis | NonDis | Dis | NonDis | Dis | NonDis | Dis | |
| Lh | pg/ml | 1.8E4 | 2.0E4 | 2.5E4 | 3.7E4 | 2.4E4 | 4.4E4 | 1.0E3 | 3.8E3 | 1.1E5 | 2.1E5 | 62 | 24 | 62 | 24 | 0.59 |
| Li | pg/ml | 5.7E3 | 1.3E4 | 1.7E4 | 5.5E4 | 2.9E4 | 1.9E5 | 2.8E2 | 3.6E1 | 1.3E5 | 9.2E5 | 62 | 24 | 62 | 24 | 0.62 |
| Lj | pg/ml | 4.2E3 | 6.6E3 | 2.8E4 | 5.1E4 | 7.9E4 | 8.9E4 | 4.1E1 | 1.0E2 | 4.7E5 | 4.1E5 | 62 | 24 | 62 | 24 | 0.58 |
| Rm | ng/ml | 1.9E1 | 2.7E1 | 4.5E1 | 8.6E1 | 6.2E1 | 1.6E2 | 1.3E0 | 4.6E0 | 2.5E2 | 6.5E2 | 42 | 15 | 42 | 15 | 0.60 |
| Rh | ng/ml | 1.8E2 | 1.2E2 | 2.9E2 | 2.4E2 | 3.6E2 | 2.3E2 | 2.6E1 | 3.6E1 | 2.0E3 | 7.4E2 | 42 | 15 | 42 | 15 | 0.50 |
| Ri | ng/ml | 1.0E-9 | 1.0E-9 | 3.6E0 | 2.7E0 | 8.2E0 | 4.8E0 | 1.0E-9 | 1.0E-9 | 4.5E1 | 1.6E1 | 42 | 15 | 42 | 15 | 0.49 |
| Rg | ng/ml | 1.0E-9 | 1.0E-9 | 1.4E-1 | 5.2E-2 | 5.9E-1 | 1.6E-1 | 1.0E-9 | 1.0E-9 | 3.3E0 | 6.2E-1 | 42 | 15 | 42 | 15 | 0.58 |
| Rj | ng/ml | 1.0E-9 | 1.0E-9 | 1.6E0 | 6.3E-1 | 4.1E0 | 1.1E0 | 1.0E-9 | 1.0E-9 | 2.4E1 | 3.2E0 | 42 | 15 | 42 | 15 | 0.45 |
| Rf | ng/ml | 4.8E-1 | 5.6E-1 | 1.4E0 | 1.2E0 | 2.6E0 | 1.2E0 | 3.2E-2 | 1.8E-2 | 1.2E1 | 3.8E0 | 42 | 15 | 42 | 15 | 0.57 |
| Ql | pg/ml | 4.5E0 | 7.2E0 | 1.8E1 | 1.3E1 | 3.7E1 | 2.1E1 | 1.0E-9 | 1.0E-9 | 1.8E2 | 8.8E1 | 43 | 16 | 43 | 16 | 0.52 |
| Qm | pg/ml | 8.6E0 | 3.2E0 | 2.2E1 | 2.8E1 | 3.8E1 | 4.7E1 | 1.0E-9 | 1.0E-9 | 1.8E2 | 1.7E2 | 43 | 16 | 43 | 16 | 0.51 |
| Qn | pg/ml | 9.5E-1 | 5.6E-1 | 4.3E0 | 1.8E1 | 8.4E0 | 6.0E1 | 1.0E-9 | 1.0E-9 | 3.8E1 | 2.4E2 | 43 | 16 | 43 | 16 | 0.39 |
| Nv | pg/ml | 4.7E3 | 5.1E3 | 9.9E3 | 1.4E4 | 1.6E4 | 2.6E4 | 4.6E2 | 7.1E2 | 8.4E4 | 1.1E5 | 62 | 25 | 62 | 25 | 0.50 |
| Nw | pg/ml | 1.0E4 | 1.6E4 | 1.5E4 | 1.8E4 | 1.2E4 | 1.3E4 | 2.6E3 | 5.8E3 | 7.3E4 | 6.1E4 | 62 | 25 | 62 | 25 | 0.60 |
| Nx | pg/ml | 2.4E2 | 2.6E2 | 5.5E2 | 6.9E2 | 7.0E2 | 7.7E2 | 1.0E-9 | 1.0E-9 | 2.6E3 | 2.5E3 | 62 | 25 | 62 | 25 | 0.55 |
| Ny | pg/ml | 9.6E0 | 7.9E0 | 1.6E1 | 3.5E1 | 2.6E1 | 8.6E1 | 1.0E-9 | 1.0E-9 | 1.5E2 | 4.2E2 | 62 | 25 | 62 | 25 | 0.54 |
| Oa | pg/ml | 1.2E2 | 6.5E2 | 3.7E2 | 1.0E3 | 5.4E2 | 1.2E3 | 1.0E-9 | 4.9E0 | 2.3E3 | 4.5E3 | 43 | 16 | 43 | 16 | 0.64 |
| Wn | ng/ml | 1.3E1 | 2.7E1 | 2.4E1 | 6.9E1 | 2.7E1 | 1.1E2 | 3.8E0 | 1.2E1 | 1.1E2 | 3.4E2 | 16 | 8 | 16 | 8 | 0.73 |
| Tk | ng/ml | 1.6E2 | 1.2E2 | 3.8E2 | 1.4E2 | 5.7E2 | 8.7E1 | 1.4E1 | 3.7E1 | 2.3E3 | 3.0E2 | 18 | 7 | 18 | 7 | 0.36 |
| Oe | pg/ml | 3.5E1 | 1.0E-9 | 2.7E2 | 2.5E2 | 5.1E2 | 4.3E2 | 1.0E-9 | 1.0E-9 | 3.4E3 | 1.6E3 | 62 | 25 | 62 | 25 | 0.48 |
| Of | pg/ml | 2.1E2 | 8.2E1 | 1.5E4 | 4.6E3 | 8.0E4 | 1.4E4 | 1.0E-9 | 1.0E-9 | 6.2E5 | 5.4E4 | 62 | 25 | 62 | 25 | 0.45 |
| Og | pg/ml | 9.8E-2 | 6.5E-2 | 1.7E0 | 2.7E-1 | 1.0E1 | 8.0E-1 | 1.0E-9 | 1.0E-9 | 7.9E1 | 4.0E0 | 62 | 25 | 62 | 25 | 0.37 |
| Oh | pg/ml | 2.6E0 | 4.2E0 | 8.4E0 | 6.9E0 | 2.5E1 | 1.0E1 | 1.0E-9 | 9.0E-2 | 2.0E2 | 5.4E1 | 62 | 25 | 62 | 25 | 0.60 |
| Oi | pg/ml | 4.1E0 | 3.0E0 | 7.2E0 | 6.0E0 | 8.9E0 | 8.4E0 | 1.0E-9 | 1.0E-9 | 4.1E1 | 3.6E1 | 62 | 25 | 62 | 25 | 0.46 |
| Ok | pg/ml | 5.4E2 | 6.4E2 | 6.1E2 | 9.0E2 | 4.0E2 | 7.8E2 | 6.4E1 | 1.4E2 | 2.0E3 | 3.2E3 | 62 | 25 | 62 | 25 | 0.61 |
| Om | pg/ml | 5.4E2 | 5.9E2 | 1.4E3 | 8.8E2 | 4.6E3 | 1.1E3 | 1.0E-9 | 9.7E1 | 3.6E4 | 5.0E3 | 62 | 25 | 62 | 25 | 0.48 |
| On | pg/ml | 2.0E2 | 2.3E2 | 2.8E2 | 3.9E2 | 2.6E2 | 4.0E2 | 2.2E1 | 3.9E1 | 1.6E3 | 1.5E3 | 62 | 25 | 62 | 25 | 0.58 |
| Or | pg/ml | 1.2E1 | 2.3E1 | 3.9E1 | 6.2E1 | 8.0E1 | 9.5E1 | 1.0E-9 | 1.0E-9 | 4.4E2 | 3.5E2 | 43 | 17 | 43 | 17 | 0.60 |
| Ow | pg/ml | 3.9E1 | 5.4E1 | 1.0E2 | 1.0E2 | 2.3E2 | 1.3E2 | 1.0E-9 | 1.0E-9 | 1.5E3 | 4.9E2 | 43 | 17 | 43 | 17 | 0.53 |
| Ou | pg/ml | 5.6E2 | 3.8E2 | 9.6E2 | 2.0E3 | 1.6E3 | 3.5E3 | 1.0E-9 | 1.0E-9 | 8.1E3 | 9.6E3 | 43 | 17 | 43 | 17 | 0.47 |
| Ul | ng/ml | 1.0E-9 | 1.0E-9 | 1.7E0 | 1.3E0 | 5.9E0 | 5.2E0 | 1.0E-9 | 1.0E-9 | 3.4E1 | 2.1E1 | 43 | 16 | 43 | 16 | 0.48 |
| Uo | ng/ml | 1.0E-9 | 1.0E-9 | 8.3E-2 | 2.4E-2 | 2.0E-1 | 8.2E-2 | 1.0E-9 | 1.0E-9 | 8.5E-1 | 3.2E-1 | 43 | 16 | 43 | 16 | 0.43 |
| Uk | ng/ml | 1.0E-9 | 1.0E-9 | 1.2E-2 | 4.5E-3 | 2.7E-2 | 1.8E-2 | 1.0E-9 | 1.0E-9 | 1.3E-1 | 7.1E-2 | 43 | 16 | 43 | 16 | 0.32 |
| Um | ng/ml | 1.0E-9 | 1.0E-9 | 1.6E-1 | 1.9E-1 | 3.3E-1 | 4.4E-1 | 1.0E-9 | 1.0E-9 | 1.3E0 | 1.4E0 | 43 | 16 | 43 | 16 | 0.45 |
| Uf | ng/ml | 7.9E-2 | 9.9E-2 | 1.6E-1 | 1.3E-1 | 2.3E-1 | 1.2E-1 | 6.8E-3 | 1.7E-2 | 1.1E0 | 5.3E-1 | 43 | 16 | 43 | 16 | 0.55 |
| Uh | ng/ml | 2.6E0 | 4.4E0 | 4.3E0 | 5.3E0 | 4.4E0 | 4.3E0 | 3.0E-1 | 4.5E-1 | 1.7E1 | 1.5E1 | 43 | 16 | 43 | 16 | 0.60 |
| Un | ng/ml | 2.5E0 | 2.3E0 | 2.7E0 | 2.4E0 | 1.5E0 | 1.3E0 | 4.6E-1 | 7.4E-1 | 8.0E0 | 5.1E0 | 43 | 16 | 43 | 16 | 0.46 |
| Ug | ng/ml | 2.2E1 | 9.7E0 | 3.2E1 | 2.1E1 | 3.4E1 | 2.8E1 | 1.2E0 | 2.3E0 | 1.8E2 | 1.1E2 | 43 | 16 | 43 | 16 | 0.36 |
| Ur | ng/ml | 9.6E-2 | 1.1E-1 | 2.8E-1 | 5.2E-1 | 4.9E-1 | 1.2E0 | 1.0E-9 | 1.0E-9 | 2.8E0 | 4.5E0 | 42 | 16 | 42 | 16 | 0.49 |
| Up | ng/ml | 1.0E-9 | 1.0E-9 | 8.9E-3 | 3.8E-3 | 1.9E-2 | 1.2E-2 | 1.0E-9 | 1.0E-9 | 9.5E-2 | 4.5E-2 | 42 | 16 | 42 | 16 | 0.36 |
| Us | ng/ml | 5.3E-3 | 1.9E-3 | 2.0E-2 | 5.4E-2 | 3.7E-2 | 9.7E-2 | 1.0E-9 | 1.0E-9 | 2.1E-1 | 3.6E-1 | 42 | 16 | 42 | 16 | 0.49 |
| Uv | ng/ml | 4.3E-3 | 2.6E-3 | 1.1E-2 | 5.3E-3 | 2.4E-2 | 8.4E-3 | 1.0E-9 | 1.0E-9 | 1.3E-1 | 3.3E-2 | 42 | 16 | 42 | 16 | 0.43 |
| Ut | ng/ml | 7.9E-1 | 7.2E-1 | 2.4E0 | 1.8E0 | 3.5E0 | 2.3E0 | 1.0E-9 | 2.9E-1 | 1.4E1 | 8.9E0 | 42 | 16 | 42 | 16 | 0.50 |
| Uu | ng/ml | 7.1E0 | 5.2E0 | 8.1E0 | 6.9E0 | 5.2E0 | 5.0E0 | 5.7E-1 | 1.5E0 | 2.2E1 | 2.1E1 | 42 | 16 | 42 | 16 | 0.42 |
| Vp | ng/ml | 1.0E-9 | 1.0E-9 | 4.1E-2 | 6.2E-2 | 1.5E-1 | 1.9E-1 | 1.0E-9 | 1.0E-9 | 7.1E-1 | 7.4E-1 | 43 | 16 | 43 | 16 | 0.51 |
| Vt | ng/ml | 7.3E0 | 1.2E1 | 1.1E1 | 1.3E1 | 1.3E1 | 7.1E0 | 1.1E0 | 1.4E0 | 6.5E1 | 2.8E1 | 43 | 16 | 43 | 16 | 0.65 |
| Vu | ng/ml | 1.0E-9 | 3.6E-1 | 2.2E0 | 2.6E0 | 4.4E0 | 5.5E0 | 1.0E-9 | 1.0E-9 | 2.1E1 | 2.2E1 | 42 | 16 | 42 | 16 | 0.56 |
| Vq | ng/ml | 4.3E2 | 6.2E2 | 1.0E3 | 1.6E3 | 2.1E3 | 3.6E3 | 9.0E-1 | 1.8E1 | 1.1E4 | 1.2E4 | 29 | 11 | 29 | 11 | 0.58 |
| Vo | ng/ml | 2.6E1 | 2.5E1 | 2.6E1 | 2.3E1 | 5.1E0 | 5.5E0 | 1.0E1 | 4.9E0 | 4.8E1 | 2.9E1 | 43 | 16 | 43 | 16 | 0.36 |
| Vs | ng/ml | 1.0E-9 | 1.0E-9 | 4.7E0 | 1.7E0 | 9.1E0 | 4.0E0 | 1.0E-9 | 1.0E-9 | 4.2E1 | 1.4E1 | 42 | 14 | 42 | 14 | 0.40 |
| Vv | ng/ml | 4.7E0 | 2.1E0 | 6.0E0 | 2.5E0 | 6.6E0 | 2.6E0 | 1.0E-9 | 1.0E-9 | 2.8E1 | 9.6E0 | 43 | 16 | 43 | 16 | 0.34 |
| Oy | pg/ml | 5.3E-1 | 3.7E-1 | 3.8E0 | 3.2E0 | 1.3E1 | 1.0E1 | 1.0E-9 | 1.0E-9 | 9.9E1 | 4.9E1 | 61 | 25 | 61 | 25 | 0.42 |
| Oz | pg/ml | 1.8E-1 | 8.8E-2 | 8.4E-1 | 3.7E-1 | 3.7E0 | 6.2E-1 | 1.0E-9 | 1.0E-9 | 2.9E1 | 2.2E0 | 61 | 25 | 61 | 25 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pa | pg/ml | 4.2E-1 | 6.1E-1 | 1.3E0 | 1.3E0 | 4.0E0 | 1.6E0 | 1.0E-9 | 1.0E-9 | 2.9E1 | 6.5E0 | 61 | 25 | 61 | 25 | 0.61 |
| Pb | pg/ml | 4.1E-2 | 1.0E-9 | 2.6E-1 | 1.1E-1 | 9.4E-1 | 2.3E-1 | 1.0E-9 | 1.0E-9 | 7.3E0 | 1.1E0 | 61 | 25 | 61 | 25 | 0.40 |
| Pc | pg/ml | 3.4E-1 | 3.4E-1 | 4.0E-1 | 3.6E-1 | 4.2E-1 | 3.8E-1 | 1.0E-9 | 1.0E-9 | 1.8E0 | 1.2E0 | 61 | 25 | 61 | 25 | 0.47 |
| Pd | pg/ml | 2.4E0 | 2.4E0 | 1.8E1 | 4.4E0 | 1.1E2 | 6.3E0 | 1.0E-9 | 1.0E-9 | 8.4E2 | 2.9E1 | 61 | 25 | 61 | 25 | 0.48 |
| Pe | pg/ml | 3.1E1 | 5.5E1 | 1.3E2 | 2.4E2 | 4.4E2 | 8.6E2 | 1.0E-9 | 1.0E-9 | 3.3E3 | 4.3E3 | 61 | 25 | 61 | 25 | 0.58 |
| Pf | pg/ml | 2.3E0 | 4.4E0 | 3.2E1 | 2.1E1 | 1.9E2 | 7.4E1 | 1.0E-9 | 1.0E-9 | 1.5E3 | 3.8E2 | 61 | 25 | 61 | 25 | 0.56 |
| Pg | pg/ml | 2.9E0 | 6.8E0 | 1.4E2 | 3.9E1 | 9.9E2 | 8.7E1 | 1.0E-9 | 1.0E-9 | 7.7E3 | 4.0E2 | 61 | 25 | 61 | 25 | 0.61 |
| Ph | ng/ml | 2.0E-1 | 1.8E-1 | 3.9E-1 | 3.4E-1 | 6.2E-1 | 5.5E-1 | 1.0E-9 | 1.0E-9 | 2.8E0 | 2.4E0 | 43 | 17 | 43 | 17 | 0.50 |
| Pi | ng/ml | 1.9E-1 | 2.4E-1 | 2.6E-1 | 3.0E-1 | 2.3E-1 | 3.3E-1 | 1.0E-9 | 1.0E-9 | 9.7E-1 | 1.4E0 | 43 | 17 | 43 | 17 | 0.54 |
| Pj | ng/mL | 7.1E0 | 5.7E0 | 8.5E0 | 7.1E0 | 6.1E0 | 4.5E0 | 9.9E-1 | 1.8E0 | 2.5E1 | 2.0E1 | 43 | 17 | 43 | 17 | 0.44 |
| Pk | ng/ml | 8.7E-3 | 9.7E-3 | 1.5E-2 | 1.1E-2 | 2.4E-2 | 8.5E-3 | 1.0E-9 | 1.0E-9 | 1.4E-1 | 2.6E-2 | 43 | 17 | 43 | 17 | 0.52 |
| aA | mg/dL | 8.5E-1 | 9.0E-1 | 9.0E-1 | 9.0E-1 | 4.1E-1 | 2.8E-1 | 2.6E-1 | 4.0E-1 | 2.6E0 | 1.4E0 | 85 | 27 | 85 | 27 | 0.54 |
| aC | mg/mL | 2.2E0 | 2.1E0 | 2.4E0 | 2.3E0 | 1.1E0 | 1.1E0 | 8.7E-1 | 7.5E-1 | 5.5E0 | 4.8E0 | 46 | 16 | 46 | 16 | 0.45 |
| aD | ug/mL | 3.4E0 | 4.7E0 | 4.7E0 | 5.9E0 | 3.8E0 | 4.9E0 | 8.7E-1 | 7.5E-1 | 2.0E1 | 2.1E1 | 46 | 16 | 46 | 16 | 0.58 |
| aE | mg/mL | 5.6E-1 | 5.1E-1 | 5.7E-1 | 5.4E-1 | 1.7E-1 | 2.0E-1 | 2.5E-1 | 1.8E-1 | 1.0E0 | 9.1E-1 | 46 | 16 | 46 | 16 | 0.46 |
| aF | ng/mL | 1.6E0 | 1.7E0 | 3.2E0 | 3.6E0 | 3.7E0 | 4.6E0 | 4.3E-3 | 3.7E-1 | 1.8E1 | 1.8E1 | 46 | 16 | 46 | 16 | 0.47 |
| aG | mg/mL | 1.6E-1 | 1.5E-1 | 1.7E-1 | 1.7E-1 | 8.9E-2 | 1.1E-1 | 5.1E-2 | 6.4E-2 | 4.0E-1 | 4.8E-1 | 46 | 16 | 46 | 16 | 0.47 |
| aH | ug/mL | 9.4E1 | 7.7E1 | 9.9E1 | 8.2E1 | 5.3E1 | 3.9E1 | 1.1E1 | 2.0E1 | 2.6E2 | 1.7E2 | 46 | 16 | 46 | 16 | 0.40 |
| aI | ug/mL | 2.0E2 | 1.5E2 | 2.0E2 | 1.5E2 | 6.4E1 | 5.3E1 | 4.8E1 | 7.8E1 | 3.4E2 | 2.8E2 | 46 | 16 | 46 | 16 | 0.28 |
| aJ | ug/mL | 2.4E0 | 3.0E0 | 3.0E0 | 3.5E0 | 2.1E0 | 2.1E0 | 1.0E0 | 8.2E-1 | 1.3E1 | 7.9E0 | 46 | 16 | 46 | 16 | 0.58 |
| aK | ng/mL | 9.8E-1 | 1.4E0 | 1.9E0 | 1.7E0 | 2.1E0 | 1.2E0 | 2.9E-4 | 4.1E-1 | 1.0E1 | 4.2E0 | 46 | 16 | 46 | 16 | 0.55 |
| aL | mg/mL | 7.9E-1 | 7.7E-1 | 8.1E-1 | 7.5E-1 | 2.6E-1 | 2.7E-1 | 3.0E-1 | 4.1E-1 | 1.7E0 | 1.5E0 | 46 | 16 | 46 | 16 | 0.43 |
| aM | U/mL | 3.0E1 | 2.5E1 | 4.6E1 | 4.6E1 | 4.1E1 | 4.8E1 | 4.2E-2 | 4.2E-2 | 1.6E2 | 1.3E2 | 46 | 16 | 46 | 16 | 0.48 |
| aN | U/mL | 1.8E1 | 2.2E1 | 3.3E1 | 2.8E1 | 5.7E1 | 2.5E1 | 2.5E-3 | 2.5E-3 | 3.8E2 | 8.5E1 | 46 | 16 | 46 | 16 | 0.52 |
| aO | pg/mL | 4.9E1 | 1.9E1 | 3.0E2 | 4.9E2 | 7.0E2 | 8.3E2 | 1.6E0 | 6.0E-2 | 3.6E3 | 2.5E3 | 46 | 16 | 46 | 16 | 0.44 |
| aP | ng/mL | 1.6E0 | 1.7E0 | 1.9E0 | 2.0E0 | 1.1E0 | 1.6E0 | 7.8E-1 | 6.8E-1 | 5.4E0 | 6.5E0 | 46 | 16 | 46 | 16 | 0.46 |
| aQ | ng/mL | 2.8E-1 | 2.2E-1 | 4.7E-1 | 2.8E-1 | 4.7E-1 | 2.1E-1 | 2.5E-2 | 5.9E-2 | 2.0E0 | 9.2E-1 | 46 | 16 | 46 | 16 | 0.42 |
| aR | ng/mL | 2.0E0 | 1.4E0 | 3.3E0 | 2.3E0 | 4.7E0 | 2.6E0 | 6.2E-1 | 6.2E-1 | 3.0E1 | 1.1E1 | 46 | 16 | 46 | 16 | 0.40 |
| aS | ng/mL | 3.5E-1 | 4.8E-1 | 8.2E-1 | 6.6E-1 | 1.3E0 | 6.0E-1 | 4.2E-3 | 7.0E-2 | 6.2E0 | 2.3E0 | 46 | 16 | 46 | 16 | 0.56 |
| aU | pg/mL | 6.1E1 | 6.1E1 | 1.1E2 | 6.8E1 | 1.4E2 | 3.7E1 | 7.4E-2 | 2.3E1 | 7.0E2 | 1.6E2 | 46 | 16 | 46 | 16 | 0.50 |
| aV | ng/mL | 6.1E-1 | 3.6E-1 | 1.7E0 | 5.5E-1 | 4.8E0 | 4.6E-1 | 3.8E-2 | 9.1E-2 | 3.3E1 | 1.6E0 | 46 | 16 | 46 | 16 | 0.40 |
| aW | pg/mL | 2.1E1 | 1.5E1 | 2.0E1 | 1.5E1 | 1.1E1 | 1.1E1 | 7.2E-2 | 7.2E-2 | 5.4E1 | 2.9E1 | 46 | 16 | 46 | 16 | 0.37 |
| aX | ng/mL | 7.7E0 | 1.5E1 | 1.1E1 | 1.4E1 | 1.1E1 | 9.8E0 | 7.0E-1 | 1.4E0 | 4.6E1 | 3.1E1 | 46 | 16 | 46 | 16 | 0.60 |
| aY | pg/mL | 6.6E1 | 4.6E1 | 1.0E2 | 4.7E1 | 1.8E2 | 3.5E1 | 4.1E-1 | 4.1E-1 | 1.2E3 | 1.1E2 | 46 | 16 | 46 | 16 | 0.35 |
| aZ | pg/mL | 1.6E2 | 2.6E2 | 4.3E2 | 4.6E2 | 9.0E2 | 5.9E2 | 1.7E0 | 1.5E1 | 5.9E3 | 2.1E3 | 46 | 16 | 46 | 16 | 0.56 |
| bA | ng/mL | 1.1E1 | 2.4E1 | 5.9E1 | 5.8E1 | 1.5E2 | 1.1E2 | 3.0E-2 | 3.0E-2 | 9.4E2 | 4.3E2 | 46 | 16 | 46 | 16 | 0.52 |
| bB | ng/mL | 3.2E2 | 2.8E2 | 3.2E2 | 2.7E2 | 1.8E2 | 1.7E2 | 3.6E1 | 2.3E1 | 8.1E2 | 6.2E2 | 46 | 16 | 46 | 16 | 0.41 |
| bC | ng/mL | 3.4E2 | 2.8E2 | 7.4E2 | 6.2E2 | 1.0E3 | 9.5E2 | 4.6E1 | 1.2E2 | 4.7E3 | 4.0E3 | 46 | 16 | 46 | 16 | 0.48 |
| bE | mg/mL | 5.1E0 | 5.0E0 | 5.6E0 | 5.3E0 | 2.3E0 | 1.9E0 | 1.4E0 | 1.8E0 | 1.2E1 | 9.6E0 | 46 | 16 | 46 | 16 | 0.46 |
| bF | pg/mL | 2.0E1 | 3.9E1 | 3.1E2 | 2.1E2 | 1.6E3 | 5.3E2 | 2.5E0 | 7.7E0 | 1.0E4 | 2.2E3 | 46 | 16 | 46 | 16 | 0.65 |
| bG | ng/mL | 1.2E0 | 1.2E0 | 2.5E0 | 3.4E0 | 3.6E0 | 3.9E0 | 2.5E-1 | 2.7E-1 | 1.7E1 | 1.3E1 | 46 | 16 | 46 | 16 | 0.58 |
| bH | pg/mL | 5.7E-1 | 5.7E-1 | 6.0E0 | 3.6E0 | 1.9E1 | 5.1E0 | 5.7E-1 | 5.7E-1 | 1.2E2 | 1.9E1 | 46 | 16 | 46 | 16 | 0.55 |
| bI | ng/mL | 4.0E-3 | 4.0E-3 | 8.9E-2 | 5.7E-2 | 1.7E-1 | 1.6E-1 | 4.0E-3 | 4.0E-3 | 7.1E-1 | 6.2E-1 | 46 | 16 | 46 | 16 | 0.47 |
| bJ | mg/mL | 2.0E0 | 1.6E0 | 2.5E0 | 1.9E0 | 2.3E0 | 1.3E0 | 2.5E-4 | 2.8E-1 | 1.1E1 | 4.9E0 | 46 | 16 | 46 | 16 | 0.45 |
| bL | pg/mL | 2.2E0 | 2.4E0 | 6.6E0 | 3.9E0 | 1.0E1 | 3.6E0 | 4.6E-2 | 4.6E-2 | 3.5E1 | 1.1E1 | 46 | 16 | 46 | 16 | 0.53 |
| bM | mg/mL | 2.4E0 | 2.2E0 | 2.9E0 | 2.3E0 | 1.6E0 | 1.2E0 | 4.1E-1 | 7.1E-1 | 7.9E0 | 4.8E0 | 46 | 16 | 46 | 16 | 0.41 |
| bN | ng/mL | 6.7E1 | 2.3E1 | 1.7E2 | 5.1E1 | 3.6E2 | 8.9E1 | 6.5E0 | 2.1E0 | 1.9E3 | 3.7E2 | 46 | 16 | 46 | 16 | 0.29 |
| bO | ng/mL | 4.0E-2 | 4.0E-2 | 4.7E0 | 5.6E0 | 1.1E1 | 1.1E1 | 4.0E-2 | 4.0E-2 | 4.3E1 | 3.7E1 | 46 | 16 | 46 | 16 | 0.50 |
| bP | mg/mL | 4.9E-1 | 6.4E-1 | 7.1E-1 | 8.1E-1 | 8.0E-1 | 7.2E-1 | 1.3E-1 | 1.1E-1 | 4.8E0 | 3.1E0 | 46 | 16 | 46 | 16 | 0.58 |
| bQ | pg/mL | 1.7E1 | 2.3E1 | 3.1E2 | 4.2E1 | 2.0E3 | 4.6E1 | 1.5E-1 | 6.4E0 | 1.3E4 | 1.8E2 | 46 | 16 | 46 | 16 | 0.64 |
| bR | ng/mL | 1.2E-2 | 1.2E-2 | 2.7E-1 | 9.1E-2 | 1.3E0 | 1.2E-1 | 1.2E-2 | 1.2E-2 | 8.7E0 | 3.9E-1 | 46 | 16 | 46 | 16 | 0.50 |
| bS | pg/mL | 9.4E-1 | 9.4E-1 | 1.2E1 | 3.5E0 | 5.8E1 | 1.0E1 | 9.4E-1 | 9.4E-1 | 3.9E2 | 4.3E1 | 46 | 16 | 46 | 16 | 0.48 |
| bU | ng/mL | 1.3E-2 | 8.5E-2 | 2.5E-1 | 1.3E-1 | 9.7E-1 | 1.7E-1 | 1.3E-2 | 1.3E-2 | 6.6E0 | 6.3E-1 | 46 | 16 | 46 | 16 | 0.52 |

| Code | Units | Median | | Average | | Standard Deviation | | Minimum | | Maximum | | Number of Samples | | Number of Patients | | AUC |
|------|-------|--------|--------|---------|--------|--------|--------|--------|--------|--------|--------|--------|-----|--------|-----|-----|
| | | NonDis | Dis | NonDis | Dis | NonDis | Dis | NonDis | Dis | NonDis | Dis | NonDis | Dis | NonDis | Dis | |
| bV | pg/mL | 4.5E2 | 5.2E2 | 5.0E2 | 6.8E2 | 1.7E2 | 6.6E2 | 2.3E2 | 3.0E2 | 8.2E2 | 3.1E3 | 46 | 16 | 46 | 16 | 0.57 |
| bW | pg/mL | 3.6E2 | 3.5E2 | 5.5E2 | 5.3E2 | 7.1E2 | 4.5E2 | 1.2E2 | 1.3E2 | 4.8E3 | 1.8E3 | 46 | 16 | 46 | 16 | 0.53 |
| bX | ng/mL | 2.5E-5 | 2.5E-5 | 1.7E-3 | 2.1E-3 | 2.5E-3 | 3.0E-3 | 2.5E-5 | 2.5E-5 | 8.8E-3 | 8.7E-3 | 46 | 16 | 46 | 16 | 0.52 |
| bZ | pg/mL | 2.5E2 | 2.9E2 | 1.7E3 | 1.0E3 | 8.6E3 | 1.6E3 | 2.4E1 | 5.7E1 | 5.8E4 | 5.6E3 | 46 | 16 | 46 | 16 | 0.60 |
| cA | pg/mL | 6.0E-1 | 6.0E-1 | 9.3E0 | 6.0E-1 | 5.5E1 | 0.0E0 | 6.0E-1 | 6.0E-1 | 3.7E2 | 6.0E-1 | 46 | 16 | 46 | 16 | 0.46 |
| cB | ng/mL | 5.0E-2 | 4.2E-2 | 8.1E-2 | 8.0E-2 | 9.2E-2 | 1.1E-1 | 1.7E-3 | 1.7E-3 | 3.7E-1 | 4.0E-1 | 46 | 16 | 46 | 16 | 0.46 |
| cC | pg/mL | 2.1E1 | 4.7E1 | 3.8E1 | 4.2E1 | 6.9E1 | 2.2E1 | 1.0E0 | 1.0E0 | 4.5E2 | 6.7E1 | 46 | 16 | 46 | 16 | 0.64 |
| cD | pg/mL | 3.3E0 | 4.3E0 | 1.0E1 | 8.4E0 | 2.9E1 | 1.3E1 | 3.3E-1 | 3.3E-1 | 1.4E2 | 4.5E1 | 46 | 16 | 46 | 16 | 0.61 |
| cE | pg/mL | 3.9E1 | 1.2E2 | 2.1E2 | 2.1E2 | 7.1E2 | 2.8E2 | 1.2E-1 | 1.2E-1 | 3.8E3 | 1.1E3 | 46 | 16 | 46 | 16 | 0.63 |
| cF | pg/mL | 9.4E0 | 5.0E0 | 1.8E1 | 1.3E1 | 4.0E1 | 1.6E1 | 5.3E-1 | 5.3E-1 | 2.7E2 | 5.0E1 | 46 | 16 | 46 | 16 | 0.48 |
| cG | pg/mL | 4.8E1 | 5.8E1 | 3.3E2 | 9.3E1 | 1.5E3 | 6.6E1 | 7.8E0 | 7.8E0 | 1.0E4 | 2.2E2 | 46 | 16 | 46 | 16 | 0.65 |
| cH | uIU/mL | 4.8E0 | 2.6E0 | 1.1E1 | 4.6E0 | 2.0E1 | 6.8E0 | 8.6E-3 | 8.6E-3 | 1.2E2 | 2.4E1 | 46 | 16 | 46 | 16 | 0.29 |
| cI | ng/mL | 5.8E0 | 1.1E1 | 1.7E1 | 1.8E1 | 2.3E1 | 2.1E1 | 8.0E-2 | 5.1E-1 | 9.4E1 | 6.4E1 | 46 | 16 | 46 | 16 | 0.52 |
| cJ | ug/mL | 6.3E1 | 6.0E1 | 1.3E2 | 9.4E1 | 1.7E2 | 1.0E2 | 1.1E1 | 7.2E0 | 6.9E2 | 3.4E2 | 46 | 16 | 46 | 16 | 0.49 |
| cK | ng/mL | 3.8E-3 | 3.8E-3 | 3.8E-2 | 3.8E-3 | 2.1E-1 | 0.0E0 | 3.8E-3 | 3.8E-3 | 1.5E0 | 3.8E-3 | 46 | 16 | 46 | 16 | 0.47 |
| cL | pg/mL | 1.7E2 | 2.0E2 | 8.7E2 | 2.4E2 | 3.6E3 | 1.7E2 | 1.6E1 | 3.1E1 | 2.4E4 | 7.6E2 | 46 | 16 | 46 | 16 | 0.54 |
| cM | pg/mL | 2.6E2 | 2.6E2 | 2.9E2 | 2.7E2 | 2.3E2 | 1.1E2 | 3.3E1 | 4.2E1 | 1.5E3 | 4.5E2 | 46 | 16 | 46 | 16 | 0.53 |
| cN | pg/mL | 1.1E2 | 1.2E2 | 1.4E2 | 1.2E2 | 1.5E2 | 3.0E1 | 4.3E1 | 7.3E1 | 1.1E3 | 1.7E2 | 46 | 16 | 46 | 16 | 0.52 |
| cO | pg/mL | 2.0E2 | 2.1E2 | 6.5E2 | 2.3E2 | 2.8E3 | 8.3E1 | 8.2E1 | 1.2E2 | 1.9E4 | 4.3E2 | 46 | 16 | 46 | 16 | 0.53 |
| cP | ng/mL | 2.9E3 | 2.4E3 | 2.9E3 | 2.7E3 | 9.7E2 | 1.1E3 | 1.1E3 | 1.6E3 | 5.5E3 | 5.9E3 | 46 | 16 | 46 | 16 | 0.39 |
| cQ | ng/mL | 6.2E-2 | 6.5E-2 | 1.5E-1 | 2.0E-1 | 2.7E-1 | 3.6E-1 | 2.0E-3 | 2.0E-3 | 1.4E0 | 1.3E0 | 46 | 16 | 46 | 16 | 0.47 |
| cR | ng/mL | 2.9E2 | 6.4E2 | 4.6E2 | 6.7E2 | 4.7E2 | 5.5E2 | 3.0E1 | 3.6E1 | 2.3E3 | 2.0E3 | 46 | 16 | 46 | 16 | 0.63 |
| cS | ng/mL | 2.6E2 | 2.5E2 | 3.2E2 | 6.2E2 | 2.1E2 | 8.3E2 | 7.0E1 | 1.4E2 | 9.4E2 | 2.6E3 | 46 | 16 | 46 | 16 | 0.53 |
| cT | ng/mL | 3.6E1 | 6.9E1 | 1.5E2 | 8.5E1 | 3.5E2 | 1.0E2 | 7.2E0 | 4.2E0 | 2.1E3 | 4.0E2 | 46 | 16 | 46 | 16 | 0.51 |
| cU | ng/mL | 6.5E1 | 5.9E1 | 1.2E2 | 9.6E1 | 2.4E2 | 1.0E2 | 1.4E1 | 1.7E1 | 1.6E3 | 4.2E2 | 46 | 16 | 46 | 16 | 0.48 |
| cV | ng/mL | 1.8E-1 | 2.8E-1 | 2.8E-1 | 3.7E-1 | 4.4E-1 | 2.4E-1 | 3.7E-2 | 4.1E-2 | 2.5E0 | 8.9E-1 | 46 | 16 | 46 | 16 | 0.73 |
| cW | mIU/mL | 4.2E-2 | 4.0E-2 | 7.3E-2 | 5.5E-2 | 7.3E-2 | 4.1E-2 | 4.8E-3 | 1.4E-2 | 2.9E-1 | 1.6E-1 | 46 | 16 | 46 | 16 | 0.49 |
| cX | ng/mL | 2.0E-1 | 5.3E-2 | 1.2E0 | 7.3E-1 | 3.3E0 | 1.3E0 | 2.3E-4 | 2.3E-4 | 2.1E1 | 4.4E0 | 46 | 16 | 46 | 16 | 0.39 |
| cY | ng/mL | 7.3E0 | 7.3E0 | 1.3E1 | 8.2E0 | 1.5E1 | 4.9E0 | 1.5E-1 | 9.8E-1 | 6.1E1 | 1.8E1 | 46 | 16 | 46 | 16 | 0.49 |
| cZ | ug/mL | 1.5E1 | 1.2E1 | 1.6E1 | 1.3E1 | 7.6E0 | 6.4E0 | 3.1E0 | 4.5E0 | 3.7E1 | 3.1E1 | 46 | 16 | 46 | 16 | 0.35 |
| dA | pg/mL | 3.6E2 | 3.2E2 | 5.0E2 | 3.1E2 | 8.2E2 | 1.2E2 | 1.3E2 | 1.5E2 | 5.8E3 | 5.5E2 | 46 | 16 | 46 | 16 | 0.37 |
| dB | ug/mL | 3.8E0 | 1.9E1 | 1.1E1 | 1.6E1 | 1.0E1 | 1.0E1 | 1.8E0 | 2.2E0 | 3.1E1 | 3.0E1 | 46 | 16 | 46 | 16 | 0.61 |
| dC | nmol/L | 3.4E1 | 3.8E1 | 3.7E1 | 3.8E1 | 1.3E1 | 1.6E1 | 1.7E1 | 7.8E0 | 7.7E1 | 6.9E1 | 46 | 16 | 46 | 16 | 0.54 |
| dD | ug/mL | 3.4E1 | 3.0E1 | 3.6E1 | 3.1E1 | 1.1E1 | 1.1E1 | 1.3E1 | 1.4E1 | 5.7E1 | 4.6E1 | 46 | 16 | 46 | 16 | 0.36 |
| dE | ng/mL | 8.4E-3 | 8.4E-3 | 3.8E-1 | 2.3E-1 | 5.6E-1 | 2.8E-1 | 8.4E-3 | 8.4E-3 | 2.0E0 | 7.0E-1 | 46 | 16 | 46 | 16 | 0.47 |
| dF | ng/mL | 2.3E2 | 2.9E2 | 2.9E2 | 3.9E2 | 2.0E2 | 2.9E2 | 1.0E2 | 7.5E1 | 1.3E3 | 1.2E3 | 46 | 16 | 46 | 16 | 0.65 |
| dG | ng/mL | 1.2E1 | 1.4E1 | 1.7E1 | 1.7E1 | 2.6E1 | 1.3E1 | 6.3E0 | 3.0E0 | 1.8E2 | 5.8E1 | 46 | 16 | 46 | 16 | 0.57 |
| dH | pg/mL | 8.0E0 | 9.4E0 | 2.4E1 | 1.2E1 | 9.8E1 | 1.1E1 | 4.0E-2 | 4.0E-2 | 6.7E2 | 4.9E1 | 46 | 16 | 46 | 16 | 0.56 |
| dI | pg/mL | 4.6E-1 | 4.6E-1 | 8.3E0 | 8.7E-1 | 4.9E1 | 9.0E-1 | 4.6E-1 | 4.6E-1 | 3.3E2 | 2.9E0 | 46 | 16 | 46 | 16 | 0.49 |
| dJ | ng/mL | 2.2E0 | 1.9E0 | 2.3E0 | 1.8E0 | 1.2E0 | 7.1E-1 | 5.6E-1 | 3.7E-1 | 5.1E0 | 2.9E0 | 46 | 16 | 46 | 16 | 0.38 |
| dK | uIU/mL | 1.6E0 | 1.2E0 | 2.3E0 | 2.2E0 | 3.3E0 | 2.1E0 | 1.8E-1 | 1.9E-1 | 2.1E1 | 6.5E0 | 46 | 16 | 46 | 16 | 0.50 |
| dL | ng/mL | 9.0E2 | 9.9E2 | 1.1E3 | 1.1E3 | 5.7E2 | 7.0E2 | 4.7E2 | 2.8E2 | 3.2E3 | 3.3E3 | 46 | 16 | 46 | 16 | 0.51 |
| dM | pg/mL | 1.1E3 | 1.1E3 | 1.1E3 | 1.3E3 | 6.0E2 | 6.9E2 | 4.2E2 | 3.7E2 | 3.3E3 | 3.1E3 | 46 | 16 | 46 | 16 | 0.57 |
| dN | ug/mL | 9.8E1 | 8.6E1 | 9.9E1 | 9.8E1 | 3.3E1 | 5.3E1 | 3.7E1 | 2.4E1 | 1.9E2 | 2.1E2 | 46 | 16 | 46 | 16 | 0.42 |
| dR | pg/ml | 1.6E3 | 1.4E3 | 2.4E3 | 1.9E3 | 2.4E3 | 1.5E3 | 1.7E2 | 3.4E2 | 9.8E3 | 5.3E3 | 44 | 16 | 44 | 16 | 0.48 |
| eF | ng/ml | 4.2E0 | 4.0E0 | 4.7E0 | 4.8E0 | 2.0E0 | 2.1E0 | 2.0E0 | 2.1E0 | 1.2E1 | 1.0E1 | 45 | 16 | 45 | 16 | 0.50 |
| eC | pg/ml | 3.3E2 | 2.2E2 | 3.5E2 | 2.4E2 | 1.5E2 | 1.6E2 | 5.1E1 | 1.9E1 | 7.5E2 | 4.9E2 | 41 | 13 | 41 | 13 | 0.31 |
| eD | pg/ml | 2.1E2 | 2.4E2 | 5.5E2 | 6.3E2 | 1.0E3 | 1.2E3 | 5.2E-1 | 1.4E2 | 5.5E3 | 3.8E3 | 33 | 9 | 33 | 9 | 0.58 |
| eM | ng/ml | 3.0E0 | 3.2E0 | 5.2E0 | 2.9E0 | 4.7E0 | 2.2E0 | 1.5E0 | 6.9E-1 | 1.8E1 | 7.3E0 | 12 | 9 | 12 | 9 | 0.34 |
| fP | ng/ml | 2.6E2 | 4.0E2 | 2.7E2 | 4.3E2 | 1.1E2 | 2.3E2 | 4.8E1 | 1.3E2 | 5.2E2 | 8.6E2 | 42 | 16 | 42 | 16 | 0.69 |
| fR | ng/ml | 1.1E5 | 1.7E5 | 1.8E5 | 2.4E5 | 1.8E5 | 1.4E5 | 3.6E4 | 9.9E4 | 6.9E5 | 4.8E5 | 19 | 8 | 19 | 8 | 0.68 |
| gC | ng/ml | 1.8E2 | 2.8E2 | 2.5E2 | 3.1E2 | 1.4E2 | 9.2E1 | 1.3E2 | 1.8E2 | 5.9E2 | 4.5E2 | 13 | 9 | 13 | 9 | 0.70 |
| gL | pg/ml | 6.1E4 | 6.0E4 | 6.6E4 | 6.5E4 | 2.5E4 | 2.4E4 | 1.1E4 | 2.1E4 | 1.6E5 | 1.3E5 | 44 | 16 | 44 | 16 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| gP | U/ml | 3.1E2 | 2.4E2 | 3.1E2 | 2.5E2 | 9.4E1 | 1.0E2 | 1.7E2 | 9.6E1 | 6.5E2 | 5.2E2 | 44 | 16 | 44 | 16 | 0.29 |
| gW | ng/ml | 3.9E2 | 6.1E2 | 7.8E2 | 1.2E3 | 1.0E3 | 1.4E3 | 2.3E0 | 1.9E2 | 4.2E3 | 4.3E3 | 39 | 9 | 39 | 9 | 0.64 |
| tF | pg/mL | 6.3E2 | 3.2E3 | 1.2E4 | 5.7E3 | 3.9E4 | 6.1E3 | 1.2E1 | 1.8E1 | 2.3E5 | 1.7E4 | 41 | 14 | 41 | 14 | 0.61 |
| hA | ng/ml | 2.0E0 | 3.0E0 | 1.7E1 | 6.6E0 | 5.4E1 | 5.8E0 | 1.7E-2 | 1.7E-2 | 2.9E2 | 1.5E1 | 33 | 9 | 33 | 9 | 0.64 |
| nM | pg/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 20 | 12 | 20 | 12 | 0.50 |
| nN | pg/ml | 1.4E3 | 2.2E3 | 2.3E3 | 3.9E3 | 3.1E3 | 5.5E3 | 1.9E2 | 2.8E2 | 1.3E4 | 2.1E4 | 20 | 12 | 20 | 12 | 0.63 |
| nO | pg/ml | 2.4E1 | 2.4E1 | 3.8E1 | 4.6E1 | 3.4E1 | 6.5E1 | 3.5E0 | 8.1E0 | 1.4E2 | 2.4E2 | 20 | 12 | 20 | 12 | 0.50 |
| nR | pg/ml | 1.1E1 | 1.6E1 | 2.4E1 | 6.9E1 | 3.6E1 | 1.3E2 | 1.7E0 | 1.0E0 | 1.5E2 | 4.2E2 | 20 | 12 | 20 | 12 | 0.59 |
| nT | pg/ml | 1.1E2 | 8.7E1 | 1.0E2 | 9.1E1 | 8.9E1 | 4.2E1 | 1.0E-9 | 1.4E1 | 3.3E2 | 1.9E2 | 20 | 12 | 20 | 12 | 0.49 |
| nU | pg/ml | 3.0E1 | 1.1E1 | 5.6E1 | 5.9E1 | 5.9E1 | 8.1E1 | 1.0E-9 | 1.0E-9 | 1.9E2 | 2.2E2 | 20 | 12 | 20 | 12 | 0.48 |
| lW | pg/ml | 1.0E-9 | 1.0E-9 | 9.0E0 | 1.9E0 | 3.7E1 | 6.6E0 | 1.0E-9 | 1.0E-9 | 1.6E2 | 2.3E1 | 20 | 12 | 20 | 12 | 0.45 |
| lX | pg/ml | 1.2E3 | 5.2E2 | 1.2E3 | 7.1E2 | 6.0E2 | 4.4E2 | 1.2E2 | 1.9E2 | 2.3E3 | 1.6E3 | 20 | 12 | 20 | 12 | 0.25 |
| lY | pg/ml | 2.8E1 | 1.9E1 | 3.1E1 | 1.8E1 | 2.4E1 | 9.0E0 | 5.4E0 | 3.7E0 | 1.2E2 | 3.0E1 | 20 | 12 | 20 | 12 | 0.26 |
| mE | pg/ml | 1.0E-9 | 1.0E-9 | 4.5E0 | 2.5E0 | 1.3E1 | 4.3E0 | 1.0E-9 | 1.0E-9 | 5.7E1 | 1.3E1 | 20 | 12 | 20 | 12 | 0.48 |
| mF | pg/ml | 1.0E-9 | 2.7E-1 | 6.3E0 | 2.7E0 | 2.3E1 | 4.5E0 | 1.0E-9 | 1.0E-9 | 1.1E2 | 1.3E1 | 20 | 12 | 20 | 12 | 0.57 |
| mH | pg/ml | 3.4E0 | 2.3E0 | 6.2E0 | 3.6E0 | 1.1E1 | 3.2E0 | 3.2E-1 | 9.8E-1 | 5.3E1 | 1.1E1 | 20 | 12 | 20 | 12 | 0.41 |
| mI | pg/ml | 1.8E0 | 1.0E-9 | 1.5E1 | 1.4E1 | 4.0E1 | 2.7E1 | 1.0E-9 | 1.0E-9 | 1.8E2 | 7.8E1 | 20 | 12 | 20 | 12 | 0.46 |
| mM | pg/ml | 2.1E1 | 2.7E1 | 4.6E1 | 5.3E1 | 6.5E1 | 8.2E1 | 1.0E-9 | 1.0E-9 | 2.4E2 | 2.9E2 | 20 | 12 | 20 | 12 | 0.52 |
| mP | pg/ml | 1.5E1 | 1.5E1 | 1.4E1 | 1.4E1 | 8.2E0 | 1.0E1 | 1.0E-9 | 1.0E-9 | 2.9E1 | 3.8E1 | 20 | 12 | 20 | 12 | 0.49 |
| mS | pg/ml | 1.6E3 | 1.2E3 | 1.6E3 | 1.6E3 | 7.6E2 | 1.2E3 | 1.0E-9 | 6.4E2 | 2.9E3 | 5.1E3 | 20 | 12 | 20 | 12 | 0.38 |
| mT | pg/ml | 4.3E1 | 5.9E1 | 7.5E1 | 6.5E1 | 6.6E1 | 3.5E1 | 1.6E1 | 1.9E1 | 2.8E2 | 1.4E2 | 20 | 12 | 20 | 12 | 0.53 |
| mU | pg/ml | 1.9E0 | 3.2E0 | 2.5E0 | 3.2E0 | 2.2E0 | 1.9E0 | 1.9E-1 | 1.0E-9 | 1.1E1 | 7.7E0 | 20 | 12 | 20 | 12 | 0.68 |
| mW | pg/ml | 2.4E3 | 2.2E3 | 2.7E3 | 2.4E3 | 1.2E3 | 1.3E3 | 1.1E3 | 7.7E2 | 5.6E3 | 5.7E3 | 20 | 12 | 20 | 12 | 0.41 |
| mY | pg/ml | 5.9E2 | 6.3E2 | 7.3E2 | 8.6E2 | 4.7E2 | 7.2E2 | 2.1E2 | 2.5E2 | 2.2E3 | 2.5E3 | 20 | 12 | 20 | 12 | 0.50 |
| mZ | pg/ml | 2.0E2 | 2.1E2 | 3.3E2 | 5.1E2 | 2.9E2 | 7.9E2 | 2.8E1 | 1.1E1 | 1.1E3 | 2.8E3 | 20 | 12 | 20 | 12 | 0.45 |
| nA | pg/ml | 1.5E0 | 4.8E-1 | 6.0E0 | 6.7E0 | 1.4E1 | 1.8E1 | 1.0E-9 | 1.0E-9 | 6.2E1 | 6.5E1 | 20 | 12 | 20 | 12 | 0.39 |
| nB | pg/ml | 3.1E2 | 3.3E2 | 3.3E2 | 3.5E2 | 1.4E2 | 1.8E2 | 9.7E1 | 3.8E1 | 6.4E2 | 6.9E2 | 20 | 12 | 20 | 12 | 0.53 |
| nC | pg/ml | 1.0E-9 | 8.5E1 | 8.0E1 | 1.5E5 | 1.8E2 | 4.4E5 | 1.0E-9 | 1.0E-9 | 6.2E2 | 1.5E6 | 20 | 12 | 20 | 12 | 0.64 |
| nD | pg/ml | 7.9E0 | 3.6E0 | 7.1E0 | 2.9E1 | 5.2E0 | 7.3E1 | 1.0E-9 | 1.0E-9 | 1.8E1 | 2.6E2 | 20 | 12 | 20 | 12 | 0.49 |
| nF | pg/ml | 1.0E-9 | 1.0E-9 | 1.5E0 | 2.3E0 | 5.0E0 | 7.9E0 | 1.0E-9 | 1.0E-9 | 2.1E1 | 2.7E1 | 20 | 12 | 20 | 12 | 0.50 |
| nH | pg/ml | 2.0E-1 | 9.8E-1 | 1.8E0 | 1.1E3 | 3.0E0 | 2.9E3 | 1.0E-9 | 1.0E-9 | 1.2E1 | 1.0E4 | 20 | 12 | 20 | 12 | 0.57 |
| nI | pg/ml | 2.8E0 | 5.0E1 | 5.8E1 | 7.1E1 | 8.1E1 | 1.0E2 | 1.0E-9 | 1.0E-9 | 2.6E2 | 3.5E2 | 20 | 12 | 20 | 12 | 0.50 |
| nJ | pg/ml | 1.0E-9 | 8.4E-2 | 5.3E-1 | 1.1E1 | 8.3E-1 | 3.8E1 | 1.0E-9 | 1.0E-9 | 2.4E0 | 1.3E2 | 20 | 12 | 20 | 12 | 0.54 |
| nK | pg/ml | 1.0E-9 | 1.0E-9 | 1.3E1 | 1.4E1 | 2.1E1 | 3.0E1 | 1.0E-9 | 1.0E-9 | 6.4E1 | 1.0E2 | 20 | 12 | 20 | 12 | 0.49 |
| nL | pg/ml | 1.0E-9 | 1.0E-9 | 4.9E0 | 1.6E3 | 1.1E1 | 4.1E3 | 1.0E-9 | 1.0E-9 | 4.5E1 | 1.4E4 | 20 | 12 | 20 | 12 | 0.60 |
| hR | pg/ml | 2.4E4 | 3.1E4 | 2.4E4 | 3.0E4 | 1.0E4 | 9.4E3 | 3.6E3 | 1.6E4 | 4.9E4 | 4.3E4 | 32 | 9 | 32 | 9 | 0.67 |
| hV | pg/ml | 4.3E2 | 4.4E2 | 4.5E2 | 4.1E2 | 2.4E2 | 2.1E2 | 6.8E1 | 1.5E2 | 9.6E2 | 7.4E2 | 32 | 9 | 32 | 9 | 0.45 |
| hW | pg/ml | 1.6E3 | 2.4E3 | 2.2E3 | 2.8E3 | 1.7E3 | 1.6E3 | 2.2E2 | 1.1E3 | 6.7E3 | 6.4E3 | 32 | 9 | 32 | 9 | 0.67 |
| hX | pg/ml | 8.7E2 | 1.4E3 | 9.1E2 | 1.8E3 | 4.5E2 | 1.9E3 | 1.3E2 | 3.1E2 | 2.3E3 | 6.6E3 | 32 | 9 | 32 | 9 | 0.74 |
| iA | pg/ml | 1.2E2 | 1.5E2 | 1.7E2 | 2.8E2 | 1.6E2 | 3.1E2 | 1.2E1 | 1.5E1 | 7.9E2 | 8.7E2 | 41 | 14 | 41 | 14 | 0.57 |
| iB | ng/ml | 3.4E0 | 7.8E0 | 4.1E0 | 8.4E0 | 2.9E0 | 6.4E0 | 4.5E-2 | 1.6E0 | 1.4E1 | 1.9E1 | 33 | 9 | 33 | 9 | 0.69 |
| iC | U/ml | 1.9E-1 | 5.8E-1 | 4.3E-1 | 2.0E0 | 8.7E-1 | 3.9E0 | 1.0E-9 | 6.8E-2 | 4.8E0 | 1.2E1 | 33 | 9 | 33 | 9 | 0.67 |
| iH | ng/ml | 1.4E5 | 1.7E5 | 1.4E5 | 1.4E5 | 5.0E4 | 5.7E4 | 7.4E4 | 2.9E3 | 2.5E5 | 2.0E5 | 41 | 14 | 41 | 14 | 0.54 |
| iJ | ng/ml | 5.3E4 | 4.5E4 | 5.6E4 | 4.6E4 | 3.9E4 | 2.1E4 | 8.0E3 | 1.8E3 | 2.5E5 | 7.5E4 | 41 | 14 | 41 | 14 | 0.46 |
| hB | ng/ml | 3.6E-1 | 5.0E-1 | 4.5E-1 | 7.6E-1 | 2.8E-1 | 7.9E-1 | 1.2E-1 | 1.2E-1 | 1.3E0 | 3.2E0 | 41 | 14 | 41 | 14 | 0.69 |
| hC | pg/ml | 4.3E3 | 7.1E3 | 1.0E4 | 8.2E3 | 1.8E4 | 8.1E3 | 1.0E-9 | 4.1E1 | 1.1E5 | 2.7E4 | 41 | 14 | 41 | 14 | 0.49 |
| hF | ng/ml | 1.0E-9 | 1.0E-9 | 1.6E-2 | 6.8E-1 | 1.0E-1 | 2.6E0 | 1.0E-9 | 1.0E-9 | 6.6E-1 | 9.6E0 | 41 | 14 | 41 | 14 | 0.52 |
| hG | pg/ml | 6.7E3 | 5.9E3 | 7.3E3 | 6.5E3 | 3.2E3 | 2.7E3 | 2.3E3 | 3.6E3 | 1.9E4 | 1.2E4 | 41 | 14 | 41 | 14 | 0.42 |
| iO | ng/ml | 3.6E5 | 3.7E5 | 3.8E5 | 4.2E5 | 1.8E5 | 2.0E5 | 8.3E4 | 1.8E5 | 9.0E5 | 8.2E5 | 41 | 14 | 41 | 14 | 0.56 |
| iP | ng/ml | 5.0E4 | 5.5E4 | 5.3E4 | 5.0E4 | 4.1E4 | 2.3E4 | 2.4E3 | 1.0E4 | 2.2E5 | 7.7E4 | 41 | 14 | 41 | 14 | 0.53 |
| iZ | ng/ml | 1.6E3 | 1.9E3 | 1.8E3 | 1.7E3 | 7.1E2 | 5.9E2 | 8.8E2 | 9.8E2 | 4.3E3 | 2.6E3 | 39 | 13 | 39 | 13 | 0.46 |
| rC | pg/ml | 1.6E3 | 1.0E3 | 2.2E3 | 1.4E3 | 2.1E3 | 1.3E3 | 1.0E-9 | 7.0E1 | 1.1E4 | 3.8E3 | 33 | 9 | 33 | 9 | 0.39 |
| rB | pg/ml | 2.5E1 | 5.1E1 | 3.2E1 | 6.9E1 | 2.3E1 | 7.6E1 | 1.0E-9 | 1.0E-9 | 9.2E1 | 2.5E2 | 33 | 9 | 33 | 9 | 0.67 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| jD | ng/ml | 4.1E1 | 2.4E1 | 4.6E1 | 3.5E1 | 3.7E1 | 3.1E1 | 1.0E-9 | 1.0E-9 | 1.5E2 | 9.9E1 | 33 | 9 | 33 | 9 | 0.42 |
| jE | ng/ml | 1.0E-9 | 1.0E-9 | 7.6E0 | 7.1E0 | 1.3E1 | 1.8E1 | 1.0E-9 | 1.0E-9 | 5.2E1 | 5.6E1 | 33 | 9 | 33 | 9 | 0.46 |
| jF | ng/ml | 3.7E1 | 3.8E1 | 5.8E1 | 3.7E1 | 6.6E1 | 3.6E1 | 1.0E-9 | 1.0E-9 | 1.9E2 | 1.1E2 | 33 | 9 | 33 | 9 | 0.45 |
| jG | ng/ml | 4.4E3 | 5.4E3 | 4.5E3 | 4.4E3 | 2.0E3 | 2.2E3 | 1.2E3 | 6.0E2 | 9.6E3 | 6.5E3 | 33 | 9 | 33 | 9 | 0.52 |
| jH | ng/ml | 7.5E1 | 7.1E1 | 8.3E1 | 6.6E1 | 5.5E1 | 1.7E1 | 2.5E1 | 3.6E1 | 3.3E2 | 8.7E1 | 33 | 9 | 33 | 9 | 0.42 |
| jI | ng/ml | 6.8E1 | 9.9E1 | 7.3E1 | 9.6E1 | 2.5E1 | 4.9E1 | 3.8E1 | 5.0E1 | 1.4E2 | 1.9E2 | 33 | 9 | 33 | 9 | 0.63 |
| rA | pg/ml | 2.3E1 | 2.0E1 | 3.4E1 | 2.4E1 | 2.8E1 | 1.5E1 | 1.0E-9 | 6.9E0 | 1.1E2 | 4.8E1 | 34 | 9 | 34 | 9 | 0.42 |
| qZ | pg/ml | 4.4E1 | 9.3E1 | 4.6E2 | 1.6E3 | 2.0E3 | 3.7E3 | 4.8E-4 | 5.3E-3 | 1.0E4 | 1.0E4 | 24 | 7 | 24 | 7 | 0.60 |
| qY | pg/ml | 2.6E1 | 1.1E1 | 6.3E1 | 1.2E1 | 7.6E1 | 6.4E0 | 2.9E0 | 5.1E0 | 3.3E2 | 2.5E1 | 34 | 9 | 34 | 9 | 0.27 |
| qX | pg/ml | 5.6E1 | 6.8E1 | 6.9E1 | 7.3E1 | 4.7E1 | 4.5E1 | 1.0E-9 | 1.7E1 | 2.1E2 | 1.3E2 | 34 | 9 | 34 | 9 | 0.54 |
| qW | pg/ml | 8.4E0 | 1.0E1 | 1.2E1 | 1.1E1 | 1.1E1 | 1.0E1 | 1.0E-9 | 1.0E-9 | 5.1E1 | 3.1E1 | 34 | 9 | 34 | 9 | 0.50 |
| qV | pg/ml | 2.2E3 | 2.4E3 | 2.7E3 | 2.9E3 | 2.1E3 | 2.6E3 | 1.0E2 | 9.5E2 | 8.2E3 | 9.6E3 | 34 | 9 | 34 | 9 | 0.50 |
| qU | pg/ml | 8.5E1 | 1.2E2 | 2.0E2 | 2.4E2 | 3.8E2 | 2.7E2 | 1.0E-9 | 1.0E-9 | 2.1E3 | 7.9E2 | 34 | 9 | 34 | 9 | 0.57 |
| qT | pg/ml | 4.1E1 | 7.7E1 | 5.8E1 | 8.4E1 | 4.8E1 | 6.2E1 | 1.0E-9 | 6.9E0 | 1.8E2 | 2.0E2 | 34 | 9 | 34 | 9 | 0.63 |
| jK | ng/ml | 1.8E3 | 1.5E3 | 1.8E3 | 1.6E3 | 7.4E2 | 8.9E2 | 7.2E2 | 8.0E2 | 4.0E3 | 3.6E3 | 33 | 9 | 33 | 9 | 0.37 |
| jL | ng/ml | 2.5E2 | 2.0E2 | 3.5E2 | 2.5E2 | 3.8E2 | 1.5E2 | 4.9E1 | 1.3E2 | 2.1E3 | 5.9E2 | 33 | 9 | 33 | 9 | 0.46 |
| jM | ng/ml | 6.4E4 | 5.2E4 | 8.0E4 | 6.1E4 | 4.8E4 | 3.6E4 | 1.1E3 | 5.7E3 | 1.7E5 | 1.2E5 | 33 | 9 | 33 | 9 | 0.39 |
| jO | pg/ml | 2.2E5 | 2.5E5 | 2.4E5 | 2.8E5 | 1.0E5 | 1.3E5 | 7.7E4 | 1.4E5 | 4.6E5 | 5.2E5 | 33 | 9 | 33 | 9 | 0.59 |
| jP | pg/ml | 2.5E5 | 2.8E5 | 2.7E5 | 3.7E5 | 1.6E5 | 3.4E5 | 7.4E4 | 1.3E5 | 7.2E5 | 1.2E6 | 33 | 9 | 33 | 9 | 0.59 |
| jQ | pg/ml | 2.8E3 | 1.7E3 | 4.0E3 | 2.4E3 | 3.9E3 | 1.9E3 | 1.4E2 | 4.6E2 | 1.4E4 | 6.1E3 | 33 | 9 | 33 | 9 | 0.42 |
| jR | pg/ml | 6.8E3 | 3.3E3 | 1.3E4 | 8.8E3 | 1.4E4 | 1.2E4 | 1.0E-9 | 3.0E1 | 5.6E4 | 3.4E4 | 33 | 9 | 33 | 9 | 0.39 |
| jT | pg/ml | 1.9E5 | 1.6E5 | 1.9E5 | 1.6E5 | 7.7E4 | 7.0E4 | 7.7E4 | 7.5E4 | 3.8E5 | 2.8E5 | 33 | 9 | 33 | 9 | 0.41 |
| jU | mIU/ml | 4.8E0 | 4.8E0 | 1.0E1 | 9.4E0 | 1.5E1 | 9.8E0 | 6.2E-2 | 8.2E-1 | 6.7E1 | 3.0E1 | 33 | 9 | 33 | 9 | 0.53 |
| jV | mIU/ml | 1.2E0 | 1.2E0 | 2.7E0 | 1.9E0 | 5.2E0 | 2.0E0 | 1.7E-3 | 7.6E-2 | 2.6E1 | 6.6E0 | 33 | 9 | 33 | 9 | 0.55 |
| jY | ng/ml | 4.2E-4 | 2.7E-3 | 3.8E-3 | 6.6E-3 | 9.6E-3 | 8.0E-3 | 1.0E-9 | 1.0E-9 | 5.1E-2 | 2.4E-2 | 33 | 9 | 33 | 9 | 0.72 |
| kC | pg/ml | 8.5E1 | 9.3E1 | 1.4E2 | 3.2E2 | 2.0E2 | 7.6E2 | 2.1E1 | 5.4E1 | 9.8E2 | 2.7E3 | 20 | 12 | 20 | 12 | 0.54 |
| kE | pg/ml | 1.4E5 | 1.2E5 | 1.4E5 | 1.3E5 | 4.3E4 | 4.4E4 | 4.1E4 | 7.5E4 | 2.1E5 | 2.0E5 | 20 | 12 | 20 | 12 | 0.39 |
| kF | pg/mL | 5.6E1 | 7.0E1 | 6.2E1 | 7.0E1 | 2.7E1 | 2.7E1 | 2.8E1 | 3.4E1 | 1.5E2 | 1.3E2 | 20 | 12 | 20 | 12 | 0.61 |
| kG | pg/mL | 9.7E3 | 1.1E4 | 1.2E4 | 1.5E4 | 8.8E3 | 1.4E4 | 3.3E3 | 3.8E3 | 3.6E4 | 5.0E4 | 20 | 12 | 20 | 12 | 0.53 |
| kI | pg/ml | 1.8E2 | 1.8E2 | 1.9E2 | 1.9E2 | 7.8E1 | 7.8E1 | 4.4E1 | 7.2E1 | 4.2E2 | 3.1E2 | 20 | 12 | 20 | 12 | 0.53 |
| kK | pg/ml | 9.1E1 | 7.5E1 | 1.2E2 | 1.6E2 | 9.6E1 | 1.7E2 | 2.9E1 | 2.9E1 | 4.6E2 | 5.1E2 | 20 | 12 | 20 | 12 | 0.49 |
| kN | pg/ml | 1.0E3 | 1.1E3 | 1.5E3 | 4.2E3 | 1.6E3 | 1.1E4 | 2.3E2 | 3.9E2 | 6.3E3 | 3.9E4 | 20 | 12 | 20 | 12 | 0.50 |
| kO | pg/ml | 7.0E3 | 5.9E3 | 7.0E3 | 1.9E4 | 2.2E3 | 4.1E4 | 3.4E3 | 3.8E3 | 1.3E4 | 1.5E5 | 20 | 12 | 20 | 12 | 0.45 |
| kP | pg/ml | 5.6E3 | 5.3E3 | 9.6E3 | 5.8E3 | 1.1E4 | 2.9E3 | 2.1E3 | 1.4E3 | 4.8E4 | 1.1E4 | 20 | 12 | 20 | 12 | 0.43 |
| kQ | pg/ml | 4.2E3 | 3.5E3 | 5.1E3 | 3.9E3 | 3.4E3 | 1.8E3 | 1.1E3 | 1.4E3 | 1.8E4 | 7.8E3 | 41 | 14 | 41 | 14 | 0.40 |
| kR | pg/ml | 2.2E1 | 2.1E1 | 2.4E1 | 2.8E1 | 1.6E1 | 2.5E1 | 1.4E-1 | 3.4E0 | 5.9E1 | 8.2E1 | 41 | 14 | 41 | 14 | 0.50 |
| kS | pg/ml | 9.3E2 | 6.7E2 | 1.1E3 | 8.7E2 | 8.2E2 | 6.9E2 | 2.1E2 | 2.9E2 | 4.1E3 | 3.0E3 | 41 | 14 | 41 | 14 | 0.36 |
| rZ | ng/ml | 1.0E-9 | 6.9E-3 | 4.7E-3 | 9.4E-3 | 8.3E-3 | 1.4E-2 | 1.0E-9 | 1.0E-9 | 3.1E-2 | 4.5E-2 | 32 | 9 | 32 | 9 | 0.65 |
| rY | ng/ml | 6.1E-2 | 8.7E-2 | 8.0E-1 | 9.5E-2 | 4.1E0 | 5.2E-2 | 1.0E-9 | 3.2E-2 | 2.3E1 | 2.1E-1 | 32 | 9 | 32 | 9 | 0.68 |
| rX | ng/ml | 1.0E-9 | 1.0E-9 | 9.4E-2 | 4.6E-3 | 5.3E-1 | 8.5E-3 | 1.0E-9 | 1.0E-9 | 3.0E0 | 2.4E-2 | 32 | 9 | 32 | 9 | 0.66 |
| lK | pg/ml | 1.6E2 | 6.8E1 | 2.7E2 | 1.1E2 | 5.8E2 | 1.1E2 | 1.0E-9 | 4.8E0 | 3.3E3 | 2.9E2 | 33 | 9 | 33 | 9 | 0.40 |
| lL | pg/ml | 1.3E3 | 1.7E3 | 2.1E3 | 2.4E3 | 2.1E3 | 2.0E3 | 1.5E1 | 1.9E2 | 6.9E3 | 5.8E3 | 33 | 9 | 33 | 9 | 0.60 |
| lM | pg/ml | 1.1E3 | 2.6E3 | 2.9E3 | 4.7E3 | 5.3E3 | 5.0E3 | 2.1E2 | 2.4E1 | 2.9E4 | 1.3E4 | 33 | 9 | 33 | 9 | 0.61 |
| lN | pg/ml | 1.0E-9 | 4.7E0 | 2.7E0 | 7.0E0 | 4.9E0 | 8.0E0 | 1.0E-9 | 1.0E-9 | 2.4E1 | 2.1E1 | 33 | 9 | 33 | 9 | 0.66 |
| lO | pg/ml | 1.0E-9 | 1.0E-9 | 3.8E0 | 3.7E0 | 2.2E1 | 1.1E1 | 1.0E-9 | 1.0E-9 | 1.3E2 | 3.4E1 | 33 | 9 | 33 | 9 | 0.54 |
| nW | pg/ml | 1.1E5 | 1.0E5 | 1.1E5 | 1.1E5 | 2.3E4 | 3.7E4 | 6.5E4 | 3.6E4 | 1.5E5 | 1.9E5 | 41 | 14 | 41 | 14 | 0.46 |
| nY | pg/ml | 2.4E3 | 2.5E3 | 2.5E3 | 2.9E3 | 1.4E3 | 2.0E3 | 1.0E3 | 6.3E2 | 7.1E3 | 8.1E3 | 41 | 14 | 41 | 14 | 0.54 |
| oO | pg/ml | 8.3E4 | 9.6E4 | 1.2E5 | 1.0E5 | 9.0E4 | 4.7E4 | 3.8E4 | 2.0E4 | 3.1E5 | 2.0E5 | 19 | 12 | 19 | 12 | 0.56 |
| oP | pg/ml | 1.2E5 | 1.4E5 | 1.4E5 | 1.6E5 | 9.5E4 | 1.1E5 | 4.8E4 | 5.6E4 | 4.5E5 | 4.6E5 | 19 | 12 | 19 | 12 | 0.57 |
| oQ | pg/ml | 2.5E3 | 3.3E3 | 3.8E3 | 3.7E3 | 3.2E3 | 2.2E3 | 1.6E3 | 7.7E2 | 1.4E4 | 8.4E3 | 19 | 12 | 19 | 12 | 0.58 |
| oE | pg/ml | 1.1E2 | 4.0E2 | 3.1E2 | 6.9E2 | 5.2E2 | 8.1E2 | 1.0E-9 | 5.4E1 | 1.9E3 | 2.6E3 | 41 | 14 | 41 | 14 | 0.72 |
| oF | pg/ml | 9.7E3 | 1.7E4 | 2.8E4 | 4.0E4 | 4.8E4 | 6.2E4 | 7.8E2 | 5.1E2 | 2.5E5 | 1.8E5 | 41 | 14 | 41 | 14 | 0.55 |
| oH | pg/ml | 4.4E1 | 3.0E1 | 9.0E1 | 5.7E1 | 1.0E2 | 6.0E1 | 6.2E0 | 1.2E1 | 4.8E2 | 1.8E2 | 41 | 14 | 41 | 14 | 0.39 |

| Code | Units | Median | | Average | | Standard Deviation | | Minimum | | Maximum | | Number of Samples | | Number of Patients | | AUC |
|------|-------|--------|-----|---------|-----|--------|------|---------|------|---------|------|--------|-----|--------|-----|------|
| | | NonDis | Dis | NonDis | Dis | NonDis | Dis | NonDis | Dis | NonDis | Dis | NonDis | Dis | NonDis | Dis | |
| oK | pg/ml | 9.5E2 | 7.8E2 | 1.7E3 | 5.2E3 | 1.9E3 | 1.4E4 | 1.4E2 | 2.3E2 | 7.2E3 | 5.3E4 | 41 | 14 | 41 | 14 | 0.52 |
| oN | pg/ml | 5.7E2 | 4.4E2 | 6.9E2 | 8.5E2 | 7.7E2 | 1.3E3 | 2.8E2 | 1.1E2 | 5.3E3 | 5.0E3 | 41 | 14 | 41 | 14 | 0.40 |
| pF | pg/ml | 6.0E-1 | 5.4E-1 | 2.7E0 | 7.9E-1 | 1.3E1 | 5.7E-1 | 1.0E-9 | 1.0E-9 | 8.7E1 | 1.7E0 | 41 | 14 | 41 | 14 | 0.57 |

Figure 28.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.5E1 | 1.1E2 | 8.6E1 | 1.3E2 | 5.6E1 | 9.6E1 | 7.0E0 | 1.6E1 | 4.0E2 | 4.8E2 | 248 | 50 | 248 | 50 | 0.66 |
| Ad | ug/mL | 4.4E-2 | 7.6E-2 | 7.0E-2 | 3.4E-1 | 8.6E-2 | 1.4E0 | 6.8E-4 | 7.8E-4 | 5.4E-1 | 8.5E0 | 139 | 35 | 139 | 35 | 0.63 |
| Af | ng/mL | 1.3E0 | 6.7E-1 | 1.1E1 | 4.4E0 | 4.9E1 | 6.7E0 | 1.7E-3 | 1.7E-3 | 5.3E2 | 2.3E1 | 139 | 35 | 139 | 35 | 0.43 |
| Aj | ug/mL | 1.6E0 | 3.2E-1 | 2.5E0 | 1.6E0 | 2.5E0 | 2.3E0 | 1.5E-3 | 1.3E-3 | 6.1E0 | 6.1E0 | 139 | 35 | 139 | 35 | 0.37 |
| Al | mg/mL | 8.3E-5 | 1.2E-4 | 2.5E-4 | 3.7E-4 | 4.3E-4 | 4.9E-4 | 4.3E-6 | 7.6E-6 | 1.8E-3 | 1.8E-3 | 139 | 35 | 139 | 35 | 0.57 |
| An | U/mL | 5.8E1 | 9.0E1 | 2.1E2 | 4.3E2 | 5.8E2 | 1.3E3 | 2.8E-1 | 6.4E-1 | 5.5E3 | 7.8E3 | 139 | 35 | 139 | 35 | 0.59 |
| Ao | pg/mL | 9.1E1 | 1.4E2 | 6.0E2 | 3.5E2 | 3.9E3 | 7.9E2 | 1.5E0 | 4.1E0 | 3.9E4 | 4.5E3 | 139 | 35 | 139 | 35 | 0.61 |
| Ap | ng/mL | 3.2E1 | 4.7E1 | 4.7E1 | 6.5E1 | 5.5E1 | 6.2E1 | 2.0E0 | 4.4E0 | 3.3E2 | 2.4E2 | 139 | 35 | 139 | 35 | 0.61 |
| Ar | ng/mL | 5.6E-1 | 2.2E0 | 2.4E0 | 6.1E0 | 5.9E0 | 1.1E1 | 3.4E-3 | 3.4E-3 | 5.1E1 | 5.0E1 | 139 | 35 | 139 | 35 | 0.69 |
| As | ng/mL | 8.7E-3 | 1.0E-2 | 1.2E-2 | 4.6E-2 | 1.7E-2 | 2.1E-1 | 1.7E-3 | 1.7E-3 | 1.3E-1 | 1.2E0 | 139 | 35 | 139 | 35 | 0.51 |
| Aw | pg/mL | 1.6E1 | 1.7E1 | 1.7E1 | 1.9E1 | 5.8E0 | 7.6E0 | 2.9E-2 | 1.1E1 | 4.2E1 | 5.1E1 | 139 | 35 | 139 | 35 | 0.57 |
| Ax | ng/mL | 1.5E0 | 8.8E0 | 1.4E1 | 9.2E1 | 6.8E1 | 2.0E2 | 1.3E-2 | 1.0E-4 | 7.7E2 | 8.5E2 | 139 | 35 | 139 | 35 | 0.68 |
| Ba | ng/mL | 7.7E1 | 3.0E2 | 5.3E2 | 1.4E3 | 1.4E3 | 2.9E3 | 1.1E0 | 3.1E0 | 8.1E3 | 1.5E4 | 139 | 35 | 139 | 35 | 0.66 |
| Bb | ng/mL | 3.7E0 | 7.6E0 | 6.3E0 | 1.2E1 | 7.9E0 | 1.1E1 | 4.1E-3 | 3.5E-1 | 4.9E1 | 4.8E1 | 139 | 35 | 139 | 35 | 0.67 |
| Bc | ng/mL | 3.2E1 | 7.2E1 | 1.2E2 | 1.8E2 | 2.2E2 | 2.8E2 | 4.9E-1 | 2.9E0 | 1.2E3 | 1.0E3 | 139 | 35 | 139 | 35 | 0.63 |
| Bg | ng/mL | 1.1E-1 | 1.6E-1 | 4.6E0 | 1.2E1 | 1.9E1 | 6.7E1 | 5.3E-4 | 1.9E-2 | 1.5E2 | 4.0E2 | 139 | 35 | 139 | 35 | 0.58 |
| Bn | ng/mL | 5.6E-2 | 5.6E-2 | 1.3E0 | 2.4E0 | 2.1E0 | 9.8E0 | 5.6E-2 | 5.6E-2 | 8.6E0 | 5.8E1 | 139 | 35 | 139 | 35 | 0.48 |
| Bo | ng/mL | 1.3E1 | 1.7E1 | 1.5E1 | 1.7E1 | 1.1E1 | 1.2E1 | 1.6E-2 | 1.6E-2 | 5.0E1 | 5.3E1 | 139 | 35 | 139 | 35 | 0.57 |
| Ch | uIU/mL | 1.1E0 | 9.6E-1 | 3.3E1 | 3.8E1 | 1.7E2 | 2.0E2 | 3.4E-3 | 3.4E-3 | 1.8E3 | 1.2E3 | 139 | 35 | 139 | 35 | 0.46 |
| Co | pg/mL | 4.4E1 | 6.1E1 | 2.4E2 | 1.9E2 | 1.5E3 | 3.7E2 | 1.5E-1 | 8.1E0 | 1.7E4 | 2.1E3 | 139 | 35 | 139 | 35 | 0.63 |
| Cp | ng/mL | 2.2E1 | 2.2E1 | 2.7E1 | 6.9E1 | 2.1E1 | 2.1E2 | 6.0E-1 | 2.5E0 | 1.3E2 | 1.3E3 | 139 | 35 | 139 | 35 | 0.58 |
| Cq | ng/mL | 2.8E-2 | 4.2E-2 | 1.1E-1 | 1.6E0 | 4.8E-1 | 8.3E0 | 8.0E-4 | 8.0E-4 | 5.1E0 | 4.9E1 | 139 | 35 | 139 | 35 | 0.59 |
| Cs | ng/mL | 4.9E1 | 2.2E2 | 3.0E2 | 1.2E3 | 9.6E2 | 3.2E3 | 8.9E-1 | 8.3E-1 | 1.1E4 | 1.8E4 | 139 | 35 | 139 | 35 | 0.67 |
| Ct | ng/mL | 3.6E-1 | 2.3E-1 | 4.2E1 | 3.7E1 | 1.3E2 | 1.1E2 | 1.3E-2 | 1.1E-4 | 6.2E2 | 4.7E2 | 139 | 35 | 139 | 35 | 0.47 |
| Cu | ng/mL | 2.5E-1 | 3.8E-1 | 4.6E-1 | 3.4E0 | 8.9E-1 | 1.1E1 | 1.9E-2 | 1.7E-2 | 9.0E0 | 6.6E1 | 139 | 35 | 139 | 35 | 0.64 |
| Cv | ng/mL | 4.1E0 | 1.3E1 | 2.5E1 | 4.7E1 | 6.5E1 | 9.2E1 | 2.0E-2 | 2.4E-2 | 5.3E2 | 4.7E2 | 139 | 35 | 139 | 35 | 0.59 |
| Cw | mIU/mL | 2.9E-2 | 4.7E-2 | 4.2E-2 | 2.4E-1 | 3.6E-2 | 1.1E0 | 8.9E-4 | 4.1E-3 | 2.3E-1 | 6.8E0 | 139 | 35 | 139 | 35 | 0.62 |
| Cx | ng/mL | 2.1E0 | 1.6E-2 | 5.3E1 | 3.9E1 | 1.0E2 | 9.0E1 | 9.3E-5 | 9.3E-5 | 3.9E2 | 2.8E2 | 139 | 35 | 139 | 35 | 0.40 |
| Db | ug/mL | 7.4E0 | 8.1E0 | 8.8E0 | 8.5E0 | 7.9E0 | 5.9E0 | 4.5E-1 | 8.8E-1 | 5.9E1 | 3.1E1 | 139 | 35 | 139 | 35 | 0.51 |
| Dc | nmol/L | 1.8E-2 | 6.3E-2 | 5.0E-2 | 6.0E-1 | 1.4E-1 | 2.4E0 | 5.2E-6 | 1.3E-3 | 1.6E0 | 1.4E1 | 139 | 35 | 139 | 35 | 0.68 |
| Dd | ug/mL | 6.3E-2 | 2.3E-1 | 1.6E-1 | 3.7E-1 | 2.5E-1 | 6.4E-1 | 4.8E-4 | 3.4E-3 | 1.6E0 | 3.6E0 | 139 | 35 | 139 | 35 | 0.63 |
| De | ng/mL | 3.4E-3 | 3.4E-3 | 7.8E-2 | 1.1E-1 | 1.3E-1 | 2.4E-1 | 3.4E-3 | 3.4E-3 | 9.2E-1 | 1.1E0 | 139 | 35 | 139 | 35 | 0.50 |
| Dg | ng/mL | 3.3E1 | 5.2E1 | 4.5E1 | 6.0E1 | 4.0E1 | 4.2E1 | 7.1E-1 | 1.9E0 | 1.9E2 | 1.5E2 | 139 | 35 | 139 | 35 | 0.61 |
| Di | pg/mL | 2.0E0 | 2.9E0 | 2.4E0 | 3.0E0 | 2.3E0 | 1.8E0 | 1.8E-1 | 1.8E-1 | 1.3E1 | 9.0E0 | 139 | 35 | 139 | 35 | 0.62 |
| Dk | uIU/mL | 1.4E-2 | 1.8E-2 | 5.2E-2 | 1.0E-1 | 1.7E-1 | 2.1E-1 | 1.1E-4 | 1.1E-4 | 1.6E0 | 9.8E-1 | 139 | 35 | 139 | 35 | 0.58 |
| Dl | ng/mL | 1.9E2 | 2.9E2 | 2.8E2 | 4.0E2 | 2.5E2 | 3.8E2 | 5.5E0 | 4.4E0 | 1.2E3 | 1.6E3 | 139 | 35 | 139 | 35 | 0.59 |
| Dp | ng/ml | 2.3E0 | 1.8E0 | 5.5E0 | 5.2E0 | 8.1E0 | 1.2E1 | 3.7E-3 | 3.7E-3 | 4.6E1 | 5.6E1 | 111 | 24 | 111 | 24 | 0.41 |
| Dr | pg/ml | 2.1E1 | 3.4E1 | 4.1E1 | 1.0E3 | 5.5E1 | 3.0E3 | 7.5E-1 | 7.5E-1 | 2.5E2 | 1.0E4 | 60 | 12 | 60 | 12 | 0.68 |
| Du | pg/ml | 1.4E2 | 4.4E2 | 9.7E2 | 3.2E3 | 2.9E3 | 7.7E3 | 1.2E0 | 1.2E0 | 2.0E4 | 2.4E4 | 48 | 9 | 48 | 9 | 0.60 |
| Ef | ng/ml | 9.5E-2 | 2.1E-1 | 7.8E-1 | 1.4E0 | 1.7E0 | 2.9E0 | 5.7E-4 | 5.7E-4 | 1.0E1 | 9.9E0 | 119 | 28 | 119 | 28 | 0.57 |
| Wm | % | 8.5E-2 | 4.5E-1 | 9.6E0 | 7.7E1 | 7.0E1 | 2.5E2 | 5.4E-2 | 8.5E-2 | 7.7E2 | 1.0E3 | 130 | 29 | 130 | 29 | 0.57 |
| Ed | pg/ml | 5.2E-1 | 2.9E1 | 2.6E1 | 8.7E1 | 4.0E1 | 1.4E2 | 5.2E-1 | 5.2E-1 | 1.9E2 | 5.0E2 | 111 | 24 | 111 | 24 | 0.66 |
| Yf | ng/mL | 1.5E1 | 2.0E1 | 4.7E1 | 9.2E1 | 9.4E1 | 1.4E2 | 2.9E-1 | 2.9E-1 | 5.9E2 | 3.5E2 | 51 | 8 | 51 | 8 | 0.54 |
| Tj | pg/mL | 3.7E-1 | 5.0E-1 | 3.9E1 | 3.5E1 | 2.3E2 | 7.9E1 | 3.7E-1 | 3.7E-1 | 2.3E3 | 3.1E2 | 119 | 26 | 119 | 26 | 0.58 |
| Po | pg/ml | 1.3E-1 | 5.2E0 | 8.0E0 | 2.2E1 | 2.6E1 | 4.5E1 | 8.0E-3 | 2.6E-2 | 2.7E2 | 2.2E2 | 293 | 61 | 293 | 61 | 0.66 |
| Ti | ug/mL | 3.0E0 | 6.9E0 | 4.5E0 | 8.0E0 | 3.9E0 | 5.3E0 | 1.2E-1 | 9.7E-1 | 1.6E1 | 1.8E1 | 73 | 11 | 73 | 11 | 0.70 |
| Em | ng/ml | 2.9E-3 | 2.6E-2 | 4.0E-2 | 1.9E-1 | 7.5E-2 | 4.5E-1 | 8.4E-4 | 8.4E-4 | 5.0E-1 | 1.9E0 | 73 | 19 | 73 | 19 | 0.59 |
| Et | ng/ml | 1.3E3 | 2.9E3 | 1.5E3 | 2.7E3 | 1.1E3 | 1.3E3 | 7.5E1 | 5.9E2 | 4.8E3 | 5.0E3 | 292 | 61 | 292 | 61 | 0.76 |
| Eq | pg/ml | 2.3E2 | 8.2E1 | 3.5E2 | 3.2E2 | 3.8E2 | 5.2E2 | 1.0E0 | 1.0E0 | 1.5E3 | 1.4E3 | 48 | 9 | 48 | 9 | 0.41 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Th | ug/mL | 1.2E0 | 1.6E0 | 1.7E0 | 2.0E0 | 1.5E0 | 2.0E0 | 1.2E-1 | 2.6E-3 | 7.5E0 | 5.9E0 | 73 | 11 | 73 | 11 | 0.49 |
| Fa | ng/ml | 3.9E1 | 7.5E1 | 9.5E1 | 2.3E2 | 3.6E2 | 5.1E2 | 2.6E-1 | 6.0E-1 | 3.7E3 | 2.5E3 | 107 | 24 | 107 | 24 | 0.68 |
| Ez | ng/ml | 3.4E0 | 5.3E0 | 1.4E1 | 2.3E1 | 2.7E1 | 4.4E1 | 1.3E-2 | 1.3E-2 | 1.6E2 | 2.0E2 | 111 | 24 | 111 | 24 | 0.59 |
| Fb | ng/ml | 2.5E1 | 2.7E1 | 2.2E1 | 2.6E1 | 1.2E1 | 9.8E0 | 6.6E-1 | 8.1E-1 | 4.3E1 | 4.1E1 | 108 | 24 | 108 | 24 | 0.58 |
| Ex | ng/ml | 5.7E-2 | 1.6E-1 | 1.7E-1 | 4.2E-1 | 3.3E-1 | 8.7E-1 | 3.5E-5 | 1.7E-4 | 2.2E0 | 4.1E0 | 82 | 23 | 82 | 23 | 0.69 |
| Fc | pg/ml | 2.2E-1 | 2.2E-1 | 2.0E1 | 4.2E1 | 8.4E1 | 1.0E2 | 2.2E-1 | 2.2E-1 | 4.5E2 | 3.1E2 | 49 | 9 | 49 | 9 | 0.62 |
| Fd | pg/ml | 2.3E1 | 4.9E2 | 4.1E2 | 6.5E3 | 7.3E2 | 1.0E4 | 4.5E-1 | 9.8E-1 | 2.9E3 | 2.5E4 | 49 | 9 | 49 | 9 | 0.71 |
| Fi | pg/ml | 2.5E-1 | 6.9E0 | 8.4E1 | 1.3E2 | 3.0E2 | 2.0E2 | 2.5E-1 | 2.5E-1 | 1.8E3 | 5.3E2 | 49 | 9 | 49 | 9 | 0.68 |
| Fn | ng/ml | 2.1E-1 | 2.5E-1 | 3.7E0 | 4.8E0 | 7.1E0 | 7.2E0 | 1.1E-14 | 1.1E-14 | 3.7E1 | 2.7E1 | 111 | 24 | 111 | 24 | 0.54 |
| Fp | ng/ml | 1.1E1 | 3.1E1 | 2.1E1 | 3.6E1 | 2.5E1 | 3.3E1 | 6.0E-3 | 1.2E0 | 1.3E2 | 1.3E2 | 295 | 61 | 295 | 61 | 0.65 |
| Fr | ng/ml | 3.0E4 | 9.2E4 | 1.1E5 | 2.4E5 | 1.8E5 | 2.7E5 | 1.9E2 | 1.8E3 | 8.4E5 | 8.4E5 | 300 | 63 | 300 | 63 | 0.70 |
| Fw | pg/ml | 2.0E0 | 9.0E0 | 4.0E1 | 7.7E1 | 2.7E2 | 1.9E2 | 1.2E-1 | 1.2E-1 | 3.0E3 | 9.1E2 | 121 | 28 | 121 | 28 | 0.62 |
| Fy | ng/ml | 3.4E1 | 5.9E1 | 5.4E1 | 1.6E2 | 5.4E1 | 2.1E2 | 1.2E-1 | 1.5E0 | 2.8E2 | 6.5E2 | 111 | 21 | 111 | 21 | 0.64 |
| Gh | pg/ml | 3.5E0 | 7.8E-1 | 2.4E1 | 9.0E0 | 5.8E1 | 1.6E1 | 2.9E-2 | 2.9E-2 | 3.2E2 | 4.6E1 | 49 | 9 | 49 | 9 | 0.46 |
| Gb | % | 4.1E1 | 5.3E1 | 4.6E1 | 9.0E1 | 4.1E1 | 9.2E1 | 2.2E0 | 2.5E1 | 2.3E2 | 3.0E2 | 49 | 9 | 49 | 9 | 0.68 |
| Gc | ng/ml | 9.5E1 | 1.4E2 | 1.5E2 | 2.6E2 | 2.0E2 | 2.1E2 | 6.4E0 | 6.5E1 | 1.2E3 | 6.7E2 | 60 | 12 | 60 | 12 | 0.70 |
| Gd | ng/ml | 3.3E1 | 2.7E1 | 3.4E1 | 2.9E1 | 1.8E1 | 2.1E1 | 3.0E0 | 7.6E0 | 8.1E1 | 8.0E1 | 68 | 14 | 68 | 14 | 0.39 |
| Gn | U/ml | 2.1E-1 | 2.8E-1 | 1.3E0 | 9.8E0 | 4.0E0 | 3.3E1 | 5.6E-3 | 2.7E-2 | 3.0E1 | 1.1E2 | 59 | 12 | 59 | 12 | 0.56 |
| Gl | pg/ml | 8.9E3 | 1.3E4 | 1.2E4 | 1.5E4 | 9.5E3 | 1.1E4 | 9.1E1 | 5.2E2 | 3.2E4 | 3.2E4 | 121 | 28 | 121 | 28 | 0.59 |
| Gp | U/ml | 1.2E0 | 9.8E-1 | 2.6E0 | 1.6E0 | 3.6E0 | 2.4E0 | 1.5E-2 | 1.5E-2 | 2.0E1 | 7.8E0 | 121 | 27 | 121 | 27 | 0.41 |
| Gz | ug/ml | 1.5E0 | 9.7E-1 | 5.7E0 | 4.2E0 | 5.7E0 | 5.8E0 | 4.2E-2 | 1.0E-1 | 2.5E1 | 1.9E1 | 74 | 20 | 74 | 20 | 0.47 |
| Ha | ng/ml | 2.0E0 | 3.0E0 | 7.1E0 | 1.6E1 | 1.6E1 | 3.2E1 | 1.7E-2 | 1.7E-2 | 1.0E2 | 1.0E2 | 109 | 24 | 109 | 24 | 0.57 |
| Nm | pg/ml | 1.2E4 | 2.4E4 | 3.0E4 | 7.6E4 | 7.7E4 | 1.4E5 | 1.0E-9 | 1.0E-9 | 9.6E5 | 8.2E5 | 296 | 61 | 296 | 61 | 0.63 |
| Nn | pg/ml | 1.4E2 | 4.9E2 | 1.4E3 | 1.0E4 | 7.1E3 | 4.3E4 | 1.0E-9 | 1.0E-9 | 9.5E4 | 3.1E5 | 296 | 61 | 296 | 61 | 0.68 |
| No | pg/ml | 1.3E1 | 3.3E1 | 2.8E1 | 1.0E2 | 5.3E1 | 1.9E2 | 1.0E-9 | 1.6E0 | 5.6E2 | 9.1E2 | 296 | 61 | 296 | 61 | 0.71 |
| Nq | pg/ml | 1.4E0 | 6.6E0 | 1.9E1 | 4.9E1 | 6.9E1 | 1.2E2 | 1.0E-9 | 1.0E-9 | 6.7E2 | 6.3E2 | 296 | 61 | 296 | 61 | 0.62 |
| Nr | pg/ml | 1.2E0 | 5.7E0 | 1.9E1 | 6.8E1 | 7.6E1 | 2.0E2 | 1.0E-9 | 1.0E-9 | 9.8E2 | 1.4E3 | 296 | 61 | 296 | 61 | 0.65 |
| Ns | pg/ml | 1.0E-9 | 1.0E-9 | 9.0E0 | 1.2E-1 | 6.9E1 | 6.4E-1 | 1.0E-9 | 1.0E-9 | 1.1E3 | 3.6E0 | 296 | 61 | 296 | 61 | 0.48 |
| Nt | pg/ml | 9.8E1 | 1.4E2 | 1.3E2 | 2.2E2 | 1.0E2 | 2.7E2 | 9.8E-1 | 4.5E1 | 8.8E2 | 1.7E3 | 296 | 61 | 296 | 61 | 0.66 |
| Nu | pg/ml | 1.5E1 | 5.0E1 | 5.2E1 | 8.1E1 | 9.2E1 | 9.4E1 | 1.0E-9 | 1.0E-9 | 6.3E2 | 3.7E2 | 296 | 61 | 296 | 61 | 0.63 |
| Lu | pg/ml | 1.0E4 | 1.0E4 | 1.6E4 | 1.1E4 | 4.3E4 | 9.4E3 | 7.7E2 | 5.2E2 | 5.6E5 | 5.7E4 | 296 | 61 | 296 | 61 | 0.49 |
| Lv | pg/ml | 1.0E-9 | 9.9E0 | 1.3E1 | 3.4E1 | 2.5E1 | 5.3E1 | 1.0E-9 | 1.0E-9 | 2.4E2 | 2.6E2 | 296 | 61 | 296 | 61 | 0.62 |
| Lw | pg/ml | 1.0E-9 | 1.0E-9 | 4.1E-1 | 4.9E0 | 5.0E0 | 2.4E1 | 1.0E-9 | 1.0E-9 | 8.0E1 | 1.8E2 | 296 | 61 | 296 | 61 | 0.56 |
| Lx | pg/ml | 1.0E-9 | 1.7E2 | 1.4E2 | 8.6E2 | 5.1E2 | 2.9E3 | 1.0E-9 | 1.0E-9 | 6.2E3 | 2.2E4 | 296 | 61 | 296 | 61 | 0.72 |
| Ly | pg/ml | 1.0E-9 | 1.0E-9 | 9.4E0 | 7.7E0 | 1.8E1 | 1.5E1 | 1.0E-9 | 1.0E-9 | 1.1E2 | 6.0E1 | 296 | 61 | 296 | 61 | 0.48 |
| Lz | pg/ml | 1.0E-9 | 1.0E-9 | 3.3E0 | 4.4E0 | 2.8E1 | 1.4E1 | 1.0E-9 | 1.0E-9 | 4.3E2 | 6.2E1 | 296 | 61 | 296 | 61 | 0.54 |
| Ma | pg/ml | 3.6E2 | 1.2E3 | 1.9E3 | 4.8E3 | 5.4E3 | 9.3E3 | 1.0E-9 | 2.4E1 | 6.5E4 | 5.2E4 | 296 | 61 | 296 | 61 | 0.67 |
| Mb | pg/ml | 2.5E1 | 2.8E1 | 3.2E1 | 3.3E1 | 1.8E1 | 1.5E1 | 9.2E0 | 4.1E0 | 2.1E2 | 7.1E1 | 296 | 61 | 296 | 61 | 0.54 |
| Mc | pg/ml | 1.0E-9 | 1.0E-9 | 6.4E-2 | 1.0E-9 | 8.0E-1 | 0.0E0 | 1.0E-9 | 1.0E-9 | 1.3E1 | 1.0E-9 | 296 | 61 | 296 | 61 | 0.49 |
| Md | pg/ml | 1.0E-9 | 1.0E-9 | 6.6E-1 | 6.9E-1 | 5.6E0 | 3.8E0 | 1.0E-9 | 1.0E-9 | 6.8E1 | 2.9E1 | 296 | 61 | 296 | 61 | 0.52 |
| Me | pg/ml | 3.1E1 | 3.5E1 | 3.1E1 | 3.3E1 | 2.5E1 | 2.5E1 | 1.0E-9 | 1.0E-9 | 3.2E2 | 1.8E2 | 296 | 61 | 296 | 61 | 0.56 |
| Mf | pg/ml | 1.0E-9 | 1.0E-9 | 6.5E-1 | 5.4E-1 | 4.2E0 | 1.6E0 | 1.0E-9 | 1.0E-9 | 5.6E1 | 9.1E0 | 296 | 61 | 296 | 61 | 0.53 |
| Mg | pg/ml | 9.4E-1 | 1.8E0 | 6.2E0 | 1.1E1 | 1.2E1 | 2.4E1 | 1.0E-9 | 1.0E-9 | 9.2E1 | 1.5E2 | 296 | 61 | 296 | 61 | 0.55 |
| Mh | pg/ml | 1.0E-9 | 1.0E-9 | 1.1E0 | 1.7E0 | 7.8E0 | 6.1E0 | 1.0E-9 | 1.0E-9 | 1.1E2 | 4.2E1 | 296 | 61 | 296 | 61 | 0.56 |
| Mi | pg/ml | 1.0E-9 | 1.0E-9 | 9.1E-1 | 1.8E1 | 8.6E0 | 7.8E1 | 1.0E-9 | 1.0E-9 | 1.2E2 | 5.2E2 | 296 | 61 | 296 | 61 | 0.57 |
| Mj | pg/ml | 1.0E-9 | 1.0E-9 | 5.8E0 | 1.4E1 | 3.3E1 | 3.8E1 | 1.0E-9 | 1.0E-9 | 4.6E2 | 2.1E2 | 296 | 61 | 296 | 61 | 0.58 |
| Mk | pg/ml | 5.3E-1 | 3.7E0 | 1.6E1 | 1.6E1 | 9.9E1 | 6.6E1 | 1.0E-9 | 1.0E-9 | 1.3E3 | 5.0E2 | 296 | 61 | 296 | 61 | 0.53 |
| Ml | pg/ml | 1.0E-9 | 1.0E-9 | 9.7E0 | 1.7E1 | 1.2E2 | 7.9E1 | 1.0E-9 | 1.0E-9 | 2.1E3 | 5.1E2 | 296 | 61 | 296 | 61 | 0.49 |
| Mm | pg/ml | 4.6E2 | 1.0E3 | 9.7E2 | 1.8E3 | 1.3E3 | 2.1E3 | 1.0E-9 | 1.0E-9 | 1.0E4 | 1.0E4 | 296 | 61 | 296 | 61 | 0.65 |
| Mn | pg/ml | 5.3E0 | 1.1E1 | 1.1E1 | 1.3E1 | 2.6E1 | 9.6E0 | 1.0E-9 | 1.1E0 | 3.5E2 | 5.1E1 | 296 | 61 | 296 | 61 | 0.70 |
| Mp | pg/ml | 1.0E-9 | 6.6E0 | 1.0E1 | 7.6E1 | 4.1E1 | 3.1E2 | 1.0E-9 | 1.0E-9 | 6.3E2 | 2.4E3 | 296 | 61 | 296 | 61 | 0.63 |
| Mq | pg/ml | 1.0E-9 | 1.0E-9 | 2.2E0 | 5.5E0 | 1.4E1 | 1.8E1 | 1.0E-9 | 1.0E-9 | 1.8E2 | 1.0E2 | 296 | 61 | 296 | 61 | 0.54 |
| Mr | pg/ml | 1.0E-9 | 1.0E-9 | 1.6E1 | 1.7E2 | 8.9E1 | 5.7E2 | 1.0E-9 | 1.0E-9 | 1.4E3 | 3.4E3 | 296 | 61 | 296 | 61 | 0.60 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ms | pg/ml | 3.4E2 | 2.5E2 | 4.9E2 | 4.1E2 | 5.6E2 | 6.5E2 | 1.0E-9 | 1.0E-9 | 3.3E3 | 4.7E3 | 296 | 61 | 296 | 61 | 0.44 |
| Mt | pg/ml | 1.0E-9 | 1.6E0 | 7.3E0 | 7.8E1 | 4.5E1 | 4.1E2 | 1.0E-9 | 1.0E-9 | 7.1E2 | 3.2E3 | 296 | 61 | 296 | 61 | 0.67 |
| Mu | pg/ml | 1.0E-9 | 1.0E-9 | 1.6E0 | 2.3E0 | 1.5E1 | 8.0E0 | 1.0E-9 | 1.0E-9 | 2.3E2 | 4.8E1 | 296 | 61 | 296 | 61 | 0.55 |
| Mv | pg/ml | 1.0E-9 | 1.0E-9 | 6.4E1 | 1.0E2 | 3.6E2 | 2.4E2 | 1.0E-9 | 1.0E-9 | 4.5E3 | 9.2E2 | 296 | 61 | 296 | 61 | 0.57 |
| Mw | pg/ml | 3.0E1 | 9.8E1 | 2.7E2 | 4.8E2 | 1.4E3 | 1.1E3 | 1.0E-9 | 1.0E-9 | 1.8E4 | 5.3E3 | 296 | 61 | 296 | 61 | 0.66 |
| Mx | pg/ml | 1.0E-9 | 1.0E-9 | 3.4E-1 | 8.7E-1 | 2.0E0 | 2.8E0 | 1.0E-9 | 1.0E-9 | 3.2E1 | 2.0E1 | 296 | 61 | 296 | 61 | 0.58 |
| My | pg/ml | 1.0E-9 | 1.0E-9 | 3.6E2 | 1.6E2 | 2.6E3 | 3.9E2 | 1.0E-9 | 1.0E-9 | 3.9E4 | 1.9E3 | 296 | 61 | 296 | 61 | 0.56 |
| Mz | pg/ml | 9.9E0 | 3.0E1 | 2.3E1 | 1.0E2 | 4.9E1 | 2.9E2 | 1.0E-9 | 1.0E-9 | 5.5E2 | 1.9E3 | 296 | 61 | 296 | 61 | 0.74 |
| Na | pg/ml | 1.0E-9 | 1.0E-9 | 6.8E-1 | 1.3E0 | 1.9E0 | 5.6E0 | 1.0E-9 | 1.0E-9 | 9.6E0 | 4.2E1 | 296 | 61 | 296 | 61 | 0.49 |
| Nb | pg/ml | 2.1E0 | 3.3E0 | 3.5E0 | 1.0E1 | 1.1E1 | 3.0E1 | 1.0E-9 | 1.0E-9 | 1.5E2 | 2.1E2 | 296 | 61 | 296 | 61 | 0.61 |
| Nc | pg/ml | 3.5E2 | 1.0E2 | 5.3E2 | 3.8E2 | 7.5E2 | 5.8E2 | 1.0E-9 | 1.0E-9 | 9.2E3 | 3.2E3 | 296 | 61 | 296 | 61 | 0.41 |
| Nd | pg/ml | 2.8E1 | 2.5E1 | 2.8E1 | 6.3E1 | 7.2E1 | 2.7E2 | 1.0E-9 | 1.0E-9 | 1.2E3 | 2.1E3 | 296 | 61 | 296 | 61 | 0.54 |
| Ne | pg/ml | 4.4E2 | 3.5E2 | 5.4E2 | 4.3E2 | 5.7E2 | 5.3E2 | 1.0E-9 | 1.0E-9 | 7.0E3 | 3.6E3 | 296 | 61 | 296 | 61 | 0.42 |
| Nf | pg/ml | 1.0E-9 | 1.0E-9 | 2.4E0 | 5.9E0 | 8.7E0 | 2.3E1 | 1.0E-9 | 1.0E-9 | 8.2E1 | 1.3E2 | 296 | 61 | 296 | 61 | 0.49 |
| Ng | pg/ml | 1.2E1 | 9.6E0 | 9.3E1 | 7.7E1 | 1.9E2 | 1.2E2 | 1.0E-9 | 1.0E-9 | 1.6E3 | 5.3E2 | 296 | 61 | 296 | 61 | 0.53 |
| Nh | pg/ml | 6.3E1 | 5.2E1 | 8.3E1 | 6.1E1 | 7.6E1 | 7.2E1 | 1.0E-9 | 4.1E0 | 5.6E2 | 5.1E2 | 296 | 61 | 296 | 61 | 0.39 |
| Ni | pg/ml | 9.4E0 | 1.0E-9 | 8.2E1 | 1.0E2 | 1.3E2 | 1.9E2 | 1.0E-9 | 1.0E-9 | 7.1E2 | 1.1E3 | 296 | 61 | 296 | 61 | 0.48 |
| Nj | pg/ml | 7.4E0 | 6.2E0 | 1.1E1 | 8.3E0 | 1.2E1 | 9.5E0 | 1.0E-9 | 1.0E-9 | 6.9E1 | 4.6E1 | 296 | 61 | 296 | 61 | 0.40 |
| Nk | pg/ml | 1.8E1 | 1.4E1 | 3.2E1 | 3.4E1 | 3.7E1 | 4.8E1 | 1.0E-9 | 1.0E-9 | 2.0E2 | 2.0E2 | 296 | 61 | 296 | 61 | 0.48 |
| Nl | pg/ml | 4.4E1 | 3.3E1 | 5.9E1 | 4.2E1 | 7.9E1 | 4.8E1 | 1.0E-9 | 1.0E-9 | 1.1E3 | 2.3E2 | 296 | 61 | 296 | 61 | 0.40 |
| Hl | pg/ml | 4.6E0 | 1.1E1 | 3.3E1 | 4.2E2 | 5.6E1 | 1.2E3 | 1.0E-9 | 1.0E-9 | 3.0E2 | 3.6E3 | 49 | 9 | 49 | 9 | 0.56 |
| Ho | pg/ml | 1.7E1 | 2.7E1 | 2.2E1 | 9.0E1 | 2.0E1 | 1.3E2 | 1.0E-9 | 7.6E0 | 8.7E1 | 3.9E2 | 49 | 9 | 49 | 9 | 0.68 |
| Hp | ng/ml | 1.6E0 | 4.0E0 | 9.4E1 | 3.0E2 | 2.7E2 | 4.4E2 | 1.0E-9 | 8.8E-1 | 8.9E2 | 8.9E2 | 49 | 9 | 49 | 9 | 0.68 |
| Tz | pg/ml | 4.0E3 | 6.5E3 | 6.7E3 | 1.1E5 | 8.5E3 | 4.2E5 | 1.0E-9 | 6.3E2 | 5.3E4 | 2.1E6 | 111 | 24 | 111 | 24 | 0.61 |
| Ua | pg/ml | 3.2E3 | 5.6E3 | 3.3E4 | 1.4E4 | 2.0E5 | 2.3E4 | 1.0E-9 | 9.1E2 | 2.1E6 | 9.9E4 | 111 | 24 | 111 | 24 | 0.59 |
| Ub | pg/ml | 5.8E2 | 4.0E2 | 9.0E2 | 5.9E2 | 1.2E3 | 8.3E2 | 1.0E-9 | 2.3E0 | 9.8E3 | 4.1E3 | 111 | 24 | 111 | 24 | 0.38 |
| Ue | pg/ml | 3.1E1 | 2.3E1 | 3.7E1 | 3.5E1 | 3.2E1 | 3.7E1 | 9.8E-2 | 4.5E0 | 2.7E2 | 1.4E2 | 111 | 24 | 111 | 24 | 0.43 |
| Uc | pg/ml | 7.0E2 | 8.7E2 | 1.1E3 | 4.5E3 | 1.3E3 | 1.2E4 | 1.0E-9 | 4.1E1 | 8.3E3 | 5.7E4 | 111 | 24 | 111 | 24 | 0.60 |
| Ud | pg/ml | 1.0E-9 | 1.0E-9 | 3.5E0 | 2.2E0 | 3.7E1 | 1.1E1 | 1.0E-9 | 1.0E-9 | 3.9E2 | 5.3E1 | 111 | 24 | 111 | 24 | 0.52 |
| Hq | pg/ml | 1.2E0 | 1.1E0 | 1.7E2 | 6.2E1 | 2.0E3 | 3.6E2 | 1.0E-9 | 1.0E-9 | 2.8E4 | 2.8E3 | 294 | 61 | 294 | 61 | 0.53 |
| Hr | pg/ml | 9.4E1 | 7.9E1 | 6.9E2 | 4.7E2 | 1.4E3 | 1.3E3 | 1.0E-9 | 1.0E-9 | 1.2E4 | 8.9E3 | 294 | 61 | 294 | 61 | 0.43 |
| Hu | pg/ml | 3.8E0 | 6.3E1 | 5.3E3 | 6.6E2 | 4.3E4 | 1.4E3 | 1.0E-9 | 1.0E-9 | 6.3E5 | 6.5E3 | 294 | 61 | 294 | 61 | 0.59 |
| Hv | pg/ml | 1.5E0 | 1.0E0 | 2.5E0 | 2.0E1 | 5.8E0 | 1.2E2 | 1.0E-9 | 1.0E-9 | 6.9E1 | 8.9E2 | 294 | 61 | 294 | 61 | 0.51 |
| Hw | pg/ml | 6.4E0 | 5.7E0 | 1.5E1 | 1.8E2 | 4.7E1 | 1.2E3 | 1.0E-9 | 4.6E-1 | 6.4E2 | 9.4E3 | 294 | 61 | 294 | 61 | 0.49 |
| Hx | pg/ml | 8.6E0 | 1.2E1 | 5.7E1 | 6.3E1 | 5.4E2 | 1.9E2 | 1.0E-9 | 1.0E-9 | 9.3E3 | 1.3E3 | 294 | 61 | 294 | 61 | 0.59 |
| Ib | ng/ml | 3.9E-2 | 2.8E-2 | 1.2E0 | 2.5E0 | 4.9E0 | 1.1E1 | 1.0E-9 | 1.0E-9 | 3.6E1 | 5.6E1 | 109 | 24 | 109 | 24 | 0.50 |
| Ic | U/ml | 2.2E2 | 2.2E2 | 3.7E2 | 2.9E3 | 9.6E2 | 1.3E4 | 2.4E0 | 2.5E1 | 8.6E3 | 6.5E4 | 109 | 24 | 109 | 24 | 0.60 |
| Id | U/ml | 5.5E-1 | 1.2E0 | 1.1E0 | 2.1E1 | 1.5E0 | 8.8E1 | 1.0E-9 | 2.7E-1 | 1.0E1 | 4.3E2 | 109 | 24 | 109 | 24 | 0.69 |
| Tt | pg/ml | 1.7E2 | 1.8E2 | 1.7E2 | 1.9E2 | 5.4E1 | 7.2E1 | 4.3E1 | 1.1E2 | 3.6E2 | 4.4E2 | 103 | 21 | 103 | 21 | 0.57 |
| To | pg/ml | 1.6E0 | 1.8E0 | 2.1E0 | 2.3E0 | 2.9E0 | 2.7E0 | 1.0E-9 | 1.0E-9 | 2.3E1 | 1.2E1 | 109 | 22 | 109 | 22 | 0.54 |
| Tr | pg/ml | 3.2E0 | 5.2E0 | 7.8E0 | 1.4E1 | 3.0E1 | 2.0E1 | 1.0E-9 | 3.9E-1 | 3.1E2 | 7.6E1 | 107 | 21 | 107 | 21 | 0.63 |
| Tn | pg/ml | 3.0E1 | 5.4E1 | 1.0E2 | 2.9E2 | 2.9E2 | 6.1E2 | 1.0E-9 | 1.3E1 | 1.8E3 | 2.3E3 | 109 | 22 | 109 | 22 | 0.67 |
| Tv | ng/ml | 1.2E1 | 1.1E1 | 1.7E1 | 4.1E2 | 1.8E1 | 1.5E3 | 1.0E-9 | 1.0E-9 | 1.1E2 | 7.1E3 | 109 | 22 | 109 | 22 | 0.52 |
| Ih | ng/ml | 5.9E1 | 1.9E2 | 2.1E2 | 5.2E2 | 3.8E2 | 7.4E2 | 1.0E-9 | 1.0E-9 | 2.5E3 | 3.6E3 | 295 | 61 | 295 | 61 | 0.64 |
| Ii | ng/ml | 7.5E1 | 1.1E2 | 2.0E2 | 3.4E2 | 4.9E2 | 7.6E2 | 7.3E-1 | 7.5E-1 | 5.2E3 | 4.5E3 | 295 | 61 | 295 | 61 | 0.60 |
| Ij | ng/ml | 7.2E1 | 1.4E2 | 1.8E2 | 6.4E2 | 6.0E2 | 3.1E3 | 2.8E0 | 9.5E0 | 6.4E3 | 2.4E4 | 292 | 60 | 292 | 60 | 0.72 |
| Ik | ng/ml | 1.1E1 | 1.4E1 | 1.5E3 | 1.9E2 | 1.2E4 | 3.9E2 | 5.9E-1 | 6.2E-1 | 1.2E5 | 1.5E3 | 292 | 60 | 292 | 60 | 0.53 |
| Il | ng/ml | 3.3E2 | 5.2E2 | 1.2E3 | 2.1E3 | 2.8E3 | 3.7E3 | 1.0E-9 | 1.0E-9 | 1.2E4 | 1.2E4 | 290 | 60 | 290 | 60 | 0.57 |
| Im | ng/ml | 2.0E2 | 7.0E2 | 3.4E2 | 1.3E3 | 5.2E2 | 2.3E3 | 1.4E1 | 2.2E1 | 6.0E3 | 1.5E4 | 291 | 61 | 291 | 61 | 0.75 |
| In | ng/ml | 3.8E0 | 2.2E0 | 2.1E1 | 9.8E1 | 9.4E1 | 5.8E2 | 1.0E-9 | 1.0E-9 | 1.1E3 | 4.5E3 | 295 | 61 | 295 | 61 | 0.46 |
| Hb | ng/ml | 2.1E1 | 4.3E1 | 3.1E1 | 5.3E1 | 3.1E1 | 5.0E1 | 4.8E-1 | 4.5E0 | 2.0E2 | 1.9E2 | 111 | 25 | 111 | 25 | 0.66 |
| Hc | pg/ml | 6.9E2 | 5.0E2 | 3.2E3 | 3.2E3 | 1.1E4 | 1.0E4 | 1.0E-9 | 1.0E-9 | 1.0E5 | 5.0E4 | 111 | 25 | 111 | 25 | 0.44 |
| Hf | ng/ml | 1.9E2 | 1.9E2 | 4.2E2 | 2.8E2 | 5.9E2 | 2.8E2 | 1.0E-9 | 1.0E-9 | 3.2E3 | 9.9E2 | 111 | 25 | 111 | 25 | 0.47 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Io | ng/ml | 7.6E3 | 1.8E4 | 1.9E4 | 2.2E4 | 4.9E4 | 2.4E4 | 1.0E-9 | 6.2E2 | 7.1E5 | 1.1E5 | 295 | 61 | 295 | 61 | 0.62 |
| Ip | ng/ml | 8.2E0 | 3.0E1 | 2.0E1 | 3.1E1 | 2.7E1 | 2.2E1 | 1.0E-9 | 1.6E-2 | 2.3E2 | 8.3E1 | 295 | 61 | 295 | 61 | 0.67 |
| Iq | ug/ml | 1.0E-1 | 1.9E-1 | 4.7E1 | 5.5E0 | 7.9E2 | 2.8E1 | 1.0E-9 | 1.0E-9 | 1.4E4 | 2.2E2 | 295 | 61 | 295 | 61 | 0.61 |
| Ir | ug/ml | 3.4E-1 | 1.2E0 | 4.4E0 | 1.5E1 | 3.4E1 | 5.3E1 | 1.0E-9 | 1.0E-9 | 5.1E2 | 3.7E2 | 294 | 61 | 294 | 61 | 0.68 |
| Is | ng/ml | 1.7E0 | 7.6E0 | 7.6E0 | 2.7E1 | 3.4E1 | 4.7E1 | 1.0E-9 | 3.3E-2 | 5.5E2 | 2.6E2 | 295 | 61 | 295 | 61 | 0.68 |
| It | ng/ml | 1.7E0 | 4.3E0 | 2.2E1 | 3.0E1 | 1.1E2 | 1.1E2 | 1.0E-9 | 1.0E-9 | 1.1E3 | 6.8E2 | 295 | 61 | 295 | 61 | 0.65 |
| Iu | ng/ml | 1.7E2 | 1.8E2 | 1.3E3 | 2.2E3 | 4.1E3 | 5.5E3 | 1.0E-9 | 1.0E-9 | 2.9E4 | 2.4E4 | 295 | 61 | 295 | 61 | 0.54 |
| Iv | ng/ml | 1.1E1 | 2.3E1 | 8.9E1 | 2.5E2 | 9.3E2 | 8.2E2 | 1.0E-9 | 1.0E-9 | 1.6E4 | 3.8E3 | 294 | 61 | 294 | 61 | 0.63 |
| Iz | ng/ml | 1.2E2 | 1.9E2 | 3.8E2 | 3.1E2 | 7.7E2 | 3.9E2 | 1.5E0 | 8.8E-1 | 6.1E3 | 1.7E3 | 111 | 25 | 111 | 25 | 0.53 |
| Yg | pg/ml | 2.4E2 | 1.7E3 | 1.7E3 | 1.6E3 | 7.4E3 | 1.3E3 | 1.0E-9 | 1.3E2 | 5.0E4 | 3.9E3 | 46 | 8 | 46 | 8 | 0.73 |
| Yh | pg/ml | 2.1E2 | 5.7E2 | 4.2E2 | 6.4E2 | 6.0E2 | 5.9E2 | 1.0E-9 | 1.0E-9 | 3.0E3 | 1.4E3 | 46 | 8 | 46 | 8 | 0.60 |
| Yi | pg/ml | 2.6E2 | 1.4E3 | 4.6E2 | 5.3E3 | 4.8E2 | 9.0E3 | 1.0E-9 | 2.4E2 | 2.0E3 | 2.6E4 | 46 | 8 | 46 | 8 | 0.85 |
| Yk | U/ml | 1.0E-9 | 1.0E-9 | 2.1E-1 | 3.3E-1 | 6.1E-1 | 6.0E-1 | 1.0E-9 | 1.0E-9 | 3.0E0 | 1.4E0 | 46 | 8 | 46 | 8 | 0.53 |
| Yj | pg/ml | 1.5E2 | 9.4E1 | 3.2E2 | 1.3E2 | 5.3E2 | 1.0E2 | 1.0E-9 | 5.9E1 | 3.2E3 | 3.7E2 | 46 | 8 | 46 | 8 | 0.37 |
| Yd | ng/ml | 2.2E-1 | 1.9E-1 | 3.6E-1 | 6.2E-1 | 4.2E-1 | 8.8E-1 | 6.6E-3 | 1.7E-2 | 1.8E0 | 2.3E0 | 49 | 9 | 49 | 9 | 0.52 |
| Wb | pg/ml | 2.7E4 | 3.5E4 | 3.3E4 | 1.2E5 | 1.9E4 | 2.0E5 | 4.9E3 | 1.4E4 | 8.4E4 | 6.4E5 | 49 | 9 | 49 | 9 | 0.70 |
| Vz | pg/ml | 3.5E0 | 5.0E0 | 4.4E0 | 6.0E0 | 4.1E0 | 6.7E0 | 1.0E-9 | 7.6E-2 | 2.1E1 | 2.2E1 | 49 | 9 | 49 | 9 | 0.56 |
| Si | ng/ml | 1.2E0 | 1.9E0 | 1.8E0 | 2.7E0 | 2.5E0 | 2.1E0 | 8.6E-3 | 5.6E-1 | 1.0E1 | 6.0E0 | 49 | 9 | 49 | 9 | 0.69 |
| Sf | mIU/mL | 1.3E1 | 2.0E1 | 5.0E1 | 2.7E1 | 1.1E2 | 2.4E1 | 6.2E-1 | 6.7E0 | 7.2E2 | 8.3E1 | 49 | 9 | 49 | 9 | 0.57 |
| Sh | mIU/mL | 1.6E1 | 1.1E1 | 4.5E1 | 1.6E1 | 9.4E1 | 1.6E1 | 7.8E-2 | 4.2E0 | 5.7E2 | 5.8E1 | 49 | 9 | 49 | 9 | 0.46 |
| Sj | ng/ml | 4.4E-1 | 4.6E-1 | 4.2E-1 | 4.6E-1 | 9.0E-2 | 1.2E-1 | 2.5E-1 | 3.4E-1 | 6.6E-1 | 7.2E-1 | 49 | 9 | 49 | 9 | 0.58 |
| Rc | pg/ml | 6.5E3 | 7.6E3 | 7.8E3 | 7.5E3 | 6.2E3 | 3.8E3 | 3.9E2 | 1.1E3 | 3.9E4 | 1.5E4 | 111 | 24 | 111 | 24 | 0.54 |
| Rb | pg/ml | 7.5E-1 | 7.5E-1 | 2.7E0 | 4.2E0 | 4.2E0 | 1.1E1 | 1.0E-9 | 1.0E-9 | 2.0E1 | 5.6E1 | 111 | 24 | 111 | 24 | 0.53 |
| Zq | 2.6ng/ml | 2.4E2 | 4.0E2 | 2.8E2 | 4.7E2 | 2.1E2 | 2.1E2 | 1.4E1 | 3.0E2 | 9.7E2 | 9.7E2 | 48 | 8 | 48 | 8 | 0.76 |
| Zw | 2.5ng/ml | 4.8E0 | 5.3E0 | 8.6E0 | 2.0E1 | 1.1E1 | 2.5E1 | 1.4E-1 | 7.7E-1 | 5.9E1 | 6.3E1 | 49 | 9 | 49 | 9 | 0.60 |
| Zx | 2.3mU/ml | 1.2E-1 | 1.8E-1 | 2.7E-1 | 4.0E-1 | 5.0E-1 | 5.8E-1 | 3.2E-2 | 7.7E-2 | 2.9E0 | 1.9E0 | 49 | 9 | 49 | 9 | 0.66 |
| Pz | ng/ml | 3.2E3 | 1.0E4 | 5.4E3 | 6.9E3 | 6.1E3 | 4.7E3 | 1.6E1 | 4.0E1 | 7.0E4 | 2.5E4 | 291 | 61 | 291 | 61 | 0.62 |
| Qa | ng/ml | 3.3E3 | 9.0E3 | 6.2E3 | 1.5E4 | 7.4E3 | 2.9E4 | 1.5E2 | 2.9E2 | 4.2E4 | 2.2E5 | 291 | 61 | 291 | 61 | 0.68 |
| Qb | ng/ml | 1.0E2 | 2.0E2 | 2.1E2 | 3.8E2 | 4.0E2 | 5.8E2 | 7.9E-1 | 8.7E0 | 5.3E3 | 4.1E3 | 291 | 61 | 291 | 61 | 0.64 |
| Qc | ng/ml | 1.9E2 | 4.8E2 | 4.1E2 | 6.8E2 | 5.5E2 | 7.5E2 | 1.0E-9 | 1.0E-9 | 3.8E3 | 4.3E3 | 291 | 61 | 291 | 61 | 0.63 |
| Qd | ng/ml | 8.2E3 | 2.0E4 | 2.3E4 | 5.3E4 | 1.2E5 | 7.9E4 | 1.5E2 | 1.7E3 | 2.0E6 | 4.3E5 | 291 | 61 | 291 | 61 | 0.70 |
| Qe | ng/ml | 7.7E2 | 2.3E3 | 1.9E3 | 3.2E3 | 6.0E3 | 3.3E3 | 1.0E-9 | 8.8E0 | 9.7E4 | 1.8E4 | 291 | 61 | 291 | 61 | 0.68 |
| Jd | ng/ml | 8.4E-1 | 1.4E0 | 4.1E0 | 3.7E0 | 1.6E1 | 5.4E0 | 1.0E-9 | 1.0E-9 | 1.2E2 | 2.1E1 | 111 | 24 | 111 | 24 | 0.62 |
| Je | ng/ml | 1.0E-9 | 5.0E-1 | 1.8E0 | 1.8E0 | 5.2E0 | 2.8E0 | 1.0E-9 | 1.0E-9 | 4.6E1 | 1.1E1 | 111 | 24 | 111 | 24 | 0.57 |
| Jf | ng/ml | 1.0E-9 | 1.0E-9 | 1.3E0 | 7.6E-1 | 2.4E0 | 1.3E0 | 1.0E-9 | 1.0E-9 | 1.2E1 | 3.9E0 | 111 | 24 | 111 | 24 | 0.47 |
| Jg | ng/ml | 3.8E2 | 1.2E3 | 7.1E2 | 1.3E3 | 9.4E2 | 1.2E3 | 5.8E0 | 4.5E1 | 1.0E4 | 7.1E3 | 294 | 61 | 294 | 61 | 0.70 |
| Jh | ng/ml | 2.6E0 | 7.5E0 | 2.1E1 | 3.6E1 | 8.7E1 | 8.1E1 | 1.0E-9 | 1.0E-9 | 1.2E3 | 4.9E2 | 294 | 61 | 294 | 61 | 0.63 |
| Ji | ng/ml | 5.1E1 | 1.3E2 | 7.3E1 | 2.1E2 | 7.6E1 | 2.2E2 | 1.1E0 | 2.0E1 | 5.3E2 | 1.3E3 | 294 | 61 | 294 | 61 | 0.77 |
| Sr | pg/mL | 3.4E2 | 1.0E3 | 7.6E2 | 2.4E3 | 1.1E3 | 4.3E3 | 1.0E-9 | 9.2E1 | 4.8E3 | 2.1E4 | 110 | 24 | 110 | 24 | 0.70 |
| Ss | pg/mL | 9.3E4 | 6.8E4 | 1.5E5 | 1.2E5 | 1.9E5 | 1.3E5 | 2.7E3 | 7.8E3 | 1.3E6 | 5.7E5 | 110 | 24 | 110 | 24 | 0.46 |
| St | pg/mL | 2.3E7 | 8.2E7 | 5.6E7 | 1.5E8 | 1.3E8 | 3.4E8 | 1.0E-9 | 2.3E6 | 1.2E9 | 1.7E9 | 109 | 24 | 109 | 24 | 0.68 |
| Wc | ng/ml | 1.0E-9 | 1.0E-9 | 5.8E-2 | 2.8E-1 | 1.0E-1 | 6.0E-1 | 1.0E-9 | 1.0E-9 | 5.2E-1 | 1.8E0 | 49 | 9 | 49 | 9 | 0.52 |
| Wd | ng/ml | 9.3E0 | 1.8E1 | 2.7E1 | 1.0E2 | 5.9E1 | 1.7E2 | 1.0E0 | 3.5E0 | 2.9E2 | 4.1E2 | 49 | 9 | 49 | 9 | 0.71 |
| We | ng/ml | 2.9E-1 | 5.8E-1 | 1.0E0 | 3.5E0 | 1.7E0 | 7.4E0 | 1.0E-9 | 1.5E-1 | 9.7E0 | 2.3E1 | 49 | 9 | 49 | 9 | 0.66 |
| Wg | ng/ml | 1.0E-9 | 1.0E-9 | 3.3E-4 | 5.9E-2 | 2.3E-3 | 1.8E-1 | 1.0E-9 | 1.0E-9 | 1.6E-2 | 5.3E-1 | 49 | 9 | 49 | 9 | 0.55 |
| Wh | ng/ml | 9.7E-3 | 2.0E-2 | 4.4E-2 | 6.3E-2 | 9.8E-2 | 1.1E-1 | 1.0E-9 | 1.0E-9 | 4.2E-1 | 3.4E-1 | 49 | 9 | 49 | 9 | 0.64 |
| Wf | ng/ml | 1.0E-9 | 1.0E-9 | 9.4E-2 | 3.1E-1 | 2.1E-1 | 7.6E-1 | 1.0E-9 | 1.0E-9 | 1.2E0 | 2.3E0 | 49 | 9 | 49 | 9 | 0.45 |
| Ra | pg/ml | 1.0E-9 | 1.0E-9 | 4.1E-1 | 3.1E0 | 9.7E-1 | 1.3E1 | 1.0E-9 | 1.0E-9 | 3.8E0 | 6.4E1 | 111 | 24 | 111 | 24 | 0.52 |
| Qz | pg/ml | 9.8E0 | 1.0E1 | 5.3E1 | 4.5E1 | 8.8E1 | 7.4E1 | 1.0E-9 | 1.0E-9 | 4.5E2 | 2.3E2 | 111 | 24 | 111 | 24 | 0.48 |
| Qy | pg/ml | 3.8E-1 | 5.6E-1 | 8.2E0 | 3.2E1 | 4.7E1 | 1.5E2 | 1.0E-9 | 1.0E-9 | 4.3E2 | 7.3E2 | 111 | 24 | 111 | 24 | 0.58 |
| Qx | pg/ml | 1.0E-9 | 1.0E-9 | 2.9E0 | 7.2E0 | 1.8E1 | 2.3E1 | 1.0E-9 | 1.0E-9 | 1.9E2 | 1.1E2 | 111 | 24 | 111 | 24 | 0.55 |
| Qw | pg/ml | 1.0E-9 | 1.0E-9 | 3.0E0 | 1.6E0 | 1.0E1 | 4.7E0 | 1.0E-9 | 1.0E-9 | 6.6E1 | 2.3E1 | 111 | 24 | 111 | 24 | 0.47 |
| Qv | pg/ml | 1.9E4 | 1.0E4 | 3.2E4 | 5.3E4 | 4.9E4 | 1.9E5 | 1.0E-9 | 4.0E2 | 3.7E5 | 9.4E5 | 111 | 24 | 111 | 24 | 0.35 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Qu | pg/ml | 7.8E0 | 7.7E0 | 9.1E1 | 9.7E1 | 1.8E2 | 2.0E2 | 1.0E-9 | 1.0E-9 | 9.8E2 | 7.3E2 | 111 | 24 | 111 | 24 | 0.50 |
| Qt | pg/ml | 1.2E1 | 1.4E1 | 4.5E1 | 4.2E1 | 1.0E2 | 6.8E1 | 1.0E-9 | 1.0E-9 | 7.1E2 | 2.2E2 | 111 | 24 | 111 | 24 | 0.52 |
| Qh | ng/ml | 1.8E1 | 3.6E1 | 3.9E1 | 1.0E2 | 6.1E1 | 1.8E2 | 2.5E-1 | 3.3E0 | 3.4E2 | 8.0E2 | 111 | 24 | 111 | 24 | 0.64 |
| Qg | ng/ml | 7.6E0 | 5.1E0 | 1.4E1 | 1.0E1 | 2.7E1 | 1.7E1 | 1.5E-1 | 3.3E-1 | 2.7E2 | 8.1E1 | 111 | 24 | 111 | 24 | 0.42 |
| Jj | ng/ml | 5.7E2 | 2.5E2 | 2.1E3 | 4.8E2 | 2.0E4 | 4.8E2 | 2.3E0 | 8.7E0 | 3.4E5 | 1.9E3 | 294 | 61 | 294 | 61 | 0.36 |
| Jk | ng/ml | 2.6E0 | 4.7E0 | 2.0E1 | 3.0E1 | 4.9E1 | 5.2E1 | 1.0E-9 | 1.2E-1 | 3.9E2 | 2.4E2 | 294 | 61 | 294 | 61 | 0.59 |
| Jl | ng/ml | 4.6E-1 | 8.8E-1 | 1.6E0 | 1.7E2 | 3.7E0 | 1.3E3 | 1.2E-3 | 7.8E-2 | 2.0E1 | 9.9E3 | 294 | 61 | 294 | 61 | 0.65 |
| Jm | ng/ml | 1.8E1 | 3.5E1 | 6.2E1 | 9.2E1 | 1.4E2 | 2.7E2 | 1.0E-9 | 4.0E-1 | 1.4E3 | 2.1E3 | 294 | 61 | 294 | 61 | 0.57 |
| Jn | pg/ml | 3.1E-1 | 6.5E-1 | 3.5E0 | 2.8E1 | 3.7E1 | 1.2E2 | 1.0E-9 | 1.0E-9 | 6.2E2 | 7.3E2 | 294 | 61 | 294 | 61 | 0.66 |
| Jo | pg/ml | 3.6E3 | 4.7E3 | 4.7E3 | 7.6E3 | 3.9E3 | 1.4E4 | 2.0E1 | 2.4E1 | 2.4E4 | 1.0E5 | 294 | 61 | 294 | 61 | 0.54 |
| Jp | pg/ml | 6.9E4 | 9.2E4 | 7.0E4 | 1.0E5 | 3.5E4 | 5.2E4 | 5.8E2 | 2.8E4 | 1.9E5 | 3.8E5 | 294 | 61 | 294 | 61 | 0.71 |
| Jq | pg/ml | 9.2E1 | 1.5E2 | 1.5E2 | 4.6E2 | 2.0E2 | 1.2E3 | 1.0E0 | 5.6E0 | 2.0E3 | 8.7E3 | 294 | 61 | 294 | 61 | 0.61 |
| Jr | pg/ml | 2.8E0 | 1.3E1 | 5.7E1 | 2.7E2 | 6.3E2 | 1.2E3 | 1.0E-9 | 1.0E-9 | 1.1E4 | 7.4E3 | 294 | 61 | 294 | 61 | 0.67 |
| Js | pg/ml | 1.4E1 | 2.0E1 | 6.8E1 | 2.0E2 | 5.9E2 | 6.4E2 | 1.0E-9 | 2.7E0 | 1.0E4 | 3.0E3 | 294 | 61 | 294 | 61 | 0.68 |
| Jt | pg/ml | 2.3E3 | 3.3E3 | 2.8E3 | 5.9E3 | 2.2E3 | 8.7E3 | 2.2E1 | 1.5E2 | 2.2E4 | 5.2E4 | 294 | 61 | 294 | 61 | 0.66 |
| Xa | pg/ml | 1.0E-9 | 2.4E1 | 8.4E0 | 2.2E2 | 1.7E1 | 4.2E2 | 1.0E-9 | 2.9E0 | 9.6E1 | 1.2E3 | 49 | 9 | 49 | 9 | 0.82 |
| Ye | pg/ml | 1.0E-9 | 1.0E-9 | 4.7E0 | 8.0E-1 | 1.4E1 | 2.4E0 | 1.0E-9 | 1.0E-9 | 6.9E1 | 7.2E0 | 49 | 9 | 49 | 9 | 0.41 |
| Tm | pg/ml | 1.0E-9 | 1.0E-9 | 2.9E0 | 9.3E-1 | 4.6E0 | 2.8E0 | 1.0E-9 | 1.0E-9 | 1.8E1 | 8.4E0 | 49 | 9 | 49 | 9 | 0.40 |
| Tl | pg/ml | 1.1E-1 | 1.0E-9 | 3.1E-1 | 2.9E0 | 4.0E-1 | 8.2E0 | 1.0E-9 | 1.0E-9 | 1.8E0 | 2.5E1 | 49 | 9 | 49 | 9 | 0.45 |
| Ju | mIU/ml | 9.1E0 | 1.6E1 | 2.5E1 | 2.2E1 | 3.7E1 | 2.3E1 | 1.7E-1 | 1.9E0 | 2.3E2 | 1.0E2 | 111 | 24 | 111 | 24 | 0.60 |
| Jv | mIU/ml | 1.6E1 | 1.3E1 | 4.1E1 | 2.7E1 | 6.6E1 | 4.0E1 | 1.7E-2 | 1.1E0 | 4.4E2 | 1.8E2 | 111 | 24 | 111 | 24 | 0.49 |
| Jy | ng/ml | 1.6E-3 | 1.9E-3 | 2.2E-3 | 4.7E-3 | 3.7E-3 | 8.9E-3 | 1.0E-9 | 4.5E-4 | 3.9E-2 | 4.1E-2 | 111 | 24 | 111 | 24 | 0.56 |
| Kc | pg/ml | 2.2E1 | 4.6E1 | 3.7E1 | 8.2E1 | 4.4E1 | 8.4E1 | 1.0E-9 | 1.0E-9 | 1.9E2 | 3.2E2 | 111 | 25 | 111 | 25 | 0.67 |
| Kd | pg/ml | 1.0E-9 | 1.0E-9 | 1.6E2 | 2.2E3 | 5.8E2 | 7.7E3 | 1.0E-9 | 1.0E-9 | 4.8E3 | 3.8E4 | 111 | 25 | 111 | 25 | 0.58 |
| Ke | pg/ml | 1.2E4 | 1.9E4 | 1.4E4 | 4.1E4 | 9.2E3 | 6.3E4 | 6.7E2 | 3.5E3 | 5.5E4 | 3.2E5 | 111 | 25 | 111 | 25 | 0.73 |
| Kf | pg/mL | 5.7E0 | 8.9E0 | 6.3E0 | 1.3E1 | 5.0E0 | 1.6E1 | 1.0E-9 | 1.0E-9 | 2.2E1 | 7.8E1 | 111 | 25 | 111 | 25 | 0.67 |
| Kg | pg/mL | 9.3E2 | 1.0E3 | 1.8E3 | 4.0E3 | 2.6E3 | 8.6E3 | 7.7E1 | 1.3E2 | 2.2E4 | 3.6E4 | 111 | 25 | 111 | 25 | 0.52 |
| Ki | pg/ml | 5.9E1 | 6.4E1 | 7.0E1 | 7.8E1 | 5.3E1 | 5.3E1 | 1.0E-9 | 1.0E-9 | 2.9E2 | 2.5E2 | 111 | 25 | 111 | 25 | 0.56 |
| Kj | pg/ml | 8.8E2 | 7.3E2 | 1.4E3 | 1.7E3 | 1.4E3 | 3.1E3 | 6.6E1 | 3.3E1 | 8.8E3 | 1.5E4 | 111 | 25 | 111 | 25 | 0.45 |
| Kk | pg/ml | 6.8E0 | 1.5E1 | 1.2E1 | 2.5E1 | 1.4E1 | 2.0E1 | 1.0E-9 | 2.0E0 | 8.1E1 | 5.9E1 | 111 | 25 | 111 | 25 | 0.73 |
| Kl | pg/ml | 1.8E4 | 1.8E4 | 2.8E4 | 2.7E4 | 2.7E4 | 2.1E4 | 2.3E2 | 6.2E2 | 1.3E5 | 6.3E4 | 111 | 25 | 111 | 25 | 0.52 |
| Kn | pg/ml | 2.9E1 | 6.3E1 | 5.2E1 | 3.3E2 | 7.4E1 | 9.6E2 | 1.0E-9 | 1.0E-9 | 3.6E2 | 4.9E3 | 111 | 25 | 111 | 25 | 0.67 |
| Ko | pg/ml | 3.1E2 | 6.5E2 | 4.3E2 | 9.2E2 | 4.7E2 | 9.9E2 | 1.0E-9 | 1.5E2 | 2.2E3 | 4.1E3 | 111 | 25 | 111 | 25 | 0.72 |
| Kp | pg/ml | 3.4E2 | 4.4E2 | 3.5E2 | 9.8E2 | 2.4E2 | 2.6E3 | 1.0E-9 | 3.7E1 | 9.4E2 | 1.3E4 | 111 | 25 | 111 | 25 | 0.63 |
| Kq | pg/ml | 3.1E2 | 5.7E2 | 4.0E2 | 7.7E3 | 3.8E2 | 3.2E4 | 1.6E0 | 7.0E1 | 2.1E3 | 1.6E5 | 106 | 25 | 106 | 25 | 0.72 |
| Kr | pg/ml | 1.6E-1 | 2.1E0 | 1.8E0 | 2.0E1 | 3.5E0 | 8.3E1 | 1.0E-9 | 1.0E-9 | 2.3E1 | 4.2E2 | 106 | 25 | 106 | 25 | 0.61 |
| Ks | pg/ml | 1.4E4 | 1.7E4 | 2.0E4 | 2.2E4 | 1.8E4 | 1.8E4 | 5.1E1 | 4.2E2 | 7.9E4 | 5.1E4 | 106 | 25 | 106 | 25 | 0.53 |
| Ps | ng/ml | 1.2E2 | 1.2E3 | 4.2E2 | 3.2E3 | 1.3E3 | 4.1E3 | 1.6E0 | 3.5E2 | 9.0E3 | 1.2E4 | 49 | 9 | 49 | 9 | 0.93 |
| Kx | ng/ml | 1.0E-9 | 6.9E-3 | 6.7E-3 | 1.6E-2 | 1.5E-2 | 2.1E-2 | 1.0E-9 | 1.0E-9 | 1.0E-1 | 6.5E-2 | 110 | 25 | 110 | 25 | 0.69 |
| Ky | ng/ml | 1.1E-1 | 4.6E-1 | 3.6E-1 | 6.1E-1 | 7.8E-1 | 6.5E-1 | 1.0E-9 | 1.0E-9 | 5.1E0 | 2.7E0 | 110 | 25 | 110 | 25 | 0.71 |
| Kz | ng/ml | 1.0E-9 | 1.0E-9 | 3.7E-3 | 5.1E-3 | 5.8E-3 | 7.6E-3 | 1.0E-9 | 1.0E-9 | 1.6E-2 | 2.5E-2 | 110 | 25 | 110 | 25 | 0.54 |
| Rz | ng/ml | 3.4E-1 | 3.3E-1 | 8.4E-1 | 1.4E0 | 1.2E0 | 2.4E0 | 4.6E-3 | 1.7E-2 | 6.7E0 | 7.5E0 | 49 | 9 | 49 | 9 | 0.56 |
| Ry | ng/ml | 1.6E-2 | 2.3E-2 | 2.4E-2 | 6.5E-2 | 2.6E-2 | 1.1E-1 | 1.0E-9 | 8.5E-3 | 1.2E-1 | 3.5E-1 | 49 | 9 | 49 | 9 | 0.66 |
| Rx | ng/ml | 1.0E-9 | 3.5E-5 | 1.4E-3 | 2.1E-3 | 2.3E-3 | 2.7E-3 | 1.0E-9 | 1.0E-9 | 8.6E-3 | 7.6E-3 | 49 | 9 | 49 | 9 | 0.62 |
| Ld | pg/ml | 1.0E-9 | 4.8E0 | 2.8E0 | 8.4E0 | 7.7E0 | 1.2E1 | 1.0E-9 | 1.0E-9 | 6.9E1 | 5.0E1 | 111 | 25 | 111 | 25 | 0.71 |
| Lh | pg/ml | 1.0E4 | 2.8E4 | 1.9E4 | 5.4E4 | 2.5E4 | 9.3E4 | 1.0E-9 | 1.3E3 | 2.6E5 | 4.8E5 | 295 | 61 | 295 | 61 | 0.68 |
| Li | pg/ml | 3.0E3 | 9.1E3 | 1.5E4 | 5.5E4 | 8.0E4 | 1.4E5 | 1.2E1 | 3.7E1 | 1.3E6 | 9.2E5 | 295 | 61 | 295 | 61 | 0.71 |
| Lj | pg/ml | 2.3E3 | 1.0E4 | 1.8E4 | 3.8E4 | 5.3E4 | 7.5E4 | 1.0E-9 | 8.9E1 | 4.3E5 | 4.1E5 | 295 | 61 | 295 | 61 | 0.67 |
| Lp | pg/ml | 1.1E1 | 9.5E0 | 6.8E1 | 3.5E2 | 1.8E2 | 5.5E2 | 1.0E-9 | 1.0E-9 | 1.1E3 | 1.4E3 | 49 | 9 | 49 | 9 | 0.53 |
| Lt | pg/ml | 1.0E-9 | 1.0E-9 | 1.7E0 | 1.0E-9 | 6.0E0 | 0.0E0 | 1.0E-9 | 1.0E-9 | 2.5E1 | 1.0E-9 | 49 | 9 | 49 | 9 | 0.46 |
| Rv | ng/ml | 5.0E-4 | 5.8E-4 | 1.3E-3 | 2.7E-3 | 2.6E-3 | 4.7E-3 | 1.0E-9 | 1.0E-9 | 1.6E-2 | 1.2E-2 | 49 | 9 | 49 | 9 | 0.52 |
| Ru | ng/ml | 1.0E-9 | 1.0E-9 | 1.1E-2 | 8.4E-2 | 5.3E-2 | 1.5E-1 | 1.0E-9 | 1.0E-9 | 3.1E-1 | 3.5E-1 | 49 | 9 | 49 | 9 | 0.65 |
| Rt | ng/ml | 7.5E-2 | 5.2E-2 | 1.2E-1 | 9.7E-1 | 1.3E-1 | 2.4E0 | 2.2E-3 | 1.3E-3 | 6.3E-1 | 7.4E0 | 49 | 9 | 49 | 9 | 0.53 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Yl | pg/ml | 1.1E1 | 2.6E1 | 1.7E1 | 4.4E1 | 1.7E1 | 6.7E1 | 1.0E-9 | 1.0E-9 | 7.9E1 | 2.2E2 | 49 | 9 | 49 | 9 | 0.65 |
| Rm | ng/ml | 1.8E1 | 5.4E1 | 3.9E1 | 9.6E1 | 6.2E1 | 1.4E2 | 2.2E-1 | 2.3E-1 | 3.4E2 | 6.5E2 | 110 | 24 | 110 | 24 | 0.60 |
| Rh | ng/ml | 1.7E2 | 1.8E2 | 4.8E2 | 9.5E2 | 1.7E3 | 3.5E3 | 7.5E0 | 2.5E1 | 1.7E4 | 1.7E4 | 110 | 24 | 110 | 24 | 0.52 |
| Ri | ng/ml | 4.1E-1 | 1.0E-9 | 4.4E0 | 1.0E0 | 8.6E0 | 3.3E0 | 1.0E-9 | 1.0E-9 | 4.9E1 | 1.6E1 | 110 | 24 | 110 | 24 | 0.32 |
| Rg | ng/ml | 1.0E-9 | 1.0E-9 | 8.6E-2 | 6.3E-2 | 4.6E-1 | 1.7E-1 | 1.0E-9 | 1.0E-9 | 3.3E0 | 6.2E-1 | 110 | 24 | 110 | 24 | 0.57 |
| Rj | ng/ml | 1.0E-9 | 1.0E-9 | 9.9E-1 | 1.2E1 | 2.1E0 | 5.5E1 | 1.0E-9 | 1.0E-9 | 1.1E1 | 2.7E2 | 110 | 24 | 110 | 24 | 0.44 |
| Rf | ng/ml | 3.5E-1 | 6.2E-1 | 7.6E-1 | 2.2E0 | 1.3E0 | 4.2E0 | 2.1E-2 | 5.5E-2 | 9.9E0 | 1.7E1 | 110 | 24 | 110 | 24 | 0.63 |
| Ql | pg/ml | 1.7E0 | 8.6E0 | 1.1E1 | 1.7E1 | 2.3E1 | 2.4E1 | 1.0E-9 | 1.0E-9 | 1.8E2 | 9.3E1 | 111 | 24 | 111 | 24 | 0.60 |
| Qm | pg/ml | 1.4E0 | 1.7E1 | 1.9E1 | 2.7E1 | 3.6E1 | 3.2E1 | 1.0E-9 | 1.0E-9 | 1.9E2 | 1.0E2 | 111 | 24 | 111 | 24 | 0.60 |
| Qn | pg/ml | 6.1E-1 | 8.5E-1 | 6.9E0 | 7.4E0 | 2.8E1 | 1.9E1 | 1.0E-9 | 1.0E-9 | 2.2E2 | 7.5E1 | 111 | 24 | 111 | 24 | 0.53 |
| Nv | pg/ml | 3.2E3 | 8.3E3 | 8.2E3 | 2.1E4 | 1.7E4 | 3.2E4 | 1.0E-9 | 1.6E2 | 1.5E5 | 1.6E5 | 296 | 61 | 296 | 61 | 0.71 |
| Nw | pg/ml | 8.3E3 | 1.9E4 | 1.2E4 | 2.9E4 | 1.6E4 | 3.8E4 | 1.9E2 | 4.5E3 | 2.1E5 | 2.2E5 | 296 | 61 | 296 | 61 | 0.79 |
| Nx | pg/ml | 2.1E2 | 5.4E2 | 3.9E2 | 7.6E2 | 5.9E2 | 8.0E2 | 1.0E-9 | 1.0E-9 | 3.5E3 | 4.1E3 | 296 | 61 | 296 | 61 | 0.65 |
| Ny | pg/ml | 5.6E0 | 2.0E1 | 1.0E2 | 1.3E2 | 1.4E3 | 3.9E2 | 1.0E-9 | 1.0E-9 | 2.5E4 | 2.8E3 | 296 | 61 | 296 | 61 | 0.73 |
| Oa | pg/ml | 1.3E2 | 5.5E2 | 3.7E2 | 1.0E3 | 5.9E2 | 1.2E3 | 1.0E-9 | 1.0E-9 | 3.0E3 | 4.5E3 | 111 | 24 | 111 | 24 | 0.70 |
| Op | pg/ml | 4.4E5 | 4.4E5 | 4.4E5 | 4.5E5 | 1.6E5 | 1.9E5 | 5.2E4 | 9.4E4 | 7.3E5 | 7.5E5 | 49 | 9 | 49 | 9 | 0.52 |
| Oe | pg/ml | 4.7E1 | 1.0E-9 | 2.5E2 | 1.9E2 | 3.8E2 | 3.4E2 | 1.0E-9 | 1.0E-9 | 2.3E3 | 1.3E3 | 295 | 60 | 295 | 60 | 0.44 |
| Of | pg/ml | 1.5E2 | 1.0E2 | 5.0E3 | 5.3E3 | 2.1E4 | 1.8E4 | 1.0E-9 | 1.0E-9 | 1.9E5 | 1.2E5 | 296 | 61 | 296 | 61 | 0.48 |
| Og | pg/ml | 7.7E-2 | 2.0E-2 | 4.0E-1 | 8.2E-2 | 1.6E0 | 1.5E-1 | 1.0E-9 | 1.0E-9 | 1.9E1 | 8.0E-1 | 296 | 61 | 296 | 61 | 0.37 |
| Oh | pg/ml | 2.4E0 | 5.5E0 | 1.4E1 | 3.0E2 | 9.3E1 | 2.0E3 | 1.0E-9 | 1.0E-9 | 1.4E3 | 1.6E4 | 296 | 61 | 296 | 61 | 0.64 |
| Oi | pg/ml | 1.8E0 | 2.6E0 | 5.1E0 | 4.6E0 | 8.1E0 | 6.1E0 | 1.0E-9 | 1.0E-9 | 6.7E1 | 3.1E1 | 296 | 61 | 296 | 61 | 0.52 |
| Ok | pg/ml | 3.6E2 | 6.7E2 | 4.7E2 | 1.2E3 | 4.7E2 | 1.5E3 | 1.5E1 | 5.3E1 | 4.2E3 | 7.8E3 | 296 | 61 | 296 | 61 | 0.73 |
| Om | pg/ml | 3.9E2 | 6.1E2 | 8.0E2 | 1.9E3 | 2.2E3 | 6.6E3 | 1.0E-9 | 7.0E1 | 3.0E4 | 5.1E4 | 296 | 61 | 296 | 61 | 0.65 |
| On | pg/ml | 1.5E2 | 3.2E2 | 2.6E2 | 7.6E2 | 4.0E2 | 1.2E3 | 1.0E-9 | 1.6E1 | 4.5E3 | 8.5E3 | 296 | 61 | 296 | 61 | 0.74 |
| Or | pg/ml | 1.0E1 | 2.4E1 | 3.1E1 | 9.3E1 | 6.1E1 | 1.4E2 | 1.0E-9 | 1.0E-9 | 4.4E2 | 5.1E2 | 112 | 25 | 112 | 25 | 0.62 |
| Ow | pg/ml | 3.6E1 | 9.4E1 | 1.1E2 | 7.3E2 | 3.2E2 | 1.7E3 | 1.0E-9 | 1.0E-9 | 2.7E3 | 8.1E3 | 112 | 25 | 112 | 25 | 0.70 |
| Ou | pg/ml | 4.2E2 | 1.3E3 | 9.1E2 | 2.7E3 | 1.5E3 | 3.3E3 | 1.0E-9 | 1.0E-9 | 9.8E3 | 1.1E4 | 112 | 25 | 112 | 25 | 0.67 |
| Ul | ng/ml | 1.0E-9 | 1.0E-9 | 1.1E0 | 3.2E0 | 4.5E0 | 1.2E1 | 1.0E-9 | 1.0E-9 | 2.9E1 | 5.6E1 | 112 | 24 | 112 | 24 | 0.50 |
| Uo | ng/ml | 1.0E-9 | 1.0E-9 | 8.2E-2 | 4.1E-2 | 2.2E-1 | 1.4E-1 | 1.0E-9 | 1.0E-9 | 1.3E0 | 6.3E-1 | 112 | 24 | 112 | 24 | 0.46 |
| Uk | ng/ml | 1.0E-9 | 1.0E-9 | 6.9E-3 | 1.9E-3 | 3.4E-2 | 6.6E-3 | 1.0E-9 | 1.0E-9 | 3.2E-1 | 3.1E-2 | 112 | 24 | 112 | 24 | 0.41 |
| Um | ng/ml | 1.0E-9 | 1.0E-9 | 2.3E-1 | 2.3E-1 | 6.0E-1 | 6.4E-1 | 1.0E-9 | 1.0E-9 | 3.6E0 | 2.3E0 | 112 | 24 | 112 | 24 | 0.46 |
| Uf | ng/ml | 5.1E-2 | 9.9E-2 | 1.3E-1 | 4.0E-1 | 1.9E-1 | 1.0E0 | 1.0E-3 | 3.5E-3 | 1.1E0 | 5.1E0 | 112 | 24 | 112 | 24 | 0.66 |
| Uh | ng/ml | 1.9E0 | 5.9E0 | 2.9E0 | 7.0E0 | 2.9E0 | 4.5E0 | 3.6E-2 | 7.1E-1 | 1.5E1 | 1.8E1 | 112 | 24 | 112 | 24 | 0.80 |
| Un | ng/ml | 1.6E0 | 2.4E0 | 1.9E0 | 3.8E0 | 1.2E0 | 4.9E0 | 3.4E-1 | 7.1E-1 | 8.0E0 | 2.5E1 | 112 | 24 | 112 | 24 | 0.70 |
| Ug | ng/ml | 1.3E1 | 6.8E0 | 2.2E1 | 1.9E1 | 2.4E1 | 3.6E1 | 1.2E0 | 1.0E0 | 1.4E2 | 1.6E2 | 112 | 24 | 112 | 24 | 0.36 |
| Ur | ng/ml | 1.1E-1 | 1.0E-9 | 2.9E-1 | 7.1E-1 | 5.0E-1 | 1.9E0 | 1.0E-9 | 1.0E-9 | 3.0E0 | 7.3E0 | 111 | 24 | 111 | 24 | 0.39 |
| Up | ng/ml | 1.0E-9 | 1.0E-9 | 2.7E-3 | 1.1E-1 | 8.4E-3 | 4.8E-1 | 1.0E-9 | 1.0E-9 | 5.3E-2 | 2.4E0 | 111 | 24 | 111 | 24 | 0.54 |
| Us | ng/ml | 3.6E-3 | 1.0E-9 | 1.6E-2 | 9.0E-2 | 4.3E-2 | 3.4E-1 | 1.0E-9 | 1.0E-9 | 3.6E-1 | 1.7E0 | 111 | 24 | 111 | 24 | 0.45 |
| Uv | ng/ml | 3.1E-3 | 1.7E-3 | 1.5E-2 | 2.3E-2 | 4.5E-2 | 8.3E-2 | 1.0E-9 | 1.0E-9 | 3.5E-1 | 4.1E-1 | 111 | 24 | 111 | 24 | 0.45 |
| Ut | ng/ml | 6.8E-1 | 1.4E0 | 2.4E0 | 7.4E0 | 5.9E0 | 1.4E1 | 1.0E-9 | 1.0E-9 | 5.2E1 | 6.5E1 | 111 | 24 | 111 | 24 | 0.64 |
| Uu | ng/ml | 7.1E0 | 5.4E0 | 7.6E0 | 6.6E0 | 5.2E0 | 4.4E0 | 5.4E-1 | 8.1E-1 | 2.9E1 | 1.7E1 | 111 | 24 | 111 | 24 | 0.44 |
| Uw | ng/ml | 2.2E0 | 5.0E0 | 2.7E0 | 8.3E0 | 2.5E0 | 1.2E1 | 1.0E-9 | 1.7E0 | 9.8E0 | 3.9E1 | 50 | 9 | 50 | 9 | 0.76 |
| Vb | ng/ml | 1.1E0 | 1.1E0 | 1.1E0 | 1.5E0 | 4.3E-1 | 1.9E0 | 8.5E-2 | 2.6E-1 | 2.0E0 | 6.4E0 | 50 | 9 | 50 | 9 | 0.43 |
| Vc | ng/ml | 1.0E-9 | 1.0E-9 | 4.3E-3 | 1.0E-9 | 1.9E-2 | 0.0E0 | 1.0E-9 | 1.0E-9 | 1.1E-1 | 1.0E-9 | 50 | 9 | 50 | 9 | 0.46 |
| Uy | ng/ml | 1.3E0 | 1.5E0 | 6.2E0 | 1.7E1 | 1.7E1 | 2.5E1 | 8.7E-2 | 2.0E-2 | 9.9E1 | 6.4E1 | 50 | 9 | 50 | 9 | 0.57 |
| Uz | ng/ml | 1.0E-9 | 1.0E-9 | 8.7E-3 | 3.7E0 | 6.2E-2 | 1.1E1 | 1.0E-9 | 1.0E-9 | 4.4E-1 | 3.3E1 | 50 | 9 | 50 | 9 | 0.60 |
| Ux | ng/ml | 1.8E2 | 1.6E2 | 1.9E2 | 1.9E2 | 1.4E2 | 1.5E2 | 4.5E0 | 4.0E1 | 4.8E2 | 4.9E2 | 50 | 9 | 50 | 9 | 0.50 |
| Va | ng/ml | 1.5E1 | 3.2E0 | 2.5E1 | 9.9E0 | 2.9E1 | 2.0E1 | 3.1E-1 | 1.2E0 | 1.2E2 | 6.2E1 | 50 | 9 | 50 | 9 | 0.31 |
| Vh | ng/ml | 1.1E-2 | 2.2E-2 | 1.9E-2 | 1.1E-1 | 2.5E-2 | 2.8E-1 | 3.9E-4 | 3.5E-3 | 1.2E-1 | 8.6E-1 | 50 | 9 | 50 | 9 | 0.70 |
| Vi | ng/ml | 3.1E-3 | 4.3E-2 | 6.9E-3 | 2.5E-1 | 8.7E-3 | 5.9E-1 | 1.0E-9 | 1.6E-2 | 3.7E-2 | 1.8E0 | 50 | 9 | 50 | 9 | 0.94 |
| Vj | ng/ml | 2.7E1 | 8.9E1 | 5.4E1 | 1.6E2 | 6.7E1 | 2.0E2 | 1.4E0 | 1.4E1 | 3.4E2 | 6.5E2 | 50 | 8 | 50 | 8 | 0.78 |
| Vp | ng/ml | 1.0E-9 | 1.0E-9 | 1.2E-1 | 2.4E0 | 5.6E-1 | 1.0E1 | 1.0E-9 | 1.0E-9 | 3.9E0 | 4.9E1 | 112 | 24 | 112 | 24 | 0.61 |
| Vt | ng/ml | 5.6E0 | 1.1E1 | 7.2E0 | 2.0E1 | 6.1E0 | 3.1E1 | 5.6E-1 | 1.9E0 | 3.2E1 | 1.6E2 | 112 | 24 | 112 | 24 | 0.75 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|-----|--------|-----|------|
| | | NonDis | Dis | NonDis | Dis | NonDis | Dis | NonDis | Dis | NonDis | Dis | NonDis | Dis | NonDis | Dis | |
| Vu | ng/ml | 1.0E-9 | 3.6E-1 | 1.3E0 | 3.0E0 | 2.6E0 | 5.7E0 | 1.0E-9 | 1.0E-9 | 1.6E1 | 2.2E1 | 110 | 22 | 110 | 22 | 0.57 |
| Vq | ng/ml | 1.3E2 | 7.9E2 | 5.1E2 | 1.8E3 | 9.0E2 | 3.0E3 | 9.2E-1 | 6.5E-1 | 5.0E3 | 1.2E4 | 90 | 18 | 90 | 18 | 0.67 |
| Vo | ng/ml | 2.6E1 | 2.5E1 | 2.5E1 | 2.4E1 | 5.4E0 | 6.5E0 | 4.9E0 | 1.9E0 | 4.8E1 | 3.1E1 | 112 | 24 | 112 | 24 | 0.47 |
| Vs | ng/ml | 1.0E-9 | 1.0E-9 | 4.5E0 | 2.5E1 | 1.2E1 | 9.7E1 | 1.0E-9 | 1.0E-9 | 8.9E1 | 4.5E2 | 111 | 22 | 111 | 22 | 0.49 |
| Vv | ng/ml | 2.9E0 | 2.6E0 | 6.0E0 | 6.6E0 | 1.0E1 | 1.0E1 | 1.0E-9 | 1.0E-9 | 8.4E1 | 4.4E1 | 112 | 23 | 112 | 23 | 0.50 |
| Vw | ng/ml | 3.6E1 | 4.3E1 | 3.4E1 | 4.4E1 | 1.8E1 | 2.2E1 | 2.5E0 | 1.1E1 | 6.7E1 | 6.9E1 | 50 | 9 | 50 | 9 | 0.66 |
| Oy | pg/ml | 4.8E-1 | 4.3E-1 | 6.7E0 | 2.7E0 | 3.3E1 | 6.4E0 | 1.0E-9 | 1.0E-9 | 4.0E2 | 3.3E1 | 295 | 61 | 295 | 61 | 0.45 |
| Oz | pg/ml | 3.1E-2 | 1.0E-9 | 3.5E-1 | 5.9E-1 | 1.7E0 | 3.6E0 | 1.0E-9 | 1.0E-9 | 2.9E1 | 2.8E1 | 295 | 61 | 295 | 61 | 0.39 |
| Pa | pg/ml | 3.8E-1 | 6.3E-1 | 1.4E0 | 7.3E0 | 6.1E0 | 3.2E1 | 1.0E-9 | 1.0E-9 | 8.6E1 | 2.3E2 | 295 | 61 | 295 | 61 | 0.62 |
| Pb | pg/ml | 1.0E-9 | 1.0E-9 | 1.8E0 | 1.1E0 | 2.8E1 | 5.4E0 | 1.0E-9 | 1.0E-9 | 4.9E2 | 3.2E1 | 295 | 61 | 295 | 61 | 0.42 |
| Pc | pg/ml | 5.4E-2 | 1.0E-9 | 4.0E-1 | 6.3E0 | 9.4E-1 | 4.2E1 | 1.0E-9 | 1.0E-9 | 1.4E1 | 3.3E2 | 295 | 61 | 295 | 61 | 0.45 |
| Pd | pg/ml | 1.6E0 | 2.4E0 | 6.6E0 | 8.1E0 | 5.0E1 | 1.8E1 | 1.0E-9 | 1.0E-9 | 8.4E2 | 1.2E2 | 295 | 61 | 295 | 61 | 0.57 |
| Pe | pg/ml | 1.9E1 | 6.1E1 | 9.5E1 | 6.6E2 | 4.0E2 | 2.2E3 | 1.0E-9 | 1.0E-9 | 4.7E3 | 1.5E4 | 295 | 61 | 295 | 61 | 0.71 |
| Pf | pg/ml | 1.3E0 | 5.8E0 | 1.4E1 | 2.9E1 | 9.4E1 | 7.4E1 | 1.0E-9 | 1.0E-9 | 1.5E3 | 4.3E2 | 295 | 61 | 295 | 61 | 0.70 |
| Pg | pg/ml | 3.3E0 | 1.1E1 | 6.9E1 | 1.5E2 | 5.4E2 | 3.8E2 | 1.0E-9 | 1.0E-9 | 7.7E3 | 2.2E3 | 295 | 61 | 295 | 61 | 0.70 |
| Ph | ng/ml | 1.4E-1 | 2.4E-1 | 3.4E-1 | 5.9E-1 | 4.9E-1 | 1.1E0 | 1.0E-9 | 1.0E-9 | 2.8E0 | 5.4E0 | 112 | 25 | 112 | 25 | 0.55 |
| Pi | ng/ml | 1.9E-1 | 3.5E-1 | 2.8E-1 | 3.8E0 | 3.8E-1 | 1.6E1 | 1.0E-9 | 1.0E-9 | 3.2E0 | 8.2E1 | 112 | 25 | 112 | 25 | 0.64 |
| Pj | ng/mL | 5.0E0 | 7.3E0 | 5.8E0 | 8.0E0 | 4.4E0 | 5.0E0 | 4.0E-1 | 6.6E-1 | 3.1E1 | 2.3E1 | 112 | 25 | 112 | 25 | 0.64 |
| Pk | ng/ml | 8.9E-3 | 1.0E-2 | 1.6E-2 | 7.3E-2 | 3.0E-2 | 3.0E-1 | 1.0E-9 | 1.0E-9 | 2.5E-1 | 1.5E0 | 112 | 25 | 112 | 25 | 0.53 |
| aA | mg/dL | 8.8E-1 | 1.0E0 | 9.9E-1 | 1.4E0 | 5.0E-1 | 9.3E-1 | 3.0E-1 | 5.0E-1 | 4.2E0 | 4.7E0 | 456 | 83 | 456 | 83 | 0.64 |
| aC | mg/mL | 2.2E0 | 2.1E0 | 2.6E0 | 2.6E0 | 1.3E0 | 1.4E0 | 7.5E-1 | 1.0E0 | 7.4E0 | 6.7E0 | 140 | 36 | 140 | 36 | 0.46 |
| aD | ug/mL | 3.0E0 | 3.4E0 | 4.7E0 | 4.7E0 | 4.9E0 | 3.9E0 | 7.5E-1 | 9.2E-1 | 3.5E1 | 1.8E1 | 140 | 36 | 140 | 36 | 0.49 |
| aE | mg/mL | 5.9E-1 | 5.3E-1 | 6.0E-1 | 5.6E-1 | 1.7E-1 | 1.7E-1 | 1.8E-1 | 2.2E-1 | 1.1E0 | 1.2E0 | 140 | 36 | 140 | 36 | 0.41 |
| aF | ng/mL | 2.1E0 | 3.0E0 | 5.0E0 | 5.3E0 | 8.0E0 | 6.3E0 | 4.3E-3 | 4.3E-3 | 5.0E1 | 2.9E1 | 140 | 36 | 140 | 36 | 0.53 |
| aG | mg/mL | 1.4E-1 | 1.2E-1 | 1.6E-1 | 1.4E-1 | 9.1E-2 | 6.2E-2 | 3.2E-2 | 6.8E-2 | 4.8E-1 | 3.2E-1 | 140 | 36 | 140 | 36 | 0.44 |
| aH | ug/mL | 7.6E1 | 5.7E1 | 8.1E1 | 6.4E1 | 4.2E1 | 3.1E1 | 8.9E0 | 1.1E1 | 2.0E2 | 1.4E2 | 140 | 36 | 140 | 36 | 0.38 |
| aI | ug/mL | 1.8E2 | 1.4E2 | 1.8E2 | 1.5E2 | 6.1E1 | 5.7E1 | 3.2E1 | 7.5E1 | 3.4E2 | 2.7E2 | 140 | 36 | 140 | 36 | 0.36 |
| aJ | ug/mL | 2.3E0 | 3.6E0 | 3.0E0 | 4.6E0 | 2.1E0 | 4.1E0 | 8.2E-1 | 1.4E0 | 1.4E1 | 2.3E1 | 140 | 36 | 140 | 36 | 0.64 |
| aK | ng/mL | 1.3E0 | 1.2E0 | 2.0E0 | 1.8E0 | 2.0E0 | 1.7E0 | 2.9E-4 | 1.0E-1 | 1.0E1 | 6.5E0 | 140 | 36 | 140 | 36 | 0.47 |
| aL | mg/mL | 7.4E-1 | 7.2E-1 | 7.8E-1 | 7.1E-1 | 2.5E-1 | 2.6E-1 | 2.2E-1 | 2.7E-1 | 1.7E0 | 1.4E0 | 140 | 36 | 140 | 36 | 0.43 |
| aM | U/mL | 1.7E1 | 2.4E1 | 3.5E1 | 8.5E1 | 5.1E1 | 1.8E2 | 4.2E-2 | 4.2E-2 | 3.5E2 | 8.2E2 | 140 | 36 | 140 | 36 | 0.61 |
| aN | U/mL | 1.4E1 | 1.9E1 | 2.3E1 | 3.7E1 | 3.7E1 | 5.8E1 | 2.5E-3 | 2.5E-3 | 3.8E2 | 3.3E2 | 140 | 36 | 140 | 36 | 0.58 |
| aO | pg/mL | 4.8E1 | 1.1E2 | 4.1E2 | 5.3E2 | 1.0E3 | 7.5E2 | 6.0E-2 | 5.0E0 | 6.6E3 | 2.4E3 | 140 | 36 | 140 | 36 | 0.60 |
| aP | ng/mL | 1.6E0 | 1.9E0 | 2.1E0 | 3.4E0 | 2.5E0 | 4.6E0 | 4.5E-1 | 7.8E-1 | 2.8E1 | 2.8E1 | 140 | 36 | 140 | 36 | 0.63 |
| aQ | ng/mL | 2.6E-1 | 2.3E-1 | 3.7E-1 | 2.8E-1 | 3.4E-1 | 1.9E-1 | 2.0E-4 | 5.1E-2 | 2.0E0 | 9.0E-1 | 140 | 36 | 140 | 36 | 0.45 |
| aR | ng/mL | 1.7E0 | 2.5E0 | 2.9E0 | 3.6E0 | 4.1E0 | 3.6E0 | 2.6E-1 | 5.6E-1 | 3.4E1 | 1.7E1 | 140 | 36 | 140 | 36 | 0.59 |
| aS | ng/mL | 3.7E-1 | 4.6E-1 | 1.0E0 | 9.1E-1 | 2.9E0 | 1.1E0 | 4.2E-3 | 6.0E-2 | 3.3E1 | 4.9E0 | 140 | 36 | 140 | 36 | 0.54 |
| aU | pg/mL | 6.7E1 | 5.9E1 | 1.0E2 | 9.1E1 | 1.1E2 | 1.0E2 | 7.4E-2 | 7.4E-2 | 7.0E2 | 5.1E2 | 140 | 36 | 140 | 36 | 0.46 |
| aV | ng/mL | 6.2E-1 | 3.6E-1 | 1.1E0 | 7.6E-1 | 2.8E0 | 1.1E0 | 7.6E-4 | 9.1E-2 | 3.3E1 | 6.0E0 | 140 | 36 | 140 | 36 | 0.43 |
| aW | pg/mL | 2.0E1 | 1.8E1 | 2.1E1 | 2.9E1 | 1.6E1 | 6.7E1 | 7.2E-2 | 7.2E-2 | 1.7E2 | 4.2E2 | 140 | 36 | 140 | 36 | 0.47 |
| aX | ng/mL | 8.3E0 | 7.6E0 | 1.4E1 | 2.6E1 | 2.2E1 | 5.6E1 | 3.0E-1 | 7.7E-1 | 2.2E2 | 3.1E2 | 140 | 36 | 140 | 36 | 0.51 |
| aY | pg/mL | 5.3E1 | 5.5E1 | 7.5E1 | 7.9E1 | 1.1E2 | 7.7E1 | 4.1E-1 | 4.1E-1 | 1.2E3 | 3.9E2 | 140 | 36 | 140 | 36 | 0.53 |
| aZ | pg/mL | 2.2E2 | 3.7E2 | 5.3E2 | 1.2E3 | 1.2E3 | 2.0E3 | 1.7E0 | 1.7E0 | 1.2E4 | 7.9E3 | 140 | 36 | 140 | 36 | 0.62 |
| bA | ng/mL | 1.0E1 | 4.6E1 | 4.8E1 | 1.8E2 | 1.1E2 | 3.1E2 | 3.0E-2 | 2.0E0 | 9.4E2 | 1.5E3 | 140 | 36 | 140 | 36 | 0.70 |
| bB | ng/mL | 2.9E2 | 2.3E2 | 3.2E2 | 2.4E2 | 1.8E2 | 1.4E2 | 2.1E0 | 1.2E1 | 9.5E2 | 5.7E2 | 140 | 36 | 140 | 36 | 0.37 |
| bC | ng/mL | 3.2E2 | 3.3E2 | 6.1E2 | 8.0E2 | 8.2E2 | 1.1E3 | 1.4E1 | 5.0E1 | 4.7E3 | 4.0E3 | 140 | 36 | 140 | 36 | 0.54 |
| bE | mg/mL | 5.2E0 | 5.0E0 | 5.5E0 | 5.5E0 | 2.1E0 | 2.6E0 | 1.8E0 | 1.3E0 | 1.2E1 | 1.2E1 | 140 | 36 | 140 | 36 | 0.47 |
| bF | pg/mL | 3.2E1 | 6.6E1 | 3.2E2 | 4.5E2 | 1.4E3 | 1.1E3 | 5.0E-2 | 8.9E0 | 1.1E4 | 6.3E3 | 140 | 36 | 140 | 36 | 0.66 |
| bG | ng/mL | 1.5E0 | 2.2E0 | 2.9E0 | 4.1E0 | 4.0E0 | 5.7E0 | 1.1E-1 | 1.6E-1 | 2.6E1 | 3.0E1 | 140 | 36 | 140 | 36 | 0.57 |
| bH | pg/mL | 5.7E-1 | 5.7E-1 | 6.6E0 | 4.7E0 | 2.6E1 | 6.0E0 | 5.7E-1 | 5.7E-1 | 2.8E2 | 2.3E1 | 140 | 36 | 140 | 36 | 0.55 |
| bI | ng/mL | 4.0E-3 | 4.0E-3 | 9.4E-2 | 8.5E-2 | 1.9E-1 | 2.1E-1 | 4.0E-3 | 4.0E-3 | 9.8E-1 | 8.8E-1 | 140 | 36 | 140 | 36 | 0.47 |
| bJ | mg/mL | 1.9E0 | 1.9E0 | 2.4E0 | 2.3E0 | 2.0E0 | 2.1E0 | 2.5E-4 | 2.5E-4 | 1.1E1 | 9.0E0 | 140 | 36 | 140 | 36 | 0.47 |
| bL | pg/mL | 4.0E0 | 3.9E0 | 9.5E0 | 8.5E0 | 1.2E1 | 8.6E0 | 4.6E-2 | 4.6E-2 | 6.0E1 | 3.2E1 | 140 | 36 | 140 | 36 | 0.53 |

| Code | Units | Median | | Average | | Standard Deviation | | Minimum | | Maximum | | Number of Samples | | Number of Patients | | AUC |
|------|-------|--------|--------|---------|--------|--------|--------|--------|--------|--------|--------|--------|-----|--------|-----|-----|
| | | NonDis | Dis | NonDis | Dis | NonDis | Dis | NonDis | Dis | NonDis | Dis | NonDis | Dis | NonDis | Dis | |
| bM | mg/mL | 1.8E0 | 2.1E0 | 2.2E0 | 2.1E0 | 1.5E0 | 1.3E0 | 1.4E-1 | 1.6E-2 | 8.6E0 | 5.4E0 | 140 | 36 | 140 | 36 | 0.52 |
| bN | ng/mL | 3.5E1 | 2.7E1 | 1.4E2 | 5.6E1 | 3.1E2 | 8.5E1 | 1.4E-1 | 5.9E-1 | 1.9E3 | 4.4E2 | 140 | 36 | 140 | 36 | 0.42 |
| bO | ng/mL | 4.0E-2 | 4.0E-2 | 8.5E0 | 1.1E1 | 1.8E1 | 2.7E1 | 4.0E-2 | 4.0E-2 | 1.2E2 | 1.3E2 | 140 | 36 | 140 | 36 | 0.45 |
| bP | mg/mL | 5.2E-1 | 5.8E-1 | 7.4E-1 | 7.5E-1 | 7.0E-1 | 7.1E-1 | 4.9E-2 | 9.2E-2 | 4.8E0 | 3.5E0 | 140 | 36 | 140 | 36 | 0.52 |
| bQ | pg/mL | 2.1E1 | 5.2E1 | 1.5E2 | 6.5E1 | 1.1E3 | 5.7E1 | 1.5E-1 | 1.5E-1 | 1.3E4 | 2.2E2 | 140 | 36 | 140 | 36 | 0.69 |
| bR | ng/mL | 1.2E-2 | 1.2E-2 | 1.8E-1 | 7.2E-2 | 7.9E-1 | 1.1E-1 | 1.2E-2 | 1.2E-2 | 8.7E0 | 3.9E-1 | 140 | 36 | 140 | 36 | 0.45 |
| bS | pg/mL | 9.4E-1 | 9.4E-1 | 1.0E1 | 6.3E0 | 4.5E1 | 1.7E1 | 9.4E-1 | 9.4E-1 | 3.9E2 | 7.2E1 | 140 | 36 | 140 | 36 | 0.49 |
| bU | ng/mL | 6.9E-2 | 1.5E-1 | 1.9E-1 | 1.7E-1 | 5.9E-1 | 1.8E-1 | 1.3E-2 | 1.3E-2 | 6.6E0 | 6.5E-1 | 140 | 36 | 140 | 36 | 0.55 |
| bV | pg/mL | 4.7E2 | 5.9E2 | 6.1E2 | 7.2E2 | 9.7E2 | 4.5E2 | 1.6E2 | 2.7E2 | 1.2E4 | 2.2E3 | 140 | 36 | 140 | 36 | 0.63 |
| bW | pg/mL | 3.3E2 | 3.1E2 | 5.0E2 | 6.3E2 | 5.4E2 | 9.4E2 | 8.4E1 | 1.3E2 | 4.8E3 | 3.9E3 | 140 | 36 | 140 | 36 | 0.51 |
| bX | ng/mL | 1.5E-3 | 2.5E-5 | 2.7E-3 | 2.1E-3 | 3.3E-3 | 2.8E-3 | 2.5E-5 | 2.5E-5 | 1.3E-2 | 7.8E-3 | 140 | 36 | 140 | 36 | 0.45 |
| bZ | pg/mL | 2.7E2 | 6.6E2 | 1.9E3 | 2.5E3 | 7.1E3 | 7.1E3 | 1.5E-1 | 1.0E2 | 5.8E4 | 4.3E4 | 140 | 36 | 140 | 36 | 0.63 |
| cA | pg/mL | 6.0E-1 | 6.0E-1 | 4.6E0 | 2.0E0 | 3.2E1 | 4.5E0 | 6.0E-1 | 6.0E-1 | 3.7E2 | 2.1E1 | 140 | 36 | 140 | 36 | 0.49 |
| cB | ng/mL | 5.1E-2 | 3.2E-2 | 7.4E-2 | 7.6E-2 | 8.2E-2 | 1.1E-1 | 1.7E-3 | 1.7E-3 | 3.8E-1 | 4.3E-1 | 140 | 36 | 140 | 36 | 0.44 |
| cC | pg/mL | 4.1E1 | 4.7E1 | 4.6E1 | 4.3E1 | 5.4E1 | 2.7E1 | 1.0E0 | 1.0E0 | 4.5E2 | 1.1E2 | 140 | 36 | 140 | 36 | 0.53 |
| cD | pg/mL | 4.9E0 | 4.7E0 | 1.4E1 | 8.2E0 | 5.0E1 | 1.5E1 | 3.3E-1 | 3.3E-1 | 4.8E2 | 9.0E1 | 140 | 36 | 140 | 36 | 0.50 |
| cE | pg/mL | 5.4E1 | 1.1E2 | 2.6E2 | 2.9E2 | 6.3E2 | 4.3E2 | 1.2E-1 | 1.4E1 | 3.8E3 | 2.1E3 | 140 | 36 | 140 | 36 | 0.64 |
| cF | pg/mL | 5.3E-1 | 4.6E0 | 1.6E1 | 1.3E1 | 3.2E1 | 1.7E1 | 5.3E-1 | 5.3E-1 | 2.7E2 | 6.5E1 | 140 | 36 | 140 | 36 | 0.50 |
| cG | pg/mL | 5.2E1 | 1.2E2 | 1.8E2 | 1.8E2 | 8.9E2 | 2.0E2 | 7.8E0 | 2.4E1 | 1.0E4 | 1.1E3 | 140 | 36 | 140 | 36 | 0.68 |
| cH | uIU/mL | 3.7E0 | 2.3E0 | 7.9E0 | 6.3E0 | 1.8E1 | 1.2E1 | 8.6E-3 | 8.6E-3 | 1.6E2 | 5.3E1 | 140 | 36 | 140 | 36 | 0.39 |
| cI | ng/mL | 6.0E0 | 9.4E0 | 1.3E1 | 2.1E1 | 1.7E1 | 3.2E1 | 2.3E-1 | 3.2E-2 | 1.0E2 | 1.2E2 | 140 | 36 | 140 | 36 | 0.54 |
| cJ | ug/mL | 7.2E1 | 3.9E1 | 1.1E2 | 6.4E1 | 1.1E2 | 6.6E1 | 6.9E0 | 5.6E0 | 6.4E2 | 3.4E2 | 140 | 36 | 140 | 36 | 0.38 |
| cK | ng/mL | 3.8E-3 | 3.8E-3 | 2.4E-2 | 1.7E-2 | 1.3E-1 | 3.8E-2 | 3.8E-3 | 3.8E-3 | 1.5E0 | 2.1E-1 | 140 | 36 | 140 | 36 | 0.53 |
| cL | pg/mL | 2.1E2 | 2.2E2 | 5.7E2 | 4.2E2 | 2.2E3 | 5.3E2 | 3.1E1 | 6.7E1 | 2.4E4 | 2.8E3 | 140 | 36 | 140 | 36 | 0.60 |
| cM | pg/mL | 2.7E2 | 2.7E2 | 2.9E2 | 2.7E2 | 1.7E2 | 1.1E2 | 2.5E1 | 5.7E1 | 1.1E3 | 4.8E2 | 140 | 36 | 140 | 36 | 0.49 |
| cN | pg/mL | 1.2E2 | 1.3E2 | 1.3E2 | 1.3E2 | 9.2E1 | 4.3E1 | 3.8E1 | 7.9E1 | 1.1E3 | 2.7E2 | 140 | 36 | 140 | 36 | 0.57 |
| cO | pg/mL | 2.0E2 | 2.7E2 | 4.3E2 | 3.3E2 | 1.6E3 | 2.5E2 | 5.4E1 | 9.6E1 | 1.9E4 | 1.5E3 | 140 | 36 | 140 | 36 | 0.62 |
| cP | ng/mL | 2.5E3 | 2.3E3 | 2.6E3 | 2.4E3 | 9.6E2 | 9.1E2 | 6.2E2 | 1.0E3 | 5.6E3 | 4.7E3 | 140 | 36 | 140 | 36 | 0.46 |
| cQ | ng/mL | 5.3E-2 | 4.5E-2 | 1.3E-1 | 1.2E-1 | 2.1E-1 | 2.1E-1 | 2.0E-3 | 2.0E-3 | 1.3E0 | 8.7E-1 | 140 | 36 | 140 | 36 | 0.46 |
| cR | ng/mL | 3.0E2 | 4.3E2 | 5.7E2 | 7.1E2 | 8.0E2 | 9.0E2 | 2.0E1 | 8.6E1 | 7.7E3 | 4.8E3 | 140 | 36 | 140 | 36 | 0.57 |
| cS | ng/mL | 2.8E2 | 3.5E2 | 4.1E2 | 7.4E2 | 4.4E2 | 1.3E3 | 4.1E1 | 9.1E1 | 2.5E3 | 7.1E3 | 140 | 36 | 140 | 36 | 0.56 |
| cT | ng/mL | 4.4E1 | 1.0E2 | 1.2E2 | 3.0E2 | 2.4E2 | 4.6E2 | 3.6E0 | 1.1E1 | 2.1E3 | 1.5E3 | 140 | 36 | 140 | 36 | 0.64 |
| cU | ng/mL | 5.6E1 | 9.5E1 | 9.2E1 | 1.2E2 | 1.6E2 | 1.0E2 | 6.2E0 | 9.0E0 | 1.6E3 | 4.2E2 | 140 | 36 | 140 | 36 | 0.65 |
| cV | ng/mL | 1.8E-1 | 2.2E-1 | 7.4E-1 | 6.6E-1 | 4.0E0 | 1.7E0 | 2.5E-2 | 3.0E-2 | 4.7E1 | 9.7E0 | 140 | 36 | 140 | 36 | 0.55 |
| cW | mIU/mL | 4.8E-2 | 5.6E-2 | 9.1E-2 | 8.2E-2 | 3.8E-1 | 7.9E-2 | 4.8E-3 | 1.4E-2 | 4.5E0 | 3.9E-1 | 140 | 36 | 140 | 36 | 0.57 |
| cX | ng/mL | 1.6E-1 | 5.4E-2 | 1.7E0 | 2.3E0 | 5.1E0 | 7.1E0 | 2.3E-4 | 2.3E-4 | 2.8E1 | 2.8E1 | 140 | 36 | 140 | 36 | 0.45 |
| cY | ng/mL | 7.4E0 | 8.0E0 | 1.1E1 | 9.9E0 | 1.1E1 | 9.2E0 | 1.7E-1 | 6.0E-1 | 6.1E1 | 3.7E1 | 140 | 36 | 140 | 36 | 0.49 |
| cZ | ug/mL | 1.3E1 | 1.2E1 | 1.5E1 | 1.3E1 | 6.8E0 | 6.8E0 | 2.3E0 | 3.3E0 | 4.6E1 | 3.0E1 | 140 | 36 | 140 | 36 | 0.41 |
| dA | pg/mL | 3.1E2 | 3.4E2 | 3.9E2 | 3.8E2 | 5.0E2 | 2.1E2 | 1.0E2 | 1.1E2 | 5.8E3 | 9.3E2 | 140 | 36 | 140 | 36 | 0.51 |
| dB | ug/mL | 1.8E1 | 2.1E1 | 1.8E1 | 1.9E1 | 2.2E1 | 9.0E0 | 2.1E0 | 2.2E0 | 2.5E2 | 3.2E1 | 140 | 36 | 140 | 36 | 0.58 |
| dC | nmol/L | 3.4E1 | 3.6E1 | 3.7E1 | 3.7E1 | 1.7E1 | 1.4E1 | 7.8E0 | 1.5E1 | 1.4E2 | 6.5E1 | 140 | 36 | 140 | 36 | 0.52 |
| dD | ug/mL | 3.4E1 | 3.0E1 | 3.6E1 | 3.2E1 | 1.1E1 | 1.1E1 | 1.4E1 | 1.4E1 | 7.4E1 | 6.0E1 | 140 | 36 | 140 | 36 | 0.37 |
| dE | ng/mL | 4.1E-1 | 5.6E-1 | 5.5E-1 | 6.9E-1 | 5.7E-1 | 7.2E-1 | 8.4E-3 | 8.4E-3 | 2.7E0 | 2.9E0 | 140 | 36 | 140 | 36 | 0.56 |
| dF | ng/mL | 2.4E2 | 3.7E2 | 3.1E2 | 4.9E2 | 2.2E2 | 3.2E2 | 7.5E1 | 1.1E2 | 1.3E3 | 1.2E3 | 140 | 36 | 140 | 36 | 0.69 |
| dG | ng/mL | 1.1E1 | 1.5E1 | 1.6E1 | 2.1E1 | 1.9E1 | 1.8E1 | 3.0E0 | 4.8E0 | 1.8E2 | 8.7E1 | 140 | 36 | 140 | 36 | 0.61 |
| dH | pg/mL | 7.6E0 | 1.1E1 | 2.2E1 | 1.6E1 | 6.8E1 | 1.8E1 | 4.0E-2 | 4.0E-2 | 6.7E2 | 7.9E1 | 140 | 36 | 140 | 36 | 0.61 |
| dI | pg/mL | 4.6E-1 | 4.6E-1 | 4.1E0 | 2.4E0 | 2.8E1 | 3.7E0 | 4.6E-1 | 4.6E-1 | 3.3E2 | 1.9E1 | 140 | 36 | 140 | 36 | 0.56 |
| dJ | ng/mL | 2.0E0 | 2.2E0 | 2.1E0 | 2.2E0 | 1.1E0 | 1.2E0 | 3.2E-2 | 3.2E-2 | 5.6E0 | 4.4E0 | 140 | 36 | 140 | 36 | 0.53 |
| dK | uIU/mL | 1.5E0 | 8.4E-1 | 2.2E0 | 1.7E0 | 3.8E0 | 2.3E0 | 2.8E-4 | 1.4E-2 | 3.9E1 | 1.1E1 | 140 | 36 | 140 | 36 | 0.38 |
| dL | ng/mL | 8.7E2 | 9.5E2 | 1.0E3 | 1.2E3 | 5.4E2 | 8.7E2 | 2.8E2 | 4.3E2 | 3.4E3 | 4.8E3 | 140 | 36 | 140 | 36 | 0.55 |
| dM | pg/mL | 9.6E2 | 1.1E3 | 1.2E3 | 1.9E3 | 1.4E3 | 1.9E3 | 3.7E2 | 6.3E2 | 1.5E4 | 9.6E3 | 140 | 36 | 140 | 36 | 0.64 |
| dN | ug/mL | 9.8E1 | 1.1E2 | 1.0E2 | 1.2E2 | 4.0E1 | 5.5E1 | 2.4E1 | 4.7E1 | 2.8E2 | 3.3E2 | 140 | 36 | 140 | 36 | 0.59 |
| dR | pg/ml | 1.6E3 | 9.4E2 | 2.1E3 | 1.5E3 | 2.0E3 | 1.9E3 | 1.4E2 | 1.3E2 | 9.8E3 | 8.9E3 | 107 | 26 | 107 | 26 | 0.36 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dU | pg/ml | 9.9E3 | 1.4E4 | 1.5E4 | 1.5E4 | 1.7E4 | 1.2E4 | 6.9E2 | 3.1E3 | 8.1E4 | 3.5E4 | 26 | 7 | 26 | 7 | 0.54 |
| dX | ng/ml | 6.6E-2 | 8.2E-2 | 1.4E-1 | 1.2E-1 | 2.1E-1 | 1.3E-1 | 2.6E-3 | 2.6E-3 | 1.2E0 | 4.2E-1 | 46 | 15 | 46 | 15 | 0.51 |
| eF | ng/ml | 4.1E0 | 4.6E0 | 5.2E0 | 6.1E0 | 4.6E0 | 5.4E0 | 2.0E0 | 2.0E0 | 4.6E1 | 2.9E1 | 107 | 26 | 107 | 26 | 0.57 |
| eC | pg/ml | 3.0E2 | 2.3E2 | 3.7E2 | 3.2E2 | 2.7E2 | 4.0E2 | 9.9E0 | 1.9E1 | 1.6E3 | 2.0E3 | 95 | 22 | 95 | 22 | 0.35 |
| eD | pg/ml | 2.2E2 | 2.1E2 | 7.6E2 | 4.1E2 | 1.7E3 | 8.2E2 | 5.2E-1 | 5.2E-1 | 8.3E3 | 3.8E3 | 78 | 19 | 78 | 19 | 0.49 |
| eM | ng/ml | 2.6E0 | 3.2E0 | 4.5E0 | 6.5E0 | 6.0E0 | 7.6E0 | 6.9E-1 | 8.8E-1 | 3.9E1 | 2.6E1 | 61 | 18 | 61 | 18 | 0.58 |
| eP | ng/ml | 3.7E-3 | 3.7E-3 | 6.6E-1 | 2.8E0 | 1.6E0 | 7.3E0 | 3.7E-3 | 3.7E-3 | 8.6E0 | 2.8E1 | 46 | 15 | 46 | 15 | 0.55 |
| eT | ng/ml | 2.8E2 | 5.6E2 | 7.0E2 | 9.7E2 | 8.1E2 | 1.1E3 | 1.0E2 | 7.1E1 | 2.9E3 | 2.9E3 | 46 | 9 | 46 | 9 | 0.52 |
| eQ | pg/ml | 1.0E0 | 1.0E0 | 6.1E1 | 2.2E1 | 1.2E2 | 5.5E1 | 1.0E0 | 1.0E0 | 4.7E2 | 1.5E2 | 26 | 7 | 26 | 7 | 0.40 |
| eZ | ng/ml | 4.8E1 | 6.2E1 | 5.4E1 | 6.9E1 | 2.5E1 | 2.4E1 | 1.8E1 | 3.2E1 | 1.2E2 | 1.1E2 | 46 | 9 | 46 | 9 | 0.71 |
| fB | ng/ml | 5.5E2 | 6.7E2 | 6.3E2 | 7.5E2 | 2.6E2 | 2.9E2 | 2.6E2 | 3.9E2 | 1.3E3 | 1.3E3 | 24 | 7 | 24 | 7 | 0.65 |
| fN | ng/ml | 2.1E-1 | 2.1E-1 | 3.7E0 | 4.9E0 | 9.2E0 | 6.2E0 | 2.1E-1 | 2.1E-1 | 5.4E1 | 1.4E1 | 46 | 9 | 46 | 9 | 0.58 |
| fP | ng/ml | 2.7E2 | 2.7E2 | 3.2E2 | 3.4E2 | 1.9E2 | 1.7E2 | 1.8E0 | 9.5E1 | 1.0E3 | 7.7E2 | 103 | 24 | 103 | 24 | 0.53 |
| fR | ng/ml | 1.3E5 | 2.3E5 | 2.0E5 | 3.0E5 | 1.6E5 | 2.2E5 | 3.6E4 | 1.9E2 | 6.9E5 | 8.7E5 | 87 | 28 | 87 | 28 | 0.64 |
| fY | ng/ml | 2.6E2 | 2.5E2 | 2.5E2 | 2.4E2 | 1.1E2 | 9.0E1 | 3.6E1 | 1.2E2 | 4.8E2 | 3.8E2 | 46 | 9 | 46 | 9 | 0.47 |
| gC | ng/ml | 2.4E2 | 2.7E2 | 2.5E2 | 3.0E2 | 1.1E2 | 1.4E2 | 8.3E1 | 1.5E2 | 6.4E2 | 5.9E2 | 37 | 10 | 37 | 10 | 0.58 |
| gL | pg/ml | 6.4E4 | 7.4E4 | 7.2E4 | 8.8E4 | 3.5E4 | 4.4E4 | 1.1E4 | 4.3E4 | 1.9E5 | 2.2E5 | 107 | 26 | 107 | 26 | 0.62 |
| gP | U/ml | 2.8E2 | 2.8E2 | 2.9E2 | 2.9E2 | 1.3E2 | 9.0E1 | 1.2E1 | 1.3E2 | 1.1E3 | 5.2E2 | 106 | 26 | 106 | 26 | 0.53 |
| gW | ng/ml | 5.7E2 | 3.6E2 | 9.7E2 | 6.7E2 | 1.1E3 | 7.4E2 | 2.3E0 | 5.5E1 | 6.1E3 | 3.1E3 | 80 | 19 | 80 | 19 | 0.44 |
| gV | ng/ml | 2.0E1 | 2.5E1 | 2.1E1 | 2.3E1 | 6.6E0 | 1.0E1 | 1.0E1 | 8.1E-2 | 3.7E1 | 3.4E1 | 30 | 8 | 30 | 8 | 0.65 |
| tF | pg/mL | 1.3E3 | 1.0E3 | 1.4E4 | 4.2E3 | 4.3E4 | 6.7E3 | 1.2E1 | 1.8E1 | 2.8E5 | 2.4E4 | 96 | 23 | 96 | 23 | 0.49 |
| gZ | ug/ml | 8.0E-1 | 7.8E-1 | 4.3E1 | 3.0E1 | 1.2E2 | 7.5E1 | 8.7E-2 | 1.4E-1 | 4.1E2 | 2.0E2 | 26 | 7 | 26 | 7 | 0.51 |
| hA | ng/ml | 2.4E0 | 3.9E0 | 1.4E1 | 1.5E1 | 5.4E1 | 2.7E1 | 1.7E-2 | 1.7E-2 | 3.5E2 | 1.1E2 | 78 | 20 | 78 | 20 | 0.66 |
| nM | pg/ml | 1.0E-9 | 1.0E-9 | 1.0E-9 | 8.5E1 | 0.0E0 | 3.6E2 | 1.0E-9 | 1.0E-9 | 1.0E-9 | 1.5E3 | 58 | 18 | 58 | 18 | 0.53 |
| nN | pg/ml | 1.3E3 | 3.3E3 | 3.9E3 | 1.4E4 | 1.4E4 | 3.6E4 | 8.1E1 | 4.1E2 | 1.0E5 | 1.5E5 | 58 | 18 | 58 | 18 | 0.68 |
| nO | pg/ml | 2.4E1 | 3.0E1 | 3.8E1 | 3.9E1 | 4.9E1 | 3.2E1 | 4.0E0 | 9.7E0 | 3.1E2 | 1.4E2 | 58 | 18 | 58 | 18 | 0.57 |
| nR | pg/ml | 1.6E1 | 4.4E1 | 6.7E1 | 2.1E2 | 1.8E2 | 4.6E2 | 1.0E0 | 2.7E0 | 1.1E3 | 1.9E3 | 58 | 18 | 58 | 18 | 0.66 |
| nT | pg/ml | 7.0E1 | 9.2E1 | 1.0E2 | 1.6E2 | 1.0E2 | 2.2E2 | 1.0E-9 | 1.0E-9 | 6.4E2 | 9.2E2 | 58 | 18 | 58 | 18 | 0.56 |
| nU | pg/ml | 2.9E1 | 1.0E2 | 7.4E1 | 2.1E2 | 2.0E2 | 2.6E2 | 1.0E-9 | 1.0E-9 | 1.5E3 | 9.2E2 | 58 | 18 | 58 | 18 | 0.75 |
| lW | pg/ml | 1.0E-9 | 1.0E-9 | 1.1E1 | 8.3E0 | 3.2E1 | 1.9E1 | 1.0E-9 | 1.0E-9 | 1.7E2 | 7.0E1 | 58 | 18 | 58 | 18 | 0.48 |
| lX | pg/ml | 9.2E2 | 8.3E2 | 1.0E3 | 9.4E2 | 5.6E2 | 6.3E2 | 2.3E2 | 1.9E2 | 2.6E3 | 2.5E3 | 58 | 18 | 58 | 18 | 0.44 |
| lY | pg/ml | 1.9E1 | 1.7E1 | 2.1E1 | 1.9E1 | 1.8E1 | 1.3E1 | 1.0E-9 | 5.7E-1 | 1.2E2 | 4.5E1 | 58 | 18 | 58 | 18 | 0.49 |
| mE | pg/ml | 1.0E-9 | 3.5E-1 | 2.2E0 | 3.5E0 | 7.6E0 | 6.8E0 | 1.0E-9 | 1.0E-9 | 5.7E1 | 2.8E1 | 58 | 18 | 58 | 18 | 0.58 |
| mF | pg/ml | 9.3E-2 | 6.4E-1 | 7.6E0 | 3.0E0 | 3.5E1 | 4.1E0 | 1.0E-9 | 1.0E-9 | 2.5E2 | 1.3E1 | 58 | 18 | 58 | 18 | 0.61 |
| mH | pg/ml | 3.2E0 | 2.7E0 | 5.5E0 | 4.7E0 | 8.6E0 | 4.6E0 | 4.0E-1 | 5.4E-1 | 5.3E1 | 1.9E1 | 58 | 18 | 58 | 18 | 0.51 |
| mI | pg/ml | 1.0E-9 | 9.9E0 | 1.1E1 | 4.4E1 | 2.7E1 | 1.1E2 | 1.0E-9 | 1.0E-9 | 1.8E2 | 4.6E2 | 58 | 18 | 58 | 18 | 0.64 |
| mM | pg/ml | 3.2E1 | 4.5E1 | 8.3E1 | 7.7E1 | 1.6E2 | 1.1E2 | 1.0E-9 | 1.0E-9 | 1.1E3 | 4.7E2 | 58 | 18 | 58 | 18 | 0.53 |
| mP | pg/ml | 1.5E1 | 1.6E1 | 1.7E1 | 6.9E1 | 1.7E1 | 1.9E2 | 1.0E-9 | 7.5E0 | 1.2E2 | 8.1E2 | 57 | 18 | 57 | 18 | 0.60 |
| mS | pg/ml | 1.6E3 | 1.7E3 | 1.8E3 | 1.8E3 | 1.0E3 | 9.2E2 | 1.0E-9 | 1.0E-9 | 5.1E3 | 3.5E3 | 58 | 18 | 58 | 18 | 0.52 |
| mT | pg/ml | 5.5E1 | 5.8E1 | 1.3E2 | 2.1E2 | 2.4E2 | 4.3E2 | 1.0E1 | 1.6E1 | 1.4E3 | 1.7E3 | 57 | 18 | 57 | 18 | 0.50 |
| mU | pg/ml | 2.2E0 | 3.1E0 | 6.8E0 | 4.6E0 | 2.9E1 | 5.2E0 | 1.0E-9 | 6.1E-1 | 2.2E2 | 2.3E1 | 57 | 18 | 57 | 18 | 0.60 |
| mW | pg/ml | 2.2E3 | 1.9E3 | 2.4E3 | 3.2E3 | 1.3E3 | 3.2E3 | 1.0E-9 | 3.7E2 | 6.2E3 | 1.1E4 | 57 | 18 | 57 | 18 | 0.50 |
| mY | pg/ml | 6.1E2 | 8.3E2 | 8.3E2 | 1.4E3 | 9.9E2 | 1.8E3 | 1.0E-9 | 1.9E2 | 5.6E3 | 8.0E3 | 58 | 18 | 58 | 18 | 0.65 |
| mZ | pg/ml | 1.9E2 | 1.5E2 | 3.7E2 | 3.8E2 | 4.8E2 | 4.5E2 | 1.0E-9 | 1.1E1 | 3.1E3 | 1.4E3 | 57 | 18 | 57 | 18 | 0.46 |
| nA | pg/ml | 1.5E0 | 3.0E0 | 7.0E0 | 6.8E0 | 1.4E1 | 1.3E1 | 1.0E-9 | 1.0E-9 | 6.5E1 | 5.7E1 | 57 | 18 | 57 | 18 | 0.51 |
| nB | pg/ml | 2.8E2 | 3.0E2 | 3.1E2 | 3.5E2 | 1.7E2 | 2.0E2 | 3.0E1 | 1.3E2 | 8.2E2 | 9.6E2 | 58 | 18 | 58 | 18 | 0.54 |
| nC | pg/ml | 1.0E-9 | 2.3E2 | 1.1E4 | 2.6E3 | 5.8E4 | 5.2E3 | 1.0E-9 | 1.0E-9 | 3.8E5 | 2.0E4 | 58 | 18 | 58 | 18 | 0.69 |
| nD | pg/ml | 6.7E0 | 8.9E0 | 1.3E1 | 1.6E1 | 3.4E1 | 2.6E1 | 1.0E-9 | 1.0E-9 | 2.6E2 | 1.2E2 | 57 | 18 | 57 | 18 | 0.57 |
| nF | pg/ml | 1.0E-9 | 1.0E-9 | 3.0E0 | 3.4E0 | 1.3E1 | 1.1E1 | 1.0E-9 | 1.0E-9 | 9.3E1 | 4.7E1 | 58 | 18 | 58 | 18 | 0.51 |
| nH | pg/ml | 1.0E-9 | 4.7E0 | 2.2E2 | 4.0E1 | 1.4E3 | 8.2E1 | 1.0E-9 | 1.0E-9 | 1.0E4 | 3.3E2 | 57 | 18 | 57 | 18 | 0.69 |
| nI | pg/ml | 4.6E1 | 1.0E-9 | 6.2E1 | 8.6E1 | 7.9E1 | 2.8E2 | 1.0E-9 | 1.0E-9 | 3.5E2 | 1.2E3 | 58 | 18 | 58 | 18 | 0.38 |
| nJ | pg/ml | 1.7E-1 | 8.8E-1 | 3.2E0 | 1.7E0 | 1.7E1 | 3.5E0 | 1.0E-9 | 1.0E-9 | 1.3E2 | 1.5E1 | 58 | 18 | 58 | 18 | 0.59 |
| nK | pg/ml | 1.0E-9 | 1.3E1 | 1.1E1 | 3.2E1 | 2.1E1 | 5.5E1 | 1.0E-9 | 1.0E-9 | 1.0E2 | 2.2E2 | 57 | 18 | 57 | 18 | 0.64 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| nL | pg/ml | 1.0E-9 | 7.2E0 | 3.2E2 | 1.2E2 | 1.9E3 | 2.6E2 | 1.0E-9 | 1.0E-9 | 1.4E4 | 9.0E2 | 58 | 18 | 58 | 18 | 0.66 |
| hL | pg/ml | 1.7E4 | 2.6E4 | 2.1E4 | 2.7E4 | 1.5E4 | 1.6E4 | 2.6E3 | 7.9E3 | 7.2E4 | 6.0E4 | 46 | 9 | 46 | 9 | 0.62 |
| hO | pg/ml | 1.6E4 | 1.5E4 | 1.6E4 | 1.5E4 | 2.6E3 | 2.2E3 | 1.3E4 | 1.1E4 | 2.4E4 | 1.8E4 | 46 | 9 | 46 | 9 | 0.33 |
| hP | ng/ml | 4.4E5 | 4.4E5 | 4.5E5 | 6.5E5 | 3.7E5 | 8.2E5 | 1.7E4 | 3.4E4 | 2.6E6 | 2.8E6 | 46 | 9 | 46 | 9 | 0.51 |
| wJ | pg/ml | 1.5E5 | 8.8E4 | 1.8E5 | 9.4E4 | 1.2E5 | 5.5E4 | 1.1E4 | 2.3E4 | 5.8E5 | 2.3E5 | 47 | 10 | 47 | 10 | 0.26 |
| wK | pg/ml | 3.3E4 | 2.9E4 | 5.2E4 | 3.7E4 | 7.3E4 | 2.8E4 | 3.7E3 | 1.1E4 | 5.0E5 | 1.0E5 | 47 | 10 | 47 | 10 | 0.41 |
| wL | pg/ml | 6.7E0 | 6.5E-1 | 5.2E1 | 4.0E1 | 1.5E2 | 1.1E2 | 1.0E-9 | 1.0E-9 | 8.4E2 | 3.5E2 | 47 | 10 | 47 | 10 | 0.39 |
| wP | pg/ml | 2.4E4 | 4.9E4 | 4.1E4 | 8.2E4 | 4.5E4 | 9.0E4 | 1.1E3 | 1.8E4 | 2.1E5 | 3.0E5 | 47 | 10 | 47 | 10 | 0.68 |
| wQ | pg/ml | 3.1E1 | 3.7E1 | 6.6E1 | 3.4E1 | 1.0E2 | 2.2E1 | 1.0E-9 | 1.0E-9 | 5.1E2 | 6.6E1 | 47 | 10 | 47 | 10 | 0.46 |
| hR | pg/ml | 2.7E4 | 2.4E4 | 2.9E4 | 2.7E4 | 1.2E4 | 1.0E4 | 1.0E-9 | 1.4E4 | 5.8E4 | 4.5E4 | 73 | 18 | 73 | 18 | 0.43 |
| hV | pg/ml | 4.7E2 | 3.7E2 | 4.7E2 | 3.8E2 | 2.4E2 | 1.9E2 | 1.0E-9 | 9.5E1 | 1.2E3 | 7.4E2 | 73 | 18 | 73 | 18 | 0.40 |
| hW | pg/ml | 1.7E3 | 2.4E3 | 2.1E3 | 4.5E3 | 1.2E3 | 8.9E3 | 1.0E-9 | 7.1E2 | 7.3E3 | 4.0E4 | 73 | 18 | 73 | 18 | 0.64 |
| hX | pg/ml | 1.0E3 | 1.2E3 | 1.1E3 | 1.2E3 | 9.7E2 | 6.1E2 | 2.5E0 | 3.1E2 | 8.6E3 | 2.9E3 | 73 | 18 | 73 | 18 | 0.59 |
| iA | pg/ml | 1.5E2 | 1.9E2 | 2.4E2 | 3.0E2 | 2.8E2 | 2.5E2 | 1.5E1 | 5.6E1 | 1.8E3 | 8.7E2 | 96 | 23 | 96 | 23 | 0.60 |
| iB | ng/ml | 4.8E0 | 7.0E0 | 5.8E0 | 9.6E0 | 4.4E0 | 6.3E0 | 3.3E-2 | 2.4E0 | 2.4E1 | 2.3E1 | 78 | 20 | 78 | 20 | 0.70 |
| iC | U/ml | 2.7E-1 | 6.2E-1 | 1.3E0 | 8.3E-1 | 6.3E0 | 8.1E-1 | 1.0E-9 | 6.8E-2 | 5.5E1 | 3.2E0 | 78 | 20 | 78 | 20 | 0.71 |
| tQ | pg/ml | 1.3E3 | 1.7E3 | 1.4E3 | 1.7E3 | 5.4E2 | 7.4E2 | 3.7E2 | 8.4E2 | 2.5E3 | 3.3E3 | 44 | 9 | 44 | 9 | 0.58 |
| tT | pg/ml | 1.9E1 | 2.3E1 | 2.0E1 | 3.1E1 | 9.9E0 | 2.6E1 | 5.4E0 | 1.1E1 | 4.8E1 | 9.3E1 | 45 | 9 | 45 | 9 | 0.65 |
| tS | pg/ml | 1.1E0 | 6.9E-1 | 1.4E0 | 1.7E0 | 1.7E0 | 3.1E0 | 1.0E-9 | 1.0E-9 | 8.5E0 | 1.0E1 | 45 | 10 | 45 | 10 | 0.45 |
| tX | pg/ml | 1.0E0 | 1.6E0 | 1.4E0 | 3.4E0 | 1.3E0 | 3.6E0 | 2.5E-2 | 3.7E-1 | 7.0E0 | 1.0E1 | 45 | 9 | 45 | 9 | 0.64 |
| tO | pg/ml | 4.4E0 | 3.3E0 | 5.3E0 | 4.7E0 | 3.6E0 | 4.0E0 | 1.0E-9 | 1.7E0 | 1.8E1 | 1.5E1 | 45 | 10 | 45 | 10 | 0.40 |
| tR | pg/ml | 2.2E-1 | 2.2E-1 | 3.3E-1 | 4.3E-1 | 3.8E-1 | 7.5E-1 | 1.0E-9 | 1.4E-2 | 1.6E0 | 2.5E0 | 44 | 10 | 44 | 10 | 0.49 |
| tU | pg/ml | 9.9E0 | 1.3E1 | 1.2E1 | 1.9E1 | 1.1E1 | 2.2E1 | 2.2E-1 | 4.9E0 | 5.5E1 | 8.0E1 | 46 | 10 | 46 | 10 | 0.61 |
| tN | pg/ml | 2.0E1 | 2.2E1 | 2.8E1 | 4.8E1 | 2.6E1 | 5.0E1 | 1.0E-9 | 9.5E0 | 1.5E2 | 1.6E2 | 44 | 9 | 44 | 9 | 0.61 |
| tV | ng/ml | 5.4E2 | 9.5E2 | 7.3E2 | 1.1E3 | 5.7E2 | 8.2E2 | 5.3E1 | 3.9E2 | 2.9E3 | 3.1E3 | 47 | 9 | 47 | 9 | 0.66 |
| iH | ng/ml | 1.6E5 | 1.9E5 | 1.6E5 | 1.8E5 | 4.9E4 | 5.3E4 | 2.9E3 | 7.7E4 | 2.6E5 | 2.5E5 | 96 | 23 | 96 | 23 | 0.64 |
| iJ | ng/ml | 5.2E4 | 3.9E4 | 5.6E4 | 4.9E4 | 3.7E4 | 2.9E4 | 1.8E3 | 1.2E4 | 2.5E5 | 1.5E5 | 96 | 23 | 96 | 23 | 0.42 |
| hB | ng/ml | 4.3E-1 | 6.3E-1 | 5.6E-1 | 9.7E-1 | 4.0E-1 | 7.2E-1 | 1.2E-1 | 2.9E-1 | 2.3E0 | 3.2E0 | 96 | 23 | 96 | 23 | 0.75 |
| hC | pg/ml | 4.0E3 | 1.0E4 | 7.7E3 | 1.0E4 | 1.2E4 | 1.2E4 | 4.1E1 | 3.0E2 | 1.1E5 | 5.7E4 | 96 | 23 | 96 | 23 | 0.60 |
| hF | ng/ml | 1.0E-9 | 1.0E-9 | 4.2E1 | 1.0E-9 | 4.1E2 | 0.0E0 | 1.0E-9 | 1.0E-9 | 4.0E3 | 1.0E-9 | 96 | 23 | 96 | 23 | 0.49 |
| hG | pg/ml | 6.8E3 | 6.7E3 | 7.4E3 | 8.3E3 | 3.1E3 | 4.3E3 | 1.8E3 | 3.6E3 | 1.8E4 | 2.0E4 | 96 | 23 | 96 | 23 | 0.53 |
| iO | ng/ml | 3.8E5 | 3.2E5 | 4.3E5 | 3.8E5 | 2.0E5 | 1.9E5 | 1.1E4 | 9.8E4 | 1.1E6 | 8.2E5 | 96 | 23 | 96 | 23 | 0.43 |
| iP | ng/ml | 5.3E4 | 4.6E4 | 5.9E4 | 5.1E4 | 5.0E4 | 2.1E4 | 1.0E-9 | 2.1E4 | 4.4E5 | 9.7E4 | 96 | 23 | 96 | 23 | 0.46 |
| iZ | ng/ml | 1.6E3 | 2.0E3 | 1.8E3 | 2.3E3 | 8.4E2 | 1.0E3 | 6.6E2 | 1.1E3 | 5.7E3 | 4.6E3 | 94 | 23 | 94 | 23 | 0.67 |
| yH | pg/ml | 9.3E2 | 1.4E3 | 2.4E3 | 1.9E3 | 5.3E3 | 1.9E3 | 1.0E-9 | 7.0E2 | 2.5E4 | 6.8E3 | 46 | 9 | 46 | 9 | 0.64 |
| yK | U/ml | 1.9E1 | 2.8E1 | 4.0E1 | 4.0E1 | 7.3E1 | 4.3E1 | 1.0E-9 | 1.0E-9 | 3.8E2 | 1.4E2 | 46 | 9 | 46 | 9 | 0.59 |
| yJ | pg/ml | 3.3E4 | 4.0E4 | 4.0E4 | 4.3E4 | 2.8E4 | 2.3E4 | 1.9E3 | 1.1E4 | 1.4E5 | 8.2E4 | 46 | 9 | 46 | 9 | 0.56 |
| yD | ng/ml | 1.3E-2 | 1.3E-2 | 1.3E-2 | 1.3E-2 | 5.9E-3 | 5.5E-3 | 1.0E-9 | 6.3E-3 | 2.8E-2 | 2.4E-2 | 47 | 10 | 47 | 10 | 0.51 |
| jB | ng/ml | 2.5E5 | 1.9E5 | 2.5E5 | 1.9E5 | 7.6E4 | 4.3E4 | 9.9E4 | 1.3E5 | 3.6E5 | 2.4E5 | 26 | 7 | 26 | 7 | 0.26 |
| wB | pg/ml | 8.7E3 | 2.2E4 | 9.8E3 | 2.1E4 | 6.9E3 | 1.1E4 | 1.7E3 | 5.3E3 | 3.3E4 | 4.2E4 | 47 | 10 | 47 | 10 | 0.80 |
| pY | pg/ml | 5.9E0 | 6.4E0 | 1.0E1 | 7.4E0 | 2.8E1 | 3.8E0 | 1.6E0 | 4.4E0 | 2.0E2 | 1.7E1 | 46 | 9 | 46 | 9 | 0.61 |
| rC | pg/ml | 1.5E3 | 1.0E3 | 2.3E3 | 1.7E3 | 2.4E3 | 1.7E3 | 1.0E-9 | 7.0E1 | 1.5E4 | 7.3E3 | 70 | 18 | 70 | 18 | 0.41 |
| rB | pg/ml | 3.0E1 | 5.9E1 | 4.8E1 | 7.4E1 | 6.3E1 | 8.4E1 | 1.0E-9 | 1.0E-9 | 3.9E2 | 3.2E2 | 70 | 18 | 70 | 18 | 0.58 |
| zG | 2.5ng/ml | 2.2E-1 | 3.8E-1 | 5.6E-1 | 6.3E-1 | 1.0E0 | 8.6E-1 | 1.0E-9 | 1.0E-9 | 4.8E0 | 2.7E0 | 46 | 9 | 46 | 9 | 0.59 |
| zH | 2.3mU/ml | 9.0E-2 | 8.5E-2 | 1.0E-1 | 8.4E-2 | 4.9E-2 | 2.6E-2 | 1.0E-2 | 4.4E-2 | 3.1E-1 | 1.2E-1 | 46 | 9 | 46 | 9 | 0.39 |
| zI | 2.6ng/ml | 2.1E0 | 3.4E0 | 4.2E0 | 6.0E0 | 5.8E0 | 5.9E0 | 5.4E-1 | 9.1E-1 | 2.7E1 | 1.6E1 | 46 | 9 | 46 | 9 | 0.62 |
| qA | ng/ml | 1.0E7 | 1.3E7 | 1.2E7 | 1.4E7 | 7.3E6 | 7.7E6 | 3.7E6 | 4.3E6 | 3.9E7 | 3.0E7 | 46 | 9 | 46 | 9 | 0.62 |
| qB | ng/ml | 6.9E5 | 5.7E5 | 8.3E5 | 9.9E5 | 5.5E5 | 1.1E6 | 1.9E5 | 2.4E5 | 2.9E6 | 3.8E6 | 46 | 9 | 46 | 9 | 0.44 |
| qC | ng/ml | 3.6E5 | 2.4E5 | 7.6E5 | 2.4E5 | 1.1E6 | 1.2E5 | 2.5E4 | 6.5E4 | 5.2E6 | 5.0E5 | 46 | 9 | 46 | 9 | 0.33 |
| qD | ng/ml | 1.6E7 | 1.4E7 | 1.8E7 | 1.4E7 | 8.9E6 | 5.1E6 | 1.2E6 | 1.0E7 | 4.5E7 | 2.6E7 | 46 | 9 | 46 | 9 | 0.38 |
| jD | ng/ml | 3.1E1 | 4.0E1 | 4.0E1 | 8.0E1 | 4.0E1 | 1.2E2 | 1.0E-9 | 1.0E-9 | 1.9E2 | 5.1E2 | 78 | 20 | 78 | 20 | 0.56 |
| jE | ng/ml | 1.0E-9 | 1.0E-9 | 5.7E0 | 5.9E0 | 1.2E1 | 1.1E1 | 1.0E-9 | 1.0E-9 | 6.2E1 | 3.5E1 | 78 | 20 | 78 | 20 | 0.51 |
| jF | ng/ml | 4.5E1 | 3.8E0 | 5.4E1 | 2.3E1 | 5.5E1 | 3.6E1 | 1.0E-9 | 1.0E-9 | 2.1E2 | 1.2E2 | 78 | 20 | 78 | 20 | 0.33 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| jG | ng/ml | 4.4E3 | 4.2E3 | 4.6E3 | 4.3E3 | 2.0E3 | 1.8E3 | 6.7E2 | 1.5E3 | 9.6E3 | 7.9E3 | 78 | 20 | 78 | 20 | 0.47 |
| jH | ng/ml | 7.9E1 | 6.2E1 | 8.3E1 | 9.8E1 | 4.3E1 | 9.4E1 | 1.3E1 | 1.5E1 | 2.4E2 | 4.3E2 | 78 | 20 | 78 | 20 | 0.46 |
| jI | ng/ml | 7.3E1 | 9.8E1 | 7.5E1 | 1.2E2 | 3.2E1 | 9.6E1 | 1.9E1 | 4.4E1 | 1.9E2 | 4.4E2 | 78 | 20 | 78 | 20 | 0.68 |
| sK | pg/mL | 3.9E3 | 3.6E3 | 4.1E3 | 5.9E3 | 1.8E3 | 6.7E3 | 1.8E3 | 2.1E3 | 1.0E4 | 2.3E4 | 44 | 9 | 44 | 9 | 0.51 |
| sM | pg/mL | 7.2E4 | 7.9E4 | 7.8E4 | 9.3E4 | 2.9E4 | 4.3E4 | 3.9E4 | 5.1E4 | 1.6E5 | 2.0E5 | 44 | 9 | 44 | 9 | 0.64 |
| sO | pg/mL | 2.3E8 | 1.9E8 | 2.5E8 | 1.9E8 | 8.8E7 | 9.6E7 | 4.9E7 | 6.6E7 | 4.4E8 | 3.2E8 | 44 | 9 | 44 | 9 | 0.34 |
| wC | ng/ml | 1.5E0 | 1.7E0 | 1.9E0 | 1.7E0 | 1.4E0 | 1.2E0 | 3.6E-1 | 6.1E-2 | 6.5E0 | 4.3E0 | 47 | 10 | 47 | 10 | 0.51 |
| wD | ng/ml | 2.1E1 | 4.4E1 | 7.9E1 | 8.6E1 | 3.1E2 | 8.7E1 | 2.8E0 | 2.1E1 | 2.1E3 | 2.9E2 | 47 | 10 | 47 | 10 | 0.77 |
| wE | ng/ml | 4.9E1 | 4.1E1 | 5.1E1 | 4.7E1 | 2.0E1 | 2.0E1 | 8.1E0 | 2.0E1 | 9.4E1 | 8.9E1 | 47 | 10 | 47 | 10 | 0.41 |
| wG | ng/ml | 1.1E-1 | 5.8E-2 | 1.4E-1 | 1.5E-1 | 1.5E-1 | 2.3E-1 | 1.0E-9 | 1.0E-9 | 7.1E-1 | 6.8E-1 | 47 | 10 | 47 | 10 | 0.42 |
| wH | ng/ml | 3.3E-2 | 4.3E-2 | 2.3E-1 | 9.2E-1 | 6.6E-1 | 1.8E0 | 1.0E-9 | 1.0E-9 | 4.2E0 | 5.6E0 | 47 | 10 | 47 | 10 | 0.58 |
| wF | ng/ml | 1.8E-1 | 6.2E-1 | 2.4E0 | 1.8E0 | 9.3E0 | 2.0E0 | 1.0E-9 | 1.0E-9 | 5.7E1 | 4.7E0 | 47 | 10 | 47 | 10 | 0.64 |
| rA | pg/ml | 2.4E1 | 2.2E1 | 3.0E1 | 2.9E1 | 2.9E1 | 2.0E1 | 1.0E-9 | 5.8E0 | 2.0E2 | 7.2E1 | 75 | 20 | 75 | 20 | 0.52 |
| qZ | pg/ml | 4.6E1 | 6.6E1 | 5.8E2 | 3.6E2 | 2.2E3 | 8.9E2 | 2.8E-4 | 6.5E-4 | 1.0E4 | 3.4E3 | 57 | 14 | 57 | 14 | 0.53 |
| qY | pg/ml | 1.8E1 | 1.3E1 | 4.3E1 | 2.3E1 | 6.1E1 | 2.6E1 | 8.7E-1 | 2.1E0 | 3.3E2 | 9.8E1 | 75 | 20 | 75 | 20 | 0.41 |
| qX | pg/ml | 5.5E1 | 7.2E1 | 6.8E1 | 8.6E1 | 4.6E1 | 5.8E1 | 1.0E-9 | 2.3E1 | 2.3E2 | 2.1E2 | 75 | 20 | 75 | 20 | 0.57 |
| qW | pg/ml | 7.8E0 | 7.2E0 | 1.2E1 | 9.5E0 | 1.7E1 | 8.9E0 | 1.0E-9 | 1.0E-9 | 1.2E2 | 3.1E1 | 75 | 20 | 75 | 20 | 0.47 |
| qV | pg/ml | 1.7E3 | 2.1E3 | 2.4E3 | 2.7E3 | 1.9E3 | 2.3E3 | 1.0E2 | 1.7E2 | 1.1E4 | 9.6E3 | 75 | 20 | 75 | 20 | 0.52 |
| qU | pg/ml | 7.3E1 | 1.0E2 | 1.9E2 | 2.1E2 | 3.2E2 | 2.9E2 | 1.0E-9 | 1.0E-9 | 2.1E3 | 1.1E3 | 75 | 20 | 75 | 20 | 0.53 |
| qT | pg/ml | 3.9E1 | 4.4E1 | 6.6E1 | 6.2E1 | 7.6E1 | 4.7E1 | 1.0E-9 | 6.9E0 | 4.9E2 | 1.6E2 | 75 | 20 | 75 | 20 | 0.54 |
| qI | ng/ml | 6.2E4 | 5.9E4 | 6.3E4 | 5.9E4 | 3.1E4 | 2.6E4 | 1.0E4 | 2.5E4 | 1.6E5 | 9.8E4 | 43 | 9 | 43 | 9 | 0.48 |
| qH | ng/ml | 5.9E4 | 5.9E4 | 6.9E4 | 7.3E4 | 4.1E4 | 3.2E4 | 1.5E4 | 4.7E4 | 1.8E5 | 1.4E5 | 43 | 9 | 43 | 9 | 0.58 |
| qG | ng/ml | 1.9E5 | 1.7E5 | 1.9E5 | 1.7E5 | 7.3E4 | 4.8E4 | 3.4E4 | 9.9E4 | 4.2E5 | 2.3E5 | 43 | 9 | 43 | 9 | 0.41 |
| jK | ng/ml | 1.6E3 | 1.3E3 | 1.7E3 | 1.5E3 | 6.4E2 | 5.8E2 | 2.8E2 | 7.5E2 | 4.1E3 | 2.9E3 | 78 | 20 | 78 | 20 | 0.37 |
| jL | ng/ml | 2.0E2 | 2.4E2 | 2.7E2 | 2.8E2 | 2.1E2 | 1.5E2 | 5.6E1 | 1.2E2 | 9.6E2 | 6.3E2 | 78 | 20 | 78 | 20 | 0.58 |
| jM | ng/ml | 7.3E4 | 6.0E4 | 7.7E4 | 6.5E4 | 4.0E4 | 3.8E4 | 4.6E3 | 1.1E4 | 1.8E5 | 1.4E5 | 78 | 20 | 78 | 20 | 0.42 |
| jO | pg/ml | 2.3E5 | 2.2E5 | 2.8E5 | 2.5E5 | 1.7E5 | 1.3E5 | 6.0E4 | 9.8E4 | 1.1E6 | 6.5E5 | 78 | 20 | 78 | 20 | 0.45 |
| jP | pg/ml | 2.6E5 | 2.4E5 | 2.8E5 | 3.1E5 | 1.4E5 | 1.7E5 | 3.6E4 | 1.3E5 | 7.1E5 | 5.8E5 | 78 | 20 | 78 | 20 | 0.52 |
| jQ | pg/ml | 2.4E3 | 1.6E3 | 3.3E3 | 2.4E3 | 3.0E3 | 2.2E3 | 5.0E0 | 2.9E2 | 1.3E4 | 9.2E3 | 78 | 20 | 78 | 20 | 0.42 |
| jR | pg/ml | 5.9E3 | 4.2E3 | 1.1E4 | 8.0E3 | 1.3E4 | 1.1E4 | 1.0E-9 | 3.0E1 | 6.8E4 | 4.6E4 | 78 | 20 | 78 | 20 | 0.42 |
| jT | pg/ml | 1.8E5 | 1.5E5 | 1.8E5 | 1.5E5 | 7.3E4 | 5.3E4 | 7.1E4 | 7.5E4 | 5.5E5 | 2.5E5 | 78 | 20 | 78 | 20 | 0.36 |
| xA | pg/ml | 4.3E0 | 7.3E0 | 1.3E1 | 1.8E1 | 2.6E1 | 3.4E1 | 1.0E-9 | 1.0E-9 | 1.6E2 | 1.1E2 | 46 | 9 | 46 | 9 | 0.56 |
| yE | pg/ml | 8.1E1 | 9.1E1 | 8.2E1 | 1.0E2 | 3.3E1 | 4.2E1 | 6.4E0 | 6.3E1 | 1.5E2 | 2.0E2 | 46 | 9 | 46 | 9 | 0.62 |
| tM | pg/ml | 4.3E1 | 3.9E1 | 3.9E1 | 4.5E1 | 1.8E1 | 2.3E1 | 1.0E-9 | 1.6E1 | 8.3E1 | 9.9E1 | 46 | 9 | 46 | 9 | 0.53 |
| tL | pg/ml | 1.0E-9 | 1.0E-9 | 1.7E0 | 2.7E-1 | 9.9E0 | 6.1E-1 | 1.0E-9 | 1.0E-9 | 6.7E1 | 1.8E0 | 46 | 9 | 46 | 9 | 0.52 |
| jU | mIU/ml | 5.2E0 | 8.0E0 | 1.2E1 | 1.3E1 | 1.9E1 | 1.5E1 | 8.1E-2 | 1.2E0 | 1.1E2 | 5.3E1 | 78 | 20 | 78 | 20 | 0.57 |
| jV | mIU/ml | 1.9E0 | 1.7E0 | 4.0E0 | 3.7E0 | 5.7E0 | 4.6E0 | 2.7E-3 | 1.0E-1 | 3.2E1 | 1.8E1 | 78 | 20 | 78 | 20 | 0.48 |
| jY | ng/ml | 7.3E-4 | 3.5E-3 | 6.8E-3 | 1.0E-2 | 3.4E-2 | 2.1E-2 | 1.0E-9 | 1.0E-9 | 3.0E-1 | 9.4E-2 | 78 | 20 | 78 | 20 | 0.68 |
| kC | pg/ml | 1.0E2 | 9.2E1 | 2.0E2 | 1.3E2 | 3.9E2 | 1.3E2 | 2.1E1 | 3.6E1 | 2.7E3 | 5.9E2 | 58 | 18 | 58 | 18 | 0.43 |
| kE | pg/ml | 1.4E5 | 1.4E5 | 1.4E5 | 1.4E5 | 3.7E4 | 4.9E4 | 4.1E4 | 3.8E4 | 2.3E5 | 2.7E5 | 58 | 18 | 58 | 18 | 0.46 |
| kF | pg/mL | 6.6E1 | 5.9E1 | 6.8E1 | 7.1E1 | 2.3E1 | 3.1E1 | 2.7E1 | 4.0E1 | 1.5E2 | 1.4E2 | 58 | 18 | 58 | 18 | 0.48 |
| kG | pg/mL | 8.5E3 | 8.9E3 | 1.1E4 | 2.3E4 | 9.8E3 | 3.8E4 | 1.3E3 | 1.1E3 | 5.8E4 | 1.6E5 | 58 | 18 | 58 | 18 | 0.54 |
| kI | pg/ml | 2.1E2 | 1.8E2 | 2.1E2 | 2.2E2 | 1.1E2 | 1.3E2 | 4.4E1 | 1.0E-9 | 6.7E2 | 5.5E2 | 58 | 18 | 58 | 18 | 0.49 |
| kK | pg/ml | 1.2E2 | 1.2E2 | 1.8E2 | 1.8E2 | 2.7E2 | 1.4E2 | 6.4E0 | 2.9E1 | 1.9E3 | 5.5E2 | 58 | 18 | 58 | 18 | 0.57 |
| kN | pg/ml | 1.2E3 | 8.2E2 | 1.5E3 | 1.7E3 | 1.5E3 | 2.3E3 | 7.3E1 | 3.8E2 | 1.0E4 | 8.7E3 | 58 | 18 | 58 | 18 | 0.42 |
| kO | pg/ml | 7.2E3 | 6.6E3 | 1.0E4 | 7.4E3 | 1.9E4 | 2.8E3 | 3.7E3 | 4.4E3 | 1.5E5 | 1.4E4 | 58 | 18 | 58 | 18 | 0.45 |
| kP | pg/ml | 6.4E3 | 4.5E3 | 7.1E3 | 5.2E3 | 4.4E3 | 3.4E3 | 9.6E2 | 1.6E3 | 2.7E4 | 1.5E4 | 58 | 18 | 58 | 18 | 0.36 |
| kQ | pg/ml | 4.4E3 | 4.3E3 | 5.3E3 | 5.6E3 | 4.0E3 | 4.3E3 | 5.6E2 | 1.4E3 | 2.5E4 | 2.2E4 | 96 | 23 | 96 | 23 | 0.52 |
| kR | pg/ml | 2.2E1 | 2.0E1 | 3.7E1 | 3.5E1 | 1.0E2 | 3.2E1 | 1.0E-9 | 2.9E0 | 1.0E3 | 1.1E2 | 96 | 23 | 96 | 23 | 0.52 |
| kS | pg/ml | 8.7E2 | 9.2E2 | 9.9E2 | 1.0E3 | 5.9E2 | 5.8E2 | 8.2E1 | 2.5E2 | 3.2E3 | 2.5E3 | 96 | 23 | 96 | 23 | 0.54 |
| pS | ng/ml | 1.6E5 | 1.2E5 | 1.9E5 | 1.3E5 | 1.0E5 | 5.5E4 | 7.5E4 | 6.8E4 | 5.7E5 | 2.6E5 | 44 | 9 | 44 | 9 | 0.23 |
| rZ | ng/ml | 1.4E-3 | 6.6E-3 | 6.5E-3 | 3.1E-2 | 1.5E-2 | 7.2E-2 | 1.0E-9 | 1.0E-9 | 9.4E-2 | 3.0E-1 | 71 | 18 | 71 | 18 | 0.65 |
| rY | ng/ml | 6.1E-2 | 7.3E-2 | 4.6E-1 | 1.1E0 | 2.5E0 | 4.2E0 | 1.0E-9 | 1.0E-9 | 2.0E1 | 1.8E1 | 71 | 18 | 71 | 18 | 0.58 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| rX | ng/ml | 1.0E-9 | 1.0E-9 | 1.1E-1 | 1.6E-1 | 5.7E-1 | 5.6E-1 | 1.0E-9 | 1.0E-9 | 3.7E0 | 2.4E0 | 71 | 18 | 71 | 18 | 0.60 |
| lK | pg/ml | 6.0E1 | 5.2E1 | 1.5E2 | 1.1E2 | 1.8E2 | 1.5E2 | 1.0E-9 | 1.0E-9 | 7.4E2 | 5.0E2 | 77 | 20 | 77 | 20 | 0.41 |
| lL | pg/ml | 1.6E3 | 1.8E3 | 3.0E3 | 2.6E3 | 5.2E3 | 2.1E3 | 7.5E1 | 5.3E2 | 4.2E4 | 7.7E3 | 78 | 20 | 78 | 20 | 0.55 |
| lM | pg/ml | 1.2E3 | 2.0E3 | 3.7E3 | 8.7E3 | 6.5E3 | 1.6E4 | 2.1E2 | 9.5E0 | 4.2E4 | 6.7E4 | 78 | 20 | 78 | 20 | 0.56 |
| lN | pg/ml | 1.0E-9 | 1.5E0 | 3.1E0 | 4.1E0 | 6.9E0 | 6.3E0 | 1.0E-9 | 1.0E-9 | 4.3E1 | 2.1E1 | 78 | 20 | 78 | 20 | 0.58 |
| lO | pg/ml | 1.0E-9 | 1.0E-9 | 2.1E0 | 6.9E0 | 1.5E1 | 3.1E1 | 1.0E-9 | 1.0E-9 | 1.3E2 | 1.4E2 | 77 | 20 | 77 | 20 | 0.51 |
| zA | ng/ml | 2.1E7 | 2.1E7 | 2.2E7 | 1.9E7 | 6.7E6 | 4.8E6 | 9.1E6 | 1.1E7 | 3.6E7 | 2.5E7 | 46 | 10 | 46 | 10 | 0.42 |
| rW | ng/ml | 1.1E-2 | 1.5E-2 | 3.5E-2 | 1.8E-2 | 6.2E-2 | 8.0E-3 | 1.0E-9 | 7.4E-3 | 3.2E-1 | 3.3E-2 | 43 | 9 | 43 | 9 | 0.59 |
| rV | ng/ml | 1.0E-9 | 6.7E-3 | 1.1E-2 | 2.9E-2 | 4.6E-2 | 5.0E-2 | 1.0E-9 | 1.0E-9 | 2.8E-1 | 1.5E-1 | 43 | 9 | 43 | 9 | 0.70 |
| rU | ng/ml | 7.1E-2 | 1.3E-1 | 1.7E-1 | 1.6E-1 | 4.2E-1 | 1.3E-1 | 1.0E-9 | 1.0E-9 | 2.7E0 | 4.0E-1 | 43 | 9 | 43 | 9 | 0.64 |
| rT | ng/ml | 6.1E0 | 6.8E0 | 6.8E0 | 7.6E0 | 4.5E0 | 5.6E0 | 6.5E-1 | 1.0E0 | 2.1E1 | 1.8E1 | 43 | 9 | 43 | 9 | 0.54 |
| rS | ng/ml | 3.5E0 | 9.0E0 | 5.4E0 | 2.4E1 | 5.1E0 | 2.5E1 | 7.6E-1 | 2.0E0 | 2.5E1 | 7.0E1 | 43 | 9 | 43 | 9 | 0.79 |
| sC | pg/mL | 5.4E3 | 7.9E3 | 1.1E4 | 1.0E4 | 1.4E4 | 7.7E3 | 1.7E3 | 3.4E3 | 7.4E4 | 2.8E4 | 44 | 9 | 44 | 9 | 0.61 |
| yL | pg/ml | 2.9E1 | 4.1E1 | 3.3E1 | 2.0E2 | 2.6E1 | 4.6E2 | 5.6E0 | 1.2E1 | 1.8E2 | 1.4E3 | 46 | 9 | 46 | 9 | 0.67 |
| rP | ng/ml | 1.2E2 | 2.1E2 | 2.0E2 | 2.8E2 | 2.0E2 | 2.2E2 | 1.0E-9 | 1.2E1 | 8.0E2 | 5.0E2 | 43 | 9 | 43 | 9 | 0.60 |
| rQ | ng/ml | 1.0E-9 | 1.0E-9 | 3.5E0 | 1.9E1 | 1.9E1 | 5.6E1 | 1.0E-9 | 1.0E-9 | 1.2E2 | 1.7E2 | 43 | 9 | 43 | 9 | 0.53 |
| rO | ng/ml | 2.0E-2 | 4.0E-3 | 4.1E-2 | 3.0E-2 | 6.9E-2 | 4.8E-2 | 1.0E-9 | 1.0E-9 | 4.0E-1 | 1.2E-1 | 43 | 9 | 43 | 9 | 0.42 |
| rR | ng/ml | 3.9E0 | 1.0E-9 | 9.2E0 | 1.8E1 | 1.8E1 | 3.2E1 | 1.0E-9 | 1.0E-9 | 7.9E1 | 9.5E1 | 43 | 9 | 43 | 9 | 0.51 |
| rN | ng/ml | 6.3E-1 | 1.3E0 | 8.0E-1 | 2.8E0 | 5.3E-1 | 4.0E0 | 5.1E-2 | 3.4E-1 | 2.3E0 | 1.3E1 | 43 | 9 | 43 | 9 | 0.72 |
| qO | pg/ml | 8.2E3 | 1.0E4 | 1.2E4 | 1.2E4 | 1.1E4 | 9.3E3 | 7.4E2 | 5.4E3 | 4.8E4 | 3.5E4 | 44 | 9 | 44 | 9 | 0.55 |
| qP | pg/ml | 3.5E2 | 3.6E2 | 4.2E2 | 3.9E2 | 2.9E2 | 2.0E2 | 7.0E1 | 1.5E2 | 1.5E3 | 7.8E2 | 44 | 9 | 44 | 9 | 0.52 |
| qQ | pg/ml | 1.5E1 | 3.3E1 | 2.3E1 | 2.6E1 | 5.6E1 | 1.8E1 | 1.0E-9 | 1.0E-9 | 2.8E2 | 4.3E1 | 44 | 9 | 44 | 9 | 0.71 |
| nW | pg/ml | 1.1E5 | 1.1E5 | 1.1E5 | 1.1E5 | 2.7E4 | 3.4E4 | 5.8E4 | 3.6E4 | 1.9E5 | 1.7E5 | 96 | 23 | 96 | 23 | 0.49 |
| nY | pg/ml | 2.2E3 | 3.5E3 | 2.6E3 | 3.2E3 | 1.5E3 | 1.8E3 | 5.1E2 | 6.3E2 | 1.0E4 | 8.1E3 | 96 | 23 | 96 | 23 | 0.63 |
| oO | pg/ml | 8.8E4 | 9.8E4 | 1.0E5 | 1.3E5 | 6.3E4 | 1.1E5 | 1.5E4 | 3.3E3 | 3.0E5 | 4.0E5 | 53 | 16 | 53 | 16 | 0.54 |
| oP | pg/ml | 1.2E5 | 1.6E5 | 1.4E5 | 1.9E5 | 8.4E4 | 1.4E5 | 2.4E4 | 2.4E4 | 4.2E5 | 5.7E5 | 53 | 16 | 53 | 16 | 0.61 |
| oQ | pg/ml | 2.9E3 | 3.9E3 | 3.5E3 | 6.6E3 | 3.1E3 | 7.8E3 | 7.7E2 | 9.1E2 | 2.1E4 | 3.2E4 | 53 | 16 | 53 | 16 | 0.65 |
| oE | pg/ml | 1.9E2 | 4.6E2 | 4.3E2 | 8.6E2 | 5.4E2 | 9.8E2 | 1.0E-9 | 1.0E-9 | 2.6E3 | 3.4E3 | 96 | 23 | 96 | 23 | 0.63 |
| oF | pg/ml | 1.1E4 | 3.0E4 | 2.4E4 | 4.8E4 | 3.7E4 | 4.8E4 | 3.5E2 | 6.5E2 | 2.5E5 | 1.7E5 | 96 | 23 | 96 | 23 | 0.70 |
| oH | pg/ml | 4.0E1 | 2.3E1 | 8.3E1 | 5.3E1 | 1.2E2 | 7.8E1 | 4.4E0 | 4.3E-1 | 8.6E2 | 3.1E2 | 96 | 23 | 96 | 23 | 0.39 |
| oK | pg/ml | 8.4E2 | 1.3E3 | 1.7E3 | 1.5E3 | 2.0E3 | 1.4E3 | 8.8E1 | 1.8E2 | 1.2E4 | 5.9E3 | 96 | 23 | 96 | 23 | 0.52 |
| oN | pg/ml | 5.4E2 | 5.8E2 | 1.0E3 | 7.3E2 | 2.1E3 | 4.0E2 | 1.1E2 | 2.8E2 | 1.8E4 | 1.8E3 | 96 | 23 | 96 | 23 | 0.59 |
| oW | pg/ml | 2.0E2 | 4.6E2 | 2.9E2 | 1.9E3 | 2.2E2 | 2.7E3 | 2.9E1 | 9.3E1 | 8.5E2 | 7.6E3 | 26 | 7 | 26 | 7 | 0.76 |
| oT | pg/ml | 2.9E2 | 2.6E2 | 3.4E2 | 2.9E2 | 1.9E2 | 1.2E2 | 1.0E2 | 1.5E2 | 7.9E2 | 5.4E2 | 26 | 7 | 26 | 7 | 0.43 |
| oV | pg/ml | 1.1E2 | 7.0E1 | 3.1E2 | 1.5E2 | 5.0E2 | 2.2E2 | 1.4E1 | 1.0E-9 | 2.2E3 | 6.3E2 | 26 | 7 | 26 | 7 | 0.38 |
| oD | pg/ml | 1.5E4 | 1.6E4 | 1.5E4 | 1.7E4 | 5.6E3 | 7.5E3 | 6.6E3 | 9.3E3 | 2.5E4 | 3.2E4 | 26 | 7 | 26 | 7 | 0.52 |
| uL | ng/ml | 3.7E1 | 4.3E1 | 5.0E1 | 4.4E1 | 4.6E1 | 2.5E1 | 1.5E1 | 1.4E1 | 2.9E2 | 8.0E1 | 43 | 9 | 43 | 9 | 0.50 |
| uO | ng/ml | 3.8E-1 | 4.8E-1 | 8.2E-1 | 8.6E-1 | 1.5E0 | 8.5E-1 | 1.0E-9 | 1.0E-9 | 9.3E0 | 2.1E0 | 43 | 9 | 43 | 9 | 0.57 |
| uM | ng/ml | 5.3E-1 | 6.2E-1 | 9.3E-1 | 7.6E-1 | 2.0E0 | 7.6E-1 | 1.0E-9 | 1.0E-9 | 1.3E1 | 2.5E0 | 43 | 9 | 43 | 9 | 0.52 |
| uI | ng/ml | 7.5E-2 | 5.1E-2 | 1.1E-1 | 1.0E-1 | 1.1E-1 | 1.2E-1 | 1.6E-2 | 1.5E-2 | 5.8E-1 | 3.8E-1 | 42 | 9 | 42 | 9 | 0.43 |
| uN | ng/ml | 1.5E1 | 1.7E1 | 1.6E1 | 2.0E1 | 6.3E0 | 9.7E0 | 8.0E0 | 1.0E1 | 3.6E1 | 4.1E1 | 43 | 9 | 43 | 9 | 0.63 |
| uG | ng/ml | 1.8E1 | 1.9E1 | 2.2E1 | 3.4E1 | 1.5E1 | 3.9E1 | 1.2E0 | 6.5E0 | 7.9E1 | 1.3E2 | 43 | 9 | 43 | 9 | 0.58 |
| uR | ng/ml | 2.3E0 | 1.7E0 | 2.9E0 | 2.3E0 | 2.3E0 | 1.8E0 | 7.5E-1 | 9.1E-1 | 1.3E1 | 6.6E0 | 46 | 9 | 46 | 9 | 0.38 |
| uP | ng/ml | 2.2E0 | 2.7E0 | 2.4E0 | 3.1E0 | 9.9E-1 | 1.7E0 | 1.2E0 | 9.3E-1 | 6.0E0 | 6.1E0 | 46 | 9 | 46 | 9 | 0.64 |
| uV | ng/ml | 1.7E-3 | 1.3E-3 | 1.5E-2 | 8.0E-3 | 3.3E-2 | 1.4E-2 | 1.0E-9 | 1.0E-9 | 2.0E-1 | 4.3E-2 | 46 | 9 | 46 | 9 | 0.46 |
| uT | ng/ml | 6.4E1 | 1.2E2 | 8.8E1 | 1.6E2 | 8.3E1 | 1.1E2 | 1.3E1 | 5.7E1 | 4.5E2 | 4.1E2 | 46 | 9 | 46 | 9 | 0.79 |
| uU | ng/ml | 1.8E0 | 1.7E0 | 1.9E0 | 3.8E0 | 1.2E0 | 6.0E0 | 5.2E-1 | 5.4E-1 | 6.0E0 | 2.0E1 | 46 | 9 | 46 | 9 | 0.54 |
| uW | ng/ml | 7.5E0 | 7.9E0 | 7.9E0 | 8.1E0 | 2.4E0 | 1.8E0 | 4.4E0 | 6.5E0 | 1.6E1 | 1.1E1 | 43 | 9 | 43 | 9 | 0.56 |
| vB | ng/ml | 3.0E0 | 3.2E0 | 3.4E0 | 2.9E0 | 2.3E0 | 8.4E-1 | 5.9E-1 | 1.3E0 | 1.0E1 | 3.8E0 | 43 | 9 | 43 | 9 | 0.50 |
| vC | ng/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 43 | 9 | 43 | 9 | 0.50 |
| uY | ng/ml | 6.1E-1 | 8.7E-1 | 9.1E-1 | 1.4E0 | 8.5E-1 | 1.3E0 | 6.8E-2 | 3.1E-1 | 3.8E0 | 4.4E0 | 43 | 9 | 43 | 9 | 0.65 |
| uZ | ng/ml | 5.8E-1 | 5.3E-1 | 7.5E-1 | 6.5E-1 | 8.1E-1 | 5.1E-1 | 1.0E-1 | 1.7E-1 | 4.9E0 | 1.9E0 | 43 | 9 | 43 | 9 | 0.46 |
| uX | ng/ml | 8.3E0 | 9.1E0 | 1.1E1 | 2.3E1 | 6.8E0 | 2.3E1 | 3.6E0 | 5.6E0 | 4.0E1 | 6.5E1 | 43 | 9 | 43 | 9 | 0.65 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| vA | ng/ml | 6.3E-2 | 6.6E-2 | 7.4E-2 | 1.2E-1 | 4.5E-2 | 1.3E-1 | 2.4E-2 | 2.5E-2 | 2.7E-1 | 4.2E-1 | 43 | 9 | 43 | 9 | 0.56 |
| vH | ng/ml | 1.2E-1 | 1.1E-1 | 1.5E-1 | 3.4E-1 | 1.3E-1 | 6.0E-1 | 2.0E-2 | 4.7E-2 | 6.6E-1 | 1.9E0 | 44 | 9 | 44 | 9 | 0.50 |
| vI | ng/ml | 1.8E0 | 3.5E0 | 2.2E0 | 3.9E0 | 1.9E0 | 2.7E0 | 6.3E-3 | 1.2E0 | 1.0E1 | 1.0E1 | 44 | 9 | 44 | 9 | 0.77 |
| vP | ng/ml | 3.8E2 | 2.9E2 | 4.7E2 | 4.3E2 | 4.4E2 | 3.2E2 | 6.7E1 | 1.5E2 | 2.4E3 | 1.1E3 | 46 | 9 | 46 | 9 | 0.53 |
| vT | ng/ml | 7.2E1 | 8.2E1 | 8.5E1 | 9.9E1 | 4.0E1 | 5.2E1 | 4.1E1 | 4.6E1 | 2.4E2 | 1.8E2 | 46 | 9 | 46 | 9 | 0.55 |
| vU | ng/ml | 1.0E-9 | 1.0E-9 | 2.7E1 | 1.9E1 | 4.0E1 | 3.7E1 | 1.0E-9 | 1.0E-9 | 1.3E2 | 1.1E2 | 46 | 9 | 46 | 9 | 0.44 |
| vQ | ng/ml | 4.0E2 | 4.5E2 | 4.2E2 | 4.0E2 | 1.6E2 | 1.8E2 | 7.2E1 | 1.9E2 | 8.4E2 | 6.5E2 | 46 | 9 | 46 | 9 | 0.47 |
| vO | ng/ml | 1.7E3 | 1.7E3 | 1.8E3 | 1.8E3 | 4.3E2 | 6.0E2 | 1.1E3 | 1.0E3 | 2.9E3 | 3.2E3 | 46 | 9 | 46 | 9 | 0.47 |
| vS | ng/ml | 1.3E3 | 1.3E3 | 1.2E3 | 1.3E3 | 4.3E2 | 5.7E2 | 1.0E-9 | 1.0E-9 | 2.1E3 | 1.9E3 | 46 | 9 | 46 | 9 | 0.56 |
| vV | ng/ml | 9.1E2 | 8.2E2 | 1.1E3 | 9.1E2 | 9.8E2 | 5.3E2 | 1.0E2 | 2.3E2 | 4.6E3 | 1.7E3 | 46 | 9 | 46 | 9 | 0.51 |
| vW | ng/ml | 1.1E2 | 2.4E2 | 1.5E2 | 2.6E2 | 1.1E2 | 2.2E2 | 4.3E1 | 6.0E1 | 6.6E2 | 7.7E2 | 46 | 9 | 46 | 9 | 0.68 |
| pF | pg/ml | 5.9E-1 | 5.7E-1 | 1.7E0 | 9.0E-1 | 8.9E0 | 7.5E-1 | 1.0E-9 | 1.8E-1 | 8.7E1 | 3.5E0 | 96 | 23 | 96 | 23 | 0.57 |
| pH | ng/ml | 7.4E0 | 1.3E1 | 8.1E0 | 1.5E1 | 3.9E0 | 5.7E0 | 1.2E0 | 6.8E0 | 1.8E1 | 2.3E1 | 26 | 7 | 26 | 7 | 0.85 |
| pI | ng/ml | 7.0E1 | 4.6E1 | 7.7E1 | 5.8E1 | 4.6E1 | 2.9E1 | 2.3E1 | 2.5E1 | 2.0E2 | 1.1E2 | 26 | 7 | 26 | 7 | 0.40 |
| pK | ng/ml | 4.5E-1 | 4.0E-1 | 4.6E-1 | 4.6E-1 | 1.9E-1 | 2.0E-1 | 1.7E-1 | 2.7E-1 | 8.6E-1 | 8.3E-1 | 26 | 7 | 26 | 7 | 0.51 |

Figure 29.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 8.1E1 | 8.1E1 | 9.4E1 | 7.5E1 | 6.8E1 | 4.2E1 | 7.0E0 | 1.6E1 | 4.8E2 | 1.5E2 | 286 | 9 | 286 | 9 | 0.43 |
| Ad | ug/mL | 4.7E-2 | 9.7E-2 | 1.2E-1 | 1.2E-1 | 6.6E-1 | 1.1E-1 | 6.8E-4 | 7.8E-4 | 8.5E0 | 3.5E-1 | 165 | 8 | 165 | 8 | 0.65 |
| Af | ng/mL | 1.2E0 | 1.4E0 | 1.0E1 | 3.0E0 | 4.5E1 | 4.1E0 | 1.7E-3 | 1.5E-1 | 5.3E2 | 1.2E1 | 165 | 8 | 165 | 8 | 0.45 |
| Aj | ug/mL | 1.1E0 | 7.5E-1 | 2.3E0 | 2.4E0 | 2.4E0 | 2.8E0 | 1.5E-3 | 1.3E-3 | 6.1E0 | 5.8E0 | 165 | 8 | 165 | 8 | 0.49 |
| Al | mg/mL | 8.3E-5 | 7.4E-4 | 2.6E-4 | 6.5E-4 | 4.3E-4 | 5.6E-4 | 4.3E-6 | 7.8E-6 | 1.8E-3 | 1.5E-3 | 165 | 8 | 165 | 8 | 0.64 |
| An | U/mL | 6.1E1 | 8.9E1 | 2.6E2 | 1.6E2 | 8.0E2 | 1.5E2 | 2.8E-1 | 2.2E1 | 7.8E3 | 4.6E2 | 165 | 8 | 165 | 8 | 0.61 |
| Ao | pg/mL | 9.4E1 | 3.9E2 | 5.3E2 | 9.7E2 | 3.6E3 | 1.5E3 | 1.5E0 | 4.1E0 | 3.9E4 | 4.5E3 | 165 | 8 | 165 | 8 | 0.65 |
| Ap | ng/mL | 3.3E1 | 1.0E2 | 4.7E1 | 1.2E2 | 5.2E1 | 9.7E1 | 2.0E0 | 4.4E0 | 3.3E2 | 2.4E2 | 165 | 8 | 165 | 8 | 0.71 |
| Ar | ng/mL | 7.0E-1 | 1.5E0 | 2.9E0 | 8.0E0 | 6.7E0 | 1.7E1 | 3.4E-3 | 2.2E-1 | 5.1E1 | 5.0E1 | 165 | 8 | 165 | 8 | 0.60 |
| As | ng/mL | 8.7E-3 | 1.7E-3 | 2.0E-2 | 6.2E-3 | 9.7E-2 | 6.6E-3 | 1.7E-3 | 1.7E-3 | 1.2E0 | 1.9E-2 | 165 | 8 | 165 | 8 | 0.38 |
| Aw | pg/mL | 1.6E1 | 2.3E1 | 1.7E1 | 2.3E1 | 6.2E0 | 4.6E0 | 2.9E-2 | 1.4E1 | 5.1E1 | 2.9E1 | 165 | 8 | 165 | 8 | 0.82 |
| Ax | ng/mL | 2.0E0 | 1.6E1 | 2.4E1 | 1.6E2 | 9.0E1 | 3.0E2 | 1.2E-2 | 1.0E-4 | 7.7E2 | 8.5E2 | 165 | 8 | 165 | 8 | 0.66 |
| Ba | ng/mL | 8.3E1 | 1.1E3 | 5.9E2 | 3.1E3 | 1.5E3 | 5.0E3 | 1.1E0 | 4.1E0 | 8.1E3 | 1.5E4 | 165 | 8 | 165 | 8 | 0.79 |
| Bb | ng/mL | 4.0E0 | 1.3E1 | 7.0E0 | 1.7E1 | 8.3E0 | 1.5E1 | 4.1E-3 | 3.5E-1 | 4.9E1 | 4.8E1 | 165 | 8 | 165 | 8 | 0.72 |
| Bc | ng/mL | 3.6E1 | 1.3E2 | 1.2E2 | 3.2E2 | 2.3E2 | 3.8E2 | 4.9E-1 | 2.9E0 | 1.2E3 | 9.9E2 | 165 | 8 | 165 | 8 | 0.68 |
| Bg | ng/mL | 1.1E-1 | 1.3E0 | 4.1E0 | 5.1E1 | 1.7E1 | 1.4E2 | 5.3E-4 | 3.1E-2 | 1.5E2 | 4.0E2 | 165 | 8 | 165 | 8 | 0.66 |
| Bn | ng/mL | 5.6E-2 | 3.5E-1 | 1.5E0 | 4.6E-1 | 4.9E0 | 4.6E-1 | 5.6E-2 | 5.6E-2 | 5.8E1 | 1.2E0 | 165 | 8 | 165 | 8 | 0.53 |
| Bo | ng/mL | 1.3E1 | 1.7E1 | 1.5E1 | 1.8E1 | 1.1E1 | 5.7E0 | 1.6E-2 | 9.7E0 | 5.3E1 | 2.6E1 | 165 | 8 | 165 | 8 | 0.62 |
| Ch | uIU/mL | 1.0E0 | 1.5E0 | 2.9E1 | 1.5E2 | 1.6E2 | 4.2E2 | 3.4E-3 | 5.9E-1 | 1.8E3 | 1.2E3 | 165 | 8 | 165 | 8 | 0.58 |
| Co | pg/mL | 4.7E1 | 1.1E2 | 2.2E2 | 4.3E2 | 1.3E3 | 6.9E2 | 1.5E-1 | 8.8E0 | 1.7E4 | 2.1E3 | 165 | 8 | 165 | 8 | 0.66 |
| Cp | ng/mL | 2.2E1 | 4.8E1 | 3.5E1 | 5.5E1 | 1.0E2 | 3.9E1 | 6.0E-1 | 1.1E1 | 1.3E3 | 1.4E2 | 165 | 8 | 165 | 8 | 0.74 |
| Cq | ng/mL | 3.0E-2 | 1.1E-1 | 4.0E-1 | 4.8E-1 | 3.8E0 | 8.1E-1 | 8.0E-4 | 8.0E-4 | 4.9E1 | 2.3E0 | 165 | 8 | 165 | 8 | 0.72 |
| Cs | ng/mL | 6.0E1 | 1.7E2 | 4.7E2 | 8.7E2 | 1.7E3 | 1.8E3 | 8.3E-1 | 5.7E0 | 1.8E4 | 5.1E3 | 165 | 8 | 165 | 8 | 0.62 |
| Ct | ng/mL | 3.1E-1 | 1.2E1 | 4.0E1 | 6.5E1 | 1.2E2 | 1.4E2 | 1.1E-4 | 1.1E-4 | 6.2E2 | 4.2E2 | 165 | 8 | 165 | 8 | 0.63 |
| Cu | ng/mL | 2.6E-1 | 2.6E0 | 8.8E-1 | 4.6E0 | 5.2E0 | 6.7E0 | 1.9E-2 | 1.7E-2 | 6.6E1 | 2.1E1 | 165 | 8 | 165 | 8 | 0.85 |
| Cv | ng/mL | 5.2E0 | 1.3E1 | 2.9E1 | 3.8E1 | 7.3E1 | 4.9E1 | 2.0E-2 | 1.0E-1 | 5.3E2 | 1.4E2 | 165 | 8 | 165 | 8 | 0.60 |
| Cw | mIU/mL | 3.6E-2 | 8.3E-2 | 8.3E-2 | 8.5E-2 | 5.2E-1 | 4.4E-2 | 8.9E-4 | 1.1E-2 | 6.8E0 | 1.5E-1 | 165 | 8 | 165 | 8 | 0.79 |
| Cx | ng/mL | 8.5E-1 | 3.8E-3 | 5.3E1 | 4.0E-1 | 1.0E2 | 6.3E-1 | 9.3E-5 | 9.3E-5 | 3.9E2 | 1.7E0 | 165 | 8 | 165 | 8 | 0.29 |
| Db | ug/mL | 7.5E0 | 7.4E0 | 8.8E0 | 8.5E0 | 7.7E0 | 3.7E0 | 4.5E-1 | 4.3E0 | 5.9E1 | 1.5E1 | 165 | 8 | 165 | 8 | 0.53 |
| Dc | nmol/L | 2.0E-2 | 1.7E-1 | 1.6E-1 | 2.9E-1 | 1.1E0 | 4.3E-1 | 5.2E-6 | 2.1E-3 | 1.4E1 | 1.3E0 | 165 | 8 | 165 | 8 | 0.79 |
| Dd | ug/mL | 7.1E-2 | 4.3E-1 | 1.9E-1 | 4.9E-1 | 3.6E-1 | 4.9E-1 | 4.8E-4 | 6.4E-3 | 3.6E0 | 1.5E0 | 165 | 8 | 165 | 8 | 0.69 |
| De | ng/mL | 3.4E-3 | 2.3E-1 | 7.5E-2 | 3.0E-1 | 1.4E-1 | 3.7E-1 | 3.4E-3 | 3.4E-3 | 9.2E-1 | 1.1E0 | 165 | 8 | 165 | 8 | 0.72 |
| Dg | ng/mL | 3.6E1 | 8.0E1 | 4.6E1 | 7.1E1 | 4.0E1 | 4.5E1 | 7.1E-1 | 1.9E0 | 1.9E2 | 1.2E2 | 165 | 8 | 165 | 8 | 0.66 |
| Di | pg/mL | 2.0E0 | 3.9E0 | 2.4E0 | 4.0E0 | 2.2E0 | 1.1E0 | 1.8E-1 | 2.7E0 | 1.3E1 | 5.4E0 | 165 | 8 | 165 | 8 | 0.78 |
| Dk | uIU/mL | 1.4E-2 | 3.2E-2 | 5.6E-2 | 1.9E-1 | 1.7E-1 | 3.4E-1 | 1.1E-4 | 6.6E-3 | 1.6E0 | 9.8E-1 | 165 | 8 | 165 | 8 | 0.63 |
| Dl | ng/mL | 2.0E2 | 3.4E2 | 2.9E2 | 4.2E2 | 2.8E2 | 3.9E2 | 5.5E0 | 4.4E0 | 1.6E3 | 1.1E3 | 165 | 8 | 165 | 8 | 0.58 |
| Wm | % | 8.5E-2 | 2.4E0 | 1.5E1 | 4.2E1 | 1.1E2 | 7.5E1 | 5.4E-2 | 8.5E-2 | 1.0E3 | 1.9E2 | 151 | 7 | 151 | 7 | 0.62 |
| Po | pg/ml | 3.0E-1 | 1.7E1 | 9.6E0 | 3.5E1 | 2.9E1 | 6.0E1 | 8.0E-3 | 2.6E-2 | 2.7E2 | 2.2E2 | 339 | 12 | 339 | 12 | 0.77 |
| Et | ng/ml | 1.4E3 | 3.1E3 | 1.7E3 | 2.9E3 | 1.2E3 | 1.5E3 | 7.5E1 | 5.9E2 | 4.8E3 | 5.0E3 | 338 | 12 | 338 | 12 | 0.72 |
| Fp | ng/ml | 1.3E1 | 2.6E1 | 2.4E1 | 3.3E1 | 2.7E1 | 3.5E1 | 6.0E-3 | 1.3E0 | 1.3E2 | 1.2E2 | 341 | 12 | 341 | 12 | 0.57 |
| Fr | ng/ml | 3.5E4 | 5.5E5 | 1.2E5 | 4.4E5 | 1.8E5 | 3.4E5 | 1.9E2 | 4.5E3 | 8.4E5 | 8.4E5 | 346 | 14 | 346 | 14 | 0.76 |
| Nm | pg/ml | 1.3E4 | 7.5E4 | 3.4E4 | 1.3E5 | 8.9E4 | 1.5E5 | 1.0E-9 | 1.0E-9 | 9.6E5 | 4.4E5 | 342 | 12 | 342 | 12 | 0.66 |
| Nn | pg/ml | 1.5E2 | 1.0E3 | 1.7E3 | 4.0E4 | 8.0E3 | 9.0E4 | 1.0E-9 | 1.0E-9 | 9.5E4 | 3.1E5 | 342 | 12 | 342 | 12 | 0.77 |
| No | pg/ml | 1.5E1 | 4.2E1 | 3.8E1 | 1.2E2 | 9.0E1 | 2.0E2 | 1.0E-9 | 1.6E0 | 9.1E2 | 7.0E2 | 342 | 12 | 342 | 12 | 0.70 |
| Nq | pg/ml | 1.9E0 | 2.1E1 | 2.0E1 | 1.3E2 | 7.3E1 | 1.8E2 | 1.0E-9 | 1.0E-9 | 6.7E2 | 5.9E2 | 342 | 12 | 342 | 12 | 0.72 |
| Nr | pg/ml | 1.6E0 | 1.1E1 | 2.3E1 | 1.6E2 | 8.3E1 | 3.9E2 | 1.0E-9 | 1.0E-9 | 9.8E2 | 1.4E3 | 342 | 12 | 342 | 12 | 0.68 |
| Ns | pg/ml | 1.0E-9 | 1.0E-9 | 7.8E0 | 3.0E-1 | 6.5E1 | 1.0E0 | 1.0E-9 | 1.0E-9 | 1.1E3 | 3.6E0 | 342 | 12 | 342 | 12 | 0.51 |
| Nt | pg/ml | 1.0E2 | 2.3E2 | 1.4E2 | 3.2E2 | 1.3E2 | 3.3E2 | 9.8E-1 | 6.4E1 | 1.7E3 | 1.2E3 | 342 | 12 | 342 | 12 | 0.75 |
| Nu | pg/ml | 1.7E1 | 9.7E1 | 5.6E1 | 1.1E2 | 9.3E1 | 8.3E1 | 1.0E-9 | 1.0E-9 | 6.3E2 | 2.9E2 | 342 | 12 | 342 | 12 | 0.74 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Lu | pg/ml | 1.0E4 | 1.0E4 | 1.5E4 | 1.1E4 | 4.0E4 | 7.5E3 | 5.2E2 | 1.4E3 | 5.6E5 | 2.7E4 | 342 | 12 | 342 | 12 | 0.53 |
| Lv | pg/ml | 1.0E-9 | 4.7E1 | 1.5E1 | 5.9E1 | 3.1E1 | 5.1E1 | 1.0E-9 | 1.0E-9 | 2.6E2 | 1.8E2 | 342 | 12 | 342 | 12 | 0.82 |
| Lw | pg/ml | 1.0E-9 | 1.0E-9 | 5.2E-1 | 2.0E1 | 5.2E0 | 5.1E1 | 1.0E-9 | 1.0E-9 | 8.0E1 | 1.8E2 | 342 | 12 | 342 | 12 | 0.66 |
| Lx | pg/ml | 1.0E-9 | 4.8E2 | 2.5E2 | 7.8E2 | 1.3E3 | 8.5E2 | 1.0E-9 | 1.0E-9 | 2.2E4 | 2.8E3 | 342 | 12 | 342 | 12 | 0.80 |
| Ly | pg/ml | 1.0E-9 | 1.0E-9 | 9.2E0 | 3.9E0 | 1.8E1 | 7.6E0 | 1.0E-9 | 1.0E-9 | 1.1E2 | 2.4E1 | 342 | 12 | 342 | 12 | 0.44 |
| Lz | pg/ml | 1.0E-9 | 1.0E-9 | 3.5E0 | 4.2E0 | 2.6E1 | 1.4E1 | 1.0E-9 | 1.0E-9 | 4.3E2 | 4.7E1 | 342 | 12 | 342 | 12 | 0.55 |
| Ma | pg/ml | 3.9E2 | 5.1E3 | 2.1E3 | 1.2E4 | 5.4E3 | 1.7E4 | 1.0E-9 | 2.4E1 | 6.5E4 | 5.2E4 | 342 | 12 | 342 | 12 | 0.69 |
| Mb | pg/ml | 2.5E1 | 2.7E1 | 3.2E1 | 3.3E1 | 1.8E1 | 1.6E1 | 4.1E0 | 1.6E1 | 2.1E2 | 5.8E1 | 342 | 12 | 342 | 12 | 0.51 |
| Mc | pg/ml | 1.0E-9 | 1.0E-9 | 5.6E-2 | 1.0E-9 | 7.4E-1 | 0.0E0 | 1.0E-9 | 1.0E-9 | 1.3E1 | 1.0E-9 | 342 | 12 | 342 | 12 | 0.49 |
| Md | pg/ml | 1.0E-9 | 1.0E-9 | 6.8E-1 | 3.9E-1 | 5.5E0 | 1.2E0 | 1.0E-9 | 1.0E-9 | 6.8E1 | 4.0E0 | 342 | 12 | 342 | 12 | 0.54 |
| Me | pg/ml | 3.1E1 | 2.9E1 | 3.1E1 | 3.7E1 | 2.4E1 | 4.9E1 | 1.0E-9 | 1.0E-9 | 3.2E1 | 1.8E2 | 342 | 12 | 342 | 12 | 0.44 |
| Mf | pg/ml | 1.0E-9 | 1.0E-9 | 6.1E-1 | 9.0E-1 | 3.9E0 | 1.8E0 | 1.0E-9 | 1.0E-9 | 5.6E1 | 5.0E0 | 342 | 12 | 342 | 12 | 0.60 |
| Mg | pg/ml | 9.4E-1 | 8.8E0 | 6.2E0 | 2.7E1 | 1.2E1 | 4.7E1 | 1.0E-9 | 1.0E-9 | 9.2E1 | 1.5E2 | 342 | 12 | 342 | 12 | 0.61 |
| Mh | pg/ml | 1.0E-9 | 2.0E-2 | 1.2E0 | 1.9E0 | 7.6E0 | 5.2E0 | 1.0E-9 | 1.0E-9 | 1.1E2 | 1.8E1 | 342 | 12 | 342 | 12 | 0.61 |
| Mi | pg/ml | 1.0E-9 | 2.0E0 | 1.9E0 | 5.9E1 | 1.9E1 | 1.5E2 | 1.0E-9 | 1.0E-9 | 3.2E2 | 5.2E2 | 342 | 12 | 342 | 12 | 0.73 |
| Mj | pg/ml | 1.0E-9 | 9.8E-1 | 6.2E0 | 3.6E1 | 3.2E1 | 6.5E1 | 1.0E-9 | 1.0E-9 | 4.6E2 | 2.1E2 | 342 | 12 | 342 | 12 | 0.67 |
| Mk | pg/ml | 1.5E0 | 5.6E0 | 1.5E1 | 5.0E1 | 9.2E1 | 1.4E2 | 1.0E-9 | 1.0E-9 | 1.3E3 | 5.0E2 | 342 | 12 | 342 | 12 | 0.63 |
| Ml | pg/ml | 1.0E-9 | 1.0E-9 | 1.1E1 | 1.9E-1 | 1.2E2 | 6.6E-1 | 1.0E-9 | 1.0E-9 | 2.1E3 | 2.3E0 | 342 | 12 | 342 | 12 | 0.43 |
| Mm | pg/ml | 5.5E2 | 2.0E3 | 1.0E3 | 3.1E3 | 1.3E3 | 3.2E3 | 1.0E-9 | 1.0E-9 | 1.0E4 | 1.0E4 | 342 | 12 | 342 | 12 | 0.68 |
| Mn | pg/ml | 6.0E0 | 1.3E1 | 1.1E1 | 1.8E1 | 2.5E1 | 1.5E1 | 1.0E-9 | 1.1E0 | 3.5E2 | 5.1E1 | 342 | 12 | 342 | 12 | 0.71 |
| Mp | pg/ml | 1.0E-9 | 2.5E1 | 1.3E1 | 2.6E2 | 5.0E1 | 6.7E2 | 1.0E-9 | 1.0E-9 | 6.3E2 | 2.4E3 | 342 | 12 | 342 | 12 | 0.75 |
| Mq | pg/ml | 1.0E-9 | 1.0E-9 | 2.4E0 | 1.4E1 | 1.4E1 | 2.6E1 | 1.0E-9 | 1.0E-9 | 1.8E2 | 7.4E1 | 342 | 12 | 342 | 12 | 0.59 |
| Mr | pg/ml | 1.0E-9 | 5.4E0 | 3.2E1 | 3.6E2 | 1.8E2 | 9.8E2 | 1.0E-9 | 1.0E-9 | 2.2E3 | 3.4E3 | 342 | 12 | 342 | 12 | 0.62 |
| Ms | pg/ml | 3.2E2 | 3.2E2 | 4.7E2 | 7.1E2 | 5.3E2 | 1.3E3 | 1.0E-9 | 1.0E-9 | 3.3E3 | 4.7E3 | 342 | 12 | 342 | 12 | 0.48 |
| Mt | pg/ml | 2.7E-1 | 1.1E1 | 1.8E1 | 5.2E1 | 1.8E2 | 8.9E1 | 1.0E-9 | 1.0E-9 | 3.2E3 | 3.1E2 | 342 | 12 | 342 | 12 | 0.74 |
| Mu | pg/ml | 1.0E-9 | 1.0E-9 | 1.5E0 | 8.2E0 | 1.4E1 | 1.6E1 | 1.0E-9 | 1.0E-9 | 2.3E2 | 4.8E1 | 342 | 12 | 342 | 12 | 0.65 |
| Mv | pg/ml | 1.0E-9 | 1.9E1 | 6.5E1 | 2.5E2 | 3.4E2 | 3.5E2 | 1.0E-9 | 1.0E-9 | 4.5E3 | 9.0E2 | 342 | 12 | 342 | 12 | 0.69 |
| Mw | pg/ml | 3.7E1 | 5.9E2 | 2.7E2 | 1.4E3 | 1.4E3 | 1.9E3 | 1.0E-9 | 1.0E-9 | 1.8E4 | 5.3E3 | 342 | 12 | 342 | 12 | 0.77 |
| Mx | pg/ml | 1.0E-9 | 1.0E-9 | 4.1E-1 | 8.9E-1 | 2.2E0 | 2.0E0 | 1.0E-9 | 1.0E-9 | 3.2E1 | 6.6E0 | 342 | 12 | 342 | 12 | 0.59 |
| My | pg/ml | 1.0E-9 | 1.2E2 | 3.2E2 | 4.8E2 | 2.4E3 | 7.1E2 | 1.0E-9 | 1.0E-9 | 3.9E4 | 1.9E3 | 342 | 12 | 342 | 12 | 0.70 |
| Mz | pg/ml | 1.1E1 | 9.1E1 | 3.5E1 | 8.7E1 | 1.3E2 | 7.0E1 | 1.0E-9 | 3.5E0 | 1.9E3 | 2.0E2 | 342 | 12 | 342 | 12 | 0.80 |
| Na | pg/ml | 1.0E-9 | 1.0E-9 | 7.6E-1 | 1.5E0 | 2.9E0 | 2.6E0 | 1.0E-9 | 1.0E-9 | 4.2E1 | 6.4E0 | 342 | 12 | 342 | 12 | 0.57 |
| Nb | pg/ml | 2.2E0 | 5.1E0 | 4.0E0 | 2.7E1 | 1.2E1 | 5.9E1 | 1.0E-9 | 1.0E-9 | 1.5E2 | 2.1E2 | 342 | 12 | 342 | 12 | 0.69 |
| Nc | pg/ml | 3.1E2 | 1.2E2 | 5.1E2 | 4.4E2 | 7.4E2 | 5.8E2 | 1.0E-9 | 1.0E-9 | 9.2E3 | 1.6E3 | 342 | 12 | 342 | 12 | 0.45 |
| Nd | pg/ml | 2.7E1 | 3.8E1 | 3.3E1 | 4.4E1 | 1.3E2 | 4.3E1 | 1.0E-9 | 7.2E-1 | 2.1E3 | 1.5E2 | 342 | 12 | 342 | 12 | 0.63 |
| Ne | pg/ml | 4.2E2 | 4.0E2 | 5.1E2 | 6.4E2 | 5.4E2 | 9.9E2 | 1.0E-9 | 1.0E-9 | 7.0E3 | 3.6E3 | 342 | 12 | 342 | 12 | 0.47 |
| Nf | pg/ml | 1.0E-9 | 1.0E-9 | 3.1E0 | 7.1E-1 | 1.3E1 | 1.7E0 | 1.0E-9 | 1.0E-9 | 1.3E2 | 4.8E0 | 342 | 12 | 342 | 12 | 0.47 |
| Ng | pg/ml | 1.2E1 | 3.6E1 | 9.0E1 | 7.2E1 | 1.8E2 | 8.6E1 | 1.0E-9 | 1.0E-9 | 1.6E3 | 2.6E2 | 342 | 12 | 342 | 12 | 0.53 |
| Nh | pg/ml | 6.1E1 | 3.7E1 | 7.9E1 | 8.2E1 | 7.2E1 | 1.4E2 | 1.0E-9 | 4.5E0 | 5.6E2 | 5.1E2 | 342 | 12 | 342 | 12 | 0.38 |
| Ni | pg/ml | 1.0E-9 | 1.0E-9 | 8.5E1 | 7.0E1 | 1.4E2 | 1.4E2 | 1.0E-9 | 1.0E-9 | 1.1E3 | 3.7E2 | 342 | 12 | 342 | 12 | 0.43 |
| Nj | pg/ml | 7.2E0 | 5.5E0 | 1.1E1 | 6.6E0 | 1.2E1 | 6.5E0 | 1.0E-9 | 1.0E-9 | 6.9E1 | 1.7E1 | 342 | 12 | 342 | 12 | 0.39 |
| Nk | pg/ml | 1.8E1 | 1.0E-9 | 3.2E1 | 2.4E1 | 3.9E1 | 3.4E1 | 1.0E-9 | 1.0E-9 | 2.0E2 | 8.7E1 | 342 | 12 | 342 | 12 | 0.40 |
| Nl | pg/ml | 4.2E1 | 3.0E1 | 5.6E1 | 4.4E1 | 7.5E1 | 5.6E1 | 1.0E-9 | 1.0E-9 | 1.1E3 | 1.8E2 | 342 | 12 | 342 | 12 | 0.40 |
| Hq | pg/ml | 1.2E0 | 7.2E0 | 1.6E2 | 2.6E1 | 1.9E3 | 5.2E1 | 1.0E-9 | 1.0E-9 | 2.8E4 | 1.8E2 | 340 | 12 | 340 | 12 | 0.68 |
| Hr | pg/ml | 9.1E1 | 1.6E2 | 6.6E2 | 4.6E2 | 1.4E3 | 9.6E2 | 1.0E-9 | 1.0E-9 | 1.2E4 | 3.4E3 | 340 | 12 | 340 | 12 | 0.48 |
| Hu | pg/ml | 1.1E1 | 5.4E2 | 4.6E3 | 1.2E3 | 4.0E4 | 1.7E3 | 1.0E-9 | 1.0E-9 | 6.3E5 | 5.9E3 | 340 | 12 | 340 | 12 | 0.65 |
| Hv | pg/ml | 1.4E0 | 2.0E0 | 5.2E0 | 1.9E1 | 4.9E1 | 4.7E1 | 1.0E-9 | 3.8E-1 | 8.9E2 | 1.6E2 | 340 | 12 | 340 | 12 | 0.64 |
| Hw | pg/ml | 6.2E0 | 1.2E1 | 4.3E1 | 7.1E1 | 5.1E2 | 1.4E2 | 1.0E-9 | 4.6E-1 | 9.4E3 | 5.0E2 | 340 | 12 | 340 | 12 | 0.62 |
| Hx | pg/ml | 8.8E0 | 2.1E1 | 5.5E1 | 1.4E2 | 5.0E2 | 3.6E2 | 1.0E-9 | 6.8E0 | 9.3E3 | 1.3E3 | 340 | 12 | 340 | 12 | 0.72 |
| Ih | ng/ml | 6.3E1 | 2.9E2 | 2.6E2 | 5.7E2 | 4.6E2 | 6.8E2 | 1.0E-9 | 2.4E0 | 3.6E3 | 1.9E3 | 341 | 12 | 341 | 12 | 0.63 |
| Ii | ng/ml | 8.3E1 | 2.4E2 | 2.1E2 | 6.7E2 | 5.1E2 | 1.2E3 | 7.3E-1 | 7.5E-1 | 5.2E3 | 4.5E3 | 341 | 12 | 341 | 12 | 0.63 |
| Ij | ng/ml | 7.9E1 | 2.5E2 | 2.5E2 | 4.9E2 | 1.4E3 | 6.5E2 | 2.8E0 | 9.5E0 | 2.4E4 | 1.9E3 | 337 | 12 | 337 | 12 | 0.73 |
| Ik | ng/ml | 1.1E1 | 3.5E1 | 1.3E3 | 2.5E2 | 1.2E4 | 4.3E2 | 5.9E-1 | 6.2E-1 | 1.2E5 | 1.5E3 | 337 | 12 | 337 | 12 | 0.57 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Il | ng/ml | 3.6E2 | 8.8E2 | 1.3E3 | 4.1E3 | 2.8E3 | 5.3E3 | 1.0E-9 | 1.9E-1 | 1.2E4 | 1.2E4 | 335 | 12 | 335 | 12 | 0.60 |
| Im | ng/ml | 2.1E2 | 7.0E2 | 4.5E2 | 2.1E3 | 7.8E2 | 4.2E3 | 1.4E1 | 2.2E1 | 6.2E3 | 1.5E4 | 337 | 12 | 337 | 12 | 0.74 |
| In | ng/ml | 3.4E0 | 1.5E0 | 3.3E1 | 8.3E1 | 2.6E2 | 1.8E2 | 1.0E-9 | 1.0E-9 | 4.5E3 | 5.9E2 | 341 | 12 | 341 | 12 | 0.50 |
| Io | ng/ml | 9.4E3 | 1.5E4 | 1.9E4 | 2.2E4 | 4.6E4 | 2.8E4 | 1.0E-9 | 9.0E2 | 7.1E5 | 1.0E5 | 341 | 12 | 341 | 12 | 0.56 |
| Ip | ng/ml | 1.0E1 | 3.0E1 | 2.1E1 | 3.4E1 | 2.6E1 | 2.1E1 | 1.0E-9 | 8.1E-2 | 2.3E2 | 7.1E1 | 341 | 12 | 341 | 12 | 0.68 |
| Iq | ug/ml | 1.0E-1 | 9.3E-1 | 4.1E1 | 4.9E0 | 7.4E2 | 7.2E0 | 1.0E-9 | 1.0E-9 | 1.4E4 | 2.1E1 | 341 | 12 | 341 | 12 | 0.70 |
| Ir | ug/ml | 3.7E-1 | 3.1E0 | 5.6E0 | 2.7E1 | 3.8E1 | 4.6E1 | 1.0E-9 | 3.5E-2 | 5.1E2 | 1.3E2 | 340 | 12 | 340 | 12 | 0.70 |
| Is | ng/ml | 2.0E0 | 2.9E1 | 9.4E0 | 5.7E1 | 3.4E1 | 7.6E1 | 1.0E-9 | 2.2E-1 | 5.5E2 | 2.6E2 | 341 | 12 | 341 | 12 | 0.77 |
| It | ng/ml | 2.1E0 | 3.8E0 | 2.3E1 | 5.1E1 | 1.0E2 | 1.6E2 | 1.0E-9 | 1.0E-9 | 1.1E3 | 5.5E2 | 341 | 12 | 341 | 12 | 0.58 |
| Iu | ng/ml | 1.7E2 | 9.7E2 | 1.3E3 | 6.2E3 | 4.1E3 | 9.3E3 | 1.0E-9 | 1.0E-9 | 2.9E4 | 2.4E4 | 341 | 12 | 341 | 12 | 0.57 |
| Iv | ng/ml | 1.3E1 | 2.4E1 | 9.8E1 | 6.9E2 | 8.8E2 | 1.5E3 | 1.0E-9 | 1.9E0 | 1.6E4 | 3.8E3 | 340 | 12 | 340 | 12 | 0.65 |
| Pz | ng/ml | 3.5E3 | 1.0E4 | 5.6E3 | 7.6E3 | 6.0E3 | 4.3E3 | 1.6E1 | 4.0E1 | 7.0E4 | 1.3E4 | 337 | 12 | 337 | 12 | 0.63 |
| Qa | ng/ml | 3.6E3 | 2.1E4 | 7.4E3 | 1.9E4 | 1.4E4 | 1.2E4 | 1.5E2 | 2.9E2 | 2.2E5 | 3.2E4 | 337 | 12 | 337 | 12 | 0.75 |
| Qb | ng/ml | 1.1E2 | 3.0E2 | 2.4E2 | 3.9E2 | 4.4E2 | 4.3E2 | 7.9E-1 | 8.7E0 | 5.3E3 | 1.6E3 | 337 | 12 | 337 | 12 | 0.62 |
| Qc | ng/ml | 2.1E2 | 7.0E2 | 4.5E2 | 6.9E2 | 6.0E2 | 5.1E2 | 1.0E-9 | 1.8E1 | 4.3E3 | 1.4E3 | 337 | 12 | 337 | 12 | 0.65 |
| Qd | ng/ml | 8.8E3 | 7.5E4 | 2.5E4 | 1.1E5 | 1.1E5 | 1.2E5 | 1.5E2 | 1.9E3 | 2.0E6 | 4.3E5 | 337 | 12 | 337 | 12 | 0.78 |
| Qe | ng/ml | 9.1E2 | 4.1E3 | 2.0E3 | 5.3E3 | 5.6E3 | 5.6E3 | 1.0E-9 | 5.7E1 | 9.7E4 | 1.8E4 | 337 | 12 | 337 | 12 | 0.67 |
| Jg | ng/ml | 4.6E2 | 1.8E3 | 7.8E2 | 2.1E3 | 9.5E2 | 1.9E3 | 5.8E0 | 4.5E1 | 1.0E4 | 7.1E3 | 340 | 12 | 340 | 12 | 0.73 |
| Jh | ng/ml | 2.9E0 | 3.3E1 | 2.2E1 | 6.0E1 | 8.6E1 | 8.2E1 | 1.0E-9 | 1.0E-9 | 1.2E3 | 2.9E2 | 340 | 12 | 340 | 12 | 0.74 |
| Ji | ng/ml | 5.7E1 | 3.1E2 | 9.0E1 | 3.0E2 | 1.1E2 | 2.4E2 | 1.1E0 | 2.3E1 | 1.3E3 | 6.9E2 | 340 | 12 | 340 | 12 | 0.76 |
| Jj | ng/ml | 5.3E2 | 1.4E2 | 1.9E3 | 3.7E2 | 1.8E4 | 4.0E2 | 2.3E0 | 8.7E0 | 3.4E5 | 1.0E3 | 340 | 12 | 340 | 12 | 0.31 |
| Jk | ng/ml | 2.6E0 | 5.4E1 | 2.0E1 | 7.1E1 | 4.8E1 | 7.8E1 | 1.0E-9 | 1.2E-1 | 3.9E2 | 2.4E2 | 340 | 12 | 340 | 12 | 0.72 |
| Jl | ng/ml | 4.8E-1 | 2.7E0 | 2.0E0 | 8.3E2 | 4.8E0 | 2.9E3 | 1.2E-3 | 2.5E-1 | 4.0E1 | 9.9E3 | 340 | 12 | 340 | 12 | 0.76 |
| Jm | ng/ml | 1.9E1 | 7.3E1 | 6.7E1 | 8.0E1 | 1.7E2 | 8.0E1 | 1.0E-9 | 4.0E-1 | 2.1E3 | 2.5E2 | 340 | 12 | 340 | 12 | 0.60 |
| Jn | pg/ml | 3.5E-1 | 1.5E0 | 7.3E0 | 2.2E1 | 6.2E1 | 6.8E1 | 1.0E-9 | 1.0E-9 | 7.3E2 | 2.4E2 | 340 | 12 | 340 | 12 | 0.71 |
| Jo | pg/ml | 3.8E3 | 5.1E3 | 5.1E3 | 8.9E3 | 6.6E3 | 1.0E4 | 2.0E1 | 2.4E1 | 1.0E5 | 3.6E4 | 340 | 12 | 340 | 12 | 0.59 |
| Jp | pg/ml | 7.1E4 | 1.1E5 | 7.4E4 | 1.2E5 | 4.0E4 | 3.8E4 | 5.8E2 | 6.7E4 | 3.8E5 | 1.9E5 | 340 | 12 | 340 | 12 | 0.80 |
| Jq | pg/ml | 9.6E1 | 2.6E2 | 2.0E2 | 4.5E2 | 5.4E2 | 4.8E2 | 1.0E0 | 1.3E1 | 8.7E3 | 1.7E3 | 340 | 12 | 340 | 12 | 0.74 |
| Jr | pg/ml | 4.4E0 | 3.0E1 | 9.0E1 | 2.4E2 | 7.6E2 | 7.0E2 | 1.0E-9 | 1.0E-9 | 1.1E4 | 2.4E3 | 340 | 12 | 340 | 12 | 0.77 |
| Js | pg/ml | 1.5E1 | 2.0E1 | 8.4E1 | 3.0E2 | 6.0E2 | 8.5E2 | 1.0E-9 | 2.7E0 | 1.0E4 | 3.0E3 | 340 | 12 | 340 | 12 | 0.66 |
| Jt | pg/ml | 2.4E3 | 4.7E3 | 3.1E3 | 1.0E4 | 3.5E3 | 1.3E4 | 2.2E1 | 1.5E2 | 5.2E4 | 4.1E4 | 340 | 12 | 340 | 12 | 0.70 |
| Lh | pg/ml | 1.3E4 | 3.5E4 | 2.3E4 | 7.5E4 | 4.2E4 | 1.2E5 | 1.0E-9 | 1.3E3 | 4.8E5 | 4.1E5 | 341 | 12 | 341 | 12 | 0.68 |
| Li | pg/ml | 3.5E3 | 3.0E4 | 2.0E4 | 9.0E4 | 9.1E4 | 1.3E5 | 1.2E1 | 3.7E1 | 1.3E6 | 4.1E5 | 341 | 12 | 341 | 12 | 0.67 |
| Lj | pg/ml | 3.0E3 | 9.7E3 | 2.1E4 | 2.4E4 | 5.9E4 | 3.8E4 | 1.0E-9 | 8.9E1 | 4.3E5 | 1.3E5 | 341 | 12 | 341 | 12 | 0.55 |
| Nv | pg/ml | 3.9E3 | 1.6E4 | 9.7E3 | 3.0E4 | 1.9E4 | 3.8E4 | 1.0E-9 | 1.6E2 | 1.6E5 | 1.2E5 | 342 | 12 | 342 | 12 | 0.78 |
| Nw | pg/ml | 9.6E3 | 2.7E4 | 1.4E4 | 3.3E4 | 2.2E4 | 2.5E4 | 1.9E2 | 4.5E3 | 2.2E5 | 7.8E4 | 342 | 12 | 342 | 12 | 0.78 |
| Nx | pg/ml | 2.2E2 | 9.3E2 | 4.3E2 | 9.1E2 | 6.3E2 | 8.0E2 | 1.0E-9 | 1.0E-9 | 4.1E3 | 2.3E3 | 342 | 12 | 342 | 12 | 0.68 |
| Ny | pg/ml | 6.5E0 | 4.2E1 | 1.1E2 | 1.3E2 | 1.3E3 | 1.9E2 | 1.0E-9 | 1.0E-9 | 2.5E4 | 6.1E2 | 342 | 12 | 342 | 12 | 0.77 |
| Oe | pg/ml | 2.9E1 | 1.0E-9 | 2.5E2 | 1.5E2 | 3.8E2 | 3.3E2 | 1.0E-9 | 1.0E-9 | 2.3E3 | 1.1E3 | 340 | 12 | 340 | 12 | 0.37 |
| Of | pg/ml | 1.3E2 | 1.7E2 | 5.1E3 | 3.4E3 | 2.1E4 | 7.6E3 | 1.0E-9 | 1.0E-9 | 1.9E5 | 2.4E4 | 342 | 12 | 342 | 12 | 0.55 |
| Og | pg/ml | 6.3E-2 | 7.3E-2 | 3.6E-1 | 7.8E-2 | 1.5E0 | 8.8E-2 | 1.0E-9 | 1.0E-9 | 1.9E1 | 3.2E-1 | 342 | 12 | 342 | 12 | 0.45 |
| Oh | pg/ml | 2.6E0 | 9.5E0 | 1.8E1 | 1.3E3 | 1.1E2 | 4.6E3 | 1.0E-9 | 1.0E-9 | 1.4E3 | 1.6E4 | 342 | 12 | 342 | 12 | 0.63 |
| Oi | pg/ml | 2.0E0 | 6.8E-1 | 5.0E0 | 3.9E0 | 7.9E0 | 5.6E0 | 1.0E-9 | 1.0E-9 | 6.7E1 | 1.6E1 | 342 | 12 | 342 | 12 | 0.45 |
| Ok | pg/ml | 3.9E2 | 1.5E3 | 5.4E2 | 2.0E3 | 6.5E2 | 2.1E3 | 1.5E1 | 5.3E1 | 7.8E3 | 7.0E3 | 342 | 12 | 342 | 12 | 0.73 |
| Om | pg/ml | 4.2E2 | 1.6E3 | 9.5E2 | 2.2E3 | 3.4E3 | 2.0E3 | 1.0E-9 | 7.0E1 | 5.1E4 | 5.6E3 | 342 | 12 | 342 | 12 | 0.76 |
| On | pg/ml | 1.8E2 | 9.6E2 | 3.0E2 | 1.7E3 | 4.4E2 | 2.4E3 | 1.0E-9 | 1.6E1 | 4.5E3 | 8.5E3 | 342 | 12 | 342 | 12 | 0.82 |
| Oy | pg/ml | 4.7E-1 | 1.4E0 | 6.1E0 | 6.2E0 | 3.1E1 | 1.0E1 | 1.0E-9 | 1.0E-9 | 4.0E2 | 3.3E1 | 341 | 12 | 341 | 12 | 0.56 |
| Oz | pg/ml | 7.0E-3 | 1.0E-9 | 3.3E-1 | 2.4E0 | 1.6E0 | 8.1E0 | 1.0E-9 | 1.0E-9 | 2.9E1 | 2.8E1 | 341 | 12 | 341 | 12 | 0.38 |
| Pa | pg/ml | 3.9E-1 | 6.7E-1 | 1.7E0 | 2.2E1 | 7.9E0 | 6.5E1 | 1.0E-9 | 1.0E-9 | 1.0E2 | 2.3E2 | 341 | 12 | 341 | 12 | 0.59 |
| Pb | pg/ml | 1.0E-9 | 1.0E-9 | 1.7E0 | 1.7E-1 | 2.6E1 | 5.5E-1 | 1.0E-9 | 1.0E-9 | 4.9E2 | 1.9E0 | 341 | 12 | 341 | 12 | 0.43 |
| Pc | pg/ml | 5.1E-2 | 1.0E-9 | 3.9E-1 | 3.1E1 | 9.0E-1 | 9.5E1 | 1.0E-9 | 1.0E-9 | 1.4E1 | 3.3E2 | 341 | 12 | 341 | 12 | 0.46 |
| Pd | pg/ml | 1.6E0 | 1.3E0 | 6.8E0 | 9.7E0 | 4.7E1 | 1.7E1 | 1.0E-9 | 1.0E-9 | 8.4E2 | 5.5E1 | 341 | 12 | 341 | 12 | 0.50 |
| Pe | pg/ml | 2.1E1 | 7.6E1 | 1.5E2 | 1.5E3 | 6.3E2 | 4.3E3 | 1.0E-9 | 1.1E0 | 6.7E3 | 1.5E4 | 341 | 12 | 341 | 12 | 0.72 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pf | pg/ml | 1.6E0 | 1.6E1 | 1.6E1 | 3.2E1 | 9.3E1 | 4.4E1 | 1.0E-9 | 1.0E-9 | 1.5E3 | 1.5E2 | 341 | 12 | 341 | 12 | 0.72 |
| Pg | pg/ml | 3.9E0 | 4.9E1 | 7.6E1 | 3.1E2 | 5.2E2 | 4.6E2 | 1.0E-9 | 8.4E-1 | 7.7E3 | 1.3E3 | 341 | 12 | 341 | 12 | 0.75 |
| aA | mg/dL | 9.0E-1 | 1.2E0 | 1.0E0 | 1.9E0 | 5.4E-1 | 1.3E0 | 3.0E-1 | 5.5E-1 | 4.2E0 | 4.7E0 | 516 | 21 | 516 | 21 | 0.74 |
| aC | mg/mL | 2.2E0 | 3.8E0 | 2.6E0 | 3.5E0 | 1.3E0 | 2.2E0 | 7.5E-1 | 1.0E0 | 7.4E0 | 6.7E0 | 166 | 9 | 166 | 9 | 0.57 |
| aD | ug/mL | 3.0E0 | 3.2E0 | 4.7E0 | 3.5E0 | 4.8E0 | 1.7E0 | 7.5E-1 | 1.8E0 | 3.5E1 | 7.1E0 | 166 | 9 | 166 | 9 | 0.47 |
| aE | mg/mL | 5.7E-1 | 5.7E-1 | 5.9E-1 | 5.5E-1 | 1.7E-1 | 9.8E-2 | 1.8E-1 | 4.1E-1 | 1.2E0 | 6.9E-1 | 166 | 9 | 166 | 9 | 0.44 |
| aF | ng/mL | 2.2E0 | 3.8E0 | 5.0E0 | 6.1E0 | 7.8E0 | 5.6E0 | 4.3E-3 | 4.3E-3 | 5.0E1 | 1.5E1 | 166 | 9 | 166 | 9 | 0.60 |
| aG | mg/mL | 1.4E-1 | 9.0E-2 | 1.6E-1 | 9.6E-2 | 8.7E-2 | 2.4E-2 | 3.2E-2 | 6.9E-2 | 4.8E-1 | 1.5E-1 | 166 | 9 | 166 | 9 | 0.26 |
| aH | ug/mL | 7.2E1 | 5.6E1 | 7.8E1 | 6.1E1 | 4.1E1 | 2.0E1 | 8.9E0 | 3.2E1 | 2.0E2 | 1.0E2 | 166 | 9 | 166 | 9 | 0.41 |
| aI | ug/mL | 1.7E2 | 1.3E2 | 1.8E2 | 1.3E2 | 6.2E1 | 3.0E1 | 3.2E1 | 7.5E1 | 3.4E2 | 1.8E2 | 166 | 9 | 166 | 9 | 0.25 |
| aJ | ug/mL | 2.4E0 | 4.4E0 | 3.2E0 | 7.0E0 | 2.3E0 | 6.4E0 | 8.2E-1 | 2.2E0 | 1.4E1 | 2.3E1 | 166 | 9 | 166 | 9 | 0.79 |
| aK | ng/mL | 1.3E0 | 5.7E-1 | 2.0E0 | 1.7E0 | 2.0E0 | 2.1E0 | 2.9E-4 | 1.3E-1 | 1.0E1 | 6.5E0 | 166 | 9 | 166 | 9 | 0.42 |
| aL | mg/mL | 7.4E-1 | 4.9E-1 | 7.7E-1 | 5.8E-1 | 2.6E-1 | 2.2E-1 | 2.2E-1 | 3.2E-1 | 1.7E0 | 9.2E-1 | 166 | 9 | 166 | 9 | 0.28 |
| aM | U/mL | 1.8E1 | 4.7E1 | 3.9E1 | 1.5E2 | 7.8E1 | 2.3E2 | 4.2E-2 | 4.2E-2 | 8.2E2 | 6.8E2 | 166 | 9 | 166 | 9 | 0.73 |
| aN | U/mL | 1.4E1 | 2.3E1 | 2.6E1 | 2.5E1 | 4.3E1 | 2.5E1 | 2.5E-3 | 7.4E0 | 3.8E2 | 8.8E1 | 166 | 9 | 166 | 9 | 0.59 |
| aO | pg/mL | 5.3E1 | 1.8E2 | 4.2E2 | 8.4E2 | 9.9E2 | 9.2E2 | 6.0E-2 | 3.2E1 | 6.6E3 | 2.1E3 | 166 | 9 | 166 | 9 | 0.73 |
| aP | ng/mL | 1.6E0 | 4.3E0 | 2.2E0 | 6.5E0 | 2.4E0 | 8.3E0 | 4.5E-1 | 1.6E0 | 2.8E1 | 2.8E1 | 166 | 9 | 166 | 9 | 0.82 |
| aQ | ng/mL | 2.4E-1 | 2.5E-1 | 3.6E-1 | 3.4E-1 | 3.2E-1 | 2.5E-1 | 2.0E-4 | 5.2E-2 | 2.0E0 | 9.0E-1 | 166 | 9 | 166 | 9 | 0.52 |
| aR | ng/mL | 1.8E0 | 3.0E0 | 3.0E0 | 4.0E0 | 4.0E0 | 4.2E0 | 2.6E-1 | 5.6E-1 | 3.4E1 | 1.4E1 | 166 | 9 | 166 | 9 | 0.59 |
| aS | ng/mL | 3.7E-1 | 5.4E-1 | 1.0E0 | 9.1E-1 | 2.7E0 | 8.2E-1 | 4.2E-3 | 8.0E-2 | 3.3E1 | 2.6E0 | 166 | 9 | 166 | 9 | 0.61 |
| aU | pg/mL | 6.7E1 | 4.4E1 | 1.0E2 | 1.0E2 | 1.0E2 | 1.6E2 | 7.4E-2 | 7.4E-2 | 7.0E2 | 5.1E2 | 166 | 9 | 166 | 9 | 0.39 |
| aV | ng/mL | 6.0E-1 | 3.5E-1 | 1.1E0 | 5.9E-1 | 2.6E0 | 7.1E-1 | 7.6E-4 | 1.5E-1 | 3.3E1 | 2.4E0 | 166 | 9 | 166 | 9 | 0.38 |
| aW | pg/mL | 1.9E1 | 2.1E1 | 2.3E1 | 2.3E1 | 3.4E1 | 1.4E1 | 7.2E-2 | 7.2E-2 | 4.2E2 | 4.7E1 | 166 | 9 | 166 | 9 | 0.58 |
| aX | ng/mL | 7.5E0 | 3.5E1 | 1.3E1 | 7.7E1 | 2.0E1 | 9.9E1 | 3.0E-1 | 2.5E0 | 2.2E2 | 3.1E2 | 166 | 9 | 166 | 9 | 0.77 |
| aY | pg/mL | 5.3E1 | 6.6E1 | 7.4E1 | 1.1E2 | 1.1E2 | 1.2E2 | 4.1E-1 | 2.7E1 | 1.2E3 | 3.9E2 | 166 | 9 | 166 | 9 | 0.61 |
| aZ | pg/mL | 2.2E2 | 5.4E2 | 5.7E2 | 2.7E3 | 1.2E3 | 3.2E3 | 1.7E0 | 7.5E1 | 1.2E4 | 7.9E3 | 166 | 9 | 166 | 9 | 0.75 |
| bA | ng/mL | 1.3E1 | 1.2E2 | 6.4E1 | 2.8E2 | 1.4E2 | 4.6E2 | 3.0E-2 | 4.7E0 | 9.4E2 | 1.5E3 | 166 | 9 | 166 | 9 | 0.79 |
| bB | ng/mL | 2.8E2 | 1.7E2 | 3.1E2 | 2.0E2 | 1.8E2 | 9.0E1 | 2.1E0 | 7.6E1 | 9.5E2 | 3.8E2 | 166 | 9 | 166 | 9 | 0.32 |
| bC | ng/mL | 3.2E2 | 4.7E2 | 5.9E2 | 1.7E3 | 7.7E2 | 1.8E3 | 1.4E1 | 5.8E1 | 4.7E3 | 4.0E3 | 166 | 9 | 166 | 9 | 0.66 |
| bE | mg/mL | 5.2E0 | 5.1E0 | 5.5E0 | 6.3E0 | 2.1E0 | 3.5E0 | 1.3E0 | 2.6E0 | 1.2E1 | 1.2E1 | 166 | 9 | 166 | 9 | 0.51 |
| bF | pg/mL | 3.5E1 | 6.8E1 | 3.3E2 | 7.6E2 | 1.3E3 | 2.1E3 | 5.0E-2 | 1.4E1 | 1.1E4 | 6.3E3 | 166 | 9 | 166 | 9 | 0.68 |
| bG | ng/mL | 1.6E0 | 1.9E0 | 2.9E0 | 7.5E0 | 3.8E0 | 9.9E0 | 1.1E-1 | 1.6E-1 | 2.6E1 | 3.0E1 | 166 | 9 | 166 | 9 | 0.60 |
| bH | pg/mL | 5.7E-1 | 9.2E0 | 6.2E0 | 8.6E0 | 2.4E1 | 8.4E0 | 5.7E-1 | 5.7E-1 | 2.8E2 | 2.3E1 | 166 | 9 | 166 | 9 | 0.66 |
| bI | ng/mL | 4.0E-3 | 4.0E-3 | 9.5E-2 | 6.5E-2 | 2.0E-1 | 1.3E-1 | 4.0E-3 | 4.0E-3 | 9.8E-1 | 3.9E-1 | 166 | 9 | 166 | 9 | 0.49 |
| bJ | mg/mL | 1.9E0 | 2.2E0 | 2.4E0 | 2.7E0 | 2.0E0 | 2.4E0 | 2.5E-4 | 8.7E-1 | 1.1E1 | 8.9E0 | 166 | 9 | 166 | 9 | 0.55 |
| bL | pg/mL | 3.7E0 | 1.4E1 | 9.1E0 | 1.3E1 | 1.2E1 | 7.3E0 | 4.6E-2 | 3.2E0 | 6.0E1 | 2.4E1 | 166 | 9 | 166 | 9 | 0.72 |
| bM | mg/mL | 1.8E0 | 1.2E0 | 2.2E0 | 1.5E0 | 1.5E0 | 1.2E0 | 1.8E-2 | 1.6E-2 | 8.6E0 | 3.8E0 | 166 | 9 | 166 | 9 | 0.36 |
| bN | ng/mL | 3.4E1 | 2.4E1 | 1.2E2 | 5.5E1 | 2.9E2 | 7.6E1 | 1.4E-1 | 6.7E0 | 1.9E3 | 2.5E2 | 166 | 9 | 166 | 9 | 0.46 |
| bO | ng/mL | 4.0E-2 | 4.0E-2 | 9.6E0 | 4.0E-2 | 2.0E1 | 0.0E0 | 4.0E-2 | 4.0E-2 | 1.3E2 | 4.0E-2 | 166 | 9 | 166 | 9 | 0.33 |
| bP | mg/mL | 5.3E-1 | 4.9E-1 | 7.5E-1 | 6.2E-1 | 7.1E-1 | 4.7E-1 | 4.9E-2 | 9.2E-2 | 4.8E0 | 1.6E0 | 166 | 9 | 166 | 9 | 0.47 |
| bQ | pg/mL | 2.1E1 | 9.3E1 | 1.4E2 | 8.7E1 | 1.1E3 | 5.3E1 | 1.5E-1 | 2.2E1 | 1.3E4 | 1.6E2 | 166 | 9 | 166 | 9 | 0.79 |
| bR | ng/mL | 1.2E-2 | 1.2E-2 | 1.6E-1 | 8.3E-2 | 7.2E-1 | 1.4E-1 | 1.2E-2 | 1.2E-2 | 8.7E0 | 3.8E-1 | 166 | 9 | 166 | 9 | 0.46 |
| bS | pg/mL | 9.4E-1 | 9.4E-1 | 9.0E0 | 1.9E1 | 4.1E1 | 3.0E1 | 9.4E-1 | 9.4E-1 | 3.9E2 | 7.2E1 | 166 | 9 | 166 | 9 | 0.61 |
| bU | ng/mL | 7.8E-2 | 1.6E-1 | 1.9E-1 | 1.6E-1 | 5.4E-1 | 1.5E-1 | 1.3E-2 | 1.3E-2 | 6.6E0 | 4.1E-1 | 166 | 9 | 166 | 9 | 0.55 |
| bV | pg/mL | 4.8E2 | 6.6E2 | 6.2E2 | 8.5E2 | 9.0E2 | 5.5E2 | 1.6E2 | 5.1E2 | 1.2E4 | 2.2E3 | 166 | 9 | 166 | 9 | 0.74 |
| bW | pg/mL | 3.1E2 | 5.7E2 | 4.7E2 | 1.2E3 | 5.0E2 | 1.5E3 | 8.4E1 | 2.5E2 | 4.8E3 | 3.9E3 | 166 | 9 | 166 | 9 | 0.70 |
| bX | ng/mL | 2.5E-5 | 2.5E-5 | 2.6E-3 | 2.4E-3 | 3.2E-3 | 3.0E-3 | 2.5E-5 | 2.5E-5 | 1.3E-2 | 7.2E-3 | 166 | 9 | 166 | 9 | 0.49 |
| bZ | pg/mL | 2.7E2 | 1.8E3 | 1.8E3 | 6.8E3 | 6.5E3 | 1.4E4 | 1.5E-1 | 1.4E2 | 5.8E4 | 4.3E4 | 166 | 9 | 166 | 9 | 0.75 |
| cA | pg/mL | 6.0E-1 | 6.0E-1 | 4.1E0 | 3.6E0 | 2.9E1 | 6.9E0 | 6.0E-1 | 6.0E-1 | 3.7E2 | 2.1E1 | 166 | 9 | 166 | 9 | 0.55 |
| cB | ng/mL | 5.1E-2 | 1.7E-3 | 7.5E-2 | 6.2E-2 | 8.7E-2 | 1.0E-1 | 1.7E-3 | 1.7E-3 | 4.3E-1 | 2.6E-1 | 166 | 9 | 166 | 9 | 0.35 |
| cC | pg/mL | 4.3E1 | 3.5E1 | 4.6E1 | 3.3E1 | 5.1E1 | 2.4E1 | 1.0E0 | 1.0E0 | 4.5E2 | 6.7E1 | 166 | 9 | 166 | 9 | 0.41 |
| cD | pg/mL | 4.9E0 | 2.9E0 | 1.3E1 | 4.9E0 | 4.6E1 | 3.6E0 | 3.3E-1 | 8.8E-1 | 4.8E2 | 9.6E0 | 166 | 9 | 166 | 9 | 0.47 |
| cE | pg/mL | 6.0E1 | 1.5E2 | 2.7E2 | 2.6E2 | 6.0E2 | 3.8E2 | 1.2E-1 | 3.2E1 | 3.8E3 | 1.3E3 | 166 | 9 | 166 | 9 | 0.66 |

| Code | Units | Median | | Average | | Standard Deviation | | Minimum | | Maximum | | Number of Samples | | Number of Patients | | AUC |
|------|-------|--------|--|---------|--|-----|------|---------|--|---------|--|-------|--|----------|--|-----|
| | | NonDis | Dis | NonDis | Dis | NonDis | Dis | NonDis | Dis | NonDis | Dis | NonDis | Dis | NonDis | Dis | |
| cF | pg/mL | 2.5E0 | 5.3E-1 | 1.6E1 | 8.0E0 | 3.0E1 | 1.3E1 | 5.3E-1 | 5.3E-1 | 2.7E2 | 3.8E1 | 166 | 9 | 166 | 9 | 0.41 |
| cG | pg/mL | 5.3E1 | 2.2E2 | 1.7E2 | 3.1E2 | 8.2E2 | 3.2E2 | 7.8E0 | 4.0E1 | 1.0E4 | 1.1E3 | 166 | 9 | 166 | 9 | 0.81 |
| cH | uIU/mL | 3.2E0 | 5.7E0 | 7.4E0 | 1.3E1 | 1.7E1 | 1.8E1 | 8.6E-3 | 8.6E-3 | 1.6E2 | 5.3E1 | 166 | 9 | 166 | 9 | 0.57 |
| cI | ng/mL | 6.1E0 | 9.1E0 | 1.4E1 | 1.3E1 | 2.2E1 | 1.5E1 | 3.2E-2 | 1.1E0 | 1.2E2 | 4.1E1 | 166 | 9 | 166 | 9 | 0.50 |
| cJ | ug/mL | 6.8E1 | 3.1E1 | 1.0E2 | 4.1E1 | 1.0E2 | 4.9E1 | 6.9E0 | 5.6E0 | 6.4E2 | 1.7E2 | 166 | 9 | 166 | 9 | 0.28 |
| cK | ng/mL | 3.8E-3 | 3.8E-3 | 2.3E-2 | 3.1E-2 | 1.2E-1 | 6.8E-2 | 3.8E-3 | 3.8E-3 | 1.5E0 | 2.1E-1 | 166 | 9 | 166 | 9 | 0.55 |
| cL | pg/mL | 2.1E2 | 2.3E2 | 5.3E2 | 6.4E2 | 2.0E3 | 8.9E2 | 3.1E1 | 6.7E1 | 2.4E4 | 2.8E3 | 166 | 9 | 166 | 9 | 0.59 |
| cM | pg/mL | 2.7E2 | 2.5E2 | 2.9E2 | 2.4E2 | 1.7E2 | 6.4E1 | 2.5E1 | 1.3E2 | 1.1E3 | 3.1E2 | 166 | 9 | 166 | 9 | 0.41 |
| cN | pg/mL | 1.2E2 | 1.3E2 | 1.3E2 | 1.3E2 | 8.6E1 | 3.9E1 | 3.8E1 | 8.6E1 | 1.1E3 | 1.9E2 | 166 | 9 | 166 | 9 | 0.57 |
| cO | pg/mL | 2.1E2 | 4.4E2 | 4.0E2 | 5.5E2 | 1.5E3 | 4.2E2 | 5.4E1 | 9.6E1 | 1.9E4 | 1.5E3 | 166 | 9 | 166 | 9 | 0.75 |
| cP | ng/mL | 2.4E3 | 2.9E3 | 2.5E3 | 2.7E3 | 9.5E2 | 1.1E3 | 6.2E2 | 1.4E3 | 5.6E3 | 4.7E3 | 166 | 9 | 166 | 9 | 0.57 |
| cQ | ng/mL | 5.2E-2 | 9.1E-2 | 1.3E-1 | 1.6E-1 | 2.1E-1 | 1.7E-1 | 2.0E-3 | 2.0E-3 | 1.3E0 | 3.9E-1 | 166 | 9 | 166 | 9 | 0.57 |
| cR | ng/mL | 3.3E2 | 6.5E2 | 5.8E2 | 9.6E2 | 8.3E2 | 7.3E2 | 2.0E1 | 1.1E2 | 7.7E3 | 2.2E3 | 166 | 9 | 166 | 9 | 0.71 |
| cS | ng/mL | 2.8E2 | 1.2E3 | 4.1E2 | 1.8E3 | 4.2E2 | 2.2E3 | 4.1E1 | 1.4E2 | 2.5E3 | 7.1E3 | 166 | 9 | 166 | 9 | 0.78 |
| cT | ng/mL | 5.0E1 | 1.5E2 | 1.5E2 | 3.4E2 | 2.9E2 | 4.9E2 | 3.6E0 | 1.9E1 | 2.1E3 | 1.5E3 | 166 | 9 | 166 | 9 | 0.67 |
| cU | ng/mL | 5.8E1 | 2.2E2 | 9.3E1 | 2.1E2 | 1.5E2 | 9.5E1 | 6.2E0 | 9.8E1 | 1.6E3 | 3.9E2 | 166 | 9 | 166 | 9 | 0.90 |
| cV | ng/mL | 1.9E-1 | 1.9E-1 | 7.4E-1 | 5.1E-1 | 3.8E0 | 7.6E-1 | 2.5E-2 | 9.7E-2 | 4.7E1 | 2.5E0 | 166 | 9 | 166 | 9 | 0.58 |
| cW | mIU/mL | 4.8E-2 | 9.9E-2 | 8.8E-2 | 1.1E-1 | 3.5E-1 | 8.1E-2 | 4.8E-3 | 3.3E-2 | 4.5E0 | 2.9E-1 | 166 | 9 | 166 | 9 | 0.72 |
| cX | ng/mL | 1.3E-1 | 3.3E-2 | 1.9E0 | 1.1E-1 | 5.7E0 | 1.4E-1 | 2.3E-4 | 1.1E-2 | 2.8E1 | 4.1E-1 | 166 | 9 | 166 | 9 | 0.38 |
| cY | ng/mL | 7.6E0 | 3.3E0 | 1.1E1 | 8.5E0 | 1.1E1 | 1.1E1 | 1.7E-1 | 6.0E-1 | 6.1E1 | 3.6E1 | 166 | 9 | 166 | 9 | 0.38 |
| cZ | ug/mL | 1.3E1 | 1.1E1 | 1.5E1 | 1.4E1 | 6.8E0 | 7.8E0 | 2.3E0 | 7.0E0 | 4.6E1 | 3.0E1 | 166 | 9 | 166 | 9 | 0.41 |
| dA | pg/mL | 3.1E2 | 3.3E2 | 3.9E2 | 4.3E2 | 4.6E2 | 2.5E2 | 1.0E2 | 1.7E2 | 5.8E3 | 8.8E2 | 166 | 9 | 166 | 9 | 0.56 |
| dB | ug/mL | 1.8E1 | 2.0E1 | 1.8E1 | 2.1E1 | 2.0E1 | 5.3E0 | 2.1E0 | 1.5E1 | 2.5E2 | 2.8E1 | 166 | 9 | 166 | 9 | 0.63 |
| dC | nmol/L | 3.5E1 | 2.7E1 | 3.8E1 | 2.9E1 | 1.7E1 | 7.7E0 | 7.8E0 | 1.5E1 | 1.4E2 | 4.1E1 | 166 | 9 | 166 | 9 | 0.32 |
| dD | ug/mL | 3.4E1 | 2.9E1 | 3.5E1 | 3.0E1 | 1.1E1 | 7.1E0 | 1.4E1 | 2.2E1 | 7.4E1 | 4.3E1 | 166 | 9 | 166 | 9 | 0.32 |
| dE | ng/mL | 4.4E-1 | 9.8E-1 | 5.5E-1 | 1.1E0 | 5.7E-1 | 9.2E-1 | 8.4E-3 | 3.0E-1 | 2.7E0 | 2.9E0 | 166 | 9 | 166 | 9 | 0.71 |
| dF | ng/mL | 2.4E2 | 3.8E2 | 3.4E2 | 5.4E2 | 2.5E2 | 2.5E2 | 7.5E1 | 2.8E2 | 1.3E3 | 8.9E2 | 166 | 9 | 166 | 9 | 0.77 |
| dG | ng/mL | 1.2E1 | 1.6E1 | 1.7E1 | 2.4E1 | 1.9E1 | 1.9E1 | 3.0E0 | 6.7E0 | 1.8E2 | 6.5E1 | 166 | 9 | 166 | 9 | 0.67 |
| dH | pg/mL | 8.0E0 | 1.1E1 | 2.1E1 | 1.8E1 | 6.3E1 | 2.2E1 | 4.0E-2 | 8.3E-1 | 6.7E2 | 7.6E1 | 166 | 9 | 166 | 9 | 0.62 |
| dI | pg/mL | 4.6E-1 | 4.6E-1 | 3.8E0 | 4.4E0 | 2.6E1 | 6.2E0 | 4.6E-1 | 4.6E-1 | 3.3E2 | 1.9E1 | 166 | 9 | 166 | 9 | 0.63 |
| dJ | ng/mL | 2.0E0 | 2.2E0 | 2.2E0 | 2.2E0 | 1.1E0 | 1.2E0 | 3.2E-2 | 5.7E-1 | 5.6E0 | 4.0E0 | 166 | 9 | 166 | 9 | 0.52 |
| dK | uIU/mL | 1.3E0 | 5.8E-1 | 2.2E0 | 1.2E0 | 3.6E0 | 1.9E0 | 2.8E-4 | 1.4E-2 | 3.9E1 | 6.1E0 | 166 | 9 | 166 | 9 | 0.28 |
| dL | ng/mL | 8.7E2 | 8.4E2 | 1.1E3 | 1.2E3 | 6.3E2 | 6.1E2 | 2.8E2 | 5.8E2 | 4.8E3 | 2.3E3 | 166 | 9 | 166 | 9 | 0.56 |
| dM | pg/mL | 9.9E2 | 1.9E3 | 1.3E3 | 2.5E3 | 1.5E3 | 2.0E3 | 3.7E2 | 7.1E2 | 1.5E4 | 5.8E3 | 166 | 9 | 166 | 9 | 0.66 |
| dN | ug/mL | 9.9E1 | 1.4E2 | 1.0E2 | 1.3E2 | 4.4E1 | 3.6E1 | 2.4E1 | 6.6E1 | 3.3E2 | 1.7E2 | 166 | 9 | 166 | 9 | 0.69 |
| fR | ng/ml | 1.4E5 | 3.2E5 | 2.1E5 | 3.5E5 | 1.7E5 | 2.4E5 | 3.6E4 | 1.9E2 | 6.9E5 | 8.7E5 | 105 | 10 | 105 | 10 | 0.70 |
| nM | pg/ml | 1.0E-9 | 1.0E-9 | 1.0E-9 | 2.2E2 | 0.0E0 | 5.8E2 | 1.0E-9 | 1.0E-9 | 1.0E-9 | 1.5E3 | 69 | 7 | 69 | 7 | 0.57 |
| nN | pg/ml | 1.4E3 | 3.0E3 | 4.0E3 | 3.0E4 | 1.3E4 | 5.6E4 | 8.1E1 | 6.3E2 | 1.0E5 | 1.5E5 | 69 | 7 | 69 | 7 | 0.69 |
| nO | pg/ml | 2.4E1 | 4.3E1 | 3.7E1 | 5.1E1 | 4.6E1 | 4.3E1 | 4.0E0 | 9.7E0 | 3.1E2 | 1.4E2 | 69 | 7 | 69 | 7 | 0.67 |
| nR | pg/ml | 1.6E1 | 7.1E1 | 6.8E1 | 4.3E2 | 1.7E2 | 7.0E2 | 1.0E0 | 1.1E1 | 1.1E3 | 1.9E3 | 69 | 7 | 69 | 7 | 0.75 |
| nT | pg/ml | 7.3E1 | 1.1E2 | 1.0E2 | 2.8E2 | 9.8E1 | 3.3E2 | 1.0E-9 | 1.0E-9 | 6.4E2 | 9.2E2 | 69 | 7 | 69 | 7 | 0.61 |
| nU | pg/ml | 4.4E1 | 4.4E2 | 7.6E1 | 3.9E2 | 1.8E2 | 3.6E2 | 1.0E-9 | 1.0E-9 | 1.5E3 | 9.2E2 | 69 | 7 | 69 | 7 | 0.78 |
| lW | pg/ml | 1.0E-9 | 1.0E-9 | 1.1E1 | 6.4E0 | 3.0E1 | 1.4E1 | 1.0E-9 | 1.0E-9 | 1.7E2 | 3.9E1 | 69 | 7 | 69 | 7 | 0.48 |
| lX | pg/ml | 9.0E2 | 9.1E2 | 9.8E2 | 1.1E3 | 5.5E2 | 7.3E2 | 1.9E2 | 4.8E2 | 2.6E3 | 2.5E3 | 69 | 7 | 69 | 7 | 0.55 |
| lY | pg/ml | 1.9E1 | 1.4E1 | 2.0E1 | 2.0E1 | 1.7E1 | 1.5E1 | 1.0E-9 | 3.1E0 | 1.2E2 | 4.5E1 | 69 | 7 | 69 | 7 | 0.51 |
| mE | pg/ml | 1.0E-9 | 1.9E0 | 2.5E0 | 2.9E0 | 7.7E0 | 3.7E0 | 1.0E-9 | 1.0E-9 | 5.7E1 | 9.2E0 | 69 | 7 | 69 | 7 | 0.61 |
| mF | pg/ml | 2.7E-1 | 5.4E0 | 6.6E0 | 5.5E0 | 3.2E1 | 5.1E0 | 1.0E-9 | 1.0E-9 | 2.5E2 | 1.3E1 | 69 | 7 | 69 | 7 | 0.74 |
| mH | pg/ml | 3.0E0 | 3.3E0 | 5.5E0 | 3.6E0 | 8.2E0 | 2.3E0 | 4.0E-1 | 5.4E-1 | 5.3E1 | 6.5E0 | 69 | 7 | 69 | 7 | 0.49 |
| mI | pg/ml | 1.0E-9 | 3.4E0 | 1.2E1 | 8.4E1 | 2.6E1 | 1.7E2 | 1.0E-9 | 1.0E-9 | 1.8E2 | 4.6E2 | 69 | 7 | 69 | 7 | 0.65 |
| mM | pg/ml | 3.5E1 | 3.8E1 | 8.0E1 | 9.3E1 | 1.5E2 | 1.7E2 | 1.0E-9 | 1.0E-9 | 1.1E3 | 4.7E2 | 69 | 7 | 69 | 7 | 0.46 |
| mP | pg/ml | 1.5E1 | 1.6E1 | 1.7E1 | 1.5E2 | 1.6E1 | 2.9E2 | 1.0E-9 | 1.4E1 | 1.2E2 | 8.1E2 | 68 | 7 | 68 | 7 | 0.70 |
| mS | pg/ml | 1.6E3 | 1.6E3 | 1.7E3 | 2.0E3 | 1.0E3 | 1.0E3 | 1.0E-9 | 6.7E2 | 5.1E3 | 3.5E3 | 69 | 7 | 69 | 7 | 0.59 |
| mT | pg/ml | 5.7E1 | 5.6E1 | 1.2E2 | 4.2E2 | 2.2E2 | 6.6E2 | 1.0E1 | 1.6E1 | 1.4E3 | 1.7E3 | 68 | 7 | 68 | 7 | 0.58 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| mU | pg/ml | 2.3E0 | 2.1E0 | 6.3E0 | 6.4E0 | 2.7E1 | 8.1E0 | 1.0E-9 | 6.1E-1 | 2.2E2 | 2.3E1 | 68 | 7 | 68 | 7 | 0.55 |
| mW | pg/ml | 2.1E3 | 3.8E3 | 2.3E3 | 5.2E3 | 1.2E3 | 4.5E3 | 1.0E-9 | 3.7E2 | 6.2E3 | 1.1E4 | 68 | 7 | 68 | 7 | 0.65 |
| mY | pg/ml | 6.5E2 | 6.5E2 | 8.5E2 | 2.0E3 | 9.4E2 | 2.8E3 | 1.0E-9 | 1.9E2 | 5.6E3 | 8.0E3 | 69 | 7 | 69 | 7 | 0.62 |
| mZ | pg/ml | 1.8E2 | 5.5E2 | 3.5E2 | 6.0E2 | 4.7E2 | 5.0E2 | 1.0E-9 | 6.6E1 | 3.1E3 | 1.4E3 | 68 | 7 | 68 | 7 | 0.68 |
| nA | pg/ml | 1.5E0 | 3.4E0 | 6.6E0 | 1.1E1 | 1.3E1 | 2.0E1 | 1.0E-9 | 1.0E-9 | 6.5E1 | 5.7E1 | 68 | 7 | 68 | 7 | 0.58 |
| nB | pg/ml | 2.9E2 | 3.0E2 | 3.1E2 | 3.8E2 | 1.6E2 | 2.8E2 | 3.0E1 | 1.3E2 | 8.2E2 | 9.6E2 | 69 | 7 | 69 | 7 | 0.54 |
| nC | pg/ml | 1.0E-9 | 7.0E2 | 9.4E3 | 4.1E3 | 5.3E4 | 7.4E3 | 1.0E-9 | 1.0E-9 | 3.8E5 | 2.0E4 | 69 | 7 | 69 | 7 | 0.72 |
| nD | pg/ml | 6.4E0 | 1.6E1 | 1.2E1 | 2.7E1 | 3.2E1 | 4.0E1 | 1.0E-9 | 1.0E-9 | 2.6E2 | 1.2E2 | 68 | 7 | 68 | 7 | 0.70 |
| nF | pg/ml | 1.0E-9 | 1.0E-9 | 2.5E0 | 8.6E0 | 1.2E1 | 1.8E1 | 1.0E-9 | 1.0E-9 | 9.3E1 | 4.7E1 | 69 | 7 | 69 | 7 | 0.60 |
| nH | pg/ml | 5.6E-1 | 1.1E1 | 1.9E2 | 6.3E1 | 1.2E3 | 1.2E2 | 1.0E-9 | 1.0E-9 | 1.0E4 | 3.3E2 | 68 | 7 | 68 | 7 | 0.71 |
| nI | pg/ml | 2.8E0 | 1.0E-9 | 5.5E1 | 2.0E2 | 7.5E1 | 4.5E2 | 1.0E-9 | 1.0E-9 | 3.5E2 | 1.2E3 | 69 | 7 | 69 | 7 | 0.48 |
| nJ | pg/ml | 1.7E-1 | 1.1E0 | 2.8E0 | 3.0E0 | 1.6E1 | 5.4E0 | 1.0E-9 | 1.0E-9 | 1.3E2 | 1.5E1 | 69 | 7 | 69 | 7 | 0.63 |
| nK | pg/ml | 1.0E-9 | 1.0E1 | 1.3E1 | 4.7E1 | 2.3E1 | 8.1E1 | 1.0E-9 | 1.0E-9 | 1.0E2 | 2.2E2 | 68 | 7 | 68 | 7 | 0.64 |
| nL | pg/ml | 1.0E-9 | 1.5E1 | 2.8E2 | 1.8E2 | 1.7E3 | 3.3E2 | 1.0E-9 | 1.0E-9 | 1.4E4 | 9.0E2 | 69 | 7 | 69 | 7 | 0.64 |
| kC | pg/ml | 9.6E1 | 1.1E2 | 1.9E2 | 1.3E2 | 3.6E2 | 7.9E1 | 2.1E1 | 5.9E1 | 2.7E3 | 2.9E2 | 69 | 7 | 69 | 7 | 0.53 |
| kE | pg/ml | 1.4E5 | 1.4E5 | 1.4E5 | 1.4E5 | 4.1E4 | 2.5E4 | 3.8E4 | 1.1E5 | 2.7E5 | 1.9E5 | 69 | 7 | 69 | 7 | 0.49 |
| kF | pg/mL | 6.2E1 | 7.6E1 | 6.7E1 | 8.7E1 | 2.3E1 | 3.7E1 | 2.7E1 | 5.2E1 | 1.5E2 | 1.4E2 | 69 | 7 | 69 | 7 | 0.67 |
| kG | pg/mL | 8.4E3 | 1.6E4 | 1.1E4 | 4.3E4 | 9.6E3 | 5.6E4 | 1.1E3 | 3.3E3 | 5.8E4 | 1.6E5 | 69 | 7 | 69 | 7 | 0.69 |
| kI | pg/ml | 2.0E2 | 1.5E2 | 2.2E2 | 1.9E2 | 1.2E2 | 9.4E1 | 1.0E-9 | 7.2E1 | 6.7E2 | 3.4E2 | 69 | 7 | 69 | 7 | 0.44 |
| kK | pg/ml | 1.2E2 | 1.1E2 | 1.8E2 | 1.8E2 | 2.6E2 | 1.7E2 | 6.4E0 | 3.4E1 | 1.9E3 | 5.5E2 | 69 | 7 | 69 | 7 | 0.53 |
| kN | pg/ml | 1.1E3 | 1.1E3 | 1.5E3 | 2.1E3 | 1.6E3 | 2.9E3 | 7.3E1 | 7.0E2 | 1.0E4 | 8.7E3 | 69 | 7 | 69 | 7 | 0.51 |
| kO | pg/ml | 7.1E3 | 6.2E3 | 9.6E3 | 8.0E3 | 1.7E4 | 3.4E3 | 3.7E3 | 5.0E3 | 1.5E5 | 1.4E4 | 69 | 7 | 69 | 7 | 0.52 |
| kP | pg/ml | 5.8E3 | 4.9E3 | 6.8E3 | 5.9E3 | 4.3E3 | 4.5E3 | 9.6E2 | 1.6E3 | 2.7E4 | 1.5E4 | 69 | 7 | 69 | 7 | 0.42 |
| oO | pg/ml | 8.8E4 | 8.9E4 | 1.0E5 | 1.6E5 | 6.2E4 | 1.5E5 | 3.3E3 | 3.8E4 | 3.0E5 | 4.0E5 | 62 | 7 | 62 | 7 | 0.54 |
| oP | pg/ml | 1.3E5 | 2.0E5 | 1.5E5 | 2.3E5 | 8.8E4 | 1.8E5 | 2.4E4 | 5.0E4 | 4.2E5 | 5.7E5 | 62 | 7 | 62 | 7 | 0.65 |
| oQ | pg/ml | 3.0E3 | 4.0E3 | 3.6E3 | 9.9E3 | 3.0E3 | 1.1E4 | 7.7E2 | 2.0E3 | 2.1E4 | 3.2E4 | 62 | 7 | 62 | 7 | 0.67 |

Figure 30.

Descriptive statistics for the assays used to form the panels presented in this figure.

| Code | Units | Median | | Average | | Standard Deviation | | Minimum | | Maximum | | Number of Samples | | Number of Patients | | AUC |
|------|-------|--------|-----|---------|-----|--------|-----|--------|-----|--------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Aa | mg/dL | 7.5E1 | 1.3E2 | 8.5E1 | 1.4E2 | 5.7E1 | 9.8E1 | 7.0E0 | 2.1E1 | 4.0E2 | 4.8E2 | 228 | 44 | 228 | 44 | 0.70 |
| Ad | ug/mL | 4.7E-2 | 8.1E-2 | 7.1E-2 | 3.8E-1 | 8.9E-2 | 1.5E0 | 6.8E-4 | 4.3E-3 | 5.4E-1 | 8.5E0 | 112 | 30 | 112 | 30 | 0.63 |
| Af | ng/mL | 1.3E0 | 6.0E-1 | 1.1E1 | 4.4E0 | 5.3E1 | 7.0E0 | 1.7E-3 | 1.7E-3 | 5.3E2 | 2.3E1 | 112 | 30 | 112 | 30 | 0.43 |
| Aj | ug/mL | 1.4E0 | 2.9E-1 | 2.5E0 | 1.4E0 | 2.5E0 | 2.2E0 | 1.5E-3 | 2.1E-3 | 6.1E0 | 6.1E0 | 112 | 30 | 112 | 30 | 0.36 |
| Al | mg/mL | 8.7E-5 | 1.2E-4 | 2.5E-4 | 3.3E-4 | 4.2E-4 | 4.8E-4 | 4.6E-6 | 7.6E-6 | 1.8E-3 | 1.8E-3 | 112 | 30 | 112 | 30 | 0.56 |
| An | U/mL | 6.3E1 | 9.5E1 | 2.4E2 | 4.9E2 | 6.4E2 | 1.4E3 | 6.1E-1 | 6.4E-1 | 5.5E3 | 7.8E3 | 112 | 30 | 112 | 30 | 0.58 |
| Ao | pg/mL | 9.1E1 | 1.2E2 | 7.1E2 | 1.8E2 | 4.4E3 | 1.8E2 | 2.8E0 | 5.4E0 | 3.9E4 | 6.9E2 | 112 | 30 | 112 | 30 | 0.60 |
| Ap | ng/mL | 3.1E1 | 4.6E1 | 4.1E1 | 5.5E1 | 4.4E1 | 4.6E1 | 2.0E0 | 6.4E0 | 2.9E2 | 2.1E2 | 112 | 30 | 112 | 30 | 0.61 |
| Ar | ng/mL | 5.3E-1 | 2.3E0 | 2.6E0 | 6.8E0 | 6.5E0 | 1.2E1 | 4.1E-2 | 3.4E-3 | 5.1E1 | 5.0E1 | 112 | 30 | 112 | 30 | 0.72 |
| As | ng/mL | 8.7E-3 | 1.1E-2 | 1.3E-2 | 5.3E-2 | 1.8E-2 | 2.2E-1 | 1.7E-3 | 1.7E-3 | 1.3E-1 | 1.2E0 | 112 | 30 | 112 | 30 | 0.53 |
| Aw | pg/mL | 1.6E1 | 1.6E1 | 1.7E1 | 1.8E1 | 6.0E0 | 8.0E0 | 2.9E-2 | 1.1E1 | 4.2E1 | 5.1E1 | 112 | 30 | 112 | 30 | 0.53 |
| Ax | ng/mL | 1.8E0 | 8.0E0 | 1.6E1 | 1.1E2 | 7.5E1 | 2.1E2 | 1.3E-2 | 1.2E-2 | 7.7E2 | 8.5E2 | 112 | 30 | 112 | 30 | 0.69 |
| Ba | ng/mL | 7.8E1 | 2.7E2 | 5.7E2 | 8.8E2 | 1.5E3 | 1.7E3 | 1.1E0 | 3.1E0 | 8.1E3 | 8.1E3 | 112 | 30 | 112 | 30 | 0.64 |
| Bb | ng/mL | 3.9E0 | 7.9E0 | 6.3E0 | 1.1E1 | 7.9E0 | 9.4E0 | 4.1E-3 | 6.8E-1 | 4.9E1 | 3.7E1 | 112 | 30 | 112 | 30 | 0.67 |
| Bc | ng/mL | 3.3E1 | 6.7E1 | 1.2E2 | 1.7E2 | 2.3E2 | 2.8E2 | 4.9E-1 | 5.6E0 | 1.2E3 | 1.0E3 | 112 | 30 | 112 | 30 | 0.63 |
| Bg | ng/mL | 1.1E-1 | 1.8E-1 | 8.8E0 | 1.3E0 | 4.2E1 | 2.2E0 | 5.3E-4 | 1.9E-2 | 4.0E2 | 8.0E0 | 112 | 30 | 112 | 30 | 0.58 |
| Bn | ng/mL | 5.6E-2 | 5.6E-2 | 1.2E0 | 2.7E0 | 2.1E0 | 1.1E1 | 5.6E-2 | 5.6E-2 | 8.6E0 | 5.8E1 | 112 | 30 | 112 | 30 | 0.47 |
| Bo | ng/mL | 1.3E1 | 1.8E1 | 1.5E1 | 1.7E1 | 1.1E1 | 1.2E1 | 1.6E-2 | 1.6E-2 | 5.0E1 | 5.3E1 | 112 | 30 | 112 | 30 | 0.56 |
| Ch | uIU/mL | 1.2E0 | 9.6E-1 | 5.0E1 | 4.2E0 | 2.2E2 | 1.0E1 | 3.4E-3 | 3.4E-3 | 1.8E3 | 5.0E1 | 112 | 30 | 112 | 30 | 0.40 |
| Co | pg/mL | 4.6E1 | 6.1E1 | 2.8E2 | 1.7E2 | 1.6E3 | 3.9E2 | 1.5E-1 | 8.1E0 | 1.7E4 | 2.1E3 | 112 | 30 | 112 | 30 | 0.63 |
| Cp | ng/mL | 2.2E1 | 2.2E1 | 2.8E1 | 7.4E1 | 2.1E1 | 2.3E2 | 6.0E-1 | 2.5E0 | 1.3E2 | 1.3E3 | 112 | 30 | 112 | 30 | 0.54 |
| Cq | ng/mL | 3.0E-2 | 3.8E-2 | 1.2E-1 | 1.8E0 | 5.3E-1 | 8.9E0 | 8.0E-4 | 8.0E-4 | 5.1E0 | 4.9E1 | 112 | 30 | 112 | 30 | 0.57 |
| Cs | ng/mL | 6.6E1 | 2.4E2 | 3.5E2 | 1.4E3 | 1.1E3 | 3.4E3 | 1.0E0 | 8.3E-1 | 1.1E4 | 1.8E4 | 112 | 30 | 112 | 30 | 0.67 |
| Ct | ng/mL | 3.6E-1 | 2.1E-1 | 5.0E1 | 2.7E1 | 1.4E2 | 8.8E1 | 1.5E-2 | 1.1E-4 | 6.2E2 | 4.7E2 | 112 | 30 | 112 | 30 | 0.45 |
| Cu | ng/mL | 2.5E-1 | 3.1E-1 | 4.1E-1 | 3.6E0 | 5.6E-1 | 1.2E1 | 2.8E-2 | 4.6E-2 | 4.5E0 | 6.6E1 | 112 | 30 | 112 | 30 | 0.62 |
| Cv | ng/mL | 3.9E0 | 1.3E1 | 2.2E1 | 4.7E1 | 6.0E1 | 9.7E1 | 2.0E-2 | 2.4E-2 | 5.3E2 | 4.7E2 | 112 | 30 | 112 | 30 | 0.59 |
| Cw | mIU/mL | 3.0E-2 | 4.5E-2 | 4.1E-2 | 2.7E-1 | 3.6E-2 | 1.2E0 | 8.9E-4 | 4.1E-3 | 2.3E-1 | 6.8E0 | 112 | 30 | 112 | 30 | 0.61 |
| Cx | ng/mL | 1.5E0 | 1.7E-2 | 4.7E1 | 4.5E1 | 9.4E1 | 9.6E1 | 9.3E-5 | 9.3E-5 | 3.9E2 | 2.8E2 | 112 | 30 | 112 | 30 | 0.42 |
| Db | ug/mL | 7.2E0 | 7.8E0 | 8.6E0 | 8.5E0 | 8.1E0 | 6.3E0 | 4.5E-1 | 8.8E-1 | 5.9E1 | 3.1E1 | 112 | 30 | 112 | 30 | 0.52 |
| Dc | nmol/L | 1.9E-2 | 2.7E-2 | 5.1E-2 | 6.8E-1 | 1.5E-1 | 2.6E0 | 5.2E-6 | 1.3E-3 | 1.6E0 | 1.4E1 | 112 | 30 | 112 | 30 | 0.66 |
| Dd | ug/mL | 6.3E-2 | 2.4E-1 | 1.7E-1 | 3.8E-1 | 2.6E-1 | 6.8E-1 | 4.8E-4 | 3.4E-3 | 1.6E0 | 3.6E0 | 112 | 30 | 112 | 30 | 0.64 |
| De | ng/mL | 3.4E-3 | 3.4E-3 | 8.5E-2 | 9.0E-2 | 1.4E-1 | 2.5E-1 | 3.4E-3 | 3.4E-3 | 9.2E-1 | 1.1E0 | 112 | 30 | 112 | 30 | 0.43 |
| Dg | ng/mL | 3.4E1 | 4.2E1 | 4.5E1 | 5.7E1 | 3.8E1 | 4.2E1 | 7.1E-1 | 2.3E0 | 1.9E2 | 1.5E2 | 112 | 30 | 112 | 30 | 0.59 |
| Di | pg/mL | 2.0E0 | 2.7E0 | 2.4E0 | 2.8E0 | 2.2E0 | 1.9E0 | 1.8E-1 | 1.8E-1 | 1.3E1 | 9.0E0 | 112 | 30 | 112 | 30 | 0.58 |
| Dk | uIU/mL | 1.5E-2 | 1.9E-2 | 5.9E-2 | 1.1E-1 | 1.8E-1 | 2.3E-1 | 1.1E-4 | 1.1E-4 | 1.6E0 | 9.8E-1 | 112 | 30 | 112 | 30 | 0.58 |
| Dl | ng/mL | 1.9E2 | 3.3E2 | 2.9E2 | 4.1E2 | 2.6E2 | 3.7E2 | 5.5E0 | 1.8E1 | 1.2E3 | 1.6E3 | 112 | 30 | 112 | 30 | 0.60 |
| Dp | ng/ml | 2.4E0 | 1.8E0 | 5.4E0 | 5.5E0 | 8.3E0 | 1.2E1 | 3.7E-3 | 3.7E-3 | 4.6E1 | 5.6E1 | 92 | 22 | 92 | 22 | 0.42 |
| Dr | pg/ml | 1.7E1 | 4.9E1 | 4.0E1 | 1.2E3 | 5.5E1 | 3.3E3 | 7.5E-1 | 7.5E-1 | 2.5E2 | 1.0E4 | 48 | 10 | 48 | 10 | 0.70 |
| Du | pg/ml | 1.4E2 | 7.0E2 | 1.1E3 | 3.6E3 | 3.2E3 | 8.1E3 | 1.2E0 | 1.2E0 | 2.0E4 | 2.4E4 | 40 | 8 | 40 | 8 | 0.63 |
| Ef | ng/ml | 9.5E-2 | 1.9E-1 | 8.3E-1 | 9.4E-1 | 1.8E0 | 2.3E0 | 5.7E-4 | 5.7E-4 | 9.5E0 | 9.9E0 | 99 | 25 | 99 | 25 | 0.56 |
| Wm | % | 8.5E-2 | 1.1E0 | 1.1E1 | 8.6E1 | 7.8E1 | 2.6E2 | 5.4E-2 | 8.5E-2 | 7.7E2 | 1.0E3 | 105 | 26 | 105 | 26 | 0.59 |
| Ed | pg/ml | 1.1E0 | 2.5E1 | 2.6E1 | 9.1E1 | 3.8E1 | 1.4E2 | 5.2E-1 | 5.2E-1 | 1.9E2 | 5.0E2 | 92 | 22 | 92 | 22 | 0.65 |
| Yf | ng/mL | 1.4E1 | 2.4E1 | 4.6E1 | 1.0E2 | 9.9E1 | 1.5E2 | 2.9E-1 | 2.9E-1 | 5.9E2 | 3.5E2 | 42 | 7 | 42 | 7 | 0.61 |
| Tj | pg/mL | 3.7E-1 | 5.0E-1 | 4.6E1 | 3.8E1 | 2.5E2 | 8.2E1 | 3.7E-1 | 3.7E-1 | 2.3E3 | 3.1E2 | 96 | 24 | 96 | 24 | 0.59 |
| Po | pg/ml | 1.4E-1 | 4.5E0 | 8.1E0 | 2.4E1 | 2.6E1 | 4.7E1 | 8.0E-3 | 2.6E-2 | 2.7E2 | 2.2E2 | 214 | 55 | 214 | 55 | 0.66 |
| Ti | ug/mL | 2.9E0 | 7.2E0 | 4.3E0 | 8.1E0 | 3.7E0 | 5.6E0 | 1.2E-1 | 9.7E-1 | 1.4E1 | 1.8E1 | 59 | 10 | 59 | 10 | 0.71 |
| Em | ng/ml | 1.3E-2 | 2.6E-2 | 4.8E-2 | 2.3E-1 | 8.2E-2 | 5.0E-1 | 8.4E-4 | 8.4E-4 | 5.0E-1 | 1.9E0 | 58 | 15 | 58 | 15 | 0.57 |
| Et | ng/ml | 1.3E3 | 2.9E3 | 1.6E3 | 2.7E3 | 1.1E3 | 1.3E3 | 7.9E1 | 5.9E2 | 4.3E3 | 5.0E3 | 213 | 55 | 213 | 55 | 0.75 |
| Eq | pg/ml | 2.0E2 | 5.6E1 | 3.7E2 | 2.4E2 | 4.0E2 | 4.9E2 | 1.0E0 | 1.0E0 | 1.5E3 | 1.4E3 | 40 | 8 | 40 | 8 | 0.33 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Th | ug/mL | 1.3E0 | 1.0E0 | 1.9E0 | 1.9E0 | 1.6E0 | 2.1E0 | 1.2E-1 | 2.6E-3 | 7.5E0 | 5.9E0 | 59 | 10 | 59 | 10 | 0.43 |
| Fa | ng/ml | 3.8E1 | 9.5E1 | 1.0E2 | 2.5E2 | 3.9E2 | 5.3E2 | 2.6E-1 | 6.0E-1 | 3.7E3 | 2.5E3 | 89 | 22 | 89 | 22 | 0.72 |
| Ez | ng/ml | 3.8E0 | 5.3E0 | 1.4E1 | 2.3E1 | 2.5E1 | 4.6E1 | 1.3E-2 | 9.5E-2 | 1.6E2 | 2.0E2 | 92 | 22 | 92 | 22 | 0.59 |
| Fb | ng/ml | 2.6E1 | 2.7E1 | 2.2E1 | 2.7E1 | 1.2E1 | 9.1E0 | 6.6E-1 | 8.1E-1 | 4.3E1 | 4.1E1 | 90 | 22 | 90 | 22 | 0.59 |
| Ex | ng/ml | 7.5E-2 | 1.7E-1 | 1.7E-1 | 2.7E-1 | 2.6E-1 | 3.2E-1 | 3.5E-5 | 1.7E-4 | 1.5E0 | 1.2E0 | 68 | 20 | 68 | 20 | 0.66 |
| Fc | pg/ml | 2.2E-1 | 3.3E0 | 1.3E1 | 4.8E1 | 6.2E1 | 1.1E2 | 2.2E-1 | 2.2E-1 | 3.9E2 | 3.1E2 | 41 | 8 | 41 | 8 | 0.66 |
| Fd | pg/ml | 2.3E1 | 5.5E2 | 3.4E2 | 7.2E3 | 6.2E2 | 1.1E4 | 4.5E-1 | 9.8E-1 | 2.7E3 | 2.5E4 | 41 | 8 | 41 | 8 | 0.73 |
| Fi | pg/ml | 2.5E-1 | 3.1E1 | 6.2E1 | 1.5E2 | 2.9E2 | 2.0E2 | 2.5E-1 | 2.5E-1 | 1.8E3 | 5.3E2 | 41 | 8 | 41 | 8 | 0.68 |
| Fn | ng/ml | 2.1E-1 | 9.5E-1 | 4.2E0 | 5.2E0 | 7.6E0 | 7.4E0 | 1.1E-14 | 1.1E-14 | 3.7E1 | 2.7E1 | 92 | 22 | 92 | 22 | 0.53 |
| Fp | ng/ml | 1.1E1 | 3.2E1 | 2.2E1 | 3.8E1 | 2.6E1 | 3.3E1 | 6.0E-3 | 1.2E0 | 1.3E2 | 1.3E2 | 217 | 55 | 217 | 55 | 0.68 |
| Fr | ng/ml | 3.1E4 | 9.2E4 | 1.2E5 | 2.2E5 | 1.9E5 | 2.5E5 | 1.9E2 | 1.8E3 | 8.4E5 | 8.4E5 | 220 | 56 | 220 | 56 | 0.69 |
| Fw | pg/ml | 8.5E-1 | 1.2E1 | 4.3E1 | 8.6E1 | 3.0E2 | 2.0E2 | 1.2E-1 | 1.2E-1 | 3.0E3 | 9.1E2 | 99 | 25 | 99 | 25 | 0.65 |
| Fy | ng/ml | 3.6E1 | 5.7E1 | 5.6E1 | 1.6E2 | 5.7E1 | 2.1E2 | 1.2E-1 | 1.5E0 | 2.8E2 | 6.5E2 | 92 | 20 | 92 | 20 | 0.62 |
| Gh | pg/ml | 2.0E0 | 2.3E0 | 2.2E1 | 1.0E1 | 5.9E1 | 1.7E1 | 2.9E-2 | 2.9E-2 | 3.2E2 | 4.6E1 | 41 | 8 | 41 | 8 | 0.52 |
| Gb | % | 4.3E1 | 5.7E1 | 4.4E1 | 9.7E1 | 3.1E1 | 9.5E1 | 2.2E0 | 2.5E1 | 1.4E2 | 3.0E2 | 41 | 8 | 41 | 8 | 0.70 |
| Gc | ng/ml | 8.2E1 | 2.0E2 | 1.6E2 | 2.9E2 | 2.2E2 | 2.2E2 | 6.4E0 | 6.7E1 | 1.2E3 | 6.7E2 | 48 | 10 | 48 | 10 | 0.73 |
| Gd | ng/ml | 3.6E1 | 2.5E1 | 3.6E1 | 3.0E1 | 1.7E1 | 2.3E1 | 3.0E0 | 7.6E0 | 6.9E1 | 8.0E1 | 56 | 11 | 56 | 11 | 0.37 |
| Gn | U/ml | 2.2E-1 | 2.8E-1 | 1.4E0 | 1.2E1 | 4.5E0 | 3.6E1 | 5.6E-3 | 2.7E-2 | 3.0E1 | 1.1E2 | 47 | 10 | 47 | 10 | 0.55 |
| Gl | pg/ml | 9.2E3 | 1.0E4 | 1.2E4 | 1.4E4 | 9.5E3 | 1.0E4 | 9.1E1 | 5.2E2 | 3.1E4 | 3.1E4 | 99 | 25 | 99 | 25 | 0.57 |
| Gp | U/ml | 1.2E0 | 9.8E-1 | 2.4E0 | 1.7E0 | 3.2E0 | 2.4E0 | 1.5E-2 | 1.5E-2 | 1.8E1 | 7.8E0 | 99 | 25 | 99 | 25 | 0.42 |
| Gz | ug/ml | 1.5E0 | 9.7E-1 | 5.8E0 | 4.5E0 | 5.9E0 | 6.0E0 | 4.2E-2 | 1.0E-1 | 2.5E1 | 1.9E1 | 62 | 18 | 62 | 18 | 0.49 |
| Ha | ng/ml | 1.9E0 | 4.1E0 | 7.5E0 | 1.7E1 | 1.7E1 | 3.3E1 | 1.7E-2 | 1.7E-2 | 1.0E2 | 1.0E2 | 90 | 22 | 90 | 22 | 0.61 |
| Nm | pg/ml | 1.4E4 | 2.5E4 | 3.4E4 | 7.2E4 | 8.7E4 | 1.4E5 | 1.0E-9 | 1.0E-9 | 9.6E5 | 8.2E5 | 217 | 55 | 217 | 55 | 0.61 |
| Nn | pg/ml | 1.5E2 | 4.3E2 | 1.8E3 | 1.1E4 | 8.2E3 | 4.5E4 | 1.0E-9 | 9.5E0 | 9.5E4 | 3.1E5 | 217 | 55 | 217 | 55 | 0.66 |
| No | pg/ml | 1.5E1 | 3.3E1 | 2.8E1 | 1.1E2 | 5.1E1 | 2.0E2 | 1.0E-9 | 4.0E0 | 5.6E2 | 9.1E2 | 217 | 55 | 217 | 55 | 0.71 |
| Nq | pg/ml | 1.4E0 | 7.5E0 | 2.5E1 | 4.9E1 | 8.2E1 | 1.2E2 | 1.0E-9 | 1.0E-9 | 6.7E2 | 6.3E2 | 217 | 55 | 217 | 55 | 0.61 |
| Nr | pg/ml | 1.3E0 | 6.1E0 | 2.0E1 | 7.4E1 | 8.2E1 | 2.1E2 | 1.0E-9 | 1.0E-9 | 9.8E2 | 1.4E3 | 217 | 55 | 217 | 55 | 0.66 |
| Ns | pg/ml | 1.0E-9 | 1.0E-9 | 1.2E1 | 6.5E-2 | 8.1E1 | 4.8E-1 | 1.0E-9 | 1.0E-9 | 1.1E3 | 3.6E0 | 217 | 55 | 217 | 55 | 0.47 |
| Nt | pg/ml | 1.1E2 | 1.4E2 | 1.4E2 | 2.3E2 | 1.0E2 | 2.8E2 | 1.5E1 | 4.5E1 | 8.8E2 | 1.7E3 | 217 | 55 | 217 | 55 | 0.63 |
| Nu | pg/ml | 2.4E1 | 4.8E1 | 5.7E1 | 8.3E1 | 8.8E1 | 9.8E1 | 1.0E-9 | 1.0E-9 | 6.3E2 | 3.7E2 | 217 | 55 | 217 | 55 | 0.60 |
| Lu | pg/ml | 1.0E4 | 1.0E4 | 1.8E4 | 1.1E4 | 4.9E4 | 9.4E3 | 9.4E2 | 5.2E2 | 5.6E5 | 5.7E4 | 217 | 55 | 217 | 55 | 0.48 |
| Lv | pg/ml | 1.0E-9 | 6.8E0 | 1.5E1 | 3.2E1 | 2.8E1 | 5.1E1 | 1.0E-9 | 1.0E-9 | 2.4E2 | 2.6E2 | 217 | 55 | 217 | 55 | 0.59 |
| Lw | pg/ml | 1.0E-9 | 1.0E-9 | 5.4E-1 | 4.8E0 | 5.8E0 | 2.5E1 | 1.0E-9 | 1.0E-9 | 8.0E1 | 1.8E2 | 217 | 55 | 217 | 55 | 0.55 |
| Lx | pg/ml | 1.0E-9 | 1.7E2 | 1.6E2 | 9.2E2 | 5.8E2 | 3.1E3 | 1.0E-9 | 1.0E-9 | 6.2E3 | 2.2E4 | 217 | 55 | 217 | 55 | 0.72 |
| Ly | pg/ml | 1.0E-9 | 1.0E-9 | 9.8E0 | 8.4E0 | 1.9E1 | 1.5E1 | 1.0E-9 | 1.0E-9 | 1.1E2 | 6.0E1 | 217 | 55 | 217 | 55 | 0.49 |
| Lz | pg/ml | 1.0E-9 | 1.0E-9 | 3.2E0 | 4.9E0 | 3.1E1 | 1.4E1 | 1.0E-9 | 1.0E-9 | 4.3E2 | 6.2E1 | 217 | 55 | 217 | 55 | 0.57 |
| Ma | pg/ml | 4.5E2 | 1.2E3 | 2.4E3 | 4.0E3 | 6.7E3 | 7.3E3 | 1.0E-9 | 2.6E1 | 6.5E4 | 3.6E4 | 217 | 55 | 217 | 55 | 0.65 |
| Mb | pg/ml | 2.5E1 | 2.8E1 | 3.3E1 | 3.2E1 | 1.9E1 | 1.5E1 | 9.2E0 | 4.1E0 | 2.1E2 | 7.1E1 | 217 | 55 | 217 | 55 | 0.50 |
| Mc | pg/ml | 1.0E-9 | 1.0E-9 | 8.8E-2 | 1.0E-9 | 9.3E-1 | 0.0E0 | 1.0E-9 | 1.0E-9 | 1.3E1 | 1.0E-9 | 217 | 55 | 217 | 55 | 0.49 |
| Md | pg/ml | 1.0E-9 | 1.0E-9 | 5.9E-1 | 7.5E-1 | 4.7E0 | 4.0E0 | 1.0E-9 | 1.0E-9 | 6.5E1 | 2.9E1 | 217 | 55 | 217 | 55 | 0.51 |
| Me | pg/ml | 3.0E1 | 3.5E1 | 3.1E1 | 3.4E1 | 2.7E1 | 2.6E1 | 1.0E-9 | 1.0E-9 | 3.2E2 | 1.8E2 | 217 | 55 | 217 | 55 | 0.56 |
| Mf | pg/ml | 1.0E-9 | 1.0E-9 | 8.4E-1 | 5.1E-1 | 4.8E0 | 1.5E0 | 1.0E-9 | 1.0E-9 | 5.6E1 | 9.1E0 | 217 | 55 | 217 | 55 | 0.51 |
| Mg | pg/ml | 1.3E0 | 1.8E0 | 7.1E0 | 1.1E1 | 1.3E1 | 2.6E1 | 1.0E-9 | 1.0E-9 | 9.2E1 | 1.5E2 | 217 | 55 | 217 | 55 | 0.54 |
| Mh | pg/ml | 1.0E-9 | 1.0E-9 | 9.1E-1 | 1.9E0 | 7.6E0 | 6.5E0 | 1.0E-9 | 1.0E-9 | 1.1E2 | 4.2E1 | 217 | 55 | 217 | 55 | 0.57 |
| Mi | pg/ml | 1.0E-9 | 1.0E-9 | 1.2E0 | 1.9E1 | 1.0E1 | 8.2E1 | 1.0E-9 | 1.0E-9 | 1.2E2 | 5.2E2 | 217 | 55 | 217 | 55 | 0.56 |
| Mj | pg/ml | 1.0E-9 | 1.0E-9 | 5.8E0 | 1.5E1 | 3.3E1 | 4.0E1 | 1.0E-9 | 1.0E-9 | 4.6E2 | 2.1E2 | 217 | 55 | 217 | 55 | 0.57 |
| Mk | pg/ml | 1.3E0 | 3.7E0 | 2.0E1 | 1.8E1 | 1.2E2 | 7.0E1 | 1.0E-9 | 1.0E-9 | 1.3E3 | 5.0E2 | 217 | 55 | 217 | 55 | 0.53 |
| Ml | pg/ml | 1.0E-9 | 1.0E-9 | 1.2E1 | 1.9E1 | 1.4E2 | 8.3E1 | 1.0E-9 | 1.0E-9 | 2.1E3 | 5.1E2 | 217 | 55 | 217 | 55 | 0.51 |
| Mm | pg/ml | 5.5E2 | 9.4E2 | 1.0E3 | 1.8E3 | 1.2E3 | 2.1E3 | 1.0E-9 | 5.2E1 | 6.3E3 | 1.0E4 | 217 | 55 | 217 | 55 | 0.63 |
| Mn | pg/ml | 5.6E0 | 1.1E1 | 1.2E1 | 1.3E1 | 3.0E1 | 8.9E0 | 1.0E-9 | 2.4E0 | 3.5E2 | 5.1E1 | 217 | 55 | 217 | 55 | 0.69 |
| Mp | pg/ml | 1.0E-9 | 6.8E0 | 1.2E1 | 8.1E1 | 4.7E1 | 3.3E2 | 1.0E-9 | 1.0E-9 | 6.3E2 | 2.4E3 | 217 | 55 | 217 | 55 | 0.63 |
| Mq | pg/ml | 1.0E-9 | 1.0E-9 | 2.4E0 | 6.1E0 | 1.5E1 | 1.9E1 | 1.0E-9 | 1.0E-9 | 1.8E2 | 1.0E2 | 217 | 55 | 217 | 55 | 0.55 |
| Mr | pg/ml | 1.0E-9 | 1.0E-9 | 2.0E1 | 1.9E2 | 1.0E2 | 6.0E2 | 1.0E-9 | 1.0E-9 | 1.4E3 | 3.4E3 | 217 | 55 | 217 | 55 | 0.59 |

| Code | Units | Median | | Average | | Standard Deviation | | Minimum | | Maximum | | Number of Samples | | Number of Patients | | AUC |
|------|-------|--------|------|---------|------|------|------|---------|------|---------|------|--------|-----|--------|-----|-----|
| | | NonDis | Dis | NonDis | Dis | NonDis | Dis | NonDis | Dis | NonDis | Dis | NonDis | Dis | NonDis | Dis | |
| Ms | pg/ml | 3.6E2 | 2.5E2 | 4.8E2 | 3.4E2 | 5.4E2 | 3.3E2 | 1.0E-9 | 1.0E-9 | 3.3E3 | 1.5E3 | 217 | 55 | 217 | 55 | 0.43 |
| Mt | pg/ml | 3.1E-1 | 1.6E0 | 9.0E0 | 8.5E1 | 5.2E1 | 4.4E2 | 1.0E-9 | 1.0E-9 | 7.1E2 | 3.2E3 | 217 | 55 | 217 | 55 | 0.67 |
| Mu | pg/ml | 1.0E-9 | 1.0E-9 | 2.1E0 | 2.5E0 | 1.8E1 | 8.4E0 | 1.0E-9 | 1.0E-9 | 2.3E2 | 4.8E1 | 217 | 55 | 217 | 55 | 0.55 |
| Mv | pg/ml | 1.0E-9 | 1.0E-9 | 8.4E1 | 1.0E2 | 4.1E2 | 2.4E2 | 1.0E-9 | 1.0E-9 | 4.5E3 | 9.2E2 | 217 | 55 | 217 | 55 | 0.56 |
| Mw | pg/ml | 3.0E1 | 9.8E1 | 3.3E2 | 5.1E2 | 1.7E3 | 1.1E3 | 1.0E-9 | 1.0E-9 | 1.8E4 | 5.3E3 | 217 | 55 | 217 | 55 | 0.66 |
| Mx | pg/ml | 1.0E-9 | 1.0E-9 | 4.0E-1 | 9.7E-1 | 2.3E0 | 2.9E0 | 1.0E-9 | 1.0E-9 | 3.2E1 | 2.0E1 | 217 | 55 | 217 | 55 | 0.60 |
| My | pg/ml | 1.0E-9 | 5.8E0 | 4.7E2 | 1.5E2 | 3.1E3 | 3.8E2 | 1.0E-9 | 1.0E-9 | 3.9E4 | 1.9E3 | 217 | 55 | 217 | 55 | 0.58 |
| Mz | pg/ml | 1.0E1 | 3.0E1 | 2.3E1 | 1.1E2 | 5.2E1 | 3.1E2 | 1.0E-9 | 1.0E-9 | 5.5E2 | 1.9E3 | 217 | 55 | 217 | 55 | 0.74 |
| Na | pg/ml | 1.0E-9 | 1.0E-9 | 5.3E-1 | 1.4E0 | 1.7E0 | 5.9E0 | 1.0E-9 | 1.0E-9 | 9.6E0 | 4.2E1 | 217 | 55 | 217 | 55 | 0.50 |
| Nb | pg/ml | 2.1E0 | 3.5E0 | 3.7E0 | 1.1E1 | 1.2E1 | 3.2E1 | 1.0E-9 | 1.0E-9 | 1.5E2 | 2.1E2 | 217 | 55 | 217 | 55 | 0.63 |
| Nc | pg/ml | 3.5E2 | 5.6E1 | 5.4E2 | 3.5E2 | 8.0E2 | 5.7E2 | 1.0E-9 | 1.0E-9 | 9.2E3 | 3.2E3 | 217 | 55 | 217 | 55 | 0.38 |
| Nd | pg/ml | 2.6E1 | 2.5E1 | 2.8E1 | 6.7E1 | 8.4E1 | 2.8E2 | 1.0E-9 | 1.0E-9 | 1.2E3 | 2.1E3 | 217 | 55 | 217 | 55 | 0.56 |
| Ne | pg/ml | 4.4E2 | 3.4E2 | 5.4E2 | 4.3E2 | 6.0E2 | 5.4E2 | 1.0E-9 | 1.0E-9 | 7.0E3 | 3.6E3 | 217 | 55 | 217 | 55 | 0.41 |
| Nf | pg/ml | 1.0E-9 | 1.0E-9 | 2.3E0 | 6.5E0 | 9.0E0 | 2.4E1 | 1.0E-9 | 1.0E-9 | 8.2E1 | 1.3E2 | 217 | 55 | 217 | 55 | 0.51 |
| Ng | pg/ml | 1.8E1 | 1.7E1 | 1.1E2 | 8.2E1 | 2.1E2 | 1.2E2 | 1.0E-9 | 1.0E-9 | 1.6E3 | 5.3E2 | 217 | 55 | 217 | 55 | 0.51 |
| Nh | pg/ml | 6.0E1 | 5.2E1 | 8.3E1 | 6.2E1 | 7.7E1 | 7.5E1 | 1.0E-9 | 4.1E0 | 5.6E2 | 5.1E2 | 217 | 55 | 217 | 55 | 0.39 |
| Ni | pg/ml | 4.5E0 | 1.0E-9 | 8.5E1 | 1.0E2 | 1.3E2 | 1.9E2 | 1.0E-9 | 1.0E-9 | 7.1E2 | 1.1E3 | 217 | 55 | 217 | 55 | 0.47 |
| Nj | pg/ml | 6.3E0 | 5.9E0 | 1.0E1 | 8.2E0 | 1.1E1 | 9.7E0 | 1.0E-9 | 1.0E-9 | 6.8E1 | 4.6E1 | 217 | 55 | 217 | 55 | 0.43 |
| Nk | pg/ml | 1.8E1 | 1.2E1 | 3.1E1 | 3.3E1 | 3.6E1 | 4.9E1 | 1.0E-9 | 1.0E-9 | 2.0E2 | 2.0E2 | 217 | 55 | 217 | 55 | 0.48 |
| Nl | pg/ml | 4.3E1 | 3.1E1 | 5.8E1 | 4.2E1 | 8.4E1 | 4.8E1 | 1.0E-9 | 1.0E-9 | 1.1E3 | 2.3E2 | 217 | 55 | 217 | 55 | 0.40 |
| Hl | pg/ml | 2.1E1 | 7.0E0 | 3.9E1 | 4.7E2 | 6.0E1 | 1.3E3 | 1.0E-9 | 1.0E-9 | 3.0E2 | 3.6E3 | 41 | 8 | 41 | 8 | 0.50 |
| Ho | pg/ml | 1.8E1 | 2.7E1 | 2.4E1 | 9.8E1 | 2.0E1 | 1.3E2 | 1.1E0 | 7.6E0 | 8.7E1 | 3.9E2 | 41 | 8 | 41 | 8 | 0.65 |
| Hp | ng/ml | 1.6E0 | 5.5E0 | 9.0E1 | 3.3E2 | 2.7E2 | 4.6E2 | 1.0E-9 | 8.8E-1 | 8.9E2 | 8.9E2 | 41 | 8 | 41 | 8 | 0.70 |
| Tz | pg/ml | 3.9E3 | 6.5E3 | 6.0E3 | 1.2E5 | 6.2E3 | 4.4E5 | 1.0E-9 | 6.8E2 | 3.2E4 | 2.1E6 | 93 | 22 | 93 | 22 | 0.62 |
| Ua | pg/ml | 3.1E3 | 5.6E3 | 1.5E4 | 1.3E4 | 2.9E4 | 2.2E4 | 1.0E-9 | 9.1E2 | 1.4E5 | 9.9E4 | 93 | 22 | 93 | 22 | 0.59 |
| Ub | pg/ml | 5.3E2 | 4.0E2 | 8.5E2 | 6.2E2 | 1.2E3 | 8.6E2 | 1.0E-9 | 2.3E0 | 9.8E3 | 4.1E3 | 93 | 22 | 93 | 22 | 0.41 |
| Ue | pg/ml | 3.1E1 | 2.3E1 | 3.7E1 | 3.7E1 | 3.3E1 | 3.8E1 | 9.8E-2 | 5.9E0 | 2.7E2 | 1.4E2 | 93 | 22 | 93 | 22 | 0.44 |
| Uc | pg/ml | 7.6E2 | 8.7E2 | 1.3E3 | 4.6E3 | 1.5E3 | 1.2E4 | 1.0E-9 | 5.5E1 | 8.3E3 | 5.7E4 | 93 | 22 | 93 | 22 | 0.59 |
| Ud | pg/ml | 1.0E-9 | 1.0E-9 | 4.2E0 | 2.4E0 | 4.0E1 | 1.1E1 | 1.0E-9 | 1.0E-9 | 3.9E2 | 5.3E1 | 93 | 22 | 93 | 22 | 0.52 |
| Hq | pg/ml | 1.2E0 | 1.6E0 | 2.4E2 | 6.8E1 | 2.4E3 | 3.8E2 | 1.0E-9 | 1.0E-9 | 2.8E4 | 2.8E3 | 215 | 55 | 215 | 55 | 0.54 |
| Hr | pg/ml | 8.5E1 | 9.0E1 | 5.7E2 | 5.1E2 | 1.1E3 | 1.4E3 | 1.0E-9 | 1.0E-9 | 8.4E3 | 8.9E3 | 215 | 55 | 215 | 55 | 0.47 |
| Hu | pg/ml | 1.2E1 | 5.3E1 | 7.0E3 | 6.5E2 | 5.0E4 | 1.5E3 | 1.0E-9 | 1.0E-9 | 6.3E5 | 6.5E3 | 215 | 55 | 215 | 55 | 0.56 |
| Hv | pg/ml | 1.5E0 | 1.3E0 | 2.5E0 | 2.3E1 | 5.6E0 | 1.2E2 | 1.0E-9 | 1.0E-9 | 6.9E1 | 8.9E2 | 215 | 55 | 215 | 55 | 0.52 |
| Hw | pg/ml | 5.5E0 | 5.7E0 | 1.6E1 | 2.0E2 | 5.4E1 | 1.3E3 | 1.0E-9 | 5.1E-1 | 6.4E2 | 9.4E3 | 215 | 55 | 215 | 55 | 0.51 |
| Hx | pg/ml | 8.8E0 | 1.4E1 | 6.9E1 | 6.8E1 | 6.3E2 | 2.0E2 | 1.0E-9 | 1.0E-9 | 9.3E3 | 1.3E3 | 215 | 55 | 215 | 55 | 0.59 |
| Ib | ng/ml | 4.0E-2 | 2.8E-2 | 1.3E0 | 2.7E0 | 5.3E0 | 1.2E1 | 1.0E-9 | 1.0E-9 | 3.6E1 | 5.6E1 | 90 | 22 | 90 | 22 | 0.49 |
| Ic | U/ml | 2.2E2 | 2.2E2 | 4.1E2 | 3.2E3 | 1.1E3 | 1.4E4 | 2.4E0 | 2.5E1 | 8.6E3 | 6.5E4 | 90 | 22 | 90 | 22 | 0.60 |
| Id | U/ml | 5.4E-1 | 1.4E0 | 1.0E0 | 2.2E1 | 1.5E0 | 9.2E1 | 1.0E-9 | 2.7E-1 | 1.0E1 | 4.3E2 | 90 | 22 | 90 | 22 | 0.74 |
| Tt | pg/ml | 1.7E2 | 1.8E2 | 1.7E2 | 1.9E2 | 5.5E1 | 7.2E1 | 4.3E1 | 1.1E2 | 3.6E2 | 4.4E2 | 85 | 19 | 85 | 19 | 0.55 |
| To | pg/ml | 1.6E0 | 1.8E0 | 2.0E0 | 2.3E0 | 2.1E0 | 2.8E0 | 1.0E-9 | 1.0E-9 | 9.9E0 | 1.2E1 | 90 | 20 | 90 | 20 | 0.52 |
| Tr | pg/ml | 3.3E0 | 5.2E0 | 9.1E0 | 1.3E1 | 3.3E1 | 1.9E1 | 1.0E-9 | 3.9E-1 | 3.1E2 | 7.6E1 | 88 | 19 | 88 | 19 | 0.62 |
| Tn | pg/ml | 3.3E1 | 5.4E1 | 1.1E2 | 2.3E2 | 3.2E2 | 5.1E2 | 2.4E0 | 1.9E1 | 2.0E3 | 2.3E3 | 90 | 20 | 90 | 20 | 0.67 |
| Tv | ng/ml | 1.2E1 | 1.1E1 | 1.7E1 | 4.4E2 | 1.7E1 | 1.6E3 | 1.0E-9 | 1.0E-9 | 7.5E1 | 7.1E3 | 90 | 20 | 90 | 20 | 0.52 |
| Ih | ng/ml | 6.4E1 | 2.1E2 | 2.0E2 | 5.4E2 | 3.3E2 | 7.4E2 | 1.0E-9 | 1.0E-9 | 2.1E3 | 3.6E3 | 216 | 55 | 216 | 55 | 0.66 |
| Ii | ng/ml | 7.8E1 | 1.3E2 | 2.1E2 | 3.7E2 | 4.7E2 | 7.9E2 | 7.3E-1 | 2.3E0 | 5.2E3 | 4.5E3 | 216 | 55 | 216 | 55 | 0.62 |
| Ij | ng/ml | 7.7E1 | 1.4E2 | 1.9E2 | 7.0E2 | 6.5E2 | 3.3E3 | 2.8E0 | 2.5E1 | 6.4E3 | 2.4E4 | 214 | 54 | 214 | 54 | 0.72 |
| Ik | ng/ml | 1.1E1 | 1.4E1 | 1.9E3 | 2.1E2 | 1.4E4 | 4.1E2 | 5.9E-1 | 2.1E0 | 1.2E5 | 1.5E3 | 214 | 54 | 214 | 54 | 0.54 |
| Il | ng/ml | 3.6E2 | 5.5E2 | 1.2E3 | 2.0E3 | 2.7E3 | 3.6E3 | 1.0E-9 | 1.0E-9 | 1.2E4 | 1.2E4 | 212 | 54 | 212 | 54 | 0.58 |
| Im | ng/ml | 2.0E2 | 7.0E2 | 3.6E2 | 1.1E3 | 5.8E2 | 1.4E3 | 1.4E1 | 4.7E1 | 6.0E3 | 6.2E3 | 213 | 55 | 213 | 55 | 0.76 |
| In | ng/ml | 3.4E0 | 2.4E0 | 1.7E1 | 1.1E2 | 7.9E1 | 6.1E2 | 1.0E-9 | 1.0E-9 | 1.1E3 | 4.5E3 | 216 | 55 | 216 | 55 | 0.49 |
| Hb | ng/ml | 2.1E1 | 4.3E1 | 3.2E1 | 5.7E1 | 3.3E1 | 5.0E1 | 4.8E-1 | 6.7E0 | 2.0E2 | 1.9E2 | 93 | 23 | 93 | 23 | 0.69 |
| Hc | pg/ml | 7.3E2 | 5.0E2 | 3.6E3 | 1.3E3 | 1.2E4 | 2.8E3 | 1.0E-9 | 1.0E-9 | 1.0E5 | 1.4E4 | 93 | 23 | 93 | 23 | 0.42 |
| Hf | ng/ml | 2.0E2 | 2.6E2 | 4.4E2 | 3.0E2 | 6.2E2 | 2.9E2 | 1.0E-9 | 1.0E-9 | 3.2E3 | 9.9E2 | 93 | 23 | 93 | 23 | 0.47 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Io | ng/ml | 1.1E4 | 1.9E4 | 2.1E4 | 2.2E4 | 5.5E4 | 2.2E4 | 1.0E-9 | 6.2E2 | 7.1E5 | 1.1E5 | 216 | 55 | 216 | 55 | 0.61 |
| Ip | ng/ml | 9.3E0 | 3.0E1 | 2.1E1 | 3.2E1 | 2.8E1 | 2.3E1 | 1.0E-9 | 1.6E-2 | 2.3E2 | 8.3E1 | 216 | 55 | 216 | 55 | 0.67 |
| Iq | ug/ml | 1.1E-1 | 1.9E-1 | 7.2E-1 | 6.0E0 | 2.8E0 | 3.0E1 | 1.0E-9 | 1.0E-9 | 3.4E1 | 2.2E2 | 216 | 55 | 216 | 55 | 0.60 |
| Ir | ug/ml | 3.6E-1 | 1.3E0 | 2.0E0 | 1.7E1 | 1.1E1 | 5.6E1 | 1.0E-9 | 1.0E-9 | 1.6E2 | 3.7E2 | 215 | 55 | 215 | 55 | 0.69 |
| Is | ng/ml | 1.7E0 | 7.6E0 | 6.2E0 | 2.7E1 | 1.2E1 | 4.8E1 | 1.0E-9 | 3.3E-2 | 8.8E1 | 2.6E2 | 216 | 55 | 216 | 55 | 0.68 |
| It | ng/ml | 1.8E0 | 5.1E0 | 1.3E1 | 3.3E1 | 6.5E1 | 1.2E2 | 1.0E-9 | 1.0E-9 | 8.3E2 | 6.8E2 | 216 | 55 | 216 | 55 | 0.69 |
| Iu | ng/ml | 1.8E2 | 1.9E2 | 1.1E3 | 2.3E3 | 3.6E3 | 5.7E3 | 1.0E-9 | 1.0E-9 | 2.9E4 | 2.4E4 | 216 | 55 | 216 | 55 | 0.55 |
| Iv | ng/ml | 1.3E1 | 2.3E1 | 3.6E1 | 2.8E2 | 8.0E1 | 8.6E2 | 1.0E-9 | 1.0E-9 | 7.7E2 | 3.8E3 | 215 | 55 | 215 | 55 | 0.63 |
| Iz | ng/ml | 1.2E2 | 1.9E2 | 3.6E2 | 2.7E2 | 7.6E2 | 2.8E2 | 1.5E0 | 4.9E0 | 6.1E3 | 1.0E3 | 93 | 23 | 93 | 23 | 0.55 |
| Yg | pg/ml | 2.4E2 | 1.4E3 | 1.7E3 | 1.5E3 | 7.7E3 | 1.4E3 | 1.0E-9 | 1.3E2 | 5.0E4 | 3.9E3 | 41 | 7 | 41 | 7 | 0.71 |
| Yh | pg/ml | 2.1E2 | 3.6E2 | 3.6E2 | 6.1E2 | 4.8E2 | 6.3E2 | 1.0E-9 | 1.0E-9 | 2.1E3 | 1.4E3 | 41 | 7 | 41 | 7 | 0.59 |
| Yi | pg/ml | 2.4E2 | 1.1E3 | 4.7E2 | 5.9E3 | 5.1E2 | 9.6E3 | 1.0E-9 | 2.4E2 | 2.0E3 | 2.6E4 | 41 | 7 | 41 | 7 | 0.83 |
| Yk | U/ml | 1.0E-9 | 1.0E-9 | 1.8E-1 | 3.7E-1 | 5.8E-1 | 6.4E-1 | 1.0E-9 | 1.0E-9 | 3.0E0 | 1.4E0 | 41 | 7 | 41 | 7 | 0.56 |
| Yj | pg/ml | 1.5E2 | 8.6E1 | 3.3E2 | 1.3E2 | 5.6E2 | 1.1E2 | 1.0E-9 | 5.9E1 | 3.2E3 | 3.7E2 | 41 | 7 | 41 | 7 | 0.38 |
| Yd | ng/ml | 2.1E-1 | 1.3E-1 | 4.0E-1 | 4.1E-1 | 5.2E-1 | 6.5E-1 | 6.6E-3 | 1.7E-2 | 2.3E0 | 1.9E0 | 42 | 8 | 42 | 8 | 0.46 |
| Wb | pg/ml | 2.7E4 | 3.4E4 | 3.4E4 | 1.2E5 | 1.9E4 | 2.1E5 | 6.3E3 | 1.4E4 | 8.4E4 | 6.4E5 | 42 | 8 | 42 | 8 | 0.66 |
| Vz | pg/ml | 3.7E0 | 5.5E0 | 4.7E0 | 6.4E0 | 4.3E0 | 7.1E0 | 1.0E-9 | 7.6E-2 | 2.1E1 | 2.2E1 | 42 | 8 | 42 | 8 | 0.57 |
| Si | ng/ml | 1.0E0 | 2.3E0 | 1.8E0 | 3.0E0 | 2.6E0 | 2.1E0 | 8.6E-3 | 5.6E-1 | 1.0E1 | 6.0E0 | 41 | 8 | 41 | 8 | 0.73 |
| Sf | mIU/mL | 2.0E1 | 2.0E1 | 5.6E1 | 2.7E1 | 1.2E2 | 2.6E1 | 7.8E-1 | 6.7E0 | 7.2E2 | 8.3E1 | 41 | 8 | 41 | 8 | 0.52 |
| Sh | mIU/mL | 1.8E1 | 1.0E1 | 5.1E1 | 1.0E1 | 1.0E2 | 5.1E0 | 7.8E-2 | 4.2E0 | 5.7E2 | 2.1E1 | 41 | 8 | 41 | 8 | 0.36 |
| Sj | ng/ml | 4.4E-1 | 4.4E-1 | 4.3E-1 | 4.5E-1 | 8.3E-2 | 1.2E-1 | 2.5E-1 | 3.4E-1 | 6.1E-1 | 7.2E-1 | 41 | 8 | 41 | 8 | 0.52 |
| Rc | pg/ml | 6.8E3 | 7.6E3 | 7.6E3 | 7.6E3 | 5.5E3 | 3.6E3 | 5.5E2 | 2.1E3 | 2.7E4 | 1.5E4 | 92 | 22 | 92 | 22 | 0.55 |
| Rb | pg/ml | 9.3E-1 | 7.5E-1 | 2.8E0 | 4.2E0 | 4.2E0 | 1.2E1 | 1.0E-9 | 1.0E-9 | 2.0E1 | 5.6E1 | 92 | 22 | 92 | 22 | 0.51 |
| Zq | 2.6ng/ml | 2.6E2 | 4.2E2 | 2.6E2 | 4.9E2 | 1.6E2 | 2.2E2 | 1.7E1 | 3.0E2 | 5.9E2 | 9.7E2 | 41 | 7 | 41 | 7 | 0.80 |
| Zw | 2.5ng/ml | 6.5E0 | 5.0E0 | 9.2E0 | 2.1E1 | 1.1E1 | 2.7E1 | 2.4E-1 | 7.7E-1 | 5.9E1 | 6.3E1 | 42 | 8 | 42 | 8 | 0.56 |
| Zx | 2.3mU/ml | 1.3E-1 | 1.6E-1 | 3.3E-1 | 2.2E-1 | 5.9E-1 | 1.9E-1 | 3.2E-2 | 7.7E-2 | 2.9E0 | 6.7E-1 | 42 | 8 | 42 | 8 | 0.58 |
| Pz | ng/ml | 3.3E3 | 1.0E4 | 5.7E3 | 7.1E3 | 6.5E3 | 4.7E3 | 1.6E1 | 3.3E2 | 7.0E4 | 2.5E4 | 212 | 55 | 212 | 55 | 0.61 |
| Qa | ng/ml | 3.5E3 | 9.0E3 | 6.4E3 | 1.6E4 | 7.4E3 | 3.0E4 | 1.5E2 | 6.8E2 | 4.2E4 | 2.2E5 | 212 | 55 | 212 | 55 | 0.68 |
| Qb | ng/ml | 1.1E2 | 2.1E2 | 2.0E2 | 4.0E2 | 2.9E2 | 6.0E2 | 7.9E-1 | 1.8E1 | 2.8E3 | 4.1E3 | 212 | 55 | 212 | 55 | 0.65 |
| Qc | ng/ml | 2.0E2 | 4.8E2 | 4.1E2 | 6.9E2 | 5.2E2 | 7.7E2 | 1.0E0 | 1.0E-9 | 3.8E3 | 4.3E3 | 212 | 55 | 212 | 55 | 0.63 |
| Qd | ng/ml | 9.3E3 | 2.0E4 | 2.6E4 | 5.4E4 | 1.4E5 | 8.2E4 | 2.4E2 | 1.7E3 | 2.0E6 | 4.3E5 | 212 | 55 | 212 | 55 | 0.70 |
| Qe | ng/ml | 8.1E2 | 2.3E3 | 2.0E3 | 3.2E3 | 6.9E3 | 3.3E3 | 7.6E0 | 8.8E0 | 9.7E4 | 1.8E4 | 212 | 55 | 212 | 55 | 0.70 |
| Jd | ng/ml | 8.6E-1 | 1.4E0 | 4.3E0 | 3.6E0 | 1.7E1 | 5.5E0 | 1.0E-9 | 1.0E-9 | 1.2E2 | 2.1E1 | 92 | 22 | 92 | 22 | 0.62 |
| Je | ng/ml | 1.0E-9 | 5.0E-1 | 2.0E0 | 1.9E0 | 5.7E0 | 2.9E0 | 1.0E-9 | 1.0E-9 | 4.6E1 | 1.1E1 | 92 | 22 | 92 | 22 | 0.58 |
| Jf | ng/ml | 1.0E-9 | 1.0E-9 | 1.4E0 | 8.3E-1 | 2.5E0 | 1.3E0 | 1.0E-9 | 1.0E-9 | 1.2E1 | 3.9E0 | 92 | 22 | 92 | 22 | 0.48 |
| Jg | ng/ml | 4.8E2 | 1.0E3 | 7.9E2 | 1.4E3 | 1.0E3 | 1.3E3 | 5.8E0 | 5.4E1 | 1.0E4 | 7.1E3 | 215 | 55 | 215 | 55 | 0.67 |
| Jh | ng/ml | 2.9E0 | 7.5E0 | 2.5E1 | 3.7E1 | 1.0E2 | 8.5E1 | 1.0E-9 | 1.0E-9 | 1.2E3 | 4.9E2 | 215 | 55 | 215 | 55 | 0.61 |
| Ji | ng/ml | 5.4E1 | 1.3E2 | 7.7E1 | 2.1E2 | 7.9E1 | 2.2E2 | 1.1E0 | 2.0E1 | 5.3E2 | 1.3E3 | 215 | 55 | 215 | 55 | 0.76 |
| Sr | pg/mL | 3.4E2 | 1.2E3 | 7.9E2 | 2.6E3 | 1.1E3 | 4.4E3 | 1.0E-9 | 9.2E1 | 4.8E3 | 2.1E4 | 91 | 22 | 91 | 22 | 0.72 |
| Ss | pg/mL | 1.0E5 | 6.8E4 | 1.5E5 | 1.2E5 | 1.9E5 | 1.4E5 | 2.7E3 | 1.4E4 | 1.3E6 | 5.7E5 | 91 | 22 | 91 | 22 | 0.46 |
| St | pg/mL | 2.4E7 | 8.2E7 | 6.2E7 | 1.6E8 | 1.4E8 | 3.5E8 | 1.0E-9 | 2.3E6 | 1.2E9 | 1.7E9 | 90 | 22 | 90 | 22 | 0.68 |
| Wc | ng/ml | 1.0E-9 | 2.1E-3 | 4.2E-2 | 3.2E-1 | 7.5E-2 | 6.3E-1 | 1.0E-9 | 1.0E-9 | 3.0E-1 | 1.8E0 | 42 | 8 | 42 | 8 | 0.57 |
| Wd | ng/ml | 9.5E0 | 1.6E1 | 2.2E1 | 1.1E2 | 4.7E1 | 1.8E2 | 1.0E0 | 3.5E0 | 2.9E2 | 4.1E2 | 42 | 8 | 42 | 8 | 0.67 |
| We | ng/ml | 3.0E-1 | 5.3E-1 | 9.6E-1 | 3.4E0 | 1.3E0 | 7.9E0 | 1.0E-9 | 1.5E-1 | 5.5E0 | 2.3E1 | 42 | 8 | 42 | 8 | 0.61 |
| Wg | ng/ml | 1.0E-9 | 1.0E-9 | 1.0E-9 | 6.7E-2 | 0.0E0 | 1.9E-1 | 1.0E-9 | 1.0E-9 | 1.0E-9 | 5.3E-1 | 42 | 8 | 42 | 8 | 0.56 |
| Wh | ng/ml | 1.0E-2 | 1.8E-2 | 3.2E-2 | 6.6E-2 | 7.1E-2 | 1.1E-1 | 1.0E-9 | 1.0E-9 | 3.5E-1 | 3.4E-1 | 42 | 8 | 42 | 8 | 0.62 |
| Wf | ng/ml | 1.0E-9 | 1.0E-9 | 1.2E-1 | 2.9E-1 | 2.3E-1 | 8.1E-1 | 1.0E-9 | 1.0E-9 | 1.2E0 | 2.3E0 | 42 | 8 | 42 | 8 | 0.39 |
| Ra | pg/ml | 1.0E-9 | 1.0E-9 | 4.1E-1 | 3.4E0 | 9.9E-1 | 1.4E1 | 1.0E-9 | 1.0E-9 | 3.8E0 | 6.4E1 | 92 | 22 | 92 | 22 | 0.54 |
| Qz | pg/ml | 9.4E0 | 1.0E1 | 5.1E1 | 4.8E1 | 8.8E1 | 7.7E1 | 1.0E-9 | 1.0E-9 | 4.5E2 | 2.3E2 | 92 | 22 | 92 | 22 | 0.49 |
| Qy | pg/ml | 3.8E-1 | 5.6E-1 | 9.6E0 | 3.4E1 | 5.1E1 | 1.6E2 | 1.0E-9 | 1.0E-9 | 4.3E2 | 7.3E2 | 92 | 22 | 92 | 22 | 0.59 |
| Qx | pg/ml | 1.0E-9 | 1.0E-9 | 3.3E0 | 7.9E0 | 2.0E1 | 2.4E1 | 1.0E-9 | 1.0E-9 | 1.9E2 | 1.1E2 | 92 | 22 | 92 | 22 | 0.55 |
| Qw | pg/ml | 4.5E-2 | 1.0E-9 | 3.6E0 | 7.6E-1 | 1.1E1 | 1.4E0 | 1.0E-9 | 1.0E-9 | 6.6E1 | 5.6E0 | 92 | 22 | 92 | 22 | 0.45 |
| Qv | pg/ml | 1.8E4 | 1.1E4 | 3.1E4 | 5.7E4 | 5.2E4 | 2.0E5 | 1.0E-9 | 4.0E2 | 3.7E5 | 9.4E5 | 92 | 22 | 92 | 22 | 0.40 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qu | pg/ml | 8.0E0 | 7.7E0 | 1.0E2 | 7.5E1 | 2.0E2 | 1.6E2 | 1.0E-9 | 1.0E-9 | 9.8E2 | 7.3E2 | 92 | 22 | 92 | 22 | 0.48 |
| Qt | pg/ml | 1.5E1 | 1.4E1 | 4.9E1 | 3.9E1 | 1.1E2 | 6.5E1 | 1.0E-9 | 1.0E-9 | 7.1E2 | 2.2E2 | 92 | 22 | 92 | 22 | 0.49 |
| Qh | ng/ml | 1.8E1 | 3.6E1 | 4.2E1 | 1.0E2 | 6.3E1 | 1.9E2 | 4.3E-1 | 5.1E0 | 3.4E2 | 8.0E2 | 92 | 22 | 92 | 22 | 0.64 |
| Qg | ng/ml | 7.6E0 | 5.1E0 | 1.2E1 | 1.1E1 | 1.3E1 | 1.7E1 | 1.5E-1 | 3.3E-1 | 7.5E1 | 8.1E1 | 92 | 22 | 92 | 22 | 0.43 |
| Jj | ng/ml | 6.1E2 | 2.6E2 | 2.6E3 | 4.9E2 | 2.3E4 | 4.9E2 | 2.0E1 | 1.2E1 | 3.4E5 | 1.9E3 | 215 | 55 | 215 | 55 | 0.35 |
| Jk | ng/ml | 3.0E0 | 4.7E0 | 2.3E1 | 3.1E1 | 5.4E1 | 5.4E1 | 1.0E-9 | 2.4E-1 | 3.9E2 | 2.4E2 | 215 | 55 | 215 | 55 | 0.58 |
| Jl | ng/ml | 4.9E-1 | 1.0E0 | 1.8E0 | 1.9E2 | 4.0E0 | 1.3E3 | 1.2E-3 | 7.8E-2 | 2.6E1 | 9.9E3 | 215 | 55 | 215 | 55 | 0.64 |
| Jm | ng/ml | 2.2E1 | 3.5E1 | 6.0E1 | 9.7E1 | 1.2E2 | 2.8E2 | 1.0E-9 | 4.7E-1 | 1.0E3 | 2.1E3 | 215 | 55 | 215 | 55 | 0.57 |
| Jn | pg/ml | 3.6E-1 | 6.5E-1 | 1.7E0 | 3.1E1 | 5.7E0 | 1.3E2 | 1.0E-9 | 1.0E-9 | 5.8E1 | 7.3E2 | 215 | 55 | 215 | 55 | 0.65 |
| Jo | pg/ml | 4.2E3 | 5.1E3 | 5.1E3 | 8.0E3 | 3.9E3 | 1.4E4 | 2.6E2 | 2.3E2 | 2.4E4 | 1.0E5 | 215 | 55 | 215 | 55 | 0.53 |
| Jp | pg/ml | 7.1E4 | 8.7E4 | 7.4E4 | 9.9E4 | 3.5E4 | 5.2E4 | 2.1E3 | 2.8E4 | 1.9E5 | 3.8E5 | 215 | 55 | 215 | 55 | 0.67 |
| Jq | pg/ml | 9.5E1 | 1.5E2 | 1.5E2 | 4.8E2 | 1.7E2 | 1.3E3 | 5.6E0 | 5.6E0 | 1.1E3 | 8.7E3 | 215 | 55 | 215 | 55 | 0.60 |
| Jr | pg/ml | 2.8E0 | 1.2E1 | 2.6E1 | 3.0E2 | 1.5E2 | 1.2E3 | 1.0E-9 | 1.0E-9 | 1.9E3 | 7.4E3 | 215 | 55 | 215 | 55 | 0.66 |
| Js | pg/ml | 1.4E1 | 2.1E1 | 4.1E1 | 2.2E2 | 1.4E2 | 6.7E2 | 1.0E-9 | 3.0E0 | 1.6E3 | 3.0E3 | 215 | 55 | 215 | 55 | 0.68 |
| Jt | pg/ml | 2.4E3 | 3.4E3 | 2.9E3 | 6.0E3 | 2.0E3 | 9.0E3 | 1.5E2 | 4.1E2 | 1.3E4 | 5.2E4 | 215 | 55 | 215 | 55 | 0.65 |
| Xa | pg/ml | 3.1E-1 | 2.4E1 | 7.7E0 | 2.4E2 | 1.7E1 | 4.4E2 | 1.0E-9 | 2.9E0 | 9.6E1 | 1.2E3 | 42 | 8 | 42 | 8 | 0.85 |
| Ye | pg/ml | 1.0E-9 | 1.0E-9 | 5.4E0 | 9.0E-1 | 1.5E1 | 2.5E0 | 1.0E-9 | 1.0E-9 | 6.9E1 | 7.2E0 | 42 | 8 | 42 | 8 | 0.40 |
| Tm | pg/ml | 1.0E-9 | 1.0E-9 | 3.1E0 | 1.0E0 | 4.8E0 | 3.0E0 | 1.0E-9 | 1.0E-9 | 1.8E1 | 8.4E0 | 42 | 8 | 42 | 8 | 0.39 |
| Tl | pg/ml | 1.3E-1 | 1.0E-9 | 3.6E-1 | 3.2E0 | 4.2E-1 | 8.7E0 | 1.0E-9 | 1.0E-9 | 1.8E0 | 2.5E1 | 42 | 8 | 42 | 8 | 0.36 |
| Ju | mIU/ml | 1.1E1 | 1.6E1 | 2.7E1 | 2.3E1 | 3.8E1 | 2.3E1 | 1.7E-1 | 5.4E0 | 2.3E2 | 1.0E2 | 92 | 22 | 92 | 22 | 0.58 |
| Jv | mIU/ml | 1.9E1 | 1.3E1 | 4.5E1 | 2.8E1 | 6.8E1 | 4.1E1 | 1.7E-2 | 1.5E0 | 4.4E2 | 1.8E2 | 92 | 22 | 92 | 22 | 0.45 |
| Jy | ng/ml | 1.6E-3 | 1.9E-3 | 2.5E-3 | 4.1E-3 | 4.5E-3 | 8.5E-3 | 1.7E-4 | 4.5E-4 | 3.9E-2 | 4.1E-2 | 92 | 22 | 92 | 22 | 0.57 |
| Kc | pg/ml | 2.2E1 | 4.6E1 | 3.4E1 | 8.3E1 | 3.9E1 | 8.7E1 | 1.0E-9 | 1.0E-9 | 1.9E2 | 3.2E2 | 93 | 23 | 93 | 23 | 0.66 |
| Kd | pg/ml | 1.0E-9 | 1.0E-9 | 1.8E2 | 2.4E3 | 6.3E2 | 8.0E3 | 1.0E-9 | 1.0E-9 | 4.8E3 | 3.8E4 | 93 | 23 | 93 | 23 | 0.60 |
| Ke | pg/ml | 1.1E4 | 2.6E4 | 1.3E4 | 4.4E4 | 8.5E3 | 6.5E4 | 6.7E2 | 4.2E3 | 5.5E4 | 3.2E5 | 93 | 23 | 93 | 23 | 0.78 |
| Kf | pg/mL | 5.7E0 | 9.2E0 | 6.2E0 | 1.4E1 | 4.8E0 | 1.7E1 | 1.0E-9 | 1.0E-9 | 2.2E1 | 7.8E1 | 93 | 23 | 93 | 23 | 0.70 |
| Kg | pg/mL | 9.9E2 | 1.0E3 | 1.6E3 | 4.2E3 | 1.5E3 | 9.0E3 | 7.7E1 | 1.3E2 | 8.1E3 | 3.6E4 | 93 | 23 | 93 | 23 | 0.52 |
| Ki | pg/ml | 5.6E1 | 6.4E1 | 6.9E1 | 7.7E1 | 5.6E1 | 5.5E1 | 1.0E-9 | 1.0E-9 | 2.9E2 | 2.5E2 | 93 | 23 | 93 | 23 | 0.56 |
| Kj | pg/ml | 8.9E2 | 7.3E2 | 1.4E3 | 1.8E3 | 1.5E3 | 3.2E3 | 6.6E1 | 3.3E1 | 8.8E3 | 1.5E4 | 93 | 23 | 93 | 23 | 0.45 |
| Kk | pg/ml | 6.8E0 | 1.4E1 | 1.1E1 | 2.6E1 | 1.4E1 | 2.1E1 | 1.0E-9 | 2.0E0 | 8.1E1 | 5.9E1 | 93 | 23 | 93 | 23 | 0.72 |
| Kl | pg/ml | 1.9E4 | 1.8E4 | 2.9E4 | 2.7E4 | 2.8E4 | 2.0E4 | 2.3E2 | 1.6E3 | 1.3E5 | 6.3E4 | 93 | 23 | 93 | 23 | 0.52 |
| Kn | pg/ml | 2.9E1 | 6.3E1 | 5.3E1 | 3.6E2 | 7.8E1 | 1.0E3 | 1.0E-9 | 1.0E-9 | 3.6E2 | 4.9E3 | 93 | 23 | 93 | 23 | 0.69 |
| Ko | pg/ml | 3.1E2 | 7.4E2 | 4.4E2 | 9.7E2 | 4.9E2 | 1.0E3 | 1.0E-9 | 1.5E2 | 2.2E3 | 4.1E3 | 93 | 23 | 93 | 23 | 0.73 |
| Kp | pg/ml | 3.6E2 | 4.2E2 | 3.5E2 | 1.0E3 | 2.4E2 | 2.7E3 | 1.0E-9 | 3.7E1 | 9.4E2 | 1.3E4 | 93 | 23 | 93 | 23 | 0.62 |
| Kq | pg/ml | 3.2E2 | 5.7E2 | 4.3E2 | 8.2E3 | 5.0E2 | 3.3E4 | 1.6E0 | 1.4E2 | 3.6E3 | 1.6E5 | 88 | 23 | 88 | 23 | 0.72 |
| Kr | pg/ml | 3.7E-1 | 2.2E0 | 1.9E0 | 2.1E1 | 3.6E0 | 8.6E1 | 1.0E-9 | 1.0E-9 | 2.3E1 | 4.2E2 | 88 | 23 | 88 | 23 | 0.62 |
| Ks | pg/ml | 1.5E4 | 1.7E4 | 2.1E4 | 2.2E4 | 1.9E4 | 1.8E4 | 5.1E1 | 9.9E2 | 7.9E4 | 5.1E4 | 88 | 23 | 88 | 23 | 0.53 |
| Ps | ng/ml | 1.3E2 | 1.3E3 | 2.6E2 | 3.5E3 | 3.4E2 | 4.3E3 | 5.5E0 | 3.5E2 | 1.5E3 | 1.2E4 | 41 | 8 | 41 | 8 | 0.94 |
| Kx | ng/ml | 1.0E-9 | 8.8E-3 | 7.0E-3 | 1.8E-2 | 1.6E-2 | 2.1E-2 | 1.0E-9 | 1.0E-9 | 1.0E-1 | 6.5E-2 | 92 | 23 | 92 | 23 | 0.71 |
| Ky | ng/ml | 1.1E-1 | 3.5E-1 | 4.0E-1 | 5.9E-1 | 8.4E-1 | 6.7E-1 | 1.0E-9 | 1.0E-9 | 5.1E0 | 2.7E0 | 92 | 23 | 92 | 23 | 0.67 |
| Kz | ng/ml | 1.0E-9 | 1.0E-9 | 3.4E-3 | 5.6E-3 | 5.6E-3 | 7.8E-3 | 1.0E-9 | 1.0E-9 | 1.4E-2 | 2.5E-2 | 92 | 23 | 92 | 23 | 0.56 |
| Rz | ng/ml | 3.4E-1 | 3.2E-1 | 6.7E-1 | 1.4E0 | 8.5E-1 | 2.5E0 | 4.6E-3 | 1.7E-2 | 3.4E0 | 7.5E0 | 41 | 8 | 41 | 8 | 0.54 |
| Ry | ng/ml | 1.6E-2 | 3.2E-2 | 2.3E-2 | 7.1E-2 | 2.7E-2 | 1.2E-1 | 1.0E-9 | 8.5E-3 | 1.2E-1 | 3.5E-1 | 41 | 8 | 41 | 8 | 0.68 |
| Rx | ng/ml | 1.0E-9 | 3.5E-5 | 1.5E-3 | 1.9E-3 | 2.4E-3 | 2.9E-3 | 1.0E-9 | 1.0E-9 | 8.6E-3 | 7.6E-3 | 41 | 8 | 41 | 8 | 0.58 |
| Ld | pg/ml | 1.0E-9 | 4.8E0 | 3.1E0 | 8.9E0 | 8.2E0 | 1.2E1 | 1.0E-9 | 1.0E-9 | 6.9E1 | 5.0E1 | 94 | 23 | 94 | 23 | 0.72 |
| Lh | pg/ml | 1.3E4 | 2.8E4 | 2.2E4 | 5.7E4 | 2.8E4 | 9.7E4 | 1.0E-9 | 1.3E3 | 2.6E5 | 4.8E5 | 216 | 55 | 216 | 55 | 0.67 |
| Li | pg/ml | 3.3E3 | 1.0E4 | 1.0E4 | 5.9E4 | 2.8E4 | 1.4E5 | 1.3E1 | 1.9E2 | 2.9E5 | 9.2E5 | 216 | 55 | 216 | 55 | 0.74 |
| Lj | pg/ml | 2.3E3 | 1.2E4 | 1.5E4 | 4.2E4 | 4.6E4 | 7.9E4 | 1.0E-9 | 1.8E2 | 4.3E5 | 4.1E5 | 216 | 55 | 216 | 55 | 0.71 |
| Lp | pg/ml | 1.2E1 | 5.3E0 | 4.6E1 | 3.9E2 | 1.1E2 | 5.8E2 | 1.0E-9 | 1.0E-9 | 5.8E2 | 1.4E3 | 41 | 8 | 41 | 8 | 0.51 |
| Lt | pg/ml | 1.0E-9 | 1.0E-9 | 2.1E0 | 1.0E-9 | 6.5E0 | 0.0E0 | 1.0E-9 | 1.0E-9 | 2.5E1 | 1.0E-9 | 41 | 8 | 41 | 8 | 0.45 |
| Rv | ng/ml | 5.0E-4 | 3.8E-4 | 1.2E-3 | 2.9E-3 | 2.5E-3 | 5.0E-3 | 1.0E-9 | 1.0E-9 | 1.6E-2 | 1.2E-2 | 41 | 8 | 41 | 8 | 0.47 |
| Ru | ng/ml | 1.0E-9 | 1.0E-9 | 1.3E-2 | 9.4E-2 | 5.8E-2 | 1.5E-1 | 1.0E-9 | 1.0E-9 | 3.1E-1 | 3.5E-1 | 41 | 8 | 41 | 8 | 0.67 |
| Rt | ng/ml | 8.0E-2 | 5.1E-2 | 1.1E-1 | 1.1E0 | 1.2E-1 | 2.6E0 | 2.2E-3 | 1.3E-3 | 5.8E-1 | 7.4E0 | 41 | 8 | 41 | 8 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Yl | pg/ml | 1.1E1 | 2.6E1 | 1.7E1 | 4.7E1 | 1.7E1 | 7.2E1 | 1.0E-9 | 1.0E-9 | 7.9E1 | 2.2E2 | 42 | 8 | 42 | 8 | 0.63 |
| Rm | ng/ml | 1.7E1 | 5.4E1 | 4.0E1 | 1.0E2 | 6.3E1 | 1.5E2 | 2.2E-1 | 3.9E-1 | 3.4E2 | 6.5E2 | 91 | 22 | 91 | 22 | 0.62 |
| Rh | ng/ml | 1.7E2 | 1.7E2 | 3.1E2 | 9.8E2 | 5.2E2 | 3.6E3 | 7.5E0 | 2.5E1 | 3.8E3 | 1.7E4 | 91 | 22 | 91 | 22 | 0.49 |
| Ri | ng/ml | 4.4E-2 | 1.0E-9 | 3.7E0 | 9.6E-1 | 7.1E0 | 3.4E0 | 1.0E-9 | 1.0E-9 | 4.5E1 | 1.6E1 | 91 | 22 | 91 | 22 | 0.33 |
| Rg | ng/ml | 1.0E-9 | 1.0E-9 | 1.0E-1 | 6.6E-2 | 5.1E-1 | 1.8E-1 | 1.0E-9 | 1.0E-9 | 3.3E0 | 6.2E-1 | 91 | 22 | 91 | 22 | 0.53 |
| Rj | ng/ml | 1.0E-9 | 1.0E-9 | 9.9E-1 | 1.3E1 | 2.2E0 | 5.8E1 | 1.0E-9 | 1.0E-9 | 1.1E1 | 2.7E2 | 91 | 22 | 91 | 22 | 0.46 |
| Rf | ng/ml | 3.4E-1 | 6.7E-1 | 6.9E-1 | 2.3E0 | 1.2E0 | 4.3E0 | 2.1E-2 | 5.5E-2 | 9.9E0 | 1.7E1 | 91 | 22 | 91 | 22 | 0.68 |
| Ql | pg/ml | 1.7E0 | 8.6E0 | 1.2E1 | 1.8E1 | 2.5E1 | 2.5E1 | 1.0E-9 | 1.0E-9 | 1.8E2 | 9.3E1 | 92 | 22 | 92 | 22 | 0.61 |
| Qm | pg/ml | 2.2E0 | 1.7E1 | 1.9E1 | 2.8E1 | 3.6E1 | 3.3E1 | 1.0E-9 | 1.0E-9 | 1.9E2 | 1.0E2 | 92 | 22 | 92 | 22 | 0.61 |
| Qn | pg/ml | 6.1E-1 | 8.5E-1 | 4.9E0 | 8.0E0 | 2.3E1 | 2.0E1 | 1.0E-9 | 1.0E-9 | 2.2E2 | 7.5E1 | 92 | 22 | 92 | 22 | 0.57 |
| Nv | pg/ml | 3.7E3 | 7.8E3 | 9.3E3 | 2.1E4 | 1.9E4 | 3.3E4 | 1.0E-9 | 3.3E2 | 1.5E5 | 1.6E5 | 217 | 55 | 217 | 55 | 0.69 |
| Nw | pg/ml | 9.0E3 | 1.9E4 | 1.3E4 | 2.9E4 | 1.7E4 | 3.9E4 | 1.9E2 | 5.1E3 | 2.1E5 | 2.2E5 | 217 | 55 | 217 | 55 | 0.79 |
| Nx | pg/ml | 2.2E2 | 4.9E2 | 4.2E2 | 7.4E2 | 6.2E2 | 8.1E2 | 1.0E-9 | 1.0E-9 | 3.5E3 | 4.1E3 | 217 | 55 | 217 | 55 | 0.63 |
| Ny | pg/ml | 5.2E0 | 1.9E1 | 1.4E2 | 1.4E2 | 1.7E3 | 4.1E2 | 1.0E-9 | 1.0E-9 | 2.5E4 | 2.8E3 | 217 | 55 | 217 | 55 | 0.72 |
| Oa | pg/ml | 1.2E2 | 6.1E2 | 3.9E2 | 1.1E3 | 6.3E2 | 1.2E3 | 1.0E-9 | 4.9E0 | 3.0E3 | 4.5E3 | 92 | 22 | 92 | 22 | 0.72 |
| Op | pg/ml | 4.4E5 | 4.2E5 | 4.3E5 | 4.1E5 | 1.6E5 | 1.7E5 | 5.2E4 | 9.4E4 | 7.5E5 | 6.6E5 | 41 | 8 | 41 | 8 | 0.48 |
| Oe | pg/ml | 7.5E1 | 1.0E-9 | 2.8E2 | 2.0E2 | 4.1E2 | 3.5E2 | 1.0E-9 | 1.0E-9 | 2.3E3 | 1.3E3 | 216 | 54 | 216 | 54 | 0.44 |
| Of | pg/ml | 2.0E2 | 1.0E2 | 6.4E3 | 5.6E3 | 2.4E4 | 1.9E4 | 1.0E-9 | 1.0E-9 | 1.9E5 | 1.2E5 | 217 | 55 | 217 | 55 | 0.46 |
| Og | pg/ml | 8.3E-2 | 2.0E-2 | 4.5E-1 | 8.7E-2 | 1.8E0 | 1.5E-1 | 1.0E-9 | 1.0E-9 | 1.9E1 | 8.0E-1 | 217 | 55 | 217 | 55 | 0.36 |
| Oh | pg/ml | 2.6E0 | 6.4E0 | 1.7E1 | 3.3E2 | 1.1E2 | 2.1E3 | 1.0E-9 | 1.0E-9 | 1.4E3 | 1.6E4 | 217 | 55 | 217 | 55 | 0.64 |
| Oi | pg/ml | 2.5E0 | 2.7E0 | 5.4E0 | 5.0E0 | 7.5E0 | 6.3E0 | 1.0E-9 | 1.0E-9 | 4.2E1 | 3.1E1 | 217 | 55 | 217 | 55 | 0.51 |
| Ok | pg/ml | 3.9E2 | 6.4E2 | 4.9E2 | 1.1E3 | 4.0E2 | 1.3E3 | 1.5E1 | 1.1E2 | 2.2E3 | 7.8E3 | 217 | 55 | 217 | 55 | 0.72 |
| Om | pg/ml | 4.1E2 | 6.0E2 | 9.1E2 | 2.0E3 | 2.5E3 | 6.9E3 | 1.0E-9 | 1.1E2 | 3.0E4 | 5.1E4 | 217 | 55 | 217 | 55 | 0.62 |
| On | pg/ml | 1.8E2 | 3.2E2 | 2.9E2 | 7.5E2 | 4.5E2 | 1.3E3 | 8.4E-1 | 2.6E1 | 4.5E3 | 8.5E3 | 217 | 55 | 217 | 55 | 0.72 |
| Or | pg/ml | 1.0E1 | 2.4E1 | 3.6E1 | 9.1E1 | 6.8E1 | 1.5E2 | 1.0E-9 | 1.0E-9 | 4.4E2 | 5.1E2 | 94 | 23 | 94 | 23 | 0.60 |
| Ow | pg/ml | 3.4E1 | 9.7E1 | 1.2E2 | 7.9E2 | 3.4E2 | 1.8E3 | 1.0E-9 | 1.0E-9 | 2.7E3 | 8.1E3 | 94 | 23 | 94 | 23 | 0.71 |
| Ou | pg/ml | 4.2E2 | 9.2E2 | 1.0E3 | 2.2E3 | 1.8E3 | 2.9E3 | 1.0E-9 | 1.0E-9 | 1.1E4 | 8.9E3 | 94 | 23 | 94 | 23 | 0.63 |
| Ul | ng/ml | 1.0E-9 | 1.0E-9 | 1.0E0 | 3.5E0 | 4.0E0 | 1.3E1 | 1.0E-9 | 1.0E-9 | 2.3E1 | 5.6E1 | 93 | 22 | 93 | 22 | 0.51 |
| Uo | ng/ml | 1.0E-9 | 1.0E-9 | 8.1E-2 | 4.5E-2 | 2.1E-1 | 1.4E-1 | 1.0E-9 | 1.0E-9 | 1.3E0 | 6.3E-1 | 93 | 22 | 93 | 22 | 0.47 |
| Uk | ng/ml | 1.0E-9 | 1.0E-9 | 7.8E-3 | 2.0E-3 | 3.7E-2 | 6.9E-3 | 1.0E-9 | 1.0E-9 | 3.2E-1 | 3.1E-2 | 93 | 22 | 93 | 22 | 0.42 |
| Um | ng/ml | 1.0E-9 | 1.0E-9 | 2.3E-1 | 2.5E-1 | 6.0E-1 | 6.7E-1 | 1.0E-9 | 1.0E-9 | 3.6E0 | 2.3E0 | 93 | 22 | 93 | 22 | 0.47 |
| Uf | ng/ml | 5.3E-2 | 9.9E-2 | 1.3E-1 | 4.2E-1 | 1.9E-1 | 1.1E0 | 1.0E-3 | 2.1E-2 | 1.1E0 | 5.1E0 | 93 | 22 | 93 | 22 | 0.68 |
| Uh | ng/ml | 2.2E0 | 6.8E0 | 3.0E0 | 7.2E0 | 2.7E0 | 4.6E0 | 3.6E-2 | 7.1E-1 | 1.3E1 | 1.8E1 | 93 | 22 | 93 | 22 | 0.79 |
| Un | ng/ml | 1.6E0 | 2.7E0 | 1.9E0 | 4.0E0 | 1.2E0 | 5.1E0 | 3.4E-1 | 7.1E-1 | 8.0E0 | 2.5E1 | 93 | 22 | 93 | 22 | 0.71 |
| Ug | ng/ml | 1.1E1 | 6.8E0 | 2.3E1 | 2.0E1 | 2.6E1 | 3.7E1 | 1.2E0 | 1.7E0 | 1.4E2 | 1.6E2 | 93 | 22 | 93 | 22 | 0.38 |
| Ur | ng/ml | 1.1E-1 | 1.0E-9 | 3.2E-1 | 7.6E-1 | 5.5E-1 | 1.9E0 | 1.0E-9 | 1.0E-9 | 3.0E0 | 7.3E0 | 92 | 22 | 92 | 22 | 0.39 |
| Up | ng/ml | 1.0E-9 | 1.0E-9 | 2.9E-3 | 1.1E-1 | 9.1E-3 | 5.1E-1 | 1.0E-9 | 1.0E-9 | 5.3E-2 | 2.4E0 | 92 | 22 | 92 | 22 | 0.54 |
| Us | ng/ml | 4.1E-3 | 1.0E-9 | 1.7E-2 | 9.6E-2 | 4.6E-2 | 3.5E-1 | 1.0E-9 | 1.0E-9 | 3.6E-1 | 1.7E0 | 92 | 22 | 92 | 22 | 0.44 |
| Uv | ng/ml | 3.2E-3 | 1.7E-3 | 1.3E-2 | 2.3E-2 | 3.4E-2 | 8.7E-2 | 1.0E-9 | 1.0E-9 | 2.3E-1 | 4.1E-1 | 92 | 22 | 92 | 22 | 0.43 |
| Ut | ng/ml | 7.4E-1 | 1.4E0 | 2.7E0 | 6.9E0 | 6.6E0 | 1.5E1 | 1.0E-9 | 1.0E-9 | 5.2E1 | 6.5E1 | 92 | 22 | 92 | 22 | 0.63 |
| Uu | ng/ml | 7.2E0 | 5.4E0 | 7.7E0 | 6.6E0 | 5.0E0 | 4.2E0 | 5.7E-1 | 1.7E0 | 2.9E1 | 1.7E1 | 92 | 22 | 92 | 22 | 0.43 |
| Uw | ng/ml | 2.3E0 | 4.5E0 | 2.7E0 | 8.5E0 | 2.2E0 | 1.3E1 | 1.0E-9 | 1.7E0 | 7.9E0 | 3.9E1 | 42 | 8 | 42 | 8 | 0.75 |
| Vb | ng/ml | 1.1E0 | 9.3E-1 | 1.1E0 | 1.5E0 | 4.2E-1 | 2.0E0 | 8.5E-2 | 2.6E-1 | 2.0E0 | 6.4E0 | 42 | 8 | 42 | 8 | 0.42 |
| Vc | ng/ml | 1.0E-9 | 1.0E-9 | 3.3E-3 | 1.0E-9 | 1.7E-2 | 0.0E0 | 1.0E-9 | 1.0E-9 | 1.1E-1 | 1.0E-9 | 42 | 8 | 42 | 8 | 0.46 |
| Uy | ng/ml | 1.3E0 | 1.3E0 | 5.9E0 | 1.9E1 | 1.7E1 | 2.6E1 | 8.7E-2 | 2.0E-2 | 9.9E1 | 6.4E1 | 42 | 8 | 42 | 8 | 0.57 |
| Uz | ng/ml | 1.0E-9 | 1.0E-9 | 1.0E-2 | 4.2E0 | 6.7E-2 | 1.2E1 | 1.0E-9 | 1.0E-9 | 4.4E-1 | 3.3E1 | 42 | 8 | 42 | 8 | 0.61 |
| Ux | ng/ml | 2.1E2 | 1.3E2 | 2.0E2 | 1.7E2 | 1.3E2 | 1.4E2 | 4.5E0 | 4.0E1 | 4.6E2 | 4.9E2 | 42 | 8 | 42 | 8 | 0.41 |
| Va | ng/ml | 1.5E1 | 3.2E0 | 2.6E1 | 1.1E1 | 3.0E1 | 2.1E1 | 3.1E-1 | 1.2E0 | 1.2E2 | 6.2E1 | 42 | 8 | 42 | 8 | 0.31 |
| Vh | ng/ml | 1.1E-2 | 2.3E-2 | 1.9E-2 | 1.3E-1 | 2.6E-2 | 3.0E-1 | 1.0E-3 | 3.5E-3 | 1.2E-1 | 8.6E-1 | 42 | 8 | 42 | 8 | 0.71 |
| Vi | ng/ml | 3.1E-3 | 3.4E-2 | 8.9E-3 | 2.6E-1 | 1.9E-2 | 6.3E-1 | 1.0E-9 | 1.6E-2 | 1.2E-1 | 1.8E0 | 42 | 8 | 42 | 8 | 0.93 |
| Vj | ng/ml | 3.1E1 | 1.0E2 | 5.8E1 | 1.7E2 | 7.1E1 | 2.2E2 | 1.4E0 | 1.4E1 | 3.4E2 | 6.5E2 | 42 | 7 | 42 | 7 | 0.79 |
| Vp | ng/ml | 1.0E-9 | 1.0E-9 | 1.0E-1 | 2.6E0 | 4.8E-1 | 1.0E1 | 1.0E-9 | 1.0E-9 | 3.9E0 | 4.9E1 | 93 | 22 | 93 | 22 | 0.60 |
| Vt | ng/ml | 6.0E0 | 1.1E1 | 7.4E0 | 2.1E1 | 6.3E0 | 3.2E1 | 5.6E-1 | 1.9E0 | 3.2E1 | 1.6E2 | 93 | 22 | 93 | 22 | 0.77 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Vu | ng/ml | 1.0E-9 | 3.6E-1 | 1.6E0 | 2.7E0 | 3.0E0 | 5.6E0 | 1.0E-9 | 1.0E-9 | 1.6E1 | 2.2E1 | 91 | 20 | 91 | 20 | 0.56 |
| Vq | ng/ml | 1.3E2 | 1.1E3 | 4.8E2 | 2.0E3 | 8.4E2 | 3.2E3 | 9.2E-1 | 1.0E1 | 5.0E3 | 1.2E4 | 75 | 16 | 75 | 16 | 0.71 |
| Vo | ng/ml | 2.5E1 | 2.5E1 | 2.5E1 | 2.4E1 | 5.8E0 | 4.5E0 | 4.9E0 | 1.1E1 | 4.8E1 | 3.0E1 | 93 | 22 | 93 | 22 | 0.48 |
| Vs | ng/ml | 1.0E-9 | 1.0E-9 | 4.3E0 | 2.7E1 | 1.2E1 | 1.0E2 | 1.0E-9 | 1.0E-9 | 8.9E1 | 4.5E2 | 92 | 20 | 92 | 20 | 0.47 |
| Vv | ng/ml | 3.3E0 | 2.6E0 | 6.0E0 | 6.5E0 | 1.0E1 | 1.1E1 | 1.0E-9 | 1.0E-9 | 8.4E1 | 4.4E1 | 93 | 21 | 93 | 21 | 0.49 |
| Vw | ng/ml | 3.7E1 | 4.9E1 | 3.3E1 | 4.4E1 | 1.7E1 | 2.3E1 | 2.5E0 | 1.1E1 | 6.7E1 | 6.9E1 | 42 | 8 | 42 | 8 | 0.66 |
| Oy | pg/ml | 5.2E-1 | 4.3E-1 | 8.3E0 | 3.0E0 | 3.8E1 | 6.7E0 | 1.0E-9 | 1.0E-9 | 4.0E2 | 3.3E1 | 216 | 55 | 216 | 55 | 0.46 |
| Oz | pg/ml | 4.1E-2 | 1.0E-9 | 4.0E-1 | 6.6E-1 | 2.0E0 | 3.8E0 | 1.0E-9 | 1.0E-9 | 2.9E1 | 2.8E1 | 216 | 55 | 216 | 55 | 0.39 |
| Pa | pg/ml | 3.9E-1 | 7.2E-1 | 1.5E0 | 8.0E0 | 6.8E0 | 3.3E1 | 1.0E-9 | 1.0E-9 | 8.6E1 | 2.3E2 | 216 | 55 | 216 | 55 | 0.63 |
| Pb | pg/ml | 1.0E-9 | 1.0E-9 | 2.4E0 | 1.2E0 | 3.3E1 | 5.6E0 | 1.0E-9 | 1.0E-9 | 4.9E2 | 3.2E1 | 216 | 55 | 216 | 55 | 0.42 |
| Pc | pg/ml | 1.4E-1 | 1.0E-9 | 4.5E-1 | 7.0E0 | 1.1E0 | 4.5E1 | 1.0E-9 | 1.0E-9 | 1.4E1 | 3.3E2 | 216 | 55 | 216 | 55 | 0.45 |
| Pd | pg/ml | 1.7E0 | 2.9E0 | 7.8E0 | 8.9E0 | 5.8E1 | 1.9E1 | 1.0E-9 | 1.0E-9 | 8.4E2 | 1.2E2 | 216 | 55 | 216 | 55 | 0.59 |
| Pe | pg/ml | 2.0E1 | 6.3E1 | 1.0E2 | 7.2E2 | 4.4E2 | 2.3E3 | 1.0E-9 | 1.0E-9 | 4.7E3 | 1.5E4 | 216 | 55 | 216 | 55 | 0.72 |
| Pf | pg/ml | 1.5E0 | 6.0E0 | 1.4E1 | 3.1E1 | 1.0E2 | 7.8E1 | 1.0E-9 | 1.0E-9 | 1.5E3 | 4.3E2 | 216 | 55 | 216 | 55 | 0.71 |
| Pg | pg/ml | 3.7E0 | 1.3E1 | 9.1E1 | 1.6E2 | 6.3E2 | 4.0E2 | 1.0E-9 | 1.0E-9 | 7.7E3 | 2.2E3 | 216 | 55 | 216 | 55 | 0.70 |
| Ph | ng/ml | 1.4E-1 | 2.4E-1 | 3.4E-1 | 6.1E-1 | 5.1E-1 | 1.2E0 | 1.0E-9 | 1.0E-9 | 2.8E0 | 5.4E0 | 94 | 23 | 94 | 23 | 0.56 |
| Pi | ng/ml | 1.9E-1 | 2.7E-1 | 2.8E-1 | 4.1E0 | 4.0E-1 | 1.7E1 | 1.0E-9 | 1.0E-9 | 3.2E0 | 8.2E1 | 94 | 23 | 94 | 23 | 0.63 |
| Pj | ng/mL | 4.9E0 | 7.4E0 | 5.8E0 | 8.5E0 | 4.6E0 | 4.9E0 | 4.0E-1 | 1.5E0 | 3.1E1 | 2.3E1 | 94 | 23 | 94 | 23 | 0.68 |
| Pk | ng/ml | 8.9E-3 | 1.1E-2 | 1.5E-2 | 7.8E-2 | 3.0E-2 | 3.1E-1 | 1.0E-9 | 1.0E-9 | 2.5E-1 | 1.5E0 | 94 | 23 | 94 | 23 | 0.55 |
| aA | mg/dL | 8.7E-1 | 1.0E0 | 9.8E-1 | 1.4E0 | 4.7E-1 | 9.1E-1 | 3.0E-1 | 5.0E-1 | 4.2E0 | 4.7E0 | 337 | 70 | 337 | 70 | 0.63 |
| aC | mg/mL | 2.2E0 | 2.1E0 | 2.5E0 | 2.4E0 | 1.2E0 | 1.2E0 | 7.5E-1 | 1.0E0 | 7.2E0 | 5.5E0 | 112 | 31 | 112 | 31 | 0.47 |
| aD | ug/mL | 3.0E0 | 3.6E0 | 4.5E0 | 4.9E0 | 4.2E0 | 4.0E0 | 7.5E-1 | 9.2E-1 | 3.1E1 | 1.8E1 | 112 | 31 | 112 | 31 | 0.51 |
| aE | mg/mL | 6.0E-1 | 5.1E-1 | 6.0E-1 | 5.6E-1 | 1.7E-1 | 1.8E-1 | 1.8E-1 | 2.2E-1 | 1.1E0 | 1.2E0 | 112 | 31 | 112 | 31 | 0.39 |
| aF | ng/mL | 2.1E0 | 2.9E0 | 5.2E0 | 5.0E0 | 8.4E0 | 6.5E0 | 4.3E-3 | 4.3E-3 | 5.0E1 | 2.9E1 | 112 | 31 | 112 | 31 | 0.49 |
| aG | mg/mL | 1.4E-1 | 1.3E-1 | 1.6E-1 | 1.4E-1 | 8.4E-2 | 6.5E-2 | 5.0E-2 | 6.8E-2 | 4.8E-1 | 3.2E-1 | 112 | 31 | 112 | 31 | 0.46 |
| aH | ug/mL | 7.4E1 | 5.9E1 | 7.8E1 | 6.5E1 | 3.9E1 | 3.3E1 | 1.5E1 | 1.1E1 | 2.0E2 | 1.4E2 | 112 | 31 | 112 | 31 | 0.40 |
| aI | ug/mL | 1.8E2 | 1.4E2 | 1.8E2 | 1.6E2 | 6.0E1 | 5.8E1 | 4.7E1 | 7.6E1 | 3.4E2 | 2.7E2 | 112 | 31 | 112 | 31 | 0.40 |
| aJ | ug/mL | 2.3E0 | 3.6E0 | 3.0E0 | 4.1E0 | 2.1E0 | 2.8E0 | 8.2E-1 | 1.4E0 | 1.4E1 | 1.1E1 | 112 | 31 | 112 | 31 | 0.64 |
| aK | ng/mL | 1.3E0 | 1.3E0 | 2.0E0 | 1.9E0 | 1.9E0 | 1.8E0 | 2.9E-4 | 1.0E-1 | 1.0E1 | 6.5E0 | 112 | 31 | 112 | 31 | 0.48 |
| aL | mg/mL | 7.3E-1 | 7.4E-1 | 7.6E-1 | 7.3E-1 | 2.3E-1 | 2.7E-1 | 2.2E-1 | 2.7E-1 | 1.7E0 | 1.4E0 | 112 | 31 | 112 | 31 | 0.48 |
| aM | U/mL | 1.5E1 | 2.3E1 | 3.3E1 | 7.2E1 | 4.9E1 | 1.6E2 | 4.2E-2 | 4.2E-2 | 3.5E2 | 8.2E2 | 112 | 31 | 112 | 31 | 0.62 |
| aN | U/mL | 1.5E1 | 1.9E1 | 2.4E1 | 4.0E1 | 4.0E1 | 6.2E1 | 2.5E-3 | 2.5E-3 | 3.8E2 | 3.3E2 | 112 | 31 | 112 | 31 | 0.58 |
| aO | pg/mL | 6.1E1 | 7.2E1 | 4.5E2 | 4.4E2 | 1.1E3 | 7.0E2 | 6.0E-2 | 5.0E0 | 6.6E3 | 2.4E3 | 112 | 31 | 112 | 31 | 0.56 |
| aP | ng/mL | 1.5E0 | 1.9E0 | 1.9E0 | 2.6E0 | 1.2E0 | 1.7E0 | 5.4E-1 | 7.8E-1 | 6.5E0 | 6.6E0 | 112 | 31 | 112 | 31 | 0.63 |
| aQ | ng/mL | 2.4E-1 | 2.2E-1 | 3.6E-1 | 2.8E-1 | 3.4E-1 | 2.0E-1 | 2.0E-4 | 5.1E-2 | 2.0E0 | 9.0E-1 | 112 | 31 | 112 | 31 | 0.45 |
| aR | ng/mL | 1.8E0 | 2.4E0 | 3.0E0 | 3.5E0 | 4.4E0 | 3.3E0 | 2.6E-1 | 6.6E-1 | 3.4E1 | 1.7E1 | 112 | 31 | 112 | 31 | 0.60 |
| aS | ng/mL | 3.9E-1 | 4.0E-1 | 1.2E0 | 8.7E-1 | 3.2E0 | 1.1E0 | 4.2E-3 | 6.0E-2 | 3.3E1 | 4.9E0 | 112 | 31 | 112 | 31 | 0.48 |
| aU | pg/mL | 6.7E1 | 6.2E1 | 1.0E2 | 1.0E2 | 1.1E2 | 1.1E2 | 7.4E-2 | 7.4E-2 | 7.0E2 | 5.1E2 | 112 | 31 | 112 | 31 | 0.50 |
| aV | ng/mL | 6.2E-1 | 3.7E-1 | 1.1E0 | 8.3E-1 | 3.1E0 | 1.1E0 | 7.6E-4 | 9.1E-2 | 3.3E1 | 6.0E0 | 112 | 31 | 112 | 31 | 0.45 |
| aW | pg/mL | 2.1E1 | 1.9E1 | 2.1E1 | 3.1E1 | 1.7E1 | 7.2E1 | 7.2E-2 | 7.2E-2 | 1.7E2 | 4.2E2 | 112 | 31 | 112 | 31 | 0.46 |
| aX | ng/mL | 7.7E0 | 7.0E0 | 1.1E1 | 1.3E1 | 1.0E1 | 2.1E1 | 3.0E-1 | 7.7E-1 | 5.4E1 | 1.1E2 | 112 | 31 | 112 | 31 | 0.46 |
| aY | pg/mL | 5.3E1 | 5.2E1 | 7.9E1 | 6.6E1 | 1.3E2 | 5.4E1 | 4.1E-1 | 4.1E-1 | 1.2E3 | 2.2E2 | 112 | 31 | 112 | 31 | 0.49 |
| aZ | pg/mL | 2.2E2 | 3.0E2 | 5.6E2 | 8.9E2 | 1.3E3 | 1.3E3 | 1.7E0 | 1.7E0 | 1.2E4 | 4.7E3 | 112 | 31 | 112 | 31 | 0.59 |
| bA | ng/mL | 1.3E1 | 4.0E1 | 5.0E1 | 1.4E2 | 1.1E2 | 2.3E2 | 3.0E-2 | 2.0E0 | 9.4E2 | 9.4E2 | 112 | 31 | 112 | 31 | 0.66 |
| bB | ng/mL | 2.8E2 | 2.7E2 | 3.0E2 | 2.5E2 | 1.6E2 | 1.5E2 | 3.6E1 | 1.2E1 | 8.1E2 | 5.7E2 | 112 | 31 | 112 | 31 | 0.42 |
| bC | ng/mL | 3.2E2 | 3.3E2 | 5.7E2 | 7.1E2 | 7.5E2 | 9.9E2 | 2.7E1 | 5.0E1 | 4.7E3 | 4.0E3 | 112 | 31 | 112 | 31 | 0.54 |
| bE | mg/mL | 5.2E0 | 4.8E0 | 5.2E0 | 5.2E0 | 1.8E0 | 2.4E0 | 1.8E0 | 1.3E0 | 1.2E1 | 1.1E1 | 112 | 31 | 112 | 31 | 0.47 |
| bF | pg/mL | 3.4E1 | 5.9E1 | 4.3E2 | 3.0E2 | 1.7E3 | 5.7E2 | 5.0E-2 | 8.9E0 | 1.1E4 | 2.2E3 | 112 | 31 | 112 | 31 | 0.64 |
| bG | ng/mL | 1.7E0 | 2.3E0 | 3.3E0 | 3.6E0 | 5.0E0 | 3.8E0 | 1.1E-1 | 2.4E-1 | 3.0E1 | 1.5E1 | 112 | 31 | 112 | 31 | 0.56 |
| bH | pg/mL | 5.7E-1 | 5.7E-1 | 7.6E0 | 4.6E0 | 2.9E1 | 5.9E0 | 5.7E-1 | 5.7E-1 | 2.8E2 | 2.3E1 | 112 | 31 | 112 | 31 | 0.53 |
| bI | ng/mL | 4.0E-3 | 4.0E-3 | 1.0E-1 | 8.0E-2 | 2.0E-1 | 2.1E-1 | 4.0E-3 | 4.0E-3 | 9.8E-1 | 8.8E-1 | 112 | 31 | 112 | 31 | 0.45 |
| bJ | mg/mL | 1.7E0 | 1.9E0 | 2.1E0 | 2.3E0 | 1.8E0 | 2.3E0 | 2.5E-4 | 2.5E-4 | 1.1E1 | 9.0E0 | 112 | 31 | 112 | 31 | 0.51 |
| bL | pg/mL | 4.2E0 | 3.2E0 | 9.1E0 | 8.1E0 | 1.1E1 | 8.7E0 | 4.6E-2 | 4.6E-2 | 4.9E1 | 3.2E1 | 112 | 31 | 112 | 31 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bM | mg/mL | 1.9E0 | 2.2E0 | 2.3E0 | 2.2E0 | 1.5E0 | 1.3E0 | 2.4E-1 | 1.8E-2 | 8.6E0 | 5.4E0 | 112 | 31 | 112 | 31 | 0.51 |
| bN | ng/mL | 4.0E1 | 3.1E1 | 1.4E2 | 5.3E1 | 3.1E2 | 8.4E1 | 1.4E-1 | 5.9E-1 | 1.9E3 | 4.4E2 | 112 | 31 | 112 | 31 | 0.40 |
| bO | ng/mL | 4.0E-2 | 4.0E-2 | 7.5E0 | 1.3E1 | 1.5E1 | 2.8E1 | 4.0E-2 | 4.0E-2 | 7.1E1 | 1.3E2 | 112 | 31 | 112 | 31 | 0.48 |
| bP | mg/mL | 4.9E-1 | 5.7E-1 | 6.9E-1 | 7.5E-1 | 6.4E-1 | 7.5E-1 | 8.2E-2 | 9.2E-2 | 4.8E0 | 3.5E0 | 112 | 31 | 112 | 31 | 0.52 |
| bQ | pg/mL | 2.1E1 | 5.2E1 | 1.8E2 | 6.1E1 | 1.3E3 | 5.6E1 | 1.5E-1 | 1.5E-1 | 1.3E4 | 2.2E2 | 112 | 31 | 112 | 31 | 0.67 |
| bR | ng/mL | 1.2E-2 | 1.2E-2 | 1.9E-1 | 7.3E-2 | 8.8E-1 | 1.1E-1 | 1.2E-2 | 1.2E-2 | 8.7E0 | 3.9E-1 | 112 | 31 | 112 | 31 | 0.47 |
| bS | pg/mL | 9.4E-1 | 9.4E-1 | 1.1E1 | 5.0E0 | 5.0E1 | 1.4E1 | 9.4E-1 | 9.4E-1 | 3.9E2 | 7.2E1 | 112 | 31 | 112 | 31 | 0.49 |
| bU | ng/mL | 3.4E-2 | 1.5E-1 | 2.0E-1 | 1.7E-1 | 6.5E-1 | 1.8E-1 | 1.3E-2 | 1.3E-2 | 6.6E0 | 6.5E-1 | 112 | 31 | 112 | 31 | 0.57 |
| bV | pg/mL | 4.6E2 | 5.5E2 | 5.3E2 | 7.1E2 | 2.6E2 | 4.8E2 | 1.8E2 | 2.7E2 | 1.6E3 | 2.2E3 | 112 | 31 | 112 | 31 | 0.60 |
| bW | pg/mL | 3.3E2 | 3.1E2 | 4.7E2 | 5.5E2 | 5.3E2 | 8.1E2 | 8.4E1 | 1.3E2 | 4.8E3 | 3.9E3 | 112 | 31 | 112 | 31 | 0.50 |
| bX | ng/mL | 1.5E-3 | 2.5E-5 | 2.6E-3 | 2.3E-3 | 2.9E-3 | 2.8E-3 | 2.5E-5 | 2.5E-5 | 1.2E-2 | 7.8E-3 | 112 | 31 | 112 | 31 | 0.47 |
| bZ | pg/mL | 2.7E2 | 3.6E2 | 2.3E3 | 1.3E3 | 8.4E3 | 1.8E3 | 1.5E-1 | 1.0E2 | 5.8E4 | 7.4E3 | 112 | 31 | 112 | 31 | 0.60 |
| cA | pg/mL | 6.0E-1 | 6.0E-1 | 5.0E0 | 2.0E0 | 3.5E1 | 4.7E0 | 6.0E-1 | 6.0E-1 | 3.7E2 | 2.1E1 | 112 | 31 | 112 | 31 | 0.50 |
| cB | ng/mL | 4.1E-2 | 4.6E-2 | 6.3E-2 | 8.0E-2 | 7.0E-2 | 1.1E-1 | 1.7E-3 | 1.7E-3 | 3.7E-1 | 4.3E-1 | 112 | 31 | 112 | 31 | 0.49 |
| cC | pg/mL | 4.1E1 | 4.8E1 | 4.7E1 | 4.5E1 | 5.8E1 | 2.8E1 | 1.0E0 | 1.0E0 | 4.5E2 | 1.1E2 | 112 | 31 | 112 | 31 | 0.55 |
| cD | pg/mL | 4.9E0 | 5.2E0 | 1.6E1 | 9.1E0 | 5.6E1 | 1.6E1 | 3.3E-1 | 3.3E-1 | 4.8E2 | 9.0E1 | 112 | 31 | 112 | 31 | 0.53 |
| cE | pg/mL | 6.3E1 | 1.3E2 | 2.7E2 | 2.9E2 | 6.8E2 | 4.2E2 | 1.2E-1 | 1.4E1 | 3.8E3 | 2.1E3 | 112 | 31 | 112 | 31 | 0.64 |
| cF | pg/mL | 5.3E-1 | 8.6E0 | 1.6E1 | 1.3E1 | 3.4E1 | 1.7E1 | 5.3E-1 | 5.3E-1 | 2.7E2 | 6.5E1 | 112 | 31 | 112 | 31 | 0.52 |
| cG | pg/mL | 5.2E1 | 9.4E1 | 1.9E2 | 1.4E2 | 9.9E2 | 1.1E2 | 7.8E0 | 2.4E1 | 1.0E4 | 4.1E2 | 112 | 31 | 112 | 31 | 0.66 |
| cH | uIU/mL | 3.7E0 | 2.2E0 | 8.9E0 | 5.0E0 | 2.0E1 | 9.0E0 | 8.6E-3 | 8.6E-3 | 1.6E2 | 3.9E1 | 112 | 31 | 112 | 31 | 0.37 |
| cI | ng/mL | 5.9E0 | 6.9E0 | 1.1E1 | 2.1E1 | 1.6E1 | 3.3E1 | 2.3E-1 | 3.2E-2 | 9.4E1 | 1.2E2 | 112 | 31 | 112 | 31 | 0.54 |
| cJ | ug/mL | 6.5E1 | 4.8E1 | 1.0E2 | 7.0E1 | 1.1E2 | 6.9E1 | 6.9E0 | 7.2E0 | 6.4E2 | 3.4E2 | 112 | 31 | 112 | 31 | 0.43 |
| cK | ng/mL | 3.8E-3 | 3.8E-3 | 2.3E-2 | 1.8E-2 | 1.4E-1 | 4.0E-2 | 3.8E-3 | 3.8E-3 | 1.5E0 | 2.1E-1 | 112 | 31 | 112 | 31 | 0.54 |
| cL | pg/mL | 2.1E2 | 2.2E2 | 6.4E2 | 3.6E2 | 2.5E3 | 3.6E2 | 3.1E1 | 1.1E2 | 2.4E4 | 1.5E3 | 112 | 31 | 112 | 31 | 0.60 |
| cM | pg/mL | 2.6E2 | 2.8E2 | 2.9E2 | 2.8E2 | 1.8E2 | 1.1E2 | 4.2E1 | 5.7E1 | 1.1E3 | 4.8E2 | 112 | 31 | 112 | 31 | 0.52 |
| cN | pg/mL | 1.1E2 | 1.3E2 | 1.3E2 | 1.4E2 | 1.0E2 | 4.2E1 | 3.8E1 | 7.9E1 | 1.1E3 | 2.7E2 | 112 | 31 | 112 | 31 | 0.59 |
| cO | pg/mL | 2.0E2 | 2.4E2 | 4.7E2 | 3.2E2 | 1.8E3 | 2.6E2 | 5.4E1 | 1.3E2 | 1.9E4 | 1.5E3 | 112 | 31 | 112 | 31 | 0.61 |
| cP | ng/mL | 2.5E3 | 2.2E3 | 2.6E3 | 2.4E3 | 1.0E3 | 9.3E2 | 6.2E2 | 1.0E3 | 5.6E3 | 4.7E3 | 112 | 31 | 112 | 31 | 0.44 |
| cQ | ng/mL | 5.2E-2 | 3.5E-2 | 1.3E-1 | 1.2E-1 | 2.1E-1 | 2.2E-1 | 2.0E-3 | 2.0E-3 | 1.3E0 | 8.7E-1 | 112 | 31 | 112 | 31 | 0.44 |
| cR | ng/mL | 3.0E2 | 3.8E2 | 5.4E2 | 6.8E2 | 8.3E2 | 9.4E2 | 2.0E1 | 8.6E1 | 7.7E3 | 4.8E3 | 112 | 31 | 112 | 31 | 0.56 |
| cS | ng/mL | 2.6E2 | 3.0E2 | 3.8E2 | 4.6E2 | 4.0E2 | 4.8E2 | 8.0E1 | 9.1E1 | 2.5E3 | 2.4E3 | 112 | 31 | 112 | 31 | 0.54 |
| cT | ng/mL | 5.0E1 | 1.0E2 | 1.4E2 | 2.9E2 | 2.6E2 | 4.5E2 | 4.2E0 | 1.1E1 | 2.1E3 | 1.5E3 | 112 | 31 | 112 | 31 | 0.62 |
| cU | ng/mL | 5.8E1 | 8.0E1 | 9.8E1 | 1.1E2 | 1.7E2 | 9.4E1 | 6.2E0 | 9.0E0 | 1.6E3 | 4.2E2 | 112 | 31 | 112 | 31 | 0.60 |
| cV | ng/mL | 2.0E-1 | 2.2E-1 | 8.9E-1 | 6.4E-1 | 4.5E0 | 1.8E0 | 3.4E-2 | 3.0E-2 | 4.7E1 | 9.7E0 | 112 | 31 | 112 | 31 | 0.50 |
| cW | mIU/mL | 4.8E-2 | 4.5E-2 | 9.6E-2 | 7.2E-2 | 4.2E-1 | 7.2E-2 | 4.8E-3 | 1.4E-2 | 4.5E0 | 3.9E-1 | 112 | 31 | 112 | 31 | 0.54 |
| cX | ng/mL | 1.8E-1 | 6.3E-2 | 2.0E0 | 2.7E0 | 5.7E0 | 7.6E0 | 2.3E-4 | 2.3E-4 | 2.8E1 | 2.8E1 | 112 | 31 | 112 | 31 | 0.46 |
| cY | ng/mL | 7.1E0 | 8.9E0 | 1.1E1 | 1.1E1 | 1.2E1 | 9.5E0 | 1.7E-1 | 6.0E-1 | 6.1E1 | 3.7E1 | 112 | 31 | 112 | 31 | 0.52 |
| cZ | ug/mL | 1.2E1 | 1.2E1 | 1.4E1 | 1.3E1 | 5.7E0 | 7.0E0 | 2.8E0 | 3.3E0 | 3.0E1 | 3.0E1 | 112 | 31 | 112 | 31 | 0.43 |
| dA | pg/mL | 3.1E2 | 3.5E2 | 3.8E2 | 3.8E2 | 5.4E2 | 2.1E2 | 1.0E2 | 1.1E2 | 5.8E3 | 9.3E2 | 112 | 31 | 112 | 31 | 0.52 |
| dB | ug/mL | 1.9E1 | 2.1E1 | 1.9E1 | 1.9E1 | 2.4E1 | 9.5E0 | 2.1E0 | 2.2E0 | 2.5E2 | 3.2E1 | 112 | 31 | 112 | 31 | 0.55 |
| dC | nmol/L | 3.4E1 | 3.6E1 | 3.8E1 | 3.9E1 | 1.8E1 | 1.4E1 | 7.8E0 | 1.5E1 | 1.4E2 | 6.5E1 | 112 | 31 | 112 | 31 | 0.55 |
| dD | ug/mL | 3.4E1 | 3.0E1 | 3.5E1 | 3.2E1 | 1.0E1 | 1.2E1 | 1.4E1 | 1.4E1 | 7.4E1 | 6.0E1 | 112 | 31 | 112 | 31 | 0.39 |
| dE | ng/mL | 4.0E-1 | 4.8E-1 | 5.3E-1 | 5.8E-1 | 5.6E-1 | 5.6E-1 | 8.4E-3 | 8.4E-3 | 2.7E0 | 2.4E0 | 112 | 31 | 112 | 31 | 0.54 |
| dF | ng/mL | 2.4E2 | 3.7E2 | 3.2E2 | 4.8E2 | 2.2E2 | 3.3E2 | 7.5E1 | 1.1E2 | 1.3E3 | 1.2E3 | 112 | 31 | 112 | 31 | 0.65 |
| dG | ng/mL | 1.2E1 | 1.5E1 | 1.6E1 | 2.0E1 | 2.0E1 | 1.7E1 | 3.0E0 | 4.8E0 | 1.8E2 | 8.7E1 | 112 | 31 | 112 | 31 | 0.61 |
| dH | pg/mL | 7.3E0 | 1.1E1 | 2.1E1 | 1.5E1 | 7.1E1 | 1.5E1 | 4.0E-2 | 4.0E-2 | 6.7E2 | 7.9E1 | 112 | 31 | 112 | 31 | 0.63 |
| dI | pg/mL | 4.6E-1 | 4.6E-1 | 4.5E0 | 1.9E0 | 3.1E1 | 2.4E0 | 4.6E-1 | 4.6E-1 | 3.3E2 | 8.4E0 | 112 | 31 | 112 | 31 | 0.56 |
| dJ | ng/mL | 1.9E0 | 2.2E0 | 2.1E0 | 2.2E0 | 1.1E0 | 1.3E0 | 3.2E-2 | 3.2E-2 | 5.1E0 | 4.4E0 | 112 | 31 | 112 | 31 | 0.55 |
| dK | uIU/mL | 1.5E0 | 8.8E-1 | 2.4E0 | 1.9E0 | 4.2E0 | 2.4E0 | 2.8E-4 | 2.9E-2 | 3.9E1 | 1.1E1 | 112 | 31 | 112 | 31 | 0.41 |
| dL | ng/mL | 8.7E2 | 1.0E3 | 1.0E3 | 1.2E3 | 5.5E2 | 8.9E2 | 2.8E2 | 4.3E2 | 3.4E3 | 4.8E3 | 112 | 31 | 112 | 31 | 0.56 |
| dM | pg/mL | 9.5E2 | 1.2E3 | 1.2E3 | 2.0E3 | 1.5E3 | 2.0E3 | 3.7E2 | 6.3E2 | 1.5E4 | 9.6E3 | 112 | 31 | 112 | 31 | 0.65 |
| dN | ug/mL | 9.7E1 | 1.1E2 | 1.0E2 | 1.2E2 | 3.6E1 | 5.7E1 | 2.4E1 | 4.7E1 | 2.2E2 | 3.3E2 | 112 | 31 | 112 | 31 | 0.58 |
| dR | pg/ml | 1.5E3 | 1.0E3 | 2.1E3 | 1.6E3 | 2.1E3 | 2.0E3 | 1.4E2 | 1.3E2 | 9.8E3 | 8.9E3 | 89 | 24 | 89 | 24 | 0.38 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dX | ng/ml | 5.2E-2 | 8.2E-2 | 1.4E-1 | 1.2E-1 | 2.4E-1 | 1.3E-1 | 2.6E-3 | 2.6E-3 | 1.2E0 | 4.2E-1 | 33 | 12 | 33 | 12 | 0.53 |
| eF | ng/ml | 4.1E0 | 4.4E0 | 4.8E0 | 5.1E0 | 2.2E0 | 2.7E0 | 2.0E0 | 2.0E0 | 1.2E1 | 1.5E1 | 89 | 24 | 89 | 24 | 0.54 |
| eC | pg/ml | 3.1E2 | 2.4E2 | 3.8E2 | 3.3E2 | 2.7E2 | 4.1E2 | 7.1E1 | 1.9E1 | 1.6E3 | 2.0E3 | 81 | 21 | 81 | 21 | 0.35 |
| eD | pg/ml | 2.2E2 | 1.8E2 | 7.3E2 | 4.3E2 | 1.5E3 | 9.0E2 | 5.2E-1 | 5.2E-1 | 7.0E3 | 3.8E3 | 67 | 16 | 67 | 16 | 0.48 |
| eM | ng/ml | 2.7E0 | 3.2E0 | 3.7E0 | 7.0E0 | 2.9E0 | 8.2E0 | 6.9E-1 | 8.8E-1 | 1.2E1 | 2.6E1 | 47 | 15 | 47 | 15 | 0.59 |
| eP | ng/ml | 3.7E-3 | 3.7E-3 | 6.7E-1 | 2.6E0 | 1.6E0 | 7.9E0 | 3.7E-3 | 3.7E-3 | 8.6E0 | 2.8E1 | 33 | 12 | 33 | 12 | 0.51 |
| eT | ng/ml | 2.8E2 | 5.6E2 | 6.8E2 | 9.7E2 | 8.0E2 | 1.1E3 | 1.0E2 | 7.1E1 | 2.9E3 | 2.9E3 | 40 | 9 | 40 | 9 | 0.51 |
| eZ | ng/ml | 4.5E1 | 6.2E1 | 5.5E1 | 6.9E1 | 2.6E1 | 2.4E1 | 1.8E1 | 3.2E1 | 1.2E2 | 1.1E2 | 40 | 9 | 40 | 9 | 0.69 |
| fN | ng/ml | 2.1E-1 | 2.1E-1 | 2.2E0 | 4.9E0 | 4.5E0 | 6.2E0 | 2.1E-1 | 2.1E-1 | 2.1E1 | 1.4E1 | 40 | 9 | 40 | 9 | 0.61 |
| fP | ng/ml | 2.8E2 | 2.8E2 | 3.3E2 | 3.4E2 | 2.0E2 | 1.7E2 | 1.8E0 | 9.5E1 | 1.0E3 | 7.7E2 | 87 | 23 | 87 | 23 | 0.53 |
| fR | ng/ml | 1.4E5 | 1.9E5 | 2.0E5 | 2.7E5 | 1.5E5 | 2.0E5 | 3.6E4 | 1.9E2 | 6.3E5 | 6.8E5 | 69 | 23 | 69 | 23 | 0.61 |
| fY | ng/ml | 2.5E2 | 2.5E2 | 2.5E2 | 2.4E2 | 1.1E2 | 9.0E1 | 3.6E1 | 1.2E2 | 4.8E2 | 3.8E2 | 40 | 9 | 40 | 9 | 0.48 |
| gC | ng/ml | 2.3E2 | 2.4E2 | 2.5E2 | 2.7E2 | 1.2E2 | 1.2E2 | 8.3E1 | 1.5E2 | 6.4E2 | 4.8E2 | 30 | 8 | 30 | 8 | 0.56 |
| gL | pg/ml | 6.2E4 | 7.4E4 | 6.9E4 | 8.4E4 | 3.1E4 | 3.6E4 | 1.1E4 | 4.3E4 | 1.6E5 | 1.7E5 | 89 | 24 | 89 | 24 | 0.62 |
| gP | U/ml | 2.7E2 | 2.8E2 | 2.8E2 | 2.9E2 | 1.3E2 | 8.9E1 | 1.2E1 | 1.3E2 | 1.1E3 | 5.2E2 | 88 | 24 | 88 | 24 | 0.54 |
| gW | ng/ml | 5.2E2 | 3.4E2 | 8.8E2 | 6.8E2 | 1.1E3 | 7.7E2 | 2.3E0 | 5.5E1 | 6.1E3 | 3.1E3 | 65 | 18 | 65 | 18 | 0.45 |
| tF | pg/mL | 1.3E3 | 1.1E3 | 1.5E4 | 4.4E3 | 4.6E4 | 6.8E3 | 1.2E1 | 1.8E1 | 2.8E5 | 2.4E4 | 82 | 22 | 82 | 22 | 0.48 |
| hA | ng/ml | 2.5E0 | 4.2E0 | 1.6E1 | 1.6E1 | 5.8E1 | 2.9E1 | 1.7E-2 | 1.7E-2 | 3.5E2 | 1.1E2 | 67 | 17 | 67 | 17 | 0.67 |
| nM | pg/ml | 1.0E-9 | 1.0E-9 | 1.0E0 | 1.1E2 | 0.0E0 | 4.1E2 | 1.0E-9 | 1.0E-9 | 1.0E-9 | 1.5E3 | 50 | 14 | 50 | 14 | 0.54 |
| nN | pg/ml | 1.2E3 | 3.6E3 | 4.3E3 | 1.8E4 | 1.5E4 | 4.0E4 | 1.1E2 | 4.1E2 | 1.0E5 | 1.5E5 | 50 | 14 | 50 | 14 | 0.70 |
| nO | pg/ml | 2.2E1 | 2.9E1 | 3.8E1 | 3.4E1 | 5.2E1 | 2.0E1 | 4.0E0 | 1.2E1 | 3.1E2 | 8.9E1 | 50 | 14 | 50 | 14 | 0.59 |
| nR | pg/ml | 1.6E1 | 4.4E1 | 6.8E1 | 2.2E2 | 1.5E2 | 5.0E2 | 1.0E0 | 2.7E0 | 8.2E2 | 1.9E3 | 50 | 14 | 50 | 14 | 0.65 |
| nT | pg/ml | 7.3E1 | 9.6E1 | 1.0E2 | 1.7E2 | 1.1E2 | 2.3E2 | 1.0E-9 | 2.3E1 | 6.4E2 | 9.2E2 | 50 | 14 | 50 | 14 | 0.58 |
| nU | pg/ml | 3.0E1 | 1.0E2 | 8.2E1 | 1.8E2 | 2.2E2 | 2.4E2 | 1.0E-9 | 1.0E-9 | 1.5E3 | 9.2E2 | 50 | 14 | 50 | 14 | 0.76 |
| lW | pg/ml | 1.0E-9 | 1.0E-9 | 1.2E1 | 1.1E1 | 3.4E1 | 2.1E1 | 1.0E-9 | 1.0E-9 | 1.7E2 | 7.0E1 | 50 | 14 | 50 | 14 | 0.51 |
| lX | pg/ml | 9.0E2 | 7.4E2 | 9.5E2 | 9.7E2 | 5.1E2 | 7.1E2 | 2.3E2 | 1.9E2 | 2.3E3 | 2.5E3 | 50 | 14 | 50 | 14 | 0.47 |
| lY | pg/ml | 1.8E1 | 2.1E1 | 2.1E1 | 2.1E1 | 1.9E1 | 1.4E1 | 1.0E-9 | 5.7E-1 | 1.2E2 | 4.5E1 | 50 | 14 | 50 | 14 | 0.55 |
| mE | pg/ml | 1.0E-9 | 3.5E-1 | 2.4E0 | 3.8E0 | 8.2E0 | 7.4E0 | 1.0E-9 | 1.0E-9 | 5.7E1 | 2.8E1 | 50 | 14 | 50 | 14 | 0.58 |
| mF | pg/ml | 1.4E-1 | 4.0E-1 | 8.9E0 | 1.3E0 | 3.7E1 | 2.2E0 | 1.0E-9 | 1.0E-9 | 2.5E2 | 6.5E0 | 50 | 14 | 50 | 14 | 0.50 |
| mH | pg/ml | 3.2E0 | 4.0E0 | 5.3E0 | 5.5E0 | 8.4E0 | 4.9E0 | 4.0E-1 | 9.0E-1 | 5.3E1 | 1.9E1 | 50 | 14 | 50 | 14 | 0.58 |
| mI | pg/ml | 1.0E-9 | 2.2E1 | 1.1E1 | 5.5E1 | 2.8E1 | 1.2E2 | 1.0E-9 | 1.0E-9 | 1.8E2 | 4.6E2 | 50 | 14 | 50 | 14 | 0.68 |
| mM | pg/ml | 3.8E1 | 6.1E1 | 9.3E1 | 9.3E1 | 1.7E2 | 1.3E2 | 1.0E-9 | 1.0E-9 | 1.1E3 | 4.7E2 | 50 | 14 | 50 | 14 | 0.54 |
| mP | pg/ml | 1.4E1 | 1.7E1 | 1.7E1 | 8.3E1 | 1.8E1 | 2.1E2 | 1.0E-9 | 7.5E0 | 1.2E2 | 8.1E2 | 49 | 14 | 49 | 14 | 0.61 |
| mS | pg/ml | 1.6E3 | 1.9E3 | 1.7E3 | 1.8E3 | 9.7E2 | 9.8E2 | 1.0E-9 | 1.0E-9 | 5.1E3 | 3.5E3 | 50 | 14 | 50 | 14 | 0.55 |
| mT | pg/ml | 7.0E1 | 6.0E1 | 1.4E2 | 2.5E2 | 2.5E2 | 4.8E2 | 1.0E1 | 1.7E1 | 1.4E3 | 1.7E3 | 49 | 14 | 49 | 14 | 0.51 |
| mU | pg/ml | 2.1E0 | 3.4E0 | 7.5E0 | 5.1E0 | 3.2E1 | 5.6E0 | 1.0E-9 | 1.2E0 | 2.2E2 | 2.3E1 | 49 | 14 | 49 | 14 | 0.66 |
| mW | pg/ml | 2.2E3 | 1.9E3 | 2.4E3 | 2.9E3 | 1.3E3 | 2.7E3 | 1.0E-9 | 8.9E2 | 6.2E3 | 1.1E4 | 49 | 14 | 49 | 14 | 0.48 |
| mY | pg/ml | 6.0E2 | 9.5E2 | 8.6E2 | 1.4E3 | 1.0E3 | 2.0E3 | 1.0E-9 | 1.9E2 | 5.6E3 | 8.0E3 | 50 | 14 | 50 | 14 | 0.65 |
| mZ | pg/ml | 1.9E2 | 9.4E1 | 3.9E2 | 2.5E2 | 5.2E2 | 3.5E2 | 1.2E1 | 1.1E1 | 3.1E3 | 1.2E3 | 49 | 14 | 49 | 14 | 0.34 |
| nA | pg/ml | 1.5E0 | 3.9E0 | 7.1E0 | 4.5E0 | 1.5E1 | 5.3E0 | 1.0E-9 | 1.0E-9 | 6.5E1 | 1.9E1 | 49 | 14 | 49 | 14 | 0.54 |
| nB | pg/ml | 2.7E2 | 3.5E2 | 3.0E2 | 3.8E2 | 1.7E2 | 2.1E2 | 3.0E1 | 1.5E2 | 8.2E2 | 9.6E2 | 50 | 14 | 50 | 14 | 0.60 |
| nC | pg/ml | 1.0E-9 | 2.3E2 | 1.3E4 | 1.9E3 | 6.2E4 | 3.1E3 | 1.0E-9 | 1.0E-9 | 3.8E5 | 9.1E3 | 50 | 14 | 50 | 14 | 0.68 |
| nD | pg/ml | 6.7E0 | 7.5E0 | 1.3E1 | 1.1E1 | 3.7E1 | 9.7E0 | 1.0E-9 | 1.0E-9 | 2.6E2 | 2.8E1 | 49 | 14 | 49 | 14 | 0.56 |
| nF | pg/ml | 1.0E-9 | 1.0E-9 | 3.4E0 | 9.6E-1 | 1.4E1 | 3.6E0 | 1.0E-9 | 1.0E-9 | 9.3E1 | 1.3E1 | 50 | 14 | 50 | 14 | 0.49 |
| nH | pg/ml | 1.8E-1 | 4.7E0 | 2.6E2 | 2.7E1 | 1.5E3 | 4.3E1 | 1.0E-9 | 1.0E-9 | 1.0E4 | 1.2E2 | 49 | 14 | 49 | 14 | 0.70 |
| nI | pg/ml | 4.6E1 | 1.0E-9 | 6.3E1 | 1.3E1 | 8.0E1 | 2.6E1 | 1.0E-9 | 1.0E-9 | 3.5E2 | 7.9E1 | 50 | 14 | 50 | 14 | 0.30 |
| nJ | pg/ml | 1.7E-1 | 1.1E0 | 3.6E0 | 1.1E0 | 1.9E1 | 9.5E-1 | 1.0E-9 | 1.0E-9 | 1.3E2 | 3.0E0 | 50 | 14 | 50 | 14 | 0.59 |
| nK | pg/ml | 1.0E-9 | 1.8E1 | 9.8E0 | 2.5E1 | 2.0E1 | 3.0E1 | 1.0E-9 | 1.0E-9 | 1.0E2 | 9.6E1 | 49 | 14 | 49 | 14 | 0.66 |
| nL | pg/ml | 1.0E-9 | 7.2E0 | 3.7E2 | 9.2E1 | 2.0E3 | 2.0E2 | 1.0E-9 | 1.0E-9 | 1.4E4 | 7.3E2 | 50 | 14 | 50 | 14 | 0.68 |
| hL | pg/ml | 1.7E4 | 2.6E4 | 2.1E4 | 2.7E4 | 1.4E4 | 1.6E4 | 2.6E3 | 7.9E3 | 7.2E4 | 6.0E4 | 40 | 9 | 40 | 9 | 0.64 |
| hO | pg/ml | 1.6E4 | 1.5E4 | 1.6E4 | 1.5E4 | 2.5E3 | 2.2E3 | 1.3E4 | 1.1E4 | 2.4E4 | 1.8E4 | 40 | 9 | 40 | 9 | 0.35 |
| hP | ng/ml | 4.4E5 | 4.4E5 | 4.6E5 | 6.5E5 | 3.9E5 | 8.2E5 | 1.7E4 | 3.4E4 | 2.6E6 | 2.8E6 | 40 | 9 | 40 | 9 | 0.51 |
| wJ | pg/ml | 1.5E5 | 8.8E4 | 1.8E5 | 9.4E4 | 1.2E5 | 5.5E4 | 1.1E4 | 2.3E4 | 5.8E5 | 2.3E5 | 41 | 10 | 41 | 10 | 0.27 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|--------|--------|--------|------|
| | | NonDis | Dis | NonDis | Dis | NonDis | Dis | NonDis | Dis | NonDis | Dis | NonDis | Dis | NonDis | Dis | |
| wK | pg/ml | 3.2E4 | 2.9E4 | 5.3E4 | 3.7E4 | 7.8E4 | 2.8E4 | 3.7E3 | 1.1E4 | 5.0E5 | 1.0E5 | 41 | 10 | 41 | 10 | 0.42 |
| wL | pg/ml | 6.7E0 | 6.5E-1 | 5.7E1 | 4.0E1 | 1.5E2 | 1.1E2 | 1.0E-9 | 1.0E-9 | 8.4E2 | 3.5E2 | 41 | 10 | 41 | 10 | 0.38 |
| wP | pg/ml | 2.8E4 | 4.9E4 | 4.4E4 | 8.2E4 | 4.8E4 | 9.0E4 | 1.3E3 | 1.8E4 | 2.1E5 | 3.0E5 | 41 | 10 | 41 | 10 | 0.66 |
| wQ | pg/ml | 3.9E1 | 3.7E1 | 6.9E1 | 3.4E1 | 1.0E2 | 2.2E1 | 1.0E-9 | 1.0E-9 | 5.1E2 | 6.6E1 | 41 | 10 | 41 | 10 | 0.45 |
| hR | pg/ml | 2.7E4 | 2.9E4 | 2.9E4 | 2.9E4 | 1.1E4 | 1.0E4 | 1.0E-9 | 1.4E4 | 5.8E4 | 4.5E4 | 62 | 15 | 62 | 15 | 0.48 |
| hV | pg/ml | 4.4E2 | 4.3E2 | 4.4E2 | 4.1E2 | 2.2E2 | 1.9E2 | 1.0E-9 | 9.5E1 | 9.6E2 | 7.4E2 | 62 | 15 | 62 | 15 | 0.47 |
| hW | pg/ml | 1.8E3 | 2.4E3 | 2.1E3 | 5.1E3 | 1.2E3 | 9.7E3 | 1.0E-9 | 1.2E3 | 7.3E3 | 4.0E4 | 62 | 15 | 62 | 15 | 0.67 |
| hX | pg/ml | 1.0E3 | 1.1E3 | 1.2E3 | 1.1E3 | 1.1E3 | 4.7E2 | 2.5E0 | 3.1E2 | 8.6E3 | 2.0E3 | 62 | 15 | 62 | 15 | 0.57 |
| iA | pg/ml | 1.7E2 | 2.2E2 | 2.6E2 | 3.2E2 | 2.9E2 | 2.5E2 | 1.5E1 | 6.2E1 | 1.8E3 | 8.7E2 | 82 | 22 | 82 | 22 | 0.60 |
| iB | ng/ml | 4.9E0 | 7.3E0 | 6.1E0 | 9.9E0 | 4.5E0 | 6.6E0 | 3.3E-2 | 2.4E0 | 2.4E1 | 2.3E1 | 67 | 17 | 67 | 17 | 0.69 |
| iC | U/ml | 3.1E-1 | 5.8E-1 | 1.4E0 | 8.6E-1 | 6.8E0 | 8.7E-1 | 1.0E-9 | 6.8E-2 | 5.5E1 | 3.2E0 | 67 | 17 | 67 | 17 | 0.68 |
| tQ | pg/ml | 1.4E3 | 1.7E3 | 1.4E3 | 1.7E3 | 5.5E2 | 7.4E2 | 3.7E2 | 8.4E2 | 2.5E3 | 3.3E3 | 38 | 9 | 38 | 9 | 0.57 |
| tT | pg/ml | 1.9E1 | 2.3E1 | 2.0E1 | 3.1E1 | 1.0E1 | 2.6E1 | 5.4E0 | 1.1E1 | 4.8E1 | 9.3E1 | 39 | 9 | 39 | 9 | 0.65 |
| tS | pg/ml | 7.7E-1 | 6.9E-1 | 1.1E0 | 1.7E0 | 1.1E0 | 3.1E0 | 1.0E-9 | 1.0E-9 | 4.9E0 | 1.0E1 | 39 | 10 | 39 | 10 | 0.49 |
| tX | pg/ml | 1.0E0 | 1.6E0 | 1.3E0 | 3.4E0 | 1.3E0 | 3.6E0 | 2.5E-2 | 3.7E-1 | 7.0E0 | 1.0E1 | 39 | 9 | 39 | 9 | 0.67 |
| tO | pg/ml | 4.4E0 | 3.3E0 | 5.0E0 | 4.7E0 | 3.3E0 | 4.0E0 | 1.0E-9 | 1.7E0 | 1.8E1 | 1.5E1 | 39 | 10 | 39 | 10 | 0.42 |
| tR | pg/ml | 1.9E-1 | 2.2E-1 | 2.8E-1 | 4.3E-1 | 3.2E-1 | 7.5E-1 | 1.0E-9 | 1.4E-2 | 1.6E0 | 2.5E0 | 38 | 10 | 38 | 10 | 0.52 |
| tU | pg/ml | 9.2E0 | 1.3E1 | 1.1E1 | 1.9E1 | 1.1E1 | 2.2E1 | 2.2E-1 | 4.9E0 | 5.5E1 | 8.0E1 | 40 | 10 | 40 | 10 | 0.64 |
| tN | pg/ml | 1.9E1 | 2.2E1 | 2.6E1 | 4.8E1 | 2.7E1 | 5.0E1 | 1.0E-9 | 9.5E0 | 1.5E2 | 1.6E2 | 38 | 9 | 38 | 9 | 0.64 |
| tV | ng/ml | 5.4E2 | 9.5E2 | 7.0E2 | 1.1E3 | 5.0E2 | 8.2E2 | 5.3E1 | 3.9E2 | 1.8E3 | 3.1E3 | 41 | 9 | 41 | 9 | 0.67 |
| iH | ng/ml | 1.6E5 | 1.9E5 | 1.6E5 | 1.8E5 | 5.0E4 | 5.4E4 | 2.9E3 | 7.7E4 | 2.6E5 | 2.5E5 | 82 | 22 | 82 | 22 | 0.62 |
| iJ | ng/ml | 5.3E4 | 4.1E4 | 5.7E4 | 5.0E4 | 3.8E4 | 2.9E4 | 1.8E3 | 1.2E4 | 2.5E5 | 1.5E5 | 82 | 22 | 82 | 22 | 0.42 |
| hB | ng/ml | 4.5E-1 | 6.3E-1 | 5.7E-1 | 9.7E-1 | 4.1E-1 | 7.4E-1 | 1.2E-1 | 2.9E-1 | 2.3E0 | 3.2E0 | 82 | 22 | 82 | 22 | 0.73 |
| hC | pg/ml | 4.6E3 | 1.0E4 | 7.7E3 | 1.1E4 | 1.3E4 | 1.2E4 | 4.1E1 | 3.0E2 | 1.1E5 | 5.7E4 | 82 | 22 | 82 | 22 | 0.62 |
| hF | ng/ml | 1.0E-9 | 1.0E-9 | 4.9E1 | 1.0E-9 | 4.4E2 | 0.0E0 | 1.0E-9 | 1.0E-9 | 4.0E3 | 1.0E-9 | 82 | 22 | 82 | 22 | 0.49 |
| hG | pg/ml | 6.8E3 | 6.7E3 | 7.7E3 | 8.2E3 | 3.2E3 | 4.4E3 | 1.8E3 | 3.6E3 | 1.8E4 | 2.0E4 | 82 | 22 | 82 | 22 | 0.49 |
| iO | ng/ml | 4.0E5 | 3.2E5 | 4.5E5 | 3.8E5 | 2.0E5 | 1.9E5 | 1.0E5 | 9.8E4 | 1.1E6 | 8.2E5 | 82 | 22 | 82 | 22 | 0.39 |
| iP | ng/ml | 5.4E4 | 4.5E4 | 6.1E4 | 5.0E4 | 5.3E4 | 2.1E4 | 7.1E3 | 2.1E4 | 4.4E5 | 9.7E4 | 82 | 22 | 82 | 22 | 0.44 |
| iZ | ng/ml | 1.6E3 | 2.1E3 | 1.8E3 | 2.3E3 | 7.9E2 | 1.0E3 | 7.5E2 | 1.1E3 | 5.7E3 | 4.6E3 | 80 | 22 | 80 | 22 | 0.68 |
| yH | pg/ml | 9.1E2 | 1.4E3 | 2.6E3 | 1.9E3 | 5.7E3 | 1.9E3 | 1.0E-9 | 7.0E2 | 2.5E4 | 6.8E3 | 40 | 9 | 40 | 9 | 0.64 |
| yK | U/ml | 1.7E1 | 2.8E1 | 4.2E1 | 4.0E1 | 7.8E1 | 4.3E1 | 1.0E-9 | 1.0E-9 | 3.8E2 | 1.4E2 | 40 | 9 | 40 | 9 | 0.59 |
| yJ | pg/ml | 3.3E4 | 4.0E4 | 4.1E4 | 4.3E4 | 2.9E4 | 2.3E4 | 1.9E3 | 1.1E4 | 1.4E5 | 8.2E4 | 40 | 9 | 40 | 9 | 0.56 |
| yD | ng/ml | 1.3E-2 | 1.3E-2 | 1.3E-2 | 1.3E-2 | 6.1E-3 | 5.5E-3 | 1.0E-9 | 6.3E-3 | 2.8E-2 | 2.4E-2 | 41 | 10 | 41 | 10 | 0.50 |
| wB | pg/ml | 8.7E3 | 2.2E4 | 9.8E3 | 2.1E4 | 7.1E3 | 1.1E4 | 1.9E3 | 5.3E3 | 3.3E4 | 4.2E4 | 41 | 10 | 41 | 10 | 0.80 |
| pY | pg/ml | 5.9E0 | 6.4E0 | 1.1E1 | 7.4E0 | 3.1E1 | 3.8E0 | 1.6E0 | 4.4E0 | 2.0E2 | 1.7E1 | 40 | 9 | 40 | 9 | 0.61 |
| rC | pg/ml | 1.4E3 | 1.1E3 | 2.2E3 | 1.8E3 | 2.6E3 | 1.8E3 | 1.0E-9 | 7.0E1 | 1.5E4 | 7.3E3 | 59 | 15 | 59 | 15 | 0.45 |
| rB | pg/ml | 2.9E1 | 3.8E1 | 5.1E1 | 6.8E1 | 6.9E1 | 9.1E1 | 1.0E-9 | 1.0E-9 | 3.9E2 | 3.2E2 | 59 | 15 | 59 | 15 | 0.52 |
| zG | 2.5ng/ml | 2.2E-1 | 3.8E-1 | 5.9E-1 | 6.3E-1 | 1.1E0 | 8.6E-1 | 1.0E-9 | 1.0E-9 | 4.8E0 | 2.7E0 | 40 | 9 | 40 | 9 | 0.59 |
| zH | 2.3mU/ml | 8.8E-2 | 8.5E-2 | 9.8E-2 | 8.4E-2 | 5.0E-2 | 2.6E-2 | 1.0E-2 | 4.4E-2 | 3.1E-1 | 1.2E-1 | 40 | 9 | 40 | 9 | 0.42 |
| zI | 2.6ng/ml | 2.1E0 | 3.4E0 | 4.2E0 | 6.0E0 | 5.8E0 | 5.9E0 | 5.4E-1 | 9.1E-1 | 2.7E1 | 1.6E1 | 40 | 9 | 40 | 9 | 0.63 |
| qA | ng/ml | 8.9E6 | 1.3E7 | 1.1E7 | 1.4E7 | 7.2E6 | 7.7E6 | 3.7E6 | 4.3E6 | 3.9E7 | 3.0E7 | 40 | 9 | 40 | 9 | 0.66 |
| qB | ng/ml | 7.2E5 | 5.7E5 | 8.3E5 | 9.9E5 | 5.7E5 | 1.1E6 | 1.9E5 | 2.4E5 | 2.9E6 | 3.8E6 | 40 | 9 | 40 | 9 | 0.44 |
| qC | ng/ml | 3.6E5 | 2.4E5 | 6.8E5 | 2.4E5 | 9.4E5 | 1.2E5 | 2.5E4 | 6.5E4 | 4.7E6 | 5.0E5 | 40 | 9 | 40 | 9 | 0.34 |
| qD | ng/ml | 1.6E7 | 1.4E7 | 1.7E7 | 1.4E7 | 7.7E6 | 5.1E6 | 7.0E6 | 1.0E7 | 3.7E7 | 2.6E7 | 40 | 9 | 40 | 9 | 0.39 |
| jD | ng/ml | 3.7E1 | 4.7E1 | 4.3E1 | 6.4E1 | 4.1E1 | 7.6E1 | 1.0E-9 | 1.0E-9 | 1.9E2 | 2.9E2 | 67 | 17 | 67 | 17 | 0.57 |
| jE | ng/ml | 1.0E-9 | 1.0E-9 | 4.9E0 | 5.9E0 | 1.2E1 | 1.2E1 | 1.0E-9 | 1.0E-9 | 6.2E1 | 3.5E1 | 67 | 17 | 67 | 17 | 0.52 |
| jF | ng/ml | 4.2E1 | 7.7E0 | 4.9E1 | 2.6E1 | 5.3E1 | 3.8E1 | 1.0E-9 | 1.0E-9 | 1.9E2 | 1.2E2 | 67 | 17 | 67 | 17 | 0.38 |
| jG | ng/ml | 4.4E3 | 4.2E3 | 4.6E3 | 4.1E3 | 2.0E3 | 1.8E3 | 6.7E2 | 1.5E3 | 9.6E3 | 7.9E3 | 67 | 17 | 67 | 17 | 0.43 |
| jH | ng/ml | 7.9E1 | 6.3E1 | 8.2E1 | 9.8E1 | 4.5E1 | 9.5E1 | 1.3E1 | 3.3E1 | 2.4E2 | 4.3E2 | 67 | 17 | 67 | 17 | 0.48 |
| jI | ng/ml | 7.3E1 | 9.6E1 | 7.9E1 | 1.2E2 | 3.3E1 | 1.0E2 | 3.8E1 | 4.4E1 | 1.9E2 | 4.4E2 | 67 | 17 | 67 | 17 | 0.63 |
| sK | pg/mL | 4.1E3 | 3.6E3 | 4.2E3 | 5.9E3 | 1.9E3 | 6.7E3 | 1.8E3 | 2.1E3 | 1.0E4 | 2.3E4 | 38 | 9 | 38 | 9 | 0.50 |
| sM | pg/mL | 7.3E4 | 7.9E4 | 7.9E4 | 9.3E4 | 3.1E4 | 4.3E4 | 3.9E4 | 5.1E4 | 1.6E5 | 2.0E5 | 38 | 9 | 38 | 9 | 0.62 |
| sO | pg/mL | 2.3E8 | 1.9E8 | 2.5E8 | 1.9E8 | 9.1E7 | 9.6E7 | 4.9E7 | 6.6E7 | 4.4E8 | 3.2E8 | 38 | 9 | 38 | 9 | 0.35 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| wC | ng/ml | 1.5E0 | 1.7E0 | 1.9E0 | 1.7E0 | 1.3E0 | 1.2E0 | 3.6E-1 | 6.1E-2 | 6.5E0 | 4.3E0 | 41 | 10 | 41 | 10 | 0.50 |
| wD | ng/ml | 2.1E1 | 4.4E1 | 8.7E1 | 8.6E1 | 3.3E2 | 8.7E1 | 2.8E0 | 2.1E1 | 2.1E3 | 2.9E2 | 41 | 10 | 41 | 10 | 0.75 |
| wE | ng/ml | 5.0E1 | 4.1E1 | 5.2E1 | 4.7E1 | 1.9E1 | 2.0E1 | 8.1E0 | 2.0E1 | 8.9E1 | 8.9E1 | 41 | 10 | 41 | 10 | 0.39 |
| wG | ng/ml | 1.2E-1 | 5.8E-2 | 1.3E-1 | 1.5E-1 | 1.2E-1 | 2.3E-1 | 1.0E-9 | 1.0E-9 | 4.8E-1 | 6.8E-1 | 41 | 10 | 41 | 10 | 0.41 |
| wH | ng/ml | 3.3E-2 | 4.3E-2 | 2.5E-1 | 9.2E-1 | 7.0E-1 | 1.8E0 | 1.0E-9 | 1.0E-9 | 4.2E0 | 5.6E0 | 41 | 10 | 41 | 10 | 0.57 |
| wF | ng/ml | 1.8E-1 | 6.2E-1 | 2.7E0 | 1.8E0 | 9.9E0 | 2.0E0 | 1.0E-9 | 1.0E-9 | 5.7E1 | 4.7E0 | 41 | 10 | 41 | 10 | 0.63 |
| rA | pg/ml | 2.4E1 | 2.2E1 | 2.7E1 | 3.1E1 | 2.1E1 | 2.1E1 | 1.0E-9 | 5.8E0 | 1.1E2 | 7.2E1 | 64 | 17 | 64 | 17 | 0.55 |
| qZ | pg/ml | 4.7E1 | 9.1E1 | 6.7E2 | 4.2E2 | 2.4E3 | 9.5E2 | 2.8E-4 | 3.2E-3 | 1.0E4 | 3.4E3 | 48 | 12 | 48 | 12 | 0.59 |
| qY | pg/ml | 1.5E1 | 1.3E1 | 4.0E1 | 2.5E1 | 5.9E1 | 2.8E1 | 8.7E-1 | 2.1E0 | 3.3E2 | 9.8E1 | 64 | 17 | 64 | 17 | 0.45 |
| qX | pg/ml | 5.5E1 | 6.8E1 | 6.6E1 | 8.8E1 | 4.3E1 | 6.3E1 | 1.0E-9 | 2.3E1 | 1.7E2 | 2.1E2 | 64 | 17 | 64 | 17 | 0.57 |
| qW | pg/ml | 7.7E0 | 7.0E0 | 9.4E0 | 9.4E0 | 8.8E0 | 9.6E0 | 1.0E-9 | 1.0E-9 | 3.6E1 | 3.1E1 | 64 | 17 | 64 | 17 | 0.47 |
| qV | pg/ml | 1.6E3 | 1.6E3 | 2.3E3 | 2.5E3 | 1.8E3 | 2.4E3 | 1.0E2 | 1.7E2 | 1.1E4 | 9.6E3 | 64 | 17 | 64 | 17 | 0.50 |
| qU | pg/ml | 7.7E1 | 9.2E1 | 1.9E2 | 2.2E2 | 3.3E2 | 3.2E2 | 1.0E-9 | 1.0E-9 | 2.1E3 | 1.1E3 | 64 | 17 | 64 | 17 | 0.52 |
| qT | pg/ml | 3.8E1 | 4.3E1 | 6.7E1 | 6.5E1 | 7.9E1 | 5.0E1 | 1.0E-9 | 6.9E0 | 4.9E2 | 1.6E2 | 64 | 17 | 64 | 17 | 0.55 |
| qI | ng/ml | 6.3E4 | 5.9E4 | 6.5E4 | 5.9E4 | 3.2E4 | 2.6E4 | 1.0E4 | 2.5E4 | 1.6E5 | 9.8E4 | 37 | 9 | 37 | 9 | 0.47 |
| qH | ng/ml | 5.9E4 | 5.9E4 | 7.1E4 | 7.3E4 | 4.2E4 | 3.2E4 | 1.5E4 | 4.7E4 | 1.8E5 | 1.4E5 | 37 | 9 | 37 | 9 | 0.57 |
| qG | ng/ml | 1.8E5 | 1.7E5 | 1.9E5 | 1.7E5 | 7.3E4 | 4.8E4 | 3.4E4 | 9.9E4 | 4.2E5 | 2.3E5 | 37 | 9 | 37 | 9 | 0.43 |
| jK | ng/ml | 1.5E3 | 1.3E3 | 1.7E3 | 1.4E3 | 6.5E2 | 5.2E2 | 2.8E2 | 7.5E2 | 4.1E3 | 2.9E3 | 67 | 17 | 67 | 17 | 0.33 |
| jL | ng/ml | 1.8E2 | 2.6E2 | 2.7E2 | 2.9E2 | 2.1E2 | 1.5E2 | 5.9E1 | 1.2E2 | 8.1E2 | 6.3E2 | 67 | 17 | 67 | 17 | 0.61 |
| jM | ng/ml | 7.1E4 | 5.5E4 | 7.4E4 | 6.4E4 | 3.9E4 | 4.0E4 | 4.6E3 | 1.1E4 | 1.7E5 | 1.4E5 | 67 | 17 | 67 | 17 | 0.41 |
| jO | pg/ml | 2.3E5 | 2.5E5 | 2.8E5 | 2.5E5 | 1.5E5 | 1.4E5 | 7.6E4 | 9.8E4 | 7.7E5 | 6.5E5 | 67 | 17 | 67 | 17 | 0.44 |
| jP | pg/ml | 2.7E5 | 2.5E5 | 3.0E5 | 3.1E5 | 1.5E5 | 1.6E5 | 6.1E4 | 1.3E5 | 7.1E5 | 5.5E5 | 67 | 17 | 67 | 17 | 0.49 |
| jQ | pg/ml | 2.2E3 | 2.3E3 | 3.1E3 | 2.6E3 | 2.8E3 | 2.3E3 | 5.0E0 | 2.9E2 | 1.3E4 | 9.2E3 | 67 | 17 | 67 | 17 | 0.45 |
| jR | pg/ml | 5.7E3 | 3.8E3 | 9.9E3 | 8.6E3 | 1.1E4 | 1.2E4 | 1.0E-9 | 3.0E1 | 5.6E4 | 4.6E4 | 67 | 17 | 67 | 17 | 0.44 |
| jT | pg/ml | 1.9E5 | 1.5E5 | 1.8E5 | 1.4E5 | 6.2E4 | 4.3E4 | 7.1E4 | 7.5E4 | 3.5E5 | 2.2E5 | 67 | 17 | 67 | 17 | 0.31 |
| xA | pg/ml | 5.2E0 | 7.3E0 | 1.4E1 | 1.8E1 | 2.8E1 | 3.4E1 | 1.0E-9 | 1.0E-9 | 1.6E2 | 1.1E2 | 40 | 9 | 40 | 9 | 0.54 |
| yE | pg/ml | 8.1E1 | 9.1E1 | 8.0E1 | 1.0E2 | 3.3E1 | 4.2E1 | 6.4E0 | 6.3E1 | 1.5E2 | 2.0E2 | 40 | 9 | 40 | 9 | 0.64 |
| tM | pg/ml | 4.3E1 | 3.9E1 | 3.9E1 | 4.5E1 | 1.8E1 | 2.3E1 | 1.0E-9 | 1.6E1 | 8.3E1 | 9.9E1 | 40 | 9 | 40 | 9 | 0.53 |
| tL | pg/ml | 1.0E-9 | 1.0E-9 | 2.0E0 | 2.7E-1 | 1.1E1 | 6.1E-1 | 1.0E-9 | 1.0E-9 | 6.7E1 | 1.8E0 | 40 | 9 | 40 | 9 | 0.50 |
| jU | mIU/ml | 5.4E0 | 6.7E0 | 1.2E1 | 1.3E1 | 2.0E1 | 1.5E1 | 8.1E-2 | 1.2E0 | 1.1E2 | 5.3E1 | 67 | 17 | 67 | 17 | 0.53 |
| jV | mIU/ml | 1.9E0 | 1.9E0 | 4.1E0 | 3.7E0 | 5.7E0 | 4.8E0 | 2.7E-3 | 1.0E-1 | 3.2E1 | 1.8E1 | 67 | 17 | 67 | 17 | 0.46 |
| jY | ng/ml | 7.4E-4 | 2.7E-3 | 7.8E-3 | 1.1E-2 | 3.7E-2 | 2.3E-2 | 1.0E-9 | 1.0E-9 | 3.0E-1 | 9.4E-2 | 67 | 17 | 67 | 17 | 0.65 |
| kC | pg/ml | 1.1E2 | 9.2E1 | 2.1E2 | 1.3E2 | 4.1E2 | 1.4E2 | 2.1E1 | 3.6E1 | 2.7E3 | 5.9E2 | 50 | 14 | 50 | 14 | 0.42 |
| kE | pg/ml | 1.5E5 | 1.4E5 | 1.4E5 | 1.4E5 | 3.7E4 | 5.4E4 | 4.1E4 | 3.8E4 | 2.3E5 | 2.7E5 | 50 | 14 | 50 | 14 | 0.41 |
| kF | pg/mL | 6.9E1 | 5.3E1 | 7.0E1 | 7.2E1 | 2.3E1 | 3.5E1 | 3.5E1 | 4.0E1 | 1.5E2 | 1.4E2 | 50 | 14 | 50 | 14 | 0.42 |
| kG | pg/mL | 8.9E3 | 8.6E3 | 1.2E4 | 2.4E4 | 1.0E4 | 4.2E4 | 1.9E3 | 1.1E3 | 5.8E4 | 1.6E5 | 50 | 14 | 50 | 14 | 0.49 |
| kI | pg/ml | 2.1E2 | 1.9E2 | 2.2E2 | 2.3E2 | 1.1E2 | 1.4E2 | 4.4E1 | 1.0E-9 | 6.7E2 | 5.5E2 | 50 | 14 | 50 | 14 | 0.50 |
| kK | pg/ml | 1.2E2 | 1.2E2 | 1.5E2 | 1.7E2 | 1.5E2 | 1.2E2 | 2.1E1 | 2.9E1 | 9.1E2 | 4.9E2 | 50 | 14 | 50 | 14 | 0.58 |
| kN | pg/ml | 1.2E3 | 7.5E2 | 1.7E3 | 1.3E3 | 1.9E3 | 1.7E3 | 2.1E2 | 3.8E2 | 1.0E4 | 7.0E3 | 50 | 14 | 50 | 14 | 0.36 |
| kO | pg/ml | 7.2E3 | 7.0E3 | 1.1E4 | 7.4E3 | 2.0E4 | 2.5E3 | 3.8E3 | 4.4E3 | 1.5E5 | 1.3E4 | 50 | 14 | 50 | 14 | 0.45 |
| kP | pg/ml | 5.9E3 | 4.5E3 | 6.9E3 | 5.6E3 | 4.4E3 | 3.7E3 | 9.6E2 | 1.6E3 | 2.7E4 | 1.5E4 | 50 | 14 | 50 | 14 | 0.40 |
| kQ | pg/ml | 4.7E3 | 4.3E3 | 5.6E3 | 5.6E3 | 4.2E3 | 4.4E3 | 5.6E2 | 1.4E3 | 2.5E4 | 2.2E4 | 82 | 22 | 82 | 22 | 0.48 |
| kR | pg/ml | 2.4E1 | 2.2E1 | 4.0E1 | 3.7E1 | 1.1E2 | 3.2E1 | 1.0E-9 | 5.5E0 | 1.0E3 | 1.1E2 | 82 | 22 | 82 | 22 | 0.53 |
| kS | pg/ml | 8.6E2 | 9.4E2 | 9.8E2 | 1.0E3 | 6.0E2 | 5.9E2 | 8.2E1 | 2.5E2 | 3.2E3 | 2.5E3 | 82 | 22 | 82 | 22 | 0.54 |
| pS | ng/ml | 1.6E5 | 1.2E5 | 2.0E5 | 1.3E5 | 1.1E5 | 5.5E4 | 7.5E4 | 6.8E4 | 5.7E5 | 2.6E5 | 38 | 9 | 38 | 9 | 0.23 |
| rZ | ng/ml | 1.6E-3 | 8.2E-3 | 7.1E-3 | 1.7E-2 | 1.6E-2 | 2.8E-2 | 1.0E-9 | 1.0E-9 | 9.4E-2 | 1.1E-1 | 60 | 15 | 60 | 15 | 0.61 |
| rY | ng/ml | 6.4E-2 | 6.9E-2 | 5.4E-1 | 1.0E-1 | 2.7E0 | 1.2E-1 | 1.0E-9 | 1.0E-9 | 2.0E1 | 5.0E-1 | 60 | 15 | 60 | 15 | 0.54 |
| rX | ng/ml | 1.0E-9 | 1.0E-9 | 1.3E-1 | 3.2E-2 | 6.2E-1 | 7.8E-2 | 1.0E-9 | 1.0E-9 | 3.7E0 | 2.9E-1 | 60 | 15 | 60 | 15 | 0.59 |
| lK | pg/ml | 6.2E1 | 6.8E1 | 1.4E2 | 1.2E2 | 1.7E2 | 1.6E2 | 1.0E-9 | 2.3E0 | 7.0E2 | 5.0E2 | 66 | 17 | 66 | 17 | 0.45 |
| lL | pg/ml | 1.5E3 | 1.8E3 | 3.1E3 | 2.4E3 | 5.6E3 | 2.0E3 | 7.5E1 | 5.3E2 | 4.2E4 | 7.7E3 | 67 | 17 | 67 | 17 | 0.52 |
| lM | pg/ml | 1.1E3 | 2.6E3 | 3.8E3 | 1.0E4 | 6.8E3 | 1.8E4 | 9.5E0 | 2.6E2 | 4.2E4 | 6.7E4 | 67 | 17 | 67 | 17 | 0.63 |
| lN | pg/ml | 1.0E-9 | 1.0E-9 | 3.1E0 | 4.0E0 | 7.3E0 | 6.5E0 | 1.0E-9 | 1.0E-9 | 4.3E1 | 2.1E1 | 67 | 17 | 67 | 17 | 0.57 |
| lO | pg/ml | 1.0E-9 | 1.0E-9 | 2.4E0 | 8.1E0 | 1.6E1 | 3.3E1 | 1.0E-9 | 1.0E-9 | 1.3E2 | 1.4E2 | 66 | 17 | 66 | 17 | 0.52 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|-----|--------|-----|------|
| | | NonDis | Dis | NonDis | Dis | NonDis | Dis | NonDis | Dis | NonDis | Dis | NonDis | Dis | NonDis | Dis | |
| zA | ng/ml | 2.1E7 | 2.1E7 | 2.2E7 | 1.9E7 | 6.5E6 | 4.8E6 | 1.0E7 | 1.1E7 | 3.6E7 | 2.5E7 | 40 | 10 | 40 | 10 | 0.40 |
| rW | ng/ml | 1.1E-2 | 1.5E-2 | 3.5E-2 | 1.8E-2 | 6.5E-2 | 8.0E-3 | 1.0E-9 | 7.4E-3 | 3.2E-1 | 3.3E-2 | 37 | 9 | 37 | 9 | 0.60 |
| rV | ng/ml | 1.0E-9 | 6.7E-3 | 9.3E-3 | 2.9E-2 | 4.6E-2 | 5.0E-2 | 1.0E-9 | 1.0E-9 | 2.8E-1 | 1.5E-1 | 37 | 9 | 37 | 9 | 0.71 |
| rU | ng/ml | 7.1E-2 | 1.3E-1 | 1.6E-1 | 1.6E-1 | 4.4E-1 | 1.3E-1 | 1.0E-9 | 1.0E-9 | 2.7E0 | 4.0E-1 | 37 | 9 | 37 | 9 | 0.66 |
| rT | ng/ml | 6.1E0 | 6.8E0 | 6.8E0 | 7.6E0 | 4.7E0 | 5.6E0 | 6.5E-1 | 1.0E0 | 2.1E1 | 1.8E1 | 37 | 9 | 37 | 9 | 0.54 |
| rS | ng/ml | 3.5E0 | 9.0E0 | 5.3E0 | 2.4E1 | 5.1E0 | 2.5E1 | 1.1E0 | 2.0E0 | 2.5E1 | 7.0E1 | 37 | 9 | 37 | 9 | 0.80 |
| sC | pg/mL | 5.4E3 | 7.9E3 | 1.1E4 | 1.0E4 | 1.5E4 | 7.7E3 | 1.7E3 | 3.4E3 | 7.4E4 | 2.8E4 | 38 | 9 | 38 | 9 | 0.62 |
| yL | pg/ml | 2.9E1 | 4.1E1 | 3.4E1 | 2.0E2 | 2.7E1 | 4.6E2 | 5.6E0 | 1.2E1 | 1.8E2 | 1.4E3 | 40 | 9 | 40 | 9 | 0.67 |
| rP | ng/ml | 1.2E2 | 2.1E2 | 2.1E2 | 2.8E2 | 2.1E2 | 2.2E2 | 1.0E-9 | 1.2E1 | 8.0E2 | 5.0E2 | 37 | 9 | 37 | 9 | 0.59 |
| rQ | ng/ml | 1.0E-9 | 1.0E-9 | 4.1E0 | 1.9E1 | 2.1E1 | 5.6E1 | 1.0E-9 | 1.0E-9 | 1.2E2 | 1.7E2 | 37 | 9 | 37 | 9 | 0.53 |
| rO | ng/ml | 2.0E-2 | 4.0E-3 | 4.1E-2 | 3.0E-2 | 7.2E-2 | 4.8E-2 | 1.0E-9 | 1.0E-9 | 4.0E-1 | 1.2E-1 | 37 | 9 | 37 | 9 | 0.41 |
| rR | ng/ml | 3.9E0 | 1.0E-9 | 1.0E1 | 1.8E1 | 1.9E1 | 3.2E1 | 1.0E-9 | 1.0E-9 | 7.9E1 | 9.5E1 | 37 | 9 | 37 | 9 | 0.51 |
| rN | ng/ml | 6.2E-1 | 1.3E0 | 7.5E-1 | 2.8E0 | 5.2E-1 | 4.0E0 | 5.1E-2 | 3.4E-1 | 2.3E0 | 1.3E1 | 37 | 9 | 37 | 9 | 0.74 |
| qO | pg/ml | 8.0E3 | 1.0E4 | 1.2E4 | 1.2E4 | 1.1E4 | 9.3E3 | 7.4E2 | 5.4E3 | 4.8E4 | 3.5E4 | 38 | 9 | 38 | 9 | 0.56 |
| qP | pg/ml | 3.3E2 | 3.6E2 | 4.2E2 | 3.9E2 | 3.0E2 | 2.0E2 | 7.0E1 | 1.5E2 | 1.5E3 | 7.8E2 | 38 | 9 | 38 | 9 | 0.52 |
| qQ | pg/ml | 1.5E1 | 3.3E1 | 2.4E1 | 2.6E1 | 6.0E1 | 1.8E1 | 1.0E-9 | 1.0E-9 | 2.8E2 | 4.3E1 | 38 | 9 | 38 | 9 | 0.72 |
| nW | pg/ml | 1.1E5 | 1.1E5 | 1.1E5 | 1.1E5 | 2.6E4 | 3.4E4 | 5.8E4 | 3.6E4 | 1.8E5 | 1.7E5 | 82 | 22 | 82 | 22 | 0.51 |
| nY | pg/ml | 2.2E3 | 3.4E3 | 2.6E3 | 3.2E3 | 1.6E3 | 1.8E3 | 5.1E2 | 6.3E2 | 1.0E4 | 8.1E3 | 82 | 22 | 82 | 22 | 0.61 |
| oO | pg/ml | 9.1E4 | 9.8E4 | 1.1E5 | 1.2E5 | 6.3E4 | 1.0E5 | 2.0E4 | 3.3E3 | 3.0E5 | 4.0E5 | 45 | 12 | 45 | 12 | 0.52 |
| oP | pg/ml | 1.3E5 | 1.6E5 | 1.5E5 | 2.0E5 | 7.6E4 | 1.5E5 | 4.2E4 | 2.4E4 | 4.1E5 | 5.7E5 | 45 | 12 | 45 | 12 | 0.62 |
| oQ | pg/ml | 3.1E3 | 4.2E3 | 3.8E3 | 6.8E3 | 3.2E3 | 8.2E3 | 7.7E2 | 9.1E2 | 2.1E4 | 3.2E4 | 45 | 12 | 45 | 12 | 0.67 |
| oE | pg/ml | 2.1E2 | 5.6E2 | 4.7E2 | 8.9E2 | 5.6E2 | 9.9E2 | 1.0E-9 | 1.0E-9 | 2.6E3 | 3.4E3 | 82 | 22 | 82 | 22 | 0.61 |
| oF | pg/ml | 1.3E4 | 3.1E4 | 2.5E4 | 4.9E4 | 3.6E4 | 4.9E4 | 4.3E2 | 6.5E2 | 2.5E5 | 1.7E5 | 82 | 22 | 82 | 22 | 0.68 |
| oH | pg/ml | 3.9E1 | 2.3E1 | 8.7E1 | 5.5E1 | 1.3E2 | 7.9E1 | 4.4E0 | 4.3E-1 | 8.6E2 | 3.1E2 | 82 | 22 | 82 | 22 | 0.40 |
| oK | pg/ml | 8.6E2 | 1.3E3 | 1.6E3 | 1.5E3 | 1.9E3 | 1.4E3 | 8.8E1 | 1.8E2 | 1.2E4 | 5.9E3 | 82 | 22 | 82 | 22 | 0.53 |
| oN | pg/ml | 5.5E2 | 5.8E2 | 1.1E3 | 7.5E2 | 2.2E3 | 3.9E2 | 1.1E2 | 3.3E2 | 1.8E4 | 1.8E3 | 82 | 22 | 82 | 22 | 0.60 |
| uL | ng/ml | 3.5E1 | 4.3E1 | 4.7E1 | 4.4E1 | 4.7E1 | 2.5E1 | 1.5E1 | 1.4E1 | 2.9E2 | 8.0E1 | 38 | 9 | 38 | 9 | 0.53 |
| uO | ng/ml | 4.1E-1 | 4.8E-1 | 8.6E-1 | 8.6E-1 | 1.6E0 | 8.5E-1 | 1.0E-9 | 1.0E-9 | 9.3E0 | 2.1E0 | 38 | 9 | 38 | 9 | 0.56 |
| uM | ng/ml | 5.4E-1 | 6.2E-1 | 9.8E-1 | 7.6E-1 | 2.1E0 | 7.6E-1 | 1.0E-9 | 1.0E-9 | 1.3E1 | 2.5E0 | 38 | 9 | 38 | 9 | 0.51 |
| uI | ng/ml | 7.2E-2 | 5.1E-2 | 1.1E-1 | 1.0E-1 | 1.2E-1 | 1.2E-1 | 1.6E-2 | 1.5E-2 | 5.8E-1 | 3.8E-1 | 37 | 9 | 37 | 9 | 0.44 |
| uN | ng/ml | 1.5E1 | 1.7E1 | 1.7E1 | 2.0E1 | 6.5E0 | 9.7E0 | 8.0E0 | 1.0E1 | 3.6E1 | 4.1E1 | 38 | 9 | 38 | 9 | 0.63 |
| uG | ng/ml | 1.8E1 | 1.9E1 | 2.2E1 | 3.4E1 | 1.5E1 | 3.9E1 | 1.2E0 | 6.5E0 | 7.9E1 | 1.3E2 | 38 | 9 | 38 | 9 | 0.58 |
| uR | ng/ml | 2.2E0 | 1.7E0 | 2.9E0 | 2.3E0 | 2.4E0 | 1.8E0 | 7.5E-1 | 9.1E-1 | 1.3E1 | 6.6E0 | 40 | 9 | 40 | 9 | 0.39 |
| uP | ng/ml | 2.1E0 | 2.7E0 | 2.4E0 | 3.1E0 | 9.7E-1 | 1.7E0 | 1.2E0 | 9.3E-1 | 6.0E0 | 6.1E0 | 40 | 9 | 40 | 9 | 0.66 |
| uV | ng/ml | 1.7E-3 | 1.3E-3 | 1.6E-2 | 8.0E-3 | 3.5E-2 | 1.4E-2 | 1.0E-9 | 1.0E-9 | 2.0E-1 | 4.3E-2 | 40 | 9 | 40 | 9 | 0.46 |
| uT | ng/ml | 6.4E1 | 1.2E2 | 9.2E1 | 1.6E2 | 8.8E1 | 1.1E2 | 1.3E1 | 5.7E1 | 4.5E2 | 4.1E2 | 40 | 9 | 40 | 9 | 0.78 |
| uU | ng/ml | 1.7E0 | 1.7E0 | 1.8E0 | 3.8E0 | 1.1E0 | 6.0E0 | 6.0E-1 | 5.4E-1 | 6.0E0 | 2.0E1 | 40 | 9 | 40 | 9 | 0.56 |
| uW | ng/ml | 7.8E0 | 7.9E0 | 8.1E0 | 8.1E0 | 2.4E0 | 1.8E0 | 4.4E0 | 6.5E0 | 1.6E1 | 1.1E1 | 38 | 9 | 38 | 9 | 0.53 |
| vB | ng/ml | 3.0E0 | 3.2E0 | 3.6E0 | 2.9E0 | 2.4E0 | 8.4E-1 | 5.9E-1 | 1.3E0 | 1.0E1 | 3.8E0 | 38 | 9 | 38 | 9 | 0.47 |
| vC | ng/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 38 | 9 | 38 | 9 | 0.50 |
| uY | ng/ml | 6.1E-1 | 8.7E-1 | 8.9E-1 | 1.4E0 | 8.7E-1 | 1.3E0 | 6.8E-2 | 3.1E-1 | 3.8E0 | 4.4E0 | 38 | 9 | 38 | 9 | 0.67 |
| uZ | ng/ml | 5.8E-1 | 5.3E-1 | 7.6E-1 | 6.5E-1 | 8.5E-1 | 5.1E-1 | 1.0E-1 | 1.7E-1 | 4.9E0 | 1.9E0 | 38 | 9 | 38 | 9 | 0.47 |
| uX | ng/ml | 8.1E0 | 9.1E0 | 1.0E1 | 2.3E1 | 6.6E0 | 2.3E1 | 3.6E0 | 5.6E0 | 4.0E1 | 6.5E1 | 38 | 9 | 38 | 9 | 0.68 |
| vA | ng/ml | 6.3E-2 | 6.6E-2 | 7.3E-2 | 1.2E-1 | 4.3E-2 | 1.3E-1 | 2.9E-2 | 2.5E-2 | 2.7E-1 | 4.2E-1 | 38 | 9 | 38 | 9 | 0.56 |
| vH | ng/ml | 1.2E-1 | 1.1E-1 | 1.5E-1 | 3.4E-1 | 1.3E-1 | 6.0E-1 | 2.0E-2 | 4.7E-2 | 6.6E-1 | 1.9E0 | 38 | 9 | 38 | 9 | 0.49 |
| vI | ng/ml | 2.1E0 | 3.5E0 | 2.4E0 | 3.9E0 | 1.9E0 | 2.7E0 | 6.3E-3 | 1.2E0 | 1.0E1 | 1.0E1 | 38 | 9 | 38 | 9 | 0.74 |
| vP | ng/ml | 3.6E2 | 2.9E2 | 4.7E2 | 4.3E2 | 4.5E2 | 3.2E2 | 6.7E1 | 1.5E2 | 2.4E3 | 1.1E3 | 40 | 9 | 40 | 9 | 0.54 |
| vT | ng/ml | 7.1E1 | 8.2E1 | 8.1E1 | 9.9E1 | 3.6E1 | 5.2E1 | 4.1E1 | 4.6E1 | 2.4E2 | 1.8E2 | 40 | 9 | 40 | 9 | 0.56 |
| vU | ng/ml | 1.0E-9 | 1.0E-9 | 2.3E1 | 1.9E1 | 3.5E1 | 3.7E1 | 1.0E-9 | 1.0E-9 | 1.2E2 | 1.1E2 | 40 | 9 | 40 | 9 | 0.46 |
| vQ | ng/ml | 4.0E2 | 4.5E2 | 4.1E2 | 4.0E2 | 1.6E2 | 1.8E2 | 7.2E1 | 1.9E2 | 8.4E2 | 6.5E2 | 40 | 9 | 40 | 9 | 0.48 |
| vO | ng/ml | 1.7E3 | 1.7E3 | 1.8E3 | 1.8E3 | 4.4E2 | 6.0E2 | 1.1E3 | 1.0E3 | 2.9E3 | 3.2E3 | 40 | 9 | 40 | 9 | 0.49 |
| vS | ng/ml | 1.3E3 | 1.3E3 | 1.2E3 | 1.3E3 | 4.5E2 | 5.7E2 | 1.0E-9 | 1.0E-9 | 2.1E3 | 1.9E3 | 40 | 9 | 40 | 9 | 0.56 |
| vV | ng/ml | 8.7E2 | 8.2E2 | 1.0E3 | 9.1E2 | 9.9E2 | 5.3E2 | 1.0E2 | 2.3E2 | 4.6E3 | 1.7E3 | 40 | 9 | 40 | 9 | 0.53 |

| Code | Units | Median | | Average | | Standard Deviation | | Minimum | | Maximum | | Number of Samples | | Number of Patients | | AUC |
|------|-------|--------|-----|---------|-----|--------|------|---------|------|---------|------|--------|-----|--------|-----|-----|
| | | NonDis | Dis | NonDis | Dis | NonDis | Dis | NonDis | Dis | NonDis | Dis | NonDis | Dis | NonDis | Dis | |
| vW | ng/ml | 1.1E2 | 2.4E2 | 1.4E2 | 2.6E2 | 8.5E1 | 2.2E2 | 4.3E1 | 6.0E1 | 4.5E2 | 7.7E2 | 40 | 9 | 40 | 9 | 0.68 |
| pF | pg/ml | 6.0E-1 | 5.6E-1 | 1.9E0 | 8.8E-1 | 9.6E0 | 7.7E-1 | 1.0E-9 | 1.8E-1 | 8.7E1 | 3.5E0 | 82 | 22 | 82 | 22 | 0.54 |

Figure 31.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.2E1 | 9.8E1 | 8.1E1 | 1.1E2 | 5.0E1 | 8.3E1 | 8.0E0 | 7.0E0 | 2.4E2 | 4.8E2 | 178 | 120 | 178 | 120 | 0.61 |
| Ad | ug/mL | 3.8E-2 | 5.3E-2 | 6.7E-2 | 1.9E-1 | 8.2E-2 | 9.3E-1 | 6.8E-4 | 7.8E-4 | 3.7E-1 | 8.5E0 | 91 | 83 | 91 | 83 | 0.59 |
| Af | ng/mL | 1.3E0 | 1.2E0 | 1.2E1 | 7.6E0 | 5.7E1 | 2.1E1 | 1.7E-3 | 1.7E-3 | 5.3E2 | 1.9E2 | 91 | 83 | 91 | 83 | 0.51 |
| Aj | ug/mL | 1.8E0 | 4.8E-1 | 2.6E0 | 2.0E0 | 2.5E0 | 2.4E0 | 1.5E-3 | 1.3E-3 | 6.1E0 | 6.1E0 | 91 | 83 | 91 | 83 | 0.42 |
| Al | mg/mL | 8.5E-5 | 8.8E-5 | 2.6E-4 | 2.9E-4 | 4.6E-4 | 4.3E-4 | 4.3E-6 | 6.6E-6 | 1.8E-3 | 1.8E-3 | 91 | 83 | 91 | 83 | 0.54 |
| An | U/mL | 5.0E1 | 7.8E1 | 1.9E2 | 3.3E2 | 6.2E2 | 9.2E2 | 2.8E-1 | 6.4E-1 | 5.5E3 | 7.8E3 | 91 | 83 | 91 | 83 | 0.60 |
| Ao | pg/mL | 8.7E1 | 1.0E2 | 8.3E2 | 2.4E2 | 4.8E3 | 5.3E2 | 1.5E0 | 4.1E0 | 3.9E4 | 4.5E3 | 91 | 83 | 91 | 83 | 0.58 |
| Ap | ng/mL | 3.2E1 | 3.8E1 | 4.8E1 | 5.3E1 | 5.8E1 | 5.5E1 | 2.0E0 | 2.4E0 | 3.3E2 | 2.9E2 | 91 | 83 | 91 | 83 | 0.55 |
| Ar | ng/mL | 4.9E-1 | 1.3E0 | 1.5E0 | 4.9E0 | 3.1E0 | 1.0E1 | 3.4E-3 | 3.4E-3 | 2.0E1 | 5.1E1 | 91 | 83 | 91 | 83 | 0.65 |
| As | ng/mL | 8.0E-3 | 1.0E-2 | 1.1E-2 | 2.8E-2 | 1.6E-2 | 1.4E-1 | 1.7E-3 | 1.7E-3 | 9.8E-2 | 1.2E0 | 91 | 83 | 91 | 83 | 0.54 |
| Aw | pg/mL | 1.6E1 | 1.7E1 | 1.7E1 | 1.8E1 | 5.5E0 | 6.9E0 | 6.8E0 | 2.9E-2 | 4.2E1 | 5.1E1 | 91 | 83 | 91 | 83 | 0.52 |
| Ax | ng/mL | 1.3E0 | 5.5E0 | 7.3E0 | 5.4E1 | 1.6E1 | 1.6E2 | 1.3E-2 | 1.0E-4 | 1.1E2 | 8.5E2 | 91 | 83 | 91 | 83 | 0.62 |
| Ba | ng/mL | 7.7E1 | 1.3E2 | 4.5E2 | 9.8E2 | 1.3E3 | 2.3E3 | 1.9E0 | 1.1E0 | 8.1E3 | 1.5E4 | 91 | 83 | 91 | 83 | 0.60 |
| Bb | ng/mL | 3.7E0 | 6.0E0 | 6.0E0 | 9.0E0 | 8.0E0 | 9.6E0 | 4.1E-3 | 4.1E-3 | 4.9E1 | 4.8E1 | 91 | 83 | 91 | 83 | 0.61 |
| Bc | ng/mL | 2.7E1 | 6.0E1 | 1.1E2 | 1.6E2 | 2.2E2 | 2.5E2 | 4.9E-1 | 1.0E0 | 1.0E3 | 1.2E3 | 91 | 83 | 91 | 83 | 0.61 |
| Bg | ng/mL | 1.0E-1 | 1.3E-1 | 2.9E0 | 9.8E0 | 1.0E1 | 4.8E1 | 5.3E-4 | 5.3E-4 | 7.7E1 | 4.0E2 | 91 | 83 | 91 | 83 | 0.54 |
| Bn | ng/mL | 5.6E-2 | 5.6E-2 | 1.0E0 | 2.0E0 | 1.9E0 | 6.6E0 | 5.6E-2 | 5.6E-2 | 7.5E0 | 5.8E1 | 91 | 83 | 91 | 83 | 0.55 |
| Bo | ng/mL | 1.3E1 | 1.4E1 | 1.4E1 | 1.7E1 | 1.0E1 | 1.2E1 | 1.6E-2 | 1.6E-2 | 4.8E1 | 5.3E1 | 91 | 83 | 91 | 83 | 0.56 |
| Ch | uIU/mL | 1.0E0 | 1.0E0 | 3.7E1 | 3.1E1 | 2.0E2 | 1.4E2 | 3.4E-3 | 3.4E-3 | 1.8E3 | 1.2E3 | 91 | 83 | 91 | 83 | 0.49 |
| Co | pg/mL | 4.6E1 | 5.3E1 | 1.4E2 | 3.2E2 | 4.5E2 | 1.8E3 | 1.5E-1 | 1.5E-1 | 3.7E3 | 1.7E4 | 91 | 83 | 91 | 83 | 0.54 |
| Cp | ng/mL | 2.1E1 | 2.2E1 | 2.6E1 | 4.6E1 | 2.0E1 | 1.4E2 | 6.0E-1 | 2.5E0 | 1.3E2 | 1.3E3 | 91 | 83 | 91 | 83 | 0.58 |
| Cq | ng/mL | 2.2E-2 | 3.4E-2 | 7.6E-2 | 7.6E-1 | 2.3E-1 | 5.4E0 | 8.0E-4 | 8.0E-4 | 2.0E0 | 4.9E1 | 91 | 83 | 91 | 83 | 0.58 |
| Cs | ng/mL | 3.8E1 | 1.3E2 | 2.0E2 | 8.1E2 | 3.9E2 | 2.4E3 | 1.0E0 | 8.3E-1 | 1.9E3 | 1.8E4 | 91 | 83 | 91 | 83 | 0.64 |
| Ct | ng/mL | 6.0E-1 | 2.3E-1 | 3.4E1 | 5.0E1 | 1.1E2 | 1.3E2 | 1.3E-2 | 1.1E-4 | 4.7E2 | 6.2E2 | 91 | 83 | 91 | 83 | 0.46 |
| Cu | ng/mL | 2.2E-1 | 3.2E-1 | 4.4E-1 | 1.7E0 | 9.6E-1 | 7.5E0 | 1.9E-2 | 1.7E-2 | 9.0E0 | 6.6E1 | 91 | 83 | 91 | 83 | 0.61 |
| Cv | ng/mL | 4.1E0 | 8.5E0 | 2.6E1 | 3.3E1 | 7.4E1 | 6.9E1 | 2.0E-2 | 2.4E-2 | 5.3E2 | 4.7E2 | 91 | 83 | 91 | 83 | 0.56 |
| Cw | mIU/mL | 2.8E-2 | 4.3E-2 | 4.1E-2 | 1.3E-1 | 3.3E-2 | 7.4E-1 | 8.9E-4 | 2.8E-3 | 1.5E-1 | 6.8E0 | 91 | 83 | 91 | 83 | 0.54 |
| Cx | ng/mL | 9.5E-1 | 6.5E-1 | 5.3E1 | 4.7E1 | 1.0E2 | 9.6E1 | 9.3E-5 | 9.3E-5 | 3.9E2 | 3.9E2 | 91 | 83 | 91 | 83 | 0.50 |
| Db | ug/mL | 7.5E0 | 7.3E0 | 9.0E0 | 8.5E0 | 7.1E0 | 8.1E0 | 4.5E-1 | 8.1E-1 | 4.3E1 | 5.9E1 | 91 | 83 | 91 | 83 | 0.47 |
| Dc | nmol/L | 1.9E-2 | 2.4E-2 | 4.1E-2 | 2.9E-1 | 6.9E-2 | 1.6E0 | 5.2E-6 | 1.1E-3 | 4.8E-1 | 1.4E1 | 91 | 83 | 91 | 83 | 0.59 |
| Dd | ug/mL | 6.2E-2 | 1.2E-1 | 1.5E-1 | 2.7E-1 | 2.5E-1 | 4.7E-1 | 4.8E-4 | 1.3E-3 | 1.6E0 | 3.6E0 | 91 | 83 | 91 | 83 | 0.59 |
| De | ng/mL | 3.4E-3 | 3.4E-3 | 6.4E-2 | 1.1E-1 | 1.4E-1 | 1.8E-1 | 3.4E-3 | 3.4E-3 | 9.2E-1 | 1.1E0 | 91 | 83 | 91 | 83 | 0.58 |
| Dg | ng/mL | 3.1E1 | 3.9E1 | 4.4E1 | 5.3E1 | 3.9E1 | 4.2E1 | 1.0E0 | 7.1E-1 | 1.8E2 | 1.9E2 | 91 | 83 | 91 | 83 | 0.57 |
| Di | pg/mL | 1.8E0 | 2.7E0 | 2.2E0 | 2.8E0 | 2.4E0 | 2.0E0 | 1.8E-1 | 1.8E-1 | 1.3E1 | 9.3E0 | 91 | 83 | 91 | 83 | 0.60 |
| Dk | uIU/mL | 1.2E-2 | 1.7E-2 | 6.1E-2 | 6.3E-2 | 2.0E-1 | 1.5E-1 | 1.1E-4 | 1.1E-4 | 1.6E0 | 9.8E-1 | 91 | 83 | 91 | 83 | 0.56 |
| Dl | ng/mL | 1.8E2 | 2.3E2 | 2.6E2 | 3.5E2 | 2.3E2 | 3.3E2 | 8.9E0 | 4.4E0 | 1.1E3 | 1.6E3 | 91 | 83 | 91 | 83 | 0.56 |
| Dp | ng/ml | 2.4E0 | 1.8E0 | 5.3E0 | 5.5E0 | 7.3E0 | 1.0E1 | 1.5E-2 | 3.7E-3 | 3.5E1 | 5.6E1 | 65 | 70 | 65 | 70 | 0.44 |
| Dr | pg/ml | 2.1E1 | 3.0E1 | 4.4E1 | 3.9E2 | 5.8E1 | 1.8E3 | 7.5E-1 | 7.5E-1 | 2.5E2 | 1.0E4 | 39 | 33 | 39 | 33 | 0.54 |
| Du | pg/ml | 7.9E1 | 2.2E2 | 5.7E2 | 2.0E3 | 1.1E3 | 5.5E3 | 1.2E0 | 1.2E0 | 5.4E3 | 2.4E4 | 28 | 29 | 28 | 29 | 0.58 |
| Dw | ng/ml | 9.2E-3 | 2.0E-2 | 4.4E-2 | 5.2E-2 | 6.5E-2 | 6.9E-2 | 9.2E-3 | 9.2E-3 | 1.9E-1 | 1.9E-1 | 8 | 10 | 8 | 10 | 0.56 |
| Ef | ng/ml | 8.7E-2 | 1.2E-1 | 7.9E-1 | 9.9E-1 | 1.7E0 | 2.3E0 | 1.6E-3 | 5.7E-4 | 1.0E1 | 9.9E0 | 72 | 75 | 72 | 75 | 0.52 |
| Wm | % | 8.5E-2 | 8.5E-2 | 4.1E0 | 4.1E1 | 2.2E1 | 1.8E2 | 5.4E-2 | 8.5E-2 | 2.0E2 | 1.0E3 | 83 | 76 | 83 | 76 | 0.52 |
| Ed | pg/ml | 5.2E-1 | 1.5E1 | 2.3E1 | 5.0E1 | 3.6E1 | 9.0E1 | 5.2E-1 | 5.2E-1 | 1.9E2 | 5.0E2 | 65 | 70 | 65 | 70 | 0.58 |
| Eo | ng/ml | 6.1E0 | 3.0E0 | 6.6E0 | 7.3E0 | 5.6E0 | 1.2E1 | 3.6E-1 | 3.6E-1 | 1.6E1 | 4.0E1 | 8 | 10 | 8 | 10 | 0.44 |
| Yf | ng/mL | 1.6E1 | 1.5E1 | 4.2E1 | 6.5E1 | 5.8E1 | 1.3E2 | 2.9E-1 | 2.9E-1 | 2.4E2 | 5.9E2 | 31 | 28 | 31 | 28 | 0.46 |
| Tj | pg/mL | 3.7E-1 | 3.7E-1 | 2.6E1 | 5.1E1 | 1.1E2 | 2.7E2 | 3.7E-1 | 3.7E-1 | 8.7E2 | 2.3E3 | 73 | 72 | 73 | 72 | 0.52 |
| Po | pg/ml | 5.7E-2 | 1.9E0 | 8.2E0 | 1.4E1 | 2.8E1 | 3.5E1 | 8.0E-3 | 2.6E-2 | 2.7E2 | 2.2E2 | 217 | 137 | 217 | 137 | 0.60 |
| Ti | ug/mL | 2.9E0 | 5.3E0 | 4.1E0 | 5.8E0 | 3.8E0 | 4.6E0 | 1.2E-1 | 4.0E-1 | 1.6E1 | 1.8E1 | 43 | 41 | 43 | 41 | 0.61 |
| Em | ng/ml | 2.9E-3 | 2.2E-2 | 4.4E-2 | 1.0E-1 | 8.7E-2 | 3.0E-1 | 8.4E-4 | 8.4E-4 | 5.0E-1 | 1.9E0 | 48 | 44 | 48 | 44 | 0.56 |

| Code | Units | Median | | Average | | Standard Deviation | | Minimum | | Maximum | | Number of Samples | | Number of Patients | | AUC |
|------|-------|--------|-----|---------|-----|--------|--------|---------|---------|---------|---------|--------|-----|--------|-----|-----|
| | | NonDis | Dis | NonDis | Dis | NonDis | Dis | NonDis | Dis | NonDis | Dis | NonDis | Dis | NonDis | Dis | |
| Et | ng/ml | 1.1E3 | 2.1E3 | 1.4E3 | 2.2E3 | 1.1E3 | 1.2E3 | 7.5E1 | 7.9E1 | 4.8E3 | 5.0E3 | 216 | 137 | 216 | 137 | 0.70 |
| Eq | pg/ml | 2.4E2 | 1.4E2 | 3.5E2 | 3.4E2 | 4.0E2 | 4.0E2 | 1.0E0 | 1.0E0 | 1.5E3 | 1.4E3 | 28 | 29 | 28 | 29 | 0.47 |
| Ew | U/ml | 1.9E0 | 2.1E0 | 2.7E0 | 2.4E0 | 2.6E0 | 1.2E0 | 1.1E0 | 1.3E0 | 8.8E0 | 5.0E0 | 8 | 10 | 8 | 10 | 0.59 |
| Th | ug/mL | 1.2E0 | 1.3E0 | 1.6E0 | 1.9E0 | 1.3E0 | 1.8E0 | 1.3E-1 | 2.6E-3 | 5.4E0 | 7.5E0 | 43 | 41 | 43 | 41 | 0.52 |
| Fa | ng/ml | 4.0E1 | 5.7E1 | 5.2E1 | 1.8E2 | 5.3E1 | 5.3E2 | 2.6E-1 | 6.0E-1 | 2.5E2 | 3.7E3 | 63 | 68 | 63 | 68 | 0.60 |
| Ez | ng/ml | 3.4E0 | 4.1E0 | 1.7E1 | 1.4E1 | 3.2E1 | 2.9E1 | 1.3E-2 | 1.3E-2 | 1.6E2 | 2.0E2 | 65 | 70 | 65 | 70 | 0.53 |
| Fb | ng/ml | 2.3E1 | 2.7E1 | 2.1E1 | 2.5E1 | 1.2E1 | 1.1E1 | 9.4E-1 | 6.6E-1 | 4.3E1 | 4.3E1 | 63 | 69 | 63 | 69 | 0.60 |
| Ex | ng/ml | 4.5E-2 | 1.1E-1 | 1.7E-1 | 2.8E-1 | 3.6E-1 | 6.1E-1 | 3.5E-5 | 1.7E-4 | 2.2E0 | 4.1E0 | 50 | 55 | 50 | 55 | 0.64 |
| Fc | pg/ml | 2.2E-1 | 2.2E-1 | 1.9E1 | 2.8E1 | 8.3E1 | 9.1E1 | 2.2E-1 | 2.2E-1 | 4.5E2 | 3.9E2 | 29 | 29 | 29 | 29 | 0.56 |
| Fd | pg/ml | 8.0E1 | 1.6E2 | 4.0E2 | 2.3E3 | 7.9E2 | 6.2E3 | 4.5E-1 | 9.8E-1 | 2.9E3 | 2.5E4 | 29 | 29 | 29 | 29 | 0.55 |
| Fi | pg/ml | 2.5E-1 | 2.5E-1 | 7.4E1 | 1.1E2 | 2.1E2 | 3.5E2 | 2.5E-1 | 2.5E-1 | 8.5E2 | 1.8E3 | 29 | 29 | 29 | 29 | 0.54 |
| Fn | ng/ml | 2.1E-1 | 2.1E-1 | 4.2E0 | 3.6E0 | 8.3E0 | 5.8E0 | 1.1E-14 | 1.1E-14 | 3.7E1 | 2.7E1 | 65 | 70 | 65 | 70 | 0.53 |
| Fp | ng/ml | 9.3E0 | 2.0E1 | 1.9E1 | 3.1E1 | 2.3E1 | 3.2E1 | 6.0E-3 | 2.8E-1 | 1.1E2 | 1.3E2 | 217 | 139 | 217 | 139 | 0.62 |
| Fr | ng/ml | 2.8E4 | 6.2E4 | 1.1E5 | 1.7E5 | 1.8E5 | 2.3E5 | 1.9E2 | 1.3E3 | 8.4E5 | 8.4E5 | 222 | 141 | 222 | 141 | 0.63 |
| Fw | pg/ml | 8.5E-1 | 5.1E0 | 5.2E1 | 4.2E1 | 3.5E2 | 1.2E2 | 1.2E-1 | 1.2E-1 | 3.0E3 | 9.1E2 | 74 | 75 | 74 | 75 | 0.60 |
| Fy | ng/ml | 2.9E1 | 4.9E1 | 4.7E1 | 9.5E1 | 4.9E1 | 1.3E2 | 1.2E-1 | 1.2E-1 | 2.2E2 | 6.5E2 | 65 | 67 | 65 | 67 | 0.60 |
| Gh | pg/ml | 5.8E-1 | 3.9E0 | 3.0E1 | 1.3E1 | 7.1E1 | 2.8E1 | 2.9E-2 | 2.9E-2 | 3.2E2 | 1.4E2 | 29 | 29 | 29 | 29 | 0.54 |
| Gb | % | 4.1E1 | 4.2E1 | 4.7E1 | 5.8E1 | 4.4E1 | 6.2E1 | 3.7E0 | 2.2E0 | 2.3E2 | 3.0E2 | 29 | 29 | 29 | 29 | 0.55 |
| Gc | ng/ml | 7.1E1 | 1.3E2 | 1.2E2 | 2.3E2 | 1.4E2 | 2.6E2 | 6.4E0 | 6.9E0 | 7.3E2 | 1.2E3 | 39 | 33 | 39 | 33 | 0.64 |
| Gd | ng/ml | 3.1E1 | 3.4E1 | 3.3E1 | 3.4E1 | 1.8E1 | 1.9E1 | 6.3E0 | 3.0E0 | 8.1E1 | 8.0E1 | 46 | 36 | 46 | 36 | 0.51 |
| Gn | U/ml | 2.1E-1 | 2.2E-1 | 1.6E0 | 4.0E0 | 5.0E0 | 2.0E1 | 5.6E-3 | 5.6E-3 | 3.0E1 | 1.1E2 | 38 | 33 | 38 | 33 | 0.51 |
| Gl | pg/ml | 8.5E3 | 1.1E4 | 1.1E4 | 1.4E4 | 9.0E3 | 1.0E4 | 9.1E1 | 4.0E2 | 3.2E4 | 3.2E4 | 74 | 75 | 74 | 75 | 0.58 |
| Gp | U/ml | 1.5E0 | 9.9E-1 | 2.8E0 | 2.0E0 | 3.7E0 | 3.1E0 | 1.5E-2 | 1.5E-2 | 2.0E1 | 1.8E1 | 74 | 74 | 74 | 74 | 0.43 |
| Gz | ug/ml | 1.0E1 | 9.9E-1 | 6.5E0 | 4.5E0 | 6.0E0 | 5.4E0 | 6.2E-2 | 4.2E-2 | 2.5E1 | 1.9E1 | 42 | 52 | 42 | 52 | 0.41 |
| Ha | ng/ml | 1.9E0 | 2.5E0 | 5.4E0 | 1.2E1 | 1.4E1 | 2.4E1 | 1.7E-2 | 1.7E-2 | 1.0E2 | 1.0E2 | 63 | 70 | 63 | 70 | 0.57 |
| Nm | pg/ml | 1.1E4 | 1.7E4 | 2.5E4 | 5.8E4 | 5.8E4 | 1.3E5 | 1.0E-9 | 1.0E-9 | 7.8E5 | 9.6E5 | 218 | 139 | 218 | 139 | 0.61 |
| Nn | pg/ml | 1.3E2 | 2.8E2 | 1.2E3 | 5.7E3 | 5.1E3 | 3.0E4 | 1.0E-9 | 1.0E-9 | 6.0E4 | 3.1E5 | 218 | 139 | 218 | 139 | 0.61 |
| No | pg/ml | 1.2E1 | 2.1E1 | 2.8E1 | 6.0E1 | 5.9E1 | 1.3E2 | 1.0E-9 | 1.0E-9 | 5.6E2 | 9.1E2 | 218 | 139 | 218 | 139 | 0.63 |
| Nq | pg/ml | 1.7E0 | 3.0E0 | 1.9E1 | 3.1E1 | 6.6E1 | 1.0E2 | 1.0E-9 | 1.0E-9 | 6.2E2 | 6.7E2 | 218 | 139 | 218 | 139 | 0.55 |
| Nr | pg/ml | 9.7E-1 | 3.2E0 | 2.1E1 | 3.8E1 | 8.4E1 | 1.4E2 | 1.0E-9 | 1.0E-9 | 9.8E2 | 1.4E3 | 218 | 139 | 218 | 139 | 0.60 |
| Ns | pg/ml | 1.0E-9 | 1.0E-9 | 4.7E0 | 1.2E1 | 2.3E1 | 9.7E1 | 1.0E-9 | 1.0E-9 | 1.7E2 | 1.1E3 | 218 | 139 | 218 | 139 | 0.50 |
| Nt | pg/ml | 8.3E1 | 1.4E2 | 1.1E2 | 1.9E2 | 9.0E1 | 2.0E2 | 9.8E-1 | 1.5E1 | 5.9E2 | 1.7E3 | 218 | 139 | 218 | 139 | 0.69 |
| Nu | pg/ml | 8.2E0 | 4.6E1 | 4.2E1 | 8.1E1 | 7.9E1 | 1.1E2 | 1.0E-9 | 1.0E-9 | 4.2E2 | 6.3E2 | 218 | 139 | 218 | 139 | 0.66 |
| Lu | pg/ml | 1.0E4 | 1.0E4 | 1.6E4 | 1.5E4 | 3.3E4 | 4.8E4 | 7.7E2 | 5.2E2 | 3.9E5 | 5.6E5 | 218 | 139 | 218 | 139 | 0.47 |
| Lv | pg/ml | 1.0E-9 | 1.4E0 | 1.2E1 | 2.3E1 | 2.5E1 | 4.1E1 | 1.0E-9 | 1.0E-9 | 2.4E2 | 2.6E2 | 218 | 139 | 218 | 139 | 0.59 |
| Lw | pg/ml | 1.0E-9 | 1.0E-9 | 1.6E-1 | 2.8E0 | 2.2E0 | 1.7E1 | 1.0E-9 | 1.0E-9 | 3.2E1 | 1.8E2 | 218 | 139 | 218 | 139 | 0.53 |
| Lx | pg/ml | 1.0E-9 | 5.5E1 | 1.3E2 | 4.7E2 | 5.0E2 | 2.0E3 | 1.0E-9 | 1.0E-9 | 6.2E3 | 2.2E4 | 218 | 139 | 218 | 139 | 0.65 |
| Ly | pg/ml | 1.0E-9 | 1.0E-9 | 8.4E0 | 1.0E1 | 1.8E1 | 1.8E1 | 1.0E-9 | 1.0E-9 | 1.1E2 | 9.6E1 | 218 | 139 | 218 | 139 | 0.53 |
| Lz | pg/ml | 1.0E-9 | 1.0E-9 | 3.4E0 | 3.6E0 | 3.0E1 | 1.6E1 | 1.0E-9 | 1.0E-9 | 4.3E2 | 1.3E2 | 218 | 139 | 218 | 139 | 0.52 |
| Ma | pg/ml | 3.5E2 | 7.0E2 | 2.0E3 | 3.2E3 | 5.9E3 | 6.9E3 | 1.0E-9 | 1.0E-9 | 6.5E4 | 5.2E4 | 218 | 139 | 218 | 139 | 0.60 |
| Mb | pg/ml | 2.4E1 | 2.8E1 | 3.0E1 | 3.5E1 | 1.4E1 | 2.2E1 | 9.2E0 | 4.1E0 | 7.2E1 | 2.1E2 | 218 | 139 | 218 | 139 | 0.57 |
| Mc | pg/ml | 1.0E-9 | 1.0E-9 | 2.0E-2 | 1.1E-1 | 2.7E-1 | 1.1E0 | 1.0E-9 | 1.0E-9 | 4.0E0 | 1.3E1 | 218 | 139 | 218 | 139 | 0.50 |
| Md | pg/ml | 1.0E-9 | 1.0E-9 | 7.0E-1 | 6.1E-1 | 6.4E0 | 3.2E0 | 1.0E-9 | 1.0E-9 | 6.8E1 | 2.9E1 | 218 | 139 | 218 | 139 | 0.52 |
| Me | pg/ml | 3.2E1 | 3.1E1 | 3.1E1 | 3.2E1 | 1.6E1 | 3.4E1 | 1.0E-9 | 1.0E-9 | 1.2E2 | 3.2E2 | 218 | 139 | 218 | 139 | 0.48 |
| Mf | pg/ml | 1.0E-9 | 1.0E-9 | 3.6E-1 | 1.1E0 | 1.7E0 | 5.8E0 | 1.0E-9 | 1.0E-9 | 2.0E1 | 5.6E1 | 218 | 139 | 218 | 139 | 0.53 |
| Mg | pg/ml | 8.5E-1 | 1.6E0 | 5.9E0 | 8.7E0 | 1.1E1 | 1.9E1 | 1.0E-9 | 1.0E-9 | 8.8E1 | 1.5E2 | 218 | 139 | 218 | 139 | 0.54 |
| Mh | pg/ml | 1.0E-9 | 1.0E-9 | 1.3E0 | 1.0E0 | 9.0E0 | 4.2E0 | 1.0E-9 | 1.0E-9 | 1.1E2 | 4.2E1 | 218 | 139 | 218 | 139 | 0.55 |
| Mi | pg/ml | 1.0E-9 | 1.0E-9 | 8.9E-1 | 8.4E0 | 8.7E0 | 5.3E1 | 1.0E-9 | 1.0E-9 | 1.2E2 | 5.2E2 | 218 | 139 | 218 | 139 | 0.53 |
| Mj | pg/ml | 1.0E-9 | 1.0E-9 | 6.3E0 | 8.5E0 | 3.7E1 | 2.8E1 | 1.0E-9 | 1.0E-9 | 4.6E2 | 2.1E2 | 218 | 139 | 218 | 139 | 0.54 |
| Mk | pg/ml | 2.3E0 | 9.1E-1 | 1.4E1 | 2.0E1 | 7.2E1 | 1.2E2 | 1.0E-9 | 1.0E-9 | 1.0E3 | 1.3E3 | 218 | 139 | 218 | 139 | 0.48 |
| Ml | pg/ml | 1.0E-9 | 1.0E-9 | 1.2E1 | 1.0E1 | 1.4E2 | 5.4E1 | 1.0E-9 | 1.0E-9 | 2.1E3 | 5.1E2 | 218 | 139 | 218 | 139 | 0.51 |
| Mm | pg/ml | 4.1E2 | 7.9E2 | 8.8E2 | 1.5E3 | 1.2E3 | 1.8E3 | 1.0E-9 | 1.0E-9 | 1.0E4 | 1.0E4 | 218 | 139 | 218 | 139 | 0.61 |
| Mn | pg/ml | 4.8E0 | 8.4E0 | 1.1E1 | 1.2E1 | 3.0E1 | 1.1E1 | 1.0E-9 | 1.0E-9 | 3.5E2 | 6.8E1 | 218 | 139 | 218 | 139 | 0.66 |

| Code | Units | Median | | Average | | Standard Deviation | | Minimum | | Maximum | | Number of Samples | | Number of Patients | | AUC |
|------|-------|--------|--------|---------|--------|--------|--------|--------|--------|--------|--------|--------|--------|--------|--------|-----|
| | | NonDis | Dis | NonDis | Dis | NonDis | Dis | NonDis | Dis | NonDis | Dis | NonDis | Dis | NonDis | Dis | |
| Mp | pg/ml | 1.0E-9 | 1.0E-9 | 8.6E0 | 4.1E1 | 2.2E1 | 2.1E2 | 1.0E-9 | 1.0E-9 | 1.8E2 | 2.4E3 | 218 | 139 | 218 | 139 | 0.56 |
| Mq | pg/ml | 1.0E-9 | 1.0E-9 | 1.8E0 | 4.3E0 | 1.3E1 | 1.6E1 | 1.0E-9 | 1.0E-9 | 1.8E2 | 1.0E2 | 218 | 139 | 218 | 139 | 0.53 |
| Mr | pg/ml | 1.0E-9 | 1.0E-9 | 1.7E1 | 8.4E1 | 1.0E2 | 3.9E2 | 1.0E-9 | 1.0E-9 | 1.4E3 | 3.4E3 | 218 | 139 | 218 | 139 | 0.58 |
| Ms | pg/ml | 3.4E2 | 3.2E2 | 5.0E2 | 4.4E2 | 5.7E2 | 5.8E2 | 1.0E-9 | 1.0E-9 | 3.2E3 | 4.7E3 | 218 | 139 | 218 | 139 | 0.46 |
| Mt | pg/ml | 1.0E-9 | 9.5E-1 | 7.8E0 | 3.8E1 | 5.1E1 | 2.8E2 | 1.0E-9 | 1.0E-9 | 7.1E2 | 3.2E3 | 218 | 139 | 218 | 139 | 0.62 |
| Mu | pg/ml | 1.0E-9 | 1.0E-9 | 9.2E-1 | 2.9E0 | 7.6E0 | 2.1E1 | 1.0E-9 | 1.0E-9 | 9.9E1 | 2.3E2 | 218 | 139 | 218 | 139 | 0.54 |
| Mv | pg/ml | 1.0E-9 | 1.0E-9 | 7.1E1 | 7.0E1 | 4.1E2 | 1.9E2 | 1.0E-9 | 1.0E-9 | 4.5E3 | 9.2E2 | 218 | 139 | 218 | 139 | 0.54 |
| Mw | pg/ml | 2.8E1 | 5.6E1 | 3.1E2 | 2.9E2 | 1.7E3 | 7.9E2 | 1.0E-9 | 1.0E-9 | 1.8E4 | 5.3E3 | 218 | 139 | 218 | 139 | 0.56 |
| Mx | pg/ml | 1.0E-9 | 1.0E-9 | 1.7E-1 | 8.4E-1 | 8.3E-1 | 3.3E0 | 1.0E-9 | 1.0E-9 | 9.2E0 | 3.2E1 | 218 | 139 | 218 | 139 | 0.59 |
| My | pg/ml | 1.0E-9 | 1.0E-9 | 4.3E2 | 1.7E2 | 3.0E3 | 7.4E2 | 1.0E-9 | 1.0E-9 | 3.9E4 | 8.0E3 | 218 | 139 | 218 | 139 | 0.50 |
| Mz | pg/ml | 8.6E0 | 1.9E1 | 2.1E1 | 6.2E1 | 4.8E1 | 2.0E2 | 1.0E-9 | 1.0E-9 | 5.5E2 | 1.9E3 | 218 | 139 | 218 | 139 | 0.66 |
| Na | pg/ml | 1.0E-9 | 1.0E-9 | 7.5E-1 | 8.3E-1 | 2.1E0 | 3.9E0 | 1.0E-9 | 1.0E-9 | 7.8E0 | 4.2E1 | 218 | 139 | 218 | 139 | 0.51 |
| Nb | pg/ml | 1.9E0 | 2.4E0 | 3.9E0 | 6.0E0 | 1.2E1 | 2.0E1 | 1.0E-9 | 1.0E-9 | 1.5E2 | 2.1E2 | 218 | 139 | 218 | 139 | 0.56 |
| Nc | pg/ml | 4.1E2 | 1.8E2 | 5.9E2 | 3.8E2 | 8.4E2 | 4.9E2 | 1.0E-9 | 1.0E-9 | 9.2E3 | 3.2E3 | 218 | 139 | 218 | 139 | 0.41 |
| Nd | pg/ml | 3.1E1 | 1.4E1 | 2.6E1 | 4.6E1 | 1.7E1 | 2.1E2 | 1.0E-9 | 1.0E-9 | 7.8E1 | 2.1E3 | 218 | 139 | 218 | 139 | 0.43 |
| Ne | pg/ml | 4.7E2 | 3.4E2 | 5.9E2 | 4.1E2 | 6.2E2 | 4.3E2 | 1.0E-9 | 1.0E-9 | 7.0E3 | 3.6E3 | 218 | 139 | 218 | 139 | 0.39 |
| Nf | pg/ml | 1.0E-9 | 1.0E-9 | 3.0E0 | 3.0E0 | 1.0E1 | 1.6E1 | 1.0E-9 | 1.0E-9 | 8.2E1 | 1.3E2 | 218 | 139 | 218 | 139 | 0.45 |
| Ng | pg/ml | 1.0E1 | 1.7E1 | 8.9E1 | 9.2E1 | 1.9E2 | 1.6E2 | 1.0E-9 | 1.0E-9 | 1.6E3 | 8.9E2 | 218 | 139 | 218 | 139 | 0.54 |
| Nh | pg/ml | 6.8E1 | 5.0E1 | 9.0E1 | 6.3E1 | 8.1E1 | 6.3E1 | 1.0E-9 | 3.1E0 | 5.6E2 | 5.1E2 | 218 | 139 | 218 | 139 | 0.38 |
| Ni | pg/ml | 2.2E0 | 1.0E-9 | 7.4E1 | 1.0E2 | 1.2E2 | 1.6E2 | 1.0E-9 | 1.0E-9 | 6.3E2 | 1.1E3 | 218 | 139 | 218 | 139 | 0.52 |
| Nj | pg/ml | 1.0E1 | 4.4E0 | 1.3E1 | 7.7E0 | 1.2E1 | 9.2E0 | 1.0E-9 | 1.0E-9 | 6.9E1 | 5.8E1 | 218 | 139 | 218 | 139 | 0.35 |
| Nk | pg/ml | 2.1E1 | 1.4E1 | 3.4E1 | 3.0E1 | 3.8E1 | 4.0E1 | 1.0E-9 | 1.0E-9 | 2.0E2 | 2.0E2 | 218 | 139 | 218 | 139 | 0.46 |
| Nl | pg/ml | 4.8E1 | 3.2E1 | 6.6E1 | 4.0E1 | 8.9E1 | 4.0E1 | 1.0E-9 | 1.0E-9 | 1.1E3 | 2.3E2 | 218 | 139 | 218 | 139 | 0.38 |
| Hl | pg/ml | 4.6E0 | 8.6E0 | 3.7E1 | 1.5E2 | 6.5E1 | 6.7E2 | 1.0E-9 | 1.0E-9 | 3.0E2 | 3.6E3 | 29 | 29 | 29 | 29 | 0.47 |
| Ho | pg/ml | 1.5E1 | 1.9E1 | 2.1E1 | 4.4E1 | 2.2E1 | 7.6E1 | 1.0E-9 | 6.7E0 | 8.3E1 | 3.9E2 | 29 | 29 | 29 | 29 | 0.65 |
| Hp | ng/ml | 1.6E0 | 1.6E0 | 1.3E2 | 1.3E2 | 3.1E2 | 3.1E2 | 3.6E-1 | 1.0E-9 | 8.9E2 | 8.9E2 | 29 | 29 | 29 | 29 | 0.50 |
| Tz | pg/ml | 3.3E3 | 7.2E3 | 5.3E3 | 4.5E4 | 7.6E3 | 2.5E5 | 7.4E1 | 1.0E-9 | 5.3E4 | 2.1E6 | 65 | 70 | 65 | 70 | 0.65 |
| Ua | pg/ml | 3.1E3 | 3.7E3 | 4.6E4 | 1.5E4 | 2.6E5 | 2.9E4 | 2.3E2 | 1.0E-9 | 2.1E6 | 1.3E5 | 65 | 70 | 65 | 70 | 0.51 |
| Ub | pg/ml | 7.2E2 | 4.1E2 | 1.1E3 | 6.2E2 | 1.4E3 | 7.6E2 | 1.6E1 | 1.0E-9 | 9.8E3 | 4.4E3 | 65 | 70 | 65 | 70 | 0.35 |
| Ue | pg/ml | 3.2E1 | 2.4E1 | 3.7E1 | 3.6E1 | 2.3E1 | 4.0E1 | 5.3E0 | 9.8E-2 | 1.0E2 | 2.7E2 | 65 | 70 | 65 | 70 | 0.43 |
| Uc | pg/ml | 6.9E2 | 7.8E2 | 1.1E3 | 2.4E3 | 1.1E3 | 7.0E3 | 2.2E1 | 1.0E-9 | 5.5E3 | 5.7E4 | 65 | 70 | 65 | 70 | 0.54 |
| Ud | pg/ml | 1.0E-9 | 1.0E-9 | 6.0E0 | 7.6E-1 | 4.8E1 | 6.4E0 | 1.0E-9 | 1.0E-9 | 3.9E2 | 5.3E1 | 65 | 70 | 65 | 70 | 0.50 |
| Hq | pg/ml | 1.1E0 | 1.3E0 | 1.0E2 | 2.4E2 | 1.4E3 | 2.4E3 | 1.0E-9 | 1.0E-9 | 2.0E4 | 2.8E4 | 218 | 137 | 218 | 137 | 0.53 |
| Hr | pg/ml | 1.0E2 | 8.0E1 | 7.3E2 | 5.2E2 | 1.4E3 | 1.2E3 | 1.0E-9 | 1.0E-9 | 1.2E4 | 8.9E3 | 218 | 137 | 218 | 137 | 0.45 |
| Hu | pg/ml | 1.0E-9 | 2.0E1 | 5.7E3 | 2.5E3 | 4.6E4 | 2.2E4 | 1.0E-9 | 1.0E-9 | 6.3E5 | 2.6E5 | 218 | 137 | 218 | 137 | 0.55 |
| Hv | pg/ml | 1.4E0 | 1.6E0 | 2.6E0 | 1.0E1 | 6.7E0 | 7.7E1 | 1.0E-9 | 1.0E-9 | 6.9E1 | 8.9E2 | 218 | 137 | 218 | 137 | 0.54 |
| Hw | pg/ml | 6.6E0 | 5.7E0 | 1.7E1 | 8.5E1 | 5.5E1 | 8.0E2 | 1.0E-9 | 1.0E-9 | 6.4E2 | 9.4E3 | 218 | 137 | 218 | 137 | 0.47 |
| Hx | pg/ml | 8.2E0 | 1.2E1 | 6.6E1 | 4.4E1 | 6.3E2 | 1.4E2 | 1.0E-9 | 1.0E-9 | 9.3E3 | 1.3E3 | 218 | 137 | 218 | 137 | 0.57 |
| Ib | ng/ml | 5.6E-2 | 2.5E-2 | 9.9E-1 | 1.8E0 | 4.2E0 | 8.1E0 | 1.0E-9 | 1.0E-9 | 3.0E1 | 5.6E1 | 65 | 68 | 65 | 68 | 0.45 |
| Ic | U/ml | 2.2E2 | 2.2E2 | 2.8E2 | 1.4E3 | 5.6E2 | 8.0E3 | 2.4E0 | 6.6E0 | 4.1E3 | 6.5E4 | 65 | 68 | 65 | 68 | 0.56 |
| Id | U/ml | 5.5E-1 | 8.2E-1 | 9.1E-1 | 8.1E0 | 1.2E0 | 5.2E1 | 1.0E-9 | 1.0E-9 | 6.9E0 | 4.3E2 | 65 | 68 | 65 | 68 | 0.61 |
| Tt | pg/ml | 1.6E2 | 1.7E2 | 1.7E2 | 1.8E2 | 5.0E1 | 6.4E1 | 4.3E1 | 7.3E1 | 3.0E2 | 4.4E2 | 60 | 64 | 60 | 64 | 0.55 |
| To | pg/ml | 1.8E0 | 1.5E0 | 2.3E0 | 2.0E0 | 3.3E0 | 2.5E0 | 1.0E-9 | 1.0E-9 | 2.3E1 | 1.2E1 | 63 | 68 | 63 | 68 | 0.46 |
| Tr | pg/ml | 3.5E0 | 3.3E0 | 5.5E0 | 1.2E1 | 6.8E0 | 3.9E1 | 1.0E-9 | 1.0E-9 | 3.8E1 | 3.1E2 | 62 | 66 | 62 | 66 | 0.54 |
| Tn | pg/ml | 2.2E1 | 4.3E1 | 9.7E1 | 1.7E2 | 2.8E2 | 4.3E2 | 1.0E-9 | 2.4E0 | 1.7E3 | 2.3E3 | 63 | 68 | 63 | 68 | 0.62 |
| Tv | ng/ml | 1.2E1 | 1.2E1 | 1.7E1 | 1.4E2 | 1.9E1 | 8.6E2 | 1.0E-9 | 1.0E-9 | 1.1E2 | 7.1E3 | 63 | 68 | 63 | 68 | 0.52 |
| Ih | ng/ml | 4.8E1 | 1.1E2 | 2.0E2 | 3.6E2 | 3.7E2 | 5.8E2 | 1.0E-9 | 1.0E-9 | 2.5E3 | 3.6E3 | 218 | 138 | 218 | 138 | 0.61 |
| Ii | ng/ml | 6.2E1 | 1.2E2 | 2.2E2 | 2.4E2 | 5.6E2 | 5.3E2 | 1.6E0 | 7.3E-1 | 5.2E3 | 4.5E3 | 218 | 138 | 218 | 138 | 0.59 |
| Ij | ng/ml | 7.1E1 | 1.1E2 | 1.9E2 | 3.6E2 | 6.9E2 | 2.1E3 | 2.8E0 | 9.5E0 | 6.4E3 | 2.4E4 | 217 | 135 | 217 | 135 | 0.64 |
| Ik | ng/ml | 7.7E0 | 1.7E1 | 1.9E3 | 2.5E2 | 1.4E4 | 4.7E2 | 5.9E-1 | 6.2E-1 | 1.2E5 | 2.1E3 | 216 | 136 | 216 | 136 | 0.60 |
| Il | ng/ml | 3.6E2 | 4.1E2 | 1.4E3 | 1.4E3 | 3.0E3 | 2.8E3 | 1.0E-9 | 1.0E-9 | 1.2E4 | 1.2E4 | 215 | 135 | 215 | 135 | 0.53 |
| Im | ng/ml | 1.8E2 | 2.9E2 | 3.1E2 | 8.2E2 | 3.7E2 | 1.7E3 | 1.4E1 | 2.2E1 | 3.1E3 | 1.5E4 | 216 | 136 | 216 | 136 | 0.66 |
| In | ng/ml | 3.8E0 | 2.7E0 | 2.4E1 | 5.0E1 | 1.1E2 | 3.9E2 | 1.0E-9 | 1.0E-9 | 1.1E3 | 4.5E3 | 218 | 138 | 218 | 138 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Hb | ng/ml | 2.0E1 | 3.2E1 | 2.8E1 | 4.2E1 | 2.8E1 | 4.2E1 | 1.6E0 | 4.8E-1 | 1.4E2 | 2.0E2 | 64 | 72 | 64 | 72 | 0.61 |
| Hc | pg/ml | 7.0E2 | 6.4E2 | 4.1E3 | 2.3E3 | 1.4E4 | 6.5E3 | 1.0E-9 | 1.0E-9 | 1.0E5 | 5.0E4 | 64 | 72 | 64 | 72 | 0.48 |
| Hf | ng/ml | 1.5E2 | 2.3E2 | 3.9E2 | 4.0E2 | 5.5E2 | 5.4E2 | 1.0E-9 | 1.0E-9 | 2.5E3 | 3.2E3 | 64 | 72 | 64 | 72 | 0.55 |
| Io | ng/ml | 6.7E3 | 1.4E4 | 1.8E4 | 2.2E4 | 5.4E4 | 2.8E4 | 1.2E2 | 1.0E-9 | 7.1E5 | 2.0E5 | 217 | 139 | 217 | 139 | 0.62 |
| Ip | ng/ml | 7.9E0 | 2.7E1 | 1.8E1 | 2.7E1 | 2.5E1 | 2.7E1 | 1.0E-9 | 1.0E-9 | 2.3E2 | 1.6E2 | 217 | 139 | 217 | 139 | 0.60 |
| Iq | ug/ml | 9.8E-2 | 1.4E-1 | 6.3E1 | 2.8E0 | 9.2E2 | 1.9E1 | 1.0E-9 | 1.0E-9 | 1.4E4 | 2.2E2 | 217 | 139 | 217 | 139 | 0.54 |
| Ir | ug/ml | 3.2E-1 | 7.6E-1 | 4.7E0 | 8.8E0 | 3.8E1 | 3.8E1 | 1.0E-9 | 1.0E-9 | 5.1E2 | 3.7E2 | 216 | 139 | 216 | 139 | 0.65 |
| Is | ng/ml | 1.5E0 | 3.9E0 | 7.6E0 | 1.6E1 | 3.9E1 | 3.4E1 | 1.0E-9 | 1.0E-9 | 5.5E2 | 2.6E2 | 217 | 139 | 217 | 139 | 0.64 |
| It | ng/ml | 1.4E0 | 2.9E0 | 2.3E1 | 2.5E1 | 1.1E2 | 1.0E2 | 1.0E-9 | 1.0E-9 | 1.1E3 | 8.3E2 | 217 | 139 | 217 | 139 | 0.61 |
| Iu | ng/ml | 1.6E2 | 1.8E2 | 1.3E3 | 1.6E3 | 4.2E3 | 4.7E3 | 1.0E-9 | 1.0E-9 | 2.9E4 | 2.4E4 | 217 | 139 | 217 | 139 | 0.53 |
| Iv | ng/ml | 8.1E0 | 2.2E1 | 1.0E2 | 1.4E2 | 1.1E3 | 5.6E2 | 1.0E-9 | 1.0E-9 | 1.6E4 | 3.8E3 | 217 | 138 | 217 | 138 | 0.66 |
| Iz | ng/ml | 1.2E2 | 1.3E2 | 4.7E2 | 2.8E2 | 9.7E2 | 3.6E2 | 1.5E0 | 8.8E-1 | 6.1E3 | 1.7E3 | 64 | 72 | 64 | 72 | 0.51 |
| Yg | pg/ml | 2.5E2 | 2.7E2 | 5.3E2 | 2.8E3 | 7.8E2 | 9.4E3 | 8.9E0 | 1.0E-9 | 3.4E3 | 5.0E4 | 26 | 28 | 26 | 28 | 0.56 |
| Yh | pg/ml | 2.1E2 | 2.3E2 | 3.5E2 | 5.4E2 | 4.2E2 | 7.2E2 | 1.0E-9 | 1.0E-9 | 1.8E3 | 3.0E3 | 26 | 28 | 26 | 28 | 0.54 |
| Yi | pg/ml | 2.3E2 | 4.8E2 | 4.6E2 | 1.9E3 | 5.0E2 | 5.1E3 | 1.0E-9 | 1.0E-9 | 2.0E3 | 2.6E4 | 26 | 28 | 26 | 28 | 0.59 |
| Yk | U/ml | 1.0E-9 | 1.0E-9 | 1.5E-1 | 2.9E-1 | 4.5E-1 | 7.3E-1 | 1.0E-9 | 1.0E-9 | 1.8E0 | 3.0E0 | 26 | 28 | 26 | 28 | 0.52 |
| Yj | pg/ml | 2.0E2 | 1.1E2 | 3.9E2 | 2.0E2 | 6.4E2 | 2.9E2 | 9.7E0 | 1.0E-9 | 3.2E3 | 1.5E3 | 26 | 28 | 26 | 28 | 0.37 |
| Yd | ng/ml | 1.8E-1 | 2.8E-1 | 3.8E-1 | 4.2E-1 | 4.9E-1 | 5.4E-1 | 6.6E-3 | 1.6E-2 | 1.8E0 | 2.3E0 | 29 | 29 | 29 | 29 | 0.54 |
| Wb | pg/ml | 2.6E4 | 3.5E4 | 2.9E4 | 6.2E4 | 1.7E4 | 1.1E5 | 4.9E3 | 7.5E3 | 8.4E4 | 6.4E5 | 29 | 29 | 29 | 29 | 0.66 |
| Vz | pg/ml | 3.8E0 | 2.9E0 | 4.8E0 | 4.6E0 | 3.9E0 | 5.3E0 | 1.0E-9 | 7.6E-2 | 1.6E1 | 2.2E1 | 29 | 29 | 29 | 29 | 0.45 |
| Si | ng/ml | 1.2E0 | 1.2E0 | 2.3E0 | 1.7E0 | 3.1E0 | 1.6E0 | 1.1E-1 | 8.6E-3 | 1.0E1 | 6.0E0 | 29 | 29 | 29 | 29 | 0.52 |
| Sf | mIU/mL | 1.5E1 | 1.9E1 | 7.2E1 | 2.0E1 | 1.4E2 | 1.9E1 | 6.2E-1 | 1.3E0 | 7.2E2 | 8.3E1 | 29 | 29 | 29 | 29 | 0.43 |
| Sh | mIU/mL | 1.2E1 | 1.3E1 | 5.7E1 | 2.4E1 | 1.2E2 | 3.4E1 | 1.8E-1 | 7.8E-2 | 5.7E2 | 1.8E2 | 29 | 29 | 29 | 29 | 0.49 |
| Sj | ng/ml | 3.8E-1 | 4.4E-1 | 4.0E-1 | 4.5E-1 | 8.7E-2 | 9.7E-2 | 2.5E-1 | 3.2E-1 | 5.7E-1 | 7.2E-1 | 29 | 29 | 29 | 29 | 0.65 |
| Rc | pg/ml | 6.6E3 | 6.9E3 | 7.6E3 | 7.8E3 | 5.4E3 | 6.3E3 | 3.9E2 | 5.5E2 | 2.8E4 | 3.9E4 | 65 | 70 | 65 | 70 | 0.50 |
| Rb | pg/ml | 7.6E-1 | 7.0E-1 | 2.8E0 | 3.2E0 | 4.1E0 | 7.5E0 | 1.0E-9 | 1.0E-9 | 1.6E1 | 5.6E1 | 65 | 70 | 65 | 70 | 0.51 |
| Zq | 2.6ng/ml | 1.7E2 | 3.4E2 | 2.7E2 | 3.4E2 | 2.3E2 | 2.0E2 | 1.4E1 | 1.7E1 | 9.7E2 | 9.7E2 | 28 | 28 | 28 | 28 | 0.62 |
| Zw | 2.5ng/ml | 4.8E0 | 5.3E0 | 9.6E0 | 1.1E1 | 1.2E1 | 1.6E1 | 1.4E-1 | 2.4E-1 | 5.9E1 | 6.3E1 | 29 | 29 | 29 | 29 | 0.47 |
| Zx | 2.3mU/ml | 8.1E-2 | 1.4E-1 | 1.9E-1 | 3.9E-1 | 2.0E-1 | 6.8E-1 | 4.2E-2 | 3.2E-2 | 8.4E-1 | 2.9E0 | 29 | 29 | 29 | 29 | 0.61 |
| Pz | ng/ml | 3.0E3 | 5.7E3 | 5.1E3 | 6.5E3 | 5.1E3 | 7.0E3 | 1.6E1 | 4.0E1 | 2.9E4 | 7.0E4 | 216 | 136 | 216 | 136 | 0.59 |
| Qa | ng/ml | 2.9E3 | 6.2E3 | 5.7E3 | 1.1E4 | 7.2E3 | 2.0E4 | 1.5E2 | 2.9E2 | 4.2E4 | 2.2E5 | 216 | 136 | 216 | 136 | 0.65 |
| Qb | ng/ml | 9.1E1 | 1.5E2 | 2.1E2 | 2.9E2 | 4.5E2 | 4.2E2 | 7.9E-1 | 6.7E0 | 5.3E3 | 4.1E3 | 216 | 136 | 216 | 136 | 0.62 |
| Qc | ng/ml | 1.6E2 | 3.6E2 | 3.9E2 | 5.6E2 | 5.7E2 | 6.3E2 | 1.0E-9 | 1.0E-9 | 3.8E3 | 4.3E3 | 216 | 136 | 216 | 136 | 0.61 |
| Qd | ng/ml | 7.1E3 | 1.4E4 | 2.2E4 | 3.6E4 | 1.4E5 | 5.9E4 | 1.5E2 | 1.2E3 | 2.0E6 | 4.3E5 | 216 | 136 | 216 | 136 | 0.68 |
| Qe | ng/ml | 6.4E2 | 1.7E3 | 1.9E3 | 2.5E3 | 6.9E3 | 2.7E3 | 1.0E-9 | 8.8E0 | 9.7E4 | 1.8E4 | 216 | 136 | 216 | 136 | 0.67 |
| Jd | ng/ml | 4.1E-1 | 1.2E0 | 5.4E0 | 2.8E0 | 2.0E1 | 4.1E0 | 1.0E-9 | 1.0E-9 | 1.2E2 | 2.1E1 | 65 | 70 | 65 | 70 | 0.61 |
| Jc | ng/ml | 1.0E-9 | 1.0E-9 | 2.0E0 | 1.6E0 | 6.5E0 | 2.5E0 | 1.0E-9 | 1.0E-9 | 4.6E1 | 1.1E1 | 65 | 70 | 65 | 70 | 0.55 |
| Jf | ng/ml | 1.0E-9 | 1.0E-9 | 9.6E-1 | 1.4E0 | 2.2E0 | 2.3E0 | 1.0E-9 | 1.0E-9 | 1.2E1 | 9.1E0 | 65 | 70 | 65 | 70 | 0.54 |
| Jg | ng/ml | 3.3E2 | 7.8E2 | 6.6E2 | 1.1E3 | 9.6E2 | 1.0E3 | 5.8E0 | 1.3E1 | 1.0E4 | 7.1E3 | 218 | 137 | 218 | 137 | 0.66 |
| Jh | ng/ml | 2.0E0 | 4.9E0 | 2.3E1 | 2.4E1 | 9.9E1 | 6.0E1 | 1.0E-9 | 1.0E-9 | 1.2E3 | 4.9E2 | 218 | 137 | 218 | 137 | 0.61 |
| Ji | ng/ml | 4.5E1 | 8.5E1 | 6.5E1 | 1.5E2 | 7.1E1 | 1.7E2 | 1.1E0 | 8.9E0 | 5.3E2 | 1.3E3 | 218 | 137 | 218 | 137 | 0.71 |
| Sr | pg/mL | 3.0E2 | 5.7E2 | 6.3E2 | 1.5E3 | 9.2E2 | 2.8E3 | 1.0E-9 | 1.0E-9 | 4.3E3 | 2.1E4 | 65 | 69 | 65 | 69 | 0.64 |
| Ss | pg/mL | 7.9E4 | 9.2E4 | 1.4E5 | 1.5E5 | 1.7E5 | 1.9E5 | 9.1E3 | 2.7E3 | 7.1E5 | 1.3E6 | 65 | 69 | 65 | 69 | 0.51 |
| St | pg/mL | 1.9E7 | 4.2E7 | 4.0E7 | 1.0E8 | 6.1E7 | 2.4E8 | 7.8E5 | 1.0E-9 | 4.1E8 | 1.7E9 | 63 | 70 | 63 | 70 | 0.64 |
| Wc | ng/ml | 1.0E-9 | 4.1E-3 | 5.9E-2 | 1.3E-1 | 1.2E-1 | 3.4E-1 | 1.0E-9 | 1.0E-9 | 5.2E-1 | 1.8E0 | 29 | 29 | 29 | 29 | 0.56 |
| Wd | ng/ml | 9.3E0 | 9.7E0 | 2.2E1 | 5.6E1 | 4.7E1 | 1.1E2 | 1.0E0 | 1.5E0 | 2.4E2 | 4.1E2 | 29 | 29 | 29 | 29 | 0.56 |
| We | ng/ml | 2.5E-1 | 4.9E-1 | 9.5E-1 | 1.8E0 | 1.4E0 | 4.5E0 | 1.0E-9 | 1.0E-9 | 5.5E0 | 2.3E1 | 29 | 29 | 29 | 29 | 0.57 |
| Wg | ng/ml | 1.0E-9 | 1.0E-9 | 5.6E-4 | 1.8E-2 | 3.0E-3 | 9.9E-2 | 1.0E-9 | 1.0E-9 | 1.6E-2 | 5.3E-1 | 29 | 29 | 29 | 29 | 0.50 |
| Wh | ng/ml | 9.7E-3 | 1.1E-2 | 3.8E-2 | 5.5E-2 | 8.5E-2 | 1.1E-1 | 1.0E-9 | 1.0E-9 | 3.6E-1 | 4.2E-1 | 29 | 29 | 29 | 29 | 0.56 |
| Wf | ng/ml | 1.0E-9 | 1.0E-9 | 6.5E-2 | 1.9E-1 | 1.4E-1 | 4.8E-1 | 1.0E-9 | 1.0E-9 | 4.8E-1 | 2.3E0 | 29 | 29 | 29 | 29 | 0.56 |
| Ra | pg/ml | 1.0E-9 | 1.0E-9 | 5.4E-1 | 1.2E0 | 1.1E0 | 7.7E0 | 1.0E-9 | 1.0E-9 | 3.8E0 | 6.4E1 | 65 | 70 | 65 | 70 | 0.44 |
| Qz | pg/ml | 1.1E1 | 9.0E0 | 6.4E1 | 3.9E1 | 1.0E2 | 6.6E1 | 1.0E-9 | 1.0E-9 | 4.5E2 | 2.8E2 | 65 | 70 | 65 | 70 | 0.44 |
| Qy | pg/ml | 3.8E-1 | 4.6E-1 | 5.3E0 | 1.9E1 | 2.9E1 | 1.0E2 | 1.0E-9 | 1.0E-9 | 2.3E2 | 7.3E2 | 65 | 70 | 65 | 70 | 0.53 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qx | pg/ml | 1.0E-9 | 1.0E-9 | 4.3E0 | 3.2E0 | 2.4E1 | 1.4E1 | 1.0E-9 | 1.0E-9 | 1.9E2 | 1.1E2 | 65 | 70 | 65 | 70 | 0.52 |
| Qw | pg/ml | 3.1E-1 | 1.0E-9 | 2.0E0 | 3.5E0 | 5.3E0 | 1.2E1 | 1.0E-9 | 1.0E-9 | 3.0E1 | 6.6E1 | 65 | 70 | 65 | 70 | 0.44 |
| Qv | pg/ml | 2.2E4 | 1.2E4 | 3.3E4 | 3.9E4 | 4.7E4 | 1.2E5 | 1.4E3 | 1.0E-9 | 3.7E5 | 9.4E5 | 65 | 70 | 65 | 70 | 0.39 |
| Qu | pg/ml | 6.2E0 | 8.0E0 | 9.5E1 | 8.9E1 | 1.9E2 | 1.8E2 | 1.0E-9 | 1.0E-9 | 7.1E2 | 9.8E2 | 65 | 70 | 65 | 70 | 0.50 |
| Qt | pg/ml | 1.1E1 | 1.6E1 | 5.0E1 | 4.0E1 | 1.2E2 | 6.9E1 | 1.0E-9 | 1.0E-9 | 7.1E2 | 3.3E2 | 65 | 70 | 65 | 70 | 0.54 |
| Qh | ng/ml | 1.7E1 | 2.3E1 | 3.2E1 | 6.7E1 | 4.5E1 | 1.2E2 | 2.5E-1 | 4.3E-1 | 2.4E2 | 8.0E2 | 65 | 70 | 65 | 70 | 0.60 |
| Qg | ng/ml | 8.6E0 | 6.5E0 | 1.7E1 | 1.0E1 | 3.5E1 | 1.2E1 | 1.5E-1 | 3.0E-1 | 2.7E2 | 8.1E1 | 65 | 70 | 65 | 70 | 0.43 |
| Jj | ng/ml | 5.6E2 | 4.0E2 | 2.6E3 | 7.3E2 | 2.3E4 | 1.2E3 | 2.3E0 | 8.7E0 | 3.4E5 | 1.1E4 | 218 | 137 | 218 | 137 | 0.42 |
| Jk | ng/ml | 2.6E0 | 2.9E0 | 1.9E1 | 2.7E1 | 4.5E1 | 5.6E1 | 1.0E-9 | 4.3E-2 | 2.8E2 | 3.9E2 | 218 | 137 | 218 | 137 | 0.55 |
| Jl | ng/ml | 3.9E-1 | 6.4E-1 | 1.5E0 | 7.6E1 | 3.4E0 | 8.5E2 | 1.2E-3 | 5.4E-3 | 2.0E1 | 9.9E3 | 218 | 137 | 218 | 137 | 0.61 |
| Jm | ng/ml | 1.6E1 | 2.8E1 | 6.1E1 | 7.7E1 | 1.5E2 | 1.9E2 | 1.0E-9 | 1.0E-9 | 1.4E3 | 2.1E3 | 218 | 137 | 218 | 137 | 0.58 |
| Jn | pg/ml | 2.3E-1 | 5.5E-1 | 4.1E0 | 1.4E1 | 4.2E1 | 8.4E1 | 1.0E-9 | 1.0E-9 | 6.2E2 | 7.3E2 | 218 | 137 | 218 | 137 | 0.64 |
| Jo | pg/ml | 3.4E3 | 4.6E3 | 4.5E3 | 6.3E3 | 3.8E3 | 9.7E3 | 2.0E1 | 2.4E1 | 2.4E4 | 1.0E5 | 218 | 137 | 218 | 137 | 0.56 |
| Jp | pg/ml | 6.5E4 | 8.4E4 | 6.7E4 | 8.8E4 | 3.7E4 | 4.1E4 | 5.8E2 | 4.6E3 | 1.9E5 | 3.8E5 | 218 | 137 | 218 | 137 | 0.67 |
| Jq | pg/ml | 8.6E1 | 1.4E2 | 1.5E2 | 3.0E2 | 2.0E2 | 8.2E2 | 1.0E0 | 5.6E0 | 2.0E3 | 8.7E3 | 218 | 137 | 218 | 137 | 0.60 |
| Jr | pg/ml | 2.6E0 | 8.3E0 | 7.2E1 | 1.3E2 | 7.3E2 | 7.9E2 | 1.0E-9 | 1.0E-9 | 1.1E4 | 7.4E3 | 218 | 137 | 218 | 137 | 0.62 |
| Js | pg/ml | 1.2E1 | 1.8E1 | 7.8E1 | 1.1E2 | 6.9E2 | 4.4E2 | 1.0E-9 | 1.9E0 | 1.0E4 | 3.0E3 | 218 | 137 | 218 | 137 | 0.64 |
| Jt | pg/ml | 2.1E3 | 3.1E3 | 2.6E3 | 4.4E3 | 2.1E3 | 6.2E3 | 2.2E1 | 1.5E2 | 2.2E4 | 5.2E4 | 218 | 137 | 218 | 137 | 0.63 |
| Xa | pg/ml | 1.0E-9 | 5.2E0 | 5.8E0 | 7.5E1 | 1.0E1 | 2.4E2 | 1.0E-9 | 1.0E-9 | 4.1E1 | 1.2E3 | 29 | 29 | 29 | 29 | 0.66 |
| Ye | pg/ml | 1.0E-9 | 1.0E-9 | 4.8E0 | 3.4E0 | 1.5E1 | 1.1E1 | 1.0E-9 | 1.0E-9 | 6.9E1 | 6.1E1 | 29 | 29 | 29 | 29 | 0.47 |
| Tm | pg/ml | 1.0E-9 | 1.0E-9 | 2.8E0 | 2.3E0 | 5.0E0 | 3.8E0 | 1.0E-9 | 1.0E-9 | 1.8E1 | 8.4E0 | 29 | 29 | 29 | 29 | 0.49 |
| Tl | pg/ml | 1.1E-1 | 1.3E-1 | 2.3E-1 | 1.2E0 | 3.6E-1 | 4.5E0 | 1.0E-9 | 1.0E-9 | 1.2E0 | 2.5E1 | 29 | 29 | 29 | 29 | 0.63 |
| Ju | mIU/ml | 7.5E0 | 1.2E1 | 2.8E1 | 2.0E1 | 4.3E1 | 2.3E1 | 2.5E-1 | 1.7E-1 | 2.3E2 | 1.1E2 | 65 | 70 | 65 | 70 | 0.54 |
| Jv | mIU/ml | 1.4E1 | 1.6E1 | 4.7E1 | 3.1E1 | 8.0E1 | 3.8E1 | 2.4E-2 | 1.7E-2 | 4.4E2 | 1.8E2 | 65 | 70 | 65 | 70 | 0.53 |
| Jy | ng/ml | 1.8E-3 | 1.6E-3 | 2.0E-3 | 3.3E-3 | 1.0E-3 | 6.9E-3 | 1.0E-9 | 1.7E-4 | 4.7E-3 | 4.1E-2 | 65 | 70 | 65 | 70 | 0.48 |
| Kc | pg/ml | 2.0E1 | 2.6E1 | 3.3E1 | 5.6E1 | 3.9E1 | 6.6E1 | 1.0E-9 | 1.0E-9 | 1.7E2 | 3.2E2 | 64 | 72 | 64 | 72 | 0.62 |
| Kd | pg/ml | 1.0E-9 | 1.0E-9 | 1.4E2 | 9.0E2 | 6.3E2 | 4.6E3 | 1.0E-9 | 1.0E-9 | 4.8E3 | 3.8E4 | 64 | 72 | 64 | 72 | 0.52 |
| Ke | pg/ml | 1.1E4 | 1.4E4 | 1.3E4 | 2.4E4 | 8.8E3 | 4.0E4 | 1.0E3 | 6.7E2 | 4.6E4 | 3.2E5 | 64 | 72 | 64 | 72 | 0.62 |
| Kf | pg/mL | 4.9E0 | 8.1E0 | 5.4E0 | 9.5E0 | 4.4E0 | 1.1E1 | 1.0E-9 | 1.0E-9 | 1.8E1 | 7.8E1 | 64 | 72 | 64 | 72 | 0.65 |
| Kg | pg/mL | 8.5E2 | 1.1E3 | 1.8E3 | 2.5E3 | 3.0E3 | 5.4E3 | 7.7E1 | 1.3E2 | 2.2E4 | 3.6E4 | 64 | 72 | 64 | 72 | 0.55 |
| Ki | pg/ml | 6.2E1 | 6.2E1 | 6.9E1 | 7.3E1 | 5.3E1 | 5.3E1 | 1.0E-9 | 1.0E-9 | 2.9E2 | 2.5E2 | 64 | 72 | 64 | 72 | 0.53 |
| Kj | pg/ml | 7.9E2 | 8.5E2 | 1.3E3 | 1.5E3 | 1.5E3 | 2.1E3 | 6.6E1 | 3.3E1 | 8.8E3 | 1.5E4 | 64 | 72 | 64 | 72 | 0.51 |
| Kk | pg/ml | 6.8E0 | 8.7E0 | 1.1E1 | 1.7E1 | 1.4E1 | 1.7E1 | 1.0E-9 | 1.0E-9 | 8.1E1 | 6.1E1 | 64 | 72 | 64 | 72 | 0.61 |
| Kl | pg/ml | 1.8E4 | 1.8E4 | 2.6E4 | 2.8E4 | 2.4E4 | 2.7E4 | 4.1E2 | 2.3E2 | 1.1E5 | 1.3E5 | 64 | 72 | 64 | 72 | 0.53 |
| Kn | pg/ml | 1.5E1 | 5.2E1 | 4.4E1 | 1.6E2 | 6.3E1 | 5.8E2 | 1.0E-9 | 1.0E-9 | 3.4E2 | 4.9E3 | 64 | 72 | 64 | 72 | 0.60 |
| Ko | pg/ml | 3.0E2 | 4.4E2 | 3.8E2 | 6.4E2 | 4.1E2 | 7.5E2 | 1.0E-9 | 1.0E-9 | 1.8E3 | 4.1E3 | 64 | 72 | 64 | 72 | 0.63 |
| Kp | pg/ml | 2.9E2 | 4.1E2 | 3.0E2 | 6.0E2 | 2.3E2 | 1.5E3 | 1.0E-9 | 1.0E-9 | 8.6E2 | 1.3E4 | 64 | 72 | 64 | 72 | 0.63 |
| Kq | pg/ml | 2.8E2 | 3.9E2 | 3.5E2 | 3.0E3 | 3.6E2 | 1.9E4 | 5.1E0 | 1.6E0 | 2.1E3 | 1.6E5 | 61 | 70 | 61 | 70 | 0.65 |
| Kr | pg/ml | 1.0E-9 | 7.3E-1 | 1.4E0 | 8.5E0 | 2.6E0 | 5.0E1 | 1.0E-9 | 1.0E-9 | 1.2E1 | 4.2E2 | 61 | 70 | 61 | 70 | 0.58 |
| Ks | pg/ml | 1.3E4 | 1.8E4 | 1.9E4 | 2.2E4 | 1.7E4 | 1.9E4 | 4.5E2 | 5.1E1 | 7.9E4 | 6.3E4 | 61 | 70 | 61 | 70 | 0.54 |
| Ps | ng/ml | 1.3E2 | 2.9E2 | 5.0E2 | 1.2E3 | 1.7E3 | 2.6E3 | 1.6E0 | 5.5E0 | 9.0E3 | 1.2E4 | 29 | 29 | 29 | 29 | 0.66 |
| Kx | ng/ml | 1.0E-9 | 3.8E-3 | 4.2E-3 | 1.2E-2 | 8.6E-3 | 2.0E-2 | 1.0E-9 | 1.0E-9 | 5.1E-2 | 1.0E-1 | 63 | 72 | 63 | 72 | 0.60 |
| Ky | ng/ml | 8.7E-2 | 2.2E-1 | 3.3E-1 | 4.8E-1 | 7.4E-1 | 7.9E-1 | 1.0E-9 | 1.0E-9 | 5.1E0 | 4.4E0 | 63 | 72 | 63 | 72 | 0.62 |
| Kz | ng/ml | 1.0E-9 | 1.0E-9 | 4.3E-3 | 3.6E-3 | 6.2E-3 | 6.2E-3 | 1.0E-9 | 1.0E-9 | 1.6E-2 | 2.5E-2 | 63 | 72 | 63 | 72 | 0.46 |
| Rz | ng/ml | 3.6E-1 | 3.3E-1 | 9.5E-1 | 8.9E-1 | 1.5E0 | 1.5E0 | 1.1E-2 | 4.6E-3 | 6.7E0 | 7.5E0 | 29 | 29 | 29 | 29 | 0.53 |
| Ry | ng/ml | 1.6E-2 | 2.3E-2 | 1.9E-2 | 4.1E-2 | 1.4E-2 | 6.8E-2 | 1.0E-9 | 1.0E-9 | 5.0E-2 | 3.5E-1 | 29 | 29 | 29 | 29 | 0.56 |
| Rx | ng/ml | 1.0E-9 | 1.0E-9 | 1.3E-3 | 1.7E-3 | 2.1E-3 | 2.6E-3 | 1.0E-9 | 1.0E-9 | 8.6E-3 | 8.6E-3 | 29 | 29 | 29 | 29 | 0.54 |
| Ld | pg/ml | 1.0E-9 | 1.0E-9 | 2.9E0 | 4.8E0 | 9.2E0 | 8.4E0 | 1.0E-9 | 1.0E-9 | 6.9E1 | 5.0E1 | 64 | 72 | 64 | 72 | 0.59 |
| Lh | pg/ml | 9.3E3 | 1.9E4 | 1.8E4 | 3.7E4 | 2.6E4 | 6.6E4 | 1.0E-9 | 1.0E-9 | 2.6E5 | 4.8E5 | 218 | 138 | 218 | 138 | 0.65 |
| Li | pg/ml | 2.6E3 | 6.6E3 | 1.6E4 | 3.1E4 | 9.1E4 | 9.5E4 | 1.2E1 | 1.3E1 | 1.3E6 | 9.2E5 | 218 | 138 | 218 | 138 | 0.64 |
| Lj | pg/ml | 2.1E3 | 4.9E3 | 1.7E4 | 2.8E4 | 5.6E4 | 6.1E4 | 1.0E-9 | 1.0E-9 | 4.3E5 | 4.1E5 | 218 | 138 | 218 | 138 | 0.60 |
| Lp | pg/ml | 1.1E1 | 9.5E0 | 8.0E1 | 1.4E2 | 2.1E2 | 3.5E2 | 1.0E-9 | 1.0E-9 | 1.1E3 | 1.4E3 | 29 | 29 | 29 | 29 | 0.51 |
| Lt | pg/ml | 1.0E-9 | 1.0E-9 | 1.4E0 | 1.6E0 | 5.3E0 | 5.9E0 | 1.0E-9 | 1.0E-9 | 2.5E1 | 2.5E1 | 29 | 29 | 29 | 29 | 0.50 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Rv | ng/ml | 5.0E-4 | 5.0E-4 | 1.1E-3 | 1.9E-3 | 1.8E-3 | 3.8E-3 | 1.0E-9 | 1.0E-9 | 9.2E-3 | 1.6E-2 | 29 | 29 | 29 | 29 | 0.54 |
| Ru | ng/ml | 1.0E-9 | 1.0E-9 | 1.8E-2 | 2.6E-2 | 6.8E-2 | 8.9E-2 | 1.0E-9 | 1.0E-9 | 3.1E-1 | 3.5E-1 | 29 | 29 | 29 | 29 | 0.52 |
| Rt | ng/ml | 7.2E-2 | 7.5E-2 | 1.0E-1 | 3.9E-1 | 9.9E-2 | 1.4E0 | 6.5E-3 | 1.3E-3 | 3.8E-1 | 7.4E0 | 29 | 29 | 29 | 29 | 0.52 |
| Yl | pg/ml | 1.4E1 | 1.2E1 | 1.7E1 | 2.5E1 | 1.3E1 | 4.2E1 | 1.0E-9 | 1.0E-9 | 4.7E1 | 2.2E2 | 29 | 29 | 29 | 29 | 0.48 |
| Rm | ng/ml | 1.8E1 | 1.9E1 | 4.0E1 | 5.9E1 | 6.6E1 | 1.0E2 | 2.2E-1 | 2.3E-1 | 3.4E2 | 6.5E2 | 65 | 69 | 65 | 69 | 0.54 |
| Rh | ng/ml | 1.8E2 | 1.7E2 | 6.2E2 | 5.1E2 | 2.1E3 | 2.1E3 | 7.5E0 | 2.5E1 | 1.7E4 | 1.7E4 | 65 | 69 | 65 | 69 | 0.46 |
| Ri | ng/ml | 4.1E-1 | 1.0E-9 | 4.6E0 | 3.0E0 | 9.1E0 | 6.8E0 | 1.0E-9 | 1.0E-9 | 4.9E1 | 4.5E1 | 65 | 69 | 65 | 69 | 0.44 |
| Rg | ng/ml | 1.0E-9 | 1.0E-9 | 8.1E-3 | 1.5E-1 | 3.6E-2 | 5.8E-1 | 1.0E-9 | 1.0E-9 | 2.7E-1 | 3.3E0 | 65 | 69 | 65 | 69 | 0.54 |
| Rj | ng/ml | 1.0E-9 | 1.0E-9 | 9.3E-1 | 4.7E0 | 1.9E0 | 3.2E1 | 1.0E-9 | 1.0E-9 | 1.1E1 | 2.7E2 | 65 | 69 | 65 | 69 | 0.48 |
| Rf | ng/ml | 4.1E-1 | 3.5E-1 | 8.3E-1 | 1.2E0 | 1.1E0 | 2.8E0 | 2.1E-2 | 2.1E-2 | 6.2E0 | 1.7E1 | 65 | 69 | 65 | 69 | 0.49 |
| Ql | pg/ml | 1.7E0 | 5.5E0 | 8.5E0 | 1.6E1 | 1.4E1 | 2.9E1 | 1.0E-9 | 1.0E-9 | 7.5E1 | 1.8E2 | 65 | 70 | 65 | 70 | 0.57 |
| Qm | pg/ml | 1.0E-9 | 9.4E0 | 1.7E1 | 2.4E1 | 3.5E1 | 3.5E1 | 1.0E-9 | 1.0E-9 | 1.9E2 | 1.8E2 | 65 | 70 | 65 | 70 | 0.59 |
| Qn | pg/ml | 6.1E-1 | 6.1E-1 | 6.3E0 | 7.6E0 | 2.7E1 | 2.6E1 | 1.0E-9 | 1.0E-9 | 2.2E2 | 2.0E2 | 65 | 70 | 65 | 70 | 0.51 |
| Nv | pg/ml | 3.1E3 | 5.9E3 | 7.3E3 | 1.5E4 | 1.5E4 | 2.6E4 | 1.0E-9 | 1.9E1 | 1.3E5 | 1.6E5 | 218 | 139 | 218 | 139 | 0.64 |
| Nw | pg/ml | 7.7E3 | 1.3E4 | 1.1E4 | 2.0E4 | 1.7E4 | 2.7E4 | 2.0E2 | 1.9E2 | 2.1E5 | 2.2E5 | 218 | 139 | 218 | 139 | 0.68 |
| Nx | pg/ml | 1.6E2 | 2.4E2 | 3.5E2 | 6.0E2 | 5.7E2 | 7.1E2 | 1.0E-9 | 1.0E-9 | 3.5E3 | 4.1E3 | 218 | 139 | 218 | 139 | 0.63 |
| Ny | pg/ml | 4.9E0 | 1.2E1 | 1.3E2 | 6.7E1 | 1.7E3 | 2.7E2 | 1.0E-9 | 1.0E-9 | 2.5E4 | 2.8E3 | 218 | 139 | 218 | 139 | 0.65 |
| Oa | pg/ml | 1.3E2 | 2.5E2 | 2.8E2 | 6.7E2 | 4.8E2 | 9.1E2 | 1.0E-9 | 1.0E-9 | 3.0E3 | 4.5E3 | 65 | 70 | 65 | 70 | 0.63 |
| Op | pg/ml | 4.3E5 | 4.4E5 | 4.4E5 | 4.4E5 | 1.4E5 | 1.9E5 | 2.1E5 | 5.2E4 | 7.3E5 | 7.5E5 | 29 | 29 | 29 | 29 | 0.51 |
| Wn | ng/ml | 9.4E0 | 1.1E1 | 1.2E2 | 1.8E1 | 3.8E2 | 1.7E1 | 1.2E0 | 7.6E-1 | 1.8E3 | 5.6E1 | 23 | 17 | 23 | 17 | 0.49 |
| Tk | ng/ml | 1.1E2 | 1.3E2 | 3.3E2 | 3.3E2 | 8.4E2 | 5.8E2 | 3.0E0 | 4.0E0 | 4.2E3 | 2.3E3 | 24 | 20 | 24 | 20 | 0.55 |
| Oe | pg/ml | 4.7E1 | 9.6E0 | 2.4E2 | 2.5E2 | 3.6E2 | 4.1E2 | 1.0E-9 | 1.0E-9 | 2.2E3 | 2.3E3 | 217 | 138 | 217 | 138 | 0.49 |
| Of | pg/ml | 1.5E2 | 1.3E2 | 4.6E3 | 5.7E3 | 2.0E4 | 2.1E4 | 1.0E-9 | 1.0E-9 | 1.9E5 | 1.7E5 | 218 | 139 | 218 | 139 | 0.49 |
| Og | pg/ml | 7.3E-2 | 6.2E-2 | 4.8E-1 | 1.4E-1 | 1.9E0 | 3.0E-1 | 1.0E-9 | 1.0E-9 | 1.9E1 | 2.5E0 | 218 | 139 | 218 | 139 | 0.43 |
| Oh | pg/ml | 2.2E0 | 4.5E0 | 1.6E1 | 1.4E2 | 1.1E2 | 1.3E3 | 1.0E-9 | 1.0E-9 | 1.4E3 | 1.6E4 | 218 | 139 | 218 | 139 | 0.61 |
| Oi | pg/ml | 1.3E0 | 2.6E0 | 4.6E0 | 5.6E0 | 8.1E0 | 7.4E0 | 1.0E-9 | 1.0E-9 | 6.7E1 | 4.2E1 | 218 | 139 | 218 | 139 | 0.56 |
| Ok | pg/ml | 3.2E2 | 5.8E2 | 4.2E2 | 8.7E2 | 4.1E2 | 1.1E3 | 2.9E1 | 1.5E1 | 2.8E3 | 7.8E3 | 218 | 139 | 218 | 139 | 0.70 |
| Om | pg/ml | 3.7E2 | 5.4E2 | 8.1E2 | 1.3E3 | 2.5E3 | 4.4E3 | 1.0E-9 | 1.0E-9 | 3.0E4 | 5.1E4 | 218 | 139 | 218 | 139 | 0.62 |
| On | pg/ml | 1.4E2 | 2.7E2 | 2.4E2 | 5.1E2 | 4.3E2 | 8.7E2 | 1.0E-9 | 1.0E1 | 4.5E3 | 8.5E3 | 218 | 139 | 218 | 139 | 0.68 |
| Or | pg/ml | 9.6E0 | 1.9E1 | 1.8E1 | 6.3E1 | 2.3E1 | 1.1E2 | 1.0E-9 | 1.0E-9 | 9.6E1 | 5.1E2 | 64 | 73 | 64 | 73 | 0.60 |
| Ow | pg/ml | 2.8E1 | 5.2E1 | 1.3E2 | 3.1E2 | 3.7E2 | 1.0E3 | 1.0E-9 | 1.0E-9 | 2.7E3 | 8.1E3 | 64 | 73 | 64 | 73 | 0.59 |
| Ou | pg/ml | 4.1E2 | 5.8E2 | 8.4E2 | 1.6E3 | 1.4E3 | 2.4E3 | 2.2E1 | 1.0E-9 | 9.8E3 | 1.1E4 | 64 | 73 | 64 | 73 | 0.59 |
| Ul | ng/ml | 1.0E-9 | 1.0E-9 | 1.4E0 | 1.5E0 | 5.4E0 | 7.4E0 | 1.0E-9 | 1.0E-9 | 2.9E1 | 5.6E1 | 66 | 70 | 66 | 70 | 0.49 |
| Uo | ng/ml | 1.0E-9 | 1.0E-9 | 1.0E-1 | 4.8E-2 | 2.6E-1 | 1.4E-1 | 1.0E-9 | 1.0E-9 | 1.3E0 | 6.9E-1 | 66 | 70 | 66 | 70 | 0.45 |
| Uk | ng/ml | 1.0E-9 | 1.0E-9 | 2.9E-3 | 8.9E-3 | 5.8E-3 | 4.2E-2 | 1.0E-9 | 1.0E-9 | 2.6E-2 | 3.2E-1 | 66 | 70 | 66 | 70 | 0.43 |
| Um | ng/ml | 1.0E-9 | 1.0E-9 | 2.9E-1 | 1.7E-1 | 7.1E-1 | 4.9E-1 | 1.0E-9 | 1.0E-9 | 3.6E0 | 2.3E0 | 66 | 70 | 66 | 70 | 0.42 |
| Uf | ng/ml | 5.1E-2 | 8.0E-2 | 1.0E-1 | 2.5E-1 | 1.4E-1 | 6.3E-1 | 2.7E-3 | 1.0E-3 | 7.0E-1 | 5.1E0 | 66 | 70 | 66 | 70 | 0.62 |
| Uh | ng/ml | 1.4E0 | 3.6E0 | 2.7E0 | 4.6E0 | 2.9E0 | 3.9E0 | 3.6E-2 | 4.7E-2 | 1.5E1 | 1.8E1 | 66 | 70 | 66 | 70 | 0.67 |
| Un | ng/ml | 1.5E0 | 2.1E0 | 1.7E0 | 2.7E0 | 1.0E0 | 3.1E0 | 3.5E-1 | 3.4E-1 | 4.9E0 | 2.5E1 | 66 | 70 | 66 | 70 | 0.64 |
| Ug | ng/ml | 1.3E1 | 8.8E0 | 2.1E1 | 2.3E1 | 1.9E1 | 3.2E1 | 1.5E0 | 1.0E0 | 8.5E1 | 1.6E2 | 66 | 70 | 66 | 70 | 0.44 |
| Ur | ng/ml | 1.5E-1 | 7.0E-2 | 2.9E-1 | 4.4E-1 | 4.5E-1 | 1.2E0 | 1.0E-9 | 1.0E-9 | 2.6E0 | 7.3E0 | 66 | 69 | 66 | 69 | 0.40 |
| Up | ng/ml | 1.0E-9 | 1.0E-9 | 2.3E-3 | 3.9E-2 | 8.0E-3 | 2.9E-1 | 1.0E-9 | 1.0E-9 | 5.3E-2 | 2.4E0 | 66 | 69 | 66 | 69 | 0.55 |
| Us | ng/ml | 1.9E-3 | 3.6E-3 | 8.0E-3 | 4.9E-2 | 1.2E-2 | 2.1E-1 | 1.0E-9 | 1.0E-9 | 6.1E-2 | 1.7E0 | 66 | 69 | 66 | 69 | 0.55 |
| Uv | ng/ml | 2.5E-3 | 3.0E-3 | 1.9E-2 | 1.4E-2 | 5.5E-2 | 5.2E-2 | 1.0E-9 | 1.0E-9 | 3.5E-1 | 4.1E-1 | 66 | 69 | 66 | 69 | 0.47 |
| Ut | ng/ml | 5.3E-1 | 9.1E-1 | 2.2E0 | 4.3E0 | 6.8E0 | 9.3E0 | 1.0E-9 | 1.0E-9 | 5.2E1 | 6.5E1 | 66 | 69 | 66 | 69 | 0.62 |
| Uu | ng/ml | 7.2E0 | 6.4E0 | 7.6E0 | 7.3E0 | 5.0E0 | 5.1E0 | 5.4E-1 | 5.7E-1 | 2.6E1 | 2.9E1 | 66 | 69 | 66 | 69 | 0.47 |
| Uw | ng/ml | 2.0E0 | 2.7E0 | 2.6E0 | 4.5E0 | 2.6E0 | 7.0E0 | 1.5E-1 | 1.0E-9 | 9.8E0 | 3.9E1 | 30 | 29 | 30 | 29 | 0.64 |
| Vb | ng/ml | 1.2E0 | 1.1E0 | 1.2E0 | 1.1E0 | 4.3E-1 | 1.1E0 | 4.7E-1 | 8.5E-2 | 2.0E0 | 6.4E0 | 30 | 29 | 30 | 29 | 0.36 |
| Vc | ng/ml | 1.0E-9 | 1.0E-9 | 6.7E-3 | 4.6E-4 | 2.4E-2 | 2.5E-3 | 1.0E-9 | 1.0E-9 | 1.1E-1 | 1.3E-2 | 30 | 29 | 30 | 29 | 0.47 |
| Uy | ng/ml | 1.3E0 | 1.4E0 | 4.4E0 | 1.1E1 | 1.1E1 | 2.4E1 | 8.7E-2 | 2.0E-2 | 5.1E1 | 9.9E1 | 30 | 29 | 30 | 29 | 0.55 |
| Uz | ng/ml | 1.0E-9 | 1.0E-9 | 1.5E-2 | 1.1E0 | 7.9E-2 | 6.1E0 | 1.0E-9 | 1.0E-9 | 4.4E-1 | 3.3E1 | 30 | 29 | 30 | 29 | 0.52 |
| Ux | ng/ml | 1.9E2 | 1.7E2 | 1.8E2 | 1.9E2 | 1.5E2 | 1.3E2 | 1.2E1 | 4.5E0 | 4.8E2 | 4.9E2 | 30 | 29 | 30 | 29 | 0.52 |
| Va | ng/ml | 1.4E1 | 5.7E0 | 2.5E1 | 2.0E1 | 3.1E1 | 2.4E1 | 3.1E-1 | 3.6E-1 | 1.2E2 | 9.6E1 | 30 | 29 | 30 | 29 | 0.44 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vh | ng/ml | 1.1E-2 | 1.7E-2 | 1.9E-2 | 4.9E-2 | 2.5E-2 | 1.6E-1 | 3.9E-4 | 1.0E-3 | 1.2E-1 | 8.6E-1 | 30 | 29 | 30 | 29 | 0.59 |
| Vi | ng/ml | 4.0E-3 | 5.3E-3 | 6.7E-3 | 8.1E-2 | 7.3E-3 | 3.4E-1 | 1.0E-9 | 1.0E-9 | 3.3E-2 | 1.8E0 | 30 | 29 | 30 | 29 | 0.59 |
| Vj | ng/ml | 2.2E1 | 5.9E1 | 4.5E1 | 9.4E1 | 4.7E1 | 1.3E2 | 4.6E0 | 1.4E0 | 1.8E2 | 6.5E2 | 30 | 28 | 30 | 28 | 0.62 |
| Vp | ng/ml | 1.0E-9 | 1.0E-9 | 1.5E-1 | 8.6E-1 | 7.2E-1 | 5.9E0 | 1.0E-9 | 1.0E-9 | 3.9E0 | 4.9E1 | 66 | 70 | 66 | 70 | 0.57 |
| Vt | ng/ml | 4.9E0 | 7.9E0 | 6.3E0 | 1.2E1 | 5.1E0 | 1.9E1 | 9.6E-1 | 5.6E-1 | 3.2E1 | 1.6E2 | 66 | 70 | 66 | 70 | 0.66 |
| Vu | ng/ml | 1.0E-9 | 1.3E-1 | 9.8E-1 | 2.2E0 | 2.4E0 | 4.0E0 | 1.0E-9 | 1.0E-9 | 1.6E1 | 2.2E1 | 64 | 68 | 64 | 68 | 0.59 |
| Vq | ng/ml | 1.0E2 | 2.9E2 | 5.6E2 | 9.0E2 | 1.0E3 | 1.9E3 | 9.2E-1 | 6.5E-1 | 5.0E3 | 1.2E4 | 55 | 53 | 55 | 53 | 0.59 |
| Vo | ng/ml | 2.6E1 | 2.6E1 | 2.4E1 | 2.5E1 | 4.5E0 | 6.5E0 | 9.7E0 | 1.9E0 | 3.5E1 | 4.8E1 | 66 | 70 | 66 | 70 | 0.51 |
| Vs | ng/ml | 1.0E-9 | 1.0E-9 | 4.9E0 | 1.1E1 | 1.3E1 | 5.6E1 | 1.0E-9 | 1.0E-9 | 8.9E1 | 4.5E2 | 66 | 67 | 66 | 67 | 0.47 |
| Vv | ng/ml | 2.2E0 | 3.6E0 | 5.6E0 | 6.5E0 | 1.1E1 | 8.9E0 | 1.0E-9 | 1.0E-9 | 8.4E1 | 4.4E1 | 66 | 69 | 66 | 69 | 0.53 |
| Vw | ng/ml | 3.6E1 | 4.1E1 | 3.3E1 | 3.7E1 | 1.7E1 | 2.0E1 | 3.1E0 | 2.5E0 | 6.7E1 | 6.9E1 | 30 | 29 | 30 | 29 | 0.57 |
| Oy | pg/ml | 4.7E-1 | 4.7E-1 | 7.7E0 | 3.5E0 | 3.7E1 | 1.1E1 | 1.0E-9 | 1.0E-9 | 4.0E2 | 9.9E1 | 218 | 138 | 218 | 138 | 0.48 |
| Oz | pg/ml | 6.7E-3 | 1.0E-9 | 2.5E-1 | 6.2E-1 | 3.9E-1 | 3.4E0 | 1.0E-9 | 1.0E-9 | 2.1E0 | 2.9E1 | 218 | 138 | 218 | 138 | 0.48 |
| Pa | pg/ml | 3.7E-1 | 5.1E-1 | 1.5E0 | 3.8E0 | 6.8E0 | 2.1E1 | 1.0E-9 | 1.0E-9 | 8.6E1 | 2.3E2 | 218 | 138 | 218 | 138 | 0.57 |
| Pb | pg/ml | 1.0E-9 | 1.0E-9 | 2.4E0 | 5.9E-1 | 3.3E1 | 3.6E0 | 1.0E-9 | 1.0E-9 | 4.9E2 | 3.2E1 | 218 | 138 | 218 | 138 | 0.47 |
| Pc | pg/ml | 4.8E-2 | 9.8E-3 | 4.2E-1 | 3.0E0 | 1.1E0 | 2.8E1 | 1.0E-9 | 1.0E-9 | 1.4E1 | 3.3E2 | 218 | 138 | 218 | 138 | 0.49 |
| Pd | pg/ml | 1.4E0 | 2.1E0 | 3.7E0 | 1.2E1 | 9.0E0 | 7.3E1 | 1.0E-9 | 1.0E-9 | 9.4E1 | 8.4E2 | 218 | 138 | 218 | 138 | 0.57 |
| Pe | pg/ml | 1.8E1 | 3.9E1 | 8.9E1 | 3.5E2 | 3.9E2 | 1.5E3 | 1.0E-9 | 1.0E-9 | 4.7E3 | 1.5E4 | 218 | 138 | 218 | 138 | 0.63 |
| Pf | pg/ml | 1.2E0 | 2.8E0 | 1.1E1 | 2.6E1 | 4.8E1 | 1.3E2 | 1.0E-9 | 1.0E-9 | 4.8E2 | 1.5E3 | 218 | 138 | 218 | 138 | 0.60 |
| Pg | pg/ml | 3.3E0 | 6.6E0 | 5.2E1 | 1.3E2 | 3.6E2 | 7.0E2 | 1.0E-9 | 1.0E-9 | 4.2E3 | 7.7E3 | 218 | 138 | 218 | 138 | 0.62 |
| Ph | ng/ml | 1.4E-1 | 1.8E-1 | 2.7E-1 | 4.9E-1 | 3.5E-1 | 8.3E-1 | 1.0E-9 | 1.0E-9 | 1.5E0 | 5.4E0 | 64 | 73 | 64 | 73 | 0.57 |
| Pi | ng/ml | 1.9E-1 | 2.2E-1 | 2.5E-1 | 1.5E0 | 4.1E-1 | 9.6E0 | 1.0E-9 | 1.0E-9 | 3.2E0 | 8.2E1 | 64 | 73 | 64 | 73 | 0.60 |
| Pj | ng/mL | 4.6E0 | 6.1E0 | 5.2E0 | 7.2E0 | 3.3E0 | 5.3E0 | 4.9E-1 | 4.0E-1 | 1.6E1 | 3.1E1 | 64 | 73 | 64 | 73 | 0.62 |
| Pk | ng/ml | 6.9E-3 | 1.1E-2 | 9.5E-3 | 4.1E-2 | 1.0E-2 | 1.8E-1 | 1.0E-9 | 1.0E-9 | 5.4E-2 | 1.5E0 | 64 | 73 | 64 | 73 | 0.63 |
| aA | mg/dL | 8.6E-1 | 1.0E0 | 9.7E-1 | 1.2E0 | 4.6E-1 | 7.9E-1 | 3.0E-1 | 3.0E-1 | 4.1E0 | 4.7E0 | 361 | 178 | 361 | 178 | 0.59 |
| aC | mg/mL | 2.7E0 | 2.1E0 | 2.9E0 | 2.3E0 | 1.4E0 | 1.2E0 | 1.1E0 | 7.5E-1 | 7.4E0 | 6.7E0 | 93 | 83 | 93 | 83 | 0.35 |
| aD | ug/mL | 2.8E0 | 3.3E0 | 4.7E0 | 4.6E0 | 5.4E0 | 3.8E0 | 7.5E-1 | 7.5E-1 | 3.5E1 | 2.1E1 | 93 | 83 | 93 | 83 | 0.51 |
| aE | mg/mL | 5.8E-1 | 5.7E-1 | 5.9E-1 | 6.0E-1 | 1.6E-1 | 1.8E-1 | 2.8E-1 | 1.8E-1 | 1.1E0 | 1.2E0 | 93 | 83 | 93 | 83 | 0.51 |
| aF | ng/mL | 2.2E0 | 2.3E0 | 5.2E0 | 4.9E0 | 8.4E0 | 6.7E0 | 4.3E-3 | 4.3E-3 | 5.0E1 | 3.5E1 | 93 | 83 | 93 | 83 | 0.52 |
| aG | mg/mL | 1.4E-1 | 1.3E-1 | 1.6E-1 | 1.5E-1 | 9.2E-2 | 8.0E-2 | 3.2E-2 | 5.1E-2 | 4.6E-1 | 4.8E-1 | 93 | 83 | 93 | 83 | 0.50 |
| aH | ug/mL | 6.9E1 | 7.1E1 | 7.6E1 | 7.8E1 | 3.9E1 | 4.2E1 | 8.9E0 | 1.1E1 | 2.0E2 | 2.0E2 | 93 | 83 | 93 | 83 | 0.50 |
| aI | ug/mL | 1.8E2 | 1.6E2 | 1.8E2 | 1.7E2 | 6.0E1 | 6.2E1 | 3.2E1 | 4.7E1 | 3.3E2 | 3.4E2 | 93 | 83 | 93 | 83 | 0.43 |
| aJ | ug/mL | 2.2E0 | 2.5E0 | 3.0E0 | 3.7E0 | 2.1E0 | 3.2E0 | 9.5E-1 | 8.2E-1 | 1.2E1 | 2.3E1 | 93 | 83 | 93 | 83 | 0.58 |
| aK | ng/mL | 1.5E0 | 1.1E0 | 2.2E0 | 1.7E0 | 2.0E0 | 1.8E0 | 2.8E-2 | 2.9E-4 | 9.1E0 | 1.0E1 | 93 | 83 | 93 | 83 | 0.42 |
| aL | mg/mL | 7.4E-1 | 7.4E-1 | 7.7E-1 | 7.5E-1 | 2.5E-1 | 2.6E-1 | 2.2E-1 | 2.7E-1 | 1.4E0 | 1.7E0 | 93 | 83 | 93 | 83 | 0.48 |
| aM | U/mL | 1.4E1 | 2.4E1 | 3.2E1 | 5.9E1 | 5.4E1 | 1.2E2 | 4.2E-2 | 4.2E-2 | 3.5E2 | 8.2E2 | 93 | 83 | 93 | 83 | 0.63 |
| aN | U/mL | 1.0E1 | 2.0E1 | 1.6E1 | 3.6E1 | 1.9E1 | 5.6E1 | 2.5E-3 | 2.5E-3 | 9.7E1 | 3.8E2 | 93 | 83 | 93 | 83 | 0.68 |
| aO | pg/mL | 4.5E1 | 7.4E1 | 4.6E2 | 4.2E2 | 1.1E3 | 7.7E2 | 6.0E-2 | 6.0E-2 | 6.6E3 | 3.9E3 | 93 | 83 | 93 | 83 | 0.55 |
| aP | ng/mL | 1.5E0 | 1.7E0 | 2.2E0 | 2.6E0 | 3.0E0 | 3.2E0 | 4.5E-1 | 6.8E-1 | 2.8E1 | 2.8E1 | 93 | 83 | 93 | 83 | 0.58 |
| aQ | ng/mL | 2.5E-1 | 2.4E-1 | 3.7E-1 | 3.4E-1 | 2.9E-1 | 3.5E-1 | 2.0E-4 | 2.0E-4 | 1.1E0 | 2.0E0 | 93 | 83 | 93 | 83 | 0.47 |
| aR | ng/mL | 1.7E0 | 2.1E0 | 2.6E0 | 3.5E0 | 3.4E0 | 4.6E0 | 2.6E-1 | 5.6E-1 | 2.1E1 | 3.4E1 | 93 | 83 | 93 | 83 | 0.59 |
| aS | ng/mL | 3.1E-1 | 5.1E-1 | 1.0E0 | 9.7E-1 | 3.5E0 | 1.2E0 | 4.2E-3 | 4.2E-3 | 3.3E1 | 6.2E0 | 93 | 83 | 93 | 83 | 0.60 |
| aU | pg/mL | 7.6E1 | 5.8E1 | 1.1E2 | 8.9E1 | 1.0E2 | 1.1E2 | 7.4E-2 | 7.4E-2 | 5.1E2 | 7.0E2 | 93 | 83 | 93 | 83 | 0.42 |
| aV | ng/mL | 6.3E-1 | 4.3E-1 | 9.5E-1 | 1.1E0 | 9.6E-1 | 3.6E0 | 3.8E-2 | 7.6E-4 | 5.2E0 | 3.3E1 | 93 | 83 | 93 | 83 | 0.42 |
| aW | pg/mL | 1.9E1 | 2.1E1 | 2.1E1 | 2.4E1 | 1.9E1 | 4.5E1 | 7.2E-2 | 7.2E-2 | 1.7E2 | 4.2E2 | 93 | 83 | 93 | 83 | 0.51 |
| aX | ng/mL | 8.9E0 | 7.4E0 | 1.5E1 | 1.8E1 | 2.6E1 | 3.8E1 | 3.0E-1 | 6.2E-1 | 2.2E2 | 3.1E2 | 93 | 83 | 93 | 83 | 0.48 |
| aY | pg/mL | 5.1E1 | 5.4E1 | 6.8E1 | 8.4E1 | 5.8E1 | 1.4E2 | 4.1E-1 | 4.1E-1 | 3.1E2 | 1.2E3 | 93 | 83 | 93 | 83 | 0.52 |
| aZ | pg/mL | 2.2E2 | 2.6E2 | 4.5E2 | 9.3E2 | 6.2E2 | 1.9E3 | 1.7E0 | 1.7E0 | 2.9E3 | 1.2E4 | 93 | 83 | 93 | 83 | 0.54 |
| bA | ng/mL | 9.5E0 | 2.5E1 | 3.9E1 | 1.1E2 | 8.9E1 | 2.3E2 | 3.0E-2 | 3.0E-2 | 6.8E2 | 1.5E3 | 93 | 83 | 93 | 83 | 0.65 |
| bB | ng/mL | 2.9E2 | 2.4E2 | 3.3E2 | 2.8E2 | 1.8E2 | 1.7E2 | 2.1E0 | 1.2E1 | 9.5E2 | 8.1E2 | 93 | 83 | 93 | 83 | 0.42 |
| bC | ng/mL | 3.2E2 | 3.3E2 | 5.4E2 | 7.8E2 | 6.8E2 | 1.1E3 | 1.4E1 | 3.5E1 | 4.0E3 | 4.7E3 | 93 | 83 | 93 | 83 | 0.53 |
| bE | mg/mL | 5.3E0 | 4.8E0 | 5.7E0 | 5.2E0 | 2.1E0 | 2.2E0 | 1.8E0 | 1.3E0 | 1.2E1 | 1.2E1 | 93 | 83 | 93 | 83 | 0.42 |
| bF | pg/mL | 3.5E1 | 4.3E1 | 2.5E2 | 4.5E2 | 1.2E3 | 1.5E3 | 5.0E-2 | 2.5E0 | 1.1E4 | 1.0E4 | 93 | 83 | 93 | 83 | 0.56 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|------|--------|------|-----|
| | | NonDis | Dis | NonDis | Dis | NonDis | Dis | NonDis | Dis | NonDis | Dis | NonDis | Dis | NonDis | Dis | |
| bG | ng/mL | 1.5E0 | 1.8E0 | 2.8E0 | 3.6E0 | 3.7E0 | 5.1E0 | 1.1E-1 | 1.1E-1 | 2.3E1 | 3.0E1 | 93 | 83 | 93 | 83 | 0.53 |
| bH | pg/mL | 5.7E-1 | 5.7E-1 | 6.7E0 | 5.8E0 | 2.9E1 | 1.5E1 | 5.7E-1 | 5.7E-1 | 2.8E2 | 1.2E2 | 93 | 83 | 93 | 83 | 0.50 |
| bI | ng/mL | 4.0E-3 | 4.0E-3 | 8.2E-2 | 1.0E-1 | 1.6E-1 | 2.3E-1 | 4.0E-3 | 4.0E-3 | 6.8E-1 | 9.8E-1 | 93 | 83 | 93 | 83 | 0.49 |
| bJ | mg/mL | 2.0E0 | 1.9E0 | 2.5E0 | 2.2E0 | 2.0E0 | 2.0E0 | 2.5E-4 | 2.5E-4 | 7.8E0 | 1.1E1 | 93 | 83 | 93 | 83 | 0.45 |
| bL | pg/mL | 4.3E0 | 3.2E0 | 1.0E1 | 8.0E0 | 1.3E1 | 9.7E0 | 4.6E-2 | 4.6E-2 | 6.0E1 | 3.5E1 | 93 | 83 | 93 | 83 | 0.44 |
| bM | mg/mL | 1.5E0 | 2.2E0 | 1.8E0 | 2.5E0 | 1.2E0 | 1.7E0 | 1.4E-1 | 1.6E-2 | 6.2E0 | 8.6E0 | 93 | 83 | 93 | 83 | 0.64 |
| bN | ng/mL | 3.5E1 | 3.1E1 | 1.4E2 | 1.0E2 | 3.1E2 | 2.6E2 | 1.4E-1 | 1.4E-1 | 1.9E3 | 1.9E3 | 93 | 83 | 93 | 83 | 0.48 |
| bO | ng/mL | 4.0E-2 | 4.0E-2 | 8.8E0 | 9.3E0 | 1.6E1 | 2.4E1 | 4.0E-2 | 4.0E-2 | 6.4E1 | 1.3E2 | 93 | 83 | 93 | 83 | 0.43 |
| bP | mg/mL | 5.2E-1 | 5.3E-1 | 7.4E-1 | 7.6E-1 | 6.2E-1 | 7.8E-1 | 4.9E-2 | 9.2E-2 | 3.7E0 | 4.8E0 | 93 | 83 | 93 | 83 | 0.49 |
| bQ | pg/mL | 2.1E1 | 2.3E1 | 4.3E1 | 2.4E2 | 6.8E1 | 1.5E3 | 1.5E-1 | 1.5E-1 | 4.8E2 | 1.3E4 | 93 | 83 | 93 | 83 | 0.54 |
| bR | ng/mL | 1.2E-2 | 1.2E-2 | 1.4E-1 | 1.8E-1 | 3.7E-1 | 9.5E-1 | 1.2E-2 | 1.2E-2 | 3.4E0 | 8.7E0 | 93 | 83 | 93 | 83 | 0.46 |
| bS | pg/mL | 9.4E-1 | 9.4E-1 | 9.2E0 | 9.9E0 | 3.7E1 | 4.4E1 | 9.4E-1 | 9.4E-1 | 3.4E2 | 3.9E2 | 93 | 83 | 93 | 83 | 0.48 |
| bU | ng/mL | 9.2E-2 | 1.3E-2 | 1.8E-1 | 2.0E-1 | 2.5E-1 | 7.3E-1 | 1.3E-2 | 1.3E-2 | 1.8E0 | 6.6E0 | 93 | 83 | 93 | 83 | 0.43 |
| bV | pg/mL | 4.4E2 | 5.3E2 | 6.4E2 | 6.3E2 | 1.2E3 | 3.8E2 | 1.6E2 | 2.3E2 | 1.2E4 | 2.2E3 | 93 | 83 | 93 | 83 | 0.59 |
| bW | pg/mL | 3.1E2 | 3.3E2 | 4.8E2 | 5.7E2 | 4.4E2 | 8.1E2 | 8.4E1 | 1.1E2 | 2.4E3 | 4.8E3 | 93 | 83 | 93 | 83 | 0.53 |
| bX | ng/mL | 1.8E-3 | 2.5E-5 | 3.0E-3 | 2.2E-3 | 3.5E-3 | 2.7E-3 | 2.5E-5 | 2.5E-5 | 1.3E-2 | 8.4E-3 | 93 | 83 | 93 | 83 | 0.45 |
| bZ | pg/mL | 2.7E2 | 3.1E2 | 1.6E3 | 2.5E3 | 5.4E3 | 8.5E3 | 1.5E-1 | 1.5E-1 | 4.4E4 | 5.8E4 | 93 | 83 | 93 | 83 | 0.51 |
| cA | pg/mL | 6.0E-1 | 6.0E-1 | 2.0E0 | 6.4E0 | 3.5E0 | 4.1E1 | 6.0E-1 | 6.0E-1 | 1.5E1 | 3.7E2 | 93 | 83 | 93 | 83 | 0.49 |
| cB | ng/mL | 5.7E-2 | 3.5E-2 | 8.1E-2 | 6.7E-2 | 8.4E-2 | 9.2E-2 | 1.7E-3 | 1.7E-3 | 3.8E-1 | 4.3E-1 | 93 | 83 | 93 | 83 | 0.41 |
| cC | pg/mL | 4.6E1 | 3.9E1 | 4.8E1 | 4.2E1 | 4.7E1 | 5.3E1 | 1.0E0 | 1.0E0 | 3.7E2 | 4.5E2 | 93 | 83 | 93 | 83 | 0.44 |
| cD | pg/mL | 6.1E0 | 3.8E0 | 1.4E1 | 1.2E1 | 5.2E1 | 3.7E1 | 3.3E-1 | 3.3E-1 | 4.8E2 | 2.9E2 | 93 | 83 | 93 | 83 | 0.37 |
| cE | pg/mL | 4.9E1 | 7.0E1 | 2.5E2 | 2.8E2 | 5.9E2 | 5.9E2 | 1.2E-1 | 1.2E-1 | 3.1E3 | 3.8E3 | 93 | 83 | 93 | 83 | 0.56 |
| cF | pg/mL | 9.4E0 | 5.3E-1 | 1.8E1 | 1.3E1 | 2.7E1 | 3.2E1 | 5.3E-1 | 5.3E-1 | 1.4E2 | 2.7E2 | 93 | 83 | 93 | 83 | 0.42 |
| cG | pg/mL | 5.0E1 | 6.4E1 | 9.7E1 | 2.6E2 | 1.6E2 | 1.1E3 | 1.1E1 | 7.8E0 | 1.1E3 | 1.0E4 | 93 | 83 | 93 | 83 | 0.60 |
| cH | uIU/mL | 3.5E0 | 3.1E0 | 7.2E0 | 8.1E0 | 1.8E1 | 1.6E1 | 8.6E-3 | 8.6E-3 | 1.6E2 | 1.2E2 | 93 | 83 | 93 | 83 | 0.49 |
| cI | ng/mL | 6.0E0 | 6.4E0 | 1.2E1 | 1.7E1 | 1.6E1 | 2.5E1 | 2.3E-1 | 3.2E-2 | 1.0E2 | 1.2E2 | 93 | 83 | 93 | 83 | 0.52 |
| cJ | ug/mL | 7.4E1 | 5.1E1 | 1.1E2 | 8.8E1 | 1.0E2 | 1.0E2 | 6.9E0 | 5.6E0 | 6.4E2 | 6.2E2 | 93 | 83 | 93 | 83 | 0.43 |
| cK | ng/mL | 3.8E-3 | 3.8E-3 | 1.9E-2 | 2.7E-2 | 4.6E-2 | 1.6E-1 | 3.8E-3 | 3.8E-3 | 3.4E-1 | 1.5E0 | 93 | 83 | 93 | 83 | 0.48 |
| cL | pg/mL | 2.2E2 | 2.1E2 | 3.6E2 | 7.4E2 | 8.0E2 | 2.8E3 | 4.0E1 | 3.1E1 | 7.1E3 | 2.4E4 | 93 | 83 | 93 | 83 | 0.54 |
| cM | pg/mL | 2.8E2 | 2.6E2 | 3.1E2 | 2.6E2 | 1.9E2 | 1.2E2 | 2.5E1 | 4.2E1 | 1.1E3 | 6.7E2 | 93 | 83 | 93 | 83 | 0.43 |
| cN | pg/mL | 1.2E2 | 1.2E2 | 1.2E2 | 1.4E2 | 4.9E1 | 1.1E2 | 3.8E1 | 6.3E1 | 3.2E2 | 1.1E3 | 93 | 83 | 93 | 83 | 0.56 |
| cO | pg/mL | 2.1E2 | 2.1E2 | 3.1E2 | 5.2E2 | 2.9E2 | 2.1E3 | 5.4E1 | 8.2E1 | 1.7E3 | 1.9E4 | 93 | 83 | 93 | 83 | 0.50 |
| cP | ng/mL | 2.3E3 | 2.4E3 | 2.5E3 | 2.6E3 | 1.0E3 | 9.0E2 | 6.2E2 | 1.0E3 | 5.6E3 | 4.7E3 | 93 | 83 | 93 | 83 | 0.53 |
| cQ | ng/mL | 4.9E-2 | 5.3E-2 | 1.2E-1 | 1.3E-1 | 2.0E-1 | 2.2E-1 | 2.0E-3 | 2.0E-3 | 1.2E0 | 1.3E0 | 93 | 83 | 93 | 83 | 0.53 |
| cR | ng/mL | 3.5E2 | 3.2E2 | 5.3E2 | 6.8E2 | 4.8E2 | 1.1E3 | 3.6E1 | 2.0E1 | 2.9E3 | 7.7E3 | 93 | 83 | 93 | 83 | 0.49 |
| cS | ng/mL | 2.7E2 | 3.1E2 | 4.5E2 | 5.2E2 | 5.1E2 | 8.7E2 | 4.1E1 | 9.1E1 | 2.5E3 | 7.1E3 | 93 | 83 | 93 | 83 | 0.54 |
| cT | ng/mL | 3.7E1 | 6.5E1 | 9.5E1 | 2.3E2 | 1.4E2 | 4.1E2 | 3.6E0 | 4.2E0 | 8.4E2 | 2.1E3 | 93 | 83 | 93 | 83 | 0.61 |
| cU | ng/mL | 5.6E1 | 7.5E1 | 7.7E1 | 1.2E2 | 8.8E1 | 1.9E2 | 6.2E0 | 9.0E0 | 7.7E2 | 1.6E3 | 93 | 83 | 93 | 83 | 0.59 |
| cV | ng/mL | 1.7E-1 | 2.2E-1 | 8.3E-1 | 6.0E-1 | 4.9E0 | 1.5E0 | 2.5E-2 | 3.0E-2 | 4.7E1 | 9.7E0 | 93 | 83 | 93 | 83 | 0.52 |
| cW | mIU/mL | 4.9E-2 | 4.5E-2 | 1.1E-1 | 6.9E-2 | 4.6E-1 | 6.4E-2 | 4.8E-3 | 4.8E-3 | 4.5E0 | 3.9E-1 | 93 | 83 | 93 | 83 | 0.51 |
| cX | ng/mL | 8.9E-2 | 2.0E-1 | 1.5E0 | 2.2E0 | 5.0E0 | 6.2E0 | 2.3E-4 | 2.3E-4 | 2.8E1 | 2.8E1 | 93 | 83 | 93 | 83 | 0.55 |
| cY | ng/mL | 7.6E0 | 6.7E0 | 1.1E1 | 1.0E1 | 9.8E0 | 1.2E1 | 2.2E-1 | 1.7E-1 | 4.1E1 | 6.1E1 | 93 | 83 | 93 | 83 | 0.45 |
| cZ | ug/mL | 1.3E1 | 1.2E1 | 1.5E1 | 1.4E1 | 7.0E0 | 6.5E0 | 2.3E0 | 2.8E0 | 4.6E1 | 3.0E1 | 93 | 83 | 93 | 83 | 0.46 |
| dA | pg/mL | 3.1E2 | 3.2E2 | 3.5E2 | 4.3E2 | 1.8E2 | 6.3E2 | 1.0E2 | 1.1E2 | 1.3E3 | 5.8E3 | 93 | 83 | 93 | 83 | 0.51 |
| dB | ug/mL | 1.7E1 | 2.0E1 | 1.9E1 | 1.7E1 | 2.6E1 | 1.0E1 | 2.1E0 | 2.1E0 | 2.5E2 | 4.0E1 | 93 | 83 | 93 | 83 | 0.54 |
| dC | nmol/L | 3.4E1 | 3.2E1 | 3.8E1 | 3.6E1 | 1.8E1 | 1.5E1 | 1.0E1 | 7.8E0 | 1.4E2 | 9.1E1 | 93 | 83 | 93 | 83 | 0.48 |
| dD | ug/mL | 3.5E1 | 3.2E1 | 3.7E1 | 3.3E1 | 1.1E1 | 1.1E1 | 1.4E1 | 1.4E1 | 7.4E1 | 6.0E1 | 93 | 83 | 93 | 83 | 0.40 |
| dE | ng/mL | 5.5E-1 | 3.9E-1 | 6.1E-1 | 5.4E-1 | 5.8E-1 | 6.3E-1 | 8.4E-3 | 8.4E-3 | 2.7E0 | 2.9E0 | 93 | 83 | 93 | 83 | 0.45 |
| dF | ng/mL | 2.3E2 | 3.0E2 | 2.9E2 | 4.1E2 | 2.0E2 | 2.9E2 | 7.5E1 | 7.5E1 | 1.2E3 | 1.3E3 | 93 | 83 | 93 | 83 | 0.63 |
| dG | ng/mL | 1.1E1 | 1.4E1 | 1.5E1 | 1.9E1 | 1.4E1 | 2.3E1 | 3.2E0 | 3.0E0 | 9.7E1 | 1.8E2 | 93 | 83 | 93 | 83 | 0.58 |
| dH | pg/mL | 7.7E0 | 8.5E0 | 1.8E1 | 2.3E1 | 4.5E1 | 7.6E1 | 4.0E-2 | 4.0E-2 | 3.1E2 | 6.7E2 | 93 | 83 | 93 | 83 | 0.55 |
| dI | pg/mL | 4.6E-1 | 4.6E-1 | 1.9E0 | 5.9E0 | 5.7E0 | 3.6E1 | 4.6E-1 | 4.6E-1 | 4.2E1 | 3.3E2 | 93 | 83 | 93 | 83 | 0.55 |
| dJ | ng/mL | 2.0E0 | 2.1E0 | 2.2E0 | 2.1E0 | 1.1E0 | 1.2E0 | 3.2E-2 | 3.2E-2 | 5.6E0 | 4.9E0 | 93 | 83 | 93 | 83 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dK | uIU/mL | 1.5E0 | 1.1E0 | 2.5E0 | 1.8E0 | 4.5E0 | 1.9E0 | 2.8E-4 | 1.4E-2 | 3.9E1 | 1.1E1 | 93 | 83 | 93 | 83 | 0.45 |
| dL | ng/mL | 8.2E2 | 9.5E2 | 9.6E2 | 1.2E3 | 5.2E2 | 7.1E2 | 3.4E2 | 2.8E2 | 3.4E3 | 4.8E3 | 93 | 83 | 93 | 83 | 0.61 |
| dM | pg/mL | 9.5E2 | 1.0E3 | 1.3E3 | 1.5E3 | 1.6E3 | 1.4E3 | 3.9E2 | 3.7E2 | 1.5E4 | 9.6E3 | 93 | 83 | 93 | 83 | 0.56 |
| dN | ug/mL | 9.6E1 | 1.0E2 | 1.0E2 | 1.1E2 | 4.2E1 | 4.5E1 | 2.5E1 | 2.4E1 | 2.8E2 | 3.3E2 | 93 | 83 | 93 | 83 | 0.56 |
| dR | pg/ml | 1.8E3 | 1.2E3 | 2.2E3 | 1.9E3 | 1.8E3 | 2.2E3 | 2.8E2 | 1.3E2 | 7.8E3 | 9.8E3 | 60 | 73 | 60 | 73 | 0.38 |
| dU | pg/ml | 7.4E3 | 1.4E4 | 1.0E4 | 1.8E4 | 8.7E3 | 1.9E4 | 3.4E3 | 6.9E2 | 3.5E4 | 8.1E4 | 12 | 21 | 12 | 21 | 0.65 |
| dX | ng/ml | 5.2E-2 | 8.2E-2 | 1.4E-1 | 1.2E-1 | 2.2E-1 | 1.5E-1 | 2.6E-3 | 2.6E-3 | 1.2E0 | 6.6E-1 | 35 | 26 | 35 | 26 | 0.50 |
| dW | ng/ml | 1.2E-1 | 1.7E-1 | 1.7E-1 | 2.7E-1 | 1.6E-1 | 2.5E-1 | 6.4E-2 | 6.8E-2 | 5.8E-1 | 8.0E-1 | 9 | 10 | 9 | 10 | 0.62 |
| eF | ng/ml | 4.2E0 | 4.1E0 | 5.6E0 | 5.2E0 | 5.8E0 | 3.6E0 | 2.0E0 | 2.0E0 | 4.6E1 | 2.9E1 | 60 | 73 | 60 | 73 | 0.50 |
| eC | pg/ml | 3.0E2 | 2.8E2 | 3.8E2 | 3.5E2 | 2.9E2 | 3.1E2 | 9.9E0 | 1.9E1 | 1.4E3 | 2.0E3 | 50 | 67 | 50 | 67 | 0.44 |
| eD | pg/ml | 2.3E2 | 1.8E2 | 7.6E2 | 6.4E2 | 1.8E3 | 1.3E3 | 5.2E-1 | 5.2E-1 | 8.3E3 | 7.0E3 | 46 | 51 | 46 | 51 | 0.44 |
| eO | ng/ml | 4.6E1 | 9.4E1 | 3.2E2 | 1.2E2 | 4.3E2 | 8.7E1 | 2.0E1 | 4.1E1 | 9.9E2 | 3.3E2 | 9 | 10 | 9 | 10 | 0.61 |
| eM | ng/ml | 2.8E0 | 2.8E0 | 4.2E0 | 5.8E0 | 4.5E0 | 8.1E0 | 7.6E-1 | 6.9E-1 | 2.7E1 | 3.9E1 | 42 | 37 | 42 | 37 | 0.51 |
| eP | ng/ml | 3.7E-3 | 3.7E-3 | 7.3E-1 | 1.8E0 | 1.7E0 | 5.7E0 | 3.7E-3 | 3.7E-3 | 8.6E0 | 2.8E1 | 35 | 26 | 35 | 26 | 0.49 |
| eT | ng/ml | 2.7E2 | 2.9E2 | 7.1E2 | 7.8E2 | 8.4E2 | 8.9E2 | 1.0E2 | 7.1E1 | 2.9E3 | 2.9E3 | 28 | 27 | 28 | 27 | 0.55 |
| eQ | pg/ml | 1.0E0 | 1.0E0 | 8.5E1 | 3.4E1 | 1.5E2 | 6.4E1 | 1.0E0 | 1.0E0 | 4.7E2 | 2.2E2 | 12 | 21 | 12 | 21 | 0.45 |
| eW | U/ml | 1.1E-2 | 6.7E-3 | 6.2E-2 | 2.0E-1 | 9.7E-2 | 4.9E-1 | 6.7E-3 | 6.7E-3 | 3.1E-1 | 1.6E0 | 9 | 10 | 9 | 10 | 0.43 |
| fA | ng/ml | 1.9E2 | 2.3E2 | 3.6E2 | 4.9E2 | 4.8E2 | 4.7E2 | 3.9E1 | 4.0E1 | 1.5E3 | 1.4E3 | 12 | 19 | 12 | 19 | 0.60 |
| eZ | ng/ml | 4.8E1 | 5.6E1 | 5.7E1 | 5.6E1 | 2.8E1 | 2.3E1 | 2.3E1 | 1.8E1 | 1.2E2 | 1.1E2 | 28 | 27 | 28 | 27 | 0.52 |
| fB | ng/ml | 5.6E2 | 6.1E2 | 6.6E2 | 6.6E2 | 2.7E2 | 2.7E2 | 3.1E2 | 2.6E2 | 1.3E3 | 1.3E3 | 12 | 19 | 12 | 19 | 0.51 |
| fN | ng/ml | 2.1E-1 | 2.1E-1 | 4.4E0 | 3.4E0 | 1.1E1 | 5.6E0 | 2.1E-1 | 2.1E-1 | 5.4E1 | 2.1E1 | 28 | 27 | 28 | 27 | 0.49 |
| fP | ng/ml | 2.6E2 | 2.8E2 | 3.2E2 | 3.3E2 | 2.0E2 | 1.7E2 | 8.9E1 | 1.8E0 | 1.0E3 | 9.5E2 | 57 | 70 | 57 | 70 | 0.54 |
| fR | ng/ml | 1.3E5 | 1.8E5 | 2.0E5 | 2.5E5 | 1.6E5 | 2.0E5 | 3.9E4 | 1.9E2 | 6.9E5 | 8.7E5 | 63 | 52 | 63 | 52 | 0.59 |
| fY | ng/ml | 2.6E2 | 2.5E2 | 2.4E2 | 2.6E2 | 9.9E1 | 1.1E2 | 6.5E1 | 3.6E1 | 3.9E2 | 4.8E2 | 28 | 27 | 28 | 27 | 0.52 |
| gC | ng/ml | 2.5E2 | 2.4E2 | 2.8E2 | 2.5E2 | 1.2E2 | 1.2E2 | 1.4E2 | 8.3E1 | 6.4E2 | 5.9E2 | 22 | 25 | 22 | 25 | 0.42 |
| gL | pg/ml | 6.7E4 | 6.6E4 | 7.4E4 | 7.6E4 | 3.6E4 | 3.9E4 | 1.4E4 | 1.1E4 | 1.9E5 | 2.2E5 | 60 | 73 | 60 | 73 | 0.50 |
| gP | U/ml | 2.7E2 | 2.8E2 | 2.9E2 | 3.0E2 | 1.1E2 | 1.3E2 | 1.3E2 | 1.2E1 | 8.0E2 | 1.1E3 | 60 | 72 | 60 | 72 | 0.53 |
| gW | ng/ml | 6.6E2 | 3.8E2 | 1.2E3 | 6.8E2 | 1.2E3 | 8.1E2 | 6.8E1 | 2.3E0 | 6.1E3 | 4.2E3 | 48 | 51 | 48 | 51 | 0.37 |
| gV | ng/ml | 2.1E1 | 2.2E1 | 2.1E1 | 2.2E1 | 6.9E0 | 8.3E0 | 1.0E1 | 8.1E-2 | 3.7E1 | 3.4E1 | 23 | 15 | 23 | 15 | 0.58 |
| lF | pg/mL | 9.5E2 | 1.3E3 | 1.3E4 | 1.1E4 | 4.4E4 | 3.5E4 | 1.8E1 | 1.2E1 | 2.8E5 | 2.5E5 | 50 | 69 | 50 | 69 | 0.53 |
| gZ | ug/ml | 5.2E-1 | 9.0E-1 | 3.5E1 | 4.4E1 | 1.2E2 | 1.0E2 | 8.7E-2 | 1.4E-1 | 4.1E2 | 4.0E2 | 12 | 21 | 12 | 21 | 0.68 |
| hA | ng/ml | 2.4E0 | 2.9E0 | 1.5E1 | 1.4E1 | 5.6E1 | 4.3E1 | 1.7E-2 | 1.7E-2 | 3.5E2 | 2.9E2 | 46 | 52 | 46 | 52 | 0.55 |
| nM | pg/ml | 1.0E-9 | 1.0E-9 | 1.0E-9 | 3.3E1 | 0.0E0 | 2.2E2 | 1.0E-9 | 1.0E-9 | 1.0E-9 | 1.5E3 | 30 | 46 | 30 | 46 | 0.51 |
| nN | pg/ml | 1.2E3 | 2.1E3 | 1.7E3 | 9.4E3 | 1.8E3 | 2.7E4 | 8.1E1 | 2.3E2 | 7.1E3 | 1.5E5 | 30 | 46 | 30 | 46 | 0.66 |
| nO | pg/ml | 2.2E1 | 2.6E1 | 2.8E1 | 4.5E1 | 1.9E1 | 5.6E1 | 8.8E0 | 4.0E0 | 9.5E1 | 3.1E2 | 30 | 46 | 30 | 46 | 0.59 |
| nR | pg/ml | 1.6E1 | 1.8E1 | 4.7E1 | 1.4E2 | 7.1E1 | 3.5E2 | 1.7E0 | 1.0E0 | 2.6E2 | 1.9E3 | 30 | 46 | 30 | 46 | 0.55 |
| nT | pg/ml | 6.5E1 | 7.9E1 | 9.1E1 | 1.3E2 | 7.3E1 | 1.7E2 | 1.0E-9 | 1.0E-9 | 2.5E2 | 9.2E2 | 30 | 46 | 30 | 46 | 0.54 |
| nU | pg/ml | 4.4E1 | 4.4E1 | 5.3E1 | 1.4E2 | 6.0E1 | 2.8E2 | 1.0E-9 | 1.0E-9 | 2.0E2 | 1.5E3 | 30 | 46 | 30 | 46 | 0.55 |
| lW | pg/ml | 1.0E-9 | 1.0E-9 | 6.0E0 | 1.4E1 | 1.4E1 | 3.6E1 | 1.0E-9 | 1.0E-9 | 5.5E1 | 1.7E2 | 30 | 46 | 30 | 46 | 0.54 |
| lX | pg/ml | 1.0E3 | 8.1E2 | 1.1E3 | 9.4E2 | 5.8E2 | 5.6E2 | 3.2E2 | 1.9E2 | 2.6E3 | 2.5E3 | 30 | 46 | 30 | 46 | 0.42 |
| lY | pg/ml | 1.8E1 | 2.0E1 | 1.9E1 | 2.1E1 | 1.2E1 | 2.0E1 | 1.0E-9 | 1.0E-9 | 4.8E1 | 1.2E2 | 30 | 46 | 30 | 46 | 0.51 |
| mE | pg/ml | 1.0E-9 | 1.0E-9 | 1.4E0 | 3.3E0 | 2.4E0 | 9.3E0 | 1.0E-9 | 1.0E-9 | 7.8E0 | 5.7E1 | 30 | 46 | 30 | 46 | 0.55 |
| mF | pg/ml | 1.0E-9 | 5.7E-1 | 1.4E0 | 9.9E0 | 2.4E0 | 3.9E1 | 1.0E-9 | 1.0E-9 | 9.5E0 | 2.5E2 | 30 | 46 | 30 | 46 | 0.60 |
| mH | pg/ml | 4.3E0 | 2.7E0 | 4.8E0 | 5.6E0 | 5.6E0 | 9.0E0 | 1.1E0 | 4.0E-1 | 3.2E1 | 5.3E1 | 30 | 46 | 30 | 46 | 0.48 |
| mI | pg/ml | 1.0E-9 | 3.0E0 | 6.0E0 | 2.7E1 | 1.1E1 | 7.3E1 | 1.0E-9 | 1.0E-9 | 3.7E1 | 4.6E2 | 30 | 46 | 30 | 46 | 0.60 |
| mM | pg/ml | 2.5E1 | 4.5E1 | 5.3E1 | 1.0E2 | 6.1E1 | 1.9E2 | 1.0E-9 | 1.0E-9 | 2.1E2 | 1.1E3 | 30 | 46 | 30 | 46 | 0.56 |
| mP | pg/ml | 1.6E1 | 1.5E1 | 1.6E1 | 3.8E1 | 1.1E1 | 1.2E2 | 1.0E-9 | 1.0E-9 | 5.8E1 | 8.1E2 | 29 | 46 | 29 | 46 | 0.52 |
| mS | pg/ml | 1.6E3 | 1.5E3 | 1.8E3 | 1.7E3 | 8.9E2 | 1.1E3 | 8.8E1 | 1.0E-9 | 3.7E3 | 5.1E3 | 30 | 46 | 30 | 46 | 0.44 |
| mT | pg/ml | 5.0E1 | 6.0E1 | 1.5E2 | 1.5E2 | 2.8E2 | 3.0E2 | 1.0E1 | 1.2E1 | 1.4E3 | 1.7E3 | 29 | 46 | 29 | 46 | 0.49 |
| mU | pg/ml | 2.3E0 | 2.1E0 | 2.8E0 | 8.4E0 | 1.8E0 | 3.3E1 | 1.0E-9 | 1.0E-9 | 8.6E0 | 2.2E2 | 29 | 46 | 29 | 46 | 0.50 |
| mW | pg/ml | 2.3E3 | 2.0E3 | 2.4E3 | 2.7E3 | 1.1E3 | 2.3E3 | 3.1E2 | 1.0E-9 | 4.9E3 | 1.1E4 | 29 | 46 | 29 | 46 | 0.46 |
| mY | pg/ml | 6.2E2 | 6.6E2 | 8.0E2 | 1.1E3 | 8.6E2 | 1.4E3 | 1.0E-9 | 1.0E-9 | 4.2E3 | 8.0E3 | 30 | 46 | 30 | 46 | 0.55 |
| mZ | pg/ml | 1.8E2 | 1.7E2 | 4.1E2 | 3.5E2 | 5.9E2 | 3.9E2 | 1.0E-9 | 1.1E1 | 3.1E3 | 1.5E3 | 29 | 46 | 29 | 46 | 0.46 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| nA | pg/ml | 1.5E0 | 2.3E0 | 6.0E0 | 7.6E0 | 1.1E1 | 1.5E1 | 1.0E-9 | 1.0E-9 | 5.4E1 | 6.5E1 | 29 | 46 | 29 | 46 | 0.50 |
| nB | pg/ml | 2.7E2 | 3.0E2 | 2.9E2 | 3.3E2 | 1.3E2 | 2.0E2 | 3.0E1 | 3.8E1 | 5.9E2 | 9.6E2 | 30 | 46 | 30 | 46 | 0.53 |
| nC | pg/ml | 1.0E-9 | 8.3E1 | 6.3E2 | 1.4E4 | 2.1E3 | 6.4E4 | 1.0E-9 | 1.0E-9 | 1.1E4 | 3.8E5 | 30 | 46 | 30 | 46 | 0.60 |
| nD | pg/ml | 8.5E0 | 6.6E0 | 8.8E0 | 1.7E1 | 7.5E0 | 4.1E1 | 1.0E-9 | 1.0E-9 | 3.3E1 | 2.6E2 | 29 | 46 | 29 | 46 | 0.48 |
| nF | pg/ml | 1.0E-9 | 1.0E-9 | 3.5E0 | 2.8E0 | 1.7E1 | 9.5E0 | 1.0E-9 | 1.0E-9 | 9.3E1 | 4.7E1 | 30 | 46 | 30 | 46 | 0.51 |
| nH | pg/ml | 1.0E-9 | 2.8E0 | 3.2E0 | 2.9E2 | 5.4E0 | 1.5E3 | 1.0E-9 | 1.0E-9 | 1.7E1 | 1.0E4 | 29 | 46 | 29 | 46 | 0.61 |
| nI | pg/ml | 4.8E1 | 1.0E-9 | 6.8E1 | 6.8E1 | 7.0E1 | 1.9E2 | 1.0E-9 | 1.0E-9 | 2.0E2 | 1.2E3 | 30 | 46 | 30 | 46 | 0.39 |
| nJ | pg/ml | 2.5E-1 | 1.7E-1 | 8.1E-1 | 4.1E0 | 1.1E0 | 2.0E1 | 1.0E-9 | 1.0E-9 | 4.9E0 | 1.3E2 | 30 | 46 | 30 | 46 | 0.49 |
| nK | pg/ml | 1.0E-9 | 1.0E-9 | 9.9E0 | 2.0E1 | 2.1E1 | 3.9E1 | 1.0E-9 | 1.0E-9 | 7.5E1 | 2.2E2 | 29 | 46 | 29 | 46 | 0.60 |
| nL | pg/ml | 1.0E-9 | 4.3E0 | 8.2E0 | 4.4E2 | 1.8E1 | 2.1E3 | 1.0E-9 | 1.0E-9 | 8.1E1 | 1.4E4 | 30 | 46 | 30 | 46 | 0.63 |
| hL | pg/ml | 1.6E4 | 1.9E4 | 2.2E4 | 2.3E4 | 1.7E4 | 1.3E4 | 2.6E3 | 5.1E3 | 7.2E4 | 6.0E4 | 28 | 27 | 28 | 27 | 0.56 |
| hO | pg/ml | 1.6E4 | 1.6E4 | 1.6E4 | 1.6E4 | 2.8E3 | 2.3E3 | 1.3E4 | 1.1E4 | 2.4E4 | 2.1E4 | 28 | 27 | 28 | 27 | 0.41 |
| hP | ng/ml | 4.4E5 | 4.4E5 | 3.8E5 | 5.9E5 | 1.8E5 | 6.3E5 | 4.7E4 | 1.7E4 | 9.0E5 | 2.8E6 | 28 | 27 | 28 | 27 | 0.60 |
| wJ | pg/ml | 1.4E5 | 1.1E5 | 1.5E5 | 1.8E5 | 7.5E4 | 1.4E5 | 1.1E4 | 1.3E4 | 3.0E5 | 5.8E5 | 26 | 31 | 26 | 31 | 0.51 |
| wK | pg/ml | 3.2E4 | 3.6E4 | 4.1E4 | 5.6E4 | 3.1E4 | 8.7E4 | 3.7E3 | 7.5E3 | 1.3E5 | 5.0E5 | 26 | 31 | 26 | 31 | 0.52 |
| wL | pg/ml | 5.6E0 | 6.7E0 | 5.4E1 | 4.6E1 | 1.7E2 | 1.1E2 | 1.0E-9 | 1.0E-9 | 8.4E2 | 4.7E2 | 26 | 31 | 26 | 31 | 0.48 |
| wP | pg/ml | 2.3E4 | 2.8E4 | 3.4E4 | 6.0E4 | 3.2E4 | 7.0E4 | 1.1E3 | 1.3E3 | 1.6E5 | 3.0E5 | 26 | 31 | 26 | 31 | 0.58 |
| wQ | pg/ml | 2.5E1 | 3.9E1 | 5.6E1 | 6.4E1 | 8.4E1 | 9.9E1 | 1.0E-9 | 1.0E-9 | 3.7E2 | 5.1E2 | 26 | 31 | 26 | 31 | 0.57 |
| hR | pg/ml | 3.0E4 | 2.4E4 | 3.2E4 | 2.6E4 | 1.2E4 | 1.0E4 | 1.2E4 | 1.0E-9 | 5.8E4 | 4.9E4 | 43 | 48 | 43 | 48 | 0.35 |
| hV | pg/ml | 5.3E2 | 3.7E2 | 5.0E2 | 4.1E2 | 2.4E2 | 2.1E2 | 1.3E2 | 1.0E-9 | 1.2E3 | 9.6E2 | 43 | 48 | 43 | 48 | 0.39 |
| hW | pg/ml | 1.7E3 | 1.9E3 | 2.0E3 | 3.0E3 | 1.1E3 | 5.6E3 | 7.1E2 | 1.0E-9 | 7.3E3 | 4.0E4 | 43 | 48 | 43 | 48 | 0.58 |
| hX | pg/ml | 1.1E3 | 1.0E3 | 1.2E3 | 1.0E3 | 1.2E3 | 5.3E2 | 3.3E2 | 2.5E0 | 8.6E3 | 2.9E3 | 43 | 48 | 43 | 48 | 0.46 |
| iA | pg/ml | 1.8E2 | 1.5E2 | 2.8E2 | 2.4E2 | 3.3E2 | 2.2E2 | 1.6E1 | 1.5E1 | 1.8E3 | 8.7E2 | 50 | 69 | 50 | 69 | 0.47 |
| iB | ng/ml | 4.6E0 | 5.8E0 | 5.6E0 | 7.5E0 | 4.3E0 | 5.5E0 | 3.3E-2 | 8.3E-1 | 1.9E1 | 2.4E1 | 46 | 52 | 46 | 52 | 0.63 |
| iC | U/ml | 2.5E-1 | 4.5E-1 | 4.1E-1 | 1.9E0 | 4.9E-1 | 7.6E0 | 1.0E-9 | 3.7E-2 | 1.8E0 | 5.5E1 | 46 | 52 | 46 | 52 | 0.65 |
| tQ | pg/ml | 1.3E3 | 1.5E3 | 1.4E3 | 1.5E3 | 5.2E2 | 6.3E2 | 3.7E2 | 5.0E2 | 2.5E3 | 3.3E3 | 25 | 28 | 25 | 28 | 0.57 |
| tT | pg/ml | 1.4E1 | 2.1E1 | 1.7E1 | 2.6E1 | 7.2E0 | 1.7E1 | 7.4E0 | 5.4E0 | 3.0E1 | 9.3E1 | 25 | 29 | 25 | 29 | 0.68 |
| tS | pg/ml | 1.1E0 | 7.7E-1 | 1.6E0 | 1.3E0 | 1.9E0 | 2.0E0 | 1.0E-9 | 1.0E-9 | 8.5E0 | 1.0E1 | 25 | 30 | 25 | 30 | 0.44 |
| tX | pg/ml | 1.0E0 | 1.2E0 | 1.3E0 | 2.1E0 | 1.1E0 | 2.5E0 | 2.5E-2 | 2.8E-1 | 4.4E0 | 1.0E1 | 25 | 29 | 25 | 29 | 0.54 |
| tO | pg/ml | 4.4E0 | 3.9E0 | 4.9E0 | 5.5E0 | 3.5E0 | 3.8E0 | 1.0E-9 | 1.7E0 | 1.4E1 | 1.8E1 | 25 | 30 | 25 | 30 | 0.55 |
| tR | pg/ml | 2.0E-1 | 2.2E-1 | 3.2E-1 | 3.7E-1 | 3.8E-1 | 5.3E-1 | 1.0E-9 | 1.0E-9 | 1.5E0 | 2.5E0 | 25 | 29 | 25 | 29 | 0.51 |
| tU | pg/ml | 1.1E1 | 9.8E0 | 1.2E1 | 1.4E1 | 7.5E0 | 1.7E1 | 1.6E0 | 2.2E-1 | 3.1E1 | 8.0E1 | 25 | 31 | 25 | 31 | 0.45 |
| tN | pg/ml | 2.0E1 | 2.0E1 | 2.4E1 | 3.7E1 | 1.8E1 | 4.0E1 | 1.0E-9 | 1.0E-9 | 8.0E1 | 1.6E2 | 25 | 28 | 25 | 28 | 0.56 |
| tV | ng/ml | 4.2E2 | 1.0E3 | 6.1E2 | 9.5E2 | 5.8E2 | 6.2E2 | 1.9E2 | 5.3E1 | 2.9E3 | 3.1E3 | 26 | 30 | 26 | 30 | 0.70 |
| iH | ng/ml | 1.5E5 | 1.7E5 | 1.5E5 | 1.6E5 | 4.5E4 | 5.3E4 | 7.1E4 | 2.9E3 | 2.6E5 | 2.5E5 | 50 | 69 | 50 | 69 | 0.57 |
| iJ | ng/ml | 5.6E4 | 4.4E4 | 5.5E4 | 5.5E4 | 2.3E4 | 4.3E4 | 5.5E3 | 1.8E3 | 1.0E5 | 2.5E5 | 50 | 69 | 50 | 69 | 0.44 |
| hB | ng/ml | 4.6E-1 | 5.1E-1 | 5.6E-1 | 7.0E-1 | 3.9E-1 | 5.7E-1 | 1.4E-1 | 1.2E-1 | 1.7E0 | 3.2E0 | 50 | 69 | 50 | 69 | 0.57 |
| hC | pg/ml | 3.5E3 | 7.5E3 | 5.9E3 | 9.9E3 | 7.1E3 | 1.5E4 | 6.0E1 | 4.1E1 | 3.6E4 | 1.1E5 | 50 | 69 | 50 | 69 | 0.61 |
| hF | ng/ml | 1.0E-9 | 1.0E-9 | 8.0E1 | 1.0E-9 | 5.7E2 | 0.0E0 | 1.0E-9 | 1.0E-9 | 4.0E3 | 1.0E-9 | 50 | 69 | 50 | 69 | 0.49 |
| hG | pg/ml | 6.8E3 | 6.7E3 | 7.2E3 | 7.8E3 | 3.0E3 | 3.7E3 | 1.8E3 | 2.3E3 | 1.8E4 | 2.0E4 | 50 | 69 | 50 | 69 | 0.52 |
| iO | ng/ml | 3.7E5 | 3.9E5 | 4.1E5 | 4.2E5 | 2.0E5 | 1.9E5 | 1.1E4 | 9.8E4 | 1.1E6 | 9.2E5 | 50 | 69 | 50 | 69 | 0.53 |
| iP | ng/ml | 5.0E4 | 5.3E4 | 5.2E4 | 6.1E4 | 2.4E4 | 5.7E4 | 1.0E-9 | 7.1E3 | 1.1E5 | 4.4E5 | 50 | 69 | 50 | 69 | 0.51 |
| iZ | ng/ml | 1.5E3 | 1.8E3 | 1.7E3 | 2.0E3 | 8.2E2 | 9.5E2 | 6.6E2 | 7.5E2 | 5.1E3 | 5.7E3 | 50 | 67 | 50 | 67 | 0.59 |
| yH | pg/ml | 1.2E3 | 9.1E2 | 1.8E3 | 2.8E3 | 2.9E3 | 6.2E3 | 1.0E-9 | 1.0E-9 | 1.5E4 | 2.5E4 | 26 | 29 | 26 | 29 | 0.45 |
| yK | U/ml | 2.2E1 | 1.8E1 | 5.3E1 | 2.9E1 | 9.4E1 | 3.3E1 | 1.0E-9 | 1.0E-9 | 3.8E2 | 1.4E2 | 26 | 29 | 26 | 29 | 0.44 |
| yJ | pg/ml | 4.5E4 | 3.0E4 | 4.7E4 | 3.5E4 | 2.6E4 | 2.8E4 | 9.2E3 | 1.9E3 | 1.0E5 | 1.4E5 | 26 | 29 | 26 | 29 | 0.36 |
| yD | ng/ml | 1.2E-2 | 1.4E-2 | 1.2E-2 | 1.4E-2 | 5.6E-3 | 5.8E-3 | 1.0E-9 | 1.0E-9 | 2.8E-2 | 2.4E-2 | 26 | 31 | 26 | 31 | 0.59 |
| jB | ng/ml | 2.8E5 | 2.3E5 | 2.7E5 | 2.2E5 | 7.3E4 | 7.1E4 | 1.5E5 | 9.9E4 | 3.6E5 | 3.5E5 | 12 | 21 | 12 | 21 | 0.32 |
| wB | pg/ml | 9.2E3 | 1.1E4 | 1.1E4 | 1.3E4 | 7.5E3 | 9.9E3 | 1.7E3 | 1.9E3 | 3.3E4 | 4.2E4 | 26 | 31 | 26 | 31 | 0.54 |
| pY | pg/ml | 5.1E0 | 6.4E0 | 1.2E1 | 7.0E0 | 3.6E1 | 3.7E0 | 2.3E0 | 1.6E0 | 2.0E2 | 1.8E1 | 28 | 27 | 28 | 27 | 0.62 |
| sI | ng/ml | 4.7E-2 | 6.2E-2 | 4.7E-2 | 6.3E-2 | 1.3E-2 | 3.5E-2 | 2.1E-2 | 1.0E-2 | 6.6E-2 | 1.5E-1 | 12 | 17 | 12 | 17 | 0.65 |
| sF | mIU/mL | 1.1E1 | 5.6E0 | 1.6E1 | 1.4E1 | 2.2E1 | 2.0E1 | 6.2E-1 | 1.2E0 | 8.1E1 | 7.5E1 | 12 | 17 | 12 | 17 | 0.39 |
| sH | mIU/mL | 5.0E0 | 1.7E0 | 6.1E0 | 4.5E0 | 6.6E0 | 5.7E0 | 1.0E-9 | 7.9E-2 | 2.5E1 | 2.1E1 | 12 | 17 | 12 | 17 | 0.38 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| sJ | ng/ml | 1.5E-1 | 1.5E-1 | 5.0E-1 | 5.3E-1 | 9.3E-1 | 1.5E0 | 1.0E-9 | 1.0E-9 | 3.3E0 | 6.4E0 | 12 | 17 | 12 | 17 | 0.44 |
| rC | pg/ml | 2.1E3 | 1.2E3 | 2.6E3 | 1.7E3 | 2.6E3 | 1.9E3 | 1.1E2 | 1.0E-9 | 1.5E4 | 1.1E4 | 42 | 46 | 42 | 46 | 0.37 |
| rB | pg/ml | 3.4E1 | 2.9E1 | 5.0E1 | 5.6E1 | 7.0E1 | 6.8E1 | 1.0E-9 | 1.0E-9 | 3.9E2 | 3.2E2 | 42 | 46 | 42 | 46 | 0.49 |
| zG | 2.5ng/ml | 2.4E-1 | 2.2E-1 | 5.7E-1 | 5.7E-1 | 9.8E-1 | 1.1E0 | 1.0E-9 | 1.0E-9 | 4.4E0 | 4.8E0 | 26 | 29 | 26 | 29 | 0.50 |
| zH | 2.3mU/ml | 9.2E-2 | 8.8E-2 | 1.1E-1 | 9.0E-2 | 5.5E-2 | 3.6E-2 | 1.0E-2 | 2.1E-2 | 3.1E-1 | 1.8E-1 | 26 | 29 | 26 | 29 | 0.39 |
| zI | 2.6ng/ml | 2.4E0 | 1.9E0 | 3.7E0 | 5.2E0 | 4.0E0 | 7.0E0 | 6.3E-1 | 5.4E-1 | 1.6E1 | 2.7E1 | 26 | 29 | 26 | 29 | 0.52 |
| qA | ng/ml | 8.9E6 | 1.2E7 | 1.1E7 | 1.3E7 | 7.5E6 | 7.2E6 | 3.7E6 | 4.3E6 | 3.9E7 | 3.0E7 | 28 | 27 | 28 | 27 | 0.62 |
| qB | ng/ml | 6.5E5 | 6.3E5 | 7.6E5 | 9.5E5 | 3.7E5 | 8.7E5 | 2.7E5 | 1.9E5 | 1.6E6 | 3.8E6 | 28 | 27 | 28 | 27 | 0.49 |
| qC | ng/ml | 3.3E5 | 2.6E5 | 7.3E5 | 6.2E5 | 1.1E6 | 1.0E6 | 3.6E4 | 2.5E4 | 5.2E6 | 4.7E6 | 28 | 27 | 28 | 27 | 0.46 |
| qD | ng/ml | 1.5E7 | 1.5E7 | 1.8E7 | 1.7E7 | 9.2E6 | 7.8E6 | 1.2E6 | 7.0E6 | 4.5E7 | 3.7E7 | 28 | 27 | 28 | 27 | 0.50 |
| jD | ng/ml | 2.6E1 | 4.1E1 | 3.4E1 | 6.1E1 | 3.4E1 | 8.5E1 | 1.0E-9 | 1.0E-9 | 1.1E2 | 5.1E2 | 46 | 52 | 46 | 52 | 0.60 |
| jE | ng/ml | 1.0E-9 | 1.0E-9 | 6.3E0 | 5.2E0 | 1.4E1 | 1.0E1 | 1.0E-9 | 1.0E-9 | 6.2E1 | 3.5E1 | 46 | 52 | 46 | 52 | 0.51 |
| jF | ng/ml | 5.0E1 | 2.1E1 | 5.9E1 | 3.7E1 | 5.7E1 | 4.8E1 | 1.0E-9 | 1.0E-9 | 2.1E2 | 1.9E2 | 46 | 52 | 46 | 52 | 0.39 |
| jG | ng/ml | 4.2E3 | 4.4E3 | 4.6E3 | 4.5E3 | 1.8E3 | 2.1E3 | 1.9E3 | 6.7E2 | 8.9E3 | 9.6E3 | 46 | 52 | 46 | 52 | 0.49 |
| jH | ng/ml | 7.8E1 | 7.9E1 | 7.7E1 | 9.4E1 | 3.6E1 | 7.0E1 | 2.1E1 | 1.3E1 | 1.6E2 | 4.3E2 | 46 | 52 | 46 | 52 | 0.54 |
| jI | ng/ml | 7.3E1 | 8.1E1 | 7.0E1 | 9.8E1 | 2.4E1 | 6.9E1 | 1.9E1 | 3.8E1 | 1.5E2 | 4.4E2 | 46 | 52 | 46 | 52 | 0.61 |
| sK | pg/mL | 3.8E3 | 3.8E3 | 3.9E3 | 5.0E3 | 1.3E3 | 4.2E3 | 1.8E3 | 2.1E3 | 7.1E3 | 2.3E4 | 26 | 27 | 26 | 27 | 0.53 |
| sM | pg/mL | 7.3E4 | 7.4E4 | 7.6E4 | 8.5E4 | 2.2E4 | 3.9E4 | 4.8E4 | 3.9E4 | 1.5E5 | 2.0E5 | 26 | 27 | 26 | 27 | 0.53 |
| sO | pg/mL | 2.4E8 | 2.2E8 | 2.5E8 | 2.3E8 | 8.2E7 | 1.0E8 | 4.9E7 | 6.6E7 | 3.7E8 | 4.4E8 | 26 | 27 | 26 | 27 | 0.43 |
| wC | ng/ml | 1.6E0 | 1.5E0 | 2.1E0 | 1.7E0 | 1.6E0 | 1.1E0 | 3.6E-1 | 6.1E-2 | 6.5E0 | 4.8E0 | 26 | 31 | 26 | 31 | 0.43 |
| wD | ng/ml | 2.0E1 | 2.8E1 | 1.2E2 | 4.9E1 | 4.1E2 | 5.7E1 | 3.8E0 | 2.8E0 | 2.1E3 | 2.9E2 | 26 | 31 | 26 | 31 | 0.67 |
| wE | ng/ml | 4.7E1 | 5.6E1 | 4.6E1 | 5.4E1 | 2.0E1 | 2.0E1 | 8.1E0 | 2.0E1 | 9.4E1 | 8.9E1 | 26 | 31 | 26 | 31 | 0.59 |
| wG | ng/ml | 1.1E-1 | 9.9E-2 | 1.4E-1 | 1.3E-1 | 1.7E-1 | 1.5E-1 | 1.0E-9 | 1.0E-9 | 7.1E-1 | 6.8E-1 | 26 | 31 | 26 | 31 | 0.50 |
| wH | ng/ml | 1.9E-2 | 3.9E-2 | 3.0E-1 | 3.9E-1 | 8.7E-1 | 1.0E0 | 1.0E-9 | 1.0E-9 | 4.2E0 | 5.6E0 | 26 | 31 | 26 | 31 | 0.56 |
| wF | ng/ml | 9.5E-2 | 3.3E-1 | 3.3E0 | 1.5E0 | 1.2E1 | 3.5E0 | 1.0E-9 | 1.0E-9 | 5.7E1 | 1.9E1 | 26 | 31 | 26 | 31 | 0.66 |
| rA | pg/ml | 2.4E1 | 2.2E1 | 3.1E1 | 2.9E1 | 3.1E1 | 2.3E1 | 1.0E-9 | 1.0E-9 | 2.0E2 | 1.1E2 | 45 | 50 | 45 | 50 | 0.50 |
| qZ | pg/ml | 4.7E1 | 4.5E1 | 3.4E2 | 7.2E2 | 1.7E3 | 2.4E3 | 2.8E-4 | 5.9E-4 | 1.0E4 | 1.0E4 | 35 | 36 | 35 | 36 | 0.50 |
| qY | pg/ml | 1.8E1 | 1.5E1 | 3.9E1 | 3.8E1 | 5.2E1 | 5.9E1 | 8.7E-1 | 2.1E0 | 2.3E2 | 3.3E2 | 45 | 50 | 45 | 50 | 0.47 |
| qX | pg/ml | 5.5E1 | 6.4E1 | 6.5E1 | 7.8E1 | 4.5E1 | 5.2E1 | 1.0E-9 | 1.0E-9 | 2.3E2 | 2.1E2 | 45 | 50 | 45 | 50 | 0.56 |
| qW | pg/ml | 7.7E0 | 7.5E0 | 1.3E1 | 1.0E1 | 2.0E1 | 9.9E0 | 1.0E-9 | 1.0E-9 | 1.2E2 | 3.6E1 | 45 | 50 | 45 | 50 | 0.49 |
| qV | pg/ml | 1.5E3 | 2.2E3 | 2.4E3 | 2.5E3 | 1.8E3 | 2.2E3 | 2.7E2 | 1.0E2 | 7.5E3 | 1.1E4 | 45 | 50 | 45 | 50 | 0.51 |
| qU | pg/ml | 5.3E1 | 9.6E1 | 1.5E2 | 2.3E2 | 2.4E2 | 3.6E2 | 1.0E-9 | 1.0E-9 | 1.2E3 | 2.1E3 | 45 | 50 | 45 | 50 | 0.58 |
| qT | pg/ml | 3.5E1 | 4.2E1 | 6.2E1 | 6.8E1 | 7.9E1 | 6.2E1 | 1.0E-9 | 1.0E-9 | 4.9E2 | 3.1E2 | 45 | 50 | 45 | 50 | 0.57 |
| qI | ng/ml | 5.1E4 | 6.5E4 | 5.7E4 | 6.7E4 | 2.9E4 | 3.2E4 | 1.0E4 | 2.5E4 | 1.3E5 | 1.6E5 | 27 | 25 | 27 | 25 | 0.59 |
| qH | ng/ml | 6.8E4 | 5.6E4 | 7.1E4 | 6.9E4 | 4.2E4 | 3.7E4 | 1.5E4 | 2.3E4 | 1.8E5 | 1.8E5 | 27 | 25 | 27 | 25 | 0.49 |
| qG | ng/ml | 1.8E5 | 1.9E5 | 1.9E5 | 2.0E5 | 6.7E4 | 7.2E4 | 3.4E4 | 8.4E4 | 3.0E5 | 4.2E5 | 27 | 25 | 27 | 25 | 0.52 |
| jK | ng/ml | 1.5E3 | 1.5E3 | 1.6E3 | 1.7E3 | 5.0E2 | 7.2E2 | 2.8E2 | 7.0E2 | 2.8E3 | 4.1E3 | 46 | 52 | 46 | 52 | 0.49 |
| jL | ng/ml | 1.7E2 | 2.1E2 | 2.7E2 | 2.8E2 | 2.2E2 | 1.8E2 | 5.6E1 | 6.4E1 | 9.6E2 | 7.6E2 | 46 | 52 | 46 | 52 | 0.57 |
| jM | ng/ml | 6.6E4 | 6.8E4 | 7.3E4 | 7.6E4 | 3.8E4 | 4.2E4 | 2.1E4 | 4.6E3 | 1.8E5 | 1.7E5 | 46 | 52 | 46 | 52 | 0.52 |
| jO | pg/ml | 2.1E5 | 2.5E5 | 2.7E5 | 2.8E5 | 1.9E5 | 1.4E5 | 6.0E4 | 9.6E4 | 1.1E6 | 6.5E5 | 46 | 52 | 46 | 52 | 0.54 |
| jP | pg/ml | 2.3E5 | 2.6E5 | 2.6E5 | 3.1E5 | 1.5E5 | 1.5E5 | 3.6E4 | 1.3E5 | 7.1E5 | 7.0E5 | 46 | 52 | 46 | 52 | 0.59 |
| jQ | pg/ml | 2.7E3 | 2.1E3 | 3.4E3 | 2.9E3 | 3.0E3 | 2.8E3 | 7.3E1 | 5.0E0 | 1.3E4 | 1.3E4 | 46 | 52 | 46 | 52 | 0.44 |
| jR | pg/ml | 7.1E3 | 4.9E3 | 1.0E4 | 9.9E3 | 1.2E4 | 1.3E4 | 3.0E1 | 1.0E-9 | 6.8E4 | 5.6E4 | 46 | 52 | 46 | 52 | 0.46 |
| jT | pg/ml | 1.7E5 | 1.8E5 | 1.8E5 | 1.8E5 | 7.8E4 | 6.4E4 | 7.1E4 | 7.5E4 | 5.5E5 | 3.5E5 | 46 | 52 | 46 | 52 | 0.52 |
| xA | pg/ml | 5.6E0 | 4.7E0 | 1.3E1 | 1.5E1 | 1.7E1 | 3.4E1 | 1.0E-9 | 1.0E-9 | 6.1E1 | 1.6E2 | 26 | 29 | 26 | 29 | 0.46 |
| yE | pg/ml | 8.3E1 | 7.9E1 | 8.4E1 | 8.5E1 | 2.9E1 | 4.0E1 | 3.6E1 | 6.4E0 | 1.4E2 | 2.0E2 | 26 | 29 | 26 | 29 | 0.48 |
| tM | pg/ml | 4.3E1 | 3.9E1 | 4.1E1 | 4.0E1 | 1.7E1 | 2.0E1 | 1.0E-9 | 1.0E-9 | 6.3E1 | 9.9E1 | 26 | 29 | 26 | 29 | 0.44 |
| tL | pg/ml | 1.0E-9 | 1.0E-9 | 2.8E0 | 3.4E-1 | 1.3E1 | 8.4E-1 | 1.0E-9 | 1.0E-9 | 6.7E1 | 3.6E0 | 26 | 29 | 26 | 29 | 0.52 |
| jU | mIU/ml | 5.5E0 | 5.4E0 | 1.4E1 | 1.0E1 | 2.3E1 | 1.3E1 | 8.1E-2 | 3.9E-1 | 1.1E2 | 5.7E1 | 46 | 52 | 46 | 52 | 0.48 |
| jV | mIU/ml | 1.9E0 | 1.9E0 | 4.7E0 | 3.4E0 | 6.8E0 | 4.0E0 | 2.7E-3 | 2.1E-2 | 3.2E1 | 1.8E1 | 46 | 52 | 46 | 52 | 0.47 |
| jY | ng/ml | 7.3E-4 | 1.7E-3 | 8.7E-3 | 6.6E-3 | 4.4E-2 | 1.5E-2 | 1.0E-9 | 1.0E-9 | 3.0E-1 | 9.4E-2 | 46 | 52 | 46 | 52 | 0.57 |
| kC | pg/ml | 9.3E1 | 1.1E2 | 1.0E2 | 2.4E2 | 6.6E1 | 4.3E2 | 2.1E1 | 3.6E1 | 2.8E2 | 2.7E3 | 30 | 46 | 30 | 46 | 0.62 |
| kE | pg/ml | 1.4E5 | 1.4E5 | 1.3E5 | 1.4E5 | 3.8E4 | 4.2E4 | 4.1E4 | 3.8E4 | 2.3E5 | 2.7E5 | 30 | 46 | 30 | 46 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| kF | pg/mL | 6.1E1 | 6.6E1 | 6.3E1 | 7.2E1 | 1.9E1 | 2.8E1 | 2.7E1 | 4.0E1 | 1.1E2 | 1.5E2 | 30 | 46 | 30 | 46 | 0.56 |
| kG | pg/mL | 7.5E3 | 9.0E3 | 1.0E4 | 1.6E4 | 8.9E3 | 2.5E4 | 1.3E3 | 1.1E3 | 4.1E4 | 1.6E5 | 30 | 46 | 30 | 46 | 0.59 |
| kI | pg/ml | 1.7E2 | 2.1E2 | 1.9E2 | 2.3E2 | 8.8E1 | 1.3E2 | 6.4E1 | 1.0E-9 | 4.2E2 | 6.7E2 | 30 | 46 | 30 | 46 | 0.58 |
| kK | pg/ml | 1.1E2 | 1.2E2 | 1.3E2 | 2.1E2 | 1.1E2 | 3.0E2 | 6.4E0 | 2.1E1 | 5.0E2 | 1.9E3 | 30 | 46 | 30 | 46 | 0.59 |
| kN | pg/ml | 8.6E2 | 1.2E3 | 1.1E3 | 1.8E3 | 6.8E2 | 2.1E3 | 2.1E2 | 7.3E1 | 2.6E3 | 1.0E4 | 30 | 46 | 30 | 46 | 0.56 |
| kO | pg/ml | 7.0E3 | 7.2E3 | 7.3E3 | 1.1E4 | 2.0E3 | 2.1E4 | 4.0E3 | 3.7E3 | 1.1E4 | 1.5E5 | 30 | 46 | 30 | 46 | 0.50 |
| kP | pg/ml | 6.7E3 | 5.1E3 | 7.7E3 | 6.0E3 | 4.7E3 | 3.8E3 | 3.1E3 | 9.6E2 | 2.7E4 | 1.6E4 | 30 | 46 | 30 | 46 | 0.37 |
| kQ | pg/ml | 4.2E3 | 4.5E3 | 4.9E3 | 5.7E3 | 3.7E3 | 4.3E3 | 5.6E2 | 1.1E3 | 2.5E4 | 2.5E4 | 50 | 69 | 50 | 69 | 0.56 |
| kR | pg/ml | 1.9E1 | 2.6E1 | 4.3E1 | 3.1E1 | 1.4E2 | 2.4E1 | 1.0E-9 | 2.9E0 | 1.0E3 | 1.1E2 | 50 | 69 | 50 | 69 | 0.59 |
| kS | pg/ml | 8.6E2 | 8.8E2 | 1.0E3 | 9.7E2 | 6.0E2 | 5.8E2 | 2.6E2 | 8.2E1 | 3.2E3 | 3.0E3 | 50 | 69 | 50 | 69 | 0.49 |
| pS | ng/ml | 1.7E5 | 1.4E5 | 1.9E5 | 1.7E5 | 8.6E4 | 1.1E5 | 9.7E4 | 6.8E4 | 5.0E5 | 5.7E5 | 26 | 27 | 26 | 27 | 0.38 |
| rZ | ng/ml | 1.0E-9 | 5.3E-3 | 4.6E-3 | 1.7E-2 | 1.5E-2 | 4.6E-2 | 1.0E-9 | 1.0E-9 | 9.4E-2 | 3.0E-1 | 41 | 48 | 41 | 48 | 0.67 |
| rY | ng/ml | 6.1E-2 | 6.4E-2 | 2.5E-1 | 8.7E-1 | 9.8E-1 | 3.8E0 | 2.4E-3 | 1.0E-9 | 6.3E0 | 2.0E1 | 41 | 48 | 41 | 48 | 0.53 |
| rX | ng/ml | 1.0E-9 | 1.0E-9 | 1.2E-1 | 1.3E-1 | 5.9E-1 | 5.6E-1 | 1.0E-9 | 1.0E-9 | 3.7E0 | 3.1E0 | 41 | 48 | 41 | 48 | 0.53 |
| lK | pg/ml | 6.5E1 | 5.9E1 | 1.6E2 | 1.3E2 | 1.9E2 | 1.6E2 | 1.0E-9 | 1.0E-9 | 7.4E2 | 6.9E2 | 46 | 51 | 46 | 51 | 0.42 |
| lL | pg/ml | 1.8E3 | 1.7E3 | 2.6E3 | 3.2E3 | 3.0E3 | 5.9E3 | 7.5E1 | 1.2E2 | 1.9E4 | 4.2E4 | 46 | 52 | 46 | 52 | 0.49 |
| lM | pg/ml | 1.6E3 | 1.1E3 | 4.2E3 | 5.2E3 | 7.4E3 | 1.1E4 | 2.7E2 | 9.5E0 | 4.2E4 | 6.7E4 | 46 | 52 | 46 | 52 | 0.44 |
| lN | pg/ml | 1.0E-9 | 1.0E-9 | 3.2E0 | 3.4E0 | 7.4E0 | 6.2E0 | 1.0E-9 | 1.0E-9 | 4.3E1 | 2.4E1 | 46 | 52 | 46 | 52 | 0.52 |
| lO | pg/ml | 1.0E-9 | 1.0E-9 | 7.4E-1 | 5.2E0 | 5.0E0 | 2.6E1 | 1.0E-9 | 1.0E-9 | 3.4E1 | 1.4E2 | 46 | 51 | 46 | 51 | 0.51 |
| zA | ng/ml | 2.2E7 | 2.1E7 | 2.2E7 | 2.1E7 | 7.0E6 | 6.0E6 | 9.1E6 | 1.0E7 | 3.4E7 | 3.6E7 | 25 | 31 | 25 | 31 | 0.46 |
| rW | ng/ml | 1.2E-2 | 1.2E-2 | 2.9E-2 | 3.5E-2 | 4.0E-2 | 7.1E-2 | 1.0E-9 | 1.0E-9 | 1.7E-1 | 3.2E-1 | 27 | 25 | 27 | 25 | 0.50 |
| rV | ng/ml | 1.0E-9 | 1.0E-9 | 1.6E-2 | 1.2E-2 | 5.8E-2 | 3.2E-2 | 1.0E-9 | 1.0E-9 | 2.8E-1 | 1.5E-1 | 27 | 25 | 27 | 25 | 0.54 |
| rU | ng/ml | 7.1E-2 | 9.5E-2 | 2.2E-1 | 1.1E-1 | 5.3E-1 | 1.1E-1 | 1.0E-9 | 1.0E-9 | 2.7E0 | 4.0E-1 | 27 | 25 | 27 | 25 | 0.50 |
| rT | ng/ml | 6.7E0 | 4.4E0 | 7.2E0 | 6.7E0 | 4.2E0 | 5.2E0 | 6.5E-1 | 1.0E0 | 2.1E1 | 2.0E1 | 27 | 25 | 27 | 25 | 0.42 |
| rS | ng/ml | 3.9E0 | 4.2E0 | 6.1E0 | 1.1E1 | 5.9E0 | 1.8E1 | 7.6E-1 | 1.1E0 | 2.5E1 | 7.0E1 | 27 | 25 | 27 | 25 | 0.54 |
| sC | pg/mL | 5.4E3 | 7.9E3 | 8.7E3 | 1.3E4 | 9.1E3 | 1.7E4 | 2.4E3 | 1.7E3 | 3.9E4 | 7.4E4 | 26 | 27 | 26 | 27 | 0.55 |
| yL | pg/ml | 3.1E1 | 2.8E1 | 3.7E1 | 8.3E1 | 3.1E1 | 2.6E2 | 5.6E0 | 9.1E0 | 1.8E2 | 1.4E3 | 26 | 29 | 26 | 29 | 0.47 |
| rP | ng/ml | 1.3E2 | 1.3E2 | 2.0E2 | 2.3E2 | 1.8E2 | 2.3E2 | 1.0E-9 | 1.2E1 | 5.0E2 | 8.0E2 | 27 | 25 | 27 | 25 | 0.51 |
| rQ | ng/ml | 1.0E-9 | 1.0E-9 | 1.0E-9 | 1.3E1 | 0.0E0 | 4.1E1 | 1.0E-9 | 1.0E-9 | 1.0E-9 | 1.7E2 | 27 | 25 | 27 | 25 | 0.56 |
| rO | ng/ml | 1.5E-2 | 2.5E-2 | 4.1E-2 | 3.8E-2 | 8.1E-2 | 4.5E-2 | 1.0E-9 | 1.0E-9 | 4.0E-1 | 1.9E-1 | 27 | 25 | 27 | 25 | 0.58 |
| rR | ng/ml | 1.0E-9 | 4.0E0 | 5.6E0 | 1.6E1 | 1.5E1 | 2.5E1 | 1.0E-9 | 1.0E-9 | 7.9E1 | 9.5E1 | 27 | 25 | 27 | 25 | 0.66 |
| rN | ng/ml | 8.1E-1 | 6.2E-1 | 9.4E-1 | 1.3E0 | 5.9E-1 | 2.6E0 | 5.1E-2 | 2.1E-1 | 2.3E0 | 1.3E1 | 27 | 25 | 27 | 25 | 0.42 |
| qO | pg/ml | 8.3E3 | 8.2E3 | 1.1E4 | 1.4E4 | 7.9E3 | 1.2E4 | 2.7E3 | 7.4E2 | 2.8E4 | 4.8E4 | 27 | 26 | 27 | 26 | 0.53 |
| qP | pg/ml | 3.6E2 | 3.2E2 | 3.9E2 | 4.4E2 | 2.1E2 | 3.4E2 | 7.0E1 | 1.1E2 | 8.3E2 | 1.5E3 | 27 | 26 | 27 | 26 | 0.50 |
| qQ | pg/ml | 1.5E1 | 1.5E1 | 2.3E1 | 2.3E1 | 5.3E1 | 5.1E1 | 1.0E-9 | 1.0E-9 | 2.8E2 | 2.6E2 | 27 | 26 | 27 | 26 | 0.47 |
| nW | pg/ml | 1.1E5 | 1.1E5 | 1.1E5 | 1.1E5 | 2.8E4 | 2.8E4 | 5.8E4 | 3.6E4 | 1.9E5 | 1.7E5 | 50 | 69 | 50 | 69 | 0.50 |
| nY | pg/ml | 2.1E3 | 2.5E3 | 2.3E3 | 2.9E3 | 1.2E3 | 1.8E3 | 5.1E2 | 6.3E2 | 4.7E3 | 1.0E4 | 50 | 69 | 50 | 69 | 0.58 |
| oO | pg/ml | 8.9E4 | 8.9E4 | 9.9E4 | 1.1E5 | 5.4E4 | 8.7E4 | 1.5E4 | 3.3E3 | 2.6E5 | 4.0E5 | 27 | 42 | 27 | 42 | 0.49 |
| oP | pg/ml | 1.2E5 | 1.3E5 | 1.4E5 | 1.6E5 | 7.4E4 | 1.2E5 | 2.4E4 | 2.4E4 | 3.1E5 | 5.7E5 | 27 | 42 | 27 | 42 | 0.51 |
| oQ | pg/ml | 3.0E3 | 3.0E3 | 3.2E3 | 4.9E3 | 2.1E3 | 5.8E3 | 9.3E2 | 7.7E2 | 1.1E4 | 3.2E4 | 27 | 42 | 27 | 42 | 0.57 |
| oE | pg/ml | 1.4E2 | 2.6E2 | 3.5E2 | 6.3E2 | 4.4E2 | 7.7E2 | 1.0E-9 | 1.0E-9 | 1.7E3 | 3.4E3 | 50 | 69 | 50 | 69 | 0.62 |
| oF | pg/ml | 9.6E3 | 1.8E4 | 2.3E4 | 3.3E4 | 3.5E4 | 4.3E4 | 4.3E2 | 3.5E2 | 1.7E5 | 2.5E5 | 50 | 69 | 50 | 69 | 0.60 |
| oH | pg/ml | 4.0E1 | 3.4E1 | 8.6E1 | 7.2E1 | 1.4E2 | 9.4E1 | 5.4E0 | 4.3E-1 | 8.6E2 | 4.8E2 | 50 | 69 | 50 | 69 | 0.46 |
| oK | pg/ml | 8.2E2 | 8.8E2 | 1.8E3 | 1.5E3 | 2.0E3 | 1.8E3 | 8.8E1 | 1.4E2 | 9.2E3 | 1.2E4 | 50 | 69 | 50 | 69 | 0.51 |
| oN | pg/ml | 5.2E2 | 5.6E2 | 1.1E3 | 8.2E2 | 2.7E3 | 8.8E2 | 1.6E2 | 1.1E2 | 1.8E4 | 5.3E3 | 50 | 69 | 50 | 69 | 0.56 |
| oW | pg/ml | 2.1E2 | 3.1E2 | 2.8E2 | 8.4E2 | 2.0E2 | 1.7E3 | 7.7E1 | 2.9E1 | 7.3E2 | 7.6E3 | 12 | 21 | 12 | 21 | 0.59 |
| oT | pg/ml | 3.2E2 | 2.8E2 | 3.4E2 | 3.2E2 | 1.6E2 | 1.9E2 | 1.0E2 | 1.1E2 | 7.4E2 | 7.9E2 | 12 | 21 | 12 | 21 | 0.42 |
| oV | pg/ml | 2.3E2 | 8.9E1 | 3.3E2 | 2.4E2 | 4.0E2 | 4.9E2 | 2.1E1 | 1.0E-9 | 1.4E3 | 2.2E3 | 12 | 21 | 12 | 21 | 0.38 |
| oD | pg/ml | 1.7E4 | 1.3E4 | 1.7E4 | 1.5E4 | 4.8E3 | 6.5E3 | 9.3E3 | 6.6E3 | 2.5E4 | 3.2E4 | 12 | 21 | 12 | 21 | 0.34 |
| uL | ng/ml | 3.5E1 | 3.9E1 | 5.4E1 | 4.4E1 | 5.8E1 | 2.2E1 | 1.5E1 | 1.4E1 | 2.9E2 | 1.1E2 | 25 | 27 | 25 | 27 | 0.51 |
| uO | ng/ml | 4.8E-1 | 2.4E-1 | 1.0E0 | 6.4E-1 | 1.8E0 | 7.4E-1 | 1.0E-9 | 1.0E-9 | 9.3E0 | 2.1E0 | 25 | 27 | 25 | 27 | 0.43 |
| uM | ng/ml | 6.4E-1 | 5.0E-1 | 1.3E0 | 5.8E-1 | 2.5E0 | 5.7E-1 | 1.0E-9 | 1.0E-9 | 1.3E1 | 2.5E0 | 25 | 27 | 25 | 27 | 0.41 |
| uI | ng/ml | 8.7E-2 | 5.9E-2 | 1.2E-1 | 1.0E-1 | 1.1E-1 | 1.2E-1 | 1.6E-2 | 1.5E-2 | 5.8E-1 | 4.3E-1 | 25 | 26 | 25 | 26 | 0.39 |

| Code | Units | Median | | Average | | Standard Deviation | | Minimum | | Maximum | | Number of Samples | | Number of Patients | | AUC |
|------|-------|--------|-----|---------|-----|------|-----|---------|-----|---------|-----|--------|-----|----------|-----|------|
| | | NonDis | Dis | NonDis | Dis | NonDis | Dis | NonDis | Dis | NonDis | Dis | NonDis | Dis | NonDis | Dis | |
| uN | ng/ml | 1.5E1 | 1.6E1 | 1.6E1 | 1.8E1 | 5.7E0 | 8.0E0 | 8.0E0 | 9.9E0 | 3.0E1 | 4.1E1 | 25 | 27 | 25 | 27 | 0.57 |
| uG | ng/ml | 1.8E1 | 1.9E1 | 2.2E1 | 2.6E1 | 1.6E1 | 2.5E1 | 6.1E0 | 1.2E0 | 7.9E1 | 1.3E2 | 25 | 27 | 25 | 27 | 0.55 |
| uR | ng/ml | 2.4E0 | 1.8E0 | 2.9E0 | 2.7E0 | 2.3E0 | 2.1E0 | 1.1E0 | 7.5E-1 | 1.3E1 | 8.3E0 | 26 | 29 | 26 | 29 | 0.37 |
| uP | ng/ml | 2.1E0 | 2.4E0 | 2.4E0 | 2.7E0 | 1.1E0 | 1.2E0 | 1.2E0 | 9.3E-1 | 6.0E0 | 6.1E0 | 26 | 29 | 26 | 29 | 0.59 |
| uV | ng/ml | 1.1E-4 | 2.3E-3 | 1.9E-2 | 9.6E-3 | 4.3E-2 | 1.2E-2 | 1.0E-9 | 1.0E-9 | 2.0E-1 | 4.3E-2 | 26 | 29 | 26 | 29 | 0.51 |
| uT | ng/ml | 6.6E1 | 7.8E1 | 1.0E2 | 9.9E1 | 1.0E2 | 8.2E1 | 1.4E1 | 1.3E1 | 4.5E2 | 4.1E2 | 26 | 29 | 26 | 29 | 0.55 |
| uU | ng/ml | 1.9E0 | 1.6E0 | 2.1E0 | 2.3E0 | 1.4E0 | 3.5E0 | 5.2E-1 | 5.4E-1 | 6.0E0 | 2.0E1 | 26 | 29 | 26 | 29 | 0.46 |
| uW | ng/ml | 7.3E0 | 7.9E0 | 7.7E0 | 8.1E0 | 2.1E0 | 2.4E0 | 4.4E0 | 5.1E0 | 1.3E1 | 1.6E1 | 25 | 27 | 25 | 27 | 0.55 |
| vB | ng/ml | 3.0E0 | 3.2E0 | 3.1E0 | 3.6E0 | 1.5E0 | 2.6E0 | 1.1E0 | 5.9E-1 | 8.3E0 | 1.0E1 | 25 | 27 | 25 | 27 | 0.52 |
| vC | ng/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 25 | 27 | 25 | 27 | 0.50 |
| uY | ng/ml | 5.9E-1 | 7.5E-1 | 7.4E-1 | 1.2E0 | 5.6E-1 | 1.2E0 | 6.8E-2 | 3.1E-1 | 2.3E0 | 4.4E0 | 25 | 27 | 25 | 27 | 0.65 |
| uZ | ng/ml | 5.8E-1 | 5.3E-1 | 7.0E-1 | 7.7E-1 | 6.0E-1 | 9.0E-1 | 1.0E-1 | 1.7E-1 | 3.0E0 | 4.9E0 | 25 | 27 | 25 | 27 | 0.49 |
| uX | ng/ml | 8.0E0 | 8.6E0 | 1.2E1 | 1.4E1 | 8.0E0 | 1.5E1 | 4.5E0 | 3.6E0 | 4.0E1 | 6.5E1 | 25 | 27 | 25 | 27 | 0.53 |
| vA | ng/ml | 6.9E-2 | 6.3E-2 | 7.5E-2 | 8.9E-2 | 4.0E-2 | 8.6E-2 | 2.4E-2 | 2.5E-2 | 1.9E-1 | 4.2E-1 | 25 | 27 | 25 | 27 | 0.49 |
| vH | ng/ml | 1.2E-1 | 1.1E-1 | 1.5E-1 | 2.0E-1 | 1.2E-1 | 3.6E-1 | 3.5E-2 | 2.0E-2 | 6.6E-1 | 1.9E0 | 26 | 27 | 26 | 27 | 0.42 |
| vI | ng/ml | 2.0E0 | 2.4E0 | 2.1E0 | 2.8E0 | 1.5E0 | 2.5E0 | 3.0E-2 | 6.3E-3 | 6.4E0 | 1.0E1 | 26 | 27 | 26 | 27 | 0.59 |
| vP | ng/ml | 3.7E2 | 3.2E2 | 4.1E2 | 5.2E2 | 3.1E2 | 4.9E2 | 7.0E1 | 6.7E1 | 1.1E3 | 2.4E3 | 26 | 29 | 26 | 29 | 0.56 |
| vT | ng/ml | 7.2E1 | 8.3E1 | 8.4E1 | 9.1E1 | 4.7E1 | 3.8E1 | 4.1E1 | 4.6E1 | 2.4E2 | 1.8E2 | 26 | 29 | 26 | 29 | 0.58 |
| vU | ng/ml | 3.0E0 | 1.0E-9 | 3.3E1 | 1.9E1 | 4.6E1 | 3.2E1 | 1.0E-9 | 1.0E-9 | 1.3E2 | 1.1E2 | 26 | 29 | 26 | 29 | 0.42 |
| vQ | ng/ml | 3.9E2 | 4.5E2 | 4.1E2 | 4.2E2 | 1.6E2 | 1.7E2 | 7.2E1 | 1.2E2 | 8.1E2 | 8.4E2 | 26 | 29 | 26 | 29 | 0.53 |
| vO | ng/ml | 1.8E3 | 1.7E3 | 1.8E3 | 1.8E3 | 4.0E2 | 5.1E2 | 1.1E3 | 1.0E3 | 2.6E3 | 3.2E3 | 26 | 29 | 26 | 29 | 0.44 |
| vS | ng/ml | 1.3E3 | 1.3E3 | 1.3E3 | 1.2E3 | 3.6E2 | 5.2E2 | 1.0E-9 | 1.0E-9 | 2.1E3 | 2.0E3 | 26 | 29 | 26 | 29 | 0.50 |
| vV | ng/ml | 8.3E2 | 9.0E2 | 8.9E2 | 1.2E3 | 6.9E2 | 1.1E3 | 1.1E2 | 1.0E2 | 2.9E3 | 4.6E3 | 26 | 29 | 26 | 29 | 0.55 |
| vW | ng/ml | 1.1E2 | 1.1E2 | 1.6E2 | 1.8E2 | 1.2E2 | 1.5E2 | 4.3E1 | 6.0E1 | 6.6E2 | 7.7E2 | 26 | 29 | 26 | 29 | 0.54 |
| pF | pg/ml | 7.6E-1 | 5.2E-1 | 1.0E0 | 2.0E0 | 1.4E0 | 1.0E1 | 1.0E-9 | 1.0E-9 | 9.4E0 | 8.7E1 | 50 | 69 | 50 | 69 | 0.42 |
| pH | ng/ml | 7.3E0 | 8.8E0 | 8.8E0 | 1.0E1 | 4.3E0 | 5.6E0 | 3.9E0 | 1.2E0 | 1.8E1 | 2.3E1 | 12 | 21 | 12 | 21 | 0.57 |
| pI | ng/ml | 7.7E1 | 6.7E1 | 7.3E1 | 7.3E1 | 3.4E1 | 4.8E1 | 2.6E1 | 2.3E1 | 1.5E2 | 2.0E2 | 12 | 21 | 12 | 21 | 0.46 |
| pK | ng/ml | 4.0E-1 | 4.6E-1 | 4.1E-1 | 4.9E-1 | 1.6E-1 | 2.1E-1 | 2.3E-1 | 1.7E-1 | 7.7E-1 | 8.6E-1 | 12 | 21 | 12 | 21 | 0.63 |

Figure 32.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 8.4E1 | 6.0E1 | 9.5E1 | 8.1E1 | 6.6E1 | 7.3E1 | 8.0E0 | 7.0E0 | 4.8E2 | 4.0E2 | 261 | 34 | 261 | 34 | 0.41 |
| Ad | ug/mL | 4.7E-2 | 5.4E-2 | 1.3E-1 | 7.5E-2 | 7.0E-1 | 7.7E-2 | 6.8E-4 | 7.8E-4 | 8.5E0 | 3.5E-1 | 147 | 26 | 147 | 26 | 0.52 |
| Af | ng/mL | 1.2E0 | 1.8E0 | 1.1E1 | 6.2E0 | 4.8E1 | 7.4E0 | 1.7E-3 | 1.7E-3 | 5.3E2 | 2.1E1 | 147 | 26 | 147 | 26 | 0.54 |
| Aj | ug/mL | 1.1E0 | 6.6E-1 | 2.4E0 | 2.0E0 | 2.5E0 | 2.4E0 | 1.5E-3 | 1.3E-3 | 6.1E0 | 5.8E0 | 147 | 26 | 147 | 26 | 0.47 |
| Al | mg/mL | 8.8E-5 | 7.9E-5 | 2.7E-4 | 3.3E-4 | 4.5E-4 | 4.6E-4 | 4.3E-6 | 6.6E-6 | 1.8E-3 | 1.5E-3 | 147 | 26 | 147 | 26 | 0.51 |
| An | U/mL | 5.8E1 | 8.7E1 | 2.4E2 | 3.4E2 | 8.1E2 | 6.3E2 | 2.8E-1 | 1.1E0 | 7.8E3 | 2.5E3 | 147 | 26 | 147 | 26 | 0.59 |
| Ao | pg/mL | 9.1E1 | 1.4E2 | 5.7E2 | 4.2E2 | 3.8E3 | 9.0E2 | 1.5E0 | 4.1E0 | 3.9E4 | 4.5E3 | 147 | 26 | 147 | 26 | 0.60 |
| Ap | ng/mL | 3.3E1 | 3.5E1 | 4.8E1 | 6.1E1 | 5.4E1 | 7.0E1 | 2.0E0 | 2.7E0 | 3.3E2 | 2.4E2 | 147 | 26 | 147 | 26 | 0.52 |
| Ar | ng/mL | 6.8E-1 | 1.1E0 | 2.5E0 | 6.6E0 | 5.6E0 | 1.4E1 | 3.4E-3 | 4.1E-2 | 4.7E1 | 5.1E1 | 147 | 26 | 147 | 26 | 0.56 |
| As | ng/mL | 8.7E-3 | 8.2E-3 | 2.1E-2 | 9.0E-3 | 1.0E-1 | 8.3E-3 | 1.7E-3 | 1.7E-3 | 1.2E0 | 3.3E-2 | 147 | 26 | 147 | 26 | 0.47 |
| Aw | pg/mL | 1.6E1 | 2.0E1 | 1.7E1 | 2.0E1 | 6.3E0 | 5.7E0 | 2.9E-2 | 8.2E0 | 5.1E1 | 3.2E1 | 147 | 26 | 147 | 26 | 0.68 |
| Ax | ng/mL | 2.0E0 | 2.6E0 | 2.3E1 | 6.7E1 | 9.1E1 | 1.9E2 | 1.2E-2 | 1.0E-4 | 7.7E2 | 8.5E2 | 147 | 26 | 147 | 26 | 0.51 |
| Ba | ng/mL | 8.3E1 | 4.4E2 | 5.2E2 | 1.8E3 | 1.4E3 | 3.3E3 | 1.9E0 | 1.1E0 | 8.1E3 | 1.5E4 | 147 | 26 | 147 | 26 | 0.64 |
| Bb | ng/mL | 4.1E0 | 5.9E0 | 7.1E0 | 9.4E0 | 8.4E0 | 1.2E1 | 4.1E-3 | 4.1E-3 | 4.9E1 | 4.8E1 | 147 | 26 | 147 | 26 | 0.52 |
| Bc | ng/mL | 3.5E1 | 7.6E1 | 1.3E2 | 1.6E2 | 2.4E2 | 2.4E2 | 4.9E-1 | 1.0E0 | 1.2E3 | 9.9E2 | 147 | 26 | 147 | 26 | 0.56 |
| Bg | ng/mL | 1.1E-1 | 2.6E-1 | 3.4E0 | 2.2E1 | 1.3E1 | 8.2E1 | 5.3E-4 | 5.3E-4 | 1.0E2 | 4.0E2 | 147 | 26 | 147 | 26 | 0.59 |
| Bn | ng/mL | 5.6E-2 | 4.4E-1 | 1.5E0 | 1.4E0 | 5.1E0 | 2.0E0 | 5.6E-2 | 5.6E-2 | 5.8E1 | 7.4E0 | 147 | 26 | 147 | 26 | 0.62 |
| Bo | ng/mL | 1.3E1 | 2.0E1 | 1.4E1 | 2.0E1 | 1.1E1 | 1.2E1 | 1.6E-2 | 2.2E0 | 5.0E1 | 5.3E1 | 147 | 26 | 147 | 26 | 0.65 |
| Ch | uIU/mL | 1.0E0 | 1.1E0 | 3.0E1 | 6.1E1 | 1.7E2 | 2.3E2 | 3.4E-3 | 1.5E-1 | 1.8E3 | 1.2E3 | 147 | 26 | 147 | 26 | 0.54 |
| Co | pg/mL | 4.7E1 | 5.9E1 | 2.4E2 | 1.9E2 | 1.4E3 | 4.1E2 | 1.5E-1 | 3.6E0 | 1.7E4 | 2.1E3 | 147 | 26 | 147 | 26 | 0.54 |
| Cp | ng/mL | 2.2E1 | 2.7E1 | 3.5E1 | 4.0E1 | 1.1E2 | 3.1E1 | 6.0E-1 | 4.7E0 | 1.3E3 | 1.4E2 | 147 | 26 | 147 | 26 | 0.62 |
| Cq | ng/mL | 2.8E-2 | 4.2E-2 | 4.4E-1 | 1.9E-1 | 4.1E0 | 4.7E-1 | 8.0E-4 | 8.0E-4 | 4.9E1 | 2.3E0 | 147 | 26 | 147 | 26 | 0.60 |
| Cs | ng/mL | 5.9E1 | 1.1E2 | 4.7E2 | 6.1E2 | 1.8E3 | 1.3E3 | 8.3E-1 | 8.9E-1 | 1.8E4 | 5.1E3 | 147 | 26 | 147 | 26 | 0.55 |
| Ct | ng/mL | 3.1E-1 | 8.7E-1 | 4.0E1 | 4.9E1 | 1.2E2 | 1.2E2 | 8.9E-3 | 1.1E-4 | 6.2E2 | 4.4E2 | 147 | 26 | 147 | 26 | 0.56 |
| Cu | ng/mL | 2.5E-1 | 7.9E-1 | 9.0E-1 | 1.9E0 | 5.5E0 | 4.1E0 | 1.9E-2 | 1.7E-2 | 6.6E1 | 2.1E1 | 147 | 26 | 147 | 26 | 0.69 |
| Cv | ng/mL | 6.1E0 | 5.1E0 | 2.8E1 | 3.7E1 | 6.7E1 | 9.4E1 | 2.0E-2 | 7.5E-2 | 5.3E2 | 4.7E2 | 147 | 26 | 147 | 26 | 0.48 |
| Cw | mIU/mL | 3.3E-2 | 4.9E-2 | 8.8E-2 | 5.5E-2 | 5.6E-1 | 3.8E-2 | 8.9E-4 | 2.8E-3 | 6.8E0 | 1.5E-1 | 147 | 26 | 147 | 26 | 0.60 |
| Cx | ng/mL | 6.9E-1 | 7.2E-1 | 4.9E1 | 5.6E1 | 9.7E1 | 1.1E2 | 9.3E-5 | 9.3E-5 | 3.9E2 | 2.8E2 | 147 | 26 | 147 | 26 | 0.47 |
| Db | ug/mL | 7.5E0 | 8.1E0 | 8.7E0 | 9.3E0 | 7.9E0 | 5.7E0 | 4.5E-1 | 8.1E-1 | 5.9E1 | 2.3E1 | 147 | 26 | 147 | 26 | 0.56 |
| Dc | nmol/L | 2.1E-2 | 2.3E-2 | 1.4E-1 | 2.7E-1 | 1.2E0 | 5.6E-1 | 5.2E-6 | 1.1E-3 | 1.4E1 | 2.2E0 | 147 | 26 | 147 | 26 | 0.56 |
| Dd | ug/mL | 7.7E-2 | 6.8E-2 | 2.0E-1 | 2.3E-1 | 3.8E-1 | 3.5E-1 | 4.8E-4 | 1.3E-3 | 3.6E0 | 1.5E0 | 147 | 26 | 147 | 26 | 0.51 |
| De | ng/mL | 3.4E-3 | 1.7E-1 | 7.0E-2 | 1.7E-1 | 1.4E-1 | 2.3E-1 | 3.4E-3 | 3.4E-3 | 9.2E-1 | 1.1E0 | 147 | 26 | 147 | 26 | 0.69 |
| Dg | ng/mL | 3.6E1 | 4.0E1 | 4.8E1 | 4.7E1 | 4.0E1 | 3.9E1 | 7.1E-1 | 7.8E-1 | 1.9E2 | 1.2E2 | 147 | 26 | 147 | 26 | 0.50 |
| Di | pg/mL | 2.0E0 | 3.3E0 | 2.3E0 | 3.3E0 | 2.2E0 | 1.9E0 | 1.8E-1 | 1.8E-1 | 1.3E1 | 9.3E0 | 147 | 26 | 147 | 26 | 0.67 |
| Dk | uIU/mL | 1.4E-2 | 3.0E-2 | 5.2E-2 | 1.2E-1 | 1.7E-1 | 2.3E-1 | 1.1E-4 | 1.1E-4 | 1.6E0 | 9.8E-1 | 147 | 26 | 147 | 26 | 0.61 |
| Dl | ng/mL | 2.1E2 | 2.0E2 | 3.0E2 | 2.9E2 | 2.8E2 | 2.8E2 | 5.5E0 | 4.4E0 | 1.6E3 | 1.1E3 | 147 | 26 | 147 | 26 | 0.49 |
| Dp | ng/ml | 2.4E0 | 1.2E0 | 5.9E0 | 3.1E0 | 9.4E0 | 5.0E0 | 3.7E-3 | 3.7E-3 | 5.6E1 | 1.8E1 | 111 | 23 | 111 | 23 | 0.37 |
| Dr | pg/ml | 2.9E1 | 2.2E1 | 2.4E2 | 6.1E1 | 1.4E3 | 1.1E2 | 7.5E-1 | 7.5E-1 | 1.0E4 | 3.6E2 | 57 | 15 | 57 | 15 | 0.44 |
| Du | pg/ml | 1.6E2 | 2.2E2 | 1.6E3 | 5.2E2 | 4.6E3 | 6.4E2 | 1.2E0 | 1.2E0 | 2.4E4 | 1.8E3 | 43 | 14 | 43 | 14 | 0.48 |
| Ef | ng/ml | 9.3E-2 | 2.0E-1 | 7.5E-1 | 1.8E0 | 1.8E0 | 3.0E0 | 5.7E-4 | 5.7E-4 | 1.0E1 | 9.9E0 | 124 | 22 | 124 | 22 | 0.59 |
| Wm | % | 8.5E-2 | 8.5E-2 | 9.8E0 | 4.8E1 | 7.0E1 | 2.0E2 | 5.4E-2 | 8.5E-2 | 7.7E2 | 1.0E3 | 130 | 28 | 130 | 28 | 0.47 |
| Ed | pg/ml | 4.4E0 | 3.1E1 | 3.1E1 | 7.0E1 | 5.9E1 | 1.1E2 | 5.2E-1 | 5.2E-1 | 5.0E2 | 4.8E2 | 111 | 23 | 111 | 23 | 0.63 |
| Yf | ng/mL | 1.5E1 | 1.7E1 | 3.8E1 | 9.9E1 | 5.5E1 | 1.8E2 | 2.9E-1 | 2.9E-1 | 2.4E2 | 5.9E2 | 45 | 14 | 45 | 14 | 0.54 |
| Tj | pg/mL | 3.7E-1 | 3.7E-1 | 4.1E1 | 2.5E1 | 2.3E2 | 6.7E1 | 3.7E-1 | 3.7E-1 | 2.3E3 | 2.5E2 | 121 | 23 | 121 | 23 | 0.46 |
| Po | pg/ml | 1.4E-1 | 7.4E0 | 9.2E0 | 1.9E1 | 3.0E1 | 3.8E1 | 8.0E-3 | 2.6E-2 | 2.7E2 | 2.2E2 | 307 | 44 | 307 | 44 | 0.69 |
| Ti | ug/mL | 3.2E0 | 3.9E0 | 5.0E0 | 4.7E0 | 4.4E0 | 3.8E0 | 1.2E-1 | 4.0E-1 | 1.8E1 | 1.1E1 | 66 | 17 | 66 | 17 | 0.49 |
| Em | ng/ml | 2.9E-3 | 2.6E-2 | 7.7E-2 | 4.8E-2 | 2.4E-1 | 6.7E-2 | 8.4E-4 | 8.4E-4 | 1.9E0 | 2.4E-1 | 74 | 18 | 74 | 18 | 0.54 |
| Et | ng/ml | 1.4E3 | 2.6E3 | 1.6E3 | 2.3E3 | 1.2E3 | 1.3E3 | 7.5E1 | 7.9E1 | 4.8E3 | 5.0E3 | 306 | 44 | 306 | 44 | 0.65 |
| Eq | pg/ml | 1.3E2 | 3.0E2 | 3.1E2 | 4.6E2 | 3.8E2 | 4.6E2 | 1.0E0 | 1.0E0 | 1.5E3 | 1.4E3 | 43 | 14 | 43 | 14 | 0.59 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Th | ug/mL | 1.2E0 | 1.5E0 | 1.7E0 | 2.1E0 | 1.5E0 | 1.7E0 | 2.6E-3 | 2.6E-1 | 7.5E0 | 5.9E0 | 66 | 17 | 66 | 17 | 0.56 |
| Fa | ng/ml | 4.2E1 | 5.8E1 | 1.3E2 | 9.7E1 | 4.2E2 | 1.1E2 | 2.6E-1 | 1.6E0 | 3.7E3 | 4.3E2 | 110 | 20 | 110 | 20 | 0.55 |
| Ez | ng/ml | 3.7E0 | 5.3E0 | 1.4E1 | 2.2E1 | 2.7E1 | 4.5E1 | 1.3E-2 | 1.3E-2 | 1.6E2 | 2.0E2 | 111 | 23 | 111 | 23 | 0.52 |
| Fb | ng/ml | 2.5E1 | 2.7E1 | 2.2E1 | 2.6E1 | 1.1E1 | 1.3E1 | 6.6E-1 | 8.9E-1 | 4.3E1 | 4.3E1 | 111 | 20 | 111 | 20 | 0.58 |
| Ex | ng/ml | 6.0E-2 | 1.6E-1 | 1.7E-1 | 5.0E-1 | 3.1E-1 | 9.7E-1 | 3.5E-5 | 1.7E-4 | 2.2E0 | 4.1E0 | 86 | 19 | 86 | 19 | 0.65 |
| Fc | pg/ml | 2.2E-1 | 2.2E-1 | 2.1E1 | 3.2E1 | 8.0E1 | 1.0E2 | 2.2E-1 | 2.2E-1 | 4.5E2 | 3.9E2 | 44 | 14 | 44 | 14 | 0.53 |
| Fd | pg/ml | 8.2E1 | 1.1E2 | 9.6E2 | 2.6E3 | 3.8E3 | 6.2E3 | 4.5E-1 | 9.8E-1 | 2.5E4 | 2.2E4 | 44 | 14 | 44 | 14 | 0.55 |
| Fi | pg/ml | 2.5E-1 | 2.5E-1 | 5.9E1 | 2.0E2 | 1.7E2 | 5.0E2 | 2.5E-1 | 2.5E-1 | 8.5E2 | 1.8E3 | 44 | 14 | 44 | 14 | 0.57 |
| Fn | ng/ml | 2.1E-1 | 2.8E-1 | 4.3E0 | 2.3E0 | 7.6E0 | 3.7E0 | 1.1E-14 | 2.1E-1 | 3.7E1 | 1.6E1 | 111 | 23 | 111 | 23 | 0.51 |
| Fp | ng/ml | 1.3E1 | 1.5E1 | 2.3E1 | 3.1E1 | 2.6E1 | 3.6E1 | 6.0E-3 | 3.0E-1 | 1.3E2 | 1.3E2 | 309 | 44 | 309 | 44 | 0.56 |
| Fr | ng/ml | 3.3E4 | 1.1E5 | 1.1E5 | 2.7E5 | 1.7E5 | 2.9E5 | 1.9E2 | 1.3E3 | 8.4E5 | 8.4E5 | 314 | 46 | 314 | 46 | 0.67 |
| Fw | pg/ml | 1.1E0 | 1.4E1 | 4.9E1 | 3.7E1 | 2.8E2 | 6.1E1 | 1.2E-1 | 1.2E-1 | 3.0E3 | 2.5E2 | 125 | 23 | 125 | 23 | 0.69 |
| Fy | ng/ml | 3.4E1 | 4.4E1 | 6.3E1 | 1.2E2 | 8.9E1 | 1.5E2 | 1.2E-1 | 1.2E-1 | 6.5E2 | 5.3E2 | 110 | 21 | 110 | 21 | 0.60 |
| Gh | pg/ml | 1.3E0 | 5.0E0 | 2.5E1 | 9.5E0 | 6.1E1 | 1.2E1 | 2.9E-2 | 2.9E-2 | 3.2E2 | 4.6E1 | 44 | 14 | 44 | 14 | 0.59 |
| Gb | % | 4.4E1 | 2.7E1 | 5.6E1 | 4.2E1 | 5.6E1 | 4.4E1 | 3.7E0 | 2.2E0 | 3.0E2 | 1.5E2 | 44 | 14 | 44 | 14 | 0.39 |
| Gc | ng/ml | 9.9E1 | 1.2E2 | 1.7E2 | 1.5E2 | 2.2E2 | 1.5E2 | 6.4E0 | 2.7E1 | 1.2E3 | 5.1E2 | 57 | 15 | 57 | 15 | 0.52 |
| Gd | ng/ml | 3.3E1 | 2.9E1 | 3.4E1 | 2.9E1 | 1.9E1 | 1.6E1 | 3.7E0 | 3.0E0 | 8.1E1 | 5.6E1 | 66 | 16 | 66 | 16 | 0.43 |
| Gn | U/ml | 2.2E-1 | 1.3E-1 | 3.3E0 | 5.1E-1 | 1.6E1 | 6.7E-1 | 5.6E-3 | 5.6E-3 | 1.1E2 | 2.2E0 | 56 | 15 | 56 | 15 | 0.44 |
| Gl | pg/ml | 8.6E3 | 2.3E4 | 1.2E4 | 1.8E4 | 9.1E3 | 1.1E4 | 9.1E1 | 5.3E2 | 3.2E4 | 3.2E4 | 125 | 23 | 125 | 23 | 0.68 |
| Gp | U/ml | 1.2E0 | 1.5E-1 | 2.6E0 | 9.2E-1 | 3.6E0 | 1.3E0 | 1.5E-2 | 1.5E-2 | 2.0E1 | 4.2E0 | 125 | 22 | 125 | 22 | 0.33 |
| Gz | ug/ml | 1.5E0 | 8.1E-1 | 6.0E0 | 3.2E0 | 5.9E0 | 4.4E0 | 6.2E-2 | 4.2E-2 | 2.5E1 | 1.1E1 | 75 | 19 | 75 | 19 | 0.36 |
| Ha | ng/ml | 2.0E0 | 2.6E0 | 8.4E0 | 9.8E0 | 2.0E1 | 2.0E1 | 1.7E-2 | 1.7E-2 | 1.0E2 | 9.2E1 | 109 | 23 | 109 | 23 | 0.57 |
| Nm | pg/ml | 1.3E4 | 1.4E4 | 3.6E4 | 5.0E4 | 9.3E4 | 9.4E4 | 1.0E-9 | 1.0E-9 | 9.6E5 | 4.4E5 | 310 | 44 | 310 | 44 | 0.53 |
| Nn | pg/ml | 1.4E2 | 7.5E2 | 1.1E3 | 1.6E4 | 4.5E3 | 5.1E4 | 1.0E-9 | 1.0E-9 | 6.0E4 | 3.1E5 | 310 | 44 | 310 | 44 | 0.69 |
| No | pg/ml | 1.5E1 | 2.5E1 | 3.5E1 | 8.5E1 | 7.9E1 | 1.7E2 | 1.0E-9 | 3.3E-1 | 9.1E2 | 7.7E2 | 310 | 44 | 310 | 44 | 0.62 |
| Nq | pg/ml | 1.6E0 | 1.1E1 | 1.8E1 | 6.4E1 | 6.7E1 | 1.4E2 | 1.0E-9 | 1.0E-9 | 6.3E2 | 6.7E2 | 310 | 44 | 310 | 44 | 0.65 |
| Nr | pg/ml | 1.3E0 | 7.1E0 | 2.3E1 | 5.7E1 | 8.6E1 | 2.1E2 | 1.0E-9 | 1.0E-9 | 9.8E2 | 1.4E3 | 310 | 44 | 310 | 44 | 0.61 |
| Ns | pg/ml | 1.0E-9 | 1.0E-9 | 4.9E0 | 2.7E1 | 2.5E1 | 1.7E2 | 1.0E-9 | 1.0E-9 | 2.4E2 | 1.1E3 | 310 | 44 | 310 | 44 | 0.51 |
| Nt | pg/ml | 1.0E2 | 1.5E2 | 1.3E2 | 2.2E2 | 1.3E2 | 2.3E2 | 9.8E-1 | 3.5E1 | 1.7E3 | 1.2E3 | 310 | 44 | 310 | 44 | 0.67 |
| Nu | pg/ml | 1.7E1 | 5.0E1 | 5.3E1 | 9.0E1 | 8.8E1 | 1.2E2 | 1.0E-9 | 1.0E-9 | 6.3E2 | 6.3E2 | 310 | 44 | 310 | 44 | 0.64 |
| Lu | pg/ml | 1.0E4 | 9.0E3 | 1.4E4 | 2.1E4 | 2.8E4 | 8.3E4 | 5.2E2 | 1.3E3 | 3.9E5 | 5.6E5 | 310 | 44 | 310 | 44 | 0.42 |
| Lv | pg/ml | 1.0E-9 | 1.6E1 | 1.4E1 | 3.6E1 | 2.8E1 | 5.3E1 | 1.0E-9 | 1.0E-9 | 2.4E2 | 2.6E2 | 310 | 44 | 310 | 44 | 0.63 |
| Lw | pg/ml | 1.0E-9 | 1.0E-9 | 5.2E-1 | 5.9E0 | 5.4E0 | 2.7E1 | 1.0E-9 | 1.0E-9 | 8.0E1 | 1.8E2 | 310 | 44 | 310 | 44 | 0.55 |
| Lx | pg/ml | 1.0E-9 | 1.8E2 | 2.3E2 | 5.0E2 | 1.4E3 | 7.2E2 | 1.0E-9 | 1.0E-9 | 2.2E4 | 2.8E3 | 310 | 44 | 310 | 44 | 0.71 |
| Ly | pg/ml | 1.0E-9 | 1.0E-9 | 9.6E0 | 5.3E0 | 1.8E1 | 1.1E1 | 1.0E-9 | 1.0E-9 | 1.1E2 | 4.8E1 | 310 | 44 | 310 | 44 | 0.45 |
| Lz | pg/ml | 1.0E-9 | 1.0E-9 | 3.3E0 | 4.7E0 | 2.7E1 | 1.5E1 | 1.0E-9 | 1.0E-9 | 4.3E2 | 6.2E1 | 310 | 44 | 310 | 44 | 0.53 |
| Ma | pg/ml | 3.9E2 | 8.0E2 | 2.0E3 | 5.9E3 | 5.3E3 | 1.1E4 | 1.0E-9 | 1.0E-9 | 6.5E4 | 5.2E4 | 310 | 44 | 310 | 44 | 0.59 |
| Mb | pg/ml | 2.5E1 | 3.0E1 | 3.1E1 | 3.7E1 | 1.7E1 | 2.0E1 | 4.1E0 | 1.5E1 | 2.1E2 | 1.1E2 | 310 | 44 | 310 | 44 | 0.58 |
| Mc | pg/ml | 1.0E-9 | 1.0E-9 | 5.6E-2 | 3.8E-2 | 7.8E-1 | 2.5E-1 | 1.0E-9 | 1.0E-9 | 1.3E1 | 1.7E0 | 310 | 44 | 310 | 44 | 0.51 |
| Md | pg/ml | 1.0E-9 | 1.0E-9 | 6.8E-1 | 6.1E-1 | 5.7E0 | 1.9E0 | 1.0E-9 | 1.0E-9 | 6.8E1 | 9.8E0 | 310 | 44 | 310 | 44 | 0.55 |
| Me | pg/ml | 3.2E1 | 2.4E1 | 3.2E1 | 2.8E1 | 2.3E1 | 3.5E1 | 1.0E-9 | 1.0E-9 | 3.2E2 | 1.8E2 | 310 | 44 | 310 | 44 | 0.38 |
| Mf | pg/ml | 1.0E-9 | 1.0E-9 | 5.1E-1 | 1.3E0 | 3.5E0 | 5.6E0 | 1.0E-9 | 1.0E-9 | 5.6E1 | 3.7E1 | 310 | 44 | 310 | 44 | 0.55 |
| Mg | pg/ml | 1.1E0 | 2.7E-1 | 6.4E0 | 1.1E1 | 1.2E1 | 2.7E1 | 1.0E-9 | 1.0E-9 | 9.2E1 | 1.5E2 | 310 | 44 | 310 | 44 | 0.50 |
| Mh | pg/ml | 1.0E-9 | 1.0E-9 | 1.2E0 | 1.3E0 | 8.0E0 | 3.7E0 | 1.0E-9 | 1.0E-9 | 1.1E2 | 1.8E1 | 310 | 44 | 310 | 44 | 0.57 |
| Mi | pg/ml | 1.0E-9 | 1.0E-9 | 2.0E0 | 1.7E1 | 2.0E1 | 8.0E1 | 1.0E-9 | 1.0E-9 | 3.2E2 | 5.2E2 | 310 | 44 | 310 | 44 | 0.56 |
| Mj | pg/ml | 1.0E-9 | 1.0E-9 | 5.9E0 | 1.6E1 | 3.3E1 | 3.8E1 | 1.0E-9 | 1.0E-9 | 4.6E2 | 2.1E2 | 310 | 44 | 310 | 44 | 0.61 |
| Mk | pg/ml | 1.5E0 | 5.4E0 | 1.6E1 | 2.0E1 | 9.7E1 | 7.6E1 | 1.0E-9 | 1.0E-9 | 1.3E3 | 5.0E2 | 310 | 44 | 310 | 44 | 0.56 |
| Ml | pg/ml | 1.0E-9 | 1.0E-9 | 1.1E1 | 1.2E1 | 1.2E2 | 5.4E1 | 1.0E-9 | 1.0E-9 | 2.1E3 | 3.4E2 | 310 | 44 | 310 | 44 | 0.48 |
| Mm | pg/ml | 5.5E2 | 8.3E2 | 1.0E3 | 1.7E3 | 1.3E3 | 2.2E3 | 1.0E-9 | 1.0E-9 | 1.0E4 | 1.0E4 | 310 | 44 | 310 | 44 | 0.57 |
| Mn | pg/ml | 5.7E0 | 1.0E1 | 1.1E1 | 1.1E1 | 2.6E1 | 9.8E0 | 1.0E-9 | 1.0E-9 | 3.5E2 | 5.1E1 | 310 | 44 | 310 | 44 | 0.61 |
| Mp | pg/ml | 1.0E-9 | 1.0E1 | 1.3E1 | 8.4E1 | 5.1E1 | 3.6E2 | 1.0E-9 | 1.0E-9 | 6.3E2 | 2.4E3 | 310 | 44 | 310 | 44 | 0.66 |
| Mq | pg/ml | 1.0E-9 | 1.0E-9 | 2.3E0 | 6.2E0 | 1.4E1 | 1.6E1 | 1.0E-9 | 1.0E-9 | 1.8E2 | 7.4E1 | 310 | 44 | 310 | 44 | 0.57 |
| Mr | pg/ml | 1.0E-9 | 1.0E-9 | 3.3E1 | 1.2E2 | 1.9E2 | 5.3E2 | 1.0E-9 | 1.0E-9 | 2.2E3 | 3.4E3 | 310 | 44 | 310 | 44 | 0.59 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ms | pg/ml | 3.2E2 | 3.5E2 | 4.6E2 | 5.4E2 | 5.2E2 | 8.7E2 | 1.0E-9 | 1.0E-9 | 3.2E3 | 4.7E3 | 310 | 44 | 310 | 44 | 0.49 |
| Mt | pg/ml | 1.7E-1 | 2.3E0 | 8.9E0 | 9.4E1 | 4.6E1 | 4.9E2 | 1.0E-9 | 1.0E-9 | 7.1E2 | 3.2E3 | 310 | 44 | 310 | 44 | 0.67 |
| Mu | pg/ml | 1.0E-9 | 1.0E-9 | 7.5E-1 | 8.5E0 | 6.4E0 | 3.6E1 | 1.0E-9 | 1.0E-9 | 9.9E1 | 2.3E2 | 310 | 44 | 310 | 44 | 0.61 |
| Mv | pg/ml | 1.0E-9 | 1.0E-9 | 6.0E1 | 1.5E2 | 3.5E2 | 2.8E2 | 1.0E-9 | 1.0E-9 | 4.5E3 | 9.2E2 | 310 | 44 | 310 | 44 | 0.61 |
| Mw | pg/ml | 3.6E1 | 7.7E1 | 2.7E2 | 5.7E2 | 1.4E3 | 1.2E3 | 1.0E-9 | 1.0E-9 | 1.8E4 | 5.3E3 | 310 | 44 | 310 | 44 | 0.61 |
| Mx | pg/ml | 1.0E-9 | 1.0E-9 | 2.3E-1 | 1.9E0 | 8.9E-1 | 5.6E0 | 1.0E-9 | 1.0E-9 | 9.2E0 | 3.2E1 | 310 | 44 | 310 | 44 | 0.64 |
| My | pg/ml | 1.0E-9 | 1.0E-9 | 3.4E2 | 2.4E2 | 2.6E3 | 5.2E2 | 1.0E-9 | 1.0E-9 | 3.9E4 | 2.1E3 | 310 | 44 | 310 | 44 | 0.57 |
| Mz | pg/ml | 1.1E1 | 2.9E1 | 2.7E1 | 1.1E2 | 8.6E1 | 2.9E2 | 1.0E-9 | 1.0E-9 | 1.3E3 | 1.9E3 | 310 | 44 | 310 | 44 | 0.72 |
| Na | pg/ml | 1.0E-9 | 1.0E-9 | 7.7E-1 | 9.3E-1 | 3.0E0 | 2.2E0 | 1.0E-9 | 1.0E-9 | 4.2E1 | 9.6E0 | 310 | 44 | 310 | 44 | 0.52 |
| Nb | pg/ml | 2.1E0 | 3.3E0 | 3.9E0 | 1.0E1 | 1.2E1 | 3.2E1 | 1.0E-9 | 1.0E-9 | 1.5E2 | 2.1E2 | 310 | 44 | 310 | 44 | 0.61 |
| Nc | pg/ml | 3.2E2 | 1.6E2 | 5.3E2 | 3.3E2 | 7.6E2 | 4.1E2 | 1.0E-9 | 1.0E-9 | 9.2E3 | 1.6E3 | 310 | 44 | 310 | 44 | 0.42 |
| Nd | pg/ml | 2.8E1 | 1.7E1 | 3.5E1 | 2.6E1 | 1.4E2 | 2.8E1 | 1.0E-9 | 7.2E-1 | 2.1E3 | 1.5E2 | 310 | 44 | 310 | 44 | 0.50 |
| Ne | pg/ml | 4.3E2 | 3.2E2 | 5.3E2 | 4.0E2 | 5.6E2 | 5.6E2 | 1.0E-9 | 1.0E-9 | 7.0E3 | 3.6E3 | 310 | 44 | 310 | 44 | 0.38 |
| Nf | pg/ml | 1.0E-9 | 1.0E-9 | 3.0E0 | 3.4E0 | 1.2E1 | 1.6E1 | 1.0E-9 | 1.0E-9 | 1.3E2 | 1.1E2 | 310 | 44 | 310 | 44 | 0.49 |
| Ng | pg/ml | 1.3E1 | 9.4E0 | 9.1E1 | 7.7E1 | 1.9E2 | 1.2E2 | 1.0E-9 | 1.0E-9 | 1.6E3 | 5.9E2 | 310 | 44 | 310 | 44 | 0.49 |
| Nh | pg/ml | 6.3E1 | 4.1E1 | 8.2E1 | 5.9E1 | 7.4E1 | 7.6E1 | 1.0E-9 | 4.5E0 | 5.6E2 | 5.1E2 | 310 | 44 | 310 | 44 | 0.38 |
| Ni | pg/ml | 1.0E-9 | 3.1E1 | 8.1E1 | 1.1E2 | 1.3E2 | 1.7E2 | 1.0E-9 | 1.0E-9 | 1.1E3 | 7.1E2 | 310 | 44 | 310 | 44 | 0.55 |
| Nj | pg/ml | 7.2E0 | 6.3E0 | 1.1E1 | 7.5E0 | 1.2E1 | 6.4E0 | 1.0E-9 | 1.0E-9 | 6.9E1 | 2.2E1 | 310 | 44 | 310 | 44 | 0.43 |
| Nk | pg/ml | 1.8E1 | 1.2E1 | 3.3E1 | 2.3E1 | 4.0E1 | 2.8E1 | 1.0E-9 | 1.0E-9 | 2.0E2 | 8.8E1 | 310 | 44 | 310 | 44 | 0.44 |
| Nl | pg/ml | 4.3E1 | 3.1E1 | 5.9E1 | 3.5E1 | 7.8E1 | 3.6E1 | 1.0E-9 | 1.0E-9 | 1.1E3 | 1.8E2 | 310 | 44 | 310 | 44 | 0.37 |
| Hl | pg/ml | 1.1E1 | 2.3E0 | 1.2E2 | 1.6E1 | 5.4E2 | 2.2E1 | 1.0E-9 | 1.0E-9 | 3.6E3 | 6.1E1 | 44 | 14 | 44 | 14 | 0.40 |
| Ho | pg/ml | 1.6E1 | 2.3E1 | 2.9E1 | 4.4E1 | 5.9E1 | 5.0E1 | 1.0E-9 | 7.6E0 | 3.9E2 | 1.7E2 | 44 | 14 | 44 | 14 | 0.66 |
| Hp | ng/ml | 1.6E0 | 2.6E0 | 1.2E2 | 1.3E2 | 3.1E2 | 3.2E2 | 3.6E-1 | 1.0E-9 | 8.9E2 | 8.9E2 | 44 | 14 | 44 | 14 | 0.57 |
| Tz | pg/ml | 3.9E3 | 9.8E3 | 5.9E3 | 1.2E5 | 6.7E3 | 4.3E5 | 1.0E-9 | 9.8E1 | 5.3E4 | 2.1E6 | 111 | 23 | 111 | 23 | 0.69 |
| Ua | pg/ml | 3.3E3 | 6.3E3 | 3.1E4 | 2.8E4 | 2.0E5 | 4.1E4 | 1.0E-9 | 2.7E2 | 2.1E6 | 1.3E5 | 111 | 23 | 111 | 23 | 0.57 |
| Ub | pg/ml | 5.8E2 | 4.2E2 | 9.3E2 | 5.0E2 | 1.2E3 | 4.4E2 | 1.0E-9 | 2.3E0 | 9.8E3 | 1.4E3 | 111 | 23 | 111 | 23 | 0.38 |
| Ue | pg/ml | 3.1E1 | 1.9E1 | 3.9E1 | 2.6E1 | 3.4E1 | 2.3E1 | 9.8E-2 | 4.5E0 | 2.7E2 | 1.1E2 | 111 | 23 | 111 | 23 | 0.35 |
| Uc | pg/ml | 6.8E2 | 9.4E2 | 1.7E3 | 1.8E3 | 5.5E3 | 2.4E3 | 1.0E-9 | 1.5E1 | 5.7E4 | 9.4E3 | 111 | 23 | 111 | 23 | 0.57 |
| Ud | pg/ml | 1.0E-9 | 1.0E-9 | 4.0E0 | 1.0E-9 | 3.7E1 | 0.0E0 | 1.0E-9 | 1.0E-9 | 3.9E2 | 1.0E-9 | 111 | 23 | 111 | 23 | 0.49 |
| Hq | pg/ml | 1.1E0 | 2.4E0 | 1.8E2 | 1.9E1 | 2.0E3 | 5.7E1 | 1.0E-9 | 1.0E-9 | 2.8E4 | 3.4E2 | 308 | 44 | 308 | 44 | 0.62 |
| Hr | pg/ml | 9.1E1 | 9.3E1 | 6.9E2 | 4.0E2 | 1.4E3 | 8.3E2 | 1.0E-9 | 1.0E-9 | 1.2E4 | 3.8E3 | 308 | 44 | 308 | 44 | 0.49 |
| Hu | pg/ml | 5.4E0 | 2.4E1 | 4.1E3 | 7.1E3 | 3.9E4 | 4.0E4 | 1.0E-9 | 1.0E-9 | 6.3E5 | 2.6E5 | 308 | 44 | 308 | 44 | 0.56 |
| Hv | pg/ml | 1.3E0 | 2.6E0 | 5.3E0 | 7.8E0 | 5.1E1 | 2.5E1 | 1.0E-9 | 1.0E-9 | 8.9E2 | 1.6E2 | 308 | 44 | 308 | 44 | 0.66 |
| Hw | pg/ml | 6.4E0 | 6.5E0 | 4.6E1 | 2.6E1 | 5.3E2 | 7.8E1 | 1.0E-9 | 1.0E-9 | 9.4E3 | 5.0E2 | 308 | 44 | 308 | 44 | 0.54 |
| Hx | pg/ml | 8.4E0 | 1.8E1 | 5.6E1 | 7.4E1 | 5.3E2 | 2.0E2 | 1.0E-9 | 1.0E-9 | 9.3E3 | 1.3E3 | 308 | 44 | 308 | 44 | 0.63 |
| Ib | ng/ml | 3.6E-2 | 2.6E-2 | 1.2E0 | 2.5E0 | 6.2E0 | 8.0E0 | 1.0E-9 | 1.0E-9 | 5.6E1 | 3.6E1 | 110 | 22 | 110 | 22 | 0.48 |
| Ic | U/ml | 2.2E2 | 2.2E2 | 9.6E2 | 2.1E2 | 6.3E3 | 1.5E2 | 2.4E0 | 1.6E1 | 6.5E4 | 4.2E2 | 110 | 22 | 110 | 22 | 0.52 |
| Id | U/ml | 6.3E-1 | 8.2E-1 | 5.1E0 | 1.6E0 | 4.1E1 | 2.2E0 | 1.0E-9 | 1.0E-9 | 4.3E2 | 9.4E0 | 110 | 22 | 110 | 22 | 0.53 |
| Tt | pg/ml | 1.7E2 | 1.9E2 | 1.7E2 | 1.8E2 | 5.9E1 | 5.0E1 | 4.3E1 | 1.0E2 | 4.4E2 | 2.8E2 | 103 | 20 | 103 | 20 | 0.57 |
| To | pg/ml | 1.6E0 | 1.4E0 | 2.3E0 | 1.8E0 | 3.0E0 | 1.9E0 | 1.0E-9 | 1.0E-9 | 2.3E1 | 6.4E0 | 107 | 23 | 107 | 23 | 0.46 |
| Tr | pg/ml | 3.4E0 | 3.0E0 | 9.0E0 | 7.8E0 | 3.1E1 | 1.3E1 | 1.0E-9 | 1.0E-9 | 3.1E2 | 5.4E1 | 106 | 21 | 106 | 21 | 0.50 |
| Tn | pg/ml | 3.0E1 | 7.2E1 | 1.1E2 | 2.5E2 | 3.5E2 | 4.4E2 | 1.0E-9 | 9.0E0 | 2.3E3 | 2.0E3 | 107 | 23 | 107 | 23 | 0.68 |
| Tv | ng/ml | 1.2E1 | 1.2E1 | 9.2E1 | 4.1E1 | 6.9E2 | 9.1E1 | 1.0E-9 | 1.0E-9 | 7.1E3 | 4.3E2 | 107 | 23 | 107 | 23 | 0.52 |
| Ih | ng/ml | 6.3E1 | 8.4E1 | 2.5E2 | 4.0E2 | 4.4E2 | 6.2E2 | 1.0E-9 | 1.4E0 | 3.6E3 | 2.8E3 | 309 | 44 | 309 | 44 | 0.57 |
| Ii | ng/ml | 7.9E1 | 1.2E2 | 2.2E2 | 2.8E2 | 5.3E2 | 6.8E2 | 7.3E-1 | 7.5E-1 | 5.2E3 | 4.5E3 | 309 | 44 | 309 | 44 | 0.56 |
| Ij | ng/ml | 7.8E1 | 1.2E2 | 2.5E2 | 2.8E2 | 1.5E3 | 4.7E2 | 2.8E0 | 9.5E0 | 2.4E4 | 2.0E3 | 305 | 44 | 305 | 44 | 0.61 |
| Ik | ng/ml | 1.1E1 | 5.1E1 | 1.4E3 | 2.7E2 | 1.2E4 | 4.1E2 | 5.9E-1 | 6.2E-1 | 1.2E5 | 1.5E3 | 305 | 44 | 305 | 44 | 0.61 |
| Il | ng/ml | 3.6E2 | 2.8E2 | 1.3E3 | 1.9E3 | 2.8E3 | 3.6E3 | 1.0E-9 | 1.0E-9 | 1.2E4 | 1.2E4 | 304 | 43 | 304 | 43 | 0.50 |
| Im | ng/ml | 2.0E2 | 6.3E2 | 4.0E2 | 1.2E3 | 6.5E2 | 2.5E3 | 1.4E1 | 2.2E1 | 5.8E3 | 1.5E4 | 305 | 44 | 305 | 44 | 0.66 |
| In | ng/ml | 3.7E0 | 2.2E0 | 3.5E1 | 3.2E1 | 2.7E2 | 1.0E2 | 1.0E-9 | 1.0E-9 | 4.5E3 | 5.9E2 | 309 | 44 | 309 | 44 | 0.44 |
| Hb | ng/ml | 2.6E1 | 1.9E1 | 3.6E1 | 3.5E1 | 3.5E1 | 4.5E1 | 1.6E0 | 4.8E-1 | 2.0E2 | 1.9E2 | 113 | 22 | 113 | 22 | 0.42 |
| Hc | pg/ml | 6.7E2 | 5.9E2 | 2.9E3 | 4.8E3 | 1.0E4 | 1.1E4 | 1.0E-9 | 1.4E2 | 1.0E5 | 5.0E4 | 113 | 22 | 113 | 22 | 0.54 |
| Hf | ng/ml | 2.0E2 | 1.2E2 | 4.3E2 | 2.2E2 | 5.8E2 | 2.5E2 | 1.0E-9 | 1.0E-9 | 3.2E3 | 8.8E2 | 113 | 22 | 113 | 22 | 0.41 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Io | ng/ml | 8.4E3 | 1.2E4 | 1.9E4 | 2.1E4 | 4.7E4 | 3.3E4 | 1.0E-9 | 2.4E2 | 7.1E5 | 2.0E5 | 309 | 44 | 309 | 44 | 0.56 |
| Ip | ng/ml | 8.9E0 | 3.0E1 | 2.0E1 | 3.2E1 | 2.5E1 | 3.2E1 | 1.0E-9 | 5.0E-3 | 2.3E2 | 1.6E2 | 309 | 44 | 309 | 44 | 0.61 |
| Iq | ug/ml | 1.0E-1 | 1.8E-1 | 4.5E1 | 6.6E0 | 7.7E2 | 3.3E1 | 1.0E-9 | 1.0E-9 | 1.4E4 | 2.2E2 | 309 | 44 | 309 | 44 | 0.55 |
| Ir | ug/ml | 3.6E-1 | 7.7E-1 | 5.6E0 | 1.1E1 | 3.9E1 | 3.1E1 | 1.0E-9 | 1.0E-9 | 5.1E2 | 1.3E2 | 308 | 44 | 308 | 44 | 0.61 |
| Is | ng/ml | 1.7E0 | 9.4E0 | 8.3E0 | 3.0E1 | 3.4E1 | 4.9E1 | 1.0E-9 | 1.4E-1 | 5.5E2 | 2.6E2 | 309 | 44 | 309 | 44 | 0.71 |
| It | ng/ml | 2.1E0 | 2.3E0 | 2.1E1 | 4.3E1 | 9.9E1 | 1.5E2 | 1.0E-9 | 1.0E-9 | 1.1E3 | 8.3E2 | 309 | 44 | 309 | 44 | 0.55 |
| Iu | ng/ml | 1.8E2 | 1.1E2 | 1.4E3 | 1.9E3 | 4.3E3 | 5.4E3 | 1.0E-9 | 1.0E-9 | 2.9E4 | 2.4E4 | 309 | 44 | 309 | 44 | 0.47 |
| Iv | ng/ml | 1.1E1 | 2.4E1 | 9.8E1 | 2.6E2 | 9.3E2 | 8.1E2 | 1.0E-9 | 1.0E-9 | 1.6E4 | 3.8E3 | 308 | 44 | 308 | 44 | 0.67 |
| Iz | ng/ml | 1.2E2 | 2.2E2 | 3.6E2 | 4.0E2 | 7.6E2 | 5.1E2 | 1.5E0 | 8.8E-1 | 6.1E3 | 1.7E3 | 113 | 22 | 113 | 22 | 0.54 |
| Yg | pg/ml | 2.5E2 | 1.7E3 | 4.5E2 | 5.3E3 | 6.5E2 | 1.3E4 | 1.0E-9 | 1.0E-9 | 3.4E3 | 5.0E4 | 40 | 14 | 40 | 14 | 0.64 |
| Yh | pg/ml | 2.1E2 | 5.7E2 | 3.4E2 | 7.6E2 | 4.7E2 | 8.1E2 | 1.0E-9 | 1.0E-9 | 2.1E3 | 3.0E3 | 40 | 14 | 40 | 14 | 0.69 |
| Yi | pg/ml | 2.6E2 | 8.4E2 | 4.6E2 | 3.2E3 | 4.5E2 | 7.1E3 | 1.0E-9 | 1.0E-9 | 2.0E3 | 2.6E4 | 40 | 14 | 40 | 14 | 0.63 |
| Yk | U/ml | 1.0E-9 | 1.0E-9 | 1.1E-1 | 5.5E-1 | 3.7E-1 | 9.7E-1 | 1.0E-9 | 1.0E-9 | 1.8E0 | 3.0E0 | 40 | 14 | 40 | 14 | 0.61 |
| Yj | pg/ml | 1.6E2 | 1.0E2 | 3.5E2 | 1.3E2 | 5.6E2 | 1.2E2 | 9.7E0 | 1.0E-9 | 3.2E3 | 4.7E2 | 40 | 14 | 40 | 14 | 0.35 |
| Yd | ng/ml | 1.9E-1 | 3.3E-1 | 3.5E-1 | 5.7E-1 | 4.2E-1 | 7.3E-1 | 6.6E-3 | 1.6E-2 | 1.8E0 | 2.3E0 | 44 | 14 | 44 | 14 | 0.56 |
| Wb | pg/ml | 2.7E4 | 3.3E4 | 3.4E4 | 8.1E4 | 2.4E4 | 1.6E5 | 4.9E3 | 7.5E3 | 1.5E5 | 6.4E5 | 44 | 14 | 44 | 14 | 0.60 |
| Vz | pg/ml | 3.9E0 | 2.4E0 | 5.2E0 | 3.0E0 | 5.0E0 | 2.6E0 | 1.0E-9 | 3.0E-1 | 2.2E1 | 9.6E0 | 44 | 14 | 44 | 14 | 0.36 |
| Si | ng/ml | 1.3E0 | 9.3E-1 | 2.1E0 | 1.6E0 | 2.6E0 | 1.7E0 | 1.1E-1 | 8.6E-3 | 1.0E1 | 6.0E0 | 44 | 14 | 44 | 14 | 0.47 |
| Sf | mIU/mL | 1.7E1 | 1.6E1 | 5.6E1 | 1.7E1 | 1.2E2 | 1.3E1 | 6.2E-1 | 1.3E0 | 7.2E2 | 4.3E1 | 44 | 14 | 44 | 14 | 0.42 |
| Sh | mIU/mL | 1.5E1 | 1.2E1 | 4.5E1 | 2.6E1 | 9.7E1 | 4.6E1 | 7.8E-2 | 1.4E-1 | 5.7E2 | 1.8E2 | 44 | 14 | 44 | 14 | 0.44 |
| Sj | ng/ml | 4.2E-1 | 4.5E-1 | 4.2E-1 | 4.6E-1 | 8.0E-2 | 1.3E-1 | 2.5E-1 | 3.2E-1 | 5.7E-1 | 7.2E-1 | 44 | 14 | 44 | 14 | 0.59 |
| Rc | pg/ml | 6.9E3 | 5.5E3 | 7.6E3 | 8.3E3 | 5.3E3 | 8.2E3 | 3.9E2 | 5.5E2 | 2.8E4 | 3.9E4 | 111 | 23 | 111 | 23 | 0.48 |
| Rb | pg/ml | 8.8E-1 | 1.0E-9 | 3.1E0 | 2.5E0 | 6.4E0 | 4.2E0 | 1.0E-9 | 1.0E-9 | 5.6E1 | 1.3E1 | 111 | 23 | 111 | 23 | 0.44 |
| Zq | 2.6ng/ml | 3.0E2 | 3.4E2 | 3.0E2 | 3.0E2 | 2.3E2 | 1.7E2 | 1.4E1 | 1.7E1 | 9.7E2 | 5.0E2 | 43 | 13 | 43 | 13 | 0.53 |
| Zw | 2.5ng/ml | 5.2E0 | 5.0E0 | 1.0E1 | 1.2E1 | 1.4E1 | 1.7E1 | 1.4E-1 | 2.4E-1 | 5.9E1 | 6.3E1 | 44 | 14 | 44 | 14 | 0.50 |
| Zx | 2.3mU/ml | 1.3E-1 | 1.5E-1 | 2.3E-1 | 4.9E-1 | 3.4E-1 | 8.4E-1 | 3.2E-2 | 6.1E-2 | 2.1E0 | 2.9E0 | 44 | 14 | 44 | 14 | 0.60 |
| Pz | ng/ml | 3.4E3 | 5.9E3 | 5.4E3 | 7.5E3 | 4.9E3 | 1.1E4 | 1.6E1 | 4.0E1 | 2.9E4 | 7.0E4 | 306 | 43 | 306 | 43 | 0.58 |
| Qa | ng/ml | 3.5E3 | 9.0E3 | 7.0E3 | 1.4E4 | 1.4E4 | 1.2E4 | 1.5E2 | 2.9E2 | 2.2E5 | 3.6E4 | 306 | 43 | 306 | 43 | 0.68 |
| Qb | ng/ml | 1.0E2 | 2.1E2 | 2.2E2 | 3.8E2 | 4.0E2 | 6.5E2 | 7.9E-1 | 8.7E0 | 5.3E3 | 4.1E3 | 306 | 43 | 306 | 43 | 0.63 |
| Qc | ng/ml | 2.0E2 | 3.5E2 | 4.4E2 | 5.9E2 | 6.0E2 | 6.0E2 | 1.0E-9 | 5.8E0 | 4.3E3 | 2.8E3 | 306 | 43 | 306 | 43 | 0.60 |
| Qd | ng/ml | 8.6E3 | 1.4E4 | 2.3E4 | 6.4E4 | 1.2E5 | 8.7E4 | 1.5E2 | 1.2E3 | 2.0E6 | 4.3E5 | 306 | 43 | 306 | 43 | 0.68 |
| Qe | ng/ml | 8.4E2 | 1.9E3 | 2.0E3 | 3.1E3 | 5.9E3 | 3.6E3 | 1.0E-9 | 5.7E1 | 9.7E4 | 1.8E4 | 306 | 43 | 306 | 43 | 0.62 |
| Jd | ng/ml | 7.8E-1 | 1.5E0 | 4.0E0 | 4.3E0 | 1.6E1 | 5.3E0 | 1.0E-9 | 1.0E-9 | 1.2E2 | 1.8E1 | 111 | 23 | 111 | 23 | 0.61 |
| Je | ng/ml | 1.0E-9 | 1.0E-9 | 1.8E0 | 1.5E0 | 5.2E0 | 2.9E0 | 1.0E-9 | 1.0E-9 | 4.6E1 | 1.1E1 | 111 | 23 | 111 | 23 | 0.48 |
| Jf | ng/ml | 1.0E-9 | 1.0E-9 | 1.3E0 | 5.7E-1 | 2.4E0 | 1.2E0 | 1.0E-9 | 1.0E-9 | 1.2E1 | 4.1E0 | 111 | 23 | 111 | 23 | 0.42 |
| Jg | ng/ml | 4.3E2 | 8.6E2 | 7.7E2 | 1.2E3 | 9.6E2 | 1.3E3 | 5.8E0 | 2.4E1 | 1.0E4 | 7.1E3 | 308 | 44 | 308 | 44 | 0.62 |
| Jh | ng/ml | 2.6E0 | 7.0E0 | 2.1E1 | 3.6E1 | 8.9E1 | 5.9E1 | 1.0E-9 | 1.0E-9 | 1.2E3 | 2.9E2 | 308 | 44 | 308 | 44 | 0.69 |
| Ji | ng/ml | 5.5E1 | 1.0E2 | 8.5E1 | 1.9E2 | 1.1E2 | 1.9E2 | 1.1E0 | 8.9E0 | 1.3E3 | 6.9E2 | 308 | 44 | 308 | 44 | 0.68 |
| Sr | pg/mL | 3.8E2 | 6.7E2 | 9.8E2 | 1.5E3 | 2.2E3 | 1.6E3 | 1.0E-9 | 1.0E-9 | 2.1E4 | 5.4E3 | 110 | 23 | 110 | 23 | 0.61 |
| Ss | pg/mL | 7.9E4 | 1.2E5 | 1.5E5 | 1.3E5 | 1.9E5 | 8.2E4 | 2.7E3 | 7.6E3 | 1.3E6 | 2.4E5 | 110 | 23 | 110 | 23 | 0.56 |
| St | pg/mL | 2.4E7 | 5.3E7 | 5.3E7 | 1.7E8 | 8.2E7 | 4.0E8 | 1.0E-9 | 9.9E5 | 5.4E8 | 1.7E9 | 109 | 23 | 109 | 23 | 0.58 |
| Wc | ng/ml | 1.0E-9 | 3.2E-2 | 9.5E-2 | 8.7E-2 | 2.8E-1 | 1.6E-1 | 1.0E-9 | 1.0E-9 | 1.8E0 | 6.3E-1 | 44 | 14 | 44 | 14 | 0.59 |
| Wd | ng/ml | 9.3E0 | 1.3E1 | 2.6E1 | 7.8E1 | 6.7E1 | 1.3E2 | 1.0E0 | 2.3E0 | 3.8E2 | 4.1E2 | 44 | 14 | 44 | 14 | 0.65 |
| We | ng/ml | 2.6E-1 | 5.9E-1 | 1.3E0 | 1.7E0 | 3.5E0 | 2.6E0 | 1.0E-9 | 1.0E-9 | 2.3E1 | 9.7E0 | 44 | 14 | 44 | 14 | 0.66 |
| Wg | ng/ml | 1.0E-9 | 1.0E-9 | 1.3E-2 | 1.0E-9 | 8.1E-2 | 0.0E0 | 1.0E-9 | 1.0E-9 | 5.3E-1 | 1.0E-9 | 44 | 14 | 44 | 14 | 0.48 |
| Wh | ng/ml | 9.2E-3 | 3.1E-2 | 3.6E-2 | 8.2E-2 | 8.4E-2 | 1.3E-1 | 1.0E-9 | 1.0E-9 | 3.6E-1 | 4.2E-1 | 44 | 14 | 44 | 14 | 0.68 |
| Wf | ng/ml | 1.0E-9 | 1.0E-9 | 1.2E-1 | 1.5E-1 | 3.6E-1 | 3.4E-1 | 1.0E-9 | 1.0E-9 | 2.3E0 | 1.2E0 | 44 | 14 | 44 | 14 | 0.54 |
| Ra | pg/ml | 1.0E-9 | 1.0E-9 | 1.0E0 | 3.3E-1 | 6.1E0 | 1.4E0 | 1.0E-9 | 1.0E-9 | 6.4E1 | 6.5E0 | 111 | 23 | 111 | 23 | 0.45 |
| Qz | pg/ml | 9.0E0 | 1.2E1 | 5.1E1 | 4.5E1 | 8.9E1 | 6.3E1 | 1.0E-9 | 1.0E-9 | 4.5E2 | 1.8E2 | 111 | 23 | 111 | 23 | 0.57 |
| Qy | pg/ml | 3.9E-1 | 5.4E-1 | 3.8E0 | 5.4E1 | 2.3E1 | 1.7E2 | 1.0E-9 | 1.0E-9 | 2.3E2 | 7.3E2 | 111 | 23 | 111 | 23 | 0.57 |
| Qx | pg/ml | 1.0E-9 | 1.0E-9 | 3.8E0 | 3.2E0 | 2.1E1 | 8.0E0 | 1.0E-9 | 1.0E-9 | 1.9E2 | 3.1E1 | 111 | 23 | 111 | 23 | 0.55 |
| Qw | pg/ml | 1.0E-9 | 4.5E-1 | 2.5E0 | 4.3E0 | 9.0E0 | 1.1E1 | 1.0E-9 | 1.0E-9 | 6.6E1 | 5.1E1 | 111 | 23 | 111 | 23 | 0.55 |
| Qv | pg/ml | 2.0E4 | 1.2E4 | 4.1E4 | 1.5E4 | 9.9E4 | 1.3E4 | 4.0E2 | 1.0E-9 | 9.4E5 | 5.1E4 | 111 | 23 | 111 | 23 | 0.33 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qu | pg/ml | 6.2E0 | 2.4E1 | 8.4E1 | 1.3E2 | 1.8E2 | 2.1E2 | 1.0E-9 | 1.0E-9 | 9.8E2 | 7.3E2 | 111 | 23 | 111 | 23 | 0.55 |
| Qt | pg/ml | 1.1E1 | 2.1E1 | 4.1E1 | 6.2E1 | 9.9E1 | 9.0E1 | 1.0E-9 | 1.0E-9 | 7.1E2 | 3.3E2 | 111 | 23 | 111 | 23 | 0.64 |
| Qh | ng/ml | 1.8E1 | 3.0E1 | 4.2E1 | 9.2E1 | 6.8E1 | 1.7E2 | 2.5E-1 | 1.6E0 | 4.6E2 | 8.0E2 | 111 | 23 | 111 | 23 | 0.60 |
| Qg | ng/ml | 7.9E0 | 5.3E0 | 1.4E1 | 1.0E1 | 2.7E1 | 1.7E1 | 1.5E-1 | 1.0E0 | 2.7E2 | 8.1E1 | 111 | 23 | 111 | 23 | 0.40 |
| Jj | ng/ml | 5.4E2 | 2.1E2 | 2.1E3 | 4.5E2 | 1.9E4 | 4.6E2 | 2.3E0 | 8.7E0 | 3.4E5 | 2.0E3 | 308 | 44 | 308 | 44 | 0.34 |
| Jk | ng/ml | 2.6E0 | 4.4E0 | 1.8E1 | 4.7E1 | 4.3E1 | 8.1E1 | 1.0E-9 | 4.3E-2 | 2.8E2 | 3.9E2 | 308 | 44 | 308 | 44 | 0.59 |
| Jl | ng/ml | 4.3E-1 | 1.3E0 | 1.8E0 | 2.3E2 | 4.5E0 | 1.5E3 | 1.2E-3 | 5.4E-3 | 4.0E1 | 9.9E3 | 308 | 44 | 308 | 44 | 0.69 |
| Jm | ng/ml | 2.0E1 | 2.0E1 | 7.1E1 | 4.7E1 | 1.8E2 | 6.6E1 | 1.0E-9 | 1.0E-9 | 2.1E3 | 2.9E2 | 308 | 44 | 308 | 44 | 0.50 |
| Jn | pg/ml | 3.2E-1 | 1.0E0 | 5.4E0 | 2.4E1 | 5.0E1 | 1.1E2 | 1.0E-9 | 1.0E-9 | 6.2E2 | 7.3E2 | 308 | 44 | 308 | 44 | 0.64 |
| Jo | pg/ml | 3.8E3 | 3.9E3 | 5.2E3 | 5.5E3 | 6.8E3 | 6.4E3 | 2.0E1 | 2.4E1 | 1.0E5 | 3.6E4 | 308 | 44 | 308 | 44 | 0.49 |
| Jp | pg/ml | 7.0E4 | 9.8E4 | 7.2E4 | 9.6E4 | 3.9E4 | 4.1E4 | 5.8E2 | 4.6E3 | 3.8E5 | 2.1E5 | 308 | 44 | 308 | 44 | 0.69 |
| Jq | pg/ml | 9.3E1 | 1.8E2 | 1.8E2 | 4.0E2 | 5.2E2 | 6.4E2 | 1.0E0 | 7.4E0 | 8.7E3 | 3.3E3 | 308 | 44 | 308 | 44 | 0.66 |
| Jr | pg/ml | 4.1E0 | 1.5E1 | 7.3E1 | 2.5E2 | 6.8E2 | 1.2E3 | 1.0E-9 | 1.0E-9 | 1.1E4 | 7.4E3 | 308 | 44 | 308 | 44 | 0.65 |
| Js | pg/ml | 1.4E1 | 1.9E1 | 7.8E1 | 1.8E2 | 6.0E2 | 6.2E2 | 1.0E-9 | 2.1E0 | 1.0E4 | 3.0E3 | 308 | 44 | 308 | 44 | 0.63 |
| Jt | pg/ml | 2.4E3 | 2.9E3 | 3.1E3 | 5.0E3 | 3.6E3 | 7.4E3 | 2.2E1 | 1.5E2 | 5.2E4 | 4.1E4 | 308 | 44 | 308 | 44 | 0.56 |
| Xa | pg/ml | 3.1E-1 | 1.4E1 | 3.3E1 | 6.3E1 | 1.8E2 | 1.5E2 | 1.0E-9 | 1.0E-9 | 1.2E3 | 5.6E2 | 44 | 14 | 44 | 14 | 0.69 |
| Ye | pg/ml | 1.0E-9 | 1.0E-9 | 3.6E0 | 5.6E0 | 1.2E1 | 1.6E1 | 1.0E-9 | 1.0E-9 | 6.9E1 | 6.1E1 | 44 | 14 | 44 | 14 | 0.55 |
| Tm | pg/ml | 1.0E-9 | 1.0E-9 | 2.6E0 | 2.4E0 | 4.6E0 | 3.9E0 | 1.0E-9 | 1.0E-9 | 1.8E1 | 8.4E0 | 44 | 14 | 44 | 14 | 0.50 |
| Tl | pg/ml | 1.3E-1 | 1.1E-1 | 8.6E-1 | 2.5E-1 | 3.7E0 | 3.2E-1 | 1.0E-9 | 1.0E-9 | 2.5E1 | 8.2E-1 | 44 | 14 | 44 | 14 | 0.45 |
| Ju | mIU/ml | 1.1E1 | 6.4E0 | 2.6E1 | 1.4E1 | 3.7E1 | 1.5E1 | 1.7E-1 | 6.0E-1 | 2.3E2 | 6.2E1 | 111 | 23 | 111 | 23 | 0.44 |
| Jv | mIU/ml | 1.6E1 | 1.1E1 | 4.1E1 | 3.1E1 | 6.5E1 | 4.6E1 | 1.7E-2 | 7.9E-2 | 4.4E2 | 1.4E2 | 111 | 23 | 111 | 23 | 0.43 |
| Jy | ng/ml | 1.6E-3 | 1.6E-3 | 2.3E-3 | 4.6E-3 | 3.7E-3 | 9.1E-3 | 1.0E-9 | 4.5E-4 | 3.9E-2 | 4.1E-2 | 111 | 23 | 111 | 23 | 0.53 |
| Kc | pg/ml | 2.2E1 | 2.8E1 | 4.2E1 | 6.4E1 | 5.0E1 | 7.9E1 | 1.0E-9 | 6.2E0 | 2.7E2 | 3.2E2 | 113 | 22 | 113 | 22 | 0.59 |
| Kd | pg/ml | 1.0E-9 | 1.0E-9 | 5.2E2 | 6.6E2 | 3.6E3 | 1.6E3 | 1.0E-9 | 1.0E-9 | 3.8E4 | 5.2E3 | 113 | 22 | 113 | 22 | 0.52 |
| Ke | pg/ml | 1.3E4 | 1.6E4 | 1.7E4 | 2.5E4 | 3.1E4 | 2.6E4 | 1.0E3 | 6.7E2 | 3.2E5 | 1.1E5 | 113 | 22 | 113 | 22 | 0.59 |
| Kf | pg/mL | 5.9E0 | 8.5E0 | 7.3E0 | 8.5E0 | 9.0E0 | 6.0E0 | 1.0E-9 | 1.0E-9 | 7.8E1 | 2.2E1 | 113 | 22 | 113 | 22 | 0.59 |
| Kg | pg/mL | 9.9E2 | 7.8E2 | 2.0E3 | 3.3E3 | 3.4E3 | 7.9E3 | 7.7E1 | 1.6E2 | 2.7E4 | 3.6E4 | 113 | 22 | 113 | 22 | 0.43 |
| Ki | pg/ml | 6.1E1 | 6.7E1 | 7.1E1 | 7.3E1 | 5.7E1 | 2.6E1 | 1.0E-9 | 6.0E0 | 2.9E2 | 1.1E2 | 113 | 22 | 113 | 22 | 0.59 |
| Kj | pg/ml | 8.3E2 | 7.3E2 | 1.4E3 | 1.4E3 | 1.9E3 | 1.8E3 | 6.6E1 | 3.3E1 | 1.5E4 | 7.7E3 | 113 | 22 | 113 | 22 | 0.46 |
| Kk | pg/ml | 7.1E0 | 9.2E0 | 1.4E1 | 1.4E1 | 1.6E1 | 1.5E1 | 1.0E-9 | 1.0E-9 | 8.1E1 | 5.8E1 | 113 | 22 | 113 | 22 | 0.55 |
| Kl | pg/ml | 1.8E4 | 1.8E4 | 2.8E4 | 2.6E4 | 2.7E4 | 2.0E4 | 2.3E2 | 2.4E2 | 1.3E5 | 5.0E4 | 113 | 22 | 113 | 22 | 0.51 |
| Kn | pg/ml | 2.9E1 | 3.0E1 | 1.1E2 | 9.1E1 | 4.6E2 | 1.4E2 | 1.0E-9 | 1.0E-9 | 4.9E3 | 4.7E2 | 113 | 22 | 113 | 22 | 0.56 |
| Ko | pg/ml | 3.5E2 | 2.0E2 | 5.1E2 | 4.2E2 | 5.9E2 | 4.1E2 | 1.0E-9 | 1.0E-9 | 4.1E3 | 1.5E3 | 113 | 22 | 113 | 22 | 0.48 |
| Kp | pg/ml | 3.6E2 | 3.9E2 | 4.7E2 | 4.0E2 | 1.3E3 | 2.5E2 | 1.0E-9 | 1.0E-9 | 1.3E4 | 8.0E2 | 113 | 22 | 113 | 22 | 0.54 |
| Kq | pg/ml | 3.2E2 | 6.1E2 | 1.9E3 | 1.4E3 | 1.5E4 | 2.6E3 | 5.1E0 | 1.6E0 | 1.6E5 | 1.2E4 | 108 | 22 | 108 | 22 | 0.61 |
| Kr | pg/ml | 3.7E-1 | 2.3E-1 | 6.0E0 | 1.5E0 | 4.0E1 | 2.1E0 | 1.0E-9 | 1.0E-9 | 4.2E2 | 6.0E0 | 108 | 22 | 108 | 22 | 0.48 |
| Ks | pg/ml | 1.6E4 | 1.1E4 | 2.1E4 | 1.8E4 | 1.8E4 | 1.8E4 | 4.5E2 | 5.1E1 | 7.9E4 | 5.0E4 | 108 | 22 | 108 | 22 | 0.44 |
| Ps | ng/ml | 1.4E2 | 5.0E2 | 5.0E2 | 1.9E3 | 1.4E3 | 3.5E3 | 1.6E0 | 5.5E0 | 9.0E3 | 1.2E4 | 44 | 14 | 44 | 14 | 0.68 |
| Kx | ng/ml | 5.5E-4 | 4.6E-3 | 7.5E-3 | 1.3E-2 | 1.6E-2 | 2.1E-2 | 1.0E-9 | 1.0E-9 | 1.0E-1 | 7.9E-2 | 112 | 22 | 112 | 22 | 0.58 |
| Ky | ng/ml | 1.1E-1 | 4.8E-1 | 3.3E-1 | 7.9E-1 | 6.9E-1 | 1.0E0 | 1.0E-9 | 1.0E-9 | 5.1E0 | 4.4E0 | 112 | 22 | 112 | 22 | 0.70 |
| Kz | ng/ml | 1.0E-9 | 1.0E-9 | 3.8E-3 | 4.6E-3 | 5.9E-3 | 7.6E-3 | 1.0E-9 | 1.0E-9 | 1.8E-2 | 2.5E-2 | 112 | 22 | 112 | 22 | 0.51 |
| Rz | ng/ml | 3.0E-1 | 9.5E-1 | 7.7E-1 | 1.4E0 | 1.3E0 | 1.9E0 | 1.1E-2 | 4.6E-3 | 6.7E0 | 7.5E0 | 44 | 14 | 44 | 14 | 0.61 |
| Ry | ng/ml | 1.6E-2 | 2.0E-2 | 3.1E-2 | 2.7E-2 | 5.5E-2 | 2.6E-2 | 1.0E-9 | 1.0E-9 | 3.5E-1 | 7.5E-2 | 44 | 14 | 44 | 14 | 0.51 |
| Rx | ng/ml | 1.0E-9 | 1.7E-3 | 1.1E-3 | 2.6E-3 | 2.0E-3 | 3.0E-3 | 1.0E-9 | 1.0E-9 | 8.6E-3 | 8.6E-3 | 44 | 14 | 44 | 14 | 0.63 |
| Ld | pg/ml | 1.0E-9 | 1.0E-9 | 3.9E0 | 4.0E0 | 9.0E0 | 7.4E0 | 1.0E-9 | 1.0E-9 | 6.9E1 | 2.9E1 | 114 | 21 | 114 | 21 | 0.51 |
| Lh | pg/ml | 1.3E4 | 3.0E4 | 2.2E4 | 4.5E4 | 4.2E4 | 6.8E4 | 1.0E-9 | 1.0E-9 | 4.8E5 | 4.1E5 | 309 | 44 | 309 | 44 | 0.64 |
| Li | pg/ml | 3.5E3 | 6.6E3 | 2.0E4 | 3.9E4 | 9.4E4 | 8.6E4 | 1.2E1 | 1.3E1 | 1.3E6 | 4.1E5 | 309 | 44 | 309 | 44 | 0.56 |
| Lj | pg/ml | 3.0E3 | 2.9E3 | 2.0E4 | 2.8E4 | 5.7E4 | 6.7E4 | 1.0E-9 | 1.0E-9 | 4.3E5 | 3.9E5 | 309 | 44 | 309 | 44 | 0.50 |
| Lp | pg/ml | 1.2E1 | 4.8E0 | 9.8E1 | 1.6E2 | 2.5E2 | 4.0E2 | 1.0E-9 | 1.0E-9 | 1.1E3 | 1.4E3 | 44 | 14 | 44 | 14 | 0.43 |
| Lt | pg/ml | 1.0E-9 | 1.0E-9 | 1.9E0 | 1.0E-9 | 6.3E0 | 0.0E0 | 1.0E-9 | 1.0E-9 | 2.5E1 | 1.0E-9 | 44 | 14 | 44 | 14 | 0.45 |
| Rv | ng/ml | 5.0E-4 | 5.0E-4 | 1.5E-3 | 1.4E-3 | 3.2E-3 | 2.4E-3 | 1.0E-9 | 1.0E-9 | 1.6E-2 | 9.2E-3 | 44 | 14 | 44 | 14 | 0.56 |
| Ru | ng/ml | 1.0E-9 | 1.0E-9 | 2.7E-2 | 5.0E-3 | 8.9E-2 | 1.9E-2 | 1.0E-9 | 1.0E-9 | 3.5E-1 | 7.1E-2 | 44 | 14 | 44 | 14 | 0.49 |
| Rt | ng/ml | 7.8E-2 | 5.8E-2 | 2.7E-1 | 2.0E-1 | 1.1E0 | 2.5E-1 | 6.5E-3 | 1.3E-3 | 7.4E0 | 6.3E-1 | 44 | 14 | 44 | 14 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Yl | pg/ml | 1.3E1 | 6.9E0 | 2.4E1 | 1.3E1 | 3.5E1 | 1.5E1 | 1.0E-9 | 1.0E-9 | 2.2E2 | 4.9E1 | 44 | 14 | 44 | 14 | 0.35 |
| Rm | ng/ml | 1.8E1 | 2.6E1 | 4.9E1 | 5.6E1 | 8.9E1 | 6.7E1 | 2.2E-1 | 2.3E-1 | 6.5E2 | 2.5E2 | 110 | 23 | 110 | 23 | 0.55 |
| Rh | ng/ml | 1.8E2 | 1.6E2 | 4.9E2 | 9.2E2 | 1.7E3 | 3.5E3 | 7.5E0 | 2.5E1 | 1.7E4 | 1.7E4 | 110 | 23 | 110 | 23 | 0.42 |
| Ri | ng/ml | 4.4E-2 | 1.0E-9 | 3.9E0 | 3.6E0 | 7.8E0 | 9.4E0 | 1.0E-9 | 1.0E-9 | 4.9E1 | 4.5E1 | 110 | 23 | 110 | 23 | 0.47 |
| Rg | ng/ml | 1.0E-9 | 1.0E-9 | 9.7E-2 | 1.4E-2 | 4.7E-1 | 4.4E-2 | 1.0E-9 | 1.0E-9 | 3.3E0 | 2.1E-1 | 110 | 23 | 110 | 23 | 0.51 |
| Rj | ng/ml | 1.0E-9 | 1.0E-9 | 3.4E0 | 5.2E-1 | 2.6E1 | 1.0E0 | 1.0E-9 | 1.0E-9 | 2.7E2 | 3.2E0 | 110 | 23 | 110 | 23 | 0.48 |
| Rf | ng/ml | 3.9E-1 | 3.3E-1 | 7.6E-1 | 2.3E0 | 9.9E-1 | 4.6E0 | 2.1E-2 | 2.1E-2 | 6.2E0 | 1.7E1 | 110 | 23 | 110 | 23 | 0.52 |
| Ql | pg/ml | 1.7E0 | 1.1E1 | 1.1E1 | 1.6E1 | 2.4E1 | 2.0E1 | 1.0E-9 | 1.0E-9 | 1.8E2 | 5.6E1 | 111 | 23 | 111 | 23 | 0.60 |
| Qm | pg/ml | 1.7E0 | 1.0E1 | 2.0E1 | 2.4E1 | 3.7E1 | 2.9E1 | 1.0E-9 | 1.0E-9 | 1.9E2 | 8.6E1 | 111 | 23 | 111 | 23 | 0.58 |
| Qn | pg/ml | 6.1E-1 | 1.3E0 | 5.3E0 | 1.5E1 | 2.2E1 | 4.3E1 | 1.0E-9 | 1.0E-9 | 2.2E2 | 2.0E2 | 111 | 23 | 111 | 23 | 0.57 |
| Nv | pg/ml | 3.8E3 | 1.0E4 | 8.3E3 | 2.5E4 | 1.6E4 | 3.9E4 | 1.0E-9 | 1.9E1 | 1.3E5 | 1.6E5 | 310 | 44 | 310 | 44 | 0.69 |
| Nw | pg/ml | 9.3E3 | 1.7E4 | 1.3E4 | 3.1E4 | 1.6E4 | 4.5E4 | 2.0E2 | 1.9E2 | 2.1E5 | 2.2E5 | 310 | 44 | 310 | 44 | 0.68 |
| Nx | pg/ml | 2.2E2 | 2.4E2 | 4.3E2 | 5.9E2 | 6.4E2 | 6.3E2 | 1.0E-9 | 1.0E-9 | 4.1E3 | 2.3E3 | 310 | 44 | 310 | 44 | 0.60 |
| Ny | pg/ml | 6.4E0 | 1.8E1 | 1.1E2 | 1.1E2 | 1.4E3 | 2.2E2 | 1.0E-9 | 1.0E-9 | 2.5E4 | 1.2E3 | 310 | 44 | 310 | 44 | 0.64 |
| Oa | pg/ml | 1.6E2 | 2.2E2 | 4.4E2 | 6.9E2 | 7.2E2 | 9.2E2 | 1.0E-9 | 1.0E-9 | 4.5E3 | 3.4E3 | 111 | 23 | 111 | 23 | 0.56 |
| Op | pg/ml | 4.4E5 | 4.5E5 | 4.4E5 | 4.5E5 | 1.5E5 | 2.1E5 | 5.2E4 | 1.4E5 | 7.3E5 | 7.5E5 | 44 | 14 | 44 | 14 | 0.51 |
| Oe | pg/ml | 2.5E1 | 1.0E-9 | 2.4E2 | 2.6E2 | 3.8E2 | 3.9E2 | 1.0E-9 | 1.0E-9 | 2.3E3 | 1.4E3 | 308 | 44 | 308 | 44 | 0.49 |
| Of | pg/ml | 1.3E2 | 1.7E2 | 5.0E3 | 5.1E3 | 2.1E4 | 1.9E4 | 1.0E-9 | 1.0E-9 | 1.9E5 | 1.2E5 | 310 | 44 | 310 | 44 | 0.50 |
| Og | pg/ml | 6.3E-2 | 7.7E-2 | 3.8E-1 | 1.2E-1 | 1.6E0 | 1.8E-1 | 1.0E-9 | 1.0E-9 | 1.9E1 | 9.4E-1 | 310 | 44 | 310 | 44 | 0.49 |
| Oh | pg/ml | 2.3E0 | 6.9E0 | 1.3E1 | 4.1E2 | 9.1E1 | 2.4E3 | 1.0E-9 | 1.0E-9 | 1.4E3 | 1.6E4 | 310 | 44 | 310 | 44 | 0.69 |
| Oi | pg/ml | 1.9E0 | 2.2E0 | 4.9E0 | 5.4E0 | 7.9E0 | 7.6E0 | 1.0E-9 | 1.0E-9 | 6.7E1 | 3.1E1 | 310 | 44 | 310 | 44 | 0.50 |
| Ok | pg/ml | 3.8E2 | 5.6E2 | 5.2E2 | 1.1E3 | 6.1E2 | 1.4E3 | 2.9E1 | 1.5E1 | 7.8E3 | 7.0E3 | 310 | 44 | 310 | 44 | 0.63 |
| Om | pg/ml | 4.1E2 | 8.7E2 | 9.3E2 | 1.5E3 | 3.6E3 | 1.7E3 | 1.0E-9 | 1.0E-9 | 5.1E4 | 6.9E3 | 310 | 44 | 310 | 44 | 0.67 |
| On | pg/ml | 1.7E2 | 4.3E2 | 2.8E2 | 8.2E2 | 4.2E2 | 1.4E3 | 1.0E-9 | 1.0E1 | 4.5E3 | 8.5E3 | 310 | 44 | 310 | 44 | 0.71 |
| Or | pg/ml | 9.6E0 | 3.5E1 | 3.3E1 | 9.1E1 | 7.2E1 | 1.3E2 | 1.0E-9 | 1.0E-9 | 5.1E2 | 4.5E2 | 114 | 22 | 114 | 22 | 0.64 |
| Ow | pg/ml | 3.7E1 | 5.4E1 | 1.9E2 | 3.8E2 | 8.1E2 | 7.6E2 | 1.0E-9 | 1.0E-9 | 8.1E3 | 3.0E3 | 114 | 22 | 114 | 22 | 0.60 |
| Ou | pg/ml | 5.2E2 | 6.7E2 | 1.0E3 | 2.4E3 | 1.7E3 | 3.0E3 | 1.0E-9 | 2.0E1 | 9.8E3 | 1.1E4 | 114 | 22 | 114 | 22 | 0.60 |
| Ul | ng/ml | 1.0E-9 | 1.0E-9 | 1.7E0 | 1.8E-1 | 7.1E0 | 8.8E-1 | 1.0E-9 | 1.0E-9 | 5.6E1 | 4.2E0 | 112 | 23 | 112 | 23 | 0.48 |
| Uo | ng/ml | 1.0E-9 | 1.0E-9 | 8.8E-2 | 1.1E-2 | 2.3E-1 | 5.0E-2 | 1.0E-9 | 1.0E-9 | 1.3E0 | 2.4E-1 | 112 | 23 | 112 | 23 | 0.39 |
| Uk | ng/ml | 1.0E-9 | 1.0E-9 | 7.1E-3 | 8.3E-4 | 3.4E-2 | 2.3E-3 | 1.0E-9 | 1.0E-9 | 3.2E-1 | 1.1E-2 | 112 | 23 | 112 | 23 | 0.43 |
| Um | ng/ml | 1.0E-9 | 1.0E-9 | 2.8E-1 | 1.0E-9 | 6.6E-1 | 0.0E0 | 1.0E-9 | 1.0E-9 | 3.6E0 | 1.0E-9 | 112 | 23 | 112 | 23 | 0.35 |
| Uf | ng/ml | 5.8E-2 | 1.5E-1 | 1.2E-1 | 4.1E-1 | 1.8E-1 | 1.0E0 | 2.7E-3 | 1.0E-3 | 1.1E0 | 5.1E0 | 112 | 23 | 112 | 23 | 0.62 |
| Uh | ng/ml | 2.2E0 | 3.4E0 | 3.4E0 | 5.0E0 | 3.2E0 | 4.9E0 | 3.6E-2 | 4.7E-2 | 1.5E1 | 1.8E1 | 112 | 23 | 112 | 23 | 0.59 |
| Un | ng/ml | 1.7E0 | 1.7E0 | 2.1E0 | 2.7E0 | 2.5E0 | 2.0E0 | 3.5E-1 | 3.4E-1 | 2.5E1 | 8.0E0 | 112 | 23 | 112 | 23 | 0.60 |
| Ug | ng/ml | 1.1E1 | 8.8E0 | 2.3E1 | 1.4E1 | 2.8E1 | 1.5E1 | 1.5E0 | 1.0E0 | 1.6E2 | 6.9E1 | 112 | 23 | 112 | 23 | 0.41 |
| Ur | ng/ml | 1.1E-1 | 7.5E-2 | 3.7E-1 | 3.1E-1 | 9.7E-1 | 5.4E-1 | 1.0E-9 | 1.0E-9 | 7.3E0 | 2.3E0 | 111 | 23 | 111 | 23 | 0.45 |
| Up | ng/ml | 1.0E-9 | 1.0E-9 | 2.4E-2 | 5.4E-3 | 2.3E-1 | 1.6E-2 | 1.0E-9 | 1.0E-9 | 2.4E0 | 7.4E-2 | 111 | 23 | 111 | 23 | 0.58 |
| Us | ng/ml | 2.1E-3 | 6.4E-3 | 3.2E-2 | 1.6E-2 | 1.6E-1 | 2.0E-2 | 1.0E-9 | 1.0E-9 | 1.7E0 | 6.4E-2 | 111 | 23 | 111 | 23 | 0.56 |
| Uv | ng/ml | 2.3E-3 | 4.9E-3 | 1.7E-2 | 1.3E-2 | 5.7E-2 | 3.2E-2 | 1.0E-9 | 1.0E-9 | 4.1E-1 | 1.5E-1 | 111 | 23 | 111 | 23 | 0.56 |
| Ut | ng/ml | 6.7E-1 | 1.9E0 | 2.7E0 | 6.1E0 | 8.1E0 | 8.3E0 | 1.0E-9 | 9.2E-2 | 6.5E1 | 3.0E1 | 111 | 23 | 111 | 23 | 0.68 |
| Uu | ng/ml | 6.9E0 | 6.4E0 | 7.4E0 | 7.3E0 | 4.9E0 | 5.3E0 | 5.4E-1 | 8.1E-1 | 2.9E1 | 2.3E1 | 111 | 23 | 111 | 23 | 0.49 |
| Uw | ng/ml | 2.1E0 | 4.4E0 | 3.4E0 | 4.1E0 | 6.0E0 | 2.4E0 | 1.5E-1 | 1.0E-9 | 3.9E1 | 7.1E0 | 45 | 14 | 45 | 14 | 0.68 |
| Vb | ng/ml | 1.1E0 | 1.1E0 | 1.2E0 | 9.3E-1 | 9.0E-1 | 4.7E-1 | 2.8E-1 | 8.5E-2 | 6.4E0 | 1.4E0 | 45 | 14 | 45 | 14 | 0.43 |
| Vc | ng/ml | 1.0E-9 | 1.0E-9 | 4.5E-3 | 9.5E-4 | 2.0E-2 | 3.6E-3 | 1.0E-9 | 1.0E-9 | 1.1E-1 | 1.3E-2 | 45 | 14 | 45 | 14 | 0.50 |
| Uy | ng/ml | 1.4E0 | 9.3E-1 | 9.2E0 | 3.5E0 | 2.0E1 | 1.0E1 | 8.7E-2 | 2.0E-2 | 9.9E1 | 3.8E1 | 45 | 14 | 45 | 14 | 0.36 |
| Uz | ng/ml | 1.0E-9 | 1.0E-9 | 7.4E-1 | 2.2E-2 | 4.9E0 | 8.3E-2 | 1.0E-9 | 1.0E-9 | 3.3E1 | 3.1E-1 | 45 | 14 | 45 | 14 | 0.51 |
| Ux | ng/ml | 1.7E2 | 1.8E2 | 1.9E2 | 1.9E2 | 1.3E2 | 1.5E2 | 1.2E1 | 4.5E0 | 4.8E2 | 4.9E2 | 45 | 14 | 45 | 14 | 0.48 |
| Va | ng/ml | 1.4E1 | 3.6E0 | 2.5E1 | 1.5E1 | 2.9E1 | 2.2E1 | 3.1E-1 | 3.6E-1 | 1.2E2 | 6.2E1 | 45 | 14 | 45 | 14 | 0.36 |
| Vh | ng/ml | 1.2E-2 | 1.9E-2 | 3.8E-2 | 1.9E-2 | 1.3E-1 | 1.6E-2 | 3.9E-4 | 1.0E-3 | 8.6E-1 | 5.8E-2 | 45 | 14 | 45 | 14 | 0.54 |
| Vi | ng/ml | 3.4E-3 | 1.9E-2 | 4.7E-2 | 3.1E-2 | 2.7E-1 | 3.7E-2 | 1.0E-9 | 1.5E-4 | 1.8E0 | 1.2E-1 | 45 | 14 | 45 | 14 | 0.71 |
| Vj | ng/ml | 2.5E1 | 7.5E1 | 4.9E1 | 1.4E2 | 5.4E1 | 1.8E2 | 4.6E0 | 1.4E0 | 2.5E2 | 6.5E2 | 45 | 13 | 45 | 13 | 0.69 |
| Vp | ng/ml | 1.0E-9 | 1.0E-9 | 5.6E-1 | 3.6E-1 | 4.7E0 | 1.2E0 | 1.0E-9 | 1.0E-9 | 4.9E1 | 5.6E0 | 112 | 23 | 112 | 23 | 0.61 |
| Vt | ng/ml | 6.2E0 | 6.0E0 | 9.3E0 | 1.0E1 | 1.5E1 | 1.0E1 | 9.6E-1 | 5.6E-1 | 1.6E2 | 3.8E1 | 112 | 23 | 112 | 23 | 0.52 |

| Code | Units | Median | | Average | | Standard Deviation | | Minimum | | Maximum | | Number of Samples | | Number of Patients | | AUC |
|------|-------|--------|--|---------|--|--------------------|--|---------|--|---------|--|-------------------|--|--------------------|--|-----|
| | | NonDis | Dis | NonDis | Dis | NonDis | Dis | NonDis | Dis | NonDis | Dis | NonDis | Dis | NonDis | Dis | |
| Vu | ng/ml | 1.0E-9 | 1.5E0 | 1.2E0 | 3.4E0 | 3.0E0 | 4.5E0 | 1.0E-9 | 1.0E-9 | 2.2E1 | 1.3E1 | 109 | 22 | 109 | 22 | 0.66 |
| Vq | ng/ml | 1.6E2 | 2.1E2 | 6.9E2 | 8.7E2 | 1.6E3 | 1.4E3 | 9.2E-1 | 6.5E-1 | 1.2E4 | 4.9E3 | 88 | 20 | 88 | 20 | 0.57 |
| Vo | ng/ml | 2.6E1 | 2.5E1 | 2.5E1 | 2.4E1 | 5.3E0 | 6.9E0 | 4.9E0 | 1.9E0 | 4.8E1 | 3.4E1 | 112 | 23 | 112 | 23 | 0.50 |
| Vs | ng/ml | 1.0E-9 | 6.3E-1 | 7.8E0 | 8.8E0 | 4.4E1 | 1.4E1 | 1.0E-9 | 1.0E-9 | 4.5E2 | 4.9E1 | 110 | 22 | 110 | 22 | 0.61 |
| Vv | ng/ml | 2.6E0 | 5.1E0 | 5.2E0 | 1.0E1 | 9.5E0 | 1.2E1 | 1.0E-9 | 1.0E-9 | 8.4E1 | 4.4E1 | 111 | 23 | 111 | 23 | 0.61 |
| Vw | ng/ml | 3.6E1 | 4.4E1 | 3.4E1 | 3.9E1 | 1.7E1 | 2.3E1 | 3.1E0 | 2.5E0 | 6.7E1 | 6.9E1 | 45 | 14 | 45 | 14 | 0.59 |
| Oy | pg/ml | 4.8E-1 | 3.9E-1 | 6.4E0 | 3.5E0 | 3.2E1 | 8.4E0 | 1.0E-9 | 1.0E-9 | 4.0E2 | 4.0E1 | 309 | 44 | 309 | 44 | 0.51 |
| Oz | pg/ml | 1.0E-9 | 5.4E-2 | 3.2E-1 | 9.2E-1 | 1.7E0 | 4.2E0 | 1.0E-9 | 1.0E-9 | 2.9E1 | 2.8E1 | 309 | 44 | 309 | 44 | 0.56 |
| Pa | pg/ml | 3.9E-1 | 4.3E-1 | 1.7E0 | 7.1E0 | 8.3E0 | 3.4E1 | 1.0E-9 | 1.0E-9 | 1.0E2 | 2.3E2 | 309 | 44 | 309 | 44 | 0.57 |
| Pb | pg/ml | 1.0E-9 | 1.0E-9 | 1.8E0 | 7.5E-1 | 2.8E1 | 4.2E0 | 1.0E-9 | 1.0E-9 | 4.9E2 | 2.8E1 | 309 | 44 | 309 | 44 | 0.52 |
| Pc | pg/ml | 2.0E-2 | 2.6E-1 | 3.7E-1 | 8.8E0 | 9.2E-1 | 5.0E1 | 1.0E-9 | 1.0E-9 | 1.4E1 | 3.3E2 | 309 | 44 | 309 | 44 | 0.57 |
| Pd | pg/ml | 1.6E0 | 1.7E0 | 7.0E0 | 6.3E0 | 4.9E1 | 1.2E1 | 1.0E-9 | 1.0E-9 | 8.4E2 | 5.5E1 | 309 | 44 | 309 | 44 | 0.53 |
| Pe | pg/ml | 2.1E1 | 4.4E1 | 1.5E2 | 5.2E2 | 6.5E2 | 2.3E3 | 1.0E-9 | 1.0E-9 | 6.7E3 | 1.5E4 | 309 | 44 | 309 | 44 | 0.62 |
| Pf | pg/ml | 1.6E0 | 5.0E0 | 1.6E1 | 2.6E1 | 9.4E1 | 6.9E1 | 1.0E-9 | 1.0E-9 | 1.5E3 | 4.3E2 | 309 | 44 | 309 | 44 | 0.61 |
| Pg | pg/ml | 3.7E0 | 1.3E1 | 7.5E1 | 1.4E2 | 5.5E2 | 3.2E2 | 1.0E-9 | 1.2E-1 | 7.7E3 | 1.3E3 | 309 | 44 | 309 | 44 | 0.68 |
| Ph | ng/ml | 1.4E-1 | 2.3E-1 | 3.6E-1 | 5.2E-1 | 6.5E-1 | 7.1E-1 | 1.0E-9 | 1.0E-9 | 5.4E0 | 2.8E0 | 114 | 22 | 114 | 22 | 0.57 |
| Pi | ng/ml | 2.0E-1 | 3.0E-1 | 1.0E0 | 5.7E-1 | 7.7E0 | 9.9E-1 | 1.0E-9 | 1.0E-9 | 8.2E1 | 4.8E0 | 114 | 22 | 114 | 22 | 0.63 |
| Pj | ng/mL | 5.6E0 | 4.8E0 | 6.2E0 | 6.0E0 | 4.3E0 | 5.5E0 | 4.9E-1 | 4.0E-1 | 3.1E1 | 2.3E1 | 114 | 22 | 114 | 22 | 0.44 |
| Pk | ng/ml | 8.8E-3 | 1.3E-2 | 2.8E-2 | 1.9E-2 | 1.4E-1 | 2.4E-2 | 1.0E-9 | 1.0E-9 | 1.5E0 | 1.1E-1 | 114 | 22 | 114 | 22 | 0.60 |
| aA | mg/dL | 8.8E-1 | 1.2E0 | 9.8E-1 | 1.6E0 | 4.8E-1 | 1.0E0 | 3.0E-1 | 4.0E-1 | 4.1E0 | 4.7E0 | 474 | 63 | 474 | 63 | 0.73 |
| aC | mg/mL | 2.3E0 | 1.9E0 | 2.7E0 | 2.4E0 | 1.3E0 | 1.5E0 | 7.5E-1 | 9.7E-1 | 7.4E0 | 6.7E0 | 149 | 26 | 149 | 26 | 0.40 |
| aD | ug/mL | 2.9E0 | 3.5E0 | 4.7E0 | 4.2E0 | 5.0E0 | 2.8E0 | 7.5E-1 | 1.1E0 | 3.5E1 | 1.1E1 | 149 | 26 | 149 | 26 | 0.52 |
| aE | mg/mL | 5.8E-1 | 5.7E-1 | 5.9E-1 | 5.9E-1 | 1.8E-1 | 1.4E-1 | 1.8E-1 | 3.4E-1 | 1.2E0 | 1.0E0 | 149 | 26 | 149 | 26 | 0.49 |
| aF | ng/mL | 2.2E0 | 2.7E0 | 4.6E0 | 8.1E0 | 7.1E0 | 1.0E1 | 4.3E-3 | 4.3E-3 | 5.0E1 | 3.5E1 | 149 | 26 | 149 | 26 | 0.58 |
| aG | mg/mL | 1.4E-1 | 1.1E-1 | 1.6E-1 | 1.4E-1 | 8.9E-2 | 6.9E-2 | 3.2E-2 | 6.9E-2 | 4.8E-1 | 3.5E-1 | 149 | 26 | 149 | 26 | 0.43 |
| aH | ug/mL | 7.4E1 | 6.1E1 | 7.8E1 | 7.3E1 | 4.0E1 | 3.9E1 | 8.9E0 | 2.3E1 | 2.0E2 | 2.0E2 | 149 | 26 | 149 | 26 | 0.46 |
| aI | ug/mL | 1.7E2 | 1.5E2 | 1.8E2 | 1.6E2 | 6.3E1 | 5.1E1 | 3.2E1 | 7.5E1 | 3.4E2 | 2.9E2 | 149 | 26 | 149 | 26 | 0.39 |
| aJ | ug/mL | 2.2E0 | 4.2E0 | 3.0E0 | 5.4E0 | 2.1E0 | 4.6E0 | 8.2E-1 | 1.5E0 | 1.2E1 | 2.3E1 | 149 | 26 | 149 | 26 | 0.71 |
| aK | ng/mL | 1.3E0 | 1.3E0 | 2.0E0 | 1.5E0 | 2.0E0 | 1.4E0 | 2.9E-4 | 1.3E-1 | 1.0E1 | 6.5E0 | 149 | 26 | 149 | 26 | 0.45 |
| aL | mg/mL | 7.4E-1 | 7.2E-1 | 7.8E-1 | 6.9E-1 | 2.6E-1 | 2.0E-1 | 2.2E-1 | 3.2E-1 | 1.7E0 | 1.2E0 | 149 | 26 | 149 | 26 | 0.41 |
| aM | U/mL | 1.7E1 | 2.6E1 | 4.0E1 | 7.5E1 | 8.1E1 | 1.4E2 | 4.2E-2 | 4.2E-2 | 8.2E2 | 6.8E2 | 149 | 26 | 149 | 26 | 0.64 |
| aN | U/mL | 1.4E1 | 2.3E1 | 2.5E1 | 2.8E1 | 4.4E1 | 2.8E1 | 2.5E-3 | 4.8E0 | 3.8E2 | 1.3E2 | 149 | 26 | 149 | 26 | 0.61 |
| aO | pg/mL | 3.7E1 | 1.8E2 | 3.8E2 | 7.9E2 | 9.5E2 | 1.1E3 | 6.0E-2 | 2.1E0 | 6.6E3 | 3.9E3 | 149 | 26 | 149 | 26 | 0.70 |
| aP | ng/mL | 1.6E0 | 2.9E0 | 2.1E0 | 4.0E0 | 2.5E0 | 5.2E0 | 4.5E-1 | 1.1E0 | 2.8E1 | 2.8E1 | 149 | 26 | 149 | 26 | 0.73 |
| aQ | ng/mL | 2.5E-1 | 2.4E-1 | 3.6E-1 | 3.1E-1 | 3.3E-1 | 2.3E-1 | 2.0E-4 | 5.2E-2 | 2.0E0 | 9.2E-1 | 149 | 26 | 149 | 26 | 0.48 |
| aR | ng/mL | 1.7E0 | 2.8E0 | 2.8E0 | 4.1E0 | 4.0E0 | 3.8E0 | 2.6E-1 | 5.6E-1 | 3.4E1 | 1.5E1 | 149 | 26 | 149 | 26 | 0.65 |
| aS | ng/mL | 3.7E-1 | 6.0E-1 | 1.0E0 | 9.9E-1 | 2.8E0 | 1.1E0 | 4.2E-3 | 2.8E-2 | 3.3E1 | 4.9E0 | 149 | 26 | 149 | 26 | 0.58 |
| aU | pg/mL | 6.8E1 | 4.7E1 | 1.0E2 | 7.9E1 | 1.1E2 | 1.0E2 | 7.4E-2 | 7.4E-2 | 7.0E2 | 5.1E2 | 149 | 26 | 149 | 26 | 0.41 |
| aV | ng/mL | 6.1E-1 | 4.7E-1 | 1.1E0 | 6.5E-1 | 2.8E0 | 5.4E-1 | 7.6E-4 | 1.2E-1 | 3.3E1 | 2.4E0 | 149 | 26 | 149 | 26 | 0.46 |
| aW | pg/mL | 1.9E1 | 2.2E1 | 2.3E1 | 2.2E1 | 3.6E1 | 1.2E1 | 7.2E-2 | 7.2E-2 | 4.2E2 | 4.7E1 | 149 | 26 | 149 | 26 | 0.58 |
| aX | ng/mL | 7.6E0 | 1.3E1 | 1.3E1 | 3.3E1 | 2.1E1 | 6.5E1 | 3.0E-1 | 1.7E0 | 2.2E2 | 3.1E2 | 149 | 26 | 149 | 26 | 0.57 |
| aY | pg/mL | 5.3E1 | 5.9E1 | 7.5E1 | 8.2E1 | 1.1E2 | 7.6E1 | 4.1E-1 | 1.2E1 | 1.2E3 | 3.9E2 | 149 | 26 | 149 | 26 | 0.57 |
| aZ | pg/mL | 2.2E2 | 3.8E2 | 5.0E2 | 1.7E3 | 1.1E3 | 2.4E3 | 1.7E0 | 1.5E1 | 1.2E4 | 7.9E3 | 149 | 26 | 149 | 26 | 0.65 |
| bA | ng/mL | 1.2E1 | 1.0E2 | 6.0E1 | 1.6E2 | 1.5E2 | 2.9E2 | 3.0E-2 | 3.0E-2 | 9.4E2 | 1.5E3 | 149 | 26 | 149 | 26 | 0.72 |
| bB | ng/mL | 2.8E2 | 2.1E2 | 3.1E2 | 2.7E2 | 1.8E2 | 1.8E2 | 2.1E0 | 6.5E1 | 9.5E2 | 7.4E2 | 149 | 26 | 149 | 26 | 0.41 |
| bC | ng/mL | 3.2E2 | 3.3E2 | 5.9E2 | 9.8E2 | 7.8E2 | 1.3E3 | 1.4E1 | 4.6E1 | 4.7E3 | 4.0E3 | 149 | 26 | 149 | 26 | 0.55 |
| bE | mg/mL | 5.2E0 | 5.1E0 | 5.5E0 | 5.6E0 | 2.1E0 | 2.5E0 | 1.3E0 | 2.6E0 | 1.2E1 | 1.2E1 | 149 | 26 | 149 | 26 | 0.49 |
| bF | pg/mL | 3.3E1 | 6.6E1 | 3.0E2 | 6.0E2 | 1.3E3 | 1.5E3 | 5.0E-2 | 1.1E1 | 1.1E4 | 6.3E3 | 149 | 26 | 149 | 26 | 0.63 |
| bG | ng/mL | 1.5E0 | 1.8E0 | 2.8E0 | 5.0E0 | 3.5E0 | 7.7E0 | 1.1E-1 | 1.6E-1 | 2.3E1 | 3.0E1 | 149 | 26 | 149 | 26 | 0.56 |
| bH | pg/mL | 5.7E-1 | 5.7E-1 | 6.5E0 | 5.0E0 | 2.5E1 | 6.3E0 | 5.7E-1 | 5.7E-1 | 2.8E2 | 2.3E1 | 149 | 26 | 149 | 26 | 0.55 |
| bI | ng/mL | 4.0E-3 | 4.0E-3 | 9.3E-2 | 9.4E-2 | 1.9E-1 | 2.5E-1 | 4.0E-3 | 4.0E-3 | 8.8E-1 | 9.8E-1 | 149 | 26 | 149 | 26 | 0.47 |
| bJ | mg/mL | 1.9E0 | 2.2E0 | 2.4E0 | 2.5E0 | 2.0E0 | 2.0E0 | 2.5E-4 | 2.5E-4 | 1.1E1 | 8.9E0 | 149 | 26 | 149 | 26 | 0.53 |
| bL | pg/mL | 3.7E0 | 5.8E0 | 9.5E0 | 7.9E0 | 1.2E1 | 6.7E0 | 4.6E-2 | 4.6E-2 | 6.0E1 | 2.4E1 | 149 | 26 | 149 | 26 | 0.55 |

| Code | Units | Median | | Average | | Standard Deviation | | Minimum | | Maximum | | Number of Samples | | Number of Patients | | AUC |
|------|-------|--------|------|---------|------|-------|------|--------|------|--------|------|--------|-----|--------|-----|------|
| | | NonDis | Dis | NonDis | Dis | NonDis | Dis | NonDis | Dis | NonDis | Dis | NonDis | Dis | NonDis | Dis | |
| bM | mg/mL | 1.8E0 | 1.9E0 | 2.1E0 | 2.3E0 | 1.4E0 | 1.8E0 | 1.8E-2 | 1.6E-2 | 7.9E0 | 8.6E0 | 149 | 26 | 149 | 26 | 0.51 |
| bN | ng/mL | 3.5E1 | 2.5E1 | 1.3E2 | 5.2E1 | 3.0E2 | 6.9E1 | 1.4E-1 | 1.4E-1 | 1.9E3 | 2.7E2 | 149 | 26 | 149 | 26 | 0.43 |
| bO | ng/mL | 4.0E-2 | 4.0E-2 | 9.8E0 | 5.3E0 | 1.9E1 | 2.3E1 | 4.0E-2 | 4.0E-2 | 1.3E2 | 1.2E2 | 149 | 26 | 149 | 26 | 0.37 |
| bP | mg/mL | 5.2E-1 | 5.7E-1 | 7.4E-1 | 8.1E-1 | 7.1E-1 | 7.0E-1 | 4.9E-2 | 9.2E-2 | 4.8E0 | 2.7E0 | 149 | 26 | 149 | 26 | 0.54 |
| bQ | pg/mL | 2.1E1 | 3.4E1 | 1.5E2 | 7.0E1 | 1.1E3 | 7.4E1 | 1.5E-1 | 8.1E0 | 1.3E4 | 3.2E2 | 149 | 26 | 149 | 26 | 0.65 |
| bR | ng/mL | 1.2E-2 | 1.2E-2 | 1.8E-1 | 7.0E-2 | 7.6E-1 | 1.1E-1 | 1.2E-2 | 1.2E-2 | 8.7E0 | 3.8E-1 | 149 | 26 | 149 | 26 | 0.44 |
| bS | pg/mL | 9.4E-1 | 9.4E-1 | 9.8E0 | 7.9E0 | 4.4E1 | 1.9E1 | 9.4E-1 | 9.4E-1 | 3.9E2 | 7.2E1 | 149 | 26 | 149 | 26 | 0.51 |
| bU | ng/mL | 8.7E-2 | 8.3E-2 | 2.0E-1 | 1.1E-1 | 5.7E-1 | 1.2E-1 | 1.3E-2 | 1.3E-2 | 6.6E0 | 4.1E-1 | 149 | 26 | 149 | 26 | 0.46 |
| bV | pg/mL | 4.6E2 | 6.5E2 | 6.1E2 | 7.7E2 | 9.4E2 | 4.5E2 | 1.6E2 | 3.4E2 | 1.2E4 | 2.2E3 | 149 | 26 | 149 | 26 | 0.70 |
| bW | pg/mL | 3.2E2 | 3.3E2 | 4.8E2 | 6.7E2 | 5.2E2 | 9.9E2 | 8.4E1 | 1.1E2 | 4.8E3 | 3.9E3 | 149 | 26 | 149 | 26 | 0.51 |
| bX | ng/mL | 2.5E-5 | 2.5E-5 | 2.6E-3 | 2.4E-3 | 3.2E-3 | 2.8E-3 | 2.5E-5 | 2.5E-5 | 1.3E-2 | 7.2E-3 | 149 | 26 | 149 | 26 | 0.49 |
| bZ | pg/mL | 2.7E2 | 7.2E2 | 1.8E3 | 3.4E3 | 6.8E3 | 8.4E3 | 1.5E-1 | 1.5E-1 | 5.8E4 | 4.3E4 | 149 | 26 | 149 | 26 | 0.64 |
| cA | pg/mL | 6.0E-1 | 6.0E-1 | 4.5E0 | 1.7E0 | 3.1E1 | 4.2E0 | 6.0E-1 | 6.0E-1 | 3.7E2 | 2.1E1 | 149 | 26 | 149 | 26 | 0.47 |
| cB | ng/mL | 5.3E-2 | 2.9E-2 | 8.0E-2 | 4.3E-2 | 9.1E-2 | 6.1E-2 | 1.7E-3 | 1.7E-3 | 4.3E-1 | 2.6E-1 | 149 | 26 | 149 | 26 | 0.35 |
| cC | pg/mL | 4.4E1 | 3.9E1 | 4.7E1 | 3.6E1 | 5.3E1 | 2.5E1 | 1.0E0 | 1.0E0 | 4.5E2 | 7.7E1 | 149 | 26 | 149 | 26 | 0.45 |
| cD | pg/mL | 4.9E0 | 4.5E0 | 1.3E1 | 8.0E0 | 4.9E1 | 1.4E1 | 3.3E-1 | 3.3E-1 | 4.8E2 | 6.9E1 | 149 | 26 | 149 | 26 | 0.48 |
| cE | pg/mL | 5.5E1 | 8.3E1 | 2.6E2 | 2.8E2 | 6.1E2 | 4.6E2 | 1.2E-1 | 6.1E0 | 3.8E3 | 1.7E3 | 149 | 26 | 149 | 26 | 0.60 |
| cF | pg/mL | 9.4E0 | 5.3E-1 | 1.8E1 | 5.1E0 | 3.1E1 | 9.7E0 | 5.3E-1 | 5.3E-1 | 2.7E2 | 3.8E1 | 149 | 26 | 149 | 26 | 0.33 |
| cG | pg/mL | 5.3E1 | 1.3E2 | 1.7E2 | 1.9E2 | 8.6E2 | 2.2E2 | 7.8E0 | 2.4E1 | 1.0E4 | 1.1E3 | 149 | 26 | 149 | 26 | 0.70 |
| cH | uIU/mL | 3.2E0 | 3.9E0 | 6.6E0 | 1.3E1 | 1.5E1 | 2.6E1 | 8.6E-3 | 8.6E-3 | 1.6E2 | 1.2E2 | 149 | 26 | 149 | 26 | 0.53 |
| cI | ng/mL | 6.0E0 | 8.2E0 | 1.5E1 | 1.0E1 | 2.3E1 | 1.1E1 | 3.2E-2 | 2.3E-1 | 1.2E2 | 4.1E1 | 149 | 26 | 149 | 26 | 0.49 |
| cJ | ug/mL | 6.7E1 | 4.7E1 | 1.0E2 | 8.1E1 | 1.0E2 | 8.5E1 | 6.9E0 | 5.6E0 | 6.4E2 | 3.4E2 | 149 | 26 | 149 | 26 | 0.45 |
| cK | ng/mL | 3.8E-3 | 3.8E-3 | 2.4E-2 | 1.6E-2 | 1.2E-1 | 4.2E-2 | 3.8E-3 | 3.8E-3 | 1.5E0 | 2.1E-1 | 149 | 26 | 149 | 26 | 0.50 |
| cL | pg/mL | 2.1E2 | 2.3E2 | 5.0E2 | 7.7E2 | 2.1E3 | 1.5E3 | 3.1E1 | 6.7E1 | 2.4E4 | 7.4E3 | 149 | 26 | 149 | 26 | 0.60 |
| cM | pg/mL | 2.7E2 | 2.8E2 | 2.9E2 | 2.7E2 | 1.7E2 | 8.6E1 | 2.5E1 | 1.2E2 | 1.1E3 | 4.4E2 | 149 | 26 | 149 | 26 | 0.50 |
| cN | pg/mL | 1.2E2 | 1.4E2 | 1.3E2 | 1.4E2 | 9.0E1 | 3.6E1 | 3.8E1 | 8.6E1 | 1.1E3 | 2.2E2 | 149 | 26 | 149 | 26 | 0.65 |
| cO | pg/mL | 2.1E2 | 2.5E2 | 4.2E2 | 3.5E2 | 1.6E3 | 3.1E2 | 5.4E1 | 8.2E1 | 1.9E4 | 1.5E3 | 149 | 26 | 149 | 26 | 0.58 |
| cP | ng/mL | 2.4E3 | 2.8E3 | 2.5E3 | 2.7E3 | 9.5E2 | 9.6E2 | 6.2E2 | 1.4E3 | 5.6E3 | 4.7E3 | 149 | 26 | 149 | 26 | 0.57 |
| cQ | ng/mL | 5.3E-2 | 5.9E-2 | 1.3E-1 | 1.3E-1 | 2.2E-1 | 1.4E-1 | 2.0E-3 | 2.0E-3 | 1.3E0 | 4.8E-1 | 149 | 26 | 149 | 26 | 0.57 |
| cR | ng/mL | 3.4E2 | 3.8E2 | 6.1E2 | 5.6E2 | 8.7E2 | 5.5E2 | 2.0E1 | 8.9E1 | 7.7E3 | 2.2E3 | 149 | 26 | 149 | 26 | 0.51 |
| cS | ng/mL | 2.7E2 | 3.6E2 | 4.0E2 | 9.1E2 | 4.3E2 | 1.5E3 | 4.1E1 | 1.2E2 | 2.5E3 | 7.1E3 | 149 | 26 | 149 | 26 | 0.67 |
| cT | ng/mL | 4.9E1 | 1.2E2 | 1.3E2 | 3.2E2 | 2.7E2 | 4.4E2 | 3.6E0 | 1.4E1 | 2.1E3 | 1.5E3 | 149 | 26 | 149 | 26 | 0.65 |
| cU | ng/mL | 5.6E1 | 1.4E2 | 9.1E1 | 1.5E2 | 1.5E2 | 9.2E1 | 6.2E0 | 1.7E1 | 1.6E3 | 3.9E2 | 149 | 26 | 149 | 26 | 0.74 |
| cV | ng/mL | 2.1E-1 | 1.9E-1 | 7.3E-1 | 7.1E-1 | 3.9E0 | 1.9E0 | 2.5E-2 | 3.4E-2 | 4.7E1 | 9.7E0 | 149 | 26 | 149 | 26 | 0.49 |
| cW | mIU/mL | 4.8E-2 | 6.2E-2 | 9.1E-2 | 7.7E-2 | 3.7E-1 | 6.0E-2 | 4.8E-3 | 1.6E-2 | 4.5E0 | 2.9E-1 | 149 | 26 | 149 | 26 | 0.59 |
| cX | ng/mL | 1.1E-1 | 1.8E-1 | 1.7E0 | 2.6E0 | 5.2E0 | 7.5E0 | 2.3E-4 | 9.1E-3 | 2.8E1 | 2.8E1 | 149 | 26 | 149 | 26 | 0.54 |
| cY | ng/mL | 7.5E0 | 6.5E0 | 1.1E1 | 8.5E0 | 1.1E1 | 7.8E0 | 1.7E-1 | 6.0E-1 | 6.1E1 | 3.6E1 | 149 | 26 | 149 | 26 | 0.45 |
| cZ | ug/mL | 1.3E1 | 1.2E1 | 1.5E1 | 1.4E1 | 6.9E0 | 6.4E0 | 2.3E0 | 6.2E0 | 4.6E1 | 3.0E1 | 149 | 26 | 149 | 26 | 0.47 |
| dA | pg/mL | 3.2E2 | 3.1E2 | 3.9E2 | 3.8E2 | 4.8E2 | 1.8E2 | 1.0E2 | 1.7E2 | 5.8E3 | 8.8E2 | 149 | 26 | 149 | 26 | 0.53 |
| dB | ug/mL | 1.8E1 | 2.2E1 | 1.8E1 | 2.0E1 | 2.1E1 | 8.0E0 | 2.1E0 | 2.9E0 | 2.5E2 | 3.7E1 | 149 | 26 | 149 | 26 | 0.63 |
| dC | nmol/L | 3.5E1 | 3.0E1 | 3.7E1 | 3.5E1 | 1.7E1 | 1.6E1 | 7.8E0 | 1.5E1 | 1.4E2 | 9.1E1 | 149 | 26 | 149 | 26 | 0.43 |
| dD | ug/mL | 3.4E1 | 2.9E1 | 3.6E1 | 3.2E1 | 1.1E1 | 8.2E0 | 1.4E1 | 2.0E1 | 7.4E1 | 5.2E1 | 149 | 26 | 149 | 26 | 0.38 |
| dE | ng/mL | 4.6E-1 | 4.7E-1 | 5.5E-1 | 7.5E-1 | 5.5E-1 | 8.3E-1 | 8.4E-3 | 8.4E-3 | 2.7E0 | 2.9E0 | 149 | 26 | 149 | 26 | 0.54 |
| dF | ng/mL | 2.4E2 | 3.8E2 | 3.2E2 | 5.0E2 | 2.4E2 | 3.0E2 | 7.5E1 | 1.3E2 | 1.3E3 | 1.2E3 | 149 | 26 | 149 | 26 | 0.70 |
| dG | ng/mL | 1.1E1 | 1.5E1 | 1.7E1 | 2.0E1 | 2.0E1 | 1.3E1 | 3.0E0 | 6.7E0 | 1.8E2 | 6.5E1 | 149 | 26 | 149 | 26 | 0.66 |
| dH | pg/mL | 8.0E0 | 9.6E0 | 2.1E1 | 1.6E1 | 6.6E1 | 2.2E1 | 4.0E-2 | 8.3E-1 | 6.7E2 | 9.7E1 | 149 | 26 | 149 | 26 | 0.56 |
| dI | pg/mL | 4.6E-1 | 4.6E-1 | 4.0E0 | 2.4E0 | 2.7E1 | 4.3E0 | 4.6E-1 | 4.6E-1 | 3.3E2 | 1.9E1 | 149 | 26 | 149 | 26 | 0.52 |
| dJ | ng/mL | 2.1E0 | 1.8E0 | 2.2E0 | 2.0E0 | 1.1E0 | 1.0E0 | 3.2E-2 | 3.7E-1 | 5.6E0 | 4.5E0 | 149 | 26 | 149 | 26 | 0.45 |
| dK | uIU/mL | 1.4E0 | 1.0E0 | 2.2E0 | 1.6E0 | 3.8E0 | 1.8E0 | 2.8E-4 | 1.4E-2 | 3.9E1 | 7.3E0 | 149 | 26 | 149 | 26 | 0.43 |
| dL | ng/mL | 8.7E2 | 1.2E3 | 1.0E3 | 1.2E3 | 6.5E2 | 4.3E2 | 2.8E2 | 5.8E2 | 4.8E3 | 2.3E3 | 149 | 26 | 149 | 26 | 0.64 |
| dM | pg/mL | 9.5E2 | 1.3E3 | 1.3E3 | 1.8E3 | 1.5E3 | 1.3E3 | 3.7E2 | 5.2E2 | 1.5E4 | 5.8E3 | 149 | 26 | 149 | 26 | 0.65 |
| dN | ug/mL | 9.8E1 | 1.1E2 | 1.0E2 | 1.2E2 | 4.5E1 | 3.9E1 | 2.4E1 | 6.4E1 | 3.3E2 | 2.2E2 | 149 | 26 | 149 | 26 | 0.61 |
| dR | pg/ml | 1.5E3 | 9.3E2 | 2.2E3 | 1.4E3 | 2.1E3 | 1.3E3 | 1.4E2 | 1.3E2 | 9.8E3 | 5.5E3 | 111 | 21 | 111 | 21 | 0.38 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dU | pg/ml | 9.7E3 | 1.1E4 | 1.6E4 | 1.3E4 | 1.8E4 | 1.1E4 | 6.9E2 | 1.7E3 | 8.1E4 | 3.5E4 | 24 | 9 | 24 | 9 | 0.48 |
| dX | ng/ml | 6.6E-2 | 8.2E-2 | 1.4E-1 | 1.2E-1 | 2.0E-1 | 1.3E-1 | 2.6E-3 | 2.6E-3 | 1.2E0 | 4.2E-1 | 50 | 11 | 50 | 11 | 0.55 |
| eF | ng/ml | 4.1E0 | 5.5E0 | 5.1E0 | 6.7E0 | 4.6E0 | 5.5E0 | 2.0E0 | 2.0E0 | 4.6E1 | 2.9E1 | 111 | 21 | 111 | 21 | 0.65 |
| eC | pg/ml | 3.0E2 | 2.7E2 | 3.7E2 | 3.1E2 | 3.2E2 | 1.8E2 | 9.9E0 | 1.1E2 | 2.0E3 | 7.3E2 | 98 | 18 | 98 | 18 | 0.45 |
| eD | pg/ml | 2.2E2 | 2.6E2 | 6.0E2 | 1.3E3 | 1.4E3 | 2.1E3 | 5.2E-1 | 5.9E1 | 8.3E3 | 7.0E3 | 81 | 15 | 81 | 15 | 0.58 |
| eM | ng/ml | 2.7E0 | 3.2E0 | 4.8E0 | 5.7E0 | 5.6E0 | 9.4E0 | 6.9E-1 | 7.6E-1 | 2.7E1 | 3.9E1 | 64 | 15 | 64 | 15 | 0.54 |
| eP | ng/ml | 3.7E-3 | 3.7E-3 | 1.2E0 | 1.4E0 | 4.1E0 | 3.0E0 | 3.7E-3 | 3.7E-3 | 2.8E1 | 1.0E1 | 50 | 11 | 50 | 11 | 0.50 |
| eQ | pg/ml | 1.0E0 | 1.0E0 | 5.7E1 | 4.2E1 | 1.2E2 | 8.4E1 | 1.0E0 | 1.0E0 | 4.7E2 | 2.2E2 | 24 | 9 | 24 | 9 | 0.45 |
| fA | ng/ml | 1.8E2 | 3.0E2 | 4.0E2 | 5.3E2 | 4.8E2 | 4.7E2 | 3.9E1 | 7.5E1 | 1.5E3 | 1.3E3 | 23 | 8 | 23 | 8 | 0.65 |
| fB | ng/ml | 5.9E2 | 6.0E2 | 6.7E2 | 6.3E2 | 2.9E2 | 2.1E2 | 2.6E2 | 3.5E2 | 1.3E3 | 9.2E2 | 22 | 9 | 22 | 9 | 0.46 |
| fP | ng/ml | 2.6E2 | 2.8E2 | 3.2E2 | 3.3E2 | 1.8E2 | 2.0E2 | 3.7E1 | 1.8E0 | 1.0E3 | 9.5E2 | 106 | 20 | 106 | 20 | 0.53 |
| fR | ng/ml | 1.3E5 | 2.2E5 | 2.1E5 | 2.9E5 | 1.7E5 | 2.1E5 | 3.6E4 | 1.9E2 | 6.9E5 | 8.7E5 | 95 | 20 | 95 | 20 | 0.63 |
| gC | ng/ml | 2.4E2 | 2.7E2 | 2.6E2 | 2.8E2 | 1.1E2 | 1.6E2 | 8.3E1 | 9.7E1 | 6.4E2 | 5.9E2 | 37 | 10 | 37 | 10 | 0.52 |
| gL | pg/ml | 6.5E4 | 7.7E4 | 7.1E4 | 9.5E4 | 3.3E4 | 5.1E4 | 1.1E4 | 2.7E4 | 1.9E5 | 2.2E5 | 111 | 21 | 111 | 21 | 0.62 |
| gP | U/ml | 2.7E2 | 2.8E2 | 2.8E2 | 3.3E2 | 1.0E2 | 2.0E2 | 1.2E1 | 6.5E1 | 8.0E2 | 1.1E3 | 110 | 21 | 110 | 21 | 0.58 |
| gW | ng/ml | 5.4E2 | 5.2E2 | 9.6E2 | 6.3E2 | 1.1E3 | 4.6E2 | 2.3E0 | 2.0E2 | 6.1E3 | 1.8E3 | 87 | 11 | 87 | 11 | 0.49 |
| gV | ng/ml | 1.9E1 | 2.3E1 | 2.0E1 | 2.6E1 | 7.5E0 | 5.1E0 | 8.1E-2 | 2.1E1 | 3.7E1 | 3.4E1 | 30 | 8 | 30 | 8 | 0.73 |
| tF | pg/mL | 9.0E2 | 4.8E3 | 9.7E3 | 2.4E4 | 3.4E4 | 5.8E4 | 1.2E1 | 1.8E1 | 2.8E5 | 2.5E5 | 99 | 19 | 99 | 19 | 0.66 |
| gZ | ug/ml | 6.8E-1 | 1.2E0 | 4.3E1 | 3.4E1 | 1.2E2 | 7.8E1 | 8.7E-2 | 1.4E-1 | 4.1E2 | 2.4E2 | 24 | 9 | 24 | 9 | 0.63 |
| hA | ng/ml | 2.3E0 | 5.2E0 | 1.4E1 | 1.5E1 | 5.3E1 | 3.0E1 | 1.7E-2 | 7.4E-1 | 3.5E2 | 1.1E2 | 81 | 16 | 81 | 16 | 0.68 |
| nM | pg/ml | 1.0E-9 | 1.0E-9 | 1.0E-9 | 8.0E1 | 0.0E0 | 3.5E2 | 1.0E-9 | 1.0E-9 | 1.0E-9 | 1.5E3 | 57 | 19 | 57 | 19 | 0.53 |
| nN | pg/ml | 1.3E3 | 2.4E3 | 2.5E3 | 1.8E4 | 3.3E3 | 4.1E4 | 8.1E1 | 2.3E2 | 2.1E4 | 1.5E5 | 57 | 19 | 57 | 19 | 0.63 |
| nO | pg/ml | 2.4E1 | 4.1E1 | 3.6E1 | 4.5E1 | 4.8E1 | 3.9E1 | 4.0E0 | 9.7E0 | 3.1E2 | 1.5E2 | 57 | 19 | 57 | 19 | 0.65 |
| nR | pg/ml | 1.5E1 | 4.8E1 | 3.8E1 | 2.9E2 | 5.9E1 | 5.0E2 | 1.0E0 | 3.5E0 | 2.6E2 | 1.9E3 | 57 | 19 | 57 | 19 | 0.75 |
| nT | pg/ml | 7.3E1 | 8.8E1 | 9.3E1 | 1.9E2 | 7.6E1 | 2.4E2 | 1.0E-9 | 1.0E-9 | 3.3E2 | 9.2E2 | 57 | 19 | 57 | 19 | 0.59 |
| nU | pg/ml | 3.1E1 | 1.2E2 | 4.8E1 | 2.8E2 | 5.5E1 | 3.9E2 | 1.0E-9 | 1.0E-9 | 2.0E2 | 1.5E3 | 57 | 19 | 57 | 19 | 0.70 |
| lW | pg/ml | 1.0E-9 | 1.0E-9 | 1.0E1 | 1.2E1 | 3.2E1 | 2.1E1 | 1.0E-9 | 1.0E-9 | 1.7E2 | 7.0E1 | 57 | 19 | 57 | 19 | 0.57 |
| lX | pg/ml | 9.0E2 | 9.1E2 | 1.0E3 | 9.9E2 | 5.9E2 | 5.0E2 | 1.9E2 | 4.8E2 | 2.6E3 | 2.5E3 | 57 | 19 | 57 | 19 | 0.52 |
| lY | pg/ml | 2.0E1 | 1.2E1 | 2.1E1 | 1.7E1 | 1.8E1 | 1.3E1 | 1.0E-9 | 1.0E-9 | 1.2E2 | 4.5E1 | 57 | 19 | 57 | 19 | 0.41 |
| mE | pg/ml | 1.0E-9 | 1.0E-9 | 3.0E0 | 1.2E0 | 8.4E0 | 2.6E0 | 1.0E-9 | 1.0E-9 | 5.7E1 | 9.2E0 | 57 | 19 | 57 | 19 | 0.43 |
| mF | pg/ml | 2.7E-1 | 7.1E-1 | 3.5E0 | 1.6E1 | 1.4E1 | 5.6E1 | 1.0E-9 | 1.0E-9 | 1.1E2 | 2.5E2 | 57 | 19 | 57 | 19 | 0.58 |
| mH | pg/ml | 3.3E0 | 2.6E0 | 5.3E0 | 5.5E0 | 7.9E0 | 7.8E0 | 4.0E-1 | 5.4E-1 | 5.3E1 | 3.2E1 | 57 | 19 | 57 | 19 | 0.45 |
| mI | pg/ml | 1.0E-9 | 2.6E0 | 1.2E1 | 3.8E1 | 2.7E1 | 1.0E2 | 1.0E-9 | 1.0E-9 | 1.8E2 | 4.6E2 | 57 | 19 | 57 | 19 | 0.55 |
| mM | pg/ml | 2.8E1 | 5.1E1 | 6.4E1 | 1.3E2 | 8.9E1 | 2.6E2 | 1.0E-9 | 1.0E-9 | 4.0E2 | 1.1E3 | 57 | 19 | 57 | 19 | 0.57 |
| mP | pg/ml | 1.5E1 | 1.6E1 | 1.5E1 | 7.2E1 | 9.9E0 | 1.8E2 | 1.0E-9 | 8.2E0 | 5.8E1 | 8.1E2 | 56 | 19 | 56 | 19 | 0.63 |
| mS | pg/ml | 1.6E3 | 1.6E3 | 1.8E3 | 1.8E3 | 9.9E2 | 1.1E3 | 1.0E-9 | 1.0E-9 | 5.1E3 | 4.2E3 | 57 | 19 | 57 | 19 | 0.49 |
| mT | pg/ml | 5.7E1 | 5.6E1 | 1.2E2 | 2.4E2 | 2.1E2 | 4.5E2 | 1.0E1 | 1.6E1 | 1.4E3 | 1.7E3 | 56 | 19 | 56 | 19 | 0.52 |
| mU | pg/ml | 2.0E0 | 3.5E0 | 2.7E0 | 1.7E1 | 2.0E0 | 5.0E1 | 1.0E-9 | 6.1E-1 | 1.0E1 | 2.2E2 | 56 | 19 | 56 | 19 | 0.68 |
| mW | pg/ml | 2.1E3 | 1.9E3 | 2.3E3 | 3.4E3 | 1.1E3 | 3.2E3 | 1.0E-9 | 2.0E2 | 6.2E3 | 1.1E4 | 56 | 19 | 56 | 19 | 0.54 |
| mY | pg/ml | 6.5E2 | 6.7E2 | 8.7E2 | 1.2E3 | 9.9E2 | 1.8E3 | 1.0E-9 | 6.1E1 | 5.6E3 | 8.0E3 | 57 | 19 | 57 | 19 | 0.56 |
| mZ | pg/ml | 1.5E2 | 3.0E2 | 3.3E2 | 4.9E2 | 4.9E2 | 4.2E2 | 1.0E-9 | 3.9E1 | 3.1E3 | 1.4E3 | 56 | 19 | 56 | 19 | 0.67 |
| nA | pg/ml | 1.5E0 | 2.7E0 | 5.9E0 | 1.0E1 | 1.2E1 | 1.9E1 | 1.0E-9 | 1.0E-9 | 6.5E1 | 5.7E1 | 56 | 19 | 56 | 19 | 0.52 |
| nB | pg/ml | 2.8E2 | 3.0E2 | 3.0E2 | 3.7E2 | 1.6E2 | 2.1E2 | 3.0E1 | 1.3E2 | 6.9E2 | 9.6E2 | 57 | 19 | 57 | 19 | 0.58 |
| nC | pg/ml | 1.0E-9 | 8.3E1 | 1.1E4 | 1.8E3 | 5.8E4 | 4.7E3 | 1.0E-9 | 1.0E-9 | 3.8E5 | 2.0E4 | 57 | 19 | 57 | 19 | 0.60 |
| nD | pg/ml | 6.6E0 | 9.2E0 | 1.2E1 | 1.8E1 | 3.4E1 | 2.7E1 | 1.0E-9 | 1.0E-9 | 2.6E2 | 1.2E2 | 56 | 19 | 56 | 19 | 0.59 |
| nF | pg/ml | 1.0E-9 | 1.0E-9 | 3.0E0 | 3.2E0 | 1.3E1 | 1.1E1 | 1.0E-9 | 1.0E-9 | 9.3E1 | 4.7E1 | 57 | 19 | 57 | 19 | 0.50 |
| nH | pg/ml | 5.6E-1 | 4.2E0 | 2.3E2 | 2.7E1 | 1.4E3 | 7.5E1 | 1.0E-9 | 1.0E-9 | 1.0E4 | 3.3E2 | 56 | 19 | 56 | 19 | 0.60 |
| nI | pg/ml | 3.0E1 | 1.0E-9 | 6.1E1 | 8.7E1 | 8.0E1 | 2.7E2 | 1.0E-9 | 1.0E-9 | 3.5E2 | 1.2E3 | 57 | 19 | 57 | 19 | 0.40 |
| nJ | pg/ml | 1.7E-1 | 6.1E-1 | 3.1E0 | 1.9E0 | 1.7E1 | 3.7E0 | 1.0E-9 | 1.0E-9 | 1.3E2 | 1.5E1 | 57 | 19 | 57 | 19 | 0.53 |
| nK | pg/ml | 1.0E-9 | 1.0E-9 | 1.4E1 | 2.1E1 | 2.4E1 | 5.2E1 | 1.0E-9 | 1.0E-9 | 1.0E2 | 2.2E2 | 56 | 19 | 56 | 19 | 0.48 |
| nL | pg/ml | 1.0E-9 | 2.5E0 | 3.4E2 | 7.5E1 | 1.9E3 | 2.1E2 | 1.0E-9 | 1.0E-9 | 1.4E4 | 9.0E2 | 57 | 19 | 57 | 19 | 0.56 |
| wJ | pg/ml | 1.3E5 | 2.5E5 | 1.5E5 | 2.9E5 | 9.4E4 | 1.7E5 | 1.1E4 | 1.1E5 | 4.3E5 | 5.8E5 | 49 | 7 | 49 | 7 | 0.78 |
| wK | pg/ml | 3.2E4 | 6.6E4 | 3.9E4 | 1.2E5 | 2.8E4 | 1.7E5 | 3.7E3 | 2.5E4 | 1.3E5 | 5.0E5 | 49 | 7 | 49 | 7 | 0.79 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| wL | pg/ml | 3.9E0 | 2.9E1 | 3.8E1 | 1.4E2 | 1.3E2 | 1.8E2 | 1.0E-9 | 6.7E0 | 8.4E2 | 4.7E2 | 49 | 7 | 49 | 7 | 0.83 |
| wP | pg/ml | 2.4E4 | 4.3E4 | 4.8E4 | 5.7E4 | 5.8E4 | 5.8E4 | 1.1E3 | 4.5E3 | 3.0E5 | 1.7E5 | 49 | 7 | 49 | 7 | 0.57 |
| wQ | pg/ml | 3.1E1 | 7.6E1 | 5.5E1 | 9.7E1 | 9.2E1 | 9.4E1 | 1.0E-9 | 1.1E1 | 5.1E2 | 2.4E2 | 49 | 7 | 49 | 7 | 0.70 |
| hR | pg/ml | 2.9E4 | 2.0E4 | 3.0E4 | 2.0E4 | 1.2E4 | 4.0E3 | 1.0E-9 | 1.4E4 | 5.8E4 | 2.7E4 | 76 | 14 | 76 | 14 | 0.20 |
| hV | pg/ml | 4.7E2 | 3.5E2 | 4.6E2 | 3.9E2 | 2.3E2 | 2.1E2 | 1.0E-9 | 1.3E2 | 1.2E3 | 8.8E2 | 76 | 14 | 76 | 14 | 0.40 |
| hW | pg/ml | 1.7E3 | 1.9E3 | 2.6E3 | 2.3E3 | 4.5E3 | 1.3E3 | 1.0E-9 | 7.1E2 | 4.0E4 | 4.7E3 | 76 | 14 | 76 | 14 | 0.53 |
| hX | pg/ml | 1.1E3 | 9.6E2 | 1.1E3 | 1.1E3 | 9.5E2 | 6.4E2 | 2.5E0 | 5.2E2 | 8.6E3 | 2.9E3 | 76 | 14 | 76 | 14 | 0.47 |
| iA | pg/ml | 1.6E2 | 1.8E2 | 2.6E2 | 2.5E2 | 2.9E2 | 2.0E2 | 1.5E1 | 2.3E1 | 1.8E3 | 6.8E2 | 98 | 20 | 98 | 20 | 0.51 |
| iB | ng/ml | 4.8E0 | 6.8E0 | 6.2E0 | 8.6E0 | 5.1E0 | 4.8E0 | 3.3E-2 | 2.5E0 | 2.4E1 | 1.9E1 | 81 | 16 | 81 | 16 | 0.69 |
| iC | U/ml | 2.8E-1 | 5.3E-1 | 1.3E0 | 5.3E-1 | 6.2E0 | 2.5E-1 | 1.0E-9 | 1.4E-1 | 5.5E1 | 1.1E0 | 81 | 16 | 81 | 16 | 0.67 |
| tQ | pg/ml | 1.4E3 | 1.3E3 | 1.5E3 | 1.5E3 | 6.0E2 | 4.9E2 | 3.7E2 | 9.5E2 | 3.3E3 | 2.2E3 | 45 | 7 | 45 | 7 | 0.50 |
| tT | pg/ml | 2.0E1 | 1.8E1 | 2.2E1 | 2.0E1 | 1.5E1 | 9.3E0 | 5.4E0 | 1.0E1 | 9.3E1 | 3.4E1 | 46 | 7 | 46 | 7 | 0.51 |
| tS | pg/ml | 7.7E-1 | 1.3E0 | 1.5E0 | 1.0E0 | 2.1E0 | 7.6E-1 | 1.0E-9 | 1.0E-9 | 1.0E1 | 2.0E0 | 47 | 7 | 47 | 7 | 0.52 |
| tX | pg/ml | 1.1E0 | 1.3E0 | 1.8E0 | 1.4E0 | 2.1E0 | 1.1E0 | 2.5E-2 | 4.0E-1 | 1.0E1 | 3.3E0 | 46 | 7 | 46 | 7 | 0.50 |
| tO | pg/ml | 3.9E0 | 5.9E0 | 5.2E0 | 5.6E0 | 3.9E0 | 2.0E0 | 1.0E-9 | 2.9E0 | 1.8E1 | 8.9E0 | 47 | 7 | 47 | 7 | 0.62 |
| tR | pg/ml | 1.9E-1 | 3.4E-1 | 3.5E-1 | 3.3E-1 | 4.9E-1 | 2.7E-1 | 1.0E-9 | 9.8E-3 | 2.5E0 | 8.7E-1 | 46 | 7 | 46 | 7 | 0.58 |
| tU | pg/ml | 1.1E1 | 5.5E0 | 1.3E1 | 1.3E1 | 1.3E1 | 1.9E1 | 2.2E-1 | 1.5E0 | 8.0E1 | 5.5E1 | 48 | 7 | 48 | 7 | 0.36 |
| tN | pg/ml | 2.0E1 | 2.5E1 | 3.2E1 | 2.6E1 | 3.4E1 | 1.9E1 | 1.0E-9 | 1.0E-9 | 1.6E2 | 5.3E1 | 45 | 7 | 45 | 7 | 0.52 |
| tV | ng/ml | 5.3E2 | 1.1E3 | 7.7E2 | 8.8E2 | 6.5E2 | 4.5E2 | 5.3E1 | 2.1E2 | 3.1E3 | 1.2E3 | 48 | 7 | 48 | 7 | 0.57 |
| iII | ng/ml | 1.6E5 | 1.8E5 | 1.6E5 | 1.7E5 | 5.1E4 | 4.3E4 | 2.9E3 | 8.1E4 | 2.6E5 | 2.5E5 | 98 | 20 | 98 | 20 | 0.58 |
| iJ | ng/ml | 4.8E4 | 5.1E4 | 5.3E4 | 6.5E4 | 3.2E4 | 5.0E4 | 1.8E3 | 1.5E4 | 2.5E5 | 2.5E5 | 98 | 20 | 98 | 20 | 0.58 |
| hB | ng/ml | 4.8E-1 | 6.4E-1 | 6.2E-1 | 7.2E-1 | 5.2E-1 | 4.3E-1 | 1.2E-1 | 2.3E-1 | 3.2E0 | 1.9E0 | 98 | 20 | 98 | 20 | 0.59 |
| hC | pg/ml | 4.0E3 | 1.0E4 | 7.9E3 | 1.0E4 | 1.3E4 | 8.0E3 | 4.1E1 | 2.3E2 | 1.1E5 | 2.6E4 | 98 | 20 | 98 | 20 | 0.63 |
| hF | ng/ml | 1.0E-9 | 1.0E-9 | 4.1E1 | 1.0E-9 | 4.0E2 | 0.0E0 | 1.0E-9 | 1.0E-9 | 4.0E3 | 1.0E-9 | 98 | 20 | 98 | 20 | 0.49 |
| hG | pg/ml | 6.7E3 | 7.7E3 | 7.2E3 | 9.5E3 | 3.1E3 | 4.1E3 | 1.8E3 | 4.5E3 | 2.0E4 | 1.8E4 | 98 | 20 | 98 | 20 | 0.68 |
| iO | ng/ml | 3.7E5 | 4.6E5 | 4.1E5 | 4.5E5 | 2.0E5 | 1.7E5 | 1.1E4 | 1.0E5 | 1.1E6 | 7.7E5 | 98 | 20 | 98 | 20 | 0.60 |
| iP | ng/ml | 5.2E4 | 6.2E4 | 5.4E4 | 7.4E4 | 3.1E4 | 8.9E4 | 1.0E-9 | 2.5E4 | 2.2E5 | 4.4E5 | 98 | 20 | 98 | 20 | 0.55 |
| iZ | ng/ml | 1.6E3 | 2.1E3 | 1.8E3 | 2.3E3 | 8.0E2 | 1.2E3 | 6.6E2 | 8.4E2 | 5.1E3 | 5.7E3 | 95 | 21 | 95 | 21 | 0.63 |
| yD | ng/ml | 1.2E-2 | 1.8E-2 | 1.3E-2 | 1.7E-2 | 5.8E-3 | 4.0E-3 | 1.0E-9 | 1.0E-2 | 2.8E-2 | 2.2E-2 | 49 | 7 | 49 | 7 | 0.76 |
| jB | ng/ml | 2.3E5 | 2.3E5 | 2.4E5 | 2.4E5 | 7.4E4 | 8.0E4 | 9.9E4 | 1.3E5 | 3.6E5 | 3.5E5 | 24 | 9 | 24 | 9 | 0.50 |
| wB | pg/ml | 8.9E3 | 1.1E4 | 1.2E4 | 9.4E3 | 9.3E3 | 5.6E3 | 1.7E3 | 1.9E3 | 4.2E4 | 1.6E4 | 49 | 7 | 49 | 7 | 0.45 |
| rC | pg/ml | 1.6E3 | 1.2E3 | 2.1E3 | 2.5E3 | 2.1E3 | 3.2E3 | 1.0E-9 | 6.0E2 | 1.5E4 | 1.1E4 | 74 | 13 | 74 | 13 | 0.47 |
| rB | pg/ml | 3.0E1 | 6.9E1 | 4.8E1 | 8.7E1 | 6.4E1 | 8.4E1 | 1.0E-9 | 4.0E0 | 3.9E2 | 3.2E2 | 74 | 13 | 74 | 13 | 0.67 |
| jD | ng/ml | 2.7E1 | 5.5E1 | 3.9E1 | 9.8E1 | 4.1E1 | 1.3E2 | 1.0E-9 | 1.0E-9 | 1.9E2 | 5.1E2 | 81 | 16 | 81 | 16 | 0.68 |
| jE | ng/ml | 1.0E-9 | 1.0E-9 | 5.3E0 | 7.3E0 | 1.3E1 | 1.1E1 | 1.0E-9 | 1.0E-9 | 6.2E1 | 2.9E1 | 81 | 16 | 81 | 16 | 0.58 |
| jF | ng/ml | 4.2E1 | 1.4E1 | 4.9E1 | 4.0E1 | 5.3E1 | 5.2E1 | 1.0E-9 | 1.0E-9 | 2.1E2 | 1.5E2 | 81 | 16 | 81 | 16 | 0.45 |
| jG | ng/ml | 4.0E3 | 5.3E3 | 4.4E3 | 5.3E3 | 2.0E3 | 1.5E3 | 6.7E2 | 3.6E3 | 9.6E3 | 9.5E3 | 81 | 16 | 81 | 16 | 0.66 |
| jH | ng/ml | 7.6E1 | 1.2E2 | 8.0E1 | 1.2E2 | 5.1E1 | 7.6E1 | 1.3E1 | 1.5E1 | 4.3E2 | 2.4E2 | 81 | 16 | 81 | 16 | 0.66 |
| jI | ng/ml | 6.8E1 | 1.3E2 | 7.6E1 | 1.3E2 | 4.9E1 | 6.3E1 | 1.9E1 | 5.2E1 | 4.4E2 | 2.6E2 | 81 | 16 | 81 | 16 | 0.81 |
| wC | ng/ml | 1.6E0 | 1.3E0 | 2.0E0 | 1.2E0 | 1.4E0 | 4.0E-1 | 6.1E-2 | 5.7E-1 | 6.5E0 | 1.7E0 | 49 | 7 | 49 | 7 | 0.29 |
| wD | ng/ml | 2.2E1 | 2.6E1 | 8.8E1 | 3.1E1 | 3.0E2 | 1.3E1 | 2.8E0 | 1.6E1 | 2.1E3 | 5.4E1 | 49 | 7 | 49 | 7 | 0.58 |
| wE | ng/ml | 4.9E1 | 4.3E1 | 5.1E1 | 4.6E1 | 2.1E1 | 1.5E1 | 8.1E0 | 2.6E1 | 9.4E1 | 6.5E1 | 49 | 7 | 49 | 7 | 0.43 |
| wG | ng/ml | 9.9E-2 | 1.1E-1 | 1.4E-1 | 1.5E-1 | 1.7E-1 | 1.3E-1 | 1.0E-9 | 1.0E-9 | 7.1E-1 | 3.3E-1 | 49 | 7 | 49 | 7 | 0.56 |
| wH | ng/ml | 3.2E-2 | 1.2E-1 | 3.7E-1 | 2.4E-1 | 1.0E0 | 2.3E-1 | 1.0E-9 | 1.3E-2 | 5.6E0 | 5.7E-1 | 49 | 7 | 49 | 7 | 0.70 |
| wF | ng/ml | 1.5E-1 | 8.6E-1 | 2.5E0 | 9.6E-1 | 9.1E0 | 6.0E-1 | 1.0E-9 | 2.7E-1 | 5.7E1 | 2.1E0 | 49 | 7 | 49 | 7 | 0.80 |
| rA | pg/ml | 2.4E1 | 2.7E1 | 3.0E1 | 3.0E1 | 2.8E1 | 2.0E1 | 1.0E-9 | 9.2E0 | 2.0E2 | 7.1E1 | 79 | 15 | 79 | 15 | 0.54 |
| qZ | pg/ml | 5.4E1 | 1.3E1 | 4.7E2 | 9.3E2 | 1.9E3 | 3.0E3 | 2.8E-4 | 6.5E-4 | 1.0E4 | 1.0E4 | 59 | 11 | 59 | 11 | 0.30 |
| qY | pg/ml | 1.5E1 | 2.2E1 | 3.8E1 | 4.6E1 | 5.7E1 | 5.3E1 | 8.7E-1 | 5.6E0 | 3.3E2 | 1.8E2 | 79 | 15 | 79 | 15 | 0.60 |
| qX | pg/ml | 6.0E1 | 7.3E1 | 7.0E1 | 8.4E1 | 4.9E1 | 5.1E1 | 1.0E-9 | 2.3E1 | 2.3E2 | 2.1E2 | 79 | 15 | 79 | 15 | 0.59 |
| qW | pg/ml | 7.7E0 | 7.6E0 | 1.2E1 | 1.0E1 | 1.7E1 | 9.3E0 | 1.0E-9 | 1.0E-9 | 1.2E2 | 3.6E1 | 79 | 15 | 79 | 15 | 0.49 |
| qV | pg/ml | 1.7E3 | 2.2E3 | 2.4E3 | 3.1E3 | 1.8E3 | 2.7E3 | 1.7E2 | 1.0E2 | 9.6E3 | 1.1E4 | 79 | 15 | 79 | 15 | 0.58 |
| qU | pg/ml | 7.3E1 | 1.1E2 | 1.9E2 | 2.2E2 | 3.2E2 | 2.8E2 | 1.0E-9 | 1.0E-9 | 2.1E3 | 1.1E3 | 79 | 15 | 79 | 15 | 0.57 |
| qT | pg/ml | 3.9E1 | 4.6E1 | 6.2E1 | 8.7E1 | 6.7E1 | 8.9E1 | 1.0E-9 | 2.2E1 | 4.9E2 | 3.1E2 | 79 | 15 | 79 | 15 | 0.59 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| jK | ng/ml | 1.5E3 | 1.5E3 | 1.6E3 | 1.8E3 | 5.7E2 | 8.9E2 | 2.8E2 | 7.0E2 | 3.6E3 | 4.1E3 | 81 | 16 | 81 | 16 | 0.54 |
| jL | ng/ml | 2.0E2 | 2.6E2 | 2.6E2 | 3.2E2 | 2.1E2 | 1.8E2 | 5.6E1 | 1.3E2 | 9.6E2 | 7.0E2 | 81 | 16 | 81 | 16 | 0.63 |
| jM | ng/ml | 6.8E4 | 6.7E4 | 7.5E4 | 7.2E4 | 4.0E4 | 4.1E4 | 4.6E3 | 1.1E4 | 1.8E5 | 1.4E5 | 81 | 16 | 81 | 16 | 0.48 |
| jO | pg/ml | 2.1E5 | 3.2E5 | 2.7E5 | 3.3E5 | 1.7E5 | 1.4E5 | 6.0E4 | 1.3E5 | 1.1E6 | 6.5E5 | 81 | 16 | 81 | 16 | 0.66 |
| jP | pg/ml | 2.5E5 | 3.2E5 | 2.8E5 | 3.6E5 | 1.4E5 | 1.6E5 | 3.6E4 | 1.4E5 | 7.1E5 | 5.8E5 | 81 | 16 | 81 | 16 | 0.65 |
| jQ | pg/ml | 2.3E3 | 2.3E3 | 3.1E3 | 3.3E3 | 2.9E3 | 2.6E3 | 5.0E0 | 6.4E2 | 1.3E4 | 9.2E3 | 81 | 16 | 81 | 16 | 0.55 |
| jR | pg/ml | 5.6E3 | 6.0E3 | 1.0E4 | 1.0E4 | 1.3E4 | 1.1E4 | 1.0E-9 | 1.6E3 | 6.8E4 | 4.6E4 | 81 | 16 | 81 | 16 | 0.56 |
| jT | pg/ml | 1.7E5 | 1.9E5 | 1.7E5 | 1.9E5 | 7.1E4 | 7.0E4 | 7.1E4 | 1.0E5 | 5.5E5 | 3.5E5 | 81 | 16 | 81 | 16 | 0.59 |
| jU | mIU/ml | 5.5E0 | 5.1E0 | 1.2E1 | 1.0E1 | 1.9E1 | 1.4E1 | 8.1E-2 | 3.9E-1 | 1.1E2 | 5.3E1 | 81 | 16 | 81 | 16 | 0.45 |
| jV | mIU/ml | 2.0E0 | 1.2E0 | 4.2E0 | 3.1E0 | 5.9E0 | 3.5E0 | 2.7E-3 | 2.1E-2 | 3.2E1 | 1.0E1 | 81 | 16 | 81 | 16 | 0.45 |
| jY | ng/ml | 7.4E-4 | 2.6E-3 | 8.1E-3 | 4.9E-3 | 3.5E-2 | 7.3E-3 | 1.0E-9 | 1.0E-9 | 3.0E-1 | 2.6E-2 | 81 | 16 | 81 | 16 | 0.59 |
| kC | pg/ml | 9.6E1 | 1.1E2 | 1.7E2 | 2.2E2 | 3.7E2 | 2.6E2 | 2.1E1 | 3.6E1 | 2.7E3 | 1.1E3 | 57 | 19 | 57 | 19 | 0.57 |
| kE | pg/ml | 1.4E5 | 1.4E5 | 1.3E5 | 1.5E5 | 3.8E4 | 4.5E4 | 3.8E4 | 5.1E4 | 2.3E5 | 2.7E5 | 57 | 19 | 57 | 19 | 0.56 |
| kF | pg/mL | 6.0E1 | 7.0E1 | 6.5E1 | 7.9E1 | 2.3E1 | 2.8E1 | 2.7E1 | 5.2E1 | 1.5E2 | 1.4E2 | 57 | 19 | 57 | 19 | 0.67 |
| kG | pg/mL | 8.2E3 | 1.1E4 | 1.1E4 | 2.3E4 | 1.0E4 | 3.6E4 | 1.1E3 | 3.3E3 | 5.8E4 | 1.6E5 | 57 | 19 | 57 | 19 | 0.63 |
| kI | pg/ml | 1.9E2 | 2.1E2 | 2.1E2 | 2.4E2 | 9.9E1 | 1.5E2 | 4.4E1 | 1.0E-9 | 5.5E2 | 6.7E2 | 57 | 19 | 57 | 19 | 0.55 |
| kK | pg/ml | 1.2E2 | 1.5E2 | 1.5E2 | 2.8E2 | 1.5E2 | 4.2E2 | 6.4E0 | 3.4E1 | 9.1E2 | 1.9E3 | 57 | 19 | 57 | 19 | 0.62 |
| kN | pg/ml | 1.0E3 | 1.1E3 | 1.3E3 | 2.3E3 | 9.7E2 | 3.0E3 | 2.1E2 | 7.3E1 | 4.9E3 | 1.0E4 | 57 | 19 | 57 | 19 | 0.55 |
| kO | pg/ml | 7.1E3 | 7.6E3 | 1.0E4 | 7.9E3 | 1.9E4 | 3.6E3 | 3.8E3 | 3.7E3 | 1.5E5 | 1.9E4 | 57 | 19 | 57 | 19 | 0.51 |
| kP | pg/ml | 6.3E3 | 4.9E3 | 7.0E3 | 5.8E3 | 4.3E3 | 4.1E3 | 1.5E3 | 9.6E2 | 2.7E4 | 1.6E4 | 57 | 19 | 57 | 19 | 0.40 |
| kQ | pg/ml | 4.2E3 | 5.3E3 | 5.3E3 | 5.5E3 | 4.3E3 | 2.5E3 | 5.6E2 | 1.5E3 | 2.5E4 | 1.2E4 | 98 | 20 | 98 | 20 | 0.60 |
| kR | pg/ml | 2.2E1 | 2.6E1 | 3.8E1 | 3.0E1 | 1.0E2 | 2.1E1 | 1.0E-9 | 2.9E0 | 1.0E3 | 7.5E1 | 98 | 20 | 98 | 20 | 0.53 |
| kS | pg/ml | 8.7E2 | 9.2E2 | 1.0E3 | 9.6E2 | 5.9E2 | 5.9E2 | 2.5E2 | 8.2E1 | 3.2E3 | 2.5E3 | 98 | 20 | 98 | 20 | 0.49 |
| rZ | ng/ml | 1.0E-3 | 1.4E-2 | 5.7E-3 | 4.6E-2 | 1.3E-2 | 8.3E-2 | 1.0E-9 | 1.0E-9 | 9.4E-2 | 3.0E-1 | 75 | 13 | 75 | 13 | 0.82 |
| rY | ng/ml | 6.2E-2 | 4.7E-2 | 1.8E-1 | 3.0E0 | 7.3E-1 | 7.1E0 | 1.0E-9 | 1.2E-2 | 6.3E0 | 2.0E1 | 75 | 13 | 75 | 13 | 0.53 |
| rX | ng/ml | 1.0E-9 | 1.0E-9 | 7.1E-2 | 4.3E-1 | 4.4E-1 | 1.0E0 | 1.0E-9 | 1.0E-9 | 3.7E0 | 3.1E0 | 75 | 13 | 75 | 13 | 0.53 |
| lK | pg/ml | 5.8E1 | 7.4E1 | 1.4E2 | 1.5E2 | 1.7E2 | 1.9E2 | 1.0E-9 | 1.0E-9 | 7.4E2 | 5.1E2 | 80 | 16 | 80 | 16 | 0.47 |
| lL | pg/ml | 1.6E3 | 3.8E3 | 2.2E3 | 6.3E3 | 2.6E3 | 9.8E3 | 7.5E1 | 6.9E2 | 1.9E4 | 4.2E4 | 81 | 16 | 81 | 16 | 0.77 |
| lM | pg/ml | 1.2E3 | 1.2E3 | 3.7E3 | 1.0E4 | 6.1E3 | 1.9E4 | 2.1E2 | 9.5E0 | 4.2E4 | 6.7E4 | 81 | 16 | 81 | 16 | 0.48 |
| lN | pg/ml | 1.0E-9 | 3.4E0 | 2.8E0 | 6.3E0 | 6.5E0 | 7.6E0 | 1.0E-9 | 1.0E-9 | 4.3E1 | 2.4E1 | 81 | 16 | 81 | 16 | 0.66 |
| lO | pg/ml | 1.0E-9 | 1.0E-9 | 3.7E0 | 1.0E-9 | 2.1E1 | 0.0E0 | 1.0E-9 | 1.0E-9 | 1.4E2 | 1.0E-9 | 80 | 16 | 80 | 16 | 0.48 |
| zA | ng/ml | 2.1E7 | 2.5E7 | 2.1E7 | 2.5E7 | 6.1E6 | 7.1E6 | 9.1E6 | 1.7E7 | 3.4E7 | 3.6E7 | 48 | 7 | 48 | 7 | 0.67 |
| nW | pg/ml | 1.1E5 | 1.2E5 | 1.1E5 | 1.2E5 | 2.9E4 | 2.3E4 | 3.6E4 | 8.4E4 | 1.9E5 | 1.6E5 | 98 | 20 | 98 | 20 | 0.62 |
| nY | pg/ml | 2.2E3 | 3.8E3 | 2.5E3 | 3.7E3 | 1.3E3 | 2.3E3 | 5.1E2 | 1.2E3 | 8.1E3 | 1.0E4 | 98 | 20 | 98 | 20 | 0.67 |
| oO | pg/ml | 9.0E4 | 8.1E4 | 1.0E5 | 1.2E5 | 6.3E4 | 1.1E5 | 3.3E3 | 3.1E4 | 3.0E5 | 4.0E5 | 52 | 17 | 52 | 17 | 0.47 |
| oP | pg/ml | 1.2E5 | 2.0E5 | 1.3E5 | 2.3E5 | 6.5E4 | 1.5E5 | 2.4E4 | 5.0E4 | 3.1E5 | 5.7E5 | 52 | 17 | 52 | 17 | 0.71 |
| oQ | pg/ml | 2.9E3 | 3.8E3 | 3.2E3 | 7.4E3 | 1.9E3 | 8.3E3 | 7.7E2 | 1.4E3 | 1.1E4 | 3.2E4 | 52 | 17 | 52 | 17 | 0.65 |
| oE | pg/ml | 1.6E2 | 5.8E2 | 4.4E2 | 8.9E2 | 5.9E2 | 9.1E2 | 1.0E-9 | 1.0E-9 | 2.8E3 | 3.4E3 | 98 | 20 | 98 | 20 | 0.68 |
| oF | pg/ml | 1.2E4 | 2.8E4 | 2.5E4 | 4.4E4 | 3.5E4 | 5.9E4 | 4.3E2 | 3.5E2 | 1.7E5 | 2.5E5 | 98 | 20 | 98 | 20 | 0.63 |
| oH | pg/ml | 3.6E1 | 3.4E1 | 7.9E1 | 7.1E1 | 1.2E2 | 9.1E1 | 4.3E-1 | 6.3E0 | 8.6E2 | 3.2E2 | 98 | 20 | 98 | 20 | 0.50 |
| oK | pg/ml | 8.4E2 | 1.3E3 | 1.6E3 | 1.7E3 | 2.0E3 | 1.4E3 | 8.8E1 | 2.1E2 | 1.2E4 | 5.9E3 | 98 | 20 | 98 | 20 | 0.56 |
| oN | pg/ml | 5.4E2 | 5.8E2 | 9.7E2 | 8.7E2 | 2.0E3 | 7.5E2 | 1.1E2 | 2.8E2 | 1.8E4 | 3.6E3 | 98 | 20 | 98 | 20 | 0.59 |
| oW | pg/ml | 2.2E2 | 2.7E2 | 4.0E2 | 1.3E3 | 5.3E2 | 2.4E3 | 2.9E1 | 9.3E1 | 2.7E3 | 7.6E3 | 24 | 9 | 24 | 9 | 0.57 |
| oT | pg/ml | 3.0E2 | 2.5E2 | 3.6E2 | 2.3E2 | 1.9E2 | 7.2E1 | 1.0E2 | 1.1E2 | 7.9E2 | 3.2E2 | 24 | 9 | 24 | 9 | 0.27 |
| oV | pg/ml | 1.4E2 | 7.0E1 | 3.4E2 | 1.0E2 | 5.2E2 | 1.0E2 | 1.1E1 | 1.0E-9 | 2.2E3 | 3.3E2 | 24 | 9 | 24 | 9 | 0.36 |
| oD | pg/ml | 1.5E4 | 1.7E4 | 1.5E4 | 1.6E4 | 5.3E3 | 7.7E3 | 6.6E3 | 8.7E3 | 2.5E4 | 3.2E4 | 24 | 9 | 24 | 9 | 0.49 |
| pF | pg/ml | 5.6E-1 | 6.0E-1 | 1.8E0 | 7.0E-1 | 8.8E0 | 4.6E-1 | 1.0E-9 | 4.3E-2 | 8.7E1 | 1.6E0 | 98 | 20 | 98 | 20 | 0.49 |
| pH | ng/ml | 7.9E0 | 9.9E0 | 8.8E0 | 1.2E1 | 4.0E0 | 7.3E0 | 1.2E0 | 3.0E0 | 1.8E1 | 2.3E1 | 24 | 9 | 24 | 9 | 0.59 |
| pI | ng/ml | 6.9E1 | 7.1E1 | 7.5E1 | 6.7E1 | 4.8E1 | 2.6E1 | 2.3E1 | 3.5E1 | 2.0E2 | 1.1E2 | 24 | 9 | 24 | 9 | 0.52 |
| pK | ng/ml | 4.9E-1 | 4.0E-1 | 4.9E-1 | 3.7E-1 | 2.0E-1 | 1.4E-1 | 2.3E-1 | 1.7E-1 | 8.6E-1 | 6.2E-1 | 24 | 9 | 24 | 9 | 0.34 |

Figure 33.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.0E1 | 1.0E2 | 8.0E1 | 1.2E2 | 5.6E1 | 8.1E1 | 7.0E0 | 1.2E1 | 4.0E2 | 4.8E2 | 171 | 101 | 171 | 101 | 0.65 |
| Ad | ug/mL | 3.8E-2 | 5.8E-2 | 6.1E-2 | 2.2E-1 | 7.5E-2 | 1.0E0 | 6.8E-4 | 9.4E-4 | 3.7E-1 | 8.5E0 | 75 | 67 | 75 | 67 | 0.64 |
| Af | ng/mL | 1.3E0 | 1.1E0 | 1.2E1 | 7.8E0 | 6.1E1 | 2.4E1 | 1.7E-3 | 1.7E-3 | 5.3E2 | 1.9E2 | 75 | 67 | 75 | 67 | 0.50 |
| Aj | ug/mL | 1.6E0 | 3.6E-1 | 2.5E0 | 2.0E0 | 2.5E0 | 2.4E0 | 1.5E-3 | 2.1E-3 | 6.1E0 | 6.1E0 | 75 | 67 | 75 | 67 | 0.41 |
| Al | mg/mL | 8.3E-5 | 9.6E-5 | 2.3E-4 | 3.1E-4 | 4.2E-4 | 4.5E-4 | 4.6E-6 | 7.6E-6 | 1.8E-3 | 1.8E-3 | 75 | 67 | 75 | 67 | 0.58 |
| An | U/mL | 6.1E1 | 7.8E1 | 2.4E2 | 3.5E2 | 7.3E2 | 9.9E2 | 6.1E-1 | 6.4E-1 | 5.5E3 | 7.8E3 | 75 | 67 | 75 | 67 | 0.58 |
| Ao | pg/mL | 9.2E1 | 9.9E1 | 9.7E2 | 1.7E2 | 5.3E3 | 1.8E2 | 2.8E0 | 5.4E0 | 3.9E4 | 8.3E2 | 75 | 67 | 75 | 67 | 0.58 |
| Ap | ng/mL | 3.1E1 | 3.8E1 | 3.9E1 | 5.1E1 | 4.1E1 | 4.8E1 | 2.0E0 | 2.4E0 | 2.8E2 | 2.9E2 | 75 | 67 | 75 | 67 | 0.60 |
| Ar | ng/mL | 4.1E-1 | 1.7E0 | 1.6E0 | 5.6E0 | 3.6E0 | 1.1E1 | 4.1E-2 | 3.4E-3 | 2.0E1 | 5.1E1 | 75 | 67 | 75 | 67 | 0.71 |
| As | ng/mL | 8.0E-3 | 1.1E-2 | 1.1E-2 | 3.2E-2 | 1.6E-2 | 1.5E-1 | 1.7E-3 | 1.7E-3 | 9.8E-2 | 1.2E0 | 75 | 67 | 75 | 67 | 0.57 |
| Aw | pg/mL | 1.6E1 | 1.6E1 | 1.7E1 | 1.7E1 | 5.9E0 | 7.0E0 | 6.8E0 | 2.9E-2 | 4.2E1 | 5.1E1 | 75 | 67 | 75 | 67 | 0.52 |
| Ax | ng/mL | 1.3E0 | 6.6E0 | 8.1E0 | 6.5E1 | 1.8E1 | 1.7E2 | 1.3E-2 | 1.2E-2 | 1.1E2 | 8.5E2 | 75 | 67 | 75 | 67 | 0.66 |
| Ba | ng/mL | 7.7E1 | 1.3E2 | 6.1E2 | 6.6E2 | 1.6E3 | 1.5E3 | 1.1E0 | 3.1E0 | 8.1E3 | 8.1E3 | 75 | 67 | 75 | 67 | 0.58 |
| Bb | ng/mL | 3.6E0 | 6.0E0 | 5.8E0 | 8.9E0 | 8.2E0 | 8.4E0 | 4.1E-3 | 6.8E-1 | 4.9E1 | 3.7E1 | 75 | 67 | 75 | 67 | 0.65 |
| Bc | ng/mL | 2.6E1 | 6.0E1 | 1.0E2 | 1.6E2 | 2.1E2 | 2.6E2 | 4.9E-1 | 3.4E0 | 1.0E3 | 1.2E3 | 75 | 67 | 75 | 67 | 0.64 |
| Bg | ng/mL | 1.0E-1 | 1.3E-1 | 5.1E0 | 9.6E0 | 2.1E1 | 5.0E1 | 5.3E-4 | 1.1E-2 | 1.5E2 | 4.0E2 | 75 | 67 | 75 | 67 | 0.53 |
| Bn | ng/mL | 5.6E-2 | 5.6E-2 | 9.7E-1 | 2.1E0 | 1.8E0 | 7.3E0 | 5.6E-2 | 5.6E-2 | 7.4E0 | 5.8E1 | 75 | 67 | 75 | 67 | 0.52 |
| Bo | ng/mL | 1.3E1 | 1.4E1 | 1.4E1 | 1.7E1 | 9.8E0 | 1.3E1 | 1.6E-2 | 1.6E-2 | 3.8E1 | 5.3E1 | 75 | 67 | 75 | 67 | 0.55 |
| Ch | uIU/mL | 1.2E0 | 1.0E0 | 4.7E1 | 3.3E1 | 2.3E2 | 1.6E2 | 3.4E-3 | 3.4E-3 | 1.8E3 | 1.2E3 | 75 | 67 | 75 | 67 | 0.44 |
| Co | pg/mL | 4.4E1 | 5.4E1 | 1.5E2 | 3.7E2 | 5.0E2 | 2.0E3 | 1.5E-1 | 1.5E-1 | 3.7E3 | 1.7E4 | 75 | 67 | 75 | 67 | 0.57 |
| Cp | ng/mL | 2.1E1 | 2.2E1 | 2.7E1 | 5.0E1 | 2.2E1 | 1.5E2 | 6.0E-1 | 2.5E0 | 1.3E2 | 1.3E3 | 75 | 67 | 75 | 67 | 0.57 |
| Cq | ng/mL | 2.8E-2 | 4.1E-2 | 7.9E-2 | 9.1E-1 | 2.5E-1 | 6.0E0 | 8.0E-4 | 8.0E-4 | 2.0E0 | 4.9E1 | 75 | 67 | 75 | 67 | 0.60 |
| Cs | ng/mL | 4.6E1 | 2.1E2 | 2.1E2 | 9.8E2 | 4.1E2 | 2.6E3 | 1.0E0 | 8.3E-1 | 1.9E3 | 1.8E4 | 75 | 67 | 75 | 67 | 0.66 |
| Ct | ng/mL | 5.7E-1 | 1.8E-1 | 4.6E1 | 4.5E1 | 1.3E2 | 1.3E2 | 2.2E-2 | 1.1E-4 | 4.7E2 | 6.2E2 | 75 | 67 | 75 | 67 | 0.43 |
| Cu | ng/mL | 2.1E-1 | 3.3E-1 | 3.4E-1 | 1.9E0 | 3.6E-1 | 8.4E0 | 2.8E-2 | 3.5E-2 | 1.7E0 | 6.6E1 | 75 | 67 | 75 | 67 | 0.63 |
| Cv | ng/mL | 2.8E0 | 9.1E0 | 2.0E1 | 3.6E1 | 6.5E1 | 7.4E1 | 2.0E-2 | 2.4E-2 | 5.3E2 | 4.7E2 | 75 | 67 | 75 | 67 | 0.61 |
| Cw | mIU/mL | 2.8E-2 | 4.3E-2 | 3.8E-2 | 1.5E-1 | 3.0E-2 | 8.2E-1 | 8.9E-4 | 4.1E-3 | 1.5E-1 | 6.8E0 | 75 | 67 | 75 | 67 | 0.58 |
| Cx | ng/mL | 8.5E-1 | 4.6E-1 | 4.8E1 | 4.5E1 | 9.4E1 | 9.5E1 | 9.3E-5 | 9.3E-5 | 3.9E2 | 3.9E2 | 75 | 67 | 75 | 67 | 0.51 |
| Db | ug/mL | 7.5E0 | 7.3E0 | 8.6E0 | 8.5E0 | 6.9E0 | 8.6E0 | 4.5E-1 | 8.2E-1 | 4.3E1 | 5.9E1 | 75 | 67 | 75 | 67 | 0.48 |
| Dc | nmol/L | 1.5E-2 | 2.9E-2 | 3.4E-2 | 3.5E-1 | 5.2E-2 | 1.7E0 | 5.2E-6 | 1.3E-3 | 3.0E-1 | 1.4E1 | 75 | 67 | 75 | 67 | 0.65 |
| Dd | ug/mL | 4.7E-2 | 1.6E-1 | 1.3E-1 | 3.0E-1 | 2.4E-1 | 5.0E-1 | 4.8E-4 | 3.4E-3 | 1.6E0 | 3.6E0 | 75 | 67 | 75 | 67 | 0.65 |
| De | ng/mL | 3.4E-3 | 3.4E-3 | 7.5E-2 | 9.8E-2 | 1.5E-1 | 1.9E-1 | 3.4E-3 | 3.4E-3 | 9.2E-1 | 1.1E0 | 75 | 67 | 75 | 67 | 0.52 |
| Dg | ng/mL | 2.9E1 | 4.2E1 | 4.0E1 | 5.5E1 | 3.5E1 | 4.2E1 | 7.8E-1 | 7.1E-1 | 1.2E2 | 1.9E2 | 75 | 67 | 75 | 67 | 0.61 |
| Di | pg/mL | 2.0E0 | 2.7E0 | 2.4E0 | 2.6E0 | 2.4E0 | 1.9E0 | 1.8E-1 | 1.8E-1 | 1.3E1 | 9.0E0 | 75 | 67 | 75 | 67 | 0.57 |
| Dk | uIU/mL | 1.2E-2 | 1.8E-2 | 6.9E-2 | 6.9E-2 | 2.2E-1 | 1.6E-1 | 1.1E-4 | 1.1E-4 | 1.6E0 | 9.8E-1 | 75 | 67 | 75 | 67 | 0.57 |
| Dl | ng/mL | 1.8E2 | 2.5E2 | 2.6E2 | 3.7E2 | 2.3E2 | 3.4E2 | 8.9E0 | 5.5E0 | 1.1E3 | 1.6E3 | 75 | 67 | 75 | 67 | 0.59 |
| Dp | ng/ml | 2.1E0 | 2.1E0 | 4.9E0 | 5.8E0 | 7.1E0 | 1.1E1 | 1.5E-2 | 3.7E-3 | 3.5E1 | 5.6E1 | 57 | 57 | 57 | 57 | 0.48 |
| Dr | pg/ml | 9.6E0 | 3.2E1 | 3.6E1 | 5.1E2 | 5.7E1 | 2.1E3 | 7.5E-1 | 7.5E-1 | 2.5E2 | 1.0E4 | 33 | 25 | 33 | 25 | 0.70 |
| Du | pg/ml | 4.8E1 | 5.4E2 | 5.3E2 | 2.6E3 | 1.1E3 | 6.2E3 | 1.2E0 | 1.2E0 | 5.4E3 | 2.4E4 | 26 | 22 | 26 | 22 | 0.68 |
| Dw | ng/ml | 9.2E-3 | 3.0E-2 | 4.4E-2 | 5.7E-2 | 6.5E-2 | 7.1E-2 | 9.2E-3 | 9.2E-3 | 1.9E-1 | 1.9E-1 | 8 | 9 | 8 | 9 | 0.59 |
| Ef | ng/ml | 8.4E-2 | 1.3E-1 | 7.5E-1 | 9.6E-1 | 1.4E0 | 2.4E0 | 5.7E-4 | 5.7E-4 | 4.7E0 | 9.9E0 | 62 | 62 | 62 | 62 | 0.56 |
| Wm | % | 8.5E-2 | 4.5E-1 | 4.2E0 | 5.1E1 | 2.3E1 | 2.0E2 | 5.4E-2 | 8.5E-2 | 2.0E2 | 1.0E3 | 70 | 61 | 70 | 61 | 0.57 |
| Ed | pg/ml | 5.2E-1 | 1.5E1 | 2.4E1 | 5.4E1 | 3.5E1 | 9.7E1 | 5.2E-1 | 5.2E-1 | 1.3E2 | 5.0E2 | 57 | 57 | 57 | 57 | 0.58 |
| Eo | ng/ml | 7.0E0 | 1.8E0 | 7.5E0 | 7.1E0 | 5.2E0 | 1.3E1 | 3.6E-1 | 3.6E-1 | 1.6E1 | 4.0E1 | 8 | 9 | 8 | 9 | 0.33 |
| Yf | ng/mL | 1.5E1 | 1.5E1 | 3.4E1 | 8.1E1 | 5.0E1 | 1.5E2 | 2.9E-1 | 2.9E-1 | 2.4E2 | 5.9E2 | 28 | 21 | 28 | 21 | 0.47 |
| Tj | pg/mL | 3.7E-1 | 3.7E-1 | 2.8E1 | 6.2E1 | 1.2E2 | 3.0E2 | 3.7E-1 | 3.7E-1 | 8.7E2 | 2.3E3 | 61 | 59 | 61 | 59 | 0.56 |
| Po | pg/ml | 1.1E-1 | 2.5E0 | 7.9E0 | 1.6E1 | 2.7E1 | 3.8E1 | 8.0E-3 | 2.6E-2 | 2.7E2 | 2.2E2 | 155 | 114 | 155 | 114 | 0.61 |
| Ti | ug/mL | 2.2E0 | 5.8E0 | 3.4E0 | 6.5E0 | 3.3E0 | 4.7E0 | 1.2E-1 | 6.0E-1 | 1.4E1 | 1.8E1 | 38 | 31 | 38 | 31 | 0.72 |
| Em | ng/ml | 1.3E-2 | 2.2E-2 | 5.9E-2 | 1.2E-1 | 9.6E-2 | 3.4E-1 | 8.4E-4 | 8.4E-4 | 5.0E-1 | 1.9E0 | 39 | 34 | 39 | 34 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Et | ng/ml | 1.3E3 | 2.2E3 | 1.5E3 | 2.3E3 | 1.1E3 | 1.2E3 | 7.9E1 | 3.1E2 | 4.3E3 | 5.0E3 | 154 | 114 | 154 | 114 | 0.70 |
| Eq | pg/ml | 2.6E2 | 6.6E1 | 3.9E2 | 3.0E2 | 4.2E2 | 4.1E2 | 1.0E0 | 1.0E0 | 1.5E3 | 1.4E3 | 26 | 22 | 26 | 22 | 0.40 |
| Ew | U/ml | 1.9E0 | 2.2E0 | 2.8E0 | 2.5E0 | 2.5E0 | 1.2E0 | 1.1E0 | 1.3E0 | 8.8E0 | 5.0E0 | 8 | 9 | 8 | 9 | 0.58 |
| Th | ug/mL | 1.2E0 | 1.6E0 | 1.8E0 | 2.0E0 | 1.4E0 | 1.9E0 | 2.4E-1 | 2.6E-3 | 5.4E0 | 7.5E0 | 38 | 31 | 38 | 31 | 0.50 |
| Fa | ng/ml | 3.3E1 | 6.5E1 | 4.5E1 | 2.2E2 | 4.5E1 | 5.9E2 | 2.6E-1 | 6.0E-1 | 2.2E2 | 3.7E3 | 56 | 55 | 56 | 55 | 0.66 |
| Ez | ng/ml | 5.0E0 | 4.1E0 | 1.6E1 | 1.5E1 | 2.9E1 | 3.2E1 | 1.3E-2 | 1.3E-2 | 1.6E2 | 2.0E2 | 57 | 57 | 57 | 57 | 0.53 |
| Fb | ng/ml | 2.3E1 | 2.7E1 | 2.1E1 | 2.5E1 | 1.3E1 | 9.6E0 | 8.9E-1 | 6.6E-1 | 4.3E1 | 4.1E1 | 56 | 56 | 56 | 56 | 0.57 |
| Ex | ng/ml | 5.5E-2 | 1.4E-1 | 1.5E-1 | 2.2E-1 | 2.4E-1 | 3.1E-1 | 3.5E-5 | 1.7E-4 | 1.0E0 | 1.5E0 | 43 | 45 | 43 | 45 | 0.64 |
| Fc | pg/ml | 2.2E-1 | 2.2E-1 | 4.1E0 | 3.6E1 | 9.9E0 | 1.0E2 | 2.2E-1 | 2.2E-1 | 4.4E1 | 3.9E2 | 27 | 22 | 27 | 22 | 0.57 |
| Fd | pg/ml | 2.3E1 | 2.1E2 | 2.9E2 | 2.9E3 | 6.2E2 | 7.0E3 | 4.5E-1 | 9.8E-1 | 2.7E3 | 2.5E4 | 27 | 22 | 27 | 22 | 0.62 |
| Fi | pg/ml | 2.5E-1 | 2.5E-1 | 2.1E1 | 1.4E2 | 6.1E1 | 4.0E2 | 2.5E-1 | 2.5E-1 | 2.5E2 | 1.8E3 | 27 | 22 | 27 | 22 | 0.61 |
| Fn | ng/ml | 2.1E-1 | 2.1E-1 | 4.8E0 | 4.1E0 | 8.6E0 | 6.3E0 | 1.1E-14 | 1.1E-14 | 3.7E1 | 2.7E1 | 57 | 57 | 57 | 57 | 0.50 |
| Fp | ng/ml | 9.3E0 | 2.3E1 | 1.9E1 | 3.4E1 | 2.4E1 | 3.2E1 | 6.0E-3 | 2.8E-1 | 1.2E2 | 1.3E2 | 156 | 116 | 156 | 116 | 0.66 |
| Fr | ng/ml | 3.0E4 | 6.5E4 | 1.2E5 | 1.7E5 | 1.9E5 | 2.3E5 | 1.9E2 | 1.8E3 | 8.4E5 | 8.4E5 | 159 | 117 | 159 | 117 | 0.63 |
| Fw | pg/ml | 8.5E-1 | 5.1E0 | 5.5E1 | 4.8E1 | 3.7E2 | 1.4E2 | 1.2E-1 | 1.2E-1 | 3.0E3 | 9.1E2 | 63 | 61 | 63 | 61 | 0.62 |
| Fy | ng/ml | 3.2E1 | 5.4E1 | 4.7E1 | 1.0E2 | 4.9E1 | 1.4E2 | 1.2E-1 | 1.2E-1 | 2.2E2 | 6.5E2 | 57 | 55 | 57 | 55 | 0.63 |
| Gh | pg/ml | 2.0E0 | 2.8E0 | 2.4E1 | 1.4E1 | 6.8E1 | 3.1E1 | 2.9E-2 | 2.9E-2 | 3.2E2 | 1.4E2 | 27 | 22 | 27 | 22 | 0.52 |
| Gb | % | 4.3E1 | 5.2E1 | 3.9E1 | 6.9E1 | 2.6E1 | 6.6E1 | 2.2E0 | 1.3E1 | 1.0E2 | 3.0E2 | 27 | 22 | 27 | 22 | 0.65 |
| Gc | ng/ml | 6.2E1 | 1.6E2 | 1.0E2 | 2.8E2 | 1.3E2 | 2.7E2 | 6.4E0 | 6.9E0 | 7.3E2 | 1.2E3 | 33 | 25 | 33 | 25 | 0.77 |
| Gd | ng/ml | 3.3E1 | 3.7E1 | 3.4E1 | 3.6E1 | 1.7E1 | 2.0E1 | 3.0E0 | 3.7E0 | 6.9E1 | 8.0E1 | 40 | 27 | 40 | 27 | 0.51 |
| Gn | U/ml | 2.3E-1 | 2.7E-1 | 1.7E0 | 5.2E0 | 5.4E0 | 2.3E1 | 5.6E-3 | 1.2E-2 | 3.0E1 | 1.1E2 | 32 | 25 | 32 | 25 | 0.55 |
| Gl | pg/ml | 8.9E3 | 1.1E4 | 1.2E4 | 1.4E4 | 9.4E3 | 9.9E3 | 9.1E3 | 4.0E2 | 3.1E4 | 3.1E4 | 63 | 61 | 63 | 61 | 0.56 |
| Gp | U/ml | 1.3E0 | 1.0E0 | 2.2E0 | 2.2E0 | 2.8E0 | 3.3E0 | 1.5E-2 | 1.5E-2 | 1.6E1 | 1.8E1 | 63 | 61 | 63 | 61 | 0.46 |
| Gz | ug/ml | 1.1E1 | 1.1E0 | 6.5E0 | 4.6E0 | 6.2E0 | 5.5E0 | 4.2E-2 | 1.0E-1 | 2.5E1 | 1.9E1 | 38 | 42 | 38 | 42 | 0.46 |
| Ha | ng/ml | 1.6E0 | 3.3E0 | 5.5E0 | 1.3E1 | 1.5E1 | 2.6E1 | 1.7E-2 | 1.7E-2 | 1.0E2 | 1.0E2 | 55 | 57 | 55 | 57 | 0.63 |
| Nm | pg/ml | 1.3E4 | 2.0E4 | 2.7E4 | 6.1E4 | 6.6E4 | 1.3E5 | 1.0E-9 | 1.0E-9 | 7.8E5 | 9.6E5 | 156 | 116 | 156 | 116 | 0.61 |
| Nn | pg/ml | 1.4E2 | 2.9E2 | 1.6E3 | 6.6E3 | 6.0E3 | 3.2E4 | 1.0E-9 | 1.0E-9 | 6.0E4 | 3.1E5 | 156 | 116 | 156 | 116 | 0.60 |
| No | pg/ml | 1.3E1 | 2.5E1 | 2.9E1 | 6.7E1 | 5.6E1 | 1.4E2 | 1.0E-9 | 1.0E-9 | 5.6E2 | 9.1E2 | 156 | 116 | 156 | 116 | 0.63 |
| Nq | pg/ml | 2.0E0 | 2.9E0 | 2.5E1 | 3.5E1 | 7.8E1 | 1.1E2 | 1.0E-9 | 1.0E-9 | 6.2E2 | 6.7E2 | 156 | 116 | 156 | 116 | 0.52 |
| Nr | pg/ml | 1.3E0 | 4.2E0 | 2.1E1 | 4.5E1 | 9.2E1 | 1.5E2 | 1.0E-9 | 1.0E-9 | 9.8E2 | 1.4E3 | 156 | 116 | 156 | 116 | 0.61 |
| Ns | pg/ml | 1.0E-9 | 1.0E-9 | 1.3E1 | 4.2E0 | 9.3E1 | 2.6E1 | 1.0E-9 | 1.0E-9 | 1.1E3 | 2.4E2 | 156 | 116 | 156 | 116 | 0.49 |
| Nt | pg/ml | 9.8E1 | 1.4E2 | 1.3E2 | 1.9E2 | 1.1E2 | 2.0E2 | 1.5E1 | 1.5E1 | 8.8E2 | 1.7E3 | 156 | 116 | 156 | 116 | 0.64 |
| Nu | pg/ml | 1.9E1 | 4.7E1 | 5.2E1 | 7.7E1 | 8.2E1 | 9.9E1 | 1.0E-9 | 1.0E-9 | 3.7E2 | 6.3E2 | 156 | 116 | 156 | 116 | 0.61 |
| Lu | pg/ml | 1.0E4 | 1.0E4 | 2.0E4 | 1.1E4 | 5.8E4 | 1.0E4 | 9.4E2 | 5.2E2 | 5.6E5 | 5.9E4 | 156 | 116 | 156 | 116 | 0.48 |
| Lv | pg/ml | 1.0E-9 | 1.4E0 | 1.3E1 | 2.5E1 | 2.6E1 | 4.3E1 | 1.0E-9 | 1.0E-9 | 2.4E2 | 2.6E2 | 156 | 116 | 156 | 116 | 0.58 |
| Lw | pg/ml | 1.0E-9 | 1.0E-9 | 2.0E-1 | 3.0E0 | 2.6E0 | 1.8E1 | 1.0E-9 | 1.0E-9 | 3.2E1 | 1.8E2 | 156 | 116 | 156 | 116 | 0.53 |
| Lx | pg/ml | 1.0E-9 | 6.0E1 | 1.5E2 | 5.4E2 | 5.6E2 | 2.2E3 | 1.0E-9 | 1.0E-9 | 6.2E3 | 2.2E4 | 156 | 116 | 156 | 116 | 0.65 |
| Ly | pg/ml | 1.0E-9 | 1.0E-9 | 8.5E0 | 1.1E1 | 1.7E1 | 1.9E1 | 1.0E-9 | 1.0E-9 | 1.1E2 | 9.6E1 | 156 | 116 | 156 | 116 | 0.54 |
| Lz | pg/ml | 1.0E-9 | 1.0E-9 | 3.0E0 | 4.3E0 | 3.4E1 | 1.8E1 | 1.0E-9 | 1.0E-9 | 4.3E2 | 1.3E2 | 156 | 116 | 156 | 116 | 0.55 |
| Ma | pg/ml | 4.3E2 | 8.2E2 | 2.2E3 | 3.3E3 | 6.4E3 | 7.4E3 | 1.0E-9 | 1.0E-9 | 6.5E4 | 5.2E4 | 156 | 116 | 156 | 116 | 0.60 |
| Mb | pg/ml | 2.5E1 | 2.8E1 | 3.1E1 | 3.5E1 | 1.4E1 | 2.3E1 | 9.2E0 | 4.1E0 | 6.9E1 | 2.1E2 | 156 | 116 | 156 | 116 | 0.54 |
| Mc | pg/ml | 1.0E-9 | 1.0E-9 | 3.8E-2 | 1.1E-1 | 3.5E-1 | 1.2E0 | 1.0E-9 | 1.0E-9 | 4.0E0 | 1.3E1 | 156 | 116 | 156 | 116 | 0.49 |
| Md | pg/ml | 1.0E-9 | 1.0E-9 | 5.5E-1 | 7.1E-1 | 5.2E0 | 3.5E0 | 1.0E-9 | 1.0E-9 | 6.5E1 | 2.9E1 | 156 | 116 | 156 | 116 | 0.52 |
| Me | pg/ml | 3.1E1 | 3.3E1 | 2.9E1 | 3.4E1 | 1.7E1 | 3.6E1 | 1.1E-1 | 1.0E-9 | 1.2E2 | 3.2E2 | 156 | 116 | 156 | 116 | 0.52 |
| Mf | pg/ml | 1.0E-9 | 1.0E-9 | 3.9E-1 | 1.3E0 | 1.9E0 | 6.3E0 | 1.0E-9 | 1.0E-9 | 2.0E1 | 5.6E1 | 156 | 116 | 156 | 116 | 0.54 |
| Mg | pg/ml | 1.0E0 | 1.8E0 | 6.7E0 | 9.6E0 | 1.2E1 | 2.0E1 | 1.0E-9 | 1.0E-9 | 8.8E1 | 1.5E2 | 156 | 116 | 156 | 116 | 0.54 |
| Mh | pg/ml | 1.0E-9 | 1.0E-9 | 1.1E0 | 1.2E0 | 8.9E0 | 4.6E0 | 1.0E-9 | 1.0E-9 | 1.1E2 | 4.2E1 | 156 | 116 | 156 | 116 | 0.57 |
| Mi | pg/ml | 1.0E-9 | 1.0E-9 | 1.2E0 | 9.5E0 | 1.0E1 | 5.8E1 | 1.0E-9 | 1.0E-9 | 1.2E2 | 5.2E2 | 156 | 116 | 156 | 116 | 0.53 |
| Mj | pg/ml | 1.0E-9 | 1.0E-9 | 6.1E0 | 9.8E0 | 3.8E1 | 3.0E1 | 1.0E-9 | 1.0E-9 | 4.6E2 | 2.1E2 | 156 | 116 | 156 | 116 | 0.53 |
| Mk | pg/ml | 3.7E0 | 2.2E0 | 1.6E1 | 2.3E1 | 8.4E1 | 1.3E2 | 1.0E-9 | 1.0E-9 | 1.0E3 | 1.3E3 | 156 | 116 | 156 | 116 | 0.48 |
| Ml | pg/ml | 1.0E-9 | 1.0E-9 | 1.5E1 | 1.2E1 | 1.7E2 | 5.9E1 | 1.0E-9 | 1.0E-9 | 2.1E3 | 5.1E2 | 156 | 116 | 156 | 116 | 0.54 |
| Mm | pg/ml | 4.0E2 | 8.7E2 | 9.0E2 | 1.5E3 | 1.1E3 | 1.8E3 | 1.0E-9 | 1.9E1 | 6.0E3 | 1.0E4 | 156 | 116 | 156 | 116 | 0.63 |
| Mn | pg/ml | 5.2E0 | 9.3E0 | 1.2E1 | 1.3E1 | 3.5E1 | 1.2E1 | 1.0E-9 | 1.0E-9 | 3.5E2 | 6.8E1 | 156 | 116 | 156 | 116 | 0.66 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Mp | pg/ml | 1.0E-9 | 1.0E-9 | 1.0E1 | 4.7E1 | 2.5E1 | 2.3E2 | 1.0E-9 | 1.0E-9 | 1.8E2 | 2.4E3 | 156 | 116 | 156 | 116 | 0.55 |
| Mq | pg/ml | 1.0E-9 | 1.0E-9 | 2.1E0 | 4.6E0 | 1.5E1 | 1.7E1 | 1.0E-9 | 1.0E-9 | 1.8E2 | 1.0E2 | 156 | 116 | 156 | 116 | 0.53 |
| Mr | pg/ml | 1.0E-9 | 1.0E-9 | 2.2E1 | 9.9E1 | 1.2E2 | 4.2E2 | 1.0E-9 | 1.0E-9 | 1.4E3 | 3.4E3 | 156 | 116 | 156 | 116 | 0.57 |
| Ms | pg/ml | 3.9E2 | 2.9E2 | 5.1E2 | 3.7E2 | 5.8E2 | 3.7E2 | 1.0E-9 | 1.0E-9 | 3.3E3 | 1.5E3 | 156 | 116 | 156 | 116 | 0.43 |
| Mt | pg/ml | 1.0E-9 | 1.0E0 | 1.0E1 | 4.4E1 | 6.0E1 | 3.0E2 | 1.0E-9 | 1.0E-9 | 7.1E2 | 3.2E3 | 156 | 116 | 156 | 116 | 0.61 |
| Mu | pg/ml | 1.0E-9 | 1.0E-9 | 1.3E0 | 3.4E0 | 9.0E0 | 2.2E1 | 1.0E-9 | 1.0E-9 | 9.9E1 | 2.3E2 | 156 | 116 | 156 | 116 | 0.54 |
| Mv | pg/ml | 1.0E-9 | 1.0E-9 | 9.8E1 | 7.4E1 | 4.8E2 | 2.0E2 | 1.0E-9 | 1.0E-9 | 4.5E3 | 9.2E2 | 156 | 116 | 156 | 116 | 0.54 |
| Mw | pg/ml | 2.3E1 | 5.8E1 | 4.0E2 | 3.2E2 | 2.0E3 | 8.5E2 | 1.0E-9 | 1.0E-9 | 1.8E4 | 5.3E3 | 156 | 116 | 156 | 116 | 0.59 |
| Mx | pg/ml | 1.0E-9 | 1.0E-9 | 2.4E-1 | 8.8E-1 | 9.0E-1 | 3.5E0 | 1.0E-9 | 1.0E-9 | 7.4E0 | 3.2E1 | 156 | 116 | 156 | 116 | 0.58 |
| My | pg/ml | 1.0E-9 | 1.0E-9 | 5.7E2 | 1.9E2 | 3.5E3 | 8.1E2 | 1.0E-9 | 1.0E-9 | 3.9E4 | 8.0E3 | 156 | 116 | 156 | 116 | 0.50 |
| Mz | pg/ml | 9.6E0 | 1.8E1 | 2.0E1 | 6.6E1 | 5.0E1 | 2.2E2 | 1.0E-9 | 1.0E-9 | 5.5E2 | 1.9E3 | 156 | 116 | 156 | 116 | 0.67 |
| Na | pg/ml | 1.0E-9 | 1.0E-9 | 5.9E-1 | 8.6E-1 | 1.9E0 | 4.1E0 | 1.0E-9 | 1.0E-9 | 9.6E0 | 4.2E1 | 156 | 116 | 156 | 116 | 0.52 |
| Nb | pg/ml | 1.8E0 | 2.5E0 | 4.2E0 | 6.5E0 | 1.4E1 | 2.2E1 | 1.0E-9 | 1.0E-9 | 1.5E2 | 2.1E2 | 156 | 116 | 156 | 116 | 0.57 |
| Nc | pg/ml | 4.1E2 | 1.7E2 | 6.0E2 | 3.7E2 | 9.0E2 | 4.9E2 | 1.0E-9 | 1.0E-9 | 9.2E3 | 3.2E3 | 156 | 116 | 156 | 116 | 0.39 |
| Nd | pg/ml | 2.8E1 | 1.3E1 | 2.4E1 | 5.1E1 | 1.8E1 | 2.3E2 | 1.0E-9 | 1.0E-9 | 7.8E1 | 2.1E3 | 156 | 116 | 156 | 116 | 0.46 |
| Ne | pg/ml | 4.6E2 | 3.5E2 | 5.8E2 | 4.3E2 | 6.7E2 | 4.4E2 | 1.0E-9 | 1.0E-9 | 7.0E3 | 3.6E3 | 156 | 116 | 156 | 116 | 0.41 |
| Nf | pg/ml | 1.0E-9 | 1.0E-9 | 2.9E0 | 3.4E0 | 1.1E1 | 1.7E1 | 1.0E-9 | 1.0E-9 | 8.2E1 | 1.3E2 | 156 | 116 | 156 | 116 | 0.47 |
| Ng | pg/ml | 1.7E1 | 1.9E1 | 1.1E2 | 9.7E1 | 2.2E2 | 1.7E2 | 1.0E-9 | 1.0E-9 | 1.6E3 | 8.9E2 | 156 | 116 | 156 | 116 | 0.52 |
| Nh | pg/ml | 6.4E1 | 5.3E1 | 8.9E1 | 6.6E1 | 8.3E1 | 6.7E1 | 1.0E-9 | 3.1E0 | 5.6E2 | 5.1E2 | 156 | 116 | 156 | 116 | 0.40 |
| Ni | pg/ml | 2.2E0 | 1.0E-9 | 8.2E1 | 9.8E1 | 1.3E2 | 1.6E2 | 1.0E-9 | 1.0E-9 | 7.1E2 | 1.1E3 | 156 | 116 | 156 | 116 | 0.50 |
| Nj | pg/ml | 7.4E0 | 4.5E0 | 1.1E1 | 7.9E0 | 1.1E1 | 9.7E0 | 1.0E-9 | 1.0E-9 | 6.8E1 | 5.8E1 | 156 | 116 | 156 | 116 | 0.38 |
| Nk | pg/ml | 2.0E1 | 1.4E1 | 3.2E1 | 3.0E1 | 3.8E1 | 4.1E1 | 1.0E-9 | 1.0E-9 | 2.0E2 | 2.0E2 | 156 | 116 | 156 | 116 | 0.48 |
| Nl | pg/ml | 4.6E1 | 3.3E1 | 6.4E1 | 4.2E1 | 9.6E1 | 4.2E1 | 1.0E-9 | 1.0E-9 | 1.1E3 | 2.3E2 | 156 | 116 | 156 | 116 | 0.40 |
| Hl | pg/ml | 2.2E1 | 1.2E1 | 4.0E1 | 1.9E2 | 6.7E1 | 7.6E2 | 1.0E-9 | 1.0E-9 | 3.0E2 | 3.6E3 | 27 | 22 | 27 | 22 | 0.51 |
| Ho | pg/ml | 1.7E1 | 2.0E1 | 2.3E1 | 5.2E1 | 2.0E1 | 8.6E1 | 1.1E0 | 6.7E0 | 8.3E1 | 3.9E2 | 27 | 22 | 27 | 22 | 0.61 |
| Hp | ng/ml | 1.8E0 | 1.6E0 | 1.4E2 | 1.2E2 | 3.2E2 | 3.1E2 | 1.0E-9 | 5.3E-1 | 8.9E2 | 8.9E2 | 27 | 22 | 27 | 22 | 0.47 |
| Tz | pg/ml | 3.2E3 | 7.3E3 | 4.9E3 | 5.3E4 | 5.7E3 | 2.8E5 | 7.4E1 | 1.0E-9 | 3.2E4 | 2.1E6 | 58 | 57 | 58 | 57 | 0.67 |
| Ua | pg/ml | 2.1E3 | 4.1E3 | 1.6E4 | 1.3E4 | 3.2E4 | 2.3E4 | 2.3E2 | 1.0E-9 | 1.4E5 | 1.2E5 | 58 | 57 | 58 | 57 | 0.58 |
| Ub | pg/ml | 6.6E2 | 4.1E2 | 9.6E2 | 6.5E2 | 1.4E3 | 8.1E2 | 5.4E0 | 1.0E-9 | 9.8E3 | 4.4E3 | 58 | 57 | 58 | 57 | 0.42 |
| Ue | pg/ml | 3.1E1 | 2.6E1 | 3.4E1 | 3.9E1 | 2.1E1 | 4.3E1 | 6.6E0 | 9.8E-2 | 9.5E1 | 2.7E2 | 58 | 57 | 58 | 57 | 0.48 |
| Uc | pg/ml | 6.6E2 | 9.4E2 | 1.1E3 | 2.7E3 | 1.1E3 | 7.7E3 | 1.5E1 | 1.0E-9 | 5.5E3 | 5.7E4 | 58 | 57 | 58 | 57 | 0.57 |
| Ud | pg/ml | 1.0E-9 | 1.0E-9 | 6.7E0 | 9.4E-1 | 5.1E1 | 7.1E0 | 1.0E-9 | 1.0E-9 | 3.9E2 | 5.3E1 | 58 | 57 | 58 | 57 | 0.50 |
| Hq | pg/ml | 1.1E0 | 1.3E0 | 1.4E2 | 2.8E2 | 1.6E3 | 2.7E3 | 1.0E-9 | 1.0E-9 | 2.0E4 | 2.8E4 | 156 | 114 | 156 | 114 | 0.52 |
| Hr | pg/ml | 8.8E1 | 8.0E1 | 5.6E2 | 5.6E2 | 1.1E3 | 1.3E3 | 1.0E-9 | 1.0E-9 | 8.4E3 | 8.9E3 | 156 | 114 | 156 | 114 | 0.49 |
| Hu | pg/ml | 7.7E0 | 2.1E1 | 7.9E3 | 2.8E3 | 5.5E4 | 2.5E4 | 1.0E-9 | 1.0E-9 | 6.3E5 | 2.6E5 | 156 | 114 | 156 | 114 | 0.54 |
| Hv | pg/ml | 1.5E0 | 1.4E0 | 2.7E0 | 1.2E1 | 6.5E0 | 8.5E1 | 1.0E-9 | 1.0E-9 | 6.9E1 | 8.9E2 | 156 | 114 | 156 | 114 | 0.51 |
| Hw | pg/ml | 5.5E0 | 5.6E0 | 1.8E1 | 1.0E2 | 6.3E1 | 8.8E2 | 1.0E-9 | 1.0E-9 | 6.4E2 | 9.4E3 | 156 | 114 | 156 | 114 | 0.49 |
| Hx | pg/ml | 8.4E0 | 1.2E1 | 8.4E1 | 4.8E1 | 7.4E2 | 1.5E2 | 1.0E-9 | 1.0E-9 | 9.3E3 | 1.3E3 | 156 | 114 | 156 | 114 | 0.57 |
| Ib | ng/ml | 4.1E-2 | 3.3E-2 | 1.8E0 | 1.2E0 | 6.6E0 | 7.6E0 | 1.0E-9 | 1.0E-9 | 3.6E1 | 5.6E1 | 57 | 55 | 57 | 55 | 0.49 |
| Ic | U/ml | 2.2E2 | 2.2E2 | 3.0E2 | 1.6E3 | 5.9E2 | 8.8E3 | 2.4E0 | 6.6E0 | 4.1E3 | 6.5E4 | 57 | 55 | 57 | 55 | 0.58 |
| Id | U/ml | 4.8E-1 | 9.9E-1 | 7.9E-1 | 9.8E0 | 1.0E0 | 5.8E1 | 1.0E-9 | 5.1E-2 | 6.4E0 | 4.3E2 | 57 | 55 | 57 | 55 | 0.70 |
| Tt | pg/ml | 1.7E2 | 1.8E2 | 1.7E2 | 1.8E2 | 5.1E1 | 6.5E1 | 4.3E1 | 7.3E1 | 3.0E2 | 4.4E2 | 51 | 53 | 51 | 53 | 0.53 |
| To | pg/ml | 1.8E0 | 1.6E0 | 1.9E0 | 2.2E0 | 1.9E0 | 2.6E0 | 1.0E-9 | 1.0E-9 | 7.6E0 | 1.2E1 | 55 | 55 | 55 | 55 | 0.50 |
| Tr | pg/ml | 2.9E0 | 3.8E0 | 5.3E0 | 1.4E1 | 6.5E0 | 4.3E1 | 1.0E-9 | 2.4E-1 | 3.8E1 | 3.1E2 | 53 | 54 | 53 | 54 | 0.58 |
| Tn | pg/ml | 2.6E1 | 4.4E1 | 8.2E1 | 1.8E2 | 2.3E2 | 4.6E2 | 3.5E0 | 2.4E0 | 1.7E3 | 2.3E3 | 55 | 55 | 55 | 55 | 0.62 |
| Tv | ng/ml | 1.2E1 | 1.5E1 | 1.5E1 | 1.7E2 | 1.6E1 | 9.6E2 | 1.0E-9 | 1.0E-9 | 7.5E1 | 7.1E3 | 55 | 55 | 55 | 55 | 0.57 |
| Ih | ng/ml | 5.3E1 | 1.3E2 | 1.8E2 | 3.8E2 | 3.2E2 | 5.9E2 | 1.0E-9 | 1.0E-9 | 1.8E3 | 3.6E3 | 156 | 115 | 156 | 115 | 0.63 |
| Ii | ng/ml | 6.3E1 | 1.2E2 | 2.2E2 | 2.7E2 | 5.4E2 | 5.8E2 | 1.6E0 | 7.3E-1 | 5.2E3 | 4.5E3 | 156 | 115 | 156 | 115 | 0.61 |
| Ij | ng/ml | 7.3E1 | 1.2E2 | 2.1E2 | 3.9E2 | 7.5E2 | 2.3E3 | 2.8E0 | 2.1E1 | 6.4E3 | 2.4E4 | 156 | 112 | 156 | 112 | 0.64 |
| Ik | ng/ml | 1.0E1 | 1.5E1 | 2.6E3 | 2.6E2 | 1.7E4 | 4.9E2 | 5.9E-1 | 2.1E0 | 1.2E5 | 2.1E3 | 155 | 113 | 155 | 113 | 0.56 |
| Il | ng/ml | 3.4E2 | 4.4E2 | 1.3E3 | 1.5E3 | 2.9E3 | 2.9E3 | 1.0E-9 | 1.0E-9 | 1.2E4 | 1.2E4 | 154 | 112 | 154 | 112 | 0.55 |
| Im | ng/ml | 1.8E2 | 3.1E2 | 3.4E2 | 7.5E2 | 5.9E2 | 1.1E3 | 1.4E1 | 4.5E1 | 6.0E3 | 6.2E3 | 155 | 113 | 155 | 113 | 0.68 |
| In | ng/ml | 3.5E0 | 2.7E0 | 2.0E1 | 5.6E1 | 9.2E1 | 4.2E2 | 1.0E-9 | 1.0E-9 | 1.1E3 | 4.5E3 | 156 | 115 | 156 | 115 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Hb | ng/ml | 1.8E1 | 3.7E1 | 2.5E1 | 4.8E1 | 2.8E1 | 4.3E1 | 4.8E-1 | 2.6E0 | 1.4E2 | 2.0E2 | 57 | 59 | 57 | 59 | 0.71 |
| Hc | pg/ml | 7.3E2 | 6.4E2 | 4.2E3 | 2.1E3 | 1.4E4 | 6.8E3 | 1.0E-9 | 1.0E-9 | 1.0E5 | 5.0E4 | 57 | 59 | 57 | 59 | 0.45 |
| Hf | ng/ml | 1.5E2 | 2.6E2 | 3.9E2 | 4.3E2 | 5.7E2 | 5.8E2 | 1.0E-9 | 1.0E-9 | 2.5E3 | 3.2E3 | 57 | 59 | 57 | 59 | 0.56 |
| Io | ng/ml | 9.5E3 | 1.4E4 | 2.1E4 | 2.2E4 | 6.3E4 | 2.4E4 | 1.2E2 | 1.0E-9 | 7.1E5 | 1.1E5 | 155 | 116 | 155 | 116 | 0.60 |
| Ip | ng/ml | 8.7E0 | 2.7E1 | 2.0E1 | 2.7E1 | 2.7E1 | 2.6E1 | 1.0E-9 | 1.0E-9 | 2.3E2 | 1.6E2 | 155 | 116 | 155 | 116 | 0.59 |
| Iq | ug/ml | 1.1E-1 | 1.5E-1 | 6.5E-1 | 3.3E0 | 2.8E0 | 2.1E1 | 1.0E-9 | 1.0E-9 | 3.4E1 | 2.2E2 | 155 | 116 | 155 | 116 | 0.57 |
| Ir | ug/ml | 3.3E-1 | 9.4E-1 | 1.2E0 | 1.0E1 | 3.3E0 | 4.1E1 | 1.0E-9 | 1.0E-9 | 2.6E1 | 3.7E2 | 154 | 116 | 154 | 116 | 0.68 |
| Is | ng/ml | 1.5E0 | 3.8E0 | 6.0E0 | 1.7E1 | 1.2E1 | 3.5E1 | 1.0E-9 | 1.0E-9 | 8.8E1 | 2.6E2 | 155 | 116 | 155 | 116 | 0.63 |
| It | ng/ml | 1.3E0 | 3.2E0 | 1.5E1 | 2.0E1 | 7.5E1 | 8.3E1 | 1.0E-9 | 1.0E-9 | 8.3E2 | 6.8E2 | 155 | 116 | 155 | 116 | 0.63 |
| Iu | ng/ml | 1.5E2 | 2.0E2 | 9.6E2 | 1.9E3 | 3.2E3 | 5.0E3 | 1.0E-9 | 1.0E-9 | 2.9E4 | 2.4E4 | 155 | 116 | 155 | 116 | 0.57 |
| Iv | ng/ml | 1.1E1 | 2.2E1 | 3.7E1 | 1.5E2 | 9.1E1 | 6.0E2 | 1.0E-9 | 1.0E-9 | 7.7E2 | 3.8E3 | 155 | 115 | 155 | 115 | 0.62 |
| Iz | ng/ml | 1.1E2 | 1.3E2 | 4.2E2 | 2.7E2 | 9.3E2 | 3.2E2 | 1.5E0 | 4.9E0 | 6.1E3 | 1.7E3 | 57 | 59 | 57 | 59 | 0.54 |
| Yg | pg/ml | 2.4E2 | 2.8E2 | 2.3E3 | 8.0E2 | 9.5E3 | 1.0E3 | 1.0E-9 | 1.0E-9 | 5.0E4 | 3.9E3 | 27 | 21 | 27 | 21 | 0.58 |
| Yh | pg/ml | 2.1E2 | 2.6E2 | 3.3E2 | 4.7E2 | 4.4E2 | 5.8E2 | 1.0E-9 | 1.0E-9 | 1.8E3 | 2.1E3 | 27 | 21 | 27 | 21 | 0.56 |
| Yi | pg/ml | 2.2E2 | 5.1E2 | 4.4E2 | 2.3E3 | 5.4E2 | 5.9E3 | 1.0E-9 | 1.0E-9 | 2.0E3 | 2.6E4 | 27 | 21 | 27 | 21 | 0.68 |
| Yk | U/ml | 1.0E-9 | 1.0E-9 | 2.5E-1 | 1.5E-1 | 7.0E-1 | 4.0E-1 | 1.0E-9 | 1.0E-9 | 3.0E0 | 1.4E0 | 27 | 21 | 27 | 21 | 0.46 |
| Yj | pg/ml | 1.5E2 | 1.1E2 | 3.6E2 | 2.2E2 | 6.4E2 | 3.2E2 | 1.0E-9 | 5.2E1 | 3.2E3 | 1.5E3 | 27 | 21 | 27 | 21 | 0.45 |
| Yd | ng/ml | 1.6E-1 | 2.8E-1 | 3.6E-1 | 4.5E-1 | 5.0E-1 | 5.9E-1 | 6.6E-3 | 1.7E-2 | 1.8E0 | 2.3E0 | 28 | 22 | 28 | 22 | 0.59 |
| Wb | pg/ml | 2.6E4 | 4.3E4 | 3.0E4 | 7.1E4 | 1.8E4 | 1.3E5 | 6.3E3 | 1.0E4 | 8.4E4 | 6.4E5 | 28 | 22 | 28 | 22 | 0.69 |
| Vz | pg/ml | 3.7E0 | 3.9E0 | 4.6E0 | 5.4E0 | 4.0E0 | 5.7E0 | 1.0E-9 | 7.6E-2 | 1.6E1 | 2.2E1 | 28 | 22 | 28 | 22 | 0.54 |
| Si | ng/ml | 1.0E0 | 1.4E0 | 2.1E0 | 1.9E0 | 3.1E0 | 1.7E0 | 8.6E-3 | 3.3E-1 | 1.0E1 | 6.0E0 | 27 | 22 | 27 | 22 | 0.61 |
| Sf | mIU/mL | 2.0E1 | 2.0E1 | 7.4E1 | 2.3E1 | 1.5E2 | 2.0E1 | 7.8E-1 | 1.7E0 | 7.2E2 | 8.3E1 | 27 | 22 | 27 | 22 | 0.47 |
| Sh | mIU/mL | 1.8E1 | 1.2E1 | 6.4E1 | 2.1E1 | 1.2E2 | 1.8E1 | 1.4E-1 | 7.8E-2 | 5.7E2 | 6.1E1 | 27 | 22 | 27 | 22 | 0.47 |
| Sj | ng/ml | 4.4E-1 | 4.5E-1 | 4.2E-1 | 4.5E-1 | 9.2E-2 | 8.6E-2 | 2.5E-1 | 3.2E-1 | 6.1E-1 | 7.2E-1 | 27 | 22 | 27 | 22 | 0.59 |
| Rc | pg/ml | 6.5E3 | 7.4E3 | 7.5E3 | 7.6E3 | 5.3E3 | 5.0E3 | 5.5E2 | 6.7E2 | 2.2E4 | 2.7E4 | 57 | 57 | 57 | 57 | 0.53 |
| Rb | pg/ml | 7.5E-1 | 8.8E-1 | 2.7E0 | 3.3E0 | 4.1E0 | 8.0E0 | 1.0E-9 | 1.0E-9 | 1.6E1 | 5.6E1 | 57 | 57 | 57 | 57 | 0.53 |
| Zq | 2.6ng/ml | 1.8E2 | 3.6E2 | 2.3E2 | 3.8E2 | 1.5E2 | 1.9E2 | 1.7E1 | 4.8E1 | 5.2E2 | 9.7E2 | 27 | 21 | 27 | 21 | 0.72 |
| Zw | 2.5ng/ml | 5.3E0 | 6.5E0 | 9.4E0 | 1.3E1 | 1.2E1 | 1.8E1 | 2.4E-1 | 2.4E-1 | 5.9E1 | 6.3E1 | 28 | 22 | 28 | 22 | 0.52 |
| Zx | 2.3mU/ml | 1.2E-1 | 1.4E-1 | 2.9E-1 | 3.5E-1 | 5.4E-1 | 5.6E-1 | 4.2E-2 | 3.2E-2 | 2.9E0 | 2.1E0 | 28 | 22 | 28 | 22 | 0.56 |
| Pz | ng/ml | 3.2E3 | 5.7E3 | 5.8E3 | 6.1E3 | 7.2E3 | 4.4E3 | 1.6E1 | 1.9E2 | 7.0E4 | 2.5E4 | 153 | 114 | 153 | 114 | 0.57 |
| Qa | ng/ml | 2.8E3 | 6.5E3 | 5.8E3 | 1.2E4 | 7.5E3 | 2.2E4 | 1.5E2 | 4.1E2 | 4.2E4 | 2.2E5 | 153 | 114 | 153 | 114 | 0.67 |
| Qb | ng/ml | 1.0E2 | 1.5E2 | 2.0E2 | 3.1E2 | 3.1E2 | 4.5E2 | 7.9E-1 | 6.7E0 | 2.8E3 | 4.1E3 | 153 | 114 | 153 | 114 | 0.61 |
| Qc | ng/ml | 1.7E2 | 3.8E2 | 3.9E2 | 5.8E2 | 5.3E2 | 6.5E2 | 1.0E0 | 1.0E-9 | 3.8E3 | 4.3E3 | 153 | 114 | 153 | 114 | 0.61 |
| Qd | ng/ml | 7.2E3 | 1.4E4 | 2.8E4 | 3.7E4 | 1.7E5 | 6.2E4 | 2.4E2 | 1.7E3 | 2.0E6 | 4.3E5 | 153 | 114 | 153 | 114 | 0.68 |
| Qe | ng/ml | 6.5E2 | 1.8E3 | 2.0E3 | 2.6E3 | 8.0E3 | 2.7E3 | 7.6E0 | 8.8E0 | 9.7E4 | 1.8E4 | 153 | 114 | 153 | 114 | 0.70 |
| Jd | ng/ml | 4.1E-1 | 1.4E0 | 5.4E0 | 2.9E0 | 2.1E1 | 4.3E0 | 1.0E-9 | 1.0E-9 | 1.2E2 | 2.1E1 | 57 | 57 | 57 | 57 | 0.65 |
| Jc | ng/ml | 1.0E-9 | 1.0E-9 | 2.3E0 | 1.6E0 | 7.0E0 | 2.6E0 | 1.0E-9 | 1.0E-9 | 4.6E1 | 1.1E1 | 57 | 57 | 57 | 57 | 0.54 |
| Jf | ng/ml | 1.0E-9 | 1.0E-9 | 1.0E0 | 1.5E0 | 2.2E0 | 2.4E0 | 1.0E-9 | 1.0E-9 | 1.2E1 | 9.1E0 | 57 | 57 | 57 | 57 | 0.54 |
| Jg | ng/ml | 3.7E2 | 8.2E2 | 7.2E2 | 1.2E3 | 1.0E3 | 1.1E3 | 5.8E0 | 1.3E1 | 1.0E4 | 7.1E3 | 156 | 114 | 156 | 114 | 0.66 |
| Jh | ng/ml | 2.9E0 | 4.8E0 | 2.9E1 | 2.6E1 | 1.2E2 | 6.4E1 | 1.0E-9 | 1.0E-9 | 1.2E3 | 4.9E2 | 156 | 114 | 156 | 114 | 0.59 |
| Ji | ng/ml | 4.6E1 | 8.5E1 | 6.9E1 | 1.5E2 | 7.4E1 | 1.7E2 | 1.1E0 | 1.4E1 | 5.3E2 | 1.3E3 | 156 | 114 | 156 | 114 | 0.70 |
| Sr | pg/mL | 2.5E2 | 7.4E2 | 6.5E2 | 1.6E3 | 1.0E3 | 3.0E3 | 1.0E-9 | 1.0E-9 | 4.3E3 | 2.1E4 | 57 | 56 | 57 | 56 | 0.70 |
| Ss | pg/mL | 1.0E5 | 8.8E4 | 1.4E5 | 1.6E5 | 1.5E5 | 2.1E5 | 7.6E3 | 2.7E3 | 7.0E5 | 1.3E6 | 57 | 56 | 57 | 56 | 0.50 |
| St | pg/mL | 2.1E7 | 5.3E7 | 4.0E7 | 1.2E8 | 6.4E7 | 2.7E8 | 7.8E5 | 1.0E-9 | 4.1E8 | 1.7E9 | 55 | 57 | 55 | 57 | 0.69 |
| Wc | ng/ml | 1.0E-9 | 1.0E-9 | 3.5E-2 | 1.5E-1 | 7.2E-2 | 3.9E-1 | 1.0E-9 | 1.0E-9 | 3.0E-1 | 1.8E0 | 28 | 22 | 28 | 22 | 0.55 |
| Wd | ng/ml | 9.7E0 | 9.7E0 | 2.7E1 | 4.8E1 | 5.7E1 | 1.1E2 | 1.0E0 | 1.5E0 | 2.9E2 | 4.1E2 | 28 | 22 | 28 | 22 | 0.52 |
| We | ng/ml | 2.8E-1 | 4.9E-1 | 1.1E0 | 1.7E0 | 1.5E0 | 4.8E0 | 1.0E-9 | 1.0E-9 | 5.5E0 | 2.3E1 | 28 | 22 | 28 | 22 | 0.52 |
| Wg | ng/ml | 1.0E-9 | 1.0E-9 | 1.0E-9 | 2.4E-2 | 0.0E0 | 1.1E-1 | 1.0E-9 | 1.0E-9 | 1.0E-9 | 5.3E-1 | 28 | 22 | 28 | 22 | 0.52 |
| Wh | ng/ml | 1.2E-2 | 1.0E-2 | 4.2E-2 | 3.2E-2 | 8.5E-2 | 7.1E-2 | 1.0E-9 | 1.0E-9 | 3.5E-1 | 3.4E-1 | 28 | 22 | 28 | 22 | 0.49 |
| Wf | ng/ml | 1.0E-9 | 1.0E-9 | 1.2E-1 | 1.8E-1 | 2.6E-1 | 4.9E-1 | 1.0E-9 | 1.0E-9 | 1.2E0 | 2.3E0 | 28 | 22 | 28 | 22 | 0.48 |
| Ra | pg/ml | 1.0E-9 | 1.0E-9 | 4.9E-1 | 1.5E0 | 1.1E0 | 8.5E0 | 1.0E-9 | 1.0E-9 | 3.8E0 | 6.4E1 | 57 | 57 | 57 | 57 | 0.47 |
| Qz | pg/ml | 1.1E1 | 9.0E0 | 6.5E1 | 3.6E1 | 1.0E2 | 6.5E1 | 1.0E-9 | 1.0E-9 | 4.5E2 | 2.8E2 | 57 | 57 | 57 | 57 | 0.41 |
| Qy | pg/ml | 3.9E-1 | 4.6E-1 | 6.7E0 | 2.2E1 | 3.2E1 | 1.1E2 | 1.0E-9 | 1.0E-9 | 2.3E2 | 7.3E2 | 57 | 57 | 57 | 57 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qx | pg/ml | 1.0E-9 | 1.0E-9 | 4.7E0 | 3.7E0 | 2.5E1 | 1.5E1 | 1.0E-9 | 1.0E-9 | 1.9E2 | 1.1E2 | 57 | 57 | 57 | 57 | 0.50 |
| Qw | pg/ml | 6.4E-1 | 1.0E-9 | 3.0E0 | 3.1E0 | 8.6E0 | 1.2E1 | 1.0E-9 | 1.0E-9 | 5.1E1 | 6.6E1 | 57 | 57 | 57 | 57 | 0.41 |
| Qv | pg/ml | 1.9E4 | 1.2E4 | 2.8E4 | 4.4E4 | 4.9E4 | 1.3E5 | 1.4E3 | 1.0E-9 | 3.7E5 | 9.4E5 | 57 | 57 | 57 | 57 | 0.46 |
| Qu | pg/ml | 6.2E0 | 8.3E0 | 1.0E2 | 9.4E1 | 1.9E2 | 2.0E2 | 1.0E-9 | 1.0E-9 | 7.1E2 | 9.8E2 | 57 | 57 | 57 | 57 | 0.50 |
| Qt | pg/ml | 1.4E1 | 1.5E1 | 5.5E1 | 3.9E1 | 1.3E2 | 6.5E1 | 1.0E-9 | 1.0E-9 | 7.1E2 | 2.2E2 | 57 | 57 | 57 | 57 | 0.49 |
| Qh | ng/ml | 1.7E1 | 2.6E1 | 3.1E1 | 7.7E1 | 4.0E1 | 1.3E2 | 4.6E-1 | 4.3E-1 | 1.8E2 | 8.0E2 | 57 | 57 | 57 | 57 | 0.64 |
| Qg | ng/ml | 7.6E0 | 7.3E0 | 1.3E1 | 1.1E1 | 1.5E1 | 1.3E1 | 1.5E-1 | 3.0E-1 | 7.5E1 | 8.1E1 | 57 | 57 | 57 | 57 | 0.48 |
| Jj | ng/ml | 6.0E2 | 4.3E2 | 3.2E3 | 7.8E2 | 2.7E4 | 1.3E3 | 2.0E1 | 1.2E1 | 3.4E5 | 1.1E4 | 156 | 114 | 156 | 114 | 0.43 |
| Jk | ng/ml | 3.0E0 | 3.0E0 | 2.2E1 | 2.7E1 | 5.2E1 | 5.6E1 | 1.0E-9 | 2.4E-1 | 2.8E2 | 3.9E2 | 156 | 114 | 156 | 114 | 0.55 |
| Jl | ng/ml | 4.6E-1 | 6.4E-1 | 1.5E0 | 9.1E1 | 3.1E0 | 9.3E2 | 1.2E-3 | 2.0E-2 | 2.0E1 | 9.9E3 | 156 | 114 | 156 | 114 | 0.59 |
| Jm | ng/ml | 1.6E1 | 3.2E1 | 5.4E1 | 8.6E1 | 1.3E2 | 2.1E2 | 1.0E-9 | 1.9E-1 | 1.0E3 | 2.1E3 | 156 | 114 | 156 | 114 | 0.61 |
| Jn | pg/ml | 2.8E-1 | 5.5E-1 | 1.8E0 | 1.6E1 | 6.5E0 | 9.2E1 | 1.0E-9 | 1.0E-9 | 5.8E1 | 7.3E2 | 156 | 114 | 156 | 114 | 0.63 |
| Jo | pg/ml | 4.1E3 | 4.9E3 | 4.9E3 | 6.7E3 | 3.9E3 | 1.0E4 | 2.6E2 | 2.3E2 | 2.4E4 | 1.0E5 | 156 | 114 | 156 | 114 | 0.55 |
| Jp | pg/ml | 6.8E4 | 8.3E4 | 7.1E4 | 9.0E4 | 3.7E4 | 4.2E4 | 2.1E3 | 2.8E4 | 1.9E5 | 3.8E5 | 156 | 114 | 156 | 114 | 0.65 |
| Jq | pg/ml | 9.1E1 | 1.4E2 | 1.4E2 | 3.2E2 | 1.8E2 | 9.0E2 | 5.6E0 | 5.6E0 | 1.1E3 | 8.7E3 | 156 | 114 | 156 | 114 | 0.60 |
| Jr | pg/ml | 2.5E0 | 8.6E0 | 3.0E1 | 1.5E2 | 1.7E2 | 8.7E2 | 1.0E-9 | 1.0E-9 | 1.9E3 | 7.4E3 | 156 | 114 | 156 | 114 | 0.63 |
| Js | pg/ml | 1.3E1 | 1.9E1 | 4.3E1 | 1.2E2 | 1.6E2 | 4.8E2 | 1.0E-9 | 1.9E0 | 1.6E3 | 3.0E3 | 156 | 114 | 156 | 114 | 0.63 |
| Jt | pg/ml | 2.2E3 | 3.3E3 | 2.7E3 | 4.7E3 | 1.8E3 | 6.6E3 | 1.5E2 | 3.8E2 | 9.2E3 | 5.2E4 | 156 | 114 | 156 | 114 | 0.64 |
| Xa | pg/ml | 1.0E-9 | 6.9E0 | 9.1E0 | 9.1E1 | 2.0E1 | 2.8E2 | 1.0E-9 | 1.0E-9 | 9.6E1 | 1.2E3 | 28 | 22 | 28 | 22 | 0.64 |
| Ye | pg/ml | 1.0E-9 | 1.0E-9 | 7.3E0 | 1.3E0 | 1.8E1 | 3.4E0 | 1.0E-9 | 1.0E-9 | 6.9E1 | 1.4E1 | 28 | 22 | 28 | 22 | 0.40 |
| Tm | pg/ml | 1.0E-9 | 1.0E-9 | 3.5E0 | 1.9E0 | 5.1E0 | 3.6E0 | 1.0E-9 | 1.0E-9 | 1.8E1 | 8.4E0 | 28 | 22 | 28 | 22 | 0.43 |
| Tl | pg/ml | 1.2E-1 | 1.3E-1 | 3.0E-1 | 1.5E0 | 3.6E-1 | 5.2E0 | 1.0E-9 | 1.0E-9 | 1.2E0 | 2.5E1 | 28 | 22 | 28 | 22 | 0.55 |
| Ju | mIU/ml | 1.0E1 | 1.4E1 | 2.9E1 | 2.3E1 | 4.5E1 | 2.4E1 | 2.5E-1 | 1.7E-1 | 2.3E2 | 1.1E2 | 57 | 57 | 57 | 57 | 0.57 |
| Jv | mIU/ml | 1.4E1 | 1.8E1 | 5.1E1 | 3.3E1 | 8.3E1 | 3.6E1 | 2.4E-2 | 1.7E-2 | 4.4E2 | 1.8E2 | 57 | 57 | 57 | 57 | 0.54 |
| Jy | ng/ml | 1.6E-3 | 1.6E-3 | 1.9E-3 | 3.6E-3 | 1.0E-3 | 7.6E-3 | 5.3E-4 | 1.7E-4 | 4.4E-3 | 4.1E-2 | 57 | 57 | 57 | 57 | 0.51 |
| Kc | pg/ml | 2.1E1 | 2.6E1 | 2.9E1 | 5.8E1 | 3.1E1 | 6.8E1 | 1.0E-9 | 1.0E-9 | 1.4E2 | 3.2E2 | 57 | 59 | 57 | 59 | 0.63 |
| Kd | pg/ml | 1.0E-9 | 1.0E-9 | 1.8E2 | 1.1E3 | 7.3E2 | 5.1E3 | 1.0E-9 | 1.0E-9 | 4.8E3 | 3.8E4 | 57 | 59 | 57 | 59 | 0.55 |
| Ke | pg/ml | 1.1E4 | 1.5E4 | 1.2E4 | 2.6E4 | 7.8E3 | 4.3E4 | 6.7E2 | 4.2E3 | 3.1E4 | 3.2E5 | 57 | 59 | 57 | 59 | 0.66 |
| Kf | pg/mL | 5.4E0 | 8.1E0 | 5.3E0 | 1.0E1 | 4.4E0 | 1.1E1 | 1.0E-9 | 1.0E-9 | 1.8E1 | 7.8E1 | 57 | 59 | 57 | 59 | 0.67 |
| Kg | pg/mL | 7.6E2 | 1.2E3 | 1.5E3 | 2.7E3 | 1.7E3 | 5.7E3 | 7.7E1 | 1.3E2 | 8.1E3 | 3.6E4 | 57 | 59 | 57 | 59 | 0.60 |
| Ki | pg/ml | 5.9E1 | 6.1E1 | 6.7E1 | 7.4E1 | 5.5E1 | 5.7E1 | 1.0E-9 | 1.0E-9 | 2.9E2 | 2.5E2 | 57 | 59 | 57 | 59 | 0.54 |
| Kj | pg/ml | 7.0E2 | 9.3E2 | 1.3E3 | 1.6E3 | 1.5E3 | 2.3E3 | 6.6E1 | 3.3E1 | 8.8E3 | 1.5E4 | 57 | 59 | 57 | 59 | 0.56 |
| Kk | pg/ml | 6.8E0 | 1.0E1 | 9.7E0 | 1.8E1 | 1.4E1 | 1.8E1 | 1.0E-9 | 1.0E-9 | 8.1E1 | 6.1E1 | 57 | 59 | 57 | 59 | 0.66 |
| Kl | pg/ml | 1.8E4 | 1.9E4 | 2.6E4 | 3.0E4 | 2.4E4 | 2.8E4 | 2.4E2 | 2.3E2 | 1.1E5 | 1.3E5 | 57 | 59 | 57 | 59 | 0.55 |
| Kn | pg/ml | 1.5E1 | 5.7E1 | 4.1E1 | 1.8E2 | 6.3E1 | 6.4E2 | 1.0E-9 | 1.0E-9 | 3.4E2 | 4.9E3 | 57 | 59 | 57 | 59 | 0.65 |
| Ko | pg/ml | 2.2E2 | 5.0E2 | 3.6E2 | 7.2E2 | 4.2E2 | 7.9E2 | 1.0E-9 | 1.0E-9 | 1.8E3 | 4.1E3 | 57 | 59 | 57 | 59 | 0.68 |
| Kp | pg/ml | 3.0E2 | 4.0E2 | 3.1E2 | 6.5E2 | 2.4E2 | 1.7E3 | 1.0E-9 | 1.0E-9 | 8.0E2 | 1.3E4 | 57 | 59 | 57 | 59 | 0.63 |
| Kq | pg/ml | 2.8E2 | 4.2E2 | 3.3E2 | 3.6E3 | 3.2E2 | 2.1E4 | 1.6E0 | 6.1E1 | 2.1E3 | 1.6E5 | 54 | 57 | 54 | 57 | 0.70 |
| Kr | pg/ml | 1.0E-9 | 9.9E-1 | 1.4E0 | 1.0E1 | 2.4E0 | 5.5E1 | 1.0E-9 | 1.0E-9 | 1.2E1 | 4.2E2 | 54 | 57 | 54 | 57 | 0.61 |
| Ks | pg/ml | 1.2E4 | 2.0E4 | 1.9E4 | 2.4E4 | 1.9E4 | 1.8E4 | 5.1E1 | 9.9E2 | 7.9E4 | 6.3E4 | 54 | 57 | 54 | 57 | 0.60 |
| Ps | ng/ml | 1.3E2 | 3.2E2 | 2.9E2 | 1.4E3 | 4.0E2 | 3.0E3 | 5.5E0 | 1.6E1 | 1.5E3 | 1.2E4 | 27 | 22 | 27 | 22 | 0.69 |
| Kx | ng/ml | 1.0E-9 | 4.8E-3 | 4.4E-3 | 1.4E-2 | 9.9E-3 | 2.2E-2 | 1.0E-9 | 1.0E-9 | 5.1E-2 | 1.0E-1 | 56 | 59 | 56 | 59 | 0.65 |
| Ky | ng/ml | 1.1E-1 | 2.0E-1 | 3.7E-1 | 5.1E-1 | 7.6E-1 | 8.6E-1 | 1.0E-9 | 1.0E-9 | 5.1E0 | 4.4E0 | 56 | 59 | 56 | 59 | 0.57 |
| Kz | ng/ml | 1.0E-9 | 1.0E-9 | 3.7E-3 | 4.0E-3 | 5.6E-3 | 6.5E-3 | 1.0E-9 | 1.0E-9 | 1.4E-2 | 2.5E-2 | 56 | 59 | 56 | 59 | 0.49 |
| Rz | ng/ml | 3.6E-1 | 3.2E-1 | 7.2E-1 | 8.6E-1 | 9.5E-1 | 1.6E0 | 4.6E-3 | 1.7E-2 | 3.4E0 | 7.5E0 | 27 | 22 | 27 | 22 | 0.54 |
| Ry | ng/ml | 1.6E-2 | 2.4E-2 | 1.9E-2 | 4.5E-2 | 1.9E-2 | 7.5E-2 | 1.0E-9 | 1.0E-9 | 6.5E-2 | 3.5E-1 | 27 | 22 | 27 | 22 | 0.61 |
| Rx | ng/ml | 1.0E-9 | 1.8E-5 | 1.7E-3 | 1.4E-3 | 2.7E-3 | 2.3E-3 | 1.0E-9 | 1.0E-9 | 8.6E-3 | 7.6E-3 | 27 | 22 | 27 | 22 | 0.52 |
| Ld | pg/ml | 1.0E-9 | 7.5E-1 | 2.9E0 | 5.5E0 | 9.6E0 | 8.9E0 | 1.0E-9 | 1.0E-9 | 6.9E1 | 5.0E1 | 57 | 60 | 57 | 60 | 0.63 |
| Lh | pg/ml | 1.1E4 | 2.1E4 | 2.0E4 | 4.0E4 | 2.9E4 | 7.1E4 | 1.0E-9 | 1.0E3 | 2.6E5 | 4.8E5 | 156 | 115 | 156 | 115 | 0.65 |
| Li | pg/ml | 2.8E3 | 7.7E3 | 8.5E3 | 3.6E4 | 2.6E4 | 1.0E5 | 1.3E1 | 3.7E1 | 2.9E5 | 9.2E5 | 156 | 115 | 156 | 115 | 0.69 |
| Lj | pg/ml | 1.9E3 | 6.1E3 | 1.1E4 | 3.3E4 | 4.3E4 | 6.6E4 | 1.0E-9 | 3.4E1 | 4.3E5 | 4.1E5 | 156 | 115 | 156 | 115 | 0.67 |
| Lp | pg/ml | 6.4E0 | 1.5E1 | 5.6E1 | 1.6E2 | 1.3E2 | 3.8E2 | 1.0E-9 | 1.0E-9 | 5.8E2 | 1.4E3 | 27 | 22 | 27 | 22 | 0.56 |
| Lt | pg/ml | 1.0E-9 | 1.0E-9 | 1.5E0 | 2.1E0 | 5.5E0 | 6.7E0 | 1.0E-9 | 1.0E-9 | 2.5E1 | 2.5E1 | 27 | 22 | 27 | 22 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Rv | ng/ml | 5.0E-4 | 5.0E-4 | 8.8E-4 | 2.2E-3 | 8.6E-4 | 4.4E-3 | 1.0E-9 | 1.0E-9 | 2.6E-3 | 1.6E-2 | 27 | 22 | 27 | 22 | 0.46 |
| Ru | ng/ml | 1.0E-9 | 1.0E-9 | 1.9E-2 | 3.4E-2 | 7.1E-2 | 1.0E-1 | 1.0E-9 | 1.0E-9 | 3.1E-1 | 3.5E-1 | 27 | 22 | 27 | 22 | 0.53 |
| Rt | ng/ml | 6.5E-2 | 8.1E-2 | 9.0E-2 | 4.8E-1 | 9.5E-2 | 1.6E0 | 2.2E-3 | 1.3E-3 | 3.8E-1 | 7.4E0 | 27 | 22 | 27 | 22 | 0.61 |
| Yl | pg/ml | 1.1E1 | 1.3E1 | 1.4E1 | 3.1E1 | 1.3E1 | 4.7E1 | 1.0E-9 | 1.0E-9 | 4.7E1 | 2.2E2 | 28 | 22 | 28 | 22 | 0.60 |
| Rm | ng/ml | 1.5E1 | 2.0E1 | 3.7E1 | 6.7E1 | 6.4E1 | 1.1E2 | 2.2E-1 | 3.9E-1 | 3.4E2 | 6.5E2 | 57 | 56 | 57 | 56 | 0.61 |
| Rh | ng/ml | 1.4E2 | 1.8E2 | 2.9E2 | 5.9E2 | 5.1E2 | 2.3E3 | 7.5E0 | 2.5E1 | 3.8E3 | 1.7E4 | 57 | 56 | 57 | 56 | 0.52 |
| Ri | ng/ml | 2.0E-1 | 1.0E-9 | 4.1E0 | 2.3E0 | 7.9E0 | 4.8E0 | 1.0E-9 | 1.0E-9 | 4.5E1 | 2.5E1 | 57 | 56 | 57 | 56 | 0.42 |
| Rg | ng/ml | 1.0E-9 | 1.0E-9 | 4.8E-3 | 1.9E-1 | 1.7E-2 | 6.4E-1 | 1.0E-9 | 1.0E-9 | 8.4E-2 | 3.3E0 | 57 | 56 | 57 | 56 | 0.57 |
| Rj | ng/ml | 1.0E-9 | 1.0E-9 | 9.1E-1 | 5.6E0 | 1.9E0 | 3.6E1 | 1.0E-9 | 1.0E-9 | 1.1E1 | 2.7E2 | 57 | 56 | 57 | 56 | 0.47 |
| Rf | ng/ml | 3.5E-1 | 3.9E-1 | 6.7E-1 | 1.4E0 | 8.1E-1 | 3.1E0 | 2.1E-2 | 3.2E-2 | 3.6E0 | 1.7E1 | 57 | 56 | 57 | 56 | 0.56 |
| Ql | pg/ml | 1.7E0 | 5.5E0 | 9.9E0 | 1.7E1 | 1.6E1 | 3.1E1 | 1.0E-9 | 1.0E-9 | 7.5E1 | 1.8E2 | 57 | 57 | 57 | 57 | 0.56 |
| Qm | pg/ml | 4.7E-1 | 1.2E1 | 1.6E1 | 2.6E1 | 3.5E1 | 3.7E1 | 1.0E-9 | 1.0E-9 | 1.9E2 | 1.8E2 | 57 | 57 | 57 | 57 | 0.59 |
| Qn | pg/ml | 5.6E-1 | 6.1E-1 | 6.1E0 | 4.9E0 | 2.9E1 | 1.3E1 | 1.0E-9 | 1.0E-9 | 2.2E2 | 7.5E1 | 57 | 57 | 57 | 57 | 0.56 |
| Nv | pg/ml | 3.1E3 | 6.2E3 | 9.5E3 | 1.5E4 | 2.1E4 | 2.5E4 | 1.0E-9 | 2.9E2 | 1.5E5 | 1.6E5 | 156 | 116 | 156 | 116 | 0.64 |
| Nw | pg/ml | 8.3E3 | 1.4E4 | 1.2E4 | 2.1E4 | 1.9E4 | 2.9E4 | 1.9E2 | 1.3E3 | 2.1E5 | 2.2E5 | 156 | 116 | 156 | 116 | 0.69 |
| Nx | pg/ml | 1.8E2 | 2.6E2 | 3.9E2 | 6.1E2 | 6.1E2 | 7.4E2 | 1.0E-9 | 1.0E-9 | 3.5E3 | 4.1E3 | 156 | 116 | 156 | 116 | 0.62 |
| Ny | pg/ml | 4.3E0 | 1.4E1 | 1.8E2 | 7.4E1 | 2.0E3 | 2.9E2 | 1.0E-9 | 1.0E-9 | 2.5E4 | 2.8E3 | 156 | 116 | 156 | 116 | 0.67 |
| Oa | pg/ml | 1.0E2 | 3.7E2 | 2.8E2 | 7.6E2 | 5.3E2 | 9.6E2 | 1.0E-9 | 1.0E-9 | 3.0E3 | 4.5E3 | 57 | 57 | 57 | 57 | 0.70 |
| Op | pg/ml | 4.0E5 | 4.5E5 | 4.0E5 | 4.6E5 | 1.5E5 | 1.7E5 | 1.4E5 | 5.2E4 | 7.3E5 | 7.5E5 | 27 | 22 | 27 | 22 | 0.64 |
| Wn | ng/ml | 9.4E0 | 1.4E1 | 1.4E2 | 2.2E1 | 4.2E2 | 1.8E1 | 1.2E0 | 3.8E0 | 1.8E3 | 5.6E1 | 19 | 12 | 19 | 12 | 0.59 |
| Tk | ng/ml | 1.2E2 | 1.3E2 | 3.6E2 | 4.0E2 | 9.1E2 | 6.5E2 | 3.0E0 | 1.4E1 | 4.2E3 | 2.3E3 | 20 | 15 | 20 | 15 | 0.56 |
| Oe | pg/ml | 9.3E1 | 1.0E1 | 2.7E2 | 2.5E2 | 3.9E2 | 4.1E2 | 1.0E-9 | 1.0E-9 | 2.2E3 | 2.3E3 | 155 | 115 | 155 | 115 | 0.48 |
| Of | pg/ml | 2.0E2 | 1.3E2 | 6.2E3 | 6.4E3 | 2.3E4 | 2.3E4 | 1.0E-9 | 1.0E-9 | 1.9E5 | 1.7E5 | 156 | 116 | 156 | 116 | 0.47 |
| Og | pg/ml | 8.2E-2 | 5.8E-2 | 5.6E-1 | 1.4E-1 | 2.1E0 | 3.1E-1 | 1.0E-9 | 1.0E-9 | 1.9E1 | 2.5E0 | 156 | 116 | 156 | 116 | 0.41 |
| Oh | pg/ml | 2.6E0 | 4.5E0 | 2.0E1 | 1.6E2 | 1.3E2 | 1.5E3 | 1.0E-9 | 1.0E-9 | 1.4E3 | 1.6E4 | 156 | 116 | 156 | 116 | 0.58 |
| Oi | pg/ml | 2.3E0 | 2.8E0 | 5.0E0 | 5.8E0 | 7.2E0 | 7.4E0 | 1.0E-9 | 1.0E-9 | 3.6E1 | 4.2E1 | 156 | 116 | 156 | 116 | 0.55 |
| Ok | pg/ml | 3.5E2 | 6.1E2 | 4.3E2 | 8.4E2 | 3.5E2 | 9.6E2 | 1.5E1 | 6.4E1 | 2.0E3 | 7.8E3 | 156 | 116 | 156 | 116 | 0.70 |
| Om | pg/ml | 3.8E2 | 5.4E2 | 9.4E2 | 1.4E3 | 2.9E3 | 4.8E3 | 1.0E-9 | 1.0E-9 | 3.0E4 | 5.1E4 | 156 | 116 | 156 | 116 | 0.61 |
| On | pg/ml | 1.6E2 | 2.7E2 | 2.9E2 | 5.2E2 | 5.0E2 | 9.3E2 | 8.4E-1 | 2.2E1 | 4.5E3 | 8.5E3 | 156 | 116 | 156 | 116 | 0.65 |
| Or | pg/ml | 9.7E0 | 2.2E1 | 2.1E1 | 7.1E1 | 2.8E1 | 1.2E2 | 1.0E-9 | 1.0E-9 | 1.5E2 | 5.1E2 | 57 | 60 | 57 | 60 | 0.59 |
| Ow | pg/ml | 2.8E1 | 5.3E1 | 1.3E2 | 3.6E2 | 3.9E2 | 1.1E3 | 1.0E-9 | 1.0E-9 | 2.7E3 | 8.1E3 | 57 | 60 | 57 | 60 | 0.61 |
| Ou | pg/ml | 3.8E2 | 6.6E2 | 8.6E2 | 1.6E3 | 1.5E3 | 2.5E3 | 2.0E1 | 1.0E-9 | 9.8E3 | 1.1E4 | 57 | 60 | 57 | 60 | 0.62 |
| Ul | ng/ml | 1.0E-9 | 1.0E-9 | 1.2E0 | 1.8E0 | 4.5E0 | 8.1E0 | 1.0E-9 | 1.0E-9 | 2.3E1 | 5.6E1 | 58 | 57 | 58 | 57 | 0.48 |
| Uo | ng/ml | 1.0E-9 | 1.0E-9 | 9.3E-2 | 5.5E-2 | 2.4E-1 | 1.5E-1 | 1.0E-9 | 1.0E-9 | 1.3E0 | 6.9E-1 | 58 | 57 | 58 | 57 | 0.47 |
| Uk | ng/ml | 1.0E-9 | 1.0E-9 | 2.8E-3 | 1.1E-2 | 6.1E-3 | 4.7E-2 | 1.0E-9 | 1.0E-9 | 2.6E-2 | 3.2E-1 | 58 | 57 | 58 | 57 | 0.45 |
| Um | ng/ml | 1.0E-9 | 1.0E-9 | 2.6E-1 | 2.1E-1 | 6.9E-1 | 5.4E-1 | 1.0E-9 | 1.0E-9 | 3.6E0 | 2.3E0 | 58 | 57 | 58 | 57 | 0.46 |
| Uf | ng/ml | 5.2E-2 | 9.5E-2 | 8.8E-2 | 2.8E-1 | 1.0E-1 | 7.0E-1 | 1.0E-3 | 6.8E-3 | 4.3E-1 | 5.1E0 | 58 | 57 | 58 | 57 | 0.66 |
| Uh | ng/ml | 1.5E0 | 4.2E0 | 2.6E0 | 5.0E0 | 2.5E0 | 4.1E0 | 3.6E-2 | 2.5E-1 | 9.6E0 | 1.8E1 | 58 | 57 | 58 | 57 | 0.71 |
| Un | ng/ml | 1.5E0 | 2.3E0 | 1.6E0 | 3.0E0 | 9.6E-1 | 3.4E0 | 3.4E-1 | 4.6E-1 | 4.4E0 | 2.5E1 | 58 | 57 | 58 | 57 | 0.70 |
| Ug | ng/ml | 1.1E1 | 8.9E0 | 2.0E1 | 2.5E1 | 2.0E1 | 3.5E1 | 1.5E0 | 1.2E0 | 8.5E1 | 1.6E2 | 58 | 57 | 58 | 57 | 0.47 |
| Ur | ng/ml | 1.4E-1 | 6.0E-2 | 3.5E-1 | 4.5E-1 | 5.5E-1 | 1.3E0 | 1.0E-9 | 1.0E-9 | 2.6E0 | 7.3E0 | 58 | 56 | 58 | 56 | 0.39 |
| Up | ng/ml | 1.0E-9 | 1.0E-9 | 2.5E-3 | 4.7E-2 | 8.7E-3 | 3.2E-1 | 1.0E-9 | 1.0E-9 | 5.3E-2 | 2.4E0 | 58 | 56 | 58 | 56 | 0.58 |
| Us | ng/ml | 3.9E-3 | 2.6E-3 | 8.5E-3 | 5.7E-2 | 1.2E-2 | 2.3E-1 | 1.0E-9 | 1.0E-9 | 6.0E-2 | 1.7E0 | 58 | 56 | 58 | 56 | 0.52 |
| Uv | ng/ml | 3.2E-3 | 2.8E-3 | 1.6E-2 | 1.4E-2 | 4.2E-2 | 5.5E-2 | 1.0E-9 | 1.0E-9 | 2.3E-1 | 4.1E-1 | 58 | 56 | 58 | 56 | 0.45 |
| Ut | ng/ml | 5.8E-1 | 1.2E0 | 2.4E0 | 4.7E0 | 7.3E0 | 1.0E1 | 1.0E-9 | 1.0E-9 | 5.2E1 | 6.5E1 | 58 | 56 | 58 | 56 | 0.63 |
| Uu | ng/ml | 7.3E0 | 6.5E0 | 7.6E0 | 7.3E0 | 5.1E0 | 4.7E0 | 8.3E-1 | 5.7E-1 | 2.6E1 | 2.9E1 | 58 | 56 | 58 | 56 | 0.48 |
| Uw | ng/ml | 2.0E0 | 2.8E0 | 2.6E0 | 4.9E0 | 2.3E0 | 7.9E0 | 1.0E-9 | 3.7E-1 | 7.1E0 | 3.9E1 | 28 | 22 | 28 | 22 | 0.64 |
| Vb | ng/ml | 1.2E0 | 9.7E-1 | 1.1E0 | 1.2E0 | 4.4E-1 | 1.2E0 | 8.5E-2 | 2.6E-1 | 2.0E0 | 6.4E0 | 28 | 22 | 28 | 22 | 0.36 |
| Vc | ng/ml | 1.0E-9 | 1.0E-9 | 4.9E-3 | 1.0E-9 | 2.1E-2 | 0.0E0 | 1.0E-9 | 1.0E-9 | 1.1E-1 | 1.0E-9 | 28 | 22 | 28 | 22 | 0.45 |
| Uy | ng/ml | 8.2E-1 | 1.6E0 | 2.7E0 | 1.5E1 | 6.8E0 | 2.7E1 | 8.7E-2 | 2.0E-2 | 3.5E1 | 9.9E1 | 28 | 22 | 28 | 22 | 0.67 |
| Uz | ng/ml | 1.0E-9 | 1.0E-9 | 1.6E-2 | 1.5E0 | 8.2E-2 | 7.0E0 | 1.0E-9 | 1.0E-9 | 4.4E-1 | 3.3E1 | 28 | 22 | 28 | 22 | 0.53 |
| Ux | ng/ml | 2.0E2 | 1.7E2 | 1.8E2 | 2.1E2 | 1.3E2 | 1.3E2 | 4.5E0 | 2.0E1 | 4.6E2 | 4.9E2 | 28 | 22 | 28 | 22 | 0.55 |
| Va | ng/ml | 1.2E1 | 7.9E0 | 2.4E1 | 2.3E1 | 3.2E1 | 2.6E1 | 3.1E-1 | 1.2E0 | 1.2E2 | 9.6E1 | 28 | 22 | 28 | 22 | 0.53 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vh | ng/ml | 7.9E-3 | 2.0E-2 | 1.7E-2 | 6.1E-2 | 2.6E-2 | 1.8E-1 | 1.0E-3 | 3.0E-3 | 1.2E-1 | 8.6E-1 | 28 | 22 | 28 | 22 | 0.72 |
| Vi | ng/ml | 4.0E-3 | 1.0E-2 | 7.1E-3 | 1.0E-1 | 7.2E-3 | 3.9E-1 | 1.0E-9 | 1.0E-9 | 2.7E-2 | 1.8E0 | 28 | 22 | 28 | 22 | 0.60 |
| Vj | ng/ml | 3.2E1 | 5.7E1 | 6.2E1 | 9.0E1 | 7.6E1 | 1.4E2 | 1.4E0 | 8.3E0 | 3.4E2 | 6.5E2 | 28 | 21 | 28 | 21 | 0.58 |
| Vp | ng/ml | 1.0E-9 | 1.0E-9 | 1.2E-1 | 1.0E0 | 5.9E-1 | 6.5E0 | 1.0E-9 | 1.0E-9 | 3.9E0 | 4.9E1 | 58 | 57 | 58 | 57 | 0.58 |
| Vt | ng/ml | 5.0E0 | 9.0E0 | 6.0E0 | 1.4E1 | 5.0E0 | 2.1E1 | 5.6E-1 | 1.2E0 | 3.2E1 | 1.6E2 | 58 | 57 | 58 | 57 | 0.72 |
| Vu | ng/ml | 1.0E-9 | 4.5E-1 | 1.0E0 | 2.5E0 | 2.5E0 | 4.4E0 | 1.0E-9 | 1.0E-9 | 1.6E1 | 2.2E1 | 56 | 55 | 56 | 55 | 0.61 |
| Vq | ng/ml | 7.2E1 | 3.5E2 | 4.7E2 | 1.1E3 | 9.1E2 | 2.1E3 | 9.2E-1 | 3.6E0 | 5.0E3 | 1.2E4 | 50 | 41 | 50 | 41 | 0.66 |
| Vo | ng/ml | 2.5E1 | 2.6E1 | 2.4E1 | 2.5E1 | 5.2E0 | 6.0E0 | 9.7E0 | 4.9E0 | 3.5E1 | 4.8E1 | 58 | 57 | 58 | 57 | 0.54 |
| Vs | ng/ml | 1.4E-1 | 1.0E-9 | 4.9E0 | 1.2E1 | 1.4E1 | 6.2E1 | 1.0E-9 | 1.0E-9 | 8.9E1 | 4.5E2 | 58 | 54 | 58 | 54 | 0.47 |
| Vv | ng/ml | 2.6E0 | 3.7E0 | 6.0E0 | 6.2E0 | 1.2E1 | 8.4E0 | 1.0E-9 | 1.0E-9 | 8.4E1 | 4.4E1 | 58 | 56 | 58 | 56 | 0.51 |
| Vw | ng/ml | 3.7E1 | 4.2E1 | 3.2E1 | 3.9E1 | 1.8E1 | 1.9E1 | 2.5E0 | 4.4E0 | 6.7E1 | 6.9E1 | 28 | 22 | 28 | 22 | 0.62 |
| Oy | pg/ml | 4.9E-1 | 5.0E-1 | 9.5E0 | 4.0E0 | 4.4E1 | 1.2E1 | 1.0E-9 | 1.0E-9 | 4.0E2 | 9.9E1 | 156 | 115 | 156 | 115 | 0.48 |
| Oz | pg/ml | 4.9E-2 | 1.0E-9 | 2.8E-1 | 6.8E-1 | 4.0E-1 | 3.7E0 | 1.0E-9 | 1.0E-9 | 2.1E0 | 2.9E1 | 156 | 115 | 156 | 115 | 0.43 |
| Pa | pg/ml | 3.7E-1 | 5.4E-1 | 1.6E0 | 4.4E0 | 7.7E0 | 2.3E1 | 1.0E-9 | 1.0E-9 | 8.6E1 | 2.3E2 | 156 | 115 | 156 | 115 | 0.59 |
| Pb | pg/ml | 2.9E-3 | 1.0E-9 | 3.2E0 | 6.9E-1 | 3.9E1 | 4.0E0 | 1.0E-9 | 1.0E-9 | 4.9E2 | 3.2E1 | 156 | 115 | 156 | 115 | 0.44 |
| Pc | pg/ml | 1.7E-1 | 1.0E-9 | 4.9E-1 | 3.5E0 | 1.2E0 | 3.1E1 | 1.0E-9 | 1.0E-9 | 1.4E1 | 3.3E2 | 156 | 115 | 156 | 115 | 0.45 |
| Pd | pg/ml | 1.6E0 | 2.4E0 | 3.7E0 | 1.4E1 | 8.9E0 | 7.9E1 | 1.0E-9 | 1.0E-9 | 9.4E1 | 8.4E2 | 156 | 115 | 156 | 115 | 0.58 |
| Pe | pg/ml | 1.9E1 | 4.4E1 | 9.2E1 | 4.1E2 | 4.4E2 | 1.7E3 | 1.0E-9 | 1.0E-9 | 4.7E3 | 1.5E4 | 156 | 115 | 156 | 115 | 0.66 |
| Pf | pg/ml | 1.2E0 | 3.1E0 | 8.6E0 | 3.0E1 | 3.4E1 | 1.5E2 | 1.0E-9 | 1.0E-9 | 3.3E2 | 1.5E3 | 156 | 115 | 156 | 115 | 0.63 |
| Pg | pg/ml | 3.8E0 | 7.3E0 | 7.2E1 | 1.5E2 | 4.3E2 | 7.7E2 | 1.0E-9 | 1.0E-9 | 4.2E3 | 7.7E3 | 156 | 115 | 156 | 115 | 0.60 |
| Ph | ng/ml | 1.3E-1 | 2.2E-1 | 2.7E-1 | 5.1E-1 | 4.0E-1 | 8.7E-1 | 1.0E-9 | 1.0E-9 | 2.2E0 | 5.4E0 | 57 | 60 | 57 | 60 | 0.60 |
| Pi | ng/ml | 1.8E-1 | 2.2E-1 | 2.4E-1 | 1.8E0 | 4.3E-1 | 1.1E1 | 1.0E-9 | 1.0E-9 | 3.2E0 | 8.2E1 | 57 | 60 | 57 | 60 | 0.62 |
| Pj | ng/mL | 3.8E0 | 7.3E0 | 4.6E0 | 8.0E0 | 3.3E0 | 5.4E0 | 4.0E-1 | 1.5E0 | 1.6E1 | 3.1E1 | 57 | 60 | 57 | 60 | 0.72 |
| Pk | ng/ml | 7.1E-3 | 1.1E-2 | 8.2E-3 | 4.6E-2 | 7.9E-3 | 2.0E-1 | 1.0E-9 | 1.0E-9 | 3.9E-2 | 1.5E0 | 57 | 60 | 57 | 60 | 0.67 |
| aA | mg/dL | 8.6E-1 | 9.6E-1 | 9.9E-1 | 1.1E0 | 4.9E-1 | 7.3E-1 | 3.0E-1 | 3.0E-1 | 4.2E0 | 4.7E0 | 265 | 142 | 265 | 142 | 0.55 |
| aC | mg/mL | 2.4E0 | 2.1E0 | 2.7E0 | 2.2E0 | 1.3E0 | 1.0E0 | 1.1E0 | 7.5E-1 | 7.2E0 | 5.5E0 | 75 | 68 | 75 | 68 | 0.38 |
| aD | ug/mL | 2.8E0 | 3.6E0 | 4.3E0 | 4.9E0 | 4.2E0 | 4.0E0 | 8.5E-1 | 7.5E-1 | 3.1E1 | 2.1E1 | 75 | 68 | 75 | 68 | 0.54 |
| aE | mg/mL | 5.6E-1 | 5.9E-1 | 5.9E-1 | 6.0E-1 | 1.6E-1 | 1.9E-1 | 2.8E-1 | 1.8E-1 | 1.1E0 | 1.2E0 | 75 | 68 | 75 | 68 | 0.52 |
| aF | ng/mL | 2.2E0 | 2.2E0 | 6.2E0 | 4.0E0 | 9.9E0 | 4.9E0 | 4.3E-3 | 4.3E-3 | 5.0E1 | 2.9E1 | 75 | 68 | 75 | 68 | 0.47 |
| aG | mg/mL | 1.3E-1 | 1.4E-1 | 1.6E-1 | 1.6E-1 | 8.1E-2 | 8.1E-2 | 5.0E-2 | 5.1E-2 | 4.2E-1 | 4.8E-1 | 75 | 68 | 75 | 68 | 0.49 |
| aH | ug/mL | 6.4E1 | 7.2E1 | 7.3E1 | 7.8E1 | 3.5E1 | 4.2E1 | 1.5E1 | 1.1E1 | 2.0E2 | 1.8E2 | 75 | 68 | 75 | 68 | 0.51 |
| aI | ug/mL | 1.8E2 | 1.6E2 | 1.7E2 | 1.7E2 | 5.7E1 | 6.4E1 | 6.1E1 | 4.7E1 | 3.3E2 | 3.4E2 | 75 | 68 | 75 | 68 | 0.46 |
| aJ | ug/mL | 2.2E0 | 2.5E0 | 3.1E0 | 3.4E0 | 2.4E0 | 2.3E0 | 9.5E-1 | 8.2E-1 | 1.4E1 | 1.1E1 | 75 | 68 | 75 | 68 | 0.57 |
| aK | ng/mL | 1.6E0 | 1.1E0 | 2.2E0 | 1.8E0 | 1.9E0 | 1.9E0 | 6.9E-2 | 2.9E-4 | 7.5E0 | 1.0E1 | 75 | 68 | 75 | 68 | 0.41 |
| aL | mg/mL | 7.1E-1 | 7.4E-1 | 7.4E-1 | 7.6E-1 | 2.1E-1 | 2.7E-1 | 2.2E-1 | 2.7E-1 | 1.3E0 | 1.7E0 | 75 | 68 | 75 | 68 | 0.52 |
| aM | U/mL | 1.3E1 | 2.3E1 | 2.7E1 | 5.7E1 | 5.0E1 | 1.1E2 | 4.2E-2 | 4.2E-2 | 3.5E2 | 8.2E2 | 75 | 68 | 75 | 68 | 0.65 |
| aN | U/mL | 1.1E1 | 2.0E1 | 1.9E1 | 3.7E1 | 2.3E1 | 6.1E1 | 2.5E-3 | 2.5E-3 | 1.3E2 | 3.8E2 | 75 | 68 | 75 | 68 | 0.65 |
| aO | pg/mL | 7.4E1 | 4.8E1 | 5.5E2 | 3.3E2 | 1.3E3 | 6.1E2 | 6.0E-2 | 6.0E-2 | 6.6E3 | 2.4E3 | 75 | 68 | 75 | 68 | 0.50 |
| aP | ng/mL | 1.5E0 | 1.7E0 | 2.0E0 | 2.2E0 | 1.3E0 | 1.3E0 | 5.4E-1 | 6.8E-1 | 6.5E0 | 6.6E0 | 75 | 68 | 75 | 68 | 0.58 |
| aQ | ng/mL | 2.3E-1 | 2.5E-1 | 3.2E-1 | 3.6E-1 | 2.6E-1 | 3.8E-1 | 2.0E-4 | 2.0E-4 | 1.1E0 | 2.0E0 | 75 | 68 | 75 | 68 | 0.51 |
| aR | ng/mL | 1.8E0 | 1.9E0 | 3.0E0 | 3.2E0 | 3.8E0 | 4.5E0 | 2.6E-1 | 5.6E-1 | 2.1E1 | 3.4E1 | 75 | 68 | 75 | 68 | 0.55 |
| aS | ng/mL | 3.7E-1 | 4.6E-1 | 1.3E0 | 9.2E-1 | 3.9E0 | 1.1E0 | 4.2E-3 | 4.2E-3 | 3.3E1 | 6.2E0 | 75 | 68 | 75 | 68 | 0.53 |
| aU | pg/mL | 7.1E1 | 6.1E1 | 1.0E2 | 9.7E1 | 1.0E2 | 1.2E2 | 7.4E0 | 7.4E-2 | 5.1E2 | 7.0E2 | 75 | 68 | 75 | 68 | 0.45 |
| aV | ng/mL | 6.3E-1 | 4.4E-1 | 8.6E-1 | 1.3E0 | 7.4E-1 | 4.0E0 | 5.9E-2 | 7.6E-4 | 4.0E0 | 3.3E1 | 75 | 68 | 75 | 68 | 0.44 |
| aW | pg/mL | 2.0E1 | 2.0E1 | 2.2E1 | 2.5E1 | 2.0E1 | 4.9E1 | 7.2E-2 | 7.2E-2 | 1.7E2 | 4.2E2 | 75 | 68 | 75 | 68 | 0.48 |
| aX | ng/mL | 8.0E0 | 7.0E0 | 1.1E1 | 1.2E1 | 9.6E0 | 1.6E1 | 3.0E-1 | 6.2E-1 | 5.4E1 | 1.1E2 | 75 | 68 | 75 | 68 | 0.46 |
| aY | pg/mL | 5.3E1 | 5.4E1 | 6.9E1 | 8.3E1 | 5.9E1 | 1.5E2 | 4.1E-1 | 4.1E-1 | 3.1E2 | 1.2E3 | 75 | 68 | 75 | 68 | 0.50 |
| aZ | pg/mL | 2.2E2 | 2.4E2 | 5.5E2 | 7.2E2 | 9.0E2 | 1.6E3 | 1.7E0 | 1.7E0 | 5.4E3 | 1.2E4 | 75 | 68 | 75 | 68 | 0.50 |
| bA | ng/mL | 1.3E1 | 2.2E1 | 4.2E1 | 1.0E2 | 6.8E1 | 2.0E2 | 3.0E-2 | 3.0E-2 | 4.1E2 | 9.4E2 | 75 | 68 | 75 | 68 | 0.59 |
| bB | ng/mL | 2.8E2 | 2.5E2 | 3.0E2 | 2.7E2 | 1.5E2 | 1.7E2 | 5.7E1 | 1.2E1 | 7.4E2 | 8.1E2 | 75 | 68 | 75 | 68 | 0.43 |
| bC | ng/mL | 3.2E2 | 3.2E2 | 4.9E2 | 7.3E2 | 5.0E2 | 1.0E3 | 2.7E1 | 3.5E1 | 3.0E3 | 4.7E3 | 75 | 68 | 75 | 68 | 0.52 |
| bE | mg/mL | 5.2E0 | 4.6E0 | 5.4E0 | 5.1E0 | 1.7E0 | 2.2E0 | 2.5E0 | 1.3E0 | 9.5E0 | 1.2E1 | 75 | 68 | 75 | 68 | 0.43 |
| bF | pg/mL | 3.7E1 | 3.8E1 | 3.6E2 | 4.5E2 | 1.5E3 | 1.5E3 | 5.0E-2 | 2.5E0 | 1.1E4 | 1.0E4 | 75 | 68 | 75 | 68 | 0.51 |

| Code | Units | Median | | Average | | Standard Deviation | | Minimum | | Maximum | | Number of Samples | | Number of Patients | | AUC |
|------|-------|--------|----|---------|----|-------|------|---------|------|---------|------|--------|-----|--------|-----|-----|
| | | NonDis | Dis | NonDis | Dis | NonDis | Dis | NonDis | Dis | NonDis | Dis | NonDis | Dis | NonDis | Dis | |
| bG | ng/mL | 1.7E0 | 2.2E0 | 3.2E0 | 3.6E0 | 4.7E0 | 4.8E0 | 1.1E-1 | 1.1E-1 | 2.6E1 | 3.0E1 | 75 | 68 | 75 | 68 | 0.51 |
| bH | pg/mL | 5.7E-1 | 5.7E-1 | 7.6E0 | 6.2E0 | 3.2E1 | 1.6E1 | 5.7E-1 | 5.7E-1 | 2.8E2 | 1.2E2 | 75 | 68 | 75 | 68 | 0.50 |
| bI | ng/mL | 4.0E-3 | 4.0E-3 | 1.0E-1 | 9.2E-2 | 2.1E-1 | 2.1E-1 | 4.0E-3 | 4.0E-3 | 9.8E-1 | 8.8E-1 | 75 | 68 | 75 | 68 | 0.46 |
| bJ | mg/mL | 1.7E0 | 1.8E0 | 2.1E0 | 2.2E0 | 1.6E0 | 2.1E0 | 2.5E-4 | 2.5E-4 | 6.6E0 | 1.1E1 | 75 | 68 | 75 | 68 | 0.49 |
| bL | pg/mL | 4.3E0 | 3.0E0 | 9.4E0 | 8.3E0 | 1.1E1 | 1.0E1 | 4.6E-2 | 4.6E-2 | 4.9E1 | 3.5E1 | 75 | 68 | 75 | 68 | 0.45 |
| bM | mg/mL | 1.6E0 | 2.2E0 | 1.9E0 | 2.6E0 | 1.3E0 | 1.6E0 | 2.4E-1 | 1.8E-2 | 8.6E0 | 7.9E0 | 75 | 68 | 75 | 68 | 0.63 |
| bN | ng/mL | 3.7E1 | 3.5E1 | 1.3E2 | 1.1E2 | 2.7E2 | 2.8E2 | 1.4E-1 | 5.9E-1 | 1.9E3 | 1.9E3 | 75 | 68 | 75 | 68 | 0.48 |
| bO | ng/mL | 4.0E-2 | 4.0E-2 | 8.1E0 | 9.3E0 | 1.5E1 | 2.2E1 | 4.0E-2 | 4.0E-2 | 6.4E1 | 1.3E2 | 75 | 68 | 75 | 68 | 0.45 |
| bP | mg/mL | 4.9E-1 | 5.1E-1 | 7.0E-1 | 7.1E-1 | 5.5E-1 | 7.7E-1 | 8.2E-2 | 9.2E-2 | 2.5E0 | 4.8E0 | 75 | 68 | 75 | 68 | 0.48 |
| bQ | pg/mL | 2.1E1 | 2.3E1 | 4.2E1 | 2.7E2 | 5.6E1 | 1.6E3 | 3.5E0 | 1.5E-1 | 3.2E2 | 1.3E4 | 75 | 68 | 75 | 68 | 0.51 |
| bR | ng/mL | 1.2E-2 | 1.2E-2 | 1.3E-1 | 2.0E-1 | 4.0E-1 | 1.0E0 | 1.2E-2 | 1.2E-2 | 3.4E0 | 8.7E0 | 75 | 68 | 75 | 68 | 0.50 |
| bS | pg/mL | 9.4E-1 | 9.4E-1 | 9.1E0 | 1.1E1 | 4.1E1 | 4.9E1 | 9.4E-1 | 9.4E-1 | 3.4E2 | 3.9E2 | 75 | 68 | 75 | 68 | 0.49 |
| bU | ng/mL | 8.7E-2 | 1.3E-2 | 1.7E-1 | 2.2E-1 | 2.6E-1 | 8.0E-1 | 1.3E-2 | 1.3E-2 | 1.8E0 | 6.6E0 | 75 | 68 | 75 | 68 | 0.46 |
| bV | pg/mL | 4.3E2 | 5.2E2 | 5.4E2 | 6.0E2 | 2.9E2 | 3.7E2 | 1.8E2 | 2.3E2 | 1.6E3 | 2.2E3 | 75 | 68 | 75 | 68 | 0.56 |
| bW | pg/mL | 3.1E2 | 3.3E2 | 4.2E2 | 5.5E2 | 3.6E2 | 7.8E2 | 8.4E1 | 1.3E2 | 2.2E3 | 4.8E3 | 75 | 68 | 75 | 68 | 0.55 |
| bX | ng/mL | 1.8E-3 | 2.5E-5 | 2.7E-3 | 2.2E-3 | 3.0E-3 | 2.8E-3 | 2.5E-5 | 2.5E-5 | 1.2E-2 | 8.4E-3 | 75 | 68 | 75 | 68 | 0.45 |
| bZ | pg/mL | 2.9E2 | 2.9E2 | 1.5E3 | 2.7E3 | 5.4E3 | 9.3E3 | 1.5E-1 | 3.5E1 | 4.4E4 | 5.8E4 | 75 | 68 | 75 | 68 | 0.51 |
| cA | pg/mL | 6.0E-1 | 6.0E-1 | 1.5E0 | 7.5E0 | 2.8E0 | 4.5E1 | 6.0E-1 | 6.0E-1 | 1.2E1 | 3.7E2 | 75 | 68 | 75 | 68 | 0.52 |
| cB | ng/mL | 4.2E-2 | 4.1E-2 | 6.1E-2 | 7.3E-2 | 6.2E-2 | 9.8E-2 | 1.7E-3 | 1.7E-3 | 3.1E-1 | 4.3E-1 | 75 | 68 | 75 | 68 | 0.48 |
| cC | pg/mL | 4.6E1 | 4.0E1 | 4.9E1 | 4.4E1 | 4.9E1 | 5.7E1 | 1.0E0 | 1.0E0 | 3.7E2 | 4.5E2 | 75 | 68 | 75 | 68 | 0.45 |
| cD | pg/mL | 6.3E0 | 3.9E0 | 1.6E1 | 1.2E1 | 5.9E1 | 4.0E1 | 3.3E-1 | 3.3E-1 | 4.8E2 | 2.9E2 | 75 | 68 | 75 | 68 | 0.37 |
| cE | pg/mL | 6.5E1 | 6.7E1 | 2.7E2 | 2.8E2 | 6.5E2 | 6.1E2 | 1.8E0 | 1.2E-1 | 3.1E3 | 3.8E3 | 75 | 68 | 75 | 68 | 0.54 |
| cF | pg/mL | 5.3E-1 | 5.3E-1 | 1.6E1 | 1.5E1 | 2.8E1 | 3.5E1 | 5.3E-1 | 5.3E-1 | 1.4E2 | 2.7E2 | 75 | 68 | 75 | 68 | 0.49 |
| cG | pg/mL | 5.2E1 | 6.3E1 | 9.8E1 | 2.7E2 | 1.7E2 | 1.3E3 | 1.5E1 | 7.8E0 | 1.1E3 | 1.0E4 | 75 | 68 | 75 | 68 | 0.56 |
| cH | uIU/mL | 3.4E0 | 2.8E0 | 8.1E0 | 8.0E0 | 2.0E1 | 1.7E1 | 8.6E-3 | 8.6E-3 | 1.6E2 | 1.2E2 | 75 | 68 | 75 | 68 | 0.47 |
| cI | ng/mL | 5.9E0 | 6.0E0 | 9.8E0 | 1.8E1 | 1.2E1 | 2.7E1 | 2.3E-1 | 3.2E-2 | 5.9E1 | 1.2E2 | 75 | 68 | 75 | 68 | 0.54 |
| cJ | ug/mL | 6.7E1 | 5.1E1 | 9.6E1 | 9.2E1 | 1.0E2 | 1.1E2 | 6.9E0 | 7.2E0 | 6.4E2 | 6.2E2 | 75 | 68 | 75 | 68 | 0.47 |
| cK | ng/mL | 3.8E-3 | 3.8E-3 | 1.2E-2 | 3.2E-2 | 2.7E-2 | 1.8E-1 | 3.8E-3 | 3.8E-3 | 1.3E-1 | 1.5E0 | 75 | 68 | 75 | 68 | 0.50 |
| cL | pg/mL | 2.2E2 | 2.0E2 | 5.0E2 | 6.8E2 | 1.2E3 | 2.9E3 | 5.0E1 | 3.1E1 | 7.4E3 | 2.4E4 | 75 | 68 | 75 | 68 | 0.50 |
| cM | pg/mL | 2.7E2 | 2.6E2 | 3.2E2 | 2.6E2 | 1.9E2 | 1.3E2 | 6.0E1 | 4.2E1 | 1.1E3 | 6.7E2 | 75 | 68 | 75 | 68 | 0.43 |
| cN | pg/mL | 1.2E2 | 1.2E2 | 1.3E2 | 1.4E2 | 5.3E1 | 1.2E2 | 3.8E1 | 6.3E1 | 3.2E2 | 1.1E3 | 75 | 68 | 75 | 68 | 0.53 |
| cO | pg/mL | 2.0E2 | 2.1E2 | 3.2E2 | 5.7E2 | 3.2E2 | 2.3E3 | 5.4E1 | 8.2E1 | 1.7E3 | 1.9E4 | 75 | 68 | 75 | 68 | 0.51 |
| cP | ng/mL | 2.4E3 | 2.4E3 | 2.6E3 | 2.6E3 | 1.1E3 | 9.0E2 | 6.2E2 | 1.0E3 | 5.6E3 | 4.7E3 | 75 | 68 | 75 | 68 | 0.50 |
| cQ | ng/mL | 4.9E-2 | 5.3E-2 | 1.2E-1 | 1.3E-1 | 2.0E-1 | 2.3E-1 | 2.0E-3 | 2.0E-3 | 1.2E0 | 1.3E0 | 75 | 68 | 75 | 68 | 0.49 |
| cR | ng/mL | 3.0E2 | 3.1E2 | 4.4E2 | 7.1E2 | 3.5E2 | 1.2E3 | 3.6E1 | 2.0E1 | 1.4E3 | 7.7E3 | 75 | 68 | 75 | 68 | 0.53 |
| cS | ng/mL | 2.4E2 | 2.8E2 | 4.0E2 | 4.0E2 | 4.5E2 | 3.8E2 | 8.0E1 | 9.1E1 | 2.5E3 | 2.4E3 | 75 | 68 | 75 | 68 | 0.54 |
| cT | ng/mL | 5.3E1 | 6.2E1 | 1.2E2 | 2.2E2 | 1.7E2 | 4.1E2 | 5.1E0 | 4.2E0 | 8.4E2 | 2.1E3 | 75 | 68 | 75 | 68 | 0.55 |
| cU | ng/mL | 5.7E1 | 6.6E1 | 8.2E1 | 1.2E2 | 9.4E1 | 2.1E2 | 6.2E0 | 9.0E0 | 7.7E2 | 1.6E3 | 75 | 68 | 75 | 68 | 0.56 |
| cV | ng/mL | 1.9E-1 | 2.2E-1 | 9.9E-1 | 6.7E-1 | 5.4E0 | 1.6E0 | 3.4E-2 | 3.0E-2 | 4.7E1 | 9.7E0 | 75 | 68 | 75 | 68 | 0.51 |
| cW | mIU/mL | 4.8E-2 | 4.5E-2 | 1.1E-1 | 6.7E-2 | 5.1E-1 | 6.2E-2 | 4.8E-3 | 4.8E-3 | 4.5E0 | 3.9E-1 | 75 | 68 | 75 | 68 | 0.51 |
| cX | ng/mL | 1.1E-1 | 1.6E-1 | 2.1E0 | 2.1E0 | 6.3E0 | 6.0E0 | 2.3E-4 | 2.3E-4 | 2.8E1 | 2.8E1 | 75 | 68 | 75 | 68 | 0.53 |
| cY | ng/mL | 7.6E0 | 7.1E0 | 1.1E1 | 1.1E1 | 9.4E0 | 1.3E1 | 2.2E-1 | 1.7E-1 | 4.1E1 | 6.1E1 | 75 | 68 | 75 | 68 | 0.47 |
| cZ | ug/mL | 1.2E1 | 1.2E1 | 1.4E1 | 1.4E1 | 5.2E0 | 6.7E0 | 6.0E0 | 2.8E0 | 2.9E1 | 3.0E1 | 75 | 68 | 75 | 68 | 0.49 |
| dA | pg/mL | 3.0E2 | 3.2E2 | 3.3E2 | 4.4E2 | 1.3E2 | 6.9E2 | 1.0E2 | 1.1E2 | 9.4E2 | 5.8E3 | 75 | 68 | 75 | 68 | 0.53 |
| dB | ug/mL | 1.9E1 | 2.0E1 | 2.1E1 | 1.7E1 | 2.8E1 | 1.0E1 | 2.1E0 | 2.1E0 | 2.5E2 | 4.0E1 | 75 | 68 | 75 | 68 | 0.48 |
| dC | nmol/L | 3.4E1 | 3.6E1 | 3.9E1 | 3.7E1 | 2.0E1 | 1.4E1 | 1.0E1 | 7.8E0 | 1.4E2 | 7.0E1 | 75 | 68 | 75 | 68 | 0.52 |
| dD | ug/mL | 3.4E1 | 3.2E1 | 3.6E1 | 3.3E1 | 1.0E1 | 1.1E1 | 1.4E1 | 1.4E1 | 7.4E1 | 6.0E1 | 75 | 68 | 75 | 68 | 0.42 |
| dE | ng/mL | 5.0E-1 | 3.8E-1 | 5.7E-1 | 5.0E-1 | 5.7E-1 | 5.4E-1 | 8.4E-3 | 8.4E-3 | 2.7E0 | 2.4E0 | 75 | 68 | 75 | 68 | 0.46 |
| dF | ng/mL | 2.4E2 | 2.8E2 | 3.1E2 | 4.0E2 | 2.1E2 | 2.9E2 | 7.5E1 | 7.5E1 | 1.2E3 | 1.3E3 | 75 | 68 | 75 | 68 | 0.59 |
| dG | ng/mL | 1.1E1 | 1.3E1 | 1.6E1 | 1.9E1 | 1.4E1 | 2.4E1 | 3.2E0 | 3.0E0 | 9.7E1 | 1.8E2 | 75 | 68 | 75 | 68 | 0.55 |
| dH | pg/mL | 7.1E0 | 8.8E0 | 1.6E1 | 2.4E1 | 3.8E1 | 8.3E1 | 4.0E-2 | 4.0E-2 | 3.1E2 | 6.7E2 | 75 | 68 | 75 | 68 | 0.59 |
| dI | pg/mL | 4.6E-1 | 4.6E-1 | 1.4E0 | 6.7E0 | 4.1E0 | 4.0E1 | 4.6E-1 | 4.6E-1 | 3.4E1 | 3.3E2 | 75 | 68 | 75 | 68 | 0.58 |
| dJ | ng/mL | 1.9E0 | 2.2E0 | 2.1E0 | 2.2E0 | 1.0E0 | 1.2E0 | 3.2E-2 | 3.2E-2 | 5.1E0 | 4.9E0 | 75 | 68 | 75 | 68 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dK | uIU/mL | 1.5E0 | 1.2E0 | 2.6E0 | 1.9E0 | 5.0E0 | 2.0E0 | 2.8E-4 | 2.9E-2 | 3.9E1 | 1.1E1 | 75 | 68 | 75 | 68 | 0.46 |
| dL | ng/mL | 8.2E2 | 9.3E2 | 9.5E2 | 1.2E3 | 5.0E2 | 7.5E2 | 3.4E2 | 2.8E2 | 3.4E3 | 4.8E3 | 75 | 68 | 75 | 68 | 0.59 |
| dM | pg/mL | 9.6E2 | 9.9E2 | 1.3E3 | 1.4E3 | 1.8E3 | 1.4E3 | 3.9E2 | 3.7E2 | 1.5E4 | 9.6E3 | 75 | 68 | 75 | 68 | 0.53 |
| dN | ug/mL | 9.6E1 | 1.0E2 | 1.0E2 | 1.1E2 | 3.7E1 | 4.7E1 | 2.5E1 | 2.4E1 | 2.2E2 | 3.3E2 | 75 | 68 | 75 | 68 | 0.55 |
| dR | pg/ml | 1.7E3 | 1.2E3 | 2.1E3 | 2.0E3 | 1.8E3 | 2.3E3 | 2.8E2 | 1.3E2 | 7.8E3 | 9.8E3 | 53 | 60 | 53 | 60 | 0.41 |
| dU | pg/ml | 7.4E3 | 1.8E4 | 9.1E3 | 2.2E4 | 8.2E3 | 2.1E4 | 1.7E3 | 6.9E2 | 3.5E4 | 8.1E4 | 14 | 15 | 14 | 15 | 0.70 |
| dX | ng/ml | 4.3E-2 | 8.2E-2 | 1.4E-1 | 1.3E-1 | 2.5E-1 | 1.6E-1 | 2.6E-3 | 2.6E-3 | 1.2E0 | 6.6E-1 | 24 | 21 | 24 | 21 | 0.55 |
| dW | ng/ml | 9.9E-2 | 2.1E-1 | 1.7E-1 | 2.9E-1 | 1.8E-1 | 2.5E-1 | 6.4E-2 | 6.8E-2 | 5.8E-1 | 8.0E-1 | 8 | 9 | 8 | 9 | 0.72 |
| eF | ng/ml | 4.4E0 | 4.0E0 | 5.1E0 | 4.7E0 | 2.4E0 | 2.2E0 | 2.0E0 | 2.0E0 | 1.2E1 | 1.5E1 | 53 | 60 | 53 | 60 | 0.45 |
| eC | pg/ml | 3.1E2 | 2.8E2 | 3.9E2 | 3.5E2 | 2.6E2 | 3.3E2 | 1.1E2 | 1.9E1 | 1.4E3 | 2.0E3 | 47 | 55 | 47 | 55 | 0.41 |
| eD | pg/ml | 2.3E2 | 1.8E2 | 8.5E2 | 5.1E2 | 1.6E3 | 1.2E3 | 5.2E-1 | 5.2E-1 | 6.8E3 | 7.0E3 | 40 | 43 | 40 | 43 | 0.42 |
| eO | ng/ml | 4.5E1 | 9.8E1 | 2.4E2 | 1.3E2 | 3.7E2 | 8.7E1 | 2.0E1 | 4.3E1 | 9.6E2 | 3.3E2 | 8 | 9 | 8 | 9 | 0.72 |
| eM | ng/ml | 3.0E0 | 2.7E0 | 3.8E0 | 5.3E0 | 2.8E0 | 6.6E0 | 7.6E-1 | 6.9E-1 | 1.2E1 | 2.6E1 | 34 | 28 | 34 | 28 | 0.50 |
| eP | ng/ml | 1.7E-2 | 3.7E-3 | 8.6E-1 | 1.6E0 | 1.9E0 | 6.0E0 | 3.7E-3 | 3.7E-3 | 8.6E0 | 2.8E1 | 24 | 21 | 24 | 21 | 0.42 |
| cT | ng/ml | 2.8E2 | 2.8E2 | 7.4E2 | 7.3E2 | 8.1E2 | 9.2E2 | 1.0E2 | 7.1E1 | 2.9E3 | 2.9E3 | 26 | 23 | 26 | 23 | 0.48 |
| eQ | pg/ml | 1.0E0 | 1.0E0 | 7.3E1 | 3.2E1 | 1.5E2 | 5.4E1 | 1.0E0 | 1.0E0 | 4.7E2 | 1.5E2 | 14 | 15 | 14 | 15 | 0.50 |
| eW | U/ml | 8.8E-3 | 6.7E-3 | 6.1E-2 | 2.2E-1 | 1.0E-1 | 5.1E-1 | 6.7E-3 | 6.7E-3 | 3.1E-1 | 1.6E0 | 8 | 9 | 8 | 9 | 0.47 |
| fA | ng/ml | 2.3E2 | 1.6E2 | 3.6E2 | 5.0E2 | 4.4E2 | 5.2E2 | 3.9E1 | 4.0E1 | 1.5E3 | 1.4E3 | 14 | 13 | 14 | 13 | 0.51 |
| eZ | ng/ml | 4.5E1 | 5.6E1 | 5.7E1 | 5.8E1 | 2.8E1 | 2.4E1 | 2.3E1 | 1.8E1 | 1.2E2 | 1.1E2 | 26 | 23 | 26 | 23 | 0.54 |
| fB | ng/ml | 5.6E2 | 6.6E2 | 6.6E2 | 7.0E2 | 2.7E2 | 2.8E2 | 3.1E2 | 2.6E2 | 1.3E3 | 1.3E3 | 14 | 14 | 14 | 14 | 0.57 |
| fN | ng/ml | 2.1E-1 | 2.1E-1 | 2.0E0 | 3.5E0 | 3.9E0 | 5.8E0 | 2.1E-1 | 2.1E-1 | 1.4E1 | 2.1E1 | 26 | 23 | 26 | 23 | 0.54 |
| fP | ng/ml | 2.8E2 | 2.7E2 | 3.5E2 | 3.2E2 | 2.3E2 | 1.5E2 | 1.8E0 | 3.7E1 | 1.0E3 | 7.7E2 | 52 | 58 | 52 | 58 | 0.50 |
| fR | ng/ml | 1.5E5 | 1.7E5 | 1.9E5 | 2.4E5 | 1.5E5 | 1.9E5 | 3.9E4 | 1.9E2 | 6.3E5 | 6.8E5 | 50 | 42 | 50 | 42 | 0.58 |
| fY | ng/ml | 2.5E2 | 2.6E2 | 2.4E2 | 2.5E2 | 1.0E2 | 1.1E2 | 6.5E1 | 3.6E1 | 4.2E2 | 4.8E2 | 26 | 23 | 26 | 23 | 0.53 |
| gC | ng/ml | 2.4E2 | 2.2E2 | 2.6E2 | 2.5E2 | 1.3E2 | 1.0E2 | 9.7E1 | 8.3E1 | 6.4E2 | 4.8E2 | 20 | 18 | 20 | 18 | 0.49 |
| gL | pg/ml | 6.5E4 | 6.5E4 | 7.3E4 | 7.2E4 | 3.2E4 | 3.4E4 | 2.3E4 | 1.1E4 | 1.6E5 | 1.7E5 | 53 | 60 | 53 | 60 | 0.47 |
| gP | U/ml | 2.6E2 | 2.9E2 | 2.8E2 | 2.9E2 | 1.4E2 | 9.4E1 | 6.5E1 | 1.2E1 | 1.1E3 | 5.2E2 | 53 | 59 | 53 | 59 | 0.59 |
| gW | ng/ml | 5.4E2 | 3.7E2 | 1.0E3 | 6.9E2 | 1.1E3 | 8.4E2 | 6.8E1 | 2.3E0 | 6.1E3 | 4.2E3 | 39 | 44 | 39 | 44 | 0.39 |
| gV | ng/ml | 1.7E1 | 2.2E1 | 1.9E1 | 1.9E1 | 5.7E0 | 8.3E0 | 1.0E1 | 8.1E-2 | 2.7E1 | 3.0E1 | 18 | 11 | 18 | 11 | 0.56 |
| lF | pg/mL | 1.6E3 | 9.0E2 | 1.5E4 | 1.1E4 | 4.6E4 | 3.7E4 | 1.8E1 | 1.2E1 | 2.8E5 | 2.5E5 | 47 | 57 | 47 | 57 | 0.43 |
| gZ | ug/ml | 6.7E-1 | 7.8E-1 | 3.0E1 | 4.1E1 | 1.1E2 | 1.1E2 | 8.7E-2 | 3.6E-1 | 4.1E2 | 4.0E2 | 14 | 15 | 14 | 15 | 0.60 |
| hA | ng/ml | 2.6E0 | 2.9E0 | 1.7E1 | 1.5E1 | 6.0E1 | 4.6E1 | 1.7E-2 | 1.7E-2 | 3.5E2 | 2.9E2 | 40 | 44 | 40 | 44 | 0.53 |
| nM | pg/ml | 1.0E-9 | 1.0E-9 | 1.0E-9 | 4.2E1 | 0.0E0 | 2.5E2 | 1.0E-9 | 1.0E-9 | 1.0E-9 | 1.5E3 | 28 | 36 | 28 | 36 | 0.51 |
| nN | pg/ml | 1.1E3 | 2.2E3 | 1.8E3 | 1.2E4 | 1.8E3 | 3.0E4 | 1.1E2 | 3.5E2 | 7.1E3 | 1.5E5 | 28 | 36 | 28 | 36 | 0.69 |
| nO | pg/ml | 2.0E1 | 2.5E1 | 2.6E1 | 4.6E1 | 1.5E1 | 6.0E1 | 8.8E0 | 4.0E0 | 6.2E1 | 3.1E2 | 28 | 36 | 28 | 36 | 0.57 |
| nR | pg/ml | 2.0E1 | 1.8E1 | 5.1E1 | 1.4E2 | 7.2E1 | 3.6E2 | 1.8E0 | 1.0E0 | 2.6E2 | 1.9E3 | 28 | 36 | 28 | 36 | 0.51 |
| nT | pg/ml | 6.7E1 | 9.2E1 | 9.0E1 | 1.4E2 | 6.8E1 | 1.8E2 | 1.0E-9 | 1.0E-9 | 2.4E2 | 9.2E2 | 28 | 36 | 28 | 36 | 0.55 |
| nU | pg/ml | 4.4E1 | 4.5E1 | 5.6E1 | 1.4E2 | 6.4E1 | 2.9E2 | 1.0E-9 | 1.0E-9 | 2.1E2 | 1.5E3 | 28 | 36 | 28 | 36 | 0.56 |
| lW | pg/ml | 1.0E-9 | 1.0E-9 | 9.3E0 | 1.4E1 | 1.6E1 | 4.0E1 | 1.0E-9 | 1.0E-9 | 5.5E1 | 1.7E2 | 28 | 36 | 28 | 36 | 0.46 |
| lX | pg/ml | 9.1E2 | 8.8E2 | 9.4E2 | 9.7E2 | 4.7E2 | 6.2E2 | 3.2E2 | 1.9E2 | 1.9E3 | 2.5E3 | 28 | 36 | 28 | 36 | 0.49 |
| lY | pg/ml | 1.6E1 | 2.3E1 | 1.6E1 | 2.4E1 | 1.2E1 | 2.1E1 | 1.0E-9 | 1.0E-9 | 4.8E1 | 1.2E2 | 28 | 36 | 28 | 36 | 0.62 |
| mE | pg/ml | 1.0E-9 | 1.0E-9 | 9.0E-1 | 4.1E0 | 1.8E0 | 1.0E1 | 1.0E-9 | 1.0E-9 | 7.6E0 | 5.7E1 | 28 | 36 | 28 | 36 | 0.61 |
| mF | pg/ml | 1.0E-9 | 5.5E-1 | 1.0E1 | 5.1E0 | 4.6E1 | 1.8E1 | 1.0E-9 | 1.0E-9 | 2.5E2 | 1.1E2 | 28 | 36 | 28 | 36 | 0.59 |
| mH | pg/ml | 4.0E0 | 2.9E0 | 5.1E0 | 5.5E0 | 5.9E0 | 8.9E0 | 1.1E0 | 4.0E-1 | 3.2E1 | 5.3E1 | 28 | 36 | 28 | 36 | 0.48 |
| mI | pg/ml | 1.0E-9 | 3.6E0 | 5.6E0 | 3.2E1 | 1.1E1 | 8.1E1 | 1.0E-9 | 1.0E-9 | 3.7E1 | 4.6E2 | 28 | 36 | 28 | 36 | 0.63 |
| mM | pg/ml | 3.3E1 | 5.7E1 | 5.9E1 | 1.2E2 | 6.0E1 | 2.1E2 | 1.0E-9 | 1.0E-9 | 2.1E2 | 1.1E3 | 28 | 36 | 28 | 36 | 0.54 |
| mP | pg/ml | 1.3E1 | 1.6E1 | 1.6E1 | 4.4E1 | 1.1E1 | 1.3E2 | 1.0E-9 | 1.0E-9 | 5.8E1 | 8.1E2 | 27 | 36 | 27 | 36 | 0.56 |
| mS | pg/ml | 1.6E3 | 1.6E3 | 1.7E3 | 1.7E3 | 8.2E2 | 1.1E3 | 8.8E1 | 1.0E-9 | 3.7E3 | 5.1E3 | 28 | 36 | 28 | 36 | 0.50 |
| mT | pg/ml | 5.0E1 | 6.6E1 | 1.5E2 | 1.7E2 | 2.9E2 | 3.4E2 | 1.0E1 | 1.2E1 | 1.4E3 | 1.7E3 | 27 | 36 | 27 | 36 | 0.51 |
| mU | pg/ml | 2.5E0 | 1.9E0 | 3.4E0 | 9.6E0 | 2.9E0 | 3.7E1 | 1.0E-9 | 1.0E-9 | 1.6E1 | 2.2E2 | 27 | 36 | 27 | 36 | 0.44 |
| mW | pg/ml | 2.3E3 | 2.1E3 | 2.5E3 | 2.6E3 | 1.1E3 | 2.0E3 | 4.3E2 | 1.0E-9 | 4.9E3 | 1.1E4 | 27 | 36 | 27 | 36 | 0.45 |
| mY | pg/ml | 5.7E2 | 7.5E2 | 7.8E2 | 1.2E3 | 9.0E2 | 1.6E3 | 1.0E-9 | 1.0E-9 | 4.2E3 | 8.0E3 | 28 | 36 | 28 | 36 | 0.59 |
| mZ | pg/ml | 2.5E2 | 1.5E2 | 4.3E2 | 3.1E2 | 6.0E2 | 3.8E2 | 4.7E1 | 1.1E1 | 3.1E3 | 1.5E3 | 27 | 36 | 27 | 36 | 0.38 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| nA | pg/ml | 1.5E0 | 1.8E0 | 6.7E0 | 6.3E0 | 1.4E1 | 1.3E1 | 1.0E-9 | 1.0E-9 | 5.4E1 | 6.5E1 | 27 | 36 | 27 | 36 | 0.52 |
| nB | pg/ml | 2.6E2 | 3.0E2 | 2.9E2 | 3.5E2 | 1.2E2 | 2.1E2 | 3.0E1 | 3.8E1 | 5.9E2 | 9.6E2 | 28 | 36 | 28 | 36 | 0.55 |
| nC | pg/ml | 1.0E-9 | 8.3E1 | 7.0E2 | 1.8E4 | 2.1E3 | 7.3E4 | 1.0E-9 | 1.0E-9 | 1.1E4 | 3.8E5 | 28 | 36 | 28 | 36 | 0.57 |
| nD | pg/ml | 6.2E0 | 7.3E0 | 8.2E0 | 1.6E1 | 8.2E0 | 4.3E1 | 1.0E-9 | 1.0E-9 | 3.7E1 | 2.6E2 | 27 | 36 | 27 | 36 | 0.52 |
| nF | pg/ml | 1.0E-9 | 1.0E-9 | 3.7E0 | 2.2E0 | 1.8E1 | 7.6E0 | 1.0E-9 | 1.0E-9 | 9.3E1 | 3.5E1 | 28 | 36 | 28 | 36 | 0.52 |
| nH | pg/ml | 1.0E-9 | 2.8E0 | 3.9E0 | 3.6E2 | 6.0E0 | 1.7E3 | 1.0E-9 | 1.0E-9 | 1.8E1 | 1.0E4 | 27 | 36 | 27 | 36 | 0.60 |
| nI | pg/ml | 2.5E1 | 1.3E-1 | 5.6E1 | 4.9E1 | 6.7E1 | 8.1E1 | 1.0E-9 | 1.0E-9 | 2.0E2 | 3.5E2 | 28 | 36 | 28 | 36 | 0.45 |
| nJ | pg/ml | 6.1E-1 | 2.9E-1 | 1.0E0 | 4.6E0 | 1.6E0 | 2.2E1 | 1.0E-9 | 1.0E-9 | 7.0E0 | 1.3E2 | 28 | 36 | 28 | 36 | 0.48 |
| nK | pg/ml | 1.0E-9 | 1.2E1 | 5.4E0 | 1.9E1 | 1.6E1 | 2.6E1 | 1.0E-9 | 1.0E-9 | 5.7E1 | 1.0E2 | 27 | 36 | 27 | 36 | 0.69 |
| nL | pg/ml | 1.0E-9 | 4.3E0 | 6.1E0 | 5.4E2 | 1.2E1 | 2.3E3 | 1.0E-9 | 1.0E-9 | 4.9E1 | 1.4E4 | 28 | 36 | 28 | 36 | 0.64 |
| hL | pg/ml | 1.6E4 | 2.4E4 | 2.0E4 | 2.4E4 | 1.5E4 | 1.4E4 | 2.6E3 | 5.1E3 | 7.2E4 | 6.0E4 | 26 | 23 | 26 | 23 | 0.60 |
| hO | pg/ml | 1.6E4 | 1.5E4 | 1.6E4 | 1.5E4 | 2.7E3 | 2.2E3 | 1.3E4 | 1.1E4 | 2.4E4 | 2.1E4 | 26 | 23 | 26 | 23 | 0.40 |
| hP | ng/ml | 4.4E5 | 4.4E5 | 3.8E5 | 6.3E5 | 1.9E5 | 6.8E5 | 4.7E4 | 1.7E4 | 9.0E5 | 2.8E6 | 26 | 23 | 26 | 23 | 0.62 |
| wJ | pg/ml | 1.5E5 | 1.0E5 | 1.6E5 | 1.6E5 | 9.5E4 | 1.4E5 | 1.1E4 | 1.3E4 | 4.0E5 | 5.8E5 | 25 | 26 | 25 | 26 | 0.44 |
| wK | pg/ml | 3.2E4 | 3.4E4 | 6.0E4 | 4.0E4 | 9.7E4 | 3.0E4 | 3.7E3 | 7.5E3 | 5.0E5 | 1.2E5 | 25 | 26 | 25 | 26 | 0.46 |
| wL | pg/ml | 9.5E0 | 3.1E0 | 7.8E1 | 3.1E1 | 1.9E2 | 9.0E1 | 1.0E-9 | 1.0E-9 | 8.4E2 | 3.5E2 | 25 | 26 | 25 | 26 | 0.36 |
| wP | pg/ml | 3.0E4 | 2.8E4 | 3.9E4 | 6.4E4 | 3.4E4 | 7.5E4 | 4.5E3 | 1.3E3 | 1.6E5 | 3.0E5 | 25 | 26 | 25 | 26 | 0.56 |
| wQ | pg/ml | 3.1E1 | 4.1E1 | 6.0E1 | 6.3E1 | 8.6E1 | 1.0E2 | 1.0E-9 | 1.0E-9 | 3.7E2 | 5.1E2 | 25 | 26 | 25 | 26 | 0.53 |
| hR | pg/ml | 2.8E4 | 2.6E4 | 3.2E4 | 2.7E4 | 1.1E4 | 1.1E4 | 1.8E4 | 1.0E-9 | 5.8E4 | 4.9E4 | 36 | 41 | 36 | 41 | 0.40 |
| hV | pg/ml | 4.7E2 | 4.4E2 | 4.5E2 | 4.2E2 | 2.1E2 | 2.2E2 | 1.3E2 | 1.0E-9 | 9.3E2 | 9.6E2 | 36 | 41 | 36 | 41 | 0.45 |
| hW | pg/ml | 1.7E3 | 2.4E3 | 2.0E3 | 3.3E3 | 1.2E3 | 6.0E3 | 9.0E2 | 1.0E-9 | 7.3E3 | 4.0E4 | 36 | 41 | 36 | 41 | 0.62 |
| hX | pg/ml | 1.1E3 | 1.1E3 | 1.3E3 | 1.1E3 | 1.3E3 | 5.5E2 | 3.3E2 | 2.5E0 | 8.6E3 | 2.9E3 | 36 | 41 | 36 | 41 | 0.49 |
| iA | pg/ml | 1.9E2 | 1.7E2 | 3.0E2 | 2.4E2 | 3.3E2 | 2.3E2 | 3.0E1 | 1.5E1 | 1.8E3 | 8.7E2 | 47 | 57 | 47 | 57 | 0.44 |
| iB | ng/ml | 4.8E0 | 5.7E0 | 5.8E0 | 7.8E0 | 4.0E0 | 5.9E0 | 3.3E-2 | 8.3E-1 | 1.9E1 | 2.4E1 | 40 | 44 | 40 | 44 | 0.59 |
| iC | U/ml | 2.6E-1 | 4.5E-1 | 4.2E-1 | 2.1E0 | 4.8E-1 | 8.3E0 | 1.0E-9 | 3.7E-2 | 1.8E0 | 5.5E1 | 40 | 44 | 40 | 44 | 0.63 |
| tQ | pg/ml | 1.3E3 | 1.6E3 | 1.4E3 | 1.6E3 | 5.1E2 | 6.6E2 | 3.7E2 | 5.0E2 | 2.5E3 | 3.3E3 | 24 | 23 | 24 | 23 | 0.55 |
| tT | pg/ml | 1.4E1 | 2.3E1 | 1.7E1 | 2.7E1 | 7.4E0 | 1.8E1 | 7.4E0 | 5.4E0 | 3.2E1 | 9.3E1 | 24 | 24 | 24 | 24 | 0.71 |
| tS | pg/ml | 8.9E-1 | 7.7E-1 | 9.7E-1 | 1.4E0 | 9.5E-1 | 2.2E0 | 1.0E-9 | 1.0E-9 | 4.0E0 | 1.0E1 | 24 | 25 | 24 | 25 | 0.53 |
| tX | pg/ml | 8.7E-1 | 1.2E0 | 1.1E0 | 2.3E0 | 8.9E-1 | 2.6E0 | 2.5E-2 | 2.8E-1 | 3.3E0 | 1.0E1 | 24 | 24 | 24 | 24 | 0.64 |
| tO | pg/ml | 4.4E0 | 3.9E0 | 4.4E0 | 5.5E0 | 2.6E0 | 4.0E0 | 1.0E-9 | 1.7E0 | 9.9E0 | 1.8E1 | 24 | 25 | 24 | 25 | 0.56 |
| tR | pg/ml | 1.7E-1 | 2.2E-1 | 2.5E-1 | 3.7E-1 | 2.6E-1 | 5.7E-1 | 1.0E-9 | 1.0E-9 | 9.1E-1 | 2.5E0 | 24 | 24 | 24 | 24 | 0.54 |
| tU | pg/ml | 9.0E0 | 9.9E0 | 1.2E1 | 1.4E1 | 1.1E1 | 1.6E1 | 1.6E0 | 2.2E-1 | 5.5E1 | 8.0E1 | 24 | 26 | 24 | 26 | 0.53 |
| tN | pg/ml | 1.9E1 | 2.0E1 | 2.0E1 | 4.1E1 | 1.4E1 | 4.3E1 | 1.0E-9 | 9.5E0 | 5.4E1 | 1.6E2 | 24 | 23 | 24 | 23 | 0.63 |
| tV | ng/ml | 3.9E2 | 9.8E2 | 5.6E2 | 9.9E2 | 4.1E2 | 6.5E2 | 1.9E2 | 5.3E1 | 1.8E3 | 3.1E3 | 25 | 25 | 25 | 25 | 0.73 |
| iH | ng/ml | 1.6E5 | 1.7E5 | 1.6E5 | 1.6E5 | 4.4E4 | 5.6E4 | 7.1E4 | 2.9E3 | 2.6E5 | 2.5E5 | 47 | 57 | 47 | 57 | 0.54 |
| iJ | ng/ml | 5.6E4 | 4.3E4 | 5.7E4 | 5.5E4 | 2.2E4 | 4.6E4 | 2.1E4 | 1.8E3 | 1.2E5 | 2.5E5 | 47 | 57 | 47 | 57 | 0.39 |
| hB | ng/ml | 4.6E-1 | 5.1E-1 | 6.0E-1 | 7.0E-1 | 4.3E-1 | 5.9E-1 | 1.4E-1 | 1.2E-1 | 1.9E0 | 3.2E0 | 47 | 57 | 47 | 57 | 0.55 |
| hC | pg/ml | 3.7E3 | 7.7E3 | 6.1E3 | 1.0E4 | 6.4E3 | 1.6E4 | 6.0E1 | 4.1E1 | 3.3E4 | 1.1E5 | 47 | 57 | 47 | 57 | 0.60 |
| hF | ng/ml | 1.0E-9 | 1.0E-9 | 8.5E1 | 1.0E-9 | 5.8E2 | 0.0E0 | 1.0E-9 | 1.0E-9 | 4.0E3 | 1.0E-9 | 47 | 57 | 47 | 57 | 0.49 |
| hG | pg/ml | 7.0E3 | 6.6E3 | 8.2E3 | 7.5E3 | 3.4E3 | 3.5E3 | 1.8E3 | 2.3E3 | 1.8E4 | 2.0E4 | 47 | 57 | 47 | 57 | 0.41 |
| iO | ng/ml | 4.1E5 | 3.8E5 | 4.6E5 | 4.1E5 | 2.0E5 | 2.0E5 | 1.9E5 | 9.8E4 | 1.1E6 | 9.2E5 | 47 | 57 | 47 | 57 | 0.43 |
| iP | ng/ml | 6.0E4 | 5.1E4 | 6.2E4 | 5.5E4 | 6.1E4 | 3.4E4 | 8.3E3 | 7.1E3 | 4.4E5 | 2.2E5 | 47 | 57 | 47 | 57 | 0.46 |
| iZ | ng/ml | 1.5E3 | 1.8E3 | 1.8E3 | 2.0E3 | 9.1E2 | 8.4E2 | 8.2E2 | 7.5E2 | 5.7E3 | 4.6E3 | 47 | 55 | 47 | 55 | 0.59 |
| yH | pg/ml | 1.0E3 | 1.1E3 | 1.7E3 | 3.2E3 | 3.1E3 | 6.6E3 | 1.0E-9 | 1.0E-9 | 1.5E4 | 2.5E4 | 24 | 25 | 24 | 25 | 0.54 |
| yK | U/ml | 1.9E1 | 2.1E1 | 5.1E1 | 3.2E1 | 9.8E1 | 3.4E1 | 1.0E-9 | 1.0E-9 | 3.8E2 | 1.4E2 | 24 | 25 | 24 | 25 | 0.55 |
| yJ | pg/ml | 3.8E4 | 3.6E4 | 4.5E4 | 3.8E4 | 2.6E4 | 2.9E4 | 9.2E3 | 1.9E3 | 1.0E5 | 1.4E5 | 24 | 25 | 24 | 25 | 0.41 |
| yD | ng/ml | 1.2E-2 | 1.3E-2 | 1.3E-2 | 1.3E-2 | 5.9E-3 | 6.1E-3 | 1.0E-9 | 1.0E-9 | 2.8E-2 | 2.4E-2 | 25 | 26 | 25 | 26 | 0.52 |
| jB | ng/ml | 2.8E5 | 2.0E5 | 2.7E5 | 2.0E5 | 7.0E4 | 6.1E4 | 1.5E5 | 9.9E4 | 3.6E5 | 3.3E5 | 14 | 15 | 14 | 15 | 0.21 |
| wB | pg/ml | 9.5E3 | 9.8E3 | 1.1E4 | 1.3E4 | 7.6E3 | 1.0E4 | 1.9E3 | 2.3E3 | 3.3E4 | 4.2E4 | 25 | 26 | 25 | 26 | 0.55 |
| pY | pg/ml | 5.3E0 | 6.4E0 | 1.3E1 | 7.3E0 | 3.8E1 | 3.8E0 | 1.6E0 | 3.0E0 | 2.0E2 | 1.8E1 | 26 | 23 | 26 | 23 | 0.64 |
| sI | ng/ml | 4.8E-2 | 6.2E-2 | 5.3E-2 | 6.1E-2 | 2.3E-2 | 3.7E-2 | 2.1E-2 | 1.0E-2 | 1.1E-1 | 1.5E-1 | 13 | 13 | 13 | 13 | 0.57 |
| sF | mIU/mL | 6.7E0 | 5.6E0 | 1.2E1 | 1.1E1 | 2.0E1 | 1.4E1 | 6.2E-1 | 1.5E0 | 7.5E1 | 5.2E1 | 13 | 13 | 13 | 13 | 0.51 |
| sH | mIU/mL | 4.6E0 | 3.5E0 | 5.1E0 | 4.1E0 | 5.7E0 | 4.0E0 | 1.0E-9 | 3.9E-1 | 2.1E1 | 1.5E1 | 13 | 13 | 13 | 13 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| sJ | ng/ml | 1.5E-1 | 1.5E-1 | 8.8E-1 | 1.9E-1 | 1.9E0 | 1.8E-1 | 1.0E-9 | 1.0E-9 | 6.4E0 | 6.1E-1 | 13 | 13 | 13 | 13 | 0.44 |
| rC | pg/ml | 1.9E3 | 1.4E3 | 2.5E3 | 1.9E3 | 2.8E3 | 2.0E3 | 1.1E2 | 1.0E-9 | 1.5E4 | 1.1E4 | 34 | 40 | 34 | 40 | 0.42 |
| rB | pg/ml | 3.1E1 | 2.9E1 | 5.2E1 | 5.6E1 | 7.8E1 | 7.0E1 | 1.0E-9 | 1.0E-9 | 3.9E2 | 3.2E2 | 34 | 40 | 34 | 40 | 0.52 |
| zG | 2.5ng/ml | 2.4E-1 | 1.9E-1 | 5.6E-1 | 6.3E-1 | 1.0E0 | 1.1E0 | 1.0E-9 | 1.0E-9 | 4.4E0 | 4.8E0 | 24 | 25 | 24 | 25 | 0.51 |
| zH | 2.3mU/ml | 9.2E-2 | 8.7E-2 | 1.0E-1 | 8.9E-2 | 5.4E-2 | 3.9E-2 | 1.0E-2 | 2.1E-2 | 3.1E-1 | 1.8E-1 | 24 | 25 | 24 | 25 | 0.41 |
| zI | 2.6ng/ml | 2.1E0 | 2.3E0 | 3.2E0 | 5.7E0 | 3.3E0 | 7.4E0 | 6.1E-1 | 5.4E-1 | 1.5E1 | 2.7E1 | 24 | 25 | 24 | 25 | 0.57 |
| qA | ng/ml | 8.0E6 | 1.3E7 | 1.0E7 | 1.3E7 | 7.7E6 | 6.7E6 | 3.7E6 | 4.3E6 | 3.9E7 | 3.0E7 | 26 | 23 | 26 | 23 | 0.69 |
| qB | ng/ml | 7.2E5 | 5.7E5 | 8.5E5 | 8.7E5 | 5.0E5 | 8.7E5 | 2.7E5 | 1.9E5 | 2.4E6 | 3.8E6 | 26 | 23 | 26 | 23 | 0.42 |
| qC | ng/ml | 3.6E5 | 2.6E5 | 6.6E5 | 5.2E5 | 7.9E5 | 9.5E5 | 3.6E4 | 2.5E4 | 3.1E6 | 4.7E6 | 26 | 23 | 26 | 23 | 0.41 |
| qD | ng/ml | 1.5E7 | 1.5E7 | 1.7E7 | 1.6E7 | 7.8E6 | 7.0E6 | 7.0E6 | 7.0E6 | 3.7E7 | 3.4E7 | 26 | 23 | 26 | 23 | 0.47 |
| jD | ng/ml | 4.0E1 | 3.2E1 | 4.1E1 | 5.3E1 | 3.4E1 | 6.1E1 | 1.0E-9 | 1.0E-9 | 1.1E2 | 2.9E2 | 40 | 44 | 40 | 44 | 0.52 |
| jE | ng/ml | 1.0E-9 | 1.0E-9 | 5.0E0 | 5.2E0 | 1.3E1 | 1.0E1 | 1.0E-9 | 1.0E-9 | 6.2E1 | 3.5E1 | 40 | 44 | 40 | 44 | 0.53 |
| jF | ng/ml | 5.0E1 | 2.1E1 | 5.2E1 | 3.7E1 | 5.3E1 | 4.8E1 | 1.0E-9 | 1.0E-9 | 1.9E2 | 1.9E2 | 40 | 44 | 40 | 44 | 0.42 |
| jG | ng/ml | 4.4E3 | 4.0E3 | 4.7E3 | 4.2E3 | 1.9E3 | 2.0E3 | 1.9E3 | 6.7E2 | 9.5E3 | 9.6E3 | 40 | 44 | 40 | 44 | 0.43 |
| jH | ng/ml | 8.0E1 | 7.5E1 | 8.2E1 | 8.9E1 | 4.3E1 | 6.9E1 | 2.1E1 | 1.3E1 | 2.1E2 | 4.3E2 | 40 | 44 | 40 | 44 | 0.49 |
| jI | ng/ml | 7.4E1 | 7.7E1 | 8.0E1 | 9.4E1 | 3.3E1 | 7.0E1 | 3.8E1 | 3.8E1 | 1.9E2 | 4.4E2 | 40 | 44 | 40 | 44 | 0.53 |
| sK | pg/mL | 4.3E3 | 3.6E3 | 4.1E3 | 5.0E3 | 1.7E3 | 4.5E3 | 1.8E3 | 2.1E3 | 8.8E3 | 2.3E4 | 24 | 23 | 24 | 23 | 0.50 |
| sM | pg/mL | 7.4E4 | 7.4E4 | 7.9E4 | 8.5E4 | 2.9E4 | 3.9E4 | 4.8E4 | 3.9E4 | 1.6E5 | 2.0E5 | 24 | 23 | 24 | 23 | 0.54 |
| sO | pg/mL | 2.3E8 | 2.3E8 | 2.4E8 | 2.4E8 | 8.2E7 | 1.1E8 | 4.9E7 | 6.6E7 | 3.7E8 | 4.4E8 | 24 | 23 | 24 | 23 | 0.48 |
| wC | ng/ml | 1.6E0 | 1.5E0 | 2.0E0 | 1.8E0 | 1.4E0 | 1.1E0 | 3.6E-1 | 6.1E-2 | 6.5E0 | 4.8E0 | 25 | 26 | 25 | 26 | 0.47 |
| wD | ng/ml | 2.1E1 | 2.8E1 | 1.2E2 | 5.3E1 | 4.2E2 | 6.1E1 | 3.8E0 | 2.8E0 | 2.1E3 | 2.9E2 | 25 | 26 | 25 | 26 | 0.63 |
| wE | ng/ml | 4.6E1 | 5.7E1 | 4.6E1 | 5.6E1 | 1.8E1 | 2.0E1 | 8.1E0 | 2.0E1 | 7.6E1 | 8.9E1 | 25 | 26 | 25 | 26 | 0.62 |
| wG | ng/ml | 1.2E-1 | 8.1E-2 | 1.5E-1 | 1.3E-1 | 1.4E-1 | 1.5E-1 | 1.0E-9 | 1.0E-9 | 4.8E-1 | 6.8E-1 | 25 | 26 | 25 | 26 | 0.45 |
| wH | ng/ml | 4.7E-2 | 3.3E-2 | 3.2E-1 | 4.4E-1 | 8.8E-1 | 1.1E0 | 1.0E-9 | 1.0E-9 | 4.2E0 | 5.6E0 | 25 | 26 | 25 | 26 | 0.51 |
| wF | ng/ml | 2.7E-1 | 2.7E-1 | 3.5E0 | 1.6E0 | 1.2E1 | 3.8E0 | 1.0E-9 | 1.0E-9 | 5.7E1 | 1.9E1 | 25 | 26 | 25 | 26 | 0.55 |
| rA | pg/ml | 2.4E1 | 2.2E1 | 2.6E1 | 2.9E1 | 1.6E1 | 2.4E1 | 1.0E-9 | 1.0E-9 | 5.9E1 | 1.1E2 | 38 | 43 | 38 | 43 | 0.50 |
| qZ | pg/ml | 4.7E1 | 5.4E1 | 3.9E2 | 8.6E2 | 1.8E3 | 2.6E3 | 2.8E-4 | 5.9E-4 | 1.0E4 | 1.0E4 | 30 | 30 | 30 | 30 | 0.55 |
| qY | pg/ml | 1.7E1 | 1.4E1 | 4.1E1 | 3.3E1 | 5.2E1 | 5.7E1 | 8.7E-1 | 2.1E0 | 1.8E2 | 3.3E2 | 38 | 43 | 38 | 43 | 0.46 |
| qX | pg/ml | 5.5E1 | 6.8E1 | 6.2E1 | 7.8E1 | 3.9E1 | 5.4E1 | 1.0E-9 | 1.0E-9 | 1.6E2 | 2.1E2 | 38 | 43 | 38 | 43 | 0.56 |
| qW | pg/ml | 7.1E0 | 7.6E0 | 8.6E0 | 1.0E1 | 8.0E0 | 9.7E0 | 1.0E-9 | 1.0E-9 | 3.6E1 | 3.6E1 | 38 | 43 | 38 | 43 | 0.52 |
| qV | pg/ml | 1.5E3 | 2.2E3 | 2.3E3 | 2.4E3 | 2.1E3 | 1.8E3 | 2.7E2 | 1.0E2 | 1.1E4 | 9.6E3 | 38 | 43 | 38 | 43 | 0.53 |
| qU | pg/ml | 4.7E1 | 9.5E1 | 1.4E2 | 2.4E2 | 2.4E2 | 3.8E2 | 1.0E-9 | 1.0E-9 | 1.2E3 | 2.1E3 | 38 | 43 | 38 | 43 | 0.61 |
| qT | pg/ml | 3.7E1 | 4.1E1 | 7.3E1 | 6.1E1 | 9.6E1 | 4.6E1 | 1.0E-9 | 1.0E-9 | 4.9E2 | 1.8E2 | 38 | 43 | 38 | 43 | 0.54 |
| qI | ng/ml | 6.0E4 | 6.4E4 | 6.0E4 | 6.7E4 | 2.8E4 | 3.4E4 | 1.0E4 | 2.5E4 | 1.3E5 | 1.6E5 | 24 | 22 | 24 | 22 | 0.55 |
| qH | ng/ml | 6.3E4 | 5.7E4 | 7.1E4 | 7.1E4 | 4.2E4 | 3.9E4 | 1.5E4 | 2.3E4 | 1.8E5 | 1.8E5 | 24 | 22 | 24 | 22 | 0.52 |
| qG | ng/ml | 1.8E5 | 1.9E5 | 1.8E5 | 2.0E5 | 6.3E4 | 7.6E4 | 3.4E4 | 8.4E4 | 3.0E5 | 4.2E5 | 24 | 22 | 24 | 22 | 0.55 |
| jK | ng/ml | 1.5E3 | 1.4E3 | 1.6E3 | 1.6E3 | 6.3E2 | 6.5E2 | 2.8E2 | 7.5E2 | 4.1E3 | 3.6E3 | 40 | 44 | 40 | 44 | 0.46 |
| jL | ng/ml | 1.7E2 | 2.1E2 | 2.7E2 | 2.8E2 | 2.1E2 | 1.8E2 | 5.9E1 | 6.4E1 | 8.1E2 | 7.6E2 | 40 | 44 | 40 | 44 | 0.55 |
| jM | ng/ml | 6.3E4 | 6.8E4 | 6.6E4 | 7.8E4 | 3.2E4 | 4.5E4 | 2.1E4 | 4.6E3 | 1.5E5 | 1.7E5 | 40 | 44 | 40 | 44 | 0.57 |
| jO | pg/ml | 2.2E5 | 2.5E5 | 2.7E5 | 2.7E5 | 1.5E5 | 1.5E5 | 7.6E4 | 9.6E4 | 7.7E5 | 6.5E5 | 40 | 44 | 40 | 44 | 0.50 |
| jP | pg/ml | 2.6E5 | 2.7E5 | 2.9E5 | 3.2E5 | 1.5E5 | 1.5E5 | 6.1E4 | 1.3E5 | 7.1E5 | 7.0E5 | 40 | 44 | 40 | 44 | 0.55 |
| jQ | pg/ml | 2.5E3 | 2.1E3 | 3.1E3 | 2.9E3 | 2.5E3 | 2.9E3 | 2.5E2 | 5.0E0 | 1.0E4 | 1.3E4 | 40 | 44 | 40 | 44 | 0.45 |
| jR | pg/ml | 5.9E3 | 4.3E3 | 8.7E3 | 1.0E4 | 8.4E3 | 1.3E4 | 3.0E1 | 1.0E-9 | 3.5E4 | 5.6E4 | 40 | 44 | 40 | 44 | 0.47 |
| jT | pg/ml | 1.7E5 | 1.7E5 | 1.7E5 | 1.7E5 | 6.1E4 | 6.0E4 | 7.1E4 | 7.5E4 | 3.5E5 | 3.5E5 | 40 | 44 | 40 | 44 | 0.51 |
| xA | pg/ml | 9.0E0 | 3.9E0 | 1.4E1 | 1.5E1 | 1.7E1 | 3.7E1 | 1.0E-9 | 1.0E-9 | 6.1E1 | 1.6E2 | 24 | 25 | 24 | 25 | 0.37 |
| yE | pg/ml | 8.1E1 | 8.6E1 | 8.0E1 | 8.7E1 | 3.2E1 | 3.8E1 | 6.4E0 | 3.5E1 | 1.4E2 | 2.0E2 | 24 | 25 | 24 | 25 | 0.53 |
| tM | pg/ml | 4.3E1 | 3.9E1 | 4.0E1 | 4.1E1 | 1.7E1 | 2.0E1 | 1.0E-9 | 1.1E1 | 6.1E1 | 9.9E1 | 24 | 25 | 24 | 25 | 0.46 |
| tL | pg/ml | 1.0E-9 | 1.0E-9 | 3.0E0 | 3.4E-1 | 1.4E1 | 8.8E-1 | 1.0E-9 | 1.0E-9 | 6.7E1 | 3.6E0 | 24 | 25 | 24 | 25 | 0.49 |
| jU | mIU/ml | 5.4E0 | 6.4E0 | 1.4E1 | 1.1E1 | 2.4E1 | 1.3E1 | 8.1E-2 | 7.1E-1 | 1.1E2 | 5.7E1 | 40 | 44 | 40 | 44 | 0.53 |
| jV | mIU/ml | 1.8E0 | 2.1E0 | 4.5E0 | 3.6E0 | 6.7E0 | 4.2E0 | 2.7E-3 | 4.9E-2 | 3.2E1 | 1.8E1 | 40 | 44 | 40 | 44 | 0.49 |
| jY | ng/ml | 5.8E-4 | 1.7E-3 | 1.0E-2 | 7.2E-3 | 4.7E-2 | 1.6E-2 | 1.0E-9 | 1.0E-9 | 3.0E-1 | 9.4E-2 | 40 | 44 | 40 | 44 | 0.57 |
| kC | pg/ml | 9.7E1 | 1.1E2 | 1.4E2 | 2.3E2 | 1.9E2 | 4.7E2 | 2.1E1 | 3.6E1 | 1.1E3 | 2.7E3 | 28 | 36 | 28 | 36 | 0.56 |
| kE | pg/ml | 1.5E5 | 1.4E5 | 1.4E5 | 1.4E5 | 3.8E4 | 4.3E4 | 4.1E4 | 3.8E4 | 2.3E5 | 2.7E5 | 28 | 36 | 28 | 36 | 0.50 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| kF | pg/mL | 6.8E1 | 6.5E1 | 6.8E1 | 7.3E1 | 1.7E1 | 3.1E1 | 3.5E1 | 4.0E1 | 1.1E2 | 1.5E2 | 28 | 36 | 28 | 36 | 0.47 |
| kG | pg/mL | 8.0E3 | 8.9E3 | 1.1E4 | 1.7E4 | 9.2E3 | 2.8E4 | 1.9E3 | 1.1E3 | 4.1E4 | 1.6E5 | 28 | 36 | 28 | 36 | 0.55 |
| kI | pg/ml | 2.2E2 | 2.0E2 | 2.3E2 | 2.2E2 | 1.2E2 | 1.2E2 | 6.4E1 | 1.0E-9 | 6.7E2 | 5.5E2 | 28 | 36 | 28 | 36 | 0.47 |
| kK | pg/ml | 1.2E2 | 1.2E2 | 1.4E2 | 1.7E2 | 1.1E2 | 1.7E2 | 2.2E1 | 2.1E1 | 5.0E2 | 9.1E2 | 28 | 36 | 28 | 36 | 0.52 |
| kN | pg/ml | 1.0E3 | 1.2E3 | 1.5E3 | 1.8E3 | 1.9E3 | 1.9E3 | 2.1E2 | 3.8E2 | 1.0E4 | 8.7E3 | 28 | 36 | 28 | 36 | 0.53 |
| kO | pg/ml | 7.0E3 | 7.4E3 | 7.6E3 | 1.2E4 | 2.9E3 | 2.3E4 | 4.0E3 | 3.8E3 | 1.9E4 | 1.5E5 | 28 | 36 | 28 | 36 | 0.52 |
| kP | pg/ml | 5.6E3 | 5.3E3 | 7.3E3 | 6.1E3 | 4.8E3 | 3.8E3 | 3.1E3 | 9.6E2 | 2.7E4 | 1.5E4 | 28 | 36 | 28 | 36 | 0.42 |
| kQ | pg/ml | 4.4E3 | 4.5E3 | 5.3E3 | 5.9E3 | 3.7E3 | 4.6E3 | 5.6E2 | 1.1E3 | 2.5E4 | 2.5E4 | 47 | 57 | 47 | 57 | 0.52 |
| kR | pg/ml | 2.2E1 | 2.6E1 | 4.8E1 | 3.2E1 | 1.5E2 | 2.4E1 | 1.0E-9 | 3.4E0 | 1.0E3 | 1.1E2 | 47 | 57 | 47 | 57 | 0.54 |
| kS | pg/ml | 8.1E2 | 9.3E2 | 9.4E2 | 1.0E3 | 5.9E2 | 6.0E2 | 8.2E1 | 2.5E2 | 3.2E3 | 3.0E3 | 47 | 57 | 47 | 57 | 0.56 |
| pS | ng/ml | 1.8E5 | 1.4E5 | 2.0E5 | 1.7E5 | 9.2E4 | 1.1E5 | 7.5E4 | 6.8E4 | 5.0E5 | 5.7E5 | 24 | 23 | 24 | 23 | 0.36 |
| rZ | ng/ml | 1.4E-3 | 2.2E-3 | 5.7E-3 | 1.2E-2 | 1.6E-2 | 2.2E-2 | 1.0E-9 | 1.0E-9 | 9.4E-2 | 1.1E-1 | 34 | 41 | 34 | 41 | 0.60 |
| rY | ng/ml | 6.4E-2 | 6.7E-2 | 3.1E-1 | 5.7E-1 | 1.1E0 | 3.1E0 | 1.6E-2 | 1.0E-9 | 6.3E0 | 2.0E1 | 34 | 41 | 34 | 41 | 0.51 |
| rX | ng/ml | 1.0E-9 | 1.0E-9 | 1.4E-1 | 8.9E-2 | 6.4E-1 | 4.8E-1 | 1.0E-9 | 1.0E-9 | 3.7E0 | 3.1E0 | 34 | 41 | 34 | 41 | 0.50 |
| lK | pg/ml | 7.2E1 | 4.4E1 | 1.5E2 | 1.3E2 | 1.8E2 | 1.6E2 | 7.6E0 | 1.0E-9 | 7.0E2 | 6.9E2 | 40 | 43 | 40 | 43 | 0.41 |
| lL | pg/ml | 2.0E3 | 1.4E3 | 3.7E3 | 2.3E3 | 7.0E3 | 2.1E3 | 7.5E1 | 1.2E2 | 4.2E4 | 7.7E3 | 40 | 44 | 40 | 44 | 0.42 |
| lM | pg/ml | 1.6E3 | 1.2E3 | 4.6E3 | 5.4E3 | 8.2E3 | 1.2E4 | 2.2E2 | 9.5E0 | 4.2E4 | 6.7E4 | 40 | 44 | 40 | 44 | 0.48 |
| lN | pg/ml | 1.0E-9 | 1.0E-9 | 3.7E0 | 3.0E0 | 8.0E0 | 6.2E0 | 1.0E-9 | 1.0E-9 | 4.3E1 | 2.4E1 | 40 | 44 | 40 | 44 | 0.49 |
| lO | pg/ml | 1.0E-9 | 1.0E-9 | 8.5E-1 | 6.1E0 | 5.4E0 | 2.8E1 | 1.0E-9 | 1.0E-9 | 3.4E1 | 1.4E2 | 40 | 43 | 40 | 43 | 0.51 |
| zA | ng/ml | 2.2E7 | 2.0E7 | 2.3E7 | 2.0E7 | 7.1E6 | 5.2E6 | 1.0E7 | 1.0E7 | 3.6E7 | 3.1E7 | 24 | 26 | 24 | 26 | 0.39 |
| rW | ng/ml | 8.5E-3 | 1.4E-2 | 2.4E-2 | 4.1E-2 | 3.9E-2 | 7.6E-2 | 1.0E-9 | 1.0E-9 | 1.7E-1 | 3.2E-1 | 25 | 21 | 25 | 21 | 0.60 |
| rV | ng/ml | 1.0E-9 | 1.0E-9 | 1.3E-2 | 1.4E-2 | 5.6E-2 | 3.5E-2 | 1.0E-9 | 1.0E-9 | 2.8E-1 | 1.5E-1 | 25 | 21 | 25 | 21 | 0.58 |
| rU | ng/ml | 4.2E-2 | 9.7E-2 | 1.9E-1 | 1.2E-1 | 5.3E-1 | 1.1E-1 | 1.0E-9 | 1.0E-9 | 2.7E0 | 4.0E-1 | 25 | 21 | 25 | 21 | 0.60 |
| rT | ng/ml | 6.3E0 | 4.4E0 | 6.7E0 | 7.2E0 | 4.3E0 | 5.5E0 | 6.5E-1 | 1.0E0 | 2.1E1 | 2.0E1 | 25 | 21 | 25 | 21 | 0.50 |
| rS | ng/ml | 3.6E0 | 5.8E0 | 5.5E0 | 1.3E1 | 5.7E0 | 1.9E1 | 1.8E0 | 1.1E0 | 2.5E1 | 7.0E1 | 25 | 21 | 25 | 21 | 0.63 |
| sC | pg/mL | 4.9E3 | 9.6E3 | 7.1E3 | 1.4E4 | 7.7E3 | 1.8E4 | 2.4E3 | 1.7E3 | 3.9E4 | 7.4E4 | 24 | 23 | 24 | 23 | 0.65 |
| yL | pg/ml | 3.2E1 | 2.8E1 | 3.9E1 | 9.0E1 | 3.3E1 | 2.8E2 | 5.6E0 | 9.1E0 | 1.8E2 | 1.4E3 | 24 | 25 | 24 | 25 | 0.44 |
| rP | ng/ml | 1.2E2 | 2.0E2 | 1.9E2 | 2.6E2 | 1.9E2 | 2.4E2 | 1.0E-9 | 1.2E1 | 5.0E2 | 8.0E2 | 25 | 21 | 25 | 21 | 0.56 |
| rQ | ng/ml | 1.0E-9 | 1.0E-9 | 1.0E-9 | 1.5E1 | 0.0E0 | 4.4E1 | 1.0E-9 | 1.0E-9 | 1.0E-9 | 1.7E2 | 25 | 21 | 25 | 21 | 0.57 |
| rO | ng/ml | 1.5E-2 | 2.5E-2 | 3.7E-2 | 4.1E-2 | 8.1E-2 | 4.8E-2 | 1.0E-9 | 1.0E-9 | 4.0E-1 | 1.9E-1 | 25 | 21 | 25 | 21 | 0.61 |
| rR | ng/ml | 1.0E-9 | 4.0E0 | 6.4E0 | 1.8E1 | 1.7E1 | 2.6E1 | 1.0E-9 | 1.0E-9 | 7.9E1 | 9.5E1 | 25 | 21 | 25 | 21 | 0.68 |
| rN | ng/ml | 7.0E-1 | 6.2E-1 | 8.6E-1 | 1.5E0 | 5.7E-1 | 2.8E0 | 5.1E-2 | 2.1E-1 | 2.3E0 | 1.3E1 | 25 | 21 | 25 | 21 | 0.47 |
| qO | pg/ml | 7.4E3 | 1.0E4 | 9.9E3 | 1.4E4 | 6.9E3 | 1.3E4 | 2.7E3 | 7.4E2 | 2.8E4 | 4.8E4 | 24 | 23 | 24 | 23 | 0.58 |
| qP | pg/ml | 3.3E2 | 3.4E2 | 3.6E2 | 4.7E2 | 2.0E2 | 3.5E2 | 7.0E1 | 1.1E2 | 8.3E2 | 1.5E3 | 24 | 23 | 24 | 23 | 0.55 |
| qQ | pg/ml | 1.5E1 | 1.5E1 | 2.3E1 | 2.5E1 | 5.6E1 | 5.4E1 | 1.0E-9 | 1.0E-9 | 2.8E2 | 2.6E2 | 24 | 23 | 24 | 23 | 0.50 |
| nW | pg/ml | 1.1E5 | 1.1E5 | 1.1E5 | 1.1E5 | 2.7E4 | 2.9E4 | 5.8E4 | 3.6E4 | 1.8E5 | 1.7E5 | 47 | 57 | 47 | 57 | 0.47 |
| nY | pg/ml | 2.2E3 | 2.5E3 | 2.6E3 | 2.8E3 | 1.7E3 | 1.5E3 | 5.1E2 | 6.3E2 | 1.0E4 | 8.1E3 | 47 | 57 | 47 | 57 | 0.56 |
| oO | pg/ml | 9.1E4 | 9.3E4 | 1.0E5 | 1.1E5 | 5.4E4 | 8.3E4 | 3.2E4 | 3.3E3 | 2.6E5 | 4.0E5 | 24 | 33 | 24 | 33 | 0.50 |
| oP | pg/ml | 1.4E5 | 1.3E5 | 1.6E5 | 1.6E5 | 6.9E4 | 1.1E5 | 4.9E4 | 2.4E4 | 3.1E5 | 5.7E5 | 24 | 33 | 24 | 33 | 0.46 |
| oQ | pg/ml | 3.4E3 | 3.1E3 | 3.5E3 | 5.0E3 | 2.1E3 | 6.0E3 | 1.1E3 | 7.7E2 | 1.1E4 | 3.2E4 | 24 | 33 | 24 | 33 | 0.53 |
| oE | pg/ml | 2.0E2 | 2.3E2 | 4.7E2 | 6.3E2 | 5.2E2 | 8.0E2 | 1.0E-9 | 1.0E-9 | 1.9E3 | 3.4E3 | 47 | 57 | 47 | 57 | 0.54 |
| oF | pg/ml | 1.1E4 | 1.9E4 | 2.3E4 | 3.6E4 | 2.9E4 | 4.7E4 | 4.3E2 | 5.1E2 | 1.5E5 | 2.5E5 | 47 | 57 | 47 | 57 | 0.58 |
| oH | pg/ml | 3.6E1 | 3.4E1 | 8.5E1 | 7.6E1 | 1.5E2 | 1.0E2 | 5.4E0 | 4.3E-1 | 8.6E2 | 4.8E2 | 47 | 57 | 47 | 57 | 0.47 |
| oK | pg/ml | 9.1E2 | 8.8E2 | 1.7E3 | 1.5E3 | 1.8E3 | 1.9E3 | 8.8E1 | 1.4E2 | 7.8E3 | 1.2E4 | 47 | 57 | 47 | 57 | 0.49 |
| oN | pg/ml | 5.5E2 | 5.7E2 | 1.2E3 | 8.2E2 | 2.8E3 | 8.9E2 | 1.6E2 | 1.1E2 | 1.8E4 | 5.3E3 | 47 | 57 | 47 | 57 | 0.54 |
| oW | pg/ml | 2.0E2 | 3.7E2 | 2.6E2 | 1.1E3 | 1.9E2 | 2.0E3 | 7.7E1 | 2.9E1 | 7.3E2 | 7.6E3 | 14 | 15 | 14 | 15 | 0.66 |
| oT | pg/ml | 2.8E2 | 3.0E2 | 3.2E2 | 3.5E2 | 1.5E2 | 2.1E2 | 1.0E2 | 1.3E2 | 7.4E2 | 7.9E2 | 14 | 15 | 14 | 15 | 0.53 |
| oV | pg/ml | 1.1E2 | 8.9E1 | 2.7E2 | 2.9E2 | 3.9E2 | 5.7E2 | 2.1E1 | 1.0E-9 | 1.4E3 | 2.2E3 | 14 | 15 | 14 | 15 | 0.47 |
| oD | pg/ml | 1.7E4 | 1.3E4 | 1.7E4 | 1.5E4 | 4.8E3 | 6.9E3 | 8.7E3 | 6.6E3 | 2.5E4 | 3.2E4 | 14 | 15 | 14 | 15 | 0.36 |
| uL | ng/ml | 3.4E1 | 3.7E1 | 5.2E1 | 4.1E1 | 5.9E1 | 1.9E1 | 1.5E1 | 1.4E1 | 2.9E2 | 8.0E1 | 24 | 23 | 24 | 23 | 0.51 |
| uO | ng/ml | 4.6E-1 | 3.4E-1 | 9.9E-1 | 7.3E-1 | 1.9E0 | 7.7E-1 | 1.0E-9 | 1.0E-9 | 9.3E0 | 2.1E0 | 24 | 23 | 24 | 23 | 0.49 |
| uM | ng/ml | 6.1E-1 | 5.0E-1 | 1.3E0 | 6.2E-1 | 2.6E0 | 6.1E-1 | 1.0E-9 | 1.0E-9 | 1.3E1 | 2.5E0 | 24 | 23 | 24 | 23 | 0.41 |
| uI | ng/ml | 7.4E-2 | 5.9E-2 | 1.0E-1 | 1.1E-1 | 1.1E-1 | 1.2E-1 | 1.6E-2 | 1.5E-2 | 5.8E-1 | 4.3E-1 | 24 | 22 | 24 | 22 | 0.46 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| uN | ng/ml | 1.5E1 | 1.6E1 | 1.7E1 | 1.8E1 | 6.2E0 | 8.3E0 | 8.0E0 | 9.9E0 | 3.0E1 | 4.1E1 | 24 | 23 | 24 | 23 | 0.53 |
| uG | ng/ml | 1.8E1 | 1.9E1 | 2.2E1 | 2.6E1 | 1.6E1 | 2.7E1 | 6.1E0 | 1.2E0 | 7.9E1 | 1.3E2 | 24 | 23 | 24 | 23 | 0.53 |
| uR | ng/ml | 2.5E0 | 1.7E0 | 3.2E0 | 2.5E0 | 2.6E0 | 2.0E0 | 1.1E0 | 7.5E-1 | 1.3E1 | 8.3E0 | 24 | 25 | 24 | 25 | 0.35 |
| uP | ng/ml | 2.0E0 | 2.4E0 | 2.3E0 | 2.7E0 | 1.0E0 | 1.2E0 | 1.2E0 | 9.3E-1 | 6.0E0 | 6.1E0 | 24 | 25 | 24 | 25 | 0.64 |
| uV | ng/ml | 1.1E-4 | 2.3E-3 | 2.0E-2 | 8.9E-3 | 4.5E-2 | 1.2E-2 | 1.0E-9 | 1.0E-9 | 2.0E-1 | 4.3E-2 | 24 | 25 | 24 | 25 | 0.49 |
| uT | ng/ml | 6.6E1 | 8.4E1 | 1.0E2 | 1.0E2 | 1.0E2 | 8.7E1 | 3.4E1 | 1.3E1 | 4.5E2 | 4.1E2 | 24 | 25 | 24 | 25 | 0.54 |
| uU | ng/ml | 1.6E0 | 1.7E0 | 1.9E0 | 2.4E0 | 1.3E0 | 3.7E0 | 6.0E-1 | 5.4E-1 | 6.0E0 | 2.0E1 | 24 | 25 | 24 | 25 | 0.52 |
| uW | ng/ml | 7.7E0 | 7.9E0 | 7.9E0 | 8.2E0 | 2.1E0 | 2.5E0 | 4.4E0 | 5.1E0 | 1.3E1 | 1.6E1 | 24 | 23 | 24 | 23 | 0.53 |
| vB | ng/ml | 3.0E0 | 3.2E0 | 3.4E0 | 3.4E0 | 2.3E0 | 2.2E0 | 5.9E-1 | 8.3E-1 | 1.0E1 | 1.0E1 | 24 | 23 | 24 | 23 | 0.51 |
| vC | ng/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 24 | 23 | 24 | 23 | 0.50 |
| uY | ng/ml | 5.7E-1 | 7.5E-1 | 8.7E-1 | 1.1E0 | 9.0E-1 | 1.0E0 | 6.8E-2 | 3.1E-1 | 3.8E0 | 4.4E0 | 24 | 23 | 24 | 23 | 0.66 |
| uZ | ng/ml | 5.8E-1 | 5.3E-1 | 6.8E-1 | 8.0E-1 | 5.9E-1 | 9.7E-1 | 1.0E-1 | 1.7E-1 | 3.0E0 | 4.9E0 | 24 | 23 | 24 | 23 | 0.52 |
| uX | ng/ml | 7.8E0 | 9.1E0 | 1.1E1 | 1.5E1 | 7.8E0 | 1.6E1 | 3.6E0 | 4.2E0 | 4.0E1 | 6.5E1 | 24 | 23 | 24 | 23 | 0.59 |
| vA | ng/ml | 6.2E-2 | 6.6E-2 | 7.0E-2 | 9.4E-2 | 3.1E-2 | 9.3E-2 | 3.2E-2 | 2.5E-2 | 1.5E-1 | 4.2E-1 | 24 | 23 | 24 | 23 | 0.52 |
| vH | ng/ml | 1.3E-1 | 8.5E-2 | 1.8E-1 | 1.9E-1 | 1.6E-1 | 3.8E-1 | 4.6E-2 | 2.0E-2 | 6.6E-1 | 1.9E0 | 24 | 23 | 24 | 23 | 0.34 |
| vI | ng/ml | 2.2E0 | 2.9E0 | 2.3E0 | 3.0E0 | 1.4E0 | 2.7E0 | 4.4E-1 | 6.3E-3 | 6.4E0 | 1.0E1 | 24 | 23 | 24 | 23 | 0.57 |
| vP | ng/ml | 3.4E2 | 3.1E2 | 3.7E2 | 5.5E2 | 2.6E2 | 5.2E2 | 7.0E1 | 6.7E1 | 1.0E3 | 2.4E3 | 24 | 25 | 24 | 25 | 0.60 |
| vT | ng/ml | 7.1E1 | 8.2E1 | 8.1E1 | 8.8E1 | 4.2E1 | 3.8E1 | 4.1E1 | 4.6E1 | 2.4E2 | 1.8E2 | 24 | 25 | 24 | 25 | 0.58 |
| vU | ng/ml | 1.0E-9 | 1.0E-9 | 2.3E1 | 2.1E1 | 3.7E1 | 3.4E1 | 1.0E-9 | 1.0E-9 | 1.2E2 | 1.1E2 | 24 | 25 | 24 | 25 | 0.48 |
| vQ | ng/ml | 3.9E2 | 4.5E2 | 3.9E2 | 4.2E2 | 1.4E2 | 1.8E2 | 7.2E1 | 1.2E2 | 7.0E2 | 8.4E2 | 24 | 25 | 24 | 25 | 0.54 |
| vO | ng/ml | 1.8E3 | 1.7E3 | 1.8E3 | 1.8E3 | 4.5E2 | 4.9E2 | 1.1E3 | 1.0E3 | 2.9E3 | 3.2E3 | 24 | 25 | 24 | 25 | 0.48 |
| vS | ng/ml | 1.3E3 | 1.3E3 | 1.3E3 | 1.2E3 | 3.7E2 | 5.6E2 | 1.0E-9 | 1.0E-9 | 2.1E3 | 2.0E3 | 24 | 25 | 24 | 25 | 0.50 |
| vV | ng/ml | 7.4E2 | 9.0E2 | 8.0E2 | 1.2E3 | 5.5E2 | 1.1E3 | 1.1E2 | 1.0E2 | 1.9E3 | 4.6E3 | 24 | 25 | 24 | 25 | 0.58 |
| vW | ng/ml | 1.1E2 | 1.1E2 | 1.4E2 | 1.9E2 | 7.6E1 | 1.6E2 | 4.3E1 | 6.0E1 | 2.9E2 | 7.7E2 | 24 | 25 | 24 | 25 | 0.56 |
| pF | pg/ml | 7.9E-1 | 5.2E-1 | 1.0E0 | 2.2E0 | 1.4E0 | 1.1E1 | 2.3E-2 | 1.0E-9 | 9.4E0 | 8.7E1 | 47 | 57 | 47 | 57 | 0.43 |
| pH | ng/ml | 6.6E0 | 8.9E0 | 8.2E0 | 1.1E1 | 4.4E0 | 5.7E0 | 3.0E0 | 1.2E0 | 1.8E1 | 2.3E1 | 14 | 15 | 14 | 15 | 0.66 |
| pI | ng/ml | 7.4E1 | 6.7E1 | 6.9E1 | 7.6E1 | 3.4E1 | 5.5E1 | 2.6E1 | 2.3E1 | 1.5E2 | 2.0E2 | 14 | 15 | 14 | 15 | 0.48 |
| pK | ng/ml | 3.6E-1 | 5.5E-1 | 3.8E-1 | 5.6E-1 | 1.7E-1 | 2.0E-1 | 1.7E-1 | 2.7E-1 | 7.7E-1 | 8.6E-1 | 14 | 15 | 14 | 15 | 0.77 |

Figure 34.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ad | ug/mL | 4.8E-2 | 9.7E-2 | 7.5E-2 | 1.2E0 | 8.8E-2 | 3.0E0 | 6.8E-4 | 7.8E-4 | 5.4E-1 | 8.5E0 | 166 | 8 | 166 | 8 | 0.66 |
| Af | ng/mL | 1.2E0 | 2.7E0 | 1.0E1 | 6.9E0 | 4.5E1 | 8.6E0 | 1.7E-3 | 1.5E-1 | 5.3E2 | 2.3E1 | 166 | 8 | 166 | 8 | 0.55 |
| Aj | ug/mL | 1.0E0 | 5.7E0 | 2.2E0 | 3.7E0 | 2.4E0 | 2.9E0 | 1.5E-3 | 1.3E-3 | 6.1E0 | 5.8E0 | 166 | 8 | 166 | 8 | 0.62 |
| Al | mg/mL | 8.3E-5 | 3.2E-4 | 2.6E-4 | 5.2E-4 | 4.4E-4 | 5.4E-4 | 4.3E-6 | 7.8E-6 | 1.8E-3 | 1.5E-3 | 166 | 8 | 166 | 8 | 0.67 |
| An | U/mL | 6.0E1 | 2.5E2 | 2.0E2 | 1.3E3 | 5.4E2 | 2.6E3 | 2.8E-1 | 2.7E1 | 5.5E3 | 7.8E3 | 166 | 8 | 166 | 8 | 0.75 |
| Ao | pg/mL | 9.4E1 | 3.7E2 | 5.3E2 | 8.4E2 | 3.6E3 | 1.5E3 | 1.5E0 | 4.1E0 | 3.9E4 | 4.5E3 | 166 | 8 | 166 | 8 | 0.67 |
| Ap | ng/mL | 3.3E1 | 5.1E1 | 4.9E1 | 8.2E1 | 5.4E1 | 9.3E1 | 2.0E0 | 4.4E0 | 3.3E2 | 2.4E2 | 166 | 8 | 166 | 8 | 0.58 |
| Ar | ng/mL | 6.8E-1 | 2.4E0 | 2.9E0 | 8.6E0 | 6.7E0 | 1.7E1 | 3.4E-3 | 2.2E-1 | 5.1E1 | 5.0E1 | 166 | 8 | 166 | 8 | 0.67 |
| As | ng/mL | 8.6E-3 | 1.1E-2 | 1.2E-2 | 1.6E-1 | 1.7E-2 | 4.4E-1 | 1.7E-3 | 1.7E-3 | 1.3E-1 | 1.2E0 | 166 | 8 | 166 | 8 | 0.53 |
| Aw | pg/mL | 1.6E1 | 2.5E1 | 1.7E1 | 2.6E1 | 5.6E0 | 1.2E1 | 2.9E-2 | 1.3E1 | 4.2E1 | 5.1E1 | 166 | 8 | 166 | 8 | 0.77 |
| Ax | ng/mL | 2.1E0 | 4.4E0 | 2.5E1 | 1.4E2 | 9.2E1 | 3.0E2 | 1.2E-2 | 1.0E-4 | 7.7E2 | 8.5E2 | 166 | 8 | 166 | 8 | 0.58 |
| Ba | ng/mL | 8.3E1 | 7.5E2 | 6.2E2 | 2.4E3 | 1.5E3 | 5.0E3 | 1.1E0 | 4.1E0 | 8.1E3 | 1.5E4 | 166 | 8 | 166 | 8 | 0.76 |
| Bb | ng/mL | 4.1E0 | 1.0E1 | 7.2E0 | 1.2E1 | 8.9E0 | 8.6E0 | 4.1E-3 | 3.5E-1 | 4.9E1 | 2.5E1 | 166 | 8 | 166 | 8 | 0.68 |
| Bc | ng/mL | 3.7E1 | 7.6E1 | 1.2E2 | 3.2E2 | 2.2E2 | 4.3E2 | 4.9E-1 | 2.9E0 | 1.2E3 | 1.0E3 | 166 | 8 | 166 | 8 | 0.62 |
| Bg | ng/mL | 1.1E-1 | 2.4E0 | 4.0E0 | 5.1E1 | 1.7E1 | 1.4E2 | 5.3E-4 | 3.1E-2 | 1.5E2 | 4.0E2 | 166 | 8 | 166 | 8 | 0.75 |
| Bn | ng/mL | 5.6E-2 | 8.0E-1 | 1.2E0 | 8.0E0 | 2.0E0 | 2.0E1 | 5.6E-2 | 5.6E-2 | 8.6E0 | 5.8E1 | 166 | 8 | 166 | 8 | 0.61 |
| Bo | ng/mL | 1.3E1 | 1.5E1 | 1.5E1 | 1.7E1 | 1.1E1 | 1.7E1 | 1.6E-2 | 1.6E-2 | 5.0E1 | 5.3E1 | 166 | 8 | 166 | 8 | 0.50 |
| Ch | uIU/mL | 9.8E-1 | 1.6E0 | 2.8E1 | 1.5E2 | 1.6E2 | 4.2E2 | 3.4E-3 | 9.2E-1 | 1.8E3 | 1.2E3 | 166 | 8 | 166 | 8 | 0.67 |
| Co | pg/mL | 4.6E1 | 1.5E2 | 2.2E2 | 4.4E2 | 1.3E3 | 6.9E2 | 1.5E-1 | 8.8E0 | 1.7E4 | 2.1E3 | 166 | 8 | 166 | 8 | 0.72 |
| Cp | ng/mL | 2.2E1 | 6.0E1 | 2.8E1 | 2.0E2 | 2.2E1 | 4.4E2 | 6.0E-1 | 1.1E1 | 1.4E2 | 1.3E3 | 166 | 8 | 166 | 8 | 0.68 |
| Cq | ng/mL | 2.9E-2 | 1.1E-1 | 1.2E-1 | 6.3E0 | 4.7E-1 | 1.7E1 | 8.0E-4 | 3.0E-2 | 5.1E0 | 4.9E1 | 166 | 8 | 166 | 8 | 0.77 |
| Cs | ng/mL | 6.2E1 | 1.3E2 | 4.5E2 | 1.3E3 | 1.7E3 | 2.3E3 | 8.3E-1 | 5.7E0 | 1.8E4 | 5.1E3 | 166 | 8 | 166 | 8 | 0.59 |
| Ct | ng/mL | 3.2E-1 | 7.6E0 | 3.8E1 | 1.1E2 | 1.2E2 | 2.0E2 | 1.1E-4 | 4.4E-2 | 6.2E2 | 4.7E2 | 166 | 8 | 166 | 8 | 0.65 |
| Cu | ng/mL | 2.6E-1 | 2.0E0 | 6.3E-1 | 9.6E0 | 1.9E0 | 2.3E1 | 1.9E-2 | 1.7E-2 | 2.1E1 | 6.6E1 | 166 | 8 | 166 | 8 | 0.74 |
| Cv | ng/mL | 5.1E0 | 1.0E1 | 2.6E1 | 9.9E1 | 6.2E1 | 1.8E2 | 2.0E-2 | 1.0E-1 | 5.3E2 | 4.7E2 | 166 | 8 | 166 | 8 | 0.57 |
| Cw | mIU/mL | 3.5E-2 | 7.4E-2 | 4.3E-2 | 9.1E-1 | 3.5E-2 | 2.4E0 | 8.9E-4 | 1.1E-2 | 2.3E-1 | 6.8E0 | 166 | 8 | 166 | 8 | 0.74 |
| Cx | ng/mL | 7.3E-1 | 7.6E-3 | 5.0E1 | 5.7E1 | 9.8E1 | 1.1E2 | 9.3E-5 | 9.3E-5 | 3.9E2 | 2.8E2 | 166 | 8 | 166 | 8 | 0.42 |
| Db | ug/mL | 7.5E0 | 1.1E1 | 8.6E0 | 1.2E1 | 7.5E0 | 8.8E0 | 4.5E-1 | 4.0E0 | 5.9E1 | 3.1E1 | 166 | 8 | 166 | 8 | 0.62 |
| Dc | nmol/L | 2.1E-2 | 1.7E-1 | 6.6E-2 | 2.1E0 | 1.8E-1 | 4.9E0 | 5.2E-6 | 2.1E-3 | 1.6E0 | 1.4E1 | 166 | 8 | 166 | 8 | 0.78 |
| Dd | ug/mL | 7.0E-2 | 4.3E-1 | 1.8E-1 | 7.8E-1 | 2.7E-1 | 1.2E0 | 4.8E-4 | 6.4E-3 | 1.6E0 | 3.6E0 | 166 | 8 | 166 | 8 | 0.76 |
| De | ng/mL | 3.4E-3 | 2.2E-1 | 7.3E-2 | 3.4E-1 | 1.3E-1 | 4.1E-1 | 3.4E-3 | 3.4E-3 | 9.2E-1 | 1.1E0 | 166 | 8 | 166 | 8 | 0.72 |
| Dg | ng/mL | 3.6E1 | 5.2E1 | 4.8E1 | 5.8E1 | 4.1E1 | 3.9E1 | 7.1E-1 | 1.9E0 | 1.9E2 | 1.2E2 | 166 | 8 | 166 | 8 | 0.60 |
| Di | pg/mL | 2.0E0 | 4.4E0 | 2.4E0 | 4.2E0 | 2.1E0 | 2.5E0 | 1.8E-1 | 1.8E-1 | 1.3E1 | 9.0E0 | 166 | 8 | 166 | 8 | 0.74 |
| Dk | uIU/mL | 1.4E-2 | 1.8E-1 | 4.9E-2 | 3.2E-1 | 1.5E-1 | 3.8E-1 | 1.1E-4 | 1.1E-4 | 1.6E0 | 9.8E-1 | 166 | 8 | 166 | 8 | 0.65 |
| Dl | ng/mL | 2.0E2 | 3.5E2 | 2.9E2 | 4.9E2 | 2.7E2 | 5.2E2 | 5.5E0 | 4.4E0 | 1.3E3 | 1.6E3 | 166 | 8 | 166 | 8 | 0.60 |
| Ef | ng/ml | 9.4E-2 | 6.4E0 | 6.9E-1 | 5.0E0 | 1.6E0 | 4.2E0 | 5.7E-4 | 4.6E-3 | 1.0E1 | 9.9E0 | 140 | 7 | 140 | 7 | 0.78 |
| Wm | % | 8.5E-2 | 2.5E0 | 2.1E1 | 3.1E1 | 1.3E2 | 6.4E1 | 5.4E-2 | 8.5E-2 | 1.0E3 | 1.9E2 | 149 | 10 | 149 | 10 | 0.68 |
| Po | pg/ml | 2.6E-1 | 2.8E1 | 8.3E0 | 5.9E1 | 2.6E1 | 7.3E1 | 8.0E-3 | 2.6E-2 | 2.7E2 | 2.2E2 | 339 | 15 | 339 | 15 | 0.79 |
| Et | ng/ml | 1.4E3 | 4.0E3 | 1.6E3 | 3.5E3 | 1.1E3 | 1.4E3 | 7.5E1 | 5.9E2 | 4.8E3 | 5.0E3 | 338 | 15 | 338 | 15 | 0.84 |
| Ex | ng/ml | 7.6E-2 | 1.6E-1 | 1.8E-1 | 9.6E-1 | 3.1E-1 | 1.5E0 | 3.5E-5 | 5.8E-2 | 2.2E0 | 4.1E0 | 98 | 7 | 98 | 7 | 0.73 |
| Fp | ng/ml | 1.3E1 | 3.1E1 | 2.3E1 | 3.8E1 | 2.7E1 | 3.2E1 | 6.0E-3 | 2.3E0 | 1.3E2 | 1.3E2 | 341 | 15 | 341 | 15 | 0.68 |
| Fr | ng/ml | 3.5E4 | 6.0E5 | 1.1E5 | 5.1E5 | 1.7E5 | 3.1E5 | 1.9E2 | 7.0E3 | 8.4E5 | 8.4E5 | 346 | 17 | 346 | 17 | 0.84 |
| Fw | pg/ml | 2.7E0 | 9.4E0 | 4.6E1 | 5.9E1 | 2.6E2 | 1.2E2 | 1.2E-1 | 1.2E-1 | 3.0E3 | 3.3E2 | 142 | 7 | 142 | 7 | 0.67 |
| Gl | pg/ml | 8.9E3 | 2.5E4 | 1.2E4 | 2.3E4 | 9.5E3 | 1.0E4 | 9.1E1 | 8.7E3 | 3.2E4 | 3.2E4 | 142 | 7 | 142 | 7 | 0.80 |
| Nm | pg/ml | 1.3E4 | 4.5E4 | 3.4E4 | 1.3E5 | 8.2E4 | 2.2E5 | 1.0E-9 | 1.0E-9 | 9.6E5 | 8.2E5 | 342 | 15 | 342 | 15 | 0.70 |
| Nn | pg/ml | 1.5E2 | 5.3E3 | 1.4E3 | 4.0E4 | 6.6E3 | 8.1E4 | 1.0E-9 | 1.0E-9 | 9.5E4 | 3.1E5 | 342 | 15 | 342 | 15 | 0.81 |
| No | pg/ml | 1.5E1 | 5.1E1 | 3.2E1 | 2.5E2 | 5.7E1 | 3.2E2 | 1.0E-9 | 1.6E0 | 5.6E2 | 9.1E2 | 342 | 15 | 342 | 15 | 0.71 |
| Nq | pg/ml | 1.9E0 | 5.5E1 | 1.8E1 | 1.6E2 | 6.5E1 | 2.1E2 | 1.0E-9 | 1.0E-9 | 6.7E2 | 6.3E2 | 342 | 15 | 342 | 15 | 0.78 |
| Nr | pg/ml | 1.5E0 | 1.5E1 | 2.1E1 | 1.8E2 | 7.5E1 | 3.7E2 | 1.0E-9 | 1.0E-9 | 9.8E2 | 1.4E3 | 342 | 15 | 342 | 15 | 0.73 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ns | pg/ml | 1.0E-9 | 1.0E-9 | 7.8E0 | 4.8E-1 | 6.5E1 | 1.3E0 | 1.0E-9 | 1.0E-9 | 1.1E3 | 3.6E0 | 342 | 15 | 342 | 15 | 0.53 |
| Nt | pg/ml | 1.0E2 | 2.2E2 | 1.3E2 | 4.1E2 | 9.9E1 | 4.8E2 | 9.8E-1 | 7.5E1 | 8.8E2 | 1.7E3 | 342 | 15 | 342 | 15 | 0.74 |
| Nu | pg/ml | 1.7E1 | 1.0E2 | 5.5E1 | 1.2E2 | 9.1E1 | 1.0E2 | 1.0E-9 | 1.0E-9 | 6.3E2 | 3.7E2 | 342 | 15 | 342 | 15 | 0.74 |
| Lu | pg/ml | 1.0E4 | 6.2E3 | 1.6E4 | 7.0E3 | 4.0E4 | 4.6E3 | 7.7E2 | 5.2E2 | 5.6E5 | 1.7E4 | 342 | 15 | 342 | 15 | 0.36 |
| Lv | pg/ml | 1.0E-9 | 6.7E1 | 1.4E1 | 7.2E1 | 2.8E1 | 6.4E1 | 1.0E-9 | 1.0E-9 | 2.6E2 | 1.9E2 | 342 | 15 | 342 | 15 | 0.78 |
| Lw | pg/ml | 1.0E-9 | 1.0E-9 | 4.7E-1 | 1.7E1 | 5.0E0 | 4.6E1 | 1.0E-9 | 1.0E-9 | 8.0E1 | 1.8E2 | 342 | 15 | 342 | 15 | 0.62 |
| Lx | pg/ml | 1.0E-9 | 8.7E2 | 1.7E2 | 2.4E3 | 5.5E2 | 5.6E3 | 1.0E-9 | 1.0E-9 | 6.2E3 | 2.2E4 | 342 | 15 | 342 | 15 | 0.81 |
| Ly | pg/ml | 1.0E-9 | 1.0E-9 | 9.2E0 | 7.5E0 | 1.8E1 | 1.6E1 | 1.0E-9 | 1.0E-9 | 1.1E2 | 6.0E1 | 342 | 15 | 342 | 15 | 0.51 |
| Lz | pg/ml | 1.0E-9 | 1.0E-9 | 3.2E0 | 8.7E0 | 2.6E1 | 2.0E1 | 1.0E-9 | 1.0E-9 | 4.3E2 | 6.2E1 | 342 | 15 | 342 | 15 | 0.60 |
| Ma | pg/ml | 3.9E2 | 3.8E3 | 2.1E3 | 1.0E4 | 5.4E3 | 1.5E4 | 1.0E-9 | 2.4E1 | 6.5E4 | 5.2E4 | 342 | 15 | 342 | 15 | 0.76 |
| Mb | pg/ml | 2.5E1 | 2.2E1 | 3.2E1 | 2.8E1 | 1.8E1 | 1.7E1 | 9.2E0 | 4.1E0 | 2.1E2 | 5.8E1 | 342 | 15 | 342 | 15 | 0.38 |
| Mc | pg/ml | 1.0E-9 | 1.0E-9 | 5.6E-2 | 1.0E-9 | 7.4E-1 | 0.0E0 | 1.0E-9 | 1.0E-9 | 1.3E1 | 1.0E-9 | 342 | 15 | 342 | 15 | 0.49 |
| Md | pg/ml | 1.0E-9 | 1.0E-9 | 5.7E-1 | 2.7E0 | 5.2E0 | 7.6E0 | 1.0E-9 | 1.0E-9 | 6.8E1 | 2.9E1 | 342 | 15 | 342 | 15 | 0.63 |
| Me | pg/ml | 3.2E1 | 2.9E1 | 3.1E1 | 3.4E1 | 2.4E1 | 4.4E1 | 1.0E-9 | 1.0E-9 | 3.2E2 | 1.8E2 | 342 | 15 | 342 | 15 | 0.44 |
| Mf | pg/ml | 1.0E-9 | 1.0E-9 | 6.3E-1 | 7.4E-1 | 3.9E0 | 1.7E0 | 1.0E-9 | 1.0E-9 | 5.6E1 | 5.0E0 | 342 | 15 | 342 | 15 | 0.59 |
| Mg | pg/ml | 9.4E-1 | 1.4E1 | 6.1E0 | 2.6E1 | 1.2E1 | 4.2E1 | 1.0E-9 | 1.0E-9 | 9.2E1 | 1.5E2 | 342 | 15 | 342 | 15 | 0.64 |
| Mh | pg/ml | 1.0E-9 | 4.0E-2 | 1.1E0 | 2.5E0 | 7.6E0 | 5.5E0 | 1.0E-9 | 1.0E-9 | 1.1E2 | 1.8E1 | 342 | 15 | 342 | 15 | 0.65 |
| Mi | pg/ml | 1.0E-9 | 1.0E-9 | 1.0E0 | 6.7E1 | 8.4E0 | 1.5E2 | 1.0E-9 | 1.0E-9 | 1.2E2 | 5.2E2 | 342 | 15 | 342 | 15 | 0.69 |
| Mj | pg/ml | 1.0E-9 | 1.0E-9 | 5.6E0 | 4.2E1 | 3.1E1 | 6.8E1 | 1.0E-9 | 1.0E-9 | 4.6E2 | 2.1E2 | 342 | 15 | 342 | 15 | 0.67 |
| Mk | pg/ml | 1.8E0 | 5.3E0 | 1.5E1 | 4.9E1 | 9.2E1 | 1.3E2 | 1.0E-9 | 1.0E-9 | 1.3E3 | 5.0E2 | 342 | 15 | 342 | 15 | 0.61 |
| Ml | pg/ml | 1.0E-9 | 1.0E-9 | 1.0E1 | 3.5E1 | 1.2E2 | 1.3E2 | 1.0E-9 | 1.0E-9 | 2.1E3 | 5.1E2 | 342 | 15 | 342 | 15 | 0.50 |
| Mm | pg/ml | 5.4E2 | 2.6E3 | 1.0E3 | 3.1E3 | 1.3E3 | 2.8E3 | 1.0E-9 | 1.0E-9 | 1.0E4 | 1.0E4 | 342 | 15 | 342 | 15 | 0.74 |
| Mn | pg/ml | 5.7E0 | 1.5E1 | 1.1E1 | 1.7E1 | 2.5E1 | 1.2E1 | 1.0E-9 | 1.1E0 | 3.5E2 | 5.1E1 | 342 | 15 | 342 | 15 | 0.75 |
| Mp | pg/ml | 1.0E-9 | 3.0E1 | 1.2E1 | 2.4E2 | 4.3E1 | 6.0E2 | 1.0E-9 | 1.0E-9 | 6.3E2 | 2.4E3 | 342 | 15 | 342 | 15 | 0.80 |
| Mq | pg/ml | 1.0E-9 | 1.0E-9 | 2.1E0 | 1.8E1 | 1.3E1 | 3.3E1 | 1.0E-9 | 1.0E-9 | 1.8E2 | 1.0E2 | 342 | 15 | 342 | 15 | 0.60 |
| Mr | pg/ml | 1.0E-9 | 1.5E1 | 2.3E1 | 5.1E2 | 1.3E2 | 1.0E3 | 1.0E-9 | 1.0E-9 | 1.5E3 | 3.4E3 | 342 | 15 | 342 | 15 | 0.68 |
| Ms | pg/ml | 3.3E2 | 1.9E2 | 4.8E2 | 2.8E2 | 5.8E2 | 3.2E2 | 1.0E-9 | 1.0E-9 | 4.7E3 | 9.8E2 | 342 | 15 | 342 | 15 | 0.39 |
| Mt | pg/ml | 2.2E-1 | 7.3E1 | 8.0E0 | 2.8E2 | 4.3E1 | 8.2E2 | 1.0E-9 | 1.0E-9 | 7.1E2 | 3.2E3 | 342 | 15 | 342 | 15 | 0.85 |
| Mu | pg/ml | 1.0E-9 | 4.4E0 | 1.4E0 | 9.1E0 | 1.4E1 | 1.4E1 | 1.0E-9 | 1.0E-9 | 2.3E2 | 4.8E1 | 342 | 15 | 342 | 15 | 0.80 |
| Mv | pg/ml | 1.0E-9 | 1.4E2 | 5.9E1 | 3.4E2 | 3.3E2 | 3.8E2 | 1.0E-9 | 1.0E-9 | 4.5E3 | 9.2E2 | 342 | 15 | 342 | 15 | 0.73 |
| Mw | pg/ml | 3.5E1 | 1.0E3 | 2.5E2 | 1.6E3 | 1.3E3 | 1.8E3 | 1.0E-9 | 1.0E-9 | 1.8E4 | 5.3E3 | 342 | 15 | 342 | 15 | 0.83 |
| Mx | pg/ml | 1.0E-9 | 6.0E-2 | 3.5E-1 | 2.1E0 | 1.9E0 | 5.1E0 | 1.0E-9 | 1.0E-9 | 3.2E1 | 2.0E1 | 342 | 15 | 342 | 15 | 0.69 |
| My | pg/ml | 1.0E-9 | 2.3E2 | 3.2E2 | 4.9E2 | 2.4E3 | 6.5E2 | 1.0E-9 | 1.0E-9 | 3.9E4 | 1.9E3 | 342 | 15 | 342 | 15 | 0.76 |
| Mz | pg/ml | 1.1E1 | 7.7E1 | 2.6E1 | 2.8E2 | 5.0E1 | 5.6E2 | 1.0E-9 | 1.0E-9 | 5.5E2 | 1.9E3 | 342 | 15 | 342 | 15 | 0.76 |
| Na | pg/ml | 1.0E-9 | 1.0E-9 | 6.4E-1 | 4.0E0 | 1.9E0 | 1.1E1 | 1.0E-9 | 1.0E-9 | 9.6E0 | 4.2E1 | 342 | 15 | 342 | 15 | 0.60 |
| Nb | pg/ml | 2.1E0 | 8.1E0 | 3.5E0 | 3.1E1 | 9.9E0 | 5.7E1 | 1.0E-9 | 1.0E-9 | 1.5E2 | 2.1E2 | 342 | 15 | 342 | 15 | 0.73 |
| Nc | pg/ml | 3.4E2 | 2.3E1 | 5.2E2 | 2.5E2 | 7.4E2 | 4.1E2 | 1.0E-9 | 1.0E-9 | 9.2E3 | 1.3E3 | 342 | 15 | 342 | 15 | 0.36 |
| Nd | pg/ml | 2.7E1 | 3.9E1 | 2.7E1 | 1.8E2 | 6.8E1 | 5.4E2 | 1.0E-9 | 1.0E-9 | 1.2E3 | 2.1E3 | 342 | 15 | 342 | 15 | 0.61 |
| Ne | pg/ml | 4.2E2 | 3.2E2 | 5.2E2 | 4.9E2 | 5.4E2 | 8.9E2 | 1.0E-9 | 1.0E-9 | 7.0E3 | 3.6E3 | 342 | 15 | 342 | 15 | 0.38 |
| Nf | pg/ml | 1.0E-9 | 1.0E-9 | 2.3E0 | 2.0E1 | 8.6E0 | 4.3E1 | 1.0E-9 | 1.0E-9 | 8.2E1 | 1.3E2 | 342 | 15 | 342 | 15 | 0.58 |
| Ng | pg/ml | 1.1E1 | 5.3E1 | 9.0E1 | 9.7E1 | 1.8E2 | 1.2E2 | 1.0E-9 | 1.0E-9 | 1.6E3 | 4.2E2 | 342 | 15 | 342 | 15 | 0.56 |
| Nh | pg/ml | 6.2E1 | 3.2E1 | 8.0E1 | 6.8E1 | 7.3E1 | 1.2E2 | 1.0E-9 | 4.1E0 | 5.6E2 | 5.1E2 | 342 | 15 | 342 | 15 | 0.33 |
| Ni | pg/ml | 1.0E-9 | 1.0E-9 | 8.1E1 | 1.7E2 | 1.3E2 | 2.9E2 | 1.0E-9 | 1.0E-9 | 7.1E2 | 1.1E3 | 342 | 15 | 342 | 15 | 0.52 |
| Nj | pg/ml | 7.2E0 | 4.4E0 | 1.1E1 | 7.2E0 | 1.2E1 | 7.4E0 | 1.0E-9 | 1.0E-9 | 6.9E1 | 2.2E1 | 342 | 15 | 342 | 15 | 0.40 |
| Nk | pg/ml | 1.8E1 | 4.6E0 | 3.3E1 | 1.7E1 | 3.9E1 | 2.4E1 | 1.0E-9 | 1.0E-9 | 2.0E2 | 6.9E1 | 342 | 15 | 342 | 15 | 0.39 |
| Nl | pg/ml | 4.3E1 | 1.5E1 | 5.7E1 | 3.7E1 | 7.5E1 | 5.4E1 | 1.0E-9 | 1.0E-9 | 1.1E3 | 1.8E2 | 342 | 15 | 342 | 15 | 0.34 |
| Hq | pg/ml | 1.2E0 | 1.5E1 | 1.5E2 | 2.1E2 | 1.9E3 | 7.1E2 | 1.0E-9 | 1.0E-9 | 2.8E4 | 2.8E3 | 340 | 15 | 340 | 15 | 0.74 |
| Hr | pg/ml | 8.9E1 | 2.8E2 | 6.2E2 | 1.4E3 | 1.3E3 | 2.4E3 | 1.0E-9 | 1.0E-9 | 1.2E4 | 8.9E3 | 340 | 15 | 340 | 15 | 0.62 |
| Hu | pg/ml | 5.4E0 | 4.5E2 | 4.6E3 | 1.4E3 | 4.0E4 | 2.1E3 | 1.0E-9 | 1.0E-9 | 6.3E5 | 6.5E3 | 340 | 15 | 340 | 15 | 0.70 |
| Hv | pg/ml | 1.4E0 | 3.0E0 | 2.9E0 | 6.7E1 | 1.0E1 | 2.3E2 | 1.0E-9 | 1.0E-9 | 1.6E2 | 8.9E2 | 340 | 15 | 340 | 15 | 0.70 |
| Hw | pg/ml | 6.1E0 | 1.5E1 | 1.5E1 | 6.9E2 | 4.5E1 | 2.4E3 | 1.0E-9 | 4.6E-1 | 6.4E2 | 9.4E3 | 340 | 15 | 340 | 15 | 0.65 |
| Hx | pg/ml | 8.6E0 | 3.8E1 | 5.2E1 | 1.8E2 | 5.0E2 | 3.7E2 | 1.0E-9 | 3.9E0 | 9.3E3 | 1.3E3 | 340 | 15 | 340 | 15 | 0.77 |
| Ih | ng/ml | 6.3E1 | 3.3E2 | 2.5E2 | 6.9E2 | 4.4E2 | 7.9E2 | 1.0E-9 | 2.4E0 | 3.6E3 | 2.8E3 | 341 | 15 | 341 | 15 | 0.70 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ii | ng/ml | 7.9E1 | 2.9E2 | 2.0E2 | 9.0E2 | 4.6E2 | 1.4E3 | 7.3E-1 | 7.5E-1 | 5.2E3 | 4.5E3 | 341 | 15 | 341 | 15 | 0.68 |
| Ij | ng/ml | 7.8E1 | 1.7E2 | 1.8E2 | 1.9E3 | 5.7E2 | 6.2E3 | 2.8E0 | 9.5E0 | 6.4E3 | 2.4E4 | 337 | 15 | 337 | 15 | 0.75 |
| Ik | ng/ml | 1.1E1 | 2.0E1 | 1.3E3 | 1.8E2 | 1.2E4 | 3.8E2 | 5.9E-1 | 6.2E-1 | 1.2E5 | 1.5E3 | 337 | 15 | 337 | 15 | 0.59 |
| Il | ng/ml | 3.3E2 | 8.0E2 | 1.3E3 | 3.9E3 | 2.8E3 | 5.1E3 | 1.0E-9 | 1.9E-1 | 1.2E4 | 1.2E4 | 335 | 15 | 335 | 15 | 0.68 |
| Im | ng/ml | 2.1E2 | 7.0E2 | 4.6E2 | 1.5E3 | 1.0E3 | 2.0E3 | 1.4E1 | 2.2E1 | 1.5E4 | 6.2E3 | 337 | 15 | 337 | 15 | 0.76 |
| In | ng/ml | 3.5E0 | 2.2E0 | 2.0E1 | 3.6E2 | 8.9E1 | 1.2E3 | 1.0E-9 | 1.0E-9 | 1.1E3 | 4.5E3 | 341 | 15 | 341 | 15 | 0.49 |
| Io | ng/ml | 9.5E3 | 1.0E4 | 2.0E4 | 1.2E4 | 4.7E4 | 1.1E4 | 1.0E-9 | 1.0E3 | 7.1E5 | 3.3E4 | 341 | 15 | 341 | 15 | 0.49 |
| Ip | ng/ml | 1.0E1 | 4.3E1 | 2.1E1 | 4.2E1 | 2.6E1 | 1.7E1 | 1.0E-9 | 9.8E0 | 2.3E2 | 7.1E1 | 341 | 15 | 341 | 15 | 0.80 |
| Iq | ug/ml | 1.0E-1 | 3.2E-1 | 4.1E1 | 1.9E1 | 7.4E2 | 5.6E1 | 1.0E-9 | 1.0E-9 | 1.4E4 | 2.2E2 | 341 | 15 | 341 | 15 | 0.71 |
| Ir | ug/ml | 3.7E-1 | 1.2E0 | 4.5E0 | 4.7E1 | 3.2E1 | 9.9E1 | 1.0E-9 | 3.5E-2 | 5.1E2 | 3.7E2 | 340 | 15 | 340 | 15 | 0.77 |
| Is | ng/ml | 2.0E0 | 3.5E1 | 9.0E0 | 5.4E1 | 3.4E1 | 6.9E1 | 1.0E-9 | 4.3E-1 | 5.5E2 | 2.6E2 | 341 | 15 | 341 | 15 | 0.83 |
| It | ng/ml | 2.1E0 | 6.2E0 | 2.2E1 | 5.5E1 | 1.0E2 | 1.7E2 | 1.0E-9 | 1.0E-9 | 1.1E3 | 6.8E2 | 341 | 15 | 341 | 15 | 0.64 |
| Iu | ng/ml | 1.6E2 | 1.0E3 | 1.2E3 | 6.0E3 | 3.9E3 | 9.8E3 | 1.0E-9 | 1.0E-9 | 2.9E4 | 2.4E4 | 341 | 15 | 341 | 15 | 0.64 |
| Iv | ng/ml | 1.3E1 | 6.1E1 | 9.4E1 | 6.3E2 | 8.9E2 | 1.3E3 | 1.0E-9 | 1.0E0 | 1.6E4 | 3.8E3 | 340 | 15 | 340 | 15 | 0.71 |
| Pz | ng/ml | 3.5E3 | 1.0E4 | 5.6E3 | 6.7E3 | 6.0E3 | 4.3E3 | 1.6E1 | 4.0E1 | 7.0E4 | 1.3E4 | 337 | 15 | 337 | 15 | 0.60 |
| Qa | ng/ml | 3.6E3 | 1.9E4 | 6.8E3 | 3.0E4 | 7.9E3 | 5.4E4 | 1.5E2 | 9.4E2 | 4.2E4 | 2.2E5 | 337 | 15 | 337 | 15 | 0.79 |
| Qb | ng/ml | 1.1E2 | 2.8E2 | 2.3E2 | 4.0E2 | 4.4E2 | 3.8E2 | 7.9E-1 | 3.2E1 | 5.3E3 | 1.6E3 | 337 | 15 | 337 | 15 | 0.72 |
| Qc | ng/ml | 2.1E2 | 5.0E2 | 4.5E2 | 6.4E2 | 5.9E2 | 7.8E2 | 1.0E-9 | 1.3E1 | 4.3E3 | 2.8E3 | 337 | 15 | 337 | 15 | 0.56 |
| Qd | ng/ml | 8.8E3 | 6.0E4 | 2.5E4 | 8.8E4 | 1.2E5 | 8.4E4 | 1.5E2 | 1.9E3 | 2.0E6 | 2.3E5 | 337 | 15 | 337 | 15 | 0.75 |
| Qe | ng/ml | 8.6E2 | 4.1E3 | 2.0E3 | 5.0E3 | 5.6E3 | 4.6E3 | 1.0E-9 | 1.2E2 | 9.7E4 | 1.8E4 | 337 | 15 | 337 | 15 | 0.78 |
| Jg | ng/ml | 4.6E2 | 2.0E3 | 7.7E2 | 2.1E3 | 9.4E2 | 1.7E3 | 5.8E0 | 8.4E1 | 1.0E4 | 7.1E3 | 340 | 15 | 340 | 15 | 0.78 |
| Jh | ng/ml | 2.9E0 | 6.0E1 | 2.0E1 | 1.0E2 | 8.2E1 | 1.4E2 | 1.0E-9 | 1.0E-9 | 1.2E3 | 4.9E2 | 340 | 15 | 340 | 15 | 0.80 |
| Ji | ng/ml | 5.6E1 | 3.2E2 | 8.6E1 | 3.6E2 | 9.6E1 | 3.2E2 | 1.1E0 | 2.3E1 | 6.9E2 | 1.3E3 | 340 | 15 | 340 | 15 | 0.84 |
| Jj | ng/ml | 5.3E2 | 1.1E2 | 1.9E3 | 4.7E2 | 1.8E4 | 5.2E2 | 2.3E0 | 8.7E0 | 3.4E5 | 1.4E3 | 340 | 15 | 340 | 15 | 0.34 |
| Jk | ng/ml | 2.6E0 | 6.5E1 | 1.9E1 | 8.1E1 | 4.6E1 | 7.8E1 | 1.0E-9 | 1.2E-1 | 3.9E2 | 2.4E2 | 340 | 15 | 340 | 15 | 0.77 |
| Jl | ng/ml | 4.8E-1 | 2.0E1 | 1.8E0 | 6.7E2 | 4.4E0 | 2.6E3 | 1.2E-3 | 1.1E-1 | 4.0E1 | 9.9E3 | 340 | 15 | 340 | 15 | 0.84 |
| Jm | ng/ml | 2.0E1 | 4.9E1 | 6.7E1 | 7.3E1 | 1.7E2 | 9.7E1 | 1.0E-9 | 4.0E-1 | 2.1E3 | 3.6E2 | 340 | 15 | 340 | 15 | 0.59 |
| Jn | pg/ml | 3.3E-1 | 1.5E0 | 4.0E0 | 9.2E1 | 3.6E1 | 2.4E2 | 1.0E-9 | 2.4E-1 | 6.2E2 | 7.3E2 | 340 | 15 | 340 | 15 | 0.80 |
| Jo | pg/ml | 3.8E3 | 5.5E3 | 4.7E3 | 1.6E4 | 3.9E3 | 2.6E4 | 2.0E1 | 2.4E1 | 2.4E4 | 1.0E5 | 340 | 15 | 340 | 15 | 0.64 |
| Jp | pg/ml | 7.0E4 | 1.0E5 | 7.4E4 | 1.2E5 | 3.9E4 | 4.4E4 | 5.8E2 | 6.5E4 | 3.8E5 | 2.1E5 | 340 | 15 | 340 | 15 | 0.79 |
| Jq | pg/ml | 9.5E1 | 3.3E2 | 1.6E2 | 1.2E3 | 2.0E2 | 2.3E3 | 1.0E0 | 1.3E1 | 2.0E3 | 8.7E3 | 340 | 15 | 340 | 15 | 0.76 |
| Jr | pg/ml | 4.0E0 | 4.0E1 | 5.9E1 | 8.8E2 | 6.0E2 | 2.2E3 | 1.0E-9 | 6.7E0 | 1.1E4 | 7.4E3 | 340 | 15 | 340 | 15 | 0.87 |
| Js | pg/ml | 1.5E1 | 2.7E1 | 7.4E1 | 4.6E2 | 5.7E2 | 1.0E3 | 1.0E-9 | 2.7E0 | 1.0E4 | 3.0E3 | 340 | 15 | 340 | 15 | 0.69 |
| Jt | pg/ml | 2.4E3 | 4.3E3 | 3.0E3 | 1.1E4 | 2.4E3 | 1.6E4 | 2.2E1 | 1.5E2 | 2.2E4 | 5.2E4 | 340 | 15 | 340 | 15 | 0.66 |
| Lh | pg/ml | 1.3E4 | 3.8E4 | 2.1E4 | 1.3E5 | 2.7E4 | 1.6E5 | 1.0E-9 | 1.8E3 | 2.6E5 | 4.8E5 | 341 | 15 | 341 | 15 | 0.80 |
| Li | pg/ml | 3.5E3 | 1.5E4 | 1.9E4 | 1.0E5 | 9.0E4 | 1.3E5 | 1.2E1 | 3.7E1 | 1.3E6 | 4.1E5 | 341 | 15 | 341 | 15 | 0.67 |
| Lj | pg/ml | 2.9E3 | 3.8E3 | 2.0E4 | 3.9E4 | 5.6E4 | 1.0E5 | 1.0E-9 | 8.9E1 | 4.3E5 | 3.9E5 | 341 | 15 | 341 | 15 | 0.58 |
| Nv | pg/ml | 3.9E3 | 2.5E4 | 8.9E3 | 4.3E4 | 1.8E4 | 4.2E4 | 1.0E-9 | 1.6E2 | 1.6E5 | 1.2E5 | 342 | 15 | 342 | 15 | 0.80 |
| Nw | pg/ml | 9.5E3 | 2.4E4 | 1.3E4 | 5.1E4 | 1.6E4 | 6.9E4 | 1.9E2 | 4.5E3 | 2.1E5 | 2.2E5 | 342 | 15 | 342 | 15 | 0.79 |
| Nx | pg/ml | 2.2E2 | 1.1E3 | 4.2E2 | 1.2E3 | 6.0E2 | 1.1E3 | 1.0E-9 | 1.0E-9 | 3.5E3 | 4.1E3 | 342 | 15 | 342 | 15 | 0.74 |
| Ny | pg/ml | 6.4E0 | 4.6E1 | 9.7E1 | 3.3E2 | 1.3E3 | 7.0E2 | 1.0E-9 | 1.0E-9 | 2.5E4 | 2.8E3 | 342 | 15 | 342 | 15 | 0.81 |
| Oe | pg/ml | 2.9E1 | 1.0E-9 | 2.4E2 | 2.4E2 | 3.8E2 | 4.2E2 | 1.0E-9 | 1.0E-9 | 2.3E3 | 1.1E3 | 340 | 15 | 340 | 15 | 0.45 |
| Of | pg/ml | 1.3E2 | 1.4E2 | 4.8E3 | 1.1E4 | 2.0E4 | 1.9E4 | 1.0E-9 | 1.0E-9 | 1.9E5 | 6.6E4 | 342 | 15 | 342 | 15 | 0.60 |
| Og | pg/ml | 6.9E-2 | 1.7E-2 | 3.6E-1 | 7.4E-2 | 1.5E0 | 1.1E-1 | 1.0E-9 | 1.0E-9 | 1.9E1 | 3.2E-1 | 342 | 15 | 342 | 15 | 0.39 |
| Oh | pg/ml | 2.5E0 | 2.5E1 | 1.4E1 | 1.2E3 | 8.8E1 | 4.1E3 | 1.0E-9 | 1.0E-9 | 1.4E3 | 1.6E4 | 342 | 15 | 342 | 15 | 0.76 |
| Oi | pg/ml | 2.0E0 | 1.0E-9 | 4.9E0 | 6.1E0 | 7.8E0 | 8.8E0 | 1.0E-9 | 1.0E-9 | 6.7E1 | 3.1E1 | 342 | 15 | 342 | 15 | 0.49 |
| Ok | pg/ml | 3.9E2 | 1.5E3 | 5.3E2 | 2.0E3 | 6.2E2 | 2.1E3 | 1.5E1 | 5.3E1 | 7.0E3 | 7.8E3 | 342 | 15 | 342 | 15 | 0.78 |
| Om | pg/ml | 4.1E2 | 1.9E3 | 7.9E2 | 5.7E3 | 2.0E3 | 1.3E4 | 1.0E-9 | 7.0E1 | 3.0E4 | 5.1E4 | 342 | 15 | 342 | 15 | 0.80 |
| On | pg/ml | 1.8E2 | 1.2E3 | 2.8E2 | 1.8E3 | 4.0E2 | 2.1E3 | 1.0E-9 | 1.6E1 | 4.5E3 | 8.5E3 | 342 | 15 | 342 | 15 | 0.88 |
| Oy | pg/ml | 4.6E-1 | 3.3E0 | 6.0E0 | 7.2E0 | 3.1E1 | 9.3E0 | 1.0E-9 | 1.0E-9 | 4.0E2 | 3.3E1 | 341 | 15 | 341 | 15 | 0.69 |
| Oz | pg/ml | 6.3E-3 | 1.0E-9 | 3.3E-1 | 2.0E0 | 1.6E0 | 7.2E0 | 1.0E-9 | 1.0E-9 | 2.9E1 | 2.8E1 | 341 | 15 | 341 | 15 | 0.42 |
| Pa | pg/ml | 3.9E-1 | 9.2E-1 | 1.3E0 | 2.6E1 | 5.7E0 | 6.1E1 | 1.0E-9 | 1.0E-9 | 8.6E1 | 2.3E2 | 341 | 15 | 341 | 15 | 0.70 |
| Pb | pg/ml | 1.0E-9 | 1.0E-9 | 1.6E0 | 2.2E0 | 2.6E1 | 8.1E0 | 1.0E-9 | 1.0E-9 | 4.9E2 | 3.2E1 | 341 | 15 | 341 | 15 | 0.43 |

| Code | Units | Median | | Average | | Standard Deviation | | Minimum | | Maximum | | Number of Samples | | Number of Patients | | AUC |
|------|-------|--------|------|---------|------|------|------|---------|------|---------|------|--------|-----|---------|-----|-----|
| | | NonDis | Dis | NonDis | Dis | NonDis | Dis | NonDis | Dis | NonDis | Dis | NonDis | Dis | NonDis | Dis | |
| Pc | pg/ml | 4.8E-2 | 1.0E-9 | 3.8E-1 | 2.5E1 | 8.8E-1 | 8.5E1 | 1.0E-9 | 1.0E-9 | 1.4E1 | 3.3E2 | 341 | 15 | 341 | 15 | 0.50 |
| Pd | pg/ml | 1.6E0 | 5.4E0 | 6.2E0 | 2.2E1 | 4.6E1 | 3.3E1 | 1.0E-9 | 7.3E-2 | 8.4E2 | 1.2E2 | 341 | 15 | 341 | 15 | 0.67 |
| Pe | pg/ml | 2.1E1 | 2.2E2 | 1.1E2 | 2.0E3 | 4.4E2 | 4.1E3 | 1.0E-9 | 3.3E0 | 4.7E3 | 1.5E4 | 341 | 15 | 341 | 15 | 0.77 |
| Pf | pg/ml | 1.6E0 | 1.8E1 | 1.5E1 | 6.2E1 | 9.0E1 | 1.1E2 | 1.0E-9 | 1.0E-9 | 1.5E3 | 4.3E2 | 341 | 15 | 341 | 15 | 0.80 |
| Pg | pg/ml | 3.8E0 | 7.8E1 | 6.7E1 | 4.4E2 | 5.1E2 | 6.5E2 | 1.0E-9 | 4.6E-1 | 7.7E3 | 2.2E3 | 341 | 15 | 341 | 15 | 0.83 |
| aA | mg/dL | 9.0E-1 | 1.5E0 | 1.0E0 | 2.1E0 | 5.0E-1 | 1.3E0 | 3.0E-1 | 5.5E-1 | 4.2E0 | 4.7E0 | 516 | 23 | 516 | 23 | 0.78 |
| aC | mg/mL | 2.2E0 | 2.4E0 | 2.6E0 | 2.9E0 | 1.3E0 | 1.7E0 | 7.5E-1 | 1.1E0 | 7.4E0 | 5.5E0 | 167 | 9 | 167 | 9 | 0.51 |
| aD | ug/mL | 2.9E0 | 3.7E0 | 4.6E0 | 5.9E0 | 4.7E0 | 5.3E0 | 7.5E-1 | 1.8E0 | 3.5E1 | 1.8E1 | 167 | 9 | 167 | 9 | 0.57 |
| aE | mg/mL | 5.7E-1 | 5.7E-1 | 5.9E-1 | 5.7E-1 | 1.7E-1 | 1.2E-1 | 1.8E-1 | 3.9E-1 | 1.2E0 | 7.2E-1 | 167 | 9 | 167 | 9 | 0.47 |
| aF | ng/mL | 2.2E0 | 9.1E0 | 4.9E0 | 8.0E0 | 7.7E0 | 5.4E0 | 4.3E-3 | 4.3E-3 | 5.0E1 | 1.5E1 | 167 | 9 | 167 | 9 | 0.68 |
| aG | mg/mL | 1.4E-1 | 9.5E-2 | 1.6E-1 | 1.1E-1 | 8.7E-2 | 3.8E-2 | 3.2E-2 | 6.9E-2 | 4.8E-1 | 1.7E-1 | 167 | 9 | 167 | 9 | 0.37 |
| aH | ug/mL | 7.1E1 | 5.6E1 | 7.8E1 | 5.9E1 | 4.1E1 | 2.7E1 | 8.9E0 | 1.1E1 | 2.0E2 | 1.0E2 | 167 | 9 | 167 | 9 | 0.39 |
| aI | ug/mL | 1.7E2 | 1.4E2 | 1.8E2 | 1.3E2 | 6.1E1 | 5.7E1 | 3.2E1 | 7.5E1 | 3.4E2 | 2.6E2 | 167 | 9 | 167 | 9 | 0.28 |
| aJ | ug/mL | 2.4E0 | 6.8E0 | 3.1E0 | 7.6E0 | 2.2E0 | 6.5E0 | 8.2E-1 | 2.2E0 | 1.4E1 | 2.3E1 | 167 | 9 | 167 | 9 | 0.79 |
| aK | ng/mL | 1.3E0 | 1.6E0 | 1.9E0 | 2.3E0 | 1.9E0 | 2.3E0 | 2.9E-4 | 1.3E-1 | 1.0E1 | 6.5E0 | 167 | 9 | 167 | 9 | 0.52 |
| aL | mg/mL | 7.4E-1 | 7.3E-1 | 7.7E-1 | 7.2E-1 | 2.5E-1 | 3.5E-1 | 2.2E-1 | 2.7E-1 | 1.7E0 | 1.4E0 | 167 | 9 | 167 | 9 | 0.44 |
| aM | U/mL | 1.9E1 | 4.7E1 | 3.9E1 | 1.6E2 | 7.0E1 | 2.7E2 | 4.2E-2 | 4.2E-2 | 6.8E2 | 8.2E2 | 167 | 9 | 167 | 9 | 0.69 |
| aN | U/mL | 1.4E1 | 2.6E1 | 2.5E1 | 3.3E1 | 4.3E1 | 2.6E1 | 2.5E-3 | 7.4E0 | 3.8E2 | 8.8E1 | 167 | 9 | 167 | 9 | 0.68 |
| aO | pg/mL | 4.9E1 | 1.3E3 | 4.0E2 | 1.1E3 | 9.7E2 | 9.8E2 | 6.0E-2 | 1.3E1 | 6.6E3 | 2.4E3 | 167 | 9 | 167 | 9 | 0.77 |
| aP | ng/mL | 1.6E0 | 4.9E0 | 2.2E0 | 6.4E0 | 2.4E0 | 8.3E0 | 4.5E-1 | 1.6E0 | 2.8E1 | 2.8E1 | 167 | 9 | 167 | 9 | 0.80 |
| aQ | ng/mL | 2.4E-1 | 4.1E-1 | 3.5E-1 | 3.8E-1 | 3.2E-1 | 2.6E-1 | 2.0E-4 | 5.1E-2 | 2.0E0 | 9.0E-1 | 167 | 9 | 167 | 9 | 0.56 |
| aR | ng/mL | 1.7E0 | 3.8E0 | 3.0E0 | 3.3E0 | 4.1E0 | 1.9E0 | 2.6E-1 | 5.6E-1 | 3.4E1 | 5.5E0 | 167 | 9 | 167 | 9 | 0.63 |
| aS | ng/mL | 3.7E-1 | 5.4E-1 | 9.9E-1 | 1.0E0 | 2.7E0 | 1.1E0 | 4.2E-3 | 7.2E-2 | 3.3E1 | 2.8E0 | 167 | 9 | 167 | 9 | 0.54 |
| aU | pg/mL | 6.6E1 | 7.8E1 | 9.8E1 | 1.4E2 | 1.0E2 | 1.7E2 | 7.4E-2 | 7.4E-2 | 7.0E2 | 5.1E2 | 167 | 9 | 167 | 9 | 0.51 |
| aV | ng/mL | 5.8E-1 | 5.8E-1 | 1.0E0 | 1.3E0 | 2.6E0 | 1.9E0 | 7.6E-4 | 1.0E-1 | 3.3E1 | 6.0E0 | 167 | 9 | 167 | 9 | 0.50 |
| aW | pg/mL | 1.9E1 | 2.1E1 | 2.2E1 | 2.4E1 | 3.4E1 | 1.3E1 | 7.2E-2 | 7.2E-2 | 4.2E2 | 4.7E1 | 167 | 9 | 167 | 9 | 0.63 |
| aX | ng/mL | 8.0E0 | 1.8E1 | 1.5E1 | 3.7E1 | 3.1E1 | 4.9E1 | 3.0E-1 | 2.6E0 | 3.1E2 | 1.3E2 | 167 | 9 | 167 | 9 | 0.64 |
| aY | pg/mL | 5.3E1 | 6.0E1 | 7.5E1 | 8.4E1 | 1.1E2 | 6.8E1 | 4.1E-1 | 2.7E1 | 1.2E3 | 2.0E2 | 167 | 9 | 167 | 9 | 0.56 |
| aZ | pg/mL | 2.2E2 | 3.0E2 | 6.8E2 | 5.0E2 | 1.5E3 | 6.1E2 | 1.7E0 | 7.5E1 | 1.2E4 | 2.1E3 | 167 | 9 | 167 | 9 | 0.59 |
| bA | ng/mL | 1.3E1 | 1.2E2 | 6.0E1 | 3.3E2 | 1.3E2 | 5.2E2 | 3.0E-2 | 4.7E0 | 9.4E2 | 1.5E3 | 167 | 9 | 167 | 9 | 0.76 |
| bB | ng/mL | 2.8E2 | 1.9E2 | 3.1E2 | 1.9E2 | 1.8E2 | 1.1E2 | 2.1E0 | 3.3E1 | 9.5E2 | 3.8E2 | 167 | 9 | 167 | 9 | 0.31 |
| bC | ng/mL | 3.2E2 | 4.7E2 | 6.1E2 | 1.5E3 | 8.1E2 | 1.6E3 | 1.4E1 | 1.4E2 | 4.7E3 | 4.0E3 | 167 | 9 | 167 | 9 | 0.69 |
| bE | mg/mL | 5.1E0 | 5.5E0 | 5.4E0 | 6.8E0 | 2.1E0 | 3.8E0 | 1.8E0 | 1.3E0 | 1.2E1 | 1.1E1 | 167 | 9 | 167 | 9 | 0.57 |
| bF | pg/mL | 3.5E1 | 6.8E1 | 3.1E2 | 9.6E2 | 1.3E3 | 2.1E3 | 5.0E-2 | 2.0E1 | 1.1E4 | 6.3E3 | 167 | 9 | 167 | 9 | 0.74 |
| bG | ng/mL | 1.6E0 | 4.6E0 | 2.9E0 | 8.0E0 | 3.8E0 | 9.5E0 | 1.1E-1 | 2.4E-1 | 2.6E1 | 3.0E1 | 167 | 9 | 167 | 9 | 0.70 |
| bH | pg/mL | 5.7E-1 | 9.2E0 | 6.1E0 | 9.3E0 | 2.4E1 | 7.9E0 | 5.7E-1 | 5.7E-1 | 2.8E2 | 2.3E1 | 167 | 9 | 167 | 9 | 0.71 |
| bI | ng/mL | 4.0E-3 | 4.0E-3 | 9.1E-2 | 1.2E-1 | 2.0E-1 | 2.0E-1 | 4.0E-3 | 4.0E-3 | 9.8E-1 | 5.1E-1 | 167 | 9 | 167 | 9 | 0.56 |
| bJ | mg/mL | 1.9E0 | 2.3E0 | 2.3E0 | 3.3E0 | 1.9E0 | 3.3E0 | 2.5E-4 | 2.5E-4 | 1.1E1 | 9.0E0 | 167 | 9 | 167 | 9 | 0.57 |
| bL | pg/mL | 3.7E0 | 1.1E1 | 9.1E0 | 1.2E1 | 1.2E1 | 7.6E0 | 4.6E-2 | 3.0E0 | 6.0E1 | 2.4E1 | 167 | 9 | 167 | 9 | 0.69 |
| bM | mg/mL | 1.8E0 | 2.2E0 | 2.2E0 | 2.0E0 | 1.5E0 | 1.0E0 | 1.6E-2 | 5.5E-1 | 8.6E0 | 3.8E0 | 167 | 9 | 167 | 9 | 0.52 |
| bN | ng/mL | 3.3E1 | 5.9E1 | 1.2E2 | 7.8E1 | 2.9E2 | 8.5E1 | 1.4E-1 | 6.7E0 | 1.9E3 | 2.5E2 | 167 | 9 | 167 | 9 | 0.53 |
| bO | ng/mL | 4.0E-2 | 4.0E-2 | 9.3E0 | 4.2E0 | 2.0E1 | 1.2E1 | 4.0E-2 | 4.0E-2 | 1.3E2 | 3.8E1 | 167 | 9 | 167 | 9 | 0.39 |
| bP | mg/mL | 5.3E-1 | 4.9E-1 | 7.5E-1 | 7.1E-1 | 7.1E-1 | 4.9E-1 | 4.9E-2 | 9.2E-2 | 4.8E0 | 1.6E0 | 167 | 9 | 167 | 9 | 0.52 |
| bQ | pg/mL | 2.1E1 | 9.3E1 | 1.4E2 | 1.0E2 | 1.1E3 | 6.6E1 | 1.5E-1 | 1.3E1 | 1.3E4 | 2.2E2 | 167 | 9 | 167 | 9 | 0.80 |
| bR | ng/mL | 1.2E-2 | 1.2E-2 | 1.6E-1 | 8.3E-2 | 7.2E-1 | 1.4E-1 | 1.2E-2 | 1.2E-2 | 8.7E0 | 3.8E-1 | 167 | 9 | 167 | 9 | 0.46 |
| bS | pg/mL | 9.4E-1 | 9.4E-1 | 9.0E0 | 1.9E1 | 4.1E1 | 3.0E1 | 9.4E-1 | 9.4E-1 | 3.9E2 | 7.2E1 | 167 | 9 | 167 | 9 | 0.61 |
| bU | ng/mL | 8.7E-2 | 1.3E-2 | 1.9E-1 | 1.3E-1 | 5.4E-1 | 1.5E-1 | 1.3E-2 | 1.3E-2 | 6.6E0 | 4.1E-1 | 167 | 9 | 167 | 9 | 0.48 |
| bV | pg/mL | 4.9E2 | 6.8E2 | 6.2E2 | 1.0E3 | 8.9E2 | 6.8E2 | 1.6E2 | 3.4E2 | 1.2E4 | 2.2E3 | 167 | 9 | 167 | 9 | 0.74 |
| bW | pg/mL | 3.2E2 | 5.6E2 | 4.9E2 | 1.2E3 | 5.4E2 | 1.5E3 | 8.4E1 | 1.8E2 | 4.8E3 | 3.9E3 | 167 | 9 | 167 | 9 | 0.67 |
| bX | ng/mL | 2.5E-5 | 2.5E-5 | 2.6E-3 | 2.4E-3 | 3.2E-3 | 3.0E-3 | 2.5E-5 | 2.5E-5 | 1.3E-2 | 7.2E-3 | 167 | 9 | 167 | 9 | 0.49 |
| bZ | pg/mL | 2.7E2 | 1.8E3 | 1.8E3 | 6.8E3 | 6.5E3 | 1.4E4 | 1.5E-1 | 1.8E2 | 5.8E4 | 4.3E4 | 167 | 9 | 167 | 9 | 0.72 |
| cA | pg/mL | 6.0E-1 | 6.0E-1 | 4.1E0 | 2.9E0 | 2.9E1 | 6.8E0 | 6.0E-1 | 6.0E-1 | 3.7E2 | 2.1E1 | 167 | 9 | 167 | 9 | 0.50 |
| cB | ng/mL | 4.9E-2 | 4.6E-2 | 7.4E-2 | 8.3E-2 | 8.7E-2 | 9.8E-2 | 1.7E-3 | 1.7E-3 | 4.3E-1 | 2.6E-1 | 167 | 9 | 167 | 9 | 0.49 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|------|--------|------|------|
| | | NonDis | Dis | NonDis | Dis | NonDis | Dis | NonDis | Dis | NonDis | Dis | NonDis | Dis | NonDis | Dis | |
| cC | pg/mL | 4.1E1 | 3.7E1 | 4.6E1 | 3.4E1 | 5.1E1 | 2.7E1 | 1.0E0 | 1.0E0 | 4.5E2 | 6.7E1 | 167 | 9 | 167 | 9 | 0.43 |
| cD | pg/mL | 4.9E0 | 2.9E0 | 1.3E1 | 4.2E0 | 4.7E1 | 3.9E0 | 3.3E-1 | 3.3E-1 | 4.8E2 | 9.6E0 | 167 | 9 | 167 | 9 | 0.40 |
| cE | pg/mL | 5.9E1 | 2.6E2 | 2.6E2 | 3.2E2 | 6.0E2 | 3.7E2 | 1.2E-1 | 3.4E1 | 3.8E3 | 1.3E3 | 167 | 9 | 167 | 9 | 0.71 |
| cF | pg/mL | 4.5E0 | 5.3E-1 | 1.6E1 | 8.6E0 | 3.0E1 | 1.6E1 | 5.3E-1 | 5.3E-1 | 2.7E2 | 3.8E1 | 167 | 9 | 167 | 9 | 0.39 |
| cG | pg/mL | 5.4E1 | 2.3E2 | 1.7E2 | 2.4E2 | 8.2E2 | 1.7E2 | 7.8E0 | 3.6E1 | 1.0E4 | 4.9E2 | 167 | 9 | 167 | 9 | 0.75 |
| cH | uIU/mL | 3.2E0 | 3.1E0 | 7.3E0 | 1.2E1 | 1.7E1 | 1.9E1 | 8.6E-3 | 8.6E-3 | 1.6E2 | 5.3E1 | 167 | 9 | 167 | 9 | 0.51 |
| cI | ng/mL | 6.1E0 | 9.1E0 | 1.4E1 | 1.2E1 | 2.2E1 | 1.4E1 | 3.2E-2 | 1.1E0 | 1.2E2 | 4.1E1 | 167 | 9 | 167 | 9 | 0.50 |
| cJ | ug/mL | 6.7E1 | 3.6E1 | 1.0E2 | 4.7E1 | 1.0E2 | 5.0E1 | 6.9E0 | 5.6E0 | 6.4E2 | 1.7E2 | 167 | 9 | 167 | 9 | 0.31 |
| cK | ng/mL | 3.8E-3 | 3.8E-3 | 2.2E-2 | 4.7E-2 | 1.2E-1 | 6.8E-2 | 3.8E-3 | 3.8E-3 | 1.5E0 | 2.1E-1 | 167 | 9 | 167 | 9 | 0.67 |
| cL | pg/mL | 2.1E2 | 2.3E2 | 5.5E2 | 3.6E2 | 2.0E3 | 3.9E2 | 3.1E1 | 6.7E1 | 2.4E4 | 1.3E3 | 167 | 9 | 167 | 9 | 0.56 |
| cM | pg/mL | 2.7E2 | 2.2E2 | 2.9E2 | 2.2E2 | 1.7E2 | 7.6E1 | 2.5E1 | 5.7E1 | 1.1E3 | 3.1E2 | 167 | 9 | 167 | 9 | 0.37 |
| cN | pg/mL | 1.2E2 | 1.2E2 | 1.3E2 | 1.3E2 | 8.6E1 | 4.0E1 | 3.8E1 | 8.6E1 | 1.1E3 | 2.0E2 | 167 | 9 | 167 | 9 | 0.55 |
| cO | pg/mL | 2.1E2 | 3.2E2 | 4.0E2 | 4.9E2 | 1.5E3 | 4.4E2 | 5.4E1 | 9.6E1 | 1.9E4 | 1.5E3 | 167 | 9 | 167 | 9 | 0.68 |
| cP | ng/mL | 2.4E3 | 2.3E3 | 2.5E3 | 2.8E3 | 9.4E2 | 1.2E3 | 6.2E2 | 1.4E3 | 5.6E3 | 4.7E3 | 167 | 9 | 167 | 9 | 0.56 |
| cQ | ng/mL | 4.9E-2 | 9.1E-2 | 1.2E-1 | 2.2E-1 | 2.1E-1 | 2.9E-1 | 2.0E-3 | 2.0E-3 | 1.3E0 | 8.7E-1 | 167 | 9 | 167 | 9 | 0.59 |
| cR | ng/mL | 3.0E2 | 5.7E2 | 5.9E2 | 7.7E2 | 8.3E2 | 6.1E2 | 2.0E1 | 3.2E2 | 7.7E3 | 2.2E3 | 167 | 9 | 167 | 9 | 0.69 |
| cS | ng/mL | 2.8E2 | 6.6E2 | 4.2E2 | 1.6E3 | 4.7E2 | 2.2E3 | 4.1E1 | 1.4E2 | 3.3E3 | 7.1E3 | 167 | 9 | 167 | 9 | 0.76 |
| cT | ng/mL | 4.9E1 | 1.2E2 | 1.5E2 | 4.0E2 | 2.8E2 | 5.9E2 | 3.6E0 | 1.9E1 | 2.1E3 | 1.5E3 | 167 | 9 | 167 | 9 | 0.70 |
| cU | ng/mL | 5.8E1 | 1.3E2 | 9.6E1 | 1.6E2 | 1.5E2 | 8.6E1 | 6.2E0 | 7.8E1 | 1.6E3 | 3.3E2 | 167 | 9 | 167 | 9 | 0.80 |
| cV | ng/mL | 1.9E-1 | 3.5E-1 | 7.3E-1 | 5.7E-1 | 3.8E0 | 7.8E-1 | 2.5E-2 | 3.0E-2 | 4.7E1 | 2.5E0 | 167 | 9 | 167 | 9 | 0.56 |
| cW | mIU/mL | 4.8E-2 | 9.4E-2 | 8.7E-2 | 1.2E-1 | 3.5E-1 | 1.1E-1 | 4.8E-3 | 2.7E-2 | 4.5E0 | 3.9E-1 | 167 | 9 | 167 | 9 | 0.68 |
| cX | ng/mL | 1.2E-1 | 3.3E-2 | 1.9E0 | 3.6E-1 | 5.7E0 | 8.1E-1 | 2.3E-4 | 1.1E-2 | 2.8E1 | 2.5E0 | 167 | 9 | 167 | 9 | 0.42 |
| cY | ng/mL | 7.4E0 | 8.9E0 | 1.1E1 | 1.4E1 | 1.1E1 | 1.5E1 | 1.7E-1 | 6.0E-1 | 6.1E1 | 3.7E1 | 167 | 9 | 167 | 9 | 0.50 |
| cZ | ug/mL | 1.3E1 | 1.1E1 | 1.5E1 | 1.4E1 | 6.7E0 | 8.2E0 | 2.3E0 | 3.3E0 | 4.6E1 | 3.0E1 | 167 | 9 | 167 | 9 | 0.45 |
| dA | pg/mL | 3.1E2 | 6.3E2 | 3.8E2 | 5.6E2 | 4.6E2 | 3.0E2 | 1.0E2 | 1.7E2 | 5.8E3 | 9.3E2 | 167 | 9 | 167 | 9 | 0.68 |
| dB | ug/mL | 1.8E1 | 2.2E1 | 1.8E1 | 2.1E1 | 2.0E1 | 8.2E0 | 2.1E0 | 4.5E0 | 2.5E2 | 3.0E1 | 167 | 9 | 167 | 9 | 0.63 |
| dC | nmol/L | 3.4E1 | 3.0E1 | 3.7E1 | 3.2E1 | 1.7E1 | 6.5E0 | 7.8E0 | 2.5E1 | 1.4E2 | 4.1E1 | 167 | 9 | 167 | 9 | 0.42 |
| dD | ug/mL | 3.4E1 | 2.8E1 | 3.5E1 | 3.0E1 | 1.1E1 | 9.7E0 | 1.4E1 | 2.1E1 | 7.4E1 | 5.0E1 | 167 | 9 | 167 | 9 | 0.32 |
| dE | ng/mL | 4.1E-1 | 8.6E-1 | 5.6E-1 | 1.0E0 | 5.8E-1 | 8.8E-1 | 8.4E-3 | 8.4E-3 | 2.9E0 | 2.4E0 | 167 | 9 | 167 | 9 | 0.67 |
| dF | ng/mL | 2.5E2 | 7.7E2 | 3.3E2 | 6.3E2 | 2.4E2 | 3.5E2 | 7.5E1 | 2.3E2 | 1.3E3 | 1.2E3 | 167 | 9 | 167 | 9 | 0.78 |
| dG | ng/mL | 1.2E1 | 2.4E1 | 1.6E1 | 3.1E1 | 1.8E1 | 2.8E1 | 3.0E0 | 6.7E0 | 1.8E2 | 8.7E1 | 167 | 9 | 167 | 9 | 0.69 |
| dH | pg/mL | 8.0E0 | 1.4E1 | 2.1E1 | 1.8E1 | 6.3E1 | 2.3E1 | 4.0E-2 | 8.3E-1 | 6.7E2 | 7.6E1 | 167 | 9 | 167 | 9 | 0.60 |
| dI | pg/mL | 4.6E-1 | 2.9E0 | 3.7E0 | 5.0E0 | 2.6E1 | 6.1E0 | 4.6E-1 | 4.6E-1 | 3.3E2 | 1.9E1 | 167 | 9 | 167 | 9 | 0.69 |
| dJ | ng/mL | 2.0E0 | 2.5E0 | 2.1E0 | 2.4E0 | 1.1E0 | 1.3E0 | 3.2E-2 | 4.9E-1 | 5.6E0 | 4.4E0 | 167 | 9 | 167 | 9 | 0.57 |
| dK | uIU/mL | 1.4E0 | 6.4E-1 | 2.2E0 | 1.1E0 | 3.6E0 | 1.9E0 | 2.8E-4 | 1.4E-2 | 3.9E1 | 6.1E0 | 167 | 9 | 167 | 9 | 0.27 |
| dL | ng/mL | 8.7E2 | 1.2E3 | 1.0E3 | 1.5E3 | 5.6E2 | 1.3E3 | 2.8E2 | 5.8E2 | 3.8E3 | 4.8E3 | 167 | 9 | 167 | 9 | 0.58 |
| dM | pg/mL | 9.7E2 | 2.5E3 | 1.3E3 | 2.9E3 | 1.4E3 | 2.1E3 | 3.7E2 | 7.1E2 | 1.5E4 | 5.8E3 | 167 | 9 | 167 | 9 | 0.70 |
| dN | ug/mL | 9.8E1 | 1.4E2 | 1.0E2 | 1.6E2 | 4.0E1 | 6.9E1 | 2.4E1 | 9.4E1 | 2.8E2 | 3.3E2 | 167 | 9 | 167 | 9 | 0.81 |
| eD | pg/ml | 2.2E2 | 2.1E2 | 7.3E2 | 2.5E2 | 1.6E3 | 1.6E2 | 5.2E-1 | 5.9E1 | 8.3E3 | 4.9E2 | 90 | 7 | 90 | 7 | 0.50 |
| fR | ng/ml | 1.5E5 | 3.4E5 | 2.1E5 | 3.7E5 | 1.7E5 | 2.8E5 | 3.6E4 | 1.9E2 | 6.9E5 | 8.7E5 | 106 | 9 | 106 | 9 | 0.68 |
| hA | ng/ml | 2.5E0 | 5.4E0 | 1.4E1 | 2.3E1 | 5.0E1 | 4.1E1 | 1.7E-2 | 1.7E-2 | 3.5E2 | 1.1E2 | 91 | 7 | 91 | 7 | 0.65 |
| nM | pg/ml | 1.0E-9 | 1.0E-9 | 1.0E-9 | 2.2E2 | 0.0E0 | 5.8E2 | 1.0E-9 | 1.0E-9 | 1.0E-9 | 1.5E3 | 69 | 7 | 69 | 7 | 0.57 |
| nN | pg/ml | 1.4E3 | 2.4E3 | 4.1E3 | 2.9E4 | 1.3E4 | 5.7E4 | 8.1E1 | 4.1E2 | 1.0E5 | 1.5E5 | 69 | 7 | 69 | 7 | 0.61 |
| nO | pg/ml | 2.4E1 | 4.1E1 | 3.9E1 | 3.4E1 | 4.8E1 | 1.5E1 | 4.0E0 | 9.7E0 | 3.1E2 | 5.2E1 | 69 | 7 | 69 | 7 | 0.59 |
| nR | pg/ml | 1.6E1 | 7.1E1 | 6.8E1 | 4.3E2 | 1.7E2 | 7.0E2 | 1.0E0 | 1.1E1 | 1.1E3 | 1.9E3 | 69 | 7 | 69 | 7 | 0.77 |
| nT | pg/ml | 7.3E1 | 5.7E1 | 1.1E2 | 2.2E2 | 1.1E2 | 3.3E2 | 1.0E-9 | 1.0E-9 | 6.4E2 | 9.2E2 | 69 | 7 | 69 | 7 | 0.51 |
| nU | pg/ml | 4.4E1 | 1.2E2 | 8.7E1 | 2.9E2 | 2.0E2 | 3.4E2 | 1.0E-9 | 1.0E-9 | 1.5E3 | 9.2E2 | 69 | 7 | 69 | 7 | 0.69 |
| lW | pg/ml | 1.0E-9 | 1.0E-9 | 1.1E1 | 6.4E0 | 3.0E1 | 1.4E1 | 1.0E-9 | 1.0E-9 | 1.7E2 | 3.9E1 | 69 | 7 | 69 | 7 | 0.48 |
| lX | pg/ml | 9.0E2 | 9.2E2 | 9.6E2 | 1.3E3 | 5.5E2 | 7.4E2 | 1.9E2 | 4.8E2 | 2.6E3 | 2.5E3 | 69 | 7 | 69 | 7 | 0.64 |
| lY | pg/ml | 1.9E1 | 1.4E1 | 2.0E1 | 2.1E1 | 1.7E1 | 1.5E1 | 1.0E-9 | 3.1E0 | 1.2E2 | 4.5E1 | 69 | 7 | 69 | 7 | 0.52 |
| mE | pg/ml | 1.0E-9 | 1.0E-9 | 2.5E0 | 2.6E0 | 7.7E0 | 3.9E0 | 1.0E-9 | 1.0E-9 | 5.7E1 | 9.2E0 | 69 | 7 | 69 | 7 | 0.55 |
| mF | pg/ml | 2.7E-1 | 6.3E0 | 6.6E0 | 5.6E0 | 3.2E1 | 5.2E0 | 1.0E-9 | 1.0E-9 | 2.5E2 | 1.3E1 | 69 | 7 | 69 | 7 | 0.75 |
| mH | pg/ml | 3.0E0 | 5.3E0 | 5.4E0 | 4.7E0 | 8.1E0 | 3.3E0 | 4.0E-1 | 5.4E-1 | 5.3E1 | 1.0E1 | 69 | 7 | 69 | 7 | 0.57 |

| Code | Units | Median | | Average | | Standard Deviation | | Minimum | | Maximum | | Number of Samples | | Number of Patients | | AUC |
|------|-------|--------|-----|---------|-----|-------|-----|---------|-----|---------|-----|--------|-----|---------|-----|-----|
|  |  | NonDis | Dis | NonDis | Dis | NonDis | Dis | NonDis | Dis | NonDis | Dis | NonDis | Dis | NonDis | Dis |  |
| mI | pg/ml | 1.0E-9 | 3.4E0 | 1.2E1 | 8.4E1 | 2.6E1 | 1.7E2 | 1.0E-9 | 1.0E-9 | 1.8E2 | 4.6E2 | 69 | 7 | 69 | 7 | 0.65 |
| mM | pg/ml | 3.3E1 | 5.1E1 | 7.9E1 | 1.0E2 | 1.5E2 | 1.7E2 | 1.0E-9 | 1.0E-9 | 1.1E3 | 4.7E2 | 69 | 7 | 69 | 7 | 0.56 |
| mP | pg/ml | 1.5E1 | 1.6E1 | 1.7E1 | 1.5E2 | 1.6E1 | 2.9E2 | 1.0E-9 | 1.4E1 | 1.2E2 | 8.1E2 | 68 | 7 | 68 | 7 | 0.65 |
| mS | pg/ml | 1.6E3 | 2.0E3 | 1.7E3 | 2.1E3 | 1.0E3 | 1.0E3 | 1.0E-9 | 6.7E2 | 5.1E3 | 3.5E3 | 69 | 7 | 69 | 7 | 0.62 |
| mT | pg/ml | 5.3E1 | 1.4E2 | 1.2E2 | 4.6E2 | 2.2E2 | 6.4E2 | 1.0E1 | 1.6E1 | 1.4E3 | 1.7E3 | 68 | 7 | 68 | 7 | 0.67 |
| mU | pg/ml | 2.4E0 | 1.8E0 | 6.3E0 | 5.6E0 | 2.7E1 | 8.2E0 | 1.0E-9 | 6.1E-1 | 2.2E2 | 2.3E1 | 68 | 7 | 68 | 7 | 0.45 |
| mW | pg/ml | 2.1E3 | 1.9E3 | 2.4E3 | 4.8E3 | 1.2E3 | 4.8E3 | 1.0E-9 | 3.7E2 | 6.2E3 | 1.1E4 | 68 | 7 | 68 | 7 | 0.53 |
| mY | pg/ml | 6.5E2 | 6.5E2 | 8.8E2 | 1.7E3 | 9.6E2 | 2.8E3 | 1.0E-9 | 1.9E2 | 5.6E3 | 8.0E3 | 69 | 7 | 69 | 7 | 0.59 |
| mZ | pg/ml | 1.8E2 | 3.0E2 | 3.6E2 | 5.2E2 | 4.7E2 | 5.4E2 | 1.0E-9 | 6.6E1 | 3.1E3 | 1.4E3 | 68 | 7 | 68 | 7 | 0.59 |
| nA | pg/ml | 1.5E0 | 3.4E0 | 7.1E0 | 5.5E0 | 1.4E1 | 6.5E0 | 1.0E-9 | 1.0E-9 | 6.5E1 | 1.9E1 | 68 | 7 | 68 | 7 | 0.56 |
| nB | pg/ml | 3.0E2 | 2.8E2 | 3.1E2 | 3.5E2 | 1.6E2 | 3.0E2 | 3.0E1 | 1.3E2 | 8.2E2 | 9.6E2 | 69 | 7 | 69 | 7 | 0.44 |
| nC | pg/ml | 1.0E-9 | 7.0E2 | 9.6E3 | 2.2E3 | 5.3E4 | 3.1E3 | 1.0E-9 | 1.0E-9 | 3.8E5 | 7.2E3 | 69 | 7 | 69 | 7 | 0.71 |
| nD | pg/ml | 6.4E0 | 1.6E1 | 1.4E1 | 1.4E1 | 3.4E1 | 7.6E0 | 1.0E-9 | 1.0E-9 | 2.6E2 | 2.2E1 | 68 | 7 | 68 | 7 | 0.68 |
| nF | pg/ml | 1.0E-9 | 1.0E-9 | 3.2E0 | 1.9E0 | 1.3E1 | 5.1E0 | 1.0E-9 | 1.0E-9 | 9.3E1 | 1.3E1 | 69 | 7 | 69 | 7 | 0.52 |
| nH | pg/ml | 5.6E-1 | 1.1E1 | 2.0E2 | 3.2E1 | 1.2E3 | 4.4E1 | 1.0E-9 | 1.0E-9 | 1.0E4 | 1.2E2 | 68 | 7 | 68 | 7 | 0.70 |
| nI | pg/ml | 3.0E1 | 1.0E-9 | 7.2E1 | 2.3E1 | 1.6E2 | 3.9E1 | 1.0E-9 | 1.0E-9 | 1.2E3 | 8.0E1 | 69 | 7 | 69 | 7 | 0.40 |
| nJ | pg/ml | 1.7E-1 | 1.1E0 | 3.0E0 | 1.2E0 | 1.6E1 | 1.1E0 | 1.0E-9 | 1.0E-9 | 1.3E2 | 3.0E0 | 69 | 7 | 69 | 7 | 0.61 |
| nK | pg/ml | 1.0E-9 | 1.0E1 | 1.5E1 | 2.3E1 | 3.4E1 | 2.7E1 | 1.0E-9 | 1.0E-9 | 2.2E2 | 6.2E1 | 68 | 7 | 68 | 7 | 0.63 |
| nL | pg/ml | 1.0E-9 | 1.5E1 | 2.9E2 | 7.0E1 | 1.7E3 | 9.0E1 | 1.0E-9 | 1.0E-9 | 1.4E4 | 2.3E2 | 69 | 7 | 69 | 7 | 0.64 |
| hR | pg/ml | 2.8E4 | 1.8E4 | 3.0E4 | 2.0E4 | 1.2E4 | 6.0E3 | 1.0E-9 | 1.6E4 | 5.8E4 | 3.2E4 | 84 | 7 | 84 | 7 | 0.21 |
| hV | pg/ml | 4.4E2 | 4.3E2 | 4.5E2 | 4.0E2 | 2.3E2 | 2.3E2 | 1.0E-9 | 9.5E1 | 1.2E3 | 6.7E2 | 84 | 7 | 84 | 7 | 0.44 |
| hW | pg/ml | 1.7E3 | 3.7E3 | 2.0E3 | 8.4E3 | 1.1E3 | 1.4E4 | 1.0E-9 | 1.5E3 | 7.3E3 | 4.0E4 | 84 | 7 | 84 | 7 | 0.79 |
| hX | pg/ml | 1.1E3 | 1.3E3 | 1.1E3 | 1.4E3 | 9.2E2 | 7.8E2 | 2.5E0 | 7.0E2 | 8.6E3 | 2.9E3 | 84 | 7 | 84 | 7 | 0.61 |
| iB | ng/ml | 4.9E0 | 1.4E1 | 6.2E0 | 1.2E1 | 4.7E0 | 6.4E0 | 3.3E-2 | 4.2E0 | 2.4E1 | 2.2E1 | 91 | 7 | 91 | 7 | 0.78 |
| iC | U/ml | 3.1E-1 | 7.0E-1 | 1.2E0 | 9.3E-1 | 5.8E0 | 8.6E-1 | 1.0E-9 | 1.5E-1 | 5.5E1 | 2.8E0 | 91 | 7 | 91 | 7 | 0.73 |
| jD | ng/ml | 3.1E1 | 5.3E1 | 4.0E1 | 1.6E2 | 4.0E1 | 1.9E2 | 1.0E-9 | 8.8E-1 | 1.9E2 | 5.1E2 | 91 | 7 | 91 | 7 | 0.72 |
| jE | ng/ml | 1.0E-9 | 1.8E1 | 5.0E0 | 1.5E1 | 1.2E1 | 1.5E1 | 1.0E-9 | 1.0E-9 | 6.2E1 | 3.5E1 | 91 | 7 | 91 | 7 | 0.69 |
| jF | ng/ml | 3.5E1 | 1.2E1 | 4.9E1 | 2.9E1 | 5.3E1 | 4.4E1 | 1.0E-9 | 1.0E-9 | 2.1E2 | 1.2E2 | 91 | 7 | 91 | 7 | 0.40 |
| jG | ng/ml | 4.4E3 | 4.2E3 | 4.5E3 | 4.8E3 | 2.0E3 | 1.4E3 | 6.7E2 | 3.6E3 | 9.6E3 | 7.3E3 | 91 | 7 | 91 | 7 | 0.57 |
| jH | ng/ml | 7.6E1 | 1.3E2 | 8.0E1 | 1.7E2 | 4.2E1 | 1.3E2 | 1.3E1 | 1.5E1 | 2.4E2 | 4.3E2 | 91 | 7 | 91 | 7 | 0.75 |
| jI | ng/ml | 7.3E1 | 2.1E2 | 7.6E1 | 2.1E2 | 3.1E1 | 1.2E2 | 1.9E1 | 5.8E1 | 1.9E2 | 4.4E2 | 91 | 7 | 91 | 7 | 0.89 |
| jK | ng/ml | 1.5E3 | 1.3E3 | 1.6E3 | 1.6E3 | 6.2E2 | 7.7E2 | 2.8E2 | 7.5E2 | 4.1E3 | 2.9E3 | 91 | 7 | 91 | 7 | 0.46 |
| jL | ng/ml | 2.0E2 | 4.0E2 | 2.7E2 | 3.7E2 | 2.0E2 | 2.0E2 | 5.6E1 | 1.2E2 | 9.6E2 | 6.3E2 | 91 | 7 | 91 | 7 | 0.66 |
| jM | ng/ml | 6.8E4 | 6.5E4 | 7.5E4 | 7.3E4 | 3.9E4 | 5.2E4 | 4.6E3 | 1.3E4 | 1.8E5 | 1.4E5 | 91 | 7 | 91 | 7 | 0.49 |
| jO | pg/ml | 2.2E5 | 2.7E5 | 2.7E5 | 3.3E5 | 1.7E5 | 1.6E5 | 6.0E4 | 1.8E5 | 1.1E6 | 6.5E5 | 91 | 7 | 91 | 7 | 0.64 |
| jP | pg/ml | 2.5E5 | 5.0E5 | 2.8E5 | 4.4E5 | 1.4E5 | 1.7E5 | 3.6E4 | 1.6E5 | 7.1E5 | 5.8E5 | 91 | 7 | 91 | 7 | 0.77 |
| jQ | pg/ml | 2.2E3 | 2.9E3 | 3.1E3 | 3.6E3 | 2.9E3 | 2.9E3 | 5.0E0 | 9.2E2 | 1.3E4 | 9.2E3 | 91 | 7 | 91 | 7 | 0.58 |
| jR | pg/ml | 5.6E3 | 8.9E3 | 9.8E3 | 1.5E4 | 1.2E4 | 1.6E4 | 1.0E-9 | 1.0E3 | 6.8E4 | 4.6E4 | 91 | 7 | 91 | 7 | 0.60 |
| jT | pg/ml | 1.7E5 | 1.6E5 | 1.8E5 | 1.7E5 | 7.2E4 | 5.0E4 | 7.1E4 | 1.1E5 | 5.5E5 | 2.5E5 | 91 | 7 | 91 | 7 | 0.50 |
| jU | mIU/ml | 5.4E0 | 6.7E0 | 1.2E1 | 8.0E0 | 1.9E1 | 7.3E0 | 8.1E-2 | 1.2E0 | 1.1E2 | 1.8E1 | 91 | 7 | 91 | 7 | 0.49 |
| jV | mIU/ml | 1.9E0 | 1.1E0 | 4.1E0 | 2.0E0 | 5.7E0 | 2.3E0 | 2.7E-3 | 3.0E-1 | 3.2E1 | 6.6E0 | 91 | 7 | 91 | 7 | 0.40 |
| jY | ng/ml | 9.7E-4 | 3.1E-3 | 7.7E-3 | 6.2E-3 | 3.3E-2 | 9.4E-3 | 1.0E-9 | 1.0E-9 | 3.0E-1 | 2.6E-2 | 91 | 7 | 91 | 7 | 0.60 |
| kC | pg/ml | 9.6E1 | 1.1E2 | 1.9E2 | 1.1E2 | 3.6E2 | 3.8E1 | 2.1E1 | 5.9E1 | 2.7E3 | 1.6E2 | 69 | 7 | 69 | 7 | 0.50 |
| kE | pg/ml | 1.4E5 | 1.2E5 | 1.4E5 | 1.2E5 | 4.0E4 | 4.5E4 | 4.1E4 | 3.8E4 | 2.7E5 | 1.9E5 | 69 | 7 | 69 | 7 | 0.42 |
| kF | pg/mL | 6.4E1 | 7.6E1 | 6.7E1 | 8.5E1 | 2.3E1 | 3.9E1 | 2.7E1 | 5.1E1 | 1.5E2 | 1.4E2 | 69 | 7 | 69 | 7 | 0.61 |
| kG | pg/mL | 8.2E3 | 1.6E4 | 1.1E4 | 4.5E4 | 9.6E3 | 5.5E4 | 1.1E3 | 3.3E3 | 5.8E4 | 1.6E5 | 69 | 7 | 69 | 7 | 0.78 |
| kI | pg/ml | 2.0E2 | 1.5E2 | 2.1E2 | 2.2E2 | 1.1E2 | 1.6E2 | 1.0E-9 | 7.2E1 | 6.7E2 | 5.5E2 | 69 | 7 | 69 | 7 | 0.45 |
| kK | pg/ml | 1.2E2 | 1.1E2 | 1.9E2 | 1.2E2 | 2.6E2 | 5.6E1 | 6.4E0 | 3.4E1 | 1.9E3 | 2.1E2 | 69 | 7 | 69 | 7 | 0.46 |
| kN | pg/ml | 1.1E3 | 1.1E3 | 1.5E3 | 2.1E3 | 1.6E3 | 2.9E3 | 7.3E1 | 7.0E2 | 1.0E4 | 8.7E3 | 69 | 7 | 69 | 7 | 0.55 |
| kO | pg/ml | 7.2E3 | 6.2E3 | 9.7E3 | 7.0E3 | 1.7E4 | 2.3E3 | 3.7E3 | 5.0E3 | 1.5E5 | 1.2E4 | 69 | 7 | 69 | 7 | 0.44 |
| kP | pg/ml | 5.6E3 | 5.4E3 | 6.7E3 | 6.8E3 | 4.3E3 | 4.6E3 | 9.6E2 | 1.6E3 | 2.7E4 | 1.5E4 | 69 | 7 | 69 | 7 | 0.51 |
| lK | pg/ml | 6.6E1 | 3.2E1 | 1.4E2 | 9.5E1 | 1.7E2 | 1.7E2 | 1.0E-9 | 1.0E-9 | 7.4E2 | 4.8E2 | 90 | 7 | 90 | 7 | 0.32 |
| lL | pg/ml | 1.7E3 | 2.7E3 | 2.9E3 | 3.1E3 | 4.9E3 | 2.5E3 | 7.5E1 | 5.3E2 | 4.2E4 | 6.8E3 | 91 | 7 | 91 | 7 | 0.59 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|---------|-----|---------|-----|------|
| | | NonDis | Dis | NonDis | Dis | NonDis | Dis | NonDis | Dis | NonDis | Dis | NonDis | Dis | NonDis | Dis | |
| lM | pg/ml | 1.2E3 | 3.1E3 | 3.8E3 | 1.7E4 | 6.3E3 | 2.6E4 | 2.1E2 | 9.5E0 | 4.2E4 | 6.7E4 | 91 | 7 | 91 | 7 | 0.68 |
| lN | pg/ml | 1.0E-9 | 1.0E-9 | 3.3E0 | 3.6E0 | 6.8E0 | 7.1E0 | 1.0E-9 | 1.0E-9 | 4.3E1 | 1.9E1 | 91 | 7 | 91 | 7 | 0.52 |
| lO | pg/ml | 1.0E-9 | 1.0E-9 | 1.8E0 | 2.0E1 | 1.4E1 | 5.2E1 | 1.0E-9 | 1.0E-9 | 1.3E2 | 1.4E2 | 90 | 7 | 90 | 7 | 0.56 |
| oO | pg/ml | 9.0E4 | 5.4E4 | 1.0E5 | 1.5E5 | 6.2E4 | 1.5E5 | 3.3E3 | 3.8E4 | 3.0E5 | 4.0E5 | 62 | 7 | 62 | 7 | 0.44 |
| oP | pg/ml | 1.3E5 | 2.0E5 | 1.4E5 | 2.4E5 | 8.8E4 | 1.7E5 | 2.4E4 | 5.0E4 | 4.2E5 | 5.7E5 | 62 | 7 | 62 | 7 | 0.72 |
| oQ | pg/ml | 3.0E3 | 4.0E3 | 3.6E3 | 9.7E3 | 3.0E3 | 1.1E4 | 7.7E2 | 1.9E3 | 2.1E4 | 3.2E4 | 62 | 7 | 62 | 7 | 0.64 |

Figure 35.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Wm | % | 8.5E-2 | 6.7E0 | 2.5E1 | 4.4E1 | 1.4E2 | 7.4E1 | 5.4E-2 | 8.5E-2 | 1.0E3 | 1.9E2 | 124 | 7 | 124 | 7 | 0.71 |
| Po | pg/ml | 3.0E-1 | 2.8E1 | 8.7E0 | 7.6E1 | 2.6E1 | 8.3E1 | 8.0E-3 | 2.6E-2 | 2.7E2 | 2.2E2 | 258 | 10 | 258 | 10 | 0.80 |
| Et | ng/ml | 1.5E3 | 4.0E3 | 1.8E3 | 3.9E3 | 1.1E3 | 9.1E2 | 7.9E1 | 1.8E3 | 4.7E3 | 5.0E3 | 257 | 10 | 257 | 10 | 0.92 |
| Fp | ng/ml | 1.4E1 | 3.9E1 | 2.5E1 | 4.3E1 | 2.8E1 | 3.6E1 | 6.0E-3 | 3.4E0 | 1.3E2 | 1.3E2 | 261 | 10 | 261 | 10 | 0.69 |
| Fr | ng/ml | 3.8E4 | 6.0E5 | 1.2E5 | 5.4E5 | 1.8E5 | 2.9E5 | 1.9E2 | 2.2E4 | 8.4E5 | 8.4E5 | 264 | 11 | 264 | 11 | 0.86 |
| Nm | pg/ml | 1.4E4 | 4.9E4 | 3.7E4 | 1.3E5 | 8.8E4 | 2.4E5 | 1.0E-9 | 1.0E4 | 9.6E5 | 8.2E5 | 261 | 10 | 261 | 10 | 0.77 |
| Nn | pg/ml | 1.8E2 | 6.1E3 | 1.8E3 | 2.4E4 | 8.0E3 | 3.9E4 | 1.0E-9 | 2.3E1 | 9.5E4 | 1.1E5 | 261 | 10 | 261 | 10 | 0.83 |
| No | pg/ml | 1.7E1 | 2.3E2 | 3.3E1 | 3.4E2 | 5.6E1 | 3.6E2 | 1.0E-9 | 4.4E0 | 5.6E2 | 9.1E2 | 261 | 10 | 261 | 10 | 0.77 |
| Nq | pg/ml | 2.0E0 | 4.6E1 | 2.4E1 | 1.2E2 | 7.7E1 | 1.9E2 | 1.0E-9 | 1.0E-9 | 6.7E2 | 6.3E2 | 261 | 10 | 261 | 10 | 0.78 |
| Nr | pg/ml | 2.0E0 | 1.2E1 | 2.3E1 | 2.4E2 | 8.0E1 | 4.5E2 | 1.0E-9 | 1.0E-9 | 9.8E2 | 1.4E3 | 261 | 10 | 261 | 10 | 0.70 |
| Ns | pg/ml | 1.0E-9 | 1.0E-9 | 9.7E0 | 3.6E-1 | 7.4E1 | 1.1E0 | 1.0E-9 | 1.0E-9 | 1.1E3 | 3.6E0 | 261 | 10 | 261 | 10 | 0.52 |
| Nt | pg/ml | 1.2E2 | 1.9E2 | 1.4E2 | 4.8E2 | 1.0E2 | 5.7E2 | 1.5E1 | 7.5E1 | 8.8E2 | 1.7E3 | 261 | 10 | 261 | 10 | 0.70 |
| Nu | pg/ml | 2.4E1 | 9.1E1 | 5.9E1 | 1.2E2 | 8.8E1 | 1.2E2 | 1.0E-9 | 1.0E-9 | 6.3E2 | 3.7E2 | 261 | 10 | 261 | 10 | 0.68 |
| Lu | pg/ml | 1.0E4 | 7.8E3 | 1.7E4 | 7.4E3 | 4.5E4 | 5.1E3 | 9.4E2 | 5.2E2 | 5.6E5 | 1.7E4 | 261 | 10 | 261 | 10 | 0.39 |
| Lv | pg/ml | 1.0E-9 | 5.9E1 | 1.6E1 | 6.4E1 | 3.2E1 | 6.9E1 | 1.0E-9 | 1.0E-9 | 2.6E2 | 1.9E2 | 261 | 10 | 261 | 10 | 0.69 |
| Lw | pg/ml | 1.0E-9 | 1.0E-9 | 4.6E-1 | 8.2E0 | 5.3E0 | 1.4E1 | 1.0E-9 | 1.0E-9 | 8.0E1 | 4.0E1 | 261 | 10 | 261 | 10 | 0.64 |
| Lx | pg/ml | 1.0E-9 | 1.3E3 | 1.9E2 | 3.3E3 | 6.1E2 | 6.8E3 | 1.0E-9 | 1.0E-9 | 6.2E3 | 2.2E4 | 261 | 10 | 261 | 10 | 0.82 |
| Ly | pg/ml | 1.0E-9 | 1.0E-9 | 9.6E0 | 7.8E0 | 1.8E1 | 1.9E1 | 1.0E-9 | 1.0E-9 | 1.1E2 | 6.0E1 | 261 | 10 | 261 | 10 | 0.51 |
| Lz | pg/ml | 1.0E-9 | 1.0E-9 | 3.2E0 | 1.3E1 | 2.9E1 | 2.3E1 | 1.0E-9 | 1.0E-9 | 4.3E2 | 6.2E1 | 261 | 10 | 261 | 10 | 0.63 |
| Ma | pg/ml | 5.2E2 | 3.6E3 | 2.6E3 | 3.8E3 | 6.8E3 | 2.6E3 | 1.0E-9 | 3.3E2 | 6.5E4 | 7.5E3 | 261 | 10 | 261 | 10 | 0.78 |
| Mb | pg/ml | 2.6E1 | 1.8E1 | 3.3E1 | 2.2E1 | 1.9E1 | 1.2E1 | 9.2E0 | 4.1E0 | 2.1E2 | 4.7E1 | 261 | 10 | 261 | 10 | 0.25 |
| Mc | pg/ml | 1.0E-9 | 1.0E-9 | 7.3E-2 | 1.0E-9 | 8.5E-1 | 0.0E0 | 1.0E-9 | 1.0E-9 | 1.3E1 | 1.0E-9 | 261 | 10 | 261 | 10 | 0.49 |
| Md | pg/ml | 1.0E-9 | 1.0E-9 | 4.9E-1 | 3.7E0 | 4.3E0 | 9.2E0 | 1.0E-9 | 1.0E-9 | 6.5E1 | 2.9E1 | 261 | 10 | 261 | 10 | 0.66 |
| Me | pg/ml | 3.1E1 | 3.3E1 | 3.1E1 | 4.1E1 | 2.6E1 | 5.2E1 | 1.0E-9 | 1.0E-9 | 3.2E2 | 1.8E2 | 261 | 10 | 261 | 10 | 0.50 |
| Mf | pg/ml | 1.0E-9 | 1.0E-9 | 7.8E-1 | 4.8E-1 | 4.4E0 | 1.4E0 | 1.0E-9 | 1.0E-9 | 5.6E1 | 4.5E0 | 261 | 10 | 261 | 10 | 0.51 |
| Mg | pg/ml | 1.4E0 | 1.3E1 | 7.4E0 | 1.2E1 | 1.5E1 | 1.3E1 | 1.0E-9 | 1.0E-9 | 1.5E2 | 3.7E1 | 261 | 10 | 261 | 10 | 0.61 |
| Mh | pg/ml | 1.0E-9 | 5.2E-2 | 1.0E0 | 3.4E0 | 7.4E0 | 6.6E0 | 1.0E-9 | 1.0E-9 | 1.1E2 | 1.8E1 | 261 | 10 | 261 | 10 | 0.66 |
| Mi | pg/ml | 1.0E-9 | 1.0E-9 | 1.1E0 | 4.7E1 | 9.2E0 | 1.0E2 | 1.0E-9 | 1.0E-9 | 1.2E2 | 3.2E2 | 261 | 10 | 261 | 10 | 0.69 |
| Mj | pg/ml | 1.0E-9 | 1.0E-9 | 5.8E0 | 4.6E1 | 3.1E1 | 7.7E1 | 1.0E-9 | 1.0E-9 | 4.6E2 | 2.1E2 | 261 | 10 | 261 | 10 | 0.63 |
| Mk | pg/ml | 2.1E0 | 3.4E0 | 1.8E1 | 6.7E1 | 1.1E2 | 1.6E2 | 1.0E-9 | 1.0E-9 | 1.3E3 | 5.0E2 | 261 | 10 | 261 | 10 | 0.55 |
| Ml | pg/ml | 1.0E-9 | 1.0E-9 | 1.2E1 | 5.3E1 | 1.3E2 | 1.6E2 | 1.0E-9 | 1.0E-9 | 2.1E3 | 5.1E2 | 261 | 10 | 261 | 10 | 0.56 |
| Mm | pg/ml | 6.2E2 | 3.0E3 | 1.1E3 | 2.9E3 | 1.3E3 | 2.0E3 | 1.0E-9 | 3.6E2 | 6.4E3 | 6.5E3 | 261 | 10 | 261 | 10 | 0.81 |
| Mn | pg/ml | 6.4E0 | 1.8E1 | 1.2E1 | 1.9E1 | 2.8E1 | 1.3E1 | 1.0E-9 | 5.5E0 | 3.5E2 | 5.1E1 | 261 | 10 | 261 | 10 | 0.77 |
| Mp | pg/ml | 1.0E-9 | 3.6E1 | 1.4E1 | 9.4E1 | 5.1E1 | 1.5E2 | 1.0E-9 | 1.0E-9 | 6.3E2 | 5.0E2 | 261 | 10 | 261 | 10 | 0.84 |
| Mq | pg/ml | 1.0E-9 | 1.0E-9 | 2.4E0 | 1.6E1 | 1.4E1 | 3.5E1 | 1.0E-9 | 1.0E-9 | 1.8E2 | 1.0E2 | 261 | 10 | 261 | 10 | 0.57 |
| Mr | pg/ml | 1.0E-9 | 9.9E0 | 2.8E1 | 7.6E2 | 1.4E2 | 1.2E3 | 1.0E-9 | 1.0E-9 | 1.5E3 | 3.4E3 | 261 | 10 | 261 | 10 | 0.68 |
| Ms | pg/ml | 3.3E2 | 2.2E2 | 4.6E2 | 3.6E2 | 5.1E2 | 3.5E2 | 1.0E-9 | 1.0E-9 | 3.3E3 | 9.8E2 | 261 | 10 | 261 | 10 | 0.46 |
| Mt | pg/ml | 4.8E-1 | 7.6E1 | 9.7E0 | 3.8E2 | 4.9E1 | 1.0E3 | 1.0E-9 | 2.8E-1 | 7.1E2 | 3.2E3 | 261 | 10 | 261 | 10 | 0.86 |
| Mu | pg/ml | 1.0E-9 | 3.9E0 | 1.9E0 | 7.7E0 | 1.6E1 | 1.1E1 | 1.0E-9 | 1.0E-9 | 2.3E2 | 3.5E1 | 261 | 10 | 261 | 10 | 0.80 |
| Mv | pg/ml | 1.0E-9 | 1.1E2 | 7.8E1 | 2.9E2 | 3.8E2 | 3.7E2 | 1.0E-9 | 1.0E-9 | 4.5E3 | 9.2E2 | 261 | 10 | 261 | 10 | 0.72 |
| Mw | pg/ml | 3.7E1 | 1.2E3 | 3.0E2 | 1.9E3 | 1.5E3 | 2.1E3 | 1.0E-9 | 1.0E-9 | 1.8E4 | 5.3E3 | 261 | 10 | 261 | 10 | 0.84 |
| Mx | pg/ml | 1.0E-9 | 7.3E-1 | 4.1E-1 | 3.0E0 | 2.1E0 | 6.1E0 | 1.0E-9 | 1.0E-9 | 3.2E1 | 2.0E1 | 261 | 10 | 261 | 10 | 0.72 |
| My | pg/ml | 1.0E-9 | 2.0E2 | 4.0E2 | 4.1E2 | 2.8E3 | 6.1E2 | 1.0E-9 | 1.0E-9 | 3.9E4 | 1.9E3 | 261 | 10 | 261 | 10 | 0.73 |
| Mz | pg/ml | 1.2E1 | 8.3E1 | 2.6E1 | 3.8E2 | 5.1E1 | 6.7E2 | 1.0E-9 | 1.0E-9 | 5.5E2 | 1.9E3 | 261 | 10 | 261 | 10 | 0.73 |
| Na | pg/ml | 1.0E-9 | 1.0E-9 | 5.1E-1 | 5.5E0 | 1.6E0 | 1.3E1 | 1.0E-9 | 1.0E-9 | 9.6E0 | 4.2E1 | 261 | 10 | 261 | 10 | 0.61 |
| Nb | pg/ml | 2.2E0 | 1.2E1 | 3.7E0 | 3.9E1 | 1.1E1 | 6.7E1 | 1.0E-9 | 1.0E-9 | 1.5E2 | 2.1E2 | 261 | 10 | 261 | 10 | 0.70 |
| Nc | pg/ml | 3.4E2 | 1.2E1 | 5.1E2 | 2.3E2 | 7.7E2 | 4.6E2 | 1.0E-9 | 1.0E-9 | 9.2E3 | 1.3E3 | 261 | 10 | 261 | 10 | 0.31 |
| Nd | pg/ml | 2.5E1 | 3.9E1 | 2.8E1 | 2.4E2 | 7.8E1 | 6.6E2 | 1.0E-9 | 1.0E-9 | 1.2E3 | 2.1E3 | 261 | 10 | 261 | 10 | 0.66 |
| Ne | pg/ml | 4.2E2 | 2.0E2 | 5.3E2 | 2.6E2 | 6.0E2 | 2.5E2 | 1.0E-9 | 1.0E-9 | 7.0E3 | 7.3E2 | 261 | 10 | 261 | 10 | 0.33 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nf | pg/ml | 1.0E-9 | 1.0E-9 | 2.1E0 | 2.8E1 | 8.8E0 | 5.1E1 | 1.0E-9 | 1.0E-9 | 8.2E1 | 1.3E2 | 261 | 10 | 261 | 10 | 0.58 |
| Ng | pg/ml | 1.7E1 | 4.7E1 | 1.0E2 | 9.7E1 | 2.0E2 | 1.3E2 | 1.0E-9 | 1.0E-9 | 1.6E3 | 4.2E2 | 261 | 10 | 261 | 10 | 0.54 |
| Nh | pg/ml | 6.0E1 | 3.1E1 | 8.1E1 | 3.9E1 | 7.8E1 | 2.9E1 | 1.0E-9 | 4.1E0 | 5.6E2 | 8.9E1 | 261 | 10 | 261 | 10 | 0.31 |
| Ni | pg/ml | 1.0E-9 | 1.0E-9 | 8.6E1 | 1.8E2 | 1.3E2 | 3.4E2 | 1.0E-9 | 1.0E-9 | 7.1E2 | 1.1E3 | 261 | 10 | 261 | 10 | 0.51 |
| Nj | pg/ml | 6.2E0 | 3.4E0 | 9.9E0 | 7.9E0 | 1.1E1 | 8.8E0 | 1.0E-9 | 1.0E-9 | 6.8E1 | 2.2E1 | 261 | 10 | 261 | 10 | 0.42 |
| Nk | pg/ml | 1.7E1 | 8.2E0 | 3.2E1 | 2.0E1 | 4.0E1 | 2.7E1 | 1.0E-9 | 1.0E-9 | 2.0E2 | 6.9E1 | 261 | 10 | 261 | 10 | 0.43 |
| Nl | pg/ml | 4.2E1 | 1.0E1 | 5.6E1 | 2.9E1 | 7.9E1 | 4.5E1 | 1.0E-9 | 1.0E-9 | 1.1E3 | 1.4E2 | 261 | 10 | 261 | 10 | 0.31 |
| Hq | pg/ml | 1.2E0 | 1.2E1 | 2.0E2 | 3.1E2 | 2.1E3 | 8.7E2 | 1.0E-9 | 6.2E-2 | 2.8E4 | 2.8E3 | 259 | 10 | 259 | 10 | 0.72 |
| Hr | pg/ml | 8.4E1 | 9.5E2 | 5.1E2 | 2.0E3 | 1.0E3 | 2.8E3 | 1.0E-9 | 1.0E-9 | 8.4E3 | 8.9E3 | 259 | 10 | 259 | 10 | 0.67 |
| Hu | pg/ml | 1.4E1 | 4.5E2 | 5.9E3 | 1.1E3 | 4.5E4 | 2.0E3 | 1.0E-9 | 1.0E-9 | 6.3E5 | 6.5E3 | 259 | 10 | 259 | 10 | 0.66 |
| Hv | pg/ml | 1.3E0 | 3.0E0 | 3.1E0 | 9.8E1 | 1.1E1 | 2.8E2 | 1.0E-9 | 1.0E-9 | 1.6E2 | 8.9E2 | 259 | 10 | 259 | 10 | 0.69 |
| Hw | pg/ml | 5.5E0 | 1.2E1 | 1.6E1 | 1.0E3 | 5.0E1 | 2.9E3 | 1.0E-9 | 5.1E-1 | 6.4E2 | 9.4E3 | 259 | 10 | 259 | 10 | 0.62 |
| Hx | pg/ml | 8.8E0 | 5.1E1 | 6.1E1 | 2.6E2 | 5.8E2 | 4.4E2 | 1.0E-9 | 3.9E0 | 9.3E3 | 1.3E3 | 259 | 10 | 259 | 10 | 0.78 |
| Ih | ng/ml | 7.3E1 | 5.9E2 | 2.4E2 | 8.0E2 | 4.3E2 | 8.8E2 | 1.0E-9 | 5.7E0 | 3.6E3 | 2.8E3 | 260 | 10 | 260 | 10 | 0.72 |
| Ii | ng/ml | 9.0E1 | 3.5E2 | 2.0E2 | 1.2E3 | 4.4E2 | 1.6E3 | 7.3E-1 | 2.3E0 | 5.2E3 | 4.5E3 | 260 | 10 | 260 | 10 | 0.74 |
| Ij | ng/ml | 8.7E1 | 1.4E2 | 2.0E2 | 2.6E3 | 6.1E2 | 7.6E3 | 2.8E0 | 4.6E1 | 6.4E3 | 2.4E4 | 257 | 10 | 257 | 10 | 0.72 |
| Ik | ng/ml | 1.2E1 | 1.8E1 | 1.7E3 | 6.8E1 | 1.3E4 | 1.4E2 | 5.9E-1 | 5.5E0 | 1.2E5 | 4.6E2 | 257 | 10 | 257 | 10 | 0.55 |
| Il | ng/ml | 3.8E2 | 7.0E2 | 1.3E3 | 3.1E3 | 2.8E3 | 4.7E3 | 1.0E-9 | 7.1E1 | 1.2E4 | 1.2E4 | 255 | 10 | 255 | 10 | 0.67 |
| Im | ng/ml | 2.3E2 | 7.0E2 | 4.7E2 | 1.7E3 | 7.6E2 | 2.3E3 | 1.4E1 | 1.5E2 | 6.0E3 | 6.2E3 | 257 | 10 | 257 | 10 | 0.77 |
| In | ng/ml | 3.3E0 | 2.3E0 | 1.9E1 | 4.8E2 | 8.2E1 | 1.4E3 | 1.0E-9 | 1.0E-9 | 1.1E3 | 4.5E3 | 260 | 10 | 260 | 10 | 0.49 |
| Io | ng/ml | 1.1E4 | 1.4E4 | 2.2E4 | 1.4E4 | 5.1E4 | 1.0E4 | 1.0E-9 | 1.5E3 | 7.1E5 | 3.3E4 | 260 | 10 | 260 | 10 | 0.50 |
| Ip | ng/ml | 1.3E1 | 4.8E1 | 2.2E1 | 4.4E1 | 2.7E1 | 1.4E1 | 1.0E-9 | 2.1E1 | 2.3E2 | 5.8E1 | 260 | 10 | 260 | 10 | 0.80 |
| Iq | ug/ml | 1.2E-1 | 2.6E-1 | 7.7E-1 | 2.7E1 | 2.7E0 | 6.8E1 | 1.0E-9 | 1.0E-9 | 3.4E1 | 2.2E2 | 260 | 10 | 260 | 10 | 0.68 |
| Ir | ug/ml | 4.1E-1 | 4.0E0 | 2.5E0 | 6.5E1 | 1.2E1 | 1.2E2 | 1.0E-9 | 3.4E-1 | 1.6E2 | 3.7E2 | 259 | 10 | 259 | 10 | 0.79 |
| Is | ng/ml | 2.2E0 | 4.0E1 | 8.1E0 | 6.6E1 | 1.7E1 | 8.1E1 | 1.0E-9 | 9.9E-1 | 1.5E2 | 2.6E2 | 260 | 10 | 260 | 10 | 0.85 |
| It | ng/ml | 2.1E0 | 8.2E0 | 1.5E1 | 8.0E1 | 6.8E1 | 2.1E2 | 1.0E-9 | 1.0E-9 | 8.3E2 | 6.8E2 | 260 | 10 | 260 | 10 | 0.66 |
| Iu | ng/ml | 1.8E2 | 6.6E2 | 1.1E3 | 5.2E3 | 3.4E3 | 1.0E4 | 1.0E-9 | 8.5E0 | 2.9E4 | 2.4E4 | 260 | 10 | 260 | 10 | 0.64 |
| Iv | ng/ml | 1.4E1 | 7.6E1 | 5.2E1 | 9.1E2 | 2.5E2 | 1.5E3 | 1.0E-9 | 1.0E0 | 3.8E3 | 3.8E3 | 259 | 10 | 259 | 10 | 0.75 |
| Pz | ng/ml | 4.2E3 | 6.9E3 | 5.9E3 | 6.6E3 | 6.3E3 | 4.4E3 | 1.6E1 | 1.1E3 | 7.0E4 | 1.3E4 | 256 | 10 | 256 | 10 | 0.59 |
| Qa | ng/ml | 3.8E3 | 1.5E4 | 7.1E3 | 3.6E4 | 8.1E3 | 6.6E4 | 1.5E2 | 2.2E3 | 4.2E4 | 2.2E5 | 256 | 10 | 256 | 10 | 0.78 |
| Qb | ng/ml | 1.1E2 | 4.0E2 | 2.4E2 | 4.0E2 | 3.9E2 | 2.0E2 | 7.9E-1 | 1.3E2 | 4.1E3 | 6.0E2 | 256 | 10 | 256 | 10 | 0.78 |
| Qc | ng/ml | 2.5E2 | 3.1E2 | 4.6E2 | 6.4E2 | 5.8E2 | 8.9E2 | 1.0E-9 | 1.3E1 | 4.3E3 | 2.8E3 | 256 | 10 | 256 | 10 | 0.53 |
| Qd | ng/ml | 1.0E4 | 2.6E4 | 2.9E4 | 7.8E4 | 1.3E5 | 8.6E4 | 2.4E2 | 3.5E3 | 2.0E6 | 2.3E5 | 256 | 10 | 256 | 10 | 0.72 |
| Qe | ng/ml | 1.0E3 | 4.3E3 | 2.1E3 | 5.5E3 | 6.3E3 | 5.4E3 | 7.6E0 | 5.7E2 | 9.7E4 | 1.8E4 | 256 | 10 | 256 | 10 | 0.78 |
| Jg | ng/ml | 5.5E2 | 2.2E3 | 8.4E2 | 2.4E3 | 9.9E2 | 1.9E3 | 5.8E0 | 2.8E2 | 1.0E4 | 7.1E3 | 259 | 10 | 259 | 10 | 0.83 |
| Jh | ng/ml | 3.0E0 | 7.8E1 | 2.4E1 | 1.2E2 | 9.4E1 | 1.5E2 | 1.0E-9 | 1.7E0 | 1.2E3 | 4.9E2 | 259 | 10 | 259 | 10 | 0.83 |
| Ji | ng/ml | 6.3E1 | 3.3E2 | 8.9E1 | 4.2E2 | 9.4E1 | 3.4E2 | 1.1E0 | 1.0E2 | 5.4E2 | 1.3E3 | 259 | 10 | 259 | 10 | 0.92 |
| Jj | ng/ml | 5.5E2 | 1.1E2 | 2.3E3 | 4.5E2 | 2.1E4 | 5.7E2 | 2.0E1 | 1.2E1 | 3.4E5 | 1.4E3 | 259 | 10 | 259 | 10 | 0.31 |
| Jk | ng/ml | 3.0E0 | 6.3E1 | 2.2E1 | 8.5E1 | 5.1E1 | 8.5E1 | 1.0E-9 | 1.3E0 | 3.9E2 | 2.4E2 | 259 | 10 | 259 | 10 | 0.77 |
| Jl | ng/ml | 5.1E-1 | 2.0E1 | 2.1E0 | 1.0E3 | 4.8E0 | 3.1E3 | 1.2E-3 | 1.1E-1 | 4.0E1 | 9.9E3 | 259 | 10 | 259 | 10 | 0.85 |
| Jm | ng/ml | 2.2E1 | 3.2E1 | 6.8E1 | 6.6E1 | 1.7E2 | 1.1E2 | 1.0E-9 | 2.1E0 | 2.1E3 | 3.6E2 | 259 | 10 | 259 | 10 | 0.57 |
| Jn | pg/ml | 3.9E-1 | 1.9E0 | 2.6E0 | 1.4E2 | 1.6E1 | 2.9E2 | 1.0E-9 | 4.2E-1 | 2.4E2 | 7.3E2 | 259 | 10 | 259 | 10 | 0.82 |
| Jo | pg/ml | 4.3E3 | 5.0E3 | 5.1E3 | 1.9E4 | 4.0E3 | 3.1E4 | 2.6E2 | 2.3E2 | 2.4E4 | 1.0E5 | 259 | 10 | 259 | 10 | 0.60 |
| Jp | pg/ml | 7.4E4 | 1.1E5 | 7.7E4 | 1.2E5 | 3.9E4 | 4.4E4 | 2.1E3 | 6.5E4 | 3.8E5 | 2.1E5 | 259 | 10 | 259 | 10 | 0.78 |
| Jq | pg/ml | 9.8E1 | 4.0E2 | 1.6E2 | 1.6E3 | 1.8E2 | 2.7E3 | 5.6E0 | 7.1E1 | 1.1E3 | 8.7E3 | 259 | 10 | 259 | 10 | 0.82 |
| Jr | pg/ml | 4.1E0 | 4.2E1 | 3.4E1 | 1.3E3 | 2.0E2 | 2.7E3 | 1.0E-9 | 6.7E0 | 2.4E3 | 7.4E3 | 259 | 10 | 259 | 10 | 0.87 |
| Js | pg/ml | 1.6E1 | 3.4E1 | 5.4E1 | 6.4E2 | 2.3E2 | 1.2E3 | 1.0E-9 | 6.1E0 | 3.0E3 | 3.0E3 | 259 | 10 | 259 | 10 | 0.71 |
| Jt | pg/ml | 2.6E3 | 4.4E3 | 3.1E3 | 1.1E4 | 2.2E3 | 1.6E4 | 1.5E2 | 1.0E3 | 1.8E4 | 5.2E4 | 259 | 10 | 259 | 10 | 0.67 |
| Lh | pg/ml | 1.5E4 | 4.0E4 | 2.3E4 | 1.6E5 | 2.9E4 | 1.9E5 | 1.0E-9 | 1.8E3 | 2.6E5 | 4.8E5 | 260 | 10 | 260 | 10 | 0.81 |
| Li | pg/ml | 3.9E3 | 4.1E4 | 1.7E4 | 8.4E4 | 6.8E4 | 1.0E5 | 1.3E1 | 5.0E2 | 9.2E5 | 3.1E5 | 260 | 10 | 260 | 10 | 0.73 |
| Lj | pg/ml | 3.1E3 | 4.5E3 | 1.9E4 | 5.5E4 | 5.1E4 | 1.2E5 | 1.0E-9 | 2.3E2 | 4.3E5 | 3.9E5 | 260 | 10 | 260 | 10 | 0.65 |
| Nv | pg/ml | 4.0E3 | 4.5E4 | 1.0E4 | 4.7E4 | 2.0E4 | 3.9E4 | 1.0E-9 | 2.6E3 | 1.6E5 | 1.1E5 | 261 | 10 | 261 | 10 | 0.82 |
| Nw | pg/ml | 1.0E4 | 2.7E4 | 1.4E4 | 6.6E4 | 1.7E4 | 8.0E4 | 1.9E2 | 1.1E4 | 2.1E5 | 2.2E5 | 261 | 10 | 261 | 10 | 0.86 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|------|--------|------|------|
| | | NonDis | Dis | NonDis | Dis | NonDis | Dis | NonDis | Dis | NonDis | Dis | NonDis | Dis | NonDis | Dis | |
| Nx | pg/ml | 2.2E2 | 1.2E3 | 4.4E2 | 1.4E3 | 6.3E2 | 1.1E3 | 1.0E-9 | 1.0E-9 | 3.5E3 | 4.1E3 | 261 | 10 | 261 | 10 | 0.81 |
| Ny | pg/ml | 7.7E0 | 1.7E2 | 1.2E2 | 4.6E2 | 1.5E3 | 8.4E2 | 1.0E-9 | 9.8E0 | 2.5E4 | 2.8E3 | 261 | 10 | 261 | 10 | 0.84 |
| Oc | pg/ml | 3.1E1 | 2.0E1 | 2.6E2 | 3.6E2 | 4.0E2 | 4.8E2 | 1.0E-9 | 1.0E-9 | 2.3E3 | 1.1E3 | 259 | 10 | 259 | 10 | 0.55 |
| Of | pg/ml | 1.9E2 | 3.4E2 | 6.0E3 | 1.5E4 | 2.3E4 | 2.3E4 | 1.0E-9 | 1.1E1 | 1.9E5 | 6.6E4 | 261 | 10 | 261 | 10 | 0.61 |
| Og | pg/ml | 7.6E-2 | 5.5E-2 | 3.9E-1 | 9.7E-2 | 1.6E0 | 1.3E-1 | 1.0E-9 | 1.0E-9 | 1.9E1 | 3.2E-1 | 261 | 10 | 261 | 10 | 0.44 |
| Oh | pg/ml | 2.8E0 | 3.8E1 | 1.7E1 | 1.5E2 | 1.0E2 | 3.5E2 | 1.0E-9 | 4.2E0 | 1.4E3 | 1.1E3 | 261 | 10 | 261 | 10 | 0.87 |
| Oi | pg/ml | 2.5E0 | 3.9E0 | 5.2E0 | 7.0E0 | 7.2E0 | 9.8E0 | 1.0E-9 | 1.0E-9 | 4.2E1 | 3.1E1 | 261 | 10 | 261 | 10 | 0.51 |
| Ok | pg/ml | 4.2E2 | 1.5E3 | 5.3E2 | 2.4E3 | 4.4E2 | 2.3E3 | 1.5E1 | 3.2E2 | 3.2E3 | 7.8E3 | 261 | 10 | 261 | 10 | 0.87 |
| Om | pg/ml | 4.3E2 | 2.9E3 | 8.7E2 | 7.7E3 | 2.3E3 | 1.5E4 | 1.0E-9 | 5.4E2 | 3.0E4 | 5.1E4 | 261 | 10 | 261 | 10 | 0.86 |
| On | pg/ml | 2.0E2 | 1.4E3 | 3.1E2 | 2.1E3 | 4.3E2 | 2.4E3 | 8.4E-1 | 3.1E2 | 4.5E3 | 8.5E3 | 261 | 10 | 261 | 10 | 0.92 |
| Oy | pg/ml | 4.8E-1 | 2.1E0 | 7.2E0 | 7.0E0 | 3.5E1 | 1.0E1 | 1.0E-9 | 1.0E-9 | 4.0E2 | 3.3E1 | 260 | 10 | 260 | 10 | 0.65 |
| Oz | pg/ml | 1.1E-2 | 1.0E-9 | 3.6E-1 | 2.9E0 | 1.8E0 | 8.8E0 | 1.0E-9 | 1.0E-9 | 2.9E1 | 2.8E1 | 260 | 10 | 260 | 10 | 0.43 |
| Pa | pg/ml | 4.2E-1 | 2.7E0 | 1.5E0 | 3.6E1 | 6.4E0 | 7.4E1 | 1.0E-9 | 1.7E-1 | 8.6E1 | 2.3E2 | 260 | 10 | 260 | 10 | 0.76 |
| Pb | pg/ml | 1.0E-9 | 1.0E-9 | 2.1E0 | 3.4E0 | 3.0E1 | 9.9E0 | 1.0E-9 | 1.0E-9 | 4.9E2 | 3.2E1 | 260 | 10 | 260 | 10 | 0.41 |
| Pc | pg/ml | 9.9E-2 | 1.0E-9 | 4.1E-1 | 4.5E0 | 9.8E-1 | 1.2E1 | 1.0E-9 | 1.0E-9 | 1.4E1 | 3.8E1 | 260 | 10 | 260 | 10 | 0.50 |
| Pd | pg/ml | 1.7E0 | 1.3E1 | 7.3E0 | 2.6E1 | 5.3E1 | 3.7E1 | 1.0E-9 | 7.3E-2 | 8.4E2 | 1.2E2 | 260 | 10 | 260 | 10 | 0.75 |
| Pe | pg/ml | 2.3E1 | 2.5E2 | 1.3E2 | 2.9E3 | 5.0E2 | 4.9E3 | 1.0E-9 | 1.4E1 | 4.7E3 | 1.5E4 | 260 | 10 | 260 | 10 | 0.78 |
| Pf | pg/ml | 1.8E0 | 2.6E1 | 1.5E1 | 7.6E1 | 9.7E1 | 1.3E2 | 1.0E-9 | 6.5E-1 | 1.5E3 | 4.3E2 | 260 | 10 | 260 | 10 | 0.81 |
| Pg | pg/ml | 4.6E0 | 7.7E1 | 8.7E1 | 4.8E2 | 5.8E2 | 7.3E2 | 1.0E-9 | 4.6E-1 | 7.7E3 | 2.2E3 | 260 | 10 | 260 | 10 | 0.82 |
| aA | mg/dL | 9.0E-1 | 2.1E0 | 9.9E-1 | 2.3E0 | 4.8E-1 | 1.4E0 | 3.0E-1 | 5.5E-1 | 4.2E0 | 4.7E0 | 392 | 14 | 392 | 14 | 0.79 |

Figure 36.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.6E1 | 1.3E2 | 8.5E1 | 1.5E2 | 5.6E1 | 1.0E2 | 7.0E0 | 1.6E1 | 4.0E2 | 4.8E2 | 260 | 38 | 260 | 38 | 0.72 |
| Ad | ug/mL | 4.6E-2 | 8.3E-2 | 7.2E-2 | 4.5E-1 | 8.7E-2 | 1.7E0 | 6.8E-4 | 7.8E-4 | 5.4E-1 | 8.5E0 | 150 | 24 | 150 | 24 | 0.63 |
| Af | ng/mL | 1.3E0 | 7.7E-1 | 1.1E1 | 4.1E0 | 4.7E1 | 6.5E0 | 1.7E-3 | 1.7E-3 | 5.3E2 | 2.3E1 | 150 | 24 | 150 | 24 | 0.44 |
| Aj | ug/mL | 1.4E0 | 2.9E-1 | 2.4E0 | 1.6E0 | 2.5E0 | 2.3E0 | 1.5E-3 | 1.3E-3 | 6.1E0 | 6.1E0 | 150 | 24 | 150 | 24 | 0.39 |
| Al | mg/mL | 8.3E-5 | 1.2E-4 | 2.6E-4 | 4.0E-4 | 4.3E-4 | 5.1E-4 | 4.3E-6 | 7.6E-6 | 1.8E-3 | 1.8E-3 | 150 | 24 | 150 | 24 | 0.58 |
| An | U/mL | 6.0E1 | 9.5E1 | 2.1E2 | 5.1E2 | 5.6E2 | 1.6E3 | 2.8E-1 | 6.4E-1 | 5.5E3 | 7.8E3 | 150 | 24 | 150 | 24 | 0.58 |
| Ao | pg/mL | 9.1E1 | 1.2E2 | 5.7E2 | 4.2E2 | 3.8E3 | 9.4E2 | 1.5E0 | 4.1E0 | 3.9E4 | 4.5E3 | 150 | 24 | 150 | 24 | 0.60 |
| Ap | ng/mL | 3.3E1 | 4.8E1 | 4.7E1 | 7.3E1 | 5.3E1 | 7.0E1 | 2.0E0 | 4.4E0 | 3.3E2 | 2.4E2 | 150 | 24 | 150 | 24 | 0.61 |
| Ar | ng/mL | 5.9E-1 | 2.4E0 | 2.7E0 | 5.5E0 | 6.9E0 | 1.0E1 | 3.4E-3 | 3.4E-3 | 5.1E1 | 5.0E1 | 150 | 24 | 150 | 24 | 0.70 |
| As | ng/mL | 8.7E-3 | 3.7E-3 | 1.2E-2 | 6.3E-2 | 1.7E-2 | 2.5E-1 | 1.7E-3 | 1.7E-3 | 1.3E-1 | 1.2E0 | 150 | 24 | 150 | 24 | 0.47 |
| Aw | pg/mL | 1.6E1 | 1.8E1 | 1.7E1 | 2.0E1 | 5.7E0 | 8.6E0 | 2.9E-2 | 1.1E1 | 4.2E1 | 5.1E1 | 150 | 24 | 150 | 24 | 0.58 |
| Ax | ng/mL | 1.7E0 | 9.0E0 | 1.9E1 | 9.8E1 | 8.2E1 | 2.1E2 | 1.3E-2 | 1.0E-4 | 7.7E2 | 8.5E2 | 150 | 24 | 150 | 24 | 0.68 |
| Ba | ng/mL | 8.2E1 | 4.6E2 | 5.2E2 | 1.9E3 | 1.4E3 | 3.5E3 | 1.1E0 | 3.1E0 | 8.1E3 | 1.5E4 | 150 | 24 | 150 | 24 | 0.65 |
| Bb | ng/mL | 3.9E0 | 1.4E1 | 6.3E0 | 1.5E1 | 7.7E0 | 1.2E1 | 4.1E-3 | 3.5E-1 | 4.9E1 | 4.8E1 | 150 | 24 | 150 | 24 | 0.74 |
| Bc | ng/mL | 3.3E1 | 9.5E1 | 1.2E2 | 2.1E2 | 2.3E2 | 3.0E2 | 4.9E-1 | 2.9E0 | 1.2E3 | 1.0E3 | 150 | 24 | 150 | 24 | 0.66 |
| Bg | ng/mL | 1.1E-1 | 1.7E-1 | 6.9E0 | 1.6E0 | 3.7E1 | 2.3E0 | 5.3E-4 | 2.4E-2 | 4.0E2 | 8.0E0 | 150 | 24 | 150 | 24 | 0.59 |
| Bn | ng/mL | 5.6E-2 | 5.6E-2 | 1.3E0 | 3.0E0 | 2.1E0 | 1.2E1 | 5.6E-2 | 5.6E-2 | 8.6E0 | 5.8E1 | 150 | 24 | 150 | 24 | 0.47 |
| Bo | ng/mL | 1.3E1 | 1.6E1 | 1.5E1 | 1.7E1 | 1.1E1 | 1.3E1 | 1.6E-2 | 1.6E-2 | 5.0E1 | 5.3E1 | 150 | 24 | 150 | 24 | 0.53 |
| Ch | uIU/mL | 1.0E0 | 9.6E-1 | 3.9E1 | 5.1E0 | 1.9E2 | 1.1E1 | 3.4E-3 | 1.4E-1 | 1.8E3 | 5.0E1 | 150 | 24 | 150 | 24 | 0.47 |
| Co | pg/mL | 4.6E1 | 6.5E1 | 2.3E2 | 2.0E2 | 1.4E3 | 4.2E2 | 1.5E-1 | 8.8E0 | 1.7E4 | 2.1E3 | 150 | 24 | 150 | 24 | 0.64 |
| Cp | ng/mL | 2.2E1 | 2.3E1 | 2.7E1 | 8.7E1 | 2.1E1 | 2.6E2 | 6.0E-1 | 1.0E1 | 1.3E2 | 1.3E3 | 150 | 24 | 150 | 24 | 0.58 |
| Cq | ng/mL | 2.9E-2 | 3.3E-2 | 1.1E-1 | 2.2E0 | 4.6E-1 | 1.0E1 | 8.0E-4 | 8.0E-4 | 5.1E0 | 4.9E1 | 150 | 24 | 150 | 24 | 0.58 |
| Cs | ng/mL | 5.6E1 | 2.4E2 | 4.2E2 | 9.5E2 | 1.7E3 | 1.6E3 | 8.9E-1 | 8.3E-1 | 1.8E4 | 5.1E3 | 150 | 24 | 150 | 24 | 0.68 |
| Ct | ng/mL | 3.4E-1 | 3.0E-1 | 4.0E1 | 5.2E1 | 1.2E2 | 1.2E2 | 1.3E-2 | 1.1E-4 | 6.2E2 | 4.7E2 | 150 | 24 | 150 | 24 | 0.49 |
| Cu | ng/mL | 2.5E-1 | 4.1E-1 | 4.9E-1 | 4.6E0 | 9.2E-1 | 1.4E1 | 1.9E-2 | 1.7E-2 | 9.0E0 | 6.6E1 | 150 | 24 | 150 | 24 | 0.66 |
| Cv | ng/mL | 4.5E0 | 1.7E1 | 2.5E1 | 5.9E1 | 6.3E1 | 1.1E2 | 2.0E-2 | 5.1E-2 | 5.3E2 | 4.7E2 | 150 | 24 | 150 | 24 | 0.61 |
| Cw | mIU/mL | 3.1E-2 | 5.3E-2 | 4.2E-2 | 3.3E-1 | 3.5E-2 | 1.4E0 | 8.9E-4 | 4.1E-3 | 2.3E-1 | 6.8E0 | 150 | 24 | 150 | 24 | 0.63 |
| Cx | ng/mL | 1.1E0 | 1.5E-2 | 5.3E1 | 3.2E1 | 1.0E2 | 7.9E1 | 9.3E-5 | 9.3E-5 | 3.9E2 | 2.8E2 | 150 | 24 | 150 | 24 | 0.39 |
| Db | ug/mL | 7.5E0 | 7.8E0 | 8.9E0 | 7.7E0 | 7.9E0 | 4.4E0 | 4.5E-1 | 1.0E0 | 5.9E1 | 1.9E1 | 150 | 24 | 150 | 24 | 0.50 |
| Dc | nmol/L | 2.0E-2 | 4.4E-2 | 5.8E-2 | 8.0E-1 | 1.6E-1 | 2.9E0 | 5.2E-6 | 2.1E-3 | 1.6E0 | 1.4E1 | 150 | 24 | 150 | 24 | 0.68 |
| Dd | ug/mL | 6.7E-2 | 2.8E-1 | 1.7E-1 | 4.5E-1 | 2.5E-1 | 7.6E-1 | 4.8E-4 | 3.4E-3 | 1.6E0 | 3.6E0 | 150 | 24 | 150 | 24 | 0.62 |
| De | ng/mL | 3.4E-3 | 3.4E-3 | 7.8E-2 | 1.3E-1 | 1.3E-1 | 2.8E-1 | 3.4E-3 | 3.4E-3 | 9.2E-1 | 1.1E0 | 150 | 24 | 150 | 24 | 0.48 |
| Dg | ng/mL | 3.7E1 | 4.1E1 | 4.7E1 | 5.7E1 | 4.0E1 | 4.3E1 | 7.1E-1 | 1.9E0 | 1.9E2 | 1.2E2 | 150 | 24 | 150 | 24 | 0.57 |
| Di | pg/mL | 2.0E0 | 3.0E0 | 2.4E0 | 3.2E0 | 2.2E0 | 1.8E0 | 1.8E-1 | 1.8E-1 | 1.3E1 | 9.0E0 | 150 | 24 | 150 | 24 | 0.66 |
| Dk | uIU/mL | 1.4E-2 | 2.2E-2 | 5.3E-2 | 1.2E-1 | 1.6E-1 | 2.5E-1 | 1.1E-4 | 1.1E-4 | 1.6E0 | 9.8E-1 | 150 | 24 | 150 | 24 | 0.58 |
| Dl | ng/mL | 1.9E2 | 2.6E2 | 2.9E2 | 3.9E2 | 2.7E2 | 3.7E2 | 5.5E0 | 4.4E0 | 1.3E3 | 1.6E3 | 150 | 24 | 150 | 24 | 0.58 |
| Dp | ng/ml | 2.3E0 | 1.7E0 | 5.6E0 | 3.3E0 | 9.1E0 | 4.9E0 | 3.7E-3 | 3.7E-3 | 5.6E1 | 1.4E1 | 121 | 14 | 121 | 14 | 0.38 |
| Dr | pg/ml | 2.2E1 | 3.4E1 | 4.1E1 | 1.2E3 | 5.4E1 | 3.3E3 | 7.5E-1 | 7.5E-1 | 2.5E2 | 1.0E4 | 62 | 10 | 62 | 10 | 0.68 |
| Du | pg/ml | 1.4E2 | 9.6E2 | 9.3E2 | 4.0E3 | 2.9E3 | 8.6E3 | 1.2E0 | 1.2E0 | 2.0E4 | 2.4E4 | 50 | 7 | 50 | 7 | 0.65 |
| Ef | ng/ml | 1.0E-1 | 2.4E-1 | 8.0E-1 | 1.6E0 | 1.8E0 | 3.0E0 | 5.7E-4 | 5.7E-4 | 1.0E1 | 9.9E0 | 130 | 17 | 130 | 17 | 0.58 |
| Wm | % | 8.5E-2 | 8.5E-2 | 1.5E1 | 7.5E1 | 9.9E1 | 2.5E2 | 5.4E-2 | 8.5E-2 | 8.7E2 | 1.0E3 | 140 | 18 | 140 | 18 | 0.52 |
| Ed | pg/mL | 5.2E-1 | 6.1E1 | 3.0E1 | 9.5E1 | 5.8E1 | 1.3E2 | 5.2E-1 | 5.2E-1 | 5.0E2 | 4.8E2 | 121 | 14 | 121 | 14 | 0.71 |
| Tj | pg/mL | 3.7E-1 | 5.0E-1 | 3.9E1 | 3.4E1 | 2.2E2 | 6.9E1 | 3.7E-1 | 3.7E-1 | 2.3E3 | 2.5E2 | 129 | 16 | 129 | 16 | 0.58 |
| Po | pg/ml | 1.4E-1 | 5.6E0 | 8.4E0 | 2.5E1 | 2.7E1 | 4.8E1 | 8.0E-3 | 2.6E-2 | 2.7E2 | 2.2E2 | 308 | 45 | 308 | 45 | 0.67 |
| Ti | ug/mL | 3.2E0 | 9.0E0 | 4.6E0 | 8.7E0 | 3.9E0 | 6.1E0 | 1.2E-1 | 9.7E-1 | 1.6E1 | 1.8E1 | 76 | 8 | 76 | 8 | 0.69 |
| Em | ng/ml | 9.2E-3 | 2.6E-2 | 4.7E-2 | 2.2E-1 | 9.0E-2 | 5.3E-1 | 8.4E-4 | 8.4E-4 | 5.0E-1 | 1.9E0 | 79 | 13 | 79 | 13 | 0.54 |
| Et | ng/ml | 1.3E3 | 3.0E3 | 1.6E3 | 2.8E3 | 1.1E3 | 1.2E3 | 7.5E1 | 5.9E2 | 4.8E3 | 5.0E3 | 307 | 45 | 307 | 45 | 0.78 |
| Eq | pg/ml | 2.3E2 | 3.1E1 | 3.6E2 | 2.5E2 | 3.8E2 | 5.2E2 | 1.0E0 | 1.0E0 | 1.5E3 | 1.4E3 | 50 | 7 | 50 | 7 | 0.32 |
| Th | ug/mL | 1.2E0 | 1.6E0 | 1.7E0 | 2.3E0 | 1.5E0 | 2.2E0 | 1.2E-1 | 2.6E-3 | 7.5E0 | 5.9E0 | 76 | 8 | 76 | 8 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fa | ng/ml | 4.0E1 | 8.2E1 | 1.2E2 | 1.6E2 | 4.1E2 | 2.1E2 | 2.6E-1 | 6.0E-1 | 3.7E3 | 7.3E2 | 118 | 13 | 118 | 13 | 0.64 |
| Ez | ng/ml | 3.8E0 | 4.4E0 | 1.4E1 | 3.0E1 | 2.6E1 | 5.6E1 | 1.3E-2 | 1.3E-2 | 1.6E2 | 2.0E2 | 121 | 14 | 121 | 14 | 0.57 |
| Fb | ng/ml | 2.5E1 | 2.8E1 | 2.2E1 | 2.8E1 | 1.2E1 | 9.7E0 | 6.6E-1 | 4.6E0 | 4.3E1 | 4.1E1 | 119 | 13 | 119 | 13 | 0.64 |
| Ex | ng/ml | 7.5E-2 | 1.6E-1 | 1.8E-1 | 5.9E-1 | 3.2E-1 | 1.1E0 | 3.5E-5 | 1.7E-4 | 2.2E0 | 4.1E0 | 92 | 13 | 92 | 13 | 0.65 |
| Fc | pg/ml | 2.2E-1 | 6.3E0 | 2.0E1 | 5.4E1 | 8.2E1 | 1.1E2 | 2.2E-1 | 2.2E-1 | 4.5E2 | 3.1E2 | 51 | 7 | 51 | 7 | 0.69 |
| Fd | pg/ml | 5.3E1 | 4.9E2 | 5.7E2 | 7.0E3 | 1.4E3 | 1.1E4 | 4.5E-1 | 9.8E-1 | 9.2E3 | 2.5E4 | 51 | 7 | 51 | 7 | 0.67 |
| Fi | pg/ml | 2.5E-1 | 6.1E1 | 8.1E1 | 1.7E2 | 3.0E2 | 2.1E2 | 2.5E-1 | 2.5E-1 | 1.8E3 | 5.3E2 | 51 | 7 | 51 | 7 | 0.69 |
| Fn | ng/ml | 2.1E-1 | 9.5E-1 | 3.7E0 | 6.0E0 | 6.9E0 | 8.4E0 | 1.1E-14 | 2.1E-1 | 3.7E1 | 2.7E1 | 121 | 14 | 121 | 14 | 0.57 |
| Fp | ng/ml | 1.2E1 | 3.4E1 | 2.2E1 | 3.7E1 | 2.6E1 | 3.3E1 | 6.0E-3 | 1.2E0 | 1.3E2 | 1.3E2 | 310 | 45 | 310 | 45 | 0.66 |
| Fr | ng/ml | 3.1E4 | 1.1E5 | 1.1E5 | 2.6E5 | 1.8E5 | 2.7E5 | 1.9E2 | 1.8E3 | 8.4E5 | 8.4E5 | 315 | 47 | 315 | 47 | 0.72 |
| Fw | pg/ml | 2.2E0 | 1.2E1 | 4.4E1 | 6.8E1 | 2.7E2 | 1.3E2 | 1.2E-1 | 1.2E-1 | 3.0E3 | 4.4E2 | 132 | 17 | 132 | 17 | 0.67 |
| Fy | ng/ml | 3.4E1 | 1.1E2 | 5.9E1 | 1.9E2 | 7.0E1 | 2.3E2 | 1.2E-1 | 7.0E0 | 5.0E2 | 6.5E2 | 120 | 12 | 120 | 12 | 0.68 |
| Gh | pg/ml | 2.3E0 | 3.9E0 | 2.3E1 | 1.2E1 | 5.7E1 | 1.7E1 | 2.9E-2 | 2.9E-2 | 3.2E2 | 4.6E1 | 51 | 7 | 51 | 7 | 0.54 |
| Gb | % | 4.1E1 | 6.2E1 | 4.6E1 | 1.0E2 | 4.0E1 | 1.0E2 | 2.2E0 | 2.5E1 | 2.3E2 | 3.0E2 | 51 | 7 | 51 | 7 | 0.71 |
| Gc | ng/ml | 9.5E1 | 2.0E2 | 1.5E2 | 2.9E2 | 2.0E2 | 2.2E2 | 6.4E0 | 6.5E1 | 1.2E3 | 6.7E2 | 62 | 10 | 62 | 10 | 0.72 |
| Gd | ng/ml | 3.3E1 | 2.7E1 | 3.4E1 | 3.2E1 | 1.8E1 | 2.3E1 | 3.0E0 | 9.6E0 | 8.1E1 | 8.0E1 | 72 | 10 | 72 | 10 | 0.43 |
| Gn | U/ml | 2.1E-1 | 2.8E-1 | 1.3E0 | 1.2E1 | 4.0E0 | 3.6E1 | 5.6E-3 | 2.7E-2 | 3.0E1 | 1.1E2 | 61 | 10 | 61 | 10 | 0.58 |
| Gl | pg/ml | 9.2E3 | 8.9E3 | 1.2E4 | 1.5E4 | 9.4E3 | 1.2E4 | 9.1E1 | 5.2E2 | 3.2E4 | 3.2E4 | 132 | 17 | 132 | 17 | 0.58 |
| Gp | U/ml | 1.1E0 | 5.1E-1 | 2.6E0 | 1.3E0 | 3.5E0 | 1.9E0 | 1.5E-2 | 1.5E-2 | 2.0E1 | 7.3E0 | 132 | 16 | 132 | 16 | 0.37 |
| Gz | ug/ml | 1.4E0 | 1.2E0 | 5.7E0 | 3.3E0 | 5.8E0 | 4.3E0 | 4.2E-2 | 1.0E-1 | 2.5E1 | 1.1E1 | 84 | 10 | 84 | 10 | 0.44 |
| Ha | ng/ml | 2.0E0 | 5.5E0 | 7.6E0 | 1.7E1 | 1.7E1 | 3.4E1 | 1.7E-2 | 1.7E-2 | 1.0E2 | 1.0E2 | 119 | 14 | 119 | 14 | 0.61 |
| Nm | pg/ml | 1.2E4 | 3.2E4 | 2.9E4 | 9.4E4 | 7.5E4 | 1.6E5 | 1.0E-9 | 1.0E-9 | 9.6E5 | 8.2E5 | 311 | 45 | 311 | 45 | 0.66 |
| Nn | pg/ml | 1.4E2 | 4.9E2 | 1.4E3 | 1.4E4 | 6.9E3 | 5.0E4 | 1.0E-9 | 1.0E-9 | 9.5E4 | 3.1E5 | 311 | 45 | 311 | 45 | 0.68 |
| No | pg/ml | 1.3E1 | 3.9E1 | 2.9E1 | 1.2E2 | 5.7E1 | 2.1E2 | 1.0E-9 | 1.6E0 | 5.6E2 | 9.1E2 | 311 | 45 | 311 | 45 | 0.75 |
| Nq | pg/ml | 1.5E0 | 5.3E0 | 1.9E1 | 5.8E1 | 6.9E1 | 1.4E2 | 1.0E-9 | 1.0E-9 | 6.7E2 | 6.3E2 | 311 | 45 | 311 | 45 | 0.64 |
| Nr | pg/ml | 1.3E0 | 6.6E0 | 2.0E1 | 7.8E1 | 7.6E1 | 2.3E2 | 1.0E-9 | 1.0E-9 | 9.8E2 | 1.4E3 | 311 | 45 | 311 | 45 | 0.67 |
| Ns | pg/ml | 1.0E-9 | 1.0E-9 | 8.6E0 | 8.0E-2 | 6.8E1 | 5.3E-1 | 1.0E-9 | 1.0E-9 | 1.1E3 | 3.6E0 | 311 | 45 | 311 | 45 | 0.47 |
| Nt | pg/ml | 9.9E1 | 1.5E2 | 1.3E2 | 2.5E2 | 9.9E1 | 3.0E2 | 9.8E-1 | 5.5E1 | 8.8E2 | 1.7E3 | 311 | 45 | 311 | 45 | 0.68 |
| Nu | pg/ml | 1.2E1 | 5.9E1 | 5.3E1 | 8.8E1 | 9.2E1 | 9.5E1 | 1.0E-9 | 1.0E-9 | 6.3E2 | 3.7E2 | 311 | 45 | 311 | 45 | 0.67 |
| Lu | pg/ml | 1.0E4 | 1.0E4 | 1.6E4 | 1.2E4 | 4.2E4 | 1.0E4 | 7.7E2 | 5.2E2 | 5.6E5 | 5.7E4 | 311 | 45 | 311 | 45 | 0.51 |
| Lv | pg/ml | 1.0E-9 | 7.3E0 | 1.5E1 | 2.9E1 | 3.1E1 | 3.9E1 | 1.0E-9 | 1.0E-9 | 2.6E2 | 1.6E2 | 311 | 45 | 311 | 45 | 0.62 |
| Lw | pg/ml | 1.0E-9 | 1.0E-9 | 3.9E-1 | 6.7E0 | 4.9E0 | 2.7E1 | 1.0E-9 | 1.0E-9 | 8.0E1 | 1.8E2 | 311 | 45 | 311 | 45 | 0.57 |
| Lx | pg/ml | 1.0E-9 | 1.6E2 | 1.6E2 | 9.9E2 | 5.6E2 | 3.3E3 | 1.0E-9 | 1.0E-9 | 6.2E3 | 2.2E4 | 311 | 45 | 311 | 45 | 0.72 |
| Ly | pg/ml | 1.0E-9 | 1.0E-9 | 9.6E0 | 5.9E0 | 1.8E1 | 1.4E1 | 1.0E-9 | 1.0E-9 | 1.1E2 | 6.0E1 | 311 | 45 | 311 | 45 | 0.44 |
| Lz | pg/ml | 1.0E-9 | 1.0E-9 | 3.2E0 | 5.1E0 | 2.7E1 | 1.5E1 | 1.0E-9 | 1.0E-9 | 4.3E2 | 6.2E1 | 311 | 45 | 311 | 45 | 0.56 |
| Ma | pg/ml | 3.7E2 | 1.6E3 | 2.1E3 | 4.5E3 | 6.0E3 | 7.7E3 | 1.0E-9 | 2.4E1 | 6.5E4 | 3.6E4 | 311 | 45 | 311 | 45 | 0.69 |
| Mb | pg/ml | 2.5E1 | 2.8E1 | 3.2E1 | 3.2E1 | 1.8E1 | 1.4E1 | 9.2E0 | 4.1E0 | 2.1E2 | 6.4E1 | 311 | 45 | 311 | 45 | 0.52 |
| Mc | pg/ml | 1.0E-9 | 1.0E-9 | 6.1E-2 | 1.0E-9 | 7.8E-1 | 0.0E0 | 1.0E-9 | 1.0E-9 | 1.3E1 | 1.0E-9 | 311 | 45 | 311 | 45 | 0.49 |
| Md | pg/ml | 1.0E-9 | 1.0E-9 | 6.3E-1 | 9.2E-1 | 5.5E0 | 4.5E0 | 1.0E-9 | 1.0E-9 | 6.8E1 | 2.9E1 | 311 | 45 | 311 | 45 | 0.53 |
| Me | pg/ml | 3.1E1 | 3.7E1 | 3.0E1 | 3.5E1 | 2.4E1 | 2.8E1 | 1.0E-9 | 1.0E-9 | 3.2E2 | 1.8E2 | 311 | 45 | 311 | 45 | 0.59 |
| Mf | pg/ml | 1.0E-9 | 1.0E-9 | 6.8E-1 | 3.6E-1 | 4.1E0 | 1.1E0 | 1.0E-9 | 1.0E-9 | 5.6E1 | 4.5E0 | 311 | 45 | 311 | 45 | 0.51 |
| Mg | pg/ml | 9.3E-1 | 1.8E0 | 6.1E0 | 1.3E1 | 1.1E1 | 2.8E1 | 1.0E-9 | 1.0E-9 | 9.2E1 | 1.5E2 | 311 | 45 | 311 | 45 | 0.55 |
| Mh | pg/ml | 1.0E-9 | 1.0E-9 | 1.2E0 | 1.2E0 | 8.0E0 | 3.7E0 | 1.0E-9 | 1.0E-9 | 1.1E2 | 1.8E1 | 311 | 45 | 311 | 45 | 0.58 |
| Mi | pg/ml | 1.0E-9 | 1.0E-9 | 8.7E-1 | 2.4E1 | 8.3E0 | 9.1E1 | 1.0E-9 | 1.0E-9 | 1.2E2 | 5.2E2 | 311 | 45 | 311 | 45 | 0.60 |
| Mj | pg/ml | 1.0E-9 | 1.0E-9 | 5.9E0 | 1.6E1 | 3.2E1 | 4.3E1 | 1.0E-9 | 1.0E-9 | 4.6E2 | 2.1E2 | 311 | 45 | 311 | 45 | 0.59 |
| Mk | pg/ml | 9.1E-1 | 3.9E0 | 1.6E1 | 2.0E1 | 9.7E1 | 7.7E1 | 1.0E-9 | 1.0E-9 | 1.3E3 | 5.0E2 | 311 | 45 | 311 | 45 | 0.54 |
| Ml | pg/ml | 1.0E-9 | 1.0E-9 | 9.3E0 | 2.3E1 | 1.2E2 | 9.2E1 | 1.0E-9 | 1.0E-9 | 2.1E3 | 5.1E2 | 311 | 45 | 311 | 45 | 0.52 |
| Mm | pg/ml | 4.7E2 | 1.1E3 | 9.8E2 | 1.9E3 | 1.3E3 | 2.2E3 | 1.0E-9 | 1.0E-9 | 1.0E4 | 1.0E4 | 311 | 45 | 311 | 45 | 0.66 |
| Mn | pg/ml | 5.5E0 | 1.1E1 | 1.1E1 | 1.4E1 | 2.6E1 | 1.0E1 | 1.0E-9 | 1.1E0 | 3.5E2 | 5.1E1 | 311 | 45 | 311 | 45 | 0.70 |
| Mp | pg/ml | 1.0E-9 | 8.8E0 | 1.1E1 | 9.4E1 | 4.1E1 | 3.6E2 | 1.0E-9 | 1.0E-9 | 6.3E2 | 2.4E3 | 311 | 45 | 311 | 45 | 0.64 |
| Mq | pg/ml | 1.0E-9 | 1.0E-9 | 2.2E0 | 6.7E0 | 1.3E1 | 2.0E1 | 1.0E-9 | 1.0E-9 | 1.8E2 | 1.0E2 | 311 | 45 | 311 | 45 | 0.54 |
| Mr | pg/ml | 1.0E-9 | 2.8E0 | 2.1E1 | 2.0E2 | 1.2E2 | 6.3E2 | 1.0E-9 | 1.0E-9 | 1.5E3 | 3.4E3 | 311 | 45 | 311 | 45 | 0.62 |
| Ms | pg/ml | 3.3E2 | 2.6E2 | 4.7E2 | 4.3E2 | 5.4E2 | 7.3E2 | 1.0E-9 | 1.0E-9 | 3.3E3 | 4.7E3 | 311 | 45 | 311 | 45 | 0.45 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|-----|---------|-----|------|
| | | NonDis | Dis | NonDis | Dis | NonDis | Dis | NonDis | Dis | NonDis | Dis | NonDis | Dis | NonDis | Dis | |
| Mt | pg/ml | 1.7E-1 | 1.6E0 | 7.6E0 | 1.0E2 | 4.4E1 | 4.8E2 | 1.0E-9 | 1.0E-9 | 7.1E2 | 3.2E3 | 311 | 45 | 311 | 45 | 0.67 |
| Mu | pg/ml | 1.0E-9 | 1.0E-9 | 1.5E0 | 2.9E0 | 1.5E1 | 9.3E0 | 1.0E-9 | 1.0E-9 | 2.3E2 | 4.8E1 | 311 | 45 | 311 | 45 | 0.55 |
| Mv | pg/ml | 1.0E-9 | 1.0E-9 | 6.5E1 | 1.1E2 | 3.5E2 | 2.5E2 | 1.0E-9 | 1.0E-9 | 4.5E3 | 9.2E2 | 311 | 45 | 311 | 45 | 0.58 |
| Mw | pg/ml | 3.0E1 | 1.2E2 | 2.7E2 | 5.9E2 | 1.4E3 | 1.2E3 | 1.0E-9 | 1.0E-9 | 1.8E4 | 5.3E3 | 311 | 45 | 311 | 45 | 0.72 |
| Mx | pg/ml | 1.0E-9 | 1.0E-9 | 3.3E-1 | 1.1E0 | 2.0E0 | 3.2E0 | 1.0E-9 | 1.0E-9 | 3.2E1 | 2.0E1 | 311 | 45 | 311 | 45 | 0.60 |
| My | pg/ml | 1.0E-9 | 1.0E-9 | 3.5E2 | 1.6E2 | 2.6E3 | 4.1E2 | 1.0E-9 | 1.0E-9 | 3.9E4 | 1.9E3 | 311 | 45 | 311 | 45 | 0.56 |
| Mz | pg/ml | 1.1E1 | 3.0E1 | 2.4E1 | 1.2E2 | 5.0E1 | 3.4E2 | 1.0E-9 | 1.0E-9 | 5.5E2 | 1.9E3 | 311 | 45 | 311 | 45 | 0.75 |
| Na | pg/ml | 1.0E-9 | 1.0E-9 | 6.7E-1 | 1.5E0 | 1.9E0 | 6.4E0 | 1.0E-9 | 1.0E-9 | 9.6E0 | 4.2E1 | 311 | 45 | 311 | 45 | 0.52 |
| Nb | pg/ml | 2.1E0 | 3.3E0 | 3.5E0 | 1.3E1 | 1.0E1 | 3.5E1 | 1.0E-9 | 1.0E-9 | 1.5E2 | 2.1E2 | 311 | 45 | 311 | 45 | 0.63 |
| Nc | pg/ml | 3.4E2 | 5.3E1 | 5.2E2 | 4.0E2 | 7.4E2 | 6.2E2 | 1.0E-9 | 1.0E-9 | 9.2E3 | 3.2E3 | 311 | 45 | 311 | 45 | 0.41 |
| Nd | pg/ml | 2.7E1 | 3.7E1 | 2.7E1 | 8.1E1 | 7.1E1 | 3.1E2 | 1.0E-9 | 1.0E-9 | 1.2E3 | 2.1E3 | 311 | 45 | 311 | 45 | 0.63 |
| Ne | pg/ml | 4.2E2 | 4.0E2 | 5.2E2 | 4.8E2 | 5.6E2 | 5.8E2 | 1.0E-9 | 1.0E-9 | 7.0E3 | 3.6E3 | 311 | 45 | 311 | 45 | 0.46 |
| Nf | pg/ml | 1.0E-9 | 1.0E-9 | 2.5E0 | 6.7E0 | 9.0E0 | 2.6E1 | 1.0E-9 | 1.0E-9 | 8.2E1 | 1.3E2 | 311 | 45 | 311 | 45 | 0.49 |
| Ng | pg/ml | 1.2E1 | 1.7E1 | 9.0E1 | 8.5E1 | 1.9E2 | 1.3E2 | 1.0E-9 | 1.0E-9 | 1.6E3 | 5.3E2 | 311 | 45 | 311 | 45 | 0.54 |
| Nh | pg/ml | 6.3E1 | 5.5E1 | 8.1E1 | 6.7E1 | 7.5E1 | 8.1E1 | 1.0E-9 | 4.1E0 | 5.6E2 | 5.1E2 | 311 | 45 | 311 | 45 | 0.42 |
| Ni | pg/ml | 9.4E0 | 1.0E-9 | 8.3E1 | 1.0E2 | 1.3E2 | 2.1E2 | 1.0E-9 | 1.0E-9 | 7.1E2 | 1.1E3 | 311 | 45 | 311 | 45 | 0.45 |
| Nj | pg/ml | 7.2E0 | 6.7E0 | 1.1E1 | 9.2E0 | 1.2E1 | 9.5E0 | 1.0E-9 | 1.0E-9 | 6.9E1 | 4.6E1 | 311 | 45 | 311 | 45 | 0.46 |
| Nk | pg/ml | 1.8E1 | 1.2E1 | 3.1E1 | 3.6E1 | 3.6E1 | 5.3E1 | 1.0E-9 | 1.0E-9 | 2.0E2 | 2.0E2 | 311 | 45 | 311 | 45 | 0.48 |
| Nl | pg/ml | 4.3E1 | 3.9E1 | 5.8E1 | 4.6E1 | 7.8E1 | 4.9E1 | 1.0E-9 | 1.0E-9 | 1.1E3 | 2.3E2 | 311 | 45 | 311 | 45 | 0.43 |
| Ill | pg/ml | 4.6E0 | 1.1E1 | 3.2E1 | 5.3E2 | 5.5E1 | 1.4E3 | 1.0E-9 | 1.0E-9 | 3.0E2 | 3.6E3 | 51 | 7 | 51 | 7 | 0.57 |
| Ho | pg/ml | 1.8E1 | 3.3E1 | 2.2E1 | 1.1E2 | 2.0E1 | 1.4E2 | 1.0E-9 | 7.6E0 | 8.7E1 | 3.9E2 | 51 | 7 | 51 | 7 | 0.67 |
| Hp | ng/ml | 1.6E0 | 6.9E0 | 9.0E1 | 3.8E2 | 2.7E2 | 4.7E2 | 1.0E-9 | 8.8E-1 | 8.9E2 | 8.9E2 | 51 | 7 | 51 | 7 | 0.72 |
| Tz | pg/ml | 4.2E3 | 7.2E3 | 7.9E3 | 1.8E5 | 1.7E4 | 5.5E5 | 1.0E-9 | 6.3E2 | 1.7E5 | 2.1E6 | 121 | 14 | 121 | 14 | 0.63 |
| Ua | pg/ml | 3.3E3 | 7.1E3 | 3.2E4 | 1.6E4 | 1.9E5 | 2.6E4 | 1.0E-9 | 1.4E3 | 2.1E6 | 9.9E4 | 121 | 14 | 121 | 14 | 0.62 |
| Ub | pg/ml | 5.7E2 | 3.2E2 | 8.7E2 | 6.9E2 | 1.2E3 | 1.1E3 | 1.0E-9 | 2.3E0 | 9.8E3 | 4.1E3 | 121 | 14 | 121 | 14 | 0.37 |
| Ue | pg/ml | 2.7E1 | 2.6E1 | 3.5E1 | 4.5E1 | 3.1E1 | 4.5E1 | 9.8E-2 | 4.5E0 | 2.7E2 | 1.4E2 | 121 | 14 | 121 | 14 | 0.51 |
| Uc | pg/ml | 7.1E2 | 1.1E3 | 1.3E3 | 5.7E3 | 1.6E3 | 1.5E4 | 1.0E-9 | 4.1E1 | 9.4E3 | 5.7E4 | 121 | 14 | 121 | 14 | 0.57 |
| Ud | pg/ml | 1.0E-9 | 1.0E-9 | 3.2E0 | 3.8E0 | 3.5E1 | 1.4E1 | 1.0E-9 | 1.0E-9 | 3.9E2 | 5.3E1 | 121 | 14 | 121 | 14 | 0.53 |
| Hq | pg/ml | 1.2E0 | 9.1E-1 | 1.7E2 | 7.2E1 | 2.0E3 | 4.1E2 | 1.0E-9 | 1.0E-9 | 2.8E4 | 2.8E3 | 309 | 45 | 309 | 45 | 0.51 |
| Hr | pg/ml | 9.3E1 | 8.6E1 | 6.8E2 | 4.2E2 | 1.4E3 | 1.4E3 | 1.0E-9 | 1.0E-9 | 1.2E4 | 8.9E3 | 309 | 45 | 309 | 45 | 0.42 |
| Hu | pg/ml | 4.8E0 | 6.5E1 | 5.1E3 | 6.4E2 | 4.2E4 | 1.4E3 | 1.0E-9 | 1.0E-9 | 6.3E5 | 6.5E3 | 309 | 45 | 309 | 45 | 0.60 |
| Hv | pg/ml | 1.5E0 | 9.2E-1 | 2.5E0 | 2.7E1 | 5.7E0 | 1.3E2 | 1.0E-9 | 1.0E-9 | 6.9E1 | 8.9E2 | 309 | 45 | 309 | 45 | 0.47 |
| Hw | pg/ml | 6.4E0 | 5.7E0 | 1.5E1 | 2.4E2 | 4.7E1 | 1.4E3 | 1.0E-9 | 4.6E-1 | 6.4E2 | 9.4E3 | 309 | 45 | 309 | 45 | 0.48 |
| Hx | pg/ml | 8.8E0 | 1.2E1 | 5.6E1 | 7.3E1 | 5.3E2 | 2.2E2 | 1.0E-9 | 1.0E-9 | 9.3E3 | 1.3E3 | 309 | 45 | 309 | 45 | 0.58 |
| Ib | ng/ml | 3.9E-2 | 2.8E-2 | 1.1E0 | 4.1E0 | 4.7E0 | 1.5E1 | 1.0E-9 | 1.0E-9 | 3.6E1 | 5.6E1 | 119 | 14 | 119 | 14 | 0.49 |
| Ic | U/ml | 2.2E2 | 2.2E2 | 9.0E2 | 2.6E2 | 6.0E3 | 1.4E2 | 2.4E0 | 2.5E1 | 6.5E4 | 4.2E2 | 119 | 14 | 119 | 14 | 0.62 |
| Id | U/ml | 6.2E-1 | 1.2E0 | 1.3E0 | 3.3E1 | 2.0E0 | 1.2E2 | 1.0E-9 | 3.3E-1 | 1.3E1 | 4.3E2 | 119 | 14 | 119 | 14 | 0.68 |
| Tt | pg/ml | 1.7E2 | 1.7E2 | 1.7E2 | 1.9E2 | 5.4E1 | 8.6E1 | 4.3E1 | 1.1E2 | 3.6E2 | 4.4E2 | 111 | 13 | 111 | 13 | 0.53 |
| To | pg/ml | 1.6E0 | 1.8E0 | 2.1E0 | 2.6E0 | 2.9E0 | 3.1E0 | 1.0E-9 | 1.0E-9 | 2.3E1 | 1.2E1 | 117 | 14 | 117 | 14 | 0.55 |
| Tr | pg/ml | 3.2E0 | 5.2E0 | 8.5E0 | 1.2E1 | 3.0E1 | 2.0E1 | 1.0E-9 | 6.3E-1 | 3.1E2 | 7.6E1 | 115 | 13 | 115 | 13 | 0.61 |
| Tn | pg/ml | 3.0E1 | 5.4E1 | 1.2E2 | 2.7E2 | 3.3E2 | 6.0E2 | 1.0E-9 | 1.3E1 | 2.0E3 | 2.3E3 | 117 | 14 | 117 | 14 | 0.67 |
| Tv | ng/ml | 1.2E1 | 8.7E0 | 2.7E1 | 5.4E2 | 7.9E1 | 1.9E3 | 1.0E-9 | 1.0E-9 | 7.9E2 | 7.1E3 | 117 | 14 | 117 | 14 | 0.44 |
| Ih | ng/ml | 5.8E1 | 2.6E2 | 2.1E2 | 6.4E2 | 3.7E2 | 8.1E2 | 1.0E-9 | 2.4E0 | 2.5E3 | 3.6E3 | 310 | 45 | 310 | 45 | 0.69 |
| Ii | ng/ml | 7.8E1 | 1.3E2 | 2.0E2 | 4.1E2 | 4.8E2 | 8.7E2 | 7.3E-1 | 7.5E-1 | 5.2E3 | 4.5E3 | 310 | 45 | 310 | 45 | 0.62 |
| Ij | ng/ml | 7.3E1 | 1.6E2 | 1.7E2 | 8.3E2 | 5.9E2 | 3.6E3 | 2.8E0 | 9.5E0 | 6.4E3 | 2.4E4 | 307 | 44 | 307 | 44 | 0.75 |
| Ik | ng/ml | 1.1E1 | 1.3E1 | 1.4E3 | 1.7E2 | 1.2E4 | 3.5E2 | 5.9E-1 | 6.2E-1 | 1.2E5 | 1.5E3 | 307 | 44 | 307 | 44 | 0.55 |
| Il | ng/ml | 3.3E2 | 5.2E2 | 1.2E3 | 2.3E3 | 2.8E3 | 3.9E3 | 1.0E-9 | 1.0E-9 | 1.2E4 | 1.2E4 | 305 | 44 | 305 | 44 | 0.59 |
| Im | ng/ml | 2.0E2 | 7.0E2 | 3.5E2 | 1.6E3 | 5.2E2 | 2.6E3 | 1.4E1 | 2.2E1 | 6.0E3 | 1.5E4 | 306 | 45 | 306 | 45 | 0.80 |
| In | ng/ml | 3.6E0 | 3.0E0 | 2.0E1 | 1.3E2 | 9.2E1 | 6.7E2 | 1.0E-9 | 1.0E-9 | 1.1E3 | 4.5E3 | 310 | 45 | 310 | 45 | 0.50 |
| Hb | ng/ml | 2.1E1 | 5.1E1 | 3.1E1 | 7.0E1 | 3.1E1 | 6.0E1 | 4.8E-1 | 4.5E0 | 2.0E2 | 1.9E2 | 122 | 14 | 122 | 14 | 0.72 |
| Hc | pg/ml | 6.7E2 | 5.2E2 | 3.3E3 | 1.7E3 | 1.1E4 | 3.6E3 | 1.0E-9 | 1.0E-9 | 1.0E5 | 1.4E4 | 122 | 14 | 122 | 14 | 0.45 |
| Hf | ng/ml | 1.8E2 | 2.6E2 | 4.0E2 | 3.3E2 | 5.7E2 | 3.1E2 | 1.0E-9 | 1.0E-9 | 3.2E3 | 9.9E2 | 122 | 14 | 122 | 14 | 0.51 |
| Io | ng/ml | 7.7E3 | 2.0E4 | 1.9E4 | 2.4E4 | 4.8E4 | 2.3E4 | 1.0E-9 | 6.2E2 | 7.1E5 | 1.0E5 | 310 | 45 | 310 | 45 | 0.65 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ip | ng/ml | 8.4E0 | 3.0E1 | 2.0E1 | 3.2E1 | 2.6E1 | 2.1E1 | 1.0E-9 | 3.3E-1 | 2.3E2 | 7.8E1 | 310 | 45 | 310 | 45 | 0.69 |
| Iq | ug/ml | 9.9E-2 | 3.1E-1 | 4.5E1 | 7.3E0 | 7.7E2 | 3.3E1 | 1.0E-9 | 1.0E-9 | 1.4E4 | 2.2E2 | 310 | 45 | 310 | 45 | 0.66 |
| Ir | ug/ml | 3.5E-1 | 1.7E0 | 4.3E-1 | 2.0E1 | 3.3E1 | 6.1E1 | 1.0E-9 | 3.5E-2 | 5.1E2 | 3.7E2 | 309 | 45 | 309 | 45 | 0.73 |
| Is | ng/ml | 1.8E0 | 9.8E0 | 7.9E0 | 3.1E1 | 3.3E1 | 5.1E1 | 1.0E-9 | 3.3E-2 | 5.5E2 | 2.6E2 | 310 | 45 | 310 | 45 | 0.68 |
| It | ng/ml | 1.8E0 | 5.4E0 | 2.2E1 | 3.9E1 | 1.0E2 | 1.3E2 | 1.0E-9 | 1.0E-9 | 1.1E3 | 6.8E2 | 310 | 45 | 310 | 45 | 0.69 |
| Iu | ng/ml | 1.6E2 | 2.4E2 | 1.3E3 | 2.5E3 | 4.1E3 | 6.3E3 | 1.0E-9 | 1.0E-9 | 2.9E4 | 2.4E4 | 310 | 45 | 310 | 45 | 0.57 |
| Iv | ng/ml | 1.1E1 | 2.6E1 | 8.6E1 | 3.3E2 | 9.0E2 | 9.4E2 | 1.0E-9 | 1.0E-9 | 1.6E4 | 3.8E3 | 309 | 45 | 309 | 45 | 0.66 |
| Iz | ng/ml | 1.2E2 | 2.0E2 | 3.8E2 | 3.0E2 | 7.5E2 | 3.2E2 | 1.5E0 | 8.8E-1 | 6.1E3 | 1.0E3 | 122 | 14 | 122 | 14 | 0.54 |
| Yd | ng/ml | 2.7E-1 | 7.6E-2 | 4.3E-1 | 1.9E-1 | 5.4E-1 | 2.0E-1 | 6.6E-3 | 1.7E-2 | 2.3E0 | 5.6E-1 | 51 | 7 | 51 | 7 | 0.37 |
| Wb | pg/ml | 2.7E4 | 3.5E4 | 3.3E4 | 1.4E5 | 1.9E4 | 2.3E5 | 4.9E3 | 1.4E4 | 8.4E4 | 6.4E5 | 51 | 7 | 51 | 7 | 0.67 |
| Vz | pg/ml | 3.5E0 | 5.0E0 | 4.5E0 | 6.0E0 | 4.1E0 | 7.5E0 | 1.0E-9 | 7.6E-2 | 2.1E1 | 2.2E1 | 51 | 7 | 51 | 7 | 0.54 |
| Si | ng/ml | 1.2E0 | 2.7E0 | 1.8E0 | 3.2E0 | 2.4E0 | 2.2E0 | 8.6E-3 | 5.6E-1 | 1.0E1 | 6.0E0 | 51 | 7 | 51 | 7 | 0.72 |
| Sf | mIU/mL | 1.5E1 | 2.0E1 | 4.9E1 | 2.8E1 | 1.1E2 | 2.7E1 | 6.2E-1 | 6.7E0 | 7.2E2 | 8.3E1 | 51 | 7 | 51 | 7 | 0.56 |
| Sh | mIU/mL | 1.6E1 | 1.0E1 | 4.5E1 | 1.0E1 | 9.2E1 | 5.5E0 | 7.8E-2 | 4.2E0 | 5.7E2 | 2.1E1 | 51 | 7 | 51 | 7 | 0.40 |
| Sj | ng/ml | 4.4E-1 | 4.3E-1 | 4.3E-1 | 4.1E-1 | 9.8E-2 | 6.1E-2 | 2.5E-1 | 3.4E-1 | 7.2E-1 | 4.8E-1 | 51 | 7 | 51 | 7 | 0.48 |
| Rc | pg/ml | 6.6E3 | 7.2E3 | 7.9E3 | 6.5E3 | 6.1E3 | 3.3E3 | 3.9E2 | 1.1E3 | 3.9E4 | 1.3E4 | 121 | 14 | 121 | 14 | 0.48 |
| Rb | pg/ml | 7.1E-1 | 1.9E0 | 2.7E0 | 6.0E0 | 4.1E0 | 1.5E1 | 1.0E-9 | 1.0E-9 | 2.0E1 | 5.6E1 | 121 | 14 | 121 | 14 | 0.55 |
| Zw | 2.5ng/ml | 5.6E0 | 4.7E0 | 9.1E0 | 1.9E1 | 1.1E1 | 2.9E1 | 1.4E-1 | 7.7E-1 | 5.9E1 | 6.3E1 | 51 | 7 | 51 | 7 | 0.51 |
| Zx | 2.3mU/ml | 1.2E-1 | 1.8E-1 | 3.0E-1 | 2.4E-1 | 5.4E-1 | 2.0E-1 | 3.2E-2 | 7.7E-2 | 2.9E0 | 6.7E-1 | 51 | 7 | 51 | 7 | 0.64 |
| Pz | ng/ml | 3.2E3 | 1.0E4 | 5.4E3 | 7.0E3 | 6.1E3 | 4.8E3 | 1.6E1 | 4.0E1 | 7.0E4 | 2.5E4 | 306 | 45 | 306 | 45 | 0.62 |
| Qa | ng/ml | 3.4E3 | 9.4E3 | 6.4E3 | 1.8E4 | 7.5E3 | 3.3E4 | 1.5E2 | 6.8E2 | 4.2E4 | 2.2E5 | 306 | 45 | 306 | 45 | 0.71 |
| Qb | ng/ml | 1.0E2 | 2.1E2 | 2.1E2 | 4.4E2 | 3.9E2 | 6.5E2 | 7.9E-1 | 1.8E1 | 5.3E3 | 4.1E3 | 306 | 45 | 306 | 45 | 0.69 |
| Qc | ng/ml | 1.8E2 | 6.5E2 | 4.0E2 | 7.9E2 | 5.4E2 | 8.1E2 | 1.0E-9 | 1.0E-9 | 3.8E3 | 4.3E3 | 306 | 45 | 306 | 45 | 0.67 |
| Qd | ng/ml | 8.2E3 | 2.3E4 | 2.3E4 | 6.2E4 | 1.2E5 | 8.8E4 | 1.5E2 | 1.7E3 | 2.0E6 | 4.3E5 | 306 | 45 | 306 | 45 | 0.73 |
| Qe | ng/ml | 7.8E2 | 2.4E3 | 2.0E3 | 3.4E3 | 5.9E3 | 3.5E3 | 1.0E-9 | 8.8E0 | 9.7E4 | 1.8E4 | 306 | 45 | 306 | 45 | 0.70 |
| Jd | ng/ml | 8.8E-1 | 2.6E0 | 3.9E0 | 5.0E0 | 1.5E1 | 6.6E0 | 1.0E-9 | 1.0E-9 | 1.2E2 | 2.1E1 | 121 | 14 | 121 | 14 | 0.64 |
| Je | ng/ml | 1.0E-9 | 2.8E-1 | 1.7E0 | 2.4E0 | 5.0E0 | 3.5E0 | 1.0E-9 | 1.0E-9 | 4.6E1 | 1.1E1 | 121 | 14 | 121 | 14 | 0.57 |
| Jf | ng/ml | 1.0E-9 | 1.0E-9 | 1.3E0 | 4.8E-1 | 2.3E0 | 1.1E0 | 1.0E-9 | 1.0E-9 | 1.2E1 | 3.7E0 | 121 | 14 | 121 | 14 | 0.41 |
| Jg | ng/ml | 4.0E2 | 1.2E3 | 7.3E2 | 1.5E3 | 9.3E2 | 1.3E3 | 5.8E0 | 5.4E1 | 1.0E4 | 7.1E3 | 309 | 45 | 309 | 45 | 0.71 |
| Jh | ng/ml | 2.6E0 | 9.4E0 | 2.0E1 | 4.5E1 | 8.5E1 | 9.2E1 | 1.0E-9 | 1.0E-9 | 1.2E3 | 4.9E2 | 309 | 45 | 309 | 45 | 0.66 |
| Ji | ng/ml | 5.3E1 | 1.4E2 | 7.6E1 | 2.4E2 | 7.7E1 | 2.5E2 | 1.1E0 | 2.3E1 | 5.3E2 | 1.3E3 | 309 | 45 | 309 | 45 | 0.75 |
| Sr | pg/mL | 3.8E2 | 2.4E3 | 7.9E2 | 3.4E3 | 1.1E3 | 5.3E3 | 1.0E-9 | 9.7E1 | 5.5E3 | 2.1E4 | 120 | 14 | 120 | 14 | 0.76 |
| Ss | pg/mL | 9.3E4 | 4.9E4 | 1.5E5 | 1.2E5 | 1.8E5 | 1.5E5 | 2.7E3 | 7.8E3 | 1.3E6 | 5.7E5 | 120 | 14 | 120 | 14 | 0.41 |
| St | pg/mL | 2.4E7 | 1.0E8 | 6.0E7 | 1.8E8 | 1.3E8 | 4.2E8 | 1.0E-9 | 3.4E6 | 1.2E9 | 1.7E9 | 119 | 14 | 119 | 14 | 0.71 |
| Wc | ng/ml | 1.0E-9 | 1.0E-9 | 5.9E-2 | 3.5E-1 | 1.0E-1 | 6.8E-1 | 1.0E-9 | 1.0E-9 | 5.2E-1 | 1.8E0 | 51 | 7 | 51 | 7 | 0.51 |
| Wd | ng/ml | 9.3E0 | 1.8E1 | 2.7E1 | 1.2E2 | 5.8E1 | 1.9E2 | 1.0E0 | 3.5E0 | 2.9E2 | 4.1E2 | 51 | 7 | 51 | 7 | 0.69 |
| Wc | ng/ml | 3.2E-1 | 4.9E-1 | 1.1E0 | 3.8E0 | 1.8E0 | 8.4E0 | 1.0E-9 | 1.5E-1 | 9.7E0 | 2.3E1 | 51 | 7 | 51 | 7 | 0.61 |
| Wg | ng/ml | 1.0E-9 | 1.0E-9 | 3.2E-4 | 7.6E-2 | 2.3E-3 | 2.0E-1 | 1.0E-9 | 1.0E-9 | 1.6E-2 | 5.3E-1 | 51 | 7 | 51 | 7 | 0.56 |
| Wh | ng/ml | 1.0E-2 | 2.0E-2 | 4.3E-2 | 7.3E-2 | 9.6E-2 | 1.2E-1 | 1.0E-9 | 1.0E-9 | 4.2E-1 | 3.4E-1 | 51 | 7 | 51 | 7 | 0.59 |
| Wf | ng/ml | 1.0E-9 | 1.0E-9 | 1.0E-1 | 3.3E-1 | 2.1E-1 | 8.6E-1 | 1.0E-9 | 1.0E-9 | 1.2E0 | 2.3E0 | 51 | 7 | 51 | 7 | 0.40 |
| Ra | pg/ml | 1.0E-9 | 1.0E-9 | 3.8E-1 | 5.3E0 | 9.3E-1 | 1.7E1 | 1.0E-9 | 1.0E-9 | 3.8E0 | 6.4E1 | 121 | 14 | 121 | 14 | 0.55 |
| Qz | pg/ml | 9.8E0 | 1.1E1 | 5.2E1 | 4.7E1 | 8.7E1 | 7.8E1 | 1.0E-9 | 1.0E-9 | 4.5E2 | 2.3E2 | 121 | 14 | 121 | 14 | 0.50 |
| Qy | pg/ml | 3.6E-1 | 6.9E-1 | 7.6E0 | 5.4E1 | 4.5E1 | 2.0E2 | 1.0E-9 | 1.0E-9 | 4.3E2 | 7.3E2 | 121 | 14 | 121 | 14 | 0.66 |
| Qx | pg/ml | 1.0E-9 | 1.0E-9 | 2.8E0 | 1.2E1 | 1.8E1 | 2.9E1 | 1.0E-9 | 1.0E-9 | 1.9E2 | 1.1E2 | 121 | 14 | 121 | 14 | 0.55 |
| Qw | pg/ml | 1.0E-9 | 5.7E-1 | 3.0E0 | 1.0E0 | 9.9E0 | 1.6E0 | 1.0E-9 | 1.0E-9 | 6.6E1 | 5.6E0 | 121 | 14 | 121 | 14 | 0.54 |
| Qv | pg/ml | 1.9E4 | 1.1E4 | 3.8E4 | 1.5E4 | 9.5E4 | 1.5E4 | 1.0E-9 | 8.5E2 | 9.4E5 | 5.0E4 | 121 | 14 | 121 | 14 | 0.35 |
| Qu | pg/ml | 6.2E0 | 2.5E1 | 9.0E1 | 1.1E2 | 1.8E2 | 2.0E2 | 1.0E-9 | 1.0E-9 | 9.8E2 | 7.3E2 | 121 | 14 | 121 | 14 | 0.54 |
| Qt | pg/ml | 1.3E1 | 1.1E1 | 4.5E1 | 4.4E1 | 1.0E2 | 7.8E1 | 1.0E-9 | 1.0E-9 | 7.1E2 | 2.2E2 | 121 | 14 | 121 | 14 | 0.48 |
| Qh | ng/ml | 1.8E1 | 3.7E1 | 4.4E1 | 1.1E2 | 7.2E1 | 2.0E2 | 2.5E-1 | 3.3E0 | 4.6E2 | 8.0E2 | 121 | 14 | 121 | 14 | 0.68 |
| Qg | ng/ml | 7.3E0 | 8.1E0 | 1.4E1 | 1.4E1 | 2.6E1 | 2.1E1 | 1.5E-1 | 3.3E-1 | 2.7E2 | 8.1E1 | 121 | 14 | 121 | 14 | 0.47 |
| Jj | ng/ml | 5.6E2 | 2.5E2 | 2.1E3 | 4.7E2 | 1.9E4 | 5.0E2 | 2.3E0 | 8.7E0 | 3.4E5 | 1.9E3 | 309 | 45 | 309 | 45 | 0.35 |
| Jk | ng/ml | 2.6E0 | 5.1E0 | 2.0E1 | 3.3E1 | 4.8E1 | 5.7E1 | 1.0E-9 | 1.2E-1 | 3.9E2 | 2.4E2 | 309 | 45 | 309 | 45 | 0.61 |
| Jl | ng/ml | 4.8E-1 | 8.9E-1 | 2.0E0 | 2.2E2 | 4.8E0 | 1.5E3 | 1.2E-3 | 9.0E-2 | 4.0E1 | 9.9E3 | 309 | 45 | 309 | 45 | 0.65 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Jm | ng/ml | 1.8E1 | 4.5E1 | 6.1E1 | 1.1E2 | 1.4E2 | 3.1E2 | 1.0E-9 | 4.0E-1 | 1.4E3 | 2.1E3 | 309 | 45 | 309 | 45 | 0.61 |
| Jn | pg/ml | 3.0E-1 | 8.4E-1 | 3.4E0 | 3.8E1 | 3.6E1 | 1.4E2 | 1.0E-9 | 1.0E-9 | 6.2E2 | 7.3E2 | 309 | 45 | 309 | 45 | 0.70 |
| Jo | pg/ml | 3.7E3 | 4.7E3 | 4.7E3 | 8.4E3 | 3.9E3 | 1.6E4 | 2.0E1 | 2.4E1 | 2.4E4 | 1.0E5 | 309 | 45 | 309 | 45 | 0.55 |
| Jp | pg/ml | 6.9E4 | 9.9E4 | 7.1E4 | 1.0E5 | 3.5E4 | 5.7E4 | 5.8E2 | 2.8E4 | 1.9E5 | 3.8E5 | 309 | 45 | 309 | 45 | 0.71 |
| Jq | pg/ml | 9.4E1 | 1.4E2 | 1.6E2 | 5.5E2 | 2.0E2 | 1.4E3 | 1.0E0 | 5.6E0 | 2.0E3 | 8.7E3 | 309 | 45 | 309 | 45 | 0.61 |
| Jr | pg/ml | 3.6E0 | 1.5E1 | 5.5E1 | 3.6E2 | 6.1E2 | 1.4E3 | 1.0E-9 | 1.0E-9 | 1.1E4 | 7.4E3 | 309 | 45 | 309 | 45 | 0.68 |
| Js | pg/ml | 1.4E1 | 2.3E1 | 6.6E1 | 2.6E2 | 5.8E2 | 7.4E2 | 1.0E-9 | 2.7E0 | 1.0E4 | 3.0E3 | 309 | 45 | 309 | 45 | 0.71 |
| Jt | pg/ml | 2.3E3 | 4.3E3 | 2.8E3 | 6.9E3 | 2.2E3 | 9.9E3 | 2.2E1 | 1.5E2 | 2.2E4 | 5.2E4 | 309 | 45 | 309 | 45 | 0.69 |
| Xa | pg/ml | 1.0E-9 | 2.4E1 | 8.6E0 | 2.7E2 | 1.7E1 | 4.7E2 | 1.0E-9 | 2.9E0 | 9.6E1 | 1.2E3 | 51 | 7 | 51 | 7 | 0.83 |
| Ye | pg/ml | 1.0E-9 | 1.0E-9 | 4.5E0 | 1.0E0 | 1.4E1 | 2.7E0 | 1.0E-9 | 1.0E-9 | 6.9E1 | 7.2E0 | 51 | 7 | 51 | 7 | 0.43 |
| Tm | pg/ml | 1.0E-9 | 1.0E-9 | 2.7E0 | 1.2E0 | 4.5E0 | 3.2E0 | 1.0E-9 | 1.0E-9 | 1.8E1 | 8.4E0 | 51 | 7 | 51 | 7 | 0.42 |
| Tl | pg/ml | 1.1E-1 | 1.0E-9 | 3.1E-1 | 3.6E0 | 4.0E-1 | 9.3E0 | 1.0E-9 | 1.0E-9 | 1.8E0 | 2.5E1 | 51 | 7 | 51 | 7 | 0.42 |
| Ju | mIU/ml | 1.0E1 | 1.2E1 | 2.5E1 | 1.8E1 | 3.6E1 | 1.7E1 | 1.7E-1 | 1.9E0 | 2.3E2 | 6.0E1 | 121 | 14 | 121 | 14 | 0.56 |
| Jv | mIU/ml | 1.6E1 | 1.3E1 | 4.1E1 | 2.3E1 | 6.5E1 | 2.7E1 | 1.7E-2 | 1.1E0 | 4.4E2 | 8.9E1 | 121 | 14 | 121 | 14 | 0.48 |
| Jy | ng/ml | 1.6E-3 | 2.4E-3 | 2.3E-3 | 5.5E-3 | 4.0E-3 | 1.0E-2 | 1.0E-9 | 5.3E-4 | 3.9E-2 | 4.1E-2 | 121 | 14 | 121 | 14 | 0.65 |
| Kc | pg/ml | 2.3E1 | 2.8E1 | 4.4E1 | 6.2E1 | 5.2E1 | 8.6E1 | 1.0E-9 | 1.0E-9 | 2.7E2 | 3.2E2 | 122 | 14 | 122 | 14 | 0.54 |
| Kd | pg/ml | 1.0E-9 | 1.0E-9 | 1.8E2 | 3.7E3 | 6.1E2 | 1.0E4 | 1.0E-9 | 1.0E-9 | 4.8E3 | 3.8E4 | 122 | 14 | 122 | 14 | 0.64 |
| Ke | pg/ml | 1.2E4 | 3.6E4 | 1.4E4 | 5.7E4 | 9.9E3 | 8.1E4 | 6.7E2 | 3.5E3 | 5.5E4 | 3.2E5 | 122 | 14 | 122 | 14 | 0.79 |
| Kf | pg/mL | 6.3E0 | 6.8E0 | 7.0E0 | 1.2E1 | 6.1E0 | 2.0E1 | 1.0E-9 | 1.0E-9 | 4.4E1 | 7.8E1 | 122 | 14 | 122 | 14 | 0.55 |
| Kg | pg/mL | 9.6E2 | 1.0E3 | 1.8E3 | 5.3E3 | 2.6E3 | 1.1E4 | 7.7E1 | 1.3E2 | 2.2E4 | 3.6E4 | 122 | 14 | 122 | 14 | 0.46 |
| Ki | pg/ml | 6.0E1 | 7.6E1 | 6.9E1 | 9.2E1 | 5.1E1 | 6.4E1 | 1.0E-9 | 1.3E1 | 2.9E2 | 2.5E2 | 122 | 14 | 122 | 14 | 0.62 |
| Kj | pg/ml | 8.6E2 | 6.9E2 | 1.3E3 | 2.3E3 | 1.4E3 | 4.1E3 | 6.6E1 | 3.3E1 | 8.8E3 | 1.5E4 | 122 | 14 | 122 | 14 | 0.43 |
| Kk | pg/ml | 6.9E0 | 1.0E1 | 1.3E1 | 2.3E1 | 1.5E1 | 2.2E1 | 1.0E-9 | 2.0E0 | 8.1E1 | 5.9E1 | 122 | 14 | 122 | 14 | 0.64 |
| Kl | pg/ml | 1.8E4 | 1.6E4 | 2.8E4 | 2.2E4 | 2.7E4 | 1.7E4 | 2.3E2 | 6.2E2 | 1.3E5 | 5.1E4 | 122 | 14 | 122 | 14 | 0.47 |
| Kn | pg/ml | 2.9E1 | 5.7E1 | 6.4E1 | 4.5E2 | 9.8E1 | 1.3E3 | 1.0E-9 | 1.0E-9 | 6.3E2 | 4.9E3 | 122 | 14 | 122 | 14 | 0.60 |
| Ko | pg/ml | 3.4E2 | 6.8E2 | 4.8E2 | 8.1E2 | 5.7E2 | 1.0E3 | 1.0E-9 | 1.5E2 | 3.8E3 | 4.1E3 | 122 | 14 | 122 | 14 | 0.65 |
| Kp | pg/ml | 3.6E2 | 3.6E2 | 3.6E2 | 1.3E3 | 2.5E2 | 3.5E3 | 1.0E-9 | 3.7E1 | 9.8E2 | 1.3E4 | 122 | 14 | 122 | 14 | 0.56 |
| Kq | pg/ml | 3.2E2 | 6.1E2 | 4.4E2 | 1.3E4 | 4.8E2 | 4.2E4 | 1.6E0 | 7.0E1 | 3.6E3 | 1.6E5 | 117 | 14 | 117 | 14 | 0.75 |
| Kr | pg/ml | 2.9E-1 | 2.5E0 | 2.0E0 | 3.2E1 | 3.8E0 | 1.1E2 | 1.0E-9 | 1.0E-9 | 2.3E1 | 4.2E2 | 117 | 14 | 117 | 14 | 0.62 |
| Ks | pg/ml | 1.4E4 | 2.0E4 | 2.0E4 | 2.2E4 | 1.8E4 | 1.8E4 | 5.1E1 | 4.2E2 | 7.9E4 | 5.0E4 | 117 | 14 | 117 | 14 | 0.53 |
| Ps | ng/ml | 1.3E2 | 1.2E3 | 6.5E2 | 2.3E3 | 2.1E3 | 2.6E3 | 1.6E0 | 3.5E2 | 1.2E4 | 7.6E3 | 51 | 7 | 51 | 7 | 0.90 |
| Kx | ng/ml | 2.8E-4 | 9.0E-3 | 7.2E-3 | 1.9E-2 | 1.5E-2 | 2.4E-2 | 1.0E-9 | 1.0E-9 | 1.0E-1 | 6.5E-2 | 121 | 14 | 121 | 14 | 0.67 |
| Ky | ng/ml | 1.2E-1 | 3.7E-1 | 3.8E-1 | 6.4E-1 | 7.6E-1 | 7.8E-1 | 1.0E-9 | 1.0E-9 | 5.1E0 | 2.7E0 | 121 | 14 | 121 | 14 | 0.66 |
| Kz | ng/ml | 1.0E-9 | 2.4E-3 | 3.5E-3 | 7.5E-3 | 5.7E-3 | 8.8E-3 | 1.0E-9 | 1.0E-9 | 1.6E-2 | 2.5E-2 | 121 | 14 | 121 | 14 | 0.61 |
| Rz | ng/ml | 3.4E-1 | 3.3E-1 | 8.4E-1 | 1.5E0 | 1.2E0 | 2.7E0 | 4.6E-3 | 1.7E-2 | 6.7E0 | 7.5E0 | 51 | 7 | 51 | 7 | 0.54 |
| Ry | ng/ml | 1.6E-2 | 2.3E-2 | 2.4E-2 | 7.4E-2 | 2.6E-2 | 1.2E-1 | 1.0E-9 | 8.5E-3 | 1.2E-1 | 3.5E-1 | 51 | 7 | 51 | 7 | 0.64 |
| Rx | ng/ml | 1.0E-9 | 3.5E-5 | 1.4E-3 | 2.1E-3 | 2.3E-3 | 3.0E-3 | 1.0E-9 | 1.0E-9 | 8.6E-3 | 7.6E-3 | 51 | 7 | 51 | 7 | 0.63 |
| Ld | pg/ml | 1.0E-9 | 4.2E0 | 3.4E0 | 8.0E0 | 8.6E0 | 9.2E0 | 1.0E-9 | 1.0E-9 | 6.9E1 | 2.9E1 | 122 | 14 | 122 | 14 | 0.72 |
| Lh | pg/ml | 1.1E4 | 2.0E4 | 2.1E4 | 5.8E4 | 2.7E4 | 1.0E5 | 1.0E-9 | 1.3E3 | 2.6E5 | 4.8E5 | 310 | 45 | 310 | 45 | 0.65 |
| Li | pg/ml | 3.1E3 | 2.0E4 | 1.8E4 | 5.1E4 | 9.4E4 | 8.5E4 | 1.2E1 | 8.4E1 | 1.3E6 | 4.1E5 | 310 | 45 | 310 | 45 | 0.77 |
| Lj | pg/ml | 2.4E3 | 1.5E4 | 1.9E4 | 3.9E4 | 5.7E4 | 6.5E4 | 1.0E-9 | 8.9E1 | 4.3E5 | 3.9E5 | 310 | 45 | 310 | 45 | 0.70 |
| Lp | pg/ml | 1.1E1 | 1.1E0 | 6.6E1 | 4.5E2 | 1.8E2 | 6.0E2 | 1.0E-9 | 1.0E-9 | 1.1E3 | 1.4E3 | 51 | 7 | 51 | 7 | 0.51 |
| Lt | pg/ml | 1.0E-9 | 1.0E-9 | 1.7E0 | 1.0E-9 | 5.9E0 | 0.0E0 | 1.0E-9 | 1.0E-9 | 2.5E1 | 1.0E-9 | 51 | 7 | 51 | 7 | 0.46 |
| Rv | ng/ml | 5.0E-4 | 5.8E-4 | 1.2E-3 | 3.3E-3 | 2.5E-3 | 5.2E-3 | 1.0E-9 | 1.0E-9 | 1.6E-2 | 1.2E-2 | 51 | 7 | 51 | 7 | 0.51 |
| Ru | ng/ml | 1.0E-9 | 1.0E-9 | 1.0E-2 | 1.1E-1 | 5.2E-2 | 1.6E-1 | 1.0E-9 | 1.0E-9 | 3.1E-1 | 3.5E-1 | 51 | 7 | 51 | 7 | 0.70 |
| Rt | ng/ml | 8.0E-2 | 5.1E-2 | 1.2E-1 | 1.2E0 | 1.4E-1 | 2.8E0 | 2.2E-3 | 1.3E-3 | 6.3E-1 | 7.4E0 | 51 | 7 | 51 | 7 | 0.41 |
| Yl | pg/ml | 1.2E1 | 1.5E1 | 1.7E1 | 4.8E1 | 1.7E1 | 7.7E1 | 1.0E-9 | 1.0E-9 | 7.9E1 | 2.2E2 | 51 | 7 | 51 | 7 | 0.59 |
| Rm | ng/ml | 1.8E1 | 5.4E1 | 4.7E1 | 7.2E1 | 8.5E1 | 8.3E1 | 2.2E-1 | 2.3E-1 | 6.5E2 | 2.5E2 | 120 | 14 | 120 | 14 | 0.62 |
| Rh | ng/ml | 1.7E2 | 1.7E2 | 4.7E2 | 1.4E3 | 1.6E3 | 4.5E3 | 7.5E0 | 2.5E1 | 1.7E4 | 1.7E4 | 120 | 14 | 120 | 14 | 0.48 |
| Ri | ng/ml | 4.4E-2 | 1.0E-9 | 4.0E0 | 1.7E0 | 8.3E0 | 4.2E0 | 1.0E-9 | 1.0E-9 | 4.9E1 | 1.6E1 | 120 | 14 | 120 | 14 | 0.37 |
| Rg | ng/ml | 1.0E-9 | 1.0E-9 | 8.9E-2 | 1.7E-2 | 4.5E-1 | 5.5E-2 | 1.0E-9 | 1.0E-9 | 3.3E0 | 2.1E-1 | 120 | 14 | 120 | 14 | 0.49 |
| Rj | ng/ml | 1.0E-9 | 1.0E-9 | 9.1E-1 | 2.0E1 | 2.0E0 | 7.2E1 | 1.0E-9 | 1.0E-9 | 1.1E1 | 2.7E2 | 120 | 14 | 120 | 14 | 0.51 |
| Rf | ng/ml | 3.5E-1 | 1.0E0 | 7.7E-1 | 3.1E0 | 1.3E0 | 5.2E0 | 2.1E-2 | 7.0E-2 | 9.9E0 | 1.7E1 | 120 | 14 | 120 | 14 | 0.71 |

| Code | Units | Median | | Average | | Standard Deviation | | Minimum | | Maximum | | Number of Samples | | Number of Patients | | AUC |
|------|-------|--------|------|---------|------|---------|------|---------|------|---------|------|--------|-----|--------|-----|-----|
| | | NonDis | Dis | NonDis | Dis | NonDis | Dis | NonDis | Dis | NonDis | Dis | NonDis | Dis | NonDis | Dis | |
| Ql | pg/ml | 1.7E0 | 1.0E1 | 1.1E1 | 2.4E1 | 2.2E1 | 2.8E1 | 1.0E-9 | 1.0E-9 | 1.8E2 | 9.3E1 | 121 | 14 | 121 | 14 | 0.67 |
| Qm | pg/ml | 1.7E0 | 2.5E1 | 1.9E1 | 3.4E1 | 3.5E1 | 3.5E1 | 1.0E-9 | 1.0E-9 | 1.9E2 | 1.0E2 | 121 | 14 | 121 | 14 | 0.65 |
| Qn | pg/ml | 6.1E-1 | 8.5E-1 | 7.5E0 | 2.6E0 | 2.8E1 | 4.1E0 | 1.0E-9 | 1.0E-9 | 2.2E2 | 1.2E1 | 121 | 14 | 121 | 14 | 0.53 |
| Nv | pg/ml | 3.4E3 | 7.8E3 | 9.1E3 | 1.9E4 | 1.9E4 | 2.8E4 | 1.0E-9 | 1.6E2 | 1.6E5 | 1.2E5 | 311 | 45 | 311 | 45 | 0.70 |
| Nw | pg/ml | 8.6E3 | 2.0E4 | 1.2E4 | 3.2E4 | 1.6E4 | 4.3E4 | 1.9E2 | 4.5E3 | 2.1E5 | 2.2E5 | 311 | 45 | 311 | 45 | 0.80 |
| Nx | pg/ml | 2.2E2 | 4.2E2 | 4.1E2 | 6.9E2 | 6.1E2 | 8.2E2 | 1.0E-9 | 1.0E-9 | 3.5E3 | 4.1E3 | 311 | 45 | 311 | 45 | 0.59 |
| Ny | pg/ml | 5.7E0 | 2.0E1 | 1.0E2 | 1.3E2 | 1.4E3 | 4.2E2 | 1.0E-9 | 1.0E-9 | 2.5E4 | 2.8E3 | 311 | 45 | 311 | 45 | 0.73 |
| Oa | pg/ml | 1.6E2 | 7.9E2 | 4.2E2 | 1.0E3 | 7.0E2 | 1.0E3 | 1.0E-9 | 1.0E-9 | 4.5E3 | 3.4E3 | 121 | 14 | 121 | 14 | 0.68 |
| Op | pg/ml | 4.5E5 | 4.0E5 | 4.5E5 | 3.9E5 | 1.6E5 | 1.7E5 | 5.2E4 | 9.4E4 | 7.5E5 | 6.6E5 | 51 | 7 | 51 | 7 | 0.41 |
| Oe | pg/ml | 4.7E1 | 1.0E-9 | 2.6E2 | 1.6E2 | 3.9E2 | 3.0E2 | 1.0E-9 | 1.0E-9 | 2.3E3 | 1.1E3 | 310 | 44 | 310 | 44 | 0.43 |
| Of | pg/ml | 1.4E2 | 9.7E1 | 5.3E3 | 3.2E3 | 2.1E4 | 1.1E4 | 1.0E-9 | 1.0E-9 | 1.9E5 | 6.6E4 | 311 | 45 | 311 | 45 | 0.47 |
| Og | pg/ml | 7.7E-2 | 1.7E-2 | 3.9E-1 | 7.4E-2 | 1.6E0 | 1.5E-1 | 1.0E-9 | 1.0E-9 | 1.9E1 | 8.0E-1 | 311 | 45 | 311 | 45 | 0.35 |
| Oh | pg/ml | 2.4E0 | 7.2E0 | 1.4E1 | 4.0E2 | 9.1E1 | 2.4E3 | 1.0E-9 | 1.0E-9 | 1.4E3 | 1.6E4 | 311 | 45 | 311 | 45 | 0.63 |
| Oi | pg/ml | 2.0E0 | 2.0E0 | 5.1E0 | 4.7E0 | 8.0E0 | 6.8E0 | 1.0E-9 | 1.0E-9 | 6.7E1 | 3.1E1 | 311 | 45 | 311 | 45 | 0.50 |
| Ok | pg/ml | 3.6E2 | 6.7E2 | 5.0E2 | 1.3E3 | 5.0E2 | 1.6E3 | 1.5E1 | 5.3E1 | 4.2E3 | 7.8E3 | 311 | 45 | 311 | 45 | 0.73 |
| Om | pg/ml | 4.0E2 | 7.9E2 | 7.9E2 | 2.3E3 | 2.1E3 | 7.6E3 | 1.0E-9 | 7.0E1 | 3.0E4 | 5.1E4 | 311 | 45 | 311 | 45 | 0.66 |
| On | pg/ml | 1.6E2 | 3.3E2 | 2.7E2 | 8.6E2 | 4.0E2 | 1.4E3 | 1.0E-9 | 1.6E1 | 4.5E3 | 8.5E3 | 311 | 45 | 311 | 45 | 0.73 |
| Or | pg/ml | 1.2E1 | 1.9E1 | 3.6E1 | 9.6E1 | 6.9E1 | 1.7E2 | 1.0E-9 | 1.0E-9 | 4.4E2 | 5.1E2 | 123 | 14 | 123 | 14 | 0.56 |
| Ow | pg/ml | 4.2E1 | 9.8E1 | 1.9E2 | 5.1E2 | 7.9E2 | 8.5E2 | 1.0E-9 | 1.0E-9 | 8.1E3 | 3.0E3 | 123 | 14 | 123 | 14 | 0.63 |
| Ou | pg/ml | 5.1E2 | 7.7E2 | 1.1E3 | 2.3E3 | 1.9E3 | 3.0E3 | 1.0E-9 | 1.0E-9 | 1.1E4 | 8.2E3 | 123 | 14 | 123 | 14 | 0.56 |
| Ul | ng/ml | 1.0E-9 | 1.0E-9 | 1.0E0 | 5.5E0 | 4.3E0 | 1.6E1 | 1.0E-9 | 1.0E-9 | 2.9E1 | 5.6E1 | 122 | 14 | 122 | 14 | 0.54 |
| Uo | ng/ml | 1.0E-9 | 1.0E-9 | 7.7E-2 | 5.1E-2 | 2.1E-1 | 1.7E-1 | 1.0E-9 | 1.0E-9 | 1.3E0 | 6.3E-1 | 122 | 14 | 122 | 14 | 0.46 |
| Uk | ng/ml | 1.0E-9 | 1.0E-9 | 6.3E-3 | 3.2E-3 | 3.2E-2 | 8.6E-3 | 1.0E-9 | 1.0E-9 | 3.2E-1 | 3.1E-2 | 122 | 14 | 122 | 14 | 0.49 |
| Um | ng/ml | 1.0E-9 | 1.0E-9 | 2.3E-1 | 2.1E-1 | 6.1E-1 | 6.2E-1 | 1.0E-9 | 1.0E-9 | 3.6E0 | 2.3E0 | 122 | 14 | 122 | 14 | 0.45 |
| Uf | ng/ml | 5.8E-2 | 9.6E-2 | 1.4E-1 | 5.0E-1 | 2.0E-1 | 1.3E0 | 1.0E-3 | 3.5E-3 | 1.1E0 | 5.1E0 | 122 | 14 | 122 | 14 | 0.62 |
| Uh | ng/ml | 2.2E0 | 6.3E0 | 3.1E0 | 8.1E0 | 3.0E0 | 5.1E0 | 3.6E-2 | 7.1E-1 | 1.5E1 | 1.8E1 | 122 | 14 | 122 | 14 | 0.81 |
| Un | ng/ml | 1.7E0 | 3.2E0 | 1.9E0 | 4.7E0 | 1.2E0 | 6.2E0 | 3.4E-1 | 1.0E0 | 8.0E0 | 2.5E1 | 122 | 14 | 122 | 14 | 0.71 |
| Ug | ng/ml | 1.1E1 | 9.1E0 | 2.1E1 | 2.5E1 | 2.4E1 | 4.5E1 | 1.2E0 | 1.0E0 | 1.4E2 | 1.6E2 | 122 | 14 | 122 | 14 | 0.44 |
| Ur | ng/ml | 1.1E-1 | 1.0E-9 | 2.9E-1 | 1.0E0 | 5.0E-1 | 2.4E0 | 1.0E-9 | 1.0E-9 | 3.0E0 | 7.3E0 | 121 | 14 | 121 | 14 | 0.36 |
| Up | ng/ml | 1.0E-9 | 1.0E-9 | 2.9E-3 | 1.8E-1 | 8.9E-3 | 6.3E-1 | 1.0E-9 | 1.0E-9 | 5.3E-2 | 2.4E0 | 121 | 14 | 121 | 14 | 0.54 |
| Us | ng/ml | 3.6E-3 | 1.0E-9 | 1.7E-2 | 1.3E-1 | 4.4E-2 | 4.4E-1 | 1.0E-9 | 1.0E-9 | 3.6E-1 | 1.7E0 | 121 | 14 | 121 | 14 | 0.42 |
| Uv | ng/ml | 3.1E-3 | 1.0E-3 | 1.5E-2 | 3.2E-2 | 4.4E-2 | 1.1E-1 | 1.0E-9 | 1.0E-9 | 3.5E-1 | 4.1E-1 | 121 | 14 | 121 | 14 | 0.40 |
| Ut | ng/ml | 6.7E-1 | 1.7E0 | 2.6E0 | 9.4E0 | 6.0E0 | 1.8E1 | 1.0E-9 | 4.0E-1 | 5.2E1 | 6.5E1 | 121 | 14 | 121 | 14 | 0.73 |
| Uu | ng/ml | 7.1E0 | 4.9E0 | 7.7E0 | 5.4E0 | 5.2E0 | 3.1E0 | 5.4E-1 | 8.1E-1 | 2.9E1 | 1.2E1 | 121 | 14 | 121 | 14 | 0.38 |
| Uw | ng/ml | 2.3E0 | 4.0E0 | 2.8E0 | 9.0E0 | 2.5E0 | 1.3E1 | 1.0E-9 | 1.7E0 | 9.8E0 | 3.9E1 | 52 | 7 | 52 | 7 | 0.73 |
| Vb | ng/ml | 1.1E0 | 1.1E0 | 1.0E0 | 1.7E0 | 4.3E-1 | 2.1E0 | 8.5E-2 | 2.6E-1 | 2.0E0 | 6.4E0 | 52 | 7 | 52 | 7 | 0.48 |
| Vc | ng/ml | 1.0E-9 | 1.0E-9 | 4.1E-3 | 1.0E-9 | 1.8E-2 | 0.0E0 | 1.0E-9 | 1.0E-9 | 1.1E-1 | 1.0E-9 | 52 | 7 | 52 | 7 | 0.46 |
| Uy | ng/ml | 1.3E0 | 1.0E0 | 6.0E0 | 2.2E1 | 1.7E1 | 2.7E1 | 8.7E-2 | 2.0E-2 | 9.9E1 | 6.4E1 | 52 | 7 | 52 | 7 | 0.54 |
| Uz | ng/ml | 1.0E-9 | 1.0E-9 | 8.4E-3 | 4.8E0 | 6.0E-2 | 1.2E1 | 1.0E-9 | 1.0E-9 | 4.4E-1 | 3.3E1 | 52 | 7 | 52 | 7 | 0.63 |
| Ux | ng/ml | 1.8E2 | 1.6E2 | 1.9E2 | 1.8E2 | 1.4E2 | 1.5E2 | 4.5E0 | 4.0E1 | 4.8E2 | 4.9E2 | 52 | 7 | 52 | 7 | 0.47 |
| Va | ng/ml | 1.5E1 | 3.2E0 | 2.5E1 | 3.2E0 | 2.9E1 | 1.5E0 | 3.1E-1 | 1.2E0 | 1.2E2 | 5.7E0 | 52 | 7 | 52 | 7 | 0.21 |
| Vh | ng/ml | 1.1E-2 | 2.7E-2 | 1.9E-2 | 1.4E-1 | 2.5E-2 | 3.2E-1 | 3.9E-4 | 3.5E-3 | 1.2E-1 | 8.6E-1 | 52 | 7 | 52 | 7 | 0.69 |
| Vi | ng/ml | 3.3E-3 | 4.3E-2 | 9.4E-3 | 3.0E-1 | 1.8E-2 | 6.7E-1 | 1.0E-9 | 1.6E-2 | 1.2E-1 | 1.8E0 | 52 | 7 | 52 | 7 | 0.91 |
| Vp | ng/ml | 1.0E-9 | 1.0E-9 | 1.3E-1 | 3.9E-1 | 5.5E-1 | 1.3E1 | 1.0E-9 | 1.0E-9 | 3.9E0 | 4.9E1 | 122 | 14 | 122 | 14 | 0.54 |
| Vt | ng/ml | 6.0E0 | 1.1E1 | 7.7E0 | 2.5E1 | 6.4E0 | 3.9E1 | 5.6E-1 | 1.9E0 | 3.2E1 | 1.6E2 | 122 | 14 | 122 | 14 | 0.73 |
| Vu | ng/ml | 1.0E-9 | 3.6E-1 | 1.5E0 | 2.1E0 | 3.3E0 | 3.9E0 | 1.0E-9 | 1.0E-9 | 2.2E1 | 1.3E1 | 120 | 12 | 120 | 12 | 0.57 |
| Vq | ng/ml | 1.5E2 | 7.2E2 | 6.6E2 | 1.4E3 | 1.5E3 | 1.8E3 | 9.2E-1 | 6.5E-1 | 1.2E4 | 4.9E3 | 98 | 10 | 98 | 10 | 0.64 |
| Vo | ng/ml | 2.6E1 | 2.5E1 | 2.5E1 | 2.2E1 | 5.4E0 | 7.0E0 | 4.9E0 | 1.9E0 | 4.8E1 | 3.0E1 | 122 | 14 | 122 | 14 | 0.41 |
| Vs | ng/ml | 1.0E-9 | 1.0E-9 | 4.4E0 | 3.9E1 | 1.1E1 | 1.3E2 | 1.0E-9 | 1.0E-9 | 8.9E1 | 4.5E2 | 120 | 13 | 120 | 13 | 0.45 |
| Vv | ng/ml | 2.9E0 | 2.6E0 | 6.0E0 | 6.8E0 | 9.9E0 | 1.2E1 | 1.0E-9 | 1.0E-9 | 8.4E1 | 4.4E1 | 122 | 13 | 122 | 13 | 0.46 |
| Vw | ng/ml | 3.6E1 | 5.7E1 | 3.4E1 | 4.7E1 | 1.7E1 | 2.4E1 | 2.5E0 | 1.1E1 | 6.7E1 | 6.9E1 | 52 | 7 | 52 | 7 | 0.70 |
| Oy | pg/ml | 4.8E-1 | 4.3E-1 | 6.5E0 | 3.0E0 | 3.2E1 | 7.0E0 | 1.0E-9 | 1.0E-9 | 4.0E2 | 3.3E1 | 310 | 45 | 310 | 45 | 0.44 |
| Oz | pg/ml | 1.7E-2 | 1.0E-9 | 3.5E-1 | 7.2E-1 | 1.7E0 | 4.2E0 | 1.0E-9 | 1.0E-9 | 2.9E1 | 2.8E1 | 310 | 45 | 310 | 45 | 0.36 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pa | pg/ml | 3.8E-1 | 7.4E-1 | 1.3E0 | 9.6E0 | 5.9E0 | 3.7E1 | 1.0E-9 | 1.0E-9 | 8.6E1 | 2.3E2 | 310 | 45 | 310 | 45 | 0.63 |
| Pb | pg/ml | 1.0E-9 | 1.0E-9 | 1.8E0 | 8.1E-1 | 2.8E1 | 4.7E0 | 1.0E-9 | 1.0E-9 | 4.9E2 | 3.2E1 | 310 | 45 | 310 | 45 | 0.39 |
| Pc | pg/ml | 6.3E-2 | 1.0E-9 | 4.0E-1 | 8.4E0 | 9.2E-1 | 4.9E1 | 1.0E-9 | 1.0E-9 | 1.4E1 | 3.3E2 | 310 | 45 | 310 | 45 | 0.42 |
| Pd | pg/ml | 1.6E0 | 2.4E0 | 6.5E0 | 9.4E0 | 4.8E1 | 2.1E1 | 1.0E-9 | 1.0E-9 | 8.4E2 | 1.2E2 | 310 | 45 | 310 | 45 | 0.58 |
| Pe | pg/ml | 2.0E1 | 6.9E1 | 1.1E2 | 7.8E2 | 4.6E2 | 2.5E3 | 1.0E-9 | 1.0E-9 | 4.7E3 | 1.5E4 | 310 | 45 | 310 | 45 | 0.74 |
| Pf | pg/ml | 1.4E0 | 7.7E0 | 1.5E1 | 2.8E1 | 9.4E1 | 6.7E1 | 1.0E-9 | 1.0E-9 | 1.5E3 | 4.3E2 | 310 | 45 | 310 | 45 | 0.73 |
| Pg | pg/ml | 3.4E0 | 1.1E1 | 6.9E1 | 1.8E2 | 5.3E2 | 4.4E2 | 1.0E-9 | 1.0E-9 | 7.7E3 | 2.2E3 | 310 | 45 | 310 | 45 | 0.71 |
| Ph | ng/ml | 1.5E-1 | 2.1E-1 | 3.5E-1 | 6.8E-1 | 5.1E-1 | 1.4E0 | 1.0E-9 | 1.0E-9 | 2.8E0 | 5.4E0 | 123 | 14 | 123 | 14 | 0.53 |
| Pi | ng/ml | 1.9E-1 | 3.5E-1 | 2.9E-1 | 6.5E0 | 3.8E-1 | 2.2E1 | 1.0E-9 | 2.1E-2 | 3.2E0 | 8.2E1 | 123 | 14 | 123 | 14 | 0.65 |
| Pj | ng/mL | 5.1E0 | 7.3E0 | 5.9E0 | 8.7E0 | 4.4E0 | 5.7E0 | 4.0E-1 | 6.6E-1 | 3.1E1 | 2.3E1 | 123 | 14 | 123 | 14 | 0.66 |
| Pk | ng/ml | 8.8E-3 | 1.2E-2 | 1.5E-2 | 1.2E-1 | 2.9E-2 | 4.0E-1 | 1.0E-9 | 1.3E-4 | 2.5E-1 | 1.5E0 | 123 | 14 | 123 | 14 | 0.65 |
| aA | mg/dL | 8.8E-1 | 1.0E0 | 9.9E-1 | 1.5E0 | 4.9E-1 | 1.0E0 | 3.0E-1 | 5.0E-1 | 4.2E0 | 4.7E0 | 475 | 62 | 475 | 62 | 0.66 |
| aC | mg/mL | 2.2E0 | 2.4E0 | 2.6E0 | 2.9E0 | 1.3E0 | 1.6E0 | 7.5E-1 | 1.0E0 | 7.4E0 | 6.7E0 | 151 | 25 | 151 | 25 | 0.54 |
| aD | ug/mL | 3.1E0 | 2.3E0 | 4.8E0 | 4.2E0 | 4.8E0 | 4.0E0 | 7.5E-1 | 9.2E-1 | 3.5E1 | 1.8E1 | 151 | 25 | 151 | 25 | 0.43 |
| aE | mg/mL | 5.8E-1 | 5.5E-1 | 6.0E-1 | 5.5E-1 | 1.7E-1 | 1.4E-1 | 1.8E-1 | 2.2E-1 | 1.2E0 | 9.6E-1 | 151 | 25 | 151 | 25 | 0.41 |
| aF | ng/mL | 2.2E0 | 2.9E0 | 5.2E0 | 4.1E0 | 8.1E0 | 4.2E0 | 4.3E-3 | 4.3E-3 | 5.0E1 | 1.5E1 | 151 | 25 | 151 | 25 | 0.51 |
| aG | mg/mL | 1.4E-1 | 1.2E-1 | 1.6E-1 | 1.4E-1 | 9.0E-2 | 5.5E-2 | 3.2E-2 | 6.8E-2 | 4.8E-1 | 2.5E-1 | 151 | 25 | 151 | 25 | 0.45 |
| aH | ug/mL | 7.6E1 | 5.9E1 | 8.0E1 | 6.1E1 | 4.2E1 | 2.3E1 | 8.9E0 | 1.1E1 | 2.0E2 | 1.1E2 | 151 | 25 | 151 | 25 | 0.39 |
| aI | ug/mL | 1.8E2 | 1.4E2 | 1.8E2 | 1.5E2 | 6.2E1 | 5.0E1 | 3.2E1 | 7.6E1 | 3.4E2 | 2.7E2 | 151 | 25 | 151 | 25 | 0.38 |
| aJ | ug/mL | 2.4E0 | 3.6E0 | 3.1E0 | 4.7E0 | 2.2E0 | 4.5E0 | 8.2E-1 | 1.4E0 | 1.4E1 | 2.3E1 | 151 | 25 | 151 | 25 | 0.62 |
| aK | ng/mL | 1.3E0 | 1.3E0 | 2.0E0 | 1.6E0 | 2.0E0 | 1.5E0 | 2.9E-4 | 1.3E-1 | 1.0E1 | 6.5E0 | 151 | 25 | 151 | 25 | 0.46 |
| aL | mg/mL | 7.4E-1 | 7.3E-1 | 7.8E-1 | 6.8E-1 | 2.6E-1 | 2.4E-1 | 2.2E-1 | 2.7E-1 | 1.7E0 | 1.1E0 | 151 | 25 | 151 | 25 | 0.41 |
| aM | U/mL | 1.8E1 | 2.5E1 | 3.5E1 | 1.0E2 | 4.9E1 | 2.1E2 | 4.2E-2 | 4.2E-2 | 3.5E2 | 8.2E2 | 151 | 25 | 151 | 25 | 0.58 |
| aN | U/mL | 1.5E1 | 1.4E1 | 2.4E1 | 3.5E1 | 3.7E1 | 6.6E1 | 2.5E-3 | 2.5E-3 | 3.8E2 | 3.3E2 | 151 | 25 | 151 | 25 | 0.51 |
| aO | pg/mL | 4.9E1 | 1.5E2 | 4.3E2 | 4.9E2 | 1.0E3 | 7.5E2 | 6.0E-2 | 6.2E0 | 6.6E3 | 2.4E3 | 151 | 25 | 151 | 25 | 0.60 |
| aP | ng/mL | 1.6E0 | 1.9E0 | 2.2E0 | 3.7E0 | 2.5E0 | 5.4E0 | 4.5E-1 | 7.8E-1 | 2.8E1 | 2.8E1 | 151 | 25 | 151 | 25 | 0.61 |
| aQ | ng/mL | 2.5E-1 | 2.4E-1 | 3.6E-1 | 2.9E-1 | 3.3E-1 | 2.0E-1 | 2.0E-4 | 5.1E-2 | 2.0E0 | 9.0E-1 | 151 | 25 | 151 | 25 | 0.47 |
| aR | ng/mL | 1.8E0 | 1.7E0 | 2.9E0 | 3.7E0 | 4.0E0 | 4.2E0 | 2.6E-1 | 6.5E-1 | 3.4E1 | 1.7E1 | 151 | 25 | 151 | 25 | 0.54 |
| aS | ng/mL | 3.7E-1 | 4.1E-1 | 1.0E0 | 9.6E-1 | 2.8E0 | 1.2E0 | 4.2E-3 | 6.0E-2 | 3.3E1 | 4.9E0 | 151 | 25 | 151 | 25 | 0.54 |
| aU | pg/mL | 6.8E1 | 5.8E1 | 1.0E2 | 8.6E1 | 1.1E2 | 1.0E2 | 7.4E-2 | 7.4E-2 | 7.0E2 | 5.1E2 | 151 | 25 | 151 | 25 | 0.45 |
| aV | ng/mL | 5.9E-1 | 3.7E-1 | 1.1E0 | 6.9E-1 | 2.8E0 | 6.2E-1 | 7.6E-4 | 9.1E-2 | 3.3E1 | 2.4E0 | 151 | 25 | 151 | 25 | 0.45 |
| aW | pg/mL | 2.0E1 | 1.8E1 | 2.3E1 | 1.9E1 | 3.6E1 | 1.1E1 | 7.2E-2 | 7.2E-2 | 4.2E2 | 4.7E1 | 151 | 25 | 151 | 25 | 0.46 |
| aX | ng/mL | 8.2E0 | 7.1E0 | 1.4E1 | 3.2E1 | 2.1E1 | 6.7E1 | 3.0E-1 | 7.7E-1 | 2.2E2 | 3.1E2 | 151 | 25 | 151 | 25 | 0.49 |
| aY | pg/mL | 5.3E1 | 4.7E1 | 7.7E1 | 6.9E1 | 1.1E2 | 7.9E1 | 4.1E-1 | 4.1E-1 | 1.2E3 | 3.9E2 | 151 | 25 | 151 | 25 | 0.47 |
| aZ | pg/mL | 2.2E2 | 4.4E2 | 5.4E2 | 1.5E3 | 1.2E3 | 2.2E3 | 1.7E0 | 1.5E1 | 1.2E4 | 7.9E3 | 151 | 25 | 151 | 25 | 0.65 |
| bA | ng/mL | 1.3E1 | 4.6E1 | 5.4E1 | 2.0E2 | 1.2E2 | 3.6E2 | 3.0E-2 | 2.0E0 | 9.4E2 | 1.5E3 | 151 | 25 | 151 | 25 | 0.67 |
| bB | ng/mL | 2.8E2 | 2.4E2 | 3.1E2 | 2.5E2 | 1.8E2 | 1.4E2 | 2.1E0 | 3.3E1 | 9.5E2 | 5.7E2 | 151 | 25 | 151 | 25 | 0.40 |
| bC | ng/mL | 3.2E2 | 3.3E2 | 6.2E2 | 8.6E2 | 8.0E2 | 1.3E3 | 1.4E1 | 5.0E1 | 4.7E3 | 4.0E3 | 151 | 25 | 151 | 25 | 0.51 |
| bE | mg/mL | 5.1E0 | 5.2E0 | 5.4E0 | 5.8E0 | 2.1E0 | 2.7E0 | 1.8E0 | 1.3E0 | 1.2E1 | 1.2E1 | 151 | 25 | 151 | 25 | 0.53 |
| bF | pg/mL | 3.5E1 | 6.4E1 | 3.6E2 | 2.5E2 | 1.4E3 | 5.5E2 | 5.0E-2 | 8.9E0 | 1.1E4 | 2.2E3 | 151 | 25 | 151 | 25 | 0.62 |
| bG | ng/mL | 1.6E0 | 1.8E0 | 3.1E0 | 3.2E0 | 4.5E0 | 3.8E0 | 1.1E-1 | 1.6E-1 | 3.0E1 | 1.5E1 | 151 | 25 | 151 | 25 | 0.53 |
| bH | pg/mL | 5.7E-1 | 5.7E-1 | 6.4E0 | 5.3E0 | 2.5E1 | 6.7E0 | 5.7E-1 | 5.7E-1 | 2.8E2 | 2.3E1 | 151 | 25 | 151 | 25 | 0.56 |
| bI | ng/mL | 4.0E-3 | 4.0E-3 | 9.4E-2 | 8.6E-2 | 2.0E-1 | 1.8E-1 | 4.0E-3 | 4.0E-3 | 9.8E-1 | 6.2E-1 | 151 | 25 | 151 | 25 | 0.49 |
| bJ | mg/mL | 1.9E0 | 1.9E0 | 2.4E0 | 2.2E0 | 2.0E0 | 1.9E0 | 2.5E-4 | 2.5E-4 | 1.1E1 | 8.9E0 | 151 | 25 | 151 | 25 | 0.47 |
| bL | pg/mL | 4.1E0 | 3.5E0 | 9.4E0 | 8.2E0 | 1.2E1 | 8.4E0 | 4.6E-2 | 4.6E-2 | 6.0E1 | 3.0E1 | 151 | 25 | 151 | 25 | 0.52 |
| bM | mg/mL | 1.8E0 | 1.7E0 | 2.2E0 | 1.8E0 | 1.5E0 | 1.2E0 | 1.4E-1 | 1.6E-2 | 8.6E0 | 5.2E0 | 151 | 25 | 151 | 25 | 0.43 |
| bN | ng/mL | 3.4E1 | 2.4E1 | 1.3E2 | 5.1E1 | 3.0E2 | 6.4E1 | 1.4E-1 | 5.9E-1 | 1.9E3 | 2.5E2 | 151 | 25 | 151 | 25 | 0.41 |
| bO | ng/mL | 4.0E-2 | 4.0E-2 | 8.4E0 | 1.3E1 | 1.8E1 | 3.1E1 | 4.0E-2 | 4.0E-2 | 1.2E2 | 1.3E2 | 151 | 25 | 151 | 25 | 0.45 |
| bP | mg/mL | 5.2E-1 | 5.7E-1 | 7.7E-1 | 6.0E-1 | 7.4E-1 | 3.6E-1 | 4.9E-2 | 9.2E-2 | 4.8E0 | 1.6E0 | 151 | 25 | 151 | 25 | 0.48 |
| bQ | pg/mL | 2.1E1 | 5.9E1 | 1.5E2 | 6.7E1 | 1.1E3 | 5.7E1 | 1.5E-1 | 1.5E-1 | 1.3E4 | 2.2E2 | 151 | 25 | 151 | 25 | 0.70 |
| bR | ng/mL | 1.2E-2 | 1.2E-2 | 1.7E-1 | 9.8E-2 | 7.6E-1 | 1.3E-1 | 1.2E-2 | 1.2E-2 | 8.7E0 | 3.9E-1 | 151 | 25 | 151 | 25 | 0.52 |
| bS | pg/mL | 9.4E-1 | 9.4E-1 | 9.6E0 | 8.6E0 | 4.3E1 | 2.0E1 | 9.4E-1 | 9.4E-1 | 3.9E2 | 7.2E1 | 151 | 25 | 151 | 25 | 0.52 |
| bU | ng/mL | 6.8E-2 | 1.6E-1 | 1.9E-1 | 1.7E-1 | 5.7E-1 | 1.8E-1 | 1.3E-2 | 1.3E-2 | 6.6E0 | 6.5E-1 | 151 | 25 | 151 | 25 | 0.55 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bV | pg/mL | 4.9E2 | 5.5E2 | 6.2E2 | 7.3E2 | 9.3E2 | 5.1E2 | 1.6E2 | 2.7E2 | 1.2E4 | 2.2E3 | 151 | 25 | 151 | 25 | 0.60 |
| bW | pg/mL | 3.2E2 | 3.2E2 | 5.0E2 | 6.6E2 | 5.7E2 | 9.9E2 | 8.4E1 | 1.5E2 | 4.8E3 | 3.9E3 | 151 | 25 | 151 | 25 | 0.54 |
| bX | ng/mL | 2.5E-5 | 3.0E-3 | 2.6E-3 | 2.7E-3 | 3.2E-3 | 2.9E-3 | 2.5E-5 | 2.5E-5 | 1.3E-2 | 7.8E-3 | 151 | 25 | 151 | 25 | 0.52 |
| bZ | pg/mL | 2.7E2 | 6.4E2 | 2.1E3 | 1.4E3 | 7.6E3 | 1.9E3 | 1.5E-1 | 1.0E2 | 5.8E4 | 7.4E3 | 151 | 25 | 151 | 25 | 0.60 |
| cA | pg/mL | 6.0E-1 | 6.0E-1 | 4.3E0 | 2.6E0 | 3.0E1 | 5.3E0 | 6.0E-1 | 6.0E-1 | 3.7E2 | 2.1E1 | 151 | 25 | 151 | 25 | 0.52 |
| cB | ng/mL | 4.5E-2 | 4.7E-2 | 7.1E-2 | 9.4E-2 | 8.0E-2 | 1.2E-1 | 1.7E-3 | 1.7E-3 | 3.8E-1 | 4.3E-1 | 151 | 25 | 151 | 25 | 0.50 |
| cC | pg/mL | 4.1E1 | 3.9E1 | 4.6E1 | 4.1E1 | 5.2E1 | 3.0E1 | 1.0E0 | 1.0E0 | 4.5E2 | 1.1E2 | 151 | 25 | 151 | 25 | 0.49 |
| cD | pg/mL | 4.9E0 | 5.5E0 | 1.4E1 | 6.1E0 | 4.9E1 | 6.5E0 | 3.3E-1 | 3.3E-1 | 4.8E2 | 3.3E1 | 151 | 25 | 151 | 25 | 0.50 |
| cE | pg/mL | 5.5E1 | 9.9E1 | 2.8E2 | 1.9E2 | 6.3E2 | 2.3E2 | 1.2E-1 | 1.4E1 | 3.8E3 | 1.1E3 | 151 | 25 | 151 | 25 | 0.62 |
| cF | pg/mL | 5.3E-1 | 5.3E-1 | 1.6E1 | 1.3E1 | 3.1E1 | 1.9E1 | 5.3E-1 | 5.3E-1 | 2.7E2 | 6.5E1 | 151 | 25 | 151 | 25 | 0.48 |
| cG | pg/mL | 5.3E1 | 1.1E2 | 1.7E2 | 1.8E2 | 8.6E2 | 2.3E2 | 7.8E0 | 2.4E1 | 1.0E4 | 1.1E3 | 151 | 25 | 151 | 25 | 0.66 |
| cH | uIU/mL | 3.6E0 | 2.5E0 | 8.1E0 | 4.7E0 | 1.8E1 | 7.2E0 | 8.6E-3 | 8.6E-3 | 1.6E2 | 3.4E1 | 151 | 25 | 151 | 25 | 0.40 |
| cI | ng/mL | 6.0E0 | 9.6E0 | 1.4E1 | 1.7E1 | 2.0E1 | 2.6E1 | 3.2E-2 | 5.1E-1 | 1.2E2 | 1.2E2 | 151 | 25 | 151 | 25 | 0.52 |
| cJ | ug/mL | 6.7E1 | 4.6E1 | 1.0E2 | 7.2E1 | 1.1E2 | 7.5E1 | 6.9E0 | 5.6E0 | 6.4E2 | 3.4E2 | 151 | 25 | 151 | 25 | 0.41 |
| cK | ng/mL | 3.8E-3 | 3.8E-3 | 2.4E-2 | 1.8E-2 | 1.2E-1 | 4.3E-2 | 3.8E-3 | 3.8E-3 | 1.5E0 | 2.1E-1 | 151 | 25 | 151 | 25 | 0.52 |
| cL | pg/mL | 2.2E2 | 2.0E2 | 5.6E2 | 4.1E2 | 2.1E3 | 5.6E2 | 3.1E1 | 6.7E1 | 2.4E4 | 2.8E3 | 151 | 25 | 151 | 25 | 0.58 |
| cM | pg/mL | 2.7E2 | 2.8E2 | 2.9E2 | 2.7E2 | 1.7E2 | 1.0E2 | 2.5E1 | 5.7E1 | 1.1E3 | 4.5E2 | 151 | 25 | 151 | 25 | 0.50 |
| cN | pg/mL | 1.2E2 | 1.2E2 | 1.3E2 | 1.3E2 | 9.0E1 | 3.6E1 | 3.8E1 | 7.9E1 | 1.1E3 | 2.0E2 | 151 | 25 | 151 | 25 | 0.51 |
| cO | pg/mL | 2.1E2 | 2.8E2 | 4.2E2 | 3.5E2 | 1.6E3 | 3.0E2 | 5.4E1 | 9.6E1 | 1.9E4 | 1.5E3 | 151 | 25 | 151 | 25 | 0.59 |
| cP | ng/mL | 2.4E3 | 2.4E3 | 2.5E3 | 2.5E3 | 9.5E2 | 1.0E3 | 6.2E2 | 1.0E3 | 5.6E3 | 4.7E3 | 151 | 25 | 151 | 25 | 0.50 |
| cQ | ng/mL | 4.9E-2 | 7.1E-2 | 1.2E-1 | 1.6E-1 | 2.1E-1 | 2.3E-1 | 2.0E-3 | 2.0E-3 | 1.3E0 | 8.7E-1 | 151 | 25 | 151 | 25 | 0.54 |
| cR | ng/mL | 3.0E2 | 4.6E2 | 5.8E2 | 6.9E2 | 8.5E2 | 6.3E2 | 2.0E1 | 8.6E1 | 7.7E3 | 2.2E3 | 151 | 25 | 151 | 25 | 0.60 |
| cS | ng/mL | 2.9E2 | 3.8E2 | 4.1E2 | 8.8E2 | 4.3E2 | 1.5E3 | 4.1E1 | 9.7E1 | 2.5E3 | 7.1E3 | 151 | 25 | 151 | 25 | 0.57 |
| cT | ng/mL | 4.6E1 | 9.6E1 | 1.3E2 | 3.2E2 | 2.5E2 | 5.0E2 | 3.6E0 | 1.1E1 | 2.1E3 | 1.5E3 | 151 | 25 | 151 | 25 | 0.62 |
| cU | ng/mL | 5.7E1 | 9.8E1 | 9.5E1 | 1.2E2 | 1.5E2 | 9.7E1 | 6.2E0 | 1.7E1 | 1.6E3 | 3.9E2 | 151 | 25 | 151 | 25 | 0.64 |
| cV | ng/mL | 1.9E-1 | 2.1E-1 | 8.0E-1 | 2.6E-1 | 4.0E0 | 1.9E-1 | 2.5E-2 | 6.8E-2 | 4.7E1 | 9.3E-1 | 151 | 25 | 151 | 25 | 0.53 |
| cW | mIU/mL | 4.8E-2 | 6.3E-2 | 8.9E-2 | 8.8E-2 | 3.6E-1 | 8.9E-2 | 4.8E-3 | 1.4E-2 | 4.5E0 | 3.9E-1 | 151 | 25 | 151 | 25 | 0.57 |
| cX | ng/mL | 1.4E-1 | 6.9E-2 | 2.1E0 | 3.1E-1 | 6.0E0 | 5.6E-1 | 2.3E-4 | 2.3E-4 | 2.8E1 | 2.5E0 | 151 | 25 | 151 | 25 | 0.45 |
| cY | ng/mL | 7.4E0 | 8.1E0 | 1.1E1 | 9.1E0 | 1.1E1 | 8.2E0 | 1.7E-1 | 6.0E-1 | 6.1E1 | 3.6E1 | 151 | 25 | 151 | 25 | 0.47 |
| cZ | ug/mL | 1.3E1 | 1.2E1 | 1.5E1 | 1.4E1 | 6.8E0 | 7.1E0 | 2.3E0 | 3.3E0 | 4.6E1 | 3.0E1 | 151 | 25 | 151 | 25 | 0.46 |
| dA | pg/mL | 3.1E2 | 3.6E2 | 3.9E2 | 3.9E2 | 4.8E2 | 2.1E2 | 1.0E2 | 1.1E2 | 5.8E3 | 8.8E2 | 151 | 25 | 151 | 25 | 0.55 |
| dB | ug/mL | 1.8E1 | 1.7E1 | 1.9E1 | 1.7E1 | 2.1E1 | 8.6E0 | 2.1E0 | 2.2E0 | 2.5E2 | 2.8E1 | 151 | 25 | 151 | 25 | 0.48 |
| dC | nmol/L | 3.4E1 | 3.2E1 | 3.8E1 | 3.5E1 | 1.7E1 | 1.4E1 | 7.8E0 | 1.5E1 | 1.4E2 | 6.5E1 | 151 | 25 | 151 | 25 | 0.45 |
| dD | ug/mL | 3.4E1 | 3.0E1 | 3.6E1 | 3.3E1 | 1.1E1 | 1.1E1 | 1.4E1 | 1.6E1 | 7.4E1 | 6.0E1 | 151 | 25 | 151 | 25 | 0.40 |
| dE | ng/mL | 4.1E-1 | 7.0E-1 | 5.4E-1 | 8.4E-1 | 5.6E-1 | 8.0E-1 | 8.4E-3 | 8.4E-3 | 2.7E0 | 2.9E0 | 151 | 25 | 151 | 25 | 0.62 |
| dF | ng/mL | 2.4E2 | 3.7E2 | 3.3E2 | 4.7E2 | 2.4E2 | 3.0E2 | 7.5E1 | 1.1E2 | 1.3E3 | 1.2E3 | 151 | 25 | 151 | 25 | 0.67 |
| dG | ng/mL | 1.2E1 | 1.4E1 | 1.7E1 | 2.0E1 | 1.9E1 | 1.9E1 | 3.0E0 | 4.8E0 | 1.8E2 | 8.7E1 | 151 | 25 | 151 | 25 | 0.57 |
| dH | pg/mL | 7.9E0 | 1.1E1 | 2.2E1 | 1.3E1 | 6.6E1 | 1.4E1 | 4.0E-2 | 4.0E-2 | 6.7E2 | 7.6E1 | 151 | 25 | 151 | 25 | 0.57 |
| dI | pg/mL | 4.6E-1 | 4.6E-1 | 4.0E0 | 2.6E0 | 2.7E1 | 4.2E0 | 4.6E-1 | 4.6E-1 | 3.3E2 | 1.9E1 | 151 | 25 | 151 | 25 | 0.55 |
| dJ | ng/mL | 2.0E0 | 2.1E0 | 2.2E0 | 2.0E0 | 1.1E0 | 1.1E0 | 3.2E-2 | 3.2E-2 | 5.6E0 | 4.0E0 | 151 | 25 | 151 | 25 | 0.48 |
| dK | uIU/mL | 1.5E0 | 7.4E-1 | 2.2E0 | 1.7E0 | 3.7E0 | 2.6E0 | 2.8E-4 | 1.4E-2 | 3.9E1 | 1.1E1 | 151 | 25 | 151 | 25 | 0.35 |
| dL | ng/mL | 8.8E2 | 8.4E2 | 1.0E3 | 1.2E3 | 5.7E2 | 8.8E2 | 2.8E2 | 4.3E2 | 3.8E3 | 4.8E3 | 151 | 25 | 151 | 25 | 0.52 |
| dM | pg/mL | 9.6E2 | 1.1E3 | 1.3E3 | 1.9E3 | 1.5E3 | 1.5E3 | 3.7E2 | 6.3E2 | 1.5E4 | 5.8E3 | 151 | 25 | 151 | 25 | 0.62 |
| dN | ug/mL | 1.0E2 | 1.1E2 | 1.0E2 | 1.2E2 | 4.0E1 | 6.0E1 | 2.4E1 | 4.7E1 | 2.8E2 | 3.3E2 | 151 | 25 | 151 | 25 | 0.56 |
| dR | pg/ml | 1.5E3 | 8.3E2 | 2.2E3 | 1.1E3 | 2.1E3 | 1.2E3 | 1.4E2 | 1.3E2 | 9.8E3 | 5.3E3 | 116 | 17 | 116 | 17 | 0.32 |
| dX | ng/ml | 8.1E-2 | 8.2E-2 | 1.3E-1 | 1.4E-1 | 2.0E-1 | 1.5E-1 | 2.6E-3 | 2.6E-3 | 1.2E0 | 4.2E-1 | 51 | 10 | 51 | 10 | 0.55 |
| eF | ng/ml | 4.1E0 | 4.7E0 | 5.2E0 | 6.8E0 | 4.4E0 | 6.5E0 | 2.0E0 | 2.0E0 | 4.6E1 | 2.9E1 | 116 | 17 | 116 | 17 | 0.56 |
| eC | pg/ml | 2.9E2 | 2.5E2 | 3.6E2 | 3.7E2 | 2.6E2 | 5.4E2 | 9.9E0 | 1.9E1 | 1.6E3 | 2.0E3 | 105 | 12 | 105 | 12 | 0.36 |
| eD | pg/ml | 2.2E2 | 2.4E2 | 7.4E2 | 2.6E2 | 1.6E3 | 1.5E2 | 5.2E-1 | 5.9E1 | 8.3E3 | 4.9E2 | 87 | 8 | 87 | 8 | 0.53 |
| eM | ng/ml | 2.8E0 | 2.8E0 | 5.0E0 | 4.7E0 | 6.6E0 | 5.7E0 | 6.9E-1 | 8.8E-1 | 3.9E1 | 2.3E1 | 66 | 13 | 66 | 13 | 0.51 |
| eP | ng/ml | 3.7E-3 | 3.7E-3 | 1.2E0 | 1.2E0 | 4.1E0 | 3.1E0 | 3.7E-3 | 3.7E-3 | 2.8E1 | 1.0E1 | 51 | 10 | 51 | 10 | 0.49 |
| fP | ng/ml | 2.7E2 | 2.4E2 | 3.3E2 | 3.0E2 | 1.9E2 | 1.5E2 | 1.8E0 | 9.5E1 | 1.0E3 | 6.0E2 | 113 | 14 | 113 | 14 | 0.47 |
| fR | ng/ml | 1.3E5 | 2.5E5 | 2.1E5 | 3.0E5 | 1.7E5 | 2.3E5 | 3.6E4 | 1.9E2 | 6.9E5 | 8.7E5 | 95 | 20 | 95 | 20 | 0.63 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| gC | ng/ml | 2.3E2 | 2.8E2 | 2.5E2 | 3.1E2 | 1.1E2 | 1.5E2 | 8.3E1 | 1.5E2 | 6.4E2 | 5.9E2 | 38 | 9 | 38 | 9 | 0.60 |
| gL | pg/ml | 6.5E4 | 7.7E4 | 7.2E4 | 9.3E4 | 3.4E4 | 5.0E4 | 1.1E4 | 4.4E4 | 1.9E5 | 2.2E5 | 116 | 17 | 116 | 17 | 0.63 |
| gP | U/ml | 2.8E2 | 2.7E2 | 2.9E2 | 2.8E2 | 1.3E2 | 8.3E1 | 1.2E1 | 1.3E2 | 1.1E3 | 4.4E2 | 115 | 17 | 115 | 17 | 0.48 |
| gW | ng/ml | 6.1E2 | 3.4E2 | 9.4E2 | 6.6E2 | 1.1E3 | 9.0E2 | 2.3E0 | 1.5E2 | 6.1E3 | 3.1E3 | 89 | 10 | 89 | 10 | 0.40 |
| tF | pg/mL | 1.3E3 | 1.0E3 | 1.3E4 | 4.4E3 | 4.1E4 | 7.3E3 | 1.2E1 | 1.8E1 | 2.8E5 | 2.4E4 | 106 | 13 | 106 | 13 | 0.50 |
| hA | ng/ml | 2.5E0 | 3.6E0 | 1.4E1 | 1.8E1 | 5.1E1 | 3.6E1 | 1.7E-2 | 2.0E0 | 3.5E2 | 1.1E2 | 87 | 9 | 87 | 9 | 0.68 |
| nM | pg/ml | 1.0E-9 | 1.0E-9 | 1.0E-9 | 1.5E2 | 0.0E0 | 4.8E2 | 1.0E-9 | 1.0E-9 | 1.0E-9 | 1.5E3 | 64 | 10 | 64 | 10 | 0.55 |
| nN | pg/ml | 1.2E3 | 3.7E3 | 3.7E3 | 2.4E4 | 1.3E4 | 4.7E4 | 8.1E1 | 6.3E2 | 1.0E5 | 1.5E5 | 64 | 10 | 64 | 10 | 0.77 |
| nO | pg/ml | 2.4E1 | 3.4E1 | 3.8E1 | 3.7E1 | 4.9E1 | 2.3E1 | 4.0E0 | 1.2E1 | 3.1E2 | 8.9E1 | 64 | 10 | 64 | 10 | 0.60 |
| nR | pg/ml | 1.6E1 | 4.4E1 | 6.6E1 | 2.4E2 | 1.4E2 | 5.9E2 | 1.0E0 | 4.7E0 | 8.2E2 | 1.9E3 | 64 | 10 | 64 | 10 | 0.63 |
| nT | pg/ml | 6.5E1 | 1.1E2 | 1.0E2 | 2.1E2 | 1.1E2 | 2.6E2 | 1.0E-9 | 3.9E1 | 6.4E2 | 9.2E2 | 64 | 10 | 64 | 10 | 0.68 |
| nU | pg/ml | 3.5E1 | 1.0E2 | 8.9E1 | 2.0E2 | 2.1E2 | 2.8E2 | 1.0E-9 | 1.0E-9 | 1.5E3 | 9.2E2 | 64 | 10 | 64 | 10 | 0.73 |
| lW | pg/ml | 1.0E-9 | 1.0E-9 | 1.1E1 | 7.9E0 | 3.1E1 | 1.3E1 | 1.0E-9 | 1.0E-9 | 1.7E2 | 3.9E1 | 64 | 10 | 64 | 10 | 0.54 |
| lX | pg/ml | 9.1E2 | 7.3E2 | 1.0E3 | 9.3E2 | 5.6E2 | 7.3E2 | 2.3E2 | 1.9E2 | 2.6E3 | 2.5E3 | 64 | 10 | 64 | 10 | 0.42 |
| lY | pg/ml | 1.9E1 | 1.7E1 | 2.0E1 | 1.9E1 | 1.8E1 | 1.5E1 | 1.0E-9 | 5.7E-1 | 1.2E2 | 4.5E1 | 64 | 10 | 64 | 10 | 0.48 |
| mE | pg/ml | 1.0E-9 | 3.5E-1 | 2.6E0 | 2.3E0 | 8.0E0 | 3.3E0 | 1.0E-9 | 1.0E-9 | 5.7E1 | 9.2E0 | 64 | 10 | 64 | 10 | 0.56 |
| mF | pg/ml | 2.7E-1 | 4.0E-1 | 7.4E0 | 1.5E0 | 3.3E1 | 3.2E0 | 1.0E-9 | 1.0E-9 | 2.5E2 | 1.0E1 | 64 | 10 | 64 | 10 | 0.51 |
| mH | pg/ml | 3.1E0 | 4.3E0 | 5.4E0 | 5.5E0 | 8.2E0 | 5.3E0 | 4.0E-1 | 9.0E-1 | 5.3E1 | 1.9E1 | 64 | 10 | 64 | 10 | 0.56 |
| mI | pg/ml | 1.0E-9 | 2.3E1 | 1.1E1 | 7.0E1 | 2.6E1 | 1.4E2 | 1.0E-9 | 1.0E-9 | 1.8E2 | 4.6E2 | 64 | 10 | 64 | 10 | 0.70 |
| mM | pg/ml | 3.4E1 | 4.2E1 | 8.1E1 | 8.7E1 | 1.6E2 | 1.4E2 | 1.0E-9 | 1.0E-9 | 1.1E3 | 4.7E2 | 64 | 10 | 64 | 10 | 0.51 |
| mP | pg/ml | 1.5E1 | 1.6E1 | 1.8E1 | 1.1E2 | 1.6E1 | 2.5E2 | 1.0E-9 | 7.5E0 | 1.2E2 | 8.1E2 | 63 | 10 | 63 | 10 | 0.54 |
| mS | pg/ml | 1.6E3 | 1.3E3 | 1.8E3 | 1.6E3 | 9.9E2 | 1.1E3 | 1.0E-9 | 1.0E-9 | 5.1E3 | 3.5E3 | 64 | 10 | 64 | 10 | 0.43 |
| mT | pg/ml | 5.0E1 | 6.0E1 | 1.3E2 | 3.1E2 | 2.3E2 | 5.7E2 | 1.0E1 | 1.6E1 | 1.4E3 | 1.7E3 | 63 | 10 | 63 | 10 | 0.54 |
| mU | pg/ml | 2.2E0 | 3.8E0 | 6.5E0 | 5.7E0 | 2.8E1 | 6.6E0 | 1.0E-9 | 1.2E0 | 2.2E2 | 2.3E1 | 63 | 10 | 63 | 10 | 0.64 |
| mW | pg/ml | 2.2E3 | 1.9E3 | 2.4E3 | 3.2E3 | 1.3E3 | 3.1E3 | 1.0E-9 | 1.3E3 | 6.2E3 | 1.1E4 | 63 | 10 | 63 | 10 | 0.48 |
| mY | pg/ml | 6.5E2 | 8.8E2 | 8.6E2 | 1.6E3 | 9.7E2 | 2.3E3 | 1.0E-9 | 1.9E2 | 5.6E3 | 8.0E3 | 64 | 10 | 64 | 10 | 0.58 |
| mZ | pg/ml | 1.9E2 | 9.4E1 | 3.9E2 | 1.5E2 | 4.9E2 | 1.6E2 | 1.0E-9 | 1.1E1 | 3.1E3 | 5.5E2 | 63 | 10 | 63 | 10 | 0.29 |
| nA | pg/ml | 1.5E0 | 1.9E0 | 7.7E0 | 3.2E0 | 1.5E1 | 3.6E0 | 1.0E-9 | 1.0E-9 | 6.5E1 | 9.1E0 | 63 | 10 | 63 | 10 | 0.45 |
| nB | pg/ml | 2.8E2 | 3.5E2 | 3.0E2 | 3.8E2 | 1.6E2 | 2.4E2 | 3.0E1 | 1.3E2 | 8.2E2 | 9.6E2 | 64 | 10 | 64 | 10 | 0.59 |
| nC | pg/ml | 1.0E-9 | 5.5E2 | 1.0E4 | 1.9E3 | 5.5E4 | 3.1E3 | 1.0E-9 | 1.0E-9 | 3.8E5 | 9.1E3 | 64 | 10 | 64 | 10 | 0.68 |
| nD | pg/ml | 7.9E0 | 5.8E0 | 1.5E1 | 9.3E0 | 3.5E1 | 1.0E1 | 1.0E-9 | 1.0E-9 | 2.6E2 | 2.8E1 | 63 | 10 | 63 | 10 | 0.46 |
| nF | pg/ml | 1.0E-9 | 1.0E-9 | 3.4E0 | 1.3E0 | 1.4E1 | 4.2E0 | 1.0E-9 | 1.0E-9 | 9.3E1 | 1.3E1 | 64 | 10 | 64 | 10 | 0.49 |
| nH | pg/ml | 1.8E-1 | 4.7E0 | 2.1E2 | 2.5E1 | 1.3E3 | 4.0E1 | 1.0E-9 | 1.0E-9 | 1.0E4 | 1.2E2 | 63 | 10 | 63 | 10 | 0.69 |
| nI | pg/ml | 4.6E1 | 1.0E-9 | 7.9E1 | 1.0E-9 | 1.6E2 | 0.0E0 | 1.0E-9 | 1.0E-9 | 1.2E3 | 1.0E-9 | 64 | 10 | 64 | 10 | 0.20 |
| nJ | pg/ml | 1.7E-1 | 7.6E-1 | 3.2E0 | 9.4E-1 | 1.7E1 | 1.0E0 | 1.0E-9 | 1.0E-9 | 1.3E2 | 3.0E0 | 64 | 10 | 64 | 10 | 0.52 |
| nK | pg/ml | 1.0E-9 | 1.8E1 | 1.6E1 | 2.0E1 | 3.5E1 | 2.1E1 | 1.0E-9 | 1.0E-9 | 2.2E2 | 6.2E1 | 63 | 10 | 63 | 10 | 0.63 |
| nL | pg/ml | 1.0E-9 | 1.7E1 | 3.0E2 | 1.1E2 | 1.8E3 | 2.3E2 | 1.0E-9 | 1.0E-9 | 1.4E4 | 7.3E2 | 64 | 10 | 64 | 10 | 0.68 |
| hR | pg/ml | 2.7E4 | 2.6E4 | 2.9E4 | 2.9E4 | 1.2E4 | 9.4E3 | 1.0E-9 | 1.8E4 | 5.8E4 | 4.3E4 | 81 | 8 | 81 | 8 | 0.49 |
| hV | pg/ml | 4.4E2 | 4.4E2 | 4.5E2 | 4.4E2 | 2.3E2 | 2.3E2 | 1.0E-9 | 9.5E1 | 1.2E3 | 7.4E2 | 81 | 8 | 81 | 8 | 0.50 |
| hW | pg/ml | 1.8E3 | 3.1E3 | 2.1E3 | 7.6E3 | 1.1E3 | 1.3E4 | 1.0E-9 | 1.5E3 | 7.3E3 | 4.0E4 | 81 | 8 | 81 | 8 | 0.76 |
| hX | pg/ml | 1.0E3 | 1.3E3 | 1.1E3 | 1.2E3 | 9.5E2 | 3.8E2 | 2.5E0 | 7.0E2 | 8.6E3 | 2.0E3 | 81 | 8 | 81 | 8 | 0.67 |
| iA | pg/ml | 1.6E2 | 1.8E2 | 2.5E2 | 2.8E2 | 2.8E2 | 2.5E2 | 1.5E1 | 5.6E1 | 1.8E3 | 8.7E2 | 105 | 14 | 105 | 14 | 0.55 |
| iB | ng/ml | 4.9E0 | 8.4E0 | 6.2E0 | 1.0E1 | 4.8E0 | 6.4E0 | 3.3E-2 | 2.4E0 | 2.4E1 | 2.2E1 | 87 | 9 | 87 | 9 | 0.70 |
| iC | U/ml | 3.1E-1 | 5.3E-1 | 1.2E0 | 7.7E-1 | 5.9E0 | 8.3E-1 | 1.0E-9 | 6.8E-2 | 5.5E1 | 2.8E0 | 87 | 9 | 87 | 9 | 0.63 |
| iH | ng/ml | 1.6E5 | 1.8E5 | 1.6E5 | 1.6E5 | 4.9E4 | 5.5E4 | 2.9E3 | 7.7E4 | 2.6E5 | 2.4E5 | 105 | 14 | 105 | 14 | 0.52 |
| iJ | ng/ml | 4.9E4 | 3.8E4 | 5.6E4 | 4.6E4 | 3.7E4 | 2.2E4 | 1.8E3 | 1.2E4 | 2.5E5 | 9.3E4 | 105 | 14 | 105 | 14 | 0.43 |
| hB | ng/ml | 4.6E-1 | 6.2E-1 | 6.1E-1 | 8.5E-1 | 4.9E-1 | 5.5E-1 | 1.2E-1 | 2.9E-1 | 3.2E0 | 2.4E0 | 105 | 14 | 105 | 14 | 0.69 |
| hC | pg/ml | 5.1E3 | 8.9E3 | 7.7E3 | 1.2E4 | 1.2E4 | 1.4E4 | 4.1E1 | 3.0E2 | 1.1E5 | 5.7E4 | 105 | 14 | 105 | 14 | 0.61 |
| hF | ng/ml | 1.0E-9 | 1.0E-9 | 3.8E1 | 1.0E-9 | 3.9E2 | 0.0E0 | 1.0E-9 | 1.0E-9 | 4.0E3 | 1.0E-9 | 105 | 14 | 105 | 14 | 0.50 |
| hG | pg/ml | 6.8E3 | 6.7E3 | 7.4E3 | 8.9E3 | 3.1E3 | 5.3E3 | 1.8E3 | 3.6E3 | 1.8E4 | 2.0E4 | 105 | 14 | 105 | 14 | 0.53 |
| iO | ng/ml | 3.8E5 | 4.2E5 | 4.2E5 | 4.2E5 | 2.0E5 | 2.0E5 | 1.1E4 | 1.0E5 | 1.1E6 | 8.2E5 | 105 | 14 | 105 | 14 | 0.51 |
| iP | ng/ml | 5.3E4 | 4.6E4 | 5.8E4 | 5.0E4 | 4.8E4 | 1.9E4 | 1.0E-9 | 2.2E4 | 4.4E5 | 7.0E4 | 105 | 14 | 105 | 14 | 0.46 |
| iZ | ng/ml | 1.6E3 | 2.1E3 | 1.8E3 | 2.5E3 | 8.5E2 | 1.1E3 | 6.6E2 | 1.1E3 | 5.7E3 | 4.6E3 | 103 | 14 | 103 | 14 | 0.70 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| rC | pg/ml | 1.5E3 | 1.9E3 | 2.2E3 | 1.7E3 | 2.4E3 | 9.5E2 | 1.0E-9 | 6.0E2 | 1.5E4 | 2.9E3 | 79 | 7 | 79 | 7 | 0.49 |
| rB | pg/ml | 3.1E1 | 7.7E1 | 4.7E1 | 1.1E2 | 6.1E1 | 1.2E2 | 1.0E-9 | 1.0E-9 | 3.9E2 | 3.2E2 | 79 | 7 | 79 | 7 | 0.65 |
| jD | ng/ml | 2.9E1 | 5.0E1 | 3.8E1 | 9.1E1 | 3.8E1 | 9.8E1 | 1.0E-9 | 7.6E0 | 1.9E2 | 2.9E2 | 87 | 9 | 87 | 9 | 0.66 |
| jE | ng/ml | 1.0E-9 | 1.0E-9 | 5.2E0 | 9.4E0 | 1.2E1 | 1.5E1 | 1.0E-9 | 1.0E-9 | 6.2E1 | 3.5E1 | 87 | 9 | 87 | 9 | 0.57 |
| jF | ng/ml | 4.2E1 | 7.7E0 | 5.0E1 | 3.1E1 | 5.4E1 | 4.7E1 | 1.0E-9 | 1.0E-9 | 2.1E2 | 1.2E2 | 87 | 9 | 87 | 9 | 0.40 |
| jG | ng/ml | 4.3E3 | 4.2E3 | 4.5E3 | 4.6E3 | 2.0E3 | 1.5E3 | 6.7E2 | 2.5E3 | 9.6E3 | 7.9E3 | 87 | 9 | 87 | 9 | 0.52 |
| jH | ng/ml | 7.5E1 | 1.2E2 | 7.9E1 | 1.4E2 | 4.3E1 | 1.2E2 | 1.3E1 | 3.3E1 | 2.4E2 | 4.3E2 | 87 | 9 | 87 | 9 | 0.70 |
| jI | ng/ml | 7.3E1 | 1.2E2 | 7.6E1 | 1.6E2 | 3.2E1 | 1.2E2 | 1.9E1 | 4.4E1 | 1.9E2 | 4.4E2 | 87 | 9 | 87 | 9 | 0.75 |
| rA | pg/ml | 2.4E1 | 2.0E1 | 3.1E1 | 2.7E1 | 2.8E1 | 1.8E1 | 1.0E-9 | 1.2E1 | 2.0E2 | 6.8E1 | 85 | 8 | 85 | 8 | 0.48 |
| qY | pg/ml | 1.8E1 | 9.0E0 | 4.1E1 | 1.3E1 | 5.8E1 | 9.0E0 | 8.7E-1 | 2.1E0 | 3.3E2 | 2.7E1 | 85 | 8 | 85 | 8 | 0.32 |
| qX | pg/ml | 5.5E1 | 7.8E1 | 7.0E1 | 9.0E1 | 4.8E1 | 6.2E1 | 1.0E-9 | 2.3E1 | 2.3E2 | 2.1E2 | 85 | 8 | 85 | 8 | 0.59 |
| qW | pg/ml | 7.8E0 | 6.8E0 | 1.2E1 | 9.4E0 | 1.6E1 | 8.6E0 | 1.0E-9 | 1.0E-9 | 1.2E2 | 2.2E1 | 85 | 8 | 85 | 8 | 0.48 |
| qV | pg/ml | 1.8E3 | 1.8E3 | 2.5E3 | 2.2E3 | 2.0E3 | 1.6E3 | 1.0E2 | 6.3E2 | 1.1E4 | 5.6E3 | 85 | 8 | 85 | 8 | 0.47 |
| qU | pg/ml | 8.1E1 | 8.7E1 | 1.9E2 | 2.5E2 | 3.1E2 | 3.7E2 | 1.0E-9 | 1.9E1 | 2.1E3 | 1.1E3 | 85 | 8 | 85 | 8 | 0.56 |
| qT | pg/ml | 4.1E1 | 3.4E1 | 6.7E1 | 5.7E1 | 7.3E1 | 5.4E1 | 1.0E-9 | 6.9E0 | 4.9E2 | 1.6E2 | 85 | 8 | 85 | 8 | 0.46 |
| jK | ng/ml | 1.6E3 | 1.2E3 | 1.7E3 | 1.3E3 | 6.2E2 | 6.3E2 | 2.8E2 | 7.5E2 | 4.1E3 | 2.9E3 | 87 | 9 | 87 | 9 | 0.26 |
| jL | ng/ml | 2.0E2 | 2.9E2 | 2.7E2 | 3.3E2 | 2.1E2 | 1.8E2 | 5.6E1 | 1.5E2 | 9.6E2 | 6.3E2 | 87 | 9 | 87 | 9 | 0.66 |
| jM | ng/ml | 7.1E4 | 4.9E4 | 7.6E4 | 6.8E4 | 3.9E4 | 5.2E4 | 4.6E3 | 1.3E4 | 1.8E5 | 1.4E5 | 87 | 9 | 87 | 9 | 0.41 |
| jO | pg/ml | 2.2E5 | 2.6E5 | 2.8E5 | 2.7E5 | 1.7E5 | 1.6E5 | 6.0E4 | 9.8E4 | 1.1E6 | 6.5E5 | 87 | 9 | 87 | 9 | 0.50 |
| jP | pg/ml | 2.5E5 | 4.9E5 | 2.8E5 | 3.9E5 | 1.4E5 | 1.9E5 | 3.6E4 | 1.3E5 | 7.1E5 | 5.5E5 | 87 | 9 | 87 | 9 | 0.66 |
| jQ | pg/ml | 2.3E3 | 1.2E3 | 3.2E3 | 2.7E3 | 2.9E3 | 2.8E3 | 5.0E0 | 4.9E2 | 1.3E4 | 9.2E3 | 87 | 9 | 87 | 9 | 0.44 |
| jR | pg/ml | 5.7E3 | 3.3E3 | 1.0E4 | 9.5E3 | 1.2E4 | 1.4E4 | 1.0E-9 | 3.0E1 | 6.8E4 | 4.6E4 | 87 | 9 | 87 | 9 | 0.42 |
| jT | pg/ml | 1.8E5 | 1.6E5 | 1.8E5 | 1.5E5 | 7.2E4 | 5.1E4 | 7.1E4 | 7.5E4 | 5.5E5 | 2.2E5 | 87 | 9 | 87 | 9 | 0.34 |
| jU | mIU/ml | 5.5E0 | 5.8E0 | 1.2E1 | 1.4E1 | 1.9E1 | 1.8E1 | 8.1E-2 | 1.2E0 | 1.1E2 | 5.3E1 | 87 | 9 | 87 | 9 | 0.51 |
| jV | mIU/ml | 1.9E0 | 1.9E0 | 4.1E0 | 3.5E0 | 5.7E0 | 3.6E0 | 2.7E-3 | 2.1E-1 | 3.2E1 | 1.0E1 | 87 | 9 | 87 | 9 | 0.49 |
| jY | ng/ml | 9.7E-4 | 2.6E-3 | 7.7E-3 | 7.4E-3 | 3.4E-2 | 9.4E-3 | 1.0E-9 | 1.0E-9 | 3.0E-1 | 2.6E-2 | 87 | 9 | 87 | 9 | 0.60 |
| kC | pg/ml | 1.0E2 | 1.0E2 | 2.0E2 | 1.4E2 | 3.7E2 | 1.6E2 | 2.1E1 | 3.6E1 | 2.7E3 | 5.9E2 | 64 | 10 | 64 | 10 | 0.46 |
| kE | pg/ml | 1.4E5 | 1.3E5 | 1.4E5 | 1.4E5 | 3.7E4 | 5.5E4 | 3.8E4 | 7.9E4 | 2.3E5 | 2.7E5 | 64 | 10 | 64 | 10 | 0.45 |
| kF | pg/mL | 6.6E1 | 5.8E1 | 6.7E1 | 7.9E1 | 2.3E1 | 3.9E1 | 2.7E1 | 4.0E1 | 1.5E2 | 1.4E2 | 64 | 10 | 64 | 10 | 0.53 |
| kG | pg/mL | 8.3E3 | 8.9E3 | 1.1E4 | 3.3E4 | 9.5E3 | 4.9E4 | 1.3E3 | 1.1E3 | 5.8E4 | 1.6E5 | 64 | 10 | 64 | 10 | 0.55 |
| kI | pg/ml | 2.1E2 | 1.6E2 | 2.2E2 | 1.9E2 | 1.2E2 | 1.0E2 | 4.4E1 | 1.0E-9 | 6.7E2 | 3.5E2 | 64 | 10 | 64 | 10 | 0.45 |
| kK | pg/ml | 1.2E2 | 1.4E2 | 1.6E2 | 1.6E2 | 1.6E2 | 8.9E1 | 6.4E0 | 2.9E1 | 9.1E2 | 2.9E2 | 64 | 10 | 64 | 10 | 0.57 |
| kN | pg/ml | 1.2E3 | 8.1E2 | 1.6E3 | 1.4E3 | 1.7E3 | 2.0E3 | 2.1E2 | 3.8E2 | 1.0E4 | 7.0E3 | 64 | 10 | 64 | 10 | 0.34 |
| kO | pg/ml | 7.1E3 | 7.3E3 | 9.8E3 | 8.0E3 | 1.8E4 | 2.6E3 | 3.8E3 | 5.0E3 | 1.5E5 | 1.3E4 | 64 | 10 | 64 | 10 | 0.55 |
| kP | pg/ml | 6.2E3 | 4.0E3 | 7.0E3 | 5.3E3 | 4.3E3 | 4.0E3 | 9.6E2 | 1.6E3 | 2.7E4 | 1.5E4 | 64 | 10 | 64 | 10 | 0.34 |
| kQ | pg/ml | 4.2E3 | 5.3E3 | 5.2E3 | 6.7E3 | 3.9E3 | 5.2E3 | 5.6E2 | 1.4E3 | 2.5E4 | 2.2E4 | 105 | 14 | 105 | 14 | 0.60 |
| kR | pg/ml | 2.2E1 | 1.5E1 | 3.7E1 | 3.2E1 | 1.0E2 | 3.3E1 | 1.0E-9 | 2.9E0 | 1.0E3 | 1.1E2 | 105 | 14 | 105 | 14 | 0.46 |
| kS | pg/ml | 8.8E2 | 8.9E2 | 9.9E2 | 1.0E3 | 5.8E2 | 6.6E2 | 8.2E1 | 3.2E2 | 3.2E3 | 2.5E3 | 105 | 14 | 105 | 14 | 0.47 |
| rZ | ng/ml | 1.4E-3 | 8.2E-3 | 1.1E-2 | 2.4E-2 | 3.6E-2 | 3.9E-2 | 1.0E-9 | 1.0E-9 | 3.0E-1 | 1.1E-1 | 80 | 7 | 80 | 7 | 0.60 |
| rY | ng/ml | 6.1E-2 | 7.6E-2 | 6.5E-1 | 8.4E-2 | 3.0E0 | 4.8E-2 | 1.0E-9 | 2.8E-2 | 2.0E1 | 1.6E-1 | 80 | 7 | 80 | 7 | 0.59 |
| rX | ng/ml | 1.0E-9 | 1.0E-9 | 1.3E-1 | 2.7E-2 | 6.0E-1 | 4.7E-2 | 1.0E-9 | 1.0E-9 | 3.7E0 | 1.2E-1 | 80 | 7 | 80 | 7 | 0.61 |
| lK | pg/ml | 6.8E1 | 3.6E1 | 1.5E2 | 9.7E1 | 1.8E2 | 1.5E2 | 1.0E-9 | 2.3E0 | 7.4E2 | 4.8E2 | 86 | 9 | 86 | 9 | 0.38 |
| lL | pg/ml | 1.6E3 | 2.5E3 | 2.9E3 | 2.8E3 | 5.0E3 | 1.7E3 | 7.5E1 | 8.8E2 | 4.2E4 | 6.8E3 | 87 | 9 | 87 | 9 | 0.63 |
| lM | pg/ml | 1.2E3 | 3.1E3 | 3.7E3 | 1.5E4 | 6.3E3 | 2.3E4 | 9.5E0 | 2.6E2 | 4.2E4 | 6.7E4 | 87 | 9 | 87 | 9 | 0.63 |
| lN | pg/ml | 1.0E-9 | 3.0E0 | 3.3E0 | 4.2E0 | 6.9E0 | 6.2E0 | 1.0E-9 | 1.0E-9 | 4.3E1 | 1.9E1 | 87 | 9 | 87 | 9 | 0.59 |
| lO | pg/ml | 1.0E-9 | 1.0E-9 | 1.9E0 | 1.5E1 | 1.4E1 | 4.6E1 | 1.0E-9 | 1.0E-9 | 1.3E2 | 1.4E2 | 86 | 9 | 86 | 9 | 0.55 |
| nW | pg/ml | 1.1E5 | 1.1E5 | 1.1E5 | 1.1E5 | 2.8E4 | 3.2E4 | 5.8E4 | 3.6E4 | 1.9E5 | 1.5E5 | 105 | 14 | 105 | 14 | 0.50 |
| nY | pg/ml | 2.2E3 | 3.5E3 | 2.6E3 | 3.4E3 | 1.5E3 | 2.0E3 | 5.1E2 | 6.3E2 | 1.0E4 | 8.1E3 | 105 | 14 | 105 | 14 | 0.63 |
| oO | pg/ml | 8.7E4 | 8.9E4 | 9.9E4 | 1.3E5 | 6.0E4 | 1.2E5 | 1.5E4 | 3.3E3 | 3.0E5 | 4.0E5 | 58 | 9 | 58 | 9 | 0.53 |
| oP | pg/ml | 1.3E5 | 1.4E5 | 1.4E5 | 1.7E5 | 8.1E4 | 1.6E5 | 2.4E4 | 2.4E4 | 4.2E5 | 5.7E5 | 58 | 9 | 58 | 9 | 0.51 |
| oQ | pg/ml | 2.9E3 | 4.0E3 | 3.5E3 | 7.3E3 | 3.0E3 | 9.5E3 | 7.7E2 | 9.1E2 | 2.1E4 | 3.2E4 | 58 | 9 | 58 | 9 | 0.64 |
| oE | pg/ml | 2.0E2 | 7.7E2 | 4.5E2 | 9.6E2 | 6.0E2 | 9.7E2 | 1.0E-9 | 1.0E-9 | 2.8E3 | 3.4E3 | 105 | 14 | 105 | 14 | 0.67 |
| oF | pg/ml | 1.2E4 | 2.3E4 | 2.6E4 | 4.5E4 | 3.9E4 | 4.4E4 | 3.5E2 | 2.0E3 | 2.5E5 | 1.4E5 | 105 | 14 | 105 | 14 | 0.69 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| oH | pg/ml | 3.9E1 | 2.3E1 | 8.1E1 | 4.9E1 | 1.2E2 | 7.7E1 | 4.3E-1 | 1.1E1 | 8.6E2 | 3.1E2 | 105 | 14 | 105 | 14 | 0.40 |
| oK | pg/ml | 8.4E2 | 1.2E3 | 1.6E3 | 1.7E3 | 1.9E3 | 1.6E3 | 8.8E1 | 2.3E2 | 1.2E4 | 5.9E3 | 105 | 14 | 105 | 14 | 0.54 |
| oN | pg/ml | 5.5E2 | 5.4E2 | 9.9E2 | 6.4E2 | 2.0E3 | 3.4E2 | 1.1E2 | 2.8E2 | 1.8E4 | 1.5E3 | 105 | 14 | 105 | 14 | 0.52 |
| pF | pg/ml | 5.8E-1 | 5.8E-1 | 1.7E0 | 7.5E-1 | 8.5E0 | 4.1E-1 | 1.0E-9 | 1.8E-1 | 8.7E1 | 1.3E0 | 105 | 14 | 105 | 14 | 0.54 |

Figure 37.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 8.1E1 | 8.1E1 | 9.4E1 | 7.9E1 | 6.8E1 | 4.4E1 | 7.0E0 | 1.6E1 | 4.8E2 | 1.5E2 | 288 | 7 | 288 | 7 | 0.45 |
| Ad | ug/mL | 4.7E-2 | 9.1E-2 | 1.3E-1 | 8.4E-2 | 6.6E-1 | 5.6E-2 | 6.8E-4 | 7.8E-4 | 8.5E0 | 1.5E-1 | 165 | 7 | 165 | 7 | 0.60 |
| Af | ng/mL | 1.2E0 | 8.7E-1 | 1.0E1 | 3.0E0 | 4.5E1 | 4.4E0 | 1.7E-3 | 1.5E-1 | 5.3E2 | 1.2E1 | 165 | 7 | 165 | 7 | 0.42 |
| Aj | ug/mL | 1.1E0 | 4.8E-1 | 2.3E0 | 1.9E0 | 2.5E0 | 2.6E0 | 1.5E-3 | 1.3E-3 | 6.1E0 | 5.8E0 | 165 | 7 | 165 | 7 | 0.44 |
| Al | mg/mL | 8.3E-5 | 7.7E-4 | 2.6E-4 | 6.3E-4 | 4.4E-4 | 6.1E-4 | 4.3E-6 | 7.8E-6 | 1.8E-3 | 1.5E-3 | 165 | 7 | 165 | 7 | 0.60 |
| An | U/mL | 6.1E1 | 9.0E1 | 2.5E2 | 1.7E2 | 8.0E2 | 1.6E2 | 2.8E-1 | 2.2E1 | 7.8E3 | 4.6E2 | 165 | 7 | 165 | 7 | 0.61 |
| Ao | pg/mL | 9.4E1 | 1.4E2 | 5.3E2 | 1.0E3 | 3.6E3 | 1.6E3 | 1.5E0 | 4.1E0 | 3.9E4 | 4.5E3 | 165 | 7 | 165 | 7 | 0.60 |
| Ap | ng/mL | 3.2E1 | 1.4E2 | 4.7E1 | 1.3E2 | 5.2E1 | 9.7E1 | 2.0E0 | 4.4E0 | 3.3E2 | 2.4E2 | 165 | 7 | 165 | 7 | 0.74 |
| Ar | ng/mL | 6.8E-1 | 2.3E0 | 2.8E0 | 9.1E0 | 6.7E0 | 1.8E1 | 3.4E-3 | 2.5E-1 | 5.1E1 | 5.0E1 | 165 | 7 | 165 | 7 | 0.66 |
| As | ng/mL | 8.7E-3 | 1.7E-3 | 2.0E-2 | 5.5E-3 | 9.7E-2 | 6.8E-3 | 1.7E-3 | 1.7E-3 | 1.2E0 | 1.9E-2 | 165 | 7 | 165 | 7 | 0.35 |
| Aw | pg/mL | 1.6E1 | 2.2E1 | 1.7E1 | 2.2E1 | 6.2E0 | 4.9E0 | 2.9E-2 | 1.4E1 | 5.1E1 | 2.9E1 | 165 | 7 | 165 | 7 | 0.80 |
| Ax | ng/mL | 2.0E0 | 1.6E1 | 2.4E1 | 1.8E2 | 9.0E1 | 3.2E2 | 1.2E-2 | 1.0E-4 | 7.7E2 | 8.5E2 | 165 | 7 | 165 | 7 | 0.70 |
| Ba | ng/mL | 8.3E1 | 1.2E3 | 5.9E2 | 3.5E3 | 1.5E3 | 5.3E3 | 1.1E0 | 4.1E0 | 8.1E3 | 1.5E4 | 165 | 7 | 165 | 7 | 0.79 |
| Bb | ng/mL | 4.0E0 | 1.9E1 | 6.9E0 | 1.8E1 | 8.3E0 | 1.6E1 | 4.1E-3 | 3.5E-1 | 4.9E1 | 4.8E1 | 165 | 7 | 165 | 7 | 0.74 |
| Bc | ng/mL | 3.5E1 | 1.6E2 | 1.2E2 | 3.6E2 | 2.3E2 | 3.9E2 | 4.9E-1 | 2.9E0 | 1.2E3 | 9.9E2 | 165 | 7 | 165 | 7 | 0.73 |
| Bg | ng/mL | 1.1E-1 | 5.9E-1 | 6.5E0 | 1.5E0 | 3.5E1 | 1.7E0 | 5.3E-4 | 3.1E-2 | 4.0E2 | 4.1E0 | 165 | 7 | 165 | 7 | 0.61 |
| Bn | ng/mL | 5.6E-2 | 2.1E-1 | 1.5E0 | 3.5E-1 | 4.9E0 | 3.8E-1 | 5.6E-2 | 5.6E-2 | 5.8E1 | 1.1E0 | 165 | 7 | 165 | 7 | 0.50 |
| Bo | ng/mL | 1.3E1 | 1.7E1 | 1.5E1 | 1.8E1 | 1.1E1 | 5.9E0 | 1.6E-2 | 9.7E0 | 5.0E1 | 2.6E1 | 165 | 7 | 165 | 7 | 0.64 |
| Ch | uIU/mL | 1.0E0 | 1.1E0 | 3.6E1 | 4.6E0 | 1.8E2 | 9.1E0 | 3.4E-3 | 5.9E-1 | 1.8E3 | 2.5E1 | 165 | 7 | 165 | 7 | 0.52 |
| Co | pg/mL | 4.7E1 | 6.1E1 | 2.2E2 | 4.2E2 | 1.3E3 | 7.5E2 | 1.5E-1 | 8.8E0 | 1.7E4 | 2.1E3 | 165 | 7 | 165 | 7 | 0.61 |
| Cp | ng/mL | 2.2E1 | 4.0E1 | 3.5E1 | 5.5E1 | 1.0E2 | 4.3E1 | 6.0E-1 | 1.1E1 | 1.3E3 | 1.4E2 | 165 | 7 | 165 | 7 | 0.72 |
| Cq | ng/mL | 3.0E-2 | 8.4E-2 | 4.0E-1 | 5.2E-1 | 3.8E0 | 8.6E-1 | 8.0E-4 | 8.0E-4 | 4.9E1 | 2.3E0 | 165 | 7 | 165 | 7 | 0.69 |
| Cs | ng/mL | 6.0E1 | 2.1E2 | 4.7E2 | 9.9E2 | 1.7E3 | 1.9E3 | 8.3E-1 | 5.7E0 | 1.8E4 | 5.1E3 | 165 | 7 | 165 | 7 | 0.64 |
| Ct | ng/mL | 3.3E-1 | 1.4E1 | 4.0E1 | 7.3E1 | 1.2E2 | 1.5E2 | 1.1E-4 | 1.1E-4 | 6.2E2 | 4.2E2 | 165 | 7 | 165 | 7 | 0.61 |
| Cu | ng/mL | 2.6E-1 | 2.9E0 | 8.7E-1 | 5.1E0 | 5.2E0 | 7.2E0 | 1.9E-2 | 1.7E-2 | 6.6E1 | 2.1E1 | 165 | 7 | 165 | 7 | 0.83 |
| Cv | ng/mL | 5.2E0 | 1.2E1 | 2.6E1 | 4.1E1 | 6.4E1 | 5.2E1 | 2.0E-2 | 1.0E-1 | 5.3E2 | 1.4E2 | 165 | 7 | 165 | 7 | 0.59 |
| Cw | mIU/mL | 3.6E-2 | 9.1E-2 | 8.3E-2 | 9.0E-2 | 5.2E-1 | 4.5E-2 | 8.9E-4 | 1.1E-2 | 6.8E0 | 1.5E-1 | 165 | 7 | 165 | 7 | 0.80 |
| Cx | ng/mL | 7.7E-1 | 7.4E-3 | 5.1E1 | 4.6E-1 | 9.9E1 | 6.6E-1 | 9.3E-5 | 9.3E-5 | 3.9E2 | 1.7E0 | 165 | 7 | 165 | 7 | 0.32 |
| Db | ug/mL | 7.5E0 | 6.7E0 | 8.8E0 | 7.9E0 | 7.7E0 | 3.6E0 | 4.5E-1 | 4.3E0 | 5.9E1 | 1.5E1 | 165 | 7 | 165 | 7 | 0.49 |
| Dc | nmol/L | 2.0E-2 | 1.9E-1 | 1.4E-1 | 3.1E-1 | 1.1E0 | 4.6E-1 | 5.2E-6 | 2.1E-3 | 1.4E1 | 1.3E0 | 165 | 7 | 165 | 7 | 0.78 |
| Dd | ug/mL | 7.1E-2 | 6.0E-1 | 1.9E-1 | 5.4E-1 | 3.6E-1 | 5.1E-1 | 4.8E-4 | 6.4E-3 | 3.6E0 | 1.5E0 | 165 | 7 | 165 | 7 | 0.69 |
| De | ng/mL | 3.4E-3 | 2.1E-1 | 7.6E-2 | 3.0E-1 | 1.4E-1 | 4.0E-1 | 3.4E-3 | 3.4E-3 | 9.2E-1 | 1.1E0 | 165 | 7 | 165 | 7 | 0.69 |
| Dg | ng/mL | 3.6E1 | 8.7E1 | 4.6E1 | 7.0E1 | 4.0E1 | 4.8E1 | 7.1E-1 | 1.9E0 | 1.9E2 | 1.2E2 | 165 | 7 | 165 | 7 | 0.65 |
| Di | pg/mL | 2.0E0 | 3.3E0 | 2.4E0 | 3.9E0 | 2.2E0 | 1.2E0 | 1.8E-1 | 2.7E0 | 1.3E1 | 5.4E0 | 165 | 7 | 165 | 7 | 0.76 |
| Dk | uIU/mL | 1.4E-2 | 2.8E-2 | 5.4E-2 | 1.7E-1 | 1.6E-1 | 3.6E-1 | 1.1E-4 | 6.6E-3 | 1.6E0 | 9.8E-1 | 165 | 7 | 165 | 7 | 0.58 |
| Dl | ng/mL | 2.0E2 | 4.7E2 | 2.9E2 | 4.7E2 | 2.8E2 | 3.9E2 | 5.5E0 | 4.4E0 | 1.6E3 | 1.1E3 | 165 | 7 | 165 | 7 | 0.64 |
| Po | pg/ml | 3.0E-1 | 2.8E1 | 9.6E0 | 4.3E1 | 2.9E1 | 6.8E1 | 8.0E-3 | 2.6E-2 | 2.7E2 | 2.2E2 | 339 | 9 | 339 | 9 | 0.81 |
| Et | ng/ml | 1.4E3 | 3.0E3 | 1.7E3 | 3.0E3 | 1.2E3 | 1.6E3 | 7.5E1 | 5.9E2 | 4.8E3 | 5.0E3 | 338 | 9 | 338 | 9 | 0.75 |
| Fp | ng/ml | 1.3E1 | 3.8E1 | 2.4E1 | 3.8E1 | 2.7E1 | 3.9E1 | 6.0E-3 | 1.3E0 | 1.3E2 | 1.2E2 | 341 | 9 | 341 | 9 | 0.60 |
| Fr | ng/ml | 3.5E4 | 5.5E5 | 1.2E5 | 4.0E5 | 1.8E5 | 3.2E5 | 1.9E2 | 7.0E3 | 8.4E5 | 8.4E5 | 346 | 11 | 346 | 11 | 0.78 |
| Nm | pg/ml | 1.2E4 | 1.2E5 | 3.4E4 | 1.5E5 | 8.9E4 | 1.7E5 | 1.0E-9 | 1.0E-9 | 9.6E5 | 4.4E5 | 342 | 9 | 342 | 9 | 0.73 |
| Nn | pg/ml | 1.5E2 | 6.8E2 | 1.7E3 | 4.1E4 | 8.0E3 | 1.0E5 | 1.0E-9 | 1.0E-9 | 9.5E4 | 3.1E5 | 342 | 9 | 342 | 9 | 0.76 |
| No | pg/ml | 1.5E1 | 8.3E1 | 3.6E1 | 1.5E2 | 8.1E1 | 2.2E2 | 1.0E-9 | 1.6E0 | 9.1E2 | 7.0E2 | 342 | 9 | 342 | 9 | 0.73 |
| Nq | pg/ml | 1.9E0 | 1.9E1 | 2.1E1 | 1.2E2 | 7.4E1 | 2.0E2 | 1.0E-9 | 1.0E-9 | 6.7E2 | 5.9E2 | 342 | 9 | 342 | 9 | 0.69 |
| Nr | pg/ml | 1.5E0 | 1.2E1 | 2.3E1 | 2.1E2 | 8.3E1 | 4.4E2 | 1.0E-9 | 1.0E-9 | 9.8E2 | 1.4E3 | 342 | 9 | 342 | 9 | 0.69 |
| Ns | pg/ml | 1.0E-9 | 1.0E-9 | 7.8E0 | 4.0E-1 | 6.5E1 | 1.2E0 | 1.0E-9 | 1.0E-9 | 1.1E3 | 3.6E0 | 342 | 9 | 342 | 9 | 0.52 |
| Nt | pg/ml | 1.0E2 | 2.4E2 | 1.4E2 | 3.7E2 | 1.3E2 | 3.7E2 | 9.8E-1 | 8.0E1 | 1.7E3 | 1.2E3 | 342 | 9 | 342 | 9 | 0.77 |
| Nu | pg/ml | 1.7E1 | 1.0E2 | 5.6E1 | 1.3E2 | 9.3E1 | 8.6E1 | 1.0E-9 | 7.8E0 | 6.3E2 | 2.9E2 | 342 | 9 | 342 | 9 | 0.79 |
| Lu | pg/ml | 1.0E4 | 1.0E4 | 1.5E4 | 1.0E4 | 4.0E4 | 7.2E3 | 5.2E2 | 3.0E3 | 5.6E5 | 2.7E4 | 342 | 9 | 342 | 9 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Lv | pg/ml | 1.0E-9 | 4.3E1 | 1.5E1 | 5.2E1 | 3.2E1 | 3.7E1 | 1.0E-9 | 7.3E0 | 2.6E2 | 1.1E2 | 342 | 9 | 342 | 9 | 0.85 |
| Lw | pg/ml | 1.0E-9 | 1.0E-9 | 4.7E-1 | 2.4E1 | 5.1E0 | 5.9E1 | 1.0E-9 | 1.0E-9 | 8.0E1 | 1.8E2 | 342 | 9 | 342 | 9 | 0.66 |
| Lx | pg/ml | 1.0E-9 | 4.8E2 | 2.4E2 | 9.0E2 | 1.3E3 | 9.4E2 | 1.0E-9 | 1.0E-9 | 2.2E4 | 2.8E3 | 342 | 9 | 342 | 9 | 0.84 |
| Ly | pg/ml | 1.0E-9 | 1.0E-9 | 9.2E0 | 5.2E0 | 1.8E1 | 8.5E0 | 1.0E-9 | 1.0E-9 | 1.1E2 | 2.4E1 | 342 | 9 | 342 | 9 | 0.48 |
| Lz | pg/ml | 1.0E-9 | 1.0E-9 | 3.5E0 | 5.6E0 | 2.6E1 | 1.6E1 | 1.0E-9 | 1.0E-9 | 4.3E2 | 4.7E1 | 342 | 9 | 342 | 9 | 0.57 |
| Ma | pg/ml | 3.9E2 | 3.4E3 | 2.2E3 | 9.2E3 | 6.0E3 | 1.3E4 | 1.0E-9 | 2.4E1 | 6.5E4 | 3.6E4 | 342 | 9 | 342 | 9 | 0.70 |
| Mb | pg/ml | 2.5E1 | 2.7E1 | 3.2E1 | 3.2E1 | 1.8E1 | 1.5E1 | 4.1E0 | 1.6E1 | 2.1E2 | 5.3E1 | 342 | 9 | 342 | 9 | 0.50 |
| Mc | pg/ml | 1.0E-9 | 1.0E-9 | 5.6E-2 | 1.0E-9 | 7.4E-1 | 0.0E0 | 1.0E-9 | 1.0E-9 | 1.3E1 | 1.0E-9 | 342 | 9 | 342 | 9 | 0.49 |
| Md | pg/ml | 1.0E-9 | 1.0E-9 | 6.6E-1 | 5.1E-1 | 5.5E0 | 1.3E0 | 1.0E-9 | 1.0E-9 | 6.8E1 | 4.0E0 | 342 | 9 | 342 | 9 | 0.57 |
| Me | pg/ml | 3.1E1 | 2.7E1 | 3.1E1 | 2.4E1 | 2.4E1 | 1.6E1 | 1.0E-9 | 1.0E-9 | 3.2E2 | 5.0E1 | 342 | 9 | 342 | 9 | 0.39 |
| Mf | pg/ml | 1.0E-9 | 1.0E-9 | 6.2E-1 | 1.4E-1 | 3.9E0 | 2.8E-1 | 1.0E-9 | 1.0E-9 | 5.6E1 | 6.8E-1 | 342 | 9 | 342 | 9 | 0.53 |
| Mg | pg/ml | 9.4E-1 | 2.0E0 | 6.2E0 | 3.2E1 | 1.2E1 | 5.4E1 | 1.0E-9 | 1.0E-9 | 9.2E1 | 1.5E2 | 342 | 9 | 342 | 9 | 0.58 |
| Mh | pg/ml | 1.0E-9 | 1.0E-9 | 1.2E0 | 2.4E0 | 7.6E0 | 6.0E0 | 1.0E-9 | 1.0E-9 | 1.1E2 | 1.8E1 | 342 | 9 | 342 | 9 | 0.59 |
| Mi | pg/ml | 1.0E-9 | 9.3E0 | 1.9E0 | 7.9E1 | 1.9E1 | 1.7E2 | 1.0E-9 | 1.0E-9 | 3.2E2 | 5.2E2 | 342 | 9 | 342 | 9 | 0.82 |
| Mj | pg/ml | 1.0E-9 | 2.0E0 | 6.3E0 | 4.5E1 | 3.2E1 | 7.3E1 | 1.0E-9 | 1.0E-9 | 4.6E2 | 2.1E2 | 342 | 9 | 342 | 9 | 0.70 |
| Mk | pg/ml | 1.8E0 | 5.6E0 | 1.5E1 | 6.4E1 | 9.2E1 | 1.7E2 | 1.0E-9 | 1.0E-9 | 1.3E3 | 5.0E2 | 342 | 9 | 342 | 9 | 0.65 |
| Ml | pg/ml | 1.0E-9 | 1.0E-9 | 1.1E1 | 2.6E-1 | 1.2E2 | 7.7E-1 | 1.0E-9 | 1.0E-9 | 2.1E3 | 2.3E0 | 342 | 9 | 342 | 9 | 0.44 |
| Mm | pg/ml | 5.5E2 | 2.2E3 | 1.0E3 | 3.2E3 | 1.3E3 | 3.3E3 | 1.0E-9 | 1.0E-9 | 1.0E4 | 1.0E4 | 342 | 9 | 342 | 9 | 0.68 |
| Mn | pg/ml | 5.9E0 | 1.2E1 | 1.1E1 | 1.4E1 | 2.5E1 | 1.1E1 | 1.0E-9 | 1.1E0 | 3.5E2 | 3.7E1 | 342 | 9 | 342 | 9 | 0.67 |
| Mp | pg/ml | 1.0E-9 | 2.7E1 | 1.3E1 | 3.3E2 | 5.0E1 | 7.7E2 | 1.0E-9 | 1.0E-9 | 6.3E2 | 2.4E3 | 342 | 9 | 342 | 9 | 0.79 |
| Mq | pg/ml | 1.0E-9 | 1.0E-9 | 2.4E0 | 1.8E1 | 1.4E1 | 2.9E1 | 1.0E-9 | 1.0E-9 | 1.8E2 | 7.4E1 | 342 | 9 | 342 | 9 | 0.64 |
| Mr | pg/ml | 1.0E-9 | 1.1E1 | 3.2E1 | 4.8E2 | 1.8E2 | 1.1E3 | 1.0E-9 | 1.0E-9 | 2.2E3 | 3.4E3 | 342 | 9 | 342 | 9 | 0.66 |
| Ms | pg/ml | 3.2E2 | 2.6E2 | 4.6E2 | 7.6E2 | 5.3E2 | 1.5E3 | 1.0E-9 | 1.0E-9 | 3.3E3 | 4.7E3 | 342 | 9 | 342 | 9 | 0.44 |
| Mt | pg/ml | 2.7E-1 | 1.2E1 | 1.8E1 | 6.7E1 | 1.8E2 | 9.9E1 | 1.0E-9 | 1.0E-9 | 3.2E3 | 3.1E2 | 342 | 9 | 342 | 9 | 0.76 |
| Mu | pg/ml | 1.0E-9 | 1.0E-9 | 1.4E0 | 6.4E0 | 1.4E1 | 1.6E1 | 1.0E-9 | 1.0E-9 | 2.3E2 | 4.8E1 | 342 | 9 | 342 | 9 | 0.61 |
| Mv | pg/ml | 1.0E-9 | 2.5E0 | 6.7E1 | 1.9E2 | 3.4E2 | 3.5E2 | 1.0E-9 | 1.0E-9 | 4.5E3 | 9.0E2 | 342 | 9 | 342 | 9 | 0.67 |
| Mw | pg/ml | 3.7E1 | 6.6E2 | 2.7E2 | 1.2E3 | 1.4E3 | 1.7E3 | 1.0E-9 | 1.0E-9 | 1.8E4 | 5.2E3 | 342 | 9 | 342 | 9 | 0.76 |
| Mx | pg/ml | 1.0E-9 | 1.0E-9 | 4.1E-1 | 1.2E0 | 2.2E0 | 2.2E0 | 1.0E-9 | 1.0E-9 | 3.2E1 | 6.6E0 | 342 | 9 | 342 | 9 | 0.65 |
| My | pg/ml | 1.0E-9 | 1.1E2 | 3.2E2 | 4.7E2 | 2.4E3 | 7.8E2 | 1.0E-9 | 1.0E-9 | 3.9E4 | 1.9E3 | 342 | 9 | 342 | 9 | 0.69 |
| Mz | pg/ml | 1.1E1 | 1.1E2 | 3.5E1 | 9.6E1 | 1.3E2 | 7.4E1 | 1.0E-9 | 3.5E0 | 1.9E3 | 2.0E2 | 342 | 9 | 342 | 9 | 0.80 |
| Na | pg/ml | 1.0E-9 | 1.0E-9 | 7.6E-1 | 2.0E0 | 2.9E0 | 2.9E0 | 1.0E-9 | 1.0E-9 | 4.2E1 | 6.4E0 | 342 | 9 | 342 | 9 | 0.63 |
| Nb | pg/ml | 2.2E0 | 8.1E0 | 3.9E0 | 3.5E1 | 1.2E1 | 6.7E1 | 1.0E-9 | 5.4E-2 | 1.5E2 | 2.1E2 | 342 | 9 | 342 | 9 | 0.74 |
| Nc | pg/ml | 3.1E2 | 1.3E1 | 5.1E2 | 3.8E2 | 7.4E2 | 5.1E2 | 1.0E-9 | 1.0E-9 | 9.2E3 | 1.4E3 | 342 | 9 | 342 | 9 | 0.43 |
| Nd | pg/ml | 2.7E1 | 3.9E1 | 3.3E1 | 4.9E1 | 1.3E2 | 4.9E1 | 1.0E-9 | 7.2E-1 | 2.1E3 | 1.5E2 | 342 | 9 | 342 | 9 | 0.66 |
| Ne | pg/ml | 4.2E2 | 3.2E2 | 5.1E2 | 6.3E2 | 5.4E2 | 1.1E3 | 1.0E-9 | 1.0E-9 | 7.0E3 | 3.6E3 | 342 | 9 | 342 | 9 | 0.41 |
| Nf | pg/ml | 1.0E-9 | 1.0E-9 | 3.1E0 | 9.4E-1 | 1.3E1 | 1.9E0 | 1.0E-9 | 1.0E-9 | 1.3E2 | 4.8E0 | 342 | 9 | 342 | 9 | 0.50 |
| Ng | pg/ml | 1.2E1 | 9.6E0 | 9.0E1 | 7.1E1 | 1.8E2 | 9.5E1 | 1.0E-9 | 1.0E-9 | 1.6E3 | 2.6E2 | 342 | 9 | 342 | 9 | 0.52 |
| Nh | pg/ml | 6.2E1 | 2.9E1 | 7.9E1 | 8.5E1 | 7.2E1 | 1.6E2 | 1.0E-9 | 4.5E0 | 5.6E2 | 5.1E2 | 342 | 9 | 342 | 9 | 0.34 |
| Ni | pg/ml | 1.0E-9 | 1.0E-9 | 8.5E1 | 5.1E1 | 1.4E2 | 1.2E2 | 1.0E-9 | 1.0E-9 | 1.1E3 | 3.5E2 | 342 | 9 | 342 | 9 | 0.38 |
| Nj | pg/ml | 7.2E0 | 4.4E0 | 1.1E1 | 6.2E0 | 1.2E1 | 6.5E0 | 1.0E-9 | 1.0E-9 | 6.9E1 | 1.7E1 | 342 | 9 | 342 | 9 | 0.37 |
| Nk | pg/ml | 1.8E1 | 1.0E-9 | 3.2E1 | 2.0E1 | 3.9E1 | 3.2E1 | 1.0E-9 | 1.0E-9 | 2.0E2 | 8.3E1 | 342 | 9 | 342 | 9 | 0.36 |
| Nl | pg/ml | 4.2E1 | 9.3E-1 | 5.6E1 | 3.9E1 | 7.5E1 | 6.0E1 | 1.0E-9 | 1.0E-9 | 1.1E3 | 1.8E2 | 342 | 9 | 342 | 9 | 0.34 |
| Hq | pg/ml | 1.1E0 | 9.1E0 | 1.6E2 | 2.7E1 | 1.9E3 | 5.8E1 | 1.0E-9 | 1.0E-9 | 2.8E4 | 1.8E2 | 340 | 9 | 340 | 9 | 0.66 |
| Hr | pg/ml | 9.1E1 | 2.7E2 | 6.6E2 | 6.0E2 | 1.4E3 | 1.1E3 | 1.0E-9 | 1.6E1 | 1.2E4 | 3.4E3 | 340 | 9 | 340 | 9 | 0.56 |
| Hu | pg/ml | 1.1E1 | 9.5E2 | 4.6E3 | 1.3E3 | 4.0E4 | 1.9E3 | 1.0E-9 | 1.0E-9 | 6.3E5 | 5.9E3 | 340 | 9 | 340 | 9 | 0.72 |
| Hv | pg/ml | 1.4E0 | 3.0E0 | 5.2E0 | 2.4E1 | 4.9E1 | 5.3E1 | 1.0E-9 | 3.8E-1 | 8.9E2 | 1.6E2 | 340 | 9 | 340 | 9 | 0.66 |
| Hw | pg/ml | 6.4E0 | 5.6E1 | 4.3E1 | 9.3E1 | 5.1E2 | 1.6E2 | 1.0E-9 | 4.6E-1 | 9.4E3 | 5.0E2 | 340 | 9 | 340 | 9 | 0.69 |
| Hx | pg/ml | 8.8E0 | 2.4E1 | 5.5E1 | 1.9E2 | 5.0E2 | 4.1E2 | 1.0E-9 | 8.8E0 | 9.3E3 | 1.3E3 | 340 | 9 | 340 | 9 | 0.78 |
| Ih | ng/ml | 6.3E1 | 3.9E2 | 2.5E2 | 7.2E2 | 4.6E2 | 7.3E2 | 1.0E-9 | 2.4E0 | 3.6E3 | 1.9E3 | 341 | 9 | 341 | 9 | 0.68 |
| Ii | ng/ml | 8.1E1 | 4.5E2 | 2.1E2 | 8.3E2 | 5.1E2 | 1.4E3 | 7.3E-1 | 7.5E-1 | 5.2E3 | 4.5E3 | 341 | 9 | 341 | 9 | 0.65 |
| Ij | ng/ml | 7.8E1 | 3.1E2 | 2.5E2 | 6.1E2 | 1.4E3 | 7.2E2 | 2.8E0 | 9.5E0 | 2.4E4 | 1.9E3 | 337 | 9 | 337 | 9 | 0.77 |
| Ik | ng/ml | 1.1E1 | 3.7E1 | 1.3E3 | 3.3E2 | 1.2E4 | 4.8E2 | 5.9E-1 | 6.2E-1 | 1.2E5 | 1.5E3 | 337 | 9 | 337 | 9 | 0.62 |
| Il | ng/ml | 3.6E2 | 1.3E3 | 1.3E3 | 4.1E3 | 2.9E3 | 5.2E3 | 1.0E-9 | 1.9E-1 | 1.2E4 | 1.2E4 | 335 | 9 | 335 | 9 | 0.64 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Im | ng/ml | 2.1E2 | 7.0E2 | 4.5E2 | 2.7E3 | 7.8E2 | 4.8E3 | 1.4E1 | 2.2E1 | 6.2E3 | 1.5E4 | 337 | 9 | 337 | 9 | 0.79 |
| In | ng/ml | 3.4E0 | 8.8E0 | 3.3E1 | 1.1E2 | 2.6E2 | 2.1E2 | 1.0E-9 | 5.4E-1 | 4.5E3 | 5.9E2 | 341 | 9 | 341 | 9 | 0.61 |
| Io | ng/ml | 9.4E3 | 2.0E4 | 1.9E4 | 2.7E4 | 4.6E4 | 3.1E4 | 1.0E-9 | 1.0E3 | 7.1E5 | 1.0E5 | 341 | 9 | 341 | 9 | 0.61 |
| Ip | ng/ml | 1.0E1 | 3.0E1 | 2.1E1 | 3.6E1 | 2.6E1 | 2.1E1 | 1.0E-9 | 9.8E0 | 2.3E2 | 7.1E1 | 341 | 9 | 341 | 9 | 0.73 |
| Iq | ug/ml | 1.0E-1 | 3.0E0 | 4.1E1 | 6.3E0 | 7.4E2 | 7.9E0 | 1.0E-9 | 1.0E-9 | 1.4E4 | 2.1E1 | 341 | 9 | 341 | 9 | 0.75 |
| Ir | ug/ml | 3.7E-1 | 5.0E0 | 5.6E0 | 3.5E1 | 3.8E1 | 5.1E1 | 1.0E-9 | 3.5E-2 | 5.1E2 | 1.3E2 | 340 | 9 | 340 | 9 | 0.77 |
| Is | ng/ml | 2.0E0 | 4.2E1 | 9.4E0 | 7.0E1 | 3.4E1 | 8.5E1 | 1.0E-9 | 2.2E-1 | 5.5E2 | 2.6E2 | 341 | 9 | 341 | 9 | 0.77 |
| It | ng/ml | 2.1E0 | 5.4E0 | 2.3E1 | 6.7E1 | 1.0E2 | 1.8E2 | 1.0E-9 | 1.0E-9 | 1.1E3 | 5.5E2 | 341 | 9 | 341 | 9 | 0.66 |
| Iu | ng/ml | 1.6E2 | 2.4E3 | 1.3E3 | 8.3E3 | 4.1E3 | 1.0E4 | 1.0E-9 | 1.0E-9 | 2.9E4 | 2.4E4 | 341 | 9 | 341 | 9 | 0.72 |
| Iv | ng/ml | 1.3E1 | 1.3E2 | 9.8E1 | 9.1E2 | 8.8E2 | 1.7E3 | 1.0E-9 | 5.0E0 | 1.6E4 | 3.8E3 | 340 | 9 | 340 | 9 | 0.74 |
| Pz | ng/ml | 3.5E3 | 1.0E4 | 5.6E3 | 7.9E3 | 6.0E3 | 4.1E3 | 1.6E1 | 4.0E1 | 7.0E4 | 1.3E4 | 337 | 9 | 337 | 9 | 0.67 |
| Qa | ng/ml | 3.6E3 | 2.3E4 | 7.5E3 | 2.1E4 | 1.4E4 | 1.2E4 | 1.5E2 | 9.4E2 | 2.2E5 | 3.2E4 | 337 | 9 | 337 | 9 | 0.80 |
| Qb | ng/ml | 1.1E2 | 3.9E2 | 2.4E2 | 4.6E2 | 4.4E2 | 4.7E2 | 7.9E-1 | 3.2E1 | 5.3E3 | 1.6E3 | 337 | 9 | 337 | 9 | 0.69 |
| Qc | ng/ml | 2.0E2 | 7.5E2 | 4.5E2 | 7.2E2 | 6.0E2 | 5.2E2 | 1.0E-9 | 3.2E1 | 4.3E3 | 1.4E3 | 337 | 9 | 337 | 9 | 0.67 |
| Qd | ng/ml | 8.8E3 | 9.4E4 | 2.4E4 | 1.4E5 | 1.1E5 | 1.3E5 | 1.5E2 | 1.9E3 | 2.0E6 | 4.3E5 | 337 | 9 | 337 | 9 | 0.84 |
| Qe | ng/ml | 9.1E2 | 6.0E3 | 2.0E3 | 6.4E3 | 5.6E3 | 6.1E3 | 1.0E-9 | 5.7E1 | 9.7E4 | 1.8E4 | 337 | 9 | 337 | 9 | 0.71 |
| Jg | ng/ml | 4.6E2 | 1.7E3 | 7.8E2 | 1.7E3 | 9.5E2 | 1.2E3 | 5.8E0 | 8.4E1 | 1.0E4 | 3.9E3 | 340 | 9 | 340 | 9 | 0.75 |
| Jh | ng/ml | 2.9E0 | 3.0E1 | 2.1E1 | 6.2E1 | 8.6E1 | 9.3E1 | 1.0E-9 | 1.0E-9 | 1.2E3 | 2.9E2 | 340 | 9 | 340 | 9 | 0.76 |
| Ji | ng/ml | 5.7E1 | 3.5E2 | 8.9E1 | 3.3E2 | 1.1E2 | 2.6E2 | 1.1E0 | 2.3E1 | 1.3E3 | 6.9E2 | 340 | 9 | 340 | 9 | 0.74 |
| Jj | ng/ml | 5.3E2 | 1.7E2 | 1.9E3 | 3.7E2 | 1.8E4 | 4.0E2 | 2.3E0 | 8.7E0 | 3.4E5 | 1.0E3 | 340 | 9 | 340 | 9 | 0.32 |
| Jk | ng/ml | 2.6E0 | 4.9E1 | 2.0E1 | 7.1E1 | 4.8E1 | 8.6E1 | 1.0E-9 | 1.2E-1 | 3.9E2 | 2.4E2 | 340 | 9 | 340 | 9 | 0.74 |
| Jl | ng/ml | 4.8E-1 | 1.2E0 | 2.1E0 | 1.1E3 | 4.9E0 | 3.3E3 | 1.2E-3 | 2.5E-1 | 4.0E1 | 9.9E3 | 340 | 9 | 340 | 9 | 0.76 |
| Jm | ng/ml | 1.9E1 | 6.8E1 | 6.8E1 | 8.3E1 | 1.7E2 | 8.8E1 | 1.0E-9 | 4.0E-1 | 2.1E3 | 2.5E2 | 340 | 9 | 340 | 9 | 0.61 |
| Jn | pg/ml | 3.5E-1 | 1.9E0 | 7.3E0 | 2.9E1 | 6.2E1 | 7.8E1 | 1.0E-9 | 1.0E-9 | 7.3E2 | 2.4E2 | 340 | 9 | 340 | 9 | 0.75 |
| Jo | pg/ml | 3.8E3 | 6.3E3 | 5.1E3 | 1.0E4 | 6.6E3 | 1.2E4 | 2.0E1 | 2.4E1 | 1.0E5 | 3.6E4 | 340 | 9 | 340 | 9 | 0.62 |
| Jp | pg/ml | 7.1E4 | 1.1E5 | 7.4E4 | 1.2E5 | 4.0E4 | 3.1E4 | 5.8E2 | 6.8E4 | 3.8E5 | 1.6E5 | 340 | 9 | 340 | 9 | 0.82 |
| Jq | pg/ml | 9.6E1 | 4.0E2 | 1.9E2 | 5.3E2 | 5.2E2 | 5.4E2 | 1.0E0 | 1.3E1 | 8.7E3 | 1.7E3 | 340 | 9 | 340 | 9 | 0.75 |
| Jr | pg/ml | 4.7E0 | 3.4E1 | 8.9E1 | 3.2E2 | 7.6E2 | 8.0E2 | 1.0E-9 | 1.0E-9 | 1.1E4 | 2.4E3 | 340 | 9 | 340 | 9 | 0.80 |
| Js | pg/ml | 1.5E1 | 2.7E1 | 8.3E1 | 4.0E2 | 6.0E2 | 9.7E2 | 1.0E-9 | 2.7E0 | 1.0E4 | 3.0E3 | 340 | 9 | 340 | 9 | 0.69 |
| Jt | pg/ml | 2.4E3 | 5.9E3 | 3.1E3 | 1.3E4 | 3.5E3 | 1.4E4 | 2.2E1 | 1.5E2 | 5.2E4 | 4.1E4 | 340 | 9 | 340 | 9 | 0.75 |
| Lh | pg/ml | 1.3E4 | 3.7E4 | 2.4E4 | 8.9E4 | 4.2E4 | 1.3E5 | 1.0E-9 | 2.0E3 | 4.8E5 | 4.1E5 | 341 | 9 | 341 | 9 | 0.70 |
| Li | pg/ml | 3.5E3 | 3.8E4 | 2.0E4 | 1.0E5 | 9.1E4 | 1.4E5 | 1.2E1 | 8.4E1 | 1.3E6 | 4.1E5 | 341 | 9 | 341 | 9 | 0.78 |
| Lj | pg/ml | 2.9E3 | 1.5E4 | 2.1E4 | 2.6E4 | 5.9E4 | 4.1E4 | 1.0E-9 | 8.9E1 | 4.3E5 | 1.3E5 | 341 | 9 | 341 | 9 | 0.61 |
| Nv | pg/ml | 3.9E3 | 1.6E4 | 9.6E3 | 3.6E4 | 1.9E4 | 4.2E4 | 1.0E-9 | 1.6E2 | 1.6E5 | 1.2E5 | 342 | 9 | 342 | 9 | 0.79 |
| Nw | pg/ml | 9.6E3 | 2.9E4 | 1.4E4 | 3.8E4 | 1.9E4 | 2.6E4 | 1.9E2 | 4.5E3 | 2.1E5 | 7.8E4 | 342 | 9 | 342 | 9 | 0.81 |
| Nx | pg/ml | 2.2E2 | 8.1E2 | 4.3E2 | 9.2E2 | 6.3E2 | 8.8E2 | 1.0E-9 | 1.0E-9 | 4.1E3 | 2.3E3 | 342 | 9 | 342 | 9 | 0.66 |
| Ny | pg/ml | 6.5E0 | 8.0E1 | 1.1E2 | 1.7E2 | 1.3E3 | 2.1E2 | 1.0E-9 | 1.0E-9 | 2.5E4 | 6.1E2 | 342 | 9 | 342 | 9 | 0.82 |
| Oe | pg/ml | 3.1E1 | 1.0E-9 | 2.5E2 | 1.7E2 | 3.8E2 | 3.8E2 | 1.0E-9 | 1.0E-9 | 2.3E3 | 1.1E3 | 340 | 9 | 340 | 9 | 0.37 |
| Of | pg/ml | 1.3E2 | 2.1E2 | 5.1E3 | 3.1E3 | 2.1E4 | 8.0E3 | 1.0E-9 | 1.0E-9 | 1.9E5 | 2.4E4 | 342 | 9 | 342 | 9 | 0.55 |
| Og | pg/ml | 6.5E-2 | 6.6E-2 | 3.6E-1 | 8.3E-2 | 1.5E0 | 9.9E-2 | 1.0E-9 | 1.0E-9 | 1.9E1 | 3.2E-1 | 342 | 9 | 342 | 9 | 0.45 |
| Oh | pg/ml | 2.5E0 | 1.3E1 | 1.8E1 | 1.8E3 | 1.1E2 | 5.3E3 | 1.0E-9 | 1.0E-9 | 1.4E3 | 1.6E4 | 342 | 9 | 342 | 9 | 0.64 |
| Oi | pg/ml | 1.9E0 | 1.0E-9 | 5.0E0 | 3.6E0 | 7.9E0 | 5.5E0 | 1.0E-9 | 1.0E-9 | 6.7E1 | 1.6E1 | 342 | 9 | 342 | 9 | 0.43 |
| Ok | pg/ml | 3.9E2 | 2.2E3 | 5.5E2 | 2.4E3 | 6.5E2 | 2.3E3 | 1.5E1 | 5.3E1 | 7.8E3 | 7.0E3 | 342 | 9 | 342 | 9 | 0.75 |
| Om | pg/ml | 4.2E2 | 1.3E3 | 9.4E2 | 2.0E3 | 3.4E3 | 1.9E3 | 1.0E-9 | 7.0E1 | 5.1E4 | 5.6E3 | 342 | 9 | 342 | 9 | 0.75 |
| On | pg/ml | 1.8E2 | 7.5E2 | 3.0E2 | 2.0E3 | 4.4E2 | 2.7E3 | 1.0E-9 | 1.6E1 | 4.5E3 | 8.5E3 | 342 | 9 | 342 | 9 | 0.82 |
| Oy | pg/ml | 4.7E-1 | 5.3E-1 | 6.1E0 | 7.6E0 | 3.1E1 | 1.2E1 | 1.0E-9 | 1.0E-9 | 4.0E2 | 3.3E1 | 341 | 9 | 341 | 9 | 0.54 |
| Oz | pg/ml | 4.5E-3 | 1.0E-9 | 3.3E-1 | 3.2E0 | 1.6E0 | 9.3E0 | 1.0E-9 | 1.0E-9 | 2.9E1 | 2.8E1 | 341 | 9 | 341 | 9 | 0.43 |
| Pa | pg/ml | 3.9E-1 | 7.1E-1 | 1.7E0 | 3.0E1 | 7.9E0 | 7.4E1 | 1.0E-9 | 1.0E-9 | 1.0E2 | 2.3E2 | 341 | 9 | 341 | 9 | 0.66 |
| Pb | pg/ml | 1.0E-9 | 1.0E-9 | 1.7E0 | 2.3E-1 | 2.6E1 | 6.4E-1 | 1.0E-9 | 1.0E-9 | 4.9E2 | 1.9E0 | 341 | 9 | 341 | 9 | 0.48 |
| Pc | pg/ml | 4.8E-2 | 1.0E-9 | 3.9E-1 | 3.7E1 | 9.0E-1 | 1.1E2 | 1.0E-9 | 1.0E-9 | 1.4E1 | 3.3E2 | 341 | 9 | 341 | 9 | 0.46 |
| Pd | pg/ml | 1.6E0 | 1.5E0 | 6.7E0 | 1.2E1 | 4.7E1 | 2.0E1 | 1.0E-9 | 6.3E-3 | 8.4E2 | 5.5E1 | 341 | 9 | 341 | 9 | 0.56 |
| Pe | pg/ml | 2.1E1 | 2.2E2 | 1.4E2 | 2.0E3 | 6.3E2 | 4.9E3 | 1.0E-9 | 3.3E0 | 6.7E3 | 1.5E4 | 341 | 9 | 341 | 9 | 0.80 |
| Pf | pg/ml | 1.6E0 | 1.4E1 | 1.6E1 | 3.2E1 | 9.3E1 | 4.9E1 | 1.0E-9 | 1.0E-9 | 1.5E3 | 1.5E2 | 341 | 9 | 341 | 9 | 0.74 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pg | pg/ml | 3.9E0 | 2.2E1 | 7.2E1 | 4.0E2 | 5.2E2 | 5.1E2 | 1.0E-9 | 1.9E0 | 7.7E3 | 1.3E3 | 341 | 9 | 341 | 9 | 0.77 |
| aA | mg/dL | 9.0E-1 | 1.4E0 | 1.0E0 | 2.1E0 | 5.3E-1 | 1.3E0 | 3.0E-1 | 8.9E-1 | 4.2E0 | 4.7E0 | 518 | 15 | 518 | 15 | 0.83 |
| aC | mg/mL | 2.2E0 | 4.1E0 | 2.6E0 | 3.7E0 | 1.3E0 | 2.2E0 | 7.5E-1 | 1.0E0 | 7.4E0 | 6.7E0 | 166 | 8 | 166 | 8 | 0.63 |
| aD | ug/mL | 3.0E0 | 2.8E0 | 4.7E0 | 3.0E0 | 4.8E0 | 1.1E0 | 7.5E-1 | 1.8E0 | 3.5E1 | 4.7E0 | 166 | 8 | 166 | 8 | 0.43 |
| aE | mg/mL | 5.7E-1 | 5.6E-1 | 5.9E-1 | 5.5E-1 | 1.7E-1 | 1.0E-1 | 1.8E-1 | 4.1E-1 | 1.2E0 | 6.9E-1 | 166 | 8 | 166 | 8 | 0.43 |
| aF | ng/mL | 2.2E0 | 2.9E0 | 5.0E0 | 5.5E0 | 7.8E0 | 5.7E0 | 4.3E-3 | 4.3E-3 | 5.0E1 | 1.5E1 | 166 | 8 | 166 | 8 | 0.56 |
| aG | mg/mL | 1.4E-1 | 9.2E-2 | 1.6E-1 | 9.7E-2 | 8.7E-2 | 2.5E-2 | 3.2E-2 | 6.9E-2 | 4.8E-1 | 1.5E-1 | 166 | 8 | 166 | 8 | 0.27 |
| aH | ug/mL | 7.2E1 | 5.9E1 | 7.8E1 | 6.5E1 | 4.1E1 | 1.8E1 | 8.9E0 | 5.0E1 | 2.0E2 | 1.0E2 | 166 | 8 | 166 | 8 | 0.45 |
| aI | ug/mL | 1.7E2 | 1.3E2 | 1.8E2 | 1.4E2 | 6.2E1 | 2.3E1 | 3.2E1 | 9.7E1 | 3.4E2 | 1.8E2 | 166 | 8 | 166 | 8 | 0.28 |
| aJ | ug/mL | 2.3E0 | 5.5E0 | 3.1E0 | 7.6E0 | 2.2E0 | 6.6E0 | 8.2E-1 | 2.2E0 | 1.4E1 | 2.3E1 | 166 | 8 | 166 | 8 | 0.83 |
| aK | ng/mL | 1.3E0 | 5.6E-1 | 2.0E0 | 1.7E0 | 2.0E0 | 2.2E0 | 2.9E-4 | 1.3E-1 | 1.0E1 | 6.5E0 | 166 | 8 | 166 | 8 | 0.42 |
| aL | mg/mL | 7.4E-1 | 5.3E-1 | 7.7E-1 | 5.9E-1 | 2.6E-1 | 2.3E-1 | 2.2E-1 | 3.2E-1 | 1.7E0 | 9.2E-1 | 166 | 8 | 166 | 8 | 0.30 |
| aM | U/mL | 1.8E1 | 4.0E1 | 4.0E1 | 1.5E2 | 7.8E1 | 2.4E2 | 4.2E-2 | 4.2E-2 | 8.2E2 | 6.8E2 | 166 | 8 | 166 | 8 | 0.70 |
| aN | U/mL | 1.4E1 | 2.4E1 | 2.5E1 | 2.7E1 | 4.3E1 | 2.6E1 | 2.5E-3 | 7.4E0 | 3.8E2 | 8.8E1 | 166 | 8 | 166 | 8 | 0.60 |
| aO | pg/mL | 5.3E1 | 1.6E2 | 4.2E2 | 6.9E2 | 9.8E2 | 8.5E2 | 6.0E-2 | 3.2E1 | 6.6E3 | 2.1E3 | 166 | 8 | 166 | 8 | 0.70 |
| aP | ng/mL | 1.6E0 | 4.6E0 | 2.2E0 | 7.1E0 | 2.4E0 | 8.6E0 | 4.5E-1 | 1.6E0 | 2.8E1 | 2.8E1 | 166 | 8 | 166 | 8 | 0.87 |
| aQ | ng/mL | 2.4E-1 | 3.0E-1 | 3.6E-1 | 3.5E-1 | 3.2E-1 | 2.6E-1 | 2.0E-4 | 5.2E-2 | 2.0E0 | 9.0E-1 | 166 | 8 | 166 | 8 | 0.53 |
| aR | ng/mL | 1.7E0 | 3.4E0 | 2.9E0 | 4.4E0 | 4.0E0 | 4.2E0 | 2.6E-1 | 6.5E-1 | 3.4E1 | 1.4E1 | 166 | 8 | 166 | 8 | 0.66 |
| aS | ng/mL | 3.7E-1 | 5.2E-1 | 1.0E0 | 8.2E-1 | 2.7E0 | 8.2E-1 | 4.2E-3 | 8.0E-2 | 3.3E1 | 2.6E0 | 166 | 8 | 166 | 8 | 0.58 |
| aU | pg/mL | 6.6E1 | 3.9E1 | 1.0E2 | 1.1E2 | 1.0E2 | 1.7E2 | 7.4E-2 | 7.4E-2 | 7.0E2 | 5.1E2 | 166 | 8 | 166 | 8 | 0.39 |
| aV | ng/mL | 5.9E-1 | 3.6E-1 | 1.1E0 | 6.2E-1 | 2.6E0 | 7.5E-1 | 7.6E-4 | 1.5E-1 | 3.3E1 | 2.4E0 | 166 | 8 | 166 | 8 | 0.39 |
| aW | pg/mL | 1.9E1 | 2.5E1 | 2.3E1 | 2.4E1 | 3.4E1 | 1.4E1 | 7.2E-2 | 7.2E-2 | 4.2E2 | 4.7E1 | 166 | 8 | 166 | 8 | 0.61 |
| aX | ng/mL | 7.5E0 | 4.7E1 | 1.3E1 | 8.4E1 | 2.0E1 | 1.0E2 | 3.0E-1 | 2.5E0 | 2.2E2 | 3.1E2 | 166 | 8 | 166 | 8 | 0.76 |
| aY | pg/mL | 5.3E1 | 6.0E1 | 7.5E1 | 1.0E2 | 1.1E2 | 1.2E2 | 4.1E-1 | 2.7E1 | 1.2E3 | 3.9E2 | 166 | 8 | 166 | 8 | 0.56 |
| aZ | pg/mL | 2.3E2 | 1.3E3 | 5.7E2 | 2.9E3 | 1.2E3 | 3.3E3 | 1.7E0 | 7.5E1 | 1.2E4 | 7.9E3 | 166 | 8 | 166 | 8 | 0.75 |
| bA | ng/mL | 1.3E1 | 1.3E2 | 6.3E1 | 3.1E2 | 1.4E2 | 4.8E2 | 3.0E-2 | 4.7E0 | 9.4E2 | 1.5E3 | 166 | 8 | 166 | 8 | 0.82 |
| bB | ng/mL | 2.8E2 | 1.8E2 | 3.1E2 | 2.2E2 | 1.8E2 | 8.2E1 | 2.1E0 | 1.3E2 | 9.5E2 | 3.8E2 | 166 | 8 | 166 | 8 | 0.35 |
| bC | ng/mL | 3.2E2 | 1.3E3 | 5.9E2 | 1.9E3 | 7.7E2 | 1.9E3 | 1.4E1 | 5.8E1 | 4.7E3 | 4.0E3 | 166 | 8 | 166 | 8 | 0.68 |
| bE | mg/mL | 5.2E0 | 5.3E0 | 5.4E0 | 6.6E0 | 2.1E0 | 3.5E0 | 1.3E0 | 2.6E0 | 1.2E1 | 1.2E1 | 166 | 8 | 166 | 8 | 0.57 |
| bF | pg/mL | 3.5E1 | 6.6E1 | 3.5E2 | 7.1E1 | 1.4E3 | 4.2E1 | 5.0E-2 | 1.4E1 | 1.1E4 | 1.5E2 | 166 | 8 | 166 | 8 | 0.65 |
| bG | ng/mL | 1.6E0 | 1.8E0 | 3.1E0 | 4.7E0 | 4.4E0 | 5.8E0 | 1.1E-1 | 1.6E-1 | 3.0E1 | 1.5E1 | 166 | 8 | 166 | 8 | 0.55 |
| bH | pg/mL | 5.7E-1 | 6.9E0 | 6.2E0 | 8.5E0 | 2.4E1 | 9.0E0 | 5.7E-1 | 5.7E-1 | 2.8E2 | 2.3E1 | 166 | 8 | 166 | 8 | 0.63 |
| bI | ng/mL | 4.0E-3 | 4.0E-3 | 9.5E-2 | 7.3E-2 | 2.0E-1 | 1.4E-1 | 4.0E-3 | 4.0E-3 | 9.8E-1 | 3.9E-1 | 166 | 8 | 166 | 8 | 0.51 |
| bJ | mg/mL | 1.9E0 | 2.2E0 | 2.4E0 | 2.8E0 | 2.0E0 | 2.5E0 | 2.5E-4 | 8.7E-1 | 1.1E1 | 8.9E0 | 166 | 8 | 166 | 8 | 0.56 |
| bL | pg/mL | 3.7E0 | 1.4E1 | 9.1E0 | 1.3E1 | 1.2E1 | 7.8E0 | 4.6E-2 | 3.2E0 | 6.0E1 | 2.4E1 | 166 | 8 | 166 | 8 | 0.72 |
| bM | mg/mL | 1.8E0 | 1.1E0 | 2.2E0 | 1.4E0 | 1.5E0 | 1.2E0 | 1.8E-2 | 1.6E-2 | 8.6E0 | 3.8E0 | 166 | 8 | 166 | 8 | 0.33 |
| bN | ng/mL | 3.3E1 | 3.0E1 | 1.2E2 | 6.1E1 | 2.9E2 | 7.9E1 | 1.4E-1 | 6.7E0 | 1.9E3 | 2.5E2 | 166 | 8 | 166 | 8 | 0.49 |
| bO | ng/mL | 4.0E-2 | 4.0E-2 | 9.6E-2 | 4.0E-2 | 2.0E1 | 0.0E0 | 4.0E-2 | 4.0E-2 | 1.3E2 | 4.0E-2 | 166 | 8 | 166 | 8 | 0.33 |
| bP | mg/mL | 5.2E-1 | 5.4E-1 | 7.5E-1 | 6.5E-1 | 7.1E-1 | 5.0E-1 | 4.9E-2 | 9.2E-2 | 4.8E0 | 1.6E0 | 166 | 8 | 166 | 8 | 0.48 |
| bQ | pg/mL | 2.1E1 | 8.1E1 | 1.4E2 | 7.8E1 | 1.1E3 | 4.9E1 | 1.5E-1 | 2.2E1 | 1.3E4 | 1.4E2 | 166 | 8 | 166 | 8 | 0.77 |
| bR | ng/mL | 1.2E-2 | 1.2E-2 | 1.6E-1 | 9.2E-2 | 7.2E-1 | 1.5E-1 | 1.2E-2 | 1.2E-2 | 8.7E0 | 3.8E-1 | 166 | 8 | 166 | 8 | 0.47 |
| bS | pg/mL | 9.4E-1 | 9.4E-1 | 9.0E0 | 2.1E1 | 4.1E1 | 3.1E1 | 9.4E-1 | 9.4E-1 | 3.9E2 | 7.2E1 | 166 | 8 | 166 | 8 | 0.64 |
| bU | ng/mL | 7.0E-2 | 2.0E-1 | 1.9E-1 | 1.8E-1 | 5.4E-1 | 1.5E-1 | 1.3E-2 | 1.3E-2 | 6.6E0 | 4.1E-1 | 166 | 8 | 166 | 8 | 0.59 |
| bV | pg/mL | 4.8E2 | 6.7E2 | 6.2E2 | 8.8E2 | 9.0E2 | 5.8E2 | 1.6E2 | 5.1E2 | 1.2E4 | 2.2E3 | 166 | 8 | 166 | 8 | 0.75 |
| bW | pg/mL | 3.1E2 | 6.2E2 | 4.7E2 | 1.4E3 | 5.0E2 | 1.6E3 | 8.4E1 | 2.5E2 | 4.8E3 | 3.9E3 | 166 | 8 | 166 | 8 | 0.74 |
| bX | ng/mL | 2.5E-5 | 1.6E-3 | 2.6E-3 | 2.7E-3 | 3.2E-3 | 3.1E-3 | 2.5E-5 | 2.5E-5 | 1.3E-2 | 7.2E-3 | 166 | 8 | 166 | 8 | 0.52 |
| bZ | pg/mL | 2.7E2 | 1.8E3 | 2.0E3 | 2.4E3 | 7.2E3 | 2.5E3 | 1.5E-1 | 1.4E2 | 5.8E4 | 7.4E3 | 166 | 8 | 166 | 8 | 0.72 |
| cA | pg/mL | 6.0E-1 | 6.0E-1 | 4.1E0 | 4.0E0 | 2.9E1 | 7.3E0 | 6.0E-1 | 6.0E-1 | 3.7E2 | 2.1E1 | 166 | 8 | 166 | 8 | 0.56 |
| cB | ng/mL | 5.1E-2 | 1.8E-2 | 7.5E-2 | 7.0E-2 | 8.8E-2 | 1.0E-1 | 1.7E-3 | 1.7E-3 | 4.3E-1 | 2.6E-1 | 166 | 8 | 166 | 8 | 0.39 |
| cC | pg/mL | 4.3E1 | 2.9E1 | 4.6E1 | 2.8E1 | 5.1E1 | 2.2E1 | 1.0E0 | 1.0E0 | 4.5E2 | 5.9E1 | 166 | 8 | 166 | 8 | 0.36 |
| cD | pg/mL | 4.9E0 | 4.4E0 | 1.3E1 | 5.1E0 | 4.6E1 | 3.7E0 | 3.3E-1 | 8.8E-1 | 4.8E2 | 9.6E0 | 166 | 8 | 166 | 8 | 0.48 |
| cE | pg/mL | 6.0E1 | 1.2E2 | 2.7E2 | 1.4E2 | 6.1E2 | 9.4E1 | 1.2E-1 | 3.2E1 | 3.8E3 | 2.8E2 | 166 | 8 | 166 | 8 | 0.63 |
| cF | pg/mL | 2.5E0 | 5.3E-1 | 1.6E1 | 9.0E0 | 3.0E1 | 1.4E1 | 5.3E-1 | 5.3E-1 | 2.7E2 | 3.8E1 | 166 | 8 | 166 | 8 | 0.44 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| cG | pg/mL | 5.3E1 | 1.8E2 | 1.7E2 | 3.2E2 | 8.2E2 | 3.4E2 | 7.8E0 | 4.0E1 | 1.0E4 | 1.1E3 | 166 | 8 | 166 | 8 | 0.80 |
| cH | uIU/mL | 3.3E0 | 4.2E0 | 7.7E0 | 7.8E0 | 1.7E1 | 1.1E1 | 8.6E-3 | 8.6E-3 | 1.6E2 | 3.4E1 | 166 | 8 | 166 | 8 | 0.52 |
| cI | ng/mL | 6.1E0 | 6.1E0 | 1.4E1 | 1.3E1 | 2.2E1 | 1.6E1 | 3.2E-2 | 1.1E0 | 1.2E2 | 4.1E1 | 166 | 8 | 166 | 8 | 0.49 |
| cJ | ug/mL | 6.7E1 | 3.1E1 | 1.0E2 | 4.3E1 | 1.0E2 | 5.2E1 | 6.9E0 | 5.6E0 | 6.4E2 | 1.7E2 | 166 | 8 | 166 | 8 | 0.28 |
| cK | ng/mL | 3.8E-3 | 3.8E-3 | 2.2E-2 | 3.5E-2 | 1.2E-1 | 7.2E-2 | 3.8E-3 | 3.8E-3 | 1.5E0 | 2.1E-1 | 166 | 8 | 166 | 8 | 0.57 |
| cL | pg/mL | 2.1E2 | 2.1E2 | 5.3E2 | 6.9E2 | 2.0E3 | 9.4E2 | 3.1E1 | 6.7E1 | 2.4E4 | 2.8E3 | 166 | 8 | 166 | 8 | 0.58 |
| cM | pg/mL | 2.7E2 | 2.3E2 | 2.9E2 | 2.3E2 | 1.7E2 | 6.8E1 | 2.5E1 | 1.3E2 | 1.1E3 | 3.1E2 | 166 | 8 | 166 | 8 | 0.40 |
| cN | pg/mL | 1.2E2 | 1.3E2 | 1.3E2 | 1.4E2 | 8.6E1 | 4.0E1 | 3.8E1 | 8.6E1 | 1.1E3 | 1.9E2 | 166 | 8 | 166 | 8 | 0.60 |
| cO | pg/mL | 2.1E2 | 4.7E2 | 4.0E2 | 5.8E2 | 1.5E3 | 4.4E2 | 5.4E1 | 9.6E1 | 1.9E4 | 1.5E3 | 166 | 8 | 166 | 8 | 0.75 |
| cP | ng/mL | 2.4E3 | 2.9E3 | 2.5E3 | 2.8E3 | 9.5E2 | 1.1E3 | 6.2E2 | 1.4E3 | 5.6E3 | 4.7E3 | 166 | 8 | 166 | 8 | 0.58 |
| cQ | ng/mL | 5.0E-2 | 1.3E-1 | 1.3E-1 | 1.8E-1 | 2.1E-1 | 1.7E-1 | 2.0E-3 | 2.0E-3 | 1.3E0 | 3.9E-1 | 166 | 8 | 166 | 8 | 0.62 |
| cR | ng/mL | 3.3E2 | 8.4E2 | 5.8E2 | 1.0E3 | 8.3E2 | 7.7E2 | 2.0E1 | 1.1E2 | 7.7E3 | 2.2E3 | 166 | 8 | 166 | 8 | 0.71 |
| cS | ng/mL | 2.8E2 | 8.8E2 | 4.1E2 | 1.9E3 | 4.2E2 | 2.4E3 | 4.1E1 | 1.4E2 | 2.5E3 | 7.1E3 | 166 | 8 | 166 | 8 | 0.75 |
| cT | ng/mL | 4.9E1 | 1.5E2 | 1.5E2 | 3.8E2 | 2.9E2 | 5.1E2 | 3.6E0 | 1.9E1 | 2.1E3 | 1.5E3 | 166 | 8 | 166 | 8 | 0.71 |
| cU | ng/mL | 5.8E1 | 1.9E2 | 9.4E1 | 2.1E2 | 1.5E2 | 1.0E2 | 6.2E0 | 9.8E1 | 1.6E3 | 3.9E2 | 166 | 8 | 166 | 8 | 0.89 |
| cV | ng/mL | 2.0E-1 | 1.8E-1 | 7.6E-1 | 2.6E-1 | 3.8E0 | 1.5E-1 | 2.5E-2 | 9.7E-2 | 4.7E1 | 4.9E-1 | 166 | 8 | 166 | 8 | 0.53 |
| cW | mIU/mL | 4.8E-2 | 1.0E-1 | 8.8E-2 | 1.1E-1 | 3.5E-1 | 8.7E-2 | 4.8E-3 | 3.3E-2 | 4.5E0 | 2.9E-1 | 166 | 8 | 166 | 8 | 0.70 |
| cX | ng/mL | 1.2E-1 | 3.8E-2 | 1.9E0 | 1.2E-1 | 5.7E0 | 1.4E-1 | 2.3E-4 | 1.1E-2 | 2.8E1 | 4.1E-1 | 166 | 8 | 166 | 8 | 0.41 |
| cY | ng/mL | 7.5E0 | 4.4E0 | 1.1E1 | 9.2E0 | 1.1E1 | 1.2E1 | 1.7E-1 | 6.0E-1 | 6.1E1 | 3.6E1 | 166 | 8 | 166 | 8 | 0.40 |
| cZ | ug/mL | 1.3E1 | 1.1E1 | 1.5E1 | 1.5E1 | 6.8E0 | 7.9E0 | 2.3E0 | 7.1E0 | 4.6E1 | 3.0E1 | 166 | 8 | 166 | 8 | 0.45 |
| dA | pg/mL | 3.1E2 | 4.1E2 | 3.8E2 | 4.6E2 | 4.6E2 | 2.5E2 | 1.0E2 | 1.7E2 | 5.8E3 | 8.8E2 | 166 | 8 | 166 | 8 | 0.62 |
| dB | ug/mL | 1.8E1 | 2.0E1 | 1.8E1 | 2.1E1 | 2.0E1 | 5.7E0 | 2.1E0 | 1.5E1 | 2.5E2 | 2.8E1 | 166 | 8 | 166 | 8 | 0.62 |
| dC | nmol/L | 3.5E1 | 2.7E1 | 3.8E1 | 2.7E1 | 1.7E1 | 6.4E0 | 7.8E0 | 1.5E1 | 1.4E2 | 3.8E1 | 166 | 8 | 166 | 8 | 0.27 |
| dD | ug/mL | 3.4E1 | 2.9E1 | 3.5E1 | 3.1E1 | 1.1E1 | 7.0E0 | 1.4E1 | 2.3E1 | 7.4E1 | 4.3E1 | 166 | 8 | 166 | 8 | 0.35 |
| dE | ng/mL | 4.1E-1 | 9.9E-1 | 5.4E-1 | 1.2E0 | 5.5E-1 | 9.3E-1 | 8.4E-3 | 3.0E-1 | 2.7E0 | 2.9E0 | 166 | 8 | 166 | 8 | 0.75 |
| dF | ng/mL | 2.4E2 | 3.8E2 | 3.4E2 | 5.1E2 | 2.5E2 | 2.5E2 | 7.5E1 | 2.8E2 | 1.3E3 | 8.9E2 | 166 | 8 | 166 | 8 | 0.75 |
| dG | ng/mL | 1.2E1 | 2.0E1 | 1.7E1 | 2.6E1 | 1.9E1 | 1.9E1 | 3.0E0 | 6.7E0 | 1.8E2 | 6.5E1 | 166 | 8 | 166 | 8 | 0.70 |
| dH | pg/mL | 8.0E0 | 1.3E1 | 2.1E1 | 1.9E1 | 6.3E1 | 2.3E1 | 4.0E-2 | 8.3E-1 | 6.7E2 | 7.6E1 | 166 | 8 | 166 | 8 | 0.65 |
| dI | pg/mL | 4.6E-1 | 1.7E0 | 3.7E0 | 4.9E0 | 2.6E1 | 6.5E0 | 4.6E-1 | 4.6E-1 | 3.3E2 | 1.9E1 | 166 | 8 | 166 | 8 | 0.66 |
| dJ | ng/mL | 2.0E0 | 2.0E0 | 2.1E0 | 2.2E0 | 1.1E0 | 1.2E0 | 3.2E-2 | 5.7E-1 | 5.6E0 | 4.0E0 | 166 | 8 | 166 | 8 | 0.51 |
| dK | uIU/mL | 1.3E0 | 3.8E-1 | 2.2E0 | 1.2E0 | 3.6E0 | 2.1E0 | 2.8E-4 | 1.4E-2 | 3.9E1 | 6.1E0 | 166 | 8 | 166 | 8 | 0.26 |
| dL | ng/mL | 8.7E2 | 1.0E3 | 1.1E3 | 1.2E3 | 6.3E2 | 6.1E2 | 2.8E2 | 6.3E2 | 4.8E3 | 2.3E3 | 166 | 8 | 166 | 8 | 0.62 |
| dM | pg/mL | 9.7E2 | 2.2E3 | 1.3E3 | 2.7E3 | 1.5E3 | 2.0E3 | 3.7E2 | 7.1E2 | 1.5E4 | 5.8E3 | 166 | 8 | 166 | 8 | 0.72 |
| dN | ug/mL | 9.8E1 | 1.4E2 | 1.0E2 | 1.3E2 | 4.4E1 | 3.6E1 | 2.4E1 | 6.6E1 | 3.3E2 | 1.7E2 | 166 | 8 | 166 | 8 | 0.72 |
| fR | ng/ml | 1.5E5 | 2.9E5 | 2.1E5 | 3.5E5 | 1.7E5 | 2.6E5 | 3.6E4 | 1.9E2 | 6.9E5 | 8.7E5 | 106 | 9 | 106 | 9 | 0.68 |

Figure 38.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.5E1 | 1.3E2 | 8.5E1 | 1.6E2 | 5.7E1 | 1.0E2 | 7.0E0 | 2.5E1 | 4.0E2 | 4.8E2 | 238 | 34 | 238 | 34 | 0.75 |
| Ad | ug/mL | 4.8E-2 | 9.2E-2 | 7.2E-2 | 5.3E-1 | 8.8E-2 | 1.9E0 | 6.8E-4 | 8.1E-3 | 5.4E-1 | 8.5E0 | 121 | 20 | 121 | 20 | 0.66 |
| Af | ng/mL | 1.3E0 | 7.7E-1 | 1.1E1 | 4.0E0 | 5.1E1 | 6.8E0 | 1.7E-3 | 1.7E-3 | 5.3E2 | 2.3E1 | 121 | 20 | 121 | 20 | 0.44 |
| Aj | ug/mL | 1.1E0 | 2.9E-1 | 2.3E0 | 1.6E0 | 2.4E0 | 2.3E0 | 1.5E-3 | 2.3E-3 | 6.1E0 | 6.1E0 | 121 | 20 | 121 | 20 | 0.40 |
| Al | mg/mL | 8.3E-5 | 1.2E-4 | 2.5E-4 | 3.7E-4 | 4.2E-4 | 5.1E-4 | 4.6E-6 | 7.6E-6 | 1.8E-3 | 1.8E-3 | 121 | 20 | 121 | 20 | 0.60 |
| An | U/mL | 6.2E1 | 1.1E2 | 2.4E2 | 5.9E2 | 6.2E2 | 1.7E3 | 6.1E-1 | 6.4E-1 | 5.5E3 | 7.8E3 | 121 | 20 | 121 | 20 | 0.59 |
| Ao | pg/mL | 9.2E1 | 1.2E2 | 6.7E2 | 1.9E2 | 4.2E3 | 1.9E2 | 2.8E0 | 1.7E1 | 3.9E4 | 6.9E2 | 121 | 20 | 121 | 20 | 0.61 |
| Ap | ng/mL | 3.2E1 | 4.6E1 | 4.2E1 | 5.8E1 | 4.3E1 | 5.2E1 | 2.0E0 | 6.4E0 | 2.9E2 | 2.1E2 | 121 | 20 | 121 | 20 | 0.60 |
| Ar | ng/mL | 5.7E-1 | 2.4E0 | 3.1E0 | 6.0E0 | 7.6E0 | 1.1E1 | 4.1E-2 | 3.4E-3 | 5.1E1 | 5.0E1 | 121 | 20 | 121 | 20 | 0.71 |
| As | ng/mL | 9.4E-3 | 1.1E-2 | 1.3E-2 | 7.5E-2 | 1.8E-2 | 2.7E-1 | 1.7E-3 | 1.7E-3 | 1.3E-1 | 1.2E0 | 121 | 20 | 121 | 20 | 0.51 |
| Aw | pg/mL | 1.6E1 | 1.7E1 | 1.7E1 | 1.9E1 | 5.8E0 | 9.3E0 | 2.9E-2 | 1.1E1 | 4.2E1 | 5.1E1 | 121 | 20 | 121 | 20 | 0.54 |
| Ax | ng/mL | 2.2E0 | 8.0E0 | 2.2E1 | 1.2E2 | 9.1E1 | 2.2E2 | 1.3E-2 | 1.2E-2 | 7.7E2 | 8.5E2 | 121 | 20 | 121 | 20 | 0.68 |
| Ba | ng/mL | 8.1E1 | 3.2E2 | 5.5E2 | 1.1E3 | 1.5E3 | 2.1E3 | 1.1E0 | 3.1E0 | 8.1E3 | 8.1E3 | 121 | 20 | 121 | 20 | 0.63 |
| Bb | ng/mL | 3.9E0 | 1.4E1 | 6.2E0 | 1.4E1 | 7.7E0 | 1.0E1 | 4.1E-3 | 1.4E0 | 4.9E1 | 3.7E1 | 121 | 20 | 121 | 20 | 0.75 |
| Bc | ng/mL | 3.3E1 | 8.0E1 | 1.2E2 | 1.8E2 | 2.3E2 | 2.9E2 | 4.9E-1 | 5.6E0 | 1.2E3 | 1.0E3 | 121 | 20 | 121 | 20 | 0.66 |
| Bg | ng/mL | 1.1E-1 | 3.9E-1 | 8.2E0 | 1.8E0 | 4.1E1 | 2.5E0 | 5.3E-4 | 2.4E-2 | 4.0E2 | 8.0E0 | 121 | 20 | 121 | 20 | 0.62 |
| Bn | ng/mL | 5.6E-2 | 5.6E-2 | 1.2E0 | 3.5E0 | 2.1E0 | 1.3E1 | 5.6E-2 | 5.6E-2 | 8.6E0 | 5.8E1 | 121 | 20 | 121 | 20 | 0.45 |
| Bo | ng/mL | 1.3E1 | 1.5E1 | 1.5E1 | 1.6E1 | 1.1E1 | 1.4E1 | 1.6E-2 | 1.6E-2 | 5.0E1 | 5.3E1 | 121 | 20 | 121 | 20 | 0.51 |
| Ch | uIU/mL | 1.2E0 | 9.4E-1 | 4.7E1 | 5.8E0 | 2.1E2 | 1.2E1 | 3.4E-3 | 1.4E-1 | 1.8E3 | 5.0E1 | 121 | 20 | 121 | 20 | 0.44 |
| Co | pg/mL | 4.6E1 | 6.5E1 | 2.7E2 | 2.0E2 | 1.6E3 | 4.5E2 | 1.5E-1 | 2.0E1 | 1.7E4 | 2.1E3 | 121 | 20 | 121 | 20 | 0.66 |
| Cp | ng/mL | 2.2E1 | 2.2E1 | 2.8E1 | 9.7E1 | 2.1E1 | 2.8E2 | 6.0E-1 | 1.0E1 | 1.3E2 | 1.3E3 | 121 | 20 | 121 | 20 | 0.56 |
| Cq | ng/mL | 3.0E-2 | 3.3E-2 | 1.2E-1 | 2.6E0 | 5.1E-1 | 1.1E1 | 8.0E-4 | 8.0E-4 | 5.1E0 | 4.9E1 | 121 | 20 | 121 | 20 | 0.56 |
| Cs | ng/mL | 7.2E1 | 3.1E2 | 4.9E2 | 1.1E3 | 1.9E3 | 1.7E3 | 1.0E0 | 8.3E-1 | 1.8E4 | 5.1E3 | 121 | 20 | 121 | 20 | 0.69 |
| Ct | ng/mL | 3.3E-1 | 3.0E-1 | 4.6E1 | 4.0E1 | 1.3E2 | 1.1E2 | 1.5E-2 | 1.1E-4 | 6.2E2 | 4.7E2 | 121 | 20 | 121 | 20 | 0.48 |
| Cu | ng/mL | 2.6E-1 | 3.7E-1 | 4.4E-1 | 5.0E0 | 6.6E-1 | 1.5E1 | 2.8E-2 | 5.4E-2 | 4.5E0 | 6.6E1 | 121 | 20 | 121 | 20 | 0.64 |
| Cv | ng/mL | 3.9E0 | 1.7E1 | 2.2E1 | 5.9E1 | 5.9E1 | 1.2E2 | 2.0E-2 | 5.1E-2 | 5.3E2 | 4.7E2 | 121 | 20 | 121 | 20 | 0.62 |
| Cw | mIU/mL | 3.1E-2 | 5.0E-2 | 4.2E-2 | 3.8E-1 | 3.5E-2 | 1.5E0 | 8.9E-4 | 4.1E-3 | 2.3E-1 | 6.8E0 | 121 | 20 | 121 | 20 | 0.62 |
| Cx | ng/mL | 9.5E-1 | 1.5E-2 | 4.8E1 | 3.8E1 | 9.6E1 | 8.6E1 | 9.3E-5 | 9.3E-5 | 3.9E2 | 2.8E2 | 121 | 20 | 121 | 20 | 0.40 |
| Db | ug/mL | 7.3E0 | 7.8E0 | 8.5E0 | 7.8E0 | 7.9E0 | 4.9E0 | 4.5E-1 | 1.0E0 | 5.9E1 | 1.9E1 | 121 | 20 | 121 | 20 | 0.52 |
| Dc | nmol/L | 2.1E-2 | 2.4E-2 | 6.1E-2 | 9.3E-1 | 1.7E-1 | 3.1E0 | 5.2E-6 | 3.3E-3 | 1.6E0 | 1.4E1 | 121 | 20 | 121 | 20 | 0.66 |
| Dd | ug/mL | 6.9E-2 | 2.8E-1 | 1.7E-1 | 4.7E-1 | 2.5E-1 | 8.2E-1 | 4.8E-4 | 3.4E-3 | 1.6E0 | 3.6E0 | 121 | 20 | 121 | 20 | 0.64 |
| De | ng/mL | 3.4E-3 | 3.4E-3 | 8.3E-2 | 1.1E-1 | 1.4E-1 | 3.0E-1 | 3.4E-3 | 3.4E-3 | 9.2E-1 | 1.1E0 | 121 | 20 | 121 | 20 | 0.41 |
| Dg | ng/mL | 3.7E1 | 3.7E1 | 4.6E1 | 5.3E1 | 3.9E1 | 4.1E1 | 7.1E-1 | 2.3E0 | 1.9E2 | 1.2E2 | 121 | 20 | 121 | 20 | 0.55 |
| Di | pg/mL | 2.0E0 | 2.8E0 | 2.4E0 | 3.1E0 | 2.2E0 | 1.9E0 | 1.8E-1 | 1.8E-1 | 1.3E1 | 9.0E0 | 121 | 20 | 121 | 20 | 0.62 |
| Dk | uIU/mL | 1.6E-2 | 2.8E-2 | 5.8E-2 | 1.4E-1 | 1.8E-1 | 2.7E-1 | 1.1E-4 | 1.1E-4 | 1.6E0 | 9.8E-1 | 121 | 20 | 121 | 20 | 0.61 |
| Dl | ng/mL | 2.0E2 | 3.0E2 | 3.0E2 | 3.9E2 | 2.8E2 | 3.6E2 | 5.5E0 | 2.0E1 | 1.3E3 | 1.6E3 | 121 | 20 | 121 | 20 | 0.59 |
| Dp | ng/ml | 2.2E0 | 1.8E0 | 5.6E0 | 3.5E0 | 9.5E0 | 5.0E0 | 3.7E-3 | 3.7E-3 | 5.6E1 | 1.4E1 | 100 | 13 | 100 | 13 | 0.41 |
| Dr | pg/ml | 1.7E1 | 3.9E1 | 4.0E1 | 1.3E3 | 5.5E1 | 3.4E3 | 7.5E-1 | 7.5E-1 | 2.5E2 | 1.0E4 | 49 | 9 | 49 | 9 | 0.69 |
| Du | pg/ml | 1.6E2 | 9.6E2 | 1.1E3 | 4.0E3 | 3.2E3 | 8.6E3 | 1.2E0 | 1.2E0 | 2.0E4 | 2.4E4 | 41 | 7 | 41 | 7 | 0.63 |
| Ef | ng/ml | 9.4E-2 | 2.4E-1 | 7.8E-1 | 1.4E0 | 1.8E0 | 2.9E0 | 5.7E-4 | 5.7E-4 | 9.5E0 | 9.9E0 | 108 | 15 | 108 | 15 | 0.59 |
| Wm | % | 8.5E-2 | 2.7E-1 | 1.8E1 | 8.4E1 | 1.1E2 | 2.6E2 | 5.4E-2 | 8.5E-2 | 8.7E2 | 1.0E3 | 114 | 16 | 114 | 16 | 0.55 |
| Ed | pg/mL | 1.1E0 | 6.8E1 | 3.1E1 | 9.9E1 | 6.1E1 | 1.3E2 | 5.2E-1 | 5.2E-1 | 5.0E2 | 4.8E2 | 100 | 13 | 100 | 13 | 0.70 |
| Tj | pg/mL | 3.7E-1 | 3.7E-1 | 4.6E1 | 3.6E1 | 2.4E2 | 7.1E1 | 3.7E-1 | 3.7E-1 | 2.3E3 | 2.5E2 | 104 | 15 | 104 | 15 | 0.58 |
| Po | pg/ml | 2.1E-1 | 4.5E0 | 8.7E0 | 2.6E1 | 2.7E1 | 5.0E1 | 8.0E-3 | 2.6E-2 | 2.7E2 | 2.2E2 | 227 | 41 | 227 | 41 | 0.66 |
| Ti | ug/mL | 3.0E0 | 9.0E0 | 4.3E0 | 8.7E0 | 3.7E0 | 6.1E0 | 1.2E-1 | 9.7E-1 | 1.4E1 | 1.8E1 | 61 | 8 | 61 | 8 | 0.71 |
| Em | ng/ml | 1.3E-2 | 2.6E-2 | 5.5E-2 | 2.8E-1 | 9.8E-2 | 6.0E-1 | 8.4E-4 | 8.4E-4 | 5.0E-1 | 1.9E0 | 63 | 10 | 63 | 10 | 0.55 |
| Et | ng/ml | 1.4E3 | 3.0E3 | 1.7E3 | 2.8E3 | 1.1E3 | 1.3E3 | 7.9E1 | 5.9E2 | 4.3E3 | 5.0E3 | 226 | 41 | 226 | 41 | 0.76 |
| Eq | pg/ml | 1.6E2 | 3.1E1 | 3.7E2 | 2.5E2 | 4.0E2 | 5.2E2 | 1.0E0 | 1.0E0 | 1.5E3 | 1.4E3 | 41 | 7 | 41 | 7 | 0.31 |
| Th | ug/mL | 1.2E0 | 1.6E0 | 1.9E0 | 2.3E0 | 1.6E0 | 2.2E0 | 1.2E-1 | 2.6E-3 | 7.5E0 | 5.9E0 | 61 | 8 | 61 | 8 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Fa | ng/ml | 4.0E1 | 9.5E1 | 1.3E2 | 1.8E2 | 4.5E2 | 2.1E2 | 2.6E-1 | 6.0E-1 | 3.7E3 | 7.3E2 | 98 | 12 | 98 | 12 | 0.69 |
| Ez | ng/ml | 4.1E0 | 4.6E0 | 1.4E1 | 3.2E1 | 2.5E1 | 5.8E1 | 1.3E-2 | 9.5E-2 | 1.6E2 | 2.0E2 | 100 | 13 | 100 | 13 | 0.59 |
| Fb | ng/ml | 2.7E1 | 2.9E1 | 2.2E1 | 3.0E1 | 1.2E1 | 6.7E0 | 6.6E-1 | 2.2E1 | 4.3E1 | 4.1E1 | 99 | 12 | 99 | 12 | 0.68 |
| Ex | ng/ml | 9.0E-2 | 1.6E-1 | 1.7E-1 | 3.2E-1 | 2.6E-1 | 4.2E-1 | 3.5E-5 | 1.7E-4 | 1.5E0 | 1.2E0 | 76 | 11 | 76 | 11 | 0.62 |
| Fc | pg/ml | 2.2E-1 | 6.3E0 | 1.2E1 | 5.4E1 | 6.1E1 | 1.1E2 | 2.2E-1 | 2.2E-1 | 3.9E2 | 3.1E2 | 42 | 7 | 42 | 7 | 0.70 |
| Fd | pg/ml | 3.8E1 | 4.9E2 | 5.5E2 | 7.0E3 | 1.5E3 | 1.1E4 | 4.5E-1 | 9.8E-1 | 9.2E3 | 2.5E4 | 42 | 7 | 42 | 7 | 0.68 |
| Fi | pg/ml | 2.5E-1 | 6.1E1 | 6.1E1 | 1.7E2 | 2.9E2 | 2.1E2 | 2.5E-1 | 2.5E-1 | 1.8E3 | 5.3E2 | 42 | 7 | 42 | 7 | 0.72 |
| Fn | ng/ml | 2.1E-1 | 1.7E0 | 4.2E0 | 6.4E0 | 7.4E0 | 8.6E0 | 1.1E-14 | 2.1E-1 | 3.7E1 | 2.7E1 | 100 | 13 | 100 | 13 | 0.57 |
| Fp | ng/ml | 1.3E1 | 3.8E1 | 2.3E1 | 4.0E1 | 2.7E1 | 3.3E1 | 6.0E-3 | 1.2E0 | 1.3E2 | 1.3E2 | 230 | 41 | 230 | 41 | 0.68 |
| Fr | ng/ml | 3.4E4 | 1.1E5 | 1.2E5 | 2.5E5 | 1.9E5 | 2.7E5 | 1.9E2 | 1.8E3 | 8.4E5 | 8.4E5 | 233 | 42 | 233 | 42 | 0.70 |
| Fw | pg/ml | 8.5E-1 | 1.5E1 | 4.8E1 | 7.6E1 | 3.0E2 | 1.3E2 | 1.2E-1 | 1.2E-1 | 3.0E3 | 4.4E2 | 108 | 15 | 108 | 15 | 0.72 |
| Fy | ng/ml | 3.6E1 | 1.1E2 | 6.1E1 | 1.9E2 | 7.3E1 | 2.3E2 | 1.2E-1 | 7.0E0 | 5.0E2 | 6.5E2 | 99 | 12 | 99 | 12 | 0.68 |
| Gh | pg/ml | 2.0E0 | 3.9E0 | 2.1E1 | 1.2E1 | 5.8E1 | 1.7E1 | 2.9E-2 | 2.9E-2 | 3.2E2 | 4.6E1 | 42 | 7 | 42 | 7 | 0.57 |
| Gb | % | 4.4E1 | 6.2E1 | 4.4E1 | 1.0E2 | 3.1E1 | 1.0E2 | 2.2E0 | 2.5E1 | 1.4E2 | 3.0E2 | 42 | 7 | 42 | 7 | 0.70 |
| Gc | ng/ml | 8.3E1 | 2.5E2 | 1.5E2 | 3.1E2 | 2.2E2 | 2.2E2 | 6.4E0 | 6.7E1 | 1.2E3 | 6.7E2 | 49 | 9 | 49 | 9 | 0.76 |
| Gd | ng/ml | 3.4E1 | 2.7E1 | 3.5E1 | 3.5E1 | 1.7E1 | 2.5E1 | 3.0E0 | 9.6E0 | 6.9E1 | 8.0E1 | 59 | 8 | 59 | 8 | 0.46 |
| Gn | U/ml | 2.4E-1 | 2.7E-1 | 1.4E0 | 1.3E1 | 4.4E0 | 3.8E1 | 5.6E-3 | 2.7E-2 | 3.0E1 | 1.1E2 | 48 | 9 | 48 | 9 | 0.54 |
| Gl | pg/ml | 1.0E4 | 8.9E3 | 1.2E4 | 1.4E4 | 9.5E3 | 1.1E4 | 9.1E1 | 5.2E2 | 3.1E4 | 3.1E4 | 108 | 15 | 108 | 15 | 0.55 |
| Gp | U/ml | 1.2E0 | 1.6E-2 | 2.4E0 | 1.2E0 | 3.2E0 | 2.0E0 | 1.5E-2 | 1.5E-2 | 1.8E1 | 7.3E0 | 108 | 15 | 108 | 15 | 0.35 |
| Gz | ug/ml | 1.4E0 | 1.5E0 | 5.6E0 | 3.6E0 | 5.8E0 | 4.5E0 | 4.2E-2 | 1.0E-1 | 2.5E1 | 1.1E1 | 70 | 9 | 70 | 9 | 0.46 |
| Ha | ng/ml | 1.9E0 | 6.2E0 | 8.3E0 | 1.9E1 | 1.9E1 | 3.5E1 | 1.7E-2 | 1.7E-2 | 1.0E2 | 1.0E2 | 98 | 13 | 98 | 13 | 0.66 |
| Nm | pg/ml | 1.4E4 | 3.2E4 | 3.4E4 | 8.7E4 | 8.5E4 | 1.6E5 | 1.0E-9 | 1.2E3 | 9.6E5 | 8.2E5 | 230 | 41 | 230 | 41 | 0.63 |
| Nn | pg/ml | 1.5E2 | 4.3E2 | 1.8E3 | 1.5E4 | 8.0E3 | 5.2E4 | 1.0E-9 | 9.5E0 | 9.5E4 | 3.1E5 | 230 | 41 | 230 | 41 | 0.67 |
| No | pg/ml | 1.5E1 | 3.9E1 | 3.0E1 | 1.3E2 | 5.7E1 | 2.2E2 | 1.0E-9 | 4.3E0 | 5.6E2 | 9.1E2 | 230 | 41 | 230 | 41 | 0.75 |
| Nq | pg/ml | 1.7E0 | 6.6E0 | 2.4E1 | 6.3E1 | 7.9E1 | 1.4E2 | 1.0E-9 | 1.0E-9 | 6.7E2 | 6.3E2 | 230 | 41 | 230 | 41 | 0.64 |
| Nr | pg/ml | 1.5E0 | 9.1E0 | 2.2E1 | 8.5E1 | 8.3E1 | 2.4E2 | 1.0E-9 | 1.0E-9 | 9.8E2 | 1.4E3 | 230 | 41 | 230 | 41 | 0.68 |
| Ns | pg/ml | 1.0E-9 | 1.0E-9 | 1.1E1 | 1.0E-9 | 7.8E1 | 0.0E0 | 1.0E-9 | 1.0E-9 | 1.1E3 | 1.0E-9 | 230 | 41 | 230 | 41 | 0.47 |
| Nt | pg/ml | 1.1E2 | 1.5E2 | 1.4E2 | 2.6E2 | 1.0E2 | 3.2E2 | 1.5E1 | 5.5E1 | 8.8E2 | 1.7E3 | 230 | 41 | 230 | 41 | 0.65 |
| Nu | pg/ml | 2.4E1 | 5.0E1 | 5.7E1 | 8.8E1 | 8.8E1 | 9.9E1 | 1.0E-9 | 1.0E-9 | 6.3E2 | 3.7E2 | 230 | 41 | 230 | 41 | 0.63 |
| Lu | pg/ml | 1.0E4 | 1.0E4 | 1.7E4 | 1.1E4 | 4.8E4 | 1.0E4 | 9.4E2 | 5.2E2 | 5.6E5 | 5.7E4 | 230 | 41 | 230 | 41 | 0.51 |
| Lv | pg/ml | 1.0E-9 | 4.5E0 | 1.7E1 | 2.8E1 | 3.4E1 | 3.9E1 | 1.0E-9 | 1.0E-9 | 2.6E2 | 1.6E2 | 230 | 41 | 230 | 41 | 0.58 |
| Lw | pg/ml | 1.0E-9 | 1.0E-9 | 5.1E-1 | 6.4E0 | 5.7E0 | 2.8E1 | 1.0E-9 | 1.0E-9 | 8.0E1 | 1.8E2 | 230 | 41 | 230 | 41 | 0.55 |
| Lx | pg/ml | 1.0E-9 | 1.6E2 | 1.8E2 | 1.0E3 | 6.3E2 | 3.5E3 | 1.0E-9 | 1.0E-9 | 6.2E3 | 2.2E4 | 230 | 41 | 230 | 41 | 0.71 |
| Ly | pg/ml | 1.0E-9 | 1.0E-9 | 1.0E1 | 6.2E0 | 1.8E1 | 1.4E1 | 1.0E-9 | 1.0E-9 | 1.1E2 | 6.0E1 | 230 | 41 | 230 | 41 | 0.44 |
| Lz | pg/ml | 1.0E-9 | 1.0E-9 | 3.2E0 | 5.6E0 | 3.0E1 | 1.6E1 | 1.0E-9 | 1.0E-9 | 4.3E2 | 6.2E1 | 230 | 41 | 230 | 41 | 0.58 |
| Ma | pg/ml | 4.7E2 | 1.6E3 | 2.4E3 | 4.5E3 | 6.6E3 | 8.0E3 | 1.0E-9 | 2.6E1 | 6.5E4 | 3.6E4 | 230 | 41 | 230 | 41 | 0.67 |
| Mb | pg/ml | 2.6E1 | 2.8E1 | 3.3E1 | 3.1E1 | 1.9E1 | 1.4E1 | 9.2E0 | 4.1E0 | 2.1E2 | 6.4E1 | 230 | 41 | 230 | 41 | 0.49 |
| Mc | pg/ml | 1.0E-9 | 1.0E-9 | 8.3E-2 | 1.0E-9 | 9.1E-1 | 0.0E0 | 1.0E-9 | 1.0E-9 | 1.3E1 | 1.0E-9 | 230 | 41 | 230 | 41 | 0.49 |
| Md | pg/ml | 1.0E-9 | 1.0E-9 | 5.5E-1 | 1.0E0 | 4.5E0 | 4.7E0 | 1.0E-9 | 1.0E-9 | 6.5E1 | 2.9E1 | 230 | 41 | 230 | 41 | 0.52 |
| Me | pg/ml | 3.0E1 | 3.7E1 | 3.0E1 | 3.5E1 | 2.7E1 | 2.9E1 | 1.0E-9 | 1.0E-9 | 3.2E2 | 1.8E2 | 230 | 41 | 230 | 41 | 0.59 |
| Mf | pg/ml | 1.0E-9 | 1.0E-9 | 8.4E-1 | 3.9E-1 | 4.7E0 | 1.1E0 | 1.0E-9 | 1.0E-9 | 5.6E1 | 4.5E0 | 230 | 41 | 230 | 41 | 0.51 |
| Mg | pg/ml | 1.2E0 | 2.0E0 | 6.9E0 | 1.4E1 | 1.3E1 | 2.9E1 | 1.0E-9 | 1.0E-9 | 9.2E1 | 1.5E2 | 230 | 41 | 230 | 41 | 0.55 |
| Mh | pg/ml | 1.0E-9 | 1.0E-9 | 1.1E0 | 1.4E0 | 7.9E0 | 3.8E0 | 1.0E-9 | 1.0E-9 | 1.1E2 | 1.8E1 | 230 | 41 | 230 | 41 | 0.59 |
| Mi | pg/ml | 1.0E-9 | 1.0E-9 | 1.2E0 | 2.5E1 | 9.7E0 | 9.5E1 | 1.0E-9 | 1.0E-9 | 1.2E2 | 5.2E2 | 230 | 41 | 230 | 41 | 0.58 |
| Mj | pg/ml | 1.0E-9 | 1.0E-9 | 5.9E0 | 1.7E1 | 3.2E1 | 4.5E1 | 1.0E-9 | 1.0E-9 | 4.6E2 | 2.1E2 | 230 | 41 | 230 | 41 | 0.59 |
| Mk | pg/ml | 1.7E0 | 3.9E0 | 1.9E1 | 2.2E1 | 1.1E2 | 8.0E1 | 1.0E-9 | 1.0E-9 | 1.3E3 | 5.0E2 | 230 | 41 | 230 | 41 | 0.54 |
| Ml | pg/ml | 1.0E-9 | 1.0E-9 | 1.2E1 | 2.5E1 | 1.4E2 | 9.6E1 | 1.0E-9 | 1.0E-9 | 2.1E3 | 5.1E2 | 230 | 41 | 230 | 41 | 0.53 |
| Mm | pg/ml | 5.7E2 | 1.1E3 | 1.0E3 | 1.9E3 | 1.2E3 | 2.2E3 | 1.0E-9 | 5.2E1 | 6.4E3 | 1.0E4 | 230 | 41 | 230 | 41 | 0.63 |
| Mn | pg/ml | 5.9E0 | 1.1E1 | 1.2E1 | 1.4E1 | 2.9E1 | 9.7E0 | 1.0E-9 | 2.4E0 | 3.5E2 | 5.1E1 | 230 | 41 | 230 | 41 | 0.70 |
| Mp | pg/ml | 1.0E-9 | 8.8E0 | 1.3E1 | 9.9E1 | 4.7E1 | 3.8E2 | 1.0E-9 | 1.0E-9 | 6.3E2 | 2.4E3 | 230 | 41 | 230 | 41 | 0.63 |
| Mq | pg/ml | 1.0E-9 | 1.0E-9 | 2.4E0 | 7.4E0 | 1.4E1 | 2.1E1 | 1.0E-9 | 1.0E-9 | 1.8E2 | 1.0E2 | 230 | 41 | 230 | 41 | 0.55 |
| Mr | pg/ml | 1.0E-9 | 3.6E0 | 2.7E1 | 2.1E2 | 1.4E2 | 6.6E2 | 1.0E-9 | 1.0E-9 | 1.5E3 | 3.4E3 | 230 | 41 | 230 | 41 | 0.62 |
| Ms | pg/ml | 3.5E2 | 2.6E2 | 4.7E2 | 3.5E2 | 5.3E2 | 3.4E2 | 1.0E-9 | 1.0E-9 | 3.3E3 | 1.5E3 | 230 | 41 | 230 | 41 | 0.45 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Mt | pg/ml | 3.4E-1 | 1.6E0 | 9.4E0 | 1.1E2 | 5.2E1 | 5.0E2 | 1.0E-9 | 1.0E-9 | 7.1E2 | 3.2E3 | 230 | 41 | 230 | 41 | 0.67 |
| Mu | pg/ml | 1.0E-9 | 1.0E-9 | 2.0E0 | 3.2E0 | 1.7E1 | 9.7E0 | 1.0E-9 | 1.0E-9 | 2.3E2 | 4.8E1 | 230 | 41 | 230 | 41 | 0.56 |
| Mv | pg/ml | 1.0E-9 | 1.0E-9 | 8.2E1 | 1.2E2 | 4.0E2 | 2.6E2 | 1.0E-9 | 1.0E-9 | 4.5E3 | 9.2E2 | 230 | 41 | 230 | 41 | 0.58 |
| Mw | pg/ml | 3.0E1 | 1.2E2 | 3.2E2 | 6.2E2 | 1.6E3 | 1.3E3 | 1.0E-9 | 1.0E-9 | 1.8E4 | 5.3E3 | 230 | 41 | 230 | 41 | 0.73 |
| Mx | pg/ml | 1.0E-9 | 1.0E-9 | 3.9E-1 | 1.2E0 | 2.2E0 | 3.3E0 | 1.0E-9 | 1.0E-9 | 3.2E1 | 2.0E1 | 230 | 41 | 230 | 41 | 0.61 |
| My | pg/ml | 1.0E-9 | 7.5E0 | 4.5E2 | 1.7E2 | 3.0E3 | 4.3E2 | 1.0E-9 | 1.0E-9 | 3.9E4 | 1.9E3 | 230 | 41 | 230 | 41 | 0.58 |
| Mz | pg/ml | 1.1E1 | 3.0E1 | 2.4E1 | 1.3E2 | 5.2E1 | 3.5E2 | 1.0E-9 | 1.0E-9 | 5.5E2 | 1.9E3 | 230 | 41 | 230 | 41 | 0.75 |
| Na | pg/ml | 1.0E-9 | 1.0E-9 | 5.3E-1 | 1.7E0 | 1.7E0 | 6.7E0 | 1.0E-9 | 1.0E-9 | 9.6E0 | 4.2E1 | 230 | 41 | 230 | 41 | 0.53 |
| Nb | pg/ml | 2.1E0 | 3.5E0 | 3.7E0 | 1.4E1 | 1.2E1 | 3.6E1 | 1.0E-9 | 1.0E-9 | 1.5E2 | 2.1E2 | 230 | 41 | 230 | 41 | 0.64 |
| Nc | pg/ml | 3.4E2 | 5.3E1 | 5.3E2 | 3.8E2 | 7.8E2 | 6.2E2 | 1.0E-9 | 1.0E-9 | 9.2E3 | 3.2E3 | 230 | 41 | 230 | 41 | 0.39 |
| Nd | pg/ml | 2.3E1 | 3.5E1 | 2.7E1 | 8.5E1 | 8.2E1 | 3.3E2 | 1.0E-9 | 1.0E-9 | 1.2E3 | 2.1E3 | 230 | 41 | 230 | 41 | 0.64 |
| Ne | pg/ml | 4.2E2 | 4.0E2 | 5.2E2 | 5.0E2 | 5.9E2 | 6.0E2 | 1.0E-9 | 1.0E-9 | 7.0E3 | 3.6E3 | 230 | 41 | 230 | 41 | 0.46 |
| Nf | pg/ml | 1.0E-9 | 1.0E-9 | 2.4E0 | 7.3E0 | 9.4E0 | 2.7E1 | 1.0E-9 | 1.0E-9 | 8.2E1 | 1.3E2 | 230 | 41 | 230 | 41 | 0.51 |
| Ng | pg/ml | 1.7E1 | 4.8E1 | 1.1E2 | 9.2E1 | 2.1E2 | 1.3E2 | 1.0E-9 | 1.0E-9 | 1.6E3 | 5.3E2 | 230 | 41 | 230 | 41 | 0.53 |
| Nh | pg/ml | 5.9E1 | 5.5E1 | 8.1E1 | 7.0E1 | 7.6E1 | 8.4E1 | 1.0E-9 | 4.1E0 | 5.6E2 | 5.1E2 | 230 | 41 | 230 | 41 | 0.44 |
| Ni | pg/ml | 2.2E0 | 1.0E-9 | 8.5E1 | 1.1E2 | 1.3E2 | 2.2E2 | 1.0E-9 | 1.0E-9 | 7.1E2 | 1.1E3 | 230 | 41 | 230 | 41 | 0.46 |
| Nj | pg/ml | 6.1E0 | 6.7E0 | 9.9E0 | 9.3E0 | 1.1E1 | 9.6E0 | 1.0E-9 | 1.0E-9 | 6.8E1 | 4.6E1 | 230 | 41 | 230 | 41 | 0.50 |
| Nk | pg/ml | 1.8E1 | 1.2E1 | 3.1E1 | 3.6E1 | 3.6E1 | 5.4E1 | 1.0E-9 | 1.0E-9 | 2.0E2 | 2.0E2 | 230 | 41 | 230 | 41 | 0.49 |
| Nl | pg/ml | 4.2E1 | 3.9E1 | 5.6E1 | 4.7E1 | 8.2E1 | 5.1E1 | 1.0E-9 | 1.0E-9 | 1.1E3 | 2.3E2 | 230 | 41 | 230 | 41 | 0.45 |
| Ill | pg/ml | 1.7E1 | 1.1E1 | 3.8E1 | 5.3E2 | 6.0E1 | 1.4E3 | 1.0E-9 | 1.0E-9 | 3.0E2 | 3.6E3 | 42 | 7 | 42 | 7 | 0.53 |
| Ho | pg/ml | 1.8E1 | 3.3E1 | 2.4E1 | 1.1E2 | 1.9E1 | 1.4E2 | 1.1E0 | 7.6E0 | 8.7E1 | 3.9E2 | 42 | 7 | 42 | 7 | 0.64 |
| Hp | ng/ml | 1.6E0 | 6.9E0 | 8.8E1 | 3.8E2 | 2.6E2 | 4.7E2 | 1.0E-9 | 8.8E-1 | 8.9E2 | 8.9E2 | 42 | 7 | 42 | 7 | 0.70 |
| Tz | pg/ml | 4.1E3 | 7.6E3 | 7.5E3 | 1.9E5 | 1.7E4 | 5.7E5 | 1.0E-9 | 6.8E2 | 1.7E5 | 2.1E6 | 101 | 13 | 101 | 13 | 0.68 |
| Ua | pg/ml | 3.2E3 | 7.8E3 | 1.4E4 | 1.7E4 | 2.8E4 | 2.7E4 | 1.0E-9 | 1.4E3 | 1.4E5 | 9.9E4 | 101 | 13 | 101 | 13 | 0.65 |
| Ub | pg/ml | 5.3E2 | 3.2E2 | 8.2E2 | 7.3E2 | 1.2E3 | 1.1E3 | 1.0E-9 | 2.3E0 | 9.8E3 | 4.1E3 | 101 | 13 | 101 | 13 | 0.41 |
| Ue | pg/ml | 2.7E1 | 2.7E1 | 3.5E1 | 4.9E1 | 3.2E1 | 4.5E1 | 9.8E-2 | 1.1E1 | 2.7E2 | 1.4E2 | 101 | 13 | 101 | 13 | 0.55 |
| Uc | pg/ml | 7.7E2 | 1.4E3 | 1.4E3 | 6.1E3 | 1.8E3 | 1.5E4 | 1.0E-9 | 1.9E2 | 9.4E3 | 5.7E4 | 101 | 13 | 101 | 13 | 0.60 |
| Ud | pg/ml | 1.0E-9 | 1.0E-9 | 3.9E0 | 4.1E0 | 3.9E1 | 1.5E1 | 1.0E-9 | 1.0E-9 | 3.9E2 | 5.3E1 | 101 | 13 | 101 | 13 | 0.53 |
| Hq | pg/ml | 1.2E0 | 1.1E0 | 2.2E2 | 7.9E1 | 2.3E3 | 4.3E2 | 1.0E-9 | 1.0E-9 | 2.8E4 | 2.8E3 | 228 | 41 | 228 | 41 | 0.53 |
| Hr | pg/ml | 8.4E1 | 9.0E1 | 5.6E2 | 4.6E2 | 1.1E3 | 1.5E3 | 1.0E-9 | 1.0E-9 | 8.4E3 | 8.9E3 | 228 | 41 | 228 | 41 | 0.46 |
| Hu | pg/ml | 1.3E1 | 6.5E1 | 6.7E3 | 6.5E2 | 4.8E4 | 1.4E3 | 1.0E-9 | 1.0E-9 | 6.3E5 | 6.5E3 | 228 | 41 | 228 | 41 | 0.57 |
| Hv | pg/ml | 1.5E0 | 9.3E-1 | 2.6E0 | 2.9E1 | 5.5E0 | 1.4E2 | 1.0E-9 | 1.0E-9 | 6.9E1 | 8.9E2 | 228 | 41 | 228 | 41 | 0.48 |
| Hw | pg/ml | 5.5E0 | 6.5E0 | 1.6E1 | 2.6E2 | 5.3E1 | 1.5E3 | 1.0E-9 | 5.1E-1 | 6.4E2 | 9.4E3 | 228 | 41 | 228 | 41 | 0.51 |
| Hx | pg/ml | 8.9E0 | 1.2E1 | 6.7E1 | 7.8E1 | 6.1E2 | 2.3E2 | 1.0E-9 | 1.0E-9 | 9.3E3 | 1.3E3 | 228 | 41 | 228 | 41 | 0.57 |
| Ib | ng/ml | 3.6E-2 | 3.3E-2 | 1.2E0 | 4.5E0 | 5.1E0 | 1.5E1 | 1.0E-9 | 1.0E-9 | 3.6E1 | 5.6E1 | 98 | 13 | 98 | 13 | 0.50 |
| Ic | U/ml | 2.2E2 | 2.2E2 | 1.1E3 | 2.6E2 | 6.6E3 | 1.4E2 | 2.4E0 | 2.5E1 | 6.5E4 | 4.2E2 | 98 | 13 | 98 | 13 | 0.61 |
| Id | U/ml | 5.5E-1 | 1.3E0 | 1.3E0 | 3.5E1 | 2.0E0 | 1.2E2 | 1.0E-9 | 3.8E-1 | 1.3E1 | 4.3E2 | 98 | 13 | 98 | 13 | 0.71 |
| Tt | pg/ml | 1.7E2 | 1.7E2 | 1.8E2 | 2.0E2 | 5.4E1 | 8.7E1 | 4.3E1 | 1.1E2 | 3.6E2 | 4.4E2 | 91 | 12 | 91 | 12 | 0.53 |
| To | pg/ml | 1.6E0 | 1.8E0 | 2.0E0 | 2.5E0 | 2.1E0 | 3.2E0 | 1.0E-9 | 1.0E-9 | 9.9E0 | 1.2E1 | 96 | 13 | 96 | 13 | 0.53 |
| Tr | pg/ml | 3.3E0 | 6.9E0 | 9.5E0 | 1.3E1 | 3.2E1 | 2.1E1 | 1.0E-9 | 6.3E-1 | 3.1E1 | 7.6E1 | 94 | 12 | 94 | 12 | 0.62 |
| Tn | pg/ml | 3.3E1 | 5.5E1 | 1.1E2 | 2.9E2 | 3.1E2 | 6.2E2 | 2.4E0 | 1.9E1 | 2.0E3 | 2.3E3 | 96 | 13 | 96 | 13 | 0.70 |
| Tv | ng/ml | 1.2E1 | 1.0E1 | 2.9E1 | 5.8E2 | 8.7E1 | 2.0E3 | 1.0E-9 | 1.0E-9 | 7.9E2 | 7.1E3 | 96 | 13 | 96 | 13 | 0.46 |
| Ih | ng/ml | 6.3E1 | 3.3E2 | 2.0E2 | 6.6E2 | 3.3E2 | 8.1E2 | 1.0E-9 | 4.2E0 | 2.1E3 | 3.6E3 | 229 | 41 | 229 | 41 | 0.72 |
| Ii | ng/ml | 8.1E1 | 1.3E2 | 2.0E2 | 4.4E2 | 4.6E2 | 9.0E2 | 7.3E-1 | 2.3E0 | 5.2E3 | 4.5E3 | 229 | 41 | 229 | 41 | 0.63 |
| Ij | ng/ml | 7.8E1 | 1.7E2 | 1.8E2 | 9.0E2 | 6.3E2 | 3.8E3 | 2.8E0 | 4.3E1 | 6.4E3 | 2.4E4 | 227 | 40 | 227 | 40 | 0.76 |
| Ik | ng/ml | 1.2E1 | 1.4E1 | 1.8E3 | 1.8E3 | 1.4E4 | 3.6E2 | 5.9E-1 | 2.7E0 | 1.2E5 | 1.5E3 | 227 | 40 | 227 | 40 | 0.55 |
| Il | ng/ml | 3.6E2 | 5.7E2 | 1.2E3 | 2.5E3 | 2.6E3 | 4.0E3 | 1.0E-9 | 1.0E-9 | 1.2E4 | 1.2E4 | 225 | 40 | 225 | 40 | 0.61 |
| Im | ng/ml | 2.0E2 | 7.0E2 | 3.7E2 | 1.3E3 | 5.7E2 | 1.6E3 | 1.4E1 | 8.7E1 | 6.0E3 | 6.2E3 | 226 | 41 | 226 | 41 | 0.81 |
| In | ng/ml | 3.3E0 | 3.3E0 | 1.6E1 | 1.4E2 | 7.7E1 | 7.0E2 | 1.0E-9 | 1.0E-9 | 1.1E3 | 4.5E3 | 229 | 41 | 229 | 41 | 0.53 |
| Hb | ng/ml | 2.1E1 | 5.9E1 | 3.2E1 | 7.5E1 | 3.2E1 | 5.9E1 | 4.8E-1 | 1.6E1 | 2.0E2 | 1.9E2 | 102 | 13 | 102 | 13 | 0.77 |
| Hc | pg/ml | 7.0E2 | 5.5E2 | 3.4E3 | 1.8E3 | 1.2E4 | 3.7E3 | 1.0E-9 | 1.0E-9 | 1.0E5 | 1.4E4 | 102 | 13 | 102 | 13 | 0.46 |
| Hf | ng/ml | 2.0E2 | 3.3E2 | 4.3E2 | 3.5E2 | 6.0E2 | 3.2E2 | 1.0E-9 | 1.0E-9 | 3.2E3 | 9.9E2 | 102 | 13 | 102 | 13 | 0.50 |
| Io | ng/ml | 1.1E4 | 2.0E4 | 2.1E4 | 2.3E4 | 5.4E4 | 2.0E4 | 1.0E-9 | 6.2E2 | 7.1E5 | 9.9E4 | 229 | 41 | 229 | 41 | 0.63 |

| Code | Units | Median | | Average | | Standard Deviation | | Minimum | | Maximum | | Number of Samples | | Number of Patients | | AUC |
|------|-------|--------|----|---------|----|--------|----|---------|----|---------|----|--------|-----|---------|-----|-----|
| | | NonDis | Dis | NonDis | Dis | NonDis | Dis | NonDis | Dis | NonDis | Dis | NonDis | Dis | NonDis | Dis | |
| Ip | ng/ml | 1.2E1 | 3.0E1 | 2.1E1 | 3.3E1 | 2.7E1 | 2.2E1 | 1.0E-9 | 3.3E-1 | 2.3E2 | 7.8E1 | 229 | 41 | 229 | 41 | 0.69 |
| Iq | ug/ml | 1.1E-1 | 3.1E-1 | 7.0E-1 | 7.9E0 | 2.7E0 | 3.4E1 | 1.0E-9 | 1.0E-9 | 3.4E1 | 2.2E2 | 229 | 41 | 229 | 41 | 0.66 |
| Ir | ug/ml | 3.6E-1 | 1.7E0 | 2.0E0 | 2.2E1 | 1.1E1 | 6.4E1 | 1.0E-9 | 9.4E-2 | 1.6E2 | 3.7E2 | 228 | 41 | 228 | 41 | 0.74 |
| Is | ng/ml | 2.0E0 | 9.8E0 | 6.7E0 | 3.2E1 | 1.3E1 | 5.3E1 | 1.0E-9 | 3.3E-2 | 1.1E2 | 2.6E2 | 229 | 41 | 229 | 41 | 0.69 |
| It | ng/ml | 1.8E0 | 5.7E0 | 1.3E1 | 4.2E1 | 6.3E1 | 1.3E2 | 1.0E-9 | 1.0E-9 | 8.3E2 | 6.8E2 | 229 | 41 | 229 | 41 | 0.72 |
| Iu | ng/ml | 1.7E2 | 2.5E2 | 1.1E3 | 2.5E3 | 3.6E3 | 6.4E3 | 1.0E-9 | 1.0E-9 | 2.9E4 | 2.4E4 | 229 | 41 | 229 | 41 | 0.58 |
| Iv | ng/ml | 1.3E1 | 3.1E1 | 3.5E1 | 3.6E2 | 7.8E1 | 9.8E2 | 1.0E-9 | 1.0E-9 | 7.7E2 | 3.8E3 | 228 | 41 | 228 | 41 | 0.66 |
| Iz | ng/ml | 1.2E2 | 2.1E2 | 3.5E2 | 3.2E2 | 7.3E2 | 3.3E2 | 1.5E0 | 8.8E0 | 6.1E3 | 1.0E3 | 102 | 13 | 102 | 13 | 0.60 |
| Yd | ng/ml | 2.2E-1 | 7.6E-2 | 4.4E-1 | 1.9E-1 | 5.7E-1 | 2.0E-1 | 6.6E-3 | 1.7E-2 | 2.3E0 | 5.6E-1 | 43 | 7 | 43 | 7 | 0.38 |
| Wb | pg/ml | 2.7E4 | 3.5E4 | 3.4E4 | 1.4E5 | 1.9E4 | 2.3E5 | 6.3E3 | 1.4E4 | 8.4E4 | 6.4E5 | 43 | 7 | 43 | 7 | 0.67 |
| Vz | pg/ml | 3.8E0 | 5.0E0 | 4.8E0 | 6.0E0 | 4.3E0 | 7.5E0 | 1.0E-9 | 7.6E-2 | 2.1E1 | 2.2E1 | 43 | 7 | 43 | 7 | 0.52 |
| Si | ng/ml | 1.1E0 | 2.7E0 | 1.8E0 | 3.2E0 | 2.5E0 | 2.2E0 | 8.6E-3 | 5.6E-1 | 1.0E1 | 6.0E0 | 42 | 7 | 42 | 7 | 0.72 |
| Sf | mIU/mL | 2.0E1 | 2.0E1 | 5.5E1 | 2.8E1 | 1.2E2 | 2.7E1 | 7.8E-1 | 6.7E0 | 7.2E2 | 8.3E1 | 42 | 7 | 42 | 7 | 0.53 |
| Sh | mIU/mL | 1.7E1 | 1.0E1 | 5.0E1 | 1.0E1 | 1.0E2 | 5.5E0 | 7.8E-2 | 4.2E0 | 5.7E2 | 2.1E1 | 42 | 7 | 42 | 7 | 0.35 |
| Sj | ng/ml | 4.4E-1 | 4.3E-1 | 4.4E-1 | 4.1E-1 | 9.4E-2 | 6.1E-2 | 2.5E-1 | 3.4E-1 | 7.2E-1 | 4.8E-1 | 42 | 7 | 42 | 7 | 0.44 |
| Rc | pg/ml | 6.9E3 | 7.6E3 | 7.7E3 | 6.9E3 | 5.4E3 | 3.0E3 | 5.5E2 | 2.1E3 | 2.7E4 | 1.3E4 | 100 | 13 | 100 | 13 | 0.51 |
| Rb | pg/ml | 7.8E-1 | 2.4E0 | 2.6E0 | 6.4E0 | 4.1E0 | 1.5E1 | 1.0E-9 | 1.0E-9 | 2.0E1 | 5.6E1 | 100 | 13 | 100 | 13 | 0.56 |
| Zw | 2.5ng/ml | 7.2E0 | 4.7E0 | 9.7E0 | 1.9E1 | 1.1E1 | 2.9E1 | 2.4E-1 | 7.7E-1 | 5.9E1 | 6.3E1 | 43 | 7 | 43 | 7 | 0.49 |
| Zx | 2.3mU/ml | 1.3E-1 | 1.8E-1 | 3.3E-1 | 2.4E-1 | 5.8E-1 | 2.0E-1 | 3.2E-2 | 7.7E-2 | 2.9E0 | 6.7E-1 | 43 | 7 | 43 | 7 | 0.61 |
| Pz | ng/ml | 3.5E3 | 1.0E4 | 5.7E3 | 7.1E3 | 6.4E3 | 4.8E3 | 1.6E1 | 3.3E2 | 7.0E4 | 2.5E4 | 225 | 41 | 225 | 41 | 0.61 |
| Qa | ng/ml | 3.6E3 | 9.4E3 | 6.5E3 | 1.8E4 | 7.4E3 | 3.5E4 | 1.5E2 | 6.8E2 | 4.2E4 | 2.2E5 | 225 | 41 | 225 | 41 | 0.71 |
| Qb | ng/ml | 1.1E2 | 2.1E2 | 2.1E2 | 4.6E2 | 2.9E2 | 6.8E2 | 7.9E-1 | 1.8E1 | 2.8E3 | 4.1E3 | 225 | 41 | 225 | 41 | 0.69 |
| Qc | ng/ml | 2.0E2 | 6.5E2 | 4.1E2 | 8.2E2 | 5.1E2 | 8.3E2 | 1.0E0 | 1.0E-9 | 3.8E3 | 4.3E3 | 225 | 41 | 225 | 41 | 0.67 |
| Qd | ng/ml | 9.5E3 | 2.3E4 | 2.6E4 | 6.4E4 | 1.4E5 | 9.2E4 | 2.4E2 | 1.7E3 | 2.0E6 | 4.3E5 | 225 | 41 | 225 | 41 | 0.73 |
| Qe | ng/ml | 7.9E2 | 2.5E3 | 2.1E3 | 3.4E3 | 6.7E3 | 3.4E3 | 7.6E0 | 8.8E0 | 9.7E4 | 1.8E4 | 225 | 41 | 225 | 41 | 0.72 |
| Jd | ng/ml | 8.9E-1 | 2.7E0 | 4.1E0 | 5.3E0 | 1.6E1 | 6.7E0 | 1.0E-9 | 1.0E-9 | 1.2E2 | 2.1E1 | 100 | 13 | 100 | 13 | 0.67 |
| Je | ng/ml | 1.0E-9 | 1.0E-9 | 1.9E0 | 2.6E0 | 5.5E0 | 3.6E0 | 1.0E-9 | 1.0E-9 | 4.6E1 | 1.1E1 | 100 | 13 | 100 | 13 | 0.56 |
| Jf | ng/ml | 1.0E-9 | 1.0E-9 | 1.4E0 | 5.1E-1 | 2.4E0 | 1.1E0 | 1.0E-9 | 1.0E-9 | 1.2E1 | 3.7E0 | 100 | 13 | 100 | 13 | 0.40 |
| Jg | ng/ml | 5.2E2 | 1.2E3 | 8.0E2 | 1.5E3 | 9.9E2 | 1.4E3 | 5.8E0 | 5.4E1 | 1.0E4 | 7.1E3 | 228 | 41 | 228 | 41 | 0.69 |
| Jh | ng/ml | 2.9E0 | 9.4E0 | 2.4E1 | 4.8E1 | 9.8E1 | 9.6E1 | 1.0E-9 | 1.0E-9 | 1.2E3 | 4.9E2 | 228 | 41 | 228 | 41 | 0.65 |
| Ji | ng/ml | 5.8E1 | 1.4E2 | 8.0E1 | 2.3E2 | 8.0E1 | 2.4E2 | 1.1E0 | 2.3E1 | 5.3E2 | 1.3E3 | 228 | 41 | 228 | 41 | 0.75 |
| Sr | pg/mL | 3.8E2 | 2.4E3 | 8.2E2 | 3.6E3 | 1.2E3 | 5.5E3 | 1.0E-9 | 9.7E1 | 5.5E3 | 2.1E4 | 99 | 13 | 99 | 13 | 0.80 |
| Ss | pg/mL | 1.0E5 | 5.5E4 | 1.5E5 | 1.2E5 | 1.8E5 | 1.5E5 | 2.7E3 | 1.4E4 | 1.3E6 | 5.7E5 | 99 | 13 | 99 | 13 | 0.44 |
| St | pg/mL | 2.4E7 | 1.0E8 | 6.6E7 | 2.0E8 | 1.4E8 | 4.4E8 | 1.0E-9 | 3.4E6 | 1.2E9 | 1.7E9 | 98 | 13 | 98 | 13 | 0.75 |
| Wc | ng/ml | 1.0E-9 | 1.0E-9 | 4.4E-2 | 3.5E-1 | 7.5E-2 | 6.8E-1 | 1.0E-9 | 1.0E-9 | 3.0E-1 | 1.8E0 | 43 | 7 | 43 | 7 | 0.52 |
| Wd | ng/ml | 9.5E0 | 1.8E1 | 2.2E1 | 1.2E2 | 4.7E1 | 1.9E2 | 1.0E0 | 3.5E0 | 2.9E2 | 4.1E2 | 43 | 7 | 43 | 7 | 0.69 |
| Wc | ng/ml | 3.2E-1 | 4.9E-1 | 9.5E-1 | 3.8E0 | 1.3E0 | 8.4E0 | 1.0E-9 | 1.5E-1 | 5.5E0 | 2.3E1 | 43 | 7 | 43 | 7 | 0.61 |
| Wg | ng/ml | 1.0E-9 | 1.0E-9 | 1.0E-9 | 7.6E-2 | 0.0E0 | 2.0E-1 | 1.0E-9 | 1.0E-9 | 1.0E-9 | 5.3E-1 | 43 | 7 | 43 | 7 | 0.57 |
| Wh | ng/ml | 1.1E-2 | 2.0E-2 | 3.2E-2 | 7.3E-2 | 7.0E-2 | 1.2E-1 | 1.0E-9 | 1.0E-9 | 3.5E-1 | 3.4E-1 | 43 | 7 | 43 | 7 | 0.60 |
| Wf | ng/ml | 1.0E-9 | 1.0E-9 | 1.1E-1 | 3.3E-1 | 2.3E-1 | 8.6E-1 | 1.0E-9 | 1.0E-9 | 1.2E0 | 2.3E0 | 43 | 7 | 43 | 7 | 0.40 |
| Ra | pg/ml | 1.0E-9 | 1.0E-9 | 3.9E-1 | 5.7E0 | 9.6E-1 | 1.8E1 | 1.0E-9 | 1.0E-9 | 3.8E0 | 6.4E1 | 100 | 13 | 100 | 13 | 0.57 |
| Qz | pg/ml | 9.4E0 | 1.2E1 | 5.1E1 | 5.1E1 | 8.8E1 | 8.0E1 | 1.0E-9 | 1.0E-9 | 4.5E2 | 2.3E2 | 100 | 13 | 100 | 13 | 0.53 |
| Qy | pg/ml | 3.6E-1 | 7.1E-1 | 8.9E0 | 5.8E1 | 4.9E1 | 2.0E2 | 1.0E-9 | 1.0E-1 | 4.3E2 | 7.3E2 | 100 | 13 | 100 | 13 | 0.72 |
| Qx | pg/ml | 1.0E-9 | 1.0E-9 | 3.2E0 | 1.3E1 | 1.9E1 | 3.0E1 | 1.0E-9 | 1.0E-9 | 1.9E2 | 1.1E2 | 100 | 13 | 100 | 13 | 0.56 |
| Qw | pg/ml | 1.0E-9 | 6.8E-1 | 3.3E0 | 1.1E0 | 1.1E1 | 1.6E0 | 1.0E-9 | 1.0E-9 | 6.6E1 | 5.6E0 | 100 | 13 | 100 | 13 | 0.56 |
| Qv | pg/ml | 1.8E4 | 1.2E4 | 3.0E4 | 1.6E4 | 5.1E4 | 1.5E4 | 1.0E-9 | 8.5E2 | 3.7E5 | 5.0E4 | 100 | 13 | 100 | 13 | 0.40 |
| Qu | pg/ml | 7.0E0 | 3.7E1 | 9.6E1 | 1.2E2 | 1.9E2 | 2.0E2 | 1.0E-9 | 1.0E-9 | 9.8E2 | 7.3E2 | 100 | 13 | 100 | 13 | 0.56 |
| Qt | pg/ml | 1.5E1 | 1.4E1 | 4.8E1 | 4.8E1 | 1.0E2 | 8.0E1 | 1.0E-9 | 1.0E-9 | 7.1E2 | 2.2E2 | 100 | 13 | 100 | 13 | 0.49 |
| Qh | ng/ml | 1.8E1 | 3.8E1 | 4.7E1 | 1.1E2 | 7.6E1 | 2.1E2 | 4.3E-1 | 1.2E1 | 4.6E2 | 8.0E2 | 100 | 13 | 100 | 13 | 0.71 |
| Qg | ng/ml | 7.2E0 | 7.8E0 | 1.1E1 | 1.4E1 | 1.3E1 | 2.2E1 | 1.5E-1 | 3.3E-1 | 7.5E1 | 8.1E1 | 100 | 13 | 100 | 13 | 0.48 |
| Jj | ng/ml | 5.8E2 | 2.6E2 | 2.5E3 | 4.9E2 | 2.2E4 | 5.0E2 | 2.0E1 | 1.2E1 | 3.4E5 | 1.9E3 | 228 | 41 | 228 | 41 | 0.35 |
| Jk | ng/ml | 3.0E0 | 5.1E0 | 2.3E1 | 3.4E1 | 5.3E1 | 5.9E1 | 1.0E-9 | 3.8E-1 | 3.9E2 | 2.4E2 | 228 | 41 | 228 | 41 | 0.61 |
| Jl | ng/ml | 5.1E-1 | 1.0E0 | 2.2E0 | 2.5E2 | 5.1E0 | 1.6E3 | 1.2E-3 | 9.0E-2 | 4.0E1 | 9.9E3 | 228 | 41 | 228 | 41 | 0.63 |

| Code | Units | Median | | Average | | Standard Deviation | | Minimum | | Maximum | | Number of Samples | | Number of Patients | | AUC |
|------|-------|--------|--------|---------|--------|------|--------|---------|--------|---------|--------|--------|--------|--------|--------|------|
| | | NonDis | Dis | NonDis | Dis | NonDis | Dis | NonDis | Dis | NonDis | Dis | NonDis | Dis | NonDis | Dis | |
| Jm | ng/ml | 2.1E1 | 4.5E1 | 5.8E1 | 1.2E2 | 1.2E2 | 3.2E2 | 1.0E-9 | 9.2E-1 | 1.0E3 | 2.1E3 | 228 | 41 | 228 | 41 | 0.61 |
| Jn | pg/ml | 3.5E-1 | 8.4E-1 | 1.6E0 | 4.1E1 | 5.5E0 | 1.5E2 | 1.0E-9 | 1.0E-9 | 5.8E1 | 7.3E2 | 228 | 41 | 228 | 41 | 0.70 |
| Jo | pg/ml | 4.2E3 | 5.1E3 | 5.0E3 | 8.9E3 | 3.9E3 | 1.6E4 | 2.6E2 | 2.3E2 | 2.4E4 | 1.0E5 | 228 | 41 | 228 | 41 | 0.54 |
| Jp | pg/ml | 7.2E4 | 8.7E4 | 7.5E4 | 1.0E5 | 3.4E4 | 5.9E4 | 2.1E3 | 2.8E4 | 1.9E5 | 3.8E5 | 228 | 41 | 228 | 41 | 0.67 |
| Jq | pg/ml | 9.7E1 | 1.4E2 | 1.5E2 | 5.7E2 | 1.7E2 | 1.5E3 | 5.6E0 | 5.6E0 | 1.1E3 | 8.7E3 | 228 | 41 | 228 | 41 | 0.60 |
| Jr | pg/ml | 2.9E0 | 1.5E1 | 2.5E1 | 4.0E2 | 1.4E2 | 1.4E3 | 1.0E-9 | 1.0E-9 | 1.9E3 | 7.4E3 | 228 | 41 | 228 | 41 | 0.67 |
| Js | pg/ml | 1.5E1 | 2.7E1 | 4.1E1 | 2.8E2 | 1.4E2 | 7.7E2 | 1.0E-9 | 6.1E0 | 1.6E3 | 3.0E3 | 228 | 41 | 228 | 41 | 0.72 |
| Jt | pg/ml | 2.5E3 | 4.3E3 | 2.9E3 | 7.0E3 | 2.0E3 | 1.0E4 | 1.5E2 | 8.7E2 | 1.3E4 | 5.2E4 | 228 | 41 | 228 | 41 | 0.68 |
| Xa | pg/ml | 6.1E-1 | 2.4E1 | 8.1E0 | 2.7E2 | 1.7E1 | 4.7E2 | 1.0E-9 | 2.9E0 | 9.6E1 | 1.2E3 | 43 | 7 | 43 | 7 | 0.83 |
| Ye | pg/ml | 1.0E-9 | 1.0E-9 | 5.3E0 | 1.0E0 | 1.5E1 | 2.7E0 | 1.0E-9 | 1.0E-9 | 6.9E1 | 7.2E0 | 43 | 7 | 43 | 7 | 0.42 |
| Tm | pg/ml | 1.0E-9 | 1.0E-9 | 3.1E0 | 1.2E0 | 4.7E0 | 3.2E0 | 1.0E-9 | 1.0E-9 | 1.8E1 | 8.4E0 | 43 | 7 | 43 | 7 | 0.41 |
| Tl | pg/ml | 1.3E-1 | 1.0E-9 | 3.5E-1 | 3.6E0 | 4.1E-1 | 9.3E0 | 1.0E-9 | 1.0E-9 | 1.8E0 | 2.5E1 | 43 | 7 | 43 | 7 | 0.40 |
| Ju | mIU/ml | 1.2E1 | 1.2E1 | 2.7E1 | 1.9E1 | 3.8E1 | 1.7E1 | 1.7E-1 | 5.5E0 | 2.3E2 | 6.0E1 | 100 | 13 | 100 | 13 | 0.55 |
| Jv | mIU/ml | 1.8E1 | 1.3E1 | 4.4E1 | 2.5E1 | 6.8E1 | 2.7E1 | 1.7E-2 | 2.9E0 | 4.4E2 | 8.9E1 | 100 | 13 | 100 | 13 | 0.47 |
| Jy | ng/ml | 1.6E-3 | 2.6E-3 | 2.4E-3 | 5.8E-3 | 4.4E-3 | 1.1E-2 | 1.7E-4 | 5.3E-4 | 3.9E-2 | 4.1E-2 | 100 | 13 | 100 | 13 | 0.70 |
| Kc | pg/ml | 2.3E1 | 2.6E1 | 4.1E1 | 5.7E1 | 4.9E1 | 8.8E1 | 1.0E-9 | 1.0E-9 | 2.7E2 | 3.2E2 | 102 | 13 | 102 | 13 | 0.52 |
| Kd | pg/ml | 1.0E-9 | 1.0E-9 | 2.1E2 | 4.0E3 | 6.7E2 | 1.1E4 | 1.0E-9 | 1.0E-9 | 4.8E3 | 3.8E4 | 102 | 13 | 102 | 13 | 0.66 |
| Ke | pg/ml | 1.2E4 | 4.4E4 | 1.4E4 | 6.1E4 | 9.6E3 | 8.3E4 | 6.7E2 | 1.3E4 | 5.5E4 | 3.2E5 | 102 | 13 | 102 | 13 | 0.85 |
| Kf | pg/mL | 6.3E0 | 7.0E0 | 7.0E0 | 1.3E1 | 6.1E0 | 2.0E1 | 1.0E-9 | 1.0E-9 | 4.4E1 | 7.8E1 | 102 | 13 | 102 | 13 | 0.60 |
| Kg | pg/mL | 9.9E2 | 1.0E3 | 1.6E3 | 5.7E3 | 1.6E3 | 1.2E4 | 7.7E1 | 1.3E2 | 8.1E3 | 3.6E4 | 102 | 13 | 102 | 13 | 0.49 |
| Ki | pg/ml | 5.8E1 | 7.8E1 | 6.8E1 | 9.4E1 | 5.5E1 | 6.7E1 | 1.0E-9 | 1.3E1 | 2.9E2 | 2.5E2 | 102 | 13 | 102 | 13 | 0.64 |
| Kj | pg/ml | 8.8E2 | 7.3E2 | 1.4E3 | 2.4E3 | 1.4E3 | 4.2E3 | 6.6E1 | 3.3E1 | 8.8E3 | 1.5E4 | 102 | 13 | 102 | 13 | 0.47 |
| Kk | pg/ml | 6.8E0 | 1.0E1 | 1.3E1 | 2.2E1 | 1.5E1 | 2.2E1 | 1.0E-9 | 2.0E0 | 8.1E1 | 5.9E1 | 102 | 13 | 102 | 13 | 0.63 |
| Kl | pg/ml | 1.9E4 | 1.8E4 | 2.9E4 | 2.3E4 | 2.7E4 | 1.7E4 | 2.3E2 | 3.0E3 | 1.3E5 | 5.1E4 | 102 | 13 | 102 | 13 | 0.49 |
| Kn | pg/ml | 2.9E1 | 6.3E1 | 6.4E1 | 4.9E2 | 1.0E2 | 1.3E3 | 1.0E-9 | 1.0E-9 | 6.3E2 | 4.9E3 | 102 | 13 | 102 | 13 | 0.64 |
| Ko | pg/ml | 3.4E2 | 7.4E2 | 5.0E2 | 8.6E2 | 5.9E2 | 1.0E3 | 1.0E-9 | 1.5E2 | 3.8E3 | 4.1E3 | 102 | 13 | 102 | 13 | 0.68 |
| Kp | pg/ml | 3.6E2 | 3.6E2 | 3.7E2 | 1.4E3 | 2.5E2 | 3.6E3 | 1.0E-9 | 3.7E1 | 9.8E2 | 1.3E4 | 102 | 13 | 102 | 13 | 0.54 |
| Kq | pg/ml | 3.2E2 | 6.6E2 | 4.4E2 | 1.4E4 | 5.0E2 | 4.4E4 | 1.6E0 | 2.9E2 | 3.6E3 | 1.6E5 | 97 | 13 | 97 | 13 | 0.80 |
| Kr | pg/ml | 3.8E-1 | 2.9E0 | 2.1E0 | 3.4E1 | 3.9E0 | 1.1E2 | 1.0E-9 | 1.0E-9 | 2.3E1 | 4.2E2 | 97 | 13 | 97 | 13 | 0.64 |
| Ks | pg/ml | 1.4E4 | 2.2E4 | 2.1E4 | 2.4E4 | 1.9E4 | 1.7E4 | 5.1E1 | 9.9E2 | 7.9E4 | 5.0E4 | 97 | 13 | 97 | 13 | 0.57 |
| Ps | ng/ml | 1.4E2 | 1.2E3 | 5.5E2 | 2.3E3 | 1.9E3 | 2.6E3 | 5.5E0 | 3.5E2 | 1.2E4 | 7.6E3 | 42 | 7 | 42 | 7 | 0.90 |
| Kx | ng/ml | 1.0E-9 | 1.3E-2 | 7.7E-3 | 2.0E-2 | 1.6E-2 | 2.5E-2 | 1.0E-9 | 1.0E-9 | 1.0E-1 | 6.5E-2 | 101 | 13 | 101 | 13 | 0.67 |
| Ky | ng/ml | 1.5E-1 | 2.7E-1 | 4.2E-1 | 6.5E-1 | 8.2E-1 | 8.1E-1 | 1.0E-9 | 1.0E-9 | 5.1E0 | 2.7E0 | 101 | 13 | 101 | 13 | 0.63 |
| Kz | ng/ml | 1.0E-9 | 4.7E-3 | 3.4E-3 | 8.1E-3 | 5.5E-3 | 8.9E-3 | 1.0E-9 | 1.0E-9 | 1.4E-2 | 2.5E-2 | 101 | 13 | 101 | 13 | 0.63 |
| Rz | ng/ml | 3.3E-1 | 3.3E-1 | 6.6E-1 | 1.5E0 | 8.4E-1 | 2.7E0 | 4.6E-3 | 1.7E-2 | 3.4E0 | 7.5E0 | 42 | 7 | 42 | 7 | 0.55 |
| Ry | ng/ml | 1.6E-2 | 2.3E-2 | 2.4E-2 | 7.4E-2 | 2.7E-2 | 1.2E-1 | 1.0E-9 | 8.5E-3 | 1.2E-1 | 3.5E-1 | 42 | 7 | 42 | 7 | 0.64 |
| Rx | ng/ml | 1.0E-9 | 3.5E-5 | 1.5E-3 | 2.1E-3 | 2.4E-3 | 3.0E-3 | 1.0E-9 | 1.0E-9 | 8.6E-3 | 7.6E-3 | 42 | 7 | 42 | 7 | 0.63 |
| Ld | pg/ml | 1.0E-9 | 3.6E0 | 3.7E0 | 8.1E0 | 9.3E0 | 9.5E0 | 1.0E-9 | 1.0E-9 | 6.9E1 | 2.9E1 | 103 | 13 | 103 | 13 | 0.70 |
| Lh | pg/ml | 1.3E4 | 2.0E4 | 2.3E4 | 6.0E4 | 3.0E4 | 1.1E5 | 1.0E-9 | 1.3E3 | 2.6E5 | 4.8E5 | 229 | 41 | 229 | 41 | 0.64 |
| Li | pg/ml | 3.3E3 | 2.0E4 | 1.4E4 | 5.4E4 | 6.6E4 | 8.8E4 | 1.3E1 | 1.9E2 | 9.2E5 | 4.1E5 | 229 | 41 | 229 | 41 | 0.78 |
| Lj | pg/ml | 2.5E3 | 1.6E4 | 1.7E4 | 4.2E4 | 5.2E4 | 6.8E4 | 1.0E-9 | 1.8E2 | 4.3E5 | 3.9E5 | 229 | 41 | 229 | 41 | 0.73 |
| Lp | pg/ml | 1.2E1 | 1.1E0 | 4.5E1 | 4.5E2 | 1.1E2 | 6.0E2 | 1.0E-9 | 1.0E-9 | 5.8E2 | 1.4E3 | 42 | 7 | 42 | 7 | 0.52 |
| Lt | pg/ml | 1.0E-9 | 1.0E-9 | 2.0E0 | 1.0E-9 | 6.5E0 | 0.0E0 | 1.0E-9 | 1.0E-9 | 2.5E1 | 1.0E-9 | 42 | 7 | 42 | 7 | 0.45 |
| Rv | ng/ml | 5.0E-4 | 5.8E-4 | 1.2E-3 | 3.3E-3 | 2.5E-3 | 5.2E-3 | 1.0E-9 | 1.0E-9 | 1.6E-2 | 1.2E-2 | 42 | 7 | 42 | 7 | 0.49 |
| Ru | ng/ml | 1.0E-9 | 1.0E-9 | 1.2E-2 | 1.1E-1 | 5.7E-2 | 1.6E-1 | 1.0E-9 | 1.0E-9 | 3.1E-1 | 3.5E-1 | 42 | 7 | 42 | 7 | 0.69 |
| Rt | ng/ml | 8.0E-2 | 5.1E-2 | 1.2E-1 | 1.2E0 | 1.2E-1 | 2.8E0 | 2.2E-3 | 1.3E-3 | 5.8E-1 | 7.4E0 | 42 | 7 | 42 | 7 | 0.42 |
| Yl | pg/ml | 1.2E1 | 1.5E1 | 1.7E1 | 4.8E1 | 1.8E1 | 7.7E1 | 1.0E-9 | 1.0E-9 | 7.9E1 | 2.2E2 | 43 | 7 | 43 | 7 | 0.59 |
| Rm | ng/ml | 1.7E1 | 7.0E1 | 4.8E1 | 7.7E1 | 9.1E1 | 8.3E1 | 2.2E-1 | 4.4E0 | 6.5E2 | 2.5E2 | 99 | 13 | 99 | 13 | 0.67 |
| Rh | ng/ml | 1.7E2 | 1.6E2 | 3.0E2 | 1.5E3 | 5.0E2 | 4.7E3 | 7.5E0 | 2.5E1 | 3.8E3 | 1.7E4 | 99 | 13 | 99 | 13 | 0.45 |
| Ri | ng/ml | 4.4E-2 | 1.0E-9 | 3.4E0 | 1.6E0 | 6.9E0 | 4.3E0 | 1.0E-9 | 1.0E-9 | 4.5E1 | 1.6E1 | 99 | 13 | 99 | 13 | 0.36 |
| Rg | ng/ml | 1.0E-9 | 1.0E-9 | 1.1E-1 | 1.7E-2 | 4.9E-1 | 5.7E-2 | 1.0E-9 | 1.0E-9 | 3.3E0 | 2.1E-1 | 99 | 13 | 99 | 13 | 0.46 |
| Rj | ng/ml | 1.0E-9 | 1.0E-9 | 9.1E-1 | 2.1E1 | 2.1E0 | 7.5E1 | 1.0E-9 | 1.0E-9 | 1.1E1 | 2.7E2 | 99 | 13 | 99 | 13 | 0.53 |
| Rf | ng/ml | 3.4E-1 | 1.1E0 | 7.1E-1 | 3.3E0 | 1.2E0 | 5.4E0 | 2.1E-2 | 1.9E-1 | 9.9E0 | 1.7E1 | 99 | 13 | 99 | 13 | 0.77 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ql | pg/ml | 1.7E0 | 1.2E1 | 1.2E1 | 2.6E1 | 2.4E1 | 2.9E1 | 1.0E-9 | 1.0E-9 | 1.8E2 | 9.3E1 | 100 | 13 | 100 | 13 | 0.69 |
| Qm | pg/ml | 2.0E0 | 2.7E1 | 1.9E1 | 3.7E1 | 3.6E1 | 3.4E1 | 1.0E-9 | 1.0E-9 | 1.9E2 | 1.0E2 | 100 | 13 | 100 | 13 | 0.69 |
| Qn | pg/ml | 6.0E-1 | 1.1E0 | 5.9E0 | 2.8E0 | 2.4E1 | 4.2E0 | 1.0E-9 | 1.0E-9 | 2.2E2 | 1.2E1 | 100 | 13 | 100 | 13 | 0.61 |
| Nv | pg/ml | 3.8E3 | 7.7E3 | 1.0E4 | 1.9E4 | 2.1E4 | 2.9E4 | 1.0E-9 | 3.3E2 | 1.6E5 | 1.2E5 | 230 | 41 | 230 | 41 | 0.68 |
| Nw | pg/ml | 9.5E3 | 1.9E4 | 1.3E4 | 3.2E4 | 1.7E4 | 4.4E4 | 1.9E2 | 5.1E3 | 2.1E5 | 2.2E5 | 230 | 41 | 230 | 41 | 0.78 |
| Nx | pg/ml | 2.2E2 | 4.2E2 | 4.5E2 | 6.6E2 | 6.4E2 | 8.3E2 | 1.0E-9 | 1.0E-9 | 3.5E3 | 4.1E3 | 230 | 41 | 230 | 41 | 0.56 |
| Ny | pg/ml | 5.7E0 | 1.9E1 | 1.3E2 | 1.4E2 | 1.6E3 | 4.4E2 | 1.0E-9 | 1.0E-9 | 2.5E4 | 2.8E3 | 230 | 41 | 230 | 41 | 0.71 |
| Oa | pg/ml | 1.4E2 | 9.1E2 | 4.5E2 | 1.1E3 | 7.5E2 | 1.0E3 | 1.0E-9 | 4.9E0 | 4.5E3 | 3.4E3 | 100 | 13 | 100 | 13 | 0.72 |
| Op | pg/ml | 4.4E5 | 4.0E5 | 4.3E5 | 3.9E5 | 1.6E5 | 1.7E5 | 5.2E4 | 9.4E4 | 7.5E5 | 6.6E5 | 42 | 7 | 42 | 7 | 0.44 |
| Oe | pg/ml | 4.9E1 | 4.7E0 | 2.8E2 | 1.7E2 | 4.1E2 | 3.1E2 | 1.0E-9 | 1.0E-9 | 2.3E3 | 1.1E3 | 229 | 40 | 229 | 40 | 0.43 |
| Of | pg/ml | 2.0E2 | 9.7E1 | 6.8E3 | 3.5E3 | 2.5E4 | 1.1E4 | 1.0E-9 | 1.0E-9 | 1.9E5 | 6.6E4 | 230 | 41 | 230 | 41 | 0.45 |
| Og | pg/ml | 8.2E-2 | 1.7E-2 | 4.4E-1 | 7.8E-2 | 1.7E0 | 1.5E-1 | 1.0E-9 | 1.0E-9 | 1.9E1 | 8.0E-1 | 230 | 41 | 230 | 41 | 0.35 |
| Oh | pg/ml | 2.7E0 | 7.2E0 | 1.7E1 | 4.4E2 | 1.1E2 | 2.5E3 | 1.0E-9 | 1.0E-9 | 1.4E3 | 1.6E4 | 230 | 41 | 230 | 41 | 0.62 |
| Oi | pg/ml | 2.6E0 | 2.4E0 | 5.4E0 | 5.1E0 | 7.4E0 | 7.0E0 | 1.0E-9 | 1.0E-9 | 4.2E1 | 3.1E1 | 230 | 41 | 230 | 41 | 0.49 |
| Ok | pg/ml | 4.1E2 | 6.4E2 | 5.1E2 | 1.1E3 | 4.4E2 | 1.4E3 | 1.5E1 | 1.1E2 | 3.2E3 | 7.8E3 | 230 | 41 | 230 | 41 | 0.71 |
| Om | pg/ml | 4.2E2 | 7.2E2 | 8.9E2 | 2.4E3 | 2.4E3 | 8.0E3 | 1.0E-9 | 1.1E2 | 3.0E4 | 5.1E4 | 230 | 41 | 230 | 41 | 0.65 |
| On | pg/ml | 1.9E2 | 3.3E2 | 3.0E2 | 8.6E2 | 4.5E2 | 1.5E3 | 8.4E-1 | 2.7E1 | 4.5E3 | 8.5E3 | 230 | 41 | 230 | 41 | 0.71 |
| Or | pg/ml | 1.2E1 | 1.9E1 | 4.0E1 | 1.0E2 | 7.4E1 | 1.7E2 | 1.0E-9 | 1.0E-9 | 4.4E2 | 5.1E2 | 103 | 13 | 103 | 13 | 0.55 |
| Ow | pg/ml | 4.2E1 | 1.2E2 | 1.4E2 | 5.4E2 | 3.7E2 | 8.8E2 | 1.0E-9 | 1.0E-9 | 2.7E3 | 3.0E3 | 103 | 13 | 103 | 13 | 0.63 |
| Ou | pg/ml | 5.6E2 | 6.2E2 | 1.2E3 | 1.9E3 | 2.0E3 | 2.9E3 | 1.0E-9 | 1.0E-9 | 1.1E4 | 8.2E3 | 103 | 13 | 103 | 13 | 0.52 |
| Ul | ng/ml | 1.0E-9 | 1.0E-9 | 9.3E-1 | 5.9E0 | 3.9E0 | 1.6E1 | 1.0E-9 | 1.0E-9 | 2.3E1 | 5.6E1 | 101 | 13 | 101 | 13 | 0.54 |
| Uo | ng/ml | 1.0E-9 | 1.0E-9 | 7.7E-2 | 5.5E-2 | 2.1E-1 | 1.7E-1 | 1.0E-9 | 1.0E-9 | 1.3E0 | 6.3E-1 | 101 | 13 | 101 | 13 | 0.46 |
| Uk | ng/ml | 1.0E-9 | 1.0E-9 | 7.2E-3 | 3.4E-3 | 3.6E-2 | 8.9E-3 | 1.0E-9 | 1.0E-9 | 3.2E-1 | 3.1E-2 | 101 | 13 | 101 | 13 | 0.50 |
| Um | ng/ml | 1.0E-9 | 1.0E-9 | 2.4E-1 | 2.3E-1 | 6.2E-1 | 6.4E-1 | 1.0E-9 | 1.0E-9 | 3.6E0 | 2.3E0 | 101 | 13 | 101 | 13 | 0.45 |
| Uf | ng/ml | 6.1E-2 | 9.6E-2 | 1.4E-1 | 5.4E-1 | 2.0E-1 | 1.4E0 | 1.0E-3 | 2.1E-2 | 1.1E0 | 5.1E0 | 101 | 13 | 101 | 13 | 0.66 |
| Uh | ng/ml | 2.2E0 | 7.2E0 | 3.2E0 | 8.3E0 | 2.9E0 | 5.2E0 | 3.6E-2 | 7.1E-1 | 1.3E1 | 1.8E1 | 101 | 13 | 101 | 13 | 0.81 |
| Un | ng/ml | 1.7E0 | 3.8E0 | 1.9E0 | 4.9E0 | 1.2E0 | 6.4E0 | 3.4E-1 | 1.0E0 | 8.0E0 | 2.5E1 | 101 | 13 | 101 | 13 | 0.73 |
| Ug | ng/ml | 1.0E1 | 9.8E0 | 2.1E1 | 2.7E1 | 2.5E1 | 4.6E1 | 1.2E0 | 2.1E0 | 1.4E2 | 1.6E2 | 101 | 13 | 101 | 13 | 0.49 |
| Ur | ng/ml | 1.1E-1 | 1.0E-9 | 3.1E-1 | 1.1E0 | 5.4E-1 | 2.5E0 | 1.0E-9 | 1.0E-9 | 3.0E0 | 7.3E0 | 100 | 13 | 100 | 13 | 0.38 |
| Up | ng/ml | 1.0E-9 | 1.0E-9 | 3.1E-3 | 1.9E-1 | 9.7E-3 | 6.6E-1 | 1.0E-9 | 1.0E-9 | 5.3E-2 | 2.4E0 | 100 | 13 | 100 | 13 | 0.56 |
| Us | ng/ml | 4.1E-3 | 1.0E-9 | 1.8E-2 | 1.4E-1 | 4.7E-2 | 4.6E-1 | 1.0E-9 | 1.0E-9 | 3.6E-1 | 1.7E0 | 100 | 13 | 100 | 13 | 0.43 |
| Uv | ng/ml | 3.1E-3 | 1.6E-3 | 1.3E-2 | 3.5E-2 | 3.3E-2 | 1.1E-1 | 1.0E-9 | 1.0E-9 | 2.3E-1 | 4.1E-1 | 100 | 13 | 100 | 13 | 0.42 |
| Ut | ng/ml | 7.0E-1 | 1.9E0 | 2.7E0 | 1.0E1 | 6.4E0 | 1.8E1 | 1.0E-9 | 4.6E-1 | 5.2E1 | 6.5E1 | 100 | 13 | 100 | 13 | 0.75 |
| Uu | ng/ml | 7.2E0 | 5.2E0 | 7.7E0 | 5.8E0 | 5.1E0 | 2.9E0 | 5.7E-1 | 2.2E0 | 2.9E1 | 1.2E1 | 100 | 13 | 100 | 13 | 0.39 |
| Uw | ng/ml | 2.3E0 | 4.0E0 | 2.7E0 | 9.0E0 | 2.2E0 | 1.3E1 | 1.0E-9 | 1.7E0 | 7.9E0 | 3.9E1 | 43 | 7 | 43 | 7 | 0.73 |
| Vb | ng/ml | 1.1E0 | 1.1E0 | 1.1E0 | 1.7E0 | 4.2E-1 | 2.1E0 | 8.5E-2 | 2.6E-1 | 2.0E0 | 6.4E0 | 43 | 7 | 43 | 7 | 0.48 |
| Vc | ng/ml | 1.0E-9 | 1.0E-9 | 3.2E-3 | 1.0E-9 | 1.7E-2 | 0.0E0 | 1.0E-9 | 1.0E-9 | 1.1E-1 | 1.0E-9 | 43 | 7 | 43 | 7 | 0.47 |
| Uy | ng/ml | 1.3E0 | 1.0E0 | 5.8E0 | 2.2E1 | 1.7E1 | 2.7E1 | 8.7E-2 | 2.0E-2 | 9.9E1 | 6.4E1 | 43 | 7 | 43 | 7 | 0.55 |
| Uz | ng/ml | 1.0E-9 | 1.0E-9 | 1.0E-2 | 4.8E0 | 6.6E-2 | 1.2E1 | 1.0E-9 | 1.0E-9 | 4.4E-1 | 3.3E1 | 43 | 7 | 43 | 7 | 0.63 |
| Ux | ng/ml | 2.0E2 | 1.6E2 | 2.0E2 | 1.8E2 | 1.3E2 | 1.5E2 | 4.5E0 | 4.0E1 | 4.6E2 | 4.9E2 | 43 | 7 | 43 | 7 | 0.44 |
| Va | ng/ml | 1.6E1 | 3.2E0 | 2.7E1 | 3.2E0 | 3.0E1 | 1.5E0 | 3.1E-1 | 1.2E0 | 1.2E2 | 5.7E0 | 43 | 7 | 43 | 7 | 0.21 |
| Vh | ng/ml | 1.1E-2 | 2.7E-2 | 1.9E-2 | 1.4E-1 | 2.6E-2 | 3.2E-1 | 1.0E-3 | 3.5E-3 | 1.2E-1 | 8.6E-1 | 43 | 7 | 43 | 7 | 0.71 |
| Vi | ng/ml | 3.1E-3 | 4.3E-2 | 9.3E-3 | 3.0E-1 | 1.9E-2 | 6.7E-1 | 1.0E-9 | 1.6E-2 | 1.2E-1 | 1.8E0 | 43 | 7 | 43 | 7 | 0.92 |
| Vp | ng/ml | 1.0E-9 | 1.0E-9 | 1.1E-1 | 4.2E0 | 4.7E-1 | 1.4E1 | 1.0E-9 | 1.0E-9 | 3.9E0 | 4.9E1 | 101 | 13 | 101 | 13 | 0.55 |
| Vt | ng/ml | 6.1E0 | 1.2E1 | 8.0E0 | 2.6E1 | 6.7E0 | 4.0E1 | 5.6E-1 | 1.9E0 | 3.2E1 | 1.6E2 | 101 | 13 | 101 | 13 | 0.75 |
| Vu | ng/ml | 1.0E-9 | 4.5E-1 | 1.7E0 | 2.3E0 | 3.6E0 | 4.0E0 | 1.0E-9 | 1.0E-9 | 2.2E1 | 1.3E1 | 99 | 11 | 99 | 11 | 0.59 |
| Vq | ng/ml | 1.3E2 | 9.2E2 | 6.4E2 | 1.5E3 | 1.6E3 | 1.8E3 | 9.2E-1 | 1.8E1 | 1.2E4 | 4.9E3 | 81 | 9 | 81 | 9 | 0.72 |
| Vo | ng/ml | 2.5E1 | 2.6E1 | 2.5E1 | 2.4E1 | 5.8E0 | 3.9E0 | 4.9E0 | 1.7E1 | 4.8E1 | 3.0E1 | 101 | 13 | 101 | 13 | 0.45 |
| Vs | ng/ml | 1.0E-9 | 1.0E-9 | 4.3E0 | 4.3E1 | 1.1E1 | 1.3E2 | 1.0E-9 | 1.0E-9 | 8.9E1 | 4.5E2 | 99 | 12 | 99 | 12 | 0.46 |
| Vv | ng/ml | 3.2E0 | 3.1E0 | 6.0E0 | 7.4E0 | 1.0E1 | 1.2E1 | 1.0E-9 | 1.0E-9 | 8.4E1 | 4.4E1 | 101 | 12 | 101 | 12 | 0.49 |
| Vw | ng/ml | 3.6E1 | 5.7E1 | 3.3E1 | 4.7E1 | 1.7E1 | 2.4E1 | 2.5E0 | 1.1E1 | 6.7E1 | 6.9E1 | 43 | 7 | 43 | 7 | 0.71 |
| Oy | pg/ml | 5.1E-1 | 4.3E-1 | 7.9E0 | 3.3E0 | 3.7E1 | 7.3E0 | 1.0E-9 | 1.0E-9 | 4.0E2 | 3.3E1 | 229 | 41 | 229 | 41 | 0.45 |
| Oz | pg/ml | 3.9E-2 | 1.0E-9 | 3.9E-1 | 7.9E-1 | 1.9E0 | 4.4E0 | 1.0E-9 | 1.0E-9 | 2.9E1 | 2.8E1 | 229 | 41 | 229 | 41 | 0.36 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Pa | pg/ml | 4.1E-1 | 7.9E-1 | 1.4E0 | 1.0E1 | 6.6E0 | 3.8E1 | 1.0E-9 | 1.0E-9 | 8.6E1 | 2.3E2 | 229 | 41 | 229 | 41 | 0.63 |
| Pb | pg/ml | 1.0E-9 | 1.0E-9 | 2.4E0 | 8.8E-1 | 3.2E1 | 4.9E0 | 1.0E-9 | 1.0E-9 | 4.9E2 | 3.2E1 | 229 | 41 | 229 | 41 | 0.38 |
| Pc | pg/ml | 1.6E-1 | 1.0E-9 | 4.4E-1 | 9.2E0 | 1.0E0 | 5.2E1 | 1.0E-9 | 1.0E-9 | 1.4E1 | 3.3E2 | 229 | 41 | 229 | 41 | 0.42 |
| Pd | pg/ml | 1.7E0 | 2.9E0 | 7.6E0 | 1.0E1 | 5.6E1 | 2.1E1 | 1.0E-9 | 1.0E-9 | 8.4E2 | 1.2E2 | 229 | 41 | 229 | 41 | 0.59 |
| Pe | pg/ml | 2.1E1 | 6.9E1 | 1.2E2 | 8.5E2 | 5.2E2 | 2.6E3 | 1.0E-9 | 1.0E-9 | 4.7E3 | 1.5E4 | 229 | 41 | 229 | 41 | 0.74 |
| Pf | pg/ml | 1.7E0 | 7.8E0 | 1.5E1 | 3.0E1 | 1.0E2 | 7.0E1 | 1.0E-9 | 1.0E-9 | 1.5E3 | 4.3E2 | 229 | 41 | 229 | 41 | 0.74 |
| Pg | pg/ml | 4.0E0 | 1.6E1 | 9.0E1 | 1.9E2 | 6.2E2 | 4.6E2 | 1.0E-9 | 1.0E-9 | 7.7E3 | 2.2E3 | 229 | 41 | 229 | 41 | 0.70 |
| Ph | ng/ml | 1.4E-1 | 2.4E-1 | 3.5E-1 | 7.3E-1 | 5.3E-1 | 1.4E0 | 1.0E-9 | 1.0E-9 | 2.8E0 | 5.4E0 | 103 | 13 | 103 | 13 | 0.58 |
| Pi | ng/ml | 1.9E-1 | 3.5E-1 | 2.9E-1 | 6.9E0 | 4.0E-1 | 2.3E1 | 1.0E-9 | 2.1E-2 | 3.2E0 | 8.2E1 | 103 | 13 | 103 | 13 | 0.65 |
| Pj | ng/mL | 4.9E0 | 7.4E0 | 5.9E0 | 9.3E0 | 4.6E0 | 5.4E0 | 4.0E-1 | 4.3E0 | 3.1E1 | 2.3E1 | 103 | 13 | 103 | 13 | 0.71 |
| Pk | ng/ml | 9.0E-3 | 1.3E-2 | 1.4E-2 | 1.3E-1 | 2.9E-2 | 4.2E-1 | 1.0E-9 | 1.3E-4 | 2.5E-1 | 1.5E0 | 103 | 13 | 103 | 13 | 0.67 |
| aA | mg/dL | 8.8E-1 | 1.0E0 | 9.8E-1 | 1.4E0 | 4.7E-1 | 1.0E0 | 3.0E-1 | 5.0E-1 | 4.2E0 | 4.7E0 | 351 | 54 | 351 | 54 | 0.64 |
| aC | mg/mL | 2.1E0 | 2.4E0 | 2.4E0 | 2.6E0 | 1.2E0 | 1.3E0 | 7.5E-1 | 1.0E0 | 7.2E0 | 5.5E0 | 121 | 21 | 121 | 21 | 0.53 |
| aD | ug/mL | 3.1E0 | 2.4E0 | 4.6E0 | 4.5E0 | 4.1E0 | 4.3E0 | 7.5E-1 | 9.2E-1 | 3.1E1 | 1.8E1 | 121 | 21 | 121 | 21 | 0.45 |
| aE | mg/mL | 5.9E-1 | 5.1E-1 | 6.0E-1 | 5.4E-1 | 1.8E-1 | 1.5E-1 | 1.8E-1 | 2.2E-1 | 1.2E0 | 9.6E-1 | 121 | 21 | 121 | 21 | 0.39 |
| aF | ng/mL | 2.2E0 | 2.9E0 | 5.5E0 | 3.7E0 | 8.5E0 | 4.0E0 | 4.3E-3 | 4.3E-3 | 5.0E1 | 1.5E1 | 121 | 21 | 121 | 21 | 0.47 |
| aG | mg/mL | 1.4E-1 | 1.3E-1 | 1.6E-1 | 1.4E-1 | 8.4E-2 | 5.7E-2 | 5.0E-2 | 6.8E-2 | 4.8E-1 | 2.5E-1 | 121 | 21 | 121 | 21 | 0.46 |
| aH | ug/mL | 7.2E1 | 5.9E1 | 7.8E1 | 6.1E1 | 4.0E1 | 2.5E1 | 1.5E1 | 1.1E1 | 2.0E2 | 1.1E2 | 121 | 21 | 121 | 21 | 0.40 |
| aI | ug/mL | 1.7E2 | 1.4E2 | 1.7E2 | 1.6E2 | 6.1E1 | 5.3E1 | 4.7E1 | 7.6E1 | 3.4E2 | 2.7E2 | 121 | 21 | 121 | 21 | 0.41 |
| aJ | ug/mL | 2.4E0 | 3.0E0 | 3.1E0 | 4.0E0 | 2.2E0 | 2.7E0 | 8.2E-1 | 1.4E0 | 1.4E1 | 1.1E1 | 121 | 21 | 121 | 21 | 0.59 |
| aK | ng/mL | 1.3E0 | 1.4E0 | 2.0E0 | 1.7E0 | 1.9E0 | 1.5E0 | 2.9E-4 | 1.3E-1 | 1.0E1 | 6.5E0 | 121 | 21 | 121 | 21 | 0.47 |
| aL | mg/mL | 7.2E-1 | 7.4E-1 | 7.6E-1 | 7.0E-1 | 2.3E-1 | 2.3E-1 | 2.2E-1 | 2.7E-1 | 1.7E0 | 1.1E0 | 121 | 21 | 121 | 21 | 0.46 |
| aM | U/mL | 1.7E1 | 2.3E1 | 3.3E1 | 8.8E1 | 4.8E1 | 1.9E2 | 4.2E-2 | 4.2E-2 | 3.5E2 | 8.2E2 | 121 | 21 | 121 | 21 | 0.58 |
| aN | U/mL | 1.5E1 | 1.4E1 | 2.5E1 | 3.9E1 | 4.0E1 | 7.1E1 | 2.5E-3 | 2.5E-3 | 3.8E2 | 3.3E2 | 121 | 21 | 121 | 21 | 0.51 |
| aO | pg/mL | 6.2E1 | 7.2E1 | 4.5E2 | 4.3E2 | 1.1E3 | 7.4E2 | 6.0E-2 | 6.2E0 | 6.6E3 | 2.4E3 | 121 | 21 | 121 | 21 | 0.55 |
| aP | ng/mL | 1.6E0 | 1.9E0 | 2.0E0 | 2.5E0 | 1.3E0 | 1.7E0 | 5.4E-1 | 7.8E-1 | 6.6E0 | 6.1E0 | 121 | 21 | 121 | 21 | 0.58 |
| aQ | ng/mL | 2.4E-1 | 2.4E-1 | 3.5E-1 | 2.9E-1 | 3.3E-1 | 2.1E-1 | 2.0E-4 | 5.1E-2 | 2.0E0 | 9.0E-1 | 121 | 21 | 121 | 21 | 0.48 |
| aR | ng/mL | 1.9E0 | 1.4E0 | 3.1E0 | 3.4E0 | 4.2E0 | 3.8E0 | 2.6E-1 | 6.6E-1 | 3.4E1 | 1.7E1 | 121 | 21 | 121 | 21 | 0.52 |
| aS | ng/mL | 4.0E-1 | 4.0E-1 | 1.2E0 | 9.4E-1 | 3.1E0 | 1.2E0 | 4.2E-3 | 6.0E-2 | 3.3E1 | 4.9E0 | 121 | 21 | 121 | 21 | 0.49 |
| aU | pg/mL | 6.8E1 | 6.1E1 | 1.0E2 | 9.7E1 | 1.1E2 | 1.1E2 | 7.4E-2 | 7.4E-2 | 7.0E2 | 5.1E2 | 121 | 21 | 121 | 21 | 0.49 |
| aV | ng/mL | 5.9E-1 | 5.2E-1 | 1.1E0 | 7.5E-1 | 3.0E0 | 6.6E-1 | 7.6E-4 | 9.1E-2 | 3.3E1 | 2.4E0 | 121 | 21 | 121 | 21 | 0.48 |
| aW | pg/mL | 2.0E1 | 1.7E1 | 2.4E1 | 1.8E1 | 4.0E1 | 1.2E1 | 7.2E-2 | 7.2E-2 | 4.2E2 | 4.7E1 | 121 | 21 | 121 | 21 | 0.44 |
| aX | ng/mL | 8.0E0 | 3.3E0 | 1.1E1 | 1.4E1 | 1.0E1 | 2.4E1 | 3.0E-1 | 7.7E-1 | 5.4E1 | 1.1E2 | 121 | 21 | 121 | 21 | 0.42 |
| aY | pg/mL | 5.3E1 | 4.6E1 | 8.0E1 | 5.3E1 | 1.2E2 | 4.3E1 | 4.1E-1 | 4.1E-1 | 1.2E3 | 2.0E2 | 121 | 21 | 121 | 21 | 0.42 |
| aZ | pg/mL | 2.2E2 | 3.0E2 | 5.7E2 | 9.9E2 | 1.3E3 | 1.3E3 | 1.7E0 | 1.5E1 | 1.2E4 | 4.7E3 | 121 | 21 | 121 | 21 | 0.62 |
| bA | ng/mL | 1.4E1 | 3.8E1 | 5.7E1 | 1.5E2 | 1.1E2 | 2.6E2 | 3.0E-2 | 2.0E0 | 9.4E2 | 9.4E2 | 121 | 21 | 121 | 21 | 0.61 |
| bB | ng/mL | 2.7E2 | 2.7E2 | 2.9E2 | 2.6E2 | 1.6E2 | 1.6E2 | 1.2E1 | 3.3E1 | 8.1E2 | 5.7E2 | 121 | 21 | 121 | 21 | 0.44 |
| bC | ng/mL | 3.2E2 | 2.9E2 | 5.8E2 | 7.1E2 | 7.4E2 | 1.1E3 | 2.7E1 | 5.0E1 | 4.7E3 | 4.0E3 | 121 | 21 | 121 | 21 | 0.49 |
| bE | mg/mL | 5.0E0 | 5.1E0 | 5.2E0 | 5.4E0 | 1.8E0 | 2.3E0 | 1.8E0 | 1.3E0 | 1.2E1 | 1.1E1 | 121 | 21 | 121 | 21 | 0.51 |
| bF | pg/mL | 3.5E1 | 5.4E1 | 4.3E2 | 2.9E2 | 1.6E3 | 5.9E2 | 5.0E-2 | 8.9E0 | 1.1E4 | 2.2E3 | 121 | 21 | 121 | 21 | 0.61 |
| bG | ng/mL | 1.7E0 | 2.3E0 | 3.4E0 | 3.6E0 | 4.9E0 | 4.0E0 | 1.1E-1 | 2.4E-1 | 3.0E1 | 1.5E1 | 121 | 21 | 121 | 21 | 0.54 |
| bH | pg/mL | 5.7E-1 | 5.7E-1 | 7.2E0 | 5.5E0 | 2.8E1 | 6.7E0 | 5.7E-1 | 5.7E-1 | 2.8E2 | 2.3E1 | 121 | 21 | 121 | 21 | 0.57 |
| bI | ng/mL | 4.0E-3 | 4.0E-3 | 1.0E-1 | 7.5E-2 | 2.1E-1 | 1.8E-1 | 4.0E-3 | 4.0E-3 | 9.8E-1 | 6.2E-1 | 121 | 21 | 121 | 21 | 0.46 |
| bJ | mg/mL | 1.7E0 | 1.9E0 | 2.1E0 | 2.2E0 | 1.8E0 | 2.1E0 | 2.5E-4 | 2.5E-4 | 1.1E1 | 8.9E0 | 121 | 21 | 121 | 21 | 0.51 |
| bL | pg/mL | 4.3E0 | 3.2E0 | 9.1E0 | 7.9E0 | 1.1E1 | 8.4E0 | 4.6E-2 | 4.6E-2 | 4.9E1 | 3.0E1 | 121 | 21 | 121 | 21 | 0.50 |
| bM | mg/mL | 1.9E0 | 1.7E0 | 2.3E0 | 1.8E0 | 1.5E0 | 1.2E0 | 2.4E-1 | 1.8E-2 | 8.6E0 | 5.2E0 | 121 | 21 | 121 | 21 | 0.42 |
| bN | ng/mL | 3.9E1 | 2.1E1 | 1.3E2 | 4.4E1 | 3.0E2 | 5.3E1 | 1.4E-1 | 5.9E-1 | 1.9E3 | 1.9E2 | 121 | 21 | 121 | 21 | 0.36 |
| bO | ng/mL | 4.0E-2 | 4.0E-2 | 7.3E0 | 1.5E1 | 1.5E1 | 3.3E1 | 4.0E-2 | 4.0E-2 | 7.1E1 | 1.3E2 | 121 | 21 | 121 | 21 | 0.48 |
| bP | mg/mL | 4.9E-1 | 5.2E-1 | 7.3E-1 | 5.5E-1 | 7.1E-1 | 3.1E-1 | 8.2E-2 | 9.2E-2 | 4.8E0 | 1.2E0 | 121 | 21 | 121 | 21 | 0.47 |
| bQ | pg/mL | 2.1E1 | 5.9E1 | 1.7E2 | 6.6E1 | 1.2E3 | 5.8E1 | 1.5E-1 | 1.5E-1 | 1.3E4 | 2.2E2 | 121 | 21 | 121 | 21 | 0.69 |
| bR | ng/mL | 1.2E-2 | 1.2E-2 | 1.7E-1 | 1.0E-1 | 8.4E-1 | 1.3E-1 | 1.2E-2 | 1.2E-2 | 8.7E0 | 3.9E-1 | 121 | 21 | 121 | 21 | 0.55 |
| bS | pg/mL | 9.4E-1 | 9.4E-1 | 1.0E1 | 6.9E0 | 4.8E1 | 1.7E1 | 9.4E-1 | 9.4E-1 | 3.9E2 | 7.2E1 | 121 | 21 | 121 | 21 | 0.52 |
| bU | ng/mL | 5.5E-2 | 1.6E-1 | 2.0E-1 | 1.7E-1 | 6.3E-1 | 1.8E-1 | 1.3E-2 | 1.3E-2 | 6.6E0 | 6.5E-1 | 121 | 21 | 121 | 21 | 0.56 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bV | pg/mL | 4.7E2 | 5.3E2 | 5.5E2 | 7.2E2 | 2.7E2 | 5.5E2 | 1.8E2 | 2.7E2 | 1.6E3 | 2.2E3 | 121 | 21 | 121 | 21 | 0.56 |
| bW | pg/mL | 3.3E2 | 3.1E2 | 4.8E2 | 5.2E2 | 5.7E2 | 8.0E2 | 8.4E1 | 1.5E2 | 4.8E3 | 3.9E3 | 121 | 21 | 121 | 21 | 0.50 |
| bX | ng/mL | 2.5E-5 | 3.1E-3 | 2.4E-3 | 2.9E-3 | 2.9E-3 | 2.9E-3 | 2.5E-5 | 2.5E-5 | 1.2E-2 | 7.8E-3 | 121 | 21 | 121 | 21 | 0.54 |
| bZ | pg/mL | 2.9E2 | 3.6E2 | 2.2E3 | 1.3E3 | 8.1E3 | 2.0E3 | 1.5E-1 | 1.0E2 | 5.8E4 | 7.4E3 | 121 | 21 | 121 | 21 | 0.58 |
| cA | pg/mL | 6.0E-1 | 6.0E-1 | 4.7E0 | 2.7E0 | 3.4E1 | 5.6E0 | 6.0E-1 | 6.0E-1 | 3.7E2 | 2.1E1 | 121 | 21 | 121 | 21 | 0.52 |
| cB | ng/mL | 3.7E-2 | 5.1E-2 | 6.0E-2 | 1.0E-1 | 6.9E-2 | 1.3E-1 | 1.7E-3 | 1.7E-3 | 3.7E-1 | 4.3E-1 | 121 | 21 | 121 | 21 | 0.55 |
| cC | pg/mL | 4.1E1 | 4.6E1 | 4.7E1 | 4.3E1 | 5.6E1 | 3.2E1 | 1.0E0 | 1.0E0 | 4.5E2 | 1.1E2 | 121 | 21 | 121 | 21 | 0.52 |
| cD | pg/mL | 4.9E0 | 5.9E0 | 1.6E1 | 6.7E0 | 5.5E1 | 6.9E0 | 3.3E-1 | 3.3E-1 | 4.8E2 | 3.3E1 | 121 | 21 | 121 | 21 | 0.53 |
| cE | pg/mL | 6.5E1 | 1.3E2 | 2.9E2 | 2.1E2 | 6.8E2 | 2.5E2 | 1.2E-1 | 1.4E1 | 3.8E3 | 1.1E3 | 121 | 21 | 121 | 21 | 0.63 |
| cF | pg/mL | 5.3E-1 | 5.3E-1 | 1.6E1 | 1.3E1 | 3.3E1 | 2.0E1 | 5.3E-1 | 5.3E-1 | 2.7E2 | 6.5E1 | 121 | 21 | 121 | 21 | 0.48 |
| cG | pg/mL | 5.3E1 | 9.0E1 | 1.9E2 | 1.3E2 | 9.6E2 | 1.1E2 | 7.8E0 | 2.4E1 | 1.0E4 | 4.1E2 | 121 | 21 | 121 | 21 | 0.63 |
| cH | uIU/mL | 3.4E0 | 2.5E0 | 8.6E0 | 4.8E0 | 1.9E1 | 7.6E0 | 8.6E-3 | 8.6E-3 | 1.6E2 | 3.4E1 | 121 | 21 | 121 | 21 | 0.40 |
| cI | ng/mL | 5.9E0 | 6.9E0 | 1.3E1 | 1.6E1 | 2.0E1 | 2.7E1 | 3.2E-2 | 5.1E-1 | 1.2E2 | 1.2E2 | 121 | 21 | 121 | 21 | 0.51 |
| cJ | ug/mL | 5.9E1 | 6.2E1 | 9.6E1 | 8.1E1 | 1.1E2 | 7.9E1 | 6.9E0 | 7.2E0 | 6.4E2 | 3.4E2 | 121 | 21 | 121 | 21 | 0.48 |
| cK | ng/mL | 3.8E-3 | 3.8E-3 | 2.2E-2 | 1.9E-2 | 1.3E-1 | 4.7E-2 | 3.8E-3 | 3.8E-3 | 1.5E0 | 2.1E-1 | 121 | 21 | 121 | 21 | 0.53 |
| cL | pg/mL | 2.2E2 | 2.0E2 | 6.3E2 | 3.2E2 | 2.4E3 | 2.8E2 | 3.1E1 | 1.1E2 | 2.4E4 | 1.3E3 | 121 | 21 | 121 | 21 | 0.58 |
| cM | pg/mL | 2.6E2 | 2.8E2 | 2.9E2 | 2.8E2 | 1.8E2 | 1.0E2 | 4.2E1 | 5.7E1 | 1.1E3 | 4.5E2 | 121 | 21 | 121 | 21 | 0.54 |
| cN | pg/mL | 1.2E2 | 1.2E2 | 1.4E2 | 1.2E2 | 9.9E1 | 3.2E1 | 3.8E1 | 7.9E1 | 1.1E3 | 2.0E2 | 121 | 21 | 121 | 21 | 0.50 |
| cO | pg/mL | 2.1E2 | 2.4E2 | 4.5E2 | 3.4E2 | 1.8E3 | 3.1E2 | 5.4E1 | 1.3E2 | 1.9E4 | 1.5E3 | 121 | 21 | 121 | 21 | 0.59 |
| cP | ng/mL | 2.5E3 | 2.3E3 | 2.6E3 | 2.5E3 | 9.8E2 | 1.0E3 | 6.2E2 | 1.0E3 | 5.6E3 | 4.7E3 | 121 | 21 | 121 | 21 | 0.48 |
| cQ | ng/mL | 4.9E-2 | 6.7E-2 | 1.2E-1 | 1.6E-1 | 2.1E-1 | 2.5E-1 | 2.0E-3 | 2.0E-3 | 1.3E0 | 8.7E-1 | 121 | 21 | 121 | 21 | 0.51 |
| cR | ng/mL | 2.9E2 | 4.2E2 | 5.6E2 | 6.3E2 | 9.0E2 | 6.1E2 | 2.0E1 | 8.6E1 | 7.7E3 | 2.2E3 | 121 | 21 | 121 | 21 | 0.58 |
| cS | ng/mL | 2.6E2 | 2.5E2 | 3.8E2 | 5.1E2 | 3.9E2 | 5.6E2 | 8.0E1 | 9.7E1 | 2.5E3 | 2.4E3 | 121 | 21 | 121 | 21 | 0.54 |
| cT | ng/mL | 5.3E1 | 9.4E1 | 1.5E2 | 3.0E2 | 2.7E2 | 4.8E2 | 4.2E0 | 1.1E1 | 2.1E3 | 1.5E3 | 121 | 21 | 121 | 21 | 0.57 |
| cU | ng/mL | 5.9E1 | 8.0E1 | 1.0E2 | 1.1E2 | 1.7E2 | 8.4E1 | 6.2E0 | 1.7E1 | 1.6E3 | 3.3E2 | 121 | 21 | 121 | 21 | 0.59 |
| cV | ng/mL | 2.2E-1 | 2.1E-1 | 9.5E-1 | 2.6E-1 | 4.4E0 | 2.0E-1 | 3.4E-2 | 6.8E-2 | 4.7E1 | 9.3E-1 | 121 | 21 | 121 | 21 | 0.48 |
| cW | mIU/mL | 4.8E-2 | 4.2E-2 | 9.4E-2 | 7.5E-2 | 4.1E-1 | 8.2E-2 | 4.8E-3 | 1.4E-2 | 4.5E0 | 3.9E-1 | 121 | 21 | 121 | 21 | 0.53 |
| cX | ng/mL | 1.7E-1 | 6.9E-2 | 2.5E0 | 3.4E-1 | 6.6E0 | 6.1E-1 | 2.3E-4 | 2.3E-4 | 2.8E1 | 2.5E0 | 121 | 21 | 121 | 21 | 0.46 |
| cY | ng/mL | 7.4E0 | 8.9E0 | 1.1E1 | 9.8E0 | 1.1E1 | 8.4E0 | 1.7E-1 | 6.0E-1 | 6.1E1 | 3.6E1 | 121 | 21 | 121 | 21 | 0.50 |
| cZ | ug/mL | 1.2E1 | 1.2E1 | 1.4E1 | 1.4E1 | 5.7E0 | 7.5E0 | 2.8E0 | 3.3E0 | 3.0E1 | 3.0E1 | 121 | 21 | 121 | 21 | 0.47 |
| dA | pg/mL | 3.1E2 | 3.6E2 | 3.8E2 | 3.9E2 | 5.2E2 | 2.2E2 | 1.0E2 | 1.1E2 | 5.8E3 | 8.8E2 | 121 | 21 | 121 | 21 | 0.54 |
| dB | ug/mL | 1.9E1 | 1.5E1 | 1.9E1 | 1.6E1 | 2.3E1 | 8.9E0 | 2.1E0 | 2.2E0 | 2.5E2 | 2.8E1 | 121 | 21 | 121 | 21 | 0.43 |
| dC | nmol/L | 3.4E1 | 3.6E1 | 3.8E1 | 3.6E1 | 1.8E1 | 1.4E1 | 7.8E0 | 1.5E1 | 1.4E2 | 6.5E1 | 121 | 21 | 121 | 21 | 0.49 |
| dD | ug/mL | 3.3E1 | 2.9E1 | 3.4E1 | 3.2E1 | 1.0E1 | 1.1E1 | 1.4E1 | 1.6E1 | 7.4E1 | 6.0E1 | 121 | 21 | 121 | 21 | 0.41 |
| dE | ng/mL | 4.1E-1 | 7.0E-1 | 5.1E-1 | 6.9E-1 | 5.4E-1 | 6.4E-1 | 8.4E-3 | 8.4E-3 | 2.7E0 | 2.4E0 | 121 | 21 | 121 | 21 | 0.59 |
| dF | ng/mL | 2.5E2 | 3.7E2 | 3.4E2 | 4.6E2 | 2.5E2 | 3.1E2 | 7.5E1 | 1.1E2 | 1.3E3 | 1.2E3 | 121 | 21 | 121 | 21 | 0.63 |
| dG | ng/mL | 1.2E1 | 1.4E1 | 1.7E1 | 1.8E1 | 2.0E1 | 1.7E1 | 3.0E0 | 4.8E0 | 1.8E2 | 8.7E1 | 121 | 21 | 121 | 21 | 0.55 |
| dH | pg/mL | 7.7E0 | 1.1E1 | 2.2E1 | 1.0E1 | 6.9E1 | 5.1E0 | 4.0E-2 | 4.0E-2 | 6.7E2 | 2.3E1 | 121 | 21 | 121 | 21 | 0.57 |
| dI | pg/mL | 4.6E-1 | 4.6E-1 | 4.3E0 | 1.7E0 | 3.0E1 | 2.4E0 | 4.6E-1 | 4.6E-1 | 3.3E2 | 8.4E0 | 121 | 21 | 121 | 21 | 0.53 |
| dJ | ng/mL | 1.9E0 | 2.2E0 | 2.1E0 | 2.1E0 | 1.1E0 | 1.1E0 | 3.2E-2 | 3.2E-2 | 5.1E0 | 4.0E0 | 121 | 21 | 121 | 21 | 0.51 |
| dK | uIU/mL | 1.5E0 | 8.6E-1 | 2.3E0 | 2.0E0 | 4.0E0 | 2.8E0 | 2.8E-4 | 4.7E-2 | 3.9E1 | 1.1E1 | 121 | 21 | 121 | 21 | 0.39 |
| dL | ng/mL | 8.8E2 | 8.2E2 | 1.0E3 | 1.1E3 | 5.9E2 | 9.0E2 | 2.8E2 | 4.3E2 | 3.8E3 | 4.8E3 | 121 | 21 | 121 | 21 | 0.50 |
| dM | pg/mL | 9.5E2 | 1.1E3 | 1.3E3 | 1.8E3 | 1.6E3 | 1.6E3 | 3.7E2 | 6.3E2 | 1.5E4 | 5.8E3 | 121 | 21 | 121 | 21 | 0.62 |
| dN | ug/mL | 9.8E1 | 1.1E2 | 1.0E2 | 1.2E2 | 3.7E1 | 6.3E1 | 2.4E1 | 4.7E1 | 2.2E2 | 3.3E2 | 121 | 21 | 121 | 21 | 0.54 |
| dR | pg/ml | 1.3E3 | 9.3E2 | 2.1E3 | 1.2E3 | 2.2E3 | 1.3E3 | 1.4E2 | 1.3E2 | 9.8E3 | 5.3E3 | 97 | 15 | 97 | 15 | 0.34 |
| dX | ng/ml | 5.2E-2 | 8.2E-2 | 1.3E-1 | 1.5E-1 | 2.2E-1 | 1.5E-1 | 2.6E-3 | 2.6E-3 | 1.2E0 | 4.2E-1 | 38 | 7 | 38 | 7 | 0.60 |
| eF | ng/ml | 4.1E0 | 4.3E0 | 4.8E0 | 5.2E0 | 2.2E0 | 3.2E0 | 2.0E0 | 2.0E0 | 1.2E1 | 1.5E1 | 97 | 15 | 97 | 15 | 0.51 |
| eC | pg/ml | 3.0E2 | 2.7E2 | 3.7E2 | 3.9E2 | 2.6E2 | 5.6E2 | 7.1E1 | 1.9E1 | 1.6E3 | 2.0E3 | 90 | 11 | 90 | 11 | 0.38 |
| eD | pg/ml | 2.1E2 | 2.4E2 | 7.2E2 | 2.6E2 | 1.5E3 | 1.5E2 | 5.2E-1 | 5.9E1 | 7.0E3 | 4.9E2 | 74 | 8 | 74 | 8 | 0.53 |
| eM | ng/ml | 3.0E0 | 2.6E0 | 4.4E0 | 4.9E0 | 4.6E0 | 6.5E0 | 6.9E-1 | 8.8E-1 | 2.6E1 | 2.3E1 | 52 | 10 | 52 | 10 | 0.49 |
| eP | ng/ml | 3.7E-3 | 3.7E-3 | 1.4E0 | 1.6E-1 | 4.6E0 | 4.0E-1 | 3.7E-3 | 3.7E-3 | 2.8E1 | 1.1E0 | 38 | 7 | 38 | 7 | 0.39 |
| fP | ng/ml | 2.8E2 | 2.6E2 | 3.4E2 | 3.1E2 | 2.0E2 | 1.6E2 | 1.8E0 | 9.5E1 | 1.0E3 | 6.0E2 | 96 | 13 | 96 | 13 | 0.46 |
| fR | ng/ml | 1.5E5 | 2.1E5 | 2.0E5 | 2.7E5 | 1.6E5 | 2.0E5 | 3.6E4 | 1.9E2 | 6.3E5 | 6.8E5 | 75 | 16 | 75 | 16 | 0.59 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| gC | ng/ml | 2.2E2 | 2.8E2 | 2.5E2 | 2.8E2 | 1.1E2 | 1.2E2 | 8.3E1 | 1.5E2 | 6.4E2 | 4.8E2 | 31 | 7 | 31 | 7 | 0.58 |
| gL | pg/ml | 6.3E4 | 7.7E4 | 7.0E4 | 8.7E4 | 3.1E4 | 4.0E4 | 1.1E4 | 4.4E4 | 1.6E5 | 1.7E5 | 97 | 15 | 97 | 15 | 0.64 |
| gP | U/ml | 2.8E2 | 2.7E2 | 2.9E2 | 2.7E2 | 1.3E2 | 7.8E1 | 1.2E1 | 1.3E2 | 1.1E3 | 3.9E2 | 96 | 15 | 96 | 15 | 0.47 |
| gW | ng/ml | 5.2E2 | 3.1E2 | 8.5E2 | 6.7E2 | 1.0E3 | 9.5E2 | 2.3E0 | 1.5E2 | 6.1E3 | 3.1E3 | 73 | 9 | 73 | 9 | 0.41 |
| tF | pg/mL | 1.3E3 | 1.4E3 | 1.4E4 | 4.7E3 | 4.4E4 | 7.6E3 | 1.2E1 | 1.8E1 | 2.8E5 | 2.4E4 | 91 | 12 | 91 | 12 | 0.49 |
| hA | ng/ml | 2.6E0 | 3.6E0 | 1.6E1 | 1.8E1 | 5.5E1 | 3.6E1 | 1.7E-2 | 2.0E0 | 3.5E2 | 1.1E2 | 74 | 9 | 74 | 9 | 0.65 |
| nM | pg/ml | 1.0E-9 | 1.0E-9 | 1.0E-9 | 1.7E2 | 0.0E0 | 5.1E2 | 1.0E-9 | 1.0E-9 | 1.0E-9 | 1.5E3 | 54 | 9 | 54 | 9 | 0.56 |
| nN | pg/ml | 1.2E3 | 3.8E3 | 4.1E3 | 2.7E4 | 1.4E4 | 4.9E4 | 1.1E2 | 1.3E3 | 1.0E5 | 1.5E5 | 54 | 9 | 54 | 9 | 0.80 |
| nO | pg/ml | 2.4E1 | 4.3E1 | 3.7E1 | 3.8E1 | 5.0E1 | 2.4E1 | 4.0E0 | 1.2E1 | 3.1E2 | 8.9E1 | 54 | 9 | 54 | 9 | 0.61 |
| nR | pg/ml | 1.7E1 | 4.2E1 | 7.6E1 | 2.6E2 | 1.5E2 | 6.3E2 | 1.0E0 | 4.7E0 | 8.2E2 | 1.9E3 | 54 | 9 | 54 | 9 | 0.60 |
| nT | pg/ml | 6.7E1 | 1.2E2 | 1.0E2 | 2.3E2 | 1.1E2 | 2.7E2 | 1.0E-9 | 8.5E1 | 6.4E2 | 9.2E2 | 54 | 9 | 54 | 9 | 0.72 |
| nU | pg/ml | 4.2E1 | 1.2E2 | 8.4E1 | 2.2E2 | 2.1E2 | 2.9E2 | 1.0E-9 | 1.0E-9 | 1.5E3 | 9.2E2 | 54 | 9 | 54 | 9 | 0.75 |
| lW | pg/ml | 1.0E-9 | 1.0E-9 | 1.3E1 | 8.8E0 | 3.4E1 | 1.4E1 | 1.0E-9 | 1.0E-9 | 1.7E2 | 3.9E1 | 54 | 9 | 54 | 9 | 0.55 |
| lX | pg/ml | 9.0E2 | 5.3E2 | 9.4E2 | 9.3E2 | 5.1E2 | 7.7E2 | 2.3E2 | 1.9E2 | 2.3E3 | 2.5E3 | 54 | 9 | 54 | 9 | 0.43 |
| lY | pg/ml | 1.9E1 | 2.0E1 | 2.1E1 | 2.0E1 | 1.9E1 | 1.5E1 | 1.0E-9 | 5.7E-1 | 1.2E2 | 4.5E1 | 54 | 9 | 54 | 9 | 0.52 |
| mE | pg/ml | 1.0E-9 | 7.0E-1 | 2.8E0 | 2.5E0 | 8.6E0 | 3.4E0 | 1.0E-9 | 1.0E-9 | 5.7E1 | 9.2E0 | 54 | 9 | 54 | 9 | 0.59 |
| mF | pg/ml | 1.4E-1 | 2.7E-1 | 8.4E0 | 5.5E-1 | 3.6E1 | 6.4E-1 | 1.0E-9 | 1.0E-9 | 2.5E2 | 1.7E0 | 54 | 9 | 54 | 9 | 0.47 |
| mH | pg/ml | 3.0E0 | 5.3E0 | 5.2E0 | 5.7E0 | 8.1E0 | 5.5E0 | 4.0E-1 | 9.0E-1 | 5.3E1 | 1.9E1 | 54 | 9 | 54 | 9 | 0.57 |
| mI | pg/ml | 1.0E-9 | 3.0E1 | 1.1E1 | 7.8E1 | 2.8E1 | 1.5E2 | 1.0E-9 | 1.0E-9 | 1.8E2 | 4.6E2 | 54 | 9 | 54 | 9 | 0.75 |
| mM | pg/ml | 3.8E1 | 6.3E1 | 9.3E1 | 9.6E1 | 1.7E2 | 1.5E2 | 1.0E-9 | 1.0E-9 | 1.1E3 | 4.7E2 | 54 | 9 | 54 | 9 | 0.50 |
| mP | pg/ml | 1.5E1 | 1.6E1 | 1.7E1 | 1.2E2 | 1.7E1 | 2.6E2 | 1.0E-9 | 7.5E0 | 1.2E2 | 8.1E2 | 53 | 9 | 53 | 9 | 0.57 |
| mS | pg/ml | 1.6E3 | 1.4E3 | 1.7E3 | 1.7E3 | 9.6E2 | 1.2E3 | 1.0E-9 | 1.0E-9 | 5.1E3 | 3.5E3 | 54 | 9 | 54 | 9 | 0.49 |
| mT | pg/ml | 5.0E1 | 6.1E1 | 1.3E2 | 3.4E2 | 2.5E2 | 5.9E2 | 1.0E1 | 3.1E1 | 1.4E3 | 1.7E3 | 53 | 9 | 53 | 9 | 0.58 |
| mU | pg/ml | 2.2E0 | 4.1E0 | 7.2E0 | 6.1E0 | 3.0E1 | 6.8E0 | 1.0E-9 | 1.2E0 | 2.2E2 | 2.3E1 | 53 | 9 | 53 | 9 | 0.70 |
| mW | pg/ml | 2.2E3 | 1.9E3 | 2.4E3 | 3.4E3 | 1.3E3 | 3.2E3 | 1.0E-9 | 1.3E3 | 6.2E3 | 1.1E4 | 53 | 9 | 53 | 9 | 0.51 |
| mY | pg/ml | 6.3E2 | 1.1E3 | 8.7E2 | 1.7E3 | 1.0E3 | 2.5E3 | 1.0E-9 | 1.9E2 | 5.6E3 | 8.0E3 | 54 | 9 | 54 | 9 | 0.59 |
| mZ | pg/ml | 2.1E2 | 9.1E1 | 4.0E2 | 1.3E2 | 5.2E2 | 1.6E2 | 1.2E1 | 1.1E1 | 3.1E3 | 5.5E2 | 53 | 9 | 53 | 9 | 0.25 |
| nA | pg/ml | 1.5E0 | 3.4E0 | 6.8E0 | 3.6E0 | 1.4E1 | 3.7E0 | 1.0E-9 | 1.0E-9 | 6.5E1 | 9.1E0 | 53 | 9 | 53 | 9 | 0.52 |
| nB | pg/ml | 2.8E2 | 4.0E2 | 3.1E2 | 4.1E2 | 1.7E2 | 2.3E2 | 3.0E1 | 1.5E2 | 8.2E2 | 9.6E2 | 54 | 9 | 54 | 9 | 0.63 |
| nC | pg/ml | 1.0E-9 | 7.8E2 | 1.2E4 | 2.1E3 | 6.0E4 | 3.2E3 | 1.0E-9 | 1.0E-9 | 3.8E5 | 9.1E3 | 54 | 9 | 54 | 9 | 0.74 |
| nD | pg/ml | 6.7E0 | 6.4E0 | 1.3E1 | 1.0E1 | 3.5E1 | 1.0E1 | 1.0E-9 | 1.0E-9 | 2.6E2 | 2.8E1 | 53 | 9 | 53 | 9 | 0.53 |
| nF | pg/ml | 1.0E-9 | 1.0E-9 | 3.2E0 | 1.5E0 | 1.4E1 | 4.5E0 | 1.0E-9 | 1.0E-9 | 9.3E1 | 1.3E1 | 54 | 9 | 54 | 9 | 0.51 |
| nH | pg/ml | 1.8E-1 | 5.1E0 | 2.4E2 | 2.8E1 | 1.4E3 | 4.1E1 | 1.0E-9 | 1.0E-9 | 1.0E4 | 1.2E2 | 53 | 9 | 53 | 9 | 0.76 |
| nI | pg/ml | 4.6E1 | 1.0E-9 | 6.1E1 | 1.0E-9 | 7.8E1 | 0.0E0 | 1.0E-9 | 1.0E-9 | 3.5E2 | 1.0E-9 | 54 | 9 | 54 | 9 | 0.19 |
| nJ | pg/ml | 2.5E-1 | 1.1E0 | 3.4E0 | 1.0E0 | 1.8E1 | 1.0E0 | 1.0E-9 | 1.0E-9 | 1.3E2 | 3.0E0 | 54 | 9 | 54 | 9 | 0.56 |
| nK | pg/ml | 1.0E-9 | 2.0E1 | 1.1E1 | 2.2E1 | 2.3E1 | 2.2E1 | 1.0E-9 | 1.0E-9 | 1.0E2 | 6.2E1 | 53 | 9 | 53 | 9 | 0.69 |
| nL | pg/ml | 1.0E-9 | 2.5E1 | 3.4E2 | 1.3E2 | 1.9E3 | 2.4E2 | 1.0E-9 | 1.0E-9 | 1.4E4 | 7.3E2 | 54 | 9 | 54 | 9 | 0.74 |
| hR | pg/ml | 2.7E4 | 2.6E4 | 2.9E4 | 2.9E4 | 1.1E4 | 9.4E3 | 1.0E-9 | 1.8E4 | 5.8E4 | 4.3E4 | 68 | 8 | 68 | 8 | 0.48 |
| hV | pg/ml | 4.4E2 | 4.4E2 | 4.3E2 | 4.4E2 | 2.2E2 | 2.3E2 | 1.0E-9 | 9.5E1 | 9.6E2 | 7.4E2 | 68 | 8 | 68 | 8 | 0.52 |
| hW | pg/ml | 1.9E3 | 3.1E3 | 2.1E3 | 7.6E3 | 1.2E3 | 1.3E4 | 1.0E-9 | 1.5E3 | 7.3E3 | 4.0E4 | 68 | 8 | 68 | 8 | 0.74 |
| hX | pg/ml | 1.1E3 | 1.3E3 | 1.2E3 | 1.2E3 | 1.0E3 | 3.8E2 | 2.5E0 | 7.0E2 | 8.6E3 | 2.0E3 | 68 | 8 | 68 | 8 | 0.65 |
| iA | pg/ml | 1.8E2 | 1.8E2 | 2.6E2 | 3.0E2 | 2.9E2 | 2.5E2 | 1.5E1 | 6.2E1 | 1.8E3 | 8.7E2 | 90 | 13 | 90 | 13 | 0.56 |
| iB | ng/ml | 5.2E0 | 8.4E0 | 6.5E0 | 1.0E1 | 4.9E0 | 6.4E0 | 3.3E-2 | 2.4E0 | 2.4E1 | 2.2E1 | 74 | 9 | 74 | 9 | 0.68 |
| iC | U/ml | 3.6E-1 | 5.3E-1 | 1.4E0 | 7.7E-1 | 6.4E0 | 8.3E-1 | 1.0E-9 | 6.8E-2 | 5.5E1 | 2.8E0 | 74 | 9 | 74 | 9 | 0.61 |
| iH | ng/ml | 1.7E5 | 1.8E5 | 1.6E5 | 1.6E5 | 5.0E4 | 5.6E4 | 2.9E3 | 7.7E4 | 2.6E5 | 2.4E5 | 90 | 13 | 90 | 13 | 0.49 |
| iJ | ng/ml | 5.0E4 | 3.8E4 | 5.7E4 | 4.7E4 | 3.8E4 | 2.3E4 | 1.8E3 | 1.2E4 | 2.5E5 | 9.3E4 | 90 | 13 | 90 | 13 | 0.43 |
| hB | ng/ml | 4.8E-1 | 5.6E-1 | 6.3E-1 | 8.3E-1 | 5.1E-1 | 5.7E-1 | 1.2E-1 | 2.9E-1 | 3.2E0 | 2.4E0 | 90 | 13 | 90 | 13 | 0.67 |
| hC | pg/ml | 5.3E3 | 1.0E4 | 7.8E3 | 1.3E4 | 1.2E4 | 1.5E4 | 4.1E1 | 3.0E2 | 1.1E5 | 5.7E4 | 90 | 13 | 90 | 13 | 0.63 |
| hF | ng/ml | 1.0E-9 | 1.0E-9 | 4.4E1 | 1.0E-9 | 4.2E2 | 0.0E0 | 1.0E-9 | 1.0E-9 | 4.0E3 | 1.0E-9 | 90 | 13 | 90 | 13 | 0.49 |
| hG | pg/ml | 6.9E3 | 6.6E3 | 7.7E3 | 8.7E3 | 3.1E3 | 5.5E3 | 1.8E3 | 3.6E3 | 1.8E4 | 2.0E4 | 90 | 13 | 90 | 13 | 0.48 |
| iO | ng/ml | 3.9E5 | 4.1E5 | 4.4E5 | 4.1E5 | 2.0E5 | 2.1E5 | 9.8E4 | 1.0E5 | 1.1E6 | 8.2E5 | 90 | 13 | 90 | 13 | 0.46 |
| iP | ng/ml | 5.4E4 | 4.4E4 | 6.0E4 | 4.9E4 | 5.1E4 | 1.8E4 | 7.1E3 | 2.2E4 | 4.4E5 | 7.0E4 | 90 | 13 | 90 | 13 | 0.43 |
| iZ | ng/ml | 1.6E3 | 2.2E3 | 1.8E3 | 2.5E3 | 8.0E2 | 1.1E3 | 7.5E2 | 1.1E3 | 5.7E3 | 4.6E3 | 88 | 13 | 88 | 13 | 0.72 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| rC | pg/ml | 1.4E3 | 1.9E3 | 2.2E3 | 1.7E3 | 2.5E3 | 9.5E2 | 1.0E-9 | 6.0E2 | 1.5E4 | 2.9E3 | 66 | 7 | 66 | 7 | 0.50 |
| rB | pg/ml | 2.9E1 | 7.7E1 | 4.9E1 | 1.1E2 | 6.6E1 | 1.2E2 | 1.0E-9 | 1.0E-9 | 3.9E2 | 3.2E2 | 66 | 7 | 66 | 7 | 0.64 |
| jD | ng/ml | 3.5E1 | 5.0E1 | 4.2E1 | 9.1E1 | 3.9E1 | 9.8E1 | 1.0E-9 | 7.6E0 | 1.9E2 | 2.9E2 | 74 | 9 | 74 | 9 | 0.64 |
| jE | ng/ml | 1.0E-9 | 1.0E-9 | 4.7E0 | 9.4E0 | 1.1E1 | 1.5E1 | 1.0E-9 | 1.0E-9 | 6.2E1 | 3.5E1 | 74 | 9 | 74 | 9 | 0.58 |
| jF | ng/ml | 2.8E1 | 7.7E0 | 4.6E1 | 3.1E1 | 5.2E1 | 4.7E1 | 1.0E-9 | 1.0E-9 | 1.9E2 | 1.2E2 | 74 | 9 | 74 | 9 | 0.42 |
| jG | ng/ml | 4.0E3 | 4.2E3 | 4.4E3 | 4.6E3 | 2.0E3 | 1.5E3 | 6.7E2 | 2.5E3 | 9.6E3 | 7.9E3 | 74 | 9 | 74 | 9 | 0.54 |
| jH | ng/ml | 7.5E1 | 1.2E2 | 7.9E1 | 1.4E2 | 4.4E1 | 1.2E2 | 1.3E1 | 3.3E1 | 2.4E2 | 4.3E2 | 74 | 9 | 74 | 9 | 0.71 |
| jI | ng/ml | 7.3E1 | 1.2E2 | 7.9E1 | 1.6E2 | 3.2E1 | 1.2E2 | 3.8E1 | 4.4E1 | 1.9E2 | 4.4E2 | 74 | 9 | 74 | 9 | 0.74 |
| rA | pg/ml | 2.4E1 | 2.0E1 | 2.7E1 | 2.7E1 | 2.1E1 | 1.8E1 | 1.0E-9 | 1.2E1 | 1.1E2 | 6.8E1 | 72 | 8 | 72 | 8 | 0.50 |
| qY | pg/ml | 1.5E1 | 9.0E0 | 3.9E1 | 1.3E1 | 5.7E1 | 9.0E0 | 8.7E-1 | 2.1E0 | 3.3E2 | 2.7E1 | 72 | 8 | 72 | 8 | 0.35 |
| qX | pg/ml | 5.5E1 | 7.8E1 | 6.6E1 | 9.0E1 | 4.4E1 | 6.2E1 | 1.0E-9 | 2.3E1 | 1.7E2 | 2.1E2 | 72 | 8 | 72 | 8 | 0.60 |
| qW | pg/ml | 7.4E0 | 6.8E0 | 9.2E0 | 9.4E0 | 8.9E0 | 8.6E0 | 1.0E-9 | 1.0E-9 | 3.6E1 | 2.2E1 | 72 | 8 | 72 | 8 | 0.51 |
| qV | pg/ml | 1.6E3 | 1.8E3 | 2.3E3 | 2.2E3 | 2.0E3 | 1.6E3 | 1.0E2 | 6.3E2 | 1.1E4 | 5.6E3 | 72 | 8 | 72 | 8 | 0.50 |
| qU | pg/ml | 7.7E1 | 8.7E1 | 1.9E2 | 2.5E2 | 3.2E2 | 3.7E2 | 1.0E-9 | 1.9E1 | 2.1E3 | 1.1E3 | 72 | 8 | 72 | 8 | 0.57 |
| qT | pg/ml | 3.9E1 | 3.4E1 | 6.6E1 | 5.7E1 | 7.6E1 | 5.4E1 | 1.0E-9 | 6.9E0 | 4.9E2 | 1.6E2 | 72 | 8 | 72 | 8 | 0.46 |
| jK | ng/ml | 1.5E3 | 1.2E3 | 1.6E3 | 1.3E3 | 6.3E2 | 6.3E2 | 2.8E2 | 7.5E2 | 4.1E3 | 2.9E3 | 74 | 9 | 74 | 9 | 0.27 |
| jL | ng/ml | 1.9E2 | 2.9E2 | 2.7E2 | 3.3E2 | 2.0E2 | 1.8E2 | 5.9E1 | 1.5E2 | 8.1E2 | 6.3E2 | 74 | 9 | 74 | 9 | 0.66 |
| jM | ng/ml | 6.8E4 | 4.9E4 | 7.3E4 | 6.8E4 | 3.8E4 | 5.2E4 | 4.6E3 | 1.3E4 | 1.7E5 | 1.4E5 | 74 | 9 | 74 | 9 | 0.43 |
| jO | pg/ml | 2.3E5 | 2.6E5 | 2.7E5 | 2.7E5 | 1.5E5 | 1.6E5 | 7.6E4 | 9.8E4 | 7.7E5 | 6.5E5 | 74 | 9 | 74 | 9 | 0.49 |
| jP | pg/ml | 2.6E5 | 4.9E5 | 3.0E5 | 3.9E5 | 1.4E5 | 1.9E5 | 6.1E4 | 1.3E5 | 7.1E5 | 5.5E5 | 74 | 9 | 74 | 9 | 0.63 |
| jQ | pg/ml | 2.2E3 | 1.2E3 | 3.0E3 | 2.7E3 | 2.7E3 | 2.8E3 | 5.0E0 | 4.9E2 | 1.3E4 | 9.2E3 | 74 | 9 | 74 | 9 | 0.45 |
| jR | pg/ml | 5.5E3 | 3.3E3 | 9.4E3 | 9.5E3 | 1.1E4 | 1.4E4 | 1.0E-9 | 3.0E1 | 5.6E4 | 4.6E4 | 74 | 9 | 74 | 9 | 0.44 |
| jT | pg/ml | 1.8E5 | 1.6E5 | 1.8E5 | 1.5E5 | 6.1E4 | 5.1E4 | 7.1E4 | 7.5E4 | 3.5E5 | 2.2E5 | 74 | 9 | 74 | 9 | 0.35 |
| jU | mIU/ml | 5.5E0 | 5.8E0 | 1.2E1 | 1.4E1 | 2.0E1 | 1.8E1 | 8.1E-2 | 1.2E0 | 1.1E2 | 5.3E1 | 74 | 9 | 74 | 9 | 0.50 |
| jV | mIU/ml | 2.0E0 | 1.9E0 | 4.1E0 | 3.5E0 | 5.8E0 | 3.6E0 | 2.7E-3 | 2.1E-1 | 3.2E1 | 1.0E1 | 74 | 9 | 74 | 9 | 0.48 |
| jY | ng/ml | 1.1E-3 | 2.6E-3 | 8.8E-3 | 7.4E-3 | 3.7E-2 | 9.4E-3 | 1.0E-9 | 1.0E-9 | 3.0E-1 | 2.6E-2 | 74 | 9 | 74 | 9 | 0.59 |
| kC | pg/ml | 1.0E2 | 9.6E1 | 2.0E2 | 1.4E2 | 4.0E2 | 1.7E2 | 2.1E1 | 3.6E1 | 2.7E3 | 5.9E2 | 54 | 9 | 54 | 9 | 0.45 |
| kE | pg/ml | 1.5E5 | 1.4E5 | 1.4E5 | 1.4E5 | 3.5E4 | 5.8E4 | 4.1E4 | 7.9E4 | 2.3E5 | 2.7E5 | 54 | 9 | 54 | 9 | 0.44 |
| kF | pg/mL | 6.8E1 | 5.2E1 | 6.9E1 | 7.9E1 | 2.2E1 | 4.1E1 | 3.5E1 | 4.0E1 | 1.5E2 | 1.4E2 | 54 | 9 | 54 | 9 | 0.46 |
| kG | pg/mL | 8.6E3 | 8.8E3 | 1.1E4 | 3.2E4 | 1.0E4 | 5.2E4 | 1.9E3 | 1.1E3 | 5.8E4 | 1.6E5 | 54 | 9 | 54 | 9 | 0.49 |
| kI | pg/ml | 2.1E2 | 1.8E2 | 2.2E2 | 2.0E2 | 1.1E2 | 1.1E2 | 4.4E1 | 1.0E-9 | 6.7E2 | 3.5E2 | 54 | 9 | 54 | 9 | 0.45 |
| kK | pg/ml | 1.2E2 | 1.2E2 | 1.6E2 | 1.5E2 | 1.5E2 | 9.2E1 | 2.1E1 | 2.9E1 | 9.1E2 | 2.9E2 | 54 | 9 | 54 | 9 | 0.54 |
| kN | pg/ml | 1.2E3 | 7.4E2 | 1.7E3 | 1.4E3 | 1.8E3 | 2.1E3 | 2.1E2 | 3.8E2 | 1.0E4 | 7.0E3 | 54 | 9 | 54 | 9 | 0.33 |
| kO | pg/ml | 7.1E3 | 7.6E3 | 1.0E4 | 8.2E3 | 1.9E4 | 2.7E3 | 3.8E3 | 5.0E3 | 1.5E5 | 1.3E4 | 54 | 9 | 54 | 9 | 0.58 |
| kP | pg/ml | 5.6E3 | 3.5E3 | 6.8E3 | 5.3E3 | 4.3E3 | 4.3E3 | 9.6E2 | 1.6E3 | 2.7E4 | 1.5E4 | 54 | 9 | 54 | 9 | 0.35 |
| kQ | pg/ml | 4.4E3 | 4.9E3 | 5.5E3 | 6.7E3 | 4.1E3 | 5.4E3 | 5.6E2 | 1.4E3 | 2.5E4 | 2.2E4 | 90 | 13 | 90 | 13 | 0.57 |
| kR | pg/ml | 2.4E1 | 1.5E1 | 4.0E1 | 3.4E1 | 1.1E2 | 3.4E1 | 1.0E-9 | 5.5E0 | 1.0E3 | 1.1E2 | 90 | 13 | 90 | 13 | 0.47 |
| kS | pg/ml | 8.8E2 | 8.7E2 | 9.9E2 | 1.0E3 | 5.9E2 | 6.9E2 | 8.2E1 | 3.2E2 | 3.2E3 | 2.5E3 | 90 | 13 | 90 | 13 | 0.47 |
| rZ | ng/ml | 1.8E-3 | 8.2E-3 | 7.7E-3 | 2.4E-2 | 1.6E-2 | 3.9E-2 | 1.0E-9 | 1.0E-9 | 9.4E-2 | 1.1E-1 | 67 | 7 | 67 | 7 | 0.60 |
| rY | ng/ml | 6.4E-2 | 7.6E-2 | 5.0E-1 | 8.4E-2 | 2.5E0 | 4.8E-2 | 1.0E-9 | 2.8E-2 | 2.0E1 | 1.6E-1 | 67 | 7 | 67 | 7 | 0.56 |
| rX | ng/ml | 1.0E-9 | 1.0E-9 | 1.2E-1 | 2.7E-2 | 5.9E-1 | 4.7E-2 | 1.0E-9 | 1.0E-9 | 3.7E0 | 1.2E-1 | 67 | 7 | 67 | 7 | 0.60 |
| lK | pg/ml | 7.1E1 | 3.6E1 | 1.4E2 | 9.7E1 | 1.7E2 | 1.5E2 | 1.0E-9 | 2.3E0 | 7.0E2 | 4.8E2 | 73 | 9 | 73 | 9 | 0.37 |
| lL | pg/ml | 1.5E3 | 2.5E3 | 3.0E3 | 2.8E3 | 5.4E3 | 1.7E3 | 7.5E1 | 8.8E2 | 4.2E4 | 6.8E3 | 74 | 9 | 74 | 9 | 0.63 |
| lM | pg/ml | 1.2E3 | 3.1E3 | 3.9E3 | 1.5E4 | 6.7E3 | 2.3E4 | 9.5E0 | 2.6E2 | 4.2E4 | 6.7E4 | 74 | 9 | 74 | 9 | 0.63 |
| lN | pg/ml | 1.0E-9 | 3.0E0 | 3.2E0 | 4.2E0 | 7.2E0 | 6.2E0 | 1.0E-9 | 1.0E-9 | 4.3E1 | 1.9E1 | 74 | 9 | 74 | 9 | 0.60 |
| lO | pg/ml | 1.0E-9 | 1.0E-9 | 2.2E0 | 1.5E1 | 1.5E1 | 4.6E1 | 1.0E-9 | 1.0E-9 | 1.3E2 | 1.4E2 | 73 | 9 | 73 | 9 | 0.54 |
| nW | pg/ml | 1.1E5 | 1.1E5 | 1.1E5 | 1.1E5 | 2.7E4 | 3.3E4 | 5.8E4 | 3.6E4 | 1.8E5 | 1.5E5 | 90 | 13 | 90 | 13 | 0.52 |
| nY | pg/ml | 2.3E3 | 3.5E3 | 2.6E3 | 3.3E3 | 1.6E3 | 2.0E3 | 5.1E2 | 6.3E2 | 1.0E4 | 8.1E3 | 90 | 13 | 90 | 13 | 0.60 |
| oO | pg/ml | 9.2E4 | 1.0E5 | 1.1E5 | 1.4E5 | 6.1E4 | 1.2E5 | 2.0E4 | 3.3E3 | 3.0E5 | 4.0E5 | 48 | 8 | 48 | 8 | 0.54 |
| oP | pg/ml | 1.3E5 | 1.4E5 | 1.5E5 | 1.9E5 | 8.4E4 | 1.7E5 | 4.2E4 | 2.4E4 | 4.2E5 | 5.7E5 | 48 | 8 | 48 | 8 | 0.54 |
| oQ | pg/ml | 3.2E3 | 4.8E3 | 3.9E3 | 8.0E3 | 3.2E3 | 1.0E4 | 7.7E2 | 9.1E2 | 2.1E4 | 3.2E4 | 48 | 8 | 48 | 8 | 0.65 |
| oE | pg/ml | 2.1E2 | 8.9E2 | 5.0E2 | 1.0E3 | 6.2E2 | 1.0E3 | 1.0E-9 | 1.0E-9 | 2.8E3 | 3.4E3 | 90 | 13 | 90 | 13 | 0.66 |
| oF | pg/ml | 1.4E4 | 2.5E4 | 2.8E4 | 4.7E4 | 3.9E4 | 4.5E4 | 4.3E2 | 2.0E3 | 2.5E5 | 1.4E5 | 90 | 13 | 90 | 13 | 0.67 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|---------|-----|------|
| | | NonDis | Dis | NonDis | Dis | NonDis | Dis | NonDis | Dis | NonDis | Dis | NonDis | Dis | NonDis | Dis | |
| oH | pg/ml | 3.9E1 | 2.4E1 | 8.5E1 | 5.2E1 | 1.3E2 | 8.0E1 | 4.4E0 | 1.1E1 | 8.6E2 | 3.1E2 | 90 | 13 | 90 | 13 | 0.41 |
| oK | pg/ml | 9.0E2 | 1.5E3 | 1.6E3 | 1.8E3 | 1.9E3 | 1.7E3 | 8.8E1 | 2.3E2 | 1.2E4 | 5.9E3 | 90 | 13 | 90 | 13 | 0.54 |
| oN | pg/ml | 5.6E2 | 5.7E2 | 1.1E3 | 6.7E2 | 2.1E3 | 3.4E2 | 1.1E2 | 3.3E2 | 1.8E4 | 1.5E3 | 90 | 13 | 90 | 13 | 0.54 |
| pF | pg/ml | 6.0E-1 | 5.7E-1 | 1.9E0 | 7.1E-1 | 9.1E0 | 4.0E-1 | 1.0E-9 | 1.8E-1 | 8.7E1 | 1.3E0 | 90 | 13 | 90 | 13 | 0.50 |

Figure 39.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 7.2E1 | 1.1E2 | 8.0E1 | 1.2E2 | 4.9E1 | 8.6E1 | 8.0E0 | 7.0E0 | 2.4E2 | 4.8E2 | 197 | 101 | 197 | 101 | 0.64 |
| Ad | ug/mL | 4.2E-2 | 5.3E-2 | 7.1E-2 | 2.1E-1 | 8.3E-2 | 1.0E0 | 6.8E-4 | 7.8E-4 | 3.7E-1 | 8.5E0 | 106 | 68 | 106 | 68 | 0.55 |
| Af | ng/mL | 1.3E0 | 1.1E0 | 1.3E1 | 5.2E0 | 5.6E1 | 6.8E0 | 1.7E-3 | 1.7E-3 | 5.3E2 | 2.3E1 | 106 | 68 | 106 | 68 | 0.50 |
| Aj | ug/mL | 2.0E0 | 3.0E-1 | 2.7E0 | 1.7E0 | 2.5E0 | 2.3E0 | 1.5E-3 | 1.3E-3 | 6.1E0 | 6.1E0 | 106 | 68 | 106 | 68 | 0.38 |
| Al | mg/mL | 8.7E-5 | 8.5E-5 | 2.6E-4 | 3.0E-4 | 4.4E-4 | 4.5E-4 | 4.3E-6 | 6.6E-6 | 1.8E-3 | 1.8E-3 | 106 | 68 | 106 | 68 | 0.54 |
| An | U/mL | 5.0E1 | 8.4E1 | 2.1E2 | 3.3E2 | 6.3E2 | 9.8E2 | 2.8E-1 | 6.4E-1 | 5.5E3 | 7.8E3 | 106 | 68 | 106 | 68 | 0.61 |
| Ao | pg/mL | 9.1E1 | 9.9E1 | 7.3E2 | 2.6E2 | 4.5E3 | 5.8E2 | 1.5E0 | 4.1E0 | 3.9E4 | 4.5E3 | 106 | 68 | 106 | 68 | 0.56 |
| Ap | ng/mL | 3.4E1 | 3.1E1 | 5.0E1 | 5.1E1 | 5.6E1 | 5.8E1 | 2.0E0 | 2.4E0 | 3.3E2 | 2.9E2 | 106 | 68 | 106 | 68 | 0.49 |
| Ar | ng/mL | 5.3E-1 | 1.3E0 | 2.3E0 | 4.4E0 | 5.8E0 | 9.4E0 | 3.4E-3 | 3.4E-3 | 4.7E1 | 5.1E1 | 106 | 68 | 106 | 68 | 0.63 |
| As | ng/mL | 8.7E-3 | 9.3E-3 | 1.2E-2 | 3.1E-2 | 1.5E-2 | 1.5E-1 | 1.7E-3 | 1.7E-3 | 9.8E-2 | 1.2E0 | 106 | 68 | 106 | 68 | 0.50 |
| Aw | pg/mL | 1.7E1 | 1.7E1 | 1.7E1 | 1.7E1 | 5.8E0 | 6.8E0 | 6.8E0 | 2.9E-2 | 4.2E1 | 5.1E1 | 106 | 68 | 106 | 68 | 0.49 |
| Ax | ng/mL | 1.5E0 | 5.4E0 | 1.5E1 | 5.2E1 | 7.6E1 | 1.5E2 | 1.3E-2 | 1.0E-4 | 7.7E2 | 8.5E2 | 106 | 68 | 106 | 68 | 0.60 |
| Ba | ng/mL | 9.0E1 | 8.9E1 | 5.2E2 | 9.8E2 | 1.4E3 | 2.4E3 | 1.9E0 | 1.1E0 | 8.1E3 | 1.5E4 | 106 | 68 | 106 | 68 | 0.55 |
| Bb | ng/mL | 3.8E0 | 6.0E0 | 6.2E0 | 9.4E0 | 7.7E0 | 1.0E1 | 4.1E-3 | 4.1E-3 | 4.9E1 | 4.8E1 | 106 | 68 | 106 | 68 | 0.58 |
| Bc | ng/mL | 3.1E1 | 6.1E1 | 1.1E2 | 1.6E2 | 2.2E2 | 2.6E2 | 4.9E-1 | 1.0E0 | 1.0E3 | 1.2E3 | 106 | 68 | 106 | 68 | 0.58 |
| Bg | ng/mL | 1.1E-1 | 1.1E-1 | 4.3E0 | 9.2E0 | 1.8E1 | 5.0E1 | 5.3E-4 | 5.3E-4 | 1.5E2 | 4.0E2 | 106 | 68 | 106 | 68 | 0.51 |
| Bn | ng/mL | 5.6E-2 | 9.7E-2 | 1.2E0 | 2.0E0 | 2.0E0 | 7.2E0 | 5.6E-2 | 5.6E-2 | 8.5E0 | 5.8E1 | 106 | 68 | 106 | 68 | 0.54 |
| Bo | ng/mL | 1.3E1 | 1.3E1 | 1.5E1 | 1.6E1 | 1.1E1 | 1.2E1 | 1.6E-2 | 1.6E-2 | 4.8E1 | 5.3E1 | 106 | 68 | 106 | 68 | 0.52 |
| Ch | uIU/mL | 1.0E0 | 9.6E-1 | 3.5E1 | 3.3E1 | 1.9E2 | 1.6E2 | 3.4E-3 | 3.9E-2 | 1.8E3 | 1.2E3 | 106 | 68 | 106 | 68 | 0.46 |
| Co | pg/mL | 4.6E1 | 5.1E1 | 1.4E2 | 3.7E2 | 4.2E2 | 2.0E3 | 1.5E-1 | 1.5E-1 | 3.7E3 | 1.7E4 | 106 | 68 | 106 | 68 | 0.51 |
| Cp | ng/mL | 2.1E1 | 2.2E1 | 2.7E1 | 4.9E1 | 2.2E1 | 1.5E2 | 6.0E-1 | 4.7E0 | 1.3E2 | 1.3E3 | 106 | 68 | 106 | 68 | 0.55 |
| Cq | ng/mL | 2.4E-2 | 3.4E-2 | 8.5E-2 | 9.0E-1 | 2.4E-1 | 6.0E0 | 8.0E-4 | 8.0E-4 | 2.0E0 | 4.9E1 | 106 | 68 | 106 | 68 | 0.57 |
| Cs | ng/mL | 3.8E1 | 1.5E2 | 3.0E2 | 7.9E2 | 1.1E3 | 2.3E3 | 1.0E0 | 8.3E-1 | 1.1E4 | 1.8E4 | 106 | 68 | 106 | 68 | 0.65 |
| Ct | ng/mL | 5.3E-1 | 1.6E-1 | 4.0E1 | 4.4E1 | 1.2E2 | 1.2E2 | 1.3E-2 | 1.1E-4 | 6.2E2 | 6.2E2 | 106 | 68 | 106 | 68 | 0.44 |
| Cu | ng/mL | 2.4E-1 | 3.0E-1 | 4.6E-1 | 2.0E0 | 9.2E-1 | 8.3E0 | 1.9E-2 | 1.7E-2 | 9.0E0 | 6.6E1 | 106 | 68 | 106 | 68 | 0.58 |
| Cv | ng/mL | 4.2E0 | 8.6E0 | 2.7E1 | 3.3E1 | 7.2E1 | 7.1E1 | 2.0E-2 | 5.1E-2 | 5.3E2 | 4.7E2 | 106 | 68 | 106 | 68 | 0.56 |
| Cw | mIU/mL | 3.1E-2 | 4.2E-2 | 4.3E-2 | 1.4E-1 | 3.4E-2 | 8.2E-1 | 8.9E-4 | 2.8E-3 | 1.8E-1 | 6.8E0 | 106 | 68 | 106 | 68 | 0.51 |
| Cx | ng/mL | 8.2E-1 | 6.6E-1 | 5.7E1 | 3.9E1 | 1.1E2 | 8.3E1 | 9.3E-5 | 9.3E-5 | 3.9E2 | 2.8E2 | 106 | 68 | 106 | 68 | 0.49 |
| Db | ug/mL | 7.8E0 | 7.0E0 | 9.2E0 | 7.9E0 | 7.2E0 | 8.0E0 | 4.5E-1 | 8.1E-1 | 4.3E1 | 5.9E1 | 106 | 68 | 106 | 68 | 0.44 |
| Dc | nmol/L | 2.0E-2 | 2.3E-2 | 4.2E-2 | 3.5E-1 | 6.7E-2 | 1.7E0 | 5.2E-6 | 1.1E-3 | 4.8E-1 | 1.4E1 | 106 | 68 | 106 | 68 | 0.58 |
| Dd | ug/mL | 6.4E-2 | 1.2E-1 | 1.6E-1 | 2.8E-1 | 2.4E-1 | 5.1E-1 | 4.8E-4 | 3.4E-3 | 1.6E0 | 3.6E0 | 106 | 68 | 106 | 68 | 0.58 |
| De | ng/mL | 3.4E-3 | 3.4E-3 | 7.0E-2 | 1.1E-1 | 1.4E-1 | 1.9E-1 | 3.4E-3 | 3.4E-3 | 9.2E-1 | 1.1E0 | 106 | 68 | 106 | 68 | 0.56 |
| Dg | ng/mL | 3.9E1 | 3.8E1 | 4.7E1 | 5.0E1 | 3.9E1 | 4.3E1 | 1.0E0 | 7.1E-1 | 1.8E2 | 1.9E2 | 106 | 68 | 106 | 68 | 0.52 |
| Di | pg/mL | 2.0E0 | 2.7E0 | 2.3E0 | 2.7E0 | 2.3E0 | 1.9E0 | 1.8E-1 | 1.8E-1 | 1.3E1 | 9.3E0 | 106 | 68 | 106 | 68 | 0.58 |
| Dk | uIU/mL | 1.4E-2 | 1.6E-2 | 6.0E-2 | 6.5E-2 | 1.9E-1 | 1.6E-1 | 1.1E-4 | 1.1E-4 | 1.6E0 | 9.8E-1 | 106 | 68 | 106 | 68 | 0.53 |
| Dl | ng/mL | 1.9E2 | 2.2E2 | 2.9E2 | 3.3E2 | 2.6E2 | 3.2E2 | 8.9E0 | 4.4E0 | 1.3E3 | 1.6E3 | 106 | 68 | 106 | 68 | 0.53 |
| Dp | ng/ml | 2.4E0 | 1.8E0 | 5.6E0 | 5.1E0 | 8.3E0 | 9.5E0 | 3.7E-3 | 3.7E-3 | 4.6E1 | 5.6E1 | 80 | 55 | 80 | 55 | 0.44 |
| Dr | pg/ml | 1.8E1 | 3.0E1 | 4.2E1 | 4.2E2 | 5.7E1 | 1.9E3 | 7.5E-1 | 7.5E-1 | 2.5E2 | 1.0E4 | 42 | 30 | 42 | 30 | 0.58 |
| Du | pg/ml | 8.2E1 | 2.5E2 | 5.5E2 | 2.2E3 | 1.1E3 | 5.8E3 | 1.2E0 | 1.2E0 | 5.4E3 | 2.4E4 | 31 | 26 | 31 | 26 | 0.59 |
| Dw | ng/ml | 9.2E-3 | 2.3E-2 | 3.9E-2 | 6.0E-2 | 5.9E-2 | 7.6E-2 | 9.2E-3 | 9.2E-3 | 1.9E-1 | 1.9E-1 | 10 | 8 | 10 | 8 | 0.58 |
| Ef | ng/ml | 1.4E-1 | 9.0E-2 | 8.9E-1 | 9.1E-1 | 1.8E0 | 2.3E0 | 1.6E-3 | 5.7E-4 | 1.0E1 | 9.9E0 | 87 | 60 | 87 | 60 | 0.46 |
| Wm | % | 8.5E-2 | 8.5E-2 | 3.9E0 | 5.2E1 | 2.0E1 | 2.0E2 | 5.4E-2 | 8.5E-2 | 2.0E2 | 1.0E3 | 98 | 60 | 98 | 60 | 0.51 |
| Ed | pg/ml | 5.2E-1 | 2.3E1 | 2.3E1 | 5.7E1 | 3.7E1 | 9.9E1 | 5.2E-1 | 5.2E-1 | 1.9E2 | 5.0E2 | 80 | 55 | 80 | 55 | 0.61 |
| Eo | ng/ml | 6.1E0 | 3.0E0 | 6.4E0 | 7.8E0 | 5.2E0 | 1.3E1 | 3.6E-1 | 3.6E-1 | 1.6E1 | 4.0E1 | 10 | 8 | 10 | 8 | 0.41 |
| Yf | ng/mL | 1.5E1 | 1.5E1 | 3.9E1 | 7.1E1 | 5.6E1 | 1.4E2 | 2.9E-1 | 2.9E-1 | 2.4E2 | 5.9E2 | 34 | 25 | 34 | 25 | 0.48 |
| Tj | pg/mL | 3.7E-1 | 3.7E-1 | 2.3E1 | 6.3E1 | 1.0E2 | 3.1E2 | 3.7E-1 | 3.7E-1 | 8.7E2 | 2.3E3 | 88 | 57 | 88 | 57 | 0.54 |
| Po | pg/ml | 1.2E-1 | 2.8E0 | 7.8E0 | 1.7E1 | 2.6E1 | 3.9E1 | 8.0E-3 | 2.6E-2 | 2.7E2 | 2.2E2 | 246 | 107 | 246 | 107 | 0.61 |
| Ti | ug/mL | 2.8E0 | 5.4E0 | 4.1E0 | 6.1E0 | 3.8E0 | 4.6E0 | 1.2E-1 | 4.0E-1 | 1.6E1 | 1.8E1 | 48 | 36 | 48 | 36 | 0.63 |
| Em | ng/ml | 2.9E-3 | 2.6E-2 | 4.1E-2 | 1.1E-1 | 8.3E-2 | 3.2E-1 | 8.4E-4 | 8.4E-4 | 5.0E-1 | 1.9E0 | 53 | 39 | 53 | 39 | 0.58 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Et | ng/ml | 1.2E3 | 2.1E3 | 1.5E3 | 2.3E3 | 1.1E3 | 1.2E3 | 7.5E1 | 7.9E1 | 4.8E3 | 5.0E3 | 245 | 107 | 245 | 107 | 0.69 |
| Eq | pg/ml | 2.8E2 | 6.6E1 | 3.9E2 | 2.9E2 | 4.0E2 | 4.0E2 | 1.0E0 | 1.0E0 | 1.5E3 | 1.4E3 | 31 | 26 | 31 | 26 | 0.40 |
| Ew | U/ml | 1.9E0 | 2.1E0 | 2.7E0 | 2.5E0 | 2.3E0 | 1.3E0 | 1.1E0 | 1.3E0 | 8.8E0 | 5.0E0 | 10 | 8 | 10 | 8 | 0.56 |
| Th | ug/mL | 1.2E0 | 1.2E0 | 1.6E0 | 2.0E0 | 1.2E0 | 1.9E0 | 1.3E-1 | 2.6E-3 | 5.4E0 | 7.5E0 | 48 | 36 | 48 | 36 | 0.49 |
| Fa | ng/ml | 3.9E1 | 5.9E1 | 5.6E1 | 2.2E2 | 5.9E1 | 6.0E2 | 2.6E-1 | 6.0E-1 | 2.6E2 | 3.7E3 | 78 | 53 | 78 | 53 | 0.59 |
| Ez | ng/ml | 3.8E0 | 4.1E0 | 1.6E1 | 1.5E1 | 3.0E1 | 3.2E1 | 1.3E-2 | 1.3E-2 | 1.6E2 | 2.0E2 | 80 | 55 | 80 | 55 | 0.50 |
| Fb | ng/ml | 2.5E1 | 2.7E1 | 2.2E1 | 2.4E1 | 1.2E1 | 1.0E1 | 6.6E-1 | 8.9E-1 | 4.3E1 | 4.3E1 | 78 | 54 | 78 | 54 | 0.55 |
| Ex | ng/ml | 5.8E-2 | 1.2E-1 | 1.8E-1 | 3.1E-1 | 3.4E-1 | 6.8E-1 | 3.5E-5 | 1.7E-4 | 2.2E0 | 4.1E0 | 63 | 42 | 63 | 42 | 0.60 |
| Fc | pg/ml | 2.2E-1 | 2.2E-1 | 1.8E1 | 3.2E1 | 7.9E1 | 9.5E1 | 2.2E-1 | 2.2E-1 | 4.5E2 | 3.9E2 | 32 | 26 | 32 | 26 | 0.59 |
| Fd | pg/ml | 6.7E1 | 1.6E2 | 4.2E2 | 2.5E3 | 7.9E2 | 6.5E3 | 4.5E-1 | 9.8E-1 | 2.9E3 | 2.5E4 | 32 | 26 | 32 | 26 | 0.54 |
| Fi | pg/ml | 2.5E-1 | 2.5E-1 | 6.7E1 | 1.2E2 | 2.0E2 | 3.7E2 | 2.5E-1 | 2.5E-1 | 8.5E2 | 1.8E3 | 32 | 26 | 32 | 26 | 0.57 |
| Fn | ng/ml | 2.1E-1 | 2.8E-1 | 3.7E0 | 4.3E0 | 7.6E0 | 6.4E0 | 1.1E-14 | 2.1E-1 | 3.7E1 | 2.7E1 | 80 | 55 | 80 | 55 | 0.57 |
| Fp | ng/ml | 1.0E1 | 2.0E1 | 2.0E1 | 3.2E1 | 2.4E1 | 3.2E1 | 6.0E-3 | 2.8E-1 | 1.3E2 | 1.3E2 | 246 | 109 | 246 | 109 | 0.63 |
| Fr | ng/ml | 3.0E4 | 6.1E4 | 1.1E5 | 1.8E5 | 1.8E5 | 2.4E5 | 1.9E2 | 1.3E3 | 8.4E5 | 8.4E5 | 251 | 111 | 251 | 111 | 0.62 |
| Fw | pg/ml | 8.5E-1 | 5.8E0 | 4.5E1 | 4.9E1 | 3.2E2 | 1.4E2 | 1.2E-1 | 1.2E-1 | 3.0E3 | 9.1E2 | 89 | 60 | 89 | 60 | 0.61 |
| Fy | ng/ml | 3.2E1 | 4.9E1 | 5.1E1 | 1.0E2 | 5.5E1 | 1.4E2 | 1.2E-1 | 1.2E-1 | 2.8E2 | 6.5E2 | 79 | 53 | 79 | 53 | 0.59 |
| Gh | pg/ml | 1.3E0 | 5.0E0 | 2.7E1 | 1.4E1 | 6.8E1 | 2.9E1 | 2.9E-2 | 2.9E-2 | 3.2E2 | 1.4E2 | 32 | 26 | 32 | 26 | 0.56 |
| Gb | % | 4.2E1 | 3.8E1 | 4.7E1 | 6.1E1 | 4.2E1 | 6.4E1 | 3.7E0 | 2.2E0 | 2.3E2 | 3.0E2 | 32 | 26 | 32 | 26 | 0.56 |
| Gc | ng/ml | 6.7E1 | 1.3E2 | 1.2E2 | 2.4E2 | 1.3E2 | 2.6E2 | 6.4E0 | 6.9E0 | 7.3E2 | 1.2E3 | 42 | 30 | 42 | 30 | 0.67 |
| Gd | ng/ml | 3.2E1 | 3.1E1 | 3.4E1 | 3.2E1 | 1.8E1 | 1.9E1 | 6.3E0 | 3.0E0 | 8.1E1 | 8.0E1 | 50 | 32 | 50 | 32 | 0.46 |
| Gn | U/ml | 2.1E-1 | 2.1E-1 | 1.5E0 | 4.4E0 | 4.8E0 | 2.1E1 | 5.6E-3 | 5.6E-3 | 3.0E1 | 1.1E2 | 41 | 30 | 41 | 30 | 0.52 |
| Gl | pg/ml | 8.5E3 | 1.4E4 | 1.1E4 | 1.5E4 | 9.0E3 | 1.0E4 | 9.1E1 | 4.0E2 | 3.2E4 | 3.2E4 | 89 | 60 | 89 | 60 | 0.59 |
| Gp | U/ml | 1.2E0 | 1.0E0 | 2.5E0 | 2.2E0 | 3.5E0 | 3.4E0 | 1.5E-2 | 1.5E-2 | 2.0E1 | 1.8E1 | 89 | 59 | 89 | 59 | 0.45 |
| Gz | ug/ml | 9.1E0 | 9.2E-1 | 6.5E0 | 4.0E0 | 6.1E0 | 4.9E0 | 4.2E-2 | 8.9E-2 | 2.5E1 | 1.5E1 | 55 | 39 | 55 | 39 | 0.39 |
| Ha | ng/ml | 1.7E0 | 2.6E0 | 5.9E0 | 1.3E1 | 1.4E1 | 2.6E1 | 1.7E-2 | 1.7E-2 | 1.0E2 | 1.0E2 | 78 | 55 | 78 | 55 | 0.60 |
| Nm | pg/ml | 1.1E4 | 2.0E4 | 2.5E4 | 6.5E4 | 5.6E4 | 1.4E5 | 1.0E-9 | 1.0E-9 | 7.8E5 | 9.6E5 | 247 | 109 | 247 | 109 | 0.62 |
| Nn | pg/ml | 1.4E2 | 2.9E2 | 1.2E3 | 7.2E3 | 4.8E3 | 3.3E4 | 1.0E-9 | 1.0E-9 | 6.0E4 | 3.1E5 | 247 | 109 | 247 | 109 | 0.61 |
| No | pg/ml | 1.3E1 | 2.5E1 | 2.9E1 | 6.8E1 | 5.7E1 | 1.5E2 | 1.0E-9 | 1.0E-9 | 5.6E2 | 9.1E2 | 247 | 109 | 247 | 109 | 0.64 |
| Nq | pg/ml | 1.9E0 | 2.1E0 | 1.8E1 | 3.7E1 | 6.3E1 | 1.1E2 | 1.0E-9 | 1.0E-9 | 6.2E2 | 6.7E2 | 247 | 109 | 247 | 109 | 0.53 |
| Nr | pg/ml | 1.2E0 | 3.0E0 | 2.1E1 | 4.3E1 | 8.0E1 | 1.6E2 | 1.0E-9 | 1.0E-9 | 9.8E2 | 1.4E3 | 247 | 109 | 247 | 109 | 0.59 |
| Ns | pg/ml | 1.0E-9 | 1.0E-9 | 5.2E0 | 1.3E1 | 2.7E1 | 1.1E2 | 1.0E-9 | 1.0E-9 | 2.4E2 | 1.1E3 | 247 | 109 | 247 | 109 | 0.50 |
| Nt | pg/ml | 8.8E1 | 1.4E2 | 1.2E2 | 2.0E2 | 8.9E1 | 2.2E2 | 9.8E-1 | 2.3E1 | 5.9E2 | 1.7E3 | 247 | 109 | 247 | 109 | 0.69 |
| Nu | pg/ml | 9.5E0 | 5.0E1 | 4.5E1 | 8.5E1 | 8.1E1 | 1.1E2 | 1.0E-9 | 1.0E-9 | 4.2E2 | 6.3E2 | 247 | 109 | 247 | 109 | 0.67 |
| Lu | pg/ml | 1.0E4 | 1.0E4 | 1.5E4 | 1.6E4 | 3.1E4 | 5.4E4 | 7.7E2 | 5.2E2 | 3.9E5 | 5.6E5 | 247 | 109 | 247 | 109 | 0.49 |
| Lv | pg/ml | 1.0E-9 | 6.8E0 | 1.2E1 | 2.6E1 | 2.5E1 | 4.4E1 | 1.0E-9 | 1.0E-9 | 2.4E2 | 2.6E2 | 247 | 109 | 247 | 109 | 0.60 |
| Lw | pg/ml | 1.0E-9 | 1.0E-9 | 1.4E-1 | 3.5E0 | 2.0E0 | 1.9E1 | 1.0E-9 | 1.0E-9 | 3.2E1 | 1.8E2 | 247 | 109 | 247 | 109 | 0.54 |
| Lx | pg/ml | 1.0E-9 | 5.5E1 | 1.4E2 | 5.6E2 | 4.8E2 | 2.3E3 | 1.0E-9 | 1.0E-9 | 6.2E3 | 2.2E4 | 247 | 109 | 247 | 109 | 0.64 |
| Ly | pg/ml | 1.0E-9 | 1.0E-9 | 8.9E0 | 9.5E0 | 1.8E1 | 1.7E1 | 1.0E-9 | 1.0E-9 | 1.1E2 | 9.6E1 | 247 | 109 | 247 | 109 | 0.51 |
| Lz | pg/ml | 1.0E-9 | 1.0E-9 | 3.4E0 | 3.7E0 | 2.9E1 | 1.6E1 | 1.0E-9 | 1.0E-9 | 4.3E2 | 1.3E2 | 247 | 109 | 247 | 109 | 0.52 |
| Ma | pg/ml | 3.7E2 | 7.6E2 | 2.0E3 | 3.4E3 | 5.7E3 | 7.4E3 | 1.0E-9 | 1.0E-9 | 6.5E4 | 5.2E4 | 247 | 109 | 247 | 109 | 0.59 |
| Mb | pg/ml | 2.4E1 | 2.9E1 | 3.0E1 | 3.5E1 | 1.8E1 | 1.7E1 | 9.2E0 | 4.1E0 | 2.1E2 | 1.1E2 | 247 | 109 | 247 | 109 | 0.58 |
| Mc | pg/ml | 1.0E-9 | 1.0E-9 | 7.0E-2 | 1.5E-2 | 8.7E-1 | 1.6E-1 | 1.0E-9 | 1.0E-9 | 1.3E1 | 1.7E0 | 247 | 109 | 247 | 109 | 0.50 |
| Md | pg/ml | 1.0E-9 | 1.0E-9 | 6.3E-1 | 7.4E-1 | 6.0E0 | 3.6E0 | 1.0E-9 | 1.0E-9 | 6.8E1 | 2.9E1 | 247 | 109 | 247 | 109 | 0.52 |
| Me | pg/ml | 3.2E1 | 3.0E1 | 3.0E1 | 3.3E1 | 1.6E1 | 3.8E1 | 1.0E-9 | 1.0E-9 | 1.2E2 | 3.2E2 | 247 | 109 | 247 | 109 | 0.48 |
| Mf | pg/ml | 1.0E-9 | 1.0E-9 | 3.4E-1 | 1.3E0 | 1.6E0 | 6.5E0 | 1.0E-9 | 1.0E-9 | 2.0E1 | 5.6E1 | 247 | 109 | 247 | 109 | 0.54 |
| Mg | pg/ml | 9.3E-1 | 1.6E0 | 6.1E0 | 8.8E0 | 1.1E1 | 2.1E1 | 1.0E-9 | 1.0E-9 | 8.8E1 | 1.5E2 | 247 | 109 | 247 | 109 | 0.53 |
| Mh | pg/ml | 1.0E-9 | 1.0E-9 | 1.2E0 | 1.1E0 | 8.5E0 | 4.6E0 | 1.0E-9 | 1.0E-9 | 1.1E2 | 4.2E1 | 247 | 109 | 247 | 109 | 0.58 |
| Mi | pg/ml | 1.0E-9 | 1.0E-9 | 7.9E-1 | 1.1E1 | 8.2E0 | 5.9E1 | 1.0E-9 | 1.0E-9 | 1.2E2 | 5.2E2 | 247 | 109 | 247 | 109 | 0.55 |
| Mj | pg/ml | 1.0E-9 | 1.0E-9 | 6.2E0 | 9.5E0 | 3.5E1 | 3.0E1 | 1.0E-9 | 1.0E-9 | 4.6E2 | 2.1E2 | 247 | 109 | 247 | 109 | 0.54 |
| Mk | pg/ml | 2.0E0 | 2.7E0 | 1.3E1 | 2.4E1 | 6.8E1 | 1.4E2 | 1.0E-9 | 1.0E-9 | 1.0E3 | 1.3E3 | 247 | 109 | 247 | 109 | 0.50 |
| Ml | pg/ml | 1.0E-9 | 1.0E-9 | 1.1E1 | 1.2E1 | 1.3E2 | 6.0E1 | 1.0E-9 | 1.0E-9 | 2.1E3 | 5.1E2 | 247 | 109 | 247 | 109 | 0.53 |
| Mm | pg/ml | 4.4E2 | 7.9E2 | 9.3E2 | 1.5E3 | 1.2E3 | 1.8E3 | 1.0E-9 | 1.0E-9 | 1.0E4 | 1.0E4 | 247 | 109 | 247 | 109 | 0.60 |
| Mn | pg/ml | 5.0E0 | 9.9E0 | 1.1E1 | 1.2E1 | 2.8E1 | 1.1E1 | 1.0E-9 | 1.0E-9 | 3.5E2 | 6.8E1 | 247 | 109 | 247 | 109 | 0.67 |

| Code | Units | Median | | Average | | Standard Deviation | | Minimum | | Maximum | | Number of Samples | | Number of Patients | | AUC |
|------|-------|--------|------|---------|------|--------|------|---------|------|---------|------|--------|------|--------|------|-----|
| | | NonDis | Dis | NonDis | Dis | NonDis | Dis | NonDis | Dis | NonDis | Dis | NonDis | Dis | NonDis | Dis | |
| Mp | pg/ml | 1.0E-9 | 1.0E-9 | 8.4E0 | 5.0E1 | 2.1E1 | 2.4E2 | 1.0E-9 | 1.0E-9 | 1.8E2 | 2.4E3 | 247 | 109 | 247 | 109 | 0.56 |
| Mq | pg/ml | 1.0E-9 | 1.0E-9 | 2.1E0 | 4.4E0 | 1.3E1 | 1.7E1 | 1.0E-9 | 1.0E-9 | 1.8E2 | 1.0E2 | 247 | 109 | 247 | 109 | 0.52 |
| Mr | pg/ml | 1.0E-9 | 1.0E-9 | 1.8E1 | 1.0E2 | 9.8E1 | 4.4E2 | 1.0E-9 | 1.0E-9 | 1.4E3 | 3.4E3 | 247 | 109 | 247 | 109 | 0.58 |
| Ms | pg/ml | 3.4E2 | 2.7E2 | 4.8E2 | 4.4E2 | 5.4E2 | 6.2E2 | 1.0E-9 | 1.0E-9 | 3.2E3 | 4.7E3 | 247 | 109 | 247 | 109 | 0.45 |
| Mt | pg/ml | 1.0E-9 | 9.5E-1 | 7.5E0 | 4.7E1 | 4.8E1 | 3.1E2 | 1.0E-9 | 1.0E-9 | 7.1E2 | 3.2E3 | 247 | 109 | 247 | 109 | 0.61 |
| Mu | pg/ml | 1.0E-9 | 1.0E-9 | 8.6E-1 | 3.6E0 | 7.2E0 | 2.3E1 | 1.0E-9 | 1.0E-9 | 9.9E1 | 2.3E2 | 247 | 109 | 247 | 109 | 0.53 |
| Mv | pg/ml | 1.0E-9 | 1.0E-9 | 6.9E1 | 7.5E1 | 3.8E2 | 2.0E2 | 1.0E-9 | 1.0E-9 | 4.5E3 | 9.2E2 | 247 | 109 | 247 | 109 | 0.54 |
| Mw | pg/ml | 3.2E1 | 5.9E1 | 3.0E2 | 3.3E2 | 1.6E3 | 8.7E2 | 1.0E-9 | 1.0E-9 | 1.8E4 | 5.3E3 | 247 | 109 | 247 | 109 | 0.57 |
| Mx | pg/ml | 1.0E-9 | 1.0E-9 | 1.9E-1 | 9.8E-1 | 8.1E-1 | 3.7E0 | 1.0E-9 | 1.0E-9 | 9.2E0 | 3.2E1 | 247 | 109 | 247 | 109 | 0.59 |
| My | pg/ml | 1.0E-9 | 1.0E-9 | 3.8E2 | 2.0E2 | 2.8E3 | 8.3E2 | 1.0E-9 | 1.0E-9 | 3.9E4 | 8.0E3 | 247 | 109 | 247 | 109 | 0.49 |
| Mz | pg/ml | 9.1E0 | 2.0E1 | 2.2E1 | 7.0E1 | 4.9E1 | 2.2E2 | 1.0E-9 | 1.0E-9 | 5.5E2 | 1.9E3 | 247 | 109 | 247 | 109 | 0.67 |
| Na | pg/ml | 1.0E-9 | 1.0E-9 | 7.4E-1 | 8.9E-1 | 2.0E0 | 4.3E0 | 1.0E-9 | 1.0E-9 | 7.8E0 | 4.2E1 | 247 | 109 | 247 | 109 | 0.51 |
| Nb | pg/ml | 2.1E0 | 2.5E0 | 3.8E0 | 6.9E0 | 1.1E1 | 2.3E1 | 1.0E-9 | 1.0E-9 | 1.5E2 | 2.1E2 | 247 | 109 | 247 | 109 | 0.57 |
| Nc | pg/ml | 3.7E2 | 2.1E2 | 5.6E2 | 3.9E2 | 8.1E2 | 5.0E2 | 1.0E-9 | 1.0E-9 | 9.2E3 | 3.2E3 | 247 | 109 | 247 | 109 | 0.43 |
| Nd | pg/ml | 3.0E1 | 1.3E1 | 2.5E1 | 5.3E1 | 1.7E1 | 2.3E2 | 1.0E-9 | 1.0E-9 | 7.8E1 | 2.1E3 | 247 | 109 | 247 | 109 | 0.46 |
| Ne | pg/ml | 4.5E2 | 3.4E2 | 5.6E2 | 4.2E2 | 6.0E2 | 4.4E2 | 1.0E-9 | 1.0E-9 | 7.0E3 | 3.6E3 | 247 | 109 | 247 | 109 | 0.41 |
| Nf | pg/ml | 1.0E-9 | 1.0E-9 | 2.7E0 | 3.8E0 | 9.4E0 | 1.8E1 | 1.0E-9 | 1.0E-9 | 8.2E1 | 1.3E2 | 247 | 109 | 247 | 109 | 0.47 |
| Ng | pg/ml | 1.3E1 | 9.6E0 | 9.4E1 | 8.0E1 | 2.0E2 | 1.4E2 | 1.0E-9 | 1.0E-9 | 1.6E3 | 8.5E2 | 247 | 109 | 247 | 109 | 0.51 |
| Nh | pg/ml | 6.4E1 | 5.3E1 | 8.7E1 | 6.3E1 | 7.9E1 | 6.4E1 | 1.0E-9 | 3.6E0 | 5.6E2 | 5.1E2 | 247 | 109 | 247 | 109 | 0.40 |
| Ni | pg/ml | 1.0E-9 | 1.0E-9 | 7.8E1 | 1.0E2 | 1.3E2 | 1.6E2 | 1.0E-9 | 1.0E-9 | 7.1E2 | 1.1E3 | 247 | 109 | 247 | 109 | 0.51 |
| Nj | pg/ml | 8.7E0 | 4.7E0 | 1.2E1 | 7.4E0 | 1.2E1 | 8.2E0 | 1.0E-9 | 1.0E-9 | 6.9E1 | 4.6E1 | 247 | 109 | 247 | 109 | 0.37 |
| Nk | pg/ml | 1.8E1 | 1.4E1 | 3.3E1 | 3.0E1 | 3.8E1 | 4.0E1 | 1.0E-9 | 1.0E-9 | 2.0E2 | 2.0E2 | 247 | 109 | 247 | 109 | 0.48 |
| Nl | pg/ml | 4.6E1 | 3.3E1 | 6.3E1 | 4.1E1 | 8.5E1 | 4.0E1 | 1.0E-9 | 1.0E-9 | 1.1E3 | 2.3E2 | 247 | 109 | 247 | 109 | 0.40 |
| Hl | pg/ml | 4.2E0 | 9.6E0 | 3.5E1 | 1.6E2 | 6.2E1 | 7.0E2 | 1.0E-9 | 1.0E-9 | 3.0E2 | 3.6E3 | 32 | 26 | 32 | 26 | 0.50 |
| Ho | pg/ml | 1.5E1 | 2.0E1 | 2.1E1 | 4.8E1 | 2.1E1 | 8.0E1 | 1.0E-9 | 7.6E0 | 8.3E1 | 3.9E2 | 32 | 26 | 32 | 26 | 0.66 |
| Hp | ng/ml | 1.7E0 | 1.6E0 | 1.4E2 | 1.0E2 | 3.3E2 | 2.9E2 | 3.6E-1 | 1.0E-9 | 8.9E2 | 8.9E2 | 32 | 26 | 32 | 26 | 0.48 |
| Tz | pg/ml | 3.5E3 | 6.9E3 | 5.9E3 | 5.5E4 | 7.6E3 | 2.8E5 | 7.4E1 | 1.0E-9 | 5.3E4 | 2.1E6 | 80 | 55 | 80 | 55 | 0.61 |
| Ua | pg/ml | 3.2E3 | 3.7E3 | 4.1E4 | 1.3E4 | 2.4E5 | 2.4E4 | 2.3E2 | 1.0E-9 | 2.1E6 | 1.2E5 | 80 | 55 | 80 | 55 | 0.51 |
| Ub | pg/ml | 6.0E2 | 4.1E2 | 9.7E2 | 6.8E2 | 1.3E3 | 8.3E2 | 1.6E1 | 1.0E-9 | 9.8E3 | 4.4E3 | 80 | 55 | 80 | 55 | 0.41 |
| Ue | pg/ml | 3.1E1 | 2.4E1 | 3.8E1 | 3.4E1 | 3.5E1 | 3.1E1 | 5.2E0 | 9.8E-2 | 2.7E2 | 1.4E2 | 80 | 55 | 80 | 55 | 0.43 |
| Uc | pg/ml | 7.0E2 | 7.9E2 | 1.2E3 | 2.6E3 | 1.3E3 | 7.8E3 | 2.2E1 | 1.0E-9 | 8.3E3 | 5.7E4 | 80 | 55 | 80 | 55 | 0.51 |
| Ud | pg/ml | 1.0E-9 | 1.0E-9 | 4.9E0 | 9.7E-1 | 4.4E1 | 7.2E0 | 1.0E-9 | 1.0E-9 | 3.9E2 | 5.3E1 | 80 | 55 | 80 | 55 | 0.50 |
| Hq | pg/ml | 1.1E0 | 1.3E0 | 9.2E1 | 3.0E2 | 1.3E3 | 2.7E3 | 1.0E-9 | 1.0E-9 | 2.0E4 | 2.8E4 | 247 | 107 | 247 | 107 | 0.51 |
| Hr | pg/ml | 9.8E1 | 8.5E1 | 7.1E2 | 5.2E2 | 1.4E3 | 1.3E3 | 1.0E-9 | 1.0E-9 | 1.2E4 | 8.9E3 | 247 | 107 | 247 | 107 | 0.47 |
| Hu | pg/ml | 1.0E-9 | 2.2E1 | 5.2E3 | 3.0E3 | 4.4E4 | 2.5E4 | 1.0E-9 | 1.0E-9 | 6.3E5 | 2.6E5 | 247 | 107 | 247 | 107 | 0.56 |
| Hv | pg/ml | 1.4E0 | 1.6E0 | 2.5E0 | 1.3E1 | 6.3E0 | 8.8E1 | 1.0E-9 | 1.0E-9 | 6.9E1 | 8.9E2 | 247 | 107 | 247 | 107 | 0.54 |
| Hw | pg/ml | 6.4E0 | 6.1E0 | 1.6E1 | 1.1E2 | 5.1E1 | 9.0E2 | 1.0E-9 | 1.0E-9 | 6.4E2 | 9.4E3 | 247 | 107 | 247 | 107 | 0.49 |
| Hx | pg/ml | 8.4E0 | 1.1E1 | 6.2E1 | 4.8E1 | 5.9E2 | 1.5E2 | 1.0E-9 | 1.0E-9 | 9.3E3 | 1.3E3 | 247 | 107 | 247 | 107 | 0.54 |
| Ib | ng/ml | 4.5E-2 | 2.6E-2 | 1.5E0 | 1.3E0 | 5.6E0 | 7.7E0 | 1.0E-9 | 1.0E-9 | 3.6E1 | 5.6E1 | 80 | 53 | 80 | 53 | 0.44 |
| Ic | U/ml | 2.2E2 | 2.2E2 | 1.2E3 | 2.0E2 | 7.3E3 | 1.6E2 | 2.4E0 | 1.1E1 | 6.5E4 | 7.9E2 | 80 | 53 | 80 | 53 | 0.49 |
| Id | U/ml | 5.8E-1 | 8.2E-1 | 1.0E0 | 1.0E1 | 1.3E0 | 5.9E1 | 1.0E-9 | 1.0E-9 | 6.9E0 | 4.3E2 | 80 | 53 | 80 | 53 | 0.60 |
| Tt | pg/ml | 1.7E2 | 1.7E2 | 1.7E2 | 1.8E2 | 5.0E1 | 6.7E1 | 4.3E1 | 7.3E1 | 3.0E2 | 4.4E2 | 73 | 51 | 73 | 51 | 0.53 |
| To | pg/ml | 1.8E0 | 1.4E0 | 2.4E0 | 1.9E0 | 3.1E0 | 2.4E0 | 1.0E-9 | 1.0E-9 | 2.3E1 | 1.2E1 | 78 | 53 | 78 | 53 | 0.43 |
| Tr | pg/ml | 3.6E0 | 3.2E0 | 5.4E0 | 1.4E1 | 6.3E0 | 4.4E1 | 1.0E-9 | 1.0E-9 | 3.8E1 | 3.1E2 | 76 | 52 | 76 | 52 | 0.53 |
| Tn | pg/ml | 3.1E1 | 4.2E1 | 9.2E1 | 2.0E2 | 2.5E2 | 4.8E2 | 1.0E-9 | 2.4E0 | 1.7E3 | 2.3E3 | 78 | 53 | 78 | 53 | 0.56 |
| Tv | ng/ml | 1.3E1 | 1.1E1 | 2.1E1 | 1.7E2 | 3.9E1 | 9.7E2 | 1.0E-9 | 1.0E-9 | 3.2E2 | 7.1E3 | 78 | 53 | 78 | 53 | 0.48 |
| Ih | ng/ml | 5.6E1 | 1.1E2 | 2.2E2 | 3.8E2 | 3.8E2 | 6.1E2 | 1.0E-9 | 1.0E-9 | 2.5E3 | 3.6E3 | 247 | 108 | 247 | 108 | 0.60 |
| Ii | ng/ml | 6.5E1 | 1.1E2 | 2.1E2 | 2.7E2 | 5.3E2 | 5.9E2 | 7.3E-1 | 7.5E-1 | 5.2E3 | 4.5E3 | 247 | 108 | 247 | 108 | 0.59 |
| Ij | ng/ml | 7.1E1 | 1.1E2 | 1.8E2 | 4.2E2 | 6.5E2 | 2.4E3 | 2.8E0 | 9.5E0 | 6.4E3 | 2.4E4 | 245 | 106 | 245 | 106 | 0.64 |
| Ik | ng/ml | 8.0E0 | 2.0E1 | 1.7E3 | 2.4E2 | 1.4E4 | 4.4E2 | 5.9E-1 | 6.2E-1 | 1.2E5 | 1.8E3 | 244 | 107 | 244 | 107 | 0.61 |
| Il | ng/ml | 3.6E2 | 4.2E2 | 1.3E3 | 1.6E3 | 2.9E3 | 3.1E3 | 1.0E-9 | 1.0E-9 | 1.2E4 | 1.2E4 | 243 | 106 | 243 | 106 | 0.55 |
| Im | ng/ml | 1.9E2 | 3.1E2 | 3.4E2 | 8.8E2 | 5.1E2 | 1.8E3 | 1.4E1 | 2.2E1 | 6.0E3 | 1.5E4 | 244 | 107 | 244 | 107 | 0.66 |
| In | ng/ml | 3.4E0 | 3.4E0 | 2.2E1 | 6.2E1 | 1.0E2 | 4.4E2 | 1.0E-9 | 1.0E-9 | 1.1E3 | 4.5E3 | 247 | 108 | 247 | 108 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Hb | ng/ml | 2.1E1 | 3.0E1 | 3.1E1 | 4.1E1 | 3.2E1 | 4.1E1 | 1.6E0 | 4.8E-1 | 2.0E2 | 1.9E2 | 79 | 57 | 79 | 57 | 0.57 |
| Hc | pg/ml | 7.2E2 | 5.8E2 | 3.9E3 | 2.1E3 | 1.2E4 | 7.0E3 | 1.0E-9 | 1.0E-9 | 1.0E5 | 5.0E4 | 79 | 57 | 79 | 57 | 0.43 |
| Hf | ng/ml | 1.6E2 | 2.5E2 | 3.6E2 | 4.4E2 | 5.1E2 | 5.9E2 | 1.0E-9 | 1.0E-9 | 2.5E3 | 3.2E3 | 79 | 57 | 79 | 57 | 0.57 |
| Io | ng/ml | 7.0E3 | 1.2E4 | 1.8E4 | 2.2E4 | 5.1E4 | 2.9E4 | 1.2E2 | 1.0E-9 | 7.1E5 | 2.0E5 | 246 | 109 | 246 | 109 | 0.60 |
| Ip | ng/ml | 8.1E0 | 3.0E1 | 2.0E1 | 2.6E1 | 2.8E1 | 2.2E1 | 1.0E-9 | 1.0E-9 | 2.3E2 | 7.8E1 | 246 | 109 | 246 | 109 | 0.60 |
| Iq | ug/ml | 1.0E-1 | 1.4E-1 | 5.6E1 | 3.4E0 | 8.7E2 | 2.1E1 | 1.0E-9 | 1.0E-9 | 1.4E4 | 2.2E2 | 246 | 109 | 246 | 109 | 0.55 |
| Ir | ug/ml | 3.4E-1 | 7.6E-1 | 4.4E0 | 1.1E1 | 3.6E1 | 4.2E1 | 1.0E-9 | 1.0E-9 | 5.1E2 | 3.7E2 | 245 | 109 | 245 | 109 | 0.63 |
| Is | ng/ml | 1.6E0 | 3.8E0 | 7.7E0 | 1.8E1 | 3.6E1 | 3.7E1 | 1.0E-9 | 1.0E-9 | 5.5E2 | 2.6E2 | 246 | 109 | 246 | 109 | 0.62 |
| It | ng/ml | 1.7E0 | 2.9E0 | 2.2E1 | 2.8E1 | 1.0E2 | 1.2E2 | 1.0E-9 | 1.0E-9 | 1.1E3 | 8.3E2 | 246 | 109 | 246 | 109 | 0.58 |
| Iu | ng/ml | 1.7E2 | 1.5E2 | 1.4E3 | 1.6E3 | 4.3E3 | 4.7E3 | 1.0E-9 | 1.0E-9 | 2.9E4 | 2.4E4 | 246 | 109 | 246 | 109 | 0.50 |
| Iv | ng/ml | 9.5E0 | 2.2E1 | 9.8E1 | 1.6E2 | 1.0E3 | 6.2E2 | 1.0E-9 | 1.0E-9 | 1.6E4 | 3.8E3 | 245 | 109 | 245 | 109 | 0.64 |
| Iz | ng/ml | 1.3E2 | 1.2E2 | 4.6E2 | 2.4E2 | 8.9E2 | 3.2E2 | 1.5E0 | 8.8E-1 | 6.1E3 | 1.7E3 | 79 | 57 | 79 | 57 | 0.45 |
| Yg | pg/ml | 2.5E2 | 2.7E2 | 2.3E3 | 1.0E3 | 9.2E3 | 1.8E3 | 1.0E-9 | 1.0E-9 | 5.0E4 | 8.2E3 | 29 | 25 | 29 | 25 | 0.53 |
| Yh | pg/ml | 2.1E2 | 2.1E2 | 3.8E2 | 5.3E2 | 4.4E2 | 7.5E2 | 1.0E-9 | 1.0E-9 | 1.8E3 | 3.0E3 | 29 | 25 | 29 | 25 | 0.52 |
| Yi | pg/ml | 2.4E2 | 4.5E2 | 5.0E2 | 2.0E3 | 5.4E2 | 5.4E3 | 1.0E-9 | 1.0E-9 | 2.0E3 | 2.6E4 | 29 | 25 | 29 | 25 | 0.57 |
| Yk | U/ml | 1.0E-9 | 1.0E-9 | 2.4E-1 | 2.1E-1 | 6.8E-1 | 5.2E-1 | 1.0E-9 | 1.0E-9 | 3.0E0 | 2.0E0 | 29 | 25 | 29 | 25 | 0.50 |
| Yj | pg/ml | 1.6E2 | 1.1E2 | 3.6E2 | 2.2E2 | 6.1E2 | 3.0E2 | 9.7E0 | 1.0E-9 | 3.2E3 | 1.5E3 | 29 | 25 | 29 | 25 | 0.42 |
| Yd | ng/ml | 1.7E-1 | 2.8E-1 | 3.8E-1 | 4.2E-1 | 4.9E-1 | 5.6E-1 | 6.6E-3 | 1.6E-2 | 1.8E0 | 2.3E0 | 32 | 26 | 32 | 26 | 0.53 |
| Wb | pg/ml | 2.6E4 | 3.5E4 | 3.0E4 | 6.4E4 | 1.9E4 | 1.2E5 | 4.9E3 | 7.5E3 | 8.4E4 | 6.4E5 | 32 | 26 | 32 | 26 | 0.66 |
| Vz | pg/ml | 3.7E0 | 3.2E0 | 4.6E0 | 4.8E0 | 3.8E0 | 5.5E0 | 1.0E-9 | 7.6E-2 | 1.6E1 | 2.2E1 | 32 | 26 | 32 | 26 | 0.48 |
| Si | ng/ml | 1.2E0 | 1.2E0 | 2.2E0 | 1.7E0 | 3.0E0 | 1.6E0 | 1.1E-1 | 8.6E-3 | 1.0E1 | 6.0E0 | 32 | 26 | 32 | 26 | 0.53 |
| Sf | mIU/mL | 1.7E1 | 1.6E1 | 6.7E1 | 2.0E1 | 1.4E2 | 1.9E1 | 6.2E-1 | 1.3E0 | 7.2E2 | 8.3E1 | 32 | 26 | 32 | 26 | 0.44 |
| Sh | mIU/mL | 1.7E1 | 1.2E1 | 5.9E1 | 1.8E1 | 1.1E2 | 1.8E1 | 1.8E-1 | 7.8E-2 | 5.7E2 | 6.1E1 | 32 | 26 | 32 | 26 | 0.44 |
| Sj | ng/ml | 3.8E-1 | 4.4E-1 | 4.1E-1 | 4.5E-1 | 9.3E-2 | 9.3E-2 | 2.5E-1 | 3.2E-1 | 6.1E-1 | 7.2E-1 | 32 | 26 | 32 | 26 | 0.61 |
| Rc | pg/ml | 7.0E3 | 5.5E3 | 8.2E3 | 7.1E3 | 5.6E3 | 6.2E3 | 3.9E2 | 5.5E2 | 2.8E4 | 3.9E4 | 80 | 55 | 80 | 55 | 0.43 |
| Rb | pg/ml | 7.5E-1 | 7.1E-1 | 2.5E0 | 3.8E0 | 3.8E0 | 8.3E0 | 1.0E-9 | 1.0E-9 | 1.6E1 | 5.6E1 | 80 | 55 | 80 | 55 | 0.52 |
| Zq | 2.6ng/ml | 1.8E2 | 3.4E2 | 2.7E2 | 3.5E2 | 2.3E2 | 2.0E2 | 1.4E1 | 1.7E1 | 9.7E2 | 9.7E2 | 31 | 25 | 31 | 25 | 0.64 |
| Zw | 2.5ng/ml | 4.2E0 | 5.4E0 | 9.5E0 | 1.1E1 | 1.2E1 | 1.7E1 | 1.4E-1 | 2.4E-1 | 5.9E1 | 6.3E1 | 32 | 26 | 32 | 26 | 0.48 |
| Zx | 2.3mU/ml | 8.6E-2 | 1.4E-1 | 2.7E-1 | 3.2E-1 | 5.1E-1 | 5.2E-1 | 3.2E-2 | 5.0E-2 | 2.9E0 | 2.1E0 | 32 | 26 | 32 | 26 | 0.59 |
| Pz | ng/ml | 3.1E3 | 5.2E3 | 5.4E3 | 6.1E3 | 6.5E3 | 4.5E3 | 1.6E1 | 4.0E1 | 7.0E4 | 2.5E4 | 245 | 106 | 245 | 106 | 0.59 |
| Qa | ng/ml | 3.0E3 | 6.5E3 | 6.1E3 | 1.2E4 | 7.4E3 | 2.3E4 | 1.5E2 | 4.2E2 | 4.2E4 | 2.2E5 | 245 | 106 | 245 | 106 | 0.64 |
| Qb | ng/ml | 9.7E1 | 1.5E2 | 2.1E2 | 3.1E2 | 4.3E2 | 4.6E2 | 7.9E-1 | 6.7E0 | 5.3E3 | 4.1E3 | 245 | 106 | 245 | 106 | 0.62 |
| Qc | ng/ml | 1.7E2 | 3.7E2 | 4.0E2 | 5.9E2 | 5.6E2 | 6.5E2 | 1.0E-9 | 1.0E-9 | 3.8E3 | 4.3E3 | 245 | 106 | 245 | 106 | 0.62 |
| Qd | ng/ml | 7.5E3 | 1.4E4 | 2.3E4 | 3.9E4 | 1.3E5 | 6.4E4 | 1.5E2 | 1.2E3 | 2.0E6 | 4.3E5 | 245 | 106 | 245 | 106 | 0.67 |
| Qe | ng/ml | 7.6E2 | 1.8E3 | 2.0E3 | 2.6E3 | 6.5E3 | 2.7E3 | 1.0E-9 | 8.8E0 | 9.7E4 | 1.8E4 | 245 | 106 | 245 | 106 | 0.66 |
| Jd | ng/ml | 6.9E-1 | 1.4E0 | 4.8E0 | 2.9E0 | 1.8E1 | 4.4E0 | 1.0E-9 | 1.0E-9 | 1.2E2 | 2.1E1 | 80 | 55 | 80 | 55 | 0.59 |
| Jc | ng/ml | 1.0E-9 | 1.0E-9 | 1.9E0 | 1.6E0 | 6.0E0 | 2.5E0 | 1.0E-9 | 1.0E-9 | 4.6E1 | 1.1E1 | 80 | 55 | 80 | 55 | 0.55 |
| Jf | ng/ml | 1.0E-9 | 1.0E-9 | 9.7E-1 | 1.5E0 | 2.2E0 | 2.3E0 | 1.0E-9 | 1.0E-9 | 1.2E1 | 9.1E0 | 80 | 55 | 80 | 55 | 0.56 |
| Jg | ng/ml | 3.6E2 | 7.8E2 | 7.0E2 | 1.1E3 | 9.5E2 | 1.1E3 | 5.8E0 | 1.3E1 | 1.0E4 | 7.1E3 | 247 | 107 | 247 | 107 | 0.65 |
| Jh | ng/ml | 2.4E0 | 4.9E0 | 2.2E1 | 2.6E1 | 9.4E1 | 6.5E1 | 1.0E-9 | 1.0E-9 | 1.2E3 | 4.9E2 | 247 | 107 | 247 | 107 | 0.59 |
| Ji | ng/ml | 4.7E1 | 8.5E1 | 6.8E1 | 1.6E2 | 7.1E1 | 1.9E2 | 1.1E0 | 8.9E0 | 5.3E2 | 1.3E3 | 247 | 107 | 247 | 107 | 0.70 |
| Sr | pg/mL | 3.2E2 | 7.0E2 | 7.5E2 | 1.5E3 | 1.1E3 | 3.0E3 | 1.0E-9 | 1.0E-9 | 5.5E3 | 2.1E4 | 80 | 54 | 80 | 54 | 0.64 |
| Ss | pg/mL | 9.7E4 | 7.4E4 | 1.5E5 | 1.4E5 | 1.6E5 | 2.1E5 | 9.1E3 | 2.7E3 | 7.1E5 | 1.3E6 | 80 | 54 | 80 | 54 | 0.45 |
| St | pg/mL | 2.2E7 | 4.2E7 | 4.6E7 | 1.1E8 | 6.5E7 | 2.7E8 | 7.8E5 | 1.0E-9 | 4.1E8 | 1.7E9 | 78 | 55 | 78 | 55 | 0.58 |
| Wc | ng/ml | 1.0E-9 | 2.1E-3 | 6.0E-2 | 1.3E-1 | 1.2E-1 | 3.6E-1 | 1.0E-9 | 1.0E-9 | 5.2E-1 | 1.8E0 | 32 | 26 | 32 | 26 | 0.55 |
| Wd | ng/ml | 9.9E0 | 8.8E0 | 3.2E1 | 4.8E1 | 6.6E1 | 1.1E2 | 1.0E0 | 1.5E0 | 2.9E2 | 4.1E2 | 32 | 26 | 32 | 26 | 0.50 |
| We | ng/ml | 2.7E-1 | 4.9E-1 | 1.0E0 | 1.8E0 | 1.4E0 | 4.7E0 | 1.0E-9 | 1.0E-9 | 5.5E0 | 2.3E1 | 32 | 26 | 32 | 26 | 0.53 |
| Wg | ng/ml | 1.0E-9 | 1.0E-9 | 5.1E-4 | 2.1E-2 | 2.9E-3 | 1.0E-1 | 1.0E-9 | 1.0E-9 | 1.6E-2 | 5.3E-1 | 32 | 26 | 32 | 26 | 0.50 |
| Wh | ng/ml | 1.0E-2 | 1.0E-2 | 4.9E-2 | 4.4E-2 | 9.8E-2 | 1.0E-1 | 1.0E-9 | 1.0E-9 | 3.6E-1 | 4.2E-1 | 32 | 26 | 32 | 26 | 0.51 |
| Wf | ng/ml | 1.0E-9 | 1.0E-9 | 1.0E-1 | 1.6E-1 | 2.4E-1 | 4.6E-1 | 1.0E-9 | 1.0E-9 | 1.2E0 | 2.3E0 | 32 | 26 | 32 | 26 | 0.53 |
| Ra | pg/ml | 1.0E-9 | 1.0E-9 | 4.5E-1 | 1.5E0 | 9.9E-1 | 8.6E0 | 1.0E-9 | 1.0E-9 | 3.8E0 | 6.4E1 | 80 | 55 | 80 | 55 | 0.47 |
| Qz | pg/ml | 9.3E0 | 1.1E1 | 5.6E1 | 4.5E1 | 9.5E1 | 7.0E1 | 1.0E-9 | 1.0E-9 | 4.5E2 | 2.8E2 | 80 | 55 | 80 | 55 | 0.52 |
| Qy | pg/ml | 3.8E-1 | 4.6E-1 | 5.7E0 | 2.2E1 | 2.8E1 | 1.1E2 | 1.0E-9 | 1.0E-9 | 2.3E2 | 7.3E2 | 80 | 55 | 80 | 55 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qx | pg/ml | 1.0E-9 | 1.0E-9 | 3.9E0 | 3.4E0 | 2.2E1 | 1.5E1 | 1.0E-9 | 1.0E-9 | 1.9E2 | 1.1E2 | 80 | 55 | 80 | 55 | 0.48 |
| Qw | pg/ml | 1.0E-9 | 1.0E-9 | 3.2E0 | 2.2E0 | 9.5E0 | 9.3E0 | 1.0E-9 | 1.0E-9 | 5.6E1 | 6.6E1 | 80 | 55 | 80 | 55 | 0.47 |
| Qv | pg/ml | 2.0E4 | 1.2E4 | 4.3E4 | 2.6E4 | 1.1E5 | 4.6E4 | 1.4E3 | 1.0E-9 | 9.4E5 | 3.3E5 | 80 | 55 | 80 | 55 | 0.40 |
| Qu | pg/ml | 1.0E1 | 1.0E-9 | 8.9E1 | 9.6E1 | 1.7E2 | 2.0E2 | 1.0E-9 | 1.0E-9 | 7.1E2 | 9.8E2 | 80 | 55 | 80 | 55 | 0.47 |
| Qt | pg/ml | 1.2E1 | 1.4E1 | 5.1E1 | 3.6E1 | 1.2E2 | 6.2E1 | 1.0E-9 | 1.0E-9 | 7.1E2 | 2.2E2 | 80 | 55 | 80 | 55 | 0.50 |
| Qh | ng/ml | 1.7E1 | 2.3E1 | 3.6E1 | 7.0E1 | 5.5E1 | 1.3E2 | 2.5E-1 | 4.3E-1 | 3.4E2 | 8.0E2 | 80 | 55 | 80 | 55 | 0.59 |
| Qg | ng/ml | 7.9E0 | 7.1E0 | 1.6E1 | 1.1E1 | 3.2E1 | 1.4E1 | 1.5E-1 | 3.0E-1 | 2.7E2 | 8.1E1 | 80 | 55 | 80 | 55 | 0.44 |
| Jj | ng/ml | 5.5E2 | 3.9E2 | 2.4E3 | 6.0E2 | 2.2E4 | 6.1E2 | 2.3E0 | 8.7E0 | 3.4E5 | 3.4E3 | 247 | 107 | 247 | 107 | 0.41 |
| Jk | ng/ml | 2.6E0 | 2.9E0 | 2.0E1 | 2.8E1 | 4.5E1 | 5.8E1 | 1.0E-9 | 4.3E-2 | 2.8E2 | 3.9E2 | 247 | 107 | 247 | 107 | 0.54 |
| Jl | ng/ml | 4.8E-1 | 6.5E-1 | 1.5E0 | 9.7E1 | 3.5E0 | 9.6E2 | 1.2E-3 | 5.4E-3 | 2.0E1 | 9.9E3 | 247 | 107 | 247 | 107 | 0.60 |
| Jm | ng/ml | 1.6E1 | 3.2E1 | 6.4E1 | 7.7E1 | 1.5E2 | 2.1E2 | 1.0E-9 | 1.0E-9 | 1.4E3 | 2.1E3 | 247 | 107 | 247 | 107 | 0.58 |
| Jn | pg/ml | 2.5E-1 | 5.2E-1 | 3.9E0 | 1.7E1 | 4.0E1 | 9.5E1 | 1.0E-9 | 1.0E-9 | 6.2E2 | 7.3E2 | 247 | 107 | 247 | 107 | 0.62 |
| Jo | pg/ml | 3.5E3 | 4.5E3 | 4.7E3 | 6.4E3 | 3.9E3 | 1.1E4 | 2.0E1 | 2.4E1 | 2.4E4 | 1.0E5 | 247 | 107 | 247 | 107 | 0.55 |
| Jp | pg/ml | 6.7E4 | 8.4E4 | 6.9E4 | 9.0E4 | 3.6E4 | 4.5E4 | 5.8E2 | 4.6E3 | 1.9E5 | 3.8E5 | 247 | 107 | 247 | 107 | 0.66 |
| Jq | pg/ml | 9.2E1 | 1.4E2 | 1.5E2 | 3.4E2 | 2.0E2 | 9.3E2 | 1.0E0 | 5.6E0 | 2.0E3 | 8.7E3 | 247 | 107 | 247 | 107 | 0.58 |
| Jr | pg/ml | 2.7E0 | 8.3E0 | 6.6E1 | 1.6E2 | 6.9E2 | 8.9E2 | 1.0E-9 | 1.0E-9 | 1.1E4 | 7.4E3 | 247 | 107 | 247 | 107 | 0.63 |
| Js | pg/ml | 1.3E1 | 1.9E1 | 7.4E1 | 1.3E2 | 6.5E2 | 4.9E2 | 1.0E-9 | 1.9E0 | 1.0E4 | 3.0E3 | 247 | 107 | 247 | 107 | 0.63 |
| Jt | pg/ml | 2.2E3 | 3.1E3 | 2.8E3 | 4.7E3 | 2.2E3 | 6.9E3 | 2.2E1 | 1.5E2 | 2.2E4 | 5.2E4 | 247 | 107 | 247 | 107 | 0.61 |
| Xa | pg/ml | 1.0E-9 | 5.0E0 | 9.1E0 | 7.9E1 | 1.9E1 | 2.6E2 | 1.0E-9 | 1.0E-9 | 9.6E1 | 1.2E3 | 32 | 26 | 32 | 26 | 0.62 |
| Ye | pg/ml | 1.0E-9 | 1.0E-9 | 6.5E0 | 1.1E0 | 1.7E1 | 3.1E0 | 1.0E-9 | 1.0E-9 | 6.9E1 | 1.4E1 | 32 | 26 | 32 | 26 | 0.42 |
| Tm | pg/ml | 1.0E-9 | 1.0E-9 | 2.8E0 | 2.3E0 | 4.9E0 | 3.8E0 | 1.0E-9 | 1.0E-9 | 1.8E1 | 8.4E0 | 32 | 26 | 32 | 26 | 0.49 |
| Tl | pg/ml | 1.1E-1 | 1.3E-1 | 2.5E-1 | 1.3E0 | 3.6E-1 | 4.8E0 | 1.0E-9 | 1.0E-9 | 1.2E0 | 2.5E1 | 32 | 26 | 32 | 26 | 0.61 |
| Ju | mIU/ml | 1.1E1 | 1.0E1 | 2.8E1 | 1.8E1 | 4.1E1 | 2.2E1 | 2.5E-1 | 1.7E-1 | 2.3E2 | 1.1E2 | 80 | 55 | 80 | 55 | 0.48 |
| Jv | mIU/ml | 1.7E1 | 1.3E1 | 5.0E1 | 2.2E1 | 7.5E1 | 2.7E1 | 2.4E-2 | 1.7E-2 | 4.4E2 | 1.4E2 | 80 | 55 | 80 | 55 | 0.42 |
| Jy | ng/ml | 1.8E-3 | 1.6E-3 | 2.0E-3 | 3.7E-3 | 1.1E-3 | 7.8E-3 | 1.0E-9 | 1.7E-4 | 4.7E-3 | 4.1E-2 | 80 | 55 | 80 | 55 | 0.48 |
| Kc | pg/ml | 2.2E1 | 2.5E1 | 4.1E1 | 5.2E1 | 4.7E1 | 6.6E1 | 1.0E-9 | 1.0E-9 | 1.9E2 | 3.2E2 | 79 | 57 | 79 | 57 | 0.56 |
| Kd | pg/ml | 1.0E-9 | 1.0E-9 | 1.7E2 | 1.1E3 | 6.5E2 | 5.1E3 | 1.0E-9 | 1.0E-9 | 4.8E3 | 3.8E4 | 79 | 57 | 79 | 57 | 0.53 |
| Ke | pg/ml | 1.2E4 | 1.5E4 | 1.3E4 | 2.6E4 | 8.7E3 | 4.4E4 | 1.0E3 | 6.7E2 | 4.6E4 | 3.2E5 | 79 | 57 | 79 | 57 | 0.62 |
| Kf | pg/mL | 5.9E0 | 6.7E0 | 6.5E0 | 9.0E0 | 5.1E0 | 1.2E1 | 1.0E-9 | 1.0E-9 | 2.2E1 | 7.8E1 | 79 | 57 | 79 | 57 | 0.54 |
| Kg | pg/mL | 8.9E2 | 1.0E3 | 1.8E3 | 2.7E3 | 2.8E3 | 6.0E3 | 7.7E1 | 1.3E2 | 2.2E4 | 3.6E4 | 79 | 57 | 79 | 57 | 0.50 |
| Ki | pg/ml | 5.7E1 | 7.3E1 | 6.4E1 | 8.1E1 | 5.0E1 | 5.6E1 | 1.0E-9 | 1.0E-9 | 2.9E2 | 2.5E2 | 79 | 57 | 79 | 57 | 0.62 |
| Kj | pg/ml | 8.4E2 | 7.7E2 | 1.4E3 | 1.5E3 | 1.5E3 | 2.2E3 | 6.6E1 | 3.3E1 | 8.8E3 | 1.5E4 | 79 | 57 | 79 | 57 | 0.47 |
| Kk | pg/ml | 6.8E0 | 8.5E0 | 1.2E1 | 1.7E1 | 1.5E1 | 1.8E1 | 1.0E-9 | 1.0E-9 | 8.1E1 | 6.1E1 | 79 | 57 | 79 | 57 | 0.59 |
| Kl | pg/ml | 2.1E4 | 1.6E4 | 2.9E4 | 2.5E4 | 2.6E4 | 2.6E4 | 4.1E2 | 2.3E2 | 1.1E5 | 1.3E5 | 79 | 57 | 79 | 57 | 0.45 |
| Kn | pg/ml | 2.9E1 | 3.0E1 | 5.7E1 | 1.7E2 | 8.3E1 | 6.5E2 | 1.0E-9 | 1.0E-9 | 3.8E2 | 4.9E3 | 79 | 57 | 79 | 57 | 0.56 |
| Ko | pg/ml | 3.4E2 | 4.0E2 | 4.4E2 | 6.2E2 | 4.6E2 | 8.0E2 | 1.0E-9 | 1.0E-9 | 2.0E3 | 4.1E3 | 79 | 57 | 79 | 57 | 0.57 |
| Kp | pg/ml | 3.4E2 | 3.9E2 | 3.4E2 | 6.2E2 | 2.5E2 | 1.7E3 | 1.0E-9 | 1.0E-9 | 9.4E2 | 1.3E4 | 79 | 57 | 79 | 57 | 0.57 |
| Kq | pg/ml | 3.2E2 | 4.2E2 | 3.7E2 | 3.7E3 | 3.4E2 | 2.1E4 | 5.1E0 | 1.6E0 | 2.1E3 | 1.6E5 | 76 | 55 | 76 | 55 | 0.62 |
| Kr | pg/ml | 1.0E-9 | 9.9E-1 | 1.5E0 | 1.0E1 | 2.8E0 | 5.6E1 | 1.0E-9 | 1.0E-9 | 1.3E1 | 4.2E2 | 76 | 55 | 76 | 55 | 0.60 |
| Ks | pg/ml | 1.4E4 | 1.9E4 | 2.0E4 | 2.2E4 | 1.8E4 | 1.8E4 | 4.5E2 | 5.1E1 | 7.9E4 | 5.1E4 | 76 | 55 | 76 | 55 | 0.53 |
| Ps | ng/ml | 1.5E2 | 2.9E2 | 5.6E2 | 1.2E3 | 1.6E3 | 2.8E3 | 1.6E0 | 5.5E0 | 9.0E3 | 1.2E4 | 32 | 26 | 32 | 26 | 0.59 |
| Kx | ng/ml | 1.4E-4 | 4.5E-3 | 6.0E-3 | 1.2E-2 | 1.4E-2 | 1.9E-2 | 1.0E-9 | 1.0E-9 | 1.0E-1 | 7.9E-2 | 78 | 57 | 78 | 57 | 0.59 |
| Ky | ng/ml | 1.1E-1 | 2.2E-1 | 3.5E-1 | 4.8E-1 | 7.7E-1 | 7.6E-1 | 1.0E-9 | 1.0E-9 | 5.1E0 | 4.4E0 | 78 | 57 | 78 | 57 | 0.58 |
| Kz | ng/ml | 1.0E-9 | 1.0E-9 | 3.9E-3 | 3.9E-3 | 5.9E-3 | 6.7E-3 | 1.0E-9 | 1.0E-9 | 1.6E-2 | 2.5E-2 | 78 | 57 | 78 | 57 | 0.48 |
| Rz | ng/ml | 3.8E-1 | 3.2E-1 | 9.4E-1 | 9.0E-1 | 1.4E0 | 1.5E0 | 1.1E-2 | 4.6E-3 | 6.7E0 | 7.5E0 | 32 | 26 | 32 | 26 | 0.52 |
| Ry | ng/ml | 1.6E-2 | 2.0E-2 | 2.1E-2 | 4.1E-2 | 1.7E-2 | 7.1E-2 | 1.0E-9 | 1.0E-9 | 6.5E-2 | 3.5E-1 | 32 | 26 | 32 | 26 | 0.54 |
| Rx | ng/ml | 1.0E-9 | 1.8E-5 | 1.2E-3 | 1.8E-3 | 2.1E-3 | 2.7E-3 | 1.0E-9 | 1.0E-9 | 8.6E-3 | 8.6E-3 | 32 | 26 | 32 | 26 | 0.56 |
| Ld | pg/ml | 1.0E-9 | 1.0E-9 | 3.1E0 | 4.9E0 | 8.6E0 | 9.0E0 | 1.0E-9 | 1.0E-9 | 6.9E1 | 5.0E1 | 79 | 57 | 79 | 57 | 0.57 |
| Lh | pg/ml | 1.0E4 | 2.0E4 | 1.9E4 | 4.0E4 | 2.6E4 | 7.3E4 | 1.0E-9 | 1.0E-9 | 2.6E5 | 4.8E5 | 247 | 108 | 247 | 108 | 0.64 |
| Li | pg/ml | 2.9E3 | 7.7E3 | 1.6E4 | 3.6E4 | 8.7E4 | 1.0E5 | 1.2E1 | 1.3E1 | 1.3E6 | 9.2E5 | 247 | 108 | 247 | 108 | 0.65 |
| Lj | pg/ml | 2.3E3 | 5.0E3 | 1.7E4 | 3.2E4 | 5.3E4 | 6.7E4 | 1.0E-9 | 1.0E-9 | 4.3E5 | 4.1E5 | 247 | 108 | 247 | 108 | 0.61 |
| Lp | pg/ml | 1.1E1 | 8.0E0 | 9.3E1 | 1.4E2 | 2.2E2 | 3.5E2 | 1.0E-9 | 1.0E-9 | 1.1E3 | 1.4E3 | 32 | 26 | 32 | 26 | 0.49 |
| Lt | pg/ml | 1.0E-9 | 1.0E-9 | 1.2E0 | 1.7E0 | 5.1E0 | 6.2E0 | 1.0E-9 | 1.0E-9 | 2.5E1 | 2.5E1 | 32 | 26 | 32 | 26 | 0.51 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Rv | ng/ml | 5.0E-4 | 5.0E-4 | 1.1E-3 | 2.0E-3 | 1.8E-3 | 4.0E-3 | 1.0E-9 | 1.0E-9 | 9.2E-3 | 1.6E-2 | 32 | 26 | 32 | 26 | 0.52 |
| Ru | ng/ml | 1.0E-9 | 1.0E-9 | 1.6E-2 | 2.9E-2 | 6.5E-2 | 9.3E-2 | 1.0E-9 | 1.0E-9 | 3.1E-1 | 3.5E-1 | 32 | 26 | 32 | 26 | 0.53 |
| Rt | ng/ml | 7.6E-2 | 6.7E-2 | 1.0E-1 | 4.3E-1 | 9.5E-2 | 1.4E0 | 6.5E-3 | 1.3E-3 | 3.8E-1 | 7.4E0 | 32 | 26 | 32 | 26 | 0.51 |
| Yl | pg/ml | 1.4E1 | 1.2E1 | 1.6E1 | 2.7E1 | 1.2E1 | 4.4E1 | 1.0E-9 | 1.0E-9 | 4.7E1 | 2.2E2 | 32 | 26 | 32 | 26 | 0.49 |
| Rm | ng/ml | 1.8E1 | 2.0E1 | 4.5E1 | 5.7E1 | 7.2E1 | 1.0E2 | 2.2E-1 | 2.3E-1 | 3.4E2 | 6.5E2 | 80 | 54 | 80 | 54 | 0.52 |
| Rh | ng/ml | 1.7E2 | 1.7E2 | 5.6E2 | 5.7E2 | 1.9E3 | 2.3E3 | 7.5E0 | 2.5E1 | 1.7E4 | 1.7E4 | 80 | 54 | 80 | 54 | 0.47 |
| Ri | ng/ml | 4.4E-2 | 2.2E-2 | 3.9E0 | 3.7E0 | 8.4E0 | 7.6E0 | 1.0E-9 | 1.0E-9 | 4.9E1 | 4.5E1 | 80 | 54 | 80 | 54 | 0.49 |
| Rg | ng/ml | 1.0E-9 | 1.0E-9 | 4.5E-2 | 1.4E-1 | 3.4E-1 | 5.3E-1 | 1.0E-9 | 1.0E-9 | 3.0E0 | 3.3E0 | 80 | 54 | 80 | 54 | 0.53 |
| Rj | ng/ml | 1.0E-9 | 1.0E-9 | 8.0E-1 | 6.0E0 | 1.8E0 | 3.7E1 | 1.0E-9 | 1.0E-9 | 1.1E1 | 2.7E2 | 80 | 54 | 80 | 54 | 0.50 |
| Rf | ng/ml | 4.1E-1 | 3.3E-1 | 7.8E-1 | 1.4E0 | 1.0E0 | 3.1E0 | 2.1E-2 | 2.1E-2 | 6.2E0 | 1.7E1 | 80 | 54 | 80 | 54 | 0.47 |
| Ql | pg/ml | 3.6E0 | 5.5E0 | 1.0E1 | 1.5E1 | 1.8E1 | 2.9E1 | 1.0E-9 | 1.0E-9 | 1.1E2 | 1.8E2 | 80 | 55 | 80 | 55 | 0.54 |
| Qm | pg/ml | 2.4E-1 | 8.6E0 | 1.8E1 | 2.4E1 | 3.5E1 | 3.6E1 | 1.0E-9 | 1.0E-9 | 1.9E2 | 1.8E2 | 80 | 55 | 80 | 55 | 0.57 |
| Qn | pg/ml | 6.1E-1 | 6.1E-1 | 6.4E0 | 7.9E0 | 2.5E1 | 2.9E1 | 1.0E-9 | 1.0E-9 | 2.2E2 | 2.0E2 | 80 | 55 | 80 | 55 | 0.47 |
| Nv | pg/ml | 3.2E3 | 6.2E3 | 7.7E3 | 1.7E4 | 1.5E4 | 2.9E4 | 1.0E-9 | 1.9E1 | 1.3E5 | 1.6E5 | 247 | 109 | 247 | 109 | 0.64 |
| Nw | pg/ml | 8.3E3 | 1.4E4 | 1.2E4 | 2.2E4 | 1.6E4 | 3.0E4 | 2.0E2 | 1.9E2 | 2.1E5 | 2.2E5 | 247 | 109 | 247 | 109 | 0.68 |
| Nx | pg/ml | 1.7E2 | 2.4E2 | 3.7E2 | 6.3E2 | 5.7E2 | 7.5E2 | 1.0E-9 | 1.0E-9 | 3.5E3 | 4.1E3 | 247 | 109 | 247 | 109 | 0.63 |
| Ny | pg/ml | 5.2E0 | 1.2E1 | 1.2E2 | 8.0E1 | 1.6E3 | 3.0E2 | 1.0E-9 | 1.0E-9 | 2.5E4 | 2.8E3 | 247 | 109 | 247 | 109 | 0.64 |
| Oa | pg/ml | 1.4E2 | 2.4E2 | 3.4E2 | 7.0E2 | 5.4E2 | 9.6E2 | 1.0E-9 | 1.0E-9 | 3.0E3 | 4.5E3 | 80 | 55 | 80 | 55 | 0.61 |
| Op | pg/ml | 4.3E5 | 4.4E5 | 4.4E5 | 4.4E5 | 1.5E5 | 1.9E5 | 2.1E5 | 5.2E4 | 7.3E5 | 7.5E5 | 32 | 26 | 32 | 26 | 0.52 |
| Wn | ng/ml | 1.1E1 | 1.1E1 | 1.1E2 | 1.7E1 | 3.6E2 | 1.7E1 | 1.2E0 | 7.6E-1 | 1.8E3 | 5.6E1 | 26 | 14 | 26 | 14 | 0.45 |
| Tk | ng/ml | 1.1E2 | 1.2E2 | 3.1E2 | 3.6E2 | 7.9E2 | 6.2E2 | 3.0E0 | 4.0E0 | 4.2E3 | 2.3E3 | 27 | 17 | 27 | 17 | 0.51 |
| Oe | pg/ml | 4.7E1 | 4.2E0 | 2.5E2 | 2.4E2 | 3.8E2 | 3.8E2 | 1.0E-9 | 1.0E-9 | 2.3E3 | 2.0E3 | 246 | 108 | 246 | 108 | 0.48 |
| Of | pg/ml | 1.6E2 | 8.9E1 | 4.5E3 | 6.3E3 | 1.9E4 | 2.3E4 | 1.0E-9 | 1.0E-9 | 1.9E5 | 1.7E5 | 247 | 109 | 247 | 109 | 0.46 |
| Og | pg/ml | 7.0E-2 | 6.6E-2 | 4.4E-1 | 1.3E-1 | 1.7E0 | 2.9E-1 | 1.0E-9 | 1.0E-9 | 1.9E1 | 2.5E0 | 247 | 109 | 247 | 109 | 0.45 |
| Oh | pg/ml | 2.3E0 | 4.1E0 | 1.5E1 | 1.7E2 | 1.0E2 | 1.5E3 | 1.0E-9 | 1.0E-9 | 1.4E3 | 1.6E4 | 247 | 109 | 247 | 109 | 0.60 |
| Oi | pg/ml | 1.5E0 | 2.6E0 | 4.9E0 | 5.3E0 | 8.2E0 | 6.9E0 | 1.0E-9 | 1.0E-9 | 6.7E1 | 3.1E1 | 247 | 109 | 247 | 109 | 0.54 |
| Ok | pg/ml | 3.4E2 | 6.1E2 | 4.5E2 | 9.3E2 | 4.2E2 | 1.2E3 | 2.9E1 | 1.5E1 | 2.8E3 | 7.8E3 | 247 | 109 | 247 | 109 | 0.69 |
| Om | pg/ml | 3.8E2 | 5.2E2 | 8.0E2 | 1.4E3 | 2.3E3 | 5.0E3 | 1.0E-9 | 1.0E-9 | 3.0E4 | 5.1E4 | 247 | 109 | 247 | 109 | 0.61 |
| On | pg/ml | 1.5E2 | 2.7E2 | 2.5E2 | 5.5E2 | 4.1E2 | 9.7E2 | 1.0E-9 | 1.0E1 | 4.5E3 | 8.5E3 | 247 | 109 | 247 | 109 | 0.66 |
| Or | pg/ml | 1.1E1 | 1.9E1 | 2.1E1 | 7.1E1 | 3.0E1 | 1.2E2 | 1.0E-9 | 1.0E-9 | 2.0E2 | 5.1E2 | 79 | 58 | 79 | 58 | 0.60 |
| Ow | pg/ml | 3.6E1 | 4.7E1 | 2.2E2 | 2.3E2 | 9.6E2 | 5.3E2 | 1.0E-9 | 1.0E-9 | 8.1E3 | 3.0E3 | 79 | 58 | 79 | 58 | 0.53 |
| Ou | pg/ml | 4.2E2 | 5.7E2 | 9.5E2 | 1.6E3 | 1.4E3 | 2.6E3 | 2.2E1 | 1.0E-9 | 9.8E3 | 1.1E4 | 79 | 58 | 79 | 58 | 0.55 |
| Ul | ng/ml | 1.0E-9 | 1.0E-9 | 1.2E0 | 1.9E0 | 4.9E0 | 8.3E0 | 1.0E-9 | 1.0E-9 | 2.9E1 | 5.6E1 | 81 | 55 | 81 | 55 | 0.51 |
| Uo | ng/ml | 1.0E-9 | 1.0E-9 | 8.5E-2 | 6.0E-2 | 2.4E-1 | 1.6E-1 | 1.0E-9 | 1.0E-9 | 1.3E0 | 6.9E-1 | 81 | 55 | 81 | 55 | 0.47 |
| Uk | ng/ml | 1.0E-9 | 1.0E-9 | 7.4E-3 | 3.9E-3 | 3.7E-2 | 1.8E-2 | 1.0E-9 | 1.0E-9 | 3.2E-1 | 1.3E-1 | 81 | 55 | 81 | 55 | 0.44 |
| Um | ng/ml | 1.0E-9 | 1.0E-9 | 3.0E-1 | 1.3E-1 | 7.0E-1 | 4.0E-1 | 1.0E-9 | 1.0E-9 | 3.6E0 | 2.3E0 | 81 | 55 | 81 | 55 | 0.41 |
| Uf | ng/ml | 5.3E-2 | 8.6E-2 | 1.2E-1 | 2.6E-1 | 1.6E-1 | 7.1E-1 | 2.7E-3 | 1.0E-3 | 7.8E-1 | 5.1E0 | 81 | 55 | 81 | 55 | 0.58 |
| Uh | ng/ml | 1.6E0 | 4.1E0 | 2.8E0 | 4.9E0 | 2.9E0 | 4.2E0 | 3.6E-2 | 4.7E-2 | 1.5E1 | 1.8E1 | 81 | 55 | 81 | 55 | 0.66 |
| Un | ng/ml | 1.7E0 | 1.8E0 | 1.8E0 | 2.8E0 | 1.0E0 | 3.5E0 | 3.5E-1 | 3.4E-1 | 4.9E0 | 2.5E1 | 81 | 55 | 81 | 55 | 0.61 |
| Ug | ng/ml | 1.3E1 | 8.8E0 | 2.3E1 | 2.0E1 | 2.5E1 | 2.9E1 | 1.5E0 | 1.0E0 | 1.4E2 | 1.6E2 | 81 | 55 | 81 | 55 | 0.43 |
| Ur | ng/ml | 1.4E-1 | 6.5E-2 | 3.1E-1 | 4.4E-1 | 5.6E-1 | 1.3E0 | 1.0E-9 | 1.0E-9 | 3.0E0 | 7.3E0 | 81 | 54 | 81 | 54 | 0.42 |
| Up | ng/ml | 1.0E-9 | 1.0E-9 | 2.0E-3 | 4.9E-2 | 7.3E-3 | 3.2E-1 | 1.0E-9 | 1.0E-9 | 5.3E-2 | 2.4E0 | 81 | 54 | 81 | 54 | 0.59 |
| Us | ng/ml | 1.8E-3 | 3.9E-3 | 9.5E-3 | 5.8E-2 | 1.9E-2 | 2.3E-1 | 1.0E-9 | 1.0E-9 | 1.2E-1 | 1.7E0 | 81 | 54 | 81 | 54 | 0.58 |
| Uv | ng/ml | 3.1E-3 | 2.5E-3 | 1.7E-2 | 1.6E-2 | 5.0E-2 | 5.9E-2 | 1.0E-9 | 1.0E-9 | 3.5E-1 | 4.1E-1 | 81 | 54 | 81 | 54 | 0.47 |
| Ut | ng/ml | 6.0E-1 | 1.0E0 | 2.3E0 | 4.8E0 | 6.3E0 | 1.0E1 | 1.0E-9 | 1.0E-9 | 5.2E1 | 6.5E1 | 81 | 54 | 81 | 54 | 0.61 |
| Uu | ng/ml | 7.2E0 | 5.8E0 | 8.0E0 | 6.5E0 | 5.3E0 | 4.4E0 | 5.4E-1 | 5.7E-1 | 2.9E1 | 2.3E1 | 81 | 54 | 81 | 54 | 0.41 |
| Uw | ng/ml | 2.1E0 | 2.6E0 | 2.8E0 | 4.5E0 | 2.8E0 | 7.4E0 | 1.5E-1 | 1.0E-9 | 9.8E0 | 3.9E1 | 33 | 26 | 33 | 26 | 0.62 |
| Vb | ng/ml | 1.1E0 | 1.1E0 | 1.1E0 | 1.1E0 | 4.1E-1 | 1.2E0 | 4.7E-1 | 8.5E-2 | 2.0E0 | 6.4E0 | 33 | 26 | 33 | 26 | 0.37 |
| Vc | ng/ml | 1.0E-9 | 1.0E-9 | 6.1E-3 | 5.1E-4 | 2.3E-2 | 2.6E-3 | 1.0E-9 | 1.0E-9 | 1.1E-1 | 1.3E-2 | 33 | 26 | 33 | 26 | 0.47 |
| Uy | ng/ml | 1.3E0 | 1.3E0 | 4.1E0 | 1.3E1 | 1.0E1 | 2.5E1 | 8.7E-2 | 2.0E-2 | 5.1E1 | 9.9E1 | 33 | 26 | 33 | 26 | 0.56 |
| Uz | ng/ml | 1.0E-9 | 1.0E-9 | 1.3E-2 | 1.3E0 | 7.6E-2 | 6.5E0 | 1.0E-9 | 1.0E-9 | 4.4E-1 | 3.3E1 | 33 | 26 | 33 | 26 | 0.52 |
| Ux | ng/ml | 2.0E2 | 1.7E2 | 1.9E2 | 1.9E2 | 1.4E2 | 1.4E2 | 1.2E1 | 4.5E0 | 4.8E2 | 4.9E2 | 33 | 26 | 33 | 26 | 0.50 |
| Va | ng/ml | 1.4E1 | 5.3E0 | 2.5E1 | 2.0E1 | 3.0E1 | 2.5E1 | 3.1E-1 | 3.6E-1 | 1.2E2 | 9.6E1 | 33 | 26 | 33 | 26 | 0.43 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vh | ng/ml | 1.0E-2 | 1.9E-2 | 1.8E-2 | 5.4E-2 | 2.4E-2 | 1.7E-1 | 3.9E-4 | 1.0E-3 | 1.2E-1 | 8.6E-1 | 33 | 26 | 33 | 26 | 0.61 |
| Vi | ng/ml | 4.2E-3 | 4.2E-3 | 7.6E-3 | 8.9E-2 | 8.2E-3 | 3.6E-1 | 1.0E-9 | 1.0E-9 | 3.3E-2 | 1.8E0 | 33 | 26 | 33 | 26 | 0.57 |
| Vj | ng/ml | 2.3E1 | 6.2E1 | 4.8E1 | 9.7E1 | 5.0E1 | 1.4E2 | 4.6E0 | 1.4E0 | 1.8E2 | 6.5E2 | 33 | 25 | 33 | 25 | 0.61 |
| Vp | ng/ml | 1.0E-9 | 1.0E-9 | 1.4E-1 | 1.1E0 | 6.5E-1 | 6.6E0 | 1.0E-9 | 1.0E-9 | 3.9E0 | 4.9E1 | 81 | 55 | 81 | 55 | 0.57 |
| Vt | ng/ml | 5.6E0 | 7.9E0 | 6.6E0 | 1.4E1 | 5.1E0 | 2.2E1 | 9.6E-1 | 5.6E-1 | 3.2E1 | 1.6E2 | 81 | 55 | 81 | 55 | 0.64 |
| Vu | ng/ml | 1.0E-9 | 1.0E-9 | 1.1E0 | 2.3E0 | 2.4E0 | 4.3E0 | 1.0E-9 | 1.0E-9 | 1.6E1 | 2.2E1 | 79 | 53 | 79 | 53 | 0.57 |
| Vq | ng/ml | 1.5E2 | 2.8E2 | 6.1E2 | 9.2E2 | 1.0E3 | 2.1E3 | 9.2E-1 | 6.5E-1 | 5.0E3 | 1.2E4 | 66 | 42 | 66 | 42 | 0.55 |
| Vo | ng/ml | 2.5E1 | 2.6E1 | 2.5E1 | 2.4E1 | 4.3E0 | 7.1E0 | 9.7E0 | 1.9E0 | 3.5E1 | 4.8E1 | 81 | 55 | 81 | 55 | 0.50 |
| Vs | ng/ml | 1.0E-9 | 1.0E-9 | 4.7E0 | 1.3E1 | 1.3E1 | 6.3E1 | 1.0E-9 | 1.0E-9 | 8.9E1 | 4.5E2 | 80 | 53 | 80 | 53 | 0.47 |
| Vv | ng/ml | 2.9E0 | 3.0E0 | 5.9E0 | 6.3E0 | 1.1E1 | 9.2E0 | 1.0E-9 | 1.0E-9 | 8.4E1 | 4.4E1 | 81 | 54 | 81 | 54 | 0.49 |
| Vw | ng/ml | 3.6E1 | 4.2E1 | 3.3E1 | 3.7E1 | 1.7E1 | 2.0E1 | 3.1E0 | 2.5E0 | 6.7E1 | 6.9E1 | 33 | 26 | 33 | 26 | 0.57 |
| Oy | pg/ml | 4.9E-1 | 3.2E-1 | 7.2E0 | 3.5E0 | 3.5E1 | 1.1E1 | 1.0E-9 | 1.0E-9 | 4.0E2 | 9.9E1 | 247 | 108 | 247 | 108 | 0.45 |
| Oz | pg/ml | 7.9E-3 | 1.0E-9 | 2.6E-1 | 7.2E-1 | 3.9E-1 | 3.8E0 | 1.0E-9 | 1.0E-9 | 2.1E0 | 2.9E1 | 247 | 108 | 247 | 108 | 0.46 |
| Pa | pg/ml | 3.8E-1 | 4.5E-1 | 1.4E0 | 4.6E0 | 6.4E0 | 2.4E1 | 1.0E-9 | 1.0E-9 | 8.6E1 | 2.3E2 | 247 | 108 | 247 | 108 | 0.55 |
| Pb | pg/ml | 1.0E-9 | 1.0E-9 | 2.1E0 | 7.3E-1 | 3.1E1 | 4.1E0 | 1.0E-9 | 1.0E-9 | 4.9E2 | 3.2E1 | 247 | 108 | 247 | 108 | 0.47 |
| Pc | pg/ml | 5.3E-2 | 1.0E-9 | 4.1E-1 | 3.7E0 | 1.0E0 | 3.2E1 | 1.0E-9 | 1.0E-9 | 1.4E1 | 3.3E2 | 247 | 108 | 247 | 108 | 0.47 |
| Pd | pg/ml | 1.5E0 | 2.1E0 | 3.8E0 | 1.4E1 | 8.6E0 | 8.2E1 | 1.0E-9 | 1.0E-9 | 9.4E1 | 8.4E2 | 247 | 108 | 247 | 108 | 0.55 |
| Pe | pg/ml | 1.9E1 | 4.1E1 | 9.2E1 | 4.2E2 | 3.8E2 | 1.7E3 | 1.0E-9 | 1.0E-9 | 4.7E3 | 1.5E4 | 247 | 108 | 247 | 108 | 0.62 |
| Pf | pg/ml | 1.4E0 | 2.6E0 | 1.1E1 | 3.1E1 | 4.5E1 | 1.5E2 | 1.0E-9 | 1.0E-9 | 4.8E2 | 1.5E3 | 247 | 108 | 247 | 108 | 0.58 |
| Pg | pg/ml | 3.5E0 | 7.5E0 | 4.8E1 | 1.6E2 | 3.4E2 | 7.9E2 | 1.0E-9 | 1.0E-9 | 4.2E3 | 7.7E3 | 247 | 108 | 247 | 108 | 0.61 |
| Ph | ng/ml | 1.5E-1 | 1.5E-1 | 3.5E-1 | 4.4E-1 | 4.6E-1 | 8.5E-1 | 1.0E-9 | 1.0E-9 | 2.2E0 | 5.4E0 | 79 | 58 | 79 | 58 | 0.50 |
| Pi | ng/ml | 1.9E-1 | 2.4E-1 | 2.9E-1 | 1.8E0 | 4.2E-1 | 1.1E1 | 1.0E-9 | 1.0E-9 | 3.2E0 | 8.2E1 | 79 | 58 | 79 | 58 | 0.57 |
| Pj | ng/mL | 4.9E0 | 6.0E0 | 5.4E0 | 7.3E0 | 3.3E0 | 5.7E0 | 4.9E-1 | 4.0E-1 | 1.6E1 | 3.1E1 | 79 | 58 | 79 | 58 | 0.59 |
| Pk | ng/ml | 7.6E-3 | 1.2E-2 | 1.3E-2 | 4.4E-2 | 3.0E-2 | 2.0E-1 | 1.0E-9 | 1.0E-9 | 2.5E-1 | 1.5E0 | 79 | 58 | 79 | 58 | 0.64 |
| aA | mg/dL | 8.8E-1 | 1.0E0 | 9.8E-1 | 1.2E0 | 4.9E-1 | 7.9E-1 | 3.0E-1 | 4.0E-1 | 4.2E0 | 4.7E0 | 397 | 140 | 397 | 140 | 0.59 |
| aC | mg/mL | 2.6E0 | 2.0E0 | 2.9E0 | 2.3E0 | 1.3E0 | 1.2E0 | 1.1E0 | 7.5E-1 | 7.4E0 | 6.7E0 | 107 | 69 | 107 | 69 | 0.34 |
| aD | ug/mL | 2.8E0 | 3.2E0 | 4.7E0 | 4.7E0 | 5.2E0 | 3.8E0 | 7.5E-1 | 7.5E-1 | 3.5E1 | 2.1E1 | 107 | 69 | 107 | 69 | 0.52 |
| aE | mg/mL | 5.8E-1 | 5.7E-1 | 5.9E-1 | 6.0E-1 | 1.6E-1 | 1.9E-1 | 2.8E-1 | 1.8E-1 | 1.1E0 | 1.2E0 | 107 | 69 | 107 | 69 | 0.50 |
| aF | ng/mL | 2.2E0 | 2.4E0 | 5.2E0 | 4.9E0 | 8.4E0 | 6.4E0 | 4.3E-3 | 4.3E-3 | 5.0E1 | 3.5E1 | 107 | 69 | 107 | 69 | 0.53 |
| aG | mg/mL | 1.4E-1 | 1.4E-1 | 1.6E-1 | 1.6E-1 | 8.9E-2 | 8.3E-2 | 3.2E-2 | 5.1E-2 | 4.6E-1 | 4.8E-1 | 107 | 69 | 107 | 69 | 0.51 |
| aH | ug/mL | 7.4E1 | 7.1E1 | 7.6E1 | 7.9E1 | 3.8E1 | 4.3E1 | 8.9E0 | 1.1E1 | 2.0E2 | 2.0E2 | 107 | 69 | 107 | 69 | 0.51 |
| aI | ug/mL | 1.8E2 | 1.6E2 | 1.8E2 | 1.7E2 | 5.9E1 | 6.3E1 | 3.2E1 | 4.7E1 | 3.3E2 | 3.4E2 | 107 | 69 | 107 | 69 | 0.43 |
| aJ | ug/mL | 2.5E0 | 2.5E0 | 3.1E0 | 3.7E0 | 2.3E0 | 3.3E0 | 9.5E-1 | 8.2E-1 | 1.4E1 | 2.3E1 | 107 | 69 | 107 | 69 | 0.56 |
| aK | ng/mL | 1.4E0 | 1.1E0 | 2.1E0 | 1.7E0 | 2.0E0 | 1.8E0 | 2.8E-2 | 2.9E-4 | 9.1E0 | 1.0E1 | 107 | 69 | 107 | 69 | 0.44 |
| aL | mg/mL | 7.5E-1 | 7.2E-1 | 7.8E-1 | 7.3E-1 | 2.6E-1 | 2.5E-1 | 2.2E-1 | 2.7E-1 | 1.4E0 | 1.7E0 | 107 | 69 | 107 | 69 | 0.44 |
| aM | U/mL | 1.5E1 | 2.3E1 | 3.2E1 | 6.4E1 | 5.2E1 | 1.3E2 | 4.2E-2 | 4.2E-2 | 3.5E2 | 8.2E2 | 107 | 69 | 107 | 69 | 0.63 |
| aN | U/mL | 1.2E1 | 2.1E1 | 1.7E1 | 3.9E1 | 1.9E1 | 6.1E1 | 2.5E-3 | 2.5E-3 | 9.7E1 | 3.8E2 | 107 | 69 | 107 | 69 | 0.68 |
| aO | pg/mL | 4.8E1 | 7.2E1 | 4.7E2 | 3.9E2 | 1.1E3 | 7.0E2 | 6.0E-2 | 6.0E-2 | 6.6E3 | 3.3E3 | 107 | 69 | 107 | 69 | 0.53 |
| aP | ng/mL | 1.6E0 | 1.7E0 | 2.3E0 | 2.6E0 | 2.8E0 | 3.4E0 | 4.5E-1 | 6.8E-1 | 2.8E1 | 2.8E1 | 107 | 69 | 107 | 69 | 0.55 |
| aQ | ng/mL | 2.5E-1 | 2.4E-1 | 3.5E-1 | 3.6E-1 | 2.8E-1 | 3.7E-1 | 2.0E-4 | 2.0E-4 | 1.1E0 | 2.0E0 | 107 | 69 | 107 | 69 | 0.48 |
| aR | ng/mL | 1.7E0 | 1.9E0 | 3.0E0 | 3.1E0 | 4.4E0 | 3.3E0 | 2.6E-1 | 5.6E-1 | 3.4E1 | 1.7E1 | 107 | 69 | 107 | 69 | 0.56 |
| aS | ng/mL | 3.1E-1 | 5.1E-1 | 9.6E-1 | 1.0E0 | 3.2E0 | 1.3E0 | 4.2E-3 | 4.2E-3 | 3.3E1 | 6.2E0 | 107 | 69 | 107 | 69 | 0.61 |
| aU | pg/mL | 7.6E1 | 5.7E1 | 1.1E2 | 8.9E1 | 1.0E2 | 1.2E2 | 7.4E-2 | 7.4E-2 | 5.1E2 | 7.0E2 | 107 | 69 | 107 | 69 | 0.42 |
| aV | ng/mL | 6.3E-1 | 4.5E-1 | 9.3E-1 | 1.2E0 | 1.0E0 | 3.9E0 | 3.8E-2 | 7.6E-4 | 6.0E0 | 3.3E1 | 107 | 69 | 107 | 69 | 0.46 |
| aW | pg/mL | 1.9E1 | 2.0E1 | 2.1E1 | 2.4E1 | 1.8E1 | 4.9E1 | 7.2E-2 | 7.2E-2 | 1.7E2 | 4.2E2 | 107 | 69 | 107 | 69 | 0.47 |
| aX | ng/mL | 8.9E0 | 7.1E0 | 1.5E1 | 1.9E1 | 2.4E1 | 4.2E1 | 3.0E-1 | 6.2E-1 | 2.2E2 | 3.1E2 | 107 | 69 | 107 | 69 | 0.45 |
| aY | pg/mL | 5.3E1 | 5.4E1 | 6.8E1 | 8.7E1 | 5.7E1 | 1.6E2 | 4.1E-1 | 4.1E-1 | 3.1E2 | 1.2E3 | 107 | 69 | 107 | 69 | 0.51 |
| aZ | pg/mL | 2.2E2 | 2.9E2 | 5.3E2 | 9.0E2 | 1.2E3 | 1.7E3 | 1.7E0 | 1.7E0 | 1.2E4 | 7.9E3 | 107 | 69 | 107 | 69 | 0.54 |
| bA | ng/mL | 1.0E1 | 2.7E1 | 4.0E1 | 1.3E2 | 8.6E1 | 2.5E2 | 3.0E-2 | 3.0E-2 | 6.8E2 | 1.5E3 | 107 | 69 | 107 | 69 | 0.65 |
| bB | ng/mL | 2.8E2 | 2.7E2 | 3.1E2 | 2.9E2 | 1.8E2 | 1.8E2 | 2.1E0 | 1.2E1 | 9.5E2 | 8.1E2 | 107 | 69 | 107 | 69 | 0.45 |
| bC | ng/mL | 3.3E2 | 3.2E2 | 5.6E2 | 7.9E2 | 7.2E2 | 1.1E3 | 1.4E1 | 3.5E1 | 4.0E3 | 4.7E3 | 107 | 69 | 107 | 69 | 0.52 |
| bE | mg/mL | 5.3E0 | 4.6E0 | 5.8E0 | 5.0E0 | 2.2E0 | 2.1E0 | 1.8E0 | 1.3E0 | 1.2E1 | 1.2E1 | 107 | 69 | 107 | 69 | 0.39 |
| bF | pg/mL | 3.5E1 | 4.3E1 | 3.2E2 | 3.9E2 | 1.3E3 | 1.5E3 | 5.0E-2 | 2.5E0 | 1.1E4 | 1.0E4 | 107 | 69 | 107 | 69 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bG | ng/mL | 1.5E0 | 1.8E0 | 2.9E0 | 3.5E0 | 4.2E0 | 4.8E0 | 1.1E-1 | 1.6E-1 | 2.6E1 | 3.0E1 | 107 | 69 | 107 | 69 | 0.54 |
| bH | pg/mL | 5.7E-1 | 5.7E-1 | 6.3E0 | 6.2E0 | 2.7E1 | 1.6E1 | 5.7E-1 | 5.7E-1 | 2.8E2 | 1.2E2 | 107 | 69 | 107 | 69 | 0.50 |
| bI | ng/mL | 4.0E-3 | 4.0E-3 | 7.5E-2 | 1.2E-1 | 1.5E-1 | 2.5E-1 | 4.0E-3 | 4.0E-3 | 6.8E-1 | 9.8E-1 | 107 | 69 | 107 | 69 | 0.51 |
| bJ | mg/mL | 2.1E0 | 1.8E0 | 2.6E0 | 2.1E0 | 2.0E0 | 2.0E0 | 2.5E-4 | 2.5E-4 | 9.0E0 | 1.1E1 | 107 | 69 | 107 | 69 | 0.41 |
| bL | pg/mL | 4.3E0 | 3.2E0 | 1.0E1 | 7.8E0 | 1.3E1 | 9.6E0 | 4.6E-2 | 4.6E-2 | 6.0E1 | 3.5E1 | 107 | 69 | 107 | 69 | 0.45 |
| bM | mg/mL | 1.6E0 | 2.2E0 | 1.9E0 | 2.6E0 | 1.2E0 | 1.8E0 | 1.4E-1 | 1.6E-2 | 6.2E0 | 8.6E0 | 107 | 69 | 107 | 69 | 0.63 |
| bN | ng/mL | 3.3E1 | 3.5E1 | 1.3E2 | 1.1E2 | 2.9E2 | 2.8E2 | 1.4E-1 | 1.4E-1 | 1.9E3 | 1.9E3 | 107 | 69 | 107 | 69 | 0.49 |
| bO | ng/mL | 4.0E-2 | 4.0E-2 | 9.1E0 | 9.0E0 | 1.6E1 | 2.5E1 | 4.0E-2 | 4.0E-2 | 6.4E1 | 1.3E2 | 107 | 69 | 107 | 69 | 0.42 |
| bP | mg/mL | 5.2E-1 | 5.3E-1 | 7.2E-1 | 7.8E-1 | 6.1E-1 | 8.3E-1 | 4.9E-2 | 9.2E-2 | 3.7E0 | 4.8E0 | 107 | 69 | 107 | 69 | 0.50 |
| bQ | pg/mL | 2.0E1 | 2.4E1 | 6.7E1 | 2.4E2 | 2.4E2 | 1.6E3 | 1.5E-1 | 1.5E-1 | 2.4E3 | 1.3E4 | 107 | 69 | 107 | 69 | 0.56 |
| bR | ng/mL | 1.2E-2 | 1.2E-2 | 1.3E-1 | 2.0E-1 | 3.5E-1 | 1.0E0 | 1.2E-2 | 1.2E-2 | 3.4E0 | 8.7E0 | 107 | 69 | 107 | 69 | 0.48 |
| bS | pg/mL | 9.4E-1 | 9.4E-1 | 9.1E0 | 1.0E1 | 3.5E1 | 4.8E1 | 9.4E-1 | 9.4E-1 | 3.4E2 | 3.9E2 | 107 | 69 | 107 | 69 | 0.47 |
| bU | ng/mL | 7.0E-2 | 1.0E-1 | 1.6E-1 | 2.3E-1 | 2.3E-1 | 7.9E-1 | 1.3E-2 | 1.3E-2 | 1.8E0 | 6.6E0 | 107 | 69 | 107 | 69 | 0.49 |
| bV | pg/mL | 4.4E2 | 5.5E2 | 6.2E2 | 6.6E2 | 1.1E3 | 4.0E2 | 1.6E2 | 2.3E2 | 1.2E4 | 2.2E3 | 107 | 69 | 107 | 69 | 0.62 |
| bW | pg/mL | 3.2E2 | 3.2E2 | 4.8E2 | 6.0E2 | 4.3E2 | 8.7E2 | 8.4E1 | 1.1E2 | 2.4E3 | 4.8E3 | 107 | 69 | 107 | 69 | 0.52 |
| bX | ng/mL | 2.5E-5 | 2.5E-5 | 2.7E-3 | 2.4E-3 | 3.4E-3 | 2.7E-3 | 2.5E-5 | 2.5E-5 | 1.3E-2 | 8.4E-3 | 107 | 69 | 107 | 69 | 0.49 |
| bZ | pg/mL | 2.7E2 | 3.1E2 | 1.9E3 | 2.3E3 | 5.9E3 | 8.6E3 | 1.5E-1 | 1.5E-1 | 4.4E4 | 5.8E4 | 107 | 69 | 107 | 69 | 0.52 |
| cA | pg/mL | 6.0E-1 | 6.0E-1 | 2.0E0 | 7.2E0 | 3.6E0 | 4.5E1 | 6.0E-1 | 6.0E-1 | 1.6E1 | 3.7E2 | 107 | 69 | 107 | 69 | 0.48 |
| cB | ng/mL | 5.0E-2 | 4.5E-2 | 7.4E-2 | 7.5E-2 | 8.1E-2 | 9.8E-2 | 1.7E-3 | 1.7E-3 | 3.8E-1 | 4.3E-1 | 107 | 69 | 107 | 69 | 0.46 |
| cC | pg/mL | 4.6E1 | 3.5E1 | 4.8E1 | 4.1E1 | 4.5E1 | 5.7E1 | 1.0E0 | 1.0E0 | 3.7E2 | 4.5E2 | 107 | 69 | 107 | 69 | 0.41 |
| cD | pg/mL | 6.0E0 | 4.0E0 | 1.3E1 | 1.2E1 | 4.9E1 | 3.9E1 | 3.3E-1 | 3.3E-1 | 4.8E2 | 2.9E2 | 107 | 69 | 107 | 69 | 0.41 |
| cE | pg/mL | 5.3E1 | 7.0E1 | 2.9E2 | 2.3E2 | 6.2E2 | 5.4E2 | 1.2E-1 | 1.2E-1 | 3.1E3 | 3.8E3 | 107 | 69 | 107 | 69 | 0.54 |
| cF | pg/mL | 8.7E0 | 5.3E-1 | 1.7E1 | 1.4E1 | 2.6E1 | 3.5E1 | 5.3E-1 | 5.3E-1 | 1.4E2 | 2.7E2 | 107 | 69 | 107 | 69 | 0.45 |
| cG | pg/mL | 5.1E1 | 7.0E1 | 1.0E2 | 2.9E2 | 1.6E2 | 1.3E3 | 1.1E1 | 7.8E0 | 1.1E3 | 1.0E4 | 107 | 69 | 107 | 69 | 0.58 |
| cH | uIU/mL | 3.1E0 | 3.7E0 | 6.9E0 | 8.7E0 | 1.7E1 | 1.7E1 | 8.6E-3 | 8.6E-3 | 1.6E2 | 1.2E2 | 107 | 69 | 107 | 69 | 0.53 |
| cI | ng/mL | 5.9E0 | 6.9E0 | 1.2E1 | 1.7E1 | 1.8E1 | 2.5E1 | 3.2E-2 | 2.3E-1 | 1.0E2 | 1.2E2 | 107 | 69 | 107 | 69 | 0.54 |
| cJ | ug/mL | 7.1E1 | 5.1E1 | 1.0E2 | 9.0E1 | 1.0E2 | 1.0E2 | 6.9E0 | 5.6E0 | 6.4E2 | 6.2E2 | 107 | 69 | 107 | 69 | 0.46 |
| cK | ng/mL | 3.8E-3 | 3.8E-3 | 1.7E-2 | 3.1E-2 | 4.4E-2 | 1.8E-1 | 3.8E-3 | 3.8E-3 | 3.4E-1 | 1.5E0 | 107 | 69 | 107 | 69 | 0.48 |
| cL | pg/mL | 2.2E2 | 2.0E2 | 4.0E2 | 7.6E2 | 8.4E2 | 3.0E3 | 3.6E1 | 3.1E1 | 7.1E3 | 2.4E4 | 107 | 69 | 107 | 69 | 0.51 |
| cM | pg/mL | 2.7E2 | 2.6E2 | 3.0E2 | 2.6E2 | 1.8E2 | 1.2E2 | 2.5E1 | 4.2E1 | 1.1E3 | 6.7E2 | 107 | 69 | 107 | 69 | 0.45 |
| cN | pg/mL | 1.2E2 | 1.2E2 | 1.3E2 | 1.4E2 | 5.2E1 | 1.2E2 | 3.8E1 | 6.3E1 | 3.2E2 | 1.1E3 | 107 | 69 | 107 | 69 | 0.54 |
| cO | pg/mL | 2.1E2 | 2.1E2 | 3.2E2 | 5.4E2 | 3.5E2 | 2.3E3 | 5.4E1 | 8.2E1 | 2.4E3 | 1.9E4 | 107 | 69 | 107 | 69 | 0.48 |
| cP | ng/mL | 2.4E3 | 2.4E3 | 2.5E3 | 2.6E3 | 9.5E2 | 9.6E2 | 6.2E2 | 1.0E3 | 5.6E3 | 4.7E3 | 107 | 69 | 107 | 69 | 0.52 |
| cQ | ng/mL | 4.9E-2 | 6.2E-2 | 1.2E-1 | 1.5E-1 | 1.9E-1 | 2.4E-1 | 2.0E-3 | 2.0E-3 | 1.2E0 | 1.3E0 | 107 | 69 | 107 | 69 | 0.54 |
| cR | ng/mL | 3.9E2 | 3.0E2 | 5.9E2 | 6.1E2 | 6.8E2 | 1.0E3 | 3.6E1 | 2.0E1 | 4.8E3 | 7.7E3 | 107 | 69 | 107 | 69 | 0.47 |
| cS | ng/mL | 2.8E2 | 3.1E2 | 4.5E2 | 5.3E2 | 4.9E2 | 9.4E2 | 4.1E1 | 9.7E1 | 2.5E3 | 7.1E3 | 107 | 69 | 107 | 69 | 0.52 |
| cT | ng/mL | 3.8E1 | 6.6E1 | 9.8E1 | 2.6E2 | 1.5E2 | 4.4E2 | 3.6E0 | 4.2E0 | 8.4E2 | 2.1E3 | 107 | 69 | 107 | 69 | 0.62 |
| cU | ng/mL | 5.6E1 | 8.0E1 | 7.8E1 | 1.3E2 | 8.7E1 | 2.1E2 | 6.2E0 | 1.4E1 | 7.7E2 | 1.6E3 | 107 | 69 | 107 | 69 | 0.61 |
| cV | ng/mL | 1.8E-1 | 2.2E-1 | 7.6E-1 | 6.6E-1 | 4.5E0 | 1.6E0 | 2.5E-2 | 3.4E-2 | 4.7E1 | 9.7E0 | 107 | 69 | 107 | 69 | 0.53 |
| cW | mIU/mL | 4.9E-2 | 4.5E-2 | 1.0E-1 | 7.0E-2 | 4.3E-1 | 6.6E-2 | 4.8E-3 | 4.8E-3 | 4.5E0 | 3.9E-1 | 107 | 69 | 107 | 69 | 0.50 |
| cX | ng/mL | 1.1E-1 | 1.6E-1 | 1.5E0 | 2.3E0 | 5.0E0 | 6.4E0 | 2.3E-4 | 2.3E-4 | 2.8E1 | 2.8E1 | 107 | 69 | 107 | 69 | 0.55 |
| cY | ng/mL | 7.6E0 | 6.7E0 | 1.1E1 | 1.0E1 | 9.8E0 | 1.2E1 | 2.2E-1 | 1.7E-1 | 4.1E1 | 6.1E1 | 107 | 69 | 107 | 69 | 0.45 |
| cZ | ug/mL | 1.4E1 | 1.2E1 | 1.5E1 | 1.4E1 | 6.8E0 | 6.7E0 | 2.3E0 | 2.8E0 | 4.6E1 | 3.0E1 | 107 | 69 | 107 | 69 | 0.44 |
| dA | pg/mL | 3.1E2 | 3.2E2 | 3.5E2 | 4.5E2 | 1.8E2 | 6.8E2 | 1.0E2 | 1.1E2 | 1.3E3 | 5.8E3 | 107 | 69 | 107 | 69 | 0.53 |
| dB | ug/mL | 1.8E1 | 1.8E1 | 2.0E1 | 1.6E1 | 2.4E1 | 9.4E0 | 2.1E0 | 2.1E0 | 2.5E2 | 3.0E1 | 107 | 69 | 107 | 69 | 0.46 |
| dC | nmol/L | 3.5E1 | 3.1E1 | 3.8E1 | 3.6E1 | 1.8E1 | 1.5E1 | 1.0E1 | 7.8E0 | 1.4E2 | 9.1E1 | 107 | 69 | 107 | 69 | 0.46 |
| dD | ug/mL | 3.5E1 | 3.2E1 | 3.7E1 | 3.3E1 | 1.1E1 | 1.1E1 | 1.4E1 | 1.4E1 | 7.4E1 | 6.0E1 | 107 | 69 | 107 | 69 | 0.39 |
| dE | ng/mL | 5.9E-1 | 3.4E-1 | 6.2E-1 | 5.2E-1 | 5.7E-1 | 6.6E-1 | 8.4E-3 | 8.4E-3 | 2.7E0 | 2.9E0 | 107 | 69 | 107 | 69 | 0.42 |
| dF | ng/mL | 2.4E2 | 3.0E2 | 3.1E2 | 4.0E2 | 2.2E2 | 2.9E2 | 7.5E1 | 7.5E1 | 1.2E3 | 1.3E3 | 107 | 69 | 107 | 69 | 0.61 |
| dG | ng/mL | 1.1E1 | 1.3E1 | 1.6E1 | 1.9E1 | 1.4E1 | 2.4E1 | 3.2E0 | 3.0E0 | 9.7E1 | 1.8E2 | 107 | 69 | 107 | 69 | 0.55 |
| dH | pg/mL | 7.9E0 | 8.5E0 | 2.0E1 | 2.2E1 | 4.6E1 | 8.0E1 | 4.0E-2 | 4.0E-2 | 3.1E2 | 6.7E2 | 107 | 69 | 107 | 69 | 0.54 |
| dI | pg/mL | 4.6E-1 | 4.6E-1 | 2.0E0 | 6.5E0 | 5.5E0 | 4.0E1 | 4.6E-1 | 4.6E-1 | 4.2E1 | 3.3E2 | 107 | 69 | 107 | 69 | 0.52 |
| dJ | ng/mL | 2.0E0 | 2.1E0 | 2.2E0 | 2.1E0 | 1.1E0 | 1.1E0 | 3.2E-2 | 3.2E-2 | 5.6E0 | 4.8E0 | 107 | 69 | 107 | 69 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dK | uIU/mL | 1.5E0 | 9.7E-1 | 2.4E0 | 1.7E0 | 4.3E0 | 2.0E0 | 2.8E-4 | 1.4E-2 | 3.9E1 | 1.1E1 | 107 | 69 | 107 | 69 | 0.42 |
| dL | ng/mL | 8.2E2 | 9.1E2 | 9.8E2 | 1.2E3 | 5.1E2 | 7.6E2 | 3.4E2 | 2.8E2 | 3.4E3 | 4.8E3 | 107 | 69 | 107 | 69 | 0.60 |
| dM | pg/mL | 9.5E2 | 1.1E3 | 1.3E3 | 1.5E3 | 1.5E3 | 1.5E3 | 3.9E2 | 3.7E2 | 1.5E4 | 9.6E3 | 107 | 69 | 107 | 69 | 0.56 |
| dN | ug/mL | 9.9E1 | 1.0E2 | 1.0E2 | 1.1E2 | 4.3E1 | 4.6E1 | 2.5E1 | 2.4E1 | 2.8E2 | 3.3E2 | 107 | 69 | 107 | 69 | 0.55 |
| dR | pg/ml | 1.7E3 | 1.2E3 | 2.0E3 | 2.0E3 | 1.7E3 | 2.3E3 | 1.4E2 | 1.3E2 | 7.8E3 | 9.8E3 | 74 | 59 | 74 | 59 | 0.42 |
| dU | pg/ml | 7.5E3 | 1.5E4 | 9.9E3 | 1.9E4 | 8.3E3 | 1.9E4 | 3.4E3 | 6.9E2 | 3.5E4 | 8.1E4 | 13 | 20 | 13 | 20 | 0.65 |
| dX | ng/ml | 8.4E-2 | 8.1E-2 | 1.6E-1 | 9.3E-2 | 2.2E-1 | 1.1E-1 | 2.6E-3 | 2.6E-3 | 1.2E0 | 4.2E-1 | 38 | 23 | 38 | 23 | 0.43 |
| dW | ng/ml | 1.2E-1 | 1.7E-1 | 2.2E-1 | 2.2E-1 | 2.4E-1 | 1.7E-1 | 6.4E-2 | 6.8E-2 | 8.0E-1 | 5.9E-1 | 11 | 8 | 11 | 8 | 0.61 |
| eF | ng/ml | 4.3E0 | 4.0E0 | 5.6E0 | 5.1E0 | 5.3E0 | 3.9E0 | 2.0E0 | 2.0E0 | 4.6E1 | 2.9E1 | 74 | 59 | 74 | 59 | 0.47 |
| eC | pg/ml | 3.0E2 | 2.8E2 | 3.8E2 | 3.4E2 | 3.0E2 | 3.0E2 | 9.9E0 | 1.9E1 | 1.6E3 | 2.0E3 | 64 | 53 | 64 | 53 | 0.45 |
| eD | pg/ml | 2.2E2 | 2.1E2 | 6.2E2 | 8.5E2 | 1.5E3 | 1.5E3 | 5.2E-1 | 5.2E-1 | 8.3E3 | 7.0E3 | 60 | 35 | 60 | 35 | 0.51 |
| eO | ng/ml | 4.6E1 | 9.4E1 | 2.9E2 | 1.0E2 | 3.9E2 | 4.6E1 | 2.0E1 | 4.3E1 | 9.9E2 | 1.7E2 | 11 | 8 | 11 | 8 | 0.61 |
| eM | ng/ml | 3.1E0 | 2.6E0 | 4.5E0 | 5.5E0 | 4.7E0 | 8.3E0 | 7.6E-1 | 6.9E-1 | 2.7E1 | 3.9E1 | 46 | 33 | 46 | 33 | 0.47 |
| eP | ng/ml | 3.7E-3 | 3.7E-3 | 7.5E-1 | 1.9E0 | 1.7E0 | 6.0E0 | 3.7E-3 | 3.7E-3 | 8.6E0 | 2.8E1 | 38 | 23 | 38 | 23 | 0.46 |
| cT | ng/ml | 2.4E2 | 3.9E2 | 6.9E2 | 8.2E2 | 8.3E2 | 9.0E2 | 1.0E2 | 7.1E1 | 2.9E3 | 2.9E3 | 33 | 22 | 33 | 22 | 0.59 |
| eQ | pg/ml | 1.0E0 | 1.0E0 | 7.9E1 | 3.6E1 | 1.5E2 | 6.6E1 | 1.0E0 | 1.0E0 | 4.7E2 | 2.2E2 | 13 | 20 | 13 | 20 | 0.47 |
| eW | U/ml | 1.1E-2 | 6.7E-3 | 1.9E-1 | 5.2E-2 | 4.6E-1 | 8.4E-2 | 6.7E-3 | 6.7E-3 | 1.6E0 | 1.9E-1 | 11 | 8 | 11 | 8 | 0.38 |
| fA | ng/ml | 2.0E2 | 2.1E2 | 3.6E2 | 4.9E2 | 4.6E2 | 4.9E2 | 3.9E1 | 4.0E1 | 1.5E3 | 1.4E3 | 13 | 18 | 13 | 18 | 0.57 |
| eZ | ng/ml | 4.7E1 | 5.7E1 | 5.5E1 | 5.9E1 | 2.7E1 | 2.3E1 | 1.8E1 | 2.3E1 | 1.2E2 | 1.1E2 | 33 | 22 | 33 | 22 | 0.57 |
| fB | ng/ml | 5.5E2 | 6.4E2 | 6.5E2 | 6.6E2 | 2.6E2 | 2.7E2 | 3.1E2 | 2.6E2 | 1.3E3 | 1.3E3 | 13 | 18 | 13 | 18 | 0.54 |
| fN | ng/ml | 2.1E-1 | 2.1E-1 | 4.1E0 | 3.5E0 | 1.0E1 | 6.0E0 | 2.1E-1 | 2.1E-1 | 5.4E1 | 2.1E1 | 33 | 22 | 33 | 22 | 0.48 |
| fP | ng/ml | 2.7E2 | 2.7E2 | 3.4E2 | 3.1E2 | 2.0E2 | 1.6E2 | 8.9E1 | 1.8E0 | 1.0E3 | 7.7E2 | 71 | 56 | 71 | 56 | 0.48 |
| fR | ng/ml | 1.3E5 | 1.7E5 | 2.2E5 | 2.4E5 | 1.7E5 | 1.9E5 | 3.9E4 | 1.9E2 | 6.9E5 | 8.7E5 | 71 | 44 | 71 | 44 | 0.55 |
| fY | ng/ml | 2.6E2 | 2.5E2 | 2.5E2 | 2.5E2 | 9.4E1 | 1.2E2 | 6.5E1 | 3.6E1 | 3.9E2 | 4.8E2 | 33 | 22 | 33 | 22 | 0.49 |
| gC | ng/ml | 2.5E2 | 2.5E2 | 2.7E2 | 2.6E2 | 1.2E2 | 1.2E2 | 1.4E2 | 8.3E1 | 6.4E2 | 5.9E2 | 23 | 24 | 23 | 24 | 0.45 |
| gL | pg/ml | 6.8E4 | 6.5E4 | 7.6E4 | 7.3E4 | 3.7E4 | 3.8E4 | 1.4E4 | 1.1E4 | 1.9E5 | 2.2E5 | 74 | 59 | 74 | 59 | 0.46 |
| gP | U/ml | 2.7E2 | 2.8E2 | 2.9E2 | 2.9E2 | 1.4E2 | 9.8E1 | 1.1E2 | 1.2E1 | 1.1E3 | 5.2E2 | 74 | 58 | 74 | 58 | 0.53 |
| gW | ng/ml | 6.1E2 | 3.7E2 | 1.0E3 | 7.2E2 | 1.1E3 | 8.8E2 | 2.3E0 | 5.3E1 | 6.1E3 | 4.2E3 | 61 | 38 | 61 | 38 | 0.41 |
| gV | ng/ml | 2.1E1 | 2.2E1 | 2.1E1 | 2.2E1 | 6.8E0 | 8.8E0 | 1.0E1 | 8.1E-2 | 3.7E1 | 3.4E1 | 25 | 13 | 25 | 13 | 0.58 |
| lF | pg/mL | 9.5E2 | 1.9E3 | 1.1E4 | 1.3E4 | 3.9E4 | 3.9E4 | 1.8E1 | 1.2E1 | 2.8E5 | 2.5E5 | 64 | 55 | 64 | 55 | 0.55 |
| gZ | ug/ml | 5.3E-1 | 8.5E-1 | 3.2E1 | 4.6E1 | 1.1E2 | 1.1E2 | 8.7E-2 | 1.4E-1 | 4.1E2 | 4.0E2 | 13 | 20 | 13 | 20 | 0.64 |
| hA | ng/ml | 2.2E0 | 3.0E0 | 1.3E1 | 1.7E1 | 5.0E1 | 5.1E1 | 1.7E-2 | 1.7E-2 | 3.5E2 | 2.9E2 | 60 | 36 | 60 | 36 | 0.60 |
| nM | pg/ml | 1.0E-9 | 1.0E-9 | 1.0E-9 | 4.5E1 | 0.0E0 | 2.6E2 | 1.0E-9 | 1.0E-9 | 1.0E-9 | 1.5E3 | 40 | 34 | 40 | 34 | 0.51 |
| nN | pg/ml | 1.1E3 | 2.2E3 | 1.8E3 | 1.2E4 | 1.9E3 | 3.1E4 | 8.1E1 | 3.5E2 | 7.1E3 | 1.5E5 | 40 | 34 | 40 | 34 | 0.68 |
| nO | pg/ml | 2.2E1 | 2.9E1 | 3.6E1 | 4.1E1 | 5.1E1 | 4.0E1 | 6.7E0 | 4.0E0 | 3.1E2 | 2.0E2 | 40 | 34 | 40 | 34 | 0.59 |
| nR | pg/ml | 1.5E1 | 2.3E1 | 4.4E1 | 1.4E2 | 6.7E1 | 3.6E2 | 1.4E0 | 1.0E0 | 2.6E2 | 1.9E3 | 40 | 34 | 40 | 34 | 0.59 |
| nT | pg/ml | 4.9E1 | 1.0E2 | 9.2E1 | 1.5E2 | 9.4E1 | 1.8E2 | 1.0E-9 | 1.0E-9 | 4.9E2 | 9.2E2 | 40 | 34 | 40 | 34 | 0.62 |
| nU | pg/ml | 4.4E1 | 4.4E1 | 7.1E1 | 1.4E2 | 1.2E2 | 3.0E2 | 1.0E-9 | 1.0E-9 | 7.5E2 | 1.5E3 | 40 | 34 | 40 | 34 | 0.52 |
| lW | pg/ml | 1.0E-9 | 1.0E-9 | 6.5E0 | 1.6E1 | 1.4E1 | 4.1E1 | 1.0E-9 | 1.0E-9 | 5.5E1 | 1.7E2 | 40 | 34 | 40 | 34 | 0.55 |
| lX | pg/ml | 1.0E3 | 8.0E2 | 1.1E3 | 9.2E2 | 5.8E2 | 5.7E2 | 3.2E2 | 1.9E2 | 2.6E3 | 2.5E3 | 40 | 34 | 40 | 34 | 0.41 |
| lY | pg/ml | 1.9E1 | 1.7E1 | 2.0E1 | 2.1E1 | 1.2E1 | 2.2E1 | 1.0E-9 | 1.0E-9 | 4.8E1 | 1.2E2 | 40 | 34 | 40 | 34 | 0.46 |
| mE | pg/ml | 1.0E-9 | 1.0E-9 | 2.0E0 | 3.3E0 | 4.7E0 | 9.9E0 | 1.0E-9 | 1.0E-9 | 2.8E1 | 5.7E1 | 40 | 34 | 40 | 34 | 0.51 |
| mF | pg/ml | 2.3E-1 | 4.8E-1 | 7.9E0 | 5.1E0 | 3.9E1 | 1.8E1 | 1.0E-9 | 1.0E-9 | 2.5E2 | 1.1E2 | 40 | 34 | 40 | 34 | 0.52 |
| mH | pg/ml | 4.0E0 | 2.9E0 | 4.8E0 | 6.2E0 | 5.2E0 | 1.0E1 | 4.0E-1 | 4.0E-1 | 3.2E1 | 5.3E1 | 40 | 34 | 40 | 34 | 0.47 |
| mI | pg/ml | 1.0E-9 | 1.7E0 | 9.4E0 | 3.0E1 | 1.5E1 | 8.4E1 | 1.0E-9 | 1.0E-9 | 6.1E1 | 4.6E2 | 40 | 34 | 40 | 34 | 0.54 |
| mM | pg/ml | 2.8E1 | 3.8E1 | 5.9E1 | 1.1E2 | 7.8E1 | 2.1E2 | 1.0E-9 | 1.0E-9 | 4.0E2 | 1.1E3 | 40 | 34 | 40 | 34 | 0.52 |
| mP | pg/ml | 1.6E1 | 1.5E1 | 1.6E1 | 4.6E1 | 1.1E1 | 1.4E2 | 1.0E-9 | 1.0E-9 | 5.8E1 | 8.1E2 | 39 | 34 | 39 | 34 | 0.50 |
| mS | pg/ml | 1.6E3 | 1.6E3 | 1.8E3 | 1.7E3 | 8.6E2 | 1.2E3 | 8.8E1 | 1.0E-9 | 3.7E3 | 5.1E3 | 40 | 34 | 40 | 34 | 0.45 |
| mT | pg/ml | 5.0E1 | 6.0E1 | 1.4E2 | 1.7E2 | 2.5E2 | 3.5E2 | 1.0E1 | 1.2E1 | 1.4E3 | 1.7E3 | 39 | 34 | 39 | 34 | 0.50 |
| mU | pg/ml | 2.3E0 | 2.1E0 | 3.2E0 | 1.0E1 | 2.7E0 | 3.8E1 | 1.0E-9 | 1.0E-9 | 1.6E1 | 2.2E2 | 39 | 34 | 39 | 34 | 0.49 |
| mW | pg/ml | 2.5E3 | 1.9E3 | 2.5E3 | 2.5E3 | 1.2E3 | 2.1E3 | 1.0E-9 | 3.7E2 | 4.9E3 | 1.1E4 | 39 | 34 | 39 | 34 | 0.38 |
| mY | pg/ml | 6.5E2 | 6.5E2 | 8.4E2 | 1.1E3 | 8.8E2 | 1.6E3 | 1.0E-9 | 1.0E-9 | 4.2E3 | 8.0E3 | 40 | 34 | 40 | 34 | 0.54 |
| mZ | pg/ml | 1.8E2 | 1.7E2 | 3.8E2 | 3.3E2 | 5.5E2 | 3.4E2 | 1.0E-9 | 1.1E1 | 3.1E3 | 1.2E3 | 39 | 34 | 39 | 34 | 0.49 |

| Code | Units | Median | | Average | | Standard Deviation | | Minimum | | Maximum | | Number of Samples | | Number of Patients | | AUC |
|------|-------|--------|------|---------|------|-----|------|---------|------|---------|------|---------|------|---------|------|-----|
| | | NonDis | Dis | NonDis | Dis | NonDis | Dis | NonDis | Dis | NonDis | Dis | NonDis | Dis | NonDis | Dis | |
| nA | pg/ml | 2.5E0 | 9.1E-1 | 8.4E0 | 5.6E0 | 1.5E1 | 1.3E1 | 1.0E-9 | 1.0E-9 | 5.7E1 | 6.5E1 | 39 | 34 | 39 | 34 | 0.41 |
| nB | pg/ml | 2.6E2 | 3.0E2 | 3.0E2 | 3.4E2 | 1.5E2 | 2.0E2 | 3.0E1 | 3.8E1 | 6.9E2 | 9.6E2 | 40 | 34 | 40 | 34 | 0.54 |
| nC | pg/ml | 1.0E-9 | 6.6E1 | 1.1E4 | 7.2E3 | 6.1E4 | 3.8E4 | 1.0E-9 | 1.0E-9 | 3.8E5 | 2.2E5 | 40 | 34 | 40 | 34 | 0.52 |
| nD | pg/ml | 9.4E0 | 4.5E0 | 1.3E1 | 1.5E1 | 1.9E1 | 4.4E1 | 1.0E-9 | 1.0E-9 | 1.2E2 | 2.6E2 | 39 | 34 | 39 | 34 | 0.40 |
| nF | pg/ml | 1.0E-9 | 1.0E-9 | 4.8E0 | 1.2E0 | 1.7E1 | 5.2E0 | 1.0E-9 | 1.0E-9 | 9.3E1 | 2.7E1 | 40 | 34 | 40 | 34 | 0.45 |
| nH | pg/ml | 7.4E-1 | 1.3E0 | 8.2E1 | 3.0E2 | 4.2E2 | 1.7E3 | 1.0E-9 | 1.0E-9 | 2.6E3 | 1.0E4 | 39 | 34 | 39 | 34 | 0.54 |
| nI | pg/ml | 4.6E1 | 1.0E-9 | 8.6E1 | 4.7E1 | 1.9E2 | 8.4E1 | 1.0E-9 | 1.0E-9 | 1.2E3 | 3.5E2 | 40 | 34 | 40 | 34 | 0.38 |
| nJ | pg/ml | 6.1E-1 | 1.7E-1 | 1.3E0 | 4.8E0 | 2.6E0 | 2.3E1 | 1.0E-9 | 1.0E-9 | 1.5E1 | 1.3E2 | 40 | 34 | 40 | 34 | 0.45 |
| nK | pg/ml | 1.0E-9 | 1.0E-9 | 1.9E1 | 1.3E1 | 4.1E1 | 2.3E1 | 1.0E-9 | 1.0E-9 | 2.2E2 | 1.0E2 | 39 | 34 | 39 | 34 | 0.49 |
| nL | pg/ml | 1.0E-9 | 1.2E0 | 1.4E2 | 4.4E2 | 6.9E2 | 2.3E3 | 1.0E-9 | 1.0E-9 | 4.3E3 | 1.4E4 | 40 | 34 | 40 | 34 | 0.54 |
| hL | pg/ml | 1.5E4 | 2.5E4 | 2.1E4 | 2.5E4 | 1.5E4 | 1.4E4 | 2.6E3 | 5.1E3 | 7.2E4 | 6.0E4 | 33 | 22 | 33 | 22 | 0.63 |
| hO | pg/ml | 1.6E4 | 1.6E4 | 1.6E4 | 1.6E4 | 2.8E3 | 2.4E3 | 1.3E4 | 1.1E4 | 2.4E4 | 2.1E4 | 33 | 22 | 33 | 22 | 0.50 |
| hP | ng/ml | 4.4E5 | 4.4E5 | 3.8E5 | 6.3E5 | 1.7E5 | 6.9E5 | 4.7E4 | 1.7E4 | 9.0E5 | 2.8E6 | 33 | 22 | 33 | 22 | 0.61 |
| wJ | pg/ml | 1.4E5 | 1.1E5 | 1.5E5 | 1.9E5 | 8.1E4 | 1.5E5 | 1.1E4 | 1.3E4 | 3.6E5 | 5.8E5 | 33 | 24 | 33 | 24 | 0.53 |
| wK | pg/ml | 3.2E4 | 3.6E4 | 4.2E4 | 6.0E4 | 3.0E4 | 9.8E4 | 3.7E3 | 7.5E3 | 1.3E5 | 5.0E5 | 33 | 24 | 33 | 24 | 0.52 |
| wL | pg/ml | 3.9E0 | 8.1E0 | 4.4E1 | 5.7E1 | 1.5E2 | 1.3E2 | 1.0E-9 | 1.0E-9 | 8.4E2 | 4.7E2 | 33 | 24 | 33 | 24 | 0.54 |
| wP | pg/ml | 2.4E4 | 3.4E4 | 3.7E4 | 6.4E4 | 3.9E4 | 7.4E4 | 1.1E3 | 1.3E3 | 1.7E5 | 3.0E5 | 33 | 24 | 33 | 24 | 0.59 |
| wQ | pg/ml | 3.1E1 | 3.6E1 | 5.8E1 | 6.4E1 | 8.2E1 | 1.1E2 | 1.0E-9 | 1.0E-9 | 3.7E2 | 5.1E2 | 33 | 24 | 33 | 24 | 0.56 |
| hR | pg/ml | 2.9E4 | 2.4E4 | 3.1E4 | 2.6E4 | 1.2E4 | 1.0E4 | 1.0E-9 | 4.3E3 | 5.8E4 | 4.9E4 | 54 | 35 | 54 | 35 | 0.37 |
| hV | pg/ml | 5.0E2 | 4.3E2 | 4.7E2 | 4.3E2 | 2.4E2 | 2.2E2 | 1.0E-9 | 8.2E1 | 1.2E3 | 9.6E2 | 54 | 35 | 54 | 35 | 0.45 |
| hW | pg/ml | 1.7E3 | 2.3E3 | 2.0E3 | 3.4E3 | 1.2E3 | 6.4E3 | 1.0E-9 | 1.1E3 | 7.3E3 | 4.0E4 | 54 | 35 | 54 | 35 | 0.63 |
| hX | pg/ml | 1.1E3 | 1.1E3 | 1.2E3 | 1.1E3 | 1.1E3 | 5.4E2 | 2.5E0 | 1.3E2 | 8.6E3 | 2.9E3 | 54 | 35 | 54 | 35 | 0.48 |
| iA | pg/ml | 1.8E2 | 1.2E2 | 2.7E2 | 2.3E2 | 3.0E2 | 2.4E2 | 1.6E1 | 1.5E1 | 1.8E3 | 8.7E2 | 64 | 55 | 64 | 55 | 0.44 |
| iB | ng/ml | 4.7E0 | 6.1E0 | 6.1E0 | 7.5E0 | 5.1E0 | 5.1E0 | 3.3E-2 | 1.6E0 | 2.4E1 | 2.2E1 | 60 | 36 | 60 | 36 | 0.62 |
| iC | U/ml | 3.0E-1 | 3.7E-1 | 1.6E0 | 6.1E-1 | 7.1E0 | 7.2E-1 | 1.0E-9 | 3.7E-2 | 5.5E1 | 3.2E0 | 60 | 36 | 60 | 36 | 0.57 |
| tQ | pg/ml | 1.3E3 | 1.4E3 | 1.4E3 | 1.5E3 | 5.5E2 | 6.4E2 | 3.7E2 | 7.2E2 | 2.5E3 | 3.3E3 | 32 | 21 | 32 | 21 | 0.50 |
| tT | pg/ml | 1.6E1 | 2.1E1 | 1.8E1 | 2.6E1 | 9.4E0 | 1.8E1 | 5.4E0 | 1.0E1 | 4.8E1 | 9.3E1 | 32 | 22 | 32 | 22 | 0.65 |
| tS | pg/ml | 1.0E0 | 7.7E-1 | 1.5E0 | 1.5E0 | 1.8E0 | 2.2E0 | 1.0E-9 | 1.0E-9 | 8.5E0 | 1.0E1 | 32 | 23 | 32 | 23 | 0.48 |
| tX | pg/ml | 9.9E-1 | 1.3E0 | 1.2E0 | 2.5E0 | 1.0E0 | 2.7E0 | 2.5E-2 | 3.2E-1 | 4.4E0 | 1.0E1 | 32 | 22 | 32 | 22 | 0.63 |
| tO | pg/ml | 4.4E0 | 3.9E0 | 4.9E0 | 5.6E0 | 3.4E0 | 4.0E0 | 1.0E-9 | 1.7E0 | 1.4E1 | 1.8E1 | 32 | 23 | 32 | 23 | 0.55 |
| tR | pg/ml | 1.9E-1 | 2.5E-1 | 2.9E-1 | 4.2E-1 | 3.4E-1 | 5.9E-1 | 1.0E-9 | 1.0E-9 | 1.5E0 | 2.5E0 | 32 | 22 | 32 | 22 | 0.55 |
| tU | pg/ml | 9.9E0 | 1.0E1 | 1.1E1 | 1.6E1 | 7.2E0 | 1.8E1 | 1.5E0 | 2.2E-1 | 3.1E1 | 8.0E1 | 32 | 24 | 32 | 24 | 0.53 |
| tN | pg/ml | 2.0E1 | 2.1E1 | 2.5E1 | 4.0E1 | 1.9E1 | 4.3E1 | 1.0E-9 | 1.0E-9 | 8.0E1 | 1.6E2 | 31 | 22 | 31 | 22 | 0.59 |
| tV | ng/ml | 4.5E2 | 1.1E3 | 6.5E2 | 9.9E2 | 5.6E2 | 6.7E2 | 1.9E2 | 5.3E1 | 2.9E3 | 3.1E3 | 33 | 23 | 33 | 23 | 0.67 |
| iH | ng/ml | 1.6E5 | 1.7E5 | 1.6E5 | 1.6E5 | 4.9E4 | 5.1E4 | 7.1E4 | 2.9E3 | 2.6E5 | 2.4E5 | 64 | 55 | 64 | 55 | 0.48 |
| iJ | ng/ml | 5.2E4 | 4.6E4 | 5.8E4 | 5.2E4 | 3.5E4 | 3.6E4 | 5.5E3 | 1.8E3 | 2.5E5 | 2.5E5 | 64 | 55 | 64 | 55 | 0.44 |
| hB | ng/ml | 4.8E-1 | 4.9E-1 | 6.2E-1 | 6.7E-1 | 4.7E-1 | 5.4E-1 | 1.4E-1 | 1.2E-1 | 2.3E0 | 3.2E0 | 64 | 55 | 64 | 55 | 0.53 |
| hC | pg/ml | 3.6E3 | 7.5E3 | 6.2E3 | 1.1E4 | 6.8E3 | 1.6E4 | 6.0E1 | 4.1E1 | 3.6E4 | 1.1E5 | 64 | 55 | 64 | 55 | 0.60 |
| hF | ng/ml | 1.0E-9 | 1.0E-9 | 6.3E1 | 1.0E-9 | 5.0E2 | 0.0E0 | 1.0E-9 | 1.0E-9 | 4.0E3 | 1.0E-9 | 64 | 55 | 64 | 55 | 0.49 |
| hG | pg/ml | 6.9E3 | 6.6E3 | 7.5E3 | 7.7E3 | 2.9E3 | 3.9E3 | 1.8E3 | 2.3E3 | 1.8E4 | 2.0E4 | 64 | 55 | 64 | 55 | 0.47 |
| iO | ng/ml | 3.7E5 | 4.0E5 | 4.1E5 | 4.3E5 | 2.1E5 | 1.8E5 | 1.1E4 | 9.8E4 | 1.1E6 | 8.2E5 | 64 | 55 | 64 | 55 | 0.55 |
| iP | ng/ml | 5.4E4 | 5.1E4 | 6.0E4 | 5.5E4 | 5.4E4 | 3.4E4 | 1.0E-9 | 7.1E3 | 4.4E5 | 2.2E5 | 64 | 55 | 64 | 55 | 0.47 |
| iZ | ng/ml | 1.5E3 | 1.9E3 | 1.8E3 | 2.0E3 | 9.1E2 | 8.8E2 | 6.6E2 | 7.5E2 | 5.7E3 | 4.6E3 | 64 | 53 | 64 | 53 | 0.58 |
| yH | pg/ml | 1.2E3 | 9.0E2 | 1.9E3 | 3.0E3 | 2.8E3 | 7.0E3 | 1.0E-9 | 1.0E-9 | 1.5E4 | 2.5E4 | 33 | 22 | 33 | 22 | 0.41 |
| yK | U/ml | 2.1E1 | 1.9E1 | 4.9E1 | 2.8E1 | 8.5E1 | 3.3E1 | 1.0E-9 | 1.0E-9 | 3.8E2 | 1.4E2 | 33 | 22 | 33 | 22 | 0.44 |
| yJ | pg/ml | 4.4E4 | 3.0E4 | 4.6E4 | 3.2E4 | 3.0E4 | 2.1E4 | 9.2E3 | 1.9E3 | 1.4E5 | 8.2E4 | 33 | 22 | 33 | 22 | 0.36 |
| yD | ng/ml | 1.2E-2 | 1.4E-2 | 1.3E-2 | 1.3E-2 | 5.6E-3 | 6.1E-3 | 1.0E-9 | 1.0E-9 | 2.8E-2 | 2.4E-2 | 33 | 24 | 33 | 24 | 0.53 |
| jB | ng/ml | 2.5E5 | 2.2E5 | 2.7E5 | 2.2E5 | 7.0E4 | 7.3E4 | 1.5E5 | 9.9E4 | 3.6E5 | 3.5E5 | 13 | 20 | 13 | 20 | 0.32 |
| wB | pg/ml | 8.8E3 | 1.2E4 | 1.0E4 | 1.4E4 | 7.0E3 | 1.1E4 | 1.7E3 | 1.9E3 | 3.3E4 | 4.2E4 | 33 | 24 | 33 | 24 | 0.59 |
| pY | pg/ml | 5.3E0 | 6.4E0 | 1.1E1 | 7.2E0 | 3.4E1 | 4.0E0 | 2.3E0 | 1.6E0 | 2.0E2 | 1.8E1 | 33 | 22 | 33 | 22 | 0.62 |
| sI | ng/ml | 5.2E-2 | 5.7E-2 | 5.1E-2 | 6.2E-2 | 1.4E-2 | 3.9E-2 | 2.1E-2 | 1.0E-2 | 7.3E-2 | 1.5E-1 | 15 | 14 | 15 | 14 | 0.55 |
| sF | mIU/mL | 1.1E1 | 5.0E0 | 1.5E1 | 1.4E1 | 2.0E1 | 2.2E1 | 6.2E-1 | 1.2E0 | 8.1E1 | 7.5E1 | 15 | 14 | 15 | 14 | 0.36 |
| sH | mIU/mL | 5.2E0 | 1.6E0 | 5.7E0 | 4.6E0 | 6.0E0 | 6.3E0 | 1.0E-9 | 7.9E-2 | 2.5E1 | 2.1E1 | 15 | 14 | 15 | 14 | 0.36 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| sJ | ng/ml | 1.5E-1 | 1.5E-1 | 4.2E-1 | 6.2E-1 | 8.4E-1 | 1.7E0 | 1.0E-9 | 1.0E-9 | 3.3E0 | 6.4E0 | 15 | 14 | 15 | 14 | 0.50 |
| rC | pg/ml | 1.8E3 | 1.2E3 | 2.3E3 | 1.9E3 | 2.4E3 | 2.2E3 | 1.1E2 | 1.0E-9 | 1.5E4 | 1.1E4 | 53 | 33 | 53 | 33 | 0.42 |
| rB | pg/ml | 3.1E1 | 2.9E1 | 4.6E1 | 6.2E1 | 6.3E1 | 7.6E1 | 1.0E-9 | 1.0E-9 | 3.9E2 | 3.2E2 | 53 | 33 | 53 | 33 | 0.52 |
| zG | 2.5ng/ml | 2.2E-1 | 2.3E-1 | 7.1E-1 | 3.7E-1 | 1.2E0 | 5.8E-1 | 1.0E-9 | 1.0E-9 | 4.8E0 | 2.7E0 | 33 | 22 | 33 | 22 | 0.47 |
| zH | 2.3mU/ml | 9.2E-2 | 8.8E-2 | 1.0E-1 | 9.2E-2 | 5.1E-2 | 3.8E-2 | 1.0E-2 | 2.1E-2 | 3.1E-1 | 1.8E-1 | 33 | 22 | 33 | 22 | 0.44 |
| zI | 2.6ng/ml | 2.2E0 | 2.0E0 | 3.7E0 | 5.7E0 | 4.0E0 | 7.6E0 | 6.3E-1 | 5.4E-1 | 1.6E1 | 2.7E1 | 33 | 22 | 33 | 22 | 0.51 |
| qA | ng/ml | 8.3E6 | 1.2E7 | 1.1E7 | 1.3E7 | 7.8E6 | 6.7E6 | 3.7E6 | 4.3E6 | 3.9E7 | 3.0E7 | 33 | 22 | 33 | 22 | 0.62 |
| qB | ng/ml | 6.2E5 | 7.9E5 | 7.7E5 | 9.7E5 | 5.4E5 | 8.3E5 | 1.9E5 | 2.4E5 | 2.9E6 | 3.8E6 | 33 | 22 | 33 | 22 | 0.56 |
| qC | ng/ml | 3.1E5 | 2.7E5 | 6.5E5 | 7.1E5 | 1.0E6 | 1.1E6 | 2.5E4 | 6.5E4 | 5.2E6 | 4.7E6 | 33 | 22 | 33 | 22 | 0.52 |
| qD | ng/ml | 1.5E7 | 1.5E7 | 1.7E7 | 1.8E7 | 8.8E6 | 8.1E6 | 1.2E6 | 8.7E6 | 4.5E7 | 3.7E7 | 33 | 22 | 33 | 22 | 0.54 |
| jD | ng/ml | 2.4E1 | 4.5E1 | 3.6E1 | 5.5E1 | 3.9E1 | 6.0E1 | 1.0E-9 | 1.0E-9 | 1.9E2 | 2.9E2 | 60 | 36 | 60 | 36 | 0.61 |
| jE | ng/ml | 1.0E-9 | 1.0E-9 | 5.4E0 | 6.0E0 | 1.3E1 | 1.0E1 | 1.0E-9 | 1.0E-9 | 6.2E1 | 3.5E1 | 60 | 36 | 60 | 36 | 0.55 |
| jF | ng/ml | 4.7E1 | 2.1E1 | 5.2E1 | 4.2E1 | 5.3E1 | 5.3E1 | 1.0E-9 | 1.0E-9 | 2.1E2 | 1.9E2 | 60 | 36 | 60 | 36 | 0.44 |
| jG | ng/ml | 4.2E3 | 4.3E3 | 4.5E3 | 4.6E3 | 1.9E3 | 2.0E3 | 6.7E2 | 1.4E3 | 8.9E3 | 9.6E3 | 60 | 36 | 60 | 36 | 0.50 |
| jH | ng/ml | 7.7E1 | 7.7E1 | 7.9E1 | 9.5E1 | 3.9E1 | 7.6E1 | 1.3E1 | 1.5E1 | 2.1E2 | 4.3E2 | 60 | 36 | 60 | 36 | 0.53 |
| jI | ng/ml | 7.3E1 | 8.6E1 | 7.3E1 | 1.0E2 | 3.0E1 | 7.5E1 | 1.9E1 | 3.8E1 | 1.9E2 | 4.4E2 | 60 | 36 | 60 | 36 | 0.61 |
| sK | pg/mL | 3.7E3 | 4.3E3 | 3.8E3 | 5.4E3 | 1.3E3 | 4.7E3 | 1.8E3 | 2.1E3 | 7.1E3 | 2.3E4 | 32 | 21 | 32 | 21 | 0.58 |
| sM | pg/mL | 7.3E4 | 7.4E4 | 7.6E4 | 8.8E4 | 2.2E4 | 4.3E4 | 4.3E4 | 3.9E4 | 1.5E5 | 2.0E5 | 32 | 21 | 32 | 21 | 0.55 |
| sO | pg/mL | 2.4E8 | 2.1E8 | 2.5E8 | 2.2E8 | 8.3E7 | 1.0E8 | 4.9E7 | 6.6E7 | 4.4E8 | 4.2E8 | 32 | 21 | 32 | 21 | 0.41 |
| wC | ng/ml | 1.5E0 | 1.5E0 | 2.1E0 | 1.6E0 | 1.5E0 | 1.0E0 | 3.6E-1 | 6.1E-2 | 6.5E0 | 4.8E0 | 33 | 24 | 33 | 24 | 0.46 |
| wD | ng/ml | 2.1E1 | 2.8E1 | 9.9E1 | 5.4E1 | 3.6E2 | 6.3E1 | 3.8E0 | 2.8E0 | 2.1E3 | 2.9E2 | 33 | 24 | 33 | 24 | 0.66 |
| wE | ng/ml | 4.9E1 | 5.0E1 | 4.9E1 | 5.2E1 | 2.1E1 | 1.9E1 | 8.1E0 | 2.0E1 | 9.4E1 | 8.9E1 | 33 | 24 | 33 | 24 | 0.52 |
| wG | ng/ml | 1.0E-1 | 1.0E-1 | 1.4E-1 | 1.4E-1 | 1.6E-1 | 1.6E-1 | 1.0E-9 | 1.0E-9 | 7.1E-1 | 6.8E-1 | 33 | 24 | 33 | 24 | 0.50 |
| wH | ng/ml | 1.9E-2 | 4.9E-2 | 2.7E-1 | 4.6E-1 | 7.8E-1 | 1.2E0 | 1.0E-9 | 1.0E-9 | 4.2E0 | 5.6E0 | 33 | 24 | 33 | 24 | 0.59 |
| wF | ng/ml | 1.2E-1 | 3.7E-1 | 2.7E0 | 1.7E0 | 1.1E1 | 3.9E0 | 1.0E-9 | 1.0E-9 | 5.7E1 | 1.9E1 | 33 | 24 | 33 | 24 | 0.65 |
| rA | pg/ml | 2.4E1 | 2.4E1 | 3.1E1 | 2.9E1 | 2.9E1 | 2.4E1 | 1.0E-9 | 1.0E-9 | 2.0E2 | 1.1E2 | 59 | 34 | 59 | 34 | 0.47 |
| qZ | pg/ml | 4.6E1 | 5.8E1 | 5.2E2 | 6.0E2 | 2.1E3 | 2.0E3 | 2.8E-4 | 5.9E-4 | 1.0E4 | 1.0E4 | 43 | 26 | 43 | 26 | 0.56 |
| qY | pg/ml | 1.8E1 | 1.4E1 | 4.1E1 | 3.4E1 | 5.1E1 | 6.3E1 | 8.7E-1 | 2.1E0 | 2.3E2 | 3.3E2 | 59 | 34 | 59 | 34 | 0.40 |
| qX | pg/ml | 6.1E1 | 5.8E1 | 7.0E1 | 7.4E1 | 5.1E1 | 4.7E1 | 1.0E-9 | 1.0E-9 | 2.3E2 | 2.1E2 | 59 | 34 | 59 | 34 | 0.53 |
| qW | pg/ml | 7.8E0 | 7.3E0 | 1.3E1 | 8.6E0 | 1.8E1 | 8.4E0 | 1.0E-9 | 1.0E-9 | 1.2E2 | 3.1E1 | 59 | 34 | 59 | 34 | 0.41 |
| qV | pg/ml | 1.8E3 | 1.9E3 | 2.6E3 | 2.4E3 | 2.1E3 | 1.9E3 | 1.7E2 | 1.0E2 | 1.1E4 | 9.6E3 | 59 | 34 | 59 | 34 | 0.48 |
| qU | pg/ml | 4.8E1 | 1.0E2 | 1.5E2 | 2.7E2 | 2.3E2 | 4.1E2 | 1.0E-9 | 1.0E-9 | 1.2E3 | 2.1E3 | 59 | 34 | 59 | 34 | 0.61 |
| qT | pg/ml | 3.9E1 | 4.1E1 | 6.8E1 | 6.1E1 | 8.0E1 | 5.3E1 | 1.0E-9 | 1.0E-9 | 4.9E2 | 2.6E2 | 59 | 34 | 59 | 34 | 0.52 |
| qI | ng/ml | 5.5E4 | 6.4E4 | 6.1E4 | 6.3E4 | 3.3E4 | 2.5E4 | 1.0E4 | 2.5E4 | 1.6E5 | 1.3E5 | 32 | 20 | 32 | 20 | 0.53 |
| qH | ng/ml | 5.5E4 | 6.1E4 | 6.7E4 | 7.5E4 | 4.1E4 | 3.9E4 | 1.5E4 | 3.2E4 | 1.8E5 | 1.8E5 | 32 | 20 | 32 | 20 | 0.57 |
| qG | ng/ml | 1.9E5 | 1.8E5 | 1.9E5 | 1.9E5 | 6.4E4 | 7.8E4 | 3.4E4 | 8.4E4 | 3.0E5 | 4.2E5 | 32 | 20 | 32 | 20 | 0.45 |
| jK | ng/ml | 1.6E3 | 1.4E3 | 1.6E3 | 1.6E3 | 5.3E2 | 7.6E2 | 2.8E2 | 7.0E2 | 3.1E3 | 4.1E3 | 60 | 36 | 60 | 36 | 0.41 |
| jL | ng/ml | 1.7E2 | 2.5E2 | 2.7E2 | 2.9E2 | 2.1E2 | 1.8E2 | 5.6E1 | 6.4E1 | 9.6E2 | 7.6E2 | 60 | 36 | 60 | 36 | 0.58 |
| jM | ng/ml | 6.8E4 | 7.4E4 | 7.5E4 | 7.6E4 | 3.7E4 | 4.5E4 | 2.1E4 | 4.6E3 | 1.8E5 | 1.7E5 | 60 | 36 | 60 | 36 | 0.50 |
| jO | pg/ml | 2.1E5 | 2.6E5 | 2.8E5 | 2.7E5 | 1.8E5 | 1.4E5 | 6.0E4 | 9.6E4 | 1.1E6 | 6.5E5 | 60 | 36 | 60 | 36 | 0.52 |
| jP | pg/ml | 2.4E5 | 3.2E5 | 2.6E5 | 3.4E5 | 1.3E5 | 1.6E5 | 3.6E4 | 1.3E5 | 7.1E5 | 7.0E5 | 60 | 36 | 60 | 36 | 0.64 |
| jQ | pg/ml | 2.7E3 | 1.8E3 | 3.3E3 | 2.9E3 | 2.8E3 | 3.1E3 | 7.3E1 | 5.0E0 | 1.3E4 | 1.3E4 | 60 | 36 | 60 | 36 | 0.43 |
| jR | pg/ml | 7.1E3 | 4.2E3 | 1.0E4 | 1.1E4 | 1.1E4 | 1.4E4 | 3.0E1 | 1.0E-9 | 6.8E4 | 5.6E4 | 60 | 36 | 60 | 36 | 0.45 |
| jT | pg/ml | 1.7E5 | 1.8E5 | 1.8E5 | 1.8E5 | 7.2E4 | 6.9E4 | 7.1E4 | 7.5E4 | 5.5E5 | 3.5E5 | 60 | 36 | 60 | 36 | 0.52 |
| xA | pg/ml | 3.9E0 | 8.9E0 | 1.0E1 | 1.9E1 | 1.5E1 | 3.9E1 | 1.0E-9 | 1.0E-9 | 6.1E1 | 1.6E2 | 33 | 22 | 33 | 22 | 0.56 |
| yE | pg/ml | 8.1E1 | 9.1E1 | 8.2E1 | 8.9E1 | 2.9E1 | 4.3E1 | 3.6E1 | 6.4E0 | 1.4E2 | 2.0E2 | 33 | 22 | 33 | 22 | 0.55 |
| tM | pg/ml | 4.3E1 | 4.2E1 | 4.0E1 | 4.1E1 | 1.8E1 | 1.9E1 | 1.0E-9 | 1.0E-9 | 8.3E1 | 9.9E1 | 33 | 22 | 33 | 22 | 0.52 |
| tL | pg/ml | 1.0E-9 | 1.0E-9 | 2.2E0 | 4.5E-1 | 1.2E1 | 9.5E-1 | 1.0E-9 | 1.0E-9 | 6.7E1 | 3.6E0 | 33 | 22 | 33 | 22 | 0.57 |
| jU | mIU/ml | 6.2E0 | 3.8E0 | 1.3E1 | 1.0E1 | 2.1E1 | 1.3E1 | 8.1E-2 | 5.3E-1 | 1.1E2 | 5.7E1 | 60 | 36 | 60 | 36 | 0.45 |
| jV | mIU/ml | 2.5E0 | 1.6E0 | 4.7E0 | 2.9E0 | 6.5E0 | 3.4E0 | 2.7E-3 | 2.1E-2 | 3.2E1 | 1.5E1 | 60 | 36 | 60 | 36 | 0.43 |
| jY | ng/ml | 7.3E-4 | 1.8E-3 | 9.0E-3 | 5.4E-3 | 4.0E-2 | 9.9E-3 | 1.0E-9 | 1.0E-9 | 3.0E-1 | 5.1E-2 | 60 | 36 | 60 | 36 | 0.58 |
| kC | pg/ml | 9.7E1 | 1.1E2 | 1.4E2 | 2.5E2 | 1.7E2 | 4.8E2 | 2.1E1 | 3.6E1 | 1.1E3 | 2.7E3 | 40 | 34 | 40 | 34 | 0.56 |
| kE | pg/ml | 1.4E5 | 1.4E5 | 1.4E5 | 1.4E5 | 4.0E4 | 3.9E4 | 3.8E4 | 7.5E4 | 2.3E5 | 2.7E5 | 40 | 34 | 40 | 34 | 0.53 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| kF | pg/mL | 6.4E1 | 6.8E1 | 6.4E1 | 7.5E1 | 1.8E1 | 3.1E1 | 2.7E1 | 4.0E1 | 1.1E2 | 1.5E2 | 40 | 34 | 40 | 34 | 0.56 |
| kG | pg/mL | 7.5E3 | 9.0E3 | 1.1E4 | 1.7E4 | 1.2E4 | 2.8E4 | 1.3E3 | 1.1E3 | 5.8E4 | 1.6E5 | 40 | 34 | 40 | 34 | 0.58 |
| kI | pg/ml | 1.9E2 | 2.1E2 | 2.2E2 | 2.1E2 | 1.3E2 | 9.6E1 | 6.4E1 | 1.0E-9 | 6.7E2 | 4.6E2 | 40 | 34 | 40 | 34 | 0.53 |
| kK | pg/ml | 1.2E2 | 1.2E2 | 1.6E2 | 1.6E2 | 1.7E2 | 1.2E2 | 6.4E0 | 2.1E1 | 9.1E2 | 4.9E2 | 40 | 34 | 40 | 34 | 0.52 |
| kN | pg/ml | 9.5E2 | 1.2E3 | 1.4E3 | 1.8E3 | 1.6E3 | 1.9E3 | 2.1E2 | 3.8E2 | 1.0E4 | 8.7E3 | 40 | 34 | 40 | 34 | 0.56 |
| kO | pg/ml | 7.0E3 | 7.4E3 | 8.1E3 | 1.1E4 | 3.9E3 | 2.4E4 | 4.0E3 | 3.8E3 | 2.5E4 | 1.5E5 | 40 | 34 | 40 | 34 | 0.50 |
| kP | pg/ml | 6.6E3 | 5.3E3 | 7.4E3 | 6.1E3 | 4.7E3 | 3.6E3 | 1.5E3 | 9.6E2 | 2.7E4 | 1.5E4 | 40 | 34 | 40 | 34 | 0.41 |
| kQ | pg/ml | 4.1E3 | 4.6E3 | 5.1E3 | 5.7E3 | 3.8E3 | 4.3E3 | 5.6E2 | 1.1E3 | 2.5E4 | 2.5E4 | 64 | 55 | 64 | 55 | 0.56 |
| kR | pg/ml | 2.0E1 | 2.7E1 | 4.1E1 | 3.1E1 | 1.3E2 | 2.3E1 | 1.0E-9 | 2.9E0 | 1.0E3 | 1.1E2 | 64 | 55 | 64 | 55 | 0.57 |
| kS | pg/ml | 8.5E2 | 9.2E2 | 1.0E3 | 1.0E3 | 5.7E2 | 6.1E2 | 2.5E2 | 8.2E1 | 3.2E3 | 3.0E3 | 64 | 55 | 64 | 55 | 0.51 |
| pS | ng/ml | 1.6E5 | 1.4E5 | 1.9E5 | 1.6E5 | 1.1E5 | 8.2E4 | 8.5E4 | 6.8E4 | 5.7E5 | 3.6E5 | 32 | 21 | 32 | 21 | 0.43 |
| rZ | ng/ml | 1.0E-9 | 7.1E-3 | 1.0E-2 | 1.4E-2 | 4.3E-2 | 2.3E-2 | 1.0E-9 | 1.0E-9 | 3.0E-1 | 1.1E-1 | 53 | 34 | 53 | 34 | 0.67 |
| rY | ng/ml | 5.8E-2 | 7.0E-2 | 5.5E-1 | 6.7E-1 | 2.6E0 | 3.4E0 | 1.0E-9 | 1.0E-9 | 1.8E1 | 2.0E1 | 53 | 34 | 53 | 34 | 0.58 |
| rX | ng/ml | 1.0E-9 | 1.0E-9 | 1.4E-1 | 1.0E-1 | 6.1E-1 | 5.3E-1 | 1.0E-9 | 1.0E-9 | 3.7E0 | 3.1E0 | 53 | 34 | 53 | 34 | 0.52 |
| lK | pg/ml | 5.8E1 | 7.6E1 | 1.4E2 | 1.4E2 | 1.8E2 | 1.7E2 | 1.0E-9 | 1.0E-9 | 7.4E2 | 6.9E2 | 59 | 36 | 59 | 36 | 0.46 |
| lL | pg/ml | 1.7E3 | 1.7E3 | 2.5E3 | 3.6E3 | 2.8E3 | 6.9E3 | 7.5E1 | 1.2E2 | 1.9E4 | 4.2E4 | 60 | 36 | 60 | 36 | 0.53 |
| lM | pg/ml | 1.4E3 | 9.9E2 | 3.9E3 | 6.1E3 | 6.7E3 | 1.3E4 | 2.7E2 | 9.5E0 | 4.2E4 | 6.7E4 | 60 | 36 | 60 | 36 | 0.43 |
| lN | pg/ml | 1.0E-9 | 1.0E-9 | 2.8E0 | 4.5E0 | 6.7E0 | 6.9E0 | 1.0E-9 | 1.0E-9 | 4.3E1 | 2.4E1 | 60 | 36 | 60 | 36 | 0.58 |
| lO | pg/ml | 1.0E-9 | 1.0E-9 | 5.7E-1 | 7.3E0 | 4.4E0 | 3.1E1 | 1.0E-9 | 1.0E-9 | 3.4E1 | 1.4E2 | 59 | 36 | 59 | 36 | 0.52 |
| zA | ng/ml | 2.1E7 | 2.1E7 | 2.1E7 | 2.1E7 | 6.4E6 | 6.6E6 | 9.1E6 | 1.0E7 | 3.4E7 | 3.6E7 | 32 | 24 | 32 | 24 | 0.51 |
| rW | ng/ml | 1.2E-2 | 1.2E-2 | 2.8E-2 | 4.0E-2 | 3.7E-2 | 8.1E-2 | 1.0E-9 | 1.0E-9 | 1.7E-1 | 3.2E-1 | 33 | 19 | 33 | 19 | 0.47 |
| rV | ng/ml | 1.0E-9 | 1.0E-9 | 1.3E-2 | 1.5E-2 | 5.2E-2 | 3.6E-2 | 1.0E-9 | 1.0E-9 | 2.8E-1 | 1.5E-1 | 33 | 19 | 33 | 19 | 0.60 |
| rU | ng/ml | 7.1E-2 | 9.5E-2 | 2.0E-1 | 1.1E-1 | 4.8E-1 | 1.2E-1 | 1.0E-9 | 1.0E-9 | 2.7E0 | 4.0E-1 | 33 | 19 | 33 | 19 | 0.49 |
| rT | ng/ml | 6.7E0 | 3.7E0 | 6.9E0 | 7.0E0 | 4.0E0 | 5.8E0 | 6.5E-1 | 1.0E0 | 2.1E1 | 2.0E1 | 33 | 19 | 33 | 19 | 0.43 |
| rS | ng/ml | 3.7E0 | 5.2E0 | 5.7E0 | 1.4E1 | 5.5E0 | 2.0E1 | 7.6E-1 | 1.8E0 | 2.5E1 | 7.0E1 | 33 | 19 | 33 | 19 | 0.60 |
| sC | pg/mL | 5.6E3 | 5.9E3 | 1.0E4 | 1.2E4 | 1.1E4 | 1.7E4 | 2.3E3 | 1.7E3 | 5.1E4 | 7.4E4 | 32 | 21 | 32 | 21 | 0.49 |
| yL | pg/ml | 3.0E1 | 3.2E1 | 3.3E1 | 1.0E2 | 2.9E1 | 3.0E2 | 5.6E0 | 9.1E0 | 1.8E2 | 1.4E3 | 33 | 22 | 33 | 22 | 0.60 |
| rP | ng/ml | 1.2E2 | 2.0E2 | 1.9E2 | 2.6E2 | 1.8E2 | 2.4E2 | 1.0E-9 | 1.2E1 | 5.0E2 | 8.0E2 | 33 | 19 | 33 | 19 | 0.56 |
| rQ | ng/ml | 1.0E-9 | 1.0E-9 | 1.0E-9 | 1.7E1 | 0.0E0 | 4.6E1 | 1.0E-9 | 1.0E-9 | 1.0E-9 | 1.7E2 | 33 | 19 | 33 | 19 | 0.58 |
| rO | ng/ml | 1.5E-2 | 2.5E-2 | 3.9E-2 | 4.0E-2 | 7.4E-2 | 5.0E-2 | 1.0E-9 | 1.0E-9 | 4.0E-1 | 1.9E-1 | 33 | 19 | 33 | 19 | 0.54 |
| rR | ng/ml | 9.5E-1 | 4.0E0 | 9.1E0 | 1.4E1 | 2.1E1 | 2.0E1 | 1.0E-9 | 1.0E-9 | 9.5E1 | 7.5E1 | 33 | 19 | 33 | 19 | 0.58 |
| rN | ng/ml | 7.6E-1 | 6.3E-1 | 9.0E-1 | 1.5E0 | 5.8E-1 | 2.9E0 | 5.1E-2 | 2.1E-1 | 2.3E0 | 1.3E1 | 33 | 19 | 33 | 19 | 0.46 |
| qO | pg/ml | 8.7E3 | 8.2E3 | 1.1E4 | 1.4E4 | 8.8E3 | 1.3E4 | 7.4E2 | 1.9E3 | 3.7E4 | 4.8E4 | 32 | 21 | 32 | 21 | 0.52 |
| qP | pg/ml | 3.6E2 | 3.2E2 | 3.9E2 | 4.4E2 | 2.3E2 | 3.4E2 | 7.0E1 | 1.2E2 | 1.0E3 | 1.5E3 | 32 | 21 | 32 | 21 | 0.50 |
| qQ | pg/ml | 1.5E1 | 1.5E1 | 2.0E1 | 2.8E1 | 4.9E1 | 5.6E1 | 1.0E-9 | 1.0E-9 | 2.8E2 | 2.6E2 | 32 | 21 | 32 | 21 | 0.57 |
| nW | pg/ml | 1.1E5 | 1.1E5 | 1.1E5 | 1.1E5 | 2.9E4 | 2.8E4 | 5.8E4 | 3.6E4 | 1.9E5 | 1.7E5 | 64 | 55 | 64 | 55 | 0.50 |
| nY | pg/ml | 2.1E3 | 2.8E3 | 2.5E3 | 2.9E3 | 1.5E3 | 1.6E3 | 5.1E2 | 6.3E2 | 1.0E4 | 8.1E3 | 64 | 55 | 64 | 55 | 0.60 |
| oO | pg/ml | 9.0E4 | 7.6E4 | 1.0E5 | 1.0E5 | 5.8E4 | 8.2E4 | 1.5E4 | 3.3E3 | 2.6E5 | 4.0E5 | 34 | 33 | 34 | 33 | 0.43 |
| oP | pg/ml | 1.3E5 | 1.3E5 | 1.4E5 | 1.5E5 | 7.1E4 | 1.2E5 | 2.4E4 | 2.4E4 | 3.1E5 | 5.7E5 | 34 | 33 | 34 | 33 | 0.47 |
| oQ | pg/ml | 3.1E3 | 2.9E3 | 3.3E3 | 4.7E3 | 2.1E3 | 6.1E3 | 9.3E2 | 7.7E2 | 1.1E4 | 3.2E4 | 34 | 33 | 34 | 33 | 0.52 |
| oE | pg/ml | 1.4E2 | 3.3E2 | 3.6E2 | 6.9E2 | 5.1E2 | 7.8E2 | 1.0E-9 | 1.0E-9 | 2.8E3 | 3.4E3 | 64 | 55 | 64 | 55 | 0.65 |
| oF | pg/ml | 1.0E4 | 1.9E4 | 2.3E4 | 3.5E4 | 3.4E4 | 4.6E4 | 4.3E2 | 3.5E2 | 1.7E5 | 2.5E5 | 64 | 55 | 64 | 55 | 0.60 |
| oH | pg/ml | 4.0E1 | 3.4E1 | 8.3E1 | 7.1E1 | 1.3E2 | 9.8E1 | 4.3E-1 | 4.4E0 | 8.6E2 | 4.8E2 | 64 | 55 | 64 | 55 | 0.45 |
| oK | pg/ml | 8.2E2 | 8.8E2 | 1.8E3 | 1.4E3 | 2.3E3 | 1.3E3 | 8.8E1 | 1.4E2 | 1.2E4 | 5.9E3 | 64 | 55 | 64 | 55 | 0.50 |
| oN | pg/ml | 5.4E2 | 5.5E2 | 1.1E3 | 7.3E2 | 2.5E3 | 7.3E2 | 1.6E2 | 1.1E2 | 1.8E4 | 5.3E3 | 64 | 55 | 64 | 55 | 0.53 |
| oW | pg/ml | 2.0E2 | 3.4E2 | 2.7E2 | 8.7E2 | 2.0E2 | 1.7E3 | 7.7E1 | 2.9E1 | 7.3E2 | 7.6E3 | 13 | 20 | 13 | 20 | 0.60 |
| oT | pg/ml | 3.1E2 | 2.7E2 | 3.4E2 | 3.2E2 | 1.6E2 | 1.9E2 | 1.0E2 | 1.1E2 | 7.4E2 | 7.9E2 | 13 | 20 | 13 | 20 | 0.41 |
| oV | pg/ml | 1.5E2 | 8.9E1 | 3.1E2 | 2.5E2 | 3.9E2 | 5.0E2 | 2.1E1 | 1.0E-9 | 1.4E3 | 2.2E3 | 13 | 20 | 13 | 20 | 0.41 |
| oD | pg/ml | 1.7E4 | 1.4E4 | 1.6E4 | 1.5E4 | 5.2E3 | 6.5E3 | 8.7E3 | 6.6E3 | 2.5E4 | 3.2E4 | 13 | 20 | 13 | 20 | 0.40 |
| uL | ng/ml | 3.8E1 | 3.5E1 | 5.4E1 | 4.2E1 | 5.2E1 | 2.3E1 | 1.5E1 | 1.4E1 | 2.9E2 | 1.1E2 | 31 | 21 | 31 | 21 | 0.45 |
| uO | ng/ml | 4.8E-1 | 2.4E-1 | 9.6E-1 | 6.4E-1 | 1.7E0 | 7.6E-1 | 1.0E-9 | 1.0E-9 | 9.3E0 | 2.1E0 | 31 | 21 | 31 | 21 | 0.45 |
| uM | ng/ml | 6.4E-1 | 4.3E-1 | 1.2E0 | 5.0E-1 | 2.3E0 | 5.5E-1 | 1.0E-9 | 1.0E-9 | 1.3E1 | 2.5E0 | 31 | 21 | 31 | 21 | 0.34 |
| uI | ng/ml | 8.5E-2 | 5.4E-2 | 1.3E-1 | 8.4E-2 | 1.2E-1 | 9.2E-2 | 1.6E-2 | 1.5E-2 | 5.8E-1 | 4.3E-1 | 31 | 20 | 31 | 20 | 0.35 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| uN | ng/ml | 1.4E1 | 1.7E1 | 1.5E1 | 2.0E1 | 5.3E0 | 8.4E0 | 8.0E0 | 1.0E1 | 3.0E1 | 4.1E1 | 31 | 21 | 31 | 21 | 0.66 |
| uG | ng/ml | 1.8E1 | 1.9E1 | 2.2E1 | 2.7E1 | 1.5E1 | 2.8E1 | 6.1E0 | 1.2E0 | 7.9E1 | 1.3E2 | 31 | 21 | 31 | 21 | 0.54 |
| uR | ng/ml | 2.3E0 | 2.0E0 | 2.6E0 | 3.1E0 | 2.2E0 | 2.3E0 | 8.9E-1 | 7.5E-1 | 1.3E1 | 8.3E0 | 33 | 22 | 33 | 22 | 0.53 |
| uP | ng/ml | 2.1E0 | 2.6E0 | 2.4E0 | 2.8E0 | 9.8E-1 | 1.3E0 | 1.2E0 | 9.3E-1 | 6.0E0 | 6.1E0 | 33 | 22 | 33 | 22 | 0.61 |
| uV | ng/ml | 2.3E-4 | 3.2E-3 | 1.7E-2 | 9.4E-3 | 3.9E-2 | 1.3E-2 | 1.0E-9 | 1.0E-9 | 2.0E-1 | 4.3E-2 | 33 | 22 | 33 | 22 | 0.50 |
| uT | ng/ml | 6.3E1 | 8.7E1 | 9.0E1 | 1.1E2 | 9.3E1 | 8.8E1 | 1.3E1 | 2.2E1 | 4.5E2 | 4.1E2 | 33 | 22 | 33 | 22 | 0.66 |
| uU | ng/ml | 1.8E0 | 1.9E0 | 1.9E0 | 2.7E0 | 1.3E0 | 3.9E0 | 5.2E-1 | 5.4E-1 | 6.0E0 | 2.0E1 | 33 | 22 | 33 | 22 | 0.56 |
| uW | ng/ml | 7.3E0 | 8.1E0 | 7.6E0 | 8.5E0 | 2.0E0 | 2.5E0 | 4.4E0 | 5.5E0 | 1.3E1 | 1.6E1 | 31 | 21 | 31 | 21 | 0.62 |
| vB | ng/ml | 2.8E0 | 3.3E0 | 3.2E0 | 3.6E0 | 1.7E0 | 2.7E0 | 1.1E0 | 5.9E-1 | 8.3E0 | 1.0E1 | 31 | 21 | 31 | 21 | 0.53 |
| vC | ng/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 31 | 21 | 31 | 21 | 0.50 |
| uY | ng/ml | 6.1E-1 | 7.5E-1 | 7.9E-1 | 1.3E0 | 5.6E-1 | 1.3E0 | 6.8E-2 | 3.1E-1 | 2.3E0 | 4.4E0 | 31 | 21 | 31 | 21 | 0.59 |
| uZ | ng/ml | 5.8E-1 | 5.3E-1 | 8.1E-1 | 6.2E-1 | 9.4E-1 | 3.9E-1 | 1.0E-1 | 1.7E-1 | 4.9E0 | 1.9E0 | 31 | 21 | 31 | 21 | 0.47 |
| uX | ng/ml | 9.4E0 | 8.3E0 | 1.2E1 | 1.5E1 | 7.3E0 | 1.7E1 | 4.5E0 | 3.6E0 | 4.0E1 | 6.5E1 | 31 | 21 | 31 | 21 | 0.49 |
| vA | ng/ml | 7.0E-2 | 6.3E-2 | 8.3E-2 | 8.0E-2 | 5.9E-2 | 8.1E-2 | 2.4E-2 | 2.5E-2 | 2.7E-1 | 4.2E-1 | 31 | 21 | 31 | 21 | 0.46 |
| vH | ng/ml | 1.2E-1 | 1.2E-1 | 1.4E-1 | 2.4E-1 | 1.2E-1 | 4.1E-1 | 2.0E-2 | 2.1E-2 | 6.6E-1 | 1.9E0 | 32 | 21 | 32 | 21 | 0.52 |
| vI | ng/ml | 1.8E0 | 2.5E0 | 2.0E0 | 3.2E0 | 1.5E0 | 2.7E0 | 6.3E-3 | 8.7E-2 | 6.4E0 | 1.0E1 | 32 | 21 | 32 | 21 | 0.65 |
| vP | ng/ml | 4.3E2 | 2.9E2 | 4.6E2 | 4.8E2 | 3.2E2 | 5.3E2 | 6.7E1 | 9.2E1 | 1.1E3 | 2.4E3 | 33 | 22 | 33 | 22 | 0.46 |
| vT | ng/ml | 7.4E1 | 7.1E1 | 9.0E1 | 8.3E1 | 4.7E1 | 3.5E1 | 4.1E1 | 4.6E1 | 2.4E2 | 1.6E2 | 33 | 22 | 33 | 22 | 0.46 |
| vU | ng/ml | 1.0E-9 | 1.0E-9 | 3.1E1 | 1.9E1 | 4.2E1 | 3.4E1 | 1.0E-9 | 1.0E-9 | 1.3E2 | 1.1E2 | 33 | 22 | 33 | 22 | 0.41 |
| vQ | ng/ml | 3.9E2 | 4.4E2 | 4.2E2 | 4.1E2 | 1.7E2 | 1.6E2 | 7.2E1 | 1.2E2 | 8.4E2 | 6.7E2 | 33 | 22 | 33 | 22 | 0.50 |
| vO | ng/ml | 1.8E3 | 1.6E3 | 1.8E3 | 1.7E3 | 4.2E2 | 5.2E2 | 1.1E3 | 1.0E3 | 2.8E3 | 3.2E3 | 33 | 22 | 33 | 22 | 0.40 |
| vS | ng/ml | 1.3E3 | 1.3E3 | 1.2E3 | 1.3E3 | 4.1E2 | 5.2E2 | 1.0E-9 | 1.0E-9 | 2.1E3 | 2.0E3 | 33 | 22 | 33 | 22 | 0.52 |
| vV | ng/ml | 9.3E2 | 7.8E2 | 9.9E2 | 1.1E3 | 7.3E2 | 1.2E3 | 1.1E2 | 1.0E2 | 2.9E3 | 4.6E3 | 33 | 22 | 33 | 22 | 0.47 |
| vW | ng/ml | 1.2E2 | 1.1E2 | 1.6E2 | 1.8E2 | 1.2E2 | 1.7E2 | 4.3E1 | 6.0E1 | 6.6E2 | 7.7E2 | 33 | 22 | 33 | 22 | 0.49 |
| pF | pg/ml | 7.2E-1 | 5.2E-1 | 1.0E0 | 2.2E0 | 1.3E0 | 1.2E1 | 1.0E-9 | 1.0E-9 | 9.4E0 | 8.7E1 | 64 | 55 | 64 | 55 | 0.41 |
| pH | ng/ml | 6.9E0 | 8.9E0 | 8.6E0 | 1.0E1 | 4.2E0 | 5.7E0 | 3.9E0 | 1.2E0 | 1.8E1 | 2.3E1 | 13 | 20 | 13 | 20 | 0.60 |
| pI | ng/ml | 7.0E1 | 6.8E1 | 7.1E1 | 7.5E1 | 3.3E1 | 4.9E1 | 2.6E1 | 2.3E1 | 1.5E2 | 2.0E2 | 13 | 20 | 13 | 20 | 0.48 |
| pK | ng/ml | 3.9E-1 | 4.9E-1 | 3.9E-1 | 5.0E-1 | 1.7E-1 | 2.0E-1 | 2.0E-1 | 1.7E-1 | 7.7E-1 | 8.6E-1 | 13 | 20 | 13 | 20 | 0.68 |

Figure 40.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Aa | mg/dL | 8.3E1 | 6.3E1 | 9.4E1 | 8.5E1 | 6.6E1 | 7.9E1 | 8.0E0 | 7.0E0 | 4.8E2 | 4.0E2 | 268 | 27 | 268 | 27 | 0.43 |
| Ad | ug/mL | 4.8E-2 | 5.0E-2 | 1.3E-1 | 6.6E-2 | 6.9E-1 | 7.9E-2 | 6.8E-4 | 7.8E-4 | 8.5E0 | 3.5E-1 | 150 | 22 | 150 | 22 | 0.46 |
| Af | ng/mL | 1.3E0 | 1.0E0 | 1.1E1 | 5.5E0 | 4.7E1 | 7.2E0 | 1.7E-3 | 1.7E-3 | 5.3E2 | 2.1E1 | 150 | 22 | 150 | 22 | 0.50 |
| Aj | ug/mL | 1.2E0 | 5.0E-1 | 2.4E0 | 1.8E0 | 2.5E0 | 2.3E0 | 1.5E-3 | 1.3E-3 | 6.1E0 | 5.8E0 | 150 | 22 | 150 | 22 | 0.43 |
| Al | mg/mL | 9.0E-5 | 6.4E-5 | 2.6E-4 | 3.5E-4 | 4.4E-4 | 4.9E-4 | 4.3E-6 | 6.6E-6 | 1.8E-3 | 1.5E-3 | 150 | 22 | 150 | 22 | 0.47 |
| An | U/mL | 6.0E1 | 7.2E1 | 2.5E2 | 2.2E2 | 8.2E2 | 4.4E2 | 2.8E-1 | 1.1E0 | 7.8E3 | 2.0E3 | 150 | 22 | 150 | 22 | 0.54 |
| Ao | pg/mL | 9.1E1 | 1.3E2 | 5.6E2 | 4.5E2 | 3.8E3 | 9.8E2 | 1.5E0 | 4.1E0 | 3.9E4 | 4.5E3 | 150 | 22 | 150 | 22 | 0.55 |
| Ap | ng/mL | 3.3E1 | 2.8E1 | 4.9E1 | 5.9E1 | 5.4E1 | 7.3E1 | 2.0E0 | 2.7E0 | 3.3E2 | 2.4E2 | 150 | 22 | 150 | 22 | 0.48 |
| Ar | ng/mL | 6.9E-1 | 7.7E-1 | 2.5E0 | 6.9E0 | 5.7E0 | 1.5E1 | 3.4E-3 | 4.1E-2 | 4.7E1 | 5.1E1 | 150 | 22 | 150 | 22 | 0.54 |
| As | ng/mL | 8.7E-3 | 3.7E-3 | 2.1E-2 | 8.8E-3 | 1.0E-1 | 8.9E-3 | 1.7E-3 | 1.7E-3 | 1.2E0 | 3.3E-2 | 150 | 22 | 150 | 22 | 0.45 |
| Aw | pg/mL | 1.6E1 | 2.0E1 | 1.7E1 | 1.9E1 | 6.3E0 | 5.3E0 | 2.9E-2 | 8.2E0 | 5.1E1 | 2.9E1 | 150 | 22 | 150 | 22 | 0.65 |
| Ax | ng/mL | 2.0E0 | 2.0E0 | 2.3E1 | 7.6E1 | 9.0E1 | 2.0E2 | 1.2E-2 | 1.0E-4 | 7.7E2 | 8.5E2 | 150 | 22 | 150 | 22 | 0.48 |
| Ba | ng/mL | 8.6E1 | 2.1E2 | 5.8E2 | 1.6E3 | 1.5E3 | 3.3E3 | 1.9E0 | 1.1E0 | 8.1E3 | 1.5E4 | 150 | 22 | 150 | 22 | 0.59 |
| Bb | ng/mL | 4.3E0 | 4.0E0 | 7.1E0 | 9.2E0 | 8.3E0 | 1.3E1 | 4.1E-3 | 4.1E-3 | 4.9E1 | 4.8E1 | 150 | 22 | 150 | 22 | 0.47 |
| Bc | ng/mL | 3.6E1 | 5.3E1 | 1.3E2 | 1.6E2 | 2.4E2 | 2.6E2 | 4.9E-1 | 1.0E0 | 1.2E3 | 9.9E2 | 150 | 22 | 150 | 22 | 0.52 |
| Bg | ng/mL | 1.1E-1 | 1.7E-1 | 4.4E0 | 1.9E1 | 1.8E1 | 8.4E1 | 5.3E-4 | 5.3E-4 | 1.5E2 | 4.0E2 | 150 | 22 | 150 | 22 | 0.53 |
| Bn | ng/mL | 5.6E-2 | 3.6E-1 | 1.5E0 | 1.3E0 | 5.1E0 | 2.0E0 | 5.6E-2 | 5.6E-2 | 5.8E1 | 7.4E0 | 150 | 22 | 150 | 22 | 0.60 |
| Bo | ng/mL | 1.3E1 | 2.0E1 | 1.4E1 | 1.9E1 | 1.1E1 | 1.1E1 | 1.6E-2 | 2.2E0 | 5.0E1 | 4.4E1 | 150 | 22 | 150 | 22 | 0.64 |
| Ch | uIU/mL | 1.0E0 | 1.1E0 | 3.1E1 | 6.0E1 | 1.7E2 | 2.5E2 | 3.4E-3 | 1.5E-1 | 1.8E3 | 1.2E3 | 150 | 22 | 150 | 22 | 0.49 |
| Co | pg/mL | 4.8E1 | 4.6E1 | 2.4E2 | 1.9E2 | 1.4E3 | 4.4E2 | 1.5E-1 | 3.6E0 | 1.7E4 | 2.1E3 | 150 | 22 | 150 | 22 | 0.48 |
| Cp | ng/mL | 2.2E1 | 2.2E1 | 3.5E1 | 3.5E1 | 1.0E2 | 3.0E1 | 6.0E-1 | 4.7E0 | 1.3E3 | 1.4E2 | 150 | 22 | 150 | 22 | 0.56 |
| Cq | ng/mL | 2.8E-2 | 4.2E-2 | 4.4E-1 | 2.1E-1 | 4.0E0 | 5.1E-1 | 8.0E-4 | 8.0E-4 | 4.9E1 | 2.3E0 | 150 | 22 | 150 | 22 | 0.60 |
| Cs | ng/mL | 6.0E1 | 1.0E2 | 4.7E2 | 6.6E2 | 1.7E3 | 1.4E3 | 8.3E-1 | 8.9E-1 | 1.8E4 | 5.1E3 | 150 | 22 | 150 | 22 | 0.54 |
| Ct | ng/mL | 3.4E-1 | 4.7E-1 | 4.3E1 | 3.7E1 | 1.3E2 | 9.6E1 | 8.9E-3 | 1.1E-4 | 6.2E2 | 4.2E2 | 150 | 22 | 150 | 22 | 0.53 |
| Cu | ng/mL | 2.5E-1 | 7.5E-1 | 9.0E-1 | 2.0E0 | 5.4E0 | 4.4E0 | 1.9E-2 | 1.7E-2 | 6.6E1 | 2.1E1 | 150 | 22 | 150 | 22 | 0.65 |
| Cv | ng/mL | 5.9E0 | 5.1E0 | 2.8E1 | 2.1E1 | 6.7E1 | 3.7E1 | 2.0E-2 | 7.5E-2 | 5.3E2 | 1.4E2 | 150 | 22 | 150 | 22 | 0.46 |
| Cw | mIU/mL | 3.4E-2 | 4.7E-2 | 8.7E-2 | 5.4E-2 | 5.5E-1 | 4.1E-2 | 8.9E-4 | 2.8E-3 | 6.8E0 | 1.5E-1 | 150 | 22 | 150 | 22 | 0.58 |
| Cx | ng/mL | 8.2E-1 | 3.5E-1 | 5.0E1 | 4.0E1 | 9.8E1 | 9.1E1 | 9.3E-5 | 9.3E-5 | 3.9E2 | 2.8E2 | 150 | 22 | 150 | 22 | 0.44 |
| Db | ug/mL | 7.5E0 | 7.1E0 | 8.8E0 | 8.4E0 | 7.9E0 | 5.2E0 | 4.5E-1 | 8.1E-1 | 5.9E1 | 2.0E1 | 150 | 22 | 150 | 22 | 0.51 |
| Dc | nmol/L | 2.1E-2 | 2.3E-2 | 1.4E-1 | 2.1E-1 | 1.1E0 | 4.3E-1 | 5.2E-6 | 1.1E-3 | 1.4E1 | 1.6E0 | 150 | 22 | 150 | 22 | 0.57 |
| Dd | ug/mL | 7.8E-2 | 4.6E-2 | 2.0E-1 | 2.5E-1 | 3.7E-1 | 3.8E-1 | 4.8E-4 | 6.2E-3 | 3.6E0 | 1.5E0 | 150 | 22 | 150 | 22 | 0.53 |
| De | ng/mL | 3.4E-3 | 1.5E-1 | 7.1E-2 | 1.8E-1 | 1.4E-1 | 2.5E-1 | 3.4E-3 | 3.4E-3 | 9.2E-1 | 1.1E0 | 150 | 22 | 150 | 22 | 0.67 |
| Dg | ng/mL | 3.7E1 | 3.4E1 | 4.8E1 | 4.3E1 | 4.0E1 | 3.9E1 | 7.1E-1 | 7.8E-1 | 1.9E2 | 1.2E2 | 150 | 22 | 150 | 22 | 0.46 |
| Di | pg/mL | 2.0E0 | 2.9E0 | 2.4E0 | 3.1E0 | 2.2E0 | 2.0E0 | 1.8E-1 | 1.8E-1 | 1.3E1 | 9.3E0 | 150 | 22 | 150 | 22 | 0.62 |
| Dk | uIU/mL | 1.4E-2 | 1.5E-2 | 5.4E-2 | 9.1E-2 | 1.7E-1 | 2.1E-1 | 1.1E-4 | 1.1E-4 | 1.6E0 | 9.8E-1 | 150 | 22 | 150 | 22 | 0.55 |
| Dl | ng/mL | 2.2E2 | 1.8E2 | 3.0E2 | 2.7E2 | 2.8E2 | 2.8E2 | 5.5E0 | 4.4E0 | 1.6E3 | 1.1E3 | 150 | 22 | 150 | 22 | 0.46 |
| Dp | ng/ml | 2.4E0 | 1.2E0 | 5.8E0 | 3.3E0 | 9.3E0 | 5.4E0 | 3.7E-3 | 3.7E-3 | 5.6E1 | 1.8E1 | 114 | 19 | 114 | 19 | 0.38 |
| Dr | pg/ml | 2.2E1 | 3.0E1 | 2.3E2 | 7.0E1 | 1.4E3 | 1.2E2 | 7.5E-1 | 7.5E-1 | 1.0E4 | 3.6E2 | 59 | 13 | 59 | 13 | 0.50 |
| Du | pg/ml | 1.6E2 | 1.4E2 | 1.5E3 | 5.1E2 | 4.5E3 | 6.6E2 | 1.2E0 | 1.2E0 | 2.4E4 | 1.8E3 | 45 | 12 | 45 | 12 | 0.46 |
| Ef | ng/ml | 9.5E-2 | 1.2E-1 | 7.9E-1 | 1.6E0 | 1.8E0 | 3.3E0 | 5.7E-4 | 5.7E-4 | 1.0E1 | 9.9E0 | 127 | 18 | 127 | 18 | 0.51 |
| Wm | % | 8.5E-2 | 8.5E-2 | 9.6E0 | 5.2E1 | 6.9E1 | 2.2E2 | 5.4E-2 | 8.5E-2 | 7.7E2 | 1.0E3 | 133 | 22 | 133 | 22 | 0.43 |
| Ed | pg/ml | 2.4E0 | 3.9E1 | 3.1E1 | 7.8E1 | 5.9E1 | 1.1E2 | 5.2E-1 | 5.2E-1 | 5.0E2 | 4.8E2 | 114 | 19 | 114 | 19 | 0.65 |
| Yf | ng/mL | 1.5E1 | 1.7E1 | 3.7E1 | 1.1E2 | 5.4E1 | 1.9E2 | 2.9E-1 | 2.9E-1 | 2.4E2 | 5.9E2 | 47 | 12 | 47 | 12 | 0.54 |
| Tj | pg/mL | 3.7E-1 | 3.7E-1 | 4.0E1 | 3.0E1 | 2.2E2 | 7.3E1 | 3.7E-1 | 3.7E-1 | 2.3E3 | 2.5E2 | 124 | 19 | 124 | 19 | 0.49 |
| Po | pg/ml | 2.1E-1 | 8.4E0 | 9.2E0 | 2.3E1 | 2.9E1 | 4.4E1 | 8.0E-3 | 2.6E-2 | 2.7E2 | 2.2E2 | 317 | 31 | 317 | 31 | 0.68 |
| Ti | ug/mL | 3.0E0 | 5.2E0 | 4.9E0 | 5.2E0 | 4.4E0 | 3.9E0 | 1.2E-1 | 4.0E-1 | 1.8E1 | 1.1E1 | 68 | 15 | 68 | 15 | 0.53 |
| Em | ng/ml | 6.1E-3 | 2.6E-2 | 7.6E-2 | 5.0E-2 | 2.4E-1 | 7.1E-2 | 8.4E-4 | 8.4E-4 | 1.9E0 | 2.4E-1 | 76 | 16 | 76 | 16 | 0.53 |
| Et | ng/ml | 1.4E3 | 2.7E3 | 1.6E3 | 2.3E3 | 1.2E3 | 1.4E3 | 7.5E1 | 7.9E1 | 4.8E3 | 5.0E3 | 316 | 31 | 316 | 31 | 0.64 |
| Eq | pg/ml | 1.6E2 | 1.9E2 | 3.3E2 | 4.1E2 | 3.8E2 | 4.8E2 | 1.0E0 | 1.0E0 | 1.5E3 | 1.4E3 | 45 | 12 | 45 | 12 | 0.53 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|-----|--------|-----|-----|
| | | NonDis | Dis | NonDis | Dis | NonDis | Dis | NonDis | Dis | NonDis | Dis | NonDis | Dis | NonDis | Dis | |
| Th | ug/mL | 1.2E0 | 1.5E0 | 1.7E0 | 2.2E0 | 1.5E0 | 1.8E0 | 2.6E-3 | 2.6E-1 | 7.5E0 | 5.9E0 | 68 | 15 | 68 | 15 | 0.55 |
| Fa | ng/ml | 4.3E1 | 4.2E1 | 1.3E2 | 8.5E1 | 4.2E2 | 1.2E2 | 2.6E-1 | 1.6E0 | 3.7E3 | 4.3E2 | 113 | 16 | 113 | 16 | 0.48 |
| Ez | ng/ml | 3.8E0 | 3.2E0 | 1.4E1 | 2.4E1 | 2.7E1 | 4.9E1 | 1.3E-2 | 1.3E-2 | 1.6E2 | 2.0E2 | 114 | 19 | 114 | 19 | 0.49 |
| Fb | ng/ml | 2.5E1 | 2.6E1 | 2.3E1 | 2.3E1 | 1.1E1 | 1.3E1 | 6.6E-1 | 8.9E-1 | 4.3E1 | 4.3E1 | 114 | 16 | 114 | 16 | 0.48 |
| Ex | ng/ml | 7.4E-2 | 1.4E-1 | 1.8E-1 | 4.8E-1 | 3.1E-1 | 1.1E0 | 3.5E-5 | 1.7E-4 | 2.2E0 | 4.1E0 | 89 | 15 | 89 | 15 | 0.60 |
| Fc | pg/ml | 2.2E-1 | 2.2E-1 | 2.0E1 | 3.8E1 | 7.9E1 | 1.1E2 | 2.2E-1 | 2.2E-1 | 4.5E2 | 3.9E2 | 46 | 12 | 46 | 12 | 0.56 |
| Fd | pg/ml | 8.2E1 | 7.9E1 | 9.5E2 | 2.8E3 | 3.7E3 | 6.6E3 | 4.5E-1 | 9.8E-1 | 2.5E4 | 2.2E4 | 46 | 12 | 46 | 12 | 0.51 |
| Fi | pg/ml | 2.5E-1 | 2.5E-1 | 5.6E1 | 2.3E2 | 1.7E2 | 5.3E2 | 2.5E-1 | 2.5E-1 | 8.5E2 | 1.8E3 | 46 | 12 | 46 | 12 | 0.61 |
| Fn | ng/ml | 2.1E-1 | 2.8E-1 | 4.2E0 | 2.5E0 | 7.5E0 | 4.0E0 | 1.1E-14 | 2.1E-1 | 3.7E1 | 1.6E1 | 114 | 19 | 114 | 19 | 0.54 |
| Fp | ng/ml | 1.3E1 | 1.5E1 | 2.3E1 | 3.2E1 | 2.6E1 | 3.8E1 | 6.0E-3 | 3.0E-1 | 1.3E2 | 1.3E2 | 319 | 31 | 319 | 31 | 0.55 |
| Fr | ng/ml | 3.4E4 | 7.0E4 | 1.2E5 | 2.5E5 | 1.8E5 | 3.0E5 | 1.9E2 | 1.3E3 | 8.4E5 | 8.4E5 | 324 | 33 | 324 | 33 | 0.63 |
| Fw | pg/ml | 2.0E0 | 9.4E0 | 4.9E1 | 3.9E1 | 2.8E2 | 6.7E1 | 1.2E-1 | 1.2E-1 | 3.0E3 | 2.5E2 | 128 | 19 | 128 | 19 | 0.65 |
| Fy | ng/ml | 3.5E1 | 4.3E1 | 6.3E1 | 1.2E2 | 8.8E1 | 1.6E2 | 1.2E-1 | 1.2E-1 | 6.5E2 | 5.3E2 | 113 | 18 | 113 | 18 | 0.57 |
| Gh | pg/ml | 1.3E0 | 6.0E0 | 2.4E1 | 1.1E1 | 6.0E1 | 1.3E1 | 2.9E-2 | 2.9E-2 | 3.2E2 | 4.6E1 | 46 | 12 | 46 | 12 | 0.62 |
| Gb | % | 4.5E1 | 2.6E1 | 5.6E1 | 4.1E1 | 5.5E1 | 4.7E1 | 3.7E0 | 2.2E0 | 3.0E2 | 1.5E2 | 46 | 12 | 46 | 12 | 0.36 |
| Gc | ng/ml | 9.9E1 | 1.2E2 | 1.7E2 | 1.6E2 | 2.2E2 | 1.6E2 | 6.4E0 | 2.7E1 | 1.2E3 | 5.1E2 | 59 | 13 | 59 | 13 | 0.53 |
| Gd | ng/ml | 3.4E1 | 2.8E1 | 3.5E1 | 2.6E1 | 1.9E1 | 1.5E1 | 3.7E0 | 3.0E0 | 8.1E1 | 5.6E1 | 68 | 14 | 68 | 14 | 0.38 |
| Gn | U/ml | 2.2E-1 | 1.3E-1 | 3.2E0 | 5.1E-1 | 1.5E1 | 7.0E-1 | 5.6E-3 | 5.6E-3 | 1.1E2 | 2.2E0 | 58 | 13 | 58 | 13 | 0.44 |
| Gl | pg/ml | 8.8E3 | 1.8E4 | 1.2E4 | 1.6E4 | 9.3E3 | 1.1E4 | 9.1E1 | 5.3E2 | 3.2E4 | 3.2E4 | 128 | 19 | 128 | 19 | 0.63 |
| Gp | U/ml | 1.2E0 | 1.5E-1 | 2.6E0 | 1.0E0 | 3.6E0 | 1.4E0 | 1.5E-2 | 1.5E-2 | 2.0E1 | 4.2E0 | 128 | 18 | 128 | 18 | 0.34 |
| Gz | ug/ml | 1.5E0 | 8.1E-1 | 5.9E0 | 3.1E0 | 5.9E0 | 4.4E0 | 4.2E-2 | 8.9E-2 | 2.5E1 | 1.1E1 | 78 | 15 | 78 | 15 | 0.36 |
| Ha | ng/ml | 2.0E0 | 2.6E0 | 8.6E0 | 8.8E0 | 2.0E1 | 2.1E1 | 1.7E-2 | 1.7E-2 | 1.0E2 | 9.2E1 | 112 | 19 | 112 | 19 | 0.53 |
| Nm | pg/ml | 1.3E4 | 1.4E4 | 3.5E4 | 5.5E4 | 9.2E4 | 1.1E5 | 1.0E-9 | 1.0E-9 | 9.6E5 | 4.4E5 | 320 | 31 | 320 | 31 | 0.52 |
| Nn | pg/ml | 1.5E2 | 5.9E2 | 1.1E3 | 1.9E4 | 4.4E3 | 5.8E4 | 1.0E-9 | 1.0E-9 | 6.0E4 | 3.1E5 | 320 | 31 | 320 | 31 | 0.63 |
| No | pg/ml | 1.5E1 | 2.3E1 | 3.5E1 | 8.0E1 | 7.8E1 | 1.6E2 | 1.0E-9 | 3.3E-1 | 9.1E2 | 7.0E2 | 320 | 31 | 320 | 31 | 0.59 |
| Nq | pg/ml | 1.9E0 | 4.6E0 | 1.8E1 | 7.3E1 | 6.6E1 | 1.7E2 | 1.0E-9 | 1.0E-9 | 6.3E2 | 6.7E2 | 320 | 31 | 320 | 31 | 0.58 |
| Nr | pg/ml | 1.4E0 | 2.8E0 | 2.3E1 | 7.6E1 | 8.5E1 | 2.5E2 | 1.0E-9 | 1.0E-9 | 9.8E2 | 1.4E3 | 320 | 31 | 320 | 31 | 0.59 |
| Ns | pg/ml | 1.0E-9 | 1.0E-9 | 4.7E0 | 3.8E1 | 2.5E1 | 2.0E2 | 1.0E-9 | 1.0E-9 | 2.4E2 | 1.1E3 | 320 | 31 | 320 | 31 | 0.53 |
| Nt | pg/ml | 1.0E2 | 1.6E2 | 1.3E2 | 2.5E2 | 1.3E2 | 2.6E2 | 9.8E-1 | 3.5E1 | 1.7E3 | 1.2E3 | 320 | 31 | 320 | 31 | 0.69 |
| Nu | pg/ml | 1.7E1 | 7.7E1 | 5.3E1 | 1.0E2 | 8.9E1 | 1.3E2 | 1.0E-9 | 1.0E-9 | 6.3E2 | 6.3E2 | 320 | 31 | 320 | 31 | 0.69 |
| Lu | pg/ml | 1.0E4 | 1.0E4 | 1.4E4 | 2.6E4 | 2.8E4 | 9.9E4 | 5.2E2 | 1.3E3 | 3.9E5 | 5.6E5 | 320 | 31 | 320 | 31 | 0.44 |
| Lv | pg/ml | 1.0E-9 | 1.8E1 | 1.4E1 | 4.2E1 | 2.7E1 | 5.9E1 | 1.0E-9 | 1.0E-9 | 2.4E2 | 2.6E2 | 320 | 31 | 320 | 31 | 0.68 |
| Lw | pg/ml | 1.0E-9 | 1.0E-9 | 5.1E-1 | 7.0E0 | 5.3E0 | 3.2E1 | 1.0E-9 | 1.0E-9 | 8.0E1 | 1.8E2 | 320 | 31 | 320 | 31 | 0.54 |
| Lx | pg/ml | 1.0E-9 | 1.3E2 | 2.4E2 | 5.2E2 | 1.4E3 | 7.4E2 | 1.0E-9 | 1.0E-9 | 2.2E4 | 2.8E3 | 320 | 31 | 320 | 31 | 0.68 |
| Ly | pg/ml | 1.0E-9 | 1.0E-9 | 9.3E0 | 6.9E0 | 1.8E1 | 1.3E1 | 1.0E-9 | 1.0E-9 | 1.1E2 | 4.8E1 | 320 | 31 | 320 | 31 | 0.48 |
| Lz | pg/ml | 1.0E-9 | 1.0E-9 | 3.2E0 | 6.7E0 | 2.7E1 | 1.8E1 | 1.0E-9 | 1.0E-9 | 4.3E2 | 6.2E1 | 320 | 31 | 320 | 31 | 0.56 |
| Ma | pg/ml | 4.0E2 | 4.1E2 | 2.0E3 | 6.5E3 | 5.2E3 | 1.3E4 | 1.0E-9 | 1.0E-9 | 6.5E4 | 5.2E4 | 320 | 31 | 320 | 31 | 0.53 |
| Mb | pg/ml | 2.5E1 | 3.7E1 | 3.1E1 | 4.1E1 | 1.7E1 | 2.2E1 | 4.1E0 | 1.6E1 | 2.1E2 | 1.1E2 | 320 | 31 | 320 | 31 | 0.63 |
| Mc | pg/ml | 1.0E-9 | 1.0E-9 | 5.4E-2 | 5.4E-2 | 7.6E-1 | 3.0E-1 | 1.0E-9 | 1.0E-9 | 1.3E1 | 1.7E0 | 320 | 31 | 320 | 31 | 0.51 |
| Md | pg/ml | 1.0E-9 | 1.0E-9 | 6.7E-1 | 5.9E-1 | 5.6E0 | 2.0E0 | 1.0E-9 | 1.0E-9 | 6.8E1 | 9.8E0 | 320 | 31 | 320 | 31 | 0.55 |
| Me | pg/ml | 3.2E1 | 2.3E1 | 3.1E1 | 2.4E1 | 2.3E1 | 2.9E1 | 1.0E-9 | 1.0E-9 | 3.2E2 | 1.6E2 | 320 | 31 | 320 | 31 | 0.33 |
| Mf | pg/ml | 1.0E-9 | 1.0E-9 | 5.0E-1 | 1.8E0 | 3.5E0 | 6.6E0 | 1.0E-9 | 1.0E-9 | 5.6E1 | 3.7E1 | 320 | 31 | 320 | 31 | 0.58 |
| Mg | pg/ml | 1.1E0 | 5.3E-1 | 6.3E0 | 1.3E1 | 1.2E1 | 3.1E1 | 1.0E-9 | 1.0E-9 | 9.2E1 | 1.5E2 | 320 | 31 | 320 | 31 | 0.51 |
| Mh | pg/ml | 1.0E-9 | 4.0E-2 | 1.1E0 | 1.9E0 | 7.8E0 | 4.3E0 | 1.0E-9 | 1.0E-9 | 1.1E2 | 1.8E1 | 320 | 31 | 320 | 31 | 0.63 |
| Mi | pg/ml | 1.0E-9 | 1.0E-9 | 2.0E0 | 2.4E1 | 2.0E1 | 9.5E1 | 1.0E-9 | 1.0E-9 | 3.2E2 | 5.2E2 | 320 | 31 | 320 | 31 | 0.60 |
| Mj | pg/ml | 1.0E-9 | 1.0E-9 | 5.9E0 | 2.2E1 | 3.3E1 | 4.4E1 | 1.0E-9 | 1.0E-9 | 4.6E2 | 2.1E2 | 320 | 31 | 320 | 31 | 0.65 |
| Mk | pg/ml | 7.2E-1 | 7.4E0 | 1.6E1 | 2.7E1 | 9.5E1 | 9.0E1 | 1.0E-9 | 1.0E-9 | 1.3E3 | 5.0E2 | 320 | 31 | 320 | 31 | 0.63 |
| Ml | pg/ml | 1.0E-9 | 1.0E-9 | 1.1E1 | 1.7E1 | 1.2E2 | 6.4E1 | 1.0E-9 | 1.0E-9 | 2.1E3 | 3.4E2 | 320 | 31 | 320 | 31 | 0.52 |
| Mm | pg/ml | 5.5E2 | 3.8E2 | 1.0E3 | 1.7E3 | 1.3E3 | 2.3E3 | 1.0E-9 | 1.0E-9 | 1.0E4 | 1.0E4 | 320 | 31 | 320 | 31 | 0.54 |
| Mn | pg/ml | 5.7E0 | 1.0E1 | 1.1E1 | 1.1E1 | 2.5E1 | 8.6E0 | 1.0E-9 | 1.0E-9 | 3.5E2 | 3.7E1 | 320 | 31 | 320 | 31 | 0.61 |
| Mp | pg/ml | 1.0E-9 | 7.4E0 | 1.3E1 | 1.1E2 | 5.1E1 | 4.2E2 | 1.0E-9 | 1.0E-9 | 6.3E2 | 2.4E3 | 320 | 31 | 320 | 31 | 0.62 |
| Mq | pg/ml | 1.0E-9 | 1.0E-9 | 2.5E0 | 6.5E0 | 1.4E1 | 1.7E1 | 1.0E-9 | 1.0E-9 | 1.8E2 | 7.4E1 | 320 | 31 | 320 | 31 | 0.56 |
| Mr | pg/ml | 1.0E-9 | 1.0E-9 | 3.2E1 | 1.7E2 | 1.9E2 | 6.2E2 | 1.0E-9 | 1.0E-9 | 2.2E3 | 3.4E3 | 320 | 31 | 320 | 31 | 0.59 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ms | pg/ml | 3.2E2 | 3.8E2 | 4.6E2 | 5.8E2 | 5.2E2 | 9.8E2 | 1.0E-9 | 1.0E-9 | 3.2E3 | 4.7E3 | 320 | 31 | 320 | 31 | 0.48 |
| Mt | pg/ml | 2.2E-1 | 2.3E0 | 9.0E0 | 1.3E2 | 4.6E1 | 5.8E2 | 1.0E-9 | 1.0E-9 | 7.1E2 | 3.2E3 | 320 | 31 | 320 | 31 | 0.65 |
| Mu | pg/ml | 1.0E-9 | 1.0E-9 | 7.6E-1 | 9.8E0 | 6.3E0 | 4.3E1 | 1.0E-9 | 1.0E-9 | 9.9E1 | 2.3E2 | 320 | 31 | 320 | 31 | 0.55 |
| Mv | pg/ml | 1.0E-9 | 1.0E-9 | 6.3E1 | 1.4E2 | 3.4E2 | 3.0E2 | 1.0E-9 | 1.0E-9 | 4.5E3 | 9.2E2 | 320 | 31 | 320 | 31 | 0.59 |
| Mw | pg/ml | 3.7E1 | 6.9E1 | 2.7E2 | 5.0E2 | 1.4E3 | 1.1E3 | 1.0E-9 | 1.0E-9 | 1.8E4 | 5.2E3 | 320 | 31 | 320 | 31 | 0.57 |
| Mx | pg/ml | 1.0E-9 | 1.0E-9 | 2.3E-1 | 2.5E0 | 8.8E-1 | 6.6E0 | 1.0E-9 | 1.0E-9 | 9.2E0 | 3.2E1 | 320 | 31 | 320 | 31 | 0.67 |
| My | pg/ml | 1.0E-9 | 1.0E-9 | 3.3E2 | 2.7E2 | 2.5E3 | 6.0E2 | 1.0E-9 | 1.0E-9 | 3.9E4 | 2.1E3 | 320 | 31 | 320 | 31 | 0.54 |
| Mz | pg/ml | 1.1E1 | 2.9E1 | 2.8E1 | 1.2E2 | 8.5E1 | 3.4E2 | 1.0E-9 | 1.0E-9 | 1.3E3 | 1.9E3 | 320 | 31 | 320 | 31 | 0.67 |
| Na | pg/ml | 1.0E-9 | 1.0E-9 | 7.7E-1 | 1.1E0 | 3.0E0 | 2.4E0 | 1.0E-9 | 1.0E-9 | 4.2E1 | 9.6E0 | 320 | 31 | 320 | 31 | 0.54 |
| Nb | pg/ml | 2.1E0 | 3.6E0 | 3.9E0 | 1.3E1 | 1.2E1 | 3.8E1 | 1.0E-9 | 1.0E-9 | 1.5E2 | 2.1E2 | 320 | 31 | 320 | 31 | 0.65 |
| Nc | pg/ml | 3.3E2 | 1.6E2 | 5.3E2 | 3.0E2 | 7.6E2 | 3.7E2 | 1.0E-9 | 1.0E-9 | 9.2E3 | 1.4E3 | 320 | 31 | 320 | 31 | 0.40 |
| Nd | pg/ml | 2.8E1 | 1.1E1 | 3.5E1 | 2.6E1 | 1.4E2 | 3.2E1 | 1.0E-9 | 7.2E-1 | 2.1E3 | 1.5E2 | 320 | 31 | 320 | 31 | 0.48 |
| Ne | pg/ml | 4.3E2 | 2.5E2 | 5.3E2 | 3.7E2 | 5.5E2 | 6.5E2 | 1.0E-9 | 1.0E-9 | 7.0E3 | 3.6E3 | 320 | 31 | 320 | 31 | 0.34 |
| Nf | pg/ml | 1.0E-9 | 1.0E-9 | 2.9E0 | 4.6E0 | 1.2E1 | 2.0E1 | 1.0E-9 | 1.0E-9 | 1.3E2 | 1.1E2 | 320 | 31 | 320 | 31 | 0.50 |
| Ng | pg/ml | 1.3E1 | 6.7E0 | 9.3E1 | 5.6E1 | 1.9E2 | 9.1E1 | 1.0E-9 | 1.0E-9 | 1.6E3 | 3.0E2 | 320 | 31 | 320 | 31 | 0.45 |
| Nh | pg/ml | 6.3E1 | 3.4E1 | 8.1E1 | 6.0E1 | 7.3E1 | 8.9E1 | 1.0E-9 | 4.5E0 | 5.6E2 | 5.1E2 | 320 | 31 | 320 | 31 | 0.36 |
| Ni | pg/ml | 1.0E-9 | 1.0E-9 | 8.2E1 | 1.0E2 | 1.4E2 | 1.5E2 | 1.0E-9 | 1.0E-9 | 1.1E3 | 4.9E2 | 320 | 31 | 320 | 31 | 0.52 |
| Nj | pg/ml | 7.4E0 | 4.4E0 | 1.1E1 | 6.1E0 | 1.2E1 | 5.5E0 | 1.0E-9 | 1.0E-9 | 6.9E1 | 1.9E1 | 320 | 31 | 320 | 31 | 0.38 |
| Nk | pg/ml | 1.8E1 | 3.4E0 | 3.3E1 | 1.7E1 | 4.0E1 | 2.3E1 | 1.0E-9 | 1.0E-9 | 2.0E2 | 8.3E1 | 320 | 31 | 320 | 31 | 0.38 |
| Nl | pg/ml | 4.4E1 | 2.3E1 | 5.9E1 | 3.0E1 | 7.7E1 | 3.7E1 | 1.0E-9 | 1.0E-9 | 1.1E3 | 1.8E2 | 320 | 31 | 320 | 31 | 0.33 |
| Hl | pg/ml | 1.1E1 | 2.3E0 | 1.1E2 | 1.6E1 | 5.3E2 | 2.2E1 | 1.0E-9 | 1.0E-9 | 3.6E3 | 6.1E1 | 46 | 12 | 46 | 12 | 0.40 |
| Ho | pg/ml | 1.7E1 | 2.5E1 | 2.9E1 | 4.8E1 | 5.7E1 | 5.3E1 | 1.0E-9 | 7.6E0 | 3.9E2 | 1.7E2 | 46 | 12 | 46 | 12 | 0.66 |
| Hp | ng/ml | 1.6E0 | 1.7E0 | 1.4E2 | 7.6E1 | 3.2E2 | 2.6E2 | 3.6E-1 | 1.0E-9 | 8.9E2 | 8.9E2 | 46 | 12 | 46 | 12 | 0.49 |
| Tz | pg/ml | 4.1E3 | 9.8E3 | 6.1E3 | 1.5E5 | 7.1E3 | 4.7E5 | 1.0E-9 | 9.8E1 | 5.3E4 | 2.1E6 | 114 | 19 | 114 | 19 | 0.66 |
| Ua | pg/ml | 3.3E3 | 4.3E3 | 3.2E4 | 2.0E4 | 2.0E5 | 2.8E4 | 1.0E-9 | 2.7E2 | 2.1E6 | 9.9E4 | 114 | 19 | 114 | 19 | 0.52 |
| Ub | pg/ml | 5.6E2 | 4.9E2 | 9.1E2 | 5.6E2 | 1.2E3 | 4.6E2 | 1.0E-9 | 2.3E0 | 9.8E3 | 1.4E3 | 114 | 19 | 114 | 19 | 0.42 |
| Ue | pg/ml | 3.1E1 | 1.9E1 | 3.8E1 | 2.5E1 | 3.4E1 | 2.3E1 | 9.8E-2 | 4.5E0 | 2.7E2 | 1.1E2 | 114 | 19 | 114 | 19 | 0.33 |
| Uc | pg/ml | 6.8E2 | 7.9E2 | 1.7E3 | 1.9E3 | 5.4E3 | 2.6E3 | 1.0E-9 | 1.5E1 | 5.7E4 | 9.4E3 | 114 | 19 | 114 | 19 | 0.53 |
| Ud | pg/ml | 1.0E-9 | 1.0E-9 | 3.9E0 | 1.0E-9 | 3.7E1 | 0.0E0 | 1.0E-9 | 1.0E-9 | 3.9E2 | 1.0E-9 | 114 | 19 | 114 | 19 | 0.49 |
| Hq | pg/ml | 1.1E0 | 2.4E0 | 1.7E2 | 1.3E1 | 1.9E3 | 3.3E1 | 1.0E-9 | 1.0E-9 | 2.8E4 | 1.8E2 | 318 | 31 | 318 | 31 | 0.62 |
| Hr | pg/ml | 9.1E1 | 1.1E2 | 6.8E2 | 4.7E2 | 1.4E3 | 9.3E2 | 1.0E-9 | 1.6E1 | 1.2E4 | 3.8E3 | 318 | 31 | 318 | 31 | 0.54 |
| Hu | pg/ml | 5.3E0 | 2.9E1 | 4.0E3 | 9.2E3 | 3.8E4 | 4.7E4 | 1.0E-9 | 1.0E-9 | 6.3E5 | 2.6E5 | 318 | 31 | 318 | 31 | 0.59 |
| Hv | pg/ml | 1.3E0 | 2.8E0 | 5.2E0 | 1.0E1 | 5.0E1 | 3.0E1 | 1.0E-9 | 1.0E-9 | 8.9E2 | 1.6E2 | 318 | 31 | 318 | 31 | 0.67 |
| Hw | pg/ml | 6.3E0 | 1.1E1 | 4.5E1 | 3.5E1 | 5.3E2 | 9.1E1 | 1.0E-9 | 1.0E-9 | 9.4E3 | 5.0E2 | 318 | 31 | 318 | 31 | 0.62 |
| Hx | pg/ml | 8.5E0 | 2.2E1 | 5.6E1 | 9.0E1 | 5.2E2 | 2.4E2 | 1.0E-9 | 1.0E-9 | 9.3E3 | 1.3E3 | 318 | 31 | 318 | 31 | 0.64 |
| Ib | ng/ml | 3.9E-2 | 2.0E-2 | 1.6E0 | 3.3E-1 | 7.0E0 | 1.1E0 | 1.0E-9 | 1.0E-9 | 5.6E1 | 4.7E0 | 113 | 18 | 113 | 18 | 0.43 |
| Ic | U/ml | 2.2E2 | 2.2E2 | 9.4E2 | 2.1E2 | 6.2E3 | 1.6E2 | 2.4E0 | 1.6E1 | 6.5E4 | 4.2E2 | 113 | 18 | 113 | 18 | 0.51 |
| Id | U/ml | 6.8E-1 | 5.4E-1 | 5.0E0 | 1.1E0 | 4.1E1 | 1.3E0 | 1.0E-9 | 1.0E-9 | 4.3E2 | 4.5E0 | 113 | 18 | 113 | 18 | 0.45 |
| Tt | pg/ml | 1.7E2 | 1.8E2 | 1.7E2 | 1.8E2 | 5.9E1 | 5.3E1 | 4.3E1 | 1.0E2 | 4.4E2 | 2.8E2 | 105 | 17 | 105 | 17 | 0.54 |
| To | pg/ml | 1.6E0 | 1.8E0 | 2.2E0 | 2.0E0 | 3.0E0 | 2.0E0 | 1.0E-9 | 1.0E-9 | 2.3E1 | 6.4E0 | 110 | 19 | 110 | 19 | 0.49 |
| Tr | pg/ml | 3.5E0 | 2.7E0 | 9.0E0 | 7.7E0 | 3.1E1 | 1.4E1 | 1.0E-9 | 1.0E-9 | 3.1E2 | 5.4E1 | 108 | 18 | 108 | 18 | 0.45 |
| Tn | pg/ml | 3.2E1 | 5.7E1 | 1.1E2 | 2.7E2 | 3.4E2 | 4.8E2 | 1.0E-9 | 9.0E0 | 2.3E3 | 2.0E3 | 110 | 19 | 110 | 19 | 0.63 |
| Tv | ng/ml | 1.2E1 | 1.2E1 | 9.0E1 | 4.5E1 | 6.8E2 | 9.9E1 | 1.0E-9 | 1.0E-9 | 7.1E3 | 4.3E2 | 110 | 19 | 110 | 19 | 0.52 |
| Ih | ng/ml | 6.3E1 | 1.1E2 | 2.5E2 | 4.7E2 | 4.4E2 | 7.0E2 | 1.0E-9 | 1.4E0 | 3.6E3 | 2.8E3 | 319 | 31 | 319 | 31 | 0.58 |
| Ii | ng/ml | 7.9E1 | 1.2E2 | 2.2E2 | 3.3E2 | 5.2E2 | 8.1E2 | 7.3E-1 | 7.5E-1 | 5.2E3 | 4.5E3 | 319 | 31 | 319 | 31 | 0.56 |
| Ij | ng/ml | 7.8E1 | 1.2E2 | 2.5E2 | 3.4E2 | 1.5E3 | 5.5E2 | 2.8E0 | 9.5E0 | 2.4E4 | 2.0E3 | 315 | 31 | 315 | 31 | 0.61 |
| Ik | ng/ml | 1.0E1 | 9.1E1 | 1.4E3 | 2.8E2 | 1.2E4 | 4.0E2 | 5.9E-1 | 6.2E-1 | 1.2E5 | 1.5E3 | 315 | 31 | 315 | 31 | 0.65 |
| Il | ng/ml | 3.6E2 | 4.9E2 | 1.3E3 | 2.4E3 | 2.8E3 | 4.1E3 | 1.0E-9 | 1.0E-9 | 1.2E4 | 1.2E4 | 313 | 31 | 313 | 31 | 0.56 |
| Im | ng/ml | 2.1E2 | 7.0E2 | 4.4E2 | 1.2E3 | 7.8E2 | 2.7E3 | 1.4E1 | 2.2E1 | 6.2E3 | 1.5E4 | 315 | 31 | 315 | 31 | 0.63 |
| In | ng/ml | 3.5E0 | 2.9E0 | 3.4E1 | 4.4E1 | 2.7E2 | 1.2E2 | 1.0E-9 | 1.0E-9 | 4.5E3 | 5.9E2 | 319 | 31 | 319 | 31 | 0.50 |
| Hb | ng/ml | 2.6E1 | 1.8E1 | 3.5E1 | 3.4E1 | 3.4E1 | 4.8E1 | 1.6E0 | 4.8E-1 | 2.0E2 | 1.9E2 | 116 | 18 | 116 | 18 | 0.40 |
| Hc | pg/ml | 6.9E2 | 4.8E2 | 3.0E3 | 4.5E3 | 1.0E4 | 1.2E4 | 1.0E-9 | 1.4E2 | 1.0E5 | 5.0E4 | 116 | 18 | 116 | 18 | 0.49 |
| Hf | ng/ml | 2.0E2 | 1.3E2 | 4.2E2 | 2.4E2 | 5.8E2 | 2.7E2 | 1.0E-9 | 1.0E-9 | 3.2E3 | 8.8E2 | 116 | 18 | 116 | 18 | 0.44 |

# EP 3 070 474 A2

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Io | ng/ml | 9.3E3 | 1.0E4 | 1.9E4 | 2.3E4 | 4.7E4 | 3.9E4 | 1.0E-9 | 2.4E2 | 7.1E5 | 2.0E5 | 319 | 31 | 319 | 31 | 0.54 |
| Ip | ng/ml | 9.3E0 | 3.0E1 | 2.1E1 | 2.7E1 | 2.7E1 | 2.1E1 | 1.0E-9 | 5.0E-3 | 2.3E2 | 7.1E1 | 319 | 31 | 319 | 31 | 0.60 |
| Iq | ug/ml | 1.0E-1 | 2.8E-1 | 4.3E1 | 9.2E0 | 7.6E2 | 3.9E1 | 1.0E-9 | 1.0E-9 | 1.4E4 | 2.2E2 | 319 | 31 | 319 | 31 | 0.56 |
| Ir | ug/ml | 3.7E-1 | 8.4E-1 | 5.5E0 | 1.5E1 | 3.8E1 | 3.6E1 | 1.0E-9 | 1.0E-9 | 5.1E2 | 1.3E2 | 318 | 31 | 318 | 31 | 0.63 |
| Is | ng/ml | 1.8E0 | 8.8E0 | 8.4E0 | 3.6E1 | 3.4E1 | 5.6E1 | 1.0E-9 | 1.4E-1 | 5.5E2 | 2.6E2 | 319 | 31 | 319 | 31 | 0.69 |
| It | ng/ml | 2.1E0 | 3.4E0 | 2.1E1 | 5.3E1 | 9.8E1 | 1.7E2 | 1.0E-9 | 1.0E-9 | 1.1E3 | 8.3E2 | 319 | 31 | 319 | 31 | 0.59 |
| Iu | ng/ml | 1.7E2 | 1.4E2 | 1.3E3 | 2.6E3 | 4.2E3 | 6.3E3 | 1.0E-9 | 1.0E-9 | 2.9E4 | 2.4E4 | 319 | 31 | 319 | 31 | 0.52 |
| Iv | ng/ml | 1.1E1 | 2.7E1 | 9.7E1 | 3.4E2 | 9.1E2 | 9.5E2 | 1.0E-9 | 1.9E0 | 1.6E4 | 3.8E3 | 318 | 31 | 318 | 31 | 0.69 |
| Iz | ng/ml | 1.2E2 | 1.1E2 | 3.8E2 | 3.0E2 | 7.6E2 | 4.4E2 | 1.5E0 | 8.8E-1 | 6.1E3 | 1.7E3 | 116 | 18 | 116 | 18 | 0.46 |
| Yg | pg/ml | 2.6E2 | 7.8E2 | 1.7E3 | 1.7E3 | 7.7E3 | 2.4E3 | 1.0E-9 | 1.0E-9 | 5.0E4 | 8.2E3 | 42 | 12 | 42 | 12 | 0.55 |
| Yh | pg/ml | 2.1E2 | 3.3E2 | 3.7E2 | 7.3E2 | 4.8E2 | 8.8E2 | 1.0E-9 | 1.0E-9 | 2.1E3 | 3.0E3 | 42 | 12 | 42 | 12 | 0.63 |
| Yi | pg/ml | 3.2E2 | 5.6E2 | 5.0E2 | 3.6E3 | 4.9E2 | 7.7E3 | 1.0E-9 | 1.0E-9 | 2.0E3 | 2.6E4 | 42 | 12 | 42 | 12 | 0.57 |
| Yk | U/ml | 1.0E-9 | 1.0E-9 | 1.8E-1 | 3.9E-1 | 5.7E-1 | 7.1E-1 | 1.0E-9 | 1.0E-9 | 3.0E0 | 2.0E0 | 42 | 12 | 42 | 12 | 0.58 |
| Yj | pg/ml | 1.5E2 | 1.0E2 | 3.4E2 | 1.4E2 | 5.5E2 | 1.3E2 | 9.7E0 | 1.0E-9 | 3.2E3 | 4.7E2 | 42 | 12 | 42 | 12 | 0.36 |
| Yd | ng/ml | 1.9E-1 | 3.3E-1 | 3.6E-1 | 5.7E-1 | 4.2E-1 | 7.7E-1 | 6.6E-3 | 1.6E-2 | 1.8E0 | 2.3E0 | 46 | 12 | 46 | 12 | 0.54 |
| Wb | pg/ml | 2.8E4 | 3.2E4 | 3.6E4 | 8.4E4 | 2.5E4 | 1.8E5 | 4.9E3 | 7.5E3 | 1.5E5 | 6.4E5 | 46 | 12 | 46 | 12 | 0.56 |
| Vz | pg/ml | 3.9E0 | 2.4E0 | 5.1E0 | 3.1E0 | 4.9E0 | 2.7E0 | 1.0E-9 | 3.0E-1 | 2.2E1 | 9.6E0 | 46 | 12 | 46 | 12 | 0.36 |
| Si | ng/ml | 1.3E0 | 9.3E-1 | 2.1E0 | 1.7E0 | 2.6E0 | 1.9E0 | 1.1E-1 | 8.6E-3 | 1.0E1 | 6.0E0 | 46 | 12 | 46 | 12 | 0.47 |
| Sf | mIU/mL | 2.0E1 | 1.0E1 | 5.4E1 | 1.5E1 | 1.2E2 | 1.3E1 | 6.2E-1 | 1.3E0 | 7.2E2 | 4.3E1 | 46 | 12 | 46 | 12 | 0.39 |
| Sh | mIU/mL | 1.7E1 | 1.1E1 | 4.8E1 | 1.3E1 | 9.7E1 | 1.5E1 | 7.8E-2 | 1.4E-1 | 5.7E2 | 5.8E1 | 46 | 12 | 46 | 12 | 0.37 |
| Sj | ng/ml | 4.2E-1 | 4.5E-1 | 4.2E-1 | 4.6E-1 | 8.4E-2 | 1.3E-1 | 2.5E-1 | 3.2E-1 | 6.1E-1 | 7.2E-1 | 46 | 12 | 46 | 12 | 0.59 |
| Rc | pg/ml | 7.0E3 | 5.3E3 | 7.8E3 | 7.5E3 | 5.4E3 | 8.6E3 | 3.9E2 | 5.5E2 | 2.8E4 | 3.9E4 | 114 | 19 | 114 | 19 | 0.41 |
| Rb | pg/ml | 8.5E-1 | 1.0E-9 | 3.1E0 | 2.7E0 | 6.4E0 | 4.5E0 | 1.0E-9 | 1.0E-9 | 5.6E1 | 1.3E1 | 114 | 19 | 114 | 19 | 0.45 |
| Zq | 2.6ng/ml | 3.0E2 | 3.4E2 | 3.1E2 | 2.9E2 | 2.3E2 | 1.8E2 | 1.4E2 | 1.7E1 | 9.7E2 | 5.0E2 | 45 | 11 | 45 | 11 | 0.51 |
| Zw | 2.5ng/ml | 5.2E0 | 5.0E0 | 1.0E1 | 1.2E1 | 1.4E1 | 1.8E1 | 1.4E-1 | 2.4E-1 | 5.9E1 | 6.3E1 | 46 | 12 | 46 | 12 | 0.47 |
| Zx | 2.3mU/ml | 1.3E-1 | 1.2E-1 | 2.8E-1 | 3.1E-1 | 5.1E-1 | 5.2E-1 | 3.2E-2 | 6.1E-2 | 2.9E0 | 1.9E0 | 46 | 12 | 46 | 12 | 0.54 |
| Pz | ng/ml | 3.4E3 | 7.2E3 | 5.6E3 | 6.3E3 | 6.1E3 | 4.3E3 | 1.6E1 | 4.0E1 | 7.0E4 | 1.3E4 | 316 | 30 | 316 | 30 | 0.58 |
| Qa | ng/ml | 3.5E3 | 1.3E4 | 7.1E3 | 1.5E4 | 1.4E4 | 1.2E4 | 1.5E2 | 4.3E2 | 2.2E5 | 3.6E4 | 316 | 30 | 316 | 30 | 0.69 |
| Qb | ng/ml | 1.0E2 | 2.1E2 | 2.2E2 | 4.5E2 | 4.0E2 | 7.6E2 | 7.9E-1 | 1.3E1 | 5.3E3 | 4.1E3 | 316 | 30 | 316 | 30 | 0.64 |
| Qc | ng/ml | 2.0E2 | 3.6E2 | 4.4E2 | 6.4E2 | 6.0E2 | 6.3E2 | 1.0E-9 | 2.0E1 | 4.3E3 | 2.8E3 | 316 | 30 | 316 | 30 | 0.62 |
| Qd | ng/ml | 8.6E3 | 2.2E4 | 2.3E4 | 7.2E4 | 1.2E5 | 9.4E4 | 1.5E2 | 1.2E3 | 2.0E6 | 4.3E5 | 316 | 30 | 316 | 30 | 0.69 |
| Qe | ng/ml | 8.5E2 | 2.5E3 | 2.0E3 | 3.6E3 | 5.8E3 | 4.1E3 | 1.0E-9 | 5.7E1 | 9.7E4 | 1.8E4 | 316 | 30 | 316 | 30 | 0.63 |
| Jd | ng/ml | 8.6E-1 | 1.5E0 | 4.0E0 | 3.9E0 | 1.5E1 | 5.6E0 | 1.0E-9 | 1.0E-9 | 1.2E2 | 1.8E1 | 114 | 19 | 114 | 19 | 0.56 |
| Je | ng/ml | 1.0E-9 | 1.0E-9 | 1.9E0 | 9.9E-1 | 5.2E0 | 2.5E0 | 1.0E-9 | 1.0E-9 | 4.6E1 | 1.1E1 | 114 | 19 | 114 | 19 | 0.43 |
| Jf | ng/ml | 1.0E-9 | 1.0E-9 | 1.3E0 | 5.8E-1 | 2.4E0 | 1.2E0 | 1.0E-9 | 1.0E-9 | 1.2E1 | 4.1E0 | 114 | 19 | 114 | 19 | 0.42 |
| Jg | ng/ml | 4.6E2 | 7.8E2 | 7.8E2 | 1.1E3 | 9.6E2 | 1.0E3 | 5.8E0 | 2.4E1 | 1.0E4 | 3.9E3 | 318 | 31 | 318 | 31 | 0.59 |
| Jh | ng/ml | 2.7E0 | 6.0E0 | 2.2E1 | 2.9E1 | 8.8E1 | 5.6E1 | 1.0E-9 | 1.0E-9 | 1.2E3 | 2.9E2 | 318 | 31 | 318 | 31 | 0.65 |
| Ji | ng/ml | 5.5E1 | 1.2E2 | 8.5E1 | 2.0E2 | 1.1E2 | 2.1E2 | 1.1E0 | 8.9E0 | 1.3E3 | 6.9E2 | 318 | 31 | 318 | 31 | 0.66 |
| Sr | pg/mL | 3.9E2 | 5.0E2 | 1.0E3 | 1.1E3 | 2.2E3 | 1.2E3 | 1.0E-9 | 1.0E-9 | 2.1E4 | 4.0E3 | 113 | 19 | 113 | 19 | 0.55 |
| Ss | pg/mL | 8.2E4 | 1.0E5 | 1.5E5 | 1.1E5 | 1.9E5 | 8.3E4 | 2.7E3 | 7.6E3 | 1.3E6 | 2.4E5 | 113 | 19 | 113 | 19 | 0.51 |
| St | pg/mL | 2.5E7 | 3.3E7 | 5.3E7 | 1.1E8 | 8.1E7 | 2.6E8 | 1.0E-9 | 9.9E5 | 5.4E8 | 1.2E9 | 112 | 19 | 112 | 19 | 0.53 |
| Wc | ng/ml | 1.0E-9 | 5.4E-2 | 9.1E-2 | 1.0E-1 | 2.8E-1 | 1.8E-1 | 1.0E-9 | 1.0E-9 | 1.8E0 | 6.3E-1 | 46 | 12 | 46 | 12 | 0.62 |
| Wd | ng/ml | 9.5E0 | 1.1E1 | 3.3E1 | 6.1E1 | 7.6E1 | 1.2E2 | 1.0E0 | 2.3E0 | 3.8E2 | 4.1E2 | 46 | 12 | 46 | 12 | 0.58 |
| We | ng/ml | 2.8E-1 | 5.4E-1 | 1.3E0 | 1.6E0 | 3.5E0 | 2.8E0 | 1.0E-9 | 1.0E-9 | 2.3E1 | 9.7E0 | 46 | 12 | 46 | 12 | 0.60 |
| Wg | ng/ml | 1.0E-9 | 1.0E-9 | 1.2E-2 | 1.0E-9 | 7.9E-2 | 0.0E0 | 1.0E-9 | 1.0E-9 | 5.3E-1 | 1.0E-9 | 46 | 12 | 46 | 12 | 0.48 |
| Wh | ng/ml | 9.8E-3 | 2.1E-2 | 4.4E-2 | 5.9E-2 | 9.5E-2 | 1.2E-1 | 1.0E-9 | 1.0E-9 | 3.6E-1 | 4.2E-1 | 46 | 12 | 46 | 12 | 0.61 |
| Wf | ng/ml | 1.0E-9 | 1.0E-9 | 1.4E-1 | 6.5E-2 | 3.9E-1 | 1.4E-1 | 1.0E-9 | 1.0E-9 | 2.3E0 | 4.7E-1 | 46 | 12 | 46 | 12 | 0.47 |
| Ra | pg/ml | 1.0E-9 | 1.0E-9 | 1.0E0 | 3.7E-1 | 6.0E0 | 1.5E0 | 1.0E-9 | 1.0E-9 | 6.4E1 | 6.5E0 | 114 | 19 | 114 | 19 | 0.44 |
| Qz | pg/ml | 9.3E0 | 1.1E1 | 5.2E1 | 4.1E1 | 8.8E1 | 6.1E1 | 1.0E-9 | 1.0E-9 | 4.5E2 | 1.8E2 | 114 | 19 | 114 | 19 | 0.55 |
| Qy | pg/ml | 3.9E-1 | 4.7E-1 | 4.3E0 | 6.2E1 | 2.3E1 | 1.9E2 | 1.0E-9 | 1.0E-9 | 2.3E2 | 7.3E2 | 114 | 19 | 114 | 19 | 0.49 |
| Qx | pg/ml | 1.0E-9 | 1.0E-9 | 3.8E0 | 3.7E0 | 2.1E1 | 8.8E0 | 1.0E-9 | 1.0E-9 | 1.9E2 | 3.1E1 | 114 | 19 | 114 | 19 | 0.54 |
| Qw | pg/ml | 1.0E-9 | 4.5E-1 | 3.0E0 | 1.8E0 | 1.0E1 | 5.1E0 | 1.0E-9 | 1.0E-9 | 6.6E1 | 2.3E1 | 114 | 19 | 114 | 19 | 0.53 |
| Qv | pg/ml | 2.0E4 | 1.2E4 | 4.0E4 | 1.3E4 | 9.8E4 | 1.2E4 | 4.0E2 | 1.0E-9 | 9.4E5 | 5.0E4 | 114 | 19 | 114 | 19 | 0.32 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|-----|--------|-----|-----|
| | | NonDis | Dis | NonDis | Dis | NonDis | Dis | NonDis | Dis | NonDis | Dis | NonDis | Dis | NonDis | Dis | |
| Qu | pg/ml | 8.0E0 | 3.8E0 | 8.7E1 | 1.3E2 | 1.8E2 | 2.2E2 | 1.0E-9 | 1.0E-9 | 9.8E2 | 7.3E2 | 114 | 19 | 114 | 19 | 0.51 |
| Qt | pg/ml | 1.1E1 | 2.0E1 | 4.4E1 | 5.0E1 | 1.0E2 | 7.4E1 | 1.0E-9 | 1.0E-9 | 7.1E2 | 2.2E2 | 114 | 19 | 114 | 19 | 0.59 |
| Qh | ng/ml | 1.8E1 | 2.3E1 | 4.3E1 | 9.6E1 | 6.8E1 | 1.9E2 | 2.5E-1 | 1.6E0 | 4.6E2 | 8.0E2 | 114 | 19 | 114 | 19 | 0.55 |
| Qg | ng/ml | 7.9E0 | 5.3E0 | 1.4E1 | 1.1E1 | 2.7E1 | 1.8E1 | 1.5E-1 | 1.0E0 | 2.7E2 | 8.1E1 | 114 | 19 | 114 | 19 | 0.40 |
| Jj | ng/ml | 5.3E2 | 2.2E2 | 2.0E3 | 5.0E2 | 1.9E4 | 4.8E2 | 2.3E0 | 8.7E0 | 3.4E5 | 2.0E3 | 318 | 31 | 318 | 31 | 0.38 |
| Jk | ng/ml | 2.6E0 | 5.6E0 | 1.9E1 | 4.6E1 | 4.4E1 | 8.5E1 | 1.0E-9 | 4.3E-2 | 2.8E2 | 3.9E2 | 318 | 31 | 318 | 31 | 0.58 |
| Jl | ng/ml | 4.6E-1 | 1.2E0 | 1.9E0 | 3.3E2 | 4.6E0 | 1.8E3 | 1.2E-3 | 5.4E-3 | 4.0E1 | 9.9E3 | 318 | 31 | 318 | 31 | 0.67 |
| Jm | ng/ml | 2.0E1 | 3.3E1 | 6.9E1 | 5.7E1 | 1.7E2 | 7.5E1 | 1.0E-9 | 1.0E-9 | 2.1E3 | 2.9E2 | 318 | 31 | 318 | 31 | 0.52 |
| Jn | pg/ml | 3.4E-1 | 1.1E0 | 5.3E0 | 3.4E1 | 5.0E1 | 1.4E2 | 1.0E-9 | 1.0E-9 | 6.2E2 | 7.3E2 | 318 | 31 | 318 | 31 | 0.63 |
| Jo | pg/ml | 3.8E3 | 5.1E3 | 5.1E3 | 6.2E3 | 6.8E3 | 7.2E3 | 2.0E1 | 2.4E1 | 1.0E5 | 3.6E4 | 318 | 31 | 318 | 31 | 0.52 |
| Jp | pg/ml | 7.0E4 | 8.8E4 | 7.3E4 | 9.4E4 | 3.9E4 | 4.6E4 | 5.8E2 | 4.6E3 | 3.8E5 | 2.1E5 | 318 | 31 | 318 | 31 | 0.66 |
| Jq | pg/ml | 9.5E1 | 1.7E2 | 1.8E2 | 3.8E2 | 5.2E2 | 5.3E2 | 1.0E0 | 7.4E0 | 8.7E3 | 2.4E3 | 318 | 31 | 318 | 31 | 0.62 |
| Jr | pg/ml | 4.4E0 | 1.5E1 | 7.1E1 | 3.4E2 | 6.7E2 | 1.4E3 | 1.0E-9 | 1.0E-9 | 1.1E4 | 7.4E3 | 318 | 31 | 318 | 31 | 0.65 |
| Js | pg/ml | 1.4E1 | 1.9E1 | 7.6E1 | 2.5E2 | 5.9E2 | 7.3E2 | 1.0E-9 | 2.1E0 | 1.0E4 | 3.0E3 | 318 | 31 | 318 | 31 | 0.61 |
| Jt | pg/ml | 2.4E3 | 2.6E3 | 3.1E3 | 5.7E3 | 3.5E3 | 8.7E3 | 2.2E1 | 1.5E2 | 5.2E4 | 4.1E4 | 318 | 31 | 318 | 31 | 0.54 |
| Xa | pg/ml | 7.0E-1 | 5.0E0 | 3.5E1 | 6.3E1 | 1.8E2 | 1.6E2 | 1.0E-9 | 1.0E-9 | 1.2E3 | 5.6E2 | 46 | 12 | 46 | 12 | 0.62 |
| Ye | pg/ml | 1.0E-9 | 1.0E-9 | 4.9E0 | 8.0E-1 | 1.5E1 | 2.1E0 | 1.0E-9 | 1.0E-9 | 6.9E1 | 7.2E0 | 46 | 12 | 46 | 12 | 0.47 |
| Tm | pg/ml | 1.0E-9 | 1.0E-9 | 2.5E0 | 2.8E0 | 4.5E0 | 4.1E0 | 1.0E-9 | 1.0E-9 | 1.8E1 | 8.4E0 | 46 | 12 | 46 | 12 | 0.53 |
| Tl | pg/ml | 1.3E-1 | 1.1E-1 | 8.3E-1 | 2.6E-1 | 3.6E0 | 3.3E-1 | 1.0E-9 | 1.0E-9 | 2.5E1 | 8.2E-1 | 46 | 12 | 46 | 12 | 0.46 |
| Ju | mIU/ml | 1.1E1 | 6.1E0 | 2.6E1 | 1.3E1 | 3.7E1 | 1.6E1 | 1.7E-1 | 6.0E-1 | 2.3E2 | 6.2E1 | 114 | 19 | 114 | 19 | 0.40 |
| Jv | mIU/ml | 1.7E1 | 1.1E1 | 4.2E1 | 2.1E1 | 6.5E1 | 3.6E1 | 1.7E-2 | 7.9E-2 | 4.4E2 | 1.4E2 | 114 | 19 | 114 | 19 | 0.37 |
| Jy | ng/ml | 1.7E-3 | 1.4E-3 | 2.3E-3 | 2.9E-3 | 3.7E-3 | 4.9E-3 | 1.0E-9 | 4.5E-4 | 3.9E-2 | 2.3E-2 | 114 | 19 | 114 | 19 | 0.47 |
| Kc | pg/ml | 2.2E1 | 2.5E1 | 4.2E1 | 6.4E1 | 5.0E1 | 8.4E1 | 1.0E-9 | 6.2E0 | 2.7E2 | 3.2E2 | 116 | 18 | 116 | 18 | 0.56 |
| Kd | pg/ml | 1.0E-9 | 1.0E-9 | 5.3E2 | 3.9E2 | 3.6E3 | 1.2E3 | 1.0E-9 | 1.0E-9 | 3.8E4 | 5.0E3 | 116 | 18 | 116 | 18 | 0.48 |
| Ke | pg/ml | 1.3E4 | 1.3E4 | 1.7E4 | 2.1E4 | 3.0E4 | 2.0E4 | 1.0E3 | 6.7E2 | 3.2E5 | 6.3E4 | 116 | 18 | 116 | 18 | 0.53 |
| Kf | pg/mL | 6.3E0 | 6.7E0 | 7.4E0 | 7.4E0 | 8.9E0 | 5.6E0 | 1.0E-9 | 1.0E-9 | 7.8E1 | 2.1E1 | 116 | 18 | 116 | 18 | 0.53 |
| Kg | pg/mL | 9.9E2 | 7.8E2 | 1.9E3 | 3.8E3 | 3.4E3 | 8.7E3 | 7.7E1 | 1.6E2 | 2.7E4 | 3.6E4 | 116 | 18 | 116 | 18 | 0.44 |
| Ki | pg/ml | 6.0E1 | 7.5E1 | 7.1E1 | 7.6E1 | 5.6E1 | 2.7E1 | 1.0E-9 | 6.0E0 | 2.9E2 | 1.1E2 | 116 | 18 | 116 | 18 | 0.62 |
| Kj | pg/ml | 8.4E2 | 6.1E2 | 1.4E3 | 1.5E3 | 1.8E3 | 2.0E3 | 6.6E1 | 3.3E1 | 1.5E4 | 7.7E3 | 116 | 18 | 116 | 18 | 0.44 |
| Kk | pg/ml | 7.1E0 | 8.2E0 | 1.4E1 | 1.3E1 | 1.6E1 | 1.3E1 | 1.0E-9 | 1.0E-9 | 8.1E1 | 4.8E1 | 116 | 18 | 116 | 18 | 0.52 |
| Kl | pg/ml | 1.8E4 | 1.6E4 | 2.8E4 | 2.2E4 | 2.7E4 | 1.9E4 | 2.3E2 | 2.4E2 | 1.3E5 | 5.0E4 | 116 | 18 | 116 | 18 | 0.44 |
| Kn | pg/ml | 3.0E1 | 3.0E1 | 1.1E2 | 7.3E1 | 4.6E2 | 1.2E2 | 1.0E-9 | 1.0E-9 | 4.9E3 | 4.3E2 | 116 | 18 | 116 | 18 | 0.51 |
| Ko | pg/ml | 3.6E2 | 1.7E2 | 5.1E2 | 3.4E2 | 5.9E2 | 3.5E2 | 1.0E-9 | 1.0E-9 | 4.1E3 | 1.0E3 | 116 | 18 | 116 | 18 | 0.43 |
| Kp | pg/ml | 3.6E2 | 3.2E2 | 4.8E2 | 3.5E2 | 1.2E3 | 2.4E2 | 1.0E-9 | 1.0E-9 | 1.3E4 | 7.9E2 | 116 | 18 | 116 | 18 | 0.48 |
| Kq | pg/ml | 3.3E2 | 4.7E2 | 1.8E3 | 1.0E3 | 1.5E4 | 1.4E3 | 5.1E0 | 1.6E0 | 1.6E5 | 5.5E3 | 111 | 18 | 111 | 18 | 0.56 |
| Kr | pg/ml | 3.8E-1 | 2.3E-1 | 5.9E0 | 1.5E0 | 3.9E1 | 2.0E0 | 1.0E-9 | 1.0E-9 | 4.2E2 | 5.7E0 | 111 | 18 | 111 | 18 | 0.48 |
| Ks | pg/ml | 1.5E4 | 1.0E4 | 2.1E4 | 2.0E4 | 1.8E4 | 1.9E4 | 4.5E2 | 5.1E1 | 7.9E4 | 5.0E4 | 111 | 18 | 111 | 18 | 0.45 |
| Ps | ng/ml | 1.5E2 | 4.6E2 | 5.5E2 | 2.0E3 | 1.4E3 | 3.8E3 | 1.6E0 | 5.5E0 | 9.0E3 | 1.2E4 | 46 | 12 | 46 | 12 | 0.61 |
| Kx | ng/ml | 2.8E-4 | 5.0E-3 | 7.4E-3 | 1.5E-2 | 1.5E-2 | 2.2E-2 | 1.0E-9 | 1.0E-9 | 1.0E-1 | 7.9E-2 | 115 | 18 | 115 | 18 | 0.61 |
| Ky | ng/ml | 1.2E-1 | 4.8E-1 | 3.3E-1 | 7.5E-1 | 6.8E-1 | 1.0E0 | 1.0E-9 | 1.0E-9 | 5.1E0 | 4.4E0 | 115 | 18 | 115 | 18 | 0.68 |
| Kz | ng/ml | 1.0E-9 | 1.0E-9 | 3.9E-3 | 4.6E-3 | 5.9E-3 | 7.9E-3 | 1.0E-9 | 1.0E-9 | 1.8E-2 | 2.5E-2 | 115 | 18 | 115 | 18 | 0.50 |
| Rz | ng/ml | 3.2E-1 | 7.5E-1 | 7.9E-1 | 1.4E0 | 1.2E0 | 2.1E0 | 1.1E-2 | 4.6E-3 | 6.7E0 | 7.5E0 | 46 | 12 | 46 | 12 | 0.57 |
| Ry | ng/ml | 1.6E-2 | 1.6E-2 | 3.3E-2 | 2.1E-2 | 5.5E-2 | 2.3E-2 | 1.0E-9 | 1.0E-9 | 3.5E-1 | 7.5E-2 | 46 | 12 | 46 | 12 | 0.44 |
| Rx | ng/ml | 1.0E-9 | 3.6E-3 | 1.1E-3 | 3.0E-3 | 2.0E-3 | 3.1E-3 | 1.0E-9 | 1.0E-9 | 8.6E-3 | 8.6E-3 | 46 | 12 | 46 | 12 | 0.70 |
| Ld | pg/ml | 1.0E-9 | 1.0E-9 | 3.8E0 | 4.9E0 | 9.0E0 | 8.0E0 | 1.0E-9 | 1.0E-9 | 6.9E1 | 2.9E1 | 117 | 17 | 117 | 17 | 0.55 |
| Lh | pg/ml | 1.3E4 | 3.2E4 | 2.3E4 | 5.3E4 | 4.2E4 | 7.9E4 | 1.0E-9 | 1.0E-9 | 4.8E5 | 4.1E5 | 319 | 31 | 319 | 31 | 0.64 |
| Li | pg/ml | 3.7E3 | 5.4E3 | 1.9E4 | 4.7E4 | 9.3E4 | 9.9E4 | 1.2E1 | 1.3E1 | 1.3E6 | 4.1E5 | 319 | 31 | 319 | 31 | 0.53 |
| Lj | pg/ml | 3.1E3 | 1.2E3 | 2.0E4 | 3.3E4 | 5.6E4 | 7.8E4 | 1.0E-9 | 1.0E-9 | 4.3E5 | 3.9E5 | 319 | 31 | 319 | 31 | 0.48 |
| Lp | pg/ml | 1.3E1 | 2.6E0 | 1.1E2 | 1.3E2 | 2.5E2 | 4.1E2 | 1.0E-9 | 1.0E-9 | 1.1E3 | 1.4E3 | 46 | 12 | 46 | 12 | 0.35 |
| Lt | pg/ml | 1.0E-9 | 1.0E-9 | 1.9E0 | 1.0E-9 | 6.2E0 | 0.0E0 | 1.0E-9 | 1.0E-9 | 2.5E1 | 1.0E-9 | 46 | 12 | 46 | 12 | 0.46 |
| Rv | ng/ml | 5.0E-4 | 5.0E-4 | 1.5E-3 | 1.4E-3 | 3.1E-3 | 2.5E-3 | 1.0E-9 | 1.0E-9 | 1.6E-2 | 9.2E-3 | 46 | 12 | 46 | 12 | 0.53 |
| Ru | ng/ml | 1.0E-9 | 1.0E-9 | 2.6E-2 | 5.9E-3 | 8.7E-2 | 2.0E-2 | 1.0E-9 | 1.0E-9 | 3.5E-1 | 7.1E-2 | 46 | 12 | 46 | 12 | 0.49 |
| Rt | ng/ml | 7.8E-2 | 3.9E-2 | 2.6E-1 | 2.1E-1 | 1.1E0 | 2.6E-1 | 6.5E-3 | 1.3E-3 | 7.4E0 | 6.3E-1 | 46 | 12 | 46 | 12 | 0.47 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Yl | pg/ml | 1.3E1 | 5.6E0 | 2.3E1 | 1.3E1 | 3.4E1 | 1.6E1 | 1.0E-9 | 1.0E-9 | 2.2E2 | 4.9E1 | 46 | 12 | 46 | 12 | 0.36 |
| Rm | ng/ml | 1.8E1 | 2.0E1 | 4.9E1 | 5.4E1 | 8.8E1 | 7.3E1 | 2.2E-1 | 2.3E-1 | 6.5E2 | 2.5E2 | 113 | 19 | 113 | 19 | 0.49 |
| Rh | ng/ml | 1.8E2 | 1.4E2 | 4.8E2 | 1.1E3 | 1.7E3 | 3.9E3 | 7.5E0 | 2.5E1 | 1.7E4 | 1.7E4 | 113 | 19 | 113 | 19 | 0.41 |
| Ri | ng/ml | 4.4E-2 | 4.4E-2 | 3.8E0 | 4.2E0 | 7.7E0 | 1.0E1 | 1.0E-9 | 1.0E-9 | 4.9E1 | 4.5E1 | 113 | 19 | 113 | 19 | 0.49 |
| Rg | ng/ml | 1.0E-9 | 1.0E-9 | 9.4E-2 | 1.5E-2 | 4.6E-1 | 4.8E-2 | 1.0E-9 | 1.0E-9 | 3.3E0 | 2.1E-1 | 113 | 19 | 113 | 19 | 0.51 |
| Rj | ng/ml | 1.0E-9 | 1.0E-9 | 3.3E0 | 4.6E-1 | 2.5E1 | 1.0E0 | 1.0E-9 | 1.0E-9 | 2.7E2 | 3.2E0 | 113 | 19 | 113 | 19 | 0.45 |
| Rf | ng/ml | 4.0E-1 | 2.6E-1 | 7.7E-1 | 1.8E0 | 9.8E-1 | 4.2E0 | 2.1E-2 | 2.1E-2 | 6.2E0 | 1.7E1 | 113 | 19 | 113 | 19 | 0.44 |
| Ql | pg/ml | 2.6E0 | 1.1E1 | 1.2E1 | 1.5E1 | 2.4E1 | 1.9E1 | 1.0E-9 | 1.0E-9 | 1.8E2 | 5.5E1 | 114 | 19 | 114 | 19 | 0.56 |
| Qm | pg/ml | 2.0E0 | 4.7E-1 | 2.0E1 | 2.0E1 | 3.6E1 | 3.1E1 | 1.0E-9 | 1.0E-9 | 1.9E2 | 8.6E1 | 114 | 19 | 114 | 19 | 0.51 |
| Qn | pg/ml | 6.1E-1 | 6.1E-1 | 5.6E0 | 1.5E1 | 2.2E1 | 4.7E1 | 1.0E-9 | 1.0E-9 | 2.2E2 | 2.0E2 | 114 | 19 | 114 | 19 | 0.51 |
| Nv | pg/ml | 3.8E3 | 8.9E3 | 8.9E3 | 2.4E4 | 1.8E4 | 3.7E4 | 1.0E-9 | 1.9E1 | 1.6E5 | 1.5E5 | 320 | 31 | 320 | 31 | 0.64 |
| Nw | pg/ml | 9.6E3 | 1.5E4 | 1.3E4 | 2.9E4 | 1.6E4 | 4.0E4 | 2.0E2 | 1.9E2 | 2.1E5 | 2.1E5 | 320 | 31 | 320 | 31 | 0.63 |
| Nx | pg/ml | 2.2E2 | 2.4E2 | 4.3E2 | 6.0E2 | 6.4E2 | 6.8E2 | 1.0E-9 | 1.0E-9 | 4.1E3 | 2.3E3 | 320 | 31 | 320 | 31 | 0.60 |
| Ny | pg/ml | 6.4E0 | 2.0E1 | 1.1E2 | 8.4E1 | 1.4E3 | 1.4E2 | 1.0E-9 | 1.0E-9 | 2.5E4 | 6.1E2 | 320 | 31 | 320 | 31 | 0.64 |
| Oa | pg/ml | 1.6E2 | 1.9E2 | 4.6E2 | 6.7E2 | 7.3E2 | 9.6E2 | 1.0E-9 | 1.0E-9 | 4.5E3 | 3.4E3 | 114 | 19 | 114 | 19 | 0.53 |
| Op | pg/ml | 4.4E5 | 4.5E5 | 4.4E5 | 4.5E5 | 1.5E5 | 2.1E5 | 5.2E4 | 1.4E5 | 7.3E5 | 7.5E5 | 46 | 12 | 46 | 12 | 0.50 |
| Oe | pg/ml | 3.1E1 | 1.0E-9 | 2.5E2 | 2.3E2 | 3.8E2 | 3.8E2 | 1.0E-9 | 1.0E-9 | 2.3E3 | 1.4E3 | 318 | 31 | 318 | 31 | 0.46 |
| Of | pg/ml | 1.4E2 | 1.3E2 | 5.3E3 | 2.5E3 | 2.1E4 | 6.3E3 | 1.0E-9 | 1.0E-9 | 1.9E5 | 2.4E4 | 320 | 31 | 320 | 31 | 0.47 |
| Og | pg/ml | 6.3E-2 | 7.8E-2 | 3.7E-1 | 1.1E-1 | 1.5E0 | 1.8E-1 | 1.0E-9 | 1.0E-9 | 1.9E1 | 9.4E-1 | 320 | 31 | 320 | 31 | 0.49 |
| Oh | pg/ml | 2.4E0 | 6.4E0 | 1.3E1 | 5.8E2 | 9.0E1 | 2.8E3 | 1.0E-9 | 1.0E-9 | 1.4E3 | 1.6E4 | 320 | 31 | 320 | 31 | 0.65 |
| Oi | pg/ml | 1.9E0 | 2.2E0 | 5.0E0 | 5.4E0 | 7.9E0 | 8.2E0 | 1.0E-9 | 1.0E-9 | 6.7E1 | 3.1E1 | 320 | 31 | 320 | 31 | 0.49 |
| Ok | pg/ml | 3.9E2 | 6.3E2 | 5.3E2 | 1.3E3 | 6.1E2 | 1.6E3 | 2.9E1 | 1.5E1 | 7.8E3 | 7.0E3 | 320 | 31 | 320 | 31 | 0.62 |
| Om | pg/ml | 4.1E2 | 6.1E2 | 9.5E2 | 1.2E3 | 3.5E3 | 1.4E3 | 1.0E-9 | 1.0E-9 | 5.1E4 | 5.6E3 | 320 | 31 | 320 | 31 | 0.63 |
| On | pg/ml | 1.8E2 | 4.7E2 | 2.8E2 | 9.3E2 | 4.2E2 | 1.6E3 | 1.0E-9 | 1.0E1 | 4.5E3 | 8.5E3 | 320 | 31 | 320 | 31 | 0.68 |
| Or | pg/ml | 9.7E0 | 3.5E1 | 3.2E1 | 1.0E2 | 7.1E1 | 1.4E2 | 1.0E-9 | 1.0E-9 | 5.1E2 | 4.5E2 | 117 | 18 | 117 | 18 | 0.64 |
| Ow | pg/ml | 3.8E1 | 5.4E1 | 1.9E2 | 4.4E2 | 8.0E2 | 8.3E2 | 1.0E-9 | 1.0E-9 | 8.1E3 | 3.0E3 | 117 | 18 | 117 | 18 | 0.60 |
| Ou | pg/ml | 5.1E2 | 1.0E3 | 1.0E3 | 2.6E3 | 1.7E3 | 3.2E3 | 1.0E-9 | 2.0E1 | 9.8E3 | 1.1E4 | 117 | 18 | 117 | 18 | 0.60 |
| Ul | ng/ml | 1.0E-9 | 1.0E-9 | 1.7E0 | 2.2E-1 | 7.0E0 | 9.7E-1 | 1.0E-9 | 1.0E-9 | 5.6E1 | 4.2E0 | 115 | 19 | 115 | 19 | 0.48 |
| Uo | ng/ml | 1.0E-9 | 1.0E-9 | 8.6E-2 | 1.3E-2 | 2.2E-1 | 5.6E-2 | 1.0E-9 | 1.0E-9 | 1.3E0 | 2.4E-1 | 115 | 19 | 115 | 19 | 0.40 |
| Uk | ng/ml | 1.0E-9 | 1.0E-9 | 6.9E-3 | 1.0E-3 | 3.3E-2 | 2.5E-3 | 1.0E-9 | 1.0E-9 | 3.2E-1 | 1.1E-2 | 115 | 19 | 115 | 19 | 0.45 |
| Um | ng/ml | 1.0E-9 | 1.0E-9 | 2.7E-1 | 1.0E-9 | 6.5E-1 | 0.0E0 | 1.0E-9 | 1.0E-9 | 3.6E0 | 1.0E-9 | 115 | 19 | 115 | 19 | 0.35 |
| Uf | ng/ml | 6.0E-2 | 9.5E-2 | 1.2E-1 | 4.6E-1 | 1.8E-1 | 1.1E0 | 2.7E-3 | 1.0E-3 | 1.1E0 | 5.1E0 | 115 | 19 | 115 | 19 | 0.59 |
| Uh | ng/ml | 2.2E0 | 3.4E0 | 3.4E0 | 4.9E0 | 3.2E0 | 4.7E0 | 3.6E-2 | 4.7E-2 | 1.5E1 | 1.8E1 | 115 | 19 | 115 | 19 | 0.59 |
| Un | ng/ml | 1.7E0 | 1.7E0 | 2.1E0 | 2.5E0 | 2.4E0 | 1.9E0 | 3.5E-1 | 3.4E-1 | 2.5E1 | 8.0E0 | 115 | 19 | 115 | 19 | 0.57 |
| Ug | ng/ml | 1.1E1 | 8.9E0 | 2.3E1 | 1.4E1 | 2.8E1 | 1.5E1 | 1.5E0 | 1.0E0 | 1.6E2 | 6.9E1 | 115 | 19 | 115 | 19 | 0.42 |
| Ur | ng/ml | 1.1E-1 | 7.5E-2 | 3.8E-1 | 2.3E-1 | 9.7E-1 | 3.4E-1 | 1.0E-9 | 1.0E-9 | 7.3E0 | 1.0E0 | 114 | 19 | 114 | 19 | 0.45 |
| Up | ng/ml | 1.0E-9 | 1.0E-4 | 2.4E-2 | 5.9E-3 | 2.2E-1 | 1.7E-2 | 1.0E-9 | 1.0E-9 | 2.4E0 | 7.4E-2 | 114 | 19 | 114 | 19 | 0.60 |
| Us | ng/ml | 2.0E-3 | 1.5E-2 | 3.1E-2 | 1.8E-2 | 1.6E-1 | 2.1E-2 | 1.0E-9 | 1.0E-9 | 1.7E0 | 6.4E-2 | 114 | 19 | 114 | 19 | 0.60 |
| Uv | ng/ml | 2.6E-3 | 3.0E-3 | 1.7E-2 | 1.4E-2 | 5.7E-2 | 3.5E-2 | 1.0E-9 | 1.0E-9 | 4.1E-1 | 1.5E-1 | 114 | 19 | 114 | 19 | 0.52 |
| Ut | ng/ml | 6.8E-1 | 1.9E0 | 2.7E0 | 5.2E0 | 8.1E0 | 7.1E0 | 1.0E-9 | 9.2E-2 | 6.5E1 | 2.4E1 | 114 | 19 | 114 | 19 | 0.65 |
| Uu | ng/ml | 7.0E0 | 6.2E0 | 7.5E0 | 6.4E0 | 5.0E0 | 5.3E0 | 5.4E-1 | 8.1E-1 | 2.9E1 | 2.3E1 | 114 | 19 | 114 | 19 | 0.42 |
| Uw | ng/ml | 2.1E0 | 4.0E0 | 3.5E0 | 3.6E0 | 5.9E0 | 2.2E0 | 1.5E-1 | 1.0E-9 | 3.9E1 | 6.4E0 | 47 | 12 | 47 | 12 | 0.61 |
| Vb | ng/ml | 1.1E0 | 1.1E0 | 1.2E0 | 9.2E-1 | 8.8E-1 | 5.1E-1 | 2.8E-1 | 8.5E-2 | 6.4E0 | 1.4E0 | 47 | 12 | 47 | 12 | 0.43 |
| Vc | ng/ml | 1.0E-9 | 1.0E-9 | 4.3E-3 | 1.1E-3 | 1.9E-2 | 3.9E-3 | 1.0E-9 | 1.0E-9 | 1.1E-1 | 1.3E-2 | 47 | 12 | 47 | 12 | 0.51 |
| Uy | ng/ml | 1.4E0 | 9.3E-1 | 8.9E0 | 3.9E0 | 2.0E1 | 1.1E1 | 8.7E-2 | 2.0E-2 | 9.9E1 | 3.8E1 | 47 | 12 | 47 | 12 | 0.36 |
| Uz | ng/ml | 1.0E-9 | 1.0E-9 | 7.1E-1 | 2.6E-2 | 4.8E0 | 9.0E-2 | 1.0E-9 | 1.0E-9 | 3.3E1 | 3.1E-1 | 47 | 12 | 47 | 12 | 0.52 |
| Ux | ng/ml | 1.8E2 | 1.4E2 | 1.9E2 | 1.7E2 | 1.3E2 | 1.6E2 | 1.2E1 | 4.5E0 | 4.8E2 | 4.9E2 | 47 | 12 | 47 | 12 | 0.43 |
| Va | ng/ml | 1.4E1 | 3.4E0 | 2.5E1 | 1.4E1 | 2.9E1 | 2.2E1 | 3.1E-1 | 3.6E-1 | 1.2E2 | 6.2E1 | 47 | 12 | 47 | 12 | 0.33 |
| Vh | ng/ml | 1.1E-2 | 2.0E-2 | 3.7E-2 | 2.1E-2 | 1.3E-1 | 1.7E-2 | 3.9E-4 | 1.0E-3 | 8.6E-1 | 5.8E-2 | 47 | 12 | 47 | 12 | 0.56 |
| Vi | ng/ml | 3.5E-3 | 1.6E-2 | 4.6E-2 | 3.2E-2 | 2.7E-1 | 4.0E-2 | 1.0E-9 | 1.5E-4 | 1.8E0 | 1.2E-1 | 47 | 12 | 47 | 12 | 0.66 |
| Vj | ng/ml | 2.7E1 | 7.5E1 | 5.1E1 | 1.4E2 | 5.5E1 | 1.9E2 | 4.6E0 | 1.4E0 | 2.5E2 | 6.5E2 | 47 | 11 | 47 | 11 | 0.67 |
| Vp | ng/ml | 1.0E-9 | 1.0E-9 | 5.5E-1 | 3.7E-1 | 4.6E0 | 1.3E0 | 1.0E-9 | 1.0E-9 | 4.9E1 | 5.6E0 | 115 | 19 | 115 | 19 | 0.59 |
| Vt | ng/ml | 6.2E0 | 5.7E0 | 9.2E0 | 9.0E0 | 1.5E1 | 9.2E0 | 9.6E-1 | 5.6E-1 | 1.6E2 | 3.8E1 | 115 | 19 | 115 | 19 | 0.48 |

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Vu | ng/ml | 1.0E-9 | 1.5E0 | 1.3E0 | 3.6E0 | 3.0E0 | 4.8E0 | 1.0E-9 | 1.0E-9 | 2.2E1 | 1.3E1 | 112 | 19 | 112 | 19 | 0.64 |
| Vq | ng/ml | 1.6E2 | 1.3E2 | 7.0E2 | 8.7E2 | 1.6E3 | 1.5E3 | 9.2E-1 | 6.5E-1 | 1.2E4 | 4.9E3 | 91 | 17 | 91 | 17 | 0.54 |
| Vo | ng/ml | 2.5E1 | 2.5E1 | 2.5E1 | 2.3E1 | 5.3E0 | 7.2E0 | 4.9E0 | 1.9E0 | 4.8E1 | 3.1E1 | 115 | 19 | 115 | 19 | 0.47 |
| Vs | ng/ml | 1.0E-9 | 4.7E-1 | 7.9E0 | 7.8E0 | 4.4E1 | 1.3E1 | 1.0E-9 | 1.0E-9 | 4.5E2 | 4.9E1 | 113 | 19 | 113 | 19 | 0.57 |
| Vv | ng/ml | 2.9E0 | 2.8E0 | 5.4E0 | 1.0E1 | 9.4E0 | 1.3E1 | 1.0E-9 | 1.0E-9 | 8.4E1 | 4.4E1 | 114 | 19 | 114 | 19 | 0.57 |
| Vw | ng/ml | 3.6E1 | 4.4E1 | 3.4E1 | 3.8E1 | 1.7E1 | 2.5E1 | 3.1E0 | 2.5E0 | 6.7E1 | 6.9E1 | 47 | 12 | 47 | 12 | 0.57 |
| Oy | pg/ml | 4.8E-1 | 2.7E-1 | 6.3E0 | 3.9E0 | 3.2E1 | 9.6E0 | 1.0E-9 | 1.0E-9 | 4.0E2 | 4.0E1 | 319 | 31 | 319 | 31 | 0.48 |
| Oz | pg/ml | 2.5E-3 | 3.3E-2 | 3.3E-1 | 1.2E0 | 1.6E0 | 5.0E0 | 1.0E-9 | 1.0E-9 | 2.9E1 | 2.8E1 | 319 | 31 | 319 | 31 | 0.53 |
| Pa | pg/ml | 3.9E-1 | 4.2E-1 | 1.7E0 | 9.5E0 | 8.1E0 | 4.1E1 | 1.0E-9 | 1.0E-9 | 1.0E2 | 2.3E2 | 319 | 31 | 319 | 31 | 0.55 |
| Pb | pg/ml | 1.0E-9 | 1.0E-9 | 1.8E0 | 1.4E-1 | 2.7E1 | 3.5E-1 | 1.0E-9 | 1.0E-9 | 4.9E2 | 1.9E0 | 319 | 31 | 319 | 31 | 0.52 |
| Pc | pg/ml | 2.0E-2 | 3.0E-1 | 3.7E-1 | 1.1E1 | 9.1E-1 | 5.9E1 | 1.0E-9 | 1.0E-9 | 1.4E1 | 3.3E2 | 319 | 31 | 319 | 31 | 0.57 |
| Pd | pg/ml | 1.6E0 | 1.5E0 | 6.8E0 | 7.4E0 | 4.8E1 | 1.3E1 | 1.0E-9 | 1.0E-9 | 8.4E2 | 5.5E1 | 319 | 31 | 319 | 31 | 0.53 |
| Pe | pg/ml | 2.1E1 | 4.7E1 | 1.4E2 | 6.9E2 | 6.4E2 | 2.7E3 | 1.0E-9 | 3.8E-1 | 6.7E3 | 1.5E4 | 319 | 31 | 319 | 31 | 0.62 |
| Pf | pg/ml | 1.7E0 | 2.4E0 | 1.5E1 | 3.1E1 | 9.3E1 | 8.1E1 | 1.0E-9 | 1.0E-9 | 1.5E3 | 4.3E2 | 319 | 31 | 319 | 31 | 0.57 |
| Pg | pg/ml | 3.8E0 | 9.3E0 | 7.4E1 | 1.5E2 | 5.4E2 | 3.3E2 | 1.0E-9 | 8.4E-1 | 7.7E3 | 1.3E3 | 319 | 31 | 319 | 31 | 0.68 |
| Ph | ng/ml | 1.5E-1 | 1.7E-1 | 3.7E-1 | 4.4E-1 | 6.6E-1 | 6.7E-1 | 1.0E-9 | 1.0E-9 | 5.4E0 | 2.8E0 | 117 | 18 | 117 | 18 | 0.52 |
| Pi | ng/ml | 1.9E-1 | 3.0E-1 | 9.8E-1 | 6.3E-1 | 7.6E0 | 1.1E0 | 1.0E-9 | 1.0E-9 | 8.2E1 | 4.8E0 | 117 | 18 | 117 | 18 | 0.64 |
| Pj | ng/mL | 5.6E0 | 4.4E0 | 6.2E0 | 5.5E0 | 4.3E0 | 5.3E0 | 4.9E-1 | 4.0E-1 | 3.1E1 | 2.3E1 | 117 | 18 | 117 | 18 | 0.41 |
| Pk | ng/ml | 9.0E-3 | 1.4E-2 | 2.7E-2 | 2.0E-2 | 1.4E-1 | 2.6E-2 | 1.0E-9 | 1.0E-9 | 1.5E0 | 1.1E-1 | 117 | 18 | 117 | 18 | 0.58 |
| aA | mg/dL | 9.0E-1 | 1.2E0 | 1.0E0 | 1.6E0 | 5.1E-1 | 1.0E0 | 3.0E-1 | 4.0E-1 | 4.2E0 | 4.7E0 | 487 | 46 | 487 | 46 | 0.72 |
| aC | mg/mL | 2.3E0 | 1.9E0 | 2.7E0 | 2.5E0 | 1.3E0 | 1.6E0 | 7.5E-1 | 9.7E-1 | 7.4E0 | 6.7E0 | 152 | 22 | 152 | 22 | 0.40 |
| aD | ug/mL | 2.9E0 | 3.5E0 | 4.7E0 | 4.1E0 | 4.9E0 | 2.4E0 | 7.5E-1 | 1.6E0 | 3.5E1 | 9.8E0 | 152 | 22 | 152 | 22 | 0.52 |
| aE | mg/mL | 5.7E-1 | 5.7E-1 | 5.9E-1 | 5.9E-1 | 1.8E-1 | 1.4E-1 | 1.8E-1 | 4.1E-1 | 1.2E0 | 1.0E0 | 152 | 22 | 152 | 22 | 0.49 |
| aF | ng/mL | 2.2E0 | 2.7E0 | 4.7E0 | 7.6E0 | 7.3E0 | 9.6E0 | 4.3E-3 | 4.3E-3 | 5.0E1 | 3.5E1 | 152 | 22 | 152 | 22 | 0.58 |
| aG | mg/mL | 1.4E-1 | 1.1E-1 | 1.6E-1 | 1.4E-1 | 8.8E-2 | 7.0E-2 | 3.2E-2 | 6.9E-2 | 4.8E-1 | 3.5E-1 | 152 | 22 | 152 | 22 | 0.43 |
| aH | ug/mL | 7.3E1 | 6.2E1 | 7.7E1 | 7.6E1 | 4.0E1 | 4.1E1 | 8.9E0 | 2.3E1 | 2.0E2 | 2.0E2 | 152 | 22 | 152 | 22 | 0.49 |
| aI | ug/mL | 1.7E2 | 1.5E2 | 1.8E2 | 1.6E2 | 6.2E1 | 5.4E1 | 3.2E1 | 7.5E1 | 3.4E2 | 2.9E2 | 152 | 22 | 152 | 22 | 0.40 |
| aJ | ug/mL | 2.3E0 | 4.0E0 | 3.1E0 | 4.9E0 | 2.2E0 | 4.5E0 | 8.2E-1 | 1.5E0 | 1.4E1 | 2.3E1 | 152 | 22 | 152 | 22 | 0.68 |
| aK | ng/mL | 1.3E0 | 1.3E0 | 2.0E0 | 1.5E0 | 2.0E0 | 1.4E0 | 2.9E-4 | 1.3E-1 | 1.0E1 | 6.5E0 | 152 | 22 | 152 | 22 | 0.45 |
| aL | mg/mL | 7.4E-1 | 7.2E-1 | 7.8E-1 | 6.7E-1 | 2.6E-1 | 2.1E-1 | 2.2E-1 | 3.2E-1 | 1.7E0 | 1.2E0 | 152 | 22 | 152 | 22 | 0.39 |
| aM | U/mL | 1.7E1 | 2.8E1 | 4.0E1 | 8.4E1 | 8.1E1 | 1.5E2 | 4.2E-2 | 4.2E-2 | 8.2E2 | 6.8E2 | 152 | 22 | 152 | 22 | 0.66 |
| aN | U/mL | 1.4E1 | 1.9E1 | 2.5E1 | 2.8E1 | 4.4E1 | 3.0E1 | 2.5E-3 | 4.8E0 | 3.8E2 | 1.3E2 | 152 | 22 | 152 | 22 | 0.59 |
| aO | pg/mL | 4.3E1 | 1.8E2 | 4.0E2 | 6.4E2 | 9.9E2 | 9.0E2 | 6.0E-2 | 2.1E0 | 6.6E3 | 3.3E3 | 152 | 22 | 152 | 22 | 0.68 |
| aP | ng/mL | 1.6E0 | 2.8E0 | 2.1E0 | 4.0E0 | 2.5E0 | 5.6E0 | 4.5E-1 | 1.1E0 | 2.8E1 | 2.8E1 | 152 | 22 | 152 | 22 | 0.70 |
| aQ | ng/mL | 2.4E-1 | 2.4E-1 | 3.6E-1 | 3.2E-1 | 3.3E-1 | 2.4E-1 | 2.0E-4 | 5.2E-2 | 2.0E0 | 9.2E-1 | 152 | 22 | 152 | 22 | 0.49 |
| aR | ng/mL | 1.7E0 | 2.8E0 | 2.8E0 | 4.2E0 | 4.0E0 | 4.0E0 | 2.6E-1 | 5.6E-1 | 3.4E1 | 1.5E1 | 152 | 22 | 152 | 22 | 0.65 |
| aS | ng/mL | 3.7E-1 | 6.0E-1 | 1.0E0 | 1.0E0 | 2.8E0 | 1.2E0 | 4.2E-3 | 2.8E-2 | 3.3E1 | 4.9E0 | 152 | 22 | 152 | 22 | 0.59 |
| aU | pg/mL | 6.8E1 | 4.2E1 | 1.0E2 | 7.7E1 | 1.1E2 | 1.1E2 | 7.4E-2 | 7.4E-2 | 7.0E2 | 5.1E2 | 152 | 22 | 152 | 22 | 0.39 |
| aV | ng/mL | 6.0E-1 | 4.7E-1 | 1.1E0 | 6.6E-1 | 2.8E0 | 5.6E-1 | 7.6E-4 | 1.5E-1 | 3.3E1 | 2.4E0 | 152 | 22 | 152 | 22 | 0.46 |
| aW | pg/mL | 1.9E1 | 2.1E1 | 2.3E1 | 2.0E1 | 3.6E1 | 1.2E1 | 7.2E-2 | 7.2E-2 | 4.2E2 | 4.7E1 | 152 | 22 | 152 | 22 | 0.52 |
| aX | ng/mL | 7.8E0 | 1.3E1 | 1.3E1 | 3.6E1 | 2.1E1 | 7.0E1 | 3.0E-1 | 1.7E0 | 2.2E2 | 3.1E2 | 152 | 22 | 152 | 22 | 0.56 |
| aY | pg/mL | 5.2E1 | 6.3E1 | 7.5E1 | 8.7E1 | 1.1E2 | 8.2E1 | 4.1E-1 | 1.2E1 | 1.2E3 | 3.9E2 | 152 | 22 | 152 | 22 | 0.59 |
| aZ | pg/mL | 2.2E2 | 4.5E2 | 5.0E2 | 1.9E3 | 1.1E3 | 2.6E3 | 1.7E0 | 1.5E1 | 1.2E4 | 7.9E3 | 152 | 22 | 152 | 22 | 0.67 |
| bA | ng/mL | 1.3E1 | 1.0E2 | 6.1E1 | 1.7E2 | 1.5E2 | 3.1E2 | 3.0E-2 | 3.0E-2 | 9.4E2 | 1.5E3 | 152 | 22 | 152 | 22 | 0.69 |
| bB | ng/mL | 2.8E2 | 2.3E2 | 3.1E2 | 2.8E2 | 1.8E2 | 1.9E2 | 2.1E0 | 6.5E1 | 9.5E2 | 7.4E2 | 152 | 22 | 152 | 22 | 0.43 |
| bC | ng/mL | 3.2E2 | 3.8E2 | 5.8E2 | 1.1E3 | 7.7E2 | 1.4E3 | 1.4E1 | 5.0E1 | 4.7E3 | 4.0E3 | 152 | 22 | 152 | 22 | 0.58 |
| bE | mg/mL | 5.2E0 | 4.8E0 | 5.5E0 | 5.4E0 | 2.1E0 | 2.5E0 | 1.3E0 | 2.6E0 | 1.2E1 | 1.2E1 | 152 | 22 | 152 | 22 | 0.46 |
| bF | pg/mL | 3.4E1 | 5.5E1 | 3.3E2 | 3.9E2 | 1.4E3 | 1.3E3 | 5.0E-2 | 1.1E1 | 1.1E4 | 6.3E3 | 152 | 22 | 152 | 22 | 0.60 |
| bG | ng/mL | 1.6E0 | 1.7E0 | 3.0E0 | 4.3E0 | 4.0E0 | 6.9E0 | 1.1E-1 | 1.6E-1 | 2.6E1 | 3.0E1 | 152 | 22 | 152 | 22 | 0.51 |
| bH | pg/mL | 5.7E-1 | 5.7E-1 | 6.6E0 | 4.6E0 | 2.5E1 | 6.5E0 | 5.7E-1 | 5.7E-1 | 2.8E2 | 2.3E1 | 152 | 22 | 152 | 22 | 0.51 |
| bI | ng/mL | 4.0E-3 | 4.0E-3 | 9.1E-2 | 1.1E-1 | 1.8E-1 | 2.7E-1 | 4.0E-3 | 4.0E-3 | 8.8E-1 | 9.8E-1 | 152 | 22 | 152 | 22 | 0.49 |
| bJ | mg/mL | 1.9E0 | 2.0E0 | 2.4E0 | 2.4E0 | 2.0E0 | 2.1E0 | 2.5E-4 | 2.5E-4 | 1.1E1 | 8.9E0 | 152 | 22 | 152 | 22 | 0.51 |
| bL | pg/mL | 3.7E0 | 5.6E0 | 9.5E0 | 7.9E0 | 1.2E1 | 7.1E0 | 4.6E-2 | 4.6E-2 | 6.0E1 | 2.4E1 | 152 | 22 | 152 | 22 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bM | mg/mL | 1.8E0 | 1.9E0 | 2.1E0 | 2.2E0 | 1.4E0 | 1.9E0 | 1.8E-2 | 1.6E-2 | 7.9E0 | 8.6E0 | 152 | 22 | 152 | 22 | 0.50 |
| bN | ng/mL | 3.4E1 | 2.6E1 | 1.3E2 | 5.7E1 | 3.0E2 | 7.4E1 | 1.4E-1 | 1.4E-1 | 1.9E3 | 2.7E2 | 152 | 22 | 152 | 22 | 0.44 |
| bO | ng/mL | 4.0E-2 | 4.0E-2 | 9.7E0 | 5.5E0 | 1.9E1 | 2.5E1 | 4.0E-2 | 4.0E-2 | 1.3E2 | 1.2E2 | 152 | 22 | 152 | 22 | 0.36 |
| bP | mg/mL | 5.2E-1 | 5.1E-1 | 7.4E-1 | 7.6E-1 | 7.1E-1 | 6.9E-1 | 4.9E-2 | 9.2E-2 | 4.8E0 | 2.7E0 | 152 | 22 | 152 | 22 | 0.51 |
| bQ | pg/mL | 2.1E1 | 3.1E1 | 1.5E2 | 6.1E1 | 1.1E3 | 5.7E1 | 1.5E-1 | 8.1E0 | 1.3E4 | 1.9E2 | 152 | 22 | 152 | 22 | 0.63 |
| bR | ng/mL | 1.2E-2 | 1.2E-2 | 1.7E-1 | 7.1E-2 | 7.6E-1 | 1.2E-1 | 1.2E-2 | 1.2E-2 | 8.7E0 | 3.8E-1 | 152 | 22 | 152 | 22 | 0.44 |
| bS | pg/mL | 9.4E-1 | 9.4E-1 | 9.8E0 | 8.2E0 | 4.3E1 | 2.0E1 | 9.4E-1 | 9.4E-1 | 3.9E2 | 7.2E1 | 152 | 22 | 152 | 22 | 0.51 |
| bU | ng/mL | 7.0E-2 | 1.5E-1 | 2.0E-1 | 1.3E-1 | 5.7E-1 | 1.2E-1 | 1.3E-2 | 1.3E-2 | 6.6E0 | 4.1E-1 | 152 | 22 | 152 | 22 | 0.49 |
| bV | pg/mL | 4.7E2 | 6.5E2 | 6.1E2 | 7.8E2 | 9.3E2 | 4.9E2 | 1.6E2 | 3.4E2 | 1.2E4 | 2.2E3 | 152 | 22 | 152 | 22 | 0.69 |
| bW | pg/mL | 3.2E2 | 3.3E2 | 4.8E2 | 7.3E2 | 5.2E2 | 1.1E3 | 8.4E1 | 1.1E2 | 4.8E3 | 3.9E3 | 152 | 22 | 152 | 22 | 0.52 |
| bX | ng/mL | 7.0E-4 | 2.5E-5 | 2.7E-3 | 2.3E-3 | 3.2E-3 | 2.8E-3 | 2.5E-5 | 2.5E-5 | 1.3E-2 | 7.2E-3 | 152 | 22 | 152 | 22 | 0.48 |
| bZ | pg/mL | 2.7E2 | 7.2E2 | 1.8E3 | 3.3E3 | 6.8E3 | 9.0E3 | 1.5E-1 | 1.5E-1 | 5.8E4 | 4.3E4 | 152 | 22 | 152 | 22 | 0.61 |
| cA | pg/mL | 6.0E-1 | 6.0E-1 | 4.4E0 | 1.8E0 | 3.0E1 | 4.5E0 | 6.0E-1 | 6.0E-1 | 3.7E2 | 2.1E1 | 152 | 22 | 152 | 22 | 0.48 |
| cB | ng/mL | 5.3E-2 | 2.9E-2 | 7.9E-2 | 4.6E-2 | 9.0E-2 | 6.5E-2 | 1.7E-3 | 1.7E-3 | 4.3E-1 | 2.6E-1 | 152 | 22 | 152 | 22 | 0.37 |
| cC | pg/mL | 4.4E1 | 3.6E1 | 4.7E1 | 3.2E1 | 5.2E1 | 2.5E1 | 1.0E0 | 1.0E0 | 4.5E2 | 7.7E1 | 152 | 22 | 152 | 22 | 0.41 |
| cD | pg/mL | 4.9E0 | 5.4E0 | 1.3E1 | 5.9E0 | 4.8E1 | 6.1E0 | 3.3E-1 | 3.3E-1 | 4.8E2 | 2.3E1 | 152 | 22 | 152 | 22 | 0.48 |
| cE | pg/mL | 5.6E1 | 7.9E1 | 2.8E2 | 1.7E2 | 6.3E2 | 2.7E2 | 1.2E-1 | 6.1E0 | 3.8E3 | 1.3E3 | 152 | 22 | 152 | 22 | 0.57 |
| cF | pg/mL | 9.4E0 | 5.3E-1 | 1.7E1 | 5.9E0 | 3.1E1 | 1.0E1 | 5.3E-1 | 5.3E-1 | 2.7E2 | 3.8E1 | 152 | 22 | 152 | 22 | 0.36 |
| cG | pg/mL | 5.3E1 | 1.2E2 | 1.7E2 | 1.9E2 | 8.5E2 | 2.3E2 | 7.8E0 | 2.4E1 | 1.0E4 | 1.1E3 | 152 | 22 | 152 | 22 | 0.67 |
| cII | uIU/mL | 3.2E0 | 5.0E0 | 6.8E0 | 1.4E1 | 1.5E1 | 2.7E1 | 8.6E-3 | 8.6E-3 | 1.6E2 | 1.2E2 | 152 | 22 | 152 | 22 | 0.53 |
| cI | ng/mL | 6.0E0 | 6.7E0 | 1.5E1 | 9.2E0 | 2.2E1 | 1.1E1 | 3.2E-2 | 2.3E-1 | 1.2E2 | 4.1E1 | 152 | 22 | 152 | 22 | 0.45 |
| cJ | ug/mL | 6.7E1 | 3.7E1 | 1.0E2 | 7.7E1 | 1.0E2 | 8.8E1 | 6.9E0 | 5.6E0 | 6.4E2 | 3.4E2 | 152 | 22 | 152 | 22 | 0.41 |
| cK | ng/mL | 3.8E-3 | 3.8E-3 | 2.4E-2 | 1.5E-2 | 1.2E-1 | 4.4E-2 | 3.8E-3 | 3.8E-3 | 1.5E0 | 2.1E-1 | 152 | 22 | 152 | 22 | 0.49 |
| cL | pg/mL | 2.1E2 | 2.2E2 | 5.0E2 | 8.2E2 | 2.0E3 | 1.6E3 | 3.1E1 | 6.7E1 | 2.4E4 | 7.4E3 | 152 | 22 | 152 | 22 | 0.58 |
| cM | pg/mL | 2.7E2 | 2.8E2 | 2.9E2 | 2.7E2 | 1.7E2 | 9.1E1 | 2.5E1 | 1.2E2 | 1.1E3 | 4.4E2 | 152 | 22 | 152 | 22 | 0.50 |
| cN | pg/mL | 1.2E2 | 1.3E2 | 1.3E2 | 1.4E2 | 9.0E1 | 3.3E1 | 3.8E1 | 8.6E1 | 1.1E3 | 2.1E2 | 152 | 22 | 152 | 22 | 0.61 |
| cO | pg/mL | 2.1E2 | 2.5E2 | 4.2E2 | 3.5E2 | 1.6E3 | 3.2E2 | 5.4E1 | 8.2E1 | 1.9E4 | 1.5E3 | 152 | 22 | 152 | 22 | 0.55 |
| cP | ng/mL | 2.4E3 | 2.8E3 | 2.5E3 | 2.8E3 | 9.4E2 | 1.0E3 | 6.2E2 | 1.4E3 | 5.6E3 | 4.7E3 | 152 | 22 | 152 | 22 | 0.57 |
| cQ | ng/mL | 5.0E-2 | 1.0E-1 | 1.3E-1 | 1.4E-1 | 2.2E-1 | 1.5E-1 | 2.0E-3 | 2.0E-3 | 1.3E0 | 4.8E-1 | 152 | 22 | 152 | 22 | 0.58 |
| cR | ng/mL | 3.4E2 | 3.5E2 | 6.1E2 | 5.4E2 | 8.6E2 | 5.9E2 | 2.0E1 | 8.9E1 | 7.7E3 | 2.2E3 | 152 | 22 | 152 | 22 | 0.49 |
| cS | ng/mL | 2.7E2 | 3.3E2 | 4.1E2 | 9.6E2 | 4.3E2 | 1.6E3 | 4.1E1 | 1.2E2 | 2.5E3 | 7.1E3 | 152 | 22 | 152 | 22 | 0.64 |
| cT | ng/mL | 4.9E1 | 1.2E2 | 1.4E2 | 3.3E2 | 2.7E2 | 4.7E2 | 3.6E0 | 1.4E1 | 2.1E3 | 1.5E3 | 152 | 22 | 152 | 22 | 0.64 |
| cU | ng/mL | 5.6E1 | 1.5E2 | 9.1E1 | 1.6E2 | 1.5E2 | 9.3E1 | 6.2E0 | 1.8E1 | 1.6E3 | 3.9E2 | 152 | 22 | 152 | 22 | 0.77 |
| cV | ng/mL | 2.1E-1 | 1.8E-1 | 7.3E-1 | 7.8E-1 | 3.9E0 | 2.1E0 | 2.5E-2 | 3.4E-2 | 4.7E1 | 9.7E0 | 152 | 22 | 152 | 22 | 0.48 |
| cW | mIU/mL | 4.8E-2 | 5.2E-2 | 9.1E-2 | 8.0E-2 | 3.6E-1 | 6.4E-2 | 4.8E-3 | 2.2E-2 | 4.5E0 | 2.9E-1 | 152 | 22 | 152 | 22 | 0.59 |
| cX | ng/mL | 1.1E-1 | 1.1E-1 | 1.7E0 | 2.9E0 | 5.2E0 | 8.2E0 | 2.3E-4 | 9.1E-3 | 2.8E1 | 2.8E1 | 152 | 22 | 152 | 22 | 0.52 |
| cY | ng/mL | 7.5E0 | 6.5E0 | 1.1E1 | 8.2E0 | 1.1E1 | 7.9E0 | 1.7E-1 | 6.0E-1 | 6.1E1 | 3.6E1 | 152 | 22 | 152 | 22 | 0.44 |
| cZ | ug/mL | 1.3E1 | 1.2E1 | 1.5E1 | 1.4E1 | 6.8E0 | 6.9E0 | 2.3E0 | 6.2E0 | 4.6E1 | 3.0E1 | 152 | 22 | 152 | 22 | 0.45 |
| dA | pg/mL | 3.2E2 | 3.0E2 | 3.9E2 | 3.8E2 | 4.8E2 | 1.9E2 | 1.0E2 | 1.7E2 | 5.8E3 | 8.8E2 | 152 | 22 | 152 | 22 | 0.51 |
| dB | ug/mL | 1.8E1 | 2.1E1 | 1.8E1 | 1.9E1 | 2.1E1 | 7.8E0 | 2.1E0 | 2.9E0 | 2.5E2 | 2.9E1 | 152 | 22 | 152 | 22 | 0.60 |
| dC | nmol/L | 3.4E1 | 3.0E1 | 3.7E1 | 3.6E1 | 1.7E1 | 1.7E1 | 7.8E0 | 1.5E1 | 1.4E2 | 9.1E1 | 152 | 22 | 152 | 22 | 0.44 |
| dD | ug/mL | 3.4E1 | 2.9E1 | 3.6E1 | 3.2E1 | 1.1E1 | 8.8E0 | 1.4E1 | 2.0E1 | 7.4E1 | 5.2E1 | 152 | 22 | 152 | 22 | 0.40 |
| dE | ng/mL | 4.7E-1 | 3.8E-1 | 5.6E-1 | 6.4E-1 | 5.6E-1 | 7.7E-1 | 8.4E-3 | 8.4E-3 | 2.7E0 | 2.9E0 | 152 | 22 | 152 | 22 | 0.50 |
| dF | ng/mL | 2.4E2 | 3.5E2 | 3.3E2 | 4.6E2 | 2.4E2 | 2.8E2 | 7.5E1 | 1.3E2 | 1.3E3 | 1.2E3 | 152 | 22 | 152 | 22 | 0.67 |
| dG | ng/mL | 1.1E1 | 1.5E1 | 1.7E1 | 1.9E1 | 1.9E1 | 1.3E1 | 3.0E0 | 6.7E0 | 1.8E2 | 6.5E1 | 152 | 22 | 152 | 22 | 0.62 |
| dH | pg/mL | 8.0E0 | 8.7E0 | 2.2E1 | 1.3E1 | 6.6E1 | 1.6E1 | 4.0E-2 | 8.3E-1 | 6.7E2 | 7.6E1 | 152 | 22 | 152 | 22 | 0.53 |
| dI | pg/mL | 4.6E-1 | 4.6E-1 | 4.0E0 | 2.4E0 | 2.7E1 | 4.5E0 | 4.6E-1 | 4.6E-1 | 3.3E2 | 1.9E1 | 152 | 22 | 152 | 22 | 0.50 |
| dJ | ng/mL | 2.0E0 | 1.8E0 | 2.2E0 | 2.0E0 | 1.1E0 | 1.1E0 | 3.2E-2 | 3.7E-1 | 5.6E0 | 4.5E0 | 152 | 22 | 152 | 22 | 0.44 |
| dK | uIU/mL | 1.4E0 | 8.1E-1 | 2.2E0 | 1.6E0 | 3.7E0 | 1.9E0 | 2.8E-4 | 1.4E-2 | 3.9E1 | 7.3E0 | 152 | 22 | 152 | 22 | 0.41 |
| dL | ng/mL | 8.7E2 | 1.2E3 | 1.0E3 | 1.2E3 | 6.5E2 | 4.5E2 | 2.8E2 | 5.8E2 | 4.8E3 | 2.3E3 | 152 | 22 | 152 | 22 | 0.63 |
| dM | pg/mL | 9.6E2 | 1.2E3 | 1.3E3 | 1.8E3 | 1.5E3 | 1.4E3 | 3.7E2 | 5.2E2 | 1.5E4 | 5.8E3 | 152 | 22 | 152 | 22 | 0.61 |
| dN | ug/mL | 9.8E1 | 1.1E2 | 1.0E2 | 1.1E2 | 4.5E1 | 3.1E1 | 2.4E1 | 6.4E1 | 3.3E2 | 1.7E2 | 152 | 22 | 152 | 22 | 0.59 |
| dR | pg/ml | 1.5E3 | 9.3E2 | 2.1E3 | 1.4E3 | 2.1E3 | 1.4E3 | 1.4E2 | 1.3E2 | 9.8E3 | 5.5E3 | 114 | 17 | 114 | 17 | 0.39 |

| Code | Units | Median | | Average | | Standard Deviation | | Minimum | | Maximum | | Number of Samples | | Number of Patients | | AUC |
|------|-------|--------|--------|---------|--------|----------|--------|---------|--------|---------|--------|--------|-----|---------|-----|------|
| | | NonDis | Dis | NonDis | Dis | NonDis | Dis | NonDis | Dis | NonDis | Dis | NonDis | Dis | NonDis | Dis | |
| dU | pg/ml | 8.5E3 | 1.3E4 | 1.6E4 | 1.3E4 | 1.8E4 | 1.1E4 | 6.9E2 | 1.7E3 | 8.1E4 | 3.5E4 | 25 | 8 | 25 | 8 | 0.49 |
| dX | ng/ml | 8.1E-2 | 8.2E-2 | 1.3E-1 | 1.2E-1 | 2.0E-1 | 1.3E-1 | 2.6E-3 | 2.6E-3 | 1.2E0 | 4.2E-1 | 51 | 10 | 51 | 10 | 0.53 |
| eF | ng/ml | 4.1E0 | 4.7E0 | 5.2E0 | 6.6E0 | 4.5E0 | 6.2E0 | 2.0E0 | 2.0E0 | 4.6E1 | 2.9E1 | 114 | 17 | 114 | 17 | 0.60 |
| eC | pg/ml | 3.0E2 | 2.7E2 | 3.7E2 | 3.1E2 | 3.1E2 | 1.9E2 | 9.9E0 | 1.1E2 | 2.0E3 | 7.3E2 | 101 | 14 | 101 | 14 | 0.44 |
| eD | pg/ml | 2.2E2 | 4.9E2 | 5.8E2 | 2.0E3 | 1.4E3 | 2.5E3 | 5.2E-1 | 8.3E1 | 8.3E3 | 7.0E3 | 84 | 9 | 84 | 9 | 0.70 |
| eM | ng/ml | 2.7E0 | 3.2E0 | 4.8E0 | 5.8E0 | 5.6E0 | 1.0E1 | 6.9E-1 | 7.6E-1 | 2.7E1 | 3.9E1 | 66 | 13 | 66 | 13 | 0.51 |
| eP | ng/ml | 3.7E-3 | 3.7E-3 | 1.1E0 | 1.5E0 | 4.1E0 | 3.2E0 | 3.7E-3 | 3.7E-3 | 2.8E1 | 1.0E1 | 51 | 10 | 51 | 10 | 0.53 |
| eQ | pg/ml | 1.0E0 | 1.0E0 | 5.5E1 | 4.7E1 | 1.1E2 | 8.8E1 | 1.0E0 | 1.0E0 | 4.7E2 | 2.2E2 | 25 | 8 | 25 | 8 | 0.47 |
| fA | ng/ml | 1.9E2 | 2.4E2 | 4.0E2 | 5.6E2 | 4.7E2 | 5.0E2 | 3.9E1 | 7.5E1 | 1.5E3 | 1.3E3 | 24 | 7 | 24 | 7 | 0.63 |
| fB | ng/ml | 5.7E2 | 6.3E2 | 6.6E2 | 6.4E2 | 2.8E2 | 2.2E2 | 2.6E2 | 3.5E2 | 1.3E3 | 9.2E2 | 23 | 8 | 23 | 8 | 0.50 |
| fP | ng/ml | 2.7E2 | 2.7E2 | 3.3E2 | 2.7E2 | 1.9E2 | 1.4E2 | 3.7E1 | 1.8E0 | 1.0E3 | 5.6E2 | 109 | 16 | 109 | 16 | 0.44 |
| fR | ng/ml | 1.4E5 | 2.2E5 | 2.1E5 | 2.7E5 | 1.8E5 | 2.1E5 | 3.6E4 | 1.9E2 | 6.9E5 | 8.7E5 | 97 | 18 | 97 | 18 | 0.61 |
| gC | ng/ml | 2.3E2 | 2.8E2 | 2.6E2 | 2.9E2 | 1.1E2 | 1.6E2 | 8.3E1 | 9.7E1 | 6.4E2 | 5.9E2 | 38 | 9 | 38 | 9 | 0.57 |
| gL | pg/ml | 6.5E4 | 6.6E4 | 7.2E4 | 8.7E4 | 3.4E4 | 5.1E4 | 1.1E4 | 2.7E4 | 1.9E5 | 2.2E5 | 114 | 17 | 114 | 17 | 0.57 |
| gP | U/ml | 2.7E2 | 2.8E2 | 2.9E2 | 2.9E2 | 1.3E2 | 9.2E1 | 1.2E1 | 6.5E1 | 1.1E3 | 4.4E2 | 113 | 17 | 113 | 17 | 0.57 |
| gW | ng/ml | 5.4E2 | 5.5E2 | 9.6E2 | 5.7E2 | 1.1E3 | 2.5E2 | 2.3E0 | 2.6E2 | 6.1E3 | 9.6E2 | 89 | 8 | 89 | 8 | 0.49 |
| gV | ng/ml | 1.9E1 | 2.2E1 | 2.0E1 | 2.6E1 | 7.4E0 | 5.5E0 | 8.1E-2 | 2.1E1 | 3.7E1 | 3.4E1 | 31 | 7 | 31 | 7 | 0.72 |
| tF | pg/mL | 1.0E3 | 5.3E3 | 9.5E3 | 3.0E4 | 3.4E4 | 6.5E4 | 1.2E1 | 1.8E1 | 2.8E5 | 2.5E5 | 102 | 15 | 102 | 15 | 0.71 |
| gZ | ug/ml | 7.0E-1 | 1.2E0 | 4.2E1 | 3.7E1 | 1.2E2 | 8.3E1 | 8.7E-2 | 1.4E-1 | 4.1E2 | 2.4E2 | 25 | 8 | 25 | 8 | 0.60 |
| hA | ng/ml | 2.3E0 | 5.4E0 | 1.4E1 | 1.7E1 | 5.2E1 | 3.4E1 | 1.7E-2 | 1.8E0 | 3.5E2 | 1.1E2 | 84 | 10 | 84 | 10 | 0.75 |
| nM | pg/ml | 1.0E-9 | 1.0E-9 | 1.0E-9 | 1.2E2 | 0.0E0 | 4.2E2 | 1.0E-9 | 1.0E-9 | 1.0E-9 | 1.5E3 | 61 | 13 | 61 | 13 | 0.54 |
| nN | pg/ml | 1.3E3 | 2.4E3 | 2.5E3 | 2.5E4 | 3.3E3 | 4.8E4 | 8.1E1 | 5.7E2 | 2.1E4 | 1.5E5 | 61 | 13 | 61 | 13 | 0.66 |
| nO | pg/ml | 2.4E1 | 4.3E1 | 3.7E1 | 4.3E1 | 4.8E1 | 3.6E1 | 4.0E0 | 9.7E0 | 3.1E2 | 1.5E2 | 61 | 13 | 61 | 13 | 0.64 |
| nR | pg/ml | 1.5E1 | 6.0E1 | 4.1E1 | 3.2E2 | 6.7E1 | 5.5E2 | 1.0E0 | 3.5E0 | 3.1E2 | 1.9E3 | 61 | 13 | 61 | 13 | 0.79 |
| nT | pg/ml | 7.2E1 | 1.1E2 | 9.7E1 | 2.2E2 | 9.0E1 | 2.7E2 | 1.0E-9 | 2.7E1 | 4.9E2 | 9.2E2 | 61 | 13 | 61 | 13 | 0.65 |
| nU | pg/ml | 3.9E1 | 8.9E1 | 6.2E1 | 3.0E2 | 1.1E2 | 4.4E2 | 1.0E-9 | 1.0E-9 | 7.5E2 | 1.5E3 | 61 | 13 | 61 | 13 | 0.69 |
| lW | pg/ml | 1.0E-9 | 1.0E-9 | 1.1E1 | 1.2E1 | 3.1E1 | 2.2E1 | 1.0E-9 | 1.0E-9 | 1.7E2 | 7.0E1 | 61 | 13 | 61 | 13 | 0.57 |
| lX | pg/ml | 9.0E2 | 9.2E2 | 9.9E2 | 1.0E3 | 5.8E2 | 5.7E2 | 1.9E2 | 4.8E2 | 2.6E3 | 2.5E3 | 61 | 13 | 61 | 13 | 0.54 |
| lY | pg/ml | 2.0E1 | 1.2E1 | 2.1E1 | 1.6E1 | 1.8E1 | 1.4E1 | 1.0E-9 | 1.0E-9 | 1.2E2 | 4.5E1 | 61 | 13 | 61 | 13 | 0.39 |
| mE | pg/ml | 1.0E-9 | 1.0E-9 | 2.8E0 | 1.4E0 | 8.1E0 | 3.1E0 | 1.0E-9 | 1.0E-9 | 5.7E1 | 9.2E0 | 61 | 13 | 61 | 13 | 0.42 |
| mF | pg/ml | 2.7E-1 | 7.1E-1 | 7.4E0 | 2.8E0 | 3.4E1 | 4.5E0 | 1.0E-9 | 1.0E-9 | 2.5E2 | 1.3E1 | 61 | 13 | 61 | 13 | 0.57 |
| mH | pg/ml | 3.1E0 | 3.3E0 | 5.2E0 | 6.6E0 | 7.7E0 | 9.1E0 | 4.0E-1 | 8.3E-1 | 5.3E1 | 3.2E1 | 61 | 13 | 61 | 13 | 0.51 |
| mI | pg/ml | 1.0E-9 | 3.4E0 | 1.2E1 | 5.2E1 | 2.6E1 | 1.3E2 | 1.0E-9 | 1.0E-9 | 1.8E2 | 4.6E2 | 61 | 13 | 61 | 13 | 0.56 |
| mM | pg/ml | 2.8E1 | 5.9E1 | 6.5E1 | 1.6E2 | 8.9E1 | 3.1E2 | 1.0E-9 | 1.0E-9 | 4.0E2 | 1.1E3 | 61 | 13 | 61 | 13 | 0.58 |
| mP | pg/ml | 1.5E1 | 1.6E1 | 1.6E1 | 9.6E1 | 1.0E1 | 2.2E2 | 1.0E-9 | 8.2E0 | 5.8E1 | 8.1E2 | 60 | 13 | 60 | 13 | 0.61 |
| mS | pg/ml | 1.5E3 | 1.8E3 | 1.7E3 | 1.9E3 | 9.7E2 | 1.2E3 | 1.0E-9 | 1.0E-9 | 5.1E3 | 4.2E3 | 61 | 13 | 61 | 13 | 0.57 |
| mT | pg/ml | 5.7E1 | 4.5E1 | 1.1E2 | 3.3E2 | 2.1E2 | 5.4E2 | 1.0E1 | 1.6E1 | 1.4E3 | 1.7E3 | 60 | 13 | 60 | 13 | 0.52 |
| mU | pg/ml | 2.2E0 | 3.5E0 | 3.0E0 | 2.2E1 | 2.6E0 | 6.1E1 | 1.0E-9 | 1.5E0 | 1.6E1 | 2.2E2 | 60 | 13 | 60 | 13 | 0.65 |
| mW | pg/ml | 2.2E3 | 1.8E3 | 2.4E3 | 3.0E3 | 1.2E3 | 3.0E3 | 1.0E-9 | 3.7E2 | 6.2E3 | 1.1E4 | 60 | 13 | 60 | 13 | 0.46 |
| mY | pg/ml | 6.5E2 | 7.3E2 | 8.7E2 | 1.4E3 | 9.9E2 | 2.1E3 | 1.0E-9 | 7.5E1 | 5.6E3 | 8.0E3 | 61 | 13 | 61 | 13 | 0.60 |
| mZ | pg/ml | 1.5E2 | 3.0E2 | 3.4E2 | 4.3E2 | 4.9E2 | 3.4E2 | 1.0E-9 | 3.9E1 | 3.1E3 | 1.1E3 | 60 | 13 | 60 | 13 | 0.64 |
| nA | pg/ml | 1.5E0 | 2.7E0 | 7.3E0 | 5.9E0 | 1.5E1 | 1.2E1 | 1.0E-9 | 1.0E-9 | 6.5E1 | 4.3E1 | 60 | 13 | 60 | 13 | 0.49 |
| nB | pg/ml | 2.8E2 | 3.0E2 | 3.0E2 | 3.7E2 | 1.5E2 | 2.5E2 | 3.0E1 | 1.3E2 | 6.9E2 | 9.6E2 | 61 | 13 | 61 | 13 | 0.56 |
| nC | pg/ml | 1.0E-9 | 8.3E1 | 1.1E4 | 9.1E2 | 5.6E4 | 1.7E3 | 1.0E-9 | 1.0E-9 | 3.8E5 | 6.1E3 | 61 | 13 | 61 | 13 | 0.60 |
| nD | pg/ml | 6.9E0 | 9.2E0 | 1.4E1 | 1.3E1 | 3.6E1 | 1.3E1 | 1.0E-9 | 1.0E-9 | 2.6E2 | 3.7E1 | 60 | 13 | 60 | 13 | 0.56 |
| nF | pg/ml | 1.0E-9 | 1.0E-9 | 3.6E0 | 1.0E0 | 1.4E1 | 3.7E0 | 1.0E-9 | 1.0E-9 | 9.3E1 | 1.3E1 | 61 | 13 | 61 | 13 | 0.48 |
| nH | pg/ml | 5.6E-1 | 4.2E0 | 2.2E2 | 1.2E1 | 1.3E3 | 2.0E1 | 1.0E-9 | 1.0E-9 | 1.0E4 | 6.9E1 | 60 | 13 | 60 | 13 | 0.61 |
| nI | pg/ml | 3.0E1 | 1.0E-9 | 7.9E1 | 1.8E1 | 1.7E2 | 3.0E1 | 1.0E-9 | 1.0E-9 | 1.2E3 | 8.0E1 | 61 | 13 | 61 | 13 | 0.33 |
| nJ | pg/ml | 1.7E-1 | 6.3E-1 | 3.2E0 | 1.6E0 | 1.7E1 | 2.2E0 | 1.0E-9 | 1.0E-9 | 1.3E2 | 7.0E0 | 61 | 13 | 61 | 13 | 0.55 |
| nK | pg/ml | 1.0E-9 | 1.0E-9 | 1.7E1 | 1.2E1 | 3.6E1 | 2.1E1 | 1.0E-9 | 1.0E-9 | 2.2E2 | 6.2E1 | 60 | 13 | 60 | 13 | 0.48 |
| nL | pg/ml | 1.0E-9 | 2.5E0 | 3.3E2 | 3.8E1 | 1.8E3 | 7.1E1 | 1.0E-9 | 1.0E-9 | 1.4E4 | 2.3E2 | 61 | 13 | 61 | 13 | 0.55 |
| hR | pg/ml | 2.8E4 | 2.1E4 | 3.0E4 | 2.1E4 | 1.2E4 | 3.7E3 | 1.0E-9 | 1.7E4 | 5.8E4 | 2.7E4 | 78 | 9 | 78 | 9 | 0.23 |
| hV | pg/ml | 4.6E2 | 3.7E2 | 4.5E2 | 4.7E2 | 2.4E2 | 2.1E2 | 1.0E-9 | 2.3E2 | 1.2E3 | 8.8E2 | 78 | 9 | 78 | 9 | 0.53 |

| Code | Units | Median | | Average | | Standard Deviation | | Minimum | | Maximum | | Number of Samples | | Number of Patients | | AUC |
|------|-------|--------|----|---------|----|--------|----|---------|----|---------|----|--------|-----|--------|-----|-----|
| | | NonDis | Dis | NonDis | Dis | NonDis | Dis | NonDis | Dis | NonDis | Dis | NonDis | Dis | NonDis | Dis | |
| hW | pg/ml | 1.7E3 | 2.4E3 | 2.5E3 | 2.5E3 | 4.4E3 | 1.1E3 | 1.0E-9 | 1.3E3 | 4.0E4 | 4.7E3 | 78 | 9 | 78 | 9 | 0.64 |
| hX | pg/ml | 1.1E3 | 8.4E2 | 1.1E3 | 1.2E3 | 9.4E2 | 7.5E2 | 2.5E0 | 6.6E2 | 8.6E3 | 2.9E3 | 78 | 9 | 78 | 9 | 0.50 |
| iA | pg/ml | 1.7E2 | 1.3E2 | 2.6E2 | 2.1E2 | 2.9E2 | 1.9E2 | 1.5E1 | 2.3E1 | 1.8E3 | 5.5E2 | 101 | 16 | 101 | 16 | 0.45 |
| iB | ng/ml | 4.8E0 | 6.8E0 | 6.4E0 | 7.9E0 | 5.2E0 | 3.9E0 | 3.3E-2 | 2.5E0 | 2.4E1 | 1.5E1 | 84 | 10 | 84 | 10 | 0.68 |
| iC | U/ml | 3.0E-1 | 3.9E-1 | 1.3E0 | 4.8E-1 | 6.1E0 | 3.1E-1 | 1.0E-9 | 1.4E-1 | 5.5E1 | 1.1E0 | 84 | 10 | 84 | 10 | 0.60 |
| iH | ng/ml | 1.6E5 | 1.8E5 | 1.6E5 | 1.7E5 | 5.1E4 | 4.2E4 | 2.9E3 | 8.1E4 | 2.6E5 | 2.4E5 | 101 | 16 | 101 | 16 | 0.54 |
| iJ | ng/ml | 4.8E4 | 4.8E4 | 5.3E4 | 6.6E4 | 3.2E4 | 5.5E4 | 1.8E3 | 1.5E4 | 2.5E5 | 2.5E5 | 101 | 16 | 101 | 16 | 0.55 |
| hB | ng/ml | 4.8E-1 | 5.1E-1 | 6.3E-1 | 6.4E-1 | 5.3E-1 | 3.4E-1 | 1.2E-1 | 2.3E-1 | 3.2E0 | 1.2E0 | 101 | 16 | 101 | 16 | 0.55 |
| hC | pg/ml | 4.0E3 | 1.0E4 | 7.9E3 | 1.0E4 | 1.3E4 | 8.3E3 | 4.1E1 | 2.3E2 | 1.1E5 | 2.6E4 | 101 | 16 | 101 | 16 | 0.62 |
| hF | ng/ml | 1.0E-9 | 1.0E-9 | 4.0E1 | 1.0E-9 | 4.0E2 | 0.0E0 | 1.0E-9 | 1.0E-9 | 4.0E3 | 1.0E-9 | 101 | 16 | 101 | 16 | 0.50 |
| hG | pg/ml | 6.7E3 | 7.0E3 | 7.3E3 | 8.9E3 | 3.2E3 | 3.9E3 | 1.8E3 | 4.5E3 | 2.0E4 | 1.8E4 | 101 | 16 | 101 | 16 | 0.63 |
| iO | ng/ml | 3.8E5 | 4.6E5 | 4.1E5 | 4.7E5 | 2.0E5 | 1.8E5 | 1.1E4 | 1.0E5 | 1.1E6 | 7.7E5 | 101 | 16 | 101 | 16 | 0.62 |
| iP | ng/ml | 5.2E4 | 6.2E4 | 5.8E4 | 5.5E4 | 4.9E4 | 1.6E4 | 1.0E-9 | 2.5E4 | 4.4E5 | 7.0E4 | 101 | 16 | 101 | 16 | 0.54 |
| iZ | ng/ml | 1.7E3 | 2.1E3 | 1.8E3 | 2.0E3 | 8.8E2 | 8.0E2 | 6.6E2 | 8.4E2 | 5.7E3 | 3.4E3 | 98 | 17 | 98 | 17 | 0.60 |
| jB | ng/ml | 2.3E5 | 2.6E5 | 2.4E5 | 2.4E5 | 7.2E4 | 8.5E4 | 9.9E4 | 1.3E5 | 3.6E5 | 3.5E5 | 25 | 8 | 25 | 8 | 0.51 |
| rC | pg/ml | 1.6E3 | 1.2E3 | 2.2E3 | 2.4E3 | 2.2E3 | 3.4E3 | 1.0E-9 | 6.0E2 | 1.5E4 | 1.1E4 | 76 | 9 | 76 | 9 | 0.46 |
| rB | pg/ml | 3.1E1 | 9.2E1 | 4.8E1 | 9.6E1 | 6.4E1 | 9.6E1 | 1.0E-9 | 4.0E0 | 3.9E2 | 3.2E2 | 76 | 9 | 76 | 9 | 0.68 |
| jD | ng/ml | 2.7E1 | 5.8E1 | 3.8E1 | 8.8E1 | 4.0E1 | 8.5E1 | 1.0E-9 | 8.8E-1 | 1.9E2 | 2.9E2 | 84 | 10 | 84 | 10 | 0.74 |
| jE | ng/ml | 1.0E-9 | 4.1E0 | 5.1E0 | 9.9E0 | 1.2E1 | 1.2E1 | 1.0E-9 | 1.0E-9 | 6.2E1 | 2.9E1 | 84 | 10 | 84 | 10 | 0.64 |
| jF | ng/ml | 3.8E1 | 4.7E1 | 4.7E1 | 6.0E1 | 5.3E1 | 5.7E1 | 1.0E-9 | 1.0E-9 | 2.1E2 | 1.5E2 | 84 | 10 | 84 | 10 | 0.58 |
| jG | ng/ml | 4.2E3 | 4.8E3 | 4.4E3 | 5.1E3 | 2.0E3 | 1.7E3 | 6.7E2 | 3.6E3 | 9.6E3 | 9.5E3 | 84 | 10 | 84 | 10 | 0.61 |
| jH | ng/ml | 7.5E1 | 1.2E2 | 8.1E1 | 1.2E2 | 5.2E1 | 8.0E1 | 1.3E1 | 1.5E1 | 4.3E2 | 2.4E2 | 84 | 10 | 84 | 10 | 0.65 |
| jI | ng/ml | 7.3E1 | 1.3E2 | 7.8E1 | 1.2E2 | 4.9E1 | 6.1E1 | 1.9E1 | 5.2E1 | 4.4E2 | 2.6E2 | 84 | 10 | 84 | 10 | 0.77 |
| rA | pg/ml | 2.4E1 | 2.8E1 | 3.0E1 | 3.3E1 | 2.8E1 | 2.1E1 | 1.0E-9 | 1.2E1 | 2.0E2 | 7.1E1 | 82 | 10 | 82 | 10 | 0.59 |
| qZ | pg/ml | 4.8E1 | 2.8E1 | 4.5E2 | 1.5E3 | 1.8E3 | 3.8E3 | 2.8E-4 | 3.2E-3 | 1.0E4 | 1.0E4 | 61 | 7 | 61 | 7 | 0.45 |
| qY | pg/ml | 1.5E1 | 2.2E1 | 3.8E1 | 3.9E1 | 5.7E1 | 5.1E1 | 8.7E-1 | 5.6E0 | 3.3E2 | 1.8E2 | 82 | 10 | 82 | 10 | 0.56 |
| qX | pg/ml | 6.0E1 | 6.9E1 | 7.0E1 | 8.6E1 | 4.9E1 | 5.3E1 | 1.0E-9 | 3.6E1 | 2.3E2 | 2.1E2 | 82 | 10 | 82 | 10 | 0.60 |
| qW | pg/ml | 7.7E0 | 7.7E0 | 1.2E1 | 8.1E0 | 1.6E1 | 6.6E0 | 1.0E-9 | 1.0E-9 | 1.2E2 | 2.1E1 | 82 | 10 | 82 | 10 | 0.45 |
| qV | pg/ml | 1.7E3 | 2.4E3 | 2.5E3 | 2.5E3 | 2.0E3 | 1.8E3 | 1.7E2 | 1.0E2 | 1.1E4 | 5.6E3 | 82 | 10 | 82 | 10 | 0.52 |
| qU | pg/ml | 7.7E1 | 1.3E2 | 1.9E2 | 2.5E2 | 3.1E2 | 3.3E2 | 1.0E-9 | 1.0E-9 | 2.1E3 | 1.1E3 | 82 | 10 | 82 | 10 | 0.59 |
| qT | pg/ml | 3.9E1 | 4.8E1 | 6.4E1 | 8.2E1 | 7.1E1 | 7.8E1 | 1.0E-9 | 2.2E1 | 4.9E2 | 2.6E2 | 82 | 10 | 82 | 10 | 0.57 |
| jK | ng/ml | 1.5E3 | 1.3E3 | 1.6E3 | 1.8E3 | 5.7E2 | 1.1E3 | 2.8E2 | 7.0E2 | 3.6E3 | 4.1E3 | 84 | 10 | 84 | 10 | 0.48 |
| jL | ng/ml | 1.9E2 | 2.6E2 | 2.6E2 | 3.3E2 | 2.0E2 | 2.1E2 | 5.6E1 | 1.3E2 | 9.6E2 | 7.0E2 | 84 | 10 | 84 | 10 | 0.63 |
| jM | ng/ml | 6.7E4 | 8.4E4 | 7.4E4 | 7.8E4 | 4.0E4 | 3.9E4 | 4.6E3 | 1.4E4 | 1.8E5 | 1.4E5 | 84 | 10 | 84 | 10 | 0.55 |
| jO | pg/ml | 2.1E5 | 3.3E5 | 2.6E5 | 3.8E5 | 1.7E5 | 1.3E5 | 6.0E4 | 2.6E5 | 1.1E6 | 6.5E5 | 84 | 10 | 84 | 10 | 0.76 |
| jP | pg/ml | 2.5E5 | 4.3E5 | 2.7E5 | 4.1E5 | 1.4E5 | 1.4E5 | 3.6E4 | 2.2E5 | 7.1E5 | 5.8E5 | 84 | 10 | 84 | 10 | 0.78 |
| jQ | pg/ml | 2.2E3 | 2.3E3 | 3.1E3 | 3.4E3 | 3.0E3 | 2.6E3 | 5.0E0 | 8.4E2 | 1.3E4 | 9.2E3 | 84 | 10 | 84 | 10 | 0.57 |
| jR | pg/ml | 5.5E3 | 9.2E3 | 1.0E4 | 1.3E4 | 1.2E4 | 1.3E4 | 1.0E-9 | 1.6E3 | 6.8E4 | 4.6E4 | 84 | 10 | 84 | 10 | 0.59 |
| jT | pg/ml | 1.7E5 | 2.1E5 | 1.7E5 | 2.1E5 | 7.1E4 | 7.0E4 | 7.1E4 | 1.2E5 | 5.5E5 | 3.5E5 | 84 | 10 | 84 | 10 | 0.66 |
| jU | mIU/ml | 5.9E0 | 5.1E0 | 1.2E1 | 1.2E1 | 1.9E1 | 1.6E1 | 8.1E-2 | 5.3E-1 | 1.1E2 | 5.3E1 | 84 | 10 | 84 | 10 | 0.47 |
| jV | mIU/ml | 2.3E0 | 1.0E0 | 4.3E0 | 2.6E0 | 5.8E0 | 3.4E0 | 2.7E-3 | 2.1E-2 | 3.2E1 | 1.0E1 | 84 | 10 | 84 | 10 | 0.38 |
| jY | ng/ml | 9.5E-4 | 2.3E-3 | 8.0E-3 | 5.7E-3 | 3.4E-2 | 9.0E-3 | 1.0E-9 | 1.0E-9 | 3.0E-1 | 2.6E-2 | 84 | 10 | 84 | 10 | 0.57 |
| kC | pg/ml | 9.6E1 | 1.2E2 | 1.9E2 | 2.0E2 | 3.8E2 | 1.8E2 | 2.1E1 | 3.6E1 | 2.7E3 | 5.9E2 | 61 | 13 | 61 | 13 | 0.59 |
| kE | pg/ml | 1.4E5 | 1.5E5 | 1.4E5 | 1.6E5 | 3.8E4 | 4.2E4 | 3.8E4 | 1.2E5 | 2.3E5 | 2.7E5 | 61 | 13 | 61 | 13 | 0.64 |
| kF | pg/mL | 6.1E1 | 8.2E1 | 6.5E1 | 8.7E1 | 2.3E1 | 3.0E1 | 2.7E1 | 5.2E1 | 1.5E2 | 1.4E2 | 61 | 13 | 61 | 13 | 0.74 |
| kG | pg/mL | 7.6E3 | 1.3E4 | 1.0E4 | 3.1E4 | 9.7E3 | 4.2E4 | 1.1E3 | 3.3E3 | 5.8E4 | 1.6E5 | 61 | 13 | 61 | 13 | 0.73 |
| kI | pg/ml | 1.9E2 | 2.1E2 | 2.2E2 | 2.2E2 | 1.1E2 | 1.2E2 | 4.4E1 | 1.0E-9 | 6.7E2 | 4.6E2 | 61 | 13 | 61 | 13 | 0.53 |
| kK | pg/ml | 1.2E2 | 1.1E2 | 1.6E2 | 1.8E2 | 1.5E2 | 1.4E2 | 6.4E0 | 3.4E1 | 9.1E2 | 4.9E2 | 61 | 13 | 61 | 13 | 0.57 |
| kN | pg/ml | 1.0E3 | 1.4E3 | 1.4E3 | 2.3E3 | 1.5E3 | 2.5E3 | 2.1E2 | 5.8E2 | 1.0E4 | 8.7E3 | 61 | 13 | 61 | 13 | 0.63 |
| kO | pg/ml | 7.1E3 | 7.7E3 | 1.0E4 | 7.6E3 | 1.8E4 | 1.9E3 | 3.8E3 | 4.9E3 | 1.5E5 | 1.2E4 | 61 | 13 | 61 | 13 | 0.54 |
| kP | pg/ml | 6.1E3 | 5.4E3 | 7.0E3 | 5.9E3 | 4.4E3 | 3.6E3 | 1.5E3 | 9.6E2 | 2.7E4 | 1.5E4 | 61 | 13 | 61 | 13 | 0.44 |
| kQ | pg/ml | 4.2E3 | 5.4E3 | 5.3E3 | 5.7E3 | 4.3E3 | 2.7E3 | 5.6E2 | 1.5E3 | 2.5E4 | 1.2E4 | 101 | 16 | 101 | 16 | 0.60 |
| kR | pg/ml | 2.2E1 | 2.6E1 | 3.8E1 | 2.8E1 | 1.0E2 | 1.8E1 | 1.0E-9 | 2.9E0 | 1.0E3 | 6.8E1 | 101 | 16 | 101 | 16 | 0.52 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| kS | pg/ml | 8.7E2 | 9.2E2 | 9.9E2 | 9.2E2 | 5.9E2 | 5.4E2 | 2.5E2 | 8.2E1 | 3.2E3 | 2.5E3 | 101 | 16 | 101 | 16 | 0.50 |
| rZ | ng/ml | 1.4E-3 | 1.4E-2 | 9.6E-3 | 3.0E-2 | 3.6E-2 | 3.8E-2 | 1.0E-9 | 1.0E-9 | 3.0E-1 | 1.1E-1 | 77 | 9 | 77 | 9 | 0.79 |
| rY | ng/ml | 6.4E-2 | 4.7E-2 | 4.1E-1 | 2.3E0 | 2.1E0 | 6.7E0 | 1.0E-9 | 2.8E-2 | 1.8E1 | 2.0E1 | 77 | 9 | 77 | 9 | 0.52 |
| rX | ng/ml | 1.0E-9 | 1.0E-9 | 1.0E-1 | 3.6E-1 | 5.1E-1 | 1.0E0 | 1.0E-9 | 1.0E-9 | 3.7E0 | 3.1E0 | 77 | 9 | 77 | 9 | 0.52 |
| lK | pg/ml | 5.9E1 | 9.3E1 | 1.4E2 | 1.8E2 | 1.7E2 | 1.9E2 | 1.0E-9 | 1.0E-9 | 7.4E2 | 5.1E2 | 83 | 10 | 83 | 10 | 0.55 |
| lL | pg/ml | 1.6E3 | 4.1E3 | 2.4E3 | 7.9E3 | 2.6E3 | 1.2E4 | 7.5E1 | 6.9E2 | 1.9E4 | 4.2E4 | 84 | 10 | 84 | 10 | 0.77 |
| lM | pg/ml | 1.2E3 | 8.6E2 | 3.6E3 | 1.1E4 | 6.0E3 | 2.1E4 | 2.1E2 | 9.5E0 | 4.2E4 | 6.7E4 | 84 | 10 | 84 | 10 | 0.45 |
| lN | pg/ml | 1.0E-9 | 4.7E0 | 2.9E0 | 8.4E0 | 6.5E0 | 8.3E0 | 1.0E-9 | 1.0E-9 | 4.3E1 | 2.4E1 | 84 | 10 | 84 | 10 | 0.75 |
| lO | pg/ml | 1.0E-9 | 1.0E-9 | 3.6E0 | 1.0E-9 | 2.1E1 | 0.0E0 | 1.0E-9 | 1.0E-9 | 1.4E2 | 1.0E-9 | 83 | 10 | 83 | 10 | 0.48 |
| nW | pg/ml | 1.1E5 | 1.2E5 | 1.1E5 | 1.2E5 | 2.9E4 | 2.2E4 | 3.6E4 | 8.4E4 | 1.9E5 | 1.5E5 | 101 | 16 | 101 | 16 | 0.60 |
| nY | pg/ml | 2.2E3 | 3.6E3 | 2.6E3 | 3.3E3 | 1.6E3 | 1.6E3 | 5.1E2 | 1.2E3 | 1.0E4 | 7.1E3 | 101 | 16 | 101 | 16 | 0.64 |
| oO | pg/ml | 9.1E4 | 5.5E4 | 1.0E5 | 9.8E4 | 6.2E4 | 1.0E5 | 3.3E3 | 3.1E4 | 3.0E5 | 4.0E5 | 55 | 12 | 55 | 12 | 0.36 |
| oP | pg/ml | 1.2E5 | 1.7E5 | 1.3E5 | 2.0E5 | 7.5E4 | 1.5E5 | 2.4E4 | 5.0E4 | 4.2E5 | 5.7E5 | 55 | 12 | 55 | 12 | 0.66 |
| oQ | pg/ml | 2.9E3 | 3.2E3 | 3.3E3 | 7.4E3 | 2.0E3 | 9.4E3 | 7.7E2 | 1.4E3 | 1.1E4 | 3.2E4 | 55 | 12 | 55 | 12 | 0.59 |
| oE | pg/ml | 1.6E2 | 7.0E2 | 4.4E2 | 1.0E3 | 5.8E2 | 9.4E2 | 1.0E-9 | 8.9E1 | 2.8E3 | 3.4E3 | 101 | 16 | 101 | 16 | 0.77 |
| oF | pg/ml | 1.2E4 | 3.2E4 | 2.5E4 | 4.9E4 | 3.5E4 | 6.3E4 | 4.3E2 | 3.5E2 | 1.7E5 | 2.5E5 | 101 | 16 | 101 | 16 | 0.66 |
| oH | pg/ml | 3.6E1 | 3.0E1 | 7.9E1 | 6.9E1 | 1.2E2 | 1.0E2 | 4.3E-1 | 6.3E0 | 8.6E2 | 3.2E2 | 101 | 16 | 101 | 16 | 0.46 |
| oK | pg/ml | 8.4E2 | 9.8E2 | 1.6E3 | 1.6E3 | 1.9E3 | 1.6E3 | 8.8E1 | 2.1E2 | 1.2E4 | 5.9E3 | 101 | 16 | 101 | 16 | 0.54 |
| oN | pg/ml | 5.4E2 | 5.7E2 | 9.8E2 | 7.3E2 | 2.0E3 | 4.4E2 | 1.1E2 | 2.8E2 | 1.8E4 | 1.8E3 | 101 | 16 | 101 | 16 | 0.56 |
| oW | pg/ml | 2.1E2 | 2.9E2 | 3.9E2 | 1.4E3 | 5.2E2 | 2.6E3 | 2.9E1 | 9.3E1 | 2.7E3 | 7.6E3 | 25 | 8 | 25 | 8 | 0.60 |
| oT | pg/ml | 3.0E2 | 2.4E2 | 3.6E2 | 2.2E2 | 1.9E2 | 7.0E1 | 1.0E2 | 1.1E2 | 7.9E2 | 3.2E2 | 25 | 8 | 25 | 8 | 0.23 |
| oV | pg/ml | 1.3E2 | 8.4E1 | 3.2E2 | 1.1E2 | 5.1E2 | 1.1E2 | 1.1E1 | 1.0E-9 | 2.2E3 | 3.3E2 | 25 | 8 | 25 | 8 | 0.39 |
| oD | pg/ml | 1.5E4 | 1.7E4 | 1.5E4 | 1.7E4 | 5.3E3 | 7.7E3 | 6.6E3 | 9.3E3 | 2.5E4 | 3.2E4 | 25 | 8 | 25 | 8 | 0.57 |
| pF | pg/ml | 5.7E-1 | 7.2E-1 | 1.7E0 | 7.1E-1 | 8.6E0 | 4.8E-1 | 1.0E-9 | 4.3E-2 | 8.7E1 | 1.6E0 | 101 | 16 | 101 | 16 | 0.50 |
| pH | ng/ml | 7.6E0 | 1.1E1 | 8.7E0 | 1.3E1 | 4.0E0 | 7.4E0 | 1.2E0 | 3.0E0 | 1.8E1 | 2.3E1 | 25 | 8 | 25 | 8 | 0.64 |
| pI | ng/ml | 6.9E1 | 7.5E1 | 7.4E1 | 7.0E1 | 4.8E1 | 2.7E1 | 2.3E1 | 3.5E1 | 2.0E2 | 1.1E2 | 25 | 8 | 25 | 8 | 0.54 |
| pK | ng/ml | 4.8E-1 | 4.0E-1 | 4.8E-1 | 3.9E-1 | 2.1E-1 | 1.3E-1 | 2.0E-1 | 1.7E-1 | 8.6E-1 | 6.2E-1 | 25 | 8 | 25 | 8 | 0.41 |

Figure 41.

Descriptive statistics for the assays used to form the panels presented in this figure.

| Code | Units | Median | | Average | | Standard Deviation | | Minimum | | Maximum | | Number of Samples | | Number of Patients | | AUC |
|------|-------|--------|-----|---------|-----|--------|-----|---------|-----|---------|-----|--------|-----|--------|-----|-----|
| | | NonDis | Dis | NonDis | Dis | NonDis | Dis | NonDis | Dis | NonDis | Dis | NonDis | Dis | NonDis | Dis | |
| Aa | mg/dL | 7.1E1 | 1.1E2 | 8.0E1 | 1.2E2 | 5.5E1 | 8.4E1 | 7.0E0 | 1.2E1 | 4.0E2 | 4.8E2 | 186 | 86 | 186 | 86 | 0.68 |
| Ad | ug/mL | 4.2E-2 | 5.9E-2 | 6.6E-2 | 2.5E-1 | 7.9E-2 | 1.1E0 | 6.8E-4 | 9.4E-4 | 3.7E-1 | 8.5E0 | 87 | 54 | 87 | 54 | 0.61 |
| Af | ng/mL | 1.3E0 | 1.1E0 | 1.3E1 | 4.7E0 | 6.0E1 | 6.4E0 | 1.7E-3 | 1.7E-3 | 5.3E2 | 2.3E1 | 87 | 54 | 87 | 54 | 0.49 |
| Aj | ug/mL | 1.6E0 | 2.7E-1 | 2.5E0 | 1.8E0 | 2.4E0 | 2.4E0 | 1.5E-3 | 2.1E-3 | 6.1E0 | 6.1E0 | 87 | 54 | 87 | 54 | 0.39 |
| Al | mg/mL | 7.5E-5 | 1.1E-4 | 2.3E-4 | 3.3E-4 | 4.2E-4 | 4.6E-4 | 4.6E-6 | 7.6E-6 | 1.8E-3 | 1.8E-3 | 87 | 54 | 87 | 54 | 0.60 |
| An | U/mL | 5.3E1 | 8.9E1 | 2.3E2 | 3.8E2 | 6.9E2 | 1.1E3 | 6.1E-1 | 6.4E-1 | 5.5E3 | 7.8E3 | 87 | 54 | 87 | 54 | 0.61 |
| Ao | pg/mL | 9.2E1 | 1.0E2 | 8.6E2 | 1.8E2 | 4.9E3 | 1.9E2 | 2.8E0 | 5.4E0 | 3.9E4 | 8.3E2 | 87 | 54 | 87 | 54 | 0.58 |
| Ap | ng/mL | 3.2E1 | 3.5E1 | 4.1E1 | 4.9E1 | 4.0E1 | 5.1E1 | 2.0E0 | 2.4E0 | 2.8E2 | 2.9E2 | 87 | 54 | 87 | 54 | 0.55 |
| Ar | ng/mL | 4.4E-1 | 1.7E0 | 2.5E0 | 5.2E0 | 6.4E0 | 1.0E1 | 4.1E-2 | 3.4E-3 | 4.7E1 | 5.1E1 | 87 | 54 | 87 | 54 | 0.69 |
| As | ng/mL | 8.7E-3 | 1.1E-2 | 1.2E-2 | 3.7E-2 | 1.5E-2 | 1.7E-1 | 1.7E-3 | 1.7E-3 | 9.8E-2 | 1.2E0 | 87 | 54 | 87 | 54 | 0.52 |
| Aw | pg/mL | 1.6E1 | 1.6E1 | 1.7E1 | 1.7E1 | 6.0E0 | 7.1E0 | 6.8E0 | 2.9E-2 | 4.2E1 | 5.1E1 | 87 | 54 | 87 | 54 | 0.47 |
| Ax | ng/mL | 1.5E0 | 6.4E0 | 1.7E1 | 6.5E1 | 8.4E1 | 1.6E2 | 1.3E-2 | 1.2E-2 | 7.7E2 | 8.5E2 | 87 | 54 | 87 | 54 | 0.66 |
| Ba | ng/mL | 9.0E1 | 9.1E1 | 5.7E2 | 7.4E2 | 1.5E3 | 1.7E3 | 1.1E0 | 3.1E0 | 8.1E3 | 8.1E3 | 87 | 54 | 87 | 54 | 0.54 |
| Bb | ng/mL | 3.7E0 | 6.0E0 | 6.0E0 | 9.4E0 | 7.9E0 | 8.9E0 | 4.1E-3 | 6.8E-1 | 4.9E1 | 3.7E1 | 87 | 54 | 87 | 54 | 0.64 |
| Bc | ng/mL | 3.1E1 | 6.1E1 | 1.1E2 | 1.6E2 | 2.1E2 | 2.7E2 | 4.9E-1 | 4.0E0 | 1.0E3 | 1.2E3 | 87 | 54 | 87 | 54 | 0.61 |
| Bg | ng/mL | 1.1E-1 | 1.2E-1 | 4.7E0 | 1.1E1 | 1.9E1 | 5.6E1 | 5.3E-4 | 1.9E-2 | 1.5E2 | 4.0E2 | 87 | 54 | 87 | 54 | 0.53 |
| Bn | ng/mL | 5.6E-2 | 5.6E-2 | 1.1E0 | 2.1E0 | 2.0E0 | 8.0E0 | 5.6E-2 | 5.6E-2 | 8.5E0 | 5.8E1 | 87 | 54 | 87 | 54 | 0.51 |
| Bo | ng/mL | 1.4E1 | 1.2E1 | 1.5E1 | 1.6E1 | 1.0E1 | 1.3E1 | 1.6E-2 | 1.6E-2 | 3.9E1 | 5.3E1 | 87 | 54 | 87 | 54 | 0.50 |
| Ch | uIU/mL | 1.2E0 | 1.0E0 | 4.2E1 | 4.0E1 | 2.1E2 | 1.8E2 | 3.4E-3 | 3.9E-2 | 1.8E3 | 1.2E3 | 87 | 54 | 87 | 54 | 0.43 |
| Co | pg/mL | 4.4E1 | 5.4E1 | 1.4E2 | 4.4E2 | 4.6E2 | 2.3E3 | 1.5E-1 | 1.5E-1 | 3.7E3 | 1.7E4 | 87 | 54 | 87 | 54 | 0.57 |
| Cp | ng/mL | 2.2E1 | 2.3E1 | 2.7E1 | 5.4E1 | 2.2E1 | 1.7E2 | 6.0E-1 | 1.0E1 | 1.3E2 | 1.3E3 | 87 | 54 | 87 | 54 | 0.56 |
| Cq | ng/mL | 2.8E-2 | 3.8E-2 | 8.9E-2 | 1.1E0 | 2.6E-1 | 6.7E0 | 8.0E-4 | 8.0E-4 | 2.0E0 | 4.9E1 | 87 | 54 | 87 | 54 | 0.59 |
| Cs | ng/mL | 4.6E1 | 2.2E2 | 3.3E2 | 9.8E2 | 1.2E3 | 2.6E3 | 1.0E0 | 8.3E-1 | 1.1E4 | 1.8E4 | 87 | 54 | 87 | 54 | 0.69 |
| Ct | ng/mL | 3.7E-1 | 1.5E-1 | 4.7E1 | 4.3E1 | 1.3E2 | 1.2E2 | 2.2E-2 | 1.1E-4 | 6.2E2 | 6.2E2 | 87 | 54 | 87 | 54 | 0.43 |
| Cu | ng/mL | 2.3E-1 | 3.4E-1 | 3.7E-1 | 2.2E0 | 4.1E-1 | 9.3E0 | 2.8E-2 | 3.5E-2 | 2.4E0 | 6.6E1 | 87 | 54 | 87 | 54 | 0.61 |
| Cv | ng/mL | 3.4E0 | 9.1E0 | 2.2E1 | 3.6E1 | 6.6E1 | 7.7E1 | 2.0E-2 | 5.1E-2 | 5.3E2 | 4.7E2 | 87 | 54 | 87 | 54 | 0.61 |
| Cw | mIU/mL | 2.8E-2 | 4.3E-2 | 4.0E-2 | 1.7E-1 | 3.3E-2 | 9.2E-1 | 8.9E-4 | 4.1E-3 | 1.8E-1 | 6.8E0 | 87 | 54 | 87 | 54 | 0.56 |
| Cx | ng/mL | 8.5E-1 | 3.3E-1 | 5.6E1 | 3.3E1 | 1.0E2 | 7.6E1 | 9.3E-5 | 9.3E-5 | 3.9E2 | 2.8E2 | 87 | 54 | 87 | 54 | 0.48 |
| Db | ug/mL | 7.5E0 | 7.2E0 | 8.5E0 | 8.2E0 | 6.7E0 | 8.8E0 | 4.5E-1 | 8.2E-1 | 4.3E1 | 5.9E1 | 87 | 54 | 87 | 54 | 0.46 |
| Dc | nmol/L | 1.8E-2 | 2.4E-2 | 4.4E-2 | 4.1E-1 | 8.2E-2 | 1.9E0 | 5.2E-6 | 1.3E-3 | 6.3E-1 | 1.4E1 | 87 | 54 | 87 | 54 | 0.62 |
| Dd | ug/mL | 6.3E-2 | 1.6E-1 | 1.4E-1 | 3.2E-1 | 2.3E-1 | 5.5E-1 | 4.8E-4 | 3.4E-3 | 1.6E0 | 3.6E0 | 87 | 54 | 87 | 54 | 0.63 |
| De | ng/mL | 3.4E-3 | 3.4E-3 | 8.0E-2 | 9.7E-2 | 1.5E-1 | 2.0E-1 | 3.4E-3 | 3.4E-3 | 9.2E-1 | 1.1E0 | 87 | 54 | 87 | 54 | 0.50 |
| Dg | ng/mL | 3.2E1 | 3.9E1 | 4.4E1 | 5.4E1 | 3.6E1 | 4.4E1 | 7.8E-1 | 7.1E-1 | 1.2E2 | 1.9E2 | 87 | 54 | 87 | 54 | 0.56 |
| Di | pg/mL | 2.0E0 | 2.7E0 | 2.4E0 | 2.7E0 | 2.4E0 | 1.8E0 | 1.8E-1 | 1.8E-1 | 1.3E1 | 9.0E0 | 87 | 54 | 87 | 54 | 0.57 |
| Dk | uIU/mL | 1.5E-2 | 1.7E-2 | 6.5E-2 | 7.7E-2 | 2.1E-1 | 1.8E-1 | 1.1E-4 | 1.1E-4 | 1.6E0 | 9.8E-1 | 87 | 54 | 87 | 54 | 0.55 |
| Dl | ng/mL | 1.9E2 | 2.5E2 | 2.8E2 | 3.6E2 | 2.7E2 | 3.2E2 | 8.9E0 | 5.5E0 | 1.3E3 | 1.6E3 | 87 | 54 | 87 | 54 | 0.57 |
| Dp | ng/ml | 2.1E0 | 2.1E0 | 5.4E0 | 5.4E0 | 8.4E0 | 1.0E1 | 3.7E-3 | 3.7E-3 | 4.6E1 | 5.6E1 | 70 | 43 | 70 | 43 | 0.48 |
| Dr | pg/ml | 1.2E1 | 3.0E1 | 3.6E1 | 5.5E2 | 5.5E1 | 2.2E3 | 7.5E-1 | 7.5E-1 | 2.5E2 | 1.0E4 | 35 | 23 | 35 | 23 | 0.68 |
| Du | pg/ml | 4.8E1 | 7.9E2 | 5.1E2 | 2.9E3 | 1.1E3 | 6.5E3 | 1.2E0 | 1.2E0 | 5.4E3 | 2.4E4 | 28 | 20 | 28 | 20 | 0.71 |
| Dw | ng/ml | 9.2E-3 | 2.3E-2 | 4.3E-2 | 6.0E-2 | 6.1E-2 | 7.6E-2 | 9.2E-3 | 9.2E-3 | 1.9E-1 | 1.9E-1 | 9 | 8 | 9 | 8 | 0.56 |
| Ef | ng/ml | 9.9E-2 | 1.1E-1 | 7.9E-1 | 9.7E-1 | 1.6E0 | 2.4E0 | 5.7E-4 | 5.7E-4 | 9.5E0 | 9.9E0 | 74 | 49 | 74 | 49 | 0.51 |
| Wm | % | 8.5E-2 | 4.5E-1 | 3.9E0 | 6.6E1 | 2.2E1 | 2.2E2 | 5.4E-2 | 8.5E-2 | 2.0E2 | 1.0E3 | 83 | 47 | 83 | 47 | 0.58 |
| Ed | pg/ml | 5.2E-1 | 2.3E1 | 2.4E1 | 6.3E1 | 3.6E1 | 1.1E2 | 5.2E-1 | 5.2E-1 | 1.3E2 | 5.0E2 | 70 | 43 | 70 | 43 | 0.61 |
| Eo | ng/ml | 6.1E0 | 3.0E0 | 6.9E0 | 7.8E0 | 5.3E0 | 1.3E1 | 3.6E-1 | 3.6E-1 | 1.6E1 | 4.0E1 | 9 | 8 | 9 | 8 | 0.37 |
| Yf | ng/mL | 1.4E1 | 1.7E1 | 3.2E1 | 8.9E1 | 4.9E1 | 1.6E2 | 2.9E-1 | 2.9E-1 | 2.4E2 | 5.9E2 | 30 | 19 | 30 | 19 | 0.54 |
| Tj | pg/mL | 3.7E-1 | 3.7E-1 | 2.4E1 | 7.9E1 | 1.1E2 | 3.4E2 | 3.7E-1 | 3.7E-1 | 8.7E2 | 2.3E3 | 74 | 45 | 74 | 45 | 0.59 |
| Po | pg/ml | 1.3E-1 | 3.5E0 | 7.6E0 | 1.9E1 | 2.5E1 | 4.3E1 | 8.0E-3 | 2.6E-2 | 2.7E2 | 2.2E2 | 180 | 88 | 180 | 88 | 0.62 |
| Ti | ug/mL | 2.3E0 | 6.3E0 | 3.6E0 | 6.7E0 | 3.4E0 | 4.8E0 | 1.2E-1 | 6.0E-1 | 1.4E1 | 1.8E1 | 42 | 27 | 42 | 27 | 0.71 |
| Em | ng/ml | 9.2E-3 | 2.6E-2 | 5.3E-2 | 1.3E-1 | 9.3E-2 | 3.6E-1 | 8.4E-4 | 8.4E-4 | 5.0E-1 | 1.9E0 | 43 | 30 | 43 | 30 | 0.55 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Et | ng/ml | 1.3E3 | 2.2E3 | 1.6E3 | 2.4E3 | 1.1E3 | 1.2E3 | 7.9E1 | 3.1E2 | 4.3E3 | 5.0E3 | 179 | 88 | 179 | 88 | 0.69 |
| Eq | pg/ml | 2.6E2 | 4.8E1 | 4.0E2 | 2.9E2 | 4.1E2 | 4.1E2 | 1.0E0 | 1.0E0 | 1.5E3 | 1.4E3 | 28 | 20 | 28 | 20 | 0.37 |
| Ew | U/ml | 2.0E0 | 2.1E0 | 2.8E0 | 2.5E0 | 2.4E0 | 1.3E0 | 1.1E0 | 1.3E0 | 8.8E0 | 5.0E0 | 9 | 8 | 9 | 8 | 0.54 |
| Th | ug/mL | 1.2E0 | 1.6E0 | 1.8E0 | 2.1E0 | 1.3E0 | 2.0E0 | 2.4E-1 | 2.6E-3 | 5.4E0 | 7.5E0 | 42 | 27 | 42 | 27 | 0.50 |
| Fa | ng/ml | 3.5E1 | 8.5E1 | 5.1E1 | 2.6E2 | 5.5E1 | 6.7E2 | 2.6E-1 | 6.0E-1 | 2.6E2 | 3.7E3 | 68 | 42 | 68 | 42 | 0.67 |
| Ez | ng/ml | 4.3E0 | 4.1E0 | 1.6E1 | 1.6E1 | 2.7E1 | 3.5E1 | 1.3E-2 | 1.3E-2 | 1.6E2 | 2.0E2 | 70 | 43 | 70 | 43 | 0.52 |
| Fb | ng/ml | 2.7E1 | 2.7E1 | 2.2E1 | 2.5E1 | 1.3E1 | 8.7E0 | 6.6E-1 | 2.7E0 | 4.3E1 | 4.1E1 | 68 | 43 | 68 | 43 | 0.56 |
| Ex | ng/ml | 7.4E-2 | 1.4E-1 | 1.6E-1 | 2.4E-1 | 2.3E-1 | 3.4E-1 | 3.5E-5 | 1.7E-4 | 1.0E0 | 1.5E0 | 53 | 34 | 53 | 34 | 0.63 |
| Fc | pg/ml | 2.2E-1 | 2.2E-1 | 3.8E0 | 4.0E1 | 9.6E0 | 1.1E2 | 2.2E-1 | 2.2E-1 | 4.4E1 | 3.9E2 | 29 | 20 | 29 | 20 | 0.59 |
| Fd | pg/ml | 2.3E1 | 2.1E2 | 5.8E2 | 2.7E3 | 1.8E3 | 7.2E3 | 4.5E-1 | 9.8E-1 | 9.2E3 | 2.5E4 | 29 | 20 | 29 | 20 | 0.61 |
| Fi | pg/ml | 2.5E-1 | 2.5E-1 | 2.0E1 | 1.6E2 | 5.9E1 | 4.2E2 | 2.5E-1 | 2.5E-1 | 2.5E2 | 1.8E3 | 29 | 20 | 29 | 20 | 0.64 |
| Fn | ng/ml | 2.5E-1 | 2.1E-1 | 4.4E0 | 4.6E0 | 8.0E0 | 6.8E0 | 1.1E-14 | 2.1E-1 | 3.7E1 | 2.7E1 | 70 | 43 | 70 | 43 | 0.52 |
| Fp | ng/ml | 1.0E1 | 2.5E1 | 2.0E1 | 3.7E1 | 2.4E1 | 3.3E1 | 6.0E-3 | 2.8E-1 | 1.3E2 | 1.3E2 | 181 | 90 | 181 | 90 | 0.67 |
| Fr | ng/ml | 3.1E4 | 6.8E4 | 1.2E5 | 1.9E5 | 1.9E5 | 2.3E5 | 1.9E2 | 1.8E3 | 8.4E5 | 8.4E5 | 184 | 91 | 184 | 91 | 0.63 |
| Fw | pg/ml | 8.5E-1 | 7.6E0 | 4.8E1 | 5.8E1 | 3.4E2 | 1.5E2 | 1.2E-1 | 1.2E-1 | 3.0E3 | 9.1E2 | 75 | 48 | 75 | 48 | 0.63 |
| Fy | ng/ml | 3.2E1 | 5.5E1 | 5.3E1 | 1.1E2 | 5.9E1 | 1.5E2 | 1.2E-1 | 1.2E-1 | 2.8E2 | 6.5E2 | 69 | 42 | 69 | 42 | 0.64 |
| Gh | pg/ml | 2.0E0 | 3.7E0 | 2.3E1 | 1.5E1 | 6.6E1 | 3.3E1 | 2.9E-2 | 2.9E-2 | 3.2E2 | 1.4E2 | 29 | 20 | 29 | 20 | 0.54 |
| Gb | % | 4.3E1 | 5.5E1 | 3.9E1 | 7.3E1 | 2.6E1 | 6.8E1 | 2.2E0 | 1.3E1 | 1.0E2 | 3.0E2 | 29 | 20 | 29 | 20 | 0.67 |
| Gc | ng/ml | 6.2E1 | 1.6E2 | 1.0E2 | 3.0E2 | 1.3E2 | 2.8E2 | 6.4E0 | 6.9E0 | 7.3E2 | 1.2E3 | 35 | 23 | 35 | 23 | 0.80 |
| Gd | ng/ml | 3.4E1 | 3.6E1 | 3.5E1 | 3.5E1 | 1.7E1 | 2.0E1 | 3.0E0 | 3.7E0 | 6.9E1 | 8.0E1 | 43 | 24 | 43 | 24 | 0.49 |
| Gn | U/ml | 2.5E-1 | 2.2E-1 | 1.6E0 | 5.6E0 | 5.2E0 | 2.3E1 | 5.6E-3 | 1.2E-2 | 3.0E1 | 1.1E2 | 34 | 23 | 34 | 23 | 0.53 |
| Gl | pg/ml | 8.5E3 | 1.6E4 | 1.1E4 | 1.5E4 | 9.2E3 | 1.0E4 | 9.1E1 | 4.0E2 | 3.1E4 | 3.1E4 | 75 | 48 | 75 | 48 | 0.59 |
| Gp | U/ml | 1.2E0 | 1.0E0 | 2.1E0 | 2.5E0 | 2.6E0 | 3.6E0 | 1.5E-2 | 1.5E-2 | 1.6E1 | 1.8E1 | 75 | 48 | 75 | 48 | 0.48 |
| Gz | ug/ml | 6.4E0 | 9.6E-1 | 6.2E0 | 4.0E0 | 6.0E0 | 5.0E0 | 4.2E-2 | 1.0E-1 | 2.5E1 | 1.5E1 | 48 | 31 | 48 | 31 | 0.43 |
| Ha | ng/ml | 1.6E0 | 4.8E0 | 5.8E0 | 1.5E1 | 1.4E1 | 2.9E1 | 1.7E-2 | 1.7E-2 | 1.0E2 | 1.0E2 | 68 | 43 | 68 | 43 | 0.67 |
| Nm | pg/ml | 1.4E4 | 2.6E4 | 2.8E4 | 6.9E4 | 6.3E4 | 1.5E5 | 1.0E-9 | 1.0E-9 | 7.8E5 | 9.6E5 | 181 | 90 | 181 | 90 | 0.63 |
| Nn | pg/ml | 1.4E2 | 3.3E2 | 2.0E3 | 7.3E3 | 9.0E3 | 3.5E4 | 1.0E-9 | 3.3E0 | 9.5E4 | 3.1E5 | 181 | 90 | 181 | 90 | 0.61 |
| No | pg/ml | 1.4E1 | 2.6E1 | 2.9E1 | 7.7E1 | 5.5E1 | 1.6E2 | 1.0E-9 | 1.0E-9 | 5.6E2 | 9.1E2 | 181 | 90 | 181 | 90 | 0.66 |
| Nq | pg/ml | 2.3E0 | 2.3E0 | 2.7E1 | 3.6E1 | 8.7E1 | 1.0E2 | 1.0E-9 | 1.0E-9 | 6.7E2 | 6.3E2 | 181 | 90 | 181 | 90 | 0.52 |
| Nr | pg/ml | 1.3E0 | 4.7E0 | 2.1E1 | 5.2E1 | 8.7E1 | 1.7E2 | 1.0E-9 | 1.0E-9 | 9.8E2 | 1.4E3 | 181 | 90 | 181 | 90 | 0.61 |
| Ns | pg/ml | 1.0E-9 | 1.0E-9 | 1.3E1 | 2.6E0 | 8.8E1 | 1.5E1 | 1.0E-9 | 1.0E-9 | 1.1E3 | 1.0E2 | 181 | 90 | 181 | 90 | 0.48 |
| Nt | pg/ml | 1.0E2 | 1.4E2 | 1.3E2 | 2.0E2 | 1.0E2 | 2.3E2 | 1.5E1 | 2.3E1 | 8.8E2 | 1.7E3 | 181 | 90 | 181 | 90 | 0.65 |
| Nu | pg/ml | 1.9E1 | 5.0E1 | 5.2E1 | 8.2E1 | 8.2E1 | 1.0E2 | 1.0E-9 | 1.0E-9 | 3.7E2 | 6.3E2 | 181 | 90 | 181 | 90 | 0.63 |
| Lu | pg/ml | 1.0E4 | 1.0E4 | 1.9E4 | 1.2E4 | 5.4E4 | 1.1E4 | 9.4E2 | 5.2E2 | 5.6E5 | 5.9E4 | 181 | 90 | 181 | 90 | 0.49 |
| Lv | pg/ml | 1.0E-9 | 7.0E0 | 1.5E1 | 2.5E1 | 3.2E1 | 4.0E1 | 1.0E-9 | 1.0E-9 | 2.6E2 | 1.9E2 | 181 | 90 | 181 | 90 | 0.58 |
| Lw | pg/ml | 1.0E-9 | 1.0E-9 | 1.8E-1 | 3.9E0 | 2.4E0 | 2.1E1 | 1.0E-9 | 1.0E-9 | 3.2E1 | 1.8E2 | 181 | 90 | 181 | 90 | 0.54 |
| Lx | pg/ml | 1.0E-9 | 6.0E1 | 1.5E2 | 6.4E2 | 5.4E2 | 2.5E3 | 1.0E-9 | 1.0E-9 | 6.2E3 | 2.2E4 | 181 | 90 | 181 | 90 | 0.65 |
| Ly | pg/ml | 1.0E-9 | 1.0E-9 | 9.3E0 | 1.0E1 | 1.8E1 | 1.8E1 | 1.0E-9 | 1.0E-9 | 1.1E2 | 9.6E1 | 181 | 90 | 181 | 90 | 0.51 |
| Lz | pg/ml | 1.0E-9 | 1.0E-9 | 3.1E0 | 4.5E0 | 3.3E1 | 1.7E1 | 1.0E-9 | 1.0E-9 | 4.3E2 | 1.3E2 | 181 | 90 | 181 | 90 | 0.56 |
| Ma | pg/ml | 4.4E2 | 8.2E2 | 2.3E3 | 3.6E3 | 6.2E3 | 7.9E3 | 1.0E-9 | 1.0E-9 | 6.5E4 | 5.2E4 | 181 | 90 | 181 | 90 | 0.60 |
| Mb | pg/ml | 2.5E1 | 2.9E1 | 3.2E1 | 3.5E1 | 1.9E1 | 1.8E1 | 9.2E0 | 4.1E0 | 2.1E2 | 1.1E2 | 181 | 90 | 181 | 90 | 0.55 |
| Mc | pg/ml | 1.0E-9 | 1.0E-9 | 1.1E-1 | 1.0E-9 | 1.0E0 | 0.0E0 | 1.0E-9 | 1.0E-9 | 1.3E1 | 1.0E-9 | 181 | 90 | 181 | 90 | 0.49 |
| Md | pg/ml | 1.0E-9 | 1.0E-9 | 5.0E-1 | 8.6E-1 | 4.9E0 | 3.9E0 | 1.0E-9 | 1.0E-9 | 6.5E1 | 2.9E1 | 181 | 90 | 181 | 90 | 0.52 |
| Me | pg/ml | 3.1E1 | 3.3E1 | 3.0E1 | 3.4E1 | 1.9E1 | 3.9E1 | 1.1E-1 | 1.0E-9 | 1.6E2 | 3.2E2 | 181 | 90 | 181 | 90 | 0.52 |
| Mf | pg/ml | 1.0E-9 | 1.0E-9 | 5.8E-1 | 1.2E0 | 3.2E0 | 6.0E0 | 1.0E-9 | 1.0E-9 | 3.7E1 | 5.6E1 | 181 | 90 | 181 | 90 | 0.54 |
| Mg | pg/ml | 1.1E0 | 1.8E0 | 6.9E0 | 1.0E1 | 1.2E1 | 2.2E1 | 1.0E-9 | 1.0E-9 | 8.8E1 | 1.5E2 | 181 | 90 | 181 | 90 | 0.52 |
| Mh | pg/ml | 1.0E-9 | 1.0E-9 | 1.0E0 | 1.3E0 | 8.3E0 | 5.1E0 | 1.0E-9 | 1.0E-9 | 1.1E2 | 4.2E1 | 181 | 90 | 181 | 90 | 0.58 |
| Mi | pg/ml | 1.0E-9 | 1.0E-9 | 1.1E0 | 1.2E1 | 9.6E0 | 6.5E1 | 1.0E-9 | 1.0E-9 | 1.2E2 | 5.2E2 | 181 | 90 | 181 | 90 | 0.54 |
| Mj | pg/ml | 1.0E-9 | 1.0E-9 | 6.5E0 | 1.0E1 | 3.6E1 | 3.2E1 | 1.0E-9 | 1.0E-9 | 4.6E2 | 2.1E2 | 181 | 90 | 181 | 90 | 0.53 |
| Mk | pg/ml | 2.1E0 | 2.9E0 | 1.6E1 | 2.7E1 | 7.9E1 | 1.5E2 | 1.0E-9 | 1.0E-9 | 1.0E3 | 1.3E3 | 181 | 90 | 181 | 90 | 0.49 |
| Ml | pg/ml | 1.0E-9 | 1.0E-9 | 1.4E1 | 1.4E1 | 1.6E2 | 6.6E1 | 1.0E-9 | 1.0E-9 | 2.1E3 | 5.1E2 | 181 | 90 | 181 | 90 | 0.56 |
| Mm | pg/ml | 4.5E2 | 8.7E2 | 9.7E2 | 1.6E3 | 1.1E3 | 1.9E3 | 1.0E-9 | 1.9E1 | 6.0E3 | 1.0E4 | 181 | 90 | 181 | 90 | 0.61 |
| Mn | pg/ml | 5.4E0 | 1.0E1 | 1.2E1 | 1.3E1 | 3.3E1 | 1.1E1 | 1.0E-9 | 1.0E-9 | 3.5E2 | 6.8E1 | 181 | 90 | 181 | 90 | 0.66 |

| Code | Units | Median | | Average | | Standard Deviation | | Minimum | | Maximum | | Number of Samples | | Number of Patients | | AUC |
|------|-------|--------|-----|---------|-----|--------------------|-----|---------|-----|---------|-----|-------------------|-----|--------------------|-----|-----|
| | | NonDis | Dis | NonDis | Dis | NonDis | Dis | NonDis | Dis | NonDis | Dis | NonDis | Dis | NonDis | Dis | |
| Mp | pg/ml | 1.0E-9 | 1.0E-9 | 1.1E1 | 5.6E1 | 2.6E1 | 2.6E2 | 1.0E-9 | 1.0E-9 | 1.8E2 | 2.4E3 | 181 | 90 | 181 | 90 | 0.54 |
| Mq | pg/ml | 1.0E-9 | 1.0E-9 | 2.1E0 | 5.3E0 | 1.4E1 | 1.8E1 | 1.0E-9 | 1.0E-9 | 1.8E2 | 1.0E2 | 181 | 90 | 181 | 90 | 0.53 |
| Mr | pg/ml | 1.0E-9 | 1.0E-9 | 2.3E1 | 1.2E2 | 1.1E2 | 4.8E2 | 1.0E-9 | 1.0E-9 | 1.4E3 | 3.4E3 | 181 | 90 | 181 | 90 | 0.59 |
| Ms | pg/ml | 3.8E2 | 2.6E2 | 4.9E2 | 3.7E2 | 5.5E2 | 3.8E2 | 1.0E-9 | 1.0E-9 | 3.3E3 | 1.5E3 | 181 | 90 | 181 | 90 | 0.43 |
| Mt | pg/ml | 3.3E-1 | 1.0E0 | 9.4E0 | 5.5E1 | 5.6E1 | 3.4E2 | 1.0E-9 | 1.0E-9 | 7.1E2 | 3.2E3 | 181 | 90 | 181 | 90 | 0.60 |
| Mu | pg/ml | 1.0E-9 | 1.0E-9 | 2.4E0 | 1.7E0 | 1.9E1 | 6.7E0 | 1.0E-9 | 1.0E-9 | 2.3E2 | 4.8E1 | 181 | 90 | 181 | 90 | 0.53 |
| Mv | pg/ml | 1.0E-9 | 1.0E-9 | 9.3E1 | 7.9E1 | 4.5E2 | 2.1E2 | 1.0E-9 | 1.0E-9 | 4.5E3 | 9.2E2 | 181 | 90 | 181 | 90 | 0.54 |
| Mw | pg/ml | 2.9E1 | 7.4E1 | 3.7E2 | 3.6E2 | 1.8E3 | 9.1E2 | 1.0E-9 | 1.0E-9 | 1.8E4 | 5.3E3 | 181 | 90 | 181 | 90 | 0.60 |
| Mx | pg/ml | 1.0E-9 | 1.0E-9 | 4.2E-1 | 7.0E-1 | 2.5E0 | 2.3E0 | 1.0E-9 | 1.0E-9 | 3.2E1 | 2.0E1 | 181 | 90 | 181 | 90 | 0.57 |
| My | pg/ml | 1.0E-9 | 1.0E-9 | 5.1E2 | 2.1E2 | 3.3E3 | 8.9E2 | 1.0E-9 | 1.0E-9 | 3.9E4 | 8.0E3 | 181 | 90 | 181 | 90 | 0.51 |
| Mz | pg/ml | 1.0E1 | 2.0E1 | 2.3E1 | 7.5E1 | 5.2E1 | 2.4E2 | 1.0E-9 | 1.0E-9 | 5.5E2 | 1.9E3 | 181 | 90 | 181 | 90 | 0.68 |
| Na | pg/ml | 1.0E-9 | 1.0E-9 | 6.1E-1 | 9.1E-1 | 1.9E0 | 4.6E0 | 1.0E-9 | 1.0E-9 | 9.6E0 | 4.2E1 | 181 | 90 | 181 | 90 | 0.52 |
| Nb | pg/ml | 2.0E0 | 2.5E0 | 4.0E0 | 7.5E0 | 1.3E1 | 2.5E1 | 1.0E-9 | 1.0E-9 | 1.5E2 | 2.1E2 | 181 | 90 | 181 | 90 | 0.57 |
| Nc | pg/ml | 3.6E2 | 2.1E2 | 5.6E2 | 4.0E2 | 8.5E2 | 5.2E2 | 1.0E-9 | 1.0E-9 | 9.2E3 | 3.2E3 | 181 | 90 | 181 | 90 | 0.44 |
| Nd | pg/ml | 2.7E1 | 1.6E1 | 2.3E1 | 6.0E1 | 1.8E1 | 2.5E2 | 1.0E-9 | 1.0E-9 | 7.8E1 | 2.1E3 | 181 | 90 | 181 | 90 | 0.48 |
| Ne | pg/ml | 4.5E2 | 3.6E2 | 5.6E2 | 4.4E2 | 6.4E2 | 4.6E2 | 1.0E-9 | 1.0E-9 | 7.0E3 | 3.6E3 | 181 | 90 | 181 | 90 | 0.44 |
| Nf | pg/ml | 1.0E-9 | 1.0E-9 | 2.5E0 | 4.3E0 | 9.8E0 | 1.9E1 | 1.0E-9 | 1.0E-9 | 8.2E1 | 1.3E2 | 181 | 90 | 181 | 90 | 0.50 |
| Ng | pg/ml | 2.0E1 | 1.7E1 | 1.1E2 | 8.9E1 | 2.2E2 | 1.5E2 | 1.0E-9 | 1.0E-9 | 1.6E3 | 8.5E2 | 181 | 90 | 181 | 90 | 0.51 |
| Nh | pg/ml | 6.1E1 | 5.4E1 | 8.6E1 | 6.5E1 | 8.0E1 | 6.8E1 | 1.0E-9 | 3.6E0 | 5.6E2 | 5.1E2 | 181 | 90 | 181 | 90 | 0.42 |
| Ni | pg/ml | 1.0E-9 | 1.0E-9 | 8.2E1 | 1.0E2 | 1.3E2 | 1.7E2 | 1.0E-9 | 1.0E-9 | 7.1E2 | 1.1E3 | 181 | 90 | 181 | 90 | 0.51 |
| Nj | pg/ml | 7.2E0 | 5.3E0 | 1.1E1 | 7.7E0 | 1.1E1 | 8.6E0 | 1.0E-9 | 1.0E-9 | 6.8E1 | 4.6E1 | 181 | 90 | 181 | 90 | 0.41 |
| Nk | pg/ml | 1.6E1 | 1.6E1 | 3.1E1 | 3.2E1 | 3.8E1 | 4.2E1 | 1.0E-9 | 1.0E-9 | 2.0E2 | 2.0E2 | 181 | 90 | 181 | 90 | 0.51 |
| Nl | pg/ml | 4.4E1 | 3.7E1 | 6.0E1 | 4.4E1 | 9.1E1 | 4.2E1 | 1.0E-9 | 1.0E-9 | 1.1E3 | 2.3E2 | 181 | 90 | 181 | 90 | 0.43 |
| Hl | pg/ml | 4.6E0 | 1.7E1 | 3.7E1 | 2.1E2 | 6.5E1 | 8.0E2 | 1.0E-9 | 1.0E-9 | 3.0E2 | 3.6E3 | 29 | 20 | 29 | 20 | 0.56 |
| Ho | pg/ml | 1.7E1 | 2.0E1 | 2.2E1 | 5.5E1 | 1.9E1 | 9.0E1 | 1.1E0 | 7.6E0 | 8.3E1 | 3.9E2 | 29 | 20 | 29 | 20 | 0.64 |
| Hp | ng/ml | 1.8E0 | 1.6E0 | 1.3E2 | 1.3E2 | 3.1E2 | 3.2E2 | 1.0E-9 | 8.1E-1 | 8.9E2 | 8.9E2 | 29 | 20 | 29 | 20 | 0.50 |
| Tz | pg/ml | 3.5E3 | 7.2E3 | 7.7E3 | 6.4E4 | 2.0E4 | 3.2E5 | 7.4E1 | 1.0E-9 | 1.7E5 | 2.1E6 | 71 | 43 | 71 | 43 | 0.63 |
| Ua | pg/ml | 2.5E3 | 4.3E3 | 1.5E4 | 1.3E4 | 3.0E4 | 2.5E4 | 2.3E2 | 1.0E-9 | 1.4E5 | 1.2E5 | 71 | 43 | 71 | 43 | 0.57 |
| Ub | pg/ml | 5.8E2 | 4.1E2 | 8.8E2 | 6.9E2 | 1.3E3 | 9.0E2 | 5.4E0 | 1.0E-9 | 9.8E3 | 4.4E3 | 71 | 43 | 71 | 43 | 0.44 |
| Ue | pg/ml | 2.8E1 | 2.6E1 | 3.6E1 | 3.8E1 | 3.5E1 | 3.3E1 | 5.2E0 | 9.8E-2 | 2.7E2 | 1.4E2 | 71 | 43 | 71 | 43 | 0.51 |
| Uc | pg/ml | 7.1E2 | 9.5E2 | 1.3E3 | 3.0E3 | 1.6E3 | 8.7E3 | 1.5E1 | 1.0E-9 | 9.4E3 | 5.7E4 | 71 | 43 | 71 | 43 | 0.55 |
| Ud | pg/ml | 1.0E-9 | 1.0E-9 | 5.5E0 | 1.2E0 | 4.6E1 | 8.1E0 | 1.0E-9 | 1.0E-9 | 3.9E2 | 5.3E1 | 71 | 43 | 71 | 43 | 0.50 |
| Hq | pg/ml | 1.2E0 | 1.2E0 | 1.2E2 | 3.7E2 | 1.5E3 | 3.0E3 | 1.0E-9 | 1.0E-9 | 2.0E4 | 2.8E4 | 181 | 88 | 181 | 88 | 0.51 |
| Hr | pg/ml | 8.5E1 | 8.8E1 | 5.5E2 | 5.5E2 | 1.0E3 | 1.4E3 | 1.0E-9 | 1.0E-9 | 8.4E3 | 8.9E3 | 181 | 88 | 181 | 88 | 0.51 |
| Hu | pg/ml | 5.3E0 | 3.2E1 | 8.3E3 | 5.3E2 | 5.4E4 | 1.3E3 | 1.0E-9 | 1.0E-9 | 6.3E5 | 6.5E3 | 181 | 88 | 181 | 88 | 0.55 |
| Hv | pg/ml | 1.5E0 | 1.3E0 | 2.6E0 | 1.5E0 | 6.1E0 | 9.7E1 | 1.0E-9 | 1.0E-9 | 6.9E1 | 8.9E2 | 181 | 88 | 181 | 88 | 0.51 |
| Hw | pg/ml | 5.5E0 | 6.5E0 | 1.7E1 | 1.3E2 | 5.8E1 | 1.0E3 | 1.0E-9 | 5.1E-1 | 6.4E2 | 9.4E3 | 181 | 88 | 181 | 88 | 0.50 |
| Hx | pg/ml | 8.8E0 | 1.1E1 | 7.7E1 | 5.2E1 | 6.9E2 | 1.7E2 | 1.0E-9 | 1.0E-9 | 9.3E3 | 1.3E3 | 181 | 88 | 181 | 88 | 0.53 |
| Ib | ng/ml | 3.3E-2 | 3.6E-2 | 1.6E0 | 1.5E0 | 6.0E0 | 8.6E0 | 1.0E-9 | 1.0E-9 | 3.6E1 | 5.6E1 | 69 | 42 | 69 | 42 | 0.50 |
| Ic | U/ml | 2.2E2 | 2.2E2 | 1.4E3 | 2.2E2 | 7.9E3 | 1.6E2 | 2.4E0 | 1.1E1 | 6.5E4 | 7.9E2 | 69 | 42 | 69 | 42 | 0.52 |
| Id | U/ml | 5.0E-1 | 1.1E0 | 8.8E-1 | 1.2E1 | 1.2E0 | 6.6E1 | 1.0E-9 | 5.1E-2 | 6.4E0 | 4.3E2 | 69 | 42 | 69 | 42 | 0.71 |
| Tt | pg/ml | 1.7E2 | 1.7E2 | 1.7E2 | 1.8E2 | 5.1E1 | 7.0E1 | 4.3E1 | 7.3E1 | 3.0E2 | 4.4E2 | 63 | 40 | 63 | 40 | 0.50 |
| To | pg/ml | 1.8E0 | 1.4E0 | 2.2E0 | 1.9E0 | 2.1E0 | 2.4E0 | 1.0E-9 | 1.0E-9 | 9.9E0 | 1.2E1 | 68 | 41 | 68 | 41 | 0.43 |
| Tr | pg/ml | 3.6E0 | 4.4E0 | 5.6E0 | 1.7E1 | 6.8E0 | 5.0E1 | 1.0E-9 | 2.4E-1 | 3.8E1 | 3.1E2 | 66 | 40 | 66 | 40 | 0.57 |
| Tn | pg/ml | 3.3E1 | 4.3E1 | 8.3E1 | 2.1E2 | 2.1E2 | 5.3E2 | 3.5E0 | 2.4E0 | 1.7E3 | 2.3E3 | 68 | 41 | 68 | 41 | 0.56 |
| Tv | ng/ml | 1.2E1 | 1.2E1 | 2.2E1 | 2.2E1 | 4.3E1 | 1.1E3 | 1.0E-9 | 1.0E-9 | 3.2E2 | 7.1E3 | 68 | 41 | 68 | 41 | 0.52 |
| Ih | ng/ml | 5.6E1 | 1.3E2 | 2.0E2 | 4.0E2 | 3.5E2 | 6.2E2 | 1.0E-9 | 1.0E-9 | 2.1E3 | 3.6E3 | 181 | 89 | 181 | 89 | 0.63 |
| Ii | ng/ml | 7.1E1 | 1.2E2 | 2.1E2 | 3.0E2 | 5.1E2 | 6.4E2 | 7.3E-1 | 2.3E0 | 5.2E3 | 4.5E3 | 181 | 89 | 181 | 89 | 0.61 |
| Ij | ng/ml | 7.3E1 | 1.2E2 | 2.0E2 | 4.7E2 | 7.0E2 | 2.6E3 | 2.8E0 | 2.1E1 | 6.4E3 | 2.4E4 | 180 | 87 | 180 | 87 | 0.66 |
| Ik | ng/ml | 9.4E0 | 1.7E1 | 2.3E3 | 2.6E2 | 1.6E4 | 4.7E2 | 5.9E-1 | 2.3E0 | 1.2E5 | 1.8E3 | 179 | 88 | 179 | 88 | 0.59 |
| Il | ng/ml | 3.4E2 | 4.9E2 | 1.2E3 | 1.7E3 | 2.7E3 | 3.2E3 | 1.0E-9 | 1.0E-9 | 1.2E4 | 1.2E4 | 178 | 87 | 178 | 87 | 0.57 |
| Im | ng/ml | 1.9E2 | 3.5E2 | 3.5E2 | 8.4E2 | 5.6E2 | 1.3E3 | 1.4E1 | 4.7E1 | 6.0E3 | 6.2E3 | 179 | 88 | 179 | 88 | 0.69 |
| In | ng/ml | 3.2E0 | 3.9E0 | 1.8E1 | 7.1E1 | 8.6E1 | 4.8E2 | 1.0E-9 | 1.0E-9 | 1.1E3 | 4.5E3 | 181 | 89 | 181 | 89 | 0.54 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|-----|--------|-----|-----|
| | | NonDis | Dis | NonDis | Dis | NonDis | Dis | NonDis | Dis | NonDis | Dis | NonDis | Dis | NonDis | Dis | |
| Hb | ng/ml | 2.0E1 | 3.7E1 | 3.0E1 | 4.8E1 | 3.4E1 | 4.2E1 | 4.8E-1 | 2.6E0 | 2.0E2 | 1.9E2 | 69 | 46 | 69 | 46 | 0.66 |
| Hc | pg/ml | 7.2E2 | 6.4E2 | 3.8E3 | 2.2E3 | 1.3E4 | 7.5E3 | 1.0E-9 | 1.0E-9 | 1.0E5 | 5.0E4 | 69 | 46 | 69 | 46 | 0.44 |
| Hf | ng/ml | 1.6E2 | 3.1E2 | 3.7E2 | 4.9E2 | 5.3E2 | 6.4E2 | 1.0E-9 | 1.0E-9 | 2.5E3 | 3.2E3 | 69 | 46 | 69 | 46 | 0.58 |
| Io | ng/ml | 1.0E4 | 1.5E4 | 2.1E4 | 2.2E4 | 5.9E4 | 2.3E4 | 1.2E2 | 1.0E-9 | 7.1E5 | 1.1E5 | 180 | 90 | 180 | 90 | 0.59 |
| Ip | ng/ml | 9.3E0 | 2.9E1 | 2.1E1 | 2.7E1 | 2.9E1 | 2.2E1 | 1.0E-9 | 1.0E-9 | 2.3E2 | 7.8E1 | 180 | 90 | 180 | 90 | 0.60 |
| Iq | ug/ml | 1.1E-1 | 1.5E-1 | 6.5E-1 | 4.1E0 | 2.7E0 | 2.3E1 | 1.0E-9 | 1.0E-9 | 3.4E1 | 2.2E2 | 180 | 90 | 180 | 90 | 0.58 |
| Ir | ug/ml | 3.5E-1 | 9.3E-1 | 1.4E0 | 1.2E1 | 3.4E0 | 4.6E1 | 1.0E-9 | 1.0E-9 | 2.6E1 | 3.7E2 | 179 | 90 | 179 | 90 | 0.65 |
| Is | ng/ml | 1.7E0 | 3.8E0 | 6.7E0 | 1.8E1 | 1.4E1 | 3.8E1 | 1.0E-9 | 1.0E-9 | 1.1E2 | 2.6E2 | 180 | 90 | 180 | 90 | 0.61 |
| It | ng/ml | 1.7E0 | 3.0E0 | 1.4E1 | 2.4E1 | 7.0E1 | 9.4E1 | 1.0E-9 | 1.0E-9 | 8.3E2 | 6.8E2 | 180 | 90 | 180 | 90 | 0.60 |
| Iu | ng/ml | 1.7E2 | 1.8E2 | 1.1E3 | 1.7E3 | 3.5E3 | 5.1E3 | 1.0E-9 | 1.0E-9 | 2.9E4 | 2.4E4 | 180 | 90 | 180 | 90 | 0.52 |
| Iv | ng/ml | 1.3E1 | 2.2E1 | 3.6E1 | 1.8E2 | 8.6E1 | 6.8E2 | 1.0E-9 | 1.0E-9 | 7.7E2 | 3.8E3 | 179 | 90 | 179 | 90 | 0.60 |
| Iz | ng/ml | 1.2E2 | 1.3E2 | 4.0E2 | 2.6E2 | 8.5E2 | 3.3E2 | 1.5E0 | 4.9E0 | 6.1E3 | 1.7E3 | 69 | 46 | 69 | 46 | 0.52 |
| Yg | pg/ml | 2.4E2 | 2.8E2 | 2.2E3 | 8.1E2 | 9.2E3 | 1.1E3 | 1.0E-9 | 6.9E1 | 5.0E4 | 3.9E3 | 29 | 19 | 29 | 19 | 0.60 |
| Yh | pg/ml | 2.1E2 | 2.6E2 | 3.6E2 | 4.5E2 | 4.7E2 | 5.6E2 | 1.0E-9 | 1.0E-9 | 1.8E3 | 2.1E3 | 29 | 19 | 29 | 19 | 0.55 |
| Yi | pg/ml | 2.2E2 | 5.1E2 | 4.8E2 | 2.4E3 | 6.0E2 | 6.2E3 | 1.0E-9 | 5.8E1 | 2.0E3 | 2.6E4 | 29 | 19 | 29 | 19 | 0.69 |
| Yk | U/ml | 1.0E-9 | 1.0E-9 | 2.8E-1 | 9.9E-2 | 7.0E-1 | 3.3E-1 | 1.0E-9 | 1.0E-9 | 3.0E0 | 1.4E0 | 29 | 19 | 29 | 19 | 0.43 |
| Yj | pg/ml | 1.5E2 | 1.1E2 | 3.4E2 | 2.4E2 | 6.2E2 | 3.3E2 | 1.0E-9 | 5.2E1 | 3.2E3 | 1.5E3 | 29 | 19 | 29 | 19 | 0.50 |
| Yd | ng/ml | 1.6E-1 | 2.8E-1 | 4.1E-1 | 3.9E-1 | 5.7E-1 | 5.0E-1 | 6.6E-3 | 1.7E-2 | 1.9E0 | 2.3E0 | 30 | 20 | 30 | 20 | 0.57 |
| Wb | pg/ml | 2.6E4 | 5.0E4 | 3.0E4 | 7.6E4 | 1.8E4 | 1.4E5 | 6.3E3 | 1.4E4 | 8.4E4 | 6.4E5 | 30 | 20 | 30 | 20 | 0.73 |
| Vz | pg/ml | 3.7E0 | 3.9E0 | 4.6E0 | 5.4E0 | 4.0E0 | 5.9E0 | 1.0E-9 | 7.6E-2 | 1.6E1 | 2.2E1 | 30 | 20 | 30 | 20 | 0.54 |
| Si | ng/ml | 1.0E0 | 1.4E0 | 2.0E0 | 2.0E0 | 3.0E0 | 1.7E0 | 8.6E-3 | 3.3E-1 | 1.0E1 | 6.0E0 | 29 | 20 | 29 | 20 | 0.61 |
| Sf | mIU/mL | 2.0E1 | 2.0E1 | 7.0E1 | 2.4E1 | 1.4E2 | 2.0E1 | 7.8E-1 | 1.7E0 | 7.2E2 | 8.3E1 | 29 | 20 | 29 | 20 | 0.49 |
| Sh | mIU/mL | 1.7E1 | 1.2E1 | 6.0E1 | 2.1E1 | 1.2E2 | 1.9E1 | 1.4E-1 | 7.8E-2 | 5.7E2 | 6.1E1 | 29 | 20 | 29 | 20 | 0.48 |
| Sj | ng/ml | 4.4E-1 | 4.4E-1 | 4.3E-1 | 4.3E-1 | 1.1E-1 | 6.0E-2 | 2.5E-1 | 3.2E-1 | 7.2E-1 | 5.1E-1 | 29 | 20 | 29 | 20 | 0.52 |
| Rc | pg/ml | 6.9E3 | 7.1E3 | 8.0E3 | 7.0E3 | 5.6E3 | 4.5E3 | 5.5E2 | 6.7E2 | 2.7E4 | 2.2E4 | 70 | 43 | 70 | 43 | 0.47 |
| Rb | pg/ml | 8.2E-1 | 1.2E0 | 2.5E0 | 4.0E0 | 3.8E0 | 9.1E0 | 1.0E-9 | 1.0E-9 | 1.6E1 | 5.6E1 | 70 | 43 | 70 | 43 | 0.54 |
| Zq | 2.6ng/ml | 1.8E2 | 3.6E2 | 2.3E2 | 3.9E2 | 1.6E2 | 1.8E2 | 1.7E1 | 1.1E2 | 5.2E2 | 9.7E2 | 29 | 19 | 29 | 19 | 0.73 |
| Zw | 2.5ng/ml | 5.3E0 | 6.5E0 | 9.8E0 | 1.3E1 | 1.2E1 | 1.8E1 | 2.4E-1 | 2.4E-1 | 5.9E1 | 6.3E1 | 30 | 20 | 30 | 20 | 0.52 |
| Zx | 2.3mU/ml | 8.8E-2 | 1.6E-1 | 2.8E-1 | 3.8E-1 | 5.3E-1 | 5.8E-1 | 3.2E-2 | 5.0E-2 | 2.9E0 | 2.1E0 | 30 | 20 | 30 | 20 | 0.61 |
| Pz | ng/ml | 3.2E3 | 5.7E3 | 5.8E3 | 6.3E3 | 6.9E3 | 4.4E3 | 1.6E1 | 1.9E2 | 7.0E4 | 2.5E4 | 178 | 88 | 178 | 88 | 0.58 |
| Qa | ng/ml | 3.3E3 | 6.7E3 | 6.3E3 | 1.2E4 | 7.6E3 | 2.5E4 | 1.5E2 | 4.2E2 | 4.2E4 | 2.2E5 | 178 | 88 | 178 | 88 | 0.65 |
| Qb | ng/ml | 1.1E2 | 1.5E2 | 2.1E2 | 3.2E2 | 3.1E2 | 5.0E2 | 7.9E-1 | 6.7E0 | 2.8E3 | 4.1E3 | 178 | 88 | 178 | 88 | 0.61 |
| Qc | ng/ml | 1.8E2 | 4.4E2 | 3.9E2 | 6.3E2 | 5.3E2 | 6.8E2 | 1.0E0 | 1.0E-9 | 3.8E3 | 4.3E3 | 178 | 88 | 178 | 88 | 0.63 |
| Qd | ng/ml | 7.9E3 | 1.4E4 | 2.7E4 | 4.1E4 | 1.5E5 | 6.8E4 | 2.4E2 | 1.7E3 | 2.0E6 | 4.3E5 | 178 | 88 | 178 | 88 | 0.68 |
| Qe | ng/ml | 7.7E2 | 1.9E3 | 2.1E3 | 2.7E3 | 7.5E3 | 2.7E3 | 7.6E0 | 8.8E0 | 9.7E4 | 1.8E4 | 178 | 88 | 178 | 88 | 0.69 |
| Jd | ng/ml | 6.9E-1 | 1.7E0 | 5.0E0 | 3.0E0 | 1.9E1 | 4.3E0 | 1.0E-9 | 1.0E-9 | 1.2E2 | 2.1E1 | 70 | 43 | 70 | 43 | 0.63 |
| Jc | ng/ml | 1.0E-9 | 1.0E-9 | 2.2E0 | 1.6E0 | 6.4E0 | 2.4E0 | 1.0E-9 | 1.0E-9 | 4.6E1 | 1.1E1 | 70 | 43 | 70 | 43 | 0.53 |
| Jf | ng/ml | 1.0E-9 | 1.0E-9 | 1.1E0 | 1.6E0 | 2.2E0 | 2.5E0 | 1.0E-9 | 1.0E-9 | 1.2E1 | 9.1E0 | 70 | 43 | 70 | 43 | 0.56 |
| Jg | ng/ml | 4.0E2 | 8.1E2 | 7.6E2 | 1.2E3 | 1.0E3 | 1.2E3 | 5.8E0 | 1.3E1 | 1.0E4 | 7.1E3 | 181 | 88 | 181 | 88 | 0.65 |
| Jh | ng/ml | 2.9E0 | 5.2E0 | 2.8E1 | 2.8E1 | 1.1E2 | 7.0E1 | 1.0E-9 | 1.0E-9 | 1.2E3 | 4.9E2 | 181 | 88 | 181 | 88 | 0.58 |
| Ji | ng/ml | 4.7E1 | 8.8E1 | 7.2E1 | 1.7E2 | 7.4E1 | 1.9E2 | 1.1E0 | 1.5E1 | 5.3E2 | 1.3E3 | 181 | 88 | 181 | 88 | 0.71 |
| Sr | pg/mL | 2.9E2 | 8.5E2 | 7.3E2 | 1.8E3 | 1.2E3 | 3.3E3 | 1.0E-9 | 1.0E-9 | 5.5E3 | 2.1E4 | 70 | 42 | 70 | 42 | 0.71 |
| Ss | pg/mL | 1.1E5 | 6.0E4 | 1.5E5 | 1.5E5 | 1.5E5 | 2.3E5 | 7.6E3 | 2.7E3 | 7.0E5 | 1.3E6 | 70 | 42 | 70 | 42 | 0.45 |
| St | pg/mL | 2.2E7 | 5.3E7 | 4.8E7 | 1.3E8 | 6.9E7 | 3.1E8 | 7.8E5 | 1.0E-9 | 4.1E8 | 1.7E9 | 68 | 43 | 68 | 43 | 0.63 |
| Wc | ng/ml | 1.0E-9 | 1.0E-9 | 4.2E-2 | 1.5E-1 | 7.5E-2 | 4.1E-1 | 1.0E-9 | 1.0E-9 | 3.0E-1 | 1.8E0 | 30 | 20 | 30 | 20 | 0.49 |
| Wd | ng/ml | 9.6E0 | 1.0E1 | 2.6E1 | 5.2E1 | 5.5E1 | 1.2E2 | 1.0E0 | 1.5E0 | 2.9E2 | 4.1E2 | 30 | 20 | 30 | 20 | 0.53 |
| We | ng/ml | 2.8E-1 | 4.9E-1 | 1.0E0 | 1.8E0 | 1.4E0 | 5.0E0 | 1.0E-9 | 1.0E-9 | 5.5E0 | 2.3E1 | 30 | 20 | 30 | 20 | 0.53 |
| Wg | ng/ml | 1.0E-9 | 1.0E-9 | 1.0E-9 | 2.7E-2 | 0.0E0 | 1.2E-1 | 1.0E-9 | 1.0E-9 | 1.0E-9 | 5.3E-1 | 30 | 20 | 30 | 20 | 0.53 |
| Wh | ng/ml | 1.2E-2 | 1.0E-2 | 4.0E-2 | 3.4E-2 | 8.2E-2 | 7.5E-2 | 1.0E-9 | 1.0E-9 | 3.5E-1 | 3.4E-1 | 30 | 20 | 30 | 20 | 0.49 |
| Wf | ng/ml | 1.0E-9 | 1.0E-9 | 1.1E-1 | 1.9E-1 | 2.5E-1 | 5.2E-1 | 1.0E-9 | 1.0E-9 | 1.2E0 | 2.3E0 | 30 | 20 | 30 | 20 | 0.51 |
| Ra | pg/ml | 1.0E-9 | 1.0E-9 | 4.1E-1 | 2.0E0 | 9.8E-1 | 9.8E0 | 1.0E-9 | 1.0E-9 | 3.8E0 | 6.4E1 | 70 | 43 | 70 | 43 | 0.50 |
| Qz | pg/ml | 9.1E0 | 1.2E1 | 5.4E1 | 4.7E1 | 9.5E1 | 7.2E1 | 1.0E-9 | 1.0E-9 | 4.5E2 | 2.8E2 | 70 | 43 | 70 | 43 | 0.54 |
| Qy | pg/ml | 3.7E-1 | 4.7E-1 | 1.2E1 | 1.8E1 | 5.9E1 | 1.1E2 | 1.0E-9 | 1.0E-9 | 4.3E2 | 7.3E2 | 70 | 43 | 70 | 43 | 0.55 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Qx | pg/ml | 1.0E-9 | 1.0E-9 | 4.3E0 | 4.2E0 | 2.3E1 | 1.7E1 | 1.0E-9 | 1.0E-9 | 1.9E2 | 1.1E2 | 70 | 43 | 70 | 43 | 0.47 |
| Qw | pg/ml | 9.0E-2 | 1.0E-9 | 3.3E0 | 2.7E0 | 1.0E1 | 1.0E1 | 1.0E-9 | 1.0E-9 | 5.6E1 | 6.6E1 | 70 | 43 | 70 | 43 | 0.47 |
| Qv | pg/ml | 1.8E4 | 1.4E4 | 2.7E4 | 2.9E4 | 4.6E4 | 5.1E4 | 1.4E3 | 1.0E-9 | 3.7E5 | 3.3E5 | 70 | 43 | 70 | 43 | 0.48 |
| Qu | pg/ml | 8.3E0 | 7.8E0 | 9.2E1 | 1.1E2 | 1.8E2 | 2.2E2 | 1.0E-9 | 1.0E-9 | 7.1E2 | 9.8E2 | 70 | 43 | 70 | 43 | 0.48 |
| Qt | pg/ml | 1.8E1 | 1.0E1 | 5.3E1 | 3.9E1 | 1.2E2 | 6.6E1 | 1.0E-9 | 1.0E-9 | 7.1E2 | 2.2E2 | 70 | 43 | 70 | 43 | 0.46 |
| Qh | ng/ml | 1.7E1 | 2.9E1 | 3.8E1 | 8.1E1 | 5.6E1 | 1.4E2 | 4.6E-1 | 4.3E-1 | 3.4E2 | 8.0E2 | 70 | 43 | 70 | 43 | 0.64 |
| Qg | ng/ml | 7.1E0 | 7.8E0 | 1.2E1 | 1.2E1 | 1.4E1 | 1.5E1 | 1.5E-1 | 3.0E-1 | 7.5E1 | 8.1E1 | 70 | 43 | 70 | 43 | 0.51 |
| Jj | ng/ml | 6.1E2 | 3.9E2 | 3.0E3 | 6.2E2 | 2.5E4 | 6.3E2 | 2.0E1 | 1.2E1 | 3.4E5 | 3.4E3 | 181 | 88 | 181 | 88 | 0.41 |
| Jk | ng/ml | 3.0E0 | 3.0E0 | 2.4E1 | 2.6E1 | 5.6E1 | 4.9E1 | 1.0E-9 | 2.4E-1 | 3.9E2 | 2.4E2 | 181 | 88 | 181 | 88 | 0.54 |
| Jl | ng/ml | 5.1E-1 | 6.4E-1 | 1.8E0 | 1.2E2 | 3.7E0 | 1.1E3 | 1.2E-3 | 7.5E-2 | 2.0E1 | 9.9E3 | 181 | 88 | 181 | 88 | 0.58 |
| Jm | ng/ml | 1.8E1 | 3.4E1 | 6.0E1 | 8.4E1 | 1.3E2 | 2.3E2 | 1.0E-9 | 1.9E-1 | 1.0E3 | 2.1E3 | 181 | 88 | 181 | 88 | 0.61 |
| Jn | pg/ml | 3.2E-1 | 5.5E-1 | 1.8E0 | 2.0E1 | 6.1E0 | 1.0E2 | 1.0E-9 | 1.0E-9 | 5.8E1 | 7.3E2 | 181 | 88 | 181 | 88 | 0.62 |
| Jo | pg/ml | 4.1E3 | 4.8E3 | 5.0E3 | 6.9E3 | 3.9E3 | 1.2E4 | 2.6E2 | 2.3E2 | 2.4E4 | 1.0E5 | 181 | 88 | 181 | 88 | 0.54 |
| Jp | pg/ml | 7.1E4 | 8.4E4 | 7.2E4 | 9.3E4 | 3.5E4 | 4.6E4 | 2.1E3 | 2.8E4 | 1.9E5 | 3.8E5 | 181 | 88 | 181 | 88 | 0.64 |
| Jq | pg/ml | 9.2E1 | 1.4E2 | 1.5E2 | 3.6E2 | 1.7E2 | 1.0E3 | 5.6E0 | 5.6E0 | 1.1E3 | 8.7E3 | 181 | 88 | 181 | 88 | 0.60 |
| Jr | pg/ml | 2.6E0 | 8.9E0 | 2.8E1 | 1.9E2 | 1.6E2 | 9.8E2 | 1.0E-9 | 1.0E-9 | 1.9E3 | 7.4E3 | 181 | 88 | 181 | 88 | 0.64 |
| Js | pg/ml | 1.4E1 | 1.9E1 | 4.5E1 | 1.4E2 | 1.5E2 | 5.4E2 | 1.0E-9 | 1.9E0 | 1.6E3 | 3.0E3 | 181 | 88 | 181 | 88 | 0.64 |
| Jt | pg/ml | 2.3E3 | 3.3E3 | 2.8E3 | 5.0E3 | 1.9E3 | 7.3E3 | 1.5E2 | 3.8E2 | 9.2E3 | 5.2E4 | 181 | 88 | 181 | 88 | 0.63 |
| Xa | pg/ml | 1.0E-9 | 6.9E0 | 9.3E0 | 9.9E1 | 1.9E1 | 2.9E2 | 1.0E-9 | 1.0E-9 | 9.6E1 | 1.2E3 | 30 | 20 | 30 | 20 | 0.64 |
| Ye | pg/ml | 1.0E-9 | 1.0E-9 | 6.8E0 | 1.4E0 | 1.8E1 | 3.5E0 | 1.0E-9 | 1.0E-9 | 6.9E1 | 1.4E1 | 30 | 20 | 30 | 20 | 0.42 |
| Tm | pg/ml | 1.0E-9 | 1.0E-9 | 3.6E0 | 1.7E0 | 5.1E0 | 3.4E0 | 1.0E-9 | 1.0E-9 | 1.8E1 | 8.4E0 | 30 | 20 | 30 | 20 | 0.41 |
| Tl | pg/ml | 1.2E-1 | 1.3E-1 | 3.0E-1 | 1.6E0 | 3.7E-1 | 5.5E0 | 1.0E-9 | 1.0E-9 | 1.2E0 | 2.5E1 | 30 | 20 | 30 | 20 | 0.54 |
| Ju | mIU/ml | 1.2E1 | 1.2E1 | 2.9E1 | 2.2E1 | 4.2E1 | 2.4E1 | 2.5E-1 | 1.7E-1 | 2.3E2 | 1.1E2 | 70 | 43 | 70 | 43 | 0.52 |
| Jv | mIU/ml | 2.4E1 | 1.5E1 | 5.2E1 | 2.6E1 | 7.7E1 | 2.9E1 | 2.4E-2 | 1.7E-2 | 4.4E2 | 1.4E2 | 70 | 43 | 70 | 43 | 0.45 |
| Jy | ng/ml | 1.6E-3 | 1.6E-3 | 1.9E-3 | 4.2E-3 | 1.0E-3 | 8.7E-3 | 2.2E-4 | 1.7E-4 | 4.4E-3 | 4.1E-2 | 70 | 43 | 70 | 43 | 0.54 |
| Kc | pg/ml | 2.2E1 | 2.6E1 | 3.6E1 | 5.3E1 | 4.2E1 | 6.8E1 | 1.0E-9 | 1.0E-9 | 1.9E2 | 3.2E2 | 69 | 46 | 69 | 46 | 0.59 |
| Kd | pg/ml | 1.0E-9 | 1.0E-9 | 1.8E2 | 1.3E3 | 7.0E2 | 5.7E3 | 1.0E-9 | 1.0E-9 | 4.8E3 | 3.8E4 | 69 | 46 | 69 | 46 | 0.57 |
| Ke | pg/ml | 1.1E4 | 1.5E4 | 1.2E4 | 3.0E4 | 7.8E3 | 4.8E4 | 6.7E2 | 4.2E3 | 3.3E4 | 3.2E5 | 69 | 46 | 69 | 46 | 0.69 |
| Kf | pg/mL | 5.9E0 | 7.1E0 | 6.4E0 | 9.6E0 | 5.1E0 | 1.3E1 | 1.0E-9 | 1.0E-9 | 2.2E1 | 7.8E1 | 69 | 46 | 69 | 46 | 0.57 |
| Kg | pg/mL | 8.2E2 | 1.1E3 | 1.5E3 | 2.9E3 | 1.6E3 | 6.5E3 | 7.7E1 | 1.3E2 | 8.1E3 | 3.6E4 | 69 | 46 | 69 | 46 | 0.56 |
| Ki | pg/ml | 5.6E1 | 7.3E1 | 6.3E1 | 8.3E1 | 5.2E1 | 6.1E1 | 1.0E-9 | 1.0E-9 | 2.9E2 | 2.5E2 | 69 | 46 | 69 | 46 | 0.63 |
| Kj | pg/ml | 7.6E2 | 9.0E2 | 1.4E3 | 1.6E3 | 1.6E3 | 2.4E3 | 6.6E1 | 3.3E1 | 8.8E3 | 1.5E4 | 69 | 46 | 69 | 46 | 0.52 |
| Kk | pg/ml | 6.8E0 | 9.4E0 | 1.1E1 | 1.8E1 | 1.5E1 | 1.8E1 | 1.0E-9 | 1.0E-9 | 8.1E1 | 6.1E1 | 69 | 46 | 69 | 46 | 0.63 |
| Kl | pg/ml | 2.1E4 | 1.7E4 | 2.9E4 | 2.7E4 | 2.6E4 | 2.7E4 | 2.4E2 | 2.3E2 | 1.1E5 | 1.3E5 | 69 | 46 | 69 | 46 | 0.47 |
| Kn | pg/ml | 1.5E1 | 5.6E1 | 5.1E1 | 2.0E2 | 7.6E1 | 7.2E2 | 1.0E-9 | 1.0E-9 | 3.4E2 | 4.9E3 | 69 | 46 | 69 | 46 | 0.62 |
| Ko | pg/ml | 3.1E2 | 4.7E2 | 4.2E2 | 7.2E2 | 4.6E2 | 8.5E2 | 1.0E-9 | 1.0E-9 | 2.0E3 | 4.1E3 | 69 | 46 | 69 | 46 | 0.63 |
| Kp | pg/ml | 3.2E2 | 3.9E2 | 3.4E2 | 6.9E2 | 2.5E2 | 1.9E3 | 1.0E-9 | 1.0E-9 | 9.4E2 | 1.3E4 | 69 | 46 | 69 | 46 | 0.58 |
| Kq | pg/ml | 3.1E2 | 4.5E2 | 3.6E2 | 4.6E3 | 3.3E2 | 2.4E4 | 1.6E0 | 6.1E1 | 2.1E3 | 1.6E5 | 66 | 44 | 66 | 44 | 0.68 |
| Kr | pg/ml | 1.0E-9 | 1.6E0 | 1.5E0 | 1.3E1 | 2.7E0 | 6.2E1 | 1.0E-9 | 1.0E-9 | 1.3E1 | 4.2E2 | 66 | 44 | 66 | 44 | 0.63 |
| Ks | pg/ml | 1.3E4 | 2.1E4 | 1.9E4 | 2.4E4 | 1.9E4 | 1.8E4 | 5.1E1 | 9.9E2 | 7.9E4 | 5.1E4 | 66 | 44 | 66 | 44 | 0.59 |
| Ps | ng/ml | 1.5E2 | 2.9E2 | 7.0E2 | 9.2E2 | 2.2E3 | 1.8E3 | 5.5E0 | 1.6E1 | 1.2E4 | 7.6E3 | 29 | 20 | 29 | 20 | 0.64 |
| Kx | ng/ml | 1.0E-9 | 4.7E-3 | 6.7E-3 | 1.3E-2 | 1.6E-2 | 2.0E-2 | 1.0E-9 | 1.0E-9 | 1.0E-1 | 7.9E-2 | 68 | 46 | 68 | 46 | 0.62 |
| Ky | ng/ml | 1.6E-1 | 2.0E-1 | 4.1E-1 | 4.9E-1 | 8.1E-1 | 8.3E-1 | 1.0E-9 | 1.0E-9 | 5.1E0 | 4.4E0 | 68 | 46 | 68 | 46 | 0.54 |
| Kz | ng/ml | 1.0E-9 | 1.0E-9 | 3.4E-3 | 4.6E-3 | 5.4E-3 | 7.0E-3 | 1.0E-9 | 1.0E-9 | 1.4E-2 | 2.5E-2 | 68 | 46 | 68 | 46 | 0.51 |
| Rz | ng/ml | 3.1E-1 | 3.4E-1 | 6.8E-1 | 9.3E-1 | 9.3E-1 | 1.7E0 | 4.6E-3 | 1.7E-2 | 3.4E0 | 7.5E0 | 29 | 20 | 29 | 20 | 0.57 |
| Ry | ng/ml | 1.6E-2 | 2.4E-2 | 1.9E-2 | 4.8E-2 | 1.9E-2 | 7.9E-2 | 1.0E-9 | 1.0E-9 | 6.5E-2 | 3.5E-1 | 29 | 20 | 29 | 20 | 0.62 |
| Rx | ng/ml | 1.0E-9 | 1.8E-5 | 1.6E-3 | 1.5E-3 | 2.6E-3 | 2.4E-3 | 1.0E-9 | 1.0E-9 | 8.6E-3 | 7.6E-3 | 29 | 20 | 29 | 20 | 0.52 |
| Ld | pg/ml | 1.0E-9 | 7.5E-1 | 3.3E0 | 5.5E0 | 9.1E0 | 9.7E0 | 1.0E-9 | 1.0E-9 | 6.9E1 | 5.0E1 | 69 | 47 | 69 | 47 | 0.58 |
| Lh | pg/ml | 1.2E4 | 2.1E4 | 2.1E4 | 4.4E4 | 2.9E4 | 7.9E4 | 1.0E-9 | 1.0E3 | 2.6E5 | 4.8E5 | 181 | 89 | 181 | 89 | 0.64 |
| Li | pg/ml | 3.1E3 | 8.6E3 | 9.4E3 | 4.3E4 | 2.9E4 | 1.1E5 | 1.3E1 | 3.7E1 | 2.9E5 | 9.2E5 | 181 | 89 | 181 | 89 | 0.71 |
| Lj | pg/ml | 2.1E3 | 6.7E3 | 1.2E4 | 3.8E4 | 4.2E4 | 7.2E4 | 1.0E-9 | 1.4E2 | 4.3E5 | 4.1E5 | 181 | 89 | 181 | 89 | 0.68 |
| Lp | pg/ml | 6.4E0 | 1.7E1 | 5.2E1 | 1.8E2 | 1.3E2 | 4.0E2 | 1.0E-9 | 1.0E-9 | 5.8E2 | 1.4E3 | 29 | 20 | 29 | 20 | 0.60 |
| Lt | pg/ml | 1.0E-9 | 1.0E-9 | 1.4E0 | 2.3E0 | 5.3E0 | 7.0E0 | 1.0E-9 | 1.0E-9 | 2.5E1 | 2.5E1 | 29 | 20 | 29 | 20 | 0.52 |

| Code | Units | Median | | Average | | Standard Deviation | | Minimum | | Maximum | | Number of Samples | | Number of Patients | | AUC |
|------|-------|--------|--------|---------|--------|--------|--------|---------|--------|---------|--------|--------|-----|--------|-----|------|
| | | NonDis | Dis | NonDis | Dis | NonDis | Dis | NonDis | Dis | NonDis | Dis | NonDis | Dis | NonDis | Dis | |
| Rv | ng/ml | 5.0E-4 | 5.4E-4 | 8.4E-4 | 2.4E-3 | 8.5E-4 | 4.5E-3 | 1.0E-9 | 1.0E-9 | 2.6E-3 | 1.6E-2 | 29 | 20 | 29 | 20 | 0.49 |
| Ru | ng/ml | 1.0E-9 | 1.0E-9 | 1.8E-2 | 3.8E-2 | 6.8E-2 | 1.1E-1 | 1.0E-9 | 1.0E-9 | 3.1E-1 | 3.5E-1 | 29 | 20 | 29 | 20 | 0.54 |
| Rt | ng/ml | 7.1E-2 | 7.8E-2 | 9.5E-2 | 5.1E-1 | 9.6E-2 | 1.6E0 | 2.2E-3 | 1.3E-3 | 3.8E-1 | 7.4E0 | 29 | 20 | 29 | 20 | 0.58 |
| Yl | pg/ml | 1.3E1 | 1.2E1 | 1.5E1 | 3.1E1 | 1.3E1 | 4.9E1 | 1.0E-9 | 1.0E-9 | 4.7E1 | 2.2E2 | 30 | 20 | 30 | 20 | 0.56 |
| Rm | ng/ml | 1.6E1 | 2.1E1 | 4.3E1 | 6.6E1 | 7.5E1 | 1.1E2 | 2.2E-1 | 3.9E-1 | 3.4E2 | 6.5E2 | 70 | 42 | 70 | 42 | 0.61 |
| Rh | ng/ml | 1.4E2 | 1.9E2 | 2.8E2 | 6.9E2 | 4.9E2 | 2.6E3 | 7.5E0 | 2.5E1 | 3.8E3 | 1.7E4 | 70 | 42 | 70 | 42 | 0.55 |
| Ri | ng/ml | 4.4E-2 | 1.0E-9 | 3.4E0 | 2.9E0 | 7.3E0 | 5.4E0 | 1.0E-9 | 1.0E-9 | 4.5E1 | 2.5E1 | 70 | 42 | 70 | 42 | 0.47 |
| Rg | ng/ml | 1.0E-9 | 1.0E-9 | 4.7E-2 | 1.7E-1 | 3.6E-1 | 5.9E-1 | 1.0E-9 | 1.0E-9 | 3.0E0 | 3.3E0 | 70 | 42 | 70 | 42 | 0.56 |
| Rj | ng/ml | 1.0E-9 | 1.0E-9 | 7.5E-1 | 7.5E0 | 1.8E0 | 4.2E1 | 1.0E-9 | 1.0E-9 | 1.1E1 | 2.7E2 | 70 | 42 | 70 | 42 | 0.52 |
| Rf | ng/ml | 3.5E-1 | 3.8E-1 | 6.6E-1 | 1.6E0 | 7.7E-1 | 3.5E0 | 2.1E-2 | 3.2E-2 | 3.6E0 | 1.7E1 | 70 | 42 | 70 | 42 | 0.55 |
| Ql | pg/ml | 2.6E0 | 7.3E0 | 1.1E1 | 1.7E1 | 1.9E1 | 3.2E1 | 1.0E-9 | 1.0E-9 | 1.1E2 | 1.8E2 | 70 | 43 | 70 | 43 | 0.56 |
| Qm | pg/ml | 4.7E-1 | 1.4E1 | 1.8E1 | 2.6E1 | 3.5E1 | 3.7E1 | 1.0E-9 | 1.0E-9 | 1.9E2 | 1.8E2 | 70 | 43 | 70 | 43 | 0.61 |
| Qn | pg/ml | 5.8E-1 | 6.1E-1 | 6.7E0 | 3.6E0 | 2.8E1 | 1.0E1 | 1.0E-9 | 1.0E-9 | 2.2E2 | 6.0E1 | 70 | 43 | 70 | 43 | 0.53 |
| Nv | pg/ml | 3.2E3 | 6.8E3 | 9.7E3 | 1.6E4 | 2.0E4 | 2.7E4 | 1.0E-9 | 3.3E2 | 1.5E5 | 1.6E5 | 181 | 90 | 181 | 90 | 0.64 |
| Nw | pg/ml | 9.0E3 | 1.5E4 | 1.2E4 | 2.3E4 | 1.8E4 | 3.2E4 | 1.9E2 | 3.0E3 | 2.1E5 | 2.2E5 | 181 | 90 | 181 | 90 | 0.70 |
| Nx | pg/ml | 2.0E2 | 2.6E2 | 4.0E2 | 6.5E2 | 6.1E2 | 7.8E2 | 1.0E-9 | 1.0E-9 | 3.5E3 | 4.1E3 | 181 | 90 | 181 | 90 | 0.63 |
| Ny | pg/ml | 4.9E0 | 1.4E1 | 1.6E2 | 8.9E1 | 1.8E3 | 3.3E2 | 1.0E-9 | 1.0E-9 | 2.5E4 | 2.8E3 | 181 | 90 | 181 | 90 | 0.66 |
| Oa | pg/ml | 1.2E2 | 3.7E2 | 3.4E2 | 8.3E2 | 5.8E2 | 1.0E3 | 1.0E-9 | 1.0E-9 | 3.0E3 | 4.5E3 | 70 | 43 | 70 | 43 | 0.69 |
| Op | pg/ml | 4.0E5 | 4.5E5 | 4.0E5 | 4.6E5 | 1.5E5 | 1.8E5 | 1.4E5 | 5.2E4 | 7.3E5 | 7.5E5 | 29 | 20 | 29 | 20 | 0.63 |
| Wn | ng/ml | 1.1E1 | 1.8E1 | 1.2E2 | 2.2E1 | 3.9E2 | 1.9E1 | 1.2E0 | 3.8E0 | 1.8E3 | 5.6E1 | 22 | 9 | 22 | 9 | 0.56 |
| Tk | ng/ml | 1.3E2 | 1.3E2 | 3.4E2 | 4.6E2 | 8.5E2 | 7.2E2 | 3.0E0 | 1.4E1 | 4.2E3 | 2.3E3 | 23 | 12 | 23 | 12 | 0.54 |
| Oe | pg/ml | 4.8E1 | 9.4E0 | 2.8E2 | 2.3E2 | 4.1E2 | 3.7E2 | 1.0E-9 | 1.0E-9 | 2.3E3 | 2.0E3 | 180 | 89 | 180 | 89 | 0.47 |
| Of | pg/ml | 2.1E2 | 9.8E1 | 5.8E3 | 7.2E3 | 2.2E4 | 2.5E4 | 1.0E-9 | 1.0E-9 | 1.9E5 | 1.7E5 | 181 | 90 | 181 | 90 | 0.45 |
| Og | pg/ml | 8.0E-2 | 6.2E-2 | 5.0E-1 | 1.3E-1 | 1.9E0 | 3.1E-1 | 1.0E-9 | 1.0E-9 | 1.9E1 | 2.5E0 | 181 | 90 | 181 | 90 | 0.43 |
| Oh | pg/ml | 2.8E0 | 4.1E0 | 1.9E1 | 2.0E2 | 1.2E2 | 1.7E3 | 1.0E-9 | 1.0E-9 | 1.4E3 | 1.6E4 | 181 | 90 | 181 | 90 | 0.56 |
| Oi | pg/ml | 2.5E0 | 2.7E0 | 5.3E0 | 5.4E0 | 7.5E0 | 6.9E0 | 1.0E-9 | 1.0E-9 | 4.2E1 | 3.1E1 | 181 | 90 | 181 | 90 | 0.53 |
| Ok | pg/ml | 3.8E2 | 6.3E2 | 4.6E2 | 9.0E2 | 3.9E2 | 1.1E3 | 1.5E1 | 6.4E1 | 2.2E3 | 7.8E3 | 181 | 90 | 181 | 90 | 0.70 |
| Om | pg/ml | 4.0E2 | 5.4E2 | 9.1E2 | 1.6E3 | 2.7E3 | 5.5E3 | 1.0E-9 | 1.0E-9 | 3.0E4 | 5.1E4 | 181 | 90 | 181 | 90 | 0.61 |
| On | pg/ml | 1.8E2 | 2.8E2 | 2.9E2 | 5.7E2 | 4.7E2 | 1.0E3 | 8.4E-1 | 2.2E1 | 4.5E3 | 8.5E3 | 181 | 90 | 181 | 90 | 0.64 |
| Or | pg/ml | 1.1E1 | 1.9E1 | 2.8E1 | 7.4E1 | 5.1E1 | 1.3E2 | 1.0E-9 | 1.0E-9 | 3.4E2 | 5.1E2 | 69 | 47 | 69 | 47 | 0.58 |
| Ow | pg/ml | 3.5E1 | 4.8E1 | 1.5E2 | 2.4E2 | 4.1E2 | 5.4E2 | 1.0E-9 | 1.0E-9 | 2.7E3 | 3.0E3 | 69 | 47 | 69 | 47 | 0.56 |
| Ou | pg/ml | 4.2E2 | 6.2E2 | 1.0E3 | 1.6E3 | 1.6E3 | 2.6E3 | 2.0E1 | 1.0E-9 | 9.8E3 | 1.1E4 | 69 | 47 | 69 | 47 | 0.56 |
| Ul | ng/ml | 1.0E-9 | 1.0E-9 | 9.9E-1 | 2.3E0 | 4.1E0 | 9.3E0 | 1.0E-9 | 1.0E-9 | 2.3E1 | 5.6E1 | 71 | 43 | 71 | 43 | 0.51 |
| Uo | ng/ml | 1.0E-9 | 1.0E-9 | 7.7E-2 | 7.1E-2 | 2.2E-1 | 1.7E-1 | 1.0E-9 | 1.0E-9 | 1.3E0 | 6.9E-1 | 71 | 43 | 71 | 43 | 0.49 |
| Uk | ng/ml | 1.0E-9 | 1.0E-9 | 8.0E-3 | 4.6E-3 | 4.0E-2 | 2.0E-2 | 1.0E-9 | 1.0E-9 | 3.2E-1 | 1.3E-1 | 71 | 43 | 71 | 43 | 0.44 |
| Um | ng/ml | 1.0E-9 | 1.0E-9 | 2.8E-1 | 1.7E-1 | 7.0E-1 | 4.5E-1 | 1.0E-9 | 1.0E-9 | 3.6E0 | 2.3E0 | 71 | 43 | 71 | 43 | 0.45 |
| Uf | ng/ml | 6.0E-2 | 9.5E-2 | 1.2E-1 | 3.0E-1 | 1.5E-1 | 7.9E-1 | 1.0E-3 | 6.8E-3 | 7.8E-1 | 5.1E0 | 71 | 43 | 71 | 43 | 0.61 |
| Uh | ng/ml | 1.8E0 | 4.5E0 | 2.8E0 | 5.5E0 | 2.6E0 | 4.3E0 | 3.6E-2 | 4.5E-1 | 1.0E1 | 1.8E1 | 71 | 43 | 71 | 43 | 0.70 |
| Un | ng/ml | 1.6E0 | 2.3E0 | 1.7E0 | 3.2E0 | 9.7E-1 | 3.9E0 | 3.4E-1 | 4.6E-1 | 4.4E0 | 2.5E1 | 71 | 43 | 71 | 43 | 0.68 |
| Ug | ng/ml | 1.1E1 | 8.5E0 | 2.2E1 | 2.2E1 | 2.6E1 | 3.2E1 | 1.5E0 | 1.2E0 | 1.4E2 | 1.6E2 | 71 | 43 | 71 | 43 | 0.46 |
| Ur | ng/ml | 1.1E-1 | 5.2E-2 | 3.5E-1 | 4.9E-1 | 6.1E-1 | 1.4E0 | 1.0E-9 | 1.0E-9 | 3.0E0 | 7.3E0 | 71 | 42 | 71 | 42 | 0.41 |
| Up | ng/ml | 1.0E-9 | 1.0E-9 | 2.3E-3 | 6.3E-2 | 7.9E-3 | 3.7E-1 | 1.0E-9 | 1.0E-9 | 5.3E-2 | 2.4E0 | 71 | 42 | 71 | 42 | 0.60 |
| Us | ng/ml | 4.2E-3 | 2.6E-3 | 1.1E-2 | 7.0E-2 | 2.0E-2 | 2.6E-1 | 1.0E-9 | 1.0E-9 | 1.2E-1 | 1.7E0 | 71 | 42 | 71 | 42 | 0.51 |
| Uv | ng/ml | 3.4E-3 | 2.5E-3 | 1.4E-2 | 1.6E-2 | 3.8E-2 | 6.3E-2 | 1.0E-9 | 1.0E-9 | 2.3E-1 | 4.1E-1 | 71 | 42 | 71 | 42 | 0.45 |
| Ut | ng/ml | 6.0E-1 | 1.2E0 | 2.6E0 | 5.1E0 | 6.9E0 | 1.1E1 | 1.0E-9 | 1.0E-9 | 5.2E1 | 6.5E1 | 71 | 42 | 71 | 42 | 0.62 |
| Uu | ng/ml | 7.2E0 | 6.1E0 | 7.9E0 | 6.7E0 | 5.4E0 | 3.9E0 | 8.3E-1 | 5.7E-1 | 2.9E1 | 1.7E1 | 71 | 42 | 71 | 42 | 0.44 |
| Uw | ng/ml | 2.0E0 | 2.8E0 | 2.6E0 | 5.1E0 | 2.3E0 | 8.3E0 | 1.0E-9 | 7.7E-1 | 7.1E0 | 3.9E1 | 30 | 20 | 30 | 20 | 0.66 |
| Vb | ng/ml | 1.2E0 | 1.1E0 | 1.1E0 | 1.2E0 | 4.5E-1 | 1.3E0 | 8.5E-2 | 2.6E-1 | 2.0E0 | 6.4E0 | 30 | 20 | 30 | 20 | 0.40 |
| Vc | ng/ml | 1.0E-9 | 1.0E-9 | 4.6E-3 | 1.0E-9 | 2.0E-2 | 0.0E0 | 1.0E-9 | 1.0E-9 | 1.1E-1 | 1.0E-9 | 30 | 20 | 30 | 20 | 0.45 |
| Uy | ng/ml | 8.6E-1 | 1.6E0 | 2.7E0 | 1.6E1 | 6.6E0 | 2.8E1 | 8.7E-2 | 2.0E-2 | 3.5E1 | 9.9E1 | 30 | 20 | 30 | 20 | 0.65 |
| Uz | ng/ml | 1.0E-9 | 1.0E-9 | 1.5E-2 | 1.7E0 | 7.9E-2 | 7.4E0 | 1.0E-9 | 1.0E-9 | 4.4E-1 | 3.3E1 | 30 | 20 | 30 | 20 | 0.53 |
| Ux | ng/ml | 1.8E2 | 1.9E2 | 1.8E2 | 2.2E2 | 1.3E2 | 1.4E2 | 4.5E0 | 2.0E1 | 4.6E2 | 4.9E2 | 30 | 20 | 30 | 20 | 0.58 |
| Va | ng/ml | 1.3E1 | 6.6E0 | 2.5E1 | 2.2E1 | 3.2E1 | 2.6E1 | 3.1E-1 | 1.2E0 | 1.2E2 | 9.6E1 | 30 | 20 | 30 | 20 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Vh | ng/ml | 8.1E-3 | 2.2E-2 | 1.7E-2 | 6.6E-2 | 2.5E-2 | 1.9E-1 | 1.0E-3 | 3.0E-3 | 1.2E-1 | 8.6E-1 | 30 | 20 | 30 | 20 | 0.73 |
| Vi | ng/ml | 4.0E-3 | 1.0E-2 | 7.5E-3 | 1.1E-1 | 7.8E-3 | 4.0E-1 | 1.0E-9 | 1.0E-9 | 2.7E-2 | 1.8E0 | 30 | 20 | 30 | 20 | 0.60 |
| Vj | ng/ml | 3.2E1 | 5.7E1 | 6.1E1 | 9.6E1 | 7.4E1 | 1.5E2 | 1.4E0 | 8.3E0 | 3.4E2 | 6.5E2 | 30 | 19 | 30 | 19 | 0.59 |
| Vp | ng/ml | 1.0E-9 | 1.0E-9 | 1.3E-1 | 1.3E0 | 5.4E-1 | 7.5E0 | 1.0E-9 | 1.0E-9 | 3.9E0 | 4.9E1 | 71 | 43 | 71 | 43 | 0.55 |
| Vt | ng/ml | 5.6E0 | 9.3E0 | 6.5E0 | 1.6E1 | 5.3E0 | 2.4E1 | 5.6E-1 | 1.9E0 | 3.2E1 | 1.6E2 | 71 | 43 | 71 | 43 | 0.72 |
| Vu | ng/ml | 1.0E-9 | 4.5E-1 | 1.4E0 | 2.4E0 | 3.0E0 | 4.5E0 | 1.0E-9 | 1.0E-9 | 1.6E1 | 2.2E1 | 69 | 41 | 69 | 41 | 0.59 |
| Vq | ng/ml | 7.5E1 | 3.5E2 | 5.0E2 | 1.2E3 | 8.9E2 | 2.4E3 | 9.2E-1 | 8.9E0 | 5.0E3 | 1.2E4 | 59 | 31 | 59 | 31 | 0.64 |
| Vo | ng/ml | 2.5E1 | 2.6E1 | 2.4E1 | 2.5E1 | 4.9E0 | 6.7E0 | 9.7E0 | 4.9E0 | 3.5E1 | 4.8E1 | 71 | 43 | 71 | 43 | 0.54 |
| Vs | ng/ml | 1.0E-9 | 1.0E-9 | 4.8E0 | 1.5E1 | 1.3E1 | 7.1E1 | 1.0E-9 | 1.0E-9 | 8.9E1 | 4.5E2 | 70 | 41 | 70 | 41 | 0.47 |
| Vv | ng/ml | 2.8E0 | 3.7E0 | 6.4E0 | 5.7E0 | 1.1E1 | 7.9E0 | 1.0E-9 | 1.0E-9 | 8.4E1 | 4.4E1 | 71 | 42 | 71 | 42 | 0.48 |
| Vw | ng/ml | 3.4E1 | 4.3E1 | 3.1E1 | 4.1E1 | 1.8E1 | 1.9E1 | 2.5E0 | 4.4E0 | 6.7E1 | 6.9E1 | 30 | 20 | 30 | 20 | 0.66 |
| Oy | pg/ml | 5.3E-1 | 4.8E-1 | 8.9E0 | 3.7E0 | 4.1E1 | 1.2E1 | 1.0E-9 | 1.0E-9 | 4.0E2 | 9.9E1 | 181 | 89 | 181 | 89 | 0.45 |
| Oz | pg/ml | 4.9E-2 | 1.0E-9 | 2.8E-1 | 8.0E-1 | 3.9E-1 | 4.2E0 | 1.0E-9 | 1.0E-9 | 2.1E0 | 2.9E1 | 181 | 89 | 181 | 89 | 0.41 |
| Pa | pg/ml | 3.9E-1 | 5.3E-1 | 1.5E0 | 5.5E0 | 7.1E-1 | 2.6E1 | 1.0E-9 | 1.0E-9 | 8.6E1 | 2.3E2 | 181 | 89 | 181 | 89 | 0.55 |
| Pb | pg/ml | 1.0E-9 | 1.0E-9 | 2.8E0 | 8.6E-1 | 3.6E1 | 4.5E0 | 1.0E-9 | 1.0E-9 | 4.9E2 | 3.2E1 | 181 | 89 | 181 | 89 | 0.45 |
| Pc | pg/ml | 2.0E-1 | 1.0E-9 | 4.8E-1 | 4.4E0 | 1.1E0 | 3.5E1 | 1.0E-9 | 1.0E-9 | 1.4E1 | 3.3E2 | 181 | 89 | 181 | 89 | 0.43 |
| Pd | pg/ml | 1.7E0 | 2.3E0 | 4.0E0 | 1.6E1 | 8.8E0 | 9.0E1 | 1.0E-9 | 1.0E-9 | 9.4E1 | 8.4E2 | 181 | 89 | 181 | 89 | 0.54 |
| Pe | pg/ml | 2.0E1 | 4.4E1 | 9.4E1 | 5.0E2 | 4.2E2 | 1.9E3 | 1.0E-9 | 1.0E-9 | 4.7E3 | 1.5E4 | 181 | 89 | 181 | 89 | 0.65 |
| Pf | pg/ml | 1.6E0 | 3.4E0 | 8.2E0 | 3.7E1 | 3.2E1 | 1.7E2 | 1.0E-9 | 1.0E-9 | 3.3E2 | 1.5E3 | 181 | 89 | 181 | 89 | 0.62 |
| Pg | pg/ml | 4.0E0 | 8.8E0 | 6.6E1 | 1.9E2 | 4.0E2 | 8.7E2 | 1.0E-9 | 1.0E-9 | 4.2E3 | 7.7E3 | 181 | 89 | 181 | 89 | 0.59 |
| Ph | ng/ml | 1.4E-1 | 1.8E-1 | 3.3E-1 | 4.9E-1 | 4.6E-1 | 9.3E-1 | 1.0E-9 | 1.0E-9 | 2.2E0 | 5.4E0 | 69 | 47 | 69 | 47 | 0.54 |
| Pi | ng/ml | 1.9E-1 | 2.2E-1 | 3.0E-1 | 2.1E0 | 4.5E-1 | 1.2E1 | 1.0E-9 | 1.0E-9 | 3.2E0 | 8.2E1 | 69 | 47 | 69 | 47 | 0.56 |
| Pj | ng/mL | 4.4E0 | 7.3E0 | 4.9E0 | 8.4E0 | 3.3E0 | 5.8E0 | 4.0E-1 | 1.5E0 | 1.6E1 | 3.1E1 | 69 | 47 | 69 | 47 | 0.71 |
| Pk | ng/ml | 7.7E-3 | 1.3E-2 | 1.2E-2 | 5.0E-2 | 3.1E-2 | 2.2E-1 | 1.0E-9 | 1.0E-9 | 2.5E-1 | 1.5E0 | 69 | 47 | 69 | 47 | 0.68 |
| aA | mg/dL | 8.8E-1 | 9.2E-1 | 1.0E0 | 1.2E0 | 4.9E-1 | 7.8E-1 | 3.0E-1 | 4.0E-1 | 4.2E0 | 4.7E0 | 294 | 111 | 294 | 111 | 0.55 |
| aC | mg/mL | 2.3E0 | 2.0E0 | 2.7E0 | 2.2E0 | 1.2E0 | 1.0E0 | 1.1E0 | 7.5E-1 | 7.2E0 | 5.5E0 | 86 | 56 | 86 | 56 | 0.37 |
| aD | ug/mL | 2.9E0 | 3.5E0 | 4.3E0 | 5.0E0 | 4.2E0 | 4.1E0 | 8.5E-1 | 7.5E-1 | 3.1E1 | 2.1E1 | 86 | 56 | 86 | 56 | 0.55 |
| aE | mg/mL | 5.6E-1 | 5.9E-1 | 5.9E-1 | 6.0E-1 | 1.6E-1 | 2.0E-1 | 2.8E-1 | 1.8E-1 | 1.1E0 | 1.2E0 | 86 | 56 | 86 | 56 | 0.52 |
| aF | ng/mL | 2.2E0 | 2.2E0 | 6.2E0 | 3.7E0 | 9.7E0 | 3.9E0 | 4.3E-3 | 4.3E-3 | 5.0E1 | 1.9E1 | 86 | 56 | 86 | 56 | 0.46 |
| aG | mg/mL | 1.3E-1 | 1.4E-1 | 1.5E-1 | 1.6E-1 | 7.9E-2 | 8.4E-2 | 5.0E-2 | 5.1E-2 | 4.2E-1 | 4.8E-1 | 86 | 56 | 86 | 56 | 0.50 |
| aH | ug/mL | 6.5E1 | 7.1E1 | 7.3E1 | 7.8E1 | 3.5E1 | 4.3E1 | 1.5E1 | 1.1E1 | 2.0E2 | 1.8E2 | 86 | 56 | 86 | 56 | 0.52 |
| aI | ug/mL | 1.7E2 | 1.6E2 | 1.7E2 | 1.7E2 | 5.7E1 | 6.5E1 | 6.1E1 | 4.7E1 | 3.3E2 | 3.4E2 | 86 | 56 | 86 | 56 | 0.46 |
| aJ | ug/mL | 2.5E0 | 2.5E0 | 3.1E0 | 3.5E0 | 2.3E0 | 2.4E0 | 9.5E-1 | 8.2E-1 | 1.4E1 | 1.1E1 | 86 | 56 | 86 | 56 | 0.56 |
| aK | ng/mL | 1.4E0 | 1.2E0 | 2.0E0 | 1.8E0 | 1.8E0 | 2.0E0 | 6.9E-2 | 2.9E-4 | 7.5E0 | 1.0E1 | 86 | 56 | 86 | 56 | 0.45 |
| aL | mg/mL | 7.3E-1 | 7.2E-1 | 7.5E-1 | 7.5E-1 | 2.2E-1 | 2.6E-1 | 2.2E-1 | 2.7E-1 | 1.3E0 | 1.7E0 | 86 | 56 | 86 | 56 | 0.49 |
| aM | U/mL | 1.4E1 | 2.3E1 | 2.8E1 | 6.1E1 | 4.8E1 | 1.2E2 | 4.2E-2 | 4.2E-2 | 3.5E2 | 8.2E2 | 86 | 56 | 86 | 56 | 0.64 |
| aN | U/mL | 1.2E1 | 2.0E1 | 1.9E1 | 4.0E1 | 2.3E1 | 6.6E1 | 2.5E-3 | 2.5E-3 | 1.3E2 | 3.8E2 | 86 | 56 | 86 | 56 | 0.66 |
| aO | pg/mL | 7.4E1 | 4.8E1 | 5.4E2 | 3.2E2 | 1.2E3 | 6.1E2 | 6.0E-2 | 6.0E-2 | 6.6E3 | 2.4E3 | 86 | 56 | 86 | 56 | 0.47 |
| aP | ng/mL | 1.5E0 | 1.7E0 | 2.0E0 | 2.2E0 | 1.3E0 | 1.4E0 | 5.4E-1 | 6.8E-1 | 6.5E0 | 6.6E0 | 86 | 56 | 86 | 56 | 0.56 |
| aQ | ng/mL | 2.3E-1 | 2.5E-1 | 3.1E-1 | 3.8E-1 | 2.5E-1 | 4.0E-1 | 2.0E-4 | 2.0E-4 | 1.1E0 | 2.0E0 | 86 | 56 | 86 | 56 | 0.53 |
| aR | ng/mL | 1.8E0 | 1.8E0 | 3.4E0 | 2.7E0 | 4.9E0 | 2.7E0 | 2.6E-1 | 5.6E-1 | 3.4E1 | 1.7E1 | 86 | 56 | 86 | 56 | 0.51 |
| aS | ng/mL | 3.7E-1 | 5.1E-1 | 1.2E0 | 1.0E0 | 3.6E0 | 1.2E0 | 4.2E-3 | 4.2E-3 | 3.3E1 | 6.2E0 | 86 | 56 | 86 | 56 | 0.56 |
| aU | pg/mL | 7.2E1 | 6.1E1 | 9.9E1 | 1.0E2 | 9.5E1 | 1.2E2 | 6.5E0 | 7.4E-2 | 5.1E2 | 7.0E2 | 86 | 56 | 86 | 56 | 0.47 |
| aV | ng/mL | 6.2E-1 | 4.6E-1 | 8.0E-1 | 1.4E0 | 7.2E-1 | 4.3E0 | 5.9E-2 | 7.6E-4 | 4.0E0 | 3.3E1 | 86 | 56 | 86 | 56 | 0.48 |
| aW | pg/mL | 2.1E1 | 1.9E1 | 2.2E1 | 2.6E1 | 1.9E1 | 5.4E1 | 7.2E-2 | 7.2E-2 | 1.7E2 | 4.2E2 | 86 | 56 | 86 | 56 | 0.45 |
| aX | ng/mL | 8.1E0 | 6.8E0 | 1.1E1 | 1.2E1 | 9.9E0 | 1.7E1 | 3.0E-1 | 6.2E-1 | 5.4E1 | 1.1E2 | 86 | 56 | 86 | 56 | 0.44 |
| aY | pg/mL | 5.3E1 | 5.2E1 | 7.0E1 | 8.5E1 | 5.8E1 | 1.7E2 | 4.1E-1 | 4.1E-1 | 3.1E2 | 1.2E3 | 86 | 56 | 86 | 56 | 0.48 |
| aZ | pg/mL | 2.2E2 | 2.4E2 | 6.9E2 | 5.5E2 | 1.5E3 | 8.8E2 | 1.7E0 | 1.7E0 | 1.2E4 | 4.7E3 | 86 | 56 | 86 | 56 | 0.50 |
| bA | ng/mL | 1.3E1 | 2.3E1 | 4.3E1 | 1.1E2 | 7.2E1 | 2.1E2 | 3.0E-2 | 3.0E-2 | 4.1E2 | 9.4E2 | 86 | 56 | 86 | 56 | 0.60 |
| bB | ng/mL | 2.8E2 | 2.7E2 | 2.9E2 | 2.8E2 | 1.5E2 | 1.8E2 | 5.7E1 | 1.2E1 | 7.4E2 | 8.1E2 | 86 | 56 | 86 | 56 | 0.47 |
| bC | ng/mL | 3.4E2 | 3.0E2 | 5.3E2 | 7.1E2 | 6.2E2 | 1.0E3 | 2.7E1 | 3.5E1 | 4.0E3 | 4.7E3 | 86 | 56 | 86 | 56 | 0.49 |
| bE | mg/mL | 5.2E0 | 4.5E0 | 5.4E0 | 4.9E0 | 1.8E0 | 2.0E0 | 1.9E0 | 1.3E0 | 1.2E1 | 1.1E1 | 86 | 56 | 86 | 56 | 0.41 |
| bF | pg/mL | 3.6E1 | 3.6E1 | 3.7E2 | 4.5E2 | 1.4E3 | 1.6E3 | 5.0E-2 | 2.5E0 | 1.1E4 | 1.0E4 | 86 | 56 | 86 | 56 | 0.49 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bG | ng/mL | 1.7E0 | 2.3E0 | 3.0E0 | 4.0E0 | 4.4E0 | 5.2E0 | 1.1E-1 | 2.4E-1 | 2.6E1 | 3.0E1 | 86 | 56 | 86 | 56 | 0.56 |
| bH | pg/mL | 5.7E-1 | 5.7E-1 | 6.8E0 | 7.2E0 | 3.0E1 | 1.7E1 | 5.7E-1 | 5.7E-1 | 2.8E2 | 1.2E2 | 86 | 56 | 86 | 56 | 0.54 |
| bI | ng/mL | 4.0E-3 | 4.0E-3 | 9.5E-2 | 1.1E-1 | 2.0E-1 | 2.2E-1 | 4.0E-3 | 4.0E-3 | 9.8E-1 | 8.8E-1 | 86 | 56 | 86 | 56 | 0.49 |
| bJ | mg/mL | 1.8E0 | 1.7E0 | 2.1E0 | 2.1E0 | 1.6E0 | 2.1E0 | 2.5E-4 | 2.5E-4 | 6.6E0 | 1.1E1 | 86 | 56 | 86 | 56 | 0.47 |
| bL | pg/mL | 4.3E0 | 3.0E0 | 9.5E0 | 8.2E0 | 1.1E1 | 1.0E1 | 4.6E-2 | 4.6E-2 | 4.9E1 | 3.5E1 | 86 | 56 | 86 | 56 | 0.46 |
| bM | mg/mL | 1.7E0 | 2.2E0 | 1.9E0 | 2.7E0 | 1.3E0 | 1.7E0 | 2.4E-1 | 1.8E-2 | 8.6E0 | 7.9E0 | 86 | 56 | 86 | 56 | 0.65 |
| bN | ng/mL | 3.6E1 | 3.6E1 | 1.2E2 | 1.2E2 | 2.6E2 | 3.0E2 | 1.4E-1 | 5.9E-1 | 1.9E3 | 1.9E3 | 86 | 56 | 86 | 56 | 0.47 |
| bO | ng/mL | 4.0E-2 | 4.0E-2 | 8.2E0 | 8.9E0 | 1.5E1 | 2.3E1 | 4.0E-2 | 4.0E-2 | 6.4E1 | 1.3E2 | 86 | 56 | 86 | 56 | 0.44 |
| bP | mg/mL | 4.7E-1 | 5.2E-1 | 6.7E-1 | 7.5E-1 | 5.3E-1 | 8.3E-1 | 8.2E-2 | 9.2E-2 | 2.5E0 | 4.8E0 | 86 | 56 | 86 | 56 | 0.51 |
| bQ | pg/mL | 2.1E1 | 2.5E1 | 6.9E1 | 2.8E2 | 2.6E2 | 1.8E3 | 3.5E0 | 1.5E-1 | 2.4E3 | 1.3E4 | 86 | 56 | 86 | 56 | 0.54 |
| bR | ng/mL | 1.2E-2 | 1.2E-2 | 1.2E-1 | 2.3E-1 | 3.8E-1 | 1.2E0 | 1.2E-2 | 1.2E-2 | 3.4E0 | 8.7E0 | 86 | 56 | 86 | 56 | 0.52 |
| bS | pg/mL | 9.4E-1 | 9.4E-1 | 9.1E0 | 1.1E1 | 3.9E1 | 5.3E1 | 9.4E-1 | 9.4E-1 | 3.4E2 | 3.9E2 | 86 | 56 | 86 | 56 | 0.48 |
| bU | ng/mL | 6.9E-2 | 5.7E-2 | 1.6E-1 | 2.5E-1 | 2.5E-1 | 8.8E-1 | 1.3E-2 | 1.3E-2 | 1.8E0 | 6.6E0 | 86 | 56 | 86 | 56 | 0.50 |
| bV | pg/mL | 4.3E2 | 5.3E2 | 5.3E2 | 6.3E2 | 2.8E2 | 3.9E2 | 1.8E2 | 2.3E2 | 1.6E3 | 2.2E3 | 86 | 56 | 86 | 56 | 0.59 |
| bW | pg/mL | 3.3E2 | 3.2E2 | 4.3E2 | 5.7E2 | 3.6E2 | 8.5E2 | 8.4E1 | 1.3E2 | 2.2E3 | 4.8E3 | 86 | 56 | 86 | 56 | 0.53 |
| bX | ng/mL | 7.0E-4 | 1.5E-3 | 2.5E-3 | 2.5E-3 | 3.0E-3 | 2.8E-3 | 2.5E-5 | 2.5E-5 | 1.2E-2 | 8.4E-3 | 86 | 56 | 86 | 56 | 0.50 |
| bZ | pg/mL | 2.8E2 | 3.2E2 | 1.8E3 | 2.6E3 | 6.0E3 | 9.5E3 | 1.5E-1 | 3.5E1 | 4.4E4 | 5.8E4 | 86 | 56 | 86 | 56 | 0.52 |
| cA | pg/mL | 6.0E-1 | 6.0E-1 | 1.6E0 | 8.6E0 | 3.1E0 | 5.0E1 | 6.0E-1 | 6.0E-1 | 1.6E1 | 3.7E2 | 86 | 56 | 86 | 56 | 0.51 |
| cB | ng/mL | 3.7E-2 | 5.2E-2 | 5.5E-2 | 8.3E-2 | 6.0E-2 | 1.0E-1 | 1.7E-3 | 1.7E-3 | 3.1E-1 | 4.3E-1 | 86 | 56 | 86 | 56 | 0.54 |
| cC | pg/mL | 4.6E1 | 3.4E1 | 4.9E1 | 4.3E1 | 4.7E1 | 6.2E1 | 1.0E0 | 1.0E0 | 3.7E2 | 4.5E2 | 86 | 56 | 86 | 56 | 0.43 |
| cD | pg/mL | 6.1E0 | 4.1E0 | 1.5E1 | 1.4E1 | 5.5E1 | 4.4E1 | 3.3E-1 | 3.3E-1 | 4.8E2 | 2.9E2 | 86 | 56 | 86 | 56 | 0.41 |
| cE | pg/mL | 6.7E1 | 6.7E1 | 2.9E2 | 2.5E2 | 6.6E2 | 6.0E2 | 1.8E0 | 1.2E-1 | 3.1E3 | 3.8E3 | 86 | 56 | 86 | 56 | 0.53 |
| cF | pg/mL | 5.3E-1 | 5.3E-1 | 1.4E1 | 1.6E1 | 2.7E1 | 3.8E1 | 5.3E-1 | 5.3E-1 | 1.4E2 | 2.7E2 | 86 | 56 | 86 | 56 | 0.52 |
| cG | pg/mL | 5.2E1 | 6.3E1 | 1.1E2 | 3.0E2 | 1.7E2 | 1.4E3 | 1.5E1 | 7.8E0 | 1.1E3 | 1.0E4 | 86 | 56 | 86 | 56 | 0.55 |
| cH | uIU/mL | 3.1E0 | 3.3E0 | 7.3E0 | 9.2E0 | 1.8E1 | 1.8E1 | 8.6E-3 | 8.6E-3 | 1.6E2 | 1.2E2 | 86 | 56 | 86 | 56 | 0.53 |
| cI | ng/mL | 5.0E0 | 6.6E0 | 1.0E1 | 1.9E1 | 1.5E1 | 2.7E1 | 3.2E-2 | 3.1E-1 | 1.0E2 | 1.2E2 | 86 | 56 | 86 | 56 | 0.56 |
| cJ | ug/mL | 6.5E1 | 5.5E1 | 9.3E1 | 9.7E1 | 1.0E2 | 1.1E2 | 6.9E0 | 7.2E0 | 6.4E2 | 6.2E2 | 86 | 56 | 86 | 56 | 0.51 |
| cK | ng/mL | 3.8E-3 | 3.8E-3 | 1.1E-2 | 3.7E-2 | 2.5E-2 | 2.0E-1 | 3.8E-3 | 3.8E-3 | 1.3E-1 | 1.5E0 | 86 | 56 | 86 | 56 | 0.51 |
| cL | pg/mL | 2.2E2 | 1.9E2 | 5.3E2 | 6.6E2 | 1.2E3 | 3.2E3 | 3.6E1 | 3.1E1 | 7.4E3 | 2.4E4 | 86 | 56 | 86 | 56 | 0.47 |
| cM | pg/mL | 2.7E2 | 2.7E2 | 3.1E2 | 2.6E2 | 1.9E2 | 1.3E2 | 6.0E1 | 4.2E1 | 1.1E3 | 6.7E2 | 86 | 56 | 86 | 56 | 0.45 |
| cN | pg/mL | 1.2E2 | 1.2E2 | 1.3E2 | 1.4E2 | 5.6E1 | 1.3E2 | 3.8E1 | 6.3E1 | 3.2E2 | 1.1E3 | 86 | 56 | 86 | 56 | 0.51 |
| cO | pg/mL | 2.0E2 | 2.1E2 | 3.3E2 | 6.0E2 | 3.8E2 | 2.6E3 | 5.4E1 | 8.2E1 | 2.4E3 | 1.9E4 | 86 | 56 | 86 | 56 | 0.50 |
| cP | ng/mL | 2.5E3 | 2.4E3 | 2.6E3 | 2.6E3 | 1.0E3 | 9.6E2 | 6.2E2 | 1.0E3 | 5.6E3 | 4.7E3 | 86 | 56 | 86 | 56 | 0.50 |
| cQ | ng/mL | 4.9E-2 | 5.5E-2 | 1.2E-1 | 1.4E-1 | 1.9E-1 | 2.5E-1 | 2.0E-3 | 2.0E-3 | 1.2E0 | 1.3E0 | 86 | 56 | 86 | 56 | 0.50 |
| cR | ng/mL | 3.0E2 | 3.0E2 | 5.2E2 | 6.4E2 | 6.6E2 | 1.1E3 | 3.6E1 | 2.0E1 | 4.8E3 | 7.7E3 | 86 | 56 | 86 | 56 | 0.51 |
| cS | ng/mL | 2.4E2 | 2.9E2 | 4.0E2 | 4.0E2 | 4.4E2 | 3.9E2 | 8.0E1 | 9.7E1 | 2.5E3 | 2.4E3 | 86 | 56 | 86 | 56 | 0.54 |
| cT | ng/mL | 5.2E1 | 6.5E1 | 1.3E2 | 2.3E2 | 2.1E2 | 4.2E2 | 5.1E0 | 4.2E0 | 1.3E3 | 2.1E3 | 86 | 56 | 86 | 56 | 0.56 |
| cU | ng/mL | 5.6E1 | 6.6E1 | 8.2E1 | 1.3E2 | 9.4E1 | 2.2E2 | 6.2E0 | 1.4E1 | 7.7E2 | 1.6E3 | 86 | 56 | 86 | 56 | 0.58 |
| cV | ng/mL | 1.9E-1 | 2.2E-1 | 1.0E0 | 5.9E-1 | 5.1E0 | 1.3E0 | 3.4E-2 | 3.6E-2 | 4.7E1 | 8.7E0 | 86 | 56 | 86 | 56 | 0.52 |
| cW | mIU/mL | 4.8E-2 | 4.5E-2 | 1.1E-1 | 6.7E-2 | 4.8E-1 | 6.4E-2 | 4.8E-3 | 4.8E-3 | 4.5E0 | 3.9E-1 | 86 | 56 | 86 | 56 | 0.51 |
| cX | ng/mL | 1.4E-1 | 7.6E-2 | 2.5E0 | 1.7E0 | 6.8E0 | 5.0E0 | 2.3E-4 | 2.3E-4 | 2.8E1 | 2.8E1 | 86 | 56 | 86 | 56 | 0.52 |
| cY | ng/mL | 7.6E0 | 7.1E0 | 1.0E1 | 1.2E1 | 9.0E0 | 1.4E1 | 2.2E-1 | 1.7E-1 | 4.1E1 | 6.1E1 | 86 | 56 | 86 | 56 | 0.49 |
| cZ | ug/mL | 1.2E1 | 1.2E1 | 1.4E1 | 1.4E1 | 5.3E0 | 6.9E0 | 6.0E0 | 2.8E0 | 2.9E1 | 3.0E1 | 86 | 56 | 86 | 56 | 0.46 |
| dA | pg/mL | 2.9E2 | 3.3E2 | 3.2E2 | 4.7E2 | 1.3E2 | 7.5E2 | 1.0E2 | 1.1E2 | 9.4E2 | 5.8E3 | 86 | 56 | 86 | 56 | 0.57 |
| dB | ug/mL | 1.9E1 | 1.7E1 | 2.1E1 | 1.5E1 | 2.7E1 | 9.7E0 | 2.1E0 | 2.1E0 | 2.5E2 | 3.0E1 | 86 | 56 | 86 | 56 | 0.41 |
| dC | nmol/L | 3.5E1 | 3.2E1 | 3.9E1 | 3.6E1 | 1.9E1 | 1.4E1 | 1.0E1 | 7.8E0 | 1.4E2 | 7.0E1 | 86 | 56 | 86 | 56 | 0.48 |
| dD | ug/mL | 3.4E1 | 3.2E1 | 3.5E1 | 3.2E1 | 9.9E0 | 1.1E1 | 1.4E1 | 1.4E1 | 7.4E1 | 6.0E1 | 86 | 56 | 86 | 56 | 0.41 |
| dE | ng/mL | 5.2E-1 | 3.4E-1 | 5.7E-1 | 4.9E-1 | 5.5E-1 | 5.8E-1 | 8.4E-3 | 8.4E-3 | 2.7E0 | 2.4E0 | 86 | 56 | 86 | 56 | 0.44 |
| dF | ng/mL | 2.5E2 | 2.8E2 | 3.3E2 | 3.9E2 | 2.4E2 | 2.9E2 | 7.5E1 | 7.5E1 | 1.2E3 | 1.3E3 | 86 | 56 | 86 | 56 | 0.57 |
| dG | ng/mL | 1.3E1 | 1.2E1 | 1.6E1 | 1.9E1 | 1.3E1 | 2.6E1 | 3.2E0 | 3.0E0 | 9.7E1 | 1.8E2 | 86 | 56 | 86 | 56 | 0.53 |
| dH | pg/mL | 7.3E0 | 8.8E0 | 1.8E1 | 2.3E1 | 4.1E1 | 8.9E1 | 4.0E-2 | 4.0E-2 | 3.1E2 | 6.7E2 | 86 | 56 | 86 | 56 | 0.57 |
| dI | pg/mL | 4.6E-1 | 4.6E-1 | 1.6E0 | 7.5E0 | 4.1E0 | 4.4E1 | 4.6E-1 | 4.6E-1 | 3.4E1 | 3.3E2 | 86 | 56 | 86 | 56 | 0.56 |
| dJ | ng/mL | 1.8E0 | 2.2E0 | 2.1E0 | 2.2E0 | 1.1E0 | 1.2E0 | 3.2E-2 | 3.2E-2 | 5.1E0 | 4.8E0 | 86 | 56 | 86 | 56 | 0.54 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| dK | uIU/mL | 1.5E0 | 1.0E0 | 2.6E0 | 1.9E0 | 4.7E0 | 2.1E0 | 2.8E-4 | 2.9E-2 | 3.9E1 | 1.1E1 | 86 | 56 | 86 | 56 | 0.43 |
| dL | ng/mL | 8.5E2 | 9.0E2 | 9.7E2 | 1.2E3 | 4.9E2 | 8.0E2 | 3.4E2 | 2.8E2 | 3.4E3 | 4.8E3 | 86 | 56 | 86 | 56 | 0.58 |
| dM | pg/mL | 9.5E2 | 1.0E3 | 1.3E3 | 1.5E3 | 1.7E3 | 1.6E3 | 3.9E2 | 3.7E2 | 1.5E4 | 9.6E3 | 86 | 56 | 86 | 56 | 0.56 |
| dN | ug/mL | 9.8E1 | 1.0E2 | 1.0E2 | 1.1E2 | 3.7E1 | 4.9E1 | 2.5E1 | 2.4E1 | 2.2E2 | 3.3E2 | 86 | 56 | 86 | 56 | 0.54 |
| dR | pg/ml | 1.4E3 | 1.2E3 | 1.9E3 | 2.1E3 | 1.7E3 | 2.5E3 | 1.4E2 | 1.3E2 | 7.8E3 | 9.8E3 | 64 | 48 | 64 | 48 | 0.45 |
| dU | pg/ml | 7.3E3 | 1.9E4 | 8.7E3 | 2.3E4 | 8.1E3 | 2.1E4 | 1.7E3 | 6.9E2 | 3.5E4 | 8.1E4 | 15 | 14 | 15 | 14 | 0.76 |
| dX | ng/ml | 5.2E-2 | 8.2E-2 | 1.6E-1 | 9.5E-2 | 2.6E-1 | 1.1E-1 | 2.6E-3 | 2.6E-3 | 1.2E0 | 4.2E-1 | 27 | 18 | 27 | 18 | 0.47 |
| dW | ng/ml | 1.2E-1 | 1.7E-1 | 2.4E-1 | 2.2E-1 | 2.7E-1 | 1.7E-1 | 6.4E-2 | 6.8E-2 | 8.0E-1 | 5.9E-1 | 9 | 8 | 9 | 8 | 0.60 |
| eF | ng/ml | 4.4E0 | 4.0E0 | 5.1E0 | 4.6E0 | 2.4E0 | 2.2E0 | 2.0E0 | 2.0E0 | 1.2E1 | 1.5E1 | 64 | 48 | 64 | 48 | 0.43 |
| eC | pg/ml | 3.1E2 | 2.8E2 | 3.9E2 | 3.5E2 | 2.9E2 | 3.1E2 | 1.1E2 | 1.9E1 | 1.6E3 | 2.0E3 | 58 | 43 | 58 | 43 | 0.44 |
| eD | pg/ml | 2.2E2 | 1.9E2 | 6.9E2 | 6.5E2 | 1.4E3 | 1.4E3 | 5.2E-1 | 5.2E-1 | 6.8E3 | 7.0E3 | 52 | 30 | 52 | 30 | 0.48 |
| eO | ng/ml | 4.6E1 | 9.4E1 | 2.5E2 | 1.0E2 | 3.5E2 | 4.6E1 | 2.0E1 | 4.3E1 | 9.6E2 | 1.7E2 | 9 | 8 | 9 | 8 | 0.64 |
| eM | ng/ml | 3.2E0 | 2.5E0 | 4.2E0 | 5.0E0 | 3.4E0 | 6.5E0 | 7.6E-1 | 6.9E-1 | 1.7E1 | 2.6E1 | 37 | 25 | 37 | 25 | 0.45 |
| eP | ng/ml | 3.1E-2 | 3.7E-3 | 8.7E-1 | 1.7E0 | 1.8E0 | 6.5E0 | 3.7E-3 | 3.7E-3 | 8.6E0 | 2.8E1 | 27 | 18 | 27 | 18 | 0.37 |
| cT | ng/ml | 2.7E2 | 2.9E2 | 7.2E2 | 7.7E2 | 8.1E2 | 9.6E2 | 1.0E2 | 7.1E1 | 2.9E3 | 2.9E3 | 31 | 18 | 31 | 18 | 0.52 |
| eQ | pg/ml | 1.0E0 | 1.0E0 | 6.8E1 | 3.5E1 | 1.4E2 | 5.6E1 | 1.0E0 | 1.0E0 | 4.7E2 | 1.5E2 | 15 | 14 | 15 | 14 | 0.52 |
| eW | U/ml | 1.1E-2 | 6.7E-3 | 2.3E-1 | 5.2E-2 | 5.1E-1 | 8.4E-2 | 6.7E-3 | 6.7E-3 | 1.6E0 | 1.9E-1 | 9 | 8 | 9 | 8 | 0.36 |
| fA | ng/ml | 2.3E2 | 1.6E2 | 3.5E2 | 5.2E2 | 4.2E2 | 5.4E2 | 3.9E1 | 4.0E1 | 1.5E3 | 1.4E3 | 15 | 12 | 15 | 12 | 0.53 |
| eZ | ng/ml | 4.3E1 | 5.8E1 | 5.5E1 | 6.2E1 | 2.7E1 | 2.4E1 | 1.8E1 | 2.3E1 | 1.2E2 | 1.1E2 | 31 | 18 | 31 | 18 | 0.60 |
| fB | ng/ml | 5.7E2 | 6.6E2 | 6.7E2 | 6.9E2 | 2.6E2 | 2.9E2 | 3.1E2 | 2.6E2 | 1.3E3 | 1.3E3 | 15 | 13 | 15 | 13 | 0.55 |
| fN | ng/ml | 2.1E-1 | 2.1E-1 | 2.1E0 | 3.8E0 | 3.8E0 | 6.4E0 | 2.1E-1 | 2.1E-1 | 1.4E1 | 2.1E1 | 31 | 18 | 31 | 18 | 0.53 |
| fP | ng/ml | 2.8E2 | 2.6E2 | 3.5E2 | 3.1E2 | 2.1E2 | 1.6E2 | 1.8E0 | 3.7E1 | 1.0E3 | 7.7E2 | 63 | 46 | 63 | 46 | 0.46 |
| fR | ng/ml | 1.7E5 | 1.7E5 | 2.1E5 | 2.3E5 | 1.6E5 | 1.8E5 | 3.9E4 | 1.9E2 | 6.3E5 | 6.8E5 | 56 | 35 | 56 | 35 | 0.54 |
| fY | ng/ml | 2.5E2 | 2.5E2 | 2.5E2 | 2.5E2 | 9.5E1 | 1.2E2 | 6.5E1 | 3.6E1 | 4.2E2 | 4.8E2 | 31 | 18 | 31 | 18 | 0.49 |
| gC | ng/ml | 2.2E2 | 2.4E2 | 2.6E2 | 2.5E2 | 1.2E2 | 1.1E2 | 9.7E1 | 8.3E1 | 6.4E2 | 4.8E2 | 21 | 17 | 21 | 17 | 0.50 |
| gL | pg/ml | 6.5E4 | 6.5E4 | 7.4E4 | 7.0E4 | 3.2E4 | 3.3E4 | 2.3E4 | 1.1E4 | 1.6E5 | 1.7E5 | 64 | 48 | 64 | 48 | 0.47 |
| gP | U/ml | 2.7E2 | 2.9E2 | 2.8E2 | 2.9E2 | 1.3E2 | 9.8E1 | 6.5E1 | 1.2E1 | 1.1E3 | 5.2E2 | 64 | 47 | 64 | 47 | 0.58 |
| gW | ng/ml | 5.2E2 | 3.6E2 | 8.9E2 | 7.5E2 | 1.0E3 | 9.4E2 | 2.3E0 | 5.3E1 | 6.1E3 | 4.2E3 | 49 | 33 | 49 | 33 | 0.44 |
| gV | ng/ml | 1.7E1 | 2.2E1 | 1.9E1 | 2.0E1 | 5.6E0 | 8.6E0 | 1.0E1 | 8.1E-2 | 2.7E1 | 3.0E1 | 19 | 10 | 19 | 10 | 0.58 |
| lF | pg/mL | 1.3E3 | 9.0E2 | 1.3E4 | 1.2E4 | 4.2E4 | 4.2E4 | 1.8E1 | 1.2E1 | 2.8E5 | 2.5E5 | 58 | 45 | 58 | 45 | 0.47 |
| gZ | ug/ml | 7.8E-1 | 7.5E-1 | 2.8E1 | 4.4E1 | 1.0E2 | 1.2E2 | 8.7E-2 | 3.6E-1 | 4.1E2 | 4.0E2 | 15 | 14 | 15 | 14 | 0.60 |
| hA | ng/ml | 2.6E0 | 2.9E0 | 1.4E1 | 1.9E1 | 5.3E1 | 5.5E1 | 1.7E-2 | 1.7E-2 | 3.5E2 | 2.9E2 | 52 | 31 | 52 | 31 | 0.54 |
| nM | pg/ml | 1.0E-9 | 1.0E-9 | 1.0E-9 | 5.7E1 | 0.0E0 | 2.9E2 | 1.0E-9 | 1.0E-9 | 1.0E-9 | 1.5E3 | 36 | 27 | 36 | 27 | 0.52 |
| nN | pg/ml | 1.1E3 | 2.2E3 | 4.8E3 | 1.1E4 | 1.7E4 | 3.0E4 | 1.1E2 | 3.5E2 | 1.0E5 | 1.5E5 | 36 | 27 | 36 | 27 | 0.66 |
| nO | pg/ml | 2.2E1 | 2.5E1 | 3.7E1 | 3.8E1 | 5.4E1 | 3.8E1 | 6.7E0 | 4.0E0 | 3.1E2 | 2.0E2 | 36 | 27 | 36 | 27 | 0.55 |
| nR | pg/ml | 2.0E1 | 1.8E1 | 7.5E1 | 1.4E2 | 1.5E2 | 3.9E2 | 1.4E0 | 1.0E0 | 8.2E2 | 1.9E3 | 36 | 27 | 36 | 27 | 0.50 |
| nT | pg/ml | 5.9E1 | 1.1E2 | 9.9E1 | 1.4E2 | 1.1E2 | 1.8E2 | 1.0E-9 | 1.0E-9 | 6.4E2 | 9.2E2 | 36 | 27 | 36 | 27 | 0.60 |
| nU | pg/ml | 4.4E1 | 4.4E1 | 9.8E1 | 1.1E2 | 2.5E2 | 2.0E2 | 1.0E-9 | 1.0E-9 | 1.5E3 | 9.2E2 | 36 | 27 | 36 | 27 | 0.51 |
| lW | pg/ml | 1.0E-9 | 1.0E-9 | 9.4E0 | 1.6E1 | 1.8E1 | 4.4E1 | 1.0E-9 | 1.0E-9 | 7.0E1 | 1.7E2 | 36 | 27 | 36 | 27 | 0.48 |
| lX | pg/ml | 9.1E2 | 8.6E2 | 9.4E2 | 9.4E2 | 4.9E2 | 6.3E2 | 3.2E2 | 1.9E2 | 1.9E3 | 2.5E3 | 36 | 27 | 36 | 27 | 0.47 |
| lY | pg/ml | 1.7E1 | 2.2E1 | 1.8E1 | 2.4E1 | 1.3E1 | 2.3E1 | 1.0E-9 | 1.0E-9 | 4.8E1 | 1.2E2 | 36 | 27 | 36 | 27 | 0.56 |
| mE | pg/ml | 1.0E-9 | 1.0E-9 | 1.7E0 | 4.1E0 | 4.7E0 | 1.1E1 | 1.0E-9 | 1.0E-9 | 2.8E1 | 5.7E1 | 36 | 27 | 36 | 27 | 0.59 |
| mF | pg/ml | 1.4E-1 | 2.7E-1 | 8.4E0 | 5.7E0 | 4.1E1 | 2.0E1 | 1.0E-9 | 1.0E-9 | 2.5E2 | 1.1E2 | 36 | 27 | 36 | 27 | 0.51 |
| mH | pg/ml | 3.3E0 | 3.1E0 | 4.7E0 | 6.0E0 | 5.3E0 | 1.0E1 | 4.0E-1 | 4.0E-1 | 3.2E1 | 5.3E1 | 36 | 27 | 36 | 27 | 0.49 |
| mI | pg/ml | 1.0E-9 | 3.4E0 | 9.5E0 | 3.5E1 | 1.8E1 | 9.3E1 | 1.0E-9 | 1.0E-9 | 6.7E1 | 4.6E2 | 36 | 27 | 36 | 27 | 0.58 |
| mM | pg/ml | 4.1E1 | 3.8E1 | 9.6E1 | 9.0E1 | 1.9E2 | 1.2E2 | 1.0E-9 | 1.0E-9 | 1.1E3 | 4.7E2 | 36 | 27 | 36 | 27 | 0.47 |
| mP | pg/ml | 1.4E1 | 1.5E1 | 1.8E1 | 5.0E1 | 2.0E1 | 1.5E2 | 1.0E-9 | 1.0E-9 | 1.2E2 | 8.1E2 | 35 | 27 | 35 | 27 | 0.51 |
| mS | pg/ml | 1.6E3 | 1.4E3 | 1.8E3 | 1.7E3 | 8.2E2 | 1.2E3 | 8.8E1 | 1.0E-9 | 3.7E3 | 5.1E3 | 36 | 27 | 36 | 27 | 0.44 |
| mT | pg/ml | 4.8E1 | 7.0E1 | 1.6E2 | 1.7E2 | 3.0E2 | 3.5E2 | 1.0E1 | 1.2E1 | 1.4E3 | 1.7E3 | 35 | 27 | 35 | 27 | 0.54 |
| mU | pg/ml | 2.5E0 | 1.9E0 | 9.5E0 | 3.8E0 | 3.7E1 | 4.6E0 | 1.0E-9 | 1.0E-9 | 2.2E2 | 2.3E1 | 35 | 27 | 35 | 27 | 0.44 |
| mW | pg/ml | 2.5E3 | 1.9E3 | 2.6E3 | 2.6E3 | 1.2E3 | 2.2E3 | 1.0E-9 | 3.7E2 | 5.5E3 | 1.1E4 | 35 | 27 | 35 | 27 | 0.40 |
| mY | pg/ml | 6.6E2 | 6.2E2 | 8.3E2 | 1.2E3 | 8.6E2 | 1.8E3 | 1.0E-9 | 1.0E-9 | 4.2E3 | 8.0E3 | 36 | 27 | 36 | 27 | 0.52 |
| mZ | pg/ml | 2.5E2 | 1.4E2 | 4.2E2 | 2.9E2 | 5.7E2 | 3.5E2 | 1.6E1 | 1.1E1 | 3.1E3 | 1.2E3 | 35 | 27 | 35 | 27 | 0.39 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| nA | pg/ml | 1.8E0 | 9.3E-1 | 7.2E0 | 5.2E0 | 1.4E1 | 1.3E1 | 1.0E-9 | 1.0E-9 | 5.4E1 | 6.5E1 | 35 | 27 | 35 | 27 | 0.44 |
| nB | pg/ml | 2.7E2 | 3.0E2 | 3.1E2 | 3.4E2 | 1.7E2 | 2.0E2 | 3.0E1 | 3.8E1 | 8.2E2 | 9.6E2 | 36 | 27 | 36 | 27 | 0.53 |
| nC | pg/ml | 1.0E-9 | 5.8E1 | 1.1E4 | 8.9E3 | 6.4E4 | 4.2E4 | 1.0E-9 | 1.0E-9 | 3.8E5 | 2.2E5 | 36 | 27 | 36 | 27 | 0.50 |
| nD | pg/ml | 7.0E0 | 6.4E0 | 9.3E0 | 1.6E1 | 9.1E0 | 4.9E1 | 1.0E-9 | 1.0E-9 | 3.7E1 | 2.6E2 | 35 | 27 | 35 | 27 | 0.45 |
| nF | pg/ml | 1.0E-9 | 1.0E-9 | 4.0E0 | 1.5E0 | 1.6E1 | 5.8E0 | 1.0E-9 | 1.0E-9 | 9.3E1 | 2.7E1 | 36 | 27 | 36 | 27 | 0.48 |
| nH | pg/ml | 1.2E0 | 1.1E0 | 7.9E1 | 3.8E2 | 4.4E2 | 1.9E3 | 1.0E-9 | 1.0E-9 | 2.6E3 | 1.0E4 | 35 | 27 | 35 | 27 | 0.52 |
| nI | pg/ml | 4.6E1 | 1.0E-9 | 5.2E1 | 5.3E1 | 6.0E1 | 9.2E1 | 1.0E-9 | 1.0E-9 | 2.0E2 | 3.5E2 | 36 | 27 | 36 | 27 | 0.41 |
| nJ | pg/ml | 6.2E-1 | 1.7E-1 | 1.2E0 | 5.5E0 | 1.8E0 | 2.5E1 | 1.0E-9 | 1.0E-9 | 7.0E0 | 1.3E2 | 36 | 27 | 36 | 27 | 0.43 |
| nK | pg/ml | 1.0E-9 | 1.0E-9 | 1.0E1 | 1.5E1 | 2.1E1 | 2.5E1 | 1.0E-9 | 1.0E-9 | 9.6E1 | 1.0E2 | 35 | 27 | 35 | 27 | 0.58 |
| nL | pg/ml | 1.0E-9 | 1.0E-9 | 1.3E2 | 5.5E2 | 7.2E2 | 2.6E3 | 1.0E-9 | 1.0E-9 | 4.3E3 | 1.4E4 | 36 | 27 | 36 | 27 | 0.55 |
| hL | pg/ml | 1.5E4 | 2.7E4 | 1.9E4 | 2.7E4 | 1.4E4 | 1.5E4 | 2.6E3 | 5.1E3 | 7.2E4 | 6.0E4 | 31 | 18 | 31 | 18 | 0.68 |
| hO | pg/ml | 1.6E4 | 1.6E4 | 1.6E4 | 1.6E4 | 2.6E3 | 2.3E3 | 1.3E4 | 1.1E4 | 2.4E4 | 2.1E4 | 31 | 18 | 31 | 18 | 0.49 |
| hP | ng/ml | 4.4E5 | 4.4E5 | 3.8E5 | 6.9E5 | 1.7E5 | 7.5E5 | 4.7E4 | 1.7E4 | 9.0E5 | 2.8E6 | 31 | 18 | 31 | 18 | 0.64 |
| wJ | pg/ml | 1.4E5 | 1.0E5 | 1.6E5 | 1.6E5 | 9.7E4 | 1.5E5 | 1.1E4 | 1.3E4 | 4.0E5 | 5.8E5 | 32 | 19 | 32 | 19 | 0.45 |
| wK | pg/ml | 3.2E4 | 3.5E4 | 5.6E4 | 3.9E4 | 8.7E4 | 3.0E4 | 3.7E3 | 7.5E3 | 5.0E5 | 1.2E5 | 32 | 19 | 32 | 19 | 0.45 |
| wL | pg/ml | 6.7E0 | 3.9E0 | 6.2E1 | 4.0E1 | 1.7E2 | 1.0E2 | 1.0E-9 | 1.0E-9 | 8.4E2 | 3.5E2 | 32 | 19 | 32 | 19 | 0.43 |
| wP | pg/ml | 2.8E4 | 2.8E4 | 4.0E4 | 7.0E4 | 4.0E4 | 8.1E4 | 4.5E3 | 1.3E3 | 1.7E5 | 3.0E5 | 32 | 19 | 32 | 19 | 0.58 |
| wQ | pg/ml | 3.5E1 | 3.9E1 | 6.1E1 | 6.3E1 | 8.4E1 | 1.1E2 | 1.0E-9 | 1.0E-9 | 3.7E2 | 5.1E2 | 32 | 19 | 32 | 19 | 0.52 |
| hR | pg/ml | 2.8E4 | 2.4E4 | 3.1E4 | 2.6E4 | 1.1E4 | 1.1E4 | 1.0E-9 | 4.3E3 | 5.8E4 | 4.9E4 | 46 | 30 | 46 | 30 | 0.39 |
| hV | pg/ml | 4.3E2 | 4.4E2 | 4.3E2 | 4.4E2 | 2.1E2 | 2.4E2 | 1.0E-9 | 8.2E1 | 9.3E2 | 9.6E2 | 46 | 30 | 46 | 30 | 0.50 |
| hW | pg/ml | 1.8E3 | 2.4E3 | 2.0E3 | 3.7E3 | 1.2E3 | 6.9E3 | 1.0E-9 | 1.1E3 | 7.3E3 | 4.0E4 | 46 | 30 | 46 | 30 | 0.64 |
| hX | pg/ml | 1.1E3 | 1.1E3 | 1.2E3 | 1.1E3 | 1.2E3 | 5.7E2 | 2.5E0 | 1.3E2 | 8.6E3 | 2.9E3 | 46 | 30 | 46 | 30 | 0.52 |
| iA | pg/ml | 1.9E2 | 1.7E2 | 2.9E2 | 2.5E2 | 3.0E2 | 2.5E2 | 2.9E1 | 1.5E1 | 1.8E3 | 8.7E2 | 58 | 45 | 58 | 45 | 0.43 |
| iB | ng/ml | 5.0E0 | 6.8E0 | 6.5E0 | 7.7E0 | 5.1E0 | 5.4E0 | 3.3E-2 | 1.6E0 | 2.4E1 | 2.2E1 | 52 | 31 | 52 | 31 | 0.57 |
| iC | U/ml | 3.6E-1 | 3.8E-1 | 1.7E0 | 6.5E-1 | 7.7E0 | 7.6E-1 | 1.0E-9 | 3.7E-2 | 5.5E1 | 3.2E0 | 52 | 31 | 52 | 31 | 0.55 |
| tQ | pg/ml | 1.5E3 | 1.4E3 | 1.5E3 | 1.5E3 | 5.3E2 | 6.9E2 | 3.7E2 | 7.2E2 | 2.5E3 | 3.3E3 | 31 | 16 | 31 | 16 | 0.47 |
| tT | pg/ml | 1.5E1 | 2.4E1 | 1.8E1 | 2.9E1 | 9.6E0 | 2.0E1 | 5.4E0 | 1.1E1 | 4.8E1 | 9.3E1 | 31 | 17 | 31 | 17 | 0.70 |
| tS | pg/ml | 7.7E-1 | 7.7E-1 | 9.8E-1 | 1.6E0 | 9.8E-1 | 2.5E0 | 1.0E-9 | 1.0E-9 | 4.0E0 | 1.0E1 | 31 | 18 | 31 | 18 | 0.56 |
| tX | pg/ml | 7.1E-1 | 1.6E0 | 1.0E0 | 2.9E0 | 8.4E-1 | 2.9E0 | 2.5E-2 | 3.2E-1 | 3.3E0 | 1.0E1 | 31 | 17 | 31 | 17 | 0.74 |
| tO | pg/ml | 4.4E0 | 3.9E0 | 4.5E0 | 5.7E0 | 2.6E0 | 4.5E0 | 1.0E-9 | 1.7E0 | 9.9E0 | 1.8E1 | 31 | 18 | 31 | 18 | 0.55 |
| tR | pg/ml | 1.9E-1 | 2.2E-1 | 2.4E-1 | 4.4E-1 | 2.4E-1 | 6.6E-1 | 1.0E-9 | 1.0E-9 | 9.1E-1 | 2.5E0 | 31 | 17 | 31 | 17 | 0.57 |
| tU | pg/ml | 8.9E0 | 1.1E1 | 1.1E1 | 1.6E1 | 1.0E1 | 1.8E1 | 1.5E0 | 2.2E-1 | 5.5E1 | 8.0E1 | 31 | 19 | 31 | 19 | 0.61 |
| tN | pg/ml | 1.9E1 | 2.2E1 | 2.2E1 | 4.5E1 | 1.7E1 | 4.7E1 | 1.0E-9 | 1.0E1 | 7.6E1 | 1.6E2 | 30 | 17 | 30 | 17 | 0.66 |
| tV | ng/ml | 4.8E2 | 1.1E3 | 6.2E2 | 1.1E3 | 4.3E2 | 7.1E2 | 1.9E2 | 5.3E1 | 1.8E3 | 3.1E3 | 32 | 18 | 32 | 18 | 0.71 |
| iH | ng/ml | 1.7E5 | 1.6E5 | 1.6E5 | 1.5E5 | 4.7E4 | 5.5E4 | 7.1E4 | 2.9E3 | 2.6E5 | 2.4E5 | 58 | 45 | 58 | 45 | 0.46 |
| iJ | ng/ml | 5.3E4 | 4.3E4 | 6.0E4 | 5.1E4 | 3.6E4 | 3.8E4 | 2.1E4 | 1.8E3 | 2.5E5 | 2.5E5 | 58 | 45 | 58 | 45 | 0.40 |
| hB | ng/ml | 4.8E-1 | 5.1E-1 | 6.4E-1 | 6.9E-1 | 4.7E-1 | 5.9E-1 | 1.4E-1 | 1.2E-1 | 2.3E0 | 3.2E0 | 58 | 45 | 58 | 45 | 0.51 |
| hC | pg/ml | 4.0E3 | 7.7E3 | 6.4E3 | 1.1E4 | 6.3E3 | 1.8E4 | 6.0E1 | 4.1E1 | 3.3E4 | 1.1E5 | 58 | 45 | 58 | 45 | 0.59 |
| hF | ng/ml | 1.0E-9 | 1.0E-9 | 6.9E1 | 1.0E-9 | 5.3E2 | 0.0E0 | 1.0E-9 | 1.0E-9 | 4.0E3 | 1.0E-9 | 58 | 45 | 58 | 45 | 0.49 |
| hG | pg/ml | 7.2E3 | 6.5E3 | 8.1E3 | 7.4E3 | 3.2E3 | 3.9E3 | 1.8E3 | 2.3E3 | 1.8E4 | 2.0E4 | 58 | 45 | 58 | 45 | 0.38 |
| iO | ng/ml | 4.0E5 | 3.9E5 | 4.5E5 | 4.1E5 | 2.1E5 | 1.8E5 | 1.3E5 | 9.8E4 | 1.1E6 | 8.2E5 | 58 | 45 | 58 | 45 | 0.45 |
| iP | ng/ml | 6.0E4 | 4.8E4 | 6.2E4 | 5.3E4 | 5.5E4 | 3.7E4 | 8.3E3 | 7.1E3 | 4.4E5 | 2.2E5 | 58 | 45 | 58 | 45 | 0.42 |
| iZ | ng/ml | 1.5E3 | 1.9E3 | 1.8E3 | 2.1E3 | 8.6E2 | 8.9E2 | 8.2E2 | 7.5E2 | 5.7E3 | 4.6E3 | 58 | 43 | 58 | 43 | 0.62 |
| yH | pg/ml | 9.1E2 | 1.1E3 | 1.8E3 | 3.6E3 | 2.9E3 | 7.6E3 | 1.0E-9 | 1.0E-9 | 1.5E4 | 2.5E4 | 31 | 18 | 31 | 18 | 0.50 |
| yK | U/ml | 1.8E1 | 2.5E1 | 4.7E1 | 3.3E1 | 8.8E1 | 3.5E1 | 1.0E-9 | 1.0E-9 | 3.8E2 | 1.4E2 | 31 | 18 | 31 | 18 | 0.56 |
| yJ | pg/ml | 3.4E4 | 3.6E4 | 4.5E4 | 3.5E4 | 3.0E4 | 2.2E4 | 9.2E3 | 1.9E3 | 1.4E5 | 8.2E4 | 31 | 18 | 31 | 18 | 0.41 |
| yD | ng/ml | 1.3E-2 | 1.3E-2 | 1.3E-2 | 1.3E-2 | 5.7E-3 | 6.5E-3 | 1.0E-9 | 1.0E-9 | 2.8E-2 | 2.4E-2 | 32 | 19 | 32 | 19 | 0.46 |
| jB | ng/ml | 2.5E5 | 2.0E5 | 2.7E5 | 2.0E5 | 7.1E4 | 6.3E4 | 1.5E5 | 9.9E4 | 3.6E5 | 3.3E5 | 15 | 14 | 15 | 14 | 0.25 |
| wB | pg/ml | 8.9E3 | 1.2E4 | 1.0E4 | 1.5E4 | 7.0E3 | 1.1E4 | 1.9E3 | 2.3E3 | 3.3E4 | 4.2E4 | 32 | 19 | 32 | 19 | 0.62 |
| pY | pg/ml | 5.7E0 | 6.8E0 | 1.2E1 | 7.6E0 | 3.5E1 | 4.2E0 | 1.6E0 | 3.0E0 | 2.0E2 | 1.8E1 | 31 | 18 | 31 | 18 | 0.64 |
| sI | ng/ml | 5.4E-2 | 5.2E-2 | 5.6E-2 | 5.9E-2 | 2.2E-2 | 4.2E-2 | 2.1E-2 | 1.0E-2 | 1.1E-1 | 1.5E-1 | 16 | 10 | 16 | 10 | 0.47 |
| sF | mIU/mL | 7.4E0 | 5.0E0 | 1.2E1 | 1.1E1 | 1.8E1 | 1.5E1 | 6.2E-1 | 1.5E0 | 7.5E1 | 5.2E1 | 16 | 10 | 16 | 10 | 0.45 |
| sH | mIU/mL | 4.6E0 | 2.6E0 | 4.9E0 | 4.1E0 | 5.2E0 | 4.5E0 | 1.0E-9 | 3.9E-1 | 2.1E1 | 1.5E1 | 16 | 10 | 16 | 10 | 0.45 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| sJ | ng/ml | 1.5E-1 | 2.4E-1 | 7.4E-1 | 2.2E-1 | 1.7E0 | 1.9E-1 | 1.0E-9 | 3.0E-2 | 6.4E0 | 6.1E-1 | 16 | 10 | 16 | 10 | 0.52 |
| rC | pg/ml | 1.6E3 | 1.4E3 | 2.3E3 | 2.1E3 | 2.5E3 | 2.3E3 | 1.1E2 | 1.0E-9 | 1.5E4 | 1.1E4 | 44 | 29 | 44 | 29 | 0.47 |
| rB | pg/ml | 3.1E1 | 2.9E1 | 4.9E1 | 6.3E1 | 7.1E1 | 7.9E1 | 1.0E-9 | 1.0E-9 | 3.9E2 | 3.2E2 | 44 | 29 | 44 | 29 | 0.53 |
| zG | 2.5ng/ml | 2.2E-1 | 2.2E-1 | 7.1E-1 | 4.1E-1 | 1.2E0 | 6.4E-1 | 1.0E-9 | 1.0E-9 | 4.8E0 | 2.7E0 | 31 | 18 | 31 | 18 | 0.48 |
| zH | 2.3mU/ml | 8.8E-2 | 8.7E-2 | 9.8E-2 | 9.1E-2 | 5.0E-2 | 4.1E-2 | 1.0E-2 | 2.1E-2 | 3.1E-1 | 1.8E-1 | 31 | 18 | 31 | 18 | 0.45 |
| zI | 2.6ng/ml | 1.9E0 | 2.4E0 | 3.4E0 | 6.5E0 | 3.5E0 | 8.2E0 | 6.1E-1 | 5.4E-1 | 1.5E1 | 2.7E1 | 31 | 18 | 31 | 18 | 0.56 |
| qA | ng/ml | 7.8E6 | 1.3E7 | 1.1E7 | 1.3E7 | 8.0E6 | 5.9E6 | 3.7E6 | 4.3E6 | 3.9E7 | 3.0E7 | 31 | 18 | 31 | 18 | 0.69 |
| qB | ng/ml | 6.6E5 | 6.7E5 | 8.5E5 | 8.8E5 | 6.3E5 | 8.1E5 | 1.9E5 | 2.4E5 | 2.9E6 | 3.8E6 | 31 | 18 | 31 | 18 | 0.50 |
| qC | ng/ml | 3.1E5 | 2.5E5 | 5.9E5 | 6.1E5 | 7.4E5 | 1.1E6 | 2.5E4 | 6.5E4 | 3.1E6 | 4.7E6 | 31 | 18 | 31 | 18 | 0.48 |
| qD | ng/ml | 1.5E7 | 1.5E7 | 1.7E7 | 1.7E7 | 7.5E6 | 7.3E6 | 7.0E6 | 8.7E6 | 3.7E7 | 3.4E7 | 31 | 18 | 31 | 18 | 0.51 |
| jD | ng/ml | 3.8E1 | 3.2E1 | 4.3E1 | 5.3E1 | 4.1E1 | 6.3E1 | 1.0E-9 | 1.0E-9 | 1.9E2 | 2.9E2 | 52 | 31 | 52 | 31 | 0.52 |
| jE | ng/ml | 1.0E-9 | 1.0E-9 | 4.6E0 | 6.1E0 | 1.2E1 | 1.1E1 | 1.0E-9 | 1.0E-9 | 6.2E1 | 3.5E1 | 52 | 31 | 52 | 31 | 0.55 |
| jF | ng/ml | 4.5E1 | 1.6E1 | 4.7E1 | 3.9E1 | 4.9E1 | 5.4E1 | 1.0E-9 | 1.0E-9 | 1.9E2 | 1.9E2 | 52 | 31 | 52 | 31 | 0.43 |
| jG | ng/ml | 4.2E3 | 4.2E3 | 4.4E3 | 4.4E3 | 1.9E3 | 2.0E3 | 6.7E2 | 1.4E3 | 9.5E3 | 9.6E3 | 52 | 31 | 52 | 31 | 0.48 |
| jH | ng/ml | 7.9E1 | 7.5E1 | 8.4E1 | 8.9E1 | 4.7E1 | 7.4E1 | 1.3E1 | 1.5E1 | 2.4E2 | 4.3E2 | 52 | 31 | 52 | 31 | 0.48 |
| jI | ng/ml | 7.4E1 | 8.7E1 | 7.9E1 | 1.0E2 | 3.3E1 | 7.9E1 | 3.8E1 | 3.8E1 | 1.9E2 | 4.4E2 | 52 | 31 | 52 | 31 | 0.56 |
| sK | pg/mL | 4.1E3 | 3.8E3 | 4.0E3 | 5.5E3 | 1.7E3 | 5.1E3 | 1.8E3 | 2.1E3 | 8.8E3 | 2.3E4 | 30 | 17 | 30 | 17 | 0.55 |
| sM | pg/mL | 7.3E4 | 7.9E4 | 7.8E4 | 8.9E4 | 2.7E4 | 4.3E4 | 4.3E4 | 3.9E4 | 1.6E5 | 2.0E5 | 30 | 17 | 30 | 17 | 0.56 |
| sO | pg/mL | 2.3E8 | 2.3E8 | 2.4E8 | 2.3E8 | 8.4E7 | 1.1E8 | 4.9E7 | 6.6E7 | 4.4E8 | 4.2E8 | 30 | 17 | 30 | 17 | 0.45 |
| wC | ng/ml | 1.5E0 | 1.6E0 | 2.0E0 | 1.8E0 | 1.4E0 | 1.1E0 | 3.6E-1 | 6.1E-2 | 6.5E0 | 4.8E0 | 32 | 19 | 32 | 19 | 0.51 |
| wD | ng/ml | 2.2E1 | 2.8E1 | 1.0E2 | 6.0E1 | 3.7E2 | 7.0E1 | 3.8E0 | 2.8E0 | 2.1E3 | 2.9E2 | 32 | 19 | 32 | 19 | 0.63 |
| wE | ng/ml | 4.9E1 | 5.8E1 | 4.9E1 | 5.4E1 | 1.9E1 | 2.0E1 | 8.1E0 | 2.0E1 | 8.9E1 | 8.9E1 | 32 | 19 | 32 | 19 | 0.56 |
| wG | ng/ml | 1.1E-1 | 8.6E-2 | 1.4E-1 | 1.3E-1 | 1.3E-1 | 1.7E-1 | 1.0E-9 | 1.0E-9 | 4.8E-1 | 6.8E-1 | 32 | 19 | 32 | 19 | 0.45 |
| wH | ng/ml | 3.2E-2 | 3.9E-2 | 2.9E-1 | 5.5E-1 | 7.9E-1 | 1.3E0 | 1.0E-9 | 1.0E-9 | 4.2E0 | 5.6E0 | 32 | 19 | 32 | 19 | 0.55 |
| wF | ng/ml | 2.7E-1 | 2.6E-1 | 2.9E0 | 2.0E0 | 1.1E1 | 4.4E0 | 1.0E-9 | 1.0E-9 | 5.7E1 | 1.9E1 | 32 | 19 | 32 | 19 | 0.54 |
| rA | pg/ml | 2.4E1 | 1.9E1 | 2.7E1 | 2.8E1 | 1.7E1 | 2.5E1 | 1.0E-9 | 1.0E-9 | 6.4E1 | 1.1E2 | 50 | 30 | 50 | 30 | 0.47 |
| qZ | pg/ml | 4.7E1 | 5.8E1 | 8.4E2 | 2.5E2 | 2.7E3 | 7.1E2 | 2.8E-4 | 5.9E-4 | 1.0E4 | 3.4E3 | 38 | 22 | 38 | 22 | 0.54 |
| qY | pg/ml | 1.5E1 | 1.3E1 | 4.1E1 | 2.9E1 | 5.0E1 | 6.1E1 | 8.7E-1 | 2.1E0 | 1.8E2 | 3.3E2 | 50 | 30 | 50 | 30 | 0.40 |
| qX | pg/ml | 5.5E1 | 6.4E1 | 6.5E1 | 7.5E1 | 4.5E1 | 4.8E1 | 1.0E-9 | 1.0E-9 | 1.7E2 | 2.1E2 | 50 | 30 | 50 | 30 | 0.55 |
| qW | pg/ml | 7.4E0 | 7.3E0 | 9.6E0 | 8.6E0 | 9.0E0 | 8.7E0 | 1.0E-9 | 1.0E-9 | 3.6E1 | 3.1E1 | 50 | 30 | 50 | 30 | 0.45 |
| qV | pg/ml | 1.5E3 | 1.9E3 | 2.3E3 | 2.4E3 | 2.0E3 | 1.9E3 | 1.7E2 | 1.0E2 | 1.1E4 | 9.6E3 | 50 | 30 | 50 | 30 | 0.53 |
| qU | pg/ml | 4.6E1 | 9.6E1 | 1.4E2 | 2.8E2 | 2.3E2 | 4.4E2 | 1.0E-9 | 1.0E-9 | 1.2E3 | 2.1E3 | 50 | 30 | 50 | 30 | 0.62 |
| qT | pg/ml | 3.9E1 | 4.0E1 | 7.3E1 | 5.3E1 | 8.8E1 | 4.0E1 | 1.0E-9 | 1.0E-9 | 4.9E2 | 1.6E2 | 50 | 30 | 50 | 30 | 0.50 |
| qI | ng/ml | 6.2E4 | 6.3E4 | 6.4E4 | 6.2E4 | 3.3E4 | 2.7E4 | 1.0E4 | 2.5E4 | 1.6E5 | 1.3E5 | 29 | 17 | 29 | 17 | 0.49 |
| qH | ng/ml | 5.4E4 | 6.2E4 | 6.6E4 | 7.9E4 | 4.0E4 | 4.0E4 | 1.5E4 | 3.2E4 | 1.8E5 | 1.8E5 | 29 | 17 | 29 | 17 | 0.62 |
| qG | ng/ml | 1.8E5 | 1.8E5 | 1.9E5 | 1.9E5 | 6.2E4 | 8.3E4 | 3.4E4 | 8.4E4 | 3.0E5 | 4.2E5 | 29 | 17 | 29 | 17 | 0.47 |
| jK | ng/ml | 1.6E3 | 1.3E3 | 1.7E3 | 1.5E3 | 6.4E2 | 6.3E2 | 2.8E2 | 7.5E2 | 4.1E3 | 3.6E3 | 52 | 31 | 52 | 31 | 0.39 |
| jL | ng/ml | 1.7E2 | 2.4E2 | 2.8E2 | 2.7E2 | 2.1E2 | 1.7E2 | 5.9E1 | 6.4E1 | 8.1E2 | 7.6E2 | 52 | 31 | 52 | 31 | 0.55 |
| jM | ng/ml | 6.7E4 | 6.8E4 | 7.0E4 | 7.6E4 | 3.4E4 | 4.8E4 | 2.1E4 | 4.6E3 | 1.7E5 | 1.7E5 | 52 | 31 | 52 | 31 | 0.53 |
| jO | pg/ml | 2.4E5 | 2.5E5 | 2.8E5 | 2.5E5 | 1.5E5 | 1.3E5 | 7.6E4 | 9.6E4 | 7.7E5 | 6.5E5 | 52 | 31 | 52 | 31 | 0.46 |
| jP | pg/ml | 2.6E5 | 3.2E5 | 2.8E5 | 3.4E5 | 1.4E5 | 1.7E5 | 6.1E4 | 1.3E5 | 7.1E5 | 7.0E5 | 52 | 31 | 52 | 31 | 0.59 |
| jQ | pg/ml | 2.4E3 | 1.3E3 | 2.9E3 | 3.0E3 | 2.3E3 | 3.3E3 | 2.5E2 | 5.0E0 | 1.0E4 | 1.3E4 | 52 | 31 | 52 | 31 | 0.44 |
| jR | pg/ml | 5.9E3 | 3.8E3 | 8.5E3 | 1.1E4 | 8.0E3 | 1.5E4 | 3.0E1 | 1.0E-9 | 3.5E4 | 5.6E4 | 52 | 31 | 52 | 31 | 0.46 |
| jT | pg/ml | 1.7E5 | 1.6E5 | 1.7E5 | 1.7E5 | 5.8E4 | 6.5E4 | 7.1E4 | 7.5E4 | 3.5E5 | 3.5E5 | 52 | 31 | 52 | 31 | 0.50 |
| xA | pg/ml | 5.7E0 | 6.0E0 | 1.2E1 | 2.1E1 | 1.5E1 | 4.3E1 | 1.0E-9 | 1.0E-9 | 6.1E1 | 1.6E2 | 31 | 18 | 31 | 18 | 0.48 |
| yE | pg/ml | 7.9E1 | 9.1E1 | 7.8E1 | 9.3E1 | 3.2E1 | 4.0E1 | 6.4E0 | 3.5E1 | 1.4E2 | 2.0E2 | 31 | 18 | 31 | 18 | 0.61 |
| tM | pg/ml | 4.3E1 | 4.2E1 | 3.9E1 | 4.3E1 | 1.9E1 | 1.9E1 | 1.0E-9 | 1.6E1 | 8.3E1 | 9.9E1 | 31 | 18 | 31 | 18 | 0.55 |
| tL | pg/ml | 1.0E-9 | 1.0E-9 | 2.4E0 | 4.7E-1 | 1.2E1 | 1.0E0 | 1.0E-9 | 1.0E-9 | 6.7E1 | 3.6E0 | 31 | 18 | 31 | 18 | 0.55 |
| jU | mIU/ml | 5.8E0 | 4.8E0 | 1.4E1 | 1.1E1 | 2.2E1 | 1.4E1 | 8.1E-2 | 7.1E-1 | 1.1E2 | 5.7E1 | 52 | 31 | 52 | 31 | 0.48 |
| jV | mIU/ml | 2.3E0 | 1.8E0 | 4.7E0 | 3.0E0 | 6.4E0 | 3.4E0 | 2.7E-3 | 1.0E-1 | 3.2E1 | 1.5E1 | 52 | 31 | 52 | 31 | 0.44 |
| jY | ng/ml | 5.8E-4 | 2.0E-3 | 1.0E-2 | 6.1E-3 | 4.3E-2 | 1.0E-2 | 1.0E-9 | 1.0E-9 | 3.0E-1 | 5.1E-2 | 52 | 31 | 52 | 31 | 0.60 |
| kC | pg/ml | 1.0E2 | 9.6E1 | 1.5E2 | 2.5E2 | 1.7E2 | 5.3E2 | 2.1E1 | 3.6E1 | 1.1E3 | 2.7E3 | 36 | 27 | 36 | 27 | 0.53 |
| kE | pg/ml | 1.5E5 | 1.4E5 | 1.4E5 | 1.4E5 | 3.6E4 | 4.2E4 | 4.1E4 | 7.5E4 | 2.3E5 | 2.7E5 | 36 | 27 | 36 | 27 | 0.48 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| kF | pg/mL | 6.9E1 | 5.7E1 | 6.9E1 | 7.4E1 | 1.7E1 | 3.4E1 | 3.5E1 | 4.0E1 | 1.1E2 | 1.5E2 | 36 | 27 | 36 | 27 | 0.44 |
| kG | pg/mL | 8.0E3 | 9.0E3 | 1.2E4 | 1.7E4 | 1.2E4 | 3.1E4 | 1.9E3 | 1.1E3 | 5.8E4 | 1.6E5 | 36 | 27 | 36 | 27 | 0.52 |
| kI | pg/ml | 2.1E2 | 2.1E2 | 2.3E2 | 2.0E2 | 1.2E2 | 9.5E1 | 6.4E1 | 1.0E-9 | 6.7E2 | 3.7E2 | 36 | 27 | 36 | 27 | 0.46 |
| kK | pg/ml | 1.2E2 | 1.2E2 | 1.7E2 | 1.5E2 | 1.7E2 | 1.0E2 | 2.2E1 | 2.1E1 | 9.1E2 | 4.6E2 | 36 | 27 | 36 | 27 | 0.50 |
| kN | pg/ml | 1.0E3 | 1.1E3 | 1.5E3 | 1.9E3 | 1.7E3 | 2.1E3 | 2.1E2 | 3.8E2 | 1.0E4 | 8.7E3 | 36 | 27 | 36 | 27 | 0.53 |
| kO | pg/ml | 7.1E3 | 7.3E3 | 8.1E3 | 1.2E4 | 4.0E3 | 2.7E4 | 4.0E3 | 3.8E3 | 2.5E4 | 1.5E5 | 36 | 27 | 36 | 27 | 0.49 |
| kP | pg/ml | 5.5E3 | 5.2E3 | 6.8E3 | 6.3E3 | 4.6E3 | 3.9E3 | 9.6E2 | 1.6E3 | 2.7E4 | 1.5E4 | 36 | 27 | 36 | 27 | 0.46 |
| kQ | pg/ml | 4.3E3 | 4.7E3 | 5.4E3 | 5.9E3 | 3.9E3 | 4.7E3 | 5.6E2 | 1.1E3 | 2.5E4 | 2.5E4 | 58 | 45 | 58 | 45 | 0.53 |
| kR | pg/ml | 2.2E1 | 2.7E1 | 4.5E1 | 3.2E1 | 1.3E2 | 2.5E1 | 1.0E-9 | 3.4E0 | 1.0E3 | 1.1E2 | 58 | 45 | 58 | 45 | 0.54 |
| kS | pg/ml | 8.3E2 | 9.5E2 | 9.5E2 | 1.0E3 | 5.7E2 | 6.4E2 | 8.2E1 | 2.6E2 | 3.2E3 | 3.0E3 | 58 | 45 | 58 | 45 | 0.56 |
| pS | ng/ml | 1.6E5 | 1.4E5 | 2.0E5 | 1.6E5 | 1.1E5 | 8.3E4 | 7.5E4 | 6.8E4 | 5.7E5 | 3.6E5 | 30 | 17 | 30 | 17 | 0.41 |
| rZ | ng/ml | 1.4E-3 | 3.2E-3 | 5.9E-3 | 1.4E-2 | 1.5E-2 | 2.5E-2 | 1.0E-9 | 1.0E-9 | 9.4E-2 | 1.1E-1 | 45 | 29 | 45 | 29 | 0.62 |
| rY | ng/ml | 6.1E-2 | 7.1E-2 | 2.6E-1 | 7.7E-1 | 9.4E-1 | 3.7E0 | 2.4E-3 | 1.0E-9 | 6.3E0 | 2.0E1 | 45 | 29 | 45 | 29 | 0.56 |
| rX | ng/ml | 1.0E-9 | 1.0E-9 | 1.2E-1 | 1.1E-1 | 5.6E-1 | 5.7E-1 | 1.0E-9 | 1.0E-9 | 3.7E0 | 3.1E0 | 45 | 29 | 45 | 29 | 0.52 |
| lK | pg/ml | 6.5E1 | 6.8E1 | 1.4E2 | 1.3E2 | 1.7E2 | 1.7E2 | 1.0E-9 | 1.0E-9 | 7.0E2 | 6.9E2 | 51 | 31 | 51 | 31 | 0.44 |
| lL | pg/ml | 1.8E3 | 1.7E3 | 3.3E3 | 2.5E3 | 6.3E3 | 2.2E3 | 7.5E1 | 1.2E2 | 4.2E4 | 7.7E3 | 52 | 31 | 52 | 31 | 0.50 |
| lM | pg/ml | 1.6E3 | 1.1E3 | 4.7E3 | 5.8E3 | 7.6E3 | 1.4E4 | 2.2E2 | 9.5E0 | 4.2E4 | 6.7E4 | 52 | 31 | 52 | 31 | 0.44 |
| lN | pg/ml | 1.0E-9 | 1.0E-9 | 2.9E0 | 4.1E0 | 7.2E0 | 7.1E0 | 1.0E-9 | 1.0E-9 | 4.3E1 | 2.4E1 | 52 | 31 | 52 | 31 | 0.56 |
| lO | pg/ml | 1.0E-9 | 1.0E-9 | 6.7E-1 | 8.5E0 | 4.7E0 | 3.3E1 | 1.0E-9 | 1.0E-9 | 3.4E1 | 1.4E2 | 51 | 31 | 51 | 31 | 0.52 |
| zA | ng/ml | 2.1E7 | 2.1E7 | 2.2E7 | 2.0E7 | 6.5E6 | 5.9E6 | 1.0E7 | 1.0E7 | 3.6E7 | 3.1E7 | 31 | 19 | 31 | 19 | 0.45 |
| rW | ng/ml | 1.2E-2 | 1.4E-2 | 2.3E-2 | 4.9E-2 | 3.5E-2 | 8.9E-2 | 1.0E-9 | 1.0E-9 | 1.7E-1 | 3.2E-1 | 31 | 15 | 31 | 15 | 0.58 |
| rV | ng/ml | 1.0E-9 | 1.0E-9 | 1.0E-2 | 1.9E-2 | 5.1E-2 | 4.0E-2 | 1.0E-9 | 1.0E-9 | 2.8E-1 | 1.5E-1 | 31 | 15 | 31 | 15 | 0.67 |
| rU | ng/ml | 4.2E-2 | 9.7E-2 | 1.7E-1 | 1.3E-1 | 4.8E-1 | 1.2E-1 | 1.0E-9 | 1.0E-9 | 2.7E0 | 4.0E-1 | 31 | 15 | 31 | 15 | 0.61 |
| rT | ng/ml | 6.3E0 | 4.2E0 | 6.5E0 | 7.8E0 | 4.1E0 | 6.2E0 | 6.5E-1 | 1.0E0 | 2.1E1 | 2.0E1 | 31 | 15 | 31 | 15 | 0.52 |
| rS | ng/ml | 3.5E0 | 7.4E0 | 5.2E0 | 1.7E1 | 5.3E0 | 2.1E1 | 1.1E0 | 2.0E0 | 2.5E1 | 7.0E1 | 31 | 15 | 31 | 15 | 0.72 |
| sC | pg/mL | 5.3E3 | 8.5E3 | 8.9E3 | 1.4E4 | 1.1E4 | 1.8E4 | 2.3E3 | 1.7E3 | 5.1E4 | 7.4E4 | 30 | 17 | 30 | 17 | 0.59 |
| yL | pg/ml | 3.0E1 | 3.2E1 | 3.5E1 | 1.2E2 | 3.0E1 | 3.3E2 | 5.6E0 | 9.1E0 | 1.8E2 | 1.4E3 | 31 | 18 | 31 | 18 | 0.58 |
| rP | ng/ml | 9.8E1 | 2.1E2 | 1.8E2 | 3.1E2 | 1.8E2 | 2.5E2 | 1.0E-9 | 1.2E1 | 5.0E2 | 8.0E2 | 31 | 15 | 31 | 15 | 0.62 |
| rQ | ng/ml | 1.0E-9 | 1.0E-9 | 1.0E-9 | 2.1E1 | 0.0E0 | 5.1E1 | 1.0E-9 | 1.0E-9 | 1.0E-9 | 1.7E2 | 31 | 15 | 31 | 15 | 0.60 |
| rO | ng/ml | 1.5E-2 | 2.5E-2 | 3.6E-2 | 4.6E-2 | 7.3E-2 | 5.5E-2 | 1.0E-9 | 1.0E-9 | 4.0E-1 | 1.9E-1 | 31 | 15 | 31 | 15 | 0.58 |
| rR | ng/ml | 1.0E-9 | 4.0E0 | 9.9E0 | 1.5E1 | 2.2E1 | 2.2E1 | 1.0E-9 | 1.0E-9 | 9.5E1 | 7.5E1 | 31 | 15 | 31 | 15 | 0.59 |
| rN | ng/ml | 6.4E-1 | 6.3E-1 | 8.3E-1 | 1.8E0 | 5.6E-1 | 3.3E0 | 5.1E-2 | 2.1E-1 | 2.3E0 | 1.3E1 | 31 | 15 | 31 | 15 | 0.52 |
| qO | pg/ml | 7.8E3 | 9.3E3 | 1.1E4 | 1.5E4 | 8.2E3 | 1.3E4 | 7.4E2 | 1.9E3 | 3.7E4 | 4.8E4 | 29 | 18 | 29 | 18 | 0.56 |
| qP | pg/ml | 3.6E2 | 3.3E2 | 3.7E2 | 4.8E2 | 2.2E2 | 3.6E2 | 7.0E1 | 1.2E2 | 1.0E3 | 1.5E3 | 29 | 18 | 29 | 18 | 0.56 |
| qQ | pg/ml | 1.5E1 | 1.5E1 | 2.0E1 | 3.1E1 | 5.1E1 | 6.0E1 | 1.0E-9 | 1.0E-9 | 2.8E2 | 2.6E2 | 29 | 18 | 29 | 18 | 0.61 |
| nW | pg/ml | 1.1E5 | 1.1E5 | 1.1E5 | 1.1E5 | 2.7E4 | 2.9E4 | 5.8E4 | 3.6E4 | 1.8E5 | 1.7E5 | 58 | 45 | 58 | 45 | 0.47 |
| nY | pg/ml | 2.2E3 | 2.8E3 | 2.5E3 | 3.0E3 | 1.6E3 | 1.6E3 | 5.1E2 | 6.3E2 | 1.0E4 | 8.1E3 | 58 | 45 | 58 | 45 | 0.59 |
| oO | pg/ml | 9.3E4 | 9.1E4 | 1.1E5 | 1.1E5 | 5.9E4 | 8.6E4 | 3.2E4 | 3.3E3 | 2.6E5 | 4.0E5 | 30 | 26 | 30 | 26 | 0.44 |
| oP | pg/ml | 1.5E5 | 1.3E5 | 1.6E5 | 1.5E5 | 8.1E4 | 1.2E5 | 4.9E4 | 2.4E4 | 4.1E5 | 5.7E5 | 30 | 26 | 30 | 26 | 0.40 |
| oQ | pg/ml | 3.5E3 | 2.9E3 | 4.3E3 | 4.7E3 | 3.7E3 | 5.9E3 | 1.1E3 | 7.7E2 | 2.1E4 | 3.2E4 | 30 | 26 | 30 | 26 | 0.46 |
| oE | pg/ml | 2.0E2 | 2.6E2 | 4.9E2 | 6.5E2 | 6.2E2 | 7.8E2 | 1.0E-9 | 1.0E-9 | 2.8E3 | 3.4E3 | 58 | 45 | 58 | 45 | 0.56 |
| oF | pg/ml | 1.3E4 | 1.9E4 | 2.5E4 | 3.7E4 | 3.0E4 | 5.0E4 | 4.3E2 | 5.1E2 | 1.5E5 | 2.5E5 | 58 | 45 | 58 | 45 | 0.56 |
| oH | pg/ml | 3.5E1 | 3.5E1 | 8.3E1 | 7.9E1 | 1.4E2 | 1.1E2 | 5.4E0 | 4.4E0 | 8.6E2 | 4.8E2 | 58 | 45 | 58 | 45 | 0.49 |
| oK | pg/ml | 1.0E3 | 8.8E2 | 1.8E3 | 1.4E3 | 2.1E3 | 1.3E3 | 8.8E1 | 1.4E2 | 1.2E4 | 5.9E3 | 58 | 45 | 58 | 45 | 0.48 |
| oN | pg/ml | 5.6E2 | 5.6E2 | 1.2E3 | 7.7E2 | 2.6E3 | 7.9E2 | 1.6E2 | 1.1E2 | 1.8E4 | 5.3E3 | 58 | 45 | 58 | 45 | 0.52 |
| oW | pg/ml | 2.0E2 | 4.1E2 | 2.7E2 | 1.1E3 | 1.8E2 | 2.0E3 | 7.7E1 | 2.9E1 | 7.3E2 | 7.6E3 | 15 | 14 | 15 | 14 | 0.65 |
| oT | pg/ml | 2.9E2 | 2.9E2 | 3.2E2 | 3.5E2 | 1.5E2 | 2.1E2 | 1.0E2 | 1.3E2 | 7.4E2 | 7.9E2 | 15 | 14 | 15 | 14 | 0.51 |
| oV | pg/ml | 9.8E1 | 8.9E1 | 2.6E2 | 3.1E2 | 3.7E2 | 5.9E2 | 2.1E1 | 1.0E-9 | 1.4E3 | 2.2E3 | 15 | 14 | 15 | 14 | 0.48 |
| oD | pg/ml | 1.7E4 | 1.4E4 | 1.6E4 | 1.5E4 | 5.0E3 | 7.0E3 | 8.7E3 | 6.6E3 | 2.5E4 | 3.2E4 | 15 | 14 | 15 | 14 | 0.40 |
| uL | ng/ml | 3.8E1 | 3.3E1 | 5.2E1 | 3.7E1 | 5.3E1 | 1.8E1 | 1.5E1 | 1.4E1 | 2.9E2 | 8.0E1 | 30 | 17 | 30 | 17 | 0.41 |
| uO | ng/ml | 4.6E-1 | 3.4E-1 | 9.3E-1 | 7.6E-1 | 1.7E0 | 7.9E-1 | 1.0E-9 | 1.0E-9 | 9.3E0 | 2.1E0 | 30 | 17 | 30 | 17 | 0.50 |
| uM | ng/ml | 6.2E-1 | 4.4E-1 | 1.2E0 | 5.3E-1 | 2.3E0 | 6.0E-1 | 1.0E-9 | 1.0E-9 | 1.3E1 | 2.5E0 | 30 | 17 | 30 | 17 | 0.34 |
| uI | ng/ml | 7.4E-2 | 5.4E-2 | 1.2E-1 | 9.0E-2 | 1.2E-1 | 1.0E-1 | 1.6E-2 | 1.5E-2 | 5.8E-1 | 4.3E-1 | 30 | 16 | 30 | 16 | 0.41 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| uN | ng/ml | 1.4E1 | 1.7E1 | 1.6E1 | 2.0E1 | 5.8E0 | 8.9E0 | 8.0E0 | 1.0E1 | 3.0E1 | 4.1E1 | 30 | 17 | 30 | 17 | 0.63 |
| uG | ng/ml | 1.8E1 | 1.9E1 | 2.2E1 | 2.7E1 | 1.5E1 | 3.0E1 | 6.1E0 | 1.2E0 | 7.9E1 | 1.3E2 | 30 | 17 | 30 | 17 | 0.51 |
| uR | ng/ml | 2.1E0 | 2.0E0 | 2.8E0 | 2.9E0 | 2.4E0 | 2.2E0 | 8.9E-1 | 7.5E-1 | 1.3E1 | 8.3E0 | 31 | 18 | 31 | 18 | 0.51 |
| uP | ng/ml | 2.1E0 | 2.6E0 | 2.3E0 | 2.9E0 | 9.4E-1 | 1.4E0 | 1.2E0 | 9.3E-1 | 6.0E0 | 6.1E0 | 31 | 18 | 31 | 18 | 0.65 |
| uV | ng/ml | 2.3E-4 | 3.2E-3 | 1.8E-2 | 8.4E-3 | 4.0E-2 | 1.2E-2 | 1.0E-9 | 1.0E-9 | 2.0E-1 | 4.3E-2 | 31 | 18 | 31 | 18 | 0.49 |
| uT | ng/ml | 6.3E1 | 1.0E2 | 9.2E1 | 1.2E2 | 9.4E1 | 9.5E1 | 1.3E1 | 2.2E1 | 4.5E2 | 4.1E2 | 31 | 18 | 31 | 18 | 0.68 |
| uU | ng/ml | 1.2E0 | 2.0E0 | 1.7E0 | 2.9E0 | 1.2E0 | 4.3E0 | 6.0E-1 | 5.4E-1 | 6.0E0 | 2.0E1 | 31 | 18 | 31 | 18 | 0.64 |
| uW | ng/ml | 7.4E0 | 8.1E0 | 7.7E0 | 8.7E0 | 2.0E0 | 2.6E0 | 4.4E0 | 5.7E0 | 1.3E1 | 1.6E1 | 30 | 17 | 30 | 17 | 0.61 |
| vB | ng/ml | 2.9E0 | 3.3E0 | 3.4E0 | 3.4E0 | 2.3E0 | 2.2E0 | 5.9E-1 | 8.3E-1 | 1.0E1 | 1.0E1 | 30 | 17 | 30 | 17 | 0.52 |
| vC | ng/ml | 1.0E-9 | 1.0E-9 | 1.0E-9 | 1.0E-9 | 0.0E0 | 0.0E0 | 1.0E-9 | 1.0E-9 | 1.0E-9 | 1.0E-9 | 30 | 17 | 30 | 17 | 0.50 |
| uY | ng/ml | 6.1E-1 | 7.5E-1 | 9.0E-1 | 1.2E0 | 8.4E-1 | 1.2E0 | 6.8E-2 | 3.1E-1 | 3.8E0 | 4.4E0 | 30 | 17 | 30 | 17 | 0.61 |
| uZ | ng/ml | 5.8E-1 | 5.3E-1 | 8.0E-1 | 6.3E-1 | 9.4E-1 | 4.1E-1 | 1.0E-1 | 1.7E-1 | 4.9E0 | 1.9E0 | 30 | 17 | 30 | 17 | 0.48 |
| uX | ng/ml | 8.1E0 | 8.6E0 | 1.1E1 | 1.6E1 | 7.1E0 | 1.8E1 | 3.6E0 | 4.2E0 | 4.0E1 | 6.5E1 | 30 | 17 | 30 | 17 | 0.54 |
| vA | ng/ml | 6.3E-2 | 6.3E-2 | 8.0E-2 | 8.5E-2 | 5.5E-2 | 9.0E-2 | 2.9E-2 | 2.5E-2 | 2.7E-1 | 4.2E-1 | 30 | 17 | 30 | 17 | 0.49 |
| vH | ng/ml | 1.3E-1 | 1.2E-1 | 1.6E-1 | 2.3E-1 | 1.5E-1 | 4.4E-1 | 2.0E-2 | 2.1E-2 | 6.6E-1 | 1.9E0 | 30 | 17 | 30 | 17 | 0.45 |
| vI | ng/ml | 2.2E0 | 2.9E0 | 2.2E0 | 3.5E0 | 1.4E0 | 2.9E0 | 6.3E-3 | 8.7E-2 | 6.4E0 | 1.0E1 | 30 | 17 | 30 | 17 | 0.65 |
| vP | ng/ml | 3.8E2 | 2.6E2 | 4.3E2 | 5.1E2 | 3.0E2 | 5.8E2 | 6.7E1 | 9.2E1 | 1.1E3 | 2.4E3 | 31 | 18 | 31 | 18 | 0.49 |
| vT | ng/ml | 7.2E1 | 7.0E1 | 8.9E1 | 7.8E1 | 4.4E1 | 3.2E1 | 4.1E1 | 4.6E1 | 2.4E2 | 1.6E2 | 31 | 18 | 31 | 18 | 0.42 |
| vU | ng/ml | 1.0E-9 | 1.0E-9 | 2.3E1 | 2.1E1 | 3.5E1 | 3.6E1 | 1.0E-9 | 1.0E-9 | 1.2E2 | 1.1E2 | 31 | 18 | 31 | 18 | 0.46 |
| vQ | ng/ml | 4.0E2 | 4.4E2 | 4.1E2 | 4.1E2 | 1.6E2 | 1.7E2 | 7.2E1 | 1.2E2 | 8.4E2 | 6.7E2 | 31 | 18 | 31 | 18 | 0.51 |
| vO | ng/ml | 1.7E3 | 1.6E3 | 1.8E3 | 1.7E3 | 4.6E2 | 4.9E2 | 1.1E3 | 1.0E3 | 2.9E3 | 3.2E3 | 31 | 18 | 31 | 18 | 0.44 |
| vS | ng/ml | 1.3E3 | 1.3E3 | 1.2E3 | 1.3E3 | 4.2E2 | 5.6E2 | 1.0E-9 | 1.0E-9 | 2.1E3 | 2.0E3 | 31 | 18 | 31 | 18 | 0.52 |
| vV | ng/ml | 9.2E2 | 7.6E2 | 9.3E2 | 1.2E3 | 6.4E2 | 1.3E3 | 1.1E2 | 1.0E2 | 2.4E3 | 4.6E3 | 31 | 18 | 31 | 18 | 0.49 |
| vW | ng/ml | 1.2E2 | 1.1E2 | 1.5E2 | 1.9E2 | 7.6E1 | 1.9E2 | 4.3E1 | 6.0E1 | 2.9E2 | 7.7E2 | 31 | 18 | 31 | 18 | 0.50 |
| pF | pg/ml | 7.7E-1 | 5.2E-1 | 1.1E0 | 2.6E0 | 1.4E0 | 1.3E1 | 1.0E-9 | 1.0E-9 | 9.4E0 | 8.7E1 | 58 | 45 | 58 | 45 | 0.40 |
| pH | ng/ml | 6.9E0 | 8.9E0 | 9.0E0 | 1.0E1 | 5.2E0 | 5.2E0 | 3.0E0 | 1.2E0 | 2.0E1 | 2.3E1 | 15 | 14 | 15 | 14 | 0.60 |
| pI | ng/ml | 7.1E1 | 6.3E1 | 6.9E1 | 7.6E1 | 3.2E1 | 5.7E1 | 2.6E1 | 2.3E1 | 1.5E2 | 2.0E2 | 15 | 14 | 15 | 14 | 0.47 |
| pK | ng/ml | 4.0E-1 | 5.7E-1 | 3.8E-1 | 5.7E-1 | 1.7E-1 | 2.0E-1 | 1.7E-1 | 2.7E-1 | 7.7E-1 | 8.6E-1 | 15 | 14 | 15 | 14 | 0.79 |

Figure 42.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Wm | % | 8.5E-2 | 2.4E0 | 2.1E1 | 4.3E1 | 1.3E2 | 7.4E1 | 5.4E-2 | 8.5E-2 | 1.0E3 | 1.9E2 | 151 | 7 | 151 | 7 | 0.64 |
| Po | pg/ml | 3.0E-1 | 3.0E1 | 8.3E0 | 7.1E1 | 2.6E1 | 7.6E1 | 8.0E-3 | 2.6E-2 | 2.7E2 | 2.2E2 | 341 | 12 | 341 | 12 | 0.83 |
| Et | ng/ml | 1.4E3 | 4.0E3 | 1.7E3 | 3.4E3 | 1.1E3 | 1.5E3 | 7.5E1 | 5.9E2 | 4.8E3 | 5.0E3 | 340 | 12 | 340 | 12 | 0.81 |
| Fp | ng/ml | 1.3E1 | 4.6E1 | 2.3E1 | 4.4E1 | 2.7E1 | 3.4E1 | 6.0E-3 | 2.3E0 | 1.3E2 | 1.3E2 | 343 | 12 | 343 | 12 | 0.72 |
| Fr | ng/ml | 3.5E4 | 6.0E5 | 1.2E5 | 5.2E5 | 1.8E5 | 3.0E5 | 1.9E2 | 7.0E3 | 8.4E5 | 8.4E5 | 348 | 14 | 348 | 14 | 0.84 |
| Nm | pg/ml | 1.3E4 | 5.3E4 | 3.3E4 | 1.5E5 | 8.2E4 | 2.4E5 | 1.0E-9 | 1.0E-9 | 9.6E5 | 8.2E5 | 344 | 12 | 344 | 12 | 0.74 |
| Nn | pg/ml | 1.6E2 | 8.3E3 | 1.4E3 | 4.9E4 | 6.6E3 | 8.9E4 | 1.0E-9 | 1.0E-9 | 9.5E4 | 3.1E5 | 344 | 12 | 344 | 12 | 0.85 |
| No | pg/ml | 1.5E1 | 1.6E2 | 3.2E1 | 3.1E2 | 5.7E1 | 3.4E2 | 1.0E-9 | 1.6E0 | 5.6E2 | 9.1E2 | 344 | 12 | 344 | 12 | 0.77 |
| Nq | pg/ml | 1.9E0 | 7.7E1 | 1.9E1 | 1.7E2 | 6.6E1 | 2.2E2 | 1.0E-9 | 1.0E-9 | 6.7E2 | 6.3E2 | 344 | 12 | 344 | 12 | 0.80 |
| Nr | pg/ml | 1.5E0 | 3.8E1 | 2.1E1 | 2.2E2 | 7.4E1 | 4.1E2 | 1.0E-9 | 1.0E-9 | 9.8E2 | 1.4E3 | 344 | 12 | 344 | 12 | 0.77 |
| Ns | pg/ml | 1.0E-9 | 1.0E-9 | 7.8E0 | 3.0E-1 | 6.4E1 | 1.0E0 | 1.0E-9 | 1.0E-9 | 1.1E3 | 3.6E0 | 344 | 12 | 344 | 12 | 0.51 |
| Nt | pg/ml | 1.0E2 | 3.0E2 | 1.3E2 | 4.8E2 | 9.9E1 | 5.2E2 | 9.8E-1 | 7.5E1 | 8.8E2 | 1.7E3 | 344 | 12 | 344 | 12 | 0.77 |
| Nu | pg/ml | 1.7E1 | 1.2E2 | 5.5E1 | 1.3E2 | 9.1E1 | 1.1E2 | 1.0E-9 | 1.0E-9 | 6.3E2 | 3.7E2 | 344 | 12 | 344 | 12 | 0.74 |
| Lu | pg/ml | 1.0E4 | 5.9E3 | 1.6E4 | 6.9E3 | 4.0E4 | 4.7E3 | 7.7E2 | 5.2E2 | 5.6E5 | 1.7E4 | 344 | 12 | 344 | 12 | 0.34 |
| Lv | pg/ml | 1.0E-9 | 5.9E1 | 1.5E1 | 6.0E1 | 3.1E1 | 4.8E1 | 1.0E-9 | 1.0E-9 | 2.6E2 | 1.6E2 | 344 | 12 | 344 | 12 | 0.78 |
| Lw | pg/ml | 1.0E-9 | 1.0E-9 | 4.7E-1 | 2.2E1 | 5.0E0 | 5.1E1 | 1.0E-9 | 1.0E-9 | 8.0E1 | 1.8E2 | 344 | 12 | 344 | 12 | 0.66 |
| Lx | pg/ml | 1.0E-9 | 1.3E3 | 1.7E2 | 3.0E3 | 5.5E2 | 6.2E3 | 1.0E-9 | 1.0E-9 | 6.2E3 | 2.2E4 | 344 | 12 | 344 | 12 | 0.84 |
| Ly | pg/ml | 1.0E-9 | 1.0E-9 | 9.1E0 | 9.1E0 | 1.8E1 | 1.8E1 | 1.0E-9 | 1.0E-9 | 1.1E2 | 6.0E1 | 344 | 12 | 344 | 12 | 0.53 |
| Lz | pg/ml | 1.0E-9 | 1.0E-9 | 3.2E0 | 1.1E1 | 2.6E1 | 2.1E1 | 1.0E-9 | 1.0E-9 | 4.3E2 | 6.2E1 | 344 | 12 | 344 | 12 | 0.63 |
| Ma | pg/ml | 3.9E2 | 4.9E3 | 2.2E3 | 8.2E3 | 6.0E3 | 1.1E4 | 1.0E-9 | 2.4E1 | 6.5E4 | 3.6E4 | 344 | 12 | 344 | 12 | 0.78 |
| Mb | pg/ml | 2.5E1 | 2.2E1 | 3.2E1 | 2.7E1 | 1.8E1 | 1.6E1 | 9.2E0 | 4.1E0 | 2.1E2 | 5.3E1 | 344 | 12 | 344 | 12 | 0.39 |
| Mc | pg/ml | 1.0E-9 | 1.0E-9 | 5.5E-2 | 1.0E-9 | 7.4E-1 | 0.0E0 | 1.0E-9 | 1.0E-9 | 1.3E1 | 1.0E-9 | 344 | 12 | 344 | 12 | 0.49 |
| Md | pg/ml | 1.0E-9 | 1.0E-9 | 5.7E-1 | 3.4E0 | 5.2E0 | 8.4E0 | 1.0E-9 | 1.0E-9 | 6.8E1 | 2.9E1 | 344 | 12 | 344 | 12 | 0.68 |
| Me | pg/ml | 3.2E1 | 2.9E1 | 3.1E1 | 3.7E1 | 2.4E1 | 4.8E1 | 1.0E-9 | 1.0E-9 | 3.2E2 | 1.8E2 | 344 | 12 | 344 | 12 | 0.45 |
| Mf | pg/ml | 1.0E-9 | 1.0E-9 | 6.4E-1 | 5.0E-1 | 3.9E0 | 1.3E0 | 1.0E-9 | 1.0E-9 | 5.6E1 | 4.5E0 | 344 | 12 | 344 | 12 | 0.58 |
| Mg | pg/ml | 1.0E0 | 8.0E0 | 6.2E0 | 2.9E1 | 1.2E1 | 4.7E1 | 1.0E-9 | 1.0E-9 | 9.2E1 | 1.5E2 | 344 | 12 | 344 | 12 | 0.59 |
| Mh | pg/ml | 1.0E-9 | 1.4E-1 | 1.1E0 | 3.1E0 | 7.6E0 | 6.0E0 | 1.0E-9 | 1.0E-9 | 1.1E2 | 1.8E1 | 344 | 12 | 344 | 12 | 0.70 |
| Mi | pg/ml | 1.0E-9 | 4.7E0 | 1.0E0 | 8.4E1 | 8.3E0 | 1.7E2 | 1.0E-9 | 1.0E-9 | 1.2E2 | 5.2E2 | 344 | 12 | 344 | 12 | 0.74 |
| Mj | pg/ml | 1.0E-9 | 1.3E1 | 5.7E0 | 5.1E1 | 3.1E1 | 7.4E1 | 1.0E-9 | 1.0E-9 | 4.6E2 | 2.1E2 | 344 | 12 | 344 | 12 | 0.69 |
| Mk | pg/ml | 1.8E0 | 6.3E0 | 1.5E1 | 6.0E1 | 9.2E1 | 1.4E2 | 1.0E-9 | 1.0E-9 | 1.3E3 | 5.0E2 | 344 | 12 | 344 | 12 | 0.65 |
| Ml | pg/ml | 1.0E-9 | 1.0E-9 | 9.9E0 | 4.4E1 | 1.2E2 | 1.5E2 | 1.0E-9 | 1.0E-9 | 2.1E3 | 5.1E2 | 344 | 12 | 344 | 12 | 0.53 |
| Mm | pg/ml | 5.5E2 | 3.0E3 | 1.0E3 | 3.3E3 | 1.3E3 | 3.0E3 | 1.0E-9 | 1.0E-9 | 1.0E4 | 1.0E4 | 344 | 12 | 344 | 12 | 0.73 |
| Mn | pg/ml | 5.7E0 | 1.3E1 | 1.1E1 | 1.7E1 | 2.5E1 | 1.3E1 | 1.0E-9 | 1.1E0 | 3.5E2 | 5.1E1 | 344 | 12 | 344 | 12 | 0.74 |
| Mp | pg/ml | 1.0E-9 | 6.9E1 | 1.2E1 | 3.0E2 | 4.3E1 | 6.7E2 | 1.0E-9 | 1.0E-9 | 6.3E2 | 2.4E3 | 344 | 12 | 344 | 12 | 0.88 |
| Mq | pg/ml | 1.0E-9 | 1.0E-9 | 2.1E0 | 2.2E1 | 1.3E1 | 3.6E1 | 1.0E-9 | 1.0E-9 | 1.8E2 | 1.0E2 | 344 | 12 | 344 | 12 | 0.64 |
| Mr | pg/ml | 1.0E-9 | 1.7E1 | 2.3E1 | 6.4E2 | 1.2E2 | 1.1E3 | 1.0E-9 | 1.0E-9 | 1.5E3 | 3.4E3 | 344 | 12 | 344 | 12 | 0.73 |
| Ms | pg/ml | 3.3E2 | 2.2E2 | 4.8E2 | 2.9E2 | 5.7E2 | 3.3E2 | 1.0E-9 | 1.0E-9 | 4.7E3 | 9.8E2 | 344 | 12 | 344 | 12 | 0.39 |
| Mt | pg/ml | 2.4E-1 | 7.8E1 | 8.0E0 | 3.5E2 | 4.3E1 | 9.2E2 | 1.0E-9 | 1.0E-9 | 7.1E2 | 3.2E3 | 344 | 12 | 344 | 12 | 0.87 |
| Mu | pg/ml | 1.0E-9 | 4.5E0 | 1.4E0 | 1.1E1 | 1.4E1 | 1.6E1 | 1.0E-9 | 1.0E-9 | 2.3E2 | 4.8E1 | 344 | 12 | 344 | 12 | 0.84 |
| Mv | pg/ml | 1.0E-9 | 2.0E2 | 6.1E1 | 3.6E2 | 3.3E2 | 4.0E2 | 1.0E-9 | 1.0E-9 | 4.5E3 | 9.2E2 | 344 | 12 | 344 | 12 | 0.72 |
| Mw | pg/ml | 3.6E1 | 1.2E3 | 2.5E2 | 1.8E3 | 1.3E3 | 1.9E3 | 1.0E-9 | 1.0E-9 | 1.8E4 | 5.3E3 | 344 | 12 | 344 | 12 | 0.87 |
| Mx | pg/ml | 1.0E-9 | 3.9E-1 | 3.5E-1 | 2.6E0 | 1.9E0 | 5.6E0 | 1.0E-9 | 1.0E-9 | 3.2E1 | 2.0E1 | 344 | 12 | 344 | 12 | 0.72 |
| My | pg/ml | 1.0E-9 | 2.1E2 | 3.2E2 | 4.9E2 | 2.4E3 | 6.9E2 | 1.0E-9 | 1.0E-9 | 3.9E4 | 1.9E3 | 344 | 12 | 344 | 12 | 0.72 |
| Mz | pg/ml | 1.1E1 | 1.1E2 | 2.6E1 | 3.4E2 | 5.0E1 | 6.1E2 | 1.0E-9 | 1.0E-9 | 5.5E2 | 1.9E3 | 344 | 12 | 344 | 12 | 0.80 |
| Na | pg/ml | 1.0E-9 | 1.6E-1 | 6.4E-1 | 5.0E0 | 1.9E0 | 1.2E1 | 1.0E-9 | 1.0E-9 | 9.6E0 | 4.2E1 | 344 | 12 | 344 | 12 | 0.66 |
| Nb | pg/ml | 2.2E0 | 1.1E1 | 3.6E0 | 3.8E1 | 9.9E0 | 6.2E1 | 1.0E-9 | 1.0E-9 | 1.5E2 | 2.1E2 | 344 | 12 | 344 | 12 | 0.75 |
| Nc | pg/ml | 3.3E2 | 6.5E0 | 5.2E2 | 1.8E2 | 7.4E2 | 3.1E2 | 1.0E-9 | 1.0E-9 | 9.2E3 | 7.9E2 | 344 | 12 | 344 | 12 | 0.31 |
| Nd | pg/ml | 2.7E1 | 4.1E1 | 2.7E1 | 2.2E2 | 6.8E1 | 6.0E2 | 1.0E-9 | 7.2E-1 | 1.2E3 | 2.1E3 | 344 | 12 | 344 | 12 | 0.69 |
| Ne | pg/ml | 4.2E2 | 2.7E2 | 5.2E2 | 5.2E2 | 5.4E2 | 9.8E2 | 1.0E-9 | 1.0E-9 | 7.0E3 | 3.6E3 | 344 | 12 | 344 | 12 | 0.38 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nf | pg/ml | 1.0E-9 | 1.0E-9 | 2.3E0 | 2.4E1 | 8.6E0 | 4.7E1 | 1.0E-9 | 1.0E-9 | 8.2E1 | 1.3E2 | 344 | 12 | 344 | 12 | 0.63 |
| Ng | pg/ml | 1.2E1 | 2.7E1 | 9.0E1 | 9.7E1 | 1.8E2 | 1.3E2 | 1.0E-9 | 1.0E-9 | 1.6E3 | 4.2E2 | 344 | 12 | 344 | 12 | 0.52 |
| Nh | pg/ml | 6.2E1 | 3.1E1 | 8.0E1 | 7.4E1 | 7.3E1 | 1.4E2 | 1.0E-9 | 4.1E0 | 5.6E2 | 5.1E2 | 344 | 12 | 344 | 12 | 0.33 |
| Ni | pg/ml | 1.0E-9 | 1.0E-9 | 8.3E1 | 1.6E2 | 1.3E2 | 3.2E2 | 1.0E-9 | 1.0E-9 | 7.1E2 | 1.1E3 | 344 | 12 | 344 | 12 | 0.47 |
| Nj | pg/ml | 7.2E0 | 4.0E0 | 1.1E1 | 6.9E0 | 1.2E1 | 7.6E0 | 1.0E-9 | 1.0E-9 | 6.9E1 | 2.2E1 | 344 | 12 | 344 | 12 | 0.39 |
| Nk | pg/ml | 1.8E1 | 3.5E-1 | 3.3E1 | 1.3E1 | 3.9E1 | 2.2E1 | 1.0E-9 | 1.0E-9 | 2.0E2 | 6.6E1 | 344 | 12 | 344 | 12 | 0.33 |
| Nl | pg/ml | 4.3E1 | 1.0E1 | 5.7E1 | 3.2E1 | 7.5E1 | 5.2E1 | 1.0E-9 | 1.0E-9 | 1.1E3 | 1.8E2 | 344 | 12 | 344 | 12 | 0.31 |
| Hq | pg/ml | 1.2E0 | 1.2E1 | 1.5E2 | 2.6E2 | 1.9E3 | 8.0E2 | 1.0E-9 | 1.0E-9 | 2.8E4 | 2.8E3 | 342 | 12 | 342 | 12 | 0.73 |
| Hr | pg/ml | 8.9E1 | 2.7E2 | 6.3E2 | 1.3E3 | 1.3E3 | 2.6E3 | 1.0E-9 | 1.0E-9 | 1.2E4 | 8.9E3 | 342 | 12 | 342 | 12 | 0.59 |
| Hu | pg/ml | 7.9E0 | 6.5E2 | 4.6E3 | 1.6E3 | 4.0E4 | 2.3E3 | 1.0E-9 | 1.0E-9 | 6.3E5 | 6.5E3 | 342 | 12 | 342 | 12 | 0.71 |
| Hv | pg/ml | 1.4E0 | 3.6E0 | 2.9E0 | 8.3E1 | 1.0E1 | 2.6E2 | 1.0E-9 | 1.0E-9 | 1.6E2 | 8.9E2 | 342 | 12 | 342 | 12 | 0.75 |
| Hw | pg/ml | 6.2E0 | 3.5E1 | 1.5E1 | 8.7E2 | 4.5E1 | 2.7E3 | 1.0E-9 | 4.6E-1 | 6.4E2 | 9.4E3 | 342 | 12 | 342 | 12 | 0.64 |
| Hx | pg/ml | 8.8E0 | 4.4E1 | 5.2E1 | 2.2E2 | 5.0E2 | 4.1E2 | 1.0E-9 | 3.9E0 | 9.3E3 | 1.3E3 | 342 | 12 | 342 | 12 | 0.77 |
| Ih | ng/ml | 6.3E1 | 6.6E2 | 2.5E2 | 8.3E2 | 4.4E2 | 8.3E2 | 1.0E-9 | 2.4E0 | 3.6E3 | 2.8E3 | 343 | 12 | 343 | 12 | 0.73 |
| Ii | ng/ml | 7.9E1 | 4.7E2 | 2.0E2 | 1.1E3 | 4.6E2 | 1.5E3 | 7.3E-1 | 7.5E-1 | 5.2E3 | 4.5E3 | 343 | 12 | 343 | 12 | 0.73 |
| Ij | ng/ml | 7.9E1 | 2.3E2 | 1.8E2 | 2.4E3 | 5.7E2 | 6.9E3 | 2.8E0 | 9.5E0 | 6.4E3 | 2.4E4 | 339 | 12 | 339 | 12 | 0.74 |
| Ik | ng/ml | 1.1E1 | 1.8E1 | 1.3E3 | 2.1E2 | 1.2E4 | 4.3E2 | 5.9E-1 | 6.2E-1 | 1.2E5 | 1.5E3 | 339 | 12 | 339 | 12 | 0.57 |
| Il | ng/ml | 3.6E2 | 7.0E2 | 1.3E3 | 3.6E3 | 2.8E3 | 5.1E3 | 1.0E-9 | 1.9E-1 | 1.2E4 | 1.2E4 | 337 | 12 | 337 | 12 | 0.64 |
| Im | ng/ml | 2.1E2 | 7.0E2 | 4.6E2 | 1.8E3 | 1.0E3 | 2.2E3 | 1.4E1 | 2.2E1 | 1.5E4 | 6.2E3 | 339 | 12 | 339 | 12 | 0.78 |
| In | ng/ml | 3.4E0 | 2.8E0 | 2.0E1 | 4.5E2 | 8.9E1 | 1.3E3 | 1.0E-9 | 1.0E-9 | 1.1E3 | 4.5E3 | 343 | 12 | 343 | 12 | 0.55 |
| Io | ng/ml | 9.6E3 | 9.4E3 | 2.0E4 | 1.3E4 | 4.6E4 | 1.2E4 | 1.0E-9 | 1.0E3 | 7.1E5 | 3.3E4 | 343 | 12 | 343 | 12 | 0.47 |
| Ip | ng/ml | 1.0E1 | 4.2E1 | 2.1E1 | 4.2E1 | 2.6E1 | 1.9E1 | 1.0E-9 | 9.8E0 | 2.3E2 | 7.1E1 | 343 | 12 | 343 | 12 | 0.79 |
| Iq | ug/ml | 1.0E-1 | 2.5E0 | 4.0E1 | 2.4E1 | 7.3E2 | 6.2E1 | 1.0E-9 | 1.0E-9 | 1.4E4 | 2.2E2 | 343 | 12 | 343 | 12 | 0.75 |
| Ir | ug/ml | 3.7E-1 | 6.0E0 | 4.5E0 | 5.8E1 | 3.2E1 | 1.1E2 | 1.0E-9 | 3.5E-2 | 5.1E2 | 3.7E2 | 342 | 12 | 342 | 12 | 0.78 |
| Is | ng/ml | 2.0E0 | 4.0E1 | 9.1E0 | 6.4E1 | 3.4E1 | 7.5E1 | 1.0E-9 | 4.3E-1 | 5.5E2 | 2.6E2 | 343 | 12 | 343 | 12 | 0.87 |
| It | ng/ml | 2.1E0 | 8.1E0 | 2.2E1 | 6.8E1 | 1.0E2 | 1.9E2 | 1.0E-9 | 1.0E-9 | 1.1E3 | 6.8E2 | 343 | 12 | 343 | 12 | 0.68 |
| Iu | ng/ml | 1.6E2 | 1.2E3 | 1.2E3 | 7.4E3 | 3.9E3 | 1.1E4 | 1.0E-9 | 1.0E-9 | 2.9E4 | 2.4E4 | 343 | 12 | 343 | 12 | 0.68 |
| Iv | ng/ml | 1.3E1 | 1.1E2 | 9.4E1 | 7.8E2 | 8.8E2 | 1.4E3 | 1.0E-9 | 1.0E0 | 1.6E4 | 3.8E3 | 342 | 12 | 342 | 12 | 0.74 |
| Pz | ng/ml | 3.6E3 | 4.0E3 | 5.6E3 | 5.8E3 | 6.0E3 | 4.4E3 | 1.6E1 | 4.0E1 | 7.0E4 | 1.3E4 | 339 | 12 | 339 | 12 | 0.56 |
| Qa | ng/ml | 3.6E3 | 2.3E4 | 6.9E3 | 3.5E4 | 7.9E3 | 6.0E4 | 1.5E2 | 9.4E2 | 4.2E4 | 2.2E5 | 339 | 12 | 339 | 12 | 0.82 |
| Qb | ng/ml | 1.1E2 | 3.8E2 | 2.3E2 | 4.5E2 | 4.4E2 | 4.1E2 | 7.9E-1 | 3.2E1 | 5.3E3 | 1.6E3 | 339 | 12 | 339 | 12 | 0.74 |
| Qc | ng/ml | 2.1E2 | 5.4E2 | 4.5E2 | 6.8E2 | 5.9E2 | 8.3E2 | 1.0E-9 | 1.3E1 | 4.3E3 | 2.8E3 | 339 | 12 | 339 | 12 | 0.57 |
| Qd | ng/ml | 8.9E3 | 1.0E5 | 2.5E4 | 1.0E5 | 1.2E5 | 8.7E4 | 1.5E2 | 1.9E3 | 2.0E6 | 2.3E5 | 339 | 12 | 339 | 12 | 0.77 |
| Qe | ng/ml | 8.8E2 | 4.4E3 | 2.0E3 | 5.2E3 | 5.6E3 | 4.9E3 | 1.0E-9 | 1.2E2 | 9.7E4 | 1.8E4 | 339 | 12 | 339 | 12 | 0.77 |
| Jg | ng/ml | 4.6E2 | 2.2E3 | 7.7E2 | 2.2E3 | 9.4E2 | 1.9E3 | 5.8E0 | 8.4E1 | 1.0E4 | 7.1E3 | 342 | 12 | 342 | 12 | 0.77 |
| Jh | ng/ml | 2.9E0 | 7.8E1 | 2.0E1 | 1.2E2 | 8.1E1 | 1.4E2 | 1.0E-9 | 1.0E-9 | 1.2E3 | 4.9E2 | 342 | 12 | 342 | 12 | 0.82 |
| Ji | ng/ml | 5.6E1 | 3.4E2 | 8.6E1 | 4.1E2 | 9.6E1 | 3.4E2 | 1.1E0 | 2.3E1 | 6.9E2 | 1.3E3 | 342 | 12 | 342 | 12 | 0.85 |
| Jj | ng/ml | 5.4E2 | 1.0E2 | 1.9E3 | 2.8E2 | 1.8E4 | 3.6E2 | 2.3E0 | 8.7E0 | 3.4E5 | 1.0E3 | 342 | 12 | 342 | 12 | 0.23 |
| Jk | ng/ml | 2.6E0 | 6.5E1 | 2.0E1 | 8.9E1 | 4.6E1 | 8.4E1 | 1.0E-9 | 1.2E-1 | 3.9E2 | 2.4E2 | 342 | 12 | 342 | 12 | 0.77 |
| Jl | ng/ml | 4.8E-1 | 1.5E1 | 1.9E0 | 8.4E2 | 4.7E0 | 2.9E3 | 1.2E-3 | 2.5E-1 | 4.0E1 | 9.9E3 | 342 | 12 | 342 | 12 | 0.87 |
| Jm | ng/ml | 2.0E1 | 3.1E1 | 6.7E1 | 7.5E1 | 1.7E2 | 1.1E2 | 1.0E-9 | 4.0E-1 | 2.1E3 | 3.6E2 | 342 | 12 | 342 | 12 | 0.57 |
| Jn | pg/ml | 3.4E-1 | 1.9E0 | 4.0E0 | 1.2E2 | 3.6E1 | 2.6E2 | 1.0E-9 | 2.4E-1 | 6.2E2 | 7.3E2 | 342 | 12 | 342 | 12 | 0.81 |
| Jo | pg/ml | 3.8E3 | 4.1E3 | 4.8E3 | 1.7E4 | 3.9E3 | 2.9E4 | 2.0E1 | 2.4E1 | 2.4E4 | 1.0E5 | 342 | 12 | 342 | 12 | 0.58 |
| Jp | pg/ml | 7.1E4 | 1.1E5 | 7.4E4 | 1.1E5 | 3.9E4 | 4.1E4 | 5.8E2 | 6.5E4 | 3.8E5 | 2.1E5 | 342 | 12 | 342 | 12 | 0.79 |
| Jq | pg/ml | 9.6E1 | 4.0E2 | 1.6E2 | 1.5E3 | 2.0E2 | 2.5E3 | 1.0E0 | 1.3E1 | 2.0E3 | 8.7E3 | 342 | 12 | 342 | 12 | 0.76 |
| Jr | pg/ml | 4.2E0 | 3.4E1 | 5.9E1 | 1.1E3 | 6.0E2 | 2.5E3 | 1.0E-9 | 6.7E0 | 1.1E4 | 7.4E3 | 342 | 12 | 342 | 12 | 0.86 |
| Js | pg/ml | 1.5E1 | 3.4E1 | 7.4E1 | 5.7E2 | 5.7E2 | 1.1E3 | 1.0E-9 | 2.7E0 | 1.0E4 | 3.0E3 | 342 | 12 | 342 | 12 | 0.74 |
| Jt | pg/ml | 2.4E3 | 4.8E3 | 3.0E3 | 1.3E4 | 2.4E3 | 1.7E4 | 2.2E1 | 1.5E2 | 2.2E4 | 5.2E4 | 342 | 12 | 342 | 12 | 0.68 |
| Lh | pg/ml | 1.3E4 | 3.7E4 | 2.1E4 | 1.5E5 | 2.7E4 | 1.8E5 | 1.0E-9 | 1.8E3 | 2.6E5 | 4.8E5 | 343 | 12 | 343 | 12 | 0.77 |
| Li | pg/ml | 3.5E3 | 9.3E4 | 1.9E4 | 1.2E5 | 8.9E4 | 1.4E5 | 1.2E1 | 8.4E1 | 1.3E6 | 4.1E5 | 343 | 12 | 343 | 12 | 0.75 |
| Lj | pg/ml | 2.9E3 | 9.2E3 | 2.0E4 | 4.8E4 | 5.6E4 | 1.1E5 | 1.0E-9 | 8.9E1 | 4.3E5 | 3.9E5 | 343 | 12 | 343 | 12 | 0.65 |
| Nv | pg/ml | 3.9E3 | 2.8E4 | 9.2E3 | 4.5E4 | 1.8E4 | 4.5E4 | 1.0E-9 | 1.6E2 | 1.6E5 | 1.2E5 | 344 | 12 | 344 | 12 | 0.78 |
| Nw | pg/ml | 9.6E3 | 2.7E4 | 1.3E4 | 6.1E4 | 1.6E4 | 7.5E4 | 1.9E2 | 4.5E3 | 2.1E5 | 2.2E5 | 344 | 12 | 344 | 12 | 0.82 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Nx | pg/ml | 2.2E2 | 1.1E3 | 4.3E2 | 1.1E3 | 6.1E2 | 1.2E3 | 1.0E-9 | 1.0E-9 | 3.5E3 | 4.1E3 | 344 | 12 | 344 | 12 | 0.69 |
| Ny | pg/ml | 6.5E0 | 1.5E2 | 9.7E1 | 4.0E2 | 1.3E3 | 7.7E2 | 1.0E-9 | 1.0E-9 | 2.5E4 | 2.8E3 | 344 | 12 | 344 | 12 | 0.80 |
| Oe | pg/ml | 3.5E1 | 1.0E-9 | 2.5E2 | 1.6E2 | 3.8E2 | 3.6E2 | 1.0E-9 | 1.0E-9 | 2.3E3 | 1.1E3 | 342 | 12 | 342 | 12 | 0.39 |
| Of | pg/ml | 1.3E2 | 1.2E2 | 4.9E3 | 8.9E3 | 2.0E4 | 1.9E4 | 1.0E-9 | 1.0E-9 | 1.9E5 | 6.6E4 | 344 | 12 | 344 | 12 | 0.55 |
| Og | pg/ml | 7.0E-2 | 9.2E-3 | 3.6E-1 | 5.8E-2 | 1.5E0 | 9.3E-2 | 1.0E-9 | 1.0E-9 | 1.9E1 | 3.2E-1 | 344 | 12 | 344 | 12 | 0.36 |
| Oh | pg/ml | 2.5E0 | 3.8E1 | 1.4E1 | 1.4E3 | 8.8E1 | 4.5E3 | 1.0E-9 | 1.0E-9 | 1.4E3 | 1.6E4 | 344 | 12 | 344 | 12 | 0.79 |
| Oi | pg/ml | 2.0E0 | 1.0E-9 | 5.0E0 | 6.1E0 | 7.8E0 | 9.6E0 | 1.0E-9 | 1.0E-9 | 6.7E1 | 3.1E1 | 344 | 12 | 344 | 12 | 0.46 |
| Ok | pg/ml | 3.9E2 | 1.3E3 | 5.4E2 | 2.1E3 | 6.2E2 | 2.3E3 | 1.5E1 | 5.3E1 | 7.0E3 | 7.8E3 | 344 | 12 | 344 | 12 | 0.78 |
| Om | pg/ml | 4.2E2 | 2.9E3 | 7.9E2 | 6.8E3 | 2.0E3 | 1.4E4 | 1.0E-9 | 7.0E1 | 3.0E4 | 5.1E4 | 344 | 12 | 344 | 12 | 0.81 |
| On | pg/ml | 1.8E2 | 1.3E3 | 2.9E2 | 2.0E3 | 4.1E2 | 2.3E3 | 1.0E-9 | 1.6E1 | 4.5E3 | 8.5E3 | 344 | 12 | 344 | 12 | 0.87 |
| Oy | pg/ml | 4.7E-1 | 3.5E0 | 6.0E0 | 8.2E0 | 3.1E1 | 1.0E1 | 1.0E-9 | 1.0E-9 | 4.0E2 | 3.3E1 | 343 | 12 | 343 | 12 | 0.68 |
| Oz | pg/ml | 4.5E-3 | 1.0E-9 | 3.2E-1 | 2.4E0 | 1.6E0 | 8.1E0 | 1.0E-9 | 1.0E-9 | 2.9E1 | 2.8E1 | 343 | 12 | 343 | 12 | 0.43 |
| Pa | pg/ml | 3.9E-1 | 4.7E0 | 1.3E0 | 3.3E1 | 5.6E0 | 6.7E1 | 1.0E-9 | 1.0E-9 | 8.6E1 | 2.3E2 | 343 | 12 | 343 | 12 | 0.74 |
| Pb | pg/ml | 1.0E-9 | 1.0E-9 | 1.6E0 | 2.8E0 | 2.6E1 | 9.1E0 | 1.0E-9 | 1.0E-9 | 4.9E2 | 3.2E1 | 343 | 12 | 343 | 12 | 0.46 |
| Pc | pg/ml | 4.4E-2 | 1.0E-9 | 3.7E-1 | 3.1E1 | 8.8E-1 | 9.4E1 | 1.0E-9 | 1.0E-9 | 1.4E1 | 3.3E2 | 343 | 12 | 343 | 12 | 0.53 |
| Pd | pg/ml | 1.6E0 | 1.4E1 | 6.2E0 | 2.6E1 | 4.6E1 | 3.5E1 | 1.0E-9 | 7.3E-2 | 8.4E2 | 1.2E2 | 343 | 12 | 343 | 12 | 0.68 |
| Pe | pg/ml | 2.2E1 | 4.4E2 | 1.1E2 | 2.5E3 | 4.4E2 | 4.5E3 | 1.0E-9 | 3.3E0 | 4.7E3 | 1.5E4 | 343 | 12 | 343 | 12 | 0.81 |
| Pf | pg/ml | 1.6E0 | 2.6E1 | 1.5E1 | 7.2E1 | 9.0E1 | 1.2E2 | 1.0E-9 | 1.0E-9 | 1.5E3 | 4.3E2 | 343 | 12 | 343 | 12 | 0.85 |
| Pg | pg/ml | 3.9E0 | 2.1E2 | 6.8E1 | 5.3E2 | 5.1E2 | 7.0E2 | 1.0E-9 | 4.6E-1 | 7.7E3 | 2.2E3 | 343 | 12 | 343 | 12 | 0.81 |
| aA | mg/dL | 9.0E-1 | 2.8E0 | 1.0E0 | 2.4E0 | 5.0E-1 | 1.3E0 | 3.0E-1 | 5.5E-1 | 4.2E0 | 4.7E0 | 521 | 16 | 521 | 16 | 0.82 |
| aC | mg/mL | 2.2E0 | 2.4E0 | 2.6E0 | 3.0E0 | 1.3E0 | 1.9E0 | 7.5E-1 | 1.1E0 | 7.4E0 | 5.5E0 | 169 | 7 | 169 | 7 | 0.53 |
| aD | ug/mL | 2.9E0 | 3.7E0 | 4.6E0 | 6.3E0 | 4.7E0 | 5.9E0 | 7.5E-1 | 1.8E0 | 3.5E1 | 1.8E1 | 169 | 7 | 169 | 7 | 0.59 |
| aE | mg/mL | 5.7E-1 | 5.5E-1 | 5.9E-1 | 5.6E-1 | 1.7E-1 | 1.4E-1 | 1.8E-1 | 3.9E-1 | 1.2E0 | 7.2E-1 | 169 | 7 | 169 | 7 | 0.46 |
| aF | ng/mL | 2.2E0 | 9.1E0 | 4.9E0 | 8.7E0 | 7.7E0 | 5.2E0 | 4.3E-3 | 4.3E-3 | 5.0E1 | 1.5E1 | 169 | 7 | 169 | 7 | 0.74 |
| aG | mg/mL | 1.4E-1 | 9.5E-2 | 1.6E-1 | 1.2E-1 | 8.7E-2 | 4.2E-2 | 3.2E-2 | 6.9E-2 | 4.8E-1 | 1.7E-1 | 169 | 7 | 169 | 7 | 0.37 |
| aH | ug/mL | 7.1E1 | 5.6E1 | 7.8E1 | 6.0E1 | 4.1E1 | 2.8E1 | 8.9E0 | 1.1E1 | 2.0E2 | 1.0E2 | 169 | 7 | 169 | 7 | 0.41 |
| aI | ug/mL | 1.7E2 | 1.4E2 | 1.8E2 | 1.2E2 | 6.1E1 | 2.8E1 | 3.2E1 | 7.6E1 | 3.4E2 | 1.6E2 | 169 | 7 | 169 | 7 | 0.23 |
| aJ | ug/mL | 2.4E0 | 7.0E0 | 3.1E0 | 9.1E0 | 2.2E0 | 6.7E0 | 8.2E-1 | 2.2E0 | 1.4E1 | 2.3E1 | 169 | 7 | 169 | 7 | 0.87 |
| aK | ng/mL | 1.3E0 | 1.6E0 | 2.0E0 | 2.0E0 | 1.9E0 | 2.3E0 | 2.9E-4 | 1.3E-1 | 1.0E1 | 6.5E0 | 169 | 7 | 169 | 7 | 0.48 |
| aL | mg/mL | 7.4E-1 | 7.3E-1 | 7.7E-1 | 6.5E-1 | 2.6E-1 | 2.5E-1 | 2.2E-1 | 2.7E-1 | 1.7E0 | 9.2E-1 | 169 | 7 | 169 | 7 | 0.40 |
| aM | U/mL | 1.9E1 | 4.7E1 | 3.9E1 | 1.9E2 | 6.9E1 | 3.1E2 | 4.2E-2 | 4.2E-2 | 6.8E2 | 8.2E2 | 169 | 7 | 169 | 7 | 0.70 |
| aN | U/mL | 1.5E1 | 2.6E1 | 2.5E1 | 3.5E1 | 4.3E1 | 2.9E1 | 2.5E-3 | 7.4E0 | 3.8E2 | 8.8E1 | 169 | 7 | 169 | 7 | 0.68 |
| aO | pg/mL | 4.9E1 | 1.3E3 | 4.1E2 | 1.1E3 | 9.8E2 | 9.6E2 | 6.0E-2 | 7.2E1 | 6.6E3 | 2.4E3 | 169 | 7 | 169 | 7 | 0.82 |
| aP | ng/mL | 1.6E0 | 5.0E0 | 2.2E0 | 7.7E0 | 2.4E0 | 9.1E0 | 4.5E-1 | 1.6E0 | 2.8E1 | 2.8E1 | 169 | 7 | 169 | 7 | 0.88 |
| aQ | ng/mL | 2.4E-1 | 4.1E-1 | 3.5E-1 | 3.9E-1 | 3.2E-1 | 2.9E-1 | 2.0E-4 | 5.1E-2 | 2.0E0 | 9.0E-1 | 169 | 7 | 169 | 7 | 0.55 |
| aR | ng/mL | 1.7E0 | 4.6E0 | 3.0E0 | 3.9E0 | 4.1E0 | 1.7E0 | 2.6E-1 | 6.5E-1 | 3.4E1 | 5.5E0 | 169 | 7 | 169 | 7 | 0.71 |
| aS | ng/mL | 3.7E-1 | 5.4E-1 | 9.9E-1 | 1.1E0 | 2.7E0 | 1.2E0 | 4.2E-3 | 8.0E-2 | 3.3E1 | 2.8E0 | 169 | 7 | 169 | 7 | 0.56 |
| aU | pg/mL | 6.6E1 | 7.8E1 | 9.9E1 | 1.2E2 | 1.0E2 | 1.8E2 | 7.4E-2 | 7.4E-2 | 7.0E2 | 5.1E2 | 169 | 7 | 169 | 7 | 0.47 |
| aV | ng/mL | 5.8E-1 | 5.8E-1 | 1.1E0 | 7.8E-1 | 2.6E0 | 8.3E-1 | 7.6E-4 | 1.0E-1 | 3.3E1 | 2.4E0 | 169 | 7 | 169 | 7 | 0.46 |
| aW | pg/mL | 1.9E1 | 3.1E1 | 2.2E1 | 2.6E1 | 3.4E1 | 1.5E1 | 7.2E-2 | 7.2E-2 | 4.2E2 | 4.7E1 | 169 | 7 | 169 | 7 | 0.68 |
| aX | ng/mL | 8.0E0 | 1.8E1 | 1.5E1 | 4.5E1 | 3.1E1 | 5.3E1 | 3.0E-1 | 2.6E0 | 3.1E2 | 1.3E2 | 169 | 7 | 169 | 7 | 0.67 |
| aY | pg/mL | 5.3E1 | 4.7E1 | 7.6E1 | 7.1E1 | 1.1E2 | 6.1E1 | 4.1E-1 | 2.7E1 | 1.2E3 | 2.0E2 | 169 | 7 | 169 | 7 | 0.50 |
| aZ | pg/mL | 2.3E2 | 2.9E2 | 6.8E2 | 5.2E2 | 1.4E3 | 7.0E2 | 1.7E0 | 7.5E1 | 1.2E4 | 2.1E3 | 169 | 7 | 169 | 7 | 0.56 |
| bA | ng/mL | 1.3E1 | 1.5E2 | 6.0E1 | 4.2E2 | 1.3E2 | 5.6E2 | 3.0E-2 | 4.7E0 | 9.4E2 | 1.5E3 | 169 | 7 | 169 | 7 | 0.82 |
| bB | ng/mL | 2.8E2 | 1.9E2 | 3.1E2 | 2.0E2 | 1.8E2 | 1.1E2 | 2.1E0 | 3.3E1 | 9.5E2 | 3.8E2 | 169 | 7 | 169 | 7 | 0.34 |
| bC | ng/mL | 3.2E2 | 1.6E3 | 6.0E2 | 1.8E3 | 8.1E2 | 1.7E3 | 1.4E1 | 1.4E2 | 4.7E3 | 4.0E3 | 169 | 7 | 169 | 7 | 0.75 |
| bE | mg/mL | 5.1E0 | 5.5E0 | 5.4E0 | 6.6E0 | 2.1E0 | 3.8E0 | 1.8E0 | 1.3E0 | 1.2E1 | 1.1E1 | 169 | 7 | 169 | 7 | 0.58 |
| bF | pg/mL | 3.5E1 | 6.8E1 | 3.5E2 | 3.3E2 | 1.4E3 | 6.8E2 | 5.0E-2 | 2.0E1 | 1.1E4 | 1.9E3 | 169 | 7 | 169 | 7 | 0.71 |
| bG | ng/mL | 1.7E0 | 2.6E0 | 3.1E0 | 5.4E0 | 4.3E0 | 5.8E0 | 1.1E-1 | 2.4E-1 | 3.0E1 | 1.5E1 | 169 | 7 | 169 | 7 | 0.63 |
| bH | pg/mL | 5.7E-1 | 1.3E1 | 6.1E0 | 9.7E0 | 2.4E1 | 9.0E0 | 5.7E-1 | 5.7E-1 | 2.8E2 | 2.3E1 | 169 | 7 | 169 | 7 | 0.68 |
| bI | ng/mL | 4.0E-3 | 4.0E-3 | 9.0E-2 | 1.6E-1 | 2.0E-1 | 2.1E-1 | 4.0E-3 | 4.0E-3 | 9.8E-1 | 5.1E-1 | 169 | 7 | 169 | 7 | 0.61 |
| bJ | mg/mL | 1.9E0 | 2.3E0 | 2.4E0 | 2.7E0 | 2.0E0 | 2.9E0 | 2.5E-4 | 2.5E-4 | 1.1E1 | 8.9E0 | 169 | 7 | 169 | 7 | 0.52 |
| bL | pg/mL | 3.7E0 | 1.1E1 | 9.1E0 | 1.3E1 | 1.2E1 | 8.0E0 | 4.6E-2 | 3.0E0 | 6.0E1 | 2.4E1 | 169 | 7 | 169 | 7 | 0.71 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| bM | mg/mL | 1.8E0 | 1.8E0 | 2.2E0 | 1.9E0 | 1.5E0 | 1.1E0 | 1.6E-2 | 5.5E-1 | 8.6E0 | 3.8E0 | 169 | 7 | 169 | 7 | 0.47 |
| bN | ng/mL | 3.3E1 | 6.0E1 | 1.2E2 | 9.5E1 | 2.9E2 | 9.0E1 | 1.4E-1 | 6.7E0 | 1.9E3 | 2.5E2 | 169 | 7 | 169 | 7 | 0.60 |
| bO | ng/mL | 4.0E-2 | 4.0E-2 | 9.4E0 | 4.0E-2 | 2.0E1 | 0.0E0 | 4.0E-2 | 4.0E-2 | 1.3E2 | 4.0E-2 | 169 | 7 | 169 | 7 | 0.33 |
| bP | mg/mL | 5.3E-1 | 4.9E-1 | 7.5E-1 | 7.1E-1 | 7.1E-1 | 5.4E-1 | 4.9E-2 | 9.2E-2 | 4.8E0 | 1.6E0 | 169 | 7 | 169 | 7 | 0.50 |
| bQ | pg/mL | 2.1E1 | 9.3E1 | 1.4E2 | 1.1E2 | 1.0E3 | 6.2E1 | 1.5E-1 | 2.2E1 | 1.3E4 | 2.2E2 | 169 | 7 | 169 | 7 | 0.85 |
| bR | ng/mL | 1.2E-2 | 1.2E-2 | 1.6E-1 | 1.0E-1 | 7.2E-1 | 1.6E-1 | 1.2E-2 | 1.2E-2 | 8.7E0 | 3.8E-1 | 169 | 7 | 169 | 7 | 0.50 |
| bS | pg/mL | 9.4E-1 | 9.4E-1 | 8.9E0 | 2.4E1 | 4.1E1 | 3.2E1 | 9.4E-1 | 9.4E-1 | 3.9E2 | 7.2E1 | 169 | 7 | 169 | 7 | 0.66 |
| bU | ng/mL | 7.0E-2 | 1.6E-1 | 1.9E-1 | 1.6E-1 | 5.4E-1 | 1.6E-1 | 1.3E-2 | 1.3E-2 | 6.6E0 | 4.1E-1 | 169 | 7 | 169 | 7 | 0.55 |
| bV | pg/mL | 4.9E2 | 1.0E3 | 6.1E2 | 1.2E3 | 8.9E2 | 7.0E2 | 1.6E2 | 5.3E2 | 1.2E4 | 2.2E3 | 169 | 7 | 169 | 7 | 0.83 |
| bW | pg/mL | 3.1E2 | 6.1E2 | 4.9E2 | 1.5E3 | 5.4E2 | 1.7E3 | 8.4E1 | 3.1E2 | 4.8E3 | 3.9E3 | 169 | 7 | 169 | 7 | 0.78 |
| bX | ng/mL | 2.5E-5 | 3.1E-3 | 2.6E-3 | 3.1E-3 | 3.2E-3 | 3.1E-3 | 2.5E-5 | 2.5E-5 | 1.3E-2 | 7.2E-3 | 169 | 7 | 169 | 7 | 0.56 |
| bZ | pg/mL | 2.7E2 | 1.8E3 | 2.0E3 | 2.6E3 | 7.2E3 | 2.7E3 | 1.5E-1 | 1.8E2 | 5.8E4 | 7.4E3 | 169 | 7 | 169 | 7 | 0.73 |
| cA | pg/mL | 6.0E-1 | 6.0E-1 | 4.1E0 | 3.5E0 | 2.9E1 | 7.7E0 | 6.0E-1 | 6.0E-1 | 3.7E2 | 2.1E1 | 169 | 7 | 169 | 7 | 0.52 |
| cB | ng/mL | 4.9E-2 | 4.6E-2 | 7.4E-2 | 8.6E-2 | 8.7E-2 | 1.1E-1 | 1.7E-3 | 1.7E-3 | 4.3E-1 | 2.6E-1 | 169 | 7 | 169 | 7 | 0.49 |
| cC | pg/mL | 4.1E1 | 3.5E1 | 4.6E1 | 2.8E1 | 5.0E1 | 2.6E1 | 1.0E0 | 1.0E0 | 4.5E2 | 5.9E1 | 169 | 7 | 169 | 7 | 0.36 |
| cD | pg/mL | 4.9E0 | 2.9E0 | 1.3E1 | 4.7E0 | 4.6E1 | 4.3E0 | 3.3E-1 | 3.3E-1 | 4.8E2 | 9.6E0 | 169 | 7 | 169 | 7 | 0.45 |
| cE | pg/mL | 5.9E1 | 2.6E2 | 2.7E2 | 2.3E2 | 6.0E2 | 1.4E2 | 1.2E-1 | 3.5E1 | 3.8E3 | 4.5E2 | 169 | 7 | 169 | 7 | 0.73 |
| cF | pg/mL | 4.5E0 | 5.3E-1 | 1.6E1 | 5.8E0 | 3.0E1 | 1.4E1 | 5.3E-1 | 5.3E-1 | 2.7E2 | 3.8E1 | 169 | 7 | 169 | 7 | 0.34 |
| cG | pg/mL | 5.4E1 | 3.3E2 | 1.7E2 | 2.7E2 | 8.1E2 | 1.8E2 | 7.8E0 | 4.0E1 | 1.0E4 | 4.9E2 | 169 | 7 | 169 | 7 | 0.80 |
| cH | uIU/mL | 3.2E0 | 2.5E0 | 7.6E0 | 7.8E0 | 1.7E1 | 1.2E1 | 8.6E-3 | 8.6E-3 | 1.6E2 | 3.4E1 | 169 | 7 | 169 | 7 | 0.45 |
| cI | ng/mL | 6.1E0 | 3.0E0 | 1.4E1 | 9.7E0 | 2.1E1 | 1.4E1 | 3.2E-2 | 1.1E0 | 1.2E2 | 4.1E1 | 169 | 7 | 169 | 7 | 0.43 |
| cJ | ug/mL | 6.6E1 | 3.6E1 | 1.0E2 | 5.0E1 | 1.0E2 | 5.8E1 | 6.9E0 | 5.6E0 | 6.4E2 | 1.7E2 | 169 | 7 | 169 | 7 | 0.31 |
| cK | ng/mL | 3.8E-3 | 3.8E-3 | 2.2E-2 | 4.9E-2 | 1.2E-1 | 7.5E-2 | 3.8E-3 | 3.8E-3 | 1.5E0 | 2.1E-1 | 169 | 7 | 169 | 7 | 0.66 |
| cL | pg/mL | 2.1E2 | 2.3E2 | 5.4E2 | 4.0E2 | 2.0E3 | 4.4E2 | 3.1E1 | 6.7E1 | 2.4E4 | 1.3E3 | 169 | 7 | 169 | 7 | 0.57 |
| cM | pg/mL | 2.7E2 | 2.1E2 | 2.9E2 | 2.1E2 | 1.7E2 | 8.3E1 | 2.5E1 | 5.7E1 | 1.1E3 | 3.1E2 | 169 | 7 | 169 | 7 | 0.34 |
| cN | pg/mL | 1.2E2 | 1.3E2 | 1.3E2 | 1.4E2 | 8.6E1 | 4.3E1 | 3.8E1 | 8.6E1 | 1.1E3 | 2.0E2 | 169 | 7 | 169 | 7 | 0.59 |
| cO | pg/mL | 2.1E2 | 4.4E2 | 4.0E2 | 5.4E2 | 1.5E3 | 4.9E2 | 5.4E1 | 9.6E1 | 1.9E4 | 1.5E3 | 169 | 7 | 169 | 7 | 0.66 |
| cP | ng/mL | 2.4E3 | 3.1E3 | 2.5E3 | 3.0E3 | 9.3E2 | 1.4E3 | 6.2E2 | 1.4E3 | 5.6E3 | 4.7E3 | 169 | 7 | 169 | 7 | 0.60 |
| cQ | ng/mL | 4.9E-2 | 1.6E-1 | 1.2E-1 | 2.8E-1 | 2.0E-1 | 3.0E-1 | 2.0E-3 | 2.0E-3 | 1.3E0 | 8.7E-1 | 169 | 7 | 169 | 7 | 0.72 |
| cR | ng/mL | 3.2E2 | 5.7E2 | 5.9E2 | 8.4E2 | 8.3E2 | 6.9E2 | 2.0E1 | 3.2E2 | 7.7E3 | 2.2E3 | 169 | 7 | 169 | 7 | 0.70 |
| cS | ng/mL | 2.8E2 | 6.6E2 | 4.2E2 | 1.8E3 | 4.7E2 | 2.5E3 | 4.1E1 | 1.4E2 | 3.3E3 | 7.1E3 | 169 | 7 | 169 | 7 | 0.77 |
| cT | ng/mL | 4.9E1 | 1.5E2 | 1.5E2 | 5.0E2 | 2.8E2 | 6.5E2 | 3.6E0 | 1.9E1 | 2.1E3 | 1.5E3 | 169 | 7 | 169 | 7 | 0.74 |
| cU | ng/mL | 5.8E1 | 1.3E2 | 9.6E1 | 1.6E2 | 1.5E2 | 9.0E1 | 6.2E0 | 8.0E1 | 1.6E3 | 3.3E2 | 169 | 7 | 169 | 7 | 0.81 |
| cV | ng/mL | 1.9E-1 | 3.5E-1 | 7.4E-1 | 3.7E-1 | 3.8E0 | 3.0E-1 | 2.5E-2 | 7.6E-2 | 4.7E1 | 9.3E-1 | 169 | 7 | 169 | 7 | 0.59 |
| cW | mIU/mL | 4.8E-2 | 9.4E-2 | 8.7E-2 | 1.3E-1 | 3.4E-1 | 1.3E-1 | 4.8E-3 | 3.3E-2 | 4.5E0 | 3.9E-1 | 169 | 7 | 169 | 7 | 0.71 |
| cX | ng/mL | 1.2E-1 | 4.4E-2 | 1.9E0 | 4.6E-1 | 5.7E0 | 9.1E-1 | 2.3E-4 | 1.1E-2 | 2.8E1 | 2.5E0 | 169 | 7 | 169 | 7 | 0.47 |
| cY | ng/mL | 7.4E0 | 8.9E0 | 1.1E1 | 1.2E1 | 1.1E1 | 1.3E1 | 1.7E-1 | 6.0E-1 | 6.1E1 | 3.6E1 | 169 | 7 | 169 | 7 | 0.47 |
| cZ | ug/mL | 1.3E1 | 1.1E1 | 1.5E1 | 1.4E1 | 6.7E0 | 8.3E0 | 2.3E0 | 3.3E0 | 4.6E1 | 3.0E1 | 169 | 7 | 169 | 7 | 0.44 |
| dA | pg/mL | 3.1E2 | 6.3E2 | 3.8E2 | 5.6E2 | 4.6E2 | 2.7E2 | 1.0E2 | 1.7E2 | 5.8E3 | 8.8E2 | 169 | 7 | 169 | 7 | 0.72 |
| dB | ug/mL | 1.8E1 | 1.9E1 | 1.8E1 | 1.9E1 | 2.0E1 | 8.5E0 | 2.1E0 | 4.5E0 | 2.5E2 | 2.8E1 | 169 | 7 | 169 | 7 | 0.57 |
| dC | nmol/L | 3.4E1 | 2.7E1 | 3.8E1 | 3.0E1 | 1.7E1 | 5.7E0 | 7.8E0 | 2.5E1 | 1.4E2 | 4.0E1 | 169 | 7 | 169 | 7 | 0.35 |
| dD | ug/mL | 3.4E1 | 2.8E1 | 3.5E1 | 2.8E1 | 1.1E1 | 6.8E0 | 1.4E1 | 2.1E1 | 7.4E1 | 4.3E1 | 169 | 7 | 169 | 7 | 0.27 |
| dE | ng/mL | 4.1E-1 | 1.0E0 | 5.5E-1 | 1.2E0 | 5.8E-1 | 9.5E-1 | 8.4E-3 | 8.4E-3 | 2.9E0 | 2.4E0 | 169 | 7 | 169 | 7 | 0.71 |
| dF | ng/mL | 2.5E2 | 7.8E2 | 3.3E2 | 6.7E2 | 2.4E2 | 3.6E2 | 7.5E1 | 2.8E2 | 1.3E3 | 1.2E3 | 169 | 7 | 169 | 7 | 0.81 |
| dG | ng/mL | 1.2E1 | 2.6E1 | 1.6E1 | 3.7E1 | 1.8E1 | 2.9E1 | 3.0E0 | 6.7E0 | 1.8E2 | 8.7E1 | 169 | 7 | 169 | 7 | 0.79 |
| dH | pg/mL | 8.0E0 | 1.4E1 | 2.1E1 | 2.2E1 | 6.3E1 | 2.5E1 | 4.0E-2 | 8.3E-1 | 6.7E2 | 7.6E1 | 169 | 7 | 169 | 7 | 0.69 |
| dI | pg/mL | 4.6E-1 | 2.9E0 | 3.7E0 | 5.5E0 | 2.6E1 | 6.7E0 | 4.6E-1 | 4.6E-1 | 3.3E2 | 1.9E1 | 169 | 7 | 169 | 7 | 0.70 |
| dJ | ng/mL | 2.0E0 | 2.2E0 | 2.1E0 | 2.1E0 | 1.1E0 | 1.2E0 | 3.2E-2 | 4.9E-1 | 5.6E0 | 4.0E0 | 169 | 7 | 169 | 7 | 0.50 |
| dK | uIU/mL | 1.3E0 | 3.2E-1 | 2.2E0 | 1.2E0 | 3.6E0 | 2.2E0 | 2.8E-4 | 1.4E-2 | 3.9E1 | 6.1E0 | 169 | 7 | 169 | 7 | 0.25 |
| dL | ng/mL | 8.7E2 | 1.3E3 | 1.0E3 | 1.8E3 | 5.5E2 | 1.4E3 | 2.8E2 | 6.3E2 | 3.8E3 | 4.8E3 | 169 | 7 | 169 | 7 | 0.69 |
| dM | pg/mL | 9.7E2 | 4.0E3 | 1.3E3 | 3.4E3 | 1.4E3 | 2.1E3 | 3.7E2 | 7.1E2 | 1.5E4 | 5.8E3 | 169 | 7 | 169 | 7 | 0.77 |
| dN | ug/mL | 9.8E1 | 1.6E2 | 1.0E2 | 1.8E2 | 4.0E1 | 6.7E1 | 2.4E1 | 1.4E2 | 2.8E2 | 3.3E2 | 169 | 7 | 169 | 7 | 0.91 |
| fR | ng/ml | 1.5E5 | 4.4E5 | 2.1E5 | 4.1E5 | 1.6E5 | 3.1E5 | 3.6E4 | 1.9E2 | 6.9E5 | 8.7E5 | 108 | 7 | 108 | 7 | 0.70 |

Figure 43.

Descriptive statistics for the assays used to form the panels presented in this figure.

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Po | pg/ml | 3.4E-1 | 8.2E1 | 8.9E0 | 9.3E1 | 2.6E1 | 8.6E1 | 8.0E-3 | 2.6E-2 | 2.7E2 | 2.2E2 | 260 | 8 | 260 | 8 | 0.86 |
| Et | ng/ml | 1.5E3 | 4.0E3 | 1.8E3 | 3.9E3 | 1.2E3 | 1.0E3 | 7.9E1 | 1.8E3 | 4.7E3 | 5.0E3 | 259 | 8 | 259 | 8 | 0.91 |
| Fp | ng/ml | 1.4E1 | 5.0E1 | 2.5E1 | 5.0E1 | 2.8E1 | 3.6E1 | 6.0E-3 | 1.1E1 | 1.3E2 | 1.3E2 | 263 | 8 | 263 | 8 | 0.77 |
| Fr | ng/ml | 3.9E4 | 6.8E5 | 1.3E5 | 5.8E5 | 1.9E5 | 2.7E5 | 1.9E2 | 2.2E4 | 8.4E5 | 8.4E5 | 266 | 9 | 266 | 9 | 0.89 |
| Nm | pg/ml | 1.4E4 | 5.3E4 | 3.8E4 | 1.6E5 | 9.1E4 | 2.7E5 | 1.0E-9 | 1.0E4 | 9.6E5 | 8.2E5 | 263 | 8 | 263 | 8 | 0.80 |
| Nn | pg/ml | 1.9E2 | 8.3E3 | 3.0E3 | 3.0E4 | 2.0E4 | 4.2E4 | 1.0E-9 | 2.8E2 | 3.1E5 | 1.1E5 | 263 | 8 | 263 | 8 | 0.91 |
| No | pg/ml | 1.7E1 | 4.6E2 | 3.4E1 | 4.3E2 | 5.8E1 | 3.5E2 | 1.0E-9 | 4.4E0 | 5.6E2 | 9.1E2 | 263 | 8 | 263 | 8 | 0.87 |
| Nq | pg/ml | 2.0E0 | 7.7E1 | 2.6E1 | 1.5E2 | 8.5E1 | 2.1E2 | 1.0E-9 | 1.0E-9 | 6.7E2 | 6.3E2 | 263 | 8 | 263 | 8 | 0.84 |
| Nr | pg/ml | 2.0E0 | 5.0E1 | 2.3E1 | 3.0E2 | 8.0E1 | 4.9E2 | 1.0E-9 | 1.0E-9 | 9.8E2 | 1.4E3 | 263 | 8 | 263 | 8 | 0.78 |
| Ns | pg/ml | 1.0E-9 | 1.0E-9 | 9.6E0 | 1.0E-9 | 7.3E1 | 0.0E0 | 1.0E-9 | 1.0E-9 | 1.1E3 | 1.0E-9 | 263 | 8 | 263 | 8 | 0.47 |
| Nt | pg/ml | 1.2E2 | 3.0E2 | 1.4E2 | 5.7E2 | 1.0E2 | 6.1E2 | 1.5E1 | 7.5E1 | 8.8E2 | 1.7E3 | 263 | 8 | 263 | 8 | 0.76 |
| Nu | pg/ml | 2.4E1 | 9.9E1 | 6.0E1 | 1.3E2 | 8.8E1 | 1.4E2 | 1.0E-9 | 1.0E-9 | 6.3E2 | 3.7E2 | 263 | 8 | 263 | 8 | 0.67 |
| Lu | pg/ml | 1.0E4 | 4.9E3 | 1.7E4 | 6.6E3 | 4.5E4 | 5.4E3 | 9.4E2 | 5.2E2 | 5.6E5 | 1.7E4 | 263 | 8 | 263 | 8 | 0.33 |
| Lv | pg/ml | 1.0E-9 | 5.9E1 | 1.7E1 | 5.6E1 | 3.4E1 | 5.7E1 | 1.0E-9 | 1.0E-9 | 2.6E2 | 1.6E2 | 263 | 8 | 263 | 8 | 0.70 |
| Lw | pg/ml | 1.0E-9 | 1.0E-9 | 1.1E0 | 1.0E1 | 1.2E1 | 1.5E1 | 1.0E-9 | 1.0E-9 | 1.8E2 | 4.0E1 | 263 | 8 | 263 | 8 | 0.68 |
| Lx | pg/ml | 1.0E-9 | 1.7E3 | 2.0E2 | 4.1E3 | 6.2E2 | 7.5E3 | 1.0E-9 | 1.0E-9 | 6.2E3 | 2.2E4 | 263 | 8 | 263 | 8 | 0.88 |
| Ly | pg/ml | 1.0E-9 | 1.0E-9 | 9.5E0 | 9.4E0 | 1.8E1 | 2.1E1 | 1.0E-9 | 1.0E-9 | 1.1E2 | 6.0E1 | 263 | 8 | 263 | 8 | 0.51 |
| Lz | pg/ml | 1.0E-9 | 1.0E-9 | 3.2E0 | 1.6E1 | 2.9E1 | 2.5E1 | 1.0E-9 | 1.0E-9 | 4.3E2 | 6.2E1 | 263 | 8 | 263 | 8 | 0.67 |
| Ma | pg/ml | 5.2E2 | 4.9E3 | 2.7E3 | 4.7E3 | 6.9E3 | 2.1E3 | 1.0E-9 | 2.0E3 | 6.5E4 | 7.5E3 | 263 | 8 | 263 | 8 | 0.86 |
| Mb | pg/ml | 2.6E1 | 2.1E1 | 3.3E1 | 2.3E1 | 1.9E1 | 1.3E1 | 9.2E0 | 4.1E0 | 2.1E2 | 4.7E1 | 263 | 8 | 263 | 8 | 0.31 |
| Mc | pg/ml | 1.0E-9 | 1.0E-9 | 7.2E-2 | 1.0E-9 | 8.5E-1 | 0.0E0 | 1.0E-9 | 1.0E-9 | 1.3E1 | 1.0E-9 | 263 | 8 | 263 | 8 | 0.49 |
| Md | pg/ml | 1.0E-9 | 1.8E-1 | 5.0E-1 | 4.6E0 | 4.3E0 | 1.0E1 | 1.0E-9 | 1.0E-9 | 6.5E1 | 2.9E1 | 263 | 8 | 263 | 8 | 0.71 |
| Me | pg/ml | 3.1E1 | 3.1E1 | 3.1E1 | 4.3E1 | 2.6E1 | 5.9E1 | 1.0E-9 | 1.0E-9 | 3.2E2 | 1.8E2 | 263 | 8 | 263 | 8 | 0.48 |
| Mf | pg/ml | 1.0E-9 | 1.0E-9 | 7.8E-1 | 6.0E-1 | 4.4E0 | 1.6E0 | 1.0E-9 | 1.0E-9 | 5.6E1 | 4.5E0 | 263 | 8 | 263 | 8 | 0.53 |
| Mg | pg/ml | 1.6E0 | 8.0E0 | 7.8E0 | 1.2E1 | 1.6E1 | 1.4E1 | 1.0E-9 | 1.0E-9 | 1.5E2 | 3.7E1 | 263 | 8 | 263 | 8 | 0.55 |
| Mh | pg/ml | 1.0E-9 | 4.2E-1 | 1.0E0 | 4.3E0 | 7.4E0 | 7.2E0 | 1.0E-9 | 1.0E-9 | 1.1E2 | 1.8E1 | 263 | 8 | 263 | 8 | 0.74 |
| Mi | pg/ml | 1.0E-9 | 1.1E1 | 3.1E0 | 5.9E1 | 3.3E1 | 1.1E2 | 1.0E-9 | 1.0E-9 | 5.2E2 | 3.2E2 | 263 | 8 | 263 | 8 | 0.73 |
| Mj | pg/ml | 1.0E-9 | 1.9E1 | 6.2E0 | 5.7E1 | 3.1E1 | 8.3E1 | 1.0E-9 | 1.0E-9 | 4.6E2 | 2.1E2 | 263 | 8 | 263 | 8 | 0.69 |
| Mk | pg/ml | 2.1E0 | 5.5E0 | 1.8E1 | 8.3E1 | 1.0E2 | 1.8E2 | 1.0E-9 | 1.0E-9 | 1.3E3 | 5.0E2 | 263 | 8 | 263 | 8 | 0.63 |
| Ml | pg/ml | 1.0E-9 | 1.0E-9 | 1.2E1 | 6.6E1 | 1.3E2 | 1.8E2 | 1.0E-9 | 1.0E-9 | 2.1E3 | 5.1E2 | 263 | 8 | 263 | 8 | 0.60 |
| Mm | pg/ml | 6.3E2 | 3.0E3 | 1.1E3 | 3.1E3 | 1.4E3 | 2.1E3 | 1.0E-9 | 3.6E2 | 1.0E4 | 6.5E3 | 263 | 8 | 263 | 8 | 0.82 |
| Mn | pg/ml | 6.5E0 | 1.8E1 | 1.2E1 | 2.0E1 | 2.8E1 | 1.4E1 | 1.0E-9 | 6.5E0 | 3.5E2 | 5.1E1 | 263 | 8 | 263 | 8 | 0.79 |
| Mp | pg/ml | 1.0E-9 | 6.9E1 | 2.3E1 | 1.2E2 | 1.5E2 | 1.6E2 | 1.0E-9 | 6.8E0 | 2.4E3 | 5.0E2 | 263 | 8 | 263 | 8 | 0.91 |
| Mq | pg/ml | 1.0E-9 | 1.0E-9 | 2.7E0 | 1.9E1 | 1.4E1 | 3.8E1 | 1.0E-9 | 1.0E-9 | 1.8E2 | 1.0E2 | 263 | 8 | 263 | 8 | 0.59 |
| Mr | pg/ml | 1.0E-9 | 3.2E2 | 2.8E1 | 9.5E2 | 1.4E2 | 1.3E3 | 1.0E-9 | 1.0E-9 | 1.5E3 | 3.4E3 | 263 | 8 | 263 | 8 | 0.77 |
| Ms | pg/ml | 3.3E2 | 2.2E2 | 4.5E2 | 3.5E2 | 5.1E2 | 3.8E2 | 1.0E-9 | 1.0E-9 | 3.3E3 | 9.8E2 | 263 | 8 | 263 | 8 | 0.44 |
| Mt | pg/ml | 4.8E-1 | 8.7E1 | 1.1E1 | 4.7E2 | 5.2E1 | 1.1E3 | 1.0E-9 | 2.8E-1 | 7.1E2 | 3.2E3 | 263 | 8 | 263 | 8 | 0.90 |
| Mu | pg/ml | 1.0E-9 | 4.5E0 | 2.0E0 | 9.7E0 | 1.6E1 | 1.2E1 | 1.0E-9 | 1.0E-9 | 2.3E2 | 3.5E1 | 263 | 8 | 263 | 8 | 0.90 |
| Mv | pg/ml | 1.0E-9 | 2.0E2 | 8.0E1 | 3.4E2 | 3.8E2 | 3.9E2 | 1.0E-9 | 1.0E-9 | 4.5E3 | 9.2E2 | 263 | 8 | 263 | 8 | 0.73 |
| Mw | pg/ml | 3.7E1 | 1.4E3 | 3.1E2 | 2.2E3 | 1.5E3 | 2.2E3 | 1.0E-9 | 1.0E2 | 1.8E4 | 5.3E3 | 263 | 8 | 263 | 8 | 0.91 |
| Mx | pg/ml | 1.0E-9 | 7.3E-1 | 4.2E-1 | 3.7E0 | 2.1E0 | 6.7E0 | 1.0E-9 | 1.0E-9 | 3.2E1 | 2.0E1 | 263 | 8 | 263 | 8 | 0.74 |
| My | pg/ml | 1.0E-9 | 2.5E2 | 4.0E2 | 4.7E2 | 2.8E3 | 6.8E2 | 1.0E-9 | 1.0E-9 | 3.9E4 | 1.9E3 | 263 | 8 | 263 | 8 | 0.69 |
| Mz | pg/ml | 1.2E1 | 1.7E2 | 2.7E1 | 4.8E2 | 5.1E1 | 7.2E2 | 1.0E-9 | 1.0E-9 | 5.5E2 | 1.9E3 | 263 | 8 | 263 | 8 | 0.82 |
| Na | pg/ml | 1.0E-9 | 1.6E-1 | 5.2E-1 | 6.8E0 | 1.7E0 | 1.4E1 | 1.0E-9 | 1.0E-9 | 9.6E0 | 4.2E1 | 263 | 8 | 263 | 8 | 0.67 |
| Nb | pg/ml | 2.2E0 | 1.6E1 | 3.9E0 | 4.8E1 | 1.1E1 | 7.4E1 | 1.0E-9 | 1.0E-9 | 1.5E2 | 2.1E2 | 263 | 8 | 263 | 8 | 0.74 |
| Nc | pg/ml | 3.4E2 | 1.0E-9 | 5.2E2 | 1.1E2 | 7.7E2 | 2.8E2 | 1.0E-9 | 1.0E-9 | 9.2E3 | 7.9E2 | 263 | 8 | 263 | 8 | 0.24 |
| Nd | pg/ml | 2.5E1 | 4.1E1 | 2.8E1 | 3.0E2 | 7.7E1 | 7.4E2 | 1.0E-9 | 4.8E0 | 1.2E3 | 2.1E3 | 263 | 8 | 263 | 8 | 0.76 |
| Ne | pg/ml | 4.2E2 | 2.0E2 | 5.3E2 | 2.3E2 | 6.0E2 | 2.0E2 | 1.0E-9 | 1.0E-9 | 7.0E3 | 4.7E2 | 263 | 8 | 263 | 8 | 0.30 |
| Nf | pg/ml | 1.0E-9 | 1.0E-9 | 2.1E0 | 3.6E1 | 8.8E0 | 5.5E1 | 1.0E-9 | 1.0E-9 | 8.2E1 | 1.3E2 | 263 | 8 | 263 | 8 | 0.62 |

| Code | Units | Median | | Average | | Standard Deviation | | Minimum | | Maximum | | Number of Samples | | Number of Patients | | AUC |
|---|---|---|---|---|---|---|---|---|---|---|---|---|---|---|---|---|
| | | NonDis | Dis | NonDis | Dis | NonDis | Dis | NonDis | Dis | NonDis | Dis | NonDis | Dis | NonDis | Dis | |
| Ng | pg/ml | 1.7E1 | 2.7E1 | 1.0E2 | 9.6E1 | 2.0E2 | 1.5E2 | 1.0E-9 | 1.0E-9 | 1.6E3 | 4.2E2 | 263 | 8 | 263 | 8 | 0.50 |
| Nh | pg/ml | 6.0E1 | 3.1E1 | 8.0E1 | 3.6E1 | 7.8E1 | 2.5E1 | 1.0E-9 | 4.1E0 | 5.6E2 | 7.5E1 | 263 | 8 | 263 | 8 | 0.29 |
| Ni | pg/ml | 1.0E-9 | 1.0E-9 | 8.5E1 | 1.9E2 | 1.3E2 | 3.8E2 | 1.0E-9 | 1.0E-9 | 7.1E2 | 1.1E3 | 263 | 8 | 263 | 8 | 0.50 |
| Nj | pg/ml | 6.2E0 | 3.4E0 | 9.9E0 | 7.8E0 | 1.1E1 | 9.0E0 | 1.0E-9 | 1.0E-9 | 6.8E1 | 2.2E1 | 263 | 8 | 263 | 8 | 0.43 |
| Nk | pg/ml | 1.7E1 | 2.6E0 | 3.2E1 | 1.5E1 | 4.0E1 | 2.4E1 | 1.0E-9 | 1.0E-9 | 2.0E2 | 6.6E1 | 263 | 8 | 263 | 8 | 0.38 |
| Nl | pg/ml | 4.2E1 | 1.0E1 | 5.6E1 | 2.0E1 | 7.9E1 | 2.8E1 | 1.0E-9 | 1.0E-9 | 1.1E3 | 8.2E1 | 263 | 8 | 263 | 8 | 0.27 |
| Hq | pg/ml | 1.2E0 | 1.2E1 | 2.0E2 | 3.8E2 | 2.1E3 | 9.7E2 | 1.0E-9 | 6.2E-2 | 2.8E4 | 2.8E3 | 261 | 8 | 261 | 8 | 0.73 |
| Hr | pg/ml | 8.5E1 | 4.9E2 | 5.1E2 | 1.8E3 | 1.0E3 | 3.1E3 | 1.0E-9 | 1.0E-9 | 8.4E3 | 8.9E3 | 261 | 8 | 261 | 8 | 0.61 |
| Hu | pg/ml | 1.4E1 | 6.5E2 | 5.9E3 | 1.3E3 | 4.5E4 | 2.2E3 | 1.0E-9 | 1.0E-9 | 6.3E5 | 6.5E3 | 261 | 8 | 261 | 8 | 0.70 |
| Hv | pg/ml | 1.4E0 | 8.1E0 | 3.1E0 | 1.2E2 | 1.1E1 | 3.1E2 | 1.0E-9 | 1.0E-9 | 1.6E2 | 8.9E2 | 261 | 8 | 261 | 8 | 0.74 |
| Hw | pg/ml | 5.5E0 | 1.1E1 | 1.6E1 | 1.3E3 | 5.0E1 | 3.3E3 | 1.0E-9 | 5.1E-1 | 6.4E2 | 9.4E3 | 261 | 8 | 261 | 8 | 0.59 |
| Hx | pg/ml | 8.9E0 | 4.2E1 | 6.1E1 | 3.1E2 | 5.7E2 | 4.8E2 | 1.0E-9 | 3.9E0 | 9.3E3 | 1.3E3 | 261 | 8 | 261 | 8 | 0.75 |
| Ih | ng/ml | 7.3E1 | 9.9E2 | 2.5E2 | 9.7E2 | 4.3E2 | 9.1E2 | 1.0E-9 | 5.7E0 | 3.6E3 | 2.8E3 | 262 | 8 | 262 | 8 | 0.76 |
| Ii | ng/ml | 9.2E1 | 4.7E2 | 2.0E2 | 1.4E3 | 4.4E2 | 1.7E3 | 7.3E-1 | 2.3E0 | 5.2E3 | 4.5E3 | 262 | 8 | 262 | 8 | 0.79 |
| Ij | ng/ml | 8.7E1 | 1.5E2 | 2.0E2 | 3.2E3 | 6.1E2 | 8.5E3 | 2.8E0 | 4.6E1 | 6.4E3 | 2.4E4 | 259 | 8 | 259 | 8 | 0.73 |
| Ik | ng/ml | 1.2E1 | 1.6E1 | 1.6E3 | 7.5E1 | 1.3E4 | 1.6E2 | 5.9E-1 | 5.5E0 | 1.2E5 | 4.6E2 | 259 | 8 | 259 | 8 | 0.53 |
| Il | ng/ml | 3.8E2 | 7.0E2 | 1.3E3 | 3.6E3 | 2.8E3 | 5.3E3 | 1.0E-9 | 7.1E1 | 1.2E4 | 1.2E4 | 257 | 8 | 257 | 8 | 0.64 |
| Im | ng/ml | 2.3E2 | 7.0E2 | 4.7E2 | 2.0E3 | 7.5E2 | 2.5E3 | 1.4E1 | 1.5E2 | 6.0E3 | 6.2E3 | 259 | 8 | 259 | 8 | 0.82 |
| In | ng/ml | 3.3E0 | 2.8E0 | 1.9E1 | 6.0E2 | 8.2E1 | 1.6E3 | 1.0E-9 | 1.0E-9 | 1.1E3 | 4.5E3 | 262 | 8 | 262 | 8 | 0.52 |
| Io | ng/ml | 1.1E4 | 1.2E4 | 2.2E4 | 1.3E4 | 5.1E4 | 1.1E4 | 1.0E-9 | 1.5E3 | 7.1E5 | 3.3E4 | 262 | 8 | 262 | 8 | 0.47 |
| Ip | ng/ml | 1.4E1 | 4.2E1 | 2.2E1 | 4.2E1 | 2.7E1 | 1.5E1 | 1.0E-9 | 2.1E1 | 2.3E2 | 5.8E1 | 262 | 8 | 262 | 8 | 0.78 |
| Iq | ug/ml | 1.2E-1 | 3.5E0 | 8.2E-1 | 3.4E1 | 2.8E0 | 7.6E1 | 1.0E-9 | 7.4E-2 | 3.4E1 | 2.2E2 | 262 | 8 | 262 | 8 | 0.77 |
| Ir | ug/ml | 4.1E-1 | 1.4E1 | 2.7E0 | 8.1E1 | 1.2E1 | 1.3E2 | 1.0E-9 | 3.4E-1 | 1.6E2 | 3.7E2 | 261 | 8 | 261 | 8 | 0.82 |
| Is | ng/ml | 2.2E0 | 5.1E1 | 8.3E0 | 8.2E1 | 1.8E1 | 8.5E1 | 1.0E-9 | 8.9E0 | 1.5E2 | 2.6E2 | 262 | 8 | 262 | 8 | 0.93 |
| It | ng/ml | 2.1E0 | 1.4E1 | 1.5E1 | 9.9E1 | 6.8E1 | 2.4E2 | 1.0E-9 | 1.0E-9 | 8.3E2 | 6.8E2 | 262 | 8 | 262 | 8 | 0.70 |
| Iu | ng/ml | 1.8E2 | 6.6E2 | 1.2E3 | 6.4E3 | 3.7E3 | 1.1E4 | 1.0E-9 | 8.5E0 | 2.9E4 | 2.4E4 | 262 | 8 | 262 | 8 | 0.64 |
| Iv | ng/ml | 1.4E1 | 3.7E2 | 5.3E1 | 1.1E3 | 2.5E2 | 1.6E3 | 1.0E-9 | 1.0E0 | 3.8E3 | 3.8E3 | 261 | 8 | 261 | 8 | 0.75 |
| Pz | ng/ml | 4.3E3 | 3.5E3 | 5.9E3 | 5.7E3 | 6.2E3 | 4.5E3 | 1.6E1 | 1.1E3 | 7.0E4 | 1.3E4 | 258 | 8 | 258 | 8 | 0.54 |
| Qa | ng/ml | 3.9E3 | 2.0E4 | 7.2E3 | 4.3E4 | 8.2E3 | 7.3E4 | 1.5E2 | 2.9E3 | 4.2E4 | 2.2E5 | 258 | 8 | 258 | 8 | 0.85 |
| Qb | ng/ml | 1.2E2 | 5.3E2 | 2.4E2 | 4.3E2 | 3.9E2 | 2.0E2 | 7.9E-1 | 1.3E2 | 4.1E3 | 6.0E2 | 258 | 8 | 258 | 8 | 0.80 |
| Qc | ng/ml | 2.5E2 | 5.4E2 | 4.6E2 | 7.8E2 | 5.8E2 | 9.5E2 | 1.0E-9 | 1.3E1 | 4.3E3 | 2.8E3 | 258 | 8 | 258 | 8 | 0.59 |
| Qd | ng/ml | 1.0E4 | 7.9E4 | 3.0E4 | 9.4E4 | 1.3E5 | 8.8E4 | 2.4E2 | 3.5E3 | 2.0E6 | 2.3E5 | 258 | 8 | 258 | 8 | 0.77 |
| Qe | ng/ml | 1.1E3 | 4.3E3 | 2.2E3 | 5.8E3 | 6.3E3 | 5.8E3 | 7.6E0 | 5.7E2 | 9.7E4 | 1.8E4 | 258 | 8 | 258 | 8 | 0.79 |
| Jg | ng/ml | 5.5E2 | 2.4E3 | 8.5E2 | 2.7E3 | 9.9E2 | 2.0E3 | 5.8E0 | 2.8E2 | 1.0E4 | 7.1E3 | 261 | 8 | 261 | 8 | 0.86 |
| Jh | ng/ml | 3.0E0 | 1.1E2 | 2.4E1 | 1.4E2 | 9.4E1 | 1.5E2 | 1.0E-9 | 4.3E0 | 1.2E3 | 4.9E2 | 261 | 8 | 261 | 8 | 0.90 |
| Ji | ng/ml | 6.3E1 | 3.8E2 | 9.2E1 | 4.8E2 | 1.0E2 | 3.5E2 | 1.1E0 | 1.2E2 | 6.7E2 | 1.3E3 | 261 | 8 | 261 | 8 | 0.95 |
| Jj | ng/ml | 5.5E2 | 1.0E2 | 2.3E3 | 2.2E2 | 2.1E4 | 3.2E2 | 2.0E1 | 1.2E1 | 3.4E5 | 1.0E3 | 261 | 8 | 261 | 8 | 0.18 |
| Jk | ng/ml | 3.0E0 | 7.8E1 | 2.2E1 | 9.8E1 | 5.1E1 | 9.0E1 | 1.0E-9 | 1.3E0 | 3.9E2 | 2.4E2 | 261 | 8 | 261 | 8 | 0.80 |
| Jl | ng/ml | 5.1E-1 | 2.0E1 | 2.2E0 | 1.3E3 | 5.1E0 | 3.5E3 | 1.2E-3 | 1.2E0 | 4.0E1 | 9.9E3 | 261 | 8 | 261 | 8 | 0.92 |
| Jm | ng/ml | 2.3E1 | 3.1E1 | 6.8E1 | 7.4E1 | 1.7E2 | 1.2E2 | 1.0E-9 | 2.1E0 | 2.1E3 | 3.6E2 | 261 | 8 | 261 | 8 | 0.57 |
| Jn | pg/ml | 4.0E-1 | 2.8E0 | 2.7E0 | 1.7E2 | 1.6E1 | 3.1E2 | 1.0E-9 | 4.2E-1 | 2.4E2 | 7.3E2 | 261 | 8 | 261 | 8 | 0.83 |
| Jo | pg/ml | 4.3E3 | 4.1E3 | 5.2E3 | 2.1E4 | 4.1E3 | 3.5E4 | 2.6E2 | 2.3E2 | 2.4E4 | 1.0E5 | 261 | 8 | 261 | 8 | 0.55 |
| Jp | pg/ml | 7.4E4 | 1.2E5 | 7.8E4 | 1.3E5 | 3.9E4 | 4.6E4 | 2.1E3 | 6.5E4 | 3.8E5 | 2.1E5 | 261 | 8 | 261 | 8 | 0.83 |
| Jq | pg/ml | 9.9E1 | 4.6E2 | 1.6E2 | 2.0E3 | 2.0E2 | 3.0E3 | 5.6E0 | 7.1E1 | 1.7E3 | 8.7E3 | 261 | 8 | 261 | 8 | 0.85 |
| Jr | pg/ml | 4.1E0 | 7.2E1 | 3.5E1 | 1.6E3 | 2.0E2 | 2.9E3 | 1.0E-9 | 6.7E0 | 2.4E3 | 7.4E3 | 261 | 8 | 261 | 8 | 0.88 |
| Js | pg/ml | 1.6E1 | 5.4E1 | 5.5E1 | 7.9E2 | 2.3E2 | 1.3E3 | 1.0E-9 | 6.1E0 | 3.0E3 | 3.0E3 | 261 | 8 | 261 | 8 | 0.78 |
| Jt | pg/ml | 2.6E3 | 4.8E3 | 3.3E3 | 1.3E4 | 3.3E3 | 1.8E4 | 1.5E2 | 1.0E3 | 4.1E4 | 5.2E4 | 261 | 8 | 261 | 8 | 0.71 |
| Lh | pg/ml | 1.5E4 | 8.2E4 | 2.4E4 | 1.9E5 | 3.1E4 | 2.0E5 | 1.0E-9 | 1.8E3 | 2.6E5 | 4.8E5 | 262 | 8 | 262 | 8 | 0.80 |
| Li | pg/ml | 4.0E3 | 9.3E4 | 1.8E4 | 1.0E5 | 6.9E4 | 1.1E5 | 1.3E1 | 2.5E3 | 9.2E5 | 3.1E5 | 262 | 8 | 262 | 8 | 0.79 |
| Lj | pg/ml | 3.1E3 | 1.8E4 | 1.9E4 | 6.9E4 | 5.1E4 | 1.3E5 | 1.0E-9 | 2.6E3 | 4.3E5 | 3.9E5 | 262 | 8 | 262 | 8 | 0.72 |
| Nv | pg/ml | 4.1E3 | 4.5E4 | 1.1E4 | 4.9E4 | 2.1E4 | 4.1E4 | 1.0E-9 | 2.6E3 | 1.6E5 | 1.1E5 | 263 | 8 | 263 | 8 | 0.82 |
| Nw | pg/ml | 1.0E4 | 3.1E4 | 1.4E4 | 7.9E4 | 1.7E4 | 8.6E4 | 1.9E2 | 1.5E4 | 2.1E5 | 2.2E5 | 263 | 8 | 263 | 8 | 0.92 |
| Nx | pg/ml | 2.2E2 | 1.2E3 | 4.5E2 | 1.4E3 | 6.4E2 | 1.2E3 | 1.0E-9 | 1.0E-9 | 3.5E3 | 4.1E3 | 263 | 8 | 263 | 8 | 0.79 |

| Code | Units | Median | | Average | | Standard Deviation | | Minimum | | Maximum | | Number of Samples | | Number of Patients | | AUC |
|------|-------|--------|-----|---------|-----|--------|------|---------|-------|---------|-------|--------|-----|--------|-----|------|
| | | NonDis | Dis | NonDis | Dis | NonDis | Dis | NonDis | Dis | NonDis | Dis | NonDis | Dis | NonDis | Dis | |
| Ny | pg/ml | 7.8E0 | 2.8E2 | 1.2E2 | 5.6E2 | 1.5E3 | 9.2E2 | 1.0E-9 | 9.8E0 | 2.5E4 | 2.8E3 | 263 | 8 | 263 | 8 | 0.85 |
| Oe | pg/ml | 3.1E1 | 4.7E0 | 2.6E2 | 2.3E2 | 4.0E2 | 4.3E2 | 1.0E-9 | 1.0E-9 | 2.3E3 | 1.1E3 | 261 | 8 | 261 | 8 | 0.46 |
| Of | pg/ml | 1.9E2 | 3.3E2 | 6.1E3 | 1.3E4 | 2.3E4 | 2.3E4 | 1.0E-9 | 1.1E1 | 1.9E5 | 6.6E4 | 263 | 8 | 263 | 8 | 0.59 |
| Og | pg/ml | 7.6E-2 | 9.2E-3 | 3.9E-1 | 6.9E-2 | 1.6E0 | 1.1E-1 | 1.0E-9 | 1.0E-9 | 1.9E1 | 3.2E-1 | 263 | 8 | 263 | 8 | 0.37 |
| Oh | pg/ml | 2.9E0 | 5.5E1 | 7.7E1 | 1.9E2 | 9.8E2 | 3.8E2 | 1.0E-9 | 8.3E0 | 1.6E4 | 1.1E3 | 263 | 8 | 263 | 8 | 0.93 |
| Oi | pg/ml | 2.6E0 | 1.0E-9 | 5.3E0 | 6.5E0 | 7.2E0 | 1.1E1 | 1.0E-9 | 1.0E-9 | 4.2E1 | 3.1E1 | 263 | 8 | 263 | 8 | 0.43 |
| Ok | pg/ml | 4.2E2 | 1.9E3 | 5.4E2 | 2.7E3 | 4.7E2 | 2.5E3 | 1.5E1 | 5.2E2 | 3.2E3 | 7.8E3 | 263 | 8 | 263 | 8 | 0.92 |
| Om | pg/ml | 4.3E2 | 4.7E3 | 8.7E2 | 9.5E3 | 2.3E3 | 1.7E4 | 1.0E-9 | 5.4E2 | 3.0E4 | 5.1E4 | 263 | 8 | 263 | 8 | 0.92 |
| On | pg/ml | 2.0E2 | 1.7E3 | 3.3E2 | 2.4E3 | 4.8E2 | 2.6E3 | 8.4E-1 | 3.3E2 | 4.5E3 | 8.5E3 | 263 | 8 | 263 | 8 | 0.94 |
| Oy | pg/ml | 4.9E-1 | 2.1E0 | 7.2E0 | 7.9E0 | 3.5E1 | 1.2E1 | 1.0E-9 | 1.0E-9 | 4.0E2 | 3.3E1 | 262 | 8 | 262 | 8 | 0.65 |
| Oz | pg/ml | 8.5E-3 | 1.0E-9 | 3.6E-1 | 3.6E0 | 1.8E0 | 9.9E0 | 1.0E-9 | 1.0E-9 | 2.9E1 | 2.8E1 | 262 | 8 | 262 | 8 | 0.44 |
| Pa | pg/ml | 4.3E-1 | 4.7E0 | 1.5E0 | 4.4E1 | 6.4E0 | 8.1E1 | 1.0E-9 | 1.7E-1 | 8.6E1 | 2.3E2 | 262 | 8 | 262 | 8 | 0.79 |
| Pb | pg/ml | 1.0E-9 | 1.0E-9 | 2.1E0 | 4.2E0 | 3.0E1 | 1.1E1 | 1.0E-9 | 1.0E-9 | 4.9E2 | 3.2E1 | 262 | 8 | 262 | 8 | 0.45 |
| Pc | pg/ml | 9.9E-2 | 1.0E-9 | 1.7E0 | 5.6E0 | 2.0E1 | 1.3E1 | 1.0E-9 | 1.0E-9 | 3.3E2 | 3.8E1 | 262 | 8 | 262 | 8 | 0.52 |
| Pd | pg/ml | 1.7E0 | 2.4E1 | 7.3E0 | 3.1E1 | 5.3E1 | 4.0E1 | 1.0E-9 | 7.3E-2 | 8.4E2 | 1.2E2 | 262 | 8 | 262 | 8 | 0.74 |
| Pe | pg/ml | 2.4E1 | 8.2E2 | 1.3E2 | 3.6E3 | 5.0E2 | 5.3E3 | 1.0E-9 | 2.6E1 | 4.7E3 | 1.5E4 | 262 | 8 | 262 | 8 | 0.83 |
| Pf | pg/ml | 1.9E0 | 4.3E1 | 1.5E1 | 9.5E1 | 9.7E1 | 1.4E2 | 1.0E-9 | 3.7E0 | 1.5E3 | 4.3E2 | 262 | 8 | 262 | 8 | 0.91 |
| Pg | pg/ml | 4.6E0 | 2.1E2 | 9.2E1 | 5.8E2 | 5.9E2 | 7.8E2 | 1.0E-9 | 4.6E-1 | 7.7E3 | 2.2E3 | 262 | 8 | 262 | 8 | 0.81 |
| aA | mg/dL | 9.0E-1 | 2.9E0 | 1.0E0 | 2.6E0 | 4.9E-1 | 1.4E0 | 3.0E-1 | 5.5E-1 | 4.2E0 | 4.7E0 | 394 | 11 | 394 | 11 | 0.84 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61308861 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- Harrison's Principles of Internal Medicine. McGraw Hill, 1741-1830
- Current Medical Diagnosis & Treatment. McGraw Hill, 2008, 785-815
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 ((4)), 177-197
- **THAKAR et al.** *J. Am. Soc. Nephrol.,* 2005, vol. 16, 162-68
- **MEHRAN et al.** *J. Am. Coll. Cardiol.,* 2004, vol. 44, 1393-99
- **WIJEYSUNDERA et al.** *JAMA,* 2007, vol. 297, 1801-9
- **GOLDSTEIN ; CHAWLA.** *Clin. J. Am. Soc. Nephrol.,* 2010, vol. 5, 943-49
- **CHAWLA et al.** *Kidney Intl.,* 2005, vol. 68, 2274-80
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210
- **HANLEY, J. A. ; MCNEIL, B.J.** The meaning and use of the area under a receiver operating characteristic (ROC) curve. *Radiology,* 1982, vol. 143, 29-36